(19)

Europäisches
Patentamt
European
Patent Office
Office européen
des brevets

(11)     **EP 2 123 668 A1**

(12)     **EUROPEAN PATENT APPLICATION**

(43) Date of publication:
**25.11.2009  Bulletin 2009/48**

(51) Int Cl.:
*C07K 11/00* [(2006.01)]     *C07K 14/00* [(2006.01)]
*A61K 39/112* [(2006.01)]     *A61P 31/04* [(2006.01)]

(21) Application number: **08290476.4**

(22) Date of filing: **22.05.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **INSTITUT PASTEUR
F-75015 Paris (FR)**

(72) Inventors:
 • **Mulard, Laurence**
  **94270 Le Kremlin Bicêtre (FR)**
 • **Phalipon, Armelle**
  **75015 Paris (FR)**
 • **Vulliez-Le Normand, Brigitte**
  **75020 Paris (FR)**

 • **Theillet, François-Xavier**
  **75004 Paris (FR)**
 • **Bentley, Graham**
  **91190 Gif sur Yvette (FR)**
 • **Saul, Frederick**
  **75015 Paris (FR)**
 • **Delepierre, Murielle**
  **92120 Montrouge (FR)**
 • **Felici, Franco**
  **00173 Roe (IT)**

(74) Representative: **Warcoin, Jacques et al
Cabinet Régimbeau
20, rue de Chazelles
75847 Paris Cedex 17 (FR)**

(54)     **Immunogenic polypeptides that mimic the surface polysaccharide O-antigen from serotype
2A shigella flexneri, method for obtaining the same, and their use in vaccine and diagnostic
compositions**

(57)    The present invention is directed to purified immunogenic polypeptides comprising an epitope unit recognized by a protective monoclonal antibody having a high affinity and a high specificity for the O-antigen from serotype 2a *Shigella flexneri.* The invention also concerns the use of said purified immunogenic polypeptides for the preparation of vaccine against serotype 2a *S. flexneri* infection and for the preparation of diagnostic compositions for the detection of serotype 2a *S. flexneri* infection. Finally, the present invention comprises high-resolution crystal structures of said natural O-antigen, or functional fragments thereof, possibly synthetic, and a the mimetic peptide, in complex with a protective monoclonal antibody, or functional fragments thereof, and to methods for selecting immunogenic polypeptides for the preparation of new candidate vaccinal compositions based on said crystals.

EP 2 123 668 A1

SEQUENCE LISTING

<110> Institut Pasteur

<120> Immunogenic polypeptides that mimic the surface polysaccharide O-antigen from serotype 2a *Shigella flexneri*, method for obtaining the same, and their use in vaccine and diagnostic compositions

<130> D26600

<160> 16

<170> PatentIn version 3.3

<210> 1
<211> 12
<212> PRT
<213> Artificial sequence

<220>
<223> Synthetic peptide

<400> 1

Asp Gln Ala Ala Gln His Pro Arg Asp Phe His Lys
1               5                   10

<210> 2
<211> 12
<212> PRT
<213> Artificial sequence

<220>
<223> Synthetic peptide

<400> 2

Tyr Leu Glu Asp Trp Ile Lys Tyr Asn Asn Gln Lys
1               5                   10

<210> 3
<211> 12
<212> PRT
<213> Artificial sequence

<220>
<223> Synthetic peptide

<400> 3

Met Lys Ser Trp Ala Ser Tyr Gln Ala His Leu Met
1               5                   10

<210> 4
<211> 12
<212> PRT

```
<213> Artificial sequence

<220>
<223> Synthetic peptide

<400> 4

Ile Asn Asp Trp Ile Ser Tyr Tyr Ser Ser Trp Lys
1               5                   10


<210> 5
<211> 12
<212> PRT
<213> Artificial sequence

<220>
<223> Synthetic peptide

<400> 5

Pro His Gly Glu Ile Phe His Met Met Arg Arg Pro
1               5                   10


<210> 6
<211> 12
<212> PRT
<213> Artificial sequence

<220>
<223> Synthetic peptide

<400> 6

Asn Arg Arg Ile Pro Cys Glu Thr Gly Cys Leu Leu
1               5                   10


<210> 7
<211> 12
<212> PRT
<213> Artificial sequence

<220>
<223> Synthetic peptide

<400> 7

Thr Phe Thr Arg Phe Cys Gly Lys Gly Cys Val Pro
1               5                   10


<210> 8
<211> 22
<212> PRT
<213> Artificial sequence

<220>
<223> Synthetic peptide
```

<400> 8

Ala Glu Gly Glu Phe Asp Gln Ala Ala Gln His Pro Arg Asp Phe His
1               5                   10                  15
Lys Asp Pro Ala Lys Cys
                20


<210> 9
<211> 22
<212> PRT
<213> Artificial sequence

<220>
<223> Synthetic peptide

<400> 9

Ala Glu Gly Glu Phe Tyr Leu Glu Asp Trp Ile Lys Tyr Asn Asn Gln
1               5                   10                  15
Lys Asp Pro Ala Lys Cys
                20


<210> 10
<211> 22
<212> PRT
<213> Artificial sequence

<220>
<223> Synthetic peptide

<400> 10

Ala Glu Gly Glu Phe Met Lys Ser Trp Ala Ser Tyr Gln Ala His Leu
1               5                   10                  15
Met Asp Pro Ala Lys Cys
                20


<210> 11
<211> 22
<212> PRT
<213> Artificial sequence

<220>
<223> Synthetic peptide

<400> 11

Ala Glu Gly Glu Phe Ile Asn Asp Trp Ile Ser Tyr Tyr Ser Ser Trp
1               5                   10                  15
Lys Asp Pro Ala Lys Cys
                20


<210> 12
<211> 22
<212> PRT
<213> Artificial sequence

```
<220>
<223> Synthetic peptide

<400> 12

Ala Glu Gly Glu Phe Pro His Gly Glu Ile Phe His Met Met Arg Arg
1               5                   10                  15
Pro Asp Pro Ala Lys Cys
                20


<210> 13
<211> 22
<212> PRT
<213> Artificial sequence

<220>
<223> Synthetic peptide

<400> 13

Ala Glu Gly Glu Phe Asn Arg Arg Ile Pro Cys Glu Thr Gly Cys Leu
1               5                   10                  15
Leu Asp Pro Ala Lys Cys
                20


<210> 14
<211> 22
<212> PRT
<213> Artificial sequence

<220>
<223> Synthetic peptide

<400> 14

Ala Glu Gly Glu Phe Thr Phe Thr Arg Phe Cys Gly Lys Gly Cys Val
1               5                   10                  15
Pro Asp Pro Ala Lys Cys
                20


<210> 15
<211> 115
<212> PRT
<213> Artificial sequence

<220>
<223> V_H domain of F22-4 antibody

<400> 15

Glu Val Lys Val Glu Glu Ser Gly Gly Gly Leu Val Gln Pro Gly Gly
1               5                   10                  15
Ser Met Lys Ile Ser Cys Val Val Ser Gly Leu Thr Phe Ser Asn Tyr
                20                  25                  30
Trp Met Ser Trp Val Arg Gln Ser Pro Glu Lys Gly Leu Glu Trp Val
            35                  40                  45
Ala Glu Ile Arg Leu Lys Ser Asp Asn Tyr Ala Thr Tyr Tyr Ala Glu
        50                  55                  60
```

```
Ser Val Lys Gly Lys Phe Thr Ile Ser Arg Asp Asp Ser Lys Ser Arg
65              70              75                      80
Leu Tyr Leu Gln Met Asn Asn Leu Arg Thr Glu Asp Thr Gly Ile Tyr
                85              90                      95
Tyr Cys Phe Leu Pro Met Asp Tyr Trp Gly Gln Gly Thr Ser Val Thr
            100             105             110
Val Ser Ser
        115
```

```
<210> 16
<211> 112
<212> PRT
<213> Artificial sequence

<220>
<223> V_L domain of F22-4 antibody

<400> 16

Asp Ile Val Met Thr Gln Ala Ala Phe Ser Asn Pro Val Thr Leu Gly
1               5               10                      15
Thr Ser Ala Ser Ile Ser Cys Arg Ser Ser Lys Ser Leu Leu His Ser
            20              25              30
Asp Gly Ile Thr Tyr Leu Tyr Trp Tyr Leu Gln Lys Pro Gly Gln Ser
            35              40              45
Pro His Leu Leu Ile Tyr His Leu Ser Asn Leu Ala Ser Gly Val Pro
        50              55              60
Asp Arg Phe Ser Ser Ser Gly Ser Gly Thr Asp Phe Thr Leu Arg Ile
65              70              75                      80
Ser Arg Val Glu Ala Glu Asp Val Gly Ile Tyr Tyr Cys Ala His Asn
                85              90                      95
Val Glu Leu Pro Arg Thr Phe Gly Gly Gly Thr Lys Leu Glu Ile Lys
            100             105             110
```

**Description**

The present invention is directed towards purified immunogenic polypeptides comprising an epitope unit recognized by a protective monoclonal antibody having a high affinity and a high specificity for the surface polysaccharide O-antigen from serotype 2a *Shigella flexneri*. The invention also concerns the use of said purified immunogenic polypeptides for the preparation of vaccine against serotype 2a *S. flexneri* infection and for the preparation of diagnostic compositions for the detection of serotype 2a *S. flexneri* infection. Finally, the present invention comprises high-resolution crystal structures of said natural surface polysaccharide O-antigen (O-Ag), or functional fragments thereof, possibly synthetic, andor of a mimetic peptide or functional fragments thereof in complex with the protective monoclonal antibody, or functional fragments thereof, and to methods for selecting immunogenic polypeptides for the preparation of new candidate vaccinal compositions based on said crystal(s).

Shigellosis (1), or bacillary dysentery, causes significant morbidity and mortality worldwide, particularly among young children (2). The disease arises from colonisation and subsequent destruction of the colonic mucosa by the Gram-negative entero-invasive bacteria *Shigella*. Immune protection induced by natural infection derives from antibodies directed against the bacterial surface antigen lipopolysaccharide (LPS) (3). Moreover, protection shows a serotype specificity that is determined by the repeat unit (RU) structure of the O-Ag, the polysaccharide moiety of LPS (4). In the species *S. flexneri,* which is responsible for endemic infections in developing countries, the serotype is defined by α-D-glucosyl and *O*-acetyl modifications added to the basic trirhamnose-*N*-acetyl-glucosamine tetrasaccharide (designated ABCD) of the O-Ag backbone (5). (Serotype 6 is an exception.) Of the 14 *S. flexneri* serotypes identified to date, the 2a serotype is the most prevalent in developing countries (2). The serotype 2a

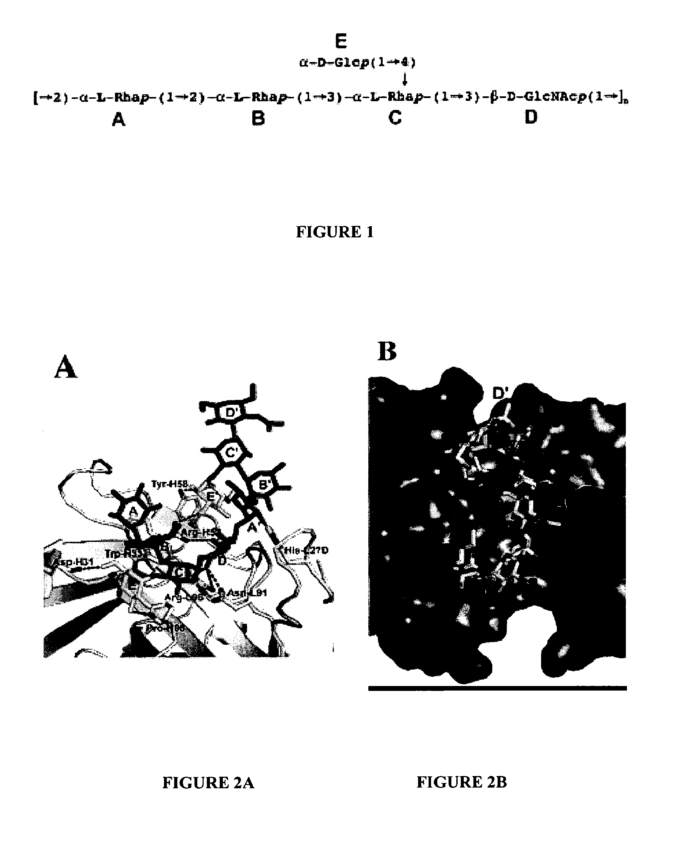

RU is characterized by a branching α-D-glucose (residue E) linked to the third rhamnose (residue C) to form the motif AB(E)CD (Figure 1).

The induction of protective immunity by natural infection with *Shigella* suggests that an effective vaccine, based for example on the O-Ag, is possible (1). No approved vaccine, however, is currently available, despite the many candidates in ongoing clinical trials (6). Nonetheless, polysaccharide-protein conjugates qualify as an important breakthrough in the field of anti-bacterial vaccines and, indeed, promising reports support this approach in the case of shigellosis (7), (8).

Carbohydrate antigens are T-cell independent, inducing weak antibody responses associated with the lack of a strong B cell memory response. Vaccine strategies have thus been mainly focused on the development of either polysaccharide-protein conjugates or anti-idiotype vaccines based on mimicking the carbohydrate structure. The difficult steps of the former approach are the purification of the polysaccharide (especially-when starting from LPS, which must be devoid of any residual lipid A-related endotoxic activity), and the loss of immunogenicity of the carbohydrate moiety during coupling to the protein carrier. Carbohydrate synthesis may diminish the problems associated with antigen purification, but nonetheless remains a limited solution due to the overall difficulties of carbohydrate chemistry.

The fact that the surface polysaccharide O-Ag of *S. flexneri* serotypes and chemically synthesized oligosaccharide fragments thereof seem to induce predominantly a T cell independent immune response or no response at all due to their hapten properties, renders these isolated or chemically synthesized antigens less valuable to use for inducing a protective immune response in the infected host.

Moreover, the synthesis of such polysaccharide antigen molecules at an industrial and commercial scale is difficult and very costly as compared with the synthesis of protein and peptide antigen compounds that are the active principals of the conventional vaccine compositions.

Thus, there is a need in the art to design protein or peptide molecules that are able to immunologically mimic the O-Ag of the serotype 2a *S. flexneri*, specifically that are able to induce a strong and protective immune response to the corresponding pathogenic organism.

This is the object of the present invention.

As an alternative to classical polysaccharide conjugate vaccines, we have developed a strategy based on synthetic carbohydrates that mimic the O-Ag of *S. flexneri* 2a, including a detailed analysis of the fine specificity of protective antibody/O-Ag recognition. Accordingly, a number of oligosaccharides representative of serotype 2a O-Ag fragments have been synthesized (9), (10) (11). Although several of these were immunogenic in mice when administered as tetanus toxoid conjugates, only certain sequences were able to induce IgG antibodies capable of recognizing the bacterial LPS (12), (13). Of particular note, the capacity of these synthetic glycoconjugates to induce IgG titers cross-reactive with LPS depended on the number of RUs present.

The synthetic oligosaccharides were also tested for their affinity to five serotype-specific murine IgG monoclonal antibodies (mAb) that we produced by infection with homologous bacteria (12). Of these five mAbs, all of which gave protection in a mouse model of infection, mAb F22-4 was unique in its binding pattern to different synthetic serotype 2a oligosaccharides and its variable domain sequence. For example, only F22-4 bound the trisaccharide ECD, the smallest oligosaccharide to be recognized (12).

The identification of peptides that mimic carbohydrate structures has also been reported (Phalipon et al., US patent serial number US 6,528,061 B1, WO 2005/003775).

As part of their general strategy, the inventors have determined the crystal structure of the Fab fragment of IgG F22-4 (protective anti-O-Ag of *S. flexneri* 2 mAb) in complex with the synthetic decasaccharide and pentadecasaccharide ligands, [AB(E)CD]$_2$ and [AB(E)CD]$_3$ (11), respectively. The inventors have demonstrated that the structures show that both ligands are bound in an identical way: six carbohydrate residues, contained within a contiguous nonasaccharide segment, make direct contacts with F22-4. They have also, selected, identified, and synthesized peptidepeptidic mimics capable of reacting with the F22-4 mAb when coupled to a protein carrier. These results have been compared with other antibody-carbohydrate structures and are discussed in the light of antigenic and immunogenic mimicry of *S. flexneri* 2a O-Ag by synthetic oligosaccharides that have been previously reported (12), (13).

In a first aspect, the present invention is directed to a purified, synthetic or recombinant polypeptide comprising a mimotope of the carbohydrate O-Ag from serotype 2a *S. flexneri* and recognized by a protective mAb having a specificity for the LPS of serotype 2a *S. flexneri,* wherein said mimotope is a 12 amino acid-long peptide selected from the group consisting of:

- DQAAQHPRDFHK (SEQ ID No. 1);
- YLEDWIKYNNQK (SEQ ID No. 2);
- MKSWASYQAHLM (SEQ ID No. 3);
- INDWISYYSSWK (SEQ ID No. 4);
- PHGEIFHMMRRP (SEQ ID No. 5);
- NRRIPCETGCLL (SEQ ID No. 6);
- TFTRFCGKGCVP (SEQ ID No. 7), and

homologous peptide thereof.In a preferred embodiment, said protective mAb having a specificity for the LPS of serotype 2a *S.flexneri* is the murine IgG named F22-4, or one of its functional fragments thereof, particularly its Fab fragment.

The hybridoma line secreting the F22-4 antibody has been deposited under the Budapest Treaty on the August 30, 2004 under the number I-3284 as part of the Collection Nationale de Culture des Microorganismes (CNCM) of the Institut Pasteur, Rue du Docteur Roux, Paris, France.

In a preferred embodiment, these peptides having the sequences SEQ ID Nos. 1 to 7 are synthesized with a flanking region AEGEF inserted at the N-terminal end and DPAKC inserted at the C-terminal end. Thus the invention also relates to synthetic or recombinant polypeptide comprising a mimotope selected from the group consisting of:

- AEGEFDQAAQHPRDFHKDPAKC (SEQ ID No. 8);
- AEGEFYLEDWIKYNNQKDPAKC (SEQ ID No. 9);
- AEGEFMKSWASYQAHLMDPAKC (SEQ ID No. 10);
- AEGEFINDWISYYSSWKDPAKC (SEQ ID No. 11);
- AEGEFPHGEIFHMMRRPDPAKC (SEQ ID No. 12);
- AEGEFNRRIPCETGCLLDPAKC (SEQ ID No. 13); and
- AEGEFTFTRFCGKGCVPDPAKC (SEQ ID No. 14).

In a more preferred embodiment, the polypeptide according to the invention comprises a mimotope selected from the group consisting of the peptides having the sequences SEQ ID Nos. 1 to 5 and SEQ ID Nos. 8 to 12. The mimotope having the sequence SEQ ID No. 2 or 9 is the most preferred.The present invention is also related to a polypeptide which is homologous to the initially selected polypeptide bearing at least a mimotope unit. By homologous peptide according to the present invention is meant a polypeptide containing one or several amino acid substitutions in the amino acid sequence of the initially selected polypeptide carrying a mimotope. In the case of an amino acid substitution, one or several consecutive or non-consecutive amino acids are replaced by "equivalent" amino acids. The expression "equivalent" amino acid is used herein to name any amino acid that may be substituted for one of the amino acids belonging to the initial polypeptide structure without decreasing the binding properties of the corresponding peptides to the mAb that has been used to select the parent peptide and without decreasing the immunogenic properties against the serotype 2a *S. flexneri.*

These equivalent amino acids can be determined either by their structural homology with the initial amino acids to be replaced, by the similarity of their net charge, and by the results of the cross-immunogenicity between the parent peptides and their modified counterparts.

The peptides containing one or several "equivalent" amino acids must retain their specificity and affinity properties to the biological targets of the parent peptide , as it can be assessed by a ligand binding assay or an ELISA assay. For example, amino acids can be placed in the following classes: non-polar, uncharged polar, basic, and acidic. Conservative substitutions, wherein an amino acid of one class is replaced with another amino acid of the same type, fall within the scope of the subject invention so long as the substitution does not materially alter (diminish) the biological activity of the compound.

The substitution is acceptable if it enhances the biological activity of the compound.

Examples of class of amino acids.

- Class of non-polar amino acid: A, V, L, I, P, M, F or W;

- Class of uncharged polar amino acid: G, S, T, C, Y, N or Q;

- Class of acidic amino acid: D or E; and

- Class of basic amino acid: K, R or H.

By modified amino acid according to the present invention is also meant the replacement of a residue in the L-form by a residue in the D-form or the replacement of a glutamic acid (E) residue by a pyroglutamic acid compound.

As an illustrative example, it should be mentioned the possibility to realize substitutions without a deep change in the immunogenic polypeptide binding properties of the corresponding modified peptides by replacing, for example, leucine by valine, or isoleucine, aspartic acid by glutamic acid, glutamine by asparagine arginine by lysine etc., it being understood that the reverse substitutions are permitted in the same conditions.

In order to design peptides homologous to the immunogenic polypeptides according to the present invention, one skilled in the art can also refer to the teachings of Bowie et al. (Sciences, 47: 1305-1310, 1990).

The polypeptides according to the present invention can be prepared in a conventional manner by peptide synthesis in liquid or solid phase by successive couplings of the different amino acid residues to be incorporated (from the N-terminal end to the C-terminal end in liquid phase, or from the C-terminal end to the N-terminal end in solid phase), wherein the N-terminal ends and the reactive side chains are previously blocked by conventional groups.

The polypeptides according to the present invention can be prepared in a conventional manner by genetic recombination (recombinant peptide). Methods for production of recombinant peptide are well known by the skilled person.

In another aspect, the present invention comprises a purified polynucleotide coding for a polypeptide according to the invention.

Cloning or expression vectors comprising a polynucleotide coding for a polypeptide according to the invention forms also part of the present invention.

The invention also concerns a prokaryotic or eukaryotic host cell that is modified by a polynucleotide or a vector as defined above, preferably an expression vector.

Host cells such plant cells, mammal cells, bacteria, yeast or fungi, can be transformed by a vector comprising a polynucleotide according to the invention.

In a preferred embodiment, the polypeptide according to the invention is covalently bound to a carrier, preferably to an immunocarrier which increases the immunogenicity of the mimotope.

The present invention is further directed to an immunogenic composition comprising a polypeptide or a polynucleotide according to the invention, and, optionally, a pharmaceutical acceptable vehicle.

The peptides or pseudopeptides according to the present invention are indeed advantageously combined with or contained in a heterologous structure, or polymerized in such a manner as to enhance their ability to induce a protective immune response against the pathogenic *S. flexneri.* As a particular embodiment of the immunogenic polypeptide according to the present invention, the immunogenic polypeptide can comprise more than one epitope unit, preferably about 2 to about 20 epitope units, more preferably about 2 to about 15 epitope units, and most preferably about 3 to about 8 epitope units per polypeptide molecule, usable as an active principle of a vaccine composition.

As another particular embodiment of the oligomeric immunogenic polypeptides according to the present invention, the peptides or pseudopeptides are embedded within a peptidic synthetic matrix in order to form a MAP (Multiple Antigenic Peptide) type structure Such MAP structures as well as their method of preparation are described for example in Tam, JP (PNAS, USA, vol. 85, 5409-5413, 1988). The embedding of the peptides or pseudopeptides of therapeutic value according to the present invention within MAP type structures can cause an increase in the immunogenic and/or protective properties of the initial molecules as regards to the pathogenic *S. flexneri* infection.

In another particular embodiment, the polypeptide according to the present invention will comprise an additional T-epitope that is covalently or non-covalently combined with said polypeptide of the invention. In a preferred embodiment, the additional T-epitope is covalently linked to the immunogenic polypeptide.

Thus, the MAP construct may comprise an additional T epitope, which is covalently linked to the immunogenic polypeptide of the MAP.

Illustrative embodiments of a suitable T-cell epitope to be combined with an immunogenic peptide mimotope according to the invention are, for example, a T-cell epitope from hepatitis delta or from woodchuck hepatitis, from the Influenza virus, from the rotavirus VP6 protein from the structural proteins of enteroviruses, specifically from the VP2, VP3, and VP1 capsid proteins, from tetanus toxin from Streptococcus mutans or from the VP1 capsid protein of the foot and mouth disease virus.

Preferred additional T-cell epitopes used according to the present invention are, for example, universal T-cell epitopes, preferably tetanus toxoid or also the VP1 poliovirus capsid protein.

In another embodiment of the peptide constructs according to the present invention, the immunogenic polypeptide is directly coupled with a carrier molecule, such as KLH (Keyhole Limpet Hemocyanin) IpA (invasion plasmid antigen) D, TT (tetanus toxoid), CRM 197 (cross-reactive material of diphtheria toxin), rEPA (*Pseudomonas aeruginosa* ExoProtein A). Useful multiple T epitopes and T epitopes are for exemple disclosed in

Infect. Immun. (2005) 73, 5835 or Infect. Immun. (2001) 31, 3816.

The polypeptides according to the invention can be presented in different ways to the immune system. In one specific embodiment the immunogenic carbohydrate peptide mimics of the invention can be presented under the form of ISCOMs (Immunostimulating complexes) that are composed of Quil A (a saponin extract from Quilaja saponaria olina bark), cholesterol and phospholipidsphospholipids associated with the immunogenic polypeptide.

The polypeptides according to the invention can also be presented onto filamentous phages surface. Van Houten, N.E. et al. Vaccine Vol 24, Issue 19, 8 May 2006, Pages 4188-4200.

The polypeptides of the invention, preferably comprising at least one aditional T-cell epitope, can also be presented in the form of biodegradable microparticles (microcapsules or microspheres), such as, for example, lactic and glutamic acid polymers, also termed poly(lactide-co-glycolide) microcapsules or microspheres.

Other microparticles used to present the polypeptide of the invention are synthetic polymer microparticles carrying on their surface one or more polypeptide mimotope units covalently bonded to the material of the microparticles, the method used for covalent binding between the immunogenic and the material making up the microparticle are well kown by the skilled person.

The immunogenic polypeptide mimics of the invention, preferably comprising at least one T-cell epitope, can also be included within or adsorbed onto liposome particles.

Thus, the present invention comprises a polypeptide, or an immunogenic composition to according to the invention, as a medicament, preferably for the preparation of a pharmaceutical or vaccinal composition intended to prevent or to treat serotype 2a *S. flexneri* infection, said composition or vaccine optionally further includes an adjuvant and/or a pharmaceutical acceptable carrier.

The invention thus pertains to a vaccine composition for immunizing humans and other mammals against *S. flexneri,* preferably serotype 2a *S. flexneri* infection, said vaccine composition comprising an immunogenic composition of the invention as described above in combination with a pharmaceutically compatible excipient (such as saline buffer), optionally in combination with at least one adjuvant of immunity, such as aluminum hydroxide or a compound belonging to the muramyl peptide family. Various methods for achieving adjuvant effect for the vaccine include the use of agents such as aluminum hydroxide or phosphate (alum), commonly used as 0.05 to 0.1 percent solution in phosphate buffered saline, in admixture with synthetic polymers of sugars (Carbopol) used as 0.25% solution. Another suitable adjuvant compound is DDA (dimethyldioctadecylammonium bromide), as well as immune modulating substances, such as lymphokines (e.g. gamma-IFN, IL-1, IL-2 and IL-12) and also gamma-IFN inducer compounds, such as poly I:C.

The vaccine according to the present invention is advantageously prepared as an injectable composition either as a liquid solution or suspension. The vaccine can also be provided in solid form suitable for solution in or suspension in a liquid prior to injection.

The vaccines are conventionally administered by parental route, by injection, for example, either subcutaneously or intramuscularly. Additional formulations are suitable for other modes of administration The vaccine compositions of the invention are administered in a manner compatible with the dosage formulation and in such amount as will be therapeutically effective and immunogenic. The quantity to be administered depends on the subject to be treated, including, e.g., the capacity of the individual's immune system to mount an immune response. Suitable dosage ranges are of the order of several hundred micrograms active immunogenic polypeptide with a preferred range about 0.1 $\mu$g g to about 1000 $\mu$g, preferably about 1 $\mu$g to about 300 $\mu$g and especially about 10 $\mu$g to about 50 $\mu$g. The dosage of the vaccine will depend on the route of administration and will vary according to the age of the patient to be vaccinated and, to a lesser degree, the weight of the person to be vaccinated.

In many instances, it will be necessary to proceed with multiple administrations of the vaccine composition according to the present invention. Preferably, the vaccine composition is administered several times.

In another aspect, the present invention is directed to a method for the production of isolated or purified antibodies, or a functional fragment thereof, susceptible to recognize the -O-Ag from serotype 2a *S. flexneri,* **characterized in that** said method comprises the steps of:

a) immunizing a suitable non-human mammal subject with an immunogenic composition comprising a polypeptide, a polynucleotide or an immunogenic composition according to the invention, said immunogen composition optionally further including an adjuvant; and

b) selecting the antibodies which are capable of specifically recognizing said O-Ag.

The antibodies obtained by the method of the present invention and the polypeptides according to the present invention are used, *in vitro* as *S. flexneri* type 2a specific diagnostic reagents in standard immunoassays.

The antibodies according to the present invention are used to test for the presence of *S. flexneri* type 2a in biological samples, for establishing the diagnosis of shigellosis in an individual presenting a diarrhoeal disease.

Alternatively, the polypeptides according to the present invention are used to test the presence of *S. flexneri* type 2a- specific antibodies. The polypeptides according to the present invention may be used for epidemiological studies, for example for determining the geographic distribution and/or the evolution of *S. flexneri* type 2a infection worldwide, as well as for evaluating the *S. flexneri* type 2a-specific antibody response induced by an immunogen.

The antibodies and the polypeptides according to the present invention may be advantageously labelled and/or immobilized onto a solid phase, according to standard protocols known to one skilled in the art. Such labels include, but are not limited to, enzymes (alkaline phosphatase, peroxydase), luminescent or fluorescent molecules. For example an oligo- or polysaccharide conjugated to biotin, according to the present invention may be immobilized onto a solid phase, to detect the presence of *S. flexneri* type 2a-specific antibodies in biological samples.

Such immunoassays include, but are not limited to, agglutination assays, radioimmunoassay, enzyme-linked immunosorbent assays, fluorescence assays, western- blots and the like.

Such assays may be for example, of direct format (where the labelled antibody/oligo- or polysaccharide is reactive with the antigen/antibody to be detected), an indirect format (where a labelled secondary antibody is reactive with said antibody/oligo or polysaccharide), a competitive format (addition of a labelled antibody/oligo- or polysaccharide), or a sandwich format (where both labelled and unlabelled antibodies are used).

For all therapeutic, prophylactic and diagnostic uses, the polypeptides according to the invention, alone or linked to a carrier, as well as antibodies and other necessary reagents and appropriate devices and accessories may be provided in kit form so as to be readily available and easily used.

Thus, the present invention relates to a kit for the diagnostic of serotype 2a *S. flexneri* infection, **characterized in that** said kit comprises the following elements:

a) a polypeptide according to the present invention; or
b) an antibody, or one of its functional fragments, obtained by the method according to the present invention;
c) optionally, the reagents for the formation of the medium favorable to the immunological reaction; and
d) optionally, the reagents allowing the demonstration of antigen-antibody complexes produced by the immunological reaction.

The present invention also comprises diagnostic method for detecting the presence of serotype 2a *S. flexneri* infection in a patient from a biological sample of said patient, said method comprising the steps of:

a) bringing into contact the biological sample with :

- a polypeptide according to the present invention; or
- an antibody, or one of its functional fragments, obtained by the method according to the present invention; and

b) detecting the formation of antigen-antibody complexes.

In another aspect, the present invention comprises a method to identify a mimotope which fully mimics the O-Ag epitopes from serotype 2a *S. flexneri* and is recognized by a protective mAb having a specificity for the lipopolysaccharide (LPS) of the surface antigen of serotype 2a *S. flexneri*, wherein said method implements the use of a O-Ag subunit from serotype 2a *S. flexneri* comprising at least two RUs of synthetic serotype 2a polysaccharide.

Preferably, said at least two RUs of synthetic serotype 2a polysaccharide are the $[AB(E)CD]_2$ decasaccharide or the pentadecasaccharide $[AB(E)CD]_3$ and wherein:

A is an alphaLRhap-(1,2) residue, B is an alphaLRhap-(1,3) residue, C is an alphaLRhap-(1,3) residue, E is an alphaDGlcp-(1,4) residue and D is a betaDGlcNAcp-(1,2) residue.

The present invention also comprises:

- A crystal compound obtained from the free Fab F22-4 antibody and having the following structural parameters:

space group          P1

(continued)

| | | |
|---|---|---|
| unit cell | | |
| | a, b, c (Å) | 47.2, 59.8, 74.4 |
| | α, β, γ (°) | 77.5, 84.0, 80.9 |
| | Z (molecules/unit cell) | 2 ; |

- A crystal having the three-dimensional atomic coordinates shown in Table 14;
- A complex crystal compound (also named "co-crystal") compound obtained from the Fab F22-4 antibody complexed with the 2a serotype [AB(E)CD]$_2$ decasaccharide and having the following structural parameters:

| | | |
|---|---|---|
| space group | | P21 |
| unit cell | | |
| | a, b, c (Å) | 67.1, 137.6, 109.8 |
| | α, β, γ (°) | 90, 95.2, 90 |
| | Z (molecules/unit cell) | 4; |

- A crystal (or co-crystal) having the three-dimensional atomic coordinates shown in Table 15;
- A complex crystal compound (or co-crystal) obtained from the Fab F22-4 antibody complexed with the 2a serotype pentadecasaccharide [AB(E)CD]$_3$ and having the following structural parameters:

| | | |
|---|---|---|
| space group | | P21 |
| unit cell | | |
| | a, b, c (Å) | 66.9, 137.2, 109.4 |
| | α, β, γ (°) | 90, 95.0, 90 |
| | Z (molecules/unit cell) | 4; |

- A crystal (or co-crystal) having the three-dimensional atomic coordinates shown in Table 16; and
- A complex crystal compound (or co-crystal) obtained from the Fab F22-4 antibody complexed with the mimotope peptide B1 having the sequence SEQ ID No.9 and the three-dimensional atomic coordinates shown in Table 13.

In general aspects, the present invention is concerned with identifying or obtaining a mimotope of an O-Ag subunit (mimotope du O-Ag pas d'un fragment du O-Ag) from serotype 2a *S. flexneri* for the preparation of vaccine or antibodies. Crystal structure information presented herein is useful in designing potential mimotope of an O-Ag subunit (voir au-dessus) from serotype 2a *S. flexneri* and modelling them or their potential interaction with the Fab F22-4 binding sites. Potential mimotopes may be brought into contact with the Fab F22-4 to test for ability to interact with the Fab F22-4 binding site. Actual mimotopes may be identified from among potential antigens synthesized following design and model work performed in silico. A mimotope identified using the present invention may be formulated into a composition, for instance a composition comprising a pharmaceutically acceptable excipient, and may be used in the manufacture of a drug for use in a method of treatment.

The present invention also relates to a method to screen a mimotope candidate of the O-Ag from serotype 2a *S. flexneri* and recognized by a protective mAb antibody having a specificity for the LPS of serotype 2a *S. flexneri*, comprising the steps of assessing the interaction of three-dimensional models of such tested mimotopes to screen with the structure of the Fab F22-4 as defined by the atomic coordinates of tables 13 to 16, particularly with at least one, preferably 2, 3, 4, 5, 6, 7 and 8 of the following amino acids: Asn L91, Arg L96, Arg H52, Tyr H58, Pro H95, Trp H33, Asn H31 and His L27D of the Fab F22-4 antibody $V_H$ and $V_L$ sequences.

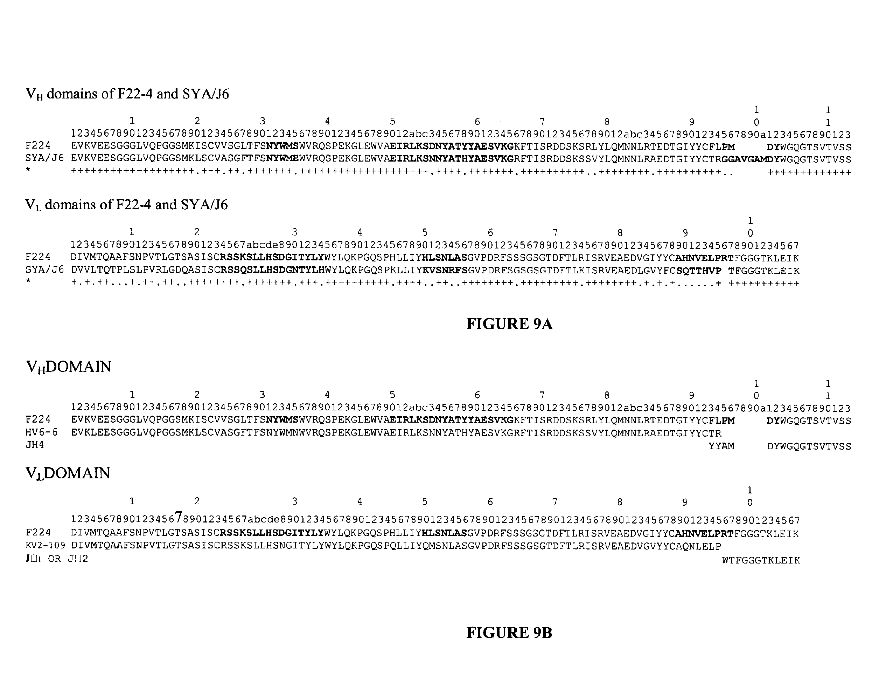

The wording "Asn L91" is intended to designate an asparagin aminoacid residue in position indicated 91 of the $V_L$ sequence of the figure 9 which corresponds to the asparagin aminoacid residue in position 96 in the sequence SEQ ID No. 16 due to the insertion of the additional positions 27A, 27B, 27C, 27D and 27E in the numbering of that $V_L$ sequence in figure 9.

The wording "Tyr H58" is intended to designate a tyrosine aminoacid residue in position indicated 58 of the $V_H$ sequence of the figure 9 which corresponds to the tyrosine aminoacid residue in position 61 in the sequence SEQ ID No. 15 due to the insertion of the additional positions 52A, 52B and 52C in the numbering of that $V_L$ sequence in figure 9.

The wording "Pro 95" is intended to designate a proline aminoacid residue in position indicated 95 of the $V_H$ sequence of the figure 9 which corresponds to the proline aminoacid residue in position 101 in the sequence SEQ ID No. 15 due to the insertion of the additional positions 52A, 52B, 52C and 82A, 82B, 82C in the numbering of that $V_L$ sequence in figure 9.

**EP 2 123 668 A1**

Thus the position Asn L91, Arg L96, Arg H52, Tyr H58, Pro H95, Trp H33, Asn H31 and His L27D of the Fab F22-4 antibody $V_H$ and $V_L$ sequences are referenced with regard to the aminoacid position numbered and depicted for the Fab F22-4 antibody $V_H$ and $V_L$ sequences represented in figure 9.

The present invention also comprises a method of identifying a mimotope candidate of the O-Ag from serotype 2a *S. flexneri* and recognized by a protective mAb antibody having a specificity for the LPS of serotype 2a *S. flexneri,* comprising the steps of:

> a) providing a model of a binding site of Fab F22-4 antibody, said model including at least the binding site presenting at least one of the 11 hydrogen bond interactions between Fab F22-4 antibody and the O-Ag fragment defined by Table 2, preferably at least 2, 3, 4, 5, 6, 7, 8, 9, 10 and the 11 hydrogen bond interactions;
> b) providing the structure of a candidate agent compound;
> c) fitting the candidate agent compound to said binding site, including determining the interactions between the candidate agent compound and the at least one of the 11 hydrogen bond interactions between Fab F22-4 antibody and the O-Ag fragment defined by Table 2, preferably and the at least 2, 3, 4, 5, 6, 7, 8, 9, 10 and the 11 hydrogen bond interactions; and
> d) selecting the fitted candidate agent compound whether this candidate compound satisfies the hydrogen bond interactions required.

By "fitting", is meant determining by automatic, or semi-automatic means, interactions between one or more atoms of a molecule and one or more atoms or binding sites of the Fab F22-4, and determining the extent to which such interactions are stable. Various computer-based methods for fitting are well known by a skilled man.

The present invention also comprises a method of identifying a mimotope candidate of the O-Ag from serotype 2a *S. flexneri* and recognized by a protective mAb having a specificity for the LPS of serotype 2a *S. flexneri,* comprising the steps of:

> a) providing a model of a complex crystal compound according to the invention; and
> b) forming a complex between Fab F22-4 antibody and said candidate agent compound; and
> c) analysing said formed complex by X-ray crystallography or NMR spectroscopy to determine the ability of said candidate agent compound to interact with the binding site comprising the 11 hydrogen bond interactions shown in table 2; and
> d) determining the ability of said candidate compound to interact with said binding site by comparing the three-dimensional atomic coordinates obtained for this candidate agent with the atomic coordinates of the complex crystal model of a).

The invention also relates to a method according to the invention, comprising the further step of: e) immunizing a non human mammal with said selected candidate agent and determine whether said candidate compound is able to raise specific antibodies directed against the serotype 2a *S. flexneri* LPS..

In another aspect, the invention comprises a computer system, intended to generate structures and/or perform rational drug design for mimotope candidates of the O-Ag from serotype 2a *S. flexneri,* the system containing either (a) at least some (preferably at least 10, 50, 100, 200, 500, 1000 and 2000) atomic coordinates of the crystal compound according to the invention, said data defining the three-dimensional structure of said crystal compound, or (b) structural parameters for crystal compound according to the invention.

Preferably, the system containing either (a) at least the atomic coordinates of the at least one, preferably 2, 3, 4, 5, 6, 7 and 8 of the amino acids Asn L91, Arg L96, Arg H52, Tyr H58, Pro H95, Trp H33, Asn H31 and His L27D of the Fab F22-4 antibody $V_H$ and $V_L$ sequences contained in the crystal compound according to the invention,
The present invention relates to a computer-based method of rational drug design comprising:

> a) providing a three-dimensional structure of Fab F22-4 antibody as defined by the atomic coordinates of Table 14;
> b) providing a three-dimensional structure of a candidate mimotope of the O-Ag from serotype 2a *S. flexneri* compound; and
> c) fitting the structure of said candidate modulator compound to the structure of said Fab F22-4 antibody, particularly the binding site region determined by the following amino acids positions of the Fab: Asn L91, Arg L96, Arg H52, Tyr H58, Pro H95, Trp H33, Asn H31 and His L27D of the Fab F22-4 antibody sequence (see SEQ ID Nos. 15 and 16, or figure 9).

The present invention also comprises a computer readable media with either (a) at least one atomic coordinates of the crystal compound according to the invention, said data defining the three-dimensional structure of said crystal compound, or (b) structural parameters for crystal compound according to the invention.

By a "computer system" it is intended to designate the hardware means, software means and data storage means used to analyse atomic coordinate data. The minimum hardware means of the computer-based systems of the present invention comprises a central processing unit (CPU), input means, output means and data storage means. Desirably a monitor is provided to visualise structure data. The data storage means may be RAM or means for accessing computer readable media of the invention. Examples of such systems are microcomputer workstations available from Silicon Graphics Incorporated and Sun Microsystems running Unix based, Windows NT or IBM OS/2 operating systems.

By "computer readable media" it is intended to designate any media which can be read and access directly by a computer e.g. so that the media is suitable for use in the above-mentioned computer system. The media include, but are not limited to: magnetic storage media such as floppy discs, hard disc storage medium and magnetic tape; optical storage media such as optical discs or CD-ROM; electrical storage media such as RAM and ROM; and hybrids of these categories such as magnetic/optical storage media.

The following figures and their legends are illustrative of embodiments of the present invention and are not meant to limit the scope of the invention as encompassed by the claims.

**Figure legends**

**Figure 1**. Chemical structure of the 2a serotype O-Ag pentasaccharide repeat unit AB(E)CD.

**Figures 2A-2B.** Views showing the interaction between F22-4 and [AB(E)CD]$_2$.

Figure 2A: View from above the antigen-binding site. The variable domains of F22-4 are shown in surface representation with the V$_H$ domain in purple and the V$_L$ domain in blue. [AB(E)CD]$_2$ is shown in atomic form with carbon in yellow, oxygen in red and nitrogen in blue. Water molecules bound to [AB(E)CD]$_2$ (and in some cases to the antibody as well) are shown as red spheres.

Figure 2B: A detailed view of [AB(E)CD]$_2$ bound to F22-4. Rha residues are in purple, Glc are in blue and GlcNAc are in green. The antibody is shown in ribbon form with residues hydrogen bonding to the ligand in atomic form. Antibody-antigen hydrogen bonds are shown as dashed lines. (See Figure 1 for stereo view).

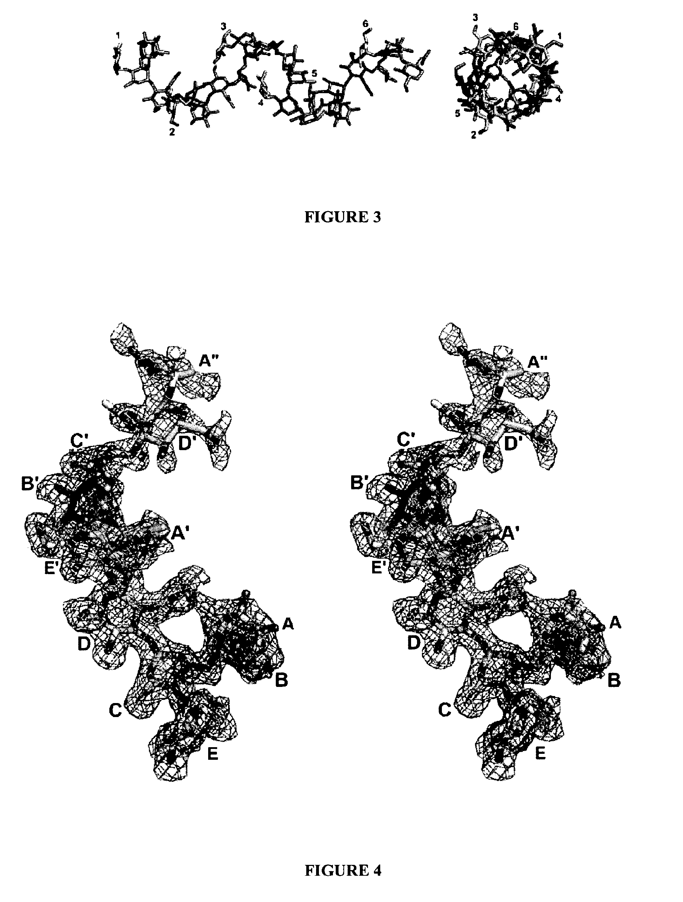

**Figure 3.** Views of the helical model of serotype 2a O-Ag generated from the structure of the F22-4/oligosaccharide complexes as described in the text. The two views are orthogonal and are taken perpendicular and parallel to the helical axis. Six RUs are shown, corresponding to almost two complete helical turns. The backbone residues are shown in brown and the branching glucose residues are in cyan. The glucose residues are numbered according to the RU to which they belong, beginning from the non-reducing end.

**Figure 4.** Stereo view of the ligand in the [AB(E)CD]$_3$ complex showing electron density from a difference omit map using phases calculated after refinement without the antigen atoms. The contour level is set at 2 times the r.m.s. of the electron density map. Visible sugar units are labelled. Weak density can be noted for residues D' and A" at the top of the figure.

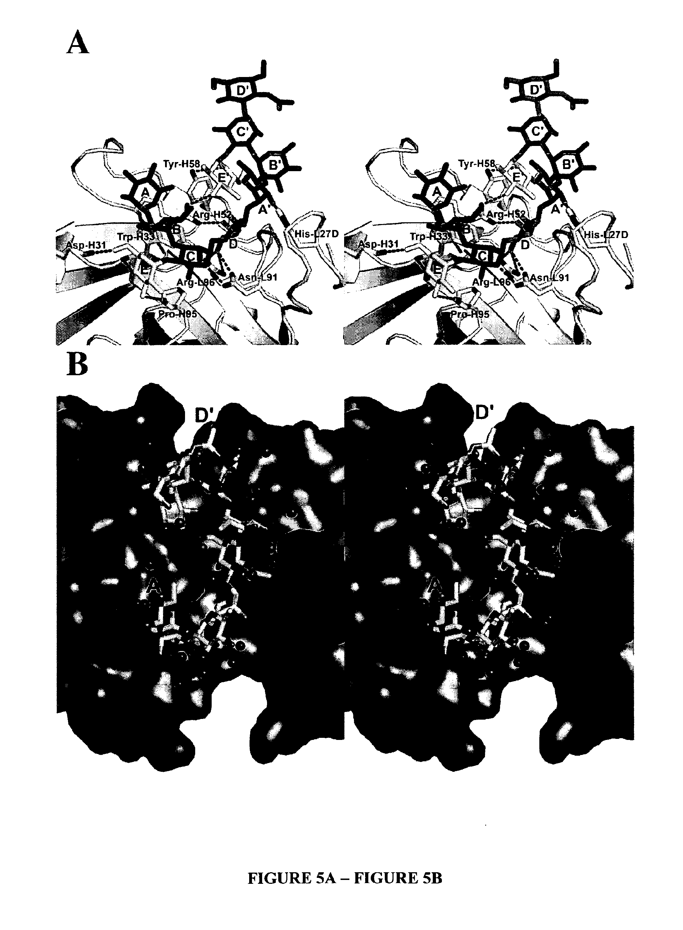

**Figure 5A-5B.** Views showing the interaction between F22-4 and [AB(E)CD]$_2$.

Figure 5A: A detailed view of [AB(E)CD]$_2$ bound to F22-4. Rha residues are in purple, Glc are in blue and GlcNAc are in green. The antibody is shown in ribbon form with residues hydrogen bonding to the ligand in atomic form. Antibody-antigen hydrogen bonds are shown as dashed lines.

Figure 5B: View from above the antigen-binding site. The variable domains of F22-4 are shown in surface representation with the V$_H$ domain in purple and the V$_L$ domain in blue. [AB(E)CD]$_2$ is shown in atomic form with carbon in yellow, oxygen in red and nitrogen in blue. Water molecules bound to [AB(E)CD]$_2$ (and in some cases to the antibody as well) are shown as red spheres.

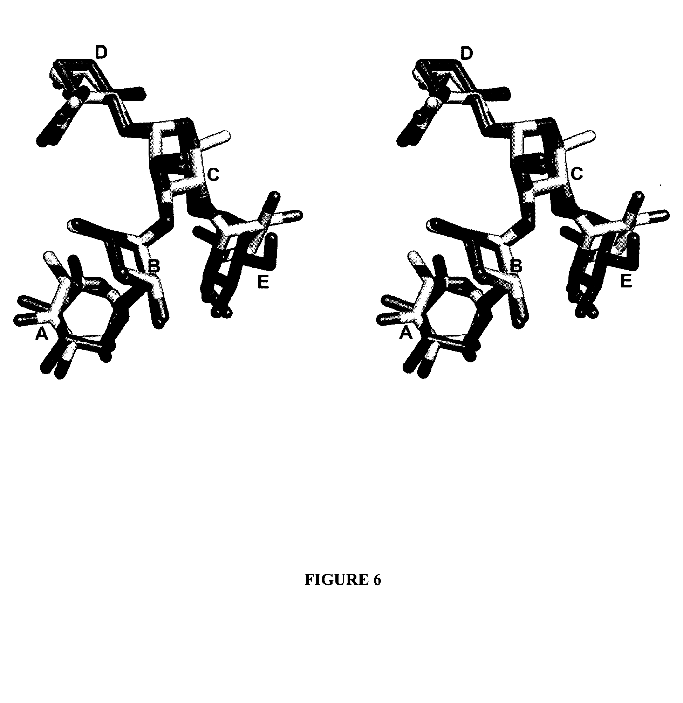

**Figure 6.** Comparison of the two RUs in the [AB(E)CD]$_2$ complex. Superposition was performed using the backbone residues only (ABCD) because the glycosidic dihedral angles of the branching glucose residues differ significantly between the two RUs. The r.m.s. difference between the two RUs after superposition is 0.44 Å for all atoms of the ABCD backbone. The first RU is coloured according to atom type (carbon in yellow, oxygen in red, nitrogen in blue) whereas the second RU is shown in magenta. The sugar rings are labelled. The close conformational similarity between the tetrasaccharide backbone units can be seen; in spite of the difference in the glycosidic dihedral angles of the branching glucose residues (E) for the two RUs, these two residues occupy essentially the same region in space after superposition.

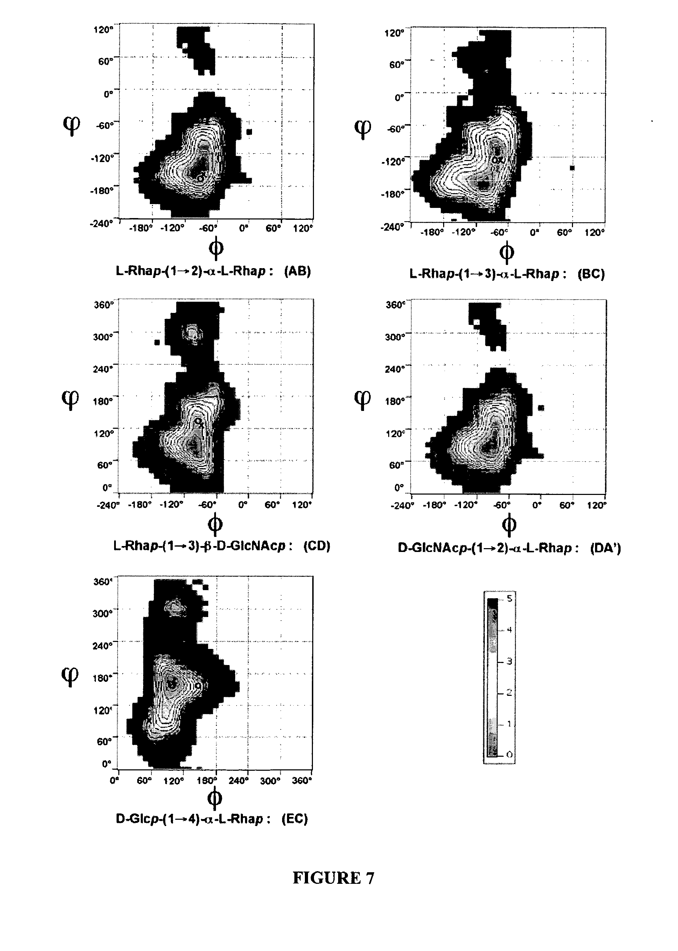

**Figure 7**. $\phi$,$\Pi$ conformational maps of disaccharide glycosidic linkages obtained from the GlycoMapsDB database (www.glycosciences.de/modelling/glycomapsdb; (14)). The coloured energy scale is in Kcals./mole and is measured relative to the energy minimum. Crosses indicate the mean 7 observed values for the first RU of the decasaccharide whereas the open circles indicate the values for the second RU. The ),$\Pi$ angles are defined according to the IUPAC convention for X-ray crystal structures (http://www.chem.qmul.ac.uk/iupac/misc/psac.html).

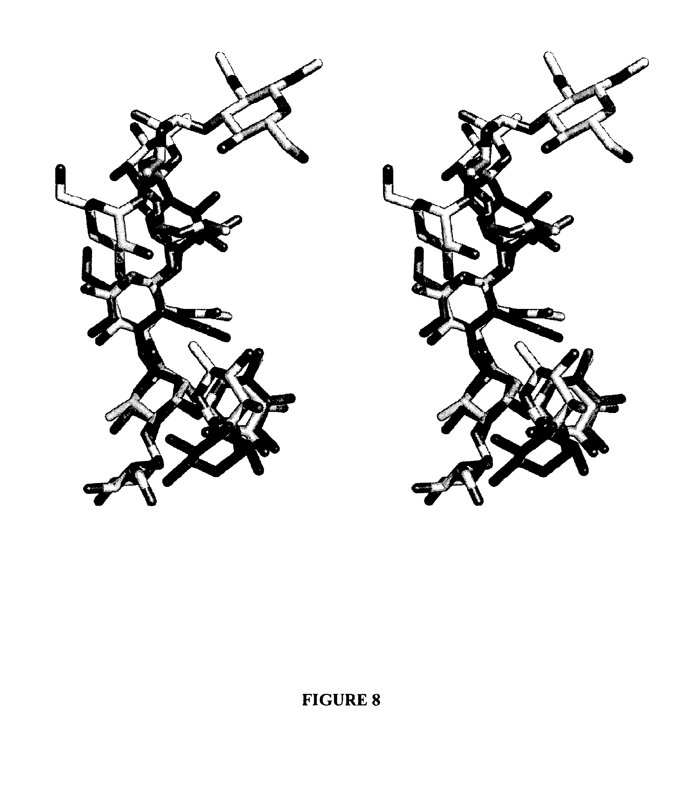

**Figure 8.** Comparison in stereo between the serotype 2a decasaccharide (AB(E)CDA'B'(E')C'D' and the serotype Y pentasaccharide (ABCDA') (15) as seen in their respective Fab complexes. The superposition was made by superimposing all atoms from the trisaccharide BCD. The serotype 2a saccharide is coloured according to atom type (carbon in yellow, oxygen in red, nitrogen in blue) and the serotype Y saccharide is shown in magenta.

**Figures 9A-9B.**

Figure 9A: Comparison of the variable domain sequences of F22-4 and SYA/J6

Figure 9B: Comparison of F22-4 variable domain sequences with germline.

**Figure 10.**

Stereo view of the Fab F22-4/peptide B1 complex viewed from above the antigen-binding site. The Fab is shown in surface presentation with the VL domain in orange and the VH domain in mauve; the peptide is shown in atomic presentation.

**Figures 11A-11B.**

Comparison of decasaccharide and peptide B1 interactions with F22-4: (A) View of the decasaccharide and the mimetic peptide B1 bound to Fab F22-4 viewed from above the antigen-binding site. The Fab is shown in surface presentation with the VL domain in orange and the VH domain in mauve. The helical cross-reacting peptide is shown in schematic ribbon presentation. (B) Stereo view showing the atomic structure of the decasaccharide (carbon in yellow) and the peptide B1 (carbon in blue) showing in detail their relative displacement in the antigen-binding site.

**Figure 12.**

Buried surface area of the Fab at the antibody/antigen interface given per Fab residue.

**Figure 13.**

Superposition of the ten lowest energy solution structures of peptide B1, derived by NMR, onto the peptide of the first complex in the asymmetric unit of the crystal structure. The crystal structure is represented in solid stick form showing all atoms whereas the solution structures are represented in line form showing the main chain atoms only.

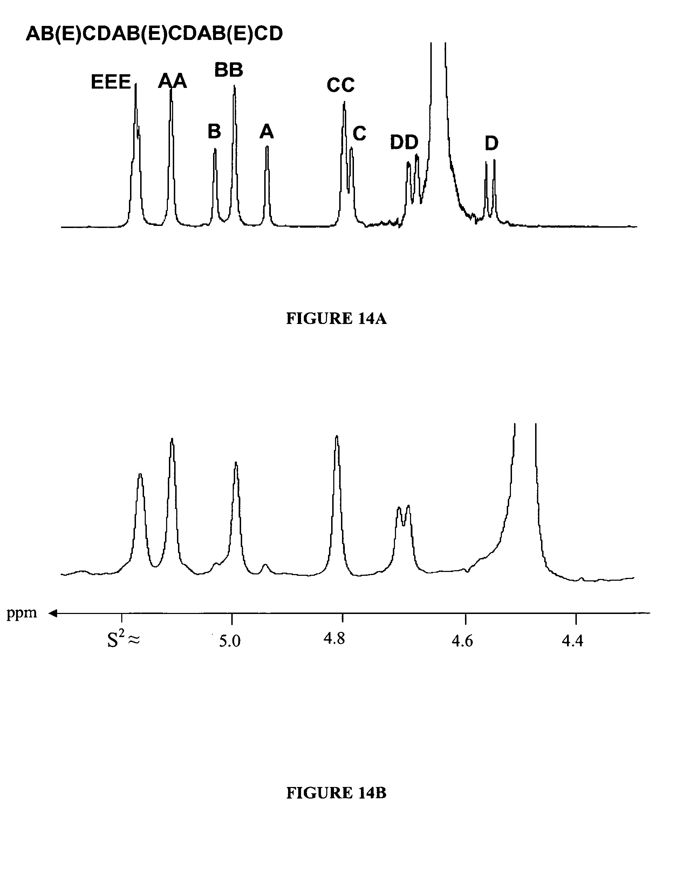

**Figures 14A-14B**. 1-D proton-NMR spectra of anomeric region

Figure 14A: pentadecasaccharide [AB(E)CD]$_3$ at 35°C.

Figure 14B: O-Ag 2a de-O-acetylated at 50°C.

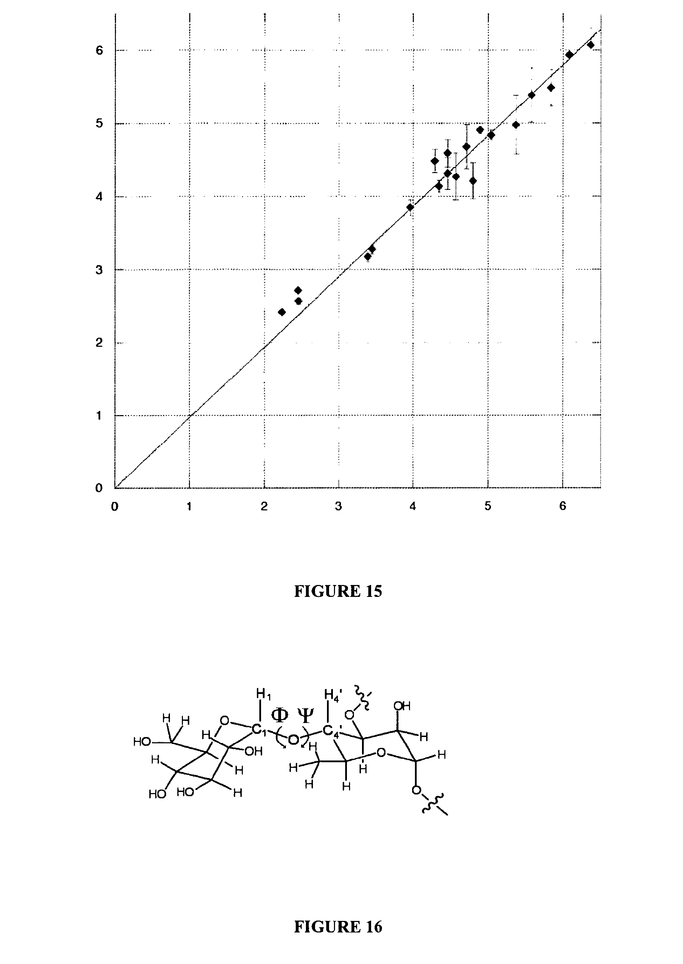

**Figure 15.** Computed from MD-trajectories (pentadecasaccharide; X-axis) vs NMR-experimental (O-Ag; Y-axis) inter-residual proton-proton NOE-distances. The fitted curve is y=0.967*x , with a linear correlation coefficient R=0.982.

**Figure 16.** Glycosidic angles for the E-C linkage

**Figures 17A-17B.**

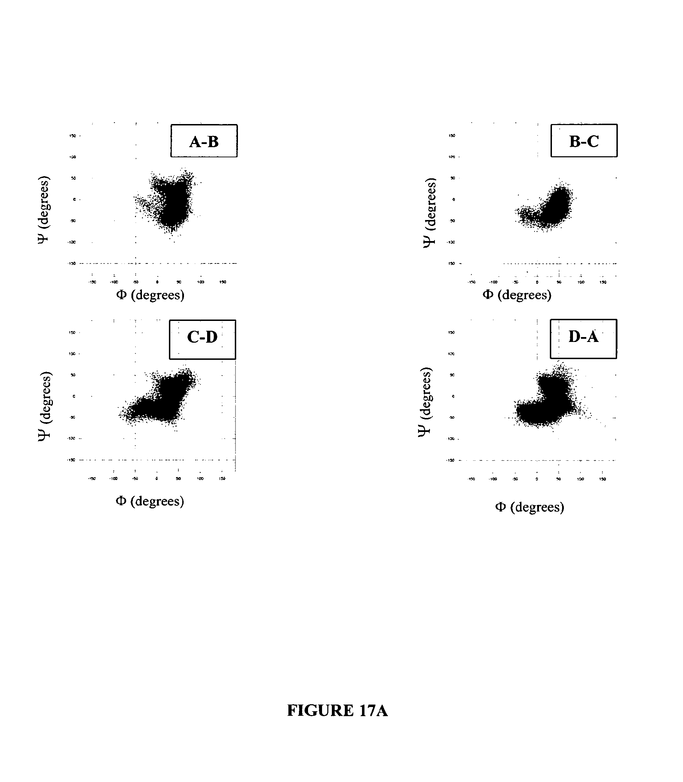

Figure 17A: Φ, Ψ plot for the glycosidic linkages of [AB(E)CD]$_3$ main chain sequence (unit 2) during MD simulation.

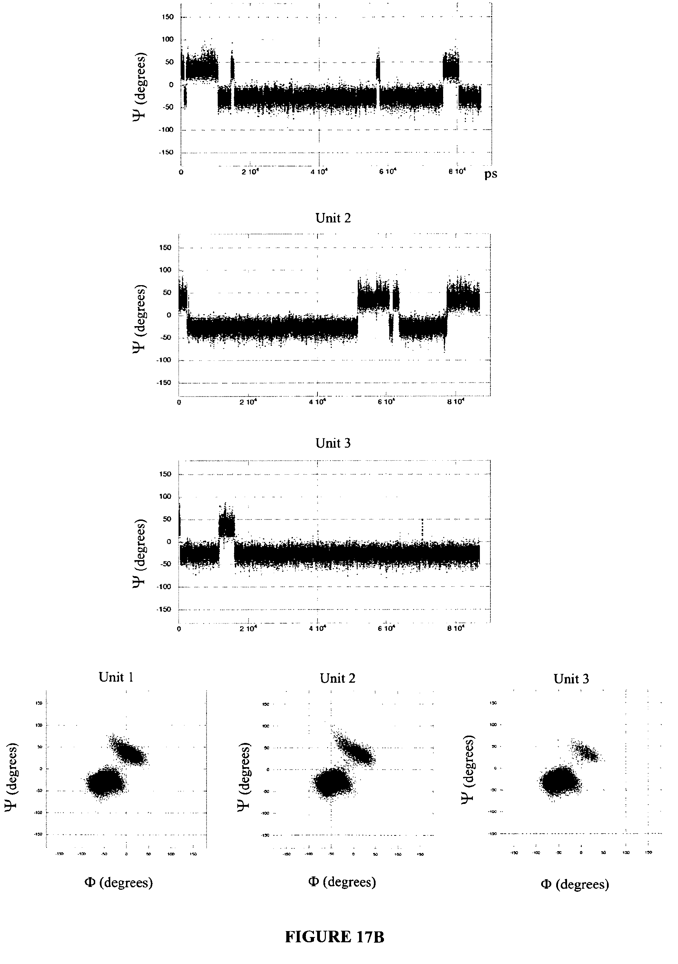

Figure 17B: Trajectories for Ψ-torsion angles for the E-C sequence in the [AB(E)CD]$_3$ pentadecasaccharide and corresponding Φ, Ψ plot.

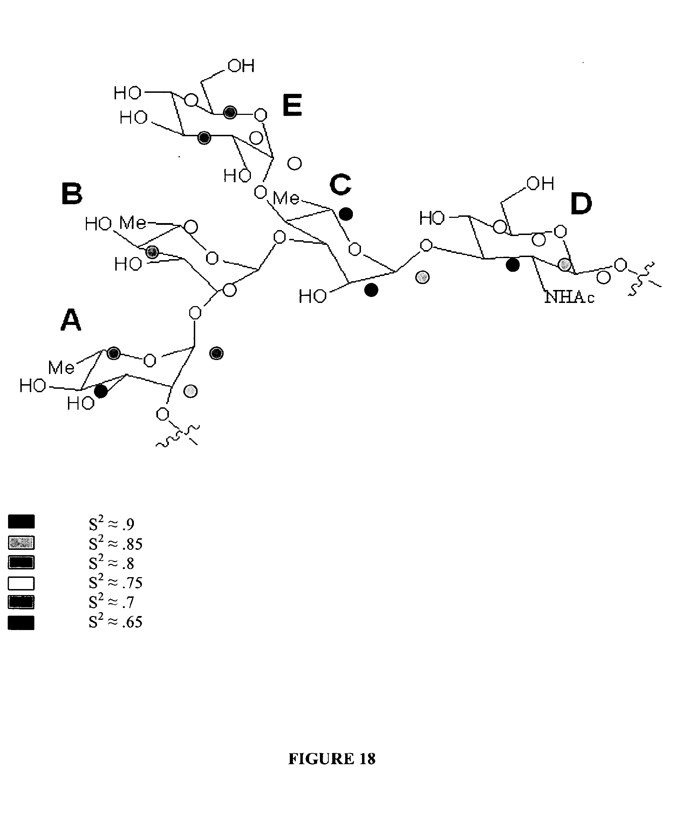

**Figure 18.** Calculated order parameters of CH vectors according to R1, R1ρ and 13C-1H noes observed at 600MHz for O-Ag 2a.

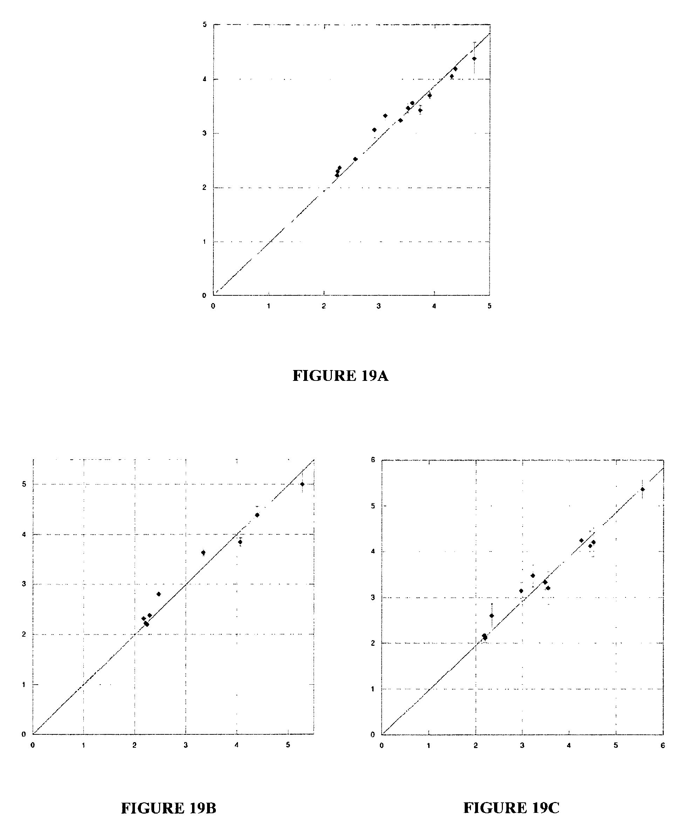

**Figures 19A-19C.** Computed from MD-trajectories (X-axis) vs NMR-experimental (Y-axis) NOEs distances: (The fitted curve is y=*x, with a linear correlation coefficient R=).

Figure 19A: B(E)CDA-OMe (y=0.969*x; R=0.977);

Figure 19B: ECDAB-OMe (y=0.998*x; R=0.980);

Figure 19C: DAB(E)C-OMe (y=0.971*x; R=0.980).

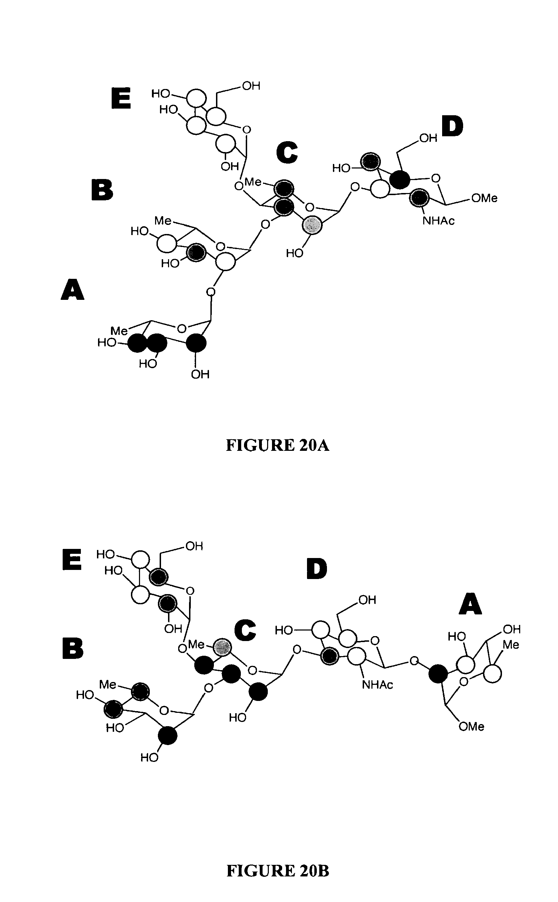

**Figures 20A-20D.** Calculated order parameters of CH vectors according to R1, R1ρ and 13C-1H noes observed at 600MHz for pentasaccharides

Figure 20A: AB(E)CD;

Figure 20B: B(E)CDA;

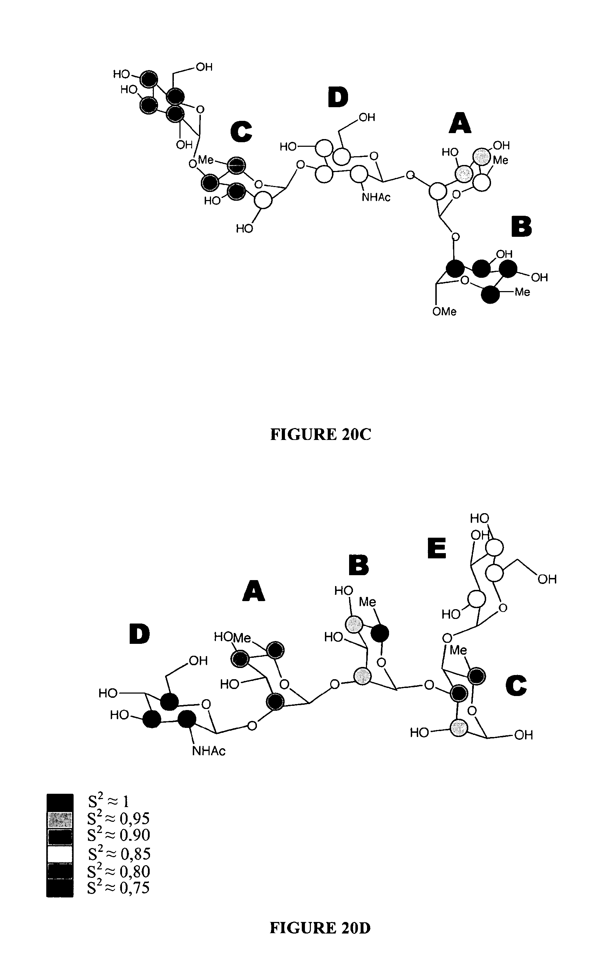

Figure 20C: ECDAB;

Figure 20D: DAB(E)C.

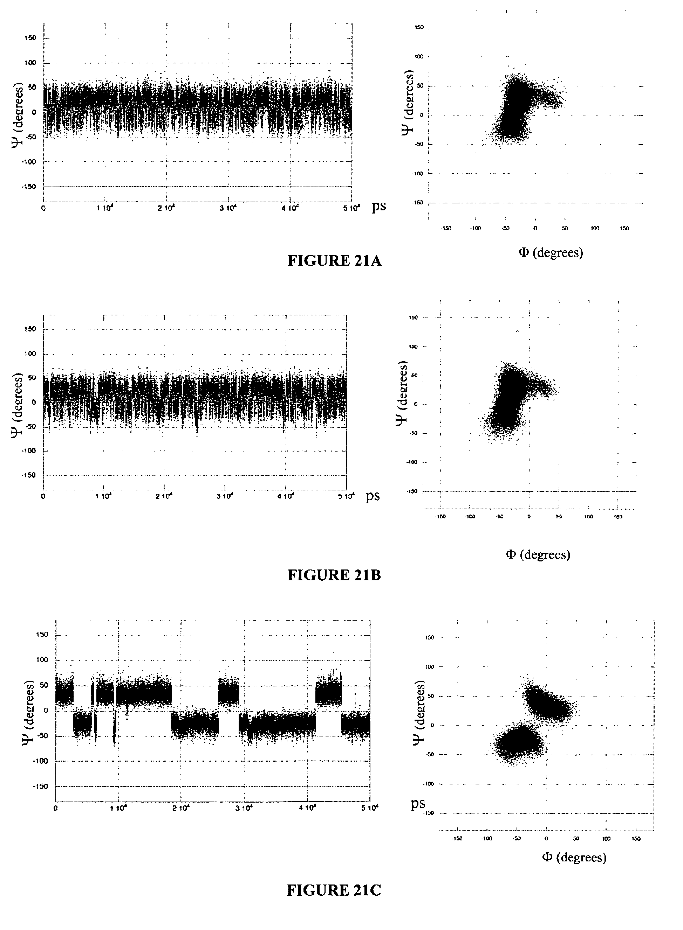

**Figures 21A-21F.** D-trajectories for Ψ-torsion angles and (Φ,Ψ) plot for the glycosidic linkage E-C of oligosaccharides.

Figure 21A: ECD;

Figure 21B: ECDAB;

Figure 21C: B(E)CD;

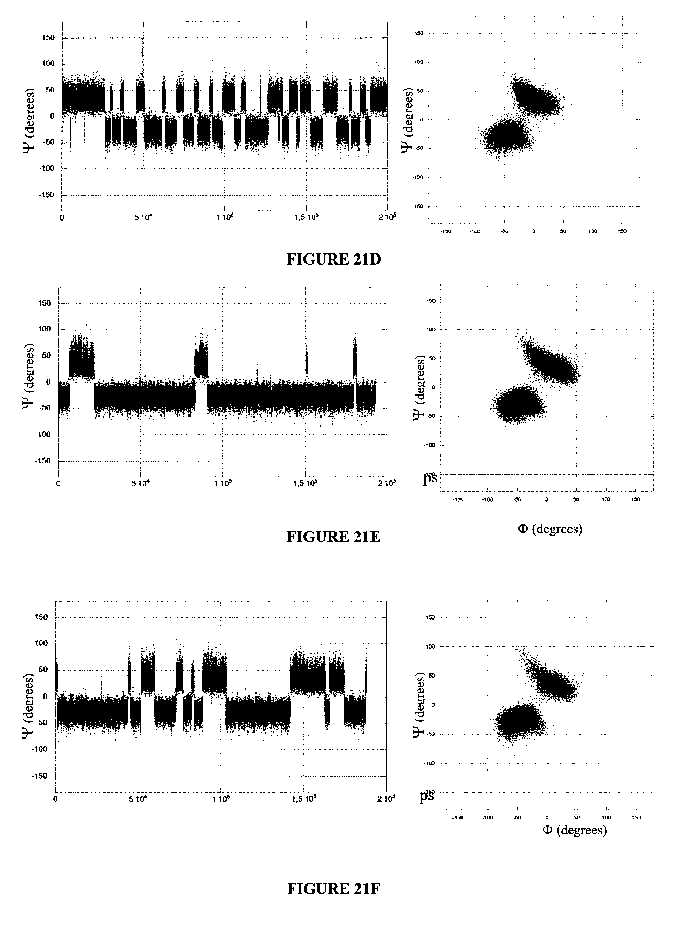

Figure 21D: B(E)CDA;

Figure 21E: DAB(E)C;

Figure 21F: AB(E)CD.

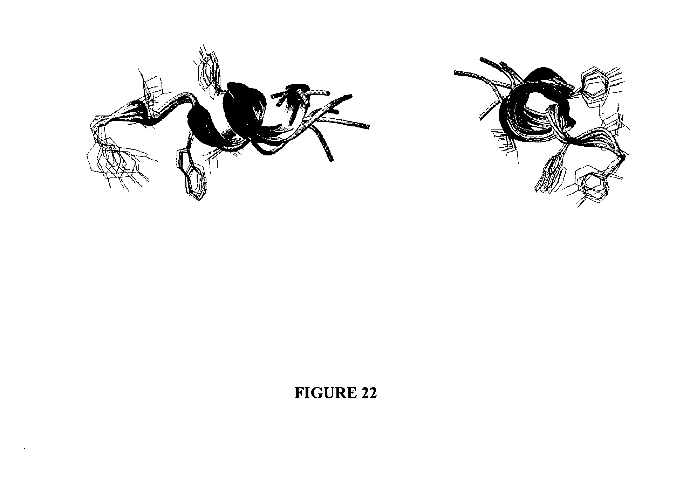

**Figure 22.** Ribbon representation of the 9 better structures.

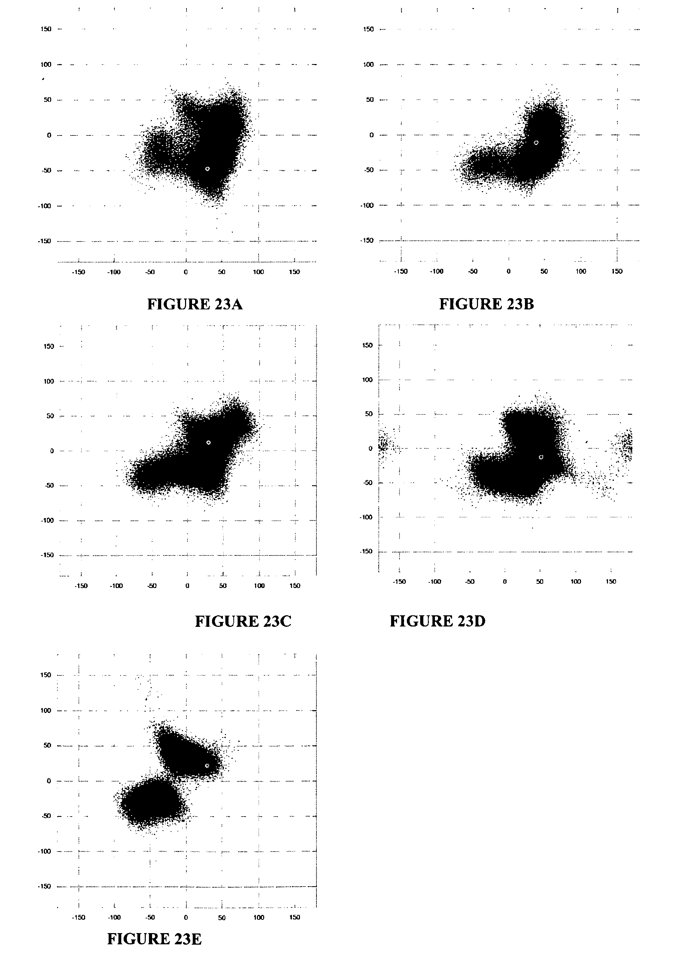

**Figures 23A-23E.** (Φ, Ψ) plots for the glycosidic linkages.

Figure 23A: A-B;

Figure 23B: B-C;

Figure 23C: C-D;
Figure 23D: D-A;
Figure 23E: E-C.
MD trajectory for B(E)CDA is represented in black dots, and the conformations of the decasaccharides in green for the first corresponding linkage and in red for the second.

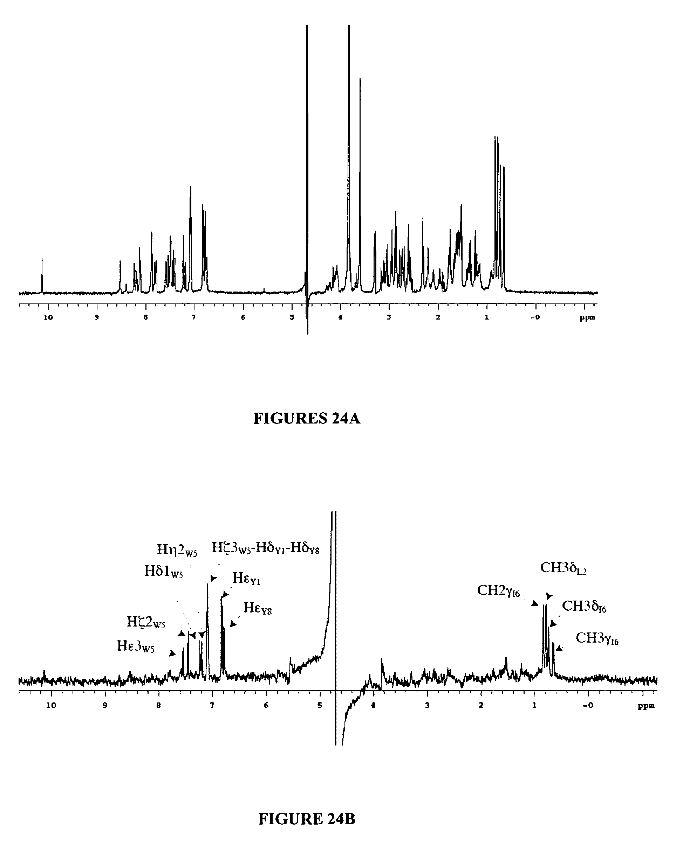

**Figures 24A-24B.** mAb binding epitope of the peptide B1.

Figure 24A: 1D 1H referencespectrum of peptide B1.

Figure 24B: 1DSTD-NMR of B1 in the presence of mIgG F22-4 with selective saturation of antibody resonances at -0.2 ppm. Protons of B1 affected by the selective saturation of mIgG F22-4 and so in contact with the mAb are labeled.

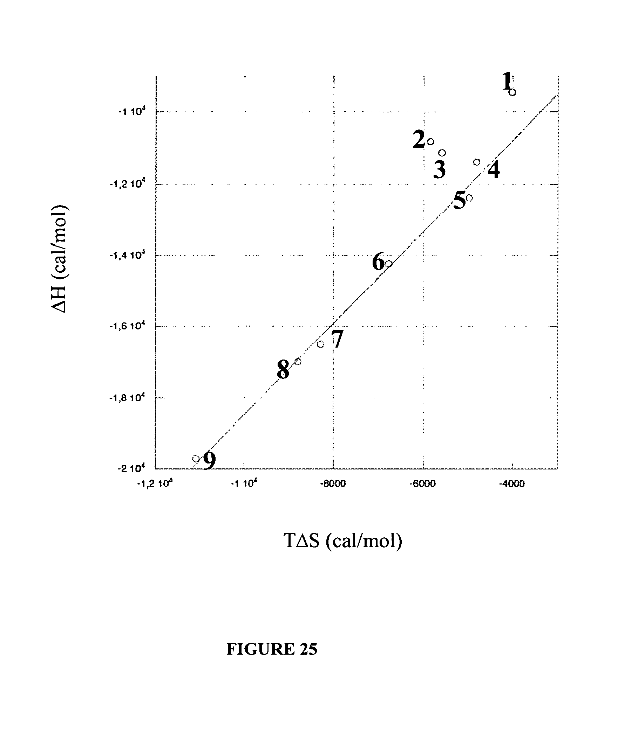

**Figure 25.** Enthalpy-entropy plot of the interactions between F22-4 and the following oligosaccharides: 1) ECD; 2) ECDA'B'; 3) ECDA'; 4) AB(E)CD; 5) B(E)CD; 6) B(E)CDA'; 7) [AB(E)CD]$_3$; 8) [AB(E)CD]$_2$; 9) B(E)CDA'B'(E')C'.

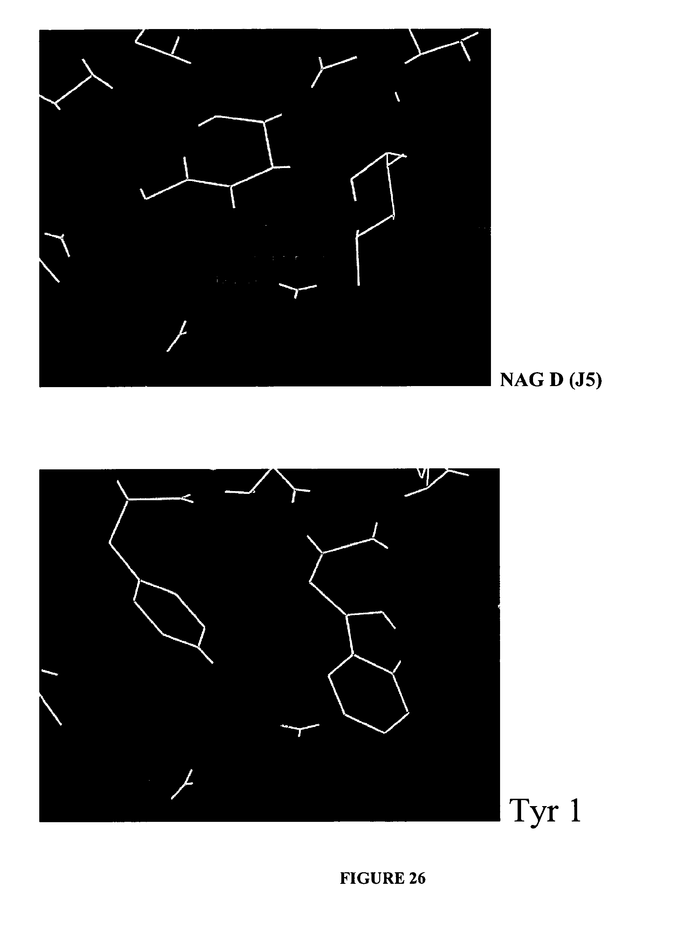

**Figure 26.** Crystallographic results: comparison of the interactions established between the *N*-acetylglucosamine (NAG(D)) from the oligosaccharide and the Fab F22-4, and in addition between the aminoacid tyrosine 1 (Tyr1) of the peptide B1 and the Fab F22-4.

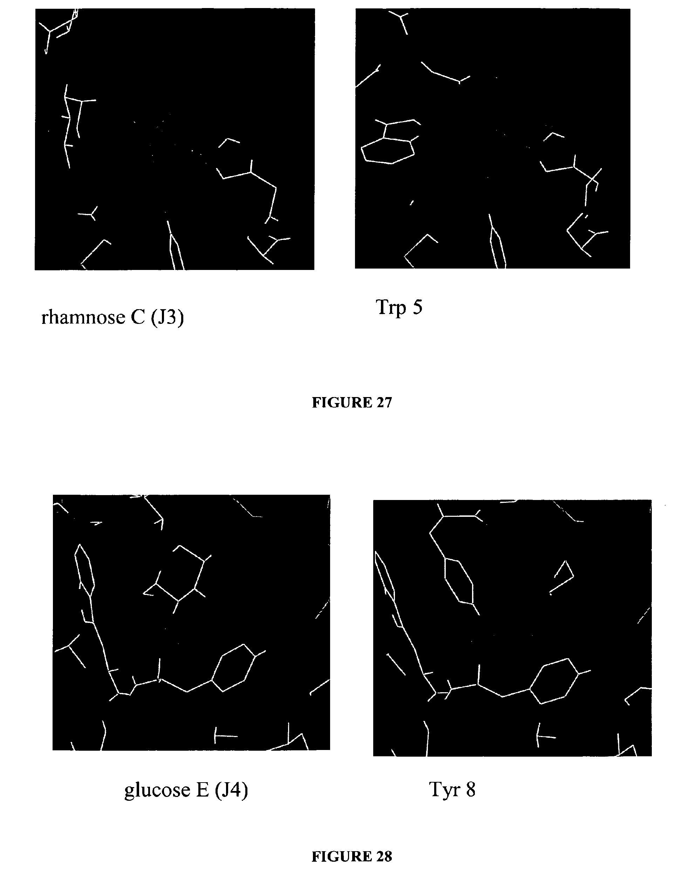

**Figure 27.** Crystallographic results: comparison of the interactions established, through water molecules, between the rhamnose C from the oligosaccharide and the Fab F22-4, and in addition between the aminoacid tryptophane 5 (Trp5) of the peptide B1 and the Fab F22-4.

**Figure 28.** Crystallographic results: comparison of the interactions established between the glucose E from the oligosaccharide and the Fab F22-4, and in addition between the aminoacid tyrosine 8 (Tyr8) of the B1 and the Fab F22-4.

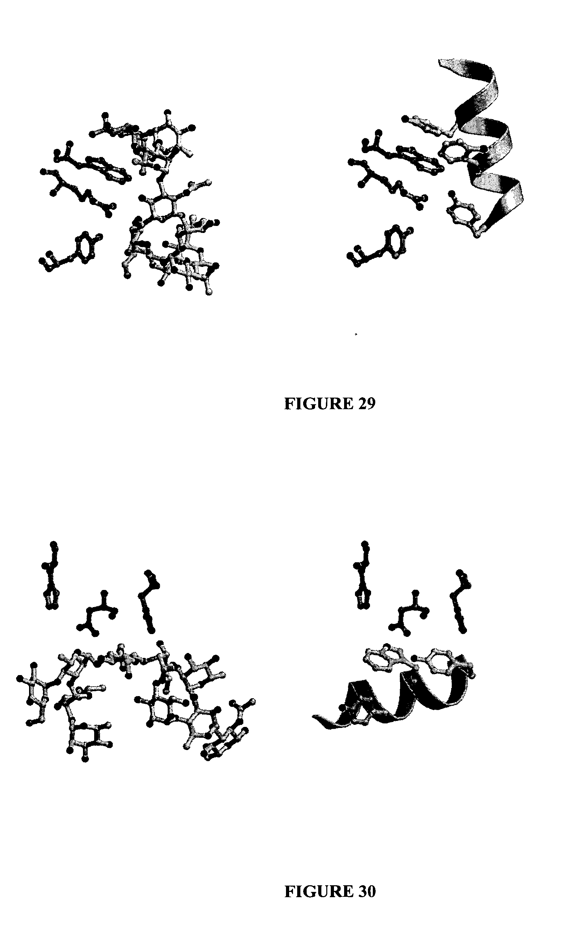

**Figure 29.** Crystallographic results: scheme of the interactions established between the heavy chain of the Fab F22-4 (in blue) and the oligosaccharide (left) and its peptidic mimotope (right).

**Figure 30.** Crystaallographic results: scheme of the interactions established between the light chain of the Fab F22-4 (in green) and the oligosaccharide (left) and its peptidic mimotope (right).

**EXAMPLE 1: Structures of synthetic O-antigen fragments from serotype 2a *S. flexneri* in complex with a protective monoclonal antibody**

**Determination of the crystal structure of the Fab fragment of IgG F22-4 in complex with the synthetic decasaccharide and pentadecasaccharide ligands, [AB(E)CD]$_2$ and [AB(E)CD]$_3$ (11)**

The structures show that both ligands are bound in an identical way: six carbohydrate residues, contained within a contiguous nonasaccharide segment, make direct contacts with F22-4. These results are compared with other antibody-carbohydrate structures and are discussed in the light of antigenic and immunogenic mimicry of *S. flexneri* 2a O-Ag by synthetic oligosaccharides that we have previously reported (12), (13).

**Results**

**General description of the Fab F22-4 structures**

The Fab fragment of F22-4 was crystallised in the non-liganded form, and in complex with the amino-ethyl derivatives of the decasaccharide [AB(E)CD]$_2$ (9) and the pentadecasaccharide [AB(E)CD]$_3$ (10), respectively. Crystals of Fab F22-4 crystals are triclinic with two independent molecules in the unit cell. The crystals of both oligosaccharide complexes are monoclinic with very similar unit cell dimensions and containing four complexes in the asymmetric unit; however, they are not strictly isomorphous to each other because of differences in the Fab elbow angles between the two crystal forms.

The complete [AB(E)CD]$_2$ ligand is visible in the electron density maps for all four independent complexes; however, only eleven residues could be traced in each of the four independent [AB(E)CD]$_3$ complexes. Interactions of [AB(E)CD]$_2$ and [AB(E)CD]$_3$ with F22-4 are essentially identical, covering two consecutive RUs (designated here as AB (E)CDA'B'(E')C'D'). For [AB(E)CD]$_3$, binding modes could conceivably involve the first and second RUs, the second and third, or indeed a mixture of these two possibilities. Examination of the electron density maps, however, indicates that only the first mode is used; although density for the eleventh residue, Rha(A"), (following GlcNAc(D')), is present, there is no density preceding the first visible rhamnose (Figure S1). Modelling confirms these observations because it shows that extension of the oligosaccharide by addition of GlcNAc(D°) to the non-reducing end of the first built rhamnose leads either to steric hindrance with the antibody or to unfavourable conformations for the D°A linkage.

**Antigen-binding site**

The antigen-binding site is groove shaped, with approximate dimensions of 20 Å long, 15 Å wide and 8 Å deep (Figure 2). It is formed by CDR-L1 on one side of the groove, and CDR-H1 and CDR-H2 on the other, with CDR-L3 and CDR-H3 at the floor of the binding site. An unusual feature of CDR-H3, apart from its short length (4 residues), is a cis peptide bond between Pro-H95 and Met-H96, present in both the liganded and free forms of F22-4. Although the F22-4 structure was determined in both the free and antigen-bound state, the presence of significant intermolecular contacts at the binding site of both non-complexed Fab fragments limits conclusions on changes in conformation and solvation upon complex formation.

**The structure of the carbohydrates**

The mean pair-wise r.m.s. difference in atomic positions of the four independent saccharides is 0.18 Å for [AB(E)CD]$_2$ and 0.43 Å for [AB(E)CD]$_3$; a pair-wise comparison between [AB(E)CD]$_2$ and [AB(E)CD]$_3$ gives a mean r.m.s. difference of 0.30 Å. Thus, [AB(E)CD]$_2$ is very similar in all four independent complexes; the moderately larger r.m.s. differences for [AB(E)CD]$_3$ essentially reflect the larger deviations for residues GlcNAc(D') and Rha(A") at the reducing end, which make no contacts with the Fab. The lack of electron density beyond residue Rha(A") of the third RU can be attributed to the lack of stabilising intermolecular contacts in this region of [AB(E)CD]$_3$ and to its intrinsic flexibility.
The two sets of backbone dihedral angles ($\phi,\varphi$) of the two RUs (ABCD and A'B'C'D') are similar to each other in both the [AB(E)CD]$_2$ and [AB(E)CD]$_3$ complexes (Table 1, Figure S2). ABCD superimposes on A'B'C'D' with a r.m.s. difference of 0.44 Å (mean value for the four independent ligands) in all backbone atom positions for [AB(E)CD]$_2$, and 0.53 Å for [AB(E)CD]$_3$. Moreover, the dihedral angles at the AB, BC and CD linkages fall into theoretical energy minima based on disaccharide models $\alpha$-L-Rha$p$-(1→2)-$\alpha$-L-Rha$p$, $\alpha$-L-Rha$p$-(1→3)-$\alpha$-L-Rha$p$ and $\alpha$-L-Rha$p$-(1→3)-$\beta$-D-Glc$p$-NAc) (Figure S3) (14). By contrast, the DA' linkage falls in a less favourable, although allowed, region of the theoretical $\beta$-D-Glc$p$NAc-(1→2)-$\alpha$-L-Rha$p$ disaccharide energy diagram. This could be due to limitations of the disaccharide model, which includes neither the influence of antibody contacts, nor a bifurcated intramolecular hydrogen bond from O5$_D$ to O3$_E$ and O4$_{E'}$, nor the many solvent molecules that form intra-oligosaccharide bridging interactions (Figure 2). The dihedral angles of the two branching $\alpha$-D-Glc$p$-(1→4)-$\alpha$-L-Rha$p$ linkages (EC and E'C') differ widely from each other. They are not close to minima in the theoretical disaccharide energy diagram, probably reflecting the important influence of the Fab on the conformation of these branching linkages (see below). Interestingly, if Glc(E') is modelled with the same dihedral conformation as Glc(E), an internal hydrogen bond is also formed to GlcNAc(D) (via O6$_D$ and O6$_E$ in this case).

**Table 1.** Saccharide ($\phi$, $\varphi$) dihedral angles (IUPAC convention; http://www.chem.qmul.ac.uk/iupac/misc/psac.html). Mean values from the four crystallographically independent molecules is given for the 2a serotype [AB(E)CD]$_2$ and [AB(E)CD]$_3$ complexes, and for the Y serotype pentasaccharide (ABCDA') complex with mAb SYA/J6 (PDB entry 1m7i).

| | [AB(E)CD]$_2$ (2a) | [AB(E)CD]$_3$ (2a) | [ABCDA'] (Y) |
|---|---|---|---|
| Rha(A)-Rha(B) | -86°, -167° | -91°, -166° | -64°, -92° |
| Rha(B)-Rha(C) | -78°, -128° | -75°, -127° | -82°, -116° |
| Rha(C)-GlcNAc(D) | -92°, 134° | -89°, 132° | -115°, 145° |
| GlcNAc(D)-Rha(A') | -67°, -139° | -66°, -138° | -90°, 177° |
| Glc(E)-Rha(C) | 146°, 147° | 145°, 151° | |
| Rha(A')-Rha(B') | -80°, -146° | -81°, -149° | |
| Rha(B')-Rha(C') | -70°, -129° | -74°, -127° | |
| Rha(C')-GlcNAc(D') | -85°, 126° | -90°, 144° | |
| Glc(E')-Rha(C') | 56°, 95° | 49°, 95° | |
| GlcNAc(D')-Rha(A") | | -81°, -126° | |

**Antibody-carbohydrate interactions**

The epitope is contained within two consecutive RUs, requiring the nonasaccharide **AB(E)CDA'B'(E')C'** to present the complete antigenic determinant (sugar residues in contact with F22-4 shown in bold). All hypervariable regions except CDR-L2 contact the antigen. Eleven hydrogen bonds are formed directly between the Fab and [AB(E)CD]$_2$ (Table 2). Fourteen water molecules, common to the four independent complexes, bridge between the two components (Figure 2B). The total buried accessible surface area at the antibody-antigen interface is 1125 Å$^2$ (calculated using a solvent probe radius of 1.4 Å with the program AREAIMOL (15)). The bridging water molecules make an important contribution to complementarity at the interface since the surface complementarity index (calculated with the program SC (15)) increases from 0.65 to 0.75 when included in the calculation.

**Table 2.** Hydrogen bond interactions between F22-4 and oligosaccharide

| Saccharide | | F22-4 |
|---|---|---|
| Rha(C) | O5 | Asn L91 Nδ2 |
| GlcNAc(D) | O4 | Asn L91 Oδ1 |
| | | Arg L96 Nζ1 |
| | | Arg L96 Nζ2 |
| GlcNAc(D) | O6 | Arg H52 Nζ2 |
| Glc(E) | O3 | Pro H95 O |
| Glc(E) | O4 | Trp H33 N |
| Glc(E) | O6 | Asn H31 O |
| Rha(A') | O3 | His L27DNε2 |
| Glc(E') | O3 | Arg H52 Nζ1 |
| Glc(E') | O5 | Tyr H58 OH |

Table 3 summarises F22-4 interactions with individual oligosaccharides residues. GlcNAc(D) and the branching residue Glc(E) make the most important contributions. These two residues are the most buried, being located in small cavities in the groove-shaped binding site. The two cavities are formed largely by a narrowing of the groove by residues Asn-L91, Arg-L96 and Trp-H33 located at the centre of the binding site. Rha(C) and Glc(E') make intermediate contributions, whereas those of Rha(A), Rha(A') and Rha(B') are weak. In the case of Rha(B'), the only contacts are those mediated by bridging water molecules. Rha(B), Rha(C') and GlcNAc(D') make no contacts at all with the antibody.

**Table 3.** Summary of the interactions per saccharide residue. The number of inter-atomic contacts (< 3.8 Å) between the antibody and oligosaccharide as well as the number of these that are hydrogen bonds are given in the first and second lines, respectively. The numbers of water molecules bridging between the antibody and oligosaccharide are given in the third line. The buried surface area of each saccharide residue at the antibody/antigen interface is calculated using a spherical probe of 1.4 Å radius.

| Residues | A | B | C | D | E | A' | B' | C' | D' | E' |
|---|---|---|---|---|---|---|---|---|---|---|
| Contacts | 2 | - | 7 | 14 | 13 | 3 | - | - | - | 7 |
| H bonds | - | - | 1 | 4 | 3 | 1 | - | - | - | 2 |
| Water bridges | 2 | - | 2 | 1 | 4 | 2 | 3 | - | - | 1 |
| Buried surface | 68 | 1 | 80 | 161 | 148 | 35 | 26 | 2 | 0 | 86 |

## Discussion

### Comparison with other antibody-carbohydrate complexes

Early work on anti-polysaccharide antibodies suggested that carbohydrate epitopes could vary from one to about seven residues, the upper limit being determined by the surface available at the antigen-binding site (16). It was concluded that small epitopes generally include the non-reducing terminal residue whereas larger epitopes are located along the length of the polysaccharide chain (17). It was further suggested that small epitopes bind to cavity-type antigen binding-site topologies while the larger internal epitopes are recognized by groove-type topologies (18). Although few bacterial oligosaccharide-antibody crystal structures have been reported to date (19), (20), (21), these early estimates of epitope size and nature, and their relationship to binding-site topology, are largely concordant with current structural data. For example, the crystal structure of mAb S-20-4, raised against the O-Ag of *Vibrio cholerae* O1 serotype Ogawa, shows that a disaccharide, corresponding to the non-reducing terminus of the cognate antigen, binds to a cavity-shaped binding site (20). The structure suggests that the epitope recognized by S-20-4 comprises essentially two sugar rings at the terminus of the cognate polysaccharide antigen. By contrast, mAb SYA/J6, raised against *S. flexneri* serotype Y O-Ag (where the RU is ABCD), binds an extended pentasaccharide in a groove-shaped binding site (21). An upper limit of about seven sugar units in the antigen-binding site is also largely substantiated by recent structural studies. Antibody Se155-4, which recognizes the *Salmonella* serotype B O-antigen, has been crystallized with an octasaccharide (22) and dodecasaccharide (19); the octasaccharide complex shows that six of the seven ordered residues make direct contact with Se155-4. However, the minimum oligosaccharide length may require more sugar units in some cases to provide the optimal number of antibody-contacting residues. With a helical polysaccharide chain, for example, the antibody-contacting residues would not form a contiguous segment because some sugar units would be solvent-exposed, as suggested from modelling studies by Evans *et al.* (23).

The epitope in the F22-4-oligosaccharide complexes is included within a contiguous nine-residue section in which six sugar rings make direct contacts with the antibody. Thus, a more compact oligosaccharide conformation - in this case helical - presents a longer segment that still conforms to the approximate seven-residue limit at the antigen-binding site. As might be anticipated, the binding site is groove-shaped to accept an epitope of this size, but this topology is modulated by two cavities. The larger cavity accommodates the branching Glc(E) residue. A similar situation occurs in the structure of the anti-*Salmonella* O-polysacchharide mAb Se115-4 complex (19) where the branching abequose ring of the antigen binds in a cavity located centrally in the binding site. A branching residue along a polysaccharide chain can therefore behave as a non-reducing terminus. The second (smaller) cavity of F22-4, by contrast, binds the backbone residue GlcNAc(D) and thus resembles the SYA/J6 complex where Rha(C) of the serotype Y ligand ABCDA' is also buried in a cavity at the centre of the groove-shaped binding site (21).

Comparison between F22-4 and SYA/J6 merits further discussion because of similarity both with respect the cognate antigen and the $V_H$ sequence. SYA/J6 was raised against *S. flexneri* serotype Y O-Ag, which lacks a branching residue. $V_H$ of SYA/J6 uses the same germline $V_H$ gene and $J_H$ minigene as F22-4, the major difference being in the length of CDR-H3 (9 residues in SYA/J6, 4 in F22-4) (Table 5). The complete SYA/J6 $V_L$ domain, however, originates from different $V_L$ and $J_\kappa$ genes and shows 28 differences over 112 residues with respect to F22-4. The binding site of F22-4 is shallower than that of SYA/J6, mainly because a salt bridge is formed between Arg-L96 and Glu-H50 in F22-4 (Arg-L96 is deleted from the SYA/J6 $V_L$ germline sequence). Consequently, the serotype Y ABCDA' segment sits lower in the antibody framework than does the serotype 2a antigen. The short CDR-H3 of F22-4 contributes to a deep pocket for the Glc(E) residue where many polar interactions between antibody and oligosaccharide are mediated by buried solvent molecules. Indeed, the highly solvated antibody/antigen interface of F22-4 contrasts with the absence of bridging water molecules in the SYA/J6 complex. Although the serotype Y lacks the branching glucose, the two antigen serotypes are remarkably close in conformation for the segment BCD. These superimpose with an r.m.s. difference of 0.73 Å in all atom positions, reflecting the similarities in the $(\phi,\varphi)$ dihedral angles of the BC and CD glycosidic linkages (Table 1).

**Influence of glucosylation on O-antigen conformation**

Addition of the $\alpha$-D-Glc*p*(E)-(1→4)-$\alpha$-L-Rha*p*(C) serotype 2a conformation observed in the F22-4 complex to the ABCDA' serotype Y structure of the SYA/J6 complex shows that Glc(E) would be sterically hindered by Rha(A), thereby forcing the AB $\varphi$ dihedral angle to more negative values, as observed in the F22-4 complex (Table 1). The AB glycosidic linkages in both the F22-4 and SYA/J6 complexes are indeed in energy minima in the $(\phi,\varphi)$ plot (Figure 3), showing that both observed AB conformations in the two serotypes are favourable. Similarly, the $\alpha$-D-Glc*p*(E)-(1→3)-$\alpha$-L-Rha*p*(B) linkage of the 5a serotype is not sterically compatible with the Y serotype structure, as has been previously noted (24). The pattern of serotype glucosylation can therefore impose constraints on the structure of extended O-Ag chains.

**Implications for O-antigen structure in LPS**

By iterative superposition of ABCD onto A'B'C'D' using the crystallographic [AB(E)CD]$_2$ coordinates, we could generate a model for an extended serotype 2a O-Ag chain that was devoid of unfavourable steric interactions. This model comprises RUs with the crystallographic glycosidic $(\phi,\varphi)$ angles of A'B'(E')C'D' moiety and the DA' linkage; no energy optimization was attempted in order to preserve the experimentally determined linkage conformations (Figure 3). (An almost identical model was generated when the AB(E)CD conformation was used in this procedure.) The model polysaccharide chain thus obtained is a right-handed helix of pitch ~23 Å, diameter ~15 Å and nearly three RUs per turn. Curiously, the helix has similar parameters to the model proposed for serotype 5a O-Ag, which was based on N.M.R. data and modelling studies (24). But unlike the serotype 5a model, where Glc(E) protrudes outwards perpendicular to the helical axis in a solvent-exposed orientation, Glc(E) in the serotype 2a is folded under the backbone at about the same radial distance from the helical axis as the residues ABCD. This is true for both the EC and E'C' conformations present in the crystal structure. Glc(E) is therefore less accessible than in the serotype 5a model where linkage to the ABCD base differs ($\alpha$-D-Glc*p*(E)-(1→3)-$\alpha$-L-Rha*p*(B)). If this hypothetical structure does indeed exist in 2a serotype O-Ag, it would probably represent a relatively transient short-range ordering because intrinsic mobility is indicated by the absence of electron density for all but the first residue of the third RU in the [AB(E)CD]$_3$ complex. Conformational flexibility is expected because, apart from the hydrogen bond formed between GlcNAc(D) and Glc(E'), intra-molecular contacts between carbohydrate residues are almost exclusively mediated by bridging solvent molecules. Nonetheless, it represents a plausible average structure because the backbone conformation within the ABCD motif is conserved between the two RUs in the crystal structure and their glycosidic dihedral angles are in favourable conformations.

**Correlation of crystal structure with oligosaccharide binding studies**

The network F22-4/oligosaccharide interactions revealed by the crystal structures is largely concordant with the LPS/F22-4 inhibition studies using 24 synthetic *S. flexneri* 2a di- to pentadeca-saccharides (12), (13). ECD, the smallest synthetic segment of the serotype 2a O-Ag that gave measurable competitive binding to F22-4, corresponds to the contiguous trisaccharide segment making the most significant interactions with F22-4 (Table 3). The pentasaccharide AB(E)CD was about an order of magnitude more effective in inhibition than ECD whereas the octasaccharide B(E)CDA'B'(C')D', which represents essentially the entire structural epitope, was about three orders of magnitude more effective. Oligosaccharides that included neither GlcNAc(D) nor Glc(E) showed no measurable binding, consistent with the key contribution of these residues to antibody-antigen interactions.

We have argued from the crystal structures that the addition of GlcNAc($D^0$) to the non-reducing end of the bound oligosaccharide would lead either to steric hindrance with F22-4 or to unfavourable $D^0$A glycosidic conformations. Nonetheless, the IC50 of $D^0$AB(E)CDA'B'(E')C' is only an order of magnitude higher than that of B(E)CDA'B'(E')C' (12), suggesting that steric hindrance between $D^0$ and F22-4 could be relieved by conformational changes, most probably in both the AB and BC glycosidic bonds. Thus, while the crystal structures imply that inclusion of the non-reducing terminus of LPS in the epitope gives a more favourable interaction with F22-4, the binding data suggest that recognition of internal epitopes along the O-Ag chain also occurs, but probably with reduced affinity.

**Structural implications for vaccine development**

An important parameter in oligosaccharide-based vaccine development is the minimum hapten length required to give optimal immune protection. In some cases, disaccharide- or trisaccharide-protein conjugates are able to induce a protective antipolysaccharide response in animal models, showing that for certain bacterial polysaccharide antigens, synthetic oligosaccharides shorter than one RU can be good immunogenic mimics (25), (26). For other polysaccharide antigens, however, several RUs may be required to obtain immunogenic mimicry (27); here, it has been suggested that the corresponding epitopes might be conformational in certain cases (28). Our previous studies have suggested that a correlation exists between the synthetic oligosaccharide length and immunogenic mimicry of serotype 2a LPS (12), (13). Although antibodies induced by a glycoconjugate bearing one synthetic RU showed only weak reactivity with bacterial LPS, antibodies induced by two RUs showed medium reactivity, whereas those induced by three RUs were highly reactive. Accordingly, the dependence of immunogenic mimicry on RU number suggests that immunodominant serotype 2a epitopes might also be conformational, requiring longer segments for the presentation of more stable structures such as the helical form we have proposed. This possibility, however, needs further examination as other factors could account for our observations.

Characterization of antigenic determinants is equally important for vaccine development. Our results provide a structural perspective for understanding the serotype 2a specificity of F22-4, revealing a significant contribution to the epitope from the branching glucosyl residues of two consecutive RUs. A minimum segment of nine consecutive sugar units is required to present the complete epitope recognized by F22-4. Accordingly, glycoconjugates should carry a minimum of two serotype 2a RUs to achieve accurate antigenic mimicry of the O-Ag. The conformation of the antigen, as seen in the crystal structures, is strongly influenced by the position of the branching sugars on the backbone, underlining the need to determine the extent of restraints on the conformational variability in synthetic oligosaccharides and to clarify its impact on antigenic mimicry. To this end, there is an interest in co-crystallizing oligosaccharides with the other anti-serotype 2a mAbs that recognize different epitopes to extend our knowledge on O-Ag conformation and its consequences for immune recognition. Detailed structural analysis of protecting epitopes on the O-Ag chain, including N.M.R. and dynamics studies of the oligosaccharides, can open new perspectives for vaccine optimization.

**Materials and methods**

**Crystallisation and data collection**

Fab F22-4 was prepared from purified IgG by papain digestion. Crystallizations were performed using the hanging drop technique. Crystals of the free Fab were grown at 17°C from the buffer Clear Strategy Screen solution no. I-3 (Molecular Dimensions) mixed with 5% 1M sodium acetate at pH 4.5. The final protein concentration was 4.1 mg/ml. Crystals of the Fab complexes were obtained by co-crystallizing with the aminoethyl derivatives of [AB(E)CD]$_2$ and [AB(E)CD]$_3$ at molar excesses of 1:10 and 1:16, respectively, using the buffer JBScreen Classic 4-A1 (Jena Bioscience). Final protein concentrations were 4.0 and 3.1 mg/ml for the [AB(E)CD]$_2$ and [AB(E)CD]$_3$ complexes, respectively. Dif-

fraction data were collected at the European Synchrotron Radiation Facility, Grenoble. The data were processed using the programs *XDS* (29) and the CCP4 program suite (15). Details of the data collection and statistics are given in Table 4.

**Table 4.** Crystal parameters and data collection statistics

| | free Fab | decasaccaride complex | pentadecasaccaride complex |
|---|---|---|---|
| space group | P1 | P2$_1$ | P2$_1$ |
| unit cell | | | |
| a, b, c (Å) | 47.2, 59.8, 74.4 | 67.1, 137.6, 109.8 | 66.9, 137.2, 109.4 |
| $\alpha$, $\beta$, $\gamma$.° | 77.5, 84.0, 80.9 | 90,95.2,90 | 90,95.0,90 |
| Z (molecules/unit cell) | 2 | 4 | 4 |
| $V_M$ (A$^3$ Da$^{-1}$) | 2.1 | 2.6 | 2.6 |
| beamline (ESRF) | ID14-1 | ID14-2 | ID14-2 |
| wavelength (Å) | 0.934 | 0.933 | 0.933 |
| resolution range (Å) | 72 - 2.0 | 48 - 1.8 | 46 - 1.8 |
| last shell (Å) | 2.1 - 2.0 | 1.9 - 1.8 | 1.9 - 1.8 |
| no. unique reflections | 50673 (7292) | 182390 (26443) | 179612 (25673) |
| redundancy | 3.8 (3.3) | 3.8 (3.7) | 3.7 (3.5) |
| $R_{symm}$ | 0.068 (0.144) | 0.105 (0.622) | 0.112 (0.668) |
| completeness (%) | 95.8 (94.8) | 99.7 (99.4) | 99.2 (97.5) |
| $<I/\sigma(I)>$ | 13.6 (7.0) | 9.8 (2.3) | 10.2 (2.0) |

**Structure solution and refinement**

Initial models of the free Fab and the two complexes were obtained by molecular replacement using the programs *AMoRe* (30) and *Phaser* (31). Known antibody structures were used as search models. The structures were refined using the programs *REFMAC* (32) and *ARP/warp* (33). The oligosaccharides in the complexes were built during the early stages of the refinement. Refinement statistics are given in Table 5. Figures of molecular structures were made with MacPyMol (www.pymol.org).

**Table 5.** Refinement statistics

| | free Fab | decasaccharide complex | pentadecasaccharide complex |
|---|---|---|---|
| resolution (Å) | 72 - 2.0 | 48 - 1.8 | 45 - 1.8 |
| last shell (Å) | 2.03 - 2.00 | 1.82 - 1.80 | 1.82 - 1.80 |
| R-value (working set) | 0.199 (0.246) | 0.176 (0.257) | 0.191 (0.281) |
| $R_{free}$ | 0.260 (0.411) | 0.230 (0.278) | 0.251 (0.354) |
| no. reflections (total) | 50671 (1737) | 182354 (6631) | 176903 (6431) |
| no. reflections for $R_{free}$ | 1307 (35) | 2708 (91) | 2670 (97) |
| no. protein atoms | 6482 | 13193 | 13150 |
| no. carbohydrate atoms | - | 447 | 479 |
| no. solvent atoms | 400 | 2474 | 2123 |
| r.m.s. deviation from ideal | | | |
| bond lengths (Å) | 0.013 | 0.012 | 0.014 |
| bond angles (°) | 1.5 | 1.5 | 1.6 |

**Data deposition:** Atomic coordinates and structure factors have been deposited in the Protein Data Bank (www.pdb.org) with for the free Fab, for the [AB(E)CD]$_2$ complex and for the [AB(E)CD]$_3$ complex.

**Abbreviations:** RU, repeat unit; O-Ag, O-antigen; LPS, Lipopolysaccharide; mAb, monoclonal antibody; $V_H$, heavy chain variable domain; $V_L$, light chain variable domain; CDR, complementarity-determining region; CDR-L1, first CDR of the light chain variable domain, etc.

**Supporting Information**

**Preparation of the Fab, crystallisation and data collection**

Production and purification of F22-4 has been described elsewhere (12). Briefly, F22-4 mIgG was precipitated with 50% ammonium sulfate from ascitic fluid, and then centrifuged and dialyzed against PBS before being purified using ion-exchange chromatography. F22-4 was diluted to 8mg/ml in 0.1M potassium phosphate buffer at pH 7.2. After addition of mercaptoethanol and EDTA to final concentrations of 14 and 12mM, respectively, the protein was treated with papain (Boehringer) at an enzyme:substrate ratio (w:w) of 1:40 for 6 hours at 37°C. After incubating for 1h at room temperature with 20mM iodoacetamide, the buffer was changed to 0.02M potassium phosphate buffer, pH 7.8, and the protein was applied to a DEAE-Sephacel (Pharmacia) support in batch, equilibrated in the same buffer. After incubation, Fab F22-4 was recovered in the non-binding fraction. Further purification on a Mono Q column led to several crystallizable species corresponding to different isoforms of the Fab fragment.

Crystals of the Fab were grown by vapour diffusion using the hanging drop technique. A volume of 1 $\mu$l of protein was mixed with 1 $\mu$l of buffer containing 24% (w/v) PEG 2000 monomethylether, 190 mM $MgCl_2$ and 50mM NaAcetate, pH 4.5 (Clear Strategy Screen solution I-3, Molecular Dimensions). The final protein concentration was 4.1 mg/ml. The drop was sealed over a reservoir containing 800 $\mu$l of buffer covered by 100 $\mu$l Al's Oil (Hampton Research), and left at 17°C. Crystals appeared after three weeks. Crystals were flash-frozen in liquid nitrogen after a brief soaking in a cryo-protecting buffer consisting of the crystallization buffer with 10% glycerol (v/v).

Crystals of the Fab fragment in complex were obtained with the aminoethyl derivatives of $[AB(E)CD]_2$ and $[AB(E)CD]_3$ representing two and three RUs of O-Ag serotype 2a, respectively. The Fab fragment and the oligosaccharide were incubated for 4 hours at 37°C, in a 1:10 and 1:16 molar ratio for $[AB(E)CD]_2$ and $[AB(E)CD]_3$, respectively. A volume of 0.7 $\mu$l of the mixture was added to 0.7 $\mu$l of buffer containing 25% (w/v) PEG 5000 monomethylether, 200 mM $Li_2SO_4$ and 100mM Tris/HCl, pH 8.6 (JBScreen Classic 4-A1, Jena Bioscience). Hanging drops were set up over a reservoir containing 700 $\mu$l of buffer and stored at 17°C. The final protein concentration was 4.0 and 3.1 mg/ml for the $[AB(E)CD]_2$ and $[AB(E)CD]_3$ complexes, respectively. Crystals appeared within a few days. Crystals were frozen in liquid $N_2$ after brief soaking in a cryoprotectant consisting of the crystallization buffer with 15 % glycerol (v/v).

Diffraction data were collected on the beamlines ID 14-1 and ID 14-2 at the European Synchrotron Radiation Facility, Grenoble (Table 4). The data were integrated and processed using the programs *XDS* (34) and the CCP4 program suite (15).

**Structure solution and refinement**

An initial model for the non-complexed Fab F22-4 was obtained by molecular replacement using the program *AMoRe* (4). The search model was divided into two independent components: the $V_H V_L$ variable domain dimer and the $C_H C_L$ constant domain dimer. The $V_H V_L$ dimer was a hybrid model comprising $V_H$ from Fab 1H-98H (PDB entry 1A4J, 75% sequence identity to F22-4) and $V_L$ from Fab 4C4 (pdb entry 1EJO, 89% sequence identity to $V_L$ of F22-4). The $C_H C_L$ dimer model was from Fab F124 (pdb entry 1F11). Rotation function calculations were carried out using all data in the 10 - 3 Å resolution range and with an integration radius of 25 Å. Translation function calculations using data within the same resolution limits were made using single-body searches to place individual variable and constant domain dimers. Multiple-body searches were then made to refer each component to a common crystallographic origin. The final result matched the variable and constant dimers to give correct connectivity of the light and heavy polypeptide chains within each independent Fab molecule and no steric hindrance between molecules in the crystal lattice. Rigid body refinements of the variable and constant domains were performed to provide an initial model for refinement of the atomic parameters. The oligosaccharide complexes were solved by molecular replacement using the refined structure of the non-complexed Fab F22-4 and the program *Phaser* (31).

All F22-4 crystal structures were refined using the programs *REFMAC* (32) and *ARP/warp* (33), with manual adjustments to the atomic models being made with the molecular graphics program O (35). The non-complexed Fab was the first structure of the series to be refined. The CDR residues and sequence of F22-4 were built into the molecular replacement model during the refinement by reference to electron density maps. In the case of the complexes, the oligosaccharides were built during the early stages of the refinement. The CDR-H1 residues between Leu-H27 and Phe-H29, inclusive, and CDR-H2 residues, Leu-H52A and Ala-H56, inclusive, could not be traced in the electron density maps for one of the Fabs in the free form; otherwise, all variable domain residues were well defined in the remaining Fab molecules, whether free or complexed. Refinement statistics are shown in Table 5.

**Characteristics of mAb F22-4**

The mAb F22-4 (isotype IgG1, $\kappa$), produced by immunisation of BALB/c mice with inactivated *S. flexneri* serotype 2a bacteria, shows no cross-reactivity with other serotypes (1). Nucleotide sequence of the $V_H$ and $V_L$ domains was determined from the cDNA produced from the total RNA extract of F22-4 hybridoma cells (GeneBank entries AJ784030 and AJ784031, respectively). The germ-line sequences corresponding to the F22-4 variable domains are closest to IGHV6-6 and JH4 for the $V_H$ domain (the *D* mini-gene could not be deduced), and IGKV2 and KJ1 for the $V_L$ domain (gene nomenclature according to the IMGT database, (36, 37). The following non-synonymous mutations with respect to the putative *V* germ-line sequences are: Leu->Val-H4, Leu->Ile-H20 (FR-H1), Ala->Val-H24 (FR-H1), Phe->Leu-H27 (FR-H1), Asn->Ser-H35 (CDR-H1), Asn->Asp-H53 (CDR-H2), His->Tyr-H58 (CDR-H2), Arg->Lys-H66 (FR-H3), Ser->Arg-H77 (FR-H3), Val->Leu-H78 (FR-H3), Ala->Thr-H84 (FR-H3) for the $V_H$ domain, and Asn->Asp-L28 (CDR-L1), Gln->His-L45 (FR-L2), Gln->His-L50 (CDR-L2), Met->Leu-L51 (CDR-L2), Val->Ile-L85 (FR-L3), Gln->His-L90 (CDR-L3) and Leu->Val-L92 (CDR-L3) for the $V_L$ domain. The complementarity-determining regions can be assigned to the following canonical conformations: CDR-H1: class 1 (10 residues), CDR-H2: class 4 (12 residues), CDR-L1: class 4 (16 residues), CDR-L2: class 1 (7 residues) and CDR-L3: class 1 (9 residues) (CDR residue length and canonical class according to (38). The CDR-H3, comprising only four residues, is significantly shorter than the average length of 8.7 residues found for murine antibodies (39).

### Consequences of O-acylation on F22-4 recognition

O-acetylation of native 2a serotype O-antigen occurs on 03 of Rha(A) (30-50% depending on LPS preparations) and O6 on GlcNAc(D) (30-60%) (40). Acetylation of Rha(A) or Rha(A') can be modelled into the Fab F22-4 complex without engendering steric hindrance. On the other hand, while 06 acetylation of GlcNAc(D') should not affect F22-4 recognition, this is not the case for GlcNAc(D) since there is no free volume to accommodate this modification at the antibody-antigen interface. Nonetheless, O-acetylation does not compromise the protective efficacy of this mAb in the animal model system for shigellosis, which suggests that F22-4 was induced by non-acetylated regions of native 2a serotype LPS.

### Conclusion

The crystal structures of complexes formed between Fab F22-4 and two synthetic oligosaccharides, a decasaccharide and a pentadecasaccharide that were previously shown to be both immunogenic and antigenic mimics of the *S. flexneri* serotype 2a O-antigen. F22-4 binds to an epitope contained within two consecutive 2a serotype pentasaccharide repeat units (RU). Six sugar residues from a contiguous nine-residue segment make direct contacts with the antibody, including the non-reducing rhamnose and both branching glucosyl residues from the two RUs. The glucosyl residue, whose position of attachment to the tetrasaccharide backbone of the RU defines the serotype 2a O-antigen, is critical for recognition by F22-4. Although the complete decasaccharide is visible in the electron density maps, the last four pentadecasaccharide residues from the reducing end, which do not contact the antibody, could not be traced. While considerable mobility in the free oligosaccharides can thus be expected, the conformational similarity between the individual RUs, both within and between the two complexes, suggests that short-range transient ordering to a helical conformation might occur in solution. Although the observed epitope includes the terminal non-reducing residue, binding to internal epitopes within the polysaccharide chain is not precluded. Our results have implications for vaccine development as they show that a minimum of two RUs of synthetic serotype 2a oligosaccharide are required to fully mimic O-Ag epitopes.

### EXAMPLE 2

### *S. flexneri* 2a LPS mimotopes

Our previous studies (Phalipon et al., 1997) showed that it is possible to select peptide sequences binding to anti-LPS mAbs. Such peptides mimic the natural antigen in binding to the paratope and are thus called "mimotopes". Their mimicking capability can be estimated at two different levels: the first is "antigenic", where the mimotopes bind to the anti-LPS antibody used for the selection and compete with LPS for the binding; the second is "immunogenic" and involves the capability of the selected peptide sequences to raise an anti-LPS response upon animal immunization. It is obvious that the identification of efficient "immunogenic" mimotopes is essential for the identification of leads for vaccine development.

The immunogenic mimotopes described in our previous work (Phalipon et al. 1997) (i.e. those raising an anti-LPS antibody response in mice) derived from two different phage- displayed libraries: pVIII-9aa and pVIII-9aa.Cys, having random inserts of nine amino acids in length, being flanked by two cysteine residues in the latter. When present, the level of the antibody response induced the mimotopes was very weak. One hypothesis is that a nine amino acid-long peptide is too short to act as efficient surrogate antigen of the LPS. Consequently, 6 new random phage-displayed peptide libraries were constructed, displaying, respectively, 12, 15 or 28 amino acid-long inserts. In one of the two libraries displaying 12 amino acid-long inserts, two cysteine residues were always present at relative distances spanning from 2 amino acids up to 10 amino acids, so as to represent a collection of disulphide constrained loops of different size.

Two protective mAbs directed against *S. flexneri* LPS 2a (IgG C1-7 and IgG F22-4) were used for mimotope selection using the two libraries displaying 12 amino acid-long inserts. Several clones reacting with IgG F22-4 were identified and their specificity was demonstrated by competition with LPS 2a. The seven phage clones that proved to be the best in the above analysis were prepared for immunization studies. Phage library selections with the other mAb (IgG C1-7) did not work efficiently, as this monoclonal antibody does not react sufficiently well with the biotinylated secondary antibody that has to be used in the library selection procedure.

**Synthetic peptides**

We have decided to ynthesize the peptides and to conjugate them to protein carriers to use as immunogens for immunization of mice.

The peptides have been synthesized with a flanking region AEGEF at the N-terminal and DPAKC at the C-terminal. The peptides have been conjugated to maleimide-activated bovine serum albumin (BSA) and to maleimide-activated keyhole limpet hemocyanine (KLH) using a kit from Pierce. The seven different sequences have been selected by Partner 4, all showing good reactivity with the *S. flexneri* 2a mAb (F22-4) and being inhibited by the LPS. Five of the sequences, B24, B1, C8, C26 and C17 (shown below in bold) have been chosen for further analyzes. The reason that the peptides C22 and C3 have been omitted are that they contain cysteine and the conjugation procedure that we exploited used cysteine for coupling to the protein. The conjugated peptides have been tested for reactivity with the F22-4 mAb and were positive when coupled to BSA. However, the C17 peptide showed a poor reactivity.

**Sequences of peptides selected using the *S. flexneri* 2a specific mAb F22-4**

| Peptide | Sequence |
|---------|----------|
| B24 | AEGEFDQAAQHPRDFHKDPAKC-COOH (SEQ ID No. 8) |
| B1 | AEGEFYLEDWIKYNNQKDPAKC-COOH (SEQ ID No. 9) |
| C8 | AEGEFMKSWASYQAHLMDPAKC-COOH (SEQ ID No. 10) |
| C26 | AEGEFINDWISYYSSWKDPAKC-COOH (SEQ ID No. 11) |
| C17 | AEGEFPHGEIFHMMRRPDPAKC-COOH (SEQ ID No. 12) |
| C22 | AEGEFNRRIPCETGCLLDPAKC-COOH (SEQ ID No. 13) |
| C3 | AEGEFTFTRFCGKGCVPDPAKC-COOH (SEQ ID No. 14) |

**EXAMPLE 3**

**Table 6**

| Antibody-Ligang binding data: Recapitulatory table (ni = not interpreted) | | | | | | | | | |
|---|---|---|---|---|---|---|---|---|---|
| ligand | Biacore Eq | Biacore Cin | | | StopFlow | | | microcal | ELISA |
| | KD($\mu$M) | KD($\mu$M) | kon(M-1.s-1) | koff(s-1) | KD($\mu$M) | kon(M-1.s-1) | koff(s-1) | KD($\mu$M) | IC50($\mu$M) |
| ECD | ni | | | | | | | 104,4$\pm$0,9 | 179$\pm$93 |
| B(E)CD | 5,8 | | ni | ni | 4 | 2,00E+05 | 8,1 | 3,48$\pm$0,04 | 5$\pm$0,9 |
| B(E)CDA | 4,1 | | ni | ni | 3,5 | 7,00E+05 | 2,4 | 3,35$\pm$0,08 | 2,5$\pm$0,4 |
| AB(E)CD | 4,44$\pm$0,26 | | ni | ni | 9,4 | 6,20E+05 | 5,8 | 15,7$\pm$1,3 | 21$\pm$9 |
| ECDAB | | | | | 300 | | | 230$\pm$2 | 354$\pm$40 |
| DAB(E)C | | | | | >1100 | | | | >1000 |
| DAB(E)CD | 49,6$\pm$12,8 | | ni | ni | 48 | 3,20E+05 | 15,1 | | |
| B(E)CDAB(E)C | 0,61$\pm$0,01 | 0,75$\pm$0,03 | 8,87$\pm$0,22$^{E}$4 | 0,068$\pm$0,03 | 0,16 | 3,10E+05 | 0,05 | 0,47$\pm$0,01 | 0,22$\pm$0,02 |
| DAB(E)CDAB(E)C | 5,32$\pm$0,38 | 4,47$\pm$0,66 | 2,84$\pm$0,52$^{E}$4 | 0,123$\pm$0,04 | 5 | 5,90E+04 | 0,29 | 5,4$\pm$0,7 | 5$\pm$1,4 |
| [AB(E)CD]$_2$ | 1,00$\pm$0,02 | 1,09$\pm$0,02 | 6,80$\pm$0,50$^{E}$4 | 0,074$\pm$0,004 | 1,5 | 8,30E+04 | 0,13 | 1,04$\pm$0,02 | 0,7$\pm$0,6 |
| [AB(E)CD]$_3$ | 0,56$\pm$0,03 | 1,56$\pm$0,30$^{(H)}$ | 4,92$\pm$0,57$^{E}$4$^{(H)}$ | 0,075+0,005$^{(H)}$ | 0,69 | 1,70E+05 | 0,11 | 1,03$\pm$0,03 | 0,32$\pm$0,07 |
| Peptide B1 | 2,98$\pm$031 | | | | | | | 2,49$\pm$0,06 | $\approx$3 |

**TABLE 7**

| ITC MICROCALORIMETRY (INTERACTION CARRIED OUT IN PBS BUFFER ÀT 25°C) | | | | |
|---|---|---|---|---|
| Ligand | KD ($\mu$M) | n | $\Delta$H (Kcal/mol) | $\Delta$S (cal/mol/K) |
| [AB(E)CD]$_3$ | 1,03 $\pm$0,03 | 2,03$\pm$0,02 | -17,3$\pm$0,8 | -30,6 |
| [AB(E)CD]$_2$ | 1,04$\pm$0,02 | 1,93$\pm$0,04 | -17,7$\pm$0,7 | -31,7 |
| B(E)CDAB(E)C | 0,47$\pm$0,01 | 2,05$\pm$0,20 | -19,5$\pm$0,2 | -36,6 |
| DAB(E)CDAB(E)C | 5,4$\pm$0,7 | 1,80$\pm$0,03 | -19,5$\pm$01,2 | -41,2 |
| AB(E)CD | 15,7$\pm$1,3 | 1,78$\pm$0,04 | -11,7$\pm$0,3 | -17,5 |
| B(E)CDA | 3,35$\pm$0,08 | 1,77$\pm$0,01 | -14,2$\pm$0,1 | -22,7 |
| B(E)CD | 3,48$\pm$0,04 | 1,74$\pm$0,01 | -12,4$\pm$0,05 | -16,7 |
| ECDAB | 230$\pm$2 | 1,72$\pm$0,01 | -10,8$\pm$0,1 | -19,6 |
| ECDA | 89$\pm$1 | 1,72$\pm$0,01 | -11,3$\pm$0,1 | -19.2 |
| ECD | 104,4$\pm$0,9 | l,74$\pm$0,01 | -9,58$\pm$0,09 | -13,5 |
| Peptide B1 | 2,49$\pm$0,06 | 1,52$\pm$0,05 | -24,0 $\pm$0,1 | -54,6 |

### <u>EXAMPLE 4</u>: Crystal structure of the Fab F22-4/peptide B1 complex

**General description of the complex**

The F22-4/B1 peptide complex crystallises in the monoclinic space group P2$_1$ with two independent molecules in the asymmetric unit. The Fab elbow angles are 139° and 149° for the two respective complexes, showing a variability already noted for F22-4 in the O-antigen serotype 2a deca- and pentadecasaccharide complexes, as well as in the free form (manuscript Fab-oligosaccharide complex). The F22-4 variable domains in the peptide and oligosaccharide complexes show little structural difference between each other, even at the level of side chain positions (0.43 and 0.74 Å r.m.s. difference in C$\alpha$ and all atomic positions, respectively, when superimposing $V_H$-$V_L$ dimers). Although comparison between the variable dimers of the peptide complex and non-complexed F22-4 shows a larger difference (0.62 Å r.m.d. difference in C$\alpha$ positions), this cannot be taken to reflect the change upon antigen binding because intensive intermolecular contacts occur at the antigen-binding site in the crystal structure of the free Fab. The unusual cis peptide bond between Pro-H95 and Met-H96 in short CDR-H3 (four residues), occurring in the free and oligosaccharide-complexed forms, is also present in the peptide complex.

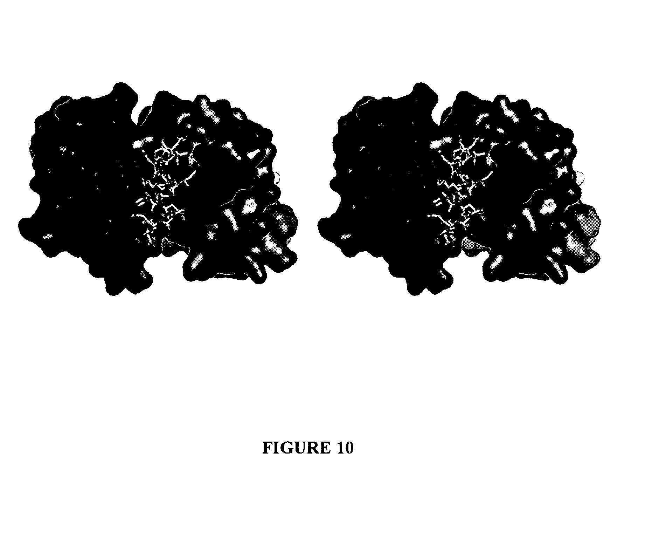

All twelve residues of the antigenic peptide B1 (YLEDWIKYNNQK SEQ ID No. 2) could be traced in the electron density maps for both complexes, although the C-terminus is better ordered in the second complex because of contacts made with neighbouring molecules in the crystal lattice. The peptide adopts an $\alpha$-helical conformation over its entire length and the two crystallographically independent copies of the ligand superimpose on each other with a r.m.s. difference of 0.16 Å between all main chain atoms and 0.74 Å when side chain atoms are included. The peptide is capped at its N-terminus by a salt bridge formed between the carboxylate group of Glu-P3 and the main chain amino group. In addition, one water molecule is maintained at the N-terminus of the peptide helix by the main chain amino group, Glu-P3 (through both the carboxylate group and the main-chain amide) and the main chain amide of Asp-P4. The peptide helix lies along the groove-shaped antigen-binding site, which is flanked on one side by CDR-L1, and on the other by CDR-H1 and CDR-H2, with CDR-L3 and CDR-H3 forming the floor (Figure 10).

**Antibody-antigen interactions**

Six residues of the peptide make direct contacts with F22-4 (Tyr-P1, Leu-P2, Asp-P4, Trp-P5, Ile-P6 and Tyr-P8; inter-atomic distances < 3.8 Å). These contacts include five hydrogen bonds and a salt bridge (Table 8). In addition, three water molecules bridge between the peptide and F22-4. The total buried accessible surface at the antibody-antigen interface is 1081 Å$^2$, a value comparable with that observed in the oligosaccharide complex (1125 Å$^2$). The surface complementarity index is 0.77 (0.79 when the buried three solvent molecules are included) and is thus higher than that occurring in the oligosaccharide (0.65 and 0.75 without and with the 14 bridging solvent molecules included, respectively). The antibody hypervariable regions forming contacts to the peptide are CDR-L1 (**His-L27D**, Asp-L28D, **Tyr-L32**), CDR-L3 (**Asn-L91, Val-L92, Glu-L93, Arg-L96**), CDR-H1 (**Asn-H31, Tyr-H32, Trp-H33**), CDR-H2 (**Arg-H52**) and CDR-H3 (**Pro H95**), where residues in bold also contact the serotype 2a oligosaccharides (Table 8). Although CDR-L3 residue Leu-L94 and CDR-H2 residues Leu-H52A and Tyr-H58 contact the antigen in the saccharide complex, they do not interact with the peptide.

The aromatic antigen residues Tyr-P1, Trp-P5 and Tyr-P8 form a continuous aromatic patch on the peptide surface, making the most important interactions with F22-4 as judged by the number of inter-atomic contacts and buried accessible surface area at the interface (Table 9). The region occupied by Tyr-P1, Trp-P5 and Tyr-P8 at the antigen-binding site partially overlaps with that occupied by GlcNAc(D), Rha(C) and Glc(E), respectively, in the oligosaccharide complex, which are the most important sugar residues in determining the affinity and specificity of F22-4 for the serotype 2a antigen-O.

**Table 8.** Polar interactions between F22-4 and the peptide, comparing with equivalent interactions to the decasaccharide.

(a) Antibody-antigen contacts

| CDR residues | | | peptide | | | oligosaccharide | |
|---|---|---|---|---|---|---|---|
| Asn | L-91 | Oδ1 | Trp 5 | Nε1 | GlcNAc(D) | | O4 |
| | | Nδ2 | Tyr 1 | OH | Rha(C) | | O5 |
| Arg | L-96 | Nζ1 | Tyr 1 | OH | GlcNAc(D) | | O4 |
| | | Nζ2 | Tyr 1 | OH | GlcNAc(D) | | O4 |
| Trp | H-33 | N | Tyr 8 | OH | Glc(E) | | O4 |
| Arg | H-52 | Nζ1 | Asp 4 | Oδ2 | Glc(E') | | O9 |

(b) Common bridging water molecules

| CDR residues | | | water - peptide | | | water - oligosaccharide | |
|---|---|---|---|---|---|---|---|
| Trp | H-33 | O | Wat 27 (33) | Tyr 8 | OH | Wat 134 | Glc(E) | O4 |
| Pro | H-95 | O | Wat 27 (33) | Tyr 8 | OH | Wat 134 | Glc(E) | O4 |
| His | L-50 | Nε1 | Wat 11 (536) | Trp 5 | Nε1 | Wat 865 | Rha(C) | O3 |

**Table 9.** Summary of interactions per peptide residue.

The number of inter-atomic contacts (< 3.8 Å) between the antibody and peptide as well as the number of these that are hydrogen bonds are given in the first and second lines, respectively. The numbers of water molecules bridging between the antibody and oligosaccharide are given in the third line. The buried surface area of each peptide residue at the antibody/antigen interface is calculated using a spherical probe of 1.4 Å radius.

Residue    Y 1    L 2         E

| Residue | Y1 | L2 | E3 | D4 | W5 | I6 | K7 | Y8 | N9 | N10 | Q11 | K12 |
|---|---|---|---|---|---|---|---|---|---|---|---|---|
| | | | | | | | | | | | | |
| Contacts | 133 | - | 3 | 7 | 5 | - | 13 | - | - | - | - | - |
| H bond | 3 | - | - | 1 | 1 | - | - | 1 | - | - | - | - |
| Water bridges | - | - | - | - | 1 | - | - | 1 | - | - | - | 1 |
| Buried surface | 129 | 92 | 13 | 37 | 97 | 46 | - | 115 | 9 | - | 2 | 45 |

**Structural mimetism of the oligosaccharide by the peptide**

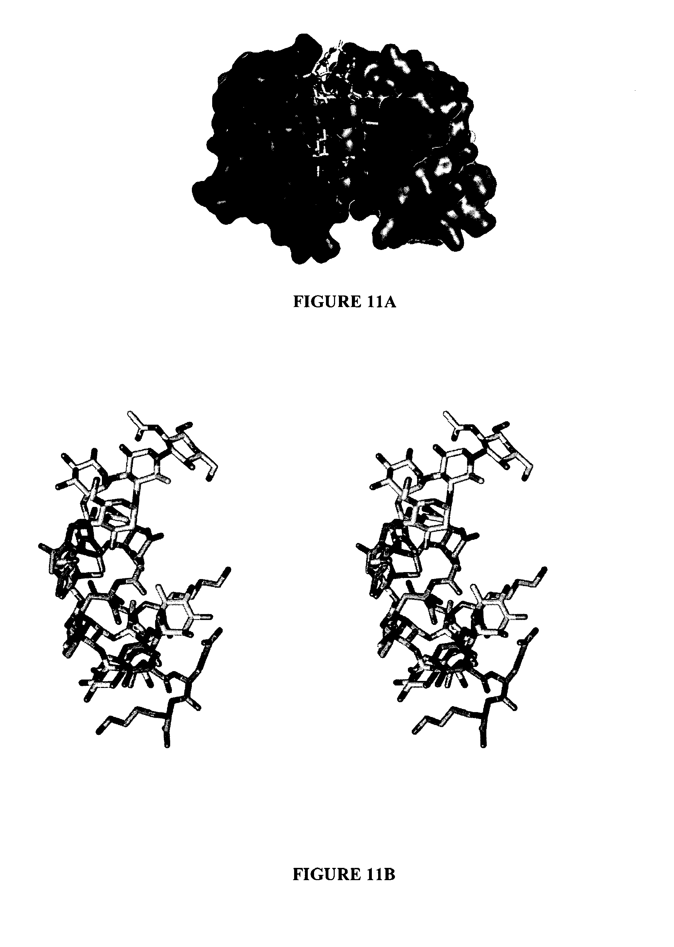

The helical peptide lies along the groove-shaped antigen-binding site but is slightly displaced relative to the position taken by the oligosaccharide, thus accounting for the minor differences in CDR residues used by the two ligands (Figures 11A-11B). Five direct hydrogen bonds and one salt bridge are formed between F22-4 and B1, with three water molecules also contributing by bridging between the antibody and ligand. This contrasts with the highly hydrophilic interactions present in the oligosaccharide complex of F22-4, where eleven direct hydrogen bonds and fourteen bridging water molecules contribute to polar interactions at the antibody-antigen interface. Nonetheless, all five peptide-F22-4 hydrogen bonds have their equivalents in the oligosaccharide complex, occupying essentially identical positions and orientations at the antigen-binding site (Table 8a). Of the three bridging water molecules in the peptide complex, two have close counterparts in the oligosaccharide complex (Table 8b).

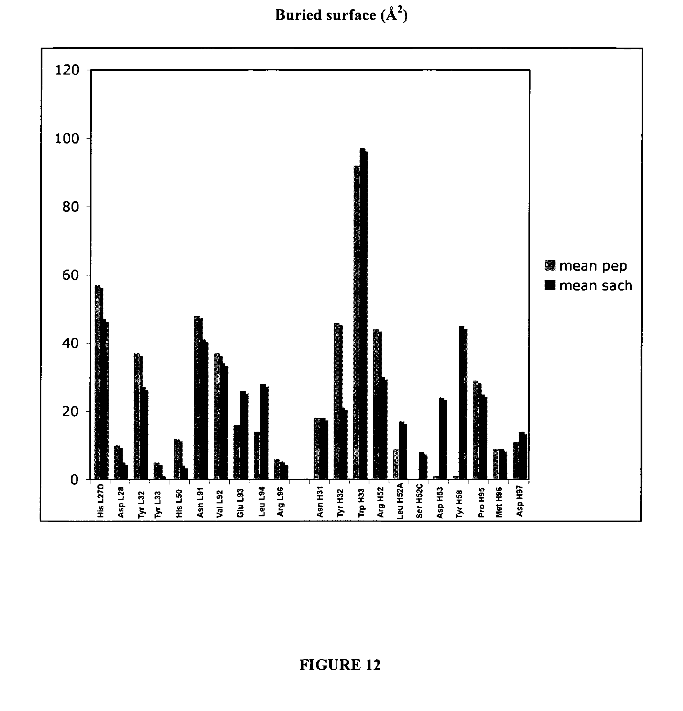

Fine details of the antigen-binding site of F22-4 are very similar in the peptide and saccharide complexes since conformational differences in the CDRs are very minor, even for the side chains. Comparison between the saccharide and peptide complexes with respect to antibody-antigen interatomic contacts (Table 10) and buried accessible surface of the antibody (Figure 12) shows that F22-4 binds to the oligosaccharide and peptide antigens using essentially the same CDR residues even though fewer polar interactions occur in the peptide complex. Although some CDR residues interact differently in the peptide and carbohydrate complexes, their contribution at the antibody-antigen interface, as indicated by the buried accessible surface (Figure 12), is very similar in the two structures.

**Table 10.** Comparison of F22-4 contacts with the antigenic peptide and oligosaccharide. The numbers of inter-atomic contacts (<3.8 Å) are given per Fab residue, with the number of hydrogen bonds shown in parenthesis.

| F22-4 residue | no. contacts peptide | no. contacts saccharide |
|---|---|---|
| His L-27D | 4 | 4(1) |
| Asp L28 | 1 | - |
| Tyr L-32 | 2 | 1 |
| Asn L-91 | 10(1) | 10(2) |
| Val L-92 | 2 | 1 |
| Glu L-93 | 2 | 1 |
| Leu L-94 | - | 2 |
| Arg L-96 | 3(2) | 3(2) |
| Asn H-31 | 1 | 2(1) |
| Tyr H-32 | 5 | 3 |
| Trp H-33 | 11(1) | 9(1) |
| Arg H-52 | 3(1) | 2(2) |
| Leu H-52A | - | 1 |
| Tyr H-58 | - | 6(1) |
| Pro H-95 | 1 | 2(1) |

**Structural comparison between the free and Fab-complexed peptides**

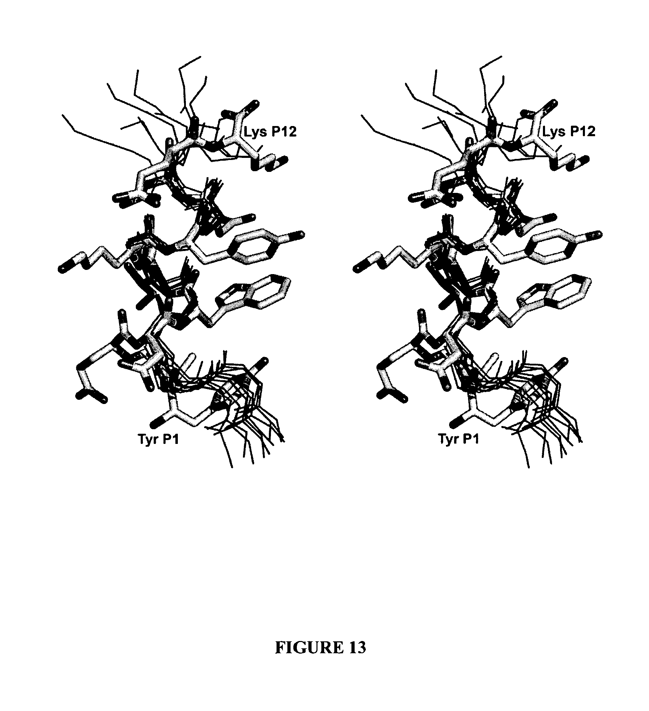

The solution structure of the free peptide determined by NMR shows that a helical conformation is present the central region between residues Asp-P4 and Asn-P10. Superposition of this region of the ten lowest energy structures onto the crystal structure gives a mean r.m.s. difference of 0.8 Å between main chain atom positions (ranging from 0.6 to 1.4 Å) (Figure 13). The N-terminal capping of the complexed peptide observed in the crystal structure is not present in the solution structures because the peptide adopts a turn conformation in this region.

The solutions structures of B1 were superposed onto the Fab-bound peptide to gain further understanding of the antigen-antibody association phase. This showed that steric hindrance would occur between the antibody and the N-terminal peptide residues Tyr-P1 and Leu-P2. The N-terminal residues in the solution conformation, which form a turn in the ten lowest energy structures selected, must therefore rearrange during the approach of the peptide to the antibody if this binding mechanism operates. Alternatively, the antibody could select a higher energy conformation, present in solution with lower frequency but closer in structure to that observed in the crystal, that avoids steric hindrance upon binding.

**Methods and materials**

**Crystallisation and structure determination**

F22-4 was prepared and purified as described for the preparation of the oligosaccharide complexes. The Fab and peptide B1 were incubated for 4 hours at 37°C, with a 1:10 molar ratio. A volume of 1 $\mu$L of the mixture was added to 1 $\mu$L of buffer of various commercial crystallization kits and sealed over a reservoir containing 0.7 ml of this buffer.

Hanging drops were stored at 17°C. The final protein concentration was 3.7 mg/ml. The best crystals were obtained from a crystallisation buffer containing 20% (w/v) PEG 6000 and 50mM imidazole pH 7.6 (about JBScreen Classic 4-B3). We used a cryoprotectant consisting of 35 % (w/v) PEG 6000 and 50mM imidazole, pH 7.6, mixed with 15 % glycerol.

Diffraction data were collected at the European Synchrotron Radiation Facility, Grenoble, on beam-line ID14-4 (Tablell). The structure of the Fab was solved by molecular replacement using the refined F22-4 coordinates using the program AMoRe. The structure was refined using the programs REFMAC and ARP/Warp. Peptide B1 was built into the electron density during the earlr stages of the refinement. Final refinement statistics are given in Table 12.

**Table 11.** Crystal parameters and data collection statistics.

| | |
|---|---|
| Space group | $P2_1$ |
| Unit cell | |
| a, b, c (Å) | 66.9, 69.5, 110.2 |
| β (°) | 97.7 |
| Z (molecules/unit cell) | 4 |
| $V_M$ ($A^3$ $Oa^{-1}$) | |
| Beamline (E.S.R.F) | ID14-4 |
| Wavelength (Å) | 0.939 |
| Resolution range (Å) | 1.7 |
| last shell (Å) | |
| No. unique reflections | |
| Redundancy | |
| $R_{symm}$ | |
| Completeness (%) | |
| I/σ(I) | |

**Table 12.** Refinement statistics

| | |
|---|---|
| Resolution (Å) | 100 - 1.7 |
| last shell (Å) | 1.72 - 1.70 |
| R-value (working set) | 0.201 (0.274) |
| $R_{free}$ | 0.248 (0.351) |
| No. reflections (total) | 105406 (3859) |
| No. reflections for $R_{free}$ | 2146 (94) |
| No. protein atoms | 6793 |
| No. solvent atoms | 783 |
| r.m.s. deviation from ideal | |
| Bond lengths (Å) | 0.012 |
| Bond angles (°) | 1.5 |

**EXAMPLE 5: PDB, Crystal structure of Fab F22-4 free or in complex with a carbohydrate-mimetic peptide (B1 peptide) or in complex with a S. flexneri 2a O-Ag synthetic fragment**

**Crystal 1- 3CK3**

Crystal structure of Fab F22-4 in complex with a carbohydrate-mimetic peptide (see Table 13)

**Crystal 2- 3C5S**

Crystal Structure of monoclonal Fab F22-4 specific for *S. flexneri* 2a O-Ag (see Table 14)

**Crystal 3- 3BZ4**

Crystal structure of Fab F22-4 in complex with a *S. flexneri* 2a O-Ag decasaccharide (see Table 15)

**Crystal 4- 3C6S**

Crystal structure of Fab F22-4 in complex with a *S. flexneri* 2a O-Ag pentadecasaccharide (see Table 16)

**EXAMPLE 6: Structural definition of the protective antibody epitope**

**Materials and Methods**

**Nomenclature.** The two torsion angles describing a glycosidic linkage are defined as $\Phi = H_1 - C_1 - O_1 - C'_X$ and $\Psi = C_1 - O_1 - C'_X - H'_X$ with the primed atoms belonging to the reducing moiety and the sign being in agreement with IUPAC nomenclature.

**NMR spectroscopy of free oligosaccharides, peptide B1 and O-Ag.** Solution concentrations of the pentasaccharides ECDAB-OMe, AB(E)CD-OMe, B(E)CDA-OMe, and DAB(E)C-OMe were 13.5 mM, 10 mM, 20 mM, and 20 mM, respectively, in 400 μL deuterium oxide. pmLPS 2a was obtained from acid hydrolysis of the LPS (the resulting purified polysaccharide moiety (pmLPS) is composed of an average of 15 repeating units). About 3 mg of pmLPS 2a were dissolved in 400 μL of deuterium oxide. Lyophilized peptide B1 was dissolved in a sodium acetate/$D_2O$ buffer (20 mM in sodium acetate, pH=5.3) to a final concentration of 2.75 mM at a temperature of 283K.

NMR experiments were recorded on Varian Unity Inova spectrometers, operating at $^1$H frequency of 500 MHz and 600 MHz, equipped with a triple resonance pulsed field gradient probe with an actively shielded z gradient and for the 600 MHz a cryoprobe. Shigemi tubes (diameter of 5 mm) were used for all samples. Chemical shifts are given relative to an external standard of sodium 2,2-dimethyl-2-silapentane-5 sulfonate (DSS) at 0 ppm for both $^{13}$C and $^1$H chemical shifts. DQF-COSY, TOCSY, off-resonance ROESY, gHSQC, gHSQC-TOCSY, and gHMBC experiments were performed as described previously [41], at 35°C or 38°C for the pentasaccharides and at 50°C for the **pmLPS 2a.** The $^3J_{H,H}$ coupling constants were measured in one-dimensional spectrum with a digital resolution of 0.1 Hz/point, or in the DQF-COSY experiment with a digital resolution of 0.75 Hz/point. The $^1J_{C1,H1}$ coupling constants were measured in the gHMBC spectrum with a digital resolution of 0.65 Hz/point.

The ROESY experiments were acquired at 35°C with mixing period of 400 ms for the pentasaccharides. The NOESY experiments were acquired with mixing periods varying between 60 and 550 ms at 50°C for pmLPS and at 10°C for peptide B1.

T1 and T1rho values for $^{13}$C were evaluated by measuring the peak intensities in 9 gChsqc experiments (rna gChsqc of the Varian-BioPack [42]) with relaxation delays, τ, ranging from 0.005 to 0.6 s or from 0.05 to 0.3 s for longitudinal and transverse relaxations, respectively. The resulting curves were fitted to the following equation

$$y = A \exp(-\tau / T_{1,2}).$$

Error on data points was estimated as 5 noise rsmds.

The steady-state $^1$H-$^{13}$C noes were obtained using the C13NOE_CT pulse sequence [2]. Two experiments were acquired for each pentasaccharides and for the mLPS: a reference spectrum, without proton-saturation, and a second one with 2s of proton-saturation. Steady-state $^1$H-$^{13}$C noes were calculated using the measured peak intensities as follows:

$$\eta = (I_{sat} - I_{ref})/I_{ref}$$

**NMR Spectroscopy of Ligand-Antibody interactions.** $^1$H NMR experiments were recorded at several temperatures, between 15°C and 40°C, both to observe signals overlapping with the water-peak and to increase the off-rate of the interactions. To study the interaction with the pentasaccharides, IgG antibody F22-4 was concentrated after repeated cycles of exchange with $D_2O$ buffer (20 mM deuterated sodium phosphate, pH 7.0) in Vivaspin 50000 MWCO concentrators.

TrNOE experiments [43] were performed on 3:1, 3.5:1, 5:1 pentasaccharide:mAb ratios in terms of binding site, with 135 μM, 25 μM and 30 μM of F22-4 antibody, for B(E)CDA, ECDAB, and AB(E)CD, respectively. Mixing times varying from 100 to 800ms were used to establish the trNOEs building curves.

One-dimensional (1D) STD spectra [44] of the pentasaccharide in the presence of IgG F22-4 were recorded after 16384 scans, with pentasaccharide:antibody ratios in binding site of 15:1 for AB(E)CD (0,9 mM), 12:1 for ECDAB (0,6mM); 15:1 for BECDA (1,2mM), and 37.5:1 for peptide B1 (0.1 mM). A 10 ms $T_{1\rho}$ filter was inserted before acquisition in order to remove F22-4 signals. For all STD-NMR experiments, selective on-resonance irradiations of antibody resonances were performed at 0.8 ppm for the oligosaccharides and at -0.2 ppm for the peptide B1, and off-resonance at 30 ppm, using series of 20 to 60 Gaussian shaped pulses of 270° (50 ms, 5$\mu$s delay between pulses, excitation width approximately $\gamma B_1/2\pi$ of 50 Hz) for a total saturation time of 1s. Subtraction of saturated spectra from reference spectra was obtained by phase cycling [45]. To quantitate the STD effect, the observed signals were integrated with respect to the corresponding signals in the reference spectra of pentasaccharides free in solution, using the VNMR software.

**Inter-proton distances from cross-peak volumes.** The cross-peak volumes from off-resonance ROESY, NOESY and trNOESY experiments were measured with the NMR-View 5.0 software. The intra-residue distance of 2.52 Å between H-1 and H-2 protons of the $\alpha$-Rhamnopyranosyl unit B was used as reference for distance calibration of pentasaccharides spectra, and the intra-residue distance of 2.43 Å between H-1 and H-5 protons of the $\beta$-D-N-acetyl-Glucosamine-pyranosyl unit D was used as reference for distance calibration of the O-Ag spectra. The proton-proton distances were calculated using the usual $1/r^6$ NOE/distance relationship [46].

Spectra were processed using the NMRPipe software package [47] and nOes were integrated with NMRView 5.03 [48].

**ARIA three-dimensional structure of peptide B1.** Structure calculation was performed with the programs ARIA 1.2 [49] and CNS 1.1 [50]. Eight iterations of automated nOe assignment were performed using the ARIA/CNS simulated annealing protocol described in [51]. 5 $\Phi\Phi$ torsion angles were set in the interval $-85 < \Phi\Phi < 25°$ (for the amino acids 2, 3, 4, 5, 7) in order to make the nOe assignment easier during the first iterations. Per iteration, 100 structures were calculated using a random conformation as starting structure, of which 10 were used for nOe-assignment. The 10 energetically best structures calculated in the last iteration were refined in explicit water, giving the final structure ensemble. Structural quality was assessed using the programs PROCHECK 3.54 [52] and WHATIF [53]. The MolMol program [54] was used to generate the figure representing the structures.

**Molecular Dynamics of the oligosaccharides.** 50 ns to 185 ns MD simulations were performed on tri-, tetra-, penta-, deca-, and pentadecasaccharides. The initial conformation of the polymer was generated from the builder module LeaP AMBER 9 suite programs [55]. The solute was then placed in a theoretical rectangular box of TIP3P water molecules, and box dimensions were designed to extend at least 10Å beyond the sugar in each direction. The MD simulations were performed under periodic boundary conditions at constant pressure (1 atm), and constant temperature (300 K), with a particle Mesh Ewald treatment of long-range electrostatic interactions, using the sander or pmemd modules of AMBER 9. The GLYCAM 04 parameters [56], [57] were implemented for the oligosaccharide simulations. Energy minimizations and MD simulations were performed with a dielectric constant of unity, and a cut-off value for non-bonded interactions of 9Å. The 1-4 electrostatic and van der Waals interactions were scaled to the unity (SCEE = 1, SCNB = 1) as recommended in the AMBER users' manual. The SHAKE [58] algorithm was applied consistently with the use of TIP3P model of water [59], and a 2 fs time step was employed in the integration of the equations of motion.

For each system, initial solvent organizations were subjected to 15000 cycles of energy minimization, composed of 1000 steepest descent cycles and 14000 cycles of conjugate gradients minimization, with restrained solute coordinates. Then, energy minimization was carried out on the entire system for 30000 cycles (1000 steepest descent followed by 29000 conjugate gradient). Subsequently a simulated annealing of the solvent was achieved, during which the water molecules were heated from 5 to 300 K over 50 ps, held at 300 K for 200 ps and then cooled to 5 K over 50 ps. The entire system was subjected to a final minimization for 30000 cycles. Prior to the MD run, the system was warmed from 5K to 300 K over a period of 50 ps.

Snapshots were collected every ps. Post-processing of the trajectories (torsion, distances) were performed using the ptraj module of AMBER 9.

The mean distances were calculated in a "noe-approach" according to the following equation: $r=(\Sigma(1/r^6))^{-1/6}$.

**Molecular Dynamics of the peptide B1.** Several MD simulations of tens of ns were performed on peptide B1 under periodic boundary conditions at constant pressure (1 atm), and constant temperature (300 K), with a particle Mesh Ewald treatment of long-range electrostatic interactions, using the sander or pmemd modules of AMBER 9. We took as initial conformations those obtained after the eighth iteration of ARIA, or the one observed in the crystal structure of the complex formed between peptide and IgG F22-4. Theoretical boxes of TIP3P water molecules extending at least 12 Å beyond the solute in each direction were generated from the builder module LeaP AMBER 9 suite programs with ff03 force field [60]. Dielectric constant was set to one, the cut-off value for non-bonded interactions to 10Å, The SHAKE [58] algorithm was applied consistently with the use of TIP3P model of water [59], and a 2 fs time step was employed in the integration of the equations of motion.

Initial solvent organizations were subjected to 1000 cycles of energy minimization (500 steepest descent and 500 cycles of conjugate gradient cycles), with restrained solute coordinates. Then, the same protocol energy minimization was carried out on the entire system. Subsequently the system was heated from 5 to 300 K over 40 ps, and then observed at 300 K for 140 ps before MD runs. Snapshots were collected every ps and post-processed using the ptraj module of AMBER 9.

**Structures of bound saccharides.** Molecular dynamics simulated annealing in vacuum with noe-derived restraints were used to calculate the approximate structures of the pentasaccharides when complexed to the F22-4 antibody. This was done in Sander module of Amber 8. The systems were heated from 0K to 600K in 5ps, and then cooled back in 14ps. The pyranose cycles were maintained in their energetically favoured conformation with strong restraints on two cyclic dihedral torsion angles for each residue: the violation energy is a well with a square bottom between lower and upper bounds 10° far from the ideal angles, with parabolic sides out to 20° and linear sides beyond. Similar terms were used to constrain the modelled distances, which were obtained by NMR. The parabolic energetic disadvantage started for distances being 0.5 Å above the experimental measurements, and linear sides were defined for differences greater than 0.7 Å.

**Conformational studies**

The highly serotype-specific recognition of the elicited antibodies, which recognize the various slightly different O-Ag, makes us confident in the ability of the O-Ag 2a oligosaccharidic fragments to provoke a well-defined immune response. However, this structural approach hides more complex phenomena, which could be of significant importance in the design of mimic molecules of these polysaccharidic molecules.

The three-dimensional conformational behaviour of the O-Ag and its oligosaccharidic fragments are of particular significance. Indeed, these are highly flexible molecules; therefore the recognition by the F22-4 antibody can be either an induction of a shape that is not adopted in solution by the quoted oligosaccharides, or a selection of a conformational epitope, i.e. one of the numerous pre-existing conformations. Hence, a deeper knowledge of these behaviours could allow us to design ligands presenting an acceptable epitope in the appropriate conformations with increased affinities and "immune-eliciting" power for the antibodies recognizing the O-Ag 2a such as F22-4. The major drawbacks are the followings: the designed ligands could elicit antibodies unable to not recognize the O-Ag 2a or they would not be specific enough; the flexibility of O-Ag 2a is advisable to obtain various types of recognition by various types of antibodies.

**The (de-O-acetylated) O-Ag free in solution**

Considering that NMR-spectra of the $\{AB(E)CD\}_3$ pentadecasaccharide and of the de-O-acetylated O-Ag 2a are very similar (Figures 14A-14B), we assume that the two molecules display very similar conformational behaviour. Thus, we performed a MD simulation of $\{AB(E)CD\}_3$ free in solution in an explicit solvent with the GLYCAM-04 force field. The correlation between the noe-derived distances and those obtained by molecular dynamics is quite good (Figure 15).

Concerning the glycosidic linkages of the primary chain (Figure 16), A-B and B-C explore mainly one well, and C-D and D-A travel over a more extensive dihedral space with at least 3 energy minima separated by low energy barriers (Figure 17A). The trajectories of dihedral angles suggest that the very important glycosidic linkage between glucose E and rhamnose C adopts somehow two conformations (Figure 17B): the most populated one corresponds to $\Phi \approx -50°$ and $\Psi \approx -25°$, and a rare second energy minimum is observed with $\Phi \approx +20°$ and $\Psi \approx +40°$. The latter is encountered in every repetitive unit, implying that this conformation is not due to an end effect.

Every $^1$H-$^{13}$C pair shows similar values of $^{13}$C NMR-relaxation parameters T1 and $T1_\rho$ and of $^1$H-$^{13}$C heteronuclear nOes (Figure 18). Hence, it seems that the glycosidic bond between residues E and C does confer weak additional degrees of freedom to E, as observed in the MD trajectories.

**The four pentasaccharidic fragments of O-Ag 2a free in solution**

The MD simulations performed for tri-, tetra- and penta- saccharides show very good correlations with the NMR-experimental results (Figures 19A-19C). Concerning the dynamical behaviour, we can state that glycosidic linkages of the primary chain display identical behaviours in these four molecules. Thanks to $^{13}$C NMR-relaxation times we can emphasize that flexibility is not significantly amplified for the branched glucose E (Figures 20A-20D), whereas more important motions are observed for the end residues.

Noteworthy differences of conformational behaviours are encountered with the branching link E-C. Great flexibility is observed in ECD, ECDAB: dihedral angles of the link E-C oscillate between two broad energetic minima, which are similar for the two oligosaccharides ($\Phi \approx -40°, \Psi \approx -15°$ and $\Phi \approx -30°, \Psi \approx +30°$ for ECD and ECDAB) with high frequencies around 100 GHz (Figures 21A-21F). Conformational changes are quite different in terms of trajectories for B(E)CD, B(E)CDA, AB(E)CD and DAB(E)C: indeed two conformations are adopted by these oligosaccharides, separated by high energy barriers ($\Phi \approx -40°, \Psi \approx -25°$ and $\Phi \approx 35°, \Psi \approx 0°$ for B(E)CD and B(E)CDA; $\Phi \approx -50°, \Psi \approx -25°$ and $\Phi \approx +20°, \Psi \approx +30°$ for DAB(E)C). B(E)CD and B(E)CDA display equally populated conformations, with a frequency turn around 200MHz (Figures 21A-21F). DAB(E)C an AB(E)CD adopt the same conformations as FB325, but with longer lifetimes and a larger population in the situation $\Psi \approx +40°$ than FB325, and with a conformational change frequency around 100 MHz.

Although a wide -but non-exhaustive- inventory of explanations can stand to describe the glycosidic bond behaviours such as steric hindrances, exo-anomeric effects, non-bonded interactions, etc., we were mostly interested in the significant importance of hydrogen-bonds and water-bridges. Indeed, their influence on glycosidic torsion angles is considerable even though they involve hydroxyl groups of residues not involved in glycosidic bond.

As far as B(E)CDA is concerned, 8 possible bridges exclusively stabilize situation 1 (3 between E and B, 4 between E and C, 1 between E and D), and 7 for the conformation close to situation $\Psi\approx+40°$ (5 between E and B, 2 between E and C). Looking at the trajectory of AB(E)CD, new stabilizing bridges can occur between E and A, so that 13 of them strengthen the situation $\Psi\approx-25°$ (5 between E and A, 3 between E and B, 4 between E and C, 1 between E and D) and 9 the $\Psi\approx+40°$ (3 between E and A, 4 between E and B, 2 between E and C). Similarly, DAB(E)C displays new bonds between E and D: 14 possible stabilizing bridges are observed for the conformation close to situation $\Psi\approx-25°$ (2 between E and D, 5 between E and A, 3 between E and B, 4 between E and C), whereas 10 stabilize exclusively the situation $\Psi\approx+40°$ (4 between E and D, 4 between E and A, 2 between E and B). The same analysis is not feasible with ECDAB because of conformers short lifetimes. However, the small number of possible H-bonds and water-bridges between E and the other residues is easily conceivable in ECDAB if we compare with the branched oligosaccharides, and together with the absence of residue B allow an increased flexibility of the glycosidic bond E-C.

The occurrence of residue A at the non-reducing end of the motif B(E)C seems to improve the mimicry of O-Ag conformational behaviour as far as the E-C glycosidic bond is concerned. We can point out that the lifetimes of the E-C conformations increase with the number of stabilizing bridges between E and residues of the backbone.

## The peptide B1 free in solution

$^1$H resonances of B1 were fully assigned (Table 17) and 293 proton proton distances (22 ambiguous and 271 unambiguous of which 113 intraresidue, 89 sequential, 62 so-called medium distances, 7 so called long distances) were used to calculate the structure of B1 together with 5 $\Phi$ torsion angles. The 10 lowest-energy ARIA structures were selected to yield the final structural ensemble in agreement with the NMR data (Figure 22). Parameters defining the quality of the structures in terms of restraints violation and deviation from ideal geometry are summed up in Table 18. Distance violations larger than 0.3 Å are scarce and the covalent geometry is respected, as evidenced by the low RMSD (root mean square deviation) values for bond lengths and valence angles. PROCHECK analysis of the ensemble of structures gives 100% of the backbone angles in the allowed regions of the Ramachandran plot and 82% in the most favored. Whereas the two last residues do not display a preferred orientation, with a poor number of inter-residue nOes, the ten first amino acids are remarkably well defined for a peptide of this size: limited to this N-terminal region, the final ensemble of tertiary structures has an average pair-wise RMSD of 0.53 $\pm$ 0.18Å and 1.45 $\pm$ 0.35Å for backbone and heavy atoms, respectively.

Looking at the ten final structures given by ARIA, a turn of $\alpha$-helix appears clearly from residues 5 to 8, and a very well defined fragment appears from residues 3 to 10. The N-terminal tyrosine residue displays a higher disorder, whereas the two C-terminal residues are not structurally constrained. The MD simulations that we performed confirm these observations. The $\Phi$, $\Psi$ dihedral angles of the residues 4 to 10 display a good convergence.

The torsion angle $\chi_1$ N-C$\alpha$-C$\beta$-C$\gamma$ of Trp5 was of peculiar interest, as the orientation of the indole ring is quite relevant in the recognition of the whole peptide by the antibody F22-4, according to the crystal structure of the complex. Selecting the nOe-constraints in which Trp5 side chain was involved never led to a trans ($\chi_1 = 180°$) conformation as observed in the crystal, but instead to the gauche+ ($\chi_1 = 60°$) conformation. A few nOe-peaks are able to discriminate between the two orientations, and each of them characterizes a predominance of the gauche+ conformation (e.g. H$\delta1_{W5}$-H$\alpha_{I6}$ is very weak, H$\epsilon3_{W5}$-H$\alpha_{W5}$ and H$\delta1_{W5}$-H$\alpha_{W5}$ are of the same size, and so are H$\delta1_{W5}$-HN$_{W5}$ and H$\delta1_{W5}$-HN$_{I6}$). However, we observed in the MD runs oscillations between these two orientations, with lifetimes of around 5 to 10 ns.

All these MD simulations show a great stability of the $\alpha$-helix from residue Trp5 to residue Asp9, which is consistent with the ARIA result, and makes us confident in the pre-formation of a turn of $\alpha$-helix prior binding to with IgG F22-4. Similarly, the free motion of the C-terminal residues Gln11 and Lys12 is confirmed, whereas the three N-terminal residues backbone explores a more extended conformational space than expected with ARIA. Thus, although one could have apprehended that ARIA protocol would have accentuated the convergence of the folding of the peptide, the relevance of the ARIA treatment of the nOe informations is confirmed by MD simulations, so that altogether the Figure 22 gives a wealthy representation of B1 overall structure.

**Table 17: 1H chemical shifts of the peptide B1 in H2O/D2O (90/10), pH 5.1 and 298K.[a]**

| Residue | $H_N$ | $H_\alpha$ | $H_\beta$ | Others | |
|---|---|---|---|---|---|
| Y1 | nd[b] | 4.15 | 3.03 - 3.11 | Hδ | 7.08 |
| | | | | Hε | 6.81 |
| L2 | 8.57 (5.70)[c] | 4.25 | 1.54 | Hγ | 1.44 |
| | | | | Hδ | 0.80 - 0.85 |
| E3 | 8.61 (4.90) | 4.06 | 1.73 | Hγ | 2.21 |
| D4 | 8.37 (5.70) | 4.46 | 2.64 | | |
| W5 | 7.89 (5.40) | 4.53 | 3.30 | Hδ1 | 7.22 |
| | | | | Hε1 | 10.2 |
| | | | | Hζ2 | 7.43 |
| | | | | Hη2 | 7.19 |
| | | | | Hζ3 | 7.08 |
| | | | | Hε3 | 7.54 |
| I6 | 7.72 (6.70) | 3.79 | 1.64 | Hγ2 | 0.65 |
| | | | | Hγ1 | 0.91 - 1.20 |
| | | | | Hδ | 0.75 |
| K7 | 7.96 (6.30) | 4.08 | 1.59 | Hγ | 1.13 - 1.24 |
| | | | | Hδ | 1.60 |
| | | | | Hε | 2.90 |
| | | | | Hζ | 7.63 |
| Y8 | 8.05 (6.70) | 4.47 | 3.03 | Hδ | 7.07 |
| | | | | Hε | 6.77 |
| N9 | 8.26 (6.90) | 4.57 | 2.56 - 2.68 | | |
| N10 | 8.31 (7.30) | 4.62 | 2.75 - 2.79 | | |
| Q11 | 8.22 (6.90) | 4.27 | 1.97 - 2.10 | Hγ | 2.33 |
| K12 | 8.06 (7.20) | 4.11 | 1.67 - 1.79 | Hγ | 1.35 |
| | | | | Hδ | 1.62 |
| | | | | Hε | 2.93 |
| | | | | Hζ | 7.52 |

a) Chemical shifts measured in ppm with an accuracy of $\pm$ 0.01 ppm are referenced to external DSS ($\delta_H$ 0.00);

b) Not determined;

c) Data in parenthesis are $^3J_{HN,H\alpha}$ coupling constant (in Hz $\pm$ 0.25 Hz) measured from the 1D spectrum.

**Table 18: Statistics of peptide B1 NMR structures[1)]**

| | B1 | |
|---|---|---|
| Experimental restraints | | |
| All unambiguous NOEs | 271 | |
| Intraresidual | 113 | |
| Sequential (|i-j|=1) | 89 | |
| Short range (1<|i-j|≤5) | 62 | |
| Long range (|i-j|>5) | 7 | |
| Ambiguous NOEs 22 | | |
| RMSDs from experimental dihedral constraints [°][2)] | | |
| φ | (5) | 0.36 $\pm$ 0.37 |
| Number ifNOE upper bound violations | | |
| Violations > 0.5 Å | 0.0 $\pm$ 0.0 | |
| Violations > 0.3 Å | 0.1 $\pm$ 0.3 | |
| Violations > 0.1 Å | 0.7 $\pm$ 0.65 | |

(continued)

|  | B1 |
|---|---|
| Deviation from the idealized covalent geometry | |
| Bonds [Å] | $0.0028 \pm 0.0002$ |
| Angles [°] | $0.41 \pm 0.03$ |
| Impropers[3] [°] | $1.01 \pm 0.14$ |
| Energies [kcal/mol] | |
| Total | $-480 \pm 18$ |
| Van der Waals | $-92 \pm 10$ |
| Electrostatic | $-477 \pm 26$ |
| Coordinate precision [Å][4] | |
| Backbone | $0.53 \pm 0.18$ |
| Heavy atoms | $1.45 \pm 0.35$ |
| Percentage of residues in Ramachandran regions[5] | |
| Core | 82.0 |
| Allowed | 18.0 |
| Generous | 0.0 |
| Disallowed | 0.0 |

[1] The statistics were obtained from a subset of the 10 best energy water-refined structures following the protocol described in Materials and Methods. The number of the various constraints is given in parentheses.

[2] The dihedral angle constraints are inferred from $^3J_{HNH\alpha}$-couplings.

[3] The improper torsion restraints serve to maintain planarity and chirality.

[4] The coordinate precision is defined as the average RMSD to the mean coordinate structure. Values are reported for the most convergent amino acids Y1-N10.

[5] These values are calculated with the program PROCHECK-NMR[2].

**The pentasaccharide-IgGF22-4 complex observed by NMR**

TrNOEs derived distances allowed us to calculate structures of AB(E)CD and B(E)CDA when bound to F22-4 antibody. The obtained conformations of the B(E)CD fragment are similar in the two structures. The glycosidic dihedral angles are consistent with the favoured conformations proposed from MD simulations (Figures 23A-23B-23C-23D-23E). Although these structures are of rather poor resolution in the absence of experimental information on the complex formed with the antibody F22-4, we can state that glycosidic bonds E-C here adopt a conformation in situation $\Psi \approx +40°$.

The NMR-unfavourable kinetics parameters, (koff = 5s$^{-1}$) of the pentasaccharide FAb complexes associated to spectral characteristics of oligosaccharide renders the interpretation of STD spectra rather difficult. Nevertheless, substantially convergent results could be obtained, pointing out contacts between ECD fragment and the F22-4 recognition site, and to a lesser extent contacts with residue A but hardly any contact with B.

**The peptide-B1-IgGF22-4 complex observed by NMR**

STD-experiments revealed that, upon peptide B1 binding F22-4 interaction, close contacts involve aromatic and hydrophobic peptide residues Y1, L2, W5, I6, Y8 and the antibody (Figures 24A-24B). Indeed, side chain proton signals for these residues are clearly observable in the spectra. W5 displays the strongest STD intensity, followed by Y1, Y8, L2 and finally I6.

**ITC experiments**

ITC experiments with F22-4 antibody and several chemically synthesized oligosaccharidic fragments of O-Ag 2a lead to two major results.

Firstly, it is interesting to note that the ΔH and TΔS values for branched oligosaccharides binding to F22-4 fell on the same line in an enthalpy-entropy plot, with a slope of 1.3. The residues that are important for the recognition lead to a gain in enthalpy greater than the loss in entropy, which is not surprising for a specific antigen-antibody interaction.

Thus, the evolution of the $K_d$ is consistent with the observed number and types of contacts in the X-Ray structure. The non-reducing end residue A and the reducing end residue D' seem to hinder the recognition of the octasaccharidic motif of the O-Ag 2a (Figure 25) (cf. differences between B(E)CD and AB(E)CD and between B(E)CDAB(E)C and (AB(E)CD)$_2$).

Then the enthalpy-entropy plot of the $\Delta H$ and $T\Delta S$ of the F22-4/linear oligosaccharides interactions are not on the previously cited line (Figure 25). The fact that three compounds repeatedly show a 1.5-2 kcal.mol$^{-1}$ interval makes us confident in this result. Together with the fact that the residue B on the non-reducing side of the E-C motif does not create any contact with F22-4 in the X-ray structures, it means that the presence of residue B leads to a different conformational behaviour of the E-C glycosidic link, so that the enthalpic and/or entropic penalties for adopting the conformation recognised by F22-4 are weaker, consistent with the observed MD trajectories.

**Conclusions**

O-Ag recognition by F22-4.

In the X-ray structure of the pentadecasaccharide [AB(E)CD]$_3$:F22-4 antibody complex, the dihedral angles measured for the pentadecasaccharide are more or less located in frequently used positions observed in the MD simulation $\Phi$-$\Psi$ dihedral torsion angles maps of glycosidic bonds, except for E-C bond. Indeed, whereas the non-reducing end repetitive unit in that complex X-ray structure corresponds to situation $\Psi \approx +40°$, rarely adopted by the O-Ag, situation $\Psi \approx -25°$ is observed for the second repetitive unit (in the second part of the recognition site of the antibody). Moreover, situation $\Psi \approx +40°$ can occur all along the polysaccharidic chain when it is free in solution. This suggests that F22-4 could recognize O-Ag 2a all along its chain, even if the presence of a N-acetylglucosamine D at the non-reducing end appeared to scale down the affinity of O-Ag 2a oligosaccharidic fragments.

Crystal structure, NMR structural informations and MD simulations, are in rather good agreement, as the F22-4 antibody apparently selects MD simulation favoured conformations.

Pentasaccharide conformational behaviours and significance for recognition by F22-4.

All structures deduced from tr-NOE analysis of the pentasaccharides when bound to the antibodies correspond to the conformations of the non-reducing end in the X-Ray structure, so that we suggest that these pentasaccharides interact in the F22-4 binding site region that recognizes the non-reducing end of longer oligosaccharides. Together with STD information and with listed contacts in crystal structures, this argues for the overwhelmed importance of the ECD recognition "spot" of F22-4.

However, if we consider only the number of positive interactions in the crystal complexes, it is difficult to explain why the affinity of ECDAB -which is equivalent to ECDA'B' in the X-ray structure- for F22-4 is so weak. Two non-exclusive explanations can be put forward: enthalpic unfavourable events occur between B' and the antibody; higher enthalpy and/or entropy losses for one or for the two interacting molecules arise in complex ECDAB/F22-4 than in the complexes AB(E)CD/F22-4 and B(E)CDA/F22-4. In first instance, the MD simulation results seem to be coherent with the second possibility: E-C glycosidic bond is more flexible in ECDAB as compared to B(E)CDA and AB(E)CD when they are free in solution and ECDAB has a higher $K_D$, and the conformation of E-C glycosidic link in complex is frequently adopted in solution by B(E)CDA and AB(E)CD and not by ECDAB. A MD simulation of the complex would probably permit to clarify this matter, thanks to the evaluation of enthalpic and entropic weights in the free energies of the previously cited interactions.

Carbohydrate mimetic peptide B1 conformational behaviour and significance for the recognition by F22-4

The peptide B1 displays in solution a $\alpha$-helical structure between amino acids 5 and 8 and leading to a well-defined structure from residues 3 to 10. Looking at the backbone, this pre-organized region adopts a conformation very similar to bound conformation. This could explain partly the measured affinity between this peptide and the mAb F22-4. However this could be a disadvantage for the recognition of B1 by other mAbs, which may recognize other conformational epitopes of the O-Ag 2a. We cannot augur of this question before similar studies have been conducted with other isolated anti-O-Ag 2a mAbs.

Design ideas

These structural descriptions provide substantial informations for the design of O-Ag mimetic molecules great.

Roughly, we are confident that the different molecules (oligosaccharides fragment of the O-Ag and peptide B1) studied here are structural epitopes, i.e. similar groups of the mAb F22-4 bind the O-Ag and these molecules. It confirms that we focus on promising mimics.

More acutely, the knowledge of the conformational epitope(s) is of overwhelmed importance, since polysaccharides are very flexible molecules. In this perspective, a good appreciation of the conformational behaviours of leading molecules free in solution is useful, as these behaviours determine partly the efficiency of the mimicry. Besides, the mapping of the antigen binding-site of a protective monoclonal antibody is obviously a great resource for the design of mimotopes of O-Ag 2a.

i) Oligosaccharides approach

Maximizing the favourable interactions between the mimotope and the antibody and reducing the unfavourable ones, but also minimizing the conformational enthalpic and entropic losses of the mimic ligand when blocked in a peculiar conformation in the complex are the different means to obtain better affinities. The dynamic aspect of the conformational behaviour of oligosaccharides has probably to be taken into consideration in the design of a ligand, which would elicit F22-4 or F22-4 like-antibodies.

For example, we can think of an oligosaccharide-like fragment presenting a non-reducing end motif AB(E)CD, which would be chemically modified in order to obtain a glycosidic bond E-C always in situation 2. All positive contacts with F22-4 would be conserved, and the enthalpic and/or entropic losses would be diminished, converting the mechanism of recognition to a more key-lock-like mechanism.

More generally speaking, it would be interesting to find chemical ways to stabilize frequently used conformations of glycosidic linkages, in order to obtain better affinities with antibodies, if we consider that usually antibodies select conformations adopted by the oligosaccharides free in solution. It stands to evidence that all the protective antibodies directed against *S. flexneri* O-Ag 2a do not recognize the O-Ag in the same manner, in terms of sequence and of conformations, so that the two or three energetic minima for each glycosidic dihedral angle can be selected by other antibodies, a diversity highly desirable. Thus, it would be wise to limit this approach to glycosidic linkage E-C, which is the most determinant and rigid -i.e. displaying the weaker diversity of conformations- glycosidic link.

ii) Peptides approach

Although modifying oligosaccharides seems to be the "more immediate" method to obtain better immunogenic ligands, peptide mimics continue to represent an attractive approach. Indeed, numerous examples of peptides eliciting carbohydrate-binding antibody responses in murine models have been described. The success of this strategy depends on the ability of peptides to mimic oligosaccharidic epitopes, so that the detailed structure of the B1/F22-4 complex provides us very good starting point in designing carbohydrate structural peptidic mimicry.

We can speculate in the same manner that for the design of mimetic oligosaccharides, if we want to improve the affinity between the final mimotope and F22-4: we have to consider the favourable and unfavourable interactions between B1 and F22-4, and the enthalpic and entropic losses due to conformational chages upon binding to F22-4. Moreover, we have also to pay attention to the structural similarity of the interactions between the carbohydrate mimetic peptide and the antibody. We can foresee that a better similarity in the binding mode would imply a better O-Ag specific immune response. We may also have to think about the addition of chemical functions on peptides, in order to refine the structural mimicry, although it is likely to render the production of such a peptide approach more complicated.

iii) General considerations

Although it offers a magnificent tool for design, the various obtained X-ray structures would be even more interesting if structures could be obtained of other O-Ag 2a specific mAb already isolated. Indeed, it would allow us to compare the conformational epitopes, giving an access to a broader range of what is recognised by the immune system.

Then, following the oligosaccharide or the peptide approach, we could try to design versatile mimotopes, which would be good structural epitopes for various recognitions. This would be particularly important for the peptide approach, since it would show a high degree of carbohydrate peptidic mimicry, providing obviously an anti-O-Ag 2a immunogenic susceptibility. Thus the present results would be the first of an ensemble of complementary structures, which would lead us to a successful design.

Our considerations encounter two main drawbacks: i) the correlation between the mAb affinity, the antigenic mimicry and the immunogenic mimicry is not indubitable; ii) the efficiency of such strategies would be obviously dependant of the ability to be recognised at the different stages of maturation process leading to antibodies similar to F22-4 -i.e. recognizing the O-Ag 2a in a similar mode of binding that F22-4.

However, numerous encouraging examples of oligosaccharidic fragment of O-Ag, CPS or carbohydrate mimetic peptides that induce protective immune response have been published. Yet only one carbohydrate conjugate vaccines and no carbohydrate mimotope peptide conjugate vaccines have been approved and commercialized. This shows the difficulty of eliciting a significant and sufficiently focused anti-carbohydrate response. Access to structural information might be very helpful in view of amplifying the ability of designed ligands from the cited leading molecules to induce a specific immune response.

Finally, we have to mention examples of peptide conjugates, unable to elicit antipolysaccharide response, but used in a prime/boost strategy to enhance, focus or prolong the immune response to an already-present carbohydrate epitope (Beenhouwer, D.O., May, R. J., Valadon, P. & Scharff, M. D. (2002)J. Immunol. 169, 6992-6999) (Dharmasena, M. N., Jewell, D. A., Taylor R. K., (2007) J. Biol. Chem 282, 33805-16). Since most individuals living in endemic areas are likely to be exposed to the several serotypes of *S. flexneri* and their LPS, pre-existing antipolysaccharide responses could be prolonged in the concerned populations thanks to a carbohydrate mimetic peptide conjugated vaccine.

**EXAMPLE 7: Crystallographic study of the serotype 2a *S. flexneri* antigen-O-Ag mimetism by the B1 peptide: Examples of main interactions**

See Figures 26, 27, 28, 29 and 30.

## TABLE 13

### Crystal 1- 3CK3

Crystal structure of Fab F22-4 in complex with a carbohydrate-mimetic peptide

```
HEADER    IMMUNE SYSTEM                           14-MAR-08   3CK3
TITLE     CRYSTAL STRUCTURE OF FAB F22-4 IN COMPLEX WITH A
TITLE    2 CARBOHYDRATE-MIMETIC PEPTIDE
CRYST1   66.880   69.530  110.190  90.00  97.71  90.00 P 1 21 1      4
ATOM      1  N   ASP A   1      25.411 -25.245  67.865  1.00 33.16           N
ATOM      2  CA  ASP A   1      24.638 -24.132  67.238  1.00 31.38           C
ATOM      3  C   ASP A   1      23.300 -23.933  67.922  1.00 29.86           C
ATOM      4  O   ASP A   1      22.639 -24.884  68.369  1.00 32.58           O
ATOM      5  CB  ASP A   1      24.398 -24.389  65.762  1.00 31.41           C
ATOM      6  CG  ASP A   1      25.663 -24.818  65.028  1.00 30.94           C
ATOM      7  OD1 ASP A   1      26.795 -24.725  65.569  1.00 31.18           O
ATOM      8  OD2 ASP A   1      25.491 -25.252  63.896  1.00 33.06           O
ATOM      9  N   ILE A   2      22.881 -22.690  68.022  1.00 22.65           N
ATOM     10  CA  ILE A   2      21.618 -22.410  68.632  1.00 21.04           C
ATOM     11  C   ILE A   2      20.506 -22.541  67.621  1.00 20.73           C
ATOM     12  O   ILE A   2      20.554 -21.954  66.534  1.00 21.75           O
ATOM     13  CB  ILE A   2      21.628 -20.990  69.289  1.00 19.43           C
ATOM     14  CG1 ILE A   2      22.703 -20.976  70.384  1.00 21.44           C
ATOM     15  CG2 ILE A   2      20.253 -20.630  69.809  1.00 19.55           C
ATOM     16  CD1 ILE A   2      22.957 -19.613  71.036  1.00 22.66           C
ATOM     17  N   VAL A   3      19.471 -23.283  68.005  1.00 18.66           N
ATOM     18  CA  VAL A   3      18.243 -23.348  67.253  1.00 18.95           C
ATOM     19  C   VAL A   3      17.246 -22.325  67.779  1.00 20.97           C
ATOM     20  O   VAL A   3      16.953 -22.292  68.981  1.00 20.44           O
ATOM     21  CB  VAL A   3      17.587 -24.746  67.324  1.00 20.47           C
ATOM     22  CG1 VAL A   3      16.299 -24.815  66.547  1.00 19.95           C
ATOM     23  CG2 VAL A   3      18.570 -25.746  66.819  1.00 21.28           C
ATOM     24  N   MET A   4      16.751 -21.497  66.871  1.00 19.35           N
ATOM     25  CA  MET A   4      15.759 -20.492  67.204  1.00 19.27           C
ATOM     26  C   MET A   4      14.468 -20.980  66.608  1.00 21.10           C
ATOM     27  O   MET A   4      14.385 -21.192  65.391  1.00 20.00           O
ATOM     28  CB  MET A   4      16.159 -19.134  66.615  1.00 19.74           C
ATOM     29  CG  MET A   4      17.485 -18.637  67.060  1.00 18.69           C
ATOM     30  SD  MET A   4      17.593 -18.151  68.778  1.00 19.28           S
ATOM     31  CE  MET A   4      16.512 -16.745  68.881  1.00 18.10           C
ATOM     32  N   THR A   5      13.445 -21.175  67.446  1.00 22.14           N
ATOM     33  CA  THR A   5      12.198 -21.782  67.010  1.00 21.99           C
ATOM     34  C   THR A   5      11.042 -20.783  66.984  1.00 22.08           C
ATOM     35  O   THR A   5      10.703 -20.188  68.001  1.00 22.64           O
ATOM     36  CB  THR A   5      11.837 -23.012  67.899  1.00 21.19           C
ATOM     37  OG1 THR A   5      12.902 -23.958  67.851  1.00 24.16           O
ATOM     38  CG2 THR A   5      10.617 -23.702  67.389  1.00 24.99           C
ATOM     39  N   GLN A   6      10.436 -20.653  65.809  1.00 22.84           N
ATOM     40  CA  GLN A   6       9.229 -19.891  65.588  1.00 22.32           C
ATOM     41  C   GLN A   6       8.193 -20.669  64.775  1.00 24.00           C
ATOM     42  O   GLN A   6       8.527 -21.370  63.802  1.00 22.65           O
ATOM     43  CB  GLN A   6       9.518 -18.626  64.810  1.00 21.73           C
ATOM     44  CG  GLN A   6      10.565 -17.740  65.356  1.00 20.97           C
ATOM     45  CD  GLN A   6      10.674 -16.487  64.529  1.00 20.79           C
ATOM     46  OE1 GLN A   6      11.573 -16.340  63.711  1.00 21.87           O
ATOM     47  NE2 GLN A   6       9.715 -15.596  64.693  1.00 21.21           N
ATOM     48  N   ALA A   7       6.933 -20.496  65.142  1.00 26.04           N
ATOM     49  CA  ALA A   7       5.821 -20.990  64.333  1.00 27.29           C
ATOM     50  C   ALA A   7       5.712 -20.205  63.019  1.00 26.96           C
ATOM     51  O   ALA A   7       5.896 -18.982  62.963  1.00 26.78           O
ATOM     52  CB  ALA A   7       4.520 -20.908  65.113  1.00 27.94           C
ATOM     53  N   ALA A   8       5.398 -20.901  61.935  1.00 28.23           N
ATOM     54  CA  ALA A   8       5.315 -20.241  60.637  1.00 28.10           C
ATOM     55  C   ALA A   8       4.215 -19.166  60.558  1.00 27.89           C
```

34

```
ATOM   56  O    ALA A   8     4.345 -18.167 59.849  1.00 26.59           O
ATOM   57  CB   ALA A   8     5.159 -21.296 59.519  1.00 28.73           C
ATOM   58  N    PHE A   9     3.136 -19.369 61.315  1.00 30.66           N
ATOM   59  CA   PHE A   9     1.986 -18.471 61.304  1.00 32.67           C
ATOM   60  C    PHE A   9     1.471 -18.149 62.704  1.00 33.46           C
ATOM   61  O    PHE A   9     1.798 -18.814 63.681  1.00 31.88           O
ATOM   62  CB   PHE A   9     0.837 -19.075 60.494  1.00 34.55           C
ATOM   63  CG   PHE A   9     1.226 -19.462 59.118  1.00 35.04           C
ATOM   64  CD1  PHE A   9     1.267 -18.514 58.105  1.00 36.68           C
ATOM   65  CD2  PHE A   9     1.603 -20.766 58.840  1.00 37.01           C
ATOM   66  CE1  PHE A   9     1.650 -18.872 56.816  1.00 36.65           C
ATOM   67  CE2  PHE A   9     1.979 -21.144 57.554  1.00 38.10           C
ATOM   68  CZ   PHE A   9     2.010 -20.193 56.539  1.00 37.94           C
ATOM   69  N    SER A  10     0.666 -17.103 62.770  1.00 35.44           N
ATOM   70  CA   SER A  10    -0.005 -16.695 64.003  1.00 37.33           C
ATOM   71  C    SER A  10    -1.507 -16.675 63.727  1.00 38.28           C
ATOM   72  O    SER A  10    -1.921 -16.739 62.566  1.00 38.36           O
ATOM   73  CB   SER A  10     0.483 -15.310 64.397  1.00 37.01           C
ATOM   74  OG   SER A  10     0.681 -14.545 63.237  1.00 38.37           O
ATOM   75  N    ASN A  11    -2.325 -16.593 64.783  1.00 39.19           N
ATOM   76  CA   ASN A  11    -3.776 -16.447 64.594  1.00 39.84           C
ATOM   77  C    ASN A  11    -4.024 -15.123 63.885  1.00 37.95           C
ATOM   78  O    ASN A  11    -3.369 -14.123 64.203  1.00 37.66           O
ATOM   79  CB   ASN A  11    -4.543 -16.489 65.932  1.00 42.20           C
ATOM   80  CG   ASN A  11    -4.761 -17.918 66.459  1.00 45.31           C
ATOM   81  OD1  ASN A  11    -5.790 -18.213 67.092  1.00 49.03           O
ATOM   82  ND2  ASN A  11    -3.793 -18.801 66.216  1.00 47.58           N
ATOM   83  N    PRO A  12    -4.923 -15.109 62.888  1.00 36.41           N
ATOM   84  CA   PRO A  12    -5.170 -13.816 62.273  1.00 36.16           C
ATOM   85  C    PRO A  12    -5.583 -12.782 63.301  1.00 34.65           C
ATOM   86  O    PRO A  12    -6.235 -13.107 64.291  1.00 34.07           O
ATOM   87  CB   PRO A  12    -6.273 -14.109 61.251  1.00 36.39           C
ATOM   88  CG   PRO A  12    -6.096 -15.551 60.931  1.00 35.97           C
ATOM   89  CD   PRO A  12    -5.678 -16.180 62.218  1.00 35.79           C
ATOM   90  N    VAL A  13    -5.160 -11.550 63.082  1.00 33.98           N
ATOM   91  CA   VAL A  13    -5.386 -10.491 64.052  1.00 33.20           C
ATOM   92  C    VAL A  13    -6.258  -9.436 63.403  1.00 32.75           C
ATOM   93  O    VAL A  13    -6.013  -9.033 62.285  1.00 32.74           O
ATOM   94  CB   VAL A  13    -4.023  -9.868 64.572  1.00 32.36           C
ATOM   95  CG1  VAL A  13    -3.214  -9.229 63.453  1.00 32.41           C
ATOM   96  CG2  VAL A  13    -4.257  -8.867 65.645  1.00 32.18           C
ATOM   97  N    THR A  14    -7.299  -9.008 64.109  1.00 32.44           N
ATOM   98  CA   THR A  14    -8.111  -7.900 63.659  1.00 32.14           C
ATOM   99  C    THR A  14    -7.347  -6.587 63.773  1.00 31.94           C
ATOM  100  O    THR A  14    -6.616  -6.352 64.735  1.00 31.71           O
ATOM  101  CB   THR A  14    -9.401  -7.873 64.480  1.00 32.57           C
ATOM  102  OG1  THR A  14   -10.141  -9.073 64.193  1.00 33.22           O
ATOM  103  CG2  THR A  14   -10.223  -6.667 64.154  1.00 33.84           C
ATOM  104  N    LEU A  15    -7.528  -5.715 62.802  1.00 32.36           N
ATOM  105  CA   LEU A  15    -6.939  -4.390 62.867  1.00 33.32           C
ATOM  106  C    LEU A  15    -7.265  -3.667 64.161  1.00 33.72           C
ATOM  107  O    LEU A  15    -8.393  -3.694 64.630  1.00 34.02           O
ATOM  108  CB   LEU A  15    -7.423  -3.521 61.719  1.00 34.20           C
ATOM  109  CG   LEU A  15    -6.936  -3.946 60.343  1.00 36.83           C
ATOM  110  CD1  LEU A  15    -7.727  -3.169 59.305  1.00 38.80           C
ATOM  111  CD2  LEU A  15    -5.447  -3.670 60.231  1.00 37.14           C
ATOM  112  N    GLY A  16    -6.263  -2.999 64.715  1.00 33.53           N
ATOM  113  CA   GLY A  16    -6.426  -2.249 65.955  1.00 32.84           C
ATOM  114  C    GLY A  16    -6.271  -3.069 67.212  1.00 33.14           C
ATOM  115  O    GLY A  16    -6.254  -2.513 68.312  1.00 35.49           O
ATOM  116  N    THR A  17    -6.160  -4.384 67.083  1.00 30.95           N
ATOM  117  CA   THR A  17    -5.980  -5.240 68.248  1.00 31.11           C
ATOM  118  C    THR A  17    -4.533  -5.729 68.325  1.00 30.41           C
ATOM  119  O    THR A  17    -3.718  -5.449 67.439  1.00 28.79           O
ATOM  120  CB   THR A  17    -6.933  -6.445 68.241  1.00 31.77           C
```

```
ATOM    121  OG1 THR A   17      -6.593   -7.308   67.163  1.00 30.37           O
ATOM    122  CG2 THR A   17      -8.371   -6.014   68.054  1.00 33.52           C
ATOM    123  N   SER A   18      -4.230   -6.465   69.392  1.00 29.57           N
ATOM    124  CA  SER A   18      -2.866   -6.863   69.679  1.00 29.41           C
ATOM    125  C   SER A   18      -2.509   -8.227   69.070  1.00 29.47           C
ATOM    126  O   SER A   18      -3.280   -9.195   69.187  1.00 28.43           O
ATOM    127  CB  SER A   18      -2.647   -6.881   71.191  1.00 30.70           C
ATOM    128  OG  SER A   18      -1.347   -7.323   71.532  1.00 32.20           O
ATOM    129  N   ALA A   19      -1.329   -8.284   68.440  1.00 28.22           N
ATOM    130  CA  ALA A   19      -0.736   -9.548   67.965  1.00 27.64           C
ATOM    131  C   ALA A   19       0.499   -9.912   68.781  1.00 27.06           C
ATOM    132  O   ALA A   19       1.257   -9.024   69.120  1.00 25.73           O
ATOM    133  CB  ALA A   19      -0.369   -9.416   66.518  1.00 27.40           C
ATOM    134  N   SER A   20       0.689  -11.203   69.091  1.00 26.41           N
ATOM    135  CA  SER A   20       1.878  -11.686   69.794  1.00 27.78           C
ATOM    136  C   SER A   20       2.629  -12.722   68.916  1.00 28.43           C
ATOM    137  O   SER A   20       2.004  -13.627   68.376  1.00 30.50           O
ATOM    138  CB  SER A   20       1.501  -12.324   71.140  1.00 28.41           C
ATOM    139  OG  SER A   20       0.929  -11.378   72.061  1.00 27.64           O
ATOM    140  N   ILE A   21       3.932  -12.522   68.734  1.00 26.77           N
ATOM    141  CA  ILE A   21       4.786  -13.423   67.931  1.00 26.94           C
ATOM    142  C   ILE A   21       5.830  -13.995   68.859  1.00 26.06           C
ATOM    143  O   ILE A   21       6.504  -13.261   69.570  1.00 25.74           O
ATOM    144  CB  ILE A   21       5.415  -12.667   66.734  1.00 27.53           C
ATOM    145  CG1 ILE A   21       4.302  -12.319   65.730  1.00 29.26           C
ATOM    146  CG2 ILE A   21       6.524  -13.484   66.064  1.00 26.76           C
ATOM    147  CD1 ILE A   21       4.678  -11.308   64.741  1.00 32.78           C
ATOM    148  N   SER A   22       5.962  -15.321   68.887  1.00 24.82           N
ATOM    149  CA  SER A   22       6.807  -15.965   69.868  1.00 26.36           C
ATOM    150  C   SER A   22       8.043  -16.558   69.227  1.00 24.55           C
ATOM    151  O   SER A   22       8.066  -16.809   68.030  1.00 24.57           O
ATOM    152  CB  SER A   22       6.036  -17.068   70.585  1.00 28.78           C
ATOM    153  OG  SER A   22       5.227  -16.469   71.582  1.00 34.39           O
ATOM    154  N   CYS A   23       9.047  -16.769   70.055  1.00 23.16           N
ATOM    155  CA  CYS A   23      10.300  -17.330   69.646  1.00 22.98           C
ATOM    156  C   CYS A   23      10.871  -18.002   70.853  1.00 24.23           C
ATOM    157  O   CYS A   23      10.630  -17.553   71.981  1.00 23.77           O
ATOM    158  CB  CYS A   23      11.251  -16.244   69.141  1.00 23.09           C
ATOM    159  SG  CYS A   23      12.891  -16.861   68.782  1.00 24.13           S
ATOM    160  N   ARG A   24      11.600  -19.089   70.644  1.00 24.40           N
ATOM    161  CA  ARG A   24      12.363  -19.680   71.732  1.00 25.63           C
ATOM    162  C   ARG A   24      13.750  -20.061   71.254  1.00 24.24           C
ATOM    163  O   ARG A   24      13.991  -20.313   70.066  1.00 24.13           O
ATOM    164  CB  ARG A   24      11.609  -20.852   72.387  1.00 27.27           C
ATOM    165  CG  ARG A   24      10.971  -21.818   71.433  1.00 31.53           C
ATOM    166  CD  ARG A   24       9.999  -22.795   72.113  1.00 35.13           C
ATOM    167  NE  ARG A   24       9.787  -23.983   71.274  1.00 35.68           N
ATOM    168  CZ  ARG A   24      10.676  -24.966   71.123  1.00 37.54           C
ATOM    169  NH1 ARG A   24      11.861  -24.918   71.736  1.00 40.05           N
ATOM    170  NH2 ARG A   24      10.393  -25.998   70.331  1.00 37.47           N
ATOM    171  N   SER A   25      14.682  -20.095   72.173  1.00 23.19           N
ATOM    172  CA  SER A   25      16.032  -20.503   71.844  1.00 23.50           C
ATOM    173  C   SER A   25      16.398  -21.741   72.618  1.00 23.79           C
ATOM    174  O   SER A   25      15.926  -21.951   73.733  1.00 25.84           O
ATOM    175  CB  SER A   25      17.020  -19.409   72.170  1.00 23.36           C
ATOM    176  OG  SER A   25      16.848  -19.031   73.520  1.00 24.21           O
ATOM    177  N   SER A   26      17.296  -22.532   72.041  1.00 24.42           N
ATOM    178  CA  SER A   26      17.739  -23.781   72.627  1.00 24.54           C
ATOM    179  C   SER A   26      18.796  -23.545   73.721  1.00 24.10           C
ATOM    180  O   SER A   26      19.185  -24.491   74.418  1.00 25.87           O
ATOM    181  CB  SER A   26      18.305  -24.678   71.526  1.00 24.08           C
ATOM    182  OG  SER A   26      19.401  -24.052   70.877  1.00 22.87           O
ATOM    183  N   LYS A   27      19.290  -22.305   73.838  1.00 23.41           N
ATOM    184  CA  LYS A   27      20.237  -21.948   74.880  1.00 24.21           C
ATOM    185  C   LYS A   27      19.814  -20.608   75.414  1.00 23.26           C
```

```
ATOM    186  O    LYS A  27      19.291 -19.785  74.673  1.00 24.65           O
ATOM    187  CB   LYS A  27      21.662 -21.785  74.330  1.00 25.45           C
ATOM    188  CG   LYS A  27      22.348 -23.041  73.801  1.00 29.69           C
ATOM    189  CD   LYS A  27      23.841 -22.793  73.631  1.00 30.57           C
ATOM    190  CE   LYS A  27      24.479 -23.738  72.573  1.00 32.34           C
ATOM    191  NZ   LYS A  27      25.885 -23.328  72.184  1.00 33.28           N
ATOM    192  N    SER A  27A     20.079 -20.356  76.693  1.00 23.05           N
ATOM    193  CA   SER A  27A     19.808 -19.020  77.236  1.00 21.22           C
ATOM    194  C    SER A  27A     20.636 -17.951  76.529  1.00 19.87           C
ATOM    195  O    SER A  27A     21.853 -18.119  76.309  1.00 19.79           O
ATOM    196  CB   SER A  27A     20.082 -18.931  78.744  1.00 23.45           C
ATOM    197  OG   SER A  27A     19.734 -17.630  79.225  1.00 22.94           O
ATOM    198  N    LEU A  27B     19.969 -16.831  76.256  1.00 19.96           N
ATOM    199  CA   LEU A  27B     20.585 -15.656  75.642  1.00 19.71           C
ATOM    200  C    LEU A  27B     20.897 -14.571  76.653  1.00 20.59           C
ATOM    201  O    LEU A  27B     21.397 -13.491  76.301  1.00 19.69           O
ATOM    202  CB   LEU A  27B     19.684 -15.107  74.545  1.00 19.25           C
ATOM    203  CG   LEU A  27B     19.316 -16.160  73.486  1.00 20.24           C
ATOM    204  CD1  LEU A  27B     18.594 -15.466  72.421  1.00 19.77           C
ATOM    205  CD2  LEU A  27B     20.524 -16.880  72.941  1.00 19.38           C
ATOM    206  N    LEU A  27C     20.608 -14.860  77.917  1.00 21.17           N
ATOM    207  CA   LEU A  27C     21.018 -13.972  79.013  1.00 22.05           C
ATOM    208  C    LEU A  27C     22.530 -14.115  79.254  1.00 22.60           C
ATOM    209  O    LEU A  27C     23.056 -15.227  79.448  1.00 24.59           O
ATOM    210  CB   LEU A  27C     20.226 -14.322  80.271  1.00 22.63           C
ATOM    211  CG   LEU A  27C     20.631 -13.596  81.554  1.00 22.96           C
ATOM    212  CD1  LEU A  27C     20.434 -12.090  81.353  1.00 21.42           C
ATOM    213  CD2  LEU A  27C     19.832 -14.105  82.711  1.00 23.60           C
ATOM    214  N    HIS A  27D     23.217 -12.977  79.263  1.00 22.08           N
ATOM    215  CA   HIS A  27D     24.614 -12.895  79.560  1.00 22.14           C
ATOM    216  C    HIS A  27D     24.852 -12.363  80.978  1.00 22.40           C
ATOM    217  O    HIS A  27D     23.948 -11.840  81.610  1.00 23.02           O
ATOM    218  CB   HIS A  27D     25.325 -12.007  78.562  1.00 22.21           C
ATOM    219  CG   HIS A  27D     26.813 -12.094  78.649  1.00 21.96           C
ATOM    220  ND1  HIS A  27D     27.619 -10.982  78.667  1.00 24.59           N
ATOM    221  CD2  HIS A  27D     27.640 -13.158  78.745  1.00 24.00           C
ATOM    222  CE1  HIS A  27D     28.883 -11.353  78.735  1.00 25.15           C
ATOM    223  NE2  HIS A  27D     28.926 -12.671  78.781  1.00 26.73           N
ATOM    224  N    SER A  27E     26.075 -12.537  81.451  1.00 25.90           N
ATOM    225  CA   SER A  27E     26.466 -12.150  82.811  1.00 26.92           C
ATOM    226  C    SER A  27E     26.442 -10.644  83.048  1.00 26.11           C
ATOM    227  O    SER A  27E     26.464 -10.217  84.215  1.00 27.44           O
ATOM    228  CB   SER A  27E     27.837 -12.716  83.125  1.00 27.23           C
ATOM    229  OG   SER A  27E     28.782 -12.282  82.186  1.00 29.81           O
ATOM    230  N    ASP A  28      26.393  -9.855  81.965  1.00 23.71           N
ATOM    231  CA   ASP A  28      26.167  -8.410  82.016  1.00 23.46           C
ATOM    232  C    ASP A  28      24.715  -8.001  82.230  1.00 21.39           C
ATOM    233  O    ASP A  28      24.404  -6.818  82.283  1.00 25.38           O
ATOM    234  CB   ASP A  28      26.752  -7.671  80.786  1.00 22.50           C
ATOM    235  CG   ASP A  28      26.071  -8.039  79.475  1.00 22.93           C
ATOM    236  OD1  ASP A  28      25.123  -8.843  79.524  1.00 22.50           O
ATOM    237  OD2  ASP A  28      26.526  -7.520  78.425  1.00 21.90           O
ATOM    238  N    GLY A  29      23.816  -8.961  82.361  1.00 22.81           N
ATOM    239  CA   GLY A  29      22.432  -8.641  82.607  1.00 21.60           C
ATOM    240  C    GLY A  29      21.627  -8.375  81.359  1.00 21.94           C
ATOM    241  O    GLY A  29      20.430  -8.166  81.448  1.00 22.89           O
ATOM    242  N    ILE A  30      22.282  -8.411  80.203  1.00 22.14           N
ATOM    243  CA   ILE A  30      21.612  -8.184  78.930  1.00 20.15           C
ATOM    244  C    ILE A  30      21.263  -9.545  78.306  1.00 19.89           C
ATOM    245  O    ILE A  30      22.023 -10.531  78.409  1.00 20.81           O
ATOM    246  CB   ILE A  30      22.519  -7.390  77.964  1.00 22.11           C
ATOM    247  CG1  ILE A  30      22.834  -5.988  78.524  1.00 22.21           C
ATOM    248  CG2  ILE A  30      21.879  -7.304  76.597  1.00 22.22           C
ATOM    249  CD1  ILE A  30      23.961  -5.254  77.775  1.00 22.72           C
ATOM    250  N    THR A  31      20.116  -9.574  77.659  1.00 19.55           N
```

| ATOM | 251 | CA | THR | A | 31 | 19.654 | -10.775 | 76.942 | 1.00 | 18.26 | C |
|------|-----|-----|-----|---|----|--------|---------|--------|------|-------|---|
| ATOM | 252 | C | THR | A | 31 | 19.744 | -10.416 | 75.468 | 1.00 | 18.41 | C |
| ATOM | 253 | O | THR | A | 31 | 19.044 | -9.509 | 75.003 | 1.00 | 17.27 | O |
| ATOM | 254 | CB | THR | A | 31 | 18.223 | -11.121 | 77.341 | 1.00 | 18.84 | C |
| ATOM | 255 | OG1 | THR | A | 31 | 18.140 | -11.278 | 78.777 | 1.00 | 18.95 | O |
| ATOM | 256 | CG2 | THR | A | 31 | 17.752 | -12.386 | 76.611 | 1.00 | 19.94 | C |
| ATOM | 257 | N | TYR | A | 32 | 20.635 | -11.112 | 74.755 | 1.00 | 17.58 | N |
| ATOM | 258 | CA | TYR | A | 32 | 21.030 | -10.731 | 73.398 | 1.00 | 17.34 | C |
| ATOM | 259 | C | TYR | A | 32 | 20.077 | -11.352 | 72.361 | 1.00 | 18.30 | C |
| ATOM | 260 | O | TYR | A | 32 | 20.470 | -12.223 | 71.553 | 1.00 | 16.57 | O |
| ATOM | 261 | CB | TYR | A | 32 | 22.487 | -11.118 | 73.208 | 1.00 | 17.74 | C |
| ATOM | 262 | CG | TYR | A | 32 | 23.444 | -10.227 | 73.980 | 1.00 | 18.83 | C |
| ATOM | 263 | CD1 | TYR | A | 32 | 23.718 | -10.427 | 75.329 | 1.00 | 19.54 | C |
| ATOM | 264 | CD2 | TYR | A | 32 | 24.058 | -9.174 | 73.353 | 1.00 | 18.44 | C |
| ATOM | 265 | CE1 | TYR | A | 32 | 24.576 | -9.573 | 76.005 | 1.00 | 22.11 | C |
| ATOM | 266 | CE2 | TYR | A | 32 | 24.915 | -8.307 | 74.022 | 1.00 | 21.59 | C |
| ATOM | 267 | CZ | TYR | A | 32 | 25.170 | -8.504 | 75.331 | 1.00 | 19.98 | C |
| ATOM | 268 | OH | TYR | A | 32 | 26.061 | -7.644 | 75.920 | 1.00 | 22.47 | O |
| ATOM | 269 | N | LEU | A | 33 | 18.818 | -10.918 | 72.431 | 1.00 | 18.58 | N |
| ATOM | 270 | CA | LEU | A | 33 | 17.729 | -11.375 | 71.571 | 1.00 | 18.36 | C |
| ATOM | 271 | C | LEU | A | 33 | 17.302 | -10.180 | 70.739 | 1.00 | 18.22 | C |
| ATOM | 272 | O | LEU | A | 33 | 17.093 | -9.091 | 71.265 | 1.00 | 18.31 | O |
| ATOM | 273 | CB | LEU | A | 33 | 16.557 | -11.918 | 72.406 | 1.00 | 18.32 | C |
| ATOM | 274 | CG | LEU | A | 33 | 15.233 | -12.386 | 71.802 | 1.00 | 18.47 | C |
| ATOM | 275 | CD1 | LEU | A | 33 | 14.400 | -11.281 | 71.243 | 1.00 | 18.60 | C |
| ATOM | 276 | CD2 | LEU | A | 33 | 15.461 | -13.471 | 70.714 | 1.00 | 19.92 | C |
| ATOM | 277 | N | TYR | A | 34 | 17.140 | -10.403 | 69.440 | 1.00 | 18.82 | N |
| ATOM | 278 | CA | TYR | A | 34 | 16.804 | -9.368 | 68.470 | 1.00 | 18.21 | C |
| ATOM | 279 | C | TYR | A | 34 | 15.588 | -9.800 | 67.635 | 1.00 | 18.67 | C |
| ATOM | 280 | O | TYR | A | 34 | 15.384 | -11.019 | 67.387 | 1.00 | 18.56 | O |
| ATOM | 281 | CB | TYR | A | 34 | 17.986 | -9.072 | 67.531 | 1.00 | 18.05 | C |
| ATOM | 282 | CG | TYR | A | 34 | 19.263 | -8.611 | 68.217 | 1.00 | 17.14 | C |
| ATOM | 283 | CD1 | TYR | A | 34 | 19.961 | -9.428 | 69.096 | 1.00 | 17.43 | C |
| ATOM | 284 | CD2 | TYR | A | 34 | 19.759 | -7.333 | 67.999 | 1.00 | 17.62 | C |
| ATOM | 285 | CE1 | TYR | A | 34 | 21.108 | -9.000 | 69.726 | 1.00 | 18.07 | C |
| ATOM | 286 | CE2 | TYR | A | 34 | 20.884 | -6.907 | 68.607 | 1.00 | 18.07 | C |
| ATOM | 287 | CZ | TYR | A | 34 | 21.560 | -7.730 | 69.519 | 1.00 | 18.45 | C |
| ATOM | 288 | OH | TYR | A | 34 | 22.751 | -7.306 | 70.138 | 1.00 | 19.07 | O |
| ATOM | 289 | N | TRP | A | 35 | 14.804 | -8.797 | 67.218 | 1.00 | 17.86 | N |
| ATOM | 290 | CA | TRP | A | 35 | 13.688 | -8.993 | 66.296 | 1.00 | 17.55 | C |
| ATOM | 291 | C | TRP | A | 35 | 13.859 | -8.191 | 65.033 | 1.00 | 16.76 | C |
| ATOM | 292 | O | TRP | A | 35 | 14.213 | -6.985 | 65.069 | 1.00 | 17.40 | O |
| ATOM | 293 | CB | TRP | A | 35 | 12.369 | -8.652 | 66.914 | 1.00 | 17.58 | C |
| ATOM | 294 | CG | TRP | A | 35 | 11.895 | -9.533 | 68.004 | 1.00 | 18.66 | C |
| ATOM | 295 | CD1 | TRP | A | 35 | 12.010 | -9.298 | 69.338 | 1.00 | 19.14 | C |
| ATOM | 296 | CD2 | TRP | A | 35 | 11.181 | -10.781 | 67.877 | 1.00 | 18.60 | C |
| ATOM | 297 | NE1 | TRP | A | 35 | 11.406 | -10.311 | 70.058 | 1.00 | 21.05 | N |
| ATOM | 298 | CE2 | TRP | A | 35 | 10.897 | -11.235 | 69.184 | 1.00 | 20.31 | C |
| ATOM | 299 | CE3 | TRP | A | 35 | 10.728 | -11.544 | 66.784 | 1.00 | 19.62 | C |
| ATOM | 300 | CZ2 | TRP | A | 35 | 10.187 | -12.419 | 69.436 | 1.00 | 21.21 | C |
| ATOM | 301 | CZ3 | TRP | A | 35 | 10.037 | -12.737 | 67.043 | 1.00 | 20.65 | C |
| ATOM | 302 | CH2 | TRP | A | 35 | 9.772 | -13.157 | 68.359 | 1.00 | 19.84 | C |
| ATOM | 303 | N | TYR | A | 36 | 13.572 | -8.840 | 63.904 | 1.00 | 17.68 | N |
| ATOM | 304 | CA | TYR | A | 36 | 13.588 | -8.193 | 62.586 | 1.00 | 17.21 | C |
| ATOM | 305 | C | TYR | A | 36 | 12.241 | -8.418 | 61.903 | 1.00 | 18.78 | C |
| ATOM | 306 | O | TYR | A | 36 | 11.590 | -9.428 | 62.130 | 1.00 | 18.65 | O |
| ATOM | 307 | CB | TYR | A | 36 | 14.687 | -8.791 | 61.665 | 1.00 | 18.01 | C |
| ATOM | 308 | CG | TYR | A | 36 | 16.048 | -8.632 | 62.228 | 1.00 | 15.79 | C |
| ATOM | 309 | CD1 | TYR | A | 36 | 16.571 | -9.577 | 63.112 | 1.00 | 17.62 | C |
| ATOM | 310 | CD2 | TYR | A | 36 | 16.835 | -7.539 | 61.886 | 1.00 | 15.52 | C |
| ATOM | 311 | CE1 | TYR | A | 36 | 17.805 | -9.412 | 63.671 | 1.00 | 16.04 | C |
| ATOM | 312 | CE2 | TYR | A | 36 | 18.101 | -7.372 | 62.422 | 1.00 | 14.59 | C |
| ATOM | 313 | CZ | TYR | A | 36 | 18.589 | -8.289 | 63.321 | 1.00 | 18.61 | C |
| ATOM | 314 | OH | TYR | A | 36 | 19.832 | -8.122 | 63.864 | 1.00 | 17.52 | O |
| ATOM | 315 | N | LEU | A | 37 | 11.864 | -7.477 | 61.052 | 1.00 | 19.26 | N |

| ATOM | 316 | CA  | LEU | A | 37 | 10.717 | -7.580  | 60.169 | 1.00 | 19.28 | C |
| ATOM | 317 | C   | LEU | A | 37 | 11.159 | -7.514  | 58.716 | 1.00 | 19.68 | C |
| ATOM | 318 | O   | LEU | A | 37 | 11.906 | -6.610  | 58.306 | 1.00 | 21.10 | O |
| ATOM | 319 | CB  | LEU | A | 37 | 9.726  | -6.445  | 60.443 | 1.00 | 20.09 | C |
| ATOM | 320 | CG  | LEU | A | 37 | 8.548  | -6.265  | 59.487 | 1.00 | 20.46 | C |
| ATOM | 321 | CD1 | LEU | A | 37 | 7.718  | -7.530  | 59.415 | 1.00 | 21.63 | C |
| ATOM | 322 | CD2 | LEU | A | 37 | 7.688  | -5.095  | 59.976 | 1.00 | 21.45 | C |
| ATOM | 323 | N   | GLN | A | 38 | 10.675 | -8.490  | 57.957 | 1.00 | 21.67 | N |
| ATOM | 324 | CA  | GLN | A | 38 | 10.838 | -8.518  | 56.525 | 1.00 | 23.25 | C |
| ATOM | 325 | C   | GLN | A | 38 | 9.485  | -8.395  | 55.851 | 1.00 | 23.73 | C |
| ATOM | 326 | O   | GLN | A | 38 | 8.679  | -9.307  | 55.889 | 1.00 | 24.79 | O |
| ATOM | 327 | CB  | GLN | A | 38 | 11.482 | -9.817  | 56.072 | 1.00 | 22.31 | C |
| ATOM | 328 | CG  | GLN | A | 38 | 11.977 | -9.703  | 54.654 | 1.00 | 23.69 | C |
| ATOM | 329 | CD  | GLN | A | 38 | 12.728 | -10.919 | 54.214 | 1.00 | 23.12 | C |
| ATOM | 330 | OE1 | GLN | A | 38 | 12.481 | -12.036 | 54.684 | 1.00 | 24.42 | O |
| ATOM | 331 | NE2 | GLN | A | 38 | 13.632 | -10.716 | 53.304 | 1.00 | 20.83 | N |
| ATOM | 332 | N   | LYS | A | 39 | 9.286  | -7.258  | 55.219 | 1.00 | 27.51 | N |
| ATOM | 333 | CA  | LYS | A | 39 | 8.061  | -6.979  | 54.463 | 1.00 | 30.44 | C |
| ATOM | 334 | C   | LYS | A | 39 | 8.222  | -7.582  | 53.080 | 1.00 | 31.90 | C |
| ATOM | 335 | O   | LYS | A | 39 | 9.348  | -7.798  | 52.628 | 1.00 | 30.34 | O |
| ATOM | 336 | CB  | LYS | A | 39 | 7.785  | -5.472  | 54.419 | 1.00 | 30.69 | C |
| ATOM | 337 | CG  | LYS | A | 39 | 7.156  | -4.973  | 55.719 | 1.00 | 32.46 | C |
| ATOM | 338 | CD  | LYS | A | 39 | 6.895  | -3.481  | 55.750 | 1.00 | 33.34 | C |
| ATOM | 339 | CE  | LYS | A | 39 | 6.350  | -3.063  | 57.123 | 1.00 | 33.65 | C |
| ATOM | 340 | NZ  | LYS | A | 39 | 5.789  | -1.684  | 57.167 | 1.00 | 33.22 | N |
| ATOM | 341 | N   | PRO | A | 40 | 7.093  | -7.917  | 52.415 | 1.00 | 35.28 | N |
| ATOM | 342 | CA  | PRO | A | 40 | 7.196  | -8.486  | 51.064 | 1.00 | 36.87 | C |
| ATOM | 343 | C   | PRO | A | 40 | 8.021  | -7.632  | 50.107 | 1.00 | 37.46 | C |
| ATOM | 344 | O   | PRO | A | 40 | 7.829  | -6.419  | 50.039 | 1.00 | 38.92 | O |
| ATOM | 345 | CB  | PRO | A | 40 | 5.736  | -8.582  | 50.612 | 1.00 | 37.73 | C |
| ATOM | 346 | CG  | PRO | A | 40 | 4.958  | -8.699  | 51.891 | 1.00 | 36.47 | C |
| ATOM | 347 | CD  | PRO | A | 40 | 5.695  | -7.851  | 52.880 | 1.00 | 36.27 | C |
| ATOM | 348 | N   | GLY | A | 41 | 8.963  | -8.271  | 49.420 | 1.00 | 37.75 | N |
| ATOM | 349 | CA  | GLY | A | 41 | 9.821  | -7.613  | 48.435 | 1.00 | 38.12 | C |
| ATOM | 350 | C   | GLY | A | 41 | 11.006 | -6.862  | 49.008 | 1.00 | 38.54 | C |
| ATOM | 351 | O   | GLY | A | 41 | 11.739 | -6.196  | 48.278 | 1.00 | 40.49 | O |
| ATOM | 352 | N   | GLN | A | 42 | 11.211 | -6.982  | 50.319 | 1.00 | 36.92 | N |
| ATOM | 353 | CA  | GLN | A | 42 | 12.206 | -6.186  | 51.022 | 1.00 | 35.77 | C |
| ATOM | 354 | C   | GLN | A | 42 | 13.169 | -7.048  | 51.827 | 1.00 | 32.72 | C |
| ATOM | 355 | O   | GLN | A | 42 | 12.996 | -8.268  | 51.971 | 1.00 | 31.47 | O |
| ATOM | 356 | CB  | GLN | A | 42 | 11.502 | -5.199  | 51.949 | 1.00 | 37.28 | C |
| ATOM | 357 | CG  | GLN | A | 42 | 10.363 | -4.448  | 51.267 | 1.00 | 39.31 | C |
| ATOM | 358 | CD  | GLN | A | 42 | 10.229 | -3.045  | 51.741 | 1.00 | 41.03 | C |
| ATOM | 359 | OE1 | GLN | A | 42 | 9.141  | -2.590  | 52.100 | 1.00 | 42.96 | O |
| ATOM | 360 | NE2 | GLN | A | 42 | 11.340 | -2.323  | 51.730 | 1.00 | 44.61 | N |
| ATOM | 361 | N   | SER | A | 43 | 14.205 | -6.407  | 52.331 | 1.00 | 31.15 | N |
| ATOM | 362 | CA  | SER | A | 43 | 15.125 | -7.089  | 53.245 | 1.00 | 31.12 | C |
| ATOM | 363 | C   | SER | A | 43 | 14.623 | -7.043  | 54.690 | 1.00 | 27.95 | C |
| ATOM | 364 | O   | SER | A | 43 | 13.691 | -6.298  | 55.036 | 1.00 | 27.26 | O |
| ATOM | 365 | CB  | SER | A | 43 | 16.521 | -6.500  | 53.159 | 1.00 | 32.42 | C |
| ATOM | 366 | OG  | SER | A | 43 | 16.601 | -5.289  | 53.872 | 1.00 | 37.02 | O |
| ATOM | 367 | N   | PRO | A | 44 | 15.222 | -7.860  | 55.557 | 1.00 | 25.72 | N |
| ATOM | 368 | CA  | PRO | A | 44 | 14.820 | -7.686  | 56.950 | 1.00 | 23.61 | C |
| ATOM | 369 | C   | PRO | A | 44 | 15.238 | -6.307  | 57.460 | 1.00 | 23.23 | C |
| ATOM | 370 | O   | PRO | A | 44 | 16.159 | -5.699  | 56.905 | 1.00 | 24.07 | O |
| ATOM | 371 | CB  | PRO | A | 44 | 15.554 | -8.835  | 57.676 | 1.00 | 23.77 | C |
| ATOM | 372 | CG  | PRO | A | 44 | 15.956 | -9.787  | 56.537 | 1.00 | 24.37 | C |
| ATOM | 373 | CD  | PRO | A | 44 | 16.213 | -8.925  | 55.388 | 1.00 | 24.70 | C |
| ATOM | 374 | N   | HIS | A | 45 | 14.536 | -5.775  | 58.440 | 1.00 | 21.03 | N |
| ATOM | 375 | CA  | HIS | A | 45 | 14.978 | -4.549  | 59.113 | 1.00 | 22.01 | C |
| ATOM | 376 | C   | HIS | A | 45 | 14.771 | -4.691  | 60.607 | 1.00 | 19.92 | C |
| ATOM | 377 | O   | HIS | A | 45 | 13.859 | -5.370  | 61.044 | 1.00 | 19.71 | O |
| ATOM | 378 | CB  | HIS | A | 45 | 14.362 | -3.249  | 58.543 | 1.00 | 26.05 | C |
| ATOM | 379 | CG  | HIS | A | 45 | 12.902 | -3.042  | 58.829 | 1.00 | 29.19 | C |
| ATOM | 380 | ND1 | HIS | A | 45 | 11.903 | -3.411  | 57.944 | 1.00 | 35.44 | N |

```
ATOM    381  CD2 HIS A  45    12.275   -2.427   59.859  1.00 33.12        C
ATOM    382  CE1 HIS A  45    10.726   -3.081   58.446  1.00 34.75        C
ATOM    383  NE2 HIS A  45    10.923   -2.471   59.602  1.00 34.61        N
ATOM    384  N   LEU A  46    15.697   -4.101   61.358  1.00 19.85        N
ATOM    385  CA  LEU A  46    15.764   -4.291   62.808  1.00 18.58        C
ATOM    386  C   LEU A  46    14.643   -3.578   63.538  1.00 18.22        C
ATOM    387  O   LEU A  46    14.433   -2.376   63.310  1.00 19.58        O
ATOM    388  CB  LEU A  46    17.113   -3.793   63.328  1.00 19.28        C
ATOM    389  CG  LEU A  46    17.315   -3.853   64.836  1.00 18.87        C
ATOM    390  CD1 LEU A  46    17.526   -5.252   65.333  1.00 19.47        C
ATOM    391  CD2 LEU A  46    18.451   -2.983   65.261  1.00 19.20        C
ATOM    392  N   LEU A  47    13.944   -4.302   64.419  1.00 19.34        N
ATOM    393  CA  LEU A  47    12.863   -3.710   65.241  1.00 18.91        C
ATOM    394  C   LEU A  47    13.266   -3.544   66.693  1.00 18.29        C
ATOM    395  O   LEU A  47    13.021   -2.506   67.323  1.00 19.55        O
ATOM    396  CB  LEU A  47    11.609   -4.567   65.212  1.00 20.23        C
ATOM    397  CG  LEU A  47    10.835   -4.720   63.916  1.00 21.85        C
ATOM    398  CD1 LEU A  47     9.682   -5.680   64.136  1.00 24.12        C
ATOM    399  CD2 LEU A  47    10.377   -3.394   63.407  1.00 22.79        C
ATOM    400  N   ILE A  48    13.823   -4.604   67.246  1.00 18.43        N
ATOM    401  CA  ILE A  48    14.153   -4.643   68.648  1.00 18.20        C
ATOM    402  C   ILE A  48    15.536   -5.199   68.843  1.00 18.89        C
ATOM    403  O   ILE A  48    15.895   -6.206   68.214  1.00 17.35        O
ATOM    404  CB  ILE A  48    13.200   -5.620   69.396  1.00 17.53        C
ATOM    405  CG1 ILE A  48    11.739   -5.206   69.265  1.00 21.99        C
ATOM    406  CG2 ILE A  48    13.628   -5.861   70.830  1.00 19.06        C
ATOM    407  CD1 ILE A  48    11.366   -3.969   69.970  1.00 19.62        C
ATOM    408  N   TYR A  49    16.274   -4.638   69.800  1.00 17.87        N
ATOM    409  CA  TYR A  49    17.552   -5.199   70.236  1.00 17.58        C
ATOM    410  C   TYR A  49    17.592   -5.407   71.762  1.00 17.67        C
ATOM    411  O   TYR A  49    16.797   -4.811   72.506  1.00 17.90        O
ATOM    412  CB  TYR A  49    18.717   -4.345   69.803  1.00 17.34        C
ATOM    413  CG  TYR A  49    18.748   -2.990   70.443  1.00 19.15        C
ATOM    414  CD1 TYR A  49    17.995   -1.941   69.920  1.00 21.23        C
ATOM    415  CD2 TYR A  49    19.549   -2.754   71.543  1.00 19.67        C
ATOM    416  CE1 TYR A  49    18.023   -0.665   70.507  1.00 23.02        C
ATOM    417  CE2 TYR A  49    19.592   -1.479   72.141  1.00 20.60        C
ATOM    418  CZ  TYR A  49    18.833   -0.444   71.586  1.00 22.31        C
ATOM    419  OH  TYR A  49    18.887    0.795   72.188  1.00 23.65        O
ATOM    420  N   HIS A  50    18.446   -6.331   72.189  1.00 17.41        N
ATOM    421  CA  HIS A  50    18.635   -6.643   73.610  1.00 18.22        C
ATOM    422  C   HIS A  50    17.287   -6.898   74.314  1.00 19.84        C
ATOM    423  O   HIS A  50    16.955   -6.285   75.350  1.00 20.15        O
ATOM    424  CB  HIS A  50    19.362   -5.485   74.300  1.00 17.52        C
ATOM    425  CG  HIS A  50    20.778   -5.298   73.863  1.00 18.94        C
ATOM    426  ND1 HIS A  50    21.541   -4.240   74.309  1.00 19.29        N
ATOM    427  CD2 HIS A  50    21.575   -6.017   73.040  1.00 20.74        C
ATOM    428  CE1 HIS A  50    22.749   -4.315   73.778  1.00 20.67        C
ATOM    429  NE2 HIS A  50    22.778   -5.359   72.967  1.00 20.27        N
ATOM    430  N   LEU A  51    16.487   -7.783   73.702  1.00 19.18        N
ATOM    431  CA  LEU A  51    15.214   -8.274   74.241  1.00 19.95        C
ATOM    432  C   LEU A  51    14.021   -7.304   74.138  1.00 20.00        C
ATOM    433  O   LEU A  51    12.948   -7.694   73.743  1.00 18.59        O
ATOM    434  CB  LEU A  51    15.382   -8.779   75.704  1.00 19.19        C
ATOM    435  CG  LEU A  51    14.206   -9.579   76.268  1.00 19.65        C
ATOM    436  CD1 LEU A  51    14.006  -10.834   75.431  1.00 22.89        C
ATOM    437  CD2 LEU A  51    14.423   -9.955   77.697  1.00 19.12        C
ATOM    438  N   SER A  52    14.188   -6.040   74.529  1.00 20.02        N
ATOM    439  CA  SER A  52    13.046   -5.185   74.671  1.00 20.88        C
ATOM    440  C   SER A  52    13.263   -3.760   74.194  1.00 20.26        C
ATOM    441  O   SER A  52    12.338   -2.945   74.251  1.00 22.04        O
ATOM    442  CB  SER A  52    12.633   -5.169   76.134  1.00 21.69        C
ATOM    443  OG  SER A  52    13.623   -4.507   76.903  1.00 22.66        O
ATOM    444  N   ASN A  53    14.454   -3.453   73.710  1.00 19.62        N
ATOM    445  CA  ASN A  53    14.715   -2.086   73.259  1.00 20.71        C
```

40

```
ATOM    446  C    ASN A  53      14.375  -1.839  71.808  1.00 20.61           C
ATOM    447  O    ASN A  53      14.809  -2.584  70.924  1.00 21.11           O
ATOM    448  CB   ASN A  53      16.149  -1.730  73.545  1.00 22.05           C
ATOM    449  CG   ASN A  53      16.461  -1.818  75.027  1.00 24.60           C
ATOM    450  OD1  ASN A  53      16.208  -0.888  75.765  1.00 26.75           O
ATOM    451  ND2  ASN A  53      16.918  -2.964  75.476  1.00 24.47           N
ATOM    452  N    LEU A  54      13.622  -0.766  71.582  1.00 20.50           N
ATOM    453  CA   LEU A  54      13.167  -0.398  70.258  1.00 21.58           C
ATOM    454  C    LEU A  54      14.274   0.298  69.487  1.00 21.94           C
ATOM    455  O    LEU A  54      14.965   1.194  70.010  1.00 22.50           O
ATOM    456  CB   LEU A  54      11.903   0.476  70.340  1.00 21.88           C
ATOM    457  CG   LEU A  54      10.569  -0.265  70.432  1.00 23.90           C
ATOM    458  CD1  LEU A  54      10.433  -0.961  71.771  1.00 24.64           C
ATOM    459  CD2  LEU A  54       9.423   0.679  70.226  1.00 24.66           C
ATOM    460  N    ALA A  55      14.466  -0.121  68.228  1.00 21.65           N
ATOM    461  CA   ALA A  55      15.382   0.535  67.338  1.00 20.34           C
ATOM    462  C    ALA A  55      14.819   1.903  66.984  1.00 21.55           C
ATOM    463  O    ALA A  55      13.613   2.133  67.095  1.00 20.46           O
ATOM    464  CB   ALA A  55      15.612  -0.325  66.098  1.00 20.57           C
ATOM    465  N    SER A  56      15.706   2.794  66.597  1.00 22.95           N
ATOM    466  CA   SER A  56      15.315   4.176  66.314  1.00 25.44           C
ATOM    467  C    SER A  56      14.220   4.201  65.276  1.00 25.98           C
ATOM    468  O    SER A  56      14.303   3.513  64.274  1.00 27.43           O
ATOM    469  CB   SER A  56      16.510   4.965  65.825  1.00 28.24           C
ATOM    470  OG   SER A  56      16.157   6.344  65.759  1.00 30.66           O
ATOM    471  N    GLY A  57      13.176   4.993  65.512  1.00 27.13           N
ATOM    472  CA   GLY A  57      12.137   5.176  64.507  1.00 26.31           C
ATOM    473  C    GLY A  57      11.074   4.095  64.496  1.00 25.88           C
ATOM    474  O    GLY A  57      10.051   4.265  63.863  1.00 26.39           O
ATOM    475  N    VAL A  58      11.279   2.997  65.221  1.00 26.39           N
ATOM    476  CA   VAL A  58      10.226   1.995  65.369  1.00 26.62           C
ATOM    477  C    VAL A  58       9.050   2.507  66.231  1.00 27.91           C
ATOM    478  O    VAL A  58       9.250   2.948  67.352  1.00 27.39           O
ATOM    479  CB   VAL A  58      10.789   0.714  65.985  1.00 25.48           C
ATOM    480  CG1  VAL A  58       9.669  -0.319  66.270  1.00 25.12           C
ATOM    481  CG2  VAL A  58      11.833   0.128  65.038  1.00 23.00           C
ATOM    482  N    PRO A  59       7.809   2.387  65.724  1.00 31.36           N
ATOM    483  CA   PRO A  59       6.670   2.859  66.502  1.00 32.36           C
ATOM    484  C    PRO A  59       6.497   2.176  67.863  1.00 32.49           C
ATOM    485  O    PRO A  59       6.792   1.003  68.011  1.00 30.28           O
ATOM    486  CB   PRO A  59       5.466   2.560  65.602  1.00 33.30           C
ATOM    487  CG   PRO A  59       6.009   2.278  64.250  1.00 33.47           C
ATOM    488  CD   PRO A  59       7.416   1.833  64.416  1.00 32.37           C
ATOM    489  N    ASP A  60       6.002   2.942  68.831  1.00 34.13           N
ATOM    490  CA   ASP A  60       5.643   2.467  70.176  1.00 35.22           C
ATOM    491  C    ASP A  60       4.575   1.373  70.198  1.00 33.69           C
ATOM    492  O    ASP A  60       4.333   0.753  71.218  1.00 34.12           O
ATOM    493  CB   ASP A  60       5.175   3.654  71.045  1.00 37.52           C
ATOM    494  CG   ASP A  60       4.101   4.508  70.366  1.00 39.87           C
ATOM    495  OD1  ASP A  60       3.430   4.022  69.416  1.00 41.86           O
ATOM    496  OD2  ASP A  60       3.928   5.682  70.785  1.00 43.98           O
ATOM    497  N    ARG A  61       3.942   1.127  69.065  1.00 34.23           N
ATOM    498  CA   ARG A  61       3.057  -0.016  68.917  1.00 33.06           C
ATOM    499  C    ARG A  61       3.816  -1.345  69.167  1.00 31.62           C
ATOM    500  O    ARG A  61       3.235  -2.315  69.630  1.00 29.78           O
ATOM    501  CB   ARG A  61       2.393   0.002  67.539  1.00 35.03           C
ATOM    502  CG   ARG A  61       2.072   1.431  67.037  1.00 38.45           C
ATOM    503  CD   ARG A  61       1.183   1.450  65.830  1.00 39.18           C
ATOM    504  NE   ARG A  61       1.910   1.311  64.566  1.00 39.31           N
ATOM    505  CZ   ARG A  61       2.086   0.160  63.915  1.00 38.47           C
ATOM    506  NH1  ARG A  61       1.614  -0.968  64.424  1.00 38.86           N
ATOM    507  NH2  ARG A  61       2.726   0.148  62.747  1.00 38.14           N
ATOM    508  N    PHE A  62       5.128  -1.349  68.910  1.00 29.51           N
ATOM    509  CA   PHE A  62       5.942  -2.565  69.086  1.00 27.43           C
ATOM    510  C    PHE A  62       6.576  -2.642  70.467  1.00 26.87           C
```

```
ATOM    511  O    PHE A  62      7.155  -1.671  70.911  1.00 27.04          O
ATOM    512  CB   PHE A  62      7.046  -2.610  68.029  1.00 27.14          C
ATOM    513  CG   PHE A  62      6.533  -2.717  66.632  1.00 27.09          C
ATOM    514  CD1  PHE A  62      6.290  -1.583  65.877  1.00 25.72          C
ATOM    515  CD2  PHE A  62      6.262  -3.956  66.077  1.00 26.73          C
ATOM    516  CE1  PHE A  62      5.799  -1.675  64.586  1.00 25.62          C
ATOM    517  CE2  PHE A  62      5.760  -4.055  64.785  1.00 26.89          C
ATOM    518  CZ   PHE A  62      5.542  -2.919  64.035  1.00 25.20          C
ATOM    519  N    SER A  63      6.488  -3.803  71.119  1.00 25.31          N
ATOM    520  CA   SER A  63      7.214  -4.049  72.348  1.00 25.13          C
ATOM    521  C    SER A  63      7.600  -5.506  72.419  1.00 24.21          C
ATOM    522  O    SER A  63      7.074  -6.321  71.663  1.00 24.64          O
ATOM    523  CB   SER A  63      6.379  -3.689  73.584  1.00 25.34          C
ATOM    524  OG   SER A  63      5.179  -4.426  73.696  1.00 27.18          O
ATOM    525  N    SER A  64      8.475  -5.841  73.343  1.00 22.78          N
ATOM    526  CA   SER A  64      8.973  -7.229  73.423  1.00 22.88          C
ATOM    527  C    SER A  64      9.445  -7.559  74.837  1.00 22.48          C
ATOM    528  O    SER A  64      9.996  -6.700  75.503  1.00 21.14          O
ATOM    529  CB   SER A  64     10.129  -7.396  72.416  1.00 22.40          C
ATOM    530  OG   SER A  64     10.643  -8.728  72.438  1.00 27.13          O
ATOM    531  N    SER A  65      9.251  -8.796  75.319  1.00 21.44          N
ATOM    532  CA   SER A  65      9.955  -9.255  76.545  1.00 23.26          C
ATOM    533  C    SER A  65     10.202 -10.746  76.401  1.00 22.63          C
ATOM    534  O    SER A  65     10.066 -11.296  75.306  1.00 23.15          O
ATOM    535  CB   SER A  65      9.217  -8.945  77.882  1.00 23.78          C
ATOM    536  OG   SER A  65     10.114  -8.837  79.029  1.00 21.13          O
ATOM    537  N    GLY A  66     10.653 -11.356  77.482  1.00 22.58          N
ATOM    538  CA   GLY A  66     11.011 -12.739  77.502  1.00 22.25          C
ATOM    539  C    GLY A  66     11.888 -13.083  78.667  1.00 21.14          C
ATOM    540  O    GLY A  66     12.251 -12.211  79.458  1.00 22.23          O
ATOM    541  N    SER A  67     12.159 -14.371  78.772  1.00 21.27          N
ATOM    542  CA   SER A  67     13.123 -14.947  79.692  1.00 21.57          C
ATOM    543  C    SER A  67     14.486 -15.019  78.981  1.00 21.42          C
ATOM    544  O    SER A  67     14.699 -14.293  78.034  1.00 22.70          O
ATOM    545  CB   SER A  67     12.625 -16.322  80.129  1.00 22.45          C
ATOM    546  OG   SER A  67     12.449 -17.177  79.010  1.00 22.29          O
ATOM    547  N    GLY A  68     15.391 -15.903  79.390  1.00 23.38          N
ATOM    548  CA   GLY A  68     16.598 -16.160  78.606  1.00 22.03          C
ATOM    549  C    GLY A  68     16.337 -17.049  77.376  1.00 21.69          C
ATOM    550  O    GLY A  68     17.151 -17.113  76.488  1.00 22.32          O
ATOM    551  N    THR A  69     15.207 -17.759  77.367  1.00 21.05          N
ATOM    552  CA   THR A  69     14.911 -18.754  76.347  1.00 20.44          C
ATOM    553  C    THR A  69     13.588 -18.621  75.615  1.00 20.49          C
ATOM    554  O    THR A  69     13.383 -19.272  74.582  1.00 20.50          O
ATOM    555  CB   THR A  69     14.987 -20.192  76.924  1.00 22.71          C
ATOM    556  OG1  THR A  69     14.032 -20.346  77.967  1.00 22.76          O
ATOM    557  CG2  THR A  69     16.356 -20.486  77.488  1.00 23.82          C
ATOM    558  N    ASP A  70     12.659 -17.827  76.114  1.00 19.93          N
ATOM    559  CA   ASP A  70     11.308 -17.746  75.540  1.00 20.77          C
ATOM    560  C    ASP A  70     10.950 -16.322  75.398  1.00 22.17          C
ATOM    561  O    ASP A  70     10.977 -15.582  76.395  1.00 25.48          O
ATOM    562  CB   ASP A  70     10.238 -18.343  76.445  1.00 24.15          C
ATOM    563  CG   ASP A  70     10.311 -19.831  76.536  1.00 26.56          C
ATOM    564  OD1  ASP A  70     10.222 -20.506  75.493  1.00 29.18          O
ATOM    565  OD2  ASP A  70     10.455 -20.331  77.674  1.00 34.16          O
ATOM    566  N    PHE A  71     10.604 -15.906  74.191  1.00 20.56          N
ATOM    567  CA   PHE A  71     10.475 -14.476  73.920  1.00 20.18          C
ATOM    568  C    PHE A  71      9.204 -14.195  73.169  1.00 22.16          C
ATOM    569  O    PHE A  71      8.697 -15.055  72.423  1.00 22.74          O
ATOM    570  CB   PHE A  71     11.671 -13.961  73.114  1.00 19.08          C
ATOM    571  CG   PHE A  71     12.935 -14.662  73.403  1.00 19.66          C
ATOM    572  CD1  PHE A  71     13.682 -14.388  74.526  1.00 17.57          C
ATOM    573  CD2  PHE A  71     13.388 -15.639  72.535  1.00 20.14          C
ATOM    574  CE1  PHE A  71     14.871 -15.058  74.780  1.00 19.07          C
ATOM    575  CE2  PHE A  71     14.559 -16.287  72.767  1.00 19.09          C
```

```
          ATOM   576  CZ   PHE A  71     15.314 -16.011  73.884  1.00 20.14           C
          ATOM   577  N    THR A  72      8.687 -12.989  73.355  1.00 21.36           N
          ATOM   578  CA   THR A  72      7.454 -12.583  72.714  1.00 22.32           C
          ATOM   579  C    THR A  72      7.495 -11.131  72.236  1.00 23.00           C
          ATOM   580  O    THR A  72      7.754 -10.206  73.007  1.00 23.80           O
          ATOM   581  CB   THR A  72      6.191 -12.823  73.617  1.00 23.16           C
          ATOM   582  OG1  THR A  72      6.126 -14.201  74.040  1.00 23.79           O
          ATOM   583  CG2  THR A  72      4.956 -12.439  72.868  1.00 23.18           C
          ATOM   584  N    LEU A  73      7.267 -10.950  70.940  1.00 22.94           N
          ATOM   585  CA   LEU A  73      7.087  -9.638  70.323  1.00 23.71           C
          ATOM   586  C    LEU A  73      5.592  -9.354  70.292  1.00 25.07           C
          ATOM   587  O    LEU A  73      4.795 -10.224  69.908  1.00 25.21           O
          ATOM   588  CB   LEU A  73      7.644  -9.595  68.888  1.00 24.39           C
          ATOM   589  CG   LEU A  73      7.397  -8.338  68.056  1.00 23.44           C
          ATOM   590  CD1  LEU A  73      8.223  -7.189  68.625  1.00 24.51           C
          ATOM   591  CD2  LEU A  73      7.792  -8.546  66.620  1.00 24.34           C
          ATOM   592  N    ARG A  74      5.209  -8.159  70.753  1.00 25.25           N
          ATOM   593  CA   ARG A  74      3.817  -7.719  70.757  1.00 25.97           C
          ATOM   594  C    ARG A  74      3.689  -6.556  69.814  1.00 25.66           C
          ATOM   595  O    ARG A  74      4.487  -5.635  69.873  1.00 26.10           O
          ATOM   596  CB   ARG A  74      3.354  -7.222  72.159  1.00 25.39           C
          ATOM   597  CG   ARG A  74      1.833  -6.924  72.269  1.00 27.70           C
          ATOM   598  CD   ARG A  74      1.483  -6.224  73.581  1.00 28.33           C
          ATOM   599  NE   ARG A  74      0.042  -5.997  73.723  1.00 30.33           N
          ATOM   600  CZ   ARG A  74     -0.539  -5.388  74.750  1.00 32.80           C
          ATOM   601  NH1  ARG A  74      0.191  -4.943  75.764  1.00 34.04           N
          ATOM   602  NH2  ARG A  74     -1.862  -5.258  74.773  1.00 33.01           N
          ATOM   603  N    ILE A  75      2.669  -6.601  68.957  1.00 26.14           N
          ATOM   604  CA   ILE A  75      2.246  -5.452  68.149  1.00 26.72           C
          ATOM   605  C    ILE A  75      0.866  -5.110  68.707  1.00 27.82           C
          ATOM   606  O    ILE A  75     -0.096  -5.837  68.501  1.00 26.20           O
          ATOM   607  CB   ILE A  75      2.137  -5.767  66.636  1.00 28.05           C
          ATOM   608  CG1  ILE A  75      3.401  -6.517  66.155  1.00 28.84           C
          ATOM   609  CG2  ILE A  75      1.897  -4.469  65.878  1.00 27.96           C
          ATOM   610  CD1  ILE A  75      3.412  -6.931  64.653  1.00 30.28           C
          ATOM   611  N    SER A  76      0.790  -4.041  69.487  1.00 29.33           N
          ATOM   612  CA   SER A  76     -0.369  -3.852  70.376  1.00 31.25           C
          ATOM   613  C    SER A  76     -1.641  -3.416  69.648  1.00 33.09           C
          ATOM   614  O    SER A  76     -2.752  -3.792  70.071  1.00 35.37           O
          ATOM   615  CB   SER A  76     -0.037  -2.833  71.474  1.00 30.51           C
          ATOM   616  OG   SER A  76      0.188  -1.572  70.885  1.00 31.73           O
          ATOM   617  N    ARG A  77     -1.474  -2.607  68.600  1.00 33.94           N
          ATOM   618  CA   ARG A  77     -2.590  -2.103  67.784  1.00 33.85           C
          ATOM   619  C    ARG A  77     -2.238  -2.279  66.325  1.00 32.89           C
          ATOM   620  O    ARG A  77     -1.752  -1.356  65.683  1.00 32.40           O
          ATOM   621  CB   ARG A  77     -2.828  -0.612  68.047  1.00 36.46           C
          ATOM   622  CG   ARG A  77     -3.418  -0.321  69.419  1.00 40.06           C
          ATOM   623  CD   ARG A  77     -3.208   1.135  69.837  1.00 42.75           C
          ATOM   624  NE   ARG A  77     -3.405   1.280  71.279  1.00 45.32           N
          ATOM   625  CZ   ARG A  77     -4.589   1.294  71.891  1.00 46.87           C
          ATOM   626  NH1  ARG A  77     -5.724   1.212  71.201  1.00 48.40           N
          ATOM   627  NH2  ARG A  77     -4.639   1.420  73.213  1.00 48.10           N
          ATOM   628  N    VAL A  78     -2.484  -3.467  65.793  1.00 31.64           N
          ATOM   629  CA   VAL A  78     -1.975  -3.783  64.446  1.00 31.98           C
          ATOM   630  C    VAL A  78     -2.584  -2.902  63.344  1.00 31.37           C
          ATOM   631  O    VAL A  78     -3.790  -2.588  63.348  1.00 29.98           O
          ATOM   632  CB   VAL A  78     -2.158  -5.255  64.113  1.00 32.07           C
          ATOM   633  CG1  VAL A  78     -1.849  -5.519  62.664  1.00 33.78           C
          ATOM   634  CG2  VAL A  78     -1.234  -6.079  64.983  1.00 31.35           C
          ATOM   635  N    GLU A  79     -1.725  -2.524  62.408  1.00 30.57           N
          ATOM   636  CA   GLU A  79     -2.120  -1.808  61.208  1.00 31.13           C
          ATOM   637  C    GLU A  79     -1.905  -2.712  59.993  1.00 30.98           C
          ATOM   638  O    GLU A  79     -1.147  -3.674  60.047  1.00 29.87           O
          ATOM   639  CB   GLU A  79     -1.300  -0.538  61.080  1.00 31.98           C
          ATOM   640  CG   GLU A  79     -1.428   0.353  62.320  1.00 34.70           C
```

43

```
ATOM    641  CD   GLU A  79     -0.676    1.657   62.242  1.00 36.83           C
ATOM    642  OE1  GLU A  79     -0.138    2.000   61.167  1.00 39.96           O
ATOM    643  OE2  GLU A  79     -0.620    2.337   63.286  1.00 39.42           O
ATOM    644  N    ALA A  80     -2.532   -2.372   58.877  1.00 30.71           N
ATOM    645  CA   ALA A  80     -2.472   -3.239   57.704  1.00 31.36           C
ATOM    646  C    ALA A  80     -1.050   -3.394   57.161  1.00 30.57           C
ATOM    647  O    ALA A  80     -0.683   -4.478   56.677  1.00 30.44           O
ATOM    648  CB   ALA A  80     -3.410   -2.730   56.621  1.00 31.91           C
ATOM    649  N    GLU A  81     -0.255   -2.339   57.293  1.00 30.08           N
ATOM    650  CA   GLU A  81      1.129   -2.283   56.794  1.00 30.71           C
ATOM    651  C    GLU A  81      2.095   -3.168   57.580  1.00 29.19           C
ATOM    652  O    GLU A  81      3.243   -3.356   57.183  1.00 29.50           O
ATOM    653  CB   GLU A  81      1.641   -0.823   56.789  1.00 32.32           C
ATOM    654  CG   GLU A  81      2.070   -0.239   58.189  1.00 36.50           C
ATOM    655  CD   GLU A  81      2.146    1.314   58.265  1.00 40.16           C
ATOM    656  OE1  GLU A  81      1.092    2.004   58.430  1.00 44.40           O
ATOM    657  OE2  GLU A  81      3.279    1.849   58.210  1.00 43.93           O
ATOM    658  N    ASP A  82      1.645   -3.722   58.698  1.00 27.34           N
ATOM    659  CA   ASP A  82      2.522   -4.536   59.528  1.00 27.29           C
ATOM    660  C    ASP A  82      2.624   -5.970   59.000  1.00 25.15           C
ATOM    661  O    ASP A  82      3.384   -6.760   59.535  1.00 25.81           O
ATOM    662  CB   ASP A  82      2.038   -4.546   60.984  1.00 27.01           C
ATOM    663  CG   ASP A  82      1.978   -3.165   61.604  1.00 27.51           C
ATOM    664  OD1  ASP A  82      2.812   -2.301   61.280  1.00 25.73           O
ATOM    665  OD2  ASP A  82      1.098   -2.972   62.466  1.00 29.08           O
ATOM    666  N    VAL A  83      1.836   -6.335   57.984  1.00 25.69           N
ATOM    667  CA   VAL A  83      1.976   -7.670   57.410  1.00 24.84           C
ATOM    668  C    VAL A  83      3.413   -7.872   56.936  1.00 23.30           C
ATOM    669  O    VAL A  83      4.055   -6.954   56.405  1.00 21.43           O
ATOM    670  CB   VAL A  83      0.996   -7.969   56.229  1.00 26.15           C
ATOM    671  CG1  VAL A  83     -0.406   -7.953   56.706  1.00 27.57           C
ATOM    672  CG2  VAL A  83      1.193   -7.017   55.059  1.00 27.11           C
ATOM    673  N    GLY A  84      3.910   -9.085   57.166  1.00 22.38           N
ATOM    674  CA   GLY A  84      5.295   -9.425   56.877  1.00 20.88           C
ATOM    675  C    GLY A  84      5.695  -10.630   57.673  1.00 21.43           C
ATOM    676  O    GLY A  84      4.869  -11.253   58.320  1.00 22.74           O
ATOM    677  N    ILE A  85      6.978  -10.963   57.643  1.00 20.76           N
ATOM    678  CA   ILE A  85      7.495  -12.080   58.417  1.00 21.60           C
ATOM    679  C    ILE A  85      8.436  -11.496   59.473  1.00 20.73           C
ATOM    680  O    ILE A  85      9.297  -10.669   59.149  1.00 22.16           O
ATOM    681  CB   ILE A  85      8.273  -13.042   57.511  1.00 21.85           C
ATOM    682  CG1  ILE A  85      7.364  -13.568   56.405  1.00 23.93           C
ATOM    683  CG2  ILE A  85      8.821  -14.198   58.307  1.00 22.54           C
ATOM    684  CD1  ILE A  85      8.098  -14.303   55.309  1.00 25.54           C
ATOM    685  N    TYR A  86      8.248  -11.934   60.713  1.00 21.07           N
ATOM    686  CA   TYR A  86      8.980  -11.416   61.862  1.00 19.71           C
ATOM    687  C    TYR A  86      9.915  -12.519   62.290  1.00 19.09           C
ATOM    688  O    TYR A  86      9.457  -13.641   62.539  1.00 19.90           O
ATOM    689  CB   TYR A  86      8.016  -11.046   62.993  1.00 19.70           C
ATOM    690  CG   TYR A  86      7.158   -9.851   62.664  1.00 21.20           C
ATOM    691  CD1  TYR A  86      5.987   -9.997   61.924  1.00 21.70           C
ATOM    692  CD2  TYR A  86      7.525   -8.571   63.086  1.00 22.70           C
ATOM    693  CE1  TYR A  86      5.211   -8.897   61.571  1.00 22.58           C
ATOM    694  CE2  TYR A  86      6.784   -7.455   62.749  1.00 19.95           C
ATOM    695  CZ   TYR A  86      5.609   -7.613   61.991  1.00 23.17           C
ATOM    696  OH   TYR A  86      4.836   -6.509   61.665  1.00 24.07           O
ATOM    697  N    TYR A  87     11.208  -12.189   62.351  1.00 18.88           N
ATOM    698  CA   TYR A  87     12.287  -13.109   62.680  1.00 18.75           C
ATOM    699  C    TYR A  87     12.976  -12.783   63.992  1.00 18.81           C
ATOM    700  O    TYR A  87     13.298  -11.622   64.244  1.00 19.89           O
ATOM    701  CB   TYR A  87     13.363  -13.025   61.608  1.00 17.64           C
ATOM    702  CG   TYR A  87     12.960  -13.515   60.254  1.00 19.57           C
ATOM    703  CD1  TYR A  87     13.034  -14.872   59.945  1.00 20.52           C
ATOM    704  CD2  TYR A  87     12.547  -12.660   59.283  1.00 21.68           C
ATOM    705  CE1  TYR A  87     12.665  -15.348   58.707  1.00 22.15           C
```

44

| ATOM | 706 | CE2 | TYR | A | 87 | 12.187 | -13.144 | 58.002 | 1.00 | 21.14 | C |
|------|-----|-----|-----|---|-----|--------|---------|--------|------|-------|---|
| ATOM | 707 | CZ | TYR | A | 87 | 12.252 | -14.475 | 57.735 | 1.00 | 23.38 | C |
| ATOM | 708 | OH | TYR | A | 87 | 11.899 | -14.911 | 56.456 | 1.00 | 22.27 | O |
| ATOM | 709 | N | CYS | A | 88 | 13.257 | -13.797 | 64.795 | 1.00 | 18.62 | N |
| ATOM | 710 | CA | CYS | A | 88 | 14.131 | -13.617 | 65.935 | 1.00 | 19.81 | C |
| ATOM | 711 | C | CYS | A | 88 | 15.556 | -14.068 | 65.600 | 1.00 | 20.14 | C |
| ATOM | 712 | O | CYS | A | 88 | 15.795 | -14.901 | 64.711 | 1.00 | 18.70 | O |
| ATOM | 713 | CB | CYS | A | 88 | 13.574 | -14.351 | 67.155 | 1.00 | 21.06 | C |
| ATOM | 714 | SG | CYS | A | 88 | 13.350 | -16.145 | 66.873 | 1.00 | 23.10 | S |
| ATOM | 715 | N | ALA | A | 89 | 16.515 | -13.474 | 66.301 | 1.00 | 18.36 | N |
| ATOM | 716 | CA | ALA | A | 89 | 17.906 | -13.842 | 66.185 | 1.00 | 19.40 | C |
| ATOM | 717 | C | ALA | A | 89 | 18.594 | -13.631 | 67.504 | 1.00 | 19.97 | C |
| ATOM | 718 | O | ALA | A | 89 | 18.196 | -12.748 | 68.266 | 1.00 | 19.23 | O |
| ATOM | 719 | CB | ALA | A | 89 | 18.593 | -12.984 | 65.128 | 1.00 | 20.17 | C |
| ATOM | 720 | N | HIS | A | 90 | 19.672 | -14.372 | 67.723 | 1.00 | 17.93 | N |
| ATOM | 721 | CA | HIS | A | 90 | 20.484 | -14.202 | 68.905 | 1.00 | 19.04 | C |
| ATOM | 722 | C | HIS | A | 90 | 21.762 | -13.484 | 68.495 | 1.00 | 18.77 | C |
| ATOM | 723 | O | HIS | A | 90 | 22.099 | -13.435 | 67.291 | 1.00 | 19.43 | O |
| ATOM | 724 | CB | HIS | A | 90 | 20.798 | -15.559 | 69.544 | 1.00 | 19.85 | C |
| ATOM | 725 | CG | HIS | A | 90 | 21.814 | -16.359 | 68.796 | 1.00 | 18.67 | C |
| ATOM | 726 | ND1 | HIS | A | 90 | 21.487 | -17.385 | 67.928 | 1.00 | 19.12 | N |
| ATOM | 727 | CD2 | HIS | A | 90 | 23.155 | -16.261 | 68.769 | 1.00 | 16.24 | C |
| ATOM | 728 | CE1 | HIS | A | 90 | 22.597 | -17.883 | 67.422 | 1.00 | 13.07 | C |
| ATOM | 729 | NE2 | HIS | A | 90 | 23.620 | -17.215 | 67.911 | 1.00 | 20.12 | N |
| ATOM | 730 | N | ASN | A | 91 | 22.497 | -12.995 | 69.496 | 1.00 | 19.58 | N |
| ATOM | 731 | CA | ASN | A | 91 | 23.848 | -12.481 | 69.305 | 1.00 | 18.66 | C |
| ATOM | 732 | C | ASN | A | 91 | 24.750 | -12.897 | 70.496 | 1.00 | 18.97 | C |
| ATOM | 733 | O | ASN | A | 91 | 25.332 | -12.052 | 71.169 | 1.00 | 17.54 | O |
| ATOM | 734 | CB | ASN | A | 91 | 23.780 | -10.939 | 69.128 | 1.00 | 17.95 | C |
| ATOM | 735 | CG | ASN | A | 91 | 25.128 | -10.271 | 68.843 | 1.00 | 18.52 | C |
| ATOM | 736 | OD1 | ASN | A | 91 | 25.293 | -9.074 | 69.182 | 1.00 | 21.80 | O |
| ATOM | 737 | ND2 | ASN | A | 91 | 26.108 | -11.005 | 68.293 | 1.00 | 17.42 | N |
| ATOM | 738 | N | VAL | A | 92 | 24.880 | -14.211 | 70.729 | 1.00 | 18.31 | N |
| ATOM | 739 | CA | VAL | A | 92 | 25.671 | -14.757 | 71.831 | 1.00 | 18.78 | C |
| ATOM | 740 | C | VAL | A | 92 | 26.825 | -15.633 | 71.422 | 1.00 | 18.47 | C |
| ATOM | 741 | O | VAL | A | 92 | 27.676 | -15.952 | 72.254 | 1.00 | 19.76 | O |
| ATOM | 742 | CB | VAL | A | 92 | 24.808 | -15.516 | 72.893 | 1.00 | 19.41 | C |
| ATOM | 743 | CG1 | VAL | A | 92 | 23.739 | -14.619 | 73.416 | 1.00 | 17.62 | C |
| ATOM | 744 | CG2 | VAL | A | 92 | 24.186 | -16.765 | 72.347 | 1.00 | 20.21 | C |
| ATOM | 745 | N | GLU | A | 93 | 26.850 | -16.032 | 70.147 | 1.00 | 18.53 | N |
| ATOM | 746 | CA | GLU | A | 93 | 27.915 | -16.822 | 69.602 | 1.00 | 18.84 | C |
| ATOM | 747 | C | GLU | A | 93 | 27.802 | -16.859 | 68.079 | 1.00 | 19.04 | C |
| ATOM | 748 | O | GLU | A | 93 | 26.822 | -16.358 | 67.505 | 1.00 | 20.29 | O |
| ATOM | 749 | CB | GLU | A | 93 | 27.769 | -18.251 | 70.106 | 1.00 | 19.75 | C |
| ATOM | 750 | CG | GLU | A | 93 | 26.501 | -18.928 | 69.664 | 1.00 | 21.65 | C |
| ATOM | 751 | CD | GLU | A | 93 | 26.454 | -20.384 | 70.137 | 1.00 | 23.67 | C |
| ATOM | 752 | OE1 | GLU | A | 93 | 26.857 | -20.625 | 71.304 | 1.00 | 28.54 | O |
| ATOM | 753 | OE2 | GLU | A | 93 | 26.015 | -21.274 | 69.361 | 1.00 | 24.34 | O |
| ATOM | 754 | N | LEU | A | 94 | 28.790 | -17.494 | 67.466 | 1.00 | 19.50 | N |
| ATOM | 755 | CA | LEU | A | 94 | 28.749 | -17.870 | 66.067 | 1.00 | 18.86 | C |
| ATOM | 756 | C | LEU | A | 94 | 28.576 | -19.377 | 66.038 | 1.00 | 20.43 | C |
| ATOM | 757 | O | LEU | A | 94 | 29.122 | -20.067 | 66.885 | 1.00 | 21.96 | O |
| ATOM | 758 | CB | LEU | A | 94 | 30.047 | -17.485 | 65.375 | 1.00 | 19.70 | C |
| ATOM | 759 | CG | LEU | A | 94 | 30.423 | -16.003 | 65.377 | 1.00 | 20.86 | C |
| ATOM | 760 | CD1 | LEU | A | 94 | 31.712 | -15.759 | 64.631 | 1.00 | 21.21 | C |
| ATOM | 761 | CD2 | LEU | A | 94 | 29.332 | -15.167 | 64.750 | 1.00 | 19.84 | C |
| ATOM | 762 | N | PRO | A | 95 | 27.836 | -19.880 | 65.060 | 1.00 | 19.88 | N |
| ATOM | 763 | CA | PRO | A | 95 | 27.233 | -19.169 | 63.948 | 1.00 | 19.14 | C |
| ATOM | 764 | C | PRO | A | 95 | 26.036 | -18.338 | 64.381 | 1.00 | 17.98 | C |
| ATOM | 765 | O | PRO | A | 95 | 25.361 | -18.671 | 65.349 | 1.00 | 17.08 | O |
| ATOM | 766 | CB | PRO | A | 95 | 26.790 | -20.317 | 63.046 | 1.00 | 19.60 | C |
| ATOM | 767 | CG | PRO | A | 95 | 26.543 | -21.425 | 63.969 | 1.00 | 21.00 | C |
| ATOM | 768 | CD | PRO | A | 95 | 27.494 | -21.317 | 65.050 | 1.00 | 20.37 | C |
| ATOM | 769 | N | ARG | A | 96 | 25.806 | -17.204 | 63.712 | 1.00 | 17.47 | N |
| ATOM | 770 | CA | ARG | A | 96 | 24.582 | -16.473 | 63.880 | 1.00 | 17.65 | C |

```
ATOM    771   C    ARG A   96     23.448 -17.303  63.320  1.00 17.22          C
ATOM    772   O    ARG A   96     23.605 -17.921  62.240  1.00 17.41          O
ATOM    773   CB   ARG A   96     24.675 -15.151  63.100  1.00 17.88          C
ATOM    774   CG   ARG A   96     25.925 -14.401  63.453  1.00 18.73          C
ATOM    775   CD   ARG A   96     25.810 -12.894  63.073  1.00 18.73          C
ATOM    776   NE   ARG A   96     27.013 -12.192  63.417  1.00 17.52          N
ATOM    777   CZ   ARG A   96     27.247 -11.678  64.623  1.00 17.57          C
ATOM    778   NH1  ARG A   96     26.304 -11.764  65.559  1.00 18.89          N
ATOM    779   NH2  ARG A   96     28.397 -11.059  64.886  1.00 19.37          N
ATOM    780   N    THR A   97     22.349 -17.377  64.050  1.00 16.77          N
ATOM    781   CA   THR A   97     21.179 -18.127  63.603  1.00 18.06          C
ATOM    782   C    THR A   97     19.920 -17.306  63.842  1.00 19.34          C
ATOM    783   O    THR A   97     19.891 -16.391  64.694  1.00 18.27          O
ATOM    784   CB   THR A   97     21.064 -19.575  64.239  1.00 20.48          C
ATOM    785   OG1  THR A   97     21.056 -19.498  65.661  1.00 19.29          O
ATOM    786   CG2  THR A   97     22.185 -20.437  63.832  1.00 19.03          C
ATOM    787   N    PHE A   98     18.904 -17.629  63.050  1.00 17.58          N
ATOM    788   CA   PHE A   98     17.650 -16.899  62.942  1.00 18.48          C
ATOM    789   C    PHE A   98     16.525 -17.886  63.126  1.00 17.95          C
ATOM    790   O    PHE A   98     16.661 -19.090  62.818  1.00 18.61          O
ATOM    791   CB   PHE A   98     17.497 -16.259  61.557  1.00 18.63          C
ATOM    792   CG   PHE A   98     18.520 -15.212  61.264  1.00 16.54          C
ATOM    793   CD1  PHE A   98     19.725 -15.540  60.679  1.00 17.35          C
ATOM    794   CD2  PHE A   98     18.334 -13.922  61.654  1.00 17.42          C
ATOM    795   CE1  PHE A   98     20.675 -14.595  60.440  1.00 17.28          C
ATOM    796   CE2  PHE A   98     19.309 -12.953  61.404  1.00 18.42          C
ATOM    797   CZ   PHE A   98     20.458 -13.293  60.837  1.00 17.76          C
ATOM    798   N    GLY A   99     15.387 -17.387  63.558  1.00 18.22          N
ATOM    799   CA   GLY A   99     14.231 -18.222  63.614  1.00 19.17          C
ATOM    800   C    GLY A   99     13.619 -18.280  62.219  1.00 21.03          C
ATOM    801   O    GLY A   99     14.107 -17.597  61.284  1.00 20.00          O
ATOM    802   N    GLY A  100     12.668 -19.196  62.039  1.00 21.13          N
ATOM    803   CA   GLY A  100     12.258 -19.574  60.686  1.00 22.44          C
ATOM    804   C    GLY A  100     11.314 -18.450  60.210  1.00 21.01          C
ATOM    805   O    GLY A  100     10.947 -18.394  59.048  1.00 21.41          O
ATOM    806   N    GLY A  101     10.915 -17.511  61.088  1.00 21.86          N
ATOM    807   CA   GLY A  101     10.026 -16.408  60.706  1.00 22.38          C
ATOM    808   C    GLY A  101      8.551 -16.757  60.931  1.00 23.76          C
ATOM    809   O    GLY A  101      8.116 -17.872  60.638  1.00 23.83          O
ATOM    810   N    THR A  102      7.786 -15.819  61.501  1.00 23.35          N
ATOM    811   CA   THR A  102      6.343 -15.967  61.694  1.00 23.06          C
ATOM    812   C    THR A  102      5.663 -14.911  60.851  1.00 23.80          C
ATOM    813   O    THR A  102      5.936 -13.720  60.979  1.00 22.31          O
ATOM    814   CB   THR A  102      5.956 -15.770  63.171  1.00 22.39          C
ATOM    815   OG1  THR A  102      6.686 -16.704  63.979  1.00 22.71          O
ATOM    816   CG2  THR A  102      4.478 -15.943  63.371  1.00 23.98          C
ATOM    817   N    LYS A  103      4.772 -15.337  59.963  1.00 25.10          N
ATOM    818   CA   LYS A  103      4.039 -14.387  59.136  1.00 24.59          C
ATOM    819   C    LYS A  103      2.816 -13.868  59.870  1.00 25.89          C
ATOM    820   O    LYS A  103      2.008 -14.651  60.389  1.00 25.94          O
ATOM    821   CB   LYS A  103      3.584 -15.043  57.835  1.00 25.90          C
ATOM    822   CG   LYS A  103      3.154 -14.077  56.782  1.00 27.72          C
ATOM    823   CD   LYS A  103      2.607 -14.784  55.554  1.00 30.68          C
ATOM    824   CE   LYS A  103      3.687 -15.509  54.768  1.00 33.03          C
ATOM    825   NZ   LYS A  103      3.019 -16.358  53.695  1.00 34.79          N
ATOM    826   N    LEU A  104      2.679 -12.555  59.908  1.00 26.07          N
ATOM    827   CA   LEU A  104      1.500 -11.944  60.496  1.00 27.49          C
ATOM    828   C    LEU A  104      0.379 -11.929  59.474  1.00 29.53          C
ATOM    829   O    LEU A  104      0.546 -11.379  58.375  1.00 29.97          O
ATOM    830   CB   LEU A  104      1.799 -10.526  60.931  1.00 27.39          C
ATOM    831   CG   LEU A  104      0.691  -9.824  61.716  1.00 26.27          C
ATOM    832   CD1  LEU A  104      0.518 -10.444  63.048  1.00 26.11          C
ATOM    833   CD2  LEU A  104      1.008  -8.381  61.848  1.00 26.98          C
ATOM    834   N    GLU A  105     -0.760 -12.517  59.838  1.00 31.66          N
ATOM    835   CA   GLU A  105     -1.974 -12.405  58.998  1.00 33.10          C
```

```
ATOM    836  C    GLU A 105      -3.033 -11.532  59.656  1.00 33.71           C
ATOM    837  O    GLU A 105      -3.410 -11.799  60.780  1.00 31.83           O
ATOM    838  CB   GLU A 105      -2.611 -13.758  58.703  1.00 34.44           C
ATOM    839  CG   GLU A 105      -3.618 -13.624  57.555  1.00 36.00           C
ATOM    840  CD   GLU A 105      -4.557 -14.800  57.399  1.00 37.62           C
ATOM    841  OE1  GLU A 105      -4.130 -15.946  57.684  1.00 39.45           O
ATOM    842  OE2  GLU A 105      -5.731 -14.556  56.981  1.00 38.57           O
ATOM    843  N    ILE A 106      -3.479 -10.496  58.943  1.00 34.44           N
ATOM    844  CA   ILE A 106      -4.573  -9.630  59.388  1.00 35.54           C
ATOM    845  C    ILE A 106      -5.913 -10.294  59.091  1.00 36.41           C
ATOM    846  O    ILE A 106      -6.142 -10.790  57.967  1.00 36.49           O
ATOM    847  CB   ILE A 106      -4.603  -8.294  58.626  1.00 35.90           C
ATOM    848  CG1  ILE A 106      -3.265  -7.554  58.715  1.00 37.08           C
ATOM    849  CG2  ILE A 106      -5.797  -7.437  59.072  1.00 36.03           C
ATOM    850  CD1  ILE A 106      -2.896  -7.048  60.039  1.00 37.33           C
ATOM    851  N    LYS A 107      -6.813 -10.264  60.069  1.00 37.06           N
ATOM    852  CA   LYS A 107      -8.199 -10.704  59.871  1.00 37.27           C
ATOM    853  C    LYS A 107      -9.069  -9.505  59.533  1.00 36.75           C
ATOM    854  O    LYS A 107      -9.078  -8.500  60.241  1.00 36.50           O
ATOM    855  CB   LYS A 107      -8.757 -11.402  61.103  1.00 38.24           C
ATOM    856  CG   LYS A 107     -10.210 -11.852  60.959  1.00 40.14           C
ATOM    857  CD   LYS A 107     -10.660 -12.678  62.142  1.00 41.69           C
ATOM    858  CE   LYS A 107     -12.181 -12.866  62.141  1.00 43.24           C
ATOM    859  NZ   LYS A 107     -12.598 -13.906  63.166  1.00 44.22           N
ATOM    860  N    ARG A 108      -9.792  -9.613  58.426  1.00 36.74           N
ATOM    861  CA   ARG A 108     -10.699  -8.560  57.995  1.00 35.65           C
ATOM    862  C    ARG A 108     -12.011  -9.174  57.559  1.00 35.28           C
ATOM    863  O    ARG A 108     -12.198 -10.401  57.628  1.00 32.92           O
ATOM    864  CB   ARG A 108     -10.074  -7.740  56.880  1.00 35.72           C
ATOM    865  CG   ARG A 108      -9.713  -8.543  55.640  1.00 35.40           C
ATOM    866  CD   ARG A 108      -9.729  -7.666  54.407  1.00 35.64           C
ATOM    867  NE   ARG A 108     -11.107  -7.450  53.951  1.00 35.89           N
ATOM    868  CZ   ARG A 108     -11.525  -6.422  53.226  1.00 36.06           C
ATOM    869  NH1  ARG A 108     -10.695  -5.472  52.843  1.00 33.75           N
ATOM    870  NH2  ARG A 108     -12.808  -6.341  52.888  1.00 38.21           N
ATOM    871  N    ALA A 109     -12.939  -8.313  57.141  1.00 36.28           N
ATOM    872  CA   ALA A 109     -14.273  -8.753  56.757  1.00 36.78           C
ATOM    873  C    ALA A 109     -14.172  -9.517  55.458  1.00 36.65           C
ATOM    874  O    ALA A 109     -13.517  -9.065  54.537  1.00 36.63           O
ATOM    875  CB   ALA A 109     -15.209  -7.560  56.576  1.00 37.54           C
ATOM    876  N    ASP A 110     -14.824 -10.672  55.401  1.00 36.07           N
ATOM    877  CA   ASP A 110     -14.903 -11.438  54.161  1.00 36.20           C
ATOM    878  C    ASP A 110     -15.276 -10.545  52.977  1.00 35.18           C
ATOM    879  O    ASP A 110     -16.075  -9.630  53.105  1.00 34.95           O
ATOM    880  CB   ASP A 110     -15.885 -12.580  54.304  1.00 36.39           C
ATOM    881  CG   ASP A 110     -15.455 -13.574  55.343  1.00 38.56           C
ATOM    882  OD1  ASP A 110     -14.376 -13.388  55.967  1.00 38.99           O
ATOM    883  OD2  ASP A 110     -16.191 -14.552  55.526  1.00 41.10           O
ATOM    884  N    ALA A 111     -14.635 -10.802  51.836  1.00 33.92           N
ATOM    885  CA   ALA A 111     -14.816 -10.015  50.622  1.00 32.72           C
ATOM    886  C    ALA A 111     -14.728 -10.976  49.446  1.00 32.67           C
ATOM    887  O    ALA A 111     -13.822 -11.810  49.405  1.00 31.85           O
ATOM    888  CB   ALA A 111     -13.768  -8.958  50.522  1.00 31.88           C
ATOM    889  N    ALA A 112     -15.708 -10.916  48.538  1.00 32.31           N
ATOM    890  CA   ALA A 112     -15.728 -11.816  47.379  1.00 30.18           C
ATOM    891  C    ALA A 112     -14.681 -11.340  46.353  1.00 29.34           C
ATOM    892  O    ALA A 112     -14.335 -10.148  46.281  1.00 26.17           O
ATOM    893  CB   ALA A 112     -17.130 -11.865  46.752  1.00 30.74           C
ATOM    894  N    PRO A 113     -14.177 -12.263  45.528  1.00 28.08           N
ATOM    895  CA   PRO A 113     -13.220 -11.846  44.518  1.00 28.72           C
ATOM    896  C    PRO A 113     -13.810 -11.043  43.346  1.00 28.01           C
ATOM    897  O    PRO A 113     -14.986 -11.202  43.006  1.00 31.00           O
ATOM    898  CB   PRO A 113     -12.699 -13.178  44.005  1.00 28.55           C
ATOM    899  CG   PRO A 113     -13.781 -14.067  44.145  1.00 28.73           C
ATOM    900  CD   PRO A 113     -14.468 -13.696  45.434  1.00 29.29           C
```

```
ATOM    901   N    THR A 114   -13.012  -10.162  42.769  1.00  29.27        N
ATOM    902   CA   THR A 114   -13.290   -9.585  41.461  1.00  28.79        C
ATOM    903   C    THR A 114   -12.597  -10.478  40.426  1.00  28.72        C
ATOM    904   O    THR A 114   -11.346  -10.582  40.413  1.00  26.44        O
ATOM    905   CB   THR A 114   -12.764   -8.161  41.354  1.00  29.24        C
ATOM    906   OG1  THR A 114   -13.378   -7.360  42.365  1.00  31.14        O
ATOM    907   CG2  THR A 114   -13.067   -7.545  40.021  1.00  29.01        C
ATOM    908   N    VAL A 115   -13.421  -11.099  39.576  1.00  27.42        N
ATOM    909   CA   VAL A 115   -12.976  -12.056  38.562  1.00  26.01        C
ATOM    910   C    VAL A 115   -12.883  -11.449  37.153  1.00  26.64        C
ATOM    911   O    VAL A 115   -13.823  -10.828  36.661  1.00  26.96        O
ATOM    912   CB   VAL A 115   -13.899  -13.240  38.529  1.00  26.68        C
ATOM    913   CG1  VAL A 115   -13.386  -14.280  37.542  1.00  25.73        C
ATOM    914   CG2  VAL A 115   -13.997  -13.837  39.921  1.00  27.22        C
ATOM    915   N    SER A 116   -11.740  -11.648  36.498  1.00  24.63        N
ATOM    916   CA   SER A 116   -11.501  -11.153  35.144  1.00  25.26        C
ATOM    917   C    SER A 116   -10.818  -12.248  34.314  1.00  25.43        C
ATOM    918   O    SER A 116    -9.879  -12.883  34.786  1.00  24.02        O
ATOM    919   CB   SER A 116   -10.628   -9.925  35.184  1.00  26.44        C
ATOM    920   OG   SER A 116   -11.178   -9.011  36.088  1.00  28.92        O
ATOM    921   N    ILE A 117   -11.295  -12.428  33.084  1.00  24.85        N
ATOM    922   CA   ILE A 117   -10.808  -13.467  32.180  1.00  24.97        C
ATOM    923   C    ILE A 117   -10.249  -12.800  30.923  1.00  24.22        C
ATOM    924   O    ILE A 117   -10.741  -11.724  30.474  1.00  22.99        O
ATOM    925   CB   ILE A 117   -11.916  -14.492  31.845  1.00  25.46        C
ATOM    926   CG1  ILE A 117   -11.314  -15.766  31.237  1.00  25.33        C
ATOM    927   CG2  ILE A 117   -13.002  -13.864  30.960  1.00  26.59        C
ATOM    928   CD1  ILE A 117   -12.304  -16.897  30.944  1.00  24.78        C
ATOM    929   N    PHE A 118    -9.166  -13.403  30.398  1.00  21.74        N
ATOM    930   CA   PHE A 118    -8.423  -12.856  29.273  1.00  23.93        C
ATOM    931   C    PHE A 118    -8.110  -13.988  28.324  1.00  23.22        C
ATOM    932   O    PHE A 118    -7.531  -14.986  28.733  1.00  23.16        O
ATOM    933   CB   PHE A 118    -7.115  -12.180  29.727  1.00  24.73        C
ATOM    934   CG   PHE A 118    -7.339  -10.991  30.625  1.00  23.57        C
ATOM    935   CD1  PHE A 118    -7.400  -11.142  31.984  1.00  25.12        C
ATOM    936   CD2  PHE A 118    -7.523   -9.735  30.089  1.00  26.14        C
ATOM    937   CE1  PHE A 118    -7.644  -10.042  32.812  1.00  26.41        C
ATOM    938   CE2  PHE A 118    -7.717   -8.639  30.911  1.00  27.00        C
ATOM    939   CZ   PHE A 118    -7.798   -8.808  32.271  1.00  26.90        C
ATOM    940   N    PRO A 119    -8.493  -13.844  27.051  1.00  23.53        N
ATOM    941   CA   PRO A 119    -8.076  -14.835  26.079  1.00  23.29        C
ATOM    942   C    PRO A 119    -6.591  -14.700  25.726  1.00  22.75        C
ATOM    943   O    PRO A 119    -5.957  -13.687  26.049  1.00  24.14        O
ATOM    944   CB   PRO A 119    -8.960  -14.522  24.865  1.00  23.46        C
ATOM    945   CG   PRO A 119    -9.280  -13.113  24.971  1.00  24.48        C
ATOM    946   CD   PRO A 119    -9.296  -12.767  26.430  1.00  23.61        C
ATOM    947   N    PRO A 120    -6.026  -15.729  25.090  1.00  24.18        N
ATOM    948   CA   PRO A 120    -4.668  -15.653  24.571  1.00  25.01        C
ATOM    949   C    PRO A 120    -4.536  -14.446  23.684  1.00  26.07        C
ATOM    950   O    PRO A 120    -5.413  -14.179  22.871  1.00  26.46        O
ATOM    951   CB   PRO A 120    -4.524  -16.920  23.749  1.00  25.46        C
ATOM    952   CG   PRO A 120    -5.501  -17.880  24.371  1.00  25.95        C
ATOM    953   CD   PRO A 120    -6.648  -17.034  24.829  1.00  24.27        C
ATOM    954   N    SER A 121    -3.443  -13.713  23.828  1.00  26.56        N
ATOM    955   CA   SER A 121    -3.215  -12.555  22.976  1.00  28.47        C
ATOM    956   C    SER A 121    -2.896  -13.016  21.561  1.00  29.47        C
ATOM    957   O    SER A 121    -2.448  -14.140  21.345  1.00  30.27        O
ATOM    958   CB   SER A 121    -2.065  -11.708  23.509  1.00  27.42        C
ATOM    959   OG   SER A 121    -0.857  -12.458  23.495  1.00  29.85        O
ATOM    960   N    SER A 122    -3.130  -12.130  20.602  1.00  32.45        N
ATOM    961   CA   SER A 122    -2.841  -12.424  19.199  1.00  33.94        C
ATOM    962   C    SER A 122    -1.359  -12.739  19.056  1.00  33.25        C
ATOM    963   O    SER A 122    -0.989  -13.693  18.401  1.00  32.62        O
ATOM    964   CB   SER A 122    -3.277  -11.269  18.267  1.00  35.47        C
ATOM    965   OG   SER A 122    -3.013   -9.976  18.810  1.00  38.22        O
```

```
ATOM    966  N    GLU A 123     -0.528 -11.962  19.746  1.00 34.89          N
ATOM    967  CA   GLU A 123      0.932 -12.136  19.693  1.00 34.11          C
ATOM    968  C    GLU A 123      1.325 -13.544  20.094  1.00 32.21          C
ATOM    969  O    GLU A 123      2.103 -14.196  19.410  1.00 32.75          O
ATOM    970  CB   GLU A 123      1.641 -11.093  20.569  1.00 35.83          C
ATOM    971  CG   GLU A 123      1.407  -9.655  20.103  1.00 38.09          C
ATOM    972  CD   GLU A 123      0.423  -8.875  20.964  1.00 42.80          C
ATOM    973  OE1  GLU A 123     -0.687  -9.406  21.258  1.00 43.39          O
ATOM    974  OE2  GLU A 123      0.763  -7.716  21.330  1.00 43.59          O
ATOM    975  N    GLN A 124      0.765 -14.047  21.186  1.00 30.81          N
ATOM    976  CA   GLN A 124      1.088 -15.411  21.597  1.00 30.28          C
ATOM    977  C    GLN A 124      0.573 -16.418  20.585  1.00 30.76          C
ATOM    978  O    GLN A 124      1.234 -17.397  20.311  1.00 30.26          O
ATOM    979  CB   GLN A 124      0.505 -15.749  22.976  1.00 28.51          C
ATOM    980  CG   GLN A 124      0.941 -17.110  23.491  1.00 27.34          C
ATOM    981  CD   GLN A 124      0.308 -17.456  24.804  1.00 25.79          C
ATOM    982  OE1  GLN A 124     -0.702 -16.880  25.174  1.00 28.01          O
ATOM    983  NE2  GLN A 124      0.894 -18.389  25.523  1.00 24.14          N
ATOM    984  N    LEU A 125     -0.624 -16.183  20.057  1.00 33.75          N
ATOM    985  CA   LEU A 125     -1.195 -17.097  19.071  1.00 35.57          C
ATOM    986  C    LEU A 125     -0.311 -17.204  17.841  1.00 37.62          C
ATOM    987  O    LEU A 125     -0.229 -18.277  17.235  1.00 39.46          O
ATOM    988  CB   LEU A 125     -2.596 -16.671  18.668  1.00 35.37          C
ATOM    989  CG   LEU A 125     -3.635 -16.914  19.758  1.00 34.45          C
ATOM    990  CD1  LEU A 125     -4.952 -16.248  19.355  1.00 34.59          C
ATOM    991  CD2  LEU A 125     -3.847 -18.387  20.065  1.00 34.36          C
ATOM    992  N    THR A 126      0.361 -16.109  17.476  1.00 39.33          N
ATOM    993  CA   THR A 126      1.351 -16.184  16.387  1.00 39.97          C
ATOM    994  C    THR A 126      2.433 -17.237  16.634  1.00 39.97          C
ATOM    995  O    THR A 126      3.116 -17.622  15.698  1.00 40.64          O
ATOM    996  CB   THR A 126      2.112 -14.873  16.158  1.00 40.34          C
ATOM    997  OG1  THR A 126      1.243 -13.747  16.280  1.00 41.69          O
ATOM    998  CG2  THR A 126      2.730 -14.864  14.751  1.00 40.74          C
ATOM    999  N    SER A 127      2.612 -17.664  17.885  1.00 40.24          N
ATOM   1000  CA   SER A 127      3.707 -18.558  18.287  1.00 40.59          C
ATOM   1001  C    SER A 127      3.342 -20.024  18.545  1.00 40.33          C
ATOM   1002  O    SER A 127      4.219 -20.815  18.900  1.00 41.49          O
ATOM   1003  CB   SER A 127      4.347 -18.024  19.580  1.00 41.48          C
ATOM   1004  OG   SER A 127      4.807 -16.691  19.426  1.00 43.11          O
ATOM   1005  N    GLY A 128      2.076 -20.406  18.429  1.00 39.19          N
ATOM   1006  CA   GLY A 128      1.729 -21.833  18.638  1.00 38.42          C
ATOM   1007  C    GLY A 128      1.437 -22.316  20.063  1.00 37.15          C
ATOM   1008  O    GLY A 128      1.249 -23.517  20.303  1.00 36.68          O
ATOM   1009  N    GLY A 129      1.396 -21.385  21.018  1.00 35.41          N
ATOM   1010  CA   GLY A 129      0.874 -21.662  22.360  1.00 33.42          C
ATOM   1011  C    GLY A 129     -0.298 -20.740  22.646  1.00 30.68          C
ATOM   1012  O    GLY A 129     -0.525 -19.753  21.932  1.00 29.85          O
ATOM   1013  N    ALA A 130     -1.060 -21.081  23.685  1.00 28.01          N
ATOM   1014  CA   ALA A 130     -2.278 -20.350  24.016  1.00 26.95          C
ATOM   1015  C    ALA A 130     -2.526 -20.402  25.496  1.00 25.84          C
ATOM   1016  O    ALA A 130     -2.938 -21.437  26.011  1.00 26.47          O
ATOM   1017  CB   ALA A 130     -3.467 -20.950  23.302  1.00 25.52          C
ATOM   1018  N    SER A 131     -2.288 -19.282  26.169  1.00 24.30          N
ATOM   1019  CA   SER A 131     -2.490 -19.153  27.605  1.00 23.15          C
ATOM   1020  C    SER A 131     -3.746 -18.329  27.896  1.00 22.75          C
ATOM   1021  O    SER A 131     -3.882 -17.205  27.413  1.00 22.33          O
ATOM   1022  CB   SER A 131     -1.268 -18.525  28.285  1.00 23.41          C
ATOM   1023  OG   SER A 131     -0.115 -19.307  28.085  1.00 24.42          O
ATOM   1024  N    VAL A 132     -4.663 -18.937  28.642  1.00 20.65          N
ATOM   1025  CA   VAL A 132     -5.873 -18.319  29.067  1.00 19.16          C
ATOM   1026  C    VAL A 132     -5.666 -17.927  30.531  1.00 18.59          C
ATOM   1027  O    VAL A 132     -5.252 -18.759  31.343  1.00 18.84          O
ATOM   1028  CB   VAL A 132     -7.096 -19.236  28.925  1.00 20.48          C
ATOM   1029  CG1  VAL A 132     -8.378 -18.386  28.888  1.00 21.73          C
ATOM   1030  CG2  VAL A 132     -7.014 -20.110  27.655  1.00 20.93          C
```

| | | | | | | | | | |
|---|---|---|---|---|---|---|---|---|---|
| ATOM | 1031 | N | VAL A 133 | -5.992 | -16.678 | 30.849 | 1.00 | 19.02 | N |
| ATOM | 1032 | CA | VAL A 133 | -5.676 | -16.099 | 32.159 | 1.00 | 21.62 | C |
| ATOM | 1033 | C | VAL A 133 | -6.952 | -15.610 | 32.861 | 1.00 | 22.33 | C |
| ATOM | 1034 | O | VAL A 133 | -7.842 | -14.999 | 32.258 | 1.00 | 22.53 | O |
| ATOM | 1035 | CB | VAL A 133 | -4.603 | -14.964 | 32.075 | 1.00 | 22.43 | C |
| ATOM | 1036 | CG1 | VAL A 133 | -4.338 | -14.371 | 33.459 | 1.00 | 24.89 | C |
| ATOM | 1037 | CG2 | VAL A 133 | -3.300 | -15.462 | 31.486 | 1.00 | 21.69 | C |
| ATOM | 1038 | N | CYS A 134 | -7.053 | -15.924 | 34.138 | 1.00 | 22.73 | N |
| ATOM | 1039 | CA ACYS A 134 | | -8.157 | -15.473 | 34.949 | 0.50 | 22.64 | C |
| ATOM | 1040 | CA BCYS A 134 | | -8.181 | -15.504 | 34.961 | 0.50 | 23.15 | C |
| ATOM | 1041 | C | CYS A 134 | -7.618 | -14.961 | 36.272 | 1.00 | 23.07 | C |
| ATOM | 1042 | O | CYS A 134 | -6.879 | -15.657 | 36.953 | 1.00 | 22.88 | O |
| ATOM | 1043 | CB ACYS A 134 | | -9.111 | -16.637 | 35.185 | 0.50 | 22.89 | C |
| ATOM | 1044 | CB BCYS A 134 | | -9.116 | -16.715 | 35.197 | 0.50 | 24.03 | C |
| ATOM | 1045 | SG ACYS A 134 | | -10.661 | -16.192 | 35.969 | 0.50 | 24.03 | S |
| ATOM | 1046 | SG BCYS A 134 | | -10.626 | -16.488 | 36.240 | 0.50 | 25.99 | S |
| ATOM | 1047 | N | PHE A 135 | -7.974 | -13.725 | 36.623 | 1.00 | 23.74 | N |
| ATOM | 1048 | CA | PHE A 135 | -7.560 | -13.167 | 37.905 | 1.00 | 24.91 | C |
| ATOM | 1049 | C | PHE A 135 | -8.748 | -13.270 | 38.844 | 1.00 | 25.80 | C |
| ATOM | 1050 | O | PHE A 135 | -9.857 | -12.920 | 38.446 | 1.00 | 26.00 | O |
| ATOM | 1051 | CB | PHE A 135 | -7.146 | -11.703 | 37.773 | 1.00 | 25.23 | C |
| ATOM | 1052 | CG | PHE A 135 | -5.950 | -11.469 | 36.882 | 1.00 | 24.96 | C |
| ATOM | 1053 | CD1 | PHE A 135 | -4.743 | -12.149 | 37.078 | 1.00 | 25.87 | C |
| ATOM | 1054 | CD2 | PHE A 135 | -6.015 | -10.558 | 35.848 | 1.00 | 26.91 | C |
| ATOM | 1055 | CE1 | PHE A 135 | -3.651 | -11.917 | 36.223 | 1.00 | 24.88 | C |
| ATOM | 1056 | CE2 | PHE A 135 | -4.929 | -10.323 | 35.003 | 1.00 | 26.71 | C |
| ATOM | 1057 | CZ | PHE A 135 | -3.750 | -11.012 | 35.187 | 1.00 | 26.25 | C |
| ATOM | 1058 | N | LEU A 136 | -8.502 | -13.777 | 40.058 | 1.00 | 26.17 | N |
| ATOM | 1059 | CA | LEU A 136 | -9.449 | -13.714 | 41.193 | 1.00 | 26.33 | C |
| ATOM | 1060 | C | LEU A 136 | -8.833 | -12.764 | 42.204 | 1.00 | 27.61 | C |
| ATOM | 1061 | O | LEU A 136 | -7.939 | -13.169 | 42.962 | 1.00 | 27.87 | O |
| ATOM | 1062 | CB | LEU A 136 | -9.644 | -15.080 | 41.843 | 1.00 | 27.10 | C |
| ATOM | 1063 | CG | LEU A 136 | -10.545 | -16.144 | 41.208 | 1.00 | 26.90 | C |
| ATOM | 1064 | CD1 | LEU A 136 | -10.402 | -16.168 | 39.726 | 1.00 | 27.87 | C |
| ATOM | 1065 | CD2 | LEU A 136 | -10.262 | -17.519 | 41.822 | 1.00 | 28.14 | C |
| ATOM | 1066 | N | ASN A 137 | -9.278 | -11.508 | 42.212 | 1.00 | 27.85 | N |
| ATOM | 1067 | CA | ASN A 137 | -8.563 | -10.441 | 42.912 | 1.00 | 28.07 | C |
| ATOM | 1068 | C | ASN A 137 | -9.262 | -9.917 | 44.161 | 1.00 | 29.46 | C |
| ATOM | 1069 | O | ASN A 137 | -10.474 | -9.836 | 44.200 | 1.00 | 31.34 | O |
| ATOM | 1070 | CB | ASN A 137 | -8.304 | -9.271 | 41.959 | 1.00 | 27.22 | C |
| ATOM | 1071 | CG | ASN A 137 | -7.094 | -9.518 | 41.047 | 1.00 | 29.07 | C |
| ATOM | 1072 | OD1 | ASN A 137 | -6.393 | -10.510 | 41.214 | 1.00 | 27.96 | O |
| ATOM | 1073 | ND2 | ASN A 137 | -6.861 | -8.636 | 40.097 | 1.00 | 28.08 | N |
| ATOM | 1074 | N | ASN A 138 | -8.465 | -9.603 | 45.186 | 1.00 | 29.67 | N |
| ATOM | 1075 | CA | ASN A 138 | -8.912 | -8.835 | 46.376 | 1.00 | 30.55 | C |
| ATOM | 1076 | C | ASN A 138 | -10.041 | -9.468 | 47.135 | 1.00 | 29.45 | C |
| ATOM | 1077 | O | ASN A 138 | -11.094 | -8.868 | 47.325 | 1.00 | 30.23 | O |
| ATOM | 1078 | CB | ASN A 138 | -9.274 | -7.397 | 45.993 | 1.00 | 30.65 | C |
| ATOM | 1079 | CG | ASN A 138 | -8.097 | -6.641 | 45.454 | 1.00 | 33.19 | C |
| ATOM | 1080 | OD1 | ASN A 138 | -7.851 | -6.640 | 44.251 | 1.00 | 36.00 | O |
| ATOM | 1081 | ND2 | ASN A 138 | -7.335 | -6.002 | 46.348 | 1.00 | 33.73 | N |
| ATOM | 1082 | N | PHE A 139 | -9.810 | -10.699 | 47.550 | 1.00 | 30.47 | N |
| ATOM | 1083 | CA | PHE A 139 | -10.756 | -11.441 | 48.339 | 1.00 | 30.29 | C |
| ATOM | 1084 | C | PHE A 139 | -10.193 | -11.743 | 49.724 | 1.00 | 31.55 | C |
| ATOM | 1085 | O | PHE A 139 | -8.980 | -11.700 | 49.951 | 1.00 | 30.78 | O |
| ATOM | 1086 | CB | PHE A 139 | -11.188 | -12.724 | 47.618 | 1.00 | 30.92 | C |
| ATOM | 1087 | CG | PHE A 139 | -10.050 | -13.642 | 47.269 | 1.00 | 29.76 | C |
| ATOM | 1088 | CD1 | PHE A 139 | -9.626 | -14.617 | 48.168 | 1.00 | 29.45 | C |
| ATOM | 1089 | CD2 | PHE A 139 | -9.421 | -13.562 | 46.037 | 1.00 | 29.54 | C |
| ATOM | 1090 | CE1 | PHE A 139 | -8.585 | -15.463 | 47.860 | 1.00 | 29.86 | C |
| ATOM | 1091 | CE2 | PHE A 139 | -8.352 | -14.407 | 45.731 | 1.00 | 29.32 | C |
| ATOM | 1092 | CZ | PHE A 139 | -7.946 | -15.364 | 46.637 | 1.00 | 29.19 | C |
| ATOM | 1093 | N | TYR A 140 | -11.094 | -12.025 | 50.652 | 1.00 | 33.28 | N |
| ATOM | 1094 | CA | TYR A 140 | -10.719 | -12.466 | 51.996 | 1.00 | 33.64 | C |
| ATOM | 1095 | C | TYR A 140 | -11.801 | -13.388 | 52.486 | 1.00 | 33.09 | C |

```
ATOM   1096  O    TYR A 140   -12.969 -13.072  52.338  1.00 32.20           O
ATOM   1097  CB   TYR A 140   -10.539 -11.297  52.965  1.00 34.32           C
ATOM   1098  CG   TYR A 140    -9.930 -11.793  54.260  1.00 35.09           C
ATOM   1099  CD1  TYR A 140   -10.721 -12.370  55.245  1.00 36.66           C
ATOM   1100  CD2  TYR A 140    -8.557 -11.771  54.456  1.00 35.29           C
ATOM   1101  CE1  TYR A 140   -10.159 -12.875  56.416  1.00 36.25           C
ATOM   1102  CE2  TYR A 140    -7.988 -12.279  55.640  1.00 36.50           C
ATOM   1103  CZ   TYR A 140    -8.805 -12.831  56.601  1.00 36.03           C
ATOM   1104  OH   TYR A 140    -8.259 -13.343  57.767  1.00 35.83           O
ATOM   1105  N    PRO A 141   -11.430 -14.545  53.062  1.00 33.81           N
ATOM   1106  CA   PRO A 141   -10.112 -15.121  53.359  1.00 33.94           C
ATOM   1107  C    PRO A 141    -9.433 -15.723  52.116  1.00 33.45           C
ATOM   1108  O    PRO A 141   -10.010 -15.701  51.020  1.00 32.22           O
ATOM   1109  CB   PRO A 141   -10.454 -16.224  54.354  1.00 34.40           C
ATOM   1110  CG   PRO A 141   -11.766 -16.693  53.908  1.00 35.15           C
ATOM   1111  CD   PRO A 141   -12.502 -15.462  53.465  1.00 34.85           C
ATOM   1112  N    LYS A 142    -8.237 -16.276  52.325  1.00 33.46           N
ATOM   1113  CA   LYS A 142    -7.342 -16.743  51.264  1.00 32.65           C
ATOM   1114  C    LYS A 142    -7.854 -17.978  50.517  1.00 31.82           C
ATOM   1115  O    LYS A 142    -7.488 -18.207  49.358  1.00 30.25           O
ATOM   1116  CB   LYS A 142    -5.956 -17.051  51.876  1.00 33.43           C
ATOM   1117  CG   LYS A 142    -4.831 -17.292  50.854  1.00 35.00           C
ATOM   1118  CD   LYS A 142    -3.480 -17.630  51.554  1.00 36.66           C
ATOM   1119  CE   LYS A 142    -2.279 -17.714  50.579  1.00 37.62           C
ATOM   1120  NZ   LYS A 142    -1.232 -16.615  50.707  1.00 37.37           N
ATOM   1121  N    ASP A 143    -8.662 -18.793  51.183  1.00 30.39           N
ATOM   1122  CA   ASP A 143    -9.134 -20.039  50.594  1.00 31.56           C
ATOM   1123  C    ASP A 143   -10.129 -19.765  49.452  1.00 30.81           C
ATOM   1124  O    ASP A 143   -11.119 -19.052  49.638  1.00 27.99           O
ATOM   1125  CB   ASP A 143    -9.818 -20.912  51.643  1.00 32.90           C
ATOM   1126  CG   ASP A 143    -8.846 -21.766  52.434  1.00 37.48           C
ATOM   1127  OD1  ASP A 143    -8.168 -22.638  51.837  1.00 41.98           O
ATOM   1128  OD2  ASP A 143    -8.783 -21.597  53.668  1.00 41.02           O
ATOM   1129  N    ILE A 144    -9.889 -20.382  48.299  1.00 29.09           N
ATOM   1130  CA   ILE A 144   -10.714 -20.140  47.114  1.00 28.54           C
ATOM   1131  C    ILE A 144   -10.497 -21.243  46.098  1.00 29.06           C
ATOM   1132  O    ILE A 144    -9.425 -21.849  46.051  1.00 28.98           O
ATOM   1133  CB   ILE A 144   -10.430 -18.747  46.504  1.00 27.90           C
ATOM   1134  CG1  ILE A 144   -11.557 -18.325  45.560  1.00 27.18           C
ATOM   1135  CG2  ILE A 144    -9.073 -18.705  45.774  1.00 28.97           C
ATOM   1136  CD1  ILE A 144   -11.519 -16.883  45.219  1.00 26.45           C
ATOM   1137  N    ASN A 145   -11.511 -21.512  45.285  1.00 28.35           N
ATOM   1138  CA   ASN A 145   -11.413 -22.559  44.261  1.00 29.38           C
ATOM   1139  C    ASN A 145   -11.653 -21.836  42.930  1.00 27.41           C
ATOM   1140  O    ASN A 145   -12.617 -21.096  42.765  1.00 27.21           O
ATOM   1141  CB   ASN A 145   -12.278 -23.752  44.563  1.00 31.72           C
ATOM   1142  CG   ASN A 145   -11.695 -24.626  45.702  1.00 36.68           C
ATOM   1143  OD1  ASN A 145   -10.522 -25.022  45.677  1.00 40.53           O
ATOM   1144  ND2  ASN A 145   -12.522 -24.928  46.691  1.00 40.04           N
ATOM   1145  N    VAL A 146   -10.831 -22.176  41.952  1.00 26.93           N
ATOM   1146  CA   VAL A 146   -11.211 -21.959  40.533  1.00 25.51           C
ATOM   1147  C    VAL A 146   -11.418 -23.163  39.675  1.00 25.16           C
ATOM   1148  O    VAL A 146   -10.763 -24.196  39.863  1.00 26.14           O
ATOM   1149  CB   VAL A 146   -10.065 -21.069  39.956  1.00 24.99           C
ATOM   1150  CG1  VAL A 146    -8.754 -21.835  39.883  1.00 26.32           C
ATOM   1151  CG2  VAL A 146   -10.401 -20.488  38.594  1.00 23.69           C
ATOM   1152  N    LYS A 147   -12.339 -23.023  38.719  1.00 24.72           N
ATOM   1153  CA   LYS A 147   -12.648 -24.062  37.774  1.00 25.03           C
ATOM   1154  C    LYS A 147   -12.638 -23.489  36.368  1.00 23.86           C
ATOM   1155  O    LYS A 147   -13.095 -22.380  36.144  1.00 26.00           O
ATOM   1156  CB   LYS A 147   -14.036 -24.638  38.077  1.00 26.72           C
ATOM   1157  CG   LYS A 147   -14.396 -25.902  37.358  1.00 29.37           C
ATOM   1158  CD   LYS A 147   -15.825 -26.351  37.729  1.00 30.63           C
ATOM   1159  CE   LYS A 147   -15.877 -27.061  39.084  1.00 32.06           C
ATOM   1160  NZ   LYS A 147   -17.241 -26.982  39.763  1.00 35.00           N
```

```
ATOM   1161  N    TRP A 148   -12.101 -24.270  35.437  1.00 22.44        N
ATOM   1162  CA   TRP A 148   -12.113 -23.961  34.015  1.00 21.98        C
ATOM   1163  C    TRP A 148   -13.068 -24.927  33.296  1.00 22.64        C
ATOM   1164  O    TRP A 148   -12.981 -26.165  33.456  1.00 22.89        O
ATOM   1165  CB   TRP A 148   -10.707 -24.057  33.407  1.00 21.78        C
ATOM   1166  CG   TRP A 148    -9.798 -22.967  33.805  1.00 20.39        C
ATOM   1167  CD1  TRP A 148    -8.949 -22.971  34.852  1.00 21.96        C
ATOM   1168  CD2  TRP A 148    -9.636 -21.701  33.157  1.00 20.83        C
ATOM   1169  NE1  TRP A 148    -8.261 -21.793  34.918  1.00 20.90        N
ATOM   1170  CE2  TRP A 148    -8.655 -20.990  33.878  1.00 22.75        C
ATOM   1171  CE3  TRP A 148   -10.214 -21.101  32.031  1.00 22.35        C
ATOM   1172  CZ2  TRP A 148    -8.248 -19.721  33.523  1.00 21.21        C
ATOM   1173  CZ3  TRP A 148    -9.830 -19.833  31.693  1.00 20.16        C
ATOM   1174  CH2  TRP A 148    -8.831 -19.157  32.416  1.00 21.76        C
ATOM   1175  N    LYS A 149   -13.984 -24.349  32.519  1.00 22.16        N
ATOM   1176  CA   LYS A 149   -14.887 -25.110  31.666  1.00 24.30        C
ATOM   1177  C    LYS A 149   -14.731 -24.582  30.238  1.00 23.30        C
ATOM   1178  O    LYS A 149   -14.735 -23.353  29.988  1.00 23.30        O
ATOM   1179  CB   LYS A 149   -16.348 -24.989  32.133  1.00 24.66        C
ATOM   1180  CG   LYS A 149   -16.562 -25.303  33.602  1.00 27.81        C
ATOM   1181  CD   LYS A 149   -18.014 -25.172  34.016  1.00 31.13        C
ATOM   1182  CE   LYS A 149   -18.812 -26.429  33.618  1.00 31.71        C
ATOM   1183  NZ   LYS A 149   -20.296 -26.407  33.920  1.00 33.06        N
ATOM   1184  N    ILE A 150   -14.546 -25.535  29.337  1.00 23.53        N
ATOM   1185  CA   ILE A 150   -14.375 -25.295  27.932  1.00 23.40        C
ATOM   1186  C    ILE A 150   -15.543 -25.994  27.225  1.00 24.07        C
ATOM   1187  O    ILE A 150   -15.666 -27.211  27.273  1.00 22.85        O
ATOM   1188  CB   ILE A 150   -13.027 -25.864  27.446  1.00 23.82        C
ATOM   1189  CG1  ILE A 150   -11.872 -25.205  28.227  1.00 23.61        C
ATOM   1190  CG2  ILE A 150   -12.874 -25.665  25.947  1.00 23.06        C
ATOM   1191  CD1  ILE A 150   -10.514 -25.774  27.878  1.00 25.65        C
ATOM   1192  N    ASP A 151   -16.393 -25.218  26.559  1.00 22.32        N
ATOM   1193  CA   ASP A 151   -17.582 -25.777  25.893  1.00 23.64        C
ATOM   1194  C    ASP A 151   -18.355 -26.672  26.851  1.00 23.15        C
ATOM   1195  O    ASP A 151   -18.764 -27.788  26.506  1.00 25.14        O
ATOM   1196  CB   ASP A 151   -17.182 -26.539  24.620  1.00 22.99        C
ATOM   1197  CG   ASP A 151   -16.695 -25.617  23.513  1.00 25.53        C
ATOM   1198  OD1  ASP A 151   -16.838 -24.399  23.667  1.00 23.22        O
ATOM   1199  OD2  ASP A 151   -16.189 -26.108  22.479  1.00 28.42        O
ATOM   1200  N    GLY A 152   -18.459 -26.240  28.091  1.00 25.25        N
ATOM   1201  CA   GLY A 152   -19.191 -27.007  29.092  1.00 27.85        C
ATOM   1202  C    GLY A 152   -18.435 -28.065  29.877  1.00 28.99        C
ATOM   1203  O    GLY A 152   -18.963 -28.556  30.874  1.00 31.28        O
ATOM   1204  N    SER A 153   -17.231 -28.432  29.441  1.00 28.88        N
ATOM   1205  CA   SER A 153   -16.472 -29.523  30.088  1.00 29.91        C
ATOM   1206  C    SER A 153   -15.398 -28.946  30.993  1.00 29.33        C
ATOM   1207  O    SER A 153   -14.681 -28.039  30.590  1.00 27.79        O
ATOM   1208  CB   SER A 153   -15.814 -30.421  29.054  1.00 30.78        C
ATOM   1209  OG   SER A 153   -16.782 -31.242  28.430  1.00 35.00        O
ATOM   1210  N    GLU A 154   -15.291 -29.469  32.212  1.00 29.22        N
ATOM   1211  CA   GLU A 154   -14.249 -29.042  33.131  1.00 28.74        C
ATOM   1212  C    GLU A 154   -12.900 -29.462  32.577  1.00 28.43        C
ATOM   1213  O    GLU A 154   -12.755 -30.565  32.053  1.00 27.17        O
ATOM   1214  CB   GLU A 154   -14.445 -29.621  34.531  1.00 30.23        C
ATOM   1215  CG   GLU A 154   -13.238 -29.368  35.408  1.00 32.28        C
ATOM   1216  CD   GLU A 154   -13.507 -29.380  36.896  1.00 36.05        C
ATOM   1217  OE1  GLU A 154   -14.624 -29.743  37.320  1.00 38.99        O
ATOM   1218  OE2  GLU A 154   -12.574 -28.998  37.638  1.00 38.42        O
ATOM   1219  N    ARG A 155   -11.932 -28.559  32.663  1.00 27.21        N
ATOM   1220  CA   ARG A 155   -10.551 -28.832  32.281  1.00 28.64        C
ATOM   1221  C    ARG A 155    -9.663 -28.637  33.507  1.00 28.38        C
ATOM   1222  O    ARG A 155    -9.674 -27.571  34.126  1.00 27.60        O
ATOM   1223  CB   ARG A 155   -10.104 -27.888  31.166  1.00 29.10        C
ATOM   1224  CG   ARG A 155    -8.639 -28.009  30.835  1.00 31.25        C
ATOM   1225  CD   ARG A 155    -8.379 -28.601  29.493  1.00 31.77        C
```

52

```
ATOM   1226  NE   ARG A 155    -6.954 -28.850  29.306  1.00 30.78        N
ATOM   1227  CZ   ARG A 155    -6.403 -29.315  28.190  1.00 31.67        C
ATOM   1228  NH1  ARG A 155    -7.136 -29.579  27.127  1.00 32.00        N
ATOM   1229  NH2  ARG A 155    -5.100 -29.548  28.149  1.00 33.44        N
ATOM   1230  N    GLN A 156    -8.916 -29.674  33.873  1.00 30.53        N
ATOM   1231  CA   GLN A 156    -7.914 -29.538  34.927  1.00 31.73        C
ATOM   1232  C    GLN A 156    -6.473 -29.585  34.398  1.00 29.70        C
ATOM   1233  O    GLN A 156    -5.587 -28.929  34.975  1.00 31.00        O
ATOM   1234  CB   GLN A 156    -8.150 -30.564  36.026  1.00 33.80        C
ATOM   1235  CG   GLN A 156    -9.383 -30.237  36.860  1.00 37.38        C
ATOM   1236  CD   GLN A 156    -9.082 -29.901  38.319  1.00 41.60        C
ATOM   1237  OE1  GLN A 156    -7.947 -29.547  38.686  1.00 45.06        O
ATOM   1238  NE2  GLN A 156   -10.106 -30.027  39.165  1.00 42.90        N
ATOM   1239  N    ASN A 157    -6.243 -30.306  33.305  1.00 28.17        N
ATOM   1240  CA AASN A 157    -4.896 -30.399  32.760  0.50 28.43        C
ATOM   1241  CA BASN A 157    -4.910 -30.419  32.657  0.50 27.88        C
ATOM   1242  C    ASN A 157    -4.436 -29.042  32.215  1.00 27.39        C
ATOM   1243  O    ASN A 157    -5.151 -28.361  31.510  1.00 27.20        O
ATOM   1244  CB AASN A 157    -4.808 -31.504  31.712  0.50 29.74        C
ATOM   1245  CB BASN A 157    -4.995 -31.364  31.431  0.50 28.60        C
ATOM   1246  CG AASN A 157    -5.006 -32.891  32.314  0.50 31.55        C
ATOM   1247  CG BASN A 157    -3.718 -31.378  30.551  0.50 28.68        C
ATOM   1248  OD1AASN A 157    -4.790 -33.097  33.510  0.50 35.43        O
ATOM   1249  OD1BASN A 157    -2.945 -30.429  30.509  0.50 29.95        O
ATOM   1250  ND2AASN A 157    -5.418 -33.849  31.487  0.50 33.39        N
ATOM   1251  ND2BASN A 157    -3.527 -32.473  29.823  0.50 31.70        N
ATOM   1252  N    GLY A 158    -3.237 -28.638  32.625  1.00 26.47        N
ATOM   1253  CA   GLY A 158    -2.637 -27.383  32.153  1.00 24.65        C
ATOM   1254  C    GLY A 158    -2.950 -26.127  32.921  1.00 23.51        C
ATOM   1255  O    GLY A 158    -2.613 -25.037  32.470  1.00 22.71        O
ATOM   1256  N    VAL A 159    -3.560 -26.275  34.085  1.00 24.25        N
ATOM   1257  CA   VAL A 159    -3.903 -25.140  34.925  1.00 23.25        C
ATOM   1258  C    VAL A 159    -2.918 -24.976  36.054  1.00 23.82        C
ATOM   1259  O    VAL A 159    -2.632 -25.933  36.783  1.00 24.27        O
ATOM   1260  CB   VAL A 159    -5.304 -25.279  35.538  1.00 23.48        C
ATOM   1261  CG1  VAL A 159    -5.611 -24.091  36.425  1.00 23.49        C
ATOM   1262  CG2  VAL A 159    -6.354 -25.396  34.422  1.00 23.52        C
ATOM   1263  N    LEU A 160    -2.437 -23.749  36.224  1.00 22.84        N
ATOM   1264  CA   LEU A 160    -1.509 -23.416  37.288  1.00 22.46        C
ATOM   1265  C    LEU A 160    -1.956 -22.152  37.986  1.00 22.40        C
ATOM   1266  O    LEU A 160    -2.231 -21.153  37.321  1.00 22.17        O
ATOM   1267  CB   LEU A 160    -0.096 -23.221  36.736  1.00 22.79        C
ATOM   1268  CG   LEU A 160     0.603 -24.481  36.264  1.00 23.40        C
ATOM   1269  CD1  LEU A 160     1.914 -24.149  35.613  1.00 23.35        C
ATOM   1270  CD2  LEU A 160     0.795 -25.435  37.396  1.00 26.71        C
ATOM   1271  N    ASN A 161    -2.013 -22.224  39.321  1.00 23.93        N
ATOM   1272  CA   ASN A 161    -2.509 -21.146  40.167  1.00 23.43        C
ATOM   1273  C    ASN A 161    -1.431 -20.487  41.035  1.00 24.83        C
ATOM   1274  O    ASN A 161    -0.568 -21.186  41.575  1.00 26.76        O
ATOM   1275  CB   ASN A 161    -3.600 -21.734  41.076  1.00 23.76        C
ATOM   1276  CG   ASN A 161    -4.783 -22.271  40.285  1.00 23.76        C
ATOM   1277  OD1  ASN A 161    -5.199 -21.662  39.305  1.00 21.70        O
ATOM   1278  ND2  ASN A 161    -5.311 -23.434  40.695  1.00 23.83        N
ATOM   1279  N    SER A 162    -1.498 -19.163  41.192  1.00 23.08        N
ATOM   1280  CA   SER A 162    -0.520 -18.433  42.012  1.00 24.76        C
ATOM   1281  C    SER A 162    -1.207 -17.437  42.908  1.00 25.00        C
ATOM   1282  O    SER A 162    -1.868 -16.531  42.411  1.00 24.76        O
ATOM   1283  CB   SER A 162     0.489 -17.671  41.163  1.00 24.88        C
ATOM   1284  OG   SER A 162     1.539 -17.174  42.010  1.00 23.40        O
ATOM   1285  N    TRP A 163    -1.009 -17.578  44.217  1.00 25.49        N
ATOM   1286  CA   TRP A 163    -1.511 -16.595  45.207  1.00 26.54        C
ATOM   1287  C    TRP A 163    -0.529 -15.491  45.582  1.00 27.61        C
ATOM   1288  O    TRP A 163     0.669 -15.732  45.776  1.00 27.12        O
ATOM   1289  CB   TRP A 163    -1.880 -17.300  46.513  1.00 28.67        C
ATOM   1290  CG   TRP A 163    -3.236 -17.916  46.590  1.00 30.91        C
```

```
ATOM   1291  CD1 TRP A 163    -4.343 -17.414  47.243  1.00 31.09           C
ATOM   1292  CD2 TRP A 163    -3.625 -19.180  46.065  1.00 31.64           C
ATOM   1293  NE1 TRP A 163    -5.387 -18.289  47.142  1.00 31.73           N
ATOM   1294  CE2 TRP A 163    -4.980 -19.383  46.423  1.00 31.46           C
ATOM   1295  CE3 TRP A 163    -2.966 -20.163  45.325  1.00 31.84           C
ATOM   1296  CZ2 TRP A 163    -5.683 -20.517  46.051  1.00 31.80           C
ATOM   1297  CZ3 TRP A 163    -3.665 -21.291  44.956  1.00 30.95           C
ATOM   1298  CH2 TRP A 163    -5.009 -21.468  45.327  1.00 33.21           C
ATOM   1299  N   THR A 164    -1.027 -14.267  45.726  1.00 26.54           N
ATOM   1300  CA  THR A 164    -0.224 -13.211  46.305  1.00 27.70           C
ATOM   1301  C   THR A 164    -0.141 -13.324  47.832  1.00 27.61           C
ATOM   1302  O   THR A 164    -0.853 -14.084  48.485  1.00 27.73           O
ATOM   1303  CB  THR A 164    -0.774 -11.827  46.013  1.00 27.52           C
ATOM   1304  OG1 THR A 164    -2.115 -11.745  46.492  1.00 27.67           O
ATOM   1305  CG2 THR A 164    -0.706 -11.521  44.543  1.00 28.42           C
ATOM   1306  N   ASP A 165     0.801 -12.569  48.359  1.00 29.22           N
ATOM   1307  CA  ASP A 165     0.894 -12.303  49.768  1.00 30.35           C
ATOM   1308  C   ASP A 165    -0.260 -11.402  50.156  1.00 30.11           C
ATOM   1309  O   ASP A 165    -0.832 -10.722  49.302  1.00 30.37           O
ATOM   1310  CB  ASP A 165     2.196 -11.568  50.050  1.00 30.48           C
ATOM   1311  CG  ASP A 165     3.396 -12.429  49.833  1.00 31.53           C
ATOM   1312  OD1 ASP A 165     3.370 -13.594  50.299  1.00 31.57           O
ATOM   1313  OD2 ASP A 165     4.365 -11.925  49.226  1.00 33.56           O
ATOM   1314  N   GLN A 166    -0.576 -11.362  51.444  1.00 29.63           N
ATOM   1315  CA  GLN A 166    -1.632 -10.446  51.914  1.00 30.52           C
ATOM   1316  C   GLN A 166    -1.308  -8.985  51.588  1.00 30.88           C
ATOM   1317  O   GLN A 166    -0.196  -8.513  51.823  1.00 29.97           O
ATOM   1318  CB  GLN A 166    -1.849 -10.613  53.412  1.00 30.30           C
ATOM   1319  CG  GLN A 166    -3.116  -9.873  53.915  1.00 29.36           C
ATOM   1320  CD  GLN A 166    -3.557 -10.376  55.260  1.00 29.94           C
ATOM   1321  OE1 GLN A 166    -2.731 -10.789  56.085  1.00 29.71           O
ATOM   1322  NE2 GLN A 166    -4.861 -10.376  55.488  1.00 28.17           N
ATOM   1323  N   ASP A 167    -2.276  -8.265  51.017  1.00 31.75           N
ATOM   1324  CA  ASP A 167    -2.060  -6.885  50.587  1.00 32.21           C
ATOM   1325  C   ASP A 167    -1.850  -5.964  51.806  1.00 33.28           C
ATOM   1326  O   ASP A 167    -2.578  -6.063  52.806  1.00 33.06           O
ATOM   1327  CB  ASP A 167    -3.252  -6.406  49.752  1.00 32.98           C
ATOM   1328  CG  ASP A 167    -3.039  -5.019  49.134  1.00 35.08           C
ATOM   1329  OD1 ASP A 167    -2.666  -4.957  47.943  1.00 35.97           O
ATOM   1330  OD2 ASP A 167    -3.233  -3.977  49.820  1.00 37.02           O
ATOM   1331  N   SER A 168    -0.856  -5.082  51.706  1.00 34.43           N
ATOM   1332  CA  SER A 168    -0.439  -4.215  52.803  1.00 35.47           C
ATOM   1333  C   SER A 168    -1.409  -3.063  53.008  1.00 35.99           C
ATOM   1334  O   SER A 168    -1.306  -2.379  54.016  1.00 36.19           O
ATOM   1335  CB  SER A 168     0.957  -3.633  52.552  1.00 36.32           C
ATOM   1336  OG  SER A 168     1.976  -4.594  52.794  1.00 39.23           O
ATOM   1337  N   LYS A 169    -2.324  -2.857  52.052  1.00 36.16           N
ATOM   1338  CA  LYS A 169    -3.323  -1.779  52.120  1.00 36.88           C
ATOM   1339  C   LYS A 169    -4.707  -2.230  52.556  1.00 36.28           C
ATOM   1340  O   LYS A 169    -5.290  -1.626  53.476  1.00 37.28           O
ATOM   1341  CB  LYS A 169    -3.429  -1.047  50.779  1.00 38.21           C
ATOM   1342  CG  LYS A 169    -2.598   0.231  50.706  1.00 40.29           C
ATOM   1343  CD  LYS A 169    -1.104  -0.036  50.565  1.00 41.55           C
ATOM   1344  CE  LYS A 169    -0.348   1.154  49.944  1.00 42.13           C
ATOM   1345  NZ  LYS A 169    -0.111   2.321  50.874  1.00 43.14           N
ATOM   1346  N   ASP A 170    -5.236  -3.264  51.905  1.00 34.41           N
ATOM   1347  CA  ASP A 170    -6.599  -3.720  52.164  1.00 33.69           C
ATOM   1348  C   ASP A 170    -6.699  -5.109  52.742  1.00 32.49           C
ATOM   1349  O   ASP A 170    -7.800  -5.605  52.939  1.00 31.66           O
ATOM   1350  CB  ASP A 170    -7.484  -3.589  50.910  1.00 34.49           C
ATOM   1351  CG  ASP A 170    -7.253  -4.696  49.884  1.00 35.51           C
ATOM   1352  OD1 ASP A 170    -6.348  -5.536  50.077  1.00 34.40           O
ATOM   1353  OD2 ASP A 170    -8.008  -4.720  48.875  1.00 38.07           O
ATOM   1354  N   SER A 171    -5.560  -5.754  53.002  1.00 30.96           N
ATOM   1355  CA  SER A 171    -5.533  -6.999  53.773  1.00 29.25           C
```

```
ATOM   1356  C    SER A 171     -6.230  -8.186  53.083  1.00  27.27           C
ATOM   1357  O    SER A 171     -6.540  -9.190  53.711  1.00  27.67           O
ATOM   1358  CB   SER A 171     -6.057  -6.735  55.214  1.00  29.46           C
ATOM   1359  OG   SER A 171     -5.317  -5.640  55.788  1.00  29.22           O
ATOM   1360  N    THR A 172     -6.443  -8.053  51.772  1.00  29.54           N
ATOM   1361  CA   THR A 172     -6.972  -9.120  50.917  1.00  28.86           C
ATOM   1362  C    THR A 172     -5.890  -9.908  50.203  1.00  29.31           C
ATOM   1363  O    THR A 172     -4.715  -9.529  50.186  1.00  30.65           O
ATOM   1364  CB   THR A 172     -7.837  -8.552  49.774  1.00  29.22           C
ATOM   1365  OG1  THR A 172     -7.022  -7.705  48.951  1.00  30.71           O
ATOM   1366  CG2  THR A 172     -9.041  -7.790  50.343  1.00  30.18           C
ATOM   1367  N    TYR A 173     -6.325 -11.012  49.615  1.00  28.61           N
ATOM   1368  CA   TYR A 173     -5.491 -11.813  48.745  1.00  28.38           C
ATOM   1369  C    TYR A 173     -5.961 -11.731  47.296  1.00  28.83           C
ATOM   1370  O    TYR A 173     -7.080 -11.300  46.996  1.00  26.92           O
ATOM   1371  CB   TYR A 173     -5.529 -13.245  49.223  1.00  29.40           C
ATOM   1372  CG   TYR A 173     -5.030 -13.394  50.643  1.00  31.43           C
ATOM   1373  CD1  TYR A 173     -5.896 -13.257  51.721  1.00  31.89           C
ATOM   1374  CD2  TYR A 173     -3.689 -13.674  50.907  1.00  32.91           C
ATOM   1375  CE1  TYR A 173     -5.456 -13.401  53.015  1.00  30.80           C
ATOM   1376  CE2  TYR A 173     -3.234 -13.815  52.227  1.00  32.69           C
ATOM   1377  CZ   TYR A 173     -4.126 -13.681  53.264  1.00  32.32           C
ATOM   1378  OH   TYR A 173     -3.681 -13.823  54.563  1.00  32.85           O
ATOM   1379  N    SER A 174     -5.081 -12.159  46.394  1.00  28.59           N
ATOM   1380  CA   SER A 174     -5.436 -12.339  44.982  1.00  27.25           C
ATOM   1381  C    SER A 174     -4.851 -13.654  44.502  1.00  27.30           C
ATOM   1382  O    SER A 174     -3.945 -14.204  45.137  1.00  25.13           O
ATOM   1383  CB   SER A 174     -4.934 -11.176  44.149  1.00  26.84           C
ATOM   1384  OG   SER A 174     -5.586  -9.993  44.547  1.00  25.84           O
ATOM   1385  N    MET A 175     -5.388 -14.167  43.401  1.00  27.15           N
ATOM   1386  CA   MET A 175     -4.921 -15.426  42.842  1.00  27.10           C
ATOM   1387  C    MET A 175     -4.981 -15.286  41.335  1.00  25.41           C
ATOM   1388  O    MET A 175     -5.890 -14.656  40.818  1.00  23.75           O
ATOM   1389  CB   MET A 175     -5.793 -16.569  43.306  1.00  28.62           C
ATOM   1390  CG   MET A 175     -5.180 -17.931  43.139  1.00  32.58           C
ATOM   1391  SD   MET A 175     -6.401 -19.235  42.972  1.00  41.16           S
ATOM   1392  CE   MET A 175     -6.736 -19.023  41.250  1.00  40.17           C
ATOM   1393  N    SER A 176     -3.991 -15.827  40.630  1.00  23.24           N
ATOM   1394  CA   SER A 176     -4.136 -15.946  39.170  1.00  21.36           C
ATOM   1395  C    SER A 176     -4.261 -17.422  38.857  1.00  21.55           C
ATOM   1396  O    SER A 176     -3.689 -18.298  39.532  1.00  20.83           O
ATOM   1397  CB   SER A 176     -2.951 -15.366  38.417  1.00  23.04           C
ATOM   1398  OG   SER A 176     -1.791 -16.131  38.737  1.00  24.41           O
ATOM   1399  N    SER A 177     -5.038 -17.704  37.837  1.00  20.16           N
ATOM   1400  CA   SER A 177     -5.158 -19.066  37.334  1.00  20.45           C
ATOM   1401  C    SER A 177     -4.864 -18.937  35.857  1.00  20.57           C
ATOM   1402  O    SER A 177     -5.438 -18.097  35.161  1.00  21.45           O
ATOM   1403  CB   SER A 177     -6.544 -19.626  37.583  1.00  20.60           C
ATOM   1404  OG   SER A 177     -6.621 -20.967  37.145  1.00  20.72           O
ATOM   1405  N    THR A 178     -3.919 -19.739  35.386  1.00  20.69           N
ATOM   1406  CA   THR A 178     -3.462 -19.659  34.015  1.00  21.23           C
ATOM   1407  C    THR A 178     -3.591 -21.058  33.427  1.00  20.85           C
ATOM   1408  O    THR A 178     -3.003 -22.002  33.942  1.00  19.91           O
ATOM   1409  CB   THR A 178     -1.987 -19.214  33.942  1.00  22.04           C
ATOM   1410  OG1  THR A 178     -1.815 -17.960  34.625  1.00  21.71           O
ATOM   1411  CG2  THR A 178     -1.540 -19.096  32.501  1.00  22.95           C
ATOM   1412  N    LEU A 179     -4.359 -21.159  32.353  1.00  20.99           N
ATOM   1413  CA   LEU A 179     -4.566 -22.385  31.601  1.00  22.40           C
ATOM   1414  C    LEU A 179     -3.735 -22.337  30.332  1.00  22.45           C
ATOM   1415  O    LEU A 179     -3.992 -21.504  29.460  1.00  22.19           O
ATOM   1416  CB   LEU A 179     -6.032 -22.530  31.236  1.00  23.70           C
ATOM   1417  CG   LEU A 179     -6.398 -23.711  30.308  1.00  20.72           C
ATOM   1418  CD1  LEU A 179     -6.120 -25.146  30.872  1.00  21.73           C
ATOM   1419  CD2  LEU A 179     -7.849 -23.541  29.921  1.00  23.72           C
ATOM   1420  N    THR A 180     -2.760 -23.236  30.227  1.00  23.28           N
```

```
ATOM   1421  CA   THR A 180      -1.896 -23.240  29.065  1.00 23.66          C
ATOM   1422  C    THR A 180      -2.217 -24.407  28.145  1.00 22.77          C
ATOM   1423  O    THR A 180      -2.197 -25.569  28.568  1.00 23.35          O
ATOM   1424  CB   THR A 180      -0.422 -23.212  29.465  1.00 25.16          C
ATOM   1425  OG1  THR A 180      -0.197 -22.189  30.441  1.00 26.18          O
ATOM   1426  CG2  THR A 180       0.435 -22.886  28.265  1.00 25.85          C
ATOM   1427  N    LEU A 181      -2.500 -24.070  26.895  1.00 21.58          N
ATOM   1428  CA   LEU A 181      -2.943 -25.000  25.844  1.00 23.72          C
ATOM   1429  C    LEU A 181      -2.022 -24.871  24.657  1.00 23.92          C
ATOM   1430  O    LEU A 181      -1.310 -23.875  24.501  1.00 24.74          O
ATOM   1431  CB   LEU A 181      -4.365 -24.666  25.356  1.00 24.49          C
ATOM   1432  CG   LEU A 181      -5.490 -24.559  26.373  1.00 24.97          C
ATOM   1433  CD1  LEU A 181      -6.806 -24.146  25.720  1.00 25.84          C
ATOM   1434  CD2  LEU A 181      -5.647 -25.860  27.077  1.00 25.78          C
ATOM   1435  N    THR A 182      -2.042 -25.870  23.777  1.00 25.66          N
ATOM   1436  CA   THR A 182      -1.405 -25.705  22.487  1.00 25.57          C
ATOM   1437  C    THR A 182      -2.339 -24.862  21.644  1.00 25.02          C
ATOM   1438  O    THR A 182      -3.549 -24.839  21.881  1.00 23.74          O
ATOM   1439  CB   THR A 182      -1.225 -27.059  21.777  1.00 25.73          C
ATOM   1440  OG1  THR A 182      -2.528 -27.616  21.514  1.00 26.91          O
ATOM   1441  CG2  THR A 182      -0.415 -28.029  22.643  1.00 25.97          C
ATOM   1442  N    LYS A 183      -1.804 -24.138  20.673  1.00 26.01          N
ATOM   1443  CA   LYS A 183      -2.648 -23.437  19.713  1.00 26.34          C
ATOM   1444  C    LYS A 183      -3.783 -24.314  19.117  1.00 25.82          C
ATOM   1445  O    LYS A 183      -4.950 -23.951  19.156  1.00 24.65          O
ATOM   1446  CB   LYS A 183      -1.787 -22.862  18.589  1.00 27.22          C
ATOM   1447  CG   LYS A 183      -2.531 -21.989  17.650  1.00 30.72          C
ATOM   1448  CD   LYS A 183      -1.626 -21.603  16.485  1.00 32.89          C
ATOM   1449  CE   LYS A 183      -2.291 -20.592  15.575  1.00 34.83          C
ATOM   1450  NZ   LYS A 183      -1.285 -19.585  15.110  1.00 36.00          N
ATOM   1451  N    ASP A 184      -3.441 -25.485  18.594  1.00 25.52          N
ATOM   1452  CA   ASP A 184      -4.450 -26.392  18.048  1.00 24.35          C
ATOM   1453  C    ASP A 184      -5.575 -26.716  19.040  1.00 22.38          C
ATOM   1454  O    ASP A 184      -6.752 -26.659  18.680  1.00 22.22          O
ATOM   1455  CB   ASP A 184      -3.817 -27.695  17.602  1.00 24.62          C
ATOM   1456  CG   ASP A 184      -2.934 -27.543  16.385  1.00 26.33          C
ATOM   1457  OD1  ASP A 184      -2.821 -26.429  15.815  1.00 26.17          O
ATOM   1458  OD2  ASP A 184      -2.352 -28.587  16.007  1.00 25.13          O
ATOM   1459  N    GLU A 185      -5.224 -26.971  20.301  1.00 21.43          N
ATOM   1460  CA   GLU A 185      -6.217 -27.367  21.293  1.00 20.95          C
ATOM   1461  C    GLU A 185      -7.107 -26.183  21.592  1.00 22.49          C
ATOM   1462  O    GLU A 185      -8.329 -26.328  21.671  1.00 20.68          O
ATOM   1463  CB   GLU A 185      -5.570 -27.886  22.565  1.00 21.66          C
ATOM   1464  CG   GLU A 185      -6.550 -28.379  23.610  1.00 23.02          C
ATOM   1465  CD   GLU A 185      -7.351 -29.605  23.164  1.00 26.25          C
ATOM   1466  OE1  GLU A 185      -8.414 -29.856  23.768  1.00 28.07          O
ATOM   1467  OE2  GLU A 185      -6.946 -30.306  22.215  1.00 28.33          O
ATOM   1468  N    TYR A 186      -6.499 -25.007  21.716  1.00 21.82          N
ATOM   1469  CA   TYR A 186      -7.267 -23.779  21.899  1.00 21.96          C
ATOM   1470  C    TYR A 186      -8.271 -23.548  20.765  1.00 23.11          C
ATOM   1471  O    TYR A 186      -9.443 -23.239  21.005  1.00 23.08          O
ATOM   1472  CB   TYR A 186      -6.337 -22.571  22.049  1.00 22.88          C
ATOM   1473  CG   TYR A 186      -7.067 -21.233  22.158  1.00 21.90          C
ATOM   1474  CD1  TYR A 186      -7.861 -20.932  23.275  1.00 21.91          C
ATOM   1475  CD2  TYR A 186      -6.996 -20.294  21.148  1.00 20.90          C
ATOM   1476  CE1  TYR A 186      -8.530 -19.723  23.387  1.00 20.83          C
ATOM   1477  CE2  TYR A 186      -7.672 -19.071  21.261  1.00 19.25          C
ATOM   1478  CZ   TYR A 186      -8.425 -18.809  22.404  1.00 23.06          C
ATOM   1479  OH   TYR A 186      -9.086 -17.636  22.537  1.00 21.28          O
ATOM   1480  N    GLU A 187      -7.816 -23.743  19.535  1.00 22.94          N
ATOM   1481  CA   GLU A 187      -8.649 -23.526  18.354  1.00 23.27          C
ATOM   1482  C    GLU A 187      -9.740 -24.589  18.154  1.00 24.54          C
ATOM   1483  O    GLU A 187     -10.673 -24.397  17.357  1.00 23.39          O
ATOM   1484  CB   GLU A 187      -7.772 -23.455  17.121  1.00 25.63          C
ATOM   1485  CG   GLU A 187      -6.989 -22.217  17.023  1.00 28.87          C
```

| ATOM | 1486 | CD  | GLU  | A | 187 | -6.192  | -22.129 | 15.734 | 1.00 | 33.64 | C |
|------|------|-----|------|---|-----|---------|---------|--------|------|-------|---|
| ATOM | 1487 | OE1 | GLU  | A | 187 | -5.976  | -23.182 | 15.060 | 1.00 | 37.39 | O |
| ATOM | 1488 | OE2 | GLU  | A | 187 | -5.792  | -20.991 | 15.414 | 1.00 | 38.10 | O |
| ATOM | 1489 | N   | ARG  | A | 188 | -9.610  | -25.698 | 18.863 | 1.00 | 24.72 | N |
| ATOM | 1490 | CA  | ARG  | A | 188 | -10.582 | -26.771 | 18.787 | 1.00 | 25.42 | C |
| ATOM | 1491 | C   | ARG  | A | 188 | -11.837 | -26.457 | 19.581 | 1.00 | 24.88 | C |
| ATOM | 1492 | O   | ARG  | A | 188 | -12.798 | -27.215 | 19.493 | 1.00 | 25.73 | O |
| ATOM | 1493 | CB  | ARG  | A | 188 | -9.982  | -28.087 | 19.310 | 1.00 | 25.43 | C |
| ATOM | 1494 | CG  | ARG  | A | 188 | -9.312  | -28.968 | 18.250 | 1.00 | 27.52 | C |
| ATOM | 1495 | CD  | ARG  | A | 188 | -9.057  | -30.375 | 18.760 | 1.00 | 27.73 | C |
| ATOM | 1496 | NE  | ARG  | A | 188 | -7.840  | -30.504 | 19.555 | 1.00 | 27.11 | N |
| ATOM | 1497 | CZ  | ARG  | A | 188 | -6.597  | -30.542 | 19.064 | 1.00 | 27.57 | C |
| ATOM | 1498 | NH1 | ARG  | A | 188 | -6.388  | -30.445 | 17.757 | 1.00 | 30.42 | N |
| ATOM | 1499 | NH2 | ARG  | A | 188 | -5.554  | -30.660 | 19.878 | 1.00 | 26.14 | N |
| ATOM | 1500 | N   | HIS  | A | 189 | -11.841 | -25.385 | 20.392 | 1.00 | 21.37 | N |
| ATOM | 1501 | CA  | HIS  | A | 189 | -12.985 | -25.072 | 21.233 | 1.00 | 20.78 | C |
| ATOM | 1502 | C   | HIS  | A | 189 | -13.233 | -23.590 | 21.235 | 1.00 | 21.10 | C |
| ATOM | 1503 | O   | HIS  | A | 189 | -12.321 | -22.824 | 20.980 | 1.00 | 22.79 | O |
| ATOM | 1504 | CB  | HIS  | A | 189 | -12.747 | -25.589 | 22.637 | 1.00 | 20.64 | C |
| ATOM | 1505 | CG  | HIS  | A | 189 | -12.784 | -27.072 | 22.689 | 1.00 | 22.74 | C |
| ATOM | 1506 | ND1 | HIS  | A | 189 | -11.651 | -27.841 | 22.803 | 1.00 | 26.56 | N |
| ATOM | 1507 | CD2 | HIS  | A | 189 | -13.817 | -27.933 | 22.549 | 1.00 | 24.94 | C |
| ATOM | 1508 | CE1 | HIS  | A | 189 | -11.985 | -29.121 | 22.763 | 1.00 | 26.47 | C |
| ATOM | 1509 | NE2 | HIS  | A | 189 | -13.295 | -29.203 | 22.622 | 1.00 | 28.18 | N |
| ATOM | 1510 | N   | ASN  | A | 190 | -14.478 | -23.184 | 21.468 | 1.00 | 22.48 | N |
| ATOM | 1511 | CA  | ASN  | A | 190 | -14.817 | -21.774 | 21.301 | 1.00 | 22.72 | C |
| ATOM | 1512 | C   | ASN  | A | 190 | -15.238 | -21.033 | 22.576 | 1.00 | 21.81 | C |
| ATOM | 1513 | O   | ASN  | A | 190 | -14.989 | -19.833 | 22.666 | 1.00 | 22.70 | O |
| ATOM | 1514 | CB  | ASN  | A | 190 | -15.873 | -21.645 | 20.191 | 1.00 | 25.53 | C |
| ATOM | 1515 | CG  | ASN  | A | 190 | -15.330 | -22.037 | 18.808 | 1.00 | 27.76 | C |
| ATOM | 1516 | OD1 | ASN  | A | 190 | -16.102 | -22.287 | 17.861 | 1.00 | 33.23 | O |
| ATOM | 1517 | ND2 | ASN  | A | 190 | -14.013 | -22.086 | 18.679 | 1.00 | 28.54 | N |
| ATOM | 1518 | N   | SER  | A | 191 | -15.857 | -21.711 | 23.541 | 1.00 | 21.87 | N |
| ATOM | 1519 | CA  | SER  | A | 191 | -16.403 | -21.042 | 24.739 | 1.00 | 21.31 | C |
| ATOM | 1520 | C   | SER  | A | 191 | -15.597 | -21.393 | 25.999 | 1.00 | 21.36 | C |
| ATOM | 1521 | O   | SER  | A | 191 | -15.532 | -22.574 | 26.408 | 1.00 | 22.13 | O |
| ATOM | 1522 | CB  | SER  | A | 191 | -17.880 | -21.422 | 24.904 | 1.00 | 21.67 | C |
| ATOM | 1523 | OG  | SER  | A | 191 | -18.473 | -20.683 | 25.927 | 1.00 | 21.36 | O |
| ATOM | 1524 | N   | TYR  | A | 192 | -14.969 | -20.373 | 26.577 | 1.00 | 19.64 | N |
| ATOM | 1525 | CA  | TYR  | A | 192 | -14.056 | -20.500 | 27.720 | 1.00 | 20.45 | C |
| ATOM | 1526 | C   | TYR  | A | 192 | -14.625 | -19.820 | 28.919 | 1.00 | 20.04 | C |
| ATOM | 1527 | O   | TYR  | A | 192 | -15.005 | -18.656 | 28.856 | 1.00 | 19.87 | O |
| ATOM | 1528 | CB  | TYR  | A | 192 | -12.675 | -19.917 | 27.383 | 1.00 | 18.56 | C |
| ATOM | 1529 | CG  | TYR  | A | 192 | -11.967 | -20.753 | 26.357 | 1.00 | 18.86 | C |
| ATOM | 1530 | CD1 | TYR  | A | 192 | -11.051 | -21.720 | 26.758 | 1.00 | 16.84 | C |
| ATOM | 1531 | CD2 | TYR  | A | 192 | -12.228 | -20.605 | 25.001 | 1.00 | 18.03 | C |
| ATOM | 1532 | CE1 | TYR  | A | 192 | -10.431 | -22.542 | 25.825 | 1.00 | 18.57 | C |
| ATOM | 1533 | CE2 | TYR  | A | 192 | -11.626 | -21.444 | 24.060 | 1.00 | 21.74 | C |
| ATOM | 1534 | CZ  | TYR  | A | 192 | -10.719 | -22.388 | 24.475 | 1.00 | 17.12 | C |
| ATOM | 1535 | OH  | TYR  | A | 192 | -10.101 | -23.199 | 23.545 | 1.00 | 18.39 | O |
| ATOM | 1536 | N   | THR  | A | 193 | -14.616 | -20.527 | 30.037 | 1.00 | 20.18 | N |
| ATOM | 1537 | CA  | THR  | A | 193 | -15.192 | -19.997 | 31.260 | 1.00 | 20.30 | C |
| ATOM | 1538 | C   | THR  | A | 193 | -14.248 | -20.255 | 32.435 | 1.00 | 19.95 | C |
| ATOM | 1539 | O   | THR  | A | 193 | -13.682 | -21.343 | 32.564 | 1.00 | 20.61 | O |
| ATOM | 1540 | CB  | THR  | A | 193 | -16.573 | -20.688 | 31.510 | 1.00 | 20.91 | C |
| ATOM | 1541 | OG1 | THR  | A | 193 | -17.584 | -20.099 | 30.662 | 1.00 | 20.27 | O |
| ATOM | 1542 | CG2 | THR  | A | 193 | -17.010 | -20.548 | 32.926 | 1.00 | 22.55 | C |
| ATOM | 1543 | N   | CYS  | A | 194 | -14.082 | -19.241 | 33.266 | 1.00 | 20.11 | N |
| ATOM | 1544 | CA  | ACYS | A | 194 | -13.448 | -19.332 | 34.576 | 0.50 | 20.29 | C |
| ATOM | 1545 | CA  | BCYS | A | 194 | -13.476 | -19.434 | 34.580 | 0.50 | 21.91 | C |
| ATOM | 1546 | C   | CYS  | A | 194 | -14.522 | -19.117 | 35.645 | 1.00 | 22.63 | C |
| ATOM | 1547 | O   | CYS  | A | 194 | -15.267 | -18.150 | 35.545 | 1.00 | 22.22 | O |
| ATOM | 1548 | CB  | ACYS | A | 194 | -12.385 | -18.225 | 34.654 | 0.50 | 19.89 | C |
| ATOM | 1549 | CB  | BCYS | A | 194 | -12.151 | -18.656 | 34.751 | 0.50 | 23.40 | C |
| ATOM | 1550 | SG  | ACYS | A | 194 | -11.570 | -17.997 | 36.228 | 0.50 | 20.69 | S |

```
ATOM   1551  SG  BCYS A 194   -12.214 -16.908  35.015  0.50 28.34        S
ATOM   1552  N    GLU A 195   -14.582 -19.996  36.649  1.00 24.47        N
ATOM   1553  CA   GLU A 195   -15.583 -19.952  37.704  1.00 25.72        C
ATOM   1554  C    GLU A 195   -14.832 -19.950  39.022  1.00 26.05        C
ATOM   1555  O    GLU A 195   -13.980 -20.803  39.280  1.00 25.96        O
ATOM   1556  CB   GLU A 195   -16.536 -21.150  37.684  1.00 27.94        C
ATOM   1557  CG   GLU A 195   -17.321 -21.322  36.402  1.00 30.72        C
ATOM   1558  CD   GLU A 195   -18.360 -22.458  36.432  1.00 33.09        C
ATOM   1559  OE1  GLU A 195   -18.381 -23.252  37.386  1.00 35.14        O
ATOM   1560  OE2  GLU A 195   -19.149 -22.556  35.463  1.00 33.81        O
ATOM   1561  N    ALA A 196   -15.162 -18.972  39.835  1.00 25.38        N
ATOM   1562  CA   ALA A 196   -14.563 -18.804  41.155  1.00 25.71        C
ATOM   1563  C    ALA A 196   -15.584 -19.097  42.238  1.00 26.89        C
ATOM   1564  O    ALA A 196   -16.685 -18.549  42.214  1.00 27.89        O
ATOM   1565  CB   ALA A 196   -14.056 -17.397  41.307  1.00 25.75        C
ATOM   1566  N    THR A 197   -15.218 -19.956  43.187  1.00 28.56        N
ATOM   1567  CA   THR A 197   -16.106 -20.337  44.275  1.00 29.62        C
ATOM   1568  C    THR A 197   -15.407 -19.910  45.551  1.00 30.78        C
ATOM   1569  O    THR A 197   -14.305 -20.365  45.825  1.00 30.48        O
ATOM   1570  CB   THR A 197   -16.375 -21.857  44.299  1.00 30.86        C
ATOM   1571  OG1  THR A 197   -16.987 -22.261  43.067  1.00 31.68        O
ATOM   1572  CG2  THR A 197   -17.325 -22.222  45.476  1.00 31.98        C
ATOM   1573  N    HIS A 198   -16.033 -19.000  46.291  1.00 31.86        N
ATOM   1574  CA   HIS A 198   -15.475 -18.423  47.521  1.00 32.55        C
ATOM   1575  C    HIS A 198   -16.534 -18.479  48.622  1.00 33.57        C
ATOM   1576  O    HIS A 198   -17.708 -18.476  48.315  1.00 33.61        O
ATOM   1577  CB   HIS A 198   -15.069 -16.987  47.242  1.00 32.34        C
ATOM   1578  CG   HIS A 198   -14.283 -16.345  48.341  1.00 34.81        C
ATOM   1579  ND1  HIS A 198   -14.796 -15.334  49.124  1.00 33.58        N
ATOM   1580  CD2  HIS A 198   -13.012 -16.540  48.763  1.00 34.19        C
ATOM   1581  CE1  HIS A 198   -13.883 -14.947  49.996  1.00 36.44        C
ATOM   1582  NE2  HIS A 198   -12.788 -15.662  49.798  1.00 34.94        N
ATOM   1583  N    LYS A 199   -16.120 -18.514  49.894  1.00 35.42        N
ATOM   1584  CA   LYS A 199   -17.079 -18.698  51.007  1.00 37.40        C
ATOM   1585  C    LYS A 199   -18.142 -17.599  51.052  1.00 38.23        C
ATOM   1586  O    LYS A 199   -19.201 -17.773  51.659  1.00 40.33        O
ATOM   1587  CB   LYS A 199   -16.350 -18.778  52.367  1.00 37.97        C
ATOM   1588  CG   LYS A 199   -15.782 -17.457  52.855  1.00 38.95        C
ATOM   1589  CD   LYS A 199   -15.062 -17.610  54.197  1.00 41.57        C
ATOM   1590  CE   LYS A 199   -16.022 -17.889  55.356  1.00 41.59        C
ATOM   1591  NZ   LYS A 199   -15.284 -17.791  56.667  1.00 42.72        N
ATOM   1592  N    THR A 200   -17.845 -16.462  50.431  1.00 38.55        N
ATOM   1593  CA   THR A 200   -18.757 -15.330  50.396  1.00 38.87        C
ATOM   1594  C    THR A 200   -20.016 -15.544  49.546  1.00 39.54        C
ATOM   1595  O    THR A 200   -20.908 -14.681  49.531  1.00 40.10        O
ATOM   1596  CB   THR A 200   -18.043 -14.107  49.884  1.00 39.07        C
ATOM   1597  OG1  THR A 200   -17.326 -14.475  48.701  1.00 41.19        O
ATOM   1598  CG2  THR A 200   -17.075 -13.598  50.928  1.00 39.35        C
ATOM   1599  N    SER A 201   -20.098 -16.666  48.840  1.00 38.84        N
ATOM   1600  CA   SER A 201   -21.356 -17.073  48.226  1.00 38.63        C
ATOM   1601  C    SER A 201   -21.383 -18.542  47.820  1.00 38.09        C
ATOM   1602  O    SER A 201   -20.364 -19.155  47.458  1.00 37.94        O
ATOM   1603  CB   SER A 201   -21.706 -16.185  47.034  1.00 37.85        C
ATOM   1604  OG   SER A 201   -22.743 -16.777  46.275  1.00 38.30        O
ATOM   1605  N    THR A 202   -22.575 -19.109  47.883  1.00 38.02        N
ATOM   1606  CA   THR A 202   -22.810 -20.459  47.381  1.00 38.21        C
ATOM   1607  C    THR A 202   -22.829 -20.445  45.846  1.00 36.32        C
ATOM   1608  O    THR A 202   -22.746 -21.484  45.222  1.00 36.64        O
ATOM   1609  CB   THR A 202   -24.144 -21.029  47.904  1.00 39.19        C
ATOM   1610  OG1  THR A 202   -25.163 -20.024  47.777  1.00 42.27        O
ATOM   1611  CG2  THR A 202   -24.028 -21.412  49.379  1.00 40.22        C
ATOM   1612  N    SER A 203   -22.960 -19.268  45.252  1.00 34.82        N
ATOM   1613  CA   SER A 203   -22.971 -19.126  43.800  1.00 35.91        C
ATOM   1614  C    SER A 203   -21.558 -18.875  43.272  1.00 34.10        C
ATOM   1615  O    SER A 203   -20.938 -17.899  43.654  1.00 34.49        O
```

```
ATOM   1616  CB  SER A 203    -23.842 -17.925  43.401  1.00 36.55          C
ATOM   1617  OG  SER A 203    -25.176 -18.083  43.852  1.00 39.58          O
ATOM   1618  N   PRO A 204    -21.050 -19.742  42.383  1.00 33.05          N
ATOM   1619  CA  PRO A 204    -19.771 -19.333  41.785  1.00 32.16          C
ATOM   1620  C   PRO A 204    -19.896 -18.070  40.954  1.00 30.99          C
ATOM   1621  O   PRO A 204    -20.961 -17.777  40.415  1.00 31.30          O
ATOM   1622  CB  PRO A 204    -19.378 -20.540  40.919  1.00 32.28          C
ATOM   1623  CG  PRO A 204    -20.618 -21.259  40.663  1.00 34.32          C
ATOM   1624  CD  PRO A 204    -21.493 -21.047  41.876  1.00 33.56          C
ATOM   1625  N   ILE A 205    -18.820 -17.306  40.889  1.00 28.52          N
ATOM   1626  CA  ILE A 205    -18.747 -16.124  40.058  1.00 27.83          C
ATOM   1627  C   ILE A 205    -18.153 -16.532  38.720  1.00 27.89          C
ATOM   1628  O   ILE A 205    -17.072 -17.096  38.671  1.00 25.02          O
ATOM   1629  CB  ILE A 205    -17.871 -15.047  40.705  1.00 26.82          C
ATOM   1630  CG1 ILE A 205    -18.516 -14.568  42.010  1.00 27.82          C
ATOM   1631  CG2 ILE A 205    -17.635 -13.877  39.736  1.00 27.10          C
ATOM   1632  CD1 ILE A 205    -17.542 -14.008  42.982  1.00 27.76          C
ATOM   1633  N   VAL A 206    -18.868 -16.259  37.630  1.00 29.06          N
ATOM   1634  CA  VAL A 206    -18.536 -16.834  36.319  1.00 29.06          C
ATOM   1635  C   VAL A 206    -18.109 -15.728  35.362  1.00 29.24          C
ATOM   1636  O   VAL A 206    -18.764 -14.690  35.283  1.00 30.45          O
ATOM   1637  CB  VAL A 206    -19.750 -17.584  35.717  1.00 29.74          C
ATOM   1638  CG1 VAL A 206    -19.447 -18.052  34.304  1.00 29.74          C
ATOM   1639  CG2 VAL A 206    -20.167 -18.755  36.623  1.00 32.31          C
ATOM   1640  N   LYS A 207    -16.995 -15.944  34.651  1.00 26.26          N
ATOM   1641  CA  LYS A 207    -16.567 -15.028  33.602  1.00 25.94          C
ATOM   1642  C   LYS A 207    -16.254 -15.866  32.357  1.00 24.53          C
ATOM   1643  O   LYS A 207    -15.726 -16.956  32.462  1.00 24.22          O
ATOM   1644  CB  LYS A 207    -15.389 -14.178  34.040  1.00 27.20          C
ATOM   1645  CG  LYS A 207    -15.710 -13.171  35.159  1.00 27.77          C
ATOM   1646  CD  LYS A 207    -16.614 -12.050  34.663  1.00 29.34          C
ATOM   1647  CE  LYS A 207    -16.914 -11.033  35.745  1.00 32.55          C
ATOM   1648  NZ  LYS A 207    -17.424  -9.757  35.148  1.00 34.09          N
ATOM   1649  N   SER A 208    -16.651 -15.369  31.196  1.00 23.52          N
ATOM   1650  CA  SER A 208    -16.543 -16.146  29.957  1.00 22.26          C
ATOM   1651  C   SER A 208    -16.112 -15.296  28.783  1.00 21.08          C
ATOM   1652  O   SER A 208    -16.228 -14.057  28.795  1.00 18.99          O
ATOM   1653  CB  SER A 208    -17.907 -16.768  29.599  1.00 21.95          C
ATOM   1654  OG  SER A 208    -18.408 -17.590  30.611  1.00 22.69          O
ATOM   1655  N   PHE A 209    -15.552 -15.960  27.779  1.00 20.55          N
ATOM   1656  CA  PHE A 209    -15.360 -15.357  26.460  1.00 21.36          C
ATOM   1657  C   PHE A 209    -15.553 -16.401  25.380  1.00 19.04          C
ATOM   1658  O   PHE A 209    -15.519 -17.617  25.642  1.00 21.99          O
ATOM   1659  CB  PHE A 209    -13.984 -14.671  26.317  1.00 20.87          C
ATOM   1660  CG  PHE A 209    -12.821 -15.611  26.289  1.00 20.68          C
ATOM   1661  CD1 PHE A 209    -12.388 -16.185  25.093  1.00 20.67          C
ATOM   1662  CD2 PHE A 209    -12.160 -15.941  27.457  1.00 20.70          C
ATOM   1663  CE1 PHE A 209    -11.342 -17.019  25.072  1.00 19.97          C
ATOM   1664  CE2 PHE A 209    -11.100 -16.803  27.441  1.00 20.79          C
ATOM   1665  CZ  PHE A 209    -10.698 -17.355  26.239  1.00 18.78          C
ATOM   1666  N   ASN A 210    -15.745 -15.916  24.167  1.00 19.67          N
ATOM   1667  CA  ASN A 210    -15.762 -16.773  23.014  1.00 20.99          C
ATOM   1668  C   ASN A 210    -14.590 -16.480  22.112  1.00 21.48          C
ATOM   1669  O   ASN A 210    -14.336 -15.330  21.755  1.00 17.91          O
ATOM   1670  CB  ASN A 210    -17.069 -16.632  22.206  1.00 21.21          C
ATOM   1671  CG  ASN A 210    -17.295 -17.792  21.264  1.00 23.75          C
ATOM   1672  OD1 ASN A 210    -16.723 -17.873  20.152  1.00 25.20          O
ATOM   1673  ND2 ASN A 210    -18.156 -18.709  21.687  1.00 22.64          N
ATOM   1674  N   ARG A 211    -13.919 -17.538  21.696  1.00 21.11          N
ATOM   1675  CA  ARG A 211    -12.724 -17.438  20.874  1.00 22.51          C
ATOM   1676  C   ARG A 211    -12.935 -16.644  19.565  1.00 23.82          C
ATOM   1677  O   ARG A 211    -12.040 -15.908  19.144  1.00 23.42          O
ATOM   1678  CB  ARG A 211    -12.208 -18.856  20.571  1.00 22.04          C
ATOM   1679  CG  ARG A 211    -10.943 -18.945  19.762  1.00 22.30          C
ATOM   1680  CD  ARG A 211    -10.562 -20.406  19.559  1.00 23.56          C
```

```
ATOM   1681   NE   ARG A 211   -11.459 -21.007  18.602  1.00 24.22          N
ATOM   1682   CZ   ARG A 211   -11.347 -20.886  17.273  1.00 26.59          C
ATOM   1683   NH1  ARG A 211   -10.325 -20.243  16.706  1.00 29.62          N
ATOM   1684   NH2  ARG A 211   -12.235 -21.443  16.504  1.00 29.64          N
ATOM   1685   N    ASN A 212   -14.094 -16.791  18.934  1.00 24.54          N
ATOM   1686   CA   ASN A 212   -14.332 -16.182  17.608  1.00 27.59          C
ATOM   1687   C    ASN A 212   -14.891 -14.744  17.647  1.00 28.27          C
ATOM   1688   O    ASN A 212   -15.220 -14.193  16.602  1.00 30.25          O
ATOM   1689   CB   ASN A 212   -15.231 -17.076  16.739  1.00 28.07          C
ATOM   1690   CG   ASN A 212   -14.499 -18.269  16.154  1.00 30.78          C
ATOM   1691   OD1  ASN A 212   -13.379 -18.136  15.636  1.00 30.84          O
ATOM   1692   ND2  ASN A 212   -15.141 -19.439  16.201  1.00 30.05          N
ATOM   1693   N    GLU A 213   -14.998 -14.132  18.826  1.00 28.27          N
ATOM   1694   CA   GLU A 213   -15.610 -12.805  18.927  1.00 29.06          C
ATOM   1695   C    GLU A 213   -14.570 -11.698  18.923  1.00 29.89          C
ATOM   1696   O    GLU A 213   -13.588 -11.836  19.637  1.00 32.66          O
ATOM   1697   CB   GLU A 213   -16.449 -12.729  20.192  1.00 28.74          C
ATOM   1698   CG   GLU A 213   -17.167 -11.420  20.358  1.00 27.13          C
ATOM   1699   CD   GLU A 213   -18.224 -11.483  21.402  1.00 26.77          C
ATOM   1700   OE1  GLU A 213   -18.193 -12.450  22.208  1.00 29.30          O
ATOM   1701   OE2  GLU A 213   -19.074 -10.575  21.428  1.00 25.64          O
TER    1702        GLU A 213
ATOM   1703   N    GLU B   1    20.300   7.638  55.322  1.00 22.55          N
ATOM   1704   CA   GLU B   1    20.841   6.725  56.358  1.00 22.12          C
ATOM   1705   C    GLU B   1    21.984   5.846  55.844  1.00 21.54          C
ATOM   1706   O    GLU B   1    22.352   5.887  54.672  1.00 20.93          O
ATOM   1707   CB   GLU B   1    19.708   5.865  56.941  1.00 24.34          C
ATOM   1708   CG   GLU B   1    19.183   6.383  58.297  1.00 28.22          C
ATOM   1709   CD   GLU B   1    20.147   6.164  59.463  1.00 31.29          C
ATOM   1710   OE1  GLU B   1    21.300   5.676  59.289  1.00 26.67          O
ATOM   1711   OE2  GLU B   1    19.735   6.542  60.588  1.00 38.40          O
ATOM   1712   N    VAL B   2    22.531   5.054  56.753  1.00 21.29          N
ATOM   1713   CA   VAL B   2    23.439   3.980  56.409  1.00 21.39          C
ATOM   1714   C    VAL B   2    22.772   3.181  55.277  1.00 21.25          C
ATOM   1715   O    VAL B   2    21.580   2.927  55.299  1.00 21.31          O
ATOM   1716   CB   VAL B   2    23.724   3.086  57.626  1.00 20.81          C
ATOM   1717   CG1  VAL B   2    24.482   1.801  57.197  1.00 21.94          C
ATOM   1718   CG2  VAL B   2    24.513   3.841  58.654  1.00 22.11          C
ATOM   1719   N    LYS B   3    23.550   2.830  54.271  1.00 20.81          N
ATOM   1720   CA   LYS B   3    23.022   2.122  53.135  1.00 21.56          C
ATOM   1721   C    LYS B   3    23.993   1.008  52.698  1.00 19.60          C
ATOM   1722   O    LYS B   3    25.192   1.215  52.578  1.00 19.23          O
ATOM   1723   CB   LYS B   3    22.729   3.026  51.961  1.00 22.34          C
ATOM   1724   CG   LYS B   3    22.089   2.290  50.804  1.00 25.11          C
ATOM   1725   CD   LYS B   3    21.645   3.199  49.687  1.00 26.85          C
ATOM   1726   CE   LYS B   3    21.047   2.377  48.522  1.00 27.55          C
ATOM   1727   NZ   LYS B   3    21.130   3.203  47.285  1.00 32.04          N
ATOM   1728   N    VAL B   4    23.430  -0.178  52.500  1.00 20.77          N
ATOM   1729   CA   VAL B   4    24.198  -1.346  52.127  1.00 18.41          C
ATOM   1730   C    VAL B   4    23.647  -1.773  50.748  1.00 16.57          C
ATOM   1731   O    VAL B   4    22.445  -1.949  50.583  1.00 16.80          O
ATOM   1732   CB   VAL B   4    23.991  -2.493  53.176  1.00 17.11          C
ATOM   1733   CG1  VAL B   4    24.686  -3.784  52.725  1.00 18.64          C
ATOM   1734   CG2  VAL B   4    24.432  -2.071  54.598  1.00 18.69          C
ATOM   1735   N    GLU B   5    24.514  -1.911  49.754  1.00 18.56          N
ATOM   1736   CA   GLU B   5    24.059  -2.273  48.398  1.00 18.79          C
ATOM   1737   C    GLU B   5    24.916  -3.344  47.749  1.00 15.93          C
ATOM   1738   O    GLU B   5    26.065  -3.124  47.397  1.00 14.85          O
ATOM   1739   CB   GLU B   5    24.106  -1.054  47.501  1.00 20.52          C
ATOM   1740   CG   GLU B   5    23.809  -1.370  46.099  1.00 25.39          C
ATOM   1741   CD   GLU B   5    23.596  -0.143  45.280  1.00 29.78          C
ATOM   1742   OE1  GLU B   5    24.487   0.737  45.313  1.00 34.09          O
ATOM   1743   OE2  GLU B   5    22.554  -0.066  44.596  1.00 34.74          O
ATOM   1744   N    GLU B   6    24.311  -4.515  47.545  1.00 17.74          N
ATOM   1745   CA   GLU B   6    25.007  -5.636  46.939  1.00 17.23          C
```

60

| | | | | | | | | | | | |
|---|---|---|---|---|---|---|---|---|---|---|---|
| ATOM | 1746 | C | GLU | B | 6 | 24.932 | -5.583 | 45.419 | 1.00 | 16.67 | C |
| ATOM | 1747 | O | GLU | B | 6 | 24.054 | -4.922 | 44.823 | 1.00 | 17.92 | O |
| ATOM | 1748 | CB | GLU | B | 6 | 24.361 | -6.935 | 47.391 | 1.00 | 16.36 | C |
| ATOM | 1749 | CG | GLU | B | 6 | 24.302 | -7.200 | 48.859 | 1.00 | 17.81 | C |
| ATOM | 1750 | CD | GLU | B | 6 | 23.108 | -6.549 | 49.580 | 1.00 | 17.33 | C |
| ATOM | 1751 | OE1 | GLU | B | 6 | 22.338 | -5.741 | 48.997 | 1.00 | 17.74 | O |
| ATOM | 1752 | OE2 | GLU | B | 6 | 22.933 | -6.820 | 50.784 | 1.00 | 23.19 | O |
| ATOM | 1753 | N | SER | B | 7 | 25.855 | -6.292 | 44.789 | 1.00 | 18.42 | N |
| ATOM | 1754 | CA | SER | B | 7 | 25.868 | -6.494 | 43.355 | 1.00 | 18.38 | C |
| ATOM | 1755 | C | SER | B | 7 | 26.619 | -7.756 | 43.012 | 1.00 | 16.70 | C |
| ATOM | 1756 | O | SER | B | 7 | 27.269 | -8.346 | 43.886 | 1.00 | 19.00 | O |
| ATOM | 1757 | CB | SER | B | 7 | 26.544 | -5.316 | 42.628 | 1.00 | 17.68 | C |
| ATOM | 1758 | OG | SER | B | 7 | 27.845 | -5.062 | 43.101 | 1.00 | 19.20 | O |
| ATOM | 1759 | N | GLY | B | 8 | 26.501 | -8.175 | 41.752 | 1.00 | 17.51 | N |
| ATOM | 1760 | CA | GLY | B | 8 | 27.275 | -9.304 | 41.233 | 1.00 | 18.39 | C |
| ATOM | 1761 | C | GLY | B | 8 | 26.569 | -10.653 | 41.004 | 1.00 | 18.46 | C |
| ATOM | 1762 | O | GLY | B | 8 | 27.199 | -11.577 | 40.504 | 1.00 | 19.70 | O |
| ATOM | 1763 | N | GLY | B | 9 | 25.308 | -10.745 | 41.395 | 1.00 | 18.56 | N |
| ATOM | 1764 | CA | GLY | B | 9 | 24.485 | -11.948 | 41.225 | 1.00 | 18.09 | C |
| ATOM | 1765 | C | GLY | B | 9 | 24.209 | -12.253 | 39.758 | 1.00 | 17.77 | C |
| ATOM | 1766 | O | GLY | B | 9 | 24.558 | -11.470 | 38.846 | 1.00 | 20.47 | O |
| ATOM | 1767 | N | GLY | B | 10 | 23.593 | -13.406 | 39.516 | 1.00 | 18.09 | N |
| ATOM | 1768 | CA | GLY | B | 10 | 23.362 | -13.920 | 38.195 | 1.00 | 16.56 | C |
| ATOM | 1769 | C | GLY | B | 10 | 23.397 | -15.416 | 38.301 | 1.00 | 15.48 | C |
| ATOM | 1770 | O | GLY | B | 10 | 23.356 | -15.959 | 39.370 | 1.00 | 14.95 | O |
| ATOM | 1771 | N | LEU | B | 11 | 23.542 | -16.037 | 37.150 | 1.00 | 17.15 | N |
| ATOM | 1772 | CA | LEU | B | 11 | 23.510 | -17.480 | 37.006 | 1.00 | 17.93 | C |
| ATOM | 1773 | C | LEU | B | 11 | 24.889 | -18.087 | 36.753 | 1.00 | 18.22 | C |
| ATOM | 1774 | O | LEU | B | 11 | 25.720 | -17.500 | 36.071 | 1.00 | 15.01 | O |
| ATOM | 1775 | CB | LEU | B | 11 | 22.589 | -17.792 | 35.835 | 1.00 | 16.45 | C |
| ATOM | 1776 | CG | LEU | B | 11 | 22.397 | -19.235 | 35.384 | 1.00 | 18.59 | C |
| ATOM | 1777 | CD1 | LEU | B | 11 | 21.600 | -19.965 | 36.411 | 1.00 | 18.48 | C |
| ATOM | 1778 | CD2 | LEU | B | 11 | 21.686 | -19.273 | 34.035 | 1.00 | 19.67 | C |
| ATOM | 1779 | N | VAL | B | 12 | 25.136 | -19.256 | 37.349 | 1.00 | 18.32 | N |
| ATOM | 1780 | CA | VAL | B | 12 | 26.311 | -20.081 | 36.991 | 1.00 | 16.96 | C |
| ATOM | 1781 | C | VAL | B | 12 | 25.929 | -21.551 | 37.133 | 1.00 | 17.05 | C |
| ATOM | 1782 | O | VAL | B | 12 | 24.928 | -21.885 | 37.744 | 1.00 | 16.91 | O |
| ATOM | 1783 | CB | VAL | B | 12 | 27.582 | -19.752 | 37.841 | 1.00 | 17.29 | C |
| ATOM | 1784 | CG1 | VAL | B | 12 | 27.445 | -20.249 | 39.275 | 1.00 | 17.38 | C |
| ATOM | 1785 | CG2 | VAL | B | 12 | 28.873 | -20.251 | 37.165 | 1.00 | 17.50 | C |
| ATOM | 1786 | N | GLN | B | 13 | 26.708 | -22.374 | 36.474 | 1.00 | 15.86 | N |
| ATOM | 1787 | CA | GLN | B | 13 | 26.481 | -23.824 | 36.423 | 1.00 | 15.47 | C |
| ATOM | 1788 | C | GLN | B | 13 | 27.007 | -24.459 | 37.682 | 1.00 | 16.40 | C |
| ATOM | 1789 | O | GLN | B | 13 | 27.953 | -23.991 | 38.299 | 1.00 | 15.02 | O |
| ATOM | 1790 | CB | GLN | B | 13 | 27.230 | -24.395 | 35.228 | 1.00 | 16.57 | C |
| ATOM | 1791 | CG | GLN | B | 13 | 26.736 | -23.881 | 33.889 | 1.00 | 16.04 | C |
| ATOM | 1792 | CD | GLN | B | 13 | 27.168 | -22.475 | 33.576 | 1.00 | 17.34 | C |
| ATOM | 1793 | OE1 | GLN | B | 13 | 28.244 | -22.069 | 33.961 | 1.00 | 17.52 | O |
| ATOM | 1794 | NE2 | GLN | B | 13 | 26.334 | -21.741 | 32.847 | 1.00 | 19.16 | N |
| ATOM | 1795 | N | PRO | B | 14 | 26.428 | -25.590 | 38.066 | 1.00 | 16.03 | N |
| ATOM | 1796 | CA | PRO | B | 14 | 26.996 | -26.296 | 39.223 | 1.00 | 16.77 | C |
| ATOM | 1797 | C | PRO | B | 14 | 28.481 | -26.610 | 39.023 | 1.00 | 16.70 | C |
| ATOM | 1798 | O | PRO | B | 14 | 28.887 | -26.981 | 37.923 | 1.00 | 18.38 | O |
| ATOM | 1799 | CB | PRO | B | 14 | 26.151 | -27.569 | 39.284 | 1.00 | 17.78 | C |
| ATOM | 1800 | CG | PRO | B | 14 | 24.919 | -27.233 | 38.588 | 1.00 | 18.31 | C |
| ATOM | 1801 | CD | PRO | B | 14 | 25.227 | -26.223 | 37.540 | 1.00 | 17.54 | C |
| ATOM | 1802 | N | GLY | B | 15 | 29.285 | -26.409 | 40.068 | 1.00 | 16.07 | N |
| ATOM | 1803 | CA | GLY | B | 15 | 30.698 | -26.622 | 40.048 | 1.00 | 16.74 | C |
| ATOM | 1804 | C | GLY | B | 15 | 31.433 | -25.349 | 39.720 | 1.00 | 15.10 | C |
| ATOM | 1805 | O | GLY | B | 15 | 32.613 | -25.275 | 39.904 | 1.00 | 16.68 | O |
| ATOM | 1806 | N | GLY | B | 16 | 30.682 | -24.354 | 39.262 | 1.00 | 16.84 | N |
| ATOM | 1807 | CA | GLY | B | 16 | 31.245 | -23.058 | 38.946 | 1.00 | 16.43 | C |
| ATOM | 1808 | C | GLY | B | 16 | 31.344 | -22.127 | 40.131 | 1.00 | 16.66 | C |
| ATOM | 1809 | O | GLY | B | 16 | 31.145 | -22.523 | 41.270 | 1.00 | 16.74 | O |
| ATOM | 1810 | N | SER | B | 17 | 31.628 | -20.863 | 39.832 | 1.00 | 16.19 | N |

| ATOM | 1811 | CA  | SER | B | 17 | 32.007 | -19.902 | 40.829 | 1.00 | 18.07 | C |
| ATOM | 1812 | C   | SER | B | 17 | 31.288 | -18.586 | 40.584 | 1.00 | 18.13 | C |
| ATOM | 1813 | O   | SER | B | 17 | 30.854 | -18.279 | 39.479 | 1.00 | 18.37 | O |
| ATOM | 1814 | CB  | SER | B | 17 | 33.510 | -19.596 | 40.708 | 1.00 | 19.36 | C |
| ATOM | 1815 | OG  | SER | B | 17 | 34.283 | -20.708 | 41.126 | 1.00 | 27.44 | O |
| ATOM | 1816 | N   | MET | B | 18 | 31.176 | -17.806 | 41.643 | 1.00 | 17.15 | N |
| ATOM | 1817 | CA  | MET | B | 18 | 30.650 | -16.443 | 41.513 | 1.00 | 17.55 | C |
| ATOM | 1818 | C   | MET | B | 18 | 31.412 | -15.577 | 42.463 | 1.00 | 17.12 | C |
| ATOM | 1819 | O   | MET | B | 18 | 31.865 | -16.019 | 43.476 | 1.00 | 16.97 | O |
| ATOM | 1820 | CB  | MET | B | 18 | 29.194 | -16.357 | 41.942 | 1.00 | 18.18 | C |
| ATOM | 1821 | CG  | MET | B | 18 | 28.197 | -17.133 | 41.143 | 1.00 | 21.98 | C |
| ATOM | 1822 | SD  | MET | B | 18 | 27.708 | -16.316 | 39.619 | 1.00 | 23.52 | S |
| ATOM | 1823 | CE  | MET | B | 18 | 26.760 | -14.939 | 40.314 | 1.00 | 25.05 | C |
| ATOM | 1824 | N   | LYS | B | 19 | 31.501 | -14.294 | 42.156 | 1.00 | 17.36 | N |
| ATOM | 1825 | CA  | LYS | B | 19 | 32.073 | -13.373 | 43.116 | 1.00 | 17.38 | C |
| ATOM | 1826 | C   | LYS | B | 19 | 31.084 | -12.203 | 43.228 | 1.00 | 16.33 | C |
| ATOM | 1827 | O   | LYS | B | 19 | 30.750 | -11.541 | 42.237 | 1.00 | 19.47 | O |
| ATOM | 1828 | CB  | LYS | B | 19 | 33.463 | -12.866 | 42.702 | 1.00 | 18.51 | C |
| ATOM | 1829 | CG  | LYS | B | 19 | 34.023 | -11.838 | 43.676 | 1.00 | 23.00 | C |
| ATOM | 1830 | CD  | LYS | B | 19 | 35.484 | -11.565 | 43.443 | 1.00 | 25.48 | C |
| ATOM | 1831 | CE  | LYS | B | 19 | 36.158 | -10.912 | 44.654 | 1.00 | 28.50 | C |
| ATOM | 1832 | NZ  | LYS | B | 19 | 37.647 | -10.904 | 44.522 | 1.00 | 28.74 | N |
| ATOM | 1833 | N   | ILE | B | 20 | 30.554 | -12.005 | 44.425 | 1.00 | 14.36 | N |
| ATOM | 1834 | CA  | ILE | B | 20 | 29.603 | -10.906 | 44.664 | 1.00 | 13.53 | C |
| ATOM | 1835 | C   | ILE | B | 20 | 30.258 | -9.907  | 45.634 | 1.00 | 14.22 | C |
| ATOM | 1836 | O   | ILE | B | 20 | 31.247 | -10.218 | 46.321 | 1.00 | 12.27 | O |
| ATOM | 1837 | CB  | ILE | B | 20 | 28.201 | -11.441 | 45.148 | 1.00 | 14.39 | C |
| ATOM | 1838 | CG1 | ILE | B | 20 | 28.321 | -12.167 | 46.511 | 1.00 | 13.71 | C |
| ATOM | 1839 | CG2 | ILE | B | 20 | 27.617 | -12.370 | 44.060 | 1.00 | 13.43 | C |
| ATOM | 1840 | CD1 | ILE | B | 20 | 26.974 | -12.588 | 47.070 | 1.00 | 14.51 | C |
| ATOM | 1841 | N   | SER | B | 21 | 29.679 | -8.714  | 45.739 | 1.00 | 16.24 | N |
| ATOM | 1842 | CA  | SER | B | 21 | 30.211 | -7.668  | 46.629 | 1.00 | 17.55 | C |
| ATOM | 1843 | C   | SER | B | 21 | 29.076 | -6.748  | 47.144 | 1.00 | 15.53 | C |
| ATOM | 1844 | O   | SER | B | 21 | 27.920 | -6.804  | 46.687 | 1.00 | 17.36 | O |
| ATOM | 1845 | CB  | SER | B | 21 | 31.285 | -6.815  | 45.914 | 1.00 | 19.09 | C |
| ATOM | 1846 | OG  | SER | B | 21 | 30.692 | -6.001  | 44.933 | 1.00 | 22.08 | O |
| ATOM | 1847 | N   | CYS | B | 22 | 29.412 | -5.912  | 48.126 | 1.00 | 15.21 | N |
| ATOM | 1848 | CA  | CYS | B | 22 | 28.499 | -4.877  | 48.533 | 1.00 | 16.34 | C |
| ATOM | 1849 | C   | CYS | B | 22 | 29.327 | -3.674  | 48.926 | 1.00 | 16.54 | C |
| ATOM | 1850 | O   | CYS | B | 22 | 30.452 | -3.814  | 49.378 | 1.00 | 15.71 | O |
| ATOM | 1851 | CB  | CYS | B | 22 | 27.559 | -5.342  | 49.648 | 1.00 | 18.66 | C |
| ATOM | 1852 | SG  | CYS | B | 22 | 28.335 | -5.739  | 51.131 | 1.00 | 19.79 | S |
| ATOM | 1853 | N   | VAL | B | 23 | 28.735 | -2.498  | 48.735 | 1.00 | 17.83 | N |
| ATOM | 1854 | CA  | VAL | B | 23 | 29.342 | -1.232  | 49.166 | 1.00 | 17.60 | C |
| ATOM | 1855 | C   | VAL | B | 23 | 28.481 | -0.674  | 50.289 | 1.00 | 14.63 | C |
| ATOM | 1856 | O   | VAL | B | 23 | 27.256 | -0.745  | 50.246 | 1.00 | 15.83 | O |
| ATOM | 1857 | CB  | VAL | B | 23 | 29.557 | -0.243  | 47.982 | 1.00 | 17.97 | C |
| ATOM | 1858 | CG1 | VAL | B | 23 | 28.276 | 0.061   | 47.254 | 1.00 | 20.02 | C |
| ATOM | 1859 | CG2 | VAL | B | 23 | 30.284 | 0.994   | 48.508 | 1.00 | 21.91 | C |
| ATOM | 1860 | N   | VAL | B | 24 | 29.123 | -0.191  | 51.347 | 1.00 | 18.10 | N |
| ATOM | 1861 | CA  | VAL | B | 24 | 28.399 | 0.384   | 52.457 | 1.00 | 18.55 | C |
| ATOM | 1862 | C   | VAL | B | 24 | 28.785 | 1.873   | 52.562 | 1.00 | 19.50 | C |
| ATOM | 1863 | O   | VAL | B | 24 | 29.977 | 2.241   | 52.565 | 1.00 | 24.33 | O |
| ATOM | 1864 | CB  | VAL | B | 24 | 28.724 | -0.279  | 53.775 | 1.00 | 19.35 | C |
| ATOM | 1865 | CG1 | VAL | B | 24 | 27.861 | 0.247   | 54.869 | 1.00 | 20.01 | C |
| ATOM | 1866 | CG2 | VAL | B | 24 | 28.594 | -1.840  | 53.644 | 1.00 | 20.96 | C |
| ATOM | 1867 | N   | SER | B | 25 | 27.758 | 2.690   | 52.615 | 1.00 | 19.75 | N |
| ATOM | 1868 | CA  | SER | B | 25 | 27.908 | 4.136   | 52.750 | 1.00 | 19.48 | C |
| ATOM | 1869 | C   | SER | B | 25 | 27.203 | 4.627   | 54.012 | 1.00 | 18.47 | C |
| ATOM | 1870 | O   | SER | B | 25 | 26.395 | 3.940   | 54.595 | 1.00 | 20.38 | O |
| ATOM | 1871 | CB  | SER | B | 25 | 27.331 | 4.775   | 51.518 | 1.00 | 16.58 | C |
| ATOM | 1872 | OG  | SER | B | 25 | 25.990 | 4.414   | 51.297 | 1.00 | 19.18 | O |
| ATOM | 1873 | N   | GLY | B | 26 | 27.486 | 5.857   | 54.428 | 1.00 | 18.98 | N |
| ATOM | 1874 | CA  | GLY | B | 26 | 26.835 | 6.370   | 55.609 | 1.00 | 17.85 | C |
| ATOM | 1875 | C   | GLY | B | 26 | 27.476 | 6.084   | 56.956 | 1.00 | 17.16 | C |

```
ATOM   1876  O    GLY B  26    26.919   6.478  57.988  1.00  18.70         O
ATOM   1877  N    LEU B  27    28.666   5.486  56.961  1.00  17.78         N
ATOM   1878  CA   LEU B  27    29.433   5.229  58.145  1.00  18.59         C
ATOM   1879  C    LEU B  27    30.834   4.945  57.693  1.00  19.70         C
ATOM   1880  O    LEU B  27    31.095   4.929  56.473  1.00  21.46         O
ATOM   1881  CB   LEU B  27    28.820   4.048  58.923  1.00  19.97         C
ATOM   1882  CG   LEU B  27    28.504   2.770  58.121  1.00  19.64         C
ATOM   1883  CD1  LEU B  27    29.746   2.063  57.601  1.00  21.70         C
ATOM   1884  CD2  LEU B  27    27.700   1.840  59.004  1.00  18.41         C
ATOM   1885  N    THR B  28    31.747   4.699  58.630  1.00  17.78         N
ATOM   1886  CA   THR B  28    33.103   4.395  58.281  1.00  19.39         C
ATOM   1887  C    THR B  28    33.182   2.885  58.221  1.00  19.82         C
ATOM   1888  O    THR B  28    33.260   2.251  59.270  1.00  20.97         O
ATOM   1889  CB   THR B  28    34.088   4.915  59.309  1.00  19.36         C
ATOM   1890  OG1  THR B  28    34.023   6.367  59.292  1.00  22.79         O
ATOM   1891  CG2  THR B  28    35.517   4.492  58.987  1.00  19.90         C
ATOM   1892  N    PHE B  29    33.214   2.351  57.003  1.00  19.98         N
ATOM   1893  CA   PHE B  29    33.172   0.890  56.766  1.00  19.91         C
ATOM   1894  C    PHE B  29    34.220   0.092  57.524  1.00  19.81         C
ATOM   1895  O    PHE B  29    33.909  -0.945  58.135  1.00  17.87         O
ATOM   1896  CB   PHE B  29    33.278   0.665  55.264  1.00  18.55         C
ATOM   1897  CG   PHE B  29    33.394  -0.794  54.840  1.00  19.07         C
ATOM   1898  CD1  PHE B  29    32.264  -1.543  54.604  1.00  20.23         C
ATOM   1899  CD2  PHE B  29    34.647  -1.363  54.614  1.00  17.49         C
ATOM   1900  CE1  PHE B  29    32.345  -2.895  54.174  1.00  19.74         C
ATOM   1901  CE2  PHE B  29    34.735  -2.729  54.153  1.00  16.52         C
ATOM   1902  CZ   PHE B  29    33.617  -3.450  53.950  1.00  18.44         C
ATOM   1903  N    SER B  30    35.461   0.573  57.519  1.00  20.16         N
ATOM   1904  CA   SER B  30    36.586  -0.085  58.180  1.00  21.02         C
ATOM   1905  C    SER B  30    36.441  -0.348  59.687  1.00  19.52         C
ATOM   1906  O    SER B  30    37.193  -1.172  60.257  1.00  21.62         O
ATOM   1907  CB   SER B  30    37.860   0.726  57.936  1.00  22.24         C
ATOM   1908  OG   SER B  30    37.779   1.985  58.556  1.00  23.59         O
ATOM   1909  N    ASN B  31    35.491   0.288  60.337  1.00  19.22         N
ATOM   1910  CA   ASN B  31    35.325   0.100  61.769  1.00  19.86         C
ATOM   1911  C    ASN B  31    34.269  -0.920  62.173  1.00  20.56         C
ATOM   1912  O    ASN B  31    33.981  -1.033  63.365  1.00  20.80         O
ATOM   1913  CB   ASN B  31    35.023   1.460  62.434  1.00  21.47         C
ATOM   1914  CG   ASN B  31    36.191   2.408  62.364  1.00  23.63         C
ATOM   1915  OD1  ASN B  31    36.002   3.638  62.348  1.00  25.38         O
ATOM   1916  ND2  ASN B  31    37.398   1.874  62.398  1.00  20.90         N
ATOM   1917  N    TYR B  32    33.698  -1.654  61.200  1.00  18.65         N
ATOM   1918  CA   TYR B  32    32.592  -2.560  61.460  1.00  17.57         C
ATOM   1919  C    TYR B  32    32.874  -3.999  61.003  1.00  15.70         C
ATOM   1920  O    TYR B  32    33.424  -4.229  59.941  1.00  16.25         O
ATOM   1921  CB   TYR B  32    31.290  -2.092  60.811  1.00  19.02         C
ATOM   1922  CG   TYR B  32    30.877  -0.759  61.358  1.00  18.79         C
ATOM   1923  CD1  TYR B  32    30.144  -0.661  62.513  1.00  20.87         C
ATOM   1924  CD2  TYR B  32    31.303   0.413  60.738  1.00  20.46         C
ATOM   1925  CE1  TYR B  32    29.803   0.597  63.055  1.00  20.45         C
ATOM   1926  CE2  TYR B  32    30.984   1.676  61.275  1.00  21.02         C
ATOM   1927  CZ   TYR B  32    30.211   1.751  62.408  1.00  21.75         C
ATOM   1928  OH   TYR B  32    29.891   2.990  62.936  1.00  24.44         O
ATOM   1929  N    TRP B  33    32.467  -4.932  61.844  1.00  15.03         N
ATOM   1930  CA   TRP B  33    32.357  -6.349  61.415  1.00  15.25         C
ATOM   1931  C    TRP B  33    31.265  -6.421  60.359  1.00  15.09         C
ATOM   1932  O    TRP B  33    30.263  -5.705  60.416  1.00  15.58         O
ATOM   1933  CB   TRP B  33    31.971  -7.212  62.600  1.00  15.18         C
ATOM   1934  CG   TRP B  33    32.944  -7.289  63.766  1.00  17.62         C
ATOM   1935  CD1  TRP B  33    34.112  -6.628  63.924  1.00  18.23         C
ATOM   1936  CD2  TRP B  33    32.746  -8.055  64.934  1.00  17.92         C
ATOM   1937  NE1  TRP B  33    34.672  -6.936  65.137  1.00  18.56         N
ATOM   1938  CE2  TRP B  33    33.841  -7.825  65.777  1.00  18.15         C
ATOM   1939  CE3  TRP B  33    31.756  -8.950  65.346  1.00  19.20         C
ATOM   1940  CZ2  TRP B  33    33.973  -8.465  67.013  1.00  18.55         C
```

```
ATOM   1941  CZ3 TRP B  33    31.890  -9.573  66.595  1.00 19.41        C
ATOM   1942  CH2 TRP B  33    32.979  -9.301  67.406  1.00 17.52        C
ATOM   1943  N   MET B  34    31.453  -7.295  59.380  1.00 14.56        N
ATOM   1944  CA  MET B  34    30.586  -7.390  58.256  1.00 15.35        C
ATOM   1945  C   MET B  34    30.397  -8.888  57.968  1.00 13.84        C
ATOM   1946  O   MET B  34    31.329  -9.674  58.165  1.00 13.86        O
ATOM   1947  CB  MET B  34    31.239  -6.778  57.050  1.00 17.29        C
ATOM   1948  CG  MET B  34    31.236  -5.225  57.023  1.00 18.95        C
ATOM   1949  SD  MET B  34    29.570  -4.606  57.020  1.00 22.15        S
ATOM   1950  CE  MET B  34    28.871  -5.210  55.505  1.00 20.50        C
ATOM   1951  N   SER B  35    29.214  -9.188  57.507  1.00 14.53        N
ATOM   1952  CA  SER B  35    28.796 -10.587  57.214  1.00 15.12        C
ATOM   1953  C   SER B  35    27.945 -10.712  55.969  1.00 14.88        C
ATOM   1954  O   SER B  35    27.443  -9.727  55.442  1.00 14.15        O
ATOM   1955  CB  SER B  35    28.052 -11.157  58.403  1.00 17.12        C
ATOM   1956  OG  SER B  35    26.688 -10.791  58.414  1.00 17.08        O
ATOM   1957  N   TRP B  36    27.842 -11.963  55.465  1.00 13.38        N
ATOM   1958  CA  TRP B  36    26.913 -12.326  54.430  1.00 12.78        C
ATOM   1959  C   TRP B  36    25.928 -13.272  55.036  1.00 12.53        C
ATOM   1960  O   TRP B  36    26.339 -14.228  55.782  1.00 13.24        O
ATOM   1961  CB  TRP B  36    27.622 -13.027  53.269  1.00 12.60        C
ATOM   1962  CG  TRP B  36    28.524 -12.096  52.436  1.00 12.85        C
ATOM   1963  CD1 TRP B  36    29.842 -11.884  52.558  1.00 13.06        C
ATOM   1964  CD2 TRP B  36    28.078 -11.344  51.334  1.00 14.32        C
ATOM   1965  NE1 TRP B  36    30.282 -11.000  51.577  1.00 13.57        N
ATOM   1966  CE2 TRP B  36    29.201 -10.640  50.823  1.00 13.94        C
ATOM   1967  CE3 TRP B  36    26.831 -11.172  50.747  1.00 14.13        C
ATOM   1968  CZ2 TRP B  36    29.105  -9.756  49.729  1.00 15.74        C
ATOM   1969  CZ3 TRP B  36    26.732 -10.275  49.607  1.00 16.44        C
ATOM   1970  CH2 TRP B  36    27.887  -9.622  49.122  1.00 14.97        C
ATOM   1971  N   VAL B  37    24.679 -13.023  54.738  1.00 13.22        N
ATOM   1972  CA  VAL B  37    23.545 -13.848  55.135  1.00 14.18        C
ATOM   1973  C   VAL B  37    22.745 -14.113  53.859  1.00 15.72        C
ATOM   1974  O   VAL B  37    22.389 -13.187  53.123  1.00 14.42        O
ATOM   1975  CB  VAL B  37    22.635 -13.164  56.160  1.00 13.65        C
ATOM   1976  CG1 VAL B  37    21.456 -14.060  56.527  1.00 15.29        C
ATOM   1977  CG2 VAL B  37    23.399 -12.817  57.410  1.00 13.71        C
ATOM   1978  N   ARG B  38    22.501 -15.385  53.544  1.00 15.11        N
ATOM   1979  CA  ARG B  38    21.670 -15.719  52.388  1.00 15.08        C
ATOM   1980  C   ARG B  38    20.257 -16.195  52.805  1.00 14.02        C
ATOM   1981  O   ARG B  38    20.023 -16.609  53.925  1.00 14.95        O
ATOM   1982  CB  ARG B  38    22.369 -16.737  51.482  1.00 14.81        C
ATOM   1983  CG  ARG B  38    22.500 -18.054  52.089  1.00 16.54        C
ATOM   1984  CD  ARG B  38    23.437 -18.943  51.285  1.00 14.79        C
ATOM   1985  NE  ARG B  38    23.507 -20.277  51.899  1.00 17.32        N
ATOM   1986  CZ  ARG B  38    24.219 -21.257  51.368  1.00 16.91        C
ATOM   1987  NH1 ARG B  38    24.896 -21.038  50.252  1.00 16.08        N
ATOM   1988  NH2 ARG B  38    24.293 -22.456  51.967  1.00 18.33        N
ATOM   1989  N   GLN B  39    19.318 -16.075  51.899  1.00 16.13        N
ATOM   1990  CA  GLN B  39    17.952 -16.526  52.118  1.00 17.91        C
ATOM   1991  C   GLN B  39    17.440 -17.401  50.970  1.00 19.62        C
ATOM   1992  O   GLN B  39    17.628 -17.087  49.803  1.00 16.57        O
ATOM   1993  CB  GLN B  39    17.051 -15.314  52.272  1.00 18.75        C
ATOM   1994  CG  GLN B  39    15.668 -15.640  52.801  1.00 19.57        C
ATOM   1995  CD  GLN B  39    14.893 -14.383  53.179  1.00 21.84        C
ATOM   1996  OE1 GLN B  39    15.029 -13.332  52.558  1.00 19.89        O
ATOM   1997  NE2 GLN B  39    14.065 -14.505  54.189  1.00 21.31        N
ATOM   1998  N   SER B  40    16.843 -18.526  51.362  1.00 20.52        N
ATOM   1999  CA  SER B  40    16.277 -19.547  50.465  1.00 23.23        C
ATOM   2000  C   SER B  40    15.070 -20.183  51.140  1.00 24.48        C
ATOM   2001  O   SER B  40    14.938 -20.106  52.353  1.00 20.94        O
ATOM   2002  CB  SER B  40    17.300 -20.640  50.192  1.00 23.12        C
ATOM   2003  OG  SER B  40    17.650 -21.427  51.354  1.00 24.96        O
ATOM   2004  N   PRO B  41    14.170 -20.813  50.352  1.00 27.14        N
ATOM   2005  CA  PRO B  41    13.042 -21.586  50.896  1.00 28.58        C
```

| ATOM | 2006 | C | PRO | B | 41 | 13.455 | -22.685 | 51.884 | 1.00 | 28.58 | C |
|------|------|------|-----|---|----|--------|---------|--------|------|-------|---|
| ATOM | 2007 | O | PRO | B | 41 | 12.911 | -22.732 | 52.986 | 1.00 | 29.04 | O |
| ATOM | 2008 | CB | PRO | B | 41 | 12.399 | -22.195 | 49.635 | 1.00 | 28.57 | C |
| ATOM | 2009 | CG | PRO | B | 41 | 12.744 | -21.232 | 48.539 | 1.00 | 30.13 | C |
| ATOM | 2010 | CD | PRO | B | 41 | 14.135 | -20.750 | 48.881 | 1.00 | 28.50 | C |
| ATOM | 2011 | N | GLU | B | 42 | 14.430 | -23.525 | 51.530 | 1.00 | 29.56 | N |
| ATOM | 2012 | CA | GLU | B | 42 | 14.719 | -24.704 | 52.335 | 1.00 | 29.84 | C |
| ATOM | 2013 | C | GLU | B | 42 | 15.382 | -24.389 | 53.666 | 1.00 | 29.75 | C |
| ATOM | 2014 | O | GLU | B | 42 | 15.061 | -25.021 | 54.675 | 1.00 | 29.87 | O |
| ATOM | 2015 | CB | GLU | B | 42 | 15.527 | -25.753 | 51.545 | 1.00 | 30.19 | C |
| ATOM | 2016 | CG | GLU | B | 42 | 16.199 | -26.880 | 52.395 | 1.00 | 33.55 | C |
| ATOM | 2017 | CD | GLU | B | 42 | 15.263 | -27.964 | 52.927 | 1.00 | 34.57 | C |
| ATOM | 2018 | OE1 | GLU | B | 42 | 14.163 | -28.182 | 52.367 | 1.00 | 33.84 | O |
| ATOM | 2019 | OE2 | GLU | B | 42 | 15.662 | -28.652 | 53.914 | 1.00 | 38.14 | O |
| ATOM | 2020 | N | LYS | B | 43 | 16.267 | -23.386 | 53.692 | 1.00 | 27.22 | N |
| ATOM | 2021 | CA | LYS | B | 43 | 17.058 | -23.112 | 54.878 | 1.00 | 25.20 | C |
| ATOM | 2022 | C | LYS | B | 43 | 16.789 | -21.749 | 55.497 | 1.00 | 23.91 | C |
| ATOM | 2023 | O | LYS | B | 43 | 17.397 | -21.453 | 56.521 | 1.00 | 23.03 | O |
| ATOM | 2024 | CB | LYS | B | 43 | 18.536 | -23.246 | 54.564 | 1.00 | 25.41 | C |
| ATOM | 2025 | CG | LYS | B | 43 | 18.943 | -24.565 | 53.915 | 1.00 | 27.21 | C |
| ATOM | 2026 | CD | LYS | B | 43 | 19.081 | -25.645 | 54.948 | 1.00 | 27.34 | C |
| ATOM | 2027 | CE | LYS | B | 43 | 19.440 | -26.973 | 54.267 | 1.00 | 29.28 | C |
| ATOM | 2028 | NZ | LYS | B | 43 | 19.359 | -28.146 | 55.196 | 1.00 | 31.33 | N |
| ATOM | 2029 | N | GLY | B | 44 | 15.860 | -20.968 | 54.917 | 1.00 | 22.99 | N |
| ATOM | 2030 | CA | GLY | B | 44 | 15.487 | -19.640 | 55.429 | 1.00 | 21.33 | C |
| ATOM | 2031 | C | GLY | B | 44 | 16.680 | -18.689 | 55.477 | 1.00 | 20.46 | C |
| ATOM | 2032 | O | GLY | B | 44 | 17.579 | -18.739 | 54.598 | 1.00 | 18.03 | O |
| ATOM | 2033 | N | LEU | B | 45 | 16.747 | -17.833 | 56.508 | 1.00 | 16.47 | N |
| ATOM | 2034 | CA | LEU | B | 45 | 17.927 | -16.961 | 56.686 | 1.00 | 17.20 | C |
| ATOM | 2035 | C | LEU | B | 45 | 19.091 | -17.742 | 57.252 | 1.00 | 17.14 | C |
| ATOM | 2036 | O | LEU | B | 45 | 19.009 | -18.313 | 58.342 | 1.00 | 19.26 | O |
| ATOM | 2037 | CB | LEU | B | 45 | 17.645 | -15.747 | 57.616 | 1.00 | 17.05 | C |
| ATOM | 2038 | CG | LEU | B | 45 | 16.629 | -14.713 | 57.132 | 1.00 | 17.70 | C |
| ATOM | 2039 | CD1 | LEU | B | 45 | 16.225 | -13.768 | 58.263 | 1.00 | 18.31 | C |
| ATOM | 2040 | CD2 | LEU | B | 45 | 17.098 | -13.939 | 55.901 | 1.00 | 19.02 | C |
| ATOM | 2041 | N | GLU | B | 46 | 20.196 | -17.694 | 56.527 | 1.00 | 15.07 | N |
| ATOM | 2042 | CA | GLU | B | 46 | 21.384 | -18.426 | 56.791 | 1.00 | 16.97 | C |
| ATOM | 2043 | C | GLU | B | 46 | 22.639 | -17.568 | 56.698 | 1.00 | 16.24 | C |
| ATOM | 2044 | O | GLU | B | 46 | 23.132 | -17.219 | 55.630 | 1.00 | 15.18 | O |
| ATOM | 2045 | CB | GLU | B | 46 | 21.406 | -19.600 | 55.807 | 1.00 | 16.34 | C |
| ATOM | 2046 | CG | GLU | B | 46 | 22.573 | -20.493 | 55.944 | 1.00 | 20.45 | C |
| ATOM | 2047 | CD | GLU | B | 46 | 22.548 | -21.627 | 54.897 | 1.00 | 21.94 | C |
| ATOM | 2048 | OE1 | GLU | B | 46 | 21.792 | -21.551 | 53.891 | 1.00 | 21.80 | O |
| ATOM | 2049 | OE2 | GLU | B | 46 | 23.290 | -22.613 | 55.118 | 1.00 | 27.36 | O |
| ATOM | 2050 | N | TRP | B | 47 | 23.184 | -17.215 | 57.864 | 1.00 | 14.38 | N |
| ATOM | 2051 | CA | TRP | B | 47 | 24.490 | -16.614 | 57.968 | 1.00 | 15.70 | C |
| ATOM | 2052 | C | TRP | B | 47 | 25.532 | -17.530 | 57.400 | 1.00 | 15.96 | C |
| ATOM | 2053 | O | TRP | B | 47 | 25.533 | -18.737 | 57.699 | 1.00 | 17.99 | O |
| ATOM | 2054 | CB | TRP | B | 47 | 24.788 | -16.307 | 59.428 | 1.00 | 15.37 | C |
| ATOM | 2055 | CG | TRP | B | 47 | 26.159 | -15.799 | 59.778 | 1.00 | 15.88 | C |
| ATOM | 2056 | CD1 | TRP | B | 47 | 26.569 | -14.469 | 59.877 | 1.00 | 13.78 | C |
| ATOM | 2057 | CD2 | TRP | B | 47 | 27.261 | -16.570 | 60.217 | 1.00 | 13.78 | C |
| ATOM | 2058 | NE1 | TRP | B | 47 | 27.848 | -14.410 | 60.279 | 1.00 | 13.01 | N |
| ATOM | 2059 | CE2 | TRP | B | 47 | 28.301 | -15.684 | 60.533 | 1.00 | 14.69 | C |
| ATOM | 2060 | CE3 | TRP | B | 47 | 27.488 | -17.950 | 60.360 | 1.00 | 14.63 | C |
| ATOM | 2061 | CZ2 | TRP | B | 47 | 29.539 | -16.109 | 60.957 | 1.00 | 15.37 | C |
| ATOM | 2062 | CZ3 | TRP | B | 47 | 28.704 | -18.362 | 60.794 | 1.00 | 14.70 | C |
| ATOM | 2063 | CH2 | TRP | B | 47 | 29.739 | -17.460 | 61.076 | 1.00 | 17.91 | C |
| ATOM | 2064 | N | VAL | B | 48 | 26.388 | -16.997 | 56.545 | 1.00 | 14.84 | N |
| ATOM | 2065 | CA | VAL | B | 48 | 27.419 | -17.826 | 55.922 | 1.00 | 14.71 | C |
| ATOM | 2066 | C | VAL | B | 48 | 28.857 | -17.444 | 56.172 | 1.00 | 15.26 | C |
| ATOM | 2067 | O | VAL | B | 48 | 29.751 | -18.284 | 56.184 | 1.00 | 16.16 | O |
| ATOM | 2068 | CB | VAL | B | 48 | 27.149 | -18.035 | 54.409 | 1.00 | 15.59 | C |
| ATOM | 2069 | CG1 | VAL | B | 48 | 25.894 | -18.828 | 54.209 | 1.00 | 18.81 | C |
| ATOM | 2070 | CG2 | VAL | B | 48 | 27.104 | -16.665 | 53.676 | 1.00 | 16.62 | C |

```
ATOM   2071  N    ALA  B   49    29.126 -16.143  56.366  1.00 14.45           N
ATOM   2072  CA   ALA  B   49    30.516 -15.724  56.505  1.00 14.45           C
ATOM   2073  C    ALA  B   49    30.606 -14.354  57.132  1.00 15.32           C
ATOM   2074  O    ALA  B   49    29.634 -13.598  57.062  1.00 14.66           O
ATOM   2075  CB   ALA  B   49    31.225 -15.709  55.193  1.00 16.36           C
ATOM   2076  N    GLU  B   50    31.727 -14.126  57.811  1.00 16.39           N
ATOM   2077  CA   GLU  B   50    31.920 -12.889  58.563  1.00 15.05           C
ATOM   2078  C    GLU  B   50    33.390 -12.508  58.652  1.00 16.05           C
ATOM   2079  O    GLU  B   50    34.265 -13.369  58.649  1.00 13.54           O
ATOM   2080  CB   GLU  B   50    31.328 -13.061  59.944  1.00 13.83           C
ATOM   2081  CG   GLU  B   50    31.295 -11.772  60.824  1.00 15.73           C
ATOM   2082  CD   GLU  B   50    30.168 -11.782  61.854  1.00 13.40           C
ATOM   2083  OE1  GLU  B   50    29.045 -12.146  61.526  1.00 16.38           O
ATOM   2084  OE2  GLU  B   50    30.414 -11.393  63.029  1.00 17.73           O
ATOM   2085  N    ILE  B   51    33.652 -11.196  58.724  1.00 15.57           N
ATOM   2086  CA   ILE  B   51    35.005 -10.675  58.744  1.00 16.10           C
ATOM   2087  C    ILE  B   51    35.064  -9.495  59.732  1.00 16.57           C
ATOM   2088  O    ILE  B   51    34.131  -8.669  59.783  1.00 15.23           O
ATOM   2089  CB   ILE  B   51    35.539 -10.293  57.345  1.00 15.75           C
ATOM   2090  CG1  ILE  B   51    37.025 -10.015  57.392  1.00 14.95           C
ATOM   2091  CG2  ILE  B   51    34.730  -9.166  56.690  1.00 15.99           C
ATOM   2092  CD1  ILE  B   51    37.650 -10.003  56.068  1.00 16.98           C
ATOM   2093  N    ARG  B   52    36.129  -9.496  60.526  1.00 16.97           N
ATOM   2094  CA   ARG  B   52    36.401  -8.464  61.524  1.00 18.59           C
ATOM   2095  C    ARG  B   52    37.352  -7.439  60.943  1.00 18.18           C
ATOM   2096  O    ARG  B   52    37.396  -7.259  59.763  1.00 17.40           O
ATOM   2097  CB   ARG  B   52    36.899  -9.078  62.820  1.00 18.46           C
ATOM   2098  CG   ARG  B   52    35.861 -10.000  63.433  1.00 17.66           C
ATOM   2099  CD   ARG  B   52    36.136 -10.399  64.835  1.00 17.65           C
ATOM   2100  NE   ARG  B   52    35.093 -11.281  65.301  1.00 19.71           N
ATOM   2101  CZ   ARG  B   52    35.131 -11.948  66.461  1.00 19.77           C
ATOM   2102  NH1  ARG  B   52    36.127 -11.743  67.300  1.00 21.71           N
ATOM   2103  NH2  ARG  B   52    34.120 -12.719  66.823  1.00 21.20           N
ATOM   2104  N    LEU  B   52A   38.085  -6.721  61.790  1.00 20.08           N
ATOM   2105  CA   LEU  B   52A   38.740  -5.512  61.366  1.00 20.41           C
ATOM   2106  C    LEU  B   52A   40.231  -5.744  61.118  1.00 21.41           C
ATOM   2107  O    LEU  B   52A   40.807  -6.776  61.467  1.00 23.35           O
ATOM   2108  CB   LEU  B   52A   38.549  -4.402  62.422  1.00 21.24           C
ATOM   2109  CG   LEU  B   52A   37.177  -4.249  63.056  1.00 20.61           C
ATOM   2110  CD1  LEU  B   52A   37.190  -3.099  64.081  1.00 22.12           C
ATOM   2111  CD2  LEU  B   52A   36.122  -3.966  62.016  1.00 19.49           C
ATOM   2112  N    LYS  B   52B   40.850  -4.757  60.500  1.00 23.49           N
ATOM   2113  CA   LYS  B   52B   42.281  -4.740  60.303  1.00 25.27           C
ATOM   2114  C    LYS  B   52B   43.026  -5.027  61.609  1.00 25.16           C
ATOM   2115  O    LYS  B   52B   43.963  -5.834  61.628  1.00 25.52           O
ATOM   2116  CB   LYS  B   52B   42.677  -3.404  59.700  1.00 27.75           C
ATOM   2117  CG   LYS  B   52B   44.146  -3.277  59.344  1.00 29.32           C
ATOM   2118  CD   LYS  B   52B   44.461  -1.862  58.871  1.00 31.53           C
ATOM   2119  CE   LYS  B   52B   45.936  -1.578  59.012  1.00 35.25           C
ATOM   2120  NZ   LYS  B   52B   46.147  -0.108  58.841  1.00 35.13           N
ATOM   2121  N    SER  B   52C   42.566  -4.419  62.705  1.00 25.59           N
ATOM   2122  CA   SER  B   52C   43.151  -4.617  64.024  1.00 26.60           C
ATOM   2123  C    SER  B   52C   42.975  -6.036  64.568  1.00 26.40           C
ATOM   2124  O    SER  B   52C   43.650  -6.428  65.509  1.00 26.86           O
ATOM   2125  CB   SER  B   52C   42.591  -3.586  65.003  1.00 27.93           C
ATOM   2126  OG   SER  B   52C   41.190  -3.645  65.062  1.00 29.23           O
ATOM   2127  N    ASP  B   53    42.111  -6.826  63.926  1.00 25.30           N
ATOM   2128  CA   ASP  B   53    41.926  -8.253  64.252  1.00 25.91           C
ATOM   2129  C    ASP  B   53    42.606  -9.158  63.216  1.00 25.19           C
ATOM   2130  O    ASP  B   53    42.347 -10.372  63.117  1.00 24.62           O
ATOM   2131  CB   ASP  B   53    40.428  -8.576  64.363  1.00 25.86           C
ATOM   2132  CG   ASP  B   53    39.668  -7.570  65.193  1.00 27.51           C
ATOM   2133  OD1  ASP  B   53    39.972  -7.426  66.405  1.00 30.17           O
ATOM   2134  OD2  ASP  B   53    38.742  -6.933  64.648  1.00 23.70           O
ATOM   2135  N    ASN  B   54    43.520  -8.565  62.455  1.00 25.35           N
```

```
ATOM   2136  CA   ASN B  54      44.139  -9.198  61.315  1.00 27.20           C
ATOM   2137  C    ASN B  54      43.086  -9.766  60.374  1.00 24.39           C
ATOM   2138  O    ASN B  54      43.254 -10.848  59.833  1.00 22.81           O
ATOM   2139  CB   ASN B  54      45.128 -10.295  61.732  1.00 28.66           C
ATOM   2140  CG   ASN B  54      46.113 -10.663  60.613  1.00 33.02           C
ATOM   2141  OD1  ASN B  54      46.353  -9.878  59.687  1.00 35.54           O
ATOM   2142  ND2  ASN B  54      46.684 -11.874  60.692  1.00 34.99           N
ATOM   2143  N    TYR B  55      41.983  -9.035  60.218  1.00 23.03           N
ATOM   2144  CA   TYR B  55      40.947  -9.417  59.261  1.00 23.38           C
ATOM   2145  C    TYR B  55      40.465 -10.854  59.483  1.00 22.19           C
ATOM   2146  O    TYR B  55      40.249 -11.593  58.526  1.00 20.44           O
ATOM   2147  CB   TYR B  55      41.441  -9.251  57.826  1.00 24.95           C
ATOM   2148  CG   TYR B  55      41.738  -7.804  57.459  1.00 27.79           C
ATOM   2149  CD1  TYR B  55      40.718  -6.871  57.433  1.00 29.72           C
ATOM   2150  CD2  TYR B  55      43.026  -7.387  57.126  1.00 29.95           C
ATOM   2151  CE1  TYR B  55      40.944  -5.563  57.084  1.00 31.93           C
ATOM   2152  CE2  TYR B  55      43.274  -6.046  56.771  1.00 32.67           C
ATOM   2153  CZ   TYR B  55      42.218  -5.150  56.756  1.00 32.45           C
ATOM   2154  OH   TYR B  55      42.393  -3.824  56.404  1.00 37.05           O
ATOM   2155  N    ALA B  56      40.228 -11.190  60.743  1.00 22.51           N
ATOM   2156  CA   ALA B  56      39.781 -12.524  61.120  1.00 21.84           C
ATOM   2157  C    ALA B  56      38.442 -12.864  60.443  1.00 20.78           C
ATOM   2158  O    ALA B  56      37.546 -12.042  60.380  1.00 20.45           O
ATOM   2159  CB   ALA B  56      39.691 -12.632  62.604  1.00 23.29           C
ATOM   2160  N    THR B  57      38.380 -14.073  59.870  1.00 21.21           N
ATOM   2161  CA   THR B  57      37.220 -14.546  59.159  1.00 18.56           C
ATOM   2162  C    THR B  57      36.616 -15.749  59.831  1.00 18.67           C
ATOM   2163  O    THR B  57      37.281 -16.465  60.625  1.00 19.50           O
ATOM   2164  CB   THR B  57      37.552 -14.920  57.741  1.00 19.36           C
ATOM   2165  OG1  THR B  57      38.549 -15.974  57.775  1.00 20.65           O
ATOM   2166  CG2  THR B  57      38.081 -13.730  56.999  1.00 21.19           C
ATOM   2167  N    TYR B  58      35.331 -15.901  59.544  1.00 19.05           N
ATOM   2168  CA   TYR B  58      34.430 -16.883  60.171  1.00 17.33           C
ATOM   2169  C    TYR B  58      33.460 -17.374  59.104  1.00 18.07           C
ATOM   2170  O    TYR B  58      33.005 -16.605  58.280  1.00 16.88           O
ATOM   2171  CB   TYR B  58      33.671 -16.296  61.372  1.00 18.08           C
ATOM   2172  CG   TYR B  58      34.592 -15.643  62.374  1.00 18.21           C
ATOM   2173  CD1  TYR B  58      35.103 -14.356  62.170  1.00 18.53           C
ATOM   2174  CD2  TYR B  58      35.035 -16.327  63.501  1.00 20.32           C
ATOM   2175  CE1  TYR B  58      36.013 -13.782  63.068  1.00 19.99           C
ATOM   2176  CE2  TYR B  58      35.921 -15.738  64.404  1.00 19.07           C
ATOM   2177  CZ   TYR B  58      36.406 -14.466  64.179  1.00 19.62           C
ATOM   2178  OH   TYR B  58      37.283 -13.955  65.126  1.00 22.64           O
ATOM   2179  N    TYR B  59      33.136 -18.668  59.143  1.00 16.68           N
ATOM   2180  CA   TYR B  59      32.316 -19.324  58.125  1.00 17.25           C
ATOM   2181  C    TYR B  59      31.358 -20.296  58.786  1.00 16.94           C
ATOM   2182  O    TYR B  59      31.705 -20.897  59.822  1.00 17.60           O
ATOM   2183  CB   TYR B  59      33.214 -20.082  57.103  1.00 17.52           C
ATOM   2184  CG   TYR B  59      34.146 -19.184  56.344  1.00 17.82           C
ATOM   2185  CD1  TYR B  59      33.685 -18.466  55.267  1.00 16.96           C
ATOM   2186  CD2  TYR B  59      35.459 -18.980  56.735  1.00 14.37           C
ATOM   2187  CE1  TYR B  59      34.527 -17.596  54.581  1.00 15.95           C
ATOM   2188  CE2  TYR B  59      36.302 -18.149  56.036  1.00 14.83           C
ATOM   2189  CZ   TYR B  59      35.807 -17.420  54.976  1.00 17.12           C
ATOM   2190  OH   TYR B  59      36.599 -16.572  54.243  1.00 17.55           O
ATOM   2191  N    ALA B  60      30.160 -20.401  58.202  1.00 15.16           N
ATOM   2192  CA   ALA B  60      29.184 -21.440  58.524  1.00 14.84           C
ATOM   2193  C    ALA B  60      29.751 -22.714  57.970  1.00 15.26           C
ATOM   2194  O    ALA B  60      30.399 -22.700  56.953  1.00 16.70           O
ATOM   2195  CB   ALA B  60      27.886 -21.183  57.887  1.00 13.80           C
ATOM   2196  N    GLU B  61      29.503 -23.823  58.666  1.00 15.90           N
ATOM   2197  CA   GLU B  61      30.036 -25.115  58.213  1.00 19.00           C
ATOM   2198  C    GLU B  61      29.649 -25.414  56.788  1.00 18.19           C
ATOM   2199  O    GLU B  61      30.478 -25.912  56.040  1.00 19.35           O
ATOM   2200  CB   GLU B  61      29.539 -26.248  59.086  1.00 18.89           C
```

| ATOM | 2201 | CG  | GLU | B | 61 | 30.212 | -27.642 | 58.785 | 1.00 | 22.03 | C |
| ATOM | 2202 | CD  | GLU | B | 61 | 31.600 | -27.721 | 59.285 | 1.00 | 23.97 | C |
| ATOM | 2203 | OE1 | GLU | B | 61 | 31.799 | -27.380 | 60.470 | 1.00 | 26.49 | O |
| ATOM | 2204 | OE2 | GLU | B | 61 | 32.503 | -28.116 | 58.514 | 1.00 | 28.03 | O |
| ATOM | 2205 | N   | SER | B | 62 | 28.410 | -25.073 | 56.446 | 1.00 | 17.94 | N |
| ATOM | 2206 | CA  | SER | B | 62 | 27.841 | -25.299 | 55.102 | 1.00 | 19.44 | C |
| ATOM | 2207 | C   | SER | B | 62 | 28.820 | -24.865 | 54.022 | 1.00 | 20.27 | C |
| ATOM | 2208 | O   | SER | B | 62 | 29.027 | -25.562 | 53.021 | 1.00 | 19.21 | O |
| ATOM | 2209 | CB  | SER | B | 62 | 26.453 | -24.662 | 54.943 | 1.00 | 20.34 | C |
| ATOM | 2210 | OG  | SER | B | 62 | 26.421 | -23.241 | 55.161 | 1.00 | 25.50 | O |
| ATOM | 2211 | N   | VAL | B | 63 | 29.471 | -23.723 | 54.214 | 1.00 | 19.62 | N |
| ATOM | 2212 | CA  | VAL | B | 63 | 30.256 | -23.127 | 53.143 | 1.00 | 18.38 | C |
| ATOM | 2213 | C   | VAL | B | 63 | 31.777 | -23.149 | 53.346 | 1.00 | 18.92 | C |
| ATOM | 2214 | O   | VAL | B | 63 | 32.554 | -22.685 | 52.518 | 1.00 | 17.86 | O |
| ATOM | 2215 | CB  | VAL | B | 63 | 29.746 | -21.695 | 52.853 | 1.00 | 17.99 | C |
| ATOM | 2216 | CG1 | VAL | B | 63 | 28.243 | -21.667 | 52.755 | 1.00 | 16.89 | C |
| ATOM | 2217 | CG2 | VAL | B | 63 | 30.199 | -20.759 | 53.920 | 1.00 | 17.65 | C |
| ATOM | 2218 | N   | LYS | B | 64 | 32.225 | -23.712 | 54.460 | 1.00 | 21.48 | N |
| ATOM | 2219 | CA  | LYS | B | 64 | 33.653 | -23.789 | 54.748 | 1.00 | 22.03 | C |
| ATOM | 2220 | C   | LYS | B | 64 | 34.322 | -24.530 | 53.620 | 1.00 | 22.18 | C |
| ATOM | 2221 | O   | LYS | B | 64 | 33.792 | -25.535 | 53.134 | 1.00 | 22.80 | O |
| ATOM | 2222 | CB  | LYS | B | 64 | 33.893 | -24.533 | 56.056 | 1.00 | 23.70 | C |
| ATOM | 2223 | CG  | LYS | B | 64 | 33.399 | -23.839 | 57.273 | 1.00 | 26.82 | C |
| ATOM | 2224 | CD  | LYS | B | 64 | 33.952 | -24.526 | 58.520 | 1.00 | 30.36 | C |
| ATOM | 2225 | CE  | LYS | B | 64 | 33.321 | -23.984 | 59.804 | 1.00 | 30.76 | C |
| ATOM | 2226 | NZ  | LYS | B | 64 | 33.764 | -24.911 | 60.937 | 1.00 | 33.29 | N |
| ATOM | 2227 | N   | GLY | B | 65 | 35.449 | -24.021 | 53.162 | 1.00 | 21.84 | N |
| ATOM | 2228 | CA  | GLY | B | 65 | 36.141 | -24.577 | 51.987 | 1.00 | 23.37 | C |
| ATOM | 2229 | C   | GLY | B | 65 | 35.647 | -24.152 | 50.614 | 1.00 | 24.30 | C |
| ATOM | 2230 | O   | GLY | B | 65 | 36.331 | -24.413 | 49.619 | 1.00 | 27.29 | O |
| ATOM | 2231 | N   | LYS | B | 66 | 34.456 | -23.556 | 50.543 | 1.00 | 20.55 | N |
| ATOM | 2232 | CA  | LYS | B | 66 | 33.825 | -23.153 | 49.305 | 1.00 | 20.17 | C |
| ATOM | 2233 | C   | LYS | B | 66 | 33.802 | -21.622 | 49.150 | 1.00 | 18.08 | C |
| ATOM | 2234 | O   | LYS | B | 66 | 33.979 | -21.093 | 48.032 | 1.00 | 18.72 | O |
| ATOM | 2235 | CB  | LYS | B | 66 | 32.369 | -23.603 | 49.265 | 1.00 | 20.86 | C |
| ATOM | 2236 | CG  | LYS | B | 66 | 32.136 | -25.065 | 48.978 | 1.00 | 24.77 | C |
| ATOM | 2237 | CD  | LYS | B | 66 | 30.696 | -25.452 | 49.285 | 1.00 | 26.21 | C |
| ATOM | 2238 | CE  | LYS | B | 66 | 29.659 | -24.885 | 48.310 | 1.00 | 24.78 | C |
| ATOM | 2239 | NZ  | LYS | B | 66 | 28.312 | -25.624 | 48.359 | 1.00 | 20.85 | N |
| ATOM | 2240 | N   | PHE | B | 67 | 33.597 | -20.937 | 50.272 | 1.00 | 16.52 | N |
| ATOM | 2241 | CA  | PHE | B | 67 | 33.428 | -19.453 | 50.257 | 1.00 | 15.56 | C |
| ATOM | 2242 | C   | PHE | B | 67 | 34.613 | -18.763 | 50.923 | 1.00 | 16.23 | C |
| ATOM | 2243 | O   | PHE | B | 67 | 35.168 | -19.250 | 51.931 | 1.00 | 15.99 | O |
| ATOM | 2244 | CB  | PHE | B | 67 | 32.162 | -19.051 | 51.019 | 1.00 | 16.26 | C |
| ATOM | 2245 | CG  | PHE | B | 67 | 30.851 | -19.346 | 50.302 | 1.00 | 18.31 | C |
| ATOM | 2246 | CD1 | PHE | B | 67 | 30.753 | -20.174 | 49.207 | 1.00 | 16.02 | C |
| ATOM | 2247 | CD2 | PHE | B | 67 | 29.681 | -18.785 | 50.777 | 1.00 | 16.26 | C |
| ATOM | 2248 | CE1 | PHE | B | 67 | 29.532 | -20.434 | 48.607 | 1.00 | 18.16 | C |
| ATOM | 2249 | CE2 | PHE | B | 67 | 28.473 | -19.039 | 50.180 | 1.00 | 16.96 | C |
| ATOM | 2250 | CZ  | PHE | B | 67 | 28.378 | -19.869 | 49.116 | 1.00 | 18.50 | C |
| ATOM | 2251 | N   | THR | B | 68 | 34.973 | -17.595 | 50.402 | 1.00 | 16.31 | N |
| ATOM | 2252 | CA  | THR | B | 68 | 35.949 | -16.730 | 51.059 | 1.00 | 16.79 | C |
| ATOM | 2253 | C   | THR | B | 68 | 35.448 | -15.300 | 51.132 | 1.00 | 15.54 | C |
| ATOM | 2254 | O   | THR | B | 68 | 35.158 | -14.702 | 50.099 | 1.00 | 17.18 | O |
| ATOM | 2255 | CB  | THR | B | 68 | 37.257 | -16.694 | 50.296 | 1.00 | 18.07 | C |
| ATOM | 2256 | OG1 | THR | B | 68 | 37.696 | -18.038 | 50.059 | 1.00 | 16.56 | O |
| ATOM | 2257 | CG2 | THR | B | 68 | 38.348 | -15.994 | 51.075 | 1.00 | 19.67 | C |
| ATOM | 2258 | N   | ILE | B | 69 | 35.372 | -14.780 | 52.349 | 1.00 | 15.43 | N |
| ATOM | 2259 | CA  | ILE | B | 69 | 34.927 | -13.385 | 52.559 | 1.00 | 15.14 | C |
| ATOM | 2260 | C   | ILE | B | 69 | 36.193 | -12.517 | 52.627 | 1.00 | 16.30 | C |
| ATOM | 2261 | O   | ILE | B | 69 | 37.203 | -12.917 | 53.216 | 1.00 | 16.76 | O |
| ATOM | 2262 | CB  | ILE | B | 69 | 34.062 | -13.263 | 53.843 | 1.00 | 14.68 | C |
| ATOM | 2263 | CG1 | ILE | B | 69 | 33.360 | -11.884 | 53.956 | 1.00 | 14.86 | C |
| ATOM | 2264 | CG2 | ILE | B | 69 | 34.902 | -13.568 | 55.078 | 1.00 | 16.28 | C |
| ATOM | 2265 | CD1 | ILE | B | 69 | 32.367 | -11.781 | 55.063 | 1.00 | 14.45 | C |

| ATOM | 2266 | N | SER | B | 70 | 36.095 | -11.288 | 52.107 | 1.00 | 16.44 | N |
|------|------|-----|-----|---|----|--------|---------|--------|------|-------|---|
| ATOM | 2267 | CA | SER | B | 70 | 37.219 | -10.387 | 52.133 | 1.00 | 16.22 | C |
| ATOM | 2268 | C | SER | B | 70 | 36.643 | -8.988 | 52.115 | 1.00 | 15.24 | C |
| ATOM | 2269 | O | SER | B | 70 | 35.454 | -8.792 | 51.819 | 1.00 | 14.81 | O |
| ATOM | 2270 | CB | SER | B | 70 | 38.161 | -10.594 | 50.933 | 1.00 | 15.29 | C |
| ATOM | 2271 | OG | SER | B | 70 | 37.588 | -10.329 | 49.678 | 1.00 | 18.26 | O |
| ATOM | 2272 | N | ARG | B | 71 | 37.494 | -8.033 | 52.439 | 1.00 | 16.98 | N |
| ATOM | 2273 | CA | ARG | B | 71 | 37.063 | -6.627 | 52.497 | 1.00 | 16.57 | C |
| ATOM | 2274 | C | ARG | B | 71 | 38.158 | -5.713 | 51.911 | 1.00 | 16.38 | C |
| ATOM | 2275 | O | ARG | B | 71 | 39.348 | -5.986 | 52.053 | 1.00 | 18.62 | O |
| ATOM | 2276 | CB | ARG | B | 71 | 36.689 | -6.224 | 53.936 | 1.00 | 17.61 | C |
| ATOM | 2277 | CG | ARG | B | 71 | 37.835 | -6.168 | 54.939 | 1.00 | 15.51 | C |
| ATOM | 2278 | CD | ARG | B | 71 | 37.362 | -6.066 | 56.359 | 1.00 | 17.22 | C |
| ATOM | 2279 | NE | ARG | B | 71 | 36.602 | -4.818 | 56.587 | 1.00 | 17.02 | N |
| ATOM | 2280 | CZ | ARG | B | 71 | 35.671 | -4.667 | 57.502 | 1.00 | 17.52 | C |
| ATOM | 2281 | NH1 | ARG | B | 71 | 35.364 | -5.679 | 58.321 | 1.00 | 15.77 | N |
| ATOM | 2282 | NH2 | ARG | B | 71 | 35.014 | -3.520 | 57.586 | 1.00 | 20.18 | N |
| ATOM | 2283 | N | ASP | B | 72 | 37.736 | -4.647 | 51.252 | 1.00 | 18.10 | N |
| ATOM | 2284 | CA | ASP | B | 72 | 38.646 | -3.602 | 50.818 | 1.00 | 18.85 | C |
| ATOM | 2285 | C | ASP | B | 72 | 38.185 | -2.298 | 51.456 | 1.00 | 17.86 | C |
| ATOM | 2286 | O | ASP | B | 72 | 37.228 | -1.682 | 50.986 | 1.00 | 20.80 | O |
| ATOM | 2287 | CB | ASP | B | 72 | 38.665 | -3.501 | 49.304 | 1.00 | 21.80 | C |
| ATOM | 2288 | CG | ASP | B | 72 | 39.661 | -2.489 | 48.808 | 1.00 | 24.50 | C |
| ATOM | 2289 | OD1 | ASP | B | 72 | 39.978 | -1.538 | 49.563 | 1.00 | 27.64 | O |
| ATOM | 2290 | OD2 | ASP | B | 72 | 40.099 | -2.617 | 47.644 | 1.00 | 26.07 | O |
| ATOM | 2291 | N | ASP | B | 73 | 38.815 | -1.966 | 52.568 | 1.00 | 19.85 | N |
| ATOM | 2292 | CA | ASP | B | 73 | 38.403 | -0.835 | 53.405 | 1.00 | 19.80 | C |
| ATOM | 2293 | C | ASP | B | 73 | 38.454 | 0.462 | 52.634 | 1.00 | 21.65 | C |
| ATOM | 2294 | O | ASP | B | 73 | 37.647 | 1.355 | 52.872 | 1.00 | 22.84 | O |
| ATOM | 2295 | CB | ASP | B | 73 | 39.248 | -0.778 | 54.663 | 1.00 | 20.34 | C |
| ATOM | 2296 | CG | ASP | B | 73 | 38.846 | -1.831 | 55.676 | 1.00 | 19.34 | C |
| ATOM | 2297 | OD1 | ASP | B | 73 | 37.708 | -2.313 | 55.573 | 1.00 | 19.56 | O |
| ATOM | 2298 | OD2 | ASP | B | 73 | 39.614 | -2.124 | 56.603 | 1.00 | 21.41 | O |
| ATOM | 2299 | N | SER | B | 74 | 39.393 | 0.535 | 51.703 | 1.00 | 23.31 | N |
| ATOM | 2300 | CA | SER | B | 74 | 39.586 | 1.766 | 50.917 | 1.00 | 24.26 | C |
| ATOM | 2301 | C | SER | B | 74 | 38.433 | 2.017 | 49.966 | 1.00 | 24.93 | C |
| ATOM | 2302 | O | SER | B | 74 | 38.197 | 3.177 | 49.555 | 1.00 | 28.22 | O |
| ATOM | 2303 | CB | SER | B | 74 | 40.885 | 1.691 | 50.125 | 1.00 | 24.98 | C |
| ATOM | 2304 | OG | SER | B | 74 | 40.847 | 0.734 | 49.071 | 1.00 | 25.87 | O |
| ATOM | 2305 | N | LYS | B | 75 | 37.711 | 0.948 | 49.584 | 1.00 | 22.26 | N |
| ATOM | 2306 | CA | LYS | B | 75 | 36.532 | 1.062 | 48.736 | 1.00 | 21.33 | C |
| ATOM | 2307 | C | LYS | B | 75 | 35.196 | 0.905 | 49.453 | 1.00 | 20.34 | C |
| ATOM | 2308 | O | LYS | B | 75 | 34.140 | 0.951 | 48.815 | 1.00 | 19.06 | O |
| ATOM | 2309 | CB | LYS | B | 75 | 36.627 | 0.024 | 47.612 | 1.00 | 22.15 | C |
| ATOM | 2310 | CG | LYS | B | 75 | 37.860 | 0.202 | 46.783 | 1.00 | 24.24 | C |
| ATOM | 2311 | CD | LYS | B | 75 | 37.924 | -0.841 | 45.683 | 1.00 | 24.03 | C |
| ATOM | 2312 | CE | LYS | B | 75 | 39.227 | -0.795 | 44.889 | 1.00 | 27.05 | C |
| ATOM | 2313 | NZ | LYS | B | 75 | 39.102 | -1.743 | 43.726 | 1.00 | 29.31 | N |
| ATOM | 2314 | N | SER | B | 76 | 35.251 | 0.732 | 50.770 | 1.00 | 19.73 | N |
| ATOM | 2315 | CA | SER | B | 76 | 34.083 | 0.424 | 51.576 | 1.00 | 19.45 | C |
| ATOM | 2316 | C | SER | B | 76 | 33.353 | -0.770 | 50.993 | 1.00 | 18.70 | C |
| ATOM | 2317 | O | SER | B | 76 | 32.131 | -0.766 | 50.908 | 1.00 | 18.59 | O |
| ATOM | 2318 | CB | SER | B | 76 | 33.135 | 1.610 | 51.661 | 1.00 | 21.73 | C |
| ATOM | 2319 | OG | SER | B | 76 | 33.779 | 2.667 | 52.324 | 1.00 | 20.71 | O |
| ATOM | 2320 | N | ARG | B | 77 | 34.108 | -1.772 | 50.555 | 1.00 | 18.05 | N |
| ATOM | 2321 | CA | ARG | B | 77 | 33.508 | -2.873 | 49.818 | 1.00 | 19.20 | C |
| ATOM | 2322 | C | ARG | B | 77 | 33.826 | -4.201 | 50.508 | 1.00 | 18.30 | C |
| ATOM | 2323 | O | ARG | B | 77 | 34.940 | -4.400 | 50.960 | 1.00 | 18.89 | O |
| ATOM | 2324 | CB | ARG | B | 77 | 34.016 | -2.896 | 48.398 | 1.00 | 21.26 | C |
| ATOM | 2325 | CG | ARG | B | 77 | 33.050 | -3.512 | 47.422 | 1.00 | 26.30 | C |
| ATOM | 2326 | CD | ARG | B | 77 | 33.426 | -3.259 | 45.982 | 1.00 | 26.71 | C |
| ATOM | 2327 | NE | ARG | B | 77 | 32.718 | -2.116 | 45.418 | 1.00 | 27.21 | N |
| ATOM | 2328 | CZ | ARG | B | 77 | 31.415 | -2.089 | 45.117 | 1.00 | 30.17 | C |
| ATOM | 2329 | NH1 | ARG | B | 77 | 30.600 | -3.140 | 45.351 | 1.00 | 29.32 | N |
| ATOM | 2330 | NH2 | ARG | B | 77 | 30.907 | -0.972 | 44.604 | 1.00 | 31.78 | N |

```
ATOM   2331  N    LEU B  78      32.795  -5.030  50.622  1.00 18.10           N
ATOM   2332  CA   LEU B  78      32.862  -6.424  51.072  1.00 15.53           C
ATOM   2333  C    LEU B  78      32.691  -7.351  49.883  1.00 15.30           C
ATOM   2334  O    LEU B  78      31.885  -7.103  48.982  1.00 16.98           O
ATOM   2335  CB   LEU B  78      31.717  -6.716  52.031  1.00 14.75           C
ATOM   2336  CG   LEU B  78      31.822  -7.975  52.926  1.00 14.59           C
ATOM   2337  CD1  LEU B  78      32.911  -7.936  53.928  1.00 18.54           C
ATOM   2338  CD2  LEU B  78      30.492  -8.284  53.634  1.00 16.43           C
ATOM   2339  N    TYR B  79      33.428  -8.453  49.903  1.00 15.75           N
ATOM   2340  CA   TYR B  79      33.329  -9.452  48.844  1.00 15.75           C
ATOM   2341  C    TYR B  79      33.030 -10.838  49.376  1.00 15.32           C
ATOM   2342  O    TYR B  79      33.367 -11.167  50.494  1.00 14.96           O
ATOM   2343  CB   TYR B  79      34.666  -9.556  48.138  1.00 16.43           C
ATOM   2344  CG   TYR B  79      35.151  -8.261  47.555  1.00 18.73           C
ATOM   2345  CD1  TYR B  79      34.732  -7.864  46.319  1.00 22.04           C
ATOM   2346  CD2  TYR B  79      36.006  -7.435  48.282  1.00 21.50           C
ATOM   2347  CE1  TYR B  79      35.184  -6.644  45.771  1.00 24.23           C
ATOM   2348  CE2  TYR B  79      36.443  -6.253  47.771  1.00 23.32           C
ATOM   2349  CZ   TYR B  79      36.031  -5.867  46.511  1.00 23.20           C
ATOM   2350  OH   TYR B  79      36.481  -4.633  46.020  1.00 27.43           O
ATOM   2351  N    LEU B  80      32.444 -11.648  48.506  1.00 15.58           N
ATOM   2352  CA   LEU B  80      32.290 -13.094  48.769  1.00 15.03           C
ATOM   2353  C    LEU B  80      32.641 -13.847  47.494  1.00 14.70           C
ATOM   2354  O    LEU B  80      31.967 -13.717  46.477  1.00 16.34           O
ATOM   2355  CB   LEU B  80      30.852 -13.414  49.172  1.00 15.78           C
ATOM   2356  CG   LEU B  80      30.627 -14.811  49.822  1.00 14.10           C
ATOM   2357  CD1  LEU B  80      31.342 -15.030  51.140  1.00 17.04           C
ATOM   2358  CD2  LEU B  80      29.182 -15.042  49.979  1.00 13.85           C
ATOM   2359  N    GLN B  81      33.737 -14.575  47.554  1.00 15.43           N
ATOM   2360  CA   GLN B  81      34.133 -15.477  46.442  1.00 16.53           C
ATOM   2361  C    GLN B  81      33.497 -16.821  46.772  1.00 15.56           C
ATOM   2362  O    GLN B  81      33.761 -17.353  47.842  1.00 17.38           O
ATOM   2363  CB   GLN B  81      35.636 -15.642  46.397  1.00 16.98           C
ATOM   2364  CG   GLN B  81      36.158 -16.569  45.310  1.00 18.05           C
ATOM   2365  CD   GLN B  81      35.795 -16.105  43.913  1.00 21.44           C
ATOM   2366  OE1  GLN B  81      36.103 -14.972  43.512  1.00 19.64           O
ATOM   2367  NE2  GLN B  81      35.143 -16.985  43.136  1.00 19.35           N
ATOM   2368  N    MET B  82      32.741 -17.389  45.826  1.00 15.05           N
ATOM   2369  CA   MET B  82      31.899 -18.533  46.062  1.00 17.00           C
ATOM   2370  C    MET B  82      32.273 -19.602  45.040  1.00 16.96           C
ATOM   2371  O    MET B  82      31.952 -19.469  43.873  1.00 16.30           O
ATOM   2372  CB   MET B  82      30.420 -18.147  45.869  1.00 17.26           C
ATOM   2373  CG   MET B  82      29.904 -16.969  46.711  1.00 18.76           C
ATOM   2374  SD   MET B  82      28.379 -16.220  46.118  1.00 22.37           S
ATOM   2375  CE   MET B  82      27.356 -17.639  46.254  1.00 18.12           C
ATOM   2376  N    ASN B  82A     32.967 -20.659  45.471  1.00 17.87           N
ATOM   2377  CA   ASN B  82A     33.466 -21.675  44.551  1.00 16.43           C
ATOM   2378  C    ASN B  82A     32.784 -23.011  44.709  1.00 16.69           C
ATOM   2379  O    ASN B  82A     32.146 -23.275  45.707  1.00 16.35           O
ATOM   2380  CB   ASN B  82A     34.961 -21.895  44.788  1.00 18.47           C
ATOM   2381  CG   ASN B  82A     35.781 -20.629  44.591  1.00 20.08           C
ATOM   2382  OD1  ASN B  82A     35.469 -19.802  43.739  1.00 21.85           O
ATOM   2383  ND2  ASN B  82A     36.842 -20.482  45.380  1.00 26.99           N
ATOM   2384  N    ASN B  82B     32.941 -23.828  43.680  1.00 17.07           N
ATOM   2385  CA   ASN B  82B     32.408 -25.203  43.669  1.00 18.08           C
ATOM   2386  C    ASN B  82B     30.914 -25.252  44.088  1.00 18.07           C
ATOM   2387  O    ASN B  82B     30.459 -26.051  44.959  1.00 17.38           O
ATOM   2388  CB   ASN B  82B     33.275 -26.094  44.533  1.00 19.23           C
ATOM   2389  CG   ASN B  82B     32.952 -27.576  44.336  1.00 19.93           C
ATOM   2390  OD1  ASN B  82B     33.068 -28.396  45.290  1.00 27.77           O
ATOM   2391  ND2  ASN B  82B     32.527 -27.914  43.164  1.00 19.91           N
ATOM   2392  N    LEU B  82C     30.134 -24.392  43.434  1.00 17.23           N
ATOM   2393  CA   LEU B  82C     28.751 -24.184  43.818  1.00 16.47           C
ATOM   2394  C    LEU B  82C     27.870 -25.358  43.483  1.00 16.90           C
ATOM   2395  O    LEU B  82C     28.083 -26.090  42.486  1.00 17.45           O
```

```
ATOM   2396  CB   LEU B  82C   28.211 -22.888  43.179  1.00 17.69      C
ATOM   2397  CG   LEU B  82C   29.023 -21.660  43.606  1.00 18.85      C
ATOM   2398  CD1  LEU B  82C   28.536 -20.394  42.901  1.00 20.13      C
ATOM   2399  CD2  LEU B  82C   28.971 -21.476  45.093  1.00 22.03      C
ATOM   2400  N    ARG B  83    26.881 -25.553  44.336  1.00 17.38      N
ATOM   2401  CA   ARG B  83    25.853 -26.558  44.127  1.00 17.61      C
ATOM   2402  C    ARG B  83    24.485 -25.883  43.995  1.00 17.69      C
ATOM   2403  O    ARG B  83    24.313 -24.696  44.354  1.00 17.41      O
ATOM   2404  CB   ARG B  83    25.856 -27.536  45.301  1.00 18.81      C
ATOM   2405  CG   ARG B  83    25.466 -26.961  46.619  1.00 21.61      C
ATOM   2406  CD   ARG B  83    25.300 -28.040  47.703  1.00 25.11      C
ATOM   2407  NE   ARG B  83    26.367 -28.995  47.601  1.00 30.12      N
ATOM   2408  CZ   ARG B  83    26.294 -30.223  47.105  1.00 28.78      C
ATOM   2409  NH1  ARG B  83    25.169 -30.775  46.673  1.00 31.76      N
ATOM   2410  NH2  ARG B  83    27.386 -30.912  47.083  1.00 30.86      N
ATOM   2411  N    THR B  84    23.476 -26.620  43.543  1.00 18.12      N
ATOM   2412  CA   THR B  84    22.181 -25.989  43.321  1.00 20.04      C
ATOM   2413  C    THR B  84    21.665 -25.354  44.609  1.00 18.88      C
ATOM   2414  O    THR B  84    20.972 -24.301  44.555  1.00 18.69      O
ATOM   2415  CB   THR B  84    21.116 -26.954  42.751  1.00 23.61      C
ATOM   2416  OG1  THR B  84    20.915 -28.006  43.654  1.00 26.51      O
ATOM   2417  CG2  THR B  84    21.545 -27.567  41.482  1.00 25.56      C
ATOM   2418  N    GLU B  85    21.973 -25.945  45.775  1.00 17.47      N
ATOM   2419  CA   GLU B  85    21.500 -25.388  47.041  1.00 18.19      C
ATOM   2420  C    GLU B  85    22.136 -24.080  47.430  1.00 16.43      C
ATOM   2421  O    GLU B  85    21.694 -23.473  48.415  1.00 19.56      O
ATOM   2422  CB   GLU B  85    21.696 -26.368  48.204  1.00 19.47      C
ATOM   2423  CG   GLU B  85    20.842 -27.593  48.074  1.00 20.58      C
ATOM   2424  CD   GLU B  85    21.538 -28.764  47.412  1.00 24.34      C
ATOM   2425  OE1  GLU B  85    22.449 -28.563  46.578  1.00 22.47      O
ATOM   2426  OE2  GLU B  85    21.124 -29.898  47.711  1.00 21.69      O
ATOM   2427  N    ASP B  86    23.178 -23.648  46.714  1.00 15.55      N
ATOM   2428  CA   ASP B  86    23.732 -22.326  46.947  1.00 15.66      C
ATOM   2429  C    ASP B  86    22.924 -21.216  46.260  1.00 16.46      C
ATOM   2430  O    ASP B  86    23.218 -20.017  46.425  1.00 16.74      O
ATOM   2431  CB   ASP B  86    25.209 -22.279  46.532  1.00 16.23      C
ATOM   2432  CG   ASP B  86    26.090 -23.223  47.361  1.00 20.29      C
ATOM   2433  OD1  ASP B  86    26.020 -23.152  48.615  1.00 17.93      O
ATOM   2434  OD2  ASP B  86    26.868 -24.020  46.771  1.00 16.85      O
ATOM   2435  N    THR B  87    21.920 -21.601  45.507  1.00 16.48      N
ATOM   2436  CA   THR B  87    20.937 -20.640  45.004  1.00 16.53      C
ATOM   2437  C    THR B  87    20.253 -19.879  46.150  1.00 17.09      C
ATOM   2438  O    THR B  87    19.791 -20.472  47.126  1.00 15.89      O
ATOM   2439  CB   THR B  87    19.906 -21.364  44.214  1.00 17.39      C
ATOM   2440  OG1  THR B  87    20.515 -21.995  43.093  1.00 15.11      O
ATOM   2441  CG2  THR B  87    18.881 -20.424  43.698  1.00 18.39      C
ATOM   2442  N    GLY B  88    20.132 -18.547  46.024  1.00 14.02      N
ATOM   2443  CA   GLY B  88    19.424 -17.800  47.046  1.00 14.58      C
ATOM   2444  C    GLY B  88    19.716 -16.331  46.873  1.00 14.58      C
ATOM   2445  O    GLY B  88    20.439 -15.954  45.969  1.00 14.32      O
ATOM   2446  N    ILE B  89    19.080 -15.538  47.744  1.00 14.70      N
ATOM   2447  CA   ILE B  89    19.362 -14.097  47.782  1.00 13.74      C
ATOM   2448  C    ILE B  89    20.451 -13.842  48.793  1.00 12.57      C
ATOM   2449  O    ILE B  89    20.323 -14.259  49.950  1.00 15.03      O
ATOM   2450  CB   ILE B  89    18.117 -13.286  48.178  1.00 15.43      C
ATOM   2451  CG1  ILE B  89    16.929 -13.707  47.323  1.00 16.13      C
ATOM   2452  CG2  ILE B  89    18.397 -11.780  48.092  1.00 15.84      C
ATOM   2453  CD1  ILE B  89    17.143 -13.591  45.894  1.00 15.01      C
ATOM   2454  N    TYR B  90    21.503 -13.133  48.392  1.00 14.52      N
ATOM   2455  CA   TYR B  90    22.647 -12.927  49.259  1.00 12.54      C
ATOM   2456  C    TYR B  90    22.631 -11.494  49.738  1.00 14.98      C
ATOM   2457  O    TYR B  90    22.604 -10.594  48.900  1.00 15.68      O
ATOM   2458  CB   TYR B  90    23.955 -13.257  48.519  1.00 14.58      C
ATOM   2459  CG   TYR B  90    24.194 -14.749  48.452  1.00 13.59      C
ATOM   2460  CD1  TYR B  90    23.414 -15.531  47.652  1.00 15.41      C
```

```
ATOM   2461  CD2 TYR B   90     25.193 -15.361  49.203  1.00 13.13           C
ATOM   2462  CE1 TYR B   90     23.575 -16.956  47.594  1.00 15.56           C
ATOM   2463  CE2 TYR B   90     25.375 -16.784  49.134  1.00 13.79           C
ATOM   2464  CZ  TYR B   90     24.553 -17.542  48.348  1.00 13.92           C
ATOM   2465  OH  TYR B   90     24.725 -18.959  48.249  1.00 15.01           O
ATOM   2466  N   TYR B   91     22.613 -11.326  51.067  1.00 15.69           N
ATOM   2467  CA  TYR B   91     22.624 -10.002  51.758  1.00 15.55           C
ATOM   2468  C   TYR B   91     23.932  -9.758  52.453  1.00 17.12           C
ATOM   2469  O   TYR B   91     24.528 -10.674  53.081  1.00 14.98           O
ATOM   2470  CB  TYR B   91     21.516  -9.972  52.803  1.00 17.52           C
ATOM   2471  CG  TYR B   91     20.130 -10.103  52.294  1.00 18.46           C
ATOM   2472  CD1 TYR B   91     19.439  -8.988  51.859  1.00 17.88           C
ATOM   2473  CD2 TYR B   91     19.453 -11.330  52.329  1.00 18.27           C
ATOM   2474  CE1 TYR B   91     18.150  -9.097  51.416  1.00 20.96           C
ATOM   2475  CE2 TYR B   91     18.169 -11.444  51.879  1.00 19.57           C
ATOM   2476  CZ  TYR B   91     17.502 -10.314  51.469  1.00 21.81           C
ATOM   2477  OH  TYR B   91     16.209 -10.423  51.037  1.00 21.86           O
ATOM   2478  N   CYS B   92     24.426  -8.518  52.337  1.00 14.69           N
ATOM   2479  CA  CYS B   92     25.382  -8.074  53.311  1.00 15.67           C
ATOM   2480  C   CYS B   92     24.558  -7.655  54.562  1.00 13.61           C
ATOM   2481  O   CYS B   92     23.509  -7.004  54.437  1.00 13.85           O
ATOM   2482  CB  CYS B   92     26.231  -6.942  52.774  1.00 19.13           C
ATOM   2483  SG  CYS B   92     27.512  -7.511  51.717  1.00 20.85           S
ATOM   2484  N   PHE B   93     25.019  -8.100  55.730  1.00 13.22           N
ATOM   2485  CA  PHE B   93     24.483  -7.808  57.051  1.00 13.42           C
ATOM   2486  C   PHE B   93     25.606  -7.293  57.933  1.00 15.22           C
ATOM   2487  O   PHE B   93     26.572  -7.957  58.198  1.00 14.77           O
ATOM   2488  CB  PHE B   93     23.885  -9.132  57.628  1.00 14.20           C
ATOM   2489  CG  PHE B   93     23.397  -9.053  59.042  1.00 16.11           C
ATOM   2490  CD1 PHE B   93     22.431  -8.096  59.450  1.00 11.34           C
ATOM   2491  CD2 PHE B   93     23.777 -10.036  59.958  1.00 14.50           C
ATOM   2492  CE1 PHE B   93     21.967  -8.093  60.760  1.00 16.26           C
ATOM   2493  CE2 PHE B   93     23.306 -10.039  61.246  1.00 16.68           C
ATOM   2494  CZ  PHE B   93     22.407  -9.069  61.662  1.00 15.99           C
ATOM   2495  N   LEU B   94     25.491  -6.039  58.357  1.00 15.72           N
ATOM   2496  CA  LEU B   94     26.353  -5.486  59.344  1.00 14.67           C
ATOM   2497  C   LEU B   94     25.755  -5.785  60.727  1.00 14.97           C
ATOM   2498  O   LEU B   94     24.757  -5.172  61.120  1.00 14.38           O
ATOM   2499  CB  LEU B   94     26.483  -3.974  59.072  1.00 13.73           C
ATOM   2500  CG  LEU B   94     27.594  -3.216  59.781  1.00 19.48           C
ATOM   2501  CD1 LEU B   94     27.806  -1.822  59.027  1.00 19.17           C
ATOM   2502  CD2 LEU B   94     27.334  -2.998  61.222  1.00 19.95           C
ATOM   2503  N   PRO B   95     26.293  -6.806  61.440  1.00 13.86           N
ATOM   2504  CA  PRO B   95     25.663  -7.214  62.677  1.00 15.36           C
ATOM   2505  C   PRO B   95     25.644  -6.062  63.682  1.00 14.76           C
ATOM   2506  O   PRO B   95     26.682  -5.438  63.892  1.00 17.09           O
ATOM   2507  CB  PRO B   95     26.560  -8.341  63.176  1.00 16.23           C
ATOM   2508  CG  PRO B   95     27.197  -8.882  61.893  1.00 16.68           C
ATOM   2509  CD  PRO B   95     27.470  -7.626  61.130  1.00 14.50           C
ATOM   2510  N   MET B   96     24.502  -5.784  64.308  1.00 16.59           N
ATOM   2511  CA  MET B   96     23.240  -6.484  64.148  1.00 15.86           C
ATOM   2512  C   MET B   96     22.174  -5.592  63.503  1.00 17.78           C
ATOM   2513  O   MET B   96     20.979  -5.904  63.457  1.00 18.91           O
ATOM   2514  CB  MET B   96     22.718  -6.941  65.511  1.00 17.25           C
ATOM   2515  CG  MET B   96     23.586  -7.865  66.242  1.00 15.76           C
ATOM   2516  SD  MET B   96     23.553  -9.547  65.562  1.00 19.30           S
ATOM   2517  CE  MET B   96     21.893 -10.082  65.939  1.00 17.77           C
ATOM   2518  N   ASP B  101     22.651  -4.385  63.010  1.00 17.51           N
ATOM   2519  CA  ASP B  101     21.774  -3.250  62.647  1.00 17.93           C
ATOM   2520  C   ASP B  101     21.332  -3.152  61.186  1.00 16.96           C
ATOM   2521  O   ASP B  101     20.289  -2.538  60.965  1.00 17.68           O
ATOM   2522  CB  ASP B  101     22.310  -1.945  63.166  1.00 19.20           C
ATOM   2523  CG  ASP B  101     22.543  -1.976  64.654  1.00 25.29           C
ATOM   2524  OD1 ASP B  101     21.904  -2.751  65.400  1.00 24.89           O
ATOM   2525  OD2 ASP B  101     23.380  -1.196  65.066  1.00 29.58           O
```

72

```
ATOM   2526  N    TYR B 102    22.132  -3.570  60.192  1.00 16.74        N
ATOM   2527  CA   TYR B 102    21.898  -3.066  58.817  1.00 16.35        C
ATOM   2528  C    TYR B 102    21.934  -4.249  57.809  1.00 16.52        C
ATOM   2529  O    TYR B 102    22.840  -5.085  57.839  1.00 16.17        O
ATOM   2530  CB   TYR B 102    22.823  -1.999  58.375  1.00 16.34        C
ATOM   2531  CG   TYR B 102    22.816  -0.842  59.306  1.00 17.31        C
ATOM   2532  CD1  TYR B 102    21.734   0.029  59.330  1.00 19.67        C
ATOM   2533  CD2  TYR B 102    23.897  -0.605  60.130  1.00 17.46        C
ATOM   2534  CE1  TYR B 102    21.732   1.134  60.186  1.00 20.28        C
ATOM   2535  CE2  TYR B 102    23.911   0.486  60.999  1.00 17.77        C
ATOM   2536  CZ   TYR B 102    22.817   1.329  61.020  1.00 21.37        C
ATOM   2537  OH   TYR B 102    22.821   2.410  61.905  1.00 21.69        O
ATOM   2538  N    TRP B 103    20.976  -4.261  56.891  1.00 15.66        N
ATOM   2539  CA   TRP B 103    20.920  -5.229  55.775  1.00 16.20        C
ATOM   2540  C    TRP B 103    20.882  -4.478  54.454  1.00 16.65        C
ATOM   2541  O    TRP B 103    20.317  -3.367  54.365  1.00 16.54        O
ATOM   2542  CB   TRP B 103    19.609  -6.020  55.799  1.00 15.61        C
ATOM   2543  CG   TRP B 103    19.390  -6.848  56.989  1.00 17.25        C
ATOM   2544  CD1  TRP B 103    19.023  -6.424  58.222  1.00 18.25        C
ATOM   2545  CD2  TRP B 103    19.504  -8.278  57.068  1.00 16.33        C
ATOM   2546  NE1  TRP B 103    18.904  -7.496  59.082  1.00 15.86        N
ATOM   2547  CE2  TRP B 103    19.198  -8.646  58.390  1.00 17.68        C
ATOM   2548  CE3  TRP B 103    19.798  -9.276  56.144  1.00 19.03        C
ATOM   2549  CZ2  TRP B 103    19.193  -9.970  58.813  1.00 16.41        C
ATOM   2550  CZ3  TRP B 103    19.819 -10.587  56.569  1.00 17.06        C
ATOM   2551  CH2  TRP B 103    19.535 -10.922  57.899  1.00 16.45        C
ATOM   2552  N    GLY B 104    21.443  -5.102  53.426  1.00 16.90        N
ATOM   2553  CA   GLY B 104    21.340  -4.623  52.023  1.00 16.67        C
ATOM   2554  C    GLY B 104    20.031  -4.987  51.382  1.00 16.93        C
ATOM   2555  O    GLY B 104    19.120  -5.453  52.079  1.00 17.96        O
ATOM   2556  N    GLN B 105    19.889  -4.746  50.078  1.00 17.39        N
ATOM   2557  CA   GLN B 105    18.698  -5.147  49.330  1.00 17.85        C
ATOM   2558  C    GLN B 105    18.747  -6.584  48.818  1.00 17.34        C
ATOM   2559  O    GLN B 105    17.728  -7.100  48.427  1.00 18.67        O
ATOM   2560  CB   GLN B 105    18.370  -4.253  48.134  1.00 20.00        C
ATOM   2561  CG   GLN B 105    19.021  -4.556  46.779  1.00 24.04        C
ATOM   2562  CD   GLN B 105    20.498  -4.249  46.699  1.00 26.58        C
ATOM   2563  OE1  GLN B 105    21.209  -4.718  45.777  1.00 27.19        O
ATOM   2564  NE2  GLN B 105    20.987  -3.451  47.649  1.00 28.56        N
ATOM   2565  N    GLY B 106    19.936  -7.162  48.814  1.00 17.97        N
ATOM   2566  CA   GLY B 106    20.167  -8.550  48.364  1.00 16.57        C
ATOM   2567  C    GLY B 106    20.501  -8.622  46.894  1.00 17.38        C
ATOM   2568  O    GLY B 106    20.024  -7.787  46.097  1.00 18.34        O
ATOM   2569  N    THR B 107    21.349  -9.582  46.502  1.00 15.63        N
ATOM   2570  CA   THR B 107    21.602  -9.855  45.116  1.00 15.36        C
ATOM   2571  C    THR B 107    21.321 -11.343  44.903  1.00 15.72        C
ATOM   2572  O    THR B 107    21.662 -12.140  45.768  1.00 14.87        O
ATOM   2573  CB   THR B 107    23.062  -9.508  44.710  1.00 15.69        C
ATOM   2574  OG1  THR B 107    23.210  -9.598  43.281  1.00 17.61        O
ATOM   2575  CG2  THR B 107    24.093 -10.394  45.382  1.00 18.72        C
ATOM   2576  N    SER B 108    20.657 -11.676  43.814  1.00 15.77        N
ATOM   2577  CA   SER B 108    20.167 -13.049  43.591  1.00 15.24        C
ATOM   2578  C    SER B 108    21.208 -13.863  42.852  1.00 15.29        C
ATOM   2579  O    SER B 108    21.707 -13.445  41.819  1.00 15.07        O
ATOM   2580  CB   SER B 108    18.872 -12.960  42.785  1.00 16.18        C
ATOM   2581  OG   SER B 108    18.373 -14.264  42.435  1.00 16.59        O
ATOM   2582  N    VAL B 109    21.435 -15.100  43.335  1.00 15.39        N
ATOM   2583  CA   VAL B 109    22.391 -16.030  42.769  1.00 16.27        C
ATOM   2584  C    VAL B 109    21.619 -17.330  42.461  1.00 15.31        C
ATOM   2585  O    VAL B 109    20.944 -17.848  43.322  1.00 14.32        O
ATOM   2586  CB   VAL B 109    23.539 -16.297  43.748  1.00 17.92        C
ATOM   2587  CG1  VAL B 109    24.394 -17.478  43.285  1.00 16.30        C
ATOM   2588  CG2  VAL B 109    24.435 -15.003  43.913  1.00 17.45        C
ATOM   2589  N    THR B 110    21.692 -17.760  41.212  1.00 15.28        N
ATOM   2590  CA   THR B 110    21.094 -19.028  40.776  1.00 15.07        C
```

```
ATOM   2591  C    THR B 110    22.215 -19.944  40.289  1.00 14.47        C
ATOM   2592  O    THR B 110    23.098 -19.533  39.539  1.00 14.59        O
ATOM   2593  CB   THR B 110    20.033 -18.795  39.710  1.00 16.56        C
ATOM   2594  OG1  THR B 110    19.009 -17.943  40.251  1.00 16.20        O
ATOM   2595  CG2  THR B 110    19.431 -20.157  39.278  1.00 17.73        C
ATOM   2596  N    VAL B 111    22.181 -21.195  40.761  1.00 13.71        N
ATOM   2597  CA   VAL B 111    23.156 -22.205  40.362  1.00 15.27        C
ATOM   2598  C    VAL B 111    22.334 -23.285  39.661  1.00 17.85        C
ATOM   2599  O    VAL B 111    21.502 -23.989  40.284  1.00 17.62        O
ATOM   2600  CB   VAL B 111    23.928 -22.792  41.574  1.00 16.15        C
ATOM   2601  CG1  VAL B 111    24.887 -23.881  41.134  1.00 16.38        C
ATOM   2602  CG2  VAL B 111    24.720 -21.714  42.312  1.00 16.07        C
ATOM   2603  N    SER B 112    22.535 -23.385  38.358  1.00 16.61        N
ATOM   2604  CA   SER B 112    21.724 -24.268  37.493  1.00 17.63        C
ATOM   2605  C    SER B 112    22.393 -24.503  36.159  1.00 19.40        C
ATOM   2606  O    SER B 112    23.086 -23.652  35.641  1.00 18.73        O
ATOM   2607  CB   SER B 112    20.304 -23.693  37.308  1.00 18.23        C
ATOM   2608  OG   SER B 112    19.490 -24.419  36.370  1.00 18.63        O
ATOM   2609  N    SER B 113    22.156 -25.678  35.575  1.00 19.83        N
ATOM   2610  CA   SER B 113    22.583 -25.897  34.191  1.00 21.90        C
ATOM   2611  C    SER B 113    21.602 -25.397  33.122  1.00 20.66        C
ATOM   2612  O    SER B 113    21.940 -25.363  31.923  1.00 22.24        O
ATOM   2613  CB   SER B 113    22.837 -27.370  33.986  1.00 23.90        C
ATOM   2614  OG   SER B 113    21.617 -28.021  34.174  1.00 25.86        O
ATOM   2615  N    ALA B 114    20.419 -24.949  33.535  1.00 19.58        N
ATOM   2616  CA   ALA B 114    19.418 -24.468  32.599  1.00 20.13        C
ATOM   2617  C    ALA B 114    19.889 -23.148  32.002  1.00 21.23        C
ATOM   2618  O    ALA B 114    20.637 -22.412  32.628  1.00 22.24        O
ATOM   2619  CB   ALA B 114    18.061 -24.290  33.293  1.00 21.90        C
ATOM   2620  N    LYS B 115    19.393 -22.837  30.820  1.00 22.21        N
ATOM   2621  CA  ALYS B 115    19.851 -21.661  30.099  0.50 22.22        C
ATOM   2622  CA  BLYS B 115    19.861 -21.659  30.110  0.50 22.37        C
ATOM   2623  C    LYS B 115    19.032 -20.423  30.402  1.00 22.28        C
ATOM   2624  O    LYS B 115    17.800 -20.486  30.586  1.00 21.52        O
ATOM   2625  CB  ALYS B 115    19.842 -21.927  28.599  0.50 22.88        C
ATOM   2626  CB  BLYS B 115    19.925 -21.919  28.608  0.50 23.24        C
ATOM   2627  CG  ALYS B 115    20.466 -23.259  28.180  0.50 24.61        C
ATOM   2628  CG  BLYS B 115    18.644 -21.666  27.828  0.50 25.10        C
ATOM   2629  CD  ALYS B 115    21.848 -23.514  28.796  0.50 23.88        C
ATOM   2630  CD  BLYS B 115    18.863 -21.967  26.370  0.50 25.91        C
ATOM   2631  CE  ALYS B 115    22.179 -24.995  28.728  0.50 24.70        C
ATOM   2632  CE  BLYS B 115    17.573 -22.091  25.581  0.50 24.84        C
ATOM   2633  NZ  ALYS B 115    21.263 -25.848  29.558  0.50 23.68        N
ATOM   2634  NZ  BLYS B 115    16.610 -20.959  25.648  0.50 27.23        N
ATOM   2635  N    THR B 116    19.719 -19.296  30.414  1.00 21.40        N
ATOM   2636  CA   THR B 116    19.070 -18.028  30.568  1.00 20.99        C
ATOM   2637  C    THR B 116    18.246 -17.815  29.318  1.00 21.08        C
ATOM   2638  O    THR B 116    18.734 -17.970  28.203  1.00 22.88        O
ATOM   2639  CB   THR B 116    20.082 -16.890  30.724  1.00 21.46        C
ATOM   2640  OG1  THR B 116    20.726 -16.993  32.009  1.00 21.92        O
ATOM   2641  CG2  THR B 116    19.422 -15.516  30.575  1.00 21.27        C
ATOM   2642  N    THR B 117    16.997 -17.419  29.531  1.00 22.05        N
ATOM   2643  CA   THR B 117    16.017 -17.197  28.483  1.00 20.61        C
ATOM   2644  C    THR B 117    15.231 -15.936  28.852  1.00 19.57        C
ATOM   2645  O    THR B 117    14.712 -15.860  29.960  1.00 18.64        O
ATOM   2646  CB   THR B 117    15.074 -18.427  28.461  1.00 21.42        C
ATOM   2647  OG1  THR B 117    15.854 -19.628  28.265  1.00 25.53        O
ATOM   2648  CG2  THR B 117    14.032 -18.313  27.377  1.00 21.87        C
ATOM   2649  N    PRO B 118    15.146 -14.925  27.960  1.00 18.59        N
ATOM   2650  CA   PRO B 118    14.287 -13.804  28.325  1.00 18.28        C
ATOM   2651  C    PRO B 118    12.783 -14.186  28.216  1.00 18.15        C
ATOM   2652  O    PRO B 118    12.442 -15.139  27.464  1.00 18.91        O
ATOM   2653  CB   PRO B 118    14.621 -12.770  27.252  1.00 18.30        C
ATOM   2654  CG   PRO B 118    14.967 -13.576  26.084  1.00 18.91        C
ATOM   2655  CD   PRO B 118    15.738 -14.729  26.625  1.00 17.93        C
```

```
ATOM   2656  N   PRO B 119     11.909 -13.450  28.918  1.00 20.04           N
ATOM   2657  CA  PRO B 119     10.482 -13.687  28.844  1.00 20.84           C
ATOM   2658  C   PRO B 119      9.876 -13.215  27.545  1.00 21.74           C
ATOM   2659  O   PRO B 119     10.377 -12.269  26.935  1.00 22.19           O
ATOM   2660  CB  PRO B 119      9.911 -12.884  30.009  1.00 20.84           C
ATOM   2661  CG  PRO B 119     10.839 -11.783  30.203  1.00 20.97           C
ATOM   2662  CD  PRO B 119     12.220 -12.295  29.774  1.00 20.41           C
ATOM   2663  N   SER B 120      8.828 -13.917  27.109  1.00 22.39           N
ATOM   2664  CA  SER B 120      7.875 -13.329  26.180  1.00 22.67           C
ATOM   2665  C   SER B 120      6.840 -12.588  26.995  1.00 24.28           C
ATOM   2666  O   SER B 120      6.362 -13.092  27.997  1.00 24.75           O
ATOM   2667  CB  SER B 120      7.207 -14.388  25.311  1.00 22.19           C
ATOM   2668  OG  SER B 120      8.161 -15.186  24.643  1.00 23.57           O
ATOM   2669  N   VAL B 121      6.499 -11.377  26.579  1.00 22.82           N
ATOM   2670  CA  VAL B 121      5.615 -10.512  27.316  1.00 24.64           C
ATOM   2671  C   VAL B 121      4.377 -10.236  26.449  1.00 25.57           C
ATOM   2672  O   VAL B 121      4.508  -9.718  25.339  1.00 26.39           O
ATOM   2673  CB  VAL B 121      6.327  -9.218  27.701  1.00 24.98           C
ATOM   2674  CG1 VAL B 121      5.397  -8.325  28.472  1.00 24.99           C
ATOM   2675  CG2 VAL B 121      7.586  -9.556  28.550  1.00 25.85           C
ATOM   2676  N   TYR B 122      3.206 -10.623  26.958  1.00 24.43           N
ATOM   2677  CA  TYR B 122      1.945 -10.535  26.214  1.00 25.11           C
ATOM   2678  C   TYR B 122      0.927  -9.681  26.898  1.00 27.36           C
ATOM   2679  O   TYR B 122      0.706  -9.814  28.101  1.00 25.86           O
ATOM   2680  CB  TYR B 122      1.332 -11.902  26.004  1.00 25.79           C
ATOM   2681  CG  TYR B 122      2.234 -12.901  25.349  1.00 24.09           C
ATOM   2682  CD1 TYR B 122      2.706 -12.720  24.044  1.00 27.29           C
ATOM   2683  CD2 TYR B 122      2.582 -14.065  26.014  1.00 27.70           C
ATOM   2684  CE1 TYR B 122      3.523 -13.660  23.449  1.00 24.67           C
ATOM   2685  CE2 TYR B 122      3.379 -15.004  25.419  1.00 25.29           C
ATOM   2686  CZ  TYR B 122      3.832 -14.811  24.138  1.00 26.73           C
ATOM   2687  OH  TYR B 122      4.617 -15.779  23.586  1.00 27.94           O
ATOM   2688  N   PRO B 123      0.228  -8.837  26.113  1.00 31.08           N
ATOM   2689  CA  PRO B 123     -0.775  -7.945  26.671  1.00 31.68           C
ATOM   2690  C   PRO B 123     -1.996  -8.744  27.041  1.00 31.81           C
ATOM   2691  O   PRO B 123     -2.363  -9.663  26.317  1.00 34.65           O
ATOM   2692  CB  PRO B 123     -1.097  -6.997  25.509  1.00 32.47           C
ATOM   2693  CG  PRO B 123     -0.811  -7.827  24.263  1.00 32.99           C
ATOM   2694  CD  PRO B 123      0.341  -8.721  24.644  1.00 32.45           C
ATOM   2695  N   LEU B 124     -2.594  -8.424  28.174  1.00 30.92           N
ATOM   2696  CA  LEU B 124     -3.871  -8.976  28.541  1.00 31.66           C
ATOM   2697  C   LEU B 124     -4.896  -7.830  28.527  1.00 32.40           C
ATOM   2698  O   LEU B 124     -5.017  -7.061  29.482  1.00 30.19           O
ATOM   2699  CB  LEU B 124     -3.809  -9.636  29.906  1.00 31.52           C
ATOM   2700  CG  LEU B 124     -2.767 -10.747  30.085  1.00 31.17           C
ATOM   2701  CD1 LEU B 124     -2.716 -11.083  31.555  1.00 30.86           C
ATOM   2702  CD2 LEU B 124     -3.098 -11.964  29.284  1.00 33.41           C
ATOM   2703  N   ALA B 125     -5.623  -7.732  27.415  1.00 35.32           N
ATOM   2704  CA  ALA B 125     -6.648  -6.701  27.249  1.00 35.90           C
ATOM   2705  C   ALA B 125     -8.021  -7.351  27.388  1.00 37.65           C
ATOM   2706  O   ALA B 125     -8.229  -8.491  26.941  1.00 38.35           O
ATOM   2707  CB  ALA B 125     -6.518  -6.049  25.902  1.00 37.01           C
ATOM   2708  N   PRO B 126     -8.967  -6.645  28.017  1.00 39.69           N
ATOM   2709  CA  PRO B 126    -10.328  -7.141  28.029  1.00 40.55           C
ATOM   2710  C   PRO B 126    -11.012  -6.878  26.689  1.00 41.78           C
ATOM   2711  O   PRO B 126    -10.731  -5.851  26.047  1.00 43.50           O
ATOM   2712  CB  PRO B 126    -10.971  -6.325  29.135  1.00 40.90           C
ATOM   2713  CG  PRO B 126    -10.245  -5.044  29.119  1.00 40.61           C
ATOM   2714  CD  PRO B 126     -8.840  -5.374  28.744  1.00 39.67           C
ATOM   2715  N   SER B 136    -16.691   1.395  35.066  1.00 51.53           N
ATOM   2716  CA  SER B 136    -16.460   1.678  36.488  1.00 51.29           C
ATOM   2717  C   SER B 136    -14.977   1.488  36.849  1.00 50.48           C
ATOM   2718  O   SER B 136    -14.218   2.461  36.950  1.00 50.10           O
ATOM   2719  CB  SER B 136    -17.364   0.792  37.360  1.00 51.68           C
ATOM   2720  OG  SER B 136    -17.006   0.867  38.735  1.00 52.87           O
```

```
ATOM   2721  N    MET B 137   -14.585   0.229  37.049  1.00 49.34        N
ATOM   2722  CA   MET B 137   -13.176  -0.151  37.162  1.00 48.05        C
ATOM   2723  C    MET B 137   -12.886  -1.161  36.058  1.00 46.08        C
ATOM   2724  O    MET B 137   -13.751  -1.955  35.696  1.00 45.67        O
ATOM   2725  CB   MET B 137   -12.886  -0.765  38.536  1.00 49.29        C
ATOM   2726  CG   MET B 137   -12.938   0.236  39.685  1.00 50.83        C
ATOM   2727  SD   MET B 137   -11.494   1.324  39.707  1.00 55.23        S
ATOM   2728  CE   MET B 137   -10.459   0.452  40.877  1.00 54.55        C
ATOM   2729  N    VAL B 138   -11.672  -1.119  35.514  1.00 43.54        N
ATOM   2730  CA   VAL B 138   -11.265  -2.044  34.454  1.00 40.75        C
ATOM   2731  C    VAL B 138    -9.951  -2.703  34.891  1.00 38.48        C
ATOM   2732  O    VAL B 138    -9.049  -2.021  35.387  1.00 36.44        O
ATOM   2733  CB   VAL B 138   -11.128  -1.320  33.089  1.00 40.91        C
ATOM   2734  CG1  VAL B 138   -10.002  -0.287  33.099  1.00 41.46        C
ATOM   2735  CG2  VAL B 138   -10.922  -2.320  31.964  1.00 41.60        C
ATOM   2736  N    THR B 139    -9.878  -4.030  34.752  1.00 35.44        N
ATOM   2737  CA   THR B 139    -8.666  -4.770  35.110  1.00 33.88        C
ATOM   2738  C    THR B 139    -7.937  -5.172  33.843  1.00 33.10        C
ATOM   2739  O    THR B 139    -8.537  -5.738  32.925  1.00 33.18        O
ATOM   2740  CB   THR B 139    -9.005  -5.964  36.014  1.00 33.89        C
ATOM   2741  OG1  THR B 139    -9.430  -5.456  37.287  1.00 32.25        O
ATOM   2742  CG2  THR B 139    -7.788  -6.904  36.229  1.00 32.59        C
ATOM   2743  N    LEU B 140    -6.656  -4.799  33.777  1.00 32.01        N
ATOM   2744  CA   LEU B 140    -5.762  -5.180  32.683  1.00 31.23        C
ATOM   2745  C    LEU B 140    -4.614  -6.068  33.207  1.00 28.84        C
ATOM   2746  O    LEU B 140    -4.455  -6.248  34.429  1.00 26.81        O
ATOM   2747  CB   LEU B 140    -5.172  -3.941  32.022  1.00 31.60        C
ATOM   2748  CG   LEU B 140    -6.140  -2.777  31.727  1.00 32.50        C
ATOM   2749  CD1  LEU B 140    -5.384  -1.528  31.293  1.00 32.75        C
ATOM   2750  CD2  LEU B 140    -7.211  -3.137  30.702  1.00 33.27        C
ATOM   2751  N    GLY B 141    -3.803  -6.583  32.291  1.00 27.43        N
ATOM   2752  CA   GLY B 141    -2.585  -7.279  32.686  1.00 27.04        C
ATOM   2753  C    GLY B 141    -1.533  -7.548  31.627  1.00 25.36        C
ATOM   2754  O    GLY B 141    -1.675  -7.194  30.446  1.00 25.73        O
ATOM   2755  N    CYS B 142    -0.441  -8.158  32.070  1.00 26.20        N
ATOM   2756  CA  ACYS B 142     0.572  -8.735  31.190  0.50 25.42        C
ATOM   2757  CA  BCYS B 142     0.445  -8.810  31.114  0.50 25.94        C
ATOM   2758  C    CYS B 142     0.972 -10.127  31.654  1.00 24.88        C
ATOM   2759  O    CYS B 142     1.074 -10.362  32.874  1.00 21.91        O
ATOM   2760  CB  ACYS B 142     1.807  -7.855  31.164  0.50 26.77        C
ATOM   2761  CB  BCYS B 142     1.541  -7.903  30.513  0.50 27.94        C
ATOM   2762  SG  ACYS B 142     1.534  -6.392  30.158  0.50 28.45        S
ATOM   2763  SG  BCYS B 142     2.939  -7.390  31.525  0.50 30.71        S
ATOM   2764  N    LEU B 143     1.222 -10.984  30.682  1.00 22.57        N
ATOM   2765  CA   LEU B 143     1.648 -12.332  30.874  1.00 22.85        C
ATOM   2766  C    LEU B 143     3.133 -12.346  30.552  1.00 20.89        C
ATOM   2767  O    LEU B 143     3.552 -11.957  29.456  1.00 19.84        O
ATOM   2768  CB   LEU B 143     0.853 -13.242  29.947  1.00 22.74        C
ATOM   2769  CG   LEU B 143     1.259 -14.721  29.997  1.00 22.08        C
ATOM   2770  CD1  LEU B 143     1.189 -15.272  31.413  1.00 22.75        C
ATOM   2771  CD2  LEU B 143     0.402 -15.538  29.071  1.00 22.62        C
ATOM   2772  N    VAL B 144     3.927 -12.794  31.524  1.00 19.56        N
ATOM   2773  CA   VAL B 144     5.369 -12.809  31.391  1.00 18.84        C
ATOM   2774  C    VAL B 144     5.779 -14.278  31.376  1.00 18.55        C
ATOM   2775  O    VAL B 144     5.968 -14.880  32.443  1.00 18.22        O
ATOM   2776  CB   VAL B 144     6.028 -12.072  32.564  1.00 20.57        C
ATOM   2777  CG1  VAL B 144     7.555 -11.889  32.330  1.00 20.26        C
ATOM   2778  CG2  VAL B 144     5.385 -10.692  32.739  1.00 21.83        C
ATOM   2779  N    LYS B 145     5.968 -14.826  30.182  1.00 17.33        N
ATOM   2780  CA   LYS B 145     6.039 -16.279  30.023  1.00 19.19        C
ATOM   2781  C    LYS B 145     7.395 -16.762  29.554  1.00 17.46        C
ATOM   2782  O    LYS B 145     7.992 -16.247  28.602  1.00 18.51        O
ATOM   2783  CB   LYS B 145     4.970 -16.745  29.045  1.00 19.72        C
ATOM   2784  CG   LYS B 145     5.009 -18.233  28.736  1.00 20.99        C
ATOM   2785  CD   LYS B 145     3.809 -18.659  27.982  1.00 22.36        C
```

```
ATOM   2786  CE   LYS B 145      3.857 -20.161  27.708  1.00 25.41           C
ATOM   2787  NZ   LYS B 145      3.969 -21.016  28.949  1.00 29.60           N
ATOM   2788  N    GLY B 146      7.864 -17.801  30.229  1.00 15.99           N
ATOM   2789  CA   GLY B 146      8.963 -18.622  29.736  1.00 16.35           C
ATOM   2790  C    GLY B 146     10.338 -18.001  29.861  1.00 16.89           C
ATOM   2791  O    GLY B 146     11.085 -17.966  28.883  1.00 17.54           O
ATOM   2792  N    TYR B 147     10.664 -17.499  31.053  1.00 16.07           N
ATOM   2793  CA   TYR B 147     11.987 -16.914  31.313  1.00 15.88           C
ATOM   2794  C    TYR B 147     12.795 -17.773  32.323  1.00 17.01           C
ATOM   2795  O    TYR B 147     12.263 -18.629  33.067  1.00 18.43           O
ATOM   2796  CB   TYR B 147     11.870 -15.465  31.763  1.00 17.83           C
ATOM   2797  CG   TYR B 147     11.213 -15.287  33.087  1.00 16.22           C
ATOM   2798  CD1  TYR B 147      9.831 -15.140  33.195  1.00 15.73           C
ATOM   2799  CD2  TYR B 147     11.953 -15.260  34.259  1.00 15.54           C
ATOM   2800  CE1  TYR B 147      9.192 -14.983  34.437  1.00 16.81           C
ATOM   2801  CE2  TYR B 147     11.331 -15.099  35.503  1.00 18.91           C
ATOM   2802  CZ   TYR B 147      9.942 -14.994  35.588  1.00 15.79           C
ATOM   2803  OH   TYR B 147      9.311 -14.799  36.789  1.00 18.10           O
ATOM   2804  N    PHE B 148     14.108 -17.588  32.313  1.00 15.30           N
ATOM   2805  CA   PHE B 148     14.960 -18.199  33.298  1.00 16.01           C
ATOM   2806  C    PHE B 148     16.280 -17.407  33.373  1.00 16.56           C
ATOM   2807  O    PHE B 148     16.755 -16.940  32.329  1.00 16.54           O
ATOM   2808  CB   PHE B 148     15.253 -19.686  32.944  1.00 16.04           C
ATOM   2809  CG   PHE B 148     15.992 -20.423  34.030  1.00 15.02           C
ATOM   2810  CD1  PHE B 148     15.294 -21.033  35.073  1.00 15.77           C
ATOM   2811  CD2  PHE B 148     17.403 -20.432  34.091  1.00 18.78           C
ATOM   2812  CE1  PHE B 148     15.938 -21.660  36.089  1.00 16.28           C
ATOM   2813  CE2  PHE B 148     18.031 -21.019  35.155  1.00 19.20           C
ATOM   2814  CZ   PHE B 148     17.296 -21.639  36.154  1.00 17.77           C
ATOM   2815  N    PRO B 149     16.859 -17.244  34.578  1.00 16.86           N
ATOM   2816  CA   PRO B 149     16.372 -17.553  35.930  1.00 17.11           C
ATOM   2817  C    PRO B 149     15.480 -16.408  36.434  1.00 16.25           C
ATOM   2818  O    PRO B 149     15.271 -15.465  35.717  1.00 16.79           O
ATOM   2819  CB   PRO B 149     17.664 -17.633  36.738  1.00 17.11           C
ATOM   2820  CG   PRO B 149     18.499 -16.548  36.105  1.00 17.18           C
ATOM   2821  CD   PRO B 149     18.201 -16.630  34.624  1.00 16.15           C
ATOM   2822  N    GLU B 150     14.945 -16.558  37.633  1.00 18.36           N
ATOM   2823  CA   GLU B 150     14.440 -15.429  38.382  1.00 18.21           C
ATOM   2824  C    GLU B 150     15.582 -14.501  38.697  1.00 18.67           C
ATOM   2825  O    GLU B 150     16.740 -14.914  38.773  1.00 19.71           O
ATOM   2826  CB   GLU B 150     13.789 -15.915  39.670  1.00 18.60           C
ATOM   2827  CG   GLU B 150     12.509 -16.636  39.431  1.00 20.83           C
ATOM   2828  CD   GLU B 150     11.305 -15.732  39.570  1.00 23.17           C
ATOM   2829  OE1  GLU B 150     10.519 -15.985  40.487  1.00 25.52           O
ATOM   2830  OE2  GLU B 150     11.164 -14.732  38.820  1.00 22.99           O
ATOM   2831  N    PRO B 151     15.275 -13.206  38.899  1.00 19.84           N
ATOM   2832  CA   PRO B 151     14.004 -12.549  38.883  1.00 18.90           C
ATOM   2833  C    PRO B 151     13.690 -11.788  37.603  1.00 17.93           C
ATOM   2834  O    PRO B 151     14.548 -11.663  36.741  1.00 17.67           O
ATOM   2835  CB   PRO B 151     14.166 -11.552  40.024  1.00 18.09           C
ATOM   2836  CG   PRO B 151     15.589 -11.074  39.843  1.00 19.26           C
ATOM   2837  CD   PRO B 151     16.343 -12.277  39.298  1.00 18.60           C
ATOM   2838  N    VAL B 152     12.451 -11.304  37.514  1.00 20.19           N
ATOM   2839  CA   VAL B 152     12.026 -10.211  36.608  1.00 19.55           C
ATOM   2840  C    VAL B 152     11.643  -9.061  37.539  1.00 20.67           C
ATOM   2841  O    VAL B 152     11.245  -9.284  38.675  1.00 21.68           O
ATOM   2842  CB   VAL B 152     10.932 -10.589  35.597  1.00 20.94           C
ATOM   2843  CG1  VAL B 152     11.490 -11.611  34.599  1.00 17.94           C
ATOM   2844  CG2  VAL B 152      9.658 -11.028  36.283  1.00 20.63           C
ATOM   2845  N    THR B 153     11.636  -7.855  37.034  1.00 21.21           N
ATOM   2846  CA   THR B 153     10.823  -6.802  37.640  1.00 24.37           C
ATOM   2847  C    THR B 153      9.823  -6.231  36.698  1.00 23.59           C
ATOM   2848  O    THR B 153     10.150  -5.865  35.562  1.00 23.56           O
ATOM   2849  CB   THR B 153     11.789  -5.661  38.140  1.00 25.00           C
ATOM   2850  OG1  THR B 153     12.382  -4.977  37.030  1.00 31.95           O
```

```
ATOM  2851  CG2 THR B 153   12.908  -6.185  38.993  1.00 26.31        C
ATOM  2852  N   VAL B 154    8.601  -6.191  37.192  1.00 25.30        N
ATOM  2853  CA  VAL B 154    7.459  -5.723  36.436  1.00 26.25        C
ATOM  2854  C   VAL B 154    7.015  -4.388  37.045  1.00 26.84        C
ATOM  2855  O   VAL B 154    6.846  -4.270  38.276  1.00 26.09        O
ATOM  2856  CB  VAL B 154    6.298  -6.728  36.515  1.00 26.73        C
ATOM  2857  CG1 VAL B 154    5.084  -6.243  35.695  1.00 27.41        C
ATOM  2858  CG2 VAL B 154    6.753  -8.087  36.059  1.00 25.15        C
ATOM  2859  N   THR B 155    6.832  -3.410  36.175  1.00 28.57        N
ATOM  2860  CA  THR B 155    6.232  -2.129  36.562  1.00 29.95        C
ATOM  2861  C   THR B 155    5.125  -1.760  35.578  1.00 30.94        C
ATOM  2862  O   THR B 155    5.021  -2.342  34.494  1.00 31.32        O
ATOM  2863  CB  THR B 155    7.287  -0.980  36.632  1.00 29.15        C
ATOM  2864  OG1 THR B 155    7.942  -0.830  35.375  1.00 28.12        O
ATOM  2865  CG2 THR B 155    8.325  -1.272  37.688  1.00 30.85        C
ATOM  2866  N   TRP B 156    4.301  -0.782  35.968  1.00 32.81        N
ATOM  2867  CA  TRP B 156    3.330  -0.213  35.060  1.00 33.12        C
ATOM  2868  C   TRP B 156    3.570   1.289  34.889  1.00 35.27        C
ATOM  2869  O   TRP B 156    3.859   1.989  35.856  1.00 35.23        O
ATOM  2870  CB  TRP B 156    1.919  -0.480  35.550  1.00 32.52        C
ATOM  2871  CG  TRP B 156    1.529  -1.916  35.553  1.00 31.24        C
ATOM  2872  CD1 TRP B 156    1.654  -2.787  36.586  1.00 31.40        C
ATOM  2873  CD2 TRP B 156    0.914  -2.642  34.485  1.00 30.96        C
ATOM  2874  NE1 TRP B 156    1.184  -4.027  36.222  1.00 29.55        N
ATOM  2875  CE2 TRP B 156    0.727  -3.965  34.934  1.00 31.40        C
ATOM  2876  CE3 TRP B 156    0.534  -2.314  33.178  1.00 30.76        C
ATOM  2877  CZ2 TRP B 156    0.151  -4.943  34.138  1.00 30.38        C
ATOM  2878  CZ3 TRP B 156   -0.051  -3.286  32.394  1.00 31.40        C
ATOM  2879  CH2 TRP B 156   -0.230  -4.584  32.871  1.00 31.07        C
ATOM  2880  N   ASN B 157    3.451   1.759  33.655  1.00 37.40        N
ATOM  2881  CA  ASN B 157    3.701   3.165  33.327  1.00 39.27        C
ATOM  2882  C   ASN B 157    4.999   3.641  33.978  1.00 40.43        C
ATOM  2883  O   ASN B 157    5.037   4.665  34.693  1.00 38.90        O
ATOM  2884  CB  ASN B 157    2.511   4.034  33.751  1.00 39.76        C
ATOM  2885  CG  ASN B 157    1.283   3.824  32.875  1.00 39.69        C
ATOM  2886  OD1 ASN B 157    1.352   3.225  31.806  1.00 40.23        O
ATOM  2887  ND2 ASN B 157    0.150   4.357  33.320  1.00 41.95        N
ATOM  2888  N   SER B 158    6.049   2.856  33.732  1.00 41.82        N
ATOM  2889  CA  SER B 158    7.404   3.123  34.228  1.00 42.20        C
ATOM  2890  C   SER B 158    7.473   3.312  35.743  1.00 42.55        C
ATOM  2891  O   SER B 158    8.383   3.961  36.235  1.00 42.99        O
ATOM  2892  CB  SER B 158    8.011   4.341  33.522  1.00 43.05        C
ATOM  2893  OG  SER B 158    7.990   4.187  32.117  1.00 43.01        O
ATOM  2894  N   GLY B 159    6.533   2.729  36.481  1.00 43.07        N
ATOM  2895  CA  GLY B 159    6.510   2.860  37.940  1.00 43.29        C
ATOM  2896  C   GLY B 159    5.520   3.895  38.459  1.00 43.73        C
ATOM  2897  O   GLY B 159    5.235   3.939  39.665  1.00 43.57        O
ATOM  2898  N   SER B 160    4.989   4.718  37.555  1.00 44.54        N
ATOM  2899  CA  SER B 160    3.957   5.725  37.897  1.00 44.92        C
ATOM  2900  C   SER B 160    2.691   5.079  38.437  1.00 44.68        C
ATOM  2901  O   SER B 160    2.067   5.579  39.380  1.00 44.21        O
ATOM  2902  CB  SER B 160    3.577   6.527  36.656  1.00 44.90        C
ATOM  2903  OG  SER B 160    4.734   7.002  36.000  1.00 47.26        O
ATOM  2904  N   LEU B 161    2.319   3.961  37.822  1.00 43.95        N
ATOM  2905  CA  LEU B 161    1.117   3.257  38.178  1.00 42.85        C
ATOM  2906  C   LEU B 161    1.531   2.140  39.121  1.00 42.80        C
ATOM  2907  O   LEU B 161    2.160   1.174  38.695  1.00 41.72        O
ATOM  2908  CB  LEU B 161    0.451   2.709  36.918  1.00 43.28        C
ATOM  2909  CG  LEU B 161   -1.073   2.614  36.903  1.00 43.09        C
ATOM  2910  CD1 LEU B 161   -1.495   1.781  35.710  1.00 43.27        C
ATOM  2911  CD2 LEU B 161   -1.643   2.037  38.188  1.00 43.70        C
ATOM  2912  N   SER B 162    1.192   2.294  40.399  1.00 42.22        N
ATOM  2913  CA  SER B 162    1.683   1.414  41.456  1.00 41.98        C
ATOM  2914  C   SER B 162    0.558   0.813  42.266  1.00 41.40        C
ATOM  2915  O   SER B 162    0.620  -0.354  42.653  1.00 41.49        O
```

| ATOM | 2916 | CB | SER | B | 162 | 2.626 | 2.182 | 42.395 | 1.00 | 42.58 | C |
|------|------|-----|-----|---|-----|-------|-------|--------|------|-------|---|
| ATOM | 2917 | OG | SER | B | 162 | 1.914 | 2.912 | 43.388 | 1.00 | 42.34 | O |
| ATOM | 2918 | N | SER | B | 163 | -0.459 | 1.618 | 42.557 | 1.00 | 40.21 | N |
| ATOM | 2919 | CA | SER | B | 163 | -1.620 | 1.130 | 43.271 | 1.00 | 40.05 | C |
| ATOM | 2920 | C | SER | B | 163 | -2.537 | 0.330 | 42.340 | 1.00 | 38.60 | C |
| ATOM | 2921 | O | SER | B | 163 | -2.565 | 0.533 | 41.123 | 1.00 | 37.33 | O |
| ATOM | 2922 | CB | SER | B | 163 | -2.380 | 2.292 | 43.910 | 1.00 | 40.92 | C |
| ATOM | 2923 | OG | SER | B | 163 | -1.466 | 3.177 | 44.544 | 1.00 | 42.28 | O |
| ATOM | 2924 | N | GLY | B | 164 | -3.277 | -0.584 | 42.943 | 1.00 | 37.55 | N |
| ATOM | 2925 | CA | GLY | B | 164 | -4.165 | -1.469 | 42.219 | 1.00 | 37.39 | C |
| ATOM | 2926 | C | GLY | B | 164 | -3.462 | -2.578 | 41.451 | 1.00 | 36.60 | C |
| ATOM | 2927 | O | GLY | B | 164 | -4.099 | -3.217 | 40.608 | 1.00 | 36.78 | O |
| ATOM | 2928 | N | VAL | B | 165 | -2.170 | -2.798 | 41.739 | 1.00 | 35.57 | N |
| ATOM | 2929 | CA | VAL | B | 165 | -1.329 | -3.767 | 41.009 | 1.00 | 34.05 | C |
| ATOM | 2930 | C | VAL | B | 165 | -1.159 | -5.089 | 41.770 | 1.00 | 32.89 | C |
| ATOM | 2931 | O | VAL | B | 165 | -0.923 | -5.109 | 42.978 | 1.00 | 31.62 | O |
| ATOM | 2932 | CB | VAL | B | 165 | 0.053 | -3.171 | 40.682 | 1.00 | 34.42 | C |
| ATOM | 2933 | CG1 | VAL | B | 165 | 1.022 | -4.250 | 40.106 | 1.00 | 35.86 | C |
| ATOM | 2934 | CG2 | VAL | B | 165 | -0.082 | -2.047 | 39.686 | 1.00 | 35.06 | C |
| ATOM | 2935 | N | HIS | B | 166 | -1.288 | -6.199 | 41.051 | 1.00 | 31.94 | N |
| ATOM | 2936 | CA | HIS | B | 166 | -0.993 | -7.512 | 41.592 | 1.00 | 31.52 | C |
| ATOM | 2937 | C | HIS | B | 166 | -0.032 | -8.235 | 40.655 | 1.00 | 29.94 | C |
| ATOM | 2938 | O | HIS | B | 166 | -0.431 | -8.661 | 39.590 | 1.00 | 28.26 | O |
| ATOM | 2939 | CB | HIS | B | 166 | -2.262 | -8.354 | 41.759 | 1.00 | 32.59 | C |
| ATOM | 2940 | CG | HIS | B | 166 | -3.267 | -7.761 | 42.692 | 1.00 | 34.51 | C |
| ATOM | 2941 | ND1 | HIS | B | 166 | -2.969 | -7.429 | 43.994 | 1.00 | 37.39 | N |
| ATOM | 2942 | CD2 | HIS | B | 166 | -4.574 | -7.448 | 42.514 | 1.00 | 36.57 | C |
| ATOM | 2943 | CE1 | HIS | B | 166 | -4.049 | -6.939 | 44.580 | 1.00 | 38.62 | C |
| ATOM | 2944 | NE2 | HIS | B | 166 | -5.038 | -6.940 | 43.704 | 1.00 | 36.23 | N |
| ATOM | 2945 | N | THR | B | 167 | 1.231 | -8.356 | 41.066 | 1.00 | 28.44 | N |
| ATOM | 2946 | CA | THR | B | 167 | 2.197 | -9.184 | 40.336 | 1.00 | 26.79 | C |
| ATOM | 2947 | C | THR | B | 167 | 2.349 | -10.489 | 41.085 | 1.00 | 24.35 | C |
| ATOM | 2948 | O | THR | B | 167 | 2.763 | -10.509 | 42.235 | 1.00 | 26.43 | O |
| ATOM | 2949 | CB | THR | B | 167 | 3.547 | -8.476 | 40.159 | 1.00 | 26.22 | C |
| ATOM | 2950 | OG1 | THR | B | 167 | 3.371 | -7.249 | 39.420 | 1.00 | 25.61 | O |
| ATOM | 2951 | CG2 | THR | B | 167 | 4.522 | -9.371 | 39.372 | 1.00 | 26.00 | C |
| ATOM | 2952 | N | PHE | B | 168 | 1.964 | -11.582 | 40.446 | 1.00 | 22.34 | N |
| ATOM | 2953 | CA | PHE | B | 168 | 1.866 | -12.860 | 41.087 | 1.00 | 22.74 | C |
| ATOM | 2954 | C | PHE | B | 168 | 3.232 | -13.558 | 41.153 | 1.00 | 22.97 | C |
| ATOM | 2955 | O | PHE | B | 168 | 4.057 | -13.359 | 40.286 | 1.00 | 23.79 | O |
| ATOM | 2956 | CB | PHE | B | 168 | 0.818 | -13.694 | 40.330 | 1.00 | 22.41 | C |
| ATOM | 2957 | CG | PHE | B | 168 | -0.550 | -13.067 | 40.392 | 1.00 | 22.20 | C |
| ATOM | 2958 | CD1 | PHE | B | 168 | -0.932 | -12.127 | 39.436 | 1.00 | 23.34 | C |
| ATOM | 2959 | CD2 | PHE | B | 168 | -1.416 | -13.382 | 41.413 | 1.00 | 23.61 | C |
| ATOM | 2960 | CE1 | PHE | B | 168 | -2.204 | -11.522 | 39.503 | 1.00 | 24.35 | C |
| ATOM | 2961 | CE2 | PHE | B | 168 | -2.675 | -12.778 | 41.512 | 1.00 | 22.73 | C |
| ATOM | 2962 | CZ | PHE | B | 168 | -3.050 | -11.827 | 40.560 | 1.00 | 21.83 | C |
| ATOM | 2963 | N | PRO | B | 169 | 3.458 | -14.381 | 42.183 | 1.00 | 22.37 | N |
| ATOM | 2964 | CA | PRO | B | 169 | 4.678 | -15.201 | 42.181 | 1.00 | 22.24 | C |
| ATOM | 2965 | C | PRO | B | 169 | 4.772 | -16.084 | 40.989 | 1.00 | 21.15 | C |
| ATOM | 2966 | O | PRO | B | 169 | 3.767 | -16.642 | 40.574 | 1.00 | 21.94 | O |
| ATOM | 2967 | CB | PRO | B | 169 | 4.555 | -16.045 | 43.454 | 1.00 | 21.70 | C |
| ATOM | 2968 | CG | PRO | B | 169 | 3.655 | -15.259 | 44.321 | 1.00 | 23.21 | C |
| ATOM | 2969 | CD | PRO | B | 169 | 2.674 | -14.509 | 43.413 | 1.00 | 22.71 | C |
| ATOM | 2970 | N | ALA | B | 170 | 5.982 | -16.268 | 40.485 | 1.00 | 19.58 | N |
| ATOM | 2971 | CA | ALA | B | 170 | 6.207 | -17.086 | 39.310 | 1.00 | 18.43 | C |
| ATOM | 2972 | C | ALA | B | 170 | 6.022 | -18.549 | 39.642 | 1.00 | 19.27 | C |
| ATOM | 2973 | O | ALA | B | 170 | 6.102 | -18.982 | 40.823 | 1.00 | 19.70 | O |
| ATOM | 2974 | CB | ALA | B | 170 | 7.562 | -16.819 | 38.718 | 1.00 | 19.27 | C |
| ATOM | 2975 | N | VAL | B | 171 | 5.701 | -19.290 | 38.608 | 1.00 | 20.06 | N |
| ATOM | 2976 | CA | VAL | B | 171 | 5.717 | -20.723 | 38.689 | 1.00 | 21.10 | C |
| ATOM | 2977 | C | VAL | B | 171 | 6.770 | -21.252 | 37.741 | 1.00 | 21.51 | C |
| ATOM | 2978 | O | VAL | B | 171 | 6.907 | -20.756 | 36.631 | 1.00 | 20.76 | O |
| ATOM | 2979 | CB | VAL | B | 171 | 4.348 | -21.347 | 38.366 | 1.00 | 22.20 | C |
| ATOM | 2980 | CG1 | VAL | B | 171 | 3.897 | -21.021 | 36.927 | 1.00 | 21.43 | C |

| ATOM | 2981 | CG2 | VAL | B | 171 | 4.431 | -22.884 | 38.564 | 1.00 | 22.69 | C |
|------|------|-----|-----|---|-----|--------|---------|--------|------|-------|---|
| ATOM | 2982 | N | LEU | B | 172 | 7.471 | -22.300 | 38.199 | 1.00 | 21.98 | N |
| ATOM | 2983 | CA | LEU | B | 172 | 8.497 | -22.974 | 37.448 | 1.00 | 22.75 | C |
| ATOM | 2984 | C | LEU | B | 172 | 7.926 | -24.256 | 36.808 | 1.00 | 23.21 | C |
| ATOM | 2985 | O | LEU | B | 172 | 7.319 | -25.082 | 37.478 | 1.00 | 23.98 | O |
| ATOM | 2986 | CB | LEU | B | 172 | 9.646 | -23.338 | 38.376 | 1.00 | 23.43 | C |
| ATOM | 2987 | CG | LEU | B | 172 | 10.776 | -24.151 | 37.752 | 1.00 | 22.54 | C |
| ATOM | 2988 | CD1 | LEU | B | 172 | 11.481 | -23.389 | 36.663 | 1.00 | 24.33 | C |
| ATOM | 2989 | CD2 | LEU | B | 172 | 11.728 | -24.571 | 38.849 | 1.00 | 25.89 | C |
| ATOM | 2990 | N | GLN | B | 173 | 8.141 | -24.413 | 35.516 | 1.00 | 23.85 | N |
| ATOM | 2991 | CA | GLN | B | 173 | 7.735 | -25.612 | 34.783 | 1.00 | 25.65 | C |
| ATOM | 2992 | C | GLN | B | 173 | 8.794 | -25.849 | 33.720 | 1.00 | 26.76 | C |
| ATOM | 2993 | O | GLN | B | 173 | 9.056 | -24.983 | 32.897 | 1.00 | 24.29 | O |
| ATOM | 2994 | CB | GLN | B | 173 | 6.358 | -25.385 | 34.159 | 1.00 | 27.38 | C |
| ATOM | 2995 | CG | GLN | B | 173 | 5.582 | -26.644 | 33.755 | 1.00 | 31.36 | C |
| ATOM | 2996 | CD | GLN | B | 173 | 4.415 | -26.360 | 32.788 | 1.00 | 33.70 | C |
| ATOM | 2997 | OE1 | GLN | B | 173 | 3.770 | -25.275 | 32.798 | 1.00 | 36.42 | O |
| ATOM | 2998 | NE2 | GLN | B | 173 | 4.138 | -27.336 | 31.943 | 1.00 | 33.47 | N |
| ATOM | 2999 | N | SER | B | 174 | 9.431 | -27.016 | 33.730 | 1.00 | 28.19 | N |
| ATOM | 3000 | CA | SER | B | 174 | 10.366 | -27.380 | 32.637 | 1.00 | 28.58 | C |
| ATOM | 3001 | C | SER | B | 174 | 11.474 | -26.345 | 32.442 | 1.00 | 27.84 | C |
| ATOM | 3002 | O | SER | B | 174 | 11.788 | -25.916 | 31.322 | 1.00 | 27.80 | O |
| ATOM | 3003 | CB | SER | B | 174 | 9.611 | -27.604 | 31.332 | 1.00 | 30.04 | C |
| ATOM | 3004 | OG | SER | B | 174 | 8.716 | -28.711 | 31.474 | 1.00 | 30.81 | O |
| ATOM | 3005 | N | ASP | B | 175 | 12.034 | -25.955 | 33.571 | 1.00 | 26.95 | N |
| ATOM | 3006 | CA | ASP | B | 175 | 13.111 | -24.983 | 33.653 | 1.00 | 25.96 | C |
| ATOM | 3007 | C | ASP | B | 175 | 12.779 | -23.635 | 33.090 | 1.00 | 21.99 | C |
| ATOM | 3008 | O | ASP | B | 175 | 13.666 | -22.911 | 32.719 | 1.00 | 20.41 | O |
| ATOM | 3009 | CB | ASP | B | 175 | 14.414 | -25.532 | 33.062 | 1.00 | 28.48 | C |
| ATOM | 3010 | CG | ASP | B | 175 | 15.116 | -26.493 | 34.019 | 1.00 | 29.53 | C |
| ATOM | 3011 | OD1 | ASP | B | 175 | 15.136 | -26.241 | 35.264 | 1.00 | 32.17 | O |
| ATOM | 3012 | OD2 | ASP | B | 175 | 15.651 | -27.476 | 33.497 | 1.00 | 32.92 | O |
| ATOM | 3013 | N | LEU | B | 176 | 11.493 | -23.290 | 33.039 | 1.00 | 21.16 | N |
| ATOM | 3014 | CA | LEU | B | 176 | 11.085 | -21.920 | 32.663 | 1.00 | 18.24 | C |
| ATOM | 3015 | C | LEU | B | 176 | 10.050 | -21.414 | 33.657 | 1.00 | 18.29 | C |
| ATOM | 3016 | O | LEU | B | 176 | 9.206 | -22.168 | 34.133 | 1.00 | 18.53 | O |
| ATOM | 3017 | CB | LEU | B | 176 | 10.500 | -21.854 | 31.250 | 1.00 | 18.35 | C |
| ATOM | 3018 | CG | LEU | B | 176 | 11.463 | -22.194 | 30.101 | 1.00 | 18.90 | C |
| ATOM | 3019 | CD1 | LEU | B | 176 | 10.742 | -22.161 | 28.810 | 1.00 | 19.74 | C |
| ATOM | 3020 | CD2 | LEU | B | 176 | 12.662 | -21.264 | 30.055 | 1.00 | 18.79 | C |
| ATOM | 3021 | N | TYR | B | 177 | 10.149 | -20.126 | 33.970 | 1.00 | 16.98 | N |
| ATOM | 3022 | CA | TYR | B | 177 | 9.210 | -19.449 | 34.861 | 1.00 | 16.88 | C |
| ATOM | 3023 | C | TYR | B | 177 | 8.164 | -18.654 | 34.071 | 1.00 | 14.92 | C |
| ATOM | 3024 | O | TYR | B | 177 | 8.432 | -18.154 | 32.981 | 1.00 | 15.09 | O |
| ATOM | 3025 | CB | TYR | B | 177 | 9.914 | -18.464 | 35.777 | 1.00 | 16.36 | C |
| ATOM | 3026 | CG | TYR | B | 177 | 10.762 | -19.074 | 36.841 | 1.00 | 16.84 | C |
| ATOM | 3027 | CD1 | TYR | B | 177 | 10.202 | -19.504 | 38.041 | 1.00 | 19.04 | C |
| ATOM | 3028 | CD2 | TYR | B | 177 | 12.110 | -19.284 | 36.617 | 1.00 | 18.79 | C |
| ATOM | 3029 | CE1 | TYR | B | 177 | 10.966 | -20.069 | 39.006 | 1.00 | 20.69 | C |
| ATOM | 3030 | CE2 | TYR | B | 177 | 12.904 | -19.866 | 37.586 | 1.00 | 20.09 | C |
| ATOM | 3031 | CZ | TYR | B | 177 | 12.322 | -20.266 | 38.771 | 1.00 | 20.31 | C |
| ATOM | 3032 | OH | TYR | B | 177 | 13.082 | -20.812 | 39.761 | 1.00 | 21.44 | O |
| ATOM | 3033 | N | THR | B | 178 | 6.978 | -18.550 | 34.664 | 1.00 | 15.95 | N |
| ATOM | 3034 | CA | THR | B | 178 | 5.892 | -17.726 | 34.163 | 1.00 | 16.04 | C |
| ATOM | 3035 | C | THR | B | 178 | 5.172 | -17.018 | 35.292 | 1.00 | 17.38 | C |
| ATOM | 3036 | O | THR | B | 178 | 4.882 | -17.597 | 36.348 | 1.00 | 16.58 | O |
| ATOM | 3037 | CB | THR | B | 178 | 4.868 | -18.552 | 33.386 | 1.00 | 17.33 | C |
| ATOM | 3038 | OG1 | THR | B | 178 | 5.499 | -19.192 | 32.290 | 1.00 | 17.77 | O |
| ATOM | 3039 | CG2 | THR | B | 178 | 3.774 | -17.725 | 32.848 | 1.00 | 17.64 | C |
| ATOM | 3040 | N | LEU | B | 179 | 4.896 | -15.746 | 35.077 | 1.00 | 17.88 | N |
| ATOM | 3041 | CA | LEU | B | 179 | 4.003 | -14.990 | 35.973 | 1.00 | 18.52 | C |
| ATOM | 3042 | C | LEU | B | 179 | 3.108 | -14.010 | 35.181 | 1.00 | 18.94 | C |
| ATOM | 3043 | O | LEU | B | 179 | 3.341 | -13.735 | 34.029 | 1.00 | 19.93 | O |
| ATOM | 3044 | CB | LEU | B | 179 | 4.786 | -14.243 | 37.057 | 1.00 | 20.01 | C |
| ATOM | 3045 | CG | LEU | B | 179 | 5.710 | -13.119 | 36.601 | 1.00 | 21.17 | C |

| ATOM | 3046 | CD1 | LEU | B | 179 | 4.947 | -11.869 | 36.146 | 1.00 | 22.74 | C |
|------|------|-----|-----|---|-----|-------|---------|--------|------|-------|---|
| ATOM | 3047 | CD2 | LEU | B | 179 | 6.734 | -12.767 | 37.715 | 1.00 | 21.49 | C |
| ATOM | 3048 | N | SER | B | 180 | 2.083 | -13.489 | 35.839 | 1.00 | 19.55 | N |
| ATOM | 3049 | CA | SER | B | 180 | 1.288 | -12.399 | 35.293 | 1.00 | 19.87 | C |
| ATOM | 3050 | C | SER | B | 180 | 1.226 | -11.299 | 36.298 | 1.00 | 21.33 | C |
| ATOM | 3051 | O | SER | B | 180 | 1.441 | -11.523 | 37.494 | 1.00 | 20.76 | O |
| ATOM | 3052 | CB | SER | B | 180 | -0.134 | -12.854 | 34.984 | 1.00 | 20.84 | C |
| ATOM | 3053 | OG | SER | B | 180 | -0.132 | -13.620 | 33.789 | 1.00 | 25.70 | O |
| ATOM | 3054 | N | SER | B | 181 | 0.883 | -10.122 | 35.802 | 1.00 | 22.19 | N |
| ATOM | 3055 | CA | SER | B | 181 | 0.622 | -8.981 | 36.646 | 1.00 | 24.81 | C |
| ATOM | 3056 | C | SER | B | 181 | -0.708 | -8.408 | 36.193 | 1.00 | 24.76 | C |
| ATOM | 3057 | O | SER | B | 181 | -0.940 | -8.315 | 34.997 | 1.00 | 25.17 | O |
| ATOM | 3058 | CB | SER | B | 181 | 1.706 | -7.945 | 36.475 | 1.00 | 24.18 | C |
| ATOM | 3059 | OG | SER | B | 181 | 1.553 | -6.928 | 37.447 | 1.00 | 26.50 | O |
| ATOM | 3060 | N | SER | B | 182 | -1.567 | -8.052 | 37.148 | 1.00 | 26.80 | N |
| ATOM | 3061 | CA | SER | B | 182 | -2.805 | -7.330 | 36.823 | 1.00 | 28.16 | C |
| ATOM | 3062 | C | SER | B | 182 | -2.668 | -5.925 | 37.340 | 1.00 | 29.95 | C |
| ATOM | 3063 | O | SER | B | 182 | -1.969 | -5.682 | 38.320 | 1.00 | 30.02 | O |
| ATOM | 3064 | CB | SER | B | 182 | -4.057 | -7.974 | 37.411 | 1.00 | 27.23 | C |
| ATOM | 3065 | OG | SER | B | 182 | -4.152 | -7.900 | 38.822 | 1.00 | 26.76 | O |
| ATOM | 3066 | N | VAL | B | 183 | -3.317 | -5.005 | 36.652 | 1.00 | 32.26 | N |
| ATOM | 3067 | CA | VAL | B | 183 | -3.570 | -3.692 | 37.205 | 1.00 | 34.55 | C |
| ATOM | 3068 | C | VAL | B | 183 | -5.056 | -3.357 | 37.040 | 1.00 | 34.98 | C |
| ATOM | 3069 | O | VAL | B | 183 | -5.634 | -3.601 | 35.987 | 1.00 | 34.84 | O |
| ATOM | 3070 | CB | VAL | B | 183 | -2.681 | -2.628 | 36.535 | 1.00 | 34.83 | C |
| ATOM | 3071 | CG1 | VAL | B | 183 | -2.917 | -2.557 | 35.025 | 1.00 | 35.04 | C |
| ATOM | 3072 | CG2 | VAL | B | 183 | -2.869 | -1.259 | 37.231 | 1.00 | 34.47 | C |
| ATOM | 3073 | N | THR | B | 184 | -5.654 | -2.807 | 38.095 | 1.00 | 36.39 | N |
| ATOM | 3074 | CA | THR | B | 184 | -7.035 | -2.321 | 38.079 | 1.00 | 37.03 | C |
| ATOM | 3075 | C | THR | B | 184 | -7.040 | -0.795 | 38.144 | 1.00 | 38.26 | C |
| ATOM | 3076 | O | THR | B | 184 | -6.441 | -0.201 | 39.043 | 1.00 | 38.46 | O |
| ATOM | 3077 | CB | THR | B | 184 | -7.813 | -2.876 | 39.284 | 1.00 | 37.72 | C |
| ATOM | 3078 | OG1 | THR | B | 184 | -7.843 | -4.305 | 39.190 | 1.00 | 37.22 | O |
| ATOM | 3079 | CG2 | THR | B | 184 | -9.251 | -2.354 | 39.306 | 1.00 | 37.88 | C |
| ATOM | 3080 | N | VAL | B | 185 | -7.700 | -0.158 | 37.185 | 1.00 | 39.42 | N |
| ATOM | 3081 | CA | VAL | B | 185 | -7.751 | 1.301 | 37.155 | 1.00 | 40.74 | C |
| ATOM | 3082 | C | VAL | B | 185 | -9.181 | 1.791 | 36.924 | 1.00 | 41.99 | C |
| ATOM | 3083 | O | VAL | B | 185 | -10.057 | 1.007 | 36.542 | 1.00 | 40.99 | O |
| ATOM | 3084 | CB | VAL | B | 185 | -6.815 | 1.860 | 36.062 | 1.00 | 40.81 | C |
| ATOM | 3085 | CG1 | VAL | B | 185 | -5.398 | 1.331 | 36.267 | 1.00 | 41.63 | C |
| ATOM | 3086 | CG2 | VAL | B | 185 | -7.333 | 1.509 | 34.667 | 1.00 | 40.22 | C |
| ATOM | 3087 | N | PRO | B | 186 | -9.440 | 3.085 | 37.183 | 1.00 | 43.34 | N |
| ATOM | 3088 | CA | PRO | B | 186 | -10.741 | 3.610 | 36.759 | 1.00 | 44.28 | C |
| ATOM | 3089 | C | PRO | B | 186 | -10.940 | 3.510 | 35.239 | 1.00 | 45.53 | C |
| ATOM | 3090 | O | PRO | B | 186 | -10.012 | 3.800 | 34.462 | 1.00 | 44.80 | O |
| ATOM | 3091 | CB | PRO | B | 186 | -10.696 | 5.082 | 37.207 | 1.00 | 44.22 | C |
| ATOM | 3092 | CG | PRO | B | 186 | -9.638 | 5.140 | 38.260 | 1.00 | 43.89 | C |
| ATOM | 3093 | CD | PRO | B | 186 | -8.630 | 4.094 | 37.887 | 1.00 | 43.19 | C |
| ATOM | 3094 | N | SER | B | 187 | -12.139 | 3.101 | 34.822 | 1.00 | 46.76 | N |
| ATOM | 3095 | CA | SER | B | 187 | -12.487 | 3.056 | 33.398 | 1.00 | 47.61 | C |
| ATOM | 3096 | C | SER | B | 187 | -12.326 | 4.433 | 32.752 | 1.00 | 48.52 | C |
| ATOM | 3097 | O | SER | B | 187 | -11.970 | 4.545 | 31.576 | 1.00 | 47.51 | O |
| ATOM | 3098 | CB | SER | B | 187 | -13.924 | 2.576 | 33.203 | 1.00 | 48.51 | C |
| ATOM | 3099 | OG | SER | B | 187 | -14.158 | 1.353 | 33.880 | 1.00 | 49.70 | O |
| ATOM | 3100 | N | SER | B | 188 | -12.601 | 5.477 | 33.539 | 1.00 | 49.51 | N |
| ATOM | 3101 | CA | SER | B | 188 | -12.395 | 6.862 | 33.108 | 1.00 | 49.73 | C |
| ATOM | 3102 | C | SER | B | 188 | -10.920 | 7.149 | 32.768 | 1.00 | 49.94 | C |
| ATOM | 3103 | O | SER | B | 188 | -10.623 | 8.127 | 32.076 | 1.00 | 50.64 | O |
| ATOM | 3104 | CB | SER | B | 188 | -12.895 | 7.841 | 34.178 | 1.00 | 49.82 | C |
| ATOM | 3105 | OG | SER | B | 188 | -12.085 | 7.819 | 35.345 | 1.00 | 51.13 | O |
| ATOM | 3106 | N | THR | B | 189 | -10.011 | 6.286 | 33.244 | 1.00 | 49.25 | N |
| ATOM | 3107 | CA | THR | B | 189 | -8.576 | 6.411 | 32.964 | 1.00 | 48.08 | C |
| ATOM | 3108 | C | THR | B | 189 | -8.044 | 5.581 | 31.779 | 1.00 | 46.70 | C |
| ATOM | 3109 | O | THR | B | 189 | -6.986 | 5.910 | 31.256 | 1.00 | 46.75 | O |
| ATOM | 3110 | CB | THR | B | 189 | -7.709 | 6.121 | 34.228 | 1.00 | 48.30 | C |

| ATOM | 3111 | OG1 | THR | B | 189 | -8.059 | 4.853 | 34.808 | 1.00 | 47.96 | O |
|------|------|-----|-----|---|-----|--------|-------|--------|------|-------|---|
| ATOM | 3112 | CG2 | THR | B | 189 | -7.891 | 7.224 | 35.267 | 1.00 | 48.33 | C |
| ATOM | 3113 | N | TRP | B | 190 | -8.754 | 4.523 | 31.366 | 1.00 | 44.48 | N |
| ATOM | 3114 | CA | TRP | B | 190 | -8.310 | 3.657 | 30.236 | 1.00 | 41.74 | C |
| ATOM | 3115 | C | TRP | B | 190 | -9.478 | 3.285 | 29.266 | 1.00 | 36.82 | C |
| ATOM | 3116 | O | TRP | B | 190 | -10.570 | 3.026 | 29.755 | 1.00 | 39.01 | O |
| ATOM | 3117 | CB | TRP | B | 190 | -7.660 | 2.373 | 30.799 | 1.00 | 42.83 | C |
| ATOM | 3118 | CG | TRP | B | 190 | -7.079 | 1.514 | 29.711 | 1.00 | 44.16 | C |
| ATOM | 3119 | CD1 | TRP | B | 190 | -5.835 | 1.620 | 29.160 | 1.00 | 44.24 | C |
| ATOM | 3120 | CD2 | TRP | B | 190 | -7.743 | 0.461 | 28.999 | 1.00 | 44.97 | C |
| ATOM | 3121 | NE1 | TRP | B | 190 | -5.681 | 0.693 | 28.162 | 1.00 | 43.79 | N |
| ATOM | 3122 | CE2 | TRP | B | 190 | -6.839 | -0.028 | 28.039 | 1.00 | 44.11 | C |
| ATOM | 3123 | CE3 | TRP | B | 190 | -9.018 | -0.120 | 29.085 | 1.00 | 45.12 | C |
| ATOM | 3124 | CZ2 | TRP | B | 190 | -7.157 | -1.079 | 27.177 | 1.00 | 44.60 | C |
| ATOM | 3125 | CZ3 | TRP | B | 190 | -9.336 | -1.162 | 28.213 | 1.00 | 44.65 | C |
| ATOM | 3126 | CH2 | TRP | B | 190 | -8.407 | -1.632 | 27.280 | 1.00 | 44.36 | C |
| ATOM | 3127 | N | PRO | B | 191 | -9.245 | 3.194 | 27.902 | 1.00 | 27.61 | N |
| ATOM | 3128 | CA | PRO | B | 191 | -8.071 | 3.349 | 27.115 | 1.00 | 27.96 | C |
| ATOM | 3129 | C | PRO | B | 191 | -7.619 | 4.748 | 26.774 | 1.00 | 28.10 | C |
| ATOM | 3130 | O | PRO | B | 191 | -7.011 | 4.936 | 25.724 | 1.00 | 29.59 | O |
| ATOM | 3131 | CB | PRO | B | 191 | -8.429 | 2.633 | 25.831 | 1.00 | 27.47 | C |
| ATOM | 3132 | CG | PRO | B | 191 | -9.857 | 2.723 | 25.744 | 1.00 | 27.34 | C |
| ATOM | 3133 | CD | PRO | B | 191 | -10.369 | 2.724 | 27.071 | 1.00 | 27.90 | C |
| ATOM | 3134 | N | SER | B | 192 | -7.868 | 5.715 | 27.630 | 1.00 | 30.17 | N |
| ATOM | 3135 | CA | SER | B | 192 | -7.850 | 7.085 | 27.174 | 1.00 | 30.02 | C |
| ATOM | 3136 | C | SER | B | 192 | -6.709 | 7.885 | 27.704 | 1.00 | 31.19 | C |
| ATOM | 3137 | O | SER | B | 192 | -6.280 | 8.860 | 27.073 | 1.00 | 32.36 | O |
| ATOM | 3138 | CB | SER | B | 192 | -9.068 | 7.775 | 27.767 | 1.00 | 29.66 | C |
| ATOM | 3139 | OG | SER | B | 192 | -8.854 | 8.021 | 29.162 | 1.00 | 31.80 | O |
| ATOM | 3140 | N | GLU | B | 193 | -6.106 | 7.446 | 28.796 | 1.00 | 31.92 | N |
| ATOM | 3141 | CA | GLU | B | 193 | -4.665 | 7.383 | 28.897 | 1.00 | 31.31 | C |
| ATOM | 3142 | C | GLU | B | 193 | -4.111 | 6.006 | 29.003 | 1.00 | 32.44 | C |
| ATOM | 3143 | O | GLU | B | 193 | -4.783 | 5.048 | 29.495 | 1.00 | 31.57 | O |
| ATOM | 3144 | CB | GLU | B | 193 | -4.322 | 8.133 | 30.173 | 1.00 | 33.03 | C |
| ATOM | 3145 | CG | GLU | B | 193 | -4.587 | 9.631 | 30.134 | 1.00 | 36.05 | C |
| ATOM | 3146 | CD | GLU | B | 193 | -5.393 | 10.086 | 31.322 | 1.00 | 39.52 | C |
| ATOM | 3147 | OE1 | GLU | B | 193 | -4.874 | 10.913 | 32.146 | 1.00 | 41.27 | O |
| ATOM | 3148 | OE2 | GLU | B | 193 | -6.573 | 9.640 | 31.417 | 1.00 | 40.03 | O |
| ATOM | 3149 | N | THR | B | 194 | -2.864 | 5.908 | 28.592 | 1.00 | 31.00 | N |
| ATOM | 3150 | CA | THR | B | 194 | -2.372 | 4.633 | 28.142 | 1.00 | 31.57 | C |
| ATOM | 3151 | C | THR | B | 194 | -1.984 | 3.860 | 29.382 | 1.00 | 31.38 | C |
| ATOM | 3152 | O | THR | B | 194 | -1.816 | 4.425 | 30.434 | 1.00 | 29.79 | O |
| ATOM | 3153 | CB | THR | B | 194 | -1.181 | 4.818 | 27.272 | 1.00 | 32.07 | C |
| ATOM | 3154 | OG1 | THR | B | 194 | -0.184 | 5.534 | 28.016 | 1.00 | 34.14 | O |
| ATOM | 3155 | CG2 | THR | B | 194 | -1.572 | 5.594 | 26.076 | 1.00 | 32.58 | C |
| ATOM | 3156 | N | VAL | B | 195 | -1.944 | 2.531 | 29.350 | 1.00 | 36.38 | N |
| ATOM | 3157 | CA | VAL | B | 195 | -1.419 | 1.764 | 30.464 | 1.00 | 37.83 | C |
| ATOM | 3158 | C | VAL | B | 195 | -0.401 | 0.853 | 29.816 | 1.00 | 37.42 | C |
| ATOM | 3159 | O | VAL | B | 195 | -0.705 | 0.210 | 28.813 | 1.00 | 37.56 | O |
| ATOM | 3160 | CB | VAL | B | 195 | -2.527 | 0.976 | 31.159 | 1.00 | 39.76 | C |
| ATOM | 3161 | CG1 | VAL | B | 195 | -1.947 | -0.074 | 32.130 | 1.00 | 41.13 | C |
| ATOM | 3162 | CG2 | VAL | B | 195 | -3.470 | 1.923 | 31.901 | 1.00 | 40.06 | C |
| ATOM | 3163 | N | THR | B | 196 | 0.814 | 0.853 | 30.358 | 1.00 | 36.95 | N |
| ATOM | 3164 | CA | THR | B | 196 | 1.920 | 0.067 | 29.812 | 1.00 | 36.37 | C |
| ATOM | 3165 | C | THR | B | 196 | 2.591 | -0.791 | 30.889 | 1.00 | 35.06 | C |
| ATOM | 3166 | O | THR | B | 196 | 2.872 | -0.295 | 31.985 | 1.00 | 33.90 | O |
| ATOM | 3167 | CB | THR | B | 196 | 2.975 | 0.993 | 29.202 | 1.00 | 36.83 | C |
| ATOM | 3168 | OG1 | THR | B | 196 | 2.374 | 1.735 | 28.140 | 1.00 | 37.29 | O |
| ATOM | 3169 | CG2 | THR | B | 196 | 4.147 | 0.186 | 28.634 | 1.00 | 36.74 | C |
| ATOM | 3170 | N | CYS | B | 197 | 2.846 | -2.072 | 30.589 | 1.00 | 33.82 | N |
| ATOM | 3171 | CA A | CYS | B | 197 | 3.659 | -2.912 | 31.488 | 0.50 | 32.90 | C |
| ATOM | 3172 | CA B | CYS | B | 197 | 3.647 | -2.907 | 31.499 | 0.50 | 33.26 | C |
| ATOM | 3173 | C | CYS | B | 197 | 5.107 | -2.914 | 31.013 | 1.00 | 32.26 | C |
| ATOM | 3174 | O | CYS | B | 197 | 5.381 | -2.974 | 29.813 | 1.00 | 32.59 | O |
| ATOM | 3175 | CB A | CYS | B | 197 | 3.111 | -4.346 | 31.621 | 0.50 | 32.48 | C |

```
ATOM   3176  CB BCYS B 197       3.035  -4.323  31.702  0.50 33.16           C
ATOM   3177  SG ACYS B 197       3.299  -5.430  30.207  0.50 32.38           S
ATOM   3178  SG BCYS B 197       3.621  -5.663  30.669  0.50 34.36           S
ATOM   3179  N   ASN B 198       6.035  -2.786  31.957  1.00 31.29           N
ATOM   3180  CA  ASN B 198       7.454  -2.778  31.664  1.00 29.26           C
ATOM   3181  C   ASN B 198       8.050  -3.972  32.391  1.00 27.02           C
ATOM   3182  O   ASN B 198       7.796  -4.167  33.594  1.00 26.04           O
ATOM   3183  CB  ASN B 198       8.146  -1.495  32.165  1.00 29.98           C
ATOM   3184  CG  ASN B 198       7.221  -0.278  32.159  1.00 29.26           C
ATOM   3185  OD1 ASN B 198       6.706   0.120  33.195  1.00 29.86           O
ATOM   3186  ND2 ASN B 198       6.957   0.253  30.987  1.00 32.25           N
ATOM   3187  N   VAL B 199       8.851  -4.743  31.673  1.00 25.41           N
ATOM   3188  CA  VAL B 199       9.407  -5.988  32.216  1.00 24.47           C
ATOM   3189  C   VAL B 199      10.902  -6.037  31.982  1.00 23.69           C
ATOM   3190  O   VAL B 199      11.346  -6.154  30.852  1.00 23.90           O
ATOM   3191  CB  VAL B 199       8.773  -7.227  31.583  1.00 24.92           C
ATOM   3192  CG1 VAL B 199       9.356  -8.482  32.227  1.00 22.81           C
ATOM   3193  CG2 VAL B 199       7.290  -7.191  31.736  1.00 23.91           C
ATOM   3194  N   ALA B 200      11.666  -5.936  33.067  1.00 21.97           N
ATOM   3195  CA  ALA B 200      13.116  -6.127  33.013  1.00 21.76           C
ATOM   3196  C   ALA B 200      13.511  -7.529  33.468  1.00 20.73           C
ATOM   3197  O   ALA B 200      13.015  -8.021  34.479  1.00 20.80           O
ATOM   3198  CB  ALA B 200      13.815  -5.106  33.887  1.00 22.87           C
ATOM   3199  N   HIS B 201      14.431  -8.135  32.730  1.00 20.63           N
ATOM   3200  CA  HIS B 201      15.018  -9.427  33.115  1.00 20.42           C
ATOM   3201  C   HIS B 201      16.551  -9.275  33.175  1.00 20.07           C
ATOM   3202  O   HIS B 201      17.240  -9.377  32.159  1.00 19.89           O
ATOM   3203  CB  HIS B 201      14.586 -10.490  32.114  1.00 18.92           C
ATOM   3204  CG  HIS B 201      15.006 -11.875  32.493  1.00 18.01           C
ATOM   3205  ND1 HIS B 201      15.663 -12.710  31.622  1.00 17.10           N
ATOM   3206  CD2 HIS B 201      14.956 -12.530  33.678  1.00 17.13           C
ATOM   3207  CE1 HIS B 201      15.928 -13.851  32.226  1.00 19.78           C
ATOM   3208  NE2 HIS B 201      15.490 -13.778  33.472  1.00 17.98           N
ATOM   3209  N   PRO B 202      17.082  -9.008  34.373  1.00 20.38           N
ATOM   3210  CA  PRO B 202      18.497  -8.742  34.531  1.00 21.83           C
ATOM   3211  C   PRO B 202      19.430  -9.805  33.942  1.00 20.71           C
ATOM   3212  O   PRO B 202      20.434  -9.467  33.309  1.00 20.07           O
ATOM   3213  CB  PRO B 202      18.654  -8.593  36.041  1.00 23.31           C
ATOM   3214  CG  PRO B 202      17.343  -8.120  36.504  1.00 23.29           C
ATOM   3215  CD  PRO B 202      16.356  -8.854  35.646  1.00 21.94           C
ATOM   3216  N   ALA B 203      19.122 -11.091  34.126  1.00 19.05           N
ATOM   3217  CA  ALA B 203      20.022 -12.107  33.609  1.00 19.11           C
ATOM   3218  C   ALA B 203      20.252 -12.058  32.101  1.00 19.05           C
ATOM   3219  O   ALA B 203      21.321 -12.440  31.629  1.00 20.73           O
ATOM   3220  CB  ALA B 203      19.568 -13.488  34.044  1.00 19.54           C
ATOM   3221  N   SER B 204      19.262 -11.624  31.336  1.00 18.57           N
ATOM   3222  CA  SER B 204      19.371 -11.578  29.876  1.00 18.62           C
ATOM   3223  C   SER B 204      19.659 -10.159  29.404  1.00 20.57           C
ATOM   3224  O   SER B 204      19.800  -9.917  28.210  1.00 23.35           O
ATOM   3225  CB  SER B 204      18.100 -12.119  29.185  1.00 18.22           C
ATOM   3226  OG  SER B 204      16.907 -11.440  29.573  1.00 19.87           O
ATOM   3227  N   SER B 205      19.777  -9.242  30.340  1.00 20.92           N
ATOM   3228  CA  SER B 205      19.983  -7.822  30.013  1.00 22.75           C
ATOM   3229  C   SER B 205      18.877  -7.243  29.113  1.00 23.64           C
ATOM   3230  O   SER B 205      19.137  -6.422  28.216  1.00 24.17           O
ATOM   3231  CB  SER B 205      21.378  -7.607  29.405  1.00 23.75           C
ATOM   3232  OG  SER B 205      22.395  -8.111  30.251  1.00 30.30           O
ATOM   3233  N   THR B 206      17.630  -7.647  29.345  1.00 22.26           N
ATOM   3234  CA  THR B 206      16.522  -7.224  28.459  1.00 23.15           C
ATOM   3235  C   THR B 206      15.509  -6.410  29.244  1.00 24.16           C
ATOM   3236  O   THR B 206      15.353  -6.601  30.447  1.00 23.17           O
ATOM   3237  CB  THR B 206      15.823  -8.396  27.788  1.00 23.20           C
ATOM   3238  OG1 THR B 206      15.373  -9.295  28.790  1.00 22.53           O
ATOM   3239  CG2 THR B 206      16.738  -9.121  26.880  1.00 21.36           C
ATOM   3240  N   LYS B 207      14.892  -5.449  28.567  1.00 25.53           N
```

```
ATOM   3241  CA   LYS B 207    13.766  -4.703  29.114  1.00 29.08         C
ATOM   3242  C    LYS B 207    12.739  -4.544  28.004  1.00 29.90         C
ATOM   3243  O    LYS B 207    13.081  -4.214  26.868  1.00 28.80         O
ATOM   3244  CB   LYS B 207    14.214  -3.340  29.657  1.00 31.44         C
ATOM   3245  CG   LYS B 207    13.106  -2.260  29.772  1.00 34.75         C
ATOM   3246  CD   LYS B 207    12.934  -1.449  28.438  1.00 36.41         C
ATOM   3247  CE   LYS B 207    11.471  -1.139  28.091  1.00 37.64         C
ATOM   3248  NZ   LYS B 207    11.284  -0.707  26.645  1.00 38.52         N
ATOM   3249  N    VAL B 208    11.480  -4.773  28.308  1.00 31.13         N
ATOM   3250  CA   VAL B 208    10.476  -4.600  27.280  1.00 34.13         C
ATOM   3251  C    VAL B 208     9.243  -3.926  27.845  1.00 35.84         C
ATOM   3252  O    VAL B 208     8.916  -4.085  29.024  1.00 34.60         O
ATOM   3253  CB   VAL B 208    10.142  -5.950  26.642  1.00 35.19         C
ATOM   3254  CG1  VAL B 208     9.471  -6.832  27.626  1.00 35.05         C
ATOM   3255  CG2  VAL B 208     9.292  -5.779  25.415  1.00 37.58         C
ATOM   3256  N    ASP B 209     8.588  -3.133  26.999  1.00 37.89         N
ATOM   3257  CA   ASP B 209     7.342  -2.484  27.362  1.00 39.32         C
ATOM   3258  C    ASP B 209     6.259  -3.025  26.446  1.00 39.55         C
ATOM   3259  O    ASP B 209     6.509  -3.279  25.276  1.00 39.67         O
ATOM   3260  CB   ASP B 209     7.436  -0.971  27.194  1.00 40.27         C
ATOM   3261  CG   ASP B 209     8.460  -0.327  28.122  1.00 41.80         C
ATOM   3262  OD1  ASP B 209     8.593  -0.725  29.304  1.00 41.90         O
ATOM   3263  OD2  ASP B 209     9.143   0.611  27.663  1.00 44.89         O
ATOM   3264  N    LYS B 210     5.058  -3.186  26.986  1.00 40.05         N
ATOM   3265  CA   LYS B 210     3.910  -3.583  26.194  1.00 40.06         C
ATOM   3266  C    LYS B 210     2.726  -2.733  26.554  1.00 40.05         C
ATOM   3267  O    LYS B 210     2.265  -2.781  27.692  1.00 37.20         O
ATOM   3268  CB   LYS B 210     3.572  -5.053  26.439  1.00 40.39         C
ATOM   3269  CG   LYS B 210     4.325  -5.983  25.521  1.00 41.00         C
ATOM   3270  CD   LYS B 210     3.673  -6.004  24.145  1.00 42.36         C
ATOM   3271  CE   LYS B 210     4.555  -6.669  23.132  1.00 42.19         C
ATOM   3272  NZ   LYS B 210     3.806  -6.847  21.853  1.00 42.05         N
ATOM   3273  N    LYS B 211     2.245  -1.948  25.584  1.00 41.51         N
ATOM   3274  CA   LYS B 211     1.043  -1.134  25.780  1.00 42.02         C
ATOM   3275  C    LYS B 211    -0.176  -2.036  25.699  1.00 41.63         C
ATOM   3276  O    LYS B 211    -0.304  -2.838  24.774  1.00 41.09         O
ATOM   3277  CB   LYS B 211     0.938   0.010  24.745  1.00 42.99         C
ATOM   3278  CG   LYS B 211    -0.140   1.071  25.093  1.00 44.09         C
ATOM   3279  CD   LYS B 211    -0.057   2.361  24.211  1.00 45.43         C
ATOM   3280  CE   LYS B 211    -0.764   2.180  22.846  1.00 46.45         C
ATOM   3281  NZ   LYS B 211    -0.873   3.437  21.995  1.00 45.77         N
ATOM   3282  N    ILE B 212    -1.063  -1.920  26.683  1.00 41.79         N
ATOM   3283  CA   ILE B 212    -2.321  -2.671  26.658  1.00 42.18         C
ATOM   3284  C    ILE B 212    -3.266  -1.903  25.736  1.00 42.99         C
ATOM   3285  O    ILE B 212    -3.676  -0.784  26.065  1.00 42.68         O
ATOM   3286  CB   ILE B 212    -2.959  -2.808  28.073  1.00 42.28         C
ATOM   3287  CG1  ILE B 212    -1.920  -3.271  29.116  1.00 42.07         C
ATOM   3288  CG2  ILE B 212    -4.177  -3.760  28.047  1.00 42.47         C
ATOM   3289  CD1  ILE B 212    -1.135  -4.487  28.740  1.00 41.09         C
ATOM   3290  N    VAL B 213    -3.577  -2.480  24.579  1.00 44.32         N
ATOM   3291  CA   VAL B 213    -4.540  -1.864  23.649  1.00 45.53         C
ATOM   3292  C    VAL B 213    -5.783  -2.742  23.424  1.00 46.20         C
ATOM   3293  O    VAL B 213    -5.666  -3.971  23.310  1.00 46.08         O
ATOM   3294  CB   VAL B 213    -3.888  -1.489  22.287  1.00 45.67         C
ATOM   3295  CG1  VAL B 213    -2.554  -0.805  22.515  1.00 46.19         C
ATOM   3296  CG2  VAL B 213    -3.730  -2.694  21.362  1.00 45.93         C
ATOM   3297  N    PRO B 214    -6.977  -2.117  23.360  1.00 47.17         N
ATOM   3298  CA   PRO B 214    -8.221  -2.842  23.035  1.00 47.68         C
ATOM   3299  C    PRO B 214    -8.104  -3.813  21.849  1.00 48.09         C
ATOM   3300  O    PRO B 214    -7.390  -3.530  20.877  1.00 48.32         O
ATOM   3301  CB   PRO B 214    -9.199  -1.715  22.693  1.00 47.89         C
ATOM   3302  CG   PRO B 214    -8.723  -0.535  23.499  1.00 47.42         C
ATOM   3303  CD   PRO B 214    -7.223  -0.679  23.615  1.00 47.03         C
TER    3304       PRO B 214
ATOM   3305  N    ASP C   1     9.758 -24.438 -17.925  1.00 35.41         N
```

84

```
ATOM   3306  CA   ASP C  1     10.414 -23.178 -17.492  1.00 34.08           C
ATOM   3307  C    ASP C  1     11.747 -22.956 -18.201  1.00 32.20           C
ATOM   3308  O    ASP C  1     12.588 -23.861 -18.254  1.00 33.10           O
ATOM   3309  CB   ASP C  1     10.687 -23.197 -15.990  1.00 35.29           C
ATOM   3310  CG   ASP C  1      9.416 -23.211 -15.157  1.00 35.73           C
ATOM   3311  OD1  ASP C  1      8.359 -23.593 -15.699  1.00 36.27           O
ATOM   3312  OD2  ASP C  1      9.504 -22.817 -13.964  1.00 37.42           O
ATOM   3313  N    ILE C  2     11.956 -21.743 -18.701  1.00 29.16           N
ATOM   3314  CA   ILE C  2     13.265 -21.389 -19.211  1.00 28.26           C
ATOM   3315  C    ILE C  2     14.284 -21.379 -18.070  1.00 26.32           C
ATOM   3316  O    ILE C  2     14.081 -20.733 -17.011  1.00 27.18           O
ATOM   3317  CB   ILE C  2     13.240 -20.030 -19.959  1.00 28.17           C
ATOM   3318  CG1  ILE C  2     12.423 -20.193 -21.251  1.00 28.99           C
ATOM   3319  CG2  ILE C  2     14.684 -19.543 -20.221  1.00 28.22           C
ATOM   3320  CD1  ILE C  2     11.999 -18.926 -21.922  1.00 29.64           C
ATOM   3321  N    VAL C  3     15.389 -22.064 -18.294  1.00 24.18           N
ATOM   3322  CA   VAL C  3     16.521 -22.043 -17.376  1.00 23.92           C
ATOM   3323  C    VAL C  3     17.591 -21.030 -17.803  1.00 23.92           C
ATOM   3324  O    VAL C  3     18.155 -21.104 -18.900  1.00 22.38           O
ATOM   3325  CB   VAL C  3     17.177 -23.418 -17.255  1.00 24.89           C
ATOM   3326  CG1  VAL C  3     18.407 -23.349 -16.401  1.00 24.88           C
ATOM   3327  CG2  VAL C  3     16.157 -24.417 -16.676  1.00 26.26           C
ATOM   3328  N    MET C  4     17.853 -20.068 -16.916  1.00 24.18           N
ATOM   3329  CA   MET C  4     18.885 -19.072 -17.136  1.00 21.91           C
ATOM   3330  C    MET C  4     20.113 -19.382 -16.311  1.00 23.02           C
ATOM   3331  O    MET C  4     20.064 -19.403 -15.070  1.00 22.17           O
ATOM   3332  CB   MET C  4     18.354 -17.672 -16.791  1.00 21.81           C
ATOM   3333  CG   MET C  4     17.068 -17.303 -17.481  1.00 22.27           C
ATOM   3334  SD   MET C  4     17.133 -17.017 -19.253  1.00 23.56           S
ATOM   3335  CE   MET C  4     18.060 -15.472 -19.317  1.00 22.40           C
ATOM   3336  N    THR C  5     21.228 -19.653 -16.994  1.00 21.89           N
ATOM   3337  CA   THR C  5     22.448 -20.107 -16.328  1.00 21.00           C
ATOM   3338  C    THR C  5     23.558 -19.069 -16.248  1.00 20.76           C
ATOM   3339  O    THR C  5     24.079 -18.617 -17.282  1.00 21.84           O
ATOM   3340  CB   THR C  5     22.989 -21.371 -17.036  1.00 22.49           C
ATOM   3341  OG1  THR C  5     21.924 -22.330 -17.100  1.00 23.72           O
ATOM   3342  CG2  THR C  5     24.181 -21.966 -16.284  1.00 24.09           C
ATOM   3343  N    GLN C  6     23.905 -18.722 -15.005  1.00 22.16           N
ATOM   3344  CA   GLN C  6     25.028 -17.865 -14.680  1.00 22.48           C
ATOM   3345  C    GLN C  6     25.969 -18.459 -13.653  1.00 23.44           C
ATOM   3346  O    GLN C  6     25.520 -19.097 -12.720  1.00 23.76           O
ATOM   3347  CB   GLN C  6     24.488 -16.581 -14.042  1.00 23.37           C
ATOM   3348  CG   GLN C  6     23.640 -15.769 -14.908  1.00 22.61           C
ATOM   3349  CD   GLN C  6     23.302 -14.445 -14.246  1.00 21.20           C
ATOM   3350  OE1  GLN C  6     22.278 -14.331 -13.591  1.00 17.98           O
ATOM   3351  NE2  GLN C  6     24.188 -13.460 -14.399  1.00 19.45           N
ATOM   3352  N    ALA C  7     27.264 -18.186 -13.766  1.00 23.25           N
ATOM   3353  CA   ALA C  7     28.200 -18.515 -12.702  1.00 24.17           C
ATOM   3354  C    ALA C  7     27.968 -17.620 -11.509  1.00 24.91           C
ATOM   3355  O    ALA C  7     27.645 -16.423 -11.651  1.00 25.09           O
ATOM   3356  CB   ALA C  7     29.647 -18.376 -13.182  1.00 24.65           C
ATOM   3357  N    ALA C  8     28.175 -18.166 -10.326  1.00 22.89           N
ATOM   3358  CA   ALA C  8     27.972 -17.397  -9.089  1.00 22.83           C
ATOM   3359  C    ALA C  8     28.979 -16.255  -8.893  1.00 23.73           C
ATOM   3360  O    ALA C  8     28.632 -15.251  -8.272  1.00 23.16           O
ATOM   3361  CB   ALA C  8     28.027 -18.342  -7.868  1.00 22.68           C
ATOM   3362  N    PHE C  9     30.234 -16.429  -9.347  1.00 26.13           N
ATOM   3363  CA   PHE C  9     31.272 -15.384  -9.218  1.00 27.92           C
ATOM   3364  C    PHE C  9     32.011 -15.041 -10.515  1.00 28.46           C
ATOM   3365  O    PHE C  9     32.143 -15.859 -11.434  1.00 29.42           O
ATOM   3366  CB   PHE C  9     32.324 -15.766  -8.176  1.00 29.34           C
ATOM   3367  CG   PHE C  9     31.752 -16.141  -6.850  1.00 31.96           C
ATOM   3368  CD1  PHE C  9     31.486 -15.166  -5.900  1.00 33.31           C
ATOM   3369  CD2  PHE C  9     31.476 -17.471  -6.550  1.00 33.14           C
ATOM   3370  CE1  PHE C  9     30.945 -15.501  -4.676  1.00 33.95           C
```

| ATOM | 3371 | CE2 | PHE | C | 9  | 30.930 | -17.816 | -5.315  | 1.00 | 33.11 | C |
|------|------|-----|-----|---|----|--------|---------|---------|------|-------|---|
| ATOM | 3372 | CZ  | PHE | C | 9  | 30.670 | -16.822 | -4.379  | 1.00 | 33.46 | C |
| ATOM | 3373 | N   | SER | C | 10 | 32.455 | -13.780 | -10.581 | 1.00 | 29.97 | N |
| ATOM | 3374 | CA  | SER | C | 10 | 33.347 | -13.279 | -11.632 | 1.00 | 30.24 | C |
| ATOM | 3375 | C   | SER | C | 10 | 34.776 | -13.650 | -11.223 | 1.00 | 31.06 | C |
| ATOM | 3376 | O   | SER | C | 10 | 35.044 | -13.835 | -10.026 | 1.00 | 31.73 | O |
| ATOM | 3377 | CB  | SER | C | 10 | 33.249 | -11.736 | -11.717 | 1.00 | 29.05 | C |
| ATOM | 3378 | OG  | SER | C | 10 | 34.085 | -11.095 | -10.724 | 1.00 | 26.49 | O |
| ATOM | 3379 | N   | ASN | C | 11 | 35.704 | -13.740 | -12.171 | 1.00 | 32.21 | N |
| ATOM | 3380 | CA  | ASN | C | 11 | 37.123 | -13.649 | -11.773 | 1.00 | 34.40 | C |
| ATOM | 3381 | C   | ASN | C | 11 | 37.320 | -12.235 | -11.208 | 1.00 | 33.65 | C |
| ATOM | 3382 | O   | ASN | C | 11 | 36.671 | -11.293 | -11.700 | 1.00 | 32.56 | O |
| ATOM | 3383 | CB  | ASN | C | 11 | 38.084 | -13.866 | -12.937 | 1.00 | 37.01 | C |
| ATOM | 3384 | CG  | ASN | C | 11 | 37.899 | -15.220 | -13.604 | 1.00 | 40.57 | C |
| ATOM | 3385 | OD1 | ASN | C | 11 | 37.914 | -16.278 | -12.935 | 1.00 | 43.10 | O |
| ATOM | 3386 | ND2 | ASN | C | 11 | 37.730 | -15.203 | -14.933 | 1.00 | 42.18 | N |
| ATOM | 3387 | N   | PRO | C | 12 | 38.145 | -12.087 | -10.146 | 1.00 | 32.02 | N |
| ATOM | 3388 | CA  | PRO | C | 12 | 38.472 | -10.748 | -9.664  | 1.00 | 31.36 | C |
| ATOM | 3389 | C   | PRO | C | 12 | 38.972 | -9.865  | -10.778 | 1.00 | 29.62 | C |
| ATOM | 3390 | O   | PRO | C | 12 | 39.832 | -10.277 | -11.548 | 1.00 | 29.95 | O |
| ATOM | 3391 | CB  | PRO | C | 12 | 39.543 | -10.988 | -8.615  | 1.00 | 32.37 | C |
| ATOM | 3392 | CG  | PRO | C | 12 | 39.227 | -12.366 | -8.097  | 1.00 | 33.27 | C |
| ATOM | 3393 | CD  | PRO | C | 12 | 38.737 | -13.127 | -9.286  | 1.00 | 33.19 | C |
| ATOM | 3394 | N   | VAL | C | 13 | 38.399 | -8.672  | -10.843 | 1.00 | 27.99 | N |
| ATOM | 3395 | CA  | VAL | C | 13 | 38.617 | -7.716  | -11.914 | 1.00 | 27.83 | C |
| ATOM | 3396 | C   | VAL | C | 13 | 39.421 | -6.538  | -11.433 | 1.00 | 27.57 | C |
| ATOM | 3397 | O   | VAL | C | 13 | 39.052 | -5.885  | -10.473 | 1.00 | 27.54 | O |
| ATOM | 3398 | CB  | VAL | C | 13 | 37.262 | -7.228  | -12.452 | 1.00 | 29.54 | C |
| ATOM | 3399 | CG1 | VAL | C | 13 | 37.453 | -6.212  | -13.573 | 1.00 | 30.57 | C |
| ATOM | 3400 | CG2 | VAL | C | 13 | 36.459 | -8.445  | -12.925 | 1.00 | 30.32 | C |
| ATOM | 3401 | N   | THR | C | 14 | 40.514 | -6.237  | -12.134 | 1.00 | 27.61 | N |
| ATOM | 3402 | CA  | THR | C | 14 | 41.340 | -5.075  | -11.827 | 1.00 | 29.36 | C |
| ATOM | 3403 | C   | THR | C | 14 | 40.595 | -3.781  | -12.111 | 1.00 | 28.29 | C |
| ATOM | 3404 | O   | THR | C | 14 | 39.979 | -3.673  | -13.162 | 1.00 | 25.51 | O |
| ATOM | 3405 | CB  | THR | C | 14 | 42.603 | -5.111  | -12.694 | 1.00 | 31.02 | C |
| ATOM | 3406 | OG1 | THR | C | 14 | 43.308 | -6.340  | -12.416 | 1.00 | 34.17 | O |
| ATOM | 3407 | CG2 | THR | C | 14 | 43.478 | -3.933  | -12.400 | 1.00 | 32.50 | C |
| ATOM | 3408 | N   | LEU | C | 15 | 40.692 | -2.801  | -11.210 | 1.00 | 26.88 | N |
| ATOM | 3409 | CA  | LEU | C | 15 | 40.002 | -1.520  | -11.409 | 1.00 | 27.87 | C |
| ATOM | 3410 | C   | LEU | C | 15 | 40.434 | -0.900  | -12.735 | 1.00 | 28.78 | C |
| ATOM | 3411 | O   | LEU | C | 15 | 41.594 | -1.004  | -13.137 | 1.00 | 28.47 | O |
| ATOM | 3412 | CB  | LEU | C | 15 | 40.238 | -0.510  | -10.281 | 1.00 | 28.46 | C |
| ATOM | 3413 | CG  | LEU | C | 15 | 39.561 | -0.744  | -8.926  | 1.00 | 29.59 | C |
| ATOM | 3414 | CD1 | LEU | C | 15 | 39.796 | 0.463   | -8.009  | 1.00 | 31.84 | C |
| ATOM | 3415 | CD2 | LEU | C | 15 | 38.069 | -1.048  | -9.082  | 1.00 | 31.23 | C |
| ATOM | 3416 | N   | GLY | C | 16 | 39.469 | -0.305  | -13.426 | 1.00 | 29.32 | N |
| ATOM | 3417 | CA  | GLY | C | 16 | 39.697 | 0.333   | -14.736 | 1.00 | 28.24 | C |
| ATOM | 3418 | C   | GLY | C | 16 | 39.685 | -0.555  | -15.951 | 1.00 | 28.29 | C |
| ATOM | 3419 | O   | GLY | C | 16 | 39.681 | -0.053  | -17.081 | 1.00 | 29.89 | O |
| ATOM | 3420 | N   | THR | C | 17 | 39.665 | -1.868  | -15.747 | 1.00 | 27.05 | N |
| ATOM | 3421 | CA  | THR | C | 17 | 39.608 | -2.820  | -16.848 | 1.00 | 27.78 | C |
| ATOM | 3422 | C   | THR | C | 17 | 38.146 | -3.253  | -17.085 | 1.00 | 26.55 | C |
| ATOM | 3423 | O   | THR | C | 17 | 37.246 | -2.853  | -16.350 | 1.00 | 24.26 | O |
| ATOM | 3424 | CB  | THR | C | 17 | 40.510 | -4.034  | -16.572 | 1.00 | 28.60 | C |
| ATOM | 3425 | OG1 | THR | C | 17 | 39.855 | -4.916  | -15.656 | 1.00 | 31.03 | O |
| ATOM | 3426 | CG2 | THR | C | 17 | 41.855 | -3.573  | -16.003 | 1.00 | 30.01 | C |
| ATOM | 3427 | N   | SER | C | 18 | 37.906 | -4.097  | -18.076 | 1.00 | 26.65 | N |
| ATOM | 3428 | CA  | SER | C | 18 | 36.546 | -4.491  | -18.400 | 1.00 | 26.54 | C |
| ATOM | 3429 | C   | SER | C | 18 | 36.202 | -5.812  | -17.732 | 1.00 | 26.56 | C |
| ATOM | 3430 | O   | SER | C | 18 | 36.985 | -6.755  | -17.780 | 1.00 | 26.72 | O |
| ATOM | 3431 | CB  | SER | C | 18 | 36.367 | -4.662  | -19.905 | 1.00 | 28.28 | C |
| ATOM | 3432 | OG  | SER | C | 18 | 36.508 | -3.412  | -20.559 | 1.00 | 30.53 | O |
| ATOM | 3433 | N   | ALA | C | 19 | 35.026 | -5.884  | -17.128 | 1.00 | 25.75 | N |
| ATOM | 3434 | CA  | ALA | C | 19 | 34.509 | -7.161  | -16.604 | 1.00 | 25.42 | C |
| ATOM | 3435 | C   | ALA | C | 19 | 33.511 | -7.693  | -17.567 | 1.00 | 26.26 | C |

| ATOM | 3436 | O   | ALA | C | 19  | 32.818 | -6.926  | -18.236 | 1.00 | 24.14 | O |
|------|------|-----|-----|---|-----|--------|---------|---------|------|-------|---|
| ATOM | 3437 | CB  | ALA | C | 19  | 33.845 | -6.965  | -15.267 | 1.00 | 25.62 | C |
| ATOM | 3438 | N   | SER | C | 20  | 33.411 | -9.009  | -17.661 | 1.00 | 26.72 | N |
| ATOM | 3439 | CA  | SER | C | 20  | 32.384 | -9.592  | -18.489 | 1.00 | 28.10 | C |
| ATOM | 3440 | C   | SER | C | 20  | 31.586 | -10.557 | -17.654 | 1.00 | 29.10 | C |
| ATOM | 3441 | O   | SER | C | 20  | 32.148 | -11.300 | -16.841 | 1.00 | 31.07 | O |
| ATOM | 3442 | CB  | SER | C | 20  | 32.952 | -10.280 | -19.726 | 1.00 | 30.83 | C |
| ATOM | 3443 | OG  | SER | C | 20  | 33.941 | -11.211 | -19.369 | 1.00 | 36.05 | O |
| ATOM | 3444 | N   | ILE | C | 21  | 30.276 | -10.521 | -17.842 | 1.00 | 27.32 | N |
| ATOM | 3445 | CA  | ILE | C | 21  | 29.373 | -11.400 | -17.138 | 1.00 | 26.22 | C |
| ATOM | 3446 | C   | ILE | C | 21  | 28.535 | -12.170 | -18.137 | 1.00 | 25.41 | C |
| ATOM | 3447 | O   | ILE | C | 21  | 27.892 | -11.609 | -19.020 | 1.00 | 23.38 | O |
| ATOM | 3448 | CB  | ILE | C | 21  | 28.585 | -10.622 | -16.087 | 1.00 | 27.18 | C |
| ATOM | 3449 | CG1 | ILE | C | 21  | 29.603 | -10.230 | -14.994 | 1.00 | 27.90 | C |
| ATOM | 3450 | CG2 | ILE | C | 21  | 27.421 | -11.443 | -15.529 | 1.00 | 26.98 | C |
| ATOM | 3451 | CD1 | ILE | C | 21  | 29.075 | -9.512  | -13.863 | 1.00 | 28.68 | C |
| ATOM | 3452 | N   | SER | C | 22  | 28.587 | -13.486 | -17.992 | 1.00 | 26.32 | N |
| ATOM | 3453 | CA  | SER | C | 22  | 27.920 | -14.393 | -18.912 | 1.00 | 26.84 | C |
| ATOM | 3454 | C   | SER | C | 22  | 26.567 | -14.884 | -18.400 | 1.00 | 23.86 | C |
| ATOM | 3455 | O   | SER | C | 22  | 26.352 | -15.078 | -17.199 | 1.00 | 22.34 | O |
| ATOM | 3456 | CB  | SER | C | 22  | 28.815 | -15.609 | -19.210 | 1.00 | 28.70 | C |
| ATOM | 3457 | OG  | SER | C | 22  | 29.938 | -15.199 | -19.968 | 1.00 | 35.41 | O |
| ATOM | 3458 | N   | CYS | C | 23  | 25.655 | -15.083 | -19.345 | 1.00 | 21.91 | N |
| ATOM | 3459 | CA  | CYS | C | 23  | 24.408 | -15.750 | -19.106 | 1.00 | 23.42 | C |
| ATOM | 3460 | C   | CYS | C | 23  | 24.083 | -16.608 | -20.318 | 1.00 | 21.85 | C |
| ATOM | 3461 | O   | CYS | C | 23  | 24.394 | -16.228 | -21.442 | 1.00 | 21.00 | O |
| ATOM | 3462 | CB  | CYS | C | 23  | 23.269 | -14.752 | -18.852 | 1.00 | 22.27 | C |
| ATOM | 3463 | SG  | CYS | C | 23  | 21.664 | -15.489 | -18.596 | 1.00 | 25.56 | S |
| ATOM | 3464 | N   | ARG | C | 24  | 23.520 | -17.785 | -20.068 | 1.00 | 22.46 | N |
| ATOM | 3465 | CA  | ARG | C | 24  | 22.972 | -18.626 | -21.138 | 1.00 | 24.46 | C |
| ATOM | 3466 | C   | ARG | C | 24  | 21.514 | -18.993 | -20.843 | 1.00 | 24.92 | C |
| ATOM | 3467 | O   | ARG | C | 24  | 21.132 | -19.182 | -19.696 | 1.00 | 25.40 | O |
| ATOM | 3468 | CB  | ARG | C | 24  | 23.848 | -19.880 | -21.383 | 1.00 | 25.92 | C |
| ATOM | 3469 | CG  | ARG | C | 24  | 25.276 | -19.527 | -21.833 | 1.00 | 27.40 | C |
| ATOM | 3470 | CD  | ARG | C | 24  | 26.138 | -20.699 | -22.341 | 1.00 | 30.66 | C |
| ATOM | 3471 | NE  | ARG | C | 24  | 27.471 | -20.246 | -22.762 | 1.00 | 31.21 | N |
| ATOM | 3472 | CZ  | ARG | C | 24  | 28.571 | -20.249 | -22.005 | 1.00 | 33.31 | C |
| ATOM | 3473 | NH1 | ARG | C | 24  | 28.547 | -20.689 | -20.751 | 1.00 | 33.71 | N |
| ATOM | 3474 | NH2 | ARG | C | 24  | 29.720 | -19.803 | -22.507 | 1.00 | 33.72 | N |
| ATOM | 3475 | N   | SER | C | 25  | 20.687 | -19.088 | -21.879 | 1.00 | 24.69 | N |
| ATOM | 3476 | CA  | SER | C | 25  | 19.325 | -19.534 | -21.715 | 1.00 | 25.41 | C |
| ATOM | 3477 | C   | SER | C | 25  | 19.129 | -20.879 | -22.406 | 1.00 | 26.11 | C |
| ATOM | 3478 | O   | SER | C | 25  | 19.785 | -21.174 | -23.416 | 1.00 | 27.09 | O |
| ATOM | 3479 | CB  | SER | C | 25  | 18.341 | -18.524 | -22.283 | 1.00 | 25.47 | C |
| ATOM | 3480 | OG  | SER | C | 25  | 18.498 | -18.395 | -23.675 | 1.00 | 28.28 | O |
| ATOM | 3481 | N   | SER | C | 26  | 18.225 | -21.672 | -21.836 | 1.00 | 26.26 | N |
| ATOM | 3482 | CA  | SER | C | 26  | 17.890 | -22.987 | -22.344 | 1.00 | 26.67 | C |
| ATOM | 3483 | C   | SER | C | 26  | 17.104 | -22.931 | -23.640 | 1.00 | 27.72 | C |
| ATOM | 3484 | O   | SER | C | 26  | 17.026 | -23.940 | -24.350 | 1.00 | 30.06 | O |
| ATOM | 3485 | CB  | SER | C | 26  | 17.078 | -23.763 | -21.293 | 1.00 | 26.00 | C |
| ATOM | 3486 | OG  | SER | C | 26  | 15.856 | -23.103 | -20.981 | 1.00 | 25.46 | O |
| ATOM | 3487 | N   | LYS | C | 27  | 16.493 | -21.787 | -23.932 | 1.00 | 28.15 | N |
| ATOM | 3488 | CA  | LYS | C | 27  | 15.678 | -21.579 | -25.128 | 1.00 | 28.53 | C |
| ATOM | 3489 | C   | LYS | C | 27  | 16.094 | -20.265 | -25.775 | 1.00 | 28.28 | C |
| ATOM | 3490 | O   | LYS | C | 27  | 16.440 | -19.310 | -25.070 | 1.00 | 28.21 | O |
| ATOM | 3491 | CB  | LYS | C | 27  | 14.202 | -21.561 | -24.700 | 1.00 | 29.82 | C |
| ATOM | 3492 | CG  | LYS | C | 27  | 13.185 | -21.077 | -25.690 | 1.00 | 33.61 | C |
| ATOM | 3493 | CD  | LYS | C | 27  | 11.741 | -21.401 | -25.228 | 1.00 | 35.74 | C |
| ATOM | 3494 | CE  | LYS | C | 27  | 10.786 | -20.232 | -25.504 | 1.00 | 38.05 | C |
| ATOM | 3495 | NZ  | LYS | C | 27  | 9.333  | -20.515 | -25.125 | 1.00 | 41.17 | N |
| ATOM | 3496 | N   | SER | C | 27A | 16.068 | -20.198 | -27.109 | 1.00 | 26.68 | N |
| ATOM | 3497 | CA  | SER | C | 27A | 16.385 | -18.930 | -27.783 | 1.00 | 25.44 | C |
| ATOM | 3498 | C   | SER | C | 27A | 15.388 | -17.858 | -27.385 | 1.00 | 24.01 | C |
| ATOM | 3499 | O   | SER | C | 27A | 14.203 | -18.129 | -27.282 | 1.00 | 24.83 | O |
| ATOM | 3500 | CB  | SER | C | 27A | 16.367 | -19.069 | -29.300 | 1.00 | 24.87 | C |

```
ATOM   3501  OG   SER C  27A    16.557 -17.800 -29.910  1.00 24.76           O
ATOM   3502  N    LEU C  27B    15.884 -16.642 -27.133  1.00 25.21           N
ATOM   3503  CA   LEU C  27B    15.041 -15.508 -26.730  1.00 24.94           C
ATOM   3504  C    LEU C  27B    14.818 -14.545 -27.881  1.00 25.90           C
ATOM   3505  O    LEU C  27B    14.210 -13.497 -27.693  1.00 25.01           O
ATOM   3506  CB   LEU C  27B    15.654 -14.735 -25.536  1.00 24.49           C
ATOM   3507  CG   LEU C  27B    16.023 -15.558 -24.291  1.00 22.99           C
ATOM   3508  CD1  LEU C  27B    16.505 -14.612 -23.188  1.00 22.78           C
ATOM   3509  CD2  LEU C  27B    14.875 -16.411 -23.844  1.00 23.71           C
ATOM   3510  N    LEU C  27C    15.310 -14.914 -29.070  1.00 27.48           N
ATOM   3511  CA   LEU C  27C    15.059 -14.158 -30.291  1.00 28.87           C
ATOM   3512  C    LEU C  27C    13.647 -14.445 -30.762  1.00 30.07           C
ATOM   3513  O    LEU C  27C    13.243 -15.606 -30.889  1.00 32.18           O
ATOM   3514  CB   LEU C  27C    16.070 -14.526 -31.385  1.00 29.32           C
ATOM   3515  CG   LEU C  27C    15.770 -13.937 -32.779  1.00 31.49           C
ATOM   3516  CD1  LEU C  27C    15.847 -12.422 -32.801  1.00 31.10           C
ATOM   3517  CD2  LEU C  27C    16.710 -14.515 -33.821  1.00 32.11           C
ATOM   3518  N    HIS C  27D    12.888 -13.393 -31.018  1.00 29.85           N
ATOM   3519  CA   HIS C  27D    11.511 -13.507 -31.456  1.00 30.89           C
ATOM   3520  C    HIS C  27D    11.479 -13.166 -32.953  1.00 31.01           C
ATOM   3521  O    HIS C  27D    12.475 -12.725 -33.524  1.00 29.83           O
ATOM   3522  CB   HIS C  27D    10.628 -12.542 -30.655  1.00 31.79           C
ATOM   3523  CG   HIS C  27D     9.161 -12.675 -30.925  1.00 31.56           C
ATOM   3524  ND1  HIS C  27D     8.355 -11.594 -31.201  1.00 34.52           N
ATOM   3525  CD2  HIS C  27D     8.351 -13.757 -30.941  1.00 31.06           C
ATOM   3526  CE1  HIS C  27D     7.114 -12.003 -31.385  1.00 32.59           C
ATOM   3527  NE2  HIS C  27D     7.085 -13.314 -31.238  1.00 33.35           N
ATOM   3528  N    SER C  27E    10.316 -13.369 -33.551  1.00 33.28           N
ATOM   3529  CA   SER C  27E    10.079 -13.119 -34.976  1.00 33.74           C
ATOM   3530  C    SER C  27E    10.045 -11.642 -35.337  1.00 33.69           C
ATOM   3531  O    SER C  27E    10.142 -11.296 -36.515  1.00 34.81           O
ATOM   3532  CB   SER C  27E     8.777 -13.781 -35.410  1.00 34.43           C
ATOM   3533  OG   SER C  27E     7.691 -13.377 -34.608  1.00 35.92           O
ATOM   3534  N    ASP C  28      9.907 -10.763 -34.346  1.00 32.00           N
ATOM   3535  CA   ASP C  28     10.052  -9.324 -34.596  1.00 31.25           C
ATOM   3536  C    ASP C  28     11.520  -8.880 -34.682  1.00 30.48           C
ATOM   3537  O    ASP C  28     11.823  -7.709 -34.943  1.00 29.60           O
ATOM   3538  CB   ASP C  28      9.261  -8.504 -33.556  1.00 31.71           C
ATOM   3539  CG   ASP C  28      9.746  -8.721 -32.133  1.00 31.60           C
ATOM   3540  OD1  ASP C  28     10.732  -9.465 -31.970  1.00 29.92           O
ATOM   3541  OD2  ASP C  28      9.123  -8.137 -31.200  1.00 32.70           O
ATOM   3542  N    GLY C  29     12.450  -9.807 -34.469  1.00 29.91           N
ATOM   3543  CA   GLY C  29     13.861  -9.492 -34.559  1.00 29.41           C
ATOM   3544  C    GLY C  29     14.493  -9.084 -33.241  1.00 28.91           C
ATOM   3545  O    GLY C  29     15.691  -8.819 -33.176  1.00 30.37           O
ATOM   3546  N    ILE C  30     13.660  -8.986 -32.209  1.00 29.45           N
ATOM   3547  CA   ILE C  30     14.118  -8.594 -30.877  1.00 27.66           C
ATOM   3548  C    ILE C  30     14.470  -9.854 -30.074  1.00 26.26           C
ATOM   3549  O    ILE C  30     13.747 -10.858 -30.109  1.00 28.01           O
ATOM   3550  CB   ILE C  30     13.058  -7.696 -30.169  1.00 27.28           C
ATOM   3551  CG1  ILE C  30     12.831  -6.415 -30.963  1.00 27.88           C
ATOM   3552  CG2  ILE C  30     13.505  -7.329 -28.777  1.00 28.01           C
ATOM   3553  CD1  ILE C  30     11.600  -5.669 -30.558  1.00 26.85           C
ATOM   3554  N    THR C  31     15.584  -9.762 -29.353  1.00 25.78           N
ATOM   3555  CA   THR C  31     16.034 -10.757 -28.403  1.00 25.65           C
ATOM   3556  C    THR C  31     15.681 -10.217 -27.009  1.00 24.64           C
ATOM   3557  O    THR C  31     16.226  -9.186 -26.564  1.00 25.21           O
ATOM   3558  CB   THR C  31     17.541 -10.965 -28.515  1.00 26.74           C
ATOM   3559  OG1  THR C  31     17.881 -11.272 -29.882  1.00 27.74           O
ATOM   3560  CG2  THR C  31     18.004 -12.114 -27.614  1.00 24.38           C
ATOM   3561  N    TYR C  32     14.775 -10.934 -26.339  1.00 23.43           N
ATOM   3562  CA   TYR C  32     14.095 -10.410 -25.143  1.00 22.64           C
ATOM   3563  C    TYR C  32     14.920 -10.801 -23.921  1.00 21.40           C
ATOM   3564  O    TYR C  32     14.498 -11.599 -23.079  1.00 20.02           O
ATOM   3565  CB   TYR C  32     12.633 -10.869 -25.085  1.00 23.92           C
```

| ATOM | 3566 | CG  | TYR C | 32 | 11.751 | -10.073 | -26.033 | 1.00 | 24.40 | C |
| ATOM | 3567 | CD1 | TYR C | 32 | 11.648 | -10.422 | -27.391 | 1.00 | 27.67 | C |
| ATOM | 3568 | CD2 | TYR C | 32 | 11.073 | -8.958  | -25.602 | 1.00 | 27.97 | C |
| ATOM | 3569 | CE1 | TYR C | 32 | 10.873 | -9.675  | -28.262 | 1.00 | 26.95 | C |
| ATOM | 3570 | CE2 | TYR C | 32 | 10.276 | -8.218  | -26.473 | 1.00 | 28.48 | C |
| ATOM | 3571 | CZ  | TYR C | 32 | 10.174 | -8.585  | -27.793 | 1.00 | 27.56 | C |
| ATOM | 3572 | OH  | TYR C | 32 | 9.389  | -7.818  | -28.634 | 1.00 | 29.88 | O |
| ATOM | 3573 | N   | LEU C | 33 | 16.115 | -10.241 | -23.882 | 1.00 | 19.46 | N |
| ATOM | 3574 | CA  | LEU C | 33 | 17.114 | -10.501 | -22.829 | 1.00 | 19.32 | C |
| ATOM | 3575 | C   | LEU C | 33 | 17.335 | -9.191  | -22.087 | 1.00 | 17.97 | C |
| ATOM | 3576 | O   | LEU C | 33 | 17.590 | -8.152  | -22.683 | 1.00 | 18.52 | O |
| ATOM | 3577 | CB  | LEU C | 33 | 18.445 | -11.055 | -23.406 | 1.00 | 18.61 | C |
| ATOM | 3578 | CG  | LEU C | 33 | 19.598 | -11.436 | -22.467 | 1.00 | 19.08 | C |
| ATOM | 3579 | CD1 | LEU C | 33 | 20.412 | -10.287 | -21.857 | 1.00 | 20.56 | C |
| ATOM | 3580 | CD2 | LEU C | 33 | 19.087 | -12.344 | -21.348 | 1.00 | 19.16 | C |
| ATOM | 3581 | N   | TYR C | 34 | 17.298 | -9.285  | -20.756 | 1.00 | 17.07 | N |
| ATOM | 3582 | CA  | TYR C | 34 | 17.429 | -8.160  | -19.859 | 1.00 | 17.65 | C |
| ATOM | 3583 | C   | TYR C | 34 | 18.496 | -8.432  | -18.803 | 1.00 | 17.33 | C |
| ATOM | 3584 | O   | TYR C | 34 | 18.615 | -9.581  | -18.335 | 1.00 | 17.44 | O |
| ATOM | 3585 | CB  | TYR C | 34 | 16.101 | -7.933  | -19.141 | 1.00 | 17.74 | C |
| ATOM | 3586 | CG  | TYR C | 34 | 14.952 | -7.569  | -20.047 | 1.00 | 16.76 | C |
| ATOM | 3587 | CD1 | TYR C | 34 | 14.412 | -8.479  | -20.937 | 1.00 | 17.75 | C |
| ATOM | 3588 | CD2 | TYR C | 34 | 14.406 | -6.293  | -20.028 | 1.00 | 15.14 | C |
| ATOM | 3589 | CE1 | TYR C | 34 | 13.355 | -8.148  | -21.749 | 1.00 | 19.79 | C |
| ATOM | 3590 | CE2 | TYR C | 34 | 13.378 | -5.978  | -20.849 | 1.00 | 17.80 | C |
| ATOM | 3591 | CZ  | TYR C | 34 | 12.856 | -6.895  | -21.706 | 1.00 | 19.34 | C |
| ATOM | 3592 | OH  | TYR C | 34 | 11.797 | -6.584  | -22.526 | 1.00 | 22.62 | O |
| ATOM | 3593 | N   | TRP C | 35 | 19.215 | -7.372  | -18.408 | 1.00 | 14.94 | N |
| ATOM | 3594 | CA  | TRP C | 35 | 20.168 | -7.407  | -17.312 | 1.00 | 17.47 | C |
| ATOM | 3595 | C   | TRP C | 35 | 19.732 | -6.494  | -16.166 | 1.00 | 17.27 | C |
| ATOM | 3596 | O   | TRP C | 35 | 19.359 | -5.327  | -16.394 | 1.00 | 14.63 | O |
| ATOM | 3597 | CB  | TRP C | 35 | 21.573 | -7.000  | -17.742 | 1.00 | 17.15 | C |
| ATOM | 3598 | CG  | TRP C | 35 | 22.295 | -7.930  | -18.682 | 1.00 | 16.90 | C |
| ATOM | 3599 | CD1 | TRP C | 35 | 22.426 | -7.807  | -20.036 | 1.00 | 19.30 | C |
| ATOM | 3600 | CD2 | TRP C | 35 | 22.966 | -9.141  | -18.320 | 1.00 | 16.92 | C |
| ATOM | 3601 | NE1 | TRP C | 35 | 23.173 | -8.857  | -20.543 | 1.00 | 19.71 | N |
| ATOM | 3602 | CE2 | TRP C | 35 | 23.515 | -9.689  | -19.509 | 1.00 | 18.72 | C |
| ATOM | 3603 | CE3 | TRP C | 35 | 23.183 | -9.797  | -17.111 | 1.00 | 18.89 | C |
| ATOM | 3604 | CZ2 | TRP C | 35 | 24.246 | -10.877 | -19.518 | 1.00 | 19.29 | C |
| ATOM | 3605 | CZ3 | TRP C | 35 | 23.938 | -10.993 | -17.127 | 1.00 | 18.31 | C |
| ATOM | 3606 | CH2 | TRP C | 35 | 24.443 | -11.504 | -18.325 | 1.00 | 17.96 | C |
| ATOM | 3607 | N   | TYR C | 36 | 19.856 | -7.008  | -14.941 | 1.00 | 16.33 | N |
| ATOM | 3608 | CA  | TYR C | 36 | 19.607 | -6.264  | -13.682 | 1.00 | 17.23 | C |
| ATOM | 3609 | C   | TYR C | 36 | 20.829 | -6.349  | -12.771 | 1.00 | 17.75 | C |
| ATOM | 3610 | O   | TYR C | 36 | 21.595 | -7.299  | -12.834 | 1.00 | 16.52 | O |
| ATOM | 3611 | CB  | TYR C | 36 | 18.408 | -6.814  | -12.905 | 1.00 | 17.33 | C |
| ATOM | 3612 | CG  | TYR C | 36 | 17.128 | -6.729  | -13.669 | 1.00 | 16.65 | C |
| ATOM | 3613 | CD1 | TYR C | 36 | 16.748 | -7.770  | -14.471 | 1.00 | 14.91 | C |
| ATOM | 3614 | CD2 | TYR C | 36 | 16.298 | -5.611  | -13.622 | 1.00 | 15.15 | C |
| ATOM | 3615 | CE1 | TYR C | 36 | 15.622 | -7.727  | -15.202 | 1.00 | 16.42 | C |
| ATOM | 3616 | CE2 | TYR C | 36 | 15.141 | -5.552  | -14.370 | 1.00 | 17.40 | C |
| ATOM | 3617 | CZ  | TYR C | 36 | 14.811 | -6.610  | -15.170 | 1.00 | 17.16 | C |
| ATOM | 3618 | OH  | TYR C | 36 | 13.672 | -6.634  | -15.882 | 1.00 | 19.97 | O |
| ATOM | 3619 | N   | LEU C | 37 | 20.989 | -5.331  | -11.936 | 1.00 | 17.14 | N |
| ATOM | 3620 | CA  | LEU C | 37 | 22.006 | -5.296  | -10.919 | 1.00 | 17.44 | C |
| ATOM | 3621 | C   | LEU C | 37 | 21.325 | -5.081  | -9.559  | 1.00 | 18.05 | C |
| ATOM | 3622 | O   | LEU C | 37 | 20.467 | -4.191  | -9.420  | 1.00 | 18.27 | O |
| ATOM | 3623 | CB  | LEU C | 37 | 22.933 | -4.103  | -11.134 | 1.00 | 16.75 | C |
| ATOM | 3624 | CG  | LEU C | 37 | 23.919 | -3.706  | -10.036 | 1.00 | 19.29 | C |
| ATOM | 3625 | CD1 | LEU C | 37 | 24.826 | -4.851  | -9.655  | 1.00 | 16.99 | C |
| ATOM | 3626 | CD2 | LEU C | 37 | 24.719 | -2.540  | -10.494 | 1.00 | 18.32 | C |
| ATOM | 3627 | N   | GLN C | 38 | 21.744 | -5.864  | -8.564  | 1.00 | 18.37 | N |
| ATOM | 3628 | CA  | GLN C | 38 | 21.392 | -5.602  | -7.173  | 1.00 | 19.52 | C |
| ATOM | 3629 | C   | GLN C | 38 | 22.663 | -5.386  | -6.366  | 1.00 | 20.02 | C |
| ATOM | 3630 | O   | GLN C | 38 | 23.489 | -6.297  | -6.195  | 1.00 | 19.32 | O |

| | | | | | | | | | | | |
|---|---|---|---|---|---|---|---|---|---|---|---|
| ATOM | 3631 | CB | GLN | C | 38 | 20.579 | -6.754 | -6.560 | 1.00 | 21.27 | C |
| ATOM | 3632 | CG | GLN | C | 38 | 20.246 | -6.478 | -5.098 | 1.00 | 22.65 | C |
| ATOM | 3633 | CD | GLN | C | 38 | 19.033 | -7.225 | -4.584 | 1.00 | 23.57 | C |
| ATOM | 3634 | OE1 | GLN | C | 38 | 18.949 | -8.439 | -4.720 | 1.00 | 28.94 | O |
| ATOM | 3635 | NE2 | GLN | C | 38 | 18.118 | -6.497 | -3.948 | 1.00 | 27.56 | N |
| ATOM | 3636 | N | LYS | C | 39 | 22.851 | -4.145 | -5.930 | 1.00 | 20.91 | N |
| ATOM | 3637 | CA | LYS | C | 39 | 23.905 | -3.818 | -5.002 | 1.00 | 24.12 | C |
| ATOM | 3638 | C | LYS | C | 39 | 23.417 | -4.194 | -3.614 | 1.00 | 25.91 | C |
| ATOM | 3639 | O | LYS | C | 39 | 22.219 | -4.262 | -3.369 | 1.00 | 26.95 | O |
| ATOM | 3640 | CB | LYS | C | 39 | 24.286 | -2.332 | -5.066 | 1.00 | 24.13 | C |
| ATOM | 3641 | CG | LYS | C | 39 | 24.812 | -1.895 | -6.426 | 1.00 | 26.27 | C |
| ATOM | 3642 | CD | LYS | C | 39 | 25.213 | -0.421 | -6.473 | 1.00 | 25.44 | C |
| ATOM | 3643 | CE | LYS | C | 39 | 26.057 | -0.148 | -7.700 | 1.00 | 27.45 | C |
| ATOM | 3644 | NZ | LYS | C | 39 | 26.410 | 1.299 | -7.792 | 1.00 | 30.54 | N |
| ATOM | 3645 | N | PRO | C | 40 | 24.346 | -4.474 | -2.695 | 1.00 | 30.65 | N |
| ATOM | 3646 | CA | PRO | C | 40 | 23.965 | -4.876 | -1.330 | 1.00 | 32.94 | C |
| ATOM | 3647 | C | PRO | C | 40 | 22.993 | -3.960 | -0.643 | 1.00 | 34.53 | C |
| ATOM | 3648 | O | PRO | C | 40 | 23.253 | -2.753 | -0.531 | 1.00 | 35.26 | O |
| ATOM | 3649 | CB | PRO | C | 40 | 25.290 | -4.852 | -0.595 | 1.00 | 32.73 | C |
| ATOM | 3650 | CG | PRO | C | 40 | 26.275 | -5.200 | -1.647 | 1.00 | 33.04 | C |
| ATOM | 3651 | CD | PRO | C | 40 | 25.808 | -4.476 | -2.870 | 1.00 | 31.32 | C |
| ATOM | 3652 | N | GLY | C | 41 | 21.870 | -4.536 | -0.217 | 1.00 | 37.27 | N |
| ATOM | 3653 | CA | GLY | C | 41 | 20.805 | -3.798 | 0.474 | 1.00 | 38.00 | C |
| ATOM | 3654 | C | GLY | C | 41 | 19.952 | -2.877 | -0.374 | 1.00 | 39.04 | C |
| ATOM | 3655 | O | GLY | C | 41 | 19.142 | -2.113 | 0.164 | 1.00 | 40.65 | O |
| ATOM | 3656 | N | GLN | C | 42 | 20.117 | -2.937 | -1.693 | 1.00 | 37.43 | N |
| ATOM | 3657 | CA | GLN | C | 42 | 19.435 | -2.011 | -2.596 | 1.00 | 37.29 | C |
| ATOM | 3658 | C | GLN | C | 42 | 18.406 | -2.797 | -3.386 | 1.00 | 35.72 | C |
| ATOM | 3659 | O | GLN | C | 42 | 18.445 | -4.018 | -3.386 | 1.00 | 35.46 | O |
| ATOM | 3660 | CB | GLN | C | 42 | 20.412 | -1.384 | -3.577 | 1.00 | 38.11 | C |
| ATOM | 3661 | CG | GLN | C | 42 | 21.110 | -0.121 | -3.123 | 1.00 | 39.93 | C |
| ATOM | 3662 | CD | GLN | C | 42 | 21.980 | 0.457 | -4.236 | 1.00 | 42.46 | C |
| ATOM | 3663 | OE1 | GLN | C | 42 | 21.713 | 0.246 | -5.434 | 1.00 | 43.23 | O |
| ATOM | 3664 | NE2 | GLN | C | 42 | 23.042 | 1.178 | -3.850 | 1.00 | 41.60 | N |
| ATOM | 3665 | N | SER | C | 43 | 17.479 | -2.091 | -4.037 | 1.00 | 35.35 | N |
| ATOM | 3666 | CA | SER | C | 43 | 16.555 | -2.767 | -4.958 | 1.00 | 34.06 | C |
| ATOM | 3667 | C | SER | C | 43 | 17.323 | -3.156 | -6.225 | 1.00 | 32.24 | C |
| ATOM | 3668 | O | SER | C | 43 | 18.205 | -2.411 | -6.636 | 1.00 | 32.62 | O |
| ATOM | 3669 | CB | SER | C | 43 | 15.360 | -1.886 | -5.328 | 1.00 | 34.64 | C |
| ATOM | 3670 | OG | SER | C | 43 | 15.683 | -0.857 | -6.245 | 1.00 | 38.70 | O |
| ATOM | 3671 | N | PRO | C | 44 | 16.954 | -4.285 | -6.863 | 1.00 | 29.95 | N |
| ATOM | 3672 | CA | PRO | C | 44 | 17.469 | -4.553 | -8.212 | 1.00 | 26.99 | C |
| ATOM | 3673 | C | PRO | C | 44 | 17.072 | -3.409 | -9.120 | 1.00 | 25.67 | C |
| ATOM | 3674 | O | PRO | C | 44 | 16.054 | -2.760 | -8.899 | 1.00 | 26.39 | O |
| ATOM | 3675 | CB | PRO | C | 44 | 16.771 | -5.846 | -8.622 | 1.00 | 27.75 | C |
| ATOM | 3676 | CG | PRO | C | 44 | 16.236 | -6.443 | -7.307 | 1.00 | 28.24 | C |
| ATOM | 3677 | CD | PRO | C | 44 | 16.005 | -5.323 | -6.400 | 1.00 | 28.95 | C |
| ATOM | 3678 | N | HIS | C | 45 | 17.881 | -3.128 | -10.119 | 1.00 | 23.06 | N |
| ATOM | 3679 | CA | HIS | C | 45 | 17.513 | -2.154 | -11.110 | 1.00 | 23.88 | C |
| ATOM | 3680 | C | HIS | C | 45 | 17.979 | -2.565 | -12.468 | 1.00 | 20.37 | C |
| ATOM | 3681 | O | HIS | C | 45 | 18.975 | -3.314 | -12.635 | 1.00 | 18.41 | O |
| ATOM | 3682 | CB | HIS | C | 45 | 18.024 | -0.747 | -10.772 | 1.00 | 26.18 | C |
| ATOM | 3683 | CG | HIS | C | 45 | 19.513 | -0.612 | -10.777 | 1.00 | 29.98 | C |
| ATOM | 3684 | ND1 | HIS | C | 45 | 20.299 | -0.953 | -9.694 | 1.00 | 33.10 | N |
| ATOM | 3685 | CD2 | HIS | C | 45 | 20.364 | -0.177 | -11.738 | 1.00 | 31.07 | C |
| ATOM | 3686 | CE1 | HIS | C | 45 | 21.567 | -0.725 | -9.986 | 1.00 | 32.72 | C |
| ATOM | 3687 | NE2 | HIS | C | 45 | 21.632 | -0.244 | -11.216 | 1.00 | 31.95 | N |
| ATOM | 3688 | N | LEU | C | 46 | 17.235 | -2.086 | -13.440 | 1.00 | 18.80 | N |
| ATOM | 3689 | CA | LEU | C | 46 | 17.421 | -2.477 | -14.823 | 1.00 | 18.75 | C |
| ATOM | 3690 | C | LEU | C | 46 | 18.665 | -1.822 | -15.351 | 1.00 | 18.52 | C |
| ATOM | 3691 | O | LEU | C | 46 | 18.865 | -0.584 | -15.170 | 1.00 | 18.04 | O |
| ATOM | 3692 | CB | LEU | C | 46 | 16.208 | -2.113 | -15.692 | 1.00 | 16.72 | C |
| ATOM | 3693 | CG | LEU | C | 46 | 16.249 | -2.439 | -17.181 | 1.00 | 19.05 | C |
| ATOM | 3694 | CD1 | LEU | C | 46 | 16.246 | -3.987 | -17.398 | 1.00 | 18.95 | C |
| ATOM | 3695 | CD2 | LEU | C | 46 | 15.138 | -1.788 | -17.969 | 1.00 | 20.54 | C |

```
ATOM   3696  N    LEU C  47    19.496  -2.634 -16.004  1.00 18.08         N
ATOM   3697  CA   LEU C  47    20.693  -2.144 -16.728  1.00 20.07         C
ATOM   3698  C    LEU C  47    20.531  -2.102 -18.234  1.00 19.79         C
ATOM   3699  O    LEU C  47    20.918  -1.138 -18.888  1.00 20.31         O
ATOM   3700  CB   LEU C  47    21.922  -3.019 -16.390  1.00 20.16         C
ATOM   3701  CG   LEU C  47    22.608  -2.877 -15.038  1.00 21.45         C
ATOM   3702  CD1  LEU C  47    23.767  -3.850 -14.976  1.00 21.69         C
ATOM   3703  CD2  LEU C  47    23.086  -1.496 -14.744  1.00 23.35         C
ATOM   3704  N    ILE C  48    20.060  -3.202 -18.789  1.00 19.75         N
ATOM   3705  CA   ILE C  48    19.979  -3.410 -20.217  1.00 20.10         C
ATOM   3706  C    ILE C  48    18.654  -4.096 -20.558  1.00 19.26         C
ATOM   3707  O    ILE C  48    18.275  -5.058 -19.904  1.00 20.65         O
ATOM   3708  CB   ILE C  48    21.115  -4.393 -20.705  1.00 20.18         C
ATOM   3709  CG1  ILE C  48    22.519  -3.889 -20.352  1.00 21.37         C
ATOM   3710  CG2  ILE C  48    20.984  -4.728 -22.211  1.00 19.53         C
ATOM   3711  CD1  ILE C  48    22.940  -2.667 -21.178  1.00 23.13         C
ATOM   3712  N    TYR C  49    17.971  -3.640 -21.620  1.00 19.26         N
ATOM   3713  CA   TYR C  49    16.765  -4.297 -22.119  1.00 21.31         C
ATOM   3714  C    TYR C  49    16.974  -4.652 -23.596  1.00 20.97         C
ATOM   3715  O    TYR C  49    17.838  -4.051 -24.268  1.00 20.19         O
ATOM   3716  CB   TYR C  49    15.531  -3.421 -21.913  1.00 22.70         C
ATOM   3717  CG   TYR C  49    15.519  -2.188 -22.788  1.00 23.14         C
ATOM   3718  CD1  TYR C  49    16.162  -1.036 -22.387  1.00 24.71         C
ATOM   3719  CD2  TYR C  49    14.855  -2.206 -24.016  1.00 26.81         C
ATOM   3720  CE1  TYR C  49    16.174   0.086 -23.203  1.00 24.63         C
ATOM   3721  CE2  TYR C  49    14.841  -1.087 -24.837  1.00 26.02         C
ATOM   3722  CZ   TYR C  49    15.490   0.056 -24.417  1.00 28.00         C
ATOM   3723  OH   TYR C  49    15.495   1.159 -25.228  1.00 28.12         O
ATOM   3724  N    HIS C  50    16.254  -5.672 -24.036  1.00 22.01         N
ATOM   3725  CA   HIS C  50    16.286  -6.162 -25.411  1.00 23.45         C
ATOM   3726  C    HIS C  50    17.693  -6.518 -25.847  1.00 22.96         C
ATOM   3727  O    HIS C  50    18.135  -6.135 -26.923  1.00 24.75         O
ATOM   3728  CB   HIS C  50    15.695  -5.097 -26.364  1.00 24.00         C
ATOM   3729  CG   HIS C  50    14.220  -4.923 -26.236  1.00 26.61         C
ATOM   3730  ND1  HIS C  50    13.528  -3.921 -26.890  1.00 25.54         N
ATOM   3731  CD2  HIS C  50    13.299  -5.612 -25.521  1.00 26.57         C
ATOM   3732  CE1  HIS C  50    12.244  -4.014 -26.595  1.00 26.23         C
ATOM   3733  NE2  HIS C  50    12.081  -5.022 -25.754  1.00 28.53         N
ATOM   3734  N    LEU C  51    18.415  -7.239 -25.004  1.00 22.79         N
ATOM   3735  CA   LEU C  51    19.792  -7.676 -25.339  1.00 22.43         C
ATOM   3736  C    LEU C  51    20.900  -6.595 -25.317  1.00 22.32         C
ATOM   3737  O    LEU C  51    21.930  -6.787 -24.690  1.00 20.33         O
ATOM   3738  CB   LEU C  51    19.797  -8.450 -26.657  1.00 23.47         C
ATOM   3739  CG   LEU C  51    21.145  -9.059 -27.020  1.00 23.66         C
ATOM   3740  CD1  LEU C  51    21.601 -10.124 -26.004  1.00 25.10         C
ATOM   3741  CD2  LEU C  51    21.122  -9.613 -28.458  1.00 24.50         C
ATOM   3742  N    SER C  52    20.706  -5.448 -25.940  1.00 22.52         N
ATOM   3743  CA   SER C  52    21.826  -4.535 -26.126  1.00 23.57         C
ATOM   3744  C    SER C  52    21.484  -3.112 -25.841  1.00 23.60         C
ATOM   3745  O    SER C  52    22.338  -2.231 -26.005  1.00 23.80         O
ATOM   3746  CB   SER C  52    22.371  -4.682 -27.543  1.00 24.39         C
ATOM   3747  OG   SER C  52    21.306  -4.674 -28.474  1.00 26.34         O
ATOM   3748  N    ASN C  53    20.262  -2.842 -25.377  1.00 24.36         N
ATOM   3749  CA   ASN C  53    19.859  -1.466 -25.188  1.00 25.80         C
ATOM   3750  C    ASN C  53    20.031  -1.000 -23.757  1.00 25.89         C
ATOM   3751  O    ASN C  53    19.574  -1.645 -22.812  1.00 24.71         O
ATOM   3752  CB   ASN C  53    18.429  -1.268 -25.680  1.00 26.56         C
ATOM   3753  CG   ASN C  53    18.279  -1.662 -27.131  1.00 28.96         C
ATOM   3754  OD1  ASN C  53    18.572  -0.874 -28.017  1.00 32.71         O
ATOM   3755  ND2  ASN C  53    17.913  -2.915 -27.382  1.00 29.68         N
ATOM   3756  N    LEU C  54    20.676   0.150 -23.618  1.00 24.89         N
ATOM   3757  CA   LEU C  54    21.005   0.734 -22.327  1.00 25.95         C
ATOM   3758  C    LEU C  54    19.776   1.341 -21.634  1.00 27.34         C
ATOM   3759  O    LEU C  54    19.045   2.126 -22.239  1.00 26.86         O
ATOM   3760  CB   LEU C  54    22.060   1.826 -22.538  1.00 27.81         C
```

91

| ATOM | 3761 | CG | LEU | C | 54 | 22.915 | 2.355 | -21.400 | 1.00 | 28.79 | C |
|------|------|-----|-----|---|----|--------|--------|---------|------|-------|---|
| ATOM | 3762 | CD1 | LEU | C | 54 | 23.945 | 1.328 | -20.996 | 1.00 | 30.28 | C |
| ATOM | 3763 | CD2 | LEU | C | 54 | 23.592 | 3.643 | -21.852 | 1.00 | 28.56 | C |
| ATOM | 3764 | N | ALA | C | 55 | 19.582 | 1.009 | -20.361 | 1.00 | 27.50 | N |
| ATOM | 3765 | CA | ALA | C | 55 | 18.434 | 1.512 | -19.596 | 1.00 | 27.00 | C |
| ATOM | 3766 | C | ALA | C | 55 | 18.669 | 2.917 | -19.056 | 1.00 | 28.02 | C |
| ATOM | 3767 | O | ALA | C | 55 | 19.792 | 3.405 | -18.988 | 1.00 | 25.08 | O |
| ATOM | 3768 | CB | ALA | C | 55 | 18.084 | 0.566 | -18.467 | 1.00 | 27.67 | C |
| ATOM | 3769 | N | SER | C | 56 | 17.583 | 3.559 | -18.650 | 1.00 | 29.42 | N |
| ATOM | 3770 | CA | SER | C | 56 | 17.664 | 4.865 | -18.011 | 1.00 | 30.20 | C |
| ATOM | 3771 | C | SER | C | 56 | 18.556 | 4.837 | -16.798 | 1.00 | 29.81 | C |
| ATOM | 3772 | O | SER | C | 56 | 18.555 | 3.852 | -16.037 | 1.00 | 31.31 | O |
| ATOM | 3773 | CB | SER | C | 56 | 16.270 | 5.352 | -17.599 | 1.00 | 31.79 | C |
| ATOM | 3774 | OG | SER | C | 56 | 15.698 | 6.062 | -18.666 | 1.00 | 35.36 | O |
| ATOM | 3775 | N | GLY | C | 57 | 19.347 | 5.892 | -16.633 | 1.00 | 28.05 | N |
| ATOM | 3776 | CA | GLY | C | 57 | 20.224 | 6.031 | -15.479 | 1.00 | 27.04 | C |
| ATOM | 3777 | C | GLY | C | 57 | 21.493 | 5.206 | -15.492 | 1.00 | 26.43 | C |
| ATOM | 3778 | O | GLY | C | 57 | 22.204 | 5.144 | -14.498 | 1.00 | 27.79 | O |
| ATOM | 3779 | N | VAL | C | 58 | 21.779 | 4.543 | -16.612 | 1.00 | 24.12 | N |
| ATOM | 3780 | CA | VAL | C | 58 | 22.923 | 3.660 | -16.714 | 1.00 | 23.91 | C |
| ATOM | 3781 | C | VAL | C | 58 | 23.946 | 4.290 | -17.671 | 1.00 | 21.03 | C |
| ATOM | 3782 | O | VAL | C | 58 | 23.585 | 4.608 | -18.803 | 1.00 | 21.11 | O |
| ATOM | 3783 | CB | VAL | C | 58 | 22.495 | 2.268 | -17.217 | 1.00 | 23.40 | C |
| ATOM | 3784 | CG1 | VAL | C | 58 | 23.683 | 1.311 | -17.306 | 1.00 | 24.97 | C |
| ATOM | 3785 | CG2 | VAL | C | 58 | 21.422 | 1.689 | -16.331 | 1.00 | 25.28 | C |
| ATOM | 3786 | N | PRO | C | 59 | 25.218 | 4.441 | -17.227 | 1.00 | 21.51 | N |
| ATOM | 3787 | CA | PRO | C | 59 | 26.228 | 5.069 | -18.088 | 1.00 | 19.34 | C |
| ATOM | 3788 | C | PRO | C | 59 | 26.662 | 4.156 | -19.203 | 1.00 | 20.14 | C |
| ATOM | 3789 | O | PRO | C | 59 | 26.594 | 2.933 | -19.050 | 1.00 | 19.12 | O |
| ATOM | 3790 | CB | PRO | C | 59 | 27.411 | 5.319 | -17.159 | 1.00 | 20.28 | C |
| ATOM | 3791 | CG | PRO | C | 59 | 27.276 | 4.286 | -16.087 | 1.00 | 19.53 | C |
| ATOM | 3792 | CD | PRO | C | 59 | 25.781 | 4.051 | -15.922 | 1.00 | 20.23 | C |
| ATOM | 3793 | N | ASP | C | 60 | 27.083 | 4.746 | -20.321 | 1.00 | 19.22 | N |
| ATOM | 3794 | CA | ASP | C | 60 | 27.417 | 3.953 | -21.524 | 1.00 | 19.39 | C |
| ATOM | 3795 | C | ASP | C | 60 | 28.661 | 3.025 | -21.443 | 1.00 | 19.68 | C |
| ATOM | 3796 | O | ASP | C | 60 | 28.990 | 2.369 | -22.415 | 1.00 | 20.70 | O |
| ATOM | 3797 | CB | ASP | C | 60 | 27.473 | 4.837 | -22.770 | 1.00 | 19.43 | C |
| ATOM | 3798 | CG | ASP | C | 60 | 28.596 | 5.857 | -22.745 | 1.00 | 19.89 | C |
| ATOM | 3799 | OD1 | ASP | C | 60 | 29.508 | 5.761 | -21.909 | 1.00 | 17.58 | O |
| ATOM | 3800 | OD2 | ASP | C | 60 | 28.571 | 6.752 | -23.642 | 1.00 | 23.29 | O |
| ATOM | 3801 | N | ARG | C | 61 | 29.340 | 2.965 | -20.297 | 1.00 | 19.52 | N |
| ATOM | 3802 | CA | ARG | C | 61 | 30.383 | 1.953 | -20.078 | 1.00 | 21.09 | C |
| ATOM | 3803 | C | ARG | C | 61 | 29.795 | 0.572 | -19.891 | 1.00 | 20.40 | C |
| ATOM | 3804 | O | ARG | C | 61 | 30.535 | -0.384 | -19.927 | 1.00 | 21.97 | O |
| ATOM | 3805 | CB | ARG | C | 61 | 31.286 | 2.299 | -18.879 | 1.00 | 21.83 | C |
| ATOM | 3806 | CG | ARG | C | 61 | 30.607 | 2.468 | -17.571 | 1.00 | 21.88 | C |
| ATOM | 3807 | CD | ARG | C | 61 | 31.628 | 3.041 | -16.538 | 1.00 | 22.52 | C |
| ATOM | 3808 | NE | ARG | C | 61 | 31.046 | 3.054 | -15.211 | 1.00 | 19.78 | N |
| ATOM | 3809 | CZ | ARG | C | 61 | 31.098 | 2.062 | -14.321 | 1.00 | 21.93 | C |
| ATOM | 3810 | NH1 | ARG | C | 61 | 31.759 | 0.942 | -14.591 | 1.00 | 22.48 | N |
| ATOM | 3811 | NH2 | ARG | C | 61 | 30.459 | 2.201 | -13.169 | 1.00 | 22.19 | N |
| ATOM | 3812 | N | PHE | C | 62 | 28.482 | 0.484 | -19.716 | 1.00 | 18.94 | N |
| ATOM | 3813 | CA | PHE | C | 62 | 27.783 | -0.809 | -19.739 | 1.00 | 18.56 | C |
| ATOM | 3814 | C | PHE | C | 62 | 27.323 | -1.091 | -21.174 | 1.00 | 18.54 | C |
| ATOM | 3815 | O | PHE | C | 62 | 26.745 | -0.229 | -21.843 | 1.00 | 20.51 | O |
| ATOM | 3816 | CB | PHE | C | 62 | 26.575 | -0.830 | -18.810 | 1.00 | 19.49 | C |
| ATOM | 3817 | CG | PHE | C | 62 | 26.918 | -0.762 | -17.340 | 1.00 | 19.65 | C |
| ATOM | 3818 | CD1 | PHE | C | 62 | 27.156 | 0.456 | -16.721 | 1.00 | 19.91 | C |
| ATOM | 3819 | CD2 | PHE | C | 62 | 27.002 | -1.910 | -16.574 | 1.00 | 21.03 | C |
| ATOM | 3820 | CE1 | PHE | C | 62 | 27.490 | 0.508 | -15.363 | 1.00 | 21.88 | C |
| ATOM | 3821 | CE2 | PHE | C | 62 | 27.313 | -1.840 | -15.205 | 1.00 | 19.27 | C |
| ATOM | 3822 | CZ | PHE | C | 62 | 27.546 | -0.648 | -14.615 | 1.00 | 22.46 | C |
| ATOM | 3823 | N | SER | C | 63 | 27.578 | -2.290 | -21.644 | 1.00 | 17.02 | N |
| ATOM | 3824 | CA | SER | C | 63 | 27.091 | -2.734 | -22.922 | 1.00 | 18.13 | C |
| ATOM | 3825 | C | SER | C | 63 | 26.823 | -4.223 | -22.836 | 1.00 | 16.67 | C |

```
ATOM   3826  O    SER C  63     27.275  -4.896 -21.911  1.00 17.19           O
ATOM   3827  CB   SER C  63     28.113  -2.439 -24.032  1.00 20.23           C
ATOM   3828  OG   SER C  63     29.333  -3.132 -23.855  1.00 18.97           O
ATOM   3829  N    SER C  64     26.095  -4.721 -23.812  1.00 17.18           N
ATOM   3830  CA   SER C  64     25.789  -6.177 -23.850  1.00 15.96           C
ATOM   3831  C    SER C  64     25.601  -6.669 -25.261  1.00 17.86           C
ATOM   3832  O    SER C  64     25.147  -5.938 -26.136  1.00 21.04           O
ATOM   3833  CB   SER C  64     24.519  -6.442 -23.047  1.00 18.12           C
ATOM   3834  OG   SER C  64     24.073  -7.802 -23.132  1.00 19.39           O
ATOM   3835  N    SER C  65     25.869  -7.963 -25.463  1.00 18.54           N
ATOM   3836  CA   SER C  65     25.714  -8.559 -26.760  1.00 17.77           C
ATOM   3837  C    SER C  65     25.365 -10.002 -26.571  1.00 19.55           C
ATOM   3838  O    SER C  65     25.562 -10.554 -25.487  1.00 19.78           O
ATOM   3839  CB   SER C  65     27.032  -8.445 -27.540  1.00 17.93           C
ATOM   3840  OG   SER C  65     28.142  -9.026 -26.865  1.00 20.26           O
ATOM   3841  N    GLY C  66     24.906 -10.630 -27.647  1.00 18.22           N
ATOM   3842  CA   GLY C  66     24.695 -12.053 -27.592  1.00 19.03           C
ATOM   3843  C    GLY C  66     23.966 -12.635 -28.762  1.00 20.49           C
ATOM   3844  O    GLY C  66     23.440 -11.933 -29.611  1.00 19.45           O
ATOM   3845  N    SER C  67     23.948 -13.965 -28.771  1.00 21.60           N
ATOM   3846  CA   SER C  67     23.094 -14.721 -29.685  1.00 22.44           C
ATOM   3847  C    SER C  67     21.691 -14.788 -29.069  1.00 22.90           C
ATOM   3848  O    SER C  67     21.323 -13.923 -28.235  1.00 24.51           O
ATOM   3849  CB   SER C  67     23.670 -16.110 -29.876  1.00 21.94           C
ATOM   3850  OG   SER C  67     23.650 -16.820 -28.644  1.00 23.25           O
ATOM   3851  N    GLY C  68     20.904 -15.791 -29.469  1.00 24.04           N
ATOM   3852  CA   GLY C  68     19.582 -16.006 -28.883  1.00 23.35           C
ATOM   3853  C    GLY C  68     19.662 -16.701 -27.537  1.00 23.24           C
ATOM   3854  O    GLY C  68     18.703 -16.669 -26.788  1.00 23.92           O
ATOM   3855  N    THR C  69     20.793 -17.350 -27.261  1.00 22.51           N
ATOM   3856  CA   THR C  69     20.995 -18.192 -26.060  1.00 23.66           C
ATOM   3857  C    THR C  69     22.241 -17.926 -25.213  1.00 22.00           C
ATOM   3858  O    THR C  69     22.379 -18.514 -24.124  1.00 22.07           O
ATOM   3859  CB   THR C  69     21.089 -19.668 -26.493  1.00 23.34           C
ATOM   3860  OG1  THR C  69     22.228 -19.853 -27.350  1.00 26.75           O
ATOM   3861  CG2  THR C  69     19.828 -20.079 -27.212  1.00 25.48           C
ATOM   3862  N    ASP C  70     23.154 -17.075 -25.682  1.00 21.78           N
ATOM   3863  CA   ASP C  70     24.442 -16.892 -25.056  1.00 21.74           C
ATOM   3864  C    ASP C  70     24.762 -15.398 -25.012  1.00 21.86           C
ATOM   3865  O    ASP C  70     24.943 -14.799 -26.046  1.00 21.47           O
ATOM   3866  CB   ASP C  70     25.514 -17.625 -25.860  1.00 23.32           C
ATOM   3867  CG   ASP C  70     26.855 -17.695 -25.158  1.00 28.81           C
ATOM   3868  OD1  ASP C  70     27.183 -16.816 -24.333  1.00 33.57           O
ATOM   3869  OD2  ASP C  70     27.625 -18.643 -25.456  1.00 30.49           O
ATOM   3870  N    PHE C  71     24.787 -14.817 -23.820  1.00 21.50           N
ATOM   3871  CA   PHE C  71     24.768 -13.351 -23.654  1.00 20.50           C
ATOM   3872  C    PHE C  71     25.939 -12.882 -22.799  1.00 19.72           C
ATOM   3873  O    PHE C  71     26.364 -13.558 -21.867  1.00 19.61           O
ATOM   3874  CB   PHE C  71     23.458 -12.903 -22.994  1.00 20.41           C
ATOM   3875  CG   PHE C  71     22.261 -13.721 -23.383  1.00 19.62           C
ATOM   3876  CD1  PHE C  71     21.705 -13.613 -24.661  1.00 20.28           C
ATOM   3877  CD2  PHE C  71     21.684 -14.613 -22.481  1.00 19.51           C
ATOM   3878  CE1  PHE C  71     20.609 -14.347 -25.019  1.00 20.53           C
ATOM   3879  CE2  PHE C  71     20.563 -15.346 -22.840  1.00 20.50           C
ATOM   3880  CZ   PHE C  71     20.036 -15.220 -24.108  1.00 20.91           C
ATOM   3881  N    THR C  72     26.511 -11.746 -23.133  1.00 17.75           N
ATOM   3882  CA   THR C  72     27.592 -11.212 -22.326  1.00 18.92           C
ATOM   3883  C    THR C  72     27.363  -9.726 -21.979  1.00 20.01           C
ATOM   3884  O    THR C  72     27.263  -8.867 -22.866  1.00 21.48           O
ATOM   3885  CB   THR C  72     28.952 -11.360 -23.004  1.00 20.37           C
ATOM   3886  OG1  THR C  72     29.153 -12.750 -23.360  1.00 20.19           O
ATOM   3887  CG2  THR C  72     30.027 -10.962 -22.075  1.00 20.22           C
ATOM   3888  N    LEU C  73     27.310  -9.447 -20.684  1.00 19.81           N
ATOM   3889  CA   LEU C  73     27.325  -8.063 -20.211  1.00 19.27           C
ATOM   3890  C    LEU C  73     28.758  -7.624 -20.052  1.00 19.87           C
```

93

```
ATOM   3891  O    LEU C  73      29.534  -8.257 -19.361  1.00 19.21           O
ATOM   3892  CB   LEU C  73      26.631  -7.941 -18.842  1.00 18.19           C
ATOM   3893  CG   LEU C  73      26.604  -6.555 -18.179  1.00 18.52           C
ATOM   3894  CD1  LEU C  73      25.768  -5.617 -18.954  1.00 17.50           C
ATOM   3895  CD2  LEU C  73      26.055  -6.663 -16.783  1.00 19.63           C
ATOM   3896  N    ARG C  74      29.105  -6.468 -20.607  1.00 18.17           N
ATOM   3897  CA   ARG C  74      30.442  -5.975 -20.480  1.00 19.30           C
ATOM   3898  C    ARG C  74      30.411  -4.685 -19.679  1.00 21.18           C
ATOM   3899  O    ARG C  74      29.685  -3.768 -20.016  1.00 20.55           O
ATOM   3900  CB   ARG C  74      31.046  -5.710 -21.854  1.00 21.64           C
ATOM   3901  CG   ARG C  74      32.421  -5.161 -21.788  1.00 27.69           C
ATOM   3902  CD   ARG C  74      33.027  -5.203 -23.142  1.00 32.35           C
ATOM   3903  NE   ARG C  74      34.395  -4.710 -23.162  1.00 37.51           N
ATOM   3904  CZ   ARG C  74      35.174  -4.791 -24.242  1.00 39.71           C
ATOM   3905  NH1  ARG C  74      34.713  -5.346 -25.370  1.00 40.15           N
ATOM   3906  NH2  ARG C  74      36.407  -4.314 -24.199  1.00 41.74           N
ATOM   3907  N    ILE C  75      31.226  -4.637 -18.637  1.00 20.50           N
ATOM   3908  CA   ILE C  75      31.384  -3.442 -17.817  1.00 20.80           C
ATOM   3909  C    ILE C  75      32.766  -2.905 -18.038  1.00 22.70           C
ATOM   3910  O    ILE C  75      33.748  -3.457 -17.556  1.00 20.89           O
ATOM   3911  CB   ILE C  75      31.172  -3.678 -16.297  1.00 22.21           C
ATOM   3912  CG1  ILE C  75      29.954  -4.557 -16.037  1.00 23.09           C
ATOM   3913  CG2  ILE C  75      31.118  -2.259 -15.574  1.00 22.61           C
ATOM   3914  CD1  ILE C  75      29.750  -4.975 -14.579  1.00 23.53           C
ATOM   3915  N    SER C  76      32.860  -1.809 -18.786  1.00 22.03           N
ATOM   3916  CA   SER C  76      34.137  -1.189 -19.044  1.00 24.48           C
ATOM   3917  C    SER C  76      34.491  -0.245 -17.904  1.00 23.41           C
ATOM   3918  O    SER C  76      33.626   0.238 -17.195  1.00 22.11           O
ATOM   3919  CB   SER C  76      34.109  -0.455 -20.382  1.00 26.84           C
ATOM   3920  OG   SER C  76      33.510   0.835 -20.258  1.00 31.53           O
ATOM   3921  N    ARG C  77      35.775  -0.004 -17.728  1.00 22.73           N
ATOM   3922  CA   ARG C  77      36.277   0.856 -16.683  1.00 23.39           C
ATOM   3923  C    ARG C  77      35.632   0.556 -15.318  1.00 23.09           C
ATOM   3924  O    ARG C  77      35.036   1.423 -14.673  1.00 22.78           O
ATOM   3925  CB   ARG C  77      36.096   2.322 -17.087  1.00 24.24           C
ATOM   3926  CG   ARG C  77      36.704   2.635 -18.481  1.00 28.30           C
ATOM   3927  CD   ARG C  77      36.018   3.815 -19.151  1.00 32.24           C
ATOM   3928  NE   ARG C  77      36.522   4.067 -20.507  1.00 33.63           N
ATOM   3929  CZ   ARG C  77      37.623   4.762 -20.787  1.00 35.04           C
ATOM   3930  NH1  ARG C  77      38.365   5.292 -19.818  1.00 36.73           N
ATOM   3931  NH2  ARG C  77      37.987   4.927 -22.058  1.00 36.45           N
ATOM   3932  N    VAL C  78      35.777  -0.692 -14.898  1.00 22.43           N
ATOM   3933  CA   VAL C  78      35.201  -1.124 -13.644  1.00 22.21           C
ATOM   3934  C    VAL C  78      35.696  -0.269 -12.482  1.00 21.94           C
ATOM   3935  O    VAL C  78      36.893   0.115 -12.417  1.00 20.63           O
ATOM   3936  CB   VAL C  78      35.491  -2.623 -13.423  1.00 23.86           C
ATOM   3937  CG1  VAL C  78      35.312  -3.038 -11.959  1.00 27.29           C
ATOM   3938  CG2  VAL C  78      34.610  -3.414 -14.297  1.00 24.51           C
ATOM   3939  N    GLU C  79      34.744   0.058 -11.604  1.00 21.98           N
ATOM   3940  CA   GLU C  79      34.981   0.840 -10.392  1.00 23.41           C
ATOM   3941  C    GLU C  79      34.680  -0.024  -9.181  1.00 23.35           C
ATOM   3942  O    GLU C  79      33.929  -1.012  -9.266  1.00 20.01           O
ATOM   3943  CB   GLU C  79      34.021   2.022 -10.311  1.00 26.23           C
ATOM   3944  CG   GLU C  79      34.123   2.989 -11.418  1.00 29.13           C
ATOM   3945  CD   GLU C  79      32.967   3.911 -11.459  1.00 30.70           C
ATOM   3946  OE1  GLU C  79      32.082   3.865 -10.560  1.00 31.77           O
ATOM   3947  OE2  GLU C  79      32.912   4.671 -12.440  1.00 35.27           O
ATOM   3948  N    ALA C  80      35.225   0.398  -8.046  1.00 24.54           N
ATOM   3949  CA   ALA C  80      35.002  -0.291  -6.768  1.00 24.52           C
ATOM   3950  C    ALA C  80      33.515  -0.404  -6.464  1.00 24.60           C
ATOM   3951  O    ALA C  80      33.031  -1.430  -5.972  1.00 22.74           O
ATOM   3952  CB   ALA C  80      35.716   0.448  -5.650  1.00 25.07           C
ATOM   3953  N    GLU C  81      32.780   0.668  -6.767  1.00 23.47           N
ATOM   3954  CA   GLU C  81      31.328   0.719  -6.537  1.00 23.79           C
ATOM   3955  C    GLU C  81      30.463  -0.203  -7.418  1.00 22.18           C
```

```
ATOM  3956  O    GLU C  81   29.253  -0.288  -7.206  1.00 20.79       O
ATOM  3957  CB   GLU C  81   30.875   2.194  -6.679  1.00 24.93       C
ATOM  3958  CG   GLU C  81   29.376   2.477  -6.683  1.00 27.69       C
ATOM  3959  CD   GLU C  81   28.710   2.312  -5.323  1.00 31.58       C
ATOM  3960  OE1  GLU C  81   29.426   2.407  -4.291  1.00 34.19       O
ATOM  3961  OE2  GLU C  81   27.467   2.069  -5.286  1.00 34.69       O
ATOM  3962  N    ASP C  82   31.067  -0.913  -8.376  1.00 21.27       N
ATOM  3963  CA   ASP C  82   30.330  -1.794  -9.285  1.00 21.85       C
ATOM  3964  C    ASP C  82   30.103  -3.138  -8.599  1.00 19.36       C
ATOM  3965  O    ASP C  82   29.476  -4.033  -9.163  1.00 19.14       O
ATOM  3966  CB   ASP C  82   31.081  -2.032 -10.605  1.00 21.32       C
ATOM  3967  CG   ASP C  82   31.067  -0.822 -11.529  1.00 24.30       C
ATOM  3968  OD1  ASP C  82   30.079  -0.059 -11.514  1.00 22.16       O
ATOM  3969  OD2  ASP C  82   32.064  -0.637 -12.266  1.00 22.84       O
ATOM  3970  N    VAL C  83   30.627  -3.307  -7.388  1.00 19.41       N
ATOM  3971  CA   VAL C  83   30.363  -4.592  -6.719  1.00 19.88       C
ATOM  3972  C    VAL C  83   28.871  -4.773  -6.520  1.00 19.72       C
ATOM  3973  O    VAL C  83   28.144  -3.849  -6.170  1.00 19.01       O
ATOM  3974  CB   VAL C  83   31.122  -4.813  -5.382  1.00 20.17       C
ATOM  3975  CG1  VAL C  83   32.603  -4.990  -5.657  1.00 21.37       C
ATOM  3976  CG2  VAL C  83   30.877  -3.696  -4.417  1.00 19.80       C
ATOM  3977  N    GLY C  84   28.427  -6.014  -6.714  1.00 17.47       N
ATOM  3978  CA   GLY C  84   27.055  -6.368  -6.600  1.00 17.89       C
ATOM  3979  C    GLY C  84   26.831  -7.648  -7.379  1.00 18.38       C
ATOM  3980  O    GLY C  84   27.789  -8.229  -7.893  1.00 19.07       O
ATOM  3981  N    ILE C  85   25.558  -8.016  -7.500  1.00 18.90       N
ATOM  3982  CA   ILE C  85   25.127  -9.220  -8.208  1.00 17.95       C
ATOM  3983  C    ILE C  85   24.329  -8.829  -9.471  1.00 18.69       C
ATOM  3984  O    ILE C  85   23.301  -8.117  -9.384  1.00 16.48       O
ATOM  3985  CB   ILE C  85   24.282 -10.124  -7.298  1.00 18.76       C
ATOM  3986  CG1  ILE C  85   25.120 -10.499  -6.058  1.00 21.65       C
ATOM  3987  CG2  ILE C  85   23.854 -11.381  -8.069  1.00 19.64       C
ATOM  3988  CD1  ILE C  85   24.387 -11.321  -5.049  1.00 21.31       C
ATOM  3989  N    TYR C  86   24.788  -9.339 -10.619  1.00 17.11       N
ATOM  3990  CA   TYR C  86   24.269  -8.983 -11.945  1.00 14.82       C
ATOM  3991  C    TYR C  86   23.437 -10.158 -12.400  1.00 16.93       C
ATOM  3992  O    TYR C  86   23.972 -11.250 -12.414  1.00 18.82       O
ATOM  3993  CB   TYR C  86   25.444  -8.742 -12.906  1.00 15.79       C
ATOM  3994  CG   TYR C  86   26.187  -7.471 -12.546  1.00 17.15       C
ATOM  3995  CD1  TYR C  86   27.174  -7.460 -11.567  1.00 16.33       C
ATOM  3996  CD2  TYR C  86   25.878  -6.274 -13.167  1.00 16.80       C
ATOM  3997  CE1  TYR C  86   27.812  -6.275 -11.199  1.00 18.61       C
ATOM  3998  CE2  TYR C  86   26.507  -5.115 -12.826  1.00 19.68       C
ATOM  3999  CZ   TYR C  86   27.487  -5.106 -11.860  1.00 19.13       C
ATOM  4000  OH   TYR C  86   28.100  -3.936 -11.502  1.00 19.10       O
ATOM  4001  N    TYR C  87   22.157  -9.929 -12.721  1.00 17.25       N
ATOM  4002  CA   TYR C  87   21.185 -10.970 -13.082  1.00 15.84       C
ATOM  4003  C    TYR C  87   20.703 -10.804 -14.515  1.00 16.93       C
ATOM  4004  O    TYR C  87   20.397  -9.682 -14.926  1.00 17.29       O
ATOM  4005  CB   TYR C  87   19.926 -10.956 -12.188  1.00 16.03       C
ATOM  4006  CG   TYR C  87   20.154 -11.180 -10.732  1.00 18.73       C
ATOM  4007  CD1  TYR C  87   20.054 -12.451 -10.193  1.00 19.70       C
ATOM  4008  CD2  TYR C  87   20.401 -10.128  -9.862  1.00 20.50       C
ATOM  4009  CE1  TYR C  87   20.260 -12.669  -8.849  1.00 20.52       C
ATOM  4010  CE2  TYR C  87   20.615 -10.352  -8.512  1.00 21.00       C
ATOM  4011  CZ   TYR C  87   20.510 -11.620  -8.003  1.00 21.38       C
ATOM  4012  OH   TYR C  87   20.704 -11.855  -6.641  1.00 22.14       O
ATOM  4013  N    CYS C  88   20.591 -11.929 -15.231  1.00 18.14       N
ATOM  4014  CA   CYS C  88   19.910 -11.988 -16.512  1.00 19.18       C
ATOM  4015  C    CYS C  88   18.459 -12.438 -16.290  1.00 18.99       C
ATOM  4016  O    CYS C  88   18.124 -13.073 -15.265  1.00 19.77       O
ATOM  4017  CB   CYS C  88   20.652 -12.867 -17.497  1.00 20.68       C
ATOM  4018  SG   CYS C  88   20.854 -14.546 -16.883  1.00 21.50       S
ATOM  4019  N    ALA C  89   17.596 -12.048 -17.227  1.00 18.92       N
ATOM  4020  CA   ALA C  89   16.186 -12.370 -17.229  1.00 20.04       C
```

95

| ATOM | 4021 | C   | ALA | C | 89 | 15.684 | -12.434 | -18.667 | 1.00 | 19.23 | C |
|------|------|-----|-----|---|----|--------|---------|---------|------|-------|---|
| ATOM | 4022 | O   | ALA | C | 89 | 16.136 | -11.641 | -19.491 | 1.00 | 19.83 | O |
| ATOM | 4023 | CB  | ALA | C | 89 | 15.380 | -11.337 | -16.476 | 1.00 | 21.22 | C |
| ATOM | 4024 | N   | HIS | C | 90 | 14.748 | -13.348 | -18.942 | 1.00 | 19.39 | N |
| ATOM | 4025 | CA  | HIS | C | 90 | 14.009 | -13.339 | -20.204 | 1.00 | 20.91 | C |
| ATOM | 4026 | C   | HIS | C | 90 | 12.640 | -12.625 | -20.093 | 1.00 | 20.98 | C |
| ATOM | 4027 | O   | HIS | C | 90 | 12.097 | -12.390 | -18.990 | 1.00 | 21.57 | O |
| ATOM | 4028 | CB  | HIS | C | 90 | 13.838 | -14.784 | -20.704 | 1.00 | 20.54 | C |
| ATOM | 4029 | CG  | HIS | C | 90 | 12.722 | -15.509 | -20.036 | 1.00 | 19.68 | C |
| ATOM | 4030 | ND1 | HIS | C | 90 | 12.913 | -16.399 | -19.003 | 1.00 | 23.51 | N |
| ATOM | 4031 | CD2 | HIS | C | 90 | 11.390 | -15.436 | -20.225 | 1.00 | 18.62 | C |
| ATOM | 4032 | CE1 | HIS | C | 90 | 11.743 | -16.864 | -18.609 | 1.00 | 21.04 | C |
| ATOM | 4033 | NE2 | HIS | C | 90 | 10.802 | -16.271 | -19.315 | 1.00 | 22.04 | N |
| ATOM | 4034 | N   | ASN | C | 91 | 12.084 | -12.262 | -21.236 | 1.00 | 21.50 | N |
| ATOM | 4035 | CA  | ASN | C | 91 | 10.699 | -11.775 | -21.333 | 1.00 | 22.06 | C |
| ATOM | 4036 | C   | ASN | C | 91 | 9.990  | -12.372 | -22.573 | 1.00 | 23.19 | C |
| ATOM | 4037 | O   | ASN | C | 91 | 9.483  | -11.657 | -23.413 | 1.00 | 25.44 | O |
| ATOM | 4038 | CB  | ASN | C | 91 | 10.686 | -10.238 | -21.365 | 1.00 | 22.19 | C |
| ATOM | 4039 | CG  | ASN | C | 91 | 9.282  | -9.641  | -21.319 | 1.00 | 22.26 | C |
| ATOM | 4040 | OD1 | ASN | C | 91 | 9.076  | -8.510  | -21.760 | 1.00 | 27.58 | O |
| ATOM | 4041 | ND2 | ASN | C | 91 | 8.317  | -10.390 | -20.788 | 1.00 | 19.12 | N |
| ATOM | 4042 | N   | VAL | C | 92 | 9.943  | -13.694 | -22.649 | 1.00 | 24.16 | N |
| ATOM | 4043 | CA  | VAL | C | 92 | 9.355  | -14.413 | -23.793 | 1.00 | 25.50 | C |
| ATOM | 4044 | C   | VAL | C | 92 | 8.251  | -15.378 | -23.412 | 1.00 | 27.32 | C |
| ATOM | 4045 | O   | VAL | C | 92 | 7.609  | -15.941 | -24.296 | 1.00 | 28.42 | O |
| ATOM | 4046 | CB  | VAL | C | 92 | 10.405 | -15.212 | -24.619 | 1.00 | 24.86 | C |
| ATOM | 4047 | CG1 | VAL | C | 92 | 11.504 | -14.314 | -25.087 | 1.00 | 24.56 | C |
| ATOM | 4048 | CG2 | VAL | C | 92 | 10.966 | -16.431 | -23.870 | 1.00 | 24.86 | C |
| ATOM | 4049 | N   | GLU | C | 93 | 8.050  | -15.601 | -22.117 | 1.00 | 27.28 | N |
| ATOM | 4050 | CA  | GLU | C | 93 | 6.901  | -16.368 | -21.646 | 1.00 | 28.07 | C |
| ATOM | 4051 | C   | GLU | C | 93 | 6.746  | -16.231 | -20.147 | 1.00 | 27.84 | C |
| ATOM | 4052 | O   | GLU | C | 93 | 7.604  | -15.637 | -19.457 | 1.00 | 26.93 | O |
| ATOM | 4053 | CB  | GLU | C | 93 | 7.092  | -17.843 | -21.976 | 1.00 | 28.18 | C |
| ATOM | 4054 | CG  | GLU | C | 93 | 8.261  | -18.479 | -21.255 | 1.00 | 30.16 | C |
| ATOM | 4055 | CD  | GLU | C | 93 | 8.454  | -19.910 | -21.636 | 1.00 | 30.08 | C |
| ATOM | 4056 | OE1 | GLU | C | 93 | 8.260  | -20.218 | -22.830 | 1.00 | 34.00 | O |
| ATOM | 4057 | OE2 | GLU | C | 93 | 8.824  | -20.703 | -20.753 | 1.00 | 33.11 | O |
| ATOM | 4058 | N   | LEU | C | 94 | 5.641  | -16.782 | -19.645 | 1.00 | 28.37 | N |
| ATOM | 4059 | CA  | LEU | C | 94 | 5.494  | -17.028 | -18.225 | 1.00 | 27.74 | C |
| ATOM | 4060 | C   | LEU | C | 94 | 5.716  | -18.526 | -17.984 | 1.00 | 28.25 | C |
| ATOM | 4061 | O   | LEU | C | 94 | 5.314  | -19.338 | -18.807 | 1.00 | 30.70 | O |
| ATOM | 4062 | CB  | LEU | C | 94 | 4.123  | -16.599 | -17.756 | 1.00 | 27.64 | C |
| ATOM | 4063 | CG  | LEU | C | 94 | 3.794  | -15.144 | -18.070 | 1.00 | 28.23 | C |
| ATOM | 4064 | CD1 | LEU | C | 94 | 2.437  | -14.849 | -17.556 | 1.00 | 28.06 | C |
| ATOM | 4065 | CD2 | LEU | C | 94 | 4.775  | -14.202 | -17.459 | 1.00 | 27.54 | C |
| ATOM | 4066 | N   | PRO | C | 95 | 6.359  | -18.896 | -16.863 | 1.00 | 28.35 | N |
| ATOM | 4067 | CA  | PRO | C | 95 | 6.758  | -17.999 | -15.785 | 1.00 | 26.88 | C |
| ATOM | 4068 | C   | PRO | C | 95 | 7.972  | -17.189 | -16.138 | 1.00 | 26.16 | C |
| ATOM | 4069 | O   | PRO | C | 95 | 8.799  | -17.610 | -16.951 | 1.00 | 25.15 | O |
| ATOM | 4070 | CB  | PRO | C | 95 | 7.135  | -18.954 | -14.657 | 1.00 | 28.27 | C |
| ATOM | 4071 | CG  | PRO | C | 95 | 7.672  | -20.163 | -15.397 | 1.00 | 28.19 | C |
| ATOM | 4072 | CD  | PRO | C | 95 | 6.764  | -20.288 | -16.581 | 1.00 | 27.68 | C |
| ATOM | 4073 | N   | ARG | C | 96 | 8.083  | -16.037 | -15.492 | 1.00 | 25.00 | N |
| ATOM | 4074 | CA  | ARG | C | 96 | 9.265  | -15.239 | -15.627 | 1.00 | 23.01 | C |
| ATOM | 4075 | C   | ARG | C | 96 | 10.363 | -15.944 | -14.845 | 1.00 | 22.20 | C |
| ATOM | 4076 | O   | ARG | C | 96 | 10.131 | -16.385 | -13.729 | 1.00 | 25.35 | O |
| ATOM | 4077 | CB  | ARG | C | 96 | 8.996  | -13.861 | -15.050 | 1.00 | 22.52 | C |
| ATOM | 4078 | CG  | ARG | C | 96 | 7.838  | -13.167 | -15.668 | 1.00 | 22.98 | C |
| ATOM | 4079 | CD  | ARG | C | 96 | 7.843  | -11.716 | -15.271 | 1.00 | 24.06 | C |
| ATOM | 4080 | NE  | ARG | C | 96 | 6.721  | -11.059 | -15.916 | 1.00 | 24.15 | N |
| ATOM | 4081 | CZ  | ARG | C | 96 | 6.720  | -10.671 | -17.181 | 1.00 | 24.54 | C |
| ATOM | 4082 | NH1 | ARG | C | 96 | 7.810  | -10.832 | -17.922 | 1.00 | 24.92 | N |
| ATOM | 4083 | NH2 | ARG | C | 96 | 5.646  | -10.097 | -17.696 | 1.00 | 24.37 | N |
| ATOM | 4084 | N   | THR | C | 97 | 11.543 | -16.099 | -15.439 | 1.00 | 21.81 | N |
| ATOM | 4085 | CA  | THR | C | 97 | 12.706 | -16.635 | -14.725 | 1.00 | 20.97 | C |

```
ATOM   4086  C    THR C  97      13.977 -15.825 -14.888  1.00 21.91           C
ATOM   4087  O    THR C  97      14.150 -15.077 -15.847  1.00 20.95           O
ATOM   4088  CB   THR C  97      13.020 -18.108 -15.141  1.00 22.63           C
ATOM   4089  OG1  THR C  97      13.235 -18.169 -16.552  1.00 23.26           O
ATOM   4090  CG2  THR C  97      11.870 -18.991 -14.764  1.00 20.62           C
ATOM   4091  N    PHE C  98      14.886 -16.053 -13.945  1.00 21.38           N
ATOM   4092  CA   PHE C  98      16.087 -15.270 -13.755  1.00 21.12           C
ATOM   4093  C    PHE C  98      17.261 -16.195 -13.650  1.00 21.10           C
ATOM   4094  O    PHE C  98      17.138 -17.348 -13.212  1.00 19.99           O
ATOM   4095  CB   PHE C  98      15.980 -14.449 -12.462  1.00 19.90           C
ATOM   4096  CG   PHE C  98      14.810 -13.505 -12.449  1.00 21.52           C
ATOM   4097  CD1  PHE C  98      13.549 -13.919 -12.051  1.00 21.65           C
ATOM   4098  CD2  PHE C  98      14.979 -12.198 -12.862  1.00 21.22           C
ATOM   4099  CE1  PHE C  98      12.505 -13.049 -12.048  1.00 20.85           C
ATOM   4100  CE2  PHE C  98      13.908 -11.318 -12.856  1.00 21.76           C
ATOM   4101  CZ   PHE C  98      12.669 -11.764 -12.442  1.00 20.64           C
ATOM   4102  N    GLY C  99      18.437 -15.686 -14.000  1.00 20.15           N
ATOM   4103  CA   GLY C  99      19.626 -16.456 -13.751  1.00 20.32           C
ATOM   4104  C    GLY C  99      20.005 -16.274 -12.286  1.00 20.18           C
ATOM   4105  O    GLY C  99      19.293 -15.569 -11.545  1.00 19.33           O
ATOM   4106  N    GLY C 100      21.013 -17.023 -11.840  1.00 20.75           N
ATOM   4107  CA   GLY C 100      21.210 -17.259 -10.398  1.00 20.18           C
ATOM   4108  C    GLY C 100      21.999 -16.031  -9.889  1.00 19.57           C
ATOM   4109  O    GLY C 100      22.173 -15.839  -8.679  1.00 17.37           O
ATOM   4110  N    GLY C 101      22.488 -15.187 -10.819  1.00 18.79           N
ATOM   4111  CA   GLY C 101      23.252 -13.993 -10.478  1.00 19.08           C
ATOM   4112  C    GLY C 101      24.759 -14.248 -10.398  1.00 19.96           C
ATOM   4113  O    GLY C 101      25.211 -15.267  -9.861  1.00 20.49           O
ATOM   4114  N    THR C 102      25.551 -13.328 -10.930  1.00 20.36           N
ATOM   4115  CA   THR C 102      26.993 -13.394 -10.832  1.00 18.67           C
ATOM   4116  C    THR C 102      27.493 -12.232 -10.001  1.00 19.87           C
ATOM   4117  O    THR C 102      27.249 -11.081 -10.350  1.00 16.93           O
ATOM   4118  CB   THR C 102      27.621 -13.324 -12.219  1.00 19.27           C
ATOM   4119  OG1  THR C 102      27.110 -14.422 -13.020  1.00 19.36           O
ATOM   4120  CG2  THR C 102      29.130 -13.367 -12.136  1.00 19.93           C
ATOM   4121  N    LYS C 103      28.223 -12.524  -8.936  1.00 18.58           N
ATOM   4122  CA   LYS C 103      28.785 -11.483  -8.111  1.00 19.64           C
ATOM   4123  C    LYS C 103      30.061 -10.969  -8.724  1.00 19.21           C
ATOM   4124  O    LYS C 103      30.989 -11.738  -9.007  1.00 19.98           O
ATOM   4125  CB   LYS C 103      29.068 -12.009  -6.709  1.00 19.59           C
ATOM   4126  CG   LYS C 103      29.491 -10.915  -5.739  1.00 23.60           C
ATOM   4127  CD   LYS C 103      29.660 -11.533  -4.349  1.00 26.69           C
ATOM   4128  CE   LYS C 103      28.306 -11.666  -3.706  1.00 28.54           C
ATOM   4129  NZ   LYS C 103      28.383 -12.292  -2.345  1.00 31.78           N
ATOM   4130  N    LEU C 104      30.144  -9.661  -8.922  1.00 18.13           N
ATOM   4131  CA   LEU C 104      31.353  -9.059  -9.416  1.00 19.96           C
ATOM   4132  C    LEU C 104      32.289  -8.830  -8.237  1.00 20.67           C
ATOM   4133  O    LEU C 104      31.905  -8.188  -7.238  1.00 21.22           O
ATOM   4134  CB   LEU C 104      31.068  -7.710 -10.099  1.00 19.41           C
ATOM   4135  CG   LEU C 104      32.206  -6.921 -10.728  1.00 21.49           C
ATOM   4136  CD1  LEU C 104      32.851  -7.699 -11.833  1.00 25.46           C
ATOM   4137  CD2  LEU C 104      31.622  -5.659 -11.345  1.00 25.33           C
ATOM   4138  N    GLU C 105      33.510  -9.341  -8.353  1.00 21.97           N
ATOM   4139  CA   GLU C 105      34.553  -9.087  -7.368  1.00 24.06           C
ATOM   4140  C    GLU C 105      35.638  -8.181  -7.952  1.00 23.11           C
ATOM   4141  O    GLU C 105      36.086  -8.397  -9.057  1.00 23.19           O
ATOM   4142  CB   GLU C 105      35.201 -10.389  -6.894  1.00 25.49           C
ATOM   4143  CG   GLU C 105      36.134 -10.125  -5.688  1.00 28.99           C
ATOM   4144  CD   GLU C 105      36.849 -11.347  -5.140  1.00 30.90           C
ATOM   4145  OE1  GLU C 105      36.369 -12.474  -5.354  1.00 32.70           O
ATOM   4146  OE2  GLU C 105      37.891 -11.143  -4.482  1.00 34.01           O
ATOM   4147  N    ILE C 106      36.062  -7.196  -7.184  1.00 23.81           N
ATOM   4148  CA   ILE C 106      37.170  -6.324  -7.563  1.00 24.58           C
ATOM   4149  C    ILE C 106      38.507  -6.861  -7.010  1.00 26.49           C
ATOM   4150  O    ILE C 106      38.585  -7.333  -5.856  1.00 25.68           O
```

| ATOM | 4151 | CB | ILE | C | 106 | 36.922 | -4.892 | -7.007 | 1.00 | 25.18 | C |
|------|------|-----|-----|---|-----|--------|--------|--------|------|-------|---|
| ATOM | 4152 | CG1 | ILE | C | 106 | 35.582 | -4.342 | -7.499 | 1.00 | 27.60 | C |
| ATOM | 4153 | CG2 | ILE | C | 106 | 38.102 | -3.949 | -7.265 | 1.00 | 27.24 | C |
| ATOM | 4154 | CD1 | ILE | C | 106 | 35.402 | -4.279 | -8.994 | 1.00 | 29.24 | C |
| ATOM | 4155 | N | LYS | C | 107 | 39.546 | -6.775 | -7.840 | 1.00 | 26.78 | N |
| ATOM | 4156 | CA | LYS | C | 107 | 40.917 | -7.064 | -7.438 | 1.00 | 26.83 | C |
| ATOM | 4157 | C | LYS | C | 107 | 41.578 | -5.830 | -6.869 | 1.00 | 27.05 | C |
| ATOM | 4158 | O | LYS | C | 107 | 41.484 | -4.727 | -7.421 | 1.00 | 26.43 | O |
| ATOM | 4159 | CB | LYS | C | 107 | 41.726 | -7.588 | -8.612 | 1.00 | 28.32 | C |
| ATOM | 4160 | CG | LYS | C | 107 | 43.009 | -8.271 | -8.198 | 1.00 | 30.45 | C |
| ATOM | 4161 | CD | LYS | C | 107 | 43.654 | -8.987 | -9.369 | 1.00 | 32.43 | C |
| ATOM | 4162 | CE | LYS | C | 107 | 45.126 | -9.315 | -9.076 | 1.00 | 34.02 | C |
| ATOM | 4163 | NZ | LYS | C | 107 | 45.945 | -9.409 | -10.326 | 1.00 | 35.29 | N |
| ATOM | 4164 | N | ARG | C | 108 | 42.249 | -6.023 | -5.739 | 1.00 | 24.97 | N |
| ATOM | 4165 | CA | ARG | C | 108 | 43.058 | -5.012 | -5.171 | 1.00 | 24.30 | C |
| ATOM | 4166 | C | ARG | C | 108 | 44.297 | -5.633 | -4.551 | 1.00 | 23.03 | C |
| ATOM | 4167 | O | ARG | C | 108 | 44.489 | -6.866 | -4.592 | 1.00 | 23.28 | O |
| ATOM | 4168 | CB | ARG | C | 108 | 42.240 | -4.185 | -4.170 | 1.00 | 24.30 | C |
| ATOM | 4169 | CG | ARG | C | 108 | 41.669 | -4.975 | -3.015 | 1.00 | 24.54 | C |
| ATOM | 4170 | CD | ARG | C | 108 | 41.544 | -4.140 | -1.764 | 1.00 | 24.05 | C |
| ATOM | 4171 | NE | ARG | C | 108 | 42.851 | -3.896 | -1.172 | 1.00 | 25.66 | N |
| ATOM | 4172 | CZ | ARG | C | 108 | 43.169 | -2.824 | -0.464 | 1.00 | 25.65 | C |
| ATOM | 4173 | NH1 | ARG | C | 108 | 42.302 | -1.865 | -0.277 | 1.00 | 27.94 | N |
| ATOM | 4174 | NH2 | ARG | C | 108 | 44.391 | -2.705 | 0.030 | 1.00 | 28.73 | N |
| ATOM | 4175 | N | ALA | C | 109 | 45.162 | -4.773 | -4.040 | 1.00 | 24.91 | N |
| ATOM | 4176 | CA | ALA | C | 109 | 46.406 | -5.188 | -3.410 | 1.00 | 24.99 | C |
| ATOM | 4177 | C | ALA | C | 109 | 46.080 | -5.930 | -2.125 | 1.00 | 24.21 | C |
| ATOM | 4178 | O | ALA | C | 109 | 45.095 | -5.632 | -1.458 | 1.00 | 22.26 | O |
| ATOM | 4179 | CB | ALA | C | 109 | 47.248 | -3.979 | -3.079 | 1.00 | 25.89 | C |
| ATOM | 4180 | N | ASP | C | 110 | 46.909 | -6.910 | -1.796 | 1.00 | 26.33 | N |
| ATOM | 4181 | CA | ASP | C | 110 | 46.708 | -7.693 | -0.599 | 1.00 | 26.10 | C |
| ATOM | 4182 | C | ASP | C | 110 | 46.728 | -6.755 | 0.599 | 1.00 | 26.47 | C |
| ATOM | 4183 | O | ASP | C | 110 | 47.473 | -5.748 | 0.631 | 1.00 | 24.56 | O |
| ATOM | 4184 | CB | ASP | C | 110 | 47.779 | -8.762 | -0.487 | 1.00 | 27.67 | C |
| ATOM | 4185 | CG | ASP | C | 110 | 47.586 | -9.902 | -1.461 | 1.00 | 27.48 | C |
| ATOM | 4186 | OD1 | ASP | C | 110 | 46.577 | -9.983 | -2.185 | 1.00 | 30.13 | O |
| ATOM | 4187 | OD2 | ASP | C | 110 | 48.450 | -10.793 | -1.461 | 1.00 | 34.38 | O |
| ATOM | 4188 | N | ALA | C | 111 | 45.899 | -7.080 | 1.585 | 1.00 | 26.28 | N |
| ATOM | 4189 | CA | ALA | C | 111 | 45.793 | -6.302 | 2.801 | 1.00 | 25.49 | C |
| ATOM | 4190 | C | ALA | C | 111 | 45.626 | -7.260 | 3.979 | 1.00 | 25.97 | C |
| ATOM | 4191 | O | ALA | C | 111 | 44.773 | -8.149 | 3.967 | 1.00 | 23.78 | O |
| ATOM | 4192 | CB | ALA | C | 111 | 44.633 | -5.344 | 2.722 | 1.00 | 26.42 | C |
| ATOM | 4193 | N | ALA | C | 112 | 46.474 | -7.059 | 4.979 | 1.00 | 25.55 | N |
| ATOM | 4194 | CA | ALA | C | 112 | 46.462 | -7.867 | 6.190 | 1.00 | 25.46 | C |
| ATOM | 4195 | C | ALA | C | 112 | 45.251 | -7.465 | 7.024 | 1.00 | 24.40 | C |
| ATOM | 4196 | O | ALA | C | 112 | 44.911 | -6.289 | 7.090 | 1.00 | 22.66 | O |
| ATOM | 4197 | CB | ALA | C | 112 | 47.768 | -7.608 | 6.975 | 1.00 | 24.79 | C |
| ATOM | 4198 | N | PRO | C | 113 | 44.611 | -8.434 | 7.707 | 1.00 | 22.29 | N |
| ATOM | 4199 | CA | PRO | C | 113 | 43.514 | -7.970 | 8.541 | 1.00 | 22.53 | C |
| ATOM | 4200 | C | PRO | C | 113 | 43.987 | -7.238 | 9.781 | 1.00 | 22.07 | C |
| ATOM | 4201 | O | PRO | C | 113 | 45.068 | -7.522 | 10.325 | 1.00 | 23.95 | O |
| ATOM | 4202 | CB | PRO | C | 113 | 42.805 | -9.261 | 8.936 | 1.00 | 23.04 | C |
| ATOM | 4203 | CG | PRO | C | 113 | 43.855 | -10.254 | 8.936 | 1.00 | 23.24 | C |
| ATOM | 4204 | CD | PRO | C | 113 | 44.824 | -9.881 | 7.837 | 1.00 | 23.92 | C |
| ATOM | 4205 | N | THR | C | 114 | 43.211 | -6.265 | 10.213 | 1.00 | 20.49 | N |
| ATOM | 4206 | CA | THR | C | 114 | 43.405 | -5.712 | 11.539 | 1.00 | 19.85 | C |
| ATOM | 4207 | C | THR | C | 114 | 42.564 | -6.527 | 12.516 | 1.00 | 18.16 | C |
| ATOM | 4208 | O | THR | C | 114 | 41.361 | -6.546 | 12.397 | 1.00 | 19.10 | O |
| ATOM | 4209 | CB | THR | C | 114 | 42.944 | -4.269 | 11.599 | 1.00 | 21.90 | C |
| ATOM | 4210 | OG1 | THR | C | 114 | 43.685 | -3.524 | 10.623 | 1.00 | 27.19 | O |
| ATOM | 4211 | CG2 | THR | C | 114 | 43.186 | -3.714 | 12.933 | 1.00 | 22.67 | C |
| ATOM | 4212 | N | VAL | C | 115 | 43.226 | -7.204 | 13.449 | 1.00 | 19.67 | N |
| ATOM | 4213 | CA | VAL | C | 115 | 42.544 | -8.096 | 14.371 | 1.00 | 17.90 | C |
| ATOM | 4214 | C | VAL | C | 115 | 42.407 | -7.522 | 15.768 | 1.00 | 19.87 | C |
| ATOM | 4215 | O | VAL | C | 115 | 43.362 | -7.002 | 16.325 | 1.00 | 18.58 | O |

| ATOM | 4216 | CB | VAL C 115 | 43.288 | -9.413 | 14.421 | 1.00 | 17.61 | C |
|------|------|-----|-----------|--------|--------|--------|------|-------|---|
| ATOM | 4217 | CG1 | VAL C 115 | 42.479 | -10.439 | 15.237 | 1.00 | 19.68 | C |
| ATOM | 4218 | CG2 | VAL C 115 | 43.583 | -9.873 | 13.016 | 1.00 | 17.42 | C |
| ATOM | 4219 | N | SER C 116 | 41.185 | -7.582 | 16.313 | 1.00 | 18.44 | N |
| ATOM | 4220 | CA | SER C 116 | 40.835 | -6.998 | 17.601 | 1.00 | 18.88 | C |
| ATOM | 4221 | C | SER C 116 | 40.075 | -8.087 | 18.356 | 1.00 | 17.47 | C |
| ATOM | 4222 | O | SER C 116 | 39.207 | -8.673 | 17.783 | 1.00 | 17.94 | O |
| ATOM | 4223 | CB | SER C 116 | 39.926 | -5.768 | 17.431 | 1.00 | 20.33 | C |
| ATOM | 4224 | OG | SER C 116 | 40.634 | -4.806 | 16.695 | 1.00 | 24.54 | O |
| ATOM | 4225 | N | ILE C 117 | 40.421 | -8.312 | 19.620 | 1.00 | 16.59 | N |
| ATOM | 4226 | CA | ILE C 117 | 39.750 | -9.298 | 20.472 | 1.00 | 17.41 | C |
| ATOM | 4227 | C | ILE C 117 | 39.095 | -8.583 | 21.665 | 1.00 | 17.40 | C |
| ATOM | 4228 | O | ILE C 117 | 39.609 | -7.554 | 22.187 | 1.00 | 18.00 | O |
| ATOM | 4229 | CB | ILE C 117 | 40.725 | -10.406 | 20.893 | 1.00 | 16.89 | C |
| ATOM | 4230 | CG1 | ILE C 117 | 39.982 | -11.649 | 21.456 | 1.00 | 16.67 | C |
| ATOM | 4231 | CG2 | ILE C 117 | 41.750 | -9.875 | 21.916 | 1.00 | 17.19 | C |
| ATOM | 4232 | CD1 | ILE C 117 | 40.812 | -12.861 | 21.585 | 1.00 | 17.29 | C |
| ATOM | 4233 | N | PHE C 118 | 37.972 | -9.130 | 22.139 | 1.00 | 15.30 | N |
| ATOM | 4234 | CA | PHE C 118 | 37.185 | -8.530 | 23.206 | 1.00 | 15.52 | C |
| ATOM | 4235 | C | PHE C 118 | 36.706 | -9.597 | 24.153 | 1.00 | 16.47 | C |
| ATOM | 4236 | O | PHE C 118 | 36.159 | -10.586 | 23.705 | 1.00 | 16.86 | O |
| ATOM | 4237 | CB | PHE C 118 | 36.003 | -7.823 | 22.596 | 1.00 | 16.41 | C |
| ATOM | 4238 | CG | PHE C 118 | 36.411 | -6.748 | 21.637 | 1.00 | 15.15 | C |
| ATOM | 4239 | CD1 | PHE C 118 | 36.587 | -7.016 | 20.303 | 1.00 | 17.80 | C |
| ATOM | 4240 | CD2 | PHE C 118 | 36.680 | -5.471 | 22.096 | 1.00 | 19.75 | C |
| ATOM | 4241 | CE1 | PHE C 118 | 37.023 | -6.046 | 19.440 | 1.00 | 21.01 | C |
| ATOM | 4242 | CE2 | PHE C 118 | 37.113 | -4.486 | 21.226 | 1.00 | 17.80 | C |
| ATOM | 4243 | CZ | PHE C 118 | 37.269 | -4.766 | 19.893 | 1.00 | 21.64 | C |
| ATOM | 4244 | N | PRO C 119 | 36.877 | -9.384 | 25.459 | 1.00 | 16.69 | N |
| ATOM | 4245 | CA | PRO C 119 | 36.435 | -10.356 | 26.434 | 1.00 | 15.82 | C |
| ATOM | 4246 | C | PRO C 119 | 34.956 | -10.230 | 26.704 | 1.00 | 16.64 | C |
| ATOM | 4247 | O | PRO C 119 | 34.331 | -9.200 | 26.376 | 1.00 | 17.96 | O |
| ATOM | 4248 | CB | PRO C 119 | 37.157 | -9.924 | 27.698 | 1.00 | 16.03 | C |
| ATOM | 4249 | CG | PRO C 119 | 37.196 | -8.439 | 27.538 | 1.00 | 17.88 | C |
| ATOM | 4250 | CD | PRO C 119 | 37.465 | -8.204 | 26.120 | 1.00 | 18.85 | C |
| ATOM | 4251 | N | PRO C 120 | 34.389 | -11.234 | 27.396 | 1.00 | 17.79 | N |
| ATOM | 4252 | CA | PRO C 120 | 33.022 | -11.158 | 27.848 | 1.00 | 18.31 | C |
| ATOM | 4253 | C | PRO C 120 | 32.750 | -9.932 | 28.695 | 1.00 | 16.96 | C |
| ATOM | 4254 | O | PRO C 120 | 33.500 | -9.614 | 29.585 | 1.00 | 16.20 | O |
| ATOM | 4255 | CB | PRO C 120 | 32.838 | -12.467 | 28.659 | 1.00 | 18.85 | C |
| ATOM | 4256 | CG | PRO C 120 | 33.898 | -13.358 | 28.254 | 1.00 | 20.38 | C |
| ATOM | 4257 | CD | PRO C 120 | 35.056 | -12.481 | 27.816 | 1.00 | 18.48 | C |
| ATOM | 4258 | N | SER C 121 | 31.624 | -9.301 | 28.450 | 1.00 | 18.36 | N |
| ATOM | 4259 | CA | SER C 121 | 31.148 | -8.261 | 29.312 | 1.00 | 19.83 | C |
| ATOM | 4260 | C | SER C 121 | 30.652 | -8.757 | 30.657 | 1.00 | 20.80 | C |
| ATOM | 4261 | O | SER C 121 | 30.102 | -9.858 | 30.772 | 1.00 | 20.52 | O |
| ATOM | 4262 | CB | SER C 121 | 30.016 | -7.494 | 28.634 | 1.00 | 20.26 | C |
| ATOM | 4263 | OG | SER C 121 | 28.893 | -8.328 | 28.342 | 1.00 | 18.29 | O |
| ATOM | 4264 | N | SER C 122 | 30.733 | -7.885 | 31.665 | 1.00 | 22.09 | N |
| ATOM | 4265 | CA | SER C 122 | 30.070 | -8.175 | 32.935 | 1.00 | 22.19 | C |
| ATOM | 4266 | C | SER C 122 | 28.595 | -8.455 | 32.727 | 1.00 | 21.98 | C |
| ATOM | 4267 | O | SER C 122 | 28.075 | -9.357 | 33.352 | 1.00 | 22.77 | O |
| ATOM | 4268 | CB | SER C 122 | 30.223 | -7.010 | 33.917 | 1.00 | 25.11 | C |
| ATOM | 4269 | OG | SER C 122 | 29.609 | -5.857 | 33.341 | 1.00 | 29.81 | O |
| ATOM | 4270 | N | GLU C 123 | 27.935 | -7.696 | 31.827 | 1.00 | 22.46 | N |
| ATOM | 4271 | CA | GLU C 123 | 26.515 | -7.855 | 31.544 | 1.00 | 22.77 | C |
| ATOM | 4272 | C | GLU C 123 | 26.206 | -9.278 | 31.098 | 1.00 | 22.33 | C |
| ATOM | 4273 | O | GLU C 123 | 25.255 | -9.888 | 31.545 | 1.00 | 21.02 | O |
| ATOM | 4274 | CB | GLU C 123 | 26.047 | -6.942 | 30.417 | 1.00 | 25.10 | C |
| ATOM | 4275 | CG | GLU C 123 | 25.940 | -5.520 | 30.777 | 1.00 | 28.30 | C |
| ATOM | 4276 | CD | GLU C 123 | 27.202 | -4.728 | 30.492 | 1.00 | 30.49 | C |
| ATOM | 4277 | OE1 | GLU C 123 | 28.317 | -5.293 | 30.606 | 1.00 | 28.38 | O |
| ATOM | 4278 | OE2 | GLU C 123 | 27.065 | -3.520 | 30.173 | 1.00 | 31.39 | O |
| ATOM | 4279 | N | GLN C 124 | 27.024 | -9.785 | 30.189 | 1.00 | 18.16 | N |
| ATOM | 4280 | CA | GLN C 124 | 26.799 | -11.129 | 29.695 | 1.00 | 18.24 | C |

```
ATOM   4281  C    GLN C 124      27.068 -12.160  30.757  1.00 17.69           C
ATOM   4282  O    GLN C 124      26.353 -13.171  30.842  1.00 15.77           O
ATOM   4283  CB   GLN C 124      27.655 -11.422  28.480  1.00 18.01           C
ATOM   4284  CG   GLN C 124      27.389 -12.832  27.935  1.00 17.56           C
ATOM   4285  CD   GLN C 124      28.212 -13.189  26.690  1.00 18.66           C
ATOM   4286  OE1  GLN C 124      29.253 -12.561  26.413  1.00 17.26           O
ATOM   4287  NE2  GLN C 124      27.769 -14.248  25.972  1.00 17.14           N
ATOM   4288  N    LEU C 125      28.115 -11.967  31.542  1.00 16.86           N
ATOM   4289  CA   LEU C 125      28.443 -13.013  32.521  1.00 16.72           C
ATOM   4290  C    LEU C 125      27.281 -13.285  33.486  1.00 16.35           C
ATOM   4291  O    LEU C 125      27.147 -14.416  33.975  1.00 16.79           O
ATOM   4292  CB   LEU C 125      29.769 -12.747  33.252  1.00 17.50           C
ATOM   4293  CG   LEU C 125      31.010 -12.892  32.353  1.00 16.09           C
ATOM   4294  CD1  LEU C 125      32.192 -12.287  33.038  1.00 20.44           C
ATOM   4295  CD2  LEU C 125      31.302 -14.324  31.960  1.00 19.83           C
ATOM   4296  N    THR C 126      26.414 -12.288  33.754  1.00 17.17           N
ATOM   4297  CA   THR C 126      25.163 -12.511  34.519  1.00 17.81           C
ATOM   4298  C    THR C 126      24.314 -13.653  34.006  1.00 19.21           C
ATOM   4299  O    THR C 126      23.669 -14.370  34.797  1.00 20.33           O
ATOM   4300  CB   THR C 126      24.292 -11.260  34.560  1.00 20.83           C
ATOM   4301  OG1  THR C 126      25.150 -10.155  34.858  1.00 22.13           O
ATOM   4302  CG2  THR C 126      23.230 -11.377  35.658  1.00 20.13           C
ATOM   4303  N    SER C 127      24.328 -13.850  32.697  1.00 15.78           N
ATOM   4304  CA   SER C 127      23.555 -14.861  32.042  1.00 16.31           C
ATOM   4305  C    SER C 127      24.083 -16.264  32.284  1.00 17.45           C
ATOM   4306  O    SER C 127      23.430 -17.237  31.874  1.00 17.21           O
ATOM   4307  CB   SER C 127      23.540 -14.625  30.529  1.00 16.37           C
ATOM   4308  OG   SER C 127      24.777 -14.913  29.900  1.00 16.44           O
ATOM   4309  N    GLY C 128      25.295 -16.367  32.824  1.00 14.81           N
ATOM   4310  CA   GLY C 128      25.999 -17.675  32.926  1.00 14.03           C
ATOM   4311  C    GLY C 128      26.665 -18.137  31.649  1.00 16.09           C
ATOM   4312  O    GLY C 128      27.111 -19.240  31.568  1.00 16.32           O
ATOM   4313  N    GLY C 129      26.675 -17.297  30.629  1.00 15.32           N
ATOM   4314  CA   GLY C 129      27.327 -17.539  29.348  1.00 16.18           C
ATOM   4315  C    GLY C 129      28.463 -16.515  29.186  1.00 14.85           C
ATOM   4316  O    GLY C 129      28.488 -15.453  29.824  1.00 15.22           O
ATOM   4317  N    ALA C 130      29.423 -16.839  28.344  1.00 15.33           N
ATOM   4318  CA   ALA C 130      30.555 -15.959  28.045  1.00 15.69           C
ATOM   4319  C    ALA C 130      30.925 -16.023  26.582  1.00 16.71           C
ATOM   4320  O    ALA C 130      31.247 -17.079  26.085  1.00 17.61           O
ATOM   4321  CB   ALA C 130      31.735 -16.389  28.842  1.00 17.04           C
ATOM   4322  N    SER C 131      30.977 -14.878  25.903  1.00 15.14           N
ATOM   4323  CA   SER C 131      31.405 -14.846  24.533  1.00 14.89           C
ATOM   4324  C    SER C 131      32.660 -13.968  24.380  1.00 15.11           C
ATOM   4325  O    SER C 131      32.739 -12.881  24.988  1.00 16.95           O
ATOM   4326  CB   SER C 131      30.290 -14.298  23.636  1.00 15.84           C
ATOM   4327  OG   SER C 131      29.109 -15.051  23.698  1.00 17.00           O
ATOM   4328  N    VAL C 132      33.638 -14.474  23.655  1.00 13.96           N
ATOM   4329  CA   VAL C 132      34.877 -13.764  23.277  1.00 14.74           C
ATOM   4330  C    VAL C 132      34.727 -13.437  21.796  1.00 14.78           C
ATOM   4331  O    VAL C 132      34.391 -14.310  20.965  1.00 16.00           O
ATOM   4332  CB   VAL C 132      36.178 -14.593  23.538  1.00 15.11           C
ATOM   4333  CG1  VAL C 132      37.421 -13.688  23.438  1.00 16.46           C
ATOM   4334  CG2  VAL C 132      36.056 -15.377  24.914  1.00 15.91           C
ATOM   4335  N    VAL C 133      34.926 -12.156  21.453  1.00 14.77           N
ATOM   4336  CA   VAL C 133      34.705 -11.721  20.084  1.00 14.76           C
ATOM   4337  C    VAL C 133      36.024 -11.327  19.443  1.00 15.10           C
ATOM   4338  O    VAL C 133      36.836 -10.600  20.053  1.00 16.04           O
ATOM   4339  CB   VAL C 133      33.718 -10.529  19.986  1.00 13.37           C
ATOM   4340  CG1  VAL C 133      33.501 -10.108  18.520  1.00 15.56           C
ATOM   4341  CG2  VAL C 133      32.348 -10.883  20.584  1.00 14.70           C
ATOM   4342  N    CYS C 134      36.190 -11.722  18.186  1.00 15.43           N
ATOM   4343  CA  ACYS C 134      37.326 -11.308  17.369  0.50 15.84           C
ATOM   4344  CA  BCYS C 134      37.330 -11.254  17.369  0.50 16.68           C
ATOM   4345  C    CYS C 134      36.821 -10.648  16.065  1.00 16.12           C
```

```
ATOM   4346  O   CYS  C 134    36.018 -11.258  15.362  1.00 15.39        O
ATOM   4347  CB ACYS  C 134    38.160 -12.550  17.072  0.50 16.21        C
ATOM   4348  CB BCYS  C 134    38.354 -12.362  17.088  0.50 17.09        C
ATOM   4349  SG ACYS  C 134    39.707 -12.261  16.293  0.50 15.08        S
ATOM   4350  SG BCYS  C 134    39.631 -12.455  18.342  0.50 20.90        S
ATOM   4351  N   PHE  C 135    37.239  -9.406  15.789  1.00 17.21        N
ATOM   4352  CA  PHE  C 135    37.031  -8.763  14.476  1.00 14.87        C
ATOM   4353  C   PHE  C 135    38.347  -8.879  13.691  1.00 16.32        C
ATOM   4354  O   PHE  C 135    39.423  -8.724  14.251  1.00 15.63        O
ATOM   4355  CB  PHE  C 135    36.594  -7.277  14.611  1.00 15.01        C
ATOM   4356  CG  PHE  C 135    35.295  -7.070  15.333  1.00 13.56        C
ATOM   4357  CD1 PHE  C 135    34.120  -7.560  14.804  1.00 16.15        C
ATOM   4358  CD2 PHE  C 135    35.221  -6.369  16.537  1.00 17.15        C
ATOM   4359  CE1 PHE  C 135    32.884  -7.366  15.444  1.00 15.97        C
ATOM   4360  CE2 PHE  C 135    33.985  -6.158  17.170  1.00 15.08        C
ATOM   4361  CZ  PHE  C 135    32.840  -6.679  16.641  1.00 14.69        C
ATOM   4362  N   LEU  C 136    38.242  -9.216  12.418  1.00 15.94        N
ATOM   4363  CA  LEU  C 136    39.352  -9.349  11.511  1.00 16.13        C
ATOM   4364  C   LEU  C 136    38.949  -8.499  10.347  1.00 16.51        C
ATOM   4365  O   LEU  C 136    38.211  -8.927   9.502  1.00 14.84        O
ATOM   4366  CB  LEU  C 136    39.535 -10.809  11.118  1.00 16.78        C
ATOM   4367  CG  LEU  C 136    39.707 -11.733  12.318  1.00 16.79        C
ATOM   4368  CD1 LEU  C 136    38.384 -12.364  12.610  1.00 18.01        C
ATOM   4369  CD2 LEU  C 136    40.735 -12.792  12.018  1.00 20.03        C
ATOM   4370  N   ASN  C 137    39.390  -7.244  10.368  1.00 17.91        N
ATOM   4371  CA  ASN  C 137    38.813  -6.225   9.482  1.00 17.24        C
ATOM   4372  C   ASN  C 137    39.735  -5.773   8.328  1.00 17.20        C
ATOM   4373  O   ASN  C 137    40.965  -5.764   8.448  1.00 17.08        O
ATOM   4374  CB  ASN  C 137    38.379  -5.026  10.310  1.00 17.89        C
ATOM   4375  CG  ASN  C 137    37.155  -5.308  11.150  1.00 19.30        C
ATOM   4376  OD1 ASN  C 137    36.442  -6.288  10.911  1.00 17.47        O
ATOM   4377  ND2 ASN  C 137    36.882  -4.458  12.097  1.00 17.70        N
ATOM   4378  N   ASN  C 138    39.095  -5.480   7.199  1.00 19.66        N
ATOM   4379  CA  ASN  C 138    39.684  -4.721   6.051  1.00 20.27        C
ATOM   4380  C   ASN  C 138    40.878  -5.425   5.454  1.00 19.08        C
ATOM   4381  O   ASN  C 138    41.984  -4.842   5.288  1.00 19.07        O
ATOM   4382  CB  ASN  C 138    39.995  -3.263   6.387  1.00 22.46        C
ATOM   4383  CG  ASN  C 138    38.757  -2.490   6.786  1.00 23.95        C
ATOM   4384  OD1 ASN  C 138    38.381  -2.516   7.919  1.00 25.66        O
ATOM   4385  ND2 ASN  C 138    38.162  -1.750   5.852  1.00 28.61        N
ATOM   4386  N   PHE  C 139    40.621  -6.681   5.108  1.00 19.01        N
ATOM   4387  CA  PHE  C 139    41.605  -7.534   4.480  1.00 20.27        C
ATOM   4388  C   PHE  C 139    41.279  -7.901   3.031  1.00 18.17        C
ATOM   4389  O   PHE  C 139    40.165  -7.728   2.557  1.00 18.26        O
ATOM   4390  CB  PHE  C 139    41.841  -8.771   5.352  1.00 20.09        C
ATOM   4391  CG  PHE  C 139    40.647  -9.682   5.501  1.00 21.00        C
ATOM   4392  CD1 PHE  C 139    40.392 -10.652   4.541  1.00 19.87        C
ATOM   4393  CD2 PHE  C 139    39.839  -9.633   6.631  1.00 16.95        C
ATOM   4394  CE1 PHE  C 139    39.304 -11.512   4.675  1.00 21.69        C
ATOM   4395  CE2 PHE  C 139    38.778 -10.465   6.771  1.00 20.34        C
ATOM   4396  CZ  PHE  C 139    38.505 -11.422   5.798  1.00 19.38        C
ATOM   4397  N   TYR  C 140    42.312  -8.365   2.331  1.00 21.12        N
ATOM   4398  CA  TYR  C 140    42.200  -8.866   0.977  1.00 21.61        C
ATOM   4399  C   TYR  C 140    43.369  -9.813   0.712  1.00 21.79        C
ATOM   4400  O   TYR  C 140    44.491  -9.515   1.087  1.00 24.40        O
ATOM   4401  CB  TYR  C 140    42.218  -7.709  -0.038  1.00 23.34        C
ATOM   4402  CG  TYR  C 140    41.878  -8.193  -1.415  1.00 23.77        C
ATOM   4403  CD1 TYR  C 140    42.875  -8.648  -2.267  1.00 24.95        C
ATOM   4404  CD2 TYR  C 140    40.550  -8.249  -1.844  1.00 23.93        C
ATOM   4405  CE1 TYR  C 140    42.566  -9.136  -3.502  1.00 25.11        C
ATOM   4406  CE2 TYR  C 140    40.233  -8.734  -3.095  1.00 24.26        C
ATOM   4407  CZ  TYR  C 140    41.256  -9.168  -3.904  1.00 22.01        C
ATOM   4408  OH  TYR  C 140    40.988  -9.665  -5.134  1.00 24.95        O
ATOM   4409  N   PRO  C 141    43.119 -10.942   0.059  1.00 22.13        N
ATOM   4410  CA  PRO  C 141    41.921 -11.447  -0.545  1.00 22.30        C
```

| ATOM | 4411 | C   | PRO | C | 141 | 40.942 | -11.944 | 0.513  | 1.00 | 23.08 | C |
|------|------|-----|-----|---|-----|--------|---------|--------|------|-------|---|
| ATOM | 4412 | O   | PRO | C | 141 | 41.260 | -11.927 | 1.718  | 1.00 | 20.92 | O |
| ATOM | 4413 | CB  | PRO | C | 141 | 42.423 | -12.584 | -1.444 | 1.00 | 22.57 | C |
| ATOM | 4414 | CG  | PRO | C | 141 | 43.649 | -13.017 | -0.850 | 1.00 | 23.08 | C |
| ATOM | 4415 | CD  | PRO | C | 141 | 44.241 | -11.880 | -0.099 | 1.00 | 22.52 | C |
| ATOM | 4416 | N   | LYS | C | 142 | 39.775 | -12.385 | 0.060  | 1.00 | 22.09 | N |
| ATOM | 4417 | CA  | LYS | C | 142 | 38.666 | -12.715 | 0.958  | 1.00 | 23.74 | C |
| ATOM | 4418 | C   | LYS | C | 142 | 38.870 | -13.990 | 1.768  | 1.00 | 23.64 | C |
| ATOM | 4419 | O   | LYS | C | 142 | 38.315 | -14.156 | 2.862  | 1.00 | 22.83 | O |
| ATOM | 4420 | CB  | LYS | C | 142 | 37.369 | -12.802 | 0.161  | 1.00 | 24.71 | C |
| ATOM | 4421 | CG  | LYS | C | 142 | 37.220 | -13.999 | -0.791 | 1.00 | 28.63 | C |
| ATOM | 4422 | CD  | LYS | C | 142 | 35.907 | -13.861 | -1.599 | 1.00 | 30.32 | C |
| ATOM | 4423 | CE  | LYS | C | 142 | 35.778 | -14.929 | -2.712 | 1.00 | 32.18 | C |
| ATOM | 4424 | NZ  | LYS | C | 142 | 34.466 | -14.835 | -3.392 | 1.00 | 33.53 | N |
| ATOM | 4425 | N   | ASP | C | 143 | 39.670 | -14.885 | 1.231  | 1.00 | 22.85 | N |
| ATOM | 4426 | CA  | ASP | C | 143 | 39.901 | -16.189 | 1.840  | 1.00 | 23.41 | C |
| ATOM | 4427 | C   | ASP | C | 143 | 40.664 | -16.038 | 3.175  | 1.00 | 21.44 | C |
| ATOM | 4428 | O   | ASP | C | 143 | 41.747 | -15.534 | 3.207  | 1.00 | 19.55 | O |
| ATOM | 4429 | CB  | ASP | C | 143 | 40.711 | -17.027 | 0.870  | 1.00 | 26.67 | C |
| ATOM | 4430 | CG  | ASP | C | 143 | 40.218 | -16.880 | -0.562 | 1.00 | 31.33 | C |
| ATOM | 4431 | OD1 | ASP | C | 143 | 39.344 | -17.707 | -0.908 | 1.00 | 37.22 | O |
| ATOM | 4432 | OD2 | ASP | C | 143 | 40.647 | -15.909 | -1.290 | 1.00 | 30.61 | O |
| ATOM | 4433 | N   | ILE | C | 144 | 40.084 | -16.517 | 4.269  | 1.00 | 20.32 | N |
| ATOM | 4434 | CA  | ILE | C | 144 | 40.740 | -16.489 | 5.560  | 1.00 | 20.20 | C |
| ATOM | 4435 | C   | ILE | C | 144 | 40.232 | -17.656 | 6.417  | 1.00 | 22.14 | C |
| ATOM | 4436 | O   | ILE | C | 144 | 39.122 | -18.151 | 6.192  | 1.00 | 23.73 | O |
| ATOM | 4437 | CB  | ILE | C | 144 | 40.408 | -15.180 | 6.230  | 1.00 | 20.07 | C |
| ATOM | 4438 | CG1 | ILE | C | 144 | 41.322 | -14.895 | 7.404  | 1.00 | 20.28 | C |
| ATOM | 4439 | CG2 | ILE | C | 144 | 38.925 | -15.154 | 6.650  | 1.00 | 21.68 | C |
| ATOM | 4440 | CD1 | ILE | C | 144 | 41.243 | -13.421 | 7.854  | 1.00 | 21.73 | C |
| ATOM | 4441 | N   | ASN | C | 145 | 41.027 | -18.067 | 7.400  | 1.00 | 23.81 | N |
| ATOM | 4442 | CA  | ASN | C | 145 | 40.635 | -19.182 | 8.294  | 1.00 | 24.20 | C |
| ATOM | 4443 | C   | ASN | C | 145 | 40.974 | -18.621 | 9.672  | 1.00 | 22.96 | C |
| ATOM | 4444 | O   | ASN | C | 145 | 42.140 | -18.335 | 9.922  | 1.00 | 21.26 | O |
| ATOM | 4445 | CB  | ASN | C | 145 | 41.405 | -20.424 | 7.977  | 1.00 | 26.20 | C |
| ATOM | 4446 | CG  | ASN | C | 145 | 40.962 | -21.576 | 8.822  | 1.00 | 31.45 | C |
| ATOM | 4447 | OD1 | ASN | C | 145 | 41.657 | -21.978 | 9.770  | 1.00 | 35.82 | O |
| ATOM | 4448 | ND2 | ASN | C | 145 | 39.758 | -22.062 | 8.546  | 1.00 | 32.98 | N |
| ATOM | 4449 | N   | VAL | C | 146 | 39.990 | -18.650 | 10.562 | 1.00 | 21.77 | N |
| ATOM | 4450 | CA  | VAL | C | 146 | 40.217 | -18.570 | 12.014 | 1.00 | 19.77 | C |
| ATOM | 4451 | C   | VAL | C | 146 | 39.973 | -19.773 | 12.867 | 1.00 | 20.24 | C |
| ATOM | 4452 | O   | VAL | C | 146 | 39.036 | -20.524 | 12.622 | 1.00 | 19.34 | O |
| ATOM | 4453 | CB  | VAL | C | 146 | 39.252 | -17.448 | 12.444 | 1.00 | 20.79 | C |
| ATOM | 4454 | CG1 | VAL | C | 146 | 39.292 | -17.197 | 13.939 | 1.00 | 21.48 | C |
| ATOM | 4455 | CG2 | VAL | C | 146 | 39.536 | -16.201 | 11.637 | 1.00 | 21.84 | C |
| ATOM | 4456 | N   | LYS | C | 147 | 40.836 | -19.944 | 13.871 | 1.00 | 20.22 | N |
| ATOM | 4457 | CA  | LYS | C | 147 | 40.668 | -20.955 | 14.886 | 1.00 | 20.20 | C |
| ATOM | 4458 | C   | LYS | C | 147 | 40.705 | -20.251 | 16.236 | 1.00 | 20.05 | C |
| ATOM | 4459 | O   | LYS | C | 147 | 41.423 | -19.271 | 16.411 | 1.00 | 20.11 | O |
| ATOM | 4460 | CB  | LYS | C | 147 | 41.809 | -21.946 | 14.826 | 1.00 | 22.32 | C |
| ATOM | 4461 | CG  | LYS | C | 147 | 41.933 | -22.603 | 13.480 | 1.00 | 22.22 | C |
| ATOM | 4462 | CD  | LYS | C | 147 | 42.949 | -23.762 | 13.525 | 1.00 | 24.31 | C |
| ATOM | 4463 | CE  | LYS | C | 147 | 43.220 | -24.290 | 12.125 | 1.00 | 28.30 | C |
| ATOM | 4464 | NZ  | LYS | C | 147 | 44.090 | -25.507 | 12.204 | 1.00 | 29.70 | N |
| ATOM | 4465 | N   | TRP | C | 148 | 39.924 | -20.778 | 17.164 | 1.00 | 18.01 | N |
| ATOM | 4466 | CA  | TRP | C | 148 | 39.920 | -20.357 | 18.531 | 1.00 | 16.67 | C |
| ATOM | 4467 | C   | TRP | C | 148 | 40.648 | -21.381 | 19.356 | 1.00 | 18.24 | C |
| ATOM | 4468 | O   | TRP | C | 148 | 40.363 | -22.566 | 19.249 | 1.00 | 19.07 | O |
| ATOM | 4469 | CB  | TRP | C | 148 | 38.515 | -20.258 | 19.067 | 1.00 | 17.46 | C |
| ATOM | 4470 | CG  | TRP | C | 148 | 37.837 | -19.066 | 18.651 | 1.00 | 16.70 | C |
| ATOM | 4471 | CD1 | TRP | C | 148 | 37.005 | -18.920 | 17.582 | 1.00 | 17.81 | C |
| ATOM | 4472 | CD2 | TRP | C | 148 | 37.944 | -17.782 | 19.267 | 1.00 | 16.97 | C |
| ATOM | 4473 | NE1 | TRP | C | 148 | 36.571 | -17.614 | 17.510 | 1.00 | 18.29 | N |
| ATOM | 4474 | CE2 | TRP | C | 148 | 37.125 | -16.902 | 18.543 | 1.00 | 18.29 | C |
| ATOM | 4475 | CE3 | TRP | C | 148 | 38.627 | -17.303 | 20.393 | 1.00 | 17.55 | C |

```
ATOM   4476  CZ2 TRP C 148     36.987 -15.551  18.887  1.00 14.25          C
ATOM   4477  CZ3 TRP C 148     38.496 -15.968  20.731  1.00 15.91          C
ATOM   4478  CH2 TRP C 148     37.657 -15.113  19.997  1.00 16.11          C
ATOM   4479  N   LYS C 149     41.520 -20.923  20.229  1.00 17.66          N
ATOM   4480  CA  LYS C 149     42.176 -21.841  21.169  1.00 19.46          C
ATOM   4481  C   LYS C 149     42.091 -21.324  22.587  1.00 20.07          C
ATOM   4482  O   LYS C 149     42.032 -20.101  22.837  1.00 17.69          O
ATOM   4483  CB  LYS C 149     43.594 -22.118  20.743  1.00 20.88          C
ATOM   4484  CG  LYS C 149     43.680 -22.424  19.235  1.00 21.70          C
ATOM   4485  CD  LYS C 149     44.897 -23.152  18.897  1.00 26.07          C
ATOM   4486  CE  LYS C 149     44.898 -23.482  17.404  1.00 26.78          C
ATOM   4487  NZ  LYS C 149     46.054 -24.326  17.058  1.00 30.46          N
ATOM   4488  N   ILE C 150     42.000 -22.271  23.499  1.00 18.07          N
ATOM   4489  CA  ILE C 150     41.828 -21.995  24.903  1.00 18.21          C
ATOM   4490  C   ILE C 150     43.025 -22.628  25.586  1.00 17.96          C
ATOM   4491  O   ILE C 150     43.225 -23.843  25.547  1.00 16.27          O
ATOM   4492  CB  ILE C 150     40.531 -22.582  25.482  1.00 17.88          C
ATOM   4493  CG1 ILE C 150     39.297 -22.114  24.662  1.00 18.80          C
ATOM   4494  CG2 ILE C 150     40.419 -22.227  26.965  1.00 17.82          C
ATOM   4495  CD1 ILE C 150     37.974 -22.802  25.001  1.00 18.32          C
ATOM   4496  N   ASP C 151     43.862 -21.761  26.141  1.00 18.15          N
ATOM   4497  CA  ASP C 151     45.145 -22.147  26.663  1.00 18.23          C
ATOM   4498  C   ASP C 151     45.894 -23.067  25.672  1.00 18.83          C
ATOM   4499  O   ASP C 151     46.450 -24.073  26.061  1.00 20.99          O
ATOM   4500  CB  ASP C 151     44.971 -22.777  28.031  1.00 16.75          C
ATOM   4501  CG  ASP C 151     44.726 -21.768  29.111  1.00 18.76          C
ATOM   4502  OD1 ASP C 151     45.344 -20.692  29.079  1.00 20.14          O
ATOM   4503  OD2 ASP C 151     43.912 -22.041  29.974  1.00 20.08          O
ATOM   4504  N   GLY C 152     45.869 -22.702  24.400  1.00 18.17          N
ATOM   4505  CA  GLY C 152     46.525 -23.472  23.355  1.00 19.92          C
ATOM   4506  C   GLY C 152     45.780 -24.641  22.764  1.00 20.61          C
ATOM   4507  O   GLY C 152     46.210 -25.158  21.743  1.00 22.90          O
ATOM   4508  N   SER C 153     44.667 -25.072  23.355  1.00 21.81          N
ATOM   4509  CA  SER C 153     43.886 -26.190  22.785  1.00 21.82          C
ATOM   4510  C   SER C 153     42.741 -25.650  21.915  1.00 19.82          C
ATOM   4511  O   SER C 153     41.935 -24.825  22.358  1.00 19.58          O
ATOM   4512  CB  SER C 153     43.303 -27.119  23.866  1.00 22.48          C
ATOM   4513  OG  SER C 153     44.298 -27.694  24.684  1.00 26.65          O
ATOM   4514  N   GLU C 154     42.688 -26.097  20.670  1.00 20.02          N
ATOM   4515  CA  GLU C 154     41.634 -25.644  19.773  1.00 19.91          C
ATOM   4516  C   GLU C 154     40.216 -26.081  20.199  1.00 20.02          C
ATOM   4517  O   GLU C 154     39.969 -27.242  20.563  1.00 18.44          O
ATOM   4518  CB  GLU C 154     41.927 -26.017  18.309  1.00 20.13          C
ATOM   4519  CG  GLU C 154     40.848 -25.514  17.398  1.00 20.95          C
ATOM   4520  CD  GLU C 154     41.021 -25.833  15.913  1.00 23.77          C
ATOM   4521  OE1 GLU C 154     41.989 -26.533  15.545  1.00 28.58          O
ATOM   4522  OE2 GLU C 154     40.161 -25.331  15.122  1.00 26.66          O
ATOM   4523  N   ARG C 155     39.298 -25.126  20.171  1.00 19.64          N
ATOM   4524  CA  ARG C 155     37.874 -25.347  20.374  1.00 19.81          C
ATOM   4525  C   ARG C 155     37.126 -24.962  19.096  1.00 21.18          C
ATOM   4526  O   ARG C 155     37.232 -23.824  18.625  1.00 18.50          O
ATOM   4527  CB  ARG C 155     37.337 -24.503  21.535  1.00 21.30          C
ATOM   4528  CG  ARG C 155     35.843 -24.497  21.678  1.00 21.27          C
ATOM   4529  CD  ARG C 155     35.305 -25.867  22.025  1.00 23.20          C
ATOM   4530  NE  ARG C 155     35.945 -26.426  23.199  1.00 24.45          N
ATOM   4531  CZ  ARG C 155     35.701 -26.103  24.456  1.00 22.72          C
ATOM   4532  NH1 ARG C 155     34.770 -25.233  24.749  1.00 23.67          N
ATOM   4533  NH2 ARG C 155     36.402 -26.666  25.443  1.00 23.33          N
ATOM   4534  N   GLN C 156     36.388 -25.919  18.533  1.00 20.90          N
ATOM   4535  CA  GLN C 156     35.568 -25.684  17.340  1.00 22.55          C
ATOM   4536  C   GLN C 156     34.075 -25.444  17.605  1.00 21.91          C
ATOM   4537  O   GLN C 156     33.411 -24.700  16.891  1.00 23.73          O
ATOM   4538  CB  GLN C 156     35.745 -26.842  16.372  1.00 22.43          C
ATOM   4539  CG  GLN C 156     37.080 -26.893  15.747  1.00 25.27          C
ATOM   4540  CD  GLN C 156     37.192 -28.067  14.770  1.00 27.85          C
```

| ATOM | 4541 | OE1 | GLN | C | 156 | 36.373 | -28.975 | 14.797 | 1.00 | 29.68 | O |
|------|------|-----|-----|---|-----|--------|---------|--------|------|-------|---|
| ATOM | 4542 | NE2 | GLN | C | 156 | 38.216 | -28.041 | 13.915 | 1.00 | 29.78 | N |
| ATOM | 4543 | N | ASN | C | 157 | 33.519 | -26.071 | 18.622 | 1.00 | 20.56 | N |
| ATOM | 4544 | CA | ASN | C | 157 | 32.116 | -25.854 | 18.893 | 1.00 | 21.54 | C |
| ATOM | 4545 | C | ASN | C | 157 | 31.907 | -24.563 | 19.632 | 1.00 | 19.83 | C |
| ATOM | 4546 | O | ASN | C | 157 | 32.748 | -24.145 | 20.437 | 1.00 | 18.42 | O |
| ATOM | 4547 | CB | ASN | C | 157 | 31.463 | -27.003 | 19.649 | 1.00 | 22.48 | C |
| ATOM | 4548 | CG | ASN | C | 157 | 32.082 | -27.262 | 20.991 | 1.00 | 27.31 | C |
| ATOM | 4549 | OD1 | ASN | C | 157 | 33.178 | -27.828 | 21.078 | 1.00 | 32.22 | O |
| ATOM | 4550 | ND2 | ASN | C | 157 | 31.390 | -26.865 | 22.059 | 1.00 | 30.98 | N |
| ATOM | 4551 | N | GLY | C | 158 | 30.751 | -23.978 | 19.397 | 1.00 | 18.81 | N |
| ATOM | 4552 | CA | GLY | C | 158 | 30.370 | -22.739 | 20.024 | 1.00 | 15.91 | C |
| ATOM | 4553 | C | GLY | C | 158 | 30.979 | -21.542 | 19.346 | 1.00 | 16.74 | C |
| ATOM | 4554 | O | GLY | C | 158 | 31.065 | -20.493 | 19.946 | 1.00 | 15.24 | O |
| ATOM | 4555 | N | VAL | C | 159 | 31.328 | -21.704 | 18.082 | 1.00 | 17.35 | N |
| ATOM | 4556 | CA | VAL | C | 159 | 31.939 | -20.613 | 17.289 | 1.00 | 17.69 | C |
| ATOM | 4557 | C | VAL | C | 159 | 30.924 | -20.104 | 16.281 | 1.00 | 19.00 | C |
| ATOM | 4558 | O | VAL | C | 159 | 30.226 | -20.882 | 15.633 | 1.00 | 17.14 | O |
| ATOM | 4559 | CB | VAL | C | 159 | 33.258 | -21.049 | 16.579 | 1.00 | 19.44 | C |
| ATOM | 4560 | CG1 | VAL | C | 159 | 33.791 | -19.976 | 15.672 | 1.00 | 19.59 | C |
| ATOM | 4561 | CG2 | VAL | C | 159 | 34.340 | -21.437 | 17.559 | 1.00 | 18.68 | C |
| ATOM | 4562 | N | LEU | C | 160 | 30.829 | -18.782 | 16.138 | 1.00 | 16.18 | N |
| ATOM | 4563 | CA | LEU | C | 160 | 29.891 | -18.193 | 15.164 | 1.00 | 18.26 | C |
| ATOM | 4564 | C | LEU | C | 160 | 30.677 | -17.188 | 14.340 | 1.00 | 18.87 | C |
| ATOM | 4565 | O | LEU | C | 160 | 31.239 | -16.249 | 14.897 | 1.00 | 18.47 | O |
| ATOM | 4566 | CB | LEU | C | 160 | 28.726 | -17.480 | 15.843 | 1.00 | 19.35 | C |
| ATOM | 4567 | CG | LEU | C | 160 | 27.589 | -16.898 | 14.950 | 1.00 | 21.33 | C |
| ATOM | 4568 | CD1 | LEU | C | 160 | 26.230 | -16.984 | 15.561 | 1.00 | 23.74 | C |
| ATOM | 4569 | CD2 | LEU | C | 160 | 27.895 | -15.441 | 14.510 | 1.00 | 22.45 | C |
| ATOM | 4570 | N | ASN | C | 161 | 30.693 | -17.406 | 13.028 | 1.00 | 17.43 | N |
| ATOM | 4571 | CA | ASN | C | 161 | 31.461 | -16.588 | 12.092 | 1.00 | 18.33 | C |
| ATOM | 4572 | C | ASN | C | 161 | 30.533 | -15.894 | 11.119 | 1.00 | 18.23 | C |
| ATOM | 4573 | O | ASN | C | 161 | 29.554 | -16.470 | 10.663 | 1.00 | 18.70 | O |
| ATOM | 4574 | CB | ASN | C | 161 | 32.412 | -17.458 | 11.321 | 1.00 | 18.50 | C |
| ATOM | 4575 | CG | ASN | C | 161 | 33.525 | -18.025 | 12.184 | 1.00 | 20.37 | C |
| ATOM | 4576 | OD1 | ASN | C | 161 | 33.966 | -17.417 | 13.163 | 1.00 | 20.62 | O |
| ATOM | 4577 | ND2 | ASN | C | 161 | 33.986 | -19.209 | 11.826 | 1.00 | 19.72 | N |
| ATOM | 4578 | N | SER | C | 162 | 30.849 | -14.650 | 10.796 | 1.00 | 16.77 | N |
| ATOM | 4579 | CA | SER | C | 162 | 30.080 | -13.880 | 9.816 | 1.00 | 17.89 | C |
| ATOM | 4580 | C | SER | C | 162 | 31.040 | -13.022 | 9.009 | 1.00 | 17.08 | C |
| ATOM | 4581 | O | SER | C | 162 | 32.056 | -12.609 | 9.526 | 1.00 | 16.94 | O |
| ATOM | 4582 | CB | SER | C | 162 | 29.076 | -12.978 | 10.502 | 1.00 | 17.89 | C |
| ATOM | 4583 | OG | SER | C | 162 | 28.294 | -12.265 | 9.561 | 1.00 | 20.20 | O |
| ATOM | 4584 | N | TRP | C | 163 | 30.705 | -12.785 | 7.738 | 1.00 | 17.92 | N |
| ATOM | 4585 | CA | TRP | C | 163 | 31.604 | -12.152 | 6.782 | 1.00 | 19.25 | C |
| ATOM | 4586 | C | TRP | C | 163 | 30.867 | -11.024 | 6.124 | 1.00 | 18.57 | C |
| ATOM | 4587 | O | TRP | C | 163 | 29.672 | -11.168 | 5.830 | 1.00 | 19.80 | O |
| ATOM | 4588 | CB | TRP | C | 163 | 32.009 | -13.172 | 5.733 | 1.00 | 20.04 | C |
| ATOM | 4589 | CG | TRP | C | 163 | 32.781 | -14.299 | 6.280 | 1.00 | 22.76 | C |
| ATOM | 4590 | CD1 | TRP | C | 163 | 34.132 | -14.364 | 6.444 | 1.00 | 24.96 | C |
| ATOM | 4591 | CD2 | TRP | C | 163 | 32.252 | -15.524 | 6.784 | 1.00 | 22.66 | C |
| ATOM | 4592 | NE1 | TRP | C | 163 | 34.484 | -15.585 | 6.994 | 1.00 | 25.40 | N |
| ATOM | 4593 | CE2 | TRP | C | 163 | 33.340 | -16.310 | 7.210 | 1.00 | 23.37 | C |
| ATOM | 4594 | CE3 | TRP | C | 163 | 30.968 | -16.038 | 6.895 | 1.00 | 23.48 | C |
| ATOM | 4595 | CZ2 | TRP | C | 163 | 33.171 | -17.598 | 7.728 | 1.00 | 23.17 | C |
| ATOM | 4596 | CZ3 | TRP | C | 163 | 30.801 | -17.292 | 7.419 | 1.00 | 25.46 | C |
| ATOM | 4597 | CH2 | TRP | C | 163 | 31.890 | -18.062 | 7.830 | 1.00 | 23.90 | C |
| ATOM | 4598 | N | THR | C | 164 | 31.513 | -9.879 | 5.952 | 1.00 | 20.32 | N |
| ATOM | 4599 | CA | THR | C | 164 | 30.878 | -8.797 | 5.186 | 1.00 | 20.91 | C |
| ATOM | 4600 | C | THR | C | 164 | 31.012 | -9.021 | 3.663 | 1.00 | 20.81 | C |
| ATOM | 4601 | O | THR | C | 164 | 31.820 | -9.803 | 3.199 | 1.00 | 22.78 | O |
| ATOM | 4602 | CB | THR | C | 164 | 31.451 | -7.423 | 5.550 | 1.00 | 21.17 | C |
| ATOM | 4603 | OG1 | THR | C | 164 | 32.875 | -7.424 | 5.360 | 1.00 | 19.77 | O |
| ATOM | 4604 | CG2 | THR | C | 164 | 31.104 | -7.041 | 6.993 | 1.00 | 22.89 | C |
| ATOM | 4605 | N | ASP | C | 165 | 30.152 | -8.343 | 2.912 | 1.00 | 22.80 | N |

| ATOM | 4606 | CA | ASP C | 165 | 30.297 | -8.194 | 1.468 | 1.00 | 23.37 | C |
|------|------|-----|-------|-----|--------|--------|-------|------|-------|---|
| ATOM | 4607 | C | ASP C | 165 | 31.543 | -7.332 | 1.175 | 1.00 | 23.13 | C |
| ATOM | 4608 | O | ASP C | 165 | 31.970 | -6.542 | 1.995 | 1.00 | 22.31 | O |
| ATOM | 4609 | CB | ASP C | 165 | 29.066 | -7.474 | 0.885 | 1.00 | 26.36 | C |
| ATOM | 4610 | CG | ASP C | 165 | 27.775 | -8.263 | 1.038 | 1.00 | 28.59 | C |
| ATOM | 4611 | OD1 | ASP C | 165 | 27.784 | -9.476 | 0.743 | 1.00 | 33.87 | O |
| ATOM | 4612 | OD2 | ASP C | 165 | 26.736 | -7.651 | 1.418 | 1.00 | 33.56 | O |
| ATOM | 4613 | N | GLN C | 166 | 32.074 | -7.438 | -0.034 | 1.00 | 20.56 | N |
| ATOM | 4614 | CA | GLN C | 166 | 33.222 | -6.645 | -0.410 | 1.00 | 19.94 | C |
| ATOM | 4615 | C | GLN C | 166 | 32.895 | -5.152 | -0.304 | 1.00 | 19.45 | C |
| ATOM | 4616 | O | GLN C | 166 | 31.846 | -4.724 | -0.764 | 1.00 | 19.96 | O |
| ATOM | 4617 | CB | GLN C | 166 | 33.638 | -7.001 | -1.835 | 1.00 | 18.84 | C |
| ATOM | 4618 | CG | GLN C | 166 | 34.929 | -6.261 | -2.256 | 1.00 | 17.09 | C |
| ATOM | 4619 | CD | GLN C | 166 | 35.555 | -6.775 | -3.502 | 1.00 | 17.07 | C |
| ATOM | 4620 | OE1 | GLN C | 166 | 34.856 | -7.170 | -4.441 | 1.00 | 19.59 | O |
| ATOM | 4621 | NE2 | GLN C | 166 | 36.873 | -6.797 | -3.538 | 1.00 | 19.69 | N |
| ATOM | 4622 | N | ASP C | 167 | 33.779 | -4.379 | 0.317 | 1.00 | 20.78 | N |
| ATOM | 4623 | CA | ASP C | 167 | 33.507 | -2.986 | 0.595 | 1.00 | 21.53 | C |
| ATOM | 4624 | C | ASP C | 167 | 33.464 | -2.234 | -0.732 | 1.00 | 22.69 | C |
| ATOM | 4625 | O | ASP C | 167 | 34.342 | -2.429 | -1.550 | 1.00 | 20.03 | O |
| ATOM | 4626 | CB | ASP C | 167 | 34.590 | -2.437 | 1.515 | 1.00 | 22.79 | C |
| ATOM | 4627 | CG | ASP C | 167 | 34.271 | -1.090 | 2.042 | 1.00 | 25.47 | C |
| ATOM | 4628 | OD1 | ASP C | 167 | 33.674 | -1.031 | 3.141 | 1.00 | 28.21 | O |
| ATOM | 4629 | OD2 | ASP C | 167 | 34.618 | -0.084 | 1.371 | 1.00 | 25.06 | O |
| ATOM | 4630 | N | SER C | 168 | 32.441 | -1.389 | -0.911 | 1.00 | 24.17 | N |
| ATOM | 4631 | CA | SER C | 168 | 32.250 | -0.608 | -2.153 | 1.00 | 25.75 | C |
| ATOM | 4632 | C | SER C | 168 | 33.237 | 0.539 | -2.308 | 1.00 | 26.08 | C |
| ATOM | 4633 | O | SER C | 168 | 33.359 | 1.073 | -3.403 | 1.00 | 26.97 | O |
| ATOM | 4634 | CB | SER C | 168 | 30.834 | -0.037 | -2.224 | 1.00 | 26.18 | C |
| ATOM | 4635 | OG | SER C | 168 | 29.927 | -1.054 | -2.598 | 1.00 | 33.84 | O |
| ATOM | 4636 | N | LYS C | 169 | 33.928 | 0.911 | -1.238 | 1.00 | 26.86 | N |
| ATOM | 4637 | CA | LYS C | 169 | 34.955 | 1.951 | -1.287 | 1.00 | 27.50 | C |
| ATOM | 4638 | C | LYS C | 169 | 36.378 | 1.431 | -1.404 | 1.00 | 26.98 | C |
| ATOM | 4639 | O | LYS C | 169 | 37.126 | 1.848 | -2.298 | 1.00 | 28.03 | O |
| ATOM | 4640 | CB | LYS C | 169 | 34.825 | 2.850 | -0.055 | 1.00 | 29.08 | C |
| ATOM | 4641 | CG | LYS C | 169 | 33.415 | 3.398 | 0.122 | 1.00 | 31.37 | C |
| ATOM | 4642 | CD | LYS C | 169 | 33.358 | 4.909 | -0.006 | 1.00 | 34.75 | C |
| ATOM | 4643 | CE | LYS C | 169 | 32.064 | 5.505 | 0.582 | 1.00 | 36.46 | C |
| ATOM | 4644 | NZ | LYS C | 169 | 32.154 | 5.736 | 2.084 | 1.00 | 38.96 | N |
| ATOM | 4645 | N | ASP C | 170 | 36.776 | 0.563 | -0.484 | 1.00 | 24.64 | N |
| ATOM | 4646 | CA | ASP C | 170 | 38.129 | 0.091 | -0.460 | 1.00 | 23.69 | C |
| ATOM | 4647 | C | ASP C | 170 | 38.292 | -1.329 | -0.932 | 1.00 | 22.14 | C |
| ATOM | 4648 | O | ASP C | 170 | 39.381 | -1.786 | -0.938 | 1.00 | 22.10 | O |
| ATOM | 4649 | CB | ASP C | 170 | 38.796 | 0.281 | 0.910 | 1.00 | 24.00 | C |
| ATOM | 4650 | CG | ASP C | 170 | 38.275 | -0.661 | 1.993 | 1.00 | 24.51 | C |
| ATOM | 4651 | OD1 | ASP C | 170 | 37.660 | -1.708 | 1.685 | 1.00 | 22.68 | O |
| ATOM | 4652 | OD2 | ASP C | 170 | 38.532 | -0.337 | 3.184 | 1.00 | 27.03 | O |
| ATOM | 4653 | N | SER C | 171 | 37.215 | -2.006 | -1.315 | 1.00 | 21.48 | N |
| ATOM | 4654 | CA | SER C | 171 | 37.300 | -3.333 | -1.943 | 1.00 | 19.48 | C |
| ATOM | 4655 | C | SER C | 171 | 37.829 | -4.428 | -1.004 | 1.00 | 20.67 | C |
| ATOM | 4656 | O | SER C | 171 | 38.297 | -5.465 | -1.469 | 1.00 | 20.45 | O |
| ATOM | 4657 | CB | SER C | 171 | 38.134 | -3.284 | -3.235 | 1.00 | 20.41 | C |
| ATOM | 4658 | OG | SER C | 171 | 37.555 | -2.366 | -4.176 | 1.00 | 18.33 | O |
| ATOM | 4659 | N | THR C | 172 | 37.800 | -4.170 | 0.301 | 1.00 | 19.61 | N |
| ATOM | 4660 | CA | THR C | 172 | 38.309 | -5.159 | 1.278 | 1.00 | 19.42 | C |
| ATOM | 4661 | C | THR C | 172 | 37.152 | -5.943 | 1.859 | 1.00 | 17.81 | C |
| ATOM | 4662 | O | THR C | 172 | 35.989 | -5.637 | 1.603 | 1.00 | 16.41 | O |
| ATOM | 4663 | CB | THR C | 172 | 39.076 | -4.520 | 2.443 | 1.00 | 18.78 | C |
| ATOM | 4664 | OG1 | THR C | 172 | 38.203 | -3.702 | 3.218 | 1.00 | 19.43 | O |
| ATOM | 4665 | CG2 | THR C | 172 | 40.292 | -3.692 | 1.948 | 1.00 | 18.94 | C |
| ATOM | 4666 | N | TYR C | 173 | 37.508 | -6.927 | 2.682 | 1.00 | 18.34 | N |
| ATOM | 4667 | CA | TYR C | 173 | 36.557 | -7.802 | 3.344 | 1.00 | 17.03 | C |
| ATOM | 4668 | C | TYR C | 173 | 36.804 | -7.749 | 4.840 | 1.00 | 17.22 | C |
| ATOM | 4669 | O | TYR C | 173 | 37.891 | -7.401 | 5.280 | 1.00 | 17.22 | O |
| ATOM | 4670 | CB | TYR C | 173 | 36.771 | -9.241 | 2.864 | 1.00 | 17.12 | C |

| ATOM | 4671 | CG | TYR C | 173 | 36.422 | -9.473 | 1.406 | 1.00 | 16.67 | C |
|------|------|-----|-------|-----|--------|--------|-------|------|-------|---|
| ATOM | 4672 | CD1 | TYR C | 173 | 37.405 | -9.304 | 0.438 | 1.00 | 19.04 | C |
| ATOM | 4673 | CD2 | TYR C | 173 | 35.133 | -9.814 | 0.993 | 1.00 | 17.96 | C |
| ATOM | 4674 | CE1 | TYR C | 173 | 37.131 | -9.502 | -0.916 | 1.00 | 19.03 | C |
| ATOM | 4675 | CE2 | TYR C | 173 | 34.832 | -10.032 | -0.391 | 1.00 | 16.58 | C |
| ATOM | 4676 | CZ | TYR C | 173 | 35.850 | -9.867 | -1.317 | 1.00 | 18.70 | C |
| ATOM | 4677 | OH | TYR C | 173 | 35.597 | -10.011 | -2.633 | 1.00 | 20.01 | O |
| ATOM | 4678 | N | SER C | 174 | 35.792 | -8.120 | 5.614 | 1.00 | 17.13 | N |
| ATOM | 4679 | CA | SER C | 174 | 35.893 | -8.177 | 7.061 | 1.00 | 16.74 | C |
| ATOM | 4680 | C | SER C | 174 | 35.167 | -9.379 | 7.606 | 1.00 | 18.53 | C |
| ATOM | 4681 | O | SER C | 174 | 34.243 | -9.886 | 6.969 | 1.00 | 16.17 | O |
| ATOM | 4682 | CB | SER C | 174 | 35.380 | -6.928 | 7.721 | 1.00 | 18.14 | C |
| ATOM | 4683 | OG | SER C | 174 | 36.161 | -5.777 | 7.335 | 1.00 | 19.61 | O |
| ATOM | 4684 | N | MET C | 175 | 35.583 | -9.804 | 8.798 | 1.00 | 17.54 | N |
| ATOM | 4685 | CA | MET C | 175 | 35.005 | -10.995 | 9.430 | 1.00 | 16.76 | C |
| ATOM | 4686 | C | MET C | 175 | 34.863 | -10.785 | 10.931 | 1.00 | 17.51 | C |
| ATOM | 4687 | O | MET C | 175 | 35.630 | -10.060 | 11.557 | 1.00 | 15.45 | O |
| ATOM | 4688 | CB | MET C | 175 | 35.889 | -12.212 | 9.124 | 1.00 | 18.33 | C |
| ATOM | 4689 | CG | MET C | 175 | 35.457 | -13.521 | 9.775 | 1.00 | 19.36 | C |
| ATOM | 4690 | SD | MET C | 175 | 36.700 | -14.811 | 9.469 | 1.00 | 20.01 | S |
| ATOM | 4691 | CE | MET C | 175 | 35.940 | -16.167 | 10.367 | 1.00 | 17.91 | C |
| ATOM | 4692 | N | SER C | 176 | 33.841 | -11.415 | 11.495 | 1.00 | 18.13 | N |
| ATOM | 4693 | CA | SER C | 176 | 33.649 | -11.494 | 12.938 | 1.00 | 16.98 | C |
| ATOM | 4694 | C | SER C | 176 | 33.554 | -12.939 | 13.326 | 1.00 | 17.80 | C |
| ATOM | 4695 | O | SER C | 176 | 32.846 | -13.703 | 12.676 | 1.00 | 18.27 | O |
| ATOM | 4696 | CB | SER C | 176 | 32.323 | -10.871 | 13.282 | 1.00 | 17.74 | C |
| ATOM | 4697 | OG | SER C | 176 | 32.114 | -10.923 | 14.674 | 1.00 | 18.79 | O |
| ATOM | 4698 | N | SER C | 177 | 34.205 | -13.288 | 14.416 | 1.00 | 16.89 | N |
| ATOM | 4699 | CA | SER C | 177 | 34.197 | -14.661 | 14.907 | 1.00 | 17.03 | C |
| ATOM | 4700 | C | SER C | 177 | 33.970 | -14.552 | 16.403 | 1.00 | 17.11 | C |
| ATOM | 4701 | O | SER C | 177 | 34.670 | -13.781 | 17.058 | 1.00 | 17.60 | O |
| ATOM | 4702 | CB | SER C | 177 | 35.529 | -15.284 | 14.596 | 1.00 | 16.41 | C |
| ATOM | 4703 | OG | SER C | 177 | 35.518 | -16.661 | 14.985 | 1.00 | 17.01 | O |
| ATOM | 4704 | N | THR C | 178 | 33.002 | -15.298 | 16.928 | 1.00 | 16.56 | N |
| ATOM | 4705 | CA | THR C | 178 | 32.616 | -15.214 | 18.331 | 1.00 | 16.28 | C |
| ATOM | 4706 | C | THR C | 178 | 32.669 | -16.624 | 18.914 | 1.00 | 16.00 | C |
| ATOM | 4707 | O | THR C | 178 | 32.050 | -17.516 | 18.333 | 1.00 | 16.80 | O |
| ATOM | 4708 | CB | THR C | 178 | 31.203 | -14.645 | 18.464 | 1.00 | 17.48 | C |
| ATOM | 4709 | OG1 | THR C | 178 | 31.183 | -13.273 | 18.002 | 1.00 | 17.36 | O |
| ATOM | 4710 | CG2 | THR C | 178 | 30.725 | -14.654 | 19.908 | 1.00 | 16.92 | C |
| ATOM | 4711 | N | LEU C | 179 | 33.412 | -16.788 | 20.003 | 1.00 | 14.21 | N |
| ATOM | 4712 | CA | LEU C | 179 | 33.476 | -18.058 | 20.766 | 1.00 | 14.20 | C |
| ATOM | 4713 | C | LEU C | 179 | 32.576 | -17.930 | 21.985 | 1.00 | 14.54 | C |
| ATOM | 4714 | O | LEU C | 179 | 32.788 | -17.064 | 22.814 | 1.00 | 15.43 | O |
| ATOM | 4715 | CB | LEU C | 179 | 34.893 | -18.337 | 21.231 | 1.00 | 16.30 | C |
| ATOM | 4716 | CG | LEU C | 179 | 35.134 | -19.535 | 22.177 | 1.00 | 16.33 | C |
| ATOM | 4717 | CD1 | LEU C | 179 | 34.877 | -20.821 | 21.429 | 1.00 | 18.05 | C |
| ATOM | 4718 | CD2 | LEU C | 179 | 36.549 | -19.521 | 22.654 | 1.00 | 17.15 | C |
| ATOM | 4719 | N | THR C | 180 | 31.651 | -18.876 | 22.175 | 1.00 | 15.17 | N |
| ATOM | 4720 | CA | THR C | 180 | 30.750 | -18.813 | 23.309 | 1.00 | 14.74 | C |
| ATOM | 4721 | C | THR C | 180 | 30.947 | -20.078 | 24.122 | 1.00 | 15.32 | C |
| ATOM | 4722 | O | THR C | 180 | 31.054 | -21.143 | 23.544 | 1.00 | 14.23 | O |
| ATOM | 4723 | CB | THR C | 180 | 29.286 | -18.654 | 22.879 | 1.00 | 16.24 | C |
| ATOM | 4724 | OG1 | THR C | 180 | 29.132 | -17.390 | 22.208 | 1.00 | 14.98 | O |
| ATOM | 4725 | CG2 | THR C | 180 | 28.326 | -18.724 | 24.039 | 1.00 | 16.25 | C |
| ATOM | 4726 | N | LEU C | 181 | 31.071 | -19.879 | 25.424 | 1.00 | 16.34 | N |
| ATOM | 4727 | CA | LEU C | 181 | 31.224 | -20.955 | 26.412 | 1.00 | 18.01 | C |
| ATOM | 4728 | C | LEU C | 181 | 30.358 | -20.683 | 27.596 | 1.00 | 15.73 | C |
| ATOM | 4729 | O | LEU C | 181 | 29.776 | -19.610 | 27.762 | 1.00 | 15.09 | O |
| ATOM | 4730 | CB | LEU C | 181 | 32.647 | -20.994 | 26.961 | 1.00 | 20.85 | C |
| ATOM | 4731 | CG | LEU C | 181 | 33.873 | -20.795 | 26.109 | 1.00 | 23.69 | C |
| ATOM | 4732 | CD1 | LEU C | 181 | 34.296 | -19.340 | 26.264 | 1.00 | 24.44 | C |
| ATOM | 4733 | CD2 | LEU C | 181 | 34.947 | -21.714 | 26.612 | 1.00 | 25.80 | C |
| ATOM | 4734 | N | THR C | 182 | 30.261 | -21.664 | 28.494 | 1.00 | 15.76 | N |
| ATOM | 4735 | CA | THR C | 182 | 29.611 | -21.404 | 29.759 | 1.00 | 14.74 | C |

| ATOM | 4736 | C   | THR | C | 182 | 30.542 | -20.525 | 30.624 | 1.00 | 13.48 | C |
|------|------|-----|-----|---|-----|--------|---------|--------|------|-------|---|
| ATOM | 4737 | O   | THR | C | 182 | 31.761 | -20.521 | 30.466 | 1.00 | 15.32 | O |
| ATOM | 4738 | CB  | THR | C | 182 | 29.243 | -22.700 | 30.510 | 1.00 | 14.13 | C |
| ATOM | 4739 | OG1 | THR | C | 182 | 30.404 | -23.504 | 30.726 | 1.00 | 15.17 | O |
| ATOM | 4740 | CG2 | THR | C | 182 | 28.279 | -23.506 | 29.702 | 1.00 | 16.80 | C |
| ATOM | 4741 | N   | LYS | C | 183 | 29.951 | -19.797 | 31.545 | 1.00 | 15.41 | N |
| ATOM | 4742 | CA  | LYS | C | 183 | 30.711 | -19.089 | 32.557 | 1.00 | 15.47 | C |
| ATOM | 4743 | C   | LYS | C | 183 | 31.645 | -20.047 | 33.325 | 1.00 | 14.72 | C |
| ATOM | 4744 | O   | LYS | C | 183 | 32.809 | -19.725 | 33.589 | 1.00 | 15.91 | O |
| ATOM | 4745 | CB  | LYS | C | 183 | 29.778 | -18.334 | 33.491 | 1.00 | 15.27 | C |
| ATOM | 4746 | CG  | LYS | C | 183 | 30.447 | -17.411 | 34.504 | 1.00 | 18.12 | C |
| ATOM | 4747 | CD  | LYS | C | 183 | 29.421 | -16.793 | 35.412 | 1.00 | 16.76 | C |
| ATOM | 4748 | CE  | LYS | C | 183 | 30.058 | -15.715 | 36.371 | 1.00 | 19.98 | C |
| ATOM | 4749 | NZ  | LYS | C | 183 | 30.712 | -16.346 | 37.523 | 1.00 | 19.21 | N |
| ATOM | 4750 | N   | ASP | C | 184 | 31.152 | -21.239 | 33.675 | 1.00 | 15.59 | N |
| ATOM | 4751 | CA  | ASP | C | 184 | 31.935 | -22.178 | 34.459 | 1.00 | 17.12 | C |
| ATOM | 4752 | C   | ASP | C | 184 | 33.183 | -22.554 | 33.651 | 1.00 | 16.96 | C |
| ATOM | 4753 | O   | ASP | C | 184 | 34.303 | -22.529 | 34.163 | 1.00 | 18.06 | O |
| ATOM | 4754 | CB  | ASP | C | 184 | 31.055 | -23.376 | 34.817 | 1.00 | 17.17 | C |
| ATOM | 4755 | CG  | ASP | C | 184 | 31.811 | -24.535 | 35.347 | 1.00 | 22.94 | C |
| ATOM | 4756 | OD1 | ASP | C | 184 | 32.660 | -24.331 | 36.248 | 1.00 | 25.52 | O |
| ATOM | 4757 | OD2 | ASP | C | 184 | 31.507 | -25.670 | 34.858 | 1.00 | 23.28 | O |
| ATOM | 4758 | N   | GLU | C | 185 | 33.009 | -22.893 | 32.372 | 1.00 | 17.35 | N |
| ATOM | 4759 | CA  | GLU | C | 185 | 34.159 | -23.267 | 31.582 | 1.00 | 16.93 | C |
| ATOM | 4760 | C   | GLU | C | 185 | 35.149 | -22.137 | 31.337 | 1.00 | 18.29 | C |
| ATOM | 4761 | O   | GLU | C | 185 | 36.371 | -22.326 | 31.393 | 1.00 | 17.34 | O |
| ATOM | 4762 | CB  | GLU | C | 185 | 33.785 | -23.915 | 30.252 | 1.00 | 18.34 | C |
| ATOM | 4763 | CG  | GLU | C | 185 | 35.058 | -24.412 | 29.578 | 1.00 | 22.05 | C |
| ATOM | 4764 | CD  | GLU | C | 185 | 34.888 | -25.236 | 28.342 | 1.00 | 26.39 | C |
| ATOM | 4765 | OE1 | GLU | C | 185 | 33.906 | -25.024 | 27.589 | 1.00 | 26.37 | O |
| ATOM | 4766 | OE2 | GLU | C | 185 | 35.830 | -26.062 | 28.114 | 1.00 | 27.68 | O |
| ATOM | 4767 | N   | TYR | C | 186 | 34.599 | -20.970 | 31.056 | 1.00 | 17.42 | N |
| ATOM | 4768 | CA  | TYR | C | 186 | 35.376 | -19.765 | 30.790 | 1.00 | 17.52 | C |
| ATOM | 4769 | C   | TYR | C | 186 | 36.318 | -19.420 | 31.961 | 1.00 | 18.22 | C |
| ATOM | 4770 | O   | TYR | C | 186 | 37.487 | -19.058 | 31.758 | 1.00 | 19.51 | O |
| ATOM | 4771 | CB  | TYR | C | 186 | 34.399 | -18.629 | 30.476 | 1.00 | 17.96 | C |
| ATOM | 4772 | CG  | TYR | C | 186 | 35.034 | -17.252 | 30.447 | 1.00 | 17.43 | C |
| ATOM | 4773 | CD1 | TYR | C | 186 | 35.941 | -16.917 | 29.456 | 1.00 | 19.18 | C |
| ATOM | 4774 | CD2 | TYR | C | 186 | 34.792 | -16.339 | 31.456 | 1.00 | 20.20 | C |
| ATOM | 4775 | CE1 | TYR | C | 186 | 36.576 | -15.704 | 29.446 | 1.00 | 19.11 | C |
| ATOM | 4776 | CE2 | TYR | C | 186 | 35.391 | -15.089 | 31.444 | 1.00 | 19.56 | C |
| ATOM | 4777 | CZ  | TYR | C | 186 | 36.310 | -14.796 | 30.432 | 1.00 | 20.14 | C |
| ATOM | 4778 | OH  | TYR | C | 186 | 36.951 | -13.561 | 30.370 | 1.00 | 22.13 | O |
| ATOM | 4779 | N   | GLU | C | 187 | 35.782 | -19.513 | 33.179 | 1.00 | 17.53 | N |
| ATOM | 4780 | CA  | GLU | C | 187 | 36.529 | -19.237 | 34.412 | 1.00 | 18.68 | C |
| ATOM | 4781 | C   | GLU | C | 187 | 37.470 | -20.368 | 34.893 | 1.00 | 20.05 | C |
| ATOM | 4782 | O   | GLU | C | 187 | 38.080 | -20.262 | 35.960 | 1.00 | 22.71 | O |
| ATOM | 4783 | CB  | GLU | C | 187 | 35.539 | -18.740 | 35.484 | 1.00 | 19.36 | C |
| ATOM | 4784 | CG  | GLU | C | 187 | 34.952 | -17.437 | 35.064 | 1.00 | 20.97 | C |
| ATOM | 4785 | CD  | GLU | C | 187 | 33.925 | -16.832 | 35.989 | 1.00 | 22.08 | C |
| ATOM | 4786 | OE1 | GLU | C | 187 | 33.301 | -17.535 | 36.810 | 1.00 | 22.07 | O |
| ATOM | 4787 | OE2 | GLU | C | 187 | 33.710 | -15.592 | 35.832 | 1.00 | 26.11 | O |
| ATOM | 4788 | N   | ARG | C | 188 | 37.674 | -21.363 | 34.057 | 1.00 | 21.86 | N |
| ATOM | 4789 | CA  | ARG | C | 188 | 38.650 | -22.424 | 34.309 | 1.00 | 21.83 | C |
| ATOM | 4790 | C   | ARG | C | 188 | 39.922 | -22.310 | 33.472 | 1.00 | 23.00 | C |
| ATOM | 4791 | O   | ARG | C | 188 | 40.827 | -23.145 | 33.595 | 1.00 | 23.26 | O |
| ATOM | 4792 | CB  | ARG | C | 188 | 37.988 | -23.748 | 34.028 | 1.00 | 23.96 | C |
| ATOM | 4793 | CG  | ARG | C | 188 | 37.006 | -24.126 | 35.097 | 1.00 | 25.83 | C |
| ATOM | 4794 | CD  | ARG | C | 188 | 36.331 | -25.428 | 34.729 | 1.00 | 30.00 | C |
| ATOM | 4795 | NE  | ARG | C | 188 | 35.241 | -25.723 | 35.652 | 1.00 | 32.67 | N |
| ATOM | 4796 | CZ  | ARG | C | 188 | 35.316 | -26.580 | 36.668 | 1.00 | 36.91 | C |
| ATOM | 4797 | NH1 | ARG | C | 188 | 36.444 | -27.261 | 36.903 | 1.00 | 36.77 | N |
| ATOM | 4798 | NH2 | ARG | C | 188 | 34.254 | -26.774 | 37.454 | 1.00 | 36.92 | N |
| ATOM | 4799 | N   | HIS | C | 189 | 39.976 | -21.288 | 32.614 | 1.00 | 20.45 | N |
| ATOM | 4800 | CA  | HIS | C | 189 | 41.084 | -21.098 | 31.678 | 1.00 | 19.38 | C |

```
ATOM   4801  C    HIS C 189   41.506 -19.672  31.613  1.00 19.12        C
ATOM   4802  O    HIS C 189   40.775 -18.797  32.044  1.00 19.65        O
ATOM   4803  CB   HIS C 189   40.695 -21.581  30.295  1.00 19.12        C
ATOM   4804  CG   HIS C 189   40.463 -23.052  30.244  1.00 22.64        C
ATOM   4805  ND1  HIS C 189   39.207 -23.598  30.148  1.00 25.21        N
ATOM   4806  CD2  HIS C 189   41.325 -24.091  30.328  1.00 25.39        C
ATOM   4807  CE1  HIS C 189   39.303 -24.917  30.168  1.00 26.89        C
ATOM   4808  NE2  HIS C 189   40.577 -25.239  30.276  1.00 27.83        N'
ATOM   4809  N    ASN C 190   42.713 -19.425  31.120  1.00 17.25        N
ATOM   4810  CA   ASN C 190   43.248 -18.066  31.118  1.00 18.29        C
ATOM   4811  C    ASN C 190   43.340 -17.425  29.739  1.00 19.06        C
ATOM   4812  O    ASN C 190   42.802 -16.341  29.537  1.00 22.24        O
ATOM   4813  CB   ASN C 190   44.597 -17.986  31.816  1.00 17.88        C
ATOM   4814  CG   ASN C 190   44.996 -16.535  32.152  1.00 18.67        C
ATOM   4815  OD1  ASN C 190   44.206 -15.787  32.712  1.00 20.24        O
ATOM   4816  ND2  ASN C 190   46.231 -16.159  31.799  1.00 22.80        N
ATOM   4817  N    SER C 191   44.045 -18.071  28.818  1.00 19.84        N
ATOM   4818  CA   SER C 191   44.432 -17.440  27.551  1.00 19.23        C
ATOM   4819  C    SER C 191   43.514 -17.876  26.419  1.00 18.84        C
ATOM   4820  O    SER C 191   43.484 -19.055  26.021  1.00 19.93        O
ATOM   4821  CB   SER C 191   45.862 -17.809  27.181  1.00 19.66        C
ATOM   4822  OG   SER C 191   46.291 -17.064  26.060  1.00 22.76        O
ATOM   4823  N    TYR C 192   42.801 -16.899  25.897  1.00 18.91        N
ATOM   4824  CA   TYR C 192   41.830 -17.089  24.809  1.00 17.94        C
ATOM   4825  C    TYR C 192   42.397 -16.425  23.611  1.00 17.74        C
ATOM   4826  O    TYR C 192   42.709 -15.218  23.656  1.00 18.28        O
ATOM   4827  CB   TYR C 192   40.478 -16.478  25.160  1.00 18.82        C
ATOM   4828  CG   TYR C 192   39.810 -17.241  26.258  1.00 17.18        C
ATOM   4829  CD1  TYR C 192   39.065 -18.373  25.975  1.00 15.73        C
ATOM   4830  CD2  TYR C 192   40.014 -16.896  27.585  1.00 15.22        C
ATOM   4831  CE1  TYR C 192   38.486 -19.104  26.957  1.00 18.31        C
ATOM   4832  CE2  TYR C 192   39.446 -17.653  28.597  1.00 18.54        C
ATOM   4833  CZ   TYR C 192   38.706 -18.757  28.270  1.00 18.23        C
ATOM   4834  OH   TYR C 192   38.127 -19.519  29.226  1.00 20.07        O
ATOM   4835  N    THR C 193   42.549 -17.187  22.535  1.00 16.91        N
ATOM   4836  CA   THR C 193   43.192 -16.686  21.332  1.00 18.46        C
ATOM   4837  C    THR C 193   42.361 -16.913  20.085  1.00 19.03        C
ATOM   4838  O    THR C 193   41.827 -17.989  19.865  1.00 17.26        O
ATOM   4839  CB   THR C 193   44.558 -17.328  21.068  1.00 20.48        C
ATOM   4840  OG1  THR C 193   44.418 -18.762  21.020  1.00 20.26        O
ATOM   4841  CG2  THR C 193   45.518 -16.943  22.148  1.00 20.03        C
ATOM   4842  N    CYS C 194   42.295 -15.863  19.280  1.00 17.41        N
ATOM   4843  CA  ACYS C 194   41.807 -15.878  17.903  0.50 18.45        C
ATOM   4844  CA  BCYS C 194   41.834 -16.044  17.913  0.50 17.93        C
ATOM   4845  C    CYS C 194   43.034 -15.933  16.973  1.00 18.20        C
ATOM   4846  O    CYS C 194   43.869 -15.016  17.033  1.00 18.46        O
ATOM   4847  CB  ACYS C 194   41.041 -14.559  17.703  0.50 19.12        C
ATOM   4848  CB  BCYS C 194   40.701 -15.113  17.539  0.50 17.97        C
ATOM   4849  SG  ACYS C 194   40.456 -14.123  16.073  0.50 17.92        S
ATOM   4850  SG  BCYS C 194   41.173 -13.418  17.446  0.50 15.28        S
ATOM   4851  N    GLU C 195   43.116 -16.949  16.124  1.00 20.03        N
ATOM   4852  CA   GLU C 195   44.297 -17.230  15.312  1.00 20.17        C
ATOM   4853  C    GLU C 195   43.870 -17.384  13.855  1.00 19.97        C
ATOM   4854  O    GLU C 195   43.204 -18.340  13.493  1.00 21.14        O
ATOM   4855  CB   GLU C 195   44.967 -18.528  15.833  1.00 21.27        C
ATOM   4856  CG   GLU C 195   45.095 -18.548  17.330  1.00 24.77        C
ATOM   4857  CD   GLU C 195   45.986 -19.632  17.877  1.00 27.91        C
ATOM   4858  OE1  GLU C 195   46.744 -20.271  17.088  1.00 26.27        O
ATOM   4859  OE2  GLU C 195   45.896 -19.840  19.118  1.00 30.05        O
ATOM   4860  N    ALA C 196   44.291 -16.437  13.038  1.00 18.51        N
ATOM   4861  CA   ALA C 196   43.880 -16.335  11.656  1.00 19.51        C
ATOM   4862  C    ALA C 196   45.009 -16.710  10.707  1.00 21.31        C
ATOM   4863  O    ALA C 196   46.153 -16.306  10.906  1.00 21.74        O
ATOM   4864  CB   ALA C 196   43.422 -14.949  11.382  1.00 19.70        C
ATOM   4865  N    THR C 197   44.654 -17.460   9.673  1.00 21.48        N
```

| ATOM | 4866 | CA | THR C 197 | 45.544 | -17.767 | 8.563 | 1.00 | 23.03 | C |
|------|------|-----|-----------|--------|---------|-------|------|-------|---|
| ATOM | 4867 | C | THR C 197 | 45.074 | -17.025 | 7.317 | 1.00 | 22.32 | C |
| ATOM | 4868 | O | THR C 197 | 43.919 | -17.161 | 6.883 | 1.00 | 22.07 | O |
| ATOM | 4869 | CB | THR C 197 | 45.590 | -19.266 | 8.337 | 1.00 | 24.72 | C |
| ATOM | 4870 | OG1 | THR C 197 | 46.111 | -19.848 | 9.527 | 1.00 | 28.27 | O |
| ATOM | 4871 | CG2 | THR C 197 | 46.524 | -19.592 | 7.201 | 1.00 | 26.42 | C |
| ATOM | 4872 | N | HIS C 198 | 45.981 | -16.238 | 6.761 | 1.00 | 23.33 | N |
| ATOM | 4873 | CA | HIS C 198 | 45.675 | -15.387 | 5.622 | 1.00 | 23.44 | C |
| ATOM | 4874 | C | HIS C 198 | 46.912 | -15.302 | 4.749 | 1.00 | 25.09 | C |
| ATOM | 4875 | O | HIS C 198 | 48.027 | -15.354 | 5.244 | 1.00 | 24.46 | O |
| ATOM | 4876 | CB | HIS C 198 | 45.267 | -13.990 | 6.100 | 1.00 | 22.46 | C |
| ATOM | 4877 | CG | HIS C 198 | 44.738 | -13.106 | 5.012 | 1.00 | 20.48 | C |
| ATOM | 4878 | ND1 | HIS C 198 | 45.444 | -12.036 | 4.502 | 1.00 | 22.42 | N |
| ATOM | 4879 | CD2 | HIS C 198 | 43.571 | -13.149 | 4.326 | 1.00 | 21.43 | C |
| ATOM | 4880 | CE1 | HIS C 198 | 44.732 | -11.462 | 3.547 | 1.00 | 21.87 | C |
| ATOM | 4881 | NE2 | HIS C 198 | 43.588 | -12.114 | 3.420 | 1.00 | 18.94 | N |
| ATOM | 4882 | N | LYS C 199 | 46.726 | -15.107 | 3.456 | 1.00 | 26.91 | N |
| ATOM | 4883 | CA | LYS C 199 | 47.883 | -15.133 | 2.583 | 1.00 | 29.17 | C |
| ATOM | 4884 | C | LYS C 199 | 48.944 | -14.065 | 2.868 | 1.00 | 28.59 | C |
| ATOM | 4885 | O | LYS C 199 | 50.090 | -14.186 | 2.421 | 1.00 | 28.14 | O |
| ATOM | 4886 | CB | LYS C 199 | 47.462 | -15.129 | 1.131 | 1.00 | 31.16 | C |
| ATOM | 4887 | CG | LYS C 199 | 46.692 | -13.941 | 0.681 | 1.00 | 35.25 | C |
| ATOM | 4888 | CD | LYS C 199 | 47.285 | -13.413 | -0.633 | 1.00 | 37.44 | C |
| ATOM | 4889 | CE | LYS C 199 | 47.489 | -14.495 | -1.678 | 1.00 | 37.78 | C |
| ATOM | 4890 | NZ | LYS C 199 | 48.738 | -14.171 | -2.425 | 1.00 | 39.07 | N |
| ATOM | 4891 | N | THR C 200 | 48.593 | -13.035 | 3.626 | 1.00 | 28.16 | N |
| ATOM | 4892 | CA | THR C 200 | 49.529 | -11.969 | 3.970 | 1.00 | 28.61 | C |
| ATOM | 4893 | C | THR C 200 | 50.485 | -12.273 | 5.148 | 1.00 | 30.62 | C |
| ATOM | 4894 | O | THR C 200 | 51.283 | -11.421 | 5.550 | 1.00 | 32.01 | O |
| ATOM | 4895 | CB | THR C 200 | 48.751 | -10.686 | 4.309 | 1.00 | 27.61 | C |
| ATOM | 4896 | OG1 | THR C 200 | 47.860 | -10.918 | 5.423 | 1.00 | 27.62 | O |
| ATOM | 4897 | CG2 | THR C 200 | 47.960 | -10.206 | 3.095 | 1.00 | 26.52 | C |
| ATOM | 4898 | N | SER C 201 | 50.398 | -13.476 | 5.703 | 1.00 | 32.05 | N |
| ATOM | 4899 | CA | SER C 201 | 51.262 | -13.888 | 6.810 | 1.00 | 32.76 | C |
| ATOM | 4900 | C | SER C 201 | 51.620 | -15.333 | 6.574 | 1.00 | 33.06 | C |
| ATOM | 4901 | O | SER C 201 | 50.751 | -16.122 | 6.246 | 1.00 | 32.11 | O |
| ATOM | 4902 | CB | SER C 201 | 50.524 | -13.758 | 8.150 | 1.00 | 33.18 | C |
| ATOM | 4903 | OG | SER C 201 | 51.338 | -14.136 | 9.269 | 1.00 | 34.20 | O |
| ATOM | 4904 | N | THR C 202 | 52.888 | -15.680 | 6.775 | 1.00 | 34.67 | N |
| ATOM | 4905 | CA | THR C 202 | 53.323 | -17.077 | 6.725 | 1.00 | 35.52 | C |
| ATOM | 4906 | C | THR C 202 | 53.002 | -17.783 | 8.047 | 1.00 | 35.04 | C |
| ATOM | 4907 | O | THR C 202 | 52.947 | -18.998 | 8.110 | 1.00 | 35.93 | O |
| ATOM | 4908 | CB | THR C 202 | 54.862 | -17.203 | 6.427 | 1.00 | 37.31 | C |
| ATOM | 4909 | OG1 | THR C 202 | 55.624 | -16.618 | 7.487 | 1.00 | 40.83 | O |
| ATOM | 4910 | CG2 | THR C 202 | 55.223 | -16.511 | 5.134 | 1.00 | 38.15 | C |
| ATOM | 4911 | N | SER C 203 | 52.792 | -17.010 | 9.103 | 1.00 | 33.98 | N |
| ATOM | 4912 | CA | SER C 203 | 52.424 | -17.557 | 10.409 | 1.00 | 34.55 | C |
| ATOM | 4913 | C | SER C 203 | 51.025 | -17.105 | 10.807 | 1.00 | 32.95 | C |
| ATOM | 4914 | O | SER C 203 | 50.544 | -16.063 | 10.335 | 1.00 | 32.57 | O |
| ATOM | 4915 | CB | SER C 203 | 53.402 | -17.070 | 11.475 | 1.00 | 34.67 | C |
| ATOM | 4916 | OG | SER C 203 | 53.435 | -15.651 | 11.494 | 1.00 | 38.37 | O |
| ATOM | 4917 | N | PRO C 204 | 50.388 | -17.865 | 11.712 | 1.00 | 32.07 | N |
| ATOM | 4918 | CA | PRO C 204 | 49.117 | -17.447 | 12.265 | 1.00 | 31.14 | C |
| ATOM | 4919 | C | PRO C 204 | 49.160 | -16.007 | 12.761 | 1.00 | 29.42 | C |
| ATOM | 4920 | O | PRO C 204 | 50.146 | -15.599 | 13.375 | 1.00 | 30.14 | O |
| ATOM | 4921 | CB | PRO C 204 | 48.947 | -18.394 | 13.450 | 1.00 | 31.05 | C |
| ATOM | 4922 | CG | PRO C 204 | 49.669 | -19.614 | 13.058 | 1.00 | 32.51 | C |
| ATOM | 4923 | CD | PRO C 204 | 50.825 | -19.156 | 12.276 | 1.00 | 31.95 | C |
| ATOM | 4924 | N | ILE C 205 | 48.089 | -15.257 | 12.513 | 1.00 | 27.86 | N |
| ATOM | 4925 | CA | ILE C 205 | 47.932 | -13.918 | 13.027 | 1.00 | 26.62 | C |
| ATOM | 4926 | C | ILE C 205 | 47.137 | -14.082 | 14.319 | 1.00 | 25.40 | C |
| ATOM | 4927 | O | ILE C 205 | 46.012 | -14.541 | 14.263 | 1.00 | 25.62 | O |
| ATOM | 4928 | CB | ILE C 205 | 47.151 | -13.040 | 12.027 | 1.00 | 28.00 | C |
| ATOM | 4929 | CG1 | ILE C 205 | 47.883 | -12.960 | 10.669 | 1.00 | 28.12 | C |
| ATOM | 4930 | CG2 | ILE C 205 | 46.953 | -11.643 | 12.591 | 1.00 | 29.01 | C |

| ATOM | 4931 | CD1 | ILE | C | 205 | 47.001 | -12.639 | 9.465 | 1.00 | 28.33 | C |
|------|------|-----|-----|---|-----|--------|---------|-------|------|-------|---|
| ATOM | 4932 | N | VAL | C | 206 | 47.715 | -13.737 | 15.464 | 1.00 | 23.57 | N |
| ATOM | 4933 | CA | VAL | C | 206 | 47.079 | -14.058 | 16.741 | 1.00 | 24.16 | C |
| ATOM | 4934 | C | VAL | C | 206 | 46.840 | -12.873 | 17.641 | 1.00 | 23.28 | C |
| ATOM | 4935 | O | VAL | C | 206 | 47.708 | -12.024 | 17.793 | 1.00 | 23.83 | O |
| ATOM | 4936 | CB | VAL | C | 206 | 47.923 | -15.091 | 17.510 | 1.00 | 25.12 | C |
| ATOM | 4937 | CG1 | VAL | C | 206 | 47.227 | -15.526 | 18.855 | 1.00 | 23.36 | C |
| ATOM | 4938 | CG2 | VAL | C | 206 | 48.203 | -16.284 | 16.635 | 1.00 | 25.43 | C |
| ATOM | 4939 | N | LYS | C | 207 | 45.668 | -12.838 | 18.270 | 1.00 | 20.72 | N |
| ATOM | 4940 | CA | LYS | C | 207 | 45.386 | -11.911 | 19.340 | 1.00 | 22.59 | C |
| ATOM | 4941 | C | LYS | C | 207 | 44.848 | -12.709 | 20.506 | 1.00 | 21.42 | C |
| ATOM | 4942 | O | LYS | C | 207 | 44.083 | -13.664 | 20.330 | 1.00 | 22.08 | O |
| ATOM | 4943 | CB | LYS | C | 207 | 44.355 | -10.875 | 18.929 | 1.00 | 22.69 | C |
| ATOM | 4944 | CG | LYS | C | 207 | 44.872 | -9.843 | 18.004 | 1.00 | 26.39 | C |
| ATOM | 4945 | CD | LYS | C | 207 | 45.541 | -8.750 | 18.779 | 1.00 | 26.87 | C |
| ATOM | 4946 | CE | LYS | C | 207 | 46.123 | -7.696 | 17.836 | 1.00 | 28.90 | C |
| ATOM | 4947 | NZ | LYS | C | 207 | 47.245 | -6.912 | 18.462 | 1.00 | 31.79 | N |
| ATOM | 4948 | N | SER | C | 208 | 45.241 | -12.306 | 21.698 | 1.00 | 21.24 | N |
| ATOM | 4949 | CA | SER | C | 208 | 44.855 | -13.020 | 22.893 | 1.00 | 20.99 | C |
| ATOM | 4950 | C | SER | C | 208 | 44.222 | -12.123 | 23.904 | 1.00 | 20.68 | C |
| ATOM | 4951 | O | SER | C | 208 | 44.489 | -10.909 | 23.927 | 1.00 | 22.33 | O |
| ATOM | 4952 | CB | SER | C | 208 | 46.059 | -13.747 | 23.516 | 1.00 | 21.46 | C |
| ATOM | 4953 | OG | SER | C | 208 | 46.924 | -12.859 | 24.235 | 1.00 | 24.39 | O |
| ATOM | 4954 | N | PHE | C | 209 | 43.334 | -12.708 | 24.695 | 1.00 | 20.92 | N |
| ATOM | 4955 | CA | PHE | C | 209 | 42.836 | -12.069 | 25.905 | 1.00 | 21.29 | C |
| ATOM | 4956 | C | PHE | C | 209 | 43.136 | -13.015 | 27.058 | 1.00 | 21.29 | C |
| ATOM | 4957 | O | PHE | C | 209 | 42.785 | -14.190 | 26.989 | 1.00 | 23.66 | O |
| ATOM | 4958 | CB | PHE | C | 209 | 41.307 | -11.823 | 25.820 | 1.00 | 20.31 | C |
| ATOM | 4959 | CG | PHE | C | 209 | 40.700 | -11.447 | 27.151 | 1.00 | 20.87 | C |
| ATOM | 4960 | CD1 | PHE | C | 209 | 40.914 | -10.182 | 27.686 | 1.00 | 22.19 | C |
| ATOM | 4961 | CD2 | PHE | C | 209 | 40.020 | -12.382 | 27.924 | 1.00 | 19.54 | C |
| ATOM | 4962 | CE1 | PHE | C | 209 | 40.420 | -9.830 | 28.952 | 1.00 | 20.31 | C |
| ATOM | 4963 | CE2 | PHE | C | 209 | 39.554 | -12.050 | 29.178 | 1.00 | 20.14 | C |
| ATOM | 4964 | CZ | PHE | C | 209 | 39.749 | -10.728 | 29.693 | 1.00 | 22.81 | C |
| ATOM | 4965 | N | ASN | C | 210 | 43.774 | -12.503 | 28.114 | 1.00 | 21.99 | N |
| ATOM | 4966 | CA | ASN | C | 210 | 44.024 | -13.265 | 29.321 | 1.00 | 22.03 | C |
| ATOM | 4967 | C | ASN | C | 210 | 43.028 | -12.881 | 30.381 | 1.00 | 22.63 | C |
| ATOM | 4968 | O | ASN | C | 210 | 42.857 | -11.700 | 30.710 | 1.00 | 21.38 | O |
| ATOM | 4969 | CB | ASN | C | 210 | 45.450 | -13.020 | 29.852 | 1.00 | 22.83 | C |
| ATOM | 4970 | CG | ASN | C | 210 | 46.518 | -13.518 | 28.914 | 1.00 | 25.74 | C |
| ATOM | 4971 | OD1 | ASN | C | 210 | 46.337 | -14.517 | 28.243 | 1.00 | 26.26 | O |
| ATOM | 4972 | ND2 | ASN | C | 210 | 47.636 | -12.789 | 28.833 | 1.00 | 28.40 | N |
| ATOM | 4973 | N | ARG | C | 211 | 42.333 | -13.876 | 30.877 | 1.00 | 22.39 | N |
| ATOM | 4974 | CA | ARG | C | 211 | 41.257 | -13.655 | 31.813 | 1.00 | 23.19 | C |
| ATOM | 4975 | C | ARG | C | 211 | 41.758 | -13.015 | 33.107 | 1.00 | 25.35 | C |
| ATOM | 4976 | O | ARG | C | 211 | 41.060 | -12.187 | 33.678 | 1.00 | 23.85 | O |
| ATOM | 4977 | CB | ARG | C | 211 | 40.529 | -14.961 | 32.091 | 1.00 | 23.49 | C |
| ATOM | 4978 | CG | ARG | C | 211 | 39.233 | -14.784 | 32.871 | 1.00 | 23.84 | C |
| ATOM | 4979 | CD | ARG | C | 211 | 38.511 | -16.074 | 33.100 | 1.00 | 24.80 | C |
| ATOM | 4980 | NE | ARG | C | 211 | 39.392 | -17.088 | 33.675 | 1.00 | 22.24 | N |
| ATOM | 4981 | CZ | ARG | C | 211 | 39.622 | -17.269 | 34.962 | 1.00 | 23.16 | C |
| ATOM | 4982 | NH1 | ARG | C | 211 | 38.993 | -16.563 | 35.887 | 1.00 | 26.86 | N |
| ATOM | 4983 | NH2 | ARG | C | 211 | 40.456 | -18.213 | 35.320 | 1.00 | 26.67 | N |
| ATOM | 4984 | N | ASN | C | 212 | 42.938 | -13.399 | 33.589 | 1.00 | 27.86 | N |
| ATOM | 4985 | CA | ASN | C | 212 | 43.563 | -12.625 | 34.679 | 1.00 | 31.55 | C |
| ATOM | 4986 | C | ASN | C | 212 | 44.140 | -11.374 | 34.060 | 1.00 | 33.32 | C |
| ATOM | 4987 | O | ASN | C | 212 | 45.251 | -11.393 | 33.543 | 1.00 | 31.63 | O |
| ATOM | 4988 | CB | ASN | C | 212 | 44.676 | -13.378 | 35.410 | 1.00 | 32.85 | C |
| ATOM | 4989 | CG | ASN | C | 212 | 45.477 | -12.463 | 36.370 | 1.00 | 36.31 | C |
| ATOM | 4990 | OD1 | ASN | C | 212 | 45.096 | -11.298 | 36.635 | 1.00 | 39.47 | O |
| ATOM | 4991 | ND2 | ASN | C | 212 | 46.592 | -12.977 | 36.876 | 1.00 | 35.39 | N |
| ATOM | 4992 | N | GLU | C | 213 | 43.369 | -10.304 | 34.096 | 1.00 | 37.38 | N |
| ATOM | 4993 | CA | GLU | C | 213 | 43.701 | -9.108 | 33.350 | 1.00 | 41.85 | C |
| ATOM | 4994 | C | GLU | C | 213 | 44.061 | -7.951 | 34.282 | 1.00 | 45.27 | C |
| ATOM | 4995 | O | GLU | C | 213 | 44.852 | -7.094 | 33.903 | 1.00 | 46.10 | O |

| ATOM | 4996 | CB | GLU | C | 213 | 42.551 | -8.729 | 32.412 | 1.00 | 42.54 | C |
|------|------|------|-----|---|-----|--------|--------|--------|------|-------|---|
| ATOM | 4997 | CG | GLU | C | 213 | 41.366 | -8.125 | 33.103 | 1.00 | 42.70 | C |
| ATOM | 4998 | CD | GLU | C | 213 | 40.252 | -7.768 | 32.150 | 1.00 | 42.95 | C |
| ATOM | 4999 | OE1 | GLU | C | 213 | 40.430 | -6.907 | 31.248 | 1.00 | 42.68 | O |
| ATOM | 5000 | OE2 | GLU | C | 213 | 39.178 | -8.359 | 32.333 | 1.00 | 42.04 | O |
| ATOM | 5001 | N | CYS | C | 214 | 43.481 | -7.929 | 35.486 | 1.00 | 48.57 | N |
| ATOM | 5002 | CA | CYS | C | 214 | 43.846 | -6.940 | 36.511 | 1.00 | 50.77 | C |
| ATOM | 5003 | C | CYS | C | 214 | 43.555 | -7.456 | 37.924 | 1.00 | 51.24 | C |
| ATOM | 5004 | O | CYS | C | 214 | 43.369 | -8.660 | 38.151 | 1.00 | 51.78 | O |
| ATOM | 5005 | CB | CYS | C | 214 | 43.120 | -5.609 | 36.268 | 1.00 | 51.23 | C |
| ATOM | 5006 | SG | CYS | C | 214 | 41.336 | -5.640 | 36.622 | 1.00 | 56.54 | S |
| TER | 5007 | | CYS | C | 214 | | | | | | |
| ATOM | 5008 | N | GLU | D | 1 | 9.900 | 10.788 | -9.858 | 1.00 | 33.12 | N |
| ATOM | 5009 | CA | GLU | D | 1 | 10.582 | 9.474 | -9.734 | 1.00 | 32.03 | C |
| ATOM | 5010 | C | GLU | D | 1 | 9.543 | 8.401 | -9.373 | 1.00 | 30.80 | C |
| ATOM | 5011 | O | GLU | D | 1 | 8.632 | 8.651 | -8.592 | 1.00 | 31.49 | O |
| ATOM | 5012 | CB | GLU | D | 1 | 11.652 | 9.538 | -8.679 | 1.00 | 32.44 | C |
| ATOM | 5013 | N | VAL | D | 2 | 9.697 | 7.214 | -9.937 | 1.00 | 28.89 | N |
| ATOM | 5014 | CA | VAL | D | 2 | 8.828 | 6.097 | -9.597 | 1.00 | 27.74 | C |
| ATOM | 5015 | C | VAL | D | 2 | 9.364 | 5.501 | -8.303 | 1.00 | 26.89 | C |
| ATOM | 5016 | O | VAL | D | 2 | 10.580 | 5.351 | -8.121 | 1.00 | 28.21 | O |
| ATOM | 5017 | CB | VAL | D | 2 | 8.792 | 5.056 | -10.732 | 1.00 | 27.51 | C |
| ATOM | 5018 | CG1 | VAL | D | 2 | 8.055 | 3.814 | -10.326 | 1.00 | 27.86 | C |
| ATOM | 5019 | CG2 | VAL | D | 2 | 8.120 | 5.644 | -11.980 | 1.00 | 28.65 | C |
| ATOM | 5020 | N | LYS | D | 3 | 8.458 | 5.214 | -7.383 | 1.00 | 26.83 | N |
| ATOM | 5021 | CA | LYS | D | 3 | 8.781 | 4.542 | -6.133 | 1.00 | 27.70 | C |
| ATOM | 5022 | C | LYS | D | 3 | 7.797 | 3.376 | -5.926 | 1.00 | 26.01 | C |
| ATOM | 5023 | O | LYS | D | 3 | 6.576 | 3.508 | -6.072 | 1.00 | 25.63 | O |
| ATOM | 5024 | CB | LYS | D | 3 | 8.742 | 5.498 | -4.937 | 1.00 | 29.59 | C |
| ATOM | 5025 | CG | LYS | D | 3 | 9.798 | 6.564 | -4.963 | 1.00 | 33.19 | C |
| ATOM | 5026 | CD | LYS | D | 3 | 9.448 | 7.750 | -4.069 | 1.00 | 34.48 | C |
| ATOM | 5027 | CE | LYS | D | 3 | 10.633 | 8.702 | -3.936 | 1.00 | 35.67 | C |
| ATOM | 5028 | NZ | LYS | D | 3 | 11.461 | 8.824 | -5.192 | 1.00 | 37.99 | N |
| ATOM | 5029 | N | VAL | D | 4 | 8.378 | 2.230 | -5.597 | 1.00 | 25.19 | N |
| ATOM | 5030 | CA | VAL | D | 4 | 7.646 | 1.014 | -5.300 | 1.00 | 25.16 | C |
| ATOM | 5031 | C | VAL | D | 4 | 8.036 | 0.618 | -3.875 | 1.00 | 23.69 | C |
| ATOM | 5032 | O | VAL | D | 4 | 9.226 | 0.439 | -3.588 | 1.00 | 24.14 | O |
| ATOM | 5033 | CB | VAL | D | 4 | 8.047 | -0.084 | -6.280 | 1.00 | 25.09 | C |
| ATOM | 5034 | CG1 | VAL | D | 4 | 7.309 | -1.406 | -5.971 | 1.00 | 23.50 | C |
| ATOM | 5035 | CG2 | VAL | D | 4 | 7.827 | 0.379 | -7.731 | 1.00 | 26.16 | C |
| ATOM | 5036 | N | GLU | D | 5 | 7.059 | 0.541 | -2.973 | 1.00 | 24.46 | N |
| ATOM | 5037 | CA | GLU | D | 5 | 7.332 | 0.310 | -1.564 | 1.00 | 25.14 | C |
| ATOM | 5038 | C | GLU | D | 5 | 6.442 | -0.783 | -0.957 | 1.00 | 23.96 | C |
| ATOM | 5039 | O | GLU | D | 5 | 5.264 | -0.586 | -0.775 | 1.00 | 23.60 | O |
| ATOM | 5040 | CB | GLU | D | 5 | 7.191 | 1.619 | -0.753 | 1.00 | 28.04 | C |
| ATOM | 5041 | CG | GLU | D | 5 | 7.528 | 1.448 | 0.731 | 1.00 | 32.13 | C |
| ATOM | 5042 | CD | GLU | D | 5 | 8.248 | 2.637 | 1.349 | 1.00 | 36.06 | C |
| ATOM | 5043 | OE1 | GLU | D | 5 | 9.025 | 2.394 | 2.311 | 1.00 | 41.62 | O |
| ATOM | 5044 | OE2 | GLU | D | 5 | 8.079 | 3.792 | 0.871 | 1.00 | 39.47 | O |
| ATOM | 5045 | N | GLU | D | 6 | 7.067 | -1.908 | -0.619 | 1.00 | 24.86 | N |
| ATOM | 5046 | CA | GLU | D | 6 | 6.397 | -3.076 | -0.066 | 1.00 | 24.21 | C |
| ATOM | 5047 | C | GLU | D | 6 | 6.301 | -2.959 | 1.452 | 1.00 | 24.31 | C |
| ATOM | 5048 | O | GLU | D | 6 | 7.143 | -2.329 | 2.082 | 1.00 | 26.44 | O |
| ATOM | 5049 | CB | GLU | D | 6 | 7.198 | -4.340 | -0.408 | 1.00 | 23.89 | C |
| ATOM | 5050 | CG | GLU | D | 6 | 7.350 | -4.625 | -1.890 | 1.00 | 24.05 | C |
| ATOM | 5051 | CD | GLU | D | 6 | 8.535 | -3.972 | -2.517 | 1.00 | 23.11 | C |
| ATOM | 5052 | OE1 | GLU | D | 6 | 9.134 | -3.082 | -1.879 | 1.00 | 24.01 | O |
| ATOM | 5053 | OE2 | GLU | D | 6 | 8.913 | -4.370 | -3.646 | 1.00 | 25.66 | O |
| ATOM | 5054 | N | SER | D | 7 | 5.276 | -3.573 | 2.028 | 1.00 | 25.36 | N |
| ATOM | 5055 | CA | SER | D | 7 | 5.131 | -3.671 | 3.492 | 1.00 | 25.13 | C |
| ATOM | 5056 | C | SER | D | 7 | 4.335 | -4.919 | 3.883 | 1.00 | 24.58 | C |
| ATOM | 5057 | O | SER | D | 7 | 3.763 | -5.614 | 3.031 | 1.00 | 23.84 | O |
| ATOM | 5058 | CB | SER | D | 7 | 4.440 | -2.415 | 4.041 | 1.00 | 24.59 | C |
| ATOM | 5059 | OG | SER | D | 7 | 3.175 | -2.207 | 3.439 | 1.00 | 24.60 | O |
| ATOM | 5060 | N | GLY | D | 8 | 4.275 | -5.196 | 5.176 | 1.00 | 25.00 | N |

| ATOM | 5061 | CA | GLY | D | 8 | 3.414 | -6.280 | 5.673 | 1.00 | 26.00 | C |
|------|------|-----|-----|---|----|--------|---------|--------|------|-------|---|
| ATOM | 5062 | C | GLY | D | 8 | 4.103 | -7.572 | 6.022 | 1.00 | 25.36 | C |
| ATOM | 5063 | O | GLY | D | 8 | 3.502 | -8.465 | 6.598 | 1.00 | 26.22 | O |
| ATOM | 5064 | N | GLY | D | 9 | 5.372 | -7.692 | 5.668 | 1.00 | 27.05 | N |
| ATOM | 5065 | CA | GLY | D | 9 | 6.112 | -8.897 | 5.916 | 1.00 | 25.93 | C |
| ATOM | 5066 | C | GLY | D | 9 | 6.420 | -9.060 | 7.384 | 1.00 | 26.20 | C |
| ATOM | 5067 | O | GLY | D | 9 | 6.348 | -8.140 | 8.174 | 1.00 | 29.43 | O |
| ATOM | 5068 | N | GLY | D | 10 | 6.757 | -10.248 | 7.788 | 1.00 | 23.97 | N |
| ATOM | 5069 | CA | GLY | D | 10 | 6.959 | -10.440 | 9.204 | 1.00 | 22.95 | C |
| ATOM | 5070 | C | GLY | D | 10 | 7.064 | -11.908 | 9.441 | 1.00 | 22.53 | C |
| ATOM | 5071 | O | GLY | D | 10 | 7.271 | -12.663 | 8.508 | 1.00 | 22.13 | O |
| ATOM | 5072 | N | LEU | D | 11 | 6.884 | -12.307 | 10.690 | 1.00 | 24.02 | N |
| ATOM | 5073 | CA | LEU | D | 11 | 7.092 | -13.690 | 11.060 | 1.00 | 23.51 | C |
| ATOM | 5074 | C | LEU | D | 11 | 5.731 | -14.363 | 11.204 | 1.00 | 26.40 | C |
| ATOM | 5075 | O | LEU | D | 11 | 4.797 | -13.785 | 11.769 | 1.00 | 26.88 | O |
| ATOM | 5076 | CB | LEU | D | 11 | 7.845 | -13.764 | 12.383 | 1.00 | 23.32 | C |
| ATOM | 5077 | CG | LEU | D | 11 | 7.945 | -15.175 | 13.000 | 1.00 | 22.92 | C |
| ATOM | 5078 | CD1 | LEU | D | 11 | 8.617 | -16.222 | 12.142 | 1.00 | 24.85 | C |
| ATOM | 5079 | CD2 | LEU | D | 11 | 8.690 | -15.058 | 14.320 | 1.00 | 25.45 | C |
| ATOM | 5080 | N | VAL | D | 12 | 5.648 | -15.585 | 10.705 | 1.00 | 26.08 | N |
| ATOM | 5081 | CA | VAL | D | 12 | 4.424 | -16.334 | 10.734 | 1.00 | 29.23 | C |
| ATOM | 5082 | C | VAL | D | 12 | 4.807 | -17.796 | 10.844 | 1.00 | 28.38 | C |
| ATOM | 5083 | O | VAL | D | 12 | 5.839 | -18.212 | 10.332 | 1.00 | 27.81 | O |
| ATOM | 5084 | CB | VAL | D | 12 | 3.588 | -16.022 | 9.453 | 1.00 | 30.20 | C |
| ATOM | 5085 | CG1 | VAL | D | 12 | 4.291 | -16.517 | 8.201 | 1.00 | 31.31 | C |
| ATOM | 5086 | CG2 | VAL | D | 12 | 2.225 | -16.560 | 9.546 | 1.00 | 32.25 | C |
| ATOM | 5087 | N | GLN | D | 13 | 4.006 | -18.575 | 11.566 | 1.00 | 31.68 | N |
| ATOM | 5088 | CA | GLN | D | 13 | 4.325 | -19.969 | 11.747 | 1.00 | 31.23 | C |
| ATOM | 5089 | C | GLN | D | 13 | 3.876 | -20.736 | 10.540 | 1.00 | 30.04 | C |
| ATOM | 5090 | O | GLN | D | 13 | 2.972 | -20.306 | 9.839 | 1.00 | 29.93 | O |
| ATOM | 5091 | CB | GLN | D | 13 | 3.645 | -20.563 | 12.984 | 1.00 | 33.80 | C |
| ATOM | 5092 | CG | GLN | D | 13 | 4.404 | -20.331 | 14.269 | 1.00 | 36.62 | C |
| ATOM | 5093 | CD | GLN | D | 13 | 4.168 | -21.434 | 15.264 | 1.00 | 39.31 | C |
| ATOM | 5094 | OE1 | GLN | D | 13 | 3.055 | -21.974 | 15.365 | 1.00 | 41.82 | O |
| ATOM | 5095 | NE2 | GLN | D | 13 | 5.213 | -21.789 | 16.008 | 1.00 | 42.56 | N |
| ATOM | 5096 | N | PRO | D | 14 | 4.470 | -21.903 | 10.324 | 1.00 | 28.97 | N |
| ATOM | 5097 | CA | PRO | D | 14 | 4.032 | -22.738 | 9.235 | 1.00 | 28.94 | C |
| ATOM | 5098 | C | PRO | D | 14 | 2.563 | -23.090 | 9.390 | 1.00 | 29.44 | C |
| ATOM | 5099 | O | PRO | D | 14 | 2.080 | -23.257 | 10.512 | 1.00 | 29.09 | O |
| ATOM | 5100 | CB | PRO | D | 14 | 4.922 | -23.974 | 9.341 | 1.00 | 29.67 | C |
| ATOM | 5101 | CG | PRO | D | 14 | 6.075 | -23.543 | 10.200 | 1.00 | 29.55 | C |
| ATOM | 5102 | CD | PRO | D | 14 | 5.576 | -22.500 | 11.082 | 1.00 | 29.47 | C |
| ATOM | 5103 | N | GLY | D | 15 | 1.863 | -23.137 | 8.266 | 1.00 | 28.84 | N |
| ATOM | 5104 | CA | GLY | D | 15 | 0.410 | -23.236 | 8.234 | 1.00 | 29.45 | C |
| ATOM | 5105 | C | GLY | D | 15 | -0.300 | -21.917 | 8.300 | 1.00 | 29.67 | C |
| ATOM | 5106 | O | GLY | D | 15 | -1.488 | -21.843 | 8.047 | 1.00 | 30.17 | O |
| ATOM | 5107 | N | GLY | D | 16 | 0.418 | -20.860 | 8.649 | 1.00 | 29.58 | N |
| ATOM | 5108 | CA | GLY | D | 16 | -0.195 | -19.552 | 8.822 | 1.00 | 29.70 | C |
| ATOM | 5109 | C | GLY | D | 16 | -0.525 | -18.834 | 7.522 | 1.00 | 29.40 | C |
| ATOM | 5110 | O | GLY | D | 16 | -0.285 | -19.342 | 6.453 | 1.00 | 29.12 | O |
| ATOM | 5111 | N | SER | D | 17 | -1.077 | -17.643 | 7.672 | 1.00 | 30.26 | N |
| ATOM | 5112 | CA | SER | D | 17 | -1.449 | -16.777 | 6.571 | 1.00 | 29.11 | C |
| ATOM | 5113 | C | SER | D | 17 | -0.884 | -15.397 | 6.783 | 1.00 | 28.32 | C |
| ATOM | 5114 | O | SER | D | 17 | -0.652 | -14.953 | 7.933 | 1.00 | 27.96 | O |
| ATOM | 5115 | CB | SER | D | 17 | -2.971 | -16.647 | 6.470 | 1.00 | 30.50 | C |
| ATOM | 5116 | OG | SER | D | 17 | -3.549 | -17.852 | 6.004 | 1.00 | 32.32 | O |
| ATOM | 5117 | N | MET | D | 18 | -0.720 | -14.695 | 5.671 | 1.00 | 25.70 | N |
| ATOM | 5118 | CA | MET | D | 18 | -0.216 | -13.353 | 5.694 | 1.00 | 26.57 | C |
| ATOM | 5119 | C | MET | D | 18 | -0.618 | -12.637 | 4.426 | 1.00 | 25.42 | C |
| ATOM | 5120 | O | MET | D | 18 | -0.640 | -13.228 | 3.359 | 1.00 | 25.51 | O |
| ATOM | 5121 | CB | MET | D | 18 | 1.311 | -13.426 | 5.754 | 1.00 | 27.20 | C |
| ATOM | 5122 | CG | MET | D | 18 | 1.998 | -12.088 | 5.839 | 1.00 | 29.40 | C |
| ATOM | 5123 | SD | MET | D | 18 | 3.769 | -12.279 | 6.003 | 1.00 | 30.89 | S |
| ATOM | 5124 | CE | MET | D | 18 | 3.915 | -12.345 | 7.768 | 1.00 | 32.48 | C |
| ATOM | 5125 | N | LYS | D | 19 | -0.929 | -11.364 | 4.548 | 1.00 | 26.57 | N |

```
ATOM   5126  CA   LYS D  19    -1.173 -10.533    3.383  1.00 26.42        C
ATOM   5127  C    LYS D  19    -0.146  -9.403    3.368  1.00 26.42        C
ATOM   5128  O    LYS D  19     0.042  -8.651    4.359  1.00 25.42        O
ATOM   5129  CB   LYS D  19    -2.606  -9.976    3.377  1.00 28.03        C
ATOM   5130  CG   LYS D  19    -3.049  -9.410    2.045  1.00 27.71        C
ATOM   5131  CD   LYS D  19    -4.461  -8.794    2.123  1.00 28.61        C
ATOM   5132  CE   LYS D  19    -5.045  -8.502    0.770  1.00 29.69        C
ATOM   5133  NZ   LYS D  19    -6.489  -8.122    0.819  1.00 31.17        N
ATOM   5134  N    ILE D  20     0.563  -9.312    2.248  1.00 24.48        N
ATOM   5135  CA   ILE D  20     1.540  -8.239    2.054  1.00 22.72        C
ATOM   5136  C    ILE D  20     1.065  -7.332    0.929  1.00 21.13        C
ATOM   5137  O    ILE D  20     0.199  -7.700    0.160  1.00 21.62        O
ATOM   5138  CB   ILE D  20     2.978  -8.796    1.804  1.00 21.95        C
ATOM   5139  CG1  ILE D  20     3.020  -9.666    0.542  1.00 20.05        C
ATOM   5140  CG2  ILE D  20     3.456  -9.551    3.008  1.00 22.25        C
ATOM   5141  CD1  ILE D  20     4.439 -10.103    0.128  1.00 21.08        C
ATOM   5142  N    SER D  21     1.640  -6.137    0.821  1.00 22.77        N
ATOM   5143  CA   SER D  21     1.221  -5.189   -0.217  1.00 24.26        C
ATOM   5144  C    SER D  21     2.376  -4.268   -0.617  1.00 23.39        C
ATOM   5145  O    SER D  21     3.412  -4.200    0.051  1.00 21.99        O
ATOM   5146  CB   SER D  21     0.000  -4.361    0.244  1.00 24.06        C
ATOM   5147  OG   SER D  21     0.398  -3.338    1.120  1.00 27.57        O
ATOM   5148  N    CYS D  22     2.193  -3.573   -1.728  1.00 22.76        N
ATOM   5149  CA   CYS D  22     3.087  -2.495   -2.083  1.00 24.84        C
ATOM   5150  C    CYS D  22     2.290  -1.351   -2.670  1.00 24.71        C
ATOM   5151  O    CYS D  22     1.242  -1.566   -3.232  1.00 24.43        O
ATOM   5152  CB   CYS D  22     4.165  -2.969   -3.056  1.00 24.58        C
ATOM   5153  SG   CYS D  22     3.566  -3.490   -4.611  1.00 26.39        S
ATOM   5154  N    VAL D  23     2.795  -0.139   -2.498  1.00 25.91        N
ATOM   5155  CA   VAL D  23     2.180   1.029   -3.082  1.00 26.31        C
ATOM   5156  C    VAL D  23     3.156   1.593   -4.082  1.00 24.90        C
ATOM   5157  O    VAL D  23     4.375   1.625   -3.843  1.00 25.60        O
ATOM   5158  CB   VAL D  23     1.803   2.079   -2.006  1.00 25.97        C
ATOM   5159  CG1  VAL D  23     3.024   2.579   -1.278  1.00 26.49        C
ATOM   5160  CG2  VAL D  23     0.992   3.244   -2.638  1.00 28.59        C
ATOM   5161  N    VAL D  24     2.621   2.035   -5.213  1.00 26.62        N
ATOM   5162  CA   VAL D  24     3.449   2.562   -6.271  1.00 25.89        C
ATOM   5163  C    VAL D  24     3.063   4.016   -6.464  1.00 27.17        C
ATOM   5164  O    VAL D  24     1.884   4.334   -6.508  1.00 27.59        O
ATOM   5165  CB   VAL D  24     3.271   1.771   -7.572  1.00 26.53        C
ATOM   5166  CG1  VAL D  24     4.174   2.296   -8.653  1.00 25.44        C
ATOM   5167  CG2  VAL D  24     3.575   0.305   -7.305  1.00 24.75        C
ATOM   5168  N    SER D  25     4.071   4.860   -6.582  1.00 27.55        N
ATOM   5169  CA   SER D  25     3.882   6.283   -6.842  1.00 28.60        C
ATOM   5170  C    SER D  25     4.784   6.717   -7.982  1.00 28.88        C
ATOM   5171  O    SER D  25     5.816   6.119   -8.214  1.00 30.26        O
ATOM   5172  CB   SER D  25     4.206   7.065   -5.584  1.00 28.94        C
ATOM   5173  OG   SER D  25     5.545   6.858   -5.142  1.00 29.91        O
ATOM   5174  N    GLY D  26     4.409   7.780   -8.688  1.00 28.58        N
ATOM   5175  CA   GLY D  26     5.271   8.317   -9.731  1.00 27.48        C
ATOM   5176  C    GLY D  26     4.882   7.846  -11.116  1.00 27.31        C
ATOM   5177  O    GLY D  26     5.560   8.149  -12.090  1.00 27.14        O
ATOM   5178  N    LEU D  27     3.821   7.058  -11.215  1.00 26.70        N
ATOM   5179  CA   LEU D  27     3.260   6.697  -12.513  1.00 27.90        C
ATOM   5180  C    LEU D  27     1.771   6.415  -12.335  1.00 28.39        C
ATOM   5181  O    LEU D  27     1.260   6.475  -11.203  1.00 30.45        O
ATOM   5182  CB   LEU D  27     4.015   5.518  -13.152  1.00 28.18        C
ATOM   5183  CG   LEU D  27     4.364   4.345  -12.260  1.00 28.20        C
ATOM   5184  CD1  LEU D  27     3.110   3.581  -11.871  1.00 28.46        C
ATOM   5185  CD2  LEU D  27     5.305   3.395  -12.973  1.00 29.05        C
ATOM   5186  N    THR D  28     1.052   6.161  -13.417  1.00 28.84        N
ATOM   5187  CA   THR D  28    -0.364   5.775  -13.287  1.00 29.80        C
ATOM   5188  C    THR D  28    -0.488   4.284  -13.059  1.00 29.04        C
ATOM   5189  O    THR D  28    -0.489   3.496  -14.015  1.00 28.21        O
ATOM   5190  CB   THR D  28    -1.197   6.195  -14.512  1.00 31.46        C
```

| ATOM | 5191 | OG1 | THR | D | 28 | -1.140 | 7.614 | -14.623 | 1.00 | 35.70 | O |
| ATOM | 5192 | CG2 | THR | D | 28 | -2.653 | 5.760 | -14.375 | 1.00 | 32.35 | C |
| ATOM | 5193 | N | PHE | D | 29 | -0.603 | 3.913 | -11.787 | 1.00 | 29.14 | N |
| ATOM | 5194 | CA | PHE | D | 29 | -0.534 | 2.514 | -11.366 | 1.00 | 29.13 | C |
| ATOM | 5195 | C | PHE | D | 29 | -1.449 | 1.620 | -12.193 | 1.00 | 28.57 | C |
| ATOM | 5196 | O | PHE | D | 29 | -1.068 | 0.544 | -12.658 | 1.00 | 26.92 | O |
| ATOM | 5197 | CB | PHE | D | 29 | -0.900 | 2.428 | -9.892 | 1.00 | 29.85 | C |
| ATOM | 5198 | CG | PHE | D | 29 | -0.999 | 1.035 | -9.373 | 1.00 | 29.73 | C |
| ATOM | 5199 | CD1 | PHE | D | 29 | 0.142 | 0.363 | -8.958 | 1.00 | 30.10 | C |
| ATOM | 5200 | CD2 | PHE | D | 29 | -2.236 | 0.381 | -9.326 | 1.00 | 28.49 | C |
| ATOM | 5201 | CE1 | PHE | D | 29 | 0.063 | -0.930 | -8.482 | 1.00 | 29.03 | C |
| ATOM | 5202 | CE2 | PHE | D | 29 | -2.332 | -0.915 | -8.850 | 1.00 | 29.22 | C |
| ATOM | 5203 | CZ | PHE | D | 29 | -1.177 | -1.565 | -8.412 | 1.00 | 29.95 | C |
| ATOM | 5204 | N | SER | D | 30 | -2.674 | 2.077 | -12.410 | 1.00 | 28.72 | N |
| ATOM | 5205 | CA | SER | D | 30 | -3.684 | 1.288 | -13.079 | 1.00 | 28.12 | C |
| ATOM | 5206 | C | SER | D | 30 | -3.363 | 0.869 | -14.520 | 1.00 | 27.79 | C |
| ATOM | 5207 | O | SER | D | 30 | -4.031 | 0.009 | -15.055 | 1.00 | 28.78 | O |
| ATOM | 5208 | CB | SER | D | 30 | -5.032 | 2.051 | -13.057 | 1.00 | 28.23 | C |
| ATOM | 5209 | OG | SER | D | 30 | -4.959 | 3.196 | -13.884 | 1.00 | 30.75 | O |
| ATOM | 5210 | N | ASN | D | 31 | -2.339 | 1.466 | -15.133 | 1.00 | 27.46 | N |
| ATOM | 5211 | CA | ASN | D | 31 | -1.969 | 1.154 | -16.513 | 1.00 | 26.11 | C |
| ATOM | 5212 | C | ASN | D | 31 | -0.896 | 0.100 | -16.671 | 1.00 | 24.82 | C |
| ATOM | 5213 | O | ASN | D | 31 | -0.490 | -0.187 | -17.800 | 1.00 | 24.65 | O |
| ATOM | 5214 | CB | ASN | D | 31 | -1.501 | 2.417 | -17.226 | 1.00 | 27.02 | C |
| ATOM | 5215 | CG | ASN | D | 31 | -2.637 | 3.402 | -17.463 | 1.00 | 29.08 | C |
| ATOM | 5216 | OD1 | ASN | D | 31 | -2.438 | 4.601 | -17.457 | 1.00 | 32.95 | O |
| ATOM | 5217 | ND2 | ASN | D | 31 | -3.821 | 2.889 | -17.635 | 1.00 | 28.31 | N |
| ATOM | 5218 | N | TYR | D | 32 | -0.437 | -0.464 | -15.553 | 1.00 | 23.32 | N |
| ATOM | 5219 | CA | TYR | D | 32 | 0.714 | -1.390 | -15.576 | 1.00 | 23.08 | C |
| ATOM | 5220 | C | TYR | D | 32 | 0.410 | -2.772 | -15.076 | 1.00 | 22.66 | C |
| ATOM | 5221 | O | TYR | D | 32 | -0.289 | -2.922 | -14.095 | 1.00 | 23.23 | O |
| ATOM | 5222 | CB | TYR | D | 32 | 1.867 | -0.800 | -14.765 | 1.00 | 24.44 | C |
| ATOM | 5223 | CG | TYR | D | 32 | 2.334 | 0.498 | -15.355 | 1.00 | 24.18 | C |
| ATOM | 5224 | CD1 | TYR | D | 32 | 3.304 | 0.510 | -16.354 | 1.00 | 27.21 | C |
| ATOM | 5225 | CD2 | TYR | D | 32 | 1.771 | 1.704 | -14.964 | 1.00 | 25.63 | C |
| ATOM | 5226 | CE1 | TYR | D | 32 | 3.712 | 1.696 | -16.932 | 1.00 | 26.87 | C |
| ATOM | 5227 | CE2 | TYR | D | 32 | 2.168 | 2.889 | -15.537 | 1.00 | 25.22 | C |
| ATOM | 5228 | CZ | TYR | D | 32 | 3.155 | 2.873 | -16.510 | 1.00 | 27.07 | C |
| ATOM | 5229 | OH | TYR | D | 32 | 3.581 | 4.037 | -17.100 | 1.00 | 27.75 | O |
| ATOM | 5230 | N | TRP | D | 33 | 0.933 | -3.776 | -15.763 | 1.00 | 21.45 | N |
| ATOM | 5231 | CA | TRP | D | 33 | 0.992 | -5.145 | -15.224 | 1.00 | 22.99 | C |
| ATOM | 5232 | C | TRP | D | 33 | 1.934 | -5.137 | -14.024 | 1.00 | 22.33 | C |
| ATOM | 5233 | O | TRP | D | 33 | 2.933 | -4.414 | -14.005 | 1.00 | 21.37 | O |
| ATOM | 5234 | CB | TRP | D | 33 | 1.521 | -6.199 | -16.209 | 1.00 | 23.74 | C |
| ATOM | 5235 | CG | TRP | D | 33 | 0.805 | -6.370 | -17.526 | 1.00 | 25.19 | C |
| ATOM | 5236 | CD1 | TRP | D | 33 | -0.329 | -5.716 | -17.935 | 1.00 | 25.79 | C |
| ATOM | 5237 | CD2 | TRP | D | 33 | 1.149 | -7.272 | -18.586 | 1.00 | 26.20 | C |
| ATOM | 5238 | NE1 | TRP | D | 33 | -0.670 | -6.122 | -19.191 | 1.00 | 26.58 | N |
| ATOM | 5239 | CE2 | TRP | D | 33 | 0.197 | -7.090 | -19.611 | 1.00 | 26.74 | C |
| ATOM | 5240 | CE3 | TRP | D | 33 | 2.163 | -8.217 | -18.775 | 1.00 | 28.59 | C |
| ATOM | 5241 | CZ2 | TRP | D | 33 | 0.256 | -7.780 | -20.815 | 1.00 | 25.48 | C |
| ATOM | 5242 | CZ3 | TRP | D | 33 | 2.198 | -8.931 | -19.964 | 1.00 | 28.50 | C |
| ATOM | 5243 | CH2 | TRP | D | 33 | 1.251 | -8.702 | -20.975 | 1.00 | 27.78 | C |
| ATOM | 5244 | N | MET | D | 34 | 1.608 | -5.941 | -13.024 | 1.00 | 20.68 | N |
| ATOM | 5245 | CA | MET | D | 34 | 2.319 | -5.916 | -11.742 | 1.00 | 22.11 | C |
| ATOM | 5246 | C | MET | D | 34 | 2.587 | -7.355 | -11.345 | 1.00 | 20.75 | C |
| ATOM | 5247 | O | MET | D | 34 | 1.771 | -8.208 | -11.589 | 1.00 | 21.85 | O |
| ATOM | 5248 | CB | MET | D | 34 | 1.442 | -5.299 | -10.653 | 1.00 | 24.90 | C |
| ATOM | 5249 | CG | MET | D | 34 | 1.289 | -3.795 | -10.723 | 1.00 | 26.22 | C |
| ATOM | 5250 | SD | MET | D | 34 | 2.817 | -2.910 | -10.567 | 1.00 | 33.16 | S |
| ATOM | 5251 | CE | MET | D | 34 | 3.318 | -3.450 | -8.936 | 1.00 | 33.05 | C |
| ATOM | 5252 | N | SER | D | 35 | 3.704 | -7.602 | -10.691 | 1.00 | 19.48 | N |
| ATOM | 5253 | CA | SER | D | 35 | 4.034 | -8.957 | -10.255 | 1.00 | 19.58 | C |
| ATOM | 5254 | C | SER | D | 35 | 4.707 | -8.970 | -8.880 | 1.00 | 18.51 | C |
| ATOM | 5255 | O | SER | D | 35 | 5.216 | -7.975 | -8.388 | 1.00 | 18.06 | O |

```
ATOM   5256  CB   SER D  35     4.916  -9.661 -11.275  1.00 21.75           C
ATOM   5257  OG   SER D  35     6.279  -9.283 -11.185  1.00 22.02           O
ATOM   5258  N    TRP D  36     4.715 -10.151  -8.286  1.00 17.17           N
ATOM   5259  CA   TRP D  36     5.500 -10.419  -7.118  1.00 16.34           C
ATOM   5260  C    TRP D  36     6.595 -11.410  -7.481  1.00 17.32           C
ATOM   5261  O    TRP D  36     6.341 -12.408  -8.116  1.00 17.57           O
ATOM   5262  CB   TRP D  36     4.633 -11.027  -6.009  1.00 17.55           C
ATOM   5263  CG   TRP D  36     3.680 -10.092  -5.391  1.00 17.36           C
ATOM   5264  CD1  TRP D  36     2.343  -9.962  -5.673  1.00 18.94           C
ATOM   5265  CD2  TRP D  36     3.961  -9.168  -4.351  1.00 18.89           C
ATOM   5266  NE1  TRP D  36     1.781  -8.992  -4.882  1.00 20.46           N
ATOM   5267  CE2  TRP D  36     2.750  -8.489  -4.055  1.00 17.03           C
ATOM   5268  CE3  TRP D  36     5.116  -8.863  -3.602  1.00 18.11           C
ATOM   5269  CZ2  TRP D  36     2.679  -7.477  -3.083  1.00 18.71           C
ATOM   5270  CZ3  TRP D  36     5.030  -7.861  -2.647  1.00 18.12           C
ATOM   5271  CH2  TRP D  36     3.803  -7.224  -2.370  1.00 18.48           C
ATOM   5272  N    VAL D  37     7.803 -11.083  -7.042  1.00 16.81           N
ATOM   5273  CA   VAL D  37     9.012 -11.892  -7.191  1.00 15.87           C
ATOM   5274  C    VAL D  37     9.655 -11.998  -5.817  1.00 16.60           C
ATOM   5275  O    VAL D  37     9.915 -10.987  -5.184  1.00 18.91           O
ATOM   5276  CB   VAL D  37    10.026 -11.242  -8.171  1.00 16.40           C
ATOM   5277  CG1  VAL D  37    11.281 -12.100  -8.219  1.00 18.04           C
ATOM   5278  CG2  VAL D  37     9.436 -11.120  -9.559  1.00 17.64           C
ATOM   5279  N    ARG D  38     9.915 -13.213  -5.364  1.00 17.47           N
ATOM   5280  CA   ARG D  38    10.535 -13.399  -4.070  1.00 19.01           C
ATOM   5281  C    ARG D  38    11.963 -13.859  -4.216  1.00 19.93           C
ATOM   5282  O    ARG D  38    12.342 -14.449  -5.221  1.00 21.05           O
ATOM   5283  CB   ARG D  38     9.749 -14.377  -3.232  1.00 18.86           C
ATOM   5284  CG   ARG D  38     9.772 -15.774  -3.742  1.00 18.12           C
ATOM   5285  CD   ARG D  38     8.748 -16.565  -2.975  1.00 19.56           C
ATOM   5286  NE   ARG D  38     8.714 -17.959  -3.411  1.00 19.94           N
ATOM   5287  CZ   ARG D  38     7.934 -18.885  -2.886  1.00 22.03           C
ATOM   5288  NH1  ARG D  38     7.090 -18.603  -1.919  1.00 21.32           N
ATOM   5289  NH2  ARG D  38     8.001 -20.113  -3.357  1.00 20.98           N
ATOM   5290  N    GLN D  39    12.758 -13.588  -3.199  1.00 20.66           N
ATOM   5291  CA   GLN D  39    14.200 -13.878  -3.201  1.00 21.49           C
ATOM   5292  C    GLN D  39    14.631 -14.634  -1.950  1.00 22.44           C
ATOM   5293  O    GLN D  39    14.393 -14.178  -0.829  1.00 20.27           O
ATOM   5294  CB   GLN D  39    14.967 -12.556  -3.272  1.00 20.07           C
ATOM   5295  CG   GLN D  39    16.471 -12.655  -3.434  1.00 21.74           C
ATOM   5296  CD   GLN D  39    17.131 -11.270  -3.529  1.00 22.62           C
ATOM   5297  OE1  GLN D  39    16.882 -10.396  -2.707  1.00 25.29           O
ATOM   5298  NE2  GLN D  39    17.982 -11.096  -4.515  1.00 26.72           N
ATOM   5299  N    SER D  40    15.316 -15.763  -2.156  1.00 25.27           N
ATOM   5300  CA   SER D  40    15.892 -16.545  -1.053  1.00 26.46           C
ATOM   5301  C    SER D  40    17.312 -17.026  -1.372  1.00 28.74           C
ATOM   5302  O    SER D  40    17.678 -17.122  -2.547  1.00 28.83           O
ATOM   5303  CB   SER D  40    14.991 -17.736  -0.788  1.00 26.90           C
ATOM   5304  OG   SER D  40    15.007 -18.594  -1.898  1.00 27.98           O
ATOM   5305  N    PRO D  41    18.143 -17.305  -0.336  1.00 31.45           N
ATOM   5306  CA   PRO D  41    19.463 -17.908  -0.623  1.00 33.51           C
ATOM   5307  C    PRO D  41    19.397 -19.184  -1.459  1.00 34.90           C
ATOM   5308  O    PRO D  41    20.155 -19.347  -2.405  1.00 36.25           O
ATOM   5309  CB   PRO D  41    20.007 -18.234   0.771  1.00 33.40           C
ATOM   5310  CG   PRO D  41    19.387 -17.199   1.671  1.00 32.81           C
ATOM   5311  CD   PRO D  41    17.976 -17.043   1.109  1.00 32.32           C
ATOM   5312  N    GLU D  42    18.457 -20.056  -1.147  1.00 37.18           N
ATOM   5313  CA   GLU D  42    18.437 -21.384  -1.766  1.00 39.00           C
ATOM   5314  C    GLU D  42    17.824 -21.392  -3.179  1.00 38.53           C
ATOM   5315  O    GLU D  42    18.180 -22.233  -4.014  1.00 39.36           O
ATOM   5316  CB   GLU D  42    17.795 -22.447  -0.836  1.00 40.83           C
ATOM   5317  CG   GLU D  42    16.755 -21.960   0.196  1.00 43.50           C
ATOM   5318  CD   GLU D  42    17.310 -21.057   1.304  1.00 45.46           C
ATOM   5319  OE1  GLU D  42    18.316 -21.403   1.971  1.00 47.79           O
ATOM   5320  OE2  GLU D  42    16.725 -19.980   1.511  1.00 46.23           O
```

| ATOM | 5321 | N | LYS | D | 43 | 16.927 | -20.454 | -3.473 | 1.00 | 37.13 | N |
| ATOM | 5322 | CA | LYS | D | 43 | 16.252 | -20.445 | -4.776 | 1.00 | 35.05 | C |
| ATOM | 5323 | C | LYS | D | 43 | 16.548 | -19.207 | -5.624 | 1.00 | 32.74 | C |
| ATOM | 5324 | O | LYS | D | 43 | 16.186 | -19.176 | -6.785 | 1.00 | 32.92 | O |
| ATOM | 5325 | CB | LYS | D | 43 | 14.733 | -20.595 | -4.582 | 1.00 | 36.86 | C |
| ATOM | 5326 | CG | LYS | D | 43 | 14.216 | -22.041 | -4.477 | 1.00 | 40.27 | C |
| ATOM | 5327 | CD | LYS | D | 43 | 13.497 | -22.486 | -5.776 | 1.00 | 41.88 | C |
| ATOM | 5328 | CE | LYS | D | 43 | 13.353 | -24.015 | -5.889 | 1.00 | 43.20 | C |
| ATOM | 5329 | NZ | LYS | D | 43 | 14.032 | -24.552 | -7.128 | 1.00 | 44.85 | N |
| ATOM | 5330 | N | GLY | D | 44 | 17.184 | -18.190 | -5.056 | 1.00 | 30.34 | N |
| ATOM | 5331 | CA | GLY | D | 44 | 17.427 | -16.929 | -5.792 | 1.00 | 28.20 | C |
| ATOM | 5332 | C | GLY | D | 44 | 16.148 | -16.151 | -6.051 | 1.00 | 26.62 | C |
| ATOM | 5333 | O | GLY | D | 44 | 15.212 | -16.193 | -5.250 | 1.00 | 24.69 | O |
| ATOM | 5334 | N | LEU | D | 45 | 16.099 | -15.450 | -7.186 | 1.00 | 24.62 | N |
| ATOM | 5335 | CA | LEU | D | 45 | 14.898 | -14.740 | -7.612 | 1.00 | 23.85 | C |
| ATOM | 5336 | C | LEU | D | 45 | 13.893 | -15.670 | -8.225 | 1.00 | 24.22 | C |
| ATOM | 5337 | O | LEU | D | 45 | 14.218 | -16.438 | -9.150 | 1.00 | 22.96 | O |
| ATOM | 5338 | CB | LEU | D | 45 | 15.244 | -13.668 | -8.637 | 1.00 | 24.07 | C |
| ATOM | 5339 | CG | LEU | D | 45 | 16.048 | -12.498 | -8.150 | 1.00 | 23.78 | C |
| ATOM | 5340 | CD1 | LEU | D | 45 | 16.491 | -11.679 | -9.367 | 1.00 | 22.34 | C |
| ATOM | 5341 | CD2 | LEU | D | 45 | 15.273 | -11.653 | -7.135 | 1.00 | 22.69 | C |
| ATOM | 5342 | N | GLU | D | 46 | 12.673 | -15.577 | -7.723 | 1.00 | 21.78 | N |
| ATOM | 5343 | CA | GLU | D | 46 | 11.588 | -16.496 | -8.083 | 1.00 | 22.03 | C |
| ATOM | 5344 | C | GLU | D | 46 | 10.271 | -15.748 | -8.289 | 1.00 | 19.82 | C |
| ATOM | 5345 | O | GLU | D | 46 | 9.655 | -15.259 | -7.344 | 1.00 | 21.41 | O |
| ATOM | 5346 | CB | GLU | D | 46 | 11.413 | -17.516 | -6.971 | 1.00 | 23.08 | C |
| ATOM | 5347 | CG | GLU | D | 46 | 10.559 | -18.674 | -7.316 | 1.00 | 26.71 | C |
| ATOM | 5348 | CD | GLU | D | 46 | 10.224 | -19.495 | -6.086 | 1.00 | 29.44 | C |
| ATOM | 5349 | OE1 | GLU | D | 46 | 10.886 | -19.298 | -5.040 | 1.00 | 30.75 | O |
| ATOM | 5350 | OE2 | GLU | D | 46 | 9.312 | -20.337 | -6.181 | 1.00 | 33.51 | O |
| ATOM | 5351 | N | TRP | D | 47 | 9.846 | -15.641 | -9.533 | 1.00 | 19.65 | N |
| ATOM | 5352 | CA | TRP | D | 47 | 8.549 | -15.060 | -9.860 | 1.00 | 21.50 | C |
| ATOM | 5353 | C | TRP | D | 47 | 7.420 | -15.921 | -9.315 | 1.00 | 22.02 | C |
| ATOM | 5354 | O | TRP | D | 47 | 7.435 | -17.154 | -9.471 | 1.00 | 21.21 | O |
| ATOM | 5355 | CB | TRP | D | 47 | 8.436 | -14.890 | -11.387 | 1.00 | 22.30 | C |
| ATOM | 5356 | CG | TRP | D | 47 | 7.142 | -14.406 | -11.908 | 1.00 | 20.28 | C |
| ATOM | 5357 | CD1 | TRP | D | 47 | 6.760 | -13.118 | -12.094 | 1.00 | 19.24 | C |
| ATOM | 5358 | CD2 | TRP | D | 47 | 6.085 | -15.211 | -12.412 | 1.00 | 20.78 | C |
| ATOM | 5359 | NE1 | TRP | D | 47 | 5.504 | -13.065 | -12.637 | 1.00 | 21.49 | N |
| ATOM | 5360 | CE2 | TRP | D | 47 | 5.076 | -14.348 | -12.869 | 1.00 | 20.97 | C |
| ATOM | 5361 | CE3 | TRP | D | 47 | 5.897 | -16.584 | -12.529 | 1.00 | 22.47 | C |
| ATOM | 5362 | CZ2 | TRP | D | 47 | 3.871 | -14.824 | -13.403 | 1.00 | 22.65 | C |
| ATOM | 5363 | CZ3 | TRP | D | 47 | 4.721 | -17.046 | -13.072 | 1.00 | 22.89 | C |
| ATOM | 5364 | CH2 | TRP | D | 47 | 3.735 | -16.191 | -13.498 | 1.00 | 21.47 | C |
| ATOM | 5365 | N | VAL | D | 48 | 6.439 | -15.297 | -8.670 | 1.00 | 21.27 | N |
| ATOM | 5366 | CA | VAL | D | 48 | 5.333 | -16.086 | -8.089 | 1.00 | 21.85 | C |
| ATOM | 5367 | C | VAL | D | 48 | 3.930 | -15.748 | -8.573 | 1.00 | 20.75 | C |
| ATOM | 5368 | O | VAL | D | 48 | 3.108 | -16.621 | -8.630 | 1.00 | 24.24 | O |
| ATOM | 5369 | CB | VAL | D | 48 | 5.385 | -16.156 | -6.545 | 1.00 | 20.86 | C |
| ATOM | 5370 | CG1 | VAL | D | 48 | 6.630 | -16.897 | -6.115 | 1.00 | 23.00 | C |
| ATOM | 5371 | CG2 | VAL | D | 48 | 5.321 | -14.791 | -5.900 | 1.00 | 22.36 | C |
| ATOM | 5372 | N | ALA | D | 49 | 3.654 | -14.498 | -8.928 | 1.00 | 20.42 | N |
| ATOM | 5373 | CA | ALA | D | 49 | 2.308 | -14.091 | -9.257 | 1.00 | 20.45 | C |
| ATOM | 5374 | C | ALA | D | 49 | 2.307 | -12.811 | -10.078 | 1.00 | 21.74 | C |
| ATOM | 5375 | O | ALA | D | 49 | 3.165 | -11.942 | -9.884 | 1.00 | 20.94 | O |
| ATOM | 5376 | CB | ALA | D | 49 | 1.504 | -13.899 | -8.005 | 1.00 | 21.73 | C |
| ATOM | 5377 | N | GLU | D | 50 | 1.356 | -12.713 | -11.017 | 1.00 | 21.93 | N |
| ATOM | 5378 | CA | GLU | D | 50 | 1.210 | -11.508 | -11.836 | 1.00 | 21.55 | C |
| ATOM | 5379 | C | GLU | D | 50 | -0.268 | -11.195 | -12.111 | 1.00 | 22.26 | C |
| ATOM | 5380 | O | GLU | D | 50 | -1.103 | -12.100 | -12.190 | 1.00 | 24.01 | O |
| ATOM | 5381 | CB | GLU | D | 50 | 1.973 | -11.684 | -13.131 | 1.00 | 21.88 | C |
| ATOM | 5382 | CG | GLU | D | 50 | 2.161 | -10.449 | -13.977 | 1.00 | 20.85 | C |
| ATOM | 5383 | CD | GLU | D | 50 | 3.309 | -10.604 | -14.930 | 1.00 | 22.39 | C |
| ATOM | 5384 | OE1 | GLU | D | 50 | 4.420 | -10.880 | -14.452 | 1.00 | 21.86 | O |
| ATOM | 5385 | OE2 | GLU | D | 50 | 3.140 | -10.444 | -16.156 | 1.00 | 23.32 | O |

| ATOM | 5386 | N | ILE | D | 51 | -0.572 | -9.903 | -12.203 | 1.00 | 22.80 | N |
| ATOM | 5387 | CA | ILE | D | 51 | -1.922 | -9.403 | -12.503 | 1.00 | 23.62 | C |
| ATOM | 5388 | C | ILE | D | 51 | -1.857 | -8.351 | -13.621 | 1.00 | 24.85 | C |
| ATOM | 5389 | O | ILE | D | 51 | -0.959 | -7.497 | -13.649 | 1.00 | 24.85 | O |
| ATOM | 5390 | CB | ILE | D | 51 | -2.636 | -8.867 | -11.231 | 1.00 | 23.67 | C |
| ATOM | 5391 | CG1 | ILE | D | 51 | -4.135 | -8.646 | -11.487 | 1.00 | 23.29 | C |
| ATOM | 5392 | CG2 | ILE | D | 51 | -1.920 | -7.650 | -10.648 | 1.00 | 23.31 | C |
| ATOM | 5393 | CD1 | ILE | D | 51 | -4.930 | -8.392 | -10.223 | 1.00 | 25.52 | C |
| ATOM | 5394 | N | ARG | D | 52 | -2.815 | -8.426 | -14.547 | 1.00 | 25.38 | N |
| ATOM | 5395 | CA | ARG | D | 52 | -2.910 | -7.474 | -15.651 | 1.00 | 26.21 | C |
| ATOM | 5396 | C | ARG | D | 52 | -3.930 | -6.380 | -15.308 | 1.00 | 26.44 | C |
| ATOM | 5397 | O | ARG | D | 52 | -4.138 | -6.109 | -14.154 | 1.00 | 24.54 | O |
| ATOM | 5398 | CB | ARG | D | 52 | -3.212 | -8.217 | -16.950 | 1.00 | 27.01 | C |
| ATOM | 5399 | CG | ARG | D | 52 | -2.095 | -9.160 | -17.308 | 1.00 | 27.65 | C |
| ATOM | 5400 | CD | ARG | D | 52 | -2.171 | -9.662 | -18.720 | 1.00 | 28.48 | C |
| ATOM | 5401 | NE | ARG | D | 52 | -1.099 | -10.611 | -19.002 | 1.00 | 29.45 | N |
| ATOM | 5402 | CZ | ARG | D | 52 | -0.962 | -11.266 | -20.147 | 1.00 | 31.44 | C |
| ATOM | 5403 | NH1 | ARG | D | 52 | -1.826 | -11.066 | -21.131 | 1.00 | 34.51 | N |
| ATOM | 5404 | NH2 | ARG | D | 52 | 0.028 | -12.122 | -20.318 | 1.00 | 32.16 | N |
| ATOM | 5405 | N | LEU | D | 52A | -4.547 | -5.740 | -16.304 | 1.00 | 28.93 | N |
| ATOM | 5406 | CA | LEU | D | 52A | -5.306 | -4.499 | -16.069 | 1.00 | 29.93 | C |
| ATOM | 5407 | C | LEU | D | 52A | -6.817 | -4.722 | -15.937 | 1.00 | 31.29 | C |
| ATOM | 5408 | O | LEU | D | 52A | -7.314 | -5.786 | -16.252 | 1.00 | 30.74 | O |
| ATOM | 5409 | CB | LEU | D | 52A | -5.057 | -3.532 | -17.229 | 1.00 | 29.61 | C |
| ATOM | 5410 | CG | LEU | D | 52A | -3.589 | -3.415 | -17.653 | 1.00 | 29.54 | C |
| ATOM | 5411 | CD1 | LEU | D | 52A | -3.419 | -2.460 | -18.814 | 1.00 | 29.07 | C |
| ATOM | 5412 | CD2 | LEU | D | 52A | -2.729 | -2.980 | -16.459 | 1.00 | 28.71 | C |
| ATOM | 5413 | N | LYS | D | 52B | -7.537 | -3.682 | -15.516 | 1.00 | 33.85 | N |
| ATOM | 5414 | CA | LYS | D | 52B | -9.025 | -3.705 | -15.501 | 1.00 | 35.99 | C |
| ATOM | 5415 | C | LYS | D | 52B | -9.586 | -4.156 | -16.842 | 1.00 | 36.61 | C |
| ATOM | 5416 | O | LYS | D | 52B | -10.504 | -4.986 | -16.905 | 1.00 | 38.15 | O |
| ATOM | 5417 | CB | LYS | D | 52B | -9.591 | -2.320 | -15.174 | 1.00 | 36.35 | C |
| ATOM | 5418 | CG | LYS | D | 52B | -11.132 | -2.212 | -15.385 | 1.00 | 38.01 | C |
| ATOM | 5419 | CD | LYS | D | 52B | -11.702 | -0.939 | -14.750 | 1.00 | 39.18 | C |
| ATOM | 5420 | CE | LYS | D | 52B | -13.090 | -0.593 | -15.306 | 1.00 | 40.58 | C |
| ATOM | 5421 | NZ | LYS | D | 52B | -13.839 | -1.775 | -15.871 | 1.00 | 41.59 | N |
| ATOM | 5422 | N | SER | D | 52C | -9.020 | -3.612 | -17.911 | 1.00 | 37.16 | N |
| ATOM | 5423 | CA | SER | D | 52C | -9.435 | -3.934 | -19.274 | 1.00 | 37.57 | C |
| ATOM | 5424 | C | SER | D | 52C | -9.117 | -5.366 | -19.686 | 1.00 | 38.04 | C |
| ATOM | 5425 | O | SER | D | 52C | -9.630 | -5.861 | -20.692 | 1.00 | 38.28 | O |
| ATOM | 5426 | CB | SER | D | 52C | -8.778 | -2.978 | -20.255 | 1.00 | 38.11 | C |
| ATOM | 5427 | OG | SER | D | 52C | -7.378 | -3.188 | -20.311 | 1.00 | 38.81 | O |
| ATOM | 5428 | N | ASP | D | 53 | -8.251 | -6.028 | -18.927 | 1.00 | 37.33 | N |
| ATOM | 5429 | CA | ASP | D | 53 | -7.992 | -7.448 | -19.126 | 1.00 | 37.21 | C |
| ATOM | 5430 | C | ASP | D | 53 | -8.816 | -8.227 | -18.117 | 1.00 | 36.54 | C |
| ATOM | 5431 | O | ASP | D | 53 | -8.600 | -9.410 | -17.909 | 1.00 | 38.40 | O |
| ATOM | 5432 | CB | ASP | D | 53 | -6.498 | -7.754 | -18.920 | 1.00 | 36.78 | C |
| ATOM | 5433 | CG | ASP | D | 53 | -5.601 | -6.847 | -19.737 | 1.00 | 37.64 | C |
| ATOM | 5434 | OD1 | ASP | D | 53 | -5.832 | -6.742 | -20.957 | 1.00 | 38.30 | O |
| ATOM | 5435 | OD2 | ASP | D | 53 | -4.692 | -6.218 | -19.160 | 1.00 | 34.77 | O |
| ATOM | 5436 | N | ASN | D | 54 | -9.743 | -7.542 | -17.465 | 1.00 | 37.50 | N |
| ATOM | 5437 | CA | ASN | D | 54 | -10.508 | -8.117 | -16.369 | 1.00 | 37.92 | C |
| ATOM | 5438 | C | ASN | D | 54 | -9.628 | -8.668 | -15.259 | 1.00 | 36.25 | C |
| ATOM | 5439 | O | ASN | D | 54 | -9.893 | -9.730 | -14.670 | 1.00 | 34.69 | O |
| ATOM | 5440 | CB | ASN | D | 54 | -11.468 | -9.176 | -16.889 | 1.00 | 39.61 | C |
| ATOM | 5441 | CG | ASN | D | 54 | -12.814 | -8.985 | -16.335 | 1.00 | 42.12 | C |
| ATOM | 5442 | OD1 | ASN | D | 54 | -13.094 | -9.396 | -15.205 | 1.00 | 45.59 | O |
| ATOM | 5443 | ND2 | ASN | D | 54 | -13.660 | -8.283 | -17.089 | 1.00 | 44.48 | N |
| ATOM | 5444 | N | TYR | D | 55 | -8.565 | -7.927 | -14.980 | 1.00 | 34.77 | N |
| ATOM | 5445 | CA | TYR | D | 55 | -7.682 | -8.262 | -13.890 | 1.00 | 33.99 | C |
| ATOM | 5446 | C | TYR | D | 55 | -7.154 | -9.704 | -13.962 | 1.00 | 31.74 | C |
| ATOM | 5447 | O | TYR | D | 55 | -7.044 | -10.386 | -12.940 | 1.00 | 31.69 | O |
| ATOM | 5448 | CB | TYR | D | 55 | -8.414 | -8.015 | -12.573 | 1.00 | 34.63 | C |
| ATOM | 5449 | CG | TYR | D | 55 | -8.770 | -6.567 | -12.310 | 1.00 | 36.61 | C |
| ATOM | 5450 | CD1 | TYR | D | 55 | -7.775 | -5.608 | -12.168 | 1.00 | 38.87 | C |

```
ATOM   5451  CD2 TYR D  55    -10.088   -6.158  -12.168  1.00 38.18          C
ATOM   5452  CE1 TYR D  55     -8.078   -4.275  -11.903  1.00 38.91          C
ATOM   5453  CE2 TYR D  55    -10.400   -4.813  -11.894  1.00 39.33          C
ATOM   5454  CZ  TYR D  55     -9.385   -3.886  -11.762  1.00 39.62          C
ATOM   5455  OH  TYR D  55     -9.668   -2.561  -11.497  1.00 42.93          O
ATOM   5456  N   ALA D  56     -6.798  -10.151  -15.161  1.00 30.69          N
ATOM   5457  CA  ALA D  56     -6.263  -11.502  -15.383  1.00 29.26          C
ATOM   5458  C   ALA D  56     -5.045  -11.751  -14.496  1.00 29.55          C
ATOM   5459  O   ALA D  56     -4.171  -10.904  -14.417  1.00 27.07          O
ATOM   5460  CB  ALA D  56     -5.861  -11.675  -16.806  1.00 29.51          C
ATOM   5461  N   THR D  57     -5.006  -12.920  -13.861  1.00 28.66          N
ATOM   5462  CA  THR D  57     -3.966  -13.248  -12.894  1.00 27.28          C
ATOM   5463  C   THR D  57     -3.224  -14.485  -13.403  1.00 27.34          C
ATOM   5464  O   THR D  57     -3.728  -15.227  -14.268  1.00 25.76          O
ATOM   5465  CB  THR D  57     -4.535  -13.517  -11.481  1.00 26.52          C
ATOM   5466  OG1 THR D  57     -5.423  -14.645  -11.524  1.00 28.97          O
ATOM   5467  CG2 THR D  57     -5.278  -12.293  -10.917  1.00 28.20          C
ATOM   5468  N   TYR D  58     -2.000  -14.655  -12.898  1.00 26.52          N
ATOM   5469  CA  TYR D  58     -1.059  -15.672  -13.323  1.00 25.90          C
ATOM   5470  C   TYR D  58     -0.206  -16.040  -12.102  1.00 26.14          C
ATOM   5471  O   TYR D  58      0.152  -15.157  -11.305  1.00 24.56          O
ATOM   5472  CB  TYR D  58     -0.154  -15.151  -14.443  1.00 27.08          C
ATOM   5473  CG  TYR D  58     -0.919  -14.638  -15.640  1.00 27.23          C
ATOM   5474  CD1 TYR D  58     -1.440  -13.368  -15.635  1.00 28.25          C
ATOM   5475  CD2 TYR D  58     -1.171  -15.455  -16.754  1.00 28.89          C
ATOM   5476  CE1 TYR D  58     -2.177  -12.881  -16.704  1.00 28.55          C
ATOM   5477  CE2 TYR D  58     -1.899  -14.954  -17.857  1.00 29.36          C
ATOM   5478  CZ  TYR D  58     -2.390  -13.668  -17.809  1.00 28.54          C
ATOM   5479  OH  TYR D  58     -3.123  -13.118  -18.851  1.00 28.97          O
ATOM   5480  N   TYR D  59      0.097  -17.329  -11.957  1.00 25.36          N
ATOM   5481  CA  TYR D  59      0.843  -17.847  -10.805  1.00 25.27          C
ATOM   5482  C   TYR D  59      1.897  -18.869  -11.163  1.00 25.21          C
ATOM   5483  O   TYR D  59      1.736  -19.660  -12.085  1.00 26.26          O
ATOM   5484  CB  TYR D  59     -0.107  -18.502   -9.770  1.00 25.31          C
ATOM   5485  CG  TYR D  59     -1.145  -17.578   -9.232  1.00 25.80          C
ATOM   5486  CD1 TYR D  59     -0.882  -16.781   -8.137  1.00 26.05          C
ATOM   5487  CD2 TYR D  59     -2.407  -17.480   -9.834  1.00 27.41          C
ATOM   5488  CE1 TYR D  59     -1.839  -15.906   -7.635  1.00 26.13          C
ATOM   5489  CE2 TYR D  59     -3.354  -16.608   -9.355  1.00 26.36          C
ATOM   5490  CZ  TYR D  59     -3.070  -15.835   -8.248  1.00 25.95          C
ATOM   5491  OH  TYR D  59     -4.005  -14.969   -7.768  1.00 25.05          O
ATOM   5492  N   ALA D  60      2.982  -18.881  -10.397  1.00 27.02          N
ATOM   5493  CA  ALA D  60      3.942  -19.962  -10.477  1.00 28.40          C
ATOM   5494  C   ALA D  60      3.237  -21.217  -10.001  1.00 29.36          C
ATOM   5495  O   ALA D  60      2.434  -21.162   -9.079  1.00 29.19          O
ATOM   5496  CB  ALA D  60      5.138  -19.686   -9.610  1.00 29.18          C
ATOM   5497  N   GLU D  61      3.519  -22.340  -10.642  1.00 32.23          N
ATOM   5498  CA  GLU D  61      2.810  -23.579  -10.316  1.00 34.84          C
ATOM   5499  C   GLU D  61      2.991  -23.910   -8.814  1.00 35.41          C
ATOM   5500  O   GLU D  61      2.048  -24.388   -8.160  1.00 35.52          O
ATOM   5501  CB  GLU D  61      3.271  -24.723  -11.231  1.00 36.25          C
ATOM   5502  CG  GLU D  61      2.266  -25.880  -11.402  1.00 40.00          C
ATOM   5503  CD  GLU D  61      1.006  -25.535  -12.200  1.00 41.73          C
ATOM   5504  OE1 GLU D  61      1.011  -24.581  -13.020  1.00 42.03          O
ATOM   5505  OE2 GLU D  61     -0.012  -26.237  -11.988  1.00 46.42          O
ATOM   5506  N   SER D  62      4.176  -23.600   -8.273  1.00 35.14          N
ATOM   5507  CA  SER D  62      4.516  -23.873   -6.871  1.00 34.93          C
ATOM   5508  C   SER D  62      3.667  -23.159   -5.824  1.00 34.46          C
ATOM   5509  O   SER D  62      3.674  -23.558   -4.649  1.00 35.35          O
ATOM   5510  CB  SER D  62      5.984  -23.567   -6.600  1.00 35.01          C
ATOM   5511  OG  SER D  62      6.273  -22.171   -6.617  1.00 36.98          O
ATOM   5512  N   VAL D  63      2.940  -22.120   -6.220  1.00 32.91          N
ATOM   5513  CA  VAL D  63      2.133  -21.366   -5.270  1.00 31.92          C
ATOM   5514  C   VAL D  63      0.660  -21.318   -5.655  1.00 31.00          C
ATOM   5515  O   VAL D  63     -0.128  -20.689   -4.973  1.00 28.18          O
```

```
ATOM   5516  CB   VAL D  63      2.672 -19.911  -5.061  1.00 31.42           C
ATOM   5517  CG1  VAL D  63      4.139 -19.933  -4.668  1.00 32.27           C
ATOM   5518  CG2  VAL D  63      2.452 -19.072  -6.294  1.00 32.11           C
ATOM   5519  N    LYS D  64      0.292 -21.981  -6.743  1.00 33.00           N
ATOM   5520  CA   LYS D  64     -1.105 -22.053  -7.142  1.00 35.20           C
ATOM   5521  C    LYS D  64     -1.947 -22.588  -6.008  1.00 34.98           C
ATOM   5522  O    LYS D  64     -1.610 -23.598  -5.405  1.00 35.66           O
ATOM   5523  CB   LYS D  64     -1.270 -22.934  -8.379  1.00 36.89           C
ATOM   5524  CG   LYS D  64     -1.256 -22.117  -9.619  1.00 38.83           C
ATOM   5525  CD   LYS D  64     -1.039 -22.916 -10.869  1.00 40.62           C
ATOM   5526  CE   LYS D  64     -0.385 -22.052 -11.935  1.00 41.55           C
ATOM   5527  NZ   LYS D  64     -0.633 -22.627 -13.277  1.00 44.18           N
ATOM   5528  N    GLY D  65     -3.038 -21.898  -5.710  1.00 35.93           N
ATOM   5529  CA   GLY D  65     -3.939 -22.330  -4.666  1.00 35.33           C
ATOM   5530  C    GLY D  65     -3.526 -22.011  -3.250  1.00 35.74           C
ATOM   5531  O    GLY D  65     -4.294 -22.296  -2.321  1.00 38.42           O
ATOM   5532  N    LYS D  66     -2.324 -21.461  -3.063  1.00 33.18           N
ATOM   5533  CA   LYS D  66     -1.898 -20.889  -1.770  1.00 31.68           C
ATOM   5534  C    LYS D  66     -1.913 -19.362  -1.791  1.00 28.46           C
ATOM   5535  O    LYS D  66     -2.178 -18.717  -0.772  1.00 28.83           O
ATOM   5536  CB   LYS D  66     -0.481 -21.317  -1.401  1.00 32.62           C
ATOM   5537  CG   LYS D  66     -0.276 -22.791  -1.138  1.00 34.99           C
ATOM   5538  CD   LYS D  66      0.838 -23.038  -0.110  1.00 35.55           C
ATOM   5539  CE   LYS D  66      2.211 -22.551  -0.580  1.00 35.28           C
ATOM   5540  NZ   LYS D  66      3.376 -23.088   0.224  1.00 33.70           N
ATOM   5541  N    PHE D  67     -1.542 -18.799  -2.932  1.00 27.17           N
ATOM   5542  CA   PHE D  67     -1.341 -17.363  -3.060  1.00 25.56           C
ATOM   5543  C    PHE D  67     -2.395 -16.784  -3.970  1.00 24.85           C
ATOM   5544  O    PHE D  67     -2.729 -17.405  -4.981  1.00 24.15           O
ATOM   5545  CB   PHE D  67      0.027 -17.092  -3.729  1.00 24.88           C
ATOM   5546  CG   PHE D  67      1.234 -17.288  -2.834  1.00 25.59           C
ATOM   5547  CD1  PHE D  67      1.158 -17.987  -1.629  1.00 26.73           C
ATOM   5548  CD2  PHE D  67      2.476 -16.815  -3.246  1.00 25.12           C
ATOM   5549  CE1  PHE D  67      2.273 -18.188  -0.834  1.00 26.63           C
ATOM   5550  CE2  PHE D  67      3.596 -17.009  -2.458  1.00 25.15           C
ATOM   5551  CZ   PHE D  67      3.497 -17.684  -1.243  1.00 25.89           C
ATOM   5552  N    THR D  68     -2.850 -15.565  -3.643  1.00 24.82           N
ATOM   5553  CA   THR D  68     -3.746 -14.786  -4.491  1.00 25.50           C
ATOM   5554  C    THR D  68     -3.254 -13.355  -4.623  1.00 22.90           C
ATOM   5555  O    THR D  68     -3.064 -12.640  -3.633  1.00 23.30           O
ATOM   5556  CB   THR D  68     -5.200 -14.741  -3.946  1.00 26.07           C
ATOM   5557  OG1  THR D  68     -5.633 -16.079  -3.668  1.00 26.97           O
ATOM   5558  CG2  THR D  68     -6.132 -14.100  -4.962  1.00 27.32           C
ATOM   5559  N    ILE D  69     -3.025 -12.951  -5.866  1.00 23.80           N
ATOM   5560  CA   ILE D  69     -2.626 -11.568  -6.166  1.00 23.71           C
ATOM   5561  C    ILE D  69     -3.869 -10.719  -6.452  1.00 23.62           C
ATOM   5562  O    ILE D  69     -4.840 -11.198  -7.009  1.00 25.02           O
ATOM   5563  CB   ILE D  69     -1.597 -11.503  -7.362  1.00 23.20           C
ATOM   5564  CG1  ILE D  69     -1.009 -10.086  -7.527  1.00 23.88           C
ATOM   5565  CG2  ILE D  69     -2.223 -12.075  -8.670  1.00 22.90           C
ATOM   5566  CD1  ILE D  69      0.135  -9.971  -8.580  1.00 22.15           C
ATOM   5567  N    SER D  70     -3.849  -9.461  -6.051  1.00 24.57           N
ATOM   5568  CA   SER D  70     -4.948  -8.567  -6.350  1.00 25.28           C
ATOM   5569  C    SER D  70     -4.424  -7.154  -6.354  1.00 25.73           C
ATOM   5570  O    SER D  70     -3.343  -6.877  -5.811  1.00 25.53           O
ATOM   5571  CB   SER D  70     -6.081  -8.725  -5.300  1.00 25.37           C
ATOM   5572  OG   SER D  70     -5.610  -8.422  -3.988  1.00 25.48           O
ATOM   5573  N    ARG D  71     -5.188  -6.251  -6.959  1.00 26.67           N
ATOM   5574  CA   ARG D  71     -4.832  -4.851  -6.961  1.00 26.81           C
ATOM   5575  C    ARG D  71     -6.009  -3.974  -6.595  1.00 28.01           C
ATOM   5576  O    ARG D  71     -7.151  -4.328  -6.853  1.00 29.01           O
ATOM   5577  CB   ARG D  71     -4.303  -4.425  -8.322  1.00 25.38           C
ATOM   5578  CG   ARG D  71     -5.286  -4.525  -9.452  1.00 26.59           C
ATOM   5579  CD   ARG D  71     -4.589  -4.686 -10.800  1.00 27.23           C
ATOM   5580  NE   ARG D  71     -3.842  -3.471 -11.103  1.00 27.10           N
```

| ATOM | 5581 | CZ | ARG | D | 71 | -2.822 | -3.377 | -11.953 | 1.00 | 25.06 | C |
|------|------|------|-----|---|----|--------|--------|---------|------|-------|---|
| ATOM | 5582 | NH1 | ARG | D | 71 | -2.397 | -4.419 | -12.632 | 1.00 | 24.92 | N |
| ATOM | 5583 | NH2 | ARG | D | 71 | -2.210 | -2.207 | -12.085 | 1.00 | 26.14 | N |
| ATOM | 5584 | N | ASP | D | 72 | -5.690 | -2.840 | -5.990 | 1.00 | 29.41 | N |
| ATOM | 5585 | CA | ASP | D | 72 | -6.642 | -1.771 | -5.766 | 1.00 | 30.58 | C |
| ATOM | 5586 | C | ASP | D | 72 | -6.097 | -0.518 | -6.418 | 1.00 | 29.89 | C |
| ATOM | 5587 | O | ASP | D | 72 | -5.316 | 0.225 | -5.808 | 1.00 | 30.94 | O |
| ATOM | 5588 | CB | ASP | D | 72 | -6.847 | -1.540 | -4.282 | 1.00 | 31.39 | C |
| ATOM | 5589 | CG | ASP | D | 72 | -7.933 | -0.512 | -3.990 | 1.00 | 34.28 | C |
| ATOM | 5590 | OD1 | ASP | D | 72 | -8.112 | 0.459 | -4.766 | 1.00 | 37.66 | O |
| ATOM | 5591 | OD2 | ASP | D | 72 | -8.586 | -0.686 | -2.944 | 1.00 | 35.83 | O |
| ATOM | 5592 | N | ASP | D | 73 | -6.541 | -0.299 | -7.643 | 1.00 | 31.01 | N |
| ATOM | 5593 | CA | ASP | D | 73 | -6.155 | 0.845 | -8.467 | 1.00 | 31.50 | C |
| ATOM | 5594 | C | ASP | D | 73 | -6.432 | 2.211 | -7.835 | 1.00 | 33.91 | C |
| ATOM | 5595 | O | ASP | D | 73 | -5.698 | 3.173 | -8.093 | 1.00 | 32.65 | O |
| ATOM | 5596 | CB | ASP | D | 73 | -6.830 | 0.719 | -9.833 | 1.00 | 31.32 | C |
| ATOM | 5597 | CG | ASP | D | 73 | -6.204 | -0.351 | -10.684 | 1.00 | 30.56 | C |
| ATOM | 5598 | OD1 | ASP | D | 73 | -5.107 | -0.818 | -10.319 | 1.00 | 31.56 | O |
| ATOM | 5599 | OD2 | ASP | D | 73 | -6.784 | -0.744 | -11.713 | 1.00 | 31.81 | O |
| ATOM | 5600 | N | SER | D | 74 | -7.452 | 2.289 | -6.976 | 1.00 | 34.57 | N |
| ATOM | 5601 | CA | SER | D | 74 | -7.744 | 3.528 | -6.260 | 1.00 | 35.18 | C |
| ATOM | 5602 | C | SER | D | 74 | -6.684 | 3.884 | -5.214 | 1.00 | 35.29 | C |
| ATOM | 5603 | O | SER | D | 74 | -6.485 | 5.054 | -4.912 | 1.00 | 36.46 | O |
| ATOM | 5604 | CB | SER | D | 74 | -9.139 | 3.454 | -5.616 | 1.00 | 35.90 | C |
| ATOM | 5605 | OG | SER | D | 74 | -9.111 | 2.808 | -4.351 | 1.00 | 36.19 | O |
| ATOM | 5606 | N | LYS | D | 75 | -6.012 | 2.884 | -4.633 | 1.00 | 33.64 | N |
| ATOM | 5607 | CA | LYS | D | 75 | -4.961 | 3.144 | -3.647 | 1.00 | 33.64 | C |
| ATOM | 5608 | C | LYS | D | 75 | -3.552 | 2.949 | -4.227 | 1.00 | 32.14 | C |
| ATOM | 5609 | O | LYS | D | 75 | -2.561 | 3.118 | -3.518 | 1.00 | 33.26 | O |
| ATOM | 5610 | CB | LYS | D | 75 | -5.132 | 2.232 | -2.434 | 1.00 | 35.05 | C |
| ATOM | 5611 | CG | LYS | D | 75 | -6.386 | 2.497 | -1.619 | 1.00 | 36.60 | C |
| ATOM | 5612 | CD | LYS | D | 75 | -6.456 | 1.579 | -0.390 | 1.00 | 38.78 | C |
| ATOM | 5613 | CE | LYS | D | 75 | -7.832 | 1.597 | 0.252 | 1.00 | 39.17 | C |
| ATOM | 5614 | NZ | LYS | D | 75 | -8.448 | 2.962 | 0.314 | 1.00 | 41.33 | N |
| ATOM | 5615 | N | SER | D | 76 | -3.472 | 2.608 | -5.509 | 1.00 | 31.22 | N |
| ATOM | 5616 | CA | SER | D | 76 | -2.183 | 2.304 | -6.167 | 1.00 | 30.46 | C |
| ATOM | 5617 | C | SER | D | 76 | -1.472 | 1.194 | -5.403 | 1.00 | 28.45 | C |
| ATOM | 5618 | O | SER | D | 76 | -0.265 | 1.248 | -5.152 | 1.00 | 27.34 | O |
| ATOM | 5619 | CB | SER | D | 76 | -1.302 | 3.547 | -6.274 | 1.00 | 31.02 | C |
| ATOM | 5620 | OG | SER | D | 76 | -1.902 | 4.493 | -7.137 | 1.00 | 31.70 | O |
| ATOM | 5621 | N | ARG | D | 77 | -2.246 | 0.195 | -5.016 | 1.00 | 28.17 | N |
| ATOM | 5622 | CA | ARG | D | 77 | -1.748 | -0.858 | -4.156 | 1.00 | 27.97 | C |
| ATOM | 5623 | C | ARG | D | 77 | -1.886 | -2.203 | -4.838 | 1.00 | 26.73 | C |
| ATOM | 5624 | O | ARG | D | 77 | -2.905 | -2.503 | -5.459 | 1.00 | 25.02 | O |
| ATOM | 5625 | CB | ARG | D | 77 | -2.496 | -0.851 | -2.825 | 1.00 | 28.04 | C |
| ATOM | 5626 | CG | ARG | D | 77 | -1.588 | -1.077 | -1.643 | 1.00 | 31.37 | C |
| ATOM | 5627 | CD | ARG | D | 77 | -2.155 | -0.599 | -0.335 | 1.00 | 32.53 | C |
| ATOM | 5628 | NE | ARG | D | 77 | -1.507 | 0.587 | 0.209 | 1.00 | 33.53 | N |
| ATOM | 5629 | CZ | ARG | D | 77 | -0.314 | 0.604 | 0.787 | 1.00 | 34.67 | C |
| ATOM | 5630 | NH1 | ARG | D | 77 | 0.415 | -0.505 | 0.876 | 1.00 | 36.00 | N |
| ATOM | 5631 | NH2 | ARG | D | 77 | 0.174 | 1.743 | 1.257 | 1.00 | 35.91 | N |
| ATOM | 5632 | N | LEU | D | 78 | -0.823 | -3.000 | -4.724 | 1.00 | 25.10 | N |
| ATOM | 5633 | CA | LEU | D | 78 | -0.842 | -4.420 | -5.080 | 1.00 | 23.77 | C |
| ATOM | 5634 | C | LEU | D | 78 | -0.782 | -5.214 | -3.820 | 1.00 | 22.77 | C |
| ATOM | 5635 | O | LEU | D | 78 | -0.111 | -4.808 | -2.905 | 1.00 | 21.04 | O |
| ATOM | 5636 | CB | LEU | D | 78 | 0.378 | -4.812 | -5.912 | 1.00 | 23.22 | C |
| ATOM | 5637 | CG | LEU | D | 78 | 0.399 | -6.174 | -6.610 | 1.00 | 21.87 | C |
| ATOM | 5638 | CD1 | LEU | D | 78 | -0.609 | -6.277 | -7.748 | 1.00 | 24.05 | C |
| ATOM | 5639 | CD2 | LEU | D | 78 | 1.838 | -6.417 | -7.124 | 1.00 | 23.29 | C |
| ATOM | 5640 | N | TYR | D | 79 | -1.433 | -6.375 | -3.827 | 1.00 | 22.85 | N |
| ATOM | 5641 | CA | TYR | D | 79 | -1.526 | -7.256 | -2.653 | 1.00 | 23.48 | C |
| ATOM | 5642 | C | TYR | D | 79 | -1.128 | -8.673 | -3.024 | 1.00 | 21.19 | C |
| ATOM | 5643 | O | TYR | D | 79 | -1.289 | -9.116 | -4.151 | 1.00 | 22.05 | O |
| ATOM | 5644 | CB | TYR | D | 79 | -2.950 | -7.306 | -2.071 | 1.00 | 23.95 | C |
| ATOM | 5645 | CG | TYR | D | 79 | -3.515 | -5.968 | -1.707 | 1.00 | 25.87 | C |

| ATOM | 5646 | CD1 | TYR | D | 79  | -3.263 | -5.400  | -0.470 | 1.00 | 29.70 | C |
| ATOM | 5647 | CD2 | TYR | D | 79  | -4.271 | -5.250  | -2.617 | 1.00 | 29.22 | C |
| ATOM | 5648 | CE1 | TYR | D | 79  | -3.769 | -4.163  | -0.134 | 1.00 | 28.61 | C |
| ATOM | 5649 | CE2 | TYR | D | 79  | -4.785 | -3.996  | -2.288 | 1.00 | 30.41 | C |
| ATOM | 5650 | CZ  | TYR | D | 79  | -4.539 | -3.477  | -1.050 | 1.00 | 29.92 | C |
| ATOM | 5651 | OH  | TYR | D | 79  | -5.022 | -2.241  | -0.711 | 1.00 | 34.37 | O |
| ATOM | 5652 | N   | LEU | D | 80  | -0.620 | -9.377  | -2.045 | 1.00 | 21.15 | N |
| ATOM | 5653 | CA  | LEU | D | 80  | -0.493 | -10.815 | -2.127 | 1.00 | 21.03 | C |
| ATOM | 5654 | C   | LEU | D | 80  | -0.951 | -11.454 | -0.815 | 1.00 | 22.50 | C |
| ATOM | 5655 | O   | LEU | D | 80  | -0.395 | -11.201 | 0.246  | 1.00 | 22.01 | O |
| ATOM | 5656 | CB  | LEU | D | 80  | 0.950  | -11.214 | -2.380 | 1.00 | 21.12 | C |
| ATOM | 5657 | CG  | LEU | D | 80  | 1.242  | -12.676 | -2.709 | 1.00 | 19.20 | C |
| ATOM | 5658 | CD1 | LEU | D | 80  | 0.691  | -13.114 | -4.079 | 1.00 | 20.67 | C |
| ATOM | 5659 | CD2 | LEU | D | 80  | 2.727  | -12.918 | -2.672 | 1.00 | 21.53 | C |
| ATOM | 5660 | N   | GLN | D | 81  | -1.946 | -12.319 | -0.947 | 1.00 | 23.54 | N |
| ATOM | 5661 | CA  | GLN | D | 81  | -2.553 | -13.052 | 0.149  | 1.00 | 24.32 | C |
| ATOM | 5662 | C   | GLN | D | 81  | -1.938 | -14.407 | 0.092  | 1.00 | 23.40 | C |
| ATOM | 5663 | O   | GLN | D | 81  | -1.987 | -15.037 | -0.926 | 1.00 | 22.89 | O |
| ATOM | 5664 | CB  | GLN | D | 81  | -4.069 | -13.157 | -0.063 | 1.00 | 25.42 | C |
| ATOM | 5665 | CG  | GLN | D | 81  | -4.809 | -14.070 | 0.930  | 1.00 | 25.70 | C |
| ATOM | 5666 | CD  | GLN | D | 81  | -4.705 | -13.569 | 2.341  | 1.00 | 28.07 | C |
| ATOM | 5667 | OE1 | GLN | D | 81  | -5.054 | -12.434 | 2.617  | 1.00 | 31.12 | O |
| ATOM | 5668 | NE2 | GLN | D | 81  | -4.229 | -14.406 | 3.244  | 1.00 | 29.90 | N |
| ATOM | 5669 | N   | MET | D | 82  | -1.344 | -14.837 | 1.190  | 1.00 | 25.27 | N |
| ATOM | 5670 | CA  | MET | D | 82  | -0.553 | -16.047 | 1.218  | 1.00 | 24.97 | C |
| ATOM | 5671 | C   | MET | D | 82  | -1.141 | -16.906 | 2.317  | 1.00 | 26.06 | C |
| ATOM | 5672 | O   | MET | D | 82  | -1.199 | -16.492 | 3.488  | 1.00 | 24.45 | O |
| ATOM | 5673 | CB  | MET | D | 82  | 0.893  | -15.701 | 1.544  | 1.00 | 26.16 | C |
| ATOM | 5674 | CG  | MET | D | 82  | 1.596  | -14.820 | 0.492  | 1.00 | 26.69 | C |
| ATOM | 5675 | SD  | MET | D | 82  | 3.312  | -14.468 | 0.892  | 1.00 | 28.64 | S |
| ATOM | 5676 | CE  | MET | D | 82  | 3.140  | -13.132 | 1.967  | 1.00 | 25.32 | C |
| ATOM | 5677 | N   | ASN | D | 82A | -1.605 | -18.077 | 1.933  | 1.00 | 26.46 | N |
| ATOM | 5678 | CA  | ASN | D | 82A | -2.204 | -18.984 | 2.874  | 1.00 | 27.98 | C |
| ATOM | 5679 | C   | ASN | D | 82A | -1.386 | -20.260 | 2.993  | 1.00 | 27.39 | C |
| ATOM | 5680 | O   | ASN | D | 82A | -0.660 | -20.649 | 2.089  | 1.00 | 26.09 | O |
| ATOM | 5681 | CB  | ASN | D | 82A | -3.659 | -19.296 | 2.446  | 1.00 | 28.91 | C |
| ATOM | 5682 | CG  | ASN | D | 82A | -4.511 | -18.067 | 2.364  | 1.00 | 30.56 | C |
| ATOM | 5683 | OD1 | ASN | D | 82A | -4.436 | -17.192 | 3.219  | 1.00 | 36.65 | O |
| ATOM | 5684 | ND2 | ASN | D | 82A | -5.327 | -17.980 | 1.329  | 1.00 | 33.75 | N |
| ATOM | 5685 | N   | ASN | D | 82B | -1.523 | -20.917 | 4.141  | 1.00 | 28.80 | N |
| ATOM | 5686 | CA  | ASN | D | 82B | -0.852 | -22.169 | 4.414  | 1.00 | 29.55 | C |
| ATOM | 5687 | C   | ASN | D | 82B | 0.635  | -22.149 | 4.052  | 1.00 | 28.38 | C |
| ATOM | 5688 | O   | ASN | D | 82B | 1.171  | -23.048 | 3.390  | 1.00 | 27.39 | O |
| ATOM | 5689 | CB  | ASN | D | 82B | -1.568 | -23.327 | 3.733  | 1.00 | 32.10 | C |
| ATOM | 5690 | CG  | ASN | D | 82B | -1.859 | -24.469 | 4.699  | 1.00 | 36.33 | C |
| ATOM | 5691 | OD1 | ASN | D | 82B | -2.997 | -24.956 | 4.781  | 1.00 | 40.51 | O |
| ATOM | 5692 | ND2 | ASN | D | 82B | -0.845 | -24.876 | 5.466  | 1.00 | 34.82 | N |
| ATOM | 5693 | N   | LEU | D | 82C | 1.292  | -21.117 | 4.563  | 1.00 | 26.79 | N |
| ATOM | 5694 | CA  | LEU | D | 82C | 2.698  | -20.896 | 4.330  | 1.00 | 26.16 | C |
| ATOM | 5695 | C   | LEU | D | 82C | 3.559  | -22.053 | 4.846  | 1.00 | 25.35 | C |
| ATOM | 5696 | O   | LEU | D | 82C | 3.247  | -22.674 | 5.864  | 1.00 | 26.39 | O |
| ATOM | 5697 | CB  | LEU | D | 82C | 3.090  | -19.573 | 4.991  | 1.00 | 25.14 | C |
| ATOM | 5698 | CG  | LEU | D | 82C | 2.484  | -18.377 | 4.255  | 1.00 | 24.20 | C |
| ATOM | 5699 | CD1 | LEU | D | 82C | 2.398  | -17.065 | 5.054  | 1.00 | 26.16 | C |
| ATOM | 5700 | CD2 | LEU | D | 82C | 3.291  | -18.200 | 2.976  | 1.00 | 25.98 | C |
| ATOM | 5701 | N   | ARG | D | 83  | 4.651  | -22.315 | 4.149  | 1.00 | 26.67 | N |
| ATOM | 5702 | CA  | ARG | D | 83  | 5.646  | -23.289 | 4.559  | 1.00 | 27.42 | C |
| ATOM | 5703 | C   | ARG | D | 83  | 7.017  | -22.631 | 4.751  | 1.00 | 27.29 | C |
| ATOM | 5704 | O   | ARG | D | 83  | 7.230  | -21.497 | 4.341  | 1.00 | 23.86 | O |
| ATOM | 5705 | CB  | ARG | D | 83  | 5.736  | -24.407 | 3.530  | 1.00 | 28.71 | C |
| ATOM | 5706 | CG  | ARG | D | 83  | 4.378  | -24.951 | 3.166  | 1.00 | 32.66 | C |
| ATOM | 5707 | CD  | ARG | D | 83  | 4.434  | -26.405 | 2.764  | 1.00 | 34.73 | C |
| ATOM | 5708 | NE  | ARG | D | 83  | 4.566  | -27.251 | 3.947  | 1.00 | 38.13 | N |
| ATOM | 5709 | CZ  | ARG | D | 83  | 3.560  | -27.749 | 4.663  | 1.00 | 41.60 | C |
| ATOM | 5710 | NH1 | ARG | D | 83  | 2.286  | -27.512 | 4.329  | 1.00 | 43.14 | N |

```
ATOM   5711  NH2 ARG D  83      3.830 -28.507   5.729  1.00 42.11           N
ATOM   5712  N   THR D  84      7.948 -23.343   5.367  1.00 27.65           N
ATOM   5713  CA  THR D  84      9.281 -22.763   5.635  1.00 27.00           C
ATOM   5714  C   THR D  84      9.931 -22.248   4.343  1.00 26.19           C
ATOM   5715  O   THR D  84     10.539 -21.173   4.338  1.00 25.85           O
ATOM   5716  CB  THR D  84     10.232 -23.766   6.364  1.00 28.06           C
ATOM   5717  OG1 THR D  84     10.342 -24.946   5.587  1.00 29.99           O
ATOM   5718  CG2 THR D  84      9.686 -24.133   7.723  1.00 28.07           C
ATOM   5719  N   GLU D  85      9.728 -22.963   3.245  1.00 25.94           N
ATOM   5720  CA  GLU D  85     10.333 -22.653   1.957  1.00 26.93           C
ATOM   5721  C   GLU D  85      9.737 -21.428   1.269  1.00 25.12           C
ATOM   5722  O   GLU D  85     10.297 -20.945   0.288  1.00 26.38           O
ATOM   5723  CB  GLU D  85     10.212 -23.794   0.957  1.00 28.73           C
ATOM   5724  CG  GLU D  85     10.282 -25.170   1.498  1.00 33.98           C
ATOM   5725  CD  GLU D  85      8.950 -25.624   1.999  1.00 34.93           C
ATOM   5726  OE1 GLU D  85      8.008 -25.788   1.169  1.00 40.79           O
ATOM   5727  OE2 GLU D  85      8.844 -25.765   3.224  1.00 33.48           O
ATOM   5728  N   ASP D  86      8.614 -20.927   1.780  1.00 24.26           N
ATOM   5729  CA  ASP D  86      8.086 -19.641   1.340  1.00 22.74           C
ATOM   5730  C   ASP D  86      8.780 -18.428   1.992  1.00 22.42           C
ATOM   5731  O   ASP D  86      8.427 -17.276   1.701  1.00 21.66           O
ATOM   5732  CB  ASP D  86      6.600 -19.572   1.624  1.00 22.94           C
ATOM   5733  CG  ASP D  86      5.823 -20.611   0.872  1.00 22.42           C
ATOM   5734  OD1 ASP D  86      6.041 -20.738  -0.338  1.00 21.64           O
ATOM   5735  OD2 ASP D  86      4.970 -21.298   1.491  1.00 27.07           O
ATOM   5736  N   THR D  87      9.718 -18.690   2.881  1.00 21.59           N
ATOM   5737  CA  THR D  87     10.556 -17.667   3.486  1.00 21.13           C
ATOM   5738  C   THR D  87     11.316 -16.953   2.380  1.00 20.30           C
ATOM   5739  O   THR D  87     11.872 -17.581   1.485  1.00 20.99           O
ATOM   5740  CB  THR D  87     11.505 -18.321   4.510  1.00 22.09           C
ATOM   5741  OG1 THR D  87     10.720 -18.741   5.645  1.00 22.36           O
ATOM   5742  CG2 THR D  87     12.579 -17.365   4.987  1.00 20.08           C
ATOM   5743  N   GLY D  88     11.290 -15.626   2.416  1.00 20.84           N
ATOM   5744  CA  GLY D  88     12.189 -14.833   1.547  1.00 19.98           C
ATOM   5745  C   GLY D  88     11.815 -13.369   1.528  1.00 19.14           C
ATOM   5746  O   GLY D  88     10.943 -12.941   2.282  1.00 18.23           O
ATOM   5747  N   ILE D  89     12.503 -12.606   0.689  1.00 19.21           N
ATOM   5748  CA  ILE D  89     12.191 -11.179   0.500  1.00 17.78           C
ATOM   5749  C   ILE D  89     11.280 -11.095  -0.697  1.00 18.29           C
ATOM   5750  O   ILE D  89     11.619 -11.579  -1.762  1.00 19.97           O
ATOM   5751  CB  ILE D  89     13.455 -10.330   0.285  1.00 18.31           C
ATOM   5752  CG1 ILE D  89     14.487 -10.580   1.408  1.00 18.30           C
ATOM   5753  CG2 ILE D  89     13.104  -8.844   0.184  1.00 17.16           C
ATOM   5754  CD1 ILE D  89     15.835  -9.989   1.082  1.00 22.12           C
ATOM   5755  N   TYR D  90     10.112 -10.489  -0.499  1.00 17.77           N
ATOM   5756  CA  TYR D  90      9.080 -10.394  -1.510  1.00 16.82           C
ATOM   5757  C   TYR D  90      9.085  -9.001  -2.081  1.00 16.90           C
ATOM   5758  O   TYR D  90      8.863  -8.055  -1.340  1.00 18.67           O
ATOM   5759  CB  TYR D  90      7.716 -10.702  -0.927  1.00 17.50           C
ATOM   5760  CG  TYR D  90      7.484 -12.185  -0.746  1.00 17.16           C
ATOM   5761  CD1 TYR D  90      8.210 -12.897   0.213  1.00 19.43           C
ATOM   5762  CD2 TYR D  90      6.578 -12.878  -1.544  1.00 17.65           C
ATOM   5763  CE1 TYR D  90      8.029 -14.281   0.382  1.00 20.39           C
ATOM   5764  CE2 TYR D  90      6.395 -14.298  -1.371  1.00 16.23           C
ATOM   5765  CZ  TYR D  90      7.121 -14.961  -0.417  1.00 19.75           C
ATOM   5766  OH  TYR D  90      6.968 -16.329  -0.207  1.00 18.55           O
ATOM   5767  N   TYR D  91      9.329  -8.928  -3.383  1.00 17.11           N
ATOM   5768  CA  TYR D  91      9.386  -7.673  -4.157  1.00 17.52           C
ATOM   5769  C   TYR D  91      8.201  -7.539  -5.079  1.00 17.46           C
ATOM   5770  O   TYR D  91      7.787  -8.499  -5.738  1.00 17.32           O
ATOM   5771  CB  TYR D  91     10.608  -7.655  -5.067  1.00 17.28           C
ATOM   5772  CG  TYR D  91     11.941  -7.714  -4.404  1.00 18.04           C
ATOM   5773  CD1 TYR D  91     12.619  -6.562  -4.068  1.00 16.53           C
ATOM   5774  CD2 TYR D  91     12.547  -8.932  -4.118  1.00 17.56           C
ATOM   5775  CE1 TYR D  91     13.878  -6.595  -3.505  1.00 19.38           C
```

| ATOM | 5776 | CE2 | TYR | D | 91 | 13.803 | -8.978 | -3.520 | 1.00 | 17.75 | C |
|------|------|-----|-----|---|-----|--------|--------|--------|------|-------|---|
| ATOM | 5777 | CZ | TYR | D | 91 | 14.474 | -7.803 | -3.229 | 1.00 | 19.25 | C |
| ATOM | 5778 | OH | TYR | D | 91 | 15.707 | -7.857 | -2.653 | 1.00 | 20.88 | O |
| ATOM | 5779 | N | CYS | D | 92 | 7.670 | -6.322 | -5.152 | 1.00 | 16.62 | N |
| ATOM | 5780 | CA | CYS | D | 92 | 6.879 | -5.924 | -6.269 | 1.00 | 17.74 | C |
| ATOM | 5781 | C | CYS | D | 92 | 7.798 | -5.599 | -7.451 | 1.00 | 16.54 | C |
| ATOM | 5782 | O | CYS | D | 92 | 8.836 | -4.901 | -7.322 | 1.00 | 16.62 | O |
| ATOM | 5783 | CB | CYS | D | 92 | 5.985 | -4.732 | -5.914 | 1.00 | 20.81 | C |
| ATOM | 5784 | SG | CYS | D | 92 | 4.550 | -5.233 | -4.922 | 1.00 | 26.00 | S |
| ATOM | 5785 | N | PHE | D | 93 | 7.429 | -6.136 | -8.599 | 1.00 | 17.65 | N |
| ATOM | 5786 | CA | PHE | D | 93 | 8.136 | -5.956 | -9.853 | 1.00 | 17.87 | C |
| ATOM | 5787 | C | PHE | D | 93 | 7.119 | -5.557 | -10.941 | 1.00 | 18.55 | C |
| ATOM | 5788 | O | PHE | D | 93 | 6.189 | -6.276 | -11.242 | 1.00 | 19.20 | O |
| ATOM | 5789 | CB | PHE | D | 93 | 8.859 | -7.275 | -10.181 | 1.00 | 18.04 | C |
| ATOM | 5790 | CG | PHE | D | 93 | 9.558 | -7.316 | -11.508 | 1.00 | 15.04 | C |
| ATOM | 5791 | CD1 | PHE | D | 93 | 10.477 | -6.347 | -11.877 | 1.00 | 16.04 | C |
| ATOM | 5792 | CD2 | PHE | D | 93 | 9.353 | -8.387 | -12.345 | 1.00 | 17.81 | C |
| ATOM | 5793 | CE1 | PHE | D | 93 | 11.146 | -6.429 | -13.091 | 1.00 | 18.70 | C |
| ATOM | 5794 | CE2 | PHE | D | 93 | 9.989 | -8.465 | -13.584 | 1.00 | 18.96 | C |
| ATOM | 5795 | CZ | PHE | D | 93 | 10.899 | -7.464 | -13.938 | 1.00 | 15.96 | C |
| ATOM | 5796 | N | LEU | D | 94 | 7.310 | -4.359 | -11.505 | 1.00 | 21.09 | N |
| ATOM | 5797 | CA | LEU | D | 94 | 6.558 | -3.928 | -12.675 | 1.00 | 22.54 | C |
| ATOM | 5798 | C | LEU | D | 94 | 7.357 | -4.314 | -13.893 | 1.00 | 21.81 | C |
| ATOM | 5799 | O | LEU | D | 94 | 8.359 | -3.658 | -14.189 | 1.00 | 21.07 | O |
| ATOM | 5800 | CB | LEU | D | 94 | 6.392 | -2.415 | -12.668 | 1.00 | 24.63 | C |
| ATOM | 5801 | CG | LEU | D | 94 | 5.354 | -1.906 | -11.721 | 1.00 | 28.18 | C |
| ATOM | 5802 | CD1 | LEU | D | 94 | 5.835 | -2.160 | -10.294 | 1.00 | 30.29 | C |
| ATOM | 5803 | CD2 | LEU | D | 94 | 5.060 | -0.417 | -11.983 | 1.00 | 28.25 | C |
| ATOM | 5804 | N | PRO | D | 95 | 6.994 | -5.423 | -14.557 | 1.00 | 20.98 | N |
| ATOM | 5805 | CA | PRO | D | 95 | 7.798 | -5.879 | -15.672 | 1.00 | 21.58 | C |
| ATOM | 5806 | C | PRO | D | 95 | 7.896 | -4.796 | -16.726 | 1.00 | 20.98 | C |
| ATOM | 5807 | O | PRO | D | 95 | 6.868 | -4.214 | -17.078 | 1.00 | 21.93 | O |
| ATOM | 5808 | CB | PRO | D | 95 | 7.008 | -7.070 | -16.209 | 1.00 | 21.80 | C |
| ATOM | 5809 | CG | PRO | D | 95 | 6.282 | -7.576 | -15.013 | 1.00 | 22.39 | C |
| ATOM | 5810 | CD | PRO | D | 95 | 5.862 | -6.340 | -14.321 | 1.00 | 22.26 | C |
| ATOM | 5811 | N | MET | D | 96 | 9.092 | -4.480 | -17.220 | 1.00 | 23.09 | N |
| ATOM | 5812 | CA | MET | D | 96 | 10.359 | -5.108 | -16.810 | 1.00 | 22.46 | C |
| ATOM | 5813 | C | MET | D | 96 | 11.215 | -4.091 | -16.108 | 1.00 | 21.54 | C |
| ATOM | 5814 | O | MET | D | 96 | 12.391 | -4.331 | -15.802 | 1.00 | 22.06 | O |
| ATOM | 5815 | CB | MET | D | 96 | 11.120 | -5.623 | -18.022 | 1.00 | 22.76 | C |
| ATOM | 5816 | CG | MET | D | 96 | 10.410 | -6.658 | -18.843 | 1.00 | 21.74 | C |
| ATOM | 5817 | SD | MET | D | 96 | 10.293 | -8.200 | -17.994 | 1.00 | 21.49 | S |
| ATOM | 5818 | CE | MET | D | 96 | 11.985 | -8.779 | -18.005 | 1.00 | 21.20 | C |
| ATOM | 5819 | N | ASP | D | 101 | 10.683 | -2.777 | -15.911 | 1.00 | 22.83 | N |
| ATOM | 5820 | CA | ASP | D | 101 | 11.404 | -1.563 | -15.523 | 1.00 | 23.86 | C |
| ATOM | 5821 | C | ASP | D | 101 | 11.678 | -1.397 | -14.014 | 1.00 | 23.27 | C |
| ATOM | 5822 | O | ASP | D | 101 | 12.632 | -0.675 | -13.703 | 1.00 | 25.19 | O |
| ATOM | 5823 | CB | ASP | D | 101 | 10.792 | -0.349 | -16.176 | 1.00 | 25.43 | C |
| ATOM | 5824 | CG | ASP | D | 101 | 11.004 | -0.334 | -17.669 | 1.00 | 26.62 | C |
| ATOM | 5825 | OD1 | ASP | D | 101 | 11.447 | -1.348 | -18.257 | 1.00 | 26.92 | O |
| ATOM | 5826 | OD2 | ASP | D | 101 | 10.750 | 0.729 | -18.278 | 1.00 | 31.65 | O |
| ATOM | 5827 | N | TYR | D | 102 | 10.785 | -1.838 | -13.101 | 1.00 | 22.06 | N |
| ATOM | 5828 | CA | TYR | D | 102 | 10.770 | -1.206 | -11.737 | 1.00 | 21.34 | C |
| ATOM | 5829 | C | TYR | D | 102 | 10.690 | -2.323 | -10.693 | 1.00 | 20.41 | C |
| ATOM | 5830 | O | TYR | D | 102 | 9.896 | -3.246 | -10.827 | 1.00 | 21.11 | O |
| ATOM | 5831 | CB | TYR | D | 102 | 9.672 | -0.198 | -11.468 | 1.00 | 23.48 | C |
| ATOM | 5832 | CG | TYR | D | 102 | 9.647 | 0.912 | -12.461 | 1.00 | 23.66 | C |
| ATOM | 5833 | CD1 | TYR | D | 102 | 10.600 | 1.908 | -12.433 | 1.00 | 27.59 | C |
| ATOM | 5834 | CD2 | TYR | D | 102 | 8.655 | 0.964 | -13.409 | 1.00 | 26.84 | C |
| ATOM | 5835 | CE1 | TYR | D | 102 | 10.546 | 2.969 | -13.359 | 1.00 | 28.66 | C |
| ATOM | 5836 | CE2 | TYR | D | 102 | 8.577 | 2.017 | -14.328 | 1.00 | 28.31 | C |
| ATOM | 5837 | CZ | TYR | D | 102 | 9.532 | 3.013 | -14.290 | 1.00 | 29.04 | C |
| ATOM | 5838 | OH | TYR | D | 102 | 9.463 | 4.052 | -15.217 | 1.00 | 29.72 | O |
| ATOM | 5839 | N | TRP | D | 103 | 11.530 | -2.227 | -9.677 | 1.00 | 19.75 | N |
| ATOM | 5840 | CA | TRP | D | 103 | 11.468 | -3.086 | -8.504 | 1.00 | 20.93 | C |

| | | | | | | | | | | | |
|---|---|---|---|---|---|---|---|---|---|---|---|
| ATOM | 5841 | C   | TRP | D | 103 | 11.289 | -2.264 | -7.231 | 1.00 | 21.79 | C |
| ATOM | 5842 | O   | TRP | D | 103 | 11.804 | -1.148 | -7.133 | 1.00 | 22.19 | O |
| ATOM | 5843 | CB  | TRP | D | 103 | 12.788 | -3.831 | -8.351 | 1.00 | 19.37 | C |
| ATOM | 5844 | CG  | TRP | D | 103 | 13.129 | -4.837 | -9.403 | 1.00 | 19.38 | C |
| ATOM | 5845 | CD1 | TRP | D | 103 | 13.633 | -4.595 | -10.650 | 1.00 | 18.54 | C |
| ATOM | 5846 | CD2 | TRP | D | 103 | 13.056 | -6.249 | -9.263 | 1.00 | 19.19 | C |
| ATOM | 5847 | NE1 | TRP | D | 103 | 13.905 | -5.786 | -11.289 | 1.00 | 19.19 | N |
| ATOM | 5848 | CE2 | TRP | D | 103 | 13.535 | -6.818 | -10.462 | 1.00 | 18.87 | C |
| ATOM | 5849 | CE3 | TRP | D | 103 | 12.631 | -7.100 | -8.231 | 1.00 | 20.41 | C |
| ATOM | 5850 | CZ2 | TRP | D | 103 | 13.584 | -8.197 | -10.662 | 1.00 | 19.75 | C |
| ATOM | 5851 | CZ3 | TRP | D | 103 | 12.717 | -8.463 | -8.421 | 1.00 | 21.88 | C |
| ATOM | 5852 | CH2 | TRP | D | 103 | 13.166 | -8.999 | -9.635 | 1.00 | 20.00 | C |
| ATOM | 5853 | N   | GLY | D | 104 | 10.626 | -2.836 | -6.230 | 1.00 | 22.51 | N |
| ATOM | 5854 | CA  | GLY | D | 104 | 10.603 | -2.231 | -4.888 | 1.00 | 22.38 | C |
| ATOM | 5855 | C   | GLY | D | 104 | 11.835 | -2.572 | -4.061 | 1.00 | 24.14 | C |
| ATOM | 5856 | O   | GLY | D | 104 | 12.826 | -3.103 | -4.570 | 1.00 | 24.61 | O |
| ATOM | 5857 | N   | GLN | D | 105 | 11.751 | -2.228 | -2.780 | 1.00 | 24.86 | N |
| ATOM | 5858 | CA  | GLN | D | 105 | 12.768 | -2.474 | -1.748 | 1.00 | 26.86 | C |
| ATOM | 5859 | C   | GLN | D | 105 | 12.704 | -3.898 | -1.170 | 1.00 | 24.36 | C |
| ATOM | 5860 | O   | GLN | D | 105 | 13.686 | -4.411 | -0.625 | 1.00 | 26.24 | O |
| ATOM | 5861 | CB  | GLN | D | 105 | 12.559 | -1.488 | -0.582 | 1.00 | 28.81 | C |
| ATOM | 5862 | CG  | GLN | D | 105 | 11.047 | -1.189 | -0.328 | 1.00 | 32.72 | C |
| ATOM | 5863 | CD  | GLN | D | 105 | 10.602 | -1.091 | 1.124 | 1.00 | 37.82 | C |
| ATOM | 5864 | OE1 | GLN | D | 105 | 9.544 | -1.631 | 1.485 | 1.00 | 37.02 | O |
| ATOM | 5865 | NE2 | GLN | D | 105 | 11.370 | -0.357 | 1.958 | 1.00 | 42.26 | N |
| ATOM | 5866 | N   | GLY | D | 106 | 11.531 | -4.495 | -1.254 | 1.00 | 22.43 | N |
| ATOM | 5867 | CA  | GLY | D | 106 | 11.275 | -5.792 | -0.703 | 1.00 | 22.92 | C |
| ATOM | 5868 | C   | GLY | D | 106 | 10.775 | -5.771 | 0.718 | 1.00 | 22.33 | C |
| ATOM | 5869 | O   | GLY | D | 106 | 11.134 | -4.891 | 1.500 | 1.00 | 23.91 | O |
| ATOM | 5870 | N   | THR | D | 107 | 9.968 | -6.767 | 1.069 | 1.00 | 21.34 | N |
| ATOM | 5871 | CA  | THR | D | 107 | 9.557 | -6.968 | 2.442 | 1.00 | 21.18 | C |
| ATOM | 5872 | C   | THR | D | 107 | 9.848 | -8.397 | 2.840 | 1.00 | 22.32 | C |
| ATOM | 5873 | O   | THR | D | 107 | 9.558 | -9.310 | 2.086 | 1.00 | 19.76 | O |
| ATOM | 5874 | CB  | THR | D | 107 | 8.074 | -6.591 | 2.687 | 1.00 | 22.77 | C |
| ATOM | 5875 | OG1 | THR | D | 107 | 7.768 | -6.666 | 4.087 | 1.00 | 24.04 | O |
| ATOM | 5876 | CG2 | THR | D | 107 | 7.104 | -7.425 | 1.890 | 1.00 | 23.00 | C |
| ATOM | 5877 | N   | SER | D | 108 | 10.421 | -8.561 | 4.027 | 1.00 | 21.59 | N |
| ATOM | 5878 | CA  | SER | D | 108 | 10.873 | -9.867 | 4.446 | 1.00 | 22.00 | C |
| ATOM | 5879 | C   | SER | D | 108 | 9.743 | -10.671 | 5.070 | 1.00 | 22.49 | C |
| ATOM | 5880 | O   | SER | D | 108 | 9.071 | -10.213 | 6.000 | 1.00 | 20.69 | O |
| ATOM | 5881 | CB  | SER | D | 108 | 12.029 | -9.738 | 5.435 | 1.00 | 23.99 | C |
| ATOM | 5882 | OG  | SER | D | 108 | 12.463 | -11.012 | 5.862 | 1.00 | 27.64 | O |
| ATOM | 5883 | N   | VAL | D | 109 | 9.583 | -11.891 | 4.563 | 1.00 | 22.38 | N |
| ATOM | 5884 | CA  | VAL | D | 109 | 8.617 | -12.855 | 5.079 | 1.00 | 21.40 | C |
| ATOM | 5885 | C   | VAL | D | 109 | 9.394 | -14.017 | 5.672 | 1.00 | 22.64 | C |
| ATOM | 5886 | O   | VAL | D | 109 | 10.203 | -14.635 | 4.983 | 1.00 | 20.86 | O |
| ATOM | 5887 | CB  | VAL | D | 109 | 7.666 | -13.387 | 3.965 | 1.00 | 22.16 | C |
| ATOM | 5888 | CG1 | VAL | D | 109 | 6.756 | -14.464 | 4.515 | 1.00 | 22.75 | C |
| ATOM | 5889 | CG2 | VAL | D | 109 | 6.817 | -12.278 | 3.382 | 1.00 | 21.95 | C |
| ATOM | 5890 | N   | THR | D | 110 | 9.181 | -14.287 | 6.965 | 1.00 | 22.69 | N |
| ATOM | 5891 | CA  | THR | D | 110 | 9.837 | -15.409 | 7.624 | 1.00 | 21.55 | C |
| ATOM | 5892 | C   | THR | D | 110 | 8.780 | -16.394 | 8.081 | 1.00 | 20.05 | C |
| ATOM | 5893 | O   | THR | D | 110 | 7.892 | -16.045 | 8.840 | 1.00 | 21.02 | O |
| ATOM | 5894 | CB  | THR | D | 110 | 10.636 | -14.970 | 8.879 | 1.00 | 22.92 | C |
| ATOM | 5895 | OG1 | THR | D | 110 | 11.584 | -13.992 | 8.505 | 1.00 | 25.53 | O |
| ATOM | 5896 | CG2 | THR | D | 110 | 11.371 | -16.130 | 9.482 | 1.00 | 22.91 | C |
| ATOM | 5897 | N   | VAL | D | 111 | 8.897 | -17.620 | 7.597 | 1.00 | 19.60 | N |
| ATOM | 5898 | CA  | VAL | D | 111 | 7.995 | -18.686 | 8.005 | 1.00 | 22.01 | C |
| ATOM | 5899 | C   | VAL | D | 111 | 8.764 | -19.699 | 8.838 | 1.00 | 23.19 | C |
| ATOM | 5900 | O   | VAL | D | 111 | 9.566 | -20.473 | 8.322 | 1.00 | 24.94 | O |
| ATOM | 5901 | CB  | VAL | D | 111 | 7.351 | -19.383 | 6.803 | 1.00 | 22.79 | C |
| ATOM | 5902 | CG1 | VAL | D | 111 | 6.294 | -20.401 | 7.310 | 1.00 | 20.19 | C |
| ATOM | 5903 | CG2 | VAL | D | 111 | 6.700 | -18.408 | 5.900 | 1.00 | 20.83 | C |
| ATOM | 5904 | N   | SER | D | 112 | 8.491 | -19.680 | 10.134 | 1.00 | 25.89 | N |
| ATOM | 5905 | CA  | SER | D | 112 | 9.279 | -20.445 | 11.110 | 1.00 | 27.23 | C |

| ATOM | 5906 | C   | SER D 112 |  8.445 | -20.762 | 12.325 | 1.00 | 26.92 | C |
| ATOM | 5907 | O   | SER D 112 |  7.629 | -19.952 | 12.750 | 1.00 | 27.57 | O |
| ATOM | 5908 | CB  | SER D 112 | 10.489 | -19.631 | 11.577 | 1.00 | 28.53 | C |
| ATOM | 5909 | OG  | SER D 112 | 11.258 | -20.322 | 12.549 | 1.00 | 26.54 | O |
| ATOM | 5910 | N   | SER D 113 |  8.701 | -21.916 | 12.922 | 1.00 | 30.87 | N |
| ATOM | 5911 | CA  | SER D 113 |  8.084 | -22.224 | 14.211 | 1.00 | 31.51 | C |
| ATOM | 5912 | C   | SER D 113 |  8.884 | -21.658 | 15.403 | 1.00 | 32.03 | C |
| ATOM | 5913 | O   | SER D 113 |  8.407 | -21.700 | 16.532 | 1.00 | 30.63 | O |
| ATOM | 5914 | CB  | SER D 113 |  7.880 | -23.733 | 14.346 | 1.00 | 32.31 | C |
| ATOM | 5915 | OG  | SER D 113 |  9.116 | -24.395 | 14.313 | 1.00 | 35.34 | O |
| ATOM | 5916 | N   | ALA D 114 | 10.076 | -21.105 | 15.146 | 1.00 | 31.04 | N |
| ATOM | 5917 | CA  | ALA D 114 | 10.893 | -20.497 | 16.202 | 1.00 | 31.03 | C |
| ATOM | 5918 | C   | ALA D 114 | 10.241 | -19.289 | 16.852 | 1.00 | 30.80 | C |
| ATOM | 5919 | O   | ALA D 114 |  9.560 | -18.497 | 16.209 | 1.00 | 33.30 | O |
| ATOM | 5920 | CB  | ALA D 114 | 12.247 | -20.127 | 15.671 | 1.00 | 30.29 | C |
| ATOM | 5921 | N   | LYS D 115 | 10.493 | -19.112 | 18.141 | 1.00 | 30.40 | N |
| ATOM | 5922 | CA  | LYS D 115 |  9.805 | -18.103 | 18.910 | 1.00 | 31.41 | C |
| ATOM | 5923 | C   | LYS D 115 | 10.492 | -16.743 | 18.804 | 1.00 | 30.39 | C |
| ATOM | 5924 | O   | LYS D 115 | 11.716 | -16.673 | 18.828 | 1.00 | 30.48 | O |
| ATOM | 5925 | CB  | LYS D 115 |  9.775 | -18.528 | 20.381 | 1.00 | 32.86 | C |
| ATOM | 5926 | CG  | LYS D 115 |  8.670 | -17.859 | 21.174 | 1.00 | 34.32 | C |
| ATOM | 5927 | CD  | LYS D 115 |  8.501 | -18.453 | 22.594 | 1.00 | 35.71 | C |
| ATOM | 5928 | CE  | LYS D 115 |  7.303 | -19.414 | 22.692 | 1.00 | 37.22 | C |
| ATOM | 5929 | NZ  | LYS D 115 |  7.124 | -20.012 | 24.074 | 1.00 | 39.13 | N |
| ATOM | 5930 | N   | THR D 116 |  9.707 | -15.677 | 18.726 | 1.00 | 28.17 | N |
| ATOM | 5931 | CA  | THR D 116 | 10.225 | -14.327 | 18.797 | 1.00 | 27.42 | C |
| ATOM | 5932 | C   | THR D 116 | 10.944 | -14.152 | 20.141 | 1.00 | 27.35 | C |
| ATOM | 5933 | O   | THR D 116 | 10.385 | -14.492 | 21.189 | 1.00 | 27.90 | O |
| ATOM | 5934 | CB  | THR D 116 |  9.108 | -13.272 | 18.672 | 1.00 | 27.64 | C |
| ATOM | 5935 | OG1 | THR D 116 |  8.510 | -13.353 | 17.365 | 1.00 | 27.69 | O |
| ATOM | 5936 | CG2 | THR D 116 |  9.647 | -11.857 | 18.884 | 1.00 | 27.37 | C |
| ATOM | 5937 | N   | THR D 117 | 12.175 | -13.636 | 20.092 | 1.00 | 25.38 | N |
| ATOM | 5938 | CA  | THR D 117 | 13.069 | -13.542 | 21.260 | 1.00 | 23.70 | C |
| ATOM | 5939 | C   | THR D 117 | 13.842 | -12.219 | 21.124 | 1.00 | 23.33 | C |
| ATOM | 5940 | O   | THR D 117 | 14.403 | -11.972 | 20.074 | 1.00 | 21.75 | O |
| ATOM | 5941 | CB  | THR D 117 | 14.061 | -14.720 | 21.260 | 1.00 | 23.52 | C |
| ATOM | 5942 | OG1 | THR D 117 | 13.357 | -15.957 | 21.163 | 1.00 | 24.38 | O |
| ATOM | 5943 | CG2 | THR D 117 | 14.910 | -14.745 | 22.496 | 1.00 | 23.53 | C |
| ATOM | 5944 | N   | PRO D 118 | 13.860 | -11.380 | 22.159 | 1.00 | 22.45 | N |
| ATOM | 5945 | CA  | PRO D 118 | 14.646 | -10.149 | 22.128 | 1.00 | 23.09 | C |
| ATOM | 5946 | C   | PRO D 118 | 16.156 | -10.405 | 22.256 | 1.00 | 22.17 | C |
| ATOM | 5947 | O   | PRO D 118 | 16.575 | -11.404 | 22.872 | 1.00 | 21.74 | O |
| ATOM | 5948 | CB  | PRO D 118 | 14.153 |  -9.395 | 23.352 | 1.00 | 23.41 | C |
| ATOM | 5949 | CG  | PRO D 118 | 13.765 | -10.464 | 24.296 | 1.00 | 23.45 | C |
| ATOM | 5950 | CD  | PRO D 118 | 13.135 | -11.512 | 23.431 | 1.00 | 22.62 | C |
| ATOM | 5951 | N   | PRO D 119 | 16.995 |  -9.526 | 21.669 | 1.00 | 22.13 | N |
| ATOM | 5952 | CA  | PRO D 119 | 18.417 |  -9.738 | 21.864 | 1.00 | 20.70 | C |
| ATOM | 5953 | C   | PRO D 119 | 18.940 |  -9.265 | 23.202 | 1.00 | 19.30 | C |
| ATOM | 5954 | O   | PRO D 119 | 18.418 |  -8.331 | 23.816 | 1.00 | 18.14 | O |
| ATOM | 5955 | CB  | PRO D 119 | 19.043 |  -8.862 | 20.779 | 1.00 | 21.03 | C |
| ATOM | 5956 | CG  | PRO D 119 | 18.073 |  -7.743 | 20.634 | 1.00 | 21.59 | C |
| ATOM | 5957 | CD  | PRO D 119 | 16.736 |  -8.370 | 20.793 | 1.00 | 22.62 | C |
| ATOM | 5958 | N   | SER D 120 | 20.012 |  -9.905 | 23.616 | 1.00 | 18.59 | N |
| ATOM | 5959 | CA  | SER D 120 | 20.835 |  -9.441 | 24.692 | 1.00 | 17.12 | C |
| ATOM | 5960 | C   | SER D 120 | 21.959 |  -8.631 | 24.016 | 1.00 | 16.38 | C |
| ATOM | 5961 | O   | SER D 120 | 22.686 |  -9.137 | 23.146 | 1.00 | 15.70 | O |
| ATOM | 5962 | CB  | SER D 120 | 21.373 | -10.636 | 25.475 | 1.00 | 17.86 | C |
| ATOM | 5963 | OG  | SER D 120 | 20.347 | -11.292 | 26.206 | 1.00 | 18.28 | O |
| ATOM | 5964 | N   | VAL D 121 | 22.057 |  -7.357 | 24.371 | 1.00 | 17.11 | N |
| ATOM | 5965 | CA  | VAL D 121 | 23.012 |  -6.487 | 23.738 | 1.00 | 18.72 | C |
| ATOM | 5966 | C   | VAL D 121 | 24.183 |  -6.188 | 24.682 | 1.00 | 18.44 | C |
| ATOM | 5967 | O   | VAL D 121 | 23.986 |  -5.617 | 25.767 | 1.00 | 18.52 | O |
| ATOM | 5968 | CB  | VAL D 121 | 22.348 |  -5.151 | 23.264 | 1.00 | 18.52 | C |
| ATOM | 5969 | CG1 | VAL D 121 | 23.398 |  -4.233 | 22.703 | 1.00 | 20.80 | C |
| ATOM | 5970 | CG2 | VAL D 121 | 21.274 |  -5.407 | 22.234 | 1.00 | 18.33 | C |

```
ATOM   5971  N   TYR D 122    25.390  -6.590  24.274  1.00 18.47           N
ATOM   5972  CA  TYR D 122    26.578  -6.532  25.100  1.00 17.62           C
ATOM   5973  C   TYR D 122    27.662  -5.635  24.529  1.00 17.42           C
ATOM   5974  O   TYR D 122    27.950  -5.688  23.334  1.00 15.84           O
ATOM   5975  CB  TYR D 122    27.168  -7.924  25.230  1.00 17.81           C
ATOM   5976  CG  TYR D 122    26.206  -8.918  25.855  1.00 16.48           C
ATOM   5977  CD1 TYR D 122    25.551  -8.625  27.009  1.00 17.68           C
ATOM   5978  CD2 TYR D 122    26.013 -10.181  25.287  1.00 18.98           C
ATOM   5979  CE1 TYR D 122    24.660  -9.566  27.612  1.00 19.63           C
ATOM   5980  CE2 TYR D 122    25.168 -11.115  25.890  1.00 20.11           C
ATOM   5981  CZ  TYR D 122    24.481 -10.785  27.027  1.00 17.89           C
ATOM   5982  OH  TYR D 122    23.633 -11.725  27.607  1.00 21.22           O
ATOM   5983  N   PRO D 123    28.305  -4.850  25.403  1.00 19.44           N
ATOM   5984  CA  PRO D 123    29.374  -3.988  24.921  1.00 20.04           C
ATOM   5985  C   PRO D 123    30.653  -4.755  24.695  1.00 19.24           C
ATOM   5986  O   PRO D 123    30.974  -5.716  25.436  1.00 18.98           O
ATOM   5987  CB  PRO D 123    29.544  -2.976  26.061  1.00 20.05           C
ATOM   5988  CG  PRO D 123    29.222  -3.787  27.298  1.00 20.79           C
ATOM   5989  CD  PRO D 123    28.089  -4.702  26.856  1.00 19.74           C
ATOM   5990  N   LEU D 124    31.389  -4.356  23.671  1.00 18.29           N
ATOM   5991  CA  LEU D 124    32.704  -4.929  23.400  1.00 18.45           C
ATOM   5992  C   LEU D 124    33.760  -3.849  23.576  1.00 19.54           C
ATOM   5993  O   LEU D 124    33.879  -2.937  22.728  1.00 19.21           O
ATOM   5994  CB  LEU D 124    32.804  -5.523  22.017  1.00 18.57           C
ATOM   5995  CG  LEU D 124    31.769  -6.600  21.699  1.00 18.20           C
ATOM   5996  CD1 LEU D 124    31.875  -7.037  20.280  1.00 16.02           C
ATOM   5997  CD2 LEU D 124    31.918  -7.738  22.607  1.00 19.14           C
ATOM   5998  N   ALA D 125    34.472  -3.949  24.703  1.00 21.31           N
ATOM   5999  CA  ALA D 125    35.558  -3.034  25.079  1.00 22.71           C
ATOM   6000  C   ALA D 125    36.853  -3.818  25.256  1.00 22.16           C
ATOM   6001  O   ALA D 125    36.827  -4.976  25.676  1.00 21.58           O
ATOM   6002  CB  ALA D 125    35.207  -2.283  26.359  1.00 24.56           C
ATOM   6003  N   PRO D 126    38.008  -3.214  24.925  1.00 23.74           N
ATOM   6004  CA  PRO D 126    39.306  -3.863  25.002  1.00 24.72           C
ATOM   6005  C   PRO D 126    39.696  -4.338  26.397  1.00 26.11           C
ATOM   6006  O   PRO D 126    39.413  -3.652  27.385  1.00 26.81           O
ATOM   6007  CB  PRO D 126    40.290  -2.770  24.591  1.00 25.65           C
ATOM   6008  CG  PRO D 126    39.467  -1.676  24.036  1.00 23.98           C
ATOM   6009  CD  PRO D 126    38.097  -1.823  24.432  1.00 24.74           C
ATOM   6010  N   GLY D 127    40.357  -5.484  26.444  1.00 27.93           N
ATOM   6011  CA  GLY D 127    40.943  -6.035  27.677  1.00 29.26           C
ATOM   6012  C   GLY D 127    42.239  -5.301  28.011  1.00 32.82           C
ATOM   6013  O   GLY D 127    42.275  -4.057  27.949  1.00 35.57           O
ATOM   6014  N   SER D 136    47.377   3.988  16.887  1.00 38.10           N
ATOM   6015  CA  SER D 136    46.685   5.138  16.329  1.00 37.55           C
ATOM   6016  C   SER D 136    45.167   5.048  16.457  1.00 36.15           C
ATOM   6017  O   SER D 136    44.502   6.059  16.693  1.00 35.47           O
ATOM   6018  CB  SER D 136    47.017   5.280  14.839  1.00 38.42           C
ATOM   6019  OG  SER D 136    46.240   6.341  14.273  1.00 39.83           O
ATOM   6020  N   MET D 137    44.638   3.839  16.265  1.00 33.64           N
ATOM   6021  CA  MET D 137    43.201   3.614  16.208  1.00 33.26           C
ATOM   6022  C   MET D 137    42.766   2.653  17.291  1.00 30.76           C
ATOM   6023  O   MET D 137    43.541   1.809  17.700  1.00 30.79           O
ATOM   6024  CB  MET D 137    42.798   3.034  14.851  1.00 33.83           C
ATOM   6025  CG  MET D 137    43.215   3.855  13.656  1.00 36.50           C
ATOM   6026  SD  MET D 137    42.270   5.360  13.619  1.00 40.79           S
ATOM   6027  CE  MET D 137    40.813   4.860  12.725  1.00 39.30           C
ATOM   6028  N   VAL D 138    41.529   2.798  17.746  1.00 27.97           N
ATOM   6029  CA  VAL D 138    40.934   1.844  18.707  1.00 26.16           C
ATOM   6030  C   VAL D 138    39.653   1.297  18.103  1.00 24.18           C
ATOM   6031  O   VAL D 138    38.867   2.014  17.527  1.00 20.34           O
ATOM   6032  CB  VAL D 138    40.685   2.477  20.111  1.00 26.92           C
ATOM   6033  CG1 VAL D 138    39.660   3.607  20.077  1.00 26.74           C
ATOM   6034  CG2 VAL D 138    40.269   1.399  21.100  1.00 27.90           C
ATOM   6035  N   THR D 139    39.458  -0.016  18.216  1.00 22.40           N
```

```
ATOM   6036  CA   THR D 139   38.246   -0.617  17.744  1.00 20.22      C
ATOM   6037  C    THR D 139   37.413   -1.028  18.950  1.00 19.10      C
ATOM   6038  O    THR D 139   37.934   -1.539  19.937  1.00 19.55      O
ATOM   6039  CB   THR D 139   38.533   -1.840  16.858  1.00 21.87      C
ATOM   6040  OG1  THR D 139   39.183   -1.428  15.647  1.00 22.43      O
ATOM   6041  CG2  THR D 139   37.279   -2.551  16.513  1.00 19.47      C
ATOM   6042  N    LEU D 140   36.134   -0.716  18.870  1.00 19.51      N
ATOM   6043  CA   LEU D 140   35.137   -1.100  19.860  1.00 19.05      C
ATOM   6044  C    LEU D 140   34.033   -1.875  19.158  1.00 18.19      C
ATOM   6045  O    LEU D 140   33.977   -1.911  17.963  1.00 18.57      O
ATOM   6046  CB   LEU D 140   34.503    0.139  20.475  1.00 20.35      C
ATOM   6047  CG   LEU D 140   35.510    1.128  21.082  1.00 19.76      C
ATOM   6048  CD1  LEU D 140   34.719    2.274  21.576  1.00 23.01      C
ATOM   6049  CD2  LEU D 140   36.390    0.494  22.183  1.00 19.43      C
ATOM   6050  N    GLY D 141   33.090   -2.437  19.904  1.00 16.93      N
ATOM   6051  CA   GLY D 141   31.936   -3.007  19.223  1.00 17.17      C
ATOM   6052  C    GLY D 141   30.791   -3.359  20.148  1.00 14.44      C
ATOM   6053  O    GLY D 141   30.808   -3.052  21.338  1.00 15.49      O
ATOM   6054  N    CYS D 142   29.796   -3.959  19.515  1.00 16.25      N
ATOM   6055  CA ACYS D 142   28.606   -4.421  20.222  0.50 16.68      C
ATOM   6056  CA BCYS D 142   28.489   -4.292  20.059  0.50 17.15      C
ATOM   6057  C    CYS D 142   28.208   -5.774  19.669  1.00 16.38      C
ATOM   6058  O    CYS D 142   28.303   -6.059  18.498  1.00 16.89      O
ATOM   6059  CB ACYS D 142   27.441   -3.455  20.124  0.50 17.57      C
ATOM   6060  CB BCYS D 142   27.488   -3.363  19.342  0.50 19.39      C
ATOM   6061  SG ACYS D 142   27.552   -2.089  21.275  0.50 22.30      S
ATOM   6062  SG BCYS D 142   25.985   -3.087  20.162  0.50 23.17      S
ATOM   6063  N    LEU D 143   27.821   -6.627  20.606  1.00 16.50      N
ATOM   6064  CA   LEU D 143   27.413   -8.003  20.346  1.00 17.14      C
ATOM   6065  C    LEU D 143   25.909   -8.066  20.624  1.00 15.26      C
ATOM   6066  O    LEU D 143   25.452   -7.719  21.694  1.00 17.85      O
ATOM   6067  CB   LEU D 143   28.187   -8.959  21.257  1.00 16.80      C
ATOM   6068  CG   LEU D 143   27.863  -10.467  21.148  1.00 15.40      C
ATOM   6069  CD1  LEU D 143   27.989  -11.000  19.749  1.00 15.77      C
ATOM   6070  CD2  LEU D 143   28.621  -11.299  22.181  1.00 16.55      C
ATOM   6071  N    VAL D 144   25.142   -8.494  19.647  1.00 14.34      N
ATOM   6072  CA   VAL D 144   23.706   -8.478  19.671  1.00 16.64      C
ATOM   6073  C    VAL D 144   23.293   -9.925  19.552  1.00 17.55      C
ATOM   6074  O    VAL D 144   23.235  -10.456  18.474  1.00 16.83      O
ATOM   6075  CB   VAL D 144   23.158   -7.643  18.498  1.00 16.84      C
ATOM   6076  CG1  VAL D 144   21.615   -7.609  18.507  1.00 17.70      C
ATOM   6077  CG2  VAL D 144   23.673   -6.186  18.593  1.00 19.06      C
ATOM   6078  N    LYS D 145   23.068  -10.561  20.691  1.00 18.30      N
ATOM   6079  CA   LYS D 145   23.063  -12.016  20.764  1.00 19.47      C
ATOM   6080  C    LYS D 145   21.700  -12.611  21.092  1.00 19.34      C
ATOM   6081  O    LYS D 145   21.031  -12.149  21.985  1.00 19.42      O
ATOM   6082  CB   LYS D 145   24.078  -12.441  21.832  1.00 20.36      C
ATOM   6083  CG   LYS D 145   24.152  -13.969  22.021  1.00 20.52      C
ATOM   6084  CD   LYS D 145   25.484  -14.356  22.654  1.00 20.37      C
ATOM   6085  CE   LYS D 145   25.555  -15.841  22.941  1.00 22.97      C
ATOM   6086  NZ   LYS D 145   25.289  -16.636  21.757  1.00 24.20      N
ATOM   6087  N    GLY D 146   21.335  -13.680  20.397  1.00 18.19      N
ATOM   6088  CA   GLY D 146   20.192  -14.516  20.761  1.00 18.96      C
ATOM   6089  C    GLY D 146   18.811  -13.966  20.517  1.00 18.78      C
ATOM   6090  O    GLY D 146   17.937  -14.106  21.373  1.00 17.83      O
ATOM   6091  N    TYR D 147   18.592  -13.403  19.328  1.00 19.69      N
ATOM   6092  CA   TYR D 147   17.278  -12.869  18.976  1.00 19.22      C
ATOM   6093  C    TYR D 147   16.617  -13.616  17.810  1.00 19.44      C
ATOM   6094  O    TYR D 147   17.251  -14.382  17.073  1.00 19.55      O
ATOM   6095  CB   TYR D 147   17.359  -11.352  18.678  1.00 19.07      C
ATOM   6096  CG   TYR D 147   18.162  -11.013  17.418  1.00 17.55      C
ATOM   6097  CD1  TYR D 147   19.542  -10.877  17.460  1.00 17.61      C
ATOM   6098  CD2  TYR D 147   17.532  -10.886  16.170  1.00 18.69      C
ATOM   6099  CE1  TYR D 147   20.256  -10.603  16.316  1.00 16.20      C
ATOM   6100  CE2  TYR D 147   18.240  -10.627  15.023  1.00 18.69      C
```

```
ATOM   6101  CZ   TYR D 147     19.597 -10.469  15.100  1.00 17.30           C
ATOM   6102  OH   TYR D 147     20.294 -10.206  13.954  1.00 20.24           O
ATOM   6103  N    PHE D 148     15.303 -13.390  17.698  1.00 21.08           N
ATOM   6104  CA   PHE D 148     14.501 -13.906  16.578  1.00 20.70           C
ATOM   6105  C    PHE D 148     13.199 -13.116  16.489  1.00 21.14           C
ATOM   6106  O    PHE D 148     12.626 -12.739  17.515  1.00 22.28           O
ATOM   6107  CB   PHE D 148     14.240 -15.394  16.781  1.00 21.40           C
ATOM   6108  CG   PHE D 148     13.739 -16.092  15.548  1.00 21.84           C
ATOM   6109  CD1  PHE D 148     14.636 -16.571  14.617  1.00 20.42           C
ATOM   6110  CD2  PHE D 148     12.363 -16.190  15.272  1.00 22.04           C
ATOM   6111  CE1  PHE D 148     14.199 -17.189  13.437  1.00 23.90           C
ATOM   6112  CE2  PHE D 148     11.928 -16.804  14.100  1.00 23.08           C
ATOM   6113  CZ   PHE D 148     12.836 -17.308  13.188  1.00 21.95           C
ATOM   6114  N    PRO D 149     12.751 -12.783  15.271  1.00 21.03           N
ATOM   6115  CA   PRO D 149     13.331 -13.022  13.968  1.00 21.53           C
ATOM   6116  C    PRO D 149     14.242 -11.851  13.585  1.00 20.97           C
ATOM   6117  O    PRO D 149     14.410 -10.911  14.381  1.00 22.88           O
ATOM   6118  CB   PRO D 149     12.081 -13.099  13.070  1.00 20.47           C
ATOM   6119  CG   PRO D 149     11.132 -12.086  13.694  1.00 22.25           C
ATOM   6120  CD   PRO D 149     11.429 -12.108  15.171  1.00 21.30           C
ATOM   6121  N    GLU D 150     14.792 -11.921  12.380  1.00 24.48           N
ATOM   6122  CA   GLU D 150     15.433 -10.749  11.782  1.00 25.01           C
ATOM   6123  C    GLU D 150     14.323  -9.765  11.445  1.00 27.55           C
ATOM   6124  O    GLU D 150     13.184 -10.172  11.220  1.00 26.09           O
ATOM   6125  CB   GLU D 150     16.179 -11.126  10.509  1.00 25.88           C
ATOM   6126  CG   GLU D 150     17.523 -11.732  10.766  1.00 27.94           C
ATOM   6127  CD   GLU D 150     18.604 -10.690  10.743  1.00 28.37           C
ATOM   6128  OE1  GLU D 150     19.259 -10.648   9.696  1.00 29.34           O
ATOM   6129  OE2  GLU D 150     18.766  -9.883  11.712  1.00 27.72           O
ATOM   6130  N    PRO D 151     14.651  -8.479  11.344  1.00 28.39           N
ATOM   6131  CA   PRO D 151     15.937  -7.861  11.553  1.00 28.38           C
ATOM   6132  C    PRO D 151     16.089  -6.995  12.808  1.00 27.88           C
ATOM   6133  O    PRO D 151     15.140  -6.786  13.571  1.00 26.17           O
ATOM   6134  CB   PRO D 151     16.034  -6.977  10.324  1.00 29.93           C
ATOM   6135  CG   PRO D 151     14.658  -6.401  10.258  1.00 30.19           C
ATOM   6136  CD   PRO D 151     13.710  -7.507  10.737  1.00 29.63           C
ATOM   6137  N    VAL D 152     17.319  -6.512  12.996  1.00 26.63           N
ATOM   6138  CA   VAL D 152     17.659  -5.563  14.065  1.00 27.24           C
ATOM   6139  C    VAL D 152     18.160  -4.341  13.286  1.00 27.99           C
ATOM   6140  O    VAL D 152     18.796  -4.511  12.244  1.00 29.17           O
ATOM   6141  CB   VAL D 152     18.598  -6.116  15.099  1.00 27.28           C
ATOM   6142  CG1  VAL D 152     17.889  -7.273  15.791  1.00 27.37           C
ATOM   6143  CG2  VAL D 152     19.868  -6.556  14.472  1.00 24.39           C
ATOM   6144  N    THR D 153     17.994  -3.161  13.867  1.00 28.28           N
ATOM   6145  CA   THR D 153     18.915  -2.049  13.509  1.00 29.82           C
ATOM   6146  C    THR D 153     20.006  -1.706  14.448  1.00 27.84           C
ATOM   6147  O    THR D 153     19.778  -1.657  15.644  1.00 26.42           O
ATOM   6148  CB   THR D 153     17.988  -0.794  13.305  1.00 30.11           C
ATOM   6149  OG1  THR D 153     17.339  -0.460  14.542  1.00 34.58           O
ATOM   6150  CG2  THR D 153     16.895  -1.080  12.247  1.00 32.85           C
ATOM   6151  N    VAL D 154     21.186  -1.444  13.878  1.00 27.40           N
ATOM   6152  CA   VAL D 154     22.347  -1.030  14.651  1.00 26.19           C
ATOM   6153  C    VAL D 154     22.905   0.268  14.093  1.00 26.49           C
ATOM   6154  O    VAL D 154     23.185   0.356  12.914  1.00 26.38           O
ATOM   6155  CB   VAL D 154     23.431  -2.098  14.639  1.00 26.98           C
ATOM   6156  CG1  VAL D 154     24.609  -1.663  15.494  1.00 25.40           C
ATOM   6157  CG2  VAL D 154     22.861  -3.412  15.166  1.00 25.97           C
ATOM   6158  N    THR D 155     23.018   1.270  14.956  1.00 26.13           N
ATOM   6159  CA   THR D 155     23.803   2.472  14.653  1.00 26.84           C
ATOM   6160  C    THR D 155     24.747   2.804  15.799  1.00 26.49           C
ATOM   6161  O    THR D 155     24.707   2.184  16.878  1.00 24.69           O
ATOM   6162  CB   THR D 155     22.892   3.675  14.314  1.00 27.18           C
ATOM   6163  OG1  THR D 155     22.101   4.015  15.445  1.00 29.85           O
ATOM   6164  CG2  THR D 155     21.964   3.313  13.204  1.00 28.55           C
ATOM   6165  N    TRP D 156     25.641   3.749  15.538  1.00 24.82           N
```

| ATOM | 6166 | CA | TRP | D | 156 | 26.556 | 4.233 | 16.532 | 1.00 | 24.78 | C |
|------|------|-----|-----|---|-----|--------|--------|--------|------|-------|---|
| ATOM | 6167 | C | TRP | D | 156 | 26.300 | 5.711 | 16.708 | 1.00 | 28.07 | C |
| ATOM | 6168 | O | TRP | D | 156 | 26.097 | 6.423 | 15.713 | 1.00 | 28.23 | O |
| ATOM | 6169 | CB | TRP | D | 156 | 27.989 | 3.954 | 16.114 | 1.00 | 24.03 | C |
| ATOM | 6170 | CG | TRP | D | 156 | 28.323 | 2.494 | 16.169 | 1.00 | 23.96 | C |
| ATOM | 6171 | CD1 | TRP | D | 156 | 28.184 | 1.595 | 15.167 | 1.00 | 24.09 | C |
| ATOM | 6172 | CD2 | TRP | D | 156 | 28.803 | 1.770 | 17.305 | 1.00 | 22.66 | C |
| ATOM | 6173 | NE1 | TRP | D | 156 | 28.568 | 0.335 | 15.594 | 1.00 | 22.36 | N |
| ATOM | 6174 | CE2 | TRP | D | 156 | 28.959 | 0.417 | 16.902 | 1.00 | 22.38 | C |
| ATOM | 6175 | CE3 | TRP | D | 156 | 29.116 | 2.132 | 18.624 | 1.00 | 22.80 | C |
| ATOM | 6176 | CZ2 | TRP | D | 156 | 29.434 | -0.575 | 17.769 | 1.00 | 20.79 | C |
| ATOM | 6177 | CZ3 | TRP | D | 156 | 29.590 | 1.140 | 19.492 | 1.00 | 24.14 | C |
| ATOM | 6178 | CH2 | TRP | D | 156 | 29.751 | -0.191 | 19.046 | 1.00 | 23.14 | C |
| ATOM | 6179 | N | ASN | D | 157 | 26.272 | 6.145 | 17.970 | 1.00 | 28.86 | N |
| ATOM | 6180 | CA | ASN | D | 157 | 26.012 | 7.547 | 18.330 | 1.00 | 31.20 | C |
| ATOM | 6181 | C | ASN | D | 157 | 24.864 | 8.116 | 17.519 | 1.00 | 32.79 | C |
| ATOM | 6182 | O | ASN | D | 157 | 24.967 | 9.223 | 16.974 | 1.00 | 34.85 | O |
| ATOM | 6183 | CB | ASN | D | 157 | 27.275 | 8.380 | 18.166 | 1.00 | 31.36 | C |
| ATOM | 6184 | CG | ASN | D | 157 | 28.296 | 8.086 | 19.232 | 1.00 | 30.51 | C |
| ATOM | 6185 | OD1 | ASN | D | 157 | 28.046 | 7.305 | 20.151 | 1.00 | 30.88 | O |
| ATOM | 6186 | ND2 | ASN | D | 157 | 29.464 | 8.696 | 19.109 | 1.00 | 32.73 | N |
| ATOM | 6187 | N | SER | D | 158 | 23.791 | 7.327 | 17.430 | 1.00 | 34.27 | N |
| ATOM | 6188 | CA | SER | D | 158 | 22.545 | 7.697 | 16.749 | 1.00 | 35.72 | C |
| ATOM | 6189 | C | SER | D | 158 | 22.699 | 7.954 | 15.246 | 1.00 | 36.82 | C |
| ATOM | 6190 | O | SER | D | 158 | 21.906 | 8.716 | 14.667 | 1.00 | 37.88 | O |
| ATOM | 6191 | CB | SER | D | 158 | 21.902 | 8.906 | 17.432 | 1.00 | 36.17 | C |
| ATOM | 6192 | OG | SER | D | 158 | 21.924 | 8.769 | 18.834 | 1.00 | 36.52 | O |
| ATOM | 6193 | N | GLY | D | 159 | 23.675 | 7.295 | 14.613 | 1.00 | 36.04 | N |
| ATOM | 6194 | CA | GLY | D | 159 | 23.911 | 7.450 | 13.179 | 1.00 | 36.32 | C |
| ATOM | 6195 | C | GLY | D | 159 | 24.947 | 8.503 | 12.825 | 1.00 | 36.28 | C |
| ATOM | 6196 | O | GLY | D | 159 | 25.399 | 8.565 | 11.686 | 1.00 | 36.62 | O |
| ATOM | 6197 | N | SER | D | 160 | 25.353 | 9.300 | 13.810 | 1.00 | 36.44 | N |
| ATOM | 6198 | CA | SER | D | 160 | 26.358 | 10.354 | 13.627 | 1.00 | 37.25 | C |
| ATOM | 6199 | C | SER | D | 160 | 27.750 | 9.790 | 13.420 | 1.00 | 36.65 | C |
| ATOM | 6200 | O | SER | D | 160 | 28.591 | 10.427 | 12.813 | 1.00 | 37.37 | O |
| ATOM | 6201 | CB | SER | D | 160 | 26.378 | 11.270 | 14.850 | 1.00 | 37.91 | C |
| ATOM | 6202 | OG | SER | D | 160 | 25.089 | 11.823 | 15.050 | 1.00 | 41.41 | O |
| ATOM | 6203 | N | LEU | D | 161 | 27.984 | 8.590 | 13.949 | 1.00 | 35.82 | N |
| ATOM | 6204 | CA | LEU | D | 161 | 29.243 | 7.876 | 13.788 | 1.00 | 35.04 | C |
| ATOM | 6205 | C | LEU | D | 161 | 28.978 | 6.779 | 12.754 | 1.00 | 35.05 | C |
| ATOM | 6206 | O | LEU | D | 161 | 28.418 | 5.749 | 13.085 | 1.00 | 33.92 | O |
| ATOM | 6207 | CB | LEU | D | 161 | 29.664 | 7.295 | 15.149 | 1.00 | 34.72 | C |
| ATOM | 6208 | CG | LEU | D | 161 | 31.140 | 6.991 | 15.428 | 1.00 | 35.86 | C |
| ATOM | 6209 | CD1 | LEU | D | 161 | 31.351 | 6.196 | 16.729 | 1.00 | 34.13 | C |
| ATOM | 6210 | CD2 | LEU | D | 161 | 31.763 | 6.268 | 14.274 | 1.00 | 37.79 | C |
| ATOM | 6211 | N | SER | D | 162 | 29.340 | 7.019 | 11.492 | 1.00 | 34.38 | N |
| ATOM | 6212 | CA | SER | D | 162 | 29.038 | 6.070 | 10.395 | 1.00 | 34.25 | C |
| ATOM | 6213 | C | SER | D | 162 | 30.273 | 5.516 | 9.709 | 1.00 | 33.41 | C |
| ATOM | 6214 | O | SER | D | 162 | 30.224 | 4.421 | 9.138 | 1.00 | 33.75 | O |
| ATOM | 6215 | CB | SER | D | 162 | 28.129 | 6.716 | 9.358 | 1.00 | 34.90 | C |
| ATOM | 6216 | OG | SER | D | 162 | 28.598 | 8.018 | 9.081 | 1.00 | 36.54 | O |
| ATOM | 6217 | N | SER | D | 163 | 31.385 | 6.254 | 9.769 | 1.00 | 32.31 | N |
| ATOM | 6218 | CA | SER | D | 163 | 32.618 | 5.815 | 9.138 | 1.00 | 31.91 | C |
| ATOM | 6219 | C | SER | D | 163 | 33.347 | 4.868 | 10.061 | 1.00 | 29.97 | C |
| ATOM | 6220 | O | SER | D | 163 | 33.232 | 4.966 | 11.284 | 1.00 | 30.41 | O |
| ATOM | 6221 | CB | SER | D | 163 | 33.520 | 7.007 | 8.791 | 1.00 | 33.19 | C |
| ATOM | 6222 | OG | SER | D | 163 | 32.783 | 7.928 | 7.996 | 1.00 | 37.58 | O |
| ATOM | 6223 | N | GLY | D | 164 | 34.076 | 3.944 | 9.451 | 1.00 | 27.93 | N |
| ATOM | 6224 | CA | GLY | D | 164 | 34.825 | 2.934 | 10.164 | 1.00 | 27.34 | C |
| ATOM | 6225 | C | GLY | D | 164 | 33.934 | 1.912 | 10.857 | 1.00 | 25.53 | C |
| ATOM | 6226 | O | GLY | D | 164 | 34.382 | 1.241 | 11.809 | 1.00 | 26.73 | O |
| ATOM | 6227 | N | VAL | D | 165 | 32.691 | 1.796 | 10.407 | 1.00 | 23.24 | N |
| ATOM | 6228 | CA | VAL | D | 165 | 31.745 | 0.811 | 11.002 | 1.00 | 22.59 | C |
| ATOM | 6229 | C | VAL | D | 165 | 31.647 | -0.422 | 10.140 | 1.00 | 22.87 | C |
| ATOM | 6230 | O | VAL | D | 165 | 31.529 | -0.309 | 8.926 | 1.00 | 24.00 | O |

| ATOM | 6231 | CB | VAL | D | 165 | 30.346 | 1.383 | 11.143 | 1.00 | 22.06 | C |
|------|------|-----|-----|---|-----|--------|-------|--------|------|-------|---|
| ATOM | 6232 | CG1 | VAL | D | 165 | 29.338 | 0.280 | 11.538 | 1.00 | 23.00 | C |
| ATOM | 6233 | CG2 | VAL | D | 165 | 30.335 | 2.522 | 12.163 | 1.00 | 22.75 | C |
| ATOM | 6234 | N | HIS | D | 166 | 31.670 | -1.608 | 10.775 | 1.00 | 22.82 | N |
| ATOM | 6235 | CA | HIS | D | 166 | 31.291 | -2.859 | 10.120 | 1.00 | 21.38 | C |
| ATOM | 6236 | C | HIS | D | 166 | 30.218 | -3.572 | 10.905 | 1.00 | 21.28 | C |
| ATOM | 6237 | O | HIS | D | 166 | 30.504 | -4.098 | 11.984 | 1.00 | 20.00 | O |
| ATOM | 6238 | CB | HIS | D | 166 | 32.463 | -3.822 | 10.025 | 1.00 | 20.69 | C |
| ATOM | 6239 | CG | HIS | D | 166 | 33.557 | -3.356 | 9.133 | 1.00 | 21.48 | C |
| ATOM | 6240 | ND1 | HIS | D | 166 | 33.422 | -3.304 | 7.763 | 1.00 | 21.87 | N |
| ATOM | 6241 | CD2 | HIS | D | 166 | 34.809 | -2.950 | 9.409 | 1.00 | 21.37 | C |
| ATOM | 6242 | CE1 | HIS | D | 166 | 34.557 | -2.897 | 7.236 | 1.00 | 20.74 | C |
| ATOM | 6243 | NE2 | HIS | D | 166 | 35.399 | -2.626 | 8.210 | 1.00 | 22.65 | N |
| ATOM | 6244 | N | THR | D | 167 | 29.007 | -3.575 | 10.374 | 1.00 | 19.64 | N |
| ATOM | 6245 | CA | THR | D | 167 | 27.900 | -4.328 | 10.963 | 1.00 | 19.96 | C |
| ATOM | 6246 | C | THR | D | 167 | 27.708 | -5.645 | 10.213 | 1.00 | 18.05 | C |
| ATOM | 6247 | O | THR | D | 167 | 27.380 | -5.681 | 9.033 | 1.00 | 19.63 | O |
| ATOM | 6248 | CB | THR | D | 167 | 26.644 | -3.525 | 11.013 | 1.00 | 20.59 | C |
| ATOM | 6249 | OG1 | THR | D | 167 | 26.819 | -2.400 | 11.884 | 1.00 | 21.75 | O |
| ATOM | 6250 | CG2 | THR | D | 167 | 25.493 | -4.375 | 11.582 | 1.00 | 20.32 | C |
| ATOM | 6251 | N | PHE | D | 168 | 27.945 | -6.751 | 10.906 | 1.00 | 19.23 | N |
| ATOM | 6252 | CA | PHE | D | 168 | 28.041 | -8.034 | 10.252 | 1.00 | 17.91 | C |
| ATOM | 6253 | C | PHE | D | 168 | 26.668 | -8.676 | 10.019 | 1.00 | 18.44 | C |
| ATOM | 6254 | O | PHE | D | 168 | 25.782 | -8.546 | 10.846 | 1.00 | 19.70 | O |
| ATOM | 6255 | CB | PHE | D | 168 | 28.965 | -8.955 | 11.062 | 1.00 | 18.06 | C |
| ATOM | 6256 | CG | PHE | D | 168 | 30.391 | -8.505 | 11.047 | 1.00 | 17.75 | C |
| ATOM | 6257 | CD1 | PHE | D | 168 | 30.870 | -7.575 | 11.969 | 1.00 | 15.97 | C |
| ATOM | 6258 | CD2 | PHE | D | 168 | 31.245 | -8.960 | 10.080 | 1.00 | 17.54 | C |
| ATOM | 6259 | CE1 | PHE | D | 168 | 32.210 | -7.132 | 11.919 | 1.00 | 16.78 | C |
| ATOM | 6260 | CE2 | PHE | D | 168 | 32.528 | -8.560 | 10.026 | 1.00 | 16.82 | C |
| ATOM | 6261 | CZ | PHE | D | 168 | 33.030 | -7.609 | 10.925 | 1.00 | 15.88 | C |
| ATOM | 6262 | N | PRO | D | 169 | 26.513 | -9.409 | 8.907 | 1.00 | 19.50 | N |
| ATOM | 6263 | CA | PRO | D | 169 | 25.275 | -10.108 | 8.701 | 1.00 | 19.88 | C |
| ATOM | 6264 | C | PRO | D | 169 | 24.934 | -11.020 | 9.885 | 1.00 | 18.97 | C |
| ATOM | 6265 | O | PRO | D | 169 | 25.817 | -11.599 | 10.452 | 1.00 | 20.89 | O |
| ATOM | 6266 | CB | PRO | D | 169 | 25.559 | -10.936 | 7.440 | 1.00 | 19.41 | C |
| ATOM | 6267 | CG | PRO | D | 169 | 26.593 | -10.151 | 6.691 | 1.00 | 22.62 | C |
| ATOM | 6268 | CD | PRO | D | 169 | 27.462 | -9.602 | 7.791 | 1.00 | 19.79 | C |
| ATOM | 6269 | N | ALA | D | 170 | 23.654 | -11.140 | 10.235 | 1.00 | 19.44 | N |
| ATOM | 6270 | CA | ALA | D | 170 | 23.257 | -12.062 | 11.304 | 1.00 | 18.66 | C |
| ATOM | 6271 | C | ALA | D | 170 | 23.452 | -13.491 | 10.829 | 1.00 | 18.46 | C |
| ATOM | 6272 | O | ALA | D | 170 | 23.391 | -13.788 | 9.639 | 1.00 | 18.70 | O |
| ATOM | 6273 | CB | ALA | D | 170 | 21.837 | -11.834 | 11.682 | 1.00 | 18.58 | C |
| ATOM | 6274 | N | VAL | D | 171 | 23.714 | -14.374 | 11.780 | 1.00 | 18.51 | N |
| ATOM | 6275 | CA | VAL | D | 171 | 23.875 | -15.792 | 11.533 | 1.00 | 19.48 | C |
| ATOM | 6276 | C | VAL | D | 171 | 22.962 | -16.519 | 12.506 | 1.00 | 19.43 | C |
| ATOM | 6277 | O | VAL | D | 171 | 22.953 | -16.221 | 13.696 | 1.00 | 18.13 | O |
| ATOM | 6278 | CB | VAL | D | 171 | 25.308 | -16.213 | 11.764 | 1.00 | 21.59 | C |
| ATOM | 6279 | CG1 | VAL | D | 171 | 25.481 | -17.745 | 11.681 | 1.00 | 22.58 | C |
| ATOM | 6280 | CG2 | VAL | D | 171 | 26.223 | -15.523 | 10.783 | 1.00 | 20.41 | C |
| ATOM | 6281 | N | LEU | D | 172 | 22.190 | -17.478 | 11.990 | 1.00 | 20.37 | N |
| ATOM | 6282 | CA | LEU | D | 172 | 21.235 | -18.263 | 12.786 | 1.00 | 20.80 | C |
| ATOM | 6283 | C | LEU | D | 172 | 21.873 | -19.557 | 13.272 | 1.00 | 21.79 | C |
| ATOM | 6284 | O | LEU | D | 172 | 22.479 | -20.316 | 12.510 | 1.00 | 21.31 | O |
| ATOM | 6285 | CB | LEU | D | 172 | 19.985 | -18.594 | 11.963 | 1.00 | 21.75 | C |
| ATOM | 6286 | CG | LEU | D | 172 | 18.815 | -19.255 | 12.697 | 1.00 | 21.57 | C |
| ATOM | 6287 | CD1 | LEU | D | 172 | 18.050 | -18.214 | 13.464 | 1.00 | 24.76 | C |
| ATOM | 6288 | CD2 | LEU | D | 172 | 17.915 | -19.996 | 11.671 | 1.00 | 23.10 | C |
| ATOM | 6289 | N | GLN | D | 173 | 21.762 | -19.778 | 14.571 | 1.00 | 22.21 | N |
| ATOM | 6290 | CA | GLN | D | 173 | 22.224 | -20.997 | 15.189 | 1.00 | 23.39 | C |
| ATOM | 6291 | C | GLN | D | 173 | 21.187 | -21.395 | 16.206 | 1.00 | 25.10 | C |
| ATOM | 6292 | O | GLN | D | 173 | 20.828 | -20.623 | 17.092 | 1.00 | 24.84 | O |
| ATOM | 6293 | CB | GLN | D | 173 | 23.572 | -20.816 | 15.905 | 1.00 | 23.61 | C |
| ATOM | 6294 | CG | GLN | D | 173 | 24.739 | -20.596 | 14.970 | 1.00 | 23.10 | C |
| ATOM | 6295 | CD | GLN | D | 173 | 26.113 | -20.727 | 15.638 | 1.00 | 23.40 | C |

| | | | | | | | | | | |
|---|---|---|---|---|---|---|---|---|---|---|
| ATOM | 6296 | OE1 | GLN | D | 173 | 26.245 | -20.600 | 16.854 | 1.00 26.08 | O |
| ATOM | 6297 | NE2 | GLN | D | 173 | 27.133 | -20.930 | 14.828 | 1.00 25.43 | N |
| ATOM | 6298 | N | SER | D | 174 | 20.746 | -22.640 | 16.109 | 1.00 28.14 | N |
| ATOM | 6299 | CA | SER | D | 174 | 19.756 | -23.167 | 17.053 | 1.00 29.43 | C |
| ATOM | 6300 | C | SER | D | 174 | 18.606 | -22.212 | 17.289 | 1.00 29.35 | C |
| ATOM | 6301 | O | SER | D | 174 | 18.258 | -21.902 | 18.436 | 1.00 30.58 | O |
| ATOM | 6302 | CB | SER | D | 174 | 20.434 | -23.523 | 18.364 | 1.00 31.24 | C |
| ATOM | 6303 | OG | SER | D | 174 | 21.382 | -24.554 | 18.126 | 1.00 32.94 | O |
| ATOM | 6304 | N | ASP | D | 175 | 18.016 | -21.750 | 16.186 | 1.00 29.01 | N |
| ATOM | 6305 | CA | ASP | D | 175 | 16.785 | -20.960 | 16.197 | 1.00 28.16 | C |
| ATOM | 6306 | C | ASP | D | 175 | 16.927 | -19.551 | 16.755 | 1.00 24.76 | C |
| ATOM | 6307 | O | ASP | D | 175 | 15.936 | -18.902 | 17.062 | 1.00 22.30 | O |
| ATOM | 6308 | CB | ASP | D | 175 | 15.656 | -21.721 | 16.903 | 1.00 31.87 | C |
| ATOM | 6309 | CG | ASP | D | 175 | 15.441 | -23.106 | 16.313 | 1.00 35.83 | C |
| ATOM | 6310 | OD1 | ASP | D | 175 | 15.059 | -23.173 | 15.129 | 1.00 39.46 | O |
| ATOM | 6311 | OD2 | ASP | D | 175 | 15.634 | -24.121 | 17.039 | 1.00 41.25 | O |
| ATOM | 6312 | N | LEU | D | 176 | 18.169 | -19.088 | 16.894 | 1.00 22.57 | N |
| ATOM | 6313 | CA | LEU | D | 176 | 18.444 | -17.728 | 17.372 | 1.00 20.15 | C |
| ATOM | 6314 | C | LEU | D | 176 | 19.520 | -17.092 | 16.534 | 1.00 19.72 | C |
| ATOM | 6315 | O | LEU | D | 176 | 20.455 | -17.734 | 16.152 | 1.00 17.09 | O |
| ATOM | 6316 | CB | LEU | D | 176 | 18.896 | -17.738 | 18.824 | 1.00 19.76 | C |
| ATOM | 6317 | CG | LEU | D | 176 | 17.829 | -18.026 | 19.870 | 1.00 18.88 | C |
| ATOM | 6318 | CD1 | LEU | D | 176 | 18.487 | -18.038 | 21.202 | 1.00 20.21 | C |
| ATOM | 6319 | CD2 | LEU | D | 176 | 16.691 | -17.047 | 19.892 | 1.00 20.72 | C |
| ATOM | 6320 | N | TYR | D | 177 | 19.371 | -15.801 | 16.259 | 1.00 18.06 | N |
| ATOM | 6321 | CA | TYR | D | 177 | 20.373 | -15.055 | 15.526 | 1.00 19.25 | C |
| ATOM | 6322 | C | TYR | D | 177 | 21.309 | -14.349 | 16.475 | 1.00 18.61 | C |
| ATOM | 6323 | O | TYR | D | 177 | 20.927 | -13.950 | 17.579 | 1.00 18.95 | O |
| ATOM | 6324 | CB | TYR | D | 177 | 19.751 | -13.946 | 14.676 | 1.00 20.11 | C |
| ATOM | 6325 | CG | TYR | D | 177 | 18.990 | -14.404 | 13.451 | 1.00 23.06 | C |
| ATOM | 6326 | CD1 | TYR | D | 177 | 19.648 | -14.768 | 12.296 | 1.00 23.07 | C |
| ATOM | 6327 | CD2 | TYR | D | 177 | 17.601 | -14.502 | 13.483 | 1.00 23.99 | C |
| ATOM | 6328 | CE1 | TYR | D | 177 | 18.937 | -15.200 | 11.171 | 1.00 24.59 | C |
| ATOM | 6329 | CE2 | TYR | D | 177 | 16.889 | -14.915 | 12.396 | 1.00 24.54 | C |
| ATOM | 6330 | CZ | TYR | D | 177 | 17.545 | -15.263 | 11.247 | 1.00 27.80 | C |
| ATOM | 6331 | OH | TYR | D | 177 | 16.801 | -15.673 | 10.179 | 1.00 26.78 | O |
| ATOM | 6332 | N | THR | D | 178 | 22.523 | -14.123 | 15.968 | 1.00 19.66 | N |
| ATOM | 6333 | CA | THR | D | 178 | 23.496 | -13.256 | 16.582 | 1.00 18.58 | C |
| ATOM | 6334 | C | THR | D | 178 | 24.129 | -12.404 | 15.513 | 1.00 17.84 | C |
| ATOM | 6335 | O | THR | D | 178 | 24.385 | -12.825 | 14.406 | 1.00 16.92 | O |
| ATOM | 6336 | CB | THR | D | 178 | 24.600 | -14.037 | 17.297 | 1.00 20.20 | C |
| ATOM | 6337 | OG1 | THR | D | 178 | 23.991 | -14.816 | 18.328 | 1.00 23.51 | O |
| ATOM | 6338 | CG2 | THR | D | 178 | 25.645 | -13.104 | 17.929 | 1.00 20.73 | C |
| ATOM | 6339 | N | LEU | D | 179 | 24.364 | -11.163 | 15.873 | 1.00 17.95 | N |
| ATOM | 6340 | CA | LEU | D | 179 | 25.212 | -10.348 | 15.036 | 1.00 20.07 | C |
| ATOM | 6341 | C | LEU | D | 179 | 26.084 | -9.479 | 15.859 | 1.00 18.67 | C |
| ATOM | 6342 | O | LEU | D | 179 | 25.976 | -9.430 | 17.067 | 1.00 17.30 | O |
| ATOM | 6343 | CB | LEU | D | 179 | 24.410 | -9.541 | 14.030 | 1.00 22.71 | C |
| ATOM | 6344 | CG | LEU | D | 179 | 23.602 | -8.285 | 14.317 | 1.00 23.57 | C |
| ATOM | 6345 | CD1 | LEU | D | 179 | 24.376 | -7.032 | 14.779 | 1.00 23.25 | C |
| ATOM | 6346 | CD2 | LEU | D | 179 | 22.830 | -7.937 | 13.029 | 1.00 24.54 | C |
| ATOM | 6347 | N | SER | D | 180 | 26.984 | -8.792 | 15.170 | 1.00 17.60 | N |
| ATOM | 6348 | CA | SER | D | 180 | 27.918 | -7.945 | 15.845 | 1.00 16.01 | C |
| ATOM | 6349 | C | SER | D | 180 | 28.218 | -6.767 | 14.956 | 1.00 16.05 | C |
| ATOM | 6350 | O | SER | D | 180 | 27.957 | -6.796 | 13.752 | 1.00 16.55 | O |
| ATOM | 6351 | CB | SER | D | 180 | 29.206 | -8.683 | 16.196 | 1.00 17.94 | C |
| ATOM | 6352 | OG | SER | D | 180 | 29.785 | -9.350 | 15.064 | 1.00 17.22 | O |
| ATOM | 6353 | N | SER | D | 181 | 28.777 | -5.750 | 15.580 | 1.00 17.36 | N |
| ATOM | 6354 | CA | SER | D | 181 | 29.174 | -4.522 | 14.849 | 1.00 18.01 | C |
| ATOM | 6355 | C | SER | D | 181 | 30.408 | -3.954 | 15.480 | 1.00 17.81 | C |
| ATOM | 6356 | O | SER | D | 181 | 30.485 | -3.867 | 16.724 | 1.00 19.25 | O |
| ATOM | 6357 | CB | SER | D | 181 | 28.072 | -3.473 | 14.864 | 1.00 19.37 | C |
| ATOM | 6358 | OG | SER | D | 181 | 28.522 | -2.295 | 14.175 | 1.00 22.27 | O |
| ATOM | 6359 | N | SER | D | 182 | 31.393 | -3.604 | 14.640 | 1.00 18.27 | N |
| ATOM | 6360 | CA | SER | D | 182 | 32.627 | -2.985 | 15.100 | 1.00 17.88 | C |

| ATOM | 6361 | C   | SER | D | 182 | 32.632 | -1.555 | 14.627 | 1.00 | 18.80 | C |
| ATOM | 6362 | O   | SER | D | 182 | 32.091 | -1.257 | 13.565 | 1.00 | 20.43 | O |
| ATOM | 6363 | CB  | SER | D | 182 | 33.883 | -3.659 | 14.578 | 1.00 | 17.47 | C |
| ATOM | 6364 | OG  | SER | D | 182 | 34.084 | -3.522 | 13.201 | 1.00 | 19.13 | O |
| ATOM | 6365 | N   | VAL | D | 183 | 33.252 | -0.710 | 15.425 | 1.00 | 20.94 | N |
| ATOM | 6366 | CA  | VAL | D | 183 | 33.454 | 0.692  | 15.043 | 1.00 | 20.94 | C |
| ATOM | 6367 | C   | VAL | D | 183 | 34.874 | 1.040  | 15.393 | 1.00 | 21.42 | C |
| ATOM | 6368 | O   | VAL | D | 183 | 35.354 | 0.671  | 16.444 | 1.00 | 19.65 | O |
| ATOM | 6369 | CB  | VAL | D | 183 | 32.431 | 1.632  | 15.671 | 1.00 | 21.53 | C |
| ATOM | 6370 | CG1 | VAL | D | 183 | 32.553 | 1.686  | 17.204 | 1.00 | 23.46 | C |
| ATOM | 6371 | CG2 | VAL | D | 183 | 32.631 | 3.026  | 15.108 | 1.00 | 23.28 | C |
| ATOM | 6372 | N   | THR | D | 184 | 35.563 | 1.750  | 14.502 | 1.00 | 23.00 | N |
| ATOM | 6373 | CA  | THR | D | 184 | 36.962 | 2.054  | 14.714 | 1.00 | 22.81 | C |
| ATOM | 6374 | C   | THR | D | 184 | 37.096 | 3.570  | 14.672 | 1.00 | 23.86 | C |
| ATOM | 6375 | O   | THR | D | 184 | 36.561 | 4.209  | 13.772 | 1.00 | 24.61 | O |
| ATOM | 6376 | CB  | THR | D | 184 | 37.875 | 1.380  | 13.669 | 1.00 | 25.26 | C |
| ATOM | 6377 | OG1 | THR | D | 184 | 37.752 | -0.054 | 13.751 | 1.00 | 23.97 | O |
| ATOM | 6378 | CG2 | THR | D | 184 | 39.290 | 1.743  | 13.932 | 1.00 | 24.54 | C |
| ATOM | 6379 | N   | VAL | D | 185 | 37.768 | 4.096  | 15.688 | 1.00 | 23.88 | N |
| ATOM | 6380 | CA  | VAL | D | 185 | 37.954 | 5.539  | 15.888 | 1.00 | 26.13 | C |
| ATOM | 6381 | C   | VAL | D | 185 | 39.415 | 5.831  | 16.246 | 1.00 | 26.76 | C |
| ATOM | 6382 | O   | VAL | D | 185 | 40.189 | 4.927  | 16.595 | 1.00 | 24.53 | O |
| ATOM | 6383 | CB  | VAL | D | 185 | 37.007 | 6.109  | 16.986 | 1.00 | 26.53 | C |
| ATOM | 6384 | CG1 | VAL | D | 185 | 35.556 | 5.886  | 16.599 | 1.00 | 27.34 | C |
| ATOM | 6385 | CG2 | VAL | D | 185 | 37.311 | 5.526  | 18.368 | 1.00 | 25.35 | C |
| ATOM | 6386 | N   | PRO | D | 186 | 39.830 | 7.119  | 16.137 | 1.00 | 26.97 | N |
| ATOM | 6387 | CA  | PRO | D | 186 | 41.167 | 7.472  | 16.616 | 1.00 | 27.92 | C |
| ATOM | 6388 | C   | PRO | D | 186 | 41.319 | 7.204  | 18.101 | 1.00 | 27.13 | C |
| ATOM | 6389 | O   | PRO | D | 186 | 40.388 | 7.450  | 18.860 | 1.00 | 28.17 | O |
| ATOM | 6390 | CB  | PRO | D | 186 | 41.248 | 8.995  | 16.370 | 1.00 | 27.58 | C |
| ATOM | 6391 | CG  | PRO | D | 186 | 40.182 | 9.297  | 15.405 | 1.00 | 28.55 | C |
| ATOM | 6392 | CD  | PRO | D | 186 | 39.096 | 8.273  | 15.588 | 1.00 | 28.51 | C |
| ATOM | 6393 | N   | SER | D | 187 | 42.497 | 6.725  | 18.505 | 1.00 | 30.53 | N |
| ATOM | 6394 | CA  | SER | D | 187 | 42.732 | 6.353  | 19.897 | 1.00 | 31.59 | C |
| ATOM | 6395 | C   | SER | D | 187 | 42.411 | 7.479  | 20.859 | 1.00 | 31.64 | C |
| ATOM | 6396 | O   | SER | D | 187 | 41.816 | 7.256  | 21.917 | 1.00 | 30.63 | O |
| ATOM | 6397 | CB  | SER | D | 187 | 44.175 | 5.906  | 20.086 | 1.00 | 33.08 | C |
| ATOM | 6398 | OG  | SER | D | 187 | 44.394 | 4.737  | 19.328 | 1.00 | 37.72 | O |
| ATOM | 6399 | N   | SER | D | 188 | 42.788 | 8.710  | 20.492 | 1.00 | 30.27 | N |
| ATOM | 6400 | CA  | SER | D | 188 | 42.583 | 9.837  | 21.394 | 1.00 | 28.90 | C |
| ATOM | 6401 | C   | SER | D | 188 | 41.122 | 10.185 | 21.637 | 1.00 | 28.81 | C |
| ATOM | 6402 | O   | SER | D | 188 | 40.815 | 10.895 | 22.592 | 1.00 | 27.74 | O |
| ATOM | 6403 | CB  | SER | D | 188 | 43.310 | 11.078 | 20.845 | 1.00 | 28.70 | C |
| ATOM | 6404 | OG  | SER | D | 188 | 42.812 | 11.416 | 19.554 | 1.00 | 26.04 | O |
| ATOM | 6405 | N   | THR | D | 189 | 40.225 | 9.704  | 20.780 | 1.00 | 28.44 | N |
| ATOM | 6406 | CA  | THR | D | 189 | 38.814 | 10.090 | 20.840 | 1.00 | 30.62 | C |
| ATOM | 6407 | C   | THR | D | 189 | 38.024 | 9.280  | 21.866 | 1.00 | 30.70 | C |
| ATOM | 6408 | O   | THR | D | 189 | 37.030 | 9.764  | 22.388 | 1.00 | 31.43 | O |
| ATOM | 6409 | CB  | THR | D | 189 | 38.090 | 9.989  | 19.471 | 1.00 | 32.00 | C |
| ATOM | 6410 | OG1 | THR | D | 189 | 37.988 | 8.624  | 19.061 | 1.00 | 35.92 | O |
| ATOM | 6411 | CG2 | THR | D | 189 | 38.820 | 10.779 | 18.411 | 1.00 | 32.76 | C |
| ATOM | 6412 | N   | TRP | D | 190 | 38.456 | 8.050  | 22.143 | 1.00 | 29.75 | N |
| ATOM | 6413 | CA  | TRP | D | 190 | 37.777 | 7.248  | 23.130 | 1.00 | 29.67 | C |
| ATOM | 6414 | C   | TRP | D | 190 | 38.686 | 6.957  | 24.302 | 1.00 | 30.89 | C |
| ATOM | 6415 | O   | TRP | D | 190 | 39.835 | 6.579  | 24.102 | 1.00 | 31.93 | O |
| ATOM | 6416 | CB  | TRP | D | 190 | 37.281 | 5.940  | 22.498 | 1.00 | 29.13 | C |
| ATOM | 6417 | CG  | TRP | D | 190 | 36.500 | 5.098  | 23.467 | 1.00 | 26.67 | C |
| ATOM | 6418 | CD1 | TRP | D | 190 | 35.194 | 5.247  | 23.802 | 1.00 | 26.81 | C |
| ATOM | 6419 | CD2 | TRP | D | 190 | 36.983 | 3.974  | 24.203 | 1.00 | 25.09 | C |
| ATOM | 6420 | NE1 | TRP | D | 190 | 34.823 | 4.281  | 24.713 | 1.00 | 26.23 | N |
| ATOM | 6421 | CE2 | TRP | D | 190 | 35.906 | 3.494  | 24.983 | 1.00 | 24.22 | C |
| ATOM | 6422 | CE3 | TRP | D | 190 | 38.225 | 3.338  | 24.303 | 1.00 | 25.89 | C |
| ATOM | 6423 | CZ2 | TRP | D | 190 | 36.025 | 2.393  | 25.832 | 1.00 | 26.55 | C |
| ATOM | 6424 | CZ3 | TRP | D | 190 | 38.339 | 2.238  | 25.159 | 1.00 | 26.36 | C |
| ATOM | 6425 | CH2 | TRP | D | 190 | 37.253 | 1.790  | 25.916 | 1.00 | 25.17 | C |

```
ATOM    6426  N    PRO D 191      38.158    7.055   25.533  1.00 32.49           N
ATOM    6427  CA   PRO D 191      36.801    7.369   25.985  1.00 33.27           C
ATOM    6428  C    PRO D 191      36.400    8.833   26.118  1.00 34.78           C
ATOM    6429  O    PRO D 191      35.317    9.124   26.618  1.00 34.97           O
ATOM    6430  CB   PRO D 191      36.747    6.702   27.359  1.00 33.41           C
ATOM    6431  CG   PRO D 191      38.132    6.686   27.825  1.00 33.51           C
ATOM    6432  CD   PRO D 191      39.028    6.662   26.651  1.00 33.13           C
ATOM    6433  N    SER D 192      37.243    9.748   25.669  1.00 35.91           N
ATOM    6434  CA   SER D 192      36.961   11.146   25.858  1.00 35.91           C
ATOM    6435  C    SER D 192      35.675   11.700   25.235  1.00 36.12           C
ATOM    6436  O    SER D 192      34.876   12.336   25.934  1.00 37.46           O
ATOM    6437  CB   SER D 192      38.096   11.986   25.203  1.00 36.31           C
ATOM    6438  OG   SER D 192      39.383   11.677   25.743  1.00 38.45           O
ATOM    6439  N    GLU D 193      35.421   11.360   23.967  1.00 35.08           N
ATOM    6440  CA   GLU D 193      34.020   11.151   23.505  1.00 34.16           C
ATOM    6441  C    GLU D 193      33.307    9.849   23.808  1.00 33.07           C
ATOM    6442  O    GLU D 193      33.938    8.809   24.019  1.00 33.43           O
ATOM    6443  CB   GLU D 193      34.030   11.419   21.982  1.00 35.48           C
ATOM    6444  CG   GLU D 193      34.761   12.730   21.575  1.00 37.08           C
ATOM    6445  CD   GLU D 193      34.751   12.986   20.084  1.00 38.56           C
ATOM    6446  OE1  GLU D 193      35.806   13.347   19.525  1.00 41.07           O
ATOM    6447  OE2  GLU D 193      33.694   12.831   19.448  1.00 42.27           O
ATOM    6448  N    THR D 194      31.989    9.961   23.867  1.00 31.72           N
ATOM    6449  CA   THR D 194      31.134    8.807   24.136  1.00 31.44           C
ATOM    6450  C    THR D 194      30.929    8.048   22.855  1.00 29.73           C
ATOM    6451  O    THR D 194      30.769    8.647   21.783  1.00 28.41           O
ATOM    6452  CB   THR D 194      29.779    9.236   24.720  1.00 31.47           C
ATOM    6453  OG1  THR D 194      29.165   10.171   23.828  1.00 33.46           O
ATOM    6454  CG2  THR D 194      30.002    9.898   26.085  1.00 32.04           C
ATOM    6455  N    VAL D 195      30.949    6.718   22.971  1.00 27.70           N
ATOM    6456  CA   VAL D 195      30.687    5.831   21.842  1.00 24.97           C
ATOM    6457  C    VAL D 195      29.609    4.876   22.328  1.00 23.86           C
ATOM    6458  O    VAL D 195      29.819    4.136   23.280  1.00 25.14           O
ATOM    6459  CB   VAL D 195      31.955    5.064   21.395  1.00 24.95           C
ATOM    6460  CG1  VAL D 195      31.615    3.981   20.363  1.00 25.27           C
ATOM    6461  CG2  VAL D 195      32.971    6.028   20.789  1.00 26.39           C
ATOM    6462  N    THR D 196      28.476    4.935   21.660  1.00 23.76           N
ATOM    6463  CA   THR D 196      27.281    4.215   22.042  1.00 24.12           C
ATOM    6464  C    THR D 196      26.666    3.500   20.869  1.00 24.51           C
ATOM    6465  O    THR D 196      26.386    4.105   19.846  1.00 24.49           O
ATOM    6466  CB   THR D 196      26.250    5.219   22.570  1.00 23.02           C
ATOM    6467  OG1  THR D 196      26.792    5.891   23.717  1.00 22.80           O
ATOM    6468  CG2  THR D 196      24.975    4.518   22.936  1.00 22.05           C
ATOM    6469  N    CYS D 197      26.382    2.208   21.033  1.00 23.96           N
ATOM    6470  CA   ACYS D 197     25.680    1.482   19.977  0.50 23.14           C
ATOM    6471  CA   BCYS D 197     25.711    1.412   20.020  0.50 24.92           C
ATOM    6472  C    CYS D 197      24.179    1.438   20.268  1.00 24.27           C
ATOM    6473  O    CYS D 197      23.740    1.202   21.400  1.00 26.07           O
ATOM    6474  CB   ACYS D 197     26.254    0.085   19.752  0.50 21.39           C
ATOM    6475  CB   BCYS D 197     26.311   -0.013   20.057  0.50 24.53           C
ATOM    6476  SG   ACYS D 197     25.815   -1.140   20.978  0.50 17.90           S
ATOM    6477  SG   BCYS D 197     25.257   -1.328   19.455  0.50 28.27           S
ATOM    6478  N    ASN D 198      23.390    1.742   19.242  1.00 24.06           N
ATOM    6479  CA   ASN D 198      21.937    1.802   19.353  1.00 24.85           C
ATOM    6480  C    ASN D 198      21.340    0.632   18.620  1.00 24.96           C
ATOM    6481  O    ASN D 198      21.528    0.493   17.411  1.00 25.33           O
ATOM    6482  CB   ASN D 198      21.398    3.092   18.739  1.00 25.62           C
ATOM    6483  CG   ASN D 198      22.254    4.281   19.049  1.00 23.81           C
ATOM    6484  OD1  ASN D 198      22.885    4.836   18.158  1.00 28.69           O
ATOM    6485  ND2  ASN D 198      22.279    4.686   20.311  1.00 27.94           N
ATOM    6486  N    VAL D 199      20.605   -0.201   19.344  1.00 24.40           N
ATOM    6487  CA   VAL D 199      20.097   -1.453   18.810  1.00 24.09           C
ATOM    6488  C    VAL D 199      18.602   -1.570   19.047  1.00 25.06           C
ATOM    6489  O    VAL D 199      18.126   -1.495   20.188  1.00 27.10           O
ATOM    6490  CB   VAL D 199      20.813   -2.641   19.453  1.00 23.54           C
```

| ATOM | 6491 | CG1 | VAL | D | 199 | 20.385 | -3.960 | 18.770 | 1.00 | 22.36 | C |
|------|------|-----|-----|---|-----|--------|--------|--------|------|-------|---|
| ATOM | 6492 | CG2 | VAL | D | 199 | 22.311 | -2.409 | 19.371 | 1.00 | 21.41 | C |
| ATOM | 6493 | N | ALA | D | 200 | 17.887 | -1.766 | 17.955 | 1.00 | 25.65 | N |
| ATOM | 6494 | CA | ALA | D | 200 | 16.448 | -1.880 | 17.963 | 1.00 | 26.45 | C |
| ATOM | 6495 | C | ALA | D | 200 | 16.068 | -3.184 | 17.323 | 1.00 | 26.00 | C |
| ATOM | 6496 | O | ALA | D | 200 | 16.624 | -3.572 | 16.297 | 1.00 | 25.25 | O |
| ATOM | 6497 | CB | ALA | D | 200 | 15.799 | -0.714 | 17.204 | 1.00 | 27.22 | C |
| ATOM | 6498 | N | HIS | D | 201 | 15.125 | -3.857 | 17.956 | 1.00 | 26.66 | N |
| ATOM | 6499 | CA | HIS | D | 201 | 14.542 | -5.080 | 17.435 | 1.00 | 26.60 | C |
| ATOM | 6500 | C | HIS | D | 201 | 13.019 | -4.920 | 17.538 | 1.00 | 29.28 | C |
| ATOM | 6501 | O | HIS | D | 201 | 12.427 | -5.293 | 18.540 | 1.00 | 28.47 | O |
| ATOM | 6502 | CB | HIS | D | 201 | 15.001 | -6.238 | 18.289 | 1.00 | 26.07 | C |
| ATOM | 6503 | CG | HIS | D | 201 | 14.535 | -7.569 | 17.809 | 1.00 | 23.40 | C |
| ATOM | 6504 | ND1 | HIS | D | 201 | 13.874 | -8.466 | 18.627 | 1.00 | 22.58 | N |
| ATOM | 6505 | CD2 | HIS | D | 201 | 14.637 | -8.161 | 16.599 | 1.00 | 23.18 | C |
| ATOM | 6506 | CE1 | HIS | D | 201 | 13.591 | -9.554 | 17.931 | 1.00 | 20.47 | C |
| ATOM | 6507 | NE2 | HIS | D | 201 | 14.049 | -9.399 | 16.702 | 1.00 | 21.63 | N |
| ATOM | 6508 | N | PRO | D | 202 | 12.397 | -4.313 | 16.514 | 1.00 | 31.99 | N |
| ATOM | 6509 | CA | PRO | D | 202 | 10.959 | -4.112 | 16.453 | 1.00 | 33.02 | C |
| ATOM | 6510 | C | PRO | D | 202 | 10.132 | -5.323 | 16.877 | 1.00 | 32.78 | C |
| ATOM | 6511 | O | PRO | D | 202 | 9.244 | -5.192 | 17.708 | 1.00 | 33.03 | O |
| ATOM | 6512 | CB | PRO | D | 202 | 10.737 | -3.813 | 14.980 | 1.00 | 33.68 | C |
| ATOM | 6513 | CG | PRO | D | 202 | 11.933 | -3.070 | 14.591 | 1.00 | 33.14 | C |
| ATOM | 6514 | CD | PRO | D | 202 | 13.063 | -3.713 | 15.340 | 1.00 | 32.32 | C |
| ATOM | 6515 | N | ALA | D | 203 | 10.442 | -6.498 | 16.348 | 1.00 | 32.01 | N |
| ATOM | 6516 | CA | ALA | D | 203 | 9.577 | -7.649 | 16.546 | 1.00 | 32.45 | C |
| ATOM | 6517 | C | ALA | D | 203 | 9.372 | -8.032 | 18.009 | 1.00 | 32.38 | C |
| ATOM | 6518 | O | ALA | D | 203 | 8.381 | -8.687 | 18.328 | 1.00 | 34.94 | O |
| ATOM | 6519 | CB | ALA | D | 203 | 10.086 | -8.828 | 15.785 | 1.00 | 31.91 | C |
| ATOM | 6520 | N | SER | D | 204 | 10.313 | -7.651 | 18.872 | 1.00 | 31.56 | N |
| ATOM | 6521 | CA | SER | D | 204 | 10.245 | -7.910 | 20.318 | 1.00 | 31.39 | C |
| ATOM | 6522 | C | SER | D | 204 | 10.007 | -6.639 | 21.117 | 1.00 | 32.00 | C |
| ATOM | 6523 | O | SER | D | 204 | 9.957 | -6.680 | 22.344 | 1.00 | 32.31 | O |
| ATOM | 6524 | CB | SER | D | 204 | 11.542 | -8.578 | 20.810 | 1.00 | 31.39 | C |
| ATOM | 6525 | OG | SER | D | 204 | 12.645 | -7.669 | 20.869 | 1.00 | 29.09 | O |
| ATOM | 6526 | N | SER | D | 205 | 9.864 | -5.516 | 20.430 | 1.00 | 34.26 | N |
| ATOM | 6527 | CA | SER | D | 205 | 9.687 | -4.209 | 21.077 | 1.00 | 36.45 | C |
| ATOM | 6528 | C | SER | D | 205 | 10.903 | -3.757 | 21.876 | 1.00 | 37.46 | C |
| ATOM | 6529 | O | SER | D | 205 | 10.764 | -3.075 | 22.901 | 1.00 | 38.22 | O |
| ATOM | 6530 | CB | SER | D | 205 | 8.432 | -4.192 | 21.962 | 1.00 | 37.66 | C |
| ATOM | 6531 | OG | SER | D | 205 | 7.300 | -4.581 | 21.200 | 1.00 | 40.23 | O |
| ATOM | 6532 | N | THR | D | 206 | 12.085 | -4.127 | 21.384 | 1.00 | 36.68 | N |
| ATOM | 6533 | CA | THR | D | 206 | 13.352 | -3.850 | 22.046 | 1.00 | 37.24 | C |
| ATOM | 6534 | C | THR | D | 206 | 14.059 | -2.604 | 21.506 | 1.00 | 35.76 | C |
| ATOM | 6535 | O | THR | D | 206 | 14.199 | -2.430 | 20.295 | 1.00 | 35.26 | O |
| ATOM | 6536 | CB | THR | D | 206 | 14.334 | -5.009 | 21.801 | 1.00 | 37.29 | C |
| ATOM | 6537 | OG1 | THR | D | 206 | 13.883 | -6.167 | 22.483 | 1.00 | 39.82 | O |
| ATOM | 6538 | CG2 | THR | D | 206 | 15.764 | -4.652 | 22.252 | 1.00 | 40.16 | C |
| ATOM | 6539 | N | LYS | D | 207 | 14.563 | -1.778 | 22.412 | 1.00 | 35.38 | N |
| ATOM | 6540 | CA | LYS | D | 207 | 15.382 | -0.636 | 22.016 | 1.00 | 35.71 | C |
| ATOM | 6541 | C | LYS | D | 207 | 16.446 | -0.444 | 23.100 | 1.00 | 34.65 | C |
| ATOM | 6542 | O | LYS | D | 207 | 16.102 | -0.234 | 24.264 | 1.00 | 34.90 | O |
| ATOM | 6543 | CB | LYS | D | 207 | 14.505 | 0.605 | 21.857 | 1.00 | 36.91 | C |
| ATOM | 6544 | CG | LYS | D | 207 | 14.760 | 1.439 | 20.602 | 1.00 | 38.88 | C |
| ATOM | 6545 | CD | LYS | D | 207 | 16.225 | 1.838 | 20.400 | 1.00 | 40.50 | C |
| ATOM | 6546 | CE | LYS | D | 207 | 16.414 | 2.531 | 19.037 | 1.00 | 41.61 | C |
| ATOM | 6547 | NZ | LYS | D | 207 | 17.748 | 2.292 | 18.396 | 1.00 | 40.75 | N |
| ATOM | 6548 | N | VAL | D | 208 | 17.722 | -0.550 | 22.736 | 1.00 | 32.19 | N |
| ATOM | 6549 | CA | VAL | D | 208 | 18.822 | -0.427 | 23.702 | 1.00 | 32.07 | C |
| ATOM | 6550 | C | VAL | D | 208 | 19.885 | 0.564 | 23.221 | 1.00 | 29.72 | C |
| ATOM | 6551 | O | VAL | D | 208 | 20.266 | 0.534 | 22.065 | 1.00 | 28.73 | O |
| ATOM | 6552 | CB | VAL | D | 208 | 19.508 | -1.816 | 23.930 | 1.00 | 32.88 | C |
| ATOM | 6553 | CG1 | VAL | D | 208 | 20.689 | -1.707 | 24.900 | 1.00 | 35.23 | C |
| ATOM | 6554 | CG2 | VAL | D | 208 | 18.500 | -2.809 | 24.431 | 1.00 | 35.55 | C |
| ATOM | 6555 | N | ASP | D | 209 | 20.394 | 1.407 | 24.120 | 1.00 | 28.77 | N |

| ATOM | 6556 | CA | ASP | D | 209 | 21.604 | 2.171 | 23.841 | 1.00 | 28.15 | C |
|------|------|-----|-----|---|-----|--------|--------|--------|------|-------|---|
| ATOM | 6557 | C | ASP | D | 209 | 22.683 | 1.710 | 24.780 | 1.00 | 27.22 | C |
| ATOM | 6558 | O | ASP | D | 209 | 22.581 | 1.883 | 25.995 | 1.00 | 28.91 | O |
| ATOM | 6559 | CB | ASP | D | 209 | 21.355 | 3.669 | 23.996 | 1.00 | 29.61 | C |
| ATOM | 6560 | CG | ASP | D | 209 | 20.235 | 4.144 | 23.127 | 1.00 | 31.52 | C |
| ATOM | 6561 | OD1 | ASP | D | 209 | 20.240 | 3.860 | 21.902 | 1.00 | 30.80 | O |
| ATOM | 6562 | OD2 | ASP | D | 209 | 19.315 | 4.793 | 23.688 | 1.00 | 37.35 | O |
| ATOM | 6563 | N | LYS | D | 210 | 23.707 | 1.067 | 24.249 | 1.00 | 25.93 | N |
| ATOM | 6564 | CA | LYS | D | 210 | 24.789 | 0.590 | 25.086 | 1.00 | 24.35 | C |
| ATOM | 6565 | C | LYS | D | 210 | 26.032 | 1.462 | 24.889 | 1.00 | 24.64 | C |
| ATOM | 6566 | O | LYS | D | 210 | 26.592 | 1.498 | 23.823 | 1.00 | 23.73 | O |
| ATOM | 6567 | CB | LYS | D | 210 | 25.085 | -0.890 | 24.769 | 1.00 | 24.98 | C |
| ATOM | 6568 | CG | LYS | D | 210 | 26.202 | -1.533 | 25.603 | 1.00 | 25.97 | C |
| ATOM | 6569 | CD | LYS | D | 210 | 25.989 | -1.571 | 27.121 | 1.00 | 26.00 | C |
| ATOM | 6570 | CE | LYS | D | 210 | 24.676 | -2.194 | 27.576 | 1.00 | 25.39 | C |
| ATOM | 6571 | NZ | LYS | D | 210 | 24.707 | -2.280 | 29.102 | 1.00 | 26.85 | N |
| ATOM | 6572 | N | LYS | D | 211 | 26.400 | 2.198 | 25.929 | 1.00 | 26.31 | N |
| ATOM | 6573 | CA | LYS | D | 211 | 27.596 | 3.027 | 25.928 | 1.00 | 26.07 | C |
| ATOM | 6574 | C | LYS | D | 211 | 28.784 | 2.121 | 26.205 | 1.00 | 27.00 | C |
| ATOM | 6575 | O | LYS | D | 211 | 28.721 | 1.279 | 27.101 | 1.00 | 28.68 | O |
| ATOM | 6576 | CB | LYS | D | 211 | 27.460 | 4.103 | 27.017 | 1.00 | 27.43 | C |
| ATOM | 6577 | CG | LYS | D | 211 | 28.657 | 4.979 | 27.189 | 1.00 | 28.09 | C |
| ATOM | 6578 | CD | LYS | D | 211 | 28.392 | 6.018 | 28.314 | 1.00 | 28.37 | C |
| ATOM | 6579 | CE | LYS | D | 211 | 29.304 | 7.231 | 28.171 | 1.00 | 32.23 | C |
| ATOM | 6580 | NZ | LYS | D | 211 | 29.115 | 8.278 | 29.266 | 1.00 | 30.09 | N |
| ATOM | 6581 | N | ILE | D | 212 | 29.837 | 2.237 | 25.403 | 1.00 | 26.11 | N |
| ATOM | 6582 | CA | ILE | D | 212 | 31.033 | 1.442 | 25.596 | 1.00 | 25.85 | C |
| ATOM | 6583 | C | ILE | D | 212 | 31.953 | 2.187 | 26.565 | 1.00 | 27.45 | C |
| ATOM | 6584 | O | ILE | D | 212 | 32.399 | 3.308 | 26.277 | 1.00 | 27.62 | O |
| ATOM | 6585 | CB | ILE | D | 212 | 31.803 | 1.173 | 24.274 | 1.00 | 26.22 | C |
| ATOM | 6586 | CG1 | ILE | D | 212 | 30.877 | 0.695 | 23.142 | 1.00 | 25.11 | C |
| ATOM | 6587 | CG2 | ILE | D | 212 | 32.939 | 0.187 | 24.501 | 1.00 | 25.73 | C |
| ATOM | 6588 | CD1 | ILE | D | 212 | 30.040 | -0.517 | 23.475 | 1.00 | 24.30 | C |
| ATOM | 6589 | N | VAL | D | 213 | 32.248 | 1.565 | 27.693 | 1.00 | 26.94 | N |
| ATOM | 6590 | CA | VAL | D | 213 | 33.089 | 2.194 | 28.695 | 1.00 | 27.86 | C |
| ATOM | 6591 | C | VAL | D | 213 | 34.325 | 1.365 | 28.942 | 1.00 | 28.66 | C |
| ATOM | 6592 | O | VAL | D | 213 | 34.323 | 0.135 | 28.793 | 1.00 | 27.44 | O |
| ATOM | 6593 | CB | VAL | D | 213 | 32.329 | 2.459 | 30.005 | 1.00 | 28.04 | C |
| ATOM | 6594 | CG1 | VAL | D | 213 | 31.016 | 3.198 | 29.714 | 1.00 | 28.64 | C |
| ATOM | 6595 | CG2 | VAL | D | 213 | 32.088 | 1.182 | 30.783 | 1.00 | 28.74 | C |
| ATOM | 6596 | N | PRO | D | 214 | 35.423 | 2.025 | 29.287 | 1.00 | 28.70 | N |
| ATOM | 6597 | CA | PRO | D | 214 | 36.616 | 1.279 | 29.572 | 1.00 | 28.98 | C |
| ATOM | 6598 | C | PRO | D | 214 | 36.403 | 0.240 | 30.662 | 1.00 | 29.65 | C |
| ATOM | 6599 | O | PRO | D | 214 | 35.659 | 0.483 | 31.582 | 1.00 | 29.03 | O |
| ATOM | 6600 | CB | PRO | D | 214 | 37.588 | 2.371 | 30.058 | 1.00 | 29.51 | C |
| ATOM | 6601 | CG | PRO | D | 214 | 37.093 | 3.599 | 29.440 | 1.00 | 28.91 | C |
| ATOM | 6602 | CD | PRO | D | 214 | 35.629 | 3.484 | 29.403 | 1.00 | 29.29 | C |
| ATOM | 6603 | N | ARG | D | 215 | 37.053 | -0.916 | 30.551 | 1.00 | 30.73 | N |
| ATOM | 6604 | CA | ARG | D | 215 | 36.991 | -1.905 | 31.625 | 1.00 | 31.61 | C |
| ATOM | 6605 | C | ARG | D | 215 | 37.882 | -1.434 | 32.779 | 1.00 | 32.18 | C |
| ATOM | 6606 | O | ARG | D | 215 | 39.057 | -1.142 | 32.548 | 1.00 | 33.98 | O |
| ATOM | 6607 | CB | ARG | D | 215 | 37.455 | -3.268 | 31.123 | 1.00 | 29.14 | C |
| ATOM | 6608 | CG | ARG | D | 215 | 36.496 | -3.872 | 30.094 | 1.00 | 29.51 | C |
| ATOM | 6609 | CD | ARG | D | 215 | 37.075 | -5.037 | 29.323 | 1.00 | 28.07 | C |
| ATOM | 6610 | NE | ARG | D | 215 | 37.324 | -6.217 | 30.155 | 1.00 | 27.78 | N |
| ATOM | 6611 | CZ | ARG | D | 215 | 36.425 | -7.144 | 30.491 | 1.00 | 28.24 | C |
| ATOM | 6612 | NH1 | ARG | D | 215 | 35.153 | -7.063 | 30.118 | 1.00 | 29.78 | N |
| ATOM | 6613 | NH2 | ARG | D | 215 | 36.813 | -8.177 | 31.224 | 1.00 | 28.87 | N |
| TER | 6614 | | ARG | D | 215 | | | | | | |
| ATOM | 6615 | N | TYR | E | 1 | 33.158 | -15.271 | 72.412 | 1.00 | 26.90 | N |
| ATOM | 6616 | CA | TYR | E | 1 | 32.376 | -14.416 | 71.482 | 1.00 | 24.44 | C |
| ATOM | 6617 | C | TYR | E | 1 | 31.910 | -13.111 | 72.121 | 1.00 | 24.25 | C |
| ATOM | 6618 | O | TYR | E | 1 | 32.260 | -12.036 | 71.646 | 1.00 | 24.31 | O |
| ATOM | 6619 | CB | TYR | E | 1 | 31.156 | -15.144 | 70.895 | 1.00 | 24.41 | C |
| ATOM | 6620 | CG | TYR | E | 1 | 30.428 | -14.178 | 70.008 | 1.00 | 21.77 | C |

| ATOM | 6621 | CD1 | TYR | E | 1 | 30.936 | -13.848 | 68.757 | 1.00 | 22.80 | C |
|------|------|-----|-----|---|---|--------|---------|--------|------|-------|---|
| ATOM | 6622 | CD2 | TYR | E | 1 | 29.347 | -13.477 | 70.475 | 1.00 | 22.49 | C |
| ATOM | 6623 | CE1 | TYR | E | 1 | 30.331 | -12.860 | 67.975 | 1.00 | 22.22 | C |
| ATOM | 6624 | CE2 | TYR | E | 1 | 28.726 | -12.517 | 69.711 | 1.00 | 20.36 | C |
| ATOM | 6625 | CZ | TYR | E | 1 | 29.220 | -12.201 | 68.470 | 1.00 | 20.78 | C |
| ATOM | 6626 | OH | TYR | E | 1 | 28.569 | -11.228 | 67.749 | 1.00 | 23.07 | O |
| ATOM | 6627 | N | LEU | E | 2 | 31.127 | -13.217 | 73.193 | 1.00 | 24.57 | N |
| ATOM | 6628 | CA | LEU | E | 2 | 30.520 | -12.038 | 73.807 | 1.00 | 24.28 | C |
| ATOM | 6629 | C | LEU | E | 2 | 31.530 | -11.034 | 74.294 | 1.00 | 24.27 | C |
| ATOM | 6630 | O | LEU | E | 2 | 31.358 | -9.826 | 74.120 | 1.00 | 25.62 | O |
| ATOM | 6631 | CB | LEU | E | 2 | 29.521 | -12.411 | 74.910 | 1.00 | 23.77 | C |
| ATOM | 6632 | CG | LEU | E | 2 | 28.111 | -12.655 | 74.350 | 1.00 | 24.15 | C |
| ATOM | 6633 | CD1 | LEU | E | 2 | 27.174 | -13.302 | 75.360 | 1.00 | 24.60 | C |
| ATOM | 6634 | CD2 | LEU | E | 2 | 27.478 | -11.404 | 73.772 | 1.00 | 24.38 | C |
| ATOM | 6635 | N | GLU | E | 3 | 32.605 | -11.525 | 74.875 | 1.00 | 25.40 | N |
| ATOM | 6636 | CA | GLU | E | 3 | 33.665 | -10.651 | 75.338 | 1.00 | 26.53 | C |
| ATOM | 6637 | C | GLU | E | 3 | 34.180 | -9.832 | 74.159 | 1.00 | 25.20 | C |
| ATOM | 6638 | O | GLU | E | 3 | 34.345 | -8.615 | 74.276 | 1.00 | 26.30 | O |
| ATOM | 6639 | CB | GLU | E | 3 | 34.806 | -11.456 | 75.970 | 1.00 | 28.88 | C |
| ATOM | 6640 | CG | GLU | E | 3 | 34.411 | -12.141 | 77.260 | 1.00 | 32.49 | C |
| ATOM | 6641 | CD | GLU | E | 3 | 33.688 | -13.477 | 77.087 | 1.00 | 35.32 | C |
| ATOM | 6642 | OE1 | GLU | E | 3 | 33.555 | -14.033 | 75.956 | 1.00 | 38.15 | O |
| ATOM | 6643 | OE2 | GLU | E | 3 | 33.257 | -13.989 | 78.134 | 1.00 | 35.97 | O |
| ATOM | 6644 | N | ASP | E | 4 | 34.418 | -10.501 | 73.026 | 1.00 | 23.12 | N |
| ATOM | 6645 | CA | ASP | E | 4 | 34.902 | -9.829 | 71.800 | 1.00 | 23.82 | C |
| ATOM | 6646 | C | ASP | E | 4 | 33.881 | -8.826 | 71.274 | 1.00 | 23.26 | C |
| ATOM | 6647 | O | ASP | E | 4 | 34.244 | -7.704 | 70.913 | 1.00 | 23.77 | O |
| ATOM | 6648 | CB | ASP | E | 4 | 35.197 | -10.849 | 70.692 | 1.00 | 24.54 | C |
| ATOM | 6649 | CG | ASP | E | 4 | 36.438 | -11.700 | 70.961 | 1.00 | 25.26 | C |
| ATOM | 6650 | OD1 | ASP | E | 4 | 37.233 | -11.391 | 71.878 | 1.00 | 28.08 | O |
| ATOM | 6651 | OD2 | ASP | E | 4 | 36.632 | -12.671 | 70.206 | 1.00 | 29.57 | O |
| ATOM | 6652 | N | TRP | E | 5 | 32.610 | -9.243 | 71.218 | 1.00 | 22.29 | N |
| ATOM | 6653 | CA | TRP | E | 5 | 31.509 | -8.379 | 70.765 | 1.00 | 21.54 | C |
| ATOM | 6654 | C | TRP | E | 5 | 31.390 | -7.153 | 71.652 | 1.00 | 22.65 | C |
| ATOM | 6655 | O | TRP | E | 5 | 31.271 | -6.006 | 71.170 | 1.00 | 22.52 | O |
| ATOM | 6656 | CB | TRP | E | 5 | 30.162 | -9.123 | 70.752 | 1.00 | 20.97 | C |
| ATOM | 6657 | CG | TRP | E | 5 | 29.007 | -8.202 | 70.521 | 1.00 | 19.02 | C |
| ATOM | 6658 | CD1 | TRP | E | 5 | 28.193 | -7.657 | 71.463 | 1.00 | 20.61 | C |
| ATOM | 6659 | CD2 | TRP | E | 5 | 28.579 | -7.655 | 69.257 | 1.00 | 19.09 | C |
| ATOM | 6660 | NE1 | TRP | E | 5 | 27.298 | -6.803 | 70.873 | 1.00 | 19.41 | N |
| ATOM | 6661 | CE2 | TRP | E | 5 | 27.508 | -6.786 | 69.523 | 1.00 | 20.30 | C |
| ATOM | 6662 | CE3 | TRP | E | 5 | 28.989 | -7.837 | 67.927 | 1.00 | 19.62 | C |
| ATOM | 6663 | CZ2 | TRP | E | 5 | 26.818 | -6.093 | 68.510 | 1.00 | 20.03 | C |
| ATOM | 6664 | CZ3 | TRP | E | 5 | 28.306 | -7.134 | 66.910 | 1.00 | 19.27 | C |
| ATOM | 6665 | CH2 | TRP | E | 5 | 27.240 | -6.272 | 67.222 | 1.00 | 20.27 | C |
| ATOM | 6666 | N | ILE | E | 6 | 31.393 | -7.388 | 72.959 | 1.00 | 22.98 | N |
| ATOM | 6667 | CA | ILE | E | 6 | 31.171 | -6.294 | 73.903 | 1.00 | 25.74 | C |
| ATOM | 6668 | C | ILE | E | 6 | 32.288 | -5.268 | 73.804 | 1.00 | 25.75 | C |
| ATOM | 6669 | O | ILE | E | 6 | 32.020 | -4.052 | 73.743 | 1.00 | 27.32 | O |
| ATOM | 6670 | CB | ILE | E | 6 | 31.006 | -6.838 | 75.327 | 1.00 | 26.29 | C |
| ATOM | 6671 | CG1 | ILE | E | 6 | 29.622 | -7.483 | 75.446 | 1.00 | 26.66 | C |
| ATOM | 6672 | CG2 | ILE | E | 6 | 31.223 | -5.707 | 76.337 | 1.00 | 28.68 | C |
| ATOM | 6673 | CD1 | ILE | E | 6 | 29.453 | -8.364 | 76.630 | 1.00 | 26.19 | C |
| ATOM | 6674 | N | LYS | E | 7 | 33.524 | -5.748 | 73.764 | 1.00 | 28.03 | N |
| ATOM | 6675 | CA | LYS | E | 7 | 34.695 | -4.887 | 73.570 | 1.00 | 29.46 | C |
| ATOM | 6676 | C | LYS | E | 7 | 34.528 | -4.103 | 72.280 | 1.00 | 29.29 | C |
| ATOM | 6677 | O | LYS | E | 7 | 34.557 | -2.867 | 72.268 | 1.00 | 30.23 | O |
| ATOM | 6678 | CB | LYS | E | 7 | 35.977 | -5.725 | 73.548 | 1.00 | 30.97 | C |
| ATOM | 6679 | CG | LYS | E | 7 | 37.301 | -4.924 | 73.665 | 1.00 | 34.02 | C |
| ATOM | 6680 | CD | LYS | E | 7 | 38.518 | -5.872 | 73.717 | 1.00 | 36.95 | C |
| ATOM | 6681 | CE | LYS | E | 7 | 39.811 | -5.253 | 73.137 | 1.00 | 38.08 | C |
| ATOM | 6682 | NZ | LYS | E | 7 | 41.007 | -6.112 | 73.458 | 1.00 | 38.76 | N |
| ATOM | 6683 | N | TYR | E | 8 | 34.304 | -4.817 | 71.187 | 1.00 | 27.44 | N |
| ATOM | 6684 | CA | TYR | E | 8 | 34.087 | -4.174 | 69.889 | 1.00 | 27.66 | C |
| ATOM | 6685 | C | TYR | E | 8 | 32.979 | -3.113 | 69.925 | 1.00 | 29.36 | C |

| ATOM | 6686 | O   | TYR | E | 8  | 33.180 | -1.976  | 69.488  | 1.00 | 30.47 | O |
| ATOM | 6687 | CB  | TYR | E | 8  | 33.772 | -5.256  | 68.850  | 1.00 | 24.60 | C |
| ATOM | 6688 | CG  | TYR | E | 8  | 33.049 | -4.774  | 67.600  | 1.00 | 23.27 | C |
| ATOM | 6689 | CD1 | TYR | E | 8  | 33.682 | -3.952  | 66.677  | 1.00 | 21.55 | C |
| ATOM | 6690 | CD2 | TYR | E | 8  | 31.744 | -5.162  | 67.341  | 1.00 | 20.34 | C |
| ATOM | 6691 | CE1 | TYR | E | 8  | 33.028 | -3.532  | 65.544  | 1.00 | 20.51 | C |
| ATOM | 6692 | CE2 | TYR | E | 8  | 31.078 | -4.746  | 66.190  | 1.00 | 22.65 | C |
| ATOM | 6693 | CZ  | TYR | E | 8  | 31.725 | -3.926  | 65.306  | 1.00 | 19.64 | C |
| ATOM | 6694 | OH  | TYR | E | 8  | 31.061 | -3.528  | 64.181  | 1.00 | 19.73 | O |
| ATOM | 6695 | N   | ASN | E | 9  | 31.819 | -3.482  | 70.455  | 1.00 | 31.61 | N |
| ATOM | 6696 | CA  | ASN | E | 9  | 30.611 | -2.656  | 70.417  | 1.00 | 33.92 | C |
| ATOM | 6697 | C   | ASN | E | 9  | 30.706 | -1.400  | 71.305  | 1.00 | 37.02 | C |
| ATOM | 6698 | O   | ASN | E | 9  | 30.043 | -0.382  | 71.034  | 1.00 | 36.58 | O |
| ATOM | 6699 | CB  | ASN | E | 9  | 29.404 | -3.508  | 70.831  | 1.00 | 34.26 | C |
| ATOM | 6700 | CG  | ASN | E | 9  | 28.090 | -2.769  | 70.730  | 1.00 | 34.57 | C |
| ATOM | 6701 | OD1 | ASN | E | 9  | 27.655 | -2.387  | 69.650  | 1.00 | 33.70 | O |
| ATOM | 6702 | ND2 | ASN | E | 9  | 27.436 | -2.575  | 71.878  | 1.00 | 36.56 | N |
| ATOM | 6703 | N   | ASN | E | 10 | 31.510 | -1.482  | 72.361  | 1.00 | 40.88 | N |
| ATOM | 6704 | CA  | ASN | E | 10 | 31.726 | -0.330  | 73.244  | 1.00 | 43.49 | C |
| ATOM | 6705 | C   | ASN | E | 10 | 32.601 | 0.698   | 72.552  | 1.00 | 45.74 | C |
| ATOM | 6706 | O   | ASN | E | 10 | 32.363 | 1.910   | 72.691  | 1.00 | 48.33 | O |
| ATOM | 6707 | CB  | ASN | E | 10 | 32.337 | -0.768  | 74.582  | 1.00 | 44.03 | C |
| ATOM | 6708 | CG  | ASN | E | 10 | 31.300 | -1.377  | 75.524  | 1.00 | 44.63 | C |
| ATOM | 6709 | OD1 | ASN | E | 10 | 30.099 | -1.356  | 75.235  | 1.00 | 45.36 | O |
| ATOM | 6710 | ND2 | ASN | E | 10 | 31.759 | -1.907  | 76.661  | 1.00 | 43.60 | N |
| ATOM | 6711 | N   | GLN | E | 11 | 33.599 | 0.214   | 71.806  | 1.00 | 46.93 | N |
| ATOM | 6712 | CA  | GLN | E | 11 | 34.481 | 1.072   | 71.004  | 1.00 | 47.49 | C |
| ATOM | 6713 | C   | GLN | E | 11 | 33.673 | 1.693   | 69.866  | 1.00 | 47.97 | C |
| ATOM | 6714 | O   | GLN | E | 11 | 34.197 | 2.477   | 69.085  | 1.00 | 48.94 | O |
| ATOM | 6715 | CB  | GLN | E | 11 | 35.655 | 0.263   | 70.425  | 1.00 | 47.44 | C |
| ATOM | 6716 | CG  | GLN | E | 11 | 36.579 | -0.352  | 71.486  | 1.00 | 48.37 | C |
| ATOM | 6717 | CD  | GLN | E | 11 | 37.448 | -1.497  | 70.969  | 1.00 | 48.31 | C |
| ATOM | 6718 | OE1 | GLN | E | 11 | 37.206 | -2.031  | 69.891  | 1.00 | 49.77 | O |
| ATOM | 6719 | NE2 | GLN | E | 11 | 38.459 | -1.889  | 71.753  | 1.00 | 47.60 | N |
| TER  | 6720 |     | GLN | E | 11 |        |         |         |      |       |   |
| ATOM | 6721 | N   | TYR | F | 1  | 2.204  | -15.451 | -25.292 | 1.00 | 33.37 | N |
| ATOM | 6722 | CA  | TYR | F | 1  | 2.588  | -14.385 | -24.317 | 1.00 | 33.31 | C |
| ATOM | 6723 | C   | TYR | F | 1  | 3.094  | -13.114 | -25.024 | 1.00 | 33.21 | C |
| ATOM | 6724 | O   | TYR | F | 1  | 2.654  | -12.000 | -24.744 | 1.00 | 33.90 | O |
| ATOM | 6725 | CB  | TYR | F | 1  | 3.651  | -14.897 | -23.307 | 1.00 | 32.85 | C |
| ATOM | 6726 | CG  | TYR | F | 1  | 4.231  | -13.753 | -22.522 | 1.00 | 31.46 | C |
| ATOM | 6727 | CD1 | TYR | F | 1  | 3.538  | -13.200 | -21.461 | 1.00 | 30.93 | C |
| ATOM | 6728 | CD2 | TYR | F | 1  | 5.430  | -13.157 | -22.901 | 1.00 | 30.56 | C |
| ATOM | 6729 | CE1 | TYR | F | 1  | 4.045  | -12.106 | -20.766 | 1.00 | 29.97 | C |
| ATOM | 6730 | CE2 | TYR | F | 1  | 5.938  | -12.073 | -22.218 | 1.00 | 29.19 | C |
| ATOM | 6731 | CZ  | TYR | F | 1  | 5.244  | -11.549 | -21.162 | 1.00 | 28.36 | C |
| ATOM | 6732 | OH  | TYR | F | 1  | 5.745  | -10.463 | -20.512 | 1.00 | 28.91 | O |
| ATOM | 6733 | N   | LEU | F | 2  | 4.042  | -13.286 | -25.934 | 1.00 | 34.81 | N |
| ATOM | 6734 | CA  | LEU | F | 2  | 4.702  | -12.149 | -26.567 | 1.00 | 35.09 | C |
| ATOM | 6735 | C   | LEU | F | 2  | 3.795  | -11.204 | -27.346 | 1.00 | 35.88 | C |
| ATOM | 6736 | O   | LEU | F | 2  | 3.990  | -9.995  | -27.323 | 1.00 | 36.04 | O |
| ATOM | 6737 | CB  | LEU | F | 2  | 5.845  | -12.618 | -27.472 | 1.00 | 36.44 | C |
| ATOM | 6738 | CG  | LEU | F | 2  | 7.205  | -12.705 | -26.763 | 1.00 | 35.30 | C |
| ATOM | 6739 | CD1 | LEU | F | 2  | 8.277  | -13.255 | -27.676 | 1.00 | 35.81 | C |
| ATOM | 6740 | CD2 | LEU | F | 2  | 7.623  | -11.349 | -26.259 | 1.00 | 35.64 | C |
| ATOM | 6741 | N   | GLU | F | 3  | 2.816  | -11.748 | -28.053 | 1.00 | 37.21 | N |
| ATOM | 6742 | CA  | GLU | F | 3  | 1.869  | -10.876 | -28.764 | 1.00 | 37.52 | C |
| ATOM | 6743 | C   | GLU | F | 3  | 1.107  | -10.018 | -27.743 | 1.00 | 35.84 | C |
| ATOM | 6744 | O   | GLU | F | 3  | 0.954  | -8.825  | -27.936 | 1.00 | 36.04 | O |
| ATOM | 6745 | CB  | GLU | F | 3  | 0.951  | -11.618 | -29.746 | 1.00 | 38.85 | C |
| ATOM | 6746 | CG  | GLU | F | 3  | 0.467  | -12.980 | -29.337 | 1.00 | 41.00 | C |
| ATOM | 6747 | CD  | GLU | F | 3  | 1.542  | -14.058 | -29.444 | 1.00 | 43.19 | C |
| ATOM | 6748 | OE1 | GLU | F | 3  | 2.310  | -14.065 | -30.438 | 1.00 | 46.18 | O |
| ATOM | 6749 | OE2 | GLU | F | 3  | 1.626  | -14.902 | -28.522 | 1.00 | 43.80 | O |
| ATOM | 6750 | N   | ASP | F | 4  | 0.698  | -10.619 | -26.634 | 1.00 | 35.10 | N |

```
ATOM   6751  CA   ASP F   4     0.150  -9.857 -25.510  1.00 33.58           C
ATOM   6752  C    ASP F   4     1.138  -8.802 -24.995  1.00 32.59           C
ATOM   6753  O    ASP F   4     0.779  -7.620 -24.871  1.00 30.45           O
ATOM   6754  CB   ASP F   4    -0.252 -10.811 -24.378  1.00 34.97           C
ATOM   6755  CG   ASP F   4    -1.530 -11.571 -24.679  1.00 35.98           C
ATOM   6756  OD1  ASP F   4    -2.188 -11.231 -25.678  1.00 37.27           O
ATOM   6757  OD2  ASP F   4    -1.888 -12.493 -23.913  1.00 36.57           O
ATOM   6758  N    TRP F   5     2.373  -9.214 -24.706  1.00 31.37           N
ATOM   6759  CA   TRP F   5     3.388  -8.257 -24.233  1.00 31.45           C
ATOM   6760  C    TRP F   5     3.605  -7.108 -25.230  1.00 31.43           C
ATOM   6761  O    TRP F   5     3.655  -5.934 -24.844  1.00 32.48           O
ATOM   6762  CB   TRP F   5     4.733  -8.936 -23.900  1.00 30.35           C
ATOM   6763  CG   TRP F   5     5.751  -7.910 -23.597  1.00 29.48           C
ATOM   6764  CD1  TRP F   5     6.662  -7.376 -24.459  1.00 28.21           C
ATOM   6765  CD2  TRP F   5     5.902  -7.198 -22.363  1.00 29.32           C
ATOM   6766  NE1  TRP F   5     7.377  -6.389 -23.841  1.00 27.99           N
ATOM   6767  CE2  TRP F   5     6.938  -6.265 -22.549  1.00 28.96           C
ATOM   6768  CE3  TRP F   5     5.272  -7.265 -21.117  1.00 28.39           C
ATOM   6769  CZ2  TRP F   5     7.355  -5.414 -21.541  1.00 27.64           C
ATOM   6770  CZ3  TRP F   5     5.691  -6.408 -20.116  1.00 25.93           C
ATOM   6771  CH2  TRP F   5     6.724  -5.509 -20.330  1.00 27.65           C
ATOM   6772  N    ILE F   6     3.726  -7.446 -26.509  1.00 32.66           N
ATOM   6773  CA   ILE F   6     3.995  -6.436 -27.540  1.00 34.04           C
ATOM   6774  C    ILE F   6     2.888  -5.385 -27.635  1.00 34.68           C
ATOM   6775  O    ILE F   6     3.171  -4.191 -27.721  1.00 35.10           O
ATOM   6776  CB   ILE F   6     4.284  -7.094 -28.906  1.00 35.04           C
ATOM   6777  CG1  ILE F   6     5.661  -7.770 -28.847  1.00 35.34           C
ATOM   6778  CG2  ILE F   6     4.275  -6.033 -30.029  1.00 35.09           C
ATOM   6779  CD1  ILE F   6     5.876  -8.905 -29.837  1.00 35.74           C
ATOM   6780  N    LYS F   7     1.638  -5.838 -27.606  1.00 36.09           N
ATOM   6781  CA   LYS F   7     0.486  -4.949 -27.625  1.00 36.19           C
ATOM   6782  C    LYS F   7     0.507  -3.988 -26.426  1.00 36.11           C
ATOM   6783  O    LYS F   7     0.448  -2.764 -26.582  1.00 35.04           O
ATOM   6784  CB   LYS F   7    -0.805  -5.773 -27.642  1.00 37.78           C
ATOM   6785  CG   LYS F   7    -2.054  -4.988 -28.056  1.00 39.97           C
ATOM   6786  CD   LYS F   7    -3.235  -5.911 -28.359  1.00 41.64           C
ATOM   6787  CE   LYS F   7    -4.488  -5.103 -28.754  1.00 42.82           C
ATOM   6788  NZ   LYS F   7    -5.583  -5.984 -29.277  1.00 43.58           N
ATOM   6789  N    TYR F   8     0.622  -4.569 -25.237  1.00 33.40           N
ATOM   6790  CA   TYR F   8     0.693  -3.804 -23.982  1.00 32.76           C
ATOM   6791  C    TYR F   8     1.816  -2.770 -23.994  1.00 33.12           C
ATOM   6792  O    TYR F   8     1.619  -1.581 -23.682  1.00 32.02           O
ATOM   6793  CB   TYR F   8     0.866  -4.800 -22.824  1.00 32.29           C
ATOM   6794  CG   TYR F   8     1.299  -4.185 -21.517  1.00 30.76           C
ATOM   6795  CD1  TYR F   8     0.459  -3.347 -20.813  1.00 30.35           C
ATOM   6796  CD2  TYR F   8     2.551  -4.468 -20.981  1.00 29.78           C
ATOM   6797  CE1  TYR F   8     0.871  -2.773 -19.629  1.00 29.44           C
ATOM   6798  CE2  TYR F   8     2.957  -3.924 -19.808  1.00 28.54           C
ATOM   6799  CZ   TYR F   8     2.128  -3.086 -19.128  1.00 28.56           C
ATOM   6800  OH   TYR F   8     2.556  -2.569 -17.950  1.00 25.61           O
ATOM   6801  N    ASN F   9     3.006  -3.241 -24.342  1.00 35.36           N
ATOM   6802  CA   ASN F   9     4.184  -2.404 -24.387  1.00 37.24           C
ATOM   6803  C    ASN F   9     4.057  -1.218 -25.350  1.00 39.56           C
ATOM   6804  O    ASN F   9     4.468  -0.112 -25.029  1.00 39.66           O
ATOM   6805  CB   ASN F   9     5.405  -3.248 -24.746  1.00 37.47           C
ATOM   6806  CG   ASN F   9     6.682  -2.452 -24.697  1.00 37.10           C
ATOM   6807  OD1  ASN F   9     7.128  -2.036 -23.629  1.00 36.84           O
ATOM   6808  ND2  ASN F   9     7.259  -2.196 -25.867  1.00 38.63           N
ATOM   6809  N    ASN F  10     3.475  -1.449 -26.518  1.00 42.44           N
ATOM   6810  CA   ASN F  10     3.290  -0.366 -27.489  1.00 43.98           C
ATOM   6811  C    ASN F  10     2.394   0.744 -26.992  1.00 44.65           C
ATOM   6812  O    ASN F  10     2.644   1.913 -27.284  1.00 47.43           O
ATOM   6813  CB   ASN F  10     2.733  -0.913 -28.792  1.00 44.52           C
ATOM   6814  CG   ASN F  10     3.763  -1.688 -29.571  1.00 45.19           C
ATOM   6815  OD1  ASN F  10     4.955  -1.392 -29.502  1.00 47.43           O
```

```
ATOM    6816  ND2 ASN F   10      3.314  -2.691 -30.316  1.00 45.81           N
ATOM    6817  N   GLN F   11      1.368   0.407 -26.225  1.00 44.76           N
ATOM    6818  CA  GLN F   11      0.465   1.469 -25.771  1.00 44.86           C
ATOM    6819  C   GLN F   11      0.785   2.052 -24.387  1.00 43.55           C
ATOM    6820  O   GLN F   11      0.057   2.904 -23.869  1.00 41.62           O
ATOM    6821  CB  GLN F   11     -1.008   1.109 -25.966  1.00 46.16           C
ATOM    6822  CG  GLN F   11     -1.432  -0.165 -25.334  1.00 48.23           C
ATOM    6823  CD  GLN F   11     -2.526   0.056 -24.316  1.00 50.87           C
ATOM    6824  OE1 GLN F   11     -2.358   0.809 -23.350  1.00 51.57           O
ATOM    6825  NE2 GLN F   11     -3.664  -0.597 -24.527  1.00 52.50           N
ATOM    6826  N   LYS F   12      1.928   1.632 -23.845  1.00 42.58           N
ATOM    6827  CA  LYS F   12      2.514   2.205 -22.627  1.00 42.32           C
ATOM    6828  C   LYS F   12      2.801   3.700 -22.738  1.00 40.78           C
ATOM    6829  O   LYS F   12      2.808   4.396 -21.729  1.00 38.46           O
ATOM    6830  CB  LYS F   12      3.859   1.532 -22.312  1.00 43.02           C
ATOM    6831  CG  LYS F   12      3.857   0.526 -21.180  1.00 45.23           C
ATOM    6832  CD  LYS F   12      5.257   0.476 -20.586  1.00 46.53           C
ATOM    6833  CE  LYS F   12      5.381  -0.469 -19.420  1.00 47.58           C
ATOM    6834  NZ  LYS F   12      6.779  -0.443 -18.841  1.00 48.69           N
ATOM    6835  OXT LYS F   12      3.090   4.207 -23.822  1.00 40.54           O
TER     6836      LYS F   12
HETATM  6837  O   HOH A  215     22.416 -19.077  60.173  1.00 15.15           O
HETATM  6838  O   HOH A  216     25.360  -4.995  72.234  1.00 21.38           O
HETATM  6839  O   HOH A  217     18.366  -7.147  77.767  1.00 19.04           O
HETATM  6840  O   HOH A  218     11.977 -21.687  63.528  1.00 24.95           O
HETATM  6841  O   HOH A  219     11.159  -5.044  55.474  1.00 21.96           O
HETATM  6842  O   HOH A  220     24.761 -20.673  67.112  1.00 26.28           O
HETATM  6843  O   HOH A  221     -0.461 -22.947  32.967  1.00 21.88           O
HETATM  6844  O   HOH A  222     13.700 -11.707  81.669  1.00 23.78           O
HETATM  6845  O   HOH A  223     -3.519 -14.561  27.746  1.00 24.41           O
HETATM  6846  O   HOH A  224     -2.004 -30.391  34.838  1.00 32.09           O
HETATM  6847  O   HOH A  225     14.475 -23.597  69.840  1.00 23.52           O
HETATM  6848  O   HOH A  226     18.240  -9.120  80.241  1.00 21.46           O
HETATM  6849  O   HOH A  227    -17.770 -23.556  29.023  1.00 27.90           O
HETATM  6850  O   HOH A  228     23.359  -4.729  69.356  1.00 19.83           O
HETATM  6851  O   HOH A  229     31.293 -18.401  68.995  1.00 24.10           O
HETATM  6852  O   HOH A  230      0.669 -15.675  37.583  1.00 23.68           O
HETATM  6853  O   HOH A  231      9.722  -3.553  74.687  1.00 23.11           O
HETATM  6854  O   HOH A  232     10.611 -18.940  79.955  1.00 23.22           O
HETATM  6855  O   HOH A  233     17.437 -21.429  64.158  1.00 17.87           O
HETATM  6856  O   HOH A  234    -10.034  -9.315  38.357  1.00 28.17           O
HETATM  6857  O   HOH A  235     -1.381 -14.335  25.594  1.00 26.60           O
HETATM  6858  O   HOH A  236     12.863   0.922  73.855  1.00 25.01           O
HETATM  6859  O   HOH A  237     -2.716  -6.076  55.542  1.00 27.90           O
HETATM  6860  O   HOH A  238      8.889 -20.382  61.187  1.00 27.60           O
HETATM  6861  O   HOH A  239      8.695 -17.955  57.329  1.00 30.57           O
HETATM  6862  O   HOH A  240     15.088 -18.365  58.752  1.00 22.67           O
HETATM  6863  O   HOH A  241     -7.373 -26.937  16.076  1.00 26.43           O
HETATM  6864  O   HOH A  242     -1.362 -18.594  37.372  1.00 24.30           O
HETATM  6865  O   HOH A  243     -1.875   1.104  65.705  1.00 29.81           O
HETATM  6866  O   HOH A  244     -1.246 -24.624  40.853  1.00 27.50           O
HETATM  6867  O   HOH A  245     21.289 -22.291  78.320  1.00 29.88           O
HETATM  6868  O   HOH A  246     20.821  -2.978  76.682  1.00 27.17           O
HETATM  6869  O   HOH A  247    -11.534  -9.144  31.024  1.00 30.12           O
HETATM  6870  O   HOH A  248     -9.333 -25.885  24.214  1.00 27.41           O
HETATM  6871  O   HOH A  249     -0.091 -11.766  55.884  1.00 31.71           O
HETATM  6872  O   HOH A  250    -13.730 -10.660  32.554  1.00 27.83           O
HETATM  6873  O   HOH A  251     -9.006 -15.701  20.765  1.00 33.62           O
HETATM  6874  O   HOH A  252    -16.518 -24.903  20.258  1.00 27.90           O
HETATM  6875  O   HOH A  253    -16.156 -13.065  23.984  1.00 22.65           O
HETATM  6876  O   HOH A  254     -3.367 -28.185  24.871  1.00 27.53           O
HETATM  6877  O   HOH A  255     18.475   2.244  67.373  1.00 30.61           O
HETATM  6878  O   HOH A  256      3.262  -3.715  72.120  1.00 29.37           O
HETATM  6879  O   HOH A  257      2.371 -10.127  73.961  1.00 30.15           O
HETATM  6880  O   HOH A  258     21.144 -23.946  64.455  1.00 28.76           O
```

```
HETATM 6881  O    HOH A 259     26.389  -5.227  74.854  1.00 31.07           O
HETATM 6882  O    HOH A 260     -9.544 -15.071  58.503  1.00 41.58           O
HETATM 6883  O    HOH A 261      3.770  -4.707  54.947  1.00 29.72           O
HETATM 6884  O    HOH A 262      0.010 -26.771  30.009  1.00 33.44           O
HETATM 6885  O    HOH A 263      7.666 -10.561  53.847  1.00 29.80           O
HETATM 6886  O    HOH A 264     24.991 -14.317  66.526  1.00 25.39           O
HETATM 6887  O    HOH A 265    -10.647 -26.733  36.372  1.00 30.17           O
HETATM 6888  O    HOH A 266     -1.340   0.396  57.625  1.00 40.60           O
HETATM 6889  O    HOH A 267     -9.428 -28.208  25.743  1.00 28.10           O
HETATM 6890  O    HOH A 268     28.209  -5.505  78.666  1.00 27.72           O
HETATM 6891  O    HOH A 269     17.489  -2.958  53.156  1.00 32.88           O
HETATM 6892  O    HOH A 270      5.683  -6.550  75.335  1.00 38.49           O
HETATM 6893  O    HOH A 271     -4.988  -8.516  47.015  1.00 31.65           O
HETATM 6894  O    HOH A 272      4.877 -23.584  61.949  1.00 38.48           O
HETATM 6895  O    HOH A 273      0.369 -16.517  34.739  1.00 26.99           O
HETATM 6896  O    HOH A 274      6.451 -19.461  67.820  1.00 35.44           O
HETATM 6897  O    HOH A 275    -19.342 -18.674  24.143  1.00 30.53           O
HETATM 6898  O    HOH A 276     14.579 -22.901  63.376  1.00 28.15           O
HETATM 6899  O    HOH A 277      2.445 -18.947  43.952  1.00 27.54           O
HETATM 6900  O    HOH A 278      1.542 -20.934  33.472  1.00 28.16           O
HETATM 6901  O    HOH A 279      1.427 -19.058  35.260  1.00 26.57           O
HETATM 6902  O    HOH A 280    -15.310 -22.897  41.129  1.00 34.84           O
HETATM 6903  O    HOH A 281     17.018  -3.426  78.474  1.00 34.82           O
HETATM 6904  O    HOH A 282    -20.421  -9.096  23.308  1.00 43.51           O
HETATM 6905  O    HOH A 283     -0.652 -26.538  18.448  1.00 34.98           O
HETATM 6906  O    HOH A 284     -9.593  -5.810  60.628  1.00 35.29           O
HETATM 6907  O    HOH A 285     14.374  -3.732  51.881  1.00 37.52           O
HETATM 6908  O    HOH A 286     -1.364  -9.971  71.500  1.00 27.64           O
HETATM 6909  O    HOH A 287      8.226  -1.103  74.933  1.00 31.20           O
HETATM 6910  O    HOH A 288     19.429  -4.683  78.533  1.00 24.03           O
HETATM 6911  O    HOH A 289     -7.407 -16.014  55.181  1.00 40.95           O
HETATM 6912  O    HOH A 290     22.823 -11.924  84.227  1.00 27.05           O
HETATM 6913  O    HOH A 291    -18.548 -21.057  28.336  1.00 33.47           O
HETATM 6914  O    HOH A 292    -14.299 -29.116  25.735  1.00 30.61           O
HETATM 6915  O    HOH A 293    -16.338 -10.330  39.524  1.00 29.53           O
HETATM 6916  O    HOH A 294     13.207 -17.228  55.621  1.00 34.06           O
HETATM 6917  O    HOH A 295     -7.923 -18.638  17.721  1.00 41.41           O
HETATM 6918  O    HOH A 296     16.462  -7.493  79.978  1.00 33.90           O
HETATM 6919  O    HOH A 297     31.056 -18.869  71.921  1.00 37.23           O
HETATM 6920  O    HOH A 298      5.798 -17.481  66.509  1.00 33.44           O
HETATM 6921  O    HOH A 299     28.050 -25.529  62.731  1.00 30.14           O
HETATM 6922  O    HOH A 300      7.595 -14.149  76.238  1.00 37.03           O
HETATM 6923  O    HOH A 301    -21.513 -15.274  38.170  1.00 32.93           O
HETATM 6924  O    HOH A 302     -8.803 -24.390  42.677  1.00 28.53           O
HETATM 6925  O    HOH A 303     16.516  -3.335  55.798  1.00 40.16           O
HETATM 6926  O    HOH A 304    -15.364 -26.159  17.995  1.00 31.65           O
HETATM 6927  O    HOH A 305    -15.153 -11.796  28.274  1.00 31.07           O
HETATM 6928  O    HOH A 306     -5.415 -31.210  25.311  1.00 35.24           O
HETATM 6929  O    HOH A 307    -16.613 -12.443  15.205  1.00 26.11           O
HETATM 6930  O    HOH A 308     11.034  -6.568  78.950  1.00 25.80           O
HETATM 6931  O    HOH A 309    -18.427 -17.633  45.382  1.00 31.28           O
HETATM 6932  O    HOH A 310      5.694  -1.564  60.224  1.00 48.76           O
HETATM 6933  O    HOH A 311     21.659 -25.126  71.441  1.00 35.61           O
HETATM 6934  O    HOH A 312     -1.124 -28.259  36.024  1.00 37.18           O
HETATM 6935  O    HOH A 313    -18.133 -15.193  46.030  1.00 34.16           O
HETATM 6936  O    HOH A 314     -3.940 -17.307  54.519  1.00 39.77           O
HETATM 6937  O    HOH A 315     26.749  -3.690  80.432  1.00 31.76           O
HETATM 6938  O    HOH A 316     14.098  -2.640  55.201  1.00 40.07           O
HETATM 6939  O    HOH A 317     -4.472 -24.690  43.190  1.00 32.91           O
HETATM 6940  O    HOH A 318      5.658  -9.036  74.671  1.00 31.15           O
HETATM 6941  O    HOH A 319     30.238  -5.865  80.099  1.00 33.35           O
HETATM 6942  O    HOH A 320    -21.815 -15.294  43.362  1.00 37.58           O
HETATM 6943  O    HOH A 321     28.003 -17.085  74.420  1.00 37.04           O
HETATM 6944  O    HOH A 322    -18.247  -8.391  20.420  1.00 38.14           O
HETATM 6945  O    HOH A 323     26.410 -17.096  76.783  1.00 30.02           O
```

```
HETATM 6946  O    HOH A 324      7.026    0.096  73.082  1.00 37.43           O
HETATM 6947  O    HOH A 325     -0.822  -12.990  73.266  1.00 43.47           O
HETATM 6948  O    HOH A 326    -13.359  -19.268  50.530  1.00 38.38           O
HETATM 6949  O    HOH A 327    -12.177  -29.715  29.216  1.00 43.89           O
HETATM 6950  O    HOH A 328     -2.406   -9.214  47.694  1.00 37.21           O
HETATM 6951  O    HOH A 329     12.586  -19.486  57.253  1.00 36.61           O
HETATM 6952  O    HOH A 330     -4.347   -0.203  59.059  1.00 42.83           O
HETATM 6953  O    HOH A 331     11.472    3.961  68.625  1.00 36.00           O
HETATM 6954  O    HOH A 332      8.214  -20.513  69.528  1.00 31.14           O
HETATM 6955  O    HOH A 333     16.328  -12.651  80.477  1.00 31.43           O
HETATM 6956  O    HOH A 334      5.608  -16.454  15.489  1.00 37.25           O
HETATM 6957  O    HOH A 335     -1.442    0.511  55.074  1.00 43.95           O
HETATM 6958  O    HOH A 336     -5.181   -0.191  63.212  1.00 38.86           O
HETATM 6959  O    HOH A 337     -6.105   -0.108  57.027  1.00 41.16           O
HETATM 6960  O    HOH A 338    -10.597  -23.879  14.667  1.00 33.07           O
HETATM 6961  O    HOH A 339     -7.133  -13.368  21.113  1.00 36.13           O
HETATM 6962  O    HOH A 340      9.422   -4.821  77.754  1.00 34.94           O
HETATM 6963  O    HOH A 341      6.641  -15.598  17.867  1.00 31.30           O
HETATM 6964  O    HOH A 342     -7.778  -10.090  66.916  1.00 42.98           O
HETATM 6965  O    HOH A 343    -11.270  -16.571  15.732  1.00 37.32           O
HETATM 6966  O    HOH A 344    -14.729  -22.329  15.045  1.00 33.83           O
HETATM 6967  O    HOH A 345    -12.137  -19.827  13.758  1.00 33.68           O
HETATM 6968  O    HOH A 346     -9.315   -5.967  42.237  1.00 35.30           O
HETATM 6969  O    HOH A 347      1.552  -21.465  40.004  1.00 29.45           O
HETATM 6970  O    HOH A 348      0.552  -19.572  45.276  1.00 34.43           O
HETATM 6971  O    HOH A 349     18.802  -23.305  63.044  1.00 31.51           O
HETATM 6972  O    HOH A 350      8.556  -24.010  63.596  1.00 42.68           O
HETATM 6973  O    HOH A 351    -17.414  -19.445  18.281  1.00 37.32           O
HETATM 6974  O    HOH A 352      2.049  -21.801  62.381  1.00 40.57           O
HETATM 6975  O    HOH A 353    -21.621  -23.562  45.317  1.00 48.18           O
HETATM 6976  O    HOH A 354      7.103  -24.163  65.911  1.00 38.74           O
HETATM 6977  O    HOH A 355      7.878  -19.351  73.371  1.00 39.11           O
HETATM 6978  O    HOH A 356      5.096  -11.859  53.870  1.00 35.22           O
HETATM 6979  O    HOH A 357      2.560  -17.765  66.315  1.00 47.41           O
HETATM 6980  O    HOH A 358      8.894  -22.165  59.358  1.00 32.85           O
HETATM 6981  O    HOH A 359     -8.183  -33.444  18.020  1.00 44.52           O
HETATM 6982  O    HOH A 360    -24.763  -17.304  48.980  1.00 46.34           O
HETATM 6983  O    HOH A 361      1.618  -23.875  40.888  1.00 37.68           O
HETATM 6984  O    HOH A 362      7.937    6.139  64.471  1.00 43.32           O
HETATM 6985  O    HOH A 363     21.252  -26.636  64.795  1.00 36.76           O
HETATM 6986  O    HOH A 364      1.108  -19.838  37.984  1.00 31.42           O
HETATM 6987  O    HOH A 365     -0.390  -29.568  30.199  1.00 39.87           O
HETATM 6988  O    HOH A 366    -20.790  -11.505  40.701  1.00 36.30           O
HETATM 6989  O    HOH A 367     16.014  -20.522  59.681  1.00 46.56           O
HETATM 6990  O    HOH A 368     -0.514  -29.716  18.334  1.00 33.88           O
HETATM 6991  O    HOH A 369     15.330    1.428  75.045  1.00 38.07           O
HETATM 6992  O    HOH A 370    -17.388  -23.111  14.815  1.00 35.75           O
HETATM 6993  O    HOH A 371     16.357    2.274  63.214  1.00 30.54           O
HETATM 6994  O    HOH A 372      1.209  -20.026  30.390  1.00 33.75           O
HETATM 6995  O    HOH A 373      6.301  -17.810  57.717  1.00 34.94           O
HETATM 6996  O    HOH A 374      1.497  -24.725  24.939  1.00 34.11           O
HETATM 6997  O    HOH A 375      3.277  -19.637  24.517  1.00 35.43           O
HETATM 6998  O    HOH A 376    -12.336  -11.496  28.305  1.00 43.30           O
HETATM 6999  O    HOH A 377     -8.406  -31.865  32.334  1.00 37.04           O
HETATM 7000  O    HOH A 378    -11.592  -29.158  26.865  1.00 41.59           O
HETATM 7001  O    HOH A 379     27.107  -15.862  78.905  1.00 32.60           O
HETATM 7002  O    HOH A 380     30.108   -9.888  82.116  1.00 43.23           O
HETATM 7003  O    HOH A 381    -16.123  -30.644  24.142  1.00 45.92           O
HETATM 7004  O    HOH A 382     30.557  -14.120  81.673  1.00 48.68           O
HETATM 7005  O    HOH A 383     20.392    4.584  67.129  1.00 34.79           O
HETATM 7006  O    HOH A 384     -0.319  -22.638  44.697  1.00 37.13           O
HETATM 7007  O    HOH A 385    -13.915  -24.649  42.216  1.00 43.52           O
HETATM 7008  O    HOH A 386    -21.963  -14.133  40.550  1.00 39.96           O
HETATM 7009  O    HOH A 387      3.591  -19.780  22.006  1.00 41.42           O
HETATM 7010  O    HOH A 388     22.814   -4.907  82.287  1.00 39.01           O
```

```
HETATM 7011  O    HOH A 389    -5.485  -9.118  70.580  1.00 39.53        O
HETATM 7012  O    HOH A 390    -0.914 -14.066  55.054  1.00 32.23        O
HETATM 7013  O    HOH A 391    21.831 -14.560  64.864  1.00 32.84        O
HETATM 7014  O    HOH A 392     1.515 -11.573  53.518  1.00 47.33        O
HETATM 7015  O    HOH A 393    19.552 -22.015  60.959  1.00 34.72        O
HETATM 7016  O    HOH A 394    -8.460 -14.505  63.917  1.00 40.39        O
HETATM 7017  O    HOH A 395   -17.574 -23.579  39.668  1.00 35.21        O
HETATM 7018  O    HOH A 396     1.145 -27.129  26.192  1.00 43.13        O
HETATM 7019  O    HOH A 397    31.824 -10.383  79.209  1.00 42.60        O
HETATM 7020  O    HOH A 398    -8.913 -25.323  37.948  1.00 39.12        O
HETATM 7021  O    HOH A 399    -7.533 -25.113  40.265  1.00 36.08        O
HETATM 7022  O    HOH A 400   -11.698 -13.967  21.890  1.00 39.36        O
HETATM 7023  O    HOH A 401    26.146 -23.945  70.054  1.00 47.88        O
HETATM 7024  O    HOH A 402    10.290  -1.519  55.797  1.00 48.61        O
HETATM 7025  O    HOH A 403    12.830   2.240  62.323  1.00 38.53        O
HETATM 7026  O    HOH A 404    11.626 -10.217  50.538  1.00 34.85        O
HETATM 7027  O    HOH A 405     6.842  -9.963  76.172  1.00 32.38        O
HETATM 7028  O    HOH A 406    -1.004 -13.360  62.414  1.00 34.52        O
HETATM 7029  O    HOH A 407    33.350 -17.175  67.736  1.00 42.97        O
HETATM 7030  O    HOH A 408   -19.828 -23.717  31.044  1.00 31.64        O
HETATM 7031  O    HOH A 409    -7.619 -33.300  23.053  1.00 35.12        O
HETATM 7032  O    HOH B 218    19.673 -19.430  60.675  1.00 17.08        O
HETATM 7033  O    HOH B 219    27.319 -26.479  51.011  1.00 17.98        O
HETATM 7034  O    HOH B 220    24.465 -20.793  59.117  1.00 19.18        O
HETATM 7035  O    HOH B 221    19.132 -15.343  40.077  1.00 22.75        O
HETATM 7036  O    HOH B 222    17.836  -3.049  59.835  1.00 23.07        O
HETATM 7037  O    HOH B 223    36.635 -12.742  48.794  1.00 20.65        O
HETATM 7038  O    HOH B 224    40.129  -8.999  53.488  1.00 21.01        O
HETATM 7039  O    HOH B 225    10.639 -12.271  39.779  1.00 25.05        O
HETATM 7040  O    HOH B 226     6.437 -21.513  33.258  1.00 25.16        O
HETATM 7041  O    HOH B 227    34.473 -23.259  41.252  1.00 21.29        O
HETATM 7042  O    HOH B 228    18.578  -2.457  57.303  1.00 20.75        O
HETATM 7043  O    HOH B 229    19.350 -20.032  53.023  1.00 20.00        O
HETATM 7044  O    HOH B 230     2.650 -17.360  38.107  1.00 22.25        O
HETATM 7045  O    HOH B 231    17.283 -12.271  36.034  1.00 17.67        O
HETATM 7046  O    HOH B 232    24.443 -29.240  42.553  1.00 21.66        O
HETATM 7047  O    HOH B 233    27.558  -3.184  45.061  1.00 21.35        O
HETATM 7048  O    HOH B 234    32.745 -12.441  64.114  1.00 24.04        O
HETATM 7049  O    HOH B 235    18.143 -16.783  43.656  1.00 19.62        O
HETATM 7050  O    HOH B 236    20.881  -0.712  53.393  1.00 21.88        O
HETATM 7051  O    HOH B 237    28.277 -23.390  61.249  1.00 22.28        O
HETATM 7052  O    HOH B 238    12.768  -8.624  29.284  1.00 21.80        O
HETATM 7053  O    HOH B 239    19.531   3.465  53.791  1.00 24.88        O
HETATM 7054  O    HOH B 240    24.393  -3.962  66.692  1.00 19.11        O
HETATM 7055  O    HOH B 241    15.849 -18.627  39.643  1.00 24.09        O
HETATM 7056  O    HOH B 242    21.307 -20.957  49.483  1.00 20.50        O
HETATM 7057  O    HOH B 243    20.042  -6.059  25.787  1.00 25.11        O
HETATM 7058  O    HOH B 244    33.961 -27.406  40.826  1.00 24.91        O
HETATM 7059  O    HOH B 245    19.775  -9.676  42.020  1.00 22.36        O
HETATM 7060  O    HOH B 246    15.891 -22.554  30.883  1.00 26.05        O
HETATM 7061  O    HOH B 247    36.437   2.397  55.390  1.00 24.78        O
HETATM 7062  O    HOH B 248    39.158 -18.431  58.680  1.00 25.34        O
HETATM 7063  O    HOH B 249    25.943   1.934  49.779  1.00 23.74        O
HETATM 7064  O    HOH B 250    36.987  -6.034  66.640  1.00 21.93        O
HETATM 7065  O    HOH B 251     8.167 -14.651  41.485  1.00 22.34        O
HETATM 7066  O    HOH B 252    38.914  -2.892  59.098  1.00 27.89        O
HETATM 7067  O    HOH B 253    23.229 -25.066  54.236  1.00 21.35        O
HETATM 7068  O    HOH B 254    36.117 -21.145  53.652  1.00 24.64        O
HETATM 7069  O    HOH B 255    16.776  -8.408  46.223  1.00 31.72        O
HETATM 7070  O    HOH B 256    39.181 -16.931  54.918  1.00 24.94        O
HETATM 7071  O    HOH B 257    20.615  -0.920  48.973  1.00 31.74        O
HETATM 7072  O    HOH B 258    18.763 -24.130  40.875  1.00 25.72        O
HETATM 7073  O    HOH B 259    26.034 -24.551  58.256  1.00 22.89        O
HETATM 7074  O    HOH B 260    25.877  -1.158  64.165  1.00 32.65        O
HETATM 7075  O    HOH B 261    34.688 -20.130  61.310  1.00 26.30        O
```

```
HETATM 7076  O   HOH B 262    22.948 -10.900  30.124  1.00 23.45           O
HETATM 7077  O   HOH B 263    25.289 -24.760  50.587  1.00 24.19           O
HETATM 7078  O   HOH B 264    16.140  -4.707  26.234  1.00 28.49           O
HETATM 7079  O   HOH B 265    22.043  -7.681  41.892  1.00 28.50           O
HETATM 7080  O   HOH B 266    39.950 -13.112  52.985  1.00 29.42           O
HETATM 7081  O   HOH B 267    -5.117  -5.453  39.587  1.00 33.34           O
HETATM 7082  O   HOH B 268    33.676   4.085  54.760  1.00 28.54           O
HETATM 7083  O   HOH B 269    18.703 -30.188  46.077  1.00 25.72           O
HETATM 7084  O   HOH B 270    21.026 -16.377  27.377  1.00 32.53           O
HETATM 7085  O   HOH B 271    24.386  -8.561  38.432  1.00 31.04           O
HETATM 7086  O   HOH B 272    23.282  -3.392  42.852  1.00 31.70           O
HETATM 7087  O   HOH B 273    29.245   7.685  53.215  1.00 23.87           O
HETATM 7088  O   HOH B 274    41.422 -12.468  56.292  1.00 31.84           O
HETATM 7089  O   HOH B 275    16.878 -26.946  31.153  1.00 43.37           O
HETATM 7090  O   HOH B 276    -4.803   1.726  40.144  1.00 46.65           O
HETATM 7091  O   HOH B 277    15.492 -14.107  42.399  1.00 30.78           O
HETATM 7092  O   HOH B 278    18.675 -22.988  47.393  1.00 22.63           O
HETATM 7093  O   HOH B 279    35.628  -0.382  65.524  1.00 29.36           O
HETATM 7094  O   HOH B 280    39.564 -16.618  62.141  1.00 36.87           O
HETATM 7095  O   HOH B 281    23.187 -22.540  33.210  1.00 26.16           O
HETATM 7096  O   HOH B 282    37.033 -18.692  61.909  1.00 29.11           O
HETATM 7097  O   HOH B 283     2.489  -6.882  43.294  1.00 35.15           O
HETATM 7098  O   HOH B 284     6.544 -12.273  41.022  1.00 29.23           O
HETATM 7099  O   HOH B 285    41.004   1.997  46.467  1.00 39.52           O
HETATM 7100  O   HOH B 286    20.721 -11.820  39.818  1.00 41.28           O
HETATM 7101  O   HOH B 287    11.563 -26.821  36.133  1.00 37.23           O
HETATM 7102  O   HOH B 288    38.393 -13.498  44.721  1.00 26.62           O
HETATM 7103  O   HOH B 289    34.511 -27.420  47.873  1.00 33.97           O
HETATM 7104  O   HOH B 290    27.901 -28.083  35.574  1.00 27.09           O
HETATM 7105  O   HOH B 291     0.699 -25.446  32.490  1.00 22.53           O
HETATM 7106  O   HOH B 292    24.504   6.230  52.973  1.00 23.89           O
HETATM 7107  O   HOH B 293    17.651  -5.832  32.356  1.00 31.17           O
HETATM 7108  O   HOH B 294    22.397 -25.396  51.558  1.00 23.94           O
HETATM 7109  O   HOH B 295     8.338 -10.351  39.909  1.00 29.23           O
HETATM 7110  O   HOH B 296    18.900  -0.420  62.213  1.00 23.96           O
HETATM 7111  O   HOH B 297    23.784 -30.173  39.857  1.00 30.55           O
HETATM 7112  O   HOH B 298    10.254  -3.189  35.243  1.00 28.79           O
HETATM 7113  O   HOH B 299    30.390 -21.408  62.288  1.00 28.50           O
HETATM 7114  O   HOH B 300    25.444 -22.691  60.930  1.00 28.79           O
HETATM 7115  O   HOH B 301    35.600   4.358  50.868  1.00 28.93           O
HETATM 7116  O   HOH B 302    34.196 -16.338  40.290  1.00 35.19           O
HETATM 7117  O   HOH B 303    20.599   0.104  56.018  1.00 33.52           O
HETATM 7118  O   HOH B 304    11.454  -9.834  26.931  1.00 25.24           O
HETATM 7119  O   HOH B 305    19.467 -13.273  37.624  1.00 27.15           O
HETATM 7120  O   HOH B 306    41.021  -3.505  53.304  1.00 33.11           O
HETATM 7121  O   HOH B 307     4.839  -5.086  40.101  1.00 31.46           O
HETATM 7122  O   HOH B 308    39.170  -4.387  45.939  1.00 35.71           O
HETATM 7123  O   HOH B 309    20.824 -27.548  37.310  1.00 27.77           O
HETATM 7124  O   HOH B 310    17.376  -4.945  34.615  1.00 33.37           O
HETATM 7125  O   HOH B 311    41.482  -0.250  57.143  1.00 34.18           O
HETATM 7126  O   HOH B 312     6.944 -21.375  29.961  1.00 32.65           O
HETATM 7127  O   HOH B 313    30.112  -7.028  42.424  1.00 34.59           O
HETATM 7128  O   HOH B 314    24.201 -22.797  57.415  1.00 30.05           O
HETATM 7129  O   HOH B 315     0.789 -27.433  34.015  1.00 35.64           O
HETATM 7130  O   HOH B 316    28.989 -28.191  47.996  1.00 28.16           O
HETATM 7131  O   HOH B 317    41.302  -1.569  62.582  1.00 34.00           O
HETATM 7132  O   HOH B 318    40.325  -5.008  67.156  1.00 32.15           O
HETATM 7133  O   HOH B 319    12.652 -25.665  28.761  1.00 31.70           O
HETATM 7134  O   HOH B 320    32.194 -19.612  63.044  1.00 35.81           O
HETATM 7135  O   HOH B 321    29.303   3.445  65.477  1.00 31.50           O
HETATM 7136  O   HOH B 322    16.899 -17.130  24.630  1.00 29.10           O
HETATM 7137  O   HOH B 323     4.632  -0.005  38.496  1.00 37.73           O
HETATM 7138  O   HOH B 324     8.158  -7.517  39.673  1.00 32.35           O
HETATM 7139  O   HOH B 325    40.960   0.017  60.048  1.00 40.11           O
HETATM 7140  O   HOH B 326    29.527 -28.765  45.273  1.00 30.81           O
```

```
HETATM 7141  O   HOH B 327      36.431 -19.407  48.084  1.00 29.67           O
HETATM 7142  O   HOH B 328      18.235 -19.197  25.689  1.00 32.10           O
HETATM 7143  O   HOH B 329       7.246 -23.298  31.527  1.00 34.71           O
HETATM 7144  O   HOH B 330      19.281   6.089  53.179  1.00 22.10           O
HETATM 7145  O   HOH B 331      17.756 -24.977  29.550  1.00 35.08           O
HETATM 7146  O   HOH B 332      41.284 -15.462  59.840  1.00 28.23           O
HETATM 7147  O   HOH B 333      38.675 -13.836  47.542  1.00 30.03           O
HETATM 7148  O   HOH B 334      16.499 -17.700  45.553  1.00 31.42           O
HETATM 7149  O   HOH B 335      17.609 -10.034  44.440  1.00 36.18           O
HETATM 7150  O   HOH B 336      -9.315  -7.009  39.532  1.00 32.42           O
HETATM 7151  O   HOH B 337      42.151  -1.178  52.226  1.00 39.63           O
HETATM 7152  O   HOH B 338      21.148  -2.836  67.740  1.00 38.94           O
HETATM 7153  O   HOH B 339      24.537 -24.424  31.083  1.00 29.04           O
HETATM 7154  O   HOH B 340      11.742 -16.065  25.232  1.00 31.82           O
HETATM 7155  O   HOH B 341      18.855 -31.877  43.990  1.00 48.26           O
HETATM 7156  O   HOH B 342      33.245 -31.450  45.241  1.00 40.75           O
HETATM 7157  O   HOH B 343      20.807   4.197  61.845  1.00 28.73           O
HETATM 7158  O   HOH B 344      10.627 -18.723  26.446  1.00 28.55           O
HETATM 7159  O   HOH B 345       3.855 -20.326  42.056  1.00 37.79           O
HETATM 7160  O   HOH B 346      14.942 -21.875  27.130  1.00 34.42           O
HETATM 7161  O   HOH B 347      40.915   2.208  56.486  1.00 36.50           O
HETATM 7162  O   HOH B 348       7.251 -21.188  27.621  1.00 38.04           O
HETATM 7163  O   HOH B 349      37.246  -8.151  68.237  1.00 39.09           O
HETATM 7164  O   HOH B 350      23.692  -0.300  67.449  1.00 41.41           O
HETATM 7165  O   HOH B 351      15.399  -7.234  49.598  1.00 40.75           O
HETATM 7166  O   HOH B 352      15.439 -23.818  49.101  1.00 45.58           O
HETATM 7167  O   HOH B 353      41.048 -15.609  53.726  1.00 33.34           O
HETATM 7168  O   HOH B 354      18.840 -23.210  58.068  1.00 36.04           O
HETATM 7169  O   HOH B 355      13.366 -31.007  52.566  1.00 39.00           O
HETATM 7170  O   HOH B 356      20.960 -22.898  51.519  1.00 25.50           O
HETATM 7171  O   HOH B 357      39.073  -9.383  47.610  1.00 38.48           O
HETATM 7172  O   HOH B 358      18.813 -23.877  50.230  1.00 31.73           O
HETATM 7173  O   HOH B 359      22.076  -8.126  37.118  1.00 32.52           O
HETATM 7174  O   HOH B 360       7.870 -18.467  42.943  1.00 34.04           O
HETATM 7175  O   HOH B 361       4.649 -26.417  37.340  1.00 36.37           O
HETATM 7176  O   HOH B 362      20.226  -6.260  43.961  1.00 44.12           O
HETATM 7177  O   HOH B 363      -0.578   4.485  41.504  1.00 41.84           O
HETATM 7178  O   HOH B 364      40.916  -6.667  49.458  1.00 38.17           O
HETATM 7179  O   HOH B 365       4.057 -23.572  29.717  1.00 38.29           O
HETATM 7180  O   HOH B 366      40.345  -1.050  65.214  1.00 39.62           O
HETATM 7181  O   HOH B 367       6.877 -23.234  40.918  1.00 29.90           O
HETATM 7182  O   HOH B 368      29.714 -11.633  39.675  1.00 35.58           O
HETATM 7183  O   HOH B 369      30.883   4.239  50.386  1.00 36.53           O
HETATM 7184  O   HOH B 370       7.103 -18.343  45.254  1.00 42.50           O
HETATM 7185  O   HOH B 371      21.547  -0.573  68.234  1.00 47.92           O
HETATM 7186  O   HOH B 372      10.186  -9.674  24.673  1.00 38.98           O
HETATM 7187  O   HOH B 373      38.592 -25.913  49.569  1.00 33.09           O
HETATM 7188  O   HOH B 374      34.797 -14.755  68.950  1.00 35.20           O
HETATM 7189  O   HOH B 375      35.449   4.907  48.624  1.00 41.51           O
HETATM 7190  O   HOH B 376      37.069 -21.379  51.319  1.00 42.51           O
HETATM 7191  O   HOH B 377      36.295   3.720  45.531  1.00 46.20           O
HETATM 7192  O   HOH B 378       3.996 -22.480  33.148  1.00 29.41           O
HETATM 7193  O   HOH B 379      25.712   1.496  64.075  1.00 32.64           O
HETATM 7194  O   HOH B 380      43.201 -13.543  60.070  1.00 42.42           O
HETATM 7195  O   HOH B 381      32.289 -33.944  44.789  1.00 36.43           O
HETATM 7196  O   HOH B 382      22.759   1.669  64.517  1.00 31.51           O
HETATM 7197  O   HOH B 383      17.818 -24.593  45.540  1.00 31.64           O
HETATM 7198  O   HOH B 384      37.182 -21.980  55.915  1.00 39.63           O
HETATM 7199  O   HOH B 385       8.369 -14.536  43.916  1.00 41.71           O
HETATM 7200  O   HOH B 386      18.145 -26.599  39.832  1.00 44.82           O
HETATM 7201  O   HOH B 387      12.802  -2.152  25.044  1.00 44.20           O
HETATM 7202  O   HOH B 388      14.544 -23.506  57.433  1.00 44.75           O
HETATM 7203  O   HOH B 389       8.702 -29.248  34.995  1.00 36.25           O
HETATM 7204  O   HOH B 390      36.987 -28.047  46.348  1.00 48.56           O
HETATM 7205  O   HOH B 391      32.959 -34.557  42.052  1.00 31.02           O
```

144

```
HETATM 7206  O   HOH B 392     27.330 -12.275  37.389  1.00 33.50           O
HETATM 7207  O   HOH B 393     15.303 -11.072  43.626  1.00 29.12           O
HETATM 7208  O   HOH B 394     44.102  -0.976  62.656  1.00 41.66           O
HETATM 7209  O   HOH B 395     35.560   7.668  60.871  1.00 37.02           O
HETATM 7210  O   HOH B 396     15.667  -5.270  37.048  1.00 41.67           O
HETATM 7211  O   HOH B 397     39.571 -19.456  54.798  1.00 36.21           O
HETATM 7212  O   HOH B 398     15.632 -21.270  40.126  1.00 30.24           O
HETATM 7213  O   HOH B 399     33.541   2.028  46.613  1.00 36.44           O
HETATM 7214  O   HOH B 400      8.395 -17.124  26.040  1.00 37.48           O
HETATM 7215  O   HOH B 401     15.074 -23.766  28.617  1.00 43.88           O
HETATM 7216  O   HOH B 402      5.795   3.014  30.654  1.00 36.62           O
HETATM 7217  O   HOH B 403     38.346   5.410  62.015  1.00 40.81           O
HETATM 7218  O   HOH B 404     15.432 -21.775  42.765  1.00 40.40           O
HETATM 7219  O   HOH B 405     15.300 -17.893  42.457  1.00 35.00           O
HETATM 7220  O   HOH B 406     11.790 -21.927  41.705  1.00 34.93           O
HETATM 7221  O   HOH B 407     37.877   4.384  52.938  1.00 34.59           O
HETATM 7222  O   HOH B 408     16.704 -28.561  56.433  1.00 51.18           O
HETATM 7223  O   HOH B 409     10.646 -17.569  42.447  1.00 37.12           O
HETATM 7224  O   HOH B 410     37.804 -15.290  67.354  1.00 35.28           O
HETATM 7225  O   HOH B 411     16.813 -23.287  39.235  1.00 39.46           O
HETATM 7226  O   HOH B 412     17.612 -24.265  43.191  1.00 33.81           O
HETATM 7227  O   HOH B 413     44.673 -11.332  57.055  1.00 37.66           O
HETATM 7228  O   HOH B 414     38.938 -10.651  67.204  1.00 36.26           O
HETATM 7229  O   HOH B 415     34.492   7.243  63.068  1.00 43.59           O
HETATM 7230  O   HOH B 416     32.584   8.516  58.210  1.00 34.68           O
HETATM 7231  O   HOH B 417      5.813 -15.764  21.510  1.00 46.71           O
HETATM 7232  O   HOH B 418     19.044  -9.685  40.052  1.00 34.67           O
HETATM 7233  O   HOH B 419     27.331  -2.763  64.731  1.00 37.39           O
HETATM 7234  O   HOH B 420    -10.843   5.913  29.590  1.00 58.55           O
HETATM 7235  O   HOH B 421     15.885  -0.244  59.533  1.00 46.78           O
HETATM 7236  O   HOH B 422      3.461 -20.877  31.044  1.00 44.50           O
HETATM 7237  O   HOH B 423     33.677 -19.282  65.499  1.00 41.42           O
HETATM 7238  O   HOH B 424     26.264  -2.186  66.994  1.00 31.99           O
HETATM 7239  O   HOH C 215     29.901 -10.025  26.246  1.00 16.84           O
HETATM 7240  O   HOH C 216     30.914 -13.274  15.235  1.00 16.03           O
HETATM 7241  O   HOH C 217     45.055 -20.084  23.673  1.00 18.58           O
HETATM 7242  O   HOH C 218     34.517 -22.130  36.909  1.00 22.26           O
HETATM 7243  O   HOH C 219     39.765  -5.145  14.245  1.00 20.32           O
HETATM 7244  O   HOH C 220     21.857 -19.587 -12.950  1.00 22.50           O
HETATM 7245  O   HOH C 221     35.844  -4.682   4.838  1.00 20.28           O
HETATM 7246  O   HOH C 222     20.989  -2.081  -6.683  1.00 21.84           O
HETATM 7247  O   HOH C 223     32.156 -10.302  24.566  1.00 19.35           O
HETATM 7248  O   HOH C 224     38.203 -23.121  16.233  1.00 21.37           O
HETATM 7249  O   HOH C 225     29.409 -17.889  19.474  1.00 14.94           O
HETATM 7250  O   HOH C 226     31.461 -24.229  27.743  1.00 22.20           O
HETATM 7251  O   HOH C 227     29.655  -5.335 -25.388  1.00 21.17           O
HETATM 7252  O   HOH C 228     34.798  -2.749  -4.257  1.00 19.17           O
HETATM 7253  O   HOH C 229     30.189   6.010 -19.326  1.00 20.07           O
HETATM 7254  O   HOH C 230     40.873  -7.483  24.560  1.00 26.27           O
HETATM 7255  O   HOH C 231     32.698 -23.494  23.095  1.00 21.80           O
HETATM 7256  O   HOH C 232     25.103  -2.547 -25.562  1.00 23.94           O
HETATM 7257  O   HOH C 233     26.723 -14.866  36.581  1.00 21.82           O
HETATM 7258  O   HOH C 234     25.270 -15.524  27.011  1.00 23.96           O
HETATM 7259  O   HOH C 235     29.273  -7.926 -24.693  1.00 21.62           O
HETATM 7260  O   HOH C 236     32.254  -8.385  -4.580  1.00 22.48           O
HETATM 7261  O   HOH C 237     28.218  -6.697   4.459  1.00 26.32           O
HETATM 7262  O   HOH C 238     26.906 -20.286  27.258  1.00 28.31           O
HETATM 7263  O   HOH C 239     42.826  -6.543  20.497  1.00 22.44           O
HETATM 7264  O   HOH C 240     34.729  -9.964  32.103  1.00 25.18           O
HETATM 7265  O   HOH C 241     27.529  -1.125 -11.118  1.00 23.23           O
HETATM 7266  O   HOH C 242     45.054 -26.334  27.115  1.00 25.94           O
HETATM 7267  O   HOH C 243     39.762 -25.918  24.179  1.00 25.94           O
HETATM 7268  O   HOH C 244     36.553 -18.209   7.273  1.00 28.46           O
HETATM 7269  O   HOH C 245     34.534   4.031 -14.353  1.00 27.27           O
HETATM 7270  O   HOH C 246     17.067 -19.824 -14.198  1.00 24.16           O
```

```
HETATM 7271  O   HOH C 247      17.142  -6.985 -29.517  1.00 28.01           O
HETATM 7272  O   HOH C 248      32.648 -20.042  37.301  1.00 22.13           O
HETATM 7273  O   HOH C 249      32.138  -2.990   4.269  1.00 31.32           O
HETATM 7274  O   HOH C 250      37.073   2.872  -7.920  1.00 24.45           O
HETATM 7275  O   HOH C 251      30.503  -3.815   2.712  1.00 30.07           O
HETATM 7276  O   HOH C 252      36.338 -12.072  32.573  1.00 25.46           O
HETATM 7277  O   HOH C 253      34.257 -10.976   4.368  1.00 22.94           O
HETATM 7278  O   HOH C 254      24.455 -17.899 -10.321  1.00 25.48           O
HETATM 7279  O   HOH C 255      31.089  -1.948 -22.122  1.00 21.08           O
HETATM 7280  O   HOH C 256      37.628  -1.108 -19.796  1.00 28.22           O
HETATM 7281  O   HOH C 257      10.958 -17.540 -11.546  1.00 24.08           O
HETATM 7282  O   HOH C 258      33.417  -5.155   3.820  1.00 23.21           O
HETATM 7283  O   HOH C 259      40.560  -5.091  21.665  1.00 25.10           O
HETATM 7284  O   HOH C 260      37.217 -19.627   9.599  1.00 23.73           O
HETATM 7285  O   HOH C 261      33.925   3.428  -7.097  1.00 22.44           O
HETATM 7286  O   HOH C 262      19.257 -21.364 -13.145  1.00 30.38           O
HETATM 7287  O   HOH C 263      46.183  -6.633  13.780  1.00 28.15           O
HETATM 7288  O   HOH C 264      21.841 -12.284 -31.418  1.00 34.28           O
HETATM 7289  O   HOH C 265      38.061   2.589 -12.236  1.00 26.83           O
HETATM 7290  O   HOH C 266      31.055 -13.875  39.148  1.00 23.51           O
HETATM 7291  O   HOH C 267      33.806   3.594  -4.307  1.00 28.08           O
HETATM 7292  O   HOH C 268      27.914 -14.136   7.055  1.00 27.08           O
HETATM 7293  O   HOH C 269      28.776 -22.588  23.188  1.00 25.23           O
HETATM 7294  O   HOH C 270      29.596 -20.023  12.192  1.00 28.14           O
HETATM 7295  O   HOH C 271      43.417  -8.395  24.919  1.00 25.50           O
HETATM 7296  O   HOH C 272      48.582  -5.057   4.611  1.00 36.57           O
HETATM 7297  O   HOH C 273      14.375 -21.555 -14.238  1.00 37.64           O
HETATM 7298  O   HOH C 274      20.473 -22.457 -19.322  1.00 23.19           O
HETATM 7299  O   HOH C 275      47.166  -3.305   0.734  1.00 29.86           O
HETATM 7300  O   HOH C 276       9.831 -19.728 -18.328  1.00 27.92           O
HETATM 7301  O   HOH C 277      20.700 -17.092  -6.999  1.00 32.03           O
HETATM 7302  O   HOH C 278      22.702 -19.317  30.036  1.00 27.08           O
HETATM 7303  O   HOH C 279      33.003 -10.564  -3.274  1.00 27.70           O
HETATM 7304  O   HOH C 280      39.503 -12.131  -2.830  1.00 25.03           O
HETATM 7305  O   HOH C 281      35.613 -13.217   3.444  1.00 29.87           O
HETATM 7306  O   HOH C 282      28.044  -1.437  -4.991  1.00 23.12           O
HETATM 7307  O   HOH C 283      49.247 -10.522   8.186  1.00 38.31           O
HETATM 7308  O   HOH C 284      28.385 -18.650   9.592  1.00 29.68           O
HETATM 7309  O   HOH C 285      44.173 -15.537   2.171  1.00 25.28           O
HETATM 7310  O   HOH C 286      43.398  -3.989   7.752  1.00 30.70           O
HETATM 7311  O   HOH C 287      24.063 -15.015  -6.816  1.00 32.23           O
HETATM 7312  O   HOH C 288      47.600 -21.132  20.810  1.00 27.81           O
HETATM 7313  O   HOH C 289      37.342 -14.382  36.283  1.00 33.10           O
HETATM 7314  O   HOH C 290      39.409  -7.448 -16.719  1.00 41.27           O
HETATM 7315  O   HOH C 291      26.766 -15.002  -6.339  1.00 33.51           O
HETATM 7316  O   HOH C 292       8.645  -5.038 -28.046  1.00 32.65           O
HETATM 7317  O   HOH C 293      38.410 -23.029  11.714  1.00 47.41           O
HETATM 7318  O   HOH C 294      48.012 -14.677  26.281  1.00 33.77           O
HETATM 7319  O   HOH C 295      33.865 -29.204  18.911  1.00 30.18           O
HETATM 7320  O   HOH C 296      45.944  -8.751  -4.753  1.00 32.25           O
HETATM 7321  O   HOH C 297      30.569 -26.536  29.300  1.00 35.65           O
HETATM 7322  O   HOH C 298      49.341 -10.178  16.680  1.00 36.33           O
HETATM 7323  O   HOH C 299      44.012 -20.217  11.865  1.00 29.83           O
HETATM 7324  O   HOH C 300      30.798   0.514 -23.421  1.00 30.61           O
HETATM 7325  O   HOH C 301      10.934  -4.158 -22.167  1.00 34.13           O
HETATM 7326  O   HOH C 302      49.242  -7.477  -3.433  1.00 36.42           O
HETATM 7327  O   HOH C 303      47.753 -11.121  38.559  1.00 47.50           O
HETATM 7328  O   HOH C 304      17.268   1.543 -15.164  1.00 28.70           O
HETATM 7329  O   HOH C 305      12.915 -19.982 -28.559  1.00 46.26           O
HETATM 7330  O   HOH C 306      21.281 -23.020 -14.454  1.00 31.38           O
HETATM 7331  O   HOH C 307      28.638  -4.423 -27.685  1.00 21.79           O
HETATM 7332  O   HOH C 308      48.062 -23.799  20.066  1.00 29.94           O
HETATM 7333  O   HOH C 309      33.564  -9.139  34.180  1.00 27.78           O
HETATM 7334  O   HOH C 310      46.934 -13.424  32.828  1.00 33.71           O
HETATM 7335  O   HOH C 311      32.151 -27.576  25.249  1.00 31.19           O
```

```
HETATM 7336  O   HOH C 312      32.068    5.315 -22.579  1.00 24.97           O
HETATM 7337  O   HOH C 313      27.864 -13.729 -25.799  1.00 23.90           O
HETATM 7338  O   HOH C 314      17.571 -18.870 -10.067  1.00 43.04           O
HETATM 7339  O   HOH C 315      19.062 -13.948  -5.545  1.00 31.07           O
HETATM 7340  O   HOH C 316      38.068 -30.324  12.431  1.00 32.69           O
HETATM 7341  O   HOH C 317      44.542 -26.716  16.806  1.00 36.75           O
HETATM 7342  O   HOH C 318      26.952 -20.510 -27.446  1.00 31.23           O
HETATM 7343  O   HOH C 319      31.948  -8.740 -24.199  1.00 34.05           O
HETATM 7344  O   HOH C 320      18.408    0.525  -7.246  1.00 37.05           O
HETATM 7345  O   HOH C 321      18.133 -16.071  -9.068  1.00 29.17           O
HETATM 7346  O   HOH C 322      38.738  -0.031  -4.285  1.00 33.49           O
HETATM 7347  O   HOH C 323      31.132 -27.473  36.600  1.00 32.03           O
HETATM 7348  O   HOH C 324      22.309    6.765 -19.524  1.00 28.80           O
HETATM 7349  O   HOH C 325      26.648 -18.823 -18.117  1.00 33.69           O
HETATM 7350  O   HOH C 326      24.210  -7.306  -3.970  1.00 33.37           O
HETATM 7351  O   HOH C 327      25.644    0.969 -11.581  1.00 32.66           O
HETATM 7352  O   HOH C 328      21.258  -9.735  -4.993  1.00 30.94           O
HETATM 7353  O   HOH C 329      46.047 -12.328  -3.805  1.00 45.11           O
HETATM 7354  O   HOH C 330      42.794 -20.101  34.511  1.00 33.32           O
HETATM 7355  O   HOH C 331      31.830 -11.948   1.280  1.00 32.69           O
HETATM 7356  O   HOH C 332      22.666 -18.455  27.673  1.00 34.16           O
HETATM 7357  O   HOH C 333      44.957 -10.020  27.873  1.00 32.60           O
HETATM 7358  O   HOH C 334      29.012 -20.863 -10.118  1.00 27.94           O
HETATM 7359  O   HOH C 335       3.567 -17.997 -21.308  1.00 34.56           O
HETATM 7360  O   HOH C 336      42.502  -2.919  -8.811  1.00 33.03           O
HETATM 7361  O   HOH C 337      31.992  -5.694 -27.145  1.00 34.19           O
HETATM 7362  O   HOH C 338      44.914  -1.961  -4.352  1.00 36.42           O
HETATM 7363  O   HOH C 339      29.535    2.072  -9.920  1.00 31.22           O
HETATM 7364  O   HOH C 340      32.542    6.824 -13.103  1.00 34.44           O
HETATM 7365  O   HOH C 341      30.078  -7.876  -2.851  1.00 37.90           O
HETATM 7366  O   HOH C 342       6.357 -24.334 -14.026  1.00 35.86           O
HETATM 7367  O   HOH C 343      32.544 -28.925  16.506  1.00 28.10           O
HETATM 7368  O   HOH C 344      32.549  -2.751 -26.411  1.00 44.62           O
HETATM 7369  O   HOH C 345      28.666 -24.738  17.437  1.00 39.22           O
HETATM 7370  O   HOH C 346      36.264 -20.600  12.737  1.00 33.50           O
HETATM 7371  O   HOH C 347      47.381  -9.166  10.219  1.00 37.03           O
HETATM 7372  O   HOH C 348      46.200 -21.540  12.619  1.00 42.79           O
HETATM 7373  O   HOH C 349      24.393 -19.546  -8.208  1.00 37.93           O
HETATM 7374  O   HOH C 350      31.550 -18.920  -9.601  1.00 38.27           O
HETATM 7375  O   HOH C 351      17.811  -9.072 -31.588  1.00 31.84           O
HETATM 7376  O   HOH C 352      28.512 -12.034   3.543  1.00 49.42           O
HETATM 7377  O   HOH C 353      28.114 -15.958 -22.278  1.00 39.95           O
HETATM 7378  O   HOH C 354      29.970 -20.773   8.883  1.00 46.92           O
HETATM 7379  O   HOH C 355      38.678 -10.828  33.353  1.00 27.34           O
HETATM 7380  O   HOH C 356      35.435 -13.630  34.856  1.00 33.82           O
HETATM 7381  O   HOH C 357      30.442  -1.357   1.100  1.00 31.83           O
HETATM 7382  O   HOH C 358       4.980 -21.944 -19.532  1.00 47.98           O
HETATM 7383  O   HOH C 359       9.356  -4.875 -25.225  1.00 32.50           O
HETATM 7384  O   HOH C 360      24.578 -18.152  -6.004  1.00 43.04           O
HETATM 7385  O   HOH C 361      36.001  -7.661 -23.067  1.00 38.45           O
HETATM 7386  O   HOH C 362      25.934  -4.655 -28.394  1.00 28.34           O
HETATM 7387  O   HOH C 363      26.208    0.626 -24.285  1.00 39.06           O
HETATM 7388  O   HOH C 364      27.421 -22.513  25.620  1.00 36.28           O
HETATM 7389  O   HOH C 365      24.988  -2.812 -30.102  1.00 37.57           O
HETATM 7390  O   HOH C 366      44.337 -17.093  -0.029  1.00 35.90           O
HETATM 7391  O   HOH C 367      33.997 -23.942  14.452  1.00 31.38           O
HETATM 7392  O   HOH C 368      43.210 -16.977  34.933  1.00 32.61           O
HETATM 7393  O   HOH C 369      43.179    0.707   1.190  1.00 48.55           O
HETATM 7394  O   HOH C 370       9.191 -13.368 -18.567  1.00 42.53           O
HETATM 7395  O   HOH C 371      28.511 -10.030  35.887  1.00 32.60           O
HETATM 7396  O   HOH C 372      28.914 -14.671 -15.476  1.00 31.91           O
HETATM 7397  O   HOH C 373      35.442 -10.324 -16.503  1.00 34.44           O
HETATM 7398  O   HOH C 374       8.910 -16.587 -30.685  1.00 39.27           O
HETATM 7399  O   HOH C 375      36.433 -22.686  14.351  1.00 39.35           O
HETATM 7400  O   HOH C 376      40.921  -7.529 -14.730  1.00 41.52           O
```

EP 2 123 668 A1

```
HETATM 7401  O   HOH C 377    26.888    5.728 -26.095  1.00 31.50           O
HETATM 7402  O   HOH C 378     9.437  -17.494 -28.300  1.00 41.29           O
HETATM 7403  O   HOH C 379    41.963  -25.668  27.230  1.00 34.02           O
HETATM 7404  O   HOH C 380    37.408    3.879 -10.415  1.00 44.93           O
HETATM 7405  O   HOH C 381    39.083  -25.436  12.815  1.00 45.85           O
HETATM 7406  O   HOH C 382    40.490    2.585 -17.316  1.00 37.13           O
HETATM 7407  O   HOH C 383    30.746  -25.143  25.100  1.00 36.03           O
HETATM 7408  O   HOH C 384    31.425   -5.244  30.958  1.00 34.00           O
HETATM 7409  O   HOH C 385    26.112  -22.500 -12.983  1.00 45.18           O
HETATM 7410  O   HOH C 386    21.725    1.475 -25.881  1.00 37.19           O
HETATM 7411  O   HOH C 387    28.037  -21.724 -14.701  1.00 39.43           O
HETATM 7412  O   HOH C 388    17.567  -18.074 -32.206  1.00 34.18           O
HETATM 7413  O   HOH C 389    43.963   -9.480  29.794  1.00 34.90           O
HETATM 7414  O   HOH C 390    25.382  -21.188  29.581  1.00 35.14           O
HETATM 7415  O   HOH C 391     7.717   -6.080 -32.015  1.00 39.77           O
HETATM 7416  O   HOH C 392    21.133   -7.522  -1.455  1.00 40.65           O
HETATM 7417  O   HOH C 393    49.016  -12.885  31.256  1.00 35.54           O
HETATM 7418  O   HOH C 394    33.581  -26.818  32.971  1.00 40.34           O
HETATM 7419  O   HOH C 395    47.968  -22.617  17.712  1.00 34.26           O
HETATM 7420  O   HOH C 396    39.906   -4.643 -20.099  1.00 36.62           O
HETATM 7421  O   HOH C 397    29.004   -1.180  30.060  1.00 36.95           O
HETATM 7422  O   HOH C 398     7.751  -21.343 -11.969  1.00 33.78           O
HETATM 7423  O   HOH C 399    14.744   -3.009 -29.146  1.00 35.25           O
HETATM 7424  O   HOH C 400    30.440  -23.683  16.173  1.00 38.44           O
HETATM 7425  O   HOH C 401    19.418    8.006 -18.260  1.00 37.95           O
HETATM 7426  O   HOH C 402    22.221  -21.275  25.800  1.00 46.72           O
HETATM 7427  O   HOH C 403    32.506  -26.191  12.707  1.00 46.29           O
HETATM 7428  O   HOH C 404    35.459    1.757   3.298  1.00 37.87           O
HETATM 7429  O   HOH C 405    42.175   -1.848   9.039  1.00 39.88           O
HETATM 7430  O   HOH C 406    41.546   -0.529  -3.927  1.00 44.79           O
HETATM 7431  O   HOH C 407    28.107  -16.929 -15.881  1.00 35.73           O
HETATM 7432  O   HOH C 408    36.252  -29.402  39.405  1.00 44.61           O
HETATM 7433  O   HOH C 409    45.202  -18.424   3.692  1.00 45.97           O
HETATM 7434  O   HOH C 410    36.645  -24.934  12.427  1.00 42.55           O
HETATM 7435  O   HOH C 411    17.533    9.195 -16.074  1.00 34.01           O
HETATM 7436  O   HOH C 412    30.094  -25.786  31.817  1.00 43.58           O
HETATM 7437  O   HOH C 413    11.922  -16.446 -28.252  1.00 41.81           O
HETATM 7438  O   HOH C 414    43.642  -23.572  33.418  1.00 36.64           O
HETATM 7439  O   HOH C 415    48.608  -16.863   8.018  1.00 31.01           O
HETATM 7440  O   HOH C 416    44.789  -21.392  33.644  1.00 40.95           O
HETATM 7441  O   HOH C 417    46.875  -21.202  14.809  1.00 37.59           O
HETATM 7442  O   HOH C 418    30.585  -27.327  16.169  1.00 46.64           O
HETATM 7443  O   HOH C 419    32.871    2.517 -22.431  1.00 40.60           O
HETATM 7444  O   HOH C 420    23.988    1.611 -12.831  1.00 43.03           O
HETATM 7445  O   HOH C 421    33.997    6.342 -23.785  1.00 42.82           O
HETATM 7446  O   HOH C 422    31.386  -11.688  -1.213  1.00 41.17           O
HETATM 7447  O   HOH C 423    27.092   -0.677  -2.708  1.00 35.44           O
HETATM 7448  O   HOH C 424    41.268   -9.774  36.177  1.00 41.21           O
HETATM 7449  O   HOH C 425    42.732  -11.419  -6.073  1.00 41.30           O
HETATM 7450  O   HOH C 426    41.611   -0.596   3.808  1.00 39.81           O
HETATM 7451  O   HOH C 427     6.621  -10.378 -34.798  1.00 49.57           O
HETATM 7452  O   HOH C 428    34.552  -13.338  -5.991  1.00 41.38           O
HETATM 7453  O   HOH C 429    37.020  -17.577   3.646  1.00 43.24           O
HETATM 7454  O   HOH D 218    18.243  -14.896  23.868  1.00 20.31           O
HETATM 7455  O   HOH D 219    26.668  -12.408  12.855  1.00 19.76           O
HETATM 7456  O   HOH D 220    28.732  -11.573  14.424  1.00 22.34           O
HETATM 7457  O   HOH D 221    13.915  -17.653 -11.689  1.00 20.67           O
HETATM 7458  O   HOH D 222    39.222   -2.744  13.010  1.00 20.94           O
HETATM 7459  O   HOH D 223     3.540   -0.540   1.207  1.00 29.37           O
HETATM 7460  O   HOH D 224    15.011   -0.481 -12.574  1.00 22.75           O
HETATM 7461  O   HOH D 225    14.034  -13.788  10.387  1.00 24.75           O
HETATM 7462  O   HOH D 226    35.334   -1.154  12.439  1.00 23.04           O
HETATM 7463  O   HOH D 227    -4.789  -10.576  -2.684  1.00 26.48           O
HETATM 7464  O   HOH D 228    28.026  -20.234  18.914  1.00 23.52           O
HETATM 7465  O   HOH D 229    12.266   -7.209  14.121  1.00 27.90           O
```

148

```
HETATM 7466  O   HOH D 230    31.054  -1.094  28.245  1.00 24.74           O
HETATM 7467  O   HOH D 231    10.461 -11.558   8.418  1.00 26.86           O
HETATM 7468  O   HOH D 232    -7.633  -7.291  -7.938  1.00 28.78           O
HETATM 7469  O   HOH D 233    13.936  -0.548  -9.984  1.00 28.70           O
HETATM 7470  O   HOH D 234    35.049   6.043  12.911  1.00 37.55           O
HETATM 7471  O   HOH D 235    30.810  -3.532   6.564  1.00 22.82           O
HETATM 7472  O   HOH D 236    19.174  -7.390  10.713  1.00 28.80           O
HETATM 7473  O   HOH D 237    28.107  -1.850   8.128  1.00 29.42           O
HETATM 7474  O   HOH D 238    24.572 -18.855  23.658  1.00 35.40           O
HETATM 7475  O   HOH D 239     9.393  -2.948 -19.656  1.00 25.79           O
HETATM 7476  O   HOH D 240    22.310 -17.838   9.127  1.00 24.96           O
HETATM 7477  O   HOH D 241    12.932 -13.367   4.766  1.00 30.38           O
HETATM 7478  O   HOH D 242    25.393  -0.233  11.393  1.00 28.67           O
HETATM 7479  O   HOH D 243    22.985 -17.112  17.537  1.00 32.46           O
HETATM 7480  O   HOH D 244    21.640  -9.367   9.143  1.00 22.80           O
HETATM 7481  O   HOH D 245    -5.957  -1.786 -14.239  1.00 35.21           O
HETATM 7482  O   HOH D 246    13.260 -17.565  -3.800  1.00 36.66           O
HETATM 7483  O   HOH D 247    -5.618 -17.463 -12.229  1.00 31.29           O
HETATM 7484  O   HOH D 248    44.145   9.483  18.110  1.00 29.98           O
HETATM 7485  O   HOH D 249    14.597  -5.536   1.652  1.00 38.10           O
HETATM 7486  O   HOH D 250    24.769   2.165  28.348  1.00 33.41           O
HETATM 7487  O   HOH D 251    29.236   0.562   7.552  1.00 32.51           O
HETATM 7488  O   HOH D 252     8.737 -19.191 -10.699  1.00 26.27           O
HETATM 7489  O   HOH D 253    -5.854 -11.122   4.779  1.00 39.71           O
HETATM 7490  O   HOH D 254    13.731 -18.648  18.853  1.00 27.12           O
HETATM 7491  O   HOH D 255    -9.791 -12.454 -15.019  1.00 41.90           O
HETATM 7492  O   HOH D 256    -6.322 -14.937  -8.712  1.00 29.38           O
HETATM 7493  O   HOH D 257    13.955 -20.616  11.605  1.00 30.93           O
HETATM 7494  O   HOH D 258    -1.060 -10.349   7.379  1.00 33.38           O
HETATM 7495  O   HOH D 259    23.373   7.421  20.666  1.00 34.64           O
HETATM 7496  O   HOH D 260    21.124 -13.660  27.787  1.00 26.41           O
HETATM 7497  O   HOH D 261    24.710 -21.154  11.101  1.00 41.60           O
HETATM 7498  O   HOH D 262    33.471  -6.445  26.389  1.00 21.12           O
HETATM 7499  O   HOH D 263    33.449   0.597   7.275  1.00 28.15           O
HETATM 7500  O   HOH D 264    24.867 -18.591  18.575  1.00 31.75           O
HETATM 7501  O   HOH D 265    40.849  12.720  19.174  1.00 40.10           O
HETATM 7502  O   HOH D 266    25.952   4.452  12.765  1.00 33.25           O
HETATM 7503  O   HOH D 267    38.365  -1.032  27.798  1.00 32.55           O
HETATM 7504  O   HOH D 268    27.628   0.946  29.521  1.00 27.75           O
HETATM 7505  O   HOH D 269    31.735   5.919  25.488  1.00 26.33           O
HETATM 7506  O   HOH D 270    17.925 -12.367  25.012  1.00 21.18           O
HETATM 7507  O   HOH D 271    33.573  -5.063  28.865  1.00 24.53           O
HETATM 7508  O   HOH D 272    -2.908  -5.699 -21.017  1.00 31.74           O
HETATM 7509  O   HOH D 273    41.733  -0.599  15.746  1.00 32.10           O
HETATM 7510  O   HOH D 274    40.263  -2.708  20.580  1.00 30.51           O
HETATM 7511  O   HOH D 275     5.411   9.154 -14.594  1.00 33.06           O
HETATM 7512  O   HOH D 276    21.084 -15.621  24.457  1.00 29.67           O
HETATM 7513  O   HOH D 277    25.202 -18.307  26.298  1.00 23.90           O
HETATM 7514  O   HOH D 278    28.541  -4.839   6.638  1.00 29.24           O
HETATM 7515  O   HOH D 279    28.126   2.854   8.796  1.00 33.20           O
HETATM 7516  O   HOH D 280     1.280 -11.498 -17.397  1.00 30.03           O
HETATM 7517  O   HOH D 281    -4.742 -17.260  -1.229  1.00 31.55           O
HETATM 7518  O   HOH D 282    23.632  -6.939   9.689  1.00 34.41           O
HETATM 7519  O   HOH D 283    19.077   1.111  15.965  1.00 36.46           O
HETATM 7520  O   HOH D 284    -8.465 -10.269 -10.082  1.00 35.91           O
HETATM 7521  O   HOH D 285    21.969 -17.598  23.133  1.00 31.48           O
HETATM 7522  O   HOH D 286    33.246  -2.266  29.491  1.00 34.72           O
HETATM 7523  O   HOH D 287    27.244 -16.032  19.260  1.00 32.02           O
HETATM 7524  O   HOH D 288     0.871  -8.458   6.823  1.00 41.54           O
HETATM 7525  O   HOH D 289    -1.052 -19.015 -14.210  1.00 33.90           O
HETATM 7526  O   HOH D 290    -4.121 -13.080   6.181  1.00 41.07           O
HETATM 7527  O   HOH D 291    46.718   9.817  19.794  1.00 29.14           O
HETATM 7528  O   HOH D 292    13.124 -20.056   1.512  1.00 29.22           O
HETATM 7529  O   HOH D 293    42.341   0.114  13.006  1.00 29.61           O
HETATM 7530  O   HOH D 294     5.331 -21.920 -13.008  1.00 35.22           O
```

```
HETATM 7531  O    HOH D 295   41.659  -1.527  18.796  1.00 36.44          O
HETATM 7532  O    HOH D 296   22.600 -17.068  20.793  1.00 24.35          O
HETATM 7533  O    HOH D 297    7.031   8.782  -6.410  1.00 31.53          O
HETATM 7534  O    HOH D 298   21.981  -2.359  10.861  1.00 38.64          O
HETATM 7535  O    HOH D 299   10.793 -18.440  -0.979  1.00 32.98          O
HETATM 7536  O    HOH D 300   26.494   2.405  11.024  1.00 36.16          O
HETATM 7537  O    HOH D 301   -1.732   0.448 -20.205  1.00 32.80          O
HETATM 7538  O    HOH D 302   31.164   9.093  10.718  1.00 29.52          O
HETATM 7539  O    HOH D 303   -7.153  -7.162  -1.840  1.00 35.00          O
HETATM 7540  O    HOH D 304   40.269   0.591  27.952  1.00 32.64          O
HETATM 7541  O    HOH D 305   10.831 -23.663  11.557  1.00 37.69          O
HETATM 7542  O    HOH D 306    1.385 -18.343 -15.656  1.00 38.60          O
HETATM 7543  O    HOH D 307   -3.623 -19.279  -6.707  1.00 35.49          O
HETATM 7544  O    HOH D 308   26.861  -2.859   6.050  1.00 33.34          O
HETATM 7545  O    HOH D 309   10.854   6.265 -14.532  1.00 37.77          O
HETATM 7546  O    HOH D 310    7.099 -18.498  14.972  1.00 32.36          O
HETATM 7547  O    HOH D 311   10.236   1.239 -20.628  1.00 38.67          O
HETATM 7548  O    HOH D 312   29.760   7.702   6.682  1.00 43.34          O
HETATM 7549  O    HOH D 313   -6.905 -11.944  -1.786  1.00 32.37          O
HETATM 7550  O    HOH D 314   -7.992  -2.408  -1.027  1.00 39.59          O
HETATM 7551  O    HOH D 315   45.833   9.310  22.592  1.00 40.34          O
HETATM 7552  O    HOH D 316   -3.228 -20.192   6.478  1.00 35.51          O
HETATM 7553  O    HOH D 317    6.920 -25.812   6.573  1.00 33.81          O
HETATM 7554  O    HOH D 318   23.247 -14.059  25.617  1.00 29.77          O
HETATM 7555  O    HOH D 319   11.450 -20.610  -2.493  1.00 40.63          O
HETATM 7556  O    HOH D 320   45.773   1.336  14.918  1.00 47.43          O
HETATM 7557  O    HOH D 321   17.509  -5.866  24.124  1.00 37.82          O
HETATM 7558  O    HOH D 322   18.401 -22.643  13.550  1.00 35.70          O
HETATM 7559  O    HOH D 323    3.256 -20.477 -14.258  1.00 39.21          O
HETATM 7560  O    HOH D 324    9.482   8.612 -14.468  1.00 30.46          O
HETATM 7561  O    HOH D 325   43.014  12.236  24.249  1.00 33.97          O
HETATM 7562  O    HOH D 326    5.556   4.533  -3.425  1.00 41.35          O
HETATM 7563  O    HOH D 327   19.018   1.397  26.585  1.00 38.08          O
HETATM 7564  O    HOH D 328   25.008   0.462  30.067  1.00 47.81          O
HETATM 7565  O    HOH D 329   21.536 -20.010  19.866  1.00 46.49          O
HETATM 7566  O    HOH D 330   27.325   8.094  22.361  1.00 43.42          O
HETATM 7567  O    HOH D 331   33.312  -1.717  32.031  1.00 37.70          O
HETATM 7568  O    HOH D 332   40.795   9.181  24.941  1.00 44.56          O
HETATM 7569  O    HOH D 333   22.110  -9.274   6.279  1.00 37.68          O
HETATM 7570  O    HOH D 334   12.457 -24.152  13.950  1.00 46.61          O
HETATM 7571  O    HOH D 335   14.094 -14.902   6.776  1.00 31.30          O
HETATM 7572  O    HOH D 336   27.031 -21.518  21.085  1.00 31.98          O
HETATM 7573  O    HOH D 337    0.692   6.492  -4.508  1.00 41.69          O
HETATM 7574  O    HOH D 338   36.968   5.968  10.444  1.00 37.71          O
HETATM 7575  O    HOH D 339    0.736 -12.873   9.478  1.00 36.23          O
HETATM 7576  O    HOH D 340   21.562  -5.275  10.844  1.00 39.83          O
HETATM 7577  O    HOH D 341    7.818 -14.535  21.948  1.00 33.18          O
HETATM 7578  O    HOH D 342    6.829 -23.051  -9.651  1.00 36.30          O
HETATM 7579  O    HOH D 343   33.215   7.563  27.211  1.00 30.16          O
HETATM 7580  O    HOH D 344   30.987  12.766  23.784  1.00 40.53          O
HETATM 7581  O    HOH D 345    5.686 -18.198  24.991  1.00 36.45          O
HETATM 7582  O    HOH D 346   25.208   7.112  25.287  1.00 37.06          O
HETATM 7583  O    HOH D 347   18.101 -16.519   8.410  1.00 39.60          O
HETATM 7584  O    HOH D 348    9.013  -4.723   5.112  1.00 35.72          O
HETATM 7585  O    HOH D 349   -4.172 -20.375  -9.114  1.00 39.83          O
HETATM 7586  O    HOH D 350    7.613  -0.777   4.501  1.00 44.35          O
HETATM 7587  O    HOH D 351   -4.392  -1.262   1.617  1.00 47.21          O
HETATM 7588  O    HOH D 352   35.093   8.729  13.376  1.00 38.46          O
HETATM 7589  O    HOH D 353   13.622  -6.862   3.339  1.00 36.75          O
HETATM 7590  O    HOH D 354   -8.787  -2.050  -8.470  1.00 42.21          O
HETATM 7591  O    HOH D 355   13.309 -23.233  17.874  1.00 39.41          O
HETATM 7592  O    HOH D 356    7.496 -11.463  15.522  1.00 34.13          O
HETATM 7593  O    HOH D 357   -6.122  -4.475 -22.119  1.00 46.62          O
HETATM 7594  O    HOH D 358   12.384 -20.548   7.977  1.00 38.66          O
HETATM 7595  O    HOH D 359   12.430  -2.097 -20.631  1.00 44.37          O
```

```
HETATM 7596  O    HOH D 360    -7.084 -11.473   0.893  1.00 41.69           O
HETATM 7597  O    HOH D 361    25.051  -5.826   7.862  1.00 34.44           O
HETATM 7598  O    HOH D 362     7.938   9.625 -12.049  1.00 37.96           O
HETATM 7599  O    HOH D 363     7.741 -10.156  13.042  1.00 39.36           O
HETATM 7600  O    HOH D 364    10.716 -10.123  10.612  1.00 45.20           O
HETATM 7601  O    HOH D 365    14.058 -20.955  -1.004  1.00 45.15           O
HETATM 7602  O    HOH D 366    11.554  -6.379   5.272  1.00 36.88           O
HETATM 7603  O    HOH E  13    29.241  -5.157  63.058  1.00 22.22           O
HETATM 7604  O    HOH E  14    34.779 -13.469  73.353  1.00 29.92           O
HETATM 7605  O    HOH E  15    35.966  -1.619  67.747  1.00 40.27           O
HETATM 7606  O    HOH E  16    37.661  -3.567  67.749  1.00 23.66           O
HETATM 7607  O    HOH E  17    34.527  -7.679  76.799  1.00 33.77           O
HETATM 7608  O    HOH E  18    38.876 -13.584  69.273  1.00 49.31           O
HETATM 7609  O    HOH E  19    30.505 -15.714  74.628  1.00 30.39           O
HETATM 7610  O    HOH E  20    35.626   2.528  65.968  1.00 41.64           O
HETATM 7611  O    HOH E  21    24.548  -2.802  70.944  1.00 30.76           O
HETATM 7612  O    HOH E  22    36.788  -7.133  70.334  1.00 38.94           O
HETATM 7613  O    HOH E  23    37.503  -4.513  70.326  1.00 40.96           O
HETATM 7614  O    HOH F  13     4.302  -3.749 -16.456  1.00 22.95           O
HETATM 7615  O    HOH F  14     0.408 -13.626 -26.534  1.00 44.23           O
HETATM 7616  O    HOH F  15     9.919  -2.520 -24.064  1.00 33.69           O
HETATM 7617  O    HOH F  16    -3.475  -3.373 -22.474  1.00 35.46           O
HETATM 7618  O    HOH F  17    11.137  -0.648 -26.633  1.00 47.51           O
HETATM 7619  O    HOH F  18     3.696 -11.140 -31.860  1.00 49.85           O
HETATM 7620  O    HOH F  19     1.137  -8.400 -30.631  1.00 52.49           O
HETATM 7621  O    HOH F  20    -1.826  -7.136 -24.456  1.00 46.11           O
HETATM 7622  O    HOH F  21    -1.114   3.268 -21.038  1.00 37.81           O
HETATM 7623  O    HOH F  22    -2.935   5.112 -21.305  1.00 40.33           O
HETATM 7624  O    HOH F  23    -0.372  -1.962 -29.080  1.00 38.59           O
HETATM 7625  O    HOH F  24     7.277  -2.026 -21.392  1.00 43.40           O
END
```

## TABLE 14

## Crystal 2- 3C5S

Crystal Structure of monoclonal Fab F22-4 specific for *Shigella flexneri* 2a O-Ag

```
HEADER    IMMUNE SYSTEM                          01-FEB-08   3C5S
TITLE     CRYSTAL STRUCTURE OF MONOCLONAL FAB F22-4 SPECIFIC FOR
TITLE     2 SHIGELLA FLEXNERI 2A O-AG
CRYST1    47.260    59.830    74.380  77.53  83.97   80.89 P 1           2
ATOM      1    N   ASP A   1     29.374  70.333   5.654  1.00 41.87           N
ATOM      2    CA  ASP A   1     28.972  69.590   6.893  1.00 41.15           C
ATOM      3    C   ASP A   1     30.104  69.575   7.916  1.00 38.78           C
ATOM      4    O   ASP A   1     31.266  69.677   7.556  1.00 38.59           O
ATOM      5    CB  ASP A   1     28.569  68.165   6.536  1.00 42.09           C
ATOM      6    CG  ASP A   1     27.581  68.118   5.373  1.00 43.27           C
ATOM      7    OD1 ASP A   1     26.579  68.889   5.425  1.00 44.83           O
ATOM      8    OD2 ASP A   1     27.821  67.333   4.416  1.00 41.26           O
ATOM      9    N   ILE A   2     29.760  69.413   9.190  1.00 35.61           N
ATOM     10    CA  ILE A   2     30.736  69.619  10.261  1.00 33.32           C
ATOM     11    C   ILE A   2     31.739  68.468  10.355  1.00 32.36           C
ATOM     12    O   ILE A   2     31.350  67.287  10.423  1.00 30.78           O
ATOM     13    CB  ILE A   2     30.046  69.821  11.613  1.00 33.05           C
ATOM     14    CG1 ILE A   2     28.996  70.912  11.483  1.00 32.56           C
ATOM     15    CG2 ILE A   2     31.070  70.182  12.708  1.00 32.78           C
ATOM     16    CD1 ILE A   2     28.526  71.514  12.773  1.00 33.28           C
ATOM     17    N   VAL A   3     33.028  68.824  10.352  1.00 30.93           N
ATOM     18    CA  VAL A   3     34.107  67.874  10.545  1.00 30.06           C
ATOM     19    C   VAL A   3     34.475  67.900  12.021  1.00 28.07           C
ATOM     20    O   VAL A   3     34.771  68.949  12.582  1.00 27.20           O
ATOM     21    CB  VAL A   3     35.329  68.193   9.643  1.00 31.25           C
ATOM     22    CG1 VAL A   3     36.494  67.251   9.922  1.00 31.37           C
ATOM     23    CG2 VAL A   3     34.931  68.093   8.165  1.00 33.03           C
ATOM     24    N   MET A   4     34.384  66.731  12.650  1.00 26.47           N
ATOM     25    CA  MET A   4     34.716  66.548  14.041  1.00 25.65           C
ATOM     26    C   MET A   4     36.043  65.820  14.023  1.00 25.26           C
ATOM     27    O   MET A   4     36.126  64.717  13.484  1.00 26.52           O
ATOM     28    CB  MET A   4     33.630  65.697  14.744  1.00 25.63           C
ATOM     29    CG  MET A   4     32.229  66.270  14.703  1.00 23.91           C
ATOM     30    SD  MET A   4     31.963  67.785  15.666  1.00 27.50           S
ATOM     31    CE  MET A   4     32.339  67.382  17.356  1.00 26.27           C
ATOM     32    N   THR A   5     37.102  66.441  14.542  1.00 25.57           N
ATOM     33    CA  THR A   5     38.423  65.853  14.466  1.00 25.54           C
ATOM     34    C   THR A   5     38.942  65.339  15.815  1.00 25.37           C
ATOM     35    O   THR A   5     39.068  66.084  16.787  1.00 25.08           O
ATOM     36    CB  THR A   5     39.468  66.871  13.886  1.00 27.65           C
ATOM     37    OG1 THR A   5     39.017  67.333  12.607  1.00 27.62           O
ATOM     38    CG2 THR A   5     40.843  66.211  13.758  1.00 27.14           C
ATOM     39    N   GLN A   6     39.265  64.059  15.850  1.00 25.28           N
ATOM     40    CA  GLN A   6     40.011  63.489  16.964  1.00 26.05           C
ATOM     41    C   GLN A   6     41.080  62.544  16.475  1.00 26.46           C
ATOM     42    O   GLN A   6     40.953  61.961  15.414  1.00 27.35           O
ATOM     43    CB  GLN A   6     39.095  62.734  17.948  1.00 26.08           C
ATOM     44    CG  GLN A   6     37.755  62.448  17.463  1.00 25.64           C
ATOM     45    CD  GLN A   6     36.979  61.509  18.355  1.00 24.16           C
ATOM     46    OE1 GLN A   6     36.030  60.896  17.905  1.00 20.50           O
ATOM     47    NE2 GLN A   6     37.380  61.394  19.614  1.00 20.28           N
ATOM     48    N   ALA A   7     42.100  62.358  17.307  1.00 28.46           N
ATOM     49    CA  ALA A   7     43.195  61.451  17.028  1.00 29.35           C
ATOM     50    C   ALA A   7     42.725  60.023  17.235  1.00 29.29           C
ATOM     51    O   ALA A   7     41.762  59.776  17.944  1.00 29.72           O
ATOM     52    CB  ALA A   7     44.376  61.763  17.945  1.00 29.38           C
ATOM     53    N   ALA A   8     43.398  59.080  16.601  1.00 29.18           N
ATOM     54    CA  ALA A   8     42.965  57.696  16.658  1.00 30.13           C
ATOM     55    C   ALA A   8     43.322  57.042  17.980  1.00 30.39           C
```

| | | | | | | | | | | |
|------|-----|-----|-----|---|--------|--------|--------|------|-------|---|
| ATOM | 56  | O   | ALA A | 8  | 42.634 | 56.128 | 18.411 | 1.00 | 28.87 | O |
| ATOM | 57  | CB  | ALA A | 8  | 43.568 | 56.918 | 15.515 | 1.00 | 31.13 | C |
| ATOM | 58  | N   | PHE A | 9  | 44.405 | 57.517 | 18.607 | 1.00 | 32.62 | N |
| ATOM | 59  | CA  | PHE A | 9  | 44.940 | 56.928 | 19.840 | 1.00 | 34.08 | C |
| ATOM | 60  | C   | PHE A | 9  | 45.180 | 57.989 | 20.880 | 1.00 | 33.59 | C |
| ATOM | 61  | O   | PHE A | 9  | 45.725 | 59.036 | 20.570 | 1.00 | 33.92 | O |
| ATOM | 62  | CB  | PHE A | 9  | 46.219 | 56.125 | 19.531 | 1.00 | 35.91 | C |
| ATOM | 63  | CG  | PHE A | 9  | 45.942 | 54.968 | 18.654 | 1.00 | 37.19 | C |
| ATOM | 64  | CD1 | PHE A | 9  | 46.083 | 55.079 | 17.281 | 1.00 | 38.73 | C |
| ATOM | 65  | CD2 | PHE A | 9  | 45.385 | 53.801 | 19.188 | 1.00 | 38.88 | C |
| ATOM | 66  | CE1 | PHE A | 9  | 45.733 | 54.016 | 16.442 | 1.00 | 39.22 | C |
| ATOM | 67  | CE2 | PHE A | 9  | 45.035 | 52.724 | 18.366 | 1.00 | 39.04 | C |
| ATOM | 68  | CZ  | PHE A | 9  | 45.200 | 52.832 | 16.989 | 1.00 | 39.54 | C |
| ATOM | 69  | N   | SER A | 10 | 44.732 | 57.732 | 22.109 | 1.00 | 32.20 | N |
| ATOM | 70  | CA  | SER A | 10 | 45.069 | 58.598 | 23.236 | 1.00 | 31.36 | C |
| ATOM | 71  | C   | SER A | 10 | 46.537 | 58.433 | 23.623 | 1.00 | 31.57 | C |
| ATOM | 72  | O   | SER A | 10 | 47.131 | 57.400 | 23.358 | 1.00 | 30.54 | O |
| ATOM | 73  | CB  | SER A | 10 | 44.251 | 58.197 | 24.453 | 1.00 | 30.99 | C |
| ATOM | 74  | OG  | SER A | 10 | 44.772 | 57.006 | 25.018 | 1.00 | 27.76 | O |
| ATOM | 75  | N   | ASN A | 11 | 47.098 | 59.410 | 24.324 | 1.00 | 32.34 | N |
| ATOM | 76  | CA  | ASN A | 11 | 48.311 | 59.152 | 25.103 | 1.00 | 33.78 | C |
| ATOM | 77  | C   | ASN A | 11 | 48.038 | 57.987 | 26.053 | 1.00 | 33.89 | C |
| ATOM | 78  | O   | ASN A | 11 | 46.998 | 57.970 | 26.705 | 1.00 | 34.51 | O |
| ATOM | 79  | CB  | ASN A | 11 | 48.725 | 60.389 | 25.896 | 1.00 | 33.74 | C |
| ATOM | 80  | CG  | ASN A | 11 | 49.171 | 61.516 | 24.993 | 1.00 | 36.30 | C |
| ATOM | 81  | OD1 | ASN A | 11 | 50.064 | 61.339 | 24.174 | 1.00 | 36.86 | O |
| ATOM | 82  | ND2 | ASN A | 11 | 48.525 | 62.678 | 25.113 | 1.00 | 37.61 | N |
| ATOM | 83  | N   | PRO A | 12 | 48.947 | 57.002 | 26.129 | 1.00 | 34.54 | N |
| ATOM | 84  | CA  | PRO A | 12 | 48.645 | 55.865 | 27.005 | 1.00 | 34.33 | C |
| ATOM | 85  | C   | PRO A | 12 | 48.601 | 56.247 | 28.478 | 1.00 | 34.23 | C |
| ATOM | 86  | O   | PRO A | 12 | 49.197 | 57.239 | 28.888 | 1.00 | 32.73 | O |
| ATOM | 87  | CB  | PRO A | 12 | 49.779 | 54.882 | 26.738 | 1.00 | 35.32 | C |
| ATOM | 88  | CG  | PRO A | 12 | 50.482 | 55.396 | 25.525 | 1.00 | 35.44 | C |
| ATOM | 89  | CD  | PRO A | 12 | 50.248 | 56.849 | 25.465 | 1.00 | 34.86 | C |
| ATOM | 90  | N   | VAL A | 13 | 47.864 | 55.477 | 29.263 | 1.00 | 33.48 | N |
| ATOM | 91  | CA  | VAL A | 13 | 47.573 | 55.889 | 30.607 | 1.00 | 32.44 | C |
| ATOM | 92  | C   | VAL A | 13 | 47.590 | 54.703 | 31.529 | 1.00 | 32.45 | C |
| ATOM | 93  | O   | VAL A | 13 | 46.938 | 53.702 | 31.284 | 1.00 | 32.10 | O |
| ATOM | 94  | CB  | VAL A | 13 | 46.219 | 56.689 | 30.673 | 1.00 | 32.82 | C |
| ATOM | 95  | CG1 | VAL A | 13 | 45.061 | 55.886 | 30.127 | 1.00 | 31.96 | C |
| ATOM | 96  | CG2 | VAL A | 13 | 45.937 | 57.157 | 32.071 | 1.00 | 32.95 | C |
| ATOM | 97  | N   | THR A | 14 | 48.386 | 54.834 | 32.590 | 1.00 | 32.40 | N |
| ATOM | 98  | CA  | THR A | 14 | 48.457 | 53.884 | 33.672 | 1.00 | 31.39 | C |
| ATOM | 99  | C   | THR A | 14 | 47.161 | 53.855 | 34.488 | 1.00 | 30.46 | C |
| ATOM | 100 | O   | THR A | 14 | 46.531 | 54.885 | 34.703 | 1.00 | 27.86 | O |
| ATOM | 101 | CB  | THR A | 14 | 49.631 | 54.292 | 34.572 | 1.00 | 32.15 | C |
| ATOM | 102 | OG1 | THR A | 14 | 50.767 | 54.563 | 33.740 | 1.00 | 33.14 | O |
| ATOM | 103 | CG2 | THR A | 14 | 49.972 | 53.225 | 35.514 | 1.00 | 32.06 | C |
| ATOM | 104 | N   | LEU A | 15 | 46.778 | 52.679 | 34.975 | 1.00 | 30.09 | N |
| ATOM | 105 | CA  | LEU A | 15 | 45.584 | 52.576 | 35.818 | 1.00 | 30.99 | C |
| ATOM | 106 | C   | LEU A | 15 | 45.681 | 53.444 | 37.062 | 1.00 | 29.79 | C |
| ATOM | 107 | O   | LEU A | 15 | 46.719 | 53.497 | 37.701 | 1.00 | 28.56 | O |
| ATOM | 108 | CB  | LEU A | 15 | 45.324 | 51.108 | 36.241 | 1.00 | 32.53 | C |
| ATOM | 109 | CG  | LEU A | 15 | 44.899 | 50.116 | 35.132 | 1.00 | 34.07 | C |
| ATOM | 110 | CD1 | LEU A | 15 | 44.526 | 48.762 | 35.732 | 1.00 | 36.20 | C |
| ATOM | 111 | CD2 | LEU A | 15 | 43.731 | 50.629 | 34.344 | 1.00 | 34.16 | C |
| ATOM | 112 | N   | GLY A | 16 | 44.570 | 54.075 | 37.429 | 1.00 | 29.77 | N |
| ATOM | 113 | CA  | GLY A | 16 | 44.534 | 55.017 | 38.553 | 1.00 | 29.14 | C |
| ATOM | 114 | C   | GLY A | 16 | 44.994 | 56.429 | 38.211 | 1.00 | 27.70 | C |
| ATOM | 115 | O   | GLY A | 16 | 44.906 | 57.334 | 39.045 | 1.00 | 28.04 | O |
| ATOM | 116 | N   | THR A | 17 | 45.507 | 56.639 | 37.008 | 1.00 | 26.72 | N |
| ATOM | 117 | CA  | THR A | 17 | 45.943 | 57.977 | 36.612 | 1.00 | 26.01 | C |
| ATOM | 118 | C   | THR A | 17 | 44.904 | 58.551 | 35.654 | 1.00 | 24.51 | C |
| ATOM | 119 | O   | THR A | 17 | 43.977 | 57.843 | 35.223 | 1.00 | 23.43 | O |
| ATOM | 120 | CB  | THR A | 17 | 47.342 | 57.981 | 35.947 | 1.00 | 25.20 | C |

| | | | | | | | | | | |
|---|---|---|---|---|---|---|---|---|---|---|
| ATOM | 121 | OG1 | THR | A | 17 | 47.282 | 57.280 | 34.714 | 1.00 27.96 | O |
| ATOM | 122 | CG2 | THR | A | 17 | 48.380 | 57.351 | 36.842 | 1.00 27.21 | C |
| ATOM | 123 | N | SER | A | 18 | 45.081 | 59.820 | 35.292 | 1.00 23.37 | N |
| ATOM | 124 | CA | SER | A | 18 | 44.044 | 60.564 | 34.592 | 1.00 22.41 | C |
| ATOM | 125 | C | SER | A | 18 | 44.287 | 60.549 | 33.094 | 1.00 22.63 | C |
| ATOM | 126 | O | SER | A | 18 | 45.429 | 60.665 | 32.664 | 1.00 22.64 | O |
| ATOM | 127 | CB | SER | A | 18 | 44.012 | 62.007 | 35.078 | 1.00 21.49 | C |
| ATOM | 128 | OG | SER | A | 18 | 43.583 | 62.076 | 36.420 | 1.00 24.07 | O |
| ATOM | 129 | N | ALA | A | 19 | 43.209 | 60.425 | 32.323 | 1.00 22.69 | N |
| ATOM | 130 | CA | ALA | A | 19 | 43.248 | 60.442 | 30.859 | 1.00 24.05 | C |
| ATOM | 131 | C | ALA | A | 19 | 42.550 | 61.693 | 30.299 | 1.00 24.91 | C |
| ATOM | 132 | O | ALA | A | 19 | 41.651 | 62.262 | 30.934 | 1.00 22.19 | O |
| ATOM | 133 | CB | ALA | A | 19 | 42.553 | 59.194 | 30.295 | 1.00 24.60 | C |
| ATOM | 134 | N | SER | A | 20 | 42.976 | 62.113 | 29.108 | 1.00 24.32 | N |
| ATOM | 135 | CA | SER | A | 20 | 42.260 | 63.132 | 28.380 | 1.00 25.56 | C |
| ATOM | 136 | C | SER | A | 20 | 42.101 | 62.727 | 26.906 | 1.00 26.88 | C |
| ATOM | 137 | O | SER | A | 20 | 42.956 | 62.016 | 26.333 | 1.00 28.13 | O |
| ATOM | 138 | CB | SER | A | 20 | 42.949 | 64.466 | 28.509 | 1.00 26.40 | C |
| ATOM | 139 | OG | SER | A | 20 | 44.218 | 64.467 | 27.892 | 1.00 28.01 | O |
| ATOM | 140 | N | ILE | A | 21 | 40.972 | 63.109 | 26.328 | 1.00 24.84 | N |
| ATOM | 141 | CA | ILE | A | 21 | 40.712 | 62.896 | 24.909 | 1.00 25.31 | C |
| ATOM | 142 | C | ILE | A | 21 | 40.183 | 64.188 | 24.318 | 1.00 24.58 | C |
| ATOM | 143 | O | ILE | A | 21 | 39.168 | 64.698 | 24.758 | 1.00 23.37 | O |
| ATOM | 144 | CB | ILE | A | 21 | 39.713 | 61.749 | 24.699 | 1.00 24.86 | C |
| ATOM | 145 | CG1 | ILE | A | 21 | 40.337 | 60.465 | 25.278 | 1.00 24.96 | C |
| ATOM | 146 | CG2 | ILE | A | 21 | 39.324 | 61.617 | 23.214 | 1.00 24.87 | C |
| ATOM | 147 | CD1 | ILE | A | 21 | 39.676 | 59.183 | 24.871 | 1.00 25.81 | C |
| ATOM | 148 | N | SER | A | 22 | 40.894 | 64.677 | 23.302 | 1.00 24.34 | N |
| ATOM | 149 | CA | SER | A | 22 | 40.608 | 65.919 | 22.613 | 1.00 24.83 | C |
| ATOM | 150 | C | SER | A | 22 | 39.729 | 65.756 | 21.393 | 1.00 24.35 | C |
| ATOM | 151 | O | SER | A | 22 | 39.771 | 64.742 | 20.698 | 1.00 24.85 | O |
| ATOM | 152 | CB | SER | A | 22 | 41.921 | 66.557 | 22.144 | 1.00 26.67 | C |
| ATOM | 153 | OG | SER | A | 22 | 42.531 | 67.209 | 23.232 | 1.00 29.39 | O |
| ATOM | 154 | N | CYS | A | 23 | 38.932 | 66.780 | 21.131 | 1.00 24.34 | N |
| ATOM | 155 | CA | CYS | A | 23 | 38.125 | 66.826 | 19.938 | 1.00 25.31 | C |
| ATOM | 156 | C | CYS | A | 23 | 37.998 | 68.279 | 19.503 | 1.00 26.52 | C |
| ATOM | 157 | O | CYS | A | 23 | 37.978 | 69.192 | 20.328 | 1.00 26.03 | O |
| ATOM | 158 | CB | CYS | A | 23 | 36.728 | 66.208 | 20.185 | 1.00 25.39 | C |
| ATOM | 159 | SG | CYS | A | 23 | 35.648 | 66.294 | 18.721 | 1.00 26.36 | S |
| ATOM | 160 | N | ARG | A | 24 | 37.935 | 68.482 | 18.200 | 1.00 28.49 | N |
| ATOM | 161 | CA | ARG | A | 24 | 37.725 | 69.812 | 17.640 | 1.00 31.80 | C |
| ATOM | 162 | C | ARG | A | 24 | 36.580 | 69.795 | 16.614 | 1.00 29.78 | C |
| ATOM | 163 | O | ARG | A | 24 | 36.468 | 68.862 | 15.796 | 1.00 31.06 | O |
| ATOM | 164 | CB | ARG | A | 24 | 39.009 | 70.296 | 16.981 | 1.00 32.86 | C |
| ATOM | 165 | CG | ARG | A | 24 | 38.901 | 71.720 | 16.462 | 1.00 36.86 | C |
| ATOM | 166 | CD | ARG | A | 24 | 40.276 | 72.302 | 16.150 | 1.00 38.30 | C |
| ATOM | 167 | NE | ARG | A | 24 | 41.144 | 71.306 | 15.526 | 1.00 42.55 | N |
| ATOM | 168 | CZ | ARG | A | 24 | 41.083 | 70.924 | 14.248 | 1.00 43.78 | C |
| ATOM | 169 | NH1 | ARG | A | 24 | 40.179 | 71.449 | 13.414 | 1.00 45.00 | N |
| ATOM | 170 | NH2 | ARG | A | 24 | 41.936 | 69.994 | 13.802 | 1.00 43.58 | N |
| ATOM | 171 | N | SER | A | 25 | 35.715 | 70.801 | 16.686 | 1.00 29.03 | N |
| ATOM | 172 | CA | SER | A | 25 | 34.672 | 70.988 | 15.680 | 1.00 29.08 | C |
| ATOM | 173 | C | SER | A | 25 | 35.052 | 72.087 | 14.666 | 1.00 28.87 | C |
| ATOM | 174 | O | SER | A | 25 | 35.673 | 73.074 | 15.032 | 1.00 26.27 | O |
| ATOM | 175 | CB | SER | A | 25 | 33.364 | 71.359 | 16.350 | 1.00 28.86 | C |
| ATOM | 176 | OG | SER | A | 25 | 32.373 | 71.547 | 15.360 | 1.00 31.00 | O |
| ATOM | 177 | N | SER | A | 26 | 34.633 | 71.917 | 13.412 | 1.00 29.62 | N |
| ATOM | 178 | CA | SER | A | 26 | 34.888 | 72.909 | 12.353 | 1.00 29.78 | C |
| ATOM | 179 | C | SER | A | 26 | 33.962 | 74.126 | 12.463 | 1.00 30.60 | C |
| ATOM | 180 | O | SER | A | 26 | 34.259 | 75.199 | 11.933 | 1.00 30.63 | O |
| ATOM | 181 | CB | SER | A | 26 | 34.782 | 72.239 | 10.989 | 1.00 30.01 | C |
| ATOM | 182 | OG | SER | A | 26 | 33.465 | 71.777 | 10.765 | 1.00 28.32 | O |
| ATOM | 183 | N | LYS | A | 27 | 32.862 | 73.970 | 13.193 | 1.00 30.73 | N |
| ATOM | 184 | CA | LYS | A | 27 | 31.915 | 75.042 | 13.438 | 1.00 32.26 | C |
| ATOM | 185 | C | LYS | A | 27 | 31.702 | 75.092 | 14.947 | 1.00 31.56 | C |

| ATOM | 186 | O | LYS | A | 27 | 31.736 | 74.050 | 15.608 | 1.00 | 31.87 | O |
|------|-----|-----|-----|---|-----|--------|--------|--------|------|-------|---|
| ATOM | 187 | CB | LYS | A | 27 | 30.603 | 74.733 | 12.688 | 1.00 | 33.62 | C |
| ATOM | 188 | CG | LYS | A | 27 | 29.334 | 75.484 | 13.159 | 1.00 | 34.91 | C |
| ATOM | 189 | CD | LYS | A | 27 | 28.173 | 75.257 | 12.172 | 1.00 | 35.86 | C |
| ATOM | 190 | CE | LYS | A | 27 | 26.922 | 76.081 | 12.518 | 1.00 | 37.54 | C |
| ATOM | 191 | NZ | LYS | A | 27 | 25.868 | 75.360 | 13.338 | 1.00 | 39.27 | N |
| ATOM | 192 | N | SER | A | 27A | 31.494 | 76.286 | 15.495 | 1.00 | 29.12 | N |
| ATOM | 193 | CA | SER | A | 27A | 31.340 | 76.430 | 16.922 | 1.00 | 28.83 | C |
| ATOM | 194 | C | SER | A | 27A | 29.975 | 75.852 | 17.326 | 1.00 | 27.46 | C |
| ATOM | 195 | O | SER | A | 27A | 28.971 | 75.982 | 16.597 | 1.00 | 24.83 | O |
| ATOM | 196 | CB | SER | A | 27A | 31.488 | 77.891 | 17.355 | 1.00 | 29.64 | C |
| ATOM | 197 | OG | SER | A | 27A | 31.071 | 78.086 | 18.707 | 1.00 | 30.21 | O |
| ATOM | 198 | N | LEU | A | 27B | 29.956 | 75.163 | 18.453 | 1.00 | 26.14 | N |
| ATOM | 199 | CA | LEU | A | 27B | 28.759 | 74.460 | 18.884 | 1.00 | 25.44 | C |
| ATOM | 200 | C | LEU | A | 27B | 28.135 | 75.165 | 20.040 | 1.00 | 25.75 | C |
| ATOM | 201 | O | LEU | A | 27B | 27.155 | 74.682 | 20.608 | 1.00 | 25.40 | O |
| ATOM | 202 | CB | LEU | A | 27B | 29.096 | 73.024 | 19.256 | 1.00 | 25.38 | C |
| ATOM | 203 | CG | LEU | A | 27B | 29.875 | 72.220 | 18.210 | 1.00 | 26.55 | C |
| ATOM | 204 | CD1 | LEU | A | 27B | 30.174 | 70.810 | 18.668 | 1.00 | 26.73 | C |
| ATOM | 205 | CD2 | LEU | A | 27B | 29.126 | 72.188 | 16.892 | 1.00 | 28.14 | C |
| ATOM | 206 | N | LEU | A | 27C | 28.717 | 76.300 | 20.400 | 1.00 | 27.01 | N |
| ATOM | 207 | CA | LEU | A | 27C | 28.230 | 77.133 | 21.504 | 1.00 | 28.55 | C |
| ATOM | 208 | C | LEU | A | 27C | 27.224 | 78.098 | 20.958 | 1.00 | 29.55 | C |
| ATOM | 209 | O | LEU | A | 27C | 27.552 | 78.851 | 20.043 | 1.00 | 29.03 | O |
| ATOM | 210 | CB | LEU | A | 27C | 29.384 | 77.933 | 22.115 | 1.00 | 29.17 | C |
| ATOM | 211 | CG | LEU | A | 27C | 29.044 | 78.983 | 23.173 | 1.00 | 28.18 | C |
| ATOM | 212 | CD1 | LEU | A | 27C | 28.470 | 78.377 | 24.429 | 1.00 | 30.02 | C |
| ATOM | 213 | CD2 | LEU | A | 27C | 30.298 | 79.757 | 23.499 | 1.00 | 29.72 | C |
| ATOM | 214 | N | HIS | A | 27D | 26.013 | 78.081 | 21.513 | 1.00 | 31.82 | N |
| ATOM | 215 | CA | HIS | A | 27D | 24.945 | 78.973 | 21.083 | 1.00 | 32.86 | C |
| ATOM | 216 | C | HIS | A | 27D | 24.628 | 80.083 | 22.094 | 1.00 | 34.34 | C |
| ATOM | 217 | O | HIS | A | 27D | 25.180 | 80.136 | 23.197 | 1.00 | 34.82 | O |
| ATOM | 218 | CB | HIS | A | 27D | 23.683 | 78.165 | 20.781 | 1.00 | 33.79 | C |
| ATOM | 219 | CG | HIS | A | 27D | 23.851 | 77.208 | 19.649 | 1.00 | 35.15 | C |
| ATOM | 220 | ND1 | HIS | A | 27D | 22.956 | 76.196 | 19.389 | 1.00 | 35.28 | N |
| ATOM | 221 | CD2 | HIS | A | 27D | 24.834 | 77.089 | 18.726 | 1.00 | 34.68 | C |
| ATOM | 222 | CE1 | HIS | A | 27D | 23.371 | 75.509 | 18.339 | 1.00 | 36.20 | C |
| ATOM | 223 | NE2 | HIS | A | 27D | 24.507 | 76.031 | 17.919 | 1.00 | 34.64 | N |
| ATOM | 224 | N | SER | A | 27E | 23.719 | 80.975 | 21.700 | 1.00 | 35.76 | N |
| ATOM | 225 | CA | SER | A | 27E | 23.377 | 82.147 | 22.490 | 1.00 | 36.29 | C |
| ATOM | 226 | C | SER | A | 27E | 22.746 | 81.774 | 23.809 | 1.00 | 36.32 | C |
| ATOM | 227 | O | SER | A | 27E | 22.782 | 82.574 | 24.750 | 1.00 | 37.14 | O |
| ATOM | 228 | CB | SER | A | 27E | 22.438 | 83.084 | 21.711 | 1.00 | 37.40 | C |
| ATOM | 229 | OG | SER | A | 27E | 21.118 | 82.554 | 21.593 | 1.00 | 38.29 | O |
| ATOM | 230 | N | ASP | A | 28 | 22.178 | 80.572 | 23.899 | 1.00 | 35.59 | N |
| ATOM | 231 | CA | ASP | A | 28 | 21.601 | 80.128 | 25.174 | 1.00 | 36.10 | C |
| ATOM | 232 | C | ASP | A | 28 | 22.676 | 79.764 | 26.195 | 1.00 | 34.85 | C |
| ATOM | 233 | O | ASP | A | 28 | 22.338 | 79.377 | 27.309 | 1.00 | 35.35 | O |
| ATOM | 234 | CB | ASP | A | 28 | 20.641 | 78.952 | 24.996 | 1.00 | 35.82 | C |
| ATOM | 235 | CG | ASP | A | 28 | 21.261 | 77.811 | 24.232 | 1.00 | 38.23 | C |
| ATOM | 236 | OD1 | ASP | A | 28 | 22.485 | 77.867 | 23.972 | 1.00 | 39.78 | O |
| ATOM | 237 | OD2 | ASP | A | 28 | 20.526 | 76.865 | 23.874 | 1.00 | 38.65 | O |
| ATOM | 238 | N | GLY | A | 29 | 23.953 | 79.897 | 25.834 | 1.00 | 33.48 | N |
| ATOM | 239 | CA | GLY | A | 29 | 25.040 | 79.517 | 26.754 | 1.00 | 32.85 | C |
| ATOM | 240 | C | GLY | A | 29 | 25.333 | 78.019 | 26.776 | 1.00 | 32.10 | C |
| ATOM | 241 | O | GLY | A | 29 | 26.185 | 77.564 | 27.555 | 1.00 | 31.63 | O |
| ATOM | 242 | N | ILE | A | 30 | 24.667 | 77.257 | 25.899 | 1.00 | 30.56 | N |
| ATOM | 243 | CA | ILE | A | 30 | 24.828 | 75.796 | 25.842 | 1.00 | 29.30 | C |
| ATOM | 244 | C | ILE | A | 30 | 25.696 | 75.385 | 24.657 | 1.00 | 27.45 | C |
| ATOM | 245 | O | ILE | A | 30 | 25.569 | 75.934 | 23.576 | 1.00 | 27.49 | O |
| ATOM | 246 | CB | ILE | A | 30 | 23.463 | 75.081 | 25.766 | 1.00 | 29.86 | C |
| ATOM | 247 | CG1 | ILE | A | 30 | 22.553 | 75.531 | 26.913 | 1.00 | 29.48 | C |
| ATOM | 248 | CG2 | ILE | A | 30 | 23.634 | 73.561 | 25.814 | 1.00 | 29.02 | C |
| ATOM | 249 | CD1 | ILE | A | 30 | 21.137 | 75.141 | 26.707 | 1.00 | 30.76 | C |
| ATOM | 250 | N | THR | A | 31 | 26.598 | 74.424 | 24.877 | 1.00 | 26.56 | N |

| ATOM | 251 | CA | THR | A | 31 | 27.434 | 73.906 | 23.797 | 1.00 | 26.68 | C |
| ATOM | 252 | C | THR | A | 31 | 26.967 | 72.503 | 23.427 | 1.00 | 25.23 | C |
| ATOM | 253 | O | THR | A | 31 | 26.951 | 71.631 | 24.267 | 1.00 | 25.35 | O |
| ATOM | 254 | CB | THR | A | 31 | 28.922 | 73.892 | 24.198 | 1.00 | 26.46 | C |
| ATOM | 255 | OG1 | THR | A | 31 | 29.254 | 75.163 | 24.776 | 1.00 | 25.57 | O |
| ATOM | 256 | CG2 | THR | A | 31 | 29.810 | 73.570 | 23.000 | 1.00 | 25.84 | C |
| ATOM | 257 | N | TYR | A | 32 | 26.571 | 72.312 | 22.171 | 1.00 | 24.54 | N |
| ATOM | 258 | CA | TYR | A | 32 | 25.894 | 71.105 | 21.747 | 1.00 | 25.13 | C |
| ATOM | 259 | C | TYR | A | 32 | 26.877 | 70.014 | 21.302 | 1.00 | 23.40 | C |
| ATOM | 260 | O | TYR | A | 32 | 26.876 | 69.552 | 20.175 | 1.00 | 23.70 | O |
| ATOM | 261 | CB | TYR | A | 32 | 24.829 | 71.431 | 20.718 | 1.00 | 25.26 | C |
| ATOM | 262 | CG | TYR | A | 32 | 23.721 | 72.228 | 21.352 | 1.00 | 26.39 | C |
| ATOM | 263 | CD1 | TYR | A | 32 | 23.807 | 73.612 | 21.432 | 1.00 | 25.51 | C |
| ATOM | 264 | CD2 | TYR | A | 32 | 22.620 | 71.588 | 21.947 | 1.00 | 26.69 | C |
| ATOM | 265 | CE1 | TYR | A | 32 | 22.806 | 74.366 | 22.038 | 1.00 | 27.06 | C |
| ATOM | 266 | CE2 | TYR | A | 32 | 21.615 | 72.326 | 22.567 | 1.00 | 27.71 | C |
| ATOM | 267 | CZ | TYR | A | 32 | 21.720 | 73.722 | 22.611 | 1.00 | 27.48 | C |
| ATOM | 268 | OH | TYR | A | 32 | 20.761 | 74.487 | 23.217 | 1.00 | 27.89 | O |
| ATOM | 269 | N | LEU | A | 33 | 27.696 | 69.609 | 22.261 | 1.00 | 23.06 | N |
| ATOM | 270 | CA | LEU | A | 33 | 28.723 | 68.624 | 22.067 | 1.00 | 22.95 | C |
| ATOM | 271 | C | LEU | A | 33 | 28.417 | 67.407 | 22.940 | 1.00 | 22.99 | C |
| ATOM | 272 | O | LEU | A | 33 | 28.026 | 67.548 | 24.096 | 1.00 | 21.56 | O |
| ATOM | 273 | CB | LEU | A | 33 | 30.033 | 69.235 | 22.487 | 1.00 | 23.75 | C |
| ATOM | 274 | CG | LEU | A | 33 | 31.314 | 68.454 | 22.291 | 1.00 | 24.93 | C |
| ATOM | 275 | CD1 | LEU | A | 33 | 31.537 | 67.436 | 23.402 | 1.00 | 24.26 | C |
| ATOM | 276 | CD2 | LEU | A | 33 | 31.357 | 67.857 | 20.891 | 1.00 | 24.75 | C |
| ATOM | 277 | N | TYR | A | 34 | 28.641 | 66.219 | 22.378 | 1.00 | 22.23 | N |
| ATOM | 278 | CA | TYR | A | 34 | 28.306 | 64.969 | 23.041 | 1.00 | 22.31 | C |
| ATOM | 279 | C | TYR | A | 34 | 29.505 | 64.022 | 22.931 | 1.00 | 21.65 | C |
| ATOM | 280 | O | TYR | A | 34 | 30.326 | 64.132 | 22.009 | 1.00 | 20.79 | O |
| ATOM | 281 | CB | TYR | A | 34 | 27.066 | 64.335 | 22.386 | 1.00 | 22.17 | C |
| ATOM | 282 | CG | TYR | A | 34 | 25.800 | 65.139 | 22.534 | 1.00 | 22.88 | C |
| ATOM | 283 | CD1 | TYR | A | 34 | 25.607 | 66.321 | 21.815 | 1.00 | 23.47 | C |
| ATOM | 284 | CD2 | TYR | A | 34 | 24.768 | 64.703 | 23.376 | 1.00 | 23.85 | C |
| ATOM | 285 | CE1 | TYR | A | 34 | 24.466 | 67.077 | 21.953 | 1.00 | 22.26 | C |
| ATOM | 286 | CE2 | TYR | A | 34 | 23.601 | 65.437 | 23.507 | 1.00 | 23.93 | C |
| ATOM | 287 | CZ | TYR | A | 34 | 23.448 | 66.623 | 22.804 | 1.00 | 25.14 | C |
| ATOM | 288 | OH | TYR | A | 34 | 22.293 | 67.376 | 22.952 | 1.00 | 24.81 | O |
| ATOM | 289 | N | TRP | A | 35 | 29.616 | 63.118 | 23.897 | 1.00 | 19.97 | N |
| ATOM | 290 | CA | TRP | A | 35 | 30.675 | 62.113 | 23.927 | 1.00 | 17.68 | C |
| ATOM | 291 | C | TRP | A | 35 | 30.004 | 60.770 | 24.102 | 1.00 | 19.41 | C |
| ATOM | 292 | O | TRP | A | 35 | 29.053 | 60.632 | 24.940 | 1.00 | 17.54 | O |
| ATOM | 293 | CB | TRP | A | 35 | 31.651 | 62.353 | 25.085 | 1.00 | 18.09 | C |
| ATOM | 294 | CG | TRP | A | 35 | 32.617 | 63.486 | 24.929 | 1.00 | 17.43 | C |
| ATOM | 295 | CD1 | TRP | A | 35 | 32.564 | 64.700 | 25.530 | 1.00 | 19.01 | C |
| ATOM | 296 | CD2 | TRP | A | 35 | 33.825 | 63.473 | 24.150 | 1.00 | 17.61 | C |
| ATOM | 297 | NE1 | TRP | A | 35 | 33.682 | 65.463 | 25.157 | 1.00 | 19.91 | N |
| ATOM | 298 | CE2 | TRP | A | 35 | 34.454 | 64.719 | 24.317 | 1.00 | 18.85 | C |
| ATOM | 299 | CE3 | TRP | A | 35 | 34.438 | 62.513 | 23.327 | 1.00 | 17.92 | C |
| ATOM | 300 | CZ2 | TRP | A | 35 | 35.664 | 65.048 | 23.663 | 1.00 | 18.75 | C |
| ATOM | 301 | CZ3 | TRP | A | 35 | 35.648 | 62.847 | 22.686 | 1.00 | 19.53 | C |
| ATOM | 302 | CH2 | TRP | A | 35 | 36.222 | 64.099 | 22.863 | 1.00 | 18.23 | C |
| ATOM | 303 | N | TYR | A | 36 | 30.512 | 59.795 | 23.349 | 1.00 | 19.30 | N |
| ATOM | 304 | CA | TYR | A | 36 | 30.066 | 58.397 | 23.419 | 1.00 | 19.76 | C |
| ATOM | 305 | C | TYR | A | 36 | 31.269 | 57.496 | 23.623 | 1.00 | 19.92 | C |
| ATOM | 306 | O | TYR | A | 36 | 32.394 | 57.769 | 23.138 | 1.00 | 19.11 | O |
| ATOM | 307 | CB | TYR | A | 36 | 29.295 | 57.978 | 22.133 | 1.00 | 19.54 | C |
| ATOM | 308 | CG | TYR | A | 36 | 28.103 | 58.836 | 21.830 | 1.00 | 20.41 | C |
| ATOM | 309 | CD1 | TYR | A | 36 | 28.231 | 60.016 | 21.089 | 1.00 | 20.83 | C |
| ATOM | 310 | CD2 | TYR | A | 36 | 26.836 | 58.503 | 22.333 | 1.00 | 20.04 | C |
| ATOM | 311 | CE1 | TYR | A | 36 | 27.165 | 60.808 | 20.872 | 1.00 | 20.44 | C |
| ATOM | 312 | CE2 | TYR | A | 36 | 25.764 | 59.288 | 22.100 | 1.00 | 20.23 | C |
| ATOM | 313 | CZ | TYR | A | 36 | 25.927 | 60.442 | 21.381 | 1.00 | 21.29 | C |
| ATOM | 314 | OH | TYR | A | 36 | 24.842 | 61.230 | 21.157 | 1.00 | 22.29 | O |
| ATOM | 315 | N | LEU | A | 37 | 31.062 | 56.423 | 24.379 | 1.00 | 21.00 | N |

| ATOM | 316 | CA | LEU | A | 37 | 32.053 | 55.363 | 24.470 | 1.00 | 23.11 | C |
|------|-----|-----|-----|---|----|--------|--------|--------|------|-------|---|
| ATOM | 317 | C | LEU | A | 37 | 31.538 | 54.120 | 23.763 | 1.00 | 24.53 | C |
| ATOM | 318 | O | LEU | A | 37 | 30.382 | 53.747 | 23.901 | 1.00 | 23.46 | O |
| ATOM | 319 | CB | LEU | A | 37 | 32.417 | 55.044 | 25.936 | 1.00 | 23.85 | C |
| ATOM | 320 | CG | LEU | A | 37 | 33.187 | 53.748 | 26.264 | 1.00 | 23.86 | C |
| ATOM | 321 | CD1 | LEU | A | 37 | 34.532 | 53.705 | 25.590 | 1.00 | 23.58 | C |
| ATOM | 322 | CD2 | LEU | A | 37 | 33.391 | 53.559 | 27.757 | 1.00 | 24.21 | C |
| ATOM | 323 | N | GLN | A | 38 | 32.407 | 53.469 | 23.006 | 1.00 | 25.79 | N |
| ATOM | 324 | CA | GLN | A | 38 | 32.039 | 52.203 | 22.409 | 1.00 | 27.67 | C |
| ATOM | 325 | C | GLN | A | 38 | 33.041 | 51.220 | 22.943 | 1.00 | 29.33 | C |
| ATOM | 326 | O | GLN | A | 38 | 34.209 | 51.220 | 22.566 | 1.00 | 27.60 | O |
| ATOM | 327 | CB | GLN | A | 38 | 32.014 | 52.255 | 20.878 | 1.00 | 27.51 | C |
| ATOM | 328 | CG | GLN | A | 38 | 31.425 | 51.018 | 20.213 | 1.00 | 27.72 | C |
| ATOM | 329 | CD | GLN | A | 38 | 31.399 | 51.134 | 18.702 | 1.00 | 28.27 | C |
| ATOM | 330 | OE1 | GLN | A | 38 | 32.170 | 51.895 | 18.109 | 1.00 | 30.60 | O |
| ATOM | 331 | NE2 | GLN | A | 38 | 30.496 | 50.407 | 18.079 | 1.00 | 29.06 | N |
| ATOM | 332 | N | LYS | A | 39 | 32.555 | 50.443 | 23.902 | 1.00 | 33.18 | N |
| ATOM | 333 | CA | LYS | A | 39 | 33.302 | 49.370 | 24.516 | 1.00 | 36.79 | C |
| ATOM | 334 | C | LYS | A | 39 | 33.411 | 48.199 | 23.536 | 1.00 | 37.90 | C |
| ATOM | 335 | O | LYS | A | 39 | 32.551 | 48.004 | 22.666 | 1.00 | 38.48 | O |
| ATOM | 336 | CB | LYS | A | 39 | 32.631 | 48.945 | 25.837 | 1.00 | 37.50 | C |
| ATOM | 337 | CG | LYS | A | 39 | 32.747 | 50.008 | 26.949 | 1.00 | 38.73 | C |
| ATOM | 338 | CD | LYS | A | 39 | 32.284 | 49.517 | 28.342 | 1.00 | 39.04 | C |
| ATOM | 339 | CE | LYS | A | 39 | 32.475 | 50.604 | 29.416 | 1.00 | 39.93 | C |
| ATOM | 340 | NZ | LYS | A | 39 | 31.987 | 50.216 | 30.805 | 1.00 | 40.93 | N |
| ATOM | 341 | N | PRO | A | 40 | 34.499 | 47.431 | 23.639 | 1.00 | 39.63 | N |
| ATOM | 342 | CA | PRO | A | 40 | 34.707 | 46.354 | 22.668 | 1.00 | 40.37 | C |
| ATOM | 343 | C | PRO | A | 40 | 33.518 | 45.377 | 22.590 | 1.00 | 41.83 | C |
| ATOM | 344 | O | PRO | A | 40 | 33.006 | 44.922 | 23.633 | 1.00 | 42.34 | O |
| ATOM | 345 | CB | PRO | A | 40 | 35.967 | 45.669 | 23.191 | 1.00 | 41.03 | C |
| ATOM | 346 | CG | PRO | A | 40 | 36.677 | 46.752 | 24.009 | 1.00 | 40.73 | C |
| ATOM | 347 | CD | PRO | A | 40 | 35.593 | 47.532 | 24.628 | 1.00 | 39.18 | C |
| ATOM | 348 | N | GLY | A | 41 | 33.055 | 45.101 | 21.369 | 1.00 | 42.44 | N |
| ATOM | 349 | CA | GLY | A | 41 | 31.923 | 44.194 | 21.146 | 1.00 | 43.38 | C |
| ATOM | 350 | C | GLY | A | 41 | 30.531 | 44.819 | 21.197 | 1.00 | 44.25 | C |
| ATOM | 351 | O | GLY | A | 41 | 29.540 | 44.181 | 20.795 | 1.00 | 45.78 | O |
| ATOM | 352 | N | GLN | A | 42 | 30.451 | 46.068 | 21.659 | 1.00 | 43.20 | N |
| ATOM | 353 | CA | GLN | A | 42 | 29.186 | 46.693 | 22.008 | 1.00 | 41.63 | C |
| ATOM | 354 | C | GLN | A | 42 | 28.886 | 47.879 | 21.102 | 1.00 | 39.12 | C |
| ATOM | 355 | O | GLN | A | 42 | 29.720 | 48.290 | 20.287 | 1.00 | 38.73 | O |
| ATOM | 356 | CB | GLN | A | 42 | 29.242 | 47.159 | 23.472 | 1.00 | 42.00 | C |
| ATOM | 357 | CG | GLN | A | 42 | 29.419 | 46.031 | 24.486 | 1.00 | 43.87 | C |
| ATOM | 358 | CD | GLN | A | 42 | 29.364 | 46.511 | 25.929 | 1.00 | 44.28 | C |
| ATOM | 359 | OE1 | GLN | A | 42 | 29.675 | 47.665 | 26.227 | 1.00 | 46.10 | O |
| ATOM | 360 | NE2 | GLN | A | 42 | 28.958 | 45.621 | 26.839 | 1.00 | 47.36 | N |
| ATOM | 361 | N | SER | A | 43 | 27.685 | 48.427 | 21.252 | 1.00 | 36.02 | N |
| ATOM | 362 | CA | SER | A | 43 | 27.295 | 49.643 | 20.563 | 1.00 | 34.29 | C |
| ATOM | 363 | C | SER | A | 43 | 27.844 | 50.875 | 21.320 | 1.00 | 33.05 | C |
| ATOM | 364 | O | SER | A | 43 | 28.288 | 50.747 | 22.462 | 1.00 | 32.88 | O |
| ATOM | 365 | CB | SER | A | 43 | 25.771 | 49.688 | 20.432 | 1.00 | 35.26 | C |
| ATOM | 366 | OG | SER | A | 43 | 25.128 | 49.676 | 21.702 | 1.00 | 38.64 | O |
| ATOM | 367 | N | PRO | A | 44 | 27.872 | 52.059 | 20.667 | 1.00 | 31.14 | N |
| ATOM | 368 | CA | PRO | A | 44 | 28.146 | 53.300 | 21.406 | 1.00 | 30.56 | C |
| ATOM | 369 | C | PRO | A | 44 | 27.144 | 53.543 | 22.559 | 1.00 | 29.49 | C |
| ATOM | 370 | O | PRO | A | 44 | 26.014 | 53.110 | 22.473 | 1.00 | 29.93 | O |
| ATOM | 371 | CB | PRO | A | 44 | 28.020 | 54.397 | 20.323 | 1.00 | 29.27 | C |
| ATOM | 372 | CG | PRO | A | 44 | 28.251 | 53.681 | 19.013 | 1.00 | 29.44 | C |
| ATOM | 373 | CD | PRO | A | 44 | 27.676 | 52.309 | 19.221 | 1.00 | 29.95 | C |
| ATOM | 374 | N | HIS | A | 45 | 27.591 | 54.182 | 23.640 | 1.00 | 28.91 | N |
| ATOM | 375 | CA | HIS | A | 45 | 26.699 | 54.652 | 24.692 | 1.00 | 28.66 | C |
| ATOM | 376 | C | HIS | A | 45 | 27.129 | 56.061 | 25.092 | 1.00 | 27.12 | C |
| ATOM | 377 | O | HIS | A | 45 | 28.336 | 56.400 | 25.099 | 1.00 | 24.64 | O |
| ATOM | 378 | CB | HIS | A | 45 | 26.625 | 53.685 | 25.915 | 1.00 | 30.77 | C |
| ATOM | 379 | CG | HIS | A | 45 | 27.838 | 53.690 | 26.800 | 1.00 | 32.30 | C |
| ATOM | 380 | ND1 | HIS | A | 45 | 28.930 | 52.869 | 26.586 | 1.00 | 36.11 | N |

| ATOM | 381 | CD2 | HIS | A | 45 | 28.114 | 54.385 | 27.932 | 1.00 | 35.06 | C |
|------|-----|-----|-----|---|----|--------|--------|--------|------|-------|---|
| ATOM | 382 | CE1 | HIS | A | 45 | 29.836 | 53.084 | 27.527 | 1.00 | 34.83 | C |
| ATOM | 383 | NE2 | HIS | A | 45 | 29.370 | 54.008 | 28.349 | 1.00 | 33.89 | N |
| ATOM | 384 | N | LEU | A | 46 | 26.118 | 56.864 | 25.394 | 1.00 | 25.49 | N |
| ATOM | 385 | CA | LEU | A | 46 | 26.278 | 58.255 | 25.782 | 1.00 | 25.46 | C |
| ATOM | 386 | C | LEU | A | 46 | 27.004 | 58.412 | 27.106 | 1.00 | 25.62 | C |
| ATOM | 387 | O | LEU | A | 46 | 26.652 | 57.759 | 28.107 | 1.00 | 24.59 | O |
| ATOM | 388 | CB | LEU | A | 46 | 24.909 | 58.918 | 25.883 | 1.00 | 24.74 | C |
| ATOM | 389 | CG | LEU | A | 46 | 24.925 | 60.394 | 26.215 | 1.00 | 24.91 | C |
| ATOM | 390 | CD1 | LEU | A | 46 | 25.617 | 61.181 | 25.088 | 1.00 | 23.71 | C |
| ATOM | 391 | CD2 | LEU | A | 46 | 23.571 | 60.927 | 26.466 | 1.00 | 25.14 | C |
| ATOM | 392 | N | LEU | A | 47 | 27.995 | 59.294 | 27.125 | 1.00 | 23.39 | N |
| ATOM | 393 | CA | LEU | A | 47 | 28.708 | 59.630 | 28.348 | 1.00 | 25.82 | C |
| ATOM | 394 | C | LEU | A | 47 | 28.388 | 61.034 | 28.849 | 1.00 | 26.06 | C |
| ATOM | 395 | O | LEU | A | 47 | 28.153 | 61.232 | 30.045 | 1.00 | 25.95 | O |
| ATOM | 396 | CB | LEU | A | 47 | 30.235 | 59.547 | 28.150 | 1.00 | 26.19 | C |
| ATOM | 397 | CG | LEU | A | 47 | 30.879 | 58.248 | 27.717 | 1.00 | 27.97 | C |
| ATOM | 398 | CD1 | LEU | A | 47 | 32.290 | 58.573 | 27.222 | 1.00 | 28.70 | C |
| ATOM | 399 | CD2 | LEU | A | 47 | 30.916 | 57.204 | 28.846 | 1.00 | 28.09 | C |
| ATOM | 400 | N | ILE | A | 48 | 28.471 | 62.005 | 27.937 | 1.00 | 26.24 | N |
| ATOM | 401 | CA | ILE | A | 48 | 28.309 | 63.418 | 28.238 | 1.00 | 25.86 | C |
| ATOM | 402 | C | ILE | A | 48 | 27.408 | 64.055 | 27.202 | 1.00 | 25.49 | C |
| ATOM | 403 | O | ILE | A | 48 | 27.554 | 63.795 | 25.993 | 1.00 | 22.66 | O |
| ATOM | 404 | CB | ILE | A | 48 | 29.659 | 64.198 | 28.157 | 1.00 | 27.03 | C |
| ATOM | 405 | CG1 | ILE | A | 48 | 30.745 | 63.589 | 29.061 | 1.00 | 27.82 | C |
| ATOM | 406 | CG2 | ILE | A | 48 | 29.436 | 65.692 | 28.435 | 1.00 | 28.21 | C |
| ATOM | 407 | CD1 | ILE | A | 48 | 30.646 | 63.863 | 30.526 | 1.00 | 28.44 | C |
| ATOM | 408 | N | TYR | A | 49 | 26.500 | 64.913 | 27.661 | 1.00 | 23.96 | N |
| ATOM | 409 | CA | TYR | A | 49 | 25.681 | 65.718 | 26.762 | 1.00 | 24.61 | C |
| ATOM | 410 | C | TYR | A | 49 | 25.821 | 67.189 | 27.087 | 1.00 | 24.47 | C |
| ATOM | 411 | O | TYR | A | 49 | 26.136 | 67.552 | 28.207 | 1.00 | 21.56 | O |
| ATOM | 412 | CB | TYR | A | 49 | 24.220 | 65.320 | 26.834 | 1.00 | 27.18 | C |
| ATOM | 413 | CG | TYR | A | 49 | 23.584 | 65.510 | 28.186 | 1.00 | 27.98 | C |
| ATOM | 414 | CD1 | TYR | A | 49 | 23.657 | 64.516 | 29.162 | 1.00 | 28.95 | C |
| ATOM | 415 | CD2 | TYR | A | 49 | 22.882 | 66.665 | 28.475 | 1.00 | 29.65 | C |
| ATOM | 416 | CE1 | TYR | A | 49 | 23.042 | 64.690 | 30.402 | 1.00 | 29.56 | C |
| ATOM | 417 | CE2 | TYR | A | 49 | 22.274 | 66.851 | 29.712 | 1.00 | 29.18 | C |
| ATOM | 418 | CZ | TYR | A | 49 | 22.348 | 65.862 | 30.654 | 1.00 | 29.41 | C |
| ATOM | 419 | OH | TYR | A | 49 | 21.735 | 66.070 | 31.872 | 1.00 | 31.48 | O |
| ATOM | 420 | N | HIS | A | 50 | 25.610 | 68.025 | 26.083 | 1.00 | 24.76 | N |
| ATOM | 421 | CA | HIS | A | 50 | 25.780 | 69.459 | 26.226 | 1.00 | 26.54 | C |
| ATOM | 422 | C | HIS | A | 50 | 27.116 | 69.793 | 26.900 | 1.00 | 25.87 | C |
| ATOM | 423 | O | HIS | A | 50 | 27.161 | 70.507 | 27.909 | 1.00 | 24.64 | O |
| ATOM | 424 | CB | HIS | A | 50 | 24.626 | 70.094 | 26.999 | 1.00 | 28.18 | C |
| ATOM | 425 | CG | HIS | A | 50 | 23.307 | 70.001 | 26.304 | 1.00 | 30.60 | C |
| ATOM | 426 | ND1 | HIS | A | 50 | 22.110 | 70.161 | 26.966 | 1.00 | 30.72 | N |
| ATOM | 427 | CD2 | HIS | A | 50 | 22.994 | 69.744 | 25.012 | 1.00 | 31.90 | C |
| ATOM | 428 | CE1 | HIS | A | 50 | 21.115 | 70.018 | 26.111 | 1.00 | 31.57 | C |
| ATOM | 429 | NE2 | HIS | A | 50 | 21.622 | 69.756 | 24.921 | 1.00 | 33.02 | N |
| ATOM | 430 | N | LEU | A | 51 | 28.181 | 69.259 | 26.319 | 1.00 | 25.28 | N |
| ATOM | 431 | CA | LEU | A | 51 | 29.571 | 69.553 | 26.709 | 1.00 | 26.41 | C |
| ATOM | 432 | C | LEU | A | 51 | 30.018 | 68.992 | 28.072 | 1.00 | 26.28 | C |
| ATOM | 433 | O | LEU | A | 51 | 31.030 | 68.304 | 28.128 | 1.00 | 26.49 | O |
| ATOM | 434 | CB | LEU | A | 51 | 29.872 | 71.043 | 26.611 | 1.00 | 25.85 | C |
| ATOM | 435 | CG | LEU | A | 51 | 31.348 | 71.416 | 26.774 | 1.00 | 26.72 | C |
| ATOM | 436 | CD1 | LEU | A | 51 | 32.178 | 70.761 | 25.736 | 1.00 | 26.97 | C |
| ATOM | 437 | CD2 | LEU | A | 51 | 31.503 | 72.920 | 26.686 | 1.00 | 28.16 | C |
| ATOM | 438 | N | SER | A | 52 | 29.259 | 69.234 | 29.145 | 1.00 | 27.12 | N |
| ATOM | 439 | CA | SER | A | 52 | 29.745 | 68.993 | 30.510 | 1.00 | 26.09 | C |
| ATOM | 440 | C | SER | A | 52 | 28.809 | 68.184 | 31.414 | 1.00 | 27.24 | C |
| ATOM | 441 | O | SER | A | 52 | 29.184 | 67.853 | 32.545 | 1.00 | 26.88 | O |
| ATOM | 442 | CB | SER | A | 52 | 30.026 | 70.331 | 31.183 | 1.00 | 27.71 | C |
| ATOM | 443 | OG | SER | A | 52 | 28.907 | 71.227 | 31.113 | 1.00 | 29.59 | O |
| ATOM | 444 | N | ASN | A | 53 | 27.635 | 67.827 | 30.914 | 1.00 | 26.88 | N |
| ATOM | 445 | CA | ASN | A | 53 | 26.679 | 67.064 | 31.697 | 1.00 | 27.81 | C |

| ATOM | 446 | C | ASN A | 53 | 26.900 | 65.568 | 31.620 | 1.00 | 28.26 | C |
|------|-----|-----|-------|----|--------|--------|--------|------|-------|---|
| ATOM | 447 | O | ASN A | 53 | 27.030 | 64.996 | 30.543 | 1.00 | 29.62 | O |
| ATOM | 448 | CB | ASN A | 53 | 25.247 | 67.427 | 31.288 | 1.00 | 27.87 | C |
| ATOM | 449 | CG | ASN A | 53 | 24.909 | 68.854 | 31.614 | 1.00 | 28.68 | C |
| ATOM | 450 | OD1 | ASN A | 53 | 24.626 | 69.161 | 32.751 | 1.00 | 34.94 | O |
| ATOM | 451 | ND2 | ASN A | 53 | 24.971 | 69.734 | 30.641 | 1.00 | 29.44 | N |
| ATOM | 452 | N | LEU A | 54 | 26.918 | 64.939 | 32.791 | 1.00 | 28.17 | N |
| ATOM | 453 | CA | LEU A | 54 | 27.179 | 63.530 | 32.935 | 1.00 | 29.34 | C |
| ATOM | 454 | C | LEU A | 54 | 25.890 | 62.756 | 32.665 | 1.00 | 30.46 | C |
| ATOM | 455 | O | LEU A | 54 | 24.839 | 63.091 | 33.236 | 1.00 | 29.02 | O |
| ATOM | 456 | CB | LEU A | 54 | 27.682 | 63.268 | 34.365 | 1.00 | 30.03 | C |
| ATOM | 457 | CG | LEU A | 54 | 28.309 | 61.924 | 34.685 | 1.00 | 31.44 | C |
| ATOM | 458 | CD1 | LEU A | 54 | 29.654 | 61.784 | 33.928 | 1.00 | 32.35 | C |
| ATOM | 459 | CD2 | LEU A | 54 | 28.471 | 61.742 | 36.201 | 1.00 | 30.91 | C |
| ATOM | 460 | N | ALA A | 55 | 25.968 | 61.740 | 31.803 | 1.00 | 30.09 | N |
| ATOM | 461 | CA | ALA A | 55 | 24.789 | 60.959 | 31.436 | 1.00 | 32.13 | C |
| ATOM | 462 | C | ALA A | 55 | 24.418 | 59.976 | 32.541 | 1.00 | 33.53 | C |
| ATOM | 463 | O | ALA A | 55 | 25.251 | 59.575 | 33.371 | 1.00 | 32.51 | O |
| ATOM | 464 | CB | ALA A | 55 | 25.012 | 60.203 | 30.153 | 1.00 | 31.97 | C |
| ATOM | 465 | N | SER A | 56 | 23.154 | 59.573 | 32.537 | 1.00 | 35.41 | N |
| ATOM | 466 | CA | SER A | 56 | 22.654 | 58.715 | 33.610 | 1.00 | 36.07 | C |
| ATOM | 467 | C | SER A | 56 | 23.457 | 57.422 | 33.709 | 1.00 | 34.82 | C |
| ATOM | 468 | O | SER A | 56 | 23.698 | 56.757 | 32.712 | 1.00 | 35.24 | O |
| ATOM | 469 | CB | SER A | 56 | 21.167 | 58.395 | 33.399 | 1.00 | 36.72 | C |
| ATOM | 470 | OG | SER A | 56 | 20.726 | 57.530 | 34.433 | 1.00 | 38.33 | O |
| ATOM | 471 | N | GLY A | 57 | 23.867 | 57.076 | 34.923 | 1.00 | 35.47 | N |
| ATOM | 472 | CA | GLY A | 57 | 24.640 | 55.856 | 35.184 | 1.00 | 34.99 | C |
| ATOM | 473 | C | GLY A | 57 | 26.101 | 55.857 | 34.723 | 1.00 | 34.50 | C |
| ATOM | 474 | O | GLY A | 57 | 26.731 | 54.805 | 34.680 | 1.00 | 34.88 | O |
| ATOM | 475 | N | VAL A | 58 | 26.634 | 57.021 | 34.359 | 1.00 | 32.82 | N |
| ATOM | 476 | CA | VAL A | 58 | 28.034 | 57.106 | 33.933 | 1.00 | 31.36 | C |
| ATOM | 477 | C | VAL A | 58 | 28.894 | 57.469 | 35.152 | 1.00 | 30.34 | C |
| ATOM | 478 | O | VAL A | 58 | 28.549 | 58.391 | 35.901 | 1.00 | 29.36 | O |
| ATOM | 479 | CB | VAL A | 58 | 28.200 | 58.116 | 32.757 | 1.00 | 29.23 | C |
| ATOM | 480 | CG1 | VAL A | 58 | 29.670 | 58.392 | 32.439 | 1.00 | 29.76 | C |
| ATOM | 481 | CG2 | VAL A | 58 | 27.533 | 57.567 | 31.542 | 1.00 | 27.61 | C |
| ATOM | 482 | N | PRO A | 59 | 30.001 | 56.736 | 35.370 | 1.00 | 29.71 | N |
| ATOM | 483 | CA | PRO A | 59 | 30.849 | 57.050 | 36.516 | 1.00 | 30.97 | C |
| ATOM | 484 | C | PRO A | 59 | 31.227 | 58.537 | 36.607 | 1.00 | 31.24 | C |
| ATOM | 485 | O | PRO A | 59 | 31.423 | 59.200 | 35.592 | 1.00 | 31.83 | O |
| ATOM | 486 | CB | PRO A | 59 | 32.092 | 56.174 | 36.301 | 1.00 | 30.71 | C |
| ATOM | 487 | CG | PRO A | 59 | 31.681 | 55.112 | 35.372 | 1.00 | 30.39 | C |
| ATOM | 488 | CD | PRO A | 59 | 30.520 | 55.606 | 34.582 | 1.00 | 29.72 | C |
| ATOM | 489 | N | ASP A | 60 | 31.320 | 59.041 | 37.828 | 1.00 | 31.31 | N |
| ATOM | 490 | CA | ASP A | 60 | 31.588 | 60.460 | 38.076 | 1.00 | 30.98 | C |
| ATOM | 491 | C | ASP A | 60 | 32.988 | 60.868 | 37.684 | 1.00 | 28.29 | C |
| ATOM | 492 | O | ASP A | 60 | 33.265 | 62.049 | 37.638 | 1.00 | 26.89 | O |
| ATOM | 493 | CB | ASP A | 60 | 31.402 | 60.764 | 39.550 | 1.00 | 32.56 | C |
| ATOM | 494 | CG | ASP A | 60 | 30.047 | 60.330 | 40.039 | 1.00 | 36.89 | C |
| ATOM | 495 | OD1 | ASP A | 60 | 29.041 | 60.996 | 39.680 | 1.00 | 38.14 | O |
| ATOM | 496 | OD2 | ASP A | 60 | 30.000 | 59.283 | 40.719 | 1.00 | 39.45 | O |
| ATOM | 497 | N | ARG A | 61 | 33.851 | 59.890 | 37.430 | 1.00 | 27.50 | N |
| ATOM | 498 | CA | ARG A | 61 | 35.238 | 60.140 | 37.021 | 1.00 | 28.43 | C |
| ATOM | 499 | C | ARG A | 61 | 35.369 | 60.726 | 35.604 | 1.00 | 25.91 | C |
| ATOM | 500 | O | ARG A | 61 | 36.428 | 61.188 | 35.224 | 1.00 | 26.42 | O |
| ATOM | 501 | CB | ARG A | 61 | 36.026 | 58.840 | 37.088 | 1.00 | 29.01 | C |
| ATOM | 502 | CG | ARG A | 61 | 36.310 | 58.357 | 38.493 | 1.00 | 30.71 | C |
| ATOM | 503 | CD | ARG A | 61 | 36.946 | 56.973 | 38.534 | 1.00 | 31.07 | C |
| ATOM | 504 | NE | ARG A | 61 | 36.239 | 55.923 | 37.778 | 1.00 | 32.25 | N |
| ATOM | 505 | CZ | ARG A | 61 | 36.500 | 55.562 | 36.524 | 1.00 | 32.67 | C |
| ATOM | 506 | NH1 | ARG A | 61 | 37.450 | 56.169 | 35.817 | 1.00 | 32.10 | N |
| ATOM | 507 | NH2 | ARG A | 61 | 35.797 | 54.584 | 35.963 | 1.00 | 31.84 | N |
| ATOM | 508 | N | PHE A | 62 | 34.302 | 60.652 | 34.823 | 1.00 | 26.18 | N |
| ATOM | 509 | CA | PHE A | 62 | 34.234 | 61.248 | 33.499 | 1.00 | 24.00 | C |
| ATOM | 510 | C | PHE A | 62 | 33.730 | 62.668 | 33.564 | 1.00 | 23.08 | C |

| ATOM | 511 | O | PHE | A | 62 | 32.843 | 62.998 | 34.338 | 1.00 | 22.23 | O |
| ATOM | 512 | CB | PHE | A | 62 | 33.305 | 60.452 | 32.609 | 1.00 | 25.59 | C |
| ATOM | 513 | CG | PHE | A | 62 | 33.819 | 59.095 | 32.258 | 1.00 | 27.17 | C |
| ATOM | 514 | CD1 | PHE | A | 62 | 33.552 | 58.000 | 33.066 | 1.00 | 28.98 | C |
| ATOM | 515 | CD2 | PHE | A | 62 | 34.540 | 58.899 | 31.104 | 1.00 | 27.85 | C |
| ATOM | 516 | CE1 | PHE | A | 62 | 34.041 | 56.735 | 32.730 | 1.00 | 29.30 | C |
| ATOM | 517 | CE2 | PHE | A | 62 | 35.012 | 57.645 | 30.763 | 1.00 | 28.49 | C |
| ATOM | 518 | CZ | PHE | A | 62 | 34.760 | 56.565 | 31.568 | 1.00 | 27.13 | C |
| ATOM | 519 | N | SER | A | 63 | 34.283 | 63.517 | 32.708 | 1.00 | 19.05 | N |
| ATOM | 520 | CA | SER | A | 63 | 33.890 | 64.885 | 32.644 | 1.00 | 19.10 | C |
| ATOM | 521 | C | SER | A | 63 | 34.375 | 65.460 | 31.315 | 1.00 | 17.82 | C |
| ATOM | 522 | O | SER | A | 63 | 35.129 | 64.838 | 30.593 | 1.00 | 15.71 | O |
| ATOM | 523 | CB | SER | A | 63 | 34.505 | 65.678 | 33.805 | 1.00 | 20.07 | C |
| ATOM | 524 | OG | SER | A | 63 | 35.897 | 65.924 | 33.560 | 1.00 | 21.37 | O |
| ATOM | 525 | N | SER | A | 64 | 33.889 | 66.627 | 30.988 | 1.00 | 18.99 | N |
| ATOM | 526 | CA | SER | A | 64 | 34.260 | 67.268 | 29.737 | 1.00 | 19.68 | C |
| ATOM | 527 | C | SER | A | 64 | 34.096 | 68.751 | 29.923 | 1.00 | 21.40 | C |
| ATOM | 528 | O | SER | A | 64 | 33.348 | 69.222 | 30.806 | 1.00 | 21.21 | O |
| ATOM | 529 | CB | SER | A | 64 | 33.399 | 66.735 | 28.587 | 1.00 | 19.49 | C |
| ATOM | 530 | OG | SER | A | 64 | 33.594 | 67.438 | 27.382 | 1.00 | 21.69 | O |
| ATOM | 531 | N | SER | A | 65 | 34.836 | 69.493 | 29.106 | 1.00 | 21.38 | N |
| ATOM | 532 | CA | SER | A | 65 | 34.810 | 70.928 | 29.167 | 1.00 | 22.85 | C |
| ATOM | 533 | C | SER | A | 65 | 35.407 | 71.422 | 27.874 | 1.00 | 21.65 | C |
| ATOM | 534 | O | SER | A | 65 | 35.965 | 70.654 | 27.113 | 1.00 | 20.02 | O |
| ATOM | 535 | CB | SER | A | 65 | 35.615 | 71.453 | 30.372 | 1.00 | 22.97 | C |
| ATOM | 536 | OG | SER | A | 65 | 37.013 | 71.385 | 30.108 | 1.00 | 25.60 | O |
| ATOM | 537 | N | GLY | A | 66 | 35.214 | 72.698 | 27.588 | 1.00 | 24.92 | N |
| ATOM | 538 | CA | GLY | A | 66 | 35.821 | 73.288 | 26.392 | 1.00 | 25.61 | C |
| ATOM | 539 | C | GLY | A | 66 | 35.125 | 74.559 | 25.970 | 1.00 | 26.76 | C |
| ATOM | 540 | O | GLY | A | 66 | 34.231 | 75.032 | 26.652 | 1.00 | 26.95 | O |
| ATOM | 541 | N | SER | A | 67 | 35.572 | 75.089 | 24.839 | 1.00 | 27.87 | N |
| ATOM | 542 | CA | SER | A | 67 | 34.955 | 76.212 | 24.170 | 1.00 | 29.44 | C |
| ATOM | 543 | C | SER | A | 67 | 33.901 | 75.671 | 23.221 | 1.00 | 29.86 | C |
| ATOM | 544 | O | SER | A | 67 | 33.562 | 74.512 | 23.281 | 1.00 | 29.28 | O |
| ATOM | 545 | CB | SER | A | 67 | 36.022 | 76.928 | 23.334 | 1.00 | 30.04 | C |
| ATOM | 546 | OG | SER | A | 67 | 36.311 | 76.169 | 22.172 | 1.00 | 29.10 | O |
| ATOM | 547 | N | GLY | A | 68 | 33.429 | 76.500 | 22.296 | 1.00 | 29.42 | N |
| ATOM | 548 | CA | GLY | A | 68 | 32.523 | 76.037 | 21.261 | 1.00 | 28.83 | C |
| ATOM | 549 | C | GLY | A | 68 | 33.157 | 75.162 | 20.201 | 1.00 | 28.08 | C |
| ATOM | 550 | O | GLY | A | 68 | 32.449 | 74.506 | 19.448 | 1.00 | 26.67 | O |
| ATOM | 551 | N | THR | A | 69 | 34.487 | 75.172 | 20.104 | 1.00 | 28.59 | N |
| ATOM | 552 | CA | THR | A | 69 | 35.174 | 74.445 | 19.041 | 1.00 | 27.96 | C |
| ATOM | 553 | C | THR | A | 69 | 36.196 | 73.432 | 19.501 | 1.00 | 26.17 | C |
| ATOM | 554 | O | THR | A | 69 | 36.546 | 72.555 | 18.731 | 1.00 | 26.82 | O |
| ATOM | 555 | CB | THR | A | 69 | 35.922 | 75.407 | 18.058 | 1.00 | 30.66 | C |
| ATOM | 556 | OG1 | THR | A | 69 | 36.757 | 76.301 | 18.795 | 1.00 | 33.71 | O |
| ATOM | 557 | CG2 | THR | A | 69 | 34.939 | 76.198 | 17.190 | 1.00 | 32.11 | C |
| ATOM | 558 | N | ASP | A | 70 | 36.715 | 73.574 | 20.720 | 1.00 | 25.72 | N |
| ATOM | 559 | CA | ASP | A | 70 | 37.743 | 72.658 | 21.278 | 1.00 | 24.95 | C |
| ATOM | 560 | C | ASP | A | 70 | 37.262 | 72.025 | 22.559 | 1.00 | 22.74 | C |
| ATOM | 561 | O | ASP | A | 70 | 36.932 | 72.720 | 23.494 | 1.00 | 20.86 | O |
| ATOM | 562 | CB | ASP | A | 70 | 38.991 | 73.405 | 21.629 | 1.00 | 25.31 | C |
| ATOM | 563 | CG | ASP | A | 70 | 39.746 | 73.809 | 20.418 | 1.00 | 25.73 | C |
| ATOM | 564 | OD1 | ASP | A | 70 | 40.388 | 72.944 | 19.846 | 1.00 | 29.41 | O |
| ATOM | 565 | OD2 | ASP | A | 70 | 39.634 | 74.969 | 20.015 | 1.00 | 33.10 | O |
| ATOM | 566 | N | PHE | A | 71 | 37.304 | 70.708 | 22.613 | 1.00 | 22.87 | N |
| ATOM | 567 | CA | PHE | A | 71 | 36.719 | 69.985 | 23.764 | 1.00 | 22.24 | C |
| ATOM | 568 | C | PHE | A | 71 | 37.695 | 68.947 | 24.268 | 1.00 | 21.48 | C |
| ATOM | 569 | O | PHE | A | 71 | 38.498 | 68.414 | 23.497 | 1.00 | 21.10 | O |
| ATOM | 570 | CB | PHE | A | 71 | 35.421 | 69.319 | 23.345 | 1.00 | 22.83 | C |
| ATOM | 571 | CG | PHE | A | 71 | 34.664 | 70.077 | 22.284 | 1.00 | 22.15 | C |
| ATOM | 572 | CD1 | PHE | A | 71 | 33.947 | 71.239 | 22.599 | 1.00 | 23.44 | C |
| ATOM | 573 | CD2 | PHE | A | 71 | 34.668 | 69.630 | 20.977 | 1.00 | 23.42 | C |
| ATOM | 574 | CE1 | PHE | A | 71 | 33.263 | 71.906 | 21.626 | 1.00 | 22.90 | C |
| ATOM | 575 | CE2 | PHE | A | 71 | 33.985 | 70.294 | 20.005 | 1.00 | 22.81 | C |

| ATOM | 576 | CZ | PHE | A | 71 | 33.295 | 71.435 | 20.320 | 1.00 | 24.25 | C |
|------|-----|-----|-----|---|----|--------|--------|--------|------|-------|---|
| ATOM | 577 | N | THR | A | 72 | 37.653 | 68.685 | 25.581 | 1.00 | 20.57 | N |
| ATOM | 578 | CA | THR | A | 72 | 38.452 | 67.652 | 26.172 | 1.00 | 19.14 | C |
| ATOM | 579 | C | THR | A | 72 | 37.582 | 66.817 | 27.110 | 1.00 | 19.20 | C |
| ATOM | 580 | O | THR | A | 72 | 37.020 | 67.338 | 28.072 | 1.00 | 18.61 | O |
| ATOM | 581 | CB | THR | A | 72 | 39.640 | 68.254 | 26.939 | 1.00 | 20.77 | C |
| ATOM | 582 | OG1 | THR | A | 72 | 40.344 | 69.187 | 26.087 | 1.00 | 22.43 | O |
| ATOM | 583 | CG2 | THR | A | 72 | 40.594 | 67.155 | 27.357 | 1.00 | 19.88 | C |
| ATOM | 584 | N | LEU | A | 73 | 37.470 | 65.535 | 26.802 | 1.00 | 18.81 | N |
| ATOM | 585 | CA | LEU | A | 73 | 36.929 | 64.551 | 27.729 | 1.00 | 18.16 | C |
| ATOM | 586 | C | LEU | A | 73 | 38.042 | 64.128 | 28.678 | 1.00 | 19.56 | C |
| ATOM | 587 | O | LEU | A | 73 | 39.134 | 63.817 | 28.230 | 1.00 | 18.51 | O |
| ATOM | 588 | CB | LEU | A | 73 | 36.471 | 63.366 | 26.937 | 1.00 | 18.26 | C |
| ATOM | 589 | CG | LEU | A | 73 | 35.956 | 62.172 | 27.712 | 1.00 | 20.46 | C |
| ATOM | 590 | CD1 | LEU | A | 73 | 34.534 | 62.489 | 28.110 | 1.00 | 19.22 | C |
| ATOM | 591 | CD2 | LEU | A | 73 | 36.059 | 60.957 | 26.843 | 1.00 | 22.23 | C |
| ATOM | 592 | N | ARG | A | 74 | 37.778 | 64.117 | 29.993 | 1.00 | 19.51 | N |
| ATOM | 593 | CA | ARG | A | 74 | 38.754 | 63.707 | 30.976 | 1.00 | 20.23 | C |
| ATOM | 594 | C | ARG | A | 74 | 38.206 | 62.560 | 31.813 | 1.00 | 22.03 | C |
| ATOM | 595 | O | ARG | A | 74 | 37.020 | 62.559 | 32.208 | 1.00 | 21.03 | O |
| ATOM | 596 | CB | ARG | A | 74 | 39.127 | 64.874 | 31.885 | 1.00 | 21.44 | C |
| ATOM | 597 | CG | ARG | A | 74 | 39.896 | 65.975 | 31.142 | 1.00 | 20.83 | C |
| ATOM | 598 | CD | ARG | A | 74 | 40.407 | 67.085 | 32.055 | 1.00 | 20.44 | C |
| ATOM | 599 | NE | ARG | A | 74 | 40.983 | 68.166 | 31.239 | 1.00 | 20.33 | N |
| ATOM | 600 | CZ | ARG | A | 74 | 42.180 | 68.145 | 30.656 | 1.00 | 20.30 | C |
| ATOM | 601 | NH1 | ARG | A | 74 | 42.975 | 67.127 | 30.794 | 1.00 | 20.28 | N |
| ATOM | 602 | NH2 | ARG | A | 74 | 42.591 | 69.179 | 29.926 | 1.00 | 20.46 | N |
| ATOM | 603 | N | ILE | A | 75 | 39.084 | 61.606 | 32.082 | 1.00 | 21.66 | N |
| ATOM | 604 | CA | ILE | A | 75 | 38.756 | 60.452 | 32.928 | 1.00 | 22.63 | C |
| ATOM | 605 | C | ILE | A | 75 | 39.787 | 60.342 | 34.044 | 1.00 | 22.52 | C |
| ATOM | 606 | O | ILE | A | 75 | 40.924 | 59.946 | 33.813 | 1.00 | 23.36 | O |
| ATOM | 607 | CB | ILE | A | 75 | 38.755 | 59.121 | 32.188 | 1.00 | 21.90 | C |
| ATOM | 608 | CG1 | ILE | A | 75 | 38.205 | 59.262 | 30.778 | 1.00 | 23.40 | C |
| ATOM | 609 | CG2 | ILE | A | 75 | 37.931 | 58.101 | 33.037 | 1.00 | 24.05 | C |
| ATOM | 610 | CD1 | ILE | A | 75 | 38.411 | 58.045 | 29.874 | 1.00 | 22.41 | C |
| ATOM | 611 | N | SER | A | 76 | 39.393 | 60.704 | 35.254 | 1.00 | 22.69 | N |
| ATOM | 612 | CA | SER | A | 76 | 40.258 | 60.524 | 36.408 | 1.00 | 24.26 | C |
| ATOM | 613 | C | SER | A | 76 | 40.281 | 59.051 | 36.831 | 1.00 | 25.87 | C |
| ATOM | 614 | O | SER | A | 76 | 39.317 | 58.293 | 36.590 | 1.00 | 27.26 | O |
| ATOM | 615 | CB | SER | A | 76 | 39.782 | 61.386 | 37.567 | 1.00 | 24.40 | C |
| ATOM | 616 | OG | SER | A | 76 | 38.514 | 60.981 | 38.033 | 1.00 | 24.33 | O |
| ATOM | 617 | N | ARG | A | 77 | 41.384 | 58.676 | 37.460 | 1.00 | 26.47 | N |
| ATOM | 618 | CA | ARG | A | 77 | 41.578 | 57.356 | 38.050 | 1.00 | 26.76 | C |
| ATOM | 619 | C | ARG | A | 77 | 41.051 | 56.256 | 37.147 | 1.00 | 26.19 | C |
| ATOM | 620 | O | ARG | A | 77 | 40.096 | 55.546 | 37.475 | 1.00 | 23.27 | O |
| ATOM | 621 | CB | ARG | A | 77 | 40.974 | 57.323 | 39.445 | 1.00 | 27.06 | C |
| ATOM | 622 | CG | ARG | A | 77 | 41.542 | 58.422 | 40.323 | 1.00 | 27.88 | C |
| ATOM | 623 | CD | ARG | A | 77 | 40.932 | 58.362 | 41.711 | 1.00 | 26.22 | C |
| ATOM | 624 | NE | ARG | A | 77 | 39.560 | 58.838 | 41.725 | 1.00 | 26.00 | N |
| ATOM | 625 | CZ | ARG | A | 77 | 39.216 | 60.111 | 41.668 | 1.00 | 25.55 | C |
| ATOM | 626 | NH1 | ARG | A | 77 | 40.144 | 61.057 | 41.546 | 1.00 | 24.82 | N |
| ATOM | 627 | NH2 | ARG | A | 77 | 37.934 | 60.434 | 41.694 | 1.00 | 24.71 | N |
| ATOM | 628 | N | VAL | A | 78 | 41.724 | 56.127 | 36.010 | 1.00 | 25.66 | N |
| ATOM | 629 | CA | VAL | A | 78 | 41.280 | 55.261 | 34.944 | 1.00 | 26.44 | C |
| ATOM | 630 | C | VAL | A | 78 | 41.277 | 53.822 | 35.384 | 1.00 | 26.50 | C |
| ATOM | 631 | O | VAL | A | 78 | 42.252 | 53.351 | 35.960 | 1.00 | 24.22 | O |
| ATOM | 632 | CB | VAL | A | 78 | 42.166 | 55.416 | 33.694 | 1.00 | 27.22 | C |
| ATOM | 633 | CG1 | VAL | A | 78 | 41.982 | 54.264 | 32.749 | 1.00 | 28.53 | C |
| ATOM | 634 | CG2 | VAL | A | 78 | 41.820 | 56.720 | 32.999 | 1.00 | 28.44 | C |
| ATOM | 635 | N | GLU | A | 79 | 40.169 | 53.146 | 35.080 | 1.00 | 27.25 | N |
| ATOM | 636 | CA | GLU | A | 79 | 39.991 | 51.716 | 35.349 | 1.00 | 28.98 | C |
| ATOM | 637 | C | GLU | A | 79 | 40.102 | 50.908 | 34.057 | 1.00 | 28.87 | C |
| ATOM | 638 | O | GLU | A | 79 | 39.865 | 51.409 | 32.949 | 1.00 | 26.58 | O |
| ATOM | 639 | CB | GLU | A | 79 | 38.619 | 51.468 | 36.017 | 1.00 | 29.00 | C |
| ATOM | 640 | CG | GLU | A | 79 | 38.493 | 52.132 | 37.372 | 1.00 | 30.60 | C |

| ATOM | 641 | CD | GLU | A | 79 | 37.166 | 51.854 | 38.070 | 1.00 | 32.70 | C |
| ATOM | 642 | OE1 | GLU | A | 79 | 36.952 | 52.389 | 39.186 | 1.00 | 34.75 | O |
| ATOM | 643 | OE2 | GLU | A | 79 | 36.341 | 51.099 | 37.508 | 1.00 | 37.65 | O |
| ATOM | 644 | N | ALA | A | 80 | 40.447 | 49.638 | 34.197 | 1.00 | 29.73 | N |
| ATOM | 645 | CA | ALA | A | 80 | 40.627 | 48.769 | 33.030 | 1.00 | 31.08 | C |
| ATOM | 646 | C | ALA | A | 80 | 39.351 | 48.675 | 32.167 | 1.00 | 31.70 | C |
| ATOM | 647 | O | ALA | A | 80 | 39.440 | 48.508 | 30.941 | 1.00 | 30.84 | O |
| ATOM | 648 | CB | ALA | A | 80 | 41.100 | 47.380 | 33.474 | 1.00 | 31.63 | C |
| ATOM | 649 | N | GLU | A | 81 | 38.181 | 48.808 | 32.806 | 1.00 | 31.32 | N |
| ATOM | 650 | CA | GLU | A | 81 | 36.886 | 48.812 | 32.102 | 1.00 | 33.26 | C |
| ATOM | 651 | C | GLU | A | 81 | 36.598 | 50.066 | 31.273 | 1.00 | 31.64 | C |
| ATOM | 652 | O | GLU | A | 81 | 35.707 | 50.048 | 30.433 | 1.00 | 31.26 | O |
| ATOM | 653 | CB | GLU | A | 81 | 35.715 | 48.608 | 33.082 | 1.00 | 34.05 | C |
| ATOM | 654 | CG | GLU | A | 81 | 35.477 | 49.794 | 34.014 | 1.00 | 36.87 | C |
| ATOM | 655 | CD | GLU | A | 81 | 34.219 | 49.650 | 34.868 | 1.00 | 38.93 | C |
| ATOM | 656 | OE1 | GLU | A | 81 | 33.643 | 48.530 | 34.927 | 1.00 | 44.06 | O |
| ATOM | 657 | OE2 | GLU | A | 81 | 33.796 | 50.681 | 35.471 | 1.00 | 44.54 | O |
| ATOM | 658 | N | ASP | A | 82 | 37.334 | 51.149 | 31.503 | 1.00 | 30.96 | N |
| ATOM | 659 | CA | ASP | A | 82 | 37.185 | 52.357 | 30.662 | 1.00 | 30.37 | C |
| ATOM | 660 | C | ASP | A | 82 | 37.716 | 52.186 | 29.234 | 1.00 | 28.91 | C |
| ATOM | 661 | O | ASP | A | 82 | 37.506 | 53.056 | 28.393 | 1.00 | 30.02 | O |
| ATOM | 662 | CB | ASP | A | 82 | 37.869 | 53.577 | 31.295 | 1.00 | 29.94 | C |
| ATOM | 663 | CG | ASP | A | 82 | 37.379 | 53.862 | 32.681 | 1.00 | 29.46 | C |
| ATOM | 664 | OD1 | ASP | A | 82 | 36.198 | 53.579 | 32.969 | 1.00 | 30.07 | O |
| ATOM | 665 | OD2 | ASP | A | 82 | 38.180 | 54.364 | 33.496 | 1.00 | 29.79 | O |
| ATOM | 666 | N | VAL | A | 83 | 38.402 | 51.086 | 28.959 | 1.00 | 27.99 | N |
| ATOM | 667 | CA | VAL | A | 83 | 38.986 | 50.861 | 27.649 | 1.00 | 25.47 | C |
| ATOM | 668 | C | VAL | A | 83 | 37.895 | 50.822 | 26.568 | 1.00 | 24.69 | C |
| ATOM | 669 | O | VAL | A | 83 | 36.751 | 50.384 | 26.798 | 1.00 | 21.01 | O |
| ATOM | 670 | CB | VAL | A | 83 | 39.901 | 49.598 | 27.629 | 1.00 | 26.37 | C |
| ATOM | 671 | CG1 | VAL | A | 83 | 39.106 | 48.339 | 27.508 | 1.00 | 26.07 | C |
| ATOM | 672 | CG2 | VAL | A | 83 | 40.943 | 49.702 | 26.490 | 1.00 | 26.41 | C |
| ATOM | 673 | N | GLY | A | 84 | 38.238 | 51.329 | 25.390 | 1.00 | 23.13 | N |
| ATOM | 674 | CA | GLY | A | 84 | 37.250 | 51.488 | 24.326 | 1.00 | 23.16 | C |
| ATOM | 675 | C | GLY | A | 84 | 37.594 | 52.629 | 23.411 | 1.00 | 22.86 | C |
| ATOM | 676 | O | GLY | A | 84 | 38.699 | 53.208 | 23.495 | 1.00 | 23.41 | O |
| ATOM | 677 | N | ILE | A | 85 | 36.646 | 52.927 | 22.526 | 1.00 | 23.61 | N |
| ATOM | 678 | CA | ILE | A | 85 | 36.771 | 53.995 | 21.548 | 1.00 | 23.23 | C |
| ATOM | 679 | C | ILE | A | 85 | 35.758 | 55.064 | 21.915 | 1.00 | 21.41 | C |
| ATOM | 680 | O | ILE | A | 85 | 34.573 | 54.767 | 22.095 | 1.00 | 19.47 | O |
| ATOM | 681 | CB | ILE | A | 85 | 36.550 | 53.488 | 20.098 | 1.00 | 24.06 | C |
| ATOM | 682 | CG1 | ILE | A | 85 | 37.489 | 52.323 | 19.812 | 1.00 | 24.86 | C |
| ATOM | 683 | CG2 | ILE | A | 85 | 36.771 | 54.642 | 19.053 | 1.00 | 24.05 | C |
| ATOM | 684 | CD1 | ILE | A | 85 | 37.625 | 52.016 | 18.351 | 1.00 | 27.32 | C |
| ATOM | 685 | N | TYR | A | 86 | 36.262 | 56.286 | 22.063 | 1.00 | 20.63 | N |
| ATOM | 686 | CA | TYR | A | 86 | 35.476 | 57.447 | 22.489 | 1.00 | 20.85 | C |
| ATOM | 687 | C | TYR | A | 86 | 35.209 | 58.364 | 21.321 | 1.00 | 20.56 | C |
| ATOM | 688 | O | TYR | A | 86 | 36.173 | 58.785 | 20.642 | 1.00 | 21.64 | O |
| ATOM | 689 | CB | TYR | A | 86 | 36.221 | 58.230 | 23.588 | 1.00 | 21.80 | C |
| ATOM | 690 | CG | TYR | A | 86 | 36.324 | 57.463 | 24.881 | 1.00 | 21.42 | C |
| ATOM | 691 | CD1 | TYR | A | 86 | 37.331 | 56.524 | 25.071 | 1.00 | 21.98 | C |
| ATOM | 692 | CD2 | TYR | A | 86 | 35.402 | 57.641 | 25.889 | 1.00 | 22.49 | C |
| ATOM | 693 | CE1 | TYR | A | 86 | 37.420 | 55.800 | 26.226 | 1.00 | 22.74 | C |
| ATOM | 694 | CE2 | TYR | A | 86 | 35.497 | 56.935 | 27.069 | 1.00 | 23.60 | C |
| ATOM | 695 | CZ | TYR | A | 86 | 36.499 | 56.009 | 27.225 | 1.00 | 22.16 | C |
| ATOM | 696 | OH | TYR | A | 86 | 36.580 | 55.285 | 28.385 | 1.00 | 23.64 | O |
| ATOM | 697 | N | TYR | A | 87 | 33.937 | 58.685 | 21.086 | 1.00 | 18.97 | N |
| ATOM | 698 | CA | TYR | A | 87 | 33.507 | 59.509 | 19.923 | 1.00 | 19.78 | C |
| ATOM | 699 | C | TYR | A | 87 | 32.943 | 60.846 | 20.357 | 1.00 | 18.12 | C |
| ATOM | 700 | O | TYR | A | 87 | 32.159 | 60.891 | 21.282 | 1.00 | 18.04 | O |
| ATOM | 701 | CB | TYR | A | 87 | 32.416 | 58.795 | 19.130 | 1.00 | 20.06 | C |
| ATOM | 702 | CG | TYR | A | 87 | 32.870 | 57.540 | 18.454 | 1.00 | 21.81 | C |
| ATOM | 703 | CD1 | TYR | A | 87 | 33.455 | 57.581 | 17.188 | 1.00 | 21.61 | C |
| ATOM | 704 | CD2 | TYR | A | 87 | 32.729 | 56.296 | 19.073 | 1.00 | 21.88 | C |
| ATOM | 705 | CE1 | TYR | A | 87 | 33.881 | 56.423 | 16.559 | 1.00 | 22.32 | C |

| ATOM | 706 | CE2 | TYR | A | 87 | 33.151 | 55.139 | 18.440 | 1.00 | 21.91 | C |
|------|-----|-----|-----|---|-----|--------|--------|--------|------|-------|---|
| ATOM | 707 | CZ | TYR | A | 87 | 33.698 | 55.203 | 17.187 | 1.00 | 23.32 | C |
| ATOM | 708 | OH | TYR | A | 87 | 34.138 | 54.042 | 16.603 | 1.00 | 24.80 | O |
| ATOM | 709 | N | CYS | A | 88 | 33.344 | 61.941 | 19.705 | 1.00 | 18.28 | N |
| ATOM | 710 | CA | CYS | A | 88 | 32.642 | 63.218 | 19.865 | 1.00 | 18.40 | C |
| ATOM | 711 | C | CYS | A | 88 | 31.562 | 63.374 | 18.769 | 1.00 | 18.71 | C |
| ATOM | 712 | O | CYS | A | 88 | 31.615 | 62.729 | 17.727 | 1.00 | 19.59 | O |
| ATOM | 713 | CB | CYS | A | 88 | 33.632 | 64.383 | 19.868 | 1.00 | 20.44 | C |
| ATOM | 714 | SG | CYS | A | 88 | 34.728 | 64.408 | 18.477 | 1.00 | 22.94 | S |
| ATOM | 715 | N | ALA | A | 89 | 30.537 | 64.177 | 19.036 | 1.00 | 19.20 | N |
| ATOM | 716 | CA | ALA | A | 89 | 29.452 | 64.407 | 18.069 | 1.00 | 19.60 | C |
| ATOM | 717 | C | ALA | A | 89 | 28.756 | 65.700 | 18.420 | 1.00 | 19.26 | C |
| ATOM | 718 | O | ALA | A | 89 | 28.903 | 66.202 | 19.532 | 1.00 | 16.35 | O |
| ATOM | 719 | CB | ALA | A | 89 | 28.421 | 63.214 | 18.072 | 1.00 | 20.05 | C |
| ATOM | 720 | N | HIS | A | 90 | 28.040 | 66.260 | 17.444 | 1.00 | 20.95 | N |
| ATOM | 721 | CA | HIS | A | 90 | 27.279 | 67.496 | 17.619 | 1.00 | 23.21 | C |
| ATOM | 722 | C | HIS | A | 90 | 25.862 | 67.168 | 17.249 | 1.00 | 25.06 | C |
| ATOM | 723 | O | HIS | A | 90 | 25.621 | 66.125 | 16.627 | 1.00 | 25.14 | O |
| ATOM | 724 | CB | HIS | A | 90 | 27.763 | 68.582 | 16.648 | 1.00 | 23.65 | C |
| ATOM | 725 | CG | HIS | A | 90 | 27.390 | 68.310 | 15.220 | 1.00 | 23.42 | C |
| ATOM | 726 | ND1 | HIS | A | 90 | 28.280 | 67.807 | 14.298 | 1.00 | 25.77 | N |
| ATOM | 727 | CD2 | HIS | A | 90 | 26.211 | 68.422 | 14.571 | 1.00 | 23.49 | C |
| ATOM | 728 | CE1 | HIS | A | 90 | 27.666 | 67.639 | 13.142 | 1.00 | 23.39 | C |
| ATOM | 729 | NE2 | HIS | A | 90 | 26.410 | 67.998 | 13.283 | 1.00 | 25.10 | N |
| ATOM | 730 | N | ASN | A | 91 | 24.938 | 68.067 | 17.576 | 1.00 | 29.78 | N |
| ATOM | 731 | CA | ASN | A | 91 | 23.599 | 68.085 | 16.940 | 1.00 | 32.38 | C |
| ATOM | 732 | C | ASN | A | 91 | 23.129 | 69.503 | 16.635 | 1.00 | 33.44 | C |
| ATOM | 733 | O | ASN | A | 91 | 22.107 | 69.958 | 17.139 | 1.00 | 35.01 | O |
| ATOM | 734 | CB | ASN | A | 91 | 22.544 | 67.326 | 17.770 | 1.00 | 34.28 | C |
| ATOM | 735 | CG | ASN | A | 91 | 22.249 | 67.986 | 19.085 | 1.00 | 35.34 | C |
| ATOM | 736 | OD1 | ASN | A | 91 | 22.985 | 68.874 | 19.496 | 1.00 | 38.05 | O |
| ATOM | 737 | ND2 | ASN | A | 91 | 21.152 | 67.586 | 19.745 | 1.00 | 35.96 | N |
| ATOM | 738 | N | VAL | A | 92 | 23.874 | 70.204 | 15.797 | 1.00 | 32.65 | N |
| ATOM | 739 | CA | VAL | A | 92 | 23.521 | 71.559 | 15.395 | 1.00 | 32.99 | C |
| ATOM | 740 | C | VAL | A | 92 | 23.119 | 71.678 | 13.934 | 1.00 | 33.52 | C |
| ATOM | 741 | O | VAL | A | 92 | 22.736 | 72.752 | 13.489 | 1.00 | 34.32 | O |
| ATOM | 742 | CB | VAL | A | 92 | 24.682 | 72.500 | 15.642 | 1.00 | 32.97 | C |
| ATOM | 743 | CG1 | VAL | A | 92 | 25.112 | 72.385 | 17.100 | 1.00 | 33.22 | C |
| ATOM | 744 | CG2 | VAL | A | 92 | 25.829 | 72.196 | 14.667 | 1.00 | 31.51 | C |
| ATOM | 745 | N | GLU | A | 93 | 23.231 | 70.587 | 13.189 | 1.00 | 32.88 | N |
| ATOM | 746 | CA | GLU | A | 93 | 22.742 | 70.524 | 11.824 | 1.00 | 32.58 | C |
| ATOM | 747 | C | GLU | A | 93 | 22.652 | 69.067 | 11.413 | 1.00 | 32.48 | C |
| ATOM | 748 | O | GLU | A | 93 | 23.163 | 68.202 | 12.101 | 1.00 | 30.95 | O |
| ATOM | 749 | CB | GLU | A | 93 | 23.667 | 71.288 | 10.855 | 1.00 | 33.23 | C |
| ATOM | 750 | CG | GLU | A | 93 | 25.078 | 70.787 | 10.791 | 1.00 | 32.18 | C |
| ATOM | 751 | CD | GLU | A | 93 | 25.959 | 71.619 | 9.887 | 1.00 | 34.10 | C |
| ATOM | 752 | OE1 | GLU | A | 93 | 26.000 | 72.860 | 10.053 | 1.00 | 34.02 | O |
| ATOM | 753 | OE2 | GLU | A | 93 | 26.639 | 71.020 | 9.028 | 1.00 | 34.61 | O |
| ATOM | 754 | N | LEU | A | 94 | 21.948 | 68.806 | 10.318 | 1.00 | 32.56 | N |
| ATOM | 755 | CA | LEU | A | 94 | 22.083 | 67.562 | 9.608 | 1.00 | 32.21 | C |
| ATOM | 756 | C | LEU | A | 94 | 23.035 | 67.919 | 8.487 | 1.00 | 31.40 | C |
| ATOM | 757 | O | LEU | A | 94 | 23.059 | 69.068 | 8.067 | 1.00 | 34.47 | O |
| ATOM | 758 | CB | LEU | A | 94 | 20.751 | 67.075 | 9.069 | 1.00 | 32.25 | C |
| ATOM | 759 | CG | LEU | A | 94 | 19.663 | 66.763 | 10.105 | 1.00 | 32.40 | C |
| ATOM | 760 | CD1 | LEU | A | 94 | 18.434 | 66.199 | 9.400 | 1.00 | 32.29 | C |
| ATOM | 761 | CD2 | LEU | A | 94 | 20.140 | 65.802 | 11.128 | 1.00 | 32.89 | C |
| ATOM | 762 | N | PRO | A | 95 | 23.877 | 66.977 | 8.052 | 1.00 | 30.38 | N |
| ATOM | 763 | CA | PRO | A | 95 | 24.038 | 65.629 | 8.575 | 1.00 | 29.77 | C |
| ATOM | 764 | C | PRO | A | 95 | 24.678 | 65.618 | 9.957 | 1.00 | 28.99 | C |
| ATOM | 765 | O | PRO | A | 95 | 25.585 | 66.412 | 10.235 | 1.00 | 27.63 | O |
| ATOM | 766 | CB | PRO | A | 95 | 24.967 | 64.979 | 7.572 | 1.00 | 29.81 | C |
| ATOM | 767 | CG | PRO | A | 95 | 25.761 | 66.131 | 7.013 | 1.00 | 30.86 | C |
| ATOM | 768 | CD | PRO | A | 95 | 24.824 | 67.258 | 6.961 | 1.00 | 30.86 | C |
| ATOM | 769 | N | ARG | A | 96 | 24.203 | 64.732 | 10.825 | 1.00 | 28.70 | N |
| ATOM | 770 | CA | ARG | A | 96 | 24.854 | 64.536 | 12.102 | 1.00 | 28.08 | C |

| ATOM | 771 | C   | ARG | A | 96  | 26.180 | 63.842 | 11.813 | 1.00 | 25.64 | C |
| ---- | --- | --- | --- | - | --- | ------ | ------ | ------ | ---- | ----- | - |
| ATOM | 772 | O   | ARG | A | 96  | 26.239 | 62.914 | 11.023 | 1.00 | 25.90 | O |
| ATOM | 773 | CB  | ARG | A | 96  | 23.995 | 63.669 | 13.016 | 1.00 | 28.13 | C |
| ATOM | 774 | CG  | ARG | A | 96  | 22.648 | 64.258 | 13.381 | 1.00 | 29.69 | C |
| ATOM | 775 | CD  | ARG | A | 96  | 21.834 | 63.241 | 14.236 | 1.00 | 31.56 | C |
| ATOM | 776 | NE  | ARG | A | 96  | 20.449 | 63.704 | 14.414 | 1.00 | 33.49 | N |
| ATOM | 777 | CZ  | ARG | A | 96  | 20.043 | 64.537 | 15.376 | 1.00 | 33.72 | C |
| ATOM | 778 | NH1 | ARG | A | 96  | 20.891 | 65.013 | 16.271 | 1.00 | 33.03 | N |
| ATOM | 779 | NH2 | ARG | A | 96  | 18.769 | 64.912 | 15.438 | 1.00 | 32.71 | N |
| ATOM | 780 | N   | THR | A | 97  | 27.249 | 64.302 | 12.438 | 1.00 | 24.50 | N |
| ATOM | 781 | CA  | THR | A | 97  | 28.546 | 63.733 | 12.194 | 1.00 | 23.39 | C |
| ATOM | 782 | C   | THR | A | 97  | 29.273 | 63.490 | 13.477 | 1.00 | 22.16 | C |
| ATOM | 783 | O   | THR | A | 97  | 29.059 | 64.173 | 14.470 | 1.00 | 23.57 | O |
| ATOM | 784 | CB  | THR | A | 97  | 29.435 | 64.606 | 11.257 | 1.00 | 24.10 | C |
| ATOM | 785 | OG1 | THR | A | 97  | 29.823 | 65.800 | 11.940 | 1.00 | 24.23 | O |
| ATOM | 786 | CG2 | THR | A | 97  | 28.735 | 64.936 | 9.942  | 1.00 | 24.48 | C |
| ATOM | 787 | N   | PHE | A | 98  | 30.156 | 62.503 | 13.411 | 1.00 | 22.50 | N |
| ATOM | 788 | CA  | PHE | A | 98  | 30.929 | 61.996 | 14.523 | 1.00 | 20.89 | C |
| ATOM | 789 | C   | PHE | A | 98  | 32.395 | 62.160 | 14.236 | 1.00 | 21.05 | C |
| ATOM | 790 | O   | PHE | A | 98  | 32.790 | 62.194 | 13.084 | 1.00 | 19.41 | O |
| ATOM | 791 | CB  | PHE | A | 98  | 30.667 | 60.517 | 14.691 | 1.00 | 21.14 | C |
| ATOM | 792 | CG  | PHE | A | 98  | 29.248 | 60.217 | 14.998 | 1.00 | 20.61 | C |
| ATOM | 793 | CD1 | PHE | A | 98  | 28.314 | 60.165 | 13.979 | 1.00 | 22.13 | C |
| ATOM | 794 | CD2 | PHE | A | 98  | 28.843 | 60.050 | 16.297 | 1.00 | 21.39 | C |
| ATOM | 795 | CE1 | PHE | A | 98  | 26.965 | 59.897 | 14.264 | 1.00 | 22.48 | C |
| ATOM | 796 | CE2 | PHE | A | 98  | 27.507 | 59.772 | 16.594 | 1.00 | 22.30 | C |
| ATOM | 797 | CZ  | PHE | A | 98  | 26.576 | 59.718 | 15.571 | 1.00 | 21.82 | C |
| ATOM | 798 | N   | GLY | A | 99  | 33.198 | 62.238 | 15.286 | 1.00 | 20.15 | N |
| ATOM | 799 | CA  | GLY | A | 99  | 34.621 | 62.118 | 15.115 | 1.00 | 22.48 | C |
| ATOM | 800 | C   | GLY | A | 99  | 34.987 | 60.698 | 14.739 | 1.00 | 22.92 | C |
| ATOM | 801 | O   | GLY | A | 99  | 34.152 | 59.782 | 14.825 | 1.00 | 23.02 | O |
| ATOM | 802 | N   | GLY | A | 100 | 36.234 | 60.501 | 14.332 | 1.00 | 21.19 | N |
| ATOM | 803 | CA  | GLY | A | 100 | 36.735 | 59.172 | 13.975 | 1.00 | 20.71 | C |
| ATOM | 804 | C   | GLY | A | 100 | 36.898 | 58.196 | 15.131 | 1.00 | 21.42 | C |
| ATOM | 805 | O   | GLY | A | 100 | 37.049 | 57.012 | 14.922 | 1.00 | 21.40 | O |
| ATOM | 806 | N   | GLY | A | 101 | 36.870 | 58.683 | 16.364 | 1.00 | 22.91 | N |
| ATOM | 807 | CA  | GLY | A | 101 | 36.981 | 57.821 | 17.527 | 1.00 | 23.40 | C |
| ATOM | 808 | C   | GLY | A | 101 | 38.422 | 57.772 | 17.964 | 1.00 | 24.78 | C |
| ATOM | 809 | O   | GLY | A | 101 | 39.331 | 57.653 | 17.135 | 1.00 | 25.26 | O |
| ATOM | 810 | N   | THR | A | 102 | 38.638 | 57.906 | 19.267 | 1.00 | 24.84 | N |
| ATOM | 811 | CA  | THR | A | 102 | 39.969 | 57.863 | 19.854 | 1.00 | 23.56 | C |
| ATOM | 812 | C   | THR | A | 102 | 39.983 | 56.650 | 20.753 | 1.00 | 23.99 | C |
| ATOM | 813 | O   | THR | A | 102 | 39.131 | 56.521 | 21.629 | 1.00 | 21.69 | O |
| ATOM | 814 | CB  | THR | A | 102 | 40.274 | 59.120 | 20.699 | 1.00 | 24.45 | C |
| ATOM | 815 | OG1 | THR | A | 102 | 40.263 | 60.298 | 19.874 | 1.00 | 23.77 | O |
| ATOM | 816 | CG2 | THR | A | 102 | 41.632 | 58.989 | 21.396 | 1.00 | 25.15 | C |
| ATOM | 817 | N   | LYS | A | 103 | 40.946 | 55.754 | 20.530 | 1.00 | 24.75 | N |
| ATOM | 818 | CA  | LYS | A | 103 | 41.087 | 54.579 | 21.385 | 1.00 | 26.09 | C |
| ATOM | 819 | C   | LYS | A | 103 | 41.868 | 54.970 | 22.645 | 1.00 | 25.27 | C |
| ATOM | 820 | O   | LYS | A | 103 | 42.953 | 55.551 | 22.554 | 1.00 | 20.64 | O |
| ATOM | 821 | CB  | LYS | A | 103 | 41.754 | 53.421 | 20.639 | 1.00 | 27.85 | C |
| ATOM | 822 | CG  | LYS | A | 103 | 41.866 | 52.151 | 21.485 | 1.00 | 31.35 | C |
| ATOM | 823 | CD  | LYS | A | 103 | 41.863 | 50.896 | 20.635 | 1.00 | 36.42 | C |
| ATOM | 824 | CE  | LYS | A | 103 | 40.499 | 50.198 | 20.652 | 1.00 | 37.91 | C |
| ATOM | 825 | NZ  | LYS | A | 103 | 40.349 | 49.303 | 19.478 | 1.00 | 39.23 | N |
| ATOM | 826 | N   | LEU | A | 104 | 41.267 | 54.701 | 23.811 | 1.00 | 24.35 | N |
| ATOM | 827 | CA  | LEU | A | 104 | 41.903 | 54.931 | 25.089 | 1.00 | 25.81 | C |
| ATOM | 828 | C   | LEU | A | 104 | 42.849 | 53.765 | 25.357 | 1.00 | 27.26 | C |
| ATOM | 829 | O   | LEU | A | 104 | 42.424 | 52.619 | 25.442 | 1.00 | 25.15 | O |
| ATOM | 830 | CB  | LEU | A | 104 | 40.890 | 55.032 | 26.235 | 1.00 | 25.48 | C |
| ATOM | 831 | CG  | LEU | A | 104 | 41.493 | 55.228 | 27.648 | 1.00 | 25.87 | C |
| ATOM | 832 | CD1 | LEU | A | 104 | 42.064 | 56.623 | 27.859 | 1.00 | 25.55 | C |
| ATOM | 833 | CD2 | LEU | A | 104 | 40.444 | 54.939 | 28.756 | 1.00 | 26.44 | C |
| ATOM | 834 | N   | GLU | A | 105 | 44.129 | 54.092 | 25.514 | 1.00 | 29.73 | N |
| ATOM | 835 | CA  | GLU | A | 105 | 45.210 | 53.110 | 25.570 | 1.00 | 32.27 | C |

| ATOM | 836 | C | GLU | A | 105 | 45.712 | 52.999 | 27.004 | 1.00 | 32.20 | C |
|------|-----|-----|-----|---|-----|--------|--------|--------|------|-------|---|
| ATOM | 837 | O | GLU | A | 105 | 46.319 | 53.937 | 27.535 | 1.00 | 31.91 | O |
| ATOM | 838 | CB | GLU | A | 105 | 46.331 | 53.593 | 24.640 | 1.00 | 34.60 | C |
| ATOM | 839 | CG | GLU | A | 105 | 47.526 | 52.654 | 24.486 | 1.00 | 39.59 | C |
| ATOM | 840 | CD | GLU | A | 105 | 47.198 | 51.405 | 23.682 | 1.00 | 43.50 | C |
| ATOM | 841 | OE1 | GLU | A | 105 | 46.310 | 51.504 | 22.781 | 1.00 | 44.85 | O |
| ATOM | 842 | OE2 | GLU | A | 105 | 47.833 | 50.339 | 23.971 | 1.00 | 45.32 | O |
| ATOM | 843 | N | ILE | A | 106 | 45.440 | 51.859 | 27.633 | 1.00 | 32.72 | N |
| ATOM | 844 | CA | ILE | A | 106 | 45.806 | 51.620 | 29.015 | 1.00 | 32.30 | C |
| ATOM | 845 | C | ILE | A | 106 | 47.189 | 51.041 | 29.056 | 1.00 | 33.09 | C |
| ATOM | 846 | O | ILE | A | 106 | 47.508 | 50.106 | 28.306 | 1.00 | 32.39 | O |
| ATOM | 847 | CB | ILE | A | 106 | 44.873 | 50.601 | 29.728 | 1.00 | 33.14 | C |
| ATOM | 848 | CG1 | ILE | A | 106 | 43.388 | 50.935 | 29.511 | 1.00 | 32.78 | C |
| ATOM | 849 | CG2 | ILE | A | 106 | 45.208 | 50.525 | 31.194 | 1.00 | 32.99 | C |
| ATOM | 850 | CD1 | ILE | A | 106 | 42.988 | 52.262 | 29.936 | 1.00 | 33.91 | C |
| ATOM | 851 | N | LYS | A | 107 | 48.007 | 51.590 | 29.947 | 1.00 | 33.99 | N |
| ATOM | 852 | CA | LYS | A | 107 | 49.375 | 51.161 | 30.126 | 1.00 | 35.52 | C |
| ATOM | 853 | C | LYS | A | 107 | 49.448 | 50.379 | 31.435 | 1.00 | 34.51 | C |
| ATOM | 854 | O | LYS | A | 107 | 48.986 | 50.848 | 32.457 | 1.00 | 36.00 | O |
| ATOM | 855 | CB | LYS | A | 107 | 50.308 | 52.372 | 30.135 | 1.00 | 35.41 | C |
| ATOM | 856 | CG | LYS | A | 107 | 51.544 | 52.170 | 30.944 | 1.00 | 38.10 | C |
| ATOM | 857 | CD | LYS | A | 107 | 52.606 | 53.217 | 30.644 | 1.00 | 39.27 | C |
| ATOM | 858 | CE | LYS | A | 107 | 53.990 | 52.739 | 31.078 | 1.00 | 40.67 | C |
| ATOM | 859 | NZ | LYS | A | 107 | 55.068 | 53.411 | 30.251 | 1.00 | 42.36 | N |
| ATOM | 860 | N | ARG | A | 108 | 49.998 | 49.169 | 31.382 | 1.00 | 34.52 | N |
| ATOM | 861 | CA | ARG | A | 108 | 50.102 | 48.288 | 32.547 | 1.00 | 32.74 | C |
| ATOM | 862 | C | ARG | A | 108 | 51.468 | 47.577 | 32.581 | 1.00 | 33.43 | C |
| ATOM | 863 | O | ARG | A | 108 | 52.342 | 47.819 | 31.735 | 1.00 | 33.03 | O |
| ATOM | 864 | CB | ARG | A | 108 | 48.941 | 47.287 | 32.568 | 1.00 | 32.26 | C |
| ATOM | 865 | CG | ARG | A | 108 | 48.776 | 46.449 | 31.285 | 1.00 | 31.86 | C |
| ATOM | 866 | CD | ARG | A | 108 | 48.275 | 45.036 | 31.614 | 1.00 | 30.87 | C |
| ATOM | 867 | NE | ARG | A | 108 | 49.328 | 44.223 | 32.225 | 1.00 | 30.76 | N |
| ATOM | 868 | CZ | ARG | A | 108 | 49.111 | 43.121 | 32.945 | 1.00 | 30.25 | C |
| ATOM | 869 | NH1 | ARG | A | 108 | 47.884 | 42.659 | 33.151 | 1.00 | 30.04 | N |
| ATOM | 870 | NH2 | ARG | A | 108 | 50.133 | 42.477 | 33.466 | 1.00 | 30.45 | N |
| ATOM | 871 | N | ALA | A | 109 | 51.649 | 46.730 | 33.590 | 1.00 | 34.47 | N |
| ATOM | 872 | CA | ALA | A | 109 | 52.833 | 45.877 | 33.715 | 1.00 | 34.55 | C |
| ATOM | 873 | C | ALA | A | 109 | 53.008 | 44.983 | 32.501 | 1.00 | 34.66 | C |
| ATOM | 874 | O | ALA | A | 109 | 52.030 | 44.464 | 31.976 | 1.00 | 34.33 | O |
| ATOM | 875 | CB | ALA | A | 109 | 52.708 | 45.017 | 34.974 | 1.00 | 34.94 | C |
| ATOM | 876 | N | ASP | A | 110 | 54.256 | 44.819 | 32.055 | 1.00 | 34.75 | N |
| ATOM | 877 | CA | ASP | A | 110 | 54.575 | 43.908 | 30.970 | 1.00 | 35.65 | C |
| ATOM | 878 | C | ASP | A | 110 | 54.154 | 42.492 | 31.312 | 1.00 | 34.77 | C |
| ATOM | 879 | O | ASP | A | 110 | 54.107 | 42.126 | 32.473 | 1.00 | 35.68 | O |
| ATOM | 880 | CB | ASP | A | 110 | 56.062 | 43.905 | 30.674 | 1.00 | 36.34 | C |
| ATOM | 881 | CG | ASP | A | 110 | 56.549 | 45.222 | 30.108 | 1.00 | 38.76 | C |
| ATOM | 882 | OD1 | ASP | A | 110 | 55.759 | 46.216 | 30.074 | 1.00 | 39.63 | O |
| ATOM | 883 | OD2 | ASP | A | 110 | 57.735 | 45.245 | 29.712 | 1.00 | 39.76 | O |
| ATOM | 884 | N | ALA | A | 111 | 53.806 | 41.719 | 30.288 | 1.00 | 34.40 | N |
| ATOM | 885 | CA | ALA | A | 111 | 53.419 | 40.324 | 30.477 | 1.00 | 32.71 | C |
| ATOM | 886 | C | ALA | A | 111 | 53.746 | 39.536 | 29.220 | 1.00 | 31.28 | C |
| ATOM | 887 | O | ALA | A | 111 | 53.356 | 39.907 | 28.120 | 1.00 | 31.53 | O |
| ATOM | 888 | CB | ALA | A | 111 | 51.954 | 40.216 | 30.814 | 1.00 | 32.15 | C |
| ATOM | 889 | N | ALA | A | 112 | 54.494 | 38.457 | 29.402 | 1.00 | 29.95 | N |
| ATOM | 890 | CA | ALA | A | 112 | 54.850 | 37.555 | 28.327 | 1.00 | 30.39 | C |
| ATOM | 891 | C | ALA | A | 112 | 53.610 | 36.806 | 27.806 | 1.00 | 29.41 | C |
| ATOM | 892 | O | ALA | A | 112 | 52.690 | 36.483 | 28.571 | 1.00 | 29.66 | O |
| ATOM | 893 | CB | ALA | A | 112 | 55.896 | 36.558 | 28.820 | 1.00 | 30.21 | C |
| ATOM | 894 | N | PRO | A | 113 | 53.581 | 36.516 | 26.510 | 1.00 | 28.62 | N |
| ATOM | 895 | CA | PRO | A | 113 | 52.437 | 35.764 | 25.994 | 1.00 | 28.48 | C |
| ATOM | 896 | C | PRO | A | 113 | 52.466 | 34.343 | 26.479 | 1.00 | 28.18 | C |
| ATOM | 897 | O | PRO | A | 113 | 53.542 | 33.764 | 26.600 | 1.00 | 27.29 | O |
| ATOM | 898 | CB | PRO | A | 113 | 52.684 | 35.758 | 24.492 | 1.00 | 27.78 | C |
| ATOM | 899 | CG | PRO | A | 113 | 54.142 | 35.880 | 24.370 | 1.00 | 28.72 | C |
| ATOM | 900 | CD | PRO | A | 113 | 54.557 | 36.798 | 25.451 | 1.00 | 28.95 | C |

| | | | | | | | | | | |
|---|---|---|---|---|---|---|---|---|---|---|
| ATOM | 901 | N | THR | A | 114 | 51.301 | 33.788 | 26.783 | 1.00 28.59 | N |
| ATOM | 902 | CA | THR | A | 114 | 51.184 | 32.333 | 26.898 | 1.00 27.44 | C |
| ATOM | 903 | C | THR | A | 114 | 51.014 | 31.776 | 25.491 | 1.00 25.13 | C |
| ATOM | 904 | O | THR | A | 114 | 50.026 | 32.100 | 24.822 | 1.00 24.42 | O |
| ATOM | 905 | CB | THR | A | 114 | 49.978 | 31.960 | 27.750 | 1.00 28.04 | C |
| ATOM | 906 | OG1 | THR | A | 114 | 50.112 | 32.560 | 29.042 | 1.00 29.31 | O |
| ATOM | 907 | CG2 | THR | A | 114 | 49.881 | 30.448 | 27.919 | 1.00 29.54 | C |
| ATOM | 908 | N | VAL | A | 115 | 51.963 | 30.950 | 25.034 | 1.00 24.04 | N |
| ATOM | 909 | CA | VAL | A | 115 | 51.900 | 30.371 | 23.686 | 1.00 23.29 | C |
| ATOM | 910 | C | VAL | A | 115 | 51.379 | 28.912 | 23.695 | 1.00 22.66 | C |
| ATOM | 911 | O | VAL | A | 115 | 51.798 | 28.121 | 24.508 | 1.00 22.51 | O |
| ATOM | 912 | CB | VAL | A | 115 | 53.263 | 30.462 | 22.985 | 1.00 24.17 | C |
| ATOM | 913 | CG1 | VAL | A | 115 | 53.151 | 30.076 | 21.550 | 1.00 24.77 | C |
| ATOM | 914 | CG2 | VAL | A | 115 | 53.790 | 31.889 | 23.058 | 1.00 25.45 | C |
| ATOM | 915 | N | SER | A | 116 | 50.473 | 28.593 | 22.772 | 1.00 21.99 | N |
| ATOM | 916 | CA | SER | A | 116 | 49.899 | 27.275 | 22.637 | 1.00 23.08 | C |
| ATOM | 917 | C | SER | A | 116 | 49.808 | 26.898 | 21.171 | 1.00 22.64 | C |
| ATOM | 918 | O | SER | A | 116 | 49.386 | 27.688 | 20.364 | 1.00 24.53 | O |
| ATOM | 919 | CB | SER | A | 116 | 48.493 | 27.275 | 23.217 | 1.00 22.76 | C |
| ATOM | 920 | OG | SER | A | 116 | 48.531 | 27.499 | 24.604 | 1.00 24.98 | O |
| ATOM | 921 | N | ILE | A | 117 | 50.203 | 25.686 | 20.828 | 1.00 22.25 | N |
| ATOM | 922 | CA | ILE | A | 117 | 50.113 | 25.238 | 19.458 | 1.00 22.66 | C |
| ATOM | 923 | C | ILE | A | 117 | 49.233 | 24.013 | 19.381 | 1.00 23.09 | C |
| ATOM | 924 | O | ILE | A | 117 | 49.170 | 23.251 | 20.340 | 1.00 20.88 | O |
| ATOM | 925 | CB | ILE | A | 117 | 51.488 | 24.953 | 18.853 | 1.00 23.22 | C |
| ATOM | 926 | CG1 | ILE | A | 117 | 51.365 | 24.737 | 17.365 | 1.00 22.41 | C |
| ATOM | 927 | CG2 | ILE | A | 117 | 52.143 | 23.736 | 19.523 | 1.00 24.83 | C |
| ATOM | 928 | CD1 | ILE | A | 117 | 52.638 | 24.956 | 16.625 | 1.00 24.56 | C |
| ATOM | 929 | N | PHE | A | 118 | 48.551 | 23.857 | 18.238 | 1.00 23.47 | N |
| ATOM | 930 | CA | PHE | A | 118 | 47.566 | 22.802 | 18.031 | 1.00 24.72 | C |
| ATOM | 931 | C | PHE | A | 118 | 47.656 | 22.213 | 16.648 | 1.00 25.26 | C |
| ATOM | 932 | O | PHE | A | 118 | 47.603 | 22.935 | 15.667 | 1.00 26.82 | O |
| ATOM | 933 | CB | PHE | A | 118 | 46.158 | 23.346 | 18.201 | 1.00 23.91 | C |
| ATOM | 934 | CG | PHE | A | 118 | 45.962 | 24.048 | 19.473 | 1.00 24.04 | C |
| ATOM | 935 | CD1 | PHE | A | 118 | 46.310 | 25.375 | 19.599 | 1.00 22.26 | C |
| ATOM | 936 | CD2 | PHE | A | 118 | 45.423 | 23.381 | 20.569 | 1.00 25.13 | C |
| ATOM | 937 | CE1 | PHE | A | 118 | 46.152 | 26.023 | 20.806 | 1.00 24.35 | C |
| ATOM | 938 | CE2 | PHE | A | 118 | 45.245 | 24.025 | 21.766 | 1.00 23.88 | C |
| ATOM | 939 | CZ | PHE | A | 118 | 45.622 | 25.341 | 21.895 | 1.00 23.28 | C |
| ATOM | 940 | N | PRO | A | 119 | 47.761 | 20.883 | 16.558 | 1.00 27.26 | N |
| ATOM | 941 | CA | PRO | A | 119 | 47.726 | 20.246 | 15.249 | 1.00 26.92 | C |
| ATOM | 942 | C | PRO | A | 119 | 46.315 | 20.222 | 14.669 | 1.00 26.54 | C |
| ATOM | 943 | O | PRO | A | 119 | 45.352 | 20.504 | 15.403 | 1.00 25.32 | O |
| ATOM | 944 | CB | PRO | A | 119 | 48.223 | 18.818 | 15.513 | 1.00 27.21 | C |
| ATOM | 945 | CG | PRO | A | 119 | 48.302 | 18.644 | 16.964 | 1.00 28.86 | C |
| ATOM | 946 | CD | PRO | A | 119 | 47.906 | 19.916 | 17.661 | 1.00 28.06 | C |
| ATOM | 947 | N | PRO | A | 120 | 46.186 | 19.889 | 13.357 | 1.00 25.41 | N |
| ATOM | 948 | CA | PRO | A | 120 | 44.901 | 19.711 | 12.714 | 1.00 24.16 | C |
| ATOM | 949 | C | PRO | A | 120 | 44.070 | 18.620 | 13.385 | 1.00 25.07 | C |
| ATOM | 950 | O | PRO | A | 120 | 44.601 | 17.600 | 13.770 | 1.00 23.52 | O |
| ATOM | 951 | CB | PRO | A | 120 | 45.268 | 19.278 | 11.306 | 1.00 24.81 | C |
| ATOM | 952 | CG | PRO | A | 120 | 46.687 | 19.779 | 11.103 | 1.00 25.37 | C |
| ATOM | 953 | CD | PRO | A | 120 | 47.305 | 19.697 | 12.422 | 1.00 24.92 | C |
| ATOM | 954 | N | SER | A | 121 | 42.774 | 18.853 | 13.481 | 1.00 26.36 | N |
| ATOM | 955 | CA | SER | A | 121 | 41.850 | 17.883 | 14.063 | 1.00 27.79 | C |
| ATOM | 956 | C | SER | A | 121 | 41.606 | 16.763 | 13.047 | 1.00 29.88 | C |
| ATOM | 957 | O | SER | A | 121 | 41.761 | 16.967 | 11.834 | 1.00 26.97 | O |
| ATOM | 958 | CB | SER | A | 121 | 40.528 | 18.587 | 14.410 | 1.00 28.45 | C |
| ATOM | 959 | OG | SER | A | 121 | 39.872 | 19.119 | 13.256 | 1.00 28.00 | O |
| ATOM | 960 | N | SER | A | 122 | 41.209 | 15.588 | 13.527 | 1.00 31.09 | N |
| ATOM | 961 | CA | SER | A | 122 | 40.792 | 14.523 | 12.626 | 1.00 32.75 | C |
| ATOM | 962 | C | SER | A | 122 | 39.618 | 14.981 | 11.746 | 1.00 32.30 | C |
| ATOM | 963 | O | SER | A | 122 | 39.505 | 14.590 | 10.578 | 1.00 31.87 | O |
| ATOM | 964 | CB | SER | A | 122 | 40.396 | 13.277 | 13.421 | 1.00 34.48 | C |
| ATOM | 965 | OG | SER | A | 122 | 39.437 | 13.605 | 14.415 | 1.00 37.90 | O |

```
ATOM    966  N    GLU A 123     38.755  15.814  12.313  1.00 31.87           N
ATOM    967  CA   GLU A 123     37.622  16.373  11.596  1.00 32.22           C
ATOM    968  C    GLU A 123     38.098  17.121  10.363  1.00 30.90           C
ATOM    969  O    GLU A 123     37.646  16.854   9.249  1.00 29.95           O
ATOM    970  CB   GLU A 123     36.847  17.374  12.472  1.00 34.37           C
ATOM    971  CG   GLU A 123     35.838  16.768  13.425  1.00 37.20           C
ATOM    972  CD   GLU A 123     36.405  16.450  14.795  1.00 39.64           C
ATOM    973  OE1  GLU A 123     37.646  16.241  14.931  1.00 38.86           O
ATOM    974  OE2  GLU A 123     35.580  16.408  15.744  1.00 41.36           O
ATOM    975  N    GLN A 124     38.983  18.088  10.571  1.00 29.33           N
ATOM    976  CA   GLN A 124     39.485  18.894   9.437  1.00 28.72           C
ATOM    977  C    GLN A 124     40.190  18.015   8.416  1.00 27.95           C
ATOM    978  O    GLN A 124     40.012  18.219   7.230  1.00 29.04           O
ATOM    979  CB   GLN A 124     40.430  20.010   9.902  1.00 28.34           C
ATOM    980  CG   GLN A 124     41.219  20.637   8.762  1.00 28.53           C
ATOM    981  CD   GLN A 124     41.943  21.895   9.163  1.00 27.42           C
ATOM    982  OE1  GLN A 124     42.683  21.902  10.131  1.00 26.94           O
ATOM    983  NE2  GLN A 124     41.725  22.966   8.422  1.00 28.91           N
ATOM    984  N    LEU A 125     40.972  17.050   8.892  1.00 28.12           N
ATOM    985  CA   LEU A 125     41.756  16.135   8.031  1.00 28.61           C
ATOM    986  C    LEU A 125     40.871  15.293   7.091  1.00 28.74           C
ATOM    987  O    LEU A 125     41.184  15.099   5.927  1.00 25.07           O
ATOM    988  CB   LEU A 125     42.668  15.244   8.877  1.00 28.91           C
ATOM    989  CG   LEU A 125     43.935  15.890   9.457  1.00 29.58           C
ATOM    990  CD1  LEU A 125     44.609  14.950  10.448  1.00 31.84           C
ATOM    991  CD2  LEU A 125     44.930  16.286   8.370  1.00 30.43           C
ATOM    992  N    THR A 126     39.736  14.842   7.610  1.00 29.60           N
ATOM    993  CA   THR A 126     38.676  14.244   6.809  1.00 31.00           C
ATOM    994  C    THR A 126     38.135  15.171   5.697  1.00 30.51           C
ATOM    995  O    THR A 126     37.772  14.699   4.630  1.00 31.31           O
ATOM    996  CB   THR A 126     37.497  13.804   7.749  1.00 32.01           C
ATOM    997  OG1  THR A 126     37.992  12.868   8.723  1.00 32.87           O
ATOM    998  CG2  THR A 126     36.359  13.169   6.953  1.00 33.72           C
ATOM    999  N    SER A 127     38.072  16.475   5.938  1.00 30.60           N
ATOM   1000  CA   SER A 127     37.639  17.411   4.901  1.00 32.07           C
ATOM   1001  C    SER A 127     38.691  17.597   3.786  1.00 30.95           C
ATOM   1002  O    SER A 127     38.344  17.998   2.667  1.00 30.43           O
ATOM   1003  CB   SER A 127     37.296  18.780   5.514  1.00 33.91           C
ATOM   1004  OG   SER A 127     36.496  18.646   6.697  1.00 38.79           O
ATOM   1005  N    GLY A 128     39.960  17.325   4.110  1.00 29.83           N
ATOM   1006  CA   GLY A 128     41.072  17.380   3.138  1.00 29.75           C
ATOM   1007  C    GLY A 128     42.074  18.481   3.394  1.00 29.27           C
ATOM   1008  O    GLY A 128     43.077  18.604   2.689  1.00 31.14           O
ATOM   1009  N    GLY A 129     41.790  19.310   4.389  1.00 28.71           N
ATOM   1010  CA   GLY A 129     42.664  20.414   4.775  1.00 27.81           C
ATOM   1011  C    GLY A 129     43.342  20.106   6.084  1.00 27.80           C
ATOM   1012  O    GLY A 129     43.008  19.099   6.742  1.00 28.42           O
ATOM   1013  N    ALA A 130     44.303  20.970   6.440  1.00 26.43           N
ATOM   1014  CA   ALA A 130     45.173  20.810   7.608  1.00 26.96           C
ATOM   1015  C    ALA A 130     45.691  22.179   8.122  1.00 28.06           C
ATOM   1016  O    ALA A 130     46.565  22.801   7.496  1.00 29.70           O
ATOM   1017  CB   ALA A 130     46.348  19.905   7.261  1.00 26.42           C
ATOM   1018  N    SER A 131     45.144  22.664   9.238  1.00 25.83           N
ATOM   1019  CA   SER A 131     45.548  23.963   9.783  1.00 24.77           C
ATOM   1020  C    SER A 131     46.322  23.805  11.081  1.00 24.03           C
ATOM   1021  O    SER A 131     45.922  23.062  11.960  1.00 25.22           O
ATOM   1022  CB   SER A 131     44.317  24.854  10.014  1.00 23.50           C
ATOM   1023  OG   SER A 131     43.626  25.112   8.816  1.00 23.69           O
ATOM   1024  N    VAL A 132     47.442  24.487  11.228  1.00 23.33           N
ATOM   1025  CA   VAL A 132     48.119  24.464  12.511  1.00 23.77           C
ATOM   1026  C    VAL A 132     47.795  25.813  13.135  1.00 23.40           C
ATOM   1027  O    VAL A 132     47.841  26.828  12.446  1.00 24.06           O
ATOM   1028  CB   VAL A 132     49.641  24.313  12.372  1.00 23.78           C
ATOM   1029  CG1  VAL A 132     50.270  24.233  13.734  1.00 23.78           C
ATOM   1030  CG2  VAL A 132     49.993  23.080  11.549  1.00 26.10           C
```

```
ATOM   1031  N    VAL A 133    47.448  25.826  14.418  1.00 22.94    N
ATOM   1032  CA   VAL A 133    47.046  27.065  15.075  1.00 22.46    C
ATOM   1033  C    VAL A 133    47.967  27.407  16.239  1.00 22.84    C
ATOM   1034  O    VAL A 133    48.373  26.557  17.000  1.00 21.12    O
ATOM   1035  CB   VAL A 133    45.554  27.005  15.530  1.00 23.42    C
ATOM   1036  CG1  VAL A 133    45.106  28.337  16.121  1.00 23.11    C
ATOM   1037  CG2  VAL A 133    44.685  26.662  14.370  1.00 22.85    C
ATOM   1038  N    CYS A 134    48.274  28.685  16.381  1.00 24.53    N
ATOM   1039  CA   CYS A 134    49.064  29.165  17.506  1.00 25.52    C
ATOM   1040  C    CYS A 134    48.324  30.353  18.122  1.00 24.54    C
ATOM   1041  O    CYS A 134    47.988  31.314  17.430  1.00 22.50    O
ATOM   1042  CB   CYS A 134    50.451  29.564  17.009  1.00 27.58    C
ATOM   1043  SG   CYS A 134    51.676  29.929  18.247  1.00 32.47    S
ATOM   1044  N    PHE A 135    47.983  30.223  19.397  1.00 24.78    N
ATOM   1045  CA   PHE A 135    47.489  31.318  20.208  1.00 24.48    C
ATOM   1046  C    PHE A 135    48.656  31.874  21.015  1.00 24.46    C
ATOM   1047  O    PHE A 135    49.438  31.133  21.592  1.00 24.98    O
ATOM   1048  CB   PHE A 135    46.404  30.828  21.172  1.00 24.52    C
ATOM   1049  CG   PHE A 135    45.189  30.294  20.485  1.00 24.84    C
ATOM   1050  CD1  PHE A 135    44.518  31.050  19.539  1.00 24.11    C
ATOM   1051  CD2  PHE A 135    44.676  29.078  20.819  1.00 26.32    C
ATOM   1052  CE1  PHE A 135    43.418  30.574  18.907  1.00 23.49    C
ATOM   1053  CE2  PHE A 135    43.554  28.595  20.188  1.00 24.15    C
ATOM   1054  CZ   PHE A 135    42.921  29.341  19.240  1.00 24.02    C
ATOM   1055  N    LEU A 136    48.779  33.188  21.047  1.00 23.90    N
ATOM   1056  CA   LEU A 136    49.751  33.860  21.883  1.00 24.38    C
ATOM   1057  C    LEU A 136    48.902  34.749  22.738  1.00 23.10    C
ATOM   1058  O    LEU A 136    48.382  35.735  22.254  1.00 20.04    O
ATOM   1059  CB   LEU A 136    50.700  34.710  21.029  1.00 25.26    C
ATOM   1060  CG   LEU A 136    51.639  33.969  20.092  1.00 27.65    C
ATOM   1061  CD1  LEU A 136    50.888  33.282  19.037  1.00 30.19    C
ATOM   1062  CD2  LEU A 136    52.585  34.970  19.462  1.00 28.21    C
ATOM   1063  N    ASN A 137    48.690  34.381  23.999  1.00 24.68    N
ATOM   1064  CA   ASN A 137    47.646  35.022  24.763  1.00 23.84    C
ATOM   1065  C    ASN A 137    48.119  35.829  25.955  1.00 23.66    C
ATOM   1066  O    ASN A 137    49.144  35.496  26.575  1.00 21.89    O
ATOM   1067  CB   ASN A 137    46.600  33.997  25.202  1.00 25.12    C
ATOM   1068  CG   ASN A 137    45.721  33.526  24.052  1.00 26.98    C
ATOM   1069  OD1  ASN A 137    45.710  34.113  22.959  1.00 27.67    O
ATOM   1070  ND2  ASN A 137    44.983  32.450  24.292  1.00 24.87    N
ATOM   1071  N    ASN A 138    47.336  36.890  26.225  1.00 22.84    N
ATOM   1072  CA   ASN A 138    47.417  37.768  27.406  1.00 23.43    C
ATOM   1073  C    ASN A 138    48.759  38.410  27.584  1.00 23.20    C
ATOM   1074  O    ASN A 138    49.340  38.346  28.652  1.00 22.62    O
ATOM   1075  CB   ASN A 138    47.022  37.064  28.709  1.00 25.22    C
ATOM   1076  CG   ASN A 138    45.580  36.618  28.715  1.00 26.29    C
ATOM   1077  OD1  ASN A 138    45.255  35.594  28.162  1.00 31.72    O
ATOM   1078  ND2  ASN A 138    44.723  37.380  29.344  1.00 30.32    N
ATOM   1079  N    PHE A 139    49.251  39.028  26.522  1.00 23.61    N
ATOM   1080  CA   PHE A 139    50.524  39.720  26.587  1.00 23.93    C
ATOM   1081  C    PHE A 139    50.373  41.251  26.605  1.00 25.06    C
ATOM   1082  O    PHE A 139    49.342  41.798  26.222  1.00 22.13    O
ATOM   1083  CB   PHE A 139    51.429  39.263  25.437  1.00 24.13    C
ATOM   1084  CG   PHE A 139    50.849  39.477  24.057  1.00 24.00    C
ATOM   1085  CD1  PHE A 139    51.072  40.672  23.371  1.00 24.87    C
ATOM   1086  CD2  PHE A 139    50.125  38.487  23.428  1.00 22.57    C
ATOM   1087  CE1  PHE A 139    50.561  40.869  22.097  1.00 24.12    C
ATOM   1088  CE2  PHE A 139    49.601  38.681  22.178  1.00 23.79    C
ATOM   1089  CZ   PHE A 139    49.831  39.866  21.488  1.00 25.34    C
ATOM   1090  N    TYR A 140    51.417  41.911  27.118  1.00 28.59    N
ATOM   1091  CA   TYR A 140    51.546  43.362  27.116  1.00 29.69    C
ATOM   1092  C    TYR A 140    53.030  43.734  27.055  1.00 30.62    C
ATOM   1093  O    TYR A 140    53.838  43.153  27.787  1.00 31.13    O
ATOM   1094  CB   TYR A 140    50.924  43.998  28.369  1.00 30.70    C
ATOM   1095  CG   TYR A 140    50.936  45.508  28.251  1.00 30.72    C
```

```
ATOM   1096  CD1 TYR A 140   49.915  46.188  27.592  1.00 30.47           C
ATOM   1097  CD2 TYR A 140   52.020  46.242  28.715  1.00 31.35           C
ATOM   1098  CE1 TYR A 140   49.958  47.595  27.431  1.00 31.02           C
ATOM   1099  CE2 TYR A 140   52.072  47.634  28.570  1.00 31.50           C
ATOM   1100  CZ  TYR A 140   51.052  48.297  27.924  1.00 31.12           C
ATOM   1101  OH  TYR A 140   51.151  49.657  27.785  1.00 31.01           O
ATOM   1102  N   PRO A 141   53.401  44.730  26.226  1.00 32.23           N
ATOM   1103  CA  PRO A 141   52.661  45.548  25.253  1.00 32.24           C
ATOM   1104  C   PRO A 141   52.134  44.814  24.018  1.00 31.79           C
ATOM   1105  O   PRO A 141   52.413  43.634  23.834  1.00 30.77           O
ATOM   1106  CB  PRO A 141   53.699  46.619  24.835  1.00 32.62           C
ATOM   1107  CG  PRO A 141   54.687  46.629  25.903  1.00 33.63           C
ATOM   1108  CD  PRO A 141   54.795  45.201  26.335  1.00 32.67           C
ATOM   1109  N   LYS A 142   51.410  45.545  23.175  1.00 31.81           N
ATOM   1110  CA  LYS A 142   50.610  44.976  22.085  1.00 33.34           C
ATOM   1111  C   LYS A 142   51.381  44.430  20.896  1.00 33.09           C
ATOM   1112  O   LYS A 142   50.851  43.614  20.149  1.00 31.52           O
ATOM   1113  CB  LYS A 142   49.621  46.007  21.561  1.00 34.07           C
ATOM   1114  CG  LYS A 142   50.255  47.080  20.651  1.00 36.24           C
ATOM   1115  CD  LYS A 142   49.294  48.234  20.358  1.00 36.15           C
ATOM   1116  CE  LYS A 142   48.515  47.997  19.073  1.00 39.14           C
ATOM   1117  NZ  LYS A 142   47.337  48.928  18.923  1.00 40.56           N
ATOM   1118  N   ASP A 143   52.608  44.910  20.696  1.00 33.80           N
ATOM   1119  CA  ASP A 143   53.428  44.503  19.539  1.00 34.37           C
ATOM   1120  C   ASP A 143   54.080  43.115  19.691  1.00 33.36           C
ATOM   1121  O   ASP A 143   54.712  42.795  20.704  1.00 31.64           O
ATOM   1122  CB  ASP A 143   54.525  45.526  19.303  1.00 35.76           C
ATOM   1123  CG  ASP A 143   53.973  46.921  19.134  1.00 40.33           C
ATOM   1124  OD1 ASP A 143   53.339  47.146  18.075  1.00 43.09           O
ATOM   1125  OD2 ASP A 143   54.140  47.767  20.067  1.00 43.51           O
ATOM   1126  N   ILE A 144   53.968  42.312  18.652  1.00 33.10           N
ATOM   1127  CA  ILE A 144   54.405  40.940  18.734  1.00 34.31           C
ATOM   1128  C   ILE A 144   54.622  40.447  17.316  1.00 34.96           C
ATOM   1129  O   ILE A 144   53.956  40.899  16.391  1.00 35.65           O
ATOM   1130  CB  ILE A 144   53.341  40.097  19.543  1.00 34.12           C
ATOM   1131  CG1 ILE A 144   53.849  38.684  19.854  1.00 35.62           C
ATOM   1132  CG2 ILE A 144   52.011  40.081  18.823  1.00 33.61           C
ATOM   1133  CD1 ILE A 144   53.689  38.308  21.322  1.00 36.41           C
ATOM   1134  N   ASN A 145   55.582  39.550  17.150  1.00 36.39           N
ATOM   1135  CA  ASN A 145   55.953  39.014  15.855  1.00 37.58           C
ATOM   1136  C   ASN A 145   55.897  37.503  15.956  1.00 36.95           C
ATOM   1137  O   ASN A 145   56.382  36.939  16.921  1.00 37.35           O
ATOM   1138  CB  ASN A 145   57.382  39.457  15.480  1.00 38.85           C
ATOM   1139  CG  ASN A 145   57.592  40.970  15.638  1.00 41.19           C
ATOM   1140  OD1 ASN A 145   57.126  41.768  14.815  1.00 43.75           O
ATOM   1141  ND2 ASN A 145   58.274  41.368  16.715  1.00 43.20           N
ATOM   1142  N   VAL A 146   55.301  36.857  14.969  1.00 36.57           N
ATOM   1143  CA  VAL A 146   55.204  35.408  14.960  1.00 36.71           C
ATOM   1144  C   VAL A 146   55.805  34.871  13.682  1.00 36.50           C
ATOM   1145  O   VAL A 146   55.512  35.375  12.598  1.00 36.41           O
ATOM   1146  CB  VAL A 146   53.753  34.972  15.101  1.00 36.92           C
ATOM   1147  CG1 VAL A 146   53.622  33.429  15.124  1.00 37.44           C
ATOM   1148  CG2 VAL A 146   53.201  35.587  16.366  1.00 37.29           C
ATOM   1149  N   LYS A 147   56.655  33.858  13.825  1.00 35.76           N
ATOM   1150  CA  LYS A 147   57.238  33.177  12.698  1.00 36.80           C
ATOM   1151  C   LYS A 147   56.894  31.692  12.763  1.00 36.08           C
ATOM   1152  O   LYS A 147   56.923  31.097  13.829  1.00 35.87           O
ATOM   1153  CB  LYS A 147   58.751  33.345  12.726  1.00 37.13           C
ATOM   1154  CG  LYS A 147   59.475  32.660  11.583  1.00 38.00           C
ATOM   1155  CD  LYS A 147   60.973  32.713  11.797  1.00 39.02           C
ATOM   1156  CE  LYS A 147   61.566  34.047  11.326  1.00 41.05           C
ATOM   1157  NZ  LYS A 147   62.956  33.857  10.807  1.00 42.11           N
ATOM   1158  N   TRP A 148   56.592  31.120  11.602  1.00 36.07           N
ATOM   1159  CA  TRP A 148   56.302  29.694  11.455  1.00 35.88           C
ATOM   1160  C   TRP A 148   57.458  29.032  10.750  1.00 36.92           C
```

| | | | | | | | | | | | |
|---|---|---|---|---|---|---|---|---|---|---|---|
| ATOM | 1161 | O | TRP | A | 148 | 57.949 | 29.572 | 9.775 | 1.00 | 37.16 | O |
| ATOM | 1162 | CB | TRP | A | 148 | 55.067 | 29.475 | 10.587 | 1.00 | 33.90 | C |
| ATOM | 1163 | CG | TRP | A | 148 | 53.788 | 29.743 | 11.278 | 1.00 | 33.32 | C |
| ATOM | 1164 | CD1 | TRP | A | 148 | 53.069 | 30.885 | 11.232 | 1.00 | 32.17 | C |
| ATOM | 1165 | CD2 | TRP | A | 148 | 53.063 | 28.837 | 12.122 | 1.00 | 31.90 | C |
| ATOM | 1166 | NE1 | TRP | A | 148 | 51.938 | 30.760 | 11.995 | 1.00 | 32.28 | N |
| ATOM | 1167 | CE2 | TRP | A | 148 | 51.914 | 29.514 | 12.559 | 1.00 | 31.89 | C |
| ATOM | 1168 | CE3 | TRP | A | 148 | 53.283 | 27.527 | 12.557 | 1.00 | 30.93 | C |
| ATOM | 1169 | CZ2 | TRP | A | 148 | 50.973 | 28.925 | 13.398 | 1.00 | 31.42 | C |
| ATOM | 1170 | CZ3 | TRP | A | 148 | 52.341 | 26.939 | 13.389 | 1.00 | 31.29 | C |
| ATOM | 1171 | CH2 | TRP | A | 148 | 51.210 | 27.637 | 13.799 | 1.00 | 31.72 | C |
| ATOM | 1172 | N | LYS | A | 149 | 57.859 | 27.858 | 11.245 | 1.00 | 37.60 | N |
| ATOM | 1173 | CA | LYS | A | 149 | 58.748 | 26.950 | 10.533 | 1.00 | 38.03 | C |
| ATOM | 1174 | C | LYS | A | 149 | 58.014 | 25.608 | 10.307 | 1.00 | 37.89 | C |
| ATOM | 1175 | O | LYS | A | 149 | 57.198 | 25.197 | 11.144 | 1.00 | 34.10 | O |
| ATOM | 1176 | CB | LYS | A | 149 | 60.010 | 26.724 | 11.362 | 1.00 | 38.71 | C |
| ATOM | 1177 | CG | LYS | A | 149 | 60.825 | 27.991 | 11.634 | 1.00 | 39.85 | C |
| ATOM | 1178 | CD | LYS | A | 149 | 61.633 | 27.872 | 12.921 | 1.00 | 40.07 | C |
| ATOM | 1179 | CE | LYS | A | 149 | 62.634 | 29.025 | 13.069 | 1.00 | 41.36 | C |
| ATOM | 1180 | NZ | LYS | A | 149 | 63.485 | 28.862 | 14.303 | 1.00 | 42.00 | N |
| ATOM | 1181 | N | ILE | A | 150 | 58.272 | 24.971 | 9.161 | 1.00 | 37.68 | N |
| ATOM | 1182 | CA | ILE | A | 150 | 57.910 | 23.576 | 8.925 | 1.00 | 38.31 | C |
| ATOM | 1183 | C | ILE | A | 150 | 59.226 | 22.847 | 8.685 | 1.00 | 40.36 | C |
| ATOM | 1184 | O | ILE | A | 150 | 59.964 | 23.203 | 7.781 | 1.00 | 40.01 | O |
| ATOM | 1185 | CB | ILE | A | 150 | 57.008 | 23.402 | 7.692 | 1.00 | 38.70 | C |
| ATOM | 1186 | CG1 | ILE | A | 150 | 55.712 | 24.212 | 7.831 | 1.00 | 38.88 | C |
| ATOM | 1187 | CG2 | ILE | A | 150 | 56.684 | 21.901 | 7.456 | 1.00 | 39.00 | C |
| ATOM | 1188 | CD1 | ILE | A | 150 | 54.845 | 24.225 | 6.580 | 1.00 | 38.75 | C |
| ATOM | 1189 | N | ASP | A | 151 | 59.532 | 21.856 | 9.512 | 1.00 | 42.19 | N |
| ATOM | 1190 | CA | ASP | A | 151 | 60.803 | 21.145 | 9.437 | 1.00 | 44.76 | C |
| ATOM | 1191 | C | ASP | A | 151 | 62.001 | 22.119 | 9.472 | 1.00 | 46.16 | C |
| ATOM | 1192 | O | ASP | A | 151 | 63.014 | 21.887 | 8.822 | 1.00 | 46.75 | O |
| ATOM | 1193 | CB | ASP | A | 151 | 60.843 | 20.232 | 8.189 | 1.00 | 43.81 | C |
| ATOM | 1194 | CG | ASP | A | 151 | 59.876 | 19.035 | 8.289 | 1.00 | 43.10 | C |
| ATOM | 1195 | OD1 | ASP | A | 151 | 59.553 | 18.578 | 9.399 | 1.00 | 40.30 | O |
| ATOM | 1196 | OD2 | ASP | A | 151 | 59.440 | 18.538 | 7.237 | 1.00 | 44.15 | O |
| ATOM | 1197 | N | GLY | A | 152 | 61.881 | 23.208 | 10.237 | 1.00 | 47.95 | N |
| ATOM | 1198 | CA | GLY | A | 152 | 62.995 | 24.153 | 10.434 | 1.00 | 48.41 | C |
| ATOM | 1199 | C | GLY | A | 152 | 63.113 | 25.277 | 9.416 | 1.00 | 50.07 | C |
| ATOM | 1200 | O | GLY | A | 152 | 63.802 | 26.264 | 9.670 | 1.00 | 51.02 | O |
| ATOM | 1201 | N | SER | A | 153 | 62.447 | 25.121 | 8.274 | 1.00 | 51.42 | N |
| ATOM | 1202 | CA | SER | A | 153 | 62.353 | 26.152 | 7.243 | 1.00 | 53.12 | C |
| ATOM | 1203 | C | SER | A | 153 | 61.167 | 27.093 | 7.465 | 1.00 | 53.80 | C |
| ATOM | 1204 | O | SER | A | 153 | 60.045 | 26.636 | 7.642 | 1.00 | 53.11 | O |
| ATOM | 1205 | CB | SER | A | 153 | 62.185 | 25.501 | 5.871 | 1.00 | 53.26 | C |
| ATOM | 1206 | OG | SER | A | 153 | 63.401 | 24.913 | 5.435 | 1.00 | 55.74 | O |
| ATOM | 1207 | N | GLU | A | 154 | 61.417 | 28.402 | 7.404 | 1.00 | 55.27 | N |
| ATOM | 1208 | CA | GLU | A | 154 | 60.357 | 29.414 | 7.553 | 1.00 | 55.90 | C |
| ATOM | 1209 | C | GLU | A | 154 | 59.285 | 29.297 | 6.454 | 1.00 | 55.31 | C |
| ATOM | 1210 | O | GLU | A | 154 | 59.612 | 29.115 | 5.288 | 1.00 | 55.51 | O |
| ATOM | 1211 | CB | GLU | A | 154 | 60.972 | 30.821 | 7.548 | 1.00 | 56.40 | C |
| ATOM | 1212 | CG | GLU | A | 154 | 59.992 | 31.957 | 7.891 | 1.00 | 57.03 | C |
| ATOM | 1213 | CD | GLU | A | 154 | 60.619 | 33.350 | 7.723 | 1.00 | 58.07 | C |
| ATOM | 1214 | OE1 | GLU | A | 154 | 61.776 | 33.559 | 8.169 | 1.00 | 60.03 | O |
| ATOM | 1215 | OE2 | GLU | A | 154 | 59.952 | 34.233 | 7.138 | 1.00 | 59.64 | O |
| ATOM | 1216 | N | ARG | A | 155 | 58.012 | 29.386 | 6.839 | 1.00 | 54.97 | N |
| ATOM | 1217 | CA | ARG | A | 155 | 56.888 | 29.316 | 5.894 | 1.00 | 54.44 | C |
| ATOM | 1218 | C | ARG | A | 155 | 56.062 | 30.594 | 6.014 | 1.00 | 52.97 | C |
| ATOM | 1219 | O | ARG | A | 155 | 55.465 | 30.844 | 7.059 | 1.00 | 52.57 | O |
| ATOM | 1220 | CB | ARG | A | 155 | 56.016 | 28.088 | 6.196 | 1.00 | 55.15 | C |
| ATOM | 1221 | CG | ARG | A | 155 | 54.756 | 27.907 | 5.326 | 1.00 | 55.45 | C |
| ATOM | 1222 | CD | ARG | A | 155 | 55.053 | 27.416 | 3.907 | 1.00 | 56.82 | C |
| ATOM | 1223 | NE | ARG | A | 155 | 53.847 | 26.881 | 3.253 | 1.00 | 57.28 | N |
| ATOM | 1224 | CZ | ARG | A | 155 | 53.663 | 25.608 | 2.871 | 1.00 | 58.22 | C |
| ATOM | 1225 | NH1 | ARG | A | 155 | 54.606 | 24.682 | 3.021 | 1.00 | 58.82 | N |

```
ATOM    1226  NH2 ARG A 155      52.523  25.250   2.299  1.00 58.18           N
ATOM    1227  N   GLN A 156      56.043  31.401   4.952  1.00 51.37           N
ATOM    1228  CA  GLN A 156      55.323  32.680   4.945  1.00 50.06           C
ATOM    1229  C   GLN A 156      53.924  32.539   4.382  1.00 47.81           C
ATOM    1230  O   GLN A 156      52.983  33.128   4.891  1.00 48.40           O
ATOM    1231  CB  GLN A 156      56.085  33.720   4.131  1.00 51.02           C
ATOM    1232  CG  GLN A 156      57.539  33.928   4.584  1.00 52.22           C
ATOM    1233  CD  GLN A 156      57.936  35.396   4.662  1.00 53.09           C
ATOM    1234  OE1 GLN A 156      57.155  36.283   4.309  1.00 54.78           O
ATOM    1235  NE2 GLN A 156      59.151  35.658   5.137  1.00 52.50           N
ATOM    1236  N   ASN A 157      53.788  31.737   3.340  1.00 46.22           N
ATOM    1237  CA  ASN A 157      52.510  31.557   2.646  1.00 44.81           C
ATOM    1238  C   ASN A 157      51.532  30.664   3.431  1.00 43.55           C
ATOM    1239  O   ASN A 157      51.938  29.690   4.060  1.00 43.49           O
ATOM    1240  CB  ASN A 157      52.757  30.953   1.254  1.00 45.62           C
ATOM    1241  CG  ASN A 157      53.967  31.589   0.530  1.00 47.46           C
ATOM    1242  OD1 ASN A 157      53.996  32.803   0.280  1.00 46.95           O
ATOM    1243  ND2 ASN A 157      54.963  30.758   0.192  1.00 48.22           N
ATOM    1244  N   GLY A 158      50.247  30.992   3.374  1.00 42.16           N
ATOM    1245  CA  GLY A 158      49.190  30.184   4.023  1.00 41.04           C
ATOM    1246  C   GLY A 158      48.954  30.531   5.488  1.00 38.33           C
ATOM    1247  O   GLY A 158      48.256  29.811   6.191  1.00 36.52           O
ATOM    1248  N   VAL A 159      49.519  31.658   5.918  1.00 36.37           N
ATOM    1249  CA  VAL A 159      49.424  32.145   7.294  1.00 36.36           C
ATOM    1250  C   VAL A 159      48.304  33.209   7.476  1.00 36.60           C
ATOM    1251  O   VAL A 159      48.174  34.177   6.693  1.00 34.85           O
ATOM    1252  CB  VAL A 159      50.789  32.690   7.747  1.00 36.21           C
ATOM    1253  CG1 VAL A 159      50.742  33.161   9.187  1.00 36.65           C
ATOM    1254  CG2 VAL A 159      51.847  31.620   7.572  1.00 35.20           C
ATOM    1255  N   LEU A 160      47.479  33.004   8.505  1.00 36.20           N
ATOM    1256  CA  LEU A 160      46.348  33.885   8.788  1.00 35.33           C
ATOM    1257  C   LEU A 160      46.414  34.376  10.246  1.00 33.82           C
ATOM    1258  O   LEU A 160      46.197  33.597  11.182  1.00 29.83           O
ATOM    1259  CB  LEU A 160      45.035  33.128   8.507  1.00 37.09           C
ATOM    1260  CG  LEU A 160      43.727  33.924   8.348  1.00 38.05           C
ATOM    1261  CD1 LEU A 160      42.784  33.250   7.334  1.00 39.95           C
ATOM    1262  CD2 LEU A 160      43.007  34.136   9.698  1.00 40.06           C
ATOM    1263  N   ASN A 161      46.731  35.667  10.416  1.00 32.56           N
ATOM    1264  CA  ASN A 161      46.851  36.289  11.728  1.00 32.32           C
ATOM    1265  C   ASN A 161      45.685  37.180  12.111  1.00 31.46           C
ATOM    1266  O   ASN A 161      45.110  37.887  11.269  1.00 30.91           O
ATOM    1267  CB  ASN A 161      48.092  37.140  11.796  1.00 32.24           C
ATOM    1268  CG  ASN A 161      49.311  36.368  11.491  1.00 34.87           C
ATOM    1269  OD1 ASN A 161      49.519  35.280  12.032  1.00 35.48           O
ATOM    1270  ND2 ASN A 161      50.135  36.900  10.603  1.00 35.76           N
ATOM    1271  N   SER A 162      45.370  37.154  13.399  1.00 29.50           N
ATOM    1272  CA  SER A 162      44.340  37.995  13.960  1.00 28.84           C
ATOM    1273  C   SER A 162      44.746  38.482  15.361  1.00 28.60           C
ATOM    1274  O   SER A 162      45.438  37.795  16.099  1.00 28.06           O
ATOM    1275  CB  SER A 162      43.039  37.205  13.992  1.00 29.01           C
ATOM    1276  OG  SER A 162      41.940  38.046  14.177  1.00 29.26           O
ATOM    1277  N   TRP A 163      44.306  39.684  15.714  1.00 28.66           N
ATOM    1278  CA  TRP A 163      44.646  40.323  16.977  1.00 28.35           C
ATOM    1279  C   TRP A 163      43.369  40.775  17.717  1.00 28.22           C
ATOM    1280  O   TRP A 163      42.429  41.276  17.079  1.00 26.74           O
ATOM    1281  CB  TRP A 163      45.504  41.545  16.678  1.00 29.17           C
ATOM    1282  CG  TRP A 163      46.809  41.235  15.996  1.00 29.27           C
ATOM    1283  CD1 TRP A 163      48.016  41.015  16.587  1.00 29.29           C
ATOM    1284  CD2 TRP A 163      47.022  41.113  14.592  1.00 30.23           C
ATOM    1285  NE1 TRP A 163      48.973  40.781  15.638  1.00 29.47           N
ATOM    1286  CE2 TRP A 163      48.386  40.833  14.400  1.00 30.32           C
ATOM    1287  CE3 TRP A 163      46.195  41.240  13.473  1.00 30.68           C
ATOM    1288  CZ2 TRP A 163      48.934  40.648  13.137  1.00 29.97           C
ATOM    1289  CZ3 TRP A 163      46.746  41.068  12.224  1.00 29.95           C
ATOM    1290  CH2 TRP A 163      48.095  40.769  12.067  1.00 30.10           C
```

171

| | | | | | | | | | | |
|---|---|---|---|---|---|---|---|---|---|---|
| ATOM | 1291 | N | THR | A | 164 | 43.302 | 40.581 | 19.037 | 1.00 28.49 | N |
| ATOM | 1292 | CA | THR | A | 164 | 42.200 | 41.170 | 19.821 | 1.00 28.90 | C |
| ATOM | 1293 | C | THR | A | 164 | 42.510 | 42.640 | 20.153 | 1.00 30.30 | C |
| ATOM | 1294 | O | THR | A | 164 | 43.661 | 43.083 | 20.063 | 1.00 28.63 | O |
| ATOM | 1295 | CB | THR | A | 164 | 41.931 | 40.436 | 21.150 | 1.00 29.36 | C |
| ATOM | 1296 | OG1 | THR | A | 164 | 43.124 | 40.381 | 21.926 | 1.00 29.53 | O |
| ATOM | 1297 | CG2 | THR | A | 164 | 41.419 | 39.002 | 20.911 | 1.00 29.68 | C |
| ATOM | 1298 | N | ASP | A | 165 | 41.476 | 43.376 | 20.544 | 1.00 31.12 | N |
| ATOM | 1299 | CA | ASP | A | 165 | 41.640 | 44.705 | 21.143 | 1.00 31.90 | C |
| ATOM | 1300 | C | ASP | A | 165 | 42.132 | 44.535 | 22.573 | 1.00 30.25 | C |
| ATOM | 1301 | O | ASP | A | 165 | 42.205 | 43.416 | 23.052 | 1.00 29.73 | O |
| ATOM | 1302 | CB | ASP | A | 165 | 40.310 | 45.460 | 21.168 | 1.00 33.72 | C |
| ATOM | 1303 | CG | ASP | A | 165 | 39.681 | 45.608 | 19.794 | 1.00 36.27 | C |
| ATOM | 1304 | OD1 | ASP | A | 165 | 40.389 | 46.060 | 18.843 | 1.00 36.81 | O |
| ATOM | 1305 | OD2 | ASP | A | 165 | 38.464 | 45.281 | 19.687 | 1.00 37.54 | O |
| ATOM | 1306 | N | GLN | A | 166 | 42.440 | 45.639 | 23.262 | 1.00 29.15 | N |
| ATOM | 1307 | CA | GLN | A | 166 | 42.859 | 45.593 | 24.674 | 1.00 29.46 | C |
| ATOM | 1308 | C | GLN | A | 166 | 41.733 | 45.072 | 25.575 | 1.00 30.20 | C |
| ATOM | 1309 | O | GLN | A | 166 | 40.596 | 45.536 | 25.521 | 1.00 29.79 | O |
| ATOM | 1310 | CB | GLN | A | 166 | 43.328 | 46.963 | 25.161 | 1.00 29.57 | C |
| ATOM | 1311 | CG | GLN | A | 166 | 44.079 | 46.944 | 26.483 | 1.00 28.56 | C |
| ATOM | 1312 | CD | GLN | A | 166 | 44.834 | 48.237 | 26.770 | 1.00 28.42 | C |
| ATOM | 1313 | OE1 | GLN | A | 166 | 44.463 | 49.330 | 26.314 | 1.00 25.57 | O |
| ATOM | 1314 | NE2 | GLN | A | 166 | 45.897 | 48.115 | 27.541 | 1.00 25.24 | N |
| ATOM | 1315 | N | ASP | A | 167 | 42.048 | 44.067 | 26.368 | 1.00 32.54 | N |
| ATOM | 1316 | CA | ASP | A | 167 | 41.037 | 43.412 | 27.182 | 1.00 33.53 | C |
| ATOM | 1317 | C | ASP | A | 167 | 40.577 | 44.362 | 28.267 | 1.00 34.02 | C |
| ATOM | 1318 | O | ASP | A | 167 | 41.396 | 45.009 | 28.921 | 1.00 33.27 | O |
| ATOM | 1319 | CB | ASP | A | 167 | 41.588 | 42.143 | 27.812 | 1.00 34.54 | C |
| ATOM | 1320 | CG | ASP | A | 167 | 40.556 | 41.433 | 28.677 | 1.00 37.34 | C |
| ATOM | 1321 | OD1 | ASP | A | 167 | 39.492 | 41.030 | 28.147 | 1.00 40.24 | O |
| ATOM | 1322 | OD2 | ASP | A | 167 | 40.794 | 41.307 | 29.898 | 1.00 40.07 | O |
| ATOM | 1323 | N | SER | A | 168 | 39.260 | 44.382 | 28.470 | 1.00 36.03 | N |
| ATOM | 1324 | CA | SER | A | 168 | 38.573 | 45.221 | 29.459 | 1.00 37.73 | C |
| ATOM | 1325 | C | SER | A | 168 | 38.828 | 44.842 | 30.896 | 1.00 37.98 | C |
| ATOM | 1326 | O | SER | A | 168 | 38.492 | 45.615 | 31.801 | 1.00 37.99 | O |
| ATOM | 1327 | CB | SER | A | 168 | 37.061 | 45.145 | 29.235 | 1.00 38.41 | C |
| ATOM | 1328 | OG | SER | A | 168 | 36.760 | 45.601 | 27.928 | 1.00 41.95 | O |
| ATOM | 1329 | N | LYS | A | 169 | 39.394 | 43.661 | 31.120 | 1.00 37.73 | N |
| ATOM | 1330 | CA | LYS | A | 169 | 39.579 | 43.158 | 32.479 | 1.00 38.38 | C |
| ATOM | 1331 | C | LYS | A | 169 | 41.035 | 43.221 | 32.948 | 1.00 36.50 | C |
| ATOM | 1332 | O | LYS | A | 169 | 41.327 | 43.738 | 34.032 | 1.00 37.97 | O |
| ATOM | 1333 | CB | LYS | A | 169 | 39.013 | 41.733 | 32.590 | 1.00 40.01 | C |
| ATOM | 1334 | CG | LYS | A | 169 | 37.461 | 41.667 | 32.579 | 1.00 41.43 | C |
| ATOM | 1335 | CD | LYS | A | 169 | 36.885 | 41.767 | 33.996 | 1.00 42.95 | C |
| ATOM | 1336 | CE | LYS | A | 169 | 35.424 | 41.271 | 34.076 | 1.00 42.98 | C |
| ATOM | 1337 | NZ | LYS | A | 169 | 34.876 | 41.379 | 35.474 | 1.00 42.86 | N |
| ATOM | 1338 | N | ASP | A | 170 | 41.952 | 42.715 | 32.143 | 1.00 34.36 | N |
| ATOM | 1339 | CA | ASP | A | 170 | 43.358 | 42.684 | 32.547 | 1.00 32.93 | C |
| ATOM | 1340 | C | ASP | A | 170 | 44.272 | 43.565 | 31.711 | 1.00 30.39 | C |
| ATOM | 1341 | O | ASP | A | 170 | 45.466 | 43.604 | 31.976 | 1.00 29.62 | O |
| ATOM | 1342 | CB | ASP | A | 170 | 43.896 | 41.237 | 32.593 | 1.00 33.41 | C |
| ATOM | 1343 | CG | ASP | A | 170 | 44.137 | 40.619 | 31.208 | 1.00 35.42 | C |
| ATOM | 1344 | OD1 | ASP | A | 170 | 43.876 | 41.244 | 30.145 | 1.00 35.07 | O |
| ATOM | 1345 | OD2 | ASP | A | 170 | 44.629 | 39.467 | 31.200 | 1.00 37.43 | O |
| ATOM | 1346 | N | SER | A | 171 | 43.711 | 44.235 | 30.710 | 1.00 28.94 | N |
| ATOM | 1347 | CA | SER | A | 171 | 44.420 | 45.235 | 29.903 | 1.00 28.89 | C |
| ATOM | 1348 | C | SER | A | 171 | 45.517 | 44.652 | 29.007 | 1.00 28.90 | C |
| ATOM | 1349 | O | SER | A | 171 | 46.436 | 45.364 | 28.559 | 1.00 29.40 | O |
| ATOM | 1350 | CB | SER | A | 171 | 44.956 | 46.366 | 30.810 | 1.00 29.56 | C |
| ATOM | 1351 | OG | SER | A | 171 | 43.884 | 47.026 | 31.472 | 1.00 27.56 | O |
| ATOM | 1352 | N | THR | A | 172 | 45.397 | 43.364 | 28.701 | 1.00 28.95 | N |
| ATOM | 1353 | CA | THR | A | 172 | 46.389 | 42.676 | 27.879 | 1.00 28.91 | C |
| ATOM | 1354 | C | THR | A | 172 | 45.848 | 42.468 | 26.469 | 1.00 28.11 | C |
| ATOM | 1355 | O | THR | A | 172 | 44.684 | 42.768 | 26.182 | 1.00 29.02 | O |

| | | | | | | | | | | |
|---|---|---|---|---|---|---|---|---|---|---|
| ATOM | 1356 | CB  | THR | A | 172 | 46.757 | 41.287 | 28.466 | 1.00 30.11 | C |
| ATOM | 1357 | OG1 | THR | A | 172 | 45.588 | 40.455 | 28.482 | 1.00 31.16 | O |
| ATOM | 1358 | CG2 | THR | A | 172 | 47.339 | 41.396 | 29.885 | 1.00 28.93 | C |
| ATOM | 1359 | N   | TYR | A | 173 | 46.713 | 41.932 | 25.608 | 1.00 26.31 | N |
| ATOM | 1360 | CA  | TYR | A | 173 | 46.424 | 41.659 | 24.225 | 1.00 24.03 | C |
| ATOM | 1361 | C   | TYR | A | 173 | 46.668 | 40.181 | 23.899 | 1.00 23.82 | C |
| ATOM | 1362 | O   | TYR | A | 173 | 47.398 | 39.470 | 24.600 | 1.00 23.11 | O |
| ATOM | 1363 | CB  | TYR | A | 173 | 47.348 | 42.505 | 23.352 | 1.00 22.99 | C |
| ATOM | 1364 | CG  | TYR | A | 173 | 47.144 | 44.005 | 23.498 | 1.00 24.09 | C |
| ATOM | 1365 | CD1 | TYR | A | 173 | 47.787 | 44.723 | 24.497 | 1.00 25.22 | C |
| ATOM | 1366 | CD2 | TYR | A | 173 | 46.291 | 44.696 | 22.654 | 1.00 23.77 | C |
| ATOM | 1367 | CE1 | TYR | A | 173 | 47.583 | 46.096 | 24.651 | 1.00 24.64 | C |
| ATOM | 1368 | CE2 | TYR | A | 173 | 46.087 | 46.086 | 22.796 | 1.00 23.29 | C |
| ATOM | 1369 | CZ  | TYR | A | 173 | 46.751 | 46.765 | 23.787 | 1.00 22.75 | C |
| ATOM | 1370 | OH  | TYR | A | 173 | 46.560 | 48.110 | 23.934 | 1.00 23.72 | O |
| ATOM | 1371 | N   | SER | A | 174 | 46.077 | 39.738 | 22.800 | 1.00 24.17 | N |
| ATOM | 1372 | CA  | SER | A | 174 | 46.185 | 38.366 | 22.373 | 1.00 24.73 | C |
| ATOM | 1373 | C   | SER | A | 174 | 46.220 | 38.329 | 20.856 | 1.00 24.87 | C |
| ATOM | 1374 | O   | SER | A | 174 | 45.716 | 39.243 | 20.184 | 1.00 23.66 | O |
| ATOM | 1375 | CB  | SER | A | 174 | 45.011 | 37.545 | 22.940 | 1.00 24.40 | C |
| ATOM | 1376 | OG  | SER | A | 174 | 45.053 | 37.475 | 24.371 | 1.00 24.62 | O |
| ATOM | 1377 | N   | MET | A | 175 | 46.826 | 37.262 | 20.328 | 1.00 26.41 | N |
| ATOM | 1378 | CA  | MET | A | 175 | 46.961 | 37.041 | 18.895 | 1.00 26.94 | C |
| ATOM | 1379 | C   | MET | A | 175 | 46.733 | 35.569 | 18.562 | 1.00 26.16 | C |
| ATOM | 1380 | O   | MET | A | 175 | 47.115 | 34.712 | 19.321 | 1.00 24.24 | O |
| ATOM | 1381 | CB  | MET | A | 175 | 48.367 | 37.419 | 18.422 | 1.00 27.54 | C |
| ATOM | 1382 | CG  | MET | A | 175 | 48.689 | 36.963 | 16.995 | 1.00 28.36 | C |
| ATOM | 1383 | SD  | MET | A | 175 | 50.302 | 37.563 | 16.480 | 1.00 30.20 | S |
| ATOM | 1384 | CE  | MET | A | 175 | 50.251 | 37.298 | 14.695 | 1.00 30.67 | C |
| ATOM | 1385 | N   | SER | A | 176 | 46.150 | 35.323 | 17.393 | 1.00 25.69 | N |
| ATOM | 1386 | CA  | SER | A | 176 | 45.948 | 34.000 | 16.820 | 1.00 26.63 | C |
| ATOM | 1387 | C   | SER | A | 176 | 46.703 | 33.979 | 15.496 | 1.00 27.37 | C |
| ATOM | 1388 | O   | SER | A | 176 | 46.645 | 34.946 | 14.741 | 1.00 27.54 | O |
| ATOM | 1389 | CB  | SER | A | 176 | 44.457 | 33.783 | 16.580 | 1.00 26.92 | C |
| ATOM | 1390 | OG  | SER | A | 176 | 44.159 | 32.516 | 16.037 | 1.00 28.80 | O |
| ATOM | 1391 | N   | SER | A | 177 | 47.449 | 32.911 | 15.243 | 1.00 27.53 | N |
| ATOM | 1392 | CA  | SER | A | 177 | 48.120 | 32.694 | 13.956 | 1.00 27.14 | C |
| ATOM | 1393 | C   | SER | A | 177 | 47.839 | 31.260 | 13.483 | 1.00 27.12 | C |
| ATOM | 1394 | O   | SER | A | 177 | 48.114 | 30.305 | 14.205 | 1.00 27.06 | O |
| ATOM | 1395 | CB  | SER | A | 177 | 49.612 | 32.945 | 14.116 | 1.00 27.33 | C |
| ATOM | 1396 | OG  | SER | A | 177 | 50.253 | 33.040 | 12.861 | 1.00 27.84 | O |
| ATOM | 1397 | N   | THR | A | 178 | 47.226 | 31.135 | 12.307 | 1.00 26.33 | N |
| ATOM | 1398 | CA  | THR | A | 178 | 46.872 | 29.868 | 11.692 | 1.00 27.54 | C |
| ATOM | 1399 | C   | THR | A | 178 | 47.640 | 29.680 | 10.373 | 1.00 29.34 | C |
| ATOM | 1400 | O   | THR | A | 178 | 47.599 | 30.530 | 9.478 | 1.00 29.65 | O |
| ATOM | 1401 | CB  | THR | A | 178 | 45.369 | 29.799 | 11.395 | 1.00 27.06 | C |
| ATOM | 1402 | OG1 | THR | A | 178 | 44.655 | 30.043 | 12.608 | 1.00 28.53 | O |
| ATOM | 1403 | CG2 | THR | A | 178 | 44.966 | 28.433 | 10.844 | 1.00 27.10 | C |
| ATOM | 1404 | N   | LEU | A | 179 | 48.347 | 28.556 | 10.289 | 1.00 30.28 | N |
| ATOM | 1405 | CA  | LEU | A | 179 | 49.055 | 28.134 | 9.087 | 1.00 30.53 | C |
| ATOM | 1406 | C   | LEU | A | 179 | 48.208 | 27.031 | 8.481 | 1.00 30.56 | C |
| ATOM | 1407 | O   | LEU | A | 179 | 47.987 | 26.012 | 9.124 | 1.00 29.98 | O |
| ATOM | 1408 | CB  | LEU | A | 179 | 50.437 | 27.628 | 9.484 | 1.00 31.23 | C |
| ATOM | 1409 | CG  | LEU | A | 179 | 51.225 | 26.762 | 8.503 | 1.00 32.45 | C |
| ATOM | 1410 | CD1 | LEU | A | 179 | 51.330 | 27.415 | 7.140 | 1.00 34.50 | C |
| ATOM | 1411 | CD2 | LEU | A | 179 | 52.610 | 26.506 | 9.086 | 1.00 32.84 | C |
| ATOM | 1412 | N   | THR | A | 180 | 47.699 | 27.242 | 7.269 | 1.00 31.13 | N |
| ATOM | 1413 | CA  | THR | A | 180 | 46.906 | 26.211 | 6.591 | 1.00 31.76 | C |
| ATOM | 1414 | C   | THR | A | 180 | 47.645 | 25.576 | 5.419 | 1.00 32.27 | C |
| ATOM | 1415 | O   | THR | A | 180 | 48.359 | 26.260 | 4.664 | 1.00 30.71 | O |
| ATOM | 1416 | CB  | THR | A | 180 | 45.532 | 26.744 | 6.184 | 1.00 32.79 | C |
| ATOM | 1417 | OG1 | THR | A | 180 | 44.870 | 27.233 | 7.363 | 1.00 32.98 | O |
| ATOM | 1418 | CG2 | THR | A | 180 | 44.661 | 25.628 | 5.509 | 1.00 31.92 | C |
| ATOM | 1419 | N   | LEU | A | 181 | 47.474 | 24.259 | 5.321 | 1.00 31.84 | N |
| ATOM | 1420 | CA  | LEU | A | 181 | 48.110 | 23.395 | 4.340 | 1.00 34.23 | C |

| ATOM | 1421 | C | LEU | A | 181 | 47.063 | 22.396 | 3.883 | 1.00 | 34.35 | C |
|---|---|---|---|---|---|---|---|---|---|---|---|
| ATOM | 1422 | O | LEU | A | 181 | 45.965 | 22.363 | 4.442 | 1.00 | 33.97 | O |
| ATOM | 1423 | CB | LEU | A | 181 | 49.256 | 22.610 | 4.998 | 1.00 | 36.30 | C |
| ATOM | 1424 | CG | LEU | A | 181 | 50.218 | 23.413 | 5.888 | 1.00 | 38.24 | C |
| ATOM | 1425 | CD1 | LEU | A | 181 | 50.878 | 22.553 | 6.955 | 1.00 | 39.30 | C |
| ATOM | 1426 | CD2 | LEU | A | 181 | 51.248 | 24.092 | 5.004 | 1.00 | 39.26 | C |
| ATOM | 1427 | N | THR | A | 182 | 47.389 | 21.575 | 2.888 | 1.00 | 34.12 | N |
| ATOM | 1428 | CA | THR | A | 182 | 46.518 | 20.449 | 2.532 | 1.00 | 33.68 | C |
| ATOM | 1429 | C | THR | A | 182 | 46.995 | 19.226 | 3.310 | 1.00 | 32.89 | C |
| ATOM | 1430 | O | THR | A | 182 | 48.165 | 19.165 | 3.730 | 1.00 | 32.12 | O |
| ATOM | 1431 | CB | THR | A | 182 | 46.555 | 20.121 | 1.017 | 1.00 | 34.39 | C |
| ATOM | 1432 | OG1 | THR | A | 182 | 47.816 | 19.506 | 0.673 | 1.00 | 35.60 | O |
| ATOM | 1433 | CG2 | THR | A | 182 | 46.335 | 21.399 | 0.184 | 1.00 | 34.69 | C |
| ATOM | 1434 | N | LYS | A | 183 | 46.108 | 18.240 | 3.459 | 1.00 | 30.91 | N |
| ATOM | 1435 | CA | LYS | A | 183 | 46.422 | 17.033 | 4.193 | 1.00 | 29.85 | C |
| ATOM | 1436 | C | LYS | A | 183 | 47.642 | 16.343 | 3.632 | 1.00 | 29.01 | C |
| ATOM | 1437 | O | LYS | A | 183 | 48.451 | 15.844 | 4.369 | 1.00 | 27.93 | O |
| ATOM | 1438 | CB | LYS | A | 183 | 45.254 | 16.054 | 4.159 | 1.00 | 29.95 | C |
| ATOM | 1439 | CG | LYS | A | 183 | 45.586 | 14.740 | 4.841 | 1.00 | 29.99 | C |
| ATOM | 1440 | CD | LYS | A | 183 | 44.414 | 13.807 | 4.982 | 1.00 | 29.81 | C |
| ATOM | 1441 | CE | LYS | A | 183 | 44.908 | 12.408 | 5.386 | 1.00 | 31.26 | C |
| ATOM | 1442 | NZ | LYS | A | 183 | 43.810 | 11.426 | 5.715 | 1.00 | 31.48 | N |
| ATOM | 1443 | N | ASP | A | 184 | 47.731 | 16.288 | 2.309 | 1.00 | 30.37 | N |
| ATOM | 1444 | CA | ASP | A | 184 | 48.874 | 15.724 | 1.599 | 1.00 | 31.97 | C |
| ATOM | 1445 | C | ASP | A | 184 | 50.227 | 16.387 | 1.990 | 1.00 | 32.57 | C |
| ATOM | 1446 | O | ASP | A | 184 | 51.191 | 15.668 | 2.297 | 1.00 | 32.36 | O |
| ATOM | 1447 | CB | ASP | A | 184 | 48.594 | 15.799 | 0.090 | 1.00 | 32.62 | C |
| ATOM | 1448 | CG | ASP | A | 184 | 49.829 | 15.611 | -0.752 | 1.00 | 35.12 | C |
| ATOM | 1449 | OD1 | ASP | A | 184 | 50.337 | 14.473 | -0.756 | 1.00 | 38.67 | O |
| ATOM | 1450 | OD2 | ASP | A | 184 | 50.265 | 16.578 | -1.439 | 1.00 | 36.74 | O |
| ATOM | 1451 | N | GLU | A | 185 | 50.310 | 17.719 | 1.989 | 1.00 | 32.65 | N |
| ATOM | 1452 | CA | GLU | A | 185 | 51.561 | 18.377 | 2.413 | 1.00 | 35.06 | C |
| ATOM | 1453 | C | GLU | A | 185 | 51.799 | 18.131 | 3.898 | 1.00 | 34.83 | C |
| ATOM | 1454 | O | GLU | A | 185 | 52.915 | 17.817 | 4.310 | 1.00 | 35.25 | O |
| ATOM | 1455 | CB | GLU | A | 185 | 51.544 | 19.884 | 2.174 | 1.00 | 35.74 | C |
| ATOM | 1456 | CG | GLU | A | 185 | 51.671 | 20.270 | 0.734 | 1.00 | 38.65 | C |
| ATOM | 1457 | CD | GLU | A | 185 | 51.040 | 21.634 | 0.383 | 1.00 | 39.41 | C |
| ATOM | 1458 | OE1 | GLU | A | 185 | 51.456 | 22.181 | -0.671 | 1.00 | 43.23 | O |
| ATOM | 1459 | OE2 | GLU | A | 185 | 50.130 | 22.142 | 1.114 | 1.00 | 41.11 | O |
| ATOM | 1460 | N | TYR | A | 186 | 50.745 | 18.254 | 4.704 | 1.00 | 34.03 | N |
| ATOM | 1461 | CA | TYR | A | 186 | 50.882 | 18.090 | 6.148 | 1.00 | 33.61 | C |
| ATOM | 1462 | C | TYR | A | 186 | 51.514 | 16.747 | 6.512 | 1.00 | 35.24 | C |
| ATOM | 1463 | O | TYR | A | 186 | 52.391 | 16.682 | 7.378 | 1.00 | 35.40 | O |
| ATOM | 1464 | CB | TYR | A | 186 | 49.523 | 18.223 | 6.840 | 1.00 | 32.43 | C |
| ATOM | 1465 | CG | TYR | A | 186 | 49.552 | 17.946 | 8.318 | 1.00 | 30.69 | C |
| ATOM | 1466 | CD1 | TYR | A | 186 | 50.233 | 18.800 | 9.186 | 1.00 | 31.33 | C |
| ATOM | 1467 | CD2 | TYR | A | 186 | 48.882 | 16.850 | 8.859 | 1.00 | 30.15 | C |
| ATOM | 1468 | CE1 | TYR | A | 186 | 50.279 | 18.565 | 10.547 | 1.00 | 30.85 | C |
| ATOM | 1469 | CE2 | TYR | A | 186 | 48.904 | 16.607 | 10.228 | 1.00 | 29.96 | C |
| ATOM | 1470 | CZ | TYR | A | 186 | 49.596 | 17.478 | 11.071 | 1.00 | 31.10 | C |
| ATOM | 1471 | OH | TYR | A | 186 | 49.642 | 17.259 | 12.435 | 1.00 | 31.40 | O |
| ATOM | 1472 | N | GLU | A | 187 | 51.074 | 15.678 | 5.847 | 1.00 | 36.99 | N |
| ATOM | 1473 | CA | GLU | A | 187 | 51.513 | 14.330 | 6.197 | 1.00 | 38.46 | C |
| ATOM | 1474 | C | GLU | A | 187 | 52.888 | 14.018 | 5.621 | 1.00 | 39.56 | C |
| ATOM | 1475 | O | GLU | A | 187 | 53.408 | 12.903 | 5.794 | 1.00 | 39.66 | O |
| ATOM | 1476 | CB | GLU | A | 187 | 50.491 | 13.295 | 5.749 | 1.00 | 39.37 | C |
| ATOM | 1477 | CG | GLU | A | 187 | 49.082 | 13.690 | 6.136 | 1.00 | 41.94 | C |
| ATOM | 1478 | CD | GLU | A | 187 | 48.299 | 12.599 | 6.804 | 1.00 | 44.73 | C |
| ATOM | 1479 | OE1 | GLU | A | 187 | 48.149 | 11.513 | 6.194 | 1.00 | 48.50 | O |
| ATOM | 1480 | OE2 | GLU | A | 187 | 47.803 | 12.848 | 7.925 | 1.00 | 45.05 | O |
| ATOM | 1481 | N | ARG | A | 188 | 53.479 | 15.006 | 4.956 | 1.00 | 39.85 | N |
| ATOM | 1482 | CA | ARG | A | 188 | 54.811 | 14.870 | 4.400 | 1.00 | 40.78 | C |
| ATOM | 1483 | C | ARG | A | 188 | 55.858 | 15.705 | 5.129 | 1.00 | 39.94 | C |
| ATOM | 1484 | O | ARG | A | 188 | 57.013 | 15.720 | 4.709 | 1.00 | 40.68 | O |
| ATOM | 1485 | CB | ARG | A | 188 | 54.776 | 15.213 | 2.913 | 1.00 | 41.21 | C |

```
ATOM   1486  CG   ARG A 188      54.176  14.095   2.070  1.00 42.71           C
ATOM   1487  CD   ARG A 188      54.210  14.425   0.577  1.00 43.52           C
ATOM   1488  NE   ARG A 188      54.098  13.220  -0.260  1.00 45.13           N
ATOM   1489  CZ   ARG A 188      52.980  12.521  -0.437  1.00 45.25           C
ATOM   1490  NH1  ARG A 188      51.863  12.868   0.182  1.00 47.17           N
ATOM   1491  NH2  ARG A 188      52.981  11.449  -1.211  1.00 45.81           N
ATOM   1492  N    HIS A 189      55.480  16.393   6.207  1.00 38.60           N
ATOM   1493  CA   HIS A 189      56.474  17.038   7.087  1.00 38.32           C
ATOM   1494  C    HIS A 189      56.284  16.525   8.503  1.00 37.47           C
ATOM   1495  O    HIS A 189      55.244  15.976   8.818  1.00 35.42           O
ATOM   1496  CB   HIS A 189      56.372  18.561   7.057  1.00 39.36           C
ATOM   1497  CG   HIS A 189      56.484  19.146   5.683  1.00 40.47           C
ATOM   1498  ND1  HIS A 189      55.393  19.308   4.855  1.00 40.33           N
ATOM   1499  CD2  HIS A 189      57.556  19.611   4.993  1.00 40.87           C
ATOM   1500  CE1  HIS A 189      55.795  19.826   3.706  1.00 42.05           C
ATOM   1501  NE2  HIS A 189      57.099  20.030   3.769  1.00 40.50           N
ATOM   1502  N    ASN A 190      57.301  16.708   9.340  1.00 37.06           N
ATOM   1503  CA   ASN A 190      57.340  16.107  10.675  1.00 37.69           C
ATOM   1504  C    ASN A 190      57.187  17.098  11.806  1.00 37.22           C
ATOM   1505  O    ASN A 190      56.524  16.780  12.785  1.00 37.30           O
ATOM   1506  CB   ASN A 190      58.647  15.337  10.876  1.00 37.95           C
ATOM   1507  CG   ASN A 190      58.805  14.197   9.889  1.00 38.52           C
ATOM   1508  OD1  ASN A 190      59.574  14.311   8.929  1.00 40.92           O
ATOM   1509  ND2  ASN A 190      58.070  13.097  10.104  1.00 36.56           N
ATOM   1510  N    SER A 191      57.816  18.270  11.680  1.00 35.94           N
ATOM   1511  CA   SER A 191      57.776  19.304  12.731  1.00 35.92           C
ATOM   1512  C    SER A 191      57.164  20.627  12.235  1.00 33.80           C
ATOM   1513  O    SER A 191      57.419  21.064  11.117  1.00 30.21           O
ATOM   1514  CB   SER A 191      59.169  19.574  13.334  1.00 37.34           C
ATOM   1515  OG   SER A 191      59.458  18.701  14.424  1.00 40.39           O
ATOM   1516  N    TYR A 192      56.330  21.215  13.086  1.00 34.02           N
ATOM   1517  CA   TYR A 192      55.677  22.519  12.841  1.00 34.97           C
ATOM   1518  C    TYR A 192      55.940  23.370  14.073  1.00 35.10           C
ATOM   1519  O    TYR A 192      55.697  22.915  15.199  1.00 33.73           O
ATOM   1520  CB   TYR A 192      54.167  22.356  12.616  1.00 35.73           C
ATOM   1521  CG   TYR A 192      53.862  21.478  11.423  1.00 36.59           C
ATOM   1522  CD1  TYR A 192      53.798  22.020  10.155  1.00 36.05           C
ATOM   1523  CD2  TYR A 192      53.705  20.099  11.560  1.00 36.61           C
ATOM   1524  CE1  TYR A 192      53.572  21.233   9.045  1.00 37.46           C
ATOM   1525  CE2  TYR A 192      53.462  19.290  10.442  1.00 37.36           C
ATOM   1526  CZ   TYR A 192      53.394  19.875   9.186  1.00 37.06           C
ATOM   1527  OH   TYR A 192      53.162  19.113   8.062  1.00 37.82           O
ATOM   1528  N    THR A 193      56.462  24.579  13.865  1.00 34.72           N
ATOM   1529  CA   THR A 193      56.871  25.440  14.976  1.00 35.74           C
ATOM   1530  C    THR A 193      56.316  26.851  14.829  1.00 35.42           C
ATOM   1531  O    THR A 193      56.360  27.435  13.749  1.00 33.61           O
ATOM   1532  CB   THR A 193      58.401  25.525  15.053  1.00 36.11           C
ATOM   1533  OG1  THR A 193      58.929  24.199  15.051  1.00 38.45           O
ATOM   1534  CG2  THR A 193      58.871  26.257  16.319  1.00 37.27           C
ATOM   1535  N    CYS A 194      55.800  27.374  15.934  1.00 35.84           N
ATOM   1536  CA   CYS A 194      55.353  28.752  16.040  1.00 36.52           C
ATOM   1537  C    CYS A 194      56.330  29.447  16.982  1.00 35.07           C
ATOM   1538  O    CYS A 194      56.449  29.023  18.114  1.00 33.08           O
ATOM   1539  CB   CYS A 194      53.928  28.764  16.631  1.00 36.53           C
ATOM   1540  SG   CYS A 194      53.276  30.383  17.056  1.00 40.91           S
ATOM   1541  N    GLU A 195      57.055  30.474  16.527  1.00 36.06           N
ATOM   1542  CA   GLU A 195      57.953  31.238  17.444  1.00 37.40           C
ATOM   1543  C    GLU A 195      57.575  32.735  17.568  1.00 36.58           C
ATOM   1544  O    GLU A 195      57.429  33.432  16.569  1.00 35.51           O
ATOM   1545  CB   GLU A 195      59.454  31.031  17.112  1.00 38.30           C
ATOM   1546  CG   GLU A 195      59.873  31.349  15.679  1.00 40.89           C
ATOM   1547  CD   GLU A 195      61.366  31.713  15.545  1.00 41.68           C
ATOM   1548  OE1  GLU A 195      62.212  30.798  15.630  1.00 45.94           O
ATOM   1549  OE2  GLU A 195      61.685  32.913  15.342  1.00 45.65           O
ATOM   1550  N    ALA A 196      57.386  33.178  18.814  1.00 36.33           N
```

```
ATOM   1551  CA   ALA A 196    56.880  34.501  19.164  1.00 37.12          C
ATOM   1552  C    ALA A 196    57.956  35.404  19.780  1.00 37.79          C
ATOM   1553  O    ALA A 196    58.447  35.133  20.886  1.00 35.84          O
ATOM   1554  CB   ALA A 196    55.742  34.362  20.171  1.00 37.39          C
ATOM   1555  N    THR A 197    58.295  36.493  19.090  1.00 38.63          N
ATOM   1556  CA   THR A 197    59.202  37.472  19.677  1.00 38.96          C
ATOM   1557  C    THR A 197    58.392  38.660  20.224  1.00 38.68          C
ATOM   1558  O    THR A 197    57.556  39.260  19.526  1.00 38.87          O
ATOM   1559  CB   THR A 197    60.338  37.933  18.715  1.00 40.27          C
ATOM   1560  OG1  THR A 197    59.982  39.180  18.110  1.00 42.81          O
ATOM   1561  CG2  THR A 197    60.652  36.883  17.646  1.00 39.82          C
ATOM   1562  N    HIS A 198    58.648  38.947  21.497  1.00 37.80          N
ATOM   1563  CA   HIS A 198    57.968  39.943  22.274  1.00 37.53          C
ATOM   1564  C    HIS A 198    59.056  40.573  23.134  1.00 38.82          C
ATOM   1565  O    HIS A 198    60.031  39.908  23.457  1.00 39.42          O
ATOM   1566  CB   HIS A 198    56.930  39.250  23.157  1.00 36.37          C
ATOM   1567  CG   HIS A 198    56.073  40.186  23.948  1.00 34.17          C
ATOM   1568  ND1  HIS A 198    56.169  40.300  25.320  1.00 32.28          N
ATOM   1569  CD2  HIS A 198    55.082  41.029  23.567  1.00 33.94          C
ATOM   1570  CE1  HIS A 198    55.277  41.177  25.749  1.00 31.78          C
ATOM   1571  NE2  HIS A 198    54.619  41.648  24.704  1.00 33.73          N
ATOM   1572  N    LYS A 199    58.891  41.834  23.517  1.00 40.56          N
ATOM   1573  CA   LYS A 199    59.928  42.565  24.265  1.00 42.08          C
ATOM   1574  C    LYS A 199    60.244  42.002  25.654  1.00 42.73          C
ATOM   1575  O    LYS A 199    61.239  42.398  26.251  1.00 42.26          O
ATOM   1576  CB   LYS A 199    59.564  44.065  24.393  1.00 42.68          C
ATOM   1577  CG   LYS A 199    58.377  44.372  25.299  1.00 42.44          C
ATOM   1578  CD   LYS A 199    58.745  44.657  26.753  1.00 42.37          C
ATOM   1579  CE   LYS A 199    58.885  46.145  27.009  1.00 43.56          C
ATOM   1580  NZ   LYS A 199    59.266  46.431  28.407  1.00 42.34          N
ATOM   1581  N    THR A 200    59.402  41.105  26.171  1.00 42.97          N
ATOM   1582  CA   THR A 200    59.598  40.516  27.502  1.00 44.59          C
ATOM   1583  C    THR A 200    60.614  39.365  27.521  1.00 46.91          C
ATOM   1584  O    THR A 200    60.839  38.741  28.570  1.00 48.15          O
ATOM   1585  CB   THR A 200    58.282  39.944  28.068  1.00 44.74          C
ATOM   1586  OG1  THR A 200    57.683  39.092  27.087  1.00 44.80          O
ATOM   1587  CG2  THR A 200    57.321  41.053  28.452  1.00 44.59          C
ATOM   1588  N    SER A 201    61.195  39.052  26.369  1.00 47.93          N
ATOM   1589  CA   SER A 201    62.232  38.035  26.287  1.00 49.09          C
ATOM   1590  C    SER A 201    63.091  38.321  25.052  1.00 49.38          C
ATOM   1591  O    SER A 201    62.560  38.503  23.948  1.00 48.80          O
ATOM   1592  CB   SER A 201    61.589  36.636  26.227  1.00 49.72          C
ATOM   1593  OG   SER A 201    62.461  35.635  25.710  1.00 51.04          O
ATOM   1594  N    THR A 202    64.411  38.373  25.243  1.00 49.62          N
ATOM   1595  CA   THR A 202    65.336  38.556  24.121  1.00 50.13          C
ATOM   1596  C    THR A 202    65.289  37.334  23.205  1.00 50.14          C
ATOM   1597  O    THR A 202    65.611  37.422  22.014  1.00 50.13          O
ATOM   1598  CB   THR A 202    66.792  38.766  24.602  1.00 50.73          C
ATOM   1599  OG1  THR A 202    67.211  37.640  25.388  1.00 50.97          O
ATOM   1600  CG2  THR A 202    66.913  40.050  25.428  1.00 50.68          C
ATOM   1601  N    SER A 203    64.882  36.204  23.786  1.00 49.42          N
ATOM   1602  CA   SER A 203    64.805  34.923  23.104  1.00 49.21          C
ATOM   1603  C    SER A 203    63.366  34.692  22.679  1.00 47.81          C
ATOM   1604  O    SER A 203    62.461  34.947  23.475  1.00 47.86          O
ATOM   1605  CB   SER A 203    65.193  33.814  24.092  1.00 50.10          C
ATOM   1606  OG   SER A 203    65.752  34.358  25.294  1.00 51.72          O
ATOM   1607  N    PRO A 204    63.136  34.175  21.454  1.00 46.24          N
ATOM   1608  CA   PRO A 204    61.736  33.886  21.096  1.00 45.55          C
ATOM   1609  C    PRO A 204    61.135  32.824  22.022  1.00 45.20          C
ATOM   1610  O    PRO A 204    61.859  31.938  22.466  1.00 45.51          O
ATOM   1611  CB   PRO A 204    61.828  33.348  19.663  1.00 45.44          C
ATOM   1612  CG   PRO A 204    63.214  33.656  19.188  1.00 45.71          C
ATOM   1613  CD   PRO A 204    64.080  33.814  20.382  1.00 45.68          C
ATOM   1614  N    ILE A 205    59.847  32.934  22.353  1.00 44.27          N
ATOM   1615  CA   ILE A 205    59.132  31.809  22.944  1.00 43.35          C
```

```
ATOM   1616  C    ILE A 205      58.750  30.919  21.783  1.00 42.85           C
ATOM   1617  O    ILE A 205      58.213  31.396  20.784  1.00 42.60           O
ATOM   1618  CB   ILE A 205      57.871  32.225  23.706  1.00 43.82           C
ATOM   1619  CG1  ILE A 205      58.268  32.948  24.994  1.00 44.18           C
ATOM   1620  CG2  ILE A 205      57.006  31.001  24.030  1.00 43.27           C
ATOM   1621  CD1  ILE A 205      57.102  33.349  25.860  1.00 44.47           C
ATOM   1622  N    VAL A 206      59.056  29.630  21.886  1.00 41.97           N
ATOM   1623  CA   VAL A 206      58.738  28.713  20.805  1.00 40.78           C
ATOM   1624  C    VAL A 206      57.921  27.524  21.307  1.00 38.88           C
ATOM   1625  O    VAL A 206      58.230  26.912  22.337  1.00 37.57           O
ATOM   1626  CB   VAL A 206      59.994  28.281  20.027  1.00 41.39           C
ATOM   1627  CG1  VAL A 206      60.952  29.457  19.860  1.00 42.13           C
ATOM   1628  CG2  VAL A 206      60.697  27.165  20.693  1.00 42.86           C
ATOM   1629  N    LYS A 207      56.850  27.240  20.575  1.00 36.75           N
ATOM   1630  CA   LYS A 207      56.095  26.016  20.738  1.00 35.64           C
ATOM   1631  C    LYS A 207      56.178  25.264  19.429  1.00 34.53           C
ATOM   1632  O    LYS A 207      56.152  25.864  18.382  1.00 32.92           O
ATOM   1633  CB   LYS A 207      54.644  26.332  21.087  1.00 35.37           C
ATOM   1634  CG   LYS A 207      54.511  27.059  22.397  1.00 36.35           C
ATOM   1635  CD   LYS A 207      54.614  26.115  23.585  1.00 38.38           C
ATOM   1636  CE   LYS A 207      54.839  26.872  24.869  1.00 39.69           C
ATOM   1637  NZ   LYS A 207      54.110  26.218  26.003  1.00 43.14           N
ATOM   1638  N    SER A 208      56.305  23.943  19.497  1.00 35.14           N
ATOM   1639  CA   SER A 208      56.241  23.107  18.316  1.00 34.79           C
ATOM   1640  C    SER A 208      55.595  21.778  18.626  1.00 34.76           C
ATOM   1641  O    SER A 208      55.291  21.449  19.782  1.00 32.52           O
ATOM   1642  CB   SER A 208      57.635  22.897  17.699  1.00 35.91           C
ATOM   1643  OG   SER A 208      58.602  22.524  18.665  1.00 37.55           O
ATOM   1644  N    PHE A 209      55.316  21.044  17.562  1.00 35.09           N
ATOM   1645  CA   PHE A 209      54.905  19.665  17.693  1.00 36.15           C
ATOM   1646  C    PHE A 209      55.406  18.890  16.496  1.00 37.09           C
ATOM   1647  O    PHE A 209      55.649  19.427  15.405  1.00 36.51           O
ATOM   1648  CB   PHE A 209      53.393  19.522  17.810  1.00 36.17           C
ATOM   1649  CG   PHE A 209      52.686  19.693  16.525  1.00 35.91           C
ATOM   1650  CD1  PHE A 209      52.375  18.594  15.738  1.00 36.56           C
ATOM   1651  CD2  PHE A 209      52.355  20.963  16.074  1.00 37.60           C
ATOM   1652  CE1  PHE A 209      51.755  18.750  14.519  1.00 36.99           C
ATOM   1653  CE2  PHE A 209      51.733  21.139  14.853  1.00 37.09           C
ATOM   1654  CZ   PHE A 209      51.428  20.036  14.068  1.00 37.19           C
ATOM   1655  N    ASN A 210      55.545  17.603  16.733  1.00 37.91           N
ATOM   1656  CA   ASN A 210      56.163  16.709  15.816  1.00 38.03           C
ATOM   1657  C    ASN A 210      55.172  15.566  15.636  1.00 39.37           C
ATOM   1658  O    ASN A 210      54.722  14.960  16.605  1.00 37.79           O
ATOM   1659  CB   ASN A 210      57.477  16.261  16.414  1.00 37.82           C
ATOM   1660  CG   ASN A 210      58.208  15.312  15.533  1.00 38.81           C
ATOM   1661  OD1  ASN A 210      57.752  14.189  15.314  1.00 38.97           O
ATOM   1662  ND2  ASN A 210      59.360  15.742  15.011  1.00 38.38           N
ATOM   1663  N    ARG A 211      54.822  15.283  14.392  1.00 41.54           N
ATOM   1664  CA   ARG A 211      53.758  14.342  14.106  1.00 44.37           C
ATOM   1665  C    ARG A 211      54.082  12.872  14.485  1.00 45.83           C
ATOM   1666  O    ARG A 211      53.197  12.013  14.422  1.00 46.41           O
ATOM   1667  CB   ARG A 211      53.387  14.473  12.632  1.00 44.22           C
ATOM   1668  CG   ARG A 211      51.930  14.168  12.305  1.00 45.46           C
ATOM   1669  CD   ARG A 211      51.530  14.683  10.917  1.00 44.80           C
ATOM   1670  NE   ARG A 211      52.626  14.589   9.971  1.00 44.00           N
ATOM   1671  CZ   ARG A 211      53.052  13.455   9.424  1.00 44.44           C
ATOM   1672  NH1  ARG A 211      52.459  12.293   9.690  1.00 43.61           N
ATOM   1673  NH2  ARG A 211      54.086  13.480   8.601  1.00 43.87           N
ATOM   1674  N    ASN A 212      55.321  12.602  14.917  1.00 47.28           N
ATOM   1675  CA   ASN A 212      55.821  11.237  15.171  1.00 48.21           C
ATOM   1676  C    ASN A 212      55.894  10.762  16.614  1.00 49.66           C
ATOM   1677  O    ASN A 212      56.414   9.676  16.871  1.00 50.17           O
ATOM   1678  CB   ASN A 212      57.232  11.099  14.613  1.00 49.14           C
ATOM   1679  CG   ASN A 212      57.242  10.611  13.220  1.00 50.42           C
ATOM   1680  OD1  ASN A 212      56.735  11.272  12.313  1.00 51.95           O
```

| ATOM | 1681 | ND2 | ASN | A | 212 | 57.835 | 9.432 | 13.018 | 1.00 | 53.62 | N |
|------|------|-----|-----|---|-----|--------|--------|--------|------|-------|---|
| ATOM | 1682 | N | GLU | A | 213 | 55.397 | 11.556 | 17.553 | 1.00 | 50.66 | N |
| ATOM | 1683 | CA | GLU | A | 213 | 55.564 | 11.254 | 18.959 | 1.00 | 51.20 | C |
| ATOM | 1684 | C | GLU | A | 213 | 54.208 | 11.163 | 19.640 | 1.00 | 52.16 | C |
| ATOM | 1685 | O | GLU | A | 213 | 53.460 | 12.146 | 19.685 | 1.00 | 53.27 | O |
| ATOM | 1686 | CB | GLU | A | 213 | 56.429 | 12.341 | 19.595 | 1.00 | 51.59 | C |
| ATOM | 1687 | CG | GLU | A | 213 | 57.870 | 12.338 | 19.082 | 1.00 | 50.64 | C |
| ATOM | 1688 | CD | GLU | A | 213 | 58.513 | 13.718 | 19.021 | 1.00 | 51.38 | C |
| ATOM | 1689 | OE1 | GLU | A | 213 | 57.984 | 14.679 | 19.628 | 1.00 | 52.19 | O |
| ATOM | 1690 | OE2 | GLU | A | 213 | 59.559 | 13.839 | 18.347 | 1.00 | 51.37 | O |
| TER | 1691 | | GLU | A | 213 | | | | | | |
| ATOM | 1692 | N | GLU | B | 1 | 12.783 | 52.405 | 31.703 | 1.00 | 51.79 | N |
| ATOM | 1693 | CA | GLU | B | 1 | 14.043 | 52.193 | 30.924 | 1.00 | 51.98 | C |
| ATOM | 1694 | C | GLU | B | 1 | 13.756 | 52.156 | 29.426 | 1.00 | 51.65 | C |
| ATOM | 1695 | O | GLU | B | 1 | 13.037 | 51.284 | 28.948 | 1.00 | 52.01 | O |
| ATOM | 1696 | CB | GLU | B | 1 | 14.742 | 50.897 | 31.349 | 1.00 | 52.18 | C |
| ATOM | 1697 | N | VAL | B | 2 | 14.305 | 53.125 | 28.699 | 1.00 | 51.21 | N |
| ATOM | 1698 | CA | VAL | B | 2 | 14.244 | 53.132 | 27.239 | 1.00 | 50.70 | C |
| ATOM | 1699 | C | VAL | B | 2 | 15.145 | 52.020 | 26.699 | 1.00 | 50.13 | C |
| ATOM | 1700 | O | VAL | B | 2 | 16.295 | 51.876 | 27.144 | 1.00 | 49.41 | O |
| ATOM | 1701 | CB | VAL | B | 2 | 14.692 | 54.497 | 26.648 | 1.00 | 50.49 | C |
| ATOM | 1702 | CG1 | VAL | B | 2 | 14.674 | 54.456 | 25.120 | 1.00 | 50.06 | C |
| ATOM | 1703 | CG2 | VAL | B | 2 | 13.808 | 55.636 | 27.178 | 1.00 | 50.60 | C |
| ATOM | 1704 | N | LYS | B | 3 | 14.601 | 51.218 | 25.779 | 1.00 | 49.50 | N |
| ATOM | 1705 | CA | LYS | B | 3 | 15.373 | 50.212 | 25.044 | 1.00 | 48.39 | C |
| ATOM | 1706 | C | LYS | B | 3 | 15.042 | 50.252 | 23.544 | 1.00 | 47.24 | C |
| ATOM | 1707 | O | LYS | B | 3 | 13.871 | 50.226 | 23.160 | 1.00 | 45.73 | O |
| ATOM | 1708 | CB | LYS | B | 3 | 15.097 | 48.810 | 25.576 | 1.00 | 49.26 | C |
| ATOM | 1709 | CG | LYS | B | 3 | 16.162 | 47.778 | 25.182 | 1.00 | 50.17 | C |
| ATOM | 1710 | CD | LYS | B | 3 | 15.537 | 46.455 | 24.776 | 1.00 | 50.91 | C |
| ATOM | 1711 | CE | LYS | B | 3 | 16.563 | 45.325 | 24.768 | 1.00 | 51.56 | C |
| ATOM | 1712 | NZ | LYS | B | 3 | 15.940 | 44.002 | 24.425 | 1.00 | 51.91 | N |
| ATOM | 1713 | N | VAL | B | 4 | 16.096 | 50.302 | 22.720 | 1.00 | 46.36 | N |
| ATOM | 1714 | CA | VAL | B | 4 | 15.997 | 50.273 | 21.259 | 1.00 | 44.78 | C |
| ATOM | 1715 | C | VAL | B | 4 | 16.382 | 48.874 | 20.762 | 1.00 | 44.54 | C |
| ATOM | 1716 | O | VAL | B | 4 | 17.548 | 48.483 | 20.857 | 1.00 | 43.28 | O |
| ATOM | 1717 | CB | VAL | B | 4 | 16.956 | 51.301 | 20.594 | 1.00 | 44.41 | C |
| ATOM | 1718 | CG1 | VAL | B | 4 | 16.545 | 51.556 | 19.143 | 1.00 | 43.50 | C |
| ATOM | 1719 | CG2 | VAL | B | 4 | 16.991 | 52.596 | 21.363 | 1.00 | 44.30 | C |
| ATOM | 1720 | N | GLU | B | 5 | 15.418 | 48.119 | 20.231 | 1.00 | 44.46 | N |
| ATOM | 1721 | CA | GLU | B | 5 | 15.717 | 46.779 | 19.689 | 1.00 | 45.67 | C |
| ATOM | 1722 | C | GLU | B | 5 | 15.638 | 46.781 | 18.149 | 1.00 | 43.48 | C |
| ATOM | 1723 | O | GLU | B | 5 | 14.617 | 47.160 | 17.579 | 1.00 | 43.29 | O |
| ATOM | 1724 | CB | GLU | B | 5 | 14.753 | 45.734 | 20.278 | 1.00 | 46.31 | C |
| ATOM | 1725 | CG | GLU | B | 5 | 15.453 | 44.507 | 20.905 | 1.00 | 49.23 | C |
| ATOM | 1726 | CD | GLU | B | 5 | 14.477 | 43.390 | 21.324 | 1.00 | 50.29 | C |
| ATOM | 1727 | OE1 | GLU | B | 5 | 14.941 | 42.283 | 21.740 | 1.00 | 54.11 | O |
| ATOM | 1728 | OE2 | GLU | B | 5 | 13.241 | 43.609 | 21.230 | 1.00 | 54.70 | O |
| ATOM | 1729 | N | GLU | B | 6 | 16.694 | 46.346 | 17.471 | 1.00 | 41.41 | N |
| ATOM | 1730 | CA | GLU | B | 6 | 16.678 | 46.359 | 16.002 | 1.00 | 39.85 | C |
| ATOM | 1731 | C | GLU | B | 6 | 16.916 | 45.005 | 15.395 | 1.00 | 38.24 | C |
| ATOM | 1732 | O | GLU | B | 6 | 17.499 | 44.145 | 16.024 | 1.00 | 39.17 | O |
| ATOM | 1733 | CB | GLU | B | 6 | 17.702 | 47.333 | 15.450 | 1.00 | 40.26 | C |
| ATOM | 1734 | CG | GLU | B | 6 | 19.088 | 47.026 | 15.802 | 1.00 | 38.83 | C |
| ATOM | 1735 | CD | GLU | B | 6 | 19.999 | 48.069 | 15.256 | 1.00 | 38.28 | C |
| ATOM | 1736 | OE1 | GLU | B | 6 | 20.363 | 47.949 | 14.058 | 1.00 | 35.41 | O |
| ATOM | 1737 | OE2 | GLU | B | 6 | 20.326 | 48.996 | 16.034 | 1.00 | 34.10 | O |
| ATOM | 1738 | N | SER | B | 7 | 16.462 | 44.819 | 14.162 | 1.00 | 36.66 | N |
| ATOM | 1739 | CA | SER | B | 7 | 16.596 | 43.529 | 13.496 | 1.00 | 35.35 | C |
| ATOM | 1740 | C | SER | B | 7 | 16.475 | 43.656 | 11.975 | 1.00 | 34.21 | C |
| ATOM | 1741 | O | SER | B | 7 | 16.234 | 44.738 | 11.442 | 1.00 | 32.41 | O |
| ATOM | 1742 | CB | SER | B | 7 | 15.503 | 42.578 | 13.997 | 1.00 | 35.07 | C |
| ATOM | 1743 | OG | SER | B | 7 | 14.272 | 42.913 | 13.390 | 1.00 | 34.39 | O |
| ATOM | 1744 | N | GLY | B | 8 | 16.567 | 42.508 | 11.305 | 1.00 | 33.81 | N |
| ATOM | 1745 | CA | GLY | B | 8 | 16.586 | 42.424 | 9.857 | 1.00 | 33.82 | C |

| ATOM | 1746 | C | GLY | B | 8 | 18.055 | 42.427 | 9.503 | 1.00 | 34.59 | C |
|------|------|-----|-----|---|----|--------|--------|--------|------|-------|---|
| ATOM | 1747 | O | GLY | B | 8 | 18.899 | 42.175 | 10.346 | 1.00 | 35.65 | O |
| ATOM | 1748 | N | GLY | B | 9 | 18.385 | 42.706 | 8.261 | 1.00 | 35.34 | N |
| ATOM | 1749 | CA | GLY | B | 9 | 19.792 | 42.748 | 7.886 | 1.00 | 35.15 | C |
| ATOM | 1750 | C | GLY | B | 9 | 20.089 | 41.415 | 7.274 | 1.00 | 33.72 | C |
| ATOM | 1751 | O | GLY | B | 9 | 19.199 | 40.586 | 7.127 | 1.00 | 37.61 | O |
| ATOM | 1752 | N | GLY | B | 10 | 21.333 | 41.198 | 6.914 | 1.00 | 30.51 | N |
| ATOM | 1753 | CA | GLY | B | 10 | 21.708 | 39.999 | 6.222 | 1.00 | 27.72 | C |
| ATOM | 1754 | C | GLY | B | 10 | 22.545 | 40.361 | 5.017 | 1.00 | 25.71 | C |
| ATOM | 1755 | O | GLY | B | 10 | 23.077 | 41.463 | 4.926 | 1.00 | 23.31 | O |
| ATOM | 1756 | N | LEU | B | 11 | 22.631 | 39.419 | 4.090 | 1.00 | 24.03 | N |
| ATOM | 1757 | CA | LEU | B | 11 | 23.488 | 39.543 | 2.912 | 1.00 | 22.89 | C |
| ATOM | 1758 | C | LEU | B | 11 | 22.619 | 39.618 | 1.675 | 1.00 | 21.41 | C |
| ATOM | 1759 | O | LEU | B | 11 | 21.621 | 38.940 | 1.574 | 1.00 | 19.13 | O |
| ATOM | 1760 | CB | LEU | B | 11 | 24.408 | 38.328 | 2.846 | 1.00 | 22.43 | C |
| ATOM | 1761 | CG | LEU | B | 11 | 25.214 | 38.137 | 1.566 | 1.00 | 20.68 | C |
| ATOM | 1762 | CD1 | LEU | B | 11 | 26.270 | 39.212 | 1.472 | 1.00 | 19.06 | C |
| ATOM | 1763 | CD2 | LEU | B | 11 | 25.816 | 36.723 | 1.509 | 1.00 | 22.36 | C |
| ATOM | 1764 | N | VAL | B | 12 | 22.979 | 40.477 | 0.736 | 1.00 | 19.46 | N |
| ATOM | 1765 | CA | VAL | B | 12 | 22.396 | 40.444 | -0.583 | 1.00 | 19.42 | C |
| ATOM | 1766 | C | VAL | B | 12 | 23.558 | 40.722 | -1.577 | 1.00 | 20.47 | C |
| ATOM | 1767 | O | VAL | B | 12 | 24.648 | 41.166 | -1.162 | 1.00 | 18.34 | O |
| ATOM | 1768 | CB | VAL | B | 12 | 21.253 | 41.502 | -0.759 | 1.00 | 20.71 | C |
| ATOM | 1769 | CG1 | VAL | B | 12 | 20.040 | 41.218 | 0.156 | 1.00 | 20.71 | C |
| ATOM | 1770 | CG2 | VAL | B | 12 | 21.767 | 42.966 | -0.515 | 1.00 | 20.07 | C |
| ATOM | 1771 | N | GLN | B | 13 | 23.341 | 40.417 | -2.849 | 1.00 | 19.53 | N |
| ATOM | 1772 | CA | GLN | B | 13 | 24.272 | 40.853 | -3.896 | 1.00 | 22.61 | C |
| ATOM | 1773 | C | GLN | B | 13 | 24.005 | 42.318 | -4.219 | 1.00 | 21.00 | C |
| ATOM | 1774 | O | GLN | B | 13 | 22.912 | 42.850 | -3.933 | 1.00 | 20.12 | O |
| ATOM | 1775 | CB | GLN | B | 13 | 24.119 | 40.038 | -5.168 | 1.00 | 22.34 | C |
| ATOM | 1776 | CG | GLN | B | 13 | 24.310 | 38.553 | -5.048 | 1.00 | 27.28 | C |
| ATOM | 1777 | CD | GLN | B | 13 | 23.707 | 37.770 | -6.238 | 1.00 | 27.64 | C |
| ATOM | 1778 | OE1 | GLN | B | 13 | 22.561 | 37.298 | -6.185 | 1.00 | 34.53 | O |
| ATOM | 1779 | NE2 | GLN | B | 13 | 24.475 | 37.630 | -7.289 | 1.00 | 30.17 | N |
| ATOM | 1780 | N | PRO | B | 14 | 24.979 | 42.988 | -4.852 | 1.00 | 20.56 | N |
| ATOM | 1781 | CA | PRO | B | 14 | 24.696 | 44.333 | -5.295 | 1.00 | 19.64 | C |
| ATOM | 1782 | C | PRO | B | 14 | 23.397 | 44.373 | -6.086 | 1.00 | 19.61 | C |
| ATOM | 1783 | O | PRO | B | 14 | 23.123 | 43.470 | -6.877 | 1.00 | 20.72 | O |
| ATOM | 1784 | CB | PRO | B | 14 | 25.905 | 44.651 | -6.207 | 1.00 | 20.34 | C |
| ATOM | 1785 | CG | PRO | B | 14 | 26.972 | 43.886 | -5.664 | 1.00 | 19.49 | C |
| ATOM | 1786 | CD | PRO | B | 14 | 26.335 | 42.571 | -5.237 | 1.00 | 20.55 | C |
| ATOM | 1787 | N | GLY | B | 15 | 22.579 | 45.394 | -5.852 | 1.00 | 19.08 | N |
| ATOM | 1788 | CA | GLY | B | 15 | 21.304 | 45.504 | -6.510 | 1.00 | 19.01 | C |
| ATOM | 1789 | C | GLY | B | 15 | 20.192 | 45.000 | -5.604 | 1.00 | 18.40 | C |
| ATOM | 1790 | O | GLY | B | 15 | 19.061 | 45.306 | -5.823 | 1.00 | 20.35 | O |
| ATOM | 1791 | N | GLY | B | 16 | 20.524 | 44.239 | -4.587 | 1.00 | 20.02 | N |
| ATOM | 1792 | CA | GLY | B | 16 | 19.502 | 43.604 | -3.731 | 1.00 | 19.36 | C |
| ATOM | 1793 | C | GLY | B | 16 | 18.885 | 44.543 | -2.724 | 1.00 | 21.47 | C |
| ATOM | 1794 | O | GLY | B | 16 | 19.277 | 45.715 | -2.584 | 1.00 | 19.08 | O |
| ATOM | 1795 | N | SER | B | 17 | 17.904 | 44.018 | -1.986 | 1.00 | 23.11 | N |
| ATOM | 1796 | CA | SER | B | 17 | 17.052 | 44.848 | -1.185 | 1.00 | 24.67 | C |
| ATOM | 1797 | C | SER | B | 17 | 16.841 | 44.175 | 0.156 | 1.00 | 26.05 | C |
| ATOM | 1798 | O | SER | B | 17 | 16.885 | 42.976 | 0.256 | 1.00 | 23.38 | O |
| ATOM | 1799 | CB | SER | B | 17 | 15.698 | 45.061 | -1.870 | 1.00 | 25.48 | C |
| ATOM | 1800 | OG | SER | B | 17 | 15.846 | 45.806 | -3.052 | 1.00 | 27.70 | O |
| ATOM | 1801 | N | MET | B | 18 | 16.594 | 44.993 | 1.162 | 1.00 | 27.97 | N |
| ATOM | 1802 | CA | MET | B | 18 | 16.593 | 44.588 | 2.532 | 1.00 | 32.52 | C |
| ATOM | 1803 | C | MET | B | 18 | 15.793 | 45.657 | 3.242 | 1.00 | 32.71 | C |
| ATOM | 1804 | O | MET | B | 18 | 15.861 | 46.835 | 2.888 | 1.00 | 29.08 | O |
| ATOM | 1805 | CB | MET | B | 18 | 18.024 | 44.632 | 2.999 | 1.00 | 34.50 | C |
| ATOM | 1806 | CG | MET | B | 18 | 18.341 | 43.968 | 4.253 | 1.00 | 35.72 | C |
| ATOM | 1807 | SD | MET | B | 18 | 20.121 | 43.977 | 4.423 | 1.00 | 38.66 | S |
| ATOM | 1808 | CE | MET | B | 18 | 20.632 | 42.839 | 3.154 | 1.00 | 38.54 | C |
| ATOM | 1809 | N | LYS | B | 19 | 14.987 | 45.229 | 4.203 | 1.00 | 34.21 | N |
| ATOM | 1810 | CA | LYS | B | 19 | 14.264 | 46.164 | 5.045 | 1.00 | 34.97 | C |

| ATOM | 1811 | C | LYS | B | 19 | 14.715 | 45.853 | 6.430 | 1.00 | 34.54 | C |
|------|------|------|-----|---|----|--------|--------|-------|------|-------|---|
| ATOM | 1812 | O | LYS | B | 19 | 14.578 | 44.709 | 6.872 | 1.00 | 35.87 | O |
| ATOM | 1813 | CB | LYS | B | 19 | 12.753 | 45.998 | 4.973 | 1.00 | 34.17 | C |
| ATOM | 1814 | CG | LYS | B | 19 | 12.047 | 47.082 | 5.802 | 1.00 | 35.99 | C |
| ATOM | 1815 | CD | LYS | B | 19 | 10.530 | 47.090 | 5.632 | 1.00 | 36.90 | C |
| ATOM | 1816 | CE | LYS | B | 19 | 9.963 | 48.496 | 5.601 | 1.00 | 36.97 | C |
| ATOM | 1817 | NZ | LYS | B | 19 | 8.502 | 48.513 | 5.967 | 1.00 | 38.50 | N |
| ATOM | 1818 | N | ILE | B | 20 | 15.276 | 46.839 | 7.112 | 1.00 | 34.24 | N |
| ATOM | 1819 | CA | ILE | B | 20 | 15.582 | 46.660 | 8.523 | 1.00 | 34.71 | C |
| ATOM | 1820 | C | ILE | B | 20 | 14.685 | 47.532 | 9.395 | 1.00 | 34.39 | C |
| ATOM | 1821 | O | ILE | B | 20 | 13.928 | 48.372 | 8.896 | 1.00 | 33.56 | O |
| ATOM | 1822 | CB | ILE | B | 20 | 17.079 | 46.839 | 8.822 | 1.00 | 34.85 | C |
| ATOM | 1823 | CG1 | ILE | B | 20 | 17.521 | 48.283 | 8.659 | 1.00 | 33.67 | C |
| ATOM | 1824 | CG2 | ILE | B | 20 | 17.890 | 45.895 | 7.915 | 1.00 | 35.20 | C |
| ATOM | 1825 | CD1 | ILE | B | 20 | 19.027 | 48.429 | 8.736 | 1.00 | 34.44 | C |
| ATOM | 1826 | N | SER | B | 21 | 14.745 | 47.296 | 10.694 | 1.00 | 34.02 | N |
| ATOM | 1827 | CA | SER | B | 21 | 13.810 | 47.924 | 11.600 | 1.00 | 34.57 | C |
| ATOM | 1828 | C | SER | B | 21 | 14.298 | 47.956 | 13.019 | 1.00 | 33.29 | C |
| ATOM | 1829 | O | SER | B | 21 | 15.210 | 47.241 | 13.409 | 1.00 | 32.37 | O |
| ATOM | 1830 | CB | SER | B | 21 | 12.477 | 47.194 | 11.566 | 1.00 | 34.99 | C |
| ATOM | 1831 | OG | SER | B | 21 | 12.613 | 45.915 | 12.128 | 1.00 | 37.65 | O |
| ATOM | 1832 | N | CYS | B | 22 | 13.691 | 48.830 | 13.791 | 1.00 | 35.25 | N |
| ATOM | 1833 | CA | CYS | B | 22 | 13.827 | 48.729 | 15.209 | 1.00 | 37.30 | C |
| ATOM | 1834 | C | CYS | B | 22 | 12.634 | 49.262 | 15.933 | 1.00 | 36.97 | C |
| ATOM | 1835 | O | CYS | B | 22 | 11.867 | 50.065 | 15.406 | 1.00 | 36.78 | O |
| ATOM | 1836 | CB | CYS | B | 22 | 15.054 | 49.445 | 15.705 | 1.00 | 38.37 | C |
| ATOM | 1837 | SG | CYS | B | 22 | 14.973 | 51.162 | 15.497 | 1.00 | 42.07 | S |
| ATOM | 1838 | N | VAL | B | 23 | 12.542 | 48.809 | 17.172 | 1.00 | 36.98 | N |
| ATOM | 1839 | CA | VAL | B | 23 | 11.437 | 49.116 | 18.045 | 1.00 | 37.43 | C |
| ATOM | 1840 | C | VAL | B | 23 | 11.981 | 49.695 | 19.341 | 1.00 | 35.75 | C |
| ATOM | 1841 | O | VAL | B | 23 | 12.946 | 49.190 | 19.914 | 1.00 | 33.24 | O |
| ATOM | 1842 | CB | VAL | B | 23 | 10.562 | 47.865 | 18.311 | 1.00 | 38.44 | C |
| ATOM | 1843 | CG1 | VAL | B | 23 | 9.874 | 47.452 | 17.036 | 1.00 | 39.87 | C |
| ATOM | 1844 | CG2 | VAL | B | 23 | 11.389 | 46.697 | 18.851 | 1.00 | 39.48 | C |
| ATOM | 1845 | N | VAL | B | 24 | 11.387 | 50.802 | 19.750 | 1.00 | 36.39 | N |
| ATOM | 1846 | CA | VAL | B | 24 | 11.749 | 51.426 | 20.989 | 1.00 | 38.12 | C |
| ATOM | 1847 | C | VAL | B | 24 | 10.655 | 51.099 | 21.996 | 1.00 | 37.97 | C |
| ATOM | 1848 | O | VAL | B | 24 | 9.475 | 51.082 | 21.662 | 1.00 | 36.07 | O |
| ATOM | 1849 | CB | VAL | B | 24 | 11.928 | 52.935 | 20.842 | 1.00 | 39.02 | C |
| ATOM | 1850 | CG1 | VAL | B | 24 | 12.475 | 53.533 | 22.158 | 1.00 | 39.60 | C |
| ATOM | 1851 | CG2 | VAL | B | 24 | 12.906 | 53.224 | 19.714 | 1.00 | 40.71 | C |
| ATOM | 1852 | N | SER | B | 25 | 11.078 | 50.796 | 23.209 | 1.00 | 38.91 | N |
| ATOM | 1853 | CA | SER | B | 25 | 10.164 | 50.608 | 24.321 | 1.00 | 40.04 | C |
| ATOM | 1854 | C | SER | B | 25 | 10.592 | 51.552 | 25.435 | 1.00 | 40.81 | C |
| ATOM | 1855 | O | SER | B | 25 | 11.756 | 51.974 | 25.514 | 1.00 | 39.80 | O |
| ATOM | 1856 | CB | SER | B | 25 | 10.205 | 49.170 | 24.804 | 1.00 | 39.89 | C |
| ATOM | 1857 | OG | SER | B | 25 | 11.502 | 48.833 | 25.245 | 1.00 | 42.47 | O |
| ATOM | 1858 | N | GLY | B | 26 | 9.636 | 51.897 | 26.277 | 1.00 | 41.36 | N |
| ATOM | 1859 | CA | GLY | B | 26 | 9.915 | 52.707 | 27.437 | 1.00 | 42.63 | C |
| ATOM | 1860 | C | GLY | B | 26 | 9.776 | 54.193 | 27.205 | 1.00 | 42.28 | C |
| ATOM | 1861 | O | GLY | B | 26 | 9.932 | 54.939 | 28.170 | 1.00 | 43.33 | O |
| ATOM | 1862 | N | SER | B | 30 | 6.025 | 58.548 | 19.587 | 1.00 | 51.80 | N |
| ATOM | 1863 | CA | SER | B | 30 | 5.377 | 59.844 | 19.512 | 1.00 | 52.01 | C |
| ATOM | 1864 | C | SER | B | 30 | 6.363 | 61.008 | 19.685 | 1.00 | 51.67 | C |
| ATOM | 1865 | O | SER | B | 30 | 6.468 | 61.860 | 18.814 | 1.00 | 50.90 | O |
| ATOM | 1866 | CB | SER | B | 30 | 4.249 | 59.938 | 20.551 | 1.00 | 52.43 | C |
| ATOM | 1867 | OG | SER | B | 30 | 4.673 | 59.516 | 21.843 | 1.00 | 53.23 | O |
| ATOM | 1868 | N | ASN | B | 31 | 7.096 | 61.027 | 20.797 | 1.00 | 51.47 | N |
| ATOM | 1869 | CA | ASN | B | 31 | 7.839 | 62.230 | 21.210 | 1.00 | 50.74 | C |
| ATOM | 1870 | C | ASN | B | 31 | 9.269 | 62.195 | 20.710 | 1.00 | 49.58 | C |
| ATOM | 1871 | O | ASN | B | 31 | 10.114 | 62.993 | 21.157 | 1.00 | 49.58 | O |
| ATOM | 1872 | CB | ASN | B | 31 | 7.862 | 62.392 | 22.746 | 1.00 | 51.28 | C |
| ATOM | 1873 | CG | ASN | B | 31 | 6.506 | 62.143 | 23.406 | 1.00 | 52.31 | C |
| ATOM | 1874 | OD1 | ASN | B | 31 | 6.446 | 61.662 | 24.548 | 1.00 | 51.73 | O |
| ATOM | 1875 | ND2 | ASN | B | 31 | 5.411 | 62.474 | 22.698 | 1.00 | 54.12 | N |

| ATOM | 1876 | N | TYR | B | 32 | 9.525 | 61.301 | 19.754 | 1.00 | 46.98 | N |
|------|------|-----|-----|---|----|-------|--------|--------|------|-------|---|
| ATOM | 1877 | CA | TYR | B | 32 | 10.865 | 60.834 | 19.501 | 1.00 | 44.33 | C |
| ATOM | 1878 | C | TYR | B | 32 | 11.272 | 60.905 | 18.039 | 1.00 | 41.14 | C |
| ATOM | 1879 | O | TYR | B | 32 | 10.501 | 60.620 | 17.129 | 1.00 | 40.57 | O |
| ATOM | 1880 | CB | TYR | B | 32 | 11.011 | 59.415 | 20.023 | 1.00 | 45.02 | C |
| ATOM | 1881 | CG | TYR | B | 32 | 10.378 | 58.317 | 19.187 | 1.00 | 46.00 | C |
| ATOM | 1882 | CD1 | TYR | B | 32 | 11.184 | 57.388 | 18.517 | 1.00 | 45.52 | C |
| ATOM | 1883 | CD2 | TYR | B | 32 | 8.984 | 58.154 | 19.117 | 1.00 | 46.00 | C |
| ATOM | 1884 | CE1 | TYR | B | 32 | 10.633 | 56.354 | 17.763 | 1.00 | 46.53 | C |
| ATOM | 1885 | CE2 | TYR | B | 32 | 8.415 | 57.093 | 18.360 | 1.00 | 47.06 | C |
| ATOM | 1886 | CZ | TYR | B | 32 | 9.255 | 56.197 | 17.687 | 1.00 | 47.47 | C |
| ATOM | 1887 | OH | TYR | B | 32 | 8.768 | 55.138 | 16.927 | 1.00 | 47.24 | O |
| ATOM | 1888 | N | TRP | B | 33 | 12.610 | 61.409 | 17.864 | 1.00 | 40.33 | N |
| ATOM | 1889 | CA | TRP | B | 33 | 13.145 | 61.340 | 16.531 | 1.00 | 36.70 | C |
| ATOM | 1890 | C | TRP | B | 33 | 13.932 | 60.059 | 16.473 | 1.00 | 33.71 | C |
| ATOM | 1891 | O | TRP | B | 33 | 14.486 | 59.635 | 17.478 | 1.00 | 31.44 | O |
| ATOM | 1892 | CB | TRP | B | 33 | 14.067 | 62.526 | 16.296 | 1.00 | 36.25 | C |
| ATOM | 1893 | CG | TRP | B | 33 | 13.401 | 63.841 | 16.366 | 1.00 | 35.85 | C |
| ATOM | 1894 | CD1 | TRP | B | 33 | 12.062 | 64.086 | 16.469 | 1.00 | 35.47 | C |
| ATOM | 1895 | CD2 | TRP | B | 33 | 14.036 | 65.120 | 16.291 | 1.00 | 36.39 | C |
| ATOM | 1896 | NE1 | TRP | B | 33 | 11.831 | 65.429 | 16.484 | 1.00 | 35.50 | N |
| ATOM | 1897 | CE2 | TRP | B | 33 | 13.026 | 66.090 | 16.364 | 1.00 | 35.80 | C |
| ATOM | 1898 | CE3 | TRP | B | 33 | 15.364 | 65.539 | 16.169 | 1.00 | 36.16 | C |
| ATOM | 1899 | CZ2 | TRP | B | 33 | 13.300 | 67.450 | 16.330 | 1.00 | 36.66 | C |
| ATOM | 1900 | CZ3 | TRP | B | 33 | 15.630 | 66.893 | 16.133 | 1.00 | 35.69 | C |
| ATOM | 1901 | CH2 | TRP | B | 33 | 14.607 | 67.831 | 16.210 | 1.00 | 35.82 | C |
| ATOM | 1902 | N | MET | B | 34 | 13.990 | 59.441 | 15.300 | 1.00 | 31.77 | N |
| ATOM | 1903 | CA | MET | B | 34 | 14.764 | 58.235 | 15.119 | 1.00 | 29.67 | C |
| ATOM | 1904 | C | MET | B | 34 | 15.703 | 58.397 | 13.929 | 1.00 | 26.62 | C |
| ATOM | 1905 | O | MET | B | 34 | 15.289 | 58.902 | 12.928 | 1.00 | 24.28 | O |
| ATOM | 1906 | CB | MET | B | 34 | 13.823 | 57.099 | 14.856 | 1.00 | 31.95 | C |
| ATOM | 1907 | CG | MET | B | 34 | 13.111 | 56.590 | 16.104 | 1.00 | 34.77 | C |
| ATOM | 1908 | SD | MET | B | 34 | 14.238 | 55.783 | 17.265 | 1.00 | 44.61 | S |
| ATOM | 1909 | CE | MET | B | 34 | 15.635 | 55.183 | 16.271 | 1.00 | 39.39 | C |
| ATOM | 1910 | N | SER | B | 35 | 16.943 | 57.933 | 14.045 | 1.00 | 25.43 | N |
| ATOM | 1911 | CA | SER | B | 35 | 17.866 | 57.955 | 12.918 | 1.00 | 23.23 | C |
| ATOM | 1912 | C | SER | B | 35 | 18.542 | 56.614 | 12.718 | 1.00 | 21.46 | C |
| ATOM | 1913 | O | SER | B | 35 | 18.496 | 55.749 | 13.574 | 1.00 | 21.41 | O |
| ATOM | 1914 | CB | SER | B | 35 | 18.935 | 59.031 | 13.132 | 1.00 | 23.68 | C |
| ATOM | 1915 | OG | SER | B | 35 | 19.828 | 58.658 | 14.164 | 1.00 | 22.30 | O |
| ATOM | 1916 | N | TRP | B | 36 | 19.208 | 56.462 | 11.579 | 1.00 | 19.60 | N |
| ATOM | 1917 | CA | TRP | B | 36 | 19.994 | 55.298 | 11.326 | 1.00 | 19.63 | C |
| ATOM | 1918 | C | TRP | B | 36 | 21.445 | 55.815 | 11.092 | 1.00 | 18.79 | C |
| ATOM | 1919 | O | TRP | B | 36 | 21.643 | 56.810 | 10.367 | 1.00 | 14.70 | O |
| ATOM | 1920 | CB | TRP | B | 36 | 19.458 | 54.540 | 10.116 | 1.00 | 23.19 | C |
| ATOM | 1921 | CG | TRP | B | 36 | 18.152 | 53.767 | 10.362 | 1.00 | 24.89 | C |
| ATOM | 1922 | CD1 | TRP | B | 36 | 16.874 | 54.206 | 10.128 | 1.00 | 26.22 | C |
| ATOM | 1923 | CD2 | TRP | B | 36 | 18.031 | 52.451 | 10.889 | 1.00 | 25.63 | C |
| ATOM | 1924 | NE1 | TRP | B | 36 | 15.954 | 53.224 | 10.488 | 1.00 | 26.69 | N |
| ATOM | 1925 | CE2 | TRP | B | 36 | 16.648 | 52.132 | 10.934 | 1.00 | 26.66 | C |
| ATOM | 1926 | CE3 | TRP | B | 36 | 18.949 | 51.503 | 11.333 | 1.00 | 28.16 | C |
| ATOM | 1927 | CZ2 | TRP | B | 36 | 16.177 | 50.910 | 11.406 | 1.00 | 28.44 | C |
| ATOM | 1928 | CZ3 | TRP | B | 36 | 18.463 | 50.272 | 11.825 | 1.00 | 27.71 | C |
| ATOM | 1929 | CH2 | TRP | B | 36 | 17.099 | 49.996 | 11.852 | 1.00 | 27.62 | C |
| ATOM | 1930 | N | VAL | B | 37 | 22.386 | 55.151 | 11.750 | 1.00 | 17.82 | N |
| ATOM | 1931 | CA | VAL | B | 37 | 23.814 | 55.458 | 11.684 | 1.00 | 20.75 | C |
| ATOM | 1932 | C | VAL | B | 37 | 24.501 | 54.159 | 11.334 | 1.00 | 21.99 | C |
| ATOM | 1933 | O | VAL | B | 37 | 24.240 | 53.133 | 11.957 | 1.00 | 23.42 | O |
| ATOM | 1934 | CB | VAL | B | 37 | 24.330 | 55.967 | 13.041 | 1.00 | 20.02 | C |
| ATOM | 1935 | CG1 | VAL | B | 37 | 25.843 | 56.152 | 13.034 | 1.00 | 22.06 | C |
| ATOM | 1936 | CG2 | VAL | B | 37 | 23.623 | 57.253 | 13.394 | 1.00 | 19.60 | C |
| ATOM | 1937 | N | ARG | B | 38 | 25.358 | 54.170 | 10.324 | 1.00 | 22.43 | N |
| ATOM | 1938 | CA | ARG | B | 38 | 26.074 | 52.970 | 9.991 | 1.00 | 23.51 | C |
| ATOM | 1939 | C | ARG | B | 38 | 27.579 | 53.073 | 10.282 | 1.00 | 25.12 | C |
| ATOM | 1940 | O | ARG | B | 38 | 28.211 | 54.159 | 10.186 | 1.00 | 25.62 | O |

| ATOM | 1941 | CB | ARG B | 38 | 25.769 | 52.541 | 8.556 | 1.00 23.66 | C |
|------|------|------|-------|----|--------|--------|-------|------------|---|
| ATOM | 1942 | CG | ARG B | 38 | 26.462 | 53.339 | 7.529 | 1.00 25.45 | C |
| ATOM | 1943 | CD | ARG B | 38 | 26.039 | 52.951 | 6.184 | 1.00 26.58 | C |
| ATOM | 1944 | NE | ARG B | 38 | 26.534 | 53.925 | 5.241 | 1.00 28.55 | N |
| ATOM | 1945 | CZ | ARG B | 38 | 26.132 | 54.008 | 3.983 | 1.00 29.33 | C |
| ATOM | 1946 | NH1 | ARG B | 38 | 25.202 | 53.176 | 3.502 | 1.00 29.15 | N |
| ATOM | 1947 | NH2 | ARG B | 38 | 26.669 | 54.923 | 3.201 | 1.00 29.25 | N |
| ATOM | 1948 | N | GLN B | 39 | 28.141 | 51.923 | 10.618 | 1.00 25.62 | N |
| ATOM | 1949 | CA | GLN B | 39 | 29.534 | 51.830 | 11.027 | 1.00 26.01 | C |
| ATOM | 1950 | C | GLN B | 39 | 30.307 | 50.835 | 10.155 | 1.00 26.58 | C |
| ATOM | 1951 | O | GLN B | 39 | 29.892 | 49.697 | 9.953 | 1.00 24.97 | O |
| ATOM | 1952 | CB | GLN B | 39 | 29.602 | 51.419 | 12.492 | 1.00 26.07 | C |
| ATOM | 1953 | CG | GLN B | 39 | 30.922 | 51.739 | 13.151 | 1.00 27.55 | C |
| ATOM | 1954 | CD | GLN B | 39 | 30.937 | 51.472 | 14.634 | 1.00 26.95 | C |
| ATOM | 1955 | OE1 | GLN B | 39 | 30.164 | 50.640 | 15.159 | 1.00 29.13 | O |
| ATOM | 1956 | NE2 | GLN B | 39 | 31.819 | 52.165 | 15.326 | 1.00 25.63 | N |
| ATOM | 1957 | N | SER B | 40 | 31.431 | 51.290 | 9.621 | 1.00 28.90 | N |
| ATOM | 1958 | CA | SER B | 40 | 32.347 | 50.423 | 8.898 | 1.00 30.70 | C |
| ATOM | 1959 | C | SER B | 40 | 33.774 | 50.832 | 9.243 | 1.00 32.37 | C |
| ATOM | 1960 | O | SER B | 40 | 34.024 | 51.980 | 9.613 | 1.00 34.68 | O |
| ATOM | 1961 | CB | SER B | 40 | 32.113 | 50.551 | 7.391 | 1.00 31.07 | C |
| ATOM | 1962 | OG | SER B | 40 | 32.448 | 51.857 | 6.941 | 1.00 31.33 | O |
| ATOM | 1963 | N | PRO B | 41 | 34.723 | 49.914 | 9.125 | 1.00 34.04 | N |
| ATOM | 1964 | CA | PRO B | 41 | 36.096 | 50.328 | 9.442 | 1.00 35.83 | C |
| ATOM | 1965 | C | PRO B | 41 | 36.655 | 51.424 | 8.520 | 1.00 37.82 | C |
| ATOM | 1966 | O | PRO B | 41 | 37.513 | 52.220 | 8.939 | 1.00 38.57 | O |
| ATOM | 1967 | CB | PRO B | 41 | 36.893 | 49.026 | 9.301 | 1.00 35.91 | C |
| ATOM | 1968 | CG | PRO B | 41 | 35.860 | 47.933 | 9.402 | 1.00 36.08 | C |
| ATOM | 1969 | CD | PRO B | 41 | 34.640 | 48.491 | 8.766 | 1.00 34.33 | C |
| ATOM | 1970 | N | GLU B | 42 | 36.139 | 51.491 | 7.298 | 1.00 39.17 | N |
| ATOM | 1971 | CA | GLU B | 42 | 36.677 | 52.388 | 6.288 | 1.00 40.16 | C |
| ATOM | 1972 | C | GLU B | 42 | 36.051 | 53.782 | 6.308 | 1.00 39.89 | C |
| ATOM | 1973 | O | GLU B | 42 | 36.713 | 54.740 | 5.940 | 1.00 40.28 | O |
| ATOM | 1974 | CB | GLU B | 42 | 36.576 | 51.760 | 4.886 | 1.00 42.63 | C |
| ATOM | 1975 | CG | GLU B | 42 | 35.201 | 51.205 | 4.504 | 1.00 46.27 | C |
| ATOM | 1976 | CD | GLU B | 42 | 34.940 | 49.796 | 5.046 | 1.00 47.57 | C |
| ATOM | 1977 | OE1 | GLU B | 42 | 35.859 | 49.226 | 5.665 | 1.00 49.75 | O |
| ATOM | 1978 | OE2 | GLU B | 42 | 33.816 | 49.264 | 4.857 | 1.00 49.90 | O |
| ATOM | 1979 | N | LYS B | 43 | 34.798 | 53.911 | 6.741 | 1.00 37.78 | N |
| ATOM | 1980 | CA | LYS B | 43 | 34.135 | 55.233 | 6.810 | 1.00 37.02 | C |
| ATOM | 1981 | C | LYS B | 43 | 33.707 | 55.661 | 8.220 | 1.00 35.50 | C |
| ATOM | 1982 | O | LYS B | 43 | 33.114 | 56.728 | 8.374 | 1.00 35.65 | O |
| ATOM | 1983 | CB | LYS B | 43 | 32.910 | 55.254 | 5.892 | 1.00 37.47 | C |
| ATOM | 1984 | CG | LYS B | 43 | 33.226 | 55.518 | 4.412 | 1.00 39.48 | C |
| ATOM | 1985 | CD | LYS B | 43 | 33.211 | 57.020 | 4.082 | 1.00 40.13 | C |
| ATOM | 1986 | CE | LYS B | 43 | 32.670 | 57.314 | 2.681 | 1.00 40.63 | C |
| ATOM | 1987 | NZ | LYS B | 43 | 31.797 | 58.536 | 2.674 | 1.00 40.70 | N |
| ATOM | 1988 | N | GLY B | 44 | 33.986 | 54.842 | 9.239 | 1.00 33.59 | N |
| ATOM | 1989 | CA | GLY B | 44 | 33.602 | 55.164 | 10.633 | 1.00 32.93 | C |
| ATOM | 1990 | C | GLY B | 44 | 32.098 | 55.181 | 10.831 | 1.00 30.42 | C |
| ATOM | 1991 | O | GLY B | 44 | 31.395 | 54.479 | 10.127 | 1.00 30.02 | O |
| ATOM | 1992 | N | LEU B | 45 | 31.615 | 55.988 | 11.776 | 1.00 29.15 | N |
| ATOM | 1993 | CA | LEU B | 45 | 30.176 | 56.204 | 12.005 | 1.00 28.12 | C |
| ATOM | 1994 | C | LEU B | 45 | 29.594 | 57.249 | 11.074 | 1.00 27.09 | C |
| ATOM | 1995 | O | LEU B | 45 | 30.011 | 58.399 | 11.115 | 1.00 29.55 | O |
| ATOM | 1996 | CB | LEU B | 45 | 29.906 | 56.654 | 13.446 | 1.00 28.40 | C |
| ATOM | 1997 | CG | LEU B | 45 | 30.137 | 55.628 | 14.559 | 1.00 27.04 | C |
| ATOM | 1998 | CD1 | LEU B | 45 | 30.193 | 56.304 | 15.903 | 1.00 25.32 | C |
| ATOM | 1999 | CD2 | LEU B | 45 | 29.038 | 54.588 | 14.514 | 1.00 27.38 | C |
| ATOM | 2000 | N | GLU B | 46 | 28.623 | 56.850 | 10.251 | 1.00 25.99 | N |
| ATOM | 2001 | CA | GLU B | 46 | 28.026 | 57.681 | 9.208 | 1.00 25.47 | C |
| ATOM | 2002 | C | GLU B | 46 | 26.515 | 57.721 | 9.427 | 1.00 24.13 | C |
| ATOM | 2003 | O | GLU B | 46 | 25.880 | 56.682 | 9.345 | 1.00 21.09 | O |
| ATOM | 2004 | CB | GLU B | 46 | 28.233 | 57.064 | 7.833 | 1.00 26.48 | C |
| ATOM | 2005 | CG | GLU B | 46 | 29.436 | 57.548 | 7.016 | 1.00 31.31 | C |

| ATOM | 2006 | CD | GLU | B | 46 | 29.339 | 57.128 | 5.537 | 1.00 | 31.53 | C |
|------|------|-----|-----|---|----|--------|--------|--------|------|-------|---|
| ATOM | 2007 | OE1 | GLU | B | 46 | 28.625 | 56.144 | 5.209 | 1.00 | 35.80 | O |
| ATOM | 2008 | OE2 | GLU | B | 46 | 29.991 | 57.789 | 4.679 | 1.00 | 39.80 | O |
| ATOM | 2009 | N | TRP | B | 47 | 25.967 | 58.907 | 9.681 | 1.00 | 23.11 | N |
| ATOM | 2010 | CA | TRP | B | 47 | 24.539 | 59.121 | 9.771 | 1.00 | 22.46 | C |
| ATOM | 2011 | C | TRP | B | 47 | 23.947 | 58.961 | 8.385 | 1.00 | 22.14 | C |
| ATOM | 2012 | O | TRP | B | 47 | 24.535 | 59.456 | 7.393 | 1.00 | 18.58 | O |
| ATOM | 2013 | CB | TRP | B | 47 | 24.279 | 60.522 | 10.317 | 1.00 | 22.36 | C |
| ATOM | 2014 | CG | TRP | B | 47 | 22.826 | 60.953 | 10.284 | 1.00 | 20.78 | C |
| ATOM | 2015 | CD1 | TRP | B | 47 | 21.874 | 60.674 | 11.219 | 1.00 | 22.27 | C |
| ATOM | 2016 | CD2 | TRP | B | 47 | 22.181 | 61.723 | 9.272 | 1.00 | 18.69 | C |
| ATOM | 2017 | NE1 | TRP | B | 47 | 20.668 | 61.237 | 10.862 | 1.00 | 21.43 | N |
| ATOM | 2018 | CE2 | TRP | B | 47 | 20.821 | 61.877 | 9.664 | 1.00 | 22.63 | C |
| ATOM | 2019 | CE3 | TRP | B | 47 | 22.605 | 62.289 | 8.072 | 1.00 | 21.38 | C |
| ATOM | 2020 | CZ2 | TRP | B | 47 | 19.883 | 62.584 | 8.893 | 1.00 | 22.05 | C |
| ATOM | 2021 | CZ3 | TRP | B | 47 | 21.683 | 62.985 | 7.300 | 1.00 | 22.50 | C |
| ATOM | 2022 | CH2 | TRP | B | 47 | 20.325 | 63.133 | 7.723 | 1.00 | 22.56 | C |
| ATOM | 2023 | N | VAL | B | 48 | 22.846 | 58.206 | 8.255 | 1.00 | 19.81 | N |
| ATOM | 2024 | CA | VAL | B | 48 | 22.263 | 58.037 | 6.906 | 1.00 | 21.36 | C |
| ATOM | 2025 | C | VAL | B | 48 | 20.836 | 58.556 | 6.689 | 1.00 | 20.95 | C |
| ATOM | 2026 | O | VAL | B | 48 | 20.452 | 58.890 | 5.567 | 1.00 | 18.52 | O |
| ATOM | 2027 | CB | VAL | B | 48 | 22.464 | 56.596 | 6.341 | 1.00 | 23.63 | C |
| ATOM | 2028 | CG1 | VAL | B | 48 | 23.959 | 56.354 | 6.091 | 1.00 | 23.94 | C |
| ATOM | 2029 | CG2 | VAL | B | 48 | 21.948 | 55.525 | 7.252 | 1.00 | 23.50 | C |
| ATOM | 2030 | N | ALA | B | 49 | 20.036 | 58.591 | 7.750 | 1.00 | 19.45 | N |
| ATOM | 2031 | CA | ALA | B | 49 | 18.638 | 58.891 | 7.582 | 1.00 | 19.38 | C |
| ATOM | 2032 | C | ALA | B | 49 | 18.016 | 59.142 | 8.952 | 1.00 | 20.00 | C |
| ATOM | 2033 | O | ALA | B | 49 | 18.536 | 58.668 | 9.956 | 1.00 | 17.58 | O |
| ATOM | 2034 | CB | ALA | B | 49 | 17.949 | 57.742 | 6.873 | 1.00 | 20.32 | C |
| ATOM | 2035 | N | GLU | B | 50 | 16.921 | 59.901 | 8.971 | 1.00 | 21.82 | N |
| ATOM | 2036 | CA | GLU | B | 50 | 16.253 | 60.316 | 10.205 | 1.00 | 23.46 | C |
| ATOM | 2037 | C | GLU | B | 50 | 14.793 | 60.578 | 9.928 | 1.00 | 23.21 | C |
| ATOM | 2038 | O | GLU | B | 50 | 14.440 | 61.063 | 8.864 | 1.00 | 22.76 | O |
| ATOM | 2039 | CB | GLU | B | 50 | 16.877 | 61.603 | 10.711 | 1.00 | 24.72 | C |
| ATOM | 2040 | CG | GLU | B | 50 | 16.420 | 62.084 | 12.074 | 1.00 | 25.44 | C |
| ATOM | 2041 | CD | GLU | B | 50 | 17.474 | 63.005 | 12.722 | 1.00 | 26.49 | C |
| ATOM | 2042 | OE1 | GLU | B | 50 | 18.659 | 62.569 | 12.818 | 1.00 | 25.58 | O |
| ATOM | 2043 | OE2 | GLU | B | 50 | 17.118 | 64.139 | 13.141 | 1.00 | 25.80 | O |
| ATOM | 2044 | N | ILE | B | 51 | 13.950 | 60.273 | 10.901 | 1.00 | 23.59 | N |
| ATOM | 2045 | CA | ILE | B | 51 | 12.522 | 60.588 | 10.805 | 1.00 | 23.86 | C |
| ATOM | 2046 | C | ILE | B | 51 | 12.112 | 61.314 | 12.081 | 1.00 | 25.08 | C |
| ATOM | 2047 | O | ILE | B | 51 | 12.517 | 60.934 | 13.158 | 1.00 | 22.86 | O |
| ATOM | 2048 | CB | ILE | B | 51 | 11.667 | 59.332 | 10.546 | 1.00 | 24.25 | C |
| ATOM | 2049 | CG1 | ILE | B | 51 | 10.205 | 59.722 | 10.297 | 1.00 | 24.32 | C |
| ATOM | 2050 | CG2 | ILE | B | 51 | 11.787 | 58.331 | 11.696 | 1.00 | 24.49 | C |
| ATOM | 2051 | CD1 | ILE | B | 51 | 9.447 | 58.636 | 9.560 | 1.00 | 24.70 | C |
| ATOM | 2052 | N | ARG | B | 52 | 11.353 | 62.397 | 11.924 | 1.00 | 27.78 | N |
| ATOM | 2053 | CA | ARG | B | 52 | 10.958 | 63.267 | 13.019 | 1.00 | 30.32 | C |
| ATOM | 2054 | C | ARG | B | 52 | 9.451 | 63.255 | 13.132 | 1.00 | 31.49 | C |
| ATOM | 2055 | O | ARG | B | 52 | 8.903 | 62.183 | 13.254 | 1.00 | 34.31 | O |
| ATOM | 2056 | CB | ARG | B | 52 | 11.468 | 64.677 | 12.770 | 1.00 | 31.11 | C |
| ATOM | 2057 | CG | ARG | B | 52 | 12.979 | 64.695 | 12.645 | 1.00 | 32.54 | C |
| ATOM | 2058 | CD | ARG | B | 52 | 13.517 | 66.077 | 12.678 | 1.00 | 33.56 | C |
| ATOM | 2059 | NE | ARG | B | 52 | 14.979 | 66.077 | 12.697 | 1.00 | 33.12 | N |
| ATOM | 2060 | CZ | ARG | B | 52 | 15.719 | 67.184 | 12.634 | 1.00 | 33.33 | C |
| ATOM | 2061 | NH1 | ARG | B | 52 | 15.153 | 68.377 | 12.516 | 1.00 | 34.26 | N |
| ATOM | 2062 | NH2 | ARG | B | 52 | 17.036 | 67.101 | 12.678 | 1.00 | 34.09 | N |
| ATOM | 2063 | N | THR | B | 57 | 11.235 | 63.484 | 6.831 | 1.00 | 29.65 | N |
| ATOM | 2064 | CA | THR | B | 57 | 12.356 | 62.554 | 6.760 | 1.00 | 29.31 | C |
| ATOM | 2065 | C | THR | B | 57 | 13.563 | 63.242 | 6.143 | 1.00 | 29.41 | C |
| ATOM | 2066 | O | THR | B | 57 | 13.420 | 64.137 | 5.325 | 1.00 | 28.79 | O |
| ATOM | 2067 | CB | THR | B | 57 | 12.048 | 61.289 | 5.923 | 1.00 | 29.29 | C |
| ATOM | 2068 | OG1 | THR | B | 57 | 11.590 | 61.664 | 4.616 | 1.00 | 28.85 | O |
| ATOM | 2069 | CG2 | THR | B | 57 | 11.019 | 60.393 | 6.625 | 1.00 | 29.75 | C |
| ATOM | 2070 | N | TYR | B | 58 | 14.756 | 62.812 | 6.543 | 1.00 | 29.51 | N |

| ATOM | 2071 | CA | TYR | B | 58 | 15.992 | 63.393 | 6.019 | 1.00 | 29.12 | C |
|------|------|-----|-----|---|----|--------|--------|--------|------|-------|---|
| ATOM | 2072 | C | TYR | B | 58 | 16.919 | 62.245 | 5.711 | 1.00 | 27.13 | C |
| ATOM | 2073 | O | TYR | B | 58 | 16.845 | 61.230 | 6.371 | 1.00 | 24.45 | O |
| ATOM | 2074 | CB | TYR | B | 58 | 16.621 | 64.337 | 7.045 | 1.00 | 31.25 | C |
| ATOM | 2075 | CG | TYR | B | 58 | 15.683 | 65.414 | 7.508 | 1.00 | 31.92 | C |
| ATOM | 2076 | CD1 | TYR | B | 58 | 14.863 | 65.233 | 8.616 | 1.00 | 33.04 | C |
| ATOM | 2077 | CD2 | TYR | B | 58 | 15.606 | 66.611 | 6.824 | 1.00 | 34.32 | C |
| ATOM | 2078 | CE1 | TYR | B | 58 | 13.987 | 66.249 | 9.039 | 1.00 | 33.72 | C |
| ATOM | 2079 | CE2 | TYR | B | 58 | 14.723 | 67.622 | 7.218 | 1.00 | 34.16 | C |
| ATOM | 2080 | CZ | TYR | B | 58 | 13.931 | 67.437 | 8.328 | 1.00 | 34.43 | C |
| ATOM | 2081 | OH | TYR | B | 58 | 13.078 | 68.462 | 8.694 | 1.00 | 35.79 | O |
| ATOM | 2082 | N | TYR | B | 59 | 17.755 | 62.401 | 4.689 | 1.00 | 25.03 | N |
| ATOM | 2083 | CA | TYR | B | 59 | 18.606 | 61.336 | 4.194 | 1.00 | 26.19 | C |
| ATOM | 2084 | C | TYR | B | 59 | 19.954 | 61.936 | 3.857 | 1.00 | 26.62 | C |
| ATOM | 2085 | O | TYR | B | 59 | 20.012 | 63.051 | 3.330 | 1.00 | 24.75 | O |
| ATOM | 2086 | CB | TYR | B | 59 | 18.032 | 60.692 | 2.910 | 1.00 | 26.00 | C |
| ATOM | 2087 | CG | TYR | B | 59 | 16.700 | 60.031 | 3.125 | 1.00 | 26.16 | C |
| ATOM | 2088 | CD1 | TYR | B | 59 | 16.621 | 58.810 | 3.774 | 1.00 | 27.36 | C |
| ATOM | 2089 | CD2 | TYR | B | 59 | 15.522 | 60.638 | 2.722 | 1.00 | 27.60 | C |
| ATOM | 2090 | CE1 | TYR | B | 59 | 15.421 | 58.196 | 4.016 | 1.00 | 26.32 | C |
| ATOM | 2091 | CE2 | TYR | B | 59 | 14.289 | 60.014 | 2.948 | 1.00 | 28.10 | C |
| ATOM | 2092 | CZ | TYR | B | 59 | 14.258 | 58.789 | 3.599 | 1.00 | 26.47 | C |
| ATOM | 2093 | OH | TYR | B | 59 | 13.067 | 58.158 | 3.830 | 1.00 | 26.69 | O |
| ATOM | 2094 | N | ALA | B | 60 | 21.013 | 61.189 | 4.161 | 1.00 | 27.11 | N |
| ATOM | 2095 | CA | ALA | B | 60 | 22.348 | 61.562 | 3.745 | 1.00 | 28.53 | C |
| ATOM | 2096 | C | ALA | B | 60 | 22.426 | 61.594 | 2.226 | 1.00 | 29.64 | C |
| ATOM | 2097 | O | ALA | B | 60 | 21.761 | 60.804 | 1.536 | 1.00 | 26.29 | O |
| ATOM | 2098 | CB | ALA | B | 60 | 23.344 | 60.588 | 4.296 | 1.00 | 28.82 | C |
| ATOM | 2099 | N | GLU | B | 61 | 23.270 | 62.487 | 1.707 | 1.00 | 32.17 | N |
| ATOM | 2100 | CA | GLU | B | 61 | 23.440 | 62.605 | 0.259 | 1.00 | 34.67 | C |
| ATOM | 2101 | C | GLU | B | 61 | 23.802 | 61.287 | -0.390 | 1.00 | 33.99 | C |
| ATOM | 2102 | O | GLU | B | 61 | 23.310 | 60.972 | -1.450 | 1.00 | 34.77 | O |
| ATOM | 2103 | CB | GLU | B | 61 | 24.503 | 63.662 | -0.087 | 1.00 | 37.10 | C |
| ATOM | 2104 | CG | GLU | B | 61 | 24.095 | 65.081 | 0.308 | 1.00 | 40.88 | C |
| ATOM | 2105 | CD | GLU | B | 61 | 22.695 | 65.462 | -0.204 | 1.00 | 44.39 | C |
| ATOM | 2106 | OE1 | GLU | B | 61 | 21.932 | 66.090 | 0.579 | 1.00 | 49.10 | O |
| ATOM | 2107 | OE2 | GLU | B | 61 | 22.356 | 65.135 | -1.374 | 1.00 | 45.73 | O |
| ATOM | 2108 | N | SER | B | 62 | 24.625 | 60.496 | 0.273 | 1.00 | 34.63 | N |
| ATOM | 2109 | CA | SER | B | 62 | 25.125 | 59.261 | -0.311 | 1.00 | 35.51 | C |
| ATOM | 2110 | C | SER | B | 62 | 24.066 | 58.194 | -0.525 | 1.00 | 35.96 | C |
| ATOM | 2111 | O | SER | B | 62 | 24.306 | 57.288 | -1.315 | 1.00 | 35.77 | O |
| ATOM | 2112 | CB | SER | B | 62 | 26.236 | 58.679 | 0.560 | 1.00 | 36.34 | C |
| ATOM | 2113 | OG | SER | B | 62 | 25.778 | 58.494 | 1.884 | 1.00 | 37.45 | O |
| ATOM | 2114 | N | VAL | B | 63 | 22.915 | 58.282 | 0.159 | 1.00 | 35.78 | N |
| ATOM | 2115 | CA | VAL | B | 63 | 21.874 | 57.233 | 0.043 | 1.00 | 36.42 | C |
| ATOM | 2116 | C | VAL | B | 63 | 20.504 | 57.746 | -0.381 | 1.00 | 37.34 | C |
| ATOM | 2117 | O | VAL | B | 63 | 19.520 | 57.009 | -0.285 | 1.00 | 37.55 | O |
| ATOM | 2118 | CB | VAL | B | 63 | 21.707 | 56.422 | 1.363 | 1.00 | 36.21 | C |
| ATOM | 2119 | CG1 | VAL | B | 63 | 23.076 | 55.964 | 1.887 | 1.00 | 37.08 | C |
| ATOM | 2120 | CG2 | VAL | B | 63 | 20.948 | 57.240 | 2.419 | 1.00 | 35.58 | C |
| ATOM | 2121 | N | LYS | B | 64 | 20.431 | 58.991 | -0.846 | 1.00 | 38.55 | N |
| ATOM | 2122 | CA | LYS | B | 64 | 19.181 | 59.537 | -1.358 | 1.00 | 40.49 | C |
| ATOM | 2123 | C | LYS | B | 64 | 18.761 | 58.711 | -2.555 | 1.00 | 39.93 | C |
| ATOM | 2124 | O | LYS | B | 64 | 19.586 | 58.393 | -3.414 | 1.00 | 40.51 | O |
| ATOM | 2125 | CB | LYS | B | 64 | 19.315 | 61.014 | -1.762 | 1.00 | 41.13 | C |
| ATOM | 2126 | CG | LYS | B | 64 | 19.302 | 61.955 | -0.586 | 1.00 | 42.05 | C |
| ATOM | 2127 | CD | LYS | B | 64 | 19.178 | 63.405 | -1.016 | 1.00 | 43.31 | C |
| ATOM | 2128 | CE | LYS | B | 64 | 19.208 | 64.343 | 0.191 | 1.00 | 44.09 | C |
| ATOM | 2129 | NZ | LYS | B | 64 | 19.530 | 65.753 | -0.215 | 1.00 | 47.11 | N |
| ATOM | 2130 | N | GLY | B | 65 | 17.491 | 58.328 | -2.578 | 1.00 | 39.11 | N |
| ATOM | 2131 | CA | GLY | B | 65 | 16.956 | 57.528 | -3.662 | 1.00 | 38.70 | C |
| ATOM | 2132 | C | GLY | B | 65 | 17.143 | 56.037 | -3.489 | 1.00 | 37.30 | C |
| ATOM | 2133 | O | GLY | B | 65 | 16.529 | 55.253 | -4.228 | 1.00 | 40.12 | O |
| ATOM | 2134 | N | LYS | B | 66 | 17.988 | 55.628 | -2.550 | 1.00 | 34.07 | N |
| ATOM | 2135 | CA | LYS | B | 66 | 18.183 | 54.205 | -2.257 | 1.00 | 31.96 | C |

| | | | | | | | | | | | |
|---|---|---|---|---|---|---|---|---|---|---|---|
| ATOM | 2136 | C | LYS | B | 66 | 17.466 | 53.758 | -0.967 | 1.00 | 30.61 | C |
| ATOM | 2137 | O | LYS | B | 66 | 16.953 | 52.642 | -0.899 | 1.00 | 30.01 | O |
| ATOM | 2138 | CB | LYS | B | 66 | 19.668 | 53.890 | -2.135 | 1.00 | 32.62 | C |
| ATOM | 2139 | CG | LYS | B | 66 | 20.490 | 54.203 | -3.377 | 1.00 | 34.23 | C |
| ATOM | 2140 | CD | LYS | B | 66 | 20.759 | 52.939 | -4.173 | 1.00 | 34.23 | C |
| ATOM | 2141 | CE | LYS | B | 66 | 21.278 | 53.205 | -5.581 | 1.00 | 33.83 | C |
| ATOM | 2142 | NZ | LYS | B | 66 | 21.457 | 51.899 | -6.283 | 1.00 | 32.38 | N |
| ATOM | 2143 | N | PHE | B | 67 | 17.464 | 54.625 | 0.047 | 1.00 | 29.14 | N |
| ATOM | 2144 | CA | PHE | B | 67 | 16.892 | 54.312 | 1.366 | 1.00 | 28.70 | C |
| ATOM | 2145 | C | PHE | B | 67 | 15.582 | 55.085 | 1.568 | 1.00 | 28.10 | C |
| ATOM | 2146 | O | PHE | B | 67 | 15.465 | 56.217 | 1.128 | 1.00 | 25.58 | O |
| ATOM | 2147 | CB | PHE | B | 67 | 17.850 | 54.734 | 2.482 | 1.00 | 27.54 | C |
| ATOM | 2148 | CG | PHE | B | 67 | 19.117 | 53.908 | 2.590 | 1.00 | 26.90 | C |
| ATOM | 2149 | CD1 | PHE | B | 67 | 19.413 | 52.888 | 1.712 | 1.00 | 27.89 | C |
| ATOM | 2150 | CD2 | PHE | B | 67 | 20.050 | 54.218 | 3.565 | 1.00 | 28.54 | C |
| ATOM | 2151 | CE1 | PHE | B | 67 | 20.583 | 52.161 | 1.831 | 1.00 | 28.44 | C |
| ATOM | 2152 | CE2 | PHE | B | 67 | 21.220 | 53.487 | 3.685 | 1.00 | 28.38 | C |
| ATOM | 2153 | CZ | PHE | B | 67 | 21.482 | 52.464 | 2.805 | 1.00 | 28.03 | C |
| ATOM | 2154 | N | THR | B | 68 | 14.630 | 54.465 | 2.270 | 1.00 | 28.83 | N |
| ATOM | 2155 | CA | THR | B | 68 | 13.380 | 55.091 | 2.668 | 1.00 | 31.68 | C |
| ATOM | 2156 | C | THR | B | 68 | 13.164 | 54.808 | 4.160 | 1.00 | 34.13 | C |
| ATOM | 2157 | O | THR | B | 68 | 12.985 | 53.652 | 4.539 | 1.00 | 33.46 | O |
| ATOM | 2158 | CB | THR | B | 68 | 12.191 | 54.493 | 1.897 | 1.00 | 32.08 | C |
| ATOM | 2159 | OG1 | THR | B | 68 | 12.492 | 54.487 | 0.509 | 1.00 | 33.40 | O |
| ATOM | 2160 | CG2 | THR | B | 68 | 10.911 | 55.295 | 2.139 | 1.00 | 33.56 | C |
| ATOM | 2161 | N | ILE | B | 69 | 13.216 | 55.852 | 4.991 | 1.00 | 37.29 | N |
| ATOM | 2162 | CA | ILE | B | 69 | 13.078 | 55.709 | 6.442 | 1.00 | 40.20 | C |
| ATOM | 2163 | C | ILE | B | 69 | 11.647 | 56.009 | 6.747 | 1.00 | 41.57 | C |
| ATOM | 2164 | O | ILE | B | 69 | 11.032 | 56.848 | 6.091 | 1.00 | 42.34 | O |
| ATOM | 2165 | CB | ILE | B | 69 | 14.010 | 56.661 | 7.269 | 1.00 | 39.72 | C |
| ATOM | 2166 | CG1 | ILE | B | 69 | 14.075 | 56.194 | 8.721 | 1.00 | 41.09 | C |
| ATOM | 2167 | CG2 | ILE | B | 69 | 13.559 | 58.123 | 7.217 | 1.00 | 40.36 | C |
| ATOM | 2168 | CD1 | ILE | B | 69 | 14.800 | 57.152 | 9.662 | 1.00 | 41.27 | C |
| ATOM | 2169 | N | SER | B | 70 | 11.116 | 55.323 | 7.743 | 1.00 | 44.22 | N |
| ATOM | 2170 | CA | SER | B | 70 | 9.685 | 55.335 | 7.986 | 1.00 | 46.41 | C |
| ATOM | 2171 | C | SER | B | 70 | 9.411 | 54.822 | 9.391 | 1.00 | 48.75 | C |
| ATOM | 2172 | O | SER | B | 70 | 10.259 | 54.169 | 10.006 | 1.00 | 49.16 | O |
| ATOM | 2173 | CB | SER | B | 70 | 8.966 | 54.440 | 6.972 | 1.00 | 46.41 | C |
| ATOM | 2174 | OG | SER | B | 70 | 8.830 | 53.115 | 7.468 | 1.00 | 45.71 | O |
| ATOM | 2175 | N | ARG | B | 71 | 8.212 | 55.090 | 9.879 | 1.00 | 51.20 | N |
| ATOM | 2176 | CA | ARG | B | 71 | 7.864 | 54.729 | 11.231 | 1.00 | 53.10 | C |
| ATOM | 2177 | C | ARG | B | 71 | 6.400 | 54.392 | 11.362 | 1.00 | 54.84 | C |
| ATOM | 2178 | O | ARG | B | 71 | 5.546 | 54.985 | 10.695 | 1.00 | 54.27 | O |
| ATOM | 2179 | CB | ARG | B | 71 | 8.198 | 55.884 | 12.165 | 1.00 | 53.35 | C |
| ATOM | 2180 | CG | ARG | B | 71 | 7.516 | 57.189 | 11.772 | 1.00 | 53.65 | C |
| ATOM | 2181 | CD | ARG | B | 71 | 8.173 | 58.361 | 12.445 | 1.00 | 53.68 | C |
| ATOM | 2182 | NE | ARG | B | 71 | 7.741 | 58.492 | 13.824 | 1.00 | 53.18 | N |
| ATOM | 2183 | CZ | ARG | B | 71 | 8.378 | 59.198 | 14.746 | 1.00 | 54.38 | C |
| ATOM | 2184 | NH1 | ARG | B | 71 | 9.523 | 59.827 | 14.466 | 1.00 | 55.21 | N |
| ATOM | 2185 | NH2 | ARG | B | 71 | 7.873 | 59.250 | 15.970 | 1.00 | 55.50 | N |
| ATOM | 2186 | N | ASP | B | 72 | 6.144 | 53.419 | 12.230 | 1.00 | 57.52 | N |
| ATOM | 2187 | CA | ASP | B | 72 | 4.828 | 53.168 | 12.748 | 1.00 | 59.72 | C |
| ATOM | 2188 | C | ASP | B | 72 | 4.839 | 53.619 | 14.199 | 1.00 | 61.33 | C |
| ATOM | 2189 | O | ASP | B | 72 | 5.332 | 52.913 | 15.091 | 1.00 | 62.00 | O |
| ATOM | 2190 | CB | ASP | B | 72 | 4.448 | 51.690 | 12.628 | 1.00 | 60.50 | C |
| ATOM | 2191 | CG | ASP | B | 72 | 2.950 | 51.488 | 12.519 | 1.00 | 60.78 | C |
| ATOM | 2192 | OD1 | ASP | B | 72 | 2.195 | 52.274 | 13.140 | 1.00 | 60.70 | O |
| ATOM | 2193 | OD2 | ASP | B | 72 | 2.529 | 50.553 | 11.803 | 1.00 | 61.43 | O |
| ATOM | 2194 | N | ASP | B | 73 | 4.297 | 54.814 | 14.412 | 1.00 | 62.76 | N |
| ATOM | 2195 | CA | ASP | B | 73 | 4.198 | 55.421 | 15.738 | 1.00 | 63.82 | C |
| ATOM | 2196 | C | ASP | B | 73 | 3.315 | 54.586 | 16.675 | 1.00 | 64.23 | C |
| ATOM | 2197 | O | ASP | B | 73 | 3.444 | 54.677 | 17.897 | 1.00 | 64.57 | O |
| ATOM | 2198 | CB | ASP | B | 73 | 3.638 | 56.844 | 15.634 | 1.00 | 64.13 | C |
| ATOM | 2199 | CG | ASP | B | 73 | 4.137 | 57.586 | 14.402 | 1.00 | 64.41 | C |
| ATOM | 2200 | OD1 | ASP | B | 73 | 4.954 | 58.508 | 14.573 | 1.00 | 66.02 | O |

| ATOM | 2201 | OD2 | ASP | B | 73 | 3.715 | 57.244 | 13.270 | 1.00 | 63.34 | O |
|------|------|-----|-----|---|----|-------|--------|--------|------|-------|---|
| ATOM | 2202 | N | SER | B | 74 | 2.417 | 53.786 | 16.096 | 1.00 | 64.64 | N |
| ATOM | 2203 | CA | SER | B | 74 | 1.572 | 52.871 | 16.863 | 1.00 | 64.79 | C |
| ATOM | 2204 | C | SER | B | 74 | 2.417 | 51.834 | 17.561 | 1.00 | 64.27 | C |
| ATOM | 2205 | O | SER | B | 74 | 2.355 | 51.685 | 18.785 | 1.00 | 65.15 | O |
| ATOM | 2206 | CB | SER | B | 74 | 0.598 | 52.139 | 15.941 | 1.00 | 65.00 | C |
| ATOM | 2207 | OG | SER | B | 74 | 1.263 | 51.223 | 15.106 | 1.00 | 66.21 | O |
| ATOM | 2208 | N | LYS | B | 75 | 3.218 | 51.135 | 16.758 | 1.00 | 63.32 | N |
| ATOM | 2209 | CA | LYS | B | 75 | 3.964 | 49.968 | 17.212 | 1.00 | 62.18 | C |
| ATOM | 2210 | C | LYS | B | 75 | 5.288 | 50.390 | 17.834 | 1.00 | 60.79 | C |
| ATOM | 2211 | O | LYS | B | 75 | 6.023 | 49.548 | 18.327 | 1.00 | 60.89 | O |
| ATOM | 2212 | CB | LYS | B | 75 | 4.257 | 48.982 | 16.059 | 1.00 | 62.50 | C |
| ATOM | 2213 | CG | LYS | B | 75 | 3.250 | 48.907 | 14.894 | 1.00 | 62.95 | C |
| ATOM | 2214 | CD | LYS | B | 75 | 1.925 | 48.263 | 15.288 | 1.00 | 63.43 | C |
| ATOM | 2215 | CE | LYS | B | 75 | 0.898 | 48.287 | 14.145 | 1.00 | 63.32 | C |
| ATOM | 2216 | NZ | LYS | B | 75 | 1.451 | 47.881 | 12.823 | 1.00 | 63.35 | N |
| ATOM | 2217 | N | SER | B | 76 | 5.607 | 51.681 | 17.813 | 1.00 | 59.05 | N |
| ATOM | 2218 | CA | SER | B | 76 | 6.951 | 52.121 | 18.151 | 1.00 | 58.21 | C |
| ATOM | 2219 | C | SER | B | 76 | 7.955 | 51.329 | 17.307 | 1.00 | 57.09 | C |
| ATOM | 2220 | O | SER | B | 76 | 8.997 | 50.916 | 17.810 | 1.00 | 56.48 | O |
| ATOM | 2221 | CB | SER | B | 76 | 7.248 | 51.917 | 19.642 | 1.00 | 58.39 | C |
| ATOM | 2222 | OG | SER | B | 76 | 6.390 | 52.677 | 20.472 | 1.00 | 59.10 | O |
| ATOM | 2223 | N | ARG | B | 77 | 7.619 | 51.122 | 16.029 | 1.00 | 55.99 | N |
| ATOM | 2224 | CA | ARG | B | 77 | 8.418 | 50.298 | 15.119 | 1.00 | 55.48 | C |
| ATOM | 2225 | C | ARG | B | 77 | 8.799 | 51.041 | 13.839 | 1.00 | 53.38 | C |
| ATOM | 2226 | O | ARG | B | 77 | 8.022 | 51.094 | 12.885 | 1.00 | 53.13 | O |
| ATOM | 2227 | CB | ARG | B | 77 | 7.667 | 49.012 | 14.764 | 1.00 | 56.13 | C |
| ATOM | 2228 | CG | ARG | B | 77 | 8.520 | 48.016 | 13.990 | 1.00 | 57.43 | C |
| ATOM | 2229 | CD | ARG | B | 77 | 7.892 | 46.635 | 13.935 | 1.00 | 57.89 | C |
| ATOM | 2230 | NE | ARG | B | 77 | 8.582 | 45.774 | 12.970 | 1.00 | 59.55 | N |
| ATOM | 2231 | CZ | ARG | B | 77 | 8.244 | 44.515 | 12.688 | 1.00 | 59.77 | C |
| ATOM | 2232 | NH1 | ARG | B | 77 | 7.202 | 43.938 | 13.289 | 1.00 | 60.08 | N |
| ATOM | 2233 | NH2 | ARG | B | 77 | 8.953 | 43.829 | 11.796 | 1.00 | 58.85 | N |
| ATOM | 2234 | N | LEU | B | 78 | 10.009 | 51.594 | 13.836 | 1.00 | 51.30 | N |
| ATOM | 2235 | CA | LEU | B | 78 | 10.561 | 52.320 | 12.686 | 1.00 | 49.63 | C |
| ATOM | 2236 | C | LEU | B | 78 | 11.300 | 51.358 | 11.763 | 1.00 | 46.83 | C |
| ATOM | 2237 | O | LEU | B | 78 | 11.949 | 50.421 | 12.219 | 1.00 | 45.87 | O |
| ATOM | 2238 | CB | LEU | B | 78 | 11.493 | 53.439 | 13.176 | 1.00 | 50.48 | C |
| ATOM | 2239 | N | TYR | B | 79 | 11.199 | 51.613 | 10.461 | 1.00 | 44.02 | N |
| ATOM | 2240 | CA | TYR | B | 79 | 11.814 | 50.779 | 9.443 | 1.00 | 41.69 | C |
| ATOM | 2241 | C | TYR | B | 79 | 12.755 | 51.584 | 8.556 | 1.00 | 37.16 | C |
| ATOM | 2242 | O | TYR | B | 79 | 12.597 | 52.799 | 8.420 | 1.00 | 38.09 | O |
| ATOM | 2243 | CB | TYR | B | 79 | 10.749 | 50.190 | 8.550 | 1.00 | 44.26 | C |
| ATOM | 2244 | CG | TYR | B | 79 | 9.722 | 49.331 | 9.244 | 1.00 | 45.42 | C |
| ATOM | 2245 | CD1 | TYR | B | 79 | 9.885 | 47.957 | 9.316 | 1.00 | 46.19 | C |
| ATOM | 2246 | CD2 | TYR | B | 79 | 8.563 | 49.888 | 9.785 | 1.00 | 46.68 | C |
| ATOM | 2247 | CE1 | TYR | B | 79 | 8.940 | 47.153 | 9.929 | 1.00 | 46.69 | C |
| ATOM | 2248 | CE2 | TYR | B | 79 | 7.603 | 49.087 | 10.403 | 1.00 | 46.81 | C |
| ATOM | 2249 | CZ | TYR | B | 79 | 7.804 | 47.720 | 10.462 | 1.00 | 46.90 | C |
| ATOM | 2250 | OH | TYR | B | 79 | 6.880 | 46.902 | 11.077 | 1.00 | 47.79 | O |
| ATOM | 2251 | N | LEU | B | 80 | 13.747 | 50.905 | 7.985 | 1.00 | 30.65 | N |
| ATOM | 2252 | CA | LEU | B | 80 | 14.571 | 51.480 | 6.909 | 1.00 | 26.67 | C |
| ATOM | 2253 | C | LEU | B | 80 | 14.542 | 50.506 | 5.754 | 1.00 | 24.36 | C |
| ATOM | 2254 | O | LEU | B | 80 | 15.061 | 49.413 | 5.866 | 1.00 | 22.80 | O |
| ATOM | 2255 | CB | LEU | B | 80 | 16.010 | 51.715 | 7.359 | 1.00 | 26.07 | C |
| ATOM | 2256 | CG | LEU | B | 80 | 16.925 | 52.414 | 6.322 | 1.00 | 26.13 | C |
| ATOM | 2257 | CD1 | LEU | B | 80 | 16.465 | 53.854 | 6.100 | 1.00 | 25.20 | C |
| ATOM | 2258 | CD2 | LEU | B | 80 | 18.371 | 52.385 | 6.780 | 1.00 | 24.26 | C |
| ATOM | 2259 | N | GLN | B | 81 | 13.930 | 50.910 | 4.659 | 1.00 | 22.49 | N |
| ATOM | 2260 | CA | GLN | B | 81 | 13.855 | 50.092 | 3.452 | 1.00 | 22.64 | C |
| ATOM | 2261 | C | GLN | B | 81 | 15.079 | 50.452 | 2.621 | 1.00 | 22.37 | C |
| ATOM | 2262 | O | GLN | B | 81 | 15.253 | 51.597 | 2.296 | 1.00 | 20.30 | O |
| ATOM | 2263 | CB | GLN | B | 81 | 12.568 | 50.400 | 2.680 | 1.00 | 22.88 | C |
| ATOM | 2264 | CG | GLN | B | 81 | 12.387 | 49.656 | 1.373 | 1.00 | 23.89 | C |
| ATOM | 2265 | CD | GLN | B | 81 | 12.062 | 48.197 | 1.586 | 1.00 | 27.72 | C |

| ATOM | 2266 | OE1 | GLN | B | 81 | 11.034 | 47.890 | 2.213 | 1.00 | 27.30 | O |
|------|------|-----|-----|---|-----|--------|--------|--------|------|-------|---|
| ATOM | 2267 | NE2 | GLN | B | 81 | 12.910 | 47.283 | 1.062 | 1.00 | 21.87 | N |
| ATOM | 2268 | N | MET | B | 82 | 15.922 | 49.455 | 2.346 | 1.00 | 23.47 | N |
| ATOM | 2269 | CA | MET | B | 82 | 17.193 | 49.620 | 1.645 | 1.00 | 24.50 | C |
| ATOM | 2270 | C | MET | B | 82 | 17.116 | 48.871 | 0.350 | 1.00 | 22.15 | C |
| ATOM | 2271 | O | MET | B | 82 | 17.312 | 47.662 | 0.329 | 1.00 | 22.62 | O |
| ATOM | 2272 | CB | MET | B | 82 | 18.322 | 49.035 | 2.482 | 1.00 | 24.50 | C |
| ATOM | 2273 | CG | MET | B | 82 | 18.469 | 49.692 | 3.845 | 1.00 | 28.41 | C |
| ATOM | 2274 | SD | MET | B | 82 | 19.690 | 48.952 | 4.954 | 1.00 | 31.80 | S |
| ATOM | 2275 | CE | MET | B | 82 | 19.380 | 47.215 | 4.786 | 1.00 | 33.92 | C |
| ATOM | 2276 | N | ASN | B | 82A | 16.813 | 49.588 | -0.727 | 1.00 | 21.34 | N |
| ATOM | 2277 | CA | ASN | B | 82A | 16.673 | 48.999 | -2.041 | 1.00 | 21.59 | C |
| ATOM | 2278 | C | ASN | B | 82A | 17.878 | 49.344 | -2.954 | 1.00 | 23.04 | C |
| ATOM | 2279 | O | ASN | B | 82A | 18.563 | 50.345 | -2.766 | 1.00 | 23.12 | O |
| ATOM | 2280 | CB | ASN | B | 82A | 15.353 | 49.418 | -2.671 | 1.00 | 20.28 | C |
| ATOM | 2281 | CG | ASN | B | 82A | 14.142 | 48.831 | -1.926 | 1.00 | 22.08 | C |
| ATOM | 2282 | OD1 | ASN | B | 82A | 14.310 | 48.104 | -0.920 | 1.00 | 20.72 | O |
| ATOM | 2283 | ND2 | ASN | B | 82A | 12.942 | 49.097 | -2.436 | 1.00 | 21.64 | N |
| ATOM | 2284 | N | ASN | B | 82B | 18.132 | 48.444 | -3.888 | 1.00 | 23.89 | N |
| ATOM | 2285 | CA | ASN | B | 82B | 19.147 | 48.607 | -4.912 | 1.00 | 25.59 | C |
| ATOM | 2286 | C | ASN | B | 82B | 20.501 | 48.900 | -4.270 | 1.00 | 24.64 | C |
| ATOM | 2287 | O | ASN | B | 82B | 21.146 | 49.888 | -4.581 | 1.00 | 26.42 | O |
| ATOM | 2288 | CB | ASN | B | 82B | 18.682 | 49.665 | -5.915 | 1.00 | 26.55 | C |
| ATOM | 2289 | CG | ASN | B | 82B | 17.359 | 49.289 | -6.589 | 1.00 | 28.78 | C |
| ATOM | 2290 | OD1 | ASN | B | 82B | 17.202 | 48.181 | -7.114 | 1.00 | 33.87 | O |
| ATOM | 2291 | ND2 | ASN | B | 82B | 16.406 | 50.207 | -6.581 | 1.00 | 34.20 | N |
| ATOM | 2292 | N | LEU | B | 82C | 20.912 | 48.007 | -3.366 | 1.00 | 22.14 | N |
| ATOM | 2293 | CA | LEU | B | 82C | 22.036 | 48.254 | -2.487 | 1.00 | 22.95 | C |
| ATOM | 2294 | C | LEU | B | 82C | 23.363 | 48.152 | -3.222 | 1.00 | 23.01 | C |
| ATOM | 2295 | O | LEU | B | 82C | 23.489 | 47.400 | -4.173 | 1.00 | 22.67 | O |
| ATOM | 2296 | CB | LEU | B | 82C | 22.041 | 47.278 | -1.320 | 1.00 | 22.91 | C |
| ATOM | 2297 | CG | LEU | B | 82C | 20.927 | 47.437 | -0.289 | 1.00 | 23.69 | C |
| ATOM | 2298 | CD1 | LEU | B | 82C | 21.006 | 46.310 | 0.759 | 1.00 | 22.81 | C |
| ATOM | 2299 | CD2 | LEU | B | 82C | 20.965 | 48.805 | 0.367 | 1.00 | 25.77 | C |
| ATOM | 2300 | N | ARG | B | 83 | 24.338 | 48.922 | -2.761 | 1.00 | 24.29 | N |
| ATOM | 2301 | CA | ARG | B | 83 | 25.658 | 48.922 | -3.338 | 1.00 | 24.47 | C |
| ATOM | 2302 | C | ARG | B | 83 | 26.660 | 48.341 | -2.384 | 1.00 | 24.07 | C |
| ATOM | 2303 | O | ARG | B | 83 | 26.461 | 48.348 | -1.166 | 1.00 | 22.28 | O |
| ATOM | 2304 | CB | ARG | B | 83 | 26.046 | 50.357 | -3.685 | 1.00 | 27.06 | C |
| ATOM | 2305 | CG | ARG | B | 83 | 25.047 | 50.995 | -4.626 | 1.00 | 28.56 | C |
| ATOM | 2306 | CD | ARG | B | 83 | 24.943 | 52.477 | -4.418 | 1.00 | 32.72 | C |
| ATOM | 2307 | NE | ARG | B | 83 | 24.510 | 52.839 | -3.072 | 1.00 | 33.79 | N |
| ATOM | 2308 | CZ | ARG | B | 83 | 24.411 | 54.094 | -2.647 | 1.00 | 35.14 | C |
| ATOM | 2309 | NH1 | ARG | B | 83 | 24.682 | 55.105 | -3.473 | 1.00 | 36.02 | N |
| ATOM | 2310 | NH2 | ARG | B | 83 | 24.042 | 54.336 | -1.396 | 1.00 | 34.81 | N |
| ATOM | 2311 | N | THR | B | 84 | 27.789 | 47.883 | -2.917 | 1.00 | 25.07 | N |
| ATOM | 2312 | CA | THR | B | 84 | 28.807 | 47.248 | -2.066 | 1.00 | 25.77 | C |
| ATOM | 2313 | C | THR | B | 84 | 29.257 | 48.149 | -0.922 | 1.00 | 25.26 | C |
| ATOM | 2314 | O | THR | B | 84 | 29.519 | 47.678 | 0.195 | 1.00 | 24.99 | O |
| ATOM | 2315 | CB | THR | B | 84 | 30.012 | 46.736 | -2.892 | 1.00 | 26.88 | C |
| ATOM | 2316 | OG1 | THR | B | 84 | 30.540 | 47.796 | -3.720 | 1.00 | 27.69 | O |
| ATOM | 2317 | CG2 | THR | B | 84 | 29.553 | 45.609 | -3.777 | 1.00 | 25.94 | C |
| ATOM | 2318 | N | GLU | B | 85 | 29.247 | 49.453 | -1.168 | 1.00 | 26.05 | N |
| ATOM | 2319 | CA | GLU | B | 85 | 29.685 | 50.438 | -0.155 | 1.00 | 26.80 | C |
| ATOM | 2320 | C | GLU | B | 85 | 28.655 | 50.611 | 0.936 | 1.00 | 25.18 | C |
| ATOM | 2321 | O | GLU | B | 85 | 28.927 | 51.285 | 1.913 | 1.00 | 24.98 | O |
| ATOM | 2322 | CB | GLU | B | 85 | 29.941 | 51.817 | -0.739 | 1.00 | 27.71 | C |
| ATOM | 2323 | CG | GLU | B | 85 | 30.458 | 51.830 | -2.119 | 1.00 | 32.66 | C |
| ATOM | 2324 | CD | GLU | B | 85 | 29.371 | 51.743 | -3.162 | 1.00 | 33.06 | C |
| ATOM | 2325 | OE1 | GLU | B | 85 | 28.521 | 52.657 | -3.224 | 1.00 | 35.48 | O |
| ATOM | 2326 | OE2 | GLU | B | 85 | 29.388 | 50.759 | -3.925 | 1.00 | 35.09 | O |
| ATOM | 2327 | N | ASP | B | 86 | 27.468 | 50.032 | 0.763 | 1.00 | 24.43 | N |
| ATOM | 2328 | CA | ASP | B | 86 | 26.454 | 50.087 | 1.810 | 1.00 | 25.17 | C |
| ATOM | 2329 | C | ASP | B | 86 | 26.662 | 49.084 | 2.900 | 1.00 | 25.10 | C |
| ATOM | 2330 | O | ASP | B | 86 | 25.878 | 49.012 | 3.845 | 1.00 | 28.59 | O |

```
ATOM   2331  CB  ASP B  86    25.052  49.979   1.212  1.00 24.40        C
ATOM   2332  CG  ASP B  86    24.716  51.171   0.332  1.00 25.00        C
ATOM   2333  OD1 ASP B  86    25.032  52.325   0.727  1.00 24.34        O
ATOM   2334  OD2 ASP B  86    24.164  50.961  -0.776  1.00 24.39        O
ATOM   2335  N   THR B  87    27.729  48.315   2.794  1.00 25.20        N
ATOM   2336  CA  THR B  87    28.018  47.255   3.729  1.00 25.04        C
ATOM   2337  C   THR B  87    28.454  47.868   5.037  1.00 24.91        C
ATOM   2338  O   THR B  87    29.230  48.814   5.033  1.00 22.79        O
ATOM   2339  CB  THR B  87    29.106  46.361   3.170  1.00 26.09        C
ATOM   2340  OG1 THR B  87    28.600  45.689   2.015  1.00 25.44        O
ATOM   2341  CG2 THR B  87    29.568  45.338   4.184  1.00 27.90        C
ATOM   2342  N   GLY B  88    27.916  47.365   6.158  1.00 22.26        N
ATOM   2343  CA  GLY B  88    28.303  47.867   7.464  1.00 21.66        C
ATOM   2344  C   GLY B  88    27.358  47.407   8.554  1.00 22.07        C
ATOM   2345  O   GLY B  88    26.464  46.616   8.297  1.00 20.10        O
ATOM   2346  N   ILE B  89    27.579  47.889   9.770  1.00 22.50        N
ATOM   2347  CA  ILE B  89    26.670  47.615  10.879  1.00 23.29        C
ATOM   2348  C   ILE B  89    25.816  48.846  11.040  1.00 21.87        C
ATOM   2349  O   ILE B  89    26.326  49.940  11.154  1.00 23.55        O
ATOM   2350  CB  ILE B  89    27.431  47.302  12.177  1.00 25.54        C
ATOM   2351  CG1 ILE B  89    28.346  46.097  11.952  1.00 27.22        C
ATOM   2352  CG2 ILE B  89    26.441  46.991  13.342  1.00 24.48        C
ATOM   2353  CD1 ILE B  89    29.567  46.166  12.745  1.00 30.64        C
ATOM   2354  N   TYR B  90    24.510  48.649  11.020  1.00 21.38        N
ATOM   2355  CA  TYR B  90    23.531  49.721  11.028  1.00 21.50        C
ATOM   2356  C   TYR B  90    22.952  49.805  12.415  1.00 23.06        C
ATOM   2357  O   TYR B  90    22.496  48.769  12.947  1.00 22.37        O
ATOM   2358  CB  TYR B  90    22.427  49.418  10.022  1.00 21.46        C
ATOM   2359  CG  TYR B  90    22.832  49.763   8.626  1.00 21.89        C
ATOM   2360  CD1 TYR B  90    23.840  49.056   7.986  1.00 23.65        C
ATOM   2361  CD2 TYR B  90    22.241  50.827   7.946  1.00 21.01        C
ATOM   2362  CE1 TYR B  90    24.259  49.406   6.690  1.00 21.24        C
ATOM   2363  CE2 TYR B  90    22.637  51.160   6.654  1.00 20.99        C
ATOM   2364  CZ  TYR B  90    23.650  50.448   6.043  1.00 21.04        C
ATOM   2365  OH  TYR B  90    24.060  50.749   4.761  1.00 20.95        O
ATOM   2366  N   TYR B  91    23.014  51.005  13.000  1.00 22.70        N
ATOM   2367  CA  TYR B  91    22.466  51.265  14.317  1.00 23.27        C
ATOM   2368  C   TYR B  91    21.209  52.114  14.225  1.00 23.71        C
ATOM   2369  O   TYR B  91    21.140  53.091  13.476  1.00 21.97        O
ATOM   2370  CB  TYR B  91    23.501  51.971  15.220  1.00 22.42        C
ATOM   2371  CG  TYR B  91    24.723  51.152  15.552  1.00 22.33        C
ATOM   2372  CD1 TYR B  91    24.714  50.208  16.588  1.00 21.07        C
ATOM   2373  CD2 TYR B  91    25.913  51.323  14.827  1.00 21.66        C
ATOM   2374  CE1 TYR B  91    25.848  49.465  16.893  1.00 22.69        C
ATOM   2375  CE2 TYR B  91    27.023  50.581  15.114  1.00 23.42        C
ATOM   2376  CZ  TYR B  91    27.008  49.660  16.150  1.00 22.87        C
ATOM   2377  OH  TYR B  91    28.148  48.929  16.405  1.00 21.83        O
ATOM   2378  N   CYS B  92    20.183  51.722  14.972  1.00 23.69        N
ATOM   2379  CA  CYS B  92    19.122  52.634  15.305  1.00 25.72        C
ATOM   2380  C   CYS B  92    19.676  53.563  16.381  1.00 25.09        C
ATOM   2381  O   CYS B  92    20.309  53.126  17.361  1.00 25.35        O
ATOM   2382  CB  CYS B  92    17.865  51.883  15.795  1.00 29.76        C
ATOM   2383  SG  CYS B  92    16.705  51.451  14.440  1.00 38.70        S
ATOM   2384  N   PHE B  93    19.475  54.853  16.176  1.00 25.34        N
ATOM   2385  CA  PHE B  93    20.033  55.857  17.051  1.00 26.35        C
ATOM   2386  C   PHE B  93    18.945  56.907  17.264  1.00 26.92        C
ATOM   2387  O   PHE B  93    18.517  57.578  16.318  1.00 28.34        O
ATOM   2388  CB  PHE B  93    21.307  56.433  16.421  1.00 26.77        C
ATOM   2389  CG  PHE B  93    21.875  57.633  17.132  1.00 26.19        C
ATOM   2390  CD1 PHE B  93    22.104  57.609  18.491  1.00 26.99        C
ATOM   2391  CD2 PHE B  93    22.179  58.790  16.435  1.00 25.97        C
ATOM   2392  CE1 PHE B  93    22.636  58.714  19.138  1.00 26.95        C
ATOM   2393  CE2 PHE B  93    22.668  59.888  17.084  1.00 24.46        C
ATOM   2394  CZ  PHE B  93    22.918  59.842  18.437  1.00 26.22        C
ATOM   2395  N   LEU B  94    18.456  57.003  18.495  1.00 27.50        N
```

| ATOM | 2396 | CA | LEU | B | 94 | 17.535 | 58.075 | 18.880 | 1.00 | 29.26 | C |
|------|------|-----|-----|---|-----|--------|--------|--------|------|-------|---|
| ATOM | 2397 | C | LEU | B | 94 | 18.359 | 59.222 | 19.354 | 1.00 | 29.12 | C |
| ATOM | 2398 | O | LEU | B | 94 | 18.841 | 59.185 | 20.487 | 1.00 | 28.37 | O |
| ATOM | 2399 | CB | LEU | B | 94 | 16.677 | 57.696 | 20.074 | 1.00 | 30.71 | C |
| ATOM | 2400 | CG | LEU | B | 94 | 15.512 | 56.775 | 19.876 | 1.00 | 31.68 | C |
| ATOM | 2401 | CD1 | LEU | B | 94 | 16.085 | 55.390 | 19.740 | 1.00 | 34.38 | C |
| ATOM | 2402 | CD2 | LEU | B | 94 | 14.576 | 56.890 | 21.053 | 1.00 | 30.96 | C |
| ATOM | 2403 | N | PRO | B | 95 | 18.503 | 60.261 | 18.527 | 1.00 | 30.48 | N |
| ATOM | 2404 | CA | PRO | B | 95 | 19.445 | 61.292 | 18.908 | 1.00 | 31.49 | C |
| ATOM | 2405 | C | PRO | B | 95 | 18.965 | 61.985 | 20.172 | 1.00 | 32.37 | C |
| ATOM | 2406 | O | PRO | B | 95 | 17.781 | 62.315 | 20.252 | 1.00 | 32.61 | O |
| ATOM | 2407 | CB | PRO | B | 95 | 19.423 | 62.269 | 17.724 | 1.00 | 32.16 | C |
| ATOM | 2408 | CG | PRO | B | 95 | 18.670 | 61.580 | 16.613 | 1.00 | 30.86 | C |
| ATOM | 2409 | CD | PRO | B | 95 | 17.802 | 60.565 | 17.268 | 1.00 | 31.50 | C |
| ATOM | 2410 | N | MET | B | 96 | 19.823 | 62.208 | 21.167 | 1.00 | 33.09 | N |
| ATOM | 2411 | CA | MET | B | 96 | 21.237 | 61.877 | 21.175 | 1.00 | 32.86 | C |
| ATOM | 2412 | C | MET | B | 96 | 21.570 | 60.801 | 22.192 | 1.00 | 32.10 | C |
| ATOM | 2413 | O | MET | B | 96 | 22.737 | 60.473 | 22.362 | 1.00 | 32.36 | O |
| ATOM | 2414 | CB | MET | B | 96 | 22.038 | 63.130 | 21.537 | 1.00 | 32.75 | C |
| ATOM | 2415 | CG | MET | B | 96 | 21.902 | 64.255 | 20.554 | 1.00 | 33.27 | C |
| ATOM | 2416 | SD | MET | B | 96 | 22.659 | 63.897 | 18.977 | 1.00 | 33.75 | S |
| ATOM | 2417 | CE | MET | B | 96 | 24.382 | 64.148 | 19.385 | 1.00 | 32.96 | C |
| ATOM | 2418 | N | ASP | B | 101 | 20.564 | 60.238 | 22.856 | 1.00 | 32.70 | N |
| ATOM | 2419 | CA | ASP | B | 101 | 20.789 | 59.439 | 24.060 | 1.00 | 33.26 | C |
| ATOM | 2420 | C | ASP | B | 101 | 20.855 | 57.909 | 23.910 | 1.00 | 32.67 | C |
| ATOM | 2421 | O | ASP | B | 101 | 21.517 | 57.268 | 24.721 | 1.00 | 33.13 | O |
| ATOM | 2422 | CB | ASP | B | 101 | 19.710 | 59.739 | 25.109 | 1.00 | 35.57 | C |
| ATOM | 2423 | CG | ASP | B | 101 | 19.648 | 61.218 | 25.534 | 1.00 | 38.23 | C |
| ATOM | 2424 | OD1 | ASP | B | 101 | 20.529 | 62.043 | 25.196 | 1.00 | 41.94 | O |
| ATOM | 2425 | OD2 | ASP | B | 101 | 18.685 | 61.546 | 26.246 | 1.00 | 43.07 | O |
| ATOM | 2426 | N | TYR | B | 102 | 20.158 | 57.313 | 22.940 | 1.00 | 32.00 | N |
| ATOM | 2427 | CA | TYR | B | 102 | 19.983 | 55.837 | 22.904 | 1.00 | 32.73 | C |
| ATOM | 2428 | C | TYR | B | 102 | 20.423 | 55.189 | 21.597 | 1.00 | 31.44 | C |
| ATOM | 2429 | O | TYR | B | 102 | 20.085 | 55.676 | 20.524 | 1.00 | 30.15 | O |
| ATOM | 2430 | CB | TYR | B | 102 | 18.522 | 55.442 | 23.189 | 1.00 | 33.95 | C |
| ATOM | 2431 | CG | TYR | B | 102 | 17.970 | 56.109 | 24.412 | 1.00 | 35.11 | C |
| ATOM | 2432 | CD1 | TYR | B | 102 | 18.408 | 55.745 | 25.678 | 1.00 | 37.05 | C |
| ATOM | 2433 | CD2 | TYR | B | 102 | 17.024 | 57.109 | 24.311 | 1.00 | 36.81 | C |
| ATOM | 2434 | CE1 | TYR | B | 102 | 17.911 | 56.358 | 26.806 | 1.00 | 37.27 | C |
| ATOM | 2435 | CE2 | TYR | B | 102 | 16.520 | 57.727 | 25.426 | 1.00 | 35.73 | C |
| ATOM | 2436 | CZ | TYR | B | 102 | 16.969 | 57.353 | 26.670 | 1.00 | 36.91 | C |
| ATOM | 2437 | OH | TYR | B | 102 | 16.471 | 57.984 | 27.791 | 1.00 | 38.22 | O |
| ATOM | 2438 | N | TRP | B | 103 | 21.170 | 54.091 | 21.702 | 1.00 | 30.45 | N |
| ATOM | 2439 | CA | TRP | B | 103 | 21.578 | 53.329 | 20.525 | 1.00 | 30.61 | C |
| ATOM | 2440 | C | TRP | B | 103 | 21.034 | 51.907 | 20.621 | 1.00 | 32.06 | C |
| ATOM | 2441 | O | TRP | B | 103 | 20.906 | 51.370 | 21.728 | 1.00 | 33.57 | O |
| ATOM | 2442 | CB | TRP | B | 103 | 23.100 | 53.255 | 20.396 | 1.00 | 28.55 | C |
| ATOM | 2443 | CG | TRP | B | 103 | 23.810 | 54.557 | 20.132 | 1.00 | 28.19 | C |
| ATOM | 2444 | CD1 | TRP | B | 103 | 24.004 | 55.562 | 21.014 | 1.00 | 27.90 | C |
| ATOM | 2445 | CD2 | TRP | B | 103 | 24.494 | 54.944 | 18.926 | 1.00 | 27.74 | C |
| ATOM | 2446 | NE1 | TRP | B | 103 | 24.729 | 56.572 | 20.435 | 1.00 | 28.64 | N |
| ATOM | 2447 | CE2 | TRP | B | 103 | 25.045 | 56.224 | 19.151 | 1.00 | 27.24 | C |
| ATOM | 2448 | CE3 | TRP | B | 103 | 24.661 | 54.350 | 17.673 | 1.00 | 26.61 | C |
| ATOM | 2449 | CZ2 | TRP | B | 103 | 25.767 | 56.927 | 18.168 | 1.00 | 27.26 | C |
| ATOM | 2450 | CZ3 | TRP | B | 103 | 25.387 | 55.045 | 16.695 | 1.00 | 27.79 | C |
| ATOM | 2451 | CH2 | TRP | B | 103 | 25.923 | 56.322 | 16.953 | 1.00 | 26.90 | C |
| ATOM | 2452 | N | GLY | B | 104 | 20.732 | 51.302 | 19.468 | 1.00 | 32.27 | N |
| ATOM | 2453 | CA | GLY | B | 104 | 20.376 | 49.889 | 19.405 | 1.00 | 33.23 | C |
| ATOM | 2454 | C | GLY | B | 104 | 21.604 | 49.002 | 19.405 | 1.00 | 34.01 | C |
| ATOM | 2455 | O | GLY | B | 104 | 22.708 | 49.477 | 19.580 | 1.00 | 34.60 | O |
| ATOM | 2456 | N | GLN | B | 105 | 21.385 | 47.705 | 19.223 | 1.00 | 35.47 | N |
| ATOM | 2457 | CA | GLN | B | 105 | 22.441 | 46.668 | 19.197 | 1.00 | 37.22 | C |
| ATOM | 2458 | C | GLN | B | 105 | 23.275 | 46.662 | 17.925 | 1.00 | 37.05 | C |
| ATOM | 2459 | O | GLN | B | 105 | 24.455 | 46.327 | 17.937 | 1.00 | 37.87 | O |
| ATOM | 2460 | CB | GLN | B | 105 | 21.819 | 45.264 | 19.374 | 1.00 | 38.07 | C |

```
ATOM   2461  CG   GLN B 105   20.382  45.130  18.790  1.00  40.13        C
ATOM   2462  CD   GLN B 105   20.098  43.810  18.085  1.00  41.43        C
ATOM   2463  OE1  GLN B 105   20.837  43.403  17.179  1.00  45.72        O
ATOM   2464  NE2  GLN B 105   18.995  43.150  18.472  1.00  43.86        N
ATOM   2465  N    GLY B 106   22.657  46.996  16.811  1.00  37.34        N
ATOM   2466  CA   GLY B 106   23.355  46.957  15.539  1.00  37.27        C
ATOM   2467  C    GLY B 106   23.088  45.668  14.790  1.00  37.12        C
ATOM   2468  O    GLY B 106   23.316  44.571  15.305  1.00  38.18        O
ATOM   2469  N    THR B 107   22.580  45.817  13.573  1.00  36.52        N
ATOM   2470  CA   THR B 107   22.356  44.710  12.671  1.00  35.02        C
ATOM   2471  C    THR B 107   23.337  44.862  11.493  1.00  32.51        C
ATOM   2472  O    THR B 107   23.478  45.925  10.911  1.00  28.69        O
ATOM   2473  CB   THR B 107   20.858  44.618  12.199  1.00  35.42        C
ATOM   2474  OG1  THR B 107   20.662  43.420  11.441  1.00  38.41        O
ATOM   2475  CG2  THR B 107   20.446  45.770  11.318  1.00  36.35        C
ATOM   2476  N    SER B 108   23.999  43.759  11.180  1.00  30.69        N
ATOM   2477  CA   SER B 108   24.939  43.669  10.087  1.00  29.45        C
ATOM   2478  C    SER B 108   24.240  43.627   8.710  1.00  26.98        C
ATOM   2479  O    SER B 108   23.261  42.921   8.492  1.00  23.24        O
ATOM   2480  CB   SER B 108   25.813  42.433  10.306  1.00  30.27        C
ATOM   2481  OG   SER B 108   26.745  42.295   9.248  1.00  31.24        O
ATOM   2482  N    VAL B 109   24.763  44.418   7.787  1.00  25.82        N
ATOM   2483  CA   VAL B 109   24.299  44.446   6.411  1.00  25.20        C
ATOM   2484  C    VAL B 109   25.515  44.213   5.502  1.00  25.50        C
ATOM   2485  O    VAL B 109   26.505  44.986   5.532  1.00  22.99        O
ATOM   2486  CB   VAL B 109   23.684  45.823   6.068  1.00  27.46        C
ATOM   2487  CG1  VAL B 109   23.451  45.971   4.539  1.00  28.13        C
ATOM   2488  CG2  VAL B 109   22.405  46.077   6.849  1.00  25.47        C
ATOM   2489  N    THR B 110   25.465  43.140   4.715  1.00  23.52        N
ATOM   2490  CA   THR B 110   26.565  42.842   3.796  1.00  23.87        C
ATOM   2491  C    THR B 110   25.988  42.844   2.391  1.00  23.44        C
ATOM   2492  O    THR B 110   24.959  42.221   2.140  1.00  21.01        O
ATOM   2493  CB   THR B 110   27.230  41.495   4.108  1.00  24.79        C
ATOM   2494  OG1  THR B 110   27.531  41.413   5.508  1.00  27.21        O
ATOM   2495  CG2  THR B 110   28.509  41.336   3.305  1.00  25.24        C
ATOM   2496  N    VAL B 111   26.634  43.595   1.500  1.00  23.03        N
ATOM   2497  CA   VAL B 111   26.310  43.634   0.093  1.00  23.07        C
ATOM   2498  C    VAL B 111   27.572  43.187  -0.624  1.00  24.86        C
ATOM   2499  O    VAL B 111   28.611  43.824  -0.514  1.00  24.10        O
ATOM   2500  CB   VAL B 111   25.920  45.049  -0.377  1.00  22.22        C
ATOM   2501  CG1  VAL B 111   25.437  44.994  -1.811  1.00  21.85        C
ATOM   2502  CG2  VAL B 111   24.850  45.660   0.513  1.00  23.43        C
ATOM   2503  N    SER B 112   27.478  42.074  -1.329  1.00  25.77        N
ATOM   2504  CA   SER B 112   28.628  41.436  -1.904  .1.00  27.55        C
ATOM   2505  C    SER B 112   28.187  40.376  -2.900  1.00  28.72        C
ATOM   2506  O    SER B 112   27.217  39.672  -2.663  1.00  26.36        O
ATOM   2507  CB   SER B 112   29.429  40.762  -0.805  1.00  28.01        C
ATOM   2508  OG   SER B 112   30.391  39.903  -1.365  1.00  30.91        O
ATOM   2509  N    SER B 113   28.927  40.246  -3.999  1.00  29.70        N
ATOM   2510  CA   SER B 113   28.640  39.215  -5.008  1.00  32.24        C
ATOM   2511  C    SER B 113   28.890  37.777  -4.486  1.00  32.29        C
ATOM   2512  O    SER B 113   28.360  36.796  -5.039  1.00  32.66        O
ATOM   2513  CB   SER B 113   29.450  39.510  -6.284  1.00  33.12        C
ATOM   2514  OG   SER B 113   30.845  39.373  -6.020  1.00  35.97        O
ATOM   2515  N    ALA B 114   29.666  37.663  -3.408  1.00  32.62        N
ATOM   2516  CA   ALA B 114   29.923  36.380  -2.750  1.00  32.42        C
ATOM   2517  C    ALA B 114   28.662  35.863  -2.058  1.00  33.42        C
ATOM   2518  O    ALA B 114   27.946  36.628  -1.413  1.00  33.20        O
ATOM   2519  CB   ALA B 114   31.034  36.518  -1.749  1.00  32.43        C
ATOM   2520  N    LYS B 115   28.408  34.565  -2.198  1.00  34.10        N
ATOM   2521  CA   LYS B 115   27.189  33.928  -1.704  1.00  34.75        C
ATOM   2522  C    LYS B 115   27.434  33.345  -0.304  1.00  33.88        C
ATOM   2523  O    LYS B 115   28.581  33.171   0.126  1.00  32.48        O
ATOM   2524  CB   LYS B 115   26.692  32.851  -2.712  1.00  35.25        C
ATOM   2525  CG   LYS B 115   25.533  31.953  -2.194  1.00  36.20        C
```

| ATOM | 2526 | CD | LYS | B | 115 | 25.086 | 30.827 | -3.168 | 1.00 | 36.36 | C |
|------|------|-----|-----|---|-----|--------|--------|--------|------|-------|---|
| ATOM | 2527 | CE | LYS | B | 115 | 23.794 | 30.181 | -2.664 | 1.00 | 38.22 | C |
| ATOM | 2528 | NZ | LYS | B | 115 | 23.856 | 29.993 | -1.164 | 1.00 | 39.09 | N |
| ATOM | 2529 | N | THR | B | 116 | 26.342 | 33.094 | 0.416 | 1.00 | 32.78 | N |
| ATOM | 2530 | CA | THR | B | 116 | 26.401 | 32.512 | 1.738 | 1.00 | 33.04 | C |
| ATOM | 2531 | C | THR | B | 116 | 27.039 | 31.124 | 1.709 | 1.00 | 33.92 | C |
| ATOM | 2532 | O | THR | B | 116 | 26.650 | 30.269 | 0.915 | 1.00 | 34.08 | O |
| ATOM | 2533 | CB | THR | B | 116 | 24.986 | 32.375 | 2.359 | 1.00 | 32.95 | C |
| ATOM | 2534 | OG1 | THR | B | 116 | 24.362 | 33.668 | 2.451 | 1.00 | 32.14 | O |
| ATOM | 2535 | CG2 | THR | B | 116 | 25.058 | 31.752 | 3.736 | 1.00 | 32.88 | C |
| ATOM | 2536 | N | THR | B | 117 | 27.996 | 30.920 | 2.618 | 1.00 | 33.98 | N |
| ATOM | 2537 | CA | THR | B | 117 | 28.755 | 29.675 | 2.778 | 1.00 | 33.30 | C |
| ATOM | 2538 | C | THR | B | 117 | 28.830 | 29.370 | 4.280 | 1.00 | 33.61 | C |
| ATOM | 2539 | O | THR | B | 117 | 29.240 | 30.234 | 5.072 | 1.00 | 33.23 | O |
| ATOM | 2540 | CB | THR | B | 117 | 30.184 | 29.827 | 2.209 | 1.00 | 32.95 | C |
| ATOM | 2541 | OG1 | THR | B | 117 | 30.100 | 30.207 | 0.835 | 1.00 | 32.64 | O |
| ATOM | 2542 | CG2 | THR | B | 117 | 30.954 | 28.519 | 2.305 | 1.00 | 33.69 | C |
| ATOM | 2543 | N | PRO | B | 118 | 28.390 | 28.173 | 4.698 | 1.00 | 33.17 | N |
| ATOM | 2544 | CA | PRO | B | 118 | 28.477 | 27.862 | 6.126 | 1.00 | 32.15 | C |
| ATOM | 2545 | C | PRO | B | 118 | 29.904 | 27.529 | 6.612 | 1.00 | 31.30 | C |
| ATOM | 2546 | O | PRO | B | 118 | 30.730 | 27.043 | 5.827 | 1.00 | 31.72 | O |
| ATOM | 2547 | CB | PRO | B | 118 | 27.574 | 26.622 | 6.273 | 1.00 | 33.36 | C |
| ATOM | 2548 | CG | PRO | B | 118 | 26.854 | 26.463 | 4.946 | 1.00 | 33.89 | C |
| ATOM | 2549 | CD | PRO | B | 118 | 27.778 | 27.071 | 3.944 | 1.00 | 33.98 | C |
| ATOM | 2550 | N | PRO | B | 119 | 30.178 | 27.749 | 7.913 | 1.00 | 30.58 | N |
| ATOM | 2551 | CA | PRO | B | 119 | 31.491 | 27.433 | 8.485 | 1.00 | 30.38 | C |
| ATOM | 2552 | C | PRO | B | 119 | 31.719 | 25.941 | 8.688 | 1.00 | 30.47 | C |
| ATOM | 2553 | O | PRO | B | 119 | 30.753 | 25.192 | 8.914 | 1.00 | 29.74 | O |
| ATOM | 2554 | CB | PRO | B | 119 | 31.460 | 28.124 | 9.846 | 1.00 | 29.76 | C |
| ATOM | 2555 | CG | PRO | B | 119 | 30.006 | 28.273 | 10.188 | 1.00 | 30.01 | C |
| ATOM | 2556 | CD | PRO | B | 119 | 29.240 | 28.288 | 8.921 | 1.00 | 30.64 | C |
| ATOM | 2557 | N | SER | B | 120 | 32.986 | 25.533 | 8.623 | 1.00 | 28.80 | N |
| ATOM | 2558 | CA | SER | B | 120 | 33.417 | 24.251 | 9.170 | 1.00 | 29.26 | C |
| ATOM | 2559 | C | SER | B | 120 | 34.020 | 24.546 | 10.531 | 1.00 | 29.20 | C |
| ATOM | 2560 | O | SER | B | 120 | 34.874 | 25.426 | 10.653 | 1.00 | 29.35 | O |
| ATOM | 2561 | CB | SER | B | 120 | 34.471 | 23.602 | 8.277 | 1.00 | 29.19 | C |
| ATOM | 2562 | OG | SER | B | 120 | 34.131 | 23.715 | 6.906 | 1.00 | 29.95 | O |
| ATOM | 2563 | N | VAL | B | 121 | 33.593 | 23.802 | 11.550 | 1.00 | 28.65 | N |
| ATOM | 2564 | CA | VAL | B | 121 | 34.036 | 24.035 | 12.927 | 1.00 | 28.63 | C |
| ATOM | 2565 | C | VAL | B | 121 | 34.939 | 22.900 | 13.353 | 1.00 | 28.72 | C |
| ATOM | 2566 | O | VAL | B | 121 | 34.561 | 21.717 | 13.288 | 1.00 | 28.36 | O |
| ATOM | 2567 | CB | VAL | B | 121 | 32.823 | 24.147 | 13.900 | 1.00 | 29.68 | C |
| ATOM | 2568 | CG1 | VAL | B | 121 | 33.274 | 24.511 | 15.299 | 1.00 | 29.46 | C |
| ATOM | 2569 | CG2 | VAL | B | 121 | 31.846 | 25.178 | 13.376 | 1.00 | 29.74 | C |
| ATOM | 2570 | N | TYR | B | 122 | 36.142 | 23.256 | 13.790 | 1.00 | 26.73 | N |
| ATOM | 2571 | CA | TYR | B | 122 | 37.120 | 22.273 | 14.151 | 1.00 | 25.62 | C |
| ATOM | 2572 | C | TYR | B | 122 | 37.635 | 22.507 | 15.550 | 1.00 | 26.13 | C |
| ATOM | 2573 | O | TYR | B | 122 | 37.954 | 23.631 | 15.903 | 1.00 | 25.34 | O |
| ATOM | 2574 | CB | TYR | B | 122 | 38.293 | 22.322 | 13.189 | 1.00 | 25.47 | C |
| ATOM | 2575 | CG | TYR | B | 122 | 37.894 | 22.163 | 11.759 | 1.00 | 24.82 | C |
| ATOM | 2576 | CD1 | TYR | B | 122 | 37.043 | 21.131 | 11.362 | 1.00 | 25.26 | C |
| ATOM | 2577 | CD2 | TYR | B | 122 | 38.414 | 23.009 | 10.778 | 1.00 | 26.25 | C |
| ATOM | 2578 | CE1 | TYR | B | 122 | 36.694 | 20.955 | 10.052 | 1.00 | 25.48 | C |
| ATOM | 2579 | CE2 | TYR | B | 122 | 38.080 | 22.842 | 9.448 | 1.00 | 24.22 | C |
| ATOM | 2580 | CZ | TYR | B | 122 | 37.232 | 21.821 | ·9.093 | 1.00 | 26.33 | C |
| ATOM | 2581 | OH | TYR | B | 122 | 36.909 | 21.645 | 7.793 | 1.00 | 26.05 | O |
| ATOM | 2582 | N | PRO | B | 123 | 37.741 | 21.423 | 16.344 | 1.00 | 26.98 | N |
| ATOM | 2583 | CA | PRO | B | 123 | 38.234 | 21.513 | 17.709 | 1.00 | 26.02 | C |
| ATOM | 2584 | C | PRO | B | 123 | 39.744 | 21.521 | 17.712 | 1.00 | 24.59 | C |
| ATOM | 2585 | O | PRO | B | 123 | 40.364 | 20.818 | 16.946 | 1.00 | 24.28 | O |
| ATOM | 2586 | CB | PRO | B | 123 | 37.700 | 20.234 | 18.352 | 1.00 | 26.67 | C |
| ATOM | 2587 | CG | PRO | B | 123 | 37.647 | 19.256 | 17.266 | 1.00 | 27.03 | C |
| ATOM | 2588 | CD | PRO | B | 123 | 37.387 | 20.026 | 15.989 | 1.00 | 27.28 | C |
| ATOM | 2589 | N | LEU | B | 124 | 40.317 | 22.343 | 18.569 | 1.00 | 23.78 | N |
| ATOM | 2590 | CA | LEU | B | 124 | 41.734 | 22.383 | 18.784 | 1.00 | 23.17 | C |

```
ATOM   2591  C    LEU B 124   42.034  21.850  20.171  1.00 23.50        C
ATOM   2592  O    LEU B 124   41.713  22.487  21.149  1.00 20.90        O
ATOM   2593  CB   LEU B 124   42.229  23.818  18.697  1.00 22.73        C
ATOM   2594  CG   LEU B 124   41.655  24.578  17.508  1.00 23.77        C
ATOM   2595  CD1  LEU B 124   42.137  26.024  17.473  1.00 25.09        C
ATOM   2596  CD2  LEU B 124   42.025  23.831  16.276  1.00 25.17        C
ATOM   2597  N    ALA B 125   42.711  20.712  20.207  1.00 25.46        N
ATOM   2598  CA   ALA B 125   43.142  21.419  21.419  1.00 26.73        C
ATOM   2599  C    ALA B 125   44.655  19.918  21.350  1.00 27.83        C
ATOM   2600  O    ALA B 125   45.205  19.791  20.257  1.00 29.61        O
ATOM   2601  CB   ALA B 125   42.498  18.686  21.481  1.00 26.21        C
ATOM   2602  N    PRO B 126   45.342  19.972  22.501  1.00 29.41        N
ATOM   2603  CA   PRO B 126   46.796  19.763  22.547  1.00 30.79        C
ATOM   2604  C    PRO B 126   47.247  18.411  21.985  1.00 32.58        C
ATOM   2605  O    PRO B 126   46.491  17.429  22.097  1.00 34.29        O
ATOM   2606  CB   PRO B 126   47.118  19.852  24.039  1.00 30.12        C
ATOM   2607  CG   PRO B 126   45.807  19.680  24.732  1.00 30.39        C
ATOM   2608  CD   PRO B 126   44.792  20.256  23.837  1.00 29.04        C
ATOM   2609  N    SER B 136   48.960  22.681  34.521  1.00 41.15        N
ATOM   2610  CA   SER B 136   48.282  23.546  35.482  1.00 40.70        C
ATOM   2611  C    SER B 136   47.011  24.122  34.872  1.00 40.05        C
ATOM   2612  O    SER B 136   45.899  23.797  35.316  1.00 38.17        O
ATOM   2613  CB   SER B 136   49.224  24.667  35.941  1.00 41.62        C
ATOM   2614  OG   SER B 136   48.551  25.690  36.677  1.00 42.73        O
ATOM   2615  N    MET B 137   47.203  24.987  33.870  1.00 38.55        N
ATOM   2616  CA   MET B 137   46.131  25.542  33.054  1.00 38.56        C
ATOM   2617  C    MET B 137   46.257  24.961  31.656  1.00 36.85        C
ATOM   2618  O    MET B 137   47.369  24.887  31.126  1.00 37.94        O
ATOM   2619  CB   MET B 137   46.255  27.067  32.947  1.00 39.48        C
ATOM   2620  CG   MET B 137   46.103  27.827  34.243  1.00 40.95        C
ATOM   2621  SD   MET B 137   44.447  27.661  34.905  1.00 44.49        S
ATOM   2622  CE   MET B 137   43.485  28.668  33.798  1.00 42.95        C
ATOM   2623  N    VAL B 138   45.135  24.564  31.057  1.00 34.43        N
ATOM   2624  CA   VAL B 138   45.142  24.073  29.675  1.00 32.50        C
ATOM   2625  C    VAL B 138   44.416  25.058  28.777  1.00 29.83        C
ATOM   2626  O    VAL B 138   43.408  25.617  29.156  1.00 28.94        O
ATOM   2627  CB   VAL B 138   44.556  22.632  29.555  1.00 32.76        C
ATOM   2628  CG1  VAL B 138   43.032  22.609  29.721  1.00 32.93        C
ATOM   2629  CG2  VAL B 138   44.942  22.017  28.248  1.00 33.22        C
ATOM   2630  N    THR B 139   44.969  25.309  27.598  1.00 27.15        N
ATOM   2631  CA   THR B 139   44.274  26.088  26.597  1.00 25.11        C
ATOM   2632  C    THR B 139   43.755  25.164  25.505  1.00 23.74        C
ATOM   2633  O    THR B 139   44.441  24.247  25.043  1.00 23.32        O
ATOM   2634  CB   THR B 139   45.165  27.209  26.063  1.00 24.88        C
ATOM   2635  OG1  THR B 139   45.384  28.141  27.127  1.00 25.15        O
ATOM   2636  CG2  THR B 139   44.514  27.948  24.901  1.00 23.38        C
ATOM   2637  N    LEU B 140   42.513  25.399  25.116  1.00 23.05        N
ATOM   2638  CA   LEU B 140   41.897  24.654  24.032  1.00 22.73        C
ATOM   2639  C    LEU B 140   41.354  25.692  23.088  1.00 22.16        C
ATOM   2640  O    LEU B 140   41.389  26.886  23.406  1.00 21.39        O
ATOM   2641  CB   LEU B 140   40.750  23.795  24.578  1.00 24.49        C
ATOM   2642  CG   LEU B 140   41.073  22.960  25.829  1.00 25.50        C
ATOM   2643  CD1  LEU B 140   39.781  22.479  26.515  1.00 27.69        C
ATOM   2644  CD2  LEU B 140   41.962  21.790  25.482  1.00 25.99        C
ATOM   2645  N    GLY B 141   40.802  25.261  21.957  1.00 22.02        N
ATOM   2646  CA   GLY B 141   40.153  26.206  21.069  1.00 22.38        C
ATOM   2647  C    GLY B 141   39.273  25.627  19.995  1.00 23.01        C
ATOM   2648  O    GLY B 141   39.111  24.414  19.889  1.00 21.39        O
ATOM   2649  N    CYS B 142   38.668  26.523  19.224  1.00 24.09        N
ATOM   2650  CA   CYS B 142   37.844  26.163  18.084  1.00 26.54        C
ATOM   2651  C    CYS B 142   38.235  26.999  16.876  1.00 25.44        C
ATOM   2652  O    CYS B 142   38.521  28.183  17.012  1.00 24.79        O
ATOM   2653  CB   CYS B 142   36.356  26.380  18.405  1.00 29.27        C
ATOM   2654  SG   CYS B 142   35.694  24.845  19.046  1.00 39.59        S
ATOM   2655  N    LEU B 143   38.249  26.356  15.714  1.00 25.05        N
```

| ATOM | 2656 | CA | LEU | B | 143 | 38.571 | 27.009 | 14.459 | 1.00 | 25.41 | C |
|------|------|-----|-----|---|-----|--------|--------|--------|------|-------|---|
| ATOM | 2657 | C | LEU | B | 143 | 37.305 | 27.007 | 13.606 | 1.00 | 24.40 | C |
| ATOM | 2658 | O | LEU | B | 143 | 36.755 | 25.959 | 13.292 | 1.00 | 24.98 | O |
| ATOM | 2659 | CB | LEU | B | 143 | 39.737 | 26.262 | 13.816 | 1.00 | 25.82 | C |
| ATOM | 2660 | CG | LEU | B | 143 | 40.292 | 26.814 | 12.524 | 1.00 | 27.66 | C |
| ATOM | 2661 | CD1 | LEU | B | 143 | 40.910 | 28.199 | 12.677 | 1.00 | 28.90 | C |
| ATOM | 2662 | CD2 | LEU | B | 143 | 41.312 | 25.827 | 12.009 | 1.00 | 27.38 | C |
| ATOM | 2663 | N | VAL | B | 144 | 36.815 | 28.199 | 13.284 | 1.00 | 23.70 | N |
| ATOM | 2664 | CA | VAL | B | 144 | 35.614 | 28.395 | 12.486 | 1.00 | 22.90 | C |
| ATOM | 2665 | C | VAL | B | 144 | 36.038 | 28.922 | 11.102 | 1.00 | 23.47 | C |
| ATOM | 2666 | O | VAL | B | 144 | 36.367 | 30.096 | 10.950 | 1.00 | 23.29 | O |
| ATOM | 2667 | CB | VAL | B | 144 | 34.714 | 29.382 | 13.188 | 1.00 | 23.75 | C |
| ATOM | 2668 | CG1 | VAL | B | 144 | 33.384 | 29.490 | 12.475 | 1.00 | 25.80 | C |
| ATOM | 2669 | CG2 | VAL | B | 144 | 34.547 | 28.977 | 14.668 | 1.00 | 23.56 | C |
| ATOM | 2670 | N | LYS | B | 145 | 36.079 | 28.034 | 10.125 | 1.00 | 23.12 | N |
| ATOM | 2671 | CA | LYS | B | 145 | 36.824 | 28.287 | 8.902 | 1.00 | 26.06 | C |
| ATOM | 2672 | C | LYS | B | 145 | 35.897 | 28.316 | 7.674 | 1.00 | 25.55 | C |
| ATOM | 2673 | O | LYS | B | 145 | 34.925 | 27.569 | 7.578 | 1.00 | 24.72 | O |
| ATOM | 2674 | CB | LYS | B | 145 | 37.977 | 27.261 | 8.781 | 1.00 | 26.59 | C |
| ATOM | 2675 | CG | LYS | B | 145 | 38.882 | 27.461 | 7.580 | 1.00 | 28.90 | C |
| ATOM | 2676 | CD | LYS | B | 145 | 40.197 | 26.705 | 7.657 | 1.00 | 28.42 | C |
| ATOM | 2677 | CE | LYS | B | 145 | 41.125 | 27.219 | 6.548 | 1.00 | 29.19 | C |
| ATOM | 2678 | NZ | LYS | B | 145 | 40.517 | 26.903 | 5.200 | 1.00 | 32.20 | N |
| ATOM | 2679 | N | GLY | B | 146 | 36.170 | 29.260 | 6.776 | 1.00 | 26.70 | N |
| ATOM | 2680 | CA | GLY | B | 146 | 35.596 | 29.261 | 5.438 | 1.00 | 26.03 | C |
| ATOM | 2681 | C | GLY | B | 146 | 34.124 | 29.556 | 5.362 | 1.00 | 26.00 | C |
| ATOM | 2682 | O | GLY | B | 146 | 33.392 | 28.838 | 4.702 | 1.00 | 26.41 | O |
| ATOM | 2683 | N | TYR | B | 147 | 33.702 | 30.645 | 6.001 | 1.00 | 26.03 | N |
| ATOM | 2684 | CA | TYR | B | 147 | 32.299 | 31.069 | 5.968 | 1.00 | 25.90 | C |
| ATOM | 2685 | C | TYR | B | 147 | 32.110 | 32.475 | 5.388 | 1.00 | 25.86 | C |
| ATOM | 2686 | O | TYR | B | 147 | 33.044 | 33.283 | 5.277 | 1.00 | 24.63 | O |
| ATOM | 2687 | CB | TYR | B | 147 | 31.671 | 31.004 | 7.373 | 1.00 | 25.77 | C |
| ATOM | 2688 | CG | TYR | B | 147 | 32.282 | 31.959 | 8.366 | 1.00 | 24.90 | C |
| ATOM | 2689 | CD1 | TYR | B | 147 | 33.409 | 31.599 | 9.105 | 1.00 | 24.46 | C |
| ATOM | 2690 | CD2 | TYR | B | 147 | 31.746 | 33.221 | 8.558 | 1.00 | 24.92 | C |
| ATOM | 2691 | CE1 | TYR | B | 147 | 33.980 | 32.469 | 9.995 | 1.00 | 24.62 | C |
| ATOM | 2692 | CE2 | TYR | B | 147 | 32.303 | 34.101 | 9.464 | 1.00 | 24.35 | C |
| ATOM | 2693 | CZ | TYR | B | 147 | 33.424 | 33.725 | 10.163 | 1.00 | 24.79 | C |
| ATOM | 2694 | OH | TYR | B | 147 | 33.970 | 34.613 | 11.035 | 1.00 | 25.31 | O |
| ATOM | 2695 | N | PHE | B | 148 | 30.868 | 32.749 | 5.021 | 1.00 | 26.67 | N |
| ATOM | 2696 | CA | PHE | B | 148 | 30.477 | 34.049 | 4.525 | 1.00 | 26.94 | C |
| ATOM | 2697 | C | PHE | B | 148 | 28.970 | 34.167 | 4.714 | 1.00 | 27.10 | C |
| ATOM | 2698 | O | PHE | B | 148 | 28.264 | 33.193 | 4.562 | 1.00 | 26.03 | O |
| ATOM | 2699 | CB | PHE | B | 148 | 30.868 | 34.237 | 3.040 | 1.00 | 27.20 | C |
| ATOM | 2700 | CG | PHE | B | 148 | 30.887 | 35.669 | 2.621 | 1.00 | 26.45 | C |
| ATOM | 2701 | CD1 | PHE | B | 148 | 31.999 | 36.441 | 2.836 | 1.00 | 27.59 | C |
| ATOM | 2702 | CD2 | PHE | B | 148 | 29.760 | 36.262 | 2.095 | 1.00 | 26.46 | C |
| ATOM | 2703 | CE1 | PHE | B | 148 | 32.014 | 37.774 | 2.499 | 1.00 | 29.05 | C |
| ATOM | 2704 | CE2 | PHE | B | 148 | 29.753 | 37.596 | 1.769 | 1.00 | 27.76 | C |
| ATOM | 2705 | CZ | PHE | B | 148 | 30.892 | 38.358 | 1.963 | 1.00 | 28.69 | C |
| ATOM | 2706 | N | PRO | B | 149 | 28.476 | 35.344 | 5.112 | 1.00 | 28.06 | N |
| ATOM | 2707 | CA | PRO | B | 149 | 29.165 | 36.565 | 5.516 | 1.00 | 29.45 | C |
| ATOM | 2708 | C | PRO | B | 149 | 29.405 | 36.531 | 7.029 | 1.00 | 30.95 | C |
| ATOM | 2709 | O | PRO | B | 149 | 29.014 | 35.578 | 7.688 | 1.00 | 30.53 | O |
| ATOM | 2710 | CB | PRO | B | 149 | 28.141 | 37.645 | 5.174 | 1.00 | 28.23 | C |
| ATOM | 2711 | CG | PRO | B | 149 | 26.839 | 36.976 | 5.515 | 1.00 | 28.27 | C |
| ATOM | 2712 | CD | PRO | B | 149 | 27.015 | 35.513 | 5.193 | 1.00 | 27.96 | C |
| ATOM | 2713 | N | GLU | B | 150 | 30.052 | 37.559 | 7.560 | 1.00 | 33.11 | N |
| ATOM | 2714 | CA | GLU | B | 150 | 30.097 | 37.753 | 9.001 | 1.00 | 34.70 | C |
| ATOM | 2715 | C | GLU | B | 150 | 28.682 | 38.173 | 9.404 | 1.00 | 34.91 | C |
| ATOM | 2716 | O | GLU | B | 150 | 27.922 | 38.661 | 8.561 | 1.00 | 35.37 | O |
| ATOM | 2717 | CB | GLU | B | 150 | 31.115 | 38.833 | 9.379 | 1.00 | 35.55 | C |
| ATOM | 2718 | CG | GLU | B | 150 | 32.547 | 38.324 | 9.529 | 1.00 | 37.71 | C |
| ATOM | 2719 | CD | GLU | B | 150 | 32.975 | 38.204 | 10.994 | 1.00 | 39.79 | C |
| ATOM | 2720 | OE1 | GLU | B | 150 | 33.599 | 39.176 | 11.485 | 1.00 | 42.33 | O |

| ATOM | 2721 | OE2 | GLU | B | 150 | 32.682 | 37.167 | 11.662 | 1.00 | 41.32 | O |
|------|------|-----|-----|---|-----|--------|--------|--------|------|-------|---|
| ATOM | 2722 | N   | PRO | B | 151 | 28.322 | 37.998 | 10.688 | 1.00 | 34.50 | N |
| ATOM | 2723 | CA  | PRO | B | 151 | 29.096 | 37.435 | 11.779 | 1.00 | 33.76 | C |
| ATOM | 2724 | C   | PRO | B | 151 | 28.779 | 35.969 | 12.127 | 1.00 | 33.40 | C |
| ATOM | 2725 | O   | PRO | B | 151 | 27.731 | 35.435 | 11.768 | 1.00 | 33.22 | O |
| ATOM | 2726 | CB  | PRO | B | 151 | 28.646 | 38.305 | 12.951 | 1.00 | 34.80 | C |
| ATOM | 2727 | CG  | PRO | B | 151 | 27.179 | 38.519 | 12.680 | 1.00 | 35.10 | C |
| ATOM | 2728 | CD  | PRO | B | 151 | 27.003 | 38.459 | 11.168 | 1.00 | 34.58 | C |
| ATOM | 2729 | N   | VAL | B | 152 | 29.694 | 35.343 | 12.849 | 1.00 | 33.35 | N |
| ATOM | 2730 | CA  | VAL | B | 152 | 29.392 | 34.150 | 13.635 | 1.00 | 33.96 | C |
| ATOM | 2731 | C   | VAL | B | 152 | 29.546 | 34.532 | 15.087 | 1.00 | 33.19 | C |
| ATOM | 2732 | O   | VAL | B | 152 | 30.261 | 35.476 | 15.405 | 1.00 | 31.72 | O |
| ATOM | 2733 | CB  | VAL | B | 152 | 30.336 | 32.938 | 13.366 | 1.00 | 34.08 | C |
| ATOM | 2734 | CG1 | VAL | B | 152 | 30.290 | 32.516 | 11.912 | 1.00 | 35.25 | C |
| ATOM | 2735 | CG2 | VAL | B | 152 | 31.760 | 33.226 | 13.811 | 1.00 | 36.16 | C |
| ATOM | 2736 | N   | THR | B | 153 | 28.878 | 33.786 | 15.964 | 1.00 | 32.99 | N |
| ATOM | 2737 | CA  | THR | B | 153 | 29.066 | 33.935 | 17.394 | 1.00 | 32.64 | C |
| ATOM | 2738 | C   | THR | B | 153 | 29.662 | 32.659 | 17.960 | 1.00 | 31.36 | C |
| ATOM | 2739 | O   | THR | B | 153 | 29.374 | 31.569 | 17.490 | 1.00 | 30.07 | O |
| ATOM | 2740 | CB  | THR | B | 153 | 27.739 | 34.233 | 18.103 | 1.00 | 34.64 | C |
| ATOM | 2741 | OG1 | THR | B | 153 | 26.763 | 33.268 | 17.712 | 1.00 | 35.89 | O |
| ATOM | 2742 | CG2 | THR | B | 153 | 27.244 | 35.617 | 17.726 | 1.00 | 35.95 | C |
| ATOM | 2743 | N   | VAL | B | 154 | 30.508 | 32.799 | 18.973 | 1.00 | 30.52 | N |
| ATOM | 2744 | CA  | VAL | B | 154 | 31.117 | 31.640 | 19.612 | 1.00 | 30.22 | C |
| ATOM | 2745 | C   | VAL | B | 154 | 30.863 | 31.691 | 21.116 | 1.00 | 29.91 | C |
| ATOM | 2746 | O   | VAL | B | 154 | 30.982 | 32.721 | 21.737 | 1.00 | 29.05 | O |
| ATOM | 2747 | CB  | VAL | B | 154 | 32.615 | 31.583 | 19.363 | 1.00 | 30.27 | C |
| ATOM | 2748 | CG1 | VAL | B | 154 | 33.205 | 30.276 | 19.923 | 1.00 | 30.77 | C |
| ATOM | 2749 | CG2 | VAL | B | 154 | 32.899 | 31.728 | 17.893 | 1.00 | 30.75 | C |
| ATOM | 2750 | N   | THR | B | 155 | 30.520 | 30.552 | 21.678 | 1.00 | 30.50 | N |
| ATOM | 2751 | CA  | THR | B | 155 | 30.313 | 30.426 | 23.097 | 1.00 | 31.23 | C |
| ATOM | 2752 | C   | THR | B | 155 | 31.055 | 29.175 | 23.556 | 1.00 | 30.33 | C |
| ATOM | 2753 | O   | THR | B | 155 | 31.277 | 28.263 | 22.772 | 1.00 | 29.99 | O |
| ATOM | 2754 | CB  | THR | B | 155 | 28.797 | 30.324 | 23.370 | 1.00 | 32.40 | C |
| ATOM | 2755 | OG1 | THR | B | 155 | 28.221 | 31.616 | 23.224 | 1.00 | 36.61 | O |
| ATOM | 2756 | CG2 | THR | B | 155 | 28.527 | 29.898 | 24.722 | 1.00 | 34.51 | C |
| ATOM | 2757 | N   | TRP | B | 156 | 31.476 | 29.149 | 24.817 | 1.00 | 30.20 | N |
| ATOM | 2758 | CA  | TRP | B | 156 | 32.015 | 27.921 | 25.408 | 1.00 | 29.93 | C |
| ATOM | 2759 | C   | TRP | B | 156 | 31.123 | 27.447 | 26.574 | 1.00 | 32.42 | C |
| ATOM | 2760 | O   | TRP | B | 156 | 30.801 | 28.230 | 27.466 | 1.00 | 30.75 | O |
| ATOM | 2761 | CB  | TRP | B | 156 | 33.432 | 28.138 | 25.883 | 1.00 | 27.96 | C |
| ATOM | 2762 | CG  | TRP | B | 156 | 34.406 | 28.369 | 24.736 | 1.00 | 26.03 | C |
| ATOM | 2763 | CD1 | TRP | B | 156 | 34.718 | 29.555 | 24.160 | 1.00 | 26.03 | C |
| ATOM | 2764 | CD2 | TRP | B | 156 | 35.155 | 27.375 | 24.050 | 1.00 | 25.25 | C |
| ATOM | 2765 | NE1 | TRP | B | 156 | 35.647 | 29.375 | 23.150 | 1.00 | 25.07 | N |
| ATOM | 2766 | CE2 | TRP | B | 156 | 35.946 | 28.038 | 23.077 | 1.00 | 25.00 | C |
| ATOM | 2767 | CE3 | TRP | B | 156 | 35.251 | 25.977 | 24.165 | 1.00 | 25.75 | C |
| ATOM | 2768 | CZ2 | TRP | B | 156 | 36.817 | 27.352 | 22.238 | 1.00 | 24.73 | C |
| ATOM | 2769 | CZ3 | TRP | B | 156 | 36.134 | 25.307 | 23.355 | 1.00 | 25.64 | C |
| ATOM | 2770 | CH2 | TRP | B | 156 | 36.902 | 25.994 | 22.392 | 1.00 | 26.30 | C |
| ATOM | 2771 | N   | ASN | B | 157 | 30.754 | 26.166 | 26.536 | 1.00 | 34.56 | N |
| ATOM | 2772 | CA  | ASN | B | 157 | 29.795 | 25.586 | 27.476 | 1.00 | 37.05 | C |
| ATOM | 2773 | C   | ASN | B | 157 | 28.602 | 26.532 | 27.670 | 1.00 | 39.59 | C |
| ATOM | 2774 | O   | ASN | B | 157 | 28.285 | 26.949 | 28.798 | 1.00 | 40.38 | O |
| ATOM | 2775 | CB  | ASN | B | 157 | 30.482 | 25.258 | 28.796 | 1.00 | 35.94 | C |
| ATOM | 2776 | CG  | ASN | B | 157 | 31.454 | 24.119 | 28.674 | 1.00 | 34.51 | C |
| ATOM | 2777 | OD1 | ASN | B | 157 | 31.623 | 23.525 | 27.608 | 1.00 | 35.68 | O |
| ATOM | 2778 | ND2 | ASN | B | 157 | 32.112 | 23.807 | 29.771 | 1.00 | 32.56 | N |
| ATOM | 2779 | N   | SER | B | 158 | 27.994 | 26.902 | 26.534 | 1.00 | 41.79 | N |
| ATOM | 2780 | CA  | SER | B | 158 | 26.808 | 27.776 | 26.472 | 1.00 | 43.53 | C |
| ATOM | 2781 | C   | SER | B | 158 | 26.891 | 29.021 | 27.356 | 1.00 | 44.22 | C |
| ATOM | 2782 | O   | SER | B | 158 | 25.875 | 29.504 | 27.838 | 1.00 | 44.94 | O |
| ATOM | 2783 | CB  | SER | B | 158 | 25.540 | 26.978 | 26.822 | 1.00 | 43.74 | C |
| ATOM | 2784 | OG  | SER | B | 158 | 25.522 | 25.721 | 26.159 | 1.00 | 45.15 | O |
| ATOM | 2785 | N   | GLY | B | 159 | 28.096 | 29.526 | 27.583 | 1.00 | 45.18 | N |

| | | | | | | | | | | | |
|---|---|---|---|---|---|---|---|---|---|---|---|
| ATOM | 2786 | CA | GLY | B | 159 | 28.296 | 30.747 | 28.373 | 1.00 | 45.96 | C |
| ATOM | 2787 | C | GLY | B | 159 | 29.026 | 30.542 | 29.690 | 1.00 | 46.38 | C |
| ATOM | 2788 | O | GLY | B | 159 | 29.673 | 31.464 | 30.185 | 1.00 | 46.98 | O |
| ATOM | 2789 | N | SER | B | 160 | 28.909 | 29.337 | 30.253 | 1.00 | 47.00 | N |
| ATOM | 2790 | CA | SER | B | 160 | 29.562 | 28.955 | 31.518 | 1.00 | 46.96 | C |
| ATOM | 2791 | C | SER | B | 160 | 31.040 | 29.347 | 31.596 | 1.00 | 47.26 | C |
| ATOM | 2792 | O | SER | B | 160 | 31.535 | 29.721 | 32.655 | 1.00 | 47.27 | O |
| ATOM | 2793 | CB | SER | B | 160 | 29.448 | 27.449 | 31.695 | 1.00 | 46.89 | C |
| ATOM | 2794 | OG | SER | B | 160 | 28.113 | 27.028 | 31.458 | 1.00 | 49.06 | O |
| ATOM | 2795 | N | LEU | B | 161 | 31.747 | 29.225 | 30.480 | 1.00 | 47.15 | N |
| ATOM | 2796 | CA | LEU | B | 161 | 33.123 | 29.722 | 30.372 | 1.00 | 47.06 | C |
| ATOM | 2797 | C | LEU | B | 161 | 33.085 | 30.985 | 29.523 | 1.00 | 47.40 | C |
| ATOM | 2798 | O | LEU | B | 161 | 32.920 | 30.917 | 28.301 | 1.00 | 48.76 | O |
| ATOM | 2799 | CB | LEU | B | 161 | 34.032 | 28.674 | 29.716 | 1.00 | 46.86 | C |
| ATOM | 2800 | CG | LEU | B | 161 | 34.081 | 27.278 | 30.340 | 1.00 | 46.43 | C |
| ATOM | 2801 | CD1 | LEU | B | 161 | 34.552 | 26.240 | 29.355 | 1.00 | 44.85 | C |
| ATOM | 2802 | CD2 | LEU | B | 161 | 34.960 | 27.281 | 31.587 | 1.00 | 47.19 | C |
| ATOM | 2803 | N | SER | B | 162 | 33.177 | 32.134 | 30.179 | 1.00 | 46.94 | N |
| ATOM | 2804 | CA | SER | B | 162 | 33.348 | 33.431 | 29.506 | 1.00 | 46.49 | C |
| ATOM | 2805 | C | SER | B | 162 | 34.699 | 34.038 | 29.879 | 1.00 | 45.54 | C |
| ATOM | 2806 | O | SER | B | 162 | 35.375 | 34.646 | 29.039 | 1.00 | 45.02 | O |
| ATOM | 2807 | CB | SER | B | 162 | 32.227 | 34.404 | 29.902 | 1.00 | 47.32 | C |
| ATOM | 2808 | OG | SER | B | 162 | 32.305 | 34.774 | 31.276 | 1.00 | 48.22 | O |
| ATOM | 2809 | N | SER | B | 163 | 35.063 | 33.889 | 31.155 | 1.00 | 44.06 | N |
| ATOM | 2810 | CA | SER | B | 163 | 36.397 | 34.214 | 31.644 | 1.00 | 43.46 | C |
| ATOM | 2811 | C | SER | B | 163 | 37.411 | 33.320 | 30.969 | 1.00 | 41.26 | C |
| ATOM | 2812 | O | SER | B | 163 | 37.198 | 32.116 | 30.836 | 1.00 | 42.18 | O |
| ATOM | 2813 | CB | SER | B | 163 | 36.478 | 33.994 | 33.158 | 1.00 | 44.23 | C |
| ATOM | 2814 | OG | SER | B | 163 | 35.602 | 34.878 | 33.842 | 1.00 | 46.09 | O |
| ATOM | 2815 | N | GLY | B | 164 | 38.527 | 33.891 | 30.554 | 1.00 | 39.28 | N |
| ATOM | 2816 | CA | GLY | B | 164 | 39.564 | 33.100 | 29.892 | 1.00 | 36.45 | C |
| ATOM | 2817 | C | GLY | B | 164 | 39.195 | 32.647 | 28.484 | 1.00 | 34.26 | C |
| ATOM | 2818 | O | GLY | B | 164 | 39.869 | 31.775 | 27.932 | 1.00 | 32.90 | O |
| ATOM | 2819 | N | VAL | B | 165 | 38.136 | 33.217 | 27.899 | 1.00 | 32.20 | N |
| ATOM | 2820 | CA | VAL | B | 165 | 37.851 | 33.029 | 26.467 | 1.00 | 30.34 | C |
| ATOM | 2821 | C | VAL | B | 165 | 38.460 | 34.191 | 25.648 | 1.00 | 29.15 | C |
| ATOM | 2822 | O | VAL | B | 165 | 38.332 | 35.343 | 26.049 | 1.00 | 30.58 | O |
| ATOM | 2823 | CB | VAL | B | 165 | 36.351 | 32.980 | 26.187 | 1.00 | 30.23 | C |
| ATOM | 2824 | CG1 | VAL | B | 165 | 36.091 | 32.853 | 24.696 | 1.00 | 30.97 | C |
| ATOM | 2825 | CG2 | VAL | B | 165 | 35.696 | 31.814 | 26.928 | 1.00 | 30.56 | C |
| ATOM | 2826 | N | HIS | B | 166 | 39.132 | 33.877 | 24.536 | 1.00 | 27.59 | N |
| ATOM | 2827 | CA | HIS | B | 166 | 39.490 | 34.873 | 23.513 | 1.00 | 26.96 | C |
| ATOM | 2828 | C | HIS | B | 166 | 38.945 | 34.475 | 22.167 | 1.00 | 26.71 | C |
| ATOM | 2829 | O | HIS | B | 166 | 39.399 | 33.505 | 21.575 | 1.00 | 27.44 | O |
| ATOM | 2830 | CB | HIS | B | 166 | 41.004 | 35.031 | 23.353 | 1.00 | 26.68 | C |
| ATOM | 2831 | CG | HIS | B | 166 | 41.670 | 35.594 | 24.559 | 1.00 | 25.70 | C |
| ATOM | 2832 | ND1 | HIS | B | 166 | 41.387 | 36.849 | 25.042 | 1.00 | 23.77 | N |
| ATOM | 2833 | CD2 | HIS | B | 166 | 42.570 | 35.056 | 25.408 | 1.00 | 26.66 | C |
| ATOM | 2834 | CE1 | HIS | B | 166 | 42.100 | 37.066 | 26.130 | 1.00 | 27.73 | C |
| ATOM | 2835 | NE2 | HIS | B | 166 | 42.836 | 35.996 | 26.367 | 1.00 | 27.54 | N |
| ATOM | 2836 | N | THR | B | 167 | 37.992 | 35.236 | 21.655 | 1.00 | 26.76 | N |
| ATOM | 2837 | CA | THR | B | 167 | 37.509 | 35.001 | 20.303 | 1.00 | 25.99 | C |
| ATOM | 2838 | C | THR | B | 167 | 38.100 | 36.076 | 19.408 | 1.00 | 25.34 | C |
| ATOM | 2839 | O | THR | B | 167 | 37.911 | 37.276 | 19.622 | 1.00 | 25.98 | O |
| ATOM | 2840 | CB | THR | B | 167 | 36.005 | 34.976 | 20.237 | 1.00 | 26.58 | C |
| ATOM | 2841 | OG1 | THR | B | 167 | 35.514 | 33.980 | 21.148 | 1.00 | 27.96 | O |
| ATOM | 2842 | CG2 | THR | B | 167 | 35.542 | 34.642 | 18.821 | 1.00 | 26.35 | C |
| ATOM | 2843 | N | PHE | B | 168 | 38.836 | 35.653 | 18.404 | 1.00 | 24.68 | N |
| ATOM | 2844 | CA | PHE | B | 168 | 39.582 | 36.608 | 17.579 | 1.00 | 24.97 | C |
| ATOM | 2845 | C | PHE | B | 168 | 38.711 | 37.165 | 16.452 | 1.00 | 27.09 | C |
| ATOM | 2846 | O | PHE | B | 168 | 37.787 | 36.488 | 15.984 | 1.00 | 27.75 | O |
| ATOM | 2847 | CB | PHE | B | 168 | 40.850 | 35.932 | 17.071 | 1.00 | 24.78 | C |
| ATOM | 2848 | CG | PHE | B | 168 | 41.789 | 35.599 | 18.163 | 1.00 | 23.58 | C |
| ATOM | 2849 | CD1 | PHE | B | 168 | 41.600 | 34.450 | 18.935 | 1.00 | 24.14 | C |
| ATOM | 2850 | CD2 | PHE | B | 168 | 42.795 | 36.452 | 18.501 | 1.00 | 23.17 | C |

| ATOM | 2851 | CE1 | PHE | B | 168 | 42.429 | 34.139 | 19.972 | 1.00 | 22.38 | C |
|------|------|-----|-----|---|-----|--------|--------|--------|------|-------|---|
| ATOM | 2852 | CE2 | PHE | B | 168 | 43.630 | 36.159 | 19.563 | 1.00 | 24.86 | C |
| ATOM | 2853 | CZ  | PHE | B | 168 | 43.431 | 35.000 | 20.320 | 1.00 | 25.66 | C |
| ATOM | 2854 | N   | PRO | B | 169 | 38.981 | 38.410 | 16.022 | 1.00 | 28.43 | N |
| ATOM | 2855 | CA  | PRO | B | 169 | 38.275 | 38.942 | 14.852 | 1.00 | 29.02 | C |
| ATOM | 2856 | C   | PRO | B | 169 | 38.418 | 38.047 | 13.634 | 1.00 | 28.35 | C |
| ATOM | 2857 | O   | PRO | B | 169 | 39.504 | 37.530 | 13.389 | 1.00 | 27.37 | O |
| ATOM | 2858 | CB  | PRO | B | 169 | 39.003 | 40.269 | 14.572 | 1.00 | 29.66 | C |
| ATOM | 2859 | CG  | PRO | B | 169 | 39.581 | 40.675 | 15.897 | 1.00 | 30.27 | C |
| ATOM | 2860 | CD  | PRO | B | 169 | 39.938 | 39.384 | 16.583 | 1.00 | 29.57 | C |
| ATOM | 2861 | N   | ALA | B | 170 | 37.344 | 37.896 | 12.868 | 1.00 | 28.84 | N |
| ATOM | 2862 | CA  | ALA | B | 170 | 37.404 | 37.137 | 11.641 | 1.00 | 29.66 | C |
| ATOM | 2863 | C   | ALA | B | 170 | 38.271 | 37.867 | 10.644 | 1.00 | 31.08 | C |
| ATOM | 2864 | O   | ALA | B | 170 | 38.324 | 39.106 | 10.623 | 1.00 | 31.12 | O |
| ATOM | 2865 | CB  | ALA | B | 170 | 36.036 | 36.934 | 11.060 | 1.00 | 30.09 | C |
| ATOM | 2866 | N   | VAL | B | 171 | 38.946 | 37.087 | 9.820  | 1.00 | 31.91 | N |
| ATOM | 2867 | CA  | VAL | B | 171 | 39.751 | 37.614 | 8.752  | 1.00 | 32.37 | C |
| ATOM | 2868 | C   | VAL | B | 171 | 39.257 | 37.022 | 7.455  | 1.00 | 32.97 | C |
| ATOM | 2869 | O   | VAL | B | 171 | 39.031 | 35.824 | 7.358  | 1.00 | 30.30 | O |
| ATOM | 2870 | CB  | VAL | B | 171 | 41.210 | 37.261 | 8.973  | 1.00 | 32.73 | C |
| ATOM | 2871 | CG1 | VAL | B | 171 | 42.055 | 37.643 | 7.762  | 1.00 | 34.15 | C |
| ATOM | 2872 | CG2 | VAL | B | 171 | 41.719 | 37.972 | 10.229 | 1.00 | 33.63 | C |
| ATOM | 2873 | N   | LEU | B | 172 | 39.115 | 37.881 | 6.456  | 1.00 | 35.06 | N |
| ATOM | 2874 | CA  | LEU | B | 172 | 38.710 | 37.476 | 5.123  | 1.00 | 37.38 | C |
| ATOM | 2875 | C   | LEU | B | 172 | 39.944 | 37.101 | 4.316  | 1.00 | 40.02 | C |
| ATOM | 2876 | O   | LEU | B | 172 | 40.867 | 37.907 | 4.184  | 1.00 | 40.92 | O |
| ATOM | 2877 | CB  | LEU | B | 172 | 37.991 | 38.633 | 4.444  | 1.00 | 37.41 | C |
| ATOM | 2878 | CG  | LEU | B | 172 | 37.243 | 38.354 | 3.153  | 1.00 | 37.11 | C |
| ATOM | 2879 | CD1 | LEU | B | 172 | 35.902 | 37.788 | 3.462  | 1.00 | 37.68 | C |
| ATOM | 2880 | CD2 | LEU | B | 172 | 37.115 | 39.657 | 2.341  | 1.00 | 37.86 | C |
| ATOM | 2881 | N   | GLN | B | 173 | 39.970 | 35.871 | 3.809  | 1.00 | 42.41 | N |
| ATOM | 2882 | CA  | GLN | B | 173 | 41.010 | 35.429 | 2.887  | 1.00 | 45.23 | C |
| ATOM | 2883 | C   | GLN | B | 173 | 40.357 | 34.710 | 1.715  | 1.00 | 45.39 | C |
| ATOM | 2884 | O   | GLN | B | 173 | 39.651 | 33.720 | 1.907  | 1.00 | 44.37 | O |
| ATOM | 2885 | CB  | GLN | B | 173 | 42.003 | 34.485 | 3.571  | 1.00 | 46.07 | C |
| ATOM | 2886 | CG  | GLN | B | 173 | 43.288 | 35.156 | 4.091  | 1.00 | 47.90 | C |
| ATOM | 2887 | CD  | GLN | B | 173 | 44.397 | 34.146 | 4.433  | 1.00 | 48.16 | C |
| ATOM | 2888 | OE1 | GLN | B | 173 | 44.639 | 33.180 | 3.694  | 1.00 | 50.30 | O |
| ATOM | 2889 | NE2 | GLN | B | 173 | 45.082 | 34.380 | 5.553  | 1.00 | 50.76 | N |
| ATOM | 2890 | N   | SER | B | 174 | 40.595 | 35.218 | 0.504  | 1.00 | 45.91 | N |
| ATOM | 2891 | CA  | SER | B | 174 | 40.097 | 34.570 | -0.711 | 1.00 | 46.35 | C |
| ATOM | 2892 | C   | SER | B | 174 | 38.580 | 34.374 | -0.630 | 1.00 | 44.70 | C |
| ATOM | 2893 | O   | SER | B | 174 | 38.051 | 33.277 | -0.882 | 1.00 | 44.86 | O |
| ATOM | 2894 | CB  | SER | B | 174 | 40.811 | 33.232 | -0.907 | 1.00 | 47.05 | C |
| ATOM | 2895 | OG  | SER | B | 174 | 42.135 | 33.317 | -0.405 | 1.00 | 49.82 | O |
| ATOM | 2896 | N   | ASP | B | 175 | 37.914 | 35.459 | -0.234 | 1.00 | 42.24 | N |
| ATOM | 2897 | CA  | ASP | B | 175 | 36.448 | 35.583 | -0.204 | 1.00 | 40.72 | C |
| ATOM | 2898 | C   | ASP | B | 175 | 35.693 | 34.747 | 0.827  | 1.00 | 36.39 | C |
| ATOM | 2899 | O   | ASP | B | 175 | 34.471 | 34.651 | 0.777  | 1.00 | 35.88 | O |
| ATOM | 2900 | CB  | ASP | B | 175 | 35.865 | 35.414 | -1.612 | 1.00 | 42.41 | C |
| ATOM | 2901 | CG  | ASP | B | 175 | 36.196 | 36.610 | -2.509 | 1.00 | 46.31 | C |
| ATOM | 2902 | OD1 | ASP | B | 175 | 35.845 | 37.759 | -2.114 | 1.00 | 47.84 | O |
| ATOM | 2903 | OD2 | ASP | B | 175 | 36.823 | 36.404 | -3.579 | 1.00 | 49.11 | O |
| ATOM | 2904 | N   | LEU | B | 176 | 36.413 | 34.191 | 1.790  | 1.00 | 32.97 | N |
| ATOM | 2905 | CA  | LEU | B | 176 | 35.791 | 33.518 | 2.935  | 1.00 | 29.90 | C |
| ATOM | 2906 | C   | LEU | B | 176 | 36.394 | 34.009 | 4.242  | 1.00 | 28.49 | C |
| ATOM | 2907 | O   | LEU | B | 176 | 37.582 | 34.361 | 4.299  | 1.00 | 26.84 | O |
| ATOM | 2908 | CB  | LEU | B | 176 | 35.986 | 32.017 | 2.824  | 1.00 | 30.83 | C |
| ATOM | 2909 | CG  | LEU | B | 176 | 35.265 | 31.305 | 1.685  | 1.00 | 31.51 | C |
| ATOM | 2910 | CD1 | LEU | B | 176 | 35.887 | 29.946 | 1.468  | 1.00 | 32.10 | C |
| ATOM | 2911 | CD2 | LEU | B | 176 | 33.766 | 31.168 | 1.963  | 1.00 | 33.52 | C |
| ATOM | 2912 | N   | TYR | B | 177 | 35.596 | 34.003 | 5.309  | 1.00 | 26.30 | N |
| ATOM | 2913 | CA  | TYR | B | 177 | 36.097 | 34.415 | 6.614  | 1.00 | 26.17 | C |
| ATOM | 2914 | C   | TYR | B | 177 | 36.570 | 33.186 | 7.387  | 1.00 | 26.53 | C |
| ATOM | 2915 | O   | TYR | B | 177 | 36.044 | 32.078 | 7.225  | 1.00 | 26.19 | O |

```
ATOM   2916  CB   TYR B 177      35.025  35.174   7.427  1.00 26.58           C
ATOM   2917  CG   TYR B 177      34.779  36.603   6.976  1.00 26.54           C
ATOM   2918  CD1  TYR B 177      35.602  37.639   7.415  1.00 27.05           C
ATOM   2919  CD2  TYR B 177      33.720  36.920   6.110  1.00 27.06           C
ATOM   2920  CE1  TYR B 177      35.388  38.969   7.012  1.00 28.22           C
ATOM   2921  CE2  TYR B 177      33.489  38.250   5.710  1.00 28.43           C
ATOM   2922  CZ   TYR B 177      34.333  39.259   6.163  1.00 27.40           C
ATOM   2923  OH   TYR B 177      34.143  40.547   5.761  1.00 28.95           O
ATOM   2924  N    THR B 178      37.577  33.396   8.227  1.00 26.05           N
ATOM   2925  CA   THR B 178      37.965  32.409   9.235  1.00 26.24           C
ATOM   2926  C    THR B 178      38.115  33.110  10.572  1.00 25.01           C
ATOM   2927  O    THR B 178      38.629  34.234  10.649  1.00 24.14           O
ATOM   2928  CB   THR B 178      39.272  31.684   8.862  1.00 25.89           C
ATOM   2929  OG1  THR B 178      39.080  30.943   7.652  1.00 28.66           O
ATOM   2930  CG2  THR B 178      39.691  30.714   9.945  1.00 26.59           C
ATOM   2931  N    LEU B 179      37.614  32.485  11.631  1.00 24.13           N
ATOM   2932  CA   LEU B 179      37.920  32.964  12.966  1.00 24.06           C
ATOM   2933  C    LEU B 179      38.334  31.832  13.877  1.00 23.46           C
ATOM   2934  O    LEU B 179      38.182  30.666  13.535  1.00 23.35           O
ATOM   2935  CB   LEU B 179      36.804  33.816  13.539  1.00 23.76           C
ATOM   2936  CG   LEU B 179      35.522  33.326  14.192  1.00 24.70           C
ATOM   2937  CD1  LEU B 179      35.729  32.514  15.439  1.00 26.62           C
ATOM   2938  CD2  LEU B 179      34.705  34.547  14.518  1.00 25.99           C
ATOM   2939  N    SER B 180      38.935  32.178  14.999  1.00 24.01           N
ATOM   2940  CA   SER B 180      39.272  31.185  15.984  1.00 24.67           C
ATOM   2941  C    SER B 180      38.980  31.722  17.362  1.00 24.40           C
ATOM   2942  O    SER B 180      38.821  32.930  17.558  1.00 23.19           O
ATOM   2943  CB   SER B 180      40.717  30.749  15.848  1.00 24.99           C
ATOM   2944  OG   SER B 180      41.559  31.843  16.090  1.00 28.47           O
ATOM   2945  N    SER B 181      38.823  30.800  18.297  1.00 24.99           N
ATOM   2946  CA   SER B 181      38.548  31.148  19.684  1.00 24.49           C
ATOM   2947  C    SER B 181      39.364  30.246  20.567  1.00 23.53           C
ATOM   2948  O    SER B 181      39.488  29.056  20.265  1.00 24.52           O
ATOM   2949  CB   SER B 181      37.058  30.972  20.005  1.00 25.51           C
ATOM   2950  OG   SER B 181      36.811  31.423  21.327  1.00 24.29           O
ATOM   2951  N    SER B 182      39.949  30.799  21.633  1.00 23.34           N
ATOM   2952  CA   SER B 182      40.674  29.994  22.597  1.00 23.20           C
ATOM   2953  C    SER B 182      39.960  30.030  23.947  1.00 23.21           C
ATOM   2954  O    SER B 182      39.280  30.989  24.272  1.00 24.05           O
ATOM   2955  CB   SER B 182      42.117  30.455  22.773  1.00 23.35           C
ATOM   2956  OG   SER B 182      42.208  31.715  23.391  1.00 23.02           O
ATOM   2957  N    VAL B 183      40.122  28.978  24.725  1.00 24.99           N
ATOM   2958  CA   VAL B 183      39.635  28.994  26.116  1.00 26.35           C
ATOM   2959  C    VAL B 183      40.676  28.344  26.997  1.00 25.98           C
ATOM   2960  O    VAL B 183      41.232  27.320  26.640  1.00 24.65           O
ATOM   2961  CB   VAL B 183      38.246  28.324  26.278  1.00 26.89           C
ATOM   2962  CG1  VAL B 183      38.300  26.848  25.978  1.00 27.59           C
ATOM   2963  CG2  VAL B 183      37.696  28.559  27.672  1.00 28.66           C
ATOM   2964  N    THR B 184      40.947  28.974  28.139  1.00 26.98           N
ATOM   2965  CA   THR B 184      41.948  28.495  29.063  1.00 27.89           C
ATOM   2966  C    THR B 184      41.217  28.104  30.352  1.00 28.89           C
ATOM   2967  O    THR B 184      40.445  28.891  30.897  1.00 28.05           O
ATOM   2968  CB   THR B 184      43.017  29.558  29.314  1.00 28.83           C
ATOM   2969  OG1  THR B 184      43.644  29.896  28.071  1.00 31.53           O
ATOM   2970  CG2  THR B 184      44.093  29.050  30.247  1.00 30.55           C
ATOM   2971  N    VAL B 185      41.423  26.861  30.778  1.00 30.51           N
ATOM   2972  CA   VAL B 185      40.825  26.333  32.000  1.00 31.83           C
ATOM   2973  C    VAL B 185      41.877  25.562  32.822  1.00 32.61           C
ATOM   2974  O    VAL B 185      42.918  25.118  32.293  1.00 30.47           O
ATOM   2975  CB   VAL B 185      39.611  25.401  31.682  1.00 31.97           C
ATOM   2976  CG1  VAL B 185      38.567  26.148  30.876  1.00 33.82           C
ATOM   2977  CG2  VAL B 185      40.035  24.140  30.937  1.00 32.01           C
ATOM   2978  N    PRO B 186      41.587  25.351  34.116  1.00 33.82           N
ATOM   2979  CA   PRO B 186      42.478  24.513  34.898  1.00 34.31           C
ATOM   2980  C    PRO B 186      42.534  23.119  34.302  1.00 34.96           C
```

| ATOM | 2981 | O   | PRO B 186 | 41.500 | 22.628 | 33.840 | 1.00 | 35.54 | O |
|------|------|-----|-----------|--------|--------|--------|------|-------|---|
| ATOM | 2982 | CB  | PRO B 186 | 41.806 | 24.477 | 36.283 | 1.00 | 34.78 | C |
| ATOM | 2983 | CG  | PRO B 186 | 40.916 | 25.641 | 36.323 | 1.00 | 34.51 | C |
| ATOM | 2984 | CD  | PRO B 186 | 40.444 | 25.833 | 34.909 | 1.00 | 34.64 | C |
| ATOM | 2985 | N   | SER B 187 | 43.711 | 22.483 | 34.334 | 1.00 | 35.61 | N |
| ATOM | 2986 | CA  | SER B 187 | 43.881 | 21.107 | 33.840 | 1.00 | 37.01 | C |
| ATOM | 2987 | C   | SER B 187 | 42.987 | 20.075 | 34.528 | 1.00 | 37.94 | C |
| ATOM | 2988 | O   | SER B 187 | 42.630 | 19.036 | 33.945 | 1.00 | 38.34 | O |
| ATOM | 2989 | CB  | SER B 187 | 45.333 | 20.675 | 33.988 | 1.00 | 37.66 | C |
| ATOM | 2990 | OG  | SER B 187 | 46.132 | 21.395 | 33.063 | 1.00 | 39.33 | O |
| ATOM | 2991 | N   | SER B 188 | 42.626 | 20.374 | 35.766 | 1.00 | 38.12 | N |
| ATOM | 2992 | CA  | SER B 188 | 41.750 | 19.534 | 36.541 | 1.00 | 38.58 | C |
| ATOM | 2993 | C   | SER B 188 | 40.301 | 19.556 | 36.051 | 1.00 | 38.57 | C |
| ATOM | 2994 | O   | SER B 188 | 39.457 | 18.815 | 36.584 | 1.00 | 38.28 | O |
| ATOM | 2995 | CB  | SER B 188 | 41.800 | 20.005 | 37.991 | 1.00 | 38.87 | C |
| ATOM | 2996 | OG  | SER B 188 | 41.545 | 21.399 | 38.047 | 1.00 | 39.25 | O |
| ATOM | 2997 | N   | THR B 189 | 39.984 | 20.410 | 35.072 | 1.00 | 37.88 | N |
| ATOM | 2998 | CA  | THR B 189 | 38.622 | 20.482 | 34.570 | 1.00 | 37.29 | C |
| ATOM | 2999 | C   | THR B 189 | 38.473 | 19.948 | 33.160 | 1.00 | 36.49 | C |
| ATOM | 3000 | O   | THR B 189 | 37.351 | 19.743 | 32.708 | 1.00 | 37.34 | O |
| ATOM | 3001 | CB  | THR B 189 | 38.073 | 21.909 | 34.611 | 1.00 | 37.95 | C |
| ATOM | 3002 | OG1 | THR B 189 | 38.794 | 22.720 | 33.671 | 1.00 | 38.46 | O |
| ATOM | 3003 | CG2 | THR B 189 | 38.201 | 22.507 | 36.031 | 1.00 | 38.71 | C |
| ATOM | 3004 | N   | TRP B 190 | 39.579 | 19.760 | 32.446 | 1.00 | 35.46 | N |
| ATOM | 3005 | CA  | TRP B 190 | 39.538 | 19.119 | 31.125 | 1.00 | 34.27 | C |
| ATOM | 3006 | C   | TRP B 190 | 40.685 | 18.116 | 31.025 | 1.00 | 34.70 | C |
| ATOM | 3007 | O   | TRP B 190 | 41.770 | 18.400 | 31.525 | 1.00 | 37.27 | O |
| ATOM | 3008 | CB  | TRP B 190 | 39.659 | 20.165 | 30.014 | 1.00 | 33.46 | C |
| ATOM | 3009 | CG  | TRP B 190 | 39.573 | 19.544 | 28.636 | 1.00 | 32.38 | C |
| ATOM | 3010 | CD1 | TRP B 190 | 38.446 | 19.350 | 27.910 | 1.00 | 32.66 | C |
| ATOM | 3011 | CD2 | TRP B 190 | 40.654 | 19.014 | 27.852 | 1.00 | 32.63 | C |
| ATOM | 3012 | NE1 | TRP B 190 | 38.749 | 18.739 | 26.718 | 1.00 | 31.85 | N |
| ATOM | 3013 | CE2 | TRP B 190 | 40.099 | 18.531 | 26.653 | 1.00 | 32.68 | C |
| ATOM | 3014 | CE3 | TRP B 190 | 42.043 | 18.932 | 28.033 | 1.00 | 32.02 | C |
| ATOM | 3015 | CZ2 | TRP B 190 | 40.877 | 17.960 | 25.648 | 1.00 | 31.07 | C |
| ATOM | 3016 | CZ3 | TRP B 190 | 42.809 | 18.365 | 27.031 | 1.00 | 32.31 | C |
| ATOM | 3017 | CH2 | TRP B 190 | 42.223 | 17.879 | 25.864 | 1.00 | 32.06 | C |
| ATOM | 3018 | N   | PRO B 191 | 40.471 | 16.938 | 30.399 | 1.00 | 34.95 | N |
| ATOM | 3019 | CA  | PRO B 191 | 39.278 | 16.372 | 29.764 | 1.00 | 35.51 | C |
| ATOM | 3020 | C   | PRO B 191 | 38.291 | 15.686 | 30.706 | 1.00 | 36.94 | C |
| ATOM | 3021 | O   | PRO B 191 | 37.325 | 15.090 | 30.232 | 1.00 | 36.65 | O |
| ATOM | 3022 | CB  | PRO B 191 | 39.862 | 15.333 | 28.795 | 1.00 | 34.96 | C |
| ATOM | 3023 | CG  | PRO B 191 | 41.107 | 14.873 | 29.444 | 1.00 | 35.35 | C |
| ATOM | 3024 | CD  | PRO B 191 | 41.627 | 16.034 | 30.261 | 1.00 | 35.40 | C |
| ATOM | 3025 | N   | SER B 192 | 38.522 | 15.760 | 32.012 | 1.00 | 37.99 | N |
| ATOM | 3026 | CA  | SER B 192 | 37.654 | 15.073 | 32.972 | 1.00 | 38.41 | C |
| ATOM | 3027 | C   | SER B 192 | 36.244 | 15.580 | 32.871 | 1.00 | 37.68 | C |
| ATOM | 3028 | O   | SER B 192 | 35.284 | 14.811 | 33.009 | 1.00 | 38.05 | O |
| ATOM | 3029 | CB  | SER B 192 | 38.157 | 15.265 | 34.401 | 1.00 | 39.77 | C |
| ATOM | 3030 | OG  | SER B 192 | 39.306 | 14.444 | 34.623 | 1.00 | 41.80 | O |
| ATOM | 3031 | N   | GLU B 193 | 36.126 | 16.888 | 32.660 | 1.00 | 35.89 | N |
| ATOM | 3032 | CA  | GLU B 193 | 34.862 | 17.495 | 32.321 | 1.00 | 35.75 | C |
| ATOM | 3033 | C   | GLU B 193 | 34.872 | 17.907 | 30.855 | 1.00 | 35.40 | C |
| ATOM | 3034 | O   | GLU B 193 | 35.946 | 18.157 | 30.288 | 1.00 | 34.66 | O |
| ATOM | 3035 | CB  | GLU B 193 | 34.588 | 18.676 | 33.234 | 1.00 | 36.21 | C |
| ATOM | 3036 | CG  | GLU B 193 | 34.091 | 18.224 | 34.595 | 1.00 | 37.81 | C |
| ATOM | 3037 | CD  | GLU B 193 | 34.270 | 19.255 | 35.658 | 1.00 | 38.22 | C |
| ATOM | 3038 | OE1 | GLU B 193 | 35.358 | 19.876 | 35.726 | 1.00 | 39.83 | O |
| ATOM | 3039 | OE2 | GLU B 193 | 33.316 | 19.436 | 36.432 | 1.00 | 38.20 | O |
| ATOM | 3040 | N   | THR B 194 | 33.675 | 17.985 | 30.259 | 1.00 | 34.42 | N |
| ATOM | 3041 | CA  | THR B 194 | 33.544 | 18.285 | 28.836 | 1.00 | 33.42 | C |
| ATOM | 3042 | C   | THR B 194 | 33.739 | 19.758 | 28.563 | 1.00 | 31.66 | C |
| ATOM | 3043 | O   | THR B 194 | 33.277 | 20.598 | 29.320 | 1.00 | 30.64 | O |
| ATOM | 3044 | CB  | THR B 194 | 32.159 | 17.937 | 28.281 | 1.00 | 33.91 | C |
| ATOM | 3045 | OG1 | THR B 194 | 31.190 | 18.830 | 28.847 | 1.00 | 37.48 | O |

| ATOM | 3046 | CG2 | THR | B | 194 | 31.802 | 16.495 | 28.573 | 1.00 | 35.33 | C |
|------|------|-----|-----|---|-----|--------|--------|--------|------|-------|---|
| ATOM | 3047 | N | VAL | B | 195 | 34.414 | 20.076 | 27.464 | 1.00 | 30.30 | N |
| ATOM | 3048 | CA | VAL | B | 195 | 34.490 | 21.465 | 27.017 | 1.00 | 29.01 | C |
| ATOM | 3049 | C | VAL | B | 195 | 34.010 | 21.518 | 25.566 | 1.00 | 28.57 | C |
| ATOM | 3050 | O | VAL | B | 195 | 34.390 | 20.690 | 24.733 | 1.00 | 28.62 | O |
| ATOM | 3051 | CB | VAL | B | 195 | 35.909 | 22.055 | 27.236 | 1.00 | 29.21 | C |
| ATOM | 3052 | CG1 | VAL | B | 195 | 36.038 | 23.460 | 26.588 | 1.00 | 29.14 | C |
| ATOM | 3053 | CG2 | VAL | B | 195 | 36.206 | 22.138 | 28.726 | 1.00 | 28.93 | C |
| ATOM | 3054 | N | THR | B | 196 | 33.145 | 22.469 | 25.290 | 1.00 | 28.74 | N |
| ATOM | 3055 | CA | THR | B | 196 | 32.392 | 22.484 | 24.040 | 1.00 | 29.79 | C |
| ATOM | 3056 | C | THR | B | 196 | 32.284 | 23.905 | 23.507 | 1.00 | 29.37 | C |
| ATOM | 3057 | O | THR | B | 196 | 31.878 | 24.807 | 24.234 | 1.00 | 27.61 | O |
| ATOM | 3058 | CB | THR | B | 196 | 30.974 | 21.864 | 24.292 | 1.00 | 30.38 | C |
| ATOM | 3059 | OG1 | THR | B | 196 | 31.129 | 20.538 | 24.819 | 1.00 | 29.62 | O |
| ATOM | 3060 | CG2 | THR | B | 196 | 30.160 | 21.782 | 23.026 | 1.00 | 32.07 | C |
| ATOM | 3061 | N | CYS | B | 197 | 32.677 | 24.140 | 22.251 | 1.00 | 29.43 | N |
| ATOM | 3062 | CA | CYS | B | 197 | 32.448 | 25.466 | 21.704 | 1.00 | 29.81 | C |
| ATOM | 3063 | C | CYS | B | 197 | 31.162 | 25.435 | 20.902 | 1.00 | 29.37 | C |
| ATOM | 3064 | O | CYS | B | 197 | 30.946 | 24.538 | 20.081 | 1.00 | 27.89 | O |
| ATOM | 3065 | CB | CYS | B | 197 | 33.627 | 25.972 | 20.875 | 1.00 | 32.05 | C |
| ATOM | 3066 | SG | CYS | B | 197 | 33.720 | 25.239 | 19.315 | 1.00 | 34.84 | S |
| ATOM | 3067 | N | ASN | B | 198 | 30.300 | 26.412 | 21.186 | 1.00 | 29.16 | N |
| ATOM | 3068 | CA | ASN | B | 198 | 29.019 | 26.558 | 20.533 | 1.00 | 29.38 | C |
| ATOM | 3069 | C | ASN | B | 198 | 29.138 | 27.670 | 19.489 | 1.00 | 29.29 | C |
| ATOM | 3070 | O | ASN | B | 198 | 29.360 | 28.851 | 19.818 | 1.00 | 28.93 | O |
| ATOM | 3071 | CB | ASN | B | 198 | 27.930 | 26.887 | 21.570 | 1.00 | 30.06 | C |
| ATOM | 3072 | CG | ASN | B | 198 | 28.169 | 26.169 | 22.899 | 1.00 | 28.85 | C |
| ATOM | 3073 | OD1 | ASN | B | 198 | 28.589 | 26.786 | 23.882 | 1.00 | 30.54 | O |
| ATOM | 3074 | ND2 | ASN | B | 198 | 28.013 | 24.853 | 22.894 | 1.00 | 30.89 | N |
| ATOM | 3075 | N | VAL | B | 199 | 28.993 | 27.283 | 18.231 | 1.00 | 28.93 | N |
| ATOM | 3076 | CA | VAL | B | 199 | 29.131 | 28.214 | 17.121 | 1.00 | 29.68 | C |
| ATOM | 3077 | C | VAL | B | 199 | 27.824 | 28.376 | 16.386 | 1.00 | 29.89 | C |
| ATOM | 3078 | O | VAL | B | 199 | 27.258 | 27.391 | 15.953 | 1.00 | 31.15 | O |
| ATOM | 3079 | CB | VAL | B | 199 | 30.163 | 27.691 | 16.099 | 1.00 | 28.78 | C |
| ATOM | 3080 | CG1 | VAL | B | 199 | 30.222 | 28.633 | 14.895 | 1.00 | 28.15 | C |
| ATOM | 3081 | CG2 | VAL | B | 199 | 31.523 | 27.519 | 16.785 | 1.00 | 27.78 | C |
| ATOM | 3082 | N | ALA | B | 200 | 27.393 | 29.618 | 16.202 | 1.00 | 30.12 | N |
| ATOM | 3083 | CA | ALA | B | 200 | 26.258 | 29.930 | 15.353 | 1.00 | 31.27 | C |
| ATOM | 3084 | C | ALA | B | 200 | 26.655 | 30.851 | 14.183 | 1.00 | 31.68 | C |
| ATOM | 3085 | O | ALA | B | 200 | 27.442 | 31.787 | 14.348 | 1.00 | 32.01 | O |
| ATOM | 3086 | CB | ALA | B | 200 | 25.170 | 30.565 | 16.169 | 1.00 | 32.03 | C |
| ATOM | 3087 | N | HIS | B | 201 | 26.103 | 30.559 | 13.006 | 1.00 | 31.85 | N |
| ATOM | 3088 | CA | HIS | B | 201 | 26.204 | 31.421 | 11.829 | 1.00 | 32.61 | C |
| ATOM | 3089 | C | HIS | B | 201 | 24.817 | 31.570 | 11.230 | 1.00 | 33.45 | C |
| ATOM | 3090 | O | HIS | B | 201 | 24.443 | 30.782 | 10.358 | 1.00 | 32.90 | O |
| ATOM | 3091 | CB | HIS | B | 201 | 27.114 | 30.791 | 10.794 | 1.00 | 31.36 | C |
| ATOM | 3092 | CG | HIS | B | 201 | 27.365 | 31.657 | 9.596 | 1.00 | 29.79 | C |
| ATOM | 3093 | ND1 | HIS | B | 201 | 27.040 | 31.263 | 8.319 | 1.00 | 28.72 | N |
| ATOM | 3094 | CD2 | HIS | B | 201 | 27.902 | 32.896 | 9.479 | 1.00 | 29.19 | C |
| ATOM | 3095 | CE1 | HIS | B | 201 | 27.407 | 32.201 | 7.462 | 1.00 | 27.55 | C |
| ATOM | 3096 | NE2 | HIS | B | 201 | 27.921 | 33.209 | 8.141 | 1.00 | 27.62 | N |
| ATOM | 3097 | N | PRO | B | 202 | 24.057 | 32.578 | 11.689 | 1.00 | 35.24 | N |
| ATOM | 3098 | CA | PRO | B | 202 | 22.634 | 32.745 | 11.318 | 1.00 | 35.21 | C |
| ATOM | 3099 | C | PRO | B | 202 | 22.388 | 32.888 | 9.819 | 1.00 | 36.20 | C |
| ATOM | 3100 | O | PRO | B | 202 | 21.433 | 32.322 | 9.297 | 1.00 | 36.61 | O |
| ATOM | 3101 | CB | PRO | B | 202 | 22.222 | 34.029 | 12.056 | 1.00 | 36.09 | C |
| ATOM | 3102 | CG | PRO | B | 202 | 23.217 | 34.153 | 13.204 | 1.00 | 35.82 | C |
| ATOM | 3103 | CD | PRO | B | 202 | 24.510 | 33.637 | 12.616 | 1.00 | 35.68 | C |
| ATOM | 3104 | N | ALA | B | 203 | 23.262 | 33.607 | 9.124 | 1.00 | 36.07 | N |
| ATOM | 3105 | CA | ALA | B | 203 | 23.146 | 33.754 | 7.675 | 1.00 | 36.25 | C |
| ATOM | 3106 | C | ALA | B | 203 | 22.917 | 32.428 | 6.957 | 1.00 | 36.35 | C |
| ATOM | 3107 | O | ALA | B | 203 | 22.272 | 32.390 | 5.909 | 1.00 | 36.46 | O |
| ATOM | 3108 | CB | ALA | B | 203 | 24.392 | 34.456 | 7.111 | 1.00 | 36.70 | C |
| ATOM | 3109 | N | SER | B | 204 | 23.448 | 31.338 | 7.506 | 1.00 | 36.64 | N |
| ATOM | 3110 | CA | SER | B | 204 | 23.349 | 30.024 | 6.874 | 1.00 | 36.12 | C |

```
ATOM   3111  C    SER B 204    22.615  29.031   7.752  1.00 36.67        C
ATOM   3112  O    SER B 204    22.713  27.826   7.534  1.00 36.00        O
ATOM   3113  CB   SER B 204    24.747  29.488   6.563  1.00 36.49        C
ATOM   3114  OG   SER B 204    25.416  29.071   7.738  1.00 35.40        O
ATOM   3115  N    SER B 205    21.905  29.533   8.759  1.00 37.64        N
ATOM   3116  CA   SER B 205    21.177  28.673   9.675  1.00 38.74        C
ATOM   3117  C    SER B 205    22.056  27.535  10.208  1.00 39.17        C
ATOM   3118  O    SER B 205    21.604  26.392  10.316  1.00 39.33        O
ATOM   3119  CB   SER B 205    19.949  28.084   8.976  1.00 39.70        C
ATOM   3120  OG   SER B 205    19.234  29.067   8.257  1.00 40.65        O
ATOM   3121  N    THR B 206    23.312  27.853  10.526  1.00 38.89        N
ATOM   3122  CA   THR B 206    24.243  26.897  11.113  1.00 38.18        C
ATOM   3123  C    THR B 206    24.301  27.103  12.618  1.00 38.31        C
ATOM   3124  O    THR B 206    24.387  28.229  13.101  1.00 38.23        O
ATOM   3125  CB   THR B 206    25.664  27.105  10.564  1.00 38.36        C
ATOM   3126  OG1  THR B 206    25.646  26.965   9.143  1.00 39.27        O
ATOM   3127  CG2  THR B 206    26.649  26.107  11.166  1.00 38.95        C
ATOM   3128  N    LYS B 207    24.243  26.005  13.353  1.00 38.36        N
ATOM   3129  CA   LYS B 207    24.541  25.998  14.773  1.00 39.30        C
ATOM   3130  C    LYS B 207    25.271  24.699  15.039  1.00 38.64        C
ATOM   3131  O    LYS B 207    24.771  23.633  14.674  1.00 38.88        O
ATOM   3132  CB   LYS B 207    23.263  26.037  15.606  1.00 39.66        C
ATOM   3133  CG   LYS B 207    22.607  27.384  15.696  1.00 40.15        C
ATOM   3134  CD   LYS B 207    21.449  27.322  16.687  1.00 41.64        C
ATOM   3135  CE   LYS B 207    20.487  28.491  16.548  1.00 42.37        C
ATOM   3136  NZ   LYS B 207    19.369  28.322  17.515  1.00 43.70        N
ATOM   3137  N    VAL B 208    26.455  24.774  15.642  1.00 37.96        N
ATOM   3138  CA   VAL B 208    27.229  23.559  15.948  1.00 37.99        C
ATOM   3139  C    VAL B 208    27.723  23.620  17.377  1.00 37.73        C
ATOM   3140  O    VAL B 208    28.013  24.698  17.899  1.00 38.07        O
ATOM   3141  CB   VAL B 208    28.448  23.394  15.007  1.00 38.20        C
ATOM   3142  CG1  VAL B 208    29.246  22.155  15.365  1.00 38.72        C
ATOM   3143  CG2  VAL B 208    27.987  23.309  13.582  1.00 39.58        C
ATOM   3144  N    ASP B 209    27.817  22.460  18.010  1.00 37.41        N
ATOM   3145  CA   ASP B 209    28.358  22.365  19.356  1.00 37.38        C
ATOM   3146  C    ASP B 209    29.469  21.340  19.330  1.00 35.91        C
ATOM   3147  O    ASP B 209    29.217  20.159  19.456  1.00 35.61        O
ATOM   3148  CB   ASP B 209    27.252  21.975  20.315  1.00 39.40        C
ATOM   3149  CG   ASP B 209    26.044  22.888  20.191  1.00 41.44        C
ATOM   3150  OD1  ASP B 209    26.165  24.096  20.514  1.00 42.33        O
ATOM   3151  OD2  ASP B 209    24.979  22.393  19.755  1.00 45.37        O
ATOM   3152  N    LYS B 210    30.698  21.824  19.143  1.00 34.71        N
ATOM   3153  CA   LYS B 210    31.896  20.993  18.943  1.00 34.30        C
ATOM   3154  C    LYS B 210    32.607  20.670  20.257  1.00 32.07        C
ATOM   3155  O    LYS B 210    33.136  21.555  20.937  1.00 29.91        O
ATOM   3156  CB   LYS B 210    32.890  21.710  18.006  1.00 34.76        C
ATOM   3157  CG   LYS B 210    33.902  20.782  17.320  1.00 37.58        C
ATOM   3158  CD   LYS B 210    33.352  20.169  16.000  1.00 38.92        C
ATOM   3159  CE   LYS B 210    32.934  18.707  16.175  1.00 40.48        C
ATOM   3160  NZ   LYS B 210    32.527  18.017  14.897  1.00 40.91        N
ATOM   3161  N    LYS B 211    32.627  19.383  20.582  1.00 31.70        N
ATOM   3162  CA   LYS B 211    33.309  18.881  21.763  1.00 31.32        C
ATOM   3163  C    LYS B 211    34.806  18.883  21.512  1.00 31.39        C
ATOM   3164  O    LYS B 211    35.260  18.486  20.446  1.00 30.80        O
ATOM   3165  CB   LYS B 211    32.859  17.438  22.051  1.00 31.50        C
ATOM   3166  CG   LYS B 211    33.302  16.892  23.404  1.00 31.19        C
ATOM   3167  CD   LYS B 211    33.165  15.375  23.450  1.00 31.94        C
ATOM   3168  CE   LYS B 211    32.833  14.897  24.867  1.00 33.07        C
ATOM   3169  NZ   LYS B 211    32.447  13.466  24.824  1.00 33.60        N
ATOM   3170  N    ILE B 212    35.579  19.281  22.507  1.00 31.17        N
ATOM   3171  CA   ILE B 212    37.008  19.186  22.399  1.00 31.51        C
ATOM   3172  C    ILE B 212    37.418  17.892  23.083  1.00 32.92        C
ATOM   3173  O    ILE B 212    37.362  17.805  24.312  1.00 32.13        O
ATOM   3174  CB   ILE B 212    37.730  20.373  23.065  1.00 30.85        C
ATOM   3175  CG1  ILE B 212    37.082  21.710  22.678  1.00 30.03        C
```

| ATOM | 3176 | CG2 | ILE | B | 212 | 39.213 | 20.357 | 22.696 | 1.00 | 30.18 | C |
|------|------|-----|-----|---|-----|--------|--------|--------|------|-------|---|
| ATOM | 3177 | CD1 | ILE | B | 212 | 37.221 | 22.064 | 21.212 | 1.00 | 29.35 | C |
| ATOM | 3178 | N | VAL | B | 213 | 37.824 | 16.900 | 22.291 | 1.00 | 34.14 | N |
| ATOM | 3179 | CA | VAL | B | 213 | 38.261 | 15.610 | 22.828 | 1.00 | 36.15 | C |
| ATOM | 3180 | C | VAL | B | 213 | 39.782 | 15.529 | 22.789 | 1.00 | 36.86 | C |
| ATOM | 3181 | O | VAL | B | 213 | 40.415 | 16.049 | 21.866 | 1.00 | 36.19 | O |
| ATOM | 3182 | CB | VAL | B | 213 | 37.587 | 14.368 | 22.123 | 1.00 | 36.66 | C |
| ATOM | 3183 | CG1 | VAL | B | 213 | 36.237 | 14.737 | 21.501 | 1.00 | 36.62 | C |
| ATOM | 3184 | CG2 | VAL | B | 213 | 38.481 | 13.727 | 21.090 | 1.00 | 38.18 | C |
| ATOM | 3185 | N | PRO | B | 214 | 40.380 | 14.911 | 23.816 | 1.00 | 39.23 | N |
| ATOM | 3186 | CA | PRO | B | 214 | 41.830 | 14.754 | 23.848 | 1.00 | 40.44 | C |
| ATOM | 3187 | C | PRO | B | 214 | 42.289 | 13.804 | 22.755 | 1.00 | 42.28 | C |
| ATOM | 3188 | O | PRO | B | 214 | 41.558 | 12.883 | 22.386 | 1.00 | 42.67 | O |
| ATOM | 3189 | CB | PRO | B | 214 | 42.101 | 14.185 | 25.248 | 1.00 | 40.27 | C |
| ATOM | 3190 | CG | PRO | B | 214 | 40.808 | 13.590 | 25.681 | 1.00 | 40.29 | C |
| ATOM | 3191 | CD | PRO | B | 214 | 39.717 | 14.332 | 25.002 | 1.00 | 39.51 | C |
| ATOM | 3192 | N | ARG | B | 215 | 43.491 | 14.030 | 22.241 | 1.00 | 44.38 | N |
| ATOM | 3193 | CA | ARG | B | 215 | 43.953 | 13.347 | 21.024 | 1.00 | 45.88 | C |
| ATOM | 3194 | C | ARG | B | 215 | 44.481 | 11.907 | 21.225 | 1.00 | 46.63 | C |
| ATOM | 3195 | O | ARG | B | 215 | 44.868 | 11.491 | 22.321 | 1.00 | 47.10 | O |
| ATOM | 3196 | CB | ARG | B | 215 | 45.009 | 14.202 | 20.324 | 1.00 | 46.07 | C |
| ATOM | 3197 | CG | ARG | B | 215 | 44.496 | 15.558 | 19.864 | 1.00 | 46.36 | C |
| ATOM | 3198 | CD | ARG | B | 215 | 45.603 | 16.403 | 19.218 | 1.00 | 47.19 | C |
| ATOM | 3199 | NE | ARG | B | 215 | 46.238 | 15.729 | 18.081 | 1.00 | 47.92 | N |
| ATOM | 3200 | CZ | ARG | B | 215 | 45.789 | 15.748 | 16.823 | 1.00 | 49.00 | C |
| ATOM | 3201 | NH1 | ARG | B | 215 | 44.682 | 16.410 | 16.485 | 1.00 | 48.95 | N |
| ATOM | 3202 | NH2 | ARG | B | 215 | 46.459 | 15.095 | 15.883 | 1.00 | 49.59 | N |
| TER | 3203 | | ARG | B | 215 | | | | | | |
| ATOM | 3204 | N | ASP | C | 1 | 57.199 | 14.305 | 48.771 | 1.00 | 42.25 | N |
| ATOM | 3205 | CA | ASP | C | 1 | 55.844 | 14.690 | 49.284 | 1.00 | 41.64 | C |
| ATOM | 3206 | C | ASP | C | 1 | 54.860 | 14.757 | 48.121 | 1.00 | 39.90 | C |
| ATOM | 3207 | O | ASP | C | 1 | 54.959 | 15.647 | 47.257 | 1.00 | 40.37 | O |
| ATOM | 3208 | CB | ASP | C | 1 | 55.892 | 16.036 | 50.007 | 1.00 | 42.66 | C |
| ATOM | 3209 | CG | ASP | C | 1 | 56.666 | 15.975 | 51.330 | 1.00 | 43.91 | C |
| ATOM | 3210 | OD1 | ASP | C | 1 | 57.442 | 15.018 | 51.548 | 1.00 | 45.17 | O |
| ATOM | 3211 | OD2 | ASP | C | 1 | 56.489 | 16.896 | 52.156 | 1.00 | 46.40 | O |
| ATOM | 3212 | N | ILE | C | 2 | 53.926 | 13.805 | 48.091 | 1.00 | 35.62 | N |
| ATOM | 3213 | CA | ILE | C | 2 | 52.966 | 13.691 | 46.987 | 1.00 | 32.26 | C |
| ATOM | 3214 | C | ILE | C | 2 | 51.987 | 14.854 | 46.958 | 1.00 | 29.92 | C |
| ATOM | 3215 | O | ILE | C | 2 | 51.351 | 15.175 | 47.962 | 1.00 | 29.54 | O |
| ATOM | 3216 | CB | ILE | C | 2 | 52.162 | 12.384 | 47.084 | 1.00 | 31.79 | C |
| ATOM | 3217 | CG1 | ILE | C | 2 | 53.111 | 11.209 | 46.951 | 1.00 | 30.68 | C |
| ATOM | 3218 | CG2 | ILE | C | 2 | 51.062 | 12.349 | 46.009 | 1.00 | 30.59 | C |
| ATOM | 3219 | CD1 | ILE | C | 2 | 52.580 | 9.953 | 47.401 | 1.00 | 32.26 | C |
| ATOM | 3220 | N | VAL | C | 3 | 51.870 | 15.477 | 45.788 | 1.00 | 28.72 | N |
| ATOM | 3221 | CA | VAL | C | 3 | 50.916 | 16.539 | 45.538 | 1.00 | 28.29 | C |
| ATOM | 3222 | C | VAL | C | 3 | 49.695 | 15.938 | 44.844 | 1.00 | 27.78 | C |
| ATOM | 3223 | O | VAL | C | 3 | 49.818 | 15.253 | 43.852 | 1.00 | 27.91 | O |
| ATOM | 3224 | CB | VAL | C | 3 | 51.538 | 17.639 | 44.611 | 1.00 | 28.89 | C |
| ATOM | 3225 | CG1 | VAL | C | 3 | 50.531 | 18.697 | 44.267 | 1.00 | 29.60 | C |
| ATOM | 3226 | CG2 | VAL | C | 3 | 52.738 | 18.283 | 45.304 | 1.00 | 30.21 | C |
| ATOM | 3227 | N | MET | C | 4 | 48.522 | 16.202 | 45.386 | 1.00 | 28.12 | N |
| ATOM | 3228 | CA | MET | C | 4 | 47.265 | 15.703 | 44.832 | 1.00 | 27.92 | C |
| ATOM | 3229 | C | MET | C | 4 | 46.518 | 16.911 | 44.308 | 1.00 | 27.65 | C |
| ATOM | 3230 | O | MET | C | 4 | 46.158 | 17.794 | 45.073 | 1.00 | 28.22 | O |
| ATOM | 3231 | CB | MET | C | 4 | 46.423 | 15.055 | 45.931 | 1.00 | 28.01 | C |
| ATOM | 3232 | CG | MET | C | 4 | 47.101 | 13.978 | 46.690 | 1.00 | 26.35 | C |
| ATOM | 3233 | SD | MET | C | 4 | 47.349 | 12.523 | 45.718 | 1.00 | 30.11 | S |
| ATOM | 3234 | CE | MET | C | 4 | 45.673 | 11.897 | 45.596 | 1.00 | 31.30 | C |
| ATOM | 3235 | N | THR | C | 5 | 46.251 | 16.950 | 43.015 | 1.00 | 27.62 | N |
| ATOM | 3236 | CA | THR | C | 5 | 45.682 | 18.166 | 42.421 | 1.00 | 27.89 | C |
| ATOM | 3237 | C | THR | C | 5 | 44.201 | 18.020 | 42.006 | 1.00 | 26.59 | C |
| ATOM | 3238 | O | THR | C | 5 | 43.846 | 17.123 | 41.253 | 1.00 | 24.88 | O |
| ATOM | 3239 | CB | THR | C | 5 | 46.550 | 18.638 | 41.230 | 1.00 | 27.86 | C |
| ATOM | 3240 | OG1 | THR | C | 5 | 47.935 | 18.559 | 41.592 | 1.00 | 29.88 | O |

| ATOM | 3241 | CG2 | THR | C | 5  | 46.213 | 20.056 | 40.883 | 1.00 | 29.81 | C |
| ATOM | 3242 | N   | GLN | C | 6  | 43.368 | 18.906 | 42.546 | 1.00 | 27.18 | N |
| ATOM | 3243 | CA  | GLN | C | 6  | 41.944 | 18.974 | 42.264 | 1.00 | 28.80 | C |
| ATOM | 3244 | C   | GLN | C | 6  | 41.583 | 20.382 | 41.880 | 1.00 | 31.63 | C |
| ATOM | 3245 | O   | GLN | C | 6  | 42.225 | 21.324 | 42.351 | 1.00 | 32.95 | O |
| ATOM | 3246 | CB  | GLN | C | 6  | 41.127 | 18.649 | 43.527 | 1.00 | 28.19 | C |
| ATOM | 3247 | CG  | GLN | C | 6  | 41.326 | 17.277 | 44.061 | 1.00 | 27.41 | C |
| ATOM | 3248 | CD  | GLN | C | 6  | 40.311 | 16.942 | 45.102 | 1.00 | 26.33 | C |
| ATOM | 3249 | OE1 | GLN | C | 6  | 40.591 | 16.957 | 46.294 | 1.00 | 26.08 | O |
| ATOM | 3250 | NE2 | GLN | C | 6  | 39.103 | 16.675 | 44.663 | 1.00 | 24.98 | N |
| ATOM | 3251 | N   | ALA | C | 7  | 40.530 | 20.513 | 41.075 | 1.00 | 33.49 | N |
| ATOM | 3252 | CA  | ALA | C | 7  | 39.834 | 21.788 | 40.831 | 1.00 | 34.91 | C |
| ATOM | 3253 | C   | ALA | C | 7  | 39.005 | 22.212 | 42.030 | 1.00 | 35.55 | C |
| ATOM | 3254 | O   | ALA | C | 7  | 38.430 | 21.367 | 42.724 | 1.00 | 36.09 | O |
| ATOM | 3255 | CB  | ALA | C | 7  | 38.897 | 21.641 | 39.628 | 1.00 | 35.40 | C |
| ATOM | 3256 | N   | ALA | C | 8  | 38.870 | 23.518 | 42.246 | 1.00 | 36.16 | N |
| ATOM | 3257 | CA  | ALA | C | 8  | 38.096 | 24.013 | 43.398 | 1.00 | 36.80 | C |
| ATOM | 3258 | C   | ALA | C | 8  | 36.582 | 23.798 | 43.271 | 1.00 | 37.04 | C |
| ATOM | 3259 | O   | ALA | C | 8  | 35.904 | 23.629 | 44.279 | 1.00 | 37.33 | O |
| ATOM | 3260 | CB  | ALA | C | 8  | 38.417 | 25.505 | 43.676 | 1.00 | 37.91 | C |
| ATOM | 3261 | N   | PHE | C | 9  | 36.043 | 23.815 | 42.048 | 1.00 | 37.56 | N |
| ATOM | 3262 | CA  | PHE | C | 9  | 34.599 | 23.548 | 41.846 | 1.00 | 38.05 | C |
| ATOM | 3263 | C   | PHE | C | 9  | 34.369 | 22.745 | 40.580 | 1.00 | 36.89 | C |
| ATOM | 3264 | O   | PHE | C | 9  | 35.085 | 22.913 | 39.595 | 1.00 | 35.80 | O |
| ATOM | 3265 | CB  | PHE | C | 9  | 33.776 | 24.852 | 41.748 | 1.00 | 40.12 | C |
| ATOM | 3266 | CG  | PHE | C | 9  | 34.293 | 25.984 | 42.597 | 1.00 | 40.24 | C |
| ATOM | 3267 | CD1 | PHE | C | 9  | 35.361 | 26.762 | 42.156 | 1.00 | 41.59 | C |
| ATOM | 3268 | CD2 | PHE | C | 9  | 33.714 | 26.267 | 43.829 | 1.00 | 40.98 | C |
| ATOM | 3269 | CE1 | PHE | C | 9  | 35.858 | 27.811 | 42.939 | 1.00 | 42.42 | C |
| ATOM | 3270 | CE2 | PHE | C | 9  | 34.197 | 27.291 | 44.622 | 1.00 | 41.23 | C |
| ATOM | 3271 | CZ  | PHE | C | 9  | 35.270 | 28.078 | 44.173 | 1.00 | 42.23 | C |
| ATOM | 3272 | N   | SER | C | 10 | 33.362 | 21.880 | 40.599 | 1.00 | 37.66 | N |
| ATOM | 3273 | CA  | SER | C | 10 | 32.935 | 21.199 | 39.364 | 1.00 | 38.10 | C |
| ATOM | 3274 | C   | SER | C | 10 | 32.212 | 22.196 | 38.446 | 1.00 | 37.70 | C |
| ATOM | 3275 | O   | SER | C | 10 | 31.820 | 23.271 | 38.884 | 1.00 | 38.54 | O |
| ATOM | 3276 | CB  | SER | C | 10 | 32.016 | 20.007 | 39.675 | 1.00 | 38.46 | C |
| ATOM | 3277 | OG  | SER | C | 10 | 30.682 | 20.424 | 39.968 | 1.00 | 38.33 | O |
| ATOM | 3278 | N   | ASN | C | 11 | 32.039 | 21.841 | 37.179 | 1.00 | 37.18 | N |
| ATOM | 3279 | CA  | ASN | C | 11 | 31.137 | 22.590 | 36.303 | 1.00 | 37.01 | C |
| ATOM | 3280 | C   | ASN | C | 11 | 29.741 | 22.401 | 36.828 | 1.00 | 36.26 | C |
| ATOM | 3281 | O   | ASN | C | 11 | 29.460 | 21.365 | 37.445 | 1.00 | 36.43 | O |
| ATOM | 3282 | CB  | ASN | C | 11 | 31.201 | 22.082 | 34.866 | 1.00 | 37.41 | C |
| ATOM | 3283 | CG  | ASN | C | 11 | 32.545 | 22.276 | 34.257 | 1.00 | 37.93 | C |
| ATOM | 3284 | OD1 | ASN | C | 11 | 33.384 | 22.990 | 34.799 | 1.00 | 40.79 | O |
| ATOM | 3285 | ND2 | ASN | C | 11 | 32.782 | 21.626 | 33.142 | 1.00 | 39.27 | N |
| ATOM | 3286 | N   | PRO | C | 12 | 28.864 | 23.404 | 36.641 | 1.00 | 34.72 | N |
| ATOM | 3287 | CA  | PRO | C | 12 | 27.516 | 23.123 | 37.101 | 1.00 | 34.81 | C |
| ATOM | 3288 | C   | PRO | C | 12 | 26.880 | 21.932 | 36.345 | 1.00 | 34.05 | C |
| ATOM | 3289 | O   | PRO | C | 12 | 27.070 | 21.785 | 35.141 | 1.00 | 34.15 | O |
| ATOM | 3290 | CB  | PRO | C | 12 | 26.774 | 24.460 | 36.873 | 1.00 | 35.07 | C |
| ATOM | 3291 | CG  | PRO | C | 12 | 27.863 | 25.505 | 36.806 | 1.00 | 34.67 | C |
| ATOM | 3292 | CD  | PRO | C | 12 | 28.990 | 24.771 | 36.099 | 1.00 | 35.09 | C |
| ATOM | 3293 | N   | VAL | C | 13 | 26.156 | 21.091 | 37.071 | 1.00 | 34.00 | N |
| ATOM | 3294 | CA  | VAL | C | 13 | 25.550 | 19.884 | 36.517 | 1.00 | 34.30 | C |
| ATOM | 3295 | C   | VAL | C | 13 | 24.065 | 19.831 | 36.882 | 1.00 | 33.22 | C |
| ATOM | 3296 | O   | VAL | C | 13 | 23.693 | 19.946 | 38.049 | 1.00 | 33.01 | O |
| ATOM | 3297 | CB  | VAL | C | 13 | 26.332 | 18.555 | 36.959 | 1.00 | 35.07 | C |
| ATOM | 3298 | CG1 | VAL | C | 13 | 27.309 | 18.808 | 38.095 | 1.00 | 35.89 | C |
| ATOM | 3299 | CG2 | VAL | C | 13 | 25.387 | 17.421 | 37.336 | 1.00 | 34.93 | C |
| ATOM | 3300 | N   | THR | C | 14 | 23.218 | 19.650 | 35.876 | 1.00 | 32.55 | N |
| ATOM | 3301 | CA  | THR | C | 14 | 21.793 | 19.599 | 36.090 | 1.00 | 31.83 | C |
| ATOM | 3302 | C   | THR | C | 14 | 21.410 | 18.355 | 36.844 | 1.00 | 32.03 | C |
| ATOM | 3303 | O   | THR | C | 14 | 21.928 | 17.281 | 36.597 | 1.00 | 30.76 | O |
| ATOM | 3304 | CB  | THR | C | 14 | 21.035 | 19.602 | 34.767 | 1.00 | 32.46 | C |
| ATOM | 3305 | OG1 | THR | C | 14 | 21.393 | 20.769 | 34.030 | 1.00 | 29.57 | O |

```
ATOM   3306  CG2 THR C  14      19.509  19.558  35.011  1.00 31.50           C
ATOM   3307  N   LEU C  15      20.482  18.492  37.768  1.00 32.57           N
ATOM   3308  CA  LEU C  15      19.980  17.326  38.463  1.00 34.77           C
ATOM   3309  C   LEU C  15      19.531  16.295  37.413  1.00 34.75           C
ATOM   3310  O   LEU C  15      19.020  16.672  36.355  1.00 34.55           O
ATOM   3311  CB  LEU C  15      18.837  17.706  39.412  1.00 36.61           C
ATOM   3312  CG  LEU C  15      18.629  16.754  40.596  1.00 39.92           C
ATOM   3313  CD1 LEU C  15      17.848  17.430  41.746  1.00 41.17           C
ATOM   3314  CD2 LEU C  15      17.971  15.389  40.171  1.00 40.96           C
ATOM   3315  N   GLY C  16      19.769  15.010  37.685  1.00 34.74           N
ATOM   3316  CA  GLY C  16      19.461  13.933  36.728  1.00 33.84           C
ATOM   3317  C   GLY C  16      20.555  13.666  35.692  1.00 32.88           C
ATOM   3318  O   GLY C  16      20.491  12.675  34.949  1.00 32.51           O
ATOM   3319  N   THR C  17      21.561  14.531  35.624  1.00 32.11           N
ATOM   3320  CA  THR C  17      22.697  14.302  34.718  1.00 30.45           C
ATOM   3321  C   THR C  17      23.992  13.934  35.496  1.00 29.72           C
ATOM   3322  O   THR C  17      23.999  13.910  36.733  1.00 26.96           O
ATOM   3323  CB  THR C  17      22.932  15.492  33.767  1.00 31.52           C
ATOM   3324  OG1 THR C  17      23.571  16.567  34.457  1.00 31.84           O
ATOM   3325  CG2 THR C  17      21.619  15.981  33.148  1.00 33.69           C
ATOM   3326  N   SER C  18      25.074  13.665  34.749  1.00 28.43           N
ATOM   3327  CA  SER C  18      26.288  13.052  35.283  1.00 28.26           C
ATOM   3328  C   SER C  18      27.329  14.097  35.621  1.00 27.57           C
ATOM   3329  O   SER C  18      27.526  15.039  34.860  1.00 25.46           O
ATOM   3330  CB  SER C  18      26.925  12.119  34.248  1.00 28.47           C
ATOM   3331  OG  SER C  18      26.103  11.022  33.922  1.00 28.36           O
ATOM   3332  N   ALA C  19      28.023  13.880  36.734  1.00 27.62           N
ATOM   3333  CA  ALA C  19      29.107  14.747  37.156  1.00 27.19           C
ATOM   3334  C   ALA C  19      30.423  14.027  37.069  1.00 25.82           C
ATOM   3335  O   ALA C  19      30.483  12.818  37.080  1.00 24.71           O
ATOM   3336  CB  ALA C  19      28.889  15.222  38.550  1.00 26.58           C
ATOM   3337  N   SER C  20      31.478  14.810  36.991  1.00 25.12           N
ATOM   3338  CA  SER C  20      32.833  14.298  36.877  1.00 25.80           C
ATOM   3339  C   SER C  20      33.800  15.190  37.679  1.00 24.27           C
ATOM   3340  O   SER C  20      33.875  16.394  37.434  1.00 21.85           O
ATOM   3341  CB  SER C  20      33.237  14.290  35.411  1.00 25.99           C
ATOM   3342  OG  SER C  20      34.269  13.352  35.234  1.00 28.28           O
ATOM   3343  N   ILE C  21      34.484  14.599  38.656  1.00 24.66           N
ATOM   3344  CA  ILE C  21      35.448  15.307  39.498  1.00 25.46           C
ATOM   3345  C   ILE C  21      36.820  14.622  39.383  1.00 25.54           C
ATOM   3346  O   ILE C  21      36.989  13.439  39.695  1.00 23.29           O
ATOM   3347  CB  ILE C  21      34.973  15.389  40.979  1.00 25.40           C
ATOM   3348  CG1 ILE C  21      33.744  16.278  41.094  1.00 26.90           C
ATOM   3349  CG2 ILE C  21      36.048  16.015  41.851  1.00 25.17           C
ATOM   3350  CD1 ILE C  21      33.050  16.205  42.502  1.00 27.95           C
ATOM   3351  N   SER C  22      37.783  15.405  38.925  1.00 25.61           N
ATOM   3352  CA  SER C  22      39.146  14.979  38.675  1.00 27.48           C
ATOM   3353  C   SER C  22      40.042  15.005  39.929  1.00 26.51           C
ATOM   3354  O   SER C  22      39.836  15.784  40.851  1.00 26.83           O
ATOM   3355  CB  SER C  22      39.746  15.945  37.631  1.00 29.09           C
ATOM   3356  OG  SER C  22      41.055  15.574  37.250  1.00 33.79           O
ATOM   3357  N   CYS C  23      41.044  14.144  39.934  1.00 27.69           N
ATOM   3358  CA  CYS C  23      42.145  14.222  40.871  1.00 27.47           C
ATOM   3359  C   CYS C  23      43.411  13.840  40.118  1.00 27.06           C
ATOM   3360  O   CYS C  23      43.399  12.920  39.310  1.00 25.03           O
ATOM   3361  CB  CYS C  23      41.928  13.271  42.062  1.00 28.75           C
ATOM   3362  SG  CYS C  23      43.245  13.312  43.305  1.00 31.52           S
ATOM   3363  N   ARG C  24      44.498  14.548  40.387  1.00 27.12           N
ATOM   3364  CA  ARG C  24      45.798  14.131  39.881  1.00 28.58           C
ATOM   3365  C   ARG C  24      46.792  13.927  41.018  1.00 25.36           C
ATOM   3366  O   ARG C  24      46.887  14.759  41.894  1.00 23.66           O
ATOM   3367  CB  ARG C  24      46.345  15.140  38.850  1.00 28.97           C
ATOM   3368  CG  ARG C  24      47.644  14.631  38.224  1.00 32.41           C
ATOM   3369  CD  ARG C  24      48.184  15.555  37.169  1.00 35.01           C
ATOM   3370  NE  ARG C  24      48.604  16.827  37.730  1.00 40.24           N
```

| | | | | | | | | | | | |
|---|---|---|---|---|---|---|---|---|---|---|---|
| ATOM | 3371 | CZ | ARG | C | 24 | 49.288 | 17.757 | 37.049 | 1.00 | 43.71 | C |
| ATOM | 3372 | NH1 | ARG | C | 24 | 49.654 | 17.536 | 35.780 | 1.00 | 45.29 | N |
| ATOM | 3373 | NH2 | ARG | C | 24 | 49.617 | 18.914 | 37.639 | 1.00 | 42.65 | N |
| ATOM | 3374 | N | SER | C | 25 | 47.515 | 12.806 | 40.966 | 1.00 | 26.00 | N |
| ATOM | 3375 | CA | SER | C | 25 | 48.622 | 12.497 | 41.863 | 1.00 | 27.70 | C |
| ATOM | 3376 | C | SER | C | 25 | 49.989 | 12.689 | 41.179 | 1.00 | 28.87 | C |
| ATOM | 3377 | O | SER | C | 25 | 50.173 | 12.272 | 40.038 | 1.00 | 25.56 | O |
| ATOM | 3378 | CB | SER | C | 25 | 48.563 | 11.057 | 42.297 | 1.00 | 28.13 | C |
| ATOM | 3379 | OG | SER | C | 25 | 49.653 | 10.794 | 43.162 | 1.00 | 31.08 | O |
| ATOM | 3380 | N | SER | C | 26 | 50.931 | 13.271 | 41.909 | 1.00 | 30.64 | N |
| ATOM | 3381 | CA | SER | C | 26 | 52.277 | 13.522 | 41.395 | 1.00 | 32.74 | C |
| ATOM | 3382 | C | SER | C | 26 | 53.143 | 12.280 | 41.415 | 1.00 | 34.09 | C |
| ATOM | 3383 | O | SER | C | 26 | 54.211 | 12.268 | 40.802 | 1.00 | 35.45 | O |
| ATOM | 3384 | CB | SER | C | 26 | 52.942 | 14.646 | 42.209 | 1.00 | 33.77 | C |
| ATOM | 3385 | OG | SER | C | 26 | 53.321 | 14.187 | 43.498 | 1.00 | 34.42 | O |
| ATOM | 3386 | N | LYS | C | 27 | 52.702 | 11.234 | 42.116 | 1.00 | 34.66 | N |
| ATOM | 3387 | CA | LYS | C | 27 | 53.347 | 9.930 | 42.077 | 1.00 | 35.07 | C |
| ATOM | 3388 | C | LYS | C | 27 | 52.274 | 8.901 | 41.757 | 1.00 | 34.53 | C |
| ATOM | 3389 | O | LYS | C | 27 | 51.126 | 9.052 | 42.159 | 1.00 | 33.59 | O |
| ATOM | 3390 | CB | LYS | C | 27 | 54.016 | 9.604 | 43.436 | 1.00 | 36.03 | C |
| ATOM | 3391 | CG | LYS | C | 27 | 54.494 | 8.123 | 43.609 | 1.00 | 36.77 | C |
| ATOM | 3392 | CD | LYS | C | 27 | 55.112 | 7.855 | 44.983 | 1.00 | 37.63 | C |
| ATOM | 3393 | CE | LYS | C | 27 | 55.624 | 6.395 | 45.157 | 1.00 | 38.92 | C |
| ATOM | 3394 | NZ | LYS | C | 27 | 54.855 | 5.590 | 46.198 | 1.00 | 39.65 | N |
| ATOM | 3395 | N | SER | C | 27A | 52.648 | 7.843 | 41.050 | 1.00 | 33.25 | N |
| ATOM | 3396 | CA | SER | C | 27A | 51.708 | 6.797 | 40.736 | 1.00 | 32.79 | C |
| ATOM | 3397 | C | SER | C | 27A | 51.295 | 6.031 | 41.998 | 1.00 | 32.56 | C |
| ATOM | 3398 | O | SER | C | 27A | 52.124 | 5.659 | 42.820 | 1.00 | 32.62 | O |
| ATOM | 3399 | CB | SER | C | 27A | 52.281 | 5.837 | 39.711 | 1.00 | 32.70 | C |
| ATOM | 3400 | OG | SER | C | 27A | 51.498 | 4.669 | 39.651 | 1.00 | 31.21 | O |
| ATOM | 3401 | N | LEU | C | 27B | 49.998 | 5.794 | 42.123 | 1.00 | 31.94 | N |
| ATOM | 3402 | CA | LEU | C | 27B | 49.430 | 5.121 | 43.268 | 1.00 | 29.87 | C |
| ATOM | 3403 | C | LEU | C | 27B | 49.132 | 3.676 | 42.934 | 1.00 | 30.53 | C |
| ATOM | 3404 | O | LEU | C | 27B | 48.588 | 2.951 | 43.774 | 1.00 | 28.68 | O |
| ATOM | 3405 | CB | LEU | C | 27B | 48.150 | 5.838 | 43.693 | 1.00 | 29.82 | C |
| ATOM | 3406 | CG | LEU | C | 27B | 48.289 | 7.340 | 43.946 | 1.00 | 29.37 | C |
| ATOM | 3407 | CD1 | LEU | C | 27B | 46.991 | 7.919 | 44.463 | 1.00 | 28.03 | C |
| ATOM | 3408 | CD2 | LEU | C | 27B | 49.473 | 7.663 | 44.920 | 1.00 | 30.22 | C |
| ATOM | 3409 | N | LEU | C | 27C | 49.472 | 3.250 | 41.712 | 1.00 | 31.07 | N |
| ATOM | 3410 | CA | LEU | C | 27C | 49.271 | 1.857 | 41.316 | 1.00 | 33.40 | C |
| ATOM | 3411 | C | LEU | C | 27C | 50.462 | 0.990 | 41.733 | 1.00 | 34.18 | C |
| ATOM | 3412 | O | LEU | C | 27C | 51.602 | 1.329 | 41.422 | 1.00 | 34.36 | O |
| ATOM | 3413 | CB | LEU | C | 27C | 49.087 | 1.762 | 39.807 | 1.00 | 34.13 | C |
| ATOM | 3414 | CG | LEU | C | 27C | 49.049 | 0.377 | 39.167 | 1.00 | 32.60 | C |
| ATOM | 3415 | CD1 | LEU | C | 27C | 47.846 | -0.390 | 39.632 | 1.00 | 33.09 | C |
| ATOM | 3416 | CD2 | LEU | C | 27C | 49.019 | 0.556 | 37.670 | 1.00 | 33.76 | C |
| ATOM | 3417 | N | HIS | C | 27D | 50.183 | -0.121 | 42.419 | 1.00 | 35.40 | N |
| ATOM | 3418 | CA | HIS | C | 27D | 51.208 | -1.092 | 42.838 | 1.00 | 35.52 | C |
| ATOM | 3419 | C | HIS | C | 27D | 51.078 | -2.460 | 42.144 | 1.00 | 37.21 | C |
| ATOM | 3420 | O | HIS | C | 27D | 50.099 | -2.734 | 41.448 | 1.00 | 37.61 | O |
| ATOM | 3421 | CB | HIS | C | 27D | 51.171 | -1.259 | 44.365 | 1.00 | 35.79 | C |
| ATOM | 3422 | CG | HIS | C | 27D | 51.432 | 0.014 | 45.111 | 1.00 | 35.05 | C |
| ATOM | 3423 | ND1 | HIS | C | 27D | 51.059 | 0.195 | 46.423 | 1.00 | 32.83 | N |
| ATOM | 3424 | CD2 | HIS | C | 27D | 52.016 | 1.173 | 44.720 | 1.00 | 33.86 | C |
| ATOM | 3425 | CE1 | HIS | C | 27D | 51.413 | 1.412 | 46.808 | 1.00 | 35.18 | C |
| ATOM | 3426 | NE2 | HIS | C | 27D | 51.991 | 2.025 | 45.792 | 1.00 | 32.18 | N |
| ATOM | 3427 | N | SER | C | 27E | 52.069 | -3.330 | 42.363 | 1.00 | 38.37 | N |
| ATOM | 3428 | CA | SER | C | 27E | 52.153 | -4.619 | 41.668 | 1.00 | 39.50 | C |
| ATOM | 3429 | C | SER | C | 27E | 51.046 | -5.600 | 42.053 | 1.00 | 39.50 | C |
| ATOM | 3430 | O | SER | C | 27E | 50.797 | -6.580 | 41.338 | 1.00 | 40.35 | O |
| ATOM | 3431 | CB | SER | C | 27E | 53.530 | -5.267 | 41.904 | 1.00 | 40.94 | C |
| ATOM | 3432 | OG | SER | C | 27E | 53.855 | -5.340 | 43.284 | 1.00 | 41.90 | O |
| ATOM | 3433 | N | ASP | C | 28 | 50.399 | -5.352 | 43.183 | 1.00 | 39.16 | N |
| ATOM | 3434 | CA | ASP | C | 28 | 49.140 | -6.041 | 43.528 | 1.00 | 38.63 | C |
| ATOM | 3435 | C | ASP | C | 28 | 48.014 | -5.704 | 42.564 | 1.00 | 37.85 | C |

| ATOM | 3436 | O   | ASP | C | 28 | 46.903 | -6.220 | 42.712 | 1.00 | 37.64 | O |
|------|------|-----|-----|---|----|--------|--------|--------|------|-------|---|
| ATOM | 3437 | CB  | ASP | C | 28 | 48.690 | -5.677 | 44.950 | 1.00 | 38.79 | C |
| ATOM | 3438 | CG  | ASP | C | 28 | 48.706 | -4.176 | 45.215 | 1.00 | 38.76 | C |
| ATOM | 3439 | OD1 | ASP | C | 28 | 48.620 | -3.383 | 44.264 | 1.00 | 39.51 | O |
| ATOM | 3440 | OD2 | ASP | C | 28 | 48.828 | -3.778 | 46.387 | 1.00 | 41.05 | O |
| ATOM | 3441 | N   | GLY | C | 29 | 48.301 | -4.811 | 41.610 | 1.00 | 37.95 | N |
| ATOM | 3442 | CA  | GLY | C | 29 | 47.292 | -4.264 | 40.689 | 1.00 | 37.69 | C |
| ATOM | 3443 | C   | GLY | C | 29 | 46.263 | -3.373 | 41.380 | 1.00 | 37.26 | C |
| ATOM | 3444 | O   | GLY | C | 29 | 45.194 | -3.128 | 40.827 | 1.00 | 37.65 | O |
| ATOM | 3445 | N   | ILE | C | 30 | 46.577 | -2.895 | 42.587 | 1.00 | 35.30 | N |
| ATOM | 3446 | CA  | ILE | C | 30 | 45.681 | -2.004 | 43.339 | 1.00 | 33.79 | C |
| ATOM | 3447 | C   | ILE | C | 30 | 46.198 | -0.577 | 43.256 | 1.00 | 30.81 | C |
| ATOM | 3448 | O   | ILE | C | 30 | 47.396 | -0.353 | 43.193 | 1.00 | 30.75 | O |
| ATOM | 3449 | CB  | ILE | C | 30 | 45.520 | -2.443 | 44.813 | 1.00 | 34.02 | C |
| ATOM | 3450 | CG1 | ILE | C | 30 | 45.056 | -3.906 | 44.867 | 1.00 | 34.44 | C |
| ATOM | 3451 | CG2 | ILE | C | 30 | 44.529 | -1.534 | 45.562 | 1.00 | 34.13 | C |
| ATOM | 3452 | CD1 | ILE | C | 30 | 45.068 | -4.527 | 46.273 | 1.00 | 35.69 | C |
| ATOM | 3453 | N   | THR | C | 31 | 45.264 | 0.373  | 43.208 | 1.00 | 29.30 | N |
| ATOM | 3454 | CA  | THR | C | 31 | 45.567 | 1.800  | 43.133 | 1.00 | 28.08 | C |
| ATOM | 3455 | C   | THR | C | 31 | 45.048 | 2.416  | 44.426 | 1.00 | 25.66 | C |
| ATOM | 3456 | O   | THR | C | 31 | 43.844 | 2.362  | 44.720 | 1.00 | 24.24 | O |
| ATOM | 3457 | CB  | THR | C | 31 | 44.926 | 2.464  | 41.876 | 1.00 | 28.33 | C |
| ATOM | 3458 | OG1 | THR | C | 31 | 45.359 | 1.782  | 40.695 | 1.00 | 29.57 | O |
| ATOM | 3459 | CG2 | THR | C | 31 | 45.327 | 3.912  | 41.749 | 1.00 | 28.30 | C |
| ATOM | 3460 | N   | TYR | C | 32 | 45.972 | 2.957  | 45.204 | 1.00 | 24.92 | N |
| ATOM | 3461 | CA  | TYR | C | 32 | 45.730 | 3.333  | 46.608 | 1.00 | 24.85 | C |
| ATOM | 3462 | C   | TYR | C | 32 | 45.280 | 4.786  | 46.683 | 1.00 | 22.58 | C |
| ATOM | 3463 | O   | TYR | C | 32 | 45.967 | 5.696  | 47.188 | 1.00 | 21.41 | O |
| ATOM | 3464 | CB  | TYR | C | 32 | 46.959 | 3.010  | 47.471 | 1.00 | 26.11 | C |
| ATOM | 3465 | CG  | TYR | C | 32 | 47.145 | 1.514  | 47.619 | 1.00 | 28.05 | C |
| ATOM | 3466 | CD1 | TYR | C | 32 | 47.874 | 0.770  | 46.681 | 1.00 | 28.61 | C |
| ATOM | 3467 | CD2 | TYR | C | 32 | 46.556 | 0.833  | 48.662 | 1.00 | 28.55 | C |
| ATOM | 3468 | CE1 | TYR | C | 32 | 48.032 | -0.617 | 46.813 | 1.00 | 28.94 | C |
| ATOM | 3469 | CE2 | TYR | C | 32 | 46.679 | -0.549 | 48.782 | 1.00 | 30.10 | C |
| ATOM | 3470 | CZ  | TYR | C | 32 | 47.423 | -1.270 | 47.867 | 1.00 | 30.02 | C |
| ATOM | 3471 | OH  | TYR | C | 32 | 47.540 | -2.643 | 48.025 | 1.00 | 30.36 | O |
| ATOM | 3472 | N   | LEU | C | 33 | 44.099 | 4.973  | 46.113 | 1.00 | 20.86 | N |
| ATOM | 3473 | CA  | LEU | C | 33 | 43.475 | 6.273  | 45.968 | 1.00 | 20.07 | C |
| ATOM | 3474 | C   | LEU | C | 33 | 42.151 | 6.191  | 46.706 | 1.00 | 19.10 | C |
| ATOM | 3475 | O   | LEU | C | 33 | 41.460 | 5.185  | 46.580 | 1.00 | 18.75 | O |
| ATOM | 3476 | CB  | LEU | C | 33 | 43.205 | 6.550  | 44.488 | 1.00 | 19.05 | C |
| ATOM | 3477 | CG  | LEU | C | 33 | 42.659 | 7.895  | 44.026 | 1.00 | 20.81 | C |
| ATOM | 3478 | CD1 | LEU | C | 33 | 41.106 | 7.964  | 44.000 | 1.00 | 19.87 | C |
| ATOM | 3479 | CD2 | LEU | C | 33 | 43.267 | 9.022  | 44.822 | 1.00 | 22.41 | C |
| ATOM | 3480 | N   | TYR | C | 34 | 41.823 | 7.251  | 47.450 | 1.00 | 19.85 | N |
| ATOM | 3481 | CA  | TYR | C | 34 | 40.586 | 7.345  | 48.245 | 1.00 | 19.96 | C |
| ATOM | 3482 | C   | TYR | C | 34 | 39.880 | 8.657  | 47.943 | 1.00 | 20.01 | C |
| ATOM | 3483 | O   | TYR | C | 34 | 40.544 | 9.660  | 47.637 | 1.00 | 19.48 | O |
| ATOM | 3484 | CB  | TYR | C | 34 | 40.923 | 7.319  | 49.734 | 1.00 | 21.15 | C |
| ATOM | 3485 | CG  | TYR | C | 34 | 41.498 | 6.017  | 50.178 | 1.00 | 22.27 | C |
| ATOM | 3486 | CD1 | TYR | C | 34 | 42.787 | 5.697  | 49.844 | 1.00 | 22.84 | C |
| ATOM | 3487 | CD2 | TYR | C | 34 | 40.748 | 5.087  | 50.945 | 1.00 | 22.13 | C |
| ATOM | 3488 | CE1 | TYR | C | 34 | 43.338 | 4.516  | 50.194 | 1.00 | 21.76 | C |
| ATOM | 3489 | CE2 | TYR | C | 34 | 41.315 | 3.913  | 51.336 | 1.00 | 21.73 | C |
| ATOM | 3490 | CZ  | TYR | C | 34 | 42.624 | 3.638  | 50.938 | 1.00 | 22.82 | C |
| ATOM | 3491 | OH  | TYR | C | 34 | 43.280 | 2.503  | 51.259 | 1.00 | 23.37 | O |
| ATOM | 3492 | N   | TRP | C | 35 | 38.545 | 8.633  | 48.009 | 1.00 | 19.15 | N |
| ATOM | 3493 | CA  | TRP | C | 35 | 37.680 | 9.800  | 47.861 | 1.00 | 17.95 | C |
| ATOM | 3494 | C   | TRP | C | 35 | 36.844 | 9.935  | 49.127 | 1.00 | 19.40 | C |
| ATOM | 3495 | O   | TRP | C | 35 | 36.338 | 8.924  | 49.647 | 1.00 | 17.88 | O |
| ATOM | 3496 | CB  | TRP | C | 35 | 36.688 | 9.637  | 46.688 | 1.00 | 19.18 | C |
| ATOM | 3497 | CG  | TRP | C | 35 | 37.265 | 9.745  | 45.354 | 1.00 | 19.36 | C |
| ATOM | 3498 | CD1 | TRP | C | 35 | 37.559 | 8.725  | 44.499 | 1.00 | 20.29 | C |
| ATOM | 3499 | CD2 | TRP | C | 35 | 37.657 | 10.953 | 44.702 | 1.00 | 17.95 | C |
| ATOM | 3500 | NE1 | TRP | C | 35 | 38.102 | 9.238  | 43.336 | 1.00 | 19.90 | N |

```
ATOM    3501  CE2 TRP C  35      38.179  10.601  43.450  1.00 20.01           C
ATOM    3502  CE3 TRP C  35      37.604  12.300  45.056  1.00 18.61           C
ATOM    3503  CZ2 TRP C  35      38.647  11.552  42.545  1.00 19.31           C
ATOM    3504  CZ3 TRP C  35      38.074  13.246  44.150  1.00 19.44           C
ATOM    3505  CH2 TRP C  35      38.585  12.857  42.918  1.00 19.51           C
ATOM    3506  N   TYR C  36      36.693  11.179  49.582  1.00 21.23           N
ATOM    3507  CA  TYR C  36      35.888  11.544  50.735  1.00 21.91           C
ATOM    3508  C   TYR C  36      34.937  12.646  50.300  1.00 24.17           C
ATOM    3509  O   TYR C  36      35.275  13.481  49.454  1.00 24.12           O
ATOM    3510  CB  TYR C  36      36.762  12.071  51.878  1.00 21.05           C
ATOM    3511  CG  TYR C  36      37.732  11.064  52.339  1.00 20.59           C
ATOM    3512  CD1 TYR C  36      39.013  10.994  51.792  1.00 18.57           C
ATOM    3513  CD2 TYR C  36      37.359  10.115  53.290  1.00 19.43           C
ATOM    3514  CE1 TYR C  36      39.908  10.024  52.205  1.00 20.31           C
ATOM    3515  CE2 TYR C  36      38.237   9.139  53.696  1.00 20.68           C
ATOM    3516  CZ  TYR C  36      39.510   9.093  53.162  1.00 19.24           C
ATOM    3517  OH  TYR C  36      40.369   8.112  53.611  1.00 19.83           O
ATOM    3518  N   LEU C  37      33.739  12.614  50.873  1.00 25.79           N
ATOM    3519  CA  LEU C  37      32.766  13.657  50.700  1.00 27.52           C
ATOM    3520  C   LEU C  37      32.525  14.346  52.035  1.00 29.00           C
ATOM    3521  O   LEU C  37      32.207  13.687  53.020  1.00 29.07           O
ATOM    3522  CB  LEU C  37      31.451  13.043  50.220  1.00 27.99           C
ATOM    3523  CG  LEU C  37      30.254  13.985  50.102  1.00 28.18           C
ATOM    3524  CD1 LEU C  37      30.578  15.165  49.174  1.00 29.73           C
ATOM    3525  CD2 LEU C  37      29.023  13.252  49.615  1.00 28.46           C
ATOM    3526  N   GLN C  38      32.657  15.668  52.037  1.00 31.64           N
ATOM    3527  CA  GLN C  38      32.169  16.497  53.124  1.00 33.09           C
ATOM    3528  C   GLN C  38      30.963  17.342  52.653  1.00 34.79           C
ATOM    3529  O   GLN C  38      31.104  18.318  51.907  1.00 32.88           O
ATOM    3530  CB  GLN C  38      33.268  17.393  53.689  1.00 32.20           C
ATOM    3531  CG  GLN C  38      32.783  18.184  54.889  1.00 32.81           C
ATOM    3532  CD  GLN C  38      33.888  18.780  55.741  1.00 32.78           C
ATOM    3533  OE1 GLN C  38      34.841  19.350  55.238  1.00 33.66           O
ATOM    3534  NE2 GLN C  38      33.721  18.688  57.045  1.00 33.71           N
ATOM    3535  N   LYS C  39      29.779  16.933  53.098  1.00 37.46           N
ATOM    3536  CA  LYS C  39      28.569  17.706  52.875  1.00 40.94           C
ATOM    3537  C   LYS C  39      28.506  18.887  53.851  1.00 43.42           C
ATOM    3538  O   LYS C  39      29.024  18.788  54.973  1.00 44.36           O
ATOM    3539  CB  LYS C  39      27.353  16.813  53.068  1.00 41.00           C
ATOM    3540  CG  LYS C  39      27.316  15.619  52.144  1.00 42.33           C
ATOM    3541  CD  LYS C  39      25.918  15.065  52.001  1.00 42.23           C
ATOM    3542  CE  LYS C  39      25.930  13.579  51.752  1.00 43.51           C
ATOM    3543  NZ  LYS C  39      26.302  12.826  52.985  1.00 44.67           N
ATOM    3544  N   PRO C  40      27.849  20.001  53.455  1.00 45.86           N
ATOM    3545  CA  PRO C  40      27.762  21.139  54.387  1.00 46.72           C
ATOM    3546  C   PRO C  40      27.036  20.731  55.670  1.00 46.94           C
ATOM    3547  O   PRO C  40      25.977  20.107  55.597  1.00 47.22           O
ATOM    3548  CB  PRO C  40      26.944  22.179  53.609  1.00 47.09           C
ATOM    3549  CG  PRO C  40      26.181  21.373  52.568  1.00 47.31           C
ATOM    3550  CD  PRO C  40      27.124  20.264  52.194  1.00 46.45           C
ATOM    3551  N   GLY C  41      27.633  21.056  56.816  1.00 47.66           N
ATOM    3552  CA  GLY C  41      27.058  20.761  58.131  1.00 48.50           C
ATOM    3553  C   GLY C  41      27.391  19.385  58.687  1.00 49.44           C
ATOM    3554  O   GLY C  41      26.870  18.983  59.734  1.00 49.24           O
ATOM    3555  N   GLN C  42      28.239  18.637  57.982  1.00 50.07           N
ATOM    3556  CA  GLN C  42      28.659  17.321  58.455  1.00 50.06           C
ATOM    3557  C   GLN C  42      30.166  17.161  58.262  1.00 48.33           C
ATOM    3558  O   GLN C  42      30.819  17.999  57.614  1.00 47.23           O
ATOM    3559  CB  GLN C  42      27.874  16.211  57.737  1.00 51.48           C
ATOM    3560  CG  GLN C  42      26.337  16.275  57.952  1.00 53.50           C
ATOM    3561  CD  GLN C  42      25.610  17.244  56.992  1.00 55.94           C
ATOM    3562  OE1 GLN C  42      26.079  17.519  55.887  1.00 57.64           O
ATOM    3563  NE2 GLN C  42      24.458  17.765  57.429  1.00 58.01           N
ATOM    3564  N   SER C  43      30.705  16.098  58.851  1.00 46.61           N
ATOM    3565  CA  SER C  43      32.137  15.766  58.715  1.00 45.76           C
```

| ATOM | 3566 | C | SER C | 43 | 32.436 | 14.921 | 57.471 | 1.00 | 43.03 | C |
|------|------|-----|-------|-----|--------|--------|--------|------|-------|---|
| ATOM | 3567 | O | SER C | 43 | 31.545 | 14.224 | 56.968 | 1.00 | 42.80 | O |
| ATOM | 3568 | CB | SER C | 43 | 32.590 | 14.971 | 59.929 | 1.00 | 46.28 | C |
| ATOM | 3569 | OG | SER C | 43 | 32.004 | 13.672 | 59.962 | 1.00 | 47.97 | O |
| ATOM | 3570 | N | PRO C | 44 | 33.703 | 14.919 | 57.010 | 1.00 | 39.85 | N |
| ATOM | 3571 | CA | PRO C | 44 | 34.036 | 14.023 | 55.892 | 1.00 | 37.10 | C |
| ATOM | 3572 | C | PRO C | 44 | 33.715 | 12.564 | 56.174 | 1.00 | 34.80 | C |
| ATOM | 3573 | O | PRO C | 44 | 33.831 | 12.105 | 57.311 | 1.00 | 34.55 | O |
| ATOM | 3574 | CB | PRO C | 44 | 35.552 | 14.220 | 55.705 | 1.00 | 36.87 | C |
| ATOM | 3575 | CG | PRO C | 44 | 35.856 | 15.510 | 56.334 | 1.00 | 37.36 | C |
| ATOM | 3576 | CD | PRO C | 44 | 34.876 | 15.682 | 57.461 | 1.00 | 38.57 | C |
| ATOM | 3577 | N | HIS C | 45 | 33.261 | 11.859 | 55.141 | 1.00 | 32.12 | N |
| ATOM | 3578 | CA | HIS C | 45 | 33.065 | 10.434 | 55.207 | 1.00 | 31.10 | C |
| ATOM | 3579 | C | HIS C | 45 | 33.672 | 9.803 | 53.970 | 1.00 | 28.84 | C |
| ATOM | 3580 | O | HIS C | 45 | 33.706 | 10.407 | 52.897 | 1.00 | 25.59 | O |
| ATOM | 3581 | CB | HIS C | 45 | 31.568 | 10.045 | 55.355 | 1.00 | 32.77 | C |
| ATOM | 3582 | CG | HIS C | 45 | 30.705 | 10.387 | 54.170 | 1.00 | 34.63 | C |
| ATOM | 3583 | ND1 | HIS C | 45 | 30.029 | 11.587 | 54.053 | 1.00 | 36.68 | N |
| ATOM | 3584 | CD2 | HIS C | 45 | 30.384 | 9.669 | 53.062 | 1.00 | 36.40 | C |
| ATOM | 3585 | CE1 | HIS C | 45 | 29.350 | 11.599 | 52.920 | 1.00 | 34.70 | C |
| ATOM | 3586 | NE2 | HIS C | 45 | 29.547 | 10.448 | 52.300 | 1.00 | 34.87 | N |
| ATOM | 3587 | N | LEU C | 46 | 34.136 | 8.584 | 54.164 | 1.00 | 27.08 | N |
| ATOM | 3588 | CA | LEU C | 46 | 34.700 | 7.767 | 53.105 | 1.00 | 27.61 | C |
| ATOM | 3589 | C | LEU C | 46 | 33.644 | 7.498 | 52.055 | 1.00 | 28.47 | C |
| ATOM | 3590 | O | LEU C | 46 | 32.505 | 7.099 | 52.374 | 1.00 | 27.88 | O |
| ATOM | 3591 | CB | LEU C | 46 | 35.220 | 6.448 | 53.661 | 1.00 | 26.32 | C |
| ATOM | 3592 | CG | LEU C | 46 | 35.803 | 5.481 | 52.644 | 1.00 | 25.85 | C |
| ATOM | 3593 | CD1 | LEU C | 46 | 37.045 | 6.089 | 52.010 | 1.00 | 24.25 | C |
| ATOM | 3594 | CD2 | LEU C | 46 | 36.134 | 4.197 | 53.267 | 1.00 | 27.11 | C |
| ATOM | 3595 | N | LEU C | 47 | 34.031 | 7.736 | 50.801 | 1.00 | 27.68 | N |
| ATOM | 3596 | CA | LEU C | 47 | 33.170 | 7.501 | 49.658 | 1.00 | 27.74 | C |
| ATOM | 3597 | C | LEU C | 47 | 33.649 | 6.264 | 48.905 | 1.00 | 27.00 | C |
| ATOM | 3598 | O | LEU C | 47 | 32.884 | 5.319 | 48.708 | 1.00 | 28.01 | O |
| ATOM | 3599 | CB | LEU C | 47 | 33.164 | 8.732 | 48.768 | 1.00 | 27.93 | C |
| ATOM | 3600 | CG | LEU C | 47 | 31.996 | 8.879 | 47.801 | 1.00 | 29.79 | C |
| ATOM | 3601 | CD1 | LEU C | 47 | 30.719 | 9.311 | 48.537 | 1.00 | 30.05 | C |
| ATOM | 3602 | CD2 | LEU C | 47 | 32.323 | 9.863 | 46.737 | 1.00 | 29.38 | C |
| ATOM | 3603 | N | ILE C | 48 | 34.925 | 6.247 | 48.534 | 1.00 | 24.24 | N |
| ATOM | 3604 | CA | ILE C | 48 | 35.492 | 5.193 | 47.707 | 1.00 | 24.34 | C |
| ATOM | 3605 | C | ILE C | 48 | 36.886 | 4.875 | 48.211 | 1.00 | 23.30 | C |
| ATOM | 3606 | O | ILE C | 48 | 37.627 | 5.774 | 48.605 | 1.00 | 19.88 | O |
| ATOM | 3607 | CB | ILE C | 48 | 35.548 | 5.646 | 46.181 | 1.00 | 25.38 | C |
| ATOM | 3608 | CG1 | ILE C | 48 | 34.158 | 5.553 | 45.541 | 1.00 | 26.12 | C |
| ATOM | 3609 | CG2 | ILE C | 48 | 36.514 | 4.796 | 45.380 | 1.00 | 24.40 | C |
| ATOM | 3610 | CD1 | ILE C | 48 | 33.945 | 6.508 | 44.463 | 1.00 | 25.69 | C |
| ATOM | 3611 | N | TYR C | 49 | 37.239 | 3.590 | 48.196 | 1.00 | 24.39 | N |
| ATOM | 3612 | CA | TYR C | 49 | 38.562 | 3.153 | 48.600 | 1.00 | 24.81 | C |
| ATOM | 3613 | C | TYR C | 49 | 39.203 | 2.343 | 47.499 | 1.00 | 25.25 | C |
| ATOM | 3614 | O | TYR C | 49 | 38.512 | 1.739 | 46.677 | 1.00 | 24.66 | O |
| ATOM | 3615 | CB | TYR C | 49 | 38.516 | 2.375 | 49.913 | 1.00 | 27.13 | C |
| ATOM | 3616 | CG | TYR C | 49 | 37.697 | 1.108 | 49.902 | 1.00 | 27.79 | C |
| ATOM | 3617 | CD1 | TYR C | 49 | 36.339 | 1.145 | 50.126 | 1.00 | 28.34 | C |
| ATOM | 3618 | CD2 | TYR C | 49 | 38.301 | -0.133 | 49.702 | 1.00 | 29.84 | C |
| ATOM | 3619 | CE1 | TYR C | 49 | 35.588 | -0.015 | 50.137 | 1.00 | 29.14 | C |
| ATOM | 3620 | CE2 | TYR C | 49 | 37.551 | -1.313 | 49.693 | 1.00 | 29.07 | C |
| ATOM | 3621 | CZ | TYR C | 49 | 36.199 | -1.240 | 49.916 | 1.00 | 29.51 | C |
| ATOM | 3622 | OH | TYR C | 49 | 35.435 | -2.399 | 49.928 | 1.00 | 30.13 | O |
| ATOM | 3623 | N | HIS C | 50 | 40.530 | 2.370 | 47.469 | 1.00 | 25.53 | N |
| ATOM | 3624 | CA | HIS C | 50 | 41.301 | 1.669 | 46.444 | 1.00 | 27.12 | C |
| ATOM | 3625 | C | HIS C | 50 | 40.756 | 1.988 | 45.023 | 1.00 | 26.83 | C |
| ATOM | 3626 | O | HIS C | 50 | 40.576 | 1.090 | 44.194 | 1.00 | 25.83 | O |
| ATOM | 3627 | CB | HIS C | 50 | 41.291 | 0.153 | 46.729 | 1.00 | 28.23 | C |
| ATOM | 3628 | CG | HIS C | 50 | 42.060 | -0.241 | 47.954 | 1.00 | 30.28 | C |
| ATOM | 3629 | ND1 | HIS C | 50 | 42.215 | -1.557 | 48.344 | 1.00 | 30.14 | N |
| ATOM | 3630 | CD2 | HIS C | 50 | 42.737 | 0.504 | 48.863 | 1.00 | 31.59 | C |

| ATOM | 3631 | CE1 | HIS | C | 50 | 42.944 | -1.602 | 49.448 | 1.00 | 32.65 | C |
|------|------|-----|-----|---|----|--------|--------|--------|------|-------|---|
| ATOM | 3632 | NE2 | HIS | C | 50 | 43.285 | -0.365 | 49.777 | 1.00 | 32.03 | N |
| ATOM | 3633 | N | LEU | C | 51 | 40.471 | 3.270 | 44.789 | 1.00 | 25.47 | N |
| ATOM | 3634 | CA | LEU | C | 51 | 40.171 | 3.823 | 43.454 | 1.00 | 26.60 | C |
| ATOM | 3635 | C | LEU | C | 51 | 38.778 | 3.578 | 42.961 | 1.00 | 25.44 | C |
| ATOM | 3636 | O | LEU | C | 51 | 38.206 | 4.454 | 42.360 | 1.00 | 26.14 | O |
| ATOM | 3637 | CB | LEU | C | 51 | 41.164 | 3.349 | 42.373 | 1.00 | 26.96 | C |
| ATOM | 3638 | CG | LEU | C | 51 | 40.977 | 3.923 | 40.946 | 1.00 | 26.52 | C |
| ATOM | 3639 | CD1 | LEU | C | 51 | 41.097 | 5.437 | 40.893 | 1.00 | 24.83 | C |
| ATOM | 3640 | CD2 | LEU | C | 51 | 41.991 | 3.274 | 40.025 | 1.00 | 28.01 | C |
| ATOM | 3641 | N | SER | C | 52 | 38.241 | 2.386 | 43.161 | 1.00 | 25.10 | N |
| ATOM | 3642 | CA | SER | C | 52 | 36.981 | 2.047 | 42.539 | 1.00 | 26.05 | C |
| ATOM | 3643 | C | SER | C | 52 | 35.982 | 1.314 | 43.421 | 1.00 | 26.71 | C |
| ATOM | 3644 | O | SER | C | 52 | 34.928 | 0.961 | 42.929 | 1.00 | 26.08 | O |
| ATOM | 3645 | CB | SER | C | 52 | 37.258 | 1.157 | 41.355 | 1.00 | 26.54 | C |
| ATOM | 3646 | OG | SER | C | 52 | 37.783 | -0.070 | 41.794 | 1.00 | 26.70 | O |
| ATOM | 3647 | N | ASN | C | 53 | 36.303 | 1.070 | 44.692 | 1.00 | 28.21 | N |
| ATOM | 3648 | CA | ASN | C | 53 | 35.352 | 0.380 | 45.608 | 1.00 | 28.05 | C |
| ATOM | 3649 | C | ASN | C | 53 | 34.536 | 1.327 | 46.438 | 1.00 | 29.14 | C |
| ATOM | 3650 | O | ASN | C | 53 | 35.076 | 2.166 | 47.146 | 1.00 | 29.21 | O |
| ATOM | 3651 | CB | ASN | C | 53 | 36.075 | -0.575 | 46.558 | 1.00 | 29.15 | C |
| ATOM | 3652 | CG | ASN | C | 53 | 36.989 | -1.527 | 45.837 | 1.00 | 30.87 | C |
| ATOM | 3653 | OD1 | ASN | C | 53 | 36.574 | -2.597 | 45.417 | 1.00 | 35.29 | O |
| ATOM | 3654 | ND2 | ASN | C | 53 | 38.237 | -1.136 | 45.672 | 1.00 | 33.20 | N |
| ATOM | 3655 | N | LEU | C | 54 | 33.222 | 1.156 | 46.384 | 1.00 | 28.85 | N |
| ATOM | 3656 | CA | LEU | C | 54 | 32.303 | 1.980 | 47.135 | 1.00 | 30.37 | C |
| ATOM | 3657 | C | LEU | C | 54 | 32.305 | 1.582 | 48.620 | 1.00 | 29.98 | C |
| ATOM | 3658 | O | LEU | C | 54 | 32.350 | 0.395 | 48.943 | 1.00 | 29.60 | O |
| ATOM | 3659 | CB | LEU | C | 54 | 30.897 | 1.780 | 46.589 | 1.00 | 30.80 | C |
| ATOM | 3660 | CG | LEU | C | 54 | 30.219 | 2.906 | 45.843 | 1.00 | 32.15 | C |
| ATOM | 3661 | CD1 | LEU | C | 54 | 31.153 | 3.543 | 44.834 | 1.00 | 32.76 | C |
| ATOM | 3662 | CD2 | LEU | C | 54 | 28.959 | 2.343 | 45.200 | 1.00 | 32.12 | C |
| ATOM | 3663 | N | ALA | C | 55 | 32.248 | 2.573 | 49.502 | 1.00 | 29.80 | N |
| ATOM | 3664 | CA | ALA | C | 55 | 32.137 | 2.312 | 50.939 | 1.00 | 30.95 | C |
| ATOM | 3665 | C | ALA | C | 55 | 30.678 | 1.940 | 51.226 | 1.00 | 32.36 | C |
| ATOM | 3666 | O | ALA | C | 55 | 29.770 | 2.342 | 50.491 | 1.00 | 31.61 | O |
| ATOM | 3667 | CB | ALA | C | 55 | 32.553 | 3.528 | 51.726 | 1.00 | 30.04 | C |
| ATOM | 3668 | N | SER | C | 56 | 30.420 | 1.186 | 52.289 | 1.00 | 35.83 | N |
| ATOM | 3669 | CA | SER | C | 56 | 29.051 | 0.682 | 52.461 | 1.00 | 37.05 | C |
| ATOM | 3670 | C | SER | C | 56 | 28.100 | 1.863 | 52.664 | 1.00 | 36.78 | C |
| ATOM | 3671 | O | SER | C | 56 | 28.461 | 2.897 | 53.253 | 1.00 | 37.07 | O |
| ATOM | 3672 | CB | SER | C | 56 | 28.929 | -0.390 | 53.556 | 1.00 | 38.02 | C |
| ATOM | 3673 | OG | SER | C | 56 | 29.802 | -0.145 | 54.635 | 1.00 | 40.71 | O |
| ATOM | 3674 | N | GLY | C | 57 | 26.911 | 1.732 | 52.083 | 1.00 | 36.67 | N |
| ATOM | 3675 | CA | GLY | C | 57 | 25.884 | 2.768 | 52.168 | 1.00 | 35.40 | C |
| ATOM | 3676 | C | GLY | C | 57 | 26.002 | 3.896 | 51.166 | 1.00 | 34.98 | C |
| ATOM | 3677 | O | GLY | C | 57 | 25.116 | 4.746 | 51.096 | 1.00 | 34.44 | O |
| ATOM | 3678 | N | VAL | C | 58 | 27.102 | 3.941 | 50.412 | 1.00 | 34.33 | N |
| ATOM | 3679 | CA | VAL | C | 58 | 27.266 | 4.945 | 49.360 | 1.00 | 33.64 | C |
| ATOM | 3680 | C | VAL | C | 58 | 26.416 | 4.553 | 48.140 | 1.00 | 32.33 | C |
| ATOM | 3681 | O | VAL | C | 58 | 26.428 | 3.405 | 47.719 | 1.00 | 32.46 | O |
| ATOM | 3682 | CB | VAL | C | 58 | 28.767 | 5.131 | 48.972 | 1.00 | 33.78 | C |
| ATOM | 3683 | CG1 | VAL | C | 58 | 28.912 | 5.956 | 47.705 | 1.00 | 34.81 | C |
| ATOM | 3684 | CG2 | VAL | C | 58 | 29.527 | 5.825 | 50.102 | 1.00 | 33.72 | C |
| ATOM | 3685 | N | PRO | C | 59 | 25.671 | 5.508 | 47.576 | 1.00 | 33.05 | N |
| ATOM | 3686 | CA | PRO | C | 59 | 24.771 | 5.132 | 46.486 | 1.00 | 34.24 | C |
| ATOM | 3687 | C | PRO | C | 59 | 25.470 | 4.619 | 45.202 | 1.00 | 34.57 | C |
| ATOM | 3688 | O | PRO | C | 59 | 26.613 | 4.997 | 44.899 | 1.00 | 34.07 | O |
| ATOM | 3689 | CB | PRO | C | 59 | 23.989 | 6.411 | 46.193 | 1.00 | 33.61 | C |
| ATOM | 3690 | CG | PRO | C | 59 | 24.340 | 7.379 | 47.259 | 1.00 | 34.29 | C |
| ATOM | 3691 | CD | PRO | C | 59 | 25.609 | 6.944 | 47.885 | 1.00 | 33.77 | C |
| ATOM | 3692 | N | ASP | C | 60 | 24.741 | 3.761 | 44.491 | 1.00 | 34.18 | N |
| ATOM | 3693 | CA | ASP | C | 60 | 25.178 | 3.093 | 43.272 | 1.00 | 34.62 | C |
| ATOM | 3694 | C | ASP | C | 60 | 25.703 | 4.026 | 42.203 | 1.00 | 31.80 | C |
| ATOM | 3695 | O | ASP | C | 60 | 26.476 | 3.625 | 41.345 | 1.00 | 32.06 | O |

```
ATOM   3696  CB   ASP C  60    23.989   2.335  42.662  1.00  36.45          C
ATOM   3697  CG   ASP C  60    24.055   0.852  42.926  1.00  41.00          C
ATOM   3698  OD1  ASP C  60    25.139   0.253  42.695  1.00  45.26          O
ATOM   3699  OD2  ASP C  60    23.028   0.281  43.347  1.00  44.71          O
ATOM   3700  N    ARG C  61    25.210   5.250  42.229  1.00  29.51          N
ATOM   3701  CA   ARG C  61    25.486   6.224  41.198  1.00  28.86          C
ATOM   3702  C    ARG C  61    26.900   6.795  41.238  1.00  26.29          C
ATOM   3703  O    ARG C  61    27.301   7.477  40.310  1.00  25.73          O
ATOM   3704  CB   ARG C  61    24.473   7.333  41.286  1.00  27.98          C
ATOM   3705  CG   ARG C  61    23.071   6.850  40.987  1.00  31.00          C
ATOM   3706  CD   ARG C  61    22.092   7.958  41.170  1.00  33.46          C
ATOM   3707  NE   ARG C  61    22.101   8.450  42.540  1.00  34.70          N
ATOM   3708  CZ   ARG C  61    22.717   9.547  42.986  1.00  36.94          C
ATOM   3709  NH1  ARG C  61    23.445  10.330  42.180  1.00  37.91          N
ATOM   3710  NH2  ARG C  61    22.621   9.847  44.278  1.00  37.17          N
ATOM   3711  N    PHE C  62    27.626   6.534  42.321  1.00  26.55          N
ATOM   3712  CA   PHE C  62    29.036   6.921  42.442  1.00  24.90          C
ATOM   3713  C    PHE C  62    29.956   5.838  41.914  1.00  22.81          C
ATOM   3714  O    PHE C  62    29.681   4.644  42.012  1.00  22.93          O
ATOM   3715  CB   PHE C  62    29.427   7.217  43.906  1.00  26.42          C
ATOM   3716  CG   PHE C  62    28.790   8.436  44.470  1.00  26.32          C
ATOM   3717  CD1  PHE C  62    27.553   8.354  45.114  1.00  29.96          C
ATOM   3718  CD2  PHE C  62    29.398   9.664  44.361  1.00  27.24          C
ATOM   3719  CE1  PHE C  62    26.934   9.496  45.622  1.00  28.84          C
ATOM   3720  CE2  PHE C  62    28.801  10.809  44.881  1.00  28.15          C
ATOM   3721  CZ   PHE C  62    27.568  10.729  45.504  1.00  29.66          C
ATOM   3722  N    SER C  63    31.099   6.260  41.371  1.00  20.31          N
ATOM   3723  CA   SER C  63    32.108   5.316  40.903  1.00  19.02          C
ATOM   3724  C    SER C  63    33.398   6.102  40.722  1.00  16.21          C
ATOM   3725  O    SER C  63    33.363   7.315  40.702  1.00  16.48          O
ATOM   3726  CB   SER C  63    31.679   4.679  39.580  1.00  20.41          C
ATOM   3727  OG   SER C  63    31.696   5.622  38.507  1.00  20.63          O
ATOM   3728  N    SER C  64    34.509   5.429  40.613  1.00  16.08          N
ATOM   3729  CA   SER C  64    35.774   6.132  40.291  1.00  16.57          C
ATOM   3730  C    SER C  64    36.597   5.208  39.441  1.00  18.05          C
ATOM   3731  O    SER C  64    36.424   4.008  39.474  1.00  16.12          O
ATOM   3732  CB   SER C  64    36.488   6.592  41.584  1.00  16.39          C
ATOM   3733  OG   SER C  64    37.852   7.037  41.424  1.00  16.49          O
ATOM   3734  N    SER C  65    37.462   5.778  38.609  1.00  18.95          N
ATOM   3735  CA   SER C  65    38.367   4.997  37.811  1.00  20.25          C
ATOM   3736  C    SER C  65    39.558   5.854  37.470  1.00  20.65          C
ATOM   3737  O    SER C  65    39.520   7.059  37.672  1.00  19.60          O
ATOM   3738  CB   SER C  65    37.698   4.599  36.503  1.00  22.79          C
ATOM   3739  OG   SER C  65    37.465   5.730  35.670  1.00  25.37          O
ATOM   3740  N    GLY C  66    40.586   5.246  36.898  1.00  22.78          N
ATOM   3741  CA   GLY C  66    41.728   6.028  36.405  1.00  24.51          C
ATOM   3742  C    GLY C  66    42.978   5.209  36.219  1.00  26.33          C
ATOM   3743  O    GLY C  66    42.966   3.984  36.340  1.00  26.36          O
ATOM   3744  N    SER C  67    44.053   5.923  35.888  1.00  29.65          N
ATOM   3745  CA   SER C  67    45.394   5.366  35.805  1.00  31.02          C
ATOM   3746  C    SER C  67    46.002   5.402  37.226  1.00  32.72          C
ATOM   3747  O    SER C  67    45.270   5.537  38.224  1.00  32.99          O
ATOM   3748  CB   SER C  67    46.229   6.178  34.806  1.00  31.00          C
ATOM   3749  OG   SER C  67    46.591   7.438  35.343  1.00  30.82          O
ATOM   3750  N    GLY C  68    47.318   5.233  37.325  1.00  32.78          N
ATOM   3751  CA   GLY C  68    48.019   5.353  38.605  1.00  31.85          C
ATOM   3752  C    GLY C  68    48.175   6.789  39.050  1.00  31.47          C
ATOM   3753  O    GLY C  68    48.464   7.037  40.224  1.00  30.19          O
ATOM   3754  N    THR C  69    47.983   7.742  38.132  1.00  31.61          N
ATOM   3755  CA   THR C  69    48.150   9.163  38.460  1.00  31.73          C
ATOM   3756  C    THR C  69    46.966  10.071  38.130  1.00  31.45          C
ATOM   3757  O    THR C  69    46.877  11.178  38.638  1.00  32.84          O
ATOM   3758  CB   THR C  69    49.353   9.737  37.694  1.00  32.29          C
ATOM   3759  OG1  THR C  69    49.113   9.583  36.297  1.00  34.52          O
ATOM   3760  CG2  THR C  69    50.626   9.018  38.063  1.00  32.71          C
```

| | | | | | | | | | | | |
|---|---|---|---|---|---|---|---|---|---|---|---|
| ATOM | 3761 | N | ASP | C | 70 | 46.061 | 9.623 | 37.272 | 1.00 | 30.52 | N |
| ATOM | 3762 | CA | ASP | C | 70 | 44.971 | 10.460 | 36.806 | 1.00 | 28.73 | C |
| ATOM | 3763 | C | ASP | C | 70 | 43.665 | 9.729 | 37.082 | 1.00 | 25.59 | C |
| ATOM | 3764 | O | ASP | C | 70 | 43.441 | 8.677 | 36.505 | 1.00 | 23.78 | O |
| ATOM | 3765 | CB | ASP | C | 70 | 45.101 | 10.701 | 35.311 | 1.00 | 30.84 | C |
| ATOM | 3766 | CG | ASP | C | 70 | 46.430 | 11.355 | 34.949 | 1.00 | 34.50 | C |
| ATOM | 3767 | OD1 | ASP | C | 70 | 46.741 | 12.405 | 35.557 | 1.00 | 37.12 | O |
| ATOM | 3768 | OD2 | ASP | C | 70 | 47.143 | 10.810 | 34.071 | 1.00 | 38.81 | O |
| ATOM | 3769 | N | PHE | C | 71 | 42.816 | 10.330 | 37.913 | 1.00 | 22.94 | N |
| ATOM | 3770 | CA | PHE | C | 71 | 41.583 | 9.678 | 38.387 | 1.00 | 21.16 | C |
| ATOM | 3771 | C | PHE | C | 71 | 40.387 | 10.591 | 38.233 | 1.00 | 21.19 | C |
| ATOM | 3772 | O | PHE | C | 71 | 40.523 | 11.799 | 38.192 | 1.00 | 19.05 | O |
| ATOM | 3773 | CB | PHE | C | 71 | 41.731 | 9.262 | 39.850 | 1.00 | 19.99 | C |
| ATOM | 3774 | CG | PHE | C | 71 | 43.151 | 9.033 | 40.282 | 1.00 | 19.88 | C |
| ATOM | 3775 | CD1 | PHE | C | 71 | 43.816 | 7.875 | 39.956 | 1.00 | 20.61 | C |
| ATOM | 3776 | CD2 | PHE | C | 71 | 43.799 | 9.973 | 41.057 | 1.00 | 19.24 | C |
| ATOM | 3777 | CE1 | PHE | C | 71 | 45.132 | 7.680 | 40.351 | 1.00 | 22.50 | C |
| ATOM | 3778 | CE2 | PHE | C | 71 | 45.113 | 9.786 | 41.451 | 1.00 | 22.87 | C |
| ATOM | 3779 | CZ | PHE | C | 71 | 45.781 | 8.644 | 41.084 | 1.00 | 21.90 | C |
| ATOM | 3780 | N | THR | C | 72 | 39.197 | 9.986 | 38.139 | 1.00 | 21.16 | N |
| ATOM | 3781 | CA | THR | C | 72 | 37.958 | 10.713 | 37.976 | 1.00 | 22.13 | C |
| ATOM | 3782 | C | THR | C | 72 | 36.913 | 10.041 | 38.864 | 1.00 | 21.02 | C |
| ATOM | 3783 | O | THR | C | 72 | 36.662 | 8.857 | 38.718 | 1.00 | 17.50 | O |
| ATOM | 3784 | CB | THR | C | 72 | 37.436 | 10.678 | 36.490 | 1.00 | 24.42 | C |
| ATOM | 3785 | OG1 | THR | C | 72 | 38.496 | 11.005 | 35.577 | 1.00 | 27.70 | O |
| ATOM | 3786 | CG2 | THR | C | 72 | 36.313 | 11.685 | 36.294 | 1.00 | 25.68 | C |
| ATOM | 3787 | N | LEU | C | 73 | 36.347 | 10.813 | 39.775 | 1.00 | 21.00 | N |
| ATOM | 3788 | CA | LEU | C | 73 | 35.116 | 10.433 | 40.476 | 1.00 | 22.44 | C |
| ATOM | 3789 | C | LEU | C | 73 | 33.920 | 10.834 | 39.575 | 1.00 | 22.08 | C |
| ATOM | 3790 | O | LEU | C | 73 | 33.827 | 11.982 | 39.157 | 1.00 | 23.13 | O |
| ATOM | 3791 | CB | LEU | C | 73 | 35.072 | 11.169 | 41.818 | 1.00 | 21.03 | C |
| ATOM | 3792 | CG | LEU | C | 73 | 33.808 | 11.009 | 42.638 | 1.00 | 23.26 | C |
| ATOM | 3793 | CD1 | LEU | C | 73 | 33.818 | 9.628 | 43.235 | 1.00 | 23.32 | C |
| ATOM | 3794 | CD2 | LEU | C | 73 | 33.735 | 12.083 | 43.697 | 1.00 | 23.75 | C |
| ATOM | 3795 | N | ARG | C | 74 | 33.037 | 9.889 | 39.266 | 1.00 | 20.73 | N |
| ATOM | 3796 | CA | ARG | C | 74 | 31.862 | 10.139 | 38.465 | 1.00 | 20.37 | C |
| ATOM | 3797 | C | ARG | C | 74 | 30.568 | 9.874 | 39.276 | 1.00 | 20.33 | C |
| ATOM | 3798 | O | ARG | C | 74 | 30.512 | 8.959 | 40.089 | 1.00 | 19.67 | O |
| ATOM | 3799 | CB | ARG | C | 74 | 31.881 | 9.273 | 37.214 | 1.00 | 21.15 | C |
| ATOM | 3800 | CG | ARG | C | 74 | 32.948 | 9.698 | 36.227 | 1.00 | 21.73 | C |
| ATOM | 3801 | CD | ARG | C | 74 | 32.972 | 8.905 | 34.976 | 1.00 | 22.40 | C |
| ATOM | 3802 | NE | ARG | C | 74 | 34.173 | 9.220 | 34.177 | 1.00 | 22.97 | N |
| ATOM | 3803 | CZ | ARG | C | 74 | 34.326 | 10.318 | 33.456 | 1.00 | 23.87 | C |
| ATOM | 3804 | NH1 | ARG | C | 74 | 33.368 | 11.233 | 33.434 | 1.00 | 23.45 | N |
| ATOM | 3805 | NH2 | ARG | C | 74 | 35.452 | 10.497 | 32.739 | 1.00 | 24.62 | N |
| ATOM | 3806 | N | ILE | C | 75 | 29.550 | 10.692 | 39.027 | 1.00 | 20.83 | N |
| ATOM | 3807 | CA | ILE | C | 75 | 28.236 | 10.552 | 39.681 | 1.00 | 22.17 | C |
| ATOM | 3808 | C | ILE | C | 75 | 27.193 | 10.567 | 38.567 | 1.00 | 22.43 | C |
| ATOM | 3809 | O | ILE | C | 75 | 27.003 | 11.596 | 37.948 | 1.00 | 23.63 | O |
| ATOM | 3810 | CB | ILE | C | 75 | 27.908 | 11.747 | 40.659 | 1.00 | 21.96 | C |
| ATOM | 3811 | CG1 | ILE | C | 75 | 29.117 | 12.125 | 41.510 | 1.00 | 23.02 | C |
| ATOM | 3812 | CG2 | ILE | C | 75 | 26.696 | 11.395 | 41.544 | 1.00 | 22.06 | C |
| ATOM | 3813 | CD1 | ILE | C | 75 | 28.973 | 13.486 | 42.254 | 1.00 | 24.28 | C |
| ATOM | 3814 | N | SER | C | 76 | 26.555 | 9.435 | 38.281 | 1.00 | 24.83 | N |
| ATOM | 3815 | CA | SER | C | 76 | 25.421 | 9.425 | 37.344 | 1.00 | 25.90 | C |
| ATOM | 3816 | C | SER | C | 76 | 24.191 | 10.065 | 38.040 | 1.00 | 27.22 | C |
| ATOM | 3817 | O | SER | C | 76 | 24.154 | 10.156 | 39.260 | 1.00 | 28.50 | O |
| ATOM | 3818 | CB | SER | C | 76 | 25.129 | 8.012 | 36.858 | 1.00 | 25.30 | C |
| ATOM | 3819 | OG | SER | C | 76 | 24.834 | 7.101 | 37.926 | 1.00 | 25.43 | O |
| ATOM | 3820 | N | ARG | C | 77 | 23.240 | 10.537 | 37.245 | 1.00 | 27.85 | N |
| ATOM | 3821 | CA | ARG | C | 77 | 21.982 | 11.103 | 37.714 | 1.00 | 28.42 | C |
| ATOM | 3822 | C | ARG | C | 77 | 22.106 | 11.863 | 39.046 | 1.00 | 30.69 | C |
| ATOM | 3823 | O | ARG | C | 77 | 21.580 | 11.419 | 40.077 | 1.00 | 30.93 | O |
| ATOM | 3824 | CB | ARG | C | 77 | 20.932 | 9.999 | 37.782 | 1.00 | 27.80 | C |
| ATOM | 3825 | CG | ARG | C | 77 | 20.774 | 9.331 | 36.403 | 1.00 | 28.10 | C |

| ATOM | 3826 | CD | ARG | C | 77 | 19.868 | 8.153 | 36.444 | 1.00 | 26.97 | C |
|---|---|---|---|---|---|---|---|---|---|---|---|
| ATOM | 3827 | NE | ARG | C | 77 | 20.454 | 7.018 | 37.108 | 1.00 | 24.76 | N |
| ATOM | 3828 | CZ | ARG | C | 77 | 21.377 | 6.225 | 36.583 | 1.00 | 26.83 | C |
| ATOM | 3829 | NH1 | ARG | C | 77 | 21.882 | 6.470 | 35.369 | 1.00 | 26.66 | N |
| ATOM | 3830 | NH2 | ARG | C | 77 | 21.840 | 5.215 | 37.301 | 1.00 | 25.62 | N |
| ATOM | 3831 | N | VAL | C | 78 | 22.823 | 12.985 | 38.980 | 1.00 | 31.88 | N |
| ATOM | 3832 | CA | VAL | C | 78 | 23.152 | 13.821 | 40.140 | 1.00 | 35.13 | C |
| ATOM | 3833 | C | VAL | C | 78 | 21.908 | 14.340 | 40.861 | 1.00 | 38.01 | C |
| ATOM | 3834 | O | VAL | C | 78 | 21.050 | 14.969 | 40.244 | 1.00 | 38.53 | O |
| ATOM | 3835 | CB | VAL | C | 78 | 24.017 | 15.042 | 39.742 | 1.00 | 34.89 | C |
| ATOM | 3836 | CG1 | VAL | C | 78 | 23.978 | 16.111 | 40.820 | 1.00 | 35.52 | C |
| ATOM | 3837 | CG2 | VAL | C | 78 | 25.474 | 14.613 | 39.490 | 1.00 | 34.11 | C |
| ATOM | 3838 | N | GLU | C | 79 | 21.860 | 14.104 | 42.170 | 1.00 | 40.81 | N |
| ATOM | 3839 | CA | GLU | C | 79 | 20.764 | 14.530 | 43.028 | 1.00 | 43.13 | C |
| ATOM | 3840 | C | GLU | C | 79 | 21.225 | 15.629 | 43.980 | 1.00 | 45.24 | C |
| ATOM | 3841 | O | GLU | C | 79 | 22.431 | 15.949 | 44.061 | 1.00 | 45.84 | O |
| ATOM | 3842 | CB | GLU | C | 79 | 20.260 | 13.344 | 43.835 | 1.00 | 43.06 | C |
| ATOM | 3843 | CG | GLU | C | 79 | 19.681 | 12.241 | 42.984 | 1.00 | 43.71 | C |
| ATOM | 3844 | CD | GLU | C | 79 | 19.238 | 11.047 | 43.799 | 1.00 | 44.42 | C |
| ATOM | 3845 | OE1 | GLU | C | 79 | 18.886 | 10.008 | 43.196 | 1.00 | 45.77 | O |
| ATOM | 3846 | OE2 | GLU | C | 79 | 19.247 | 11.140 | 45.047 | 1.00 | 46.05 | O |
| ATOM | 3847 | N | ALA | C | 80 | 20.261 | 16.193 | 44.712 | 1.00 | 45.64 | N |
| ATOM | 3848 | CA | ALA | C | 80 | 20.514 | 17.294 | 45.648 | 1.00 | 45.89 | C |
| ATOM | 3849 | C | ALA | C | 80 | 21.511 | 16.893 | 46.734 | 1.00 | 45.39 | C |
| ATOM | 3850 | O | ALA | C | 80 | 22.543 | 17.539 | 46.930 | 1.00 | 45.26 | O |
| ATOM | 3851 | CB | ALA | C | 80 | 19.172 | 17.756 | 46.283 | 1.00 | 45.95 | C |
| ATOM | 3852 | N | GLU | C | 81 | 21.178 | 15.817 | 47.432 | 1.00 | 45.50 | N |
| ATOM | 3853 | CA | GLU | C | 81 | 22.019 | 15.238 | 48.480 | 1.00 | 45.67 | C |
| ATOM | 3854 | C | GLU | C | 81 | 23.505 | 14.945 | 48.123 | 1.00 | 45.24 | C |
| ATOM | 3855 | O | GLU | C | 81 | 24.248 | 14.469 | 48.988 | 1.00 | 45.38 | O |
| ATOM | 3856 | CB | GLU | C | 81 | 21.383 | 13.930 | 48.952 | 1.00 | 46.86 | C |
| ATOM | 3857 | CG | GLU | C | 81 | 20.930 | 13.033 | 47.794 | 1.00 | 49.34 | C |
| ATOM | 3858 | CD | GLU | C | 81 | 21.540 | 11.648 | 47.822 | 1.00 | 51.88 | C |
| ATOM | 3859 | OE1 | GLU | C | 81 | 21.655 | 11.045 | 48.916 | 1.00 | 54.44 | O |
| ATOM | 3860 | OE2 | GLU | C | 81 | 21.905 | 11.162 | 46.724 | 1.00 | 53.27 | O |
| ATOM | 3861 | N | ASP | C | 82 | 23.920 | 15.187 | 46.875 | 1.00 | 44.00 | N |
| ATOM | 3862 | CA | ASP | C | 82 | 25.281 | 14.860 | 46.410 | 1.00 | 42.70 | C |
| ATOM | 3863 | C | ASP | C | 82 | 26.187 | 16.063 | 46.536 | 1.00 | 41.31 | C |
| ATOM | 3864 | O | ASP | C | 82 | 27.400 | 15.949 | 46.383 | 1.00 | 41.03 | O |
| ATOM | 3865 | CB | ASP | C | 82 | 25.290 | 14.433 | 44.924 | 1.00 | 41.69 | C |
| ATOM | 3866 | CG | ASP | C | 82 | 24.487 | 13.186 | 44.653 | 1.00 | 40.76 | C |
| ATOM | 3867 | OD1 | ASP | C | 82 | 23.998 | 13.055 | 43.517 | 1.00 | 40.59 | O |
| ATOM | 3868 | OD2 | ASP | C | 82 | 24.351 | 12.321 | 45.545 | 1.00 | 40.10 | O |
| ATOM | 3869 | N | VAL | C | 83 | 25.595 | 17.224 | 46.779 | 1.00 | 40.26 | N |
| ATOM | 3870 | CA | VAL | C | 83 | 26.346 | 18.466 | 46.880 | 1.00 | 39.71 | C |
| ATOM | 3871 | C | VAL | C | 83 | 27.291 | 18.449 | 48.102 | 1.00 | 38.23 | C |
| ATOM | 3872 | O | VAL | C | 83 | 26.954 | 17.921 | 49.158 | 1.00 | 38.35 | O |
| ATOM | 3873 | CB | VAL | C | 83 | 25.371 | 19.659 | 46.964 | 1.00 | 39.95 | C |
| ATOM | 3874 | CG1 | VAL | C | 83 | 26.105 | 20.962 | 47.300 | 1.00 | 40.08 | C |
| ATOM | 3875 | CG2 | VAL | C | 83 | 24.596 | 19.778 | 45.656 | 1.00 | 40.51 | C |
| ATOM | 3876 | N | GLY | C | 84 | 28.470 | 19.020 | 47.938 | 1.00 | 37.09 | N |
| ATOM | 3877 | CA | GLY | C | 84 | 29.455 | 19.078 | 49.015 | 1.00 | 36.18 | C |
| ATOM | 3878 | C | GLY | C | 84 | 30.843 | 19.287 | 48.460 | 1.00 | 35.38 | C |
| ATOM | 3879 | O | GLY | C | 84 | 30.997 | 19.715 | 47.315 | 1.00 | 35.90 | O |
| ATOM | 3880 | N | ILE | C | 85 | 31.854 | 18.993 | 49.278 | 1.00 | 34.19 | N |
| ATOM | 3881 | CA | ILE | C | 85 | 33.234 | 19.035 | 48.827 | 1.00 | 33.32 | C |
| ATOM | 3882 | C | ILE | C | 85 | 33.784 | 17.616 | 48.746 | 1.00 | 31.47 | C |
| ATOM | 3883 | O | ILE | C | 85 | 33.609 | 16.818 | 49.667 | 1.00 | 31.05 | O |
| ATOM | 3884 | CB | ILE | C | 85 | 34.091 | 19.912 | 49.734 | 1.00 | 34.22 | C |
| ATOM | 3885 | CG1 | ILE | C | 85 | 33.496 | 21.340 | 49.729 | 1.00 | 34.86 | C |
| ATOM | 3886 | CG2 | ILE | C | 85 | 35.551 | 19.885 | 49.238 | 1.00 | 34.76 | C |
| ATOM | 3887 | CD1 | ILE | C | 85 | 34.343 | 22.437 | 50.375 | 1.00 | 34.72 | C |
| ATOM | 3888 | N | TYR | C | 86 | 34.435 | 17.311 | 47.622 | 1.00 | 29.79 | N |
| ATOM | 3889 | CA | TYR | C | 86 | 34.928 | 15.958 | 47.330 | 1.00 | 28.11 | C |
| ATOM | 3890 | C | TYR | C | 86 | 36.442 | 15.978 | 47.312 | 1.00 | 26.39 | C |

| ATOM | 3891 | O | TYR | C | 86 | 37.032 | 16.665 | 46.485 | 1.00 | 26.92 | O |
|------|------|-----|-----|---|----|--------|--------|--------|------|-------|---|
| ATOM | 3892 | CB | TYR | C | 86 | 34.406 | 15.456 | 45.965 | 1.00 | 27.21 | C |
| ATOM | 3893 | CG | TYR | C | 86 | 32.938 | 15.087 | 45.969 | 1.00 | 27.56 | C |
| ATOM | 3894 | CD1 | TYR | C | 86 | 31.962 | 16.045 | 45.736 | 1.00 | 27.86 | C |
| ATOM | 3895 | CD2 | TYR | C | 86 | 32.527 | 13.790 | 46.229 | 1.00 | 27.92 | C |
| ATOM | 3896 | CE1 | TYR | C | 86 | 30.616 | 15.718 | 45.743 | 1.00 | 27.88 | C |
| ATOM | 3897 | CE2 | TYR | C | 86 | 31.169 | 13.449 | 46.231 | 1.00 | 27.85 | C |
| ATOM | 3898 | CZ | TYR | C | 86 | 30.232 | 14.434 | 45.993 | 1.00 | 27.17 | C |
| ATOM | 3899 | OH | TYR | C | 86 | 28.899 | 14.129 | 45.992 | 1.00 | 29.98 | O |
| ATOM | 3900 | N | TYR | C | 87 | 37.056 | 15.207 | 48.209 | 1.00 | 25.04 | N |
| ATOM | 3901 | CA | TYR | C | 87 | 38.508 | 15.211 | 48.386 | 1.00 | 23.89 | C |
| ATOM | 3902 | C | TYR | C | 87 | 39.072 | 13.850 | 48.015 | 1.00 | 23.13 | C |
| ATOM | 3903 | O | TYR | C | 87 | 38.512 | 12.802 | 48.392 | 1.00 | 21.15 | O |
| ATOM | 3904 | CB | TYR | C | 87 | 38.915 | 15.462 | 49.836 | 1.00 | 24.64 | C |
| ATOM | 3905 | CG | TYR | C | 87 | 38.479 | 16.765 | 50.429 | 1.00 | 26.39 | C |
| ATOM | 3906 | CD1 | TYR | C | 87 | 39.280 | 17.908 | 50.327 | 1.00 | 26.67 | C |
| ATOM | 3907 | CD2 | TYR | C | 87 | 37.298 | 16.855 | 51.150 | 1.00 | 27.54 | C |
| ATOM | 3908 | CE1 | TYR | C | 87 | 38.879 | 19.111 | 50.906 | 1.00 | 25.83 | C |
| ATOM | 3909 | CE2 | TYR | C | 87 | 36.907 | 18.056 | 51.744 | 1.00 | 27.08 | C |
| ATOM | 3910 | CZ | TYR | C | 87 | 37.693 | 19.162 | 51.604 | 1.00 | 25.67 | C |
| ATOM | 3911 | OH | TYR | C | 87 | 37.263 | 20.345 | 52.155 | 1.00 | 29.75 | O |
| ATOM | 3912 | N | CYS | C | 88 | 40.192 | 13.880 | 47.299 | 1.00 | 21.68 | N |
| ATOM | 3913 | CA | CYS | C | 88 | 40.971 | 12.692 | 47.055 | 1.00 | 21.39 | C |
| ATOM | 3914 | C | CYS | C | 88 | 42.155 | 12.644 | 48.043 | 1.00 | 20.27 | C |
| ATOM | 3915 | O | CYS | C | 88 | 42.534 | 13.648 | 48.668 | 1.00 | 19.96 | O |
| ATOM | 3916 | CB | CYS | C | 88 | 41.405 | 12.647 | 45.589 | 1.00 | 22.98 | C |
| ATOM | 3917 | SG | CYS | C | 88 | 42.387 | 14.060 | 45.052 | 1.00 | 28.02 | S |
| ATOM | 3918 | N | ALA | C | 89 | 42.697 | 11.458 | 48.219 | 1.00 | 19.89 | N |
| ATOM | 3919 | CA | ALA | C | 89 | 43.762 | 11.210 | 49.166 | 1.00 | 19.49 | C |
| ATOM | 3920 | C | ALA | C | 89 | 44.475 | 9.938 | 48.725 | 1.00 | 19.00 | C |
| ATOM | 3921 | O | ALA | C | 89 | 43.931 | 9.134 | 48.002 | 1.00 | 19.52 | O |
| ATOM | 3922 | CB | ALA | C | 89 | 43.195 | 11.053 | 50.601 | 1.00 | 20.50 | C |
| ATOM | 3923 | N | HIS | C | 90 | 45.710 | 9.775 | 49.147 | 1.00 | 21.06 | N |
| ATOM | 3924 | CA | HIS | C | 90 | 46.467 | 8.593 | 48.834 | 1.00 | 22.56 | C |
| ATOM | 3925 | C | HIS | C | 90 | 46.821 | 7.928 | 50.141 | 1.00 | 24.39 | C |
| ATOM | 3926 | O | HIS | C | 90 | 46.747 | 8.547 | 51.212 | 1.00 | 23.36 | O |
| ATOM | 3927 | CB | HIS | C | 90 | 47.764 | 8.949 | 48.082 | 1.00 | 22.97 | C |
| ATOM | 3928 | CG | HIS | C | 90 | 48.820 | 9.556 | 48.952 | 1.00 | 21.59 | C |
| ATOM | 3929 | ND1 | HIS | C | 90 | 49.110 | 10.903 | 48.952 | 1.00 | 24.62 | N |
| ATOM | 3930 | CD2 | HIS | C | 90 | 49.639 | 9.000 | 49.863 | 1.00 | 20.19 | C |
| ATOM | 3931 | CE1 | HIS | C | 90 | 50.067 | 11.145 | 49.827 | 1.00 | 22.29 | C |
| ATOM | 3932 | NE2 | HIS | C | 90 | 50.403 | 10.005 | 50.395 | 1.00 | 23.32 | N |
| ATOM | 3933 | N | ASN | C | 91 | 47.288 | 6.701 | 50.002 | 1.00 | 27.05 | N |
| ATOM | 3934 | CA | ASN | C | 91 | 47.642 | 5.829 | 51.099 | 1.00 | 30.75 | C |
| ATOM | 3935 | C | ASN | C | 91 | 48.938 | 5.090 | 50.791 | 1.00 | 31.41 | C |
| ATOM | 3936 | O | ASN | C | 91 | 48.976 | 3.873 | 50.932 | 1.00 | 34.32 | O |
| ATOM | 3937 | CB | ASN | C | 91 | 46.523 | 4.789 | 51.264 | 1.00 | 30.43 | C |
| ATOM | 3938 | CG | ASN | C | 91 | 46.605 | 4.070 | 52.541 | 1.00 | 31.95 | C |
| ATOM | 3939 | OD1 | ASN | C | 91 | 47.289 | 4.507 | 53.438 | 1.00 | 35.08 | O |
| ATOM | 3940 | ND2 | ASN | C | 91 | 45.912 | 2.951 | 52.648 | 1.00 | 33.29 | N |
| ATOM | 3941 | N | VAL | C | 92 | 49.975 | 5.798 | 50.352 | 1.00 | 30.70 | N |
| ATOM | 3942 | CA | VAL | C | 92 | 51.233 | 5.140 | 49.934 | 1.00 | 30.26 | C |
| ATOM | 3943 | C | VAL | C | 92 | 52.469 | 5.523 | 50.754 | 1.00 | 30.32 | C |
| ATOM | 3944 | O | VAL | C | 92 | 53.449 | 4.778 | 50.797 | 1.00 | 32.82 | O |
| ATOM | 3945 | CB | VAL | C | 92 | 51.516 | 5.347 | 48.406 | 1.00 | 29.74 | C |
| ATOM | 3946 | CG1 | VAL | C | 92 | 50.366 | 4.825 | 47.591 | 1.00 | 30.49 | C |
| ATOM | 3947 | CG2 | VAL | C | 92 | 51.823 | 6.798 | 48.073 | 1.00 | 29.16 | C |
| ATOM | 3948 | N | GLU | C | 93 | 52.438 | 6.671 | 51.401 | 1.00 | 28.79 | N |
| ATOM | 3949 | CA | GLU | C | 93 | 53.565 | 7.112 | 52.183 | 1.00 | 28.98 | C |
| ATOM | 3950 | C | GLU | C | 93 | 53.121 | 8.138 | 53.181 | 1.00 | 27.45 | C |
| ATOM | 3951 | O | GLU | C | 93 | 51.941 | 8.473 | 53.232 | 1.00 | 26.45 | O |
| ATOM | 3952 | CB | GLU | C | 93 | 54.628 | 7.738 | 51.267 | 1.00 | 29.00 | C |
| ATOM | 3953 | CG | GLU | C | 93 | 54.185 | 8.941 | 50.503 | 1.00 | 28.87 | C |
| ATOM | 3954 | CD | GLU | C | 93 | 55.330 | 9.568 | 49.701 | 1.00 | 31.32 | C |
| ATOM | 3955 | OE1 | GLU | C | 93 | 55.891 | 8.878 | 48.828 | 1.00 | 33.65 | O |

| | | | | | | | | | | |
|---|---|---|---|---|---|---|---|---|---|---|
| ATOM | 3956 | OE2 | GLU | C | 93 | 55.657 | 10.757 | 49.940 | 1.00 33.93 | O |
| ATOM | 3957 | N | LEU | C | 94 | 54.089 | 8.616 | 53.967 | 1.00 27.28 | N |
| ATOM | 3958 | CA | LEU | C | 94 | 53.961 | 9.789 | 54.825 | 1.00 25.93 | C |
| ATOM | 3959 | C | LEU | C | 94 | 54.834 | 10.897 | 54.264 | 1.00 26.58 | C |
| ATOM | 3960 | O | LEU | C | 94 | 55.914 | 10.626 | 53.790 | 1.00 27.10 | O |
| ATOM | 3961 | CB | LEU | C | 94 | 54.419 | 9.480 | 56.238 | 1.00 26.05 | C |
| ATOM | 3962 | CG | LEU | C | 94 | 53.613 | 8.448 | 57.016 | 1.00 26.63 | C |
| ATOM | 3963 | CD1 | LEU | C | 94 | 54.113 | 8.395 | 58.440 | 1.00 26.68 | C |
| ATOM | 3964 | CD2 | LEU | C | 94 | 52.167 | 8.748 | 56.982 | 1.00 25.58 | C |
| ATOM | 3965 | N | PRO | C | 95 | 54.377 | 12.149 | 54.313 | 1.00 26.40 | N |
| ATOM | 3966 | CA | PRO | C | 95 | 53.080 | 12.589 | 54.820 | 1.00 24.69 | C |
| ATOM | 3967 | C | PRO | C | 95 | 51.888 | 12.156 | 53.974 | 1.00 24.34 | C |
| ATOM | 3968 | O | PRO | C | 95 | 51.960 | 12.031 | 52.742 | 1.00 23.23 | O |
| ATOM | 3969 | CB | PRO | C | 95 | 53.202 | 14.115 | 54.831 | 1.00 24.86 | C |
| ATOM | 3970 | CG | PRO | C | 95 | 54.229 | 14.424 | 53.761 | 1.00 25.84 | C |
| ATOM | 3971 | CD | PRO | C | 95 | 55.203 | 13.277 | 53.846 | 1.00 27.16 | C |
| ATOM | 3972 | N | ARG | C | 96 | 50.790 | 11.897 | 54.671 | 1.00 21.93 | N |
| ATOM | 3973 | CA | ARG | C | 96 | 49.494 | 11.800 | 54.038 | 1.00 22.30 | C |
| ATOM | 3974 | C | ARG | C | 96 | 49.133 | 13.152 | 53.449 | 1.00 19.15 | C |
| ATOM | 3975 | O | ARG | C | 96 | 49.199 | 14.183 | 54.120 | 1.00 20.72 | O |
| ATOM | 3976 | CB | ARG | C | 96 | 48.424 | 11.418 | 55.071 | 1.00 22.17 | C |
| ATOM | 3977 | CG | ARG | C | 96 | 48.764 | 10.163 | 55.814 | 1.00 23.23 | C |
| ATOM | 3978 | CD | ARG | C | 96 | 47.582 | 9.659 | 56.625 | 1.00 24.90 | C |
| ATOM | 3979 | NE | ARG | C | 96 | 47.983 | 8.470 | 57.378 | 1.00 26.18 | N |
| ATOM | 3980 | CZ | ARG | C | 96 | 48.048 | 7.252 | 56.854 | 1.00 28.39 | C |
| ATOM | 3981 | NH1 | ARG | C | 96 | 47.684 | 7.047 | 55.602 | 1.00 31.49 | N |
| ATOM | 3982 | NH2 | ARG | C | 96 | 48.455 | 6.217 | 57.591 | 1.00 28.41 | N |
| ATOM | 3983 | N | THR | C | 97 | 48.727 | 13.147 | 52.198 | 1.00 18.02 | N |
| ATOM | 3984 | CA | THR | C | 97 | 48.269 | 14.348 | 51.591 | 1.00 18.87 | C |
| ATOM | 3985 | C | THR | C | 97 | 46.946 | 14.130 | 50.920 | 1.00 17.12 | C |
| ATOM | 3986 | O | THR | C | 97 | 46.622 | 13.025 | 50.535 | 1.00 18.67 | O |
| ATOM | 3987 | CB | THR | C | 97 | 49.319 | 14.930 | 50.618 | 1.00 18.84 | C |
| ATOM | 3988 | OG1 | THR | C | 97 | 49.610 | 13.970 | 49.635 | 1.00 17.46 | O |
| ATOM | 3989 | CG2 | THR | C | 97 | 50.571 | 15.220 | 51.361 | 1.00 21.32 | C |
| ATOM | 3990 | N | PHE | C | 98 | 46.203 | 15.229 | 50.825 | 1.00 18.26 | N |
| ATOM | 3991 | CA | PHE | C | 98 | 44.873 | 15.296 | 50.267 | 1.00 19.78 | C |
| ATOM | 3992 | C | PHE | C | 98 | 44.817 | 16.310 | 49.146 | 1.00 21.04 | C |
| ATOM | 3993 | O | PHE | C | 98 | 45.578 | 17.269 | 49.133 | 1.00 21.29 | O |
| ATOM | 3994 | CB | PHE | C | 98 | 43.905 | 15.783 | 51.336 | 1.00 20.15 | C |
| ATOM | 3995 | CG | PHE | C | 98 | 43.862 | 14.907 | 52.537 | 1.00 20.67 | C |
| ATOM | 3996 | CD1 | PHE | C | 98 | 44.742 | 15.105 | 53.575 | 1.00 20.33 | C |
| ATOM | 3997 | CD2 | PHE | C | 98 | 42.940 | 13.875 | 52.624 | 1.00 22.47 | C |
| ATOM | 3998 | CE1 | PHE | C | 98 | 44.730 | 14.310 | 54.659 | 1.00 19.80 | C |
| ATOM | 3999 | CE2 | PHE | C | 98 | 42.925 | 13.061 | 53.737 | 1.00 20.72 | C |
| ATOM | 4000 | CZ | PHE | C | 98 | 43.822 | 13.275 | 54.740 | 1.00 22.35 | C |
| ATOM | 4001 | N | GLY | C | 99 | 43.898 | 16.097 | 48.220 | 1.00 21.94 | N |
| ATOM | 4002 | CA | GLY | C | 99 | 43.555 | 17.114 | 47.250 | 1.00 23.40 | C |
| ATOM | 4003 | C | GLY | C | 99 | 42.927 | 18.337 | 47.907 | 1.00 24.37 | C |
| ATOM | 4004 | O | GLY | C | 99 | 42.416 | 18.280 | 49.041 | 1.00 22.01 | O |
| ATOM | 4005 | N | GLY | C | 100 | 42.982 | 19.459 | 47.191 | 1.00 25.69 | N |
| ATOM | 4006 | CA | GLY | C | 100 | 42.424 | 20.719 | 47.683 | 1.00 26.70 | C |
| ATOM | 4007 | C | GLY | C | 100 | 40.921 | 20.679 | 47.910 | 1.00 28.22 | C |
| ATOM | 4008 | O | GLY | C | 100 | 40.373 | 21.550 | 48.587 | 1.00 29.64 | O |
| ATOM | 4009 | N | GLY | C | 101 | 40.232 | 19.703 | 47.323 | 1.00 28.40 | N |
| ATOM | 4010 | CA | GLY | C | 101 | 38.776 | 19.600 | 47.480 | 1.00 28.60 | C |
| ATOM | 4011 | C | GLY | C | 101 | 38.070 | 20.257 | 46.304 | 1.00 29.32 | C |
| ATOM | 4012 | O | GLY | C | 101 | 38.479 | 21.332 | 45.870 | 1.00 30.31 | O |
| ATOM | 4013 | N | THR | C | 102 | 37.053 | 19.578 | 45.766 | 1.00 29.48 | N |
| ATOM | 4014 | CA | THR | C | 102 | 36.223 | 20.090 | 44.670 | 1.00 29.01 | C |
| ATOM | 4015 | C | THR | C | 102 | 34.767 | 20.125 | 45.119 | 1.00 29.21 | C |
| ATOM | 4016 | O | THR | C | 102 | 34.182 | 19.107 | 45.499 | 1.00 27.63 | O |
| ATOM | 4017 | CB | THR | C | 102 | 36.292 | 19.239 | 43.379 | 1.00 28.93 | C |
| ATOM | 4018 | OG1 | THR | C | 102 | 37.645 | 18.978 | 43.003 | 1.00 27.42 | O |
| ATOM | 4019 | CG2 | THR | C | 102 | 35.582 | 19.951 | 42.228 | 1.00 29.43 | C |
| ATOM | 4020 | N | LYS | C | 103 | 34.188 | 21.321 | 45.076 | 1.00 31.03 | N |

| ATOM | 4021 | CA | LYS C 103 | 32.800 | 21.503 | 45.426 | 1.00 | 33.76 | C |
|---|---|---|---|---|---|---|---|---|---|
| ATOM | 4022 | C | LYS C 103 | 31.930 | 21.173 | 44.197 | 1.00 | 32.81 | C |
| ATOM | 4023 | O | LYS C 103 | 32.148 | 21.706 | 43.122 | 1.00 | 33.02 | O |
| ATOM | 4024 | CB | LYS C 103 | 32.580 | 22.953 | 45.885 | 1.00 | 34.71 | C |
| ATOM | 4025 | CG | LYS C 103 | 31.309 | 23.191 | 46.666 | 1.00 | 37.13 | C |
| ATOM | 4026 | CD | LYS C 103 | 31.184 | 24.662 | 47.047 | 1.00 | 37.64 | C |
| ATOM | 4027 | CE | LYS C 103 | 32.243 | 25.143 | 48.040 | 1.00 | 39.67 | C |
| ATOM | 4028 | NZ | LYS C 103 | 31.934 | 26.541 | 48.524 | 1.00 | 40.28 | N |
| ATOM | 4029 | N | LEU C 104 | 30.975 | 20.267 | 44.365 | 1.00 | 33.55 | N |
| ATOM | 4030 | CA | LEU C 104 | 29.989 | 19.968 | 43.314 | 1.00 | 34.29 | C |
| ATOM | 4031 | C | LEU C 104 | 29.014 | 21.155 | 43.117 | 1.00 | 34.61 | C |
| ATOM | 4032 | O | LEU C 104 | 28.249 | 21.478 | 44.038 | 1.00 | 33.40 | O |
| ATOM | 4033 | CB | LEU C 104 | 29.179 | 18.716 | 43.698 | 1.00 | 34.26 | C |
| ATOM | 4034 | CG | LEU C 104 | 28.131 | 18.199 | 42.696 | 1.00 | 33.63 | C |
| ATOM | 4035 | CD1 | LEU C 104 | 28.781 | 17.784 | 41.395 | 1.00 | 33.03 | C |
| ATOM | 4036 | CD2 | LEU C 104 | 27.346 | 17.048 | 43.281 | 1.00 | 33.96 | C |
| ATOM | 4037 | N | GLU C 105 | 29.061 | 21.794 | 41.941 | 1.00 | 34.61 | N |
| ATOM | 4038 | CA | GLU C 105 | 28.108 | 22.854 | 41.553 | 1.00 | 35.79 | C |
| ATOM | 4039 | C | GLU C 105 | 26.878 | 22.263 | 40.790 | 1.00 | 35.49 | C |
| ATOM | 4040 | O | GLU C 105 | 27.028 | 21.449 | 39.877 | 1.00 | 33.53 | O |
| ATOM | 4041 | CB | GLU C 105 | 28.809 | 23.933 | 40.695 | 1.00 | 36.66 | C |
| ATOM | 4042 | CG | GLU C 105 | 29.869 | 24.779 | 41.445 | 1.00 | 38.62 | C |
| ATOM | 4043 | CD | GLU C 105 | 30.593 | 25.830 | 40.559 | 1.00 | 39.37 | C |
| ATOM | 4044 | OE1 | GLU C 105 | 30.749 | 25.630 | 39.320 | 1.00 | 42.60 | O |
| ATOM | 4045 | OE2 | GLU C 105 | 31.024 | 26.863 | 41.119 | 1.00 | 40.92 | O |
| ATOM | 4046 | N | ILE C 106 | 25.673 | 22.654 | 41.207 | 1.00 | 34.75 | N |
| ATOM | 4047 | CA | ILE C 106 | 24.442 | 22.245 | 40.549 | 1.00 | 35.24 | C |
| ATOM | 4048 | C | ILE C 106 | 24.002 | 23.328 | 39.551 | 1.00 | 35.50 | C |
| ATOM | 4049 | O | ILE C 106 | 24.113 | 24.521 | 39.830 | 1.00 | 34.61 | O |
| ATOM | 4050 | CB | ILE C 106 | 23.339 | 21.960 | 41.587 | 1.00 | 35.14 | C |
| ATOM | 4051 | CG1 | ILE C 106 | 23.755 | 20.798 | 42.505 | 1.00 | 35.69 | C |
| ATOM | 4052 | CG2 | ILE C 106 | 21.982 | 21.697 | 40.912 | 1.00 | 35.32 | C |
| ATOM | 4053 | CD1 | ILE C 106 | 24.049 | 19.469 | 41.796 | 1.00 | 35.17 | C |
| ATOM | 4054 | N | LYS C 107 | 23.547 | 22.896 | 38.373 | 1.00 | 36.37 | N |
| ATOM | 4055 | CA | LYS C 107 | 23.026 | 23.804 | 37.346 | 1.00 | 36.94 | C |
| ATOM | 4056 | C | LYS C 107 | 21.509 | 23.872 | 37.393 | 1.00 | 35.56 | C |
| ATOM | 4057 | O | LYS C 107 | 20.833 | 22.856 | 37.562 | 1.00 | 36.01 | O |
| ATOM | 4058 | CB | LYS C 107 | 23.446 | 23.362 | 35.950 | 1.00 | 37.44 | C |
| ATOM | 4059 | CG | LYS C 107 | 22.930 | 24.240 | 34.830 | 1.00 | 37.90 | C |
| ATOM | 4060 | CD | LYS C 107 | 23.708 | 23.954 | 33.568 | 1.00 | 38.78 | C |
| ATOM | 4061 | CE | LYS C 107 | 23.211 | 24.725 | 32.355 | 1.00 | 40.26 | C |
| ATOM | 4062 | NZ | LYS C 107 | 23.725 | 24.071 | 31.101 | 1.00 | 41.29 | N |
| ATOM | 4063 | N | ARG C 108 | 20.992 | 25.082 | 37.222 | 1.00 | 35.41 | N |
| ATOM | 4064 | CA | ARG C 108 | 19.558 | 25.305 | 37.160 | 1.00 | 36.40 | C |
| ATOM | 4065 | C | ARG C 108 | 19.268 | 26.485 | 36.234 | 1.00 | 35.99 | C |
| ATOM | 4066 | O | ARG C 108 | 20.188 | 27.123 | 35.724 | 1.00 | 36.62 | O |
| ATOM | 4067 | CB | ARG C 108 | 18.989 | 25.516 | 38.570 | 1.00 | 36.63 | C |
| ATOM | 4068 | CG | ARG C 108 | 19.708 | 26.611 | 39.378 | 1.00 | 37.66 | C |
| ATOM | 4069 | CD | ARG C 108 | 18.805 | 27.773 | 39.605 | 1.00 | 39.13 | C |
| ATOM | 4070 | NE | ARG C 108 | 17.675 | 27.352 | 40.411 | 1.00 | 40.95 | N |
| ATOM | 4071 | CZ | ARG C 108 | 16.465 | 27.889 | 40.374 | 1.00 | 39.46 | C |
| ATOM | 4072 | NH1 | ARG C 108 | 16.160 | 28.867 | 39.534 | 1.00 | 41.19 | N |
| ATOM | 4073 | NH2 | ARG C 108 | 15.546 | 27.410 | 41.174 | 1.00 | 39.30 | N |
| ATOM | 4074 | N | ALA C 109 | 17.984 | 26.718 | 35.982 | 1.00 | 36.98 | N |
| ATOM | 4075 | CA | ALA C 109 | 17.539 | 27.815 | 35.133 | 1.00 | 37.04 | C |
| ATOM | 4076 | C | ALA C 109 | 17.907 | 29.123 | 35.818 | 1.00 | 37.06 | C |
| ATOM | 4077 | O | ALA C 109 | 17.884 | 29.217 | 37.044 | 1.00 | 36.45 | O |
| ATOM | 4078 | CB | ALA C 109 | 16.009 | 27.730 | 34.891 | 1.00 | 37.75 | C |
| ATOM | 4079 | N | ASP C 110 | 18.282 | 30.122 | 35.027 | 1.00 | 36.94 | N |
| ATOM | 4080 | CA | ASP C 110 | 18.721 | 31.393 | 35.577 | 1.00 | 37.19 | C |
| ATOM | 4081 | C | ASP C 110 | 17.621 | 32.018 | 36.430 | 1.00 | 36.31 | C |
| ATOM | 4082 | O | ASP C 110 | 16.438 | 31.736 | 36.229 | 1.00 | 35.28 | O |
| ATOM | 4083 | CB | ASP C 110 | 19.144 | 32.353 | 34.468 | 1.00 | 38.02 | C |
| ATOM | 4084 | CG | ASP C 110 | 20.333 | 31.836 | 33.658 | 1.00 | 39.41 | C |
| ATOM | 4085 | OD1 | ASP C 110 | 20.735 | 30.658 | 33.789 | 1.00 | 40.91 | O |

| ATOM | 4086 | OD2 | ASP | C | 110 | 20.878 | 32.631 | 32.874 | 1.00 | 41.29 | O |
|------|------|-----|-----|---|-----|--------|--------|--------|------|-------|---|
| ATOM | 4087 | N | ALA | C | 111 | 18.028 | 32.818 | 37.417 | 1.00 | 35.25 | N |
| ATOM | 4088 | CA | ALA | C | 111 | 17.090 | 33.487 | 38.311 | 1.00 | 34.26 | C |
| ATOM | 4089 | C | ALA | C | 111 | 17.647 | 34.843 | 38.646 | 1.00 | 33.38 | C |
| ATOM | 4090 | O | ALA | C | 111 | 18.832 | 34.979 | 38.959 | 1.00 | 33.41 | O |
| ATOM | 4091 | CB | ALA | C | 111 | 16.849 | 32.666 | 39.573 | 1.00 | 34.56 | C |
| ATOM | 4092 | N | ALA | C | 112 | 16.814 | 35.870 | 38.502 | 1.00 | 33.87 | N |
| ATOM | 4093 | CA | ALA | C | 112 | 17.199 | 37.227 | 38.872 | 1.00 | 32.94 | C |
| ATOM | 4094 | C | ALA | C | 112 | 17.144 | 37.294 | 40.381 | 1.00 | 32.07 | C |
| ATOM | 4095 | O | ALA | C | 112 | 16.287 | 36.653 | 40.986 | 1.00 | 31.65 | O |
| ATOM | 4096 | CB | ALA | C | 112 | 16.238 | 38.260 | 38.263 | 1.00 | 33.58 | C |
| ATOM | 4097 | N | PRO | C | 113 | 18.054 | 38.066 | 41.000 | 1.00 | 31.80 | N |
| ATOM | 4098 | CA | PRO | C | 113 | 18.042 | 38.224 | 42.448 | 1.00 | 31.42 | C |
| ATOM | 4099 | C | PRO | C | 113 | 16.903 | 39.081 | 42.959 | 1.00 | 31.93 | C |
| ATOM | 4100 | O | PRO | C | 113 | 16.521 | 40.035 | 42.293 | 1.00 | 32.53 | O |
| ATOM | 4101 | CB | PRO | C | 113 | 19.358 | 38.947 | 42.720 | 1.00 | 32.33 | C |
| ATOM | 4102 | CG | PRO | C | 113 | 19.651 | 39.668 | 41.461 | 1.00 | 31.84 | C |
| ATOM | 4103 | CD | PRO | C | 113 | 19.167 | 38.804 | 40.380 | 1.00 | 30.85 | C |
| ATOM | 4104 | N | THR | C | 114 | 16.368 | 38.722 | 44.131 | 1.00 | 30.61 | N |
| ATOM | 4105 | CA | THR | C | 114 | 15.540 | 39.617 | 44.919 | 1.00 | 30.73 | C |
| ATOM | 4106 | C | THR | C | 114 | 16.451 | 40.481 | 45.775 | 1.00 | 29.91 | C |
| ATOM | 4107 | O | THR | C | 114 | 17.227 | 39.974 | 46.599 | 1.00 | 29.37 | O |
| ATOM | 4108 | CB | THR | C | 114 | 14.583 | 38.860 | 45.835 | 1.00 | 30.95 | C |
| ATOM | 4109 | OG1 | THR | C | 114 | 13.842 | 37.918 | 45.064 | 1.00 | 33.24 | O |
| ATOM | 4110 | CG2 | THR | C | 114 | 13.622 | 39.830 | 46.522 | 1.00 | 30.65 | C |
| ATOM | 4111 | N | VAL | C | 115 | 16.347 | 41.795 | 45.582 | 1.00 | 30.38 | N |
| ATOM | 4112 | CA | VAL | C | 115 | 17.278 | 42.737 | 46.166 | 1.00 | 28.63 | C |
| ATOM | 4113 | C | VAL | C | 115 | 16.549 | 43.554 | 47.227 | 1.00 | 28.33 | C |
| ATOM | 4114 | O | VAL | C | 115 | 15.488 | 44.129 | 46.953 | 1.00 | 28.25 | O |
| ATOM | 4115 | CB | VAL | C | 115 | 17.864 | 43.630 | 45.064 | 1.00 | 29.49 | C |
| ATOM | 4116 | CG1 | VAL | C | 115 | 18.862 | 44.610 | 45.608 | 1.00 | 28.83 | C |
| ATOM | 4117 | CG2 | VAL | C | 115 | 18.505 | 42.761 | 43.992 | 1.00 | 29.95 | C |
| ATOM | 4118 | N | SER | C | 116 | 17.108 | 43.587 | 48.441 | 1.00 | 27.06 | N |
| ATOM | 4119 | CA | SER | C | 116 | 16.578 | 44.410 | 49.529 | 1.00 | 26.35 | C |
| ATOM | 4120 | C | SER | C | 116 | 17.644 | 45.335 | 50.046 | 1.00 | 25.89 | C |
| ATOM | 4121 | O | SER | C | 116 | 18.764 | 44.901 | 50.255 | 1.00 | 26.30 | O |
| ATOM | 4122 | CB | SER | C | 116 | 16.102 | 43.518 | 50.682 | 1.00 | 25.86 | C |
| ATOM | 4123 | OG | SER | C | 116 | 15.005 | 42.761 | 50.231 | 1.00 | 27.66 | O |
| ATOM | 4124 | N | ILE | C | 117 | 17.301 | 46.598 | 50.295 | 1.00 | 25.72 | N |
| ATOM | 4125 | CA | ILE | C | 117 | 18.245 | 47.521 | 50.931 | 1.00 | 25.48 | C |
| ATOM | 4126 | C | ILE | C | 117 | 17.763 | 47.970 | 52.313 | 1.00 | 24.43 | C |
| ATOM | 4127 | O | ILE | C | 117 | 16.583 | 48.223 | 52.500 | 1.00 | 24.04 | O |
| ATOM | 4128 | CB | ILE | C | 117 | 18.543 | 48.751 | 50.025 | 1.00 | 26.20 | C |
| ATOM | 4129 | CG1 | ILE | C | 117 | 19.801 | 49.464 | 50.499 | 1.00 | 25.98 | C |
| ATOM | 4130 | CG2 | ILE | C | 117 | 17.350 | 49.685 | 49.991 | 1.00 | 26.77 | C |
| ATOM | 4131 | CD1 | ILE | C | 117 | 20.181 | 50.608 | 49.642 | 1.00 | 27.52 | C |
| ATOM | 4132 | N | PHE | C | 118 | 18.694 | 48.053 | 53.268 | 1.00 | 24.50 | N |
| ATOM | 4133 | CA | PHE | C | 118 | 18.390 | 48.436 | 54.653 | 1.00 | 24.67 | C |
| ATOM | 4134 | C | PHE | C | 118 | 19.255 | 49.618 | 55.150 | 1.00 | 24.55 | C |
| ATOM | 4135 | O | PHE | C | 118 | 20.500 | 49.568 | 55.089 | 1.00 | 23.65 | O |
| ATOM | 4136 | CB | PHE | C | 118 | 18.517 | 47.217 | 55.581 | 1.00 | 24.80 | C |
| ATOM | 4137 | CG | PHE | C | 118 | 17.682 | 46.050 | 55.136 | 1.00 | 24.32 | C |
| ATOM | 4138 | CD1 | PHE | C | 118 | 18.142 | 45.188 | 54.168 | 1.00 | 25.04 | C |
| ATOM | 4139 | CD2 | PHE | C | 118 | 16.407 | 45.851 | 55.646 | 1.00 | 25.46 | C |
| ATOM | 4140 | CE1 | PHE | C | 118 | 17.364 | 44.124 | 53.714 | 1.00 | 25.14 | C |
| ATOM | 4141 | CE2 | PHE | C | 118 | 15.605 | 44.790 | 55.186 | 1.00 | 24.73 | C |
| ATOM | 4142 | CZ | PHE | C | 118 | 16.081 | 43.926 | 54.235 | 1.00 | 24.60 | C |
| ATOM | 4143 | N | PRO | C | 119 | 18.597 | 50.680 | 55.667 | 1.00 | 24.12 | N |
| ATOM | 4144 | CA | PRO | C | 119 | 19.353 | 51.777 | 56.296 | 1.00 | 24.96 | C |
| ATOM | 4145 | C | PRO | C | 119 | 19.970 | 51.366 | 57.627 | 1.00 | 24.41 | C |
| ATOM | 4146 | O | PRO | C | 119 | 19.550 | 50.349 | 58.215 | 1.00 | 24.42 | O |
| ATOM | 4147 | CB | PRO | C | 119 | 18.301 | 52.882 | 56.531 | 1.00 | 24.26 | C |
| ATOM | 4148 | CG | PRO | C | 119 | 17.001 | 52.352 | 56.143 | 1.00 | 26.13 | C |
| ATOM | 4149 | CD | PRO | C | 119 | 17.138 | 50.903 | 55.717 | 1.00 | 25.34 | C |
| ATOM | 4150 | N | PRO | C | 120 | 20.937 | 52.145 | 58.124 | 1.00 | 22.70 | N |

| ATOM | 4151 | CA | PRO | C | 120 | 21.466 | 51.894 | 59.442 | 1.00 | 21.33 | C |
|------|------|-----|-----|---|-----|--------|--------|--------|------|-------|---|
| ATOM | 4152 | C | PRO | C | 120 | 20.353 | 51.789 | 60.489 | 1.00 | 21.03 | C |
| ATOM | 4153 | O | PRO | C | 120 | 19.401 | 52.537 | 60.441 | 1.00 | 19.79 | O |
| ATOM | 4154 | CB | PRO | C | 120 | 22.315 | 53.103 | 59.690 | 1.00 | 22.13 | C |
| ATOM | 4155 | CG | PRO | C | 120 | 22.809 | 53.476 | 58.328 | 1.00 | 20.82 | C |
| ATOM | 4156 | CD | PRO | C | 120 | 21.620 | 53.277 | 57.482 | 1.00 | 23.88 | C |
| ATOM | 4157 | N | SER | C | 121 | 20.488 | 50.875 | 61.427 | 1.00 | 20.65 | N |
| ATOM | 4158 | CA | SER | C | 121 | 19.525 | 50.801 | 62.525 | 1.00 | 21.50 | C |
| ATOM | 4159 | C | SER | C | 121 | 19.640 | 52.025 | 63.454 | 1.00 | 21.94 | C |
| ATOM | 4160 | O | SER | C | 121 | 20.690 | 52.636 | 63.578 | 1.00 | 18.86 | O |
| ATOM | 4161 | CB | SER | C | 121 | 19.698 | 49.489 | 63.302 | 1.00 | 21.46 | C |
| ATOM | 4162 | OG | SER | C | 121 | 20.939 | 49.373 | 63.993 | 1.00 | 22.01 | O |
| ATOM | 4163 | N | SER | C | 122 | 18.561 | 52.360 | 64.158 | 1.00 | 21.56 | N |
| ATOM | 4164 | CA | SER | C | 122 | 18.612 | 53.430 | 65.117 | 1.00 | 22.20 | C |
| ATOM | 4165 | C | SER | C | 122 | 19.664 | 53.096 | 66.169 | 1.00 | 22.59 | C |
| ATOM | 4166 | O | SER | C | 122 | 20.484 | 53.942 | 66.561 | 1.00 | 24.01 | O |
| ATOM | 4167 | CB | SER | C | 122 | 17.225 | 53.648 | 65.744 | 1.00 | 24.62 | C |
| ATOM | 4168 | OG | SER | C | 122 | 16.875 | 52.521 | 66.525 | 1.00 | 25.70 | O |
| ATOM | 4169 | N | GLU | C | 123 | 19.693 | 51.842 | 66.588 | 1.00 | 21.93 | N |
| ATOM | 4170 | CA | GLU | C | 123 | 20.663 | 51.387 | 67.600 | 1.00 | 21.81 | C |
| ATOM | 4171 | C | GLU | C | 123 | 22.118 | 51.584 | 67.178 | 1.00 | 18.90 | C |
| ATOM | 4172 | O | GLU | C | 123 | 22.945 | 51.989 | 67.978 | 1.00 | 19.66 | O |
| ATOM | 4173 | CB | GLU | C | 123 | 20.427 | 49.909 | 67.946 | 1.00 | 24.55 | C |
| ATOM | 4174 | CG | GLU | C | 123 | 19.014 | 49.635 | 68.495 | 1.00 | 27.97 | C |
| ATOM | 4175 | CD | GLU | C | 123 | 17.952 | 49.228 | 67.425 | 1.00 | 32.71 | C |
| ATOM | 4176 | OE1 | GLU | C | 123 | 17.763 | 49.914 | 66.390 | 1.00 | 28.57 | O |
| ATOM | 4177 | OE2 | GLU | C | 123 | 17.259 | 48.196 | 67.663 | 1.00 | 37.42 | O |
| ATOM | 4178 | N | GLN | C | 124 | 22.419 | 51.252 | 65.941 | 1.00 | 18.08 | N |
| ATOM | 4179 | CA | GLN | C | 124 | 23.773 | 51.421 | 65.392 | 1.00 | 19.76 | C |
| ATOM | 4180 | C | GLN | C | 124 | 24.119 | 52.898 | 65.359 | 1.00 | 18.62 | C |
| ATOM | 4181 | O | GLN | C | 124 | 25.158 | 53.297 | 65.858 | 1.00 | 22.08 | O |
| ATOM | 4182 | CB | GLN | C | 124 | 23.883 | 50.873 | 63.991 | 1.00 | 18.24 | C |
| ATOM | 4183 | CG | GLN | C | 124 | 25.349 | 50.788 | 63.580 | 1.00 | 20.23 | C |
| ATOM | 4184 | CD | GLN | C | 124 | 25.551 | 50.212 | 62.248 | 1.00 | 19.00 | C |
| ATOM | 4185 | OE1 | GLN | C | 124 | 24.694 | 50.325 | 61.383 | 1.00 | 18.74 | O |
| ATOM | 4186 | NE2 | GLN | C | 124 | 26.709 | 49.579 | 62.050 | 1.00 | 21.24 | N |
| ATOM | 4187 | N | LEU | C | 125 | 23.214 | 53.686 | 64.807 | 1.00 | 19.31 | N |
| ATOM | 4188 | CA | LEU | C | 125 | 23.357 | 55.147 | 64.761 | 1.00 | 22.12 | C |
| ATOM | 4189 | C | LEU | C | 125 | 23.670 | 55.763 | 66.116 | 1.00 | 22.50 | C |
| ATOM | 4190 | O | LEU | C | 125 | 24.518 | 56.647 | 66.192 | 1.00 | 21.29 | O |
| ATOM | 4191 | CB | LEU | C | 125 | 22.147 | 55.793 | 64.104 | 1.00 | 22.12 | C |
| ATOM | 4192 | CG | LEU | C | 125 | 22.069 | 55.503 | 62.609 | 1.00 | 23.97 | C |
| ATOM | 4193 | CD1 | LEU | C | 125 | 20.765 | 56.006 | 62.013 | 1.00 | 23.65 | C |
| ATOM | 4194 | CD2 | LEU | C | 125 | 23.265 | 56.155 | 61.872 | 1.00 | 24.93 | C |
| ATOM | 4195 | N | THR | C | 126 | 23.022 | 55.275 | 67.182 | 1.00 | 21.82 | N |
| ATOM | 4196 | CA | THR | C | 126 | 23.262 | 55.766 | 68.536 | 1.00 | 22.37 | C |
| ATOM | 4197 | C | THR | C | 126 | 24.731 | 55.517 | 68.970 | 1.00 | 23.23 | C |
| ATOM | 4198 | O | THR | C | 126 | 25.366 | 56.366 | 69.611 | 1.00 | 21.35 | O |
| ATOM | 4199 | CB | THR | C | 126 | 22.267 | 55.118 | 69.535 | 1.00 | 22.49 | C |
| ATOM | 4200 | OG1 | THR | C | 126 | 20.938 | 55.562 | 69.222 | 1.00 | 21.59 | O |
| ATOM | 4201 | CG2 | THR | C | 126 | 22.600 | 55.499 | 71.001 | 1.00 | 23.16 | C |
| ATOM | 4202 | N | SER | C | 127 | 25.245 | 54.354 | 68.577 | 1.00 | 23.09 | N |
| ATOM | 4203 | CA | SER | C | 127 | 26.607 | 53.944 | 68.833 | 1.00 | 24.76 | C |
| ATOM | 4204 | C | SER | C | 127 | 27.630 | 54.731 | 67.989 | 1.00 | 25.04 | C |
| ATOM | 4205 | O | SER | C | 127 | 28.833 | 54.726 | 68.302 | 1.00 | 25.72 | O |
| ATOM | 4206 | CB | SER | C | 127 | 26.736 | 52.444 | 68.516 | 1.00 | 24.59 | C |
| ATOM | 4207 | OG | SER | C | 127 | 27.878 | 51.902 | 69.114 | 1.00 | 32.20 | O |
| ATOM | 4208 | N | GLY | C | 128 | 27.170 | 55.374 | 66.920 | 1.00 | 24.67 | N |
| ATOM | 4209 | CA | GLY | C | 128 | 28.041 | 56.189 | 66.066 | 1.00 | 25.97 | C |
| ATOM | 4210 | C | GLY | C | 128 | 28.486 | 55.530 | 64.779 | 1.00 | 27.78 | C |
| ATOM | 4211 | O | GLY | C | 128 | 29.324 | 56.099 | 64.028 | 1.00 | 28.85 | O |
| ATOM | 4212 | N | GLY | C | 129 | 27.929 | 54.341 | 64.505 | 1.00 | 26.54 | N |
| ATOM | 4213 | CA | GLY | C | 129 | 28.237 | 53.595 | 63.297 | 1.00 | 25.81 | C |
| ATOM | 4214 | C | GLY | C | 129 | 27.102 | 53.665 | 62.311 | 1.00 | 24.37 | C |
| ATOM | 4215 | O | GLY | C | 129 | 26.000 | 54.050 | 62.653 | 1.00 | 26.19 | O |

```
ATOM   4216  N   ALA C 130      27.362  53.299  61.075  1.00 24.99           N
ATOM   4217  CA  ALA C 130      26.333  53.376  60.059  1.00 26.68           C
ATOM   4218  C   ALA C 130      26.566  52.369  58.940  1.00 28.32           C
ATOM   4219  O   ALA C 130      27.335  52.631  58.009  1.00 30.11           O
ATOM   4220  CB  ALA C 130      26.253  54.773  59.509  1.00 27.46           C
ATOM   4221  N   SER C 131      25.908  51.217  59.028  1.00 26.00           N
ATOM   4222  CA  SER C 131      26.078  50.184  58.010  1.00 25.07           C
ATOM   4223  C   SER C 131      24.834  50.112  57.174  1.00 25.92           C
ATOM   4224  O   SER C 131      23.708  49.918  57.693  1.00 24.11           O
ATOM   4225  CB  SER C 131      26.363  48.826  58.636  1.00 25.48           C
ATOM   4226  OG  SER C 131      27.502  48.903  59.450  1.00 24.39           O
ATOM   4227  N   VAL C 132      25.013  50.264  55.868  1.00 22.94           N
ATOM   4228  CA  VAL C 132      23.917  50.118  54.959  1.00 23.62           C
ATOM   4229  C   VAL C 132      24.034  48.715  54.384  1.00 24.04           C
ATOM   4230  O   VAL C 132      25.123  48.324  53.926  1.00 23.59           O
ATOM   4231  CB  VAL C 132      23.935  51.182  53.831  1.00 24.26           C
ATOM   4232  CG1 VAL C 132      22.673  51.134  53.086  1.00 25.06           C
ATOM   4233  CG2 VAL C 132      24.161  52.586  54.402  1.00 26.33           C
ATOM   4234  N   VAL C 133      22.924  47.960  54.413  1.00 23.50           N
ATOM   4235  CA  VAL C 133      22.961  46.555  54.018  1.00 23.94           C
ATOM   4236  C   VAL C 133      22.102  46.312  52.774  1.00 25.37           C
ATOM   4237  O   VAL C 133      21.011  46.853  52.617  1.00 25.66           O
ATOM   4238  CB  VAL C 133      22.604  45.584  55.204  1.00 22.90           C
ATOM   4239  CG1 VAL C 133      22.605  44.135  54.747  1.00 23.11           C
ATOM   4240  CG2 VAL C 133      23.583  45.757  56.348  1.00 21.75           C
ATOM   4241  N   CYS C 134      22.624  45.491  51.878  1.00 27.55           N
ATOM   4242  CA  CYS C 134      21.893  45.094  50.691  1.00 27.75           C
ATOM   4243  C   CYS C 134      21.977  43.564  50.603  1.00 26.56           C
ATOM   4244  O   CYS C 134      23.068  42.991  50.723  1.00 25.08           O
ATOM   4245  CB  CYS C 134      22.532  45.751  49.456  1.00 29.46           C
ATOM   4246  SG  CYS C 134      21.623  45.508  47.893  1.00 35.52           S
ATOM   4247  N   PHE C 135      20.831  42.921  50.439  1.00 25.14           N
ATOM   4248  CA  PHE C 135      20.776  41.485  50.235  1.00 25.41           C
ATOM   4249  C   PHE C 135      20.412  41.273  48.793  1.00 25.53           C
ATOM   4250  O   PHE C 135      19.524  41.923  48.278  1.00 24.28           O
ATOM   4251  CB  PHE C 135      19.714  40.812  51.121  1.00 25.43           C
ATOM   4252  CG  PHE C 135      20.044  40.821  52.579  1.00 24.98           C
ATOM   4253  CD1 PHE C 135      21.281  40.438  53.034  1.00 26.27           C
ATOM   4254  CD2 PHE C 135      19.092  41.168  53.511  1.00 28.49           C
ATOM   4255  CE1 PHE C 135      21.587  40.459  54.383  1.00 24.65           C
ATOM   4256  CE2 PHE C 135      19.389  41.166  54.858  1.00 26.09           C
ATOM   4257  CZ  PHE C 135      20.637  40.825  55.290  1.00 25.93           C
ATOM   4258  N   LEU C 136      21.111  40.345  48.153  1.00 26.93           N
ATOM   4259  CA  LEU C 136      20.807  39.923  46.807  1.00 28.27           C
ATOM   4260  C   LEU C 136      20.519  38.417  46.883  1.00 28.30           C
ATOM   4261  O   LEU C 136      21.439  37.627  47.018  1.00 28.91           O
ATOM   4262  CB  LEU C 136      22.016  40.233  45.941  1.00 29.14           C
ATOM   4263  CG  LEU C 136      22.474  41.690  46.075  1.00 32.08           C
ATOM   4264  CD1 LEU C 136      23.646  41.829  47.044  1.00 34.05           C
ATOM   4265  CD2 LEU C 136      22.879  42.196  44.729  1.00 33.25           C
ATOM   4266  N   ASN C 137      19.252  38.027  46.831  1.00 28.75           N
ATOM   4267  CA  ASN C 137      18.867  36.676  47.234  1.00 28.83           C
ATOM   4268  C   ASN C 137      18.254  35.793  46.155  1.00 29.84           C
ATOM   4269  O   ASN C 137      17.450  36.244  45.339  1.00 29.75           O
ATOM   4270  CB  ASN C 137      17.880  36.732  48.406  1.00 29.92           C
ATOM   4271  CG  ASN C 137      18.543  37.124  49.725  1.00 29.29           C
ATOM   4272  OD1 ASN C 137      19.734  36.942  49.907  1.00 29.73           O
ATOM   4273  ND2 ASN C 137      17.759  37.662  50.639  1.00 26.63           N
ATOM   4274  N   ASN C 138      18.641  34.515  46.207  1.00 29.70           N
ATOM   4275  CA  ASN C 138      18.116  33.447  45.364  1.00 30.04           C
ATOM   4276  C   ASN C 138      18.251  33.670  43.868  1.00 30.08           C
ATOM   4277  O   ASN C 138      17.291  33.502  43.100  1.00 32.13           O
ATOM   4278  CB  ASN C 138      16.699  33.062  45.774  1.00 31.42           C
ATOM   4279  CG  ASN C 138      16.617  32.656  47.220  1.00 31.70           C
ATOM   4280  OD1 ASN C 138      16.639  33.496  48.099  1.00 34.49           O
```

| ATOM | 4281 | ND2 | ASN | C | 138 | 16.561 | 31.364 | 47.478 | 1.00 | 36.02 | N |
|------|------|-----|-----|---|-----|--------|--------|--------|------|-------|---|
| ATOM | 4282 | N   | PHE | C | 139 | 19.467 | 34.009 | 43.462 | 1.00 | 29.37 | N |
| ATOM | 4283 | CA  | PHE | C | 139 | 19.797 | 34.154 | 42.046 | 1.00 | 29.45 | C |
| ATOM | 4284 | C   | PHE | C | 139 | 20.596 | 32.960 | 41.469 | 1.00 | 29.45 | C |
| ATOM | 4285 | O   | PHE | C | 139 | 21.077 | 32.094 | 42.201 | 1.00 | 27.43 | O |
| ATOM | 4286 | CB  | PHE | C | 139 | 20.517 | 35.482 | 41.805 | 1.00 | 29.49 | C |
| ATOM | 4287 | CG  | PHE | C | 139 | 21.838 | 35.627 | 42.532 | 1.00 | 30.04 | C |
| ATOM | 4288 | CD1 | PHE | C | 139 | 23.011 | 35.176 | 41.950 | 1.00 | 29.69 | C |
| ATOM | 4289 | CD2 | PHE | C | 139 | 21.918 | 36.270 | 43.761 | 1.00 | 30.50 | C |
| ATOM | 4290 | CE1 | PHE | C | 139 | 24.220 | 35.302 | 42.583 | 1.00 | 29.05 | C |
| ATOM | 4291 | CE2 | PHE | C | 139 | 23.137 | 36.405 | 44.414 | 1.00 | 28.48 | C |
| ATOM | 4292 | CZ  | PHE | C | 139 | 24.295 | 35.925 | 43.816 | 1.00 | 30.30 | C |
| ATOM | 4293 | N   | TYR | C | 140 | 20.702 | 32.936 | 40.137 | 1.00 | 30.82 | N |
| ATOM | 4294 | CA  | TYR | C | 140 | 21.497 | 31.949 | 39.389 | 1.00 | 30.67 | C |
| ATOM | 4295 | C   | TYR | C | 140 | 21.732 | 32.488 | 37.953 | 1.00 | 32.17 | C |
| ATOM | 4296 | O   | TYR | C | 140 | 20.788 | 32.915 | 37.281 | 1.00 | 31.16 | O |
| ATOM | 4297 | CB  | TYR | C | 140 | 20.800 | 30.567 | 39.328 | 1.00 | 30.66 | C |
| ATOM | 4298 | CG  | TYR | C | 140 | 21.739 | 29.512 | 38.778 | 1.00 | 31.45 | C |
| ATOM | 4299 | CD1 | TYR | C | 140 | 22.589 | 28.801 | 39.632 | 1.00 | 29.31 | C |
| ATOM | 4300 | CD2 | TYR | C | 140 | 21.879 | 29.314 | 37.390 | 1.00 | 31.00 | C |
| ATOM | 4301 | CE1 | TYR | C | 140 | 23.505 | 27.881 | 39.125 | 1.00 | 30.37 | C |
| ATOM | 4302 | CE2 | TYR | C | 140 | 22.783 | 28.396 | 36.886 | 1.00 | 31.30 | C |
| ATOM | 4303 | CZ  | TYR | C | 140 | 23.609 | 27.688 | 37.757 | 1.00 | 30.52 | C |
| ATOM | 4304 | OH  | TYR | C | 140 | 24.520 | 26.776 | 37.259 | 1.00 | 30.40 | O |
| ATOM | 4305 | N   | PRO | C | 141 | 22.969 | 32.415 | 37.449 | 1.00 | 34.32 | N |
| ATOM | 4306 | CA  | PRO | C | 141 | 24.163 | 31.782 | 38.005 | 1.00 | 35.55 | C |
| ATOM | 4307 | C   | PRO | C | 141 | 24.825 | 32.551 | 39.146 | 1.00 | 36.90 | C |
| ATOM | 4308 | O   | PRO | C | 141 | 24.348 | 33.620 | 39.542 | 1.00 | 36.04 | O |
| ATOM | 4309 | CB  | PRO | C | 141 | 25.097 | 31.675 | 36.785 | 1.00 | 35.49 | C |
| ATOM | 4310 | CG  | PRO | C | 141 | 24.702 | 32.801 | 35.909 | 1.00 | 35.17 | C |
| ATOM | 4311 | CD  | PRO | C | 141 | 23.220 | 32.979 | 36.103 | 1.00 | 35.05 | C |
| ATOM | 4312 | N   | LYS | C | 142 | 25.914 | 31.963 | 39.638 | 1.00 | 39.23 | N |
| ATOM | 4313 | CA  | LYS | C | 142 | 26.681 | 32.390 | 40.816 | 1.00 | 41.55 | C |
| ATOM | 4314 | C   | LYS | C | 142 | 27.316 | 33.762 | 40.704 | 1.00 | 42.55 | C |
| ATOM | 4315 | O   | LYS | C | 142 | 27.438 | 34.471 | 41.699 | 1.00 | 42.02 | O |
| ATOM | 4316 | CB  | LYS | C | 142 | 27.790 | 31.357 | 41.052 | 1.00 | 42.29 | C |
| ATOM | 4317 | CG  | LYS | C | 142 | 28.810 | 31.619 | 42.154 | 1.00 | 42.20 | C |
| ATOM | 4318 | CD  | LYS | C | 142 | 29.778 | 30.409 | 42.185 | 1.00 | 43.23 | C |
| ATOM | 4319 | CE  | LYS | C | 142 | 30.717 | 30.406 | 43.376 | 1.00 | 43.76 | C |
| ATOM | 4320 | NZ  | LYS | C | 142 | 31.419 | 29.087 | 43.505 | 1.00 | 44.07 | N |
| ATOM | 4321 | N   | ASP | C | 143 | 27.748 | 34.128 | 39.505 | 1.00 | 44.32 | N |
| ATOM | 4322 | CA  | ASP | C | 143 | 28.456 | 35.402 | 39.329 | 1.00 | 45.37 | C |
| ATOM | 4323 | C   | ASP | C | 143 | 27.516 | 36.603 | 39.265 | 1.00 | 44.59 | C |
| ATOM | 4324 | O   | ASP | C | 143 | 26.529 | 36.588 | 38.551 | 1.00 | 43.36 | O |
| ATOM | 4325 | CB  | ASP | C | 143 | 29.383 | 35.346 | 38.109 | 1.00 | 46.52 | C |
| ATOM | 4326 | CG  | ASP | C | 143 | 30.740 | 34.782 | 38.460 | 1.00 | 48.67 | C |
| ATOM | 4327 | OD1 | ASP | C | 143 | 31.551 | 35.545 | 39.048 | 1.00 | 51.05 | O |
| ATOM | 4328 | OD2 | ASP | C | 143 | 30.986 | 33.581 | 38.182 | 1.00 | 49.82 | O |
| ATOM | 4329 | N   | ILE | C | 144 | 27.854 | 37.634 | 40.030 | 1.00 | 45.28 | N |
| ATOM | 4330 | CA  | ILE | C | 144 | 27.028 | 38.831 | 40.155 | 1.00 | 46.23 | C |
| ATOM | 4331 | C   | ILE | C | 144 | 27.917 | 40.012 | 40.514 | 1.00 | 47.28 | C |
| ATOM | 4332 | O   | ILE | C | 144 | 28.948 | 39.832 | 41.160 | 1.00 | 47.40 | O |
| ATOM | 4333 | CB  | ILE | C | 144 | 25.958 | 38.647 | 41.260 | 1.00 | 45.92 | C |
| ATOM | 4334 | CG1 | ILE | C | 144 | 24.970 | 39.821 | 41.273 | 1.00 | 45.84 | C |
| ATOM | 4335 | CG2 | ILE | C | 144 | 26.621 | 38.458 | 42.629 | 1.00 | 45.59 | C |
| ATOM | 4336 | CD1 | ILE | C | 144 | 23.539 | 39.413 | 41.509 | 1.00 | 44.77 | C |
| ATOM | 4337 | N   | ASN | C | 145 | 27.524 | 41.215 | 40.113 | 1.00 | 48.28 | N |
| ATOM | 4338 | CA  | ASN | C | 145 | 28.220 | 42.414 | 40.571 | 1.00 | 49.26 | C |
| ATOM | 4339 | C   | ASN | C | 145 | 27.257 | 43.355 | 41.265 | 1.00 | 49.38 | C |
| ATOM | 4340 | O   | ASN | C | 145 | 26.095 | 43.467 | 40.876 | 1.00 | 49.23 | O |
| ATOM | 4341 | CB  | ASN | C | 145 | 28.912 | 43.123 | 39.410 | 1.00 | 50.16 | C |
| ATOM | 4342 | CG  | ASN | C | 145 | 30.128 | 42.360 | 38.909 | 1.00 | 51.57 | C |
| ATOM | 4343 | OD1 | ASN | C | 145 | 31.116 | 42.172 | 39.634 | 1.00 | 51.40 | O |
| ATOM | 4344 | ND2 | ASN | C | 145 | 30.056 | 41.909 | 37.661 | 1.00 | 53.30 | N |
| ATOM | 4345 | N   | VAL | C | 146 | 27.739 | 44.007 | 42.319 | 1.00 | 49.14 | N |

```
ATOM   4346  CA  VAL C 146    26.940  44.992  43.020  1.00 48.89           C
ATOM   4347  C   VAL C 146    27.760  46.257  43.071  1.00 47.96           C
ATOM   4348  O   VAL C 146    28.940  46.205  43.413  1.00 47.29           O
ATOM   4349  CB  VAL C 146    26.579  44.543  44.456  1.00 49.08           C
ATOM   4350  CG1 VAL C 146    25.512  45.463  45.062  1.00 49.07           C
ATOM   4351  CG2 VAL C 146    26.081  43.102  44.453  1.00 50.05           C
ATOM   4352  N   LYS C 147    27.135  47.384  42.729  1.00 47.69           N
ATOM   4353  CA  LYS C 147    27.755  48.702  42.919  1.00 47.16           C
ATOM   4354  C   LYS C 147    26.941  49.537  43.921  1.00 45.33           C
ATOM   4355  O   LYS C 147    25.702  49.582  43.872  1.00 43.48           O
ATOM   4356  CB  LYS C 147    27.883  49.459  41.584  1.00 47.60           C
ATOM   4357  CG  LYS C 147    28.810  50.694  41.652  1.00 48.56           C
ATOM   4358  CD  LYS C 147    28.438  51.807  40.641  1.00 48.68           C
ATOM   4359  CE  LYS C 147    29.467  51.989  39.521  1.00 50.20           C
ATOM   4360  NZ  LYS C 147    29.425  53.382  38.897  1.00 49.98           N
ATOM   4361  N   TRP C 148    27.663  50.193  44.823  1.00 44.02           N
ATOM   4362  CA  TRP C 148    27.072  51.136  45.762  1.00 43.44           C
ATOM   4363  C   TRP C 148    27.190  52.556  45.195  1.00 43.56           C
ATOM   4364  O   TRP C 148    28.242  52.941  44.676  1.00 43.76           O
ATOM   4365  CB  TRP C 148    27.764  51.047  47.134  1.00 42.01           C
ATOM   4366  CG  TRP C 148    27.266  49.911  47.952  1.00 41.52           C
ATOM   4367  CD1 TRP C 148    27.796  48.658  48.021  1.00 41.50           C
ATOM   4368  CD2 TRP C 148    26.114  49.909  48.803  1.00 41.27           C
ATOM   4369  NE1 TRP C 148    27.042  47.866  48.871  1.00 41.51           N
ATOM   4370  CE2 TRP C 148    26.009  48.611  49.368  1.00 40.72           C
ATOM   4371  CE3 TRP C 148    25.157  50.872  49.142  1.00 40.29           C
ATOM   4372  CZ2 TRP C 148    25.000  48.259  50.257  1.00 40.16           C
ATOM   4373  CZ3 TRP C 148    24.143  50.511  50.030  1.00 41.17           C
ATOM   4374  CH2 TRP C 148    24.077  49.215  50.569  1.00 40.58           C
ATOM   4375  N   LYS C 149    26.101  53.308  45.287  1.00 43.30           N
ATOM   4376  CA  LYS C 149    26.111  54.726  44.988  1.00 44.56           C
ATOM   4377  C   LYS C 149    25.505  55.536  46.143  1.00 44.42           C
ATOM   4378  O   LYS C 149    24.510  55.134  46.752  1.00 44.25           O
ATOM   4379  CB  LYS C 149    25.349  55.000  43.685  1.00 45.31           C
ATOM   4380  CG  LYS C 149    26.035  54.426  42.433  1.00 45.51           C
ATOM   4381  CD  LYS C 149    25.146  54.537  41.218  1.00 45.49           C
ATOM   4382  CE  LYS C 149    25.665  53.662  40.077  1.00 46.58           C
ATOM   4383  NZ  LYS C 149    24.811  53.737  38.848  1.00 45.91           N
ATOM   4384  N   ILE C 150    26.130  56.667  46.451  1.00 44.41           N
ATOM   4385  CA  ILE C 150    25.626  57.593  47.451  1.00 45.06           C
ATOM   4386  C   ILE C 150    25.442  58.940  46.772  1.00 45.67           C
ATOM   4387  O   ILE C 150    26.398  59.461  46.199  1.00 45.22           O
ATOM   4388  CB  ILE C 150    26.637  57.791  48.609  1.00 44.86           C
ATOM   4389  CG1 ILE C 150    27.073  56.444  49.214  1.00 44.56           C
ATOM   4390  CG2 ILE C 150    26.039  58.709  49.663  1.00 45.27           C
ATOM   4391  CD1 ILE C 150    28.346  56.523  50.036  1.00 44.16           C
ATOM   4392  N   ASP C 151    24.241  59.511  46.862  1.00 47.19           N
ATOM   4393  CA  ASP C 151    23.897  60.780  46.185  1.00 47.82           C
ATOM   4394  C   ASP C 151    24.299  60.776  44.701  1.00 49.02           C
ATOM   4395  O   ASP C 151    24.839  61.766  44.210  1.00 48.13           O
ATOM   4396  CB  ASP C 151    24.583  61.979  46.865  1.00 48.37           C
ATOM   4397  CG  ASP C 151    23.963  62.355  48.194  1.00 49.16           C
ATOM   4398  OD1 ASP C 151    22.822  61.933  48.503  1.00 49.77           O
ATOM   4399  OD2 ASP C 151    24.646  63.104  48.928  1.00 49.57           O
ATOM   4400  N   GLY C 152    24.067  59.661  44.007  1.00 50.24           N
ATOM   4401  CA  GLY C 152    24.471  59.519  42.606  1.00 51.71           C
ATOM   4402  C   GLY C 152    25.881  58.990  42.396  1.00 52.83           C
ATOM   4403  O   GLY C 152    26.126  58.240  41.447  1.00 54.25           O
ATOM   4404  N   SER C 153    26.802  59.348  43.289  1.00 54.04           N
ATOM   4405  CA  SER C 153    28.228  59.023  43.140  1.00 55.25           C
ATOM   4406  C   SER C 153    28.640  57.599  43.584  1.00 56.51           C
ATOM   4407  O   SER C 153    28.338  57.164  44.706  1.00 56.01           O
ATOM   4408  CB  SER C 153    29.067  60.053  43.911  1.00 55.75           C
ATOM   4409  OG  SER C 153    28.631  61.378  43.637  1.00 55.58           O
ATOM   4410  N   GLU C 154    29.372  56.904  42.714  1.00 57.37           N
```

| ATOM | 4411 | CA | GLU C 154 | 29.807 | 55.529 | 42.968 | 1.00 | 58.29 | C |
|---|---|---|---|---|---|---|---|---|---|
| ATOM | 4412 | C | GLU C 154 | 30.710 | 55.417 | 44.191 | 1.00 | 58.09 | C |
| ATOM | 4413 | O | GLU C 154 | 31.607 | 56.236 | 44.382 | 1.00 | 58.69 | O |
| ATOM | 4414 | CB | GLU C 154 | 30.539 | 54.959 | 41.743 | 1.00 | 58.76 | C |
| ATOM | 4415 | CG | GLU C 154 | 31.290 | 53.628 | 41.983 | 1.00 | 59.06 | C |
| ATOM | 4416 | CD | GLU C 154 | 32.461 | 53.426 | 41.018 | 1.00 | 60.17 | C |
| ATOM | 4417 | OE1 | GLU C 154 | 32.275 | 53.617 | 39.783 | 1.00 | 60.89 | O |
| ATOM | 4418 | OE2 | GLU C 154 | 33.566 | 53.083 | 41.508 | 1.00 | 61.11 | O |
| ATOM | 4419 | N | ARG C 155 | 30.494 | 54.356 | 44.970 | 1.00 | 57.53 | N |
| ATOM | 4420 | CA | ARG C 155 | 31.194 | 54.116 | 46.226 | 1.00 | 57.19 | C |
| ATOM | 4421 | C | ARG C 155 | 31.904 | 52.751 | 46.247 | 1.00 | 57.70 | C |
| ATOM | 4422 | O | ARG C 155 | 31.272 | 51.703 | 46.099 | 1.00 | 58.18 | O |
| ATOM | 4423 | CB | ARG C 155 | 30.178 | 54.182 | 47.361 | 1.00 | 56.58 | C |
| ATOM | 4424 | CG | ARG C 155 | 30.754 | 54.029 | 48.728 | 1.00 | 55.35 | C |
| ATOM | 4425 | CD | ARG C 155 | 31.483 | 55.259 | 49.157 | 1.00 | 54.07 | C |
| ATOM | 4426 | NE | ARG C 155 | 32.034 | 55.075 | 50.491 | 1.00 | 52.92 | N |
| ATOM | 4427 | CZ | ARG C 155 | 32.374 | 56.056 | 51.314 | 1.00 | 52.30 | C |
| ATOM | 4428 | NH1 | ARG C 155 | 32.228 | 57.333 | 50.967 | 1.00 | 52.19 | N |
| ATOM | 4429 | NH2 | ARG C 155 | 32.866 | 55.749 | 52.502 | 1.00 | 52.98 | N |
| ATOM | 4430 | N | GLN C 156 | 33.221 | 52.775 | 46.437 | 1.00 | 57.99 | N |
| ATOM | 4431 | CA | GLN C 156 | 34.017 | 51.555 | 46.564 | 1.00 | 58.19 | C |
| ATOM | 4432 | C | GLN C 156 | 34.485 | 51.345 | 48.013 | 1.00 | 58.17 | C |
| ATOM | 4433 | O | GLN C 156 | 34.509 | 50.215 | 48.500 | 1.00 | 58.79 | O |
| ATOM | 4434 | CB | GLN C 156 | 35.229 | 51.608 | 45.625 | 1.00 | 58.90 | C |
| ATOM | 4435 | CG | GLN C 156 | 34.894 | 51.681 | 44.122 | 1.00 | 59.38 | C |
| ATOM | 4436 | CD | GLN C 156 | 36.154 | 51.745 | 43.235 | 1.00 | 59.57 | C |
| ATOM | 4437 | OE1 | GLN C 156 | 36.968 | 50.815 | 43.216 | 1.00 | 60.66 | O |
| ATOM | 4438 | NE2 | GLN C 156 | 36.310 | 52.844 | 42.504 | 1.00 | 59.68 | N |
| ATOM | 4439 | N | ASN C 157 | 34.846 | 52.433 | 48.696 | 1.00 | 57.52 | N |
| ATOM | 4440 | CA | ASN C 157 | 35.380 | 52.378 | 50.068 | 1.00 | 56.82 | C |
| ATOM | 4441 | C | ASN C 157 | 34.385 | 51.785 | 51.104 | 1.00 | 55.80 | C |
| ATOM | 4442 | O | ASN C 157 | 33.199 | 52.123 | 51.105 | 1.00 | 55.38 | O |
| ATOM | 4443 | CB | ASN C 157 | 35.816 | 53.796 | 50.494 | 1.00 | 57.58 | C |
| ATOM | 4444 | CG | ASN C 157 | 36.727 | 53.800 | 51.710 | 1.00 | 58.88 | C |
| ATOM | 4445 | OD1 | ASN C 157 | 36.524 | 53.042 | 52.657 | 1.00 | 60.07 | O |
| ATOM | 4446 | ND2 | ASN C 157 | 37.734 | 54.674 | 51.694 | 1.00 | 59.57 | N |
| ATOM | 4447 | N | GLY C 158 | 34.882 | 50.902 | 51.971 | 1.00 | 54.20 | N |
| ATOM | 4448 | CA | GLY C 158 | 34.092 | 50.365 | 53.086 | 1.00 | 53.42 | C |
| ATOM | 4449 | C | GLY C 158 | 32.955 | 49.425 | 52.699 | 1.00 | 51.58 | C |
| ATOM | 4450 | O | GLY C 158 | 32.015 | 49.227 | 53.482 | 1.00 | 50.57 | O |
| ATOM | 4451 | N | VAL C 159 | 33.036 | 48.848 | 51.498 | 1.00 | 49.74 | N |
| ATOM | 4452 | CA | VAL C 159 | 32.071 | 47.844 | 51.052 | 1.00 | 47.98 | C |
| ATOM | 4453 | C | VAL C 159 | 32.629 | 46.425 | 51.237 | 1.00 | 46.66 | C |
| ATOM | 4454 | O | VAL C 159 | 33.685 | 46.103 | 50.702 | 1.00 | 44.99 | O |
| ATOM | 4455 | CB | VAL C 159 | 31.709 | 48.027 | 49.590 | 1.00 | 47.93 | C |
| ATOM | 4456 | CG1 | VAL C 159 | 30.727 | 46.926 | 49.145 | 1.00 | 47.75 | C |
| ATOM | 4457 | CG2 | VAL C 159 | 31.135 | 49.412 | 49.354 | 1.00 | 48.25 | C |
| ATOM | 4458 | N | LEU C 160 | 31.895 | 45.599 | 51.990 | 1.00 | 45.47 | N |
| ATOM | 4459 | CA | LEU C 160 | 32.258 | 44.216 | 52.278 | 1.00 | 44.19 | C |
| ATOM | 4460 | C | LEU C 160 | 31.149 | 43.231 | 51.879 | 1.00 | 42.77 | C |
| ATOM | 4461 | O | LEU C 160 | 29.993 | 43.315 | 52.354 | 1.00 | 40.45 | O |
| ATOM | 4462 | CB | LEU C 160 | 32.563 | 44.043 | 53.764 | 1.00 | 45.47 | C |
| ATOM | 4463 | CG | LEU C 160 | 33.867 | 44.648 | 54.302 | 1.00 | 47.35 | C |
| ATOM | 4464 | CD1 | LEU C 160 | 34.188 | 44.077 | 55.675 | 1.00 | 47.82 | C |
| ATOM | 4465 | CD2 | LEU C 160 | 35.063 | 44.424 | 53.365 | 1.00 | 48.53 | C |
| ATOM | 4466 | N | ASN C 161 | 31.546 | 42.267 | 51.050 | 1.00 | 40.68 | N |
| ATOM | 4467 | CA | ASN C 161 | 30.644 | 41.342 | 50.392 | 1.00 | 39.46 | C |
| ATOM | 4468 | C | ASN C 161 | 30.793 | 39.936 | 50.959 | 1.00 | 37.72 | C |
| ATOM | 4469 | O | ASN C 161 | 31.888 | 39.548 | 51.401 | 1.00 | 36.71 | O |
| ATOM | 4470 | CB | ASN C 161 | 30.949 | 41.334 | 48.901 | 1.00 | 39.93 | C |
| ATOM | 4471 | CG | ASN C 161 | 30.733 | 42.700 | 48.248 | 1.00 | 40.72 | C |
| ATOM | 4472 | OD1 | ASN C 161 | 29.894 | 43.489 | 48.692 | 1.00 | 40.70 | O |
| ATOM | 4473 | ND2 | ASN C 161 | 31.474 | 42.972 | 47.168 | 1.00 | 39.86 | N |
| ATOM | 4474 | N | SER C 162 | 29.688 | 39.186 | 50.975 | 1.00 | 35.48 | N |
| ATOM | 4475 | CA | SER C 162 | 29.684 | 37.793 | 51.470 | 1.00 | 33.16 | C |

220

| ATOM | 4476 | C   | SER C 162 | 28.668 | 36.932 | 50.707 | 1.00 32.11 | C |
| ATOM | 4477 | O   | SER C 162 | 27.592 | 37.400 | 50.368 | 1.00 30.61 | O |
| ATOM | 4478 | CB  | SER C 162 | 29.378 | 37.762 | 52.962 | 1.00 33.69 | C |
| ATOM | 4479 | OG  | SER C 162 | 29.694 | 36.495 | 53.527 | 1.00 33.79 | O |
| ATOM | 4480 | N   | TRP C 163 | 29.009 | 35.668 | 50.455 | 1.00 31.85 | N |
| ATOM | 4481 | CA  | TRP C 163 | 28.197 | 34.789 | 49.594 | 1.00 32.68 | C |
| ATOM | 4482 | C   | TRP C 163 | 27.753 | 33.538 | 50.322 | 1.00 31.21 | C |
| ATOM | 4483 | O   | TRP C 163 | 28.543 | 32.946 | 51.018 | 1.00 31.32 | O |
| ATOM | 4484 | CB  | TRP C 163 | 29.022 | 34.378 | 48.382 | 1.00 34.88 | C |
| ATOM | 4485 | CG  | TRP C 163 | 29.438 | 35.540 | 47.593 | 1.00 36.57 | C |
| ATOM | 4486 | CD1 | TRP C 163 | 28.820 | 36.032 | 46.478 | 1.00 36.39 | C |
| ATOM | 4487 | CD2 | TRP C 163 | 30.548 | 36.400 | 47.857 | 1.00 36.36 | C |
| ATOM | 4488 | NE1 | TRP C 163 | 29.479 | 37.145 | 46.031 | 1.00 36.86 | N |
| ATOM | 4489 | CE2 | TRP C 163 | 30.543 | 37.399 | 46.856 | 1.00 36.87 | C |
| ATOM | 4490 | CE3 | TRP C 163 | 31.553 | 36.421 | 48.837 | 1.00 36.80 | C |
| ATOM | 4491 | CZ2 | TRP C 163 | 31.505 | 38.414 | 46.801 | 1.00 36.57 | C |
| ATOM | 4492 | CZ3 | TRP C 163 | 32.523 | 37.426 | 48.781 | 1.00 36.65 | C |
| ATOM | 4493 | CH2 | TRP C 163 | 32.488 | 38.414 | 47.769 | 1.00 36.65 | C |
| ATOM | 4494 | N   | THR C 164 | 26.502 | 33.116 | 50.178 | 1.00 30.72 | N |
| ATOM | 4495 | CA  | THR C 164 | 26.125 | 31.808 | 50.713 | 1.00 30.68 | C |
| ATOM | 4496 | C   | THR C 164 | 26.609 | 30.712 | 49.768 | 1.00 31.50 | C |
| ATOM | 4497 | O   | THR C 164 | 26.950 | 30.964 | 48.599 | 1.00 30.30 | O |
| ATOM | 4498 | CB  | THR C 164 | 24.601 | 31.613 | 50.949 | 1.00 29.85 | C |
| ATOM | 4499 | OG1 | THR C 164 | 23.874 | 31.909 | 49.759 | 1.00 29.47 | O |
| ATOM | 4500 | CG2 | THR C 164 | 24.112 | 32.481 | 52.073 | 1.00 29.21 | C |
| ATOM | 4501 | N   | ASP C 165 | 26.652 | 29.502 | 50.309 | 1.00 31.47 | N |
| ATOM | 4502 | CA  | ASP C 165 | 26.858 | 28.311 | 49.520 | 1.00 33.54 | C |
| ATOM | 4503 | C   | ASP C 165 | 25.549 | 27.996 | 48.786 | 1.00 33.01 | C |
| ATOM | 4504 | O   | ASP C 165 | 24.464 | 28.417 | 49.218 | 1.00 34.34 | O |
| ATOM | 4505 | CB  | ASP C 165 | 27.313 | 27.141 | 50.413 | 1.00 34.62 | C |
| ATOM | 4506 | CG  | ASP C 165 | 28.794 | 27.256 | 50.840 | 1.00 37.54 | C |
| ATOM | 4507 | OD1 | ASP C 165 | 29.609 | 27.918 | 50.140 | 1.00 40.28 | O |
| ATOM | 4508 | OD2 | ASP C 165 | 29.147 | 26.681 | 51.890 | 1.00 41.10 | O |
| ATOM | 4509 | N   | GLN C 166 | 25.642 | 27.257 | 47.688 | 1.00 31.14 | N |
| ATOM | 4510 | CA  | GLN C 166 | 24.484 | 27.023 | 46.846 | 1.00 30.52 | C |
| ATOM | 4511 | C   | GLN C 166 | 23.349 | 26.451 | 47.666 | 1.00 31.39 | C |
| ATOM | 4512 | O   | GLN C 166 | 23.546 | 25.493 | 48.366 | 1.00 29.95 | O |
| ATOM | 4513 | CB  | GLN C 166 | 24.820 | 26.050 | 45.735 | 1.00 30.32 | C |
| ATOM | 4514 | CG  | GLN C 166 | 23.713 | 25.867 | 44.734 | 1.00 29.32 | C |
| ATOM | 4515 | CD  | GLN C 166 | 24.227 | 25.361 | 43.405 | 1.00 30.52 | C |
| ATOM | 4516 | OE1 | GLN C 166 | 25.083 | 24.483 | 43.363 | 1.00 31.12 | O |
| ATOM | 4517 | NE2 | GLN C 166 | 23.696 | 25.892 | 42.318 | 1.00 29.31 | N |
| ATOM | 4518 | N   | ASP C 167 | 22.159 | 27.031 | 47.556 | 1.00 33.21 | N |
| ATOM | 4519 | CA  | ASP C 167 | 21.033 | 26.604 | 48.391 | 1.00 35.36 | C |
| ATOM | 4520 | C   | ASP C 167 | 20.724 | 25.120 | 48.213 | 1.00 36.96 | C |
| ATOM | 4521 | O   | ASP C 167 | 20.614 | 24.628 | 47.093 | 1.00 36.95 | O |
| ATOM | 4522 | CB  | ASP C 167 | 19.791 | 27.427 | 48.084 | 1.00 35.98 | C |
| ATOM | 4523 | CG  | ASP C 167 | 18.637 | 27.087 | 49.007 | 1.00 37.58 | C |
| ATOM | 4524 | OD1 | ASP C 167 | 18.417 | 27.818 | 49.988 | 1.00 41.51 | O |
| ATOM | 4525 | OD2 | ASP C 167 | 17.981 | 26.067 | 48.772 | 1.00 37.51 | O |
| ATOM | 4526 | N   | SER C 168 | 20.582 | 24.412 | 49.330 | 1.00 39.61 | N |
| ATOM | 4527 | CA  | SER C 168 | 20.334 | 22.976 | 49.294 | 1.00 41.28 | C |
| ATOM | 4528 | C   | SER C 168 | 19.026 | 22.644 | 48.572 | 1.00 42.18 | C |
| ATOM | 4529 | O   | SER C 168 | 18.952 | 21.637 | 47.884 | 1.00 43.65 | O |
| ATOM | 4530 | CB  | SER C 168 | 20.318 | 22.390 | 50.710 | 1.00 41.28 | C |
| ATOM | 4531 | OG  | SER C 168 | 19.136 | 22.773 | 51.385 | 1.00 41.48 | O |
| ATOM | 4532 | N   | LYS C 169 | 17.999 | 23.481 | 48.723 | 1.00 43.34 | N |
| ATOM | 4533 | CA  | LYS C 169 | 16.687 | 23.201 | 48.109 | 1.00 43.28 | C |
| ATOM | 4534 | C   | LYS C 169 | 16.546 | 23.679 | 46.651 | 1.00 42.15 | C |
| ATOM | 4535 | O   | LYS C 169 | 16.369 | 22.861 | 45.754 | 1.00 43.72 | O |
| ATOM | 4536 | CB  | LYS C 169 | 15.546 | 23.755 | 48.968 | 1.00 44.94 | C |
| ATOM | 4537 | CG  | LYS C 169 | 15.322 | 22.974 | 50.288 | 1.00 45.79 | C |
| ATOM | 4538 | CD  | LYS C 169 | 14.801 | 21.538 | 50.043 | 1.00 47.36 | C |
| ATOM | 4539 | CE  | LYS C 169 | 15.041 | 20.627 | 51.235 | 1.00 47.79 | C |
| ATOM | 4540 | NZ  | LYS C 169 | 14.215 | 21.022 | 52.433 | 1.00 48.76 | N |

221

```
ATOM   4541  N    ASP C 170      16.645  24.979  46.406  1.00 39.18           N
ATOM   4542  CA   ASP C 170      16.363  25.506  45.086  1.00 37.22           C
ATOM   4543  C    ASP C 170      17.592  25.666  44.186  1.00 35.64           C
ATOM   4544  O    ASP C 170      17.442  26.091  43.054  1.00 34.69           O
ATOM   4545  CB   ASP C 170      15.580  26.828  45.180  1.00 38.40           C
ATOM   4546  CG   ASP C 170      16.358  27.940  45.853  1.00 39.96           C
ATOM   4547  OD1  ASP C 170      17.571  27.791  46.055  1.00 41.62           O
ATOM   4548  OD2  ASP C 170      15.753  28.982  46.181  1.00 41.92           O
ATOM   4549  N    SER C 171      18.783  25.343  44.694  1.00 34.20           N
ATOM   4550  CA   SER C 171      20.052  25.442  43.946  1.00 33.52           C
ATOM   4551  C    SER C 171      20.465  26.886  43.557  1.00 30.93           C
ATOM   4552  O    SER C 171      21.296  27.093  42.672  1.00 29.33           O
ATOM   4553  CB   SER C 171      20.035  24.519  42.709  1.00 33.71           C
ATOM   4554  OG   SER C 171      19.867  23.150  43.086  1.00 34.71           O
ATOM   4555  N    THR C 172      19.918  27.874  44.253  1.00 29.60           N
ATOM   4556  CA   THR C 172      20.285  29.271  44.020  1.00 29.30           C
ATOM   4557  C    THR C 172      21.450  29.680  44.913  1.00 28.95           C
ATOM   4558  O    THR C 172      21.928  28.896  45.754  1.00 28.99           O
ATOM   4559  CB   THR C 172      19.079  30.226  44.237  1.00 30.08           C
ATOM   4560  OG1  THR C 172      18.671  30.222  45.617  1.00 29.64           O
ATOM   4561  CG2  THR C 172      17.907  29.793  43.368  1.00 30.26           C
ATOM   4562  N    TYR C 173      21.924  30.902  44.680  1.00 28.54           N
ATOM   4563  CA   TYR C 173      22.912  31.574  45.499  1.00 27.44           C
ATOM   4564  C    TYR C 173      22.312  32.847  46.054  1.00 26.55           C
ATOM   4565  O    TYR C 173      21.339  33.380  45.515  1.00 24.07           O
ATOM   4566  CB   TYR C 173      24.117  31.981  44.642  1.00 28.86           C
ATOM   4567  CG   TYR C 173      24.826  30.810  44.052  1.00 30.11           C
ATOM   4568  CD1  TYR C 173      24.435  30.290  42.830  1.00 29.71           C
ATOM   4569  CD2  TYR C 173      25.861  30.192  44.738  1.00 29.63           C
ATOM   4570  CE1  TYR C 173      25.052  29.187  42.301  1.00 31.19           C
ATOM   4571  CE2  TYR C 173      26.505  29.088  44.206  1.00 32.17           C
ATOM   4572  CZ   TYR C 173      26.099  28.592  42.983  1.00 31.44           C
ATOM   4573  OH   TYR C 173      26.723  27.504  42.441  1.00 30.62           O
ATOM   4574  N    SER C 174      22.945  33.358  47.098  1.00 26.34           N
ATOM   4575  CA   SER C 174      22.577  34.648  47.676  1.00 27.18           C
ATOM   4576  C    SER C 174      23.834  35.417  48.059  1.00 26.93           C
ATOM   4577  O    SER C 174      24.891  34.836  48.249  1.00 27.26           O
ATOM   4578  CB   SER C 174      21.645  34.434  48.870  1.00 26.74           C
ATOM   4579  OG   SER C 174      20.376  33.960  48.429  1.00 24.92           O
ATOM   4580  N    MET C 175      23.723  36.735  48.153  1.00 28.65           N
ATOM   4581  CA   MET C 175      24.874  37.576  48.462  1.00 30.23           C
ATOM   4582  C    MET C 175      24.470  38.697  49.385  1.00 28.36           C
ATOM   4583  O    MET C 175      23.388  39.240  49.228  1.00 27.19           O
ATOM   4584  CB   MET C 175      25.476  38.182  47.190  1.00 31.19           C
ATOM   4585  CG   MET C 175      26.457  39.285  47.497  1.00 34.33           C
ATOM   4586  SD   MET C 175      27.479  39.913  46.145  1.00 36.58           S
ATOM   4587  CE   MET C 175      28.243  41.275  46.966  1.00 36.19           C
ATOM   4588  N    SER C 176      25.329  39.004  50.358  1.00 26.70           N
ATOM   4589  CA   SER C 176      25.160  40.183  51.210  1.00 28.12           C
ATOM   4590  C    SER C 176      26.230  41.172  50.807  1.00 27.88           C
ATOM   4591  O    SER C 176      27.354  40.770  50.568  1.00 27.29           O
ATOM   4592  CB   SER C 176      25.373  39.844  52.675  1.00 27.91           C
ATOM   4593  OG   SER C 176      24.872  40.892  53.482  1.00 31.20           O
ATOM   4594  N    SER C 177      25.881  42.443  50.720  1.00 28.48           N
ATOM   4595  CA   SER C 177      26.885  43.495  50.597  1.00 28.65           C
ATOM   4596  C    SER C 177      26.581  44.601  51.599  1.00 28.06           C
ATOM   4597  O    SER C 177      25.474  45.106  51.645  1.00 28.66           O
ATOM   4598  CB   SER C 177      26.905  44.053  49.185  1.00 29.27           C
ATOM   4599  OG   SER C 177      27.944  44.996  49.050  1.00 31.26           O
ATOM   4600  N    THR C 178      27.582  44.957  52.394  1.00 28.08           N
ATOM   4601  CA   THR C 178      27.463  45.930  53.447  1.00 27.82           C
ATOM   4602  C    THR C 178      28.434  47.109  53.170  1.00 28.85           C
ATOM   4603  O    THR C 178      29.642  46.937  52.979  1.00 26.58           O
ATOM   4604  CB   THR C 178      27.713  45.286  54.816  1.00 27.96           C
ATOM   4605  OG1  THR C 178      26.772  44.212  55.024  1.00 28.77           O
```

```
ATOM   4606  CG2 THR C 178      27.542  46.283  55.945  1.00 27.84           C
ATOM   4607  N   LEU C 179      27.850  48.297  53.099  1.00 28.88           N
ATOM   4608  CA  LEU C 179      28.592  49.543  53.031  1.00 29.30           C
ATOM   4609  C   LEU C 179      28.674  50.103  54.459  1.00 29.88           C
ATOM   4610  O   LEU C 179      27.647  50.471  55.040  1.00 29.77           O
ATOM   4611  CB  LEU C 179      27.875  50.495  52.076  1.00 28.27           C
ATOM   4612  CG  LEU C 179      28.452  51.893  51.896  1.00 28.92           C
ATOM   4613  CD1 LEU C 179      29.776  51.848  51.179  1.00 31.04           C
ATOM   4614  CD2 LEU C 179      27.502  52.744  51.139  1.00 29.49           C
ATOM   4615  N   THR C 180      29.877  50.136  55.031  1.00 31.02           N
ATOM   4616  CA  THR C 180      30.078  50.633  56.396  1.00 33.40           C
ATOM   4617  C   THR C 180      30.728  52.024  56.445  1.00 33.88           C
ATOM   4618  O   THR C 180      31.897  52.189  56.095  1.00 35.21           O
ATOM   4619  CB  THR C 180      30.923  49.645  57.204  1.00 34.69           C
ATOM   4620  OG1 THR C 180      30.298  48.350  57.169  1.00 37.13           O
ATOM   4621  CG2 THR C 180      31.070  50.102  58.664  1.00 35.48           C
ATOM   4622  N   LEU C 181      29.953  53.009  56.890  1.00 33.34           N
ATOM   4623  CA  LEU C 181      30.373  54.392  56.965  1.00 32.93           C
ATOM   4624  C   LEU C 181      30.320  54.822  58.403  1.00 32.00           C
ATOM   4625  O   LEU C 181      29.601  54.237  59.178  1.00 31.46           O
ATOM   4626  CB  LEU C 181      29.395  55.259  56.191  1.00 32.67           C
ATOM   4627  CG  LEU C 181      29.119  54.846  54.751  1.00 34.23           C
ATOM   4628  CD1 LEU C 181      28.050  55.751  54.169  1.00 34.95           C
ATOM   4629  CD2 LEU C 181      30.374  54.953  53.944  1.00 34.23           C
ATOM   4630  N   THR C 182      31.065  55.863  58.755  1.00 31.29           N
ATOM   4631  CA  THR C 182      30.850  56.524  60.031  1.00 31.02           C
ATOM   4632  C   THR C 182      29.499  57.240  59.978  1.00 29.80           C
ATOM   4633  O   THR C 182      29.001  57.564  58.895  1.00 28.60           O
ATOM   4634  CB  THR C 182      31.972  57.530  60.389  1.00 31.44           C
ATOM   4635  OG1 THR C 182      31.883  58.678  59.547  1.00 30.90           O
ATOM   4636  CG2 THR C 182      33.339  56.883  60.269  1.00 32.53           C
ATOM   4637  N   LYS C 183      28.900  57.475  61.143  1.00 29.51           N
ATOM   4638  CA  LYS C 183      27.608  58.147  61.200  1.00 29.23           C
ATOM   4639  C   LYS C 183      27.718  59.555  60.584  1.00 29.79           C
ATOM   4640  O   LYS C 183      26.840  59.995  59.847  1.00 29.30           O
ATOM   4641  CB  LYS C 183      27.087  58.263  62.636  1.00 28.84           C
ATOM   4642  CG  LYS C 183      25.851  59.106  62.707  1.00 28.93           C
ATOM   4643  CD  LYS C 183      25.122  59.027  64.020  1.00 30.12           C
ATOM   4644  CE  LYS C 183      24.373  60.342  64.244  1.00 30.64           C
ATOM   4645  NZ  LYS C 183      23.405  60.297  65.399  1.00 32.25           N
ATOM   4646  N   ASP C 184      28.795  60.248  60.911  1.00 31.11           N
ATOM   4647  CA  ASP C 184      29.068  61.575  60.332  1.00 32.66           C
ATOM   4648  C   ASP C 184      29.007  61.586  58.808  1.00 31.37           C
ATOM   4649  O   ASP C 184      28.301  62.385  58.208  1.00 30.77           O
ATOM   4650  CB  ASP C 184      30.430  62.072  60.785  1.00 34.04           C
ATOM   4651  CG  ASP C 184      30.324  63.194  61.776  1.00 38.33           C
ATOM   4652  OD1 ASP C 184      29.853  62.956  62.929  1.00 41.29           O
ATOM   4653  OD2 ASP C 184      30.688  64.323  61.376  1.00 40.95           O
ATOM   4654  N   GLU C 185      29.753  60.683  58.198  1.00 32.35           N
ATOM   4655  CA  GLU C 185      29.760  60.533  56.751  1.00 33.73           C
ATOM   4656  C   GLU C 185      28.360  60.262  56.249  1.00 32.61           C
ATOM   4657  O   GLU C 185      27.915  60.889  55.301  1.00 31.59           O
ATOM   4658  CB  GLU C 185      30.560  59.317  56.301  1.00 34.14           C
ATOM   4659  CG  GLU C 185      31.992  59.196  56.706  1.00 36.99           C
ATOM   4660  CD  GLU C 185      32.600  57.991  56.013  1.00 37.51           C
ATOM   4661  OE1 GLU C 185      32.712  58.104  54.763  1.00 42.17           O
ATOM   4662  OE2 GLU C 185      32.909  56.955  56.682  1.00 35.39           O
ATOM   4663  N   TYR C 186      27.698  59.274  56.862  1.00 31.93           N
ATOM   4664  CA  TYR C 186      26.362  58.836  56.452  1.00 32.13           C
ATOM   4665  C   TYR C 186      25.357  59.969  56.472  1.00 32.60           C
ATOM   4666  O   TYR C 186      24.521  60.062  55.586  1.00 33.12           O
ATOM   4667  CB  TYR C 186      25.862  57.681  57.363  1.00 31.24           C
ATOM   4668  CG  TYR C 186      24.394  57.332  57.210  1.00 30.47           C
ATOM   4669  CD1 TYR C 186      23.929  56.652  56.082  1.00 29.64           C
ATOM   4670  CD2 TYR C 186      23.470  57.642  58.218  1.00 29.88           C
```

| ATOM | 4671 | CE1 | TYR | C | 186 | 22.580 | 56.319 | 55.940 | 1.00 | 28.80 | C |
| ATOM | 4672 | CE2 | TYR | C | 186 | 22.119 | 57.310 | 58.077 | 1.00 | 27.37 | C |
| ATOM | 4673 | CZ | TYR | C | 186 | 21.691 | 56.653 | 56.955 | 1.00 | 29.22 | C |
| ATOM | 4674 | OH | TYR | C | 186 | 20.355 | 56.342 | 56.821 | 1.00 | 31.17 | O |
| ATOM | 4675 | N | GLU | C | 187 | 25.440 | 60.824 | 57.482 | 1.00 | 34.49 | N |
| ATOM | 4676 | CA | GLU | C | 187 | 24.481 | 61.935 | 57.642 | 1.00 | 36.94 | C |
| ATOM | 4677 | C | GLU | C | 187 | 24.782 | 63.156 | 56.753 | 1.00 | 37.64 | C |
| ATOM | 4678 | O | GLU | C | 187 | 23.996 | 64.101 | 56.735 | 1.00 | 38.27 | O |
| ATOM | 4679 | CB | GLU | C | 187 | 24.413 | 62.403 | 59.099 | 1.00 | 37.47 | C |
| ATOM | 4680 | CG | GLU | C | 187 | 24.196 | 61.304 | 60.137 | 1.00 | 39.39 | C |
| ATOM | 4681 | CD | GLU | C | 187 | 22.762 | 60.906 | 60.320 | 1.00 | 40.91 | C |
| ATOM | 4682 | OE1 | GLU | C | 187 | 22.000 | 61.050 | 59.355 | 1.00 | 44.43 | O |
| ATOM | 4683 | OE2 | GLU | C | 187 | 22.398 | 60.415 | 61.422 | 1.00 | 41.96 | O |
| ATOM | 4684 | N | ARG | C | 188 | 25.898 | 63.151 | 56.029 | 1.00 | 38.37 | N |
| ATOM | 4685 | CA | ARG | C | 188 | 26.175 | 64.240 | 55.093 | 1.00 | 40.07 | C |
| ATOM | 4686 | C | ARG | C | 188 | 25.764 | 63.922 | 53.659 | 1.00 | 39.52 | C |
| ATOM | 4687 | O | ARG | C | 188 | 26.102 | 64.670 | 52.742 | 1.00 | 39.14 | O |
| ATOM | 4688 | CB | ARG | C | 188 | 27.636 | 64.719 | 55.181 | 1.00 | 40.39 | C |
| ATOM | 4689 | CG | ARG | C | 188 | 28.704 | 63.729 | 54.839 | 1.00 | 41.46 | C |
| ATOM | 4690 | CD | ARG | C | 188 | 30.089 | 64.370 | 54.929 | 1.00 | 42.45 | C |
| ATOM | 4691 | NE | ARG | C | 188 | 30.291 | 65.064 | 56.204 | 1.00 | 43.96 | N |
| ATOM | 4692 | CZ | ARG | C | 188 | 31.017 | 64.633 | 57.243 | 1.00 | 44.67 | C |
| ATOM | 4693 | NH1 | ARG | C | 188 | 31.679 | 63.468 | 57.219 | 1.00 | 44.13 | N |
| ATOM | 4694 | NH2 | ARG | C | 188 | 31.094 | 65.408 | 58.331 | 1.00 | 44.72 | N |
| ATOM | 4695 | N | HIS | C | 189 | 25.002 | 62.837 | 53.479 | 1.00 | 39.58 | N |
| ATOM | 4696 | CA | HIS | C | 189 | 24.422 | 62.480 | 52.184 | 1.00 | 39.46 | C |
| ATOM | 4697 | C | HIS | C | 189 | 22.952 | 62.038 | 52.342 | 1.00 | 39.51 | C |
| ATOM | 4698 | O | HIS | C | 189 | 22.555 | 61.595 | 53.412 | 1.00 | 38.67 | O |
| ATOM | 4699 | CB | HIS | C | 189 | 25.262 | 61.381 | 51.540 | 1.00 | 40.39 | C |
| ATOM | 4700 | CG | HIS | C | 189 | 26.684 | 61.783 | 51.308 | 1.00 | 41.75 | C |
| ATOM | 4701 | ND1 | HIS | C | 189 | 27.058 | 62.621 | 50.279 | 1.00 | 42.15 | N |
| ATOM | 4702 | CD2 | HIS | C | 189 | 27.821 | 61.492 | 51.991 | 1.00 | 42.33 | C |
| ATOM | 4703 | CE1 | HIS | C | 189 | 28.365 | 62.821 | 50.332 | 1.00 | 42.94 | C |
| ATOM | 4704 | NE2 | HIS | C | 189 | 28.853 | 62.150 | 51.361 | 1.00 | 42.56 | N |
| ATOM | 4705 | N | ASN | C | 190 | 22.153 | 62.160 | 51.277 | 1.00 | 38.72 | N |
| ATOM | 4706 | CA | ASN | C | 190 | 20.711 | 61.883 | 51.361 | 1.00 | 38.70 | C |
| ATOM | 4707 | C | ASN | C | 190 | 20.260 | 60.529 | 50.803 | 1.00 | 38.32 | C |
| ATOM | 4708 | O | ASN | C | 190 | 19.333 | 59.927 | 51.337 | 1.00 | 37.49 | O |
| ATOM | 4709 | CB | ASN | C | 190 | 19.902 | 62.979 | 50.654 | 1.00 | 38.99 | C |
| ATOM | 4710 | CG | ASN | C | 190 | 19.815 | 64.262 | 51.453 | 1.00 | 39.31 | C |
| ATOM | 4711 | OD1 | ASN | C | 190 | 20.167 | 65.333 | 50.956 | 1.00 | 40.07 | O |
| ATOM | 4712 | ND2 | ASN | C | 190 | 19.344 | 64.165 | 52.695 | 1.00 | 39.43 | N |
| ATOM | 4713 | N | SER | C | 191 | 20.874 | 60.083 | 49.713 | 1.00 | 38.17 | N |
| ATOM | 4714 | CA | SER | C | 191 | 20.367 | 58.919 | 48.976 | 1.00 | 38.32 | C |
| ATOM | 4715 | C | SER | C | 191 | 21.427 | 57.857 | 48.907 | 1.00 | 36.73 | C |
| ATOM | 4716 | O | SER | C | 191 | 22.619 | 58.150 | 48.692 | 1.00 | 36.84 | O |
| ATOM | 4717 | CB | SER | C | 191 | 19.937 | 59.275 | 47.550 | 1.00 | 39.59 | C |
| ATOM | 4718 | OG | SER | C | 191 | 21.038 | 59.169 | 46.640 | 1.00 | 42.09 | O |
| ATOM | 4719 | N | TYR | C | 192 | 20.986 | 56.617 | 49.105 | 1.00 | 34.65 | N |
| ATOM | 4720 | CA | TYR | C | 192 | 21.879 | 55.461 | 49.114 | 1.00 | 33.08 | C |
| ATOM | 4721 | C | TYR | C | 192 | 21.265 | 54.435 | 48.217 | 1.00 | 34.49 | C |
| ATOM | 4722 | O | TYR | C | 192 | 20.043 | 54.217 | 48.265 | 1.00 | 34.16 | O |
| ATOM | 4723 | CB | TYR | C | 192 | 22.033 | 54.921 | 50.523 | 1.00 | 31.26 | C |
| ATOM | 4724 | CG | TYR | C | 192 | 22.842 | 55.855 | 51.356 | 1.00 | 29.82 | C |
| ATOM | 4725 | CD1 | TYR | C | 192 | 24.214 | 55.740 | 51.412 | 1.00 | 28.37 | C |
| ATOM | 4726 | CD2 | TYR | C | 192 | 22.240 | 56.896 | 52.056 | 1.00 | 30.13 | C |
| ATOM | 4727 | CE1 | TYR | C | 192 | 24.966 | 56.609 | 52.149 | 1.00 | 27.74 | C |
| ATOM | 4728 | CE2 | TYR | C | 192 | 23.016 | 57.787 | 52.815 | 1.00 | 28.50 | C |
| ATOM | 4729 | CZ | TYR | C | 192 | 24.364 | 57.631 | 52.835 | 1.00 | 28.27 | C |
| ATOM | 4730 | OH | TYR | C | 192 | 25.142 | 58.486 | 53.558 | 1.00 | 29.53 | O |
| ATOM | 4731 | N | THR | C | 193 | 22.100 | 53.824 | 47.384 | 1.00 | 35.04 | N |
| ATOM | 4732 | CA | THR | C | 193 | 21.612 | 52.920 | 46.353 | 1.00 | 36.71 | C |
| ATOM | 4733 | C | THR | C | 193 | 22.517 | 51.695 | 46.220 | 1.00 | 37.79 | C |
| ATOM | 4734 | O | THR | C | 193 | 23.735 | 51.796 | 46.231 | 1.00 | 37.22 | O |
| ATOM | 4735 | CB | THR | C | 193 | 21.492 | 53.656 | 44.987 | 1.00 | 36.88 | C |

| ATOM | 4736 | OG1 | THR | C | 193 | 20.621 | 54.788 | 45.136 | 1.00 | 37.48 | O |
|------|------|-----|-----|---|-----|--------|--------|--------|------|-------|---|
| ATOM | 4737 | CG2 | THR | C | 193 | 20.957 | 52.746 | 43.881 | 1.00 | 36.30 | C |
| ATOM | 4738 | N | CYS | C | 194 | 21.881 | 50.541 | 46.085 | 1.00 | 39.85 | N |
| ATOM | 4739 | CA | CYS | C | 194 | 22.557 | 49.289 | 45.815 | 1.00 | 41.27 | C |
| ATOM | 4740 | C | CYS | C | 194 | 22.066 | 48.789 | 44.444 | 1.00 | 42.30 | C |
| ATOM | 4741 | O | CYS | C | 194 | 20.860 | 48.580 | 44.243 | 1.00 | 41.38 | O |
| ATOM | 4742 | CB | CYS | C | 194 | 22.210 | 48.293 | 46.920 | 1.00 | 40.87 | C |
| ATOM | 4743 | SG | CYS | C | 194 | 22.715 | 46.622 | 46.570 | 1.00 | 43.72 | S |
| ATOM | 4744 | N | GLU | C | 195 | 22.983 | 48.630 | 43.493 | 1.00 | 44.02 | N |
| ATOM | 4745 | CA | GLU | C | 195 | 22.587 | 48.190 | 42.154 | 1.00 | 46.50 | C |
| ATOM | 4746 | C | GLU | C | 195 | 23.249 | 46.846 | 41.796 | 1.00 | 45.88 | C |
| ATOM | 4747 | O | GLU | C | 195 | 24.482 | 46.713 | 41.805 | 1.00 | 45.59 | O |
| ATOM | 4748 | CB | GLU | C | 195 | 22.858 | 49.273 | 41.090 | 1.00 | 47.58 | C |
| ATOM | 4749 | CG | GLU | C | 195 | 24.328 | 49.670 | 40.914 | 1.00 | 49.64 | C |
| ATOM | 4750 | CD | GLU | C | 195 | 24.609 | 50.345 | 39.569 | 1.00 | 50.04 | C |
| ATOM | 4751 | OE1 | GLU | C | 195 | 23.810 | 51.234 | 39.178 | 1.00 | 52.82 | O |
| ATOM | 4752 | OE2 | GLU | C | 195 | 25.629 | 49.987 | 38.918 | 1.00 | 52.41 | O |
| ATOM | 4753 | N | ALA | C | 196 | 22.391 | 45.865 | 41.511 | 1.00 | 45.35 | N |
| ATOM | 4754 | CA | ALA | C | 196 | 22.780 | 44.507 | 41.142 | 1.00 | 45.71 | C |
| ATOM | 4755 | C | ALA | C | 196 | 22.917 | 44.332 | 39.618 | 1.00 | 45.82 | C |
| ATOM | 4756 | O | ALA | C | 196 | 21.979 | 44.588 | 38.876 | 1.00 | 45.16 | O |
| ATOM | 4757 | CB | ALA | C | 196 | 21.723 | 43.532 | 41.666 | 1.00 | 45.34 | C |
| ATOM | 4758 | N | THR | C | 197 | 24.069 | 43.864 | 39.159 | 1.00 | 46.50 | N |
| ATOM | 4759 | CA | THR | C | 197 | 24.216 | 43.503 | 37.754 | 1.00 | 47.73 | C |
| ATOM | 4760 | C | THR | C | 197 | 24.483 | 42.001 | 37.613 | 1.00 | 47.69 | C |
| ATOM | 4761 | O | THR | C | 197 | 25.373 | 41.441 | 38.254 | 1.00 | 46.91 | O |
| ATOM | 4762 | CB | THR | C | 197 | 25.278 | 44.370 | 37.039 | 1.00 | 48.25 | C |
| ATOM | 4763 | OG1 | THR | C | 197 | 26.466 | 44.465 | 37.838 | 1.00 | 49.42 | O |
| ATOM | 4764 | CG2 | THR | C | 197 | 24.722 | 45.761 | 36.820 | 1.00 | 48.26 | C |
| ATOM | 4765 | N | HIS | C | 198 | 23.665 | 41.368 | 36.780 | 1.00 | 48.31 | N |
| ATOM | 4766 | CA | HIS | C | 198 | 23.582 | 39.928 | 36.693 | 1.00 | 49.11 | C |
| ATOM | 4767 | C | HIS | C | 198 | 23.146 | 39.545 | 35.281 | 1.00 | 50.80 | C |
| ATOM | 4768 | O | HIS | C | 198 | 22.257 | 40.181 | 34.705 | 1.00 | 50.74 | O |
| ATOM | 4769 | CB | HIS | C | 198 | 22.552 | 39.438 | 37.722 | 1.00 | 48.77 | C |
| ATOM | 4770 | CG | HIS | C | 198 | 22.598 | 37.966 | 37.979 | 1.00 | 47.67 | C |
| ATOM | 4771 | ND1 | HIS | C | 198 | 21.531 | 37.132 | 37.720 | 1.00 | 47.45 | N |
| ATOM | 4772 | CD2 | HIS | C | 198 | 23.576 | 37.181 | 38.488 | 1.00 | 47.23 | C |
| ATOM | 4773 | CE1 | HIS | C | 198 | 21.855 | 35.895 | 38.052 | 1.00 | 46.64 | C |
| ATOM | 4774 | NE2 | HIS | C | 198 | 23.090 | 35.898 | 38.522 | 1.00 | 46.30 | N |
| ATOM | 4775 | N | LYS | C | 199 | 23.738 | 38.489 | 34.731 | 1.00 | 52.42 | N |
| ATOM | 4776 | CA | LYS | C | 199 | 23.417 | 38.068 | 33.363 | 1.00 | 53.90 | C |
| ATOM | 4777 | C | LYS | C | 199 | 21.907 | 38.001 | 33.081 | 1.00 | 53.69 | C |
| ATOM | 4778 | O | LYS | C | 199 | 21.504 | 38.040 | 31.923 | 1.00 | 53.53 | O |
| ATOM | 4779 | CB | LYS | C | 199 | 24.089 | 36.722 | 33.014 | 1.00 | 54.50 | C |
| ATOM | 4780 | CG | LYS | C | 199 | 23.435 | 35.475 | 33.634 | 1.00 | 55.17 | C |
| ATOM | 4781 | CD | LYS | C | 199 | 23.609 | 34.227 | 32.756 | 1.00 | 54.96 | C |
| ATOM | 4782 | CE | LYS | C | 199 | 22.792 | 34.320 | 31.469 | 1.00 | 56.13 | C |
| ATOM | 4783 | NZ | LYS | C | 199 | 22.487 | 32.990 | 30.844 | 1.00 | 56.23 | N |
| ATOM | 4784 | N | THR | C | 200 | 21.083 | 37.895 | 34.125 | 1.00 | 53.77 | N |
| ATOM | 4785 | CA | THR | C | 200 | 19.630 | 37.783 | 33.958 | 1.00 | 54.52 | C |
| ATOM | 4786 | C | THR | C | 200 | 18.943 | 39.051 | 33.436 | 1.00 | 55.26 | C |
| ATOM | 4787 | O | THR | C | 200 | 17.767 | 39.000 | 33.032 | 1.00 | 55.20 | O |
| ATOM | 4788 | CB | THR | C | 200 | 18.928 | 37.349 | 35.276 | 1.00 | 54.93 | C |
| ATOM | 4789 | OG1 | THR | C | 200 | 19.417 | 38.125 | 36.380 | 1.00 | 54.13 | O |
| ATOM | 4790 | CG2 | THR | C | 200 | 19.180 | 35.876 | 35.535 | 1.00 | 55.14 | C |
| ATOM | 4791 | N | SER | C | 201 | 19.653 | 40.181 | 33.454 | 1.00 | 55.54 | N |
| ATOM | 4792 | CA | SER | C | 201 | 19.146 | 41.399 | 32.821 | 1.00 | 56.14 | C |
| ATOM | 4793 | C | SER | C | 201 | 20.277 | 42.250 | 32.278 | 1.00 | 56.13 | C |
| ATOM | 4794 | O | SER | C | 201 | 21.445 | 42.007 | 32.562 | 1.00 | 55.87 | O |
| ATOM | 4795 | CB | SER | C | 201 | 18.292 | 42.216 | 33.797 | 1.00 | 56.35 | C |
| ATOM | 4796 | OG | SER | C | 201 | 17.646 | 43.294 | 33.139 | 1.00 | 57.01 | O |
| ATOM | 4797 | N | THR | C | 202 | 19.897 | 43.236 | 31.474 | 1.00 | 56.69 | N |
| ATOM | 4798 | CA | THR | C | 202 | 20.826 | 44.193 | 30.872 | 1.00 | 57.10 | C |
| ATOM | 4799 | C | THR | C | 202 | 20.978 | 45.415 | 31.779 | 1.00 | 56.82 | C |
| ATOM | 4800 | O | THR | C | 202 | 22.072 | 45.978 | 31.924 | 1.00 | 57.26 | O |

| ATOM | 4801 | CB | THR | C | 202 | 20.298 | 44.640 | 29.490 | 1.00 | 57.46 | C |
|------|------|------|-----|---|-----|--------|--------|--------|------|-------|---|
| ATOM | 4802 | OG1 | THR | C | 202 | 18.862 | 44.650 | 29.513 | 1.00 | 57.29 | O |
| ATOM | 4803 | CG2 | THR | C | 202 | 20.752 | 43.674 | 28.405 | 1.00 | 57.67 | C |
| ATOM | 4804 | N | SER | C | 203 | 19.860 | 45.819 | 32.375 | 1.00 | 56.37 | N |
| ATOM | 4805 | CA | SER | C | 203 | 19.812 | 46.936 | 33.307 | 1.00 | 55.45 | C |
| ATOM | 4806 | C | SER | C | 203 | 19.932 | 46.390 | 34.724 | 1.00 | 54.61 | C |
| ATOM | 4807 | O | SER | C | 203 | 19.377 | 45.323 | 35.013 | 1.00 | 54.28 | O |
| ATOM | 4808 | CB | SER | C | 203 | 18.480 | 47.655 | 33.166 | 1.00 | 55.58 | C |
| ATOM | 4809 | OG | SER | C | 203 | 17.426 | 46.714 | 33.281 | 1.00 | 56.41 | O |
| ATOM | 4810 | N | PRO | C | 204 | 20.620 | 47.130 | 35.619 | 1.00 | 53.04 | N |
| ATOM | 4811 | CA | PRO | C | 204 | 20.767 | 46.656 | 36.990 | 1.00 | 52.10 | C |
| ATOM | 4812 | C | PRO | C | 204 | 19.433 | 46.558 | 37.747 | 1.00 | 51.16 | C |
| ATOM | 4813 | O | PRO | C | 204 | 18.442 | 47.158 | 37.324 | 1.00 | 51.72 | O |
| ATOM | 4814 | CB | PRO | C | 204 | 21.680 | 47.709 | 37.633 | 1.00 | 52.68 | C |
| ATOM | 4815 | CG | PRO | C | 204 | 22.327 | 48.435 | 36.500 | 1.00 | 52.58 | C |
| ATOM | 4816 | CD | PRO | C | 204 | 21.299 | 48.420 | 35.417 | 1.00 | 53.27 | C |
| ATOM | 4817 | N | ILE | C | 205 | 19.389 | 45.779 | 38.825 | 1.00 | 48.72 | N |
| ATOM | 4818 | CA | ILE | C | 205 | 18.262 | 45.881 | 39.746 | 1.00 | 48.20 | C |
| ATOM | 4819 | C | ILE | C | 205 | 18.677 | 46.881 | 40.812 | 1.00 | 47.23 | C |
| ATOM | 4820 | O | ILE | C | 205 | 19.715 | 46.710 | 41.461 | 1.00 | 46.49 | O |
| ATOM | 4821 | CB | ILE | C | 205 | 17.841 | 44.542 | 40.381 | 1.00 | 48.19 | C |
| ATOM | 4822 | CG1 | ILE | C | 205 | 17.256 | 43.619 | 39.304 | 1.00 | 48.37 | C |
| ATOM | 4823 | CG2 | ILE | C | 205 | 16.823 | 44.791 | 41.508 | 1.00 | 48.02 | C |
| ATOM | 4824 | CD1 | ILE | C | 205 | 16.703 | 42.297 | 39.819 | 1.00 | 47.92 | C |
| ATOM | 4825 | N | VAL | C | 206 | 17.865 | 47.924 | 40.980 | 1.00 | 46.12 | N |
| ATOM | 4826 | CA | VAL | C | 206 | 18.232 | 49.054 | 41.825 | 1.00 | 45.35 | C |
| ATOM | 4827 | C | VAL | C | 206 | 17.310 | 49.119 | 43.031 | 1.00 | 43.30 | C |
| ATOM | 4828 | O | VAL | C | 206 | 16.095 | 48.978 | 42.903 | 1.00 | 43.37 | O |
| ATOM | 4829 | CB | VAL | C | 206 | 18.208 | 50.404 | 41.045 | 1.00 | 46.57 | C |
| ATOM | 4830 | CG1 | VAL | C | 206 | 19.272 | 50.410 | 39.946 | 1.00 | 47.33 | C |
| ATOM | 4831 | CG2 | VAL | C | 206 | 16.833 | 50.681 | 40.436 | 1.00 | 47.53 | C |
| ATOM | 4832 | N | LYS | C | 207 | 17.906 | 49.283 | 44.206 | 1.00 | 40.51 | N |
| ATOM | 4833 | CA | LYS | C | 207 | 17.159 | 49.593 | 45.404 | 1.00 | 38.94 | C |
| ATOM | 4834 | C | LYS | C | 207 | 17.843 | 50.757 | 46.073 | 1.00 | 37.07 | C |
| ATOM | 4835 | O | LYS | C | 207 | 19.057 | 50.823 | 46.098 | 1.00 | 35.75 | O |
| ATOM | 4836 | CB | LYS | C | 207 | 17.110 | 48.391 | 46.348 | 1.00 | 38.65 | C |
| ATOM | 4837 | CG | LYS | C | 207 | 16.294 | 47.248 | 45.813 | 1.00 | 39.10 | C |
| ATOM | 4838 | CD | LYS | C | 207 | 14.833 | 47.521 | 45.934 | 1.00 | 39.94 | C |
| ATOM | 4839 | CE | LYS | C | 207 | 14.028 | 46.623 | 45.035 | 1.00 | 40.14 | C |
| ATOM | 4840 | NZ | LYS | C | 207 | 12.626 | 46.575 | 45.534 | 1.00 | 41.65 | N |
| ATOM | 4841 | N | SER | C | 208 | 17.050 | 51.687 | 46.590 | 1.00 | 37.22 | N |
| ATOM | 4842 | CA | SER | C | 208 | 17.587 | 52.857 | 47.298 | 1.00 | 37.31 | C |
| ATOM | 4843 | C | SER | C | 208 | 16.680 | 53.313 | 48.420 | 1.00 | 35.98 | C |
| ATOM | 4844 | O | SER | C | 208 | 15.522 | 52.895 | 48.550 | 1.00 | 34.24 | O |
| ATOM | 4845 | CB | SER | C | 208 | 17.823 | 54.026 | 46.331 | 1.00 | 38.16 | C |
| ATOM | 4846 | OG | SER | C | 208 | 16.595 | 54.550 | 45.862 | 1.00 | 41.31 | O |
| ATOM | 4847 | N | PHE | C | 209 | 17.240 | 54.152 | 49.261 | 1.00 | 35.20 | N |
| ATOM | 4848 | CA | PHE | C | 209 | 16.437 | 54.842 | 50.235 | 1.00 | 36.13 | C |
| ATOM | 4849 | C | PHE | C | 209 | 17.017 | 56.228 | 50.362 | 1.00 | 37.07 | C |
| ATOM | 4850 | O | PHE | C | 209 | 18.204 | 56.429 | 50.094 | 1.00 | 37.55 | O |
| ATOM | 4851 | CB | PHE | C | 209 | 16.412 | 54.103 | 51.593 | 1.00 | 34.16 | C |
| ATOM | 4852 | CG | PHE | C | 209 | 17.728 | 54.098 | 52.296 | 1.00 | 33.00 | C |
| ATOM | 4853 | CD1 | PHE | C | 209 | 18.093 | 55.148 | 53.133 | 1.00 | 31.76 | C |
| ATOM | 4854 | CD2 | PHE | C | 209 | 18.624 | 53.063 | 52.102 | 1.00 | 31.82 | C |
| ATOM | 4855 | CE1 | PHE | C | 209 | 19.329 | 55.151 | 53.766 | 1.00 | 31.21 | C |
| ATOM | 4856 | CE2 | PHE | C | 209 | 19.854 | 53.062 | 52.739 | 1.00 | 31.75 | C |
| ATOM | 4857 | CZ | PHE | C | 209 | 20.215 | 54.108 | 53.573 | 1.00 | 31.15 | C |
| ATOM | 4858 | N | ASN | C | 210 | 16.158 | 57.159 | 50.757 | 1.00 | 39.52 | N |
| ATOM | 4859 | CA | ASN | C | 210 | 16.532 | 58.516 | 51.118 | 1.00 | 41.19 | C |
| ATOM | 4860 | C | ASN | C | 210 | 16.392 | 58.671 | 52.633 | 1.00 | 41.94 | C |
| ATOM | 4861 | O | ASN | C | 210 | 15.421 | 58.186 | 53.213 | 1.00 | 41.47 | O |
| ATOM | 4862 | CB | ASN | C | 210 | 15.627 | 59.533 | 50.386 | 1.00 | 41.91 | C |
| ATOM | 4863 | CG | ASN | C | 210 | 16.336 | 60.239 | 49.217 | 1.00 | 42.81 | C |
| ATOM | 4864 | OD1 | ASN | C | 210 | 16.415 | 61.472 | 49.187 | 1.00 | 42.59 | O |
| ATOM | 4865 | ND2 | ASN | C | 210 | 16.852 | 59.460 | 48.258 | 1.00 | 42.87 | N |

| ATOM | 4866 | N | ARG | C | 211 | 17.337 | 59.363 | 53.266 | 1.00 | 43.23 | N |
|------|------|-----|-----|---|-----|--------|--------|--------|------|-------|---|
| ATOM | 4867 | CA | ARG | C | 211 | 17.366 | 59.488 | 54.727 | 1.00 | 44.51 | C |
| ATOM | 4868 | C | ARG | C | 211 | 16.200 | 60.219 | 55.410 | 1.00 | 46.31 | C |
| ATOM | 4869 | O | ARG | C | 211 | 15.737 | 59.789 | 56.475 | 1.00 | 47.05 | O |
| ATOM | 4870 | CB | ARG | C | 211 | 18.642 | 60.184 | 55.160 | 1.00 | 44.91 | C |
| ATOM | 4871 | CG | ARG | C | 211 | 19.861 | 59.337 | 55.130 | 1.00 | 44.36 | C |
| ATOM | 4872 | CD | ARG | C | 211 | 20.868 | 59.887 | 56.123 | 1.00 | 45.89 | C |
| ATOM | 4873 | NE | ARG | C | 211 | 21.281 | 61.241 | 55.768 | 1.00 | 46.76 | N |
| ATOM | 4874 | CZ | ARG | C | 211 | 20.822 | 62.360 | 56.326 | 1.00 | 46.93 | C |
| ATOM | 4875 | NH1 | ARG | C | 211 | 19.944 | 62.335 | 57.324 | 1.00 | 46.94 | N |
| ATOM | 4876 | NH2 | ARG | C | 211 | 21.276 | 63.523 | 55.888 | 1.00 | 46.59 | N |
| ATOM | 4877 | N | ASN | C | 212 | 15.752 | 61.341 | 54.857 | 1.00 | 47.36 | N |
| ATOM | 4878 | CA | ASN | C | 212 | 14.702 | 62.127 | 55.528 | 1.00 | 48.68 | C |
| ATOM | 4879 | C | ASN | C | 212 | 13.357 | 61.941 | 54.844 | 1.00 | 48.41 | C |
| ATOM | 4880 | O | ASN | C | 212 | 13.031 | 60.839 | 54.404 | 1.00 | 48.87 | O |
| ATOM | 4881 | CB | ASN | C | 212 | 15.065 | 63.626 | 55.601 | 1.00 | 49.50 | C |
| ATOM | 4882 | CG | ASN | C | 212 | 16.483 | 63.886 | 56.153 | 1.00 | 50.14 | C |
| ATOM | 4883 | OD1 | ASN | C | 212 | 16.730 | 63.858 | 57.365 | 1.00 | 50.57 | O |
| ATOM | 4884 | ND2 | ASN | C | 212 | 17.407 | 64.178 | 55.252 | 1.00 | 50.84 | N |
| TER | 4885 | | ASN | C | 212 | | | | | | |
| ATOM | 4886 | N | GLU | D | 1 | 27.748 | 1.817 | 65.133 | 1.00 | 33.39 | N |
| ATOM | 4887 | CA | GLU | D | 1 | 28.482 | 2.696 | 64.172 | 1.00 | 32.42 | C |
| ATOM | 4888 | C | GLU | D | 1 | 29.638 | 3.401 | 64.897 | 1.00 | 30.03 | C |
| ATOM | 4889 | O | GLU | D | 1 | 29.555 | 3.706 | 66.086 | 1.00 | 29.10 | O |
| ATOM | 4890 | CB | GLU | D | 1 | 27.535 | 3.742 | 63.571 | 1.00 | 33.23 | C |
| ATOM | 4891 | CG | GLU | D | 1 | 28.161 | 4.559 | 62.409 | 1.00 | 35.78 | C |
| ATOM | 4892 | CD | GLU | D | 1 | 27.293 | 5.685 | 61.898 | 1.00 | 37.32 | C |
| ATOM | 4893 | OE1 | GLU | D | 1 | 26.109 | 5.794 | 62.248 | 1.00 | 44.39 | O |
| ATOM | 4894 | OE2 | GLU | D | 1 | 27.873 | 6.532 | 61.032 | 1.00 | 42.19 | O |
| ATOM | 4895 | N | VAL | D | 2 | 30.727 | 3.658 | 64.182 | 1.00 | 27.22 | N |
| ATOM | 4896 | CA | VAL | D | 2 | 31.854 | 4.376 | 64.775 | 1.00 | 24.40 | C |
| ATOM | 4897 | C | VAL | D | 2 | 31.539 | 5.865 | 64.886 | 1.00 | 22.78 | C |
| ATOM | 4898 | O | VAL | D | 2 | 31.046 | 6.462 | 63.937 | 1.00 | 23.58 | O |
| ATOM | 4899 | CB | VAL | D | 2 | 33.116 | 4.168 | 63.948 | 1.00 | 24.14 | C |
| ATOM | 4900 | CG1 | VAL | D | 2 | 34.266 | 4.993 | 64.510 | 1.00 | 24.31 | C |
| ATOM | 4901 | CG2 | VAL | D | 2· | 33.444 | 2.689 | 63.923 | 1.00 | 23.10 | C |
| ATOM | 4902 | N | LYS | D | 3 | 31.822 | 6.452 | 66.048 | 1.00 | 22.13 | N |
| ATOM | 4903 | CA | LYS | D | 3 | 31.741 | 7.896 | 66.223 | 1.00 | 23.27 | C |
| ATOM | 4904 | C | LYS | D | 3 | 33.032 | 8.446 | 66.791 | 1.00 | 22.02 | C |
| ATOM | 4905 | O | LYS | D | 3 | 33.494 | 8.036 | 67.844 | 1.00 | 21.20 | O |
| ATOM | 4906 | CB | LYS | D | 3 | 30.609 | 8.321 | 67.160 | 1.00 | 25.34 | C |
| ATOM | 4907 | CG | LYS | D | 3 | 30.147 | 9.709 | 66.842 | 1.00 | 26.75 | C |
| ATOM | 4908 | CD | LYS | D | 3 | 30.150 | 10.628 | 67.980 | 1.00 | 30.68 | C |
| ATOM | 4909 | CE | LYS | D | 3 | 29.459 | 11.911 | 67.521 | 1.00 | 32.48 | C |
| ATOM | 4910 | NZ | LYS | D | 3 | 29.656 | 13.039 | 68.465 | 1.00 | 36.70 | N |
| ATOM | 4911 | N | VAL | D | 4 | 33.572 | 9.439 | 66.099 | 1.00 | 22.96 | N |
| ATOM | 4912 | CA | VAL | D | 4 | 34.763 | 10.166 | 66.543 | 1.00 | 21.40 | C |
| ATOM | 4913 | C | VAL | D | 4 | 34.360 | 11.586 | 66.930 | 1.00 | 20.78 | C |
| ATOM | 4914 | O | VAL | D | 4 | 33.718 | 12.287 | 66.167 | 1.00 | 21.89 | O |
| ATOM | 4915 | CB | VAL | D | 4 | 35.806 | 10.196 | 65.398 | 1.00 | 21.12 | C |
| ATOM | 4916 | CG1 | VAL | D | 4 | 37.145 | 10.784 | 65.886 | 1.00 | 20.94 | C |
| ATOM | 4917 | CG2 | VAL | D | 4 | 36.023 | 8.812 | 64.884 | 1.00 | 21.90 | C |
| ATOM | 4918 | N | GLU | D | 5 | 34.749 | 12.019 | 68.112 | 1.00 | 22.86 | N |
| ATOM | 4919 | CA | GLU | D | 5 | 34.289 | 13.281 | 68.637 | 1.00 | 26.54 | C |
| ATOM | 4920 | C | GLU | D | 5 | 35.408 | 14.069 | 69.325 | 1.00 | 25.44 | C |
| ATOM | 4921 | O | GLU | D | 5 | 35.881 | 13.685 | 70.397 | 1.00 | 25.06 | O |
| ATOM | 4922 | CB | GLU | D | 5 | 33.146 | 13.087 | 69.640 | 1.00 | 27.84 | C |
| ATOM | 4923 | CG | GLU | D | 5 | 32.739 | 14.428 | 70.209 | 1.00 | 30.39 | C |
| ATOM | 4924 | CD | GLU | D | 5 | 31.552 | 14.359 | 71.107 | 1.00 | 32.60 | C |
| ATOM | 4925 | OE1 | GLU | D | 5 | 31.562 | 13.560 | 72.084 | 1.00 | 35.49 | O |
| ATOM | 4926 | OE2 | GLU | D | 5 | 30.613 | 15.136 | 70.827 | 1.00 | 39.23 | O |
| ATOM | 4927 | N | GLU | D | 6 | 35.774 | 15.193 | 68.703 | 1.00 | 25.10 | N |
| ATOM | 4928 | CA | GLU | D | 6 | 36.849 | 16.060 | 69.180 | 1.00 | 24.48 | C |
| ATOM | 4929 | C | GLU | D | 6 | 36.320 | 17.111 | 70.122 | 1.00 | 23.76 | C |
| ATOM | 4930 | O | GLU | D | 6 | 35.200 | 17.549 | 69.972 | 1.00 | 22.04 | O |

| ATOM | 4931 | CB | GLU | D | 6 | 37.496 | 16.831 | 68.012 | 1.00 | 23.56 | C |
|------|------|-----|-----|---|----|--------|--------|--------|------|-------|---|
| ATOM | 4932 | CG | GLU | D | 6 | 38.123 | 15.979 | 66.927 | 1.00 | 22.55 | C |
| ATOM | 4933 | CD | GLU | D | 6 | 37.160 | 15.579 | 65.877 | 1.00 | 23.54 | C |
| ATOM | 4934 | OE1 | GLU | D | 6 | 35.960 | 15.907 | 66.046 | 1.00 | 27.36 | O |
| ATOM | 4935 | OE2 | GLU | D | 6 | 37.583 | 14.943 | 64.887 | 1.00 | 22.40 | O |
| ATOM | 4936 | N | SER | D | 7 | 37.187 | 17.558 | 71.035 | 1.00 | 24.12 | N |
| ATOM | 4937 | CA | SER | D | 7 | 36.940 | 18.717 | 71.877 | 1.00 | 24.44 | C |
| ATOM | 4938 | C | SER | D | 7 | 38.226 | 19.433 | 72.270 | 1.00 | 23.13 | C |
| ATOM | 4939 | O | SER | D | 7 | 39.338 | 18.914 | 72.091 | 1.00 | 22.71 | O |
| ATOM | 4940 | CB | SER | D | 7 | 36.282 | 18.282 | 73.177 | 1.00 | 24.78 | C |
| ATOM | 4941 | OG | SER | D | 7 | 37.135 | 17.382 | 73.837 | 1.00 | 25.86 | O |
| ATOM | 4942 | N | GLY | D | 8 | 38.031 | 20.604 | 72.858 | 1.00 | 21.56 | N |
| ATOM | 4943 | CA | GLY | D | 8 | 39.078 | 21.321 | 73.565 | 1.00 | 23.50 | C |
| ATOM | 4944 | C | GLY | D | 8 | 39.615 | 22.512 | 72.769 | 1.00 | 24.33 | C |
| ATOM | 4945 | O | GLY | D | 8 | 40.449 | 23.242 | 73.253 | 1.00 | 25.89 | O |
| ATOM | 4946 | N | GLY | D | 9 | 39.132 | 22.701 | 71.554 | 1.00 | 25.35 | N |
| ATOM | 4947 | CA | GLY | D | 9 | 39.484 | 23.877 | 70.760 | 1.00 | 27.29 | C |
| ATOM | 4948 | C | GLY | D | 9 | 38.968 | 25.193 | 71.334 | 1.00 | 27.96 | C |
| ATOM | 4949 | O | GLY | D | 9 | 38.009 | 25.231 | 72.105 | 1.00 | 28.91 | O |
| ATOM | 4950 | N | GLY | D | 10 | 39.606 | 26.281 | 70.928 | 1.00 | 26.03 | N |
| ATOM | 4951 | CA | GLY | D | 10 | 39.222 | 27.591 | 71.379 | 1.00 | 24.98 | C |
| ATOM | 4952 | C | GLY | D | 10 | 40.202 | 28.638 | 70.866 | 1.00 | 24.02 | C |
| ATOM | 4953 | O | GLY | D | 10 | 40.834 | 28.472 | 69.827 | 1.00 | 22.00 | O |
| ATOM | 4954 | N | LEU | D | 11 | 40.309 | 29.717 | 71.625 | 1.00 | 24.07 | N |
| ATOM | 4955 | CA | LEU | D | 11 | 41.124 | 30.859 | 71.275 | 1.00 | 23.43 | C |
| ATOM | 4956 | C | LEU | D | 11 | 42.397 | 30.842 | 72.071 | 1.00 | 22.40 | C |
| ATOM | 4957 | O | LEU | D | 11 | 42.371 | 30.685 | 73.291 | 1.00 | 19.08 | O |
| ATOM | 4958 | CB | LEU | D | 11 | 40.372 | 32.151 | 71.590 | 1.00 | 24.11 | C |
| ATOM | 4959 | CG | LEU | D | 11 | 41.138 | 33.440 | 71.261 | 1.00 | 24.90 | C |
| ATOM | 4960 | CD1 | LEU | D | 11 | 41.603 | 33.461 | 69.807 | 1.00 | 25.44 | C |
| ATOM | 4961 | CD2 | LEU | D | 11 | 40.275 | 34.631 | 71.543 | 1.00 | 25.38 | C |
| ATOM | 4962 | N | VAL | D | 12 | 43.535 | 31.077 | 71.407 | 1.00 | 21.40 | N |
| ATOM | 4963 | CA | VAL | D | 12 | 44.760 | 31.291 | 72.141 | 1.00 | 21.46 | C |
| ATOM | 4964 | C | VAL | D | 12 | 45.575 | 32.370 | 71.443 | 1.00 | 21.62 | C |
| ATOM | 4965 | O | VAL | D | 12 | 45.440 | 32.535 | 70.238 | 1.00 | 18.85 | O |
| ATOM | 4966 | CB | VAL | D | 12 | 45.600 | 29.981 | 72.261 | 1.00 | 22.84 | C |
| ATOM | 4967 | CG1 | VAL | D | 12 | 46.168 | 29.556 | 70.929 | 1.00 | 23.01 | C |
| ATOM | 4968 | CG2 | VAL | D | 12 | 46.705 | 30.146 | 73.257 | 1.00 | 24.65 | C |
| ATOM | 4969 | N | GLN | D | 13 | 46.401 | 33.093 | 72.200 | 1.00 | 22.72 | N |
| ATOM | 4970 | CA | GLN | D | 13 | 47.274 | 34.130 | 71.592 | 1.00 | 23.14 | C |
| ATOM | 4971 | C | GLN | D | 13 | 48.413 | 33.450 | 70.901 | 1.00 | 20.21 | C |
| ATOM | 4972 | O | GLN | D | 13 | 48.853 | 32.417 | 71.336 | 1.00 | 18.32 | O |
| ATOM | 4973 | CB | GLN | D | 13 | 47.856 | 35.100 | 72.629 | 1.00 | 23.39 | C |
| ATOM | 4974 | CG | GLN | D | 13 | 46.833 | 35.906 | 73.369 | 1.00 | 26.49 | C |
| ATOM | 4975 | CD | GLN | D | 13 | 47.415 | 36.653 | 74.555 | 1.00 | 28.03 | C |
| ATOM | 4976 | OE1 | GLN | D | 13 | 48.311 | 37.494 | 74.406 | 1.00 | 31.41 | O |
| ATOM | 4977 | NE2 | GLN | D | 13 | 46.885 | 36.373 | 75.742 | 1.00 | 30.11 | N |
| ATOM | 4978 | N | PRO | D | 14 | 48.923 | 34.047 | 69.799 | 1.00 | 20.55 | N |
| ATOM | 4979 | CA | PRO | D | 14 | 50.116 | 33.500 | 69.208 | 1.00 | 19.79 | C |
| ATOM | 4980 | C | PRO | D | 14 | 51.221 | 33.391 | 70.224 | 1.00 | 19.59 | C |
| ATOM | 4981 | O | PRO | D | 14 | 51.401 | 34.309 | 71.033 | 1.00 | 21.59 | O |
| ATOM | 4982 | CB | PRO | D | 14 | 50.475 | 34.548 | 68.147 | 1.00 | 20.39 | C |
| ATOM | 4983 | CG | PRO | D | 14 | 49.197 | 35.088 | 67.776 | 1.00 | 21.73 | C |
| ATOM | 4984 | CD | PRO | D | 14 | 48.442 | 35.202 | 69.043 | 1.00 | 20.97 | C |
| ATOM | 4985 | N | GLY | D | 15 | 51.970 | 32.291 | 70.158 | 1.00 | 20.61 | N |
| ATOM | 4986 | CA | GLY | D | 15 | 52.992 | 31.951 | 71.121 | 1.00 | 19.52 | C |
| ATOM | 4987 | C | GLY | D | 15 | 52.425 | 31.063 | 72.229 | 1.00 | 20.24 | C |
| ATOM | 4988 | O | GLY | D | 15 | 53.165 | 30.596 | 73.103 | 1.00 | 20.86 | O |
| ATOM | 4989 | N | GLY | D | 16 | 51.127 | 30.814 | 72.192 | 1.00 | 20.75 | N |
| ATOM | 4990 | CA | GLY | D | 16 | 50.457 | 30.070 | 73.267 | 1.00 | 18.84 | C |
| ATOM | 4991 | C | GLY | D | 16 | 50.386 | 28.606 | 72.921 | 1.00 | 19.34 | C |
| ATOM | 4992 | O | GLY | D | 16 | 50.911 | 28.159 | 71.885 | 1.00 | 18.59 | O |
| ATOM | 4993 | N | SER | D | 17 | 49.698 | 27.869 | 73.788 | 1.00 | 19.95 | N |
| ATOM | 4994 | CA | SER | D | 17 | 49.659 | 26.416 | 73.791 | 1.00 | 20.59 | C |
| ATOM | 4995 | C | SER | D | 17 | 48.199 | 26.003 | 74.002 | 1.00 | 21.09 | C |

| ATOM | 4996 | O | SER D | 17 | 47.420 | 26.734 | 74.609 | 1.00 18.96 | O |
| ATOM | 4997 | CB | SER D | 17 | 50.424 | 25.886 | 75.002 | 1.00 22.68 | C |
| ATOM | 4998 | OG | SER D | 17 | 51.825 | 26.088 | 74.886 | 1.00 28.21 | O |
| ATOM | 4999 | N | MET D | 18 | 47.853 | 24.822 | 73.528 | 1.00 21.68 | N |
| ATOM | 5000 | CA | MET D | 18 | 46.510 | 24.268 | 73.694 | 1.00 22.96 | C |
| ATOM | 5001 | C | MET D | 18 | 46.638 | 22.768 | 73.523 | 1.00 23.10 | C |
| ATOM | 5002 | O | MET D | 18 | 47.487 | 22.295 | 72.743 | 1.00 23.52 | O |
| ATOM | 5003 | CB | MET D | 18 | 45.599 | 24.867 | 72.632 | 1.00 24.23 | C |
| ATOM | 5004 | CG | MET D | 18 | 44.155 | 24.365 | 72.617 | 1.00 23.33 | C |
| ATOM | 5005 | SD | MET D | 18 | 43.193 | 25.159 | 71.354 | 1.00 24.36 | S |
| ATOM | 5006 | CE | MET D | 18 | 43.617 | 26.877 | 71.614 | 1.00 26.97 | C |
| ATOM | 5007 | N | LYS D | 19 | 45.818 | 22.024 | 74.266 | 1.00 23.59 | N |
| ATOM | 5008 | CA | LYS D | 19 | 45.734 | 20.584 | 74.107 | 1.00 22.93 | C |
| ATOM | 5009 | C | LYS D | 19 | 44.317 | 20.227 | 73.694 | 1.00 23.37 | C |
| ATOM | 5010 | O | LYS D | 19 | 43.359 | 20.595 | 74.360 | 1.00 22.23 | O |
| ATOM | 5011 | CB | LYS D | 19 | 46.125 | 19.889 | 75.402 | 1.00 23.31 | C |
| ATOM | 5012 | CG | LYS D | 19 | 46.264 | 18.394 | 75.295 | 1.00 23.50 | C |
| ATOM | 5013 | CD | LYS D | 19 | 46.949 | 17.829 | 76.508 | 1.00 25.17 | C |
| ATOM | 5014 | CE | LYS D | 19 | 47.353 | 16.387 | 76.281 | 1.00 25.74 | C |
| ATOM | 5015 | NZ | LYS D | 19 | 48.238 | 15.957 | 77.379 | 1.00 28.09 | N |
| ATOM | 5016 | N | ILE D | 20 | 44.182 | 19.535 | 72.579 | 1.00 21.99 | N |
| ATOM | 5017 | CA | ILE D | 20 | 42.860 | 19.090 | 72.143 | 1.00 22.22 | C |
| ATOM | 5018 | C | ILE D | 20 | 42.852 | 17.570 | 72.192 | 1.00 21.86 | C |
| ATOM | 5019 | O | ILE D | 20 | 43.899 | 16.915 | 72.396 | 1.00 20.76 | O |
| ATOM | 5020 | CB | ILE D | 20 | 42.477 | 19.610 | 70.725 | 1.00 21.57 | C |
| ATOM | 5021 | CG1 | ILE D | 20 | 43.517 | 19.148 | 69.692 | 1.00 20.75 | C |
| ATOM | 5022 | CG2 | ILE D | 20 | 42.360 | 21.146 | 70.725 | 1.00 22.26 | C |
| ATOM | 5023 | CD1 | ILE D | 20 | 43.266 | 19.635 | 68.287 | 1.00 20.43 | C |
| ATOM | 5024 | N | SER D | 21 | 41.664 | 17.003 | 72.054 | 1.00 22.86 | N |
| ATOM | 5025 | CA | SER D | 21 | 41.513 | 15.570 | 72.182 | 1.00 23.52 | C |
| ATOM | 5026 | C | SER D | 21 | 40.296 | 15.094 | 71.428 | 1.00 23.48 | C |
| ATOM | 5027 | O | SER D | 21 | 39.479 | 15.885 | 70.966 | 1.00 22.34 | O |
| ATOM | 5028 | CB | SER D | 21 | 41.407 | 15.168 | 73.651 | 1.00 25.45 | C |
| ATOM | 5029 | OG | SER D | 21 | 40.135 | 15.525 | 74.183 | 1.00 25.58 | O |
| ATOM | 5030 | N | CYS D | 22 | 40.210 | 13.792 | 71.240 | 1.00 24.08 | N |
| ATOM | 5031 | CA | CYS D | 22 | 38.967 | 13.223 | 70.740 | 1.00 25.15 | C |
| ATOM | 5032 | C | CYS D | 22 | 38.801 | 11.856 | 71.327 | 1.00 23.81 | C |
| ATOM | 5033 | O | CYS D | 22 | 39.778 | 11.170 | 71.596 | 1.00 24.37 | O |
| ATOM | 5034 | CB | CYS D | 22 | 38.937 | 13.138 | 69.204 | 1.00 26.72 | C |
| ATOM | 5035 | SG | CYS D | 22 | 40.120 | 12.015 | 68.549 | 1.00 29.99 | S |
| ATOM | 5036 | N | VAL D | 23 | 37.544 | 11.485 | 71.523 | 1.00 24.35 | N |
| ATOM | 5037 | CA | VAL D | 23 | 37.191 | 10.148 | 71.988 | 1.00 23.34 | C |
| ATOM | 5038 | C | VAL D | 23 | 36.490 | 9.383 | 70.850 | 1.00 20.72 | C |
| ATOM | 5039 | O | VAL D | 23 | 35.736 | 9.948 | 70.070 | 1.00 19.51 | O |
| ATOM | 5040 | CB | VAL D | 23 | 36.313 | 10.259 | 73.258 | 1.00 24.29 | C |
| ATOM | 5041 | CG1 | VAL D | 23 | 34.990 | 10.978 | 72.945 | 1.00 25.62 | C |
| ATOM | 5042 | CG2 | VAL D | 23 | 36.083 | 8.895 | 73.892 | 1.00 25.74 | C |
| ATOM | 5043 | N | VAL D | 24 | 36.759 | 8.089 | 70.764 | 1.00 21.91 | N |
| ATOM | 5044 | CA | VAL D | 24 | 36.162 | 7.249 | 69.761 | 1.00 22.51 | C |
| ATOM | 5045 | C | VAL D | 24 | 35.393 | 6.078 | 70.419 | 1.00 25.25 | C |
| ATOM | 5046 | O | VAL D | 24 | 35.863 | 5.471 | 71.385 | 1.00 24.25 | O |
| ATOM | 5047 | CB | VAL D | 24 | 37.205 | 6.633 | 68.813 | 1.00 23.00 | C |
| ATOM | 5048 | CG1 | VAL D | 24 | 36.509 | 6.009 | 67.604 | 1.00 22.62 | C |
| ATOM | 5049 | CG2 | VAL D | 24 | 38.231 | 7.701 | 68.343 | 1.00 20.91 | C |
| ATOM | 5050 | N | SER D | 25 | 34.247 | 5.764 | 69.829 | 1.00 26.13 | N |
| ATOM | 5051 | CA | SER D | 25 | 33.378 | 4.694 | 70.295 | 1.00 28.33 | C |
| ATOM | 5052 | C | SER D | 25 | 32.947 | 3.869 | 69.087 | 1.00 26.95 | C |
| ATOM | 5053 | O | SER D | 25 | 33.036 | 4.325 | 67.944 | 1.00 26.41 | O |
| ATOM | 5054 | CB | SER D | 25 | 32.164 | 5.299 | 71.036 | 1.00 29.72 | C |
| ATOM | 5055 | OG | SER D | 25 | 31.536 | 6.340 | 70.304 | 1.00 32.52 | O |
| ATOM | 5056 | N | GLY D | 26 | 32.494 | 2.642 | 69.326 | 1.00 25.94 | N |
| ATOM | 5057 | CA | GLY D | 26 | 32.029 | 1.801 | 68.246 | 1.00 23.40 | C |
| ATOM | 5058 | C | GLY D | 26 | 33.118 | 0.998 | 67.575 | 1.00 23.67 | C |
| ATOM | 5059 | O | GLY D | 26 | 32.859 | 0.342 | 66.580 | 1.00 23.54 | O |
| ATOM | 5060 | N | LEU D | 27 | 34.339 | 1.081 | 68.095 | 1.00 24.26 | N |

| ATOM | 5061 | CA | LEU | D | 27 | 35.432 | 0.247 | 67.676 | 1.00 | 24.92 | C |
| ATOM | 5062 | C | LEU | D | 27 | 36.405 | 0.049 | 68.853 | 1.00 | 24.51 | C |
| ATOM | 5063 | O | LEU | D | 27 | 36.276 | 0.693 | 69.897 | 1.00 | 26.64 | O |
| ATOM | 5064 | CB | LEU | D | 27 | 36.108 | 0.838 | 66.427 | 1.00 | 25.21 | C |
| ATOM | 5065 | CG | LEU | D | 27 | 36.598 | 2.306 | 66.466 | 1.00 | 25.77 | C |
| ATOM | 5066 | CD1 | LEU | D | 27 | 37.610 | 2.572 | 67.534 | 1.00 | 26.53 | C |
| ATOM | 5067 | CD2 | LEU | D | 27 | 37.187 | 2.688 | 65.092 | 1.00 | 25.67 | C |
| ATOM | 5068 | N | THR | D | 28 | 37.324 | -0.898 | 68.697 | 1.00 | 23.99 | N |
| ATOM | 5069 | CA | THR | D | 28 | 38.376 | -1.141 | 69.652 | 1.00 | 24.18 | C |
| ATOM | 5070 | C | THR | D | 28 | 39.475 | -0.116 | 69.368 | 1.00 | 24.47 | C |
| ATOM | 5071 | O | THR | D | 28 | 40.353 | -0.343 | 68.538 | 1.00 | 23.85 | O |
| ATOM | 5072 | CB | THR | D | 28 | 38.940 | -2.582 | 69.550 | 1.00 | 24.77 | C |
| ATOM | 5073 | OG1 | THR | D | 28 | 37.857 | -3.531 | 69.506 | 1.00 | 29.63 | O |
| ATOM | 5074 | CG2 | THR | D | 28 | 39.801 | -2.890 | 70.747 | 1.00 | 24.83 | C |
| ATOM | 5075 | N | PHE | D | 29 | 39.407 | 0.999 | 70.076 | 1.00 | 24.52 | N |
| ATOM | 5076 | CA | PHE | D | 29 | 40.363 | 2.098 | 69.950 | 1.00 | 25.68 | C |
| ATOM | 5077 | C | PHE | D | 29 | 41.823 | 1.636 | 69.810 | 1.00 | 26.16 | C |
| ATOM | 5078 | O | PHE | D | 29 | 42.519 | 2.033 | 68.861 | 1.00 | 26.12 | O |
| ATOM | 5079 | CB | PHE | D | 29 | 40.205 | 3.049 | 71.144 | 1.00 | 25.32 | C |
| ATOM | 5080 | CG | PHE | D | 29 | 41.078 | 4.286 | 71.061 | 1.00 | 24.95 | C |
| ATOM | 5081 | CD1 | PHE | D | 29 | 40.589 | 5.463 | 70.517 | 1.00 | 25.44 | C |
| ATOM | 5082 | CD2 | PHE | D | 29 | 42.372 | 4.271 | 71.528 | 1.00 | 24.79 | C |
| ATOM | 5083 | CE1 | PHE | D | 29 | 41.397 | 6.602 | 70.445 | 1.00 | 26.16 | C |
| ATOM | 5084 | CE2 | PHE | D | 29 | 43.176 | 5.408 | 71.443 | 1.00 | 24.74 | C |
| ATOM | 5085 | CZ | PHE | D | 29 | 42.681 | 6.564 | 70.908 | 1.00 | 23.13 | C |
| ATOM | 5086 | N | SER | D | 30 | 42.287 | 0.802 | 70.744 | 1.00 | 26.44 | N |
| ATOM | 5087 | CA | SER | D | 30 | 43.697 | 0.328 | 70.782 | 1.00 | 26.08 | C |
| ATOM | 5088 | C | SER | D | 30 | 44.236 | -0.372 | 69.512 | 1.00 | 25.03 | C |
| ATOM | 5089 | O | SER | D | 30 | 45.450 | -0.594 | 69.375 | 1.00 | 25.66 | O |
| ATOM | 5090 | CB | SER | D | 30 | 43.890 | -0.609 | 71.984 | 1.00 | 27.25 | C |
| ATOM | 5091 | OG | SER | D | 30 | 43.332 | -1.891 | 71.726 | 1.00 | 29.45 | O |
| ATOM | 5092 | N | ASN | D | 31 | 43.343 | -0.716 | 68.588 | 1.00 | 24.48 | N |
| ATOM | 5093 | CA | ASN | D | 31 | 43.688 | -1.449 | 67.368 | 1.00 | 23.76 | C |
| ATOM | 5094 | C | ASN | D | 31 | 43.896 | -0.544 | 66.127 | 1.00 | 21.75 | C |
| ATOM | 5095 | O | ASN | D | 31 | 44.201 | -1.051 | 65.070 | 1.00 | 20.12 | O |
| ATOM | 5096 | CB | ASN | D | 31 | 42.582 | -2.478 | 67.045 | 1.00 | 24.76 | C |
| ATOM | 5097 | CG | ASN | D | 31 | 42.601 | -3.718 | 67.973 | 1.00 | 28.84 | C |
| ATOM | 5098 | OD1 | ASN | D | 31 | 41.852 | -4.670 | 67.746 | 1.00 | 34.47 | O |
| ATOM | 5099 | ND2 | ASN | D | 31 | 43.429 | -3.710 | 68.985 | 1.00 | 28.06 | N |
| ATOM | 5100 | N | TYR | D | 32 | 43.662 | 0.759 | 66.248 | 1.00 | 20.88 | N |
| ATOM | 5101 | CA | TYR | D | 32 | 43.648 | 1.653 | 65.098 | 1.00 | 21.54 | C |
| ATOM | 5102 | C | TYR | D | 32 | 44.755 | 2.688 | 65.154 | 1.00 | 20.44 | C |
| ATOM | 5103 | O | TYR | D | 32 | 45.006 | 3.255 | 66.188 | 1.00 | 18.12 | O |
| ATOM | 5104 | CB | TYR | D | 32 | 42.322 | 2.393 | 65.016 | 1.00 | 23.59 | C |
| ATOM | 5105 | CG | TYR | D | 32 | 41.219 | 1.510 | 64.542 | 1.00 | 24.23 | C |
| ATOM | 5106 | CD1 | TYR | D | 32 | 40.575 | 0.648 | 65.430 | 1.00 | 24.83 | C |
| ATOM | 5107 | CD2 | TYR | D | 32 | 40.865 | 1.472 | 63.215 | 1.00 | 25.43 | C |
| ATOM | 5108 | CE1 | TYR | D | 32 | 39.599 | -0.185 | 65.011 | 1.00 | 25.27 | C |
| ATOM | 5109 | CE2 | TYR | D | 32 | 39.874 | 0.632 | 62.781 | 1.00 | 26.68 | C |
| ATOM | 5110 | CZ | TYR | D | 32 | 39.261 | -0.205 | 63.702 | 1.00 | 25.91 | C |
| ATOM | 5111 | OH | TYR | D | 32 | 38.281 | -1.044 | 63.295 | 1.00 | 28.60 | O |
| ATOM | 5112 | N | TRP | D | 33 | 45.379 | 2.957 | 64.013 | 1.00 | 19.94 | N |
| ATOM | 5113 | CA | TRP | D | 33 | 46.222 | 4.116 | 63.894 | 1.00 | 19.97 | C |
| ATOM | 5114 | C | TRP | D | 33 | 45.345 | 5.354 | 63.978 | 1.00 | 18.69 | C |
| ATOM | 5115 | O | TRP | D | 33 | 44.218 | 5.373 | 63.480 | 1.00 | 17.86 | O |
| ATOM | 5116 | CB | TRP | D | 33 | 46.970 | 4.136 | 62.551 | 1.00 | 20.92 | C |
| ATOM | 5117 | CG | TRP | D | 33 | 47.823 | 2.971 | 62.320 | 1.00 | 22.34 | C |
| ATOM | 5118 | CD1 | TRP | D | 33 | 48.166 | 1.997 | 63.227 | 1.00 | 24.72 | C |
| ATOM | 5119 | CD2 | TRP | D | 33 | 48.515 | 2.647 | 61.106 | 1.00 | 24.09 | C |
| ATOM | 5120 | NE1 | TRP | D | 33 | 49.008 | 1.076 | 62.634 | 1.00 | 24.56 | N |
| ATOM | 5121 | CE2 | TRP | D | 33 | 49.231 | 1.453 | 61.333 | 1.00 | 22.92 | C |
| ATOM | 5122 | CE3 | TRP | D | 33 | 48.586 | 3.249 | 59.838 | 1.00 | 24.63 | C |
| ATOM | 5123 | CZ2 | TRP | D | 33 | 50.007 | 0.853 | 60.351 | 1.00 | 23.83 | C |
| ATOM | 5124 | CZ3 | TRP | D | 33 | 49.356 | 2.640 | 58.860 | 1.00 | 24.52 | C |
| ATOM | 5125 | CH2 | TRP | D | 33 | 50.064 | 1.467 | 59.124 | 1.00 | 24.01 | C |

```
ATOM   5126  N    MET D  34     45.912    6.407   64.546   1.00  17.56        N
ATOM   5127  CA   MET D  34     45.204    7.648   64.794   1.00  19.31        C
ATOM   5128  C    MET D  34     46.042    8.809   64.246   1.00  16.84        C
ATOM   5129  O    MET D  34     47.250    8.848   64.476   1.00  16.94        O
ATOM   5130  CB   MET D  34     45.068    7.851   66.281   1.00  19.21        C
ATOM   5131  CG   MET D  34     44.329    6.715   67.027   1.00  24.24        C
ATOM   5132  SD   MET D  34     42.579    7.005   66.916   1.00  28.62        S
ATOM   5133  CE   MET D  34     41.941    5.332   67.082   1.00  27.96        C
ATOM   5134  N    SER D  35     45.370    9.772   63.627   1.00  17.96        N
ATOM   5135  CA   SER D  35     46.024   10.981   63.104   1.00  17.43        C
ATOM   5136  C    SER D  35     45.190   12.218   63.363   1.00  17.65        C
ATOM   5137  O    SER D  35     43.989   12.151   63.674   1.00  14.15        O
ATOM   5138  CB   SER D  35     46.317   10.836   61.603   1.00  16.87        C
ATOM   5139  OG   SER D  35     45.126   10.911   60.837   1.00  16.39        O
ATOM   5140  N    TRP D  36     45.843   13.371   63.217   1.00  15.47        N
ATOM   5141  CA   TRP D  36     45.171   14.643   63.218   1.00  13.94        C
ATOM   5142  C    TRP D  36     45.394   15.312   61.881   1.00  16.11        C
ATOM   5143  O    TRP D  36     46.555   15.333   61.382   1.00  13.14        O
ATOM   5144  CB   TRP D  36     45.742   15.538   64.307   1.00  16.62        C
ATOM   5145  CG   TRP D  36     45.349   15.160   65.689   1.00  15.55        C
ATOM   5146  CD1  TRP D  36     46.056   14.385   66.546   1.00  15.76        C
ATOM   5147  CD2  TRP D  36     44.162   15.534   66.361   1.00  16.55        C
ATOM   5148  NE1  TRP D  36     45.362   14.218   67.720   1.00  14.98        N
ATOM   5149  CE2  TRP D  36     44.209   14.939   67.652   1.00  15.57        C
ATOM   5150  CE3  TRP D  36     43.063   16.336   66.024   1.00  17.56        C
ATOM   5151  CZ2  TRP D  36     43.193   15.103   68.597   1.00  18.82        C
ATOM   5152  CZ3  TRP D  36     42.030   16.470   66.958   1.00  16.97        C
ATOM   5153  CH2  TRP D  36     42.122   15.880   68.238   1.00  17.53        C
ATOM   5154  N    VAL D  37     44.298   15.849   61.340   1.00  15.97        N
ATOM   5155  CA   VAL D  37     44.238   16.530   60.035   1.00  17.62        C
ATOM   5156  C    VAL D  37     43.529   17.829   60.284   1.00  18.27        C
ATOM   5157  O    VAL D  37     42.447   17.846   60.907   1.00  18.36        O
ATOM   5158  CB   VAL D  37     43.434   15.718   58.993   1.00  16.43        C
ATOM   5159  CG1  VAL D  37     43.476   16.403   57.631   1.00  19.72        C
ATOM   5160  CG2  VAL D  37     43.939   14.293   58.914   1.00  16.07        C
ATOM   5161  N    ARG D  38     44.128   18.935   59.847   1.00  17.01        N
ATOM   5162  CA   ARG D  38     43.521   20.240   60.030   1.00  17.51        C
ATOM   5163  C    ARG D  38     43.039   20.807   58.714   1.00  21.02        C
ATOM   5164  O    ARG D  38     43.568   20.465   57.638   1.00  22.10        O
ATOM   5165  CB   ARG D  38     44.451   21.214   60.735   1.00  16.98        C
ATOM   5166  CG   ARG D  38     45.737   21.542   59.989   1.00  17.71        C
ATOM   5167  CD   ARG D  38     46.643   22.326   60.869   1.00  19.59        C
ATOM   5168  NE   ARG D  38     47.849   22.735   60.149   1.00  20.56        N
ATOM   5169  CZ   ARG D  38     48.898   23.304   60.718   1.00  20.83        C
ATOM   5170  NH1  ARG D  38     48.911   23.537   62.017   1.00  20.42        N
ATOM   5171  NH2  ARG D  38     49.941   23.657   59.972   1.00  22.69        N
ATOM   5172  N    GLN D  39     42.059   21.698   58.806   1.00  22.45        N
ATOM   5173  CA   GLN D  39     41.450   22.313   57.642   1.00  25.03        C
ATOM   5174  C    GLN D  39     41.439   23.812   57.784   1.00  27.05        C
ATOM   5175  O    GLN D  39     40.902   24.333   58.742   1.00  24.23        O
ATOM   5176  CB   GLN D  39     40.023   21.815   57.524   1.00  24.98        C
ATOM   5177  CG   GLN D  39     39.258   22.306   56.303   1.00  26.07        C
ATOM   5178  CD   GLN D  39     37.938   21.586   56.157   1.00  27.91        C
ATOM   5179  OE1  GLN D  39     37.174   21.418   57.135   1.00  27.24        O
ATOM   5180  NE2  GLN D  39     37.660   21.134   54.940   1.00  30.06        N
ATOM   5181  N    SER D  40     42.029   24.515   56.824   1.00  32.18        N
ATOM   5182  CA   SER D  40     41.862   25.965   56.744   1.00  37.31        C
ATOM   5183  C    SER D  40     41.435   26.419   55.337   1.00  39.43        C
ATOM   5184  O    SER D  40     41.603   25.677   54.366   1.00  39.54        O
ATOM   5185  CB   SER D  40     43.158   26.643   57.129   1.00  37.16        C
ATOM   5186  OG   SER D  40     44.176   26.199   56.262   1.00  39.36        O
ATOM   5187  N    PRO D  41     40.872   27.639   55.224   1.00  42.56        N
ATOM   5188  CA   PRO D  41     40.678   28.273   53.909   1.00  44.10        C
ATOM   5189  C    PRO D  41     41.925   28.296   52.997   1.00  45.70        C
ATOM   5190  O    PRO D  41     41.768   28.224   51.779   1.00  46.76        O
```

| ATOM | 5191 | CB | PRO | D | 41 | 40.261 | 29.711 | 54.269 | 1.00 | 44.58 | C |
|------|------|-----|-----|---|----|--------|--------|--------|------|-------|---|
| ATOM | 5192 | CG | PRO | D | 41 | 39.607 | 29.591 | 55.607 | 1.00 | 44.18 | C |
| ATOM | 5193 | CD | PRO | D | 41 | 40.363 | 28.486 | 56.319 | 1.00 | 43.47 | C |
| ATOM | 5194 | N | GLU | D | 42 | 43.137 | 28.377 | 53.553 | 1.00 | 47.24 | N |
| ATOM | 5195 | CA | GLU | D | 42 | 44.343 | 28.535 | 52.696 | 1.00 | 48.43 | C |
| ATOM | 5196 | C | GLU | D | 42 | 45.218 | 27.300 | 52.418 | 1.00 | 49.49 | C |
| ATOM | 5197 | O | GLU | D | 42 | 46.099 | 27.381 | 51.541 | 1.00 | 51.10 | O |
| ATOM | 5198 | CB | GLU | D | 42 | 45.240 | 29.707 | 53.169 | 1.00 | 50.33 | C |
| ATOM | 5199 | CG | GLU | D | 42 | 46.182 | 29.435 | 54.361 | 1.00 | 52.11 | C |
| ATOM | 5200 | CD | GLU | D | 42 | 45.442 | 29.270 | 55.682 | 1.00 | 53.70 | C |
| ATOM | 5201 | OE1 | GLU | D | 42 | 44.244 | 29.651 | 55.738 | 1.00 | 54.63 | O |
| ATOM | 5202 | OE2 | GLU | D | 42 | 46.063 | 28.769 | 56.655 | 1.00 | 53.43 | O |
| ATOM | 5203 | N | LYS | D | 43 | 45.020 | 26.184 | 53.127 | 1.00 | 48.44 | N |
| ATOM | 5204 | CA | LYS | D | 43 | 45.793 | 24.948 | 52.827 | 1.00 | 47.10 | C |
| ATOM | 5205 | C | LYS | D | 43 | 44.933 | 23.692 | 52.619 | 1.00 | 45.03 | C |
| ATOM | 5206 | O | LYS | D | 43 | 45.473 | 22.582 | 52.581 | 1.00 | 45.65 | O |
| ATOM | 5207 | CB | LYS | D | 43 | 46.838 | 24.647 | 53.921 | 1.00 | 48.29 | C |
| ATOM | 5208 | CG | LYS | D | 43 | 47.862 | 25.743 | 54.199 | 1.00 | 50.55 | C |
| ATOM | 5209 | CD | LYS | D | 43 | 48.917 | 25.856 | 53.104 | 1.00 | 51.78 | C |
| ATOM | 5210 | CE | LYS | D | 43 | 50.016 | 24.804 | 53.221 | 1.00 | 52.11 | C |
| ATOM | 5211 | NZ | LYS | D | 43 | 51.053 | 25.038 | 52.157 | 1.00 | 52.72 | N |
| ATOM | 5212 | N | GLY | D | 44 | 43.620 | 23.851 | 52.457 | 1.00 | 42.29 | N |
| ATOM | 5213 | CA | GLY | D | 44 | 42.694 | 22.698 | 52.475 | 1.00 | 41.08 | C |
| ATOM | 5214 | C | GLY | D | 44 | 42.969 | 21.783 | 53.673 | 1.00 | 37.01 | C |
| ATOM | 5215 | O | GLY | D | 44 | 43.235 | 22.281 | 54.779 | 1.00 | 37.09 | O |
| ATOM | 5216 | N | LEU | D | 45 | 42.951 | 20.466 | 53.441 | 1.00 | 31.79 | N |
| ATOM | 5217 | CA | LEU | D | 45 | 43.297 | 19.477 | 54.458 | 1.00 | 28.74 | C |
| ATOM | 5218 | C | LEU | D | 45 | 44.779 | 19.183 | 54.529 | 1.00 | 27.36 | C |
| ATOM | 5219 | O | LEU | D | 45 | 45.378 | 18.778 | 53.547 | 1.00 | 25.51 | O |
| ATOM | 5220 | CB | LEU | D | 45 | 42.607 | 18.148 | 54.187 | 1.00 | 28.14 | C |
| ATOM | 5221 | CG | LEU | D | 45 | 41.102 | 18.138 | 54.297 | 1.00 | 27.30 | C |
| ATOM | 5222 | CD1 | LEU | D | 45 | 40.590 | 16.799 | 53.775 | 1.00 | 25.83 | C |
| ATOM | 5223 | CD2 | LEU | D | 45 | 40.704 | 18.393 | 55.753 | 1.00 | 27.04 | C |
| ATOM | 5224 | N | GLU | D | 46 | 45.328 | 19.305 | 55.728 | 1.00 | 25.61 | N |
| ATOM | 5225 | CA | GLU | D | 46 | 46.730 | 19.041 | 56.005 | 1.00 | 25.40 | C |
| ATOM | 5226 | C | GLU | D | 46 | 46.863 | 18.068 | 57.163 | 1.00 | 22.61 | C |
| ATOM | 5227 | O | GLU | D | 46 | 46.455 | 18.375 | 58.278 | 1.00 | 18.52 | O |
| ATOM | 5228 | CB | GLU | D | 46 | 47.426 | 20.340 | 56.374 | 1.00 | 25.47 | C |
| ATOM | 5229 | CG | GLU | D | 46 | 48.923 | 20.221 | 56.364 | 1.00 | 29.12 | C |
| ATOM | 5230 | CD | GLU | D | 46 | 49.631 | 21.467 | 56.908 | 1.00 | 30.28 | C |
| ATOM | 5231 | OE1 | GLU | D | 46 | 48.956 | 22.461 | 57.294 | 1.00 | 32.47 | O |
| ATOM | 5232 | OE2 | GLU | D | 46 | 50.882 | 21.424 | 56.961 | 1.00 | 38.43 | O |
| ATOM | 5233 | N | TRP | D | 47 | 47.410 | 16.888 | 56.870 | 1.00 | 20.63 | N |
| ATOM | 5234 | CA | TRP | D | 47 | 47.792 | 15.922 | 57.877 | 1.00 | 20.02 | C |
| ATOM | 5235 | C | TRP | D | 47 | 48.879 | 16.522 | 58.744 | 1.00 | 20.72 | C |
| ATOM | 5236 | O | TRP | D | 47 | 49.846 | 17.109 | 58.223 | 1.00 | 17.60 | O |
| ATOM | 5237 | CB | TRP | D | 47 | 48.304 | 14.687 | 57.182 | 1.00 | 19.58 | C |
| ATOM | 5238 | CG | TRP | D | 47 | 48.900 | 13.626 | 58.074 | 1.00 | 19.62 | C |
| ATOM | 5239 | CD1 | TRP | D | 47 | 48.227 | 12.692 | 58.806 | 1.00 | 19.40 | C |
| ATOM | 5240 | CD2 | TRP | D | 47 | 50.282 | 13.381 | 58.282 | 1.00 | 18.42 | C |
| ATOM | 5241 | NE1 | TRP | D | 47 | 49.112 | 11.867 | 59.442 | 1.00 | 19.14 | N |
| ATOM | 5242 | CE2 | TRP | D | 47 | 50.385 | 12.283 | 59.154 | 1.00 | 18.87 | C |
| ATOM | 5243 | CE3 | TRP | D | 47 | 51.461 | 13.989 | 57.817 | 1.00 | 19.52 | C |
| ATOM | 5244 | CZ2 | TRP | D | 47 | 51.598 | 11.786 | 59.570 | 1.00 | 19.06 | C |
| ATOM | 5245 | CZ3 | TRP | D | 47 | 52.669 | 13.483 | 58.240 | 1.00 | 18.62 | C |
| ATOM | 5246 | CH2 | TRP | D | 47 | 52.732 | 12.387 | 59.079 | 1.00 | 19.96 | C |
| ATOM | 5247 | N | VAL | D | 48 | 48.754 | 16.411 | 60.065 | 1.00 | 18.94 | N |
| ATOM | 5248 | CA | VAL | D | 48 | 49.839 | 16.965 | 60.904 | 1.00 | 20.33 | C |
| ATOM | 5249 | C | VAL | D | 48 | 50.539 | 15.987 | 61.802 | 1.00 | 19.73 | C |
| ATOM | 5250 | O | VAL | D | 48 | 51.700 | 16.178 | 62.129 | 1.00 | 16.78 | O |
| ATOM | 5251 | CB | VAL | D | 48 | 49.442 | 18.222 | 61.702 | 1.00 | 21.65 | C |
| ATOM | 5252 | CG1 | VAL | D | 48 | 49.046 | 19.329 | 60.756 | 1.00 | 22.86 | C |
| ATOM | 5253 | CG2 | VAL | D | 48 | 48.365 | 17.951 | 62.721 | 1.00 | 21.13 | C |
| ATOM | 5254 | N | ALA | D | 49 | 49.858 | 14.930 | 62.228 | 1.00 | 16.92 | N |
| ATOM | 5255 | CA | ALA | D | 49 | 50.482 | 14.076 | 63.163 | 1.00 | 16.29 | C |

```
ATOM   5256  C    ALA D  49      49.767  12.760  63.261  1.00  17.56           C
ATOM   5257  O    ALA D  49      48.570  12.693  62.986  1.00  14.88           O
ATOM   5258  CB   ALA D  49      50.580  14.779  64.508  1.00  15.62           C
ATOM   5259  N    GLU D  50      50.523  11.720  63.620  1.00  17.39           N
ATOM   5260  CA   GLU D  50      49.991  10.359  63.610  1.00  19.16           C
ATOM   5261  C    GLU D  50      50.660   9.555  64.665  1.00  19.30           C
ATOM   5262  O    GLU D  50      51.855   9.756  64.949  1.00  20.70           O
ATOM   5263  CB   GLU D  50      50.185   9.749  62.233  1.00  19.85           C
ATOM   5264  CG   GLU D  50      49.720   8.343  62.069  1.00  21.57           C
ATOM   5265  CD   GLU D  50      49.427   8.026  60.628  1.00  24.19           C
ATOM   5266  OE1  GLU D  50      48.893   8.893  59.914  1.00  26.59           O
ATOM   5267  OE2  GLU D  50      49.700   6.907  60.201  1.00  25.76           O
ATOM   5268  N    ILE D  51      49.888   8.664  65.285  1.00  19.19           N
ATOM   5269  CA   ILE D  51      50.416   7.769  66.283  1.00  19.04           C
ATOM   5270  C    ILE D  51      49.990   6.308  65.997  1.00  20.01           C
ATOM   5271  O    ILE D  51      48.841   6.049  65.603  1.00  20.53           O
ATOM   5272  CB   ILE D  51      50.060   8.246  67.716  1.00  20.03           C
ATOM   5273  CG1  ILE D  51      50.813   7.413  68.748  1.00  19.80           C
ATOM   5274  CG2  ILE D  51      48.544   8.222  67.966  1.00  19.75           C
ATOM   5275  CD1  ILE D  51      50.727   7.935  70.115  1.00  20.43           C
ATOM   5276  N    ARG D  52      50.898   5.365  66.209  1.00  18.24           N
ATOM   5277  CA   ARG D  52      50.592   3.977  65.912  1.00  20.46           C
ATOM   5278  C    ARG D  52      50.226   3.223  67.211  1.00  19.00           C
ATOM   5279  O    ARG D  52      49.870   3.862  68.191  1.00  17.35           O
ATOM   5280  CB   ARG D  52      51.731   3.336  65.122  1.00  21.83           C
ATOM   5281  CG   ARG D  52      52.108   4.127  63.860  1.00  23.57           C
ATOM   5282  CD   ARG D  52      51.055   3.961  62.803  1.00  27.24           C
ATOM   5283  NE   ARG D  52      51.242   4.786  61.604  1.00  28.78           N
ATOM   5284  CZ   ARG D  52      52.075   4.509  60.605  1.00  28.24           C
ATOM   5285  NH1  ARG D  52      52.867   3.451  60.635  1.00  28.80           N
ATOM   5286  NH2  ARG D  52      52.123   5.311  59.564  1.00  29.84           N
ATOM   5287  N    LEU D  52A     50.296   1.899  67.190  1.00  20.58           N
ATOM   5288  CA   LEU D  52A     49.777   1.054  68.282  1.00  21.61           C
ATOM   5289  C    LEU D  52A     50.807   0.821  69.367  1.00  23.68           C
ATOM   5290  O    LEU D  52A     51.992   1.048  69.163  1.00  23.77           O
ATOM   5291  CB   LEU D  52A     49.338  -0.298  67.705  1.00  21.10           C
ATOM   5292  CG   LEU D  52A     48.443  -0.234  66.468  1.00  22.01           C
ATOM   5293  CD1  LEU D  52A     47.851  -1.601  66.153  1.00  24.07           C
ATOM   5294  CD2  LEU D  52A     47.356   0.763  66.689  1.00  21.23           C
ATOM   5295  N    LYS D  52B     50.352   0.321  70.518  1.00  25.65           N
ATOM   5296  CA   LYS D  52B     51.257  -0.136  71.577  1.00  26.20           C
ATOM   5297  C    LYS D  52B     52.296  -1.106  71.051  1.00  25.83           C
ATOM   5298  O    LYS D  52B     53.462  -1.024  71.423  1.00  26.45           O
ATOM   5299  CB   LYS D  52B     50.456  -0.819  72.706  1.00  27.93           C
ATOM   5300  CG   LYS D  52B     51.285  -1.352  73.864  1.00  28.17           C
ATOM   5301  CD   LYS D  52B     50.342  -1.870  74.972  1.00  28.93           C
ATOM   5302  CE   LYS D  52B     51.042  -2.047  76.305  1.00  31.41           C
ATOM   5303  NZ   LYS D  52B     50.034  -2.147  77.475  1.00  31.88           N
ATOM   5304  N    SER D  52C     51.871  -2.032  70.199  1.00  25.00           N
ATOM   5305  CA   SER D  52C     52.776  -3.007  69.595  1.00  25.03           C
ATOM   5306  C    SER D  52C     53.773  -2.342  68.641  1.00  24.60           C
ATOM   5307  O    SER D  52C     54.771  -2.964  68.232  1.00  24.44           O
ATOM   5308  CB   SER D  52C     51.984  -4.077  68.856  1.00  26.00           C
ATOM   5309  OG   SER D  52C     51.112  -3.522  67.897  1.00  25.60           O
ATOM   5310  N    ASP D  53      53.521  -1.071  68.302  1.00  24.11           N
ATOM   5311  CA   ASP D  53      54.470  -0.290  67.501  1.00  23.60           C
ATOM   5312  C    ASP D  53      55.292   0.651  68.381  1.00  23.97           C
ATOM   5313  O    ASP D  53      55.961   1.562  67.873  1.00  22.94           O
ATOM   5314  CB   ASP D  53      53.743   0.512  66.435  1.00  24.64           C
ATOM   5315  CG   ASP D  53      52.832  -0.343  65.557  1.00  26.14           C
ATOM   5316  OD1  ASP D  53      53.277  -1.396  65.063  1.00  27.56           O
ATOM   5317  OD2  ASP D  53      51.672   0.059  65.324  1.00  26.01           O
ATOM   5318  N    ASN D  54      55.231   0.424  69.700  1.00  22.32           N
ATOM   5319  CA   ASN D  54      55.838   1.307  70.694  1.00  22.72           C
ATOM   5320  C    ASN D  54      55.326   2.723  70.584  1.00  22.31           C
```

| | | | | | | | | | | | |
|---|---|---|---|---|---|---|---|---|---|---|---|
| ATOM | 5321 | O | ASN | D | 54 | 56.102 | 3.658 | 70.772 | 1.00 | 21.15 | O |
| ATOM | 5322 | CB | ASN | D | 54 | 57.374 | 1.272 | 70.576 | 1.00 | 22.40 | C |
| ATOM | 5323 | CG | ASN | D | 54 | 57.906 | -0.132 | 70.664 | 1.00 | 24.56 | C |
| ATOM | 5324 | OD1 | ASN | D | 54 | 57.813 | -0.763 | 71.700 | 1.00 | 25.39 | O |
| ATOM | 5325 | ND2 | ASN | D | 54 | 58.435 | -0.631 | 69.584 | 1.00 | 26.01 | N |
| ATOM | 5326 | N | TYR | D | 55 | 54.032 | 2.855 | 70.245 | 1.00 | 22.47 | N |
| ATOM | 5327 | CA | TYR | D | 55 | 53.355 | 4.119 | 70.076 | 1.00 | 22.39 | C |
| ATOM | 5328 | C | TYR | D | 55 | 54.148 | 5.075 | 69.170 | 1.00 | 22.52 | C |
| ATOM | 5329 | O | TYR | D | 55 | 54.278 | 6.264 | 69.465 | 1.00 | 20.72 | O |
| ATOM | 5330 | CB | TYR | D | 55 | 53.157 | 4.752 | 71.431 | 1.00 | 25.28 | C |
| ATOM | 5331 | CG | TYR | D | 55 | 52.186 | 3.984 | 72.317 | 1.00 | 26.80 | C |
| ATOM | 5332 | CD1 | TYR | D | 55 | 50.880 | 3.767 | 71.908 | 1.00 | 26.95 | C |
| ATOM | 5333 | CD2 | TYR | D | 55 | 52.580 | 3.489 | 73.548 | 1.00 | 29.35 | C |
| ATOM | 5334 | CE1 | TYR | D | 55 | 49.961 | 3.092 | 72.714 | 1.00 | 27.08 | C |
| ATOM | 5335 | CE2 | TYR | D | 55 | 51.660 | 2.789 | 74.381 | 1.00 | 28.07 | C |
| ATOM | 5336 | CZ | TYR | D | 55 | 50.365 | 2.613 | 73.945 | 1.00 | 28.03 | C |
| ATOM | 5337 | OH | TYR | D | 55 | 49.467 | 1.929 | 74.742 | 1.00 | 30.38 | O |
| ATOM | 5338 | N | ALA | D | 56 | 54.678 | 4.542 | 68.087 | 1.00 | 21.69 | N |
| ATOM | 5339 | CA | ALA | D | 56 | 55.447 | 5.356 | 67.124 | 1.00 | 21.70 | C |
| ATOM | 5340 | C | ALA | D | 56 | 54.630 | 6.537 | 66.583 | 1.00 | 21.50 | C |
| ATOM | 5341 | O | ALA | D | 56 | 53.463 | 6.400 | 66.193 | 1.00 | 19.45 | O |
| ATOM | 5342 | CB | ALA | D | 56 | 55.940 | 4.476 | 65.985 | 1.00 | 21.94 | C |
| ATOM | 5343 | N | THR | D | 57 | 55.286 | 7.696 | 66.545 | 1.00 | 22.32 | N |
| ATOM | 5344 | CA | THR | D | 57 | 54.708 | 8.954 | 66.118 | 1.00 | 20.09 | C |
| ATOM | 5345 | C | THR | D | 57 | 55.386 | 9.495 | 64.862 | 1.00 | 21.92 | C |
| ATOM | 5346 | O | THR | D | 57 | 56.572 | 9.241 | 64.630 | 1.00 | 19.99 | O |
| ATOM | 5347 | CB | THR | D | 57 | 54.818 | 10.030 | 67.216 | 1.00 | 20.64 | C |
| ATOM | 5348 | OG1 | THR | D | 57 | 56.188 | 10.220 | 67.620 | 1.00 | 19.54 | O |
| ATOM | 5349 | CG2 | THR | D | 57 | 53.994 | 9.637 | 68.441 | 1.00 | 17.46 | C |
| ATOM | 5350 | N | TYR | D | 58 | 54.591 | 10.199 | 64.063 | 1.00 | 22.27 | N |
| ATOM | 5351 | CA | TYR | D | 58 | 55.016 | 10.890 | 62.840 | 1.00 | 23.73 | C |
| ATOM | 5352 | C | TYR | D | 58 | 54.338 | 12.241 | 62.820 | 1.00 | 23.26 | C |
| ATOM | 5353 | O | TYR | D | 58 | 53.176 | 12.380 | 63.240 | 1.00 | 19.60 | O |
| ATOM | 5354 | CB | TYR | D | 58 | 54.591 | 10.087 | 61.615 | 1.00 | 25.60 | C |
| ATOM | 5355 | CG | TYR | D | 58 | 55.067 | 8.680 | 61.709 | 1.00 | 26.44 | C |
| ATOM | 5356 | CD1 | TYR | D | 58 | 54.308 | 7.725 | 62.355 | 1.00 | 27.22 | C |
| ATOM | 5357 | CD2 | TYR | D | 58 | 56.310 | 8.315 | 61.195 | 1.00 | 27.94 | C |
| ATOM | 5358 | CE1 | TYR | D | 58 | 54.746 | 6.440 | 62.476 | 1.00 | 28.22 | C |
| ATOM | 5359 | CE2 | TYR | D | 58 | 56.769 | 7.015 | 61.319 | 1.00 | 28.86 | C |
| ATOM | 5360 | CZ | TYR | D | 58 | 55.969 | 6.087 | 61.970 | 1.00 | 28.05 | C |
| ATOM | 5361 | OH | TYR | D | 58 | 56.393 | 4.805 | 62.122 | 1.00 | 28.16 | O |
| ATOM | 5362 | N | TYR | D | 59 | 55.068 | 13.229 | 62.302 | 1.00 | 21.58 | N |
| ATOM | 5363 | CA | TYR | D | 59 | 54.636 | 14.578 | 62.241 | 1.00 | 21.40 | C |
| ATOM | 5364 | C | TYR | D | 59 | 54.917 | 15.175 | 60.886 | 1.00 | 23.60 | C |
| ATOM | 5365 | O | TYR | D | 59 | 55.914 | 14.813 | 60.229 | 1.00 | 22.58 | O |
| ATOM | 5366 | CB | TYR | D | 59 | 55.387 | 15.406 | 63.268 | 1.00 | 22.69 | C |
| ATOM | 5367 | CG | TYR | D | 59 | 55.079 | 15.004 | 64.672 | 1.00 | 23.19 | C |
| ATOM | 5368 | CD1 | TYR | D | 59 | 53.888 | 15.404 | 65.287 | 1.00 | 20.63 | C |
| ATOM | 5369 | CD2 | TYR | D | 59 | 55.960 | 14.194 | 65.385 | 1.00 | 21.91 | C |
| ATOM | 5370 | CE1 | TYR | D | 59 | 53.598 | 15.019 | 66.585 | 1.00 | 22.58 | C |
| ATOM | 5371 | CE2 | TYR | D | 59 | 55.682 | 13.814 | 66.660 | 1.00 | 24.63 | C |
| ATOM | 5372 | CZ | TYR | D | 59 | 54.500 | 14.219 | 67.268 | 1.00 | 23.33 | C |
| ATOM | 5373 | OH | TYR | D | 59 | 54.276 | 13.804 | 68.552 | 1.00 | 22.80 | O |
| ATOM | 5374 | N | ALA | D | 60 | 54.062 | 16.112 | 60.499 | 1.00 | 23.10 | N |
| ATOM | 5375 | CA | ALA | D | 60 | 54.332 | 16.964 | 59.360 | 1.00 | 25.51 | C |
| ATOM | 5376 | C | ALA | D | 60 | 55.571 | 17.845 | 59.608 | 1.00 | 26.05 | C |
| ATOM | 5377 | O | ALA | D | 60 | 55.755 | 18.416 | 60.678 | 1.00 | 21.28 | O |
| ATOM | 5378 | CB | ALA | D | 60 | 53.142 | 17.812 | 59.072 | 1.00 | 25.45 | C |
| ATOM | 5379 | N | GLU | D | 61 | 56.415 | 17.990 | 58.588 | 1.00 | 29.38 | N |
| ATOM | 5380 | CA | GLU | D | 61 | 57.562 | 18.888 | 58.733 | 1.00 | 32.18 | C |
| ATOM | 5381 | C | GLU | D | 61 | 57.216 | 20.266 | 59.328 | 1.00 | 31.54 | C |
| ATOM | 5382 | O | GLU | D | 61 | 57.922 | 20.756 | 60.189 | 1.00 | 31.67 | O |
| ATOM | 5383 | CB | GLU | D | 61 | 58.288 | 19.019 | 57.391 | 1.00 | 34.43 | C |
| ATOM | 5384 | CG | GLU | D | 61 | 59.087 | 17.770 | 57.036 | 1.00 | 38.23 | C |
| ATOM | 5385 | CD | GLU | D | 61 | 59.957 | 17.248 | 58.188 | 1.00 | 41.92 | C |

| ATOM | 5386 | OE1 | GLU | D | 61 | 60.780 | 18.032 | 58.734 | 1.00 | 43.59 | O |
|------|------|-----|-----|---|----|--------|--------|--------|------|-------|---|
| ATOM | 5387 | OE2 | GLU | D | 61 | 59.803 | 16.048 | 58.546 | 1.00 | 44.40 | O |
| ATOM | 5388 | N   | SER | D | 62 | 56.102 | 20.849 | 58.902 | 1.00 | 32.81 | N |
| ATOM | 5389 | CA  | SER | D | 62 | 55.624 | 22.144 | 59.392 | 1.00 | 33.53 | C |
| ATOM | 5390 | C   | SER | D | 62 | 55.327 | 22.249 | 60.888 | 1.00 | 33.81 | C |
| ATOM | 5391 | O   | SER | D | 62 | 55.257 | 23.354 | 61.425 | 1.00 | 33.89 | O |
| ATOM | 5392 | CB  | SER | D | 62 | 54.337 | 22.512 | 58.654 | 1.00 | 35.12 | C |
| ATOM | 5393 | OG  | SER | D | 62 | 53.377 | 21.452 | 58.707 | 1.00 | 37.93 | O |
| ATOM | 5394 | N   | VAL | D | 63 | 55.114 | 21.125 | 61.558 | 1.00 | 32.54 | N |
| ATOM | 5395 | CA  | VAL | D | 63 | 54.761 | 21.166 | 62.982 | 1.00 | 32.45 | C |
| ATOM | 5396 | C   | VAL | D | 63 | 55.719 | 20.398 | 63.885 | 1.00 | 33.30 | C |
| ATOM | 5397 | O   | VAL | D | 63 | 55.690 | 20.581 | 65.092 | 1.00 | 33.15 | O |
| ATOM | 5398 | CB  | VAL | D | 63 | 53.310 | 20.701 | 63.231 | 1.00 | 32.03 | C |
| ATOM | 5399 | CG1 | VAL | D | 63 | 52.360 | 21.543 | 62.413 | 1.00 | 30.90 | C |
| ATOM | 5400 | CG2 | VAL | D | 63 | 53.145 | 19.232 | 62.951 | 1.00 | 31.99 | C |
| ATOM | 5401 | N   | LYS | D | 64 | 56.575 | 19.556 | 63.299 | 1.00 | 35.84 | N |
| ATOM | 5402 | CA  | LYS | D | 64 | 57.501 | 18.732 | 64.067 | 1.00 | 35.89 | C |
| ATOM | 5403 | C   | LYS | D | 64 | 58.144 | 19.618 | 65.123 | 1.00 | 34.48 | C |
| ATOM | 5404 | O   | LYS | D | 64 | 58.574 | 20.748 | 64.848 | 1.00 | 34.03 | O |
| ATOM | 5405 | CB  | LYS | D | 64 | 58.565 | 18.084 | 63.151 | 1.00 | 37.03 | C |
| ATOM | 5406 | CG  | LYS | D | 64 | 59.490 | 17.073 | 63.860 | 1.00 | 38.12 | C |
| ATOM | 5407 | CD  | LYS | D | 64 | 60.478 | 16.396 | 62.883 | 1.00 | 39.16 | C |
| ATOM | 5408 | CE  | LYS | D | 64 | 61.798 | 15.951 | 63.559 | 1.00 | 40.56 | C |
| ATOM | 5409 | NZ  | LYS | D | 64 | 62.905 | 15.588 | 62.532 | 1.00 | 41.25 | N |
| ATOM | 5410 | N   | GLY | D | 65 | 58.146 | 19.139 | 66.359 | 1.00 | 32.66 | N |
| ATOM | 5411 | CA  | GLY | D | 65 | 58.803 | 19.879 | 67.425 | 1.00 | 31.41 | C |
| ATOM | 5412 | C   | GLY | D | 65 | 58.007 | 21.029 | 68.017 | 1.00 | 30.88 | C |
| ATOM | 5413 | O   | GLY | D | 65 | 58.496 | 21.654 | 68.952 | 1.00 | 30.91 | O |
| ATOM | 5414 | N   | LYS | D | 66 | 56.827 | 21.344 | 67.457 | 1.00 | 28.15 | N |
| ATOM | 5415 | CA  | LYS | D | 66 | 55.821 | 22.196 | 68.127 | 1.00 | 27.29 | C |
| ATOM | 5416 | C   | LYS | D | 66 | 54.692 | 21.346 | 68.732 | 1.00 | 24.86 | C |
| ATOM | 5417 | O   | LYS | D | 66 | 54.077 | 21.723 | 69.724 | 1.00 | 24.12 | O |
| ATOM | 5418 | CB  | LYS | D | 66 | 55.096 | 23.079 | 67.127 | 1.00 | 27.12 | C |
| ATOM | 5419 | CG  | LYS | D | 66 | 55.919 | 24.089 | 66.422 | 1.00 | 28.80 | C |
| ATOM | 5420 | CD  | LYS | D | 66 | 55.156 | 25.379 | 66.283 | 1.00 | 27.26 | C |
| ATOM | 5421 | CE  | LYS | D | 66 | 54.009 | 25.318 | 65.328 | 1.00 | 26.32 | C |
| ATOM | 5422 | NZ  | LYS | D | 66 | 53.571 | 26.757 | 65.150 | 1.00 | 23.64 | N |
| ATOM | 5423 | N   | PHE | D | 67 | 54.368 | 20.255 | 68.046 | 1.00 | 23.17 | N |
| ATOM | 5424 | CA  | PHE | D | 67 | 53.145 | 19.458 | 68.328 | 1.00 | 21.41 | C |
| ATOM | 5425 | C   | PHE | D | 67 | 53.550 | 18.102 | 68.861 | 1.00 | 21.76 | C |
| ATOM | 5426 | O   | PHE | D | 67 | 54.559 | 17.553 | 68.432 | 1.00 | 20.97 | O |
| ATOM | 5427 | CB  | PHE | D | 67 | 52.365 | 19.235 | 67.023 | 1.00 | 19.13 | C |
| ATOM | 5428 | CG  | PHE | D | 67 | 51.527 | 20.401 | 66.574 | 1.00 | 16.96 | C |
| ATOM | 5429 | CD1 | PHE | D | 67 | 51.644 | 21.645 | 67.130 | 1.00 | 17.59 | C |
| ATOM | 5430 | CD2 | PHE | D | 67 | 50.649 | 20.238 | 65.535 | 1.00 | 18.28 | C |
| ATOM | 5431 | CE1 | PHE | D | 67 | 50.904 | 22.698 | 66.665 | 1.00 | 17.47 | C |
| ATOM | 5432 | CE2 | PHE | D | 67 | 49.877 | 21.271 | 65.095 | 1.00 | 18.31 | C |
| ATOM | 5433 | CZ  | PHE | D | 67 | 50.025 | 22.512 | 65.662 | 1.00 | 19.10 | C |
| ATOM | 5434 | N   | THR | D | 68 | 52.765 | 17.545 | 69.788 | 1.00 | 21.06 | N |
| ATOM | 5435 | CA  | THR | D | 68 | 53.024 | 16.226 | 70.313 | 1.00 | 22.56 | C |
| ATOM | 5436 | C   | THR | D | 68 | 51.706 | 15.474 | 70.345 | 1.00 | 21.96 | C |
| ATOM | 5437 | O   | THR | D | 68 | 50.766 | 15.930 | 70.978 | 1.00 | 20.69 | O |
| ATOM | 5438 | CB  | THR | D | 68 | 53.562 | 16.257 | 71.775 | 1.00 | 23.97 | C |
| ATOM | 5439 | OG1 | THR | D | 68 | 54.719 | 17.088 | 71.842 | 1.00 | 28.39 | O |
| ATOM | 5440 | CG2 | THR | D | 68 | 53.920 | 14.850 | 72.270 | 1.00 | 23.70 | C |
| ATOM | 5441 | N   | ILE | D | 69 | 51.682 | 14.321 | 69.696 | 1.00 | 21.85 | N |
| ATOM | 5442 | CA  | ILE | D | 69 | 50.507 | 13.472 | 69.669 | 1.00 | 21.82 | C |
| ATOM | 5443 | C   | ILE | D | 69 | 50.688 | 12.347 | 70.677 | 1.00 | 21.21 | C |
| ATOM | 5444 | O   | ILE | D | 69 | 51.785 | 11.860 | 70.864 | 1.00 | 20.76 | O |
| ATOM | 5445 | CB  | ILE | D | 69 | 50.205 | 12.963 | 68.219 | 1.00 | 21.70 | C |
| ATOM | 5446 | CG1 | ILE | D | 69 | 48.847 | 12.242 | 68.165 | 1.00 | 21.67 | C |
| ATOM | 5447 | CG2 | ILE | D | 69 | 51.347 | 12.102 | 67.652 | 1.00 | 21.73 | C |
| ATOM | 5448 | CD1 | ILE | D | 69 | 48.508 | 11.584 | 66.845 | 1.00 | 22.37 | C |
| ATOM | 5449 | N   | SER | D | 70 | 49.585 | 11.980 | 71.348 | 1.00 | 20.29 | N |
| ATOM | 5450 | CA  | SER | D | 70 | 49.549 | 10.903 | 72.307 | 1.00 | 19.62 | C |

| ATOM | 5451 | C | SER | D | 70 | 48.147 | 10.261 | 72.333 | 1.00 | 19.55 | C |
|------|------|-----|-----|---|----|--------|--------|--------|------|-------|---|
| ATOM | 5452 | O | SER | D | 70 | 47.192 | 10.785 | 71.781 | 1.00 | 18.33 | O |
| ATOM | 5453 | CB | SER | D | 70 | 49.904 | 11.388 | 73.723 | 1.00 | 20.19 | C |
| ATOM | 5454 | OG | SER | D | 70 | 49.090 | 12.466 | 74.117 | 1.00 | 21.09 | O |
| ATOM | 5455 | N | ARG | D | 71 | 48.077 | 9.114 | 72.972 | 1.00 | 20.99 | N |
| ATOM | 5456 | CA | ARG | D | 71 | 46.862 | 8.324 | 73.044 | 1.00 | 21.45 | C |
| ATOM | 5457 | C | ARG | D | 71 | 46.797 | 7.665 | 74.404 | 1.00 | 22.89 | C |
| ATOM | 5458 | O | ARG | D | 71 | 47.829 | 7.359 | 75.009 | 1.00 | 23.46 | O |
| ATOM | 5459 | CB | ARG | D | 71 | 46.808 | 7.285 | 71.926 | 1.00 | 20.11 | C |
| ATOM | 5460 | CG | ARG | D | 71 | 47.690 | 6.099 | 72.091 | 1.00 | 20.48 | C |
| ATOM | 5461 | CD | ARG | D | 71 | 47.842 | 5.346 | 70.809 | 1.00 | 22.49 | C |
| ATOM | 5462 | NE | ARG | D | 71 | 46.667 | 4.556 | 70.472 | 1.00 | 22.99 | N |
| ATOM | 5463 | CZ | ARG | D | 71 | 46.258 | 4.247 | 69.234 | 1.00 | 25.21 | C |
| ATOM | 5464 | NH1 | ARG | D | 71 | 46.913 | 4.655 | 68.132 | 1.00 | 23.87 | N |
| ATOM | 5465 | NH2 | ARG | D | 71 | 45.152 | 3.527 | 69.095 | 1.00 | 22.44 | N |
| ATOM | 5466 | N | ASP | D | 72 | 45.570 | 7.494 | 74.875 | 1.00 | 25.62 | N |
| ATOM | 5467 | CA | ASP | D | 72 | 45.275 | 6.747 | 76.109 | 1.00 | 26.08 | C |
| ATOM | 5468 | C | ASP | D | 72 | 44.271 | 5.680 | 75.714 | 1.00 | 26.27 | C |
| ATOM | 5469 | O | ASP | D | 72 | 43.082 | 5.963 | 75.585 | 1.00 | 24.34 | O |
| ATOM | 5470 | CB | ASP | D | 72 | 44.721 | 7.693 | 77.169 | 1.00 | 27.53 | C |
| ATOM | 5471 | CG | ASP | D | 72 | 44.601 | 7.033 | 78.526 | 1.00 | 31.23 | C |
| ATOM | 5472 | OD1 | ASP | D | 72 | 44.534 | 5.782 | 78.582 | 1.00 | 31.01 | O |
| ATOM | 5473 | OD2 | ASP | D | 72 | 44.599 | 7.777 | 79.524 | 1.00 | 36.26 | O |
| ATOM | 5474 | N | ASP | D | 73 | 44.767 | 4.468 | 75.465 | 1.00 | 27.16 | N |
| ATOM | 5475 | CA | ASP | D | 73 | 43.932 | 3.416 | 74.936 | 1.00 | 29.69 | C |
| ATOM | 5476 | C | ASP | D | 73 | 42.839 | 3.037 | 75.943 | 1.00 | 31.45 | C |
| ATOM | 5477 | O | ASP | D | 73 | 41.700 | 2.764 | 75.545 | 1.00 | 31.82 | O |
| ATOM | 5478 | CB | ASP | D | 73 | 44.762 | 2.187 | 74.572 | 1.00 | 30.72 | C |
| ATOM | 5479 | CG | ASP | D | 73 | 45.598 | 2.389 | 73.319 | 1.00 | 30.51 | C |
| ATOM | 5480 | OD1 | ASP | D | 73 | 45.305 | 3.327 | 72.547 | 1.00 | 28.32 | O |
| ATOM | 5481 | OD2 | ASP | D | 73 | 46.548 | 1.602 | 73.129 | 1.00 | 29.95 | O |
| ATOM | 5482 | N | SER | D | 74 | 43.178 | 3.076 | 77.234 | 1.00 | 32.97 | N |
| ATOM | 5483 | CA | SER | D | 74 | 42.227 | 2.730 | 78.301 | 1.00 | 34.41 | C |
| ATOM | 5484 | C | SER | D | 74 | 41.077 | 3.737 | 78.385 | 1.00 | 35.07 | C |
| ATOM | 5485 | O | SER | D | 74 | 40.001 | 3.428 | 78.920 | 1.00 | 35.27 | O |
| ATOM | 5486 | CB | SER | D | 74 | 42.943 | 2.630 | 79.644 | 1.00 | 34.82 | C |
| ATOM | 5487 | OG | SER | D | 74 | 43.583 | 3.850 | 79.981 | 1.00 | 37.72 | O |
| ATOM | 5488 | N | LYS | D | 75 | 41.299 | 4.938 | 77.843 | 1.00 | 33.79 | N |
| ATOM | 5489 | CA | LYS | D | 75 | 40.276 | 5.967 | 77.797 | 1.00 | 32.41 | C |
| ATOM | 5490 | C | LYS | D | 75 | 39.694 | 6.161 | 76.386 | 1.00 | 30.60 | C |
| ATOM | 5491 | O | LYS | D | 75 | 38.813 | 7.004 | 76.185 | 1.00 | 29.83 | O |
| ATOM | 5492 | CB | LYS | D | 75 | 40.868 | 7.290 | 78.311 | 1.00 | 34.42 | C |
| ATOM | 5493 | CG | LYS | D | 75 | 41.027 | 7.391 | 79.838 | 1.00 | 35.38 | C |
| ATOM | 5494 | CD | LYS | D | 75 | 41.505 | 8.796 | 80.241 | 1.00 | 35.92 | C |
| ATOM | 5495 | CE | LYS | D | 75 | 41.639 | 8.984 | 81.758 | 1.00 | 37.22 | C |
| ATOM | 5496 | NZ | LYS | D | 75 | 42.306 | 10.301 | 82.095 | 1.00 | 37.87 | N |
| ATOM | 5497 | N | SER | D | 76 | 40.168 | 5.394 | 75.404 | 1.00 | 28.67 | N |
| ATOM | 5498 | CA | SER | D | 76 | 39.735 | 5.586 | 74.014 | 1.00 | 27.86 | C |
| ATOM | 5499 | C | SER | D | 76 | 39.860 | 7.060 | 73.573 | 1.00 | 24.67 | C |
| ATOM | 5500 | O | SER | D | 76 | 39.037 | 7.545 | 72.845 | 1.00 | 24.33 | O |
| ATOM | 5501 | CB | SER | D | 76 | 38.283 | 5.088 | 73.838 | 1.00 | 28.20 | C |
| ATOM | 5502 | OG | SER | D | 76 | 38.211 | 3.693 | 74.087 | 1.00 | 29.43 | O |
| ATOM | 5503 | N | ARG | D | 77 | 40.916 | 7.734 | 74.035 | 1.00 | 23.48 | N |
| ATOM | 5504 | CA | ARG | D | 77 | 41.116 | 9.147 | 73.837 | 1.00 | 24.75 | C |
| ATOM | 5505 | C | ARG | D | 77 | 42.457 | 9.385 | 73.113 | 1.00 | 21.94 | C |
| ATOM | 5506 | O | ARG | D | 77 | 43.469 | 8.808 | 73.461 | 1.00 | 21.39 | O |
| ATOM | 5507 | CB | ARG | D | 77 | 41.142 | 9.897 | 75.163 | 1.00 | 25.39 | C |
| ATOM | 5508 | CG | ARG | D | 77 | 41.059 | 11.414 | 74.997 | 1.00 | 27.52 | C |
| ATOM | 5509 | CD | ARG | D | 77 | 40.646 | 12.142 | 76.277 | 1.00 | 30.39 | C |
| ATOM | 5510 | NE | ARG | D | 77 | 39.287 | 11.827 | 76.714 | 1.00 | 32.10 | N |
| ATOM | 5511 | CZ | ARG | D | 77 | 38.174 | 12.362 | 76.216 | 1.00 | 34.66 | C |
| ATOM | 5512 | NH1 | ARG | D | 77 | 38.202 | 13.262 | 75.212 | 1.00 | 34.50 | N |
| ATOM | 5513 | NH2 | ARG | D | 77 | 37.003 | 11.968 | 76.718 | 1.00 | 36.20 | N |
| ATOM | 5514 | N | LEU | D | 78 | 42.403 | 10.201 | 72.078 | 1.00 | 22.37 | N |
| ATOM | 5515 | CA | LEU | D | 78 | 43.583 | 10.674 | 71.382 | 1.00 | 21.21 | C |

| ATOM | 5516 | C | LEU | D | 78 | 43.748 | 12.147 | 71.743 | 1.00 | 20.47 | C |
| ATOM | 5517 | O | LEU | D | 78 | 42.750 | 12.857 | 71.883 | 1.00 | 20.25 | O |
| ATOM | 5518 | CB | LEU | D | 78 | 43.360 | 10.487 | 69.884 | 1.00 | 22.36 | C |
| ATOM | 5519 | CG | LEU | D | 78 | 44.449 | 11.010 | 68.936 | 1.00 | 22.26 | C |
| ATOM | 5520 | CD1 | LEU | D | 78 | 45.628 | 10.084 | 69.024 | 1.00 | 23.60 | C |
| ATOM | 5521 | CD2 | LEU | D | 78 | 43.956 | 11.104 | 67.524 | 1.00 | 23.07 | C |
| ATOM | 5522 | N | TYR | D | 79 | 45.004 | 12.619 | 71.851 | 1.00 | 20.81 | N |
| ATOM | 5523 | CA | TYR | D | 79 | 45.317 | 14.009 | 72.213 | 1.00 | 18.93 | C |
| ATOM | 5524 | C | TYR | D | 79 | 46.281 | 14.655 | 71.215 | 1.00 | 16.58 | C |
| ATOM | 5525 | O | TYR | D | 79 | 47.080 | 13.961 | 70.624 | 1.00 | 14.91 | O |
| ATOM | 5526 | CB | TYR | D | 79 | 46.012 | 14.066 | 73.552 | 1.00 | 22.55 | C |
| ATOM | 5527 | CG | TYR | D | 79 | 45.276 | 13.428 | 74.706 | 1.00 | 25.07 | C |
| ATOM | 5528 | CD1 | TYR | D | 79 | 44.344 | 14.144 | 75.442 | 1.00 | 26.11 | C |
| ATOM | 5529 | CD2 | TYR | D | 79 | 45.547 | 12.118 | 75.071 | 1.00 | 26.80 | C |
| ATOM | 5530 | CE1 | TYR | D | 79 | 43.662 | 13.538 | 76.523 | 1.00 | 28.49 | C |
| ATOM | 5531 | CE2 | TYR | D | 79 | 44.880 | 11.510 | 76.141 | 1.00 | 28.66 | C |
| ATOM | 5532 | CZ | TYR | D | 79 | 43.949 | 12.222 | 76.851 | 1.00 | 27.51 | C |
| ATOM | 5533 | OH | TYR | D | 79 | 43.322 | 11.595 | 77.912 | 1.00 | 31.51 | O |
| ATOM | 5534 | N | LEU | D | 80 | 46.178 | 15.971 | 71.049 | 1.00 | 15.49 | N |
| ATOM | 5535 | CA | LEU | D | 80 | 47.227 | 16.766 | 70.339 | 1.00 | 16.13 | C |
| ATOM | 5536 | C | LEU | D | 80 | 47.590 | 17.939 | 71.231 | 1.00 | 16.16 | C |
| ATOM | 5537 | O | LEU | D | 80 | 46.753 | 18.793 | 71.470 | 1.00 | 18.65 | O |
| ATOM | 5538 | CB | LEU | D | 80 | 46.747 | 17.286 | 69.010 | 1.00 | 13.83 | C |
| ATOM | 5539 | CG | LEU | D | 80 | 47.878 | 17.948 | 68.133 | 1.00 | 11.72 | C |
| ATOM | 5540 | CD1 | LEU | D | 80 | 48.882 | 16.938 | 67.728 | 1.00 | 13.38 | C |
| ATOM | 5541 | CD2 | LEU | D | 80 | 47.203 | 18.497 | 66.966 | 1.00 | 13.24 | C |
| ATOM | 5542 | N | GLN | D | 81 | 48.826 | 17.951 | 71.725 | 1.00 | 16.70 | N |
| ATOM | 5543 | CA | GLN | D | 81 | 49.356 | 19.045 | 72.492 | 1.00 | 18.81 | C |
| ATOM | 5544 | C | GLN | D | 81 | 50.093 | 19.944 | 71.538 | 1.00 | 20.31 | C |
| ATOM | 5545 | O | GLN | D | 81 | 51.005 | 19.476 | 70.849 | 1.00 | 20.36 | O |
| ATOM | 5546 | CB | GLN | D | 81 | 50.329 | 18.562 | 73.558 | 1.00 | 21.39 | C |
| ATOM | 5547 | CG | GLN | D | 81 | 51.059 | 19.698 | 74.289 | 1.00 | 25.10 | C |
| ATOM | 5548 | CD | GLN | D | 81 | 50.166 | 20.505 | 75.189 | 1.00 | 28.15 | C |
| ATOM | 5549 | OE1 | GLN | D | 81 | 49.544 | 19.969 | 76.113 | 1.00 | 31.60 | O |
| ATOM | 5550 | NE2 | GLN | D | 81 | 50.136 | 21.815 | 74.976 | 1.00 | 29.58 | N |
| ATOM | 5551 | N | MET | D | 82 | 49.703 | 21.227 | 71.509 | 1.00 | 21.94 | N |
| ATOM | 5552 | CA | MET | D | 82 | 50.229 | 22.205 | 70.537 | 1.00 | 23.80 | C |
| ATOM | 5553 | C | MET | D | 82 | 50.887 | 23.344 | 71.278 | 1.00 | 23.78 | C |
| ATOM | 5554 | O | MET | D | 82 | 50.250 | 23.958 | 72.102 | 1.00 | 23.14 | O |
| ATOM | 5555 | CB | MET | D | 82 | 49.080 | 22.741 | 69.692 | 1.00 | 23.94 | C |
| ATOM | 5556 | CG | MET | D | 82 | 48.473 | 21.686 | 68.790 | 1.00 | 24.64 | C |
| ATOM | 5557 | SD | MET | D | 82 | 46.998 | 22.181 | 67.927 | 1.00 | 26.42 | S |
| ATOM | 5558 | CE | MET | D | 82 | 45.826 | 22.337 | 69.277 | 1.00 | 25.08 | C |
| ATOM | 5559 | N | ASN | D | 82A | 52.179 | 23.570 | 71.021 | 1.00 | 22.60 | N |
| ATOM | 5560 | CA | ASN | D | 82A | 52.940 | 24.628 | 71.672 | 1.00 | 21.72 | C |
| ATOM | 5561 | C | ASN | D | 82A | 53.386 | 25.637 | 70.598 | 1.00 | 21.10 | C |
| ATOM | 5562 | O | ASN | D | 82A | 53.474 | 25.288 | 69.427 | 1.00 | 18.82 | O |
| ATOM | 5563 | CB | ASN | D | 82A | 54.175 | 24.070 | 72.410 | 1.00 | 23.79 | C |
| ATOM | 5564 | CG | ASN | D | 82A | 53.823 | 23.099 | 73.577 | 1.00 | 26.26 | C |
| ATOM | 5565 | OD1 | ASN | D | 82A | 52.928 | 23.344 | 74.383 | 1.00 | 28.81 | O |
| ATOM | 5566 | ND2 | ASN | D | 82A | 54.576 | 22.005 | 73.670 | 1.00 | 31.22 | N |
| ATOM | 5567 | N | ASN | D | 82B | 53.629 | 26.884 | 71.017 | 1.00 | 21.11 | N |
| ATOM | 5568 | CA | ASN | D | 82B | 54.011 | 27.986 | 70.109 | 1.00 | 21.02 | C |
| ATOM | 5569 | C | ASN | D | 82B | 53.110 | 28.075 | 68.888 | 1.00 | 21.38 | C |
| ATOM | 5570 | O | ASN | D | 82B | 53.573 | 28.113 | 67.713 | 1.00 | 19.24 | O |
| ATOM | 5571 | CB | ASN | D | 82B | 55.489 | 27.864 | 69.701 | 1.00 | 21.22 | C |
| ATOM | 5572 | CG | ASN | D | 82B | 55.962 | 29.045 | 68.886 | 1.00 | 21.03 | C |
| ATOM | 5573 | OD1 | ASN | D | 82B | 55.464 | 30.143 | 69.045 | 1.00 | 21.75 | O |
| ATOM | 5574 | ND2 | ASN | D | 82B | 56.894 | 28.808 | 67.984 | 1.00 | 20.35 | N |
| ATOM | 5575 | N | LEU | D | 82C | 51.806 | 28.091 | 69.163 | 1.00 | 18.01 | N |
| ATOM | 5576 | CA | LEU | D | 82C | 50.816 | 28.210 | 68.124 | 1.00 | 18.28 | C |
| ATOM | 5577 | C | LEU | D | 82C | 50.953 | 29.568 | 67.446 | 1.00 | 19.50 | C |
| ATOM | 5578 | O | LEU | D | 82C | 51.190 | 30.568 | 68.096 | 1.00 | 20.17 | O |
| ATOM | 5579 | CB | LEU | D | 82C | 49.408 | 28.071 | 68.707 | 1.00 | 18.81 | C |
| ATOM | 5580 | CG | LEU | D | 82C | 49.022 | 26.618 | 69.028 | 1.00 | 17.21 | C |

```
ATOM   5581   CD1  LEU  D   82C    47.795   26.558   69.930   1.00  18.58        C
ATOM   5582   CD2  LEU  D   82C    48.752   25.857   67.751   1.00  19.08        C
ATOM   5583   N    ARG  D   83     50.799   29.592   66.132   1.00  21.40        N
ATOM   5584   CA   ARG  D   83     50.822   30.840   65.384   1.00  21.98        C
ATOM   5585   C    ARG  D   83     49.683   30.885   64.411   1.00  24.07        C
ATOM   5586   O    ARG  D   83     48.912   29.921   64.286   1.00  23.96        O
ATOM   5587   CB   ARG  D   83     52.176   30.968   64.661   1.00  22.55        C
ATOM   5588   CG   ARG  D   83     53.340   30.952   65.645   1.00  20.70        C
ATOM   5589   CD   ARG  D   83     53.451   32.299   66.311   1.00  23.54        C
ATOM   5590   NE   ARG  D   83     54.497   32.290   67.306   1.00  24.60        N
ATOM   5591   CZ   ARG  D   83     54.933   33.354   67.967   1.00  25.85        C
ATOM   5592   NH1  ARG  D   83     54.436   34.553   67.732   1.00  26.18        N
ATOM   5593   NH2  ARG  D   83     55.897   33.199   68.861   1.00  27.97        N
ATOM   5594   N    THR  D   84     49.560   32.032   63.748   1.00  25.04        N
ATOM   5595   CA   THR  D   84     48.466   32.320   62.831   1.00  26.63        C
ATOM   5596   C    THR  D   84     48.216   31.160   61.882   1.00  27.10        C
ATOM   5597   O    THR  D   84     47.057   30.790   61.648   1.00  26.47        O
ATOM   5598   CB   THR  D   84     48.748   33.609   61.990   1.00  27.51        C
ATOM   5599   OG1  THR  D   84     48.904   34.744   62.862   1.00  26.88        O
ATOM   5600   CG2  THR  D   84     47.602   33.863   61.004   1.00  28.98        C
ATOM   5601   N    GLU  D   85     49.304   30.565   61.363   1.00  27.43        N
ATOM   5602   CA   GLU  D   85     49.233   29.474   60.374   1.00  28.19        C
ATOM   5603   C    GLU  D   85     48.614   28.171   60.932   1.00  24.63        C
ATOM   5604   O    GLU  D   85     48.255   27.280   60.171   1.00  23.82        O
ATOM   5605   CB   GLU  D   85     50.609   29.171   59.746   1.00  29.15        C
ATOM   5606   CG   GLU  D   85     51.843   29.832   60.432   1.00  33.85        C
ATOM   5607   CD   GLU  D   85     51.759   31.356   60.488   1.00  33.79        C
ATOM   5608   OE1  GLU  D   85     51.537   32.042   59.448   1.00  41.45        O
ATOM   5609   OE2  GLU  D   85     51.913   31.879   61.578   1.00  32.22        O
ATOM   5610   N    ASP  D   86     48.496   28.093   62.259   1.00  22.11        N
ATOM   5611   CA   ASP  D   86     47.918   26.907   62.912   1.00  21.36        C
ATOM   5612   C    ASP  D   86     46.410   27.027   63.209   1.00  21.00        C
ATOM   5613   O    ASP  D   86     45.794   26.055   63.677   1.00  21.56        O
ATOM   5614   CB   ASP  D   86     48.691   26.620   64.196   1.00  20.13        C
ATOM   5615   CG   ASP  D   86     50.189   26.394   63.953   1.00  20.40        C
ATOM   5616   OD1  ASP  D   86     50.571   25.561   63.075   1.00  16.25        O
ATOM   5617   OD2  ASP  D   86     50.970   27.032   64.683   1.00  17.83        O
ATOM   5618   N    THR  D   87     45.835   28.193   62.946   1.00  21.27        N
ATOM   5619   CA   THR  D   87     44.381   28.397   63.012   1.00  21.77        C
ATOM   5620   C    THR  D   87     43.669   27.481   62.032   1.00  20.47        C
ATOM   5621   O    THR  D   87     44.014   27.421   60.862   1.00  17.32        O
ATOM   5622   CB   THR  D   87     43.972   29.851   62.693   1.00  21.90        C
ATOM   5623   OG1  THR  D   87     44.337   30.695   63.776   1.00  20.46        O
ATOM   5624   CG2  THR  D   87     42.459   29.974   62.473   1.00  21.86        C
ATOM   5625   N    GLY  D   88     42.650   26.764   62.512   1.00  16.95        N
ATOM   5626   CA   GLY  D   88     41.907   25.939   61.614   1.00  17.56        C
ATOM   5627   C    GLY  D   88     40.984   25.030   62.380   1.00  18.46        C
ATOM   5628   O    GLY  D   88     40.926   25.112   63.598   1.00  16.66        O
ATOM   5629   N    ILE  D   89     40.317   24.149   61.644   1.00  18.93        N
ATOM   5630   CA   ILE  D   89     39.505   23.089   62.235   1.00  18.18        C
ATOM   5631   C    ILE  D   89     40.319   21.828   62.302   1.00  16.56        C
ATOM   5632   O    ILE  D   89     40.802   21.329   61.281   1.00  16.94        O
ATOM   5633   CB   ILE  D   89     38.245   22.802   61.394   1.00  18.82        C
ATOM   5634   CG1  ILE  D   89     37.398   24.065   61.280   1.00  20.44        C
ATOM   5635   CG2  ILE  D   89     37.438   21.643   62.051   1.00  18.04        C
ATOM   5636   CD1  ILE  D   89     36.302   23.988   60.239   1.00  22.45        C
ATOM   5637   N    TYR  D   90     40.478   21.270   63.502   1.00  14.83        N
ATOM   5638   CA   TYR  D   90     41.249   20.051   63.665   1.00  14.31        C
ATOM   5639   C    TYR  D   90     40.370   18.809   63.797   1.00  17.84        C
ATOM   5640   O    TYR  D   90     39.511   18.750   64.697   1.00  14.25        O
ATOM   5641   CB   TYR  D   90     42.155   20.191   64.890   1.00  15.33        C
ATOM   5642   CG   TYR  D   90     43.343   21.132   64.670   1.00  13.87        C
ATOM   5643   CD1  TYR  D   90     43.162   22.486   64.518   1.00  14.72        C
ATOM   5644   CD2  TYR  D   90     44.641   20.644   64.608   1.00  17.06        C
ATOM   5645   CE1  TYR  D   90     44.265   23.366   64.266   1.00  14.86        C
```

| ATOM | 5646 | CE2 | TYR | D | 90 | 45.740 | 21.524 | 64.395 | 1.00 | 14.95 | C |
|------|------|-----|-----|---|-----|--------|--------|--------|------|-------|---|
| ATOM | 5647 | CZ | TYR | D | 90 | 45.533 | 22.867 | 64.225 | 1.00 | 14.38 | C |
| ATOM | 5648 | OH | TYR | D | 90 | 46.603 | 23.758 | 63.999 | 1.00 | 16.84 | O |
| ATOM | 5649 | N | TYR | D | 91 | 40.639 | 17.803 | 62.957 | 1.00 | 19.08 | N |
| ATOM | 5650 | CA | TYR | D | 91 | 39.889 | 16.557 | 62.933 | 1.00 | 19.58 | C |
| ATOM | 5651 | C | TYR | D | 91 | 40.736 | 15.432 | 63.368 | 1.00 | 19.36 | C |
| ATOM | 5652 | O | TYR | D | 91 | 41.899 | 15.382 | 63.004 | 1.00 | 16.32 | O |
| ATOM | 5653 | CB | TYR | D | 91 | 39.448 | 16.221 | 61.506 | 1.00 | 20.90 | C |
| ATOM | 5654 | CG | TYR | D | 91 | 38.530 | 17.220 | 60.846 | 1.00 | 22.28 | C |
| ATOM | 5655 | CD1 | TYR | D | 91 | 37.143 | 17.137 | 61.008 | 1.00 | 21.66 | C |
| ATOM | 5656 | CD2 | TYR | D | 91 | 39.040 | 18.254 | 60.041 | 1.00 | 21.03 | C |
| ATOM | 5657 | CE1 | TYR | D | 91 | 36.292 | 18.025 | 60.367 | 1.00 | 22.24 | C |
| ATOM | 5658 | CE2 | TYR | D | 91 | 38.197 | 19.160 | 59.429 | 1.00 | 21.85 | C |
| ATOM | 5659 | CZ | TYR | D | 91 | 36.822 | 19.038 | 59.590 | 1.00 | 21.51 | C |
| ATOM | 5660 | OH | TYR | D | 91 | 35.974 | 19.929 | 58.976 | 1.00 | 23.36 | O |
| ATOM | 5661 | N | CYS | D | 92 | 40.164 | 14.503 | 64.138 | 1.00 | 17.04 | N |
| ATOM | 5662 | CA | CYS | D | 92 | 40.751 | 13.182 | 64.311 | 1.00 | 19.37 | C |
| ATOM | 5663 | C | CYS | D | 92 | 40.403 | 12.359 | 63.101 | 1.00 | 19.41 | C |
| ATOM | 5664 | O | CYS | D | 92 | 39.239 | 12.267 | 62.701 | 1.00 | 19.34 | O |
| ATOM | 5665 | CB | CYS | D | 92 | 40.215 | 12.469 | 65.573 | 1.00 | 23.00 | C |
| ATOM | 5666 | SG | CYS | D | 92 | 41.001 | 13.103 | 67.031 | 1.00 | 29.05 | S |
| ATOM | 5667 | N | PHE | D | 93 | 41.425 | 11.780 | 62.486 | 1.00 | 19.58 | N |
| ATOM | 5668 | CA | PHE | D | 93 | 41.249 | 10.964 | 61.305 | 1.00 | 18.82 | C |
| ATOM | 5669 | C | PHE | D | 93 | 41.889 | 9.619 | 61.586 | 1.00 | 18.68 | C |
| ATOM | 5670 | O | PHE | D | 93 | 43.079 | 9.533 | 61.881 | 1.00 | 17.01 | O |
| ATOM | 5671 | CB | PHE | D | 93 | 41.874 | 11.679 | 60.078 | 1.00 | 19.45 | C |
| ATOM | 5672 | CG | PHE | D | 93 | 41.945 | 10.829 | 58.820 | 1.00 | 18.13 | C |
| ATOM | 5673 | CD1 | PHE | D | 93 | 40.828 | 10.225 | 58.315 | 1.00 | 18.58 | C |
| ATOM | 5674 | CD2 | PHE | D | 93 | 43.144 | 10.699 | 58.117 | 1.00 | 19.17 | C |
| ATOM | 5675 | CE1 | PHE | D | 93 | 40.894 | 9.506 | 57.163 | 1.00 | 19.55 | C |
| ATOM | 5676 | CE2 | PHE | D | 93 | 43.229 | 9.946 | 56.978 | 1.00 | 18.37 | C |
| ATOM | 5677 | CZ | PHE | D | 93 | 42.104 | 9.381 | 56.466 | 1.00 | 18.98 | C |
| ATOM | 5678 | N | LEU | D | 94 | 41.071 | 8.580 | 61.518 | 1.00 | 19.00 | N |
| ATOM | 5679 | CA | LEU | D | 94 | 41.500 | 7.209 | 61.680 | 1.00 | 19.81 | C |
| ATOM | 5680 | C | LEU | D | 94 | 41.747 | 6.717 | 60.284 | 1.00 | 20.02 | C |
| ATOM | 5681 | O | LEU | D | 94 | 40.791 | 6.421 | 59.565 | 1.00 | 19.28 | O |
| ATOM | 5682 | CB | LEU | D | 94 | 40.394 | 6.364 | 62.336 | 1.00 | 19.59 | C |
| ATOM | 5683 | CG | LEU | D | 94 | 40.357 | 6.472 | 63.852 | 1.00 | 20.85 | C |
| ATOM | 5684 | CD1 | LEU | D | 94 | 39.974 | 7.865 | 64.297 | 1.00 | 21.64 | C |
| ATOM | 5685 | CD2 | LEU | D | 94 | 39.406 | 5.402 | 64.445 | 1.00 | 21.56 | C |
| ATOM | 5686 | N | PRO | D | 95 | 43.032 | 6.660 | 59.856 | 1.00 | 20.42 | N |
| ATOM | 5687 | CA | PRO | D | 95 | 43.182 | 6.383 | 58.430 | 1.00 | 20.34 | C |
| ATOM | 5688 | C | PRO | D | 95 | 42.794 | 4.946 | 58.094 | 1.00 | 20.59 | C |
| ATOM | 5689 | O | PRO | D | 95 | 43.212 | 4.025 | 58.798 | 1.00 | 21.36 | O |
| ATOM | 5690 | CB | PRO | D | 95 | 44.687 | 6.620 | 58.147 | 1.00 | 21.27 | C |
| ATOM | 5691 | CG | PRO | D | 95 | 45.262 | 7.264 | 59.406 | 1.00 | 21.07 | C |
| ATOM | 5692 | CD | PRO | D | 95 | 44.330 | 6.786 | 60.542 | 1.00 | 20.33 | C |
| ATOM | 5693 | N | MET | D | 96 | 42.031 | 4.704 | 57.027 | 1.00 | 21.98 | N |
| ATOM | 5694 | CA | MET | D | 96 | 41.574 | 5.672 | 56.033 | 1.00 | 21.81 | C |
| ATOM | 5695 | C | MET | D | 96 | 40.047 | 5.863 | 56.123 | 1.00 | 21.97 | C |
| ATOM | 5696 | O | MET | D | 96 | 39.456 | 6.586 | 55.320 | 1.00 | 18.74 | O |
| ATOM | 5697 | CB | MET | D | 96 | 41.919 | 5.147 | 54.643 | 1.00 | 22.48 | C |
| ATOM | 5698 | CG | MET | D | 96 | 43.410 | 5.048 | 54.327 | 1.00 | 23.12 | C |
| ATOM | 5699 | SD | MET | D | 96 | 44.241 | 6.631 | 54.437 | 1.00 | 22.80 | S |
| ATOM | 5700 | CE | MET | D | 96 | 43.650 | 7.459 | 52.988 | 1.00 | 22.74 | C |
| ATOM | 5701 | N | ASP | D | 101 | 39.409 | 5.256 | 57.124 | 1.00 | 24.05 | N |
| ATOM | 5702 | CA | ASP | D | 101 | 37.933 | 5.121 | 57.115 | 1.00 | 24.73 | C |
| ATOM | 5703 | C | ASP | D | 101 | 37.089 | 6.117 | 57.921 | 1.00 | 25.48 | C |
| ATOM | 5704 | O | ASP | D | 101 | 35.945 | 6.353 | 57.551 | 1.00 | 26.45 | O |
| ATOM | 5705 | CB | ASP | D | 101 | 37.542 | 3.712 | 57.568 | 1.00 | 26.25 | C |
| ATOM | 5706 | CG | ASP | D | 101 | 38.022 | 2.646 | 56.621 | 1.00 | 29.47 | C |
| ATOM | 5707 | OD1 | ASP | D | 101 | 38.716 | 2.974 | 55.633 | 1.00 | 30.38 | O |
| ATOM | 5708 | OD2 | ASP | D | 101 | 37.714 | 1.469 | 56.860 | 1.00 | 33.88 | O |
| ATOM | 5709 | N | TYR | D | 102 | 37.613 | 6.684 | 59.014 | 1.00 | 24.12 | N |
| ATOM | 5710 | CA | TYR | D | 102 | 36.770 | 7.497 | 59.921 | 1.00 | 23.60 | C |

| ATOM | 5711 | C | TYR | D | 102 | 37.280 | 8.892 | 60.247 | 1.00 | 21.44 | C |
|------|------|-----|-----|---|-----|--------|--------|--------|------|-------|---|
| ATOM | 5712 | O | TYR | D | 102 | 38.435 | 9.060 | 60.601 | 1.00 | 21.22 | O |
| ATOM | 5713 | CB | TYR | D | 102 | 36.548 | 6.750 | 61.239 | 1.00 | 21.96 | C |
| ATOM | 5714 | CG | TYR | D | 102 | 36.149 | 5.312 | 61.040 | 1.00 | 22.97 | C |
| ATOM | 5715 | CD1 | TYR | D | 102 | 34.855 | 4.974 | 60.648 | 1.00 | 24.17 | C |
| ATOM | 5716 | CD2 | TYR | D | 102 | 37.041 | 4.283 | 61.286 | 1.00 | 22.79 | C |
| ATOM | 5717 | CE1 | TYR | D | 102 | 34.462 | 3.616 | 60.488 | 1.00 | 24.46 | C |
| ATOM | 5718 | CE2 | TYR | D | 102 | 36.670 | 2.926 | 61.120 | 1.00 | 23.82 | C |
| ATOM | 5719 | CZ | TYR | D | 102 | 35.386 | 2.611 | 60.722 | 1.00 | 24.37 | C |
| ATOM | 5720 | OH | TYR | D | 102 | 35.035 | 1.282 | 60.572 | 1.00 | 26.70 | O |
| ATOM | 5721 | N | TRP | D | 103 | 36.382 | 9.872 | 60.135 | 1.00 | 21.13 | N |
| ATOM | 5722 | CA | TRP | D | 103 | 36.610 | 11.245 | 60.580 | 1.00 | 19.81 | C |
| ATOM | 5723 | C | TRP | D | 103 | 35.691 | 11.640 | 61.727 | 1.00 | 21.17 | C |
| ATOM | 5724 | O | TRP | D | 103 | 34.544 | 11.166 | 61.808 | 1.00 | 18.77 | O |
| ATOM | 5725 | CB | TRP | D | 103 | 36.342 | 12.216 | 59.459 | 1.00 | 18.81 | C |
| ATOM | 5726 | CG | TRP | D | 103 | 37.264 | 12.075 | 58.267 | 1.00 | 19.24 | C |
| ATOM | 5727 | CD1 | TRP | D | 103 | 37.206 | 11.124 | 57.302 | 1.00 | 19.44 | C |
| ATOM | 5728 | CD2 | TRP | D | 103 | 38.319 | 12.970 | 57.881 | 1.00 | 19.01 | C |
| ATOM | 5729 | NE1 | TRP | D | 103 | 38.182 | 11.343 | 56.359 | 1.00 | 19.47 | N |
| ATOM | 5730 | CE2 | TRP | D | 103 | 38.873 | 12.474 | 56.689 | 1.00 | 18.42 | C |
| ATOM | 5731 | CE3 | TRP | D | 103 | 38.852 | 14.131 | 58.437 | 1.00 | 20.15 | C |
| ATOM | 5732 | CZ2 | TRP | D | 103 | 39.956 | 13.109 | 56.027 | 1.00 | 20.20 | C |
| ATOM | 5733 | CZ3 | TRP | D | 103 | 39.920 | 14.763 | 57.773 | 1.00 | 18.74 | C |
| ATOM | 5734 | CH2 | TRP | D | 103 | 40.456 | 14.239 | 56.598 | 1.00 | 17.36 | C |
| ATOM | 5735 | N | GLY | D | 104 | 36.194 | 12.537 | 62.576 | 1.00 | 19.99 | N |
| ATOM | 5736 | CA | GLY | D | 104 | 35.381 | 13.252 | 63.524 | 1.00 | 21.13 | C |
| ATOM | 5737 | C | GLY | D | 104 | 34.738 | 14.489 | 62.974 | 1.00 | 23.58 | C |
| ATOM | 5738 | O | GLY | D | 104 | 34.728 | 14.703 | 61.783 | 1.00 | 23.58 | O |
| ATOM | 5739 | N | GLN | D | 105 | 34.237 | 15.335 | 63.870 | 1.00 | 24.67 | N |
| ATOM | 5740 | CA | GLN | D | 105 | 33.475 | 16.527 | 63.490 | 1.00 | 26.86 | C |
| ATOM | 5741 | C | GLN | D | 105 | 34.321 | 17.776 | 63.381 | 1.00 | 25.93 | C |
| ATOM | 5742 | O | GLN | D | 105 | 33.909 | 18.744 | 62.759 | 1.00 | 25.27 | O |
| ATOM | 5743 | CB | GLN | D | 105 | 32.332 | 16.798 | 64.473 | 1.00 | 29.54 | C |
| ATOM | 5744 | CG | GLN | D | 105 | 32.563 | 16.291 | 65.929 | 1.00 | 32.82 | C |
| ATOM | 5745 | CD | GLN | D | 105 | 33.111 | 17.339 | 66.898 | 1.00 | 35.24 | C |
| ATOM | 5746 | OE1 | GLN | D | 105 | 32.848 | 18.535 | 66.784 | 1.00 | 38.63 | O |
| ATOM | 5747 | NE2 | GLN | D | 105 | 33.812 | 16.872 | 67.901 | 1.00 | 37.14 | N |
| ATOM | 5748 | N | GLY | D | 106 | 35.500 | 17.743 | 63.976 | 1.00 | 24.88 | N |
| ATOM | 5749 | CA | GLY | D | 106 | 36.375 | 18.906 | 64.007 | 1.00 | 24.02 | C |
| ATOM | 5750 | C | GLY | D | 106 | 36.166 | 19.711 | 65.264 | 1.00 | 23.48 | C |
| ATOM | 5751 | O | GLY | D | 106 | 35.043 | 19.803 | 65.797 | 1.00 | 23.52 | O |
| ATOM | 5752 | N | THR | D | 107 | 37.256 | 20.247 | 65.787 | 1.00 | 21.91 | N |
| ATOM | 5753 | CA | THR | D | 107 | 37.214 | 21.247 | 66.832 | 1.00 | 21.87 | C |
| ATOM | 5754 | C | THR | D | 107 | 37.997 | 22.472 | 66.311 | 1.00 | 22.23 | C |
| ATOM | 5755 | O | THR | D | 107 | 39.064 | 22.324 | 65.740 | 1.00 | 20.15 | O |
| ATOM | 5756 | CB | THR | D | 107 | 37.784 | 20.734 | 68.159 | 1.00 | 22.54 | C |
| ATOM | 5757 | OG1 | THR | D | 107 | 37.557 | 21.708 | 69.186 | 1.00 | 22.40 | O |
| ATOM | 5758 | CG2 | THR | D | 107 | 39.303 | 20.431 | 68.082 | 1.00 | 22.63 | C |
| ATOM | 5759 | N | SER | D | 108 | 37.431 | 23.659 | 66.508 | 1.00 | 21.88 | N |
| ATOM | 5760 | CA | SER | D | 108 | 37.996 | 24.900 | 66.010 | 1.00 | 22.70 | C |
| ATOM | 5761 | C | SER | D | 108 | 39.097 | 25.512 | 66.913 | 1.00 | 22.53 | C |
| ATOM | 5762 | O | SER | D | 108 | 38.889 | 25.683 | 68.099 | 1.00 | 19.99 | O |
| ATOM | 5763 | CB | SER | D | 108 | 36.884 | 25.898 | 65.806 | 1.00 | 23.51 | C |
| ATOM | 5764 | OG | SER | D | 108 | 37.429 | 27.002 | 65.128 | 1.00 | 27.16 | O |
| ATOM | 5765 | N | VAL | D | 109 | 40.249 | 25.842 | 66.302 | 1.00 | 20.07 | N |
| ATOM | 5766 | CA | VAL | D | 109 | 41.442 | 26.375 | 66.965 | 1.00 | 20.53 | C |
| ATOM | 5767 | C | VAL | D | 109 | 41.743 | 27.724 | 66.280 | 1.00 | 19.79 | C |
| ATOM | 5768 | O | VAL | D | 109 | 41.891 | 27.793 | 65.076 | 1.00 | 17.12 | O |
| ATOM | 5769 | CB | VAL | D | 109 | 42.692 | 25.401 | 66.891 | 1.00 | 20.02 | C |
| ATOM | 5770 | CG1 | VAL | D | 109 | 43.985 | 26.079 | 67.430 | 1.00 | 20.03 | C |
| ATOM | 5771 | CG2 | VAL | D | 109 | 42.445 | 24.109 | 67.665 | 1.00 | 21.24 | C |
| ATOM | 5772 | N | THR | D | 110 | 41.650 | 28.789 | 67.057 | 1.00 | 19.85 | N |
| ATOM | 5773 | CA | THR | D | 110 | 41.852 | 30.121 | 66.581 | 1.00 | 21.49 | C |
| ATOM | 5774 | C | THR | D | 110 | 43.047 | 30.643 | 67.333 | 1.00 | 21.24 | C |
| ATOM | 5775 | O | THR | D | 110 | 43.068 | 30.628 | 68.572 | 1.00 | 19.52 | O |

| ATOM | 5776 | CB | THR | D | 110 | 40.650 | 31.046 | 66.861 | 1.00 | 23.18 | C |
|------|------|-----|-----|---|-----|--------|--------|--------|------|-------|---|
| ATOM | 5777 | OG1 | THR | D | 110 | 39.435 | 30.430 | 66.420 | 1.00 | 25.75 | O |
| ATOM | 5778 | CG2 | THR | D | 110 | 40.816 | 32.343 | 66.096 | 1.00 | 24.41 | C |
| ATOM | 5779 | N | VAL | D | 111 | 44.058 | 31.062 | 66.570 | 1.00 | 20.35 | N |
| ATOM | 5780 | CA | VAL | D | 111 | 45.228 | 31.662 | 67.135 | 1.00 | 19.77 | C |
| ATOM | 5781 | C | VAL | D | 111 | 45.278 | 33.125 | 66.686 | 1.00 | 21.55 | C |
| ATOM | 5782 | O | VAL | D | 111 | 45.435 | 33.410 | 65.490 | 1.00 | 19.42 | O |
| ATOM | 5783 | CB | VAL | D | 111 | 46.507 | 30.965 | 66.674 | 1.00 | 20.19 | C |
| ATOM | 5784 | CG1 | VAL | D | 111 | 47.685 | 31.407 | 67.569 | 1.00 | 20.09 | C |
| ATOM | 5785 | CG2 | VAL | D | 111 | 46.378 | 29.467 | 66.666 | 1.00 | 18.29 | C |
| ATOM | 5786 | N | SER | D | 112 | 45.125 | 34.036 | 67.642 | 1.00 | 21.99 | N |
| ATOM | 5787 | CA | SER | D | 112 | 44.988 | 35.462 | 67.359 | 1.00 | 23.34 | C |
| ATOM | 5788 | C | SER | D | 112 | 45.199 | 36.302 | 68.632 | 1.00 | 23.76 | C |
| ATOM | 5789 | O | SER | D | 112 | 44.954 | 35.828 | 69.754 | 1.00 | 21.02 | O |
| ATOM | 5790 | CB | SER | D | 112 | 43.603 | 35.761 | 66.768 | 1.00 | 24.32 | C |
| ATOM | 5791 | OG | SER | D | 112 | 43.354 | 37.151 | 66.658 | 1.00 | 23.81 | O |
| ATOM | 5792 | N | SER | D | 113 | 45.623 | 37.558 | 68.453 | 1.00 | 23.46 | N |
| ATOM | 5793 | CA | SER | D | 113 | 45.692 | 38.499 | 69.587 | 1.00 | 24.25 | C |
| ATOM | 5794 | C | SER | D | 113 | 44.364 | 39.166 | 69.890 | 1.00 | 22.40 | C |
| ATOM | 5795 | O | SER | D | 113 | 44.257 | 39.849 | 70.896 | 1.00 | 21.34 | O |
| ATOM | 5796 | CB | SER | D | 113 | 46.740 | 39.617 | 69.390 | 1.00 | 25.21 | C |
| ATOM | 5797 | OG | SER | D | 113 | 47.662 | 39.316 | 68.374 | 1.00 | 28.94 | O |
| ATOM | 5798 | N | ALA | D | 114 | 43.368 | 39.014 | 69.025 | 1.00 | 22.31 | N |
| ATOM | 5799 | CA | ALA | D | 114 | 42.040 | 39.594 | 69.288 | 1.00 | 23.68 | C |
| ATOM | 5800 | C | ALA | D | 114 | 41.403 | 38.897 | 70.505 | 1.00 | 23.95 | C |
| ATOM | 5801 | O | ALA | D | 114 | 41.648 | 37.722 | 70.751 | 1.00 | 23.84 | O |
| ATOM | 5802 | CB | ALA | D | 114 | 41.168 | 39.439 | 68.065 | 1.00 | 24.25 | C |
| ATOM | 5803 | N | LYS | D | 115 | 40.612 | 39.611 | 71.279 | 1.00 | 25.07 | N |
| ATOM | 5804 | CA | LYS | D | 115 | 40.051 | 39.014 | 72.486 | 1.00 | 27.62 | C |
| ATOM | 5805 | C | LYS | D | 115 | 38.593 | 38.656 | 72.309 | 1.00 | 26.69 | C |
| ATOM | 5806 | O | LYS | D | 115 | 37.901 | 39.237 | 71.475 | 1.00 | 28.19 | O |
| ATOM | 5807 | CB | LYS | D | 115 | 40.232 | 39.967 | 73.660 | 1.00 | 28.05 | C |
| ATOM | 5808 | CG | LYS | D | 115 | 41.698 | 40.171 | 74.025 | 1.00 | 30.11 | C |
| ATOM | 5809 | CD | LYS | D | 115 | 41.861 | 41.039 | 75.263 | 1.00 | 30.34 | C |
| ATOM | 5810 | CE | LYS | D | 115 | 41.191 | 40.441 | 76.496 | 1.00 | 31.58 | C |
| ATOM | 5811 | NZ | LYS | D | 115 | 41.754 | 41.028 | 77.740 | 1.00 | 31.65 | N |
| ATOM | 5812 | N | THR | D | 116 | 38.118 | 37.716 | 73.107 | 1.00 | 26.44 | N |
| ATOM | 5813 | CA | THR | D | 116 | 36.721 | 37.346 | 73.082 | 1.00 | 25.84 | C |
| ATOM | 5814 | C | THR | D | 116 | 35.869 | 38.575 | 73.356 | 1.00 | 26.17 | C |
| ATOM | 5815 | O | THR | D | 116 | 36.076 | 39.254 | 74.338 | 1.00 | 26.50 | O |
| ATOM | 5816 | CB | THR | D | 116 | 36.403 | 36.266 | 74.120 | 1.00 | 27.07 | C |
| ATOM | 5817 | OG1 | THR | D | 116 | 37.249 | 35.127 | 73.904 | 1.00 | 27.93 | O |
| ATOM | 5818 | CG2 | THR | D | 116 | 34.945 | 35.811 | 74.017 | 1.00 | 27.26 | C |
| ATOM | 5819 | N | THR | D | 117 | 34.929 | 38.851 | 72.450 | 1.00 | 25.70 | N |
| ATOM | 5820 | CA | THR | D | 117 | 34.095 | 40.042 | 72.448 | 1.00 | 26.37 | C |
| ATOM | 5821 | C | THR | D | 117 | 32.714 | 39.630 | 71.896 | 1.00 | 26.43 | C |
| ATOM | 5822 | O | THR | D | 117 | 32.635 | 38.984 | 70.838 | 1.00 | 23.50 | O |
| ATOM | 5823 | CB | THR | D | 117 | 34.714 | 41.114 | 71.527 | 1.00 | 27.77 | C |
| ATOM | 5824 | OG1 | THR | D | 117 | 36.101 | 41.259 | 71.842 | 1.00 | 30.54 | O |
| ATOM | 5825 | CG2 | THR | D | 117 | 34.039 | 42.451 | 71.710 | 1.00 | 29.64 | C |
| ATOM | 5826 | N | PRO | D | 118 | 31.618 | 39.927 | 72.644 | 1.00 | 27.36 | N |
| ATOM | 5827 | CA | PRO | D | 118 | 30.316 | 39.499 | 72.121 | 1.00 | 26.24 | C |
| ATOM | 5828 | C | PRO | D | 118 | 29.861 | 40.401 | 70.984 | 1.00 | 25.34 | C |
| ATOM | 5829 | O | PRO | D | 118 | 30.364 | 41.502 | 70.853 | 1.00 | 25.89 | O |
| ATOM | 5830 | CB | PRO | D | 118 | 29.373 | 39.630 | 73.337 | 1.00 | 26.63 | C |
| ATOM | 5831 | CG | PRO | D | 118 | 30.007 | 40.652 | 74.202 | 1.00 | 27.41 | C |
| ATOM | 5832 | CD | PRO | D | 118 | 31.506 | 40.612 | 73.946 | 1.00 | 26.63 | C |
| ATOM | 5833 | N | PRO | D | 119 | 28.943 | 39.917 | 70.135 | 1.00 | 24.92 | N |
| ATOM | 5834 | CA | PRO | D | 119 | 28.491 | 40.745 | 69.037 | 1.00 | 24.98 | C |
| ATOM | 5835 | C | PRO | D | 119 | 27.560 | 41.860 | 69.450 | 1.00 | 26.37 | C |
| ATOM | 5836 | O | PRO | D | 119 | 26.944 | 41.821 | 70.544 | 1.00 | 27.57 | O |
| ATOM | 5837 | CB | PRO | D | 119 | 27.767 | 39.757 | 68.146 | 1.00 | 25.65 | C |
| ATOM | 5838 | CG | PRO | D | 119 | 27.214 | 38.707 | 69.137 | 1.00 | 25.02 | C |
| ATOM | 5839 | CD | PRO | D | 119 | 28.337 | 38.574 | 70.109 | 1.00 | 25.52 | C |
| ATOM | 5840 | N | SER | D | 120 | 27.487 | 42.871 | 68.592 | 1.00 | 25.72 | N |

| ATOM | 5841 | CA | SER | D | 120 | 26.392 | 43.847 | 68.633 | 1.00 | 26.54 | C |
|------|------|-----|-----|---|-----|--------|--------|--------|------|-------|---|
| ATOM | 5842 | C | SER | D | 120 | 25.411 | 43.424 | 67.573 | 1.00 | 25.58 | C |
| ATOM | 5843 | O | SER | D | 120 | 25.796 | 43.198 | 66.426 | 1.00 | 27.16 | O |
| ATOM | 5844 | CB | SER | D | 120 | 26.895 | 45.251 | 68.326 | 1.00 | 26.87 | C |
| ATOM | 5845 | OG | SER | D | 120 | 27.939 | 45.581 | 69.209 | 1.00 | 27.41 | O |
| ATOM | 5846 | N | VAL | D | 121 | 24.143 | 43.312 | 67.950 | 1.00 | 24.69 | N |
| ATOM | 5847 | CA | VAL | D | 121 | 23.143 | 42.813 | 67.028 | 1.00 | 23.63 | C |
| ATOM | 5848 | C | VAL | D | 121 | 22.177 | 43.946 | 66.734 | 1.00 | 23.44 | C |
| ATOM | 5849 | O | VAL | D | 121 | 21.587 | 44.518 | 67.654 | 1.00 | 22.57 | O |
| ATOM | 5850 | CB | VAL | D | 121 | 22.383 | 41.576 | 67.622 | 1.00 | 24.46 | C |
| ATOM | 5851 | CG1 | VAL | D | 121 | 21.323 | 41.068 | 66.647 | 1.00 | 24.43 | C |
| ATOM | 5852 | CG2 | VAL | D | 121 | 23.359 | 40.474 | 67.996 | 1.00 | 22.65 | C |
| ATOM | 5853 | N | TYR | D | 122 | 22.010 | 44.242 | 65.444 | 1.00 | 22.05 | N |
| ATOM | 5854 | CA | TYR | D | 122 | 21.211 | 45.350 | 64.976 | 1.00 | 22.31 | C |
| ATOM | 5855 | C | TYR | D | 122 | 20.104 | 44.841 | 64.097 | 1.00 | 22.56 | C |
| ATOM | 5856 | O | TYR | D | 122 | 20.342 | 44.036 | 63.226 | 1.00 | 23.33 | O |
| ATOM | 5857 | CB | TYR | D | 122 | 22.106 | 46.318 | 64.172 | 1.00 | 23.26 | C |
| ATOM | 5858 | CG | TYR | D | 122 | 23.221 | 46.933 | 65.000 | 1.00 | 22.57 | C |
| ATOM | 5859 | CD1 | TYR | D | 122 | 22.931 | 47.680 | 66.123 | 1.00 | 24.17 | C |
| ATOM | 5860 | CD2 | TYR | D | 122 | 24.548 | 46.791 | 64.646 | 1.00 | 23.94 | C |
| ATOM | 5861 | CE1 | TYR | D | 122 | 23.931 | 48.269 | 66.884 | 1.00 | 24.00 | C |
| ATOM | 5862 | CE2 | TYR | D | 122 | 25.569 | 47.376 | 65.413 | 1.00 | 22.80 | C |
| ATOM | 5863 | CZ | TYR | D | 122 | 25.250 | 48.117 | 66.519 | 1.00 | 22.89 | C |
| ATOM | 5864 | OH | TYR | D | 122 | 26.219 | 48.716 | 67.291 | 1.00 | 24.57 | O |
| ATOM | 5865 | N | PRO | D | 123 | 18.872 | 45.324 | 64.299 | 1.00 | 26.23 | N |
| ATOM | 5866 | CA | PRO | D | 123 | 17.804 | 44.832 | 63.435 | 1.00 | 27.66 | C |
| ATOM | 5867 | C | PRO | D | 123 | 17.875 | 45.510 | 62.083 | 1.00 | 27.66 | C |
| ATOM | 5868 | O | PRO | D | 123 | 18.277 | 46.665 | 62.012 | 1.00 | 29.23 | O |
| ATOM | 5869 | CB | PRO | D | 123 | 16.526 | 45.254 | 64.181 | 1.00 | 28.14 | C |
| ATOM | 5870 | CG | PRO | D | 123 | 16.932 | 46.546 | 64.873 | 1.00 | 28.67 | C |
| ATOM | 5871 | CD | PRO | D | 123 | 18.380 | 46.293 | 65.302 | 1.00 | 26.92 | C |
| ATOM | 5872 | N | LEU | D | 124 | 17.511 | 44.797 | 61.022 | 1.00 | 26.19 | N |
| ATOM | 5873 | CA | LEU | D | 124 | 17.347 | 45.396 | 59.712 | 1.00 | 26.28 | C |
| ATOM | 5874 | C | LEU | D | 124 | 15.884 | 45.394 | 59.289 | 1.00 | 27.64 | C |
| ATOM | 5875 | O | LEU | D | 124 | 15.354 | 44.373 | 58.848 | 1.00 | 27.37 | O |
| ATOM | 5876 | CB | LEU | D | 124 | 18.202 | 44.696 | 58.668 | 1.00 | 25.39 | C |
| ATOM | 5877 | CG | LEU | D | 124 | 19.685 | 44.572 | 59.072 | 1.00 | 25.87 | C |
| ATOM | 5878 | CD1 | LEU | D | 124 | 20.476 | 43.704 | 58.079 | 1.00 | 26.16 | C |
| ATOM | 5879 | CD2 | LEU | D | 124 | 20.285 | 45.941 | 59.235 | 1.00 | 26.85 | C |
| ATOM | 5880 | N | ALA | D | 125 | 15.236 | 46.544 | 59.412 | 1.00 | 28.75 | N |
| ATOM | 5881 | CA | ALA | D | 125 | 13.838 | 46.651 | 58.982 | 1.00 | 31.20 | C |
| ATOM | 5882 | C | ALA | D | 125 | 13.777 | 47.384 | 57.644 | 1.00 | 33.21 | C |
| ATOM | 5883 | O | ALA | D | 125 | 14.608 | 48.260 | 57.368 | 1.00 | 33.64 | O |
| ATOM | 5884 | CB | ALA | D | 125 | 13.022 | 47.368 | 60.017 | 1.00 | 30.50 | C |
| ATOM | 5885 | N | PRO | D | 126 | 12.787 | 47.039 | 56.802 | 1.00 | 35.03 | N |
| ATOM | 5886 | CA | PRO | D | 126 | 12.723 | 47.665 | 55.498 | 1.00 | 36.49 | C |
| ATOM | 5887 | C | PRO | D | 126 | 12.130 | 49.089 | 55.540 | 1.00 | 37.60 | C |
| ATOM | 5888 | O | PRO | D | 126 | 12.153 | 49.766 | 56.594 | 1.00 | 39.06 | O |
| ATOM | 5889 | CB | PRO | D | 126 | 11.820 | 46.709 | 54.715 | 1.00 | 36.81 | C |
| ATOM | 5890 | CG | PRO | D | 126 | 10.931 | 46.098 | 55.722 | 1.00 | 35.92 | C |
| ATOM | 5891 | CD | PRO | D | 126 | 11.684 | 46.087 | 57.017 | 1.00 | 35.46 | C |
| ATOM | 5892 | N | SER | D | 136 | 3.705 | 41.515 | 49.478 | 1.00 | 45.55 | N |
| ATOM | 5893 | CA | SER | D | 136 | 3.458 | 40.074 | 49.416 | 1.00 | 45.48 | C |
| ATOM | 5894 | C | SER | D | 136 | 4.403 | 39.307 | 50.347 | 1.00 | 44.60 | C |
| ATOM | 5895 | O | SER | D | 136 | 3.976 | 38.798 | 51.391 | 1.00 | 44.35 | O |
| ATOM | 5896 | CB | SER | D | 136 | 3.607 | 39.580 | 47.977 | 1.00 | 46.25 | C |
| ATOM | 5897 | OG | SER | D | 136 | 3.742 | 38.168 | 47.929 | 1.00 | 48.40 | O |
| ATOM | 5898 | N | MET | D | 137 | 5.677 | 39.222 | 49.949 | 1.00 | 42.70 | N |
| ATOM | 5899 | CA | MET | D | 137 | 6.742 | 38.689 | 50.787 | 1.00 | 40.73 | C |
| ATOM | 5900 | C | MET | D | 137 | 7.648 | 39.849 | 51.178 | 1.00 | 39.16 | C |
| ATOM | 5901 | O | MET | D | 137 | 8.001 | 40.650 | 50.322 | 1.00 | 38.44 | O |
| ATOM | 5902 | CB | MET | D | 137 | 7.568 | 37.660 | 50.012 | 1.00 | 42.58 | C |
| ATOM | 5903 | CG | MET | D | 137 | 6.861 | 36.346 | 49.727 | 1.00 | 43.94 | C |
| ATOM | 5904 | SD | MET | D | 137 | 6.285 | 35.546 | 51.226 | 1.00 | 47.74 | S |
| ATOM | 5905 | CE | MET | D | 137 | 7.672 | 34.494 | 51.642 | 1.00 | 47.35 | C |

```
ATOM    5906  N    VAL D 138       8.039  39.909  52.455  1.00 37.18           N
ATOM    5907  CA   VAL D 138       8.940  40.957  52.977  1.00 36.45           C
ATOM    5908  C    VAL D 138      10.228  40.332  53.496  1.00 33.68           C
ATOM    5909  O    VAL D 138      10.203  39.253  54.076  1.00 33.60           O
ATOM    5910  CB   VAL D 138       8.259  41.843  54.078  1.00 36.21           C
ATOM    5911  CG1  VAL D 138       7.812  41.039  55.302  1.00 36.88           C
ATOM    5912  CG2  VAL D 138       9.169  42.985  54.510  1.00 36.99           C
ATOM    5913  N    THR D 139      11.349  41.011  53.261  1.00 30.50           N
ATOM    5914  CA   THR D 139      12.659  40.556  53.707  1.00 28.03           C
ATOM    5915  C    THR D 139      13.142  41.432  54.841  1.00 26.79           C
ATOM    5916  O    THR D 139      13.096  42.670  54.758  1.00 26.44           O
ATOM    5917  CB   THR D 139      13.646  40.537  52.538  1.00 28.10           C
ATOM    5918  OG1  THR D 139      13.202  39.541  51.631  1.00 28.19           O
ATOM    5919  CG2  THR D 139      15.055  40.171  52.975  1.00 27.24           C
ATOM    5920  N    LEU D 140      13.559  40.773  55.925  1.00 24.67           N
ATOM    5921  CA   LEU D 140      14.070  41.418  57.127  1.00 23.76           C
ATOM    5922  C    LEU D 140      15.444  40.870  57.344  1.00 22.63           C
ATOM    5923  O    LEU D 140      15.776  39.829  56.800  1.00 23.64           O
ATOM    5924  CB   LEU D 140      13.223  41.035  58.346  1.00 24.96           C
ATOM    5925  CG   LEU D 140      11.704  41.107  58.185  1.00 25.34           C
ATOM    5926  CD1  LEU D 140      11.074  40.375  59.358  1.00 27.29           C
ATOM    5927  CD2  LEU D 140      11.255  42.546  58.114  1.00 26.27           C
ATOM    5928  N    GLY D 141      16.222  41.524  58.193  1.00 22.84           N
ATOM    5929  CA   GLY D 141      17.545  41.018  58.538  1.00 23.24           C
ATOM    5930  C    GLY D 141      18.087  41.429  59.883  1.00 22.03           C
ATOM    5931  O    GLY D 141      17.480  42.223  60.596  1.00 21.73           O
ATOM    5932  N    CYS D 142      19.214  40.827  60.248  1.00 22.50           N
ATOM    5933  CA   CYS D 142      19.971  41.180  61.456  1.00 24.29           C
ATOM    5934  C    CYS D 142      21.434  41.327  61.032  1.00 23.05           C
ATOM    5935  O    CYS D 142      21.923  40.560  60.211  1.00 22.11           O
ATOM    5936  CB   CYS D 142      19.854  40.118  62.574  1.00 26.89           C
ATOM    5937  SG   CYS D 142      18.327  40.379  63.551  1.00 38.31           S
ATOM    5938  N    LEU D 143      22.096  42.305  61.606  1.00 22.46           N
ATOM    5939  CA   LEU D 143      23.534  42.543  61.386  1.00 22.27           C
ATOM    5940  C    LEU D 143      24.181  42.245  62.706  1.00 20.31           C
ATOM    5941  O    LEU D 143      23.816  42.838  63.725  1.00 20.90           O
ATOM    5942  CB   LEU D 143      23.757  44.003  61.013  1.00 22.59           C
ATOM    5943  CG   LEU D 143      25.183  44.467  60.731  1.00 23.70           C
ATOM    5944  CD1  LEU D 143      25.779  43.642  59.627  1.00 24.27           C
ATOM    5945  CD2  LEU D 143      25.148  45.982  60.383  1.00 24.89           C
ATOM    5946  N    VAL D 144      25.106  41.292  62.689  1.00 19.23           N
ATOM    5947  CA   VAL D 144      25.797  40.828  63.847  1.00 19.09           C
ATOM    5948  C    VAL D 144      27.246  41.250  63.670  1.00 21.15           C
ATOM    5949  O    VAL D 144      27.954  40.694  62.837  1.00 22.40           O
ATOM    5950  CB   VAL D 144      25.625  39.319  63.941  1.00 18.96           C
ATOM    5951  CG1  VAL D 144      26.308  38.774  65.178  1.00 21.38           C
ATOM    5952  CG2  VAL D 144      24.116  39.014  63.912  1.00 18.27           C
ATOM    5953  N    LYS D 145      27.664  42.221  64.463  1.00 22.57           N
ATOM    5954  CA   LYS D 145      28.875  42.964  64.192  1.00 23.60           C
ATOM    5955  C    LYS D 145      29.820  42.962  65.387  1.00 23.50           C
ATOM    5956  O    LYS D 145      29.394  43.007  66.563  1.00 21.36           O
ATOM    5957  CB   LYS D 145      28.476  44.400  63.803  1.00 25.23           C
ATOM    5958  CG   LYS D 145      29.613  45.293  63.213  1.00 25.03           C
ATOM    5959  CD   LYS D 145      29.030  46.462  62.490  1.00 25.01           C
ATOM    5960  CE   LYS D 145      30.111  47.541  62.227  1.00 28.05           C
ATOM    5961  NZ   LYS D 145      31.033  47.065  61.179  1.00 31.21           N
ATOM    5962  N    GLY D 146      31.117  42.878  65.086  1.00 22.78           N
ATOM    5963  CA   GLY D 146      32.136  43.144  66.081  1.00 21.97           C
ATOM    5964  C    GLY D 146      32.331  42.059  67.112  1.00 20.66           C
ATOM    5965  O    GLY D 146      32.596  42.351  68.251  1.00 23.40           O
ATOM    5966  N    TYR D 147      32.291  40.796  66.703  1.00 20.86           N
ATOM    5967  CA   TYR D 147      32.524  39.689  67.615  1.00 20.44           C
ATOM    5968  C    TYR D 147      33.782  38.929  67.289  1.00 21.38           C
ATOM    5969  O    TYR D 147      34.351  39.100  66.220  1.00 22.67           O
ATOM    5970  CB   TYR D 147      31.333  38.727  67.653  1.00 19.27           C
```

| ATOM | 5971 | CG | TYR D 147 | 31.038 | 37.987 | 66.380 | 1.00 16.93 | C |
|---|---|---|---|---|---|---|---|---|
| ATOM | 5972 | CD1 | TYR D 147 | 30.193 | 38.513 | 65.413 | 1.00 16.51 | C |
| ATOM | 5973 | CD2 | TYR D 147 | 31.585 | 36.740 | 66.153 | 1.00 17.59 | C |
| ATOM | 5974 | CE1 | TYR D 147 | 29.934 | 37.801 | 64.244 | 1.00 17.53 | C |
| ATOM | 5975 | CE2 | TYR D 147 | 31.340 | 36.060 | 65.004 | 1.00 16.97 | C |
| ATOM | 5976 | CZ | TYR D 147 | 30.517 | 36.577 | 64.063 | 1.00 17.33 | C |
| ATOM | 5977 | OH | TYR D 147 | 30.283 | 35.815 | 62.944 | 1.00 18.15 | O |
| ATOM | 5978 | N | PHE D 148 | 34.220 | 38.143 | 68.260 | 1.00 21.18 | N |
| ATOM | 5979 | CA | PHE D 148 | 35.410 | 37.300 | 68.154 | 1.00 22.83 | C |
| ATOM | 5980 | C | PHE D 148 | 35.434 | 36.320 | 69.321 | 1.00 22.30 | C |
| ATOM | 5981 | O | PHE D 148 | 35.020 | 36.679 | 70.400 | 1.00 23.24 | O |
| ATOM | 5982 | CB | PHE D 148 | 36.690 | 38.144 | 68.189 | 1.00 22.28 | C |
| ATOM | 5983 | CG | PHE D 148 | 37.861 | 37.440 | 67.606 | 1.00 22.65 | C |
| ATOM | 5984 | CD1 | PHE D 148 | 38.100 | 37.502 | 66.242 | 1.00 23.34 | C |
| ATOM | 5985 | CD2 | PHE D 148 | 38.687 | 36.662 | 68.391 | 1.00 22.73 | C |
| ATOM | 5986 | CE1 | PHE D 148 | 39.179 | 36.817 | 65.680 | 1.00 24.52 | C |
| ATOM | 5987 | CE2 | PHE D 148 | 39.762 | 35.975 | 67.826 | 1.00 23.67 | C |
| ATOM | 5988 | CZ | PHE D 148 | 39.996 | 36.051 | 66.474 | 1.00 21.76 | C |
| ATOM | 5989 | N | PRO D 149 | 35.886 | 35.080 | 69.106 | 1.00 23.47 | N |
| ATOM | 5990 | CA | PRO D 149 | 36.104 | 34.408 | 67.831 | 1.00 25.11 | C |
| ATOM | 5991 | C | PRO D 149 | 34.794 | 33.986 | 67.169 | 1.00 26.71 | C |
| ATOM | 5992 | O | PRO D 149 | 33.719 | 34.282 | 67.673 | 1.00 26.67 | O |
| ATOM | 5993 | CB | PRO D 149 | 36.912 | 33.157 | 68.229 | 1.00 24.67 | C |
| ATOM | 5994 | CG | PRO D 149 | 36.488 | 32.847 | 69.614 | 1.00 23.49 | C |
| ATOM | 5995 | CD | PRO D 149 | 36.257 | 34.212 | 70.237 | 1.00 25.12 | C |
| ATOM | 5996 | N | GLU D 150 | 34.909 | 33.317 | 66.032 | 1.00 28.80 | N |
| ATOM | 5997 | CA | GLU D 150 | 33.811 | 32.575 | 65.474 | 1.00 31.34 | C |
| ATOM | 5998 | C | GLU D 150 | 33.579 | 31.392 | 66.380 | 1.00 31.33 | C |
| ATOM | 5999 | O | GLU D 150 | 34.465 | 31.022 | 67.158 | 1.00 32.26 | O |
| ATOM | 6000 | CB | GLU D 150 | 34.129 | 32.107 | 64.059 | 1.00 31.15 | C |
| ATOM | 6001 | CG | GLU D 150 | 33.889 | 33.183 | 63.019 | 1.00 33.86 | C |
| ATOM | 6002 | CD | GLU D 150 | 34.202 | 32.738 | 61.610 | 1.00 34.84 | C |
| ATOM | 6003 | OE1 | GLU D 150 | 35.163 | 31.978 | 61.420 | 1.00 37.80 | O |
| ATOM | 6004 | OE2 | GLU D 150 | 33.492 | 33.160 | 60.676 | 1.00 42.07 | O |
| ATOM | 6005 | N | PRO D 151 | 32.382 | 30.794 | 66.326 | 1.00 31.47 | N |
| ATOM | 6006 | CA | PRO D 151 | 31.213 | 31.131 | 65.577 | 1.00 30.25 | C |
| ATOM | 6007 | C | PRO D 151 | 30.127 | 31.871 | 66.368 | 1.00 29.15 | C |
| ATOM | 6008 | O | PRO D 151 | 30.178 | 31.983 | 67.612 | 1.00 26.11 | O |
| ATOM | 6009 | CB | PRO D 151 | 30.689 | 29.759 | 65.211 | 1.00 30.97 | C |
| ATOM | 6010 | CG | PRO D 151 | 30.895 | 28.993 | 66.468 | 1.00 31.24 | C |
| ATOM | 6011 | CD | PRO D 151 | 32.163 | 29.538 | 67.073 | 1.00 31.76 | C |
| ATOM | 6012 | N | VAL D 152 | 29.146 | 32.361 | 65.619 | 1.00 28.34 | N |
| ATOM | 6013 | CA | VAL D 152 | 27.865 | 32.758 | 66.191 | 1.00 29.08 | C |
| ATOM | 6014 | C | VAL D 152 | 26.817 | 31.856 | 65.576 | 1.00 28.49 | C |
| ATOM | 6015 | O | VAL D 152 | 27.036 | 31.264 | 64.539 | 1.00 27.19 | O |
| ATOM | 6016 | CB | VAL D 152 | 27.494 | 34.257 | 65.970 | 1.00 29.06 | C |
| ATOM | 6017 | CG1 | VAL D 152 | 28.554 | 35.185 | 66.608 | 1.00 29.81 | C |
| ATOM | 6018 | CG2 | VAL D 152 | 27.312 | 34.568 | 64.503 | 1.00 30.88 | C |
| ATOM | 6019 | N | THR D 153 | 25.675 | 31.731 | 66.240 | 1.00 29.88 | N |
| ATOM | 6020 | CA | THR D 153 | 24.569 | 30.988 | 65.668 | 1.00 30.01 | C |
| ATOM | 6021 | C | THR D 153 | 23.405 | 31.940 | 65.569 | 1.00 28.98 | C |
| ATOM | 6022 | O | THR D 153 | 23.078 | 32.632 | 66.553 | 1.00 30.17 | O |
| ATOM | 6023 | CB | THR D 153 | 24.283 | 29.756 | 66.527 | 1.00 31.41 | C |
| ATOM | 6024 | OG1 | THR D 153 | 25.277 | 28.764 | 66.208 | 1.00 34.05 | O |
| ATOM | 6025 | CG2 | THR D 153 | 22.881 | 29.180 | 66.254 | 1.00 32.95 | C |
| ATOM | 6026 | N | VAL D 154 | 22.802 | 32.007 | 64.385 | 1.00 26.36 | N |
| ATOM | 6027 | CA | VAL D 154 | 21.646 | 32.854 | 64.175 | 1.00 25.34 | C |
| ATOM | 6028 | C | VAL D 154 | 20.400 | 31.991 | 63.901 | 1.00 24.91 | C |
| ATOM | 6029 | O | VAL D 154 | 20.438 | 30.988 | 63.190 | 1.00 24.88 | O |
| ATOM | 6030 | CB | VAL D 154 | 21.839 | 33.885 | 63.025 | 1.00 24.95 | C |
| ATOM | 6031 | CG1 | VAL D 154 | 20.652 | 34.811 | 62.924 | 1.00 25.30 | C |
| ATOM | 6032 | CG2 | VAL D 154 | 23.114 | 34.729 | 63.249 | 1.00 23.95 | C |
| ATOM | 6033 | N | THR D 155 | 19.297 | 32.431 | 64.462 | 1.00 24.13 | N |
| ATOM | 6034 | CA | THR D 155 | 18.048 | 31.728 | 64.337 | 1.00 24.73 | C |
| ATOM | 6035 | C | THR D 155 | 16.958 | 32.768 | 64.322 | 1.00 22.70 | C |

```
ATOM   6036  O    THR D 155      17.154  33.830  64.866  1.00 23.80           O
ATOM   6037  CB   THR D 155      17.990  30.728  65.497  1.00 26.04           C
ATOM   6038  OG1  THR D 155      18.344  29.429  64.995  1.00 30.46           O
ATOM   6039  CG2  THR D 155      16.669  30.716  66.180  1.00 26.98           C
ATOM   6040  N    TRP D 156      15.831  32.494  63.661  1.00 23.34           N
ATOM   6041  CA   TRP D 156      14.740  33.460  63.566  1.00 22.93           C
ATOM   6042  C    TRP D 156      13.447  32.864  64.187  1.00 24.15           C
ATOM   6043  O    TRP D 156      13.017  31.813  63.780  1.00 22.81           O
ATOM   6044  CB   TRP D 156      14.501  33.807  62.126  1.00 22.04           C
ATOM   6045  CG   TRP D 156      15.597  34.663  61.465  1.00 23.44           C
ATOM   6046  CD1  TRP D 156      16.687  34.218  60.774  1.00 23.09           C
ATOM   6047  CD2  TRP D 156      15.661  36.087  61.434  1.00 23.08           C
ATOM   6048  NE1  TRP D 156      17.410  35.276  60.295  1.00 21.90           N
ATOM   6049  CE2  TRP D 156      16.818  36.439  60.689  1.00 23.00           C
ATOM   6050  CE3  TRP D 156      14.857  37.094  61.943  1.00 22.22           C
ATOM   6051  CZ2  TRP D 156      17.184  37.750  60.458  1.00 21.96           C
ATOM   6052  CZ3  TRP D 156      15.222  38.409  61.716  1.00 23.44           C
ATOM   6053  CH2  TRP D 156      16.388  38.724  60.972  1.00 22.65           C
ATOM   6054  N    ASN D 157      12.858  33.565  65.144  1.00 24.80           N
ATOM   6055  CA   ASN D 157      11.699  33.069  65.941  1.00 26.34           C
ATOM   6056  C    ASN D 157      11.955  31.677  66.508  1.00 27.26           C
ATOM   6057  O    ASN D 157      11.146  30.758  66.338  1.00 28.26           O
ATOM   6058  CB   ASN D 157      10.392  33.128  65.130  1.00 26.10           C
ATOM   6059  CG   ASN D 157       9.793  34.516  65.068  1.00 23.43           C
ATOM   6060  OD1  ASN D 157      10.271  35.463  65.701  1.00 23.98           O
ATOM   6061  ND2  ASN D 157       8.743  34.649  64.294  1.00 23.61           N
ATOM   6062  N    SER D 158      13.085  31.587  67.207  1.00 27.01           N
ATOM   6063  CA   SER D 158      13.655  30.375  67.802  1.00 28.02           C
ATOM   6064  C    SER D 158      13.700  29.109  66.918  1.00 27.98           C
ATOM   6065  O    SER D 158      13.564  27.982  67.413  1.00 27.73           O
ATOM   6066  CB   SER D 158      12.960  30.087  69.133  1.00 28.76           C
ATOM   6067  OG   SER D 158      12.951  31.247  69.958  1.00 29.83           O
ATOM   6068  N    GLY D 159      13.901  29.302  65.624  1.00 27.51           N
ATOM   6069  CA   GLY D 159      13.962  28.213  64.669  1.00 28.36           C
ATOM   6070  C    GLY D 159      12.755  28.103  63.753  1.00 29.99           C
ATOM   6071  O    GLY D 159      12.863  27.536  62.672  1.00 30.91           O
ATOM   6072  N    SER D 160      11.595  28.647  64.139  1.00 31.09           N
ATOM   6073  CA   SER D 160      10.356  28.329  63.384  1.00 31.22           C
ATOM   6074  C    SER D 160      10.355  28.917  61.988  1.00 30.38           C
ATOM   6075  O    SER D 160       9.724  28.355  61.086  1.00 27.26           O
ATOM   6076  CB   SER D 160       9.058  28.724  64.132  1.00 31.53           C
ATOM   6077  OG   SER D 160       9.030  30.099  64.419  1.00 34.36           O
ATOM   6078  N    LEU D 161      11.038  30.049  61.810  1.00 30.19           N
ATOM   6079  CA   LEU D 161      11.351  30.539  60.477  1.00 32.04           C
ATOM   6080  C    LEU D 161      12.726  29.978  60.109  1.00 33.84           C
ATOM   6081  O    LEU D 161      13.773  30.540  60.446  1.00 35.61           O
ATOM   6082  CB   LEU D 161      11.368  32.069  60.424  1.00 32.61           C
ATOM   6083  CG   LEU D 161      10.070  32.776  60.780  1.00 32.96           C
ATOM   6084  CD1  LEU D 161      10.351  34.240  61.129  1.00 33.34           C
ATOM   6085  CD2  LEU D 161       9.027  32.654  59.649  1.00 32.90           C
ATOM   6086  N    SER D 162      12.708  28.813  59.495  1.00 34.90           N
ATOM   6087  CA   SER D 162      13.909  28.117  59.085  1.00 35.76           C
ATOM   6088  C    SER D 162      14.063  28.287  57.599  1.00 35.48           C
ATOM   6089  O    SER D 162      15.103  28.697  57.115  1.00 34.36           O
ATOM   6090  CB   SER D 162      13.793  26.626  59.406  1.00 35.89           C
ATOM   6091  OG   SER D 162      14.462  26.342  60.610  1.00 39.42           O
ATOM   6092  N    SER D 163      13.002  27.953  56.872  1.00 35.90           N
ATOM   6093  CA   SER D 163      13.012  28.070  55.428  1.00 36.52           C
ATOM   6094  C    SER D 163      13.005  29.554  55.055  1.00 36.36           C
ATOM   6095  O    SER D 163      12.467  30.387  55.769  1.00 35.92           O
ATOM   6096  CB   SER D 163      11.805  27.336  54.848  1.00 37.38           C
ATOM   6097  OG   SER D 163      10.595  27.914  55.305  1.00 37.24           O
ATOM   6098  N    GLY D 164      13.630  29.908  53.945  1.00 36.97           N
ATOM   6099  CA   GLY D 164      13.711  31.347  53.580  1.00 36.21           C
ATOM   6100  C    GLY D 164      14.660  32.192  54.439  1.00 34.84           C
```

| ATOM | 6101 | O | GLY | D | 164 | 14.627 | 33.432 | 54.380 | 1.00 | 35.34 | O |
| ATOM | 6102 | N | VAL | D | 165 | 15.521 | 31.538 | 55.218 | 1.00 | 32.22 | N |
| ATOM | 6103 | CA | VAL | D | 165 | 16.580 | 32.234 | 55.960 | 1.00 | 29.12 | C |
| ATOM | 6104 | C | VAL | D | 165 | 17.861 | 32.128 | 55.141 | 1.00 | 27.62 | C |
| ATOM | 6105 | O | VAL | D | 165 | 18.087 | 31.120 | 54.515 | 1.00 | 28.52 | O |
| ATOM | 6106 | CB | VAL | D | 165 | 16.780 | 31.629 | 57.362 | 1.00 | 28.01 | C |
| ATOM | 6107 | CG1 | VAL | D | 165 | 18.064 | 32.108 | 57.992 | 1.00 | 26.32 | C |
| ATOM | 6108 | CG2 | VAL | D | 165 | 15.586 | 31.959 | 58.289 | 1.00 | 27.93 | C |
| ATOM | 6109 | N | HIS | D | 166 | 18.671 | 33.179 | 55.106 | 1.00 | 25.53 | N |
| ATOM | 6110 | CA | HIS | D | 166 | 20.018 | 33.117 | 54.525 | 1.00 | 24.80 | C |
| ATOM | 6111 | C | HIS | D | 166 | 20.978 | 33.739 | 55.522 | 1.00 | 23.39 | C |
| ATOM | 6112 | O | HIS | D | 166 | 20.884 | 34.911 | 55.836 | 1.00 | 23.40 | O |
| ATOM | 6113 | CB | HIS | D | 166 | 20.101 | 33.875 | 53.204 | 1.00 | 25.88 | C |
| ATOM | 6114 | CG | HIS | D | 166 | 19.236 | 33.314 | 52.123 | 1.00 | 24.89 | C |
| ATOM | 6115 | ND1 | HIS | D | 166 | 19.422 | 32.054 | 51.603 | 1.00 | 28.08 | N |
| ATOM | 6116 | CD2 | HIS | D | 166 | 18.179 | 33.837 | 51.466 | 1.00 | 26.03 | C |
| ATOM | 6117 | CE1 | HIS | D | 166 | 18.527 | 31.829 | 50.658 | 1.00 | 28.20 | C |
| ATOM | 6118 | NE2 | HIS | D | 166 | 17.753 | 32.894 | 50.561 | 1.00 | 28.34 | N |
| ATOM | 6119 | N | THR | D | 167 | 21.879 | 32.950 | 56.059 | 1.00 | 23.72 | N |
| ATOM | 6120 | CA | THR | D | 167 | 22.838 | 33.462 | 57.036 | 1.00 | 24.84 | C |
| ATOM | 6121 | C | THR | D | 167 | 24.180 | 33.436 | 56.335 | 1.00 | 25.47 | C |
| ATOM | 6122 | O | THR | D | 167 | 24.587 | 32.416 | 55.807 | 1.00 | 26.93 | O |
| ATOM | 6123 | CB | THR | D | 167 | 22.792 | 32.670 | 58.329 | 1.00 | 25.77 | C |
| ATOM | 6124 | OG1 | THR | D | 167 | 21.496 | 32.865 | 58.955 | 1.00 | 26.26 | O |
| ATOM | 6125 | CG2 | THR | D | 167 | 23.876 | 33.149 | 59.254 | 1.00 | 26.25 | C |
| ATOM | 6126 | N | PHE | D | 168 | 24.803 | 34.599 | 56.244 | 1.00 | 26.44 | N |
| ATOM | 6127 | CA | PHE | D | 168 | 25.937 | 34.802 | 55.360 | 1.00 | 25.37 | C |
| ATOM | 6128 | C | PHE | D | 168 | 27.249 | 34.548 | 56.094 | 1.00 | 26.68 | C |
| ATOM | 6129 | O | PHE | D | 168 | 27.343 | 34.758 | 57.317 | 1.00 | 25.89 | O |
| ATOM | 6130 | CB | PHE | D | 168 | 25.873 | 36.205 | 54.791 | 1.00 | 25.88 | C |
| ATOM | 6131 | CG | PHE | D | 168 | 24.717 | 36.402 | 53.864 | 1.00 | 25.93 | C |
| ATOM | 6132 | CD1 | PHE | D | 168 | 23.514 | 36.946 | 54.317 | 1.00 | 24.94 | C |
| ATOM | 6133 | CD2 | PHE | D | 168 | 24.806 | 35.989 | 52.545 | 1.00 | 25.29 | C |
| ATOM | 6134 | CE1 | PHE | D | 168 | 22.446 | 37.077 | 53.462 | 1.00 | 24.09 | C |
| ATOM | 6135 | CE2 | PHE | D | 168 | 23.742 | 36.135 | 51.692 | 1.00 | 24.50 | C |
| ATOM | 6136 | CZ | PHE | D | 168 | 22.553 | 36.662 | 52.157 | 1.00 | 24.37 | C |
| ATOM | 6137 | N | PRO | D | 169 | 28.264 | 34.046 | 55.374 | 1.00 | 26.26 | N |
| ATOM | 6138 | CA | PRO | D | 169 | 29.529 | 33.830 | 56.069 | 1.00 | 26.57 | C |
| ATOM | 6139 | C | PRO | D | 169 | 30.082 | 35.074 | 56.749 | 1.00 | 24.14 | C |
| ATOM | 6140 | O | PRO | D | 169 | 29.919 | 36.151 | 56.226 | 1.00 | 26.02 | O |
| ATOM | 6141 | CB | PRO | D | 169 | 30.465 | 33.360 | 54.942 | 1.00 | 27.49 | C |
| ATOM | 6142 | CG | PRO | D | 169 | 29.577 | 32.758 | 53.949 | 1.00 | 26.75 | C |
| ATOM | 6143 | CD | PRO | D | 169 | 28.323 | 33.589 | 53.977 | 1.00 | 27.05 | C |
| ATOM | 6144 | N | ALA | D | 170 | 30.747 | 34.922 | 57.891 | 1.00 | 23.63 | N |
| ATOM | 6145 | CA | ALA | D | 170 | 31.298 | 36.071 | 58.605 | 1.00 | 23.66 | C |
| ATOM | 6146 | C | ALA | D | 170 | 32.537 | 36.524 | 57.876 | 1.00 | 24.99 | C |
| ATOM | 6147 | O | ALA | D | 170 | 33.238 | 35.691 | 57.337 | 1.00 | 24.97 | O |
| ATOM | 6148 | CB | ALA | D | 170 | 31.676 | 35.713 | 59.982 | 1.00 | 24.55 | C |
| ATOM | 6149 | N | VAL | D | 171 | 32.800 | 37.820 | 57.900 | 1.00 | 26.28 | N |
| ATOM | 6150 | CA | VAL | D | 171 | 34.045 | 38.375 | 57.374 | 1.00 | 29.14 | C |
| ATOM | 6151 | C | VAL | D | 171 | 34.763 | 39.182 | 58.476 | 1.00 | 30.45 | C |
| ATOM | 6152 | O | VAL | D | 171 | 34.149 | 39.907 | 59.265 | 1.00 | 26.39 | O |
| ATOM | 6153 | CB | VAL | D | 171 | 33.786 | 39.222 | 56.136 | 1.00 | 29.36 | C |
| ATOM | 6154 | CG1 | VAL | D | 171 | 35.118 | 39.682 | 55.504 | 1.00 | 30.70 | C |
| ATOM | 6155 | CG2 | VAL | D | 171 | 32.968 | 38.390 | 55.126 | 1.00 | 30.35 | C |
| ATOM | 6156 | N | LEU | D | 172 | 36.078 | 38.997 | 58.522 | 1.00 | 34.29 | N |
| ATOM | 6157 | CA | LEU | D | 172 | 36.946 | 39.693 | 59.442 | 1.00 | 37.93 | C |
| ATOM | 6158 | C | LEU | D | 172 | 37.136 | 41.153 | 59.010 | 1.00 | 40.77 | C |
| ATOM | 6159 | O | LEU | D | 172 | 37.728 | 41.429 | 57.970 | 1.00 | 43.78 | O |
| ATOM | 6160 | CB | LEU | D | 172 | 38.281 | 38.959 | 59.497 | 1.00 | 38.35 | C |
| ATOM | 6161 | CG | LEU | D | 172 | 39.179 | 39.328 | 60.675 | 1.00 | 39.75 | C |
| ATOM | 6162 | CD1 | LEU | D | 172 | 38.566 | 38.837 | 61.983 | 1.00 | 39.31 | C |
| ATOM | 6163 | CD2 | LEU | D | 172 | 40.577 | 38.770 | 60.468 | 1.00 | 39.06 | C |
| ATOM | 6164 | N | GLN | D | 173 | 36.614 | 42.081 | 59.799 | 1.00 | 43.18 | N |
| ATOM | 6165 | CA | GLN | D | 173 | 36.718 | 43.510 | 59.520 | 1.00 | 44.71 | C |

| ATOM | 6166 | C   | GLN | D | 173 | 37.164 | 44.238 | 60.785 | 1.00 | 44.57 | C |
| ATOM | 6167 | O   | GLN | D | 173 | 36.485 | 44.184 | 61.806 | 1.00 | 44.28 | O |
| ATOM | 6168 | CB  | GLN | D | 173 | 35.357 | 44.050 | 59.054 | 1.00 | 45.24 | C |
| ATOM | 6169 | CG  | GLN | D | 173 | 35.138 | 45.579 | 59.122 | 1.00 | 45.63 | C |
| ATOM | 6170 | CD  | GLN | D | 173 | 33.720 | 45.987 | 58.690 | 1.00 | 47.21 | C |
| ATOM | 6171 | OE1 | GLN | D | 173 | 33.543 | 46.686 | 57.685 | 1.00 | 50.58 | O |
| ATOM | 6172 | NE2 | GLN | D | 173 | 32.705 | 45.536 | 59.443 | 1.00 | 48.38 | N |
| ATOM | 6173 | N   | SER | D | 174 | 38.288 | 44.945 | 60.693 | 1.00 | 44.64 | N |
| ATOM | 6174 | CA  | SER | D | 174 | 38.830 | 45.733 | 61.814 | 1.00 | 43.83 | C |
| ATOM | 6175 | C   | SER | D | 174 | 39.155 | 44.800 | 62.983 | 1.00 | 41.40 | C |
| ATOM | 6176 | O   | SER | D | 174 | 38.946 | 45.131 | 64.171 | 1.00 | 42.07 | O |
| ATOM | 6177 | CB  | SER | D | 174 | 37.893 | 46.893 | 62.214 | 1.00 | 44.67 | C |
| ATOM | 6178 | OG  | SER | D | 174 | 38.245 | 48.100 | 61.536 | 1.00 | 47.11˙ | O |
| ATOM | 6179 | N   | ASP | D | 175 | 39.685 | 43.637 | 62.601 | 1.00 | 37.20 | N |
| ATOM | 6180 | CA  | ASP | D | 175 | 40.135 | 42.613 | 63.509 | 1.00 | 35.59 | C |
| ATOM | 6181 | C   | ASP | D | 175 | 38.993 | 41.955 | 64.329 | 1.00 | 34.10 | C |
| ATOM | 6182 | O   | ASP | D | 175 | 39.246 | 41.293 | 65.339 | 1.00 | 32.18 | O |
| ATOM | 6183 | CB  | ASP | D | 175 | 41.252 | 43.184 | 64.386 | 1.00 | 37.10 | C |
| ATOM | 6184 | CG  | ASP | D | 175 | 42.322 | 43.938 | 63.553 | 1.00 | 39.01 | C |
| ATOM | 6185 | OD1 | ASP | D | 175 | 43.450 | 43.417 | 63.451 | 1.00 | 39.86 | O |
| ATOM | 6186 | OD2 | ASP | D | 175 | 42.022 | 45.031 | 62.981 | 1.00 | 39.96 | O |
| ATOM | 6187 | N   | LEU | D | 176 | 37.754 | 42.121 | 63.854 | 1.00 | 31.81 | N |
| ATOM | 6188 | CA  | LEU | D | 176 | 36.567 | 41.471 | 64.428 | 1.00 | 32.07 | C |
| ATOM | 6189 | C   | LEU | D | 176 | 35.723 | 40.898 | 63.299 | 1.00 | 30.64 | C |
| ATOM | 6190 | O   | LEU | D | 176 | 35.881 | 41.291 | 62.130 | 1.00 | 28.75 | O |
| ATOM | 6191 | CB  | LEU | D | 176 | 35.735 | 42.506 | 65.176 | 1.00 | 32.41 | C |
| ATOM | 6192 | CG  | LEU | D | 176 | 36.433 | 43.178 | 66.353 | 1.00 | 32.89 | C |
| ATOM | 6193 | CD1 | LEU | D | 176 | 35.818 | 44.513 | 66.632 | 1.00 | 34.22 | C |
| ATOM | 6194 | CD2 | LEU | D | 176 | 36.402 | 42.295 | 67.574 | 1.00 | 34.00 | C |
| ATOM | 6195 | N   | TYR | D | 177 | 34.793 | 40.013 | 63.650 | 1.00 | 29.32 | N |
| ATOM | 6196 | CA  | TYR | D | 177 | 33.912 | 39.374 | 62.654 | 1.00 | 27.72 | C |
| ATOM | 6197 | C   | TYR | D | 177 | 32.578 | 40.088 | 62.523 | 1.00 | 26.64 | C |
| ATOM | 6198 | O   | TYR | D | 177 | 32.045 | 40.643 | 63.491 | 1.00 | 25.94 | O |
| ATOM | 6199 | CB  | TYR | D | 177 | 33.668 | 37.910 | 62.989 | 1.00 | 28.31 | C |
| ATOM | 6200 | CG  | TYR | D | 177 | 34.803 | 37.019 | 62.644 | 1.00 | 29.12 | C |
| ATOM | 6201 | CD1 | TYR | D | 177 | 35.087 | 36.713 | 61.319 | 1.00 | 27.89 | C |
| ATOM | 6202 | CD2 | TYR | D | 177 | 35.609 | 36.478 | 63.640 | 1.00 | 29.07 | C |
| ATOM | 6203 | CE1 | TYR | D | 177 | 36.134 | 35.891 | 61.001 | 1.00 | 29.47 | C |
| ATOM | 6204 | CE2 | TYR | D | 177 | 36.649 | 35.668 | 63.332 | 1.00 | 30.32 | C |
| ATOM | 6205 | CZ  | TYR | D | 177 | 36.908 | 35.363 | 62.016 | 1.00 | 29.98 | C |
| ATOM | 6206 | OH  | TYR | D | 177 | 37.979 | 34.551 | 61.728 | 1.00 | 31.20 | O |
| ATOM | 6207 | N   | THR | D | 178 | 32.058 | 40.103 | 61.305 | 1.00 | 25.18 | N |
| ATOM | 6208 | CA  | THR | D | 178 | 30.708 | 40.616 | 61.067 | 1.00 | 25.47 | C |
| ATOM | 6209 | C   | THR | D | 178 | 29.951 | 39.666 | 60.177 | 1.00 | 24.14 | C |
| ATOM | 6210 | O   | THR | D | 178 | 30.470 | 39.182 | 59.196 | 1.00 | 21.75 | O |
| ATOM | 6211 | CB  | THR | D | 178 | 30.713 | 42.018 | 60.387 | 1.00 | 25.77 | C |
| ATOM | 6212 | OG1 | THR | D | 178 | 31.409 | 42.945 | 61.223 | 1.00 | 25.21 | O |
| ATOM | 6213 | CG2 | THR | D | 178 | 29.287 | 42.519 | 60.186 | 1.00 | 26.05 | C |
| ATOM | 6214 | N   | LEU | D | 179 | 28.707 | 39.395 | 60.513 | 1.00 | 23.52 | N |
| ATOM | 6215 | CA  | LEU | D | 179 | 27.864 | 38.722 | 59.557 | 1.00 | 23.69 | C |
| ATOM | 6216 | C   | LEU | D | 179 | 26.500 | 39.338 | 59.530 | 1.00 | 23.15 | C |
| ATOM | 6217 | O   | LEU | D | 179 | 26.161 | 40.150 | 60.393 | 1.00 | 21.79 | O |
| ATOM | 6218 | CB  | LEU | D | 179 | 27.823 | 37.220 | 59.818 | 1.00 | 24.18 | C |
| ATOM | 6219 | CG  | LEU | D | 179 | 27.155 | 36.580 | 61.026 | 1.00 | 23.84 | C |
| ATOM | 6220 | CD1 | LEU | D | 179 | 25.649 | 36.813 | 61.107 | 1.00 | 25.66 | C |
| ATOM | 6221 | CD2 | LEU | D | 179 | 27.453 | 35.085 | 60.932 | 1.00 | 24.23 | C |
| ATOM | 6222 | N   | SER | D | 180 | 25.738 | 38.972 | 58.507 | 1.00 | 24.38 | N |
| ATOM | 6223 | CA  | SER | D | 180 | 24.335 | 39.313 | 58.449 | 1.00 | 24.85 | C |
| ATOM | 6224 | C   | SER | D | 180 | 23.492 | 38.072 | 58.130 | 1.00 | 23.04 | C |
| ATOM | 6225 | O   | SER | D | 180 | 23.985 | 37.027 | 57.704 | 1.00 | 21.36 | O |
| ATOM | 6226 | CB  | SER | D | 180 | 24.106 | 40.414 | 57.412 | 1.00 | 25.17 | C |
| ATOM | 6227 | OG  | SER | D | 180 | 24.532 | 39.905 | 56.178 | 1.00 | 28.19 | O |
| ATOM | 6228 | N   | SER | D | 181 | 22.201 | 38.204 | 58.372 | 1.00 | 23.39 | N |
| ATOM | 6229 | CA  | SER | D | 181 | 21.254 | 37.132 | 58.104 | 1.00 | 21.86 | C |
| ATOM | 6230 | C   | SER | D | 181 | 20.025 | 37.781 | 57.543 | 1.00 | 19.68 | C |

| ATOM | 6231 | O | SER | D | 181 | .19.659 | 38.820 | 58.001 | 1.00 | 19.67 | O |
| ATOM | 6232 | CB | SER | D | 181 | 20.945 | 36.333 | 59.397 | 1.00 | 23.75 | C |
| ATOM | 6233 | OG | SER | D | 181 | 20.024 | 35.266 | 59.137 | 1.00 | 21.14 | O |
| ATOM | 6234 | N | SER | D | 182 | 19.404 | 37.191 | 56.530 | 1.00 | 20.09 | N |
| ATOM | 6235 | CA | SER | D | 182 | 18.136 | 37.666 | 56.039 | 1.00 | 20.90 | C |
| ATOM | 6236 | C | SER | D | 182 | 17.057 | 36.616 | 56.279 | 1.00 | 20.74 | C |
| ATOM | 6237 | O | SER | D | 182 | 17.346 | 35.425 | 56.386 | 1.00 | 20.36 | O |
| ATOM | 6238 | CB | SER | D | 182 | 18.200 | 37.969 | 54.549 | 1.00 | 22.00 | C |
| ATOM | 6239 | OG | SER | D | 182 | 18.563 | 36.822 | 53.827 | 1.00 | 24.22 | O |
| ATOM | 6240 | N | VAL | D | 183 | 15.821 | 37.069 | 56.338 | 1.00 | 22.22 | N |
| ATOM | 6241 | CA | VAL | D | 183 | 14.663 | 36.155 | 56.384 | 1.00 | 23.25 | C |
| ATOM | 6242 | C | VAL | D | 183 | 13.515 | 36.792 | 55.589 | 1.00 | 25.87 | C |
| ATOM | 6243 | O | VAL | D | 183 | 13.289 | 37.990 | 55.675 | 1.00 | 26.67 | O |
| ATOM | 6244 | CB | VAL | D | 183 | 14.232 | 35.823 | 57.834 | 1.00 | 23.04 | C |
| ATOM | 6245 | CG1 | VAL | D | 183 | 13.687 | 37.059 | 58.554 | 1.00 | 23.82 | C |
| ATOM | 6246 | CG2 | VAL | D | 183 | 13.160 | 34.685 | 57.863 | 1.00 | 23.28 | C |
| ATOM | 6247 | N | THR | D | 184 | 12.837 | 35.989 | 54.776 | 1.00 | 27.65 | N |
| ATOM | 6248 | CA | THR | D | 184 | 11.670 | 36.416 | 54.038 | 1.00 | 30.08 | C |
| ATOM | 6249 | C | THR | D | 184 | 10.411 | 35.829 | 54.690 | 1.00 | 30.25 | C |
| ATOM | 6250 | O | THR | D | 184 | 10.342 | 34.627 | 54.941 | 1.00 | 29.42 | O |
| ATOM | 6251 | CB | THR | D | 184 | 11.796 | 36.020 | 52.548 | 1.00 | 31.38 | C |
| ATOM | 6252 | OG1 | THR | D | 184 | 13.105 | 36.387 | 52.065 | 1.00 | 35.31 | O |
| ATOM | 6253 | CG2 | THR | D | 184 | 10.792 | 36.741 | 51.703 | 1.00 | 32.12 | C |
| ATOM | 6254 | N | VAL | D | 185 | 9.430 | 36.688 | 54.970 | 1.00 | 31.04 | N |
| ATOM | 6255 | CA | VAL | D | 185 | 8.167 | 36.266 | 55.582 | 1.00 | 32.75 | C |
| ATOM | 6256 | C | VAL | D | 185 | 6.980 | 36.872 | 54.840 | 1.00 | 34.23 | C |
| ATOM | 6257 | O | VAL | D | 185 | 7.145 | 37.857 | 54.122 | 1.00 | 35.61 | O |
| ATOM | 6258 | CB | VAL | D | 185 | 8.089 | 36.661 | 57.063 | 1.00 | 32.76 | C |
| ATOM | 6259 | CG1 | VAL | D | 185 | 9.254 | 36.051 | 57.839 | 1.00 | 33.49 | C |
| ATOM | 6260 | CG2 | VAL | D | 185 | 8.059 | 38.192 | 57.255 | 1.00 | 33.67 | C |
| ATOM | 6261 | N | PRO | D | 186 | 5.771 | 36.285 | 54.992 | 1.00 | 34.51 | N |
| ATOM | 6262 | CA | PRO | D | 186 | 4.620 | 36.964 | 54.398 | 1.00 | 33.96 | C |
| ATOM | 6263 | C | PRO | D | 186 | 4.385 | 38.349 | 55.006 | 1.00 | 33.86 | C |
| ATOM | 6264 | O | PRO | D | 186 | 4.602 | 38.559 | 56.194 | 1.00 | 34.38 | O |
| ATOM | 6265 | CB | PRO | D | 186 | 3.454 | 36.000 | 54.685 | 1.00 | 34.69 | C |
| ATOM | 6266 | CG | PRO | D | 186 | 4.113 | 34.665 | 54.903 | 1.00 | 34.59 | C |
| ATOM | 6267 | CD | PRO | D | 186 | 5.384 | 35.015 | 55.630 | 1.00 | 34.55 | C |
| ATOM | 6268 | N | SER | D | 187 | 3.978 | 39.304 | 54.180 | 1.00 | 34.96 | N |
| ATOM | 6269 | CA | SER | D | 187 | 3.590 | 40.621 | 54.663 | 1.00 | 35.58 | C |
| ATOM | 6270 | C | SER | D | 187 | 2.352 | 40.547 | 55.550 | 1.00 | 34.96 | C |
| ATOM | 6271 | O | SER | D | 187 | 2.093 | 41.451 | 56.340 | 1.00 | 33.97 | O |
| ATOM | 6272 | CB | SER | D | 187 | 3.322 | 41.557 | 53.489 | 1.00 | 36.91 | C |
| ATOM | 6273 | OG | SER | D | 187 | 4.519 | 41.769 | 52.746 | 1.00 | 40.54 | O |
| ATOM | 6274 | N | SER | D | 188 | 1.583 | 39.475 | 55.417 | 1.00 | 35.02 | N |
| ATOM | 6275 | CA | SER | D | 188 | 0.429 | 39.270 | 56.279 | 1.00 | 35.53 | C |
| ATOM | 6276 | C | SER | D | 188 | 0.854 | 38.948 | 57.713 | 1.00 | 35.31 | C |
| ATOM | 6277 | O | SER | D | 188 | 0.112 | 39.221 | 58.663 | 1.00 | 36.23 | O |
| ATOM | 6278 | CB | SER | D | 188 | -0.467 | 38.161 | 55.715 | 1.00 | 36.15 | C |
| ATOM | 6279 | OG | SER | D | 188 | 0.253 | 36.971 | 55.462 | 1.00 | 36.41 | O |
| ATOM | 6280 | N | THR | D | 189 | 2.057 | 38.385 | 57.866 | 1.00 | 34.19 | N |
| ATOM | 6281 | CA | THR | D | 189 | 2.569 | 37.966 | 59.172 | 1.00 | 33.42 | C |
| ATOM | 6282 | C | THR | D | 189 | 3.341 | 39.075 | 59.878 | 1.00 | 32.21 | C |
| ATOM | 6283 | O | THR | D | 189 | 3.202 | 39.246 | 61.095 | 1.00 | 31.64 | O |
| ATOM | 6284 | CB | THR | D | 189 | 3.470 | 36.743 | 59.028 | 1.00 | 33.87 | C |
| ATOM | 6285 | OG1 | THR | D | 189 | 2.689 | 35.645 | 58.555 | 1.00 | 35.05 | O |
| ATOM | 6286 | CG2 | THR | D | 189 | 4.124 | 36.378 | 60.345 | 1.00 | 35.00 | C |
| ATOM | 6287 | N | TRP | D | 190 | 4.144 | 39.818 | 59.111 | 1.00 | 30.38 | N |
| ATOM | 6288 | CA | TRP | D | 190 | 4.943 | 40.924 | 59.652 | 1.00 | 30.06 | C |
| ATOM | 6289 | C | TRP | D | 190 | 4.498 | 42.227 | 59.005 | 1.00 | 29.75 | C |
| ATOM | 6290 | O | TRP | D | 190 | 4.257 | 42.259 | 57.803 | 1.00 | 29.61 | O |
| ATOM | 6291 | CB | TRP | D | 190 | 6.439 | 40.694 | 59.386 | 1.00 | 29.33 | C |
| ATOM | 6292 | CG | TRP | D | 190 | 7.290 | 41.653 | 60.085 | 1.00 | 29.07 | C |
| ATOM | 6293 | CD1 | TRP | D | 190 | 7.798 | 41.532 | 61.329 | 1.00 | 28.74 | C |
| ATOM | 6294 | CD2 | TRP | D | 190 | 7.742 | 42.917 | 59.582 | 1.00 | 30.04 | C |
| ATOM | 6295 | NE1 | TRP | D | 190 | 8.529 | 42.652 | 61.654 | 1.00 | 29.49 | N |

| ATOM | 6296 | CE2 | TRP | D | 190 | 8.523 | 43.510 | 60.590 | 1.00 | 29.18 | C |
|------|------|-----|-----|---|-----|-------|--------|--------|------|-------|---|
| ATOM | 6297 | CE3 | TRP | D | 190 | 7.552 | 43.608 | 58.378 | 1.00 | 29.58 | C |
| ATOM | 6298 | CZ2 | TRP | D | 190 | 9.115 | 44.767 | 60.437 | 1.00 | 30.46 | C |
| ATOM | 6299 | CZ3 | TRP | D | 190 | 8.134 | 44.869 | 58.229 | 1.00 | 29.90 | C |
| ATOM | 6300 | CH2 | TRP | D | 190 | 8.913 | 45.425 | 59.247 | 1.00 | 28.92 | C |
| ATOM | 6301 | N | PRO | D | 191 | 4.385 | 43.308 | 59.788 | 1.00 | 29.35 | N |
| ATOM | 6302 | CA | PRO | D | 191 | 4.613 | 43.460 | 61.208 | 1.00 | 30.19 | C |
| ATOM | 6303 | C | PRO | D | 191 | 3.464 | 43.113 | 62.154 | 1.00 | 29.31 | C |
| ATOM | 6304 | O | PRO | D | 191 | 3.645 | 43.274 | 63.345 | 1.00 | 30.17 | O |
| ATOM | 6305 | CB | PRO | D | 191 | 4.928 | 44.951 | 61.343 | 1.00 | 29.75 | C |
| ATOM | 6306 | CG | PRO | D | 191 | 4.197 | 45.579 | 60.288 | 1.00 | 30.04 | C |
| ATOM | 6307 | CD | PRO | D | 191 | 4.035 | 44.594 | 59.173 | 1.00 | 30.48 | C |
| ATOM | 6308 | N | SER | D | 192 | 2.330 | 42.620 | 61.668 | 1.00 | 30.73 | N |
| ATOM | 6309 | CA | SER | D | 192 | 1.208 | 42.265 | 62.578 | 1.00 | 32.14 | C |
| ATOM | 6310 | C | SER | D | 192 | 1.596 | 41.304 | 63.723 | 1.00 | 32.19 | C |
| ATOM | 6311 | O | SER | D | 192 | 1.033 | 41.377 | 64.838 | 1.00 | 33.71 | O |
| ATOM | 6312 | CB | SER | D | 192 | 0.025 | 41.700 | 61.790 | 1.00 | 32.80 | C |
| ATOM | 6313 | OG | SER | D | 192 | 0.451 | 40.666 | 60.923 | 1.00 | 34.94 | O |
| ATOM | 6314 | N | GLU | D | 193 | 2.531 | 40.397 | 63.452 | 1.00 | 31.50 | N |
| ATOM | 6315 | CA | GLU | D | 193 | 3.091 | 39.517 | 64.488 | 1.00 | 32.08 | C |
| ATOM | 6316 | C | GLU | D | 193 | 4.620 | 39.645 | 64.466 | 1.00 | 31.03 | C |
| ATOM | 6317 | O | GLU | D | 193 | 5.179 | 40.117 | 63.487 | 1.00 | 30.09 | O |
| ATOM. | 6318 | CB | GLU | D | 193 | 2.656 | 38.051 | 64.281 | 1.00 | 32.11 | C |
| ATOM | 6319 | CG | GLU | D | 193 | 1.126 | 37.815 | 64.307 | 1.00 | 33.51 | C |
| ATOM | 6320 | CD | GLU | D | 193 | 0.470 | 38.080 | 65.649 | 1.00 | 35.03 | C |
| ATOM | 6321 | OE1 | GLU | D | 193 | -0.790 | 38.157 | 65.682 | 1.00 | 36.73 | O |
| ATOM | 6322 | OE2 | GLU | D | 193 | 1.191 | 38.235 | 66.669 | 1.00 | 34.86 | O |
| ATOM | 6323 | N | THR | D | 194 | 5.258 | 39.215 | 65.550 | 1.00 | 30.26 | N |
| ATOM | 6324 | CA | THR | D | 194 | 6.675 | 39.523 | 65.835 | 1.00 | 30.82 | C |
| ATOM | 6325 | C | THR | D | 194 | 7.659 | 38.681 | 64.987 | 1.00 | 28.54 | C |
| ATOM | 6326 | O | THR | D | 194 | 7.428 | 37.499 | 64.708 | 1.00 | 24.94 | O |
| ATOM | 6327 | CB | THR | D | 194 | 6.954 | 39.339 | 67.384 | 1.00 | 31.07 | C |
| ATOM | 6328 | OG1 | THR | D | 194 | 6.196 | 40.301 | 68.125 | 1.00 | 36.10 | O |
| ATOM | 6329 | CG2 | THR | D | 194 | 8.370 | 39.583 | 67.765 | 1.00 | 34.74 | C |
| ATOM | 6330 | N | VAL | D | 195 | 8.765 | 39.301 | 64.574 | 1.00 | 27.18 | N |
| ATOM | 6331 | CA | VAL | D | 195 | 9.871 | 38.551 | 63.996 | 1.00 | 25.44 | C |
| ATOM | 6332 | C | VAL | D | 195 | 11.145 | 38.983 | 64.707 | 1.00 | 24.93 | C |
| ATOM | 6333 | O | VAL | D | 195 | 11.418 | 40.182 | 64.838 | 1.00 | 24.29 | O |
| ATOM | 6334 | CB | VAL | D | 195 | 9.934 | 38.723 | 62.482 | 1.00 | 26.85 | C |
| ATOM | 6335 | CG1 | VAL | D | 195 | 11.234 | 38.108 | 61.892 | 1.00 | 24.51 | C |
| ATOM | 6336 | CG2 | VAL | D | 195 | 8.715 | 38.074 | 61.865 | 1.00 | 26.47 | C |
| ATOM | 6337 | N | THR | D | 196 | 11.865 | 37.999 | 65.226 | 1.00 | 24.99 | N |
| ATOM | 6338 | CA | THR | D | 196 | 12.972 | 38.208 | 66.140 | 1.00 | 26.56 | C |
| ATOM | 6339 | C | THR | D | 196 | 14.135 | 37.327 | 65.704 | 1.00 | 26.84 | C |
| ATOM | 6340 | O | THR | D | 196 | 13.958 | 36.142 | 65.444 | 1.00 | 26.17 | O |
| ATOM | 6341 | CB | THR | D | 196 | 12.585 | 37.789 | 67.574 | 1.00 | 27.32 | C |
| ATOM | 6342 | OG1 | THR | D | 196 | 11.540 | 38.631 | 68.057 | 1.00 | 28.70 | O |
| ATOM | 6343 | CG2 | THR | D | 196 | 13.760 | 37.900 | 68.519 | 1.00 | 28.94 | C |
| ATOM | 6344 | N | CYS | D | 197 | 15.334 | 37.886 | 65.639 | 1.00 | 26.26 | N |
| ATOM | 6345 | CA | CYS | D | 197 | 16.485 | 37.064 | 65.351 | 1.00 | 25.53 | C |
| ATOM | 6346 | C | CYS | D | 197 | 17.183 | 36.832 | 66.646 | 1.00 | 23.77 | C |
| ATOM | 6347 | O | CYS | D | 197 | 17.323 | 37.741 | 67.471 | 1.00 | 23.60 | O |
| ATOM | 6348 | CB | CYS | D | 197 | 17.400 | 37.715 | 64.351 | 1.00 | 27.79 | C |
| ATOM | 6349 | SG | CYS | D | 197 | 18.379 | 38.983 | 65.035 | 1.00 | 33.57 | S |
| ATOM | 6350 | N | ASN | D | 198 | 17.578 | 35.584 | 66.852 | 1.00 | 22.02 | N |
| ATOM | 6351 | CA | ASN | D | 198 | 18.183 | 35.161 | 68.074 | 1.00 | 22.55 | C |
| ATOM | 6352 | C | ASN | D | 198 | 19.629 | 34.868 | 67.758 | 1.00 | 21.17 | C |
| ATOM | 6353 | O | ASN | D | 198 | 19.914 | 34.091 | 66.867 | 1.00 | 22.01 | O |
| ATOM | 6354 | CB | ASN | D | 198 | 17.477 | 33.893 | 68.561 | 1.00 | 23.97 | C |
| ATOM | 6355 | CG | ASN | D | 198 | 15.995 | 33.928 | 68.270 | 1.00 | 24.16 | C |
| ATOM | 6356 | OD1 | ASN | D | 198 | 15.534 | 33.307 | 67.318 | 1.00 | 24.07 | O |
| ATOM | 6357 | ND2 | ASN | D | 198 | 15.262 | 34.744 | 69.021 | 1.00 | 23.41 | N |
| ATOM | 6358 | N | VAL | D | 199 | 20.539 | 35.463 | 68.494 | 1.00 | 21.25 | N |
| ATOM | 6359 | CA | VAL | D | 199 | 21.976 | 35.302 | 68.191 | 1.00 | 21.48 | C |
| ATOM | 6360 | C | VAL | D | 199 | 22.672 | 34.758 | 69.413 | 1.00 | 22.46 | C |

| ATOM | 6361 | O | VAL D 199 | 22.561 | 35.326 | 70.506 | 1.00 24.86 | O |
|------|------|------|------|------|------|------|------|------|
| ATOM | 6362 | CB | VAL D 199 | 22.608 | 36.633 | 67.719 | 1.00 20.23 | C |
| ATOM | 6363 | CG1 | VAL D 199 | 24.108 | 36.442 | 67.382 | 1.00 21.37 | C |
| ATOM | 6364 | CG2 | VAL D 199 | 21.862 | 37.157 | 66.524 | 1.00 20.58 | C |
| ATOM | 6365 | N | ALA D 200 | 23.351 | 33.631 | 69.234 | 1.00 23.47 | N |
| ATOM | 6366 | CA | ALA D 200 | 24.152 | 33.040 | 70.280 | 1.00 25.01 | C |
| ATOM | 6367 | C | ALA D 200 | 25.625 | 33.174 | 69.901 | 1.00 25.13 | C |
| ATOM | 6368 | O | ALA D 200 | 25.996 | 32.920 | 68.746 | 1.00 26.38 | O |
| ATOM | 6369 | CB | ALA D 200 | 23.796 | 31.571 | 70.486 | 1.00 24.99 | C |
| ATOM | 6370 | N | HIS D 201 | 26.420 | 33.615 | 70.874 | 1.00 25.17 | N |
| ATOM | 6371 | CA | HIS D 201 | 27.885 | 33.575 | 70.803 | 1.00 26.96 | C |
| ATOM | 6372 | C | HIS D 201 | 28.425 | 32.838 | 72.024 | 1.00 27.57 | C |
| ATOM | 6373 | O | HIS D 201 | 28.618 | 33.448 | 73.071 | 1.00 25.77 | O |
| ATOM | 6374 | CB | HIS D 201 | 28.451 | 34.986 | 70.766 | 1.00 26.33 | C |
| ATOM | 6375 | CG | HIS D 201 | 29.909 | 35.039 | 70.446 | 1.00 24.65 | C |
| ATOM | 6376 | ND1 | HIS D 201 | 30.812 | 35.707 | 71.234 | 1.00 24.40 | N |
| ATOM | 6377 | CD2 | HIS D 201 | 30.612 | 34.524 | 69.418 | 1.00 25.09 | C |
| ATOM | 6378 | CE1 | HIS D 201 | 32.015 | 35.606 | 70.695 | 1.00 27.01 | C |
| ATOM | 6379 | NE2 | HIS D 201 | 31.922 | 34.886 | 69.595 | 1.00 25.24 | N |
| ATOM | 6380 | N | PRO D 202 | 28.630 | 31.515 | 71.907 | 1.00 30.09 | N |
| ATOM | 6381 | CA | PRO D 202 | 29.017 | 30.722 | 73.086 | 1.00 31.51 | C |
| ATOM | 6382 | C | PRO D 202 | 30.319 | 31.171 | 73.744 | 1.00 32.04 | C |
| ATOM | 6383 | O | PRO D 202 | 30.425 | 31.129 | 74.973 | 1.00 31.65 | O |
| ATOM | 6384 | CB | PRO D 202 | 29.145 | 29.299 | 72.542 | 1.00 32.41 | C |
| ATOM | 6385 | CG | PRO D 202 | 28.337 | 29.302 | 71.262 | 1.00 32.58 | C |
| ATOM | 6386 | CD | PRO D 202 | 28.477 | 30.681 | 70.702 | 1.00 30.97 | C |
| ATOM | 6387 | N | ALA D 203 | 31.279 | 31.650 | 72.957 | 1.00 31.26 | N |
| ATOM | 6388 | CA | ALA D 203 | 32.541 | 32.130 | 73.526 | 1.00 31.69 | C |
| ATOM | 6389 | C | ALA D 203 | 32.366 | 33.175 | 74.643 | 1.00 31.80 | C |
| ATOM | 6390 | O | ALA D 203 | 33.058 | 33.120 | 75.661 | 1.00 31.49 | O |
| ATOM | 6391 | CB | ALA D 203 | 33.464 | 32.675 | 72.406 | 1.00 30.69 | C |
| ATOM | 6392 | N | SER D 204 | 31.471 | 34.141 | 74.453 | 1.00 32.87 | N |
| ATOM | 6393 | CA | SER D 204 | 31.207 | 35.167 | 75.480 | 1.00 33.94 | C |
| ATOM | 6394 | C | SER D 204 | 29.934 | 34.852 | 76.253 | 1.00 34.11 | C |
| ATOM | 6395 | O | SER D 204 | 29.437 | 35.704 | 76.980 | 1.00 34.06 | O |
| ATOM | 6396 | CB | SER D 204 | 31.106 | 36.568 | 74.856 | 1.00 33.34 | C |
| ATOM | 6397 | OG | SER D 204 | 30.145 | 36.581 | 73.811 | 1.00 34.52 | O |
| ATOM | 6398 | N | SER D 205 | 29.445 | 33.618 | 76.105 | 1.00 35.96 | N |
| ATOM | 6399 | CA | SER D 205 | 28.228 | 33.111 | 76.782 | 1.00 37.20 | C |
| ATOM | 6400 | C | SER D 205 | 27.060 | 34.060 | 76.584 | 1.00 38.62 | C |
| ATOM | 6401 | O | SER D 205 | 26.306 | 34.312 | 77.518 | 1.00 39.67 | O |
| ATOM | 6402 | CB | SER D 205 | 28.468 | 32.870 | 78.286 | 1.00 37.86 | C |
| ATOM | 6403 | OG | SER D 205 | 29.290 | 31.730 | 78.513 | 1.00 37.25 | O |
| ATOM | 6404 | N | THR D 206 | 26.925 | 34.557 | 75.353 | 1.00 38.27 | N |
| ATOM | 6405 | CA | THR D 206 | 26.000 | 35.637 | 74.992 | 1.00 39.14 | C |
| ATOM | 6406 | C | THR D 206 | 24.771 | 35.101 | 74.244 | 1.00 38.59 | C |
| ATOM | 6407 | O | THR D 206 | 24.912 | 34.296 | 73.335 | 1.00 37.44 | O |
| ATOM | 6408 | CB | THR D 206 | 26.747 | 36.694 | 74.105 | 1.00 39.83 | C |
| ATOM | 6409 | OG1 | THR D 206 | 27.351 | 37.684 | 74.958 | 1.00 41.90 | O |
| ATOM | 6410 | CG2 | THR D 206 | 25.817 | 37.377 | 73.110 | 1.00 41.20 | C |
| ATOM | 6411 | N | LYS D 207 | 23.581 | 35.560 | 74.640 | 1.00 38.16 | N |
| ATOM | 6412 | CA | LYS D 207 | 22.333 | 35.185 | 73.987 | 1.00 39.36 | C |
| ATOM | 6413 | C | LYS D 207 | 21.466 | 36.441 | 73.869 | 1.00 39.05 | C |
| ATOM | 6414 | O | LYS D 207 | 20.971 | 36.953 | 74.879 | 1.00 39.81 | O |
| ATOM | 6415 | CB | LYS D 207 | 21.593 | 34.111 | 74.800 | 1.00 39.69 | C |
| ATOM | 6416 | CG | LYS D 207 | 21.947 | 32.680 | 74.466 | 1.00 40.42 | C |
| ATOM | 6417 | CD | LYS D 207 | 21.817 | 31.725 | 75.697 | 1.00 40.93 | C |
| ATOM | 6418 | CE | LYS D 207 | 20.469 | 31.824 | 76.419 | 1.00 42.58 | C |
| ATOM | 6419 | NZ | LYS D 207 | 20.377 | 30.915 | 77.630 | 1.00 42.89 | N |
| ATOM | 6420 | N | VAL D 208 | 21.295 | 36.939 | 72.649 | 1.00 37.59 | N |
| ATOM | 6421 | CA | VAL D 208 | 20.549 | 38.163 | 72.423 | 1.00 38.06 | C |
| ATOM | 6422 | C | VAL D 208 | 19.420 | 37.892 | 71.426 | 1.00 38.01 | C |
| ATOM | 6423 | O | VAL D 208 | 19.642 | 37.224 | 70.425 | 1.00 37.78 | O |
| ATOM | 6424 | CB | VAL D 208 | 21.443 | 39.277 | 71.848 | 1.00 39.12 | C |
| ATOM | 6425 | CG1 | VAL D 208 | 21.003 | 40.626 | 72.413 | 1.00 41.26 | C |

| | | | | | | | | | | | |
|---|---|---|---|---|---|---|---|---|---|---|---|
| ATOM | 6426 | CG2 | VAL | D | 208 | 22.902 | 39.056 | 72.172 | 1.00 | 39.02 | C |
| ATOM | 6427 | N | ASP | D | 209 | 18.217 | 38.389 | 71.710 | 1.00 | 37.21 | N |
| ATOM | 6428 | CA | ASP | D | 209 | 17.098 | 38.354 | 70.753 | 1.00 | 36.81 | C |
| ATOM | 6429 | C | ASP | D | 209 | 16.848 | 39.796 | 70.304 | 1.00 | 35.40 | C |
| ATOM | 6430 | O | ASP | D | 209 | 16.709 | 40.671 | 71.145 | 1.00 | 36.20 | O |
| ATOM | 6431 | CB | ASP | D | 209 | 15.821 | 37.833 | 71.420 | 1.00 | 37.55 | C |
| ATOM | 6432 | CG | ASP | D | 209 | 16.019 | 36.525 | 72.140 | 1.00 | 39.81 | C |
| ATOM | 6433 | OD1 | ASP | D | 209 | 16.609 | 35.603 | 71.532 | 1.00 | 40.44 | O |
| ATOM | 6434 | OD2 | ASP | D | 209 | 15.574 | 36.421 | 73.320 | 1.00 | 42.60 | O |
| ATOM | 6435 | N | LYS | D | 210 | 16.802 | 40.062 | 69.009 | 1.00 | 32.78 | N |
| ATOM | 6436 | CA | LYS | D | 210 | 16.488 | 41.402 | 68.558 | 1.00 | 32.34 | C |
| ATOM | 6437 | C | LYS | D | 210 | 15.232 | 41.372 | 67.730 | 1.00 | 30.02 | C |
| ATOM | 6438 | O | LYS | D | 210 | 15.230 | 40.797 | 66.650 | 1.00 | 26.95 | O |
| ATOM | 6439 | CB | LYS | D | 210 | 17.636 | 42.018 | 67.733 | 1.00 | 33.52 | C |
| ATOM | 6440 | CG | LYS | D | 210 | 18.889 | 42.303 | 68.555 | 1.00 | 36.52 | C |
| ATOM | 6441 | CD | LYS | D | 210 | 18.770 | 43.521 | 69.459 | 1.00 | 38.07 | C |
| ATOM | 6442 | CE | LYS | D | 210 | 19.908 | 43.551 | 70.472 | 1.00 | 38.48 | C |
| ATOM | 6443 | NZ | LYS | D | 210 | 19.834 | 44.672 | 71.474 | 1.00 | 40.43 | N |
| ATOM | 6444 | N | LYS | D | 211 | 14.177 | 42.007 | 68.247 | 1.00 | 29.66 | N |
| ATOM | 6445 | CA | LYS | D | 211 | 12.927 | 42.144 | 67.526 | 1.00 | 31.34 | C |
| ATOM | 6446 | C | LYS | D | 211 | 13.133 | 43.103 | 66.375 | 1.00 | 30.87 | C |
| ATOM | 6447 | O | LYS | D | 211 | 13.799 | 44.123 | 66.522 | 1.00 | 31.24 | O |
| ATOM | 6448 | CB | LYS | D | 211 | 11.832 | 42.674 | 68.445 | 1.00 | 31.93 | C |
| ATOM | 6449 | CG | LYS | D | 211 | 10.456 | 42.775 | 67.796 | 1.00 | 34.70 | C |
| ATOM | 6450 | CD | LYS | D | 211 | 9.376 | 43.095 | 68.848 | 1.00 | 35.48 | C |
| ATOM | 6451 | CE | LYS | D | 211 | 8.161 | 43.781 | 68.232 | 1.00 | 37.99 | C |
| ATOM | 6452 | NZ | LYS | D | 211 | 7.109 | 44.132 | 69.271 | 1.00 | 38.79 | N |
| ATOM | 6453 | N | ILE | D | 212 | 12.581 | 42.767 | 65.225 | 1.00 | 30.88 | N |
| ATOM | 6454 | CA | ILE | D | 212 | 12.627 | 43.667 | 64.087 | 1.00 | 32.94 | C |
| ATOM | 6455 | C | ILE | D | 212 | 11.352 | 44.490 | 64.187 | 1.00 | 34.56 | C |
| ATOM | 6456 | O | ILE | D | 212 | 10.282 | 43.972 | 63.917 | 1.00 | 34.35 | O |
| ATOM | 6457 | CB | ILE | D | 212 | 12.659 | 42.922 | 62.716 | 1.00 | 32.72 | C |
| ATOM | 6458 | CG1 | ILE | D | 212 | 13.635 | 41.744 | 62.712 | 1.00 | 31.85 | C |
| ATOM | 6459 | CG2 | ILE | D | 212 | 13.016 | 43.896 | 61.585 | 1.00 | 32.30 | C |
| ATOM | 6460 | CD1 | ILE | D | 212 | 15.094 | 42.115 | 62.892 | 1.00 | 31.85 | C |
| ATOM | 6461 | N | VAL | D | 213 | 11.460 | 45.746 | 64.615 | 1.00 | 36.25 | N |
| ATOM | 6462 | CA | VAL | D | 213 | 10.290 | 46.637 | 64.673 | 1.00 | 37.85 | C |
| ATOM | 6463 | C | VAL | D | 213 | 10.199 | 47.435 | 63.372 | 1.00 | 38.74 | C |
| ATOM | 6464 | O | VAL | D | 213 | 11.233 | 47.844 | 62.814 | 1.00 | 37.59 | O |
| ATOM | 6465 | CB | VAL | D | 213 | 10.306 | 47.580 | 65.898 | 1.00 | 38.38 | C |
| ATOM | 6466 | CG1 | VAL | D | 213 | 10.502 | 46.778 | 67.191 | 1.00 | 39.54 | C |
| ATOM | 6467 | CG2 | VAL | D | 213 | 11.385 | 48.655 | 65.772 | 1.00 | 39.72 | C |
| ATOM | 6468 | N | PRO | D | 214 | 8.967 | 47.622 | 62.857 | 1.00 | 39.80 | N |
| ATOM | 6469 | CA | PRO | D | 214 | 8.810 | 48.361 | 61.609 | 1.00 | 40.79 | C |
| ATOM | 6470 | C | PRO | D | 214 | 9.136 | 49.827 | 61.847 | 1.00 | 41.73 | C |
| ATOM | 6471 | O | PRO | D | 214 | 9.035 | 50.297 | 62.979 | 1.00 | 41.37 | O |
| ATOM | 6472 | CB | PRO | D | 214 | 7.330 | 48.174 | 61.272 | 1.00 | 40.61 | C |
| ATOM | 6473 | CG | PRO | D | 214 | 6.676 | 47.921 | 62.588 | 1.00 | 39.69 | C |
| ATOM | 6474 | CD | PRO | D | 214 | 7.668 | 47.184 | 63.410 | 1.00 | 40.13 | C |
| ATOM | 6475 | N | ARG | D | 215 | 9.546 | 50.525 | 60.793 | 1.00 | 43.65 | N |
| ATOM | 6476 | CA | ARG | D | 215 | 9.934 | 51.941 | 60.895 | 1.00 | 45.10 | C |
| ATOM | 6477 | C | ARG | D | 215 | 8.730 | 52.884 | 60.796 | 1.00 | 44.87 | C |
| ATOM | 6478 | O | ARG | D | 215 | 8.149 | 53.061 | 59.728 | 1.00 | 45.84 | O |
| ATOM | 6479 | CB | ARG | D | 215 | 10.996 | 52.277 | 59.840 | 1.00 | 45.41 | C |
| ATOM | 6480 | CG | ARG | D | 215 | 12.375 | 51.767 | 60.231 | 1.00 | 47.10 | C |
| ATOM | 6481 | CD | ARG | D | 215 | 13.260 | 51.494 | 59.031 | 1.00 | 48.52 | C |
| ATOM | 6482 | NE | ARG | D | 215 | 14.619 | 51.167 | 59.459 | 1.00 | 50.13 | N |
| ATOM | 6483 | CZ | ARG | D | 215 | 15.621 | 52.037 | 59.613 | 1.00 | 50.44 | C |
| ATOM | 6484 | NH1 | ARG | D | 215 | 15.476 | 53.334 | 59.349 | 1.00 | 50.78 | N |
| ATOM | 6485 | NH2 | ARG | D | 215 | 16.795 | 51.596 | 60.025 | 1.00 | 50.40 | N |
| TER | 6486 | | ARG | D | 215 | | | | | | |
| HETATM | 6487 | O | HOH | A | 215 | 26.821 | 73.392 | 27.775 | 1.00 | 21.98 | O |
| HETATM | 6488 | O | HOH | A | 216 | 40.222 | 23.148 | 5.812 | 1.00 | 19.98 | O |
| HETATM | 6489 | O | HOH | A | 217 | 27.615 | 61.179 | 9.631 | 1.00 | 23.15 | O |
| HETATM | 6490 | O | HOH | A | 218 | 46.288 | 42.104 | 20.107 | 1.00 | 21.71 | O |

| HETATM | 6491 | O | HOH | A | 219 | 35.061 | 63.794 | 37.104 | 1.00 | 20.46 | O |
|--------|------|---|-----|---|-----|--------|--------|--------|------|-------|---|
| HETATM | 6492 | O | HOH | A | 220 | 38.880 | 65.508 | 35.687 | 1.00 | 17.17 | O |
| HETATM | 6493 | O | HOH | A | 221 | 42.671 | 64.657 | 32.563 | 1.00 | 21.49 | O |
| HETATM | 6494 | O | HOH | A | 222 | 48.447 | 42.908 | 19.227 | 1.00 | 29.38 | O |
| HETATM | 6495 | O | HOH | A | 223 | 31.865 | 67.269 | 33.284 | 1.00 | 18.50 | O |
| HETATM | 6496 | O | HOH | A | 224 | 38.322 | 77.162 | 21.205 | 1.00 | 31.50 | O |
| HETATM | 6497 | O | HOH | A | 225 | 43.896 | 45.838 | 33.767 | 1.00 | 43.36 | O |
| HETATM | 6498 | O | HOH | A | 226 | 29.794 | 50.475 | 24.757 | 1.00 | 31.50 | O |
| HETATM | 6499 | O | HOH | A | 227 | 37.930 | 62.544 | 13.646 | 1.00 | 27.23 | O |
| HETATM | 6500 | O | HOH | A | 228 | 44.542 | 32.731 | 13.044 | 1.00 | 28.46 | O |
| HETATM | 6501 | O | HOH | A | 229 | 39.910 | 59.544 | 15.010 | 1.00 | 27.73 | O |
| HETATM | 6502 | O | HOH | A | 230 | 37.009 | 63.749 | 34.941 | 1.00 | 22.45 | O |
| HETATM | 6503 | O | HOH | A | 231 | 29.762 | 63.818 | 39.872 | 1.00 | 39.40 | O |
| HETATM | 6504 | O | HOH | A | 232 | 45.599 | 29.610 | 7.437 | 1.00 | 30.14 | O |
| HETATM | 6505 | O | HOH | A | 233 | 33.628 | 64.349 | 11.493 | 1.00 | 23.93 | O |
| HETATM | 6506 | O | HOH | A | 234 | 51.380 | 24.052 | 23.222 | 1.00 | 22.38 | O |
| HETATM | 6507 | O | HOH | A | 235 | 41.015 | 76.552 | 18.767 | 1.00 | 32.68 | O |
| HETATM | 6508 | O | HOH | A | 236 | 33.007 | 73.749 | 29.861 | 1.00 | 23.54 | O |
| HETATM | 6509 | O | HOH | A | 237 | 41.680 | 47.473 | 29.826 | 1.00 | 24.18 | O |
| HETATM | 6510 | O | HOH | A | 238 | 41.350 | 64.469 | 34.840 | 1.00 | 24.93 | O |
| HETATM | 6511 | O | HOH | A | 239 | 60.036 | 23.046 | 12.591 | 1.00 | 31.81 | O |
| HETATM | 6512 | O | HOH | A | 240 | 39.022 | 54.388 | 39.624 | 1.00 | 34.10 | O |
| HETATM | 6513 | O | HOH | A | 241 | 47.938 | 30.055 | 24.634 | 1.00 | 25.24 | O |
| HETATM | 6514 | O | HOH | A | 242 | 40.987 | 13.899 | 3.891 | 1.00 | 29.36 | O |
| HETATM | 6515 | O | HOH | A | 243 | 43.404 | 62.524 | 22.326 | 1.00 | 21.05 | O |
| HETATM | 6516 | O | HOH | A | 244 | 38.029 | 68.649 | 30.283 | 1.00 | 21.61 | O |
| HETATM | 6517 | O | HOH | A | 245 | 42.293 | 41.049 | 24.410 | 1.00 | 32.15 | O |
| HETATM | 6518 | O | HOH | A | 246 | 27.152 | 68.371 | 9.862 | 1.00 | 25.73 | O |
| HETATM | 6519 | O | HOH | A | 247 | 38.796 | 42.131 | 20.330 | 1.00 | 31.92 | O |
| HETATM | 6520 | O | HOH | A | 248 | 33.404 | 57.978 | 13.009 | 1.00 | 30.10 | O |
| HETATM | 6521 | O | HOH | A | 249 | 32.935 | 71.520 | 32.051 | 1.00 | 32.37 | O |
| HETATM | 6522 | O | HOH | A | 250 | 53.953 | 50.620 | 27.766 | 1.00 | 39.41 | O |
| HETATM | 6523 | O | HOH | A | 251 | 44.260 | 39.369 | 25.987 | 1.00 | 31.29 | O |
| HETATM | 6524 | O | HOH | A | 252 | 53.792 | 29.991 | 26.984 | 1.00 | 25.22 | O |
| HETATM | 6525 | O | HOH | A | 253 | 52.932 | 20.407 | 21.124 | 1.00 | 39.80 | O |
| HETATM | 6526 | O | HOH | A | 254 | 37.469 | 69.694 | 12.790 | 1.00 | 32.74 | O |
| HETATM | 6527 | O | HOH | A | 255 | 60.509 | 28.413 | 24.312 | 1.00 | 40.63 | O |
| HETATM | 6528 | O | HOH | A | 256 | 20.618 | 66.383 | 24.772 | 1.00 | 35.04 | O |
| HETATM | 6529 | O | HOH | A | 257 | 24.410 | 61.445 | 35.701 | 1.00 | 42.18 | O |
| HETATM | 6530 | O | HOH | A | 258 | 50.840 | 27.124 | 3.993 | 1.00 | 47.44 | O |
| HETATM | 6531 | O | HOH | A | 259 | 42.031 | 52.050 | 38.653 | 1.00 | 33.98 | O |
| HETATM | 6532 | O | HOH | A | 260 | 45.421 | 60.397 | 28.051 | 1.00 | 26.13 | O |
| HETATM | 6533 | O | HOH | A | 261 | 43.505 | 50.320 | 23.673 | 1.00 | 28.87 | O |
| HETATM | 6534 | O | HOH | A | 262 | 45.356 | 39.506 | 9.173 | 1.00 | 32.41 | O |
| HETATM | 6535 | O | HOH | A | 263 | 37.697 | 47.948 | 35.636 | 1.00 | 32.35 | O |
| HETATM | 6536 | O | HOH | A | 264 | 40.810 | 58.145 | 12.820 | 1.00 | 35.80 | O |
| HETATM | 6537 | O | HOH | A | 265 | 31.958 | 78.618 | 13.722 | 1.00 | 35.87 | O |
| HETATM | 6538 | O | HOH | A | 266 | 31.491 | 76.186 | 25.928 | 1.00 | 28.24 | O |
| HETATM | 6539 | O | HOH | A | 267 | 49.838 | 39.276 | 9.637 | 1.00 | 43.82 | O |
| HETATM | 6540 | O | HOH | A | 268 | 62.930 | 22.487 | 4.444 | 1.00 | 36.54 | O |
| HETATM | 6541 | O | HOH | A | 269 | 50.771 | 48.418 | 24.143 | 1.00 | 23.48 | O |
| HETATM | 6542 | O | HOH | A | 270 | 19.622 | 69.410 | 22.622 | 1.00 | 36.68 | O |
| HETATM | 6543 | O | HOH | A | 271 | 40.932 | 48.654 | 36.972 | 1.00 | 26.51 | O |
| HETATM | 6544 | O | HOH | A | 272 | 49.608 | 46.130 | 35.727 | 1.00 | 44.35 | O |
| HETATM | 6545 | O | HOH | A | 273 | 41.020 | 70.066 | 23.253 | 1.00 | 38.23 | O |
| HETATM | 6546 | O | HOH | A | 274 | 34.275 | 54.261 | 13.738 | 1.00 | 28.25 | O |
| HETATM | 6547 | O | HOH | A | 275 | 56.864 | 43.621 | 22.403 | 1.00 | 38.03 | O |
| HETATM | 6548 | O | HOH | A | 276 | 46.853 | 58.932 | 17.601 | 1.00 | 38.97 | O |
| HETATM | 6549 | O | HOH | A | 277 | 41.211 | 67.515 | 17.610 | 1.00 | 42.47 | O |
| HETATM | 6550 | O | HOH | A | 278 | 48.522 | 50.365 | 34.902 | 1.00 | 31.39 | O |
| HETATM | 6551 | O | HOH | A | 279 | 44.030 | 19.593 | 17.332 | 1.00 | 33.82 | O |
| HETATM | 6552 | O | HOH | A | 280 | 20.478 | 76.573 | 20.418 | 1.00 | 45.88 | O |
| HETATM | 6553 | O | HOH | A | 281 | 41.790 | 21.621 | 12.664 | 1.00 | 24.56 | O |
| HETATM | 6554 | O | HOH | A | 282 | 28.392 | 75.443 | 27.169 | 1.00 | 39.34 | O |
| HETATM | 6555 | O | HOH | A | 283 | 56.788 | 30.686 | 2.053 | 1.00 | 37.92 | O |

| | | | | | | | | | | |
|---|---|---|---|---|---|---|---|---|---|---|
| HETATM | 6556 | O | HOH | A | 284 | 39.186 | 74.932 | 16.960 | 1.00 50.87 | O |
| HETATM | 6557 | O | HOH | A | 285 | 56.920 | 46.286 | 22.393 | 1.00 36.48 | O |
| HETATM | 6558 | O | HOH | A | 287 | 26.124 | 66.355 | 35.176 | 1.00 26.29 | O |
| HETATM | 6559 | O | HOH | A | 288 | 23.846 | 65.082 | 34.341 | 1.00 40.57 | O |
| HETATM | 6560 | O | HOH | A | 289 | 31.993 | 73.438 | 9.274 | 1.00 29.53 | O |
| HETATM | 6561 | O | HOH | A | 290 | 44.022 | 22.971 | 13.932 | 1.00 30.33 | O |
| HETATM | 6562 | O | HOH | A | 291 | 52.193 | 49.992 | 34.672 | 1.00 43.36 | O |
| HETATM | 6563 | O | HOH | A | 292 | 24.391 | 51.439 | 23.874 | 1.00 47.83 | O |
| HETATM | 6564 | O | HOH | A | 293 | 42.291 | 63.270 | 20.085 | 1.00 53.52 | O |
| HETATM | 6565 | O | HOH | A | 294 | 38.417 | 56.183 | 41.630 | 1.00 31.84 | O |
| HETATM | 6566 | O | HOH | A | 295 | 22.046 | 72.015 | 29.010 | 1.00 44.27 | O |
| HETATM | 6567 | O | HOH | A | 296 | 40.812 | 12.236 | 10.302 | 1.00 53.52 | O |
| HETATM | 6568 | O | HOH | A | 297 | 56.132 | 48.364 | 28.399 | 1.00 42.14 | O |
| HETATM | 6569 | O | HOH | A | 298 | 38.847 | 72.281 | 28.104 | 1.00 33.14 | O |
| HETATM | 6570 | O | HOH | A | 299 | 31.546 | 54.321 | 31.254 | 1.00 43.88 | O |
| HETATM | 6571 | O | HOH | A | 300 | 38.435 | 71.117 | 10.898 | 1.00 44.44 | O |
| HETATM | 6572 | O | HOH | A | 301 | 61.066 | 21.802 | 18.527 | 1.00 36.25 | O |
| HETATM | 6573 | O | HOH | A | 302 | 50.102 | 20.534 | 20.724 | 1.00 33.81 | O |
| HETATM | 6574 | O | HOH | A | 303 | 23.709 | 71.275 | 6.955 | 1.00 51.06 | O |
| HETATM | 6575 | O | HOH | A | 304 | 42.394 | 41.078 | 13.375 | 1.00 34.00 | O |
| HETATM | 6576 | O | HOH | A | 305 | 36.395 | 63.659 | 10.628 | 1.00 38.55 | O |
| HETATM | 6577 | O | HOH | A | 306 | 49.433 | 43.820 | 37.099 | 1.00 36.83 | O |
| HETATM | 6578 | O | HOH | A | 307 | 46.715 | 61.401 | 21.100 | 1.00 47.03 | O |
| HETATM | 6579 | O | HOH | A | 308 | 18.890 | 81.902 | 22.651 | 1.00 21.17 | O |
| HETATM | 6580 | O | HOH | A | 309 | 48.609 | 23.242 | 22.914 | 1.00 32.26 | O |
| HETATM | 6581 | O | HOH | A | 310 | 48.958 | 11.895 | 0.654 | 1.00 33.98 | O |
| HETATM | 6582 | O | HOH | A | 311 | 24.898 | 73.241 | 29.356 | 1.00 46.47 | O |
| HETATM | 6583 | O | HOH | A | 313 | 25.765 | 64.383 | 37.901 | 1.00 44.91 | O |
| HETATM | 6584 | O | HOH | A | 314 | 45.178 | 60.173 | 14.572 | 1.00 39.03 | O |
| HETATM | 6585 | O | HOH | A | 315 | 33.512 | 52.844 | 32.434 | 1.00 39.10 | O |
| HETATM | 6586 | O | HOH | A | 316 | 38.099 | 54.494 | 15.543 | 1.00 43.25 | O |
| HETATM | 6587 | O | HOH | A | 317 | 22.664 | 49.543 | 23.534 | 1.00 41.85 | O |
| HETATM | 6588 | O | HOH | A | 318 | 26.268 | 75.499 | 16.053 | 1.00 35.35 | O |
| HETATM | 6589 | O | HOH | A | 319 | 56.238 | 32.849 | 9.023 | 1.00 44.45 | O |
| HETATM | 6590 | O | HOH | A | 320 | 35.515 | 48.446 | 28.461 | 1.00 35.47 | O |
| HETATM | 6591 | O | HOH | A | 321 | 43.634 | 60.283 | 38.478 | 1.00 20.61 | O |
| HETATM | 6592 | O | HOH | A | 322 | 36.765 | 68.285 | 32.578 | 1.00 18.31 | O |
| HETATM | 6593 | O | HOH | A | 323 | 54.223 | 28.692 | -1.472 | 1.00 34.53 | O |
| HETATM | 6594 | O | HOH | B | 218 | 31.493 | 31.957 | 26.262 | 1.00 22.68 | O |
| HETATM | 6595 | O | HOH | B | 219 | 25.876 | 40.532 | 7.246 | 1.00 25.19 | O |
| HETATM | 6596 | O | HOH | B | 220 | 47.115 | 23.611 | 24.938 | 1.00 22.28 | O |
| HETATM | 6597 | O | HOH | B | 221 | 24.217 | 64.674 | 3.758 | 1.00 25.98 | O |
| HETATM | 6598 | O | HOH | B | 222 | 31.006 | 16.964 | 31.957 | 1.00 25.20 | O |
| HETATM | 6599 | O | HOH | B | 223 | 25.642 | 37.688 | -1.950 | 1.00 25.14 | O |
| HETATM | 6600 | O | HOH | B | 224 | 41.975 | 31.587 | 26.197 | 1.00 26.65 | O |
| HETATM | 6601 | O | HOH | B | 225 | 16.277 | 44.063 | -5.391 | 1.00 33.17 | O |
| HETATM | 6602 | O | HOH | B | 226 | 34.855 | 13.165 | 30.818 | 1.00 54.99 | O |
| HETATM | 6603 | O | HOH | B | 227 | 40.239 | 39.726 | 25.157 | 1.00 42.50 | O |
| HETATM | 6604 | O | HOH | B | 228 | 23.531 | 55.803 | 25.152 | 1.00 28.23 | O |
| HETATM | 6605 | O | HOH | B | 229 | 21.430 | 36.951 | 4.976 | 1.00 26.67 | O |
| HETATM | 6606 | O | HOH | B | 230 | 13.850 | 52.327 | -0.467 | 1.00 30.33 | O |
| HETATM | 6607 | O | HOH | B | 231 | 30.197 | 61.100 | 10.825 | 1.00 19.59 | O |
| HETATM | 6608 | O | HOH | B | 232 | 35.313 | 18.075 | 26.393 | 1.00 30.27 | O |
| HETATM | 6609 | O | HOH | B | 233 | 44.687 | 16.583 | 23.569 | 1.00 32.93 | O |
| HETATM | 6610 | O | HOH | B | 234 | 46.413 | 31.106 | 26.837 | 1.00 31.95 | O |
| HETATM | 6611 | O | HOH | B | 235 | 38.157 | 27.353 | 4.167 | 1.00 38.33 | O |
| HETATM | 6612 | O | HOH | B | 236 | 13.235 | 43.760 | 9.265 | 1.00 41.44 | O |
| HETATM | 6613 | O | HOH | B | 237 | 31.168 | 42.449 | -3.925 | 1.00 40.83 | O |
| HETATM | 6614 | O | HOH | B | 238 | 19.282 | 39.591 | 3.069 | 1.00 29.96 | O |
| HETATM | 6615 | O | HOH | B | 239 | 16.995 | 64.619 | 2.619 | 1.00 36.00 | O |
| HETATM | 6616 | O | HOH | B | 240 | 25.151 | 24.696 | 7.712 | 1.00 41.11 | O |
| HETATM | 6617 | O | HOH | B | 241 | 16.773 | 40.656 | 1.354 | 1.00 37.27 | O |
| HETATM | 6618 | O | HOH | B | 242 | 40.561 | 16.708 | 33.567 | 1.00 33.75 | O |
| HETATM | 6619 | O | HOH | B | 243 | 31.328 | 25.202 | 32.293 | 1.00 33.71 | O |
| HETATM | 6620 | O | HOH | B | 244 | 26.298 | 25.859 | 33.265 | 1.00 36.82 | O |

```
HETATM 6621  O   HOH B 245   30.458  53.754   7.751  1.00  32.82        O
HETATM 6622  O   HOH B 246   15.636  58.619  -0.101  1.00  30.30        O
HETATM 6623  O   HOH B 247   13.272  45.235  15.878  1.00  41.83        O
HETATM 6624  O   HOH B 248   31.581  22.103  10.981  1.00  33.88        O
HETATM 6625  O   HOH B 249   31.068  43.889   0.491  1.00  30.21        O
HETATM 6626  O   HOH B 250   30.288  40.088   6.049  1.00  34.53        O
HETATM 6627  O   HOH B 251   35.963  15.571  27.659  1.00  32.15        O
HETATM 6628  O   HOH B 252   19.045  64.069  23.377  1.00  34.11        O
HETATM 6629  O   HOH B 253   27.604  46.441  17.596  1.00  38.32        O
HETATM 6630  O   HOH B 254   41.852  33.443  13.832  1.00  39.53        O
HETATM 6631  O   HOH B 255   31.413  35.620  19.322  1.00  38.25        O
HETATM 6632  O   HOH B 256   23.476  41.149  12.713  1.00  38.70        O
HETATM 6633  O   HOH B 257   30.523  20.983  27.368  1.00  40.67        O
HETATM 6634  O   HOH B 258   39.467  17.435  19.638  1.00  35.67        O
HETATM 6635  O   HOH B 259   30.824  48.275  14.425  1.00  43.68        O
HETATM 6636  O   HOH B 260    8.435  62.912   4.485  1.00  51.51        O
HETATM 6637  O   HOH B 261   30.876  32.492  -0.540  1.00  29.04        O
HETATM 6638  O   HOH B 262   26.658  61.637   2.334  1.00  37.26        O
HETATM 6639  O   HOH B 263   33.775  13.314  28.268  1.00  61.75        O
HETATM 6640  O   HOH B 264   13.644  42.468   3.864  1.00  37.39        O
HETATM 6641  O   HOH B 265   40.899  35.352  12.448  1.00  35.12        O
HETATM 6642  O   HOH B 266   32.257  52.000   4.079  1.00  32.36        O
HETATM 6643  O   HOH B 267   27.429  23.613  25.557  1.00  37.11        O
HETATM 6644  O   HOH B 268   28.166  62.586   0.334  1.00  35.30        O
HETATM 6645  O   HOH B 269   29.712  28.444  -1.048  1.00  35.18        O
HETATM 6646  O   HOH B 270   24.499  41.450  -8.546  1.00  34.21        O
HETATM 6647  O   HOH B 271   17.049  60.147  23.334  1.00  42.70        O
HETATM 6648  O   HOH B 272   24.241  24.733   5.159  1.00  34.39        O
HETATM 6649  O   HOH B 273   10.880  59.338   3.159  1.00  41.66        O
HETATM 6650  O   HOH B 274   37.167  37.625  23.236  1.00  43.31        O
HETATM 6651  O   HOH B 275   22.377  29.997  13.523  1.00  39.82        O
HETATM 6652  O   HOH B 276   35.994  37.203  25.787  1.00  47.17        O
HETATM 6653  O   HOH B 277   26.926  54.459   0.172  1.00  38.16        O
HETATM 6654  O   HOH B 278   34.940  39.147  13.738  1.00  42.42        O
HETATM 6655  O   HOH B 279   21.068  64.417  26.064  1.00  47.00        O
HETATM 6656  O   HOH B 280   12.739  51.306  -4.922  1.00  24.82        O
HETATM 6657  O   HOH B 281   25.802  35.371   9.676  1.00  31.89        O
HETATM 6658  O   HOH B 282   27.498  30.725  19.552  1.00  40.13        O
HETATM 6659  O   HOH B 283   19.423  64.655  28.263  1.00  48.21        O
HETATM 6660  O   HOH B 284   41.950  26.698   3.076  1.00  37.74        O
HETATM 6661  O   HOH B 285   32.990  40.266   0.045  1.00  42.18        O
HETATM 6662  O   HOH B 286   29.128  37.934  16.762  1.00  37.80        O
HETATM 6663  O   HOH B 287   36.591  18.598  38.375  1.00  42.36        O
HETATM 6664  O   HOH B 288   34.096  37.582  -0.176  1.00  45.98        O
HETATM 6665  O   HOH B 289   35.445  37.500  16.247  1.00  37.37        O
HETATM 6666  O   HOH B 290   49.404  16.377  22.822  1.00  47.83        O
HETATM 6667  O   HOH B 291   30.057  13.128  25.533  1.00  32.98        O
HETATM 6668  O   HOH B 292   50.834  23.388  32.645  1.00  45.85        O
HETATM 6669  O   HOH B 293   10.130  52.462   4.531  1.00  48.54        O
HETATM 6670  O   HOH B 294   44.132  17.744  31.199  1.00  43.69        O
HETATM 6671  O   HOH B 295    5.766  56.363   8.290  1.00  50.05        O
HETATM 6672  O   HOH B 296   12.454  69.190  13.098  1.00  38.23        O
HETATM 6673  O   HOH B 297   22.109  53.552  24.512  1.00  32.23        O
HETATM 6674  O   HOH B 298   30.032  30.746  -2.877  1.00  43.58        O
HETATM 6675  O   HOH B 299   44.731  22.172  37.608  1.00  49.63        O
HETATM 6676  O   HOH B 300   22.826  44.465  -9.481  1.00  34.29        O
HETATM 6677  O   HOH B 301   31.384  49.684   3.738  1.00  37.73        O
HETATM 6678  O   HOH B 302   25.663  45.769  -9.659  1.00  35.71        O
HETATM 6679  O   HOH B 303   32.461  48.832   1.359  1.00  29.28        O
HETATM 6680  O   HOH C 286   33.724  -2.504  43.544  1.00  44.55        O
HETATM 6681  O   HOH C 287   33.886   5.829  36.975  1.00  14.16        O
HETATM 6682  O   HOH C 288   29.135   6.674  38.308  1.00  20.62        O
HETATM 6683  O   HOH C 289   21.858  48.546  58.792  1.00  22.29        O
HETATM 6684  O   HOH C 290   28.019  41.986  53.725  1.00  25.96        O
HETATM 6685  O   HOH C 291   48.772  16.718  54.404  1.00  19.68        O
```

```
HETATM 6686  O    HOH C 292    29.027  49.816  64.182  1.00 25.09        O
HETATM 6687  O    HOH C 293    22.388  48.934  61.528  1.00 24.00        O
HETATM 6688  O    HOH C 294    16.450  40.044  49.235  1.00 30.15        O
HETATM 6689  O    HOH C 295    28.219  26.317  47.101  1.00 30.36        O
HETATM 6690  O    HOH C 296    30.937  63.872  65.391  1.00 28.36        O
HETATM 6691  O    HOH C 297    14.238  47.220  49.628  1.00 26.52        O
HETATM 6692  O    HOH C 298    33.630   2.576  40.972  1.00 28.99        O
HETATM 6693  O    HOH C 299    19.396  56.621  66.609  1.00 29.29        O
HETATM 6694  O    HOH C 300    39.905   1.997  37.442  1.00 37.76        O
HETATM 6695  O    HOH C 301    16.123  50.926  63.427  1.00 32.37        O
HETATM 6696  O    HOH C 302    53.227  12.592  50.554  1.00 22.98        O
HETATM 6697  O    HOH C 303    42.637  -0.496  42.873  1.00 32.17        O
HETATM 6698  O    HOH C 304    35.860   7.428  36.370  1.00 24.50        O
HETATM 6699  O    HOH C 305    22.063  57.360  45.191  1.00 25.90        O
HETATM 6700  O    HOH C 306    26.191  29.097  53.213  1.00 31.62        O
HETATM 6701  O    HOH C 307    44.189  -0.504  40.185  1.00 27.81        O
HETATM 6702  O    HOH C 308    30.544  11.245  34.705  1.00 22.17        O
HETATM 6703  O    HOH C 309    14.038  51.206  46.056  1.00 35.26        O
HETATM 6704  O    HOH C 310    15.030  38.108  49.884  1.00 36.80        O
HETATM 6705  O    HOH C 311    23.207  10.749  34.232  1.00 27.60        O
HETATM 6706  O    HOH C 312    13.782  46.822  52.421  1.00 28.93        O
HETATM 6707  O    HOH C 313    40.712  22.171  45.327  1.00 34.20        O
HETATM 6708  O    HOH C 314    22.359  29.914  49.080  1.00 36.29        O
HETATM 6709  O    HOH C 315    49.308   4.237  35.454  1.00 36.47        O
HETATM 6710  O    HOH C 316    13.964  42.791  43.937  1.00 33.56        O
HETATM 6711  O    HOH C 317    31.712  35.054  52.273  1.00 50.58        O
HETATM 6712  O    HOH C 318    40.724  -0.575  40.388  1.00 37.20        O
HETATM 6713  O    HOH C 319    18.051  54.616  60.197  1.00 30.92        O
HETATM 6714  O    HOH C 320    26.913  29.305  38.715  1.00 32.13        O
HETATM 6715  O    HOH C 321    32.076  -1.159  45.114  1.00 29.81        O
HETATM 6716  O    HOH C 322    19.253  53.740  70.241  1.00 33.56        O
HETATM 6717  O    HOH C 323    20.215  31.350  47.715  1.00 27.58        O
HETATM 6718  O    HOH C 324    47.511   8.137  33.157  1.00 39.89        O
HETATM 6719  O    HOH C 325    42.688  17.150  38.747  1.00 50.51        O
HETATM 6720  O    HOH C 326    21.615  25.274  51.863  1.00 38.60        O
HETATM 6721  O    HOH C 327    17.075  52.403  69.554  1.00 45.50        O
HETATM 6722  O    HOH C 328    27.625  23.931  44.882  1.00 30.35        O
HETATM 6723  O    HOH C 329    44.477  20.237  44.794  1.00 30.26        O
HETATM 6724  O    HOH C 330    27.782   4.379  38.988  1.00 24.47        O
HETATM 6725  O    HOH C 331    41.262  24.816  46.651  1.00 39.26        O
HETATM 6726  O    HOH C 332    33.611  41.499  40.811  1.00 52.51        O
HETATM 6727  O    HOH C 333    17.191  62.653  52.911  1.00 39.77        O
HETATM 6728  O    HOH C 334    14.416  51.977  66.477  1.00 47.58        O
HETATM 6729  O    HOH C 335    56.000  16.208  44.939  1.00 37.51        O
HETATM 6730  O    HOH C 336    26.750  14.108  55.882  1.00 55.63        O
HETATM 6731  O    HOH C 337    52.771  16.972  49.323  1.00 66.46        O
HETATM 6732  O    HOH C 338    20.926  51.661  71.727  1.00 33.30        O
HETATM 6733  O    HOH C 339    13.718  56.138  50.960  1.00 32.09        O
HETATM 6734  O    HOH C 340    17.177  48.978  58.747  1.00 41.63        O
HETATM 6735  O    HOH C 341    18.491  11.482  33.870  1.00 40.52        O
HETATM 6736  O    HOH C 342    14.638  32.732  42.329  1.00 47.59        O
HETATM 6737  O    HOH C 343    23.254  51.619  70.717  1.00 29.99        O
HETATM 6738  O    HOH C 344    44.884   2.466  38.080  1.00 29.14        O
HETATM 6739  O    HOH C 345    31.164  53.602  61.420  1.00 60.35        O
HETATM 6740  O    HOH C 346    16.834  44.479  35.180  1.00 40.11        O
HETATM 6741  O    HOH C 347    53.160  18.122  52.191  1.00 35.41        O
HETATM 6742  O    HOH C 348    18.568  65.823  54.610  1.00 34.99        O
HETATM 6743  O    HOH C 349    27.705   0.796  49.069  1.00 39.95        O
HETATM 6744  O    HOH C 350    27.094  62.420  63.360  1.00 35.10        O
HETATM 6745  O    HOH C 351    20.057  66.530  57.156  1.00 40.55        O
HETATM 6746  O    HOH C 352    22.342   2.621  45.515  1.00 41.08        O
HETATM 6747  O    HOH C 353    35.075  21.664  31.759  1.00 34.84        O
HETATM 6748  O    HOH C 354    22.168  22.851  45.473  1.00 36.55        O
HETATM 6749  O    HOH C 355    18.822   7.077  39.358  1.00 39.35        O
HETATM 6750  O    HOH C 356    52.749   4.281  45.171  1.00 36.54        O
```

| HETATM | 6751 | O | HOH C 357 | 33.033 | 0.885 | 54.329 | 1.00 | 41.20 | O |
|--------|------|---|-----------|--------|-------|--------|------|-------|---|
| HETATM | 6752 | O | HOH C 358 | 13.366 | 26.167 | 42.588 | 1.00 | 53.24 | O |
| HETATM | 6753 | O | HOH C 359 | 14.324 | 35.719 | 37.180 | 1.00 | 35.17 | O |
| HETATM | 6754 | O | HOH C 360 | 55.630 | 13.599 | 44.346 | 1.00 | 40.88 | O |
| HETATM | 6755 | O | HOH C 361 | 36.976 | 1.863 | 37.784 | 1.00 | 25.84 | O |
| HETATM | 6756 | O | HOH C 362 | 28.915 | 9.160 | 34.852 | 1.00 | 26.75 | O |
| HETATM | 6757 | O | HOH C 363 | 31.440 | 46.867 | 55.374 | 1.00 | 45.10 | O |
| HETATM | 6758 | O | HOH D 312 | 41.758 | 31.260 | 77.204 | 1.00 | 50.02 | O |
| HETATM | 6759 | O | HOH D 313 | 58.470 | 18.374 | 72.807 | 1.00 | 44.07 | O |
| HETATM | 6760 | O | HOH D 314 | 16.119 | 29.989 | 61.821 | 1.00 | 14.77 | O |
| HETATM | 6761 | O | HOH D 315 | 50.814 | 7.995 | 73.733 | 1.00 | 20.93 | O |
| HETATM | 6762 | O | HOH D 316 | 30.546 | 33.038 | 62.906 | 1.00 | 21.92 | O |
| HETATM | 6763 | O | HOH D 317 | 44.657 | 23.312 | 76.486 | 1.00 | 23.85 | O |
| HETATM | 6764 | O | HOH D 318 | 5.608 | 36.049 | 66.007 | 1.00 | 28.24 | O |
| HETATM | 6765 | O | HOH D 319 | 48.622 | 23.366 | 76.681 | 1.00 | 21.20 | O |
| HETATM | 6766 | O | HOH D 320 | 43.187 | 3.787 | 61.573 | 1.00 | 22.97 | O |
| HETATM | 6767 | O | HOH D 321 | 8.644 | 42.182 | 64.811 | 1.00 | 24.21 | O |
| HETATM | 6768 | O | HOH D 322 | 52.700 | 25.779 | 61.733 | 1.00 | 24.13 | O |
| HETATM | 6769 | O | HOH D 323 | 47.076 | -0.832 | 74.534 | 1.00 | 47.68 | O |
| HETATM | 6770 | O | HOH D 324 | 56.016 | 16.391 | 56.307 | 1.00 | 28.31 | O |
| HETATM | 6771 | O | HOH D 325 | 33.736 | 9.054 | 59.185 | 1.00 | 26.85 | O |
| HETATM | 6772 | O | HOH D 326 | 38.120 | 28.937 | 67.892 | 1.00 | 27.62 | O |
| HETATM | 6773 | O | HOH D 327 | 6.870 | 33.068 | 62.792 | 1.00 | 30.08 | O |
| HETATM | 6774 | O | HOH D 328 | 37.324 | -2.646 | 66.276 | 1.00 | 31.19 | O |
| HETATM | 6775 | O | HOH D 329 | 30.306 | 32.734 | 59.417 | 1.00 | 38.07 | O |
| HETATM | 6776 | O | HOH D 330 | 34.951 | 23.851 | 67.674 | 1.00 | 29.59 | O |
| HETATM | 6777 | O | HOH D 331 | 10.205 | 49.045 | 58.467 | 1.00 | 28.64 | O |
| HETATM | 6778 | O | HOH D 332 | 3.758 | 37.856 | 67.402 | 1.00 | 22.25 | O |
| HETATM | 6779 | O | HOH D 333 | 47.361 | 17.757 | 52.139 | 1.00 | 18.02 | O |
| HETATM | 6780 | O | HOH D 334 | 56.069 | 12.449 | 69.846 | 1.00 | 27.54 | O |
| HETATM | 6781 | O | HOH D 335 | 49.820 | 14.902 | 73.416 | 1.00 | 20.39 | O |
| HETATM | 6782 | O | HOH D 336 | 51.272 | 34.307 | 64.347 | 1.00 | 25.32 | O |
| HETATM | 6783 | O | HOH D 337 | 54.263 | 19.745 | 71.890 | 1.00 | 27.94 | O |
| HETATM | 6784 | O | HOH D 338 | 49.309 | -3.004 | 69.980 | 1.00 | 29.70 | O |
| HETATM | 6785 | O | HOH D 339 | 9.269 | 37.154 | 67.390 | 1.00 | 28.45 | O |
| HETATM | 6786 | O | HOH D 340 | 58.404 | 11.013 | 66.383 | 1.00 | 28.28 | O |
| HETATM | 6787 | O | HOH D 341 | 26.009 | 3.639 | 66.323 | 1.00 | 24.67 | O |
| HETATM | 6788 | O | HOH D 342 | 27.219 | 39.538 | 56.076 | 1.00 | 27.96 | O |
| HETATM | 6789 | O | HOH D 343 | 12.193 | 34.391 | 68.969 | 1.00 | 34.70 | O |
| HETATM | 6790 | O | HOH D 344 | 35.113 | 31.734 | 75.747 | 1.00 | 30.04 | O |
| HETATM | 6791 | O | HOH D 345 | 40.530 | 3.312 | 59.273 | 1.00 | 25.80 | O |
| HETATM | 6792 | O | HOH D 346 | 39.958 | 36.457 | 75.198 | 1.00 | 19.68 | O |
| HETATM | 6793 | O | HOH D 347 | 58.945 | -0.361 | 66.732 | 1.00 | 32.76 | O |
| HETATM | 6794 | O | HOH D 348 | 47.472 | 0.443 | 70.680 | 1.00 | 22.83 | O |
| HETATM | 6795 | O | HOH D 349 | 39.226 | 1.615 | 53.413 | 1.00 | 38.98 | O |
| HETATM | 6796 | O | HOH D 350 | 25.653 | 31.437 | 74.065 | 1.00 | 33.83 | O |
| HETATM | 6797 | O | HOH D 351 | 52.021 | -3.478 | 65.327 | 1.00 | 29.32 | O |
| HETATM | 6798 | O | HOH D 352 | 33.055 | 1.509 | 71.914 | 1.00 | 33.05 | O |
| HETATM | 6799 | O | HOH D 353 | 46.335 | 35.083 | 63.659 | 1.00 | 31.31 | O |
| HETATM | 6800 | O | HOH D 354 | 38.035 | 1.226 | 72.843 | 1.00 | 37.42 | O |
| HETATM | 6801 | O | HOH D 355 | 44.250 | 19.947 | 50.847 | 1.00 | 29.71 | O |
| HETATM | 6802 | O | HOH D 356 | 56.956 | 16.899 | 67.479 | 1.00 | 31.68 | O |
| HETATM | 6803 | O | HOH D 357 | 43.552 | 36.142 | 71.962 | 1.00 | 27.37 | O |
| HETATM | 6804 | O | HOH D 358 | 20.853 | 31.371 | 67.968 | 1.00 | 32.43 | O |
| HETATM | 6805 | O | HOH D 359 | 42.090 | 32.626 | 75.230 | 1.00 | 30.05 | O |
| HETATM | 6806 | O | HOH D 360 | 49.296 | 41.459 | 67.914 | 1.00 | 41.85 | O |
| HETATM | 6807 | O | HOH D 361 | 58.404 | 26.502 | 67.720 | 1.00 | 35.12 | O |
| HETATM | 6808 | O | HOH D 362 | 49.030 | 19.189 | 50.699 | 1.00 | 34.17 | O |
| HETATM | 6809 | O | HOH D 363 | 32.909 | 45.585 | 63.823 | 1.00 | 34.31 | O |
| HETATM | 6810 | O | HOH D 364 | 51.247 | 17.592 | 55.911 | 1.00 | 27.13 | O |
| HETATM | 6811 | O | HOH D 365 | 44.379 | 33.503 | 62.887 | 1.00 | 31.03 | O |
| HETATM | 6812 | O | HOH D 366 | 57.915 | 0.150 | 73.866 | 1.00 | 35.66 | O |
| HETATM | 6813 | O | HOH D 367 | 55.920 | -1.844 | 64.379 | 1.00 | 27.56 | O |
| HETATM | 6814 | O | HOH D 368 | 20.905 | 29.543 | 51.491 | 1.00 | 42.41 | O |
| HETATM | 6815 | O | HOH D 369 | 37.518 | 32.386 | 65.141 | 1.00 | 31.75 | O |

| | | | | | | | | | | | |
|---|---|---|---|---|---|---|---|---|---|---|---|
| HETATM | 6816 | O | HOH | D | 370 | -1.417 | 38.533 | 61.052 | 1.00 | 37.11 | O |
| HETATM | 6817 | O | HOH | D | 371 | 41.033 | 0.202 | 73.228 | 1.00 | 36.20 | O |
| HETATM | 6818 | O | HOH | D | 372 | 54.899 | 7.680 | 71.519 | 1.00 | 28.72 | O |
| HETATM | 6819 | O | HOH | D | 373 | 53.656 | 11.419 | 72.249 | 1.00 | 34.30 | O |
| HETATM | 6820 | O | HOH | D | 374 | 50.567 | -1.026 | 63.635 | 1.00 | 34.56 | O |
| HETATM | 6821 | O | HOH | D | 375 | 58.692 | 15.158 | 60.640 | 1.00 | 40.35 | O |
| HETATM | 6822 | O | HOH | D | 376 | 18.981 | 37.459 | 76.652 | 1.00 | 39.10 | O |
| HETATM | 6823 | O | HOH | D | 377 | 41.760 | 22.937 | 75.293 | 1.00 | 28.39 | O |
| HETATM | 6824 | O | HOH | D | 378 | 32.695 | 10.119 | 63.360 | 1.00 | 24.82 | O |
| HETATM | 6825 | O | HOH | D | 379 | 31.614 | 32.496 | 78.176 | 1.00 | 39.05 | O |
| HETATM | 6826 | O | HOH | D | 380 | 40.202 | 42.573 | 67.519 | 1.00 | 33.40 | O |
| HETATM | 6827 | O | HOH | D | 381 | 27.538 | 32.325 | 59.044 | 1.00 | 23.81 | O |
| HETATM | 6828 | O | HOH | D | 382 | 6.127 | 43.749 | 65.060 | 1.00 | 36.84 | O |
| HETATM | 6829 | O | HOH | D | 383 | 39.085 | 30.075 | 74.351 | 1.00 | 35.02 | O |
| HETATM | 6830 | O | HOH | D | 384 | 10.889 | 43.541 | 51.772 | 1.00 | 37.97 | O |
| HETATM | 6831 | O | HOH | D | 385 | 48.679 | 12.637 | 76.573 | 1.00 | 33.97 | O |
| HETATM | 6832 | O | HOH | D | 386 | 54.781 | 20.051 | 56.566 | 1.00 | 28.95 | O |
| HETATM | 6833 | O | HOH | D | 387 | 30.504 | -0.013 | 65.954 | 1.00 | 31.41 | O |
| HETATM | 6834 | O | HOH | D | 388 | 35.129 | 21.820 | 69.656 | 1.00 | 38.61 | O |
| HETATM | 6835 | O | HOH | D | 389 | 42.110 | 36.957 | 64.044 | 1.00 | 29.19 | O |
| HETATM | 6836 | O | HOH | D | 390 | 38.101 | 38.970 | 76.507 | 1.00 | 32.22 | O |
| HETATM | 6837 | O | HOH | D | 391 | 6.781 | 40.669 | 47.432 | 1.00 | 57.63 | O |
| HETATM | 6838 | O | HOH | D | 392 | 17.596 | 28.053 | 57.995 | 1.00 | 43.73 | O |
| HETATM | 6839 | O | HOH | D | 393 | 18.486 | 29.319 | 60.167 | 1.00 | 34.49 | O |
| HETATM | 6840 | O | HOH | D | 394 | 16.134 | 48.688 | 61.976 | 1.00 | 32.97 | O |
| HETATM | 6841 | O | HOH | D | 395 | 29.373 | 38.694 | 56.059 | 1.00 | 40.41 | O |
| HETATM | 6842 | O | HOH | D | 396 | 45.103 | 27.202 | 75.646 | 1.00 | 29.51 | O |
| HETATM | 6843 | O | HOH | D | 397 | 15.846 | 35.585 | 53.123 | 1.00 | 29.80 | O |
| HETATM | 6844 | O | HOH | D | 398 | 20.822 | 30.841 | 60.296 | 1.00 | 29.67 | O |
| HETATM | 6845 | O | HOH | D | 399 | 37.379 | 0.004 | 53.763 | 1.00 | 39.80 | O |
| HETATM | 6846 | O | HOH | D | 400 | 30.576 | 3.187 | 61.516 | 1.00 | 27.64 | O |
| HETATM | 6847 | O | HOH | D | 401 | 4.197 | 43.941 | 55.874 | 1.00 | 34.56 | O |
| HETATM | 6848 | O | HOH | D | 402 | 36.706 | 25.183 | 69.270 | 1.00 | 33.22 | O |
| HETATM | 6849 | O | HOH | D | 403 | 13.933 | 47.376 | 63.833 | 1.00 | 32.26 | O |
| HETATM | 6850 | O | HOH | D | 404 | 58.502 | 7.363 | 64.976 | 1.00 | 46.21 | O |
| HETATM | 6851 | O | HOH | D | 405 | 51.793 | 25.489 | 58.223 | 1.00 | 46.81 | O |
| HETATM | 6852 | O | HOH | D | 406 | 6.388 | 45.962 | 66.584 | 1.00 | 42.50 | O |
| HETATM | 6853 | O | HOH | D | 407 | 44.329 | 1.520 | 61.747 | 1.00 | 32.87 | O |
| HETATM | 6854 | O | HOH | D | 408 | 58.206 | 12.587 | 61.941 | 1.00 | 28.95 | O |
| HETATM | 6855 | O | HOH | D | 409 | 53.600 | 27.767 | 73.728 | 1.00 | 25.81 | O |
| HETATM | 6856 | O | HOH | D | 410 | 44.248 | 40.534 | 77.896 | 1.00 | 33.88 | O |
| HETATM | 6857 | O | HOH | D | 411 | 12.818 | 44.390 | 52.981 | 1.00 | 36.06 | O |
| HETATM | 6858 | O | HOH | D | 412 | 57.125 | 35.341 | 70.480 | 1.00 | 43.70 | O |
| HETATM | 6859 | O | HOH | D | 413 | 54.887 | -3.215 | 62.347 | 1.00 | 28.08 | O |
| HETATM | 6860 | O | HOH | D | 414 | 35.740 | 28.964 | 68.321 | 1.00 | 35.06 | O |
| HETATM | 6861 | O | HOH | D | 415 | 45.774 | 21.500 | 49.365 | 1.00 | 38.75 | O |
| HETATM | 6862 | O | HOH | D | 416 | 45.519 | 29.129 | 59.497 | 1.00 | 31.83 | O |
| HETATM | 6863 | O | HOH | D | 417 | 22.978 | 29.355 | 60.217 | 1.00 | 36.83 | O |
| HETATM | 6864 | O | HOH | D | 418 | 58.811 | 24.916 | 65.295 | 1.00 | 36.69 | O |
| HETATM | 6865 | O | HOH | D | 419 | 34.465 | 29.141 | 75.916 | 1.00 | 44.55 | O |
| HETATM | 6866 | O | HOH | D | 420 | 51.014 | 36.897 | 71.020 | 1.00 | 36.86 | O |
| HETATM | 6867 | O | HOH | D | 421 | 39.437 | 26.812 | 59.179 | 1.00 | 46.80 | O |
| HETATM | 6868 | O | HOH | D | 422 | 39.709 | 28.023 | 63.592 | 1.00 | 31.85 | O |
| HETATM | 6869 | O | HOH | D | 423 | 24.125 | 30.671 | 62.175 | 1.00 | 35.21 | O |
| HETATM | 6870 | O | HOH | D | 424 | 16.634 | 29.949 | 52.600 | 1.00 | 41.70 | O |
| HETATM | 6871 | O | HOH | D | 425 | 39.546 | 17.729 | 75.535 | 1.00 | 31.86 | O |
| HETATM | 6872 | O | HOH | D | 426 | 32.873 | 9.024 | 70.487 | 1.00 | 31.22 | O |
| HETATM | 6873 | O | HOH | D | 427 | 46.081 | 25.489 | 59.962 | 1.00 | 24.02 | O |
| HETATM | 6874 | O | HOH | D | 428 | 57.077 | 15.710 | 73.028 | 1.00 | 44.97 | O |
| HETATM | 6875 | O | HOH | D | 429 | 45.948 | 0.690 | 59.801 | 1.00 | 42.39 | O |
| HETATM | 6876 | O | HOH | D | 430 | 11.322 | 39.536 | 70.794 | 1.00 | 35.38 | O |
| HETATM | 6877 | O | HOH | D | 431 | 50.054 | -5.126 | 72.199 | 1.00 | 41.97 | O |
| HETATM | 6878 | O | HOH | D | 432 | 47.811 | 20.659 | 78.068 | 1.00 | 46.91 | O |
| HETATM | 6879 | O | HOH | D | 433 | 21.319 | 28.369 | 63.312 | 1.00 | 41.24 | O |
| HETATM | 6880 | O | HOH | D | 434 | 33.853 | 26.458 | 67.897 | 1.00 | 34.81 | O |

```
HETATM 6881  O    HOH D 435    11.088  39.884  49.781  1.00 39.75        O
HETATM 6882  O    HOH D 436    28.645   8.120  63.830  1.00 45.46        O
HETATM 6883  O    HOH D 437    33.648  38.279  76.189  1.00 36.34        O
HETATM 6884  O    HOH D 438    34.179  47.831  55.495  1.00 43.27        O
HETATM 6885  O    HOH D 439     1.520  42.206  59.117  1.00 35.60        O
HETATM 6886  O    HOH D 440     7.295  26.206  60.655  1.00 42.31        O
HETATM 6887  O    HOH D 441    12.933  51.838  55.423  1.00 38.33        O
END
```

## TABLE 15

### Crystal 3- 3BZ4

Crystal structure of Fab F22-4 in complex with a *Shigella flexneri* 2a O-Ag decasaccharide

```
HEADER    IMMUNE SYSTEM                        17-JAN-08    3BZ4
TITLE     CRYSTAL STRUCTURE OF FAB F22-4 IN COMPLEX WITH A SHIGELLA
TITLE    2 FLEXNERI 2A O-AG DECASACCHARIDE
CRYST1   67.060   137.600   109.800   90.00   95.15   90.00 P 1 21 1        8
ATOM      1  N   ASP A   1       2.817  31.264  25.864  1.00 13.75           N
ATOM      2  CA  ASP A   1       4.204  31.101  25.398  1.00 15.00           C
ATOM      3  C   ASP A   1       5.094  30.810  26.569  1.00 14.48           C
ATOM      4  O   ASP A   1       4.982  31.443  27.632  1.00 14.52           O
ATOM      5  CB  ASP A   1       4.759  32.353  24.694  1.00 16.50           C
ATOM      6  CG  ASP A   1       4.042  32.698  23.391  1.00 18.69           C
ATOM      7  OD1 ASP A   1       3.126  31.965  22.984  1.00 19.78           O
ATOM      8  OD2 ASP A   1       4.405  33.738  22.772  1.00 19.31           O
ATOM      9  N   ILE A   2       5.978  29.830  26.367  1.00 14.64           N
ATOM     10  CA  ILE A   2       7.067  29.502  27.288  1.00 14.92           C
ATOM     11  C   ILE A   2       8.343  29.508  26.437  1.00 14.72           C
ATOM     12  O   ILE A   2       8.345  28.983  25.308  1.00 14.23           O
ATOM     13  CB  ILE A   2       6.857  28.094  27.926  1.00 15.14           C
ATOM     14  CG1 ILE A   2       5.619  28.125  28.831  1.00 15.82           C
ATOM     15  CG2 ILE A   2       8.068  27.653  28.747  1.00 15.70           C
ATOM     16  CD1 ILE A   2       5.143  26.788  29.348  1.00 16.18           C
ATOM     17  N   VAL A   3       9.390  30.131  26.970  1.00 15.01           N
ATOM     18  CA  VAL A   3      10.727  30.116  26.394  1.00 14.87           C
ATOM     19  C   VAL A   3      11.562  29.076  27.160  1.00 14.43           C
ATOM     20  O   VAL A   3      11.628  29.103  28.398  1.00 13.86           O
ATOM     21  CB  VAL A   3      11.374  31.525  26.412  1.00 15.12           C
ATOM     22  CG1 VAL A   3      12.841  31.473  25.954  1.00 16.22           C
ATOM     23  CG2 VAL A   3      10.561  32.478  25.529  1.00 14.34           C
ATOM     24  N   MET A   4      12.160  28.155  26.407  1.00 14.56           N
ATOM     25  CA  MET A   4      13.099  27.159  26.942  1.00 14.39           C
ATOM     26  C   MET A   4      14.492  27.583  26.558  1.00 14.20           C
ATOM     27  O   MET A   4      14.776  27.758  25.382  1.00 14.98           O
ATOM     28  CB  MET A   4      12.827  25.772  26.356  1.00 14.48           C
ATOM     29  CG  MET A   4      11.416  25.249  26.528  1.00 15.33           C
ATOM     30  SD  MET A   4      10.891  25.042  28.229  1.00 15.20           S
ATOM     31  CE  MET A   4      11.706  23.532  28.716  1.00 17.32           C
ATOM     32  N   THR A   5      15.371  27.736  27.542  1.00 12.95           N
ATOM     33  CA  THR A   5      16.701  28.280  27.291  1.00 14.27           C
ATOM     34  C   THR A   5      17.793  27.250  27.548  1.00 14.58           C
ATOM     35  O   THR A   5      17.909  26.700  28.651  1.00 15.40           O
ATOM     36  CB  THR A   5      16.956  29.540  28.125  1.00 15.82           C
ATOM     37  OG1 THR A   5      15.894  30.481  27.901  1.00 17.30           O
ATOM     38  CG2 THR A   5      18.287  30.170  27.734  1.00 16.25           C
ATOM     39  N   GLN A   6      18.572  26.954  26.519  1.00 14.65           N
ATOM     40  CA  GLN A   6      19.777  26.151  26.702  1.00 15.69           C
ATOM     41  C   GLN A   6      20.925  26.678  25.850  1.00 16.93           C
ATOM     42  O   GLN A   6      20.723  27.319  24.825  1.00 16.80           O
ATOM     43  CB  GLN A   6      19.512  24.654  26.429  1.00 16.50           C
ATOM     44  CG  GLN A   6      18.932  24.373  25.119  1.00 18.86           C
ATOM     45  CD  GLN A   6      18.458  22.935  24.916  1.00 15.06           C
ATOM     46  OE1 GLN A   6      17.474  22.741  24.242  1.00 15.88           O
ATOM     47  NE2 GLN A   6      19.206  21.930  25.411  1.00 17.82           N
ATOM     48  N   ALA A   7      22.143  26.407  26.314  1.00 18.58           N
ATOM     49  CA  ALA A   7      23.366  26.799  25.618  1.00 19.25           C
ATOM     50  C   ALA A   7      23.537  26.050  24.307  1.00 20.01           C
ATOM     51  O   ALA A   7      23.310  24.856  24.257  1.00 18.80           O
ATOM     52  CB  ALA A   7      24.534  26.505  26.502  1.00 20.86           C
ATOM     53  N   ALA A   8      23.964  26.740  23.247  1.00 21.93           N
```

| ATOM | 54 | CA | ALA | A | 8 | 24.212 | 26.070 | 21.965 | 1.00 | 23.21 | C |
|------|-----|-----|-----|---|----|--------|--------|--------|------|-------|---|
| ATOM | 55 | C | ALA | A | 8 | 25.295 | 24.996 | 22.076 | 1.00 | 24.79 | C |
| ATOM | 56 | O | ALA | A | 8 | 25.214 | 23.964 | 21.414 | 1.00 | 25.27 | O |
| ATOM | 57 | CB | ALA | A | 8 | 24.584 | 27.082 | 20.888 | 1.00 | 23.01 | C |
| ATOM | 58 | N | PHE | A | 9 | 26.300 | 25.225 | 22.921 | 1.00 | 27.46 | N |
| ATOM | 59 | CA | PHE | A | 9 | 27.440 | 24.305 | 23.042 | 1.00 | 30.93 | C |
| ATOM | 60 | C | PHE | A | 9 | 27.786 | 23.918 | 24.478 | 1.00 | 32.88 | C |
| ATOM | 61 | O | PHE | A | 9 | 27.398 | 24.579 | 25.431 | 1.00 | 31.42 | O |
| ATOM | 62 | CB | PHE | A | 9 | 28.689 | 24.931 | 22.412 | 1.00 | 32.16 | C |
| ATOM | 63 | CG | PHE | A | 9 | 28.475 | 25.398 | 21.019 | 1.00 | 32.84 | C |
| ATOM | 64 | CD1 | PHE | A | 9 | 28.276 | 24.482 | 20.002 | 1.00 | 33.68 | C |
| ATOM | 65 | CD2 | PHE | A | 9 | 28.444 | 26.749 | 20.723 | 1.00 | 32.74 | C |
| ATOM | 66 | CE1 | PHE | A | 9 | 28.055 | 24.907 | 18.704 | 1.00 | 34.54 | C |
| ATOM | 67 | CE2 | PHE | A | 9 | 28.221 | 27.186 | 19.434 | 1.00 | 34.24 | C |
| ATOM | 68 | CZ | PHE | A | 9 | 28.027 | 26.274 | 18.422 | 1.00 | 34.01 | C |
| ATOM | 69 | N | SER | A | 10 | 28.536 | 22.830 | 24.605 | 1.00 | 35.97 | N |
| ATOM | 70 | CA | SER | A | 10 | 29.081 | 22.394 | 25.888 | 1.00 | 37.74 | C |
| ATOM | 71 | C | SER | A | 10 | 30.585 | 22.128 | 25.759 | 1.00 | 38.71 | C |
| ATOM | 72 | O | SER | A | 10 | 31.106 | 21.993 | 24.645 | 1.00 | 39.38 | O |
| ATOM | 73 | CB | SER | A | 10 | 28.334 | 21.146 | 26.357 | 1.00 | 38.85 | C |
| ATOM | 74 | OG | SER | A | 10 | 28.395 | 20.101 | 25.404 | 1.00 | 38.91 | O |
| ATOM | 75 | N | ASN | A | 11 | 31.292 | 22.075 | 26.889 | 1.00 | 39.73 | N |
| ATOM | 76 | CA | ASN | A | 11 | 32.717 | 21.738 | 26.860 | 1.00 | 39.66 | C |
| ATOM | 77 | C | ASN | A | 11 | 32.892 | 20.355 | 26.253 | 1.00 | 37.71 | C |
| ATOM | 78 | O | ASN | A | 11 | 32.162 | 19.424 | 26.616 | 1.00 | 37.07 | O |
| ATOM | 79 | CB | ASN | A | 11 | 33.335 | 21.745 | 28.259 | 1.00 | 41.45 | C |
| ATOM | 80 | CG | ASN | A | 11 | 33.403 | 23.142 | 28.875 | 1.00 | 43.91 | C |
| ATOM | 81 | OD1 | ASN | A | 11 | 32.951 | 24.131 | 28.281 | 1.00 | 45.92 | O |
| ATOM | 82 | ND2 | ASN | A | 11 | 33.975 | 23.223 | 30.079 | 1.00 | 46.06 | N |
| ATOM | 83 | N | PRO | A | 12 | 33.848 | 20.211 | 25.320 | 1.00 | 35.82 | N |
| ATOM | 84 | CA | PRO | A | 12 | 34.120 | 18.879 | 24.791 | 1.00 | 34.16 | C |
| ATOM | 85 | C | PRO | A | 12 | 34.404 | 17.907 | 25.925 | 1.00 | 32.65 | C |
| ATOM | 86 | O | PRO | A | 12 | 35.096 | 18.258 | 26.888 | 1.00 | 31.89 | O |
| ATOM | 87 | CB | PRO | A | 12 | 35.356 | 19.090 | 23.912 | 1.00 | 34.80 | C |
| ATOM | 88 | CG | PRO | A | 12 | 35.282 | 20.525 | 23.521 | 1.00 | 35.33 | C |
| ATOM | 89 | CD | PRO | A | 12 | 34.697 | 21.238 | 24.692 | 1.00 | 35.51 | C |
| ATOM | 90 | N | VAL | A | 13 | 33.838 | 16.712 | 25.817 | 1.00 | 30.14 | N |
| ATOM | 91 | CA | VAL | A | 13 | 33.884 | 15.722 | 26.867 | 1.00 | 29.32 | C |
| ATOM | 92 | C | VAL | A | 13 | 34.677 | 14.507 | 26.412 | 1.00 | 28.30 | C |
| ATOM | 93 | O | VAL | A | 13 | 34.477 | 13.972 | 25.319 | 1.00 | 26.41 | O |
| ATOM | 94 | CB | VAL | A | 13 | 32.444 | 15.331 | 27.297 | 1.00 | 29.66 | C |
| ATOM | 95 | CG1 | VAL | A | 13 | 32.461 | 14.266 | 28.351 | 1.00 | 30.67 | C |
| ATOM | 96 | CG2 | VAL | A | 13 | 31.725 | 16.564 | 27.801 | 1.00 | 29.31 | C |
| ATOM | 97 | N | THR | A | 14 | 35.588 | 14.072 | 27.271 | 1.00 | 26.73 | N |
| ATOM | 98 | CA | THR | A | 14 | 36.399 | 12.910 | 26.996 | 1.00 | 26.58 | C |
| ATOM | 99 | C | THR | A | 14 | 35.555 | 11.652 | 27.035 | 1.00 | 26.69 | C |
| ATOM | 100 | O | THR | A | 14 | 34.665 | 11.504 | 27.873 | 1.00 | 25.99 | O |
| ATOM | 101 | CB | THR | A | 14 | 37.561 | 12.820 | 28.013 | 1.00 | 26.60 | C |
| ATOM | 102 | OG1 | THR | A | 14 | 38.411 | 13.956 | 27.832 | 1.00 | 27.38 | O |
| ATOM | 103 | CG2 | THR | A | 14 | 38.355 | 11.554 | 27.815 | 1.00 | 27.09 | C |
| ATOM | 104 | N | LEU | A | 15 | 35.825 | 10.722 | 26.133 | 1.00 | 27.30 | N |
| ATOM | 105 | CA | LEU | A | 15 | 35.096 | 9.478 | 26.162 | 1.00 | 27.57 | C |
| ATOM | 106 | C | LEU | A | 15 | 35.203 | 8.821 | 27.531 | 1.00 | 28.48 | C |
| ATOM | 107 | O | LEU | A | 15 | 36.285 | 8.749 | 28.126 | 1.00 | 29.23 | O |
| ATOM | 108 | CB | LEU | A | 15 | 35.597 | 8.504 | 25.089 | 1.00 | 29.01 | C |
| ATOM | 109 | CG | LEU | A | 15 | 35.113 | 8.655 | 23.652 | 1.00 | 30.52 | C |
| ATOM | 110 | CD1 | LEU | A | 15 | 35.764 | 7.557 | 22.839 | 1.00 | 32.80 | C |
| ATOM | 111 | CD2 | LEU | A | 15 | 33.611 | 8.560 | 23.528 | 1.00 | 31.10 | C |
| ATOM | 112 | N | GLY | A | 16 | 34.080 | 8.314 | 28.020 | 1.00 | 28.05 | N |
| ATOM | 113 | CA | GLY | A | 16 | 34.063 | 7.564 | 29.270 | 1.00 | 28.72 | C |
| ATOM | 114 | C | GLY | A | 16 | 33.877 | 8.424 | 30.504 | 1.00 | 28.37 | C |
| ATOM | 115 | O | GLY | A | 16 | 33.805 | 7.895 | 31.621 | 1.00 | 29.51 | O |
| ATOM | 116 | N | THR | A | 17 | 33.805 | 9.739 | 30.314 | 1.00 | 27.53 | N |
| ATOM | 117 | CA | THR | A | 17 | 33.516 | 10.661 | 31.398 | 1.00 | 27.75 | C |
| ATOM | 118 | C | THR | A | 17 | 32.145 | 11.334 | 31.216 | 1.00 | 27.24 | C |

| ATOM | 119 | O | THR | A | 17 | 31.477 | 11.149 | 30.203 | 1.00 | 26.53 | O |
| ATOM | 120 | CB | THR | A | 17 | 34.584 | 11.734 | 31.512 | 1.00 | 27.18 | C |
| ATOM | 121 | OG1 | THR | A | 17 | 34.428 | 12.700 | 30.473 | 1.00 | 28.03 | O |
| ATOM | 122 | CG2 | THR | A | 17 | 35.983 | 11.108 | 31.459 | 1.00 | 28.33 | C |
| ATOM | 123 | N | SER | A | 18 | 31.754 | 12.138 | 32.201 | 1.00 | 25.98 | N |
| ATOM | 124 | CA | SER | A | 18 | 30.378 | 12.589 | 32.290 | 1.00 | 25.22 | C |
| ATOM | 125 | C | SER | A | 18 | 30.155 | 13.908 | 31.575 | 1.00 | 23.28 | C |
| ATOM | 126 | O | SER | A | 18 | 31.000 | 14.805 | 31.592 | 1.00 | 21.20 | O |
| ATOM | 127 | CB | SER | A | 18 | 29.983 | 12.719 | 33.761 | 1.00 | 25.82 | C |
| ATOM | 128 | OG | SER | A | 18 | 30.590 | 13.878 | 34.305 | 1.00 | 28.34 | O |
| ATOM | 129 | N | ALA | A | 19 | 28.988 | 14.039 | 30.952 | 1.00 | 22.67 | N |
| ATOM | 130 | CA | ALA | A | 19 | 28.553 | 15.325 | 30.422 | 1.00 | 22.66 | C |
| ATOM | 131 | C | ALA | A | 19 | 27.336 | 15.803 | 31.205 | 1.00 | 21.73 | C |
| ATOM | 132 | O | ALA | A | 19 | 26.643 | 15.002 | 31.788 | 1.00 | 21.21 | O |
| ATOM | 133 | CB | ALA | A | 19 | 28.198 | 15.186 | 28.929 | 1.00 | 23.01 | C |
| ATOM | 134 | N | SER | A | 20 | 27.114 | 17.111 | 31.215 | 1.00 | 22.91 | N |
| ATOM | 135 | CA | SER | A | 20 | 25.994 | 17.741 | 31.922 | 1.00 | 23.66 | C |
| ATOM | 136 | C | SER | A | 20 | 25.415 | 18.889 | 31.077 | 1.00 | 23.52 | C |
| ATOM | 137 | O | SER | A | 20 | 26.107 | 19.880 | 30.770 | 1.00 | 25.22 | O |
| ATOM | 138 | CB | SER | A | 20 | 26.479 | 18.263 | 33.282 | 1.00 | 24.61 | C |
| ATOM | 139 | OG | SER | A | 20 | 25.430 | 18.809 | 34.070 | 1.00 | 27.18 | O |
| ATOM | 140 | N | ILE | A | 21 | 24.154 | 18.747 | 30.697 | 1.00 | 21.90 | N |
| ATOM | 141 | CA | ILE | A | 21 | 23.484 | 19.725 | 29.850 | 1.00 | 22.43 | C |
| ATOM | 142 | C | ILE | A | 21 | 22.318 | 20.355 | 30.625 | 1.00 | 21.07 | C |
| ATOM | 143 | O | ILE | A | 21 | 21.483 | 19.648 | 31.195 | 1.00 | 19.89 | O |
| ATOM | 144 | CB | ILE | A | 21 | 22.998 | 19.052 | 28.564 | 1.00 | 21.53 | C |
| ATOM | 145 | CG1 | ILE | A | 21 | 24.211 | 18.505 | 27.792 | 1.00 | 23.93 | C |
| ATOM | 146 | CG2 | ILE | A | 21 | 22.223 | 20.032 | 27.679 | 1.00 | 22.15 | C |
| ATOM | 147 | CD1 | ILE | A | 21 | 23.872 | 17.722 | 26.528 | 1.00 | 23.83 | C |
| ATOM | 148 | N | SER | A | 22 | 22.273 | 21.684 | 30.607 | 1.00 | 21.46 | N |
| ATOM | 149 | CA | SER | A | 22 | 21.306 | 22.443 | 31.377 | 1.00 | 20.65 | C |
| ATOM | 150 | C | SER | A | 22 | 20.239 | 23.030 | 30.487 | 1.00 | 19.55 | C |
| ATOM | 151 | O | SER | A | 22 | 20.443 | 23.218 | 29.286 | 1.00 | 19.40 | O |
| ATOM | 152 | CB | SER | A | 22 | 21.994 | 23.565 | 32.125 | 1.00 | 22.09 | C |
| ATOM | 153 | OG | SER | A | 22 | 22.865 | 23.017 | 33.091 | 1.00 | 26.84 | O |
| ATOM | 154 | N | CYS | A | 23 | 19.105 | 23.314 | 31.107 | 1.00 | 18.17 | N |
| ATOM | 155 | CA | CYS | A | 23 | 18.001 | 23.991 | 30.446 | 1.00 | 18.67 | C |
| ATOM | 156 | C | CYS | A | 23 | 17.239 | 24.717 | 31.531 | 1.00 | 17.88 | C |
| ATOM | 157 | O | CYS | A | 23 | 17.305 | 24.355 | 32.707 | 1.00 | 16.20 | O |
| ATOM | 158 | CB | CYS | A | 23 | 17.117 | 22.988 | 29.698 | 1.00 | 19.40 | C |
| ATOM | 159 | SG | CYS | A | 23 | 15.568 | 23.678 | 28.951 | 1.00 | 22.80 | S |
| ATOM | 160 | N | ARG | A | 24 | 16.542 | 25.765 | 31.135 | 1.00 | 17.26 | N |
| ATOM | 161 | CA | ARG | A | 24 | 15.651 | 26.430 | 32.044 | 1.00 | 18.81 | C |
| ATOM | 162 | C | ARG | A | 24 | 14.426 | 26.901 | 31.294 | 1.00 | 17.20 | C |
| ATOM | 163 | O | ARG | A | 24 | 14.463 | 27.049 | 30.080 | 1.00 | 16.95 | O |
| ATOM | 164 | CB | ARG | A | 24 | 16.379 | 27.575 | 32.733 | 1.00 | 19.14 | C |
| ATOM | 165 | CG | ARG | A | 24 | 16.844 | 28.663 | 31.834 | 1.00 | 20.87 | C |
| ATOM | 166 | CD | ARG | A | 24 | 17.669 | 29.664 | 32.625 | 1.00 | 23.47 | C |
| ATOM | 167 | NE | ARG | A | 24 | 18.189 | 30.753 | 31.793 | 1.00 | 25.54 | N |
| ATOM | 168 | CZ | ARG | A | 24 | 17.464 | 31.803 | 31.403 | 1.00 | 27.76 | C |
| ATOM | 169 | NH1 | ARG | A | 24 | 16.193 | 31.934 | 31.759 | 1.00 | 28.48 | N |
| ATOM | 170 | NH2 | ARG | A | 24 | 18.017 | 32.734 | 30.651 | 1.00 | 30.03 | N |
| ATOM | 171 | N | SER | A | 25 | 13.337 | 27.105 | 32.022 | 1.00 | 15.59 | N |
| ATOM | 172 | CA | SER | A | 25 | 12.085 | 27.526 | 31.424 | 1.00 | 15.71 | C |
| ATOM | 173 | C | SER | A | 25 | 11.644 | 28.873 | 32.013 | 1.00 | 16.04 | C |
| ATOM | 174 | O | SER | A | 25 | 12.011 | 29.241 | 33.138 | 1.00 | 15.14 | O |
| ATOM | 175 | CB | SER | A | 25 | 11.000 | 26.482 | 31.644 | 1.00 | 16.11 | C |
| ATOM | 176 | OG | SER | A | 25 | 10.800 | 26.249 | 33.017 | 1.00 | 18.15 | O |
| ATOM | 177 | N | SER | A | 26 | 10.842 | 29.586 | 31.237 | 1.00 | 15.79 | N |
| ATOM | 178 | CA | SER | A | 26 | 10.318 | 30.879 | 31.629 | 1.00 | 15.86 | C |
| ATOM | 179 | C | SER | A | 26 | 9.135 | 30.759 | 32.593 | 1.00 | 15.95 | C |
| ATOM | 180 | O | SER | A | 26 | 8.671 | 31.759 | 33.114 | 1.00 | 17.75 | O |
| ATOM | 181 | CB | SER | A | 26 | 9.919 | 31.676 | 30.394 | 1.00 | 15.75 | C |
| ATOM | 182 | OG | SER | A | 26 | 8.908 | 31.035 | 29.633 | 1.00 | 15.68 | O |
| ATOM | 183 | N | LYS | A | 27 | 8.654 | 29.539 | 32.819 | 1.00 | 16.16 | N |

| ATOM | 184 | CA | LYS | A | 27 | 7.492 | 29.292 | 33.644 | 1.00 | 18.72 | C |
| ATOM | 185 | C | LYS | A | 27 | 7.716 | 27.921 | 34.268 | 1.00 | 16.61 | C |
| ATOM | 186 | O | LYS | A | 27 | 8.304 | 27.040 | 33.637 | 1.00 | 16.21 | O |
| ATOM | 187 | CB | LYS | A | 27 | 6.229 | 29.264 | 32.762 | 1.00 | 20.28 | C |
| ATOM | 188 | CG | LYS | A | 27 | 4.928 | 29.235 | 33.511 | 1.00 | 23.93 | C |
| ATOM | 189 | CD | LYS | A | 27 | 3.699 | 29.029 | 32.610 | 1.00 | 24.25 | C |
| ATOM | 190 | CE | LYS | A | 27 | 3.475 | 30.138 | 31.558 | 1.00 | 26.31 | C |
| ATOM | 191 | NZ | LYS | A | 27 | 3.291 | 31.517 | 32.161 | 1.00 | 29.57 | N |
| ATOM | 192 | N | SER | A | 27A | 7.231 | 27.728 | 35.492 | 1.00 | 14.61 | N |
| ATOM | 193 | CA | SER | A | 27A | 7.292 | 26.411 | 36.108 | 1.00 | 14.09 | C |
| ATOM | 194 | C | SER | A | 27A | 6.547 | 25.341 | 35.317 | 1.00 | 14.42 | C |
| ATOM | 195 | O | SER | A | 27A | 5.395 | 25.527 | 34.893 | 1.00 | 14.03 | O |
| ATOM | 196 | CB | SER | A | 27A | 6.731 | 26.445 | 37.529 | 1.00 | 15.65 | C |
| ATOM | 197 | OG | SER | A | 27A | 6.878 | 25.156 | 38.109 | 1.00 | 15.20 | O |
| ATOM | 198 | N | LEU | A | 27B | 7.198 | 24.197 | 35.169 | 1.00 | 13.36 | N |
| ATOM | 199 | CA | LEU | A | 27B | 6.644 | 23.024 | 34.487 | 1.00 | 13.32 | C |
| ATOM | 200 | C | LEU | A | 27B | 6.166 | 21.978 | 35.489 | 1.00 | 14.01 | C |
| ATOM | 201 | O | LEU | A | 27B | 5.716 | 20.892 | 35.106 | 1.00 | 14.73 | O |
| ATOM | 202 | CB | LEU | A | 27B | 7.718 | 22.415 | 33.565 | 1.00 | 13.80 | C |
| ATOM | 203 | CG | LEU | A | 27B | 8.371 | 23.355 | 32.554 | 1.00 | 12.39 | C |
| ATOM | 204 | CD1 | LEU | A | 27B | 9.213 | 22.569 | 31.583 | 1.00 | 13.30 | C |
| ATOM | 205 | CD2 | LEU | A | 27B | 7.375 | 24.217 | 31.786 | 1.00 | 13.90 | C |
| ATOM | 206 | N | LEU | A | 27C | 6.255 | 22.309 | 36.783 | 1.00 | 16.43 | N |
| ATOM | 207 | CA | LEU | A | 27C | 5.736 | 21.459 | 37.858 | 1.00 | 17.55 | C |
| ATOM | 208 | C | LEU | A | 27C | 4.229 | 21.659 | 37.973 | 1.00 | 18.32 | C |
| ATOM | 209 | O | LEU | A | 27C | 3.761 | 22.728 | 38.352 | 1.00 | 19.36 | O |
| ATOM | 210 | CB | LEU | A | 27C | 6.418 | 21.773 | 39.200 | 1.00 | 17.19 | C |
| ATOM | 211 | CG | LEU | A | 27C | 5.864 | 21.065 | 40.446 | 1.00 | 18.77 | C |
| ATOM | 212 | CD1 | LEU | A | 27C | 5.800 | 19.532 | 40.271 | 1.00 | 18.39 | C |
| ATOM | 213 | CD2 | LEU | A | 27C | 6.734 | 21.439 | 41.626 | 1.00 | 19.61 | C |
| ATOM | 214 | N | HIS | A | 27D | 3.482 | 20.604 | 37.644 | 1.00 | 19.64 | N |
| ATOM | 215 | CA | HIS | A | 27D | 2.023 | 20.620 | 37.627 | 1.00 | 20.29 | C |
| ATOM | 216 | C | HIS | A | 27D | 1.449 | 20.336 | 39.017 | 1.00 | 20.56 | C |
| ATOM | 217 | O | HIS | A | 27D | 2.130 | 19.779 | 39.872 | 1.00 | 19.43 | O |
| ATOM | 218 | CB | HIS | A | 27D | 1.545 | 19.554 | 36.644 | 1.00 | 21.59 | C |
| ATOM | 219 | CG | HIS | A | 27D | 0.116 | 19.680 | 36.232 | 1.00 | 22.43 | C |
| ATOM | 220 | ND1 | HIS | A | 27D | -0.914 | 19.101 | 36.940 | 1.00 | 22.69 | N |
| ATOM | 221 | CD2 | HIS | A | 27D | -0.456 | 20.282 | 35.163 | 1.00 | 24.16 | C |
| ATOM | 222 | CE1 | HIS | A | 27D | -2.060 | 19.347 | 36.330 | 1.00 | 23.86 | C |
| ATOM | 223 | NE2 | HIS | A | 27D | -1.812 | 20.076 | 35.257 | 1.00 | 24.39 | N |
| ATOM | 224 | N | SER | A | 27E | 0.186 | 20.693 | 39.229 | 1.00 | 22.85 | N |
| ATOM | 225 | CA | SER | A | 27E | -0.499 | 20.379 | 40.486 | 1.00 | 23.29 | C |
| ATOM | 226 | C | SER | A | 27E | -0.558 | 18.863 | 40.766 | 1.00 | 22.85 | C |
| ATOM | 227 | O | SER | A | 27E | -0.634 | 18.456 | 41.927 | 1.00 | 24.10 | O |
| ATOM | 228 | CB | SER | A | 27E | -1.900 | 21.000 | 40.517 | 1.00 | 25.14 | C |
| ATOM | 229 | OG | SER | A | 27E | -2.756 | 20.351 | 39.589 | 1.00 | 28.73 | O |
| ATOM | 230 | N | ASP | A | 28 | -0.486 | 18.033 | 39.718 | 1.00 | 21.45 | N |
| ATOM | 231 | CA | ASP | A | 28 | -0.473 | 16.580 | 39.869 | 1.00 | 20.52 | C |
| ATOM | 232 | C | ASP | A | 28 | 0.858 | 15.988 | 40.320 | 1.00 | 19.11 | C |
| ATOM | 233 | O | ASP | A | 28 | 0.979 | 14.764 | 40.444 | 1.00 | 18.91 | O |
| ATOM | 234 | CB | ASP | A | 28 | -0.923 | 15.895 | 38.574 | 1.00 | 20.42 | C |
| ATOM | 235 | CG | ASP | A | 28 | 0.058 | 16.101 | 37.401 | 1.00 | 20.48 | C |
| ATOM | 236 | OD1 | ASP | A | 28 | 1.189 | 16.595 | 37.609 | 1.00 | 18.77 | O |
| ATOM | 237 | OD2 | ASP | A | 28 | -0.331 | 15.748 | 36.255 | 1.00 | 22.53 | O |
| ATOM | 238 | N | GLY | A | 29 | 1.856 | 16.845 | 40.532 | 1.00 | 18.38 | N |
| ATOM | 239 | CA | GLY | A | 29 | 3.155 | 16.433 | 41.074 | 1.00 | 17.59 | C |
| ATOM | 240 | C | GLY | A | 29 | 4.204 | 16.151 | 40.013 | 1.00 | 16.80 | C |
| ATOM | 241 | O | GLY | A | 29 | 5.380 | 15.976 | 40.333 | 1.00 | 17.05 | O |
| ATOM | 242 | N | ILE | A | 30 | 3.790 | 16.122 | 38.745 | 1.00 | 15.60 | N |
| ATOM | 243 | CA | ILE | A | 30 | 4.697 | 15.790 | 37.662 | 1.00 | 15.93 | C |
| ATOM | 244 | C | ILE | A | 30 | 5.327 | 17.062 | 37.121 | 1.00 | 14.92 | C |
| ATOM | 245 | O | ILE | A | 30 | 4.635 | 18.046 | 36.943 | 1.00 | 13.94 | O |
| ATOM | 246 | CB | ILE | A | 30 | 3.977 | 15.027 | 36.536 | 1.00 | 16.51 | C |
| ATOM | 247 | CG1 | ILE | A | 30 | 3.507 | 13.668 | 37.067 | 1.00 | 17.71 | C |
| ATOM | 248 | CG2 | ILE | A | 30 | 4.895 | 14.822 | 35.319 | 1.00 | 15.75 | C |

| ATOM | 249 | CD1 | ILE | A | 30 | 2.619 | 12.918 | 36.106 | 1.00 | 19.02 | C |
|------|-----|-----|-----|---|----|-------|--------|--------|------|-------|---|
| ATOM | 250 | N   | THR | A | 31 | 6.635 | 17.012 | 36.848 | 1.00 | 14.50 | N |
| ATOM | 251 | CA  | THR | A | 31 | 7.321 | 18.106 | 36.167 | 1.00 | 13.62 | C |
| ATOM | 252 | C   | THR | A | 31 | 7.403 | 17.744 | 34.676 | 1.00 | 13.93 | C |
| ATOM | 253 | O   | THR | A | 31 | 8.097 | 16.798 | 34.298 | 1.00 | 12.11 | O |
| ATOM | 254 | CB  | THR | A | 31 | 8.732 | 18.371 | 36.724 | 1.00 | 14.02 | C |
| ATOM | 255 | OG1 | THR | A | 31 | 8.649 | 18.703 | 38.111 | 1.00 | 13.72 | O |
| ATOM | 256 | CG2 | THR | A | 31 | 9.399 | 19.514 | 36.018 | 1.00 | 13.98 | C |
| ATOM | 257 | N   | TYR | A | 32 | 6.663 | 18.485 | 33.847 | 1.00 | 12.14 | N |
| ATOM | 258 | CA  | TYR | A | 32 | 6.477 | 18.131 | 32.439 | 1.00 | 13.59 | C |
| ATOM | 259 | C   | TYR | A | 32 | 7.595 | 18.632 | 31.520 | 1.00 | 13.54 | C |
| ATOM | 260 | O   | TYR | A | 32 | 7.379 | 19.469 | 30.638 | 1.00 | 13.30 | O |
| ATOM | 261 | CB  | TYR | A | 32 | 5.102 | 18.605 | 31.953 | 1.00 | 14.31 | C |
| ATOM | 262 | CG  | TYR | A | 32 | 3.970 | 17.777 | 32.502 | 1.00 | 14.54 | C |
| ATOM | 263 | CD1 | TYR | A | 32 | 3.399 | 18.065 | 33.727 | 1.00 | 15.47 | C |
| ATOM | 264 | CD2 | TYR | A | 32 | 3.506 | 16.659 | 31.817 | 1.00 | 14.47 | C |
| ATOM | 265 | CE1 | TYR | A | 32 | 2.380 | 17.303 | 34.228 | 1.00 | 13.96 | C |
| ATOM | 266 | CE2 | TYR | A | 32 | 2.513 | 15.856 | 32.333 | 1.00 | 17.30 | C |
| ATOM | 267 | CZ  | TYR | A | 32 | 1.949 | 16.184 | 33.541 | 1.00 | 16.16 | C |
| ATOM | 268 | OH  | TYR | A | 32 | 0.928 | 15.445 | 34.054 | 1.00 | 16.05 | O |
| ATOM | 269 | N   | LEU | A | 33 | 8.777 | 18.087 | 31.752 | 1.00 | 15.06 | N |
| ATOM | 270 | CA  | LEU | A | 33 | 10.017 | 18.470 | 31.083 | 1.00 | 14.18 | C |
| ATOM | 271 | C   | LEU | A | 33 | 10.485 | 17.234 | 30.321 | 1.00 | 14.24 | C |
| ATOM | 272 | O   | LEU | A | 33 | 10.487 | 16.126 | 30.860 | 1.00 | 12.17 | O |
| ATOM | 273 | CB  | LEU | A | 33 | 11.067 | 18.924 | 32.121 | 1.00 | 14.45 | C |
| ATOM | 274 | CG  | LEU | A | 33 | 12.475 | 19.374 | 31.667 | 1.00 | 14.21 | C |
| ATOM | 275 | CD1 | LEU | A | 33 | 13.384 | 18.206 | 31.233 | 1.00 | 16.62 | C |
| ATOM | 276 | CD2 | LEU | A | 33 | 12.446 | 20.426 | 30.594 | 1.00 | 14.12 | C |
| ATOM | 277 | N   | TYR | A | 34 | 10.896 | 17.442 | 29.065 | 1.00 | 11.54 | N |
| ATOM | 278 | CA  | TYR | A | 34 | 11.271 | 16.347 | 28.184 | 1.00 | 12.25 | C |
| ATOM | 279 | C   | TYR | A | 34 | 12.605 | 16.648 | 27.563 | 1.00 | 12.40 | C |
| ATOM | 280 | O   | TYR | A | 34 | 12.915 | 17.805 | 27.322 | 1.00 | 12.64 | O |
| ATOM | 281 | CB  | TYR | A | 34 | 10.229 | 16.141 | 27.072 | 1.00 | 11.80 | C |
| ATOM | 282 | CG  | TYR | A | 34 | 8.883 | 15.699 | 27.546 | 1.00 | 12.99 | C |
| ATOM | 283 | CD1 | TYR | A | 34 | 8.099 | 16.550 | 28.343 | 1.00 | 14.09 | C |
| ATOM | 284 | CD2 | TYR | A | 34 | 8.352 | 14.457 | 27.198 | 1.00 | 13.23 | C |
| ATOM | 285 | CE1 | TYR | A | 34 | 6.854 | 16.141 | 28.820 | 1.00 | 14.56 | C |
| ATOM | 286 | CE2 | TYR | A | 34 | 7.108 | 14.055 | 27.660 | 1.00 | 14.89 | C |
| ATOM | 287 | CZ  | TYR | A | 34 | 6.363 | 14.903 | 28.468 | 1.00 | 13.55 | C |
| ATOM | 288 | OH  | TYR | A | 34 | 5.126 | 14.523 | 28.922 | 1.00 | 13.94 | O |
| ATOM | 289 | N   | TRP | A | 35 | 13.378 | 15.599 | 27.322 | 1.00 | 13.29 | N |
| ATOM | 290 | CA  | TRP | A | 35 | 14.613 | 15.701 | 26.549 | 1.00 | 13.60 | C |
| ATOM | 291 | C   | TRP | A | 35 | 14.540 | 14.820 | 25.290 | 1.00 | 14.47 | C |
| ATOM | 292 | O   | TRP | A | 35 | 14.099 | 13.662 | 25.352 | 1.00 | 15.34 | O |
| ATOM | 293 | CB  | TRP | A | 35 | 15.835 | 15.300 | 27.377 | 1.00 | 14.52 | C |
| ATOM | 294 | CG  | TRP | A | 35 | 16.211 | 16.184 | 28.569 | 1.00 | 14.71 | C |
| ATOM | 295 | CD1 | TRP | A | 35 | 15.926 | 15.958 | 29.894 | 1.00 | 14.46 | C |
| ATOM | 296 | CD2 | TRP | A | 35 | 16.993 | 17.389 | 28.530 | 1.00 | 14.00 | C |
| ATOM | 297 | NE1 | TRP | A | 35 | 16.465 | 16.963 | 30.671 | 1.00 | 14.53 | N |
| ATOM | 298 | CE2 | TRP | A | 35 | 17.124 | 17.849 | 29.859 | 1.00 | 14.09 | C |
| ATOM | 299 | CE3 | TRP | A | 35 | 17.583 | 18.128 | 27.500 | 1.00 | 15.36 | C |
| ATOM | 300 | CZ2 | TRP | A | 35 | 17.815 | 19.017 | 30.183 | 1.00 | 16.56 | C |
| ATOM | 301 | CZ3 | TRP | A | 35 | 18.285 | 19.299 | 27.827 | 1.00 | 15.38 | C |
| ATOM | 302 | CH2 | TRP | A | 35 | 18.405 | 19.718 | 29.158 | 1.00 | 14.87 | C |
| ATOM | 303 | N   | TYR | A | 36 | 14.998 | 15.387 | 24.171 | 1.00 | 14.30 | N |
| ATOM | 304 | CA  | TYR | A | 36 | 15.115 | 14.738 | 22.877 | 1.00 | 14.28 | C |
| ATOM | 305 | C   | TYR | A | 36 | 16.573 | 14.804 | 22.402 | 1.00 | 14.25 | C |
| ATOM | 306 | O   | TYR | A | 36 | 17.277 | 15.780 | 22.639 | 1.00 | 12.20 | O |
| ATOM | 307 | CB  | TYR | A | 36 | 14.202 | 15.418 | 21.815 | 1.00 | 14.77 | C |
| ATOM | 308 | CG  | TYR | A | 36 | 12.746 | 15.335 | 22.156 | 1.00 | 14.25 | C |
| ATOM | 309 | CD1 | TYR | A | 36 | 12.009 | 14.204 | 21.846 | 1.00 | 14.47 | C |
| ATOM | 310 | CD2 | TYR | A | 36 | 12.105 | 16.367 | 22.836 | 1.00 | 15.20 | C |
| ATOM | 311 | CE1 | TYR | A | 36 | 10.667 | 14.105 | 22.195 | 1.00 | 13.18 | C |
| ATOM | 312 | CE2 | TYR | A | 36 | 10.749 | 16.287 | 23.179 | 1.00 | 13.78 | C |
| ATOM | 313 | CZ  | TYR | A | 36 | 10.041 | 15.152 | 22.875 | 1.00 | 13.01 | C |

| ATOM | 314 | OH | TYR | A | 36 | 8.709 | 15.056 | 23.209 | 1.00 | 14.14 | O |
|------|-----|-----|-----|---|----|--------|--------|--------|------|-------|---|
| ATOM | 315 | N | LEU | A | 37 | 17.018 | 13.760 | 21.719 | 1.00 | 14.50 | N |
| ATOM | 316 | CA | LEU | A | 37 | 18.316 | 13.774 | 21.051 | 1.00 | 14.18 | C |
| ATOM | 317 | C | LEU | A | 37 | 18.088 | 13.673 | 19.551 | 1.00 | 13.98 | C |
| ATOM | 318 | O | LEU | A | 37 | 17.276 | 12.851 | 19.100 | 1.00 | 14.34 | O |
| ATOM | 319 | CB | LEU | A | 37 | 19.172 | 12.577 | 21.492 | 1.00 | 15.04 | C |
| ATOM | 320 | CG | LEU | A | 37 | 20.503 | 12.299 | 20.794 | 1.00 | 14.25 | C |
| ATOM | 321 | CD1 | LEU | A | 37 | 21.447 | 13.493 | 20.790 | 1.00 | 15.33 | C |
| ATOM | 322 | CD2 | LEU | A | 37 | 21.195 | 11.109 | 21.459 | 1.00 | 16.56 | C |
| ATOM | 323 | N | GLN | A | 38 | 18.799 | 14.524 | 18.817 | 1.00 | 14.00 | N |
| ATOM | 324 | CA | GLN | A | 38 | 18.886 | 14.460 | 17.362 | 1.00 | 14.06 | C |
| ATOM | 325 | C | GLN | A | 38 | 20.349 | 14.188 | 16.976 | 1.00 | 15.51 | C |
| ATOM | 326 | O | GLN | A | 38 | 21.216 | 15.076 | 17.045 | 1.00 | 15.12 | O |
| ATOM | 327 | CB | GLN | A | 38 | 18.384 | 15.758 | 16.735 | 1.00 | 14.04 | C |
| ATOM | 328 | CG | GLN | A | 38 | 18.257 | 15.665 | 15.237 | 1.00 | 14.82 | C |
| ATOM | 329 | CD | GLN | A | 38 | 17.563 | 16.842 | 14.590 | 1.00 | 16.81 | C |
| ATOM | 330 | OE1 | GLN | A | 38 | 17.558 | 17.965 | 15.113 | 1.00 | 20.02 | O |
| ATOM | 331 | NE2 | GLN | A | 38 | 16.930 | 16.579 | 13.478 | 1.00 | 18.97 | N |
| ATOM | 332 | N | LYS | A | 39 | 20.622 | 12.930 | 16.630 | 1.00 | 17.74 | N |
| ATOM | 333 | CA | LYS | A | 39 | 21.933 | 12.504 | 16.163 | 1.00 | 19.16 | C |
| ATOM | 334 | C | LYS | A | 39 | 22.164 | 13.008 | 14.731 | 1.00 | 19.93 | C |
| ATOM | 335 | O | LYS | A | 39 | 21.211 | 13.271 | 13.998 | 1.00 | 19.44 | O |
| ATOM | 336 | CB | LYS | A | 39 | 22.033 | 10.988 | 16.233 | 1.00 | 19.19 | C |
| ATOM | 337 | CG | LYS | A | 39 | 21.999 | 10.450 | 17.655 | 1.00 | 19.97 | C |
| ATOM | 338 | CD | LYS | A | 39 | 22.240 | 8.942 | 17.675 | 1.00 | 21.03 | C |
| ATOM | 339 | CE | LYS | A | 39 | 22.303 | 8.427 | 19.102 | 1.00 | 22.24 | C |
| ATOM | 340 | NZ | LYS | A | 39 | 22.602 | 6.972 | 19.196 | 1.00 | 23.29 | N |
| ATOM | 341 | N | PRO | A | 40 | 23.433 | 13.184 | 14.326 | 1.00 | 23.06 | N |
| ATOM | 342 | CA | PRO | A | 40 | 23.604 | 13.756 | 12.982 | 1.00 | 24.46 | C |
| ATOM | 343 | C | PRO | A | 40 | 22.932 | 12.935 | 11.874 | 1.00 | 24.97 | C |
| ATOM | 344 | O | PRO | A | 40 | 23.051 | 11.697 | 11.840 | 1.00 | 25.38 | O |
| ATOM | 345 | CB | PRO | A | 40 | 25.132 | 13.819 | 12.796 | 1.00 | 25.06 | C |
| ATOM | 346 | CG | PRO | A | 40 | 25.704 | 13.716 | 14.174 | 1.00 | 25.00 | C |
| ATOM | 347 | CD | PRO | A | 40 | 24.720 | 12.955 | 15.013 | 1.00 | 23.68 | C |
| ATOM | 348 | N | GLY | A | 41 | 22.190 | 13.638 | 11.015 | 1.00 | 24.57 | N |
| ATOM | 349 | CA | GLY | A | 41 | 21.424 | 13.029 | 9.933 | 1.00 | 25.64 | C |
| ATOM | 350 | C | GLY | A | 41 | 20.120 | 12.324 | 10.285 | 1.00 | 26.04 | C |
| ATOM | 351 | O | GLY | A | 41 | 19.488 | 11.727 | 9.406 | 1.00 | 27.17 | O |
| ATOM | 352 | N | GLN | A | 42 | 19.720 | 12.377 | 11.558 | 1.00 | 24.52 | N |
| ATOM | 353 | CA | GLN | A | 42 | 18.554 | 11.660 | 12.034 | 1.00 | 24.33 | C |
| ATOM | 354 | C | GLN | A | 42 | 17.447 | 12.622 | 12.474 | 1.00 | 20.98 | C |
| ATOM | 355 | O | GLN | A | 42 | 17.662 | 13.823 | 12.605 | 1.00 | 18.89 | O |
| ATOM | 356 | CB | GLN | A | 42 | 18.931 | 10.774 | 13.225 | 1.00 | 25.12 | C |
| ATOM | 357 | CG | GLN | A | 42 | 19.985 | 9.722 | 12.995 | 1.00 | 27.38 | C |
| ATOM | 358 | CD | GLN | A | 42 | 20.051 | 8.678 | 14.143 | 1.00 | 29.48 | C |
| ATOM | 359 | OE1 | GLN | A | 42 | 19.235 | 8.683 | 15.108 | 1.00 | 31.83 | O |
| ATOM | 360 | NE2 | GLN | A | 42 | 21.035 | 7.779 | 14.048 | 1.00 | 32.27 | N |
| ATOM | 361 | N | SER | A | 43 | 16.258 | 12.078 | 12.702 | 1.00 | 19.21 | N |
| ATOM | 362 | CA | SER | A | 43 | 15.190 | 12.811 | 13.384 | 1.00 | 19.56 | C |
| ATOM | 363 | C | SER | A | 43 | 15.449 | 12.898 | 14.899 | 1.00 | 18.73 | C |
| ATOM | 364 | O | SER | A | 43 | 16.225 | 12.111 | 15.453 | 1.00 | 19.07 | O |
| ATOM | 365 | CB | SER | A | 43 | 13.853 | 12.111 | 13.178 | 1.00 | 20.07 | C |
| ATOM | 366 | OG | SER | A | 43 | 13.572 | 11.960 | 11.808 | 1.00 | 22.01 | O |
| ATOM | 367 | N | PRO | A | 44 | 14.774 | 13.841 | 15.586 | 1.00 | 18.21 | N |
| ATOM | 368 | CA | PRO | A | 44 | 14.794 | 13.784 | 17.058 | 1.00 | 17.41 | C |
| ATOM | 369 | C | PRO | A | 44 | 14.199 | 12.469 | 17.576 | 1.00 | 17.20 | C |
| ATOM | 370 | O | PRO | A | 44 | 13.350 | 11.900 | 16.916 | 1.00 | 14.93 | O |
| ATOM | 371 | CB | PRO | A | 44 | 13.925 | 14.965 | 17.475 | 1.00 | 17.98 | C |
| ATOM | 372 | CG | PRO | A | 44 | 13.887 | 15.870 | 16.276 | 1.00 | 15.94 | C |
| ATOM | 373 | CD | PRO | A | 44 | 13.981 | 14.969 | 15.084 | 1.00 | 16.46 | C |
| ATOM | 374 | N | HIS | A | 45 | 14.690 | 11.977 | 18.715 | 1.00 | 17.62 | N |
| ATOM | 375 | CA | HIS | A | 45 | 14.050 | 10.851 | 19.413 | 1.00 | 17.91 | C |
| ATOM | 376 | C | HIS | A | 45 | 13.991 | 11.173 | 20.911 | 1.00 | 17.33 | C |
| ATOM | 377 | O | HIS | A | 45 | 14.853 | 11.890 | 21.445 | 1.00 | 16.46 | O |
| ATOM | 378 | CB | HIS | A | 45 | 14.703 | 9.468 | 19.139 | 1.00 | 18.48 | C |

| ATOM | 379 | CG | HIS | A | 45 | 16.087 | 9.312 | 19.700 | 1.00 | 19.61 | C |
|------|-----|-----|-----|---|----|--------|-------|--------|------|-------|---|
| ATOM | 380 | ND1 | HIS | A | 45 | 17.212 | 9.768 | 19.042 | 1.00 | 19.83 | N |
| ATOM | 381 | CD2 | HIS | A | 45 | 16.528 | 8.755 | 20.858 | 1.00 | 20.59 | C |
| ATOM | 382 | CE1 | HIS | A | 45 | 18.288 | 9.489 | 19.762 | 1.00 | 21.06 | C |
| ATOM | 383 | NE2 | HIS | A | 45 | 17.899 | 8.876 | 20.870 | 1.00 | 22.14 | N |
| ATOM | 384 | N | LEU | A | 46 | 12.924 | 10.696 | 21.540 | 1.00 | 16.48 | N |
| ATOM | 385 | CA | LEU | A | 46 | 12.650 | 10.935 | 22.953 | 1.00 | 15.89 | C |
| ATOM | 386 | C | LEU | A | 46 | 13.692 | 10.208 | 23.778 | 1.00 | 16.91 | C |
| ATOM | 387 | O | LEU | A | 46 | 13.892 | 8.997 | 23.586 | 1.00 | 15.48 | O |
| ATOM | 388 | CB | LEU | A | 46 | 11.225 | 10.427 | 23.325 | 1.00 | 15.34 | C |
| ATOM | 389 | CG | LEU | A | 46 | 10.804 | 10.583 | 24.790 | 1.00 | 16.07 | C |
| ATOM | 390 | CD1 | LEU | A | 46 | 10.737 | 12.059 | 25.151 | 1.00 | 13.79 | C |
| ATOM | 391 | CD2 | LEU | A | 46 | 9.478 | 9.928 | 25.092 | 1.00 | 15.31 | C |
| ATOM | 392 | N | LEU | A | 47 | 14.324 | 10.931 | 24.699 | 1.00 | 16.25 | N |
| ATOM | 393 | CA | LEU | A | 47 | 15.242 | 10.347 | 25.692 | 1.00 | 18.25 | C |
| ATOM | 394 | C | LEU | A | 47 | 14.589 | 10.250 | 27.072 | 1.00 | 18.27 | C |
| ATOM | 395 | O | LEU | A | 47 | 14.594 | 9.198 | 27.732 | 1.00 | 19.20 | O |
| ATOM | 396 | CB | LEU | A | 47 | 16.455 | 11.249 | 25.874 | 1.00 | 19.62 | C |
| ATOM | 397 | CG | LEU | A | 47 | 17.712 | 11.223 | 25.048 | 1.00 | 23.79 | C |
| ATOM | 398 | CD1 | LEU | A | 47 | 18.730 | 12.006 | 25.827 | 1.00 | 26.06 | C |
| ATOM | 399 | CD2 | LEU | A | 47 | 18.175 | 9.820 | 24.822 | 1.00 | 24.36 | C |
| ATOM | 400 | N | ILE | A | 48 | 14.069 | 11.392 | 27.527 | 1.00 | 17.51 | N |
| ATOM | 401 | CA | ILE | A | 48 | 13.553 | 11.527 | 28.873 | 1.00 | 18.33 | C |
| ATOM | 402 | C | ILE | A | 48 | 12.171 | 12.174 | 28.836 | 1.00 | 17.10 | C |
| ATOM | 403 | O | ILE | A | 48 | 11.985 | 13.140 | 28.131 | 1.00 | 15.28 | O |
| ATOM | 404 | CB | ILE | A | 48 | 14.464 | 12.452 | 29.731 | 1.00 | 18.32 | C |
| ATOM | 405 | CG1 | ILE | A | 48 | 15.911 | 11.951 | 29.820 | 1.00 | 18.93 | C |
| ATOM | 406 | CG2 | ILE | A | 48 | 13.860 | 12.681 | 31.126 | 1.00 | 18.46 | C |
| ATOM | 407 | CD1 | ILE | A | 48 | 16.094 | 10.667 | 30.554 | 1.00 | 21.32 | C |
| ATOM | 408 | N | TYR | A | 49 | 11.221 | 11.668 | 29.623 | 1.00 | 17.28 | N |
| ATOM | 409 | CA | TYR | A | 49 | 9.920 | 12.335 | 29.766 | 1.00 | 17.24 | C |
| ATOM | 410 | C | TYR | A | 49 | 9.608 | 12.527 | 31.226 | 1.00 | 17.78 | C |
| ATOM | 411 | O | TYR | A | 49 | 10.168 | 11.846 | 32.090 | 1.00 | 17.83 | O |
| ATOM | 412 | CB | TYR | A | 49 | 8.770 | 11.530 | 29.121 | 1.00 | 18.71 | C |
| ATOM | 413 | CG | TYR | A | 49 | 8.539 | 10.155 | 29.732 | 1.00 | 20.18 | C |
| ATOM | 414 | CD1 | TYR | A | 49 | 9.324 | 9.072 | 29.366 | 1.00 | 20.67 | C |
| ATOM | 415 | CD2 | TYR | A | 49 | 7.513 | 9.940 | 30.651 | 1.00 | 20.54 | C |
| ATOM | 416 | CE1 | TYR | A | 49 | 9.113 | 7.816 | 29.898 | 1.00 | 20.86 | C |
| ATOM | 417 | CE2 | TYR | A | 49 | 7.293 | 8.678 | 31.185 | 1.00 | 21.58 | C |
| ATOM | 418 | CZ | TYR | A | 49 | 8.092 | 7.625 | 30.801 | 1.00 | 21.14 | C |
| ATOM | 419 | OH | TYR | A | 49 | 7.901 | 6.362 | 31.332 | 1.00 | 24.12 | O |
| ATOM | 420 | N | HIS | A | 50 | 8.704 | 13.459 | 31.489 | 1.00 | 16.99 | N |
| ATOM | 421 | CA | HIS | A | 50 | 8.306 | 13.788 | 32.853 | 1.00 | 16.78 | C |
| ATOM | 422 | C | HIS | A | 50 | 9.513 | 13.988 | 33.777 | 1.00 | 16.19 | C |
| ATOM | 423 | O | HIS | A | 50 | 9.570 | 13.425 | 34.885 | 1.00 | 16.74 | O |
| ATOM | 424 | CB | HIS | A | 50 | 7.394 | 12.713 | 33.438 | 1.00 | 16.22 | C |
| ATOM | 425 | CG | HIS | A | 50 | 6.061 | 12.605 | 32.776 | 1.00 | 17.81 | C |
| ATOM | 426 | ND1 | HIS | A | 50 | 5.206 | 11.555 | 33.020 | 1.00 | 17.06 | N |
| ATOM | 427 | CD2 | HIS | A | 50 | 5.418 | 13.423 | 31.902 | 1.00 | 17.06 | C |
| ATOM | 428 | CE1 | HIS | A | 50 | 4.101 | 11.719 | 32.308 | 1.00 | 18.35 | C |
| ATOM | 429 | NE2 | HIS | A | 50 | 4.211 | 12.838 | 31.615 | 1.00 | 16.69 | N |
| ATOM | 430 | N | LEU | A | 51 | 10.494 | 14.741 | 33.280 | 1.00 | 16.79 | N |
| ATOM | 431 | CA | LEU | A | 51 | 11.691 | 15.181 | 34.014 | 1.00 | 16.70 | C |
| ATOM | 432 | C | LEU | A | 51 | 12.756 | 14.125 | 34.242 | 1.00 | 16.76 | C |
| ATOM | 433 | O | LEU | A | 51 | 13.931 | 14.408 | 34.043 | 1.00 | 17.41 | O |
| ATOM | 434 | CB | LEU | A | 51 | 11.334 | 15.838 | 35.357 | 1.00 | 16.14 | C |
| ATOM | 435 | CG | LEU | A | 51 | 12.537 | 16.472 | 36.097 | 1.00 | 16.82 | C |
| ATOM | 436 | CD1 | LEU | A | 51 | 13.194 | 17.540 | 35.263 | 1.00 | 17.64 | C |
| ATOM | 437 | CD2 | LEU | A | 51 | 12.140 | 17.029 | 37.460 | 1.00 | 17.60 | C |
| ATOM | 438 | N | SER | A | 52 | 12.363 | 12.918 | 34.647 | 1.00 | 16.64 | N |
| ATOM | 439 | CA | SER | A | 52 | 13.339 | 11.921 | 35.114 | 1.00 | 16.40 | C |
| ATOM | 440 | C | SER | A | 52 | 13.144 | 10.518 | 34.592 | 1.00 | 17.44 | C |
| ATOM | 441 | O | SER | A | 52 | 13.909 | 9.639 | 34.939 | 1.00 | 16.31 | O |
| ATOM | 442 | CB | SER | A | 52 | 13.278 | 11.878 | 36.639 | 1.00 | 17.17 | C |
| ATOM | 443 | OG | SER | A | 52 | 13.677 | 13.126 | 37.161 | 1.00 | 19.05 | O |

| ATOM | 444 | N | ASN | A | 53 | 12.137 | 10.289 | 33.770 | 1.00 | 17.48 | N |
|------|-----|-----|-----|---|----|--------|--------|--------|------|-------|---|
| ATOM | 445 | CA | ASN | A | 53 | 11.836 | 8.923 | 33.320 | 1.00 | 18.90 | C |
| ATOM | 446 | C | ASN | A | 53 | 12.461 | 8.619 | 31.975 | 1.00 | 20.77 | C |
| ATOM | 447 | O | ASN | A | 53 | 12.286 | 9.370 | 31.027 | 1.00 | 20.06 | O |
| ATOM | 448 | CB | ASN | A | 53 | 10.337 | 8.731 | 33.269 | 1.00 | 19.90 | C |
| ATOM | 449 | CG | ASN | A | 53 | 9.713 | 8.885 | 34.627 | 1.00 | 23.30 | C |
| ATOM | 450 | OD1 | ASN | A | 53 | 9.860 | 8.012 | 35.474 | 1.00 | 26.30 | O |
| ATOM | 451 | ND2 | ASN | A | 53 | 9.131 | 10.037 | 34.887 | 1.00 | 25.35 | N |
| ATOM | 452 | N | LEU | A | 54 | 13.166 | 7.495 | 31.888 | 1.00 | 20.90 | N |
| ATOM | 453 | CA | LEU | A | 54 | 13.878 | 7.147 | 30.665 | 1.00 | 21.26 | C |
| ATOM | 454 | C | LEU | A | 54 | 12.915 | 6.540 | 29.663 | 1.00 | 21.78 | C |
| ATOM | 455 | O | LEU | A | 54 | 12.019 | 5.753 | 30.018 | 1.00 | 19.07 | O |
| ATOM | 456 | CB | LEU | A | 54 | 15.058 | 6.206 | 30.958 | 1.00 | 21.81 | C |
| ATOM | 457 | CG | LEU | A | 54 | 16.409 | 6.853 | 31.305 | 1.00 | 23.28 | C |
| ATOM | 458 | CD1 | LEU | A | 54 | 16.388 | 7.610 | 32.639 | 1.00 | 25.60 | C |
| ATOM | 459 | CD2 | LEU | A | 54 | 17.498 | 5.792 | 31.347 | 1.00 | 23.53 | C |
| ATOM | 460 | N | ALA | A | 55 | 13.071 | 6.925 | 28.401 | 1.00 | 21.47 | N |
| ATOM | 461 | CA | ALA | A | 55 | 12.276 | 6.318 | 27.361 | 1.00 | 23.19 | C |
| ATOM | 462 | C | ALA | A | 55 | 12.771 | 4.883 | 27.172 | 1.00 | 24.46 | C |
| ATOM | 463 | O | ALA | A | 55 | 13.902 | 4.552 | 27.521 | 1.00 | 23.07 | O |
| ATOM | 464 | CB | ALA | A | 55 | 12.373 | 7.126 | 26.060 | 1.00 | 23.97 | C |
| ATOM | 465 | N | SER | A | 56 | 11.906 | 4.059 | 26.613 | 1.00 | 27.16 | N |
| ATOM | 466 | CA | SER | A | 56 | 12.198 | 2.646 | 26.406 | 1.00 | 29.31 | C |
| ATOM | 467 | C | SER | A | 56 | 13.414 | 2.510 | 25.500 | 1.00 | 29.71 | C |
| ATOM | 468 | O | SER | A | 56 | 13.490 | 3.178 | 24.468 | 1.00 | 29.45 | O |
| ATOM | 469 | CB | SER | A | 56 | 10.967 | 1.978 | 25.796 | 1.00 | 30.78 | C |
| ATOM | 470 | OG | SER | A | 56 | 11.175 | 0.599 | 25.583 | 1.00 | 33.13 | O |
| ATOM | 471 | N | GLY | A | 57 | 14.386 | 1.693 | 25.911 | 1.00 | 29.75 | N |
| ATOM | 472 | CA | GLY | A | 57 | 15.626 | 1.500 | 25.138 | 1.00 | 29.36 | C |
| ATOM | 473 | C | GLY | A | 57 | 16.767 | 2.452 | 25.476 | 1.00 | 28.89 | C |
| ATOM | 474 | O | GLY | A | 57 | 17.938 | 2.186 | 25.155 | 1.00 | 27.79 | O |
| ATOM | 475 | N | VAL | A | 58 | 16.454 | 3.581 | 26.111 | 1.00 | 27.72 | N |
| ATOM | 476 | CA | VAL | A | 58 | 17.484 | 4.525 | 26.492 | 1.00 | 26.80 | C |
| ATOM | 477 | C | VAL | A | 58 | 18.356 | 3.934 | 27.614 | 1.00 | 27.26 | C |
| ATOM | 478 | O | VAL | A | 58 | 17.817 | 3.487 | 28.617 | 1.00 | 27.99 | O |
| ATOM | 479 | CB | VAL | A | 58 | 16.873 | 5.871 | 26.953 | 1.00 | 26.13 | C |
| ATOM | 480 | CG1 | VAL | A | 58 | 17.961 | 6.792 | 27.427 | 1.00 | 25.90 | C |
| ATOM | 481 | CG2 | VAL | A | 58 | 16.093 | 6.492 | 25.813 | 1.00 | 24.39 | C |
| ATOM | 482 | N | PRO | A | 59 | 19.689 | 3.914 | 27.432 | 1.00 | 28.80 | N |
| ATOM | 483 | CA | PRO | A | 59 | 20.671 | 3.436 | 28.439 | 1.00 | 29.03 | C |
| ATOM | 484 | C | PRO | A | 59 | 20.606 | 4.178 | 29.763 | 1.00 | 29.94 | C |
| ATOM | 485 | O | PRO | A | 59 | 20.274 | 5.348 | 29.777 | 1.00 | 29.03 | O |
| ATOM | 486 | CB | PRO | A | 59 | 22.022 | 3.719 | 27.776 | 1.00 | 28.85 | C |
| ATOM | 487 | CG | PRO | A | 59 | 21.772 | 3.841 | 26.377 | 1.00 | 29.69 | C |
| ATOM | 488 | CD | PRO | A | 59 | 20.362 | 4.303 | 26.178 | 1.00 | 29.49 | C |
| ATOM | 489 | N | ASP | A | 60 | 20.945 | 3.527 | 30.875 | 1.00 | 31.72 | N |
| ATOM | 490 | CA | ASP | A | 60 | 20.830 | 4.204 | 32.189 | 1.00 | 32.60 | C |
| ATOM | 491 | C | ASP | A | 60 | 22.020 | 5.091 | 32.527 | 1.00 | 30.82 | C |
| ATOM | 492 | O | ASP | A | 60 | 22.091 | 5.672 | 33.608 | 1.00 | 31.47 | O |
| ATOM | 493 | CB | ASP | A | 60 | 20.535 | 3.211 | 33.325 | 1.00 | 34.71 | C |
| ATOM | 494 | CG | ASP | A | 60 | 21.514 | 2.083 | 33.378 | 1.00 | 37.22 | C |
| ATOM | 495 | OD1 | ASP | A | 60 | 22.716 | 2.317 | 33.097 | 1.00 | 40.13 | O |
| ATOM | 496 | OD2 | ASP | A | 60 | 21.070 | 0.952 | 33.697 | 1.00 | 42.09 | O |
| ATOM | 497 | N | ARG | A | 61 | 22.940 | 5.210 | 31.578 | 1.00 | 28.70 | N |
| ATOM | 498 | CA | ARG | A | 61 | 23.921 | 6.279 | 31.567 | 1.00 | 27.19 | C |
| ATOM | 499 | C | ARG | A | 61 | 23.241 | 7.659 | 31.527 | 1.00 | 24.05 | C |
| ATOM | 500 | O | ARG | A | 61 | 23.795 | 8.645 | 32.011 | 1.00 | 22.46 | O |
| ATOM | 501 | CB | ARG | A | 61 | 24.818 | 6.158 | 30.333 | 1.00 | 28.32 | C |
| ATOM | 502 | CG | ARG | A | 61 | 25.386 | 4.770 | 30.110 | 1.00 | 30.41 | C |
| ATOM | 503 | CD | ARG | A | 61 | 26.484 | 4.769 | 29.071 | 1.00 | 30.25 | C |
| ATOM | 504 | NE | ARG | A | 61 | 25.989 | 4.808 | 27.691 | 1.00 | 32.08 | N |
| ATOM | 505 | CZ | ARG | A | 61 | 25.966 | 5.881 | 26.911 | 1.00 | 27.50 | C |
| ATOM | 506 | NH1 | ARG | A | 61 | 26.391 | 7.044 | 27.356 | 1.00 | 29.75 | N |
| ATOM | 507 | NH2 | ARG | A | 61 | 25.524 | 5.765 | 25.674 | 1.00 | 28.68 | N |
| ATOM | 508 | N | PHE | A | 62 | 22.051 | 7.716 | 30.930 | 1.00 | 22.86 | N |

| | | | | | | | | | | | |
|---|---|---|---|---|---|---|---|---|---|---|---|
| ATOM | 509 | CA | PHE | A | 62 | 21.264 | 8.942 | 30.900 | 1.00 | 20.74 | C |
| ATOM | 510 | C | PHE | A | 62 | 20.398 | 9.095 | 32.161 | 1.00 | 19.24 | C |
| ATOM | 511 | O | PHE | A | 62 | 19.795 | 8.138 | 32.628 | 1.00 | 20.24 | O |
| ATOM | 512 | CB | PHE | A | 62 | 20.400 | 8.982 | 29.639 | 1.00 | 20.95 | C |
| ATOM | 513 | CG | PHE | A | 62 | 21.192 | 9.040 | 28.370 | 1.00 | 21.08 | C |
| ATOM | 514 | CD1 | PHE | A | 62 | 21.615 | 7.875 | 27.737 | 1.00 | 21.81 | C |
| ATOM | 515 | CD2 | PHE | A | 62 | 21.530 | 10.260 | 27.802 | 1.00 | 22.10 | C |
| ATOM | 516 | CE1 | PHE | A | 62 | 22.363 | 7.945 | 26.558 | 1.00 | 20.11 | C |
| ATOM | 517 | CE2 | PHE | A | 62 | 22.278 | 10.325 | 26.627 | 1.00 | 20.45 | C |
| ATOM | 518 | CZ | PHE | A | 62 | 22.689 | 9.163 | 26.014 | 1.00 | 21.24 | C |
| ATOM | 519 | N | SER | A | 63 | 20.376 | 10.297 | 32.717 | 1.00 | 17.00 | N |
| ATOM | 520 | CA | SER | A | 63 | 19.549 | 10.617 | 33.886 | 1.00 | 15.75 | C |
| ATOM | 521 | C | SER | A | 63 | 19.202 | 12.094 | 33.844 | 1.00 | 15.79 | C |
| ATOM | 522 | O | SER | A | 63 | 19.890 | 12.865 | 33.187 | 1.00 | 15.34 | O |
| ATOM | 523 | CB | SER | A | 63 | 20.268 | 10.257 | 35.203 | 1.00 | 15.35 | C |
| ATOM | 524 | OG | SER | A | 63 | 21.354 | 11.119 | 35.468 | 1.00 | 17.41 | O |
| ATOM | 525 | N | SER | A | 64 | 18.107 | 12.498 | 34.481 | 1.00 | 14.13 | N |
| ATOM | 526 | CA | SER | A | 64 | 17.737 | 13.919 | 34.468 | 1.00 | 15.19 | C |
| ATOM | 527 | C | SER | A | 64 | 17.067 | 14.323 | 35.772 | 1.00 | 14.85 | C |
| ATOM | 528 | O | SER | A | 64 | 16.377 | 13.533 | 36.396 | 1.00 | 14.33 | O |
| ATOM | 529 | CB | SER | A | 64 | 16.825 | 14.189 | 33.263 | 1.00 | 15.41 | C |
| ATOM | 530 | OG | SER | A | 64 | 16.295 | 15.512 | 33.257 | 1.00 | 16.02 | O |
| ATOM | 531 | N | SER | A | 65 | 17.290 | 15.565 | 36.175 | 1.00 | 15.44 | N |
| ATOM | 532 | CA | SER | A | 65 | 16.740 | 16.070 | 37.402 | 1.00 | 14.63 | C |
| ATOM | 533 | C | SER | A | 65 | 16.412 | 17.533 | 37.211 | 1.00 | 14.41 | C |
| ATOM | 534 | O | SER | A | 65 | 16.872 | 18.171 | 36.258 | 1.00 | 12.59 | O |
| ATOM | 535 | CB | SER | A | 65 | 17.744 | 15.897 | 38.544 | 1.00 | 15.96 | C |
| ATOM | 536 | OG | SER | A | 65 | 18.930 | 16.646 | 38.316 | 1.00 | 14.77 | O |
| ATOM | 537 | N | GLY | A | 66 | 15.607 | 18.070 | 38.112 | 1.00 | 12.65 | N |
| ATOM | 538 | CA | GLY | A | 66 | 15.352 | 19.491 | 38.066 | 1.00 | 11.76 | C |
| ATOM | 539 | C | GLY | A | 66 | 14.191 | 19.936 | 38.922 | 1.00 | 11.99 | C |
| ATOM | 540 | O | GLY | A | 66 | 13.459 | 19.124 | 39.510 | 1.00 | 11.02 | O |
| ATOM | 541 | N | SER | A | 67 | 14.056 | 21.245 | 39.003 | 1.00 | 12.45 | N |
| ATOM | 542 | CA | SER | A | 67 | 12.931 | 21.879 | 39.660 | 1.00 | 13.44 | C |
| ATOM | 543 | C | SER | A | 67 | 11.792 | 22.111 | 38.654 | 1.00 | 15.01 | C |
| ATOM | 544 | O | SER | A | 67 | 11.749 | 21.462 | 37.610 | 1.00 | 16.90 | O |
| ATOM | 545 | CB | SER | A | 67 | 13.376 | 23.206 | 40.283 | 1.00 | 13.74 | C |
| ATOM | 546 | OG | SER | A | 67 | 13.758 | 24.139 | 39.286 | 1.00 | 12.33 | O |
| ATOM | 547 | N | GLY | A | 68 | 10.855 | 23.005 | 38.956 | 1.00 | 15.65 | N |
| ATOM | 548 | CA | GLY | A | 68 | 9.913 | 23.453 | 37.930 | 1.00 | 15.47 | C |
| ATOM | 549 | C | GLY | A | 68 | 10.534 | 24.298 | 36.806 | 1.00 | 15.50 | C |
| ATOM | 550 | O | GLY | A | 68 | 9.939 | 24.431 | 35.753 | 1.00 | 16.27 | O |
| ATOM | 551 | N | THR | A | 69 | 11.700 | 24.894 | 37.033 | 1.00 | 14.76 | N |
| ATOM | 552 | CA | THR | A | 69 | 12.276 | 25.861 | 36.117 | 1.00 | 15.70 | C |
| ATOM | 553 | C | THR | A | 69 | 13.709 | 25.636 | 35.676 | 1.00 | 15.78 | C |
| ATOM | 554 | O | THR | A | 69 | 14.141 | 26.276 | 34.710 | 1.00 | 15.42 | O |
| ATOM | 555 | CB | THR | A | 69 | 12.230 | 27.271 | 36.727 | 1.00 | 16.78 | C |
| ATOM | 556 | OG1 | THR | A | 69 | 13.024 | 27.320 | 37.933 | 1.00 | 19.49 | O |
| ATOM | 557 | CG2 | THR | A | 69 | 10.806 | 27.640 | 37.020 | 1.00 | 18.16 | C |
| ATOM | 558 | N | ASP | A | 70 | 14.461 | 24.794 | 36.395 | 1.00 | 16.45 | N |
| ATOM | 559 | CA | ASP | A | 70 | 15.870 | 24.506 | 36.065 | 1.00 | 16.10 | C |
| ATOM | 560 | C | ASP | A | 70 | 16.112 | 23.014 | 36.034 | 1.00 | 15.88 | C |
| ATOM | 561 | O | ASP | A | 70 | 15.746 | 22.302 | 36.969 | 1.00 | 14.32 | O |
| ATOM | 562 | CB | ASP | A | 70 | 16.816 | 25.124 | 37.088 | 1.00 | 18.83 | C |
| ATOM | 563 | CG | ASP | A | 70 | 16.714 | 26.612 | 37.153 | 1.00 | 20.11 | C |
| ATOM | 564 | OD1 | ASP | A | 70 | 17.167 | 27.305 | 36.224 | 1.00 | 22.86 | O |
| ATOM | 565 | OD2 | ASP | A | 70 | 16.167 | 27.116 | 38.150 | 1.00 | 25.52 | O |
| ATOM | 566 | N | PHE | A | 71 | 16.765 | 22.555 | 34.972 | 1.00 | 14.13 | N |
| ATOM | 567 | CA | PHE | A | 71 | 16.861 | 21.127 | 34.658 | 1.00 | 14.76 | C |
| ATOM | 568 | C | PHE | A | 71 | 18.263 | 20.747 | 34.210 | 1.00 | 15.08 | C |
| ATOM | 569 | O | PHE | A | 71 | 18.979 | 21.554 | 33.634 | 1.00 | 15.06 | O |
| ATOM | 570 | CB | PHE | A | 71 | 15.851 | 20.752 | 33.570 | 1.00 | 16.58 | C |
| ATOM | 571 | CG | PHE | A | 71 | 14.582 | 21.578 | 33.607 | 1.00 | 14.66 | C |
| ATOM | 572 | CD1 | PHE | A | 71 | 13.599 | 21.341 | 34.578 | 1.00 | 13.56 | C |
| ATOM | 573 | CD2 | PHE | A | 71 | 14.371 | 22.597 | 32.678 | 1.00 | 16.23 | C |

```
ATOM    574   CE1 PHE A   71      12.433  22.094  34.610  1.00 14.53          C
ATOM    575   CE2 PHE A   71      13.219  23.377  32.727  1.00 14.60          C
ATOM    576   CZ  PHE A   71      12.247  23.132  33.688  1.00 16.09          C
ATOM    577   N   THR A   72      18.643  19.503  34.487  1.00 14.78          N
ATOM    578   CA  THR A   72      19.959  19.023  34.145  1.00 14.80          C
ATOM    579   C   THR A   72      19.889  17.616  33.618  1.00 15.53          C
ATOM    580   O   THR A   72      19.451  16.720  34.325  1.00 15.01          O
ATOM    581   CB  THR A   72      20.889  19.003  35.396  1.00 15.47          C
ATOM    582   OG1 THR A   72      20.950  20.310  35.989  1.00 14.61          O
ATOM    583   CG2 THR A   72      22.274  18.567  35.001  1.00 15.29          C
ATOM    584   N   LEU A   73      20.390  17.421  32.400  1.00 14.94          N
ATOM    585   CA  LEU A   73      20.603  16.101  31.825  1.00 16.32          C
ATOM    586   C   LEU A   73      22.049  15.658  32.091  1.00 16.41          C
ATOM    587   O   LEU A   73      22.976  16.393  31.817  1.00 16.65          O
ATOM    588   CB  LEU A   73      20.359  16.157  30.313  1.00 16.26          C
ATOM    589   CG  LEU A   73      20.613  14.886  29.525  1.00 16.71          C
ATOM    590   CD1 LEU A   73      19.640  13.797  29.932  1.00 17.69          C
ATOM    591   CD2 LEU A   73      20.547  15.149  28.027  1.00 17.73          C
ATOM    592   N   ARG A   74      22.230  14.438  32.590  1.00 18.29          N
ATOM    593   CA  ARG A   74      23.563  13.914  32.833  1.00 18.19          C
ATOM    594   C   ARG A   74      23.735  12.656  32.009  1.00 18.06          C
ATOM    595   O   ARG A   74      22.819  11.837  31.941  1.00 16.19          O
ATOM    596   CB AARG A   74      23.833  13.628  34.324  0.50 18.67          C
ATOM    597   CB BARG A   74      23.721  13.615  34.330  0.50 18.28          C
ATOM    598   CG AARG A   74      25.260  13.097  34.577  0.50 19.17          C
ATOM    599   CG BARG A   74      24.986  12.880  34.700  0.50 18.82          C
ATOM    600   CD AARG A   74      25.707  13.111  36.043  0.50 20.14          C
ATOM    601   CD BARG A   74      25.114  12.661  36.199  0.50 19.13          C
ATOM    602   NE AARG A   74      26.960  12.360  36.248  0.50 20.77          N
ATOM    603   NE BARG A   74      26.411  12.060  36.526  0.50 20.79          N
ATOM    604   CZ AARG A   74      27.042  11.038  36.462  0.50 21.34          C
ATOM    605   CZ BARG A   74      27.572  12.713  36.549  0.50 19.11          C
ATOM    606   NH1AARG A   74      25.953  10.274  36.507  0.50 21.43          N
ATOM    607   NH1BARG A   74      27.628  14.010  36.277  0.50 18.72          N
ATOM    608   NH2AARG A   74      28.233  10.471  36.616  0.50 22.27          N
ATOM    609   NH2BARG A   74      28.690  12.062  36.842  0.50 19.76          N
ATOM    610   N   ILE A   75      24.884  12.558  31.345  1.00 19.23          N
ATOM    611   CA  ILE A   75      25.317  11.338  30.668  1.00 22.64          C
ATOM    612   C   ILE A   75      26.601  10.938  31.370  1.00 23.97          C
ATOM    613   O   ILE A   75      27.564  11.682  31.350  1.00 24.47          O
ATOM    614   CB  ILE A   75      25.591  11.557  29.165  1.00 22.98          C
ATOM    615   CG1 ILE A   75      24.494  12.416  28.528  1.00 23.53          C
ATOM    616   CG2 ILE A   75      25.667  10.200  28.453  1.00 24.37          C
ATOM    617   CD1 ILE A   75      24.847  12.885  27.090  1.00 24.96          C
ATOM    618   N   SER A   76      26.595   9.777  32.018  1.00 26.77          N
ATOM    619   CA  SER A   76      27.589   9.448  33.047  1.00 28.92          C
ATOM    620   C   SER A   76      28.953   9.033  32.501  1.00 31.06          C
ATOM    621   O   SER A   76      30.004   9.375  33.103  1.00 32.82          O
ATOM    622   CB  SER A   76      27.049   8.333  33.945  1.00 28.02          C
ATOM    623   OG  SER A   76      26.786   7.180  33.187  1.00 28.28          O
ATOM    624   N   ARG A   77      28.920   8.266  31.409  1.00 31.54          N
ATOM    625   CA  ARG A   77      30.113   7.730  30.744  1.00 32.77          C
ATOM    626   C   ARG A   77      29.906   7.776  29.231  1.00 30.23          C
ATOM    627   O   ARG A   77      29.548   6.771  28.595  1.00 31.50          O
ATOM    628   CB  ARG A   77      30.356   6.288  31.153  1.00 33.28          C
ATOM    629   CG  ARG A   77      30.983   6.137  32.522  1.00 36.01          C
ATOM    630   CD  ARG A   77      30.856   4.711  33.010  1.00 37.98          C
ATOM    631   NE  ARG A   77      29.637   4.476  33.787  1.00 41.14          N
ATOM    632   CZ  ARG A   77      29.445   4.849  35.060  1.00 42.81          C
ATOM    633   NH1 ARG A   77      30.370   5.534  35.738  1.00 43.85          N
ATOM    634   NH2 ARG A   77      28.290   4.554  35.657  1.00 43.48          N
ATOM    635   N   VAL A   78      30.156   8.950  28.684  1.00 28.80          N
ATOM    636   CA  VAL A   78      29.802   9.292  27.321  1.00 27.69          C
ATOM    637   C   VAL A   78      30.472   8.334  26.330  1.00 27.72          C
ATOM    638   O   VAL A   78      31.625   7.932  26.514  1.00 26.01          O
```

| ATOM | 639 | CB | VAL | A | 78 | 30.183 | 10.767 | 27.053 | 1.00 | 28.64 | C |
|------|-----|-----|-----|---|----|--------|--------|--------|------|-------|---|
| ATOM | 640 | CG1 | VAL | A | 78 | 30.243 | 11.086 | 25.585 | 1.00 | 29.31 | C |
| ATOM | 641 | CG2 | VAL | A | 78 | 29.177 | 11.689 | 27.740 | 1.00 | 29.09 | C |
| ATOM | 642 | N | GLU | A | 79 | 29.717 | 7.942 | 25.308 | 1.00 | 26.70 | N |
| ATOM | 643 | CA | GLU | A | 79 | 30.235 | 7.136 | 24.212 | 1.00 | 26.37 | C |
| ATOM | 644 | C | GLU | A | 79 | 30.204 | 7.952 | 22.920 | 1.00 | 24.14 | C |
| ATOM | 645 | O | GLU | A | 79 | 29.517 | 8.962 | 22.858 | 1.00 | 22.29 | O |
| ATOM | 646 | CB | GLU | A | 79 | 29.416 | 5.869 | 24.093 | 1.00 | 27.09 | C |
| ATOM | 647 | CG | GLU | A | 79 | 29.472 | 5.051 | 25.372 | 1.00 | 30.10 | C |
| ATOM | 648 | CD | GLU | A | 79 | 28.587 | 3.837 | 25.374 | 1.00 | 31.16 | C |
| ATOM | 649 | OE1 | GLU | A | 79 | 27.866 | 3.596 | 24.382 | 1.00 | 37.08 | O |
| ATOM | 650 | OE2 | GLU | A | 79 | 28.608 | 3.106 | 26.390 | 1.00 | 35.51 | O |
| ATOM | 651 | N | ALA | A | 80 | 30.952 | 7.521 | 21.899 | 1.00 | 22.36 | N |
| ATOM | 652 | CA | ALA | A | 80 | 31.049 | 8.283 | 20.645 | 1.00 | 21.82 | C |
| ATOM | 653 | C | ALA | A | 80 | 29.678 | 8.446 | 20.007 | 1.00 | 21.71 | C |
| ATOM | 654 | O | ALA | A | 80 | 29.372 | 9.497 | 19.437 | 1.00 | 20.20 | O |
| ATOM | 655 | CB | ALA | A | 80 | 32.025 | 7.600 | 19.668 | 1.00 | 21.77 | C |
| ATOM | 656 | N | GLU | A | 81 | 28.845 | 7.413 | 20.140 | 1.00 | 22.31 | N |
| ATOM | 657 | CA | GLU | A | 81 | 27.471 | 7.394 | 19.609 | 1.00 | 25.62 | C |
| ATOM | 658 | C | GLU | A | 81 | 26.543 | 8.469 | 20.178 | 1.00 | 23.39 | C |
| ATOM | 659 | O | GLU | A | 81 | 25.451 | 8.673 | 19.643 | 1.00 | 23.59 | O |
| ATOM | 660 | CB | GLU | A | 81 | 26.794 | 6.053 | 19.922 | 1.00 | 27.34 | C |
| ATOM | 661 | CG | GLU | A | 81 | 26.386 | 5.935 | 21.408 | 1.00 | 30.98 | C |
| ATOM | 662 | CD | GLU | A | 81 | 26.334 | 4.499 | 21.924 | 1.00 | 33.75 | C |
| ATOM | 663 | OE1 | GLU | A | 81 | 27.382 | 3.795 | 21.860 | 1.00 | 41.56 | O |
| ATOM | 664 | OE2 | GLU | A | 81 | 25.256 | 4.075 | 22.413 | 1.00 | 40.25 | O |
| ATOM | 665 | N | ASP | A | 82 | 26.935 | 9.086 | 21.292 | 1.00 | 21.25 | N |
| ATOM | 666 | CA | ASP | A | 82 | 26.090 | 10.049 | 21.985 | 1.00 | 19.67 | C |
| ATOM | 667 | C | ASP | A | 82 | 26.132 | 11.456 | 21.391 | 1.00 | 19.58 | C |
| ATOM | 668 | O | ASP | A | 82 | 25.380 | 12.327 | 21.821 | 1.00 | 17.85 | O |
| ATOM | 669 | CB | ASP | A | 82 | 26.513 | 10.159 | 23.441 | 1.00 | 20.30 | C |
| ATOM | 670 | CG | ASP | A | 82 | 26.302 | 8.887 | 24.222 | 1.00 | 21.46 | C |
| ATOM | 671 | OD1 | ASP | A | 82 | 25.531 | 8.002 | 23.790 | 1.00 | 22.28 | O |
| ATOM | 672 | OD2 | ASP | A | 82 | 26.933 | 8.776 | 25.293 | 1.00 | 23.19 | O |
| ATOM | 673 | N | VAL | A | 83 | 27.010 | 11.705 | 20.420 | 1.00 | 16.99 | N |
| ATOM | 674 | CA | VAL | A | 83 | 27.102 | 13.063 | 19.875 | 1.00 | 17.76 | C |
| ATOM | 675 | C | VAL | A | 83 | 25.835 | 13.400 | 19.126 | 1.00 | 16.82 | C |
| ATOM | 676 | O | VAL | A | 83 | 25.165 | 12.543 | 18.551 | 1.00 | 16.92 | O |
| ATOM | 677 | CB | VAL | A | 83 | 28.326 | 13.306 | 18.938 | 1.00 | 17.49 | C |
| ATOM | 678 | CG1 | VAL | A | 83 | 29.600 | 13.208 | 19.719 | 1.00 | 20.06 | C |
| ATOM | 679 | CG2 | VAL | A | 83 | 28.301 | 12.361 | 17.764 | 1.00 | 18.72 | C |
| ATOM | 680 | N | GLY | A | 84 | 25.518 | 14.674 | 19.139 | 1.00 | 16.40 | N |
| ATOM | 681 | CA | GLY | A | 84 | 24.284 | 15.124 | 18.586 | 1.00 | 17.19 | C |
| ATOM | 682 | C | GLY | A | 84 | 23.840 | 16.363 | 19.317 | 1.00 | 16.07 | C |
| ATOM | 683 | O | GLY | A | 84 | 24.593 | 16.961 | 20.083 | 1.00 | 16.88 | O |
| ATOM | 684 | N | ILE | A | 85 | 22.593 | 16.722 | 19.075 | 1.00 | 15.76 | N |
| ATOM | 685 | CA | ILE | A | 85 | 22.032 | 17.927 | 19.639 | 1.00 | 16.86 | C |
| ATOM | 686 | C | ILE | A | 85 | 20.917 | 17.477 | 20.591 | 1.00 | 14.75 | C |
| ATOM | 687 | O | ILE | A | 85 | 19.995 | 16.750 | 20.207 | 1.00 | 14.71 | O |
| ATOM | 688 | CB | ILE | A | 85 | 21.489 | 18.859 | 18.556 | 1.00 | 16.94 | C |
| ATOM | 689 | CG1 | ILE | A | 85 | 22.629 | 19.269 | 17.618 | 1.00 | 18.46 | C |
| ATOM | 690 | CG2 | ILE | A | 85 | 20.868 | 20.081 | 19.193 | 1.00 | 17.70 | C |
| ATOM | 691 | CD1 | ILE | A | 85 | 22.210 | 20.164 | 16.468 | 1.00 | 20.58 | C |
| ATOM | 692 | N | TYR | A | 86 | 21.018 | 17.942 | 21.825 | 1.00 | 14.13 | N |
| ATOM | 693 | CA | TYR | A | 86 | 20.072 | 17.611 | 22.885 | 1.00 | 13.39 | C |
| ATOM | 694 | C | TYR | A | 86 | 19.137 | 18.793 | 23.073 | 1.00 | 14.20 | C |
| ATOM | 695 | O | TYR | A | 86 | 19.622 | 19.895 | 23.331 | 1.00 | 15.50 | O |
| ATOM | 696 | CB | TYR | A | 86 | 20.844 | 17.308 | 24.184 | 1.00 | 13.21 | C |
| ATOM | 697 | CG | TYR | A | 86 | 21.593 | 15.996 | 24.111 | 1.00 | 13.43 | C |
| ATOM | 698 | CD1 | TYR | A | 86 | 22.844 | 15.922 | 23.503 | 1.00 | 13.08 | C |
| ATOM | 699 | CD2 | TYR | A | 86 | 21.054 | 14.847 | 24.645 | 1.00 | 13.79 | C |
| ATOM | 700 | CE1 | TYR | A | 86 | 23.535 | 14.721 | 23.411 | 1.00 | 11.57 | C |
| ATOM | 701 | CE2 | TYR | A | 86 | 21.737 | 13.613 | 24.548 | 1.00 | 13.16 | C |
| ATOM | 702 | CZ | TYR | A | 86 | 22.976 | 13.575 | 23.928 | 1.00 | 14.67 | C |
| ATOM | 703 | OH | TYR | A | 86 | 23.639 | 12.374 | 23.847 | 1.00 | 15.74 | O |

| | | | | | | | | | | |
|---|---|---|---|---|---|---|---|---|---|---|
| ATOM | 704 | N | TYR | A | 87 | 17.823 | 18.562 | 22.915 | 1.00 14.87 | N |
| ATOM | 705 | CA | TYR | A | 87 | 16.787 | 19.596 | 23.058 | 1.00 14.31 | C |
| ATOM | 706 | C | TYR | A | 87 | 15.895 | 19.360 | 24.263 | 1.00 14.00 | C |
| ATOM | 707 | O | TYR | A | 87 | 15.432 | 18.242 | 24.492 | 1.00 13.71 | O |
| ATOM | 708 | CB | TYR | A | 87 | 15.825 | 19.583 | 21.864 | 1.00 15.08 | C |
| ATOM | 709 | CG | TYR | A | 87 | 16.441 | 19.907 | 20.556 | 1.00 14.86 | C |
| ATOM | 710 | CD1 | TYR | A | 87 | 16.565 | 21.229 | 20.127 | 1.00 13.14 | C |
| ATOM | 711 | CD2 | TYR | A | 87 | 16.884 | 18.898 | 19.723 | 1.00 15.67 | C |
| ATOM | 712 | CE1 | TYR | A | 87 | 17.108 | 21.527 | 18.885 | 1.00 14.58 | C |
| ATOM | 713 | CE2 | TYR | A | 87 | 17.442 | 19.181 | 18.513 | 1.00 15.50 | C |
| ATOM | 714 | CZ | TYR | A | 87 | 17.548 | 20.490 | 18.095 | 1.00 16.00 | C |
| ATOM | 715 | OH | TYR | A | 87 | 18.111 | 20.741 | 16.868 | 1.00 18.14 | O |
| ATOM | 716 | N | CYS | A | 88 | 15.634 | 20.415 | 25.011 | 1.00 13.69 | N |
| ATOM | 717 | CA | CYS | A | 88 | 14.621 | 20.350 | 26.064 | 1.00 14.88 | C |
| ATOM | 718 | C | CYS | A | 88 | 13.279 | 20.841 | 25.494 | 1.00 15.05 | C |
| ATOM | 719 | O | CYS | A | 88 | 13.238 | 21.486 | 24.451 | 1.00 15.91 | O |
| ATOM | 720 | CB | CYS | A | 88 | 15.057 | 21.134 | 27.292 | 1.00 15.90 | C |
| ATOM | 721 | SG | CYS | A | 88 | 15.436 | 22.873 | 27.002 | 1.00 18.53 | S |
| ATOM | 722 | N | ALA | A | 89 | 12.186 | 20.450 | 26.135 | 1.00 14.55 | N |
| ATOM | 723 | CA | ALA | A | 89 | 10.829 | 20.855 | 25.731 | 1.00 13.42 | C |
| ATOM | 724 | C | ALA | A | 89 | 9.878 | 20.714 | 26.915 | 1.00 12.75 | C |
| ATOM | 725 | O | ALA | A | 89 | 10.097 | 19.881 | 27.805 | 1.00 13.93 | O |
| ATOM | 726 | CB | ALA | A | 89 | 10.327 | 19.964 | 24.585 | 1.00 14.00 | C |
| ATOM | 727 | N | HIS | A | 90 | 8.785 | 21.459 | 26.884 | 1.00 11.95 | N |
| ATOM | 728 | CA | HIS | A | 90 | 7.780 | 21.352 | 27.924 | 1.00 11.35 | C |
| ATOM | 729 | C | HIS | A | 90 | 6.548 | 20.666 | 27.347 | 1.00 13.33 | C |
| ATOM | 730 | O | HIS | A | 90 | 6.383 | 20.587 | 26.106 | 1.00 13.77 | O |
| ATOM | 731 | CB | HIS | A | 90 | 7.418 | 22.728 | 28.467 | 1.00 11.37 | C |
| ATOM | 732 | CG | HIS | A | 90 | 6.635 | 23.546 | 27.506 | 1.00 9.79 | C |
| ATOM | 733 | ND1 | HIS | A | 90 | 5.277 | 23.395 | 27.329 | 1.00 11.98 | N |
| ATOM | 734 | CD2 | HIS | A | 90 | 7.029 | 24.495 | 26.622 | 1.00 11.87 | C |
| ATOM | 735 | CE1 | HIS | A | 90 | 4.864 | 24.224 | 26.388 | 1.00 10.84 | C |
| ATOM | 736 | NE2 | HIS | A | 90 | 5.906 | 24.910 | 25.947 | 1.00 10.70 | N |
| ATOM | 737 | N | ASN | A | 91 | 5.708 | 20.145 | 28.249 | 1.00 13.77 | N |
| ATOM | 738 | CA | ASN | A | 91 | 4.364 | 19.687 | 27.934 | 1.00 12.80 | C |
| ATOM | 739 | C | ASN | A | 91 | 3.328 | 20.190 | 28.941 | 1.00 13.25 | C |
| ATOM | 740 | O | ASN | A | 91 | 2.608 | 19.399 | 29.572 | 1.00 13.97 | O |
| ATOM | 741 | CB | ASN | A | 91 | 4.342 | 18.151 | 27.863 | 1.00 14.13 | C |
| ATOM | 742 | CG | ASN | A | 91 | 2.998 | 17.591 | 27.485 | 1.00 12.80 | C |
| ATOM | 743 | OD1 | ASN | A | 91 | 2.226 | 18.232 | 26.813 | 1.00 11.76 | O |
| ATOM | 744 | ND2 | ASN | A | 91 | 2.722 | 16.339 | 27.902 | 1.00 13.06 | N |
| ATOM | 745 | N | VAL | A | 92 | 3.217 | 21.516 | 29.047 | 1.00 14.28 | N |
| ATOM | 746 | CA | VAL | A | 92 | 2.235 | 22.143 | 29.948 | 1.00 15.79 | C |
| ATOM | 747 | C | VAL | A | 92 | 1.219 | 23.053 | 29.248 | 1.00 14.09 | C |
| ATOM | 748 | O | VAL | A | 92 | 0.217 | 23.441 | 29.861 | 1.00 13.65 | O |
| ATOM | 749 | CB | VAL | A | 92 | 2.932 | 22.923 | 31.098 | 1.00 15.41 | C |
| ATOM | 750 | CG1 | VAL | A | 92 | 3.974 | 22.028 | 31.771 | 1.00 16.41 | C |
| ATOM | 751 | CG2 | VAL | A | 92 | 3.562 | 24.194 | 30.624 | 1.00 18.01 | C |
| ATOM | 752 | N | GLU | A | 93 | 1.472 | 23.386 | 27.981 | 1.00 13.44 | N |
| ATOM | 753 | CA | GLU | A | 93 | 0.590 | 24.275 | 27.215 | 1.00 14.54 | C |
| ATOM | 754 | C | GLU | A | 93 | 0.871 | 24.177 | 25.715 | 1.00 14.99 | C |
| ATOM | 755 | O | GLU | A | 93 | 1.883 | 23.600 | 25.295 | 1.00 14.95 | O |
| ATOM | 756 | CB | GLU | A | 93 | 0.747 | 25.736 | 27.681 | 1.00 15.82 | C |
| ATOM | 757 | CG | GLU | A | 93 | 2.082 | 26.391 | 27.285 | 1.00 14.96 | C |
| ATOM | 758 | CD | GLU | A | 93 | 2.084 | 27.880 | 27.474 | 1.00 17.62 | C |
| ATOM | 759 | OE1 | GLU | A | 93 | 1.433 | 28.333 | 28.439 | 1.00 19.68 | O |
| ATOM | 760 | OE2 | GLU | A | 93 | 2.742 | 28.590 | 26.666 | 1.00 20.64 | O |
| ATOM | 761 | N | LEU | A | 94 | -0.026 | 24.753 | 24.919 | 1.00 13.24 | N |
| ATOM | 762 | CA | LEU | A | 94 | 0.247 | 25.083 | 23.528 | 1.00 12.81 | C |
| ATOM | 763 | C | LEU | A | 94 | 0.577 | 26.596 | 23.503 | 1.00 12.78 | C |
| ATOM | 764 | O | LEU | A | 94 | 0.016 | 27.349 | 24.281 | 1.00 13.45 | O |
| ATOM | 765 | CB | LEU | A | 94 | -0.984 | 24.789 | 22.650 | 1.00 11.45 | C |
| ATOM | 766 | CG | LEU | A | 94 | -1.504 | 23.336 | 22.692 | 1.00 14.63 | C |
| ATOM | 767 | CD1 | LEU | A | 94 | -2.713 | 23.183 | 21.799 | 1.00 14.86 | C |
| ATOM | 768 | CD2 | LEU | A | 94 | -0.388 | 22.362 | 22.335 | 1.00 14.87 | C |

| ATOM | 769 | N | PRO | A | 95 | 1.520 | 27.037 | 22.652 | 1.00 | 14.65 | N |
|------|-----|-----|-----|---|-----|--------|--------|--------|------|-------|---|
| ATOM | 770 | CA | PRO | A | 95 | 2.300 | 26.288 | 21.686 | 1.00 | 14.14 | C |
| ATOM | 771 | C | PRO | A | 95 | 3.290 | 25.402 | 22.363 | 1.00 | 13.46 | C |
| ATOM | 772 | O | PRO | A | 95 | 3.762 | 25.696 | 23.451 | 1.00 | 12.21 | O |
| ATOM | 773 | CB | PRO | A | 95 | 3.039 | 27.375 | 20.903 | 1.00 | 13.77 | C |
| ATOM | 774 | CG | PRO | A | 95 | 3.138 | 28.501 | 21.840 | 1.00 | 14.76 | C |
| ATOM | 775 | CD | PRO | A | 95 | 1.909 | 28.461 | 22.680 | 1.00 | 14.29 | C |
| ATOM | 776 | N | ARG | A | 96 | 3.582 | 24.284 | 21.731 | 1.00 | 13.73 | N |
| ATOM | 777 | CA | ARG | A | 96 | 4.655 | 23.429 | 22.199 | 1.00 | 12.88 | C |
| ATOM | 778 | C | ARG | A | 96 | 5.960 | 24.078 | 21.785 | 1.00 | 14.58 | C |
| ATOM | 779 | O | ARG | A | 96 | 6.112 | 24.461 | 20.619 | 1.00 | 17.45 | O |
| ATOM | 780 | CB | ARG | A | 96 | 4.533 | 22.065 | 21.555 | 1.00 | 11.54 | C |
| ATOM | 781 | CG | ARG | A | 96 | 3.146 | 21.494 | 21.667 | 1.00 | 12.73 | C |
| ATOM | 782 | CD | ARG | A | 96 | 3.183 | 19.984 | 21.510 | 1.00 | 13.02 | C |
| ATOM | 783 | NE | ARG | A | 96 | 1.862 | 19.391 | 21.755 | 1.00 | 10.72 | N |
| ATOM | 784 | CZ | ARG | A | 96 | 1.419 | 19.022 | 22.949 | 1.00 | 13.11 | C |
| ATOM | 785 | NH1 | ARG | A | 96 | 2.172 | 19.173 | 24.046 | 1.00 | 14.10 | N |
| ATOM | 786 | NH2 | ARG | A | 96 | 0.210 | 18.491 | 23.059 | 1.00 | 11.53 | N |
| ATOM | 787 | N | THR | A | 97 | 6.868 | 24.280 | 22.734 | 1.00 | 12.63 | N |
| ATOM | 788 | CA | THR | A | 97 | 8.109 | 24.947 | 22.437 | 1.00 | 13.84 | C |
| ATOM | 789 | C | THR | A | 97 | 9.290 | 24.109 | 22.878 | 1.00 | 14.89 | C |
| ATOM | 790 | O | THR | A | 97 | 9.184 | 23.278 | 23.799 | 1.00 | 14.93 | O |
| ATOM | 791 | CB | THR | A | 97 | 8.198 | 26.391 | 23.011 | 1.00 | 14.46 | C |
| ATOM | 792 | OG1 | THR | A | 97 | 8.044 | 26.374 | 24.429 | 1.00 | 15.15 | O |
| ATOM | 793 | CG2 | THR | A | 97 | 7.113 | 27.266 | 22.420 | 1.00 | 13.97 | C |
| ATOM | 794 | N | PHE | A | 98 | 10.398 | 24.312 | 22.166 | 1.00 | 13.41 | N |
| ATOM | 795 | CA | PHE | A | 98 | 11.626 | 23.594 | 22.353 | 1.00 | 13.98 | C |
| ATOM | 796 | C | PHE | A | 98 | 12.751 | 24.572 | 22.684 | 1.00 | 13.73 | C |
| ATOM | 797 | O | PHE | A | 98 | 12.735 | 25.735 | 22.257 | 1.00 | 14.14 | O |
| ATOM | 798 | CB | PHE | A | 98 | 11.996 | 22.792 | 21.091 | 1.00 | 14.41 | C |
| ATOM | 799 | CG | PHE | A | 98 | 10.960 | 21.791 | 20.673 | 1.00 | 14.19 | C |
| ATOM | 800 | CD1 | PHE | A | 98 | 9.892 | 22.179 | 19.891 | 1.00 | 12.98 | C |
| ATOM | 801 | CD2 | PHE | A | 98 | 11.087 | 20.442 | 21.017 | 1.00 | 12.41 | C |
| ATOM | 802 | CE1 | PHE | A | 98 | 8.936 | 21.275 | 19.504 | 1.00 | 14.53 | C |
| ATOM | 803 | CE2 | PHE | A | 98 | 10.109 | 19.525 | 20.633 | 1.00 | 12.42 | C |
| ATOM | 804 | CZ | PHE | A | 98 | 9.053 | 19.942 | 19.864 | 1.00 | 12.74 | C |
| ATOM | 805 | N | GLY | A | 99 | 13.747 | 24.103 | 23.418 | 1.00 | 14.95 | N |
| ATOM | 806 | CA | GLY | A | 99 | 14.997 | 24.842 | 23.527 | 1.00 | 15.25 | C |
| ATOM | 807 | C | GLY | A | 99 | 15.736 | 24.878 | 22.182 | 1.00 | 14.96 | C |
| ATOM | 808 | O | GLY | A | 99 | 15.332 | 24.212 | 21.210 | 1.00 | 15.76 | O |
| ATOM | 809 | N | GLY | A | 100 | 16.810 | 25.658 | 22.108 | 1.00 | 15.52 | N |
| ATOM | 810 | CA | GLY | A | 100 | 17.567 | 25.792 | 20.874 | 1.00 | 15.83 | C |
| ATOM | 811 | C | GLY | A | 100 | 18.493 | 24.641 | 20.578 | 1.00 | 15.40 | C |
| ATOM | 812 | O | GLY | A | 100 | 19.034 | 24.557 | 19.481 | 1.00 | 16.32 | O |
| ATOM | 813 | N | GLY | A | 101 | 18.661 | 23.747 | 21.546 | 1.00 | 13.67 | N |
| ATOM | 814 | CA | GLY | A | 101 | 19.496 | 22.567 | 21.394 | 1.00 | 15.09 | C |
| ATOM | 815 | C | GLY | A | 101 | 20.905 | 22.818 | 21.883 | 1.00 | 15.07 | C |
| ATOM | 816 | O | GLY | A | 101 | 21.493 | 23.881 | 21.640 | 1.00 | 14.66 | O |
| ATOM | 817 | N | THR | A | 102 | 21.445 | 21.861 | 22.600 | 1.00 | 16.10 | N |
| ATOM | 818 | CA | THR | A | 102 | 22.849 | 21.888 | 23.013 | 1.00 | 16.46 | C |
| ATOM | 819 | C | THR | A | 102 | 23.617 | 20.755 | 22.325 | 1.00 | 16.88 | C |
| ATOM | 820 | O | THR | A | 102 | 23.254 | 19.598 | 22.440 | 1.00 | 16.58 | O |
| ATOM | 821 | CB | THR | A | 102 | 22.996 | 21.714 | 24.539 | 1.00 | 17.08 | C |
| ATOM | 822 | OG1 | THR | A | 102 | 22.247 | 22.729 | 25.227 | 1.00 | 16.60 | O |
| ATOM | 823 | CG2 | THR | A | 102 | 24.460 | 21.815 | 24.948 | 1.00 | 16.16 | C |
| ATOM | 824 | N | LYS | A | 103 | 24.690 | 21.103 | 21.613 | 1.00 | 18.94 | N |
| ATOM | 825 | CA | LYS | A | 103 | 25.484 | 20.115 | 20.898 | 1.00 | 20.50 | C |
| ATOM | 826 | C | LYS | A | 103 | 26.507 | 19.510 | 21.831 | 1.00 | 19.77 | C |
| ATOM | 827 | O | LYS | A | 103 | 27.279 | 20.224 | 22.495 | 1.00 | 19.91 | O |
| ATOM | 828 | CB | LYS | A | 103 | 26.180 | 20.732 | 19.691 | 1.00 | 20.36 | C |
| ATOM | 829 | CG | LYS | A | 103 | 26.993 | 19.704 | 18.873 | 1.00 | 22.59 | C |
| ATOM | 830 | CD | LYS | A | 103 | 27.629 | 20.363 | 17.654 | 1.00 | 25.12 | C |
| ATOM | 831 | CE | LYS | A | 103 | 26.591 | 20.599 | 16.535 | 1.00 | 28.16 | C |
| ATOM | 832 | NZ | LYS | A | 103 | 27.113 | 21.554 | 15.471 | 1.00 | 29.60 | N |
| ATOM | 833 | N | LEU | A | 104 | 26.517 | 18.194 | 21.893 | 1.00 | 20.56 | N |

| ATOM | 834 | CA | LEU | A | 104 | 27.517 | 17.484 | 22.664 | 1.00 | 23.17 | C |
|------|-----|-----|-----|---|-----|--------|--------|--------|------|-------|---|
| ATOM | 835 | C | LEU | A | 104 | 28.705 | 17.221 | 21.735 | 1.00 | 23.88 | C |
| ATOM | 836 | O | LEU | A | 104 | 28.511 | 16.643 | 20.668 | 1.00 | 21.98 | O |
| ATOM | 837 | CB | LEU | A | 104 | 26.957 | 16.168 | 23.172 | 1.00 | 22.74 | C |
| ATOM | 838 | CG | LEU | A | 104 | 27.904 | 15.337 | 24.039 | 1.00 | 24.39 | C |
| ATOM | 839 | CD1 | LEU | A | 104 | 28.273 | 16.126 | 25.282 | 1.00 | 26.54 | C |
| ATOM | 840 | CD2 | LEU | A | 104 | 27.267 | 14.039 | 24.412 | 1.00 | 25.43 | C |
| ATOM | 841 | N | GLU | A | 105 | 29.897 | 17.671 | 22.139 | 1.00 | 25.11 | N |
| ATOM | 842 | CA | GLU | A | 105 | 31.152 | 17.351 | 21.433 | 1.00 | 26.51 | C |
| ATOM | 843 | C | GLU | A | 105 | 32.019 | 16.442 | 22.286 | 1.00 | 25.25 | C |
| ATOM | 844 | O | GLU | A | 105 | 32.119 | 16.633 | 23.499 | 1.00 | 24.28 | O |
| ATOM | 845 | CB | GLU | A | 105 | 31.974 | 18.601 | 21.101 | 1.00 | 27.49 | C |
| ATOM | 846 | CG | GLU | A | 105 | 33.100 | 18.260 | 20.096 | 1.00 | 28.75 | C |
| ATOM | 847 | CD | GLU | A | 105 | 34.195 | 19.311 | 19.911 | 1.00 | 31.33 | C |
| ATOM | 848 | OE1 | GLU | A | 105 | 33.971 | 20.511 | 20.205 | 1.00 | 33.37 | O |
| ATOM | 849 | OE2 | GLU | A | 105 | 35.294 | 18.909 | 19.432 | 1.00 | 32.11 | O |
| ATOM | 850 | N | ILE | A | 106 | 32.616 | 15.455 | 21.631 | 1.00 | 25.27 | N |
| ATOM | 851 | CA | ILE | A | 106 | 33.546 | 14.520 | 22.225 | 1.00 | 25.51 | C |
| ATOM | 852 | C | ILE | A | 106 | 34.975 | 14.992 | 21.985 | 1.00 | 25.43 | C |
| ATOM | 853 | O | ILE | A | 106 | 35.357 | 15.261 | 20.839 | 1.00 | 23.27 | O |
| ATOM | 854 | CB | ILE | A | 106 | 33.417 | 13.134 | 21.569 | 1.00 | 27.46 | C |
| ATOM | 855 | CG1 | ILE | A | 106 | 32.004 | 12.565 | 21.765 | 1.00 | 29.69 | C |
| ATOM | 856 | CG2 | ILE | A | 106 | 34.534 | 12.195 | 22.045 | 1.00 | 26.94 | C |
| ATOM | 857 | CD1 | ILE | A | 106 | 31.822 | 11.718 | 22.970 | 1.00 | 31.41 | C |
| ATOM | 858 | N | LYS | A | 107 | 35.750 | 15.082 | 23.063 | 1.00 | 25.51 | N |
| ATOM | 859 | CA | LYS | A | 107 | 37.181 | 15.328 | 22.999 | 1.00 | 28.14 | C |
| ATOM | 860 | C | LYS | A | 107 | 37.867 | 13.980 | 22.855 | 1.00 | 27.00 | C |
| ATOM | 861 | O | LYS | A | 107 | 37.632 | 13.067 | 23.648 | 1.00 | 27.16 | O |
| ATOM | 862 | CB | LYS | A | 107 | 37.677 | 16.032 | 24.272 | 1.00 | 29.63 | C |
| ATOM | 863 | CG | LYS | A | 107 | 39.118 | 16.507 | 24.205 | 1.00 | 31.66 | C |
| ATOM | 864 | CD | LYS | A | 107 | 39.644 | 16.887 | 25.595 | 1.00 | 32.47 | C |
| ATOM | 865 | CE | LYS | A | 107 | 40.966 | 17.662 | 25.495 | 1.00 | 34.05 | C |
| ATOM | 866 | NZ | LYS | A | 107 | 41.494 | 18.123 | 26.836 | 1.00 | 35.66 | N |
| ATOM | 867 | N | ARG | A | 108 | 38.685 | 13.860 | 21.815 | 1.00 | 26.50 | N |
| ATOM | 868 | CA | ARG | A | 108 | 39.475 | 12.676 | 21.571 | 1.00 | 25.06 | C |
| ATOM | 869 | C | ARG | A | 108 | 40.899 | 13.081 | 21.240 | 1.00 | 23.91 | C |
| ATOM | 870 | O | ARG | A | 108 | 41.223 | 14.270 | 21.186 | 1.00 | 23.49 | O |
| ATOM | 871 | CB | ARG | A | 108 | 38.864 | 11.867 | 20.437 | 1.00 | 24.99 | C |
| ATOM | 872 | CG | ARG | A | 108 | 38.750 | 12.647 | 19.120 | 1.00 | 23.28 | C |
| ATOM | 873 | CD | ARG | A | 108 | 38.868 | 11.715 | 17.929 | 1.00 | 23.69 | C |
| ATOM | 874 | NE | ARG | A | 108 | 40.246 | 11.233 | 17.769 | 1.00 | 22.86 | N |
| ATOM | 875 | CZ | ARG | A | 108 | 40.580 | 10.103 | 17.152 | 1.00 | 23.89 | C |
| ATOM | 876 | NH1 | ARG | A | 108 | 39.668 | 9.311 | 16.655 | 1.00 | 22.63 | N |
| ATOM | 877 | NH2 | ARG | A | 108 | 41.858 | 9.755 | 17.050 | 1.00 | 24.35 | N |
| ATOM | 878 | N | ALA | A | 109 | 41.746 | 12.080 | 21.022 | 1.00 | 22.85 | N |
| ATOM | 879 | CA | ALA | A | 109 | 43.155 | 12.312 | 20.736 | 1.00 | 23.95 | C |
| ATOM | 880 | C | ALA | A | 109 | 43.312 | 13.014 | 19.381 | 1.00 | 23.44 | C |
| ATOM | 881 | O | ALA | A | 109 | 42.539 | 12.740 | 18.478 | 1.00 | 24.22 | O |
| ATOM | 882 | CB | ALA | A | 109 | 43.873 | 10.999 | 20.729 | 1.00 | 25.12 | C |
| ATOM | 883 | N | ASP | A | 110 | 44.296 | 13.900 | 19.243 | 1.00 | 24.31 | N |
| ATOM | 884 | CA | ASP | A | 110 | 44.510 | 14.605 | 17.979 | 1.00 | 24.16 | C |
| ATOM | 885 | C | ASP | A | 110 | 44.799 | 13.595 | 16.877 | 1.00 | 23.34 | C |
| ATOM | 886 | O | ASP | A | 110 | 45.462 | 12.606 | 17.119 | 1.00 | 21.37 | O |
| ATOM | 887 | CB | ASP | A | 110 | 45.658 | 15.608 | 18.055 | 1.00 | 25.51 | C |
| ATOM | 888 | CG | ASP | A | 110 | 45.370 | 16.768 | 18.984 | 1.00 | 26.60 | C |
| ATOM | 889 | OD1 | ASP | A | 110 | 44.241 | 16.847 | 19.521 | 1.00 | 28.33 | O |
| ATOM | 890 | OD2 | ASP | A | 110 | 46.284 | 17.603 | 19.178 | 1.00 | 28.31 | O |
| ATOM | 891 | N | ALA | A | 111 | 44.272 | 13.849 | 15.679 | 1.00 | 20.98 | N |
| ATOM | 892 | CA | ALA | A | 111 | 44.448 | 12.953 | 14.540 | 1.00 | 18.62 | C |
| ATOM | 893 | C | ALA | A | 111 | 44.719 | 13.829 | 13.323 | 1.00 | 18.22 | C |
| ATOM | 894 | O | ALA | A | 111 | 43.989 | 14.765 | 13.072 | 1.00 | 16.84 | O |
| ATOM | 895 | CB | ALA | A | 111 | 43.203 | 12.135 | 14.318 | 1.00 | 19.11 | C |
| ATOM | 896 | N | ALA | A | 112 | 45.773 | 13.523 | 12.580 | 1.00 | 15.66 | N |
| ATOM | 897 | CA | ALA | A | 112 | 46.079 | 14.241 | 11.353 | 1.00 | 15.10 | C |
| ATOM | 898 | C | ALA | A | 112 | 45.054 | 13.874 | 10.270 | 1.00 | 15.53 | C |

```
ATOM    899  O    ALA A 112      44.574  12.736  10.238  1.00 15.07           O
ATOM    900  CB   ALA A 112      47.450  13.897  10.878  1.00 15.34           C
ATOM    901  N    PRO A 113      44.761  14.805   9.344  1.00 14.96           N
ATOM    902  CA   PRO A 113      43.920  14.395   8.208  1.00 15.98           C
ATOM    903  C    PRO A 113      44.629  13.453   7.241  1.00 14.67           C
ATOM    904  O    PRO A 113      45.848  13.505   7.114  1.00 14.61           O
ATOM    905  CB   PRO A 113      43.647  15.709   7.492  1.00 16.38           C
ATOM    906  CG   PRO A 113      44.835  16.542   7.837  1.00 16.33           C
ATOM    907  CD   PRO A 113      45.164  16.220   9.241  1.00 17.13           C
ATOM    908  N    THR A 114      43.851  12.579   6.610  1.00 14.66           N
ATOM    909  CA   THR A 114      44.228  11.866   5.408  1.00 14.01           C
ATOM    910  C    THR A 114      43.721  12.669   4.214  1.00 13.59           C
ATOM    911  O    THR A 114      42.505  12.870   4.080  1.00 13.04           O
ATOM    912  CB   THR A 114      43.566  10.466   5.428  1.00 15.59           C
ATOM    913  OG1  THR A 114      43.982   9.793   6.620  1.00 16.47           O
ATOM    914  CG2  THR A 114      43.939   9.646   4.245  1.00 15.48           C
ATOM    915  N    VAL A 115      44.643  13.078   3.341  1.00 12.99           N
ATOM    916  CA   VAL A 115      44.347  13.999   2.257  1.00 14.11           C
ATOM    917  C    VAL A 115      44.362  13.244   0.944  1.00 14.52           C
ATOM    918  O    VAL A 115      45.269  12.451   0.688  1.00 13.32           O
ATOM    919  CB   VAL A 115      45.399  15.114   2.248  1.00 15.63           C
ATOM    920  CG1  VAL A 115      45.089  16.222   1.189  1.00 16.73           C
ATOM    921  CG2  VAL A 115      45.508  15.705   3.640  1.00 17.30           C
ATOM    922  N    SER A 116      43.323  13.465   0.130  1.00 13.23           N
ATOM    923  CA   SER A 116      43.203  12.882  -1.183  1.00 13.37           C
ATOM    924  C    SER A 116      42.854  13.998  -2.155  1.00 13.38           C
ATOM    925  O    SER A 116      41.959  14.756  -1.876  1.00 14.66           O
ATOM    926  CB   SER A 116      42.094  11.843  -1.211  1.00 13.52           C
ATOM    927  OG   SER A 116      42.370  10.769  -0.334  1.00 16.57           O
ATOM    928  N    ILE A 117      43.557  14.066  -3.288  1.00 13.03           N
ATOM    929  CA   ILE A 117      43.239  14.996  -4.368  1.00 13.09           C
ATOM    930  C    ILE A 117      42.723  14.198  -5.576  1.00 14.25           C
ATOM    931  O    ILE A 117      43.191  13.063  -5.844  1.00 13.76           O
ATOM    932  CB   ILE A 117      44.445  15.894  -4.741  1.00 13.53           C
ATOM    933  CG1  ILE A 117      43.997  17.077  -5.622  1.00 12.53           C
ATOM    934  CG2  ILE A 117      45.539  15.119  -5.404  1.00 13.34           C
ATOM    935  CD1  ILE A 117      45.099  18.059  -5.924  1.00 14.32           C
ATOM    936  N    PHE A 118      41.750  14.791  -6.274  1.00 11.43           N
ATOM    937  CA   PHE A 118      41.073  14.183  -7.432  1.00 12.16           C
ATOM    938  C    PHE A 118      40.981  15.135  -8.622  1.00 12.78           C
ATOM    939  O    PHE A 118      40.417  16.225  -8.488  1.00 10.99           O
ATOM    940  CB   PHE A 118      39.656  13.749  -7.088  1.00 12.60           C
ATOM    941  CG   PHE A 118      39.572  12.814  -5.909  1.00 12.39           C
ATOM    942  CD1  PHE A 118      39.779  11.435  -6.064  1.00 14.40           C
ATOM    943  CD2  PHE A 118      39.244  13.312  -4.659  1.00 13.21           C
ATOM    944  CE1  PHE A 118      39.695  10.591  -4.960  1.00 14.53           C
ATOM    945  CE2  PHE A 118      39.144  12.482  -3.562  1.00 15.86           C
ATOM    946  CZ   PHE A 118      39.348  11.117  -3.710  1.00 14.03           C
ATOM    947  N    PRO A 119      41.493  14.703  -9.794  1.00 13.38           N
ATOM    948  CA   PRO A 119      41.337  15.476 -10.997  1.00 13.49           C
ATOM    949  C    PRO A 119      39.892  15.479 -11.483  1.00 13.00           C
ATOM    950  O    PRO A 119      39.065  14.668 -11.036  1.00 12.34           O
ATOM    951  CB   PRO A 119      42.239  14.746 -12.036  1.00 13.46           C
ATOM    952  CG   PRO A 119      43.031  13.801 -11.300  1.00 14.16           C
ATOM    953  CD   PRO A 119      42.208  13.439 -10.076  1.00 14.81           C
ATOM    954  N    PRO A 120      39.561  16.441 -12.360  1.00 13.63           N
ATOM    955  CA   PRO A 120      38.248  16.405 -12.979  1.00 12.88           C
ATOM    956  C    PRO A 120      38.009  15.112 -13.735  1.00 13.46           C
ATOM    957  O    PRO A 120      38.934  14.583 -14.361  1.00 14.11           O
ATOM    958  CB   PRO A 120      38.270  17.575 -13.954  1.00 13.34           C
ATOM    959  CG   PRO A 120      39.371  18.441 -13.529  1.00 14.77           C
ATOM    960  CD   PRO A 120      40.354  17.604 -12.783  1.00 14.05           C
ATOM    961  N    SER A 121      36.767  14.652 -13.692  1.00 12.25           N
ATOM    962  CA   SER A 121      36.301  13.511 -14.481  1.00 12.44           C
ATOM    963  C    SER A 121      36.232  13.843 -15.964  1.00 13.12           C
```

```
ATOM    964  O    SER A 121     36.039  15.014 -16.359  1.00  8.72          O
ATOM    965  CB   SER A 121     34.918  13.084 -13.997  1.00 12.88          C
ATOM    966  OG   SER A 121     33.903  14.109 -14.131  1.00 13.97          O
ATOM    967  N    SER A 122     36.344  12.826 -16.812  1.00 13.24          N
ATOM    968  CA   SER A 122     36.160  13.051 -18.238  1.00 14.18          C
ATOM    969  C    SER A 122     34.725  13.489 -18.543  1.00 13.58          C
ATOM    970  O    SER A 122     34.522  14.279 -19.463  1.00 13.51          O
ATOM    971  CB   SER A 122     36.561  11.802 -19.063  1.00 16.95          C
ATOM    972  OG   SER A 122     35.902  10.660 -18.563  1.00 17.78          O
ATOM    973  N    GLU A 123     33.744  13.061 -17.738  1.00 13.76          N
ATOM    974  CA   GLU A 123     32.358  13.499 -17.909  1.00 13.42          C
ATOM    975  C    GLU A 123     32.275  15.006 -17.701  1.00 11.06          C
ATOM    976  O    GLU A 123     31.683  15.704 -18.480  1.00 11.26          O
ATOM    977  CB   GLU A 123     31.411  12.857 -16.897  1.00 16.11          C
ATOM    978  CG   GLU A 123     31.171  11.344 -17.088  1.00 17.81          C
ATOM    979  CD   GLU A 123     32.131  10.455 -16.301  1.00 19.98          C
ATOM    980  OE1  GLU A 123     33.306  10.843 -16.059  1.00 16.41          O
ATOM    981  OE2  GLU A 123     31.691   9.324 -15.946  1.00 23.68          O
ATOM    982  N    GLN A 124     32.854  15.486 -16.616  1.00 10.66          N
ATOM    983  CA   GLN A 124     32.740  16.915 -16.349  1.00  9.68          C
ATOM    984  C    GLN A 124     33.439  17.693 -17.446  1.00  9.72          C
ATOM    985  O    GLN A 124     32.942  18.754 -17.863  1.00 12.54          O
ATOM    986  CB   GLN A 124     33.311  17.261 -14.992  1.00 10.73          C
ATOM    987  CG   GLN A 124     32.924  18.724 -14.602  1.00  9.20          C
ATOM    988  CD   GLN A 124     33.458  19.165 -13.273  1.00 10.53          C
ATOM    989  OE1  GLN A 124     34.382  18.568 -12.737  1.00  9.73          O
ATOM    990  NE2  GLN A 124     32.919  20.292 -12.754  1.00 11.37          N
ATOM    991  N    LEU A 125     34.614  17.226 -17.860  1.00 11.17          N
ATOM    992  CA   LEU A 125     35.353  17.865 -18.943  1.00 10.84          C
ATOM    993  C    LEU A 125     34.557  17.923 -20.245  1.00 10.59          C
ATOM    994  O    LEU A 125     34.523  18.947 -20.898  1.00  9.90          O
ATOM    995  CB   LEU A 125     36.703  17.171 -19.158  1.00 12.09          C
ATOM    996  CG   LEU A 125     37.753  17.425 -18.050  1.00 12.15          C
ATOM    997  CD1  LEU A 125     38.987  16.577 -18.200  1.00 13.35          C
ATOM    998  CD2  LEU A 125     38.092  18.898 -17.994  1.00 13.44          C
ATOM    999  N    THR A 126     33.882  16.841 -20.612  1.00 10.04          N
ATOM   1000  CA   THR A 126     32.958  16.852 -21.748  1.00 10.06          C
ATOM   1001  C    THR A 126     31.898  17.954 -21.679  1.00 10.62          C
ATOM   1002  O    THR A 126     31.543  18.569 -22.690  1.00 12.49          O
ATOM   1003  CB   THR A 126     32.301  15.446 -21.885  1.00 11.19          C
ATOM   1004  OG1  THR A 126     33.322  14.540 -22.318  1.00 10.90          O
ATOM   1005  CG2  THR A 126     31.175  15.491 -22.879  1.00 11.94          C
ATOM   1006  N    SER A 127     31.397  18.204 -20.474  1.00  9.48          N
ATOM   1007  CA   SER A 127     30.400  19.228 -20.233  1.00  8.31          C
ATOM   1008  C    SER A 127     30.973  20.639 -20.231  1.00  8.35          C
ATOM   1009  O    SER A 127     30.187  21.594 -20.161  1.00 10.01          O
ATOM   1010  CB   SER A 127     29.680  18.973 -18.901  1.00  6.89          C
ATOM   1011  OG   SER A 127     30.383  19.460 -17.755  1.00  8.56          O
ATOM   1012  N    GLY A 128     32.299  20.791 -20.226  1.00  9.03          N
ATOM   1013  CA   GLY A 128     32.938  22.133 -20.249  1.00  9.86          C
ATOM   1014  C    GLY A 128     33.413  22.638 -18.896  1.00 11.41          C
ATOM   1015  O    GLY A 128     33.829  23.798 -18.765  1.00 10.32          O
ATOM   1016  N    GLY A 129     33.327  21.780 -17.885  1.00 11.21          N
ATOM   1017  CA   GLY A 129     33.667  22.139 -16.513  1.00 11.54          C
ATOM   1018  C    GLY A 129     34.863  21.391 -15.988  1.00 11.85          C
ATOM   1019  O    GLY A 129     35.291  20.398 -16.570  1.00 12.06          O
ATOM   1020  N    ALA A 130     35.437  21.871 -14.881  1.00 12.02          N
ATOM   1021  CA   ALA A 130     36.593  21.211 -14.308  1.00 11.08          C
ATOM   1022  C    ALA A 130     36.687  21.501 -12.823  1.00 12.45          C
ATOM   1023  O    ALA A 130     37.108  22.569 -12.434  1.00 13.28          O
ATOM   1024  CB   ALA A 130     37.836  21.631 -14.996  1.00 12.08          C
ATOM   1025  N    SER A 131     36.237  20.562 -12.004  1.00 13.18          N
ATOM   1026  CA   SER A 131     36.326  20.703 -10.563  1.00 13.79          C
ATOM   1027  C    SER A 131     37.446  19.820 -10.046  1.00 14.35          C
ATOM   1028  O    SER A 131     37.540  18.625 -10.410  1.00 14.17          O
```

| | | | | | | | | | | |
|------|------|-----|-----|---|-----|--------|--------|---------|------|-------|---|
| ATOM | 1029 | CB  | SER | A | 131 | 35.000 | 20.324 | -9.908  | 1.00 | 12.66 | C |
| ATOM | 1030 | OG  | SER | A | 131 | 33.996 | 21.245 | -10.246 | 1.00 | 13.42 | O |
| ATOM | 1031 | N   | VAL | A | 132 | 38.331 | 20.419 | -9.267  | 1.00 | 12.49 | N |
| ATOM | 1032 | CA  | VAL | A | 132 | 39.368 | 19.675 | -8.586  | 1.00 | 13.31 | C |
| ATOM | 1033 | C   | VAL | A | 132 | 38.964 | 19.544 | -7.103  | 1.00 | 12.89 | C |
| ATOM | 1034 | O   | VAL | A | 132 | 38.633 | 20.547 | -6.426  | 1.00 | 9.96  | O |
| ATOM | 1035 | CB  | VAL | A | 132 | 40.733 | 20.345 | -8.729  | 1.00 | 13.62 | C |
| ATOM | 1036 | CG1 | VAL | A | 132 | 41.819 | 19.379 | -8.263  | 1.00 | 14.80 | C |
| ATOM | 1037 | CG2 | VAL | A | 132 | 40.950 | 20.763 | -10.179 | 1.00 | 15.59 | C |
| ATOM | 1038 | N   | VAL | A | 133 | 38.980 | 18.308 | -6.604  | 1.00 | 10.87 | N |
| ATOM | 1039 | CA  | VAL | A | 133 | 38.442 | 18.000 | -5.277  | 1.00 | 11.59 | C |
| ATOM | 1040 | C   | VAL | A | 133 | 39.543 | 17.483 | -4.363  | 1.00 | 11.60 | C |
| ATOM | 1041 | O   | VAL | A | 133 | 40.393 | 16.691 | -4.755  | 1.00 | 11.05 | O |
| ATOM | 1042 | CB  | VAL | A | 133 | 37.236 | 17.004 | -5.327  | 1.00 | 10.92 | C |
| ATOM | 1043 | CG1 | VAL | A | 133 | 36.696 | 16.764 | -3.925  | 1.00 | 11.58 | C |
| ATOM | 1044 | CG2 | VAL | A | 133 | 36.122 | 17.551 | -6.259  | 1.00 | 12.07 | C |
| ATOM | 1045 | N   | CYS | A | 134 | 39.568 | 18.014 | -3.145  | 1.00 | 12.86 | N |
| ATOM | 1046 | CA  | CYS | A | 134 | 40.492 | 17.571 | -2.126  | 1.00 | 13.69 | C |
| ATOM | 1047 | C   | CYS | A | 134 | 39.683 | 17.171 | -0.899  | 1.00 | 12.49 | C |
| ATOM | 1048 | O   | CYS | A | 134 | 38.874 | 17.965 | -0.423  | 1.00 | 12.10 | O |
| ATOM | 1049 | CB  | CYS | A | 134 | 41.451 | 18.722 | -1.811  | 1.00 | 15.57 | C |
| ATOM | 1050 | SG  | CYS | A | 134 | 42.727 | 18.325 | -0.664  | 1.00 | 22.07 | S |
| ATOM | 1051 | N   | PHE | A | 135 | 39.843 | 15.930 | -0.405  | 1.00 | 11.66 | N |
| ATOM | 1052 | CA  | PHE | A | 135 | 39.223 | 15.508 | 0.843   | 1.00 | 11.56 | C |
| ATOM | 1053 | C   | PHE | A | 135 | 40.291 | 15.556 | 1.948   | 1.00 | 12.90 | C |
| ATOM | 1054 | O   | PHE | A | 135 | 41.407 | 15.095 | 1.748   | 1.00 | 12.02 | O |
| ATOM | 1055 | CB  | PHE | A | 135 | 38.668 | 14.059 | 0.840   | 1.00 | 12.89 | C |
| ATOM | 1056 | CG  | PHE | A | 135 | 37.490 | 13.811 | -0.081  | 1.00 | 12.46 | C |
| ATOM | 1057 | CD1 | PHE | A | 135 | 36.585 | 14.805 | -0.431  | 1.00 | 13.34 | C |
| ATOM | 1058 | CD2 | PHE | A | 135 | 37.244 | 12.518 | -0.558  | 1.00 | 14.81 | C |
| ATOM | 1059 | CE1 | PHE | A | 135 | 35.478 | 14.525 | -1.255  | 1.00 | 14.00 | C |
| ATOM | 1060 | CE2 | PHE | A | 135 | 36.164 | 12.252 | -1.384  | 1.00 | 16.35 | C |
| ATOM | 1061 | CZ  | PHE | A | 135 | 35.290 | 13.263 | -1.757  | 1.00 | 14.16 | C |
| ATOM | 1062 | N   | LEU | A | 136 | 39.905 | 16.111 | 3.094   | 1.00 | 12.89 | N |
| ATOM | 1063 | CA  | LEU | A | 136 | 40.721 | 16.194 | 4.297   | 1.00 | 14.00 | C |
| ATOM | 1064 | C   | LEU | A | 136 | 39.924 | 15.407 | 5.316   | 1.00 | 14.29 | C |
| ATOM | 1065 | O   | LEU | A | 136 | 39.007 | 15.933 | 5.927   | 1.00 | 14.10 | O |
| ATOM | 1066 | CB  | LEU | A | 136 | 40.883 | 17.639 | 4.762   | 1.00 | 15.27 | C |
| ATOM | 1067 | CG  | LEU | A | 136 | 41.747 | 18.539 | 3.872   | 1.00 | 17.65 | C |
| ATOM | 1068 | CD1 | LEU | A | 136 | 41.028 | 18.945 | 2.592   | 1.00 | 19.41 | C |
| ATOM | 1069 | CD2 | LEU | A | 136 | 42.136 | 19.764 | 4.694   | 1.00 | 16.99 | C |
| ATOM | 1070 | N   | ASN | A | 137 | 40.233 | 14.119 | 5.439   | 1.00 | 11.99 | N |
| ATOM | 1071 | CA  | ASN | A | 137 | 39.360 | 13.186 | 6.160   | 1.00 | 12.35 | C |
| ATOM | 1072 | C   | ASN | A | 137 | 39.850 | 12.755 | 7.530   | 1.00 | 12.48 | C |
| ATOM | 1073 | O   | ASN | A | 137 | 41.041 | 12.494 | 7.720   | 1.00 | 12.84 | O |
| ATOM | 1074 | CB  | ASN | A | 137 | 39.130 | 11.926 | 5.317   | 1.00 | 13.07 | C |
| ATOM | 1075 | CG  | ASN | A | 137 | 38.099 | 12.131 | 4.228   | 1.00 | 15.60 | C |
| ATOM | 1076 | OD1 | ASN | A | 137 | 37.365 | 13.120 | 4.247   | 1.00 | 16.62 | O |
| ATOM | 1077 | ND2 | ASN | A | 137 | 38.050 | 11.215 | 3.261   | 1.00 | 14.36 | N |
| ATOM | 1078 | N   | ASN | A | 138 | 38.887 | 12.653 | 8.445   | 1.00 | 13.12 | N |
| ATOM | 1079 | CA  | ASN | A | 138 | 39.062 | 11.999 | 9.745   | 1.00 | 15.06 | C |
| ATOM | 1080 | C   | ASN | A | 138 | 40.198 | 12.586 | 10.580  | 1.00 | 14.83 | C |
| ATOM | 1081 | O   | ASN | A | 138 | 41.145 | 11.908 | 10.999  | 1.00 | 15.03 | O |
| ATOM | 1082 | CB  | ASN | A | 138 | 39.221 | 10.493 | 9.557   | 1.00 | 14.95 | C |
| ATOM | 1083 | CG  | ASN | A | 138 | 38.064 | 9.882  | 8.807   | 1.00 | 17.53 | C |
| ATOM | 1084 | OD1 | ASN | A | 138 | 38.057 | 9.850  | 7.584   | 1.00 | 21.05 | O |
| ATOM | 1085 | ND2 | ASN | A | 138 | 37.079 | 9.395  | 9.533   | 1.00 | 19.60 | N |
| ATOM | 1086 | N   | PHE | A | 139 | 40.064 | 13.875 | 10.855  | 1.00 | 15.27 | N |
| ATOM | 1087 | CA  | PHE | A | 139 | 41.007 | 14.582 | 11.711  | 1.00 | 15.49 | C |
| ATOM | 1088 | C   | PHE | A | 139 | 40.365 | 15.125 | 13.002  | 1.00 | 14.77 | C |
| ATOM | 1089 | O   | PHE | A | 139 | 39.151 | 15.192 | 13.164  | 1.00 | 14.75 | O |
| ATOM | 1090 | CB  | PHE | A | 139 | 41.675 | 15.718 | 10.941  | 1.00 | 15.76 | C |
| ATOM | 1091 | CG  | PHE | A | 139 | 40.709 | 16.723 | 10.371  | 1.00 | 16.93 | C |
| ATOM | 1092 | CD1 | PHE | A | 139 | 40.347 | 17.857 | 11.112  | 1.00 | 14.99 | C |
| ATOM | 1093 | CD2 | PHE | A | 139 | 40.200 | 16.582 | 9.093   | 1.00 | 15.23 | C |

| ATOM | 1094 | CE1 | PHE A 139 | 39.458 | 18.792 | 10.604 | 1.00 17.59 | C |
|---|---|---|---|---|---|---|---|---|
| ATOM | 1095 | CE2 | PHE A 139 | 39.318 | 17.533 | 8.580 | 1.00 15.48 | C |
| ATOM | 1096 | CZ | PHE A 139 | 38.953 | 18.640 | 9.337 | 1.00 16.28 | C |
| ATOM | 1097 | N | TYR A 140 | 41.224 | 15.454 | 13.945 | 1.00 16.20 | N |
| ATOM | 1098 | CA | TYR A 140 | 40.786 | 16.044 | 15.193 | 1.00 18.10 | C |
| ATOM | 1099 | C | TYR A 140 | 41.947 | 16.846 | 15.745 | 1.00 19.20 | C |
| ATOM | 1100 | O | TYR A 140 | 43.073 | 16.373 | 15.710 | 1.00 19.62 | O |
| ATOM | 1101 | CB | TYR A 140 | 40.366 | 14.967 | 16.191 | 1.00 19.44 | C |
| ATOM | 1102 | CG | TYR A 140 | 39.755 | 15.601 | 17.406 | 1.00 20.38 | C |
| ATOM | 1103 | CD1 | TYR A 140 | 40.549 | 16.160 | 18.398 | 1.00 20.07 | C |
| ATOM | 1104 | CD2 | TYR A 140 | 38.381 | 15.751 | 17.508 | 1.00 20.17 | C |
| ATOM | 1105 | CE1 | TYR A 140 | 39.967 | 16.809 | 19.499 | 1.00 20.49 | C |
| ATOM | 1106 | CE2 | TYR A 140 | 37.810 | 16.386 | 18.590 | 1.00 21.33 | C |
| ATOM | 1107 | CZ | TYR A 140 | 38.603 | 16.918 | 19.572 | 1.00 21.05 | C |
| ATOM | 1108 | OH | TYR A 140 | 37.996 | 17.569 | 20.639 | 1.00 22.46 | O |
| ATOM | 1109 | N | PRO A 141 | 41.690 | 18.073 | 16.244 | 1.00 19.86 | N |
| ATOM | 1110 | CA | PRO A 141 | 40.420 | 18.795 | 16.393 | 1.00 20.62 | C |
| ATOM | 1111 | C | PRO A 141 | 39.856 | 19.390 | 15.094 | 1.00 19.87 | C |
| ATOM | 1112 | O | PRO A 141 | 40.483 | 19.279 | 14.037 | 1.00 17.47 | O |
| ATOM | 1113 | CB | PRO A 141 | 40.790 | 19.916 | 17.374 | 1.00 20.76 | C |
| ATOM | 1114 | CG | PRO A 141 | 42.194 | 20.221 | 17.011 | 1.00 21.01 | C |
| ATOM | 1115 | CD | PRO A 141 | 42.814 | 18.864 | 16.765 | 1.00 20.87 | C |
| ATOM | 1116 | N | LYS A 142 | 38.706 | 20.063 | 15.199 | 1.00 22.01 | N |
| ATOM | 1117 | CA | LYS A 142 | 37.942 | 20.545 | 14.027 | 1.00 22.75 | C |
| ATOM | 1118 | C | LYS A 142 | 38.639 | 21.615 | 13.175 | 1.00 21.74 | C |
| ATOM | 1119 | O | LYS A 142 | 38.397 | 21.705 | 11.963 | 1.00 20.14 | O |
| ATOM | 1120 | CB | LYS A 142 | 36.554 | 21.068 | 14.473 | 1.00 24.74 | C |
| ATOM | 1121 | CG | LYS A 142 | 35.602 | 21.451 | 13.329 | 1.00 26.43 | C |
| ATOM | 1122 | CD | LYS A 142 | 34.112 | 21.608 | 13.737 | 1.00 27.01 | C |
| ATOM | 1123 | CE | LYS A 142 | 33.185 | 21.838 | 12.509 | 1.00 28.98 | C |
| ATOM | 1124 | NZ | LYS A 142 | 31.793 | 21.183 | 12.588 | 1.00 30.93 | N |
| ATOM | 1125 | N | ASP A 143 | 39.467 | 22.464 | 13.787 | 1.00 22.76 | N |
| ATOM | 1126 | CA | ASP A 143 | 40.122 | 23.535 | 13.038 | 1.00 23.45 | C |
| ATOM | 1127 | C | ASP A 143 | 41.133 | 23.012 | 12.035 | 1.00 21.02 | C |
| ATOM | 1128 | O | ASP A 143 | 41.952 | 22.146 | 12.338 | 1.00 19.73 | O |
| ATOM | 1129 | CB | ASP A 143 | 40.803 | 24.539 | 13.958 | 1.00 26.03 | C |
| ATOM | 1130 | CG | ASP A 143 | 39.865 | 25.630 | 14.406 | 1.00 30.99 | C |
| ATOM | 1131 | OD1 | ASP A 143 | 38.903 | 25.334 | 15.153 | 1.00 35.33 | O |
| ATOM | 1132 | OD2 | ASP A 143 | 40.084 | 26.797 | 13.991 | 1.00 37.26 | O |
| ATOM | 1133 | N | ILE A 144 | 41.057 | 23.541 | 10.822 | 1.00 19.28 | N |
| ATOM | 1134 | CA | ILE A 144 | 41.957 | 23.125 | 9.768 | 1.00 20.31 | C |
| ATOM | 1135 | C | ILE A 144 | 41.937 | 24.173 | 8.661 | 1.00 20.75 | C |
| ATOM | 1136 | O | ILE A 144 | 40.951 | 24.904 | 8.504 | 1.00 20.85 | O |
| ATOM | 1137 | CB | ILE A 144 | 41.556 | 21.717 | 9.223 | 1.00 20.74 | C |
| ATOM | 1138 | CG1 | ILE A 144 | 42.721 | 21.066 | 8.471 | 1.00 20.56 | C |
| ATOM | 1139 | CG2 | ILE A 144 | 40.344 | 21.813 | 8.333 | 1.00 21.26 | C |
| ATOM | 1140 | CD1 | ILE A 144 | 42.526 | 19.583 | 8.240 | 1.00 20.08 | C |
| ATOM | 1141 | N | ASN A 145 | 43.013 | 24.228 | 7.900 | 1.00 22.49 | N |
| ATOM | 1142 | CA | ASN A 145 | 43.129 | 25.149 | 6.774 | 1.00 22.92 | C |
| ATOM | 1143 | C | ASN A 145 | 43.608 | 24.448 | 5.517 | 1.00 20.94 | C |
| ATOM | 1144 | O | ASN A 145 | 44.432 | 23.546 | 5.571 | 1.00 21.16 | O |
| ATOM | 1145 | CB | ASN A 145 | 44.051 | 26.298 | 7.132 | 1.00 25.71 | C |
| ATOM | 1146 | CG | ASN A 145 | 43.326 | 27.401 | 7.900 | 1.00 30.36 | C |
| ATOM | 1147 | OD1 | ASN A 145 | 42.557 | 28.177 | 7.321 | 1.00 36.22 | O |
| ATOM | 1148 | ND2 | ASN A 145 | 43.549 | 27.463 | 9.204 | 1.00 33.78 | N |
| ATOM | 1149 | N | VAL A 146 | 43.073 | 24.875 | 4.380 | 1.00 19.76 | N |
| ATOM | 1150 | CA | VAL A 146 | 43.437 | 24.304 | 3.101 | 1.00 19.58 | C |
| ATOM | 1151 | C | VAL A 146 | 43.888 | 25.433 | 2.202 | 1.00 20.03 | C |
| ATOM | 1152 | O | VAL A 146 | 43.278 | 26.495 | 2.204 | 1.00 18.85 | O |
| ATOM | 1153 | CB | VAL A 146 | 42.252 | 23.499 | 2.472 | 1.00 21.29 | C |
| ATOM | 1154 | CG1 | VAL A 146 | 41.012 | 24.389 | 2.237 | 1.00 22.81 | C |
| ATOM | 1155 | CG2 | VAL A 146 | 42.671 | 22.840 | 1.175 | 1.00 21.21 | C |
| ATOM | 1156 | N | LYS A 147 | 44.964 | 25.196 | 1.478 | 1.00 18.96 | N |
| ATOM | 1157 | CA | LYS A 147 | 45.443 | 26.092 | 0.457 | 1.00 22.16 | C |
| ATOM | 1158 | C | LYS A 147 | 45.603 | 25.364 | -0.877 | 1.00 21.25 | C |

| ATOM | 1159 | O | LYS A 147 | 46.123 | 24.244 | -0.931 | 1.00 | 20.97 | O |
|------|------|------|-----------|--------|--------|--------|------|-------|---|
| ATOM | 1160 | CB | LYS A 147 | 46.770 | 26.691 | 0.871 | 1.00 | 22.43 | C |
| ATOM | 1161 | CG | LYS A 147 | 47.325 | 27.709 | -0.091 | 1.00 | 25.20 | C |
| ATOM | 1162 | CD | LYS A 147 | 48.793 | 28.023 | 0.250 | 1.00 | 26.65 | C |
| ATOM | 1163 | CE | LYS A 147 | 48.896 | 28.892 | 1.499 | 1.00 | 30.03 | C |
| ATOM | 1164 | NZ | LYS A 147 | 50.318 | 29.352 | 1.742 | 1.00 | 30.26 | N |
| ATOM | 1165 | N | TRP A 148 | 45.134 | 26.012 | -1.943 | 1.00 | 20.05 | N |
| ATOM | 1166 | CA | TRP A 148 | 45.277 | 25.509 | -3.306 | 1.00 | 17.76 | C |
| ATOM | 1167 | C | TRP A 148 | 46.406 | 26.228 | -4.020 | 1.00 | 19.32 | C |
| ATOM | 1168 | O | TRP A 148 | 46.564 | 27.436 | -3.872 | 1.00 | 20.30 | O |
| ATOM | 1169 | CB | TRP A 148 | 44.007 | 25.749 | -4.107 | 1.00 | 14.84 | C |
| ATOM | 1170 | CG | TRP A 148 | 42.940 | 24.851 | -3.732 | 1.00 | 14.55 | C |
| ATOM | 1171 | CD1 | TRP A 148 | 42.007 | 25.039 | -2.770 | 1.00 | 14.81 | C |
| ATOM | 1172 | CD2 | TRP A 148 | 42.715 | 23.554 | -4.273 | 1.00 | 13.30 | C |
| ATOM | 1173 | NE1 | TRP A 148 | 41.188 | 23.927 | -2.674 | 1.00 | 14.91 | N |
| ATOM | 1174 | CE2 | TRP A 148 | 41.615 | 22.995 | -3.583 | 1.00 | 13.60 | C |
| ATOM | 1175 | CE3 | TRP A 148 | 43.336 | 22.806 | -5.264 | 1.00 | 13.87 | C |
| ATOM | 1176 | CZ2 | TRP A 148 | 41.109 | 21.734 | -3.892 | 1.00 | 14.58 | C |
| ATOM | 1177 | CZ3 | TRP A 148 | 42.851 | 21.525 | -5.530 | 1.00 | 14.91 | C |
| ATOM | 1178 | CH2 | TRP A 148 | 41.740 | 21.028 | -4.872 | 1.00 | 13.79 | C |
| ATOM | 1179 | N | LYS A 149 | 47.153 | 25.484 | -4.819 | 1.00 | 18.51 | N |
| ATOM | 1180 | CA | LYS A 149 | 48.113 | 26.070 | -5.746 | 1.00 | 21.41 | C |
| ATOM | 1181 | C | LYS A 149 | 47.937 | 25.514 | -7.159 | 1.00 | 21.21 | C |
| ATOM | 1182 | O | LYS A 149 | 47.627 | 24.328 | -7.353 | 1.00 | 20.29 | O |
| ATOM | 1183 | CB | LYS A 149 | 49.544 | 25.836 | -5.280 | 1.00 | 21.58 | C |
| ATOM | 1184 | CG | LYS A 149 | 49.895 | 26.516 | -3.956 | 1.00 | 23.30 | C |
| ATOM | 1185 | CD | LYS A 149 | 51.241 | 26.042 | -3.495 | 1.00 | 25.70 | C |
| ATOM | 1186 | CE | LYS A 149 | 51.702 | 26.770 | -2.247 | 1.00 | 28.91 | C |
| ATOM | 1187 | NZ | LYS A 149 | 53.013 | 26.185 | -1.805 | 1.00 | 29.57 | N |
| ATOM | 1188 | N | ILE A 150 | 48.155 | 26.383 | -8.141 | 1.00 | 20.56 | N |
| ATOM | 1189 | CA | ILE A 150 | 48.138 | 26.002 | -9.542 | 1.00 | 21.18 | C |
| ATOM | 1190 | C | ILE A 150 | 49.483 | 26.393 | -10.150 | 1.00 | 22.10 | C |
| ATOM | 1191 | O | ILE A 150 | 49.840 | 27.550 | -10.139 | 1.00 | 21.80 | O |
| ATOM | 1192 | CB | ILE A 150 | 47.012 | 26.721 | -10.306 | 1.00 | 20.89 | C |
| ATOM | 1193 | CG1 | ILE A 150 | 45.651 | 26.400 | -9.672 | 1.00 | 21.30 | C |
| ATOM | 1194 | CG2 | ILE A 150 | 47.042 | 26.328 | -11.774 | 1.00 | 18.65 | C |
| ATOM | 1195 | CD1 | ILE A 150 | 44.472 | 27.103 | -10.325 | 1.00 | 22.75 | C |
| ATOM | 1196 | N | ASP A 151 | 50.219 | 25.408 | -10.639 | 1.00 | 23.49 | N |
| ATOM | 1197 | CA | ASP A 151 | 51.575 | 25.600 | -11.146 | 1.00 | 26.43 | C |
| ATOM | 1198 | C | ASP A 151 | 52.426 | 26.321 | -10.111 | 1.00 | 28.35 | C |
| ATOM | 1199 | O | ASP A 151 | 53.213 | 27.202 | -10.450 | 1.00 | 28.85 | O |
| ATOM | 1200 | CB | ASP A 151 | 51.550 | 26.390 | -12.459 | 1.00 | 26.75 | C |
| ATOM | 1201 | CG | ASP A 151 | 51.079 | 25.564 | -13.643 | 1.00 | 27.59 | C |
| ATOM | 1202 | OD1 | ASP A 151 | 51.021 | 24.318 | -13.542 | 1.00 | 29.31 | O |
| ATOM | 1203 | OD2 | ASP A 151 | 50.814 | 26.173 | -14.699 | 1.00 | 31.24 | O |
| ATOM | 1204 | N | GLY A 152 | 52.226 | 25.973 | -8.844 | 1.00 | 30.16 | N |
| ATOM | 1205 | CA | GLY A 152 | 53.027 | 26.527 | -7.761 | 1.00 | 30.94 | C |
| ATOM | 1206 | C | GLY A 152 | 52.509 | 27.779 | -7.097 | 1.00 | 31.75 | C |
| ATOM | 1207 | O | GLY A 152 | 52.954 | 28.082 | -6.000 | 1.00 | 33.78 | O |
| ATOM | 1208 | N | SER A 153 | 51.581 | 28.506 | -7.726 | 1.00 | 31.64 | N |
| ATOM | 1209 | CA | SER A 153 | 51.096 | 29.762 | -7.163 | 1.00 | 31.27 | C |
| ATOM | 1210 | C | SER A 153 | 49.775 | 29.572 | -6.450 | 1.00 | 29.76 | C |
| ATOM | 1211 | O | SER A 153 | 48.907 | 28.850 | -6.934 | 1.00 | 28.73 | O |
| ATOM | 1212 | CB | SER A 153 | 50.932 | 30.816 | -8.252 | 1.00 | 31.96 | C |
| ATOM | 1213 | OG | SER A 153 | 52.038 | 30.797 | -9.139 | 1.00 | 36.03 | O |
| ATOM | 1214 | N | GLU A 154 | 49.627 | 30.237 | -5.304 | 1.00 | 28.66 | N |
| ATOM | 1215 | CA | GLU A 154 | 48.427 | 30.137 | -4.495 | 1.00 | 28.68 | C |
| ATOM | 1216 | C | GLU A 154 | 47.230 | 30.616 | -5.289 | 1.00 | 28.51 | C |
| ATOM | 1217 | O | GLU A 154 | 47.263 | 31.687 | -5.909 | 1.00 | 27.84 | O |
| ATOM | 1218 | CB | GLU A 154 | 48.547 | 30.964 | -3.211 | 1.00 | 28.41 | C |
| ATOM | 1219 | CG | GLU A 154 | 47.306 | 30.918 | -2.313 | 1.00 | 29.31 | C |
| ATOM | 1220 | CD | GLU A 154 | 47.532 | 31.542 | -0.945 | 1.00 | 30.98 | C |
| ATOM | 1221 | OE1 | GLU A 154 | 48.708 | 31.765 | -0.601 | 1.00 | 33.34 | O |
| ATOM | 1222 | OE2 | GLU A 154 | 46.543 | 31.802 | -0.205 | 1.00 | 34.30 | O |
| ATOM | 1223 | N | ARG A 155 | 46.174 | 29.817 | -5.268 | 1.00 | 27.66 | N |

```
ATOM   1224  CA   ARG A 155      44.921  30.183  -5.900  1.00 28.04           C
ATOM   1225  C    ARG A 155      43.867  30.376  -4.825  1.00 29.88           C
ATOM   1226  O    ARG A 155      43.683  29.501  -3.985  1.00 28.50           O
ATOM   1227  CB   ARG A 155      44.488  29.061  -6.837  1.00 27.67           C
ATOM   1228  CG   ARG A 155      43.061  29.138  -7.253  1.00 27.08           C
ATOM   1229  CD   ARG A 155      42.867  30.241  -8.235  1.00 26.98           C
ATOM   1230  NE   ARG A 155      41.509  30.211  -8.728  1.00 25.74           N
ATOM   1231  CZ   ARG A 155      41.141  30.432  -9.987  1.00 27.76           C
ATOM   1232  NH1  ARG A 155      42.025  30.665 -10.956  1.00 27.00           N
ATOM   1233  NH2  ARG A 155      39.847  30.396 -10.272  1.00 28.83           N
ATOM   1234  N    GLN A 156      43.137  31.486  -4.873  1.00 31.95           N
ATOM   1235  CA   GLN A 156      42.114  31.761  -3.853  1.00 34.36           C
ATOM   1236  C    GLN A 156      40.648  31.609  -4.314  1.00 34.32           C
ATOM   1237  O    GLN A 156      39.841  30.953  -3.647  1.00 35.91           O
ATOM   1238  CB   GLN A 156      42.349  33.157  -3.265  1.00 35.89           C
ATOM   1239  CG   GLN A 156      43.810  33.363  -2.827  1.00 38.35           C
ATOM   1240  CD   GLN A 156      43.970  34.189  -1.564  1.00 40.58           C
ATOM   1241  OE1  GLN A 156      42.990  34.607  -0.951  1.00 41.56           O
ATOM   1242  NE2  GLN A 156      45.223  34.418  -1.161  1.00 42.76           N
ATOM   1243  N    ASN A 157      40.285  32.215  -5.432  1.00 33.06           N
ATOM   1244  CA   ASN A 157      38.864  32.227  -5.822  1.00 31.32           C
ATOM   1245  C    ASN A 157      38.458  30.993  -6.636  1.00 26.41           C
ATOM   1246  O    ASN A 157      39.290  30.350  -7.243  1.00 25.07           O
ATOM   1247  CB   ASN A 157      38.542  33.522  -6.540  1.00 34.02           C
ATOM   1248  CG   ASN A 157      39.206  34.718  -5.876  1.00 36.63           C
ATOM   1249  OD1  ASN A 157      38.915  35.060  -4.711  1.00 38.81           O
ATOM   1250  ND2  ASN A 157      40.173  35.309  -6.582  1.00 40.19           N
ATOM   1251  N    GLY A 158      37.175  30.658  -6.579  1.00 23.45           N
ATOM   1252  CA   GLY A 158      36.629  29.419  -7.157  1.00 21.51           C
ATOM   1253  C    GLY A 158      36.618  28.216  -6.222  1.00 19.70           C
ATOM   1254  O    GLY A 158      36.272  27.108  -6.646  1.00 20.00           O
ATOM   1255  N    VAL A 159      36.976  28.440  -4.959  1.00 18.96           N
ATOM   1256  CA   VAL A 159      37.158  27.381  -3.965  1.00 19.87           C
ATOM   1257  C    VAL A 159      35.968  27.382  -3.032  1.00 21.52           C
ATOM   1258  O    VAL A 159      35.607  28.418  -2.482  1.00 21.98           O
ATOM   1259  CB   VAL A 159      38.469  27.599  -3.147  1.00 18.98           C
ATOM   1260  CG1  VAL A 159      38.582  26.583  -1.983  1.00 19.91           C
ATOM   1261  CG2  VAL A 159      39.685  27.511  -4.044  1.00 18.29           C
ATOM   1262  N    LEU A 160      35.350  26.223  -2.850  1.00 20.18           N
ATOM   1263  CA   LEU A 160      34.255  26.101  -1.921  1.00 20.75           C
ATOM   1264  C    LEU A 160      34.586  24.996  -0.923  1.00 19.36           C
ATOM   1265  O    LEU A 160      34.974  23.914  -1.336  1.00 18.39           O
ATOM   1266  CB   LEU A 160      32.986  25.763  -2.676  1.00 22.47           C
ATOM   1267  CG   LEU A 160      32.289  26.950  -3.366  1.00 25.08           C
ATOM   1268  CD1  LEU A 160      31.080  26.426  -4.072  1.00 27.60           C
ATOM   1269  CD2  LEU A 160      31.913  28.040  -2.357  1.00 27.78           C
ATOM   1270  N    ASN A 161      34.436  25.296   0.371  1.00 20.04           N
ATOM   1271  CA   ASN A 161      34.717  24.344   1.463  1.00 18.96           C
ATOM   1272  C    ASN A 161      33.456  23.868   2.201  1.00 19.34           C
ATOM   1273  O    ASN A 161      32.475  24.606   2.318  1.00 19.22           O
ATOM   1274  CB   ASN A 161      35.711  24.960   2.454  1.00 19.73           C
ATOM   1275  CG   ASN A 161      37.056  25.273   1.836  1.00 19.39           C
ATOM   1276  OD1  ASN A 161      37.740  26.201   2.281  1.00 24.55           O
ATOM   1277  ND2  ASN A 161      37.434  24.550   0.800  1.00 17.81           N
ATOM   1278  N    SER A 162      33.471  22.620   2.680  1.00 18.12           N
ATOM   1279  CA   SER A 162      32.365  22.065   3.465  1.00 18.22           C
ATOM   1280  C    SER A 162      32.899  21.099   4.531  1.00 19.74           C
ATOM   1281  O    SER A 162      33.749  20.271   4.235  1.00 17.29           O
ATOM   1282  CB   SER A 162      31.381  21.320   2.584  1.00 18.64           C
ATOM   1283  OG   SER A 162      30.244  20.905   3.323  1.00 17.96           O
ATOM   1284  N    TRP A 163      32.359  21.209   5.747  1.00 20.61           N
ATOM   1285  CA   TRP A 163      32.820  20.465   6.901  1.00 21.75           C
ATOM   1286  C    TRP A 163      31.722  19.538   7.403  1.00 20.97           C
ATOM   1287  O    TRP A 163      30.573  19.951   7.535  1.00 18.34           O
ATOM   1288  CB   TRP A 163      33.187  21.460   8.008  1.00 25.11           C
```

```
ATOM   1289  CG   TRP A 163      34.264  22.468   7.622  1.00 26.46           C
ATOM   1290  CD1  TRP A 163      35.577  22.386   7.906  1.00 27.23           C
ATOM   1291  CD2  TRP A 163      34.086  23.694   6.889  1.00 26.57           C
ATOM   1292  NE1  TRP A 163      36.243  23.467   7.407  1.00 28.42           N
ATOM   1293  CE2  TRP A 163      35.352  24.290   6.773  1.00 25.53           C
ATOM   1294  CE3  TRP A 163      32.969  24.350   6.340  1.00 26.84           C
ATOM   1295  CZ2  TRP A 163      35.554  25.515   6.126  1.00 27.38           C
ATOM   1296  CZ3  TRP A 163      33.164  25.580   5.688  1.00 27.27           C
ATOM   1297  CH2  TRP A 163      34.448  26.145   5.588  1.00 27.10           C
ATOM   1298  N    THR A 164      32.063  18.289   7.706  1.00 19.84           N
ATOM   1299  CA   THR A 164      31.113  17.394   8.343  1.00 19.68           C
ATOM   1300  C    THR A 164      30.928  17.764   9.816  1.00 20.41           C
ATOM   1301  O    THR A 164      31.754  18.478  10.419  1.00 19.56           O
ATOM   1302  CB   THR A 164      31.558  15.931   8.293  1.00 20.67           C
ATOM   1303  OG1  THR A 164      32.832  15.808   8.943  1.00 20.86           O
ATOM   1304  CG2  THR A 164      31.648  15.430   6.850  1.00 21.51           C
ATOM   1305  N    ASP A 165      29.822  17.270  10.364  1.00 20.80           N
ATOM   1306  CA   ASP A 165      29.614  17.192  11.814  1.00 22.09           C
ATOM   1307  C    ASP A 165      30.533  16.143  12.415  1.00 21.07           C
ATOM   1308  O    ASP A 165      31.060  15.289  11.711  1.00 19.49           O
ATOM   1309  CB   ASP A 165      28.162  16.801  12.123  1.00 23.41           C
ATOM   1310  CG   ASP A 165      27.160  17.942  11.864  1.00 26.24           C
ATOM   1311  OD1  ASP A 165      27.539  19.137  11.938  1.00 28.83           O
ATOM   1312  OD2  ASP A 165      25.972  17.634  11.650  1.00 31.23           O
ATOM   1313  N    GLN A 166      30.696  16.170  13.732  1.00 19.02           N
ATOM   1314  CA   GLN A 166      31.541  15.169  14.372  1.00 18.08           C
ATOM   1315  C    GLN A 166      30.991  13.758  14.152  1.00 18.58           C
ATOM   1316  O    GLN A 166      29.801  13.513  14.311  1.00 17.54           O
ATOM   1317  CB   GLN A 166      31.679  15.458  15.867  1.00 16.95           C
ATOM   1318  CG   GLN A 166      32.698  14.552  16.542  1.00 17.41           C
ATOM   1319  CD   GLN A 166      33.033  15.012  17.934  1.00 18.25           C
ATOM   1320  OE1  GLN A 166      32.155  15.478  18.682  1.00 17.93           O
ATOM   1321  NE2  GLN A 166      34.307  14.914  18.291  1.00 17.21           N
ATOM   1322  N    ASP A 167      31.865  12.835  13.764  1.00 20.49           N
ATOM   1323  CA   ASP A 167      31.451  11.501  13.409  1.00 22.40           C
ATOM   1324  C    ASP A 167      30.998  10.737  14.641  1.00 23.30           C
ATOM   1325  O    ASP A 167      31.628  10.817  15.685  1.00 23.32           O
ATOM   1326  CB   ASP A 167      32.597  10.733  12.765  1.00 23.20           C
ATOM   1327  CG   ASP A 167      32.143   9.401  12.200  1.00 26.49           C
ATOM   1328  OD1  ASP A 167      31.425   9.422  11.172  1.00 30.09           O
ATOM   1329  OD2  ASP A 167      32.482   8.341  12.778  1.00 26.38           O
ATOM   1330  N    SER A 168      29.933   9.965  14.481  1.00 24.48           N
ATOM   1331  CA   SER A 168      29.317   9.209  15.573  1.00 25.57           C
ATOM   1332  C    SER A 168      30.115   7.970  15.955  1.00 25.65           C
ATOM   1333  O    SER A 168      29.872   7.410  17.010  1.00 25.94           O
ATOM   1334  CB   SER A 168      27.884   8.787  15.189  1.00 26.04           C
ATOM   1335  OG   SER A 168      26.960   9.874  15.287  1.00 29.00           O
ATOM   1336  N    LYS A 169      31.041   7.525  15.097  1.00 26.09           N
ATOM   1337  CA   LYS A 169      31.833   6.327  15.384  1.00 26.69           C
ATOM   1338  C    LYS A 169      33.228   6.633  15.859  1.00 25.94           C
ATOM   1339  O    LYS A 169      33.686   6.051  16.845  1.00 27.81           O
ATOM   1340  CB   LYS A 169      31.915   5.405  14.163  1.00 28.21           C
ATOM   1341  CG   LYS A 169      30.775   4.396  14.075  1.00 31.56           C
ATOM   1342  CD   LYS A 169      29.483   5.030  13.589  1.00 34.24           C
ATOM   1343  CE   LYS A 169      28.235   4.336  14.158  1.00 36.00           C
ATOM   1344  NZ   LYS A 169      27.918   4.657  15.597  1.00 37.75           N
ATOM   1345  N    ASP A 170      33.933   7.527  15.172  1.00 23.13           N
ATOM   1346  CA   ASP A 170      35.326   7.781  15.534  1.00 22.15           C
ATOM   1347  C    ASP A 170      35.604   9.181  16.108  1.00 18.50           C
ATOM   1348  O    ASP A 170      36.731   9.513  16.398  1.00 18.50           O
ATOM   1349  CB   ASP A 170      36.256   7.431  14.368  1.00 23.00           C
ATOM   1350  CG   ASP A 170      36.229   8.454  13.242  1.00 23.80           C
ATOM   1351  OD1  ASP A 170      35.619   9.533  13.379  1.00 22.34           O
ATOM   1352  OD2  ASP A 170      36.876   8.165  12.220  1.00 27.72           O
ATOM   1353  N    SER A 171      34.561   9.989  16.265  1.00 16.68           N
```

| ATOM | 1354 | CA | SER A 171 | 34.671 | 11.301 | 16.892 | 1.00 | 17.20 | C |
|------|------|-----|-----------|--------|--------|--------|------|-------|---|
| ATOM | 1355 | C | SER A 171 | 35.576 | 12.280 | 16.136 | 1.00 | 15.95 | C |
| ATOM | 1356 | O | SER A 171 | 36.022 | 13.319 | 16.688 | 1.00 | 16.96 | O |
| ATOM | 1357 | CB | SER A 171 | 35.098 | 11.135 | 18.361 | 1.00 | 17.87 | C |
| ATOM | 1358 | OG | SER A 171 | 34.173 | 10.273 | 19.015 | 1.00 | 17.98 | O |
| ATOM | 1359 | N | THR A 172 | 35.789 | 11.990 | 14.856 | 1.00 | 15.86 | N |
| ATOM | 1360 | CA | THR A 172 | 36.565 | 12.883 | 13.980 | 1.00 | 14.67 | C |
| ATOM | 1361 | C | THR A 172 | 35.670 | 13.827 | 13.155 | 1.00 | 15.49 | C |
| ATOM | 1362 | O | THR A 172 | 34.442 | 13.705 | 13.129 | 1.00 | 15.09 | O |
| ATOM | 1363 | CB | THR A 172 | 37.506 | 12.108 | 13.021 | 1.00 | 15.40 | C |
| ATOM | 1364 | OG1 | THR A 172 | 36.744 | 11.351 | 12.077 | 1.00 | 16.34 | O |
| ATOM | 1365 | CG2 | THR A 172 | 38.465 | 11.207 | 13.821 | 1.00 | 15.24 | C |
| ATOM | 1366 | N | TYR A 173 | 36.342 | 14.744 | 12.482 | 1.00 | 15.12 | N |
| ATOM | 1367 | CA | TYR A 173 | 35.755 | 15.673 | 11.520 | 1.00 | 14.75 | C |
| ATOM | 1368 | C | TYR A 173 | 36.388 | 15.448 | 10.155 | 1.00 | 14.93 | C |
| ATOM | 1369 | O | TYR A 173 | 37.498 | 14.940 | 10.073 | 1.00 | 14.03 | O |
| ATOM | 1370 | CB | TYR A 173 | 36.085 | 17.087 | 11.967 | 1.00 | 16.61 | C |
| ATOM | 1371 | CG | TYR A 173 | 35.460 | 17.449 | 13.302 | 1.00 | 16.95 | C |
| ATOM | 1372 | CD1 | TYR A 173 | 36.191 | 17.379 | 14.495 | 1.00 | 17.53 | C |
| ATOM | 1373 | CD2 | TYR A 173 | 34.125 | 17.822 | 13.373 | 1.00 | 17.17 | C |
| ATOM | 1374 | CE1 | TYR A 173 | 35.595 | 17.730 | 15.734 | 1.00 | 17.74 | C |
| ATOM | 1375 | CE2 | TYR A 173 | 33.532 | 18.158 | 14.602 | 1.00 | 17.21 | C |
| ATOM | 1376 | CZ | TYR A 173 | 34.265 | 18.097 | 15.763 | 1.00 | 18.30 | C |
| ATOM | 1377 | OH | TYR A 173 | 33.646 | 18.446 | 16.956 | 1.00 | 19.22 | O |
| ATOM | 1378 | N | SER A 174 | 35.688 | 15.854 | 9.084 | 1.00 | 13.45 | N |
| ATOM | 1379 | CA | SER A 174 | 36.257 | 15.821 | 7.739 | 1.00 | 12.78 | C |
| ATOM | 1380 | C | SER A 174 | 35.844 | 17.126 | 7.014 | 1.00 | 14.08 | C |
| ATOM | 1381 | O | SER A 174 | 34.892 | 17.802 | 7.430 | 1.00 | 12.46 | O |
| ATOM | 1382 | CB | SER A 174 | 35.785 | 14.578 | 6.981 | 1.00 | 15.13 | C |
| ATOM | 1383 | OG | SER A 174 | 36.171 | 13.358 | 7.635 | 1.00 | 15.37 | O |
| ATOM | 1384 | N | MET A 175 | 36.579 | 17.477 | 5.962 | 1.00 | 15.43 | N |
| ATOM | 1385 | CA | MET A 175 | 36.309 | 18.680 | 5.189 | 1.00 | 18.28 | C |
| ATOM | 1386 | C | MET A 175 | 36.556 | 18.356 | 3.719 | 1.00 | 17.06 | C |
| ATOM | 1387 | O | MET A 175 | 37.448 | 17.580 | 3.414 | 1.00 | 14.53 | O |
| ATOM | 1388 | CB | MET A 175 | 37.237 | 19.792 | 5.644 | 1.00 | 17.58 | C |
| ATOM | 1389 | CG | MET A 175 | 36.873 | 21.181 | 5.161 | 1.00 | 24.18 | C |
| ATOM | 1390 | SD | MET A 175 | 38.349 | 22.234 | 5.098 | 1.00 | 28.91 | S |
| ATOM | 1391 | CE | MET A 175 | 38.873 | 21.599 | 3.522 | 1.00 | 30.03 | C |
| ATOM | 1392 | N | SER A 176 | 35.755 | 18.928 | 2.820 | 1.00 | 15.90 | N |
| ATOM | 1393 | CA | SER A 176 | 36.027 | 18.824 | 1.382 | 1.00 | 16.64 | C |
| ATOM | 1394 | C | SER A 176 | 36.328 | 20.216 | 0.915 | 1.00 | 17.44 | C |
| ATOM | 1395 | O | SER A 176 | 35.733 | 21.163 | 1.410 | 1.00 | 17.72 | O |
| ATOM | 1396 | CB | SER A 176 | 34.817 | 18.348 | 0.594 | 1.00 | 18.61 | C |
| ATOM | 1397 | OG | SER A 176 | 33.819 | 19.351 | 0.692 | 1.00 | 20.69 | O |
| ATOM | 1398 | N | SER A 177 | 37.257 | 20.327 | -0.030 | 1.00 | 16.16 | N |
| ATOM | 1399 | CA | SER A 177 | 37.567 | 21.602 | -0.650 | 1.00 | 15.88 | C |
| ATOM | 1400 | C | SER A 177 | 37.491 | 21.349 | -2.141 | 1.00 | 14.30 | C |
| ATOM | 1401 | O | SER A 177 | 38.067 | 20.374 | -2.624 | 1.00 | 12.39 | O |
| ATOM | 1402 | CB | SER A 177 | 38.938 | 22.106 | -0.241 | 1.00 | 15.57 | C |
| ATOM | 1403 | OG | SER A 177 | 39.119 | 23.402 | -0.746 | 1.00 | 17.65 | O |
| ATOM | 1404 | N | THR A 178 | 36.741 | 22.181 | -2.865 | 1.00 | 13.02 | N |
| ATOM | 1405 | CA | THR A 178 | 36.547 | 21.972 | -4.296 | 1.00 | 14.11 | C |
| ATOM | 1406 | C | THR A 178 | 36.930 | 23.253 | -5.023 | 1.00 | 13.51 | C |
| ATOM | 1407 | O | THR A 178 | 36.415 | 24.298 | -4.696 | 1.00 | 13.20 | O |
| ATOM | 1408 | CB | THR A 178 | 35.104 | 21.587 | -4.623 | 1.00 | 15.80 | C |
| ATOM | 1409 | OG1 | THR A 178 | 34.729 | 20.428 | -3.860 | 1.00 | 17.47 | O |
| ATOM | 1410 | CG2 | THR A 178 | 34.950 | 21.267 | -6.096 | 1.00 | 17.10 | C |
| ATOM | 1411 | N | LEU A 179 | 37.842 | 23.147 | -5.984 | 1.00 | 13.13 | N |
| ATOM | 1412 | CA | LEU A 179 | 38.258 | 24.277 | -6.808 | 1.00 | 12.57 | C |
| ATOM | 1413 | C | LEU A 179 | 37.561 | 24.117 | -8.151 | 1.00 | 12.93 | C |
| ATOM | 1414 | O | LEU A 179 | 37.771 | 23.122 | -8.817 | 1.00 | 10.46 | O |
| ATOM | 1415 | CB | LEU A 179 | 39.759 | 24.261 | -6.992 | 1.00 | 14.59 | C |
| ATOM | 1416 | CG | LEU A 179 | 40.379 | 25.195 | -8.029 | 1.00 | 15.08 | C |
| ATOM | 1417 | CD1 | LEU A 179 | 40.006 | 26.630 | -7.750 | 1.00 | 17.57 | C |
| ATOM | 1418 | CD2 | LEU A 179 | 41.884 | 24.998 | -8.097 | 1.00 | 16.43 | C |

```
ATOM   1419  N    THR A 180      36.717  25.057   -8.549  1.00 12.15          N
ATOM   1420  CA   THR A 180      35.951  24.850   -9.788  1.00 12.87          C
ATOM   1421  C    THR A 180      36.365  25.865  -10.830  1.00 14.10          C
ATOM   1422  O    THR A 180      36.335  27.083  -10.594  1.00 12.67          O
ATOM   1423  CB   THR A 180      34.438  24.802   -9.582  1.00 14.56          C
ATOM   1424  OG1  THR A 180      34.104  23.656   -8.797  1.00 15.96          O
ATOM   1425  CG2  THR A 180      33.681  24.676  -10.919  1.00 13.08          C
ATOM   1426  N    LEU A 181      36.776  25.331  -11.964  1.00 12.80          N
ATOM   1427  CA   LEU A 181      37.222  26.113  -13.101  1.00 12.85          C
ATOM   1428  C    LEU A 181      36.418  25.735  -14.340  1.00 13.05          C
ATOM   1429  O    LEU A 181      35.726  24.703  -14.362  1.00 12.45          O
ATOM   1430  CB   LEU A 181      38.702  25.865  -13.353  1.00 12.32          C
ATOM   1431  CG   LEU A 181      39.704  26.069  -12.193  1.00 16.43          C
ATOM   1432  CD1  LEU A 181      41.052  25.448  -12.534  1.00 18.58          C
ATOM   1433  CD2  LEU A 181      39.842  27.536  -11.861  1.00 19.17          C
ATOM   1434  N    THR A 182      36.518  26.556  -15.388  1.00 12.27          N
ATOM   1435  CA   THR A 182      36.062  26.158  -16.716  1.00 11.05          C
ATOM   1436  C    THR A 182      37.089  25.174  -17.236  1.00 10.78          C
ATOM   1437  O    THR A 182      38.242  25.220  -16.807  1.00 10.20          O
ATOM   1438  CB   THR A 182      36.012  27.370  -17.699  1.00 12.33          C
ATOM   1439  OG1  THR A 182      37.308  27.984  -17.776  1.00 10.93          O
ATOM   1440  CG2  THR A 182      34.937  28.344  -17.267  1.00 13.13          C
ATOM   1441  N    LYS A 183      36.684  24.288  -18.139  1.00 10.14          N
ATOM   1442  CA   LYS A 183      37.625  23.480  -18.894  1.00  9.98          C
ATOM   1443  C    LYS A 183      38.722  24.321  -19.513  1.00 10.77          C
ATOM   1444  O    LYS A 183      39.917  23.994  -19.427  1.00  8.81          O
ATOM   1445  CB   LYS A 183      36.897  22.716  -20.011  1.00 11.32          C
ATOM   1446  CG   LYS A 183      37.815  21.859  -20.818  1.00 12.05          C
ATOM   1447  CD   LYS A 183      37.085  21.084  -21.906  1.00 12.97          C
ATOM   1448  CE   LYS A 183      38.090  20.509  -22.891  1.00 16.89          C
ATOM   1449  NZ   LYS A 183      37.438  19.944  -24.082  1.00 16.48          N
ATOM   1450  N    ASP A 184      38.333  25.448  -20.086  1.00  9.90          N
ATOM   1451  CA   ASP A 184      39.316  26.293  -20.762  1.00 11.64          C
ATOM   1452  C    ASP A 184      40.431  26.726  -19.818  1.00 11.61          C
ATOM   1453  O    ASP A 184      41.608  26.620  -20.168  1.00 13.08          O
ATOM   1454  CB   ASP A 184      38.635  27.491  -21.422  1.00 11.69          C
ATOM   1455  CG   ASP A 184      39.619  28.473  -21.977  1.00 17.06          C
ATOM   1456  OD1  ASP A 184      40.393  28.097  -22.893  1.00 19.32          O
ATOM   1457  OD2  ASP A 184      39.641  29.620  -21.454  1.00 17.98          O
ATOM   1458  N    GLU A 185      40.074  27.246  -18.654  1.00 12.75          N
ATOM   1459  CA   GLU A 185      41.079  27.700  -17.702  1.00 14.51          C
ATOM   1460  C    GLU A 185      41.905  26.519  -17.148  1.00 14.24          C
ATOM   1461  O    GLU A 185      43.113  26.638  -17.013  1.00 12.65          O
ATOM   1462  CB   GLU A 185      40.467  28.477  -16.553  1.00 15.61          C
ATOM   1463  CG   GLU A 185      41.557  29.051  -15.631  1.00 17.90          C
ATOM   1464  CD   GLU A 185      41.075  30.086  -14.646  1.00 19.09          C
ATOM   1465  OE1  GLU A 185      41.949  30.666  -13.977  1.00 21.83          O
ATOM   1466  OE2  GLU A 185      39.856  30.307  -14.507  1.00 21.72          O
ATOM   1467  N    TYR A 186      41.229  25.407  -16.843  1.00 13.12          N
ATOM   1468  CA   TYR A 186      41.882  24.199  -16.321  1.00 14.01          C
ATOM   1469  C    TYR A 186      42.987  23.701  -17.232  1.00 14.63          C
ATOM   1470  O    TYR A 186      44.053  23.277  -16.771  1.00 14.23          O
ATOM   1471  CB   TYR A 186      40.841  23.091  -16.133  1.00 13.22          C
ATOM   1472  CG   TYR A 186      41.430  21.743  -15.743  1.00 12.75          C
ATOM   1473  CD1  TYR A 186      42.041  21.562  -14.489  1.00 14.76          C
ATOM   1474  CD2  TYR A 186      41.342  20.657  -16.577  1.00 12.34          C
ATOM   1475  CE1  TYR A 186      42.559  20.325  -14.096  1.00 12.58          C
ATOM   1476  CE2  TYR A 186      41.861  19.405  -16.183  1.00 11.27          C
ATOM   1477  CZ   TYR A 186      42.496  19.277  -14.956  1.00 13.40          C
ATOM   1478  OH   TYR A 186      43.021  18.059  -14.547  1.00 14.79          O
ATOM   1479  N    GLU A 187      42.730  23.713  -18.528  1.00 14.56          N
ATOM   1480  CA   GLU A 187      43.695  23.181  -19.484  1.00 16.60          C
ATOM   1481  C    GLU A 187      44.813  24.186  -19.826  1.00 17.14          C
ATOM   1482  O    GLU A 187      45.683  23.868  -20.631  1.00 15.73          O
ATOM   1483  CB   GLU A 187      42.956  22.661  -20.719  1.00 16.38          C
```

```
ATOM  1484  CG   GLU A 187    42.044  21.496 -20.369  1.00 16.74           C
ATOM  1485  CD   GLU A 187    41.415  20.772 -21.539  1.00 19.60           C
ATOM  1486  OE1  GLU A 187    41.140  21.395 -22.600  1.00 21.29           O
ATOM  1487  OE2  GLU A 187    41.152  19.547 -21.363  1.00 22.74           O
ATOM  1488  N    ARG A 188    44.815  25.376 -19.210  1.00 17.99           N
ATOM  1489  CA   ARG A 188    45.933  26.316 -19.357  1.00 20.58           C
ATOM  1490  C    ARG A 188    47.019  26.141 -18.294  1.00 22.13           C
ATOM  1491  O    ARG A 188    48.019  26.880 -18.308  1.00 22.78           O
ATOM  1492  CB   ARG A 188    45.421  27.762 -19.369  1.00 21.51           C
ATOM  1493  CG   ARG A 188    44.676  28.053 -20.632  1.00 24.11           C
ATOM  1494  CD   ARG A 188    43.922  29.348 -20.603  1.00 25.99           C
ATOM  1495  NE   ARG A 188    43.030  29.420 -21.766  1.00 28.39           N
ATOM  1496  CZ   ARG A 188    43.372  29.887 -22.971  1.00 30.66           C
ATOM  1497  NH1  ARG A 188    44.604  30.339 -23.210  1.00 32.18           N
ATOM  1498  NH2  ARG A 188    42.479  29.919 -23.954  1.00 31.43           N
ATOM  1499  N    HIS A 189    46.856  25.156 -17.405  1.00 21.97           N
ATOM  1500  CA   HIS A 189    47.808  24.940 -16.303  1.00 22.86           C
ATOM  1501  C    HIS A 189    48.049  23.461 -16.171  1.00 23.12           C
ATOM  1502  O    HIS A 189    47.202  22.672 -16.589  1.00 24.76           O
ATOM  1503  CB   HIS A 189    47.264  25.510 -14.989  1.00 22.75           C
ATOM  1504  CG   HIS A 189    46.853  26.943 -15.074  1.00 24.97           C
ATOM  1505  ND1  HIS A 189    47.761  27.978 -15.086  1.00 24.67           N
ATOM  1506  CD2  HIS A 189    45.629  27.514 -15.162  1.00 25.55           C
ATOM  1507  CE1  HIS A 189    47.114  29.127 -15.176  1.00 27.52           C
ATOM  1508  NE2  HIS A 189    45.818  28.872 -15.226  1.00 28.12           N
ATOM  1509  N    ASN A 190    49.184  23.057 -15.597  1.00 23.51           N
ATOM  1510  CA   ASN A 190    49.493  21.621 -15.523  1.00 25.45           C
ATOM  1511  C    ASN A 190    49.441  21.008 -14.139  1.00 23.54           C
ATOM  1512  O    ASN A 190    48.980  19.900 -14.005  1.00 23.89           O
ATOM  1513  CB   ASN A 190    50.847  21.271 -16.167  1.00 27.54           C
ATOM  1514  CG   ASN A 190    50.926  19.791 -16.553  1.00 31.12           C
ATOM  1515  OD1  ASN A 190    50.059  19.285 -17.276  1.00 35.48           O
ATOM  1516  ND2  ASN A 190    51.935  19.089 -16.047  1.00 33.84           N
ATOM  1517  N    SER A 191    49.931  21.725 -13.132  1.00 22.99           N
ATOM  1518  CA   SER A 191    50.070  21.172 -11.794  1.00 22.43           C
ATOM  1519  C    SER A 191    49.014  21.746 -10.838  1.00 20.86           C
ATOM  1520  O    SER A 191    48.775  22.954 -10.782  1.00 20.00           O
ATOM  1521  CB   SER A 191    51.505  21.372 -11.283  1.00 23.32           C
ATOM  1522  OG   SER A 191    51.544  21.701  -9.907  1.00 26.33           O
ATOM  1523  N    TYR A 192    48.364  20.846 -10.110  1.00 19.08           N
ATOM  1524  CA   TYR A 192    47.298  21.223  -9.186  1.00 17.51           C
ATOM  1525  C    TYR A 192    47.599  20.633  -7.819  1.00 16.36           C
ATOM  1526  O    TYR A 192    47.812  19.431  -7.694  1.00 16.93           O
ATOM  1527  CB   TYR A 192    45.940  20.719  -9.712  1.00 15.54           C
ATOM  1528  CG   TYR A 192    45.461  21.526 -10.892  1.00 13.92           C
ATOM  1529  CD1  TYR A 192    44.679  22.652 -10.712  1.00 12.85           C
ATOM  1530  CD2  TYR A 192    45.869  21.208 -12.173  1.00 13.80           C
ATOM  1531  CE1  TYR A 192    44.289  23.439 -11.802  1.00 12.45           C
ATOM  1532  CE2  TYR A 192    45.488  21.973 -13.258  1.00 15.18           C
ATOM  1533  CZ   TYR A 192    44.720  23.100 -13.065  1.00 14.26           C
ATOM  1534  OH   TYR A 192    44.319  23.835 -14.158  1.00 15.46           O
ATOM  1535  N    THR A 193    47.585  21.493  -6.805  1.00 16.45           N
ATOM  1536  CA   THR A 193    47.997  21.114  -5.451  1.00 17.47           C
ATOM  1537  C    THR A 193    47.026  21.582  -4.368  1.00 17.06           C
ATOM  1538  O    THR A 193    46.549  22.716  -4.405  1.00 16.24           O
ATOM  1539  CB   THR A 193    49.405  21.719  -5.134  1.00 17.36           C
ATOM  1540  OG1  THR A 193    50.368  21.239  -6.082  1.00 20.74           O
ATOM  1541  CG2  THR A 193    49.845  21.351  -3.743  1.00 19.20           C
ATOM  1542  N    CYS A 194    46.713  20.695  -3.415  1.00 17.10           N
ATOM  1543  CA   CYS A 194    46.012  21.112  -2.185  1.00 19.30           C
ATOM  1544  C    CYS A 194    46.931  20.808  -0.987  1.00 19.24           C
ATOM  1545  O    CYS A 194    47.607  19.780  -0.960  1.00 17.66           O
ATOM  1546  CB   CYS A 194    44.591  20.504  -2.040  1.00 20.53           C
ATOM  1547  SG   CYS A 194    44.472  18.744  -1.756  1.00 26.44           S
ATOM  1548  N    GLU A 195    46.982  21.745  -0.041  1.00 19.18           N
```

282

| ATOM | 1549 | CA | GLU | A | 195 | 47.870 | 21.664 | 1.125 | 1.00 | 20.27 | C |
|------|------|------|-----|---|-----|--------|--------|--------|------|-------|---|
| ATOM | 1550 | C | GLU | A | 195 | 47.020 | 21.803 | 2.375 | 1.00 | 19.26 | C |
| ATOM | 1551 | O | GLU | A | 195 | 46.292 | 22.777 | 2.528 | 1.00 | 17.13 | O |
| ATOM | 1552 | CB | GLU | A | 195 | 48.934 | 22.763 | 1.124 | 1.00 | 20.94 | C |
| ATOM | 1553 | CG | GLU | A | 195 | 49.733 | 22.928 | -0.174 | 1.00 | 23.02 | C |
| ATOM | 1554 | CD | GLU | A | 195 | 51.009 | 23.732 | 0.021 | 1.00 | 24.28 | C |
| ATOM | 1555 | OE1 | GLU | A | 195 | 51.104 | 24.487 | 1.012 | 1.00 | 29.56 | O |
| ATOM | 1556 | OE2 | GLU | A | 195 | 51.927 | 23.616 | -0.822 | 1.00 | 28.30 | O |
| ATOM | 1557 | N | ALA | A | 196 | 47.100 | 20.815 | 3.257 | 1.00 | 17.74 | N |
| ATOM | 1558 | CA | ALA | A | 196 | 46.325 | 20.792 | 4.484 | 1.00 | 17.98 | C |
| ATOM | 1559 | C | ALA | A | 196 | 47.233 | 21.136 | 5.680 | 1.00 | 18.58 | C |
| ATOM | 1560 | O | ALA | A | 196 | 48.269 | 20.479 | 5.888 | 1.00 | 19.05 | O |
| ATOM | 1561 | CB | ALA | A | 196 | 45.704 | 19.413 | 4.689 | 1.00 | 19.16 | C |
| ATOM | 1562 | N | THR | A | 197 | 46.838 | 22.165 | 6.423 | 1.00 | 19.94 | N |
| ATOM | 1563 | CA | THR | A | 197 | 47.510 | 22.577 | 7.653 | 1.00 | 20.63 | C |
| ATOM | 1564 | C | THR | A | 197 | 46.649 | 22.311 | 8.884 | 1.00 | 19.95 | C |
| ATOM | 1565 | O | THR | A | 197 | 45.498 | 22.782 | 9.019 | 1.00 | 19.01 | O |
| ATOM | 1566 | CB | THR | A | 197 | 47.909 | 24.049 | 7.600 | 1.00 | 21.51 | C |
| ATOM | 1567 | OG1 | THR | A | 197 | 48.769 | 24.266 | 6.465 | 1.00 | 23.32 | O |
| ATOM | 1568 | CG2 | THR | A | 197 | 48.645 | 24.447 | 8.883 | 1.00 | 23.03 | C |
| ATOM | 1569 | N | HIS | A | 198 | 47.221 | 21.537 | 9.801 | 1.00 | 18.90 | N |
| ATOM | 1570 | CA | HIS | A | 198 | 46.517 | 21.085 | 10.993 | 1.00 | 17.27 | C |
| ATOM | 1571 | C | HIS | A | 198 | 47.503 | 21.128 | 12.180 | 1.00 | 17.62 | C |
| ATOM | 1572 | O | HIS | A | 198 | 48.700 | 21.014 | 11.991 | 1.00 | 16.88 | O |
| ATOM | 1573 | CB | HIS | A | 198 | 45.976 | 19.688 | 10.759 | 1.00 | 16.06 | C |
| ATOM | 1574 | CG | HIS | A | 198 | 44.997 | 19.215 | 11.789 | 1.00 | 13.73 | C |
| ATOM | 1575 | ND1 | HIS | A | 198 | 45.291 | 18.195 | 12.663 | 1.00 | 13.78 | N |
| ATOM | 1576 | CD2 | HIS | A | 198 | 43.733 | 19.608 | 12.085 | 1.00 | 14.88 | C |
| ATOM | 1577 | CE1 | HIS | A | 198 | 44.255 | 17.971 | 13.448 | 1.00 | 14.65 | C |
| ATOM | 1578 | NE2 | HIS | A | 198 | 43.290 | 18.816 | 13.117 | 1.00 | 16.33 | N |
| ATOM | 1579 | N | LYS | A | 199 | 46.986 | 21.272 | 13.387 | 1.00 | 19.94 | N |
| ATOM | 1580 | CA | LYS | A | 199 | 47.844 | 21.377 | 14.565 | 1.00 | 21.48 | C |
| ATOM | 1581 | C | LYS | A | 199 | 48.714 | 20.144 | 14.832 | 1.00 | 20.68 | C |
| ATOM | 1582 | O | LYS | A | 199 | 49.674 | 20.204 | 15.587 | 1.00 | 20.94 | O |
| ATOM | 1583 | CB | LYS | A | 199 | 47.001 | 21.704 | 15.790 | 1.00 | 22.81 | C |
| ATOM | 1584 | CG | LYS | A | 199 | 46.225 | 20.553 | 16.373 | 1.00 | 24.80 | C |
| ATOM | 1585 | CD | LYS | A | 199 | 45.568 | 20.956 | 17.693 | 1.00 | 24.43 | C |
| ATOM | 1586 | CE | LYS | A | 199 | 46.595 | 21.054 | 18.808 | 1.00 | 27.71 | C |
| ATOM | 1587 | NZ | LYS | A | 199 | 46.002 | 21.505 | 20.088 | 1.00 | 29.24 | N |
| ATOM | 1588 | N | THR | A | 200 | 48.386 | 19.027 | 14.202 | 1.00 | 19.28 | N |
| ATOM | 1589 | CA | THR | A | 200 | 49.160 | 17.801 | 14.325 | 1.00 | 18.62 | C |
| ATOM | 1590 | C | THR | A | 200 | 50.557 | 17.822 | 13.668 | 1.00 | 19.01 | C |
| ATOM | 1591 | O | THR | A | 200 | 51.352 | 16.919 | 13.903 | 1.00 | 17.74 | O |
| ATOM | 1592 | CB | THR | A | 200 | 48.319 | 16.595 | 13.802 | 1.00 | 18.00 | C |
| ATOM | 1593 | OG1 | THR | A | 200 | 47.748 | 16.913 | 12.526 | 1.00 | 16.77 | O |
| ATOM | 1594 | CG2 | THR | A | 200 | 47.223 | 16.264 | 14.763 | 1.00 | 16.51 | C |
| ATOM | 1595 | N | SER | A | 201 | 50.863 | 18.835 | 12.853 | 1.00 | 18.14 | N |
| ATOM | 1596 | CA | SER | A | 201 | 52.223 | 19.053 | 12.351 | 1.00 | 19.18 | C |
| ATOM | 1597 | C | SER | A | 201 | 52.407 | 20.531 | 12.025 | 1.00 | 20.23 | C |
| ATOM | 1598 | O | SER | A | 201 | 51.435 | 21.215 | 11.692 | 1.00 | 19.07 | O |
| ATOM | 1599 | CB | SER | A | 201 | 52.515 | 18.212 | 11.109 | 1.00 | 19.95 | C |
| ATOM | 1600 | OG | SER | A | 201 | 53.849 | 18.399 | 10.622 | 1.00 | 20.59 | O |
| ATOM | 1601 | N | THR | A | 202 | 53.653 | 20.997 | 12.095 | 1.00 | 21.22 | N |
| ATOM | 1602 | CA | THR | A | 202 | 53.998 | 22.346 | 11.631 | 1.00 | 23.16 | C |
| ATOM | 1603 | C | THR | A | 202 | 54.238 | 22.367 | 10.112 | 1.00 | 23.62 | C |
| ATOM | 1604 | O | THR | A | 202 | 54.401 | 23.431 | 9.538 | 1.00 | 26.44 | O |
| ATOM | 1605 | CB | THR | A | 202 | 55.249 | 22.906 | 12.352 | 1.00 | 23.37 | C |
| ATOM | 1606 | OG1 | THR | A | 202 | 56.399 | 22.186 | 11.932 | 1.00 | 25.92 | O |
| ATOM | 1607 | CG2 | THR | A | 202 | 55.126 | 22.770 | 13.860 | 1.00 | 23.94 | C |
| ATOM | 1608 | N | SER | A | 203 | 54.269 | 21.197 | 9.474 | 1.00 | 23.33 | N |
| ATOM | 1609 | CA | SER | A | 203 | 54.352 | 21.082 | 8.016 | 1.00 | 23.62 | C |
| ATOM | 1610 | C | SER | A | 203 | 52.960 | 20.761 | 7.448 | 1.00 | 23.17 | C |
| ATOM | 1611 | O | SER | A | 203 | 52.249 | 19.897 | 7.980 | 1.00 | 21.74 | O |
| ATOM | 1612 | CB | SER | A | 203 | 55.262 | 19.915 | 7.614 | 1.00 | 24.34 | C |
| ATOM | 1613 | OG | SER | A | 203 | 56.638 | 20.176 | 7.816 | 1.00 | 27.96 | O |

| ATOM | 1614 | N | PRO A 204 | 52.581 | 21.408 | 6.331 | 1.00 | 22.16 | N |
| ATOM | 1615 | CA | PRO A 204 | 51.358 | 20.945 | 5.684 | 1.00 | 21.28 | C |
| ATOM | 1616 | C | PRO A 204 | 51.514 | 19.579 | 5.008 | 1.00 | 21.51 | C |
| ATOM | 1617 | O | PRO A 204 | 52.635 | 19.176 | 4.645 | 1.00 | 22.11 | O |
| ATOM | 1618 | CB | PRO A 204 | 51.058 | 22.045 | 4.659 | 1.00 | 20.86 | C |
| ATOM | 1619 | CG | PRO A 204 | 52.342 | 22.675 | 4.383 | 1.00 | 22.01 | C |
| ATOM | 1620 | CD | PRO A 204 | 53.190 | 22.544 | 5.627 | 1.00 | 21.82 | C |
| ATOM | 1621 | N | ILE A 205 | 50.386 | 18.887 | 4.841 | 1.00 | 20.81 | N |
| ATOM | 1622 | CA | ILE A 205 | 50.293 | 17.674 | 4.042 | 1.00 | 21.59 | C |
| ATOM | 1623 | C | ILE A 205 | 49.934 | 18.096 | 2.633 | 1.00 | 21.29 | C |
| ATOM | 1624 | O | ILE A 205 | 48.942 | 18.786 | 2.443 | 1.00 | 22.19 | O |
| ATOM | 1625 | CB | ILE A 205 | 49.215 | 16.729 | 4.583 | 1.00 | 21.20 | C |
| ATOM | 1626 | CG1 | ILE A 205 | 49.584 | 16.263 | 5.991 | 1.00 | 22.89 | C |
| ATOM | 1627 | CG2 | ILE A 205 | 49.013 | 15.504 | 3.638 | 1.00 | 21.84 | C |
| ATOM | 1628 | CD1 | ILE A 205 | 48.388 | 15.780 | 6.792 | 1.00 | 23.50 | C |
| ATOM | 1629 | N | VAL A 206 | 50.758 | 17.719 | 1.669 | 1.00 | 21.36 | N |
| ATOM | 1630 | CA | VAL A 206 | 50.658 | 18.236 | 0.307 | 1.00 | 21.20 | C |
| ATOM | 1631 | C | VAL A 206 | 50.294 | 17.091 | -0.644 | 1.00 | 20.81 | C |
| ATOM | 1632 | O | VAL A 206 | 50.944 | 16.049 | -0.647 | 1.00 | 20.94 | O |
| ATOM | 1633 | CB | VAL A 206 | 51.970 | 18.961 | -0.108 | 1.00 | 21.59 | C |
| ATOM | 1634 | CG1 | VAL A 206 | 51.847 | 19.605 | -1.492 | 1.00 | 22.07 | C |
| ATOM | 1635 | CG2 | VAL A 206 | 52.325 | 20.021 | 0.927 | 1.00 | 22.61 | C |
| ATOM | 1636 | N | LYS A 207 | 49.219 | 17.279 | -1.418 | 1.00 | 19.97 | N |
| ATOM | 1637 | CA | LYS A 207 | 48.837 | 16.349 | -2.462 | 1.00 | 18.20 | C |
| ATOM | 1638 | C | LYS A 207 | 48.706 | 17.105 | -3.784 | 1.00 | 18.14 | C |
| ATOM | 1639 | O | LYS A 207 | 48.172 | 18.221 | -3.828 | 1.00 | 17.10 | O |
| ATOM | 1640 | CB | LYS A 207 | 47.530 | 15.628 | -2.096 | 1.00 | 18.48 | C |
| ATOM | 1641 | CG | LYS A 207 | 47.628 | 14.740 | -0.852 | 1.00 | 18.40 | C |
| ATOM | 1642 | CD | LYS A 207 | 48.404 | 13.478 | -1.151 | 1.00 | 20.81 | C |
| ATOM | 1643 | CE | LYS A 207 | 48.482 | 12.567 | 0.058 | 1.00 | 22.81 | C |
| ATOM | 1644 | NZ | LYS A 207 | 48.727 | 11.178 | -0.418 | 1.00 | 27.61 | N |
| ATOM | 1645 | N | SER A 208 | 49.196 | 16.486 | -4.849 | 1.00 | 18.27 | N |
| ATOM | 1646 | CA | SER A 208 | 49.282 | 17.119 | -6.150 | 1.00 | 19.52 | C |
| ATOM | 1647 | C | SER A 208 | 49.056 | 16.106 | -7.251 | 1.00 | 18.92 | C |
| ATOM | 1648 | O | SER A 208 | 49.189 | 14.897 | -7.044 | 1.00 | 17.79 | O |
| ATOM | 1649 | CB | SER A 208 | 50.660 | 17.745 | -6.369 | 1.00 | 20.59 | C |
| ATOM | 1650 | OG | SER A 208 | 50.958 | 18.658 | -5.337 | 1.00 | 26.00 | O |
| ATOM | 1651 | N | PHE A 209 | 48.680 | 16.621 | -8.418 | 1.00 | 19.18 | N |
| ATOM | 1652 | CA | PHE A 209 | 48.740 | 15.860 | -9.646 | 1.00 | 18.46 | C |
| ATOM | 1653 | C | PHE A 209 | 49.125 | 16.764 | -10.807 | 1.00 | 18.73 | C |
| ATOM | 1654 | O | PHE A 209 | 49.052 | 17.994 | -10.724 | 1.00 | 16.96 | O |
| ATOM | 1655 | CB | PHE A 209 | 47.429 | 15.138 | -9.927 | 1.00 | 18.69 | C |
| ATOM | 1656 | CG | PHE A 209 | 46.293 | 16.058 | -10.262 | 1.00 | 17.03 | C |
| ATOM | 1657 | CD1 | PHE A 209 | 46.093 | 16.496 | -11.569 | 1.00 | 16.95 | C |
| ATOM | 1658 | CD2 | PHE A 209 | 45.429 | 16.467 | -9.279 | 1.00 | 16.18 | C |
| ATOM | 1659 | CE1 | PHE A 209 | 45.060 | 17.337 | -11.890 | 1.00 | 17.59 | C |
| ATOM | 1660 | CE2 | PHE A 209 | 44.363 | 17.313 | -9.588 | 1.00 | 16.74 | C |
| ATOM | 1661 | CZ | PHE A 209 | 44.186 | 17.756 | -10.889 | 1.00 | 15.72 | C |
| ATOM | 1662 | N | ASN A 210 | 49.574 | 16.128 | -11.884 | 1.00 | 20.27 | N |
| ATOM | 1663 | CA | ASN A 210 | 49.852 | 16.825 | -13.132 | 1.00 | 20.79 | C |
| ATOM | 1664 | C | ASN A 210 | 48.754 | 16.520 | -14.124 | 1.00 | 20.84 | C |
| ATOM | 1665 | O | ASN A 210 | 48.363 | 15.368 | -14.281 | 1.00 | 20.77 | O |
| ATOM | 1666 | CB | ASN A 210 | 51.206 | 16.408 | -13.713 | 1.00 | 21.61 | C |
| ATOM | 1667 | CG | ASN A 210 | 52.343 | 16.676 | -12.772 | 1.00 | 21.98 | C |
| ATOM | 1668 | OD1 | ASN A 210 | 52.565 | 17.808 | -12.331 | 1.00 | 23.64 | O |
| ATOM | 1669 | ND2 | ASN A 210 | 53.074 | 15.621 | -12.440 | 1.00 | 24.54 | N |
| ATOM | 1670 | N | ARG A 211 | 48.252 | 17.557 | -14.782 | 1.00 | 21.17 | N |
| ATOM | 1671 | CA | ARG A 211 | 47.212 | 17.360 | -15.763 | 1.00 | 23.02 | C |
| ATOM | 1672 | C | ARG A 211 | 47.798 | 16.633 | -16.959 | 1.00 | 24.34 | C |
| ATOM | 1673 | O | ARG A 211 | 47.187 | 15.655 | -17.410 | 1.00 | 29.50 | O |
| ATOM | 1674 | CB | ARG A 211 | 46.597 | 18.703 | -16.174 | 1.00 | 22.73 | C |
| ATOM | 1675 | CG | ARG A 211 | 45.459 | 18.589 | -17.168 | 1.00 | 22.97 | C |
| ATOM | 1676 | CD | ARG A 211 | 44.989 | 19.954 | -17.615 | 1.00 | 23.19 | C |
| ATOM | 1677 | NE | ARG A 211 | 46.082 | 20.696 | -18.213 | 1.00 | 25.00 | N |
| ATOM | 1678 | CZ | ARG A 211 | 46.518 | 20.535 | -19.456 | 1.00 | 25.90 | C |

```
ATOM   1679  NH1 ARG A 211    45.946  19.671 -20.280  1.00 27.29           N
ATOM   1680  NH2 ARG A 211    47.525  21.272 -19.880  1.00 27.80           N
TER    1681      ARG A 211
ATOM   1682  N   GLU B   1     8.285  -1.327  15.102  1.00 32.14           N
ATOM   1683  CA  GLU B   1     7.103  -0.990  15.943  1.00 30.53           C
ATOM   1684  C   GLU B   1     6.363   0.209  15.349  1.00 29.06           C
ATOM   1685  O   GLU B   1     5.804   0.095  14.266  1.00 29.58           O
ATOM   1686  CB  GLU B   1     7.537  -0.702  17.369  1.00 31.24           C
ATOM   1687  N   VAL B   2     6.373   1.352  16.038  1.00 28.15           N
ATOM   1688  CA  VAL B   2     5.697   2.546  15.535  1.00 26.94           C
ATOM   1689  C   VAL B   2     6.536   3.123  14.411  1.00 25.69           C
ATOM   1690  O   VAL B   2     7.725   3.343  14.571  1.00 25.16           O
ATOM   1691  CB  VAL B   2     5.449   3.598  16.642  1.00 26.81           C
ATOM   1692  CG1 VAL B   2     4.913   4.899  16.065  1.00 27.56           C
ATOM   1693  CG2 VAL B   2     4.480   3.045  17.677  1.00 26.81           C
ATOM   1694  N   LYS B   3     5.903   3.349  13.264  1.00 26.08           N
ATOM   1695  CA  LYS B   3     6.576   3.932  12.107  1.00 25.81           C
ATOM   1696  C   LYS B   3     5.768   5.111  11.620  1.00 22.35           C
ATOM   1697  O   LYS B   3     4.578   4.992  11.399  1.00 21.61           O
ATOM   1698  CB  LYS B   3     6.691   2.903  10.994  1.00 27.02           C
ATOM   1699  CG  LYS B   3     7.626   1.745  11.333  1.00 29.35           C
ATOM   1700  CD  LYS B   3     7.553   0.646  10.278  1.00 31.02           C
ATOM   1701  CE  LYS B   3     8.331  -0.606  10.699  1.00 32.49           C
ATOM   1702  NZ  LYS B   3     8.844  -1.365   9.502  1.00 36.76           N
ATOM   1703  N   VAL B   4     6.429   6.247  11.476  1.00 21.44           N
ATOM   1704  CA  VAL B   4     5.796   7.490  11.039  1.00 19.60           C
ATOM   1705  C   VAL B   4     6.524   7.939   9.770  1.00 19.51           C
ATOM   1706  O   VAL B   4     7.748   8.110   9.776  1.00 17.58           O
ATOM   1707  CB  VAL B   4     5.926   8.557  12.146  1.00 19.96           C
ATOM   1708  CG1 VAL B   4     5.272   9.888  11.717  1.00 18.84           C
ATOM   1709  CG2 VAL B   4     5.314   8.028  13.431  1.00 19.95           C
ATOM   1710  N   GLU B   5     5.797   8.109   8.673  1.00 18.63           N
ATOM   1711  CA  GLU B   5     6.460   8.295   7.383  1.00 21.33           C
ATOM   1712  C   GLU B   5     5.794   9.392   6.561  1.00 19.65           C
ATOM   1713  O   GLU B   5     4.675   9.219   6.105  1.00 18.17           O
ATOM   1714  CB  GLU B   5     6.469   6.972   6.601  1.00 22.17           C
ATOM   1715  CG  GLU B   5     6.924   7.141   5.160  1.00 26.37           C
ATOM   1716  CD  GLU B   5     7.479   5.875   4.519  1.00 28.29           C
ATOM   1717  OE1 GLU B   5     7.252   4.752   5.045  1.00 35.99           O
ATOM   1718  OE2 GLU B   5     8.166   6.017   3.472  1.00 36.29           O
ATOM   1719  N   GLU B   6     6.500  10.510   6.382  1.00 18.69           N
ATOM   1720  CA  GLU B   6     5.953  11.672   5.708  1.00 18.37           C
ATOM   1721  C   GLU B   6     6.153  11.576   4.206  1.00 19.42           C
ATOM   1722  O   GLU B   6     7.016  10.856   3.740  1.00 19.07           O
ATOM   1723  CB  GLU B   6     6.617  12.957   6.220  1.00 17.83           C
ATOM   1724  CG  GLU B   6     6.417  13.202   7.717  1.00 18.05           C
ATOM   1725  CD  GLU B   6     7.533  12.652   8.584  1.00 17.70           C
ATOM   1726  OE1 GLU B   6     8.226  11.693   8.162  1.00 17.54           O
ATOM   1727  OE2 GLU B   6     7.750  13.177   9.690  1.00 19.72           O
ATOM   1728  N   SER B   7     5.329  12.298   3.456  1.00 20.12           N
ATOM   1729  CA  SER B   7     5.513  12.414   2.022  1.00 20.57           C
ATOM   1730  C   SER B   7     4.939  13.731   1.556  1.00 18.36           C
ATOM   1731  O   SER B   7     4.293  14.430   2.343  1.00 17.42           O
ATOM   1732  CB  SER B   7     4.869  11.234   1.292  1.00 21.79           C
ATOM   1733  OG  SER B   7     3.495  11.179   1.515  1.00 24.26           O
ATOM   1734  N   GLY B   8     5.217  14.083   0.299  1.00 19.18           N
ATOM   1735  CA  GLY B   8     4.603  15.264  -0.351  1.00 18.49           C
ATOM   1736  C   GLY B   8     5.422  16.529  -0.442  1.00 20.11           C
ATOM   1737  O   GLY B   8     4.971  17.546  -1.016  1.00 19.39           O
ATOM   1738  N   GLY B   9     6.621  16.503   0.105  1.00 19.65           N
ATOM   1739  CA  GLY B   9     7.443  17.683   0.061  1.00 20.02           C
ATOM   1740  C   GLY B   9     7.928  17.977  -1.341  1.00 20.53           C
ATOM   1741  O   GLY B   9     7.701  17.200  -2.294  1.00 21.07           O
ATOM   1742  N   GLY B  10     8.557  19.130  -1.491  1.00 19.95           N
ATOM   1743  CA  GLY B  10     9.062  19.520  -2.785  1.00 20.55           C
```

| ATOM | 1744 | C    | GLY B | 10 | 9.309  | 20.998 | -2.825 | 1.00 | 19.03 | C |
| ATOM | 1745 | O    | GLY B | 10 | 9.476  | 21.643 | -1.794 | 1.00 | 19.44 | O |
| ATOM | 1746 | N    | LEU B | 11 | 9.326  | 21.519 | -4.033 | 1.00 | 17.21 | N |
| ATOM | 1747 | CA   | LEU B | 11 | 9.514  | 22.941 | -4.258 | 1.00 | 17.66 | C |
| ATOM | 1748 | C    | LEU B | 11 | 8.181  | 23.582 | -4.636 | 1.00 | 16.18 | C |
| ATOM | 1749 | O    | LEU B | 11 | 7.360  | 23.003 | -5.359 | 1.00 | 16.22 | O |
| ATOM | 1750 | CB   | LEU B | 11 | 10.557 | 23.137 | -5.353 | 1.00 | 17.11 | C |
| ATOM | 1751 | CG   | LEU B | 11 | 10.897 | 24.563 | -5.822 | 1.00 | 17.91 | C |
| ATOM | 1752 | CD1  | LEU B | 11 | 11.816 | 25.266 | -4.890 | 1.00 | 21.59 | C |
| ATOM | 1753 | CD2  | LEU B | 11 | 11.571 | 24.469 | -7.170 | 1.00 | 21.62 | C |
| ATOM | 1754 | N    | VAL B | 12 | 7.955  | 24.795 | -4.128 | 1.00 | 16.87 | N |
| ATOM | 1755 | CA   | VAL B | 12 | 6.830  | 25.613 | -4.550 | 1.00 | 18.24 | C |
| ATOM | 1756 | C    | VAL B | 12 | 7.314  | 27.076 | -4.547 | 1.00 | 18.21 | C |
| ATOM | 1757 | O    | VAL B | 12 | 8.285  | 27.413 | -3.886 | 1.00 | 15.63 | O |
| ATOM | 1758 | CB   | VAL B | 12 | 5.572  | 25.407 | -3.630 | 1.00 | 19.99 | C |
| ATOM | 1759 | CG1  | VAL B | 12 | 5.838  | 25.843 | -2.224 | 1.00 | 19.96 | C |
| ATOM | 1760 | CG2  | VAL B | 12 | 4.369  | 26.096 | -4.200 | 1.00 | 23.39 | C |
| ATOM | 1761 | N    | GLN B | 13 | 6.644  | 27.912 | -5.337 | 1.00 | 21.07 | N |
| ATOM | 1762 | CA   | GLN B | 13 | 6.997  | 29.319 | -5.502 | 1.00 | 21.67 | C |
| ATOM | 1763 | C    | GLN B | 13 | 6.346  | 30.104 | -4.368 | 1.00 | 21.75 | C |
| ATOM | 1764 | O    | GLN B | 13 | 5.311  | 29.682 | -3.865 | 1.00 | 19.94 | O |
| ATOM | 1765 | CB   | GLN B | 13 | 6.463  | 29.853 | -6.849 | 1.00 | 23.29 | C |
| ATOM | 1766 | CG   | GLN B | 13 | 6.929  | 29.070 | -8.084 | 1.00 | 26.25 | C |
| ATOM | 1767 | CD   | GLN B | 13 | 6.224  | 27.711 | -8.285 | 1.00 | 28.84 | C |
| ATOM | 1768 | OE1  | GLN B | 13 | 5.059  | 27.513 | -7.880 | 1.00 | 30.69 | O |
| ATOM | 1769 | NE2  | GLN B | 13 | 6.946  | 26.768 | -8.899 | 1.00 | 28.92 | N |
| ATOM | 1770 | N    | PRO B | 14 | 6.941  | 31.242 | -3.963 | 1.00 | 23.10 | N |
| ATOM | 1771 | CA   | PRO B | 14 | 6.249  | 32.060 | -2.966 | 1.00 | 23.80 | C |
| ATOM | 1772 | C    | PRO B | 14 | 4.804  | 32.388 | -3.348 | 1.00 | 24.36 | C |
| ATOM | 1773 | O    | PRO B | 14 | 4.486  | 32.594 | -4.531 | 1.00 | 23.65 | O |
| ATOM | 1774 | CB   | PRO B | 14 | 7.129  | 33.301 | -2.874 | 1.00 | 24.68 | C |
| ATOM | 1775 | CG   | PRO B | 14 | 8.486  | 32.806 | -3.230 | 1.00 | 23.78 | C |
| ATOM | 1776 | CD   | PRO B | 14 | 8.232  | 31.846 | -4.335 | 1.00 | 22.92 | C |
| ATOM | 1777 | N    | GLY B | 15 | 3.916  | 32.354 | -2.354 | 1.00 | 23.80 | N |
| ATOM | 1778 | CA   | GLY B | 15 | 2.489  | 32.556 | -2.572 | 1.00 | 23.35 | C |
| ATOM | 1779 | C    | GLY B | 15 | 1.717  | 31.315 | -2.943 | 1.00 | 23.02 | C |
| ATOM | 1780 | O    | GLY B | 15 | 0.490  | 31.328 | -3.014 | 1.00 | 24.51 | O |
| ATOM | 1781 | N    | GLY B | 16 | 2.434  | 30.221 | -3.181 | 1.00 | 22.51 | N |
| ATOM | 1782 | CA   | GLY B | 16 | 1.814  | 28.995 | -3.611 | 1.00 | 22.00 | C |
| ATOM | 1783 | C    | GLY B | 16 | 1.425  | 28.103 | -2.442 | 1.00 | 21.79 | C |
| ATOM | 1784 | O    | GLY B | 16 | 1.640  | 28.442 | -1.279 | 1.00 | 21.56 | O |
| ATOM | 1785 | N    | SER B | 17 | 0.868  | 26.955 | -2.792 | 1.00 | 21.77 | N |
| ATOM | 1786 | CA   | SER B | 17 | 0.320  | 26.017 | -1.842 | 1.00 | 22.27 | C |
| ATOM | 1787 | C    | SER B | 17 | 0.989  | 24.665 | -1.982 | 1.00 | 21.75 | C |
| ATOM | 1788 | O    | SER B | 17 | 1.493  | 24.295 | -3.056 | 1.00 | 20.39 | O |
| ATOM | 1789 | CB   | SER B | 17 | -1.178 | 25.849 | -2.090 | 1.00 | 23.51 | C |
| ATOM | 1790 | OG   | SER B | 17 | -1.851 | 27.089 | -1.902 | 1.00 | 26.69 | O |
| ATOM | 1791 | N    | MET B | 18 | 0.943  | 23.904 | -0.894 | 1.00 | 20.13 | N |
| ATOM | 1792 | CA   | MET B | 18 | 1.442  | 22.543 | -0.885 | 1.00 | 21.39 | C |
| ATOM | 1793 | C    | MET B | 18 | 0.698  | 21.747 | 0.175  | 1.00 | 20.04 | C |
| ATOM | 1794 | O    | MET B | 18 | 0.374  | 22.291 | 1.236  | 1.00 | 19.11 | O |
| ATOM | 1795 | CB   | MET B | 18 | 2.918  | 22.554 | -0.554 | 1.00 | 21.36 | C |
| ATOM | 1796 | CG   | MET B | 18 | 3.584  | 21.199 | -0.574 | 1.00 | 24.58 | C |
| ATOM | 1797 | SD   | MET B | 18 | 5.361  | 21.395 | -0.430 | 1.00 | 28.45 | S |
| ATOM | 1798 | CE   | MET B | 18 | 5.785  | 21.840 | -2.096 | 1.00 | 29.48 | C |
| ATOM | 1799 | N    | LYS B | 19 | 0.477  | 20.465 | -0.089 | 1.00 | 20.27 | N |
| ATOM | 1800 | CA   | LYS B | 19 | -0.158 | 19.583 | 0.894  | 1.00 | 21.21 | C |
| ATOM | 1801 | C    | LYS B | 19 | 0.774  | 18.414 | 1.161  | 1.00 | 20.39 | C |
| ATOM | 1802 | O    | LYS B | 19 | 1.172  | 17.699 | 0.236  | 1.00 | 20.39 | O |
| ATOM | 1803 | CB   | LYS B | 19 | -1.521 | 19.059 | 0.421  | 1.00 | 22.42 | C |
| ATOM | 1804 | CG   | LYS B | 19 | -2.347 | 18.456 | 1.556  | 1.00 | 24.32 | C |
| ATOM | 1805 | CD   | LYS B | 19 | -3.665 | 17.863 | 1.096  | 1.00 | 24.13 | C |
| ATOM | 1806 | CE   | LYS B | 19 | -4.649 | 17.696 | 2.257  | 1.00 | 26.87 | C |
| ATOM | 1807 | NZ   | LYS B | 19 | -5.990 | 17.070 | 1.817  | 1.00 | 27.33 | N |
| ATOM | 1808 | N    | ILE B | 20 | 1.142  | 18.247 | 2.427  | 1.00 | 18.04 | N |

```
ATOM   1809  CA   ILE B  20      1.999  17.161   2.841  1.00 16.23           C
ATOM   1810  C    ILE B  20      1.195  16.205   3.739  1.00 16.41           C
ATOM   1811  O    ILE B  20      0.107  16.544   4.244  1.00 13.63           O
ATOM   1812  CB   ILE B  20      3.323  17.666   3.507  1.00 16.00           C
ATOM   1813  CG1  ILE B  20      3.047  18.510   4.754  1.00 15.65           C
ATOM   1814  CG2  ILE B  20      4.177  18.461   2.513  1.00 16.63           C
ATOM   1815  CD1  ILE B  20      4.328  18.854   5.543  1.00 14.42           C
ATOM   1816  N    SER B  21      1.711  14.990   3.907  1.00 16.59           N
ATOM   1817  CA   SER B  21      1.013  14.016   4.688  1.00 16.18           C
ATOM   1818  C    SER B  21      1.965  13.040   5.363  1.00 16.72           C
ATOM   1819  O    SER B  21      3.165  13.030   5.114  1.00 16.89           O
ATOM   1820  CB   SER B  21     -0.009  13.246   3.830  1.00 17.57           C
ATOM   1821  OG   SER B  21      0.636  12.422   2.893  1.00 20.49           O
ATOM   1822  N    CYS B  22      1.411  12.237   6.243  1.00 18.27           N
ATOM   1823  CA   CYS B  22      2.160  11.126   6.820  1.00 20.41           C
ATOM   1824  C    CYS B  22      1.234  10.044   7.285  1.00 19.59           C
ATOM   1825  O    CYS B  22      0.107  10.305   7.704  1.00 19.26           O
ATOM   1826  CB   CYS B  22      3.083  11.585   7.950  1.00 22.41           C
ATOM   1827  SG   CYS B  22      2.314  12.056   9.468  1.00 28.62           S
ATOM   1828  N    VAL B  23      1.716   8.809   7.183  1.00 19.77           N
ATOM   1829  CA   VAL B  23      0.945   7.656   7.597  1.00 21.12           C
ATOM   1830  C    VAL B  23      1.702   6.979   8.723  1.00 19.95           C
ATOM   1831  O    VAL B  23      2.937   6.920   8.699  1.00 19.97           O
ATOM   1832  CB   VAL B  23      0.675   6.708   6.404  1.00 22.02           C
ATOM   1833  CG1  VAL B  23      1.965   6.390   5.685  1.00 23.04           C
ATOM   1834  CG2  VAL B  23     -0.037   5.443   6.852  1.00 23.28           C
ATOM   1835  N    VAL B  24      0.954   6.519   9.724  1.00 19.50           N
ATOM   1836  CA   VAL B  24      1.512   5.964  10.941  1.00 20.31           C
ATOM   1837  C    VAL B  24      1.108   4.495  11.005  1.00 21.49           C
ATOM   1838  O    VAL B  24     -0.031   4.167  10.730  1.00 22.95           O
ATOM   1839  CB   VAL B  24      0.988   6.691  12.210  1.00 19.58           C
ATOM   1840  CG1  VAL B  24      1.643   6.136  13.469  1.00 20.30           C
ATOM   1841  CG2  VAL B  24      1.240   8.201  12.111  1.00 18.52           C
ATOM   1842  N    SER B  25      2.056   3.621  11.317  1.00 22.92           N
ATOM   1843  CA   SER B  25      1.719   2.225  11.593  1.00 23.70           C
ATOM   1844  C    SER B  25      2.259   1.822  12.968  1.00 23.11           C
ATOM   1845  O    SER B  25      3.050   2.540  13.589  1.00 22.17           O
ATOM   1846  CB   SER B  25      2.253   1.309  10.488  1.00 23.96           C
ATOM   1847  OG   SER B  25      3.663   1.384  10.369  1.00 26.81           O
ATOM   1848  N    GLY B  26      1.822   0.664  13.450  1.00 23.50           N
ATOM   1849  CA   GLY B  26      2.278   0.160  14.738  1.00 24.03           C
ATOM   1850  C    GLY B  26      1.560   0.749  15.949  1.00 23.62           C
ATOM   1851  O    GLY B  26      1.922   0.461  17.081  1.00 24.64           O
ATOM   1852  N    LEU B  27      0.551   1.572  15.714  1.00 23.20           N
ATOM   1853  CA   LEU B  27     -0.367   1.984  16.767  1.00 22.59           C
ATOM   1854  C    LEU B  27     -1.737   2.265  16.166  1.00 22.46           C
ATOM   1855  O    LEU B  27     -1.909   2.240  14.950  1.00 23.80           O
ATOM   1856  CB   LEU B  27      0.200   3.186  17.542  1.00 22.82           C
ATOM   1857  CG   LEU B  27      0.621   4.430  16.753  1.00 22.15           C
ATOM   1858  CD1  LEU B  27     -0.559   5.115  16.087  1.00 22.60           C
ATOM   1859  CD2  LEU B  27      1.332   5.421  17.663  1.00 23.07           C
ATOM   1860  N    THR B  28     -2.721   2.494  17.020  1.00 21.72           N
ATOM   1861  CA   THR B  28     -4.055   2.840  16.588  1.00 21.94           C
ATOM   1862  C    THR B  28     -4.118   4.332  16.399  1.00 21.30           C
ATOM   1863  O    THR B  28     -4.287   5.075  17.358  1.00 20.80           O
ATOM   1864  CB   THR B  28     -5.087   2.411  17.609  1.00 23.02           C
ATOM   1865  OG1  THR B  28     -4.927   0.997  17.844  1.00 25.73           O
ATOM   1866  CG2  THR B  28     -6.499   2.727  17.117  1.00 24.34           C
ATOM   1867  N    PHE B  29     -3.977   4.746  15.148  1.00 21.55           N
ATOM   1868  CA   PHE B  29     -3.830   6.165  14.783  1.00 20.18           C
ATOM   1869  C    PHE B  29     -4.889   7.060  15.424  1.00 19.57           C
ATOM   1870  O    PHE B  29     -4.572   8.119  15.957  1.00 19.74           O
ATOM   1871  CB   PHE B  29     -3.835   6.269  13.250  1.00 19.27           C
ATOM   1872  CG   PHE B  29     -3.757   7.668  12.710  1.00 18.87           C
ATOM   1873  CD1  PHE B  29     -2.536   8.230  12.355  1.00 19.89           C
```

| | | | | | | | | | | | |
|---|---|---|---|---|---|---|---|---|---|---|---|
| ATOM | 1874 | CD2 | PHE | B | 29 | -4.910 | 8.396 | 12.477 | 1.00 | 17.98 | C |
| ATOM | 1875 | CE1 | PHE | B | 29 | -2.463 | 9.505 | 11.824 | 1.00 | 18.43 | C |
| ATOM | 1876 | CE2 | PHE | B | 29 | -4.847 | 9.654 | 11.926 | 1.00 | 18.56 | C |
| ATOM | 1877 | CZ | PHE | B | 29 | -3.616 | 10.223 | 11.604 | 1.00 | 18.39 | C |
| ATOM | 1878 | N | SER | B | 30 | -6.148 | 6.634 | 15.373 | 1.00 | 19.26 | N |
| ATOM | 1879 | CA | SER | B | 30 | -7.276 | 7.443 | 15.850 | 1.00 | 20.01 | C |
| ATOM | 1880 | C | SER | B | 30 | -7.263 | 7.769 | 17.345 | 1.00 | 18.72 | C |
| ATOM | 1881 | O | SER | B | 30 | -8.030 | 8.614 | 17.790 | 1.00 | 17.44 | O |
| ATOM | 1882 | CB | SER | B | 30 | -8.598 | 6.748 | 15.496 | 1.00 | 20.48 | C |
| ATOM | 1883 | OG | SER | B | 30 | -8.619 | 5.407 | 15.995 | 1.00 | 23.06 | O |
| ATOM | 1884 | N | ASN | B | 31 | -6.418 | 7.097 | 18.119 | 1.00 | 18.67 | N |
| ATOM | 1885 | CA | ASN | B | 31 | -6.357 | 7.336 | 19.560 | 1.00 | 19.56 | C |
| ATOM | 1886 | C | ASN | B | 31 | -5.261 | 8.338 | 19.939 | 1.00 | 18.63 | C |
| ATOM | 1887 | O | ASN | B | 31 | -5.095 | 8.619 | 21.114 | 1.00 | 18.53 | O |
| ATOM | 1888 | CB | ASN | B | 31 | -6.090 | 6.027 | 20.339 | 1.00 | 20.83 | C |
| ATOM | 1889 | CG | ASN | B | 31 | -7.255 | 5.032 | 20.283 | 1.00 | 23.08 | C |
| ATOM | 1890 | OD1 | ASN | B | 31 | -7.079 | 3.841 | 20.573 | 1.00 | 27.20 | O |
| ATOM | 1891 | ND2 | ASN | B | 31 | -8.426 | 5.511 | 19.963 | 1.00 | 24.21 | N |
| ATOM | 1892 | N | TYR | B | 32 | -4.524 | 8.871 | 18.955 | 1.00 | 17.70 | N |
| ATOM | 1893 | CA | TYR | B | 32 | -3.384 | 9.734 | 19.231 | 1.00 | 17.81 | C |
| ATOM | 1894 | C | TYR | B | 32 | -3.572 | 11.132 | 18.686 | 1.00 | 15.81 | C |
| ATOM | 1895 | O | TYR | B | 32 | -4.050 | 11.318 | 17.584 | 1.00 | 15.65 | O |
| ATOM | 1896 | CB | TYR | B | 32 | -2.121 | 9.140 | 18.621 | 1.00 | 19.70 | C |
| ATOM | 1897 | CG | TYR | B | 32 | -1.608 | 7.967 | 19.396 | 1.00 | 21.57 | C |
| ATOM | 1898 | CD1 | TYR | B | 32 | -0.604 | 8.128 | 20.332 | 1.00 | 21.50 | C |
| ATOM | 1899 | CD2 | TYR | B | 32 | -2.158 | 6.700 | 19.218 | 1.00 | 20.61 | C |
| ATOM | 1900 | CE1 | TYR | B | 32 | -0.136 | 7.071 | 21.051 | 1.00 | 23.95 | C |
| ATOM | 1901 | CE2 | TYR | B | 32 | -1.702 | 5.627 | 19.943 | 1.00 | 22.68 | C |
| ATOM | 1902 | CZ | TYR | B | 32 | -0.684 | 5.817 | 20.860 | 1.00 | 23.42 | C |
| ATOM | 1903 | OH | TYR | B | 32 | -0.183 | 4.776 | 21.608 | 1.00 | 24.22 | O |
| ATOM | 1904 | N | TRP | B | 33 | -3.203 | 12.116 | 19.498 | 1.00 | 14.62 | N |
| ATOM | 1905 | CA | TRP | B | 33 | -3.004 | 13.476 | 19.029 | 1.00 | 14.60 | C |
| ATOM | 1906 | C | TRP | B | 33 | -1.884 | 13.520 | 17.997 | 1.00 | 13.91 | C |
| ATOM | 1907 | O | TRP | B | 33 | -0.891 | 12.790 | 18.100 | 1.00 | 14.38 | O |
| ATOM | 1908 | CB | TRP | B | 33 | -2.644 | 14.384 | 20.200 | 1.00 | 13.86 | C |
| ATOM | 1909 | CG | TRP | B | 33 | -3.640 | 14.436 | 21.309 | 1.00 | 14.62 | C |
| ATOM | 1910 | CD1 | TRP | B | 33 | -4.919 | 13.929 | 21.321 | 1.00 | 14.17 | C |
| ATOM | 1911 | CD2 | TRP | B | 33 | -3.464 | 15.094 | 22.566 | 1.00 | 13.85 | C |
| ATOM | 1912 | NE1 | TRP | B | 33 | -5.521 | 14.219 | 22.506 | 1.00 | 14.99 | N |
| ATOM | 1913 | CE2 | TRP | B | 33 | -4.659 | 14.939 | 23.290 | 1.00 | 13.93 | C |
| ATOM | 1914 | CE3 | TRP | B | 33 | -2.418 | 15.810 | 23.141 | 1.00 | 15.09 | C |
| ATOM | 1915 | CZ2 | TRP | B | 33 | -4.833 | 15.460 | 24.549 | 1.00 | 14.03 | C |
| ATOM | 1916 | CZ3 | TRP | B | 33 | -2.596 | 16.340 | 24.401 | 1.00 | 14.13 | C |
| ATOM | 1917 | CH2 | TRP | B | 33 | -3.786 | 16.151 | 25.104 | 1.00 | 14.63 | C |
| ATOM | 1918 | N | MET | B | 34 | -2.040 | 14.396 | 17.007 | 1.00 | 13.94 | N |
| ATOM | 1919 | CA | MET | B | 34 | -1.090 | 14.534 | 15.923 | 1.00 | 14.70 | C |
| ATOM | 1920 | C | MET | B | 34 | -0.686 | 16.004 | 15.751 | 1.00 | 13.62 | C |
| ATOM | 1921 | O | MET | B | 34 | -1.520 | 16.895 | 15.847 | 1.00 | 13.78 | O |
| ATOM | 1922 | CB | MET | B | 34 | -1.750 | 14.103 | 14.636 | 1.00 | 16.39 | C |
| ATOM | 1923 | CG | MET | B | 34 | -2.204 | 12.676 | 14.618 | 1.00 | 16.57 | C |
| ATOM | 1924 | SD | MET | B | 34 | -0.825 | 11.626 | 14.225 | 1.00 | 20.44 | S |
| ATOM | 1925 | CE | MET | B | 34 | -1.440 | 10.135 | 15.042 | 1.00 | 19.75 | C |
| ATOM | 1926 | N | SER | B | 35 | 0.583 | 16.241 | 15.456 | 1.00 | 14.44 | N |
| ATOM | 1927 | CA | SER | B | 35 | 1.058 | 17.590 | 15.181 | 1.00 | 13.12 | C |
| ATOM | 1928 | C | SER | B | 35 | 2.116 | 17.613 | 14.083 | 1.00 | 13.83 | C |
| ATOM | 1929 | O | SER | B | 35 | 2.652 | 16.590 | 13.699 | 1.00 | 12.66 | O |
| ATOM | 1930 | CB | SER | B | 35 | 1.609 | 18.231 | 16.458 | 1.00 | 13.25 | C |
| ATOM | 1931 | OG | SER | B | 35 | 2.868 | 17.664 | 16.818 | 1.00 | 12.61 | O |
| ATOM | 1932 | N | TRP | B | 36 | 2.411 | 18.824 | 13.604 | 1.00 | 12.83 | N |
| ATOM | 1933 | CA | TRP | B | 36 | 3.505 | 19.070 | 12.674 | 1.00 | 13.33 | C |
| ATOM | 1934 | C | TRP | B | 36 | 4.480 | 20.011 | 13.344 | 1.00 | 13.00 | C |
| ATOM | 1935 | O | TRP | B | 36 | 4.080 | 21.029 | 13.921 | 1.00 | 13.06 | O |
| ATOM | 1936 | CB | TRP | B | 36 | 2.991 | 19.688 | 11.379 | 1.00 | 12.70 | C |
| ATOM | 1937 | CG | TRP | B | 36 | 2.191 | 18.691 | 10.526 | 1.00 | 12.21 | C |
| ATOM | 1938 | CD1 | TRP | B | 36 | 0.855 | 18.554 | 10.467 | 1.00 | 13.79 | C |

| ATOM | 1939 | CD2 | TRP | B | 36 | 2.731 | 17.772 | 9.573 | 1.00 | 12.63 | C |
|------|------|-----|-----|---|----|-------|--------|-------|------|-------|---|
| ATOM | 1940 | NE1 | TRP | B | 36 | 0.511 | 17.557 | 9.563 | 1.00 | 13.87 | N |
| ATOM | 1941 | CE2 | TRP | B | 36 | 1.650 | 17.075 | 8.994 | 1.00 | 12.87 | C |
| ATOM | 1942 | CE3 | TRP | B | 36 | 4.028 | 17.472 | 9.150 | 1.00 | 13.79 | C |
| ATOM | 1943 | CZ2 | TRP | B | 36 | 1.832 | 16.104 | 8.009 | 1.00 | 14.85 | C |
| ATOM | 1944 | CZ3 | TRP | B | 36 | 4.210 | 16.498 | 8.173 | 1.00 | 14.19 | C |
| ATOM | 1945 | CH2 | TRP | B | 36 | 3.120 | 15.835 | 7.614 | 1.00 | 14.06 | C |
| ATOM | 1946 | N | VAL | B | 37 | 5.752 | 19.637 | 13.270 | 1.00 | 13.60 | N |
| ATOM | 1947 | CA | VAL | B | 37 | 6.867 | 20.453 | 13.780 | 1.00 | 13.23 | C |
| ATOM | 1948 | C | VAL | B | 37 | 7.854 | 20.598 | 12.638 | 1.00 | 13.12 | C |
| ATOM | 1949 | O | VAL | B | 37 | 8.289 | 19.594 | 12.064 | 1.00 | 12.98 | O |
| ATOM | 1950 | CB | VAL | B | 37 | 7.592 | 19.754 | 14.989 | 1.00 | 13.74. | C |
| ATOM | 1951 | CG1 | VAL | B | 37 | 8.811 | 20.581 | 15.450 | 1.00 | 13.37 | C |
| ATOM | 1952 | CG2 | VAL | B | 37 | 6.630 | 19.548 | 16.151 | 1.00 | 14.34 | C |
| ATOM | 1953 | N | ARG | B | 38 | 8.252 | 21.829 | 12.344 | 1.00 | 12.62 | N |
| ATOM | 1954 | CA | ARG | B | 38 | 9.259 | 22.070 | 11.306 | 1.00 | 13.12 | C |
| ATOM | 1955 | C | ARG | B | 38 | 10.582 | 22.497 | 11.885 | 1.00 | 14.07 | C |
| ATOM | 1956 | O | ARG | B | 38 | 10.649 | 22.998 | 13.000 | 1.00 | 13.58 | O |
| ATOM | 1957 | CB | ARG | B | 38 | 8.759 | 23.101 | 10.301 | 1.00 | 13.53 | C |
| ATOM | 1958 | CG | ARG | B | 38 | 8.529 | 24.470 | 10.896 | 1.00 | 14.57 | C |
| ATOM | 1959 | CD | ARG | B | 38 | 7.776 | 25.330 | 9.917 | 1.00 | 15.32 | C |
| ATOM | 1960 | NE | ARG | B | 38 | 7.725 | 26.712 | 10.400 | 1.00 | 17.53 | N |
| ATOM | 1961 | CZ | ARG | B | 38 | 7.160 | 27.706 | 9.738 | 1.00 | 18.15 | C |
| ATOM | 1962 | NH1 | ARG | B | 38 | 6.516 | 27.479 | 8.595 | 1.00 | 17.67 | N |
| ATOM | 1963 | NH2 | ARG | B | 38 | 7.176 | 28.933 | 10.263 | 1.00 | 19.65 | N |
| ATOM | 1964 | N | GLN | B | 39 | 11.636 | 22.271 | 11.116 | 1.00 | 14.72 | N |
| ATOM | 1965 | CA | GLN | B | 39 | 12.988 | 22.573 | 11.537 | 1.00 | 15.88 | C |
| ATOM | 1966 | C | GLN | B | 39 | 13.719 | 23.321 | 10.433 | 1.00 | 17.02 | C |
| ATOM | 1967 | O | GLN | B | 39 | 13.695 | 22.909 | 9.276 | 1.00 | 14.64 | O |
| ATOM | 1968 | CB | GLN | B | 39 | 13.730 | 21.293 | 11.851 | 1.00 | 15.76 | C |
| ATOM | 1969 | CG | GLN | B | 39 | 15.033 | 21.530 | 12.534 | 1.00 | 17.67 | C |
| ATOM | 1970 | CD | GLN | B | 39 | 15.626 | 20.283 | 13.086 | 1.00 | 18.91 | C |
| ATOM | 1971 | OE1 | GLN | B | 39 | 15.539 | 19.216 | 12.466 | 1.00 | 21.57 | O |
| ATOM | 1972 | NE2 | GLN | B | 39 | 16.266 | 20.398 | 14.256 | 1.00 | 21.49 | N |
| ATOM | 1973 | N | SER | B | 40 | 14.353 | 24.420 | 10.801 | 1.00 | 20.98 | N |
| ATOM | 1974 | CA | SER | B | 40 | 15.255 | 25.130 | 9.898 | 1.00 | 24.76 | C |
| ATOM | 1975 | C | SER | B | 40 | 16.476 | 25.561 | 10.708 | 1.00 | 27.59 | C |
| ATOM | 1976 | O | SER | B | 40 | 16.416 | 25.611 | 11.945 | 1.00 | 27.96 | O |
| ATOM | 1977 | CB | SER | B | 40 | 14.559 | 26.329 | 9.259 | 1.00 | 25.63 | C |
| ATOM | 1978 | OG | SER | B | 40 | 14.107 | 27.247 | 10.246 | 1.00 | 27.26 | O |
| ATOM | 1979 | N | PRO | B | 41 | 17.609 | 25.814 | 10.030 | 1.00 | 31.45 | N |
| ATOM | 1980 | CA | PRO | B | 41 | 18.809 | 26.285 | 10.738 | 1.00 | 32.42 | C |
| ATOM | 1981 | C | PRO | B | 41 | 18.624 | 27.613 | 11.463 | 1.00 | 33.39 | C |
| ATOM | 1982 | O | PRO | B | 41 | 19.134 | 27.787 | 12.569 | 1.00 | 34.93 | O |
| ATOM | 1983 | CB | PRO | B | 41 | 19.854 | 26.413 | 9.623 | 1.00 | 32.80 | C |
| ATOM | 1984 | CG | PRO | B | 41 | 19.348 | 25.546 | 8.520 | 1.00 | 33.51 | C |
| ATOM | 1985 | CD | PRO | B | 41 | 17.863 | 25.605 | 8.593 | 1.00 | 31.92 | C |
| ATOM | 1986 | N | GLU | B | 42 | 17.883 | 28.535 | 10.872 | 1.00 | 34.65 | N |
| ATOM | 1987 | CA | GLU | B | 42 | 17.788 | 29.877 | 11.446 | 1.00 | 35.64 | C |
| ATOM | 1988 | C | GLU | B | 42 | 16.769 | 29.973 | 12.584 | 1.00 | 34.69 | C |
| ATOM | 1989 | O | GLU | B | 42 | 16.869 | 30.869 | 13.435 | 1.00 | 34.55 | O |
| ATOM | 1990 | CB | GLU | B | 42 | 17.518 | 30.956 | 10.373 | 1.00 | 37.46 | C |
| ATOM | 1991 | CG | GLU | B | 42 | 16.471 | 30.617 | 9.312 | 1.00 | 38.91 | C |
| ATOM | 1992 | CD | GLU | B | 42 | 17.075 | 29.897 | 8.118 | 1.00 | 41.16 | C |
| ATOM | 1993 | OE1 | GLU | B | 42 | 16.915 | 28.661 | 8.038 | 1.00 | 40.82 | O |
| ATOM | 1994 | OE2 | GLU | B | 42 | 17.720 | 30.555 | 7.263 | 1.00 | 42.26 | O |
| ATOM | 1995 | N | LYS | B | 43 | 15.819 | 29.040 | 12.630 | 1.00 | 32.58 | N |
| ATOM | 1996 | CA | LYS | B | 43 | 14.759 | 29.083 | 13.632 | 1.00 | 29.65 | C |
| ATOM | 1997 | C | LYS | B | 43 | 14.656 | 27.877 | 14.577 | 1.00 | 27.93 | C |
| ATOM | 1998 | O | LYS | B | 43 | 13.991 | 27.970 | 15.592 | 1.00 | 28.72 | O |
| ATOM | 1999 | CB | LYS | B | 43 | 13.424 | 29.342 | 12.933 | 1.00 | 31.94 | C |
| ATOM | 2000 | CG | LYS | B | 43 | 13.146 | 30.837 | 12.683 | 1.00 | 34.46 | C |
| ATOM | 2001 | CD | LYS | B | 43 | 12.570 | 31.474 | 13.952 | 1.00 | 35.08 | C |
| ATOM | 2002 | CE | LYS | B | 43 | 12.530 | 32.996 | 13.916 | 1.00 | 35.89 | C |
| ATOM | 2003 | NZ | LYS | B | 43 | 11.624 | 33.527 | 15.001 | 1.00 | 36.43 | N |

| ATOM | 2004 | N | GLY | B | 44 | 15.310 | 26.766 | 14.262 | 1.00 | 23.94 | N |
|------|------|------|-----|---|----|--------|--------|--------|------|-------|---|
| ATOM | 2005 | CA | GLY | B | 44 | 15.264 | 25.567 | 15.100 | 1.00 | 23.65 | C |
| ATOM | 2006 | C | GLY | B | 44 | 13.976 | 24.771 | 14.929 | 1.00 | 20.26 | C |
| ATOM | 2007 | O | GLY | B | 44 | 13.371 | 24.802 | 13.865 | 1.00 | 17.85 | O |
| ATOM | 2008 | N | LEU | B | 45 | 13.575 | 24.061 | 15.989 | 1.00 | 18.34 | N |
| ATOM | 2009 | CA | LEU | B | 45 | 12.361 | 23.260 | 15.987 | 1.00 | 17.08 | C |
| ATOM | 2010 | C | LEU | B | 45 | 11.204 | 24.160 | 16.311 | 1.00 | 17.12 | C |
| ATOM | 2011 | O | LEU | B | 45 | 11.244 | 24.886 | 17.329 | 1.00 | 17.27 | O |
| ATOM | 2012 | CB | LEU | B | 45 | 12.432 | 22.134 | 17.015 | 1.00 | 16.57 | C |
| ATOM | 2013 | CG | LEU | B | 45 | 13.334 | 20.937 | 16.728 | 1.00 | 17.63 | C |
| ATOM | 2014 | CD1 | LEU | B | 45 | 13.445 | 20.055 | 17.947 | 1.00 | 19.15 | C |
| ATOM | 2015 | CD2 | LEU | B | 45 | 12.839 | 20.148 | 15.524 | 1.00 | 17.40 | C |
| ATOM | 2016 | N | GLU | B | 46 | 10.172 | 24.114 | 15.465 | 1.00 | 14.81 | N |
| ATOM | 2017 | CA | GLU | B | 46 | 9.001 | 24.978 | 15.606 | 1.00 | 17.22 | C |
| ATOM | 2018 | C | GLU | B | 46 | 7.710 | 24.202 | 15.365 | 1.00 | 15.80 | C |
| ATOM | 2019 | O | GLU | B | 46 | 7.420 | 23.776 | 14.245 | 1.00 | 14.04 | O |
| ATOM | 2020 | CB | GLU | B | 46 | 9.128 | 26.122 | 14.612 | 1.00 | 18.26 | C |
| ATOM | 2021 | CG | GLU | B | 46 | 7.998 | 27.064 | 14.572 | 1.00 | 21.96 | C |
| ATOM | 2022 | CD | GLU | B | 46 | 8.234 | 28.207 | 13.571 | 1.00 | 24.44 | C |
| ATOM | 2023 | OE1 | GLU | B | 46 | 9.216 | 28.122 | 12.747 | 1.00 | 30.05 | O |
| ATOM | 2024 | OE2 | GLU | B | 46 | 7.434 | 29.176 | 13.636 | 1.00 | 31.06 | O |
| ATOM | 2025 | N | TRP | B | 47 | 6.944 | 24.013 | 16.437 | 1.00 | 15.02 | N |
| ATOM | 2026 | CA | TRP | B | 47 | 5.611 | 23.409 | 16.360 | 1.00 | 13.65 | C |
| ATOM | 2027 | C | TRP | B | 47 | 4.717 | 24.379 | 15.580 | 1.00 | 13.47 | C |
| ATOM | 2028 | O | TRP | B | 47 | 4.740 | 25.588 | 15.845 | 1.00 | 13.70 | O |
| ATOM | 2029 | CB | TRP | B | 47 | 5.096 | 23.204 | 17.791 | 1.00 | 13.69 | C |
| ATOM | 2030 | CG | TRP | B | 47 | 3.655 | 22.761 | 17.963 | 1.00 | 13.22 | C |
| ATOM | 2031 | CD1 | TRP | B | 47 | 3.213 | 21.486 | 18.082 | 1.00 | 13.20 | C |
| ATOM | 2032 | CD2 | TRP | B | 47 | 2.509 | 23.605 | 18.158 | 1.00 | 13.75 | C |
| ATOM | 2033 | NE1 | TRP | B | 47 | 1.861 | 21.465 | 18.266 | 1.00 | 14.31 | N |
| ATOM | 2034 | CE2 | TRP | B | 47 | 1.399 | 22.760 | 18.341 | 1.00 | 13.39 | C |
| ATOM | 2035 | CE3 | TRP | B | 47 | 2.317 | 24.995 | 18.198 | 1.00 | 14.01 | C |
| ATOM | 2036 | CZ2 | TRP | B | 47 | 0.103 | 23.255 | 18.544 | 1.00 | 14.79 | C |
| ATOM | 2037 | CZ3 | TRP | B | 47 | 1.025 | 25.487 | 18.374 | 1.00 | 14.66 | C |
| ATOM | 2038 | CH2 | TRP | B | 47 | -0.063 | 24.622 | 18.557 | 1.00 | 14.63 | C |
| ATOM | 2039 | N | VAL | B | 48 | 3.954 | 23.880 | 14.614 | 1.00 | 13.85 | N |
| ATOM | 2040 | CA | VAL | B | 48 | 3.086 | 24.766 | 13.805 | 1.00 | 15.63 | C |
| ATOM | 2041 | C | VAL | B | 48 | 1.591 | 24.449 | 13.847 | 1.00 | 15.25 | C |
| ATOM | 2042 | O | VAL | B | 48 | 0.770 | 25.340 | 13.670 | 1.00 | 15.09 | O |
| ATOM | 2043 | CB | VAL | B | 48 | 3.556 | 24.855 | 12.343 | 1.00 | 16.65 | C |
| ATOM | 2044 | CG1 | VAL | B | 48 | 4.898 | 25.579 | 12.304 | 1.00 | 17.32 | C |
| ATOM | 2045 | CG2 | VAL | B | 48 | 3.639 | 23.461 | 11.692 | 1.00 | 17.71 | C |
| ATOM | 2046 | N | ALA | B | 49 | 1.232 | 23.184 | 14.046 | 1.00 | 13.37 | N |
| ATOM | 2047 | CA | ALA | B | 49 | -0.186 | 22.791 | 14.004 | 1.00 | 13.78 | C |
| ATOM | 2048 | C | ALA | B | 49 | -0.384 | 21.494 | 14.737 | 1.00 | 12.60 | C |
| ATOM | 2049 | O | ALA | B | 49 | 0.512 | 20.657 | 14.760 | 1.00 | 12.40 | O |
| ATOM | 2050 | CB | ALA | B | 49 | -0.664 | 22.628 | 12.589 | 1.00 | 13.19 | C |
| ATOM | 2051 | N | GLU | B | 50 | -1.559 | 21.348 | 15.333 | 1.00 | 13.37 | N |
| ATOM | 2052 | CA | GLU | B | 50 | -1.933 | 20.131 | 16.072 | 1.00 | 12.84 | C |
| ATOM | 2053 | C | GLU | B | 50 | -3.424 | 19.826 | 15.922 | 1.00 | 13.08 | C |
| ATOM | 2054 | O | GLU | B | 50 | -4.246 | 20.737 | 15.829 | 1.00 | 12.78 | O |
| ATOM | 2055 | CB | GLU | B | 50 | -1.595 | 20.310 | 17.556 | 1.00 | 13.18 | C |
| ATOM | 2056 | CG | GLU | B | 50 | -1.857 | 19.090 | 18.418 | 1.00 | 13.67 | C |
| ATOM | 2057 | CD | GLU | B | 50 | -0.999 | 19.027 | 19.655 | 1.00 | 15.51 | C |
| ATOM | 2058 | OE1 | GLU | B | 50 | 0.185 | 19.415 | 19.589 | 1.00 | 14.10 | O |
| ATOM | 2059 | OE2 | GLU | B | 50 | -1.491 | 18.527 | 20.693 | 1.00 | 17.02 | O |
| ATOM | 2060 | N | ILE | B | 51 | -3.748 | 18.536 | 15.887 | 1.00 | 12.44 | N |
| ATOM | 2061 | CA | ILE | B | 51 | -5.122 | 18.066 | 15.801 | 1.00 | 13.38 | C |
| ATOM | 2062 | C | ILE | B | 51 | -5.403 | 16.992 | 16.845 | 1.00 | 14.03 | C |
| ATOM | 2063 | O | ILE | B | 51 | -4.628 | 16.028 | 17.030 | 1.00 | 14.76 | O |
| ATOM | 2064 | CB | ILE | B | 51 | -5.494 | 17.564 | 14.358 | 1.00 | 11.90 | C |
| ATOM | 2065 | CG1 | ILE | B | 51 | -6.997 | 17.302 | 14.245 | 1.00 | 14.07 | C |
| ATOM | 2066 | CG2 | ILE | B | 51 | -4.639 | 16.375 | 13.936 | 1.00 | 11.48 | C |
| ATOM | 2067 | CD1 | ILE | B | 51 | -7.464 | 17.260 | 12.831 | 1.00 | 14.06 | C |
| ATOM | 2068 | N | ARG | B | 52 | -6.544 | 17.156 | 17.506 | 1.00 | 13.50 | N |

```
ATOM   2069  CA   ARG B   52     -7.006  16.203  18.509  1.00  15.89           C
ATOM   2070  C    ARG B   52     -7.908  15.106  17.908  1.00  16.34           C
ATOM   2071  O    ARG B   52     -7.822  14.824  16.713  1.00  17.36           O
ATOM   2072  CB   ARG B   52     -7.653  16.960  19.664  1.00  15.58           C
ATOM   2073  CG   ARG B   52     -6.718  18.033  20.276  1.00  16.03           C
ATOM   2074  CD   ARG B   52     -5.540  17.379  20.918  1.00  16.11           C
ATOM   2075  NE   ARG B   52     -4.450  18.246  21.328  1.00  17.06           N
ATOM   2076  CZ   ARG B   52     -4.417  18.985  22.434  1.00  16.69           C
ATOM   2077  NH1  ARG B   52     -5.432  19.018  23.277  1.00  16.15           N
ATOM   2078  NH2  ARG B   52     -3.320  19.674  22.719  1.00  16.02           N
ATOM   2079  N    LEU B   52A    -8.728  14.465  18.742  1.00  16.05           N
ATOM   2080  CA   LEU B   52A    -9.402  13.212  18.371  1.00  16.55           C
ATOM   2081  C    LEU B   52A   -10.831  13.430  17.860  1.00  15.12           C
ATOM   2082  O    LEU B   52A   -11.394  14.513  17.949  1.00  14.41           O
ATOM   2083  CB   LEU B   52A    -9.448  12.253  19.565  1.00  16.40           C
ATOM   2084  CG   LEU B   52A    -8.122  12.000  20.299  1.00  15.84           C
ATOM   2085  CD1  LEU B   52A    -8.308  10.970  21.393  1.00  17.61           C
ATOM   2086  CD2  LEU B   52A    -7.076  11.581  19.319  1.00  17.01           C
ATOM   2087  N    LYS B   52B   -11.423  12.369  17.345  1.00  16.37           N
ATOM   2088  CA   LYS B   52B   -12.812  12.430  16.909  1.00  18.75           C
ATOM   2089  C    LYS B   52B   -13.704  12.938  18.065  1.00  18.78           C
ATOM   2090  O    LYS B   52B   -14.518  13.874  17.907  1.00  20.39           O
ATOM   2091  CB   LYS B   52B   -13.260  11.053  16.414  1.00  18.71           C
ATOM   2092  CG   LYS B   52B   -14.675  11.062  15.874  1.00  19.16           C
ATOM   2093  CD   LYS B   52B   -15.021   9.725  15.240  1.00  21.00           C
ATOM   2094  CE   LYS B   52B   -16.545   9.574  15.064  1.00  22.36           C
ATOM   2095  NZ   LYS B   52B   -16.815   8.201  14.569  1.00  27.78           N
ATOM   2096  N    SER B   52C   -13.459  12.401  19.253  1.00  19.70           N
ATOM   2097  CA   SER B   52C   -14.213  12.755  20.443  1.00  19.51           C
ATOM   2098  C    SER B   52C   -14.057  14.211  20.866  1.00  19.55           C
ATOM   2099  O    SER B   52C   -14.868  14.731  21.629  1.00  20.54           O
ATOM   2100  CB   SER B   52C   -13.855  11.803  21.605  1.00  20.98           C
ATOM   2101  OG   SER B   52C   -12.458  11.709  21.790  1.00  22.52           O
ATOM   2102  N    ASP B   53    -13.043  14.887  20.340  1.00  19.28           N
ATOM   2103  CA   ASP B   53    -12.816  16.292  20.600  1.00  17.30           C
ATOM   2104  C    ASP B   53    -13.309  17.120  19.424  1.00  17.65           C
ATOM   2105  O    ASP B   53    -12.965  18.286  19.294  1.00  17.18           O
ATOM   2106  CB   ASP B   53    -11.310  16.527  20.792  1.00  17.88           C
ATOM   2107  CG   ASP B   53    -10.699  15.570  21.796  1.00  17.82           C
ATOM   2108  OD1  ASP B   53    -11.225  15.549  22.935  1.00  18.83           O
ATOM   2109  OD2  ASP B   53     -9.750  14.830  21.437  1.00  16.93           O
ATOM   2110  N    ASN B   54    -14.122  16.507  18.565  1.00  17.76           N
ATOM   2111  CA   ASN B   54    -14.578  17.117  17.328  1.00  16.94           C
ATOM   2112  C    ASN B   54    -13.384  17.609  16.478  1.00  17.02           C
ATOM   2113  O    ASN B   54    -13.458  18.651  15.812  1.00  16.51           O
ATOM   2114  CB   ASN B   54    -15.598  18.227  17.610  1.00  18.86           C
ATOM   2115  CG   ASN B   54    -16.972  17.681  18.011  1.00  16.82           C
ATOM   2116  OD1  ASN B   54    -17.722  18.354  18.691  1.00  23.48           O
ATOM   2117  ND2  ASN B   54    -17.298  16.469  17.581  1.00  17.63           N
ATOM   2118  N    TYR B   55    -12.300  16.838  16.541  1.00  16.25           N
ATOM   2119  CA   TYR B   55    -11.085  17.074  15.797  1.00  15.65           C
ATOM   2120  C    TYR B   55    -10.574  18.509  15.986  1.00  15.25           C
ATOM   2121  O    TYR B   55    -10.128  19.157  15.044  1.00  14.34           O
ATOM   2122  CB   TYR B   55    -11.323  16.774  14.328  1.00  16.18           C
ATOM   2123  CG   TYR B   55    -11.617  15.323  14.029  1.00  16.80           C
ATOM   2124  CD1  TYR B   55    -10.686  14.334  14.347  1.00  15.87           C
ATOM   2125  CD2  TYR B   55    -12.798  14.931  13.407  1.00  17.21           C
ATOM   2126  CE1  TYR B   55    -10.900  13.012  14.057  1.00  17.27           C
ATOM   2127  CE2  TYR B   55    -13.030  13.582  13.115  1.00  15.35           C
ATOM   2128  CZ   TYR B   55    -12.068  12.632  13.436  1.00  16.76           C
ATOM   2129  OH   TYR B   55    -12.252  11.288  13.146  1.00  17.72           O
ATOM   2130  N    ALA B   56    -10.616  18.973  17.231  1.00  15.01           N
ATOM   2131  CA   ALA B   56    -10.163  20.306  17.555  1.00  15.27           C
ATOM   2132  C    ALA B   56     -8.750  20.547  17.000  1.00  14.55           C
ATOM   2133  O    ALA B   56     -7.902  19.652  17.029  1.00  15.22           O
```

```
ATOM   2134   CB    ALA B   56    -10.202   20.505   19.066   1.00 15.64        C
ATOM   2135   N     THR B   57     -8.523   21.741   16.439   1.00 15.72        N
ATOM   2136   CA    THR B   57     -7.219   22.097   15.850   1.00 15.59        C
ATOM   2137   C     THR B   57     -6.608   23.303   16.516   1.00 15.74        C
ATOM   2138   O     THR B   57     -7.331   24.140   17.060   1.00 16.40        O
ATOM   2139   CB    THR B   57     -7.298   22.379   14.358   1.00 16.69        C
ATOM   2140   OG1   THR B   57     -8.151   23.511   14.127   1.00 17.64        O
ATOM   2141   CG2   THR B   57     -7.796   21.128   13.629   1.00 17.15        C
ATOM   2142   N     TYR B   58     -5.267   23.368   16.493   1.00 15.50        N
ATOM   2143   CA    TYR B   58     -4.495   24.476   17.104   1.00 15.51        C
ATOM   2144   C     TYR B   58     -3.319   24.806   16.231   1.00 15.59        C
ATOM   2145   O     TYR B   58     -2.720   23.904   15.650   1.00 14.08        O
ATOM   2146   CB    TYR B   58     -3.987   24.083   18.497   1.00 16.73        C
ATOM   2147   CG    TYR B   58     -5.106   23.584   19.367   1.00 16.83        C
ATOM   2148   CD1   TYR B   58     -5.934   24.477   20.051   1.00 18.35        C
ATOM   2149   CD2   TYR B   58     -5.363   22.226   19.484   1.00 17.40        C
ATOM   2150   CE1   TYR B   58     -6.978   24.009   20.833   1.00 18.41        C
ATOM   2151   CE2   TYR B   58     -6.407   21.755   20.245   1.00 18.46        C
ATOM   2152   CZ    TYR B   58     -7.212   22.649   20.917   1.00 18.02        C
ATOM   2153   OH    TYR B   58     -8.242   22.165   21.683   1.00 20.00        O
ATOM   2154   N     TYR B   59     -2.962   26.095   16.168   1.00 15.24        N
ATOM   2155   CA    TYR B   59     -1.919   26.568   15.263   1.00 15.46        C
ATOM   2156   C     TYR B   59     -0.974   27.543   15.929   1.00 15.74        C
ATOM   2157   O     TYR B   59     -1.380   28.344   16.784   1.00 15.85        O
ATOM   2158   CB    TYR B   59     -2.548   27.262   14.056   1.00 16.01        C
ATOM   2159   CG    TYR B   59     -3.419   26.339   13.254   1.00 15.89        C
ATOM   2160   CD1   TYR B   59     -2.866   25.479   12.308   1.00 15.44        C
ATOM   2161   CD2   TYR B   59     -4.795   26.309   13.444   1.00 15.31        C
ATOM   2162   CE1   TYR B   59     -3.658   24.612   11.605   1.00 16.87        C
ATOM   2163   CE2   TYR B   59     -5.588   25.446   12.741   1.00 16.27        C
ATOM   2164   CZ    TYR B   59     -5.026   24.619   11.790   1.00 14.90        C
ATOM   2165   OH    TYR B   59     -5.830   23.742   11.073   1.00 14.11        O
ATOM   2166   N     ALA B   60      0.290   27.500   15.517   1.00 16.11        N
ATOM   2167   CA    ALA B   60      1.226   28.552   15.894   1.00 17.42        C
ATOM   2168   C     ALA B   60      0.735   29.871   15.306   1.00 19.53        C
ATOM   2169   O     ALA B   60      0.141   29.885   14.228   1.00 19.38        O
ATOM   2170   CB    ALA B   60      2.621   28.239   15.385   1.00 17.44        C
ATOM   2171   N     GLU B   61      0.982   30.960   16.034   1.00 22.95        N
ATOM   2172   CA    GLU B   61      0.686   32.326   15.561   1.00 26.11        C
ATOM   2173   C     GLU B   61      1.395   32.624   14.255   1.00 27.44        C
ATOM   2174   O     GLU B   61      0.832   33.263   13.387   1.00 27.45        O
ATOM   2175   CB    GLU B   61      1.121   33.371   16.599   1.00 28.05        C
ATOM   2176   CG    GLU B   61      0.251   33.437   17.827   1.00 32.42        C
ATOM   2177   CD    GLU B   61     -1.166   33.901   17.512   1.00 35.92        C
ATOM   2178   OE1   GLU B   61     -1.321   34.911   16.774   1.00 39.79        O
ATOM   2179   OE2   GLU B   61     -2.120   33.243   17.992   1.00 40.81        O
ATOM   2180   N     SER B   62      2.641   32.163   14.134   1.00 29.21        N
ATOM   2181   CA    SER B   62      3.437   32.323   12.902   1.00 31.40        C
ATOM   2182   C     SER B   62      2.795   31.790   11.598   1.00 31.50        C
ATOM   2183   O     SER B   62      3.216   32.180   10.506   1.00 34.12        O
ATOM   2184   CB    SER B   62      4.808   31.670   13.086   1.00 32.21        C
ATOM   2185   OG    SER B   62      4.687   30.269   13.332   1.00 33.86        O
ATOM   2186   N     VAL B   63      1.796   30.916   11.688   1.00 30.72        N
ATOM   2187   CA    VAL B   63      1.158   30.338   10.496   1.00 30.79        C
ATOM   2188   C     VAL B   63     -0.370   30.315   10.507   1.00 31.93        C
ATOM   2189   O     VAL B   63     -0.994   29.791    9.568   1.00 30.79        O
ATOM   2190   CB    VAL B   63      1.627   28.872   10.277   1.00 30.97        C
ATOM   2191   CG1   VAL B   63      3.147   28.793   10.230   1.00 30.25        C
ATOM   2192   CG2   VAL B   63      1.070   27.946   11.365   1.00 30.27        C
ATOM   2193   N     LYS B   64     -1.003   30.841   11.561   1.00 33.49        N
ATOM   2194   CA    LYS B   64     -2.454   30.746   11.634   1.00 34.37        C
ATOM   2195   C     LYS B   64     -3.036   31.570   10.469   1.00 32.73        C
ATOM   2196   O     LYS B   64     -2.522   32.639   10.125   1.00 34.39        O
ATOM   2197   CB    LYS B   64     -2.998   31.177   13.006   1.00 35.30        C
ATOM   2198   CG    LYS B   64     -3.012   32.684   13.263   1.00 36.32        C
```

| ATOM | 2199 | CD | LYS | B | 64 | -3.755 | 32.993 | 14.567 | 1.00 | 36.74 | C |
|------|------|-----|-----|---|----|--------|--------|--------|------|-------|---|
| ATOM | 2200 | CE | LYS | B | 64 | -3.995 | 34.486 | 14.734 | 1.00 | 38.27 | C |
| ATOM | 2201 | NZ | LYS | B | 64 | -2.727 | 35.272 | 14.643 | 1.00 | 39.45 | N |
| ATOM | 2202 | N | GLY | B | 65 | -4.066 | 31.030 | 9.846 | 1.00 | 31.27 | N |
| ATOM | 2203 | CA | GLY | B | 65 | -4.664 | 31.607 | 8.663 | 1.00 | 31.04 | C |
| ATOM | 2204 | C | GLY | B | 65 | -4.145 | 30.973 | 7.385 | 1.00 | 30.23 | C |
| ATOM | 2205 | O | GLY | B | 65 | -4.827 | 31.011 | 6.362 | 1.00 | 32.26 | O |
| ATOM | 2206 | N | LYS | B | 66 | -2.941 | 30.402 | 7.431 | 1.00 | 27.87 | N |
| ATOM | 2207 | CA | LYS | B | 66 | -2.285 | 29.841 | 6.239 | 1.00 | 26.67 | C |
| ATOM | 2208 | C | LYS | B | 66 | -2.220 | 28.327 | 6.196 | 1.00 | 24.96 | C |
| ATOM | 2209 | O | LYS | B | 66 | -2.181 | 27.753 | 5.107 | 1.00 | 22.77 | O |
| ATOM | 2210 | CB | LYS | B | 66 | -0.859 | 30.317 | 6.166 | 1.00 | 28.29 | C |
| ATOM | 2211 | CG | LYS | B | 66 | -0.684 | 31.678 | 5.634 | 1.00 | 29.97 | C |
| ATOM | 2212 | CD | LYS | B | 66 | 0.705 | 32.145 | 5.918 | 1.00 | 31.90 | C |
| ATOM | 2213 | CE | LYS | B | 66 | 1.760 | 31.173 | 5.432 | 1.00 | 33.62 | C |
| ATOM | 2214 | NZ | LYS | B | 66 | 3.114 | 31.830 | 5.399 | 1.00 | 34.69 | N |
| ATOM | 2215 | N | PHE | B | 67 | -2.143 | 27.686 | 7.363 | 1.00 | 21.98 | N |
| ATOM | 2216 | CA | PHE | B | 67 | -2.065 | 26.228 | 7.433 | 1.00 | 22.00 | C |
| ATOM | 2217 | C | PHE | B | 67 | -3.390 | 25.639 | 7.935 | 1.00 | 22.01 | C |
| ATOM | 2218 | O | PHE | B | 67 | -4.064 | 26.239 | 8.798 | 1.00 | 20.76 | O |
| ATOM | 2219 | CB | PHE | B | 67 | -0.930 | 25.808 | 8.375 | 1.00 | 23.04 | C |
| ATOM | 2220 | CG | PHE | B | 67 | 0.467 | 26.060 | 7.848 | 1.00 | 23.41 | C |
| ATOM | 2221 | CD1 | PHE | B | 67 | 0.717 | 26.828 | 6.714 | 1.00 | 23.22 | C |
| ATOM | 2222 | CD2 | PHE | B | 67 | 1.561 | 25.522 | 8.522 | 1.00 | 22.84 | C |
| ATOM | 2223 | CE1 | PHE | B | 67 | 2.006 | 27.036 | 6.265 | 1.00 | 22.64 | C |
| ATOM | 2224 | CE2 | PHE | B | 67 | 2.864 | 25.751 | 8.063 | 1.00 | 23.92 | C |
| ATOM | 2225 | CZ | PHE | B | 67 | 3.078 | 26.499 | 6.946 | 1.00 | 23.04 | C |
| ATOM | 2226 | N | THR | B | 68 | -3.743 | 24.471 | 7.384 | 1.00 | 20.21 | N |
| ATOM | 2227 | CA | THR | B | 68 | -4.865 | 23.663 | 7.826 | 1.00 | 21.22 | C |
| ATOM | 2228 | C | THR | B | 68 | -4.431 | 22.222 | 8.054 | 1.00 | 19.36 | C |
| ATOM | 2229 | O | THR | B | 68 | -3.935 | 21.548 | 7.136 | 1.00 | 18.41 | O |
| ATOM | 2230 | CB | THR | B | 68 | -6.014 | 23.694 | 6.795 | 1.00 | 21.82 | C |
| ATOM | 2231 | OG1 | THR | B | 68 | -6.426 | 25.048 | 6.617 | 1.00 | 23.55 | O |
| ATOM | 2232 | CG2 | THR | B | 68 | -7.190 | 22.889 | 7.267 | 1.00 | 21.43 | C |
| ATOM | 2233 | N | ILE | B | 69 | -4.598 | 21.756 | 9.292 | 1.00 | 17.86 | N |
| ATOM | 2234 | CA | ILE | B | 69 | -4.277 | 20.363 | 9.643 | 1.00 | 15.72 | C |
| ATOM | 2235 | C | ILE | B | 69 | -5.534 | 19.490 | 9.634 | 1.00 | 16.32 | C |
| ATOM | 2236 | O | ILE | B | 69 | -6.587 | 19.922 | 10.086 | 1.00 | 16.05 | O |
| ATOM | 2237 | CB | ILE | B | 69 | -3.503 | 20.317 | 11.005 | 1.00 | 15.51 | C |
| ATOM | 2238 | CG1 | ILE | B | 69 | -2.906 | 18.928 | 11.252 | 1.00 | 14.36 | C |
| ATOM | 2239 | CG2 | ILE | B | 69 | -4.360 | 20.831 | 12.148 | 1.00 | 14.78 | C |
| ATOM | 2240 | CD1 | ILE | B | 69 | -2.068 | 18.824 | 12.535 | 1.00 | 16.00 | C |
| ATOM | 2241 | N | SER | B | 70 | -5.433 | 18.271 | 9.090 | 1.00 | 16.27 | N |
| ATOM | 2242 | CA | SER | B | 70 | -6.574 | 17.357 | 9.041 | 1.00 | 15.71 | C |
| ATOM | 2243 | C | SER | B | 70 | -6.071 | 15.929 | 9.154 | 1.00 | 15.98 | C |
| ATOM | 2244 | O | SER | B | 70 | -4.892 | 15.686 | 9.021 | 1.00 | 15.71 | O |
| ATOM | 2245 | CB | SER | B | 70 | -7.393 | 17.543 | 7.748 | 1.00 | 16.34 | C |
| ATOM | 2246 | OG | SER | B | 70 | -6.599 | 17.283 | 6.615 | 1.00 | 17.81 | O |
| ATOM | 2247 | N | ARG | B | 71 | -6.972 | 14.999 | 9.431 | 1.00 | 15.85 | N |
| ATOM | 2248 | CA | ARG | B | 71 | -6.608 | 13.595 | 9.614 | 1.00 | 17.09 | C |
| ATOM | 2249 | C | ARG | B | 71 | -7.675 | 12.689 | 9.009 | 1.00 | 18.13 | C |
| ATOM | 2250 | O | ARG | B | 71 | -8.855 | 13.002 | 9.044 | 1.00 | 20.29 | O |
| ATOM | 2251 | CB | ARG | B | 71 | -6.385 | 13.279 | 11.099 | 1.00 | 16.01 | C |
| ATOM | 2252 | CG | ARG | B | 71 | -7.626 | 13.398 | 11.980 | 1.00 | 17.02 | C |
| ATOM | 2253 | CD | ARG | B | 71 | -7.329 | 13.356 | 13.468 | 1.00 | 16.13 | C |
| ATOM | 2254 | NE | ARG | B | 71 | -6.764 | 12.080 | 13.896 | 1.00 | 15.36 | N |
| ATOM | 2255 | CZ | ARG | B | 71 | -5.965 | 11.918 | 14.952 | 1.00 | 16.56 | C |
| ATOM | 2256 | NH1 | ARG | B | 71 | -5.604 | 12.957 | 15.731 | 1.00 | 14.48 | N |
| ATOM | 2257 | NH2 | ARG | B | 71 | -5.506 | 10.704 | 15.220 | 1.00 | 18.06 | N |
| ATOM | 2258 | N | ASP | B | 72 | -7.238 | 11.569 | 8.448 | 1.00 | 20.16 | N |
| ATOM | 2259 | CA | ASP | B | 72 | -8.145 | 10.508 | 8.018 | 1.00 | 21.21 | C |
| ATOM | 2260 | C | ASP | B | 72 | -7.863 | 9.263 | 8.845 | 1.00 | 20.51 | C |
| ATOM | 2261 | O | ASP | B | 72 | -6.958 | 8.493 | 8.552 | 1.00 | 19.69 | O |
| ATOM | 2262 | CB | ASP | B | 72 | -7.982 | 10.237 | 6.523 | 1.00 | 22.37 | C |
| ATOM | 2263 | CG | ASP | B | 72 | -8.976 | 9.219 | 6.003 | 1.00 | 25.28 | C |

| ATOM | 2264 | OD1 | ASP | B | 72 | -9.576 | 8.469 | 6.805 | 1.00 | 28.02 | O |
|------|------|-----|-----|---|----|--------|-------|-------|------|-------|---|
| ATOM | 2265 | OD2 | ASP | B | 72 | -9.153 | 9.175 | 4.779 | 1.00 | 28.27 | O |
| ATOM | 2266 | N | ASP | B | 73 | -8.656 | 9.075 | 9.894 | 1.00 | 20.44 | N |
| ATOM | 2267 | CA | ASP | B | 73 | -8.419 | 8.001 | 10.833 | 1.00 | 21.34 | C |
| ATOM | 2268 | C | ASP | B | 73 | -8.491 | 6.652 | 10.112 | 1.00 | 22.67 | C |
| ATOM | 2269 | O | ASP | B | 73 | -7.752 | 5.726 | 10.448 | 1.00 | 23.30 | O |
| ATOM | 2270 | CB | ASP | B | 73 | -9.432 | 8.062 | 11.993 | 1.00 | 20.68 | C |
| ATOM | 2271 | CG | ASP | B | 73 | -9.157 | 9.216 | 12.969 | 1.00 | 21.68 | C |
| ATOM | 2272 | OD1 | ASP | B | 73 | -8.028 | 9.733 | 12.939 | 1.00 | 21.21 | O |
| ATOM | 2273 | OD2 | ASP | B | 73 | -10.073 | 9.593 | 13.756 | 1.00 | 20.45 | O |
| ATOM | 2274 | N | SER | B | 74 | -9.376 | 6.558 | 9.124 | 1.00 | 24.16 | N |
| ATOM | 2275 | CA | SER | B | 74 | -9.540 | 5.310 | 8.348 | 1.00 | 24.65 | C |
| ATOM | 2276 | C | SER | B | 74 | -8.295 | 4.918 | 7.553 | 1.00 | 25.59 | C |
| ATOM | 2277 | O | SER | B | 74 | -8.062 | 3.748 | 7.317 | 1.00 | 27.51 | O |
| ATOM | 2278 | CB | SER | B | 74 | -10.779 | 5.384 | 7.424 | 1.00 | 24.84 | C |
| ATOM | 2279 | OG | SER | B | 74 | -10.584 | 6.173 | 6.255 | 1.00 | 23.49 | O |
| ATOM | 2280 | N | LYS | B | 75 | -7.468 | 5.882 | 7.175 | 1.00 | 24.99 | N |
| ATOM | 2281 | CA | LYS | B | 75 | -6.263 | 5.576 | 6.429 | 1.00 | 25.75 | C |
| ATOM | 2282 | C | LYS | B | 75 | -4.998 | 5.707 | 7.265 | 1.00 | 23.83 | C |
| ATOM | 2283 | O | LYS | B | 75 | -3.901 | 5.515 | 6.743 | 1.00 | 22.75 | O |
| ATOM | 2284 | CB | LYS | B | 75 | -6.174 | 6.507 | 5.227 | 1.00 | 27.02 | C |
| ATOM | 2285 | CG | LYS | B | 75 | -7.298 | 6.357 | 4.219 | 1.00 | 29.02 | C |
| ATOM | 2286 | CD | LYS | B | 75 | -7.142 | 7.427 | 3.122 | 1.00 | 29.18 | C |
| ATOM | 2287 | CE | LYS | B | 75 | -8.353 | 7.515 | 2.169 | 1.00 | 31.08 | C |
| ATOM | 2288 | NZ | LYS | B | 75 | -8.201 | 8.665 | 1.203 | 1.00 | 32.31 | N |
| ATOM | 2289 | N | SER | B | 76 | -5.151 | 6.060 | 8.549 | 1.00 | 22.96 | N |
| ATOM | 2290 | CA | SER | B | 76 | -4.019 | 6.328 | 9.440 | 1.00 | 21.71 | C |
| ATOM | 2291 | C | SER | B | 76 | -3.111 | 7.428 | 8.885 | 1.00 | 19.97 | C |
| ATOM | 2292 | O | SER | B | 76 | -1.902 | 7.354 | 9.026 | 1.00 | 19.25 | O |
| ATOM | 2293 | CB | SER | B | 76 | -3.218 | 5.038 | 9.696 | 1.00 | 21.41 | C |
| ATOM | 2294 | OG | SER | B | 76 | -3.975 | 4.116 | 10.458 | 1.00 | 23.61 | O |
| ATOM | 2295 | N | ARG | B | 77 | -3.717 | 8.443 | 8.269 | 1.00 | 20.03 | N |
| ATOM | 2296 | CA | ARG | B | 77 | -2.990 | 9.483 | 7.539 | 1.00 | 20.75 | C |
| ATOM | 2297 | C | ARG | B | 77 | -3.289 | 10.879 | 8.095 | 1.00 | 18.49 | C |
| ATOM | 2298 | O | ARG | B | 77 | -4.443 | 11.224 | 8.348 | 1.00 | 17.72 | O |
| ATOM | 2299 | CB | ARG | B | 77 | -3.349 | 9.428 | 6.050 | 1.00 | 21.92 | C |
| ATOM | 2300 | CG | ARG | B | 77 | -2.365 | 10.158 | 5.162 | 1.00 | 25.08 | C |
| ATOM | 2301 | CD | ARG | B | 77 | -2.405 | 9.665 | 3.719 | 1.00 | 26.42 | C |
| ATOM | 2302 | NE | ARG | B | 77 | -1.540 | 8.501 | 3.458 | 1.00 | 30.39 | N |
| ATOM | 2303 | CZ | ARG | B | 77 | -0.213 | 8.553 | 3.267 | 1.00 | 32.53 | C |
| ATOM | 2304 | NH1 | ARG | B | 77 | 0.470 | 9.701 | 3.365 | 1.00 | 30.63 | N |
| ATOM | 2305 | NH2 | ARG | B | 77 | 0.455 | 7.430 | 2.997 | 1.00 | 35.47 | N |
| ATOM | 2306 | N | LEU | B | 78 | -2.234 | 11.653 | 8.317 | 1.00 | 17.36 | N |
| ATOM | 2307 | CA | LEU | B | 78 | -2.315 | 13.037 | 8.782 | 1.00 | 16.57 | C |
| ATOM | 2308 | C | LEU | B | 78 | -1.954 | 13.938 | 7.628 | 1.00 | 16.36 | C |
| ATOM | 2309 | O | LEU | B | 78 | -1.073 | 13.611 | 6.869 | 1.00 | 17.07 | O |
| ATOM | 2310 | CB | LEU | B | 78 | -1.303 | 13.274 | 9.917 | 1.00 | 17.11 | C |
| ATOM | 2311 | CG | LEU | B | 78 | -1.204 | 14.691 | 10.484 | 1.00 | 17.29 | C |
| ATOM | 2312 | CD1 | LEU | B | 78 | -2.451 | 15.045 | 11.209 | 1.00 | 17.50 | C |
| ATOM | 2313 | CD2 | LEU | B | 78 | 0.029 | 14.787 | 11.394 | 1.00 | 17.11 | C |
| ATOM | 2314 | N | TYR | B | 79 | -2.611 | 15.097 | 7.528 | 1.00 | 16.90 | N |
| ATOM | 2315 | CA | TYR | B | 79 | -2.298 | 16.063 | 6.486 | 1.00 | 17.73 | C |
| ATOM | 2316 | C | TYR | B | 79 | -1.971 | 17.464 | 7.004 | 1.00 | 15.89 | C |
| ATOM | 2317 | O | TYR | B | 79 | -2.413 | 17.868 | 8.074 | 1.00 | 15.07 | O |
| ATOM | 2318 | CB | TYR | B | 79 | -3.479 | 16.213 | 5.555 | 1.00 | 19.95 | C |
| ATOM | 2319 | CG | TYR | B | 79 | -3.952 | 14.936 | 4.907 | 1.00 | 20.20 | C |
| ATOM | 2320 | CD1 | TYR | B | 79 | -3.373 | 14.477 | 3.744 | 1.00 | 22.71 | C |
| ATOM | 2321 | CD2 | TYR | B | 79 | -5.006 | 14.226 | 5.439 | 1.00 | 20.73 | C |
| ATOM | 2322 | CE1 | TYR | B | 79 | -3.813 | 13.293 | 3.132 | 1.00 | 23.66 | C |
| ATOM | 2323 | CE2 | TYR | B | 79 | -5.468 | 13.070 | 4.839 | 1.00 | 23.83 | C |
| ATOM | 2324 | CZ | TYR | B | 79 | -4.863 | 12.612 | 3.678 | 1.00 | 23.58 | C |
| ATOM | 2325 | OH | TYR | B | 79 | -5.317 | 11.448 | 3.080 | 1.00 | 26.34 | O |
| ATOM | 2326 | N | LEU | B | 80 | -1.205 | 18.190 | 6.203 | 1.00 | 15.42 | N |
| ATOM | 2327 | CA | LEU | B | 80 | -1.010 | 19.622 | 6.389 | 1.00 | 15.39 | C |
| ATOM | 2328 | C | LEU | B | 80 | -1.124 | 20.328 | 5.035 | 1.00 | 17.00 | C |

| ATOM | 2329 | O | LEU | B | 80 | -0.284 | 20.109 | 4.144 | 1.00 | 14.14 | O |
|------|------|------|-----|---|-----|--------|--------|--------|------|-------|---|
| ATOM | 2330 | CB | LEU | B | 80 | 0.342 | 19.907 | 7.005 | 1.00 | 14.95 | C |
| ATOM | 2331 | CG | LEU | B | 80 | 0.568 | 21.363 | 7.451 | 1.00 | 13.97 | C |
| ATOM | 2332 | CD1 | LEU | B | 80 | -0.456 | 21.855 | 8.502 | 1.00 | 15.59 | C |
| ATOM | 2333 | CD2 | LEU | B | 80 | 1.952 | 21.519 | 7.962 | 1.00 | 14.19 | C |
| ATOM | 2334 | N | GLN | B | 81 | -2.177 | 21.138 | 4.898 | 1.00 | 17.61 | N |
| ATOM | 2335 | CA | GLN | B | 81 | -2.414 | 21.998 | 3.742 | 1.00 | 17.77 | C |
| ATOM | 2336 | C | GLN | B | 81 | -1.813 | 23.376 | 4.057 | 1.00 | 18.18 | C |
| ATOM | 2337 | O | GLN | B | 81 | -2.138 | 23.991 | 5.081 | 1.00 | 17.39 | O |
| ATOM | 2338 | CB | GLN | B | 81 | -3.930 | 22.118 | 3.447 | 1.00 | 17.86 | C |
| ATOM | 2339 | CG | GLN | B | 81 | -4.277 | 23.084 | 2.285 | 1.00 | 19.82 | C |
| ATOM | 2340 | CD | GLN | B | 81 | -3.753 | 22.585 | 0.970 | 1.00 | 22.73 | C |
| ATOM | 2341 | OE1 | GLN | B | 81 | -4.042 | 21.455 | 0.562 | 1.00 | 25.57 | O |
| ATOM | 2342 | NE2 | GLN | B | 81 | -2.951 | 23.410 | 0.299 | 1.00 | 25.47 | N |
| ATOM | 2343 | N | MET | B | 82 | -0.909 | 23.834 | 3.193 | 1.00 | 17.88 | N |
| ATOM | 2344 | CA | MET | B | 82 | -0.130 | 25.024 | 3.434 | 1.00 | 21.31 | C |
| ATOM | 2345 | C | MET | B | 82 | -0.385 | 25.999 | 2.287 | 1.00 | 20.91 | C |
| ATOM | 2346 | O | MET | B | 82 | -0.138 | 25.668 | 1.137 | 1.00 | 20.90 | O |
| ATOM | 2347 | CB | MET | B | 82 | 1.345 | 24.633 | 3.521 | 1.00 | 21.87 | C |
| ATOM | 2348 | CG | MET | B | 82 | 1.558 | 23.264 | 4.220 | 1.00 | 25.21 | C |
| ATOM | 2349 | SD | MET | B | 82 | 3.212 | 22.546 | 3.946 | 1.00 | 28.42 | S |
| ATOM | 2350 | CE | MET | B | 82 | 4.001 | 23.778 | 4.854 | 1.00 | 20.44 | C |
| ATOM | 2351 | N | ASN | B | 82A | -0.908 | 27.177 | 2.610 | 1.00 | 21.86 | N |
| ATOM | 2352 | CA | ASN | B | 82A | -1.263 | 28.194 | 1.611 | 1.00 | 22.87 | C |
| ATOM | 2353 | C | ASN | B | 82A | -0.447 | 29.435 | 1.818 | 1.00 | 22.05 | C |
| ATOM | 2354 | O | ASN | B | 82A | 0.068 | 29.642 | 2.901 | 1.00 | 21.01 | O |
| ATOM | 2355 | CB | ASN | B | 82A | -2.749 | 28.549 | 1.692 | 1.00 | 23.98 | C |
| ATOM | 2356 | CG | ASN | B | 82A | -3.630 | 27.377 | 1.382 | 1.00 | 26.18 | C |
| ATOM | 2357 | OD1 | ASN | B | 82A | -3.433 | 26.686 | 0.391 | 1.00 | 27.62 | O |
| ATOM | 2358 | ND2 | ASN | B | 82A | -4.602 | 27.121 | 2.253 | 1.00 | 30.51 | N |
| ATOM | 2359 | N | ASN | B | 82B | -0.360 | 30.279 | 0.782 | 1.00 | 24.15 | N |
| ATOM | 2360 | CA | ASN | B | 82B | 0.514 | 31.469 | 0.787 | 1.00 | 24.59 | C |
| ATOM | 2361 | C | ASN | B | 82B | 1.846 | 31.259 | 1.448 | 1.00 | 25.13 | C |
| ATOM | 2362 | O | ASN | B | 82B | 2.281 | 32.044 | 2.292 | 1.00 | 25.77 | O |
| ATOM | 2363 | CB | ASN | B | 82B | -0.137 | 32.705 | 1.424 | 1.00 | 26.72 | C |
| ATOM | 2364 | CG | ASN | B | 82B | 0.784 | 33.939 | 1.346 | 1.00 | 27.85 | C |
| ATOM | 2365 | OD1 | ASN | B | 82B | 1.541 | 34.094 | 0.375 | 1.00 | 33.47 | O |
| ATOM | 2366 | ND2 | ASN | B | 82B | 0.759 | 34.790 | 2.379 | 1.00 | 32.11 | N |
| ATOM | 2367 | N | LEU | B | 82C | 2.534 | 30.205 | 1.035 | 1.00 | 24.05 | N |
| ATOM | 2368 | CA | LEU | B | 82C | 3.825 | 29.921 | 1.571 | 1.00 | 23.74 | C |
| ATOM | 2369 | C | LEU | B | 82C | 4.789 | 31.011 | 1.203 | 1.00 | 23.60 | C |
| ATOM | 2370 | O | LEU | B | 82C | 4.728 | 31.565 | 0.092 | 1.00 | 24.85 | O |
| ATOM | 2371 | CB | LEU | B | 82C | 4.319 | 28.556 | 1.075 | 1.00 | 23.32 | C |
| ATOM | 2372 | CG | LEU | B | 82C | 3.611 | 27.390 | 1.771 | 1.00 | 22.78 | C |
| ATOM | 2373 | CD1 | LEU | B | 82C | 3.805 | 26.088 | 1.024 | 1.00 | 21.47 | C |
| ATOM | 2374 | CD2 | LEU | B | 82C | 4.132 | 27.249 | 3.191 | 1.00 | 24.02 | C |
| ATOM | 2375 | N | ARG | B | 83 | 5.652 | 31.340 | 2.155 | 1.00 | 23.98 | N |
| ATOM | 2376 | CA | ARG | B | 83 | 6.746 | 32.293 | 1.940 | 1.00 | 24.31 | C |
| ATOM | 2377 | C | ARG | B | 83 | 8.068 | 31.553 | 2.061 | 1.00 | 24.37 | C |
| ATOM | 2378 | O | ARG | B | 83 | 8.109 | 30.433 | 2.580 | 1.00 | 23.44 | O |
| ATOM | 2379 | CB | ARG | B | 83 | 6.695 | 33.409 | 2.983 | 1.00 | 25.86 | C |
| ATOM | 2380 | CG | ARG | B | 83 | 5.365 | 34.116 | 3.112 | 1.00 | 26.67 | C |
| ATOM | 2381 | CD | ARG | B | 83 | 4.730 | 34.508 | 1.782 | 1.00 | 27.78 | C |
| ATOM | 2382 | NE | ARG | B | 83 | 5.588 | 35.253 | 0.836 | 1.00 | 29.07 | N |
| ATOM | 2383 | CZ | ARG | B | 83 | 5.163 | 35.690 | -0.353 | 1.00 | 27.45 | C |
| ATOM | 2384 | NH1 | ARG | B | 83 | 3.916 | 35.450 | -0.739 | 1.00 | 29.19 | N |
| ATOM | 2385 | NH2 | ARG | B | 83 | 5.968 | 36.359 | -1.171 | 1.00 | 25.78 | N |
| ATOM | 2386 | N | THR | B | 84 | 9.155 | 32.188 | 1.636 | 1.00 | 22.74 | N |
| ATOM | 2387 | CA | THR | B | 84 | 10.483 | 31.586 | 1.718 | 1.00 | 23.23 | C |
| ATOM | 2388 | C | THR | B | 84 | 10.856 | 31.153 | 3.146 | 1.00 | 22.53 | C |
| ATOM | 2389 | O | THR | B | 84 | 11.507 | 30.124 | 3.346 | 1.00 | 20.77 | O |
| ATOM | 2390 | CB | THR | B | 84 | 11.600 | 32.513 | 1.166 | 1.00 | 23.41 | C |
| ATOM | 2391 | OG1 | THR | B | 84 | 11.628 | 33.738 | 1.904 | 1.00 | 24.72 | O |
| ATOM | 2392 | CG2 | THR | B | 84 | 11.364 | 32.822 | -0.310 | 1.00 | 24.11 | C |
| ATOM | 2393 | N | GLU | B | 85 | 10.430 | 31.944 | 4.129 | 1.00 | 22.13 | N |

| ATOM | 2394 | CA | GLU B | 85 | 10.656 | 31.636 | 5.556 | 1.00 | 22.90 | C |
|------|------|----|-------|----|--------|--------|-------|------|-------|---|
| ATOM | 2395 | C | GLU B | 85 | 9.993 | 30.335 | 6.019 | 1.00 | 20.80 | C |
| ATOM | 2396 | O | GLU B | 85 | 10.358 | 29.807 | 7.084 | 1.00 | 20.83 | O |
| ATOM | 2397 | CB AGLU B | | 85 | 10.083 | 32.771 | 6.422 | 0.50 | 23.25 | C |
| ATOM | 2398 | CB BGLU B | | 85 | 10.233 | 32.792 | 6.473 | 0.50 | 22.36 | C |
| ATOM | 2399 | CG AGLU B | | 85 | 8.602 | 33.067 | 6.089 | 0.50 | 24.71 | C |
| ATOM | 2400 | CG BGLU B | | 85 | 8.847 | 33.334 | 6.208 | 0.50 | 22.34 | C |
| ATOM | 2401 | CD AGLU B | | 85 | 7.822 | 33.714 | 7.212 | 0.50 | 25.14 | C |
| ATOM | 2402 | CD BGLU B | | 85 | 8.887 | 34.665 | 5.469 | 0.50 | 21.62 | C |
| ATOM | 2403 | OE1AGLU B | | 85 | 8.435 | 34.363 | 8.088 | 0.50 | 28.87 | O |
| ATOM | 2404 | OE1BGLU B | | 85 | 9.288 | 34.698 | 4.284 | 0.50 | 18.57 | O |
| ATOM | 2405 | OE2AGLU B | | 85 | 6.582 | 33.584 | 7.201 | 0.50 | 27.42 | O |
| ATOM | 2406 | OE2BGLU B | | 85 | 8.523 | 35.677 | 6.101 | 0.50 | 21.47 | O |
| ATOM | 2407 | N | ASP B | 86 | 9.002 | 29.863 | 5.269 | 1.00 | 18.75 | N |
| ATOM | 2408 | CA | ASP B | 86 | 8.352 | 28.572 | 5.552 | 1.00 | 18.83 | C |
| ATOM | 2409 | C | ASP B | 86 | 9.155 | 27.357 | 5.036 | 1.00 | 18.56 | C |
| ATOM | 2410 | O | ASP B | 86 | 8.743 | 26.210 | 5.213 | 1.00 | 16.97 | O |
| ATOM | 2411 | CB | ASP B | 86 | 6.942 | 28.529 | 4.971 | 1.00 | 19.39 | C |
| ATOM | 2412 | CG | ASP B | 86 | 6.013 | 29.583 | 5.573 | 1.00 | 21.49 | C |
| ATOM | 2413 | OD1 | ASP B | 86 | 5.887 | 29.600 | 6.820 | 1.00 | 21.90 | O |
| ATOM | 2414 | OD2 | ASP B | 86 | 5.388 | 30.362 | 4.801 | 1.00 | 21.37 | O |
| ATOM | 2415 | N | THR B | 87 | 10.302 | 27.605 | 4.412 | 1.00 | 17.70 | N |
| ATOM | 2416 | CA | THR B | 87 | 11.189 | 26.522 | 3.994 | 1.00 | 16.53 | C |
| ATOM | 2417 | C | THR B | 87 | 11.700 | 25.727 | 5.199 | 1.00 | 17.00 | C |
| ATOM | 2418 | O | THR B | 87 | 12.152 | 26.306 | 6.201 | 1.00 | 17.72 | O |
| ATOM | 2419 | CB | THR B | 87 | 12.363 | 27.103 | 3.211 | 1.00 | 16.34 | C |
| ATOM | 2420 | OG1 | THR B | 87 | 11.854 | 27.727 | 2.025 | 1.00 | 15.53 | O |
| ATOM | 2421 | CG2 | THR B | 87 | 13.412 | 26.057 | 2.873 | 1.00 | 16.64 | C |
| ATOM | 2422 | N | GLY B | 88 | 11.664 | 24.397 | 5.098 | 1.00 | 15.36 | N |
| ATOM | 2423 | CA | GLY B | 88 | 12.231 | 23.588 | 6.143 | 1.00 | 15.18 | C |
| ATOM | 2424 | C | GLY B | 88 | 11.876 | 22.126 | 6.021 | 1.00 | 13.66 | C |
| ATOM | 2425 | O | GLY B | 88 | 11.192 | 21.717 | 5.081 | 1.00 | 12.32 | O |
| ATOM | 2426 | N | ILE B | 89 | 12.345 | 21.362 | 6.997 | 1.00 | 15.07 | N |
| ATOM | 2427 | CA | ILE B | 89 | 11.978 | 19.955 | 7.123 | 1.00 | 15.22 | C |
| ATOM | 2428 | C | ILE B | 89 | 10.736 | 19.876 | 8.012 | 1.00 | 14.33 | C |
| ATOM | 2429 | O | ILE B | 89 | 10.767 | 20.352 | 9.166 | 1.00 | 14.70 | O |
| ATOM | 2430 | CB | ILE B | 89 | 13.102 | 19.104 | 7.760 | 1.00 | 15.52 | C |
| ATOM | 2431 | CG1 | ILE B | 89 | 14.472 | 19.356 | 7.082 | 1.00 | 19.34 | C |
| ATOM | 2432 | CG2 | ILE B | 89 | 12.698 | 17.616 | 7.731 | 1.00 | 16.77 | C |
| ATOM | 2433 | CD1 | ILE B | 89 | 14.495 | 19.124 | 5.610 | 1.00 | 20.89 | C |
| ATOM | 2434 | N | TYR B | 90 | 9.655 | 19.300 | 7.486 | 1.00 | 13.04 | N |
| ATOM | 2435 | CA | TYR B | 90 | 8.417 | 19.132 | 8.229 | 1.00 | 13.01 | C |
| ATOM | 2436 | C | TYR B | 90 | 8.300 | 17.704 | 8.734 | 1.00 | 13.55 | C |
| ATOM | 2437 | O | TYR B | 90 | 8.292 | 16.766 | 7.949 | 1.00 | 12.11 | O |
| ATOM | 2438 | CB | TYR B | 90 | 7.200 | 19.512 | 7.365 | 1.00 | 14.54 | C |
| ATOM | 2439 | CG | TYR B | 90 | 7.080 | 21.028 | 7.168 | 1.00 | 12.66 | C |
| ATOM | 2440 | CD1 | TYR B | 90 | 8.019 | 21.718 | 6.427 | 1.00 | 16.90 | C |
| ATOM | 2441 | CD2 | TYR B | 90 | 6.051 | 21.751 | 7.749 | 1.00 | 12.36 | C |
| ATOM | 2442 | CE1 | TYR B | 90 | 7.915 | 23.138 | 6.260 | 1.00 | 15.17 | C |
| ATOM | 2443 | CE2 | TYR B | 90 | 5.941 | 23.130 | 7.601 | 1.00 | 13.11 | C |
| ATOM | 2444 | CZ | TYR B | 90 | 6.870 | 23.821 | 6.850 | 1.00 | 14.62 | C |
| ATOM | 2445 | OH | TYR B | 90 | 6.780 | 25.218 | 6.706 | 1.00 | 15.08 | O |
| ATOM | 2446 | N | TYR B | 91 | 8.181 | 17.583 | 10.053 | 1.00 | 13.82 | N |
| ATOM | 2447 | CA | TYR B | 91 | 8.060 | 16.294 | 10.730 | 1.00 | 14.61 | C |
| ATOM | 2448 | C | TYR B | 91 | 6.652 | 16.152 | 11.260 | 1.00 | 15.57 | C |
| ATOM | 2449 | O | TYR B | 91 | 6.089 | 17.113 | 11.839 | 1.00 | 14.13 | O |
| ATOM | 2450 | CB | TYR B | 91 | 8.976 | 16.237 | 11.965 | 1.00 | 15.57 | C |
| ATOM | 2451 | CG | TYR B | 91 | 10.461 | 16.265 | 11.710 | 1.00 | 15.63 | C |
| ATOM | 2452 | CD1 | TYR B | 91 | 11.149 | 15.107 | 11.379 | 1.00 | 14.89 | C |
| ATOM | 2453 | CD2 | TYR B | 91 | 11.174 | 17.433 | 11.839 | 1.00 | 15.98 | C |
| ATOM | 2454 | CE1 | TYR B | 91 | 12.523 | 15.127 | 11.160 | 1.00 | 16.96 | C |
| ATOM | 2455 | CE2 | TYR B | 91 | 12.536 | 17.466 | 11.635 | 1.00 | 18.00 | C |
| ATOM | 2456 | CZ | TYR B | 91 | 13.216 | 16.314 | 11.307 | 1.00 | 17.06 | C |
| ATOM | 2457 | OH | TYR B | 91 | 14.581 | 16.368 | 11.092 | 1.00 | 19.74 | O |
| ATOM | 2458 | N | CYS B | 92 | 6.107 | 14.943 | 11.144 | 1.00 | 15.87 | N |

| ATOM | 2459 | CA  | CYS | B | 92  | 4.974  | 14.555 | 11.977 | 1.00 | 16.90 | C |
|------|------|-----|-----|---|-----|--------|--------|--------|------|-------|---|
| ATOM | 2460 | C   | CYS | B | 92  | 5.523  | 14.225 | 13.345 | 1.00 | 15.46 | C |
| ATOM | 2461 | O   | CYS | B | 92  | 6.515  | 13.498 | 13.479 | 1.00 | 13.61 | O |
| ATOM | 2462 | CB  | CYS | B | 92  | 4.233  | 13.337 | 11.430 | 1.00 | 20.28 | C |
| ATOM | 2463 | SG  | CYS | B | 92  | 3.308  | 13.770 | 9.979  | 1.00 | 27.42 | S |
| ATOM | 2464 | N   | PHE | B | 93  | 4.885  | 14.799 | 14.356 | 1.00 | 15.42 | N |
| ATOM | 2465 | CA  | PHE | B | 93  | 5.257  | 14.593 | 15.741 | 1.00 | 13.64 | C |
| ATOM | 2466 | C   | PHE | B | 93  | 3.987  | 14.263 | 16.489 | 1.00 | 13.93 | C |
| ATOM | 2467 | O   | PHE | B | 93  | 3.046  | 15.071 | 16.511 | 1.00 | 12.71 | O |
| ATOM | 2468 | CB  | PHE | B | 93  | 5.882  | 15.878 | 16.282 | 1.00 | 13.05 | C |
| ATOM | 2469 | CG  | PHE | B | 93  | 6.141  | 15.882 | 17.770 | 1.00 | 12.42 | C |
| ATOM | 2470 | CD1 | PHE | B | 93  | 6.908  | 14.905 | 18.383 | 1.00 | 11.64 | C |
| ATOM | 2471 | CD2 | PHE | B | 93  | 5.660  | 16.924 | 18.557 | 1.00 | 13.37 | C |
| ATOM | 2472 | CE1 | PHE | B | 93  | 7.164  | 14.980 | 19.767 | 1.00 | 12.25 | C |
| ATOM | 2473 | CE2 | PHE | B | 93  | 5.907  | 16.977 | 19.908 | 1.00 | 12.48 | C |
| ATOM | 2474 | CZ  | PHE | B | 93  | 6.664  | 16.027 | 20.504 | 1.00 | 11.59 | C |
| ATOM | 2475 | N   | LEU | B | 94  | 3.966  | 13.065 | 17.075 | 1.00 | 12.82 | N |
| ATOM | 2476 | CA  | LEU | B | 94  | 2.881  | 12.574 | 17.897 | 1.00 | 14.39 | C |
| ATOM | 2477 | C   | LEU | B | 94  | 3.274  | 12.908 | 19.325 | 1.00 | 14.15 | C |
| ATOM | 2478 | O   | LEU | B | 94  | 4.097  | 12.212 | 19.933 | 1.00 | 14.94 | O |
| ATOM | 2479 | CB  | LEU | B | 94  | 2.711  | 11.059 | 17.739 | 1.00 | 14.29 | C |
| ATOM | 2480 | CG  | LEU | B | 94  | 1.978  | 10.573 | 16.485 | 1.00 | 14.83 | C |
| ATOM | 2481 | CD1 | LEU | B | 94  | 2.707  | 10.982 | 15.220 | 1.00 | 16.60 | C |
| ATOM | 2482 | CD2 | LEU | B | 94  | 1.839  | 9.046  | 16.563 | 1.00 | 15.82 | C |
| ATOM | 2483 | N   | PRO | B | 95  | 2.726  | 13.994 | 19.855 | 1.00 | 14.66 | N |
| ATOM | 2484 | CA  | PRO | B | 95  | 3.221  | 14.420 | 21.144 | 1.00 | 14.62 | C |
| ATOM | 2485 | C   | PRO | B | 95  | 2.947  | 13.358 | 22.201 | 1.00 | 14.17 | C |
| ATOM | 2486 | O   | PRO | B | 95  | 1.862  | 12.791 | 22.179 | 1.00 | 13.85 | O |
| ATOM | 2487 | CB  | PRO | B | 95  | 2.444  | 15.711 | 21.402 | 1.00 | 15.33 | C |
| ATOM | 2488 | CG  | PRO | B | 95  | 1.879  | 16.101 | 20.059 | 1.00 | 16.53 | C |
| ATOM | 2489 | CD  | PRO | B | 95  | 1.647  | 14.862 | 19.347 | 1.00 | 15.17 | C |
| ATOM | 2490 | N   | MET | B | 96  | 3.906  | 13.027 | 23.074 | 1.00 | 13.67 | N |
| ATOM | 2491 | CA  | MET | B | 96  | 5.272  | 13.552 | 23.065 | 1.00 | 13.78 | C |
| ATOM | 2492 | C   | MET | B | 96  | 6.290  | 12.527 | 22.551 | 1.00 | 14.36 | C |
| ATOM | 2493 | O   | MET | B | 96  | 7.479  | 12.773 | 22.579 | 1.00 | 12.85 | O |
| ATOM | 2494 | CB  | MET | B | 96  | 5.666  | 13.992 | 24.470 | 1.00 | 13.78 | C |
| ATOM | 2495 | CG  | MET | B | 96  | 4.804  | 15.089 | 25.073 | 1.00 | 15.00 | C |
| ATOM | 2496 | SD  | MET | B | 96  | 4.946  | 16.677 | 24.248 | 1.00 | 16.71 | S |
| ATOM | 2497 | CE  | MET | B | 96  | 6.604  | 17.157 | 24.691 | 1.00 | 17.08 | C |
| ATOM | 2498 | N   | ASP | B | 101 | 5.822  | 11.379 | 22.076 | 1.00 | 14.99 | N |
| ATOM | 2499 | CA  | ASP | B | 101 | 6.683  | 10.207 | 22.028 | 1.00 | 17.17 | C |
| ATOM | 2500 | C   | ASP | B | 101 | 7.318  | 9.883  | 20.694 | 1.00 | 16.86 | C |
| ATOM | 2501 | O   | ASP | B | 101 | 8.356  | 9.219  | 20.681 | 1.00 | 15.31 | O |
| ATOM | 2502 | CB  | ASP | B | 101 | 5.929  | 8.996  | 22.566 | 1.00 | 18.35 | C |
| ATOM | 2503 | CG  | ASP | B | 101 | 5.531  | 9.177  | 24.007 | 1.00 | 22.80 | C |
| ATOM | 2504 | OD1 | ASP | B | 101 | 6.322  | 9.763  | 24.771 | 1.00 | 28.43 | O |
| ATOM | 2505 | OD2 | ASP | B | 101 | 4.427  | 8.780  | 24.388 | 1.00 | 29.33 | O |
| ATOM | 2506 | N   | TYR | B | 102 | 6.723  | 10.325 | 19.583 | 1.00 | 15.66 | N |
| ATOM | 2507 | CA  | TYR | B | 102 | 7.165  | 9.820  | 18.263 | 1.00 | 17.66 | C |
| ATOM | 2508 | C   | TYR | B | 102 | 7.376  | 10.911 | 17.231 | 1.00 | 16.01 | C |
| ATOM | 2509 | O   | TYR | B | 102 | 6.580  | 11.822 | 17.152 | 1.00 | 17.59 | O |
| ATOM | 2510 | CB  | TYR | B | 102 | 6.180  | 8.784  | 17.690 | 1.00 | 18.54 | C |
| ATOM | 2511 | CG  | TYR | B | 102 | 5.759  | 7.703  | 18.666 | 1.00 | 19.95 | C |
| ATOM | 2512 | CD1 | TYR | B | 102 | 6.620  | 6.662  | 18.974 | 1.00 | 22.17 | C |
| ATOM | 2513 | CD2 | TYR | B | 102 | 4.506  | 7.713  | 19.259 | 1.00 | 20.94 | C |
| ATOM | 2514 | CE1 | TYR | B | 102 | 6.265  | 5.674  | 19.871 | 1.00 | 21.50 | C |
| ATOM | 2515 | CE2 | TYR | B | 102 | 4.139  | 6.716  | 20.160 | 1.00 | 22.53 | C |
| ATOM | 2516 | CZ  | TYR | B | 102 | 5.034  | 5.697  | 20.455 | 1.00 | 21.92 | C |
| ATOM | 2517 | OH  | TYR | B | 102 | 4.716  | 4.684  | 21.358 | 1.00 | 21.60 | O |
| ATOM | 2518 | N   | TRP | B | 103 | 8.432  | 10.748 | 16.422 | 1.00 | 15.67 | N |
| ATOM | 2519 | CA  | TRP | B | 103 | 8.764  | 11.637 | 15.317 | 1.00 | 15.60 | C |
| ATOM | 2520 | C   | TRP | B | 103 | 8.872  | 10.829 | 14.039 | 1.00 | 16.55 | C |
| ATOM | 2521 | O   | TRP | B | 103 | 9.357  | 9.683  | 14.046 | 1.00 | 16.24 | O |
| ATOM | 2522 | CB  | TRP | B | 103 | 10.123 | 12.303 | 15.536 | 1.00 | 14.93 | C |
| ATOM | 2523 | CG  | TRP | B | 103 | 10.208 | 13.245 | 16.654 | 1.00 | 14.97 | C |

| ATOM | 2524 | CD1 | TRP | B | 103 | 10.336 | 12.938 | 17.974 | 1.00 | 15.38 | C |
|------|------|-----|-----|---|-----|--------|--------|--------|------|-------|---|
| ATOM | 2525 | CD2 | TRP | B | 103 | 10.193 | 14.675 | 16.575 | 1.00 | 14.40 | C |
| ATOM | 2526 | NE1 | TRP | B | 103 | 10.393 | 14.075 | 18.727 | 1.00 | 15.24 | N |
| ATOM | 2527 | CE2 | TRP | B | 103 | 10.311 | 15.162 | 17.895 | 1.00 | 15.99 | C |
| ATOM | 2528 | CE3 | TRP | B | 103 | 10.124 | 15.524 | 15.524 | 1.00 | 16.10 | C |
| ATOM | 2529 | CZ2 | TRP | B | 103 | 10.356 | 16.510 | 18.190 | 1.00 | 14.83 | C |
| ATOM | 2530 | CZ3 | TRP | B | 103 | 10.143 | 16.941 | 15.816 | 1.00 | 15.77 | C |
| ATOM | 2531 | CH2 | TRP | B | 103 | 10.262 | 17.395 | 17.134 | 1.00 | 15.69 | C |
| ATOM | 2532 | N | GLY | B | 104 | 8.476 | 11.445 | 12.932 | 1.00 | 17.12 | N |
| ATOM | 2533 | CA | GLY | B | 104 | 8.712 | 10.876 | 11.614 | 1.00 | 17.48 | C |
| ATOM | 2534 | C | GLY | B | 104 | 10.111 | 11.148 | 11.102 | 1.00 | 17.60 | C |
| ATOM | 2535 | O | GLY | B | 104 | 10.994 | 11.518 | 11.866 | 1.00 | 17.24 | O |
| ATOM | 2536 | N | GLN | B | 105 | 10.305 | 10.926 | 9.799 | 1.00 | 18.56 | N |
| ATOM | 2537 | CA | GLN | B | 105 | 11.611 | 11.051 | 9.142 | 1.00 | 20.63 | C |
| ATOM | 2538 | C | GLN | B | 105 | 11.817 | 12.446 | 8.562 | 1.00 | 17.88 | C |
| ATOM | 2539 | O | GLN | B | 105 | 12.931 | 12.809 | 8.232 | 1.00 | 15.90 | O |
| ATOM | 2540 | CB | GLN | B | 105 | 11.731 | 10.029 | 8.001 | 1.00 | 22.76 | C |
| ATOM | 2541 | CG | GLN | B | 105 | 11.078 | 10.506 | 6.689 | 1.00 | 28.30 | C |
| ATOM | 2542 | CD | GLN | B | 105 | 10.416 | 9.440 | 5.830 | 1.00 | 29.38 | C |
| ATOM | 2543 | OE1 | GLN | B | 105 | 9.589 | 9.777 | 4.958 | 1.00 | 33.67 | O |
| ATOM | 2544 | NE2 | GLN | B | 105 | 10.745 | 8.156 | 6.066 | 1.00 | 35.21 | N |
| ATOM | 2545 | N | GLY | B | 106 | 10.734 | 13.207 | 8.439 | 1.00 | 16.87 | N |
| ATOM | 2546 | CA | GLY | B | 106 | 10.772 | 14.540 | 7.850 | 1.00 | 17.18 | C |
| ATOM | 2547 | C | GLY | B | 106 | 10.473 | 14.541 | 6.357 | 1.00 | 16.49 | C |
| ATOM | 2548 | O | GLY | B | 106 | 10.871 | 13.630 | 5.625 | 1.00 | 16.06 | O |
| ATOM | 2549 | N | THR | B | 107 | 9.774 | 15.562 | 5.872 | 1.00 | 14.60 | N |
| ATOM | 2550 | CA | THR | B | 107 | 9.762 | 15.808 | 4.434 | 1.00 | 14.61 | C |
| ATOM | 2551 | C | THR | B | 107 | 10.192 | 17.243 | 4.194 | 1.00 | 15.42 | C |
| ATOM | 2552 | O | THR | B | 107 | 9.827 | 18.114 | 4.943 | 1.00 | 15.74 | O |
| ATOM | 2553 | CB | THR | B | 107 | 8.399 | 15.480 | 3.773 | 1.00 | 16.18 | C |
| ATOM | 2554 | OG1 | THR | B | 107 | 8.538 | 15.523 | 2.330 | 1.00 | 17.98 | O |
| ATOM | 2555 | CG2 | THR | B | 107 | 7.274 | 16.404 | 4.260 | 1.00 | 16.20 | C |
| ATOM | 2556 | N | SER | B | 108 | 11.008 | 17.461 | 3.171 | 1.00 | 15.67 | N |
| ATOM | 2557 | CA | SER | B | 108 | 11.595 | 18.761 | 2.927 | 1.00 | 15.19 | C |
| ATOM | 2558 | C | SER | B | 108 | 10.671 | 19.594 | 2.045 | 1.00 | 15.47 | C |
| ATOM | 2559 | O | SER | B | 108 | 10.193 | 19.130 | 1.008 | 1.00 | 15.86 | O |
| ATOM | 2560 | CB | SER | B | 108 | 12.985 | 18.615 | 2.297 | 1.00 | 16.04 | C |
| ATOM | 2561 | OG | SER | B | 108 | 13.571 | 19.890 | 2.090 | 1.00 | 18.68 | O |
| ATOM | 2562 | N | VAL | B | 109 | 10.436 | 20.837 | 2.466 | 1.00 | 13.73 | N |
| ATOM | 2563 | CA | VAL | B | 109 | 9.629 | 21.780 | 1.714 | 1.00 | 13.82 | C |
| ATOM | 2564 | C | VAL | B | 109 | 10.515 | 22.999 | 1.440 | 1.00 | 14.17 | C |
| ATOM | 2565 | O | VAL | B | 109 | 11.096 | 23.560 | 2.352 | 1.00 | 13.83 | O |
| ATOM | 2566 | CB | VAL | B | 109 | 8.374 | 22.196 | 2.544 | 1.00 | 15.10 | C |
| ATOM | 2567 | CG1 | VAL | B | 109 | 7.621 | 23.357 | 1.871 | 1.00 | 16.46 | C |
| ATOM | 2568 | CG2 | VAL | B | 109 | 7.458 | 21.034 | 2.796 | 1.00 | 15.45 | C |
| ATOM | 2569 | N | THR | B | 110 | 10.684 | 23.352 | 0.164 | 1.00 | 14.42 | N |
| ATOM | 2570 | CA | THR | B | 110 | 11.457 | 24.525 | -0.215 | 1.00 | 14.27 | C |
| ATOM | 2571 | C | THR | B | 110 | 10.519 | 25.503 | -0.885 | 1.00 | 14.58 | C |
| ATOM | 2572 | O | THR | B | 110 | 9.772 | 25.136 | -1.807 | 1.00 | 14.95 | O |
| ATOM | 2573 | CB | THR | B | 110 | 12.618 | 24.162 | -1.183 | 1.00 | 14.16 | C |
| ATOM | 2574 | OG1 | THR | B | 110 | 13.547 | 23.313 | -0.522 | 1.00 | 14.31 | O |
| ATOM | 2575 | CG2 | THR | B | 110 | 13.358 | 25.418 | -1.605 | 1.00 | 16.01 | C |
| ATOM | 2576 | N | VAL | B | 111 | 10.524 | 26.751 | -0.421 | 1.00 | 16.93 | N |
| ATOM | 2577 | CA | VAL | B | 111 | 9.701 | 27.808 | -1.024 | 1.00 | 16.70 | C |
| ATOM | 2578 | C | VAL | B | 111 | 10.655 | 28.802 | -1.665 | 1.00 | 18.21 | C |
| ATOM | 2579 | O | VAL | B | 111 | 11.503 | 29.391 | -0.989 | 1.00 | 17.40 | O |
| ATOM | 2580 | CB | VAL | B | 111 | 8.760 | 28.496 | 0.008 | 1.00 | 18.59 | C |
| ATOM | 2581 | CG1 | VAL | B | 111 | 7.899 | 29.484 | -0.687 | 1.00 | 17.22 | C |
| ATOM | 2582 | CG2 | VAL | B | 111 | 7.879 | 27.410 | 0.726 | 1.00 | 19.13 | C |
| ATOM | 2583 | N | SER | B | 112 | 10.579 | 28.905 | -2.988 | 1.00 | 18.98 | N |
| ATOM | 2584 | CA | SER | B | 112 | 11.627 | 29.590 | -3.738 | 1.00 | 21.94 | C |
| ATOM | 2585 | C | SER | B | 112 | 11.188 | 29.899 | -5.136 | 1.00 | 22.54 | C |
| ATOM | 2586 | O | SER | B | 112 | 10.390 | 29.164 | -5.726 | 1.00 | 21.39 | O |
| ATOM | 2587 | CB | SER | B | 112 | 12.918 | 28.743 | -3.776 | 1.00 | 21.79 | C |
| ATOM | 2588 | OG | SER | B | 112 | 13.953 | 29.401 | -4.491 | 1.00 | 24.03 | O |

| ATOM | 2589 | N   | SER | B | 113 | 11.691 | 31.014 | -5.665  | 1.00 | 24.28 | N |
| ATOM | 2590 | CA  | SER | B | 113 | 11.446 | 31.359 | -7.054  | 1.00 | 25.87 | C |
| ATOM | 2591 | C   | SER | B | 113 | 12.447 | 30.670 | -8.006  | 1.00 | 25.43 | C |
| ATOM | 2592 | O   | SER | B | 113 | 12.241 | 30.685 | -9.216  | 1.00 | 27.11 | O |
| ATOM | 2593 | CB  | SER | B | 113 | 11.471 | 32.889 | -7.249  | 1.00 | 27.04 | C |
| ATOM | 2594 | OG  | SER | B | 113 | 12.717 | 33.417 | -6.856  | 1.00 | 31.31 | O |
| ATOM | 2595 | N   | ALA | B | 114 | 13.513 | 30.079 | -7.472  | 1.00 | 24.51 | N |
| ATOM | 2596 | CA  | ALA | B | 114 | 14.491 | 29.357 | -8.294  | 1.00 | 25.04 | C |
| ATOM | 2597 | C   | ALA | B | 114 | 13.873 | 28.083 | -8.852  | 1.00 | 24.89 | C |
| ATOM | 2598 | O   | ALA | B | 114 | 12.909 | 27.560 | -8.307  | 1.00 | 25.82 | O |
| ATOM | 2599 | CB  | ALA | B | 114 | 15.738 | 29.055 | -7.502  | 1.00 | 25.30 | C |
| ATOM | 2600 | N   | LYS | B | 115 | 14.400 | 27.592 | -9.968  | 1.00 | 24.66 | N |
| ATOM | 2601 | CA  | LYS | B | 115 | 13.781 | 26.457 | -10.618 | 1.00 | 24.92 | C |
| ATOM | 2602 | C   | LYS | B | 115 | 14.470 | 25.176 | -10.184 | 1.00 | 20.74 | C |
| ATOM | 2603 | O   | LYS | B | 115 | 15.660 | 25.166 | -9.884  | 1.00 | 18.91 | O |
| ATOM | 2604 | CB  | LYS | B | 115 | 13.853 | 26.608 | -12.134 | 1.00 | 27.45 | C |
| ATOM | 2605 | CG  | LYS | B | 115 | 12.944 | 27.724 | -12.686 | 1.00 | 31.82 | C |
| ATOM | 2606 | CD  | LYS | B | 115 | 11.465 | 27.390 | -12.490 | 1.00 | 34.11 | C |
| ATOM | 2607 | CE  | LYS | B | 115 | 10.598 | 27.965 | -13.613 | 1.00 | 36.07 | C |
| ATOM | 2608 | NZ  | LYS | B | 115 | 9.151  | 27.767 | -13.315 | 1.00 | 36.37 | N |
| ATOM | 2609 | N   | THR | B | 116 | 13.717 | 24.091 | -10.170 | 1.00 | 18.81 | N |
| ATOM | 2610 | CA  | THR | B | 116 | 14.307 | 22.793 | -9.923  | 1.00 | 18.46 | C |
| ATOM | 2611 | C   | THR | B | 116 | 15.313 | 22.464 | -11.024 | 1.00 | 17.99 | C |
| ATOM | 2612 | O   | THR | B | 116 | 15.056 | 22.690 | -12.217 | 1.00 | 16.82 | O |
| ATOM | 2613 | CB  | THR | B | 116 | 13.225 | 21.706 | -9.811  | 1.00 | 19.40 | C |
| ATOM | 2614 | OG1 | THR | B | 116 | 12.448 | 21.964 | -8.637  | 1.00 | 21.06 | O |
| ATOM | 2615 | CG2 | THR | B | 116 | 13.845 | 20.319 | -9.704  | 1.00 | 19.37 | C |
| ATOM | 2616 | N   | THR | B | 117 | 16.477 | 21.971 | -10.607 | 1.00 | 18.10 | N |
| ATOM | 2617 | CA  | THR | B | 117 | 17.552 | 21.578 | -11.523 | 1.00 | 17.30 | C |
| ATOM | 2618 | C   | THR | B | 117 | 18.140 | 20.255 | -11.024 | 1.00 | 17.03 | C |
| ATOM | 2619 | O   | THR | B | 117 | 18.467 | 20.134 | -9.857  | 1.00 | 15.10 | O |
| ATOM | 2620 | CB  | THR | B | 117 | 18.687 | 22.622 | -11.536 | 1.00 | 17.77 | C |
| ATOM | 2621 | OG1 | THR | B | 117 | 18.172 | 23.924 | -11.851 | 1.00 | 18.93 | O |
| ATOM | 2622 | CG2 | THR | B | 117 | 19.701 | 22.277 | -12.557 | 1.00 | 19.19 | C |
| ATOM | 2623 | N   | PRO | B | 118 | 18.268 | 19.267 | -11.910 | 1.00 | 17.58 | N |
| ATOM | 2624 | CA  | PRO | B | 118 | 18.840 | 17.975 | -11.494 | 1.00 | 18.53 | C |
| ATOM | 2625 | C   | PRO | B | 118 | 20.355 | 18.005 | -11.340 | 1.00 | 16.34 | C |
| ATOM | 2626 | O   | PRO | B | 118 | 21.039 | 18.790 | -12.019 | 1.00 | 17.37 | O |
| ATOM | 2627 | CB  | PRO | B | 118 | 18.431 | 17.025 | -12.629 | 1.00 | 18.84 | C |
| ATOM | 2628 | CG  | PRO | B | 118 | 18.234 | 17.876 | -13.788 | 1.00 | 19.34 | C |
| ATOM | 2629 | CD  | PRO | B | 118 | 17.869 | 19.264 | -13.320 | 1.00 | 18.71 | C |
| ATOM | 2630 | N   | PRO | B | 119 | 20.889 | 17.144 | -10.468 | 1.00 | 15.58 | N |
| ATOM | 2631 | CA  | PRO | B | 119 | 22.333 | 17.089 | -10.279 | 1.00 | 16.23 | C |
| ATOM | 2632 | C   | PRO | B | 119 | 23.089 | 16.467 | -11.462 | 1.00 | 15.34 | C |
| ATOM | 2633 | O   | PRO | B | 119 | 22.543 | 15.633 | -12.213 | 1.00 | 14.96 | O |
| ATOM | 2634 | CB  | PRO | B | 119 | 22.477 | 16.213 | -9.027  | 1.00 | 17.24 | C |
| ATOM | 2635 | CG  | PRO | B | 119 | 21.299 | 15.296 | -9.090  | 1.00 | 18.13 | C |
| ATOM | 2636 | CD  | PRO | B | 119 | 20.190 | 16.205 | -9.571  | 1.00 | 17.01 | C |
| ATOM | 2637 | N   | SER | B | 120 | 24.339 | 16.880 | -11.599 | 1.00 | 14.48 | N |
| ATOM | 2638 | CA  | SER | B | 120 | 25.325 | 16.247 | -12.437 | 1.00 | 13.49 | C |
| ATOM | 2639 | C   | SER | B | 120 | 26.215 | 15.440 | -11.472 | 1.00 | 14.22 | C |
| ATOM | 2640 | O   | SER | B | 120 | 26.703 | 15.953 | -10.488 | 1.00 | 13.98 | O |
| ATOM | 2641 | CB  | SER | B | 120 | 26.103 | 17.301 | -13.228 | 1.00 | 13.99 | C |
| ATOM | 2642 | OG  | SER | B | 120 | 25.244 | 17.923 | -14.199 | 1.00 | 14.80 | O |
| ATOM | 2643 | N   | VAL | B | 121 | 26.370 | 14.151 | -11.733 | 1.00 | 12.48 | N |
| ATOM | 2644 | CA  | VAL | B | 121 | 27.145 | 13.278 | -10.836 | 1.00 | 12.95 | C |
| ATOM | 2645 | C   | VAL | B | 121 | 28.444 | 12.858 | -11.516 | 1.00 | 12.25 | C |
| ATOM | 2646 | O   | VAL | B | 121 | 28.418 | 12.228 | -12.593 | 1.00 | 12.09 | O |
| ATOM | 2647 | CB  | VAL | B | 121 | 26.345 | 11.991 | -10.490 | 1.00 | 14.05 | C |
| ATOM | 2648 | CG1 | VAL | B | 121 | 27.111 | 11.137 | -9.517  | 1.00 | 14.39 | C |
| ATOM | 2649 | CG2 | VAL | B | 121 | 24.985 | 12.361 | -9.928  | 1.00 | 15.20 | C |
| ATOM | 2650 | N   | TYR | B | 122 | 29.570 | 13.172 | -10.872 | 1.00 | 12.50 | N |
| ATOM | 2651 | CA  | TYR | B | 122 | 30.876 | 12.919 | -11.417 | 1.00 | 12.62 | C |
| ATOM | 2652 | C   | TYR | B | 122 | 31.667 | 12.003 | -10.505 | 1.00 | 12.52 | C |
| ATOM | 2653 | O   | TYR | B | 122 | 31.703 | 12.220 | -9.295  | 1.00 | 11.28 | O |

| ATOM | 2654 | CB  | TYR | B | 122 | 31.667 | 14.225 | -11.547 | 1.00 | 13.21 | C |
| ATOM | 2655 | CG  | TYR | B | 122 | 30.964 | 15.271 | -12.407 | 1.00 | 11.86 | C |
| ATOM | 2656 | CD1 | TYR | B | 122 | 30.549 | 14.983 | -13.699 | 1.00 | 13.14 | C |
| ATOM | 2657 | CD2 | TYR | B | 122 | 30.746 | 16.546 | -11.925 | 1.00 | 15.32 | C |
| ATOM | 2658 | CE1 | TYR | B | 122 | 29.888 | 15.977 | -14.495 | 1.00 | 11.85 | C |
| ATOM | 2659 | CE2 | TYR | B | 122 | 30.108 | 17.515 | -12.704 | 1.00 | 12.71 | C |
| ATOM | 2660 | CZ  | TYR | B | 122 | 29.697 | 17.225 | -13.980 | 1.00 | 14.03 | C |
| ATOM | 2661 | OH  | TYR | B | 122 | 29.069 | 18.198 | -14.743 | 1.00 | 13.28 | O |
| ATOM | 2662 | N   | PRO | B | 123 | 32.359 | 11.020 | -11.100 | 1.00 | 13.76 | N |
| ATOM | 2663 | CA  | PRO | B | 123 | 33.233 | 10.143 | -10.313 | 1.00 | 14.34 | C |
| ATOM | 2664 | C   | PRO | B | 123 | 34.505 | 10.832 | -9.823  | 1.00 | 13.99 | C |
| ATOM | 2665 | O   | PRO | B | 123 | 35.084 | 11.646 | -10.553 | 1.00 | 16.36 | O |
| ATOM | 2666 | CB  | PRO | B | 123 | 33.610 | 9.064  | -11.321 | 1.00 | 15.30 | C |
| ATOM | 2667 | CG  | PRO | B | 123 | 33.567 | 9.772  | -12.641 | 1.00 | 15.50 | C |
| ATOM | 2668 | CD  | PRO | B | 123 | 32.403 | 10.686 | -12.538 | 1.00 | 13.79 | C |
| ATOM | 2669 | N   | LEU | B | 124 | 34.936 | 10.505 | -8.611  | 1.00 | 14.02 | N |
| ATOM | 2670 | CA  | LEU | B | 124 | 36.201 | 10.961 | -8.057  | 1.00 | 13.80 | C |
| ATOM | 2671 | C   | LEU | B | 124 | 37.049 | 9.713  | -7.874  | 1.00 | 14.98 | C |
| ATOM | 2672 | O   | LEU | B | 124 | 36.839 | 8.937  | -6.955  | 1.00 | 14.48 | O |
| ATOM | 2673 | CB  | LEU | B | 124 | 36.025 | 11.686 | -6.739  | 1.00 | 14.06 | C |
| ATOM | 2674 | CG  | LEU | B | 124 | 35.113 | 12.923 | -6.820  | 1.00 | 14.28 | C |
| ATOM | 2675 | CD1 | LEU | B | 124 | 34.942 | 13.551 | -5.463  | 1.00 | 13.96 | C |
| ATOM | 2676 | CD2 | LEU | B | 124 | 35.647 | 13.886 | -7.827  | 1.00 | 15.53 | C |
| ATOM | 2677 | N   | ALA | B | 125 | 38.018 | 9.568  | -8.758  | 1.00 | 16.43 | N |
| ATOM | 2678 | CA  | ALA | B | 125 | 38.939 | 8.425  | -8.732  | 1.00 | 17.40 | C |
| ATOM | 2679 | C   | ALA | B | 125 | 40.342 | 8.994  | -8.675  | 1.00 | 18.70 | C |
| ATOM | 2680 | O   | ALA | B | 125 | 40.599 | 10.056 | -9.236  | 1.00 | 18.72 | O |
| ATOM | 2681 | CB  | ALA | B | 125 | 38.741 | 7.581  | -9.935  | 1.00 | 16.67 | C |
| ATOM | 2682 | N   | PRO | B | 126 | 41.258 | 8.302  | -7.982  | 1.00 | 21.69 | N |
| ATOM | 2683 | CA  | PRO | B | 126 | 42.632 | 8.759  | -7.813  | 1.00 | 23.03 | C |
| ATOM | 2684 | C   | PRO | B | 126 | 43.309 | 8.971  | -9.151  | 1.00 | 25.11 | C |
| ATOM | 2685 | O   | PRO | B | 126 | 43.049 | 8.213  | -10.091 | 1.00 | 26.11 | O |
| ATOM | 2686 | CB  | PRO | B | 126 | 43.302 | 7.605  | -7.073  | 1.00 | 23.17 | C |
| ATOM | 2687 | CG  | PRO | B | 126 | 42.197 | 6.817  | -6.479  | 1.00 | 22.70 | C |
| ATOM | 2688 | CD  | PRO | B | 126 | 41.010 | 6.999  | -7.334  | 1.00 | 22.15 | C |
| ATOM | 2689 | N   | GLY | B | 127 | 44.164 | 9.991  | -9.246  | 1.00 | 27.02 | N |
| ATOM | 2690 | CA  | GLY | B | 127 | 44.958 | 10.227 | -10.456 | 1.00 | 28.58 | C |
| ATOM | 2691 | C   | GLY | B | 127 | 45.886 | 9.065  | -10.802 | 1.00 | 29.60 | C |
| ATOM | 2692 | O   | GLY | B | 127 | 46.127 | 8.161  | -9.974  | 1.00 | 31.32 | O |
| ATOM | 2693 | N   | SER | B | 136 | 47.317 | 0.424  | 1.398   | 1.00 | 24.13 | N |
| ATOM | 2694 | CA  | SER | B | 136 | 46.389 | -0.439 | 2.113   | 1.00 | 24.63 | C |
| ATOM | 2695 | C   | SER | B | 136 | 44.917 | -0.092 | 1.821   | 1.00 | 23.16 | C |
| ATOM | 2696 | O   | SER | B | 136 | 44.078 | -0.969 | 1.562   | 1.00 | 22.45 | O |
| ATOM | 2697 | CB  | SER | B | 136 | 46.680 | -0.321 | 3.603   | 1.00 | 25.10 | C |
| ATOM | 2698 | OG  | SER | B | 136 | 45.757 | -1.088 | 4.350   | 1.00 | 30.84 | O |
| ATOM | 2699 | N   | MET | B | 137 | 44.626 | 1.204  | 1.858   | 1.00 | 22.75 | N |
| ATOM | 2700 | CA  | MET | B | 137 | 43.282 | 1.721  | 1.685   | 1.00 | 21.05 | C |
| ATOM | 2701 | C   | MET | B | 137 | 43.271 | 2.654  | 0.483   | 1.00 | 20.40 | C |
| ATOM | 2702 | O   | MET | B | 137 | 44.318 | 3.157  | 0.057   | 1.00 | 20.59 | O |
| ATOM | 2703 | CB  | MET | B | 137 | 42.867 | 2.449  | 2.957   | 1.00 | 22.42 | C |
| ATOM | 2704 | CG  | MET | B | 137 | 43.000 | 1.603  | 4.240   | 1.00 | 24.23 | C |
| ATOM | 2705 | SD  | MET | B | 137 | 41.842 | 0.202  | 4.371   | 1.00 | 26.56 | S |
| ATOM | 2706 | CE  | MET | B | 137 | 40.289 | 1.078  | 4.457   | 1.00 | 25.99 | C |
| ATOM | 2707 | N   | VAL | B | 138 | 42.099 | 2.844  | -0.106  | 1.00 | 19.48 | N |
| ATOM | 2708 | CA  | VAL | B | 138 | 41.929 | 3.815  | -1.194  | 1.00 | 18.38 | C |
| ATOM | 2709 | C   | VAL | B | 138 | 40.647 | 4.616  | -0.900  | 1.00 | 16.78 | C |
| ATOM | 2710 | O   | VAL | B | 138 | 39.676 | 4.075  | -0.395  | 1.00 | 16.64 | O |
| ATOM | 2711 | CB  | VAL | B | 138 | 41.941 | 3.127  | -2.597  | 1.00 | 18.17 | C |
| ATOM | 2712 | CG1 | VAL | B | 138 | 40.882 | 2.052  | -2.730  | 1.00 | 19.81 | C |
| ATOM | 2713 | CG2 | VAL | B | 138 | 41.800 | 4.148° | -3.734  | 1.00 | 20.62 | C |
| ATOM | 2714 | N   | THR | B | 139 | 40.674 | 5.911  | -1.199  | 1.00 | 15.08 | N |
| ATOM | 2715 | CA  | THR | B | 139 | 39.544 | 6.802  | -0.956  | 1.00 | 14.39 | C |
| ATOM | 2716 | C   | THR | B | 139 | 39.030 | 7.217  | -2.339  | 1.00 | 12.68 | C |
| ATOM | 2717 | O   | THR | B | 139 | 39.812 | 7.634  | -3.221  | 1.00 | 12.31 | O |
| ATOM | 2718 | CB  | THR | B | 139 | 39.959 | 8.041  | -0.094  | 1.00 | 14.09 | C |

```
ATOM   2719  OG1 THR B 139      40.304    7.617    1.236  1.00 16.63           O
ATOM   2720  CG2 THR B 139      38.858    9.025    0.011  1.00 14.88           C
ATOM   2721  N   LEU B 140      37.728    7.019   -2.547  1.00 12.85           N
ATOM   2722  CA  LEU B 140      37.046    7.374   -3.764  1.00 12.86           C
ATOM   2723  C   LEU B 140      35.961    8.352   -3.376  1.00 11.34           C
ATOM   2724  O   LEU B 140      35.724    8.571   -2.193  1.00 13.07           O
ATOM   2725  CB  LEU B 140      36.344    6.151   -4.365  1.00 13.40           C
ATOM   2726  CG  LEU B 140      37.253    4.932   -4.550  1.00 14.87           C
ATOM   2727  CD1 LEU B 140      36.426    3.739   -5.007  1.00 16.74           C
ATOM   2728  CD2 LEU B 140      38.373    5.224   -5.535  1.00 16.57           C
ATOM   2729  N   GLY B 141      35.260    8.862   -4.377  1.00 12.77           N
ATOM   2730  CA  GLY B 141      34.168    9.770   -4.116  1.00 12.59           C
ATOM   2731  C   GLY B 141      33.258    9.997   -5.299  1.00 11.76           C
ATOM   2732  O   GLY B 141      33.498    9.524   -6.407  1.00 10.24           O
ATOM   2733  N   CYS B 142      32.226   10.798   -5.036  1.00 13.86           N
ATOM   2734  CA  CYS B 142      31.313   11.295   -6.069  1.00 15.62           C
ATOM   2735  C   CYS B 142      31.087   12.776   -5.802  1.00 13.77           C
ATOM   2736  O   CYS B 142      30.948   13.175   -4.657  1.00 13.14           O
ATOM   2737  CB  CYS B 142      29.999   10.526   -6.038  1.00 20.53           C
ATOM   2738  SG  CYS B 142      30.088    9.108   -7.124  1.00 32.75           S
ATOM   2739  N   LEU B 143      31.143   13.567   -6.862  1.00 12.14           N
ATOM   2740  CA  LEU B 143      30.833   14.994   -6.802  1.00 11.77           C
ATOM   2741  C   LEU B 143      29.441   15.173   -7.422  1.00 10.42           C
ATOM   2742  O   LEU B 143      29.193   14.746   -8.557  1.00 11.02           O
ATOM   2743  CB  LEU B 143      31.889   15.751   -7.569  1.00 11.67           C
ATOM   2744  CG  LEU B 143      31.757   17.269   -7.748  1.00 11.26           C
ATOM   2745  CD1 LEU B 143      31.841   17.918   -6.420  1.00 12.16           C
ATOM   2746  CD2 LEU B 143      32.853   17.718   -8.707  1.00 13.49           C
ATOM   2747  N   VAL B 144      28.541   15.786   -6.645  1.00 11.95           N
ATOM   2748  CA  VAL B 144      27.141   15.970   -7.010  1.00 11.17           C
ATOM   2749  C   VAL B 144      26.919   17.479   -7.147  1.00 13.05           C
ATOM   2750  O   VAL B 144      26.897   18.193   -6.163  1.00 12.51           O
ATOM   2751  CB  VAL B 144      26.188   15.362   -5.951  1.00 13.47           C
ATOM   2752  CG1 VAL B 144      24.773   15.458   -6.403  1.00 15.63           C
ATOM   2753  CG2 VAL B 144      26.541   13.861   -5.672  1.00 12.94           C
ATOM   2754  N   LYS B 145      26.798   17.939   -8.382  1.00 12.07           N
ATOM   2755  CA  LYS B 145      26.956   19.354   -8.671  1.00 13.19           C
ATOM   2756  C   LYS B 145      25.764   19.957   -9.379  1.00 12.97           C
ATOM   2757  O   LYS B 145      25.184   19.335  -10.262  1.00 12.79           O
ATOM   2758  CB  LYS B 145      28.206   19.508   -9.535  1.00 13.92           C
ATOM   2759  CG  LYS B 145      28.596   20.911   -9.788  1.00 15.48           C
ATOM   2760  CD  LYS B 145      30.053   21.037  -10.232  1.00 17.85           C
ATOM   2761  CE  LYS B 145      30.380   22.537  -10.377  1.00 20.60           C
ATOM   2762  NZ  LYS B 145      30.336   23.234   -9.060  1.00 23.34           N
ATOM   2763  N   GLY B 146      25.441   21.214   -9.047  1.00 12.54           N
ATOM   2764  CA  GLY B 146      24.537   21.953   -9.910  1.00 13.90           C
ATOM   2765  C   GLY B 146      23.073   21.642   -9.748  1.00 12.66           C
ATOM   2766  O   GLY B 146      22.318   21.761  -10.706  1.00 15.36           O
ATOM   2767  N   TYR B 147      22.644   21.285   -8.545  1.00 12.79           N
ATOM   2768  CA  TYR B 147      21.224   20.961   -8.314  1.00 11.87           C
ATOM   2769  C   TYR B 147      20.486   21.978   -7.433  1.00 12.45           C
ATOM   2770  O   TYR B 147      21.065   22.748   -6.687  1.00 14.29           O
ATOM   2771  CB  TYR B 147      21.046   19.546   -7.738  1.00 12.96           C
ATOM   2772  CG  TYR B 147      21.602   19.362   -6.339  1.00 13.10           C
ATOM   2773  CD1 TYR B 147      22.939   19.042   -6.127  1.00 11.17           C
ATOM   2774  CD2 TYR B 147      20.791   19.477   -5.229  1.00 12.92           C
ATOM   2775  CE1 TYR B 147      23.445   18.868   -4.870  1.00 12.87           C
ATOM   2776  CE2 TYR B 147      21.302   19.325   -3.950  1.00 13.26           C
ATOM   2777  CZ  TYR B 147      22.616   19.019   -3.775  1.00 13.81           C
ATOM   2778  OH  TYR B 147      23.091   18.855   -2.503  1.00 14.99           O
ATOM   2779  N   PHE B 148      19.175   21.948   -7.564  1.00 13.50           N
ATOM   2780  CA  PHE B 148      18.281   22.721   -6.731  1.00 13.67           C
ATOM   2781  C   PHE B 148      16.889   22.093   -6.788  1.00 14.74           C
ATOM   2782  O   PHE B 148      16.494   21.581   -7.835  1.00 14.07           O
ATOM   2783  CB  PHE B 148      18.235   24.170   -7.216  1.00 14.12           C
```

| | | | | | | | | | | |
|------|------|-----|-----|---|-----|--------|--------|--------|------|-------|---|
| ATOM | 2784 | CG | PHE | B | 148 | 17.491 | 25.049 | -6.295 | 1.00 | 13.71 | C |
| ATOM | 2785 | CD1 | PHE | B | 148 | 18.135 | 25.640 | -5.224 | 1.00 | 14.07 | C |
| ATOM | 2786 | CD2 | PHE | B | 148 | 16.129 | 25.219 | -6.440 | 1.00 | 14.71 | C |
| ATOM | 2787 | CE1 | PHE | B | 148 | 17.398 | 26.446 | -4.291 | 1.00 | 14.94 | C |
| ATOM | 2788 | CE2 | PHE | B | 148 | 15.408 | 26.011 | -5.550 | 1.00 | 13.40 | C |
| ATOM | 2789 | CZ | PHE | B | 148 | 16.034 | 26.619 | -4.483 | 1.00 | 14.37 | C |
| ATOM | 2790 | N | PRO | B | 149 | 16.156 | 22.058 | -5.643 | 1.00 | 16.04 | N |
| ATOM | 2791 | CA | PRO | B | 149 | 16.571 | 22.417 | -4.295 | 1.00 | 17.09 | C |
| ATOM | 2792 | C | PRO | B | 149 | 17.252 | 21.205 | -3.625 | 1.00 | 18.59 | C |
| ATOM | 2793 | O | PRO | B | 149 | 17.387 | 20.179 | -4.244 | 1.00 | 18.51 | O |
| ATOM | 2794 | CB | PRO | B | 149 | 15.244 | 22.713 | -3.621 | 1.00 | 17.67 | C |
| ATOM | 2795 | CG | PRO | B | 149 | 14.344 | 21.715 | -4.191 | 1.00 | 16.42 | C |
| ATOM | 2796 | CD | PRO | B | 149 | 14.779 | 21.548 | -5.643 | 1.00 | 17.11 | C |
| ATOM | 2797 | N | GLU | B | 150 | 17.699 | 21.379 | -2.391 | 1.00 | 21.32 | N |
| ATOM | 2798 | CA | GLU | B | 150 | 17.978 | 20.257 | -1.504 | 1.00 | 22.64 | C |
| ATOM | 2799 | C | GLU | B | 150 | 16.688 | 19.478 | -1.264 | 1.00 | 22.96 | C |
| ATOM | 2800 | O | GLU | B | 150 | 15.571 | 20.024 | -1.388 | 1.00 | 23.47 | O |
| ATOM | 2801 | CB | GLU | B | 150 | 18.533 | 20.766 | -0.183 | 1.00 | 23.93 | C |
| ATOM | 2802 | CG | GLU | B | 150 | 19.969 | 21.196 | -0.223 | 1.00 | 25.58 | C |
| ATOM | 2803 | CD | GLU | B | 150 | 20.909 | 20.114 | 0.297 | 1.00 | 27.94 | C |
| ATOM | 2804 | OE1 | GLU | B | 150 | 21.369 | 20.297 | 1.433 | 1.00 | 27.91 | O |
| ATOM | 2805 | OE2 | GLU | B | 150 | 21.181 | 19.099 | -0.413 | 1.00 | 26.16 | O |
| ATOM | 2806 | N | PRO | B | 151 | 16.805 | 18.188 | -0.908 | 1.00 | 23.10 | N |
| ATOM | 2807 | CA | PRO | B | 151 | 18.021 | 17.438 | -0.657 | 1.00 | 21.58 | C |
| ATOM | 2808 | C | PRO | B | 151 | 18.369 | 16.518 | -1.795 | 1.00 | 20.24 | C |
| ATOM | 2809 | O | PRO | B | 151 | 17.586 | 16.354 | -2.744 | 1.00 | 19.01 | O |
| ATOM | 2810 | CB | PRO | B | 151 | 17.611 | 16.600 | 0.561 | 1.00 | 22.45 | C |
| ATOM | 2811 | CG | PRO | B | 151 | 16.174 | 16.255 | 0.287 | 1.00 | 21.93 | C |
| ATOM | 2812 | CD | PRO | B | 151 | 15.630 | 17.327 | -0.671 | 1.00 | 22.30 | C |
| ATOM | 2813 | N | VAL | B | 152 | 19.555 | 15.910 | -1.696 | 1.00 | 20.09 | N |
| ATOM | 2814 | CA | VAL | B | 152 | 19.856 | 14.698 | -2.429 | 1.00 | 20.66 | C |
| ATOM | 2815 | C | VAL | B | 152 | 20.170 | 13.612 | -1.398 | 1.00 | 22.39 | C |
| ATOM | 2816 | O | VAL | B | 152 | 20.487 | 13.923 | -0.268 | 1.00 | 22.99 | O |
| ATOM | 2817 | CB | VAL | B | 152 | 21.055 | 14.842 | -3.390 | 1.00 | 20.56 | C |
| ATOM | 2818 | CG1 | VAL | B | 152 | 20.718 | 15.785 | -4.540 | 1.00 | 20.36 | C |
| ATOM | 2819 | CG2 | VAL | B | 152 | 22.328 | 15.303 | -2.650 | 1.00 | 21.48 | C |
| ATOM | 2820 | N | THR | B | 153 | 20.058 | 12.363 | -1.805 | 1.00 | 23.67 | N |
| ATOM | 2821 | CA | THR | B | 153 | 20.620 | 11.257 | -1.021 | 1.00 | 24.83 | C |
| ATOM | 2822 | C | THR | B | 153 | 21.696 | 10.560 | -1.818 | 1.00 | 21.62 | C |
| ATOM | 2823 | O | THR | B | 153 | 21.549 | 10.320 | -3.019 | 1.00 | 24.14 | O |
| ATOM | 2824 | CB | THR | B | 153 | 19.558 | 10.250 | -0.599 | 1.00 | 25.32 | C |
| ATOM | 2825 | OG1 | THR | B | 153 | 18.871 | 9.757 | -1.750 | 1.00 | 30.70 | O |
| ATOM | 2826 | CG2 | THR | B | 153 | 18.564 | 10.900 | 0.319 | 1.00 | 27.28 | C |
| ATOM | 2827 | N | VAL | B | 154 | 22.808 | 10.264 | -1.155 | 1.00 | 20.22 | N |
| ATOM | 2828 | CA | VAL | B | 154 | 23.899 | 9.579 | -1.801 | 1.00 | 18.91 | C |
| ATOM | 2829 | C | VAL | B | 154 | 24.144 | 8.288 | -1.040 | 1.00 | 18.94 | C |
| ATOM | 2830 | O | VAL | B | 154 | 24.267 | 8.325 | 0.168 | 1.00 | 21.29 | O |
| ATOM | 2831 | CB | VAL | B | 154 | 25.172 | 10.387 | -1.814 | 1.00 | 18.98 | C |
| ATOM | 2832 | CG1 | VAL | B | 154 | 26.299 | 9.595 | -2.552 | 1.00 | 18.57 | C |
| ATOM | 2833 | CG2 | VAL | B | 154 | 24.923 | 11.739 | -2.489 | 1.00 | 20.01 | C |
| ATOM | 2834 | N | THR | B | 155 | 24.156 | 7.186 | -1.760 | 1.00 | 19.32 | N |
| ATOM | 2835 | CA | THR | B | 155 | 24.522 | 5.884 | -1.195 | 1.00 | 18.64 | C |
| ATOM | 2836 | C | THR | B | 155 | 25.659 | 5.283 | -2.005 | 1.00 | 18.23 | C |
| ATOM | 2837 | O | THR | B | 155 | 26.042 | 5.788 | -3.068 | 1.00 | 16.21 | O |
| ATOM | 2838 | CB | THR | B | 155 | 23.292 | 4.909 | -1.061 | 1.00 | 18.68 | C |
| ATOM | 2839 | OG1 | THR | B | 155 | 22.713 | 4.619 | -2.332 | 1.00 | 20.97 | O |
| ATOM | 2840 | CG2 | THR | B | 155 | 22.226 | 5.491 | -0.161 | 1.00 | 20.05 | C |
| ATOM | 2841 | N | TRP | B | 156 | 26.252 | 4.211 | -1.470 | 1.00 | 16.10 | N |
| ATOM | 2842 | CA | TRP | B | 156 | 27.294 | 3.502 | -2.149 | 1.00 | 15.68 | C |
| ATOM | 2843 | C | TRP | B | 156 | 26.885 | 2.044 | -2.240 | 1.00 | 17.16 | C |
| ATOM | 2844 | O | TRP | B | 156 | 26.416 | 1.483 | -1.249 | 1.00 | 16.93 | O |
| ATOM | 2845 | CB | TRP | B | 156 | 28.610 | 3.651 | -1.384 | 1.00 | 15.41 | C |
| ATOM | 2846 | CG | TRP | B | 156 | 29.150 | 5.060 | -1.470 | 1.00 | 14.11 | C |
| ATOM | 2847 | CD1 | TRP | B | 156 | 28.954 | 6.065 | -0.596 | 1.00 | 14.42 | C |
| ATOM | 2848 | CD2 | TRP | B | 156 | 29.952 | 5.580 | -2.518 | 1.00 | 14.75 | C |

```
ATOM   2849  NE1 TRP B 156    29.620   7.210  -1.021  1.00 14.17           N
ATOM   2850  CE2 TRP B 156    30.221   6.935  -2.215  1.00 12.70           C
ATOM   2851  CE3 TRP B 156    30.470   5.038  -3.692  1.00 13.29           C
ATOM   2852  CZ2 TRP B 156    31.005   7.726  -3.025  1.00 13.71           C
ATOM   2853  CZ3 TRP B 156    31.252   5.823  -4.494  1.00 15.66           C
ATOM   2854  CH2 TRP B 156    31.490   7.177  -4.168  1.00 14.38           C
ATOM   2855  N   ASN B 157    27.039   1.477  -3.431  1.00 18.53           N
ATOM   2856  CA  ASN B 157    26.603   0.109  -3.771  1.00 21.26           C
ATOM   2857  C   ASN B 157    25.172  -0.169  -3.272  1.00 22.79           C
ATOM   2858  O   ASN B 157    24.894  -1.203  -2.614  1.00 23.14           O
ATOM   2859  CB  ASN B 157    27.625  -0.927  -3.252  1.00 20.48           C
ATOM   2860  CG  ASN B 157    28.916  -0.949  -4.050  1.00 20.84           C
ATOM   2861  OD1 ASN B 157    29.027  -0.344  -5.123  1.00 21.65           O
ATOM   2862  ND2 ASN B 157    29.922  -1.688  -3.533  1.00 20.57           N
ATOM   2863  N   SER B 158    24.276   0.763  -3.608  1.00 22.76           N
ATOM   2864  CA  SER B 158    22.843   0.746  -3.253  1.00 24.16           C
ATOM   2865  C   SER B 158    22.525   0.718  -1.743  1.00 25.61           C
ATOM   2866  O   SER B 158    21.475   0.185  -1.326  1.00 26.32           O
ATOM   2867  CB  SER B 158    22.139  -0.396  -3.976  1.00 24.84           C
ATOM   2868  OG  SER B 158    22.540  -0.463  -5.338  1.00 26.13           O
ATOM   2869  N   GLY B 159    23.414   1.319  -0.944  1.00 24.36           N
ATOM   2870  CA  GLY B 159    23.275   1.381   0.494  1.00 25.27           C
ATOM   2871  C   GLY B 159    24.001   0.255   1.240  1.00 25.87           C
ATOM   2872  O   GLY B 159    24.065   0.275   2.465  1.00 26.39           O
ATOM   2873  N   SER B 160    24.545  -0.713   0.507  1.00 26.11           N
ATOM   2874  CA  SER B 160    25.245  -1.839   1.111  1.00 27.39           C
ATOM   2875  C   SER B 160    26.571  -1.424   1.719  1.00 26.30           C
ATOM   2876  O   SER B 160    26.917  -1.840   2.834  1.00 25.83           O
ATOM   2877  CB  SER B 160    25.495  -2.929   0.075  1.00 28.39           C
ATOM   2878  OG  SER B 160    24.257  -3.464  -0.331  1.00 32.68           O
ATOM   2879  N   LEU B 161    27.327  -0.631   0.966  1.00 23.62           N
ATOM   2880  CA  LEU B 161    28.604  -0.127   1.421  1.00 22.08           C
ATOM   2881  C   LEU B 161    28.381   1.078   2.343  1.00 21.55           C
ATOM   2882  O   LEU B 161    28.121   2.197   1.877  1.00 19.38           O
ATOM   2883  CB  LEU B 161    29.428   0.261   0.201  1.00 23.16           C
ATOM   2884  CG  LEU B 161    30.924   0.349   0.353  1.00 22.77           C
ATOM   2885  CD1 LEU B 161    31.530   0.685  -1.021  1.00 24.15           C
ATOM   2886  CD2 LEU B 161    31.315   1.366   1.424  1.00 24.57           C
ATOM   2887  N   SER B 162    28.455   0.853   3.650  1.00 20.55           N
ATOM   2888  CA  SER B 162    28.078   1.863   4.612  1.00 21.70           C
ATOM   2889  C   SER B 162    29.198   2.317   5.551  1.00 22.58           C
ATOM   2890  O   SER B 162    29.129   3.420   6.111  1.00 23.79           O
ATOM   2891  CB  SER B 162    26.874   1.376   5.404  1.00 22.86           C
ATOM   2892  OG  SER B 162    27.147   0.133   6.034  1.00 22.80           O
ATOM   2893  N   SER B 163    30.211   1.480   5.750  1.00 21.07           N
ATOM   2894  CA  SER B 163    31.325   1.832   6.613  1.00 22.02           C
ATOM   2895  C   SER B 163    32.328   2.663   5.810  1.00 20.39           C
ATOM   2896  O   SER B 163    32.478   2.479   4.601  1.00 20.63           O
ATOM   2897  CB  SER B 163    31.974   0.560   7.188  1.00 22.93           C
ATOM   2898  OG  SER B 163    33.168   0.835   7.908  1.00 25.52           O
ATOM   2899  N   GLY B 164    32.990   3.592   6.481  1.00 19.56           N
ATOM   2900  CA  GLY B 164    33.974   4.438   5.835  1.00 18.59           C
ATOM   2901  C   GLY B 164    33.395   5.432   4.844  1.00 17.89           C
ATOM   2902  O   GLY B 164    34.098   5.895   3.945  1.00 16.33           O
ATOM   2903  N   VAL B 165    32.132   5.784   5.044  1.00 16.31           N
ATOM   2904  CA  VAL B 165    31.452   6.750   4.179  1.00 16.99           C
ATOM   2905  C   VAL B 165    31.156   8.026   4.967  1.00 17.46           C
ATOM   2906  O   VAL B 165    30.686   7.993   6.111  1.00 16.80           O
ATOM   2907  CB  VAL B 165    30.145   6.172   3.632  1.00 16.31           C
ATOM   2908  CG1 VAL B 165    29.349   7.244   2.830  1.00 15.67           C
ATOM   2909  CG2 VAL B 165    30.427   4.982   2.778  1.00 15.17           C
ATOM   2910  N   HIS B 166    31.391   9.164   4.348  1.00 18.32           N
ATOM   2911  CA  HIS B 166    30.676  10.341   4.824  1.00 18.86           C
ATOM   2912  C   HIS B 166    30.361  11.237   3.642  1.00 17.34           C
ATOM   2913  O   HIS B 166    31.039  11.217   2.632  1.00 14.41           O
```

| ATOM | 2914 | CB | HIS | B | 166 | 31.432 | 11.087 | 5.911 | 1.00 | 19.90 | C |
|------|------|-----|-----|---|-----|--------|--------|--------|------|-------|---|
| ATOM | 2915 | CG | HIS | B | 166 | 32.858 | 11.345 | 5.576 | 1.00 | 21.21 | C |
| ATOM | 2916 | ND1 | HIS | B | 166 | 33.890 | 10.634 | 6.147 | 1.00 | 21.33 | N |
| ATOM | 2917 | CD2 | HIS | B | 166 | 33.432 | 12.259 | 4.763 | 1.00 | 21.52 | C |
| ATOM | 2918 | CE1 | HIS | B | 166 | 35.038 | 11.059 | 5.656 | 1.00 | 20.59 | C |
| ATOM | 2919 | NE2 | HIS | B | 166 | 34.791 | 12.068 | 4.837 | 1.00 | 22.88 | N |
| ATOM | 2920 | N | THR | B | 167 | 29.303 | 12.004 | 3.802 | 1.00 | 18.73 | N |
| ATOM | 2921 | CA | THR | B | 167 | 28.853 | 12.878 | 2.763 | 1.00 | 19.01 | C |
| ATOM | 2922 | C | THR | B | 167 | 28.904 | 14.276 | 3.351 | 1.00 | 17.81 | C |
| ATOM | 2923 | O | THR | B | 167 | 28.474 | 14.511 | 4.475 | 1.00 | 18.53 | O |
| ATOM | 2924 | CB | THR | B | 167 | 27.446 | 12.460 | 2.299 | 1.00 | 19.99 | C |
| ATOM | 2925 | OG1 | THR | B | 167 | 27.489 | 11.131 | 1.756 | 1.00 | 21.38 | O |
| ATOM | 2926 | CG2 | THR | B | 167 | 26.937 | 13.373 | 1.247 | 1.00 | 19.95 | C |
| ATOM | 2927 | N | PHE | B | 168 | 29.461 | 15.197 | 2.597 | 1.00 | 16.04 | N |
| ATOM | 2928 | CA | PHE | B | 168 | 29.671 | 16.557 | 3.059 | 1.00 | 16.58 | C |
| ATOM | 2929 | C | PHE | B | 168 | 28.356 | 17.381 | 2.894 | 1.00 | 16.98 | C |
| ATOM | 2930 | O | PHE | B | 168 | 27.573 | 17.138 | 1.973 | 1.00 | 17.99 | O |
| ATOM | 2931 | CB | PHE | B | 168 | 30.855 | 17.172 | 2.306 | 1.00 | 16.25 | C |
| ATOM | 2932 | CG | PHE | B | 168 | 32.163 | 16.484 | 2.579 | 1.00 | 16.16 | C |
| ATOM | 2933 | CD1 | PHE | B | 168 | 32.645 | 15.492 | 1.738 | 1.00 | 16.22 | C |
| ATOM | 2934 | CD2 | PHE | B | 168 | 32.890 | 16.812 | 3.718 | 1.00 | 16.58 | C |
| ATOM | 2935 | CE1 | PHE | B | 168 | 33.851 | 14.842 | 2.005 | 1.00 | 16.13 | C |
| ATOM | 2936 | CE2 | PHE | B | 168 | 34.087 | 16.169 | 3.994 | 1.00 | 15.39 | C |
| ATOM | 2937 | CZ | PHE | B | 168 | 34.577 | 15.191 | 3.124 | 1.00 | 15.87 | C |
| ATOM | 2938 | N | PRO | B | 169 | 28.069 | 18.286 | 3.844 | 1.00 | 17.39 | N |
| ATOM | 2939 | CA | PRO | B | 169 | 26.925 | 19.178 | 3.653 | 1.00 | 17.54 | C |
| ATOM | 2940 | C | PRO | B | 169 | 27.012 | 19.912 | 2.318 | 1.00 | 17.31 | C |
| ATOM | 2941 | O | PRO | B | 169 | 28.107 | 20.277 | 1.875 | 1.00 | 17.27 | O |
| ATOM | 2942 | CB | PRO | B | 169 | 27.068 | 20.165 | 4.815 | 1.00 | 17.83 | C |
| ATOM | 2943 | CG | PRO | B | 169 | 27.779 | 19.412 | 5.859 | 1.00 | 18.39 | C |
| ATOM | 2944 | CD | PRO | B | 169 | 28.739 | 18.530 | 5.135 | 1.00 | 16.96 | C |
| ATOM | 2945 | N | ALA | B | 170 | 25.868 | 20.097 | 1.669 | 1.00 | 18.01 | N |
| ATOM | 2946 | CA | ALA | B | 170 | 25.822 | 20.861 | 0.415 | 1.00 | 17.87 | C |
| ATOM | 2947 | C | ALA | B | 170 | 26.111 | 22.307 | 0.666 | 1.00 | 18.85 | C |
| ATOM | 2948 | O | ALA | B | 170 | 25.774 | 22.816 | 1.730 | 1.00 | 20.25 | O |
| ATOM | 2949 | CB | ALA | B | 170 | 24.490 | 20.734 | -0.231 | 1.00 | 16.51 | C |
| ATOM | 2950 | N | VAL | B | 171 | 26.734 | 22.950 | -0.312 | 1.00 | 19.61 | N |
| ATOM | 2951 | CA | VAL | B | 171 | 27.007 | 24.385 | -0.290 | 1.00 | 20.66 | C |
| ATOM | 2952 | C | VAL | B | 171 | 26.284 | 25.010 | -1.477 | 1.00 | 20.50 | C |
| ATOM | 2953 | O | VAL | B | 171 | 26.372 | 24.538 | -2.608 | 1.00 | 17.30 | O |
| ATOM | 2954 | CB | VAL | B | 171 | 28.517 | 24.645 | -0.326 | 1.00 | 21.96 | C |
| ATOM | 2955 | CG1 | VAL | B | 171 | 28.848 | 26.122 | -0.454 | 1.00 | 24.52 | C |
| ATOM | 2956 | CG2 | VAL | B | 171 | 29.140 | 24.055 | 0.933 | 1.00 | 23.33 | C |
| ATOM | 2957 | N | LEU | B | 172 | 25.548 | 26.074 | -1.187 | 1.00 | 23.07 | N |
| ATOM | 2958 | CA | LEU | B | 172 | 24.785 | 26.788 | -2.177 | 1.00 | 24.17 | C |
| ATOM | 2959 | C | LEU | B | 172 | 25.658 | 27.864 | -2.746 | 1.00 | 27.57 | C |
| ATOM | 2960 | O | LEU | B | 172 | 26.212 | 28.654 | -1.990 | 1.00 | 27.25 | O |
| ATOM | 2961 | CB | LEU | B | 172 | 23.588 | 27.445 | -1.496 | 1.00 | 24.41 | C |
| ATOM | 2962 | CG | LEU | B | 172 | 22.559 | 28.122 | -2.385 | 1.00 | 23.67 | C |
| ATOM | 2963 | CD1 | LEU | B | 172 | 22.032 | 27.156 | -3.445 | 1.00 | 22.58 | C |
| ATOM | 2964 | CD2 | LEU | B | 172 | 21.435 | 28.668 | -1.499 | 1.00 | 25.06 | C |
| ATOM | 2965 | N | GLN | B | 173 | 25.801 | 27.880 | -4.067 | 1.00 | 31.05 | N |
| ATOM | 2966 | CA | GLN | B | 173 | 26.299 | 29.052 | -4.768 | 1.00 | 34.65 | C |
| ATOM | 2967 | C | GLN | B | 173 | 25.503 | 29.321 | -6.045 | 1.00 | 35.51 | C |
| ATOM | 2968 | O | GLN | B | 173 | 25.313 | 28.427 | -6.863 | 1.00 | 36.54 | O |
| ATOM | 2969 | CB | GLN | B | 173 | 27.765 | 28.873 | -5.113 | 1.00 | 35.58 | C |
| ATOM | 2970 | CG | GLN | B | 173 | 28.448 | 30.181 | -5.463 | 1.00 | 37.57 | C |
| ATOM | 2971 | CD | GLN | B | 173 | 29.907 | 29.997 | -5.763 | 1.00 | 37.53 | C |
| ATOM | 2972 | OE1 | GLN | B | 173 | 30.276 | 29.404 | -6.782 | 1.00 | 41.21 | O |
| ATOM | 2973 | NE2 | GLN | B | 173 | 30.759 | 30.502 | -4.872 | 1.00 | 41.26 | N |
| ATOM | 2974 | N | SER | B | 174 | 25.046 | 30.560 | -6.221 | 1.00 | 36.42 | N |
| ATOM | 2975 | CA | SER | B | 174 | 24.452 | 30.976 | -7.490 | 1.00 | 36.15 | C |
| ATOM | 2976 | C | SER | B | 174 | 23.237 | 30.128 | -7.828 | 1.00 | 34.53 | C |
| ATOM | 2977 | O | SER | B | 174 | 23.109 | 29.615 | -8.958 | 1.00 | 36.02 | O |
| ATOM | 2978 | CB | SER | B | 174 | 25.485 | 30.893 | -8.631 | 1.00 | 37.71 | C |

```
ATOM   2979  OG  SER B 174    26.709  31.520  -8.270  1.00 40.03          O
ATOM   2980  N   ASP B 175    22.365  29.981  -6.833  1.00 31.47          N
ATOM   2981  CA  ASP B 175    21.102  29.219  -6.923  1.00 29.75          C
ATOM   2982  C   ASP B 175    21.283  27.733  -7.137  1.00 23.65          C
ATOM   2983  O   ASP B 175    20.316  27.055  -7.445  1.00 22.96          O
ATOM   2984  CB  ASP B 175    20.179  29.735  -8.043  1.00 32.32          C
ATOM   2985  CG  ASP B 175    19.881  31.225  -7.951  1.00 36.64          C
ATOM   2986  OD1 ASP B 175    20.182  31.863  -6.906  1.00 39.57          O
ATOM   2987  OD2 ASP B 175    19.333  31.741  -8.961  1.00 40.83          O
ATOM   2988  N   LEU B 176    22.507  27.222  -7.015  1.00 20.15          N
ATOM   2989  CA  LEU B 176    22.733  25.793  -7.167  1.00 16.38          C
ATOM   2990  C   LEU B 176    23.620  25.253  -6.051  1.00 15.59          C
ATOM   2991  O   LEU B 176    24.539  25.944  -5.546  1.00 15.62          O
ATOM   2992  CB  LEU B 176    23.365  25.454  -8.516  1.00 15.41          C
ATOM   2993  CG  LEU B 176    22.563  25.834  -9.766  1.00 16.55          C
ATOM   2994  CD1 LEU B 176    23.427  25.571 -10.986  1.00 19.38          C
ATOM   2995  CD2 LEU B 176    21.222  25.123  -9.907  1.00 17.73          C
ATOM   2996  N   TYR B 177    23.324  24.001  -5.706  1.00 14.48          N
ATOM   2997  CA  TYR B 177    24.008  23.288  -4.642  1.00 14.37          C
ATOM   2998  C   TYR B 177    25.026  22.332  -5.211  1.00 14.66          C
ATOM   2999  O   TYR B 177    24.826  21.761  -6.286  1.00 13.91          O
ATOM   3000  CB  TYR B 177    23.011  22.480  -3.818  1.00 16.08          C
ATOM   3001  CG  TYR B 177    22.137  23.284  -2.926  1.00 17.42          C
ATOM   3002  CD1 TYR B 177    22.597  23.708  -1.693  1.00 19.14          C
ATOM   3003  CD2 TYR B 177    20.827  23.570  -3.283  1.00 18.55          C
ATOM   3004  CE1 TYR B 177    21.799  24.412  -0.837  1.00 20.87          C
ATOM   3005  CE2 TYR B 177    20.016  24.298  -2.437  1.00 20.36          C
ATOM   3006  CZ  TYR B 177    20.508  24.719  -1.218  1.00 19.48          C
ATOM   3007  OH  TYR B 177    19.705  25.445  -0.373  1.00 22.01          O
ATOM   3008  N   THR B 178    26.109  22.163  -4.472  1.00 15.18          N
ATOM   3009  CA  THR B 178    27.086  21.142  -4.778  1.00 16.21          C
ATOM   3010  C   THR B 178    27.452  20.440  -3.475  1.00 15.76          C
ATOM   3011  O   THR B 178    27.546  21.053  -2.419  1.00 13.02          O
ATOM   3012  CB  THR B 178    28.333  21.725  -5.446  1.00 17.75          C
ATOM   3013  OG1 THR B 178    27.972  22.422  -6.659  1.00 19.48          O
ATOM   3014  CG2 THR B 178    29.318  20.635  -5.809  1.00 17.35          C
ATOM   3015  N   LEU B 179    27.635  19.129  -3.566  1.00 15.63          N
ATOM   3016  CA  LEU B 179    28.202  18.376  -2.469  1.00 17.04          C
ATOM   3017  C   LEU B 179    29.048  17.233  -3.001  1.00 15.08          C
ATOM   3018  O   LEU B 179    29.042  16.941  -4.179  1.00 13.31          O
ATOM   3019  CB  LEU B 179    27.122  17.862  -1.514  1.00 18.32          C
ATOM   3020  CG  LEU B 179    26.121  16.790  -1.874  1.00 19.43          C
ATOM   3021  CD1 LEU B 179    26.768  15.382  -2.114  1.00 18.24          C
ATOM   3022  CD2 LEU B 179    25.117  16.692  -0.756  1.00 20.09          C
ATOM   3023  N   SER B 180    29.767  16.580  -2.097  1.00 16.08          N
ATOM   3024  CA  SER B 180    30.490  15.391  -2.445  1.00 15.69          C
ATOM   3025  C   SER B 180    30.361  14.369  -1.333  1.00 14.39          C
ATOM   3026  O   SER B 180    30.042  14.683  -0.190  1.00 12.83          O
ATOM   3027  CB  SER B 180    31.947  15.729  -2.681  1.00 15.90          C
ATOM   3028  OG  SER B 180    32.398  16.523  -1.591  1.00 22.35          O
ATOM   3029  N   SER B 181    30.586  13.126  -1.725  1.00 13.63          N
ATOM   3030  CA  SER B 181    30.594  12.016  -0.808  1.00 13.61          C
ATOM   3031  C   SER B 181    31.928  11.259  -0.938  1.00 13.58          C
ATOM   3032  O   SER B 181    32.448  11.073  -2.028  1.00 10.43          O
ATOM   3033  CB  SER B 181    29.413  11.094  -1.092  1.00 13.72          C
ATOM   3034  OG  SER B 181    29.336  10.024  -0.169  1.00 14.74          O
ATOM   3035  N   SER B 182    32.431  10.831   0.218  1.00 13.92          N
ATOM   3036  CA  SER B 182    33.706  10.140   0.367  1.00 14.20          C
ATOM   3037  C   SER B 182    33.492   8.675   0.817  1.00 12.34          C
ATOM   3038  O   SER B 182    32.707   8.401   1.719  1.00 14.18          O
ATOM   3039  CB  SER B 182    34.502  10.882   1.430  1.00 14.19          C
ATOM   3040  OG  SER B 182    35.742  10.289   1.640  1.00 17.36          O
ATOM   3041  N   VAL B 183    34.183   7.758   0.166  1.00 12.69          N
ATOM   3042  CA  VAL B 183    34.226   6.375   0.615  1.00 13.75          C
ATOM   3043 ·C   VAL B 183    35.663   5.849   0.629  1.00 13.71          C
```

| ATOM | 3044 | O   | VAL B 183 | 36.446 | 6.002  | -0.325 | 1.00 12.71 | O |
| ATOM | 3045 | CB  | VAL B 183 | 33.262 | 5.505  | -0.187 | 1.00 13.15 | C |
| ATOM | 3046 | CG1 | VAL B 183 | 33.636 | 5.442  | -1.674 | 1.00 15.64 | C |
| ATOM | 3047 | CG2 | VAL B 183 | 33.169 | 4.076  | 0.417  | 1.00 14.53 | C |
| ATOM | 3048 | N   | THR B 184 | 35.996 | 5.198  | 1.732  | 1.00 15.55 | N |
| ATOM | 3049 | CA  | THR B 184 | 37.323 | 4.634  | 1.914  | 1.00 14.96 | C |
| ATOM | 3050 | C   | THR B 184 | 37.168 | 3.124  | 2.082  | 1.00 15.88 | C |
| ATOM | 3051 | O   | THR B 184 | 36.440 | 2.671  | 2.965  | 1.00 15.30 | O |
| ATOM | 3052 | CB  | THR B 184 | 37.984 | 5.236  | 3.119  | 1.00 16.10 | C |
| ATOM | 3053 | OG1 | THR B 184 | 38.127 | 6.666  | 2.910  | 1.00 16.71 | O |
| ATOM | 3054 | CG2 | THR B 184 | 39.355 | 4.594  | 3.374  | 1.00 16.01 | C |
| ATOM | 3055 | N   | VAL B 185 | 37.838 | 2.383  | 1.213  | 1.00 17.32 | N |
| ATOM | 3056 | CA  | VAL B 185 | 37.743 | 0.914  | 1.183  | 1.00 18.31 | C |
| ATOM | 3057 | C   | VAL B 185 | 39.176 | 0.350  | 1.116  | 1.00 18.91 | C |
| ATOM | 3058 | O   | VAL B 185 | 40.097 | 1.066  | 0.733  | 1.00 18.76 | O |
| ATOM | 3059 | CB  | VAL B 185 | 36.907 | 0.410  | -0.044 | 1.00 18.86 | C |
| ATOM | 3060 | CG1 | VAL B 185 | 35.473 | 0.953  | -0.008 | 1.00 17.31 | C |
| ATOM | 3061 | CG2 | VAL B 185 | 37.557 | 0.758  | -1.368 | 1.00 19.11 | C |
| ATOM | 3062 | N   | PRO B 186 | 39.384 | -0.935 | 1.501  | 1.00 19.19 | N |
| ATOM | 3063 | CA  | PRO B 186 | 40.713 | -1.543 | 1.296  | 1.00 18.56 | C |
| ATOM | 3064 | C   | PRO B 186 | 41.072 | -1.592 | -0.156 | 1.00 17.70 | C |
| ATOM | 3065 | O   | PRO B 186 | 40.197 | -1.818 | -0.974 | 1.00 17.55 | O |
| ATOM | 3066 | CB  | PRO B 186 | 40.521 | -2.988 | 1.804  | 1.00 18.93 | C |
| ATOM | 3067 | CG  | PRO B 186 | 39.392 | -2.885 | 2.772  | 1.00 19.08 | C |
| ATOM | 3068 | CD  | PRO B 186 | 38.453 | -1.879 | 2.135  | 1.00 18.97 | C |
| ATOM | 3069 | N   | SER B 187 | 42.347 | -1.417 | -0.497 | 1.00 18.49 | N |
| ATOM | 3070 | CA  | SER B 187 | 42.755 | -1.445 | -1.902 | 1.00 19.20 | C |
| ATOM | 3071 | C   | SER B 187 | 42.595 | -2.855 | -2.498 | 1.00 19.08 | C |
| ATOM | 3072 | O   | SER B 187 | 42.537 | -3.005 | -3.702 | 1.00 18.67 | O |
| ATOM | 3073 | CB  | SER B 187 | 44.183 | -0.930 | -2.103 | 1.00 21.37 | C |
| ATOM | 3074 | OG  | SER B 187 | 45.137 | -1.679 | -1.356 | 1.00 25.46 | O |
| ATOM | 3075 | N   | SER B 188 | 42.486 | -3.873 | -1.644 | 1.00 18.95 | N |
| ATOM | 3076 | CA  | SER B 188 | 42.139 | -5.223 | -2.105 | 1.00 19.33 | C |
| ATOM | 3077 | C   | SER B 188 | 40.705 | -5.326 | -2.643 | 1.00 20.44 | C |
| ATOM | 3078 | O   | SER B 188 | 40.402 | -6.283 | -3.331 | 1.00 21.54 | O |
| ATOM | 3079 | CB  | SER B 188 | 42.310 | -6.245 | -0.979 | 1.00 18.29 | C |
| ATOM | 3080 | OG  | SER B 188 | 41.639 | -5.862 | 0.199  | 1.00 18.74 | O |
| ATOM | 3081 | N   | THR B 189 | 39.830 | -4.357 | -2.326 | 1.00 19.48 | N |
| ATOM | 3082 | CA  | THR B 189 | 38.431 | -4.429 | -2.737 | 1.00 20.76 | C |
| ATOM | 3083 | C   | THR B 189 | 38.067 | -3.614 | -3.972 | 1.00 20.74 | C |
| ATOM | 3084 | O   | THR B 189 | 37.069 | -3.896 | -4.604 | 1.00 21.80 | O |
| ATOM | 3085 | CB  | THR B 189 | 37.473 | -4.052 | -1.597 | 1.00 22.15 | C |
| ATOM | 3086 | OG1 | THR B 189 | 37.722 | -2.705 | -1.181 | 1.00 25.38 | O |
| ATOM | 3087 | CG2 | THR B 189 | 37.679 | -4.966 | -0.411 | 1.00 23.50 | C |
| ATOM | 3088 | N   | TRP B 190 | 38.853 | -2.581 | -4.279 | 1.00 20.30 | N |
| ATOM | 3089 | CA  | TRP B 190 | 38.649 | -1.768 | -5.463 | 1.00 19.13 | C |
| ATOM | 3090 | C   | TRP B 190 | 39.997 | -1.565 | -6.106 | 1.00 18.84 | C |
| ATOM | 3091 | O   | TRP B 190 | 40.959 | -1.305 | -5.407 | 1.00 18.32 | O |
| ATOM | 3092 | CB  | TRP B 190 | 38.116 | -0.364 | -5.091 | 1.00 17.56 | C |
| ATOM | 3093 | CG  | TRP B 190 | 37.814 | 0.490  | -6.287 | 1.00 16.38 | C |
| ATOM | 3094 | CD1 | TRP B 190 | 36.654 | 0.514  | -6.994 | 1.00 17.16 | C |
| ATOM | 3095 | CD2 | TRP B 190 | 38.693 | 1.417  | -6.930 | 1.00 16.09 | C |
| ATOM | 3096 | NE1 | TRP B 190 | 36.748 | 1.409  | -8.033 | 1.00 17.07 | N |
| ATOM | 3097 | CE2 | TRP B 190 | 37.998 | 1.969  | -8.018 | 1.00 16.35 | C |
| ATOM | 3098 | CE3 | TRP B 190 | 40.001 | 1.853  | -6.676 | 1.00 16.80 | C |
| ATOM | 3099 | CZ2 | TRP B 190 | 38.571 | 2.924  | -8.862 | 1.00 17.41 | C |
| ATOM | 3100 | CZ3 | TRP B 190 | 40.573 | 2.779  | -7.531 | 1.00 15.27 | C |
| ATOM | 3101 | CH2 | TRP B 190 | 39.852 | 3.316  | -8.595 | 1.00 16.48 | C |
| ATOM | 3102 | N   | PRO B 191 | 40.061 | -1.624 | -7.438 | 1.00 19.91 | N |
| ATOM | 3103 | CA  | PRO B 191 | 38.966 | -1.844 | -8.366 | 1.00 21.97 | C |
| ATOM | 3104 | C   | PRO B 191 | 38.619 | -3.307 | -8.704 | 1.00 22.03 | C |
| ATOM | 3105 | O   | PRO B 191 | 37.815 | -3.535 | -9.602 | 1.00 24.09 | O |
| ATOM | 3106 | CB  | PRO B 191 | 39.467 | -1.135 | -9.625 | 1.00 21.81 | C |
| ATOM | 3107 | CG  | PRO B 191 | 40.886 | -1.397 | -9.604 | 1.00 21.07 | C |
| ATOM | 3108 | CD  | PRO B 191 | 41.315 | -1.350 | -8.161 | 1.00 20.09 | C |

```
ATOM   3109  N    SER B 192    39.200   -4.280   -8.009  1.00 23.53          N
ATOM   3110  CA   SER B 192    38.870   -5.700   -8.258  1.00 23.96          C
ATOM   3111  C    SER B 192    37.351   -5.967   -8.201  1.00 24.74          C
ATOM   3112  O    SER B 192    36.822   -6.769   -8.985  1.00 24.78          O
ATOM   3113  CB   SER B 192    39.609   -6.588   -7.255  1.00 23.67          C
ATOM   3114  OG   SER B 192    39.169   -6.338   -5.933  1.00 25.68          O
ATOM   3115  N    GLU B 193    36.650   -5.290   -7.280  1.00 24.91          N
ATOM   3116  CA   GLU B 193    35.189   -5.319   -7.229  1.00 25.06          C
ATOM   3117  C    GLU B 193    34.616   -3.898   -7.373  1.00 25.07          C
ATOM   3118  O    GLU B 193    35.308   -2.903   -7.157  1.00 24.96          O
ATOM   3119  CB   GLU B 193    34.716   -5.964   -5.928  1.00 27.08          C
ATOM   3120  CG   GLU B 193    35.300   -7.366   -5.660  1.00 28.85          C
ATOM   3121  CD   GLU B 193    34.757   -8.464   -6.571  1.00 31.40          C
ATOM   3122  OE1  GLU B 193    35.423   -9.524   -6.690  1.00 35.29          O
ATOM   3123  OE2  GLU B 193    33.680   -8.293   -7.174  1.00 31.36          O
ATOM   3124  N    THR B 194    33.349   -3.800   -7.729  1.00 24.69          N
ATOM   3125  CA   THR B 194    32.806   -2.491   -8.132  1.00 24.82          C
ATOM   3126  C    THR B 194    32.387   -1.622   -6.959  1.00 23.31          C
ATOM   3127  O    THR B 194    31.930   -2.105   -5.912  1.00 22.95          O
ATOM   3128  CB   THR B 194    31.655   -2.644   -9.131  1.00 24.78          C
ATOM   3129  OG1  THR B 194    30.583   -3.326   -8.504  1.00 29.13          O
ATOM   3130  CG2  THR B 194    32.101   -3.429  -10.326  1.00 25.99          C
ATOM   3131  N    VAL B 195    32.573   -0.316   -7.149  1.00 22.19          N
ATOM   3132  CA   VAL B 195    32.131    0.694   -6.205  1.00 20.11          C
ATOM   3133  C    VAL B 195    31.373    1.728   -7.026  1.00 18.57          C
ATOM   3134  O    VAL B 195    31.887    2.263   -8.002  1.00 15.14          O
ATOM   3135  CB   VAL B 195    33.296    1.334   -5.433  1.00 19.48          C
ATOM   3136  CG1  VAL B 195    32.807    2.503   -4.568  1.00 19.92          C
ATOM   3137  CG2  VAL B 195    33.968    0.287   -4.541  1.00 19.00          C
ATOM   3138  N    THR B 196    30.133    1.950   -6.624  1.00 17.66          N
ATOM   3139  CA   THR B 196    29.178    2.710   -7.421  1.00 18.67          C
ATOM   3140  C    THR B 196    28.498    3.687   -6.489  1.00 17.25          C
ATOM   3141  O    THR B 196    28.000    3.303   -5.425  1.00 16.93          O
ATOM   3142  CB   THR B 196    28.158    1.755   -8.108  1.00 18.26          C
ATOM   3143  OG1  THR B 196    28.858    0.954   -9.081  1.00 19.70          O
ATOM   3144  CG2  THR B 196    27.055    2.533   -8.804  1.00 18.91          C
ATOM   3145  N    CYS B 197    28.460    4.971   -6.837  1.00 18.33          N
ATOM   3146  CA   CYS B 197    27.644    5.851   -6.020  1.00 18.92          C
ATOM   3147  C    CYS B 197    26.268    6.051   -6.656  1.00 18.29          C
ATOM   3148  O    CYS B 197    26.125    6.133   -7.880  1.00 17.28          O
ATOM   3149  CB   CYS B 197    28.332    7.167   -5.709  1.00 22.54          C
ATOM   3150  SG   CYS B 197    28.208    8.356   -6.975  1.00 27.41          S
ATOM   3151  N    ASN B 198    25.266    6.083   -5.799  1.00 17.31          N
ATOM   3152  CA   ASN B 198    23.878    6.171   -6.212  1.00 18.02          C
ATOM   3153  C    ASN B 198    23.338    7.469   -5.670  1.00 16.87          C
ATOM   3154  O    ASN B 198    23.371    7.719   -4.471  1.00 16.97          O
ATOM   3155  CB   ASN B 198    23.053    5.002   -5.669  1.00 18.86          C
ATOM   3156  CG   ASN B 198    23.828    3.700   -5.622  1.00 19.04          C
ATOM   3157  OD1  ASN B 198    24.260    3.300   -4.546  1.00 20.10          O
ATOM   3158  ND2  ASN B 198    24.024    3.047   -6.777  1.00 20.89          N
ATOM   3159  N    VAL B 199    22.834    8.314   -6.564  1.00 16.79          N
ATOM   3160  CA   VAL B 199    22.417    9.624   -6.183  1.00 16.53          C
ATOM   3161  C    VAL B 199    20.955    9.781   -6.594  1.00 17.86          C
ATOM   3162  O    VAL B 199    20.591    9.430   -7.724  1.00 20.03          O
ATOM   3163  CB   VAL B 199    23.234   10.691   -6.928  1.00 16.71          C
ATOM   3164  CG1  VAL B 199    22.769   12.055   -6.487  1.00 16.64          C
ATOM   3165  CG2  VAL B 199    24.730   10.499   -6.646  1.00 16.85          C
ATOM   3166  N    ALA B 200    20.149   10.271   -5.669  1.00 20.06          N
ATOM   3167  CA   ALA B 200    18.732   10.524   -5.921  1.00 20.87          C
ATOM   3168  C    ALA B 200    18.387   11.964   -5.598  1.00 20.92          C
ATOM   3169  O    ALA B 200    18.706   12.447   -4.533  1.00 18.90          O
ATOM   3170  CB   ALA B 200    17.886    9.605   -5.104  1.00 22.45          C
ATOM   3171  N    HIS B 201    17.746   12.637   -6.558  1.00 21.34          N
ATOM   3172  CA   HIS B 201    17.217   13.982   -6.358  1.00 21.44          C
ATOM   3173  C    HIS B 201    15.735   13.861   -6.701  1.00 23.63          C
```

| ATOM | 3174 | O   | HIS B 201 | 15.365 | 13.841 | -7.879  | 1.00 | 24.67 | O |
| ATOM | 3175 | CB  | HIS B 201 | 17.885 | 14.966 | -7.295  | 1.00 | 19.93 | C |
| ATOM | 3176 | CG  | HIS B 201 | 17.534 | 16.400 | -7.040  | 1.00 | 16.62 | C |
| ATOM | 3177 | ND1 | HIS B 201 | 17.114 | 17.238 | -8.046  | 1.00 | 19.61 | N |
| ATOM | 3178 | CD2 | HIS B 201 | 17.598 | 17.157 | -5.923  | 1.00 | 18.52 | C |
| ATOM | 3179 | CE1 | HIS B 201 | 16.899 | 18.450 | -7.556  | 1.00 | 15.87 | C |
| ATOM | 3180 | NE2 | HIS B 201 | 17.194 | 18.433 | -6.268  | 1.00 | 14.49 | N |
| ATOM | 3181 | N   | PRO B 202 | 14.905 | 13.742 | -5.677  | 1.00 | 24.25 | N |
| ATOM | 3182 | CA  | PRO B 202 | 13.485 | 13.501 | -5.882  | 1.00 | 26.57 | C |
| ATOM | 3183 | C   | PRO B 202 | 12.790 | 14.658 | -6.610  | 1.00 | 26.57 | C |
| ATOM | 3184 | O   | PRO B 202 | 11.978 | 14.418 | -7.518  | 1.00 | 25.93 | O |
| ATOM | 3185 | CB  | PRO B 202 | 12.952 | 13.348 | -4.455  | 1.00 | 25.97 | C |
| ATOM | 3186 | CG  | PRO B 202 | 13.930 | 14.038 | -3.594  | 1.00 | 26.79 | C |
| ATOM | 3187 | CD  | PRO B 202 | 15.249 | 13.844 | -4.248  | 1.00 | 25.63 | C |
| ATOM | 3188 | N   | ALA B 203 | 13.156 | 15.891 | -6.263  | 1.00 | 27.42 | N |
| ATOM | 3189 | CA  | ALA B 203 | 12.558 | 17.078 | -6.896  | 1.00 | 26.95 | C |
| ATOM | 3190 | C   | ALA B 203 | 12.599 | 17.041 | -8.419  | 1.00 | 27.56 | C |
| ATOM | 3191 | O   | ALA B 203 | 11.669 | 17.516 | -9.056  | 1.00 | 27.77 | O |
| ATOM | 3192 | CB  | ALA B 203 | 13.216 | 18.351 | -6.376  | 1.00 | 27.80 | C |
| ATOM | 3193 | N   | SER B 204 | 13.658 | 16.485 | -9.007  | 1.00 | 26.67 | N |
| ATOM | 3194 | CA  | SER B 204 | 13.813 | 16.438 | -10.466 | 1.00 | 27.15 | C |
| ATOM | 3195 | C   | SER B 204 | 13.474 | 15.040 | -11.012 | 1.00 | 28.68 | C |
| ATOM | 3196 | O   | SER B 204 | 13.570 | 14.787 | -12.231 | 1.00 | 28.80 | O |
| ATOM | 3197 | CB  | SER B 204 | 15.233 | 16.828 | -10.863 | 1.00 | 27.25 | C |
| ATOM | 3198 | OG  | SER B 204 | 16.172 | 15.864 | -10.408 | 1.00 | 25.83 | O |
| ATOM | 3199 | N   | SER B 205 | 13.003 | 14.202 | -10.090 | 1.00 | 28.66 | N |
| ATOM | 3200 | CA  | SER B 205 | 12.896 | 12.749 | -10.197 | 1.00 | 31.81 | C |
| ATOM | 3201 | C   | SER B 205 | 14.024 | 12.060 | -10.972 | 1.00 | 32.79 | C |
| ATOM | 3202 | O   | SER B 205 | 13.857 | 11.409 | -12.005 | 1.00 | 34.43 | O |
| ATOM | 3203 | CB  | SER B 205 | 11.474 | 12.350 | -10.589 | 1.00 | 32.19 | C |
| ATOM | 3204 | OG  | SER B 205 | 10.670 | 12.498 | -9.405  | 1.00 | 34.28 | O |
| ATOM | 3205 | N   | THR B 206 | 15.197 | 12.188 | -10.380 | 1.00 | 33.08 | N |
| ATOM | 3206 | CA  | THR B 206 | 16.414 | 11.719 | -10.974 | 1.00 | 32.36 | C |
| ATOM | 3207 | C   | THR B 206 | 16.997 | 10.650 | -10.072 | 1.00 | 31.73 | C |
| ATOM | 3208 | O   | THR B 206 | 16.930 | 10.751 | -8.845  | 1.00 | 28.88 | O |
| ATOM | 3209 | CB  | THR B 206 | 17.363 | 12.854 | -11.063 | 1.00 | 32.94 | C |
| ATOM | 3210 | OG1 | THR B 206 | 17.332 | 13.537 | -9.805  | 1.00 | 34.72 | O |
| ATOM | 3211 | CG2 | THR B 206 | 16.937 | 13.816 | -12.167 | 1.00 | 32.47 | C |
| ATOM | 3212 | N   | LYS B 207 | 17.515 | 9.606  | -10.713 | 1.00 | 31.71 | N |
| ATOM | 3213 | CA  | LYS B 207 | 18.213 | 8.496  | -10.047 | 1.00 | 32.34 | C |
| ATOM | 3214 | C   | LYS B 207 | 19.389 | 8.187  | -10.930 | 1.00 | 29.54 | C |
| ATOM | 3215 | O   | LYS B 207 | 19.223 | 7.838  | -12.103 | 1.00 | 30.31 | O |
| ATOM | 3216 | CB  | LYS B 207 | 17.323 | 7.261  | -9.915  | 1.00 | 33.39 | C |
| ATOM | 3217 | CG  | LYS B 207 | 16.739 | 7.026  | -8.529  | 1.00 | 34.92 | C |
| ATOM | 3218 | CD  | LYS B 207 | 15.553 | 6.039  | -8.620  | 1.00 | 35.53 | C |
| ATOM | 3219 | CE  | LYS B 207 | 15.286 | 5.319  | -7.296  | 1.00 | 37.29 | C |
| ATOM | 3220 | NZ  | LYS B 207 | 13.815 | 5.150  | -7.027  | 1.00 | 39.31 | N |
| ATOM | 3221 | N   | VAL B 208 | 20.588 | 8.383  | -10.398 | 1.00 | 27.15 | N |
| ATOM | 3222 | CA  | VAL B 208 | 21.772 | 8.182  | -11.175 | 1.00 | 24.54 | C |
| ATOM | 3223 | C   | VAL B 208 | 22.706 | 7.258  | -10.422 | 1.00 | 22.48 | C |
| ATOM | 3224 | O   | VAL B 208 | 22.916 | 7.416  | -9.230  | 1.00 | 21.22 | O |
| ATOM | 3225 | CB  | VAL B 208 | 22.504 | 9.529  | -11.486 | 1.00 | 25.40 | C |
| ATOM | 3226 | CG1 | VAL B 208 | 23.812 | 9.251  | -12.192 | 1.00 | 26.20 | C |
| ATOM | 3227 | CG2 | VAL B 208 | 21.625 | 10.428 | -12.326 | 1.00 | 27.80 | C |
| ATOM | 3228 | N   | ASP B 209 | 23.231 | 6.281  | -11.135 | 1.00 | 21.31 | N |
| ATOM | 3229 | CA  | ASP B 209 | 24.308 | 5.470  | -10.618 | 1.00 | 22.10 | C |
| ATOM | 3230 | C   | ASP B 209 | 25.570 | 5.810  | -11.390 | 1.00 | 21.59 | C |
| ATOM | 3231 | O   | ASP B 209 | 25.584 | 5.814  | -12.632 | 1.00 | 24.35 | O |
| ATOM | 3232 | CB  | ASP B 209 | 23.951 | 3.991  | -10.730 | 1.00 | 21.87 | C |
| ATOM | 3233 | CG  | ASP B 209 | 22.670 | 3.642  | -9.983  | 1.00 | 24.44 | C |
| ATOM | 3234 | OD1 | ASP B 209 | 22.379 | 4.205  | -8.896  | 1.00 | 22.90 | O |
| ATOM | 3235 | OD2 | ASP B 209 | 21.935 | 2.782  | -10.493 | 1.00 | 30.83 | O |
| ATOM | 3236 | N   | LYS B 210 | 26.642 | 6.097  | -10.671 | 1.00 | 19.17 | N |
| ATOM | 3237 | CA  | LYS B 210 | 27.909 | 6.350  | -11.317 | 1.00 | 17.52 | C |
| ATOM | 3238 | C   | LYS B 210 | 28.946 | 5.353  | -10.808 | 1.00 | 16.39 | C |

| ATOM | 3239 | O | LYS | B | 210 | 29.329 | 5.432 | -9.669 | 1.00 | 16.89 | O |
|------|------|-----|-----|---|-----|--------|--------|---------|------|-------|---|
| ATOM | 3240 | CB | LYS | B | 210 | 28.345 | 7.783 | -10.994 | 1.00 | 17.23 | C |
| ATOM | 3241 | CG | LYS | B | 210 | 29.645 | 8.256 | -11.608 | 1.00 | 17.91 | C |
| ATOM | 3242 | CD | LYS | B | 210 | 29.785 | 8.015 | -13.102 | 1.00 | 18.21 | C |
| ATOM | 3243 | CE | LYS | B | 210 | 28.782 | 8.796 | -13.924 | 1.00 | 16.81 | C |
| ATOM | 3244 | NZ | LYS | B | 210 | 28.978 | 8.675 | -15.416 | 1.00 | 16.84 | N |
| ATOM | 3245 | N | LYS | B | 211 | 29.398 | 4.428 | -11.651 | 1.00 | 18.14 | N |
| ATOM | 3246 | CA | LYS | B | 211 | 30.470 | 3.514 | -11.243 | 1.00 | 19.25 | C |
| ATOM | 3247 | C | LYS | B | 211 | 31.818 | 4.241 | -11.237 | 1.00 | 18.76 | C |
| ATOM | 3248 | O | LYS | B | 211 | 32.142 | 4.971 | -12.177 | 1.00 | 18.00 | O |
| ATOM | 3249 | CB | LYS | B | 211 | 30.536 | 2.326 | -12.194 | 1.00 | 19.93 | C |
| ATOM | 3250 | CG | LYS | B | 211 | 31.481 | 1.221 | -11.741 | 1.00 | 22.36 | C |
| ATOM | 3251 | CD | LYS | B | 211 | 31.331 | -0.012 | -12.617 | 1.00 | 24.13 | C |
| ATOM | 3252 | CE | LYS | B | 211 | 32.658 | -0.780 | -12.733 | 1.00 | 26.64 | C |
| ATOM | 3253 | NZ | LYS | B | 211 | 32.625 | -1.929 | -13.714 | 1.00 | 28.71 | N |
| ATOM | 3254 | N | ILE | B | 212 | 32.596 | 4.048 | -10.177 | 1.00 | 17.16 | N |
| ATOM | 3255 | CA | ILE | B | 212 | 33.941 | 4.604 | -10.093 | 1.00 | 17.55 | C |
| ATOM | 3256 | C | ILE | B | 212 | 34.921 | 3.640 | -10.757 | 1.00 | 21.01 | C |
| ATOM | 3257 | O | ILE | B | 212 | 35.134 | 2.507 | -10.286 | 1.00 | 18.22 | O |
| ATOM | 3258 | CB | ILE | B | 212 | 34.366 | 4.893 | -8.631 | 1.00 | 17.91 | C |
| ATOM | 3259 | CG1 | ILE | B | 212 | 33.237 | 5.587 | -7.852 | 1.00 | 17.74 | C |
| ATOM | 3260 | CG2 | ILE | B | 212 | 35.643 | 5.764 | -8.598 | 1.00 | 17.41 | C |
| ATOM | 3261 | CD1 | ILE | B | 212 | 32.742 | 6.887 | -8.482 | 1.00 | 14.58 | C |
| ATOM | 3262 | N | VAL | B | 213 | 35.479 | 4.073 | -11.882 | 1.00 | 21.11 | N |
| ATOM | 3263 | CA | VAL | B | 213 | 36.411 | 3.235 | -12.636 | 1.00 | 24.77 | C |
| ATOM | 3264 | C | VAL | B | 213 | 37.798 | 3.868 | -12.568 | 1.00 | 26.14 | C |
| ATOM | 3265 | O | VAL | B | 213 | 37.935 | 5.108 | -12.576 | 1.00 | 25.95 | O |
| ATOM | 3266 | CB | VAL | B | 213 | 35.921 | 2.985 | -14.092 | 1.00 | 24.89 | C |
| ATOM | 3267 | CG1 | VAL | B | 213 | 34.547 | 2.336 | -14.076 | 1.00 | 27.43 | C |
| ATOM | 3268 | CG2 | VAL | B | 213 | 35.841 | 4.229 | -14.878 | 1.00 | 26.37 | C |
| ATOM | 3269 | N | PRO | B | 214 | 38.846 | 3.026 | -12.462 | 1.00 | 28.12 | N |
| ATOM | 3270 | CA | PRO | B | 214 | 40.183 | 3.596 | -12.389 | 1.00 | 28.75 | C |
| ATOM | 3271 | C | PRO | B | 214 | 40.512 | 4.382 | -13.659 | 1.00 | 30.16 | C |
| ATOM | 3272 | O | PRO | B | 214 | 39.968 | 4.104 | -14.730 | 1.00 | 30.29 | O |
| ATOM | 3273 | CB | PRO | B | 214 | 41.095 | 2.372 | -12.185 | 1.00 | 29.44 | C |
| ATOM | 3274 | CG | PRO | B | 214 | 40.269 | 1.176 | -12.509 | 1.00 | 28.79 | C |
| ATOM | 3275 | CD | PRO | B | 214 | 38.835 | 1.550 | -12.404 | 1.00 | 27.67 | C |
| ATOM | 3276 | N | ARG | B | 215 | 41.357 | 5.395 | -13.527 | 1.00 | 32.50 | N |
| ATOM | 3277 | CA | ARG | B | 215 | 41.636 | 6.299 | -14.645 | 1.00 | 34.13 | C |
| ATOM | 3278 | C | ARG | B | 215 | 42.696 | 5.740 | -15.602 | 1.00 | 34.91 | C |
| ATOM | 3279 | O | ARG | B | 215 | 43.741 | 5.236 | -15.170 | 1.00 | 36.05 | O |
| ATOM | 3280 | CB | ARG | B | 215 | 42.056 | 7.666 | -14.104 | 1.00 | 34.13 | C |
| ATOM | 3281 | CG | ARG | B | 215 | 40.933 | 8.362 | -13.348 | 1.00 | 34.73 | C |
| ATOM | 3282 | CD | ARG | B | 215 | 41.416 | 9.588 | -12.613 | 1.00 | 34.02 | C |
| ATOM | 3283 | NE | ARG | B | 215 | 42.181 | 10.463 | -13.502 | 1.00 | 35.06 | N |
| ATOM | 3284 | CZ | ARG | B | 215 | 41.693 | 11.522 | -14.151 | 1.00 | 34.09 | C |
| ATOM | 3285 | NH1 | ARG | B | 215 | 40.416 | 11.868 | -14.028 | 1.00 | 33.27 | N |
| ATOM | 3286 | NH2 | ARG | B | 215 | 42.495 | 12.237 | -14.933 | 1.00 | 33.33 | N |
| TER | 3287 | | ARG | B | 215 | | | | | | |
| ATOM | 3288 | N | ASP | C | 1 | 28.869 | 35.358 | 1.767 | 1.00 | 32.08 | N |
| ATOM | 3289 | CA | ASP | C | 1 | 28.159 | 36.574 | 1.263 | 1.00 | 30.78 | C |
| ATOM | 3290 | C | ASP | C | 1 | 26.794 | 36.681 | 1.920 | 1.00 | 27.64 | C |
| ATOM | 3291 | O | ASP | C | 1 | 26.105 | 35.674 | 2.130 | 1.00 | 28.94 | O |
| ATOM | 3292 | CB | ASP | C | 1 | 27.972 | 36.522 | -0.251 | 1.00 | 31.99 | C |
| ATOM | 3293 | CG | ASP | C | 1 | 29.264 | 36.273 | -0.992 | 1.00 | 34.47 | C |
| ATOM | 3294 | OD1 | ASP | C | 1 | 30.313 | 36.100 | -0.336 | 1.00 | 35.43 | O |
| ATOM | 3295 | OD2 | ASP | C | 1 | 29.224 | 36.265 | -2.240 | 1.00 | 38.84 | O |
| ATOM | 3296 | N | ILE | C | 2 | 26.397 | 37.909 | 2.227 | 1.00 | 21.74 | N |
| ATOM | 3297 | CA | ILE | C | 2 | 25.122 | 38.138 | 2.880 | 1.00 | 18.33 | C |
| ATOM | 3298 | C | ILE | C | 2 | 24.008 | 37.995 | 1.877 | 1.00 | 16.67 | C |
| ATOM | 3299 | O | ILE | C | 2 | 24.068 | 38.524 | 0.770 | 1.00 | 16.03 | O |
| ATOM | 3300 | CB | ILE | C | 2 | 25.089 | 39.543 | 3.554 | 1.00 | 18.52 | C |
| ATOM | 3301 | CG1 | ILE | C | 2 | 26.115 | 39.572 | 4.680 | 1.00 | 17.54 | C |
| ATOM | 3302 | CG2 | ILE | C | 2 | 23.676 | 39.894 | 4.052 | 1.00 | 16.86 | C |
| ATOM | 3303 | CD1 | ILE | C | 2 | 26.274 | 40.903 | 5.330 | 1.00 | 19.29 | C |

| ATOM | 3304 | N | VAL | C | 3 | 22.992 | 37.250 | 2.267 | 1.00 | 15.14 | N |
|------|------|---|-----|---|---|--------|--------|-------|------|-------|---|
| ATOM | 3305 | CA | VAL | C | 3 | 21.789 | 37.094 | 1.478 | 1.00 | 15.73 | C |
| ATOM | 3306 | C | VAL | C | 3 | 20.694 | 38.005 | 2.013 | 1.00 | 14.44 | C |
| ATOM | 3307 | O | VAL | C | 3 | 20.300 | 37.878 | 3.162 | 1.00 | 13.92 | O |
| ATOM | 3308 | CB | VAL | C | 3 | 21.280 | 35.634 | 1.516 | 1.00 | 15.94 | C |
| ATOM | 3309 | CG1 | VAL | C | 3 | 19.963 | 35.492 | 0.734 | 1.00 | 16.42 | C |
| ATOM | 3310 | CG2 | VAL | C | 3 | 22.359 | 34.701 | 0.960 | 1.00 | 18.58 | C |
| ATOM | 3311 | N | MET | C | 4 | 20.185 | 38.892 | 1.145 | 1.00 | 12.98 | N |
| ATOM | 3312 | CA | MET | C | 4 | 19.078 | 39.777 | 1.466 | 1.00 | 13.46 | C |
| ATOM | 3313 | C | MET | C | 4 | 17.819 | 39.226 | 0.832 | 1.00 | 13.04 | C |
| ATOM | 3314 | O | MET | C | 4 | 17.795 | 39.026 | -0.365 | 1.00 | 14.95 | O |
| ATOM | 3315 | CB | MET | C | 4 | 19.344 | 41.189 | 0.924 | 1.00 | 13.02 | C |
| ATOM | 3316 | CG | MET | C | 4 | 20.670 | 41.776 | 1.409 | 1.00 | 12.18 | C |
| ATOM | 3317 | SD | MET | C | 4 | 20.815 | 42.152 | 3.169 | 1.00 | 14.06 | S |
| ATOM | 3318 | CE | MET | C | 4 | 19.567 | 43.406 | 3.351 | 1.00 | 13.03 | C |
| ATOM | 3319 | N | THR | C | 5 | 16.803 | 38.951 | 1.642 | 1.00 | 12.66 | N |
| ATOM | 3320 | CA | THR | C | 5 | 15.580 | 38.311 | 1.197 | 1.00 | 14.05 | C |
| ATOM | 3321 | C | THR | C | 5 | 14.358 | 39.211 | 1.341 | 1.00 | 14.86 | C |
| ATOM | 3322 | O | THR | C | 5 | 13.973 | 39.613 | 2.445 | 1.00 | 15.87 | O |
| ATOM | 3323 | CB | THR | C | 5 | 15.335 | 36.959 | 1.939 | 1.00 | 14.59 | C |
| ATOM | 3324 | OG1 | THR | C | 5 | 16.461 | 36.116 | 1.759 | 1.00 | 15.59 | O |
| ATOM | 3325 | CG2 | THR | C | 5 | 14.079 | 36.264 | 1.413 | 1.00 | 15.32 | C |
| ATOM | 3326 | N | GLN | C | 6 | 13.725 | 39.487 | 0.207 | 1.00 | 15.05 | N |
| ATOM | 3327 | CA | GLN | C | 6 | 12.474 | 40.209 | 0.171 | 1.00 | 14.76 | C |
| ATOM | 3328 | C | GLN | C | 6 | 11.558 | 39.626 | -0.918 | 1.00 | 16.15 | C |
| ATOM | 3329 | O | GLN | C | 6 | 12.025 | 39.036 | -1.884 | 1.00 | 17.07 | O |
| ATOM | 3330 | CB | GLN | C | 6 | 12.724 | 41.709 | -0.054 | 1.00 | 16.15 | C |
| ATOM | 3331 | CG | GLN | C | 6 | 13.445 | 42.001 | -1.327 | 1.00 | 15.63 | C |
| ATOM | 3332 | CD | GLN | C | 6 | 13.771 | 43.466 | -1.537 | 1.00 | 14.19 | C |
| ATOM | 3333 | OE1 | GLN | C | 6 | 14.866 | 43.794 | -1.993 | 1.00 | 13.70 | O |
| ATOM | 3334 | NE2 | GLN | C | 6 | 12.804 | 44.349 | -1.268 | 1.00 | 14.04 | N |
| ATOM | 3335 | N | ALA | C | 7 | 10.257 | 39.761 | -0.705 | 1.00 | 17.23 | N |
| ATOM | 3336 | CA | ALA | C | 7 | 9.225 | 39.256 | -1.612 | 1.00 | 17.98 | C |
| ATOM | 3337 | C | ALA | C | 7 | 9.283 | 39.990 | -2.935 | 1.00 | 18.83 | C |
| ATOM | 3338 | O | ALA | C | 7 | 9.496 | 41.194 | -2.956 | 1.00 | 19.54 | O |
| ATOM | 3339 | CB | ALA | C | 7 | 7.852 | 39.455 | -0.990 | 1.00 | 19.48 | C |
| ATOM | 3340 | N | ALA | C | 8 | 9.033 | 39.282 | -4.030 | 1.00 | 20.19 | N |
| ATOM | 3341 | CA | ALA | C | 8 | 9.030 | 39.919 | -5.349 | 1.00 | 20.90 | C |
| ATOM | 3342 | C | ALA | C | 8 | 7.855 | 40.851 | -5.532 | 1.00 | 22.31 | C |
| ATOM | 3343 | O | ALA | C | 8 | 7.977 | 41.852 | -6.223 | 1.00 | 20.05 | O |
| ATOM | 3344 | CB | ALA | C | 8 | 9.041 | 38.876 | -6.456 | 1.00 | 22.62 | C |
| ATOM | 3345 | N | PHE | C | 9 | 6.723 | 40.534 | -4.915 | 1.00 | 23.96 | N |
| ATOM | 3346 | CA | PHE | C | 9 | 5.510 | 41.339 | -5.080 | 1.00 | 28.01 | C |
| ATOM | 3347 | C | PHE | C | 9 | 4.891 | 41.700 | -3.738 | 1.00 | 29.37 | C |
| ATOM | 3348 | O | PHE | C | 9 | 4.854 | 40.887 | -2.837 | 1.00 | 28.22 | O |
| ATOM | 3349 | CB | PHE | C | 9 | 4.508 | 40.603 | -5.971 | 1.00 | 29.78 | C |
| ATOM | 3350 | CG | PHE | C | 9 | 5.069 | 40.236 | -7.301 | 1.00 | 31.18 | C |
| ATOM | 3351 | CD1 | PHE | C | 9 | 5.246 | 41.203 | -8.277 | 1.00 | 31.84 | C |
| ATOM | 3352 | CD2 | PHE | C | 9 | 5.474 | 38.939 | -7.563 | 1.00 | 31.73 | C |
| ATOM | 3353 | CE1 | PHE | C | 9 | 5.789 | 40.882 | -9.518 | 1.00 | 32.64 | C |
| ATOM | 3354 | CE2 | PHE | C | 9 | 6.030 | 38.602 | -8.802 | 1.00 | 32.95 | C |
| ATOM | 3355 | CZ | PHE | C | 9 | 6.189 | 39.583 | -9.782 | 1.00 | 32.34 | C |
| ATOM | 3356 | N | SER | C | 10 | 4.470 | 42.954 | -3.604 | 1.00 | 31.87 | N |
| ATOM | 3357 | CA | SER | C | 10 | 3.803 | 43.433 | -2.389 | 1.00 | 33.55 | C |
| ATOM | 3358 | C | SER | C | 10 | 2.342 | 42.997 | -2.445 | 1.00 | 35.07 | C |
| ATOM | 3359 | O | SER | C | 10 | 1.856 | 42.554 | -3.498 | 1.00 | 35.05 | O |
| ATOM | 3360 | CB | SER | C | 10 | 3.898 | 44.974 | -2.285 | 1.00 | 33.09 | C |
| ATOM | 3361 | OG | SER | C | 10 | 2.853 | 45.632 | -3.003 | 1.00 | 36.22 | O |
| ATOM | 3362 | N | ASN | C | 11 | 1.618 | 43.142 | -1.336 | 1.00 | 36.74 | N |
| ATOM | 3363 | CA | ASN | C | 11 | 0.162 | 43.056 | -1.443 | 1.00 | 37.53 | C |
| ATOM | 3364 | C | ASN | C | 11 | -0.310 | 44.320 | -2.183 | 1.00 | 37.85 | C |
| ATOM | 3365 | O | ASN | C | 11 | 0.172 | 45.413 | -1.892 | 1.00 | 38.76 | O |
| ATOM | 3366 | CB | AASN | C | 11 | -0.492 | 42.986 | -0.057 | 0.50 | 37.66 | C |
| ATOM | 3367 | CB | BASN | C | 11 | -0.515 | 42.902 | -0.074 | 0.50 | 37.95 | C |
| ATOM | 3368 | CG | AASN | C | 11 | 0.132 | 41.927 | 0.849 | 0.50 | 37.76 | C |

| ATOM | 3369 | CG  | BASN | C | 11 | -1.664 | 41.885 | -0.094 | 0.50 | 38.37 | C |
|------|------|-----|------|---|----|--------|--------|--------|------|-------|---|
| ATOM | 3370 | OD1 | AASN | C | 11 | 1.099  | 41.255 | 0.478  | 0.50 | 37.49 | O |
| ATOM | 3371 | OD1 | BASN | C | 11 | -1.716 | 40.991 | -0.945 | 0.50 | 38.68 | O |
| ATOM | 3372 | ND2 | AASN | C | 11 | -0.419 | 41.785 | 2.048  | 0.50 | 37.68 | N |
| ATOM | 3373 | ND2 | BASN | C | 11 | -2.581 | 42.016 | 0.859  | 0.50 | 38.90 | N |
| ATOM | 3374 | N   | PRO  | C | 12 | -1.193 | 44.174 | -3.183 | 1.00 | 37.30 | N |
| ATOM | 3375 | CA  | PRO  | C | 12 | -1.749 | 45.339 | -3.891 | 1.00 | 36.51 | C |
| ATOM | 3376 | C   | PRO  | C | 12 | -2.368 | 46.362 | -2.928 | 1.00 | 34.58 | C |
| ATOM | 3377 | O   | PRO  | C | 12 | -3.149 | 45.987 | -2.050 | 1.00 | 35.40 | O |
| ATOM | 3378 | CB  | PRO  | C | 12 | -2.808 | 44.725 | -4.802 | 1.00 | 37.16 | C |
| ATOM | 3379 | CG  | PRO  | C | 12 | -2.354 | 43.331 | -5.011 | 1.00 | 37.46 | C |
| ATOM | 3380 | CD  | PRO  | C | 12 | -1.687 | 42.907 | -3.744 | 1.00 | 38.20 | C |
| ATOM | 3381 | N   | VAL  | C | 13 | -2.004 | 47.632 | -3.095 | 1.00 | 31.47 | N |
| ATOM | 3382 | CA  | VAL  | C | 13 | -2.294 | 48.667 | -2.116 | 1.00 | 30.24 | C |
| ATOM | 3383 | C   | VAL  | C | 13 | -3.195 | 49.737 | -2.681 | 1.00 | 28.43 | C |
| ATOM | 3384 | O   | VAL  | C | 13 | -2.889 | 50.345 | -3.699 | 1.00 | 26.99 | O |
| ATOM | 3385 | CB  | VAL  | C | 13 | -0.984 | 49.332 | -1.637 | 1.00 | 29.93 | C |
| ATOM | 3386 | CG1 | VAL  | C | 13 | -1.263 | 50.478 | -0.681 | 1.00 | 30.74 | C |
| ATOM | 3387 | CG2 | VAL  | C | 13 | -0.113 | 48.289 | -1.008 | 1.00 | 31.23 | C |
| ATOM | 3388 | N   | THR  | C | 14 | -4.298 | 50.001 | -1.993 | 1.00 | 27.18 | N |
| ATOM | 3389 | CA  | THR  | C | 14 | -5.173 | 51.106 | -2.360 | 1.00 | 27.50 | C |
| ATOM | 3390 | C   | THR  | C | 14 | -4.518 | 52.486 | -2.221 | 1.00 | 27.35 | C |
| ATOM | 3391 | O   | THR  | C | 14 | -3.854 | 52.779 | -1.229 | 1.00 | 26.62 | O |
| ATOM | 3392 | CB  | THR  | C | 14 | -6.457 | 51.054 | -1.522 | 1.00 | 28.62 | C |
| ATOM | 3393 | OG1 | THR  | C | 14 | -7.142 | 49.829 | -1.804 | 1.00 | 27.67 | O |
| ATOM | 3394 | CG2 | THR  | C | 14 | -7.371 | 52.236 | -1.831 | 1.00 | 29.35 | C |
| ATOM | 3395 | N   | LEU  | C | 15 | -4.699 | 53.342 | -3.221 | 1.00 | 27.15 | N |
| ATOM | 3396 | CA  | LEU  | C | 15 | -4.211 | 54.713 | -3.123 | 1.00 | 28.64 | C |
| ATOM | 3397 | C   | LEU  | C | 15 | -4.696 | 55.364 | -1.835 | 1.00 | 28.45 | C |
| ATOM | 3398 | O   | LEU  | C | 15 | -5.867 | 55.228 | -1.450 | 1.00 | 29.72 | O |
| ATOM | 3399 | CB  | LEU  | C | 15 | -4.674 | 55.565 | -4.310 | 1.00 | 29.74 | C |
| ATOM | 3400 | CG  | LEU  | C | 15 | -3.928 | 55.458 | -5.637 | 1.00 | 31.99 | C |
| ATOM | 3401 | CD1 | LEU  | C | 15 | -4.519 | 56.458 | -6.629 | 1.00 | 33.16 | C |
| ATOM | 3402 | CD2 | LEU  | C | 15 | -2.448 | 55.732 | -5.457 | 1.00 | 33.67 | C |
| ATOM | 3403 | N   | GLY  | C | 16 | -3.800 | 56.062 | -1.169 | 1.00 | 27.60 | N |
| ATOM | 3404 | CA  | GLY  | C | 16 | -4.137 | 56.789 | 0.056  | 1.00 | 27.55 | C |
| ATOM | 3405 | C   | GLY  | C | 16 | -4.036 | 55.975 | 1.333  | 1.00 | 27.14 | C |
| ATOM | 3406 | O   | GLY  | C | 16 | -4.232 | 56.510 | 2.416  | 1.00 | 28.01 | O |
| ATOM | 3407 | N   | THR  | C | 17 | -3.734 | 54.683 | 1.212  | 1.00 | 26.49 | N |
| ATOM | 3408 | CA  | THR  | C | 17 | -3.540 | 53.814 | 2.369  | 1.00 | 25.83 | C |
| ATOM | 3409 | C   | THR  | C | 17 | -2.083 | 53.362 | 2.441  | 1.00 | 25.03 | C |
| ATOM | 3410 | O   | THR  | C | 17 | -1.275 | 53.747 | 1.591  | 1.00 | 22.42 | O |
| ATOM | 3411 | CB  | THR  | C | 17 | -4.468 | 52.605 | 2.319  | 1.00 | 26.28 | C |
| ATOM | 3412 | OG1 | THR  | C | 17 | -4.024 | 51.684 | 1.324  | 1.00 | 26.09 | O |
| ATOM | 3413 | CG2 | THR  | C | 17 | -5.910 | 53.046 | 2.008  | 1.00 | 27.11 | C |
| ATOM | 3414 | N   | SER  | C | 18 | -1.750 | 52.543 | 3.440  | 1.00 | 24.84 | N |
| ATOM | 3415 | CA  | SER  | C | 18 | -0.345 | 52.232 | 3.711  | 1.00 | 24.38 | C |
| ATOM | 3416 | C   | SER  | C | 18 | 0.143  | 50.908 | 3.124  | 1.00 | 23.87 | C |
| ATOM | 3417 | O   | SER  | C | 18 | -0.601 | 49.923 | 3.059  | 1.00 | 23.50 | O |
| ATOM | 3418 | CB  | SER  | C | 18 | -0.098 | 52.248 | 5.211  | 1.00 | 26.43 | C |
| ATOM | 3419 | OG  | SER  | C | 18 | -0.251 | 53.566 | 5.708  | 1.00 | 26.82 | O |
| ATOM | 3420 | N   | ALA  | C | 19 | 1.396  | 50.926 | 2.667  | 1.00 | 21.73 | N |
| ATOM | 3421 | CA  | ALA  | C | 19 | 2.093  | 49.746 | 2.191  | 1.00 | 21.22 | C |
| ATOM | 3422 | C   | ALA  | C | 19 | 3.166  | 49.381 | 3.202  | 1.00 | 19.58 | C |
| ATOM | 3423 | O   | ALA  | C | 19 | 3.675  | 50.246 | 3.901  | 1.00 | 17.20 | O |
| ATOM | 3424 | CB  | ALA  | C | 19 | 2.711  | 50.017 | 0.853  | 1.00 | 20.33 | C |
| ATOM | 3425 | N   | SER  | C | 20 | 3.499  | 48.096 | 3.273  | 1.00 | 18.83 | N |
| ATOM | 3426 | CA  | SER  | C | 20 | 4.529  | 47.593 | 4.152  | 1.00 | 19.59 | C |
| ATOM | 3427 | C   | SER  | C | 20 | 5.351  | 46.590 | 3.363  | 1.00 | 19.47 | C |
| ATOM | 3428 | O   | SER  | C | 20 | 4.797  | 45.654 | 2.767  | 1.00 | 19.14 | O |
| ATOM | 3429 | CB  | SER  | C | 20 | 3.898  | 46.910 | 5.360  | 1.00 | 20.24 | C |
| ATOM | 3430 | OG  | SER  | C | 20 | 4.868  | 46.437 | 6.270  | 1.00 | 21.97 | O |
| ATOM | 3431 | N   | ILE  | C | 21 | 6.656  | 46.800 | 3.333  | 1.00 | 17.78 | N |
| ATOM | 3432 | CA  | ILE  | C | 21 | 7.575  | 45.920 | 2.596  | 1.00 | 18.04 | C |
| ATOM | 3433 | C   | ILE  | C | 21 | 8.641  | 45.384 | 3.547  | 1.00 | 17.85 | C |

| | | | | | | | | | | | |
|---|---|---|---|---|---|---|---|---|---|---|---|
| ATOM | 3434 | O | ILE | C | 21 | 9.359 | 46.136 | 4.196 | 1.00 | 17.01 | O |
| ATOM | 3435 | CB | ILE | C | 21 | 8.250 | 46.676 | 1.403 | 1.00 | 17.67 | C |
| ATOM | 3436 | CG1 | ILE | C | 21 | 7.172 | 47.126 | 0.390 | 1.00 | 19.79 | C |
| ATOM | 3437 | CG2 | ILE | C | 21 | 9.328 | 45.786 | 0.716 | 1.00 | 19.82 | C |
| ATOM | 3438 | CD1 | ILE | C | 21 | 7.714 | 47.878 | -0.795 | 1.00 | 18.90 | C |
| ATOM | 3439 | N | SER | C | 22 | 8.751 | 44.059 | 3.586 | 1.00 | 17.72 | N |
| ATOM | 3440 | CA | SER | C | 22 | 9.680 | 43.354 | 4.432 | 1.00 | 18.02 | C |
| ATOM | 3441 | C | SER | C | 22 | 10.978 | 43.016 | 3.740 | 1.00 | 15.31 | C |
| ATOM | 3442 | O | SER | C | 22 | 10.988 | 42.714 | 2.548 | 1.00 | 14.37 | O |
| ATOM | 3443 | CB | SER | C | 22 | 9.056 | 42.013 | 4.837 | 1.00 | 18.68 | C |
| ATOM | 3444 | OG | SER | C | 22 | 8.009 | 42.244 | 5.733 | 1.00 | 26.79 | O |
| ATOM | 3445 | N | CYS | C | 23 | 12.045 | 42.971 | 4.533 | 1.00 | 15.44 | N |
| ATOM | 3446 | CA | CYS | C | 23 | 13.308 | 42.388 | 4.140 | 1.00 | 15.74 | C |
| ATOM | 3447 | C | CYS | C | 23 | 13.937 | 41.665 | 5.342 | 1.00 | 14.71 | C |
| ATOM | 3448 | O | CYS | C | 23 | 13.641 | 41.957 | 6.513 | 1.00 | 14.29 | O |
| ATOM | 3449 | CB | CYS | C | 23 | 14.252 | 43.496 | 3.608 | 1.00 | 15.49 | C |
| ATOM | 3450 | SG | CYS | C | 23 | 15.886 | 42.918 | 3.062 | 1.00 | 17.53 | S |
| ATOM | 3451 | N | ARG | C | 24 | 14.789 | 40.694 | 5.059 | 1.00 | 14.75 | N |
| ATOM | 3452 | CA | ARG | C | 24 | 15.618 | 40.116 | 6.081 | 1.00 | 13.83 | C |
| ATOM | 3453 | C | ARG | C | 24 | 17.023 | 39.777 | 5.571 | 1.00 | 13.94 | C |
| ATOM | 3454 | O | ARG | C | 24 | 17.250 | 39.639 | 4.368 | 1.00 | 15.41 | O |
| ATOM | 3455 | CB | ARG | C | 24 | 14.913 | 38.909 | 6.672 | 1.00 | 16.82 | C |
| ATOM | 3456 | CG | ARG | C | 24 | 14.896 | 37.746 | 5.778 | 1.00 | 17.84 | C |
| ATOM | 3457 | CD | ARG | C | 24 | 14.217 | 36.610 | 6.465 | 1.00 | 22.23 | C |
| ATOM | 3458 | NE | ARG | C | 24 | 14.237 | 35.426 | 5.617 | 1.00 | 22.63 | N |
| ATOM | 3459 | CZ | ARG | C | 24 | 13.276 | 35.054 | 4.793 | 1.00 | 23.80 | C |
| ATOM | 3460 | NH1 | ARG | C | 24 | 12.155 | 35.771 | 4.639 | 1.00 | 25.76 | N |
| ATOM | 3461 | NH2 | ARG | C | 24 | 13.453 | 33.924 | 4.104 | 1.00 | 25.55 | N |
| ATOM | 3462 | N | SER | C | 25 | 17.978 | 39.715 | 6.480 | 1.00 | 12.40 | N |
| ATOM | 3463 | CA | SER | C | 25 | 19.358 | 39.490 | 6.116 | 1.00 | 13.71 | C |
| ATOM | 3464 | C | SER | C | 25 | 19.853 | 38.247 | 6.803 | 1.00 | 13.26 | C |
| ATOM | 3465 | O | SER | C | 25 | 19.437 | 37.968 | 7.922 | 1.00 | 13.15 | O |
| ATOM | 3466 | CB A | SER | C | 25 | 20.184 | 40.676 | 6.584 | 0.50 | 14.48 | C |
| ATOM | 3467 | CB B | SER | C | 25 | 20.241 | 40.662 | 6.512 | 0.50 | 14.03 | C |
| ATOM | 3468 | OG A | SER | C | 25 | 21.558 | 40.433 | 6.430 | 0.50 | 15.84 | O |
| ATOM | 3469 | OG B | SER | C | 25 | 20.140 | 40.912 | 7.894 | 0.50 | 13.68 | O |
| ATOM | 3470 | N | SER | C | 26 | 20.782 | 37.547 | 6.164 | 1.00 | 14.84 | N |
| ATOM | 3471 | CA | SER | C | 26 | 21.332 | 36.305 | 6.728 | 1.00 | 15.52 | C |
| ATOM | 3472 | C | SER | C | 26 | 22.395 | 36.530 | 7.812 | 1.00 | 17.40 | C |
| ATOM | 3473 | O | SER | C | 26 | 22.799 | 35.585 | 8.511 | 1.00 | 18.24 | O |
| ATOM | 3474 | CB | SER | C | 26 | 21.921 | 35.458 | 5.604 | 1.00 | 15.65 | C |
| ATOM | 3475 | OG | SER | C | 26 | 22.967 | 36.122 | 4.964 | 1.00 | 12.17 | O |
| ATOM | 3476 | N | LYS | C | 27 | 22.880 | 37.765 | 7.923 | 1.00 | 17.24 | N |
| ATOM | 3477 | CA | LYS | C | 27 | 23.782 | 38.176 | 8.991 | 1.00 | 19.54 | C |
| ATOM | 3478 | C | LYS | C | 27 | 23.237 | 39.451 | 9.582 | 1.00 | 18.28 | C |
| ATOM | 3479 | O | LYS | C | 27 | 22.591 | 40.242 | 8.882 | 1.00 | 17.11 | O |
| ATOM | 3480 | CB | LYS | C | 27 | 25.176 | 38.485 | 8.461 | 1.00 | 21.04 | C |
| ATOM | 3481 | CG | LYS | C | 27 | 25.951 | 37.318 | 7.964 | 1.00 | 24.76 | C |
| ATOM | 3482 | CD | LYS | C | 27 | 27.415 | 37.734 | 7.728 | 1.00 | 25.14 | C |
| ATOM | 3483 | CE | LYS | C | 27 | 28.178 | 36.737 | 6.863 | 1.00 | 28.52 | C |
| ATOM | 3484 | NZ | LYS | C | 27 | 29.486 | 37.328 | 6.422 | 1.00 | 27.56 | N |
| ATOM | 3485 | N | SER | C | 27A | 23.520 | 39.672 | 10.860 | 1.00 | 17.16 | N |
| ATOM | 3486 | CA | SER | C | 27A | 23.085 | 40.897 | 11.496 | 1.00 | 16.00 | C |
| ATOM | 3487 | C | SER | C | 27A | 23.838 | 42.090 | 10.905 | 1.00 | 15.50 | C |
| ATOM | 3488 | O | SER | C | 27A | 25.043 | 42.062 | 10.727 | 1.00 | 14.76 | O |
| ATOM | 3489 | CB | SER | C | 27A | 23.280 | 40.859 | 13.008 | 1.00 | 16.96 | C |
| ATOM | 3490 | OG | SER | C | 27A | 23.027 | 42.144 | 13.535 | 1.00 | 16.70 | O |
| ATOM | 3491 | N | LEU | C | 27B | 23.079 | 43.136 | 10.619 | 1.00 | 14.37 | N |
| ATOM | 3492 | CA | LEU | C | 27B | 23.593 | 44.393 | 10.107 | 1.00 | 13.42 | C |
| ATOM | 3493 | C | LEU | C | 27B | 23.783 | 45.426 | 11.215 | 1.00 | 14.29 | C |
| ATOM | 3494 | O | LEU | C | 27B | 24.180 | 46.578 | 10.936 | 1.00 | 12.37 | O |
| ATOM | 3495 | CB | LEU | C | 27B | 22.612 | 44.920 | 9.050 | 1.00 | 11.52 | C |
| ATOM | 3496 | CG | LEU | C | 27B | 22.323 | 43.931 | 7.908 | 1.00 | 11.34 | C |
| ATOM | 3497 | CD1 | LEU | C | 27B | 21.434 | 44.563 | 6.835 | 1.00 | 12.19 | C |
| ATOM | 3498 | CD2 | LEU | C | 27B | 23.571 | 43.393 | 7.309 | 1.00 | 11.59 | C |

| ATOM | 3499 | N | LEU C | 27C | 23.498 | 45.025 | 12.460 | 1.00 | 14.27 | N |
|------|------|-----|--------|-----|--------|--------|--------|------|-------|---|
| ATOM | 3500 | CA | LEU C | 27C | 23.696 | 45.895 | 13.617 | 1.00 | 15.76 | C |
| ATOM | 3501 | C | LEU C | 27C | 25.171 | 45.832 | 14.005 | 1.00 | 16.13 | C |
| ATOM | 3502 | O | LEU C | 27C | 25.690 | 44.775 | 14.342 | 1.00 | 18.71 | O |
| ATOM | 3503 | CB | LEU C | 27C | 22.760 | 45.535 | 14.783 | 1.00 | 15.79 | C |
| ATOM | 3504 | CG | LEU C | 27C | 23.075 | 46.276 | 16.105 | 1.00 | 15.69 | C |
| ATOM | 3505 | CD1 | LEU C | 27C | 23.044 | 47.766 | 15.910 | 1.00 | 16.97 | C |
| ATOM | 3506 | CD2 | LEU C | 27C | 22.149 | 45.878 | 17.235 | 1.00 | 17.52 | C |
| ATOM | 3507 | N | HIS C | 27D | 25.833 | 46.984 | 13.943 | 1.00 | 15.78 | N |
| ATOM | 3508 | CA | HIS C | 27D | 27.258 | 47.141 | 14.140 | 1.00 | 17.85 | C |
| ATOM | 3509 | C | HIS C | 27D | 27.503 | 47.523 | 15.613 | 1.00 | 18.21 | C |
| ATOM | 3510 | O | HIS C | 27D | 26.593 | 47.946 | 16.348 | 1.00 | 18.20 | O |
| ATOM | 3511 | CB | HIS C | 27D | 27.769 | 48.232 | 13.166 | 1.00 | 17.95 | C |
| ATOM | 3512 | CG | HIS C | 27D | 29.263 | 48.358 | 13.069 | 1.00 | 17.67 | C |
| ATOM | 3513 | ND1 | HIS C | 27D | 30.022 | 49.006 | 14.020 | 1.00 | 18.08 | N |
| ATOM | 3514 | CD2 | HIS C | 27D | 30.129 | 47.991 | 12.092 | 1.00 | 18.68 | C |
| ATOM | 3515 | CE1 | HIS C | 27D | 31.296 | 48.982 | 13.664 | 1.00 | 18.30 | C |
| ATOM | 3516 | NE2 | HIS C | 27D | 31.387 | 48.366 | 12.499 | 1.00 | 18.54 | N |
| ATOM | 3517 | N | SER C | 27E | 28.735 | 47.349 | 16.056 | 1.00 | 19.75 | N |
| ATOM | 3518 | CA | SER C | 27E | 29.102 | 47.691 | 17.424 | 1.00 | 21.08 | C |
| ATOM | 3519 | C | SER C | 27E | 28.943 | 49.185 | 17.679 | 1.00 | 19.98 | C |
| ATOM | 3520 | O | SER C | 27E | 28.773 | 49.592 | 18.819 | 1.00 | 21.83 | O |
| ATOM | 3521 | CB | SER C | 27E | 30.536 | 47.252 | 17.717 | 1.00 | 21.58 | C |
| ATOM | 3522 | OG | SER C | 27E | 31.388 | 47.660 | 16.686 | 1.00 | 26.19 | O |
| ATOM | 3523 | N | ASP C | 28 | 28.980 | 49.997 | 16.621 | 1.00 | 19.37 | N |
| ATOM | 3524 | CA | ASP C | 28 | 28.801 | 51.456 | 16.758 | 1.00 | 18.04 | C |
| ATOM | 3525 | C | ASP C | 28 | 27.341 | 51.875 | 16.983 | 1.00 | 19.08 | C |
| ATOM | 3526 | O | ASP C | 28 | 27.053 | 53.069 | 17.153 | 1.00 | 18.60 | O |
| ATOM | 3527 | CB | ASP C | 28 | 29.446 | 52.214 | 15.582 | 1.00 | 19.46 | C |
| ATOM | 3528 | CG | ASP C | 28 | 28.771 | 51.951 | 14.223 | 1.00 | 19.70 | C |
| ATOM | 3529 | OD1 | ASP C | 28 | 27.671 | 51.387 | 14.201 | 1.00 | 19.40 | O |
| ATOM | 3530 | OD2 | ASP C | 28 | 29.372 | 52.336 | 13.178 | 1.00 | 19.58 | O |
| ATOM | 3531 | N | GLY C | 29 | 26.436 | 50.893 | 16.996 | 1.00 | 17.19 | N |
| ATOM | 3532 | CA | GLY C | 29 | 25.018 | 51.118 | 17.250 | 1.00 | 17.07 | C |
| ATOM | 3533 | C | GLY C | 29 | 24.151 | 51.388 | 16.033 | 1.00 | 15.20 | C |
| ATOM | 3534 | O | GLY C | 29 | 22.922 | 51.470 | 16.173 | 1.00 | 14.99 | O |
| ATOM | 3535 | N | ILE C | 30 | 24.774 | 51.529 | 14.859 | 1.00 | 13.73 | N |
| ATOM | 3536 | CA | ILE C | 30 | 24.058 | 51.779 | 13.621 | 1.00 | 14.14 | C |
| ATOM | 3537 | C | ILE C | 30 | 23.707 | 50.448 | 12.969 | 1.00 | 13.12 | C |
| ATOM | 3538 | O | ILE C | 30 | 24.513 | 49.530 | 12.950 | 1.00 | 12.00 | O |
| ATOM | 3539 | CB | ILE C | 30 | 24.887 | 52.664 | 12.640 | 1.00 | 15.20 | C |
| ATOM | 3540 | CG1 | ILE C | 30 | 25.093 | 54.064 | 13.251 | 1.00 | 16.89 | C |
| ATOM | 3541 | CG2 | ILE C | 30 | 24.217 | 52.763 | 11.241 | 1.00 | 14.29 | C |
| ATOM | 3542 | CD1 | ILE C | 30 | 26.190 | 54.842 | 12.603 | 1.00 | 17.36 | C |
| ATOM | 3543 | N | THR C | 31 | 22.487 | 50.371 | 12.446 | 1.00 | 12.73 | N |
| ATOM | 3544 | CA | THR C | 31 | 22.080 | 49.238 | 11.609 | 1.00 | 11.90 | C |
| ATOM | 3545 | C | THR C | 31 | 22.246 | 49.667 | 10.161 | 1.00 | 13.62 | C |
| ATOM | 3546 | O | THR C | 31 | 21.531 | 50.551 | 9.664 | 1.00 | 13.57 | O |
| ATOM | 3547 | CB | THR C | 31 | 20.668 | 48.808 | 11.908 | 1.00 | 11.69 | C |
| ATOM | 3548 | OG1 | THR C | 31 | 20.564 | 48.543 | 13.309 | 1.00 | 11.47 | O |
| ATOM | 3549 | CG2 | THR C | 31 | 20.329 | 47.520 | 11.133 | 1.00 | 11.62 | C |
| ATOM | 3550 | N | TYR C | 32 | 23.209 | 49.026 | 9.490 | 1.00 | 12.65 | N |
| ATOM | 3551 | CA | TYR C | 32 | 23.590 | 49.387 | 8.137 | 1.00 | 12.99 | C |
| ATOM | 3552 | C | TYR C | 32 | 22.692 | 48.760 | 7.073 | 1.00 | 13.62 | C |
| ATOM | 3553 | O | TYR C | 32 | 23.156 | 47.980 | 6.238 | 1.00 | 12.32 | O |
| ATOM | 3554 | CB | TYR C | 32 | 25.055 | 49.064 | 7.907 | 1.00 | 14.05 | C |
| ATOM | 3555 | CG | TYR C | 32 | 25.968 | 49.974 | 8.688 | 1.00 | 13.88 | C |
| ATOM | 3556 | CD1 | TYR C | 32 | 26.369 | 49.662 | 9.969 | 1.00 | 14.48 | C |
| ATOM | 3557 | CD2 | TYR C | 32 | 26.425 | 51.166 | 8.124 | 1.00 | 15.92 | C |
| ATOM | 3558 | CE1 | TYR C | 32 | 27.227 | 50.532 | 10.690 | 1.00 | 14.24 | C |
| ATOM | 3559 | CE2 | TYR C | 32 | 27.269 | 52.026 | 8.825 | 1.00 | 14.95 | C |
| ATOM | 3560 | CZ | TYR C | 32 | 27.653 | 51.710 | 10.101 | 1.00 | 14.90 | C |
| ATOM | 3561 | OH | TYR C | 32 | 28.491 | 52.567 | 10.781 | 1.00 | 14.77 | O |
| ATOM | 3562 | N | LEU C | 33 | 21.416 | 49.155 | 7.125 | 1.00 | 13.66 | N |
| ATOM | 3563 | CA | LEU C | 33 | 20.364 | 48.724 | 6.212 | 1.00 | 12.89 | C |

| ATOM | 3564 | C   | LEU C | 33 | 19.945 | 49.917 | 5.366   | 1.00 | 13.51 | C |
| ATOM | 3565 | O   | LEU C | 33 | 19.743 | 51.027 | 5.880   | 1.00 | 12.90 | O |
| ATOM | 3566 | CB  | LEU C | 33 | 19.163 | 48.175 | 6.985   | 1.00 | 12.94 | C |
| ATOM | 3567 | CG  | LEU C | 33 | 17.948 | 47.612 | 6.220   | 1.00 | 13.49 | C |
| ATOM | 3568 | CD1 | LEU C | 33 | 17.002 | 48.669 | 5.663   | 1.00 | 12.84 | C |
| ATOM | 3569 | CD2 | LEU C | 33 | 18.423 | 46.640 | 5.127   | 1.00 | 14.52 | C |
| ATOM | 3570 | N   | TYR C | 34 | 19.824 | 49.682 | 4.068   | 1.00 | 12.63 | N |
| ATOM | 3571 | CA  | TYR C | 34 | 19.519 | 50.743 | 3.118   | 1.00 | 12.27 | C |
| ATOM | 3572 | C   | TYR C | 34 | 18.348 | 50.329 | 2.248   | 1.00 | 11.96 | C |
| ATOM | 3573 | O   | TYR C | 34 | 18.194 | 49.157 | 1.922   | 1.00 | 11.75 | O |
| ATOM | 3574 | CB  | TYR C | 34 | 20.740 | 51.033 | 2.237   | 1.00 | 11.78 | C |
| ATOM | 3575 | CG  | TYR C | 34 | 21.945 | 51.579 | 2.969   | 1.00 | 11.06 | C |
| ATOM | 3576 | CD1 | TYR C | 34 | 22.670 | 50.796 | 3.880   | 1.00 | 9.78  | C |
| ATOM | 3577 | CD2 | TYR C | 34 | 22.400 | 52.863 | 2.717   | 1.00 | 12.10 | C |
| ATOM | 3578 | CE1 | TYR C | 34 | 23.751 | 51.311 | 4.586   | 1.00 | 12.20 | C |
| ATOM | 3579 | CE2 | TYR C | 34 | 23.511 | 53.383 | 3.402   | 1.00 | 12.10 | C |
| ATOM | 3580 | CZ  | TYR C | 34 | 24.178 | 52.614 | 4.316   | 1.00 | 13.98 | C |
| ATOM | 3581 | OH  | TYR C | 34 | 25.272 | 53.149 | 4.961   | 1.00 | 12.50 | O |
| ATOM | 3582 | N   | TRP C | 35 | 17.519 | 51.286 | 1.871   | 1.00 | 11.42 | N |
| ATOM | 3583 | CA  | TRP C | 35 | 16.454 | 51.063 | 0.907   | 1.00 | 11.38 | C |
| ATOM | 3584 | C   | TRP C | 35 | 16.674 | 51.959 | -0.328  | 1.00 | 12.01 | C |
| ATOM | 3585 | O   | TRP C | 35 | 16.919 | 53.161 | -0.200  | 1.00 | 12.50 | O |
| ATOM | 3586 | CB  | TRP C | 35 | 15.084 | 51.391 | 1.500   | 1.00 | 12.51 | C |
| ATOM | 3587 | CG  | TRP C | 35 | 14.579 | 50.465 | 2.594   | 1.00 | 13.15 | C |
| ATOM | 3588 | CD1 | TRP C | 35 | 14.567 | 50.711 | 3.936   | 1.00 | 13.26 | C |
| ATOM | 3589 | CD2 | TRP C | 35 | 13.981 | 49.176 | 2.423   | 1.00 | 13.97 | C |
| ATOM | 3590 | NE1 | TRP C | 35 | 14.013 | 49.651 | 4.620   | 1.00 | 13.97 | N |
| ATOM | 3591 | CE2 | TRP C | 35 | 13.657 | 48.688 | 3.715   | 1.00 | 12.65 | C |
| ATOM | 3592 | CE3 | TRP C | 35 | 13.724 | 48.367 | 1.314   | 1.00 | 14.62 | C |
| ATOM | 3593 | CZ2 | TRP C | 35 | 13.040 | 47.440 | 3.916   | 1.00 | 14.42 | C |
| ATOM | 3594 | CZ3 | TRP C | 35 | 13.105 | 47.153 | 1.515   | 1.00 | 14.74 | C |
| ATOM | 3595 | CH2 | TRP C | 35 | 12.789 | 46.691 | 2.808   | 1.00 | 14.12 | C |
| ATOM | 3596 | N   | TYR C | 36 | 16.546 | 51.365 | -1.506  | 1.00 | 9.28  | N |
| ATOM | 3597 | CA  | TYR C | 36 | 16.572 | 52.061 | -2.799  | 1.00 | 10.96 | C |
| ATOM | 3598 | C   | TYR C | 36 | 15.244 | 51.842 | -3.535  | 1.00 | 11.72 | C |
| ATOM | 3599 | O   | TYR C | 36 | 14.603 | 50.767 | -3.400  | 1.00 | 12.67 | O |
| ATOM | 3600 | CB  | TYR C | 36 | 17.752 | 51.554 | -3.679  | 1.00 | 11.38 | C |
| ATOM | 3601 | CG  | TYR C | 36 | 19.104 | 51.766 | -3.051  | 1.00 | 11.62 | C |
| ATOM | 3602 | CD1 | TYR C | 36 | 19.826 | 52.924 | -3.271  | 1.00 | 12.45 | C |
| ATOM | 3603 | CD2 | TYR C | 36 | 19.693 | 50.784 | -2.252  | 1.00 | 11.75 | C |
| ATOM | 3604 | CE1 | TYR C | 36 | 21.043 | 53.127 | -2.705  | 1.00 | 8.56  | C |
| ATOM | 3605 | CE2 | TYR C | 36 | 20.901 | 50.980 | -1.672  | 1.00 | 8.90  | C |
| ATOM | 3606 | CZ  | TYR C | 36 | 21.586 | 52.169 | -1.883  | 1.00 | 11.45 | C |
| ATOM | 3607 | OH  | TYR C | 36 | 22.786 | 52.404 | -1.306  | 1.00 | 11.19 | O |
| ATOM | 3608 | N   | LEU C | 37 | 14.819 | 52.858 | -4.296  | 1.00 | 10.93 | N |
| ATOM | 3609 | CA  | LEU C | 37 | 13.675 | 52.746 | -5.187  | 1.00 | 10.73 | C |
| ATOM | 3610 | C   | LEU C | 37 | 14.108 | 52.905 | -6.630  | 1.00 | 11.80 | C |
| ATOM | 3611 | O   | LEU C | 37 | 14.784 | 53.882 | -6.997  | 1.00 | 11.50 | O |
| ATOM | 3612 | CB  | LEU C | 37 | 12.610 | 53.783 | -4.849  | 1.00 | 11.51 | C |
| ATOM | 3613 | CG  | LEU C | 37 | 11.428 | 53.946 | -5.797  | 1.00 | 11.24 | C |
| ATOM | 3614 | CD1 | LEU C | 37 | 10.665 | 52.640 | -5.992  | 1.00 | 12.89 | C |
| ATOM | 3615 | CD2 | LEU C | 37 | 10.455 | 54.993 | -5.263  | 1.00 | 13.54 | C |
| ATOM | 3616 | N   | GLN C | 38 | 13.739 | 51.929 | -7.448  | 1.00 | 10.65 | N |
| ATOM | 3617 | CA  | GLN C | 38 | 13.887 | 52.031 | -8.887  | 1.00 | 14.54 | C |
| ATOM | 3618 | C   | GLN C | 38 | 12.507 | 52.177 | -9.556  | 1.00 | 16.75 | C |
| ATOM | 3619 | O   | GLN C | 38 | 11.740 | 51.210 | -9.669  | 1.00 | 15.67 | O |
| ATOM | 3620 | CB  | GLN C | 38 | 14.654 | 50.823 | -9.452  | 1.00 | 13.38 | C |
| ATOM | 3621 | CG  | GLN C | 38 | 14.978 | 50.963 | -10.917 | 1.00 | 15.66 | C |
| ATOM | 3622 | CD  | GLN C | 38 | 15.943 | 49.880 | -11.391 | 1.00 | 16.11 | C |
| ATOM | 3623 | OE1 | GLN C | 38 | 15.956 | 48.781 | -10.855 | 1.00 | 19.41 | O |
| ATOM | 3624 | NE2 | GLN C | 38 | 16.767 | 50.212 | -12.361 | 1.00 | 17.13 | N |
| ATOM | 3625 | N   | LYS C | 39 | 12.189 | 53.408 | -9.961  | 1.00 | 19.96 | N |
| ATOM | 3626 | CA  | LYS C | 39 | 10.963 | 53.672 | -10.706 | 1.00 | 23.86 | C |
| ATOM | 3627 | C   | LYS C | 39 | 11.130 | 53.170 | -12.149 | 1.00 | 25.59 | C |
| ATOM | 3628 | O   | LYS C | 39 | 12.254 | 53.056 | -12.648 | 1.00 | 24.75 | O |

| ATOM | 3629 | CB  | LYS | C | 39 | 10.583 | 55.168 | -10.645 | 1.00 | 23.74 | C |
| ATOM | 3630 | CG  | LYS | C | 39 | 10.173 | 55.642 | -9.248  | 1.00 | 25.92 | C |
| ATOM | 3631 | CD  | LYS | C | 39 | 9.840  | 57.120 | -9.175  | 1.00 | 26.50 | C |
| ATOM | 3632 | CE  | LYS | C | 39 | 9.330  | 57.525 | -7.787  | 1.00 | 27.24 | C |
| ATOM | 3633 | NZ  | LYS | C | 39 | 8.944  | 58.992 | -7.708  | 1.00 | 28.38 | N |
| ATOM | 3634 | N   | PRO | C | 40 | 10.008 | 52.846 | -12.826 | 1.00 | 28.65 | N |
| ATOM | 3635 | CA  | PRO | C | 40 | 10.069 | 52.406 | -14.215 | 1.00 | 29.35 | C |
| ATOM | 3636 | C   | PRO | C | 40 | 10.834 | 53.379 | -15.120 | 1.00 | 28.85 | C |
| ATOM | 3637 | O   | PRO | C | 40 | 10.573 | 54.584 | -15.109 | 1.00 | 30.10 | O |
| ATOM | 3638 | CB  | PRO | C | 40 | 8.594  | 52.363 | -14.639 | 1.00 | 30.00 | C |
| ATOM | 3639 | CG  | PRO | C | 40 | 7.838  | 52.208 | -13.392 | 1.00 | 30.61 | C |
| ATOM | 3640 | CD  | PRO | C | 40 | 8.623  | 52.894 | -12.324 | 1.00 | 29.40 | C |
| ATOM | 3641 | N   | GLY | C | 41 | 11.774 | 52.845 | -15.881 | 1.00 | 29.05 | N |
| ATOM | 3642 | CA  | GLY | C | 41 | 12.593 | 53.637 | -16.782 | 1.00 | 28.96 | C |
| ATOM | 3643 | C   | GLY | C | 41 | 13.766 | 54.359 | -16.140 | 1.00 | 28.30 | C |
| ATOM | 3644 | O   | GLY | C | 41 | 14.559 | 54.965 | -16.856 | 1.00 | 29.41 | O |
| ATOM | 3645 | N   | GLN | C | 42 | 13.891 | 54.295 | -14.811 | 1.00 | 26.09 | N |
| ATOM | 3646 | CA  | GLN | C | 42 | 14.914 | 55.064 | -14.087 | 1.00 | 24.85 | C |
| ATOM | 3647 | C   | GLN | C | 42 | 16.004 | 54.157 | -13.476 | 1.00 | 21.99 | C |
| ATOM | 3648 | O   | GLN | C | 42 | 15.886 | 52.920 | -13.463 | 1.00 | 20.39 | O |
| ATOM | 3649 | CB  | GLN | C | 42 | 14.279 | 55.910 | -12.967 | 1.00 | 25.61 | C |
| ATOM | 3650 | CG  | GLN | C | 42 | 13.198 | 56.938 | -13.361 | 1.00 | 27.26 | C |
| ATOM | 3651 | CD  | GLN | C | 42 | 12.741 | 57.844 | -12.172 | 1.00 | 28.15 | C |
| ATOM | 3652 | OE1 | GLN | C | 42 | 13.251 | 57.760 | -11.035 | 1.00 | 30.43 | O |
| ATOM | 3653 | NE2 | GLN | C | 42 | 11.771 | 58.728 | -12.448 | 1.00 | 30.87 | N |
| ATOM | 3654 | N   | SER | C | 43 | 17.058 | 54.796 | -12.974 | 1.00 | 20.05 | N |
| ATOM | 3655 | CA  | SER | C | 43 | 18.070 | 54.144 | -12.167 | 1.00 | 18.32 | C |
| ATOM | 3656 | C   | SER | C | 43 | 17.538 | 54.043 | -10.744 | 1.00 | 16.03 | C |
| ATOM | 3657 | O   | SER | C | 43 | 16.587 | 54.735 | -10.399 | 1.00 | 16.35 | O |
| ATOM | 3658 | CB  | SER | C | 43 | 19.363 | 54.955 | -12.172 | 1.00 | 19.11 | C |
| ATOM | 3659 | OG  | SER | C | 43 | 19.862 | 55.063 | -13.508 | 1.00 | 21.12 | O |
| ATOM | 3660 | N   | PRO | C | 44 | 18.125 | 53.154 | -9.919  | 1.00 | 13.38 | N |
| ATOM | 3661 | CA  | PRO | C | 44 | 17.745 | 53.149 | -8.501  | 1.00 | 13.12 | C |
| ATOM | 3662 | C   | PRO | C | 44 | 18.150 | 54.482 | -7.846  | 1.00 | 13.55 | C |
| ATOM | 3663 | O   | PRO | C | 44 | 19.132 | 55.122 | -8.278  | 1.00 | 12.23 | O |
| ATOM | 3664 | CB  | PRO | C | 44 | 18.556 | 51.993 | -7.905  | 1.00 | 13.56 | C |
| ATOM | 3665 | CG  | PRO | C | 44 | 19.054 | 51.193 | -9.137  | 1.00 | 13.49 | C |
| ATOM | 3666 | CD  | PRO | C | 44 | 19.151 | 52.148 | -10.257 | 1.00 | 12.99 | C |
| ATOM | 3667 | N   | HIS | C | 45 | 17.432 | 54.877 | -6.800  | 1.00 | 12.72 | N |
| ATOM | 3668 | CA  | HIS | C | 45 | 17.860 | 55.975 | -5.972  | 1.00 | 13.65 | C |
| ATOM | 3669 | C   | HIS | C | 45 | 17.663 | 55.666 | -4.510  | 1.00 | 13.04 | C |
| ATOM | 3670 | O   | HIS | C | 45 | 16.770 | 54.916 | -4.147  | 1.00 | 15.44 | O |
| ATOM | 3671 | CB  | HIS | C | 45 | 17.157 | 57.288 | -6.363  | 1.00 | 15.77 | C |
| ATOM | 3672 | CG  | HIS | C | 45 | 15.690 | 57.324 | -6.055  | 1.00 | 16.20 | C |
| ATOM | 3673 | ND1 | HIS | C | 45 | 14.727 | 56.859 | -6.929  | 1.00 | 20.12 | N |
| ATOM | 3674 | CD2 | HIS | C | 45 | 15.019 | 57.790 | -4.975  | 1.00 | 18.27 | C |
| ATOM | 3675 | CE1 | HIS | C | 45 | 13.528 | 57.051 | -6.404  | 1.00 | 18.76 | C |
| ATOM | 3676 | NE2 | HIS | C | 45 | 13.678 | 57.617 | -5.220  | 1.00 | 17.44 | N |
| ATOM | 3677 | N   | LEU | C | 46 | 18.515 | 56.257 | -3.684  | 1.00 | 13.20 | N |
| ATOM | 3678 | CA  | LEU | C | 46 | 18.511 | 56.053 | -2.235  | 1.00 | 12.33 | C |
| ATOM | 3679 | C   | LEU | C | 46 | 17.263 | 56.656 | -1.622  | 1.00 | 14.95 | C |
| ATOM | 3680 | O   | LEU | C | 46 | 16.955 | 57.841 | -1.869  | 1.00 | 12.52 | O |
| ATOM | 3681 | CB  | LEU | C | 46 | 19.752 | 56.701 | -1.599  | 1.00 | 12.36 | C |
| ATOM | 3682 | CG  | LEU | C | 46 | 19.899 | 56.493 | -0.093  | 1.00 | 9.53  | C |
| ATOM | 3683 | CD1 | LEU | C | 46 | 20.109 | 54.991 | 0.182   | 1.00 | 10.36 | C |
| ATOM | 3684 | CD2 | LEU | C | 46 | 21.049 | 57.255 | 0.487   | 1.00 | 12.50 | C |
| ATOM | 3685 | N   | LEU | C | 47 | 16.562 | 55.830 | -0.847  | 1.00 | 14.88 | N |
| ATOM | 3686 | CA  | LEU | C | 47 | 15.399 | 56.238 | -0.053  | 1.00 | 15.66 | C |
| ATOM | 3687 | C   | LEU | C | 47 | 15.724 | 56.411 | 1.406   | 1.00 | 17.14 | C |
| ATOM | 3688 | O   | LEU | C | 47 | 15.499 | 57.486 | 1.998   | 1.00 | 15.53 | O |
| ATOM | 3689 | CB  | LEU | C | 47 | 14.290 | 55.193 | -0.140  | 1.00 | 18.55 | C |
| ATOM | 3690 | CG  | LEU | C | 47 | 13.311 | 55.183 | -1.277  | 1.00 | 22.21 | C |
| ATOM | 3691 | CD1 | LEU | C | 47 | 12.310 | 54.105 | -0.917  | 1.00 | 23.96 | C |
| ATOM | 3692 | CD2 | LEU | C | 47 | 12.603 | 56.526 | -1.490  | 1.00 | 23.29 | C |
| ATOM | 3693 | N   | ILE | C | 48 | 16.252 | 55.340 | 2.007   | 1.00 | 15.30 | N |

| ATOM | 3694 | CA | ILE | C | 48 | 16.545 | 55.306 | 3.421 | 1.00 | 15.87 | C |
|------|------|-----|-----|---|----|--------|--------|--------|------|-------|---|
| ATOM | 3695 | C | ILE | C | 48 | 17.941 | 54.750 | 3.636 | 1.00 | 15.04 | C |
| ATOM | 3696 | O | ILE | C | 48 | 18.314 | 53.774 | 2.999 | 1.00 | 14.71 | O |
| ATOM | 3697 | CB | ILE | C | 48 | 15.567 | 54.360 | 4.178 | 1.00 | 16.87 | C |
| ATOM | 3698 | CG1 | ILE | C | 48 | 14.115 | 54.796 | 4.065 | 1.00 | 20.88 | C |
| ATOM | 3699 | CG2 | ILE | C | 48 | 15.984 | 54.137 | 5.641 | 1.00 | 16.67 | C |
| ATOM | 3700 | CD1 | ILE | C | 48 | 13.807 | 56.010 | 4.703 | 1.00 | 21.10 | C |
| ATOM | 3701 | N | TYR | C | 49 | 18.673 | 55.347 | 4.572 | 1.00 | 13.64 | N |
| ATOM | 3702 | CA | TYR | C | 49 | 19.992 | 54.876 | 4.986 | 1.00 | 14.31 | C |
| ATOM | 3703 | C | TYR | C | 49 | 20.040 | 54.653 | 6.505 | 1.00 | 14.11 | C |
| ATOM | 3704 | O | TYR | C | 49 | 19.214 | 55.194 | 7.259 | 1.00 | 14.72 | O |
| ATOM | 3705 | CB | TYR | C | 49 | 21.103 | 55.812 | 4.527 | 1.00 | 16.24 | C |
| ATOM | 3706 | CG | TYR | C | 49 | 21.102 | 57.170 | 5.208 | 1.00 | 16.26 | C |
| ATOM | 3707 | CD1 | TYR | C | 49 | 20.318 | 58.209 | 4.717 | 1.00 | 17.84 | C |
| ATOM | 3708 | CD2 | TYR | C | 49 | 21.876 | 57.387 | 6.344 | 1.00 | 17.09 | C |
| ATOM | 3709 | CE1 | TYR | C | 49 | 20.323 | 59.472 | 5.336 | 1.00 | 16.99 | C |
| ATOM | 3710 | CE2 | TYR | C | 49 | 21.863 | 58.664 | 6.992 | 1.00 | 17.33 | C |
| ATOM | 3711 | CZ | TYR | C | 49 | 21.089 | 59.665 | 6.460 | 1.00 | 18.41 | C |
| ATOM | 3712 | OH | TYR | C | 49 | 21.084 | 60.895 | 7.078 | 1.00 | 19.33 | O |
| ATOM | 3713 | N | HIS | C | 50 | 20.982 | 53.811 | 6.922 | 1.00 | 14.25 | N |
| ATOM | 3714 | CA | HIS | C | 50 | 21.201 | 53.482 | 8.325 | 1.00 | 13.85 | C |
| ATOM | 3715 | C | HIS | C | 50 | 19.878 | 53.105 | 8.978 | 1.00 | 13.96 | C |
| ATOM | 3716 | O | HIS | C | 50 | 19.529 | 53.613 | 10.054 | 1.00 | 12.23 | O |
| ATOM | 3717 | CB | HIS | C | 50 | 21.875 | 54.651 | 9.074 | 1.00 | 14.16 | C |
| ATOM | 3718 | CG | HIS | C | 50 | 23.287 | 54.923 | 8.649 | 1.00 | 16.54 | C |
| ATOM | 3719 | ND1 | HIS | C | 50 | 24.031 | 55.960 | 9.172 | 1.00 | 16.22 | N |
| ATOM | 3720 | CD2 | HIS | C | 50 | 24.089 | 54.301 | 7.753 | 1.00 | 17.08 | C |
| ATOM | 3721 | CE1 | HIS | C | 50 | 25.232 | 55.956 | 8.620 | 1.00 | 18.02 | C |
| ATOM | 3722 | NE2 | HIS | C | 50 | 25.295 | 54.953 | 7.762 | 1.00 | 17.35 | N |
| ATOM | 3723 | N | LEU | C | 51 | 19.132 | 52.241 | 8.285 | 1.00 | 13.11 | N |
| ATOM | 3724 | CA | LEU | C | 51 | 17.861 | 51.670 | 8.733 | 1.00 | 13.10 | C |
| ATOM | 3725 | C | LEU | C | 51 | 16.651 | 52.603 | 8.737 | 1.00 | 14.11 | C |
| ATOM | 3726 | O | LEU | C | 51 | 15.577 | 52.228 | 8.258 | 1.00 | 14.19 | O |
| ATOM | 3727 | CB | LEU | C | 51 | 18.008 | 50.956 | 10.078 | 1.00 | 13.76 | C |
| ATOM | 3728 | CG | LEU | C | 51 | 16.764 | 50.253 | 10.616 | 1.00 | 12.63 | C |
| ATOM | 3729 | CD1 | LEU | C | 51 | 16.363 | 49.081 | 9.683 | 1.00 | 15.14 | C |
| ATOM | 3730 | CD2 | LEU | C | 51 | 16.925 | 49.772 | 12.027 | 1.00 | 13.42 | C |
| ATOM | 3731 | N | SER | C | 52 | 16.814 | 53.816 | 9.256 | 1.00 | 14.37 | N |
| ATOM | 3732 | CA | SER | C | 52 | 15.640 | 54.666 | 9.531 | 1.00 | 16.01 | C |
| ATOM | 3733 | C | SER | C | 52 | 15.772 | 56.123 | 9.080 | 1.00 | 17.49 | C |
| ATOM | 3734 | O | SER | C | 52 | 14.843 | 56.912 | 9.266 | 1.00 | 17.90 | O |
| ATOM | 3735 | CB | SER | C | 52 | 15.408 | 54.603 | 11.021 | 1.00 | 16.09 | C |
| ATOM | 3736 | OG | SER | C | 52 | 14.953 | 53.292 | 11.339 | 1.00 | 19.95 | O |
| ATOM | 3737 | N | ASN | C | 53 | 16.889 | 56.464 | 8.448 | 1.00 | 15.77 | N |
| ATOM | 3738 | CA | ASN | C | 53 | 17.142 | 57.837 | 8.038 | 1.00 | 16.95 | C |
| ATOM | 3739 | C | ASN | C | 53 | 16.770 | 58.128 | 6.599 | 1.00 | 17.61 | C |
| ATOM | 3740 | O | ASN | C | 53 | 17.186 | 57.453 | 5.692 | 1.00 | 17.01 | O |
| ATOM | 3741 | CB | ASN | C | 53 | 18.595 | 58.201 | 8.319 | 1.00 | 18.55 | C |
| ATOM | 3742 | CG | ASN | C | 53 | 18.918 | 58.070 | 9.770 | 1.00 | 22.15 | C |
| ATOM | 3743 | OD1 | ASN | C | 53 | 18.229 | 58.641 | 10.603 | 1.00 | 27.73 | O |
| ATOM | 3744 | ND2 | ASN | C | 53 | 19.911 | 57.260 | 10.101 | 1.00 | 26.62 | N |
| ATOM | 3745 | N | LEU | C | 54 | 15.995 | 59.190 | 6.389 | 1.00 | 18.21 | N |
| ATOM | 3746 | CA | LEU | C | 54 | 15.517 | 59.510 | 5.048 | 1.00 | 19.09 | C |
| ATOM | 3747 | C | LEU | C | 54 | 16.579 | 60.265 | 4.278 | 1.00 | 18.93 | C |
| ATOM | 3748 | O | LEU | C | 54 | 17.279 | 61.131 | 4.842 | 1.00 | 18.98 | O |
| ATOM | 3749 | CB | LEU | C | 54 | 14.184 | 60.295 | 5.101 | 1.00 | 19.96 | C |
| ATOM | 3750 | CG | LEU | C | 54 | 12.889 | 59.491 | 5.205 | 1.00 | 20.20 | C |
| ATOM | 3751 | CD1 | LEU | C | 54 | 12.841 | 58.716 | 6.481 | 1.00 | 22.60 | C |
| ATOM | 3752 | CD2 | LEU | C | 54 | 11.681 | 60.402 | 5.138 | 1.00 | 22.75 | C |
| ATOM | 3753 | N | ALA | C | 55 | 16.733 | 59.913 | 3.008 | 1.00 | 17.65 | N |
| ATOM | 3754 | CA | ALA | C | 55 | 17.658 | 60.612 | 2.130 | 1.00 | 18.89 | C |
| ATOM | 3755 | C | ALA | C | 55 | 17.060 | 61.985 | 1.813 | 1.00 | 20.81 | C |
| ATOM | 3756 | O | ALA | C | 55 | 15.852 | 62.192 | 1.942 | 1.00 | 19.16 | O |
| ATOM | 3757 | CB | ALA | C | 55 | 17.887 | 59.821 | 0.850 | 1.00 | 18.96 | C |
| ATOM | 3758 | N | SER | C | 56 | 17.930 | 62.904 | 1.412 | 1.00 | 23.94 | N |

| ATOM | 3759 | CA | SER | C | 56 | 17.525 | 64.281 | 1.115 | 1.00 | 25.97 | C |
|------|------|-----|-----|---|----|--------|--------|--------|------|-------|---|
| ATOM | 3760 | C | SER | C | 56 | 16.381 | 64.294 | 0.113 | 1.00 | 24.90 | C |
| ATOM | 3761 | O | SER | C | 56 | 16.497 | 63.735 | -0.960 | 1.00 | 24.30 | O |
| ATOM | 3762 | CB | SER | C | 56 | 18.711 | 65.059 | 0.556 | 1.00 | 26.48 | C |
| ATOM | 3763 | OG | SER | C | 56 | 18.305 | 66.350 | 0.155 | 1.00 | 32.51 | O |
| ATOM | 3764 | N | GLY | C | 57 | 15.263 | 64.914 | 0.487 | 1.00 | 25.78 | N |
| ATOM | 3765 | CA | GLY | C | 57 | 14.127 | 65.085 | -0.413 | 1.00 | 24.53 | C |
| ATOM | 3766 | C | GLY | C | 57 | 13.087 | 63.990 | -0.413 | 1.00 | 24.08 | C |
| ATOM | 3767 | O | GLY | C | 57 | 12.061 | 64.102 | -1.073 | 1.00 | 24.68 | O |
| ATOM | 3768 | N | VAL | C | 58 | 13.335 | 62.917 | 0.335 | 1.00 | 22.77 | N |
| ATOM | 3769 | CA | VAL | C | 58 | 12.370 | 61.839 | 0.437 | 1.00 | 21.39 | C |
| ATOM | 3770 | C | VAL | C | 58 | 11.251 | 62.274 | 1.372 | 1.00 | 21.58 | C |
| ATOM | 3771 | O | VAL | C | 58 | 11.537 | 62.752 | 2.456 | 1.00 | 20.44 | O |
| ATOM | 3772 | CB | VAL | C | 58 | 13.039 | 60.537 | 0.956 | 1.00 | 19.57 | C |
| ATOM | 3773 | CG1 | VAL | C | 58 | 12.015 | 59.452 | 1.164 | 1.00 | 20.14 | C |
| ATOM | 3774 | CG2 | VAL | C | 58 | 14.103 | 60.108 | -0.026 | 1.00 | 19.74 | C |
| ATOM | 3775 | N | PRO | C | 59 | 9.985 | 62.119 | 0.940 | 1.00 | 23.45 | N |
| ATOM | 3776 | CA | PRO | C | 59 | 8.853 | 62.493 | 1.773 | 1.00 | 24.73 | C |
| ATOM | 3777 | C | PRO | C | 59 | 8.767 | 61.681 | 3.059 | 1.00 | 26.37 | C |
| ATOM | 3778 | O | PRO | C | 59 | 9.136 | 60.492 | 3.094 | 1.00 | 23.75 | O |
| ATOM | 3779 | CB | PRO | C | 59 | 7.630 | 62.221 | 0.891 | 1.00 | 25.35 | C |
| ATOM | 3780 | CG | PRO | C | 59 | 8.111 | 61.831 | -0.411 | 1.00 | 25.44 | C |
| ATOM | 3781 | CD | PRO | C | 59 | 9.570 | 61.604 | -0.372 | 1.00 | 24.02 | C |
| ATOM | 3782 | N | ASP | C | 60 | 8.264 | 62.322 | 4.113 | 1.00 | 27.87 | N |
| ATOM | 3783 | CA | ASP | C | 60 | 8.179 | 61.666 | 5.410 | 1.00 | 28.43 | C |
| ATOM | 3784 | C | ASP | C | 60 | 7.060 | 60.627 | 5.509 | 1.00 | 26.42 | C |
| ATOM | 3785 | O | ASP | C | 60 | 6.858 | 60.055 | 6.565 | 1.00 | 27.44 | O |
| ATOM | 3786 | CB | ASP | C | 60 | 8.086 | 62.700 | 6.529 | 1.00 | 30.67 | C |
| ATOM | 3787 | CG | ASP | C | 60 | 6.838 | 63.520 | 6.474 | 1.00 | 32.95 | C |
| ATOM | 3788 | OD1 | ASP | C | 60 | 5.917 | 63.246 | 5.662 | 1.00 | 33.77 | O |
| ATOM | 3789 | OD2 | ASP | C | 60 | 6.795 | 64.491 | 7.265 | 1.00 | 40.04 | O |
| ATOM | 3790 | N | ARG | C | 61 | 6.358 | 60.379 | 4.409 | 1.00 | 25.56 | N |
| ATOM | 3791 | CA | ARG | C | 61 | 5.458 | 59.224 | 4.268 | 1.00 | 25.12 | C |
| ATOM | 3792 | C | ARG | C | 61 | 6.223 | 57.901 | 4.344 | 1.00 | 22.26 | C |
| ATOM | 3793 | O | ARG | C | 61 | 5.653 | 56.845 | 4.642 | 1.00 | 21.74 | O |
| ATOM | 3794 | CB | ARG | C | 61 | 4.744 | 59.288 | 2.914 | 1.00 | 25.88 | C |
| ATOM | 3795 | CG | ARG | C | 61 | 3.857 | 60.525 | 2.755 | 1.00 | 27.57 | C |
| ATOM | 3796 | CD | ARG | C | 61 | 3.017 | 60.453 | 1.489 | 1.00 | 27.76 | C |
| ATOM | 3797 | NE | ARG | C | 61 | 3.820 | 60.529 | 0.268 | 1.00 | 27.81 | N |
| ATOM | 3798 | CZ | ARG | C | 61 | 4.107 | 59.504 | -0.526 | 1.00 | 27.76 | C |
| ATOM | 3799 | NH1 | ARG | C | 61 | 3.661 | 58.290 | -0.244 | 1.00 | 27.27 | N |
| ATOM | 3800 | NH2 | ARG | C | 61 | 4.827 | 59.713 | -1.625 | 1.00 | 28.19 | N |
| ATOM | 3801 | N | PHE | C | 62 | 7.506 | 57.967 | 4.006 | 1.00 | 20.99 | N |
| ATOM | 3802 | CA | PHE | C | 62 | 8.392 | 56.796 | 4.052 | 1.00 | 19.30 | C |
| ATOM | 3803 | C | PHE | C | 62 | 9.024 | 56.682 | 5.418 | 1.00 | 18.02 | C |
| ATOM | 3804 | O | PHE | C | 62 | 9.516 | 57.670 | 5.988 | 1.00 | 18.26 | O |
| ATOM | 3805 | CB | PHE | C | 62 | 9.454 | 56.896 | 2.962 | 1.00 | 18.85 | C |
| ATOM | 3806 | CG | PHE | C | 62 | 8.886 | 56.818 | 1.575 | 1.00 | 19.57 | C |
| ATOM | 3807 | CD1 | PHE | C | 62 | 8.503 | 57.967 | 0.909 | 1.00 | 20.34 | C |
| ATOM | 3808 | CD2 | PHE | C | 62 | 8.719 | 55.603 | 0.944 | 1.00 | 19.52 | C |
| ATOM | 3809 | CE1 | PHE | C | 62 | 7.965 | 57.905 | -0.364 | 1.00 | 21.16 | C |
| ATOM | 3810 | CE2 | PHE | C | 62 | 8.198 | 55.543 | -0.348 | 1.00 | 21.43 | C |
| ATOM | 3811 | CZ | PHE | C | 62 | 7.826 | 56.708 | -0.997 | 1.00 | 19.73 | C |
| ATOM | 3812 | N | SER | C | 63 | 9.017 | 55.471 | 5.972 | 1.00 | 16.44 | N |
| ATOM | 3813 | CA | SER | C | 63 | 9.669 | 55.245 | 7.248 | 1.00 | 15.19 | C |
| ATOM | 3814 | C | SER | C | 63 | 10.106 | 53.795 | 7.298 | 1.00 | 14.83 | C |
| ATOM | 3815 | O | SER | C | 63 | 9.572 | 52.947 | 6.586 | 1.00 | 14.64 | O |
| ATOM | 3816 | CB | SER | C | 63 | 8.754 | 55.554 | 8.432 | 1.00 | 14.89 | C |
| ATOM | 3817 | OG | SER | C | 63 | 7.624 | 54.730 | 8.437 | 1.00 | 17.85 | O |
| ATOM | 3818 | N | SER | C | 64 | 11.085 | 53.519 | 8.143 | 1.00 | 14.73 | N |
| ATOM | 3819 | CA | SER | C | 64 | 11.684 | 52.171 | 8.194 | 1.00 | 13.88 | C |
| ATOM | 3820 | C | SER | C | 64 | 12.166 | 51.818 | 9.595 | 1.00 | 12.80 | C |
| ATOM | 3821 | O | SER | C | 64 | 12.611 | 52.666 | 10.360 | 1.00 | 12.52 | O |
| ATOM | 3822 | CB | SER | C | 64 | 12.825 | 52.070 | 7.161 | 1.00 | 15.09 | C |
| ATOM | 3823 | OG | SER | C | 64 | 13.539 | 50.824 | 7.248 | 1.00 | 13.03 | O |

| ATOM | 3824 | N | SER | C | 65 | 12.026 | 50.554 | 9.955 | 1.00 | 12.90 | N |
| ATOM | 3825 | CA | SER | C | 65 | 12.394 | 50.081 | 11.276 | 1.00 | 12.21 | C |
| ATOM | 3826 | C | SER | C | 65 | 12.978 | 48.672 | 11.104 | 1.00 | 11.24 | C |
| ATOM | 3827 | O | SER | C | 65 | 12.808 | 48.071 | 10.059 | 1.00 | 11.35 | O |
| ATOM | 3828 | CB | SER | C | 65 | 11.173 | 50.048 | 12.206 | 1.00 | 13.22 | C |
| ATOM | 3829 | OG | SER | C | 65 | 10.160 | 49.150 | 11.732 | 1.00 | 12.32 | O |
| ATOM | 3830 | N | GLY | C | 66 | 13.630 | 48.163 | 12.135 | 1.00 | 11.24 | N |
| ATOM | 3831 | CA | GLY | C | 66 | 14.135 | 46.798 | 12.109 | 1.00 | 11.14 | C |
| ATOM | 3832 | C | GLY | C | 66 | 15.137 | 46.462 | 13.191 | 1.00 | 12.69 | C |
| ATOM | 3833 | O | GLY | C | 66 | 15.663 | 47.326 | 13.886 | 1.00 | 12.60 | O |
| ATOM | 3834 | N | SER | C | 67 | 15.415 | 45.173 | 13.308 | 1.00 | 11.12 | N |
| ATOM | 3835 | CA | SER | C | 67 | 16.494 | 44.687 | 14.132 | 1.00 | 12.94 | C |
| ATOM | 3836 | C | SER | C | 67 | 17.749 | 44.648 | 13.291 | 1.00 | 14.27 | C |
| ATOM | 3837 | O | SER | C | 67 | 17.839 | 45.309 | 12.248 | 1.00 | 15.82 | O |
| ATOM | 3838 | CB | SER | C | 67 | 16.125 | 43.297 | 14.632 | 1.00 | 12.96 | C |
| ATOM | 3839 | OG | SER | C | 67 | 16.020 | 42.426 | 13.536 | 1.00 | 13.73 | O |
| ATOM | 3840 | N | GLY | C | 68 | 18.730 | 43.850 | 13.708 | 1.00 | 14.48 | N |
| ATOM | 3841 | CA | GLY | C | 68 | 19.868 | 43.605 | 12.870 | 1.00 | 14.44 | C |
| ATOM | 3842 | C | GLY | C | 68 | 19.584 | 42.703 | 11.683 | 1.00 | 13.52 | C |
| ATOM | 3843 | O | GLY | C | 68 | 20.392 | 42.683 | 10.751 | 1.00 | 13.09 | O |
| ATOM | 3844 | N | THR | C | 69 | 18.474 | 41.955 | 11.723 | 1.00 | 13.27 | N |
| ATOM | 3845 | CA | THR | C | 69 | 18.181 | 40.906 | 10.723 | 1.00 | 13.86 | C |
| ATOM | 3846 | C | THR | C | 69 | 16.821 | 40.965 | 10.007 | 1.00 | 12.78 | C |
| ATOM | 3847 | O | THR | C | 69 | 16.652 | 40.342 | 8.962 | 1.00 | 12.64 | O |
| ATOM | 3848 | CB | THR | C | 69 | 18.365 | 39.520 | 11.357 | 1.00 | 13.40 | C |
| ATOM | 3849 | OG1 | THR | C | 69 | 17.485 | 39.382 | 12.462 | 1.00 | 15.70 | O |
| ATOM | 3850 | CG2 | THR | C | 69 | 19.806 | 39.343 | 11.839 | 1.00 | 17.25 | C |
| ATOM | 3851 | N | ASP | C | 70 | 15.850 | 41.689 | 10.557 | 1.00 | 13.95 | N |
| ATOM | 3852 | CA | ASP | C | 70 | 14.513 | 41.772 | 10.008 | 1.00 | 14.40 | C |
| ATOM | 3853 | C | ASP | C | 70 | 14.122 | 43.263 | 9.936 | 1.00 | 14.74 | C |
| ATOM | 3854 | O | ASP | C | 70 | 14.318 | 44.003 | 10.904 | 1.00 | 14.44 | O |
| ATOM | 3855 | CB | ASP | C | 70 | 13.519 | 40.976 | 10.880 | 1.00 | 16.36 | C |
| ATOM | 3856 | CG | ASP | C | 70 | 13.818 | 39.505 | 10.912 | 1.00 | 20.56 | C |
| ATOM | 3857 | OD1 | ASP | C | 70 | 13.481 | 38.806 | 9.931 | 1.00 | 20.65 | O |
| ATOM | 3858 | OD2 | ASP | C | 70 | 14.365 | 39.023 | 11.927 | 1.00 | 22.32 | O |
| ATOM | 3859 | N | PHE | C | 71 | 13.621 | 43.704 | 8.777 | 1.00 | 12.95 | N |
| ATOM | 3860 | CA | PHE | C | 71 | 13.396 | 45.132 | 8.499 | 1.00 | 14.09 | C |
| ATOM | 3861 | C | PHE | C | 71 | 12.055 | 45.309 | 7.790 | 1.00 | 14.15 | C |
| ATOM | 3862 | O | PHE | C | 71 | 11.537 | 44.374 | 7.160 | 1.00 | 13.25 | O |
| ATOM | 3863 | CB | PHE | C | 71 | 14.538 | 45.713 | 7.634 | 1.00 | 12.95 | C |
| ATOM | 3864 | CG | PHE | C | 71 | 15.866 | 45.036 | 7.853 | 1.00 | 13.26 | C |
| ATOM | 3865 | CD1 | PHE | C | 71 | 16.629 | 45.302 | 8.982 | 1.00 | 11.59 | C |
| ATOM | 3866 | CD2 | PHE | C | 71 | 16.343 | 44.120 | 6.926 | 1.00 | 12.76 | C |
| ATOM | 3867 | CE1 | PHE | C | 71 | 17.843 | 44.659 | 9.197 | 1.00 | 13.35 | C |
| ATOM | 3868 | CE2 | PHE | C | 71 | 17.551 | 43.470 | 7.125 | 1.00 | 14.34 | C |
| ATOM | 3869 | CZ | PHE | C | 71 | 18.311 | 43.729 | 8.255 | 1.00 | 11.82 | C |
| ATOM | 3870 | N | THR | C | 72 | 11.484 | 46.503 | 7.938 | 1.00 | 13.10 | N |
| ATOM | 3871 | CA | THR | C | 72 | 10.167 | 46.807 | 7.410 | 1.00 | 13.90 | C |
| ATOM | 3872 | C | THR | C | 72 | 10.174 | 48.261 | 6.927 | 1.00 | 13.09 | C |
| ATOM | 3873 | O | THR | C | 72 | 10.422 | 49.184 | 7.723 | 1.00 | 14.76 | O |
| ATOM | 3874 | CB | THR | C | 72 | 9.057 | 46.652 | 8.467 | 1.00 | 14.58 | C |
| ATOM | 3875 | OG1 | THR | C | 72 | 9.064 | 45.322 | 9.007 | 1.00 | 16.68 | O |
| ATOM | 3876 | CG2 | THR | C | 72 | 7.677 | 46.943 | 7.869 | 1.00 | 15.68 | C |
| ATOM | 3877 | N | LEU | C | 73 | 9.934 | 48.435 | 5.626 | 1.00 | 13.47 | N |
| ATOM | 3878 | CA | LEU | C | 73 | 9.683 | 49.742 | 5.027 | 1.00 | 14.07 | C |
| ATOM | 3879 | C | LEU | C | 73 | 8.188 | 49.995 | 5.056 | 1.00 | 14.72 | C |
| ATOM | 3880 | O | LEU | C | 73 | 7.404 | 49.156 | 4.587 | 1.00 | 14.55 | O |
| ATOM | 3881 | CB | LEU | C | 73 | 10.169 | 49.761 | 3.569 | 1.00 | 13.77 | C |
| ATOM | 3882 | CG | LEU | C | 73 | 9.966 | 51.062 | 2.784 | 1.00 | 13.91 | C |
| ATOM | 3883 | CD1 | LEU | C | 73 | 10.692 | 52.271 | 3.356 | 1.00 | 14.20 | C |
| ATOM | 3884 | CD2 | LEU | C | 73 | 10.353 | 50.862 | 1.343 | 1.00 | 15.63 | C |
| ATOM | 3885 | N | ARG | C | 74 | 7.782 | 51.150 | 5.574 | 1.00 | 15.98 | N |
| ATOM | 3886 | CA | ARG | C | 74 | 6.375 | 51.543 | 5.545 | 1.00 | 17.43 | C |
| ATOM | 3887 | C | ARG | C | 74 | 6.206 | 52.792 | 4.712 | 1.00 | 16.61 | C |
| ATOM | 3888 | O | ARG | C | 74 | 7.000 | 53.715 | 4.819 | 1.00 | 17.23 | O |

| ATOM | 3889 | CB  | AARG | C | 74 | 5.779   | 51.749 | 6.935   | 0.50 | 17.62 | C |
|------|------|-----|------|---|----|---------|--------|---------|------|-------|---|
| ATOM | 3890 | CB  | BARG | C | 74 | 5.884   | 51.801 | 6.975   | 0.50 | 17.92 | C |
| ATOM | 3891 | CG  | AARG | C | 74 | 4.241   | 51.800 | 6.900   | 0.50 | 17.67 | C |
| ATOM | 3892 | CG  | BARG | C | 74 | 4.523   | 52.474 | 7.067   | 0.50 | 18.62 | C |
| ATOM | 3893 | CD  | AARG | C | 74 | 3.642   | 51.333 | 8.186   | 0.50 | 17.92 | C |
| ATOM | 3894 | CD  | BARG | C | 74 | 3.934   | 52.391 | 8.458   | 0.50 | 19.65 | C |
| ATOM | 3895 | NE  | AARG | C | 74 | 2.189   | 51.520 | 8.252   | 0.50 | 15.65 | N |
| ATOM | 3896 | NE  | BARG | C | 74 | 2.553   | 52.878 | 8.466   | 0.50 | 20.56 | N |
| ATOM | 3897 | CZ  | AARG | C | 74 | 1.291   | 50.546 | 8.175   | 0.50 | 14.45 | C |
| ATOM | 3898 | CZ  | BARG | C | 74 | 2.207   | 54.160 | 8.570   | 0.50 | 20.74 | C |
| ATOM | 3899 | NH1 | AARG | C | 74 | 1.674   | 49.297 | 8.010   | 0.50 | 14.29 | N |
| ATOM | 3900 | NH1 | BARG | C | 74 | 3.137   | 55.111 | 8.688   | 0.50 | 20.12 | N |
| ATOM | 3901 | NH2 | AARG | C | 74 | -0.008  | 50.819 | 8.267   | 0.50 | 16.75 | N |
| ATOM | 3902 | NH2 | BARG | C | 74 | 0.923   | 54.492 | 8.560   | 0.50 | 21.53 | N |
| ATOM | 3903 | N   | ILE  | C | 75 | 5.150   | 52.798 | 3.906   | 1.00 | 17.44 | N |
| ATOM | 3904 | CA  | ILE  | C | 75 | 4.781   | 53.945 | 3.073   | 1.00 | 19.23 | C |
| ATOM | 3905 | C   | ILE  | C | 75 | 3.358   | 54.317 | 3.436   | 1.00 | 21.69 | C |
| ATOM | 3906 | O   | ILE  | C | 75 | 2.441   | 53.589 | 3.130   | 1.00 | 22.71 | O |
| ATOM | 3907 | CB  | ILE  | C | 75 | 4.833   | 53.624 | 1.558   | 1.00 | 18.81 | C |
| ATOM | 3908 | CG1 | ILE  | C | 75 | 6.116   | 52.885 | 1.208   | 1.00 | 17.68 | C |
| ATOM | 3909 | CG2 | ILE  | C | 75 | 4.689   | 54.914 | 0.734   | 1.00 | 17.85 | C |
| ATOM | 3910 | CD1 | ILE  | C | 75 | 6.161   | 52.353 | -0.225  | 1.00 | 18.60 | C |
| ATOM | 3911 | N   | SER  | C | 76 | 3.183   | 55.434 | 4.131   | 1.00 | 25.05 | N |
| ATOM | 3912 | CA  | SER  | C | 76 | 1.841   | 55.910 | 4.447   | 1.00 | 28.02 | C |
| ATOM | 3913 | C   | SER  | C | 76 | 1.268   | 56.681 | 3.246   | 1.00 | 28.67 | C |
| ATOM | 3914 | O   | SER  | C | 76 | 2.014   | 57.211 | 2.416   | 1.00 | 29.39 | O |
| ATOM | 3915 | CB  | SER  | C | 76 | 1.889   | 56.787 | 5.694   | 1.00 | 29.01 | C |
| ATOM | 3916 | OG  | SER  | C | 76 | 2.399   | 58.062 | 5.383   | 1.00 | 30.13 | O |
| ATOM | 3917 | N   | ARG  | C | 77 | -0.055  | 56.721 | 3.144   | 1.00 | 29.28 | N |
| ATOM | 3918 | CA  | ARG  | C | 77 | -0.740  | 57.507 | 2.127   | 1.00 | 30.26 | C |
| ATOM | 3919 | C   | ARG  | C | 77 | -0.061  | 57.315 | 0.780   | 1.00 | 27.93 | C |
| ATOM | 3920 | O   | ARG  | C | 77 | 0.396   | 58.277 | 0.152   | 1.00 | 27.63 | O |
| ATOM | 3921 | CB  | ARG  | C | 77 | -0.775  | 58.995 | 2.510   | 1.00 | 31.07 | C |
| ATOM | 3922 | CG  | ARG  | C | 77 | -1.488  | 59.274 | 3.841   | 1.00 | 33.41 | C |
| ATOM | 3923 | CD  | ARG  | C | 77 | -1.380  | 60.748 | 4.267   | 1.00 | 35.39 | C |
| ATOM | 3924 | NE  | ARG  | C | 77 | -0.016  | 61.199 | 4.580   | 1.00 | 38.54 | N |
| ATOM | 3925 | CZ  | ARG  | C | 77 | 0.695   | 60.825 | 5.651   | 1.00 | 40.00 | C |
| ATOM | 3926 | NH1 | ARG  | C | 77 | 0.204   | 59.962 | 6.546   | 1.00 | 41.16 | N |
| ATOM | 3927 | NH2 | ARG  | C | 77 | 1.920   | 61.313 | 5.836   | 1.00 | 41.16 | N |
| ATOM | 3928 | N   | VAL  | C | 78 | -0.015  | 56.064 | 0.337   | 1.00 | 26.40 | N |
| ATOM | 3929 | CA  | VAL  | C | 78 | 0.618   | 55.738 | -0.937  | 1.00 | 26.23 | C |
| ATOM | 3930 | C   | VAL  | C | 78 | 0.019   | 56.572 | -2.080  | 1.00 | 26.18 | C |
| ATOM | 3931 | O   | VAL  | C | 78 | -1.210  | 56.734 | -2.183  | 1.00 | 25.83 | O |
| ATOM | 3932 | CB  | VAL  | C | 78 | 0.473   | 54.235 | -1.289  | 1.00 | 25.68 | C |
| ATOM | 3933 | CG1 | VAL  | C | 78 | 0.847   | 53.975 | -2.739  | 1.00 | 27.48 | C |
| ATOM | 3934 | CG2 | VAL  | C | 78 | 1.351   | 53.349 | -0.393  | 1.00 | 24.79 | C |
| ATOM | 3935 | N   | GLU  | C | 79 | 0.902   | 57.067 | -2.943  | 1.00 | 25.29 | N |
| ATOM | 3936 | CA  | GLU  | C | 79 | 0.528   | 57.801 | -4.147  | 1.00 | 25.51 | C |
| ATOM | 3937 | C   | GLU  | C | 79 | 0.860   | 56.991 | -5.379  | 1.00 | 24.57 | C |
| ATOM | 3938 | O   | GLU  | C | 79 | 1.708   | 56.092 | -5.347  | 1.00 | 21.86 | O |
| ATOM | 3939 | CB  | GLU  | C | 79 | 1.319   | 59.093 | -4.213  | 1.00 | 25.02 | C |
| ATOM | 3940 | CG  | GLU  | C | 79 | 1.149   | 59.927 | -2.990  | 1.00 | 27.73 | C |
| ATOM | 3941 | CD  | GLU  | C | 79 | 1.873   | 61.257 | -3.083  | 1.00 | 28.97 | C |
| ATOM | 3942 | OE1 | GLU  | C | 79 | 2.621   | 61.479 | -4.066  | 1.00 | 33.04 | O |
| ATOM | 3943 | OE2 | GLU  | C | 79 | 1.704   | 62.072 | -2.148  | 1.00 | 33.96 | O |
| ATOM | 3944 | N   | ALA  | C | 80 | 0.242   | 57.349 | -6.495  | 1.00 | 24.13 | N |
| ATOM | 3945 | CA  | ALA  | C | 80 | 0.453   | 56.599 | -7.725  | 1.00 | 24.21 | C |
| ATOM | 3946 | C   | ALA  | C | 80 | 1.926   | 56.657 | -8.175  | 1.00 | 24.23 | C |
| ATOM | 3947 | O   | ALA  | C | 80 | 2.438   | 55.681 | -8.734  | 1.00 | 24.66 | O |
| ATOM | 3948 | CB  | ALA  | C | 80 | -0.493  | 57.092 | -8.818  | 1.00 | 24.53 | C |
| ATOM | 3949 | N   | GLU  | C | 81 | 2.622   | 57.764 | -7.880  | 1.00 | 23.58 | N |
| ATOM | 3950 | CA  | GLU  | C | 81 | 4.022   | 57.911 | -8.280  | 1.00 | 25.46 | C |
| ATOM | 3951 | C   | GLU  | C | 81 | 4.981   | 57.049 | -7.448  | 1.00 | 23.38 | C |
| ATOM | 3952 | O   | GLU  | C | 81 | 6.178   | 57.006 | -7.725  | 1.00 | 24.63 | O |
| ATOM | 3953 | CB  | GLU  | C | 81 | 4.484   | 59.369 | -8.210  | 1.00 | 26.41 | C |

```
ATOM   3954  CG   GLU C  81      4.853  59.872  -6.793  1.00 31.03           C
ATOM   3955  CD   GLU C  81      5.600  61.208  -6.808  1.00 31.92           C
ATOM   3956  OE1  GLU C  81      5.033  62.213  -6.296  1.00 37.74           O
ATOM   3957  OE2  GLU C  81      6.752  61.263  -7.337  1.00 35.50           O
ATOM   3958  N    ASP C  82      4.469  56.389  -6.423  1.00 21.28           N
ATOM   3959  CA   ASP C  82      5.291  55.505  -5.579  1.00 19.96           C
ATOM   3960  C    ASP C  82      5.557  54.132  -6.219  1.00 20.64           C
ATOM   3961  O    ASP C  82      6.393  53.375  -5.716  1.00 19.34           O
ATOM   3962  CB   ASP C  82      4.607  55.310  -4.208  1.00 19.14           C
ATOM   3963  CG   ASP C  82      4.587  56.574  -3.371  1.00 18.89           C
ATOM   3964  OD1  ASP C  82      5.432  57.473  -3.592  1.00 19.10           O
ATOM   3965  OD2  ASP C  82      3.702  56.683  -2.486  1.00 19.53           O
ATOM   3966  N    VAL C  83      4.862  53.783  -7.305  1.00 19.41           N
ATOM   3967  CA   VAL C  83      5.072  52.465  -7.890  1.00 20.22           C
ATOM   3968  C    VAL C  83      6.496  52.346  -8.402  1.00 18.68           C
ATOM   3969  O    VAL C  83      7.100  53.310  -8.857  1.00 18.33           O
ATOM   3970  CB   VAL C  83      4.091  52.098  -9.038  1.00 20.84           C
ATOM   3971  CG1  VAL C  83      2.695  52.025  -8.518  1.00 22.82           C
ATOM   3972  CG2  VAL C  83      4.218  53.072 -10.214  1.00 20.76           C
ATOM   3973  N    GLY C  84      7.015  51.138  -8.313  1.00 18.06           N
ATOM   3974  CA   GLY C  84      8.375  50.853  -8.665  1.00 16.84           C
ATOM   3975  C    GLY C  84      8.819  49.622  -7.891  1.00 15.54           C
ATOM   3976  O    GLY C  84      8.016  48.948  -7.252  1.00 15.03           O
ATOM   3977  N    ILE C  85     10.108  49.364  -7.946  1.00 14.28           N
ATOM   3978  CA   ILE C  85     10.716  48.250  -7.250  1.00 14.40           C
ATOM   3979  C    ILE C  85     11.608  48.788  -6.142  1.00 14.70           C
ATOM   3980  O    ILE C  85     12.490  49.622  -6.380  1.00 13.68           O
ATOM   3981  CB   ILE C  85     11.500  47.372  -8.219  1.00 16.00           C
ATOM   3982  CG1  ILE C  85     10.567  46.839  -9.317  1.00 17.60           C
ATOM   3983  CG2  ILE C  85     12.210  46.211  -7.483  1.00 15.17           C
ATOM   3984  CD1  ILE C  85     11.289  46.267 -10.465  1.00 19.93           C
ATOM   3985  N    TYR C  86     11.378  48.270  -4.935  1.00 13.05           N
ATOM   3986  CA   TYR C  86     12.062  48.695  -3.701  1.00 12.92           C
ATOM   3987  C    TYR C  86     13.074  47.632  -3.334  1.00 12.76           C
ATOM   3988  O    TYR C  86     12.683  46.437  -3.149  1.00 13.51           O
ATOM   3989  CB   TYR C  86     11.042  48.898  -2.542  1.00 13.38           C
ATOM   3990  CG   TYR C  86     10.144  50.093  -2.750  1.00 12.77           C
ATOM   3991  CD1  TYR C  86      9.013  50.010  -3.563  1.00 14.17           C
ATOM   3992  CD2  TYR C  86     10.442  51.318  -2.167  1.00 14.18           C
ATOM   3993  CE1  TYR C  86      8.214  51.137  -3.804  1.00 13.87           C
ATOM   3994  CE2  TYR C  86      9.637  52.441  -2.393  1.00 14.67           C
ATOM   3995  CZ   TYR C  86      8.536  52.335  -3.220  1.00 14.86           C
ATOM   3996  OH   TYR C  86      7.736  53.448  -3.454  1.00 15.94           O
ATOM   3997  N    TYR C  87     14.345  48.020  -3.221  1.00 11.45           N
ATOM   3998  CA   TYR C  87     15.420  47.072  -2.924  1.00 11.18           C
ATOM   3999  C    TYR C  87     16.024  47.350  -1.567  1.00 12.84           C
ATOM   4000  O    TYR C  87     16.325  48.504  -1.244  1.00 11.94           O
ATOM   4001  CB   TYR C  87     16.568  47.177  -3.942  1.00 12.77           C
ATOM   4002  CG   TYR C  87     16.229  46.892  -5.352  1.00 13.80           C
ATOM   4003  CD1  TYR C  87     16.363  45.611  -5.855  1.00 13.51           C
ATOM   4004  CD2  TYR C  87     15.794  47.898  -6.220  1.00 13.26           C
ATOM   4005  CE1  TYR C  87     16.082  45.332  -7.151  1.00 15.63           C
ATOM   4006  CE2  TYR C  87     15.507  47.620  -7.528  1.00 17.17           C
ATOM   4007  CZ   TYR C  87     15.648  46.323  -7.989  1.00 16.15           C
ATOM   4008  OH   TYR C  87     15.353  45.968  -9.285  1.00 18.64           O
ATOM   4009  N    CYS C  88     16.269  46.294  -0.799  1.00 13.66           N
ATOM   4010  CA   CYS C  88     17.111  46.400   0.382  1.00 14.68           C
ATOM   4011  C    CYS C  88     18.575  46.059   0.073  1.00 14.61           C
ATOM   4012  O    CYS C  88     18.884  45.319  -0.860  1.00 13.59           O
ATOM   4013  CB   CYS C  88     16.567  45.538   1.530  1.00 15.76           C
ATOM   4014  SG   CYS C  88     16.291  43.795   1.135  1.00 17.40           S
ATOM   4015  N    ALA C  89     19.478  46.637   0.863  1.00 12.70           N
ATOM   4016  CA   ALA C  89     20.897  46.421   0.731  1.00 10.96           C
ATOM   4017  C    ALA C  89     21.541  46.585   2.087  1.00 11.18           C
ATOM   4018  O    ALA C  89     21.015  47.322   2.955  1.00 11.38           O
```

| ATOM | 4019 | CB | ALA C | 89 | 21.515 | 47.429 | -0.267 | 1.00 | 11.45 | C |
|---|---|---|---|---|---|---|---|---|---|---|
| ATOM | 4020 | N | HIS C | 90 | 22.688 | 45.947 | 2.266 | 1.00 | 11.19 | N |
| ATOM | 4021 | CA | HIS C | 90 | 23.504 | 46.130 | 3.461 | 1.00 | 11.43 | C |
| ATOM | 4022 | C | HIS C | 90 | 24.736 | 46.937 | 3.141 | 1.00 | 13.07 | C |
| ATOM | 4023 | O | HIS C | 90 | 25.187 | 47.028 | 1.980 | 1.00 | 9.82 | O |
| ATOM | 4024 | CB | HIS C | 90 | 23.947 | 44.780 | 4.072 | 1.00 | 11.87 | C |
| ATOM | 4025 | CG | HIS C | 90 | 24.962 | 44.060 | 3.254 | 1.00 | 11.62 | C |
| ATOM | 4026 | ND1 | HIS C | 90 | 26.316 | 44.286 | 3.365 | 1.00 | 12.05 | N |
| ATOM | 4027 | CD2 | HIS C | 90 | 24.816 | 43.111 | 2.296 | 1.00 | 11.74 | C |
| ATOM | 4028 | CE1 | HIS C | 90 | 26.960 | 43.533 | 2.494 | 1.00 | 12.26 | C |
| ATOM | 4029 | NE2 | HIS C | 90 | 26.073 | 42.797 | 1.843 | 1.00 | 10.92 | N |
| ATOM | 4030 | N | ASN C | 91 | 25.320 | 47.495 | 4.196 | 1.00 | 13.03 | N |
| ATOM | 4031 | CA | ASN C | 91 | 26.650 | 48.076 | 4.096 | 1.00 | 13.09 | C |
| ATOM | 4032 | C | ASN C | 91 | 27.518 | 47.688 | 5.289 | 1.00 | 13.45 | C |
| ATOM | 4033 | O | ASN C | 91 | 28.001 | 48.562 | 6.044 | 1.00 | 13.76 | O |
| ATOM | 4034 | CB | ASN C | 91 | 26.536 | 49.592 | 4.001 | 1.00 | 13.63 | C |
| ATOM | 4035 | CG | ASN C | 91 | 27.880 | 50.286 | 3.940 | 1.00 | 14.33 | C |
| ATOM | 4036 | OD1 | ASN C | 91 | 28.895 | 49.719 | 3.492 | 1.00 | 12.39 | O |
| ATOM | 4037 | ND2 | ASN C | 91 | 27.903 | 51.527 | 4.420 | 1.00 | 12.53 | N |
| ATOM | 4038 | N | VAL C | 92 | 27.720 | 46.391 | 5.463 | 1.00 | 13.50 | N |
| ATOM | 4039 | CA | VAL C | 92 | 28.596 | 45.898 | 6.542 | 1.00 | 15.42 | C |
| ATOM | 4040 | C | VAL C | 92 | 29.829 | 45.117 | 6.096 | 1.00 | 16.34 | C |
| ATOM | 4041 | O | VAL C | 92 | 30.721 | 44.868 | 6.911 | 1.00 | 15.89 | O |
| ATOM | 4042 | CB | VAL C | 92 | 27.837 | 45.030 | 7.579 | 1.00 | 15.36 | C |
| ATOM | 4043 | CG1 | VAL C | 92 | 26.674 | 45.822 | 8.164 | 1.00 | 16.33 | C |
| ATOM | 4044 | CG2 | VAL C | 92 | 27.406 | 43.656 | 6.998 | 1.00 | 16.53 | C |
| ATOM | 4045 | N | GLU C | 93 | 29.881 | 44.745 | 4.826 | 1.00 | 15.86 | N |
| ATOM | 4046 | CA | GLU C | 93 | 31.038 | 44.066 | 4.274 | 1.00 | 16.40 | C |
| ATOM | 4047 | C | GLU C | 93 | 30.982 | 44.089 | 2.759 | 1.00 | 15.82 | C |
| ATOM | 4048 | O | GLU C | 93 | 29.964 | 44.493 | 2.145 | 1.00 | 14.93 | O |
| ATOM | 4049 | CB | GLU C | 93 | 31.061 | 42.604 | 4.739 | 1.00 | 16.57 | C |
| ATOM | 4050 | CG | GLU C | 93 | 29.868 | 41.793 | 4.224 | 1.00 | 15.50 | C |
| ATOM | 4051 | CD | GLU C | 93 | 29.913 | 40.304 | 4.615 | 1.00 | 19.60 | C |
| ATOM | 4052 | OE1 | GLU C | 93 | 30.264 | 40.005 | 5.774 | 1.00 | 22.21 | O |
| ATOM | 4053 | OE2 | GLU C | 93 | 29.551 | 39.439 | 3.774 | 1.00 | 18.61 | O |
| ATOM | 4054 | N | LEU C | 94 | 32.065 | 43.599 | 2.168 | 1.00 | 16.73 | N |
| ATOM | 4055 | CA | LEU C | 94 | 32.103 | 43.204 | 0.776 | 1.00 | 16.25 | C |
| ATOM | 4056 | C | LEU C | 94 | 31.983 | 41.664 | 0.714 | 1.00 | 17.23 | C |
| ATOM | 4057 | O | LEU C | 94 | 32.476 | 40.979 | 1.590 | 1.00 | 18.02 | O |
| ATOM | 4058 | CB | LEU C | 94 | 33.400 | 43.660 | 0.113 | 1.00 | 16.46 | C |
| ATOM | 4059 | CG | LEU C | 94 | 33.789 | 45.154 | 0.144 | 1.00 | 16.23 | C |
| ATOM | 4060 | CD1 | LEU C | 94 | 34.970 | 45.435 | -0.721 | 1.00 | 16.67 | C |
| ATOM | 4061 | CD2 | LEU C | 94 | 32.619 | 45.997 | -0.307 | 1.00 | 14.83 | C |
| ATOM | 4062 | N | PRO C | 95 | 31.315 | 41.127 | -0.311 | 1.00 | 17.27 | N |
| ATOM | 4063 | CA | PRO C | 95 | 30.646 | 41.858 | -1.382 | 1.00 | 16.76 | C |
| ATOM | 4064 | C | PRO C | 95 | 29.397 | 42.608 | -0.961 | 1.00 | 14.75 | C |
| ATOM | 4065 | O | PRO C | 95 | 28.698 | 42.228 | -0.048 | 1.00 | 12.29 | O |
| ATOM | 4066 | CB | PRO C | 95 | 30.266 | 40.755 | -2.386 | 1.00 | 17.59 | C |
| ATOM | 4067 | CG | PRO C | 95 | 30.195 | 39.529 | -1.581 | 1.00 | 17.05 | C |
| ATOM | 4068 | CD | PRO C | 95 | 31.188 | 39.662 | -0.489 | 1.00 | 17.96 | C |
| ATOM | 4069 | N | ARG C | 96 | 29.116 | 43.687 | -1.674 | 1.00 | 14.68 | N |
| ATOM | 4070 | CA | ARG C | 96 | 27.901 | 44.391 | -1.448 | 1.00 | 13.55 | C |
| ATOM | 4071 | C | ARG C | 96 | 26.820 | 43.577 | -2.111 | 1.00 | 12.75 | C |
| ATOM | 4072 | O | ARG C | 96 | 26.988 | 43.155 | -3.247 | 1.00 | 13.65 | O |
| ATOM | 4073 | CB | ARG C | 96 | 27.973 | 45.787 | -2.055 | 1.00 | 11.92 | C |
| ATOM | 4074 | CG | ARG C | 96 | 29.171 | 46.533 | -1.610 | 1.00 | 11.41 | C |
| ATOM | 4075 | CD | ARG C | 96 | 29.040 | 48.021 | -1.914 | 1.00 | 12.09 | C |
| ATOM | 4076 | NE | ARG C | 96 | 30.206 | 48.779 | -1.450 | 1.00 | 13.67 | N |
| ATOM | 4077 | CZ | ARG C | 96 | 30.393 | 49.186 | -0.194 | 1.00 | 14.80 | C |
| ATOM | 4078 | NH1 | ARG C | 96 | 29.507 | 48.920 | 0.755 | 1.00 | 12.78 | N |
| ATOM | 4079 | NH2 | ARG C | 96 | 31.480 | 49.872 | 0.116 | 1.00 | 14.43 | N |
| ATOM | 4080 | N | THR C | 97 | 25.720 | 43.343 | -1.394 | 1.00 | 12.58 | N |
| ATOM | 4081 | CA | THR C | 97 | 24.618 | 42.605 | -1.944 | 1.00 | 12.96 | C |
| ATOM | 4082 | C | THR C | 97 | 23.282 | 43.299 | -1.710 | 1.00 | 13.19 | C |
| ATOM | 4083 | O | THR C | 97 | 23.137 | 44.095 | -0.788 | 1.00 | 13.16 | O |

```
ATOM   4084  CB   THR C  97     24.540  41.152  -1.386  1.00 15.31          C
ATOM   4085  OG1  THR C  97     24.388  41.184   0.027  1.00 12.57          O
ATOM   4086  CG2  THR C  97     25.802  40.373  -1.738  1.00 16.83          C
ATOM   4087  N    PHE C  98     22.336  42.945  -2.571  1.00 12.50          N
ATOM   4088  CA   PHE C  98     21.032  43.567  -2.669  1.00 13.39          C
ATOM   4089  C    PHE C  98     19.953  42.506  -2.599  1.00 14.18          C
ATOM   4090  O    PHE C  98     20.148  41.359  -3.075  1.00 13.07          O
ATOM   4091  CB   PHE C  98     20.891  44.297  -4.018  1.00 14.16          C
ATOM   4092  CG   PHE C  98     21.866  45.408  -4.212  1.00 13.60          C
ATOM   4093  CD1  PHE C  98     23.102  45.175  -4.786  1.00 14.33          C
ATOM   4094  CD2  PHE C  98     21.531  46.724  -3.850  1.00 14.14          C
ATOM   4095  CE1  PHE C  98     24.015  46.209  -4.964  1.00 14.83          C
ATOM   4096  CE2  PHE C  98     22.445  47.757  -4.025  1.00 15.19          C
ATOM   4097  CZ   PHE C  98     23.675  47.509  -4.567  1.00 15.38          C
ATOM   4098  N    GLY C  99     18.795  42.878  -2.054  1.00 12.73          N
ATOM   4099  CA   GLY C  99     17.602  42.096  -2.233  1.00 12.81          C
ATOM   4100  C    GLY C  99     17.166  41.988  -3.684  1.00 13.61          C
ATOM   4101  O    GLY C  99     17.668  42.703  -4.566  1.00 13.40          O
ATOM   4102  N    GLY C 100     16.254  41.044  -3.960  1.00 12.79          N
ATOM   4103  CA   GLY C 100     15.743  40.853  -5.292  1.00 12.68          C
ATOM   4104  C    GLY C 100     14.782  41.938  -5.768  1.00 13.09          C
ATOM   4105  O    GLY C 100     14.360  41.938  -6.931  1.00 11.25          O
ATOM   4106  N    GLY C 101     14.379  42.820  -4.849  1.00 12.18          N
ATOM   4107  CA   GLY C 101     13.458  43.889  -5.155  1.00 14.82          C
ATOM   4108  C    GLY C 101     12.020  43.486  -4.914  1.00 15.37          C
ATOM   4109  O    GLY C 101     11.635  42.365  -5.237  1.00 16.82          O
ATOM   4110  N    THR C 102     11.255  44.357  -4.264  1.00 14.57          N
ATOM   4111  CA   THR C 102      9.816  44.161  -4.108  1.00 15.87          C
ATOM   4112  C    THR C 102      9.059  45.217  -4.923  1.00 16.59          C
ATOM   4113  O    THR C 102      9.245  46.421  -4.710  1.00 15.92          O
ATOM   4114  CB   THR C 102      9.339  44.292  -2.647  1.00 16.59          C
ATOM   4115  OG1  THR C 102     10.040  43.370  -1.803  1.00 13.71          O
ATOM   4116  CG2  THR C 102      7.852  44.048  -2.552  1.00 16.89          C
ATOM   4117  N    LYS C 103      8.199  44.751  -5.822  1.00 18.17          N
ATOM   4118  CA   LYS C 103      7.349  45.629  -6.631  1.00 19.53          C
ATOM   4119  C    LYS C 103      6.107  46.076  -5.871  1.00 19.42          C
ATOM   4120  O    LYS C 103      5.305  45.258  -5.399  1.00 19.07          O
ATOM   4121  CB   LYS C 103      6.944  44.951  -7.944  1.00 20.60          C
ATOM   4122  CG   LYS C 103      6.292  45.901  -8.955  1.00 22.08          C
ATOM   4123  CD   LYS C 103      5.663  45.154 -10.106  1.00 25.85          C
ATOM   4124  CE   LYS C 103      6.681  44.615 -11.062  1.00 29.83          C
ATOM   4125  NZ   LYS C 103      6.092  44.463 -12.440  1.00 33.31          N
ATOM   4126  N    LEU C 104      5.955  47.382  -5.732  1.00 18.68          N
ATOM   4127  CA   LEU C 104      4.755  47.939  -5.142  1.00 21.50          C
ATOM   4128  C    LEU C 104      3.694  47.943  -6.234  1.00 22.96          C
ATOM   4129  O    LEU C 104      3.956  48.501  -7.296  1.00 21.73          O
ATOM   4130  CB   LEU C 104      5.013  49.365  -4.674  1.00 20.01          C
ATOM   4131  CG   LEU C 104      3.857  50.115  -4.016  1.00 19.63          C
ATOM   4132  CD1  LEU C 104      3.498  49.416  -2.750  1.00 22.24          C
ATOM   4133  CD2  LEU C 104      4.237  51.555  -3.769  1.00 21.73          C
ATOM   4134  N    GLU C 105      2.546  47.285  -5.995  1.00 26.11          N
ATOM   4135  CA   GLU C 105      1.380  47.371  -6.904  1.00 27.88          C
ATOM   4136  C    GLU C 105      0.241  48.145  -6.235  1.00 28.77          C.
ATOM   4137  O    GLU C 105     -0.100  47.890  -5.084  1.00 28.56          O
ATOM   4138  CB   GLU C 105      0.849  45.986  -7.359  1.00 29.49          C
ATOM   4139  CG   GLU C 105     -0.639  46.081  -7.896  1.00 30.95          C
ATOM   4140  CD   GLU C 105     -1.087  45.023  -8.920  1.00 33.22          C
ATOM   4141  OE1  GLU C 105     -0.694  43.827  -8.794  1.00 39.25          O
ATOM   4142  OE2  GLU C 105     -1.886  45.394  -9.834  1.00 35.27          O
ATOM   4143  N    ILE C 106     -0.334  49.084  -6.981  1.00 29.90          N
ATOM   4144  CA   ILE C 106     -1.485  49.854  -6.539  1.00 31.02          C
ATOM   4145  C    ILE C 106     -2.802  49.184  -6.935  1.00 31.59          C
ATOM   4146  O    ILE C 106     -2.944  48.697  -8.062  1.00 30.90          O
ATOM   4147  CB   ILE C 106     -1.462  51.226  -7.182  1.00 31.61          C
ATOM   4148  CG1  ILE C 106     -0.209  51.983  -6.744  1.00 33.68          C
```

322

| ATOM | 4149 | CG2 | ILE | C | 106 | -2.742 | 52.008 | -6.869 | 1.00 | 32.32 | C |
|------|------|-----|-----|---|-----|--------|--------|--------|------|-------|---|
| ATOM | 4150 | CD1 | ILE | C | 106 | -0.243 | 52.469 | -5.339 | 1.00 | 35.54 | C |
| ATOM | 4151 | N | LYS | C | 107 | -3.765 | 49.177 | -6.013 | 1.00 | 32.16 | N |
| ATOM | 4152 | CA | LYS | C | 107 | -5.130 | 48.734 | -6.313 | 1.00 | 33.36 | C |
| ATOM | 4153 | C | LYS | C | 107 | -5.924 | 49.992 | -6.632 | 1.00 | 33.49 | C |
| ATOM | 4154 | O | LYS | C | 107 | -5.842 | 50.982 | -5.911 | 1.00 | 33.87 | O |
| ATOM | 4155 | CB | LYS | C | 107 | -5.744 | 47.987 | -5.126 | 1.00 | 33.36 | C |
| ATOM | 4156 | CG | LYS | C | 107 | -7.131 | 47.394 | -5.389 | 1.00 | 33.54 | C |
| ATOM | 4157 | CD | LYS | C | 107 | -7.578 | 46.498 | -4.241 | 1.00 | 34.54 | C |
| ATOM | 4158 | CE | LYS | C | 107 | -9.066 | 46.112 | -4.335 | 1.00 | 35.25 | C |
| ATOM | 4159 | NZ | LYS | C | 107 | -9.520 | 45.206 | -3.220 | 1.00 | 35.45 | N |
| ATOM | 4160 | N | ARG | C | 108 | -6.640 | 49.969 | -7.753 | 1.00 | 35.19 | N |
| ATOM | 4161 | CA | ARG | C | 108 | -7.514 | 51.068 | -8.145 | 1.00 | 34.87 | C |
| ATOM | 4162 | C | ARG | C | 108 | -8.787 | 50.509 | -8.760 | 1.00 | 35.83 | C |
| ATOM | 4163 | O | ARG | C | 108 | -8.982 | 49.294 | -8.808 | 1.00 | 36.22 | O |
| ATOM | 4164 | CB | ARG | C | 108 | -6.794 | 52.008 | -9.108 | 1.00 | 34.99 | C |
| ATOM | 4165 | CG | ARG | C | 108 | -6.298 | 51.334 | -10.386 | 1.00 | 34.93 | C |
| ATOM | 4166 | CD | ARG | C | 108 | -6.345 | 52.301 | -11.540 | 1.00 | 35.54 | C |
| ATOM | 4167 | NE | ARG | C | 108 | -7.703 | 52.385 | -12.072 | 1.00 | 35.07 | N |
| ATOM | 4168 | CZ | ARG | C | 108 | -8.186 | 53.424 | -12.742 | 1.00 | 35.14 | C |
| ATOM | 4169 | NH1 | ARG | C | 108 | -7.439 | 54.491 | -12.988 | 1.00 | 32.32 | N |
| ATOM | 4170 | NH2 | ARG | C | 108 | -9.443 | 53.393 | -13.165 | 1.00 | 35.91 | N |
| ATOM | 4171 | N | ALA | C | 109 | -9.676 | 51.392 | -9.200 | 1.00 | 37.70 | N |
| ATOM | 4172 | CA | ALA | C | 109 | -10.970 | 50.954 | -9.755 | 1.00 | 38.41 | C |
| ATOM | 4173 | C | ALA | C | 109 | -10.813 | 50.172 | -11.064 | 1.00 | 38.10 | C |
| ATOM | 4174 | O | ALA | C | 109 | -9.999 | 50.532 | -11.904 | 1.00 | 38.19 | O |
| ATOM | 4175 | CB | ALA | C | 109 | -11.866 | 52.160 | -9.969 | 1.00 | 38.83 | C |
| ATOM | 4176 | N | ASP | C | 110 | -11.587 | 49.103 | -11.230 | 1.00 | 38.64 | N |
| ATOM | 4177 | CA | ASP | C | 110 | -11.648 | 48.397 | -12.513 | 1.00 | 39.03 | C |
| ATOM | 4178 | C | ASP | C | 110 | -11.845 | 49.409 | -13.652 | 1.00 | 38.50 | C |
| ATOM | 4179 | O | ASP | C | 110 | -12.523 | 50.427 | -13.480 | 1.00 | 39.05 | O |
| ATOM | 4180 | CB | ASP | C | 110 | -12.775 | 47.349 | -12.522 | 1.00 | 39.56 | C |
| ATOM | 4181 | CG | ASP | C | 110 | -12.358 | 46.010 | -11.888 | 1.00 | 41.14 | C |
| ATOM | 4182 | OD1 | ASP | C | 110 | -11.309 | 45.948 | -11.201 | 1.00 | 42.34 | O |
| ATOM | 4183 | OD2 | ASP | C | 110 | -13.092 | 45.004 | -12.071 | 1.00 | 42.50 | O |
| ATOM | 4184 | N | ALA | C | 111 | -11.211 | 49.153 | -14.799 | 1.00 | 37.57 | N |
| ATOM | 4185 | CA | ALA | C | 111 | -11.370 | 50.002 | -15.979 | 1.00 | 35.48 | C |
| ATOM | 4186 | C | ALA | C | 111 | -11.113 | 49.219 | -17.266 | 1.00 | 34.45 | C |
| ATOM | 4187 | O | ALA | C | 111 | -10.129 | 48.491 | -17.378 | 1.00 | 35.18 | O |
| ATOM | 4188 | CB | ALA | C | 111 | -10.459 | 51.203 | -15.903 | 1.00 | 34.98 | C |
| ATOM | 4189 | N | ALA | C | 112 | -12.014 | 49.371 | -18.235 | 1.00 | 33.16 | N |
| ATOM | 4190 | CA | ALA | C | 112 | -11.906 | 48.659 | -19.508 | 1.00 | 31.45 | C |
| ATOM | 4191 | C | ALA | C | 112 | -10.896 | 49.356 | -20.432 | 1.00 | 29.39 | C |
| ATOM | 4192 | O | ALA | C | 112 | -10.663 | 50.561 | -20.328 | 1.00 | 28.83 | O |
| ATOM | 4193 | CB | ALA | C | 112 | -13.282 | 48.558 | -20.184 | 1.00 | 31.60 | C |
| ATOM | 4194 | N | PRO | C | 113 | -10.299 | 48.595 | -21.350 | 1.00 | 28.18 | N |
| ATOM | 4195 | CA | PRO | C | 113 | -9.305 | 49.148 | -22.264 | 1.00 | 28.03 | C |
| ATOM | 4196 | C | PRO | C | 113 | -9.905 | 50.050 | -23.352 | 1.00 | 28.05 | C |
| ATOM | 4197 | O | PRO | C | 113 | -11.051 | 49.825 | -23.772 | 1.00 | 26.28 | O |
| ATOM | 4198 | CB | PRO | C | 113 | -8.682 | 47.892 | -22.890 | 1.00 | 27.58 | C |
| ATOM | 4199 | CG | PRO | C | 113 | -9.726 | 46.913 | -22.863 | 1.00 | 28.08 | C |
| ATOM | 4200 | CD | PRO | C | 113 | -10.505 | 47.155 | -21.584 | 1.00 | 28.70 | C |
| ATOM | 4201 | N | THR | C | 114 | -9.138 | 51.067 | -23.748 | 1.00 | 28.09 | N |
| ATOM | 4202 | CA | THR | C | 114 | -9.358 | 51.847 | -24.959 | 1.00 | 28.57 | C |
| ATOM | 4203 | C | THR | C | 114 | -8.580 | 51.168 | -26.076 | 1.00 | 28.01 | C |
| ATOM | 4204 | O | THR | C | 114 | -7.357 | 51.197 | -26.067 | 1.00 | 28.88 | O |
| ATOM | 4205 | CB | THR | C | 114 | -8.805 | 53.288 | -24.804 | 1.00 | 28.97 | C |
| ATOM | 4206 | OG1 | THR | C | 114 | -9.496 | 53.962 | -23.741 | 1.00 | 30.43 | O |
| ATOM | 4207 | CG2 | THR | C | 114 | -8.938 | 54.099 | -26.099 | 1.00 | 29.28 | C |
| ATOM | 4208 | N | VAL | C | 115 | -9.299 | 50.557 | -27.020 | 1.00 | 27.47 | N |
| ATOM | 4209 | CA | VAL | C | 115 | -8.701 | 49.770 | -28.096 | 1.00 | 26.02 | C |
| ATOM | 4210 | C | VAL | C | 115 | -8.532 | 50.609 | -29.360 | 1.00 | 25.70 | C |
| ATOM | 4211 | O | VAL | C | 115 | -9.451 | 51.343 | -29.753 | 1.00 | 24.47 | O |
| ATOM | 4212 | CB | VAL | C | 115 | -9.553 | 48.523 | -28.405 | 1.00 | 26.09 | C |
| ATOM | 4213 | CG1 | VAL | C | 115 | -8.832 | 47.582 | -29.361 | 1.00 | 24.62 | C |

| | | | | | | | | | |
|------|------|-----|-----|---|-----|--------|--------|---------|-----------|---|
| ATOM | 4214 | CG2 | VAL | C | 115 | -9.886 | 47.780 | -27.119 | 1.00 26.95 | C |
| ATOM | 4215 | N   | SER | C | 116 | -7.346 | 50.520 | -29.970 | 1.00 24.54 | N |
| ATOM | 4216 | CA  | SER | C | 116 | -7.049 | 51.120 | -31.281 | 1.00 23.98 | C |
| ATOM | 4217 | C   | SER | C | 116 | -6.379 | 50.087 | -32.185 | 1.00 23.58 | C |
| ATOM | 4218 | O   | SER | C | 116 | -5.527 | 49.302 | -31.721 | 1.00 21.61 | O |
| ATOM | 4219 | CB  | SER | C | 116 | -6.105 | 52.322 | -31.151 | 1.00 24.68 | C |
| ATOM | 4220 | OG  | SER | C | 116 | -6.585 | 53.266 | -30.242 | 1.00 26.27 | O |
| ATOM | 4221 | N   | ILE | C | 117 | -6.770 | 50.087 | -33.467 | 1.00 21.95 | N |
| ATOM | 4222 | CA  | ILE | C | 117 | -6.186 | 49.211 | -34.457 | 1.00 22.07 | C |
| ATOM | 4223 | C   | ILE | C | 117 | -5.494 | 50.047 | -35.541 | 1.00 21.63 | C |
| ATOM | 4224 | O   | ILE | C | 117 | -5.963 | 51.143 | -35.906 | 1.00 21.59 | O |
| ATOM | 4225 | CB  | ILE | C | 117 | -7.225 | 48.217 | -35.077 | 1.00 21.89 | C |
| ATOM | 4226 | CG1 | ILE | C | 117 | -6.492 | 47.120 | -35.841 | 1.00 22.81 | C |
| ATOM | 4227 | CG2 | ILE | C | 117 | -8.278 | 48.978 | -35.948 | 1.00 21.72 | C |
| ATOM | 4228 | CD1 | ILE | C | 117 | -7.324 | 45.964 | -36.299 | 1.00 21.67 | C |
| ATOM | 4229 | N   | PHE | C | 118 | -4.359 | 49.549 | -36.028 | 1.00 20.78 | N |
| ATOM | 4230 | CA  | PHE | C | 118 | -3.571 | 50.254 | -37.045 | 1.00 20.45 | C |
| ATOM | 4231 | C   | PHE | C | 118 | -3.202 | 49.291 | -38.155 | 1.00 20.68 | C |
| ATOM | 4232 | O   | PHE | C | 118 | -2.622 | 48.230 | -37.906 | 1.00 19.51 | O |
| ATOM | 4233 | CB  | PHE | C | 118 | -2.306 | 50.853 | -36.424 | 1.00 21.37 | C |
| ATOM | 4234 | CG  | PHE | C | 118 | -2.572 | 51.749 | -35.254 | 1.00 20.53 | C |
| ATOM | 4235 | CD1 | PHE | C | 118 | -2.800 | 53.109 | -35.444 | 1.00 19.82 | C |
| ATOM | 4236 | CD2 | PHE | C | 118 | -2.554 | 51.252 | -33.960 | 1.00 20.82 | C |
| ATOM | 4237 | CE1 | PHE | C | 118 | -3.051 | 53.944 | -34.379 | 1.00 21.71 | C |
| ATOM | 4238 | CE2 | PHE | C | 118 | -2.786 | 52.091 | -32.886 | 1.00 21.91 | C |
| ATOM | 4239 | CZ  | PHE | C | 118 | -3.048 | 53.441 | -33.096 | 1.00 22.19 | C |
| ATOM | 4240 | N   | PRO | C | 119 | -3.548 | 49.640 | -39.397 | 1.00 21.02 | N |
| ATOM | 4241 | CA  | PRO | C | 119 | -3.121 | 48.812 | -40.508 | 1.00 20.32 | C |
| ATOM | 4242 | C   | PRO | C | 119 | -1.619 | 49.051 | -40.816 | 1.00 19.62 | C |
| ATOM | 4243 | O   | PRO | C | 119 | -1.021 | 50.007 | -40.272 | 1.00 19.24 | O |
| ATOM | 4244 | CB  | PRO | C | 119 | -4.025 | 49.263 | -41.659 | 1.00 21.09 | C |
| ATOM | 4245 | CG  | PRO | C | 119 | -4.497 | 50.624 | -41.306 | 1.00 22.89 | C |
| ATOM | 4246 | CD  | PRO | C | 119 | -4.320 | 50.822 | -39.824 | 1.00 21.96 | C |
| ATOM | 4247 | N   | PRO | C | 120 | -1.003 | 48.182 | -41.631 | 1.00 19.87 | N |
| ATOM | 4248 | CA  | PRO | C | 120 |  0.394 | 48.374 | -42.031 | 1.00 20.22 | C |
| ATOM | 4249 | C   | PRO | C | 120 |  0.668 | 49.720 | -42.713 | 1.00 21.39 | C |
| ATOM | 4250 | O   | PRO | C | 120 | -0.168 | 50.229 | -43.469 | 1.00 22.57 | O |
| ATOM | 4251 | CB  | PRO | C | 120 |  0.672 | 47.224 | -43.002 | 1.00 20.76 | C |
| ATOM | 4252 | CG  | PRO | C | 120 | -0.560 | 46.447 | -43.122 | 1.00 20.99 | C |
| ATOM | 4253 | CD  | PRO | C | 120 | -1.586 | 46.956 | -42.193 | 1.00 19.92 | C |
| ATOM | 4254 | N   | SER | C | 121 |  1.843 | 50.270 | -42.443 | 1.00 20.75 | N |
| ATOM | 4255 | CA  | SER | C | 121 |  2.345 | 51.457 | -43.119 | 1.00 20.47 | C |
| ATOM | 4256 | C   | SER | C | 121 |  2.697 | 51.159 | -44.580 | 1.00 20.39 | C |
| ATOM | 4257 | O   | SER | C | 121 |  2.938 | 50.016 | -44.959 | 1.00 18.14 | O |
| ATOM | 4258 | CB  | SER | C | 121 |  3.589 | 51.965 | -42.368 | 1.00 20.84 | C |
| ATOM | 4259 | OG  | SER | C | 121 |  4.620 | 50.990 | -42.378 | 1.00 21.57 | O |
| ATOM | 4260 | N   | SER | C | 122 |  2.729 | 52.195 | -45.418 | 1.00 20.53 | N |
| ATOM | 4261 | CA  | SER | C | 122 |  3.182 | 52.017 | -46.806 | 1.00 21.17 | C |
| ATOM | 4262 | C   | SER | C | 122 |  4.659 | 51.632 | -46.862 | 1.00 21.07 | C |
| ATOM | 4263 | O   | SER | C | 122 |  5.072 | 50.827 | -47.712 | 1.00 20.82 | O |
| ATOM | 4264 | CB  | SER | C | 122 |  2.927 | 53.293 | -47.622 | 1.00 22.52 | C |
| ATOM | 4265 | OG  | SER | C | 122 |  3.383 | 54.417 | -46.914 | 1.00 24.24 | O |
| ATOM | 4266 | N   | GLU | C | 123 |  5.439 | 52.202 | -45.945 | 1.00 21.40 | N |
| ATOM | 4267 | CA  | GLU | C | 123 |  6.860 | 51.859 | -45.767 | 1.00 21.24 | C |
| ATOM | 4268 | C   | GLU | C | 123 |  7.027 | 50.355 | -45.607 | 1.00 18.69 | C |
| ATOM | 4269 | O   | GLU | C | 123 |  7.751 | 49.687 | -46.355 | 1.00 19.62 | O |
| ATOM | 4270 | CB  | GLU | C | 123 |  7.406 | 52.517 | -44.501 | 1.00 21.86 | C |
| ATOM | 4271 | CG  | GLU | C | 123 |  7.638 | 54.009 | -44.584 | 1.00 24.32 | C |
| ATOM | 4272 | CD  | GLU | C | 123 |  6.490 | 54.843 | -44.024 | 1.00 24.41 | C |
| ATOM | 4273 | OE1 | GLU | C | 123 |  5.325 | 54.358 | -43.955 | 1.00 25.72 | O |
| ATOM | 4274 | OE2 | GLU | C | 123 |  6.762 | 56.009 | -43.664 | 1.00 27.04 | O |
| ATOM | 4275 | N   | GLN | C | 124 |  6.336 | 49.811 | -44.620 | 1.00 18.66 | N |
| ATOM | 4276 | CA  | GLN | C | 124 |  6.413 | 48.388 | -44.378 | 1.00 17.54 | C |
| ATOM | 4277 | C   | GLN | C | 124 |  5.991 | 47.575 | -45.584 | 1.00 17.58 | C |
| ATOM | 4278 | O   | GLN | C | 124 |  6.671 | 46.613 | -45.944 | 1.00 15.55 | O |

```
ATOM   4279  CB   GLN C 124      5.647  47.964 -43.109  1.00 17.75           C
ATOM   4280  CG   GLN C 124      6.091  46.596 -42.644  1.00 17.20           C
ATOM   4281  CD   GLN C 124      5.368  46.088 -41.423  1.00 17.39           C
ATOM   4282  OE1  GLN C 124      4.313  46.590 -41.049  1.00 16.77           O
ATOM   4283  NE2  GLN C 124      5.937  45.048 -40.803  1.00 20.11           N
ATOM   4284  N    LEU C 125      4.878  47.936 -46.231  1.00 17.93           N
ATOM   4285  CA   LEU C 125      4.415  47.156 -47.374  1.00 17.48           C
ATOM   4286  C    LEU C 125      5.436  47.169 -48.512  1.00 17.91           C
ATOM   4287  O    LEU C 125      5.683  46.142 -49.135  1.00 18.85           O
ATOM   4288  CB   LEU C 125      3.030  47.619 -47.857  1.00 19.55           C
ATOM   4289  CG   LEU C 125      1.880  47.202 -46.923  1.00 20.79           C
ATOM   4290  CD1  LEU C 125      0.630  48.036 -47.206  1.00 20.45           C
ATOM   4291  CD2  LEU C 125      1.587  45.702 -47.102  1.00 20.83           C
ATOM   4292  N    THR C 126      6.063  48.314 -48.744  1.00 18.57           N
ATOM   4293  CA   THR C 126      7.140  48.397 -49.737  1.00 18.85           C
ATOM   4294  C    THR C 126      8.301  47.416 -49.465  1.00 19.40           C
ATOM   4295  O    THR C 126      8.900  46.870 -50.413  1.00 20.07           O
ATOM   4296  CB   THR C 126      7.623  49.836 -49.845  1.00 20.04           C
ATOM   4297  OG1  THR C 126      6.562  50.615 -50.412  1.00 19.15           O
ATOM   4298  CG2  THR C 126      8.863  49.936 -50.703  1.00 19.26           C
ATOM   4299  N    SER C 127      8.573  47.174 -48.178  1.00 19.93           N
ATOM   4300  CA   SER C 127      9.657  46.294 -47.740  1.00 19.17           C
ATOM   4301  C    SER C 127      9.265  44.834 -47.809  1.00 19.25           C
ATOM   4302  O    SER C 127     10.124  43.960 -47.663  1.00 19.74           O
ATOM   4303  CB   SER C 127     10.080  46.643 -46.312  1.00 19.09           C
ATOM   4304  OG   SER C 127      9.164  46.116 -45.346  1.00 17.24           O
ATOM   4305  N    GLY C 128      7.971  44.550 -48.030  1.00 19.61           N
ATOM   4306  CA   GLY C 128      7.520  43.168 -48.205  1.00 18.58           C
ATOM   4307  C    GLY C 128      6.901  42.588 -46.945  1.00 18.65           C
ATOM   4308  O    GLY C 128      6.664  41.363 -46.850  1.00 19.23           O
ATOM   4309  N    GLY C 129      6.626  43.450 -45.972  1.00 18.71           N
ATOM   4310  CA   GLY C 129      6.021  43.005 -44.708  1.00 17.87           C
ATOM   4311  C    GLY C 129      4.701  43.685 -44.381  1.00 18.26           C
ATOM   4312  O    GLY C 129      4.342  44.701 -44.979  1.00 16.64           O
ATOM   4313  N    ALA C 130      3.975  43.129 -43.406  1.00 17.86           N
ATOM   4314  CA   ALA C 130      2.668  43.682 -43.032  1.00 19.42           C
ATOM   4315  C    ALA C 130      2.366  43.374 -41.584  1.00 19.14           C
ATOM   4316  O    ALA C 130      2.002  42.251 -41.260  1.00 20.46           O
ATOM   4317  CB   ALA C 130      1.589  43.106 -43.919  1.00 18.89           C
ATOM   4318  N    SER C 131      2.582  44.345 -40.713  1.00 18.34           N
ATOM   4319  CA   SER C 131      2.159  44.223 -39.327  1.00 18.41           C
ATOM   4320  C    SER C 131      0.911  45.031 -39.068  1.00 19.40           C
ATOM   4321  O    SER C 131      0.838  46.219 -39.446  1.00 19.12           O
ATOM   4322  CB   SER C 131      3.244  44.704 -38.361  1.00 17.67           C
ATOM   4323  OG   SER C 131      4.428  43.956 -38.496  1.00 18.86           O
ATOM   4324  N    VAL C 132     -0.032  44.407 -38.362  1.00 19.02           N
ATOM   4325  CA   VAL C 132     -1.218  45.080 -37.861  1.00 18.84           C
ATOM   4326  C    VAL C 132     -1.079  45.189 -36.343  1.00 19.35           C
ATOM   4327  O    VAL C 132     -0.757  44.194 -35.668  1.00 19.14           O
ATOM   4328  CB   VAL C 132     -2.518  44.303 -38.198  1.00 19.69           C
ATOM   4329  CG1  VAL C 132     -3.712  45.164 -37.924  1.00 20.65           C
ATOM   4330  CG2  VAL C 132     -2.534  43.852 -39.628  1.00 21.18           C
ATOM   4331  N    VAL C 133     -1.329  46.386 -35.821  1.00 19.55           N
ATOM   4332  CA   VAL C 133     -1.150  46.675 -34.408  1.00 20.48           C
ATOM   4333  C    VAL C 133     -2.470  47.011 -33.731  1.00 22.03           C
ATOM   4334  O    VAL C 133     -3.321  47.671 -34.291  1.00 21.13           O
ATOM   4335  CB   VAL C 133     -0.118  47.801 -34.182  1.00 21.24           C
ATOM   4336  CG1  VAL C 133      0.013  48.144 -32.665  1.00 21.01           C
ATOM   4337  CG2  VAL C 133      1.217  47.384 -34.761  1.00 21.01           C
ATOM   4338  N    CYS C 134     -2.623  46.483 -32.520  1.00 23.51           N
ATOM   4339  CA   CYS C 134     -3.750  46.753 -31.662  1.00 23.52           C
ATOM   4340  C    CYS C 134     -3.192  47.257 -30.329  1.00 22.88           C
ATOM   4341  O    CYS C 134     -2.410  46.539 -29.683  1.00 22.71           O
ATOM   4342  CB   CYS C 134     -4.535  45.445 -31.494  1.00 25.04           C
ATOM   4343  SG   CYS C 134     -6.130  45.596 -30.722  1.00 29.62           S
```

325

```
ATOM   4344  N   PHE C 135    -3.500  48.508 -29.965  1.00 22.20      N
ATOM   4345  CA  PHE C 135    -3.247  48.992 -28.614  1.00 23.21      C
ATOM   4346  C   PHE C 135    -4.482  48.783 -27.728  1.00 24.05      C
ATOM   4347  O   PHE C 135    -5.595  49.167 -28.112  1.00 23.29      O
ATOM   4348  CB  PHE C 135    -2.894  50.476 -28.602  1.00 22.42      C
ATOM   4349  CG  PHE C 135    -1.574  50.818 -29.233  1.00 23.10      C
ATOM   4350  CD1 PHE C 135    -0.511  49.922 -29.257  1.00 23.42      C
ATOM   4351  CD2 PHE C 135    -1.359  52.093 -29.727  1.00 23.85      C
ATOM   4352  CE1 PHE C 135     0.716  50.284 -29.827  1.00 23.48      C
ATOM   4353  CE2 PHE C 135    -0.130  52.441 -30.292  1.00 24.08      C
ATOM   4354  CZ  PHE C 135     0.900  51.528 -30.333  1.00 23.69      C
ATOM   4355  N   LEU C 136    -4.263  48.179 -26.553  1.00 24.12      N
ATOM   4356  CA  LEU C 136    -5.270  48.036 -25.491  1.00 24.93      C
ATOM   4357  C   LEU C 136    -4.764  48.892 -24.342  1.00 25.30      C
ATOM   4358  O   LEU C 136    -3.927  48.450 -23.542  1.00 24.97      O
ATOM   4359  CB  LEU C 136    -5.373  46.578 -25.034  1.00 25.39      C
ATOM   4360  CG  LEU C 136    -6.110  45.543 -25.895  1.00 27.07      C
ATOM   4361  CD1 LEU C 136    -5.769  45.670 -27.357  1.00 28.30      C
ATOM   4362  CD2 LEU C 136    -5.815  44.110 -25.416  1.00 27.38      C
ATOM   4363  N   ASN C 137    -5.237  50.126 -24.264  1.00 25.54      N
ATOM   4364  CA  ASN C 137    -4.651  51.094 -23.368  1.00 26.50      C
ATOM   4365  C   ASN C 137    -5.476  51.396 -22.109  1.00 27.93      C
ATOM   4366  O   ASN C 137    -6.712  51.367 -22.127  1.00 28.59      O
ATOM   4367  CB  ASN C 137    -4.348  52.370 -24.133  1.00 26.38      C
ATOM   4368  CG  ASN C 137    -3.157  52.216 -25.063  1.00 26.87      C
ATOM   4369  OD1 ASN C 137    -2.431  51.222 -25.002  1.00 29.58      O
ATOM   4370  ND2 ASN C 137    -2.957  53.187 -25.921  1.00 28.08      N
ATOM   4371  N   ASN C 138    -4.743  51.663 -21.025  1.00 28.62      N
ATOM   4372  CA  ASN C 138    -5.278  52.154 -19.757  1.00 29.07      C
ATOM   4373  C   ASN C 138    -6.429  51.362 -19.163  1.00 28.95      C
ATOM   4374  O   ASN C 138    -7.511  51.890 -18.890  1.00 27.83      O
ATOM   4375  CB  ASN C 138    -5.650  53.622 -19.887  1.00 30.21      C
ATOM   4376  CG  ASN C 138    -4.541  54.437 -20.486  1.00 31.60      C
ATOM   4377  OD1 ASN C 138    -4.403  54.522 -21.704  1.00 34.93      O
ATOM   4378  ND2 ASN C 138    -3.738  55.047 -19.637  1.00 32.68      N
ATOM   4379  N   PHE C 139    -6.170  50.088 -18.914  1.00 28.06      N
ATOM   4380  CA  PHE C 139    -7.138  49.251 -18.255  1.00 28.27      C
ATOM   4381  C   PHE C 139    -6.665  48.797 -16.860  1.00 29.98      C
ATOM   4382  O   PHE C 139    -5.493  48.941 -16.504  1.00 29.21      O
ATOM   4383  CB  PHE C 139    -7.466  48.055 -19.139  1.00 27.42      C
ATOM   4384  CG  PHE C 139    -6.272  47.192 -19.488  1.00 26.55      C
ATOM   4385  CD1 PHE C 139    -5.900  46.150 -18.662  1.00 26.08      C
ATOM   4386  CD2 PHE C 139    -5.538  47.414 -20.651  1.00 26.27      C
ATOM   4387  CE1 PHE C 139    -4.803  45.348 -18.968  1.00 26.16      C
ATOM   4388  CE2 PHE C 139    -4.455  46.612 -20.973  1.00 24.06      C
ATOM   4389  CZ  PHE C 139    -4.092  45.567 -20.131  1.00 26.49      C
ATOM   4390  N   TYR C 140    -7.599  48.236 -16.100  1.00 31.03      N
ATOM   4391  CA  TYR C 140    -7.318  47.643 -14.791  1.00 32.53      C
ATOM   4392  C   TYR C 140    -8.413  46.622 -14.451  1.00 33.15      C
ATOM   4393  O   TYR C 140    -9.590  46.920 -14.618  1.00 32.39      O
ATOM   4394  CB  TYR C 140    -7.258  48.723 -13.715  1.00 33.27      C
ATOM   4395  CG  TYR C 140    -6.720  48.166 -12.428  1.00 34.17      C
ATOM   4396  CD1 TYR C 140    -7.558  47.498 -11.537  1.00 35.23      C
ATOM   4397  CD2 TYR C 140    -5.370  48.251 -12.124  1.00 34.94      C
ATOM   4398  CE1 TYR C 140    -7.063  46.944 -10.370  1.00 34.69      C
ATOM   4399  CE2 TYR C 140    -4.866  47.702 -10.954  1.00 34.92      C
ATOM   4400  CZ  TYR C 140    -5.723  47.051 -10.087  1.00 34.52      C
ATOM   4401  OH  TYR C 140    -5.243  46.501  -8.927  1.00 34.53      O
ATOM   4402  N   PRO C 141    -8.046  45.421 -13.966  1.00 33.93      N
ATOM   4403  CA  PRO C 141    -6.733  44.874 -13.606  1.00 34.35      C
ATOM   4404  C   PRO C 141    -5.879  44.438 -14.810  1.00 34.21      C
ATOM   4405  O   PRO C 141    -6.327  44.537 -15.950  1.00 32.11      O
ATOM   4406  CB  PRO C 141    -7.100  43.673 -12.748  1.00 34.52      C
ATOM   4407  CG  PRO C 141    -8.398  43.197 -13.334  1.00 34.46      C
ATOM   4408  CD  PRO C 141    -9.123  44.439 -13.731  1.00 34.23      C
```

```
ATOM   4409  N    LYS C 142    -4.678  43.928 -14.539  1.00 34.60        N
ATOM   4410  CA   LYS C 142    -3.655  43.706 -15.580  1.00 35.23        C
ATOM   4411  C    LYS C 142    -3.969  42.585 -16.567  1.00 35.11        C
ATOM   4412  O    LYS C 142    -3.524  42.626 -17.711  1.00 34.72        O
ATOM   4413  CB   LYS C 142    -2.277  43.441 -14.936  1.00 35.69        C
ATOM   4414  CG   LYS C 142    -1.098  43.407 -15.938  1.00 36.37        C
ATOM   4415  CD   LYS C 142     0.267  43.288 -15.237  1.00 36.62        C
ATOM   4416  CE   LYS C 142     1.431  43.723 -16.157  1.00 38.04        C
ATOM   4417  NZ   LYS C 142     2.733  44.055 -15.437  1.00 38.47        N
ATOM   4418  N    ASP C 143    -4.714  41.575 -16.137  1.00 35.40        N
ATOM   4419  CA   ASP C 143    -4.950  40.417 -16.998  1.00 35.86        C
ATOM   4420  C    ASP C 143    -5.987  40.726 -18.086  1.00 34.47        C
ATOM   4421  O    ASP C 143    -6.981  41.383 -17.843  1.00 33.45        O
ATOM   4422  CB   ASP C 143    -5.335  39.187 -16.172  1.00 37.04        C
ATOM   4423  CG   ASP C 143    -4.143  38.612 -15.388  1.00 40.12        C
ATOM   4424  OD1  ASP C 143    -3.109  38.253 -16.020  1.00 41.22        O
ATOM   4425  OD2  ASP C 143    -4.237  38.536 -14.138  1.00 42.50        O
ATOM   4426  N    ILE C 144    -5.701  40.267 -19.297  1.00 34.11        N
ATOM   4427  CA   ILE C 144    -6.533  40.558 -20.459  1.00 33.56        C
ATOM   4428  C    ILE C 144    -6.175  39.593 -21.563  1.00 33.54        C
ATOM   4429  O    ILE C 144    -5.043  39.113 -21.617  1.00 34.96        O
ATOM   4430  CB   ILE C 144    -6.320  42.009 -20.948  1.00 33.43        C
ATOM   4431  CG1  ILE C 144    -7.431  42.427 -21.912  1.00 32.98        C
ATOM   4432  CG2  ILE C 144    -4.938  42.198 -21.620  1.00 32.48        C
ATOM   4433  CD1  ILE C 144    -7.502  43.905 -22.079  1.00 33.16        C
ATOM   4434  N    ASN C 145    -7.133  39.307 -22.437  1.00 33.80        N
ATOM   4435  CA   ASN C 145    -6.896  38.431 -23.580  1.00 34.30        C
ATOM   4436  C    ASN C 145    -7.218  39.157 -24.877  1.00 32.94        C
ATOM   4437  O    ASN C 145    -8.142  39.978 -24.935  1.00 32.50        O
ATOM   4438  CB   ASN C 145    -7.703  37.135 -23.467  1.00 35.84        C
ATOM   4439  CG   ASN C 145    -6.991  36.072 -22.636  1.00 38.86        C
ATOM   4440  OD1  ASN C 145    -6.837  34.933 -23.074  1.00 42.21        O
ATOM   4441  ND2  ASN C 145    -6.556  36.439 -21.440  1.00 40.56        N
ATOM   4442  N    VAL C 146    -6.416  38.876 -25.899  1.00 32.45        N
ATOM   4443  CA   VAL C 146    -6.604  39.469 -27.209  1.00 32.20        C
ATOM   4444  C    VAL C 146    -6.715  38.351 -28.223  1.00 31.49        C
ATOM   4445  O    VAL C 146    -6.019  37.343 -28.133  1.00 31.11        O
ATOM   4446  CB   VAL C 146    -5.450  40.446 -27.581  1.00 32.96        C
ATOM   4447  CG1  VAL C 146    -4.170  39.675 -27.931  1.00 33.65        C
ATOM   4448  CG2  VAL C 146    -5.867  41.371 -28.733  1.00 33.08        C
ATOM   4449  N    LYS C 147    -7.617  38.533 -29.179  1.00 31.53        N
ATOM   4450  CA   LYS C 147    -7.823  37.568 -30.236  1.00 30.81        C
ATOM   4451  C    LYS C 147    -7.763  38.301 -31.573  1.00 29.81        C
ATOM   4452  O    LYS C 147    -8.331  39.386 -31.694  1.00 30.37        O
ATOM   4453  CB   LYS C 147    -9.185  36.899 -30.069  1.00 32.96        C
ATOM   4454  CG   LYS C 147    -9.386  35.684 -30.968  1.00 35.60        C
ATOM   4455  CD   LYS C 147   -10.847  35.496 -31.358  1.00 37.30        C
ATOM   4456  CE   LYS C 147   -11.730  35.137 -30.155  1.00 39.49        C
ATOM   4457  NZ   LYS C 147   -13.197  35.418 -30.424  1.00 39.91        N
ATOM   4458  N    TRP C 148    -7.064  37.699 -32.542  1.00 27.64        N
ATOM   4459  CA   TRP C 148    -6.946  38.205 -33.921  1.00 27.27        C
ATOM   4460  C    TRP C 148    -7.745  37.329 -34.860  1.00 26.72        C
ATOM   4461  O    TRP C 148    -7.675  36.096 -34.775  1.00 26.77        O
ATOM   4462  CB   TRP C 148    -5.487  38.201 -34.394  1.00 25.26        C
ATOM   4463  CG   TRP C 148    -4.699  39.316 -33.812  1.00 24.71        C
ATOM   4464  CD1  TRP C 148    -3.910  39.265 -32.702  1.00 23.92        C
ATOM   4465  CD2  TRP C 148    -4.648  40.671 -34.283  1.00 24.30        C
ATOM   4466  NE1  TRP C 148    -3.375  40.499 -32.451  1.00 25.44        N
ATOM   4467  CE2  TRP C 148    -3.803  41.382 -33.408  1.00 25.03        C
ATOM   4468  CE3  TRP C 148    -5.236  41.355 -35.361  1.00 24.04        C
ATOM   4469  CZ2  TRP C 148    -3.513  42.744 -33.584  1.00 24.44        C
ATOM   4470  CZ3  TRP C 148    -4.960  42.696 -35.521  1.00 23.99        C
ATOM   4471  CH2  TRP C 148    -4.107  43.377 -34.640  1.00 24.07        C
ATOM   4472  N    LYS C 149    -8.496  37.968 -35.753  1.00 26.28        N
ATOM   4473  CA   LYS C 149    -9.210  37.268 -36.819  1.00 26.85        C
```

327

```
ATOM   4474   C    LYS C 149    -8.868  37.878 -38.174  1.00 25.21          C
ATOM   4475   O    LYS C 149    -8.846  39.109 -38.332  1.00 23.91          O
ATOM   4476   CB   LYS C 149   -10.733  37.338 -36.636  1.00 26.97          C
ATOM   4477   CG   LYS C 149   -11.295  36.689 -35.385  1.00 29.06          C
ATOM   4478   CD   LYS C 149   -12.800  36.585 -35.525  1.00 30.29          C
ATOM   4479   CE   LYS C 149   -13.482  36.197 -34.226  1.00 33.18          C
ATOM   4480   NZ   LYS C 149   -14.897  36.704 -34.234  1.00 35.00          N
ATOM   4481   N    ILE C 150    -8.598  37.007 -39.136  1.00 24.42          N
ATOM   4482   CA   ILE C 150    -8.353  37.397 -40.514  1.00 24.24          C
ATOM   4483   C    ILE C 150    -9.499  36.765 -41.300  1.00 24.64          C
ATOM   4484   O    ILE C 150    -9.672  35.544 -41.271  1.00 24.63          O
ATOM   4485   CB   ILE C 150    -6.996  36.878 -41.020  1.00 24.41          C
ATOM   4486   CG1  ILE C 150    -5.866  37.461 -40.162  1.00 24.94          C
ATOM   4487   CG2  ILE C 150    -6.803  37.216 -42.524  1.00 24.23          C
ATOM   4488   CD1  ILE C 150    -4.445  36.965 -40.510  1.00 25.85          C
ATOM   4489   N    ASP C 151   -10.286  37.607 -41.957  1.00 24.30          N
ATOM   4490   CA   ASP C 151   -11.496  37.178 -42.664  1.00 26.23          C
ATOM   4491   C    ASP C 151   -12.330  36.234 -41.799  1.00 26.75          C
ATOM   4492   O    ASP C 151   -12.785  35.190 -42.251  1.00 27.01          O
ATOM   4493   CB   ASP C 151   -11.144  36.567 -44.020  1.00 26.60          C
ATOM   4494   CG   ASP C 151   -10.783  37.615 -45.050  1.00 26.44          C
ATOM   4495   OD1  ASP C 151   -11.053  38.809 -44.837  1.00 29.15          O
ATOM   4496   OD2  ASP C 151   -10.230  37.236 -46.093  1.00 31.04          O
ATOM   4497   N    GLY C 152   -12.509  36.641 -40.542  1.00 26.79          N
ATOM   4498   CA   GLY C 152   -13.337  35.928 -39.597  1.00 28.15          C
ATOM   4499   C    GLY C 152   -12.727  34.676 -39.006  1.00 29.99          C
ATOM   4500   O    GLY C 152   -13.349  34.031 -38.162  1.00 30.01          O
ATOM   4501   N    SER C 153   -11.530  34.311 -39.456  1.00 31.76          N
ATOM   4502   CA   SER C 153   -10.851  33.147 -38.916  1.00 33.28          C
ATOM   4503   C    SER C 153    -9.841  33.590 -37.887  1.00 34.55          C
ATOM   4504   O    SER C 153    -9.087  34.524 -38.114  1.00 33.68          O
ATOM   4505   CB   SER C 153   -10.161  32.358 -40.021  1.00 33.80          C
ATOM   4506   OG   SER C 153   -11.125  31.830 -40.915  1.00 35.38          O
ATOM   4507   N    GLU C 154    -9.835  32.899 -36.758  1.00 36.65          N
ATOM   4508   CA   GLU C 154    -8.941  33.202 -35.667  1.00 39.40          C
ATOM   4509   C    GLU C 154    -7.497  32.922 -36.072  1.00 39.26          C
ATOM   4510   O    GLU C 154    -7.208  31.917 -36.711  1.00 38.97          O
ATOM   4511   CB   GLU C 154    -9.338  32.366 -34.459  1.00 40.52          C
ATOM   4512   CG   GLU C 154    -8.737  32.822 -33.155  1.00 43.33          C
ATOM   4513   CD   GLU C 154    -9.187  31.954 -32.009  1.00 43.33          C
ATOM   4514   OE1  GLU C 154   -10.289  32.211 -31.467  1.00 46.44          O
ATOM   4515   OE2  GLU C 154    -8.427  31.019 -31.660  1.00 47.71          O
ATOM   4516   N    ARG C 155    -6.597  33.826 -35.705  1.00 40.59          N
ATOM   4517   CA   ARG C 155    -5.196  33.734 -36.083  1.00 41.59          C
ATOM   4518   C    ARG C 155    -4.329  33.839 -34.824  1.00 42.77          C
ATOM   4519   O    ARG C 155    -4.434  34.810 -34.082  1.00 41.90          O
ATOM   4520   CB   ARG C 155    -4.878  34.853 -37.078  1.00 42.32          C
ATOM   4521   CG   ARG C 155    -3.420  35.004 -37.454  1.00 43.70          C
ATOM   4522   CD   ARG C 155    -2.804  33.675 -37.797  1.00 45.89          C
ATOM   4523   NE   ARG C 155    -1.635  33.830 -38.647  1.00 46.68          N
ATOM   4524   CZ   ARG C 155    -1.660  33.822 -39.975  1.00 48.22          C
ATOM   4525   NH1  ARG C 155    -2.804  33.658 -40.647  1.00 49.18          N
ATOM   4526   NH2  ARG C 155    -0.517  33.970 -40.635  1.00 48.67          N
ATOM   4527   N    GLN C 156    -3.477  32.843 -34.580  1.00 44.33          N
ATOM   4528   CA   GLN C 156    -2.704  32.789 -33.329  1.00 45.84          C
ATOM   4529   C    GLN C 156    -1.191  32.902 -33.486  1.00 45.46          C
ATOM   4530   O    GLN C 156    -0.535  33.543 -32.656  1.00 46.31          O
ATOM   4531   CB   GLN C 156    -3.053  31.523 -32.551  1.00 46.13          C
ATOM   4532   CG   GLN C 156    -4.523  31.446 -32.205  1.00 47.68          C
ATOM   4533   CD   GLN C 156    -4.928  30.116 -31.608  1.00 48.12          C
ATOM   4534   OE1  GLN C 156    -5.622  30.073 -30.587  1.00 51.10          O
ATOM   4535   NE2  GLN C 156    -4.506  29.021 -32.242  1.00 49.53          N
ATOM   4536   N    ASN C 157    -0.625  32.288 -34.522  1.00 44.53          N
ATOM   4537   CA   ASN C 157     0.806  32.439 -34.764  1.00 43.77          C
ATOM   4538   C    ASN C 157     1.074  33.763 -35.493  1.00 41.25          C
```

| ATOM | 4539 | O | ASN C 157 | 0.200 | 34.283 | -36.186 | 1.00 | 41.56 | O |
|------|------|-----|-----------|--------|--------|---------|------|-------|---|
| ATOM | 4540 | CB | ASN C 157 | 1.378 | 31.244 | -35.536 | 1.00 | 45.26 | C |
| ATOM | 4541 | CG | ASN C 157 | 1.467 | 29.963 | -34.680 | 1.00 | 47.49 | C |
| ATOM | 4542 | OD1 | ASN C 157 | 2.316 | 29.847 | -33.767 | 1.00 | 48.92 | O |
| ATOM | 4543 | ND2 | ASN C 157 | 0.602 | 28.989 | -34.988 | 1.00 | 47.98 | N |
| ATOM | 4544 | N | GLY C 158 | 2.271 | 34.316 | -35.304 | 1.00 | 38.00 | N |
| ATOM | 4545 | CA | GLY C 158 | 2.621 | 35.630 | -35.842 | 1.00 | 34.75 | C |
| ATOM | 4546 | C | GLY C 158 | 2.175 | 36.773 | -34.949 | 1.00 | 32.11 | C |
| ATOM | 4547 | O | GLY C 158 | 2.334 | 37.928 | -35.316 | 1.00 | 30.71 | O |
| ATOM | 4548 | N | VAL C 159 | 1.663 | 36.449 | -33.762 | 1.00 | 30.00 | N |
| ATOM | 4549 | CA | VAL C 159 | 1.201 | 37.438 | -32.795 | 1.00 | 28.43 | C |
| ATOM | 4550 | C | VAL C 159 | 2.240 | 37.716 | -31.703 | 1.00 | 28.22 | C |
| ATOM | 4551 | O | VAL C 159 | 2.797 | 36.788 | -31.125 | 1.00 | 28.26 | O |
| ATOM | 4552 | CB | VAL C 159 | -0.100 | 36.955 | -32.129 | 1.00 | 28.74 | C |
| ATOM | 4553 | CG1 | VAL C 159 | -0.615 | 37.980 | -31.120 | 1.00 | 28.68 | C |
| ATOM | 4554 | CG2 | VAL C 159 | -1.154 | 36.654 | -33.204 | 1.00 | 28.02 | C |
| ATOM | 4555 | N | LEU C 160 | 2.484 | 38.988 | -31.414 | 1.00 | 26.37 | N |
| ATOM | 4556 | CA | LEU C 160 | 3.438 | 39.387 | -30.375 | 1.00 | 26.72 | C |
| ATOM | 4557 | C | LEU C 160 | 2.744 | 40.334 | -29.397 | 1.00 | 25.64 | C |
| ATOM | 4558 | O | LEU C 160 | 2.238 | 41.381 | -29.814 | 1.00 | 26.33 | O |
| ATOM | 4559 | CB | LEU C 160 | 4.620 | 40.081 | -31.038 | 1.00 | 27.27 | C |
| ATOM | 4560 | CG | LEU C 160 | 5.842 | 40.475 | -30.206 | 1.00 | 27.69 | C |
| ATOM | 4561 | CD1 | LEU C 160 | 7.069 | 40.457 | -31.124 | 1.00 | 29.81 | C |
| ATOM | 4562 | CD2 | LEU C 160 | 5.679 | 41.832 | -29.507 | 1.00 | 29.19 | C |
| ATOM | 4563 | N | ASN C 161 | 2.734 | 39.980 | -28.110 | 1.00 | 24.50 | N |
| ATOM | 4564 | CA | ASN C 161 | 2.031 | 40.756 | -27.076 | 1.00 | 24.42 | C |
| ATOM | 4565 | C | ASN C 161 | 3.002 | 41.323 | -26.025 | 1.00 | 24.53 | C |
| ATOM | 4566 | O | ASN C 161 | 3.933 | 40.634 | -25.602 | 1.00 | 24.38 | O |
| ATOM | 4567 | CB | ASN C 161 | 0.998 | 39.867 | -26.375 | 1.00 | 24.98 | C |
| ATOM | 4568 | CG | ASN C 161 | -0.148 | 39.440 | -27.284 | 1.00 | 27.05 | C |
| ATOM | 4569 | OD1 | ASN C 161 | -0.712 | 38.352 | -27.122 | 1.00 | 30.76 | O |
| ATOM | 4570 | ND2 | ASN C 161 | -0.497 | 40.280 | -28.232 | 1.00 | 25.64 | N |
| ATOM | 4571 | N | SER C 162 | 2.780 | 42.559 | -25.591 | 1.00 | 23.46 | N |
| ATOM | 4572 | CA | SER C 162 | 3.659 | 43.202 | -24.618 | 1.00 | 23.42 | C |
| ATOM | 4573 | C | SER C 162 | 2.843 | 44.100 | -23.683 | 1.00 | 23.35 | C |
| ATOM | 4574 | O | SER C 162 | 2.039 | 44.900 | -24.147 | 1.00 | 22.78 | O |
| ATOM | 4575 | CB | SER C 162 | 4.741 | 44.008 | -25.361 | 1.00 | 23.58 | C |
| ATOM | 4576 | OG | SER C 162 | 5.577 | 44.763 | -24.497 | 1.00 | 23.86 | O |
| ATOM | 4577 | N | TRP C 163 | 3.055 | 43.949 | -22.373 | 1.00 | 23.52 | N |
| ATOM | 4578 | CA | TRP C 163 | 2.397 | 44.771 | -21.341 | 1.00 | 23.54 | C |
| ATOM | 4579 | C | TRP C 163 | 3.348 | 45.815 | -20.758 | 1.00 | 23.81 | C |
| ATOM | 4580 | O | TRP C 163 | 4.495 | 45.518 | -20.480 | 1.00 | 21.69 | O |
| ATOM | 4581 | CB | TRP C 163 | 1.938 | 43.899 | -20.158 | 1.00 | 25.51 | C |
| ATOM | 4582 | CG | TRP C 163 | 0.664 | 43.127 | -20.366 | 1.00 | 26.64 | C |
| ATOM | 4583 | CD1 | TRP C 163 | -0.565 | 43.394 | -19.805 | 1.00 | 26.83 | C |
| ATOM | 4584 | CD2 | TRP C 163 | 0.496 | 41.938 | -21.148 | 1.00 | 25.82 | C |
| ATOM | 4585 | NE1 | TRP C 163 | -1.474 | 42.439 | -20.203 | 1.00 | 26.81 | N |
| ATOM | 4586 | CE2 | TRP C 163 | -0.854 | 41.549 | -21.034 | 1.00 | 26.80 | C |
| ATOM | 4587 | CE3 | TRP C 163 | 1.352 | 41.171 | -21.935 | 1.00 | 26.00 | C |
| ATOM | 4588 | CZ2 | TRP C 163 | -1.364 | 40.423 | -21.687 | 1.00 | 26.48 | C |
| ATOM | 4589 | CZ3 | TRP C 163 | 0.841 | 40.056 | -22.582 | 1.00 | 26.81 | C |
| ATOM | 4590 | CH2 | TRP C 163 | -0.494 | 39.699 | -22.459 | 1.00 | 26.47 | C |
| ATOM | 4591 | N | THR C 164 | 2.852 | 47.023 | -20.524 | 1.00 | 23.95 | N |
| ATOM | 4592 | CA | THR C 164 | 3.596 | 48.036 | -19.792 | 1.00 | 24.64 | C |
| ATOM | 4593 | C | THR C 164 | 3.629 | 47.673 | -18.309 | 1.00 | 24.74 | C |
| ATOM | 4594 | O | THR C 164 | 2.882 | 46.820 | -17.859 | 1.00 | 25.03 | O |
| ATOM | 4595 | CB | THR C 164 | 2.891 | 49.385 | -19.860 | 1.00 | 25.48 | C |
| ATOM | 4596 | OG1 | THR C 164 | 1.516 | 49.201 | -19.495 | 1.00 | 25.08 | O |
| ATOM | 4597 | CG2 | THR C 164 | 2.977 | 49.986 | -21.256 | 1.00 | 27.17 | C |
| ATOM | 4598 | N | ASP C 165 | 4.477 | 48.355 | -17.558 | 1.00 | 25.42 | N |
| ATOM | 4599 | CA | ASP C 165 | 4.377 | 48.352 | -16.104 | 1.00 | 25.99 | C |
| ATOM | 4600 | C | ASP C 165 | 3.230 | 49.267 | -15.704 | 1.00 | 25.90 | C |
| ATOM | 4601 | O | ASP C 165 | 2.718 | 50.021 | -16.528 | 1.00 | 27.13 | O |
| ATOM | 4602 | CB | ASP C 165 | 5.668 | 48.856 | -15.470 | 1.00 | 27.13 | C |
| ATOM | 4603 | CG | ASP C 165 | 6.856 | 47.998 | -15.819 | 1.00 | 28.01 | C |

| | | | | | | | | | |
|---|---|---|---|---|---|---|---|---|---|
| ATOM | 4604 | OD1 | ASP | C | 165 | 6.784 | 46.770 | -15.625 | 1.00 30.10 | O |
| ATOM | 4605 | OD2 | ASP | C | 165 | 7.862 | 48.562 | -16.296 | 1.00 31.76 | O |
| ATOM | 4606 | N | GLN | C | 166 | 2.832 | 49.203 | -14.439 | 1.00 25.34 | N |
| ATOM | 4607 | CA | GLN | C | 166 | 1.733 | 50.018 | -13.945 | 1.00 25.16 | C |
| ATOM | 4608 | C | GLN | C | 166 | 2.070 | 51.476 | -14.131 | 1.00 24.82 | C |
| ATOM | 4609 | O | GLN | C | 166 | 3.178 | 51.899 | -13.853 | 1.00 23.24 | O |
| ATOM | 4610 | CB | GLN | C | 166 | 1.470 | 49.726 | -12.461 | 1.00 25.75 | C |
| ATOM | 4611 | CG | GLN | C | 166 | 0.215 | 50.395 | -11.904 | 1.00 25.15 | C |
| ATOM | 4612 | CD | GLN | C | 166 | -0.283 | 49.679 | -10.666 | 1.00 25.04 | C |
| ATOM | 4613 | OE1 | GLN | C | 166 | 0.512 | 49.226 | -9.833 | 1.00 23.50 | O |
| ATOM | 4614 | NE2 | GLN | C | 166 | -1.599 | 49.560 | -10.541 | 1.00 23.18 | N |
| ATOM | 4615 | N | ASP | C | 167 | 1.108 | 52.240 | -14.632 | 1.00 26.16 | N |
| ATOM | 4616 | CA | ASP | C | 167 | 1.340 | 53.648 | -14.947 | 1.00 27.07 | C |
| ATOM | 4617 | C | ASP | C | 167 | 1.528 | 54.485 | -13.686 | 1.00 28.56 | C |
| ATOM | 4618 | O | ASP | C | 167 | 0.791 | 54.343 | -12.717 | 1.00 27.23 | O |
| ATOM | 4619 | CB | ASP | C | 167 | 0.170 | 54.180 | -15.751 | 1.00 28.10 | C |
| ATOM | 4620 | CG | ASP | C | 167 | 0.457 | 55.516 | -16.361 | 1.00 30.25 | C |
| ATOM | 4621 | OD1 | ASP | C | 167 | 0.824 | 55.547 | -17.547 | 1.00 34.16 | O |
| ATOM | 4622 | OD2 | ASP | C | 167 | 0.346 | 56.536 | -15.652 | 1.00 32.74 | O |
| ATOM | 4623 | N | SER | C | 168 | 2.509 | 55.378 | -13.727 | 1.00 30.41 | N |
| ATOM | 4624 | CA | SER | C | 168 | 2.870 | 56.227 | -12.582 | 1.00 32.12 | C |
| ATOM | 4625 | C | SER | C | 168 | 1.805 | 57.260 | -12.231 | 1.00 33.17 | C |
| ATOM | 4626 | O | SER | C | 168 | 1.804 | 57.797 | -11.116 | 1.00 32.42 | O |
| ATOM | 4627 | CB | SER | C | 168 | 4.185 | 56.966 | -12.868 | 1.00 32.20 | C |
| ATOM | 4628 | OG | SER | C | 168 | 5.315 | 56.224 | -12.429 | 1.00 35.06 | O |
| ATOM | 4629 | N | LYS | C | 169 | 0.930 | 57.552 | -13.196 | 1.00 34.40 | N |
| ATOM | 4630 | CA | LYS | C | 169 | -0.080 | 58.597 | -13.062 | 1.00 35.95 | C |
| ATOM | 4631 | C | LYS | C | 169 | -1.424 | 58.034 | -12.704 | 1.00 35.25 | C |
| ATOM | 4632 | O | LYS | C | 169 | -2.000 | 58.417 | -11.700 | 1.00 36.73 | O |
| ATOM | 4633 | CB | LYS | C | 169 | -0.215 | 59.398 | -14.362 | 1.00 37.25 | C |
| ATOM | 4634 | CG | LYS | C | 169 | 0.776 | 60.523 | -14.491 | 1.00 39.95 | C |
| ATOM | 4635 | CD | LYS | C | 169 | 2.155 | 60.036 | -14.870 | 1.00 42.30 | C |
| ATOM | 4636 | CE | LYS | C | 169 | 3.069 | 61.221 | -15.122 | 1.00 43.96 | C |
| ATOM | 4637 | NZ | LYS | C | 169 | 3.141 | 62.096 | -13.914 | 1.00 45.15 | N |
| ATOM | 4638 | N | ASP | C | 170 | -1.945 | 57.137 | -13.530 | 1.00 34.47 | N |
| ATOM | 4639 | CA | ASP | C | 170 | -3.303 | 56.641 | -13.325 | 1.00 33.19 | C |
| ATOM | 4640 | C | ASP | C | 170 | -3.362 | 55.226 | -12.811 | 1.00 31.63 | C |
| ATOM | 4641 | O | ASP | C | 170 | -4.448 | 54.697 | -12.621 | 1.00 30.98 | O |
| ATOM | 4642 | CB | ASP | C | 170 | -4.147 | 56.779 | -14.608 | 1.00 34.66 | C |
| ATOM | 4643 | CG | ASP | C | 170 | -3.653 | 55.910 | -15.755 | 1.00 35.69 | C |
| ATOM | 4644 | OD1 | ASP | C | 170 | -2.955 | 54.900 | -15.520 | 1.00 35.70 | O |
| ATOM | 4645 | OD2 | ASP | C | 170 | -3.978 | 56.247 | -16.917 | 1.00 37.97 | O |
| ATOM | 4646 | N | SER | C | 171 | -2.202 | 54.599 | -12.610 | 1.00 29.96 | N |
| ATOM | 4647 | CA | SER | C | 171 | -2.136 | 53.252 | -12.030 | 1.00 28.36 | C |
| ATOM | 4648 | C | SER | C | 171 | -2.798 | 52.170 | -12.886 | 1.00 26.94 | C |
| ATOM | 4649 | O | SER | C | 171 | -3.200 | 51.131 | -12.364 | 1.00 26.73 | O |
| ATOM | 4650 | CB | SER | C | 171 | -2.728 | 53.252 | -10.603 | 1.00 28.72 | C |
| ATOM | 4651 | OG | SER | C | 171 | -2.243 | 54.339 | -9.823 | 1.00 27.06 | O |
| ATOM | 4652 | N | THR | C | 172 | -2.899 | 52.410 | -14.201 | 1.00 26.14 | N |
| ATOM | 4653 | CA | THR | C | 172 | -3.458 | 51.431 | -15.133 | 1.00 26.43 | C |
| ATOM | 4654 | C | THR | C | 172 | -2.359 | 50.625 | -15.828 | 1.00 25.14 | C |
| ATOM | 4655 | O | THR | C | 172 | -1.171 | 50.913 | -15.680 | 1.00 24.20 | O |
| ATOM | 4656 | CB | THR | C | 172 | -4.331 | 52.102 | -16.246 | 1.00 26.89 | C |
| ATOM | 4657 | OG1 | THR | C | 172 | -3.595 | 53.154 | -16.885 | 1.00 27.75 | O |
| ATOM | 4658 | CG2 | THR | C | 172 | -5.622 | 52.658 | -15.675 | 1.00 28.37 | C |
| ATOM | 4659 | N | TYR | C | 173 | -2.774 | 49.612 | -16.578 | 1.00 24.93 | N |
| ATOM | 4660 | CA | TYR | C | 173 | -1.873 | 48.894 | -17.470 | 1.00 25.12 | C |
| ATOM | 4661 | C | TYR | C | 173 | -2.307 | 49.103 | -18.911 | 1.00 25.16 | C |
| ATOM | 4662 | O | TYR | C | 173 | -3.456 | 49.470 | -19.181 | 1.00 24.91 | O |
| ATOM | 4663 | CB | TYR | C | 173 | -1.894 | 47.410 | -17.161 | 1.00 26.75 | C |
| ATOM | 4664 | CG | TYR | C | 173 | -1.449 | 47.119 | -15.765 | 1.00 26.45 | C |
| ATOM | 4665 | CD1 | TYR | C | 173 | -2.355 | 47.146 | -14.716 | 1.00 27.65 | C |
| ATOM | 4666 | CD2 | TYR | C | 173 | -0.122 | 46.831 | -15.487 | 1.00 28.93 | C |
| ATOM | 4667 | CE1 | TYR | C | 173 | -1.957 | 46.892 | -13.421 | 1.00 28.56 | C |
| ATOM | 4668 | CE2 | TYR | C | 173 | 0.288 | 46.563 | -14.186 | 1.00 28.41 | C |

```
ATOM   4669  CZ   TYR C 173     -0.638  46.601 -13.160  1.00 28.77           C
ATOM   4670  OH   TYR C 173     -0.249  46.343 -11.863  1.00 27.90           O
ATOM   4671  N    SER C 174     -1.377  48.866 -19.826  1.00 24.47           N
ATOM   4672  CA   SER C 174     -1.681  48.838 -21.249  1.00 24.32           C
ATOM   4673  C    SER C 174     -0.980  47.659 -21.874  1.00 24.73           C
ATOM   4674  O    SER C 174     -0.015  47.103 -21.308  1.00 23.41           O
ATOM   4675  CB   SER C 174     -1.225  50.122 -21.926  1.00 24.39           C
ATOM   4676  OG   SER C 174     -1.835  51.234 -21.300  1.00 24.59           O
ATOM   4677  N    MET C 175     -1.470  47.279 -23.048  1.00 23.83           N
ATOM   4678  CA   MET C 175     -0.984  46.116 -23.736  1.00 24.73           C
ATOM   4679  C    MET C 175     -0.968  46.387 -25.239  1.00 24.21           C
ATOM   4680  O    MET C 175     -1.854  47.069 -25.774  1.00 22.47           O
ATOM   4681  CB   MET C 175     -1.892  44.948 -23.409  1.00 27.02           C
ATOM   4682  CG   MET C 175     -1.344  43.593 -23.752  1.00 30.76           C
ATOM   4683  SD   MET C 175     -1.784  43.031 -25.394  1.00 35.65           S
ATOM   4684  CE   MET C 175     -2.510  41.429 -25.032  1.00 33.18           C
ATOM   4685  N    SER C 176      0.054  45.871 -25.909  1.00 22.38           N
ATOM   4686  CA   SER C 176      0.177  46.038 -27.359  1.00 22.39           C
ATOM   4687  C    SER C 176      0.221  44.656 -27.944  1.00 20.99           C
ATOM   4688  O    SER C 176      0.922  43.776 -27.419  1.00 21.73           O
ATOM   4689  CB   SER C 176      1.430  46.834 -27.717  1.00 22.20           C
ATOM   4690  OG   SER C 176      1.757  46.739 -29.095  1.00 24.47           O
ATOM   4691  N    SER C 177     -0.533  44.465 -29.019  1.00 19.60           N
ATOM   4692  CA   SER C 177     -0.583  43.198 -29.730  1.00 19.93           C
ATOM   4693  C    SER C 177     -0.280  43.457 -31.198  1.00 20.54           C
ATOM   4694  O    SER C 177     -0.933  44.279 -31.832  1.00 18.28           O
ATOM   4695  CB   SER C 177     -1.960  42.559 -29.587  1.00 20.95           C
ATOM   4696  OG   SER C 177     -1.952  41.242 -30.149  1.00 21.41           O
ATOM   4697  N    THR C 178      0.725  42.765 -31.728  1.00 21.33           N
ATOM   4698  CA   THR C 178      1.123  42.937 -33.127  1.00 21.93           C
ATOM   4699  C    THR C 178      1.044  41.618 -33.852  1.00 22.18           C
ATOM   4700  O    THR C 178      1.639  40.624 -33.434  1.00 22.07           O
ATOM   4701  CB   THR C 178      2.553  43.492 -33.257  1.00 22.79           C
ATOM   4702  OG1  THR C 178      2.664  44.699 -32.505  1.00 22.70           O
ATOM   4703  CG2  THR C 178      2.911  43.781 -34.710  1.00 22.49           C
ATOM   4704  N    LEU C 179      0.293  41.616 -34.947  1.00 22.23           N
ATOM   4705  CA   LEU C 179      0.190  40.473 -35.827  1.00 22.55           C
ATOM   4706  C    LEU C 179      1.038  40.796 -37.032  1.00 23.11           C
ATOM   4707  O    LEU C 179      0.760  41.761 -37.736  1.00 20.75           O
ATOM   4708  CB   LEU C 179     -1.255  40.303 -36.284  1.00 23.92           C
ATOM   4709  CG   LEU C 179     -1.503  39.250 -37.357  1.00 24.48           C
ATOM   4710  CD1  LEU C 179     -1.336  37.860 -36.791  1.00 24.75           C
ATOM   4711  CD2  LEU C 179     -2.869  39.422 -37.959  1.00 24.88           C
ATOM   4712  N    THR C 180      2.072  40.006 -37.278  1.00 22.98           N
ATOM   4713  CA   THR C 180      2.949  40.279 -38.414  1.00 23.75           C
ATOM   4714  C    THR C 180      2.786  39.202 -39.477  1.00 23.60           C
ATOM   4715  O    THR C 180      2.832  38.000 -39.185  1.00 24.47           O
ATOM   4716  CB   THR C 180      4.410  40.439 -37.970  1.00 23.74           C
ATOM   4717  OG1  THR C 180      4.511  41.607 -37.152  1.00 25.02           O
ATOM   4718  CG2  THR C 180      5.332  40.600 -39.179  1.00 24.59           C
ATOM   4719  N    LEU C 181      2.567  39.654 -40.703  1.00 22.70           N
ATOM   4720  CA   LEU C 181      2.420  38.783 -41.862  1.00 23.10           C
ATOM   4721  C    LEU C 181      3.368  39.239 -42.952  1.00 22.02           C
ATOM   4722  O    LEU C 181      3.913  40.348 -42.895  1.00 22.13           O
ATOM   4723  CB   LEU C 181      0.996  38.878 -42.419  1.00 23.46           C
ATOM   4724  CG   LEU C 181     -0.189  38.650 -41.482  1.00 24.63           C
ATOM   4725  CD1  LEU C 181     -1.423  39.119 -42.171  1.00 24.76           C
ATOM   4726  CD2  LEU C 181     -0.295  37.184 -41.094  1.00 24.66           C
ATOM   4727  N    THR C 182      3.554  38.379 -43.943  1.00 22.20           N
ATOM   4728  CA   THR C 182      4.228  38.783 -45.181  1.00 22.24           C
ATOM   4729  C    THR C 182      3.227  39.597 -45.968  1.00 22.33           C
ATOM   4730  O    THR C 182      2.031  39.425 -45.799  1.00 21.12           O
ATOM   4731  CB   THR C 182      4.652  37.572 -46.042  1.00 22.12           C
ATOM   4732  OG1  THR C 182      3.497  36.838 -46.465  1.00 23.91           O
ATOM   4733  CG2  THR C 182      5.559  36.671 -45.277  1.00 23.32           C
```

| ATOM | 4734 | N | LYS C 183 | 3.721 | 40.498 | -46.811 | 1.00 | 22.99 | N |
|------|------|-----|-----------|-------|--------|---------|------|-------|---|
| ATOM | 4735 | CA | LYS C 183 | 2.874 | 41.261 | -47.726 | 1.00 | 23.36 | C |
| ATOM | 4736 | C | LYS C 183 | 1.976 | 40.324 | -48.535 | 1.00 | 23.55 | C |
| ATOM | 4737 | O | LYS C 183 | 0.786 | 40.545 | -48.668 | 1.00 | 22.37 | O |
| ATOM | 4738 | CB | LYS C 183 | 3.745 | 42.083 | -48.680 | 1.00 | 23.83 | C |
| ATOM | 4739 | CG | LYS C 183 | 2.955 | 42.905 | -49.683 | 1.00 | 24.56 | C |
| ATOM | 4740 | CD | LYS C 183 | 3.808 | 43.441 | -50.811 | 1.00 | 24.67 | C |
| ATOM | 4741 | CE | LYS C 183 | 2.957 | 44.185 | -51.852 | 1.00 | 25.75 | C |
| ATOM | 4742 | NZ | LYS C 183 | 3.689 | 44.457 | -53.129 | 1.00 | 26.79 | N |
| ATOM | 4743 | N | ASP C 184 | 2.576 | 39.283 | -49.091 | 1.00 | 24.84 | N |
| ATOM | 4744 | CA | ASP C 184 | 1.847 | 38.278 | -49.874 | 1.00 | 26.46 | C |
| ATOM | 4745 | C | ASP C 184 | 0.611 | 37.761 | -49.123 | 1.00 | 27.15 | C |
| ATOM | 4746 | O | ASP C 184 | -0.497 | 37.851 | -49.636 | 1.00 | 27.45 | O |
| ATOM | 4747 | CB | ASP C 184 | 2.807 | 37.141 | -50.256 | 1.00 | 27.37 | C |
| ATOM | 4748 | CG | ASP C 184 | 2.102 | 35.929 | -50.818 | 1.00 | 30.38 | C |
| ATOM | 4749 | OD1 | ASP C 184 | 1.249 | 36.087 | -51.721 | 1.00 | 30.78 | O |
| ATOM | 4750 | OD2 | ASP C 184 | 2.414 | 34.808 | -50.350 | 1.00 | 33.14 | O |
| ATOM | 4751 | N | GLU C 185 | 0.794 | 37.235 | -47.912 | 1.00 | 27.79 | N |
| ATOM | 4752 | CA | GLU C 185 | -0.335 | 36.753 | -47.110 | 1.00 | 29.50 | C |
| ATOM | 4753 | C | GLU C 185 | -1.335 | 37.842 | -46.749 | 1.00 | 27.91 | C |
| ATOM | 4754 | O | GLU C 185 | -2.550 | 37.614 | -46.759 | 1.00 | 28.04 | O |
| ATOM | 4755 | CB | GLU C 185 | 0.127 | 36.071 | -45.827 | 1.00 | 30.07 | C |
| ATOM | 4756 | CG | GLU C 185 | -1.061 | 35.405 | -45.127 | 1.00 | 34.14 | C |
| ATOM | 4757 | CD | GLU C 185 | -0.698 | 34.539 | -43.945 | 1.00 | 35.24 | C |
| ATOM | 4758 | OE1 | GLU C 185 | 0.409 | 34.705 | -43.362 | 1.00 | 39.95 | O |
| ATOM | 4759 | OE2 | GLU C 185 | -1.554 | 33.692 | -43.590 | 1.00 | 40.61 | O |
| ATOM | 4760 | N | TYR C 186 | -0.831 | 39.023 | -46.401 | 1.00 | 26.20 | N |
| ATOM | 4761 | CA | TYR C 186 | -1.691 | 40.138 | -46.029 | 1.00 | 24.26 | C |
| ATOM | 4762 | C | TYR C 186 | -2.648 | 40.506 | -47.152 | 1.00 | 24.71 | C |
| ATOM | 4763 | O | TYR C 186 | -3.808 | 40.807 | -46.891 | 1.00 | 25.30 | O |
| ATOM | 4764 | CB | TYR C 186 | -0.857 | 41.351 | -45.632 | 1.00 | 23.52 | C |
| ATOM | 4765 | CG | TYR C 186 | -1.640 | 42.608 | -45.369 | 1.00 | 22.81 | C |
| ATOM | 4766 | CD1 | TYR C 186 | -2.433 | 42.743 | -44.229 | 1.00 | 22.43 | C |
| ATOM | 4767 | CD2 | TYR C 186 | -1.568 | 43.677 | -46.243 | 1.00 | 21.39 | C |
| ATOM | 4768 | CE1 | TYR C 186 | -3.147 | 43.885 | -43.991 | 1.00 | 22.25 | C |
| ATOM | 4769 | CE2 | TYR C 186 | -2.253 | 44.830 | -46.010 | 1.00 | 21.81 | C |
| ATOM | 4770 | CZ | TYR C 186 | -3.052 | 44.940 | -44.878 | 1.00 | 22.85 | C |
| ATOM | 4771 | OH | TYR C 186 | -3.736 | 46.106 | -44.651 | 1.00 | 21.98 | O |
| ATOM | 4772 | N | GLU C 187 | -2.173 | 40.462 | -48.393 | 1.00 | 23.47 | N |
| ATOM | 4773 | CA | GLU C 187 | -2.987 | 40.905 | -49.523 | 1.00 | 25.30 | C |
| ATOM | 4774 | C | GLU C 187 | -3.896 | 39.806 | -50.075 | 1.00 | 25.62 | C |
| ATOM | 4775 | O | GLU C 187 | -4.613 | 40.026 | -51.050 | 1.00 | 26.23 | O |
| ATOM | 4776 | CB | GLU C 187 | -2.091 | 41.483 | -50.619 | 1.00 | 25.77 | C |
| ATOM | 4777 | CG | GLU C 187 | -1.296 | 42.634 | -50.110 | 1.00 | 26.64 | C |
| ATOM | 4778 | CD | GLU C 187 | -0.544 | 43.404 | -51.165 | 1.00 | 27.90 | C |
| ATOM | 4779 | OE1 | GLU C 187 | -0.038 | 42.790 | -52.147 | 1.00 | 28.03 | O |
| ATOM | 4780 | OE2 | GLU C 187 | -0.416 | 44.642 | -50.964 | 1.00 | 30.90 | O |
| ATOM | 4781 | N | ARG C 188 | -3.853 | 38.627 | -49.466 | 1.00 | 26.22 | N |
| ATOM | 4782 | CA | ARG C 188 | -4.773 | 37.554 | -49.830 | 1.00 | 27.85 | C |
| ATOM | 4783 | C | ARG C 188 | -6.075 | 37.606 | -49.007 | 1.00 | 27.63 | C |
| ATOM | 4784 | O | ARG C 188 | -6.973 | 36.803 | -49.237 | 1.00 | 27.85 | O |
| ATOM | 4785 | CB | AARG C 188 | -4.107 | 36.195 | -49.655 | 0.50 | 28.35 | C |
| ATOM | 4786 | CB | BARG C 188 | -4.096 | 36.194 | -49.668 | 0.50 | 28.26 | C |
| ATOM | 4787 | CG | AARG C 188 | -2.925 | 35.957 | -50.574 | 0.50 | 29.21 | C |
| ATOM | 4788 | CG | BARG C 188 | -3.006 | 35.911 | -50.694 | 0.50 | 28.94 | C |
| ATOM | 4789 | CD | AARG C 188 | -2.621 | 34.479 | -50.690 | 0.50 | 29.50 | C |
| ATOM | 4790 | CD | BARG C 188 | -2.284 | 34.612 | -50.374 | 0.50 | 29.13 | C |
| ATOM | 4791 | NE | AARG C 188 | -2.421 | 33.846 | -49.380 | 0.50 | 30.13 | N |
| ATOM | 4792 | NE | BARG C 188 | -1.191 | 34.324 | -51.301 | 0.50 | 28.79 | N |
| ATOM | 4793 | CZ | AARG C 188 | -1.243 | 33.497 | -48.854 | 0.50 | 29.79 | C |
| ATOM | 4794 | CZ | BARG C 188 | -1.342 | 33.863 | -52.542 | 0.50 | 29.70 | C |
| ATOM | 4795 | NH1 | AARG C 188 | -0.101 | 33.700 | -49.497 | 0.50 | 30.13 | N |
| ATOM | 4796 | NH1 | BARG C 188 | -2.548 | 33.622 | -53.049 | 0.50 | 29.97 | N |
| ATOM | 4797 | NH2 | AARG C 188 | -1.207 | 32.929 | -47.656 | 0.50 | 30.06 | N |
| ATOM | 4798 | NH2 | BARG C 188 | -0.269 | 33.639 | -53.292 | 0.50 | 30.62 | N |

| ATOM | 4799 | N   | HIS C 189 | -6.174  | 38.544 | -48.061 | 1.00 | 27.11 | N |
|------|------|-----|-----------|---------|--------|---------|------|-------|---|
| ATOM | 4800 | CA  | HIS C 189 | -7.379  | 38.692 | -47.226 | 1.00 | 27.47 | C |
| ATOM | 4801 | C   | HIS C 189 | -7.662  | 40.171 | -47.031 | 1.00 | 26.42 | C |
| ATOM | 4802 | O   | HIS C 189 | -6.771  | 40.981 | -47.213 | 1.00 | 24.28 | O |
| ATOM | 4803 | CB  | HIS C 189 | -7.149  | 38.015 | -45.884 | 1.00 | 28.67 | C |
| ATOM | 4804 | CG  | HIS C 189 | -6.766  | 36.577 | -46.011 | 1.00 | 31.09 | C |
| ATOM | 4805 | ND1 | HIS C 189 | -7.648  | 35.609 | -46.443 | 1.00 | 34.12 | N |
| ATOM | 4806 | CD2 | HIS C 189 | -5.585  | 35.947 | -45.810 | 1.00 | 34.42 | C |
| ATOM | 4807 | CE1 | HIS C 189 | -7.037  | 34.439 | -46.473 | 1.00 | 34.30 | C |
| ATOM | 4808 | NE2 | HIS C 189 | -5.780  | 34.616 | -46.099 | 1.00 | 34.54 | N |
| ATOM | 4809 | N   | ASN C 190 | -8.879  | 40.550 | -46.658 | 1.00 | 25.57 | N |
| ATOM | 4810 | CA  | ASN C 190 | -9.099  | 41.982 | -46.412 | 1.00 | 25.26 | C |
| ATOM | 4811 | C   | ASN C 190 | -9.724  | 42.434 | -45.093 | 1.00 | 23.89 | C |
| ATOM | 4812 | O   | ASN C 190 | -9.659  | 43.613 | -44.780 | 1.00 | 22.33 | O |
| ATOM | 4813 | CB  | ASN C 190 | -9.756  | 42.668 | -47.622 | 1.00 | 28.05 | C |
| ATOM | 4814 | CG  | ASN C 190 | -11.157 | 42.205 | -47.888 | 1.00 | 30.19 | C |
| ATOM | 4815 | OD1 | ASN C 190 | -11.806 | 42.678 | -48.845 | 1.00 | 33.70 | O |
| ATOM | 4816 | ND2 | ASN C 190 | -11.648 | 41.283 | -47.070 | 1.00 | 31.72 | N |
| ATOM | 4817 | N   | SER C 191 | -10.273 | 41.527 | -44.297 | 1.00 | 21.86 | N |
| ATOM | 4818 | CA  | SER C 191 | -10.903 | 41.912 | -43.017 | 1.00 | 22.60 | C |
| ATOM | 4819 | C   | SER C 191 | -9.945  | 41.617 | -41.879 | 1.00 | 22.17 | C |
| ATOM | 4820 | O   | SER C 191 | -9.547  | 40.472 | -41.721 | 1.00 | 22.02 | O |
| ATOM | 4821 | CB  | SER C 191 | -12.183 | 41.103 | -42.767 | 1.00 | 23.43 | C |
| ATOM | 4822 | OG  | SER C 191 | -13.167 | 41.363 | -43.750 | 1.00 | 25.19 | O |
| ATOM | 4823 | N   | TYR C 192 | -9.551  | 42.626 | -41.105 | 1.00 | 21.07 | N |
| ATOM | 4824 | CA  | TYR C 192 | -8.622  | 42.381 | -39.990 | 1.00 | 21.45 | C |
| ATOM | 4825 | C   | TYR C 192 | -9.224  | 42.909 | -38.693 | 1.00 | 21.26 | C |
| ATOM | 4826 | O   | TYR C 192 | -9.652  | 44.050 | -38.627 | 1.00 | 21.67 | O |
| ATOM | 4827 | CB  | TYR C 192 | -7.245  | 43.001 | -40.272 | 1.00 | 21.98 | C |
| ATOM | 4828 | CG  | TYR C 192 | -6.532  | 42.298 | -41.410 | 1.00 | 21.23 | C |
| ATOM | 4829 | CD1 | TYR C 192 | -5.654  | 41.252 | -41.161 | 1.00 | 21.66 | C |
| ATOM | 4830 | CD2 | TYR C 192 | -6.763  | 42.664 | -42.735 | 1.00 | 21.44 | C |
| ATOM | 4831 | CE1 | TYR C 192 | -5.012  | 40.606 | -42.189 | 1.00 | 20.84 | C |
| ATOM | 4832 | CE2 | TYR C 192 | -6.134  | 42.016 | -43.771 | 1.00 | 21.25 | C |
| ATOM | 4833 | CZ  | TYR C 192 | -5.266  | 40.985 | -43.506 | 1.00 | 20.49 | C |
| ATOM | 4834 | OH  | TYR C 192 | -4.631  | 40.345 | -44.556 | 1.00 | 21.86 | O |
| ATOM | 4835 | N   | THR C 193 | -9.221  | 42.067 | -37.663 | 1.00 | 22.67 | N |
| ATOM | 4836 | CA  | THR C 193 | -9.962  | 42.349 | -36.436 | 1.00 | 23.67 | C |
| ATOM | 4837 | C   | THR C 193 | -9.142  | 41.999 | -35.201 | 1.00 | 22.66 | C |
| ATOM | 4838 | O   | THR C 193 | -8.551  | 40.941 | -35.174 | 1.00 | 21.32 | O |
| ATOM | 4839 | CB  | THR C 193 | -11.242 | 41.483 | -36.399 | 1.00 | 24.71 | C |
| ATOM | 4840 | OG1 | THR C 193 | -12.045 | 41.772 | -37.544 | 1.00 | 29.86 | O |
| ATOM | 4841 | CG2 | THR C 193 | -12.052 | 41.744 | -35.135 | 1.00 | 26.61 | C |
| ATOM | 4842 | N   | CYS C 194 | -9.113  | 42.884 | -34.201 | 1.00 | 22.98 | N |
| ATOM | 4843 | CA  | CYS C 194 | -8.609  | 42.505 | -32.865 | 1.00 | 24.31 | C |
| ATOM | 4844 | C   | CYS C 194 | -9.740  | 42.603 | -31.842 | 1.00 | 24.20 | C |
| ATOM | 4845 | O   | CYS C 194 | -10.518 | 43.554 | -31.859 | 1.00 | 23.43 | O |
| ATOM | 4846 | CB  | CYS C 194 | -7.372  | 43.312 | -32.418 | 1.00 | 25.02 | C |
| ATOM | 4847 | SG  | CYS C 194 | -7.518  | 45.131 | -32.185 | 1.00 | 27.92 | S |
| ATOM | 4848 | N   | GLU C 195 | -9.818  | 41.608 | -30.962 | 1.00 | 26.82 | N |
| ATOM | 4849 | CA  | GLU C 195 | -10.879 | 41.544 | -29.945 | 1.00 | 29.08 | C |
| ATOM | 4850 | C   | GLU C 195 | -10.263 | 41.436 | -28.564 | 1.00 | 28.56 | C |
| ATOM | 4851 | O   | GLU C 195 | -9.539  | 40.482 | -28.294 | 1.00 | 29.32 | O |
| ATOM | 4852 | CB  | GLU C 195 | -11.756 | 40.325 | -30.161 | 1.00 | 29.68 | C |
| ATOM | 4853 | CG  | GLU C 195 | -12.334 | 40.193 | -31.558 | 1.00 | 31.98 | C |
| ATOM | 4854 | CD  | GLU C 195 | -13.389 | 39.118 | -31.637 | 1.00 | 32.57 | C |
| ATOM | 4855 | OE1 | GLU C 195 | -13.644 | 38.456 | -30.602 | 1.00 | 35.76 | O |
| ATOM | 4856 | OE2 | GLU C 195 | -13.967 | 38.945 | -32.733 | 1.00 | 36.16 | O |
| ATOM | 4857 | N   | ALA C 196 | -10.548 | 42.419 | -27.719 | 1.00 | 29.17 | N |
| ATOM | 4858 | CA  | ALA C 196 | -10.022 | 42.484 | -26.359 | 1.00 | 30.52 | C |
| ATOM | 4859 | C   | ALA C 196 | -11.066 | 41.947 | -25.379 | 1.00 | 32.13 | C |
| ATOM | 4860 | O   | ALA C 196 | -12.166 | 42.485 | -25.312 | 1.00 | 31.33 | O |
| ATOM | 4861 | CB  | ALA C 196 | -9.680  | 43.922 | -26.009 | 1.00 | 30.37 | C |
| ATOM | 4862 | N   | THR C 197 | -10.717 | 40.892 | -24.638 | 1.00 | 33.95 | N |
| ATOM | 4863 | CA  | THR C 197 | -11.590 | 40.352 | -23.580 | 1.00 | 35.17 | C |

```
ATOM   4864  C    THR C 197    -10.979  40.640 -22.191  1.00 35.45           C
ATOM   4865  O    THR C 197     -9.888  40.154 -21.879  1.00 34.30           O
ATOM   4866  CB   THR C 197    -11.824  38.843 -23.777  1.00 35.35           C
ATOM   4867  OG1  THR C 197    -12.606  38.631 -24.958  1.00 36.49           O
ATOM   4868  CG2  THR C 197    -12.560  38.226 -22.591  1.00 36.28           C
ATOM   4869  N    HIS C 198    -11.705  41.423 -21.387  1.00 35.92           N
ATOM   4870  CA   HIS C 198    -11.273  41.911 -20.076  1.00 36.68           C
ATOM   4871  C    HIS C 198    -12.393  41.621 -19.061  1.00 38.44           C
ATOM   4872  O    HIS C 198    -13.545  41.416 -19.449  1.00 38.40           O
ATOM   4873  CB   HIS C 198    -11.008  43.414 -20.173  1.00 35.68           C
ATOM   4874  CG   HIS C 198    -10.292  44.002 -18.995  1.00 33.99           C
ATOM   4875  ND1  HIS C 198    -10.849  44.985 -18.212  1.00 33.04           N
ATOM   4876  CD2  HIS C 198     -9.056  43.777 -18.488  1.00 33.53           C
ATOM   4877  CE1  HIS C 198    -10.000  45.330 -17.262  1.00 32.92           C
ATOM   4878  NE2  HIS C 198     -8.903  44.612 -17.407  1.00 31.79           N
ATOM   4879  N    LYS C 199    -12.070  41.627 -17.765  1.00 40.35           N
ATOM   4880  CA   LYS C 199    -13.076  41.337 -16.715  1.00 40.72           C
ATOM   4881  C    LYS C 199    -14.179  42.386 -16.625  1.00 41.35           C
ATOM   4882  O    LYS C 199    -15.262  42.095 -16.120  1.00 42.08           O
ATOM   4883  CB   LYS C 199    -12.420  41.164 -15.337  1.00 41.35           C
ATOM   4884  CG   LYS C 199    -12.239  42.435 -14.528  1.00 41.86           C
ATOM   4885  CD   LYS C 199    -11.667  42.112 -13.145  1.00 42.38           C
ATOM   4886  CE   LYS C 199    -12.728  41.618 -12.176  1.00 43.43           C
ATOM   4887  NZ   LYS C 199    -12.107  41.076 -10.937  1.00 42.97           N
ATOM   4888  N    THR C 200    -13.902  43.596 -17.101  1.00 41.45           N
ATOM   4889  CA   THR C 200    -14.900  44.655 -17.167  1.00 41.60           C
ATOM   4890  C    THR C 200    -16.095  44.358 -18.109  1.00 42.01           C
ATOM   4891  O    THR C 200    -17.017  45.169 -18.183  1.00 43.03           O
ATOM   4892  CB   THR C 200    -14.259  46.009 -17.548  1.00 41.86           C
ATOM   4893  OG1  THR C 200    -13.383  45.832 -18.670  1.00 41.91           O
ATOM   4894  CG2  THR C 200    -13.469  46.589 -16.370  1.00 41.31           C
ATOM   4895  N    SER C 201    -16.086  43.223 -18.817  1.00 41.90           N
ATOM   4896  CA   SER C 201    -17.280  42.754 -19.540  1.00 42.05           C
ATOM   4897  C    SER C 201    -17.194  41.321 -20.057  1.00 42.39           C
ATOM   4898  O    SER C 201    -16.117  40.784 -20.317  1.00 42.34           O
ATOM   4899  CB   SER C 201    -17.640  43.677 -20.713  1.00 42.35           C
ATOM   4900  OG   SER C 201    -18.612  43.070 -21.565  1.00 42.28           O
ATOM   4901  N    THR C 202    -18.371  40.719 -20.200  1.00 42.63           N
ATOM   4902  CA   THR C 202    -18.541  39.444 -20.887  1.00 43.31           C
ATOM   4903  C    THR C 202    -18.317  39.560 -22.401  1.00 42.97           C
ATOM   4904  O    THR C 202    -17.946  38.579 -23.043  1.00 42.80           O
ATOM   4905  CB   THR C 202    -19.975  38.862 -20.661  1.00 43.75           C
ATOM   4906  OG1  THR C 202    -20.190  37.754 -21.545  1.00 45.17           O
ATOM   4907  CG2  THR C 202    -21.078  39.922 -20.917  1.00 44.07           C
ATOM   4908  N    SER C 203    -18.579  40.748 -22.954  1.00 42.59           N
ATOM   4909  CA   SER C 203    -18.461  41.002 -24.392  1.00 42.42           C
ATOM   4910  C    SER C 203    -17.143  41.704 -24.716  1.00 40.93           C
ATOM   4911  O    SER C 203    -16.808  42.703 -24.086  1.00 41.37           O
ATOM   4912  CB   SER C 203    -19.606  41.890 -24.869  1.00 42.86           C
ATOM   4913  OG   SER C 203    -20.847  41.207 -24.800  1.00 45.10           O
ATOM   4914  N    PRO C 204    -16.398  41.191 -25.705  1.00 39.83           N
ATOM   4915  CA   PRO C 204    -15.127  41.844 -26.072  1.00 38.27           C
ATOM   4916  C    PRO C 204    -15.259  43.244 -26.688  1.00 37.42           C
ATOM   4917  O    PRO C 204    -16.295  43.599 -27.243  1.00 36.70           O
ATOM   4918  CB   PRO C 204    -14.497  40.878 -27.084  1.00 38.43           C
ATOM   4919  CG   PRO C 204    -15.592  39.981 -27.553  1.00 39.68           C
ATOM   4920  CD   PRO C 204    -16.667  39.976 -26.501  1.00 39.35           C
ATOM   4921  N    ILE C 205    -14.200  44.033 -26.569  1.00 35.69           N
ATOM   4922  CA   ILE C 205    -14.111  45.292 -27.273  1.00 34.47           C
ATOM   4923  C    ILE C 205    -13.417  44.946 -28.575  1.00 33.60           C
ATOM   4924  O    ILE C 205    -12.321  44.381 -28.561  1.00 32.70           O
ATOM   4925  CB   ILE C 205    -13.305  46.340 -26.495  1.00 35.09           C
ATOM   4926  CG1  ILE C 205    -13.938  46.578 -25.121  1.00 35.89           C
ATOM   4927  CG2  ILE C 205    -13.265  47.646 -27.257  1.00 34.93           C
ATOM   4928  CD1  ILE C 205    -12.963  47.013 -24.089  1.00 36.80           C
```

```
ATOM   4929  N    VAL C 206    -14.075  45.271 -29.689  1.00 32.62        N
ATOM   4930  CA   VAL C 206    -13.652  44.844 -31.020  1.00 32.03        C
ATOM   4931  C    VAL C 206    -13.326  46.065 -31.874  1.00 30.15        C
ATOM   4932  O    VAL C 206    -14.073  47.040 -31.883  1.00 31.66        O
ATOM   4933  CB   VAL C 206    -14.755  43.978 -31.707  1.00 32.46        C
ATOM   4934  CG1  VAL C 206    -14.344  43.576 -33.103  1.00 32.58        C
ATOM   4935  CG2  VAL C 206    -15.050  42.731 -30.877  1.00 32.93        C
ATOM   4936  N    LYS C 207    -12.195  46.007 -32.578  1.00 27.84        N
ATOM   4937  CA   LYS C 207    -11.820  47.012 -33.562  1.00 26.66        C
ATOM   4938  C    LYS C 207    -11.426  46.306 -34.845  1.00 24.48        C
ATOM   4939  O    LYS C 207    -10.875  45.212 -34.819  1.00 22.72        O
ATOM   4940  CB   LYS C 207    -10.675  47.867 -33.056  1.00 27.72        C
ATOM   4941  CG   LYS C 207    -11.048  48.718 -31.855  1.00 29.08        C
ATOM   4942  CD   LYS C 207    -11.915  49.922 -32.238  1.00 30.28        C
ATOM   4943  CE   LYS C 207    -12.712  50.427 -31.031  1.00 30.59        C
ATOM   4944  NZ   LYS C 207    -13.231  51.809 -31.284  1.00 32.06        N
ATOM   4945  N    SER C 208    -11.730  46.927 -35.976  1.00 23.54        N
ATOM   4946  CA   SER C 208    -11.592  46.253 -37.264  1.00 24.07        C
ATOM   4947  C    SER C 208    -11.288  47.246 -38.362  1.00 22.61        C
ATOM   4948  O    SER C 208    -11.647  48.409 -38.262  1.00 23.19        O
ATOM   4949  CB   SER C 208    -12.907  45.552 -37.637  1.00 25.62        C
ATOM   4950  OG   SER C 208    -13.457  44.869 -36.530  1.00 30.00        O
ATOM   4951  N    PHE C 209    -10.652  46.768 -39.418  1.00 23.26        N
ATOM   4952  CA   PHE C 209    -10.571  47.519 -40.668  1.00 23.42        C
ATOM   4953  C    PHE C 209    -10.564  46.566 -41.846  1.00 22.71        C
ATOM   4954  O    PHE C 209    -10.297  45.368 -41.701  1.00 22.93        O
ATOM   4955  CB   PHE C 209     -9.327  48.410 -40.710  1.00 23.56        C
ATOM   4956  CG   PHE C 209     -8.038  47.650 -40.938  1.00 23.16        C
ATOM   4957  CD1  PHE C 209     -7.547  47.437 -42.215  1.00 23.66        C
ATOM   4958  CD2  PHE C 209     -7.315  47.162 -39.864  1.00 24.84        C
ATOM   4959  CE1  PHE C 209     -6.370  46.722 -42.419  1.00 22.66        C
ATOM   4960  CE2  PHE C 209     -6.121  46.464 -40.060  1.00 23.95        C
ATOM   4961  CZ   PHE C 209     -5.651  46.237 -41.325  1.00 23.45        C
ATOM   4962  N    ASN C 210    -10.854  47.118 -43.020  1.00 22.37        N
ATOM   4963  CA   ASN C 210    -10.839  46.353 -44.236  1.00 21.77        C
ATOM   4964  C    ASN C 210     -9.726  46.923 -45.105  1.00 21.87        C
ATOM   4965  O    ASN C 210     -9.653  48.133 -45.303  1.00 21.63        O
ATOM   4966  CB   ASN C 210    -12.185  46.465 -44.939  1.00 21.22        C
ATOM   4967  CG   ASN C 210    -12.310  45.513 -46.120  1.00 22.81        C
ATOM   4968  OD1  ASN C 210    -11.617  45.664 -47.150  1.00 23.03        O
ATOM   4969  ND2  ASN C 210    -13.206  44.545 -45.997  1.00 21.63        N
ATOM   4970  N    ARG C 211     -8.883  46.047 -45.622  1.00 22.51        N
ATOM   4971  CA   ARG C 211     -7.737  46.445 -46.439  1.00 23.51        C
ATOM   4972  C    ARG C 211     -8.121  47.250 -47.674  1.00 24.68        C
ATOM   4973  O    ARG C 211     -7.330  48.100 -48.118  1.00 23.51        O
ATOM   4974  CB   ARG C 211     -6.953  45.200 -46.861  1.00 24.29        C
ATOM   4975  CG   ARG C 211     -5.648  45.479 -47.572  1.00 25.11        C
ATOM   4976  CD   ARG C 211     -4.927  44.196 -47.901  1.00 25.27        C
ATOM   4977  NE   ARG C 211     -5.764  43.207 -48.583  1.00 24.59        N
ATOM   4978  CZ   ARG C 211     -5.965  43.140 -49.893  1.00 27.95        C
ATOM   4979  NH1  ARG C 211     -5.416  44.021 -50.708  1.00 29.18        N
ATOM   4980  NH2  ARG C 211     -6.742  42.178 -50.402  1.00 29.02        N
ATOM   4981  N    ASN C 212     -9.315  47.005 -48.223  1.00 25.95        N
ATOM   4982  CA   ASN C 212     -9.759  47.659 -49.459  1.00 28.53        C
ATOM   4983  C    ASN C 212    -10.589  48.921 -49.246  1.00 29.64        C
ATOM   4984  O    ASN C 212    -11.039  49.524 -50.221  1.00 31.57        O
ATOM   4985  CB   ASN C 212    -10.582  46.696 -50.315  1.00 28.78        C
ATOM   4986  CG   ASN C 212     -9.788  45.500 -50.807  1.00 30.85        C
ATOM   4987  OD1  ASN C 212     -8.852  45.635 -51.595  1.00 33.00        O
ATOM   4988  ND2  ASN C 212    -10.191  44.309 -50.374  1.00 32.44        N
ATOM   4989  N    GLU C 213    -10.807  49.320 -47.995  1.00 30.48        N
ATOM   4990  CA   GLU C 213    -11.663  50.460 -47.683  1.00 31.43        C
ATOM   4991  C    GLU C 213    -10.872  51.765 -47.581  1.00 34.46        C
ATOM   4992  O    GLU C 213     -9.820  51.809 -46.920  1.00 36.52        O
ATOM   4993  CB   GLU C 213    -12.426  50.202 -46.381  1.00 30.24        C
```

| ATOM | 4994 | CG  | GLU C | 213 | -13.407 | 51.304 | -46.035 | 1.00 | 29.45 | C |
|------|------|-----|-------|-----|---------|--------|---------|------|-------|---|
| ATOM | 4995 | CD  | GLU C | 213 | -14.450 | 50.862 | -45.045 | 1.00 | 30.19 | C |
| ATOM | 4996 | OE1 | GLU C | 213 | -14.232 | 49.844 | -44.350 | 1.00 | 30.59 | O |
| ATOM | 4997 | OE2 | GLU C | 213 | -15.498 | 51.524 | -44.976 | 1.00 | 28.24 | O |
| TER  | 4998 |     | GLU C | 213 |         |        |         |      |       |   |
| ATOM | 4999 | N   | GLU D | 1   | 24.478  | 69.726 | -8.032  | 1.00 | 33.06 | N |
| ATOM | 5000 | CA  | GLU D | 1   | 23.875  | 68.395 | -8.319  | 1.00 | 32.02 | C |
| ATOM | 5001 | C   | GLU D | 1   | 24.979  | 67.406 | -8.699  | 1.00 | 27.76 | C |
| ATOM | 5002 | O   | GLU D | 1   | 25.808  | 67.675 | -9.578  | 1.00 | 28.04 | O |
| ATOM | 5003 | CB  | GLU D | 1   | 22.844  | 68.533 | -9.441  | 1.00 | 32.51 | C |
| ATOM | 5004 | CG  | GLU D | 1   | 22.306  | 67.243 | -10.015 | 1.00 | 34.48 | C |
| ATOM | 5005 | CD  | GLU D | 1   | 21.500  | 67.464 | -11.286 | 1.00 | 36.46 | C |
| ATOM | 5006 | OE1 | GLU D | 1   | 21.578  | 68.577 | -11.869 | 1.00 | 40.13 | O |
| ATOM | 5007 | OE2 | GLU D | 1   | 20.792  | 66.520 | -11.715 | 1.00 | 41.74 | O |
| ATOM | 5008 | N   | VAL D | 2   | 25.001  | 66.268 | -8.017  | 1.00 | 24.11 | N |
| ATOM | 5009 | CA  | VAL D | 2   | 25.901  | 65.194 | -8.400  | 1.00 | 20.71 | C |
| ATOM | 5010 | C   | VAL D | 2   | 25.370  | 64.643 | -9.719  | 1.00 | 19.95 | C |
| ATOM | 5011 | O   | VAL D | 2   | 24.167  | 64.437 | -9.882  | 1.00 | 19.24 | O |
| ATOM | 5012 | CB  | VAL D | 2   | 25.992  | 64.094 | -7.313  | 1.00 | 19.52 | C |
| ATOM | 5013 | CG1 | VAL D | 2   | 26.869  | 62.969 | -7.776  | 1.00 | 18.71 | C |
| ATOM | 5014 | CG2 | VAL D | 2   | 26.498  | 64.691 | -5.983  | 1.00 | 17.64 | C |
| ATOM | 5015 | N   | LYS D | 3   | 26.257  | 64.435 | -10.677 | 1.00 | 19.34 | N |
| ATOM | 5016 | CA  | LYS D | 3   | 25.869  | 63.864 | -11.952 | 1.00 | 20.36 | C |
| ATOM | 5017 | C   | LYS D | 3   | 26.841  | 62.715 | -12.247 | 1.00 | 17.91 | C |
| ATOM | 5018 | O   | LYS D | 3   | 28.035  | 62.866 | -12.139 | 1.00 | 17.42 | O |
| ATOM | 5019 | CB  | LYS D | 3   | 25.859  | 64.989 | -12.993 | 1.00 | 21.71 | C |
| ATOM | 5020 | CG  | LYS D | 3   | 25.706  | 64.573 | -14.425 | 1.00 | 25.52 | C |
| ATOM | 5021 | CD  | LYS D | 3   | 25.627  | 65.798 | -15.328 | 1.00 | 26.10 | C |
| ATOM | 5022 | CE  | LYS D | 3   | 25.693  | 65.371 | -16.790 | 1.00 | 30.04 | C |
| ATOM | 5023 | NZ  | LYS D | 3   | 26.103  | 66.498 | -17.697 | 1.00 | 32.35 | N |
| ATOM | 5024 | N   | VAL D | 4   | 26.304  | 61.531 | -12.534 | 1.00 | 16.86 | N |
| ATOM | 5025 | CA  | VAL D | 4   | 27.128  | 60.356 | -12.830 | 1.00 | 15.43 | C |
| ATOM | 5026 | C   | VAL D | 4   | 26.699  | 59.832 | -14.178 | 1.00 | 14.88 | C |
| ATOM | 5027 | O   | VAL D | 4   | 25.538  | 59.528 | -14.335 | 1.00 | 13.65 | O |
| ATOM | 5028 | CB  | VAL D | 4   | 26.888  | 59.269 | -11.760 | 1.00 | 15.60 | C |
| ATOM | 5029 | CG1 | VAL D | 4   | 27.736  | 58.038 | -12.024 | 1.00 | 15.20 | C |
| ATOM | 5030 | CG2 | VAL D | 4   | 27.129  | 59.862 | -10.399 | 1.00 | 17.09 | C |
| ATOM | 5031 | N   | GLU D | 5   | 27.627  | 59.742 | -15.139 | 1.00 | 16.41 | N |
| ATOM | 5032 | CA  | GLU D | 5   | 27.267  | 59.521 | -16.530 | 1.00 | 18.79 | C |
| ATOM | 5033 | C   | GLU D | 5   | 28.152  | 58.477 | -17.192 | 1.00 | 16.20 | C |
| ATOM | 5034 | O   | GLU D | 5   | 29.298  | 58.743 | -17.491 | 1.00 | 13.53 | O |
| ATOM | 5035 | CB  | GLU D | 5   | 27.363  | 60.839 | -17.310 | 1.00 | 20.07 | C |
| ATOM | 5036 | CG  | GLU D | 5   | 27.067  | 60.701 | -18.795 | 1.00 | 24.95 | C |
| ATOM | 5037 | CD  | GLU D | 5   | 27.023  | 62.060 | -19.536 | 1.00 | 26.36 | C |
| ATOM | 5038 | OE1 | GLU D | 5   | 27.179  | 63.132 | -18.894 | 1.00 | 32.56 | O |
| ATOM | 5039 | OE2 | GLU D | 5   | 26.841  | 62.025 | -20.778 | 1.00 | 33.56 | O |
| ATOM | 5040 | N   | GLU D | 6   | 27.585  | 57.299 | -17.456 | 1.00 | 16.29 | N |
| ATOM | 5041 | CA  | GLU D | 6   | 28.353  | 56.203 | -18.050 | 1.00 | 14.44 | C |
| ATOM | 5042 | C   | GLU D | 6   | 28.394  | 56.256 | -19.569 | 1.00 | 15.90 | C |
| ATOM | 5043 | O   | GLU D | 6   | 27.476  | 56.751 | -20.224 | 1.00 | 15.69 | O |
| ATOM | 5044 | CB  | GLU D | 6   | 27.733  | 54.846 | -17.651 | 1.00 | 13.41 | C |
| ATOM | 5045 | CG  | GLU D | 6   | 27.698  | 54.604 | -16.158 | 1.00 | 16.16 | C |
| ATOM | 5046 | CD  | GLU D | 6   | 26.454  | 55.139 | -15.464 | 1.00 | 16.30 | C |
| ATOM | 5047 | OE1 | GLU D | 6   | 25.774  | 56.069 | -15.977 | 1.00 | 15.92 | O |
| ATOM | 5048 | OE2 | GLU D | 6   | 26.148  | 54.641 | -14.374 | 1.00 | 16.45 | O |
| ATOM | 5049 | N   | SER D | 7   | 29.432  | 55.656 | -20.149 | 1.00 | 15.86 | N |
| ATOM | 5050 | CA  | SER D | 7   | 29.461  | 55.427 | -21.576 | 1.00 | 16.40 | C |
| ATOM | 5051 | C   | SER D | 7   | 30.312  | 54.205 | -21.909 | 1.00 | 15.00 | C |
| ATOM | 5052 | O   | SER D | 7   | 31.004  | 53.660 | -21.040 | 1.00 | 14.47 | O |
| ATOM | 5053 | CB  | SER D | 7   | 30.031  | 56.649 | -22.297 | 1.00 | 18.14 | C |
| ATOM | 5054 | OG  | SER D | 7   | 31.352  | 56.875 | -21.888 | 1.00 | 18.71 | O |
| ATOM | 5055 | N   | GLY D | 8   | 30.271  | 53.819 | -23.178 | 1.00 | 15.17 | N |
| ATOM | 5056 | CA  | GLY D | 8   | 31.121  | 52.730 | -23.689 | 1.00 | 15.16 | C |
| ATOM | 5057 | C   | GLY D | 8   | 30.433  | 51.400 | -23.943 | 1.00 | 15.43 | C |
| ATOM | 5058 | O   | GLY D | 8   | 31.081  | 50.420 | -24.318 | 1.00 | 18.49 | O |

| ATOM | 5059 | N | GLY D | 9 | 29.130 | 51.348 | -23.722 | 1.00 | 15.95 | N |
| ATOM | 5060 | CA | GLY D | 9 | 28.367 | 50.124 | -23.942 | 1.00 | 14.66 | C |
| ATOM | 5061 | C | GLY D | 9 | 28.263 | 49.761 | -25.406 | 1.00 | 14.92 | C |
| ATOM | 5062 | O | GLY D | 9 | 28.743 | 50.476 | -26.286 | 1.00 | 14.75 | O |
| ATOM | 5063 | N | GLY D | 10 | 27.698 | 48.595 | -25.669 | 1.00 | 12.40 | N |
| ATOM | 5064 | CA | GLY D | 10 | 27.583 | 48.103 | -27.006 | 1.00 | 11.64 | C |
| ATOM | 5065 | C | GLY D | 10 | 27.497 | 46.590 | -26.970 | 1.00 | 11.35 | C |
| ATOM | 5066 | O | GLY D | 10 | 27.058 | 46.017 | -25.962 | 1.00 | 10.63 | O |
| ATOM | 5067 | N | LEU D | 11 | 27.869 | 45.979 | -28.087 | 1.00 | 11.41 | N |
| ATOM | 5068 | CA | LEU D | 11 | 27.824 | 44.503 | -28.259 | 1.00 | 10.66 | C |
| ATOM | 5069 | C | LEU D | 11 | 29.220 | 43.934 | -28.552 | 1.00 | 10.82 | C |
| ATOM | 5070 | O | LEU D | 11 | 30.008 | 44.530 | -29.282 | 1.00 | 9.99 | O |
| ATOM | 5071 | CB | LEU D | 11 | 26.863 | 44.136 | -29.385 | 1.00 | 10.15 | C |
| ATOM | 5072 | CG | LEU D | 11 | 26.739 | 42.605 | -29.610 | 1.00 | 11.40 | C |
| ATOM | 5073 | CD1 | LEU D | 11 | 25.377 | 42.264 | -30.093 | 1.00 | 12.36 | C |
| ATOM | 5074 | CD2 | LEU D | 11 | 27.761 | 42.149 | -30.627 | 1.00 | 11.21 | C |
| ATOM | 5075 | N | VAL D | 12 | 29.526 | 42.767 | -27.988 | 1.00 | 9.56 | N |
| ATOM | 5076 | CA | VAL D | 12 | 30.710 | 42.064 | -28.360 | 1.00 | 10.97 | C |
| ATOM | 5077 | C | VAL D | 12 | 30.390 | 40.567 | -28.254 | 1.00 | 10.19 | C |
| ATOM | 5078 | O | VAL D | 12 | 29.429 | 40.193 | -27.587 | 1.00 | 9.78 | O |
| ATOM | 5079 | CB | VAL D | 12 | 31.913 | 42.459 | -27.460 | 1.00 | 11.04 | C |
| ATOM | 5080 | CG1 | VAL D | 12 | 31.792 | 41.889 | -26.015 | 1.00 | 13.00 | C |
| ATOM | 5081 | CG2 | VAL D | 12 | 33.260 | 42.099 | -28.130 | 1.00 | 13.32 | C |
| ATOM | 5082 | N | GLN D | 13 | 31.209 | 39.755 | -28.906 | 1.00 | 11.62 | N |
| ATOM | 5083 | CA | GLN D | 13 | 31.023 | 38.323 | -28.943 | 1.00 | 11.14 | C |
| ATOM | 5084 | C | GLN D | 13 | 31.553 | 37.680 | -27.668 | 1.00 | 11.34 | C |
| ATOM | 5085 | O | GLN D | 13 | 32.466 | 38.192 | -27.018 | 1.00 | 10.73 | O |
| ATOM | 5086 | CB | GLN D | 13 | 31.758 | 37.728 | -30.146 | 1.00 | 11.42 | C |
| ATOM | 5087 | CG | GLN D | 13 | 31.292 | 38.225 | -31.521 | 1.00 | 13.04 | C |
| ATOM | 5088 | CD | GLN D | 13 | 31.636 | 39.703 | -31.815 | 1.00 | 11.71 | C |
| ATOM | 5089 | OE1 | GLN D | 13 | 32.647 | 40.241 | -31.375 | 1.00 | 11.83 | O |
| ATOM | 5090 | NE2 | GLN D | 13 | 30.769 | 40.333 | -32.572 | 1.00 | 12.76 | N |
| ATOM | 5091 | N | PRO D | 14 | 31.038 | 36.484 | -27.336 | 1.00 | 11.53 | N |
| ATOM | 5092 | CA | PRO D | 14 | 31.632 | 35.785 | -26.231 | 1.00 | 12.04 | C |
| ATOM | 5093 | C | PRO D | 14 | 33.142 | 35.564 | -26.447 | 1.00 | 10.68 | C |
| ATOM | 5094 | O | PRO D | 14 | 33.594 | 35.247 | -27.556 | 1.00 | 10.96 | O |
| ATOM | 5095 | CB | PRO D | 14 | 30.871 | 34.458 | -26.210 | 1.00 | 11.78 | C |
| ATOM | 5096 | CG | PRO D | 14 | 29.565 | 34.754 | -26.888 | 1.00 | 11.53 | C |
| ATOM | 5097 | CD | PRO D | 14 | 29.912 | 35.758 | -27.938 | 1.00 | 12.07 | C |
| ATOM | 5098 | N | GLY D | 15 | 33.885 | 35.744 | -25.376 | 1.00 | 9.46 | N |
| ATOM | 5099 | CA | GLY D | 15 | 35.315 | 35.704 | -25.350 | 1.00 | 9.60 | C |
| ATOM | 5100 | C | GLY D | 15 | 35.966 | 37.015 | -25.693 | 1.00 | 11.04 | C |
| ATOM | 5101 | O | GLY D | 15 | 37.166 | 37.132 | -25.614 | 1.00 | 10.25 | O |
| ATOM | 5102 | N | GLY D | 16 | 35.153 | 38.004 | -26.061 | 1.00 | 10.24 | N |
| ATOM | 5103 | CA | GLY D | 16 | 35.644 | 39.309 | -26.429 | 1.00 | 10.72 | C |
| ATOM | 5104 | C | GLY D | 16 | 35.752 | 40.233 | -25.235 | 1.00 | 11.12 | C |
| ATOM | 5105 | O | GLY D | 16 | 35.483 | 39.848 | -24.090 | 1.00 | 10.48 | O |
| ATOM | 5106 | N | SER D | 17 | 36.071 | 41.495 | -25.547 | 1.00 | 12.10 | N |
| ATOM | 5107 | CA | SER D | 17 | 36.352 | 42.553 | -24.560 | 1.00 | 12.95 | C |
| ATOM | 5108 | C | SER D | 17 | 35.556 | 43.844 | -24.777 | 1.00 | 12.91 | C |
| ATOM | 5109 | O | SER D | 17 | 35.103 | 44.156 | -25.887 | 1.00 | 10.39 | O |
| ATOM | 5110 | CB | SER D | 17 | 37.834 | 42.933 | -24.621 | 1.00 | 12.29 | C |
| ATOM | 5111 | OG | SER D | 17 | 38.628 | 41.893 | -24.120 | 1.00 | 17.26 | O |
| ATOM | 5112 | N | MET D | 18 | 35.381 | 44.570 | -23.680 | 1.00 | 13.19 | N |
| ATOM | 5113 | CA | MET D | 18 | 34.691 | 45.886 | -23.699 | 1.00 | 14.56 | C |
| ATOM | 5114 | C | MET D | 18 | 35.393 | 46.755 | -22.669 | 1.00 | 13.69 | C |
| ATOM | 5115 | O | MET D | 18 | 35.990 | 46.256 | -21.748 | 1.00 | 13.72 | O |
| ATOM | 5116 | CB | MET D | 18 | 33.231 | 45.741 | -23.291 | 1.00 | 15.89 | C |
| ATOM | 5117 | CG | MET D | 18 | 32.374 | 44.891 | -24.206 | 1.00 | 19.92 | C |
| ATOM | 5118 | SD | MET D | 18 | 31.834 | 45.773 | -25.681 | 1.00 | 23.05 | S |
| ATOM | 5119 | CE | MET D | 18 | 30.777 | 46.956 | -24.847 | 1.00 | 22.21 | C |
| ATOM | 5120 | N | LYS D | 19 | 35.326 | 48.071 | -22.876 | 1.00 | 13.02 | N |
| ATOM | 5121 | CA | LYS D | 19 | 35.837 | 48.970 | -21.840 | 1.00 | 14.07 | C |
| ATOM | 5122 | C | LYS D | 19 | 34.794 | 50.060 | -21.653 | 1.00 | 13.16 | C |
| ATOM | 5123 | O | LYS D | 19 | 34.443 | 50.725 | -22.616 | 1.00 | 13.42 | O |

| ATOM | 5124 | CB | LYS | D | 19 | 37.179 | 49.600 | -22.211 | 1.00 | 15.46 | C |
|------|------|-----|-----|---|----|--------|--------|---------|------|-------|---|
| ATOM | 5125 | CG | LYS | D | 19 | 37.739 | 50.430 | -21.025 | 1.00 | 16.91 | C |
| ATOM | 5126 | CD | LYS | D | 19 | 39.108 | 50.932 | -21.255 | 1.00 | 19.35 | C |
| ATOM | 5127 | CE | LYS | D | 19 | 39.762 | 51.368 | -19.974 | 1.00 | 21.96 | C |
| ATOM | 5128 | NZ | LYS | D | 19 | 41.029 | 52.109 | -20.259 | 1.00 | 25.90 | N |
| ATOM | 5129 | N | ILE | D | 20 | 34.265 | 50.194 | -20.450 | 1.00 | 12.68 | N |
| ATOM | 5130 | CA | ILE | D | 20 | 33.235 | 51.193 | -20.172 | 1.00 | 12.16 | C |
| ATOM | 5131 | C | ILE | D | 20 | 33.775 | 52.195 | -19.167 | 1.00 | 10.75 | C |
| ATOM | 5132 | O | ILE | D | 20 | 34.751 | 51.946 | -18.469 | 1.00 | 11.35 | O |
| ATOM | 5133 | CB | ILE | D | 20 | 31.904 | 50.563 | -19.694 | 1.00 | 11.73 | C |
| ATOM | 5134 | CG1 | ILE | D | 20 | 32.052 | 49.877 | -18.340 | 1.00 | 12.55 | C |
| ATOM | 5135 | CG2 | ILE | D | 20 | 31.394 | 49.570 | -20.765 | 1.00 | 12.81 | C |
| ATOM | 5136 | CD1 | ILE | D | 20 | 30.794 | 49.149 | -17.798 | 1.00 | 12.55 | C |
| ATOM | 5137 | N | SER | D | 21 | 33.134 | 53.342 | -19.073 | 1.00 | 12.07 | N |
| ATOM | 5138 | CA | SER | D | 21 | 33.597 | 54.328 | -18.125 | 1.00 | 11.26 | C |
| ATOM | 5139 | C | SER | D | 21 | 32.464 | 55.208 | -17.637 | 1.00 | 10.56 | C |
| ATOM | 5140 | O | SER | D | 21 | 31.348 | 55.162 | -18.151 | 1.00 | 12.13 | O |
| ATOM | 5141 | CB | SER | D | 21 | 34.707 | 55.148 | -18.762 | 1.00 | 12.67 | C |
| ATOM | 5142 | OG | SER | D | 21 | 34.201 | 55.880 | -19.835 | 1.00 | 14.48 | O |
| ATOM | 5143 | N | CYS | D | 22 | 32.735 | 55.981 | -16.607 | 1.00 | 11.79 | N |
| ATOM | 5144 | CA | CYS | D | 22 | 31.792 | 57.007 | -16.207 | 1.00 | 13.80 | C |
| ATOM | 5145 | C | CYS | D | 22 | 32.535 | 58.198 | -15.673 | 1.00 | 12.98 | C |
| ATOM | 5146 | O | CYS | D | 22 | 33.615 | 58.059 | -15.098 | 1.00 | 12.24 | O |
| ATOM | 5147 | CB | CYS | D | 22 | 30.783 | 56.542 | -15.189 | 1.00 | 16.29 | C |
| ATOM | 5148 | SG | CYS | D | 22 | 31.391 | 56.178 | -13.636 | 1.00 | 23.32 | S |
| ATOM | 5149 | N | VAL | D | 23 | 31.943 | 59.358 | -15.947 | 1.00 | 15.63 | N |
| ATOM | 5150 | CA | VAL | D | 23 | 32.434 | 60.644 | -15.449 | 1.00 | 14.74 | C |
| ATOM | 5151 | C | VAL | D | 23 | 31.467 | 61.209 | -14.405 | 1.00 | 13.70 | C |
| ATOM | 5152 | O | VAL | D | 23 | 30.245 | 61.193 | -14.609 | 1.00 | 14.68 | O |
| ATOM | 5153 | CB | VAL | D | 23 | 32.702 | 61.653 | -16.605 | 1.00 | 15.78 | C |
| ATOM | 5154 | CG1 | VAL | D | 23 | 31.450 | 61.977 | -17.393 | 1.00 | 16.35 | C |
| ATOM | 5155 | CG2 | VAL | D | 23 | 33.322 | 62.933 | -16.030 | 1.00 | 19.07 | C |
| ATOM | 5156 | N | VAL | D | 24 | 32.036 | 61.777 | -13.329 | 1.00 | 13.16 | N |
| ATOM | 5157 | CA | VAL | D | 24 | 31.275 | 62.261 | -12.193 | 1.00 | 13.73 | C |
| ATOM | 5158 | C | VAL | D | 24 | 31.575 | 63.739 | -12.014 | 1.00 | 15.34 | C |
| ATOM | 5159 | O | VAL | D | 24 | 32.736 | 64.132 | -12.081 | 1.00 | 15.83 | O |
| ATOM | 5160 | CB | VAL | D | 24 | 31.676 | 61.531 | -10.942 | 1.00 | 13.68 | C |
| ATOM | 5161 | CG1 | VAL | D | 24 | 30.935 | 62.054 | -9.705 | 1.00 | 14.94 | C |
| ATOM | 5162 | CG2 | VAL | D | 24 | 31.417 | 60.004 | -11.142 | 1.00 | 13.86 | C |
| ATOM | 5163 | N | SER | D | 25 | 30.527 | 64.525 | -11.805 | 1.00 | 16.98 | N |
| ATOM | 5164 | CA | SER | D | 25 | 30.669 | 65.946 | -11.487 | 1.00 | 18.11 | C |
| ATOM | 5165 | C | SER | D | 25 | 29.765 | 66.237 | -10.298 | 1.00 | 17.96 | C |
| ATOM | 5166 | O | SER | D | 25 | 28.824 | 65.499 | -10.032 | 1.00 | 17.32 | O |
| ATOM | 5167 | CB | SER | D | 25 | 30.266 | 66.774 | -12.696 | 1.00 | 18.10 | C |
| ATOM | 5168 | OG | SER | D | 25 | 28.907 | 66.556 | -13.045 | 1.00 | 20.30 | O |
| ATOM | 5169 | N | GLY | D | 26 | 30.037 | 67.319 | -9.570 | 1.00 | 19.07 | N |
| ATOM | 5170 | CA | GLY | D | 26 | 29.180 | 67.694 | -8.447 | 1.00 | 18.20 | C |
| ATOM | 5171 | C | GLY | D | 26 | 29.799 | 67.368 | -7.099 | 1.00 | 19.18 | C |
| ATOM | 5172 | O | GLY | D | 26 | 29.300 | 67.803 | -6.073 | 1.00 | 20.02 | O |
| ATOM | 5173 | N | LEU | D | 27 | 30.875 | 66.584 | -7.094 | 1.00 | 17.69 | N |
| ATOM | 5174 | CA | LEU | D | 27 | 31.673 | 66.360 | -5.902 | 1.00 | 18.58 | C |
| ATOM | 5175 | C | LEU | D | 27 | 33.091 | 66.139 | -6.358 | 1.00 | 16.30 | C |
| ATOM | 5176 | O | LEU | D | 27 | 33.346 | 66.090 | -7.562 | 1.00 | 17.72 | O |
| ATOM | 5177 | CB | LEU | D | 27 | 31.170 | 65.174 | -5.076 | 1.00 | 20.16 | C |
| ATOM | 5178 | CG | LEU | D | 27 | 30.815 | 63.894 | -5.856 | 1.00 | 21.37 | C |
| ATOM | 5179 | CD1 | LEU | D | 27 | 32.061 | 63.223 | -6.418 | 1.00 | 22.65 | C |
| ATOM | 5180 | CD2 | LEU | D | 27 | 30.048 | 62.940 | -4.918 | 1.00 | 21.33 | C |
| ATOM | 5181 | N | THR | D | 28 | 34.015 | 66.050 | -5.410 | 1.00 | 16.09 | N |
| ATOM | 5182 | CA | THR | D | 28 | 35.412 | 65.805 | -5.718 | 1.00 | 14.63 | C |
| ATOM | 5183 | C | THR | D | 28 | 35.584 | 64.309 | -5.911 | 1.00 | 14.83 | C |
| ATOM | 5184 | O | THR | D | 28 | 35.665 | 63.553 | -4.950 | 1.00 | 14.07 | O |
| ATOM | 5185 | CB | THR | D | 28 | 36.342 | 66.307 | -4.622 | 1.00 | 15.40 | C |
| ATOM | 5186 | OG1 | THR | D | 28 | 36.071 | 67.702 | -4.389 | 1.00 | 17.71 | O |
| ATOM | 5187 | CG2 | THR | D | 28 | 37.775 | 66.146 | -5.044 | 1.00 | 14.54 | C |
| ATOM | 5188 | N | PHE | D | 29 | 35.646 | 63.913 | -7.173 | 1.00 | 14.85 | N |

| ATOM | 5189 | CA | PHE | D | 29 | 35.695 | 62.482 | -7.550 | 1.00 | 13.77 | C |
| ATOM | 5190 | C | PHE | D | 29 | 36.706 | 61.709 | -6.771 | 1.00 | 13.63 | C |
| ATOM | 5191 | O | PHE | D | 29 | 36.407 | 60.610 | -6.257 | 1.00 | 13.48 | O |
| ATOM | 5192 | CB | PHE | D | 29 | 35.986 | 62.389 | -9.050 | 1.00 | 13.46 | C |
| ATOM | 5193 | CG | PHE | D | 29 | 36.194 | 60.972 | -9.562 | 1.00 | 15.32 | C |
| ATOM | 5194 | CD1 | PHE | D | 29 | 35.121 | 60.207 | -10.003 | 1.00 | 15.06 | C |
| ATOM | 5195 | CD2 | PHE | D | 29 | 37.473 | 60.448 | -9.614 | 1.00 | 14.39 | C |
| ATOM | 5196 | CE1 | PHE | D | 29 | 35.331 | 58.919 | -10.499 | 1.00 | 15.23 | C |
| ATOM | 5197 | CE2 | PHE | D | 29 | 37.694 | 59.162 | -10.110 | 1.00 | 16.37 | C |
| ATOM | 5198 | CZ | PHE | D | 29 | 36.628 | 58.403 | -10.524 | 1.00 | 13.53 | C |
| ATOM | 5199 | N | SER | D | 30 | 37.914 | 62.258 | -6.637 | 1.00 | 13.37 | N |
| ATOM | 5200 | CA | SER | D | 30 | 39.000 | 61.568 | -5.939 | 1.00 | 14.72 | C |
| ATOM | 5201 | C | SER | D | 30 | 38.757 | 61.280 | -4.467 | 1.00 | 13.81 | C |
| ATOM | 5202 | O | SER | D | 30 | 39.478 | 60.494 | -3.869 | 1.00 | 15.78 | O |
| ATOM | 5203 | CB | SER | D | 30 | 40.289 | 62.370 | -6.062 | 1.00 | 16.75 | C |
| ATOM | 5204 | OG | SER | D | 30 | 40.126 | 63.591 | -5.373 | 1.00 | 17.26 | O |
| ATOM | 5205 | N | ASN | D | 31 | 37.757 | 61.923 | -3.874 | 1.00 | 13.34 | N |
| ATOM | 5206 | CA | ASN | D | 31 | 37.470 | 61.750 | -2.474 | 1.00 | 14.86 | C |
| ATOM | 5207 | C | ASN | D | 31 | 36.502 | 60.600 | -2.156 | 1.00 | 14.45 | C |
| ATOM | 5208 | O | ASN | D | 31 | 36.269 | 60.343 | -0.974 | 1.00 | 13.87 | O |
| ATOM | 5209 | CB | ASN | D | 31 | 36.858 | 63.043 | -1.881 | 1.00 | 15.59 | C |
| ATOM | 5210 | CG | ASN | D | 31 | 37.856 | 64.201 | -1.792 | 1.00 | 21.17 | C |
| ATOM | 5211 | OD1 | ASN | D | 31 | 37.593 | 65.195 | -1.086 | 1.00 | 23.75 | O |
| ATOM | 5212 | ND2 | ASN | D | 31 | 38.981 | 64.107 | -2.520 | 1.00 | 22.09 | N |
| ATOM | 5213 | N | TYR | D | 32 | 35.962 | 59.931 | -3.200 | 1.00 | 13.69 | N |
| ATOM | 5214 | CA | TYR | D | 32 | 34.814 | 59.011 | -3.077 | 1.00 | 14.11 | C |
| ATOM | 5215 | C | TYR | D | 32 | 35.164 | 57.633 | -3.612 | 1.00 | 11.79 | C |
| ATOM | 5216 | O | TYR | D | 32 | 35.806 | 57.517 | -4.644 | 1.00 | 12.70 | O |
| ATOM | 5217 | CB | TYR | D | 32 | 33.579 | 59.549 | -3.797 | 1.00 | 16.04 | C |
| ATOM | 5218 | CG | TYR | D | 32 | 33.109 | 60.739 | -3.000 | 1.00 | 18.08 | C |
| ATOM | 5219 | CD1 | TYR | D | 32 | 32.357 | 60.559 | -1.837 | 1.00 | 20.21 | C |
| ATOM | 5220 | CD2 | TYR | D | 32 | 33.575 | 61.999 | -3.296 | 1.00 | 18.56 | C |
| ATOM | 5221 | CE1 | TYR | D | 32 | 32.006 | 61.650 | -1.035 | 1.00 | 21.51 | C |
| ATOM | 5222 | CE2 | TYR | D | 32 | 33.216 | 63.096 | -2.525 | 1.00 | 20.08 | C |
| ATOM | 5223 | CZ | TYR | D | 32 | 32.443 | 62.927 | -1.398 | 1.00 | 20.73 | C |
| ATOM | 5224 | OH | TYR | D | 32 | 32.116 | 64.009 | -0.596 | 1.00 | 22.78 | O |
| ATOM | 5225 | N | TRP | D | 33 | 34.760 | 56.631 | -2.863 | 1.00 | 12.69 | N |
| ATOM | 5226 | CA | TRP | D | 33 | 34.813 | 55.243 | -3.326 | 1.00 | 12.06 | C |
| ATOM | 5227 | C | TRP | D | 33 | 33.915 | 55.095 | -4.560 | 1.00 | 11.78 | C |
| ATOM | 5228 | O | TRP | D | 33 | 32.866 | 55.742 | -4.684 | 1.00 | 11.68 | O |
| ATOM | 5229 | CB | TRP | D | 33 | 34.358 | 54.252 | -2.229 | 1.00 | 11.44 | C |
| ATOM | 5230 | CG | TRP | D | 33 | 35.108 | 54.270 | -0.920 | 1.00 | 11.49 | C |
| ATOM | 5231 | CD1 | TRP | D | 33 | 36.283 | 54.942 | -0.640 | 1.00 | 11.21 | C |
| ATOM | 5232 | CD2 | TRP | D | 33 | 34.764 | 53.574 | 0.257 | 1.00 | 9.42 | C |
| ATOM | 5233 | NE1 | TRP | D | 33 | 36.651 | 54.730 | 0.646 | 1.00 | 11.77 | N |
| ATOM | 5234 | CE2 | TRP | D | 33 | 35.750 | 53.871 | 1.226 | 1.00 | 11.29 | C |
| ATOM | 5235 | CE3 | TRP | D | 33 | 33.717 | 52.718 | 0.610 | 1.00 | 11.17 | C |
| ATOM | 5236 | CZ2 | TRP | D | 33 | 35.726 | 53.336 | 2.502 | 1.00 | 11.22 | C |
| ATOM | 5237 | CZ3 | TRP | D | 33 | 33.692 | 52.199 | 1.891 | 1.00 | 10.99 | C |
| ATOM | 5238 | CH2 | TRP | D | 33 | 34.676 | 52.520 | 2.828 | 1.00 | 11.10 | C |
| ATOM | 5239 | N | MET | D | 34 | 34.339 | 54.222 | -5.487 | 1.00 | 12.18 | N |
| ATOM | 5240 | CA | MET | D | 34 | 33.638 | 54.039 | -6.739 | 1.00 | 11.91 | C |
| ATOM | 5241 | C | MET | D | 34 | 33.448 | 52.539 | -6.986 | 1.00 | 11.12 | C |
| ATOM | 5242 | O | MET | D | 34 | 34.332 | 51.747 | -6.708 | 1.00 | 13.40 | O |
| ATOM | 5243 | CB | MET | D | 34 | 34.467 | 54.588 | -7.893 | 1.00 | 13.08 | C |
| ATOM | 5244 | CG | MET | D | 34 | 34.714 | 56.115 | -7.857 | 1.00 | 13.08 | C |
| ATOM | 5245 | SD | MET | D | 34 | 33.260 | 56.944 | -8.347 | 1.00 | 15.49 | S |
| ATOM | 5246 | CE | MET | D | 34 | 33.588 | 58.535 | -7.552 | 1.00 | 14.86 | C |
| ATOM | 5247 | N | SER | D | 35 | 32.282 | 52.176 | -7.488 | 1.00 | 12.03 | N |
| ATOM | 5248 | CA | SER | D | 35 | 31.996 | 50.752 | -7.788 | 1.00 | 10.31 | C |
| ATOM | 5249 | C | SER | D | 35 | 31.230 | 50.660 | -9.071 | 1.00 | 12.24 | C |
| ATOM | 5250 | O | SER | D | 35 | 30.678 | 51.642 | -9.582 | 1.00 | 9.95 | O |
| ATOM | 5251 | CB | SER | D | 35 | 31.207 | 50.059 | -6.658 | 1.00 | 10.38 | C |
| ATOM | 5252 | OG | SER | D | 35 | 29.853 | 50.505 | -6.575 | 1.00 | 12.69 | O |
| ATOM | 5253 | N | TRP | D | 36 | 31.172 | 49.429 | -9.579 | 1.00 | 9.17 | N |

| ATOM | 5254 | CA | TRP | D | 36 | 30.328 | 49.091 | -10.681 | 1.00 | 9.15 | C |
|------|------|-----|-----|---|----|--------|--------|---------|------|------|---|
| ATOM | 5255 | C | TRP | D | 36 | 29.343 | 48.047 | -10.201 | 1.00 | 9.77 | C |
| ATOM | 5256 | O | TRP | D | 36 | 29.725 | 47.093 | -9.510 | 1.00 | 10.19 | O |
| ATOM | 5257 | CB | TRP | D | 36 | 31.142 | 48.550 | -11.844 | 1.00 | 9.60 | C |
| ATOM | 5258 | CG | TRP | D | 36 | 31.939 | 49.555 | -12.568 | 1.00 | 9.47 | C |
| ATOM | 5259 | CD1 | TRP | D | 36 | 33.253 | 49.808 | -12.405 | 1.00 | 8.69 | C |
| ATOM | 5260 | CD2 | TRP | D | 36 | 31.481 | 50.401 | -13.605 | 1.00 | 8.10 | C |
| ATOM | 5261 | NE1 | TRP | D | 36 | 33.656 | 50.788 | -13.286 | 1.00 | 10.75 | N |
| ATOM | 5262 | CE2 | TRP | D | 36 | 32.575 | 51.179 | -14.023 | 1.00 | 9.65 | C |
| ATOM | 5263 | CE3 | TRP | D | 36 | 30.245 | 50.588 | -14.220 | 1.00 | 9.48 | C |
| ATOM | 5264 | CZ2 | TRP | D | 36 | 32.468 | 52.144 | -15.043 | 1.00 | 9.63 | C |
| ATOM | 5265 | CZ3 | TRP | D | 36 | 30.131 | 51.553 | -15.218 | 1.00 | 11.98 | C |
| ATOM | 5266 | CH2 | TRP | D | 36 | 31.256 | 52.307 | -15.628 | 1.00 | 10.13 | C |
| ATOM | 5267 | N | VAL | D | 37 | 28.074 | 48.262 | -10.549 | 1.00 | 11.73 | N |
| ATOM | 5268 | CA | VAL | D | 37 | 26.997 | 47.337 | -10.248 | 1.00 | 11.28 | C |
| ATOM | 5269 | C | VAL | D | 37 | 26.214 | 47.070 | -11.525 | 1.00 | 12.85 | C |
| ATOM | 5270 | O | VAL | D | 37 | 25.787 | 48.008 | -12.198 | 1.00 | 13.83 | O |
| ATOM | 5271 | CB | VAL | D | 37 | 26.026 | 47.908 | -9.167 | 1.00 | 10.44 | C |
| ATOM | 5272 | CG1 | VAL | D | 37 | 24.839 | 47.003 | -8.920 | 1.00 | 11.02 | C |
| ATOM | 5273 | CG2 | VAL | D | 37 | 26.734 | 48.208 | -7.883 | 1.00 | 7.47 | C |
| ATOM | 5274 | N | ARG | D | 38 | 25.993 | 45.793 | -11.845 | 1.00 | 11.13 | N |
| ATOM | 5275 | CA | ARG | D | 38 | 25.210 | 45.466 | -13.021 | 1.00 | 10.93 | C |
| ATOM | 5276 | C | ARG | D | 38 | 23.813 | 44.960 | -12.690 | 1.00 | 12.20 | C |
| ATOM | 5277 | O | ARG | D | 38 | 23.574 | 44.470 | -11.595 | 1.00 | 11.71 | O |
| ATOM | 5278 | CB | ARG | D | 38 | 25.961 | 44.517 | -13.926 | 1.00 | 12.23 | C |
| ATOM | 5279 | CG | ARG | D | 38 | 26.208 | 43.181 | -13.314 | 1.00 | 12.97 | C |
| ATOM | 5280 | CD | ARG | D | 38 | 27.145 | 42.383 | -14.201 | 1.00 | 16.29 | C |
| ATOM | 5281 | NE | ARG | D | 38 | 27.274 | 41.033 | -13.698 | 1.00 | 14.42 | N |
| ATOM | 5282 | CZ | ARG | D | 38 | 28.170 | 40.150 | -14.115 | 1.00 | 14.80 | C |
| ATOM | 5283 | NH1 | ARG | D | 38 | 29.054 | 40.461 | -15.029 | 1.00 | 11.70 | N |
| ATOM | 5284 | NH2 | ARG | D | 38 | 28.190 | 38.928 | -13.579 | 1.00 | 16.90 | N |
| ATOM | 5285 | N | GLN | D | 39 | 22.899 | 45.130 | -13.641 | 1.00 | 12.40 | N |
| ATOM | 5286 | CA | GLN | D | 39 | 21.517 | 44.673 | -13.510 | 1.00 | 13.47 | C |
| ATOM | 5287 | C | GLN | D | 39 | 21.109 | 43.823 | -14.701 | 1.00 | 14.27 | C |
| ATOM | 5288 | O | GLN | D | 39 | 21.337 | 44.186 | -15.838 | 1.00 | 10.93 | O |
| ATOM | 5289 | CB | GLN | D | 39 | 20.568 | 45.872 | -13.353 | 1.00 | 13.49 | C |
| ATOM | 5290 | CG | GLN | D | 39 | 19.125 | 45.493 | -13.018 | 1.00 | 14.65 | C |
| ATOM | 5291 | CD | GLN | D | 39 | 18.304 | 46.690 | -12.568 | 1.00 | 16.60 | C |
| ATOM | 5292 | OE1 | GLN | D | 39 | 18.439 | 47.768 | -13.121 | 1.00 | 20.28 | O |
| ATOM | 5293 | NE2 | GLN | D | 39 | 17.446 | 46.486 | -11.563 | 1.00 | 20.29 | N |
| ATOM | 5294 | N | SER | D | 40 | 20.520 | 42.662 | -14.410 | 1.00 | 16.56 | N |
| ATOM | 5295 | CA | SER | D | 40 | 19.926 | 41.788 | -15.433 | 1.00 | 17.61 | C |
| ATOM | 5296 | C | SER | D | 40 | 18.635 | 41.202 | -14.879 | 1.00 | 19.47 | C |
| ATOM | 5297 | O | SER | D | 40 | 18.477 | 41.117 | -13.663 | 1.00 | 18.43 | O |
| ATOM | 5298 | CB | SER | D | 40 | 20.874 | 40.642 | -15.766 | 1.00 | 18.63 | C |
| ATOM | 5299 | OG | SER | D | 40 | 21.135 | 39.847 | -14.607 | 1.00 | 20.29 | O |
| ATOM | 5300 | N | PRO | D | 41 | 17.703 | 40.780 | -15.758 | 1.00 | 22.61 | N |
| ATOM | 5301 | CA | PRO | D | 41 | 16.517 | 40.094 | -15.226 | 1.00 | 24.46 | C |
| ATOM | 5302 | C | PRO | D | 41 | 16.829 | 38.818 | -14.447 | 1.00 | 25.50 | C |
| ATOM | 5303 | O | PRO | D | 41 | 16.183 | 38.531 | -13.444 | 1.00 | 26.80 | O |
| ATOM | 5304 | CB | PRO | D | 41 | 15.718 | 39.762 | -16.488 | 1.00 | 24.11 | C |
| ATOM | 5305 | CG | PRO | D | 41 | 16.171 | 40.741 | -17.486 | 1.00 | 24.48 | C |
| ATOM | 5306 | CD | PRO | D | 41 | 17.628 | 40.917 | -17.217 | 1.00 | 22.11 | C |
| ATOM | 5307 | N | GLU | D | 42 | 17.842 | 38.077 | -14.866 | 1.00 | 27.22 | N |
| ATOM | 5308 | CA | GLU | D | 42 | 18.111 | 36.785 | -14.245 | 1.00 | 27.64 | C |
| ATOM | 5309 | C | GLU | D | 42 | 18.850 | 36.832 | -12.886 | 1.00 | 27.73 | C |
| ATOM | 5310 | O | GLU | D | 42 | 18.713 | 35.900 | -12.087 | 1.00 | 27.55 | O |
| ATOM | 5311 | CB | GLU | D | 42 | 18.783 | 35.796 | -15.234 | 1.00 | 29.41 | C |
| ATOM | 5312 | CG | GLU | D | 42 | 19.758 | 36.363 | -16.230 | 1.00 | 32.78 | C |
| ATOM | 5313 | CD | GLU | D | 42 | 19.075 | 37.018 | -17.421 | 1.00 | 33.62 | C |
| ATOM | 5314 | OE1 | GLU | D | 42 | 19.151 | 38.258 | -17.525 | 1.00 | 32.84 | O |
| ATOM | 5315 | OE2 | GLU | D | 42 | 18.449 | 36.313 | -18.245 | 1.00 | 35.36 | O |
| ATOM | 5316 | N | LYS | D | 43 | 19.614 | 37.897 | -12.621 | 1.00 | 25.84 | N |
| ATOM | 5317 | CA | LYS | D | 43 | 20.412 | 38.001 | -11.397 | 1.00 | 25.14 | C |
| ATOM | 5318 | C | LYS | D | 43 | 20.126 | 39.253 | -10.547 | 1.00 | 23.10 | C |

```
ATOM  5319  O    LYS D  43    20.709  39.403  -9.482  1.00 22.56       O
ATOM  5320  CB   LYS D  43    21.916  37.947 -11.715  1.00 27.76       C
ATOM  5321  CG   LYS D  43    22.429  36.643 -12.333  1.00 30.55       C
ATOM  5322  CD   LYS D  43    22.304  35.441 -11.395  1.00 32.69       C
ATOM  5323  CE   LYS D  43    22.895  34.177 -12.040  1.00 33.02       C
ATOM  5324  NZ   LYS D  43    22.553  32.909 -11.300  1.00 34.72       N
ATOM  5325  N    GLY D  44    19.253  40.133 -11.024  1.00 19.93       N
ATOM  5326  CA   GLY D  44    18.942  41.400 -10.335  1.00 19.18       C
ATOM  5327  C    GLY D  44    20.169  42.286 -10.267  1.00 16.66       C
ATOM  5328  O    GLY D  44    21.001  42.270 -11.185  1.00 15.98       O
ATOM  5329  N    LEU D  45    20.268  43.088  -9.206  1.00 14.48       N
ATOM  5330  CA   LEU D  45    21.430  43.939  -9.001  1.00 12.73       C
ATOM  5331  C    LEU D  45    22.600  43.162  -8.432  1.00 13.12       C
ATOM  5332  O    LEU D  45    22.475  42.523  -7.379  1.00 12.84       O
ATOM  5333  CB   LEU D  45    21.105  45.062  -8.001  1.00 13.97       C
ATOM  5334  CG   LEU D  45    20.116  46.085  -8.483  1.00 12.74       C
ATOM  5335  CD1  LEU D  45    19.629  46.923  -7.318  1.00 15.83       C
ATOM  5336  CD2  LEU D  45    20.691  46.948  -9.611  1.00 14.19       C
ATOM  5337  N    GLU D  46    23.736  43.271  -9.113  1.00 12.63       N
ATOM  5338  CA   GLU D  46    24.952  42.575  -8.786  1.00 13.88       C
ATOM  5339  C    GLU D  46    26.187  43.495  -8.765  1.00 12.22       C
ATOM  5340  O    GLU D  46    26.667  43.916  -9.794  1.00 11.82       O
ATOM  5341  CB   GLU D  46    25.101  41.501  -9.854  1.00 15.44       C
ATOM  5342  CG   GLU D  46    26.317  40.702  -9.855  1.00 18.59       C
ATOM  5343  CD   GLU D  46    26.160  39.494 -10.837  1.00 20.16       C
ATOM  5344  OE1  GLU D  46    25.360  39.568 -11.827  1.00 20.53       O
ATOM  5345  OE2  GLU D  46    26.841  38.481 -10.584  1.00 27.29       O
ATOM  5346  N    TRP D  47    26.720  43.733  -7.571  1.00 12.21       N
ATOM  5347  CA   TRP D  47    27.985  44.431  -7.393  1.00 10.75       C
ATOM  5348  C    TRP D  47    29.106  43.603  -7.992  1.00 12.81       C
ATOM  5349  O    TRP D  47    29.197  42.392  -7.696  1.00 11.31       O
ATOM  5350  CB   TRP D  47    28.218  44.620  -5.921  1.00 12.72       C
ATOM  5351  CG   TRP D  47    29.482  45.229  -5.516  1.00 12.72       C
ATOM  5352  CD1  TRP D  47    29.741  46.580  -5.391  1.00 13.68       C
ATOM  5353  CD2  TRP D  47    30.650  44.552  -5.053  1.00 13.44       C
ATOM  5354  NE1  TRP D  47    30.986  46.767  -4.905  1.00 12.13       N
ATOM  5355  CE2  TRP D  47    31.588  45.549  -4.699  1.00 12.75       C
ATOM  5356  CE3  TRP D  47    31.014  43.183  -4.926  1.00 12.31       C
ATOM  5357  CZ2  TRP D  47    32.872  45.241  -4.224  1.00 12.49       C
ATOM  5358  CZ3  TRP D  47    32.289  42.881  -4.448  1.00 12.89       C
ATOM  5359  CH2  TRP D  47    33.202  43.901  -4.107  1.00 13.88       C
ATOM  5360  N    VAL D  48    29.937  44.217  -8.826  1.00 11.33       N
ATOM  5361  CA   VAL D  48    31.048  43.486  -9.479  1.00 13.81       C
ATOM  5362  C    VAL D  48    32.461  43.952  -9.172  1.00 12.26       C
ATOM  5363  O    VAL D  48    33.400  43.150  -9.166  1.00 11.89       O
ATOM  5364  CB   VAL D  48    30.842  43.318 -11.007  1.00 14.51       C
ATOM  5365  CG1  VAL D  48    29.485  42.615 -11.282  1.00 15.83       C
ATOM  5366  CG2  VAL D  48    30.968  44.650 -11.748  1.00 16.61       C
ATOM  5367  N    ALA D  49    32.657  45.253  -8.896  1.00 10.62       N
ATOM  5368  CA   ALA D  49    33.984  45.767  -8.692  1.00  9.59       C
ATOM  5369  C    ALA D  49    33.923  47.077  -7.917  1.00  8.66       C
ATOM  5370  O    ALA D  49    32.957  47.848  -8.060  1.00  9.94       O
ATOM  5371  CB   ALA D  49    34.674  45.994 -10.010  1.00 10.46       C
ATOM  5372  N    GLU D  50    34.954  47.309  -7.116  1.00 10.84       N
ATOM  5373  CA   GLU D  50    35.084  48.566  -6.338  1.00  9.96       C
ATOM  5374  C    GLU D  50    36.541  49.022  -6.216  1.00 10.81       C
ATOM  5375  O    GLU D  50    37.450  48.231  -6.104  1.00 10.38       O
ATOM  5376  CB   GLU D  50    34.481  48.366  -4.961  1.00 11.31       C
ATOM  5377  CG   GLU D  50    34.358  49.644  -4.118  1.00 11.10       C
ATOM  5378  CD   GLU D  50    33.356  49.516  -3.027  1.00 11.17       C
ATOM  5379  OE1  GLU D  50    32.264  48.988  -3.300  1.00 11.51       O
ATOM  5380  OE2  GLU D  50    33.632  49.957  -1.883  1.00 13.83       O
ATOM  5381  N    ILE D  51    36.750  50.340  -6.201  1.00 10.26       N
ATOM  5382  CA   ILE D  51    38.068  50.920  -6.047  1.00 10.55       C
ATOM  5383  C    ILE D  51    38.023  52.020  -4.988  1.00 10.56       C
```

| ATOM | 5384 | O   | ILE | D | 51  | 37.080 | 52.809 | -4.921 | 1.00 | 11.35 | O |
| ATOM | 5385 | CB  | ILE | D | 51  | 38.655 | 51.427 | -7.396 | 1.00 | 10.58 | C |
| ATOM | 5386 | CG1 | ILE | D | 51  | 40.147 | 51.784 | -7.249 | 1.00 | 9.03  | C |
| ATOM | 5387 | CG2 | ILE | D | 51  | 37.816 | 52.539 | -8.001 | 1.00 | 10.92 | C |
| ATOM | 5388 | CD1 | ILE | D | 51  | 40.895 | 51.820 | -8.513 | 1.00 | 10.50 | C |
| ATOM | 5389 | N   | ARG | D | 52  | 39.048 | 52.020 | -4.144 | 1.00 | 12.33 | N |
| ATOM | 5390 | CA  | ARG | D | 52  | 39.116 | 52.973 | -3.024 | 1.00 | 12.43 | C |
| ATOM | 5391 | C   | ARG | D | 52  | 40.003 | 54.134 | -3.442 | 1.00 | 13.25 | C |
| ATOM | 5392 | O   | ARG | D | 52  | 40.143 | 54.414 | -4.618 | 1.00 | 12.65 | O |
| ATOM | 5393 | CB  | ARG | D | 52  | 39.576 | 52.278 | -1.763 | 1.00 | 12.39 | C |
| ATOM | 5394 | CG  | ARG | D | 52  | 38.656 | 51.095 | -1.313 | 1.00 | 11.75 | C |
| ATOM | 5395 | CD  | ARG | D | 52  | 37.287 | 51.598 | -0.891 | 1.00 | 14.00 | C |
| ATOM | 5396 | NE  | ARG | D | 52  | 36.266 | 50.564 | -0.694 | 1.00 | 13.59 | N |
| ATOM | 5397 | CZ  | ARG | D | 52  | 36.126 | 49.838 | 0.403  | 1.00 | 12.79 | C |
| ATOM | 5398 | NH1 | ARG | D | 52  | 36.969 | 49.918 | 1.417  | 1.00 | 14.04 | N |
| ATOM | 5399 | NH2 | ARG | D | 52  | 35.151 | 48.983 | 0.473  | 1.00 | 13.31 | N |
| ATOM | 5400 | N   | LEU | D | 52A | 40.565 | 54.842 | -2.475 | 1.00 | 14.39 | N |
| ATOM | 5401 | CA  | LEU | D | 52A | 41.153 | 56.133 | -2.767 | 1.00 | 14.42 | C |
| ATOM | 5402 | C   | LEU | D | 52A | 42.653 | 56.058 | -2.919 | 1.00 | 17.14 | C |
| ATOM | 5403 | O   | LEU | D | 52A | 43.268 | 55.082 | -2.527 | 1.00 | 14.94 | O |
| ATOM | 5404 | CB  | LEU | D | 52A | 40.807 | 57.122 | -1.652 | 1.00 | 14.26 | C |
| ATOM | 5405 | CG  | LEU | D | 52A | 39.350 | 57.219 | -1.251 | 1.00 | 11.82 | C |
| ATOM | 5406 | CD1 | LEU | D | 52A | 39.174 | 58.281 | -0.123 | 1.00 | 16.23 | C |
| ATOM | 5407 | CD2 | LEU | D | 52A | 38.445 | 57.530 | -2.426 | 1.00 | 11.29 | C |
| ATOM | 5408 | N   | LYS | D | 52B | 43.243 | 57.131 | -3.452 | 1.00 | 19.41 | N |
| ATOM | 5409 | CA  | LYS | D | 52B | 44.710 | 57.270 | -3.468 | 1.00 | 21.92 | C |
| ATOM | 5410 | C   | LYS | D | 52B | 45.355 | 56.997 | -2.111 | 1.00 | 22.13 | C |
| ATOM | 5411 | O   | LYS | D | 52B | 46.365 | 56.303 | -2.048 | 1.00 | 24.58 | O |
| ATOM | 5412 | CB  | LYS | D | 52B | 45.107 | 58.648 | -3.985 | 1.00 | 22.52 | C |
| ATOM | 5413 | CG  | LYS | D | 52B | 46.622 | 58.911 | -4.032 | 1.00 | 23.71 | C |
| ATOM | 5414 | CD  | LYS | D | 52B | 46.930 | 60.132 | -4.898 | 1.00 | 25.73 | C |
| ATOM | 5415 | CE  | LYS | D | 52B | 48.086 | 60.954 | -4.358 | 1.00 | 28.75 | C |
| ATOM | 5416 | NZ  | LYS | D | 52B | 48.281 | 62.183 | -5.196 | 1.00 | 29.13 | N |
| ATOM | 5417 | N   | SER | D | 52C | 44.744 | 57.469 | -1.022 | 1.00 | 22.46 | N |
| ATOM | 5418 | CA  | SER | D | 52C | 45.267 | 57.259 | 0.319  | 1.00 | 22.50 | C |
| ATOM | 5419 | C   | SER | D | 52C | 45.180 | 55.804 | 0.781  | 1.00 | 22.35 | C |
| ATOM | 5420 | O   | SER | D | 52C | 45.767 | 55.437 | 1.786  | 1.00 | 21.89 | O |
| ATOM | 5421 | CB  | SER | D | 52C | 44.547 | 58.158 | 1.316  | 1.00 | 23.41 | C |
| ATOM | 5422 | OG  | SER | D | 52C | 43.152 | 57.930 | 1.348  | 1.00 | 23.41 | O |
| ATOM | 5423 | N   | ASP | D | 53  | 44.425 | 54.991 | 0.040  | 1.00 | 20.97 | N |
| ATOM | 5424 | CA  | ASP | D | 53  | 44.314 | 53.560 | 0.266  | 1.00 | 20.71 | C |
| ATOM | 5425 | C   | ASP | D | 53  | 45.132 | 52.772 | -0.771 | 1.00 | 21.55 | C |
| ATOM | 5426 | O   | ASP | D | 53  | 44.882 | 51.581 | -0.991 | 1.00 | 21.57 | O |
| ATOM | 5427 | CB  | ASP | D | 53  | 42.848 | 53.148 | 0.121  | 1.00 | 20.62 | C |
| ATOM | 5428 | CG  | ASP | D | 53  | 41.921 | 53.974 | 0.978  | 1.00 | 21.59 | C |
| ATOM | 5429 | OD1 | ASP | D | 53  | 42.161 | 54.012 | 2.208  | 1.00 | 23.45 | O |
| ATOM | 5430 | OD2 | ASP | D | 53  | 40.943 | 54.563 | 0.446  | 1.00 | 18.12 | O |
| ATOM | 5431 | N   | ASN | D | 54  | 46.083 | 53.434 | -1.418 | 1.00 | 21.52 | N |
| ATOM | 5432 | CA  | ASN | D | 54  | 46.810 | 52.872 | -2.553 | 1.00 | 21.87 | C |
| ATOM | 5433 | C   | ASN | D | 54  | 45.898 | 52.296 | -3.639 | 1.00 | 19.84 | C |
| ATOM | 5434 | O   | ASN | D | 54  | 46.202 | 51.244 | -4.238 | 1.00 | 18.88 | O |
| ATOM | 5435 | CB  | ASN | D | 54  | 47.791 | 51.790 | -2.081 | 1.00 | 24.14 | C |
| ATOM | 5436 | CG  | ASN | D | 54  | 48.911 | 51.530 | -3.090 | 1.00 | 28.72 | C |
| ATOM | 5437 | OD1 | ASN | D | 54  | 49.242 | 52.389 | -3.921 | 1.00 | 32.86 | O |
| ATOM | 5438 | ND2 | ASN | D | 54  | 49.489 | 50.319 | -3.033 | 1.00 | 32.27 | N |
| ATOM | 5439 | N   | TYR | D | 55  | 44.790 | 52.985 | -3.890 | 1.00 | 17.88 | N |
| ATOM | 5440 | CA  | TYR | D | 55  | 43.848 | 52.578 | -4.902 | 1.00 | 18.36 | C |
| ATOM | 5441 | C   | TYR | D | 55  | 43.433 | 51.119 | -4.801 | 1.00 | 16.48 | C |
| ATOM | 5442 | O   | TYR | D | 55  | 43.342 | 50.435 | -5.829 | 1.00 | 15.98 | O |
| ATOM | 5443 | CB  | TYR | D | 55  | 44.430 | 52.826 | -6.274 | 1.00 | 19.29 | C |
| ATOM | 5444 | CG  | TYR | D | 55  | 44.624 | 54.294 | -6.572 | 1.00 | 21.43 | C |
| ATOM | 5445 | CD1 | TYR | D | 55  | 43.537 | 55.149 | -6.611 | 1.00 | 21.13 | C |
| ATOM | 5446 | CD2 | TYR | D | 55  | 45.875 | 54.806 | -6.852 | 1.00 | 21.46 | C |
| ATOM | 5447 | CE1 | TYR | D | 55  | 43.685 | 56.480 | -6.891 | 1.00 | 23.55 | C |
| ATOM | 5448 | CE2 | TYR | D | 55  | 46.038 | 56.148 | -7.136 | 1.00 | 24.48 | C |

```
ATOM   5449  CZ   TYR D  55     44.927  56.973   -7.163  1.00 23.23           C
ATOM   5450  OH   TYR D  55     45.055  58.307   -7.444  1.00 26.06           O
ATOM   5451  N    ALA D  56     43.154  50.660   -3.585  1.00 17.50           N
ATOM   5452  CA   ALA D  56     42.771  49.254   -3.370  1.00 16.57           C
ATOM   5453  C    ALA D  56     41.550  48.882   -4.219  1.00 16.38           C
ATOM   5454  O    ALA D  56     40.579  49.633   -4.278  1.00 13.46           O
ATOM   5455  CB   ALA D  56     42.495  48.980   -1.923  1.00 17.63           C
ATOM   5456  N    THR D  57     41.610  47.708   -4.855  1.00 15.85           N
ATOM   5457  CA   THR D  57     40.482  47.195   -5.654  1.00 15.09           C
ATOM   5458  C    THR D  57     39.912  45.900   -5.056  1.00 15.05           C
ATOM   5459  O    THR D  57     40.599  45.165   -4.330  1.00 14.76           O
ATOM   5460  CB   THR D  57     40.870  46.939   -7.119  1.00 15.28           C
ATOM   5461  OG1  THR D  57     41.947  45.997   -7.169  1.00 15.42           O
ATOM   5462  CG2  THR D  57     41.279  48.214   -7.842  1.00 16.07           C
ATOM   5463  N    TYR D  58     38.621  45.664   -5.319  1.00 13.21           N
ATOM   5464  CA   TYR D  58     37.897  44.516   -4.807  1.00 14.12           C
ATOM   5465  C    TYR D  58     36.954  44.070   -5.907  1.00 13.31           C
ATOM   5466  O    TYR D  58     36.465  44.884   -6.673  1.00 11.69           O
ATOM   5467  CB   TYR D  58     37.086  44.877   -3.543  1.00 15.46           C
ATOM   5468  CG   TYR D  58     37.961  45.472   -2.452  1.00 14.58           C
ATOM   5469  CD1  TYR D  58     38.766  44.659   -1.663  1.00 15.27           C
ATOM   5470  CD2  TYR D  58     38.041  46.837   -2.279  1.00 16.71           C
ATOM   5471  CE1  TYR D  58     39.606  45.189   -0.706  1.00 18.21           C
ATOM   5472  CE2  TYR D  58     38.873  47.381   -1.296  1.00 15.00           C
ATOM   5473  CZ   TYR D  58     39.643  46.549   -0.521  1.00 17.21           C
ATOM   5474  OH   TYR D  58     40.469  47.092    0.451  1.00 19.03           O
ATOM   5475  N    TYR D  59     36.703  42.767   -6.001  1.00 13.70           N
ATOM   5476  CA   TYR D  59     35.914  42.231   -7.088  1.00 12.38           C
ATOM   5477  C    TYR D  59     34.984  41.146   -6.631  1.00 13.17           C
ATOM   5478  O    TYR D  59     35.309  40.413   -5.688  1.00 14.89           O
ATOM   5479  CB   TYR D  59     36.825  41.633   -8.157  1.00 11.78           C
ATOM   5480  CG   TYR D  59     37.732  42.608   -8.808  1.00 12.82           C
ATOM   5481  CD1  TYR D  59     37.315  43.336   -9.907  1.00  9.97           C
ATOM   5482  CD2  TYR D  59     39.007  42.840   -8.306  1.00 13.83           C
ATOM   5483  CE1  TYR D  59     38.131  44.228  -10.504  1.00 11.35           C
ATOM   5484  CE2  TYR D  59     39.836  43.727   -8.905  1.00 12.97           C
ATOM   5485  CZ   TYR D  59     39.381  44.447   -9.994  1.00 11.17           C
ATOM   5486  OH   TYR D  59     40.196  45.324  -10.626  1.00 11.10           O
ATOM   5487  N    ALA D  60     33.853  41.005   -7.308  1.00 13.66           N
ATOM   5488  CA   ALA D  60     32.976  39.865   -7.062  1.00 12.61           C
ATOM   5489  C    ALA D  60     33.706  38.598   -7.538  1.00 13.22           C
ATOM   5490  O    ALA D  60     34.432  38.621   -8.533  1.00 12.64           O
ATOM   5491  CB   ALA D  60     31.673  40.002   -7.808  1.00 12.89           C
ATOM   5492  N    GLU D  61     33.504  37.495   -6.815  1.00 15.85           N
ATOM   5493  CA   GLU D  61     34.206  36.241   -7.136  1.00 19.68           C
ATOM   5494  C    GLU D  61     34.038  35.883   -8.623  1.00 19.75           C
ATOM   5495  O    GLU D  61     34.987  35.456   -9.297  1.00 20.28           O
ATOM   5496  CB   GLU D  61     33.686  35.097   -6.250  1.00 22.34           C
ATOM   5497  CG   GLU D  61     33.968  33.717   -6.832  1.00 28.96           C
ATOM   5498  CD   GLU D  61     34.403  32.692   -5.809  1.00 33.16           C
ATOM   5499  OE1  GLU D  61     35.122  33.059   -4.842  1.00 38.82           O
ATOM   5500  OE2  GLU D  61     34.052  31.503   -6.012  1.00 37.45           O
ATOM   5501  N    SER D  62     32.845  36.100   -9.141  1.00 20.17           N
ATOM   5502  CA   SER D  62     32.528  35.647  -10.504  1.00 20.52           C
ATOM   5503  C    SER D  62     33.214  36.483  -11.597  1.00 19.36           C
ATOM   5504  O    SER D  62     33.227  36.086  -12.751  1.00 21.08           O
ATOM   5505  CB   SER D  62     31.021  35.631  -10.706  1.00 21.70           C
ATOM   5506  OG   SER D  62     30.477  36.921  -10.599  1.00 24.32           O
ATOM   5507  N    VAL D  63     33.761  37.652  -11.270  1.00 16.84           N
ATOM   5508  CA   VAL D  63     34.467  38.422  -12.293  1.00 17.22           C
ATOM   5509  C    VAL D  63     35.971  38.556  -12.069  1.00 19.15           C
ATOM   5510  O    VAL D  63     36.665  39.092  -12.925  1.00 18.42           O
ATOM   5511  CB   VAL D  63     33.822  39.826  -12.497  1.00 16.15           C
ATOM   5512  CG1  VAL D  63     32.343  39.688  -12.742  1.00 14.51           C
ATOM   5513  CG2  VAL D  63     34.075  40.677  -11.316  1.00 14.22           C
```

| ATOM | 5514 | N   | LYS D | 64 | 36.483 | 38.083 | -10.928 | 1.00 | 21.30 | N |
|------|------|-----|-------|----|--------|--------|---------|------|-------|---|
| ATOM | 5515 | CA  | LYS D | 64 | 37.908 | 38.202 | -10.620 | 1.00 | 23.06 | C |
| ATOM | 5516 | C   | LYS D | 64 | 38.733 | 37.584 | -11.748 | 1.00 | 23.15 | C |
| ATOM | 5517 | O   | LYS D | 64 | 38.418 | 36.488 | -12.228 | 1.00 | 23.73 | O |
| ATOM | 5518 | CB  | LYS D | 64 | 38.270 | 37.506 | -9.299  | 1.00 | 25.46 | C |
| ATOM | 5519 | CG  | LYS D | 64 | 37.489 | 37.953 | -8.081  | 1.00 | 28.26 | C |
| ATOM | 5520 | CD  | LYS D | 64 | 38.369 | 38.117 | -6.820  | 1.00 | 30.44 | C |
| ATOM | 5521 | CE  | LYS D | 64 | 37.513 | 38.296 | -5.555  | 1.00 | 29.94 | C |
| ATOM | 5522 | NZ  | LYS D | 64 | 38.333 | 38.389 | -4.313  | 1.00 | 32.72 | N |
| ATOM | 5523 | N   | GLY D | 65 | 39.772 | 38.293 | -12.170 | 1.00 | 22.78 | N |
| ATOM | 5524 | CA  | GLY D | 65 | 40.578 | 37.891 | -13.316 | 1.00 | 23.23 | C |
| ATOM | 5525 | C   | GLY D | 65 | 40.030 | 38.237 | -14.692 | 1.00 | 22.87 | C |
| ATOM | 5526 | O   | GLY D | 65 | 40.771 | 38.175 | -15.660 | 1.00 | 26.01 | O |
| ATOM | 5527 | N   | LYS D | 66 | 38.748 | 38.579 | -14.808 | 1.00 | 20.20 | N |
| ATOM | 5528 | CA  | LYS D | 66 | 38.205 | 39.025 | -16.084 | 1.00 | 18.03 | C |
| ATOM | 5529 | C   | LYS D | 66 | 38.037 | 40.546 | -16.154 | 1.00 | 15.83 | C |
| ATOM | 5530 | O   | LYS D | 66 | 38.109 | 41.105 | -17.236 | 1.00 | 14.48 | O |
| ATOM | 5531 | CB  | LYS D | 66 | 36.854 | 38.412 | -16.333 | 1.00 | 19.98 | C |
| ATOM | 5532 | CG  | LYS D | 66 | 36.865 | 36.878 | -16.560 | 1.00 | 21.95 | C |
| ATOM | 5533 | CD  | LYS D | 66 | 35.541 | 36.480 | -17.163 | 1.00 | 23.12 | C |
| ATOM | 5534 | CE  | LYS D | 66 | 35.443 | 35.014 | -17.463 | 1.00 | 23.61 | C |
| ATOM | 5535 | NZ  | LYS D | 66 | 36.027 | 34.551 | -18.771 | 1.00 | 23.38 | N |
| ATOM | 5536 | N   | PHE D | 67 | 37.698 | 41.168 | -15.025 | 1.00 | 13.64 | N |
| ATOM | 5537 | CA  | PHE D | 67 | 37.417 | 42.627 | -14.972 | 1.00 | 14.15 | C |
| ATOM | 5538 | C   | PHE D | 67 | 38.523 | 43.361 | -14.217 | 1.00 | 13.59 | C |
| ATOM | 5539 | O   | PHE D | 67 | 39.060 | 42.845 | -13.231 | 1.00 | 15.00 | O |
| ATOM | 5540 | CB  | PHE D | 67 | 36.119 | 42.883 | -14.199 | 1.00 | 13.06 | C |
| ATOM | 5541 | CG  | PHE D | 67 | 34.869 | 42.515 | -14.912 | 1.00 | 13.80 | C |
| ATOM | 5542 | CD1 | PHE D | 67 | 34.872 | 41.815 | -16.104 | 1.00 | 12.99 | C |
| ATOM | 5543 | CD2 | PHE D | 67 | 33.650 | 42.824 | -14.307 | 1.00 | 12.33 | C |
| ATOM | 5544 | CE1 | PHE D | 67 | 33.677 | 41.484 | -16.708 | 1.00 | 15.05 | C |
| ATOM | 5545 | CE2 | PHE D | 67 | 32.457 | 42.498 | -14.912 | 1.00 | 15.95 | C |
| ATOM | 5546 | CZ  | PHE D | 67 | 32.481 | 41.813 | -16.106 | 1.00 | 13.37 | C |
| ATOM | 5547 | N   | THR D | 68 | 38.829 | 44.580 | -14.649 | 1.00 | 12.10 | N |
| ATOM | 5548 | CA  | THR D | 68 | 39.790 | 45.425 | -13.965 | 1.00 | 11.90 | C |
| ATOM | 5549 | C   | THR D | 68 | 39.183 | 46.800 | -13.850 | 1.00 | 10.02 | C |
| ATOM | 5550 | O   | THR D | 68 | 38.821 | 47.372 | -14.842 | 1.00 | 9.60  | O |
| ATOM | 5551 | CB  | THR D | 68 | 41.095 | 45.539 | -14.712 | 1.00 | 13.77 | C |
| ATOM | 5552 | OG1 | THR D | 68 | 41.651 | 44.228 | -14.855 | 1.00 | 16.81 | O |
| ATOM | 5553 | CG2 | THR D | 68 | 42.097 | 46.413 | -13.930 | 1.00 | 14.71 | C |
| ATOM | 5554 | N   | ILE D | 69 | 39.009 | 47.254 | -12.620 | 1.00 | 11.28 | N |
| ATOM | 5555 | CA  | ILE D | 69 | 38.476 | 48.607 | -12.349 | 1.00 | 11.23 | C |
| ATOM | 5556 | C   | ILE D | 69 | 39.652 | 49.553 | -12.132 | 1.00 | 11.90 | C |
| ATOM | 5557 | O   | ILE D | 69 | 40.621 | 49.187 | -11.504 | 1.00 | 10.88 | O |
| ATOM | 5558 | CB  | ILE D | 69 | 37.499 | 48.602 | -11.094 | 1.00 | 11.13 | C |
| ATOM | 5559 | CG1 | ILE D | 69 | 36.789 | 49.973 | -10.923 | 1.00 | 10.68 | C |
| ATOM | 5560 | CG2 | ILE D | 69 | 38.181 | 48.179 | -9.802  | 1.00 | 12.24 | C |
| ATOM | 5561 | CD1 | ILE D | 69 | 35.724 | 49.962 | -9.841  | 1.00 | 11.84 | C |
| ATOM | 5562 | N   | SER D | 70 | 39.554 | 50.783 | -12.637 | 1.00 | 12.04 | N |
| ATOM | 5563 | CA  | SER D | 70 | 40.640 | 51.748 | -12.492 | 1.00 | 12.90 | C |
| ATOM | 5564 | C   | SER D | 70 | 40.001 | 53.120 | -12.456 | 1.00 | 11.76 | C |
| ATOM | 5565 | O   | SER D | 70 | 38.856 | 53.267 | -12.837 | 1.00 | 10.49 | O |
| ATOM | 5566 | CB  | SER D | 70 | 41.664 | 51.651 | -13.653 | 1.00 | 13.36 | C |
| ATOM | 5567 | OG  | SER D | 70 | 41.088 | 51.893 | -14.924 | 1.00 | 15.02 | O |
| ATOM | 5568 | N   | ARG D | 71 | 40.736 | 54.118 | -11.963 | 1.00 | 13.06 | N |
| ATOM | 5569 | CA  | ARG D | 71 | 40.191 | 55.463 | -11.901 | 1.00 | 13.98 | C |
| ATOM | 5570 | C   | ARG D | 71 | 41.257 | 56.471 | -12.314 | 1.00 | 14.54 | C |
| ATOM | 5571 | O   | ARG D | 71 | 42.444 | 56.254 | -12.089 | 1.00 | 12.90 | O |
| ATOM | 5572 | CB  | ARG D | 71 | 39.664 | 55.743 | -10.500 | 1.00 | 14.87 | C |
| ATOM | 5573 | CG  | ARG D | 71 | 40.751 | 55.789 | -9.402  | 1.00 | 13.11 | C |
| ATOM | 5574 | CD  | ARG D | 71 | 40.162 | 55.763 | -7.995  | 1.00 | 14.36 | C |
| ATOM | 5575 | NE  | ARG D | 71 | 39.384 | 56.977 | -7.746  | 1.00 | 14.30 | N |
| ATOM | 5576 | CZ  | ARG D | 71 | 38.418 | 57.099 | -6.850  | 1.00 | 14.34 | C |
| ATOM | 5577 | NH1 | ARG D | 71 | 38.072 | 56.084 | -6.064  | 1.00 | 12.69 | N |
| ATOM | 5578 | NH2 | ARG D | 71 | 37.759 | 58.242 | -6.768  | 1.00 | 13.26 | N |

| ATOM | 5579 | N | ASP | D | 72 | 40.819 | 57.550 | -12.934 | 1.00 | 14.51 | N |
| ATOM | 5580 | CA | ASP | D | 72 | 41.715 | 58.647 | -13.306 | 1.00 | 15.85 | C |
| ATOM | 5581 | C | ASP | D | 72 | 41.163 | 59.865 | -12.592 | 1.00 | 14.32 | C |
| ATOM | 5582 | O | ASP | D | 72 | 40.258 | 60.511 | -13.095 | 1.00 | 15.37 | O |
| ATOM | 5583 | CB | ASP | D | 72 | 41.706 | 58.889 | -14.810 | 1.00 | 17.15 | C |
| ATOM | 5584 | CG | ASP | D | 72 | 42.694 | 59.971 | -15.228 | 1.00 | 19.84 | C |
| ATOM | 5585 | OD1 | ASP | D | 72 | 43.162 | 60.717 | -14.329 | 1.00 | 22.72 | O |
| ATOM | 5586 | OD2 | ASP | D | 72 | 43.030 | 60.032 | -16.427 | 1.00 | 21.66 | O |
| ATOM | 5587 | N | ASP | D | 73 | 41.714 | 60.137 | -11.424 | 1.00 | 15.93 | N |
| ATOM | 5588 | CA | ASP | D | 73 | 41.233 | 61.219 | -10.550 | 1.00 | 17.78 | C |
| ATOM | 5589 | C | ASP | D | 73 | 41.297 | 62.554 | -11.272 | 1.00 | 18.04 | C |
| ATOM | 5590 | O | ASP | D | 73 | 40.384 | 63.371 | -11.139 | 1.00 | 17.12 | O |
| ATOM | 5591 | CB | ASP | D | 73 | 42.015 | 61.215 | -9.232 | 1.00 | 17.97 | C |
| ATOM | 5592 | CG | ASP | D | 73 | 41.567 | 60.098 | -8.289 | 1.00 | 17.10 | C |
| ATOM | 5593 | OD1 | ASP | D | 73 | 40.459 | 59.540 | -8.506 | 1.00 | 19.79 | O |
| ATOM | 5594 | OD2 | ASP | D | 73 | 42.304 | 59.790 | -7.330 | 1.00 | 17.15 | O |
| ATOM | 5595 | N | SER | D | 74 | 42.309 | 62.734 | -12.116 | 1.00 | 19.31 | N |
| ATOM | 5596 | CA | SER | D | 74 | 42.496 | 64.013 | -12.808 | 1.00 | 20.18 | C |
| ATOM | 5597 | C | SER | D | 74 | 41.385 | 64.309 | -13.802 | 1.00 | 20.51 | C |
| ATOM | 5598 | O | SER | D | 74 | 41.078 | 65.490 | -14.066 | 1.00 | 21.47 | O |
| ATOM | 5599 | CB | SER | D | 74 | 43.873 | 64.071 | -13.492 | 1.00 | 21.87 | C |
| ATOM | 5600 | OG | SER | D | 74 | 43.933 | 63.205 | -14.610 | 1.00 | 24.41 | O |
| ATOM | 5601 | N | LYS | D | 75 | 40.747 | 63.256 | -14.328 | 1.00 | 18.62 | N |
| ATOM | 5602 | CA | LYS | D | 75 | 39.652 | 63.382 | -15.287 | 1.00 | 20.51 | C |
| ATOM | 5603 | C | LYS | D | 75 | 38.270 | 63.070 | -14.715 | 1.00 | 18.23 | C |
| ATOM | 5604 | O | LYS | D | 75 | 37.260 | 63.121 | -15.438 | 1.00 | 19.01 | O |
| ATOM | 5605 | CB | LYS | D | 75 | 39.898 | 62.446 | -16.464 | 1.00 | 21.49 | C |
| ATOM | 5606 | CG | LYS | D | 75 | 41.176 | 62.734 | -17.216 | 1.00 | 24.69 | C |
| ATOM | 5607 | CD | LYS | D | 75 | 41.162 | 62.032 | -18.576 | 1.00 | 26.92 | C |
| ATOM | 5608 | CE | LYS | D | 75 | 42.581 | 61.733 | -19.056 | 1.00 | 30.77 | C |
| ATOM | 5609 | NZ | LYS | D | 75 | 42.765 | 62.126 | -20.468 | 1.00 | 34.22 | N |
| ATOM | 5610 | N | SER | D | 76 | 38.232 | 62.759 | -13.427 | 1.00 | 17.00 | N |
| ATOM | 5611 | CA | SER | D | 76 | 37.021 | 62.399 | -12.725 | 1.00 | 16.13 | C |
| ATOM | 5612 | C | SER | D | 76 | 36.348 | 61.203 | -13.442 | 1.00 | 16.04 | C |
| ATOM | 5613 | O | SER | D | 76 | 35.123 | 61.187 | -13.639 | 1.00 | 14.55 | O |
| ATOM | 5614 | CB | SER | D | 76 | 36.088 | 63.609 | -12.612 | 1.00 | 17.03 | C |
| ATOM | 5615 | OG | SER | D | 76 | 36.701 | 64.572 | -11.762 | 1.00 | 14.49 | O |
| ATOM | 5616 | N | ARG | D | 77 | 37.178 | 60.241 | -13.837 | 1.00 | 16.31 | N |
| ATOM | 5617 | CA | ARG | D | 77 | 36.683 | 59.116 | -14.646 | 1.00 | 16.53 | C |
| ATOM | 5618 | C | ARG | D | 77 | 36.949 | 57.776 | -13.996 | 1.00 | 14.17 | C |
| ATOM | 5619 | O | ARG | D | 77 | 38.028 | 57.538 | -13.460 | 1.00 | 13.35 | O |
| ATOM | 5620 | CB | ARG | D | 77 | 37.305 | 59.113 | -16.052 | 1.00 | 17.96 | C |
| ATOM | 5621 | CG | ARG | D | 77 | 36.492 | 58.296 | -17.054 | 1.00 | 20.07 | C |
| ATOM | 5622 | CD | ARG | D | 77 | 36.784 | 58.660 | -18.490 | 1.00 | 21.55 | C |
| ATOM | 5623 | NE | ARG | D | 77 | 36.032 | 59.817 | -18.961 | 1.00 | 24.22 | N |
| ATOM | 5624 | CZ | ARG | D | 77 | 34.774 | 59.796 | -19.426 | 1.00 | 26.49 | C |
| ATOM | 5625 | NH1 | ARG | D | 77 | 34.039 | 58.672 | -19.455 | 1.00 | 25.37 | N |
| ATOM | 5626 | NH2 | ARG | D | 77 | 34.216 | 60.935 | -19.840 | 1.00 | 28.53 | N |
| ATOM | 5627 | N | LEU | D | 78 | 35.939 | 56.906 | -14.048 | 1.00 | 13.19 | N |
| ATOM | 5628 | CA | LEU | D | 78 | 36.036 | 55.519 | -13.559 | 1.00 | 12.65 | C |
| ATOM | 5629 | C | LEU | D | 78 | 35.985 | 54.636 | -14.781 | 1.00 | 11.43 | C |
| ATOM | 5630 | O | LEU | D | 78 | 35.242 | 54.948 | -15.699 | 1.00 | 11.68 | O |
| ATOM | 5631 | CB | LEU | D | 78 | 34.853 | 55.195 | -12.659 | 1.00 | 13.91 | C |
| ATOM | 5632 | CG | LEU | D | 78 | 34.854 | 53.833 | -11.950 | 1.00 | 12.95 | C |
| ATOM | 5633 | CD1 | LEU | D | 78 | 35.981 | 53.800 | -10.953 | 1.00 | 15.23 | C |
| ATOM | 5634 | CD2 | LEU | D | 78 | 33.519 | 53.545 | -11.276 | 1.00 | 13.78 | C |
| ATOM | 5635 | N | TYR | D | 79 | 36.731 | 53.525 | -14.763 | 1.00 | 11.20 | N |
| ATOM | 5636 | CA | TYR | D | 79 | 36.797 | 52.601 | -15.871 | 1.00 | 12.11 | C |
| ATOM | 5637 | C | TYR | D | 79 | 36.532 | 51.190 | -15.411 | 1.00 | 9.52 | C |
| ATOM | 5638 | O | TYR | D | 79 | 36.828 | 50.846 | -14.297 | 1.00 | 9.93 | O |
| ATOM | 5639 | CB | TYR | D | 79 | 38.186 | 52.586 | -16.456 | 1.00 | 15.22 | C |
| ATOM | 5640 | CG | TYR | D | 79 | 38.629 | 53.896 | -17.039 | 1.00 | 16.38 | C |
| ATOM | 5641 | CD1 | TYR | D | 79 | 38.250 | 54.250 | -18.318 | 1.00 | 18.33 | C |
| ATOM | 5642 | CD2 | TYR | D | 79 | 39.472 | 54.749 | -16.329 | 1.00 | 18.49 | C |
| ATOM | 5643 | CE1 | TYR | D | 79 | 38.677 | 55.430 | -18.895 | 1.00 | 20.40 | C |

345

| ATOM | 5644 | CE2 | TYR | D | 79 | 39.899 | 55.929 | -16.892 | 1.00 | 20.02 | C |
|------|------|-----|-----|---|-----|--------|--------|---------|------|-------|---|
| ATOM | 5645 | CZ | TYR | D | 79 | 39.493 | 56.258 | -18.180 | 1.00 | 20.85 | C |
| ATOM | 5646 | OH | TYR | D | 79 | 39.907 | 57.427 | -18.780 | 1.00 | 23.20 | O |
| ATOM | 5647 | N | LEU | D | 80 | 35.984 | 50.402 | -16.308 | 1.00 | 10.61 | N |
| ATOM | 5648 | CA | LEU | D | 80 | 35.946 | 48.946 | -16.139 | 1.00 | 10.81 | C |
| ATOM | 5649 | C | LEU | D | 80 | 36.375 | 48.312 | -17.450 | 1.00 | 9.47 | C |
| ATOM | 5650 | O | LEU | D | 80 | 35.682 | 48.443 | -18.475 | 1.00 | 10.93 | O |
| ATOM | 5651 | CB | LEU | D | 80 | 34.540 | 48.497 | -15.763 | 1.00 | 10.26 | C |
| ATOM | 5652 | CG | LEU | D | 80 | 34.404 | 47.033 | -15.292 | 1.00 | 10.07 | C |
| ATOM | 5653 | CD1 | LEU | D | 80 | 35.109 | 46.802 | -13.963 | 1.00 | 11.59 | C |
| ATOM | 5654 | CD2 | LEU | D | 80 | 32.943 | 46.656 | -15.191 | 1.00 | 10.94 | C |
| ATOM | 5655 | N | GLN | D | 81 | 37.509 | 47.625 | -17.411 | 1.00 | 12.84 | N |
| ATOM | 5656 | CA | GLN | D | 81 | 38.002 | 46.802 | -18.519 | 1.00 | 12.32 | C |
| ATOM | 5657 | C | GLN | D | 81 | 37.438 | 45.386 | -18.318 | 1.00 | 13.23 | C |
| ATOM | 5658 | O | GLN | D | 81 | 37.567 | 44.807 | -17.248 | 1.00 | 11.86 | O |
| ATOM | 5659 | CB | GLN | D | 81 | 39.530 | 46.796 | -18.511 | 1.00 | 14.80 | C |
| ATOM | 5660 | CG | GLN | D | 81 | 40.190 | 45.928 | -19.600 | 1.00 | 15.51 | C |
| ATOM | 5661 | CD | GLN | D | 81 | 39.872 | 46.412 | -20.983 | 1.00 | 17.60 | C |
| ATOM | 5662 | OE1 | GLN | D | 81 | 40.028 | 47.600 | -21.281 | 1.00 | 20.58 | O |
| ATOM | 5663 | NE2 | GLN | D | 81 | 39.417 | 45.502 | -21.858 | 1.00 | 17.77 | N |
| ATOM | 5664 | N | MET | D | 82 | 36.786 | 44.865 | -19.341 | 1.00 | 12.34 | N |
| ATOM | 5665 | CA | MET | D | 82 | 36.023 | 43.646 | -19.212 | 1.00 | 14.08 | C |
| ATOM | 5666 | C | MET | D | 82 | 36.582 | 42.710 | -20.260 | 1.00 | 12.86 | C |
| ATOM | 5667 | O | MET | D | 82 | 36.392 | 42.963 | -21.442 | 1.00 | 14.59 | O |
| ATOM | 5668 | CB | MET | D | 82 | 34.539 | 43.972 | -19.472 | 1.00 | 12.88 | C |
| ATOM | 5669 | CG | MET | D | 82 | 33.941 | 45.033 | -18.513 | 1.00 | 16.91 | C |
| ATOM | 5670 | SD | MET | D | 82 | 32.349 | 45.749 | -19.061 | 1.00 | 19.29 | S |
| ATOM | 5671 | CE | MET | D | 82 | 31.452 | 44.243 | -18.794 | 1.00 | 18.61 | C |
| ATOM | 5672 | N | ASN | D | 82A | 37.282 | 41.645 | -19.850 | 1.00 | 13.56 | N |
| ATOM | 5673 | CA | ASN | D | 82A | 37.920 | 40.737 | -20.795 | 1.00 | 12.64 | C |
| ATOM | 5674 | C | ASN | D | 82A | 37.297 | 39.336 | -20.740 | 1.00 | 10.81 | C |
| ATOM | 5675 | O | ASN | D | 82A | 36.706 | 38.942 | -19.732 | 1.00 | 11.10 | O |
| ATOM | 5676 | CB | ASN | D | 82A | 39.398 | 40.612 | -20.505 | 1.00 | 15.37 | C |
| ATOM | 5677 | CG | ASN | D | 82A | 40.147 | 41.892 | -20.706 | 1.00 | 16.85 | C |
| ATOM | 5678 | OD1 | ASN | D | 82A | 39.862 | 42.686 | -21.619 | 1.00 | 18.40 | O |
| ATOM | 5679 | ND2 | ASN | D | 82A | 41.132 | 42.102 | -19.864 | 1.00 | 22.21 | N |
| ATOM | 5680 | N | ASN | D | 82B | 37.420 | 38.629 | -21.852 | 1.00 | 10.42 | N |
| ATOM | 5681 | CA | ASN | D | 82B | 36.961 | 37.229 | -21.959 | 1.00 | 9.66 | C |
| ATOM | 5682 | C | ASN | D | 82B | 35.494 | 37.055 | -21.523 | 1.00 | 11.03 | C |
| ATOM | 5683 | O | ASN | D | 82B | 35.127 | 36.221 | -20.654 | 1.00 | 10.81 | O |
| ATOM | 5684 | CB | ASN | D | 82B | 37.899 | 36.330 | -21.201 | 1.00 | 11.56 | C |
| ATOM | 5685 | CG | ASN | D | 82B | 37.821 | 34.875 | -21.694 | 1.00 | 11.75 | C |
| ATOM | 5686 | OD1 | ASN | D | 82B | 37.171 | 34.612 | -22.700 | 1.00 | 12.31 | O |
| ATOM | 5687 | ND2 | ASN | D | 82B | 38.463 | 33.962 | -20.990 | 1.00 | 16.76 | N |
| ATOM | 5688 | N | LEU | D | 82C | 34.644 | 37.890 | -22.104 | 1.00 | 10.62 | N |
| ATOM | 5689 | CA | LEU | D | 82C | 33.266 | 37.997 | -21.660 | 1.00 | 10.85 | C |
| ATOM | 5690 | C | LEU | D | 82C | 32.401 | 36.789 | -21.958 | 1.00 | 10.93 | C |
| ATOM | 5691 | O | LEU | D | 82C | 32.621 | 36.012 | -22.918 | 1.00 | 10.53 | O |
| ATOM | 5692 | CB | LEU | D | 82C | 32.606 | 39.253 | -22.247 | 1.00 | 10.35 | C |
| ATOM | 5693 | CG | LEU | D | 82C | 33.119 | 40.540 | -21.572 | 1.00 | 11.61 | C |
| ATOM | 5694 | CD1 | LEU | D | 82C | 32.840 | 41.728 | -22.437 | 1.00 | 10.93 | C |
| ATOM | 5695 | CD2 | LEU | D | 82C | 32.478 | 40.739 | -20.185 | 1.00 | 14.77 | C |
| ATOM | 5696 | N | ARG | D | 83 | 31.431 | 36.637 | -21.077 | 1.00 | 10.97 | N |
| ATOM | 5697 | CA | ARG | D | 83 | 30.433 | 35.595 | -21.155 | 1.00 | 12.32 | C |
| ATOM | 5698 | C | ARG | D | 83 | 29.048 | 36.135 | -21.369 | 1.00 | 13.13 | C |
| ATOM | 5699 | O | ARG | D | 83 | 28.708 | 37.300 | -21.066 | 1.00 | 11.28 | O |
| ATOM | 5700 | CB | ARG | D | 83 | 30.434 | 34.789 | -19.858 | 1.00 | 13.71 | C |
| ATOM | 5701 | CG | ARG | D | 83 | 31.793 | 34.368 | -19.430 | 1.00 | 13.94 | C |
| ATOM | 5702 | CD | ARG | D | 83 | 31.861 | 34.107 | -17.946 | 1.00 | 17.12 | C |
| ATOM | 5703 | NE | ARG | D | 83 | 31.853 | 35.361 | -17.180 | 1.00 | 18.33 | N |
| ATOM | 5704 | CZ | ARG | D | 83 | 32.130 | 35.465 | -15.882 | 1.00 | 20.85 | C |
| ATOM | 5705 | NH1 | ARG | D | 83 | 32.499 | 34.404 | -15.153 | 1.00 | 21.42 | N |
| ATOM | 5706 | NH2 | ARG | D | 83 | 32.052 | 36.653 | -15.310 | 1.00 | 21.78 | N |
| ATOM | 5707 | N | THR | D | 84 | 28.182 | 35.257 | -21.840 | 1.00 | 14.37 | N |
| ATOM | 5708 | CA | THR | D | 84 | 26.803 | 35.621 | -21.993 | 1.00 | 16.88 | C |

| ATOM | 5709 | C | THR | D | 84 | 26.197 | 36.181 | -20.709 | 1.00 | 17.31 | C |
|------|------|------|-----|---|----|--------|--------|---------|------|-------|---|
| ATOM | 5710 | O | THR | D | 84 | 25.471 | 37.198 | -20.767 | 1.00 | 19.06 | O |
| ATOM | 5711 | CB | THR | D | 84 | 26.009 | 34.426 | -22.570 | 1.00 | 17.84 | C |
| ATOM | 5712 | OG1 | THR | D | 84 | 26.533 | 34.150 | -23.890 | 1.00 | 21.81 | O |
| ATOM | 5713 | CG2 | THR | D | 84 | 24.541 | 34.763 | -22.629 | 1.00 | 18.27 | C |
| ATOM | 5714 | N | GLU | D | 85 | 26.578 | 35.597 | -19.571 | 1.00 | 16.98 | N |
| ATOM | 5715 | CA | GLU | D | 85 | 26.136 | 35.999 | -18.235 | 1.00 | 18.45 | C |
| ATOM | 5716 | C | GLU | D | 85 | 26.587 | 37.396 | -17.809 | 1.00 | 15.92 | C |
| ATOM | 5717 | O | GLU | D | 85 | 26.146 | 37.900 | -16.797 | 1.00 | 17.58 | O |
| ATOM | 5718 | CB | GLU | D | 85 | 26.617 | 34.988 | -17.172 | 1.00 | 19.52 | C |
| ATOM | 5719 | CG | GLU | D | 85 | 28.053 | 35.166 | -16.665 | 1.00 | 21.23 | C |
| ATOM | 5720 | CD | GLU | D | 85 | 28.639 | 33.932 | -15.968 | 1.00 | 23.24 | C |
| ATOM | 5721 | OE1 | GLU | D | 85 | 28.694 | 32.817 | -16.576 | 1.00 | 27.18 | O |
| ATOM | 5722 | OE2 | GLU | D | 85 | 29.086 | 34.081 | -14.816 | 1.00 | 26.33 | O |
| ATOM | 5723 | N | ASP | D | 86 | 27.496 | 37.993 | -18.565 | 1.00 | 14.48 | N |
| ATOM | 5724 | CA | ASP | D | 86 | 27.976 | 39.351 | -18.271 | 1.00 | 12.29 | C |
| ATOM | 5725 | C | ASP | D | 86 | 27.133 | 40.408 | -18.993 | 1.00 | 11.76 | C |
| ATOM | 5726 | O | ASP | D | 86 | 27.360 | 41.624 | -18.852 | 1.00 | 12.14 | O |
| ATOM | 5727 | CB | ASP | D | 86 | 29.432 | 39.469 | -18.683 | 1.00 | 12.54 | C |
| ATOM | 5728 | CG | ASP | D | 86 | 30.340 | 38.584 | -17.883 | 1.00 | 11.07 | C |
| ATOM | 5729 | OD1 | ASP | D | 86 | 30.248 | 38.600 | -16.635 | 1.00 | 11.35 | O |
| ATOM | 5730 | OD2 | ASP | D | 86 | 31.208 | 37.908 | -18.479 | 1.00 | 15.25 | O |
| ATOM | 5731 | N | THR | D | 87 | 26.150 | 39.962 | -19.765 | 1.00 | 10.42 | N |
| ATOM | 5732 | CA | THR | D | 87 | 25.233 | 40.876 | -20.429 | 1.00 | 10.24 | C |
| ATOM | 5733 | C | THR | D | 87 | 24.416 | 41.597 | -19.312 | 1.00 | 9.41 | C |
| ATOM | 5734 | O | THR | D | 87 | 23.983 | 40.993 | -18.348 | 1.00 | 9.26 | O |
| ATOM | 5735 | CB | THR | D | 87 | 24.279 | 40.113 | -21.359 | 1.00 | 10.24 | C |
| ATOM | 5736 | OG1 | THR | D | 87 | 25.019 | 39.570 | -22.463 | 1.00 | 9.39 | O |
| ATOM | 5737 | CG2 | THR | D | 87 | 23.151 | 41.009 | -21.840 | 1.00 | 13.14 | C |
| ATOM | 5738 | N | GLY | D | 88 | 24.224 | 42.896 | -19.447 | 1.00 | 9.35 | N |
| ATOM | 5739 | CA | GLY | D | 88 | 23.447 | 43.606 | -18.505 | 1.00 | 8.19 | C |
| ATOM | 5740 | C | GLY | D | 88 | 23.612 | 45.113 | -18.630 | 1.00 | 9.65 | C |
| ATOM | 5741 | O | GLY | D | 88 | 24.379 | 45.601 | -19.442 | 1.00 | 8.80 | O |
| ATOM | 5742 | N | ILE | D | 89 | 22.908 | 45.818 | -17.772 | 1.00 | 10.69 | N |
| ATOM | 5743 | CA | ILE | D | 89 | 23.095 | 47.256 | -17.641 | 1.00 | 10.22 | C |
| ATOM | 5744 | C | ILE | D | 89 | 24.114 | 47.487 | -16.543 | 1.00 | 8.66 | C |
| ATOM | 5745 | O | ILE | D | 89 | 23.922 | 47.024 | -15.435 | 1.00 | 11.33 | O |
| ATOM | 5746 | CB | ILE | D | 89 | 21.787 | 47.982 | -17.231 | 1.00 | 11.14 | C |
| ATOM | 5747 | CG1 | ILE | D | 89 | 20.652 | 47.738 | -18.223 | 1.00 | 14.31 | C |
| ATOM | 5748 | CG2 | ILE | D | 89 | 22.061 | 49.454 | -17.045 | 1.00 | 12.15 | C |
| ATOM | 5749 | CD1 | ILE | D | 89 | 20.964 | 48.059 | -19.597 | 1.00 | 17.87 | C |
| ATOM | 5750 | N | TYR | D | 90 | 25.186 | 48.227 | -16.845 | 1.00 | 9.44 | N |
| ATOM | 5751 | CA | TYR | D | 90 | 26.269 | 48.508 | -15.918 | 1.00 | 7.91 | C |
| ATOM | 5752 | C | TYR | D | 90 | 26.150 | 49.947 | -15.399 | 1.00 | 9.52 | C |
| ATOM | 5753 | O | TYR | D | 90 | 26.155 | 50.875 | -16.198 | 1.00 | 9.39 | O |
| ATOM | 5754 | CB | TYR | D | 90 | 27.633 | 48.319 | -16.609 | 1.00 | 8.94 | C |
| ATOM | 5755 | CG | TYR | D | 90 | 27.981 | 46.838 | -16.730 | 1.00 | 8.11 | C |
| ATOM | 5756 | CD1 | TYR | D | 90 | 27.320 | 46.036 | -17.663 | 1.00 | 10.08 | C |
| ATOM | 5757 | CD2 | TYR | D | 90 | 28.896 | 46.255 | -15.883 | 1.00 | 9.42 | C |
| ATOM | 5758 | CE1 | TYR | D | 90 | 27.601 | 44.648 | -17.771 | 1.00 | 9.49 | C |
| ATOM | 5759 | CE2 | TYR | D | 90 | 29.193 | 44.876 | -15.995 | 1.00 | 10.52 | C |
| ATOM | 5760 | CZ | TYR | D | 90 | 28.532 | 44.095 | -16.935 | 1.00 | 9.82 | C |
| ATOM | 5761 | OH | TYR | D | 90 | 28.816 | 42.721 | -17.036 | 1.00 | 12.06 | O |
| ATOM | 5762 | N | TYR | D | 91 | 25.979 | 50.066 | -14.097 | 1.00 | 10.78 | N |
| ATOM | 5763 | CA | TYR | D | 91 | 25.857 | 51.340 | -13.373 | 1.00 | 11.50 | C |
| ATOM | 5764 | C | TYR | D | 91 | 27.119 | 51.628 | -12.610 | 1.00 | 12.74 | C |
| ATOM | 5765 | O | TYR | D | 91 | 27.694 | 50.790 | -11.910 | 1.00 | 11.81 | O |
| ATOM | 5766 | CB | TYR | D | 91 | 24.723 | 51.307 | -12.330 | 1.00 | 12.77 | C |
| ATOM | 5767 | CG | TYR | D | 91 | 23.314 | 51.130 | -12.858 | 1.00 | 13.26 | C |
| ATOM | 5768 | CD1 | TYR | D | 91 | 22.571 | 52.212 | -13.320 | 1.00 | 13.48 | C |
| ATOM | 5769 | CD2 | TYR | D | 91 | 22.707 | 49.886 | -12.863 | 1.00 | 13.81 | C |
| ATOM | 5770 | CE1 | TYR | D | 91 | 21.285 | 52.051 | -13.789 | 1.00 | 12.60 | C |
| ATOM | 5771 | CE2 | TYR | D | 91 | 21.434 | 49.720 | -13.351 | 1.00 | 14.64 | C |
| ATOM | 5772 | CZ | TYR | D | 91 | 20.710 | 50.811 | -13.796 | 1.00 | 14.89 | C |
| ATOM | 5773 | OH | TYR | D | 91 | 19.422 | 50.633 | -14.262 | 1.00 | 16.87 | O |

| ATOM | 5774 | N | CYS | D | 92 | 27.517 | 52.884 | -12.671 | 1.00 | 12.02 | N |
|------|------|-----|-----|---|-----|--------|--------|---------|------|-------|---|
| ATOM | 5775 | CA | CYS | D | 92 | 28.437 | 53.413 | -11.731 | 1.00 | 14.00 | C |
| ATOM | 5776 | C | CYS | D | 92 | 27.643 | 53.690 | -10.438 | 1.00 | 14.11 | C |
| ATOM | 5777 | O | CYS | D | 92 | 26.575 | 54.314 | -10.459 | 1.00 | 14.06 | O |
| ATOM | 5778 | CB | CYS | D | 92 | 29.051 | 54.678 | -12.357 | 1.00 | 15.05 | C |
| ATOM | 5779 | SG | CYS | D | 92 | 30.546 | 54.356 | -13.299 | 1.00 | 24.14 | S |
| ATOM | 5780 | N | PHE | D | 93 | 28.138 | 53.156 | -9.335 | 1.00 | 13.10 | N |
| ATOM | 5781 | CA | PHE | D | 93 | 27.529 | 53.282 | -8.003 | 1.00 | 11.35 | C |
| ATOM | 5782 | C | PHE | D | 93 | 28.575 | 53.778 | -7.011 | 1.00 | 11.74 | C |
| ATOM | 5783 | O | PHE | D | 93 | 29.581 | 53.109 | -6.717 | 1.00 | 10.04 | O |
| ATOM | 5784 | CB | PHE | D | 93 | 26.983 | 51.921 | -7.562 | 1.00 | 11.68 | C |
| ATOM | 5785 | CG | PHE | D | 93 | 26.440 | 51.879 | -6.147 | 1.00 | 10.42 | C |
| ATOM | 5786 | CD1 | PHE | D | 93 | 25.453 | 52.744 | -5.737 | 1.00 | 9.49 | C |
| ATOM | 5787 | CD2 | PHE | D | 93 | 26.877 | 50.925 | -5.249 | 1.00 | 11.81 | C |
| ATOM | 5788 | CE1 | PHE | D | 93 | 24.922 | 52.667 | -4.438 | 1.00 | 9.57 | C |
| ATOM | 5789 | CE2 | PHE | D | 93 | 26.365 | 50.856 | -3.969 | 1.00 | 12.83 | C |
| ATOM | 5790 | CZ | PHE | D | 93 | 25.390 | 51.739 | -3.562 | 1.00 | 9.89 | C |
| ATOM | 5791 | N | LEU | D | 94 | 28.355 | 54.986 | -6.499 | 1.00 | 10.69 | N |
| ATOM | 5792 | CA | LEU | D | 94 | 29.179 | 55.546 | -5.452 | 1.00 | 10.62 | C |
| ATOM | 5793 | C | LEU | D | 94 | 28.593 | 55.124 | -4.113 | 1.00 | 9.37 | C |
| ATOM | 5794 | O | LEU | D | 94 | 27.543 | 55.635 | -3.694 | 1.00 | 9.70 | O |
| ATOM | 5795 | CB | LEU | D | 94 | 29.136 | 57.092 | -5.531 | 1.00 | 9.85 | C |
| ATOM | 5796 | CG | LEU | D | 94 | 29.961 | 57.743 | -6.629 | 1.00 | 13.68 | C |
| ATOM | 5797 | CD1 | LEU | D | 94 | 29.474 | 57.376 | -8.018 | 1.00 | 14.97 | C |
| ATOM | 5798 | CD2 | LEU | D | 94 | 29.849 | 59.280 | -6.456 | 1.00 | 13.96 | C |
| ATOM | 5799 | N | PRO | D | 95 | 29.194 | 54.150 | -3.454 | 1.00 | 9.68 | N |
| ATOM | 5800 | CA | PRO | D | 95 | 28.525 | 53.645 | -2.260 | 1.00 | 11.03 | C |
| ATOM | 5801 | C | PRO | D | 95 | 28.459 | 54.726 | -1.180 | 1.00 | 12.69 | C |
| ATOM | 5802 | O | PRO | D | 95 | 29.456 | 55.372 | -0.966 | 1.00 | 12.34 | O |
| ATOM | 5803 | CB | PRO | D | 95 | 29.467 | 52.526 | -1.792 | 1.00 | 12.24 | C |
| ATOM | 5804 | CG | PRO | D | 95 | 30.222 | 52.140 | -2.963 | 1.00 | 11.59 | C |
| ATOM | 5805 | CD | PRO | D | 95 | 30.421 | 53.389 | -3.750 | 1.00 | 11.71 | C |
| ATOM | 5806 | N | MET | D | 96 | 27.325 | 54.928 | -0.517 | 1.00 | 11.37 | N |
| ATOM | 5807 | CA | MET | D | 96 | 26.059 | 54.253 | -0.768 | 1.00 | 13.40 | C |
| ATOM | 5808 | C | MET | D | 96 | 24.992 | 55.130 | -1.467 | 1.00 | 14.73 | C |
| ATOM | 5809 | O | MET | D | 96 | 23.878 | 54.676 | -1.688 | 1.00 | 13.85 | O |
| ATOM | 5810 | CB | MET | D | 96 | 25.519 | 53.750 | 0.579 | 1.00 | 13.98 | C |
| ATOM | 5811 | CG | MET | D | 96 | 26.423 | 52.771 | 1.281 | 1.00 | 15.00 | C |
| ATOM | 5812 | SD | MET | D | 96 | 26.501 | 51.174 | 0.439 | 1.00 | 14.60 | S |
| ATOM | 5813 | CE | MET | D | 96 | 24.878 | 50.473 | 0.693 | 1.00 | 16.30 | C |
| ATOM | 5814 | N | ASP | D | 101 | 25.331 | 56.378 | -1.825 | 1.00 | 15.33 | N |
| ATOM | 5815 | CA | ASP | D | 101 | 24.315 | 57.407 | -2.089 | 1.00 | 16.22 | C |
| ATOM | 5816 | C | ASP | D | 101 | 23.941 | 57.690 | -3.546 | 1.00 | 15.78 | C |
| ATOM | 5817 | O | ASP | D | 101 | 22.854 | 58.216 | -3.771 | 1.00 | 17.03 | O |
| ATOM | 5818 | CB | ASP | D | 101 | 24.759 | 58.719 | -1.407 | 1.00 | 17.79 | C |
| ATOM | 5819 | CG | ASP | D | 101 | 24.953 | 58.545 | 0.095 | 1.00 | 19.18 | C |
| ATOM | 5820 | OD1 | ASP | D | 101 | 24.241 | 57.714 | 0.699 | 1.00 | 25.72 | O |
| ATOM | 5821 | OD2 | ASP | D | 101 | 25.851 | 59.165 | 0.678 | 1.00 | 26.26 | O |
| ATOM | 5822 | N | TYR | D | 102 | 24.799 | 57.346 | -4.518 | 1.00 | 15.93 | N |
| ATOM | 5823 | CA | TYR | D | 102 | 24.617 | 57.780 | -5.918 | 1.00 | 15.17 | C |
| ATOM | 5824 | C | TYR | D | 102 | 24.745 | 56.663 | -6.957 | 1.00 | 13.40 | C |
| ATOM | 5825 | O | TYR | D | 102 | 25.675 | 55.894 | -6.888 | 1.00 | 15.18 | O |
| ATOM | 5826 | CB | TYR | D | 102 | 25.629 | 58.893 | -6.265 | 1.00 | 16.60 | C |
| ATOM | 5827 | CG | TYR | D | 102 | 25.461 | 60.082 | -5.362 | 1.00 | 17.42 | C |
| ATOM | 5828 | CD1 | TYR | D | 102 | 24.404 | 60.949 | -5.542 | 1.00 | 20.66 | C |
| ATOM | 5829 | CD2 | TYR | D | 102 | 26.316 | 60.298 | -4.292 | 1.00 | 19.24 | C |
| ATOM | 5830 | CE1 | TYR | D | 102 | 24.209 | 62.018 | -4.708 | 1.00 | 18.52 | C |
| ATOM | 5831 | CE2 | TYR | D | 102 | 26.124 | 61.386 | -3.426 | 1.00 | 18.86 | C |
| ATOM | 5832 | CZ | TYR | D | 102 | 25.060 | 62.225 | -3.647 | 1.00 | 19.92 | C |
| ATOM | 5833 | OH | TYR | D | 102 | 24.827 | 63.313 | -2.812 | 1.00 | 22.83 | O |
| ATOM | 5834 | N | TRP | D | 103 | 23.813 | 56.646 | -7.908 | 1.00 | 12.15 | N |
| ATOM | 5835 | CA | TRP | D | 103 | 23.817 | 55.787 | -9.094 | 1.00 | 13.12 | C |
| ATOM | 5836 | C | TRP | D | 103 | 23.886 | 56.622 | -10.362 | 1.00 | 13.94 | C |
| ATOM | 5837 | O | TRP | D | 103 | 23.282 | 57.711 | -10.468 | 1.00 | 15.99 | O |
| ATOM | 5838 | CB | TRP | D | 103 | 22.512 | 54.969 | -9.155 | 1.00 | 12.61 | C |

| | | | | | | | | | | |
|---|---|---|---|---|---|---|---|---|---|---|
| ATOM | 5839 | CG | TRP | D | 103 | 22.321 | 54.025 | -8.044 | 1.00 12.65 | C |
| ATOM | 5840 | CD1 | TRP | D | 103 | 21.922 | 54.301 | -6.771 | 1.00 12.57 | C |
| ATOM | 5841 | CD2 | TRP | D | 103 | 22.517 | 52.604 | -8.108 | 1.00 11.58 | C |
| ATOM | 5842 | NE1 | TRP | D | 103 | 21.840 | 53.137 | -6.044 | 1.00 12.92 | N |
| ATOM | 5843 | CE2 | TRP | D | 103 | 22.203 | 52.085 | -6.847 | 1.00 12.10 | C |
| ATOM | 5844 | CE3 | TRP | D | 103 | 22.896 | 51.727 | -9.129 | 1.00 13.45 | C |
| ATOM | 5845 | CZ2 | TRP | D | 103 | 22.293 | 50.718 | -6.553 | 1.00 11.89 | C |
| ATOM | 5846 | CZ3 | TRP | D | 103 | 22.966 | 50.366 | -8.849 | 1.00 12.86 | C |
| ATOM | 5847 | CH2 | TRP | D | 103 | 22.666 | 49.877 | -7.566 | 1.00 12.22 | C |
| ATOM | 5848 | N | GLY | D | 104 | 24.573 | 56.099 | -11.357 | 1.00 14.36 | N |
| ATOM | 5849 | CA | GLY | D | 104 | 24.507 | 56.648 | -12.701 | 1.00 14.09 | C |
| ATOM | 5850 | C | GLY | D | 104 | 23.289 | 56.243 | -13.498 | 1.00 13.00 | C |
| ATOM | 5851 | O | GLY | D | 104 | 22.284 | 55.789 | -12.957 | 1.00 13.36 | O |
| ATOM | 5852 | N | GLN | D | 105 | 23.346 | 56.443 | -14.803 | 1.00 13.98 | N |
| ATOM | 5853 | CA | GLN | D | 105 | 22.204 | 56.216 | -15.657 | 1.00 15.95 | C |
| ATOM | 5854 | C | GLN | D | 105 | 22.236 | 54.833 | -16.296 | 1.00 13.86 | C |
| ATOM | 5855 | O | GLN | D | 105 | 21.228 | 54.355 | -16.808 | 1.00 14.60 | O |
| ATOM | 5856 | CB | GLN | D | 105 | 22.124 | 57.331 | -16.690 | 1.00 19.84 | C |
| ATOM | 5857 | CG | GLN | D | 105 | 21.737 | 58.685 | -15.987 | 1.00 24.54 | C |
| ATOM | 5858 | CD | GLN | D | 105 | 20.288 | 58.687 | -15.417 | 1.00 27.90 | C |
| ATOM | 5859 | OE1 | GLN | D | 105 | 20.075 | 58.668 | -14.192 | 1.00 31.86 | O |
| ATOM | 5860 | NE2 | GLN | D | 105 | 19.293 | 58.687 | -16.315 | 1.00 31.25 | N |
| ATOM | 5861 | N | GLY | D | 106 | 23.387 | 54.190 | -16.178 | 1.00 12.85 | N |
| ATOM | 5862 | CA | GLY | D | 106 | 23.670 | 52.883 | -16.778 | 1.00 12.30 | C |
| ATOM | 5863 | C | GLY | D | 106 | 24.105 | 52.918 | -18.226 | 1.00 12.65 | C |
| ATOM | 5864 | O | GLY | D | 106 | 23.698 | 53.787 | -18.988 | 1.00 13.76 | O |
| ATOM | 5865 | N | THR | D | 107 | 24.896 | 51.944 | -18.630 | 1.00 10.83 | N |
| ATOM | 5866 | CA | THR | D | 107 | 25.233 | 51.744 | -20.021 | 1.00 10.96 | C |
| ATOM | 5867 | C | THR | D | 107 | 25.052 | 50.236 | -20.239 | 1.00 12.12 | C |
| ATOM | 5868 | O | THR | D | 107 | 25.454 | 49.446 | -19.389 | 1.00 12.39 | O |
| ATOM | 5869 | CB | THR | D | 107 | 26.654 | 52.265 | -20.393 | 1.00 12.29 | C |
| ATOM | 5870 | OG1 | THR | D | 107 | 26.849 | 52.190 | -21.814 | 1.00 11.65 | O |
| ATOM | 5871 | CG2 | THR | D | 107 | 27.812 | 51.485 | -19.667 | 1.00 11.86 | C |
| ATOM | 5872 | N | SER | D | 108 | 24.383 | 49.882 | -21.339 | 1.00 10.87 | N |
| ATOM | 5873 | CA | SER | D | 108 | 24.082 | 48.495 | -21.679 | 1.00 11.58 | C |
| ATOM | 5874 | C | SER | D | 108 | 25.224 | 47.808 | -22.420 | 1.00 11.24 | C |
| ATOM | 5875 | O | SER | D | 108 | 25.785 | 48.341 | -23.393 | 1.00 10.34 | O |
| ATOM | 5876 | CB | SER | D | 108 | 22.832 | 48.416 | -22.533 | 1.00 13.12 | C |
| ATOM | 5877 | OG | SER | D | 108 | 22.475 | 47.052 | -22.735 | 1.00 16.71 | O |
| ATOM | 5878 | N | VAL | D | 109 | 25.525 | 46.587 | -21.962 | 1.00 10.45 | N |
| ATOM | 5879 | CA | VAL | D | 109 | 26.539 | 45.731 | -22.542 | 1.00 10.54 | C |
| ATOM | 5880 | C | VAL | D | 109 | 25.858 | 44.408 | -22.903 | 1.00 11.29 | C |
| ATOM | 5881 | O | VAL | D | 109 | 25.235 | 43.778 | -22.055 | 1.00 9.07 | O |
| ATOM | 5882 | CB | VAL | D | 109 | 27.684 | 45.459 | -21.532 | 1.00 10.97 | C |
| ATOM | 5883 | CG1 | VAL | D | 109 | 28.624 | 44.410 | -22.061 | 1.00 10.64 | C |
| ATOM | 5884 | CG2 | VAL | D | 109 | 28.463 | 46.741 | -21.251 | 1.00 11.46 | C |
| ATOM | 5885 | N | THR | D | 110 | 25.936 | 44.044 | -24.176 | 1.00 11.20 | N |
| ATOM | 5886 | CA | THR | D | 110 | 25.428 | 42.774 | -24.667 | 1.00 11.10 | C |
| ATOM | 5887 | C | THR | D | 110 | 26.569 | 41.886 | -25.130 | 1.00 10.33 | C |
| ATOM | 5888 | O | THR | D | 110 | 27.386 | 42.274 | -25.951 | 1.00 9.18 | O |
| ATOM | 5889 | CB | THR | D | 110 | 24.405 | 43.010 | -25.801 | 1.00 12.89 | C |
| ATOM | 5890 | OG1 | THR | D | 110 | 23.277 | 43.732 | -25.284 | 1.00 15.05 | O |
| ATOM | 5891 | CG2 | THR | D | 110 | 23.918 | 41.687 | -26.371 | 1.00 13.07 | C |
| ATOM | 5892 | N | VAL | D | 111 | 26.620 | 40.663 | -24.591 | 1.00 9.10 | N |
| ATOM | 5893 | CA | VAL | D | 111 | 27.566 | 39.662 | -25.052 | 1.00 9.50 | C |
| ATOM | 5894 | C | VAL | D | 111 | 26.770 | 38.591 | -25.792 | 1.00 11.30 | C |
| ATOM | 5895 | O | VAL | D | 111 | 25.903 | 37.902 | -25.200 | 1.00 12.31 | O |
| ATOM | 5896 | CB | VAL | D | 111 | 28.348 | 39.039 | -23.870 | 1.00 9.48 | C |
| ATOM | 5897 | CG1 | VAL | D | 111 | 29.386 | 38.129 | -24.379 | 1.00 10.60 | C |
| ATOM | 5898 | CG2 | VAL | D | 111 | 28.960 | 40.175 | -23.009 | 1.00 10.94 | C |
| ATOM | 5899 | N | SER | D | 112 | 27.024 | 38.513 | -27.088 | 1.00 10.28 | N |
| ATOM | 5900 | CA | SER | D | 112 | 26.171 | 37.776 | -28.020 | 1.00 11.36 | C |
| ATOM | 5901 | C | SER | D | 112 | 26.868 | 37.464 | -29.318 | 1.00 13.23 | C |
| ATOM | 5902 | O | SER | D | 112 | 27.661 | 38.245 | -29.814 | 1.00 10.53 | O |
| ATOM | 5903 | CB | SER | D | 112 | 24.901 | 38.549 | -28.339 | 1.00 12.52 | C |

| ATOM | 5904 | OG | SER D 112 | 24.096 | 37.857 | -29.285 | 1.00 | 12.85 | O |
| ATOM | 5905 | N | SER D 113 | 26.558 | 36.296 | -29.881 | 1.00 | 13.59 | N |
| ATOM | 5906 | CA | SER D 113 | 27.049 | 35.974 | -31.193 | 1.00 | 14.99 | C |
| ATOM | 5907 | C | SER D 113 | 26.089 | 36.451 | -32.304 | 1.00 | 15.65 | C |
| ATOM | 5908 | O | SER D 113 | 26.419 | 36.310 | -33.499 | 1.00 | 16.19 | O |
| ATOM | 5909 | CB | SER D 113 | 27.262 | 34.462 | -31.298 | 1.00 | 16.59 | C |
| ATOM | 5910 | OG | SER D 113 | 26.023 | 33.812 | -31.061 | 1.00 | 17.19 | O |
| ATOM | 5911 | N | ALA D 114 | 24.924 | 36.988 | -31.925 | 1.00 | 13.31 | N |
| ATOM | 5912 | CA | ALA D 114 | 23.989 | 37.556 | -32.873 | 1.00 | 15.22 | C |
| ATOM | 5913 | C | ALA D 114 | 24.529 | 38.902 | -33.360 | 1.00 | 16.37 | C |
| ATOM | 5914 | O | ALA D 114 | 25.391 | 39.507 | -32.690 | 1.00 | 17.88 | O |
| ATOM | 5915 | CB | ALA D 114 | 22.581 | 37.692 | -32.236 | 1.00 | 16.42 | C |
| ATOM | 5916 | N | LYS D 115 | 24.073 | 39.342 | -34.524 | 1.00 | 16.06 | N |
| ATOM | 5917 | CA | LYS D 115 | 24.576 | 40.576 | -35.123 | 1.00 | 17.42 | C |
| ATOM | 5918 | C | LYS D 115 | 23.711 | 41.767 | -34.749 | 1.00 | 17.01 | C |
| ATOM | 5919 | O | LYS D 115 | 22.502 | 41.675 | -34.670 | 1.00 | 16.72 | O |
| ATOM | 5920 | CB ALYS D 115 | | 24.703 | 40.453 | -36.648 | 0.50 | 19.18 | C |
| ATOM | 5921 | CB BLYS D 115 | | 24.650 | 40.451 | -36.660 | 0.50 | 18.25 | C |
| ATOM | 5922 | CG ALYS D 115 | | 25.842 | 39.518 | -37.105 | 0.50 | 20.46 | C |
| ATOM | 5923 | CG BLYS D 115 | | 23.307 | 40.501 | -37.414 | 0.50 | 17.82 | C |
| ATOM | 5924 | CD ALYS D 115 | | 25.325 | 38.104 | -37.283 | 0.50 | 23.07 | C |
| ATOM | 5925 | CD BLYS D 115 | | 23.492 | 40.641 | -38.929 | 0.50 | 18.16 | C |
| ATOM | 5926 | CE ALYS D 115 | | 26.385 | 37.052 | -37.118 | 0.50 | 23.51 | C |
| ATOM | 5927 | CE BLYS D 115 | | 22.158 | 40.829 | -39.645 | 0.50 | 19.25 | C |
| ATOM | 5928 | NZ ALYS D 115 | | 25.782 | 35.853 | -36.453 | 0.50 | 24.01 | N |
| ATOM | 5929 | NZ BLYS D 115 | | 21.652 | 42.266 | -39.742 | 0.50 | 17.11 | N |
| ATOM | 5930 | N | THR D 116 | 24.357 | 42.899 | -34.540 | 1.00 | 14.76 | N |
| ATOM | 5931 | CA | THR D 116 | 23.656 | 44.136 | -34.417 | 1.00 | 13.33 | C |
| ATOM | 5932 | C | THR D 116 | 22.914 | 44.488 | -35.715 | 1.00 | 14.07 | C |
| ATOM | 5933 | O | THR D 116 | 23.477 | 44.496 | -36.841 | 1.00 | 11.99 | O |
| ATOM | 5934 | CB | THR D 116 | 24.612 | 45.259 | -33.987 | 1.00 | 12.58 | C |
| ATOM | 5935 | OG1 | THR D 116 | 25.138 | 44.941 | -32.695 | 1.00 | 13.08 | O |
| ATOM | 5936 | CG2 | THR D 116 | 23.891 | 46.613 | -33.961 | 1.00 | 14.75 | C |
| ATOM | 5937 | N | THR D 117 | 21.644 | 44.832 | -35.539 | 1.00 | 13.50 | N |
| ATOM | 5938 | CA | THR D 117 | 20.785 | 45.276 | -36.644 | 1.00 | 14.07 | C |
| ATOM | 5939 | C | THR D 117 | 20.031 | 46.500 | -36.138 | 1.00 | 13.37 | C |
| ATOM | 5940 | O | THR D 117 | 19.440 | 46.443 | -35.060 | 1.00 | 12.79 | O |
| ATOM | 5941 | CB | THR D 117 | 19.791 | 44.150 | -37.001 | 1.00 | 13.86 | C |
| ATOM | 5942 | OG1 | THR D 117 | 20.494 | 42.922 | -37.245 | 1.00 | 17.71 | O |
| ATOM | 5943 | CG2 | THR D 117 | 18.954 | 44.474 | -38.219 | 1.00 | 15.99 | C |
| ATOM | 5944 | N | PRO D 118 | 20.013 | 47.597 | -36.908 | 1.00 | 12.58 | N |
| ATOM | 5945 | CA | PRO D 118 | 19.303 | 48.751 | -36.449 | 1.00 | 13.81 | C |
| ATOM | 5946 | C | PRO D 118 | 17.806 | 48.572 | -36.708 | 1.00 | 14.77 | C |
| ATOM | 5947 | O | PRO D 118 | 17.449 | 47.775 | -37.568 | 1.00 | 15.00 | O |
| ATOM | 5948 | CB | PRO D 118 | 19.866 | 49.868 | -37.339 | 1.00 | 13.01 | C |
| ATOM | 5949 | CG | PRO D 118 | 20.123 | 49.185 | -38.620 | 1.00 | 14.28 | C |
| ATOM | 5950 | CD | PRO D 118 | 20.602 | 47.826 | -38.244 | 1.00 | 12.77 | C |
| ATOM | 5951 | N | PRO D 119 | 16.956 | 49.329 | -35.989 | 1.00 | 14.45 | N |
| ATOM | 5952 | CA | PRO D 119 | 15.500 | 49.225 | -36.134 | 1.00 | 15.50 | C |
| ATOM | 5953 | C | PRO D 119 | 14.916 | 49.849 | -37.407 | 1.00 | 16.24 | C |
| ATOM | 5954 | O | PRO D 119 | 15.480 | 50.794 | -37.982 | 1.00 | 16.49 | O |
| ATOM | 5955 | CB | PRO D 119 | 14.962 | 49.976 | -34.913 | 1.00 | 16.03 | C |
| ATOM | 5956 | CG | PRO D 119 | 16.019 | 50.896 | -34.509 | 1.00 | 16.82 | C |
| ATOM | 5957 | CD | PRO D 119 | 17.343 | 50.308 | -34.967 | 1.00 | 15.48 | C |
| ATOM | 5958 | N | SER D 120 | 13.802 | 49.268 | -37.839 | 1.00 | 16.66 | N |
| ATOM | 5959 | CA | SER D 120 | 12.972 | 49.824 | -38.901 | 1.00 | 17.04 | C |
| ATOM | 5960 | C | SER D 120 | 11.800 | 50.414 | -38.139 | 1.00 | 17.26 | C |
| ATOM | 5961 | O | SER D 120 | 11.226 | 49.760 | -37.277 | 1.00 | 18.22 | O |
| ATOM | 5962 | CB | SER D 120 | 12.512 | 48.723 | -39.856 | 1.00 | 16.66 | C |
| ATOM | 5963 | OG | SER D 120 | 13.605 | 48.210 | -40.618 | 1.00 | 19.65 | O |
| ATOM | 5964 | N | VAL D 121 | 11.490 | 51.676 | -38.407 | 1.00 | 18.02 | N |
| ATOM | 5965 | CA | VAL D 121 | 10.520 | 52.419 | -37.622 | 1.00 | 17.57 | C |
| ATOM | 5966 | C | VAL D 121 | 9.332 | 52.750 | -38.539 | 1.00 | 18.89 | C |
| ATOM | 5967 | O | VAL D 121 | 9.494 | 53.387 | -39.577 | 1.00 | 16.92 | O |
| ATOM | 5968 | CB | VAL D 121 | 11.123 | 53.735 | -37.070 | 1.00 | 17.64 | C |

```
ATOM   5969  CG1 VAL D 121    10.122  54.494 -36.225  1.00 17.08           C
ATOM   5970  CG2 VAL D 121    12.375  53.456 -36.239  1.00 17.32           C
ATOM   5971  N   TYR D 122     8.152  52.296 -38.149  1.00 17.85           N
ATOM   5972  CA  TYR D 122     6.971  52.413 -38.988  1.00 17.87           C
ATOM   5973  C   TYR D 122     5.952  53.250 -38.259  1.00 18.73           C
ATOM   5974  O   TYR D 122     5.722  53.041 -37.063  1.00 19.48           O
ATOM   5975  CB  TYR D 122     6.393  51.023 -39.254  1.00 16.35           C
ATOM   5976  CG  TYR D 122     7.341  50.076 -39.957  1.00 15.71           C
ATOM   5977  CD1 TYR D 122     7.918  50.409 -41.160  1.00 15.39           C
ATOM   5978  CD2 TYR D 122     7.639  48.845 -39.406  1.00 16.41           C
ATOM   5979  CE1 TYR D 122     8.775  49.538 -41.821  1.00 17.04           C
ATOM   5980  CE2 TYR D 122     8.492  47.963 -40.047  1.00 18.54           C
ATOM   5981  CZ  TYR D 122     9.069  48.313 -41.254  1.00 16.61           C
ATOM   5982  OH  TYR D 122     9.927  47.419 -41.891  1.00 18.18           O
ATOM   5983  N   PRO D 123     5.289  54.175 -38.977  1.00 20.72           N
ATOM   5984  CA  PRO D 123     4.271  54.995 -38.321  1.00 21.82           C
ATOM   5985  C   PRO D 123     2.994  54.191 -38.070  1.00 22.16           C
ATOM   5986  O   PRO D 123     2.638  53.301 -38.866  1.00 23.44           O
ATOM   5987  CB  PRO D 123     3.996  56.099 -39.345  1.00 21.09           C
ATOM   5988  CG  PRO D 123     4.233  55.443 -40.662  1.00 20.35           C
ATOM   5989  CD  PRO D 123     5.368  54.439 -40.423  1.00 20.75           C
ATOM   5990  N   LEU D 124     2.322  54.511 -36.968  1.00 22.91           N
ATOM   5991  CA  LEU D 124     1.007  53.976 -36.658  1.00 23.60           C
ATOM   5992  C   LEU D 124    -0.002  55.126 -36.670  1.00 24.26           C
ATOM   5993  O   LEU D 124    -0.082  55.920 -35.736  1.00 21.98           O
ATOM   5994  CB  LEU D 124     0.998  53.276 -35.303  1.00 23.52           C
ATOM   5995  CG  LEU D 124     1.940  52.076 -35.158  1.00 24.57           C
ATOM   5996  CD1 LEU D 124     1.971  51.604 -33.703  1.00 24.71           C
ATOM   5997  CD2 LEU D 124     1.506  50.950 -36.058  1.00 23.09           C
ATOM   5998  N   ALA D 125    -0.752  55.199 -37.767  1.00 26.55           N
ATOM   5999  CA  ALA D 125    -1.740  56.258 -37.970  1.00 27.63           C
ATOM   6000  C   ALA D 125    -3.089  55.600 -38.168  1.00 28.15           C
ATOM   6001  O   ALA D 125    -3.158  54.513 -38.728  1.00 26.62           O
ATOM   6002  CB  ALA D 125    -1.371  57.110 -39.186  1.00 27.66           C
ATOM   6003  N   PRO D 126    -4.165  56.246 -37.697  1.00 29.06           N
ATOM   6004  CA  PRO D 126    -5.507  55.701 -37.874  1.00 30.50           C
ATOM   6005  C   PRO D 126    -5.886  55.462 -39.328  1.00 31.00           C
ATOM   6006  O   PRO D 126    -5.554  56.279 -40.188  1.00 30.59           O
ATOM   6007  CB  PRO D 126    -6.405  56.778 -37.262  1.00 30.62           C
ATOM   6008  CG  PRO D 126    -5.554  57.522 -36.342  1.00 30.33           C
ATOM   6009  CD  PRO D 126    -4.187  57.515 -36.955  1.00 30.10           C
ATOM   6010  N   GLY D 127    -6.559  54.337 -39.591  1.00 31.54           N
ATOM   6011  CA  GLY D 127    -7.089  54.027 -40.926  1.00 32.26           C
ATOM   6012  C   GLY D 127    -8.048  55.094 -41.451  1.00 33.17           C
ATOM   6013  O   GLY D 127    -8.536  55.934 -40.684  1.00 32.91           O
ATOM   6014  N   SER D 136   -12.612  63.051 -29.488  1.00 42.84           N
ATOM   6015  CA  SER D 136   -11.865  64.196 -28.960  1.00 42.89           C
ATOM   6016  C   SER D 136   -10.351  63.935 -28.846  1.00 42.21           C
ATOM   6017  O   SER D 136    -9.542  64.859 -28.937  1.00 41.55           O
ATOM   6018  CB  SER D 136   -12.413  64.575 -27.588  1.00 43.37           C
ATOM   6019  OG  SER D 136   -11.522  65.451 -26.910  1.00 45.68           O
ATOM   6020  N   MET D 137    -9.977  62.681 -28.604  1.00 41.50           N
ATOM   6021  CA  MET D 137    -8.565  62.298 -28.583  1.00 40.61           C
ATOM   6022  C   MET D 137    -8.256  61.416 -29.776  1.00 38.19           C
ATOM   6023  O   MET D 137    -9.139  60.753 -30.326  1.00 37.97           O
ATOM   6024  CB  MET D 137    -8.213  61.564 -27.284  1.00 41.62           C
ATOM   6025  CG  MET D 137    -8.395  62.396 -26.019  1.00 43.62           C
ATOM   6026  SD  MET D 137    -7.523  63.983 -26.027  1.00 47.07           S
ATOM   6027  CE  MET D 137    -5.932  63.576 -25.292  1.00 46.40           C
ATOM   6028  N   VAL D 138    -6.991  61.421 -30.179  1.00 35.43           N
ATOM   6029  CA  VAL D 138    -6.515  60.516 -31.224  1.00 32.58           C
ATOM   6030  C   VAL D 138    -5.256  59.836 -30.698  1.00 30.84           C
ATOM   6031  O   VAL D 138    -4.411  60.477 -30.064  1.00 27.38           O
ATOM   6032  CB  VAL D 138    -6.242  61.239 -32.554  1.00 31.98           C
ATOM   6033  CG1 VAL D 138    -5.301  62.419 -32.366  1.00 32.48           C
```

```
ATOM   6034  CG2 VAL D 138    -5.709  60.272 -33.593  1.00 32.48          C
ATOM   6035  N   THR D 139    -5.169  58.533 -30.928  1.00 29.08          N
ATOM   6036  CA  THR D 139    -4.019  57.757 -30.484  1.00 28.00          C
ATOM   6037  C   THR D 139    -3.201  57.410 -31.716  1.00 27.03          C
ATOM   6038  O   THR D 139    -3.723  56.888 -32.702  1.00 25.71          O
ATOM   6039  CB  THR D 139    -4.434  56.497 -29.692  1.00 28.27          C
ATOM   6040  OG1 THR D 139    -5.082  56.894 -28.473  1.00 27.68          O
ATOM   6041  CG2 THR D 139    -3.205  55.644 -29.317  1.00 28.44          C
ATOM   6042  N   LEU D 140    -1.920  57.755 -31.650  1.00 26.44          N
ATOM   6043  CA  LEU D 140    -0.964  57.505 -32.722  1.00 25.70          C
ATOM   6044  C   LEU D 140     0.138  56.633 -32.137  1.00 24.66          C
ATOM   6045  O   LEU D 140     0.125  56.326 -30.949  1.00 23.69          O
ATOM   6046  CB  LEU D 140    -0.354  58.817 -33.211  1.00 26.32          C
ATOM   6047  CG  LEU D 140    -1.349  59.905 -33.639  1.00 28.18          C
ATOM   6048  CD1 LEU D 140    -0.647  61.207 -33.975  1.00 28.94          C
ATOM   6049  CD2 LEU D 140    -2.161  59.412 -34.800  1.00 27.46          C
ATOM   6050  N   GLY D 141     1.106  56.245 -32.953  1.00 24.31          N
ATOM   6051  CA  GLY D 141     2.137  55.338 -32.436  1.00 24.18          C
ATOM   6052  C   GLY D 141     3.275  55.112 -33.385  1.00 23.87          C
ATOM   6053  O   GLY D 141     3.233  55.582 -34.527  1.00 22.10          O
ATOM   6054  N   CYS D 142     4.302  54.409 -32.889  1.00 23.20          N
ATOM   6055  CA  CYS D 142     5.429  53.959 -33.712  1.00 24.55          C
ATOM   6056  C   CYS D 142     5.657  52.498 -33.459  1.00 21.74          C
ATOM   6057  O   CYS D 142     5.530  52.033 -32.336  1.00 22.60          O
ATOM   6058  CB  CYS D 142     6.703  54.775 -33.422  1.00 27.18          C
ATOM   6059  SG  CYS D 142     6.647  56.355 -34.358  1.00 35.66          S
ATOM   6060  N   LEU D 143     5.946  51.769 -34.518  1.00 20.41          N
ATOM   6061  CA  LEU D 143     6.275  50.360 -34.410  1.00 18.74          C
ATOM   6062  C   LEU D 143     7.761  50.270 -34.747  1.00 17.81          C
ATOM   6063  O   LEU D 143     8.180  50.707 -35.811  1.00 16.65          O
ATOM   6064  CB  LEU D 143     5.444  49.567 -35.391  1.00 19.30          C
ATOM   6065  CG  LEU D 143     5.733  48.078 -35.483  1.00 19.33          C
ATOM   6066  CD1 LEU D 143     5.475  47.421 -34.152  1.00 19.24          C
ATOM   6067  CD2 LEU D 143     4.923  47.414 -36.601  1.00 19.73          C
ATOM   6068  N   VAL D 144     8.546  49.712 -33.822  1.00 18.08          N
ATOM   6069  CA  VAL D 144    10.000  49.683 -33.927  1.00 17.43          C
ATOM   6070  C   VAL D 144    10.386  48.217 -34.031  1.00 17.41          C
ATOM   6071  O   VAL D 144    10.215  47.447 -33.084  1.00 18.20          O
ATOM   6072  CB  VAL D 144    10.653  50.347 -32.717  1.00 17.05          C
ATOM   6073  CG1 VAL D 144    12.210  50.414 -32.879  1.00 18.34          C
ATOM   6074  CG2 VAL D 144    10.067  51.753 -32.502  1.00 18.58          C
ATOM   6075  N   LYS D 145    10.860  47.833 -35.202  1.00 17.46          N
ATOM   6076  CA  LYS D 145    10.927  46.430 -35.578  1.00 18.87          C
ATOM   6077  C   LYS D 145    12.286  46.034 -36.107  1.00 17.24          C
ATOM   6078  O   LYS D 145    13.004  46.839 -36.692  1.00 18.12          O
ATOM   6079  CB  LYS D 145     9.862  46.160 -36.657  1.00 20.18          C
ATOM   6080  CG  LYS D 145     9.638  44.724 -36.970  1.00 22.77          C
ATOM   6081  CD  LYS D 145     8.183  44.499 -37.457  1.00 23.03          C
ATOM   6082  CE  LYS D 145     7.922  43.040 -37.683  1.00 23.87          C
ATOM   6083  NZ  LYS D 145     8.248  42.219 -36.483  1.00 25.90          N
ATOM   6084  N   GLY D 146    12.644  44.780 -35.895  1.00 15.36          N
ATOM   6085  CA  GLY D 146    13.778  44.211 -36.606  1.00 16.43          C
ATOM   6086  C   GLY D 146    15.157  44.551 -36.087  1.00 15.67          C
ATOM   6087  O   GLY D 146    16.131  44.431 -36.835  1.00 16.79          O
ATOM   6088  N   TYR D 147    15.265  44.906 -34.807  1.00 16.40          N
ATOM   6089  CA  TYR D 147    16.561  45.315 -34.219  1.00 14.95          C
ATOM   6090  C   TYR D 147    17.143  44.340 -33.225  1.00 14.82          C
ATOM   6091  O   TYR D 147    16.456  43.454 -32.675  1.00 14.45          O
ATOM   6092  CB  TYR D 147    16.488  46.718 -33.568  1.00 15.64          C
ATOM   6093  CG  TYR D 147    15.652  46.816 -32.291  1.00 15.08          C
ATOM   6094  CD1 TYR D 147    14.266  46.989 -32.349  1.00 14.21          C
ATOM   6095  CD2 TYR D 147    16.256  46.744 -31.032  1.00 13.89          C
ATOM   6096  CE1 TYR D 147    13.496  47.072 -31.182  1.00 13.75          C
ATOM   6097  CE2 TYR D 147    15.516  46.831 -29.882  1.00 13.91          C
ATOM   6098  CZ  TYR D 147    14.115  46.983 -29.956  1.00 15.55          C
```

| | | | | | | | | | |
|------|------|-----|-----|---|-----|---------|--------|---------|-----------|---|
| ATOM | 6099 | OH | TYR | D | 147 | 13.395 | 47.083 | -28.790 | 1.00 15.74 | O |
| ATOM | 6100 | N | PHE | D | 148 | 18.434 | 44.517 | -32.998 | 1.00 13.84 | N |
| ATOM | 6101 | CA | PHE | D | 148 | 19.162 | 43.748 | -32.021 | 1.00 13.83 | C |
| ATOM | 6102 | C | PHE | D | 148 | 20.458 | 44.483 | -31.745 | 1.00 13.34 | C |
| ATOM | 6103 | O | PHE | D | 148 | 21.026 | 45.064 | -32.671 | 1.00 12.39 | O |
| ATOM | 6104 | CB | PHE | D | 148 | 19.455 | 42.334 | -32.524 | 1.00 14.24 | C |
| ATOM | 6105 | CG | PHE | D | 148 | 20.022 | 41.452 | -31.463 | 1.00 13.93 | C |
| ATOM | 6106 | CD1 | PHE | D | 148 | 19.188 | 40.766 | -30.610 | 1.00 14.77 | C |
| ATOM | 6107 | CD2 | PHE | D | 148 | 21.412 | 41.392 | -31.252 | 1.00 14.98 | C |
| ATOM | 6108 | CE1 | PHE | D | 148 | 19.714 | 39.981 | -29.578 | 1.00 14.06 | C |
| ATOM | 6109 | CE2 | PHE | D | 148 | 21.924 | 40.631 | -30.231 | 1.00 13.80 | C |
| ATOM | 6110 | CZ | PHE | D | 148 | 21.057 | 39.949 | -29.378 | 1.00 14.43 | C |
| ATOM | 6111 | N | PRO | D | 149 | 20.912 | 44.514 | -30.486 | 1.00 12.83 | N |
| ATOM | 6112 | CA | PRO | D | 149 | 20.269 | 44.100 | -29.244 | 1.00 13.27 | C |
| ATOM | 6113 | C | PRO | D | 149 | 19.396 | 45.219 | -28.666 | 1.00 14.53 | C |
| ATOM | 6114 | O | PRO | D | 149 | 19.385 | 46.329 | -29.199 | 1.00 15.01 | O |
| ATOM | 6115 | CB | PRO | D | 149 | 21.463 | 43.860 | -28.320 | 1.00 12.97 | C |
| ATOM | 6116 | CG | PRO | D | 149 | 22.454 | 44.908 | -28.737 | 1.00 13.40 | C |
| ATOM | 6117 | CD | PRO | D | 149 | 22.267 | 45.032 | -30.249 | 1.00 13.76 | C |
| ATOM | 6118 | N | GLU | D | 150 | 18.688 | 44.923 | -27.579 | 1.00 15.52 | N |
| ATOM | 6119 | CA | GLU | D | 150 | 18.171 | 45.961 | -26.692 | 1.00 16.48 | C |
| ATOM | 6120 | C | GLU | D | 150 | 19.342 | 46.719 | -26.116 | 1.00 17.24 | C |
| ATOM | 6121 | O | GLU | D | 150 | 20.452 | 46.185 | -26.074 | 1.00 16.89 | O |
| ATOM | 6122 | CB | GLU | D | 150 | 17.354 | 45.340 | -25.568 | 1.00 18.09 | C |
| ATOM | 6123 | CG | GLU | D | 150 | 15.949 | 45.020 | -25.981 | 1.00 19.23 | C |
| ATOM | 6124 | CD | GLU | D | 150 | 15.002 | 46.162 | -25.669 | 1.00 23.91 | C |
| ATOM | 6125 | OE1 | GLU | D | 150 | 14.428 | 46.103 | -24.560 | 1.00 27.10 | O |
| ATOM | 6126 | OE2 | GLU | D | 150 | 14.847 | 47.106 | -26.502 | 1.00 20.62 | O |
| ATOM | 6127 | N | PRO | D | 151 | 19.116 | 47.967 | -25.674 | 1.00 16.81 | N |
| ATOM | 6128 | CA | PRO | D | 151 | 17.886 | 48.745 | -25.678 | 1.00 17.44 | C |
| ATOM | 6129 | C | PRO | D | 151 | 17.693 | 49.711 | -26.863 | 1.00 19.36 | C |
| ATOM | 6130 | O | PRO | D | 151 | 18.599 | 49.947 | -27.647 | 1.00 17.63 | O |
| ATOM | 6131 | CB | PRO | D | 151 | 18.046 | 49.583 | -24.415 | 1.00 17.86 | C |
| ATOM | 6132 | CG | PRO | D | 151 | 19.500 | 49.904 | -24.384 | 1.00 17.92 | C |
| ATOM | 6133 | CD | PRO | D | 151 | 20.197 | 48.696 | -24.978 | 1.00 17.64 | C |
| ATOM | 6134 | N | VAL | D | 152 | 16.473 | 50.227 | -26.998 | 1.00 21.56 | N |
| ATOM | 6135 | CA | VAL | D | 152 | 16.241 | 51.425 | -27.790 | 1.00 23.71 | C |
| ATOM | 6136 | C | VAL | D | 152 | 15.554 | 52.416 | -26.866 | 1.00 25.30 | C |
| ATOM | 6137 | O | VAL | D | 152 | 14.978 | 52.024 | -25.847 | 1.00 25.43 | O |
| ATOM | 6138 | CB | VAL | D | 152 | 15.388 | 51.197 | -29.071 | 1.00 25.13 | C |
| ATOM | 6139 | CG1 | VAL | D | 152 | 16.023 | 50.163 | -30.003 | 1.00 24.88 | C |
| ATOM | 6140 | CG2 | VAL | D | 152 | 13.958 | 50.842 | -28.727 | 1.00 26.21 | C |
| ATOM | 6141 | N | THR | D | 153 | 15.631 | 53.693 | -27.199 | 1.00 26.44 | N |
| ATOM | 6142 | CA | THR | D | 153 | 14.861 | 54.685 | -26.470 | 1.00 27.99 | C |
| ATOM | 6143 | C | THR | D | 153 | 13.992 | 55.470 | -27.452 | 1.00 26.95 | C |
| ATOM | 6144 | O | THR | D | 153 | 14.470 | 55.950 | -28.482 | 1.00 26.63 | O |
| ATOM | 6145 | CB | THR | D | 153 | 15.741 | 55.638 | -25.695 | 1.00 28.68 | C |
| ATOM | 6146 | OG1 | THR | D | 153 | 16.333 | 56.576 | -26.594 | 1.00 33.78 | O |
| ATOM | 6147 | CG2 | THR | D | 153 | 16.809 | 54.895 | -24.951 | 1.00 31.09 | C |
| ATOM | 6148 | N | VAL | D | 154 | 12.725 | 55.605 | -27.096 | 1.00 26.50 | N |
| ATOM | 6149 | CA | VAL | D | 154 | 11.732 | 56.235 | -27.943 | 1.00 26.14 | C |
| ATOM | 6150 | C | VAL | D | 154 | 11.281 | 57.506 | -27.247 | 1.00 26.80 | C |
| ATOM | 6151 | O | VAL | D | 154 | 10.955 | 57.486 | -26.064 | 1.00 25.92 | O |
| ATOM | 6152 | CB | VAL | D | 154 | 10.528 | 55.306 | -28.140 | 1.00 26.71 | C |
| ATOM | 6153 | CG1 | VAL | D | 154 | 9.427 | 55.999 | -28.950 | 1.00 25.54 | C |
| ATOM | 6154 | CG2 | VAL | D | 154 | 10.979 | 54.010 | -28.790 | 1.00 27.16 | C |
| ATOM | 6155 | N | THR | D | 155 | 11.291 | 58.602 | -27.991 | 1.00 26.89 | N |
| ATOM | 6156 | CA | THR | D | 155 | 10.743 | 59.859 | -27.522 | 1.00 27.22 | C |
| ATOM | 6157 | C | THR | D | 155 | 9.779 | 60.353 | -28.577 | 1.00 26.95 | C |
| ATOM | 6158 | O | THR | D | 155 | 9.802 | 59.893 | -29.729 | 1.00 25.15 | O |
| ATOM | 6159 | CB | THR | D | 155 | 11.836 | 60.918 | -27.266 | 1.00 27.00 | C |
| ATOM | 6160 | OG1 | THR | D | 155 | 12.551 | 61.207 | -28.478 | 1.00 28.35 | O |
| ATOM | 6161 | CG2 | THR | D | 155 | 12.787 | 60.427 | -26.194 | 1.00 28.15 | C |
| ATOM | 6162 | N | TRP | D | 156 | 8.916 | 61.275 | -28.167 | 1.00 28.15 | N |
| ATOM | 6163 | CA | TRP | D | 156 | 7.971 | 61.906 | -29.087 | 1.00 28.85 | C |

```
ATOM   6164  C    TRP D 156      8.238  63.391 -29.150  1.00 30.06          C
ATOM   6165  O    TRP D 156      8.330  64.045 -28.117  1.00 30.12          O
ATOM   6166  CB   TRP D 156      6.549  61.646 -28.635  1.00 28.46          C
ATOM   6167  CG   TRP D 156      6.155  60.226 -28.829  1.00 27.99          C
ATOM   6168  CD1  TRP D 156      6.254  59.220 -27.919  1.00 27.76          C
ATOM   6169  CD2  TRP D 156      5.605  59.651 -30.010  1.00 27.64          C
ATOM   6170  NE1  TRP D 156      5.804  58.040 -28.464  1.00 28.63          N
ATOM   6171  CE2  TRP D 156      5.396  58.275 -29.748  1.00 27.69          C
ATOM   6172  CE3  TRP D 156      5.272  60.157 -31.270  1.00 28.10          C
ATOM   6173  CZ2  TRP D 156      4.866  57.402 -30.697  1.00 27.88          C
ATOM   6174  CZ3  TRP D 156      4.742  59.287 -32.219  1.00 27.78          C
ATOM   6175  CH2  TRP D 156      4.538  57.922 -31.921  1.00 28.38          C
ATOM   6176  N    ASN D 157      8.358  63.907 -30.366  1.00 31.99          N
ATOM   6177  CA   ASN D 157      8.698  65.315 -30.582  1.00 34.08          C
ATOM   6178  C    ASN D 157      9.879  65.713 -29.713  1.00 35.40          C
ATOM   6179  O    ASN D 157      9.794  66.641 -28.894  1.00 35.89          O
ATOM   6180  CB   ASN D 157      7.493  66.221 -30.306  1.00 34.09          C
ATOM   6181  CG   ASN D 157      6.418  66.088 -31.345  1.00 34.16          C
ATOM   6182  OD1  ASN D 157      6.600  65.435 -32.380  1.00 35.79          O
ATOM   6183  ND2  ASN D 157      5.288  66.727 -31.093  1.00 33.83          N
ATOM   6184  N    SER D 158     10.964  64.957 -29.886  1.00 36.95          N
ATOM   6185  CA   SER D 158     12.237  65.184 -29.208  1.00 37.92          C
ATOM   6186  C    SER D 158     12.127  65.394 -27.705  1.00 38.78          C
ATOM   6187  O    SER D 158     12.920  66.129 -27.129  1.00 39.89          O
ATOM   6188  CB   SER D 158     12.951  66.362 -29.865  1.00 37.94          C
ATOM   6189  OG   SER D 158     13.013  66.176 -31.272  1.00 38.00          O
ATOM   6190  N    GLY D 159     11.167  64.723 -27.074  1.00 39.81          N
ATOM   6191  CA   GLY D 159     10.954  64.825 -25.630  1.00 41.02          C
ATOM   6192  C    GLY D 159      9.853  65.789 -25.176  1.00 41.90          C
ATOM   6193  O    GLY D 159      9.427  65.722 -24.020  1.00 41.54          O
ATOM   6194  N    SER D 160      9.390  66.674 -26.068  1.00 42.60          N
ATOM   6195  CA   SER D 160      8.315  67.630 -25.743  1.00 43.05          C
ATOM   6196  C    SER D 160      7.030  66.888 -25.403  1.00 43.92          C
ATOM   6197  O    SER D 160      6.372  67.175 -24.396  1.00 44.30          O
ATOM   6198  CB   SER D 160      8.023  68.564 -26.922  1.00 43.29          C
ATOM   6199  OG   SER D 160      9.183  68.876 -27.671  1.00 44.84          O
ATOM   6200  N    LEU D 161      6.680  65.935 -26.260  1.00 44.00          N
ATOM   6201  CA   LEU D 161      5.473  65.146 -26.089  1.00 44.30          C
ATOM   6202  C    LEU D 161      5.758  64.061 -25.061  1.00 44.49          C
ATOM   6203  O    LEU D 161      6.223  62.966 -25.401  1.00 43.82          O
ATOM   6204  CB   LEU D 161      5.038  64.529 -27.427  1.00 44.55          C
ATOM   6205  CG   LEU D 161      3.630  64.854 -27.917  1.00 45.25          C
ATOM   6206  CD1  LEU D 161      3.423  64.291 -29.317  1.00 44.88          C
ATOM   6207  CD2  LEU D 161      2.578  64.334 -26.939  1.00 46.37          C
ATOM   6208  N    SER D 162      5.495  64.378 -23.798  1.00 44.06          N
ATOM   6209  CA   SER D 162      5.779  63.463 -22.692  1.00 44.40          C
ATOM   6210  C    SER D 162      4.507  62.868 -22.096  1.00 43.43          C
ATOM   6211  O    SER D 162      4.491  61.709 -21.683  1.00 43.58          O
ATOM   6212  CB   SER D 162      6.612  64.177 -21.604  1.00 45.09          C
ATOM   6213  OG   SER D 162      6.509  65.597 -21.701  1.00 45.78          O
ATOM   6214  N    SER D 163      3.441  63.661 -22.048  1.00 42.05          N
ATOM   6215  CA   SER D 163      2.182  63.203 -21.481  1.00 40.81          C
ATOM   6216  C    SER D 163      1.420  62.336 -22.480  1.00 38.94          C
ATOM   6217  O    SER D 163      1.488  62.557 -23.689  1.00 39.17          O
ATOM   6218  CB   SER D 163      1.333  64.401 -21.066  1.00 41.17          C
ATOM   6219  OG   SER D 163      0.412  64.041 -20.055  1.00 42.25          O
ATOM   6220  N    GLY D 164      0.702  61.347 -21.967  1.00 37.28          N
ATOM   6221  CA   GLY D 164     -0.084  60.435 -22.802  1.00 36.50          C
ATOM   6222  C    GLY D 164      0.729  59.451 -23.643  1.00 34.94          C
ATOM   6223  O    GLY D 164      0.259  58.987 -24.681  1.00 33.90          O
ATOM   6224  N    VAL D 165      1.930  59.123 -23.174  1.00 34.66          N
ATOM   6225  CA   VAL D 165      2.851  58.214 -23.866  1.00 34.05          C
ATOM   6226  C    VAL D 165      3.020  56.920 -23.063  1.00 34.25          C
ATOM   6227  O    VAL D 165      3.338  56.994 -21.876  1.00 35.02          O
ATOM   6228  CB   VAL D 165      4.246  58.856 -24.026  1.00 33.75          C
```

| | | | | | | | | | | | |
|---|---|---|---|---|---|---|---|---|---|---|---|
| ATOM | 6229 | CG1 | VAL | D | 165 | 5.207 | 57.900 | -24.712 | 1.00 | 33.93 | C |
| ATOM | 6230 | CG2 | VAL | D | 165 | 4.161 | 60.153 | -24.800 | 1.00 | 34.96 | C |
| ATOM | 6231 | N | HIS | D | 166 | 2.767 | 55.769 | -23.708 | 1.00 | 33.08 | N |
| ATOM | 6232 | CA | HIS | D | 166 | 3.151 | 54.428 | -23.219 | 1.00 | 31.92 | C |
| ATOM | 6233 | C | HIS | D | 166 | 4.183 | 53.877 | -24.187 | 1.00 | 29.95 | C |
| ATOM | 6234 | O | HIS | D | 166 | 3.920 | 53.869 | -25.383 | 1.00 | 29.68 | O |
| ATOM | 6235 | CB | HIS | D | 166 | 1.989 | 53.409 | -23.289 | 1.00 | 32.34 | C |
| ATOM | 6236 | CG | HIS | D | 166 | 0.711 | 53.867 | -22.671 | 1.00 | 33.09 | C |
| ATOM | 6237 | ND1 | HIS | D | 166 | 0.621 | 54.265 | -21.354 | 1.00 | 36.15 | N |
| ATOM | 6238 | CD2 | HIS | D | 166 | -0.545 | 53.912 | -23.165 | 1.00 | 33.55 | C |
| ATOM | 6239 | CE1 | HIS | D | 166 | -0.633 | 54.578 | -21.075 | 1.00 | 36.00 | C |
| ATOM | 6240 | NE2 | HIS | D | 166 | -1.361 | 54.371 | -22.159 | 1.00 | 36.24 | N |
| ATOM | 6241 | N | THR | D | 167 | 5.323 | 53.373 | -23.698 | 1.00 | 27.47 | N |
| ATOM | 6242 | CA | THR | D | 167 | 6.213 | 52.574 | -24.549 | 1.00 | 25.51 | C |
| ATOM | 6243 | C | THR | D | 167 | 6.196 | 51.123 | -24.020 | 1.00 | 23.63 | C |
| ATOM | 6244 | O | THR | D | 167 | 6.423 | 50.876 | -22.828 | 1.00 | 22.71 | O |
| ATOM | 6245 | CB | THR | D | 167 | 7.645 | 53.198 | -24.632 | 1.00 | 27.14 | C |
| ATOM | 6246 | OG1 | THR | D | 167 | 7.579 | 54.493 | -25.263 | 1.00 | 27.91 | O |
| ATOM | 6247 | CG2 | THR | D | 167 | 8.590 | 52.327 | -25.442 | 1.00 | 27.10 | C |
| ATOM | 6248 | N | PHE | D | 168 | 5.895 | 50.181 | -24.906 | 1.00 | 21.03 | N |
| ATOM | 6249 | CA | PHE | D | 168 | 5.730 | 48.796 | -24.532 | 1.00 | 20.57 | C |
| ATOM | 6250 | C | PHE | D | 168 | 7.087 | 48.107 | -24.529 | 1.00 | 21.25 | C |
| ATOM | 6251 | O | PHE | D | 168 | 7.867 | 48.280 | -25.453 | 1.00 | 20.49 | O |
| ATOM | 6252 | CB | PHE | D | 168 | 4.751 | 48.133 | -25.505 | 1.00 | 20.86 | C |
| ATOM | 6253 | CG | PHE | D | 168 | 3.382 | 48.780 | -25.481 | 1.00 | 22.24 | C |
| ATOM | 6254 | CD1 | PHE | D | 168 | 3.079 | 49.845 | -26.325 | 1.00 | 23.10 | C |
| ATOM | 6255 | CD2 | PHE | D | 168 | 2.431 | 48.354 | -24.576 | 1.00 | 22.94 | C |
| ATOM | 6256 | CE1 | PHE | D | 168 | 1.834 | 50.467 | -26.267 | 1.00 | 23.44 | C |
| ATOM | 6257 | CE2 | PHE | D | 168 | 1.195 | 48.958 | -24.516 | 1.00 | 22.27 | C |
| ATOM | 6258 | CZ | PHE | D | 168 | 0.900 | 50.022 | -25.355 | 1.00 | 23.02 | C |
| ATOM | 6259 | N | PRO | D | 169 | 7.376 | 47.307 | -23.488 | 1.00 | 23.02 | N |
| ATOM | 6260 | CA | PRO | D | 169 | 8.605 | 46.523 | -23.493 | 1.00 | 23.16 | C |
| ATOM | 6261 | C | PRO | D | 169 | 8.824 | 45.732 | -24.781 | 1.00 | 22.07 | C |
| ATOM | 6262 | O | PRO | D | 169 | 7.875 | 45.218 | -25.382 | 1.00 | 22.18 | O |
| ATOM | 6263 | CB | PRO | D | 169 | 8.389 | 45.535 | -22.345 | 1.00 | 23.49 | C |
| ATOM | 6264 | CG | PRO | D | 169 | 7.521 | 46.255 | -21.412 | 1.00 | 24.68 | C |
| ATOM | 6265 | CD | PRO | D | 169 | 6.583 | 47.054 | -22.270 | 1.00 | 23.49 | C |
| ATOM | 6266 | N | ALA | D | 170 | 10.088 | 45.603 | -25.174 | 1.00 | 20.65 | N |
| ATOM | 6267 | CA | ALA | D | 170 | 10.445 | 44.845 | -26.357 | 1.00 | 20.64 | C |
| ATOM | 6268 | C | ALA | D | 170 | 10.244 | 43.358 | -26.160 | 1.00 | 21.95 | C |
| ATOM | 6269 | O | ALA | D | 170 | 10.405 | 42.854 | -25.054 | 1.00 | 21.89 | O |
| ATOM | 6270 | CB | ALA | D | 170 | 11.895 | 45.110 | -26.726 | 1.00 | 20.78 | C |
| ATOM | 6271 | N | VAL | D | 171 | 9.950 | 42.675 | -27.255 | 1.00 | 22.65 | N |
| ATOM | 6272 | CA | VAL | D | 171 | 9.801 | 41.225 | -27.294 | 1.00 | 23.42 | C |
| ATOM | 6273 | C | VAL | D | 171 | 10.670 | 40.653 | -28.402 | 1.00 | 23.47 | C |
| ATOM | 6274 | O | VAL | D | 171 | 10.689 | 41.142 | -29.533 | 1.00 | 21.97 | O |
| ATOM | 6275 | CB | VAL | D | 171 | 8.362 | 40.827 | -27.515 | 1.00 | 23.92 | C |
| ATOM | 6276 | CG1 | VAL | D | 171 | 8.220 | 39.284 | -27.625 | 1.00 | 25.52 | C |
| ATOM | 6277 | CG2 | VAL | D | 171 | 7.518 | 41.402 | -26.378 | 1.00 | 25.04 | C |
| ATOM | 6278 | N | LEU | D | 172 | 11.445 | 39.639 | -28.031 | 1.00 | 24.75 | N |
| ATOM | 6279 | CA | LEU | D | 172 | 12.362 | 38.984 | -28.928 | 1.00 | 26.04 | C |
| ATOM | 6280 | C | LEU | D | 172 | 11.664 | 37.861 | -29.672 | 1.00 | 28.99 | C |
| ATOM | 6281 | O | LEU | D | 172 | 10.955 | 37.054 | -29.054 | 1.00 | 28.46 | O |
| ATOM | 6282 | CB | LEU | D | 172 | 13.521 | 38.398 | -28.117 | 1.00 | 25.92 | C |
| ATOM | 6283 | CG | LEU | D | 172 | 14.663 | 37.739 | -28.874 | 1.00 | 26.52 | C |
| ATOM | 6284 | CD1 | LEU | D | 172 | 15.454 | 38.781 | -29.637 | 1.00 | 26.46 | C |
| ATOM | 6285 | CD2 | LEU | D | 172 | 15.539 | 37.027 | -27.863 | 1.00 | 26.33 | C |
| ATOM | 6286 | N | GLN | D | 173 | 11.886 | 37.816 | -30.984 | 1.00 | 32.47 | N |
| ATOM | 6287 | CA | GLN | D | 173 | 11.497 | 36.696 | -31.850 | 1.00 | 36.48 | C |
| ATOM | 6288 | C | GLN | D | 173 | 12.511 | 36.547 | -32.980 | 1.00 | 37.60 | C |
| ATOM | 6289 | O | GLN | D | 173 | 12.855 | 37.526 | -33.643 | 1.00 | 37.80 | O |
| ATOM | 6290 | CB | GLN | D | 173 | 10.118 | 36.928 | -32.454 | 1.00 | 37.62 | C |
| ATOM | 6291 | CG | GLN | D | 173 | 9.640 | 35.756 | -33.309 | 1.00 | 39.98 | C |
| ATOM | 6292 | CD | GLN | D | 173 | 8.297 | 36.006 | -33.952 | 1.00 | 40.36 | C |
| ATOM | 6293 | OE1 | GLN | D | 173 | 7.408 | 36.631 | -33.357 | 1.00 | 43.54 | O |

| ATOM | 6294 | NE2 | GLN | D | 173 | 8.130 | 35.504 | -35.177 | 1.00 | 43.53 | N |
|------|------|-----|-----|---|-----|-------|--------|---------|------|-------|---|
| ATOM | 6295 | N | SER | D | 174 | 12.988 | 35.324 | -33.203 | 1.00 | 38.73 | N |
| ATOM | 6296 | CA | SER | D | 174 | 13.957 | 35.028 | -34.281 | 1.00 | 38.60 | C |
| ATOM | 6297 | C | SER | D | 174 | 15.195 | 35.937 | -34.240 | 1.00 | 37.89 | C |
| ATOM | 6298 | O | SER | D | 174 | 15.662 | 36.435 | -35.278 | 1.00 | 38.15 | O |
| ATOM | 6299 | CB | SER | D | 174 | 13.285 | 35.108 | -35.666 | 1.00 | 39.42 | C |
| ATOM | 6300 | OG | SER | D | 174 | 12.022 | 34.453 | -35.699 | 1.00 | 41.11 | O |
| ATOM | 6301 | N | ASP | D | 175 | 15.716 | 36.124 | -33.027 | 1.00 | 35.36 | N |
| ATOM | 6302 | CA | ASP | D | 175 | 16.850 | 37.009 | -32.730 | 1.00 | 34.43 | C |
| ATOM | 6303 | C | ASP | D | 175 | 16.694 | 38.471 | -33.122 | 1.00 | 29.54 | C |
| ATOM | 6304 | O | ASP | D | 175 | 17.689 | 39.181 | -33.236 | 1.00 | 29.75 | O |
| ATOM | 6305 | CB | ASP | D | 175 | 18.167 | 36.446 | -33.286 | 1.00 | 36.36 | C |
| ATOM | 6306 | CG | ASP | D | 175 | 19.088 | 35.899 | -32.180 | 1.00 | 38.89 | C |
| ATOM | 6307 | OD1 | ASP | D | 175 | 19.181 | 36.514 | -31.077 | 1.00 | 41.70 | O |
| ATOM | 6308 | OD2 | ASP | D | 175 | 19.727 | 34.857 | -32.430 | 1.00 | 41.74 | O |
| ATOM | 6309 | N | LEU | D | 176 | 15.462 | 38.943 | -33.287 | 1.00 | 26.27 | N |
| ATOM | 6310 | CA | LEU | D | 176 | 15.238 | 40.397 | -33.464 | 1.00 | 21.28 | C |
| ATOM | 6311 | C | LEU | D | 176 | 14.138 | 40.842 | -32.535 | 1.00 | 20.06 | C |
| ATOM | 6312 | O | LEU | D | 176 | 13.216 | 40.061 | -32.225 | 1.00 | 18.69 | O |
| ATOM | 6313 | CB | LEU | D | 176 | 14.878 | 40.729 | -34.904 | 1.00 | 20.72 | C |
| ATOM | 6314 | CG | LEU | D | 176 | 15.946 | 40.338 | -35.925 | 1.00 | 20.13 | C |
| ATOM | 6315 | CD1 | LEU | D | 176 | 15.348 | 40.320 | -37.297 | 1.00 | 21.21 | C |
| ATOM | 6316 | CD2 | LEU | D | 176 | 17.142 | 41.283 | -35.856 | 1.00 | 20.80 | C |
| ATOM | 6317 | N | TYR | D | 177 | 14.224 | 42.101 | -32.097 | 1.00 | 17.05 | N |
| ATOM | 6318 | CA | TYR | D | 177 | 13.225 | 42.676 | -31.204 | 1.00 | 16.81 | C |
| ATOM | 6319 | C | TYR | D | 177 | 12.181 | 43.458 | -31.967 | 1.00 | 17.62 | C |
| ATOM | 6320 | O | TYR | D | 177 | 12.468 | 44.008 | -33.037 | 1.00 | 16.47 | O |
| ATOM | 6321 | CB | TYR | D | 177 | 13.884 | 43.617 | -30.193 | 1.00 | 16.39 | C |
| ATOM | 6322 | CG | TYR | D | 177 | 14.643 | 42.885 | -29.125 | 1.00 | 17.28 | C |
| ATOM | 6323 | CD1 | TYR | D | 177 | 13.990 | 42.403 | -28.000 | 1.00 | 16.51 | C |
| ATOM | 6324 | CD2 | TYR | D | 177 | 16.001 | 42.637 | -29.262 | 1.00 | 18.28 | C |
| ATOM | 6325 | CE1 | TYR | D | 177 | 14.678 | 41.707 | -26.993 | 1.00 | 17.92 | C |
| ATOM | 6326 | CE2 | TYR | D | 177 | 16.714 | 41.954 | -28.265 | 1.00 | 15.92 | C |
| ATOM | 6327 | CZ | TYR | D | 177 | 16.047 | 41.492 | -27.137 | 1.00 | 18.56 | C |
| ATOM | 6328 | OH | TYR | D | 177 | 16.754 | 40.804 | -26.159 | 1.00 | 19.85 | O |
| ATOM | 6329 | N | THR | D | 178 | 10.984 | 43.506 | -31.384 | 1.00 | 19.22 | N |
| ATOM | 6330 | CA | THR | D | 178 | 9.918 | 44.429 | -31.760 | 1.00 | 19.98 | C |
| ATOM | 6331 | C | THR | D | 178 | 9.483 | 45.156 | -30.525 | 1.00 | 19.40 | C |
| ATOM | 6332 | O | THR | D | 178 | 9.314 | 44.539 | -29.476 | 1.00 | 18.28 | O |
| ATOM | 6333 | CB | THR | D | 178 | 8.652 | 43.672 | -32.273 | 1.00 | 21.68 | C |
| ATOM | 6334 | OG1 | THR | D | 178 | 9.020 | 42.766 | -33.310 | 1.00 | 22.80 | O |
| ATOM | 6335 | CG2 | THR | D | 178 | 7.627 | 44.626 | -32.816 | 1.00 | 23.09 | C |
| ATOM | 6336 | N | LEU | D | 179 | 9.308 | 46.466 | -30.620 | 1.00 | 18.34 | N |
| ATOM | 6337 | CA | LEU | D | 179 | 8.526 | 47.170 | -29.634 | 1.00 | 19.89 | C |
| ATOM | 6338 | C | LEU | D | 179 | 7.646 | 48.226 | -30.289 | 1.00 | 19.96 | C |
| ATOM | 6339 | O | LEU | D | 179 | 7.732 | 48.477 | -31.479 | 1.00 | 19.19 | O |
| ATOM | 6340 | CB | LEU | D | 179 | 9.359 | 47.755 | -28.513 | 1.00 | 20.87 | C |
| ATOM | 6341 | CG | LEU | D | 179 | 10.257 | 48.972 | -28.588 | 1.00 | 20.79 | C |
| ATOM | 6342 | CD1 | LEU | D | 179 | 9.534 | 50.291 | -28.855 | 1.00 | 21.46 | C |
| ATOM | 6343 | CD2 | LEU | D | 179 | 10.978 | 49.072 | -27.266 | 1.00 | 21.57 | C |
| ATOM | 6344 | N | SER | D | 180 | 6.780 | 48.809 | -29.494 | 1.00 | 20.29 | N |
| ATOM | 6345 | CA | SER | D | 180 | 5.882 | 49.841 | -29.983 | 1.00 | 21.49 | C |
| ATOM | 6346 | C | SER | D | 180 | 5.712 | 50.900 | -28.909 | 1.00 | 20.31 | C |
| ATOM | 6347 | O | SER | D | 180 | 5.984 | 50.684 | -27.727 | 1.00 | 19.18 | O |
| ATOM | 6348 | CB | SER | D | 180 | 4.524 | 49.241 | -30.370 | 1.00 | 22.23 | C |
| ATOM | 6349 | OG | SER | D | 180 | 3.946 | 48.562 | -29.270 | 1.00 | 24.76 | O |
| ATOM | 6350 | N | SER | D | 181 | 5.313 | 52.084 | -29.337 | 1.00 | 21.06 | N |
| ATOM | 6351 | CA | SER | D | 181 | 5.013 | 53.148 | -28.410 | 1.00 | 21.95 | C |
| ATOM | 6352 | C | SER | D | 181 | 3.740 | 53.848 | -28.878 | 1.00 | 23.38 | C |
| ATOM | 6353 | O | SER | D | 181 | 3.571 | 54.064 | -30.084 | 1.00 | 19.89 | O |
| ATOM | 6354 | CB | SER | D | 181 | 6.153 | 54.153 | -28.376 | 1.00 | 22.37 | C |
| ATOM | 6355 | OG | SER | D | 181 | 5.928 | 55.165 | -27.405 | 1.00 | 23.24 | O |
| ATOM | 6356 | N | SER | D | 182 | 2.859 | 54.168 | -27.929 | 1.00 | 25.42 | N |
| ATOM | 6357 | CA | SER | D | 182 | 1.631 | 54.919 | -28.233 | 1.00 | 26.55 | C |
| ATOM | 6358 | C | SER | D | 182 | 1.683 | 56.318 | -27.626 | 1.00 | 26.30 | C |

| ATOM | 6359 | O | SER | D | 182 | 2.296 | 56.536 | -26.587 | 1.00 | 25.79 | O |
|------|------|------|------|---|-----|--------|--------|---------|------|-------|---|
| ATOM | 6360 | CB | SER | D | 182 | 0.371 | 54.130 | -27.827 | 1.00 | 28.49 | C |
| ATOM | 6361 | OG | SER | D | 182 | -0.129 | 54.425 | -26.551 | 1.00 | 30.66 | O |
| ATOM | 6362 | N | VAL | D | 183 | 1.087 | 57.280 | -28.332 | 1.00 | 25.77 | N |
| ATOM | 6363 | CA | VAL | D | 183 | 0.933 | 58.639 | -27.835 | 1.00 | 26.61 | C |
| ATOM | 6364 | C | VAL | D | 183 | -0.498 | 59.053 | -28.157 | 1.00 | 27.81 | C |
| ATOM | 6365 | O | VAL | D | 183 | -0.993 | 58.782 | -29.248 | 1.00 | 27.51 | O |
| ATOM | 6366 | CB | VAL | D | 183 | 2.005 | 59.626 | -28.412 | 1.00 | 26.20 | C |
| ATOM | 6367 | CG1 | VAL | D | 183 | 2.008 | 59.673 | -29.921 | 1.00 | 24.95 | C |
| ATOM | 6368 | CG2 | VAL | D | 183 | 1.858 | 61.026 | -27.813 | 1.00 | 26.72 | C |
| ATOM | 6369 | N | THR | D | 184 | -1.173 | 59.642 | -27.176 | 1.00 | 29.81 | N |
| ATOM | 6370 | CA | THR | D | 184 | -2.547 | 60.109 | -27.337 | 1.00 | 31.11 | C |
| ATOM | 6371 | C | THR | D | 184 | -2.577 | 61.611 | -27.137 | 1.00 | 32.28 | C |
| ATOM | 6372 | O | THR | D | 184 | -2.086 | 62.110 | -26.121 | 1.00 | 31.17 | O |
| ATOM | 6373 | CB | THR | D | 184 | -3.456 | 59.420 | -26.329 | 1.00 | 32.19 | C |
| ATOM | 6374 | OG1 | THR | D | 184 | -3.472 | 58.027 | -26.630 | 1.00 | 31.01 | O |
| ATOM | 6375 | CG2 | THR | D | 184 | -4.875 | 59.965 | -26.388 | 1.00 | 31.84 | C |
| ATOM | 6376 | N | VAL | D | 185 | -3.130 | 62.314 | -28.125 | 1.00 | 33.91 | N |
| ATOM | 6377 | CA | VAL | D | 185 | -3.166 | 63.781 | -28.139 | 1.00 | 35.96 | C |
| ATOM | 6378 | C | VAL | D | 185 | -4.577 | 64.283 | -28.469 | 1.00 | 37.06 | C |
| ATOM | 6379 | O | VAL | D | 185 | -5.388 | 63.527 | -28.996 | 1.00 | 37.10 | O |
| ATOM | 6380 | CB | VAL | D | 185 | -2.184 | 64.386 | -29.185 | 1.00 | 35.86 | C |
| ATOM | 6381 | CG1 | VAL | D | 185 | -0.738 | 64.084 | -28.816 | 1.00 | 36.75 | C |
| ATOM | 6382 | CG2 | VAL | D | 185 | -2.502 | 63.913 | -30.607 | 1.00 | 36.12 | C |
| ATOM | 6383 | N | PRO | D | 186 | -4.870 | 65.565 | -28.161 | 1.00 | 39.36 | N |
| ATOM | 6384 | CA | PRO | D | 186 | -6.135 | 66.161 | -28.625 | 1.00 | 39.90 | C |
| ATOM | 6385 | C | PRO | D | 186 | -6.225 | 66.235 | -30.140 | 1.00 | 40.62 | C |
| ATOM | 6386 | O | PRO | D | 186 | -5.243 | 66.572 | -30.798 | 1.00 | 39.83 | O |
| ATOM | 6387 | CB | PRO | D | 186 | -6.112 | 67.570 | -28.021 | 1.00 | 40.11 | C |
| ATOM | 6388 | CG | PRO | D | 186 | -5.127 | 67.483 | -26.875 | 1.00 | 40.44 | C |
| ATOM | 6389 | CD | PRO | D | 186 | -4.087 | 66.508 | -27.342 | 1.00 | 39.44 | C |
| ATOM | 6390 | N | SER | D | 187 | -7.402 | 65.920 | -30.677 | 1.00 | 42.27 | N |
| ATOM | 6391 | CA | SER | D | 187 | -7.633 | 65.938 | -32.122 | 1.00 | 43.51 | C |
| ATOM | 6392 | C | SER | D | 187 | -7.373 | 67.309 | -32.736 | 1.00 | 44.24 | C |
| ATOM | 6393 | O | SER | D | 187 | -7.061 | 67.410 | -33.925 | 1.00 | 44.38 | O |
| ATOM | 6394 | CB | SER | D | 187 | -9.057 | 65.475 | -32.455 | 1.00 | 43.99 | C |
| ATOM | 6395 | OG | SER | D | 187 | -9.141 | 64.057 | -32.468 | 1.00 | 44.97 | O |
| ATOM | 6396 | N | SER | D | 188 | -7.480 | 68.359 | -31.925 | 1.00 | 44.98 | N |
| ATOM | 6397 | CA | SER | D | 188 | -7.167 | 69.712 | -32.385 | 1.00 | 45.33 | C |
| ATOM | 6398 | C | SER | D | 188 | -5.710 | 69.848 | -32.840 | 1.00 | 45.38 | C |
| ATOM | 6399 | O | SER | D | 188 | -5.414 | 70.643 | -33.734 | 1.00 | 46.02 | O |
| ATOM | 6400 | CB | SER | D | 188 | -7.491 | 70.744 | -31.293 | 1.00 | 45.45 | C |
| ATOM | 6401 | OG | SER | D | 188 | -6.769 | 70.503 | -30.088 | 1.00 | 46.27 | O |
| ATOM | 6402 | N | THR | D | 189 | -4.815 | 69.048 | -32.252 | 1.00 | 44.65 | N |
| ATOM | 6403 | CA | THR | D | 189 | -3.373 | 69.201 | -32.465 | 1.00 | 43.93 | C |
| ATOM | 6404 | C | THR | D | 189 | -2.774 | 68.324 | -33.573 | 1.00 | 42.66 | C |
| ATOM | 6405 | O | THR | D | 189 | -1.640 | 68.546 | -33.972 | 1.00 | 42.87 | O |
| ATOM | 6406 | CB | THR | D | 189 | -2.592 | 68.941 | -31.159 | 1.00 | 44.29 | C |
| ATOM | 6407 | OG1 | THR | D | 189 | -2.736 | 67.568 | -30.774 | 1.00 | 45.02 | O |
| ATOM | 6408 | CG2 | THR | D | 189 | -3.114 | 69.838 | -30.037 | 1.00 | 43.96 | C |
| ATOM | 6409 | N | TRP | D | 190 | -3.514 | 67.335 | -34.067 | 1.00 | 41.10 | N |
| ATOM | 6410 | CA | TRP | D | 190 | -3.021 | 66.484 | -35.149 | 1.00 | 39.73 | C |
| ATOM | 6411 | C | TRP | D | 190 | -4.177 | 66.155 | -36.091 | 1.00 | 40.52 | C |
| ATOM | 6412 | O | TRP | D | 190 | -5.270 | 65.844 | -35.624 | 1.00 | 41.43 | O |
| ATOM | 6413 | CB | TRP | D | 190 | -2.395 | 65.189 | -34.584 | 1.00 | 37.73 | C |
| ATOM | 6414 | CG | TRP | D | 190 | -1.821 | 64.284 | -35.646 | 1.00 | 35.98 | C |
| ATOM | 6415 | CD1 | TRP | D | 190 | -0.554 | 64.307 | -36.136 | 1.00 | 34.77 | C |
| ATOM | 6416 | CD2 | TRP | D | 190 | -2.512 | 63.247 | -36.358 | 1.00 | 34.77 | C |
| ATOM | 6417 | NE1 | TRP | D | 190 | -0.406 | 63.349 | -37.106 | 1.00 | 34.75 | N |
| ATOM | 6418 | CE2 | TRP | D | 190 | -1.592 | 62.683 | -37.266 | 1.00 | 34.92 | C |
| ATOM | 6419 | CE3 | TRP | D | 190 | -3.822 | 62.749 | -36.323 | 1.00 | 34.67 | C |
| ATOM | 6420 | CZ2 | TRP | D | 190 | -1.933 | 61.626 | -38.129 | 1.00 | 34.74 | C |
| ATOM | 6421 | CZ3 | TRP | D | 190 | -4.159 | 61.691 | -37.167 | 1.00 | 35.12 | C |
| ATOM | 6422 | CH2 | TRP | D | 190 | -3.214 | 61.149 | -38.067 | 1.00 | 35.59 | C |
| ATOM | 6423 | N | PRO | D | 191 | -3.941 | 66.197 | -37.417 | 1.00 | 40.86 | N |

```
ATOM   6424  CA  PRO D 191   -2.658  66.409 -38.104  1.00 41.24      C
ATOM   6425  C   PRO D 191   -2.211  67.855 -38.210  1.00 41.43      C
ATOM   6426  O   PRO D 191   -1.243  68.130 -38.912  1.00 41.35      O
ATOM   6427  CB  PRO D 191   -2.900  65.812 -39.492  1.00 41.29      C
ATOM   6428  CG  PRO D 191   -4.386  65.845 -39.690  1.00 40.79      C
ATOM   6429  CD  PRO D 191   -5.051  66.005 -38.368  1.00 41.15      C
ATOM   6430  N   SER D 192   -2.892  68.751 -37.486  1.00 42.00      N
ATOM   6431  CA  SER D 192   -2.617  70.188 -37.500  1.00 42.64      C
ATOM   6432  C   SER D 192   -1.169  70.498 -37.119  1.00 43.05      C
ATOM   6433  O   SER D 192   -0.457  71.191 -37.857  1.00 42.68      O
ATOM   6434  CB  SER D 192   -3.570  70.907 -36.525  1.00 42.63      C
ATOM   6435  OG  SER D 192   -4.752  70.141 -36.321  1.00 43.34      O
ATOM   6436  N   GLU D 193   -0.759  70.010 -35.950  1.00 42.82      N
ATOM   6437  CA  GLU D 193    0.634  70.073 -35.527  1.00 43.36      C
ATOM   6438  C   GLU D 193    1.297  68.738 -35.835  1.00 41.78      C
ATOM   6439  O   GLU D 193    0.612  67.733 -36.042  1.00 42.12      O
ATOM   6440  CB  GLU D 193    0.767  70.346 -34.030  1.00 43.96      C
ATOM   6441  CG  GLU D 193   -0.054  71.488 -33.472  1.00 45.24      C
ATOM   6442  CD  GLU D 193    0.318  71.758 -32.024  1.00 46.06      C
ATOM   6443  OE1 GLU D 193    1.480  72.162 -31.784  1.00 48.91      O
ATOM   6444  OE2 GLU D 193   -0.535  71.545 -31.127  1.00 48.71      O
ATOM   6445  N   THR D 194    2.627  68.729 -35.845  1.00 39.97      N
ATOM   6446  CA  THR D 194    3.366  67.537 -36.217  1.00 38.18      C
ATOM   6447  C   THR D 194    3.613  66.657 -35.006  1.00 35.90      C
ATOM   6448  O   THR D 194    3.810  67.145 -33.891  1.00 34.65      O
ATOM   6449  CB  THR D 194    4.707  67.878 -36.854  1.00 38.24      C
ATOM   6450  OG1 THR D 194    5.507  68.598 -35.918  1.00 39.42      O
ATOM   6451  CG2 THR D 194    4.502  68.713 -38.114  1.00 38.99      C
ATOM   6452  N   VAL D 195    3.571  65.351 -35.239  1.00 33.15      N
ATOM   6453  CA  VAL D 195    3.870  64.364 -34.202  1.00 31.91      C
ATOM   6454  C   VAL D 195    4.839  63.368 -34.813  1.00 30.41      C
ATOM   6455  O   VAL D 195    4.545  62.759 -35.845  1.00 29.61      O
ATOM   6456  CB  VAL D 195    2.607  63.668 -33.670  1.00 31.59      C
ATOM   6457  CG1 VAL D 195    2.985  62.529 -32.709  1.00 31.24      C
ATOM   6458  CG2 VAL D 195    1.704  64.679 -32.960  1.00 31.45      C
ATOM   6459  N   THR D 196    6.003  63.252 -34.169  1.00 29.52      N
ATOM   6460  CA  THR D 196    7.168  62.553 -34.711  1.00 28.07      C
ATOM   6461  C   THR D 196    7.772  61.682 -33.593  1.00 26.88      C
ATOM   6462  O   THR D 196    8.043  62.175 -32.502  1.00 26.06      O
ATOM   6463  CB  THR D 196    8.221  63.586 -35.186  1.00 28.34      C
ATOM   6464  OG1 THR D 196    7.640  64.470 -36.156  1.00 27.68      O
ATOM   6465  CG2 THR D 196    9.440  62.921 -35.820  1.00 28.79      C
ATOM   6466  N   CYS D 197    7.951  60.388 -33.832  1.00 26.56      N
ATOM   6467  CA  CYS D 197    8.719  59.610 -32.874  1.00 26.33      C
ATOM   6468  C   CYS D 197   10.185  59.589 -33.270  1.00 24.28      C
ATOM   6469  O   CYS D 197   10.532  59.647 -34.449  1.00 23.76      O
ATOM   6470  CB  CYS D 197    8.187  58.198 -32.701  1.00 28.52      C
ATOM   6471  SG  CYS D 197    8.484  57.151 -34.066  1.00 32.31      S
ATOM   6472  N   ASN D 198   11.025  59.540 -32.243  1.00 23.54      N
ATOM   6473  CA  ASN D 198   12.470  59.562 -32.368  1.00 22.89      C
ATOM   6474  C   ASN D 198   12.932  58.283 -31.714  1.00 21.44      C
ATOM   6475  O   ASN D 198   12.605  58.041 -30.555  1.00 20.24      O
ATOM   6476  CB  ASN D 198   13.058  60.739 -31.610  1.00 24.07      C
ATOM   6477  CG  ASN D 198   12.134  61.953 -31.606  1.00 25.35      C
ATOM   6478  OD1 ASN D 198   11.521  62.282 -30.580  1.00 28.22      O
ATOM   6479  ND2 ASN D 198   12.004  62.590 -32.753  1.00 27.51      N
ATOM   6480  N   VAL D 199   13.650  57.460 -32.466  1.00 20.08      N
ATOM   6481  CA  VAL D 199   14.077  56.166 -31.945  1.00 20.35      C
ATOM   6482  C   VAL D 199   15.599  56.115 -31.941  1.00 18.15      C
ATOM   6483  O   VAL D 199   16.211  56.185 -32.995  1.00 19.03      O
ATOM   6484  CB  VAL D 199   13.498  55.023 -32.784  1.00 20.73      C
ATOM   6485  CG1 VAL D 199   14.020  53.690 -32.285  1.00 21.45      C
ATOM   6486  CG2 VAL D 199   11.973  55.054 -32.741  1.00 20.81      C
ATOM   6487  N   ALA D 200   16.203  55.985 -30.762  1.00 18.38      N
ATOM   6488  CA  ALA D 200   17.654  55.873 -30.653  1.00 18.19      C
```

358

```
ATOM   6489  C    ALA D 200     18.029  54.431 -30.312  1.00 17.71           C
ATOM   6490  O    ALA D 200     17.396  53.819 -29.444  1.00 18.68           O
ATOM   6491  CB   ALA D 200     18.177  56.813 -29.589  1.00 18.92           C
ATOM   6492  N    HIS D 201     19.047  53.919 -30.995  1.00 16.01           N
ATOM   6493  CA   HIS D 201     19.567  52.551 -30.774  1.00 15.50           C
ATOM   6494  C    HIS D 201     21.065  52.655 -30.515  1.00 14.66           C
ATOM   6495  O    HIS D 201     21.835  52.746 -31.462  1.00 14.30           O
ATOM   6496  CB   HIS D 201     19.338  51.710 -32.020  1.00 14.20           C
ATOM   6497  CG   HIS D 201     19.652  50.268 -31.833  1.00 13.53           C
ATOM   6498  ND1  HIS D 201     20.277  49.514 -32.799  1.00 13.10           N
ATOM   6499  CD2  HIS D 201     19.406  49.431 -30.800  1.00 15.64           C
ATOM   6500  CE1  HIS D 201     20.402  48.273 -32.370  1.00 13.88           C
ATOM   6501  NE2  HIS D 201     19.886  48.194 -31.156  1.00 13.22           N
ATOM   6502  N    PRO D 202     21.473  52.711 -29.245  1.00 14.76           N
ATOM   6503  CA   PRO D 202     22.900  52.829 -28.897  1.00 15.44           C
ATOM   6504  C    PRO D 202     23.858  51.858 -29.594  1.00 14.52           C
ATOM   6505  O    PRO D 202     24.954  52.272 -30.034  1.00 12.50           O
ATOM   6506  CB   PRO D 202     22.907  52.574 -27.385  1.00 15.67           C
ATOM   6507  CG   PRO D 202     21.587  52.818 -26.928  1.00 17.07           C
ATOM   6508  CD   PRO D 202     20.625  52.798 -28.044  1.00 15.81           C
ATOM   6509  N    ALA D 203     23.442  50.595 -29.760  1.00 14.58           N
ATOM   6510  CA   ALA D 203     24.343  49.601 -30.317  1.00 14.04           C
ATOM   6511  C    ALA D 203     24.740  49.855 -31.761  1.00 14.97           C
ATOM   6512  O    ALA D 203     25.832  49.468 -32.182  1.00 15.11           O
ATOM   6513  CB   ALA D 203     23.753  48.231 -30.172  1.00 14.70           C
ATOM   6514  N    SER D 204     23.852  50.495 -32.518  1.00 15.01           N
ATOM   6515  CA   SER D 204     24.114  50.862 -33.901  1.00 15.20           C
ATOM   6516  C    SER D 204     24.469  52.351 -34.044  1.00 14.80           C
ATOM   6517  O    SER D 204     24.745  52.804 -35.148  1.00 14.34           O
ATOM   6518  CB   SER D 204     22.910  50.503 -34.809  1.00 14.98           C
ATOM   6519  OG   SER D 204     21.722  51.209 -34.425  1.00 15.09           O
ATOM   6520  N    SER D 205     24.487  53.083 -32.943  1.00 16.17           N
ATOM   6521  CA   SER D 205     24.821  54.508 -32.971  1.00 17.42           C
ATOM   6522  C    SER D 205     23.886  55.267 -33.924  1.00 18.56           C
ATOM   6523  O    SER D 205     24.321  56.160 -34.672  1.00 18.95           O
ATOM   6524  CB   SER D 205     26.300  54.681 -33.358  1.00 18.00           C
ATOM   6525  OG   SER D 205     27.132  54.125 -32.347  1.00 17.86           O
ATOM   6526  N    THR D 206     22.596  54.895 -33.914  1.00 18.94           N
ATOM   6527  CA   THR D 206     21.620  55.479 -34.841  1.00 19.35           C
ATOM   6528  C    THR D 206     20.512  56.170 -34.071  1.00 20.92           C
ATOM   6529  O    THR D 206     20.196  55.788 -32.952  1.00 18.75           O
ATOM   6530  CB   THR D 206     20.965  54.430 -35.751  1.00 20.40           C
ATOM   6531  OG1  THR D 206     20.372  53.408 -34.953  1.00 19.33           O
ATOM   6532  CG2  THR D 206     21.967  53.822 -36.714  1.00 21.29           C
ATOM   6533  N    LYS D 207     19.951  57.217 -34.679  1.00 22.85           N
ATOM   6534  CA   LYS D 207     18.720  57.839 -34.212  1.00 24.72           C
ATOM   6535  C    LYS D 207     17.867  58.139 -35.447  1.00 24.95           C
ATOM   6536  O    LYS D 207     18.346  58.734 -36.408  1.00 24.67           O
ATOM   6537  CB   LYS D 207     18.998  59.117 -33.409  1.00 26.63           C
ATOM   6538  CG   LYS D 207     17.741  59.628 -32.674  1.00 28.37           C
ATOM   6539  CD   LYS D 207     18.052  60.682 -31.636  1.00 28.98           C
ATOM   6540  CE   LYS D 207     16.974  60.735 -30.571  1.00 30.31           C
ATOM   6541  NZ   LYS D 207     17.320  61.746 -29.510  1.00 33.78           N
ATOM   6542  N    VAL D 208     16.629  57.655 -35.442  1.00 25.45           N
ATOM   6543  CA   VAL D 208     15.737  57.743 -36.587  1.00 26.01           C
ATOM   6544  C    VAL D 208     14.492  58.479 -36.147  1.00 26.21           C
ATOM   6545  O    VAL D 208     14.021  58.252 -35.049  1.00 25.51           O
ATOM   6546  CB   VAL D 208     15.279  56.323 -37.065  1.00 26.43           C
ATOM   6547  CG1  VAL D 208     14.097  56.441 -38.022  1.00 28.29           C
ATOM   6548  CG2  VAL D 208     16.414  55.605 -37.723  1.00 28.73           C
ATOM   6549  N    ASP D 209     13.962  59.330 -37.026  1.00 27.97           N
ATOM   6550  CA   ASP D 209     12.686  60.023 -36.805  1.00 29.09           C
ATOM   6551  C    ASP D 209     11.668  59.532 -37.821  1.00 28.36           C
ATOM   6552  O    ASP D 209     12.014  59.255 -38.987  1.00 30.03           O
ATOM   6553  CB   ASP D 209     12.849  61.542 -36.939  1.00 30.68           C
```

```
ATOM    6554  CG   ASP D 209    13.704   62.144  -35.832  1.00 33.48         C
ATOM    6555  OD1  ASP D 209    13.522   61.797  -34.648  1.00 37.84         O
ATOM    6556  OD2  ASP D 209    14.572   62.982  -36.147  1.00 37.28         O
ATOM    6557  N    LYS D 210    10.424   59.397  -37.370  1.00 25.72         N
ATOM    6558  CA   LYS D 210     9.314   59.059  -38.239  1.00 25.38         C
ATOM    6559  C    LYS D 210     8.168   60.008  -37.903  1.00 25.69         C
ATOM    6560  O    LYS D 210     7.622   59.975  -36.808  1.00 24.40         O
ATOM    6561  CB   LYS D 210     8.891   57.593  -38.074  1.00 25.36         C
ATOM    6562  CG   LYS D 210     7.762   57.111  -38.985  1.00 25.55         C
ATOM    6563  CD   LYS D 210     7.935   57.465  -40.474  1.00 25.77         C
ATOM    6564  CE   LYS D 210     8.924   56.530  -41.180  1.00 24.76         C
ATOM    6565  NZ   LYS D 210     9.296   56.984  -42.565  1.00 26.29         N
ATOM    6566  N    LYS D 211     7.835   60.875  -38.851  1.00 26.53         N
ATOM    6567  CA   LYS D 211     6.660   61.731  -38.706  1.00 27.89         C
ATOM    6568  C    LYS D 211     5.409   60.907  -38.968  1.00 26.10         C
ATOM    6569  O    LYS D 211     5.354   60.122  -39.928  1.00 26.51         O
ATOM    6570  CB   LYS D 211     6.720   62.930  -39.661  1.00 28.35         C
ATOM    6571  CG   LYS D 211     5.522   63.896  -39.482  1.00 30.08         C
ATOM    6572  CD   LYS D 211     5.747   65.259  -40.139  1.00 31.33         C
ATOM    6573  CE   LYS D 211     5.747   65.166  -41.647  1.00 34.11         C
ATOM    6574  NZ   LYS D 211     5.443   66.494  -42.325  1.00 34.56         N
ATOM    6575  N    ILE D 212     4.415   61.087  -38.111  1.00 26.61         N
ATOM    6576  CA   ILE D 212     3.130   60.400  -38.260  1.00 27.38         C
ATOM    6577  C    ILE D 212     2.184   61.289  -39.072  1.00 29.00         C
ATOM    6578  O    ILE D 212     1.871   62.404  -38.662  1.00 29.44         O
ATOM    6579  CB   ILE D 212     2.501   60.061  -36.891  1.00 27.34         C
ATOM    6580  CG1  ILE D 212     3.528   59.337  -35.994  1.00 27.54         C
ATOM    6581  CG2  ILE D 212     1.252   59.189  -37.087  1.00 27.42         C
ATOM    6582  CD1  ILE D 212     4.045   58.042  -36.599  1.00 27.06         C
ATOM    6583  N    VAL D 213     1.769   60.809  -40.236  1.00 31.12         N
ATOM    6584  CA   VAL D 213     0.906   61.608  -41.117  1.00 32.48         C
ATOM    6585  C    VAL D 213    -0.387   60.874  -41.466  1.00 33.75         C
ATOM    6586  O    VAL D 213    -0.403   59.642  -41.586  1.00 32.96         O
ATOM    6587  CB   VAL D 213     1.632   62.032  -42.410  1.00 32.41         C
ATOM    6588  CG1  VAL D 213     2.904   62.765  -42.081  1.00 33.50         C
ATOM    6589  CG2  VAL D 213     1.949   60.856  -43.288  1.00 32.99         C
ATOM    6590  N    PRO D 214    -1.486   61.632  -41.640  1.00 35.64         N
ATOM    6591  CA   PRO D 214    -2.761   61.043  -42.000  1.00 36.45         C
ATOM    6592  C    PRO D 214    -2.692   60.094  -43.174  1.00 37.25         C
ATOM    6593  O    PRO D 214    -1.987   60.343  -44.152  1.00 37.56         O
ATOM    6594  CB   PRO D 214    -3.602   62.263  -42.385  1.00 36.47         C
ATOM    6595  CG   PRO D 214    -3.107   63.302  -41.520  1.00 36.59         C
ATOM    6596  CD   PRO D 214    -1.612   63.093  -41.485  1.00 36.20         C
ATOM    6597  N    ARG D 215    -3.431   59.002  -43.059  1.00 38.41         N
ATOM    6598  CA   ARG D 215    -3.602   58.078  -44.150  1.00 39.32         C
ATOM    6599  C    ARG D 215    -4.591   58.670  -45.165  1.00 39.72         C
ATOM    6600  O    ARG D 215    -5.603   59.268  -44.778  1.00 40.71         O
ATOM    6601  CB   ARG D 215    -4.090   56.737  -43.608  1.00 39.21         C
ATOM    6602  CG   ARG D 215    -3.055   56.056  -42.707  1.00 39.46         C
ATOM    6603  CD   ARG D 215    -3.557   54.745  -42.136  1.00 40.28         C
ATOM    6604  NE   ARG D 215    -3.883   53.767  -43.172  1.00 40.04         N
ATOM    6605  CZ   ARG D 215    -3.028   52.880  -43.691  1.00 42.04         C
ATOM    6606  NH1  ARG D 215    -1.752   52.825  -43.290  1.00 41.32         N
ATOM    6607  NH2  ARG D 215    -3.457   52.029  -44.624  1.00 40.97         N
TER     6608       ARG D 215
ATOM    6609  N    ASP E   1     0.989   33.600   27.725  1.00 15.69         N
ATOM    6610  CA   ASP E   1     1.561   34.968   27.947  1.00 16.07         C
ATOM    6611  C    ASP E   1     2.756   35.227   27.061  1.00 15.61         C
ATOM    6612  O    ASP E   1     3.460   34.317   26.711  1.00 15.53         O
ATOM    6613  CB   ASP E   1     1.885   35.248   29.427  1.00 16.53         C
ATOM    6614  CG   ASP E   1     3.121   34.470   29.991  1.00 18.29         C
ATOM    6615  OD1  ASP E   1     3.617   33.470   29.461  1.00 17.44         O
ATOM    6616  OD2  ASP E   1     3.626   34.875   31.057  1.00 20.15         O
ATOM    6617  N    ILE E   2     2.955   36.477   26.654  1.00 12.85         N
ATOM    6618  CA   ILE E   2     4.154   36.845   25.896  1.00 14.43         C
```

| ATOM | 6619 | C | ILE | E | 2 | 5.343 | 36.910 | 26.852 | 1.00 | 15.72 | C |
|------|------|------|------|------|------|------|------|------|------|------|------|
| ATOM | 6620 | O | ILE | E | 2 | 5.263 | 37.513 | 27.931 | 1.00 | 15.57 | O |
| ATOM | 6621 | CB | ILE | E | 2 | 3.975 | 38.213 | 25.156 | 1.00 | 14.07 | C |
| ATOM | 6622 | CG1 | ILE | E | 2 | 2.900 | 38.094 | 24.063 | 1.00 | 14.53 | C |
| ATOM | 6623 | CG2 | ILE | E | 2 | 5.313 | 38.734 | 24.583 | 1.00 | 13.76 | C |
| ATOM | 6624 | CD1 | ILE | E | 2 | 2.418 | 39.458 | 23.465 | 1.00 | 14.95 | C |
| ATOM | 6625 | N | VAL | E | 3 | 6.450 | 36.300 | 26.438 | 1.00 | 15.40 | N |
| ATOM | 6626 | CA | VAL | E | 3 | 7.662 | 36.296 | 27.218 | 1.00 | 14.60 | C |
| ATOM | 6627 | C | VAL | E | 3 | 8.552 | 37.382 | 26.654 | 1.00 | 14.51 | C |
| ATOM | 6628 | O | VAL | E | 3 | 8.818 | 37.407 | 25.448 | 1.00 | 14.40 | O |
| ATOM | 6629 | CB | VAL | E | 3 | 8.379 | 34.937 | 27.154 | 1.00 | 13.96 | C |
| ATOM | 6630 | CG1 | VAL | E | 3 | 9.661 | 34.980 | 27.952 | 1.00 | 14.30 | C |
| ATOM | 6631 | CG2 | VAL | E | 3 | 7.450 | 33.840 | 27.672 | 1.00 | 14.24 | C |
| ATOM | 6632 | N | MET | E | 4 | 8.987 | 38.281 | 27.532 | 1.00 | 13.60 | N |
| ATOM | 6633 | CA | MET | E | 4 | 9.948 | 39.331 | 27.184 | 1.00 | 14.41 | C |
| ATOM | 6634 | C | MET | E | 4 | 11.290 | 38.975 | 27.808 | 1.00 | 15.15 | C |
| ATOM | 6635 | O | MET | E | 4 | 11.393 | 38.873 | 29.035 | 1.00 | 16.01 | O |
| ATOM | 6636 | CB | MET | E | 4 | 9.441 | 40.694 | 27.718 | 1.00 | 14.24 | C |
| ATOM | 6637 | CG | MET | E | 4 | 8.040 | 41.079 | 27.259 | 1.00 | 14.21 | C |
| ATOM | 6638 | SD | MET | E | 4 | 7.935 | 41.385 | 25.481 | 1.00 | 16.48 | S |
| ATOM | 6639 | CE | MET | E | 4 | 8.948 | 42.833 | 25.309 | 1.00 | 14.41 | C |
| ATOM | 6640 | N | THR | E | 5 | 12.300 | 38.725 | 26.978 | 1.00 | 14.33 | N |
| ATOM | 6641 | CA | THR | E | 5 | 13.593 | 38.202 | 27.435 | 1.00 | 15.02 | C |
| ATOM | 6642 | C | THR | E | 5 | 14.726 | 39.217 | 27.368 | 1.00 | 14.65 | C |
| ATOM | 6643 | O | THR | E | 5 | 15.083 | 39.703 | 26.300 | 1.00 | 14.91 | O |
| ATOM | 6644 | CB | THR | E | 5 | 14.034 | 36.946 | 26.649 | 1.00 | 15.79 | C |
| ATOM | 6645 | OG1 | THR | E | 5 | 13.046 | 35.927 | 26.807 | 1.00 | 16.90 | O |
| ATOM | 6646 | CG2 | THR | E | 5 | 15.377 | 36.440 | 27.161 | 1.00 | 17.94 | C |
| ATOM | 6647 | N | GLN | E | 6 | 15.307 | 39.480 | 28.526 | 1.00 | 14.78 | N |
| ATOM | 6648 | CA | GLN | E | 6 | 16.420 | 40.408 | 28.665 | 1.00 | 17.22 | C |
| ATOM | 6649 | C | GLN | E | 6 | 17.428 | 39.792 | 29.626 | 1.00 | 17.05 | C |
| ATOM | 6650 | O | GLN | E | 6 | 17.055 | 39.154 | 30.587 | 1.00 | 19.00 | O |
| ATOM | 6651 | CB | GLN | E | 6 | 16.023 | 41.725 | 29.352 | 1.00 | 17.49 | C |
| ATOM | 6652 | CG | GLN | E | 6 | 14.704 | 42.310 | 29.114 | 1.00 | 20.26 | C |
| ATOM | 6653 | CD | GLN | E | 6 | 14.554 | 43.622 | 29.898 | 1.00 | 17.82 | C |
| ATOM | 6654 | OE1 | GLN | E | 6 | 13.617 | 43.785 | 30.676 | 1.00 | 15.56 | O |
| ATOM | 6655 | NE2 | GLN | E | 6 | 15.507 | 44.521 | 29.728 | 1.00 | 17.95 | N |
| ATOM | 6656 | N | ALA | E | 7 | 18.695 | 40.053 | 29.391 | 1.00 | 19.06 | N |
| ATOM | 6657 | CA | ALA | E | 7 | 19.744 | 39.673 | 30.320 | 1.00 | 19.65 | C |
| ATOM | 6658 | C | ALA | E | 7 | 19.646 | 40.471 | 31.609 | 1.00 | 20.06 | C |
| ATOM | 6659 | O | ALA | E | 7 | 19.357 | 41.667 | 31.591 | 1.00 | 20.29 | O |
| ATOM | 6660 | CB | ALA | E | 7 | 21.089 | 39.915 | 29.672 | 1.00 | 19.31 | C |
| ATOM | 6661 | N | ALA | E | 8 | 19.985 | 39.837 | 32.727 | 1.00 | 21.33 | N |
| ATOM | 6662 | CA | ALA | E | 8 | 19.965 | 40.518 | 34.026 | 1.00 | 22.61 | C |
| ATOM | 6663 | C | ALA | E | 8 | 21.008 | 41.635 | 34.110 | 1.00 | 24.01 | C |
| ATOM | 6664 | O | ALA | E | 8 | 20.799 | 42.644 | 34.775 | 1.00 | 21.78 | O |
| ATOM | 6665 | CB | ALA | E | 8 | 20.184 | 39.514 | 35.136 | 1.00 | 22.30 | C |
| ATOM | 6666 | N | PHE | E | 9 | 22.131 | 41.454 | 33.427 | 1.00 | 26.22 | N |
| ATOM | 6667 | CA | PHE | E | 9 | 23.239 | 42.378 | 33.526 | 1.00 | 29.35 | C |
| ATOM | 6668 | C | PHE | E | 9 | 23.799 | 42.709 | 32.164 | 1.00 | 31.64 | C |
| ATOM | 6669 | O | PHE | E | 9 | 23.587 | 41.985 | 31.190 | 1.00 | 30.36 | O |
| ATOM | 6670 | CB | PHE | E | 9 | 24.343 | 41.785 | 34.404 | 1.00 | 31.25 | C |
| ATOM | 6671 | CG | PHE | E | 9 | 23.873 | 41.427 | 35.769 | 1.00 | 32.03 | C |
| ATOM | 6672 | CD1 | PHE | E | 9 | 23.580 | 42.424 | 36.688 | 1.00 | 33.39 | C |
| ATOM | 6673 | CD2 | PHE | E | 9 | 23.670 | 40.096 | 36.130 | 1.00 | 33.03 | C |
| ATOM | 6674 | CE1 | PHE | E | 9 | 23.116 | 42.111 | 37.953 | 1.00 | 33.40 | C |
| ATOM | 6675 | CE2 | PHE | E | 9 | 23.207 | 39.776 | 37.398 | 1.00 | 33.41 | C |
| ATOM | 6676 | CZ | PHE | E | 9 | 22.919 | 40.788 | 38.306 | 1.00 | 33.37 | C |
| ATOM | 6677 | N | SER | E | 10 | 24.507 | 43.830 | 32.121 | 1.00 | 33.74 | N |
| ATOM | 6678 | CA | SER | E | 10 | 25.241 | 44.255 | 30.950 | 1.00 | 35.90 | C |
| ATOM | 6679 | C | SER | E | 10 | 26.694 | 44.503 | 31.370 | 1.00 | 36.80 | C |
| ATOM | 6680 | O | SER | E | 10 | 26.994 | 44.552 | 32.559 | 1.00 | 37.27 | O |
| ATOM | 6681 | CB | SER | E | 10 | 24.577 | 45.509 | 30.376 | 1.00 | 36.41 | C |
| ATOM | 6682 | OG | SER | E | 10 | 24.521 | 46.570 | 31.328 | 1.00 | 37.59 | O |
| ATOM | 6683 | N | ASN | E | 11 | 27.604 | 44.626 | 30.405 | 1.00 | 38.45 | N |

| ATOM | 6684 | CA | ASN | E | 11 | 29.003 | 44.951 | 30.727 | 1.00 | 39.01 | C |
|------|------|-----|-----|---|----|--------|--------|--------|------|-------|---|
| ATOM | 6685 | C | ASN | E | 11 | 29.091 | 46.360 | 31.291 | 1.00 | 37.33 | C |
| ATOM | 6686 | O | ASN | E | 11 | 28.477 | 47.270 | 30.731 | 1.00 | 37.52 | O |
| ATOM | 6687 | CB | ASN | E | 11 | 29.894 | 44.873 | 29.478 | 1.00 | 40.98 | C |
| ATOM | 6688 | CG | ASN | E | 11 | 30.192 | 43.445 | 29.053 | 1.00 | 42.42 | C |
| ATOM | 6689 | OD1 | ASN | E | 11 | 30.526 | 42.584 | 29.881 | 1.00 | 44.81 | O |
| ATOM | 6690 | ND2 | ASN | E | 11 | 30.091 | 43.190 | 27.749 | 1.00 | 44.20 | N |
| ATOM | 6691 | N | PRO | E | 12 | 29.868 | 46.557 | 32.377 | 1.00 | 35.65 | N |
| ATOM | 6692 | CA | PRO | E | 12 | 30.009 | 47.891 | 32.946 | 1.00 | 34.32 | C |
| ATOM | 6693 | C | PRO | E | 12 | 30.500 | 48.835 | 31.875 | 1.00 | 33.44 | C |
| ATOM | 6694 | O | PRO | E | 12 | 31.354 | 48.453 | 31.093 | 1.00 | 34.22 | O |
| ATOM | 6695 | CB | PRO | E | 12 | 31.073 | 47.718 | 34.033 | 1.00 | 34.60 | C |
| ATOM | 6696 | CG | PRO | E | 12 | 31.120 | 46.294 | 34.318 | 1.00 | 35.04 | C |
| ATOM | 6697 | CD | PRO | E | 12 | 30.677 | 45.564 | 33.105 | 1.00 | 35.13 | C |
| ATOM | 6698 | N | VAL | E | 13 | 29.955 | 50.042 | 31.839 | 1.00 | 31.92 | N |
| ATOM | 6699 | CA | VAL | E | 13 | 30.199 | 50.978 | 30.761 | 1.00 | 31.48 | C |
| ATOM | 6700 | C | VAL | E | 13 | 30.919 | 52.211 | 31.289 | 1.00 | 30.47 | C |
| ATOM | 6701 | O | VAL | E | 13 | 30.539 | 52.773 | 32.311 | 1.00 | 29.21 | O |
| ATOM | 6702 | CB | VAL | E | 13 | 28.858 | 51.333 | 30.058 | 1.00 | 31.65 | C |
| ATOM | 6703 | CG1 | VAL | E | 13 | 28.930 | 52.650 | 29.326 | 1.00 | 32.03 | C |
| ATOM | 6704 | CG2 | VAL | E | 13 | 28.487 | 50.221 | 29.100 | 1.00 | 32.04 | C |
| ATOM | 6705 | N | THR | E | 14 | 31.970 | 52.624 | 30.583 | 1.00 | 30.38 | N |
| ATOM | 6706 | CA | THR | E | 14 | 32.763 | 53.762 | 30.995 | 1.00 | 30.25 | C |
| ATOM | 6707 | C | THR | E | 14 | 31.941 | 55.003 | 30.769 | 1.00 | 30.45 | C |
| ATOM | 6708 | O | THR | E | 14 | 31.245 | 55.099 | 29.755 | 1.00 | 29.10 | O |
| ATOM | 6709 | CB | THR | E | 14 | 34.091 | 53.840 | 30.197 | 1.00 | 31.46 | C |
| ATOM | 6710 | OG1 | THR | E | 14 | 34.977 | 52.810 | 30.652 | 1.00 | 31.54 | O |
| ATOM | 6711 | CG2 | THR | E | 14 | 34.767 | 55.187 | 30.380 | 1.00 | 31.85 | C |
| ATOM | 6712 | N | LEU | E | 15 | 32.015 | 55.961 | 31.693 | 1.00 | 30.82 | N |
| ATOM | 6713 | CA | LEU | E | 15 | 31.247 | 57.194 | 31.541 | 1.00 | 31.38 | C |
| ATOM | 6714 | C | LEU | E | 15 | 31.603 | 57.894 | 30.242 | 1.00 | 31.20 | C |
| ATOM | 6715 | O | LEU | E | 15 | 32.752 | 57.914 | 29.825 | 1.00 | 30.91 | O |
| ATOM | 6716 | CB | LEU | E | 15 | 31.458 | 58.157 | 32.710 | 1.00 | 32.26 | C |
| ATOM | 6717 | CG | LEU | E | 15 | 30.825 | 57.868 | 34.077 | 1.00 | 34.49 | C |
| ATOM | 6718 | CD1 | LEU | E | 15 | 31.063 | 59.063 | 34.979 | 1.00 | 35.34 | C |
| ATOM | 6719 | CD2 | LEU | E | 15 | 29.345 | 57.570 | 34.009 | 1.00 | 35.62 | C |
| ATOM | 6720 | N | GLY | E | 16 | 30.601 | 58.463 | 29.587 | 1.00 | 31.42 | N |
| ATOM | 6721 | CA | GLY | E | 16 | 30.815 | 59.131 | 28.311 | 1.00 | 31.77 | C |
| ATOM | 6722 | C | GLY | E | 16 | 30.791 | 58.192 | 27.117 | 1.00 | 31.06 | C |
| ATOM | 6723 | O | GLY | E | 16 | 30.754 | 58.655 | 25.986 | 1.00 | 33.12 | O |
| ATOM | 6724 | N | THR | E | 17 | 30.798 | 56.884 | 27.348 | 1.00 | 30.09 | N |
| ATOM | 6725 | CA | THR | E | 17 | 30.692 | 55.922 | 26.249 | 1.00 | 29.43 | C |
| ATOM | 6726 | C | THR | E | 17 | 29.262 | 55.356 | 26.145 | 1.00 | 28.12 | C |
| ATOM | 6727 | O | THR | E | 17 | 28.406 | 55.653 | 26.981 | 1.00 | 27.32 | O |
| ATOM | 6728 | CB | THR | E | 17 | 31.722 | 54.784 | 26.382 | 1.00 | 29.43 | C |
| ATOM | 6729 | OG1 | THR | E | 17 | 31.376 | 53.912 | 27.461 | 1.00 | 30.38 | O |
| ATOM | 6730 | CG2 | THR | E | 17 | 33.112 | 55.350 | 26.615 | 1.00 | 30.84 | C |
| ATOM | 6731 | N | SER | E | 18 | 29.027 | 54.540 | 25.113 | 1.00 | 26.15 | N |
| ATOM | 6732 | CA | SER | E | 18 | 27.692 | 54.063 | 24.788 | 1.00 | 24.59 | C |
| ATOM | 6733 | C | SER | E | 18 | 27.399 | 52.729 | 25.446 | 1.00 | 22.65 | C |
| ATOM | 6734 | O | SER | E | 18 | 28.286 | 51.916 | 25.584 | 1.00 | 22.89 | O |
| ATOM | 6735 | CB | SER | E | 18 | 27.554 | 53.895 | 23.284 | 1.00 | 24.56 | C |
| ATOM | 6736 | OG | SER | E | 18 | 28.225 | 52.698 | 22.910 | 1.00 | 27.60 | O |
| ATOM | 6737 | N | ALA | E | 19 | 26.152 | 52.538 | 25.868 | 1.00 | 21.23 | N |
| ATOM | 6738 | CA | ALA | E | 19 | 25.652 | 51.256 | 26.364 | 1.00 | 20.41 | C |
| ATOM | 6739 | C | ALA | E | 19 | 24.611 | 50.712 | 25.388 | 1.00 | 20.82 | C |
| ATOM | 6740 | O | ALA | E | 19 | 23.953 | 51.494 | 24.704 | 1.00 | 22.86 | O |
| ATOM | 6741 | CB | ALA | E | 19 | 25.009 | 51.456 | 27.713 | 1.00 | 21.19 | C |
| ATOM | 6742 | N | SER | E | 20 | 24.437 | 49.393 | 25.377 | 1.00 | 21.27 | N |
| ATOM | 6743 | CA | SER | E | 20 | 23.478 | 48.698 | 24.506 | 1.00 | 21.46 | C |
| ATOM | 6744 | C | SER | E | 20 | 22.757 | 47.624 | 25.314 | 1.00 | 21.33 | C |
| ATOM | 6745 | O | SER | E | 20 | 23.408 | 46.765 | 25.931 | 1.00 | 21.67 | O |
| ATOM | 6746 | CB | SER | E | 20 | 24.207 | 48.059 | 23.319 | 1.00 | 22.16 | C |
| ATOM | 6747 | OG | SER | E | 20 | 23.296 | 47.610 | 22.342 | 1.00 | 24.26 | O |
| ATOM | 6748 | N | ILE | E | 21 | 21.427 | 47.715 | 25.367 | 1.00 | 19.51 | N |

```
ATOM   6749  CA   ILE E  21      20.592  46.732  26.067  1.00 20.00           C
ATOM   6750  C    ILE E  21      19.634  46.058  25.084  1.00 19.05           C
ATOM   6751  O    ILE E  21      18.896  46.735  24.385  1.00 18.95           O
ATOM   6752  CB   ILE E  21      19.852  47.409  27.243  1.00 20.10           C
ATOM   6753  CG1  ILE E  21      20.880  47.785  28.325  1.00 22.06           C
ATOM   6754  CG2  ILE E  21      18.792  46.487  27.869  1.00 20.67           C
ATOM   6755  CD1  ILE E  21      20.308  48.624  29.445  1.00 21.51           C
ATOM   6756  N    SER E  22      19.668  44.724  25.030  1.00 19.85           N
ATOM   6757  CA   SER E  22      18.777  43.944  24.186  1.00 19.98           C
ATOM   6758  C    SER E  22      17.538  43.426  24.880  1.00 19.43           C
ATOM   6759  O    SER E  22      17.526  43.149  26.078  1.00 18.47           O
ATOM   6760  CB   SER E  22      19.505  42.724  23.635  1.00 21.72           C
ATOM   6761  OG   SER E  22      20.713  43.108  23.037  1.00 26.11           O
ATOM   6762  N    CYS E  23      16.502  43.216  24.079  1.00 18.09           N
ATOM   6763  CA   CYS E  23      15.308  42.518  24.508  1.00 16.92           C
ATOM   6764  C    CYS E  23      14.759  41.745  23.316  1.00 16.28           C
ATOM   6765  O    CYS E  23      14.967  42.114  22.163  1.00 14.46           O
ATOM   6766  CB   CYS E  23      14.272  43.496  25.091  1.00 18.39           C
ATOM   6767  SG   CYS E  23      12.652  42.783  25.550  1.00 22.14           S
ATOM   6768  N    ARG E  24      14.099  40.639  23.603  1.00 16.03           N
ATOM   6769  CA   ARG E  24      13.315  39.964  22.596  1.00 18.27           C
ATOM   6770  C    ARG E  24      11.983  39.548  23.147  1.00 14.70           C
ATOM   6771  O    ARG E  24      11.833  39.366  24.361  1.00 15.19           O
ATOM   6772  CB   ARG E  24      14.046  38.775  21.998  1.00 18.57           C
ATOM   6773  CG   ARG E  24      14.422  37.703  22.962  1.00 23.52           C
ATOM   6774  CD   ARG E  24      15.185  36.609  22.235  1.00 26.18           C
ATOM   6775  NE   ARG E  24      16.092  35.927  23.165  1.00 30.46           N
ATOM   6776  CZ   ARG E  24      15.921  34.712  23.670  1.00 32.86           C
ATOM   6777  NH1  ARG E  24      14.871  33.954  23.339  1.00 35.85           N
ATOM   6778  NH2  ARG E  24      16.841  34.239  24.508  1.00 34.20           N
ATOM   6779  N    SER E  25      11.025  39.409  22.233  1.00 13.44           N
ATOM   6780  CA   SER E  25       9.659  39.014  22.539  1.00 12.34           C
ATOM   6781  C    SER E  25       9.303  37.714  21.830  1.00 13.77           C
ATOM   6782  O    SER E  25       9.725  37.494  20.687  1.00 14.32           O
ATOM   6783  CB  ASER E  25       8.706  40.111  22.058  0.50 11.64           C
ATOM   6784  CB  BSER E  25       8.688  40.107  22.113  0.50 12.19           C
ATOM   6785  OG  ASER E  25       7.349  39.709  22.155  0.50  8.83           O
ATOM   6786  OG  BSER E  25       8.636  40.201  20.711  0.50 11.28           O
ATOM   6787  N    SER E  26       8.535  36.857  22.504  1.00 14.26           N
ATOM   6788  CA   SER E  26       8.103  35.589  21.961  1.00 13.99           C
ATOM   6789  C    SER E  26       7.020  35.717  20.900  1.00 16.34           C
ATOM   6790  O    SER E  26       6.765  34.747  20.179  1.00 15.84           O
ATOM   6791  CB   SER E  26       7.606  34.681  23.070  1.00 13.66           C
ATOM   6792  OG   SER E  26       6.413  35.160  23.663  1.00 12.39           O
ATOM   6793  N    LYS E  27       6.384  36.890  20.826  1.00 17.49           N
ATOM   6794  CA   LYS E  27       5.378  37.205  19.813  1.00 19.67           C
ATOM   6795  C    LYS E  27       5.777  38.493  19.137  1.00 19.03           C
ATOM   6796  O    LYS E  27       6.298  39.409  19.790  1.00 17.86           O
ATOM   6797  CB   LYS E  27       3.994  37.425  20.427  1.00 20.64           C
ATOM   6798  CG   LYS E  27       3.294  36.185  20.967  1.00 25.09           C
ATOM   6799  CD   LYS E  27       1.776  36.399  21.137  1.00 24.36           C
ATOM   6800  CE   LYS E  27       1.158  35.482  22.230  1.00 26.43           C
ATOM   6801  NZ   LYS E  27      -0.306  35.744  22.447  1.00 27.17           N
ATOM   6802  N    SER E  27A      5.531  38.590  17.826  1.00 18.10           N
ATOM   6803  CA   SER E  27A      5.790  39.845  17.136  1.00 17.19           C
ATOM   6804  C    SER E  27A      4.898  40.953  17.690  1.00 17.01           C
ATOM   6805  O    SER E  27A      3.698  40.763  17.914  1.00 17.37           O
ATOM   6806  CB   SER E  27A      5.605  39.728  15.625  1.00 17.81           C
ATOM   6807  OG   SER E  27A      5.654  41.017  15.014  1.00 17.03           O
ATOM   6808  N    LEU E  27B      5.515  42.098  17.940  1.00 17.06           N
ATOM   6809  CA   LEU E  27B      4.834  43.291  18.440  1.00 15.26           C
ATOM   6810  C    LEU E  27B      4.556  44.333  17.332  1.00 16.40           C
ATOM   6811  O    LEU E  27B      4.075  45.430  17.614  1.00 14.66           O
ATOM   6812  CB   LEU E  27B      5.687  43.919  19.538  1.00 15.47           C
ATOM   6813  CG   LEU E  27B      6.155  42.988  20.656  1.00 12.67           C
```

| ATOM | 6814 | CD1 | LEU | E | 27B | 6.899 | 43.772 | 21.728 | 1.00 | 13.97 | C |
|------|------|-----|-----|---|-----|-------|--------|--------|------|-------|---|
| ATOM | 6815 | CD2 | LEU | E | 27B | 4.994 | 42.190 | 21.288 | 1.00 | 15.21 | C |
| ATOM | 6816 | N | LEU | E | 27C | 4.892 | 43.990 | 16.089 | 1.00 | 16.60 | N |
| ATOM | 6817 | CA | LEU | E | 27C | 4.606 | 44.849 | 14.942 | 1.00 | 16.45 | C |
| ATOM | 6818 | C | LEU | E | 27C | 3.143 | 44.618 | 14.552 | 1.00 | 17.34 | C |
| ATOM | 6819 | O | LEU | E | 27C | 2.727 | 43.501 | 14.218 | 1.00 | 17.40 | O |
| ATOM | 6820 | CB | LEU | E | 27C | 5.569 | 44.569 | 13.778 | 1.00 | 17.37 | C |
| ATOM | 6821 | CG | LEU | E | 27C | 5.240 | 45.313 | 12.460 | 1.00 | 16.18 | C |
| ATOM | 6822 | CD1 | LEU | E | 27C | 5.160 | 46.794 | 12.663 | 1.00 | 14.62 | C |
| ATOM | 6823 | CD2 | LEU | E | 27C | 6.236 | 44.937 | 11.324 | 1.00 | 18.38 | C |
| ATOM | 6824 | N | HIS | E | 27D | 2.356 | 45.680 | 14.664 | 1.00 | 18.55 | N |
| ATOM | 6825 | CA | HIS | E | 27D | 0.933 | 45.631 | 14.391 | 1.00 | 20.83 | C |
| ATOM | 6826 | C | HIS | E | 27D | 0.660 | 46.032 | 12.936 | 1.00 | 20.89 | C |
| ATOM | 6827 | O | HIS | E | 27D | 1.475 | 46.689 | 12.296 | 1.00 | 19.25 | O |
| ATOM | 6828 | CB | HIS | E | 27D | 0.233 | 46.601 | 15.341 | 1.00 | 20.76 | C |
| ATOM | 6829 | CG | HIS | E | 27D | -1.263 | 46.504 | 15.339 | 1.00 | 22.52 | C |
| ATOM | 6830 | ND1 | HIS | E | 27D | -2.052 | 47.119 | 14.389 | 1.00 | 23.20 | N |
| ATOM | 6831 | CD2 | HIS | E | 27D | -2.117 | 45.932 | 16.218 | 1.00 | 23.50 | C |
| ATOM | 6832 | CE1 | HIS | E | 27D | -3.326 | 46.891 | 14.660 | 1.00 | 24.58 | C |
| ATOM | 6833 | NE2 | HIS | E | 27D | -3.393 | 46.171 | 15.766 | 1.00 | 24.77 | N |
| ATOM | 6834 | N | SER | E | 27E | -0.497 | 45.626 | 12.417 | 1.00 | 22.75 | N |
| ATOM | 6835 | CA | SER | E | 27E | -0.915 | 46.005 | 11.061 | 1.00 | 23.75 | C |
| ATOM | 6836 | C | SER | E | 27E | -0.943 | 47.504 | 10.820 | 1.00 | 23.57 | C |
| ATOM | 6837 | O | SER | E | 27E | -0.811 | 47.950 | 9.677 | 1.00 | 24.13 | O |
| ATOM | 6838 | CB | SER | E | 27E | -2.284 | 45.403 | 10.719 | 1.00 | 25.00 | C |
| ATOM | 6839 | OG | SER | E | 27E | -3.261 | 45.777 | 11.666 | 1.00 | 27.89 | O |
| ATOM | 6840 | N | ASP | E | 28 | -1.100 | 48.292 | 11.884 | 1.00 | 22.43 | N |
| ATOM | 6841 | CA | ASP | E | 28 | -1.056 | 49.751 | 11.772 | 1.00 | 21.35 | C |
| ATOM | 6842 | C | ASP | E | 28 | 0.369 | 50.290 | 11.610 | 1.00 | 20.68 | C |
| ATOM | 6843 | O | ASP | E | 28 | 0.576 | 51.501 | 11.489 | 1.00 | 22.11 | O |
| ATOM | 6844 | CB | ASP | E | 28 | -1.785 | 50.411 | 12.948 | 1.00 | 21.85 | C |
| ATOM | 6845 | CG | ASP | E | 28 | -1.144 | 50.137 | 14.282 | 1.00 | 21.75 | C |
| ATOM | 6846 | OD1 | ASP | E | 28 | 0.018 | 49.724 | 14.290 | 1.00 | 22.58 | O |
| ATOM | 6847 | OD2 | ASP | E | 28 | -1.811 | 50.345 | 15.329 | 1.00 | 21.87 | O |
| ATOM | 6848 | N | GLY | E | 29 | 1.351 | 49.396 | 11.586 | 1.00 | 18.66 | N |
| ATOM | 6849 | CA | GLY | E | 29 | 2.716 | 49.782 | 11.301 | 1.00 | 17.68 | C |
| ATOM | 6850 | C | GLY | E | 29 | 3.502 | 50.194 | 12.534 | 1.00 | 17.63 | C |
| ATOM | 6851 | O | GLY | E | 29 | 4.697 | 50.496 | 12.427 | 1.00 | 17.66 | O |
| ATOM | 6852 | N | ILE | E | 30 | 2.845 | 50.196 | 13.698 | 1.00 | 16.11 | N |
| ATOM | 6853 | CA | ILE | E | 30 | 3.509 | 50.512 | 14.960 | 1.00 | 16.42 | C |
| ATOM | 6854 | C | ILE | E | 30 | 4.018 | 49.244 | 15.596 | 1.00 | 13.70 | C |
| ATOM | 6855 | O | ILE | E | 30 | 3.329 | 48.241 | 15.602 | 1.00 | 14.69 | O |
| ATOM | 6856 | CB | ILE | E | 30 | 2.569 | 51.298 | 15.906 | 1.00 | 17.02 | C |
| ATOM | 6857 | CG1 | ILE | E | 30 | 2.330 | 52.679 | 15.289 | 1.00 | 18.98 | C |
| ATOM | 6858 | CG2 | ILE | E | 30 | 3.143 | 51.406 | 17.339 | 1.00 | 15.57 | C |
| ATOM | 6859 | CD1 | ILE | E | 30 | 1.282 | 53.529 | 16.051 | 1.00 | 19.19 | C |
| ATOM | 6860 | N | THR | E | 31 | 5.237 | 49.310 | 16.129 | 1.00 | 14.21 | N |
| ATOM | 6861 | CA | THR | E | 31 | 5.790 | 48.230 | 16.935 | 1.00 | 13.66 | C |
| ATOM | 6862 | C | THR | E | 31 | 5.579 | 48.636 | 18.397 | 1.00 | 13.98 | C |
| ATOM | 6863 | O | THR | E | 31 | 6.161 | 49.587 | 18.858 | 1.00 | 14.13 | O |
| ATOM | 6864 | CB | THR | E | 31 | 7.269 | 47.988 | 16.634 | 1.00 | 15.33 | C |
| ATOM | 6865 | OG1 | THR | E | 31 | 7.414 | 47.648 | 15.250 | 1.00 | 13.93 | O |
| ATOM | 6866 | CG2 | THR | E | 31 | 7.830 | 46.852 | 17.495 | 1.00 | 14.24 | C |
| ATOM | 6867 | N | TYR | E | 32 | 4.736 | 47.880 | 19.091 | 1.00 | 15.05 | N |
| ATOM | 6868 | CA | TYR | E | 32 | 4.302 | 48.216 | 20.450 | 1.00 | 15.19 | C |
| ATOM | 6869 | C | TYR | E | 32 | 5.269 | 47.704 | 21.530 | 1.00 | 14.11 | C |
| ATOM | 6870 | O | TYR | E | 32 | 4.914 | 46.865 | 22.358 | 1.00 | 15.57 | O |
| ATOM | 6871 | CB | TYR | E | 32 | 2.865 | 47.731 | 20.670 | 1.00 | 17.04 | C |
| ATOM | 6872 | CG | TYR | E | 32 | 1.853 | 48.573 | 19.907 | 1.00 | 16.31 | C |
| ATOM | 6873 | CD1 | TYR | E | 32 | 1.530 | 48.281 | 18.586 | 1.00 | 17.41 | C |
| ATOM | 6874 | CD2 | TYR | E | 32 | 1.274 | 49.701 | 20.494 | 1.00 | 18.34 | C |
| ATOM | 6875 | CE1 | TYR | E | 32 | 0.608 | 49.065 | 17.870 | 1.00 | 18.44 | C |
| ATOM | 6876 | CE2 | TYR | E | 32 | 0.364 | 50.498 | 19.783 | 1.00 | 17.44 | C |
| ATOM | 6877 | CZ | TYR | E | 32 | 0.037 | 50.174 | 18.475 | 1.00 | 18.48 | C |
| ATOM | 6878 | OH | TYR | E | 32 | -0.874 | 50.961 | 17.773 | 1.00 | 18.97 | O |

```
ATOM   6879   N     LEU  E  33      6.467   48.274   21.511   1.00  14.39        N
ATOM   6880   CA    LEU  E  33      7.555   47.954   22.430   1.00  13.39        C
ATOM   6881   C     LEU  E  33      7.871   49.233   23.195   1.00  13.63        C
ATOM   6882   O     LEU  E  33      7.930   50.328   22.624   1.00  14.79        O
ATOM   6883   CB    LEU  E  33      8.773   47.456   21.635   1.00  13.57        C
ATOM   6884   CG    LEU  E  33     10.034   47.000   22.391   1.00  13.06        C
ATOM   6885   CD1   LEU  E  33     10.849   48.162   22.947   1.00  14.75        C
ATOM   6886   CD2   LEU  E  33      9.690   45.928   23.457   1.00  13.15        C
ATOM   6887   N     TYR  E  34      8.041   49.061   24.503   1.00  14.04        N
ATOM   6888   CA    TYR  E  34      8.209   50.155   25.467   1.00  13.07        C
ATOM   6889   C     TYR  E  34      9.410   49.831   26.329   1.00  13.78        C
ATOM   6890   O     TYR  E  34      9.655   48.675   26.655   1.00  12.15        O
ATOM   6891   CB    TYR  E  34      6.952   50.258   26.344   1.00  13.43        C
ATOM   6892   CG    TYR  E  34      5.725   50.670   25.589   1.00  14.93        C
ATOM   6893   CD1   TYR  E  34      5.093   49.788   24.714   1.00  15.00        C
ATOM   6894   CD2   TYR  E  34      5.159   51.935   25.762   1.00  15.20        C
ATOM   6895   CE1   TYR  E  34      3.964   50.165   24.002   1.00  15.80        C
ATOM   6896   CE2   TYR  E  34      4.026   52.306   25.060   1.00  14.42        C
ATOM   6897   CZ    TYR  E  34      3.438   51.415   24.192   1.00  15.38        C
ATOM   6898   OH    TYR  E  34      2.314   51.765   23.531   1.00  15.65        O
ATOM   6899   N     TRP  E  35     10.169   50.860   26.685   1.00  13.67        N
ATOM   6900   CA    TRP  E  35     11.228   50.740   27.675   1.00  13.18        C
ATOM   6901   C     TRP  E  35     10.903   51.649   28.891   1.00  14.02        C
ATOM   6902   O     TRP  E  35     10.489   52.806   28.719   1.00  14.28        O
ATOM   6903   CB    TRP  E  35     12.556   51.183   27.100   1.00  13.47        C
ATOM   6904   CG    TRP  E  35     13.115   50.346   26.006   1.00  13.20        C
ATOM   6905   CD1   TRP  E  35     13.071   50.594   24.671   1.00  14.51        C
ATOM   6906   CD2   TRP  E  35     13.870   49.145   26.169   1.00  14.95        C
ATOM   6907   NE1   TRP  E  35     13.730   49.607   23.979   1.00  14.39        N
ATOM   6908   CE2   TRP  E  35     14.245   48.710   24.883   1.00  14.46        C
ATOM   6909   CE3   TRP  E  35     14.285   48.408   27.284   1.00  15.32        C
ATOM   6910   CZ2   TRP  E  35     14.983   47.544   24.681   1.00  15.73        C
ATOM   6911   CZ3   TRP  E  35     15.001   47.264   27.081   1.00  15.66        C
ATOM   6912   CH2   TRP  E  35     15.369   46.853   25.799   1.00  14.56        C
ATOM   6913   N     TYR  E  36     11.124   51.104   30.080   1.00  12.47        N
ATOM   6914   CA    TYR  E  36     11.007   51.801   31.364   1.00  13.64        C
ATOM   6915   C     TYR  E  36     12.351   51.744   32.059   1.00  15.86        C
ATOM   6916   O     TYR  E  36     13.107   50.780   31.908   1.00  16.71        O
ATOM   6917   CB    TYR  E  36      9.942   51.138   32.240   1.00  13.65        C
ATOM   6918   CG    TYR  E  36      8.585   51.166   31.622   1.00  14.71        C
ATOM   6919   CD1   TYR  E  36      7.768   52.283   31.782   1.00  14.56        C
ATOM   6920   CD2   TYR  E  36      8.113   50.107   30.852   1.00  16.23        C
ATOM   6921   CE1   TYR  E  36      6.545   52.363   31.185   1.00  15.52        C
ATOM   6922   CE2   TYR  E  36      6.859   50.167   30.263   1.00  14.41        C
ATOM   6923   CZ    TYR  E  36      6.085   51.310   30.427   1.00  14.17        C
ATOM   6924   OH    TYR  E  36      4.867   51.414   29.849   1.00  14.89        O
ATOM   6925   N     LEU  E  37     12.661   52.786   32.815   1.00  16.20        N
ATOM   6926   CA    LEU  E  37     13.817   52.784   33.697   1.00  15.66        C
ATOM   6927   C     LEU  E  37     13.330   52.900   35.123   1.00  15.13        C
ATOM   6928   O     LEU  E  37     12.476   53.733   35.410   1.00  13.66        O
ATOM   6929   CB    LEU  E  37     14.709   53.962   33.381   1.00  16.75        C
ATOM   6930   CG    LEU  E  37     15.865   54.300   34.302   1.00  16.04        C
ATOM   6931   CD1   LEU  E  37     16.751   53.083   34.552   1.00  17.62        C
ATOM   6932   CD2   LEU  E  37     16.688   55.446   33.717   1.00  16.78        C
ATOM   6933   N     GLN  E  38     13.860   52.050   36.000   1.00  14.07        N
ATOM   6934   CA    GLN  E  38     13.632   52.138   37.428   1.00  16.36        C
ATOM   6935   C     GLN  E  38     14.962   52.412   38.131   1.00  17.97        C
ATOM   6936   O     GLN  E  38     15.779   51.518   38.306   1.00  16.52        O
ATOM   6937   CB    GLN  E  38     12.995   50.855   37.959   1.00  16.28        C
ATOM   6938   CG    GLN  E  38     12.617   50.943   39.426   1.00  16.20        C
ATOM   6939   CD    GLN  E  38     11.809   49.771   39.898   1.00  17.45        C
ATOM   6940   OE1   GLN  E  38     12.018   48.635   39.451   1.00  19.49        O
ATOM   6941   NE2   GLN  E  38     10.887   50.029   40.824   1.00  15.21        N
ATOM   6942   N     LYS  E  39     15.180   53.679   38.494   1.00  19.39        N
ATOM   6943   CA    LYS  E  39     16.379   54.079   39.225   1.00  20.55        C
```

| ATOM | 6944 | C | LYS | E | 39 | 16.303 | 53.656 | 40.680 | 1.00 | 22.60 | C |
|------|------|------|-----|---|----|--------|--------|--------|------|-------|---|
| ATOM | 6945 | O | LYS | E | 39 | 15.214 | 53.516 | 41.223 | 1.00 | 22.27 | O |
| ATOM | 6946 | CB | LYS | E | 39 | 16.566 | 55.594 | 39.147 | 1.00 | 19.36 | C |
| ATOM | 6947 | CG | LYS | E | 39 | 16.936 | 56.066 | 37.792 | 1.00 | 20.93 | C |
| ATOM | 6948 | CD | LYS | E | 39 | 16.927 | 57.572 | 37.695 | 1.00 | 20.92 | C |
| ATOM | 6949 | CE | LYS | E | 39 | 17.378 | 58.036 | 36.338 | 1.00 | 23.40 | C |
| ATOM | 6950 | NZ | LYS | E | 39 | 17.834 | 59.468 | 36.306 | 1.00 | 22.86 | N |
| ATOM | 6951 | N | PRO | E | 40 | 17.471 | 53.445 | 41.321 | 1.00 | 25.03 | N |
| ATOM | 6952 | CA | PRO | E | 40 | 17.478 | 52.926 | 42.687 | 1.00 | 26.48 | C |
| ATOM | 6953 | C | PRO | E | 40 | 16.549 | 53.722 | 43.614 | 1.00 | 27.02 | C |
| ATOM | 6954 | O | PRO | E | 40 | 16.620 | 54.959 | 43.648 | 1.00 | 28.19 | O |
| ATOM | 6955 | CB | PRO | E | 40 | 18.947 | 53.092 | 43.115 | 1.00 | 27.04 | C |
| ATOM | 6956 | CG | PRO | E | 40 | 19.724 | 53.060 | 41.832 | 1.00 | 27.40 | C |
| ATOM | 6957 | CD | PRO | E | 40 | 18.829 | 53.675 | 40.800 | 1.00 | 26.14 | C |
| ATOM | 6958 | N | GLY | E | 41 | 15.667 | 53.014 | 44.316 | 1.00 | 27.52 | N |
| ATOM | 6959 | CA | GLY | E | 41 | 14.723 | 53.617 | 45.248 | 1.00 | 28.65 | C |
| ATOM | 6960 | C | GLY | E | 41 | 13.517 | 54.312 | 44.637 | 1.00 | 29.02 | C |
| ATOM | 6961 | O | GLY | E | 41 | 12.692 | 54.871 | 45.366 | 1.00 | 29.77 | O |
| ATOM | 6962 | N | GLN | E | 42 | 13.408 | 54.285 | 43.307 | 1.00 | 27.60 | N |
| ATOM | 6963 | CA | GLN | E | 42 | 12.362 | 55.001 | 42.609 | 1.00 | 27.24 | C |
| ATOM | 6964 | C | GLN | E | 42 | 11.369 | 54.018 | 41.963 | 1.00 | 24.70 | C |
| ATOM | 6965 | O | GLN | E | 42 | 11.603 | 52.796 | 41.894 | 1.00 | 23.27 | O |
| ATOM | 6966 | CB | GLN | E | 42 | 12.965 | 55.914 | 41.537 | 1.00 | 27.43 | C |
| ATOM | 6967 | CG | GLN | E | 42 | 13.885 | 57.041 | 42.019 | 1.00 | 30.02 | C |
| ATOM | 6968 | CD | GLN | E | 42 | 14.231 | 58.039 | 40.894 | 1.00 | 30.80 | C |
| ATOM | 6969 | OE1 | GLN | E | 42 | 13.768 | 57.903 | 39.746 | 1.00 | 34.03 | O |
| ATOM | 6970 | NE2 | GLN | E | 42 | 15.050 | 59.053 | 41.220 | 1.00 | 33.68 | N |
| ATOM | 6971 | N | SER | E | 43 | 10.246 | 54.556 | 41.500 | 1.00 | 22.60 | N |
| ATOM | 6972 | CA | SER | E | 43 | 9.302 | 53.774 | 40.697 | 1.00 | 20.89 | C |
| ATOM | 6973 | C | SER | E | 43 | 9.845 | 53.669 | 39.273 | 1.00 | 18.38 | C |
| ATOM | 6974 | O | SER | E | 43 | 10.786 | 54.382 | 38.915 | 1.00 | 17.21 | O |
| ATOM | 6975 | CB | SER | E | 43 | 7.930 | 54.445 | 40.657 | 1.00 | 21.59 | C |
| ATOM | 6976 | OG | SER | E | 43 | 7.431 | 54.646 | 41.968 | 1.00 | 22.62 | O |
| ATOM | 6977 | N | PRO | E | 44 | 9.287 | 52.748 | 38.462 | 1.00 | 16.79 | N |
| ATOM | 6978 | CA | PRO | E | 44 | 9.648 | 52.772 | 37.037 | 1.00 | 16.47 | C |
| ATOM | 6979 | C | PRO | E | 44 | 9.110 | 54.032 | 36.382 | 1.00 | 17.05 | C |
| ATOM | 6980 | O | PRO | E | 44 | 8.106 | 54.575 | 36.854 | 1.00 | 15.87 | O |
| ATOM | 6981 | CB | PRO | E | 44 | 8.924 | 51.539 | 36.470 | 1.00 | 16.38 | C |
| ATOM | 6982 | CG | PRO | E | 44 | 8.705 | 50.667 | 37.669 | 1.00 | 15.26 | C |
| ATOM | 6983 | CD | PRO | E | 44 | 8.402 | 51.615 | 38.779 | 1.00 | 17.61 | C |
| ATOM | 6984 | N | HIS | E | 45 | 9.773 | 54.502 | 35.332 | 1.00 | 16.50 | N |
| ATOM | 6985 | CA | HIS | E | 45 | 9.260 | 55.586 | 34.525 | 1.00 | 16.91 | C |
| ATOM | 6986 | C | HIS | E | 45 | 9.504 | 55.288 | 33.032 | 1.00 | 16.93 | C |
| ATOM | 6987 | O | HIS | E | 45 | 10.463 | 54.617 | 32.664 | 1.00 | 16.62 | O |
| ATOM | 6988 | CB | HIS | E | 45 | 9.821 | 56.975 | 34.939 | 1.00 | 18.74 | C |
| ATOM | 6989 | CG | HIS | E | 45 | 11.263 | 57.197 | 34.603 | 1.00 | 20.62 | C |
| ATOM | 6990 | ND1 | HIS | E | 45 | 12.294 | 56.806 | 35.431 | 1.00 | 21.97 | N |
| ATOM | 6991 | CD2 | HIS | E | 45 | 11.848 | 57.791 | 33.534 | 1.00 | 22.90 | C |
| ATOM | 6992 | CE1 | HIS | E | 45 | 13.449 | 57.147 | 34.889 | 1.00 | 21.24 | C |
| ATOM | 6993 | NE2 | HIS | E | 45 | 13.205 | 57.748 | 33.738 | 1.00 | 22.91 | N |
| ATOM | 6994 | N | LEU | E | 46 | 8.597 | 55.776 | 32.201 | 1.00 | 17.26 | N |
| ATOM | 6995 | CA | LEU | E | 46 | 8.642 | 55.554 | 30.763 | 1.00 | 16.35 | C |
| ATOM | 6996 | C | LEU | E | 46 | 9.798 | 56.294 | 30.134 | 1.00 | 17.56 | C |
| ATOM | 6997 | O | LEU | E | 46 | 9.961 | 57.481 | 30.351 | 1.00 | 16.02 | O |
| ATOM | 6998 | CB | LEU | E | 46 | 7.329 | 56.032 | 30.118 | 1.00 | 17.27 | C |
| ATOM | 6999 | CG | LEU | E | 46 | 7.204 | 55.800 | 28.613 | 1.00 | 16.11 | C |
| ATOM | 7000 | CD1 | LEU | E | 46 | 7.125 | 54.297 | 28.305 | 1.00 | 16.65 | C |
| ATOM | 7001 | CD2 | LEU | E | 46 | 5.993 | 56.474 | 28.053 | 1.00 | 18.31 | C |
| ATOM | 7002 | N | LEU | E | 47 | 10.587 | 55.582 | 29.333 | 1.00 | 16.07 | N |
| ATOM | 7003 | CA | LEU | E | 47 | 11.649 | 56.165 | 28.546 | 1.00 | 18.86 | C |
| ATOM | 7004 | C | LEU | E | 47 | 11.292 | 56.251 | 27.074 | 1.00 | 18.10 | C |
| ATOM | 7005 | O | LEU | E | 47 | 11.530 | 57.262 | 26.441 | 1.00 | 17.34 | O |
| ATOM | 7006 | CB | LEU | E | 47 | 12.877 | 55.262 | 28.571 | 1.00 | 19.97 | C |
| ATOM | 7007 | CG | LEU | E | 47 | 13.951 | 55.218 | 29.610 | 1.00 | 23.67 | C |
| ATOM | 7008 | CD1 | LEU | E | 47 | 15.053 | 54.443 | 28.934 | 1.00 | 24.33 | C |

| | | | | | | | | | | |
|------|------|-----|-----|---|----|--------|--------|--------|------|-------|---|
| ATOM | 7009 | CD2 | LEU | E | 47 | 14.456 | 56.580 | 30.039 | 1.00 | 24.52 | C |
| ATOM | 7010 | N   | ILE | E | 48 | 10.787 | 55.137 | 26.518 | 1.00 | 18.16 | N |
| ATOM | 7011 | CA  | ILE | E | 48 | 10.572 | 55.009 | 25.089 | 1.00 | 17.96 | C |
| ATOM | 7012 | C   | ILE | E | 48 | 9.254  | 54.310 | 24.867 | 1.00 | 16.71 | C |
| ATOM | 7013 | O   | ILE | E | 48 | 8.974  | 53.334 | 25.535 | 1.00 | 14.01 | O |
| ATOM | 7014 | CB  | ILE | E | 48 | 11.685 | 54.109 | 24.403 | 1.00 | 19.36 | C |
| ATOM | 7015 | CG1 | ILE | E | 48 | 13.104 | 54.669 | 24.572 | 1.00 | 21.83 | C |
| ATOM | 7016 | CG2 | ILE | E | 48 | 11.360 | 53.829 | 22.935 | 1.00 | 18.17 | C |
| ATOM | 7017 | CD1 | ILE | E | 48 | 13.331 | 55.963 | 23.936 | 1.00 | 23.73 | C |
| ATOM | 7018 | N   | TYR | E | 49 | 8.448  | 54.814 | 23.930 | 1.00 | 16.89 | N |
| ATOM | 7019 | CA  | TYR | E | 49 | 7.201  | 54.149 | 23.547 | 1.00 | 17.07 | C |
| ATOM | 7020 | C   | TYR | E | 49 | 7.144  | 53.892 | 22.043 | 1.00 | 14.95 | C |
| ATOM | 7021 | O   | TYR | E | 49 | 7.848  | 54.521 | 21.263 | 1.00 | 14.36 | O |
| ATOM | 7022 | CB  | TYR | E | 49 | 5.977  | 54.934 | 24.011 | 1.00 | 18.38 | C |
| ATOM | 7023 | CG  | TYR | E | 49 | 5.832  | 56.266 | 23.338 | 1.00 | 19.63 | C |
| ATOM | 7024 | CD1 | TYR | E | 49 | 6.523  | 57.388 | 23.805 | 1.00 | 18.09 | C |
| ATOM | 7025 | CD2 | TYR | E | 49 | 5.023  | 56.398 | 22.213 | 1.00 | 19.91 | C |
| ATOM | 7026 | CE1 | TYR | E | 49 | 6.380  | 58.605 | 23.171 | 1.00 | 21.68 | C |
| ATOM | 7027 | CE2 | TYR | E | 49 | 4.892  | 57.590 | 21.580 | 1.00 | 19.88 | C |
| ATOM | 7028 | CZ  | TYR | E | 49 | 5.565  | 58.693 | 22.065 | 1.00 | 20.65 | C |
| ATOM | 7029 | OH  | TYR | E | 49 | 5.414  | 59.882 | 21.399 | 1.00 | 22.86 | O |
| ATOM | 7030 | N   | HIS | E | 50 | 6.329  | 52.923 | 21.645 | 1.00 | 15.59 | N |
| ATOM | 7031 | CA  | HIS | E | 50 | 6.189  | 52.591 | 20.237 | 1.00 | 15.33 | C |
| ATOM | 7032 | C   | HIS | E | 50 | 7.543  | 52.351 | 19.582 | 1.00 | 15.49 | C |
| ATOM | 7033 | O   | HIS | E | 50 | 7.790  | 52.830 | 18.475 | 1.00 | 14.46 | O |
| ATOM | 7034 | CB  | HIS | E | 50 | 5.402  | 53.676 | 19.481 | 1.00 | 14.78 | C |
| ATOM | 7035 | CG  | HIS | E | 50 | 3.961  | 53.794 | 19.883 | 1.00 | 14.89 | C |
| ATOM | 7036 | ND1 | HIS | E | 50 | 3.122  | 54.753 | 19.344 | 1.00 | 16.56 | N |
| ATOM | 7037 | CD2 | HIS | E | 50 | 3.203  | 53.070 | 20.736 | 1.00 | 16.49 | C |
| ATOM | 7038 | CE1 | HIS | E | 50 | 1.908  | 54.605 | 19.858 | 1.00 | 16.81 | C |
| ATOM | 7039 | NE2 | HIS | E | 50 | 1.935  | 53.601 | 20.715 | 1.00 | 17.45 | N |
| ATOM | 7040 | N   | LEU | E | 51 | 8.405  | 51.598 | 20.283 | 1.00 | 16.15 | N |
| ATOM | 7041 | CA  | LEU | E | 51 | 9.747  | 51.173 | 19.839 | 1.00 | 16.00 | C |
| ATOM | 7042 | C   | LEU | E | 51 | 10.849 | 52.227 | 19.859 | 1.00 | 16.09 | C |
| ATOM | 7043 | O   | LEU | E | 51 | 11.952 | 51.932 | 20.270 | 1.00 | 15.37 | O |
| ATOM | 7044 | CB  | LEU | E | 51 | 9.718  | 50.550 | 18.438 | 1.00 | 16.65 | C |
| ATOM | 7045 | CG  | LEU | E | 51 | 11.031 | 49.924 | 17.936 | 1.00 | 15.57 | C |
| ATOM | 7046 | CD1 | LEU | E | 51 | 11.499 | 48.745 | 18.825 | 1.00 | 15.92 | C |
| ATOM | 7047 | CD2 | LEU | E | 51 | 10.860 | 49.465 | 16.500 | 1.00 | 17.49 | C |
| ATOM | 7048 | N   | SER | E | 52 | 10.578 | 53.434 | 19.344 | 1.00 | 16.34 | N |
| ATOM | 7049 | CA  | SER | E | 52 | 11.653 | 54.382 | 19.037 | 1.00 | 16.71 | C |
| ATOM | 7050 | C   | SER | E | 52 | 11.321 | 55.820 | 19.379 | 1.00 | 18.49 | C |
| ATOM | 7051 | O   | SER | E | 52 | 12.110 | 56.702 | 19.104 | 1.00 | 20.39 | O |
| ATOM | 7052 | CB  | SER | E | 52 | 12.010 | 54.318 | 17.547 | 1.00 | 16.44 | C |
| ATOM | 7053 | OG  | SER | E | 52 | 12.421 | 53.016 | 17.156 | 1.00 | 20.47 | O |
| ATOM | 7054 | N   | ASN | E | 53 | 10.167 | 56.059 | 19.974 | 1.00 | 20.18 | N |
| ATOM | 7055 | CA  | ASN | E | 53 | 9.764  | 57.420 | 20.359 | 1.00 | 21.53 | C |
| ATOM | 7056 | C   | ASN | E | 53 | 10.102 | 57.732 | 21.790 | 1.00 | 22.01 | C |
| ATOM | 7057 | O   | ASN | E | 53 | 9.728  | 56.975 | 22.691 | 1.00 | 20.52 | O |
| ATOM | 7058 | CB  | ASN | E | 53 | 8.276  | 57.596 | 20.144 | 1.00 | 22.21 | C |
| ATOM | 7059 | CG  | ASN | E | 53 | 7.900  | 57.538 | 18.695 | 1.00 | 25.63 | C |
| ATOM | 7060 | OD1 | ASN | E | 53 | 8.532  | 58.165 | 17.845 | 1.00 | 30.09 | O |
| ATOM | 7061 | ND2 | ASN | E | 53 | 6.878  | 56.790 | 18.397 | 1.00 | 30.56 | N |
| ATOM | 7062 | N   | LEU | E | 54 | 10.787 | 58.863 | 21.993 | 1.00 | 22.31 | N |
| ATOM | 7063 | CA  | LEU | E | 54 | 11.205 | 59.285 | 23.321 | 1.00 | 23.43 | C |
| ATOM | 7064 | C   | LEU | E | 54 | 10.013 | 59.866 | 24.084 | 1.00 | 23.31 | C |
| ATOM | 7065 | O   | LEU | E | 54 | 9.219  | 60.620 | 23.519 | 1.00 | 22.84 | O |
| ATOM | 7066 | CB  | LEU | E | 54 | 12.356 | 60.297 | 23.237 | 1.00 | 24.05 | C |
| ATOM | 7067 | CG  | LEU | E | 54 | 13.779 | 59.747 | 23.138 | 1.00 | 24.77 | C |
| ATOM | 7068 | CD1 | LEU | E | 54 | 13.891 | 58.787 | 21.953 | 1.00 | 27.46 | C |
| ATOM | 7069 | CD2 | LEU | E | 54 | 14.759 | 60.883 | 22.980 | 1.00 | 26.08 | C |
| ATOM | 7070 | N   | ALA | E | 55 | 9.860  | 59.464 | 25.344 | 1.00 | 23.36 | N |
| ATOM | 7071 | CA  | ALA | E | 55 | 8.816  | 60.021 | 26.212 | 1.00 | 24.42 | C |
| ATOM | 7072 | C   | ALA | E | 55 | 9.172  | 61.462 | 26.541 | 1.00 | 25.98 | C |
| ATOM | 7073 | O   | ALA | E | 55 | 10.338 | 61.872 | 26.433 | 1.00 | 23.93 | O |

| ATOM | 7074 | CB | ALA | E | 55 | 8.684 | 59.236 | 27.477 | 1.00 | 24.42 | C |
|------|------|-----|-----|---|----|-------|--------|--------|------|-------|---|
| ATOM | 7075 | N | SER | E | 56 | 8.168 | 62.217 | 26.972 | 1.00 | 28.39 | N |
| ATOM | 7076 | CA | SER | E | 56 | 8.367 | 63.638 | 27.264 | 1.00 | 29.75 | C |
| ATOM | 7077 | C | SER | E | 56 | 9.514 | 63.796 | 28.245 | 1.00 | 30.10 | C |
| ATOM | 7078 | O | SER | E | 56 | 9.598 | 63.056 | 29.221 | 1.00 | 32.64 | O |
| ATOM | 7079 | CB | SER | E | 56 | 7.090 | 64.252 | 27.850 | 1.00 | 30.35 | C |
| ATOM | 7080 | OG | SER | E | 56 | 7.198 | 65.664 | 27.886 | 1.00 | 32.64 | O |
| ATOM | 7081 | N | GLY | E | 57 | 10.427 | 64.729 | 27.976 | 1.00 | 30.62 | N |
| ATOM | 7082 | CA | GLY | E | 57 | 11.528 | 65.007 | 28.905 | 1.00 | 29.92 | C |
| ATOM | 7083 | C | GLY | E | 57 | 12.760 | 64.117 | 28.839 | 1.00 | 29.89 | C |
| ATOM | 7084 | O | GLY | E | 57 | 13.756 | 64.400 | 29.491 | 1.00 | 29.68 | O |
| ATOM | 7085 | N | VAL | E | 58 | 12.715 | 63.038 | 28.054 | 1.00 | 28.92 | N |
| ATOM | 7086 | CA | VAL | E | 58 | 13.842 | 62.095 | 27.976 | 1.00 | 27.32 | C |
| ATOM | 7087 | C | VAL | E | 58 | 14.918 | 62.669 | 27.055 | 1.00 | 27.56 | C |
| ATOM | 7088 | O | VAL | E | 58 | 14.587 | 63.135 | 25.971 | 1.00 | 27.97 | O |
| ATOM | 7089 | CB | VAL | E | 58 | 13.364 | 60.695 | 27.466 | 1.00 | 25.28 | C |
| ATOM | 7090 | CG1 | VAL | E | 58 | 14.539 | 59.715 | 27.285 | 1.00 | 24.45 | C |
| ATOM | 7091 | CG2 | VAL | E | 58 | 12.361 | 60.128 | 28.406 | 1.00 | 25.55 | C |
| ATOM | 7092 | N | PRO | E | 59 | 16.197 | 62.653 | 27.494 | 1.00 | 28.37 | N |
| ATOM | 7093 | CA | PRO | E | 59 | 17.328 | 63.127 | 26.676 | 1.00 | 28.82 | C |
| ATOM | 7094 | C | PRO | E | 59 | 17.527 | 62.331 | 25.371 | 1.00 | 29.94 | C |
| ATOM | 7095 | O | PRO | E | 59 | 17.286 | 61.128 | 25.338 | 1.00 | 29.00 | O |
| ATOM | 7096 | CB | PRO | E | 59 | 18.550 | 62.943 | 27.584 | 1.00 | 28.11 | C |
| ATOM | 7097 | CG | PRO | E | 59 | 18.102 | 62.362 | 28.818 | 1.00 | 28.76 | C |
| ATOM | 7098 | CD | PRO | E | 59 | 16.624 | 62.213 | 28.832 | 1.00 | 28.62 | C |
| ATOM | 7099 | N | ASP | E | 60 | 17.976 | 63.021 | 24.323 | 1.00 | 30.70 | N |
| ATOM | 7100 | CA | ASP | E | 60 | 18.237 | 62.402 | 23.028 | 1.00 | 31.66 | C |
| ATOM | 7101 | C | ASP | E | 60 | 19.385 | 61.395 | 23.014 | 1.00 | 29.59 | C |
| ATOM | 7102 | O | ASP | E | 60 | 19.621 | 60.765 | 21.993 | 1.00 | 30.13 | O |
| ATOM | 7103 | CB | ASP | E | 60 | 18.488 | 63.470 | 21.961 | 1.00 | 33.49 | C |
| ATOM | 7104 | CG | ASP | E | 60 | 19.644 | 64.398 | 22.304 | 1.00 | 36.55 | C |
| ATOM | 7105 | OD1 | ASP | E | 60 | 20.489 | 64.061 | 23.172 | 1.00 | 39.60 | O |
| ATOM | 7106 | OD2 | ASP | E | 60 | 19.695 | 65.495 | 21.700 | 1.00 | 41.11 | O |
| ATOM | 7107 | N | ARG | E | 61 | 20.097 | 61.262 | 24.131 | 1.00 | 28.37 | N |
| ATOM | 7108 | CA | ARG | E | 61 | 21.132 | 60.237 | 24.302 | 1.00 | 27.93 | C |
| ATOM | 7109 | C | ARG | E | 61 | 20.538 | 58.831 | 24.252 | 1.00 | 24.71 | C |
| ATOM | 7110 | O | ARG | E | 61 | 21.244 | 57.854 | 23.970 | 1.00 | 23.91 | O |
| ATOM | 7111 | CB | ARG | E | 61 | 21.861 | 60.410 | 25.646 | 1.00 | 28.81 | C |
| ATOM | 7112 | CG | ARG | E | 61 | 22.477 | 61.783 | 25.846 | 1.00 | 30.47 | C |
| ATOM | 7113 | CD | ARG | E | 61 | 23.248 | 61.886 | 27.154 | 1.00 | 31.82 | C |
| ATOM | 7114 | NE | ARG | E | 61 | 22.390 | 61.822 | 28.357 | 1.00 | 33.48 | N |
| ATOM | 7115 | CZ | ARG | E | 61 | 22.256 | 60.766 | 29.165 | 1.00 | 32.96 | C |
| ATOM | 7116 | NH1 | ARG | E | 61 | 22.893 | 59.636 | 28.928 | 1.00 | 34.33 | N |
| ATOM | 7117 | NH2 | ARG | E | 61 | 21.470 | 60.849 | 30.226 | 1.00 | 32.25 | N |
| ATOM | 7118 | N | PHE | E | 62 | 19.246 | 58.741 | 24.547 | 1.00 | 22.14 | N |
| ATOM | 7119 | CA | PHE | E | 62 | 18.506 | 57.484 | 24.492 | 1.00 | 20.53 | C |
| ATOM | 7120 | C | PHE | E | 62 | 17.871 | 57.251 | 23.114 | 1.00 | 20.13 | C |
| ATOM | 7121 | O | PHE | E | 62 | 17.254 | 58.144 | 22.547 | 1.00 | 19.22 | O |
| ATOM | 7122 | CB | PHE | E | 62 | 17.435 | 57.466 | 25.585 | 1.00 | 20.21 | C |
| ATOM | 7123 | CG | PHE | E | 62 | 17.999 | 57.392 | 26.978 | 1.00 | 20.70 | C |
| ATOM | 7124 | CD1 | PHE | E | 62 | 18.281 | 58.549 | 27.697 | 1.00 | 20.44 | C |
| ATOM | 7125 | CD2 | PHE | E | 62 | 18.258 | 56.167 | 27.564 | 1.00 | 20.08 | C |
| ATOM | 7126 | CE1 | PHE | E | 62 | 18.794 | 58.461 | 28.972 | 1.00 | 20.46 | C |
| ATOM | 7127 | CE2 | PHE | E | 62 | 18.772 | 56.060 | 28.824 | 1.00 | 19.30 | C |
| ATOM | 7128 | CZ | PHE | E | 62 | 19.051 | 57.201 | 29.540 | 1.00 | 21.45 | C |
| ATOM | 7129 | N | SER | E | 63 | 18.044 | 56.045 | 22.574 | 1.00 | 18.35 | N |
| ATOM | 7130 | CA | SER | E | 63 | 17.454 | 55.658 | 21.291 | 1.00 | 18.14 | C |
| ATOM | 7131 | C | SER | E | 63 | 17.189 | 54.156 | 21.284 | 1.00 | 16.40 | C |
| ATOM | 7132 | O | SER | E | 63 | 17.790 | 53.414 | 22.053 | 1.00 | 17.10 | O |
| ATOM | 7133 | CB | SER | E | 63 | 18.342 | 56.044 | 20.091 | 1.00 | 18.15 | C |
| ATOM | 7134 | OG | SER | E | 63 | 19.583 | 55.353 | 20.124 | 1.00 | 18.65 | O |
| ATOM | 7135 | N | SER | E | 64 | 16.279 | 53.734 | 20.420 | 1.00 | 15.58 | N |
| ATOM | 7136 | CA | SER | E | 64 | 15.837 | 52.358 | 20.391 | 1.00 | 15.07 | C |
| ATOM | 7137 | C | SER | E | 64 | 15.392 | 51.989 | 18.990 | 1.00 | 15.75 | C |
| ATOM | 7138 | O | SER | E | 64 | 14.825 | 52.793 | 18.285 | 1.00 | 14.92 | O |

| ATOM | 7139 | CB | SER | E | 64 | 14.707 | 52.137 | 21.400 | 1.00 | 14.84 | C |
|------|------|----|----|---|----|--------|--------|--------|------|-------|---|
| ATOM | 7140 | OG | SER | E | 64 | 14.105 | 50.860 | 21.313 | 1.00 | 14.29 | O |
| ATOM | 7141 | N | SER | E | 65 | 15.683 | 50.753 | 18.615 | 1.00 | 15.32 | N |
| ATOM | 7142 | CA | SER | E | 65 | 15.315 | 50.200 | 17.317 | 1.00 | 14.07 | C |
| ATOM | 7143 | C | SER | E | 65 | 14.953 | 48.739 | 17.485 | 1.00 | 14.69 | C |
| ATOM | 7144 | O | SER | E | 65 | 15.229 | 48.105 | 18.523 | 1.00 | 13.98 | O |
| ATOM | 7145 | CB | SER | E | 65 | 16.501 | 50.300 | 16.334 | 1.00 | 14.51 | C |
| ATOM | 7146 | OG | SER | E | 65 | 17.643 | 49.594 | 16.810 | 1.00 | 14.16 | O |
| ATOM | 7147 | N | GLY | E | 66 | 14.359 | 48.175 | 16.454 | 1.00 | 13.63 | N |
| ATOM | 7148 | CA | GLY | E | 66 | 14.063 | 46.742 | 16.487 | 1.00 | 11.89 | C |
| ATOM | 7149 | C | GLY | E | 66 | 13.063 | 46.283 | 15.473 | 1.00 | 12.25 | C |
| ATOM | 7150 | O | GLY | E | 66 | 12.465 | 47.072 | 14.750 | 1.00 | 11.40 | O |
| ATOM | 7151 | N | SER | E | 67 | 12.891 | 44.966 | 15.406 | 1.00 | 12.04 | N |
| ATOM | 7152 | CA | SER | E | 67 | 11.907 | 44.388 | 14.530 | 1.00 | 13.55 | C |
| ATOM | 7153 | C | SER | E | 67 | 10.612 | 44.198 | 15.315 | 1.00 | 14.14 | C |
| ATOM | 7154 | O | SER | E | 67 | 10.398 | 44.887 | 16.294 | 1.00 | 15.53 | O |
| ATOM | 7155 | CB | SER | E | 67 | 12.440 | 43.081 | 13.980 | 1.00 | 14.12 | C |
| ATOM | 7156 | OG | SER | E | 67 | 12.625 | 42.161 | 15.019 | 1.00 | 13.22 | O |
| ATOM | 7157 | N | GLY | E | 68 | 9.738 | 43.277 | 14.920 | 1.00 | 13.72 | N |
| ATOM | 7158 | CA | GLY | E | 68 | 8.629 | 42.911 | 15.793 | 1.00 | 12.58 | C |
| ATOM | 7159 | C | GLY | E | 68 | 9.039 | 42.074 | 16.991 | 1.00 | 13.31 | C |
| ATOM | 7160 | O | GLY | E | 68 | 8.300 | 41.986 | 17.941 | 1.00 | 15.47 | O |
| ATOM | 7161 | N | THR | E | 69 | 10.218 | 41.438 | 16.937 | 1.00 | 14.77 | N |
| ATOM | 7162 | CA | THR | E | 69 | 10.626 | 40.471 | 17.946 | 1.00 | 13.84 | C |
| ATOM | 7163 | C | THR | E | 69 | 11.989 | 40.702 | 18.635 | 1.00 | 13.57 | C |
| ATOM | 7164 | O | THR | E | 69 | 12.238 | 40.124 | 19.676 | 1.00 | 13.31 | O |
| ATOM | 7165 | CB | THR | E | 69 | 10.608 | 39.048 | 17.363 | 1.00 | 14.42 | C |
| ATOM | 7166 | OG1 | THR | E | 69 | 11.551 | 38.949 | 16.292 | 1.00 | 15.58 | O |
| ATOM | 7167 | CG2 | THR | E | 69 | 9.221 | 38.678 | 16.894 | 1.00 | 18.24 | C |
| ATOM | 7168 | N | ASP | E | 70 | 12.863 | 41.542 | 18.081 | 1.00 | 13.27 | N |
| ATOM | 7169 | CA | ASP | E | 70 | 14.172 | 41.767 | 18.645 | 1.00 | 14.40 | C |
| ATOM | 7170 | C | ASP | E | 70 | 14.445 | 43.261 | 18.658 | 1.00 | 14.31 | C |
| ATOM | 7171 | O | ASP | E | 70 | 14.283 | 43.929 | 17.622 | 1.00 | 13.47 | O |
| ATOM | 7172 | CB | ASP | E | 70 | 15.260 | 41.101 | 17.806 | 1.00 | 15.60 | C |
| ATOM | 7173 | CG | ASP | E | 70 | 15.117 | 39.592 | 17.743 | 1.00 | 19.67 | C |
| ATOM | 7174 | OD1 | ASP | E | 70 | 15.495 | 38.912 | 18.717 | 1.00 | 20.54 | O |
| ATOM | 7175 | OD2 | ASP | E | 70 | 14.636 | 39.112 | 16.700 | 1.00 | 22.38 | O |
| ATOM | 7176 | N | PHE | E | 71 | 14.897 | 43.772 | 19.806 | 1.00 | 13.63 | N |
| ATOM | 7177 | CA | PHE | E | 71 | 14.999 | 45.224 | 20.014 | 1.00 | 12.89 | C |
| ATOM | 7178 | C | PHE | E | 71 | 16.234 | 45.613 | 20.802 | 1.00 | 12.98 | C |
| ATOM | 7179 | O | PHE | E | 71 | 16.767 | 44.835 | 21.576 | 1.00 | 12.70 | O |
| ATOM | 7180 | CB | PHE | E | 71 | 13.803 | 45.756 | 20.784 | 1.00 | 12.37 | C |
| ATOM | 7181 | CG | PHE | E | 71 | 12.562 | 44.935 | 20.636 | 1.00 | 12.43 | C |
| ATOM | 7182 | CD1 | PHE | E | 71 | 11.755 | 45.065 | 19.515 | 1.00 | 12.35 | C |
| ATOM | 7183 | CD2 | PHE | E | 71 | 12.209 | 44.022 | 21.621 | 1.00 | 13.46 | C |
| ATOM | 7184 | CE1 | PHE | E | 71 | 10.609 | 44.299 | 19.376 | 1.00 | 11.76 | C |
| ATOM | 7185 | CE2 | PHE | E | 71 | 11.088 | 43.302 | 21.512 | 1.00 | 13.14 | C |
| ATOM | 7186 | CZ | PHE | E | 71 | 10.254 | 43.450 | 20.395 | 1.00 | 12.36 | C |
| ATOM | 7187 | N | THR | E | 72 | 16.663 | 46.854 | 20.618 | 1.00 | 14.00 | N |
| ATOM | 7188 | CA | THR | E | 72 | 17.930 | 47.312 | 21.168 | 1.00 | 12.71 | C |
| ATOM | 7189 | C | THR | E | 72 | 17.770 | 48.727 | 21.687 | 1.00 | 13.97 | C |
| ATOM | 7190 | O | THR | E | 72 | 17.482 | 49.636 | 20.920 | 1.00 | 13.37 | O |
| ATOM | 7191 | CB | THR | E | 72 | 19.054 | 47.317 | 20.100 | 1.00 | 13.20 | C |
| ATOM | 7192 | OG1 | THR | E | 72 | 19.194 | 46.014 | 19.519 | 1.00 | 13.83 | O |
| ATOM | 7193 | CG2 | THR | E | 72 | 20.373 | 47.691 | 20.727 | 1.00 | 16.47 | C |
| ATOM | 7194 | N | LEU | E | 73 | 17.940 | 48.897 | 22.993 | 1.00 | 14.31 | N |
| ATOM | 7195 | CA | LEU | E | 73 | 18.088 | 50.232 | 23.575 | 1.00 | 15.38 | C |
| ATOM | 7196 | C | LEU | E | 73 | 19.554 | 50.657 | 23.535 | 1.00 | 15.99 | C |
| ATOM | 7197 | O | LEU | E | 73 | 20.427 | 49.906 | 23.961 | 1.00 | 16.41 | O |
| ATOM | 7198 | CB | LEU | E | 73 | 17.601 | 50.205 | 25.034 | 1.00 | 15.51 | C |
| ATOM | 7199 | CG | LEU | E | 73 | 17.651 | 51.533 | 25.794 | 1.00 | 15.69 | C |
| ATOM | 7200 | CD1 | LEU | E | 73 | 16.770 | 52.542 | 25.188 | 1.00 | 16.26 | C |
| ATOM | 7201 | CD2 | LEU | E | 73 | 17.287 | 51.296 | 27.241 | 1.00 | 16.43 | C |
| ATOM | 7202 | N | ARG | E | 74 | 19.817 | 51.879 | 23.072 | 1.00 | 17.34 | N |
| ATOM | 7203 | CA | ARG | E | 74 | 21.170 | 52.422 | 23.072 | 1.00 | 17.94 | C |

| ATOM | 7204 | C    | ARG  | E | 74 | 21.212 | 53.708 | 23.896 | 1.00 | 19.40 | C |
|------|------|------|------|---|----|--------|--------|--------|------|-------|---|
| ATOM | 7205 | O    | ARG  | E | 74 | 20.347 | 54.575 | 23.753 | 1.00 | 17.66 | O |
| ATOM | 7206 | CB   | AARG | E | 74 | 21.604 | 52.696 | 21.633 | 0.50 | 18.65 | C |
| ATOM | 7207 | CB   | BARG | E | 74 | 21.699 | 52.666 | 21.654 | 0.50 | 18.44 | C |
| ATOM | 7208 | CG   | AARG | E | 74 | 23.064 | 53.029 | 21.479 | 0.50 | 18.34 | C |
| ATOM | 7209 | CG   | BARG | E | 74 | 23.186 | 53.046 | 21.628 | 0.50 | 17.78 | C |
| ATOM | 7210 | CD   | AARG | E | 74 | 23.446 | 53.249 | 20.036 | 0.50 | 19.36 | C |
| ATOM | 7211 | CD   | BARG | E | 74 | 23.915 | 52.549 | 20.393 | 0.50 | 18.86 | C |
| ATOM | 7212 | NE   | AARG | E | 74 | 24.699 | 54.006 | 19.940 | 0.50 | 21.34 | N |
| ATOM | 7213 | NE   | BARG | E | 74 | 25.345 | 52.907 | 20.384 | 0.50 | 18.06 | N |
| ATOM | 7214 | CZ   | AARG | E | 74 | 24.791 | 55.328 | 19.829 | 0.50 | 21.51 | C |
| ATOM | 7215 | CZ   | BARG | E | 74 | 26.370 | 52.060 | 20.442 | 0.50 | 18.64 | C |
| ATOM | 7216 | NH1  | AARG | E | 74 | 23.701 | 56.092 | 19.781 | 0.50 | 22.83 | N |
| ATOM | 7217 | NH1  | BARG | E | 74 | 26.191 | 50.747 | 20.535 | 0.50 | 17.91 | N |
| ATOM | 7218 | NH2  | AARG | E | 74 | 25.987 | 55.897 | 19.756 | 0.50 | 22.02 | N |
| ATOM | 7219 | NH2  | BARG | E | 74 | 27.610 | 52.533 | 20.406 | 0.50 | 19.69 | N |
| ATOM | 7220 | N    | ILE  | E | 75 | 22.221 | 53.825 | 24.747 | 1.00 | 21.78 | N |
| ATOM | 7221 | CA   | ILE  | E | 75 | 22.458 | 55.064 | 25.500 | 1.00 | 24.41 | C |
| ATOM | 7222 | C    | ILE  | E | 75 | 23.805 | 55.552 | 25.020 | 1.00 | 26.14 | C |
| ATOM | 7223 | O    | ILE  | E | 75 | 24.784 | 54.878 | 25.225 | 1.00 | 25.10 | O |
| ATOM | 7224 | CB   | ILE  | E | 75 | 22.507 | 54.813 | 27.021 | 1.00 | 24.96 | C |
| ATOM | 7225 | CG1  | ILE  | E | 75 | 21.298 | 53.979 | 27.458 | 1.00 | 25.86 | C |
| ATOM | 7226 | CG2  | ILE  | E | 75 | 22.615 | 56.154 | 27.768 | 1.00 | 25.54 | C |
| ATOM | 7227 | CD1  | ILE  | E | 75 | 21.297 | 53.614 | 28.966 | 1.00 | 26.17 | C |
| ATOM | 7228 | N    | SER  | E | 76 | 23.863 | 56.697 | 24.351 | 1.00 | 29.27 | N |
| ATOM | 7229 | CA   | SER  | E | 76 | 25.063 | 57.006 | 23.560 | 1.00 | 32.00 | C |
| ATOM | 7230 | C    | SER  | E | 76 | 26.267 | 57.452 | 24.400 | 1.00 | 34.37 | C |
| ATOM | 7231 | O    | SER  | E | 76 | 27.405 | 56.956 | 24.177 | 1.00 | 37.48 | O |
| ATOM | 7232 | CB   | SER  | E | 76 | 24.759 | 58.047 | 22.486 | 1.00 | 31.97 | C |
| ATOM | 7233 | OG   | SER  | E | 76 | 24.458 | 59.283 | 23.079 | 1.00 | 32.06 | O |
| ATOM | 7234 | N    | ARG  | E | 77 | 26.027 | 58.386 | 25.327 | 1.00 | 33.90 | N |
| ATOM | 7235 | CA   | ARG  | E | 77 | 27.073 | 58.956 | 26.184 | 1.00 | 34.37 | C |
| ATOM | 7236 | C    | ARG  | E | 77 | 26.611 | 58.847 | 27.626 | 1.00 | 31.41 | C |
| ATOM | 7237 | O    | ARG  | E | 77 | 26.009 | 59.770 | 28.172 | 1.00 | 30.91 | O |
| ATOM | 7238 | CB   | ARG  | E | 77 | 27.337 | 60.427 | 25.850 | 1.00 | 35.27 | C |
| ATOM | 7239 | CG   | ARG  | E | 77 | 28.247 | 60.648 | 24.641 | 1.00 | 37.81 | C |
| ATOM | 7240 | CD   | ARG  | E | 77 | 27.984 | 62.009 | 24.003 | 1.00 | 39.20 | C |
| ATOM | 7241 | NE   | ARG  | E | 77 | 26.854 | 61.940 | 23.071 | 1.00 | 42.87 | N |
| ATOM | 7242 | CZ   | ARG  | E | 77 | 25.675 | 62.555 | 23.213 | 1.00 | 43.60 | C |
| ATOM | 7243 | NH1  | ARG  | E | 77 | 25.414 | 63.347 | 24.255 | 1.00 | 44.14 | N |
| ATOM | 7244 | NH2  | ARG  | E | 77 | 24.744 | 62.381 | 22.279 | 1.00 | 43.93 | N |
| ATOM | 7245 | N    | VAL  | E | 78 | 26.909 | 57.712 | 28.232 | 1.00 | 29.18 | N |
| ATOM | 7246 | CA   | VAL  | E | 78 | 26.337 | 57.370 | 29.526 | 1.00 | 27.36 | C |
| ATOM | 7247 | C    | VAL  | E | 78 | 26.759 | 58.357 | 30.615 | 1.00 | 27.25 | C |
| ATOM | 7248 | O    | VAL  | E | 78 | 27.909 | 58.795 | 30.653 | 1.00 | 26.68 | O |
| ATOM | 7249 | CB   | VAL  | E | 78 | 26.731 | 55.952 | 29.933 | 1.00 | 27.16 | C |
| ATOM | 7250 | CG1  | VAL  | E | 78 | 26.342 | 55.677 | 31.355 | 1.00 | 28.52 | C |
| ATOM | 7251 | CG2  | VAL  | E | 78 | 26.056 | 54.940 | 29.033 | 1.00 | 26.81 | C |
| ATOM | 7252 | N    | GLU  | E | 79 | 25.798 | 58.688 | 31.476 | 1.00 | 26.45 | N |
| ATOM | 7253 | CA   | GLU  | E | 79 | 25.989 | 59.521 | 32.651 | 1.00 | 26.94 | C |
| ATOM | 7254 | C    | GLU  | E | 79 | 25.752 | 58.680 | 33.916 | 1.00 | 24.54 | C |
| ATOM | 7255 | O    | GLU  | E | 79 | 25.106 | 57.626 | 33.877 | 1.00 | 22.21 | O |
| ATOM | 7256 | CB   | GLU  | E | 79 | 25.019 | 60.713 | 32.621 | 1.00 | 27.44 | C |
| ATOM | 7257 | CG   | GLU  | E | 79 | 25.185 | 61.644 | 31.413 | 1.00 | 29.93 | C |
| ATOM | 7258 | CD   | GLU  | E | 79 | 24.233 | 62.837 | 31.432 | 1.00 | 31.14 | C |
| ATOM | 7259 | OE1  | GLU  | E | 79 | 23.473 | 63.021 | 32.418 | 1.00 | 37.35 | O |
| ATOM | 7260 | OE2  | GLU  | E | 79 | 24.234 | 63.604 | 30.443 | 1.00 | 36.81 | O |
| ATOM | 7261 | N    | ALA  | E | 80 | 26.300 | 59.149 | 35.040 | 1.00 | 23.55 | N |
| ATOM | 7262 | CA   | ALA  | E | 80 | 26.197 | 58.455 | 36.329 | 1.00 | 22.61 | C |
| ATOM | 7263 | C    | ALA  | E | 80 | 24.754 | 58.216 | 36.752 | 1.00 | 23.16 | C |
| ATOM | 7264 | O    | ALA  | E | 80 | 24.430 | 57.160 | 37.304 | 1.00 | 22.19 | O |
| ATOM | 7265 | CB   | ALA  | E | 80 | 26.940 | 59.257 | 37.410 | 1.00 | 22.90 | C |
| ATOM | 7266 | N    | GLU  | E | 81 | 23.906 | 59.197 | 36.460 | 1.00 | 23.67 | N |
| ATOM | 7267 | CA   | GLU  | E | 81 | 22.500 | 59.176 | 36.818 | 1.00 | 25.43 | C |
| ATOM | 7268 | C    | GLU  | E | 81 | 21.686 | 58.160 | 35.986 | 1.00 | 23.65 | C |

| ATOM | 7269 | O   | GLU | E | 81 | 20.530 | 57.885 | 36.312 | 1.00 | 24.44 | O |
|------|------|-----|-----|---|----|--------|--------|--------|------|-------|---|
| ATOM | 7270 | CB  | GLU | E | 81 | 21.906 | 60.598 | 36.686 | 1.00 | 26.88 | C |
| ATOM | 7271 | CG  | GLU | E | 81 | 21.758 | 61.118 | 35.253 | 1.00 | 30.66 | C |
| ATOM | 7272 | CD  | GLU | E | 81 | 21.655 | 62.656 | 35.166 | 1.00 | 32.13 | C |
| ATOM | 7273 | OE1 | GLU | E | 81 | 22.682 | 63.351 | 35.402 | 1.00 | 40.19 | O |
| ATOM | 7274 | OE2 | GLU | E | 81 | 20.558 | 63.177 | 34.849 | 1.00 | 37.72 | O |
| ATOM | 7275 | N   | ASP | E | 82 | 22.296 | 57.577 | 34.949 | 1.00 | 21.94 | N |
| ATOM | 7276 | CA  | ASP | E | 82 | 21.616 | 56.577 | 34.105 | 1.00 | 21.40 | C |
| ATOM | 7277 | C   | ASP | E | 82 | 21.585 | 55.185 | 34.735 | 1.00 | 20.58 | C |
| ATOM | 7278 | O   | ASP | E | 82 | 20.934 | 54.273 | 34.207 | 1.00 | 20.84 | O |
| ATOM | 7279 | CB  | ASP | E | 82 | 22.296 | 56.481 | 32.728 | 1.00 | 21.18 | C |
| ATOM | 7280 | CG  | ASP | E | 82 | 22.210 | 57.780 | 31.927 | 1.00 | 20.86 | C |
| ATOM | 7281 | OD1 | ASP | E | 82 | 21.295 | 58.591 | 32.175 | 1.00 | 20.60 | O |
| ATOM | 7282 | OD2 | ASP | E | 82 | 23.066 | 57.987 | 31.035 | 1.00 | 22.87 | O |
| ATOM | 7283 | N   | VAL | E | 83 | 22.294 | 54.991 | 35.848 | 1.00 | 19.19 | N |
| ATOM | 7284 | CA  | VAL | E | 83 | 22.298 | 53.665 | 36.471 | 1.00 | 19.07 | C |
| ATOM | 7285 | C   | VAL | E | 83 | 20.912 | 53.295 | 36.974 | 1.00 | 17.38 | C |
| ATOM | 7286 | O   | VAL | E | 83 | 20.122 | 54.140 | 37.432 | 1.00 | 17.19 | O |
| ATOM | 7287 | CB  | VAL | E | 83 | 23.365 | 53.486 | 37.606 | 1.00 | 20.37 | C |
| ATOM | 7288 | CG1 | VAL | E | 83 | 24.767 | 53.727 | 37.046 | 1.00 | 22.12 | C |
| ATOM | 7289 | CG2 | VAL | E | 83 | 23.057 | 54.343 | 38.812 | 1.00 | 21.08 | C |
| ATOM | 7290 | N   | GLY | E | 84 | 20.602 | 52.008 | 36.881 | 1.00 | 16.87 | N |
| ATOM | 7291 | CA  | GLY | E | 84 | 19.288 | 51.540 | 37.244 | 1.00 | 17.27 | C |
| ATOM | 7292 | C   | GLY | E | 84 | 18.963 | 50.306 | 36.434 | 1.00 | 16.62 | C |
| ATOM | 7293 | O   | GLY | E | 84 | 19.826 | 49.788 | 35.735 | 1.00 | 16.76 | O |
| ATOM | 7294 | N   | ILE | E | 85 | 17.718 | 49.864 | 36.531 | 1.00 | 15.49 | N |
| ATOM | 7295 | CA  | ILE | E | 85 | 17.227 | 48.689 | 35.802 | 1.00 | 16.29 | C |
| ATOM | 7296 | C   | ILE | E | 85 | 16.292 | 49.121 | 34.681 | 1.00 | 15.43 | C |
| ATOM | 7297 | O   | ILE | E | 85 | 15.311 | 49.841 | 34.918 | 1.00 | 17.09 | O |
| ATOM | 7298 | CB  | ILE | E | 85 | 16.532 | 47.692 | 36.718 | 1.00 | 16.83 | C |
| ATOM | 7299 | CG1 | ILE | E | 85 | 17.539 | 47.184 | 37.784 | 1.00 | 20.97 | C |
| ATOM | 7300 | CG2 | ILE | E | 85 | 15.979 | 46.515 | 35.913 | 1.00 | 17.48 | C |
| ATOM | 7301 | CD1 | ILE | E | 85 | 16.893 | 46.670 | 38.983 | 1.00 | 22.97 | C |
| ATOM | 7302 | N   | TYR | E | 86 | 16.602 | 48.647 | 33.475 | 1.00 | 15.93 | N |
| ATOM | 7303 | CA  | TYR | E | 86 | 15.864 | 48.973 | 32.256 | 1.00 | 14.03 | C |
| ATOM | 7304 | C   | TYR | E | 86 | 14.980 | 47.788 | 31.909 | 1.00 | 14.65 | C |
| ATOM | 7305 | O   | TYR | E | 86 | 15.473 | 46.671 | 31.762 | 1.00 | 14.98 | O |
| ATOM | 7306 | CB  | TYR | E | 86 | 16.846 | 49.285 | 31.102 | 1.00 | 14.79 | C |
| ATOM | 7307 | CG  | TYR | E | 86 | 17.583 | 50.606 | 31.288 | 1.00 | 13.51 | C |
| ATOM | 7308 | CD1 | TYR | E | 86 | 18.718 | 50.678 | 32.092 | 1.00 | 12.84 | C |
| ATOM | 7309 | CD2 | TYR | E | 86 | 17.138 | 51.764 | 30.700 | 1.00 | 15.04 | C |
| ATOM | 7310 | CE1 | TYR | E | 86 | 19.397 | 51.841 | 32.297 | 1.00 | 13.60 | C |
| ATOM | 7311 | CE2 | TYR | E | 86 | 17.809 | 52.972 | 30.923 | 1.00 | 13.02 | C |
| ATOM | 7312 | CZ  | TYR | E | 86 | 18.951 | 52.988 | 31.713 | 1.00 | 15.71 | C |
| ATOM | 7313 | OH  | TYR | E | 86 | 19.640 | 54.161 | 31.932 | 1.00 | 16.70 | O |
| ATOM | 7314 | N   | TYR | E | 87 | 13.676 | 48.033 | 31.788 | 1.00 | 13.55 | N |
| ATOM | 7315 | CA  | TYR | E | 87 | 12.698 | 46.987 | 31.534 | 1.00 | 14.74 | C |
| ATOM | 7316 | C   | TYR | E | 87 | 12.106 | 47.188 | 30.159 | 1.00 | 14.58 | C |
| ATOM | 7317 | O   | TYR | E | 87 | 11.819 | 48.326 | 29.780 | 1.00 | 15.82 | O |
| ATOM | 7318 | CB  | TYR | E | 87 | 11.552 | 47.090 | 32.531 | 1.00 | 14.98 | C |
| ATOM | 7319 | CG  | TYR | E | 87 | 11.920 | 46.764 | 33.954 | 1.00 | 16.46 | C |
| ATOM | 7320 | CD1 | TYR | E | 87 | 11.910 | 45.455 | 34.411 | 1.00 | 14.58 | C |
| ATOM | 7321 | CD2 | TYR | E | 87 | 12.241 | 47.785 | 34.864 | 1.00 | 16.54 | C |
| ATOM | 7322 | CE1 | TYR | E | 87 | 12.227 | 45.151 | 35.724 | 1.00 | 17.24 | C |
| ATOM | 7323 | CE2 | TYR | E | 87 | 12.571 | 47.482 | 36.177 | 1.00 | 17.28 | C |
| ATOM | 7324 | CZ  | TYR | E | 87 | 12.569 | 46.169 | 36.591 | 1.00 | 16.27 | C |
| ATOM | 7325 | OH  | TYR | E | 87 | 12.868 | 45.872 | 37.889 | 1.00 | 18.09 | O |
| ATOM | 7326 | N   | CYS | E | 88 | 11.910 | 46.094 | 29.430 | 1.00 | 14.90 | N |
| ATOM | 7327 | CA  | CYS | E | 88 | 11.110 | 46.139 | 28.198 | 1.00 | 14.88 | C |
| ATOM | 7328 | C   | CYS | E | 88 | 9.705  | 45.614 | 28.502 | 1.00 | 15.00 | C |
| ATOM | 7329 | O   | CYS | E | 88 | 9.496  | 44.882 | 29.474 | 1.00 | 13.10 | O |
| ATOM | 7330 | CB  | CYS | E | 88 | 11.768 | 45.377 | 27.054 | 1.00 | 15.63 | C |
| ATOM | 7331 | SG  | CYS | E | 88 | 12.095 | 43.622 | 27.445 | 1.00 | 18.63 | S |
| ATOM | 7332 | N   | ALA | E | 89 | 8.732  | 46.054 | 27.703 | 1.00 | 13.27 | N |
| ATOM | 7333 | CA  | ALA | E | 89 | 7.349  | 45.651 | 27.869 | 1.00 | 13.06 | C |

```
ATOM   7334  C    ALA E  89     6.615  45.766  26.530  1.00 13.29        C
ATOM   7335  O    ALA E  89     6.984  46.584  25.694  1.00 12.51        O
ATOM   7336  CB   ALA E  89     6.661  46.529  28.908  1.00 15.23        C
ATOM   7337  N    HIS E  90     5.590  44.942  26.342  1.00 13.01        N
ATOM   7338  CA   HIS E  90     4.799  44.988  25.108  1.00 12.93        C
ATOM   7339  C    HIS E  90     3.476  45.666  25.420  1.00 14.93        C
ATOM   7340  O    HIS E  90     3.044  45.733  26.591  1.00 14.37        O
ATOM   7341  CB   HIS E  90     4.510  43.602  24.516  1.00 12.99        C
ATOM   7342  CG   HIS E  90     3.597  42.772  25.358  1.00 12.41        C
ATOM   7343  ND1  HIS E  90     2.224  42.835  25.259  1.00 12.72        N
ATOM   7344  CD2  HIS E  90     3.862  41.907  26.360  1.00 12.42        C
ATOM   7345  CE1  HIS E  90     1.684  42.029  26.154  1.00 12.78        C
ATOM   7346  NE2  HIS E  90     2.659  41.439  26.823  1.00 14.29        N
ATOM   7347  N    ASN E  91     2.824  46.131  24.360  1.00 14.50        N
ATOM   7348  CA   ASN E  91     1.436  46.615  24.458  1.00 15.13        C
ATOM   7349  C    ASN E  91     0.615  46.069  23.283  1.00 15.48        C
ATOM   7350  O    ASN E  91     0.103  46.844  22.474  1.00 15.06        O
ATOM   7351  CB   ASN E  91     1.421  48.134  24.483  1.00 15.56        C
ATOM   7352  CG   ASN E  91     0.026  48.705  24.589  1.00 14.99        C
ATOM   7353  OD1  ASN E  91    -0.912  48.045  25.074  1.00 13.95        O
ATOM   7354  ND2  ASN E  91    -0.125  49.927  24.119  1.00 16.31        N
ATOM   7355  N    VAL E  92     0.499  44.736  23.187  1.00 15.80        N
ATOM   7356  CA   VAL E  92    -0.308  44.119  22.123  1.00 17.37        C
ATOM   7357  C    VAL E  92    -1.478  43.256  22.594  1.00 17.71        C
ATOM   7358  O    VAL E  92    -2.314  42.848  21.779  1.00 17.65        O
ATOM   7359  CB   VAL E  92     0.555  43.308  21.110  1.00 17.84        C
ATOM   7360  CG1  VAL E  92     1.595  44.194  20.496  1.00 18.63        C
ATOM   7361  CG2  VAL E  92     1.223  42.109  21.755  1.00 18.02        C
ATOM   7362  N    GLU E  93    -1.543  42.997  23.897  1.00 17.82        N
ATOM   7363  CA   GLU E  93    -2.489  42.040  24.465  1.00 18.63        C
ATOM   7364  C    GLU E  93    -2.475  42.088  25.983  1.00 18.78        C
ATOM   7365  O    GLU E  93    -1.528  42.632  26.605  1.00 18.91        O
ATOM   7366  CB   GLU E  93    -2.174  40.605  24.000  1.00 19.83        C
ATOM   7367  CG   GLU E  93    -0.951  39.964  24.627  1.00 18.11        C
ATOM   7368  CD   GLU E  93    -0.835  38.460  24.331  1.00 20.81        C
ATOM   7369  OE1  GLU E  93    -1.179  38.072  23.201  1.00 22.41        O
ATOM   7370  OE2  GLU E  93    -0.402  37.683  25.221  1.00 19.14        O
ATOM   7371  N    LEU E  94    -3.536  41.531  26.575  1.00 19.26        N
ATOM   7372  CA   LEU E  94    -3.532  41.142  27.967  1.00 19.77        C
ATOM   7373  C    LEU E  94    -3.204  39.636  28.003  1.00 21.33        C
ATOM   7374  O    LEU E  94    -3.605  38.901  27.096  1.00 21.05        O
ATOM   7375  CB   LEU E  94    -4.873  41.459  28.623  1.00 19.79        C
ATOM   7376  CG   LEU E  94    -5.367  42.904  28.415  1.00 19.01        C
ATOM   7377  CD1  LEU E  94    -6.679  43.156  29.159  1.00 20.68        C
ATOM   7378  CD2  LEU E  94    -4.341  43.930  28.871  1.00 19.70        C
ATOM   7379  N    PRO E  95    -2.400  39.190  28.996  1.00 20.80        N
ATOM   7380  CA   PRO E  95    -1.824  39.987  30.076  1.00 19.49        C
ATOM   7381  C    PRO E  95    -0.640  40.854  29.658  1.00 17.88        C
ATOM   7382  O    PRO E  95     0.109  40.521  28.731  1.00 17.17        O
ATOM   7383  CB   PRO E  95    -1.391  38.938  31.091  1.00 20.66        C
ATOM   7384  CG   PRO E  95    -1.053  37.722  30.260  1.00 20.59        C
ATOM   7385  CD   PRO E  95    -2.013  37.768  29.092  1.00 20.64        C
ATOM   7386  N    ARG E  96    -0.516  41.993  30.326  1.00 17.93        N
ATOM   7387  CA   ARG E  96     0.594  42.902  30.115  1.00 17.12        C
ATOM   7388  C    ARG E  96     1.787  42.242  30.784  1.00 17.19        C
ATOM   7389  O    ARG E  96     1.661  41.792  31.924  1.00 17.61        O
ATOM   7390  CB   ARG E  96     0.318  44.247  30.746  1.00 17.95        C
ATOM   7391  CG   ARG E  96    -0.954  44.860  30.269  1.00 17.27        C
ATOM   7392  CD   ARG E  96    -0.949  46.347  30.523  1.00 16.54        C
ATOM   7393  NE   ARG E  96    -2.144  46.975  29.993  1.00 16.81        N
ATOM   7394  CZ   ARG E  96    -2.335  47.314  28.726  1.00 16.69        C
ATOM   7395  NH1  ARG E  96    -1.417  47.078  27.795  1.00 16.58        N
ATOM   7396  NH2  ARG E  96    -3.472  47.894  28.379  1.00 15.35        N
ATOM   7397  N    THR E  97     2.883  42.089  30.047  1.00 15.74        N
ATOM   7398  CA   THR E  97     4.089  41.469  30.600  1.00 14.95        C
```

372

| ATOM | 7399 | C | THR | E | 97 | 5.324 | 42.335 | 30.398 | 1.00 | 14.23 | C |
|------|------|------|-----|---|-----|--------|--------|--------|------|-------|---|
| ATOM | 7400 | O | THR | E | 97 | 5.380 | 43.201 | 29.487 | 1.00 | 14.87 | O |
| ATOM | 7401 | CB | THR | E | 97 | 4.320 | 40.013 | 30.078 | 1.00 | 15.97 | C |
| ATOM | 7402 | OG1 | THR | E | 97 | 4.480 | 40.010 | 28.664 | 1.00 | 15.97 | O |
| ATOM | 7403 | CG2 | THR | E | 97 | 3.158 | 39.108 | 30.439 | 1.00 | 16.10 | C |
| ATOM | 7404 | N | PHE | E | 98 | 6.307 | 42.103 | 31.276 | 1.00 | 12.96 | N |
| ATOM | 7405 | CA | PHE | E | 98 | 7.544 | 42.834 | 31.341 | 1.00 | 13.26 | C |
| ATOM | 7406 | C | PHE | E | 98 | 8.713 | 41.878 | 31.266 | 1.00 | 13.05 | C |
| ATOM | 7407 | O | PHE | E | 98 | 8.605 | 40.713 | 31.695 | 1.00 | 13.72 | O |
| ATOM | 7408 | CB | PHE | E | 98 | 7.629 | 43.614 | 32.666 | 1.00 | 14.06 | C |
| ATOM | 7409 | CG | PHE | E | 98 | 6.498 | 44.561 | 32.867 | 1.00 | 14.61 | C |
| ATOM | 7410 | CD1 | PHE | E | 98 | 5.297 | 44.118 | 33.406 | 1.00 | 15.26 | C |
| ATOM | 7411 | CD2 | PHE | E | 98 | 6.637 | 45.906 | 32.535 | 1.00 | 16.35 | C |
| ATOM | 7412 | CE1 | PHE | E | 98 | 4.221 | 44.999 | 33.600 | 1.00 | 14.88 | C |
| ATOM | 7413 | CE2 | PHE | E | 98 | 5.577 | 46.785 | 32.698 | 1.00 | 15.47 | C |
| ATOM | 7414 | CZ | PHE | E | 98 | 4.362 | 46.331 | 33.235 | 1.00 | 15.86 | C |
| ATOM | 7415 | N | GLY | E | 99 | 9.830 | 42.380 | 30.746 | 1.00 | 13.67 | N |
| ATOM | 7416 | CA | GLY | E | 99 | 11.104 | 41.666 | 30.844 | 1.00 | 13.50 | C |
| ATOM | 7417 | C | GLY | E | 99 | 11.594 | 41.690 | 32.279 | 1.00 | 14.21 | C |
| ATOM | 7418 | O | GLY | E | 99 | 11.034 | 42.403 | 33.146 | 1.00 | 14.38 | O |
| ATOM | 7419 | N | GLY | E | 100 | 12.605 | 40.878 | 32.558 | 1.00 | 13.21 | N |
| ATOM | 7420 | CA | GLY | E | 100 | 13.173 | 40.790 | 33.891 | 1.00 | 13.71 | C |
| ATOM | 7421 | C | GLY | E | 100 | 13.968 | 41.999 | 34.367 | 1.00 | 13.84 | C |
| ATOM | 7422 | O | GLY | E | 100 | 14.263 | 42.110 | 35.568 | 1.00 | 15.25 | O |
| ATOM | 7423 | N | GLY | E | 101 | 14.335 | 42.889 | 33.449 | 1.00 | 14.40 | N |
| ATOM | 7424 | CA | GLY | E | 101 | 15.154 | 44.043 | 33.776 | 1.00 | 15.59 | C |
| ATOM | 7425 | C | GLY | E | 101 | 16.631 | 43.749 | 33.493 | 1.00 | 17.42 | C |
| ATOM | 7426 | O | GLY | E | 101 | 17.128 | 42.637 | 33.788 | 1.00 | 17.57 | O |
| ATOM | 7427 | N | THR | E | 102 | 17.310 | 44.726 | 32.901 | 1.00 | 16.71 | N |
| ATOM | 7428 | CA | THR | E | 102 | 18.766 | 44.696 | 32.714 | 1.00 | 16.67 | C |
| ATOM | 7429 | C | THR | E | 102 | 19.340 | 45.834 | 33.536 | 1.00 | 17.01 | C |
| ATOM | 7430 | O | THR | E | 102 | 18.957 | 46.986 | 33.353 | 1.00 | 16.23 | O |
| ATOM | 7431 | CB | THR | E | 102 | 19.174 | 44.891 | 31.220 | 1.00 | 16.51 | C |
| ATOM | 7432 | OG1 | THR | E | 102 | 18.562 | 43.878 | 30.387 | 1.00 | 15.36 | O |
| ATOM | 7433 | CG2 | THR | E | 102 | 20.675 | 44.812 | 31.050 | 1.00 | 18.06 | C |
| ATOM | 7434 | N | LYS | E | 103 | 20.298 | 45.530 | 34.412 | 1.00 | 18.38 | N |
| ATOM | 7435 | CA | LYS | E | 103 | 20.911 | 46.556 | 35.238 | 1.00 | 20.81 | C |
| ATOM | 7436 | C | LYS | E | 103 | 22.073 | 47.188 | 34.484 | 1.00 | 21.05 | C |
| ATOM | 7437 | O | LYS | E | 103 | 22.956 | 46.477 | 34.012 | 1.00 | 21.63 | O |
| ATOM | 7438 | CB | LYS | E | 103 | 21.394 | 45.952 | 36.550 | 1.00 | 22.51 | C |
| ATOM | 7439 | CG | LYS | E | 103 | 22.026 | 46.979 | 37.492 | 1.00 | 24.52 | C |
| ATOM | 7440 | CD | LYS | E | 103 | 22.238 | 46.373 | 38.874 | 1.00 | 26.87 | C |
| ATOM | 7441 | CE | LYS | E | 103 | 20.884 | 46.007 | 39.522 | 1.00 | 30.83 | C |
| ATOM | 7442 | NZ | LYS | E | 103 | 20.897 | 46.195 | 41.008 | 1.00 | 33.65 | N |
| ATOM | 7443 | N | LEU | E | 104 | 22.058 | 48.509 | 34.347 | 1.00 | 21.05 | N |
| ATOM | 7444 | CA | LEU | E | 104 | 23.184 | 49.241 | 33.815 | 1.00 | 21.85 | C |
| ATOM | 7445 | C | LEU | E | 104 | 24.181 | 49.546 | 34.943 | 1.00 | 23.64 | C |
| ATOM | 7446 | O | LEU | E | 104 | 23.795 | 50.082 | 35.989 | 1.00 | 22.16 | O |
| ATOM | 7447 | CB | LEU | E | 104 | 22.738 | 50.542 | 33.171 | 1.00 | 20.99 | C |
| ATOM | 7448 | CG | LEU | E | 104 | 23.853 | 51.313 | 32.486 | 1.00 | 21.64 | C |
| ATOM | 7449 | CD1 | LEU | E | 104 | 24.243 | 50.573 | 31.205 | 1.00 | 24.46 | C |
| ATOM | 7450 | CD2 | LEU | E | 104 | 23.465 | 52.733 | 32.166 | 1.00 | 22.77 | C |
| ATOM | 7451 | N | GLU | E | 105 | 25.445 | 49.184 | 34.720 | 1.00 | 26.58 | N |
| ATOM | 7452 | CA | GLU | E | 105 | 26.524 | 49.488 | 35.663 | 1.00 | 29.51 | C |
| ATOM | 7453 | C | GLU | E | 105 | 27.588 | 50.330 | 34.987 | 1.00 | 29.36 | C |
| ATOM | 7454 | O | GLU | E | 105 | 27.923 | 50.102 | 33.827 | 1.00 | 30.01 | O |
| ATOM | 7455 | CB | GLU | E | 105 | 27.165 | 48.220 | 36.244 | 1.00 | 30.11 | C |
| ATOM | 7456 | CG | GLU | E | 105 | 28.023 | 48.553 | 37.484 | 1.00 | 32.36 | C |
| ATOM | 7457 | CD | GLU | E | 105 | 29.150 | 47.565 | 37.823 | 1.00 | 33.08 | C |
| ATOM | 7458 | OE1 | GLU | E | 105 | 28.935 | 46.327 | 37.741 | 1.00 | 35.82 | O |
| ATOM | 7459 | OE2 | GLU | E | 105 | 30.250 | 48.057 | 38.230 | 1.00 | 32.59 | O |
| ATOM | 7460 | N | ILE | E | 106 | 28.100 | 51.300 | 35.728 | 1.00 | 30.21 | N |
| ATOM | 7461 | CA | ILE | E | 106 | 29.165 | 52.168 | 35.275 | 1.00 | 30.94 | C |
| ATOM | 7462 | C | ILE | E | 106 | 30.525 | 51.593 | 35.668 | 1.00 | 30.96 | C |
| ATOM | 7463 | O | ILE | E | 106 | 30.677 | 51.021 | 36.746 | 1.00 | 29.53 | O |

| ATOM | 7464 | CB  | ILE | E | 106 | 29.052 | 53.557 | 35.915 | 1.00 | 31.32 | C |
|------|------|-----|-----|---|-----|--------|--------|--------|------|-------|---|
| ATOM | 7465 | CG1 | ILE | E | 106 | 27.711 | 54.221 | 35.553 | 1.00 | 33.21 | C |
| ATOM | 7466 | CG2 | ILE | E | 106 | 30.256 | 54.424 | 35.560 | 1.00 | 32.49 | C |
| ATOM | 7467 | CD1 | ILE | E | 106 | 27.510 | 54.561 | 34.120 | 1.00 | 33.87 | C |
| ATOM | 7468 | N   | LYS | E | 107 | 31.499 | 51.764 | 34.780 | 1.00 | 31.19 | N |
| ATOM | 7469 | CA  | LYS | E | 107 | 32.897 | 51.476 | 35.066 | 1.00 | 32.17 | C |
| ATOM | 7470 | C   | LYS | E | 107 | 33.579 | 52.797 | 35.352 | 1.00 | 31.44 | C |
| ATOM | 7471 | O   | LYS | E | 107 | 33.516 | 53.725 | 34.552 | 1.00 | 32.72 | O |
| ATOM | 7472 | CB  | LYS | E | 107 | 33.578 | 50.798 | 33.885 | 1.00 | 32.93 | C |
| ATOM | 7473 | CG  | LYS | E | 107 | 34.971 | 50.281 | 34.218 | 1.00 | 34.47 | C |
| ATOM | 7474 | CD  | LYS | E | 107 | 35.801 | 50.008 | 32.976 | 1.00 | 35.22 | C |
| ATOM | 7475 | CE  | LYS | E | 107 | 37.165 | 49.388 | 33.349 | 1.00 | 36.71 | C |
| ATOM | 7476 | NZ  | LYS | E | 107 | 37.900 | 48.792 | 32.166 | 1.00 | 37.84 | N |
| ATOM | 7477 | N   | ARG | E | 108 | 34.221 | 52.894 | 36.503 | 1.00 | 30.05 | N |
| ATOM | 7478 | CA  | ARG | E | 108 | 34.878 | 54.123 | 36.890 | 1.00 | 28.85 | C |
| ATOM | 7479 | C   | ARG | E | 108 | 36.209 | 53.757 | 37.493 | 1.00 | 28.16 | C |
| ATOM | 7480 | O   | ARG | E | 108 | 36.524 | 52.572 | 37.612 | 1.00 | 26.72 | O |
| ATOM | 7481 | CB  | ARG | E | 108 | 34.015 | 54.904 | 37.875 | 1.00 | 28.98 | C |
| ATOM | 7482 | CG  | ARG | E | 108 | 33.635 | 54.159 | 39.152 | 1.00 | 28.11 | C |
| ATOM | 7483 | CD  | ARG | E | 108 | 33.494 | 55.141 | 40.296 | 1.00 | 28.39 | C |
| ATOM | 7484 | NE  | ARG | E | 108 | 34.814 | 55.600 | 40.733 | 1.00 | 29.37 | N |
| ATOM | 7485 | CZ  | ARG | E | 108 | 35.058 | 56.706 | 41.425 | 1.00 | 27.27 | C |
| ATOM | 7486 | NH1 | ARG | E | 108 | 34.089 | 57.526 | 41.788 | 1.00 | 27.20 | N |
| ATOM | 7487 | NH2 | ARG | E | 108 | 36.305 | 56.981 | 41.756 | 1.00 | 27.72 | N |
| ATOM | 7488 | N   | ALA | E | 109 | 36.992 | 54.767 | 37.863 | 1.00 | 27.47 | N |
| ATOM | 7489 | CA  | ALA | E | 109 | 38.294 | 54.524 | 38.477 | 1.00 | 28.64 | C |
| ATOM | 7490 | C   | ALA | E | 109 | 38.134 | 53.779 | 39.798 | 1.00 | 28.15 | C |
| ATOM | 7491 | O   | ALA | E | 109 | 37.176 | 54.003 | 40.523 | 1.00 | 28.70 | O |
| ATOM | 7492 | CB  | ALA | E | 109 | 39.031 | 55.817 | 38.690 | 1.00 | 28.97 | C |
| ATOM | 7493 | N   | ASP | E | 110 | 39.059 | 52.869 | 40.076 | 1.00 | 28.41 | N |
| ATOM | 7494 | CA  | ASP | E | 110 | 39.125 | 52.228 | 41.376 | 1.00 | 27.64 | C |
| ATOM | 7495 | C   | ASP | E | 110 | 39.180 | 53.276 | 42.487 | 1.00 | 26.75 | C |
| ATOM | 7496 | O   | ASP | E | 110 | 39.934 | 54.238 | 42.403 | 1.00 | 25.57 | O |
| ATOM | 7497 | CB  | ASP | E | 110 | 40.363 | 51.351 | 41.472 | 1.00 | 28.67 | C |
| ATOM | 7498 | CG  | ASP | E | 110 | 40.243 | 50.090 | 40.668 | 1.00 | 30.52 | C |
| ATOM | 7499 | OD1 | ASP | E | 110 | 39.194 | 49.854 | 40.040 | 1.00 | 32.66 | O |
| ATOM | 7500 | OD2 | ASP | E | 110 | 41.215 | 49.326 | 40.669 | 1.00 | 35.46 | O |
| ATOM | 7501 | N   | ALA | E | 111 | 38.386 | 53.062 | 43.537 | 1.00 | 26.27 | N |
| ATOM | 7502 | CA  | ALA | E | 111 | 38.435 | 53.880 | 44.738 | 1.00 | 25.02 | C |
| ATOM | 7503 | C   | ALA | E | 111 | 38.491 | 52.978 | 45.981 | 1.00 | 25.45 | C |
| ATOM | 7504 | O   | ALA | E | 111 | 37.802 | 51.956 | 46.057 | 1.00 | 22.25 | O |
| ATOM | 7505 | CB  | ALA | E | 111 | 37.254 | 54.785 | 44.802 | 1.00 | 25.33 | C |
| ATOM | 7506 | N   | ALA | E | 112 | 39.328 | 53.355 | 46.943 | 1.00 | 24.85 | N |
| ATOM | 7507 | CA  | ALA | E | 112 | 39.416 | 52.624 | 48.205 | 1.00 | 24.72 | C |
| ATOM | 7508 | C   | ALA | E | 112 | 38.251 | 52.987 | 49.115 | 1.00 | 24.30 | C |
| ATOM | 7509 | O   | ALA | E | 112 | 37.765 | 54.122 | 49.095 | 1.00 | 24.07 | O |
| ATOM | 7510 | CB  | ALA | E | 112 | 40.755 | 52.939 | 48.917 | 1.00 | 25.02 | C |
| ATOM | 7511 | N   | PRO | E | 113 | 37.816 | 52.026 | 49.953 | 1.00 | 24.10 | N |
| ATOM | 7512 | CA  | PRO | E | 113 | 36.766 | 52.378 | 50.888 | 1.00 | 24.17 | C |
| ATOM | 7513 | C   | PRO | E | 113 | 37.236 | 53.303 | 52.016 | 1.00 | 24.00 | C |
| ATOM | 7514 | O   | PRO | E | 113 | 38.379 | 53.223 | 52.457 | 1.00 | 24.63 | O |
| ATOM | 7515 | CB  | PRO | E | 113 | 36.338 | 51.020 | 51.452 | 1.00 | 24.21 | C |
| ATOM | 7516 | CG  | PRO | E | 113 | 37.523 | 50.172 | 51.372 | 1.00 | 23.93 | C |
| ATOM | 7517 | CD  | PRO | E | 113 | 38.235 | 50.617 | 50.101 | 1.00 | 24.15 | C |
| ATOM | 7518 | N   | THR | E | 114 | 36.337 | 54.166 | 52.469 | 1.00 | 22.91 | N |
| ATOM | 7519 | CA  | THR | E | 114 | 36.508 | 54.897 | 53.707 | 1.00 | 22.97 | C |
| ATOM | 7520 | C   | THR | E | 114 | 35.825 | 54.042 | 54.765 | 1.00 | 22.70 | C |
| ATOM | 7521 | O   | THR | E | 114 | 34.625 | 53.694 | 54.638 | 1.00 | 21.01 | O |
| ATOM | 7522 | CB  | THR | E | 114 | 35.843 | 56.256 | 53.629 | 1.00 | 23.82 | C |
| ATOM | 7523 | OG1 | THR | E | 114 | 36.376 | 56.964 | 52.501 | 1.00 | 26.56 | O |
| ATOM | 7524 | CG2 | THR | E | 114 | 36.076 | 57.074 | 54.902 | 1.00 | 24.99 | C |
| ATOM | 7525 | N   | VAL | E | 115 | 36.597 | 53.655 | 55.771 | 1.00 | 20.78 | N |
| ATOM | 7526 | CA  | VAL | E | 115 | 36.110 | 52.708 | 56.788 | 1.00 | 21.22 | C |
| ATOM | 7527 | C   | VAL | E | 115 | 35.873 | 53.436 | 58.103 | 1.00 | 20.65 | C |
| ATOM | 7528 | O   | VAL | E | 115 | 36.710 | 54.214 | 58.531 | 1.00 | 19.35 | O |

```
ATOM   7529  CB  VAL E 115    37.077  51.542  56.957  1.00 21.18          C
ATOM   7530  CG1 VAL E 115    36.540  50.533  57.973  1.00 21.80          C
ATOM   7531  CG2 VAL E 115    37.323  50.890  55.625  1.00 22.33          C
ATOM   7532  N   SER E 116    34.693  53.213  58.695  1.00 20.99          N
ATOM   7533  CA  SER E 116    34.277  53.820  59.972  1.00 20.82          C
ATOM   7534  C   SER E 116    33.749  52.721  60.884  1.00 19.04          C
ATOM   7535  O   SER E 116    32.920  51.925  60.438  1.00 19.23          O
ATOM   7536  CB  SER E 116    33.106  54.771  59.753  1.00 21.62          C
ATOM   7537  OG  SER E 116    33.513  55.957  59.130  1.00 25.70          O
ATOM   7538  N   ILE E 117    34.148  52.722  62.157  1.00 17.57          N
ATOM   7539  CA  ILE E 117    33.623  51.756  63.136  1.00 17.28          C
ATOM · 7540  C   ILE E 117    32.904  52.524  64.256  1.00 17.41          C
ATOM   7541  O   ILE E 117    33.292  53.657  64.600  1.00 18.26          O
ATOM   7542  CB  ILE E 117    34.732  50.821  63.711  1.00 16.28          C
ATOM   7543  CG1 ILE E 117    34.125  49.617  64.423  1.00 17.86          C
ATOM   7544  CG2 ILE E 117    35.643  51.556  64.658  1.00 16.62          C
ATOM   7545  CD1 ILE E 117    35.138  48.546  64.822  1.00 18.46          C
ATOM   7546  N   PHE E 118    31.832  51.914  64.771  1.00 17.68          N
ATOM   7547  CA  PHE E 118    30.951  52.516  65.747  1.00 17.87          C
ATOM   7548  C   PHE E 118    30.652  51.523  66.867  1.00 18.50          C
ATOM   7549  O   PHE E 118    30.155  50.420  66.603  1.00 16.44          O
ATOM   7550  CB  PHE E 118    29.631  52.939  65.087  1.00 20.44          C
ATOM   7551  CG  PHE E 118    29.810  53.983  64.028  1.00 19.88          C
ATOM   7552  CD1 PHE E 118    29.854  55.322  64.357  1.00 20.68          C
ATOM   7553  CD2 PHE E 118    29.936  53.614  62.702  1.00 23.42          C
ATOM   7554  CE1 PHE E 118    30.041  56.268  63.387  1.00 22.27          C
ATOM   7555  CE2 PHE E 118    30.127  54.559  61.727  1.00 23.12          C
ATOM   7556  CZ  PHE E 118    30.184  55.893  62.074  1.00 22.83          C
ATOM   7557  N   PRO E 119    30.939  51.910  68.117  1.00 18.55          N
ATOM   7558  CA  PRO E 119    30.587  51.057  69.246  1.00 17.71          C
ATOM   7559  C   PRO E 119    29.086  51.093  69.515  1.00 17.42          C
ATOM   7560  O   PRO E 119    28.397  51.964  69.007  1.00 13.65          O
ATOM   7561  CB  PRO E 119    31.341  51.673  70.424  1.00 18.45          C
ATOM   7562  CG  PRO E 119    31.607  53.101  70.023  1.00 20.52          C
ATOM   7563  CD  PRO E 119    31.545  53.198  68.538  1.00 18.85          C
ATOM   7564  N   PRO E 120    28.569  50.135  70.300  1.00 16.42          N
ATOM   7565  CA  PRO E 120    27.165  50.227  70.695  1.00 15.92          C
ATOM   7566  C   PRO E 120    26.777  51.482  71.464  1.00 14.75          C
ATOM   7567  O   PRO E 120    27.586  52.054  72.232  1.00 14.36          O
ATOM   7568  CB  PRO E 120    26.951  48.944  71.533  1.00 17.76          C
ATOM   7569  CG  PRO E 120    28.261  48.484  71.865  1.00 17.87          C
ATOM   7570  CD  PRO E 120    29.191  48.903  70.809  1.00 18.05          C
ATOM   7571  N   SER E 121    25.543  51.910  71.239  1.00 14.90          N
ATOM   7572  CA  SER E 121    24.963  53.051  71.934  1.00 14.22          C
ATOM   7573  C   SER E 121    24.650  52.714  73.385  1.00 15.02          C
ATOM   7574  O   SER E 121    24.397  51.565  73.728  1.00 12.27          O
ATOM   7575  CB  SER E 121    23.683  53.549  71.209  1.00 14.23          C
ATOM   7576  OG  SER E 121    22.644  52.584  71.230  1.00 14.62          O
ATOM   7577  N   SER E 122    24.617  53.737  74.234  1.00 15.77          N
ATOM   7578  CA  SER E 122    24.107  53.550  75.583  1.00 15.53          C
ATOM   7579  C   SER E 122    22.651  53.056  75.588  1.00 15.64          C
ATOM   7580  O   SER E 122    22.286  52.231  76.448  1.00 14.62          O
ATOM   7581  CB  SER E 122    24.255  54.851  76.389  1.00 17.37          C
ATOM   7582  OG  SER E 122    23.590  55.917  75.733  1.00 21.10          O
ATOM   7583  N   GLU E 123    21.822  53.520  74.636  1.00 14.96          N
ATOM   7584  CA  GLU E 123    20.432  53.081  74.555  1.00 15.89          C
ATOM   7585  C   GLU E 123    20.387  51.567  74.359  1.00 14.46          C
ATOM   7586  O   GLU E 123    19.668  50.874  75.040  1.00 15.23          O
ATOM   7587  CB  GLU E 123    19.699  53.713  73.388  1.00 17.53          C
ATOM   7588  CG  GLU E 123    19.383  55.196  73.559  1.00 20.30          C
ATOM   7589  CD  GLU E 123    20.412  56.096  72.932  1.00 21.95          C
ATOM   7590  OE1 GLU E 123    21.573  55.667  72.755  1.00 20.90          O
ATOM   7591  OE2 GLU E 123    20.055  57.262  72.619  1.00 24.62          O
ATOM   7592  N   GLN E 124    21.142  51.082  73.393  1.00 12.68          N
ATOM   7593  CA  GLN E 124    21.160  49.653  73.088  1.00 12.99          C
```

| ATOM | 7594 | C | GLN | E | 124 | 21.689 | 48.843 | 74.269 | 1.00 | 12.32 | C |
|---|---|---|---|---|---|---|---|---|---|---|---|
| ATOM | 7595 | O | GLN | E | 124 | 21.161 | 47.784 | 74.601 | 1.00 | 11.93 | O |
| ATOM | 7596 | CB | GLN | E | 124 | 21.991 | 49.376 | 71.834 | 1.00 | 13.22 | C |
| ATOM | 7597 | CG | GLN | E | 124 | 21.738 | 47.956 | 71.340 | 1.00 | 11.78 | C |
| ATOM | 7598 | CD | GLN | E | 124 | 22.514 | 47.523 | 70.108 | 1.00 | 12.49 | C |
| ATOM | 7599 | OE1 | GLN | E | 124 | 23.506 | 48.127 | 69.711 | 1.00 | 14.73 | O |
| ATOM | 7600 | NE2 | GLN | E | 124 | 22.083 | 46.416 | 69.538 | 1.00 | 11.95 | N |
| ATOM | 7601 | N | LEU | E | 125 | 22.719 | 49.340 | 74.928 | 1.00 | 11.56 | N |
| ATOM | 7602 | CA | LEU | E | 125 | 23.293 | 48.600 | 76.075 | 1.00 | 11.54 | C |
| ATOM | 7603 | C | LEU | E | 125 | 22.308 | 48.552 | 77.260 | 1.00 | 11.92 | C |
| ATOM | 7604 | O | LEU | E | 125 | 22.157 | 47.504 | 77.897 | 1.00 | 10.55 | O |
| ATOM | 7605 | CB | LEU | E | 125 | 24.632 | 49.196 | 76.494 | 1.00 | 11.86 | C |
| ATOM | 7606 | CG | LEU | E | 125 | 25.805 | 48.992 | 75.542 | 1.00 | 13.35 | C |
| ATOM | 7607 | CD1 | LEU | E | 125 | 26.877 | 49.959 | 75.950 | 1.00 | 15.67 | C |
| ATOM | 7608 | CD2 | LEU | E | 125 | 26.295 | 47.555 | 75.601 | 1.00 | 14.56 | C |
| ATOM | 7609 | N | THR | E | 126 | 21.606 | 49.648 | 77.533 | 1.00 | 9.70 | N |
| ATOM | 7610 | CA | THR | E | 126 | 20.534 | 49.620 | 78.537 | 1.00 | 11.29 | C |
| ATOM | 7611 | C | THR | E | 126 | 19.462 | 48.561 | 78.227 | 1.00 | 10.44 | C |
| ATOM | 7612 | O | THR | E | 126 | 18.914 | 47.948 | 79.139 | 1.00 | 11.62 | O |
| ATOM | 7613 | CB | THR | E | 126 | 19.888 | 50.996 | 78.659 | 1.00 | 11.80 | C |
| ATOM | 7614 | OG1 | THR | E | 126 | 20.861 | 51.937 | 79.158 | 1.00 | 13.18 | O |
| ATOM | 7615 | CG2 | THR | E | 126 | 18.659 | 50.964 | 79.530 | 1.00 | 14.07 | C |
| ATOM | 7616 | N | SER | E | 127 | 19.174 | 48.359 | 76.947 | 1.00 | 11.83 | N |
| ATOM | 7617 | CA | SER | E | 127 | 18.199 | 47.357 | 76.522 | 1.00 | 11.02 | C |
| ATOM | 7618 | C | SER | E | 127 | 18.746 | 45.943 | 76.580 | 1.00 | 11.51 | C |
| ATOM | 7619 | O | SER | E | 127 | 17.996 | 45.010 | 76.316 | 1.00 | 11.18 | O |
| ATOM | 7620 | CB | SER | E | 127 | 17.696 | 47.643 | 75.116 | 1.00 | 11.08 | C |
| ATOM | 7621 | OG | SER | E | 127 | 18.577 | 47.211 | 74.088 | 1.00 | 12.20 | O |
| ATOM | 7622 | N | GLY | E | 128 | 20.036 | 45.779 | 76.848 | 1.00 | 10.30 | N |
| ATOM | 7623 | CA | GLY | E | 128 | 20.649 | 44.412 | 76.867 | 1.00 | 11.55 | C |
| ATOM | 7624 | C | GLY | E | 128 | 21.280 | 43.897 | 75.589 | 1.00 | 12.23 | C |
| ATOM | 7625 | O | GLY | E | 128 | 21.599 | 42.677 | 75.474 | 1.00 | 11.97 | O |
| ATOM | 7626 | N | GLY | E | 129 | 21.475 | 44.790 | 74.623 | 1.00 | 11.55 | N |
| ATOM | 7627 | CA | GLY | E | 129 | 22.049 | 44.399 | 73.333 | 1.00 | 13.04 | C |
| ATOM | 7628 | C | GLY | E | 129 | 23.341 | 45.122 | 73.059 | 1.00 | 12.68 | C |
| ATOM | 7629 | O | GLY | E | 129 | 23.640 | 46.130 | 73.709 | 1.00 | 12.73 | O |
| ATOM | 7630 | N | ALA | E | 130 | 24.097 | 44.645 | 72.068 | 1.00 | 13.21 | N |
| ATOM | 7631 | CA | ALA | E | 130 | 25.337 | 45.306 | 71.687 | 1.00 | 15.30 | C |
| ATOM | 7632 | C | ALA | E | 130 | 25.698 | 44.986 | 70.229 | 1.00 | 15.67 | C |
| ATOM | 7633 | O | ALA | E | 130 | 26.231 | 43.920 | 69.908 | 1.00 | 16.47 | O |
| ATOM | 7634 | CB | ALA | E | 130 | 26.493 | 44.934 | 72.637 | 1.00 | 15.62 | C |
| ATOM | 7635 | N | SER | E | 131 | 25.423 | 45.958 | 69.371 | 1.00 | 16.72 | N |
| ATOM | 7636 | CA | SER | E | 131 | 25.818 | 45.920 | 67.958 | 1.00 | 17.09 | C |
| ATOM | 7637 | C | SER | E | 131 | 26.999 | 46.862 | 67.709 | 1.00 | 17.55 | C |
| ATOM | 7638 | O | SER | E | 131 | 27.007 | 48.007 | 68.158 | 1.00 | 15.29 | O |
| ATOM | 7639 | CB | SER | E | 131 | 24.659 | 46.349 | 67.081 | 1.00 | 17.49 | C |
| ATOM | 7640 | OG | SER | E | 131 | 23.555 | 45.492 | 67.237 | 1.00 | 17.24 | O |
| ATOM | 7641 | N | VAL | E | 132 | 27.984 | 46.364 | 66.974 | 1.00 | 17.57 | N |
| ATOM | 7642 | CA | VAL | E | 132 | 29.115 | 47.141 | 66.528 | 1.00 | 18.38 | C |
| ATOM | 7643 | C | VAL | E | 132 | 28.967 | 47.236 | 65.014 | 1.00 | 18.68 | C |
| ATOM | 7644 | O | VAL | E | 132 | 28.762 | 46.226 | 64.353 | 1.00 | 17.21 | O |
| ATOM | 7645 | CB | VAL | E | 132 | 30.447 | 46.436 | 66.864 | 1.00 | 19.65 | C |
| ATOM | 7646 | CG1 | VAL | E | 132 | 31.585 | 47.311 | 66.458 | 1.00 | 20.23 | C |
| ATOM | 7647 | CG2 | VAL | E | 132 | 30.510 | 46.112 | 68.345 | 1.00 | 20.41 | C |
| ATOM | 7648 | N | VAL | E | 133 | 29.035 | 48.450 | 64.490 | 1.00 | 18.44 | N |
| ATOM | 7649 | CA | VAL | E | 133 | 28.724 | 48.677 | 63.086 | 1.00 | 18.18 | C |
| ATOM | 7650 | C | VAL | E | 133 | 29.955 | 49.209 | 62.389 | 1.00 | 18.63 | C |
| ATOM | 7651 | O | VAL | E | 133 | 30.600 | 50.139 | 62.882 | 1.00 | 16.97 | O |
| ATOM | 7652 | CB | VAL | E | 133 | 27.556 | 49.674 | 62.910 | 1.00 | 18.77 | C |
| ATOM | 7653 | CG1 | VAL | E | 133 | 27.319 | 49.976 | 61.406 | 1.00 | 20.16 | C |
| ATOM | 7654 | CG2 | VAL | E | 133 | 26.297 | 49.136 | 63.552 | 1.00 | 19.48 | C |
| ATOM | 7655 | N | CYS | E | 134 | 30.238 | 48.631 | 61.224 | 1.00 | 18.95 | N |
| ATOM | 7656 | CA | CYS | E | 134 | 31.325 | 49.060 | 60.369 | 1.00 | 19.38 | C |
| ATOM | 7657 | C | CYS | E | 134 | 30.737 | 49.439 | 59.013 | 1.00 | 19.44 | C |
| ATOM | 7658 | O | CYS | E | 134 | 30.004 | 48.653 | 58.397 | 1.00 | 18.46 | O |

```
ATOM   7659  CB  CYS E 134      32.311  47.914  60.221  1.00 21.84           C
ATOM   7660  SG  CYS E 134      33.830  48.307  59.368  1.00 23.93           S
ATOM   7661  N   PHE E 135      31.017  50.660  58.573  1.00 18.59           N
ATOM   7662  CA  PHE E 135      30.632  51.099  57.247  1.00 18.50           C
ATOM   7663  C   PHE E 135      31.887  51.127  56.394  1.00 19.37           C
ATOM   7664  O   PHE E 135      32.887  51.707  56.811  1.00 19.21           O
ATOM   7665  CB  PHE E 135      30.115  52.526  57.261  1.00 18.71           C
ATOM   7666  CG  PHE E 135      28.782  52.719  57.924  1.00 19.86           C
ATOM   7667  CD1 PHE E 135      27.759  51.792  57.798  1.00 20.06           C
ATOM   7668  CD2 PHE E 135      28.530  53.886  58.630  1.00 21.06           C
ATOM   7669  CE1 PHE E 135      26.546  52.007  58.398  1.00 20.06           C
ATOM   7670  CE2 PHE E 135      27.281  54.090  59.245  1.00 22.97           C
ATOM   7671  CZ  PHE E 135      26.309  53.151  59.108  1.00 17.81           C
ATOM   7672  N   LEU E 136      31.798  50.544  55.209  1.00 19.67           N
ATOM   7673  CA  LEU E 136      32.825  50.634  54.162  1.00 21.24           C
ATOM   7674  C   LEU E 136      32.210  51.442  53.014  1.00 21.71           C
ATOM   7675  O   LEU E 136      31.423  50.914  52.230  1.00 21.88           O
ATOM   7676  CB  LEU E 136      33.210  49.229  53.710  1.00 21.09           C
ATOM   7677  CG  LEU E 136      33.795  48.273  54.762  1.00 23.98           C
ATOM   7678  CD1 LEU E 136      32.769  47.898  55.838  1.00 25.38           C
ATOM   7679  CD2 LEU E 136      34.291  47.001  54.099  1.00 23.33           C
ATOM   7680  N   ASN E 137      32.518  52.733  52.937  1.00 20.69           N
ATOM   7681  CA  ASN E 137      31.816  53.638  52.029  1.00 21.99           C
ATOM   7682  C   ASN E 137      32.606  54.072  50.787  1.00 23.03           C
ATOM   7683  O   ASN E 137      33.784  54.433  50.881  1.00 20.94           O
ATOM   7684  CB  ASN E 137      31.391  54.895  52.774  1.00 21.89           C
ATOM   7685  CG  ASN E 137      30.249  54.630  53.757  1.00 23.84           C
ATOM   7686  OD1 ASN E 137      29.613  53.580  53.709  1.00 24.97           O
ATOM   7687  ND2 ASN E 137      29.978  55.590  54.619  1.00 24.41           N
ATOM   7688  N   ASN E 138      31.926  54.033  49.640  1.00 23.15           N
ATOM   7689  CA  ASN E 138      32.356  54.724  48.411  1.00 23.61           C
ATOM   7690  C   ASN E 138      33.618  54.141  47.806  1.00 23.38           C
ATOM   7691  O   ASN E 138      34.625  54.834  47.586  1.00 24.50           O
ATOM   7692  CB  ASN E 138      32.473  56.231  48.649  1.00 24.82           C
ATOM   7693  CG  ASN E 138      31.186  56.837  49.149  1.00 27.04           C
ATOM   7694  OD1 ASN E 138      30.974  56.961  50.353  1.00 30.67           O
ATOM   7695  ND2 ASN E 138      30.300  57.203  48.234  1.00 30.06           N
ATOM   7696  N   PHE E 139      33.540  52.853  47.523  1.00 21.98           N
ATOM   7697  CA  PHE E 139      34.629  52.122  46.907  1.00 22.35           C
ATOM   7698  C   PHE E 139      34.233  51.674  45.495  1.00 22.14           C
ATOM   7699  O   PHE E 139      33.059  51.672  45.132  1.00 20.27           O
ATOM   7700  CB  PHE E 139      35.068  50.929  47.774  1.00 22.95           C
ATOM   7701  CG  PHE E 139      33.962  49.961  48.115  1.00 22.51           C
ATOM   7702  CD1 PHE E 139      33.676  48.890  47.280  1.00 23.78           C
ATOM   7703  CD2 PHE E 139      33.217  50.114  49.287  1.00 23.61           C
ATOM   7704  CE1 PHE E 139      32.648  47.975  47.594  1.00 25.26           C
ATOM   7705  CE2 PHE E 139      32.181  49.219  49.602  1.00 23.23           C
ATOM   7706  CZ  PHE E 139      31.904  48.142  48.763  1.00 23.73           C
ATOM   7707  N   TYR E 140      35.234  51.349  44.693  1.00 23.06           N
ATOM   7708  CA  TYR E 140      35.018  50.765  43.374  1.00 24.58           C
ATOM   7709  C   TYR E 140      36.215  49.890  43.058  1.00 24.40           C
ATOM   7710  O   TYR E 140      37.331  50.298  43.307  1.00 24.84           O
ATOM   7711  CB  TYR E 140      34.854  51.831  42.281  1.00 25.99           C
ATOM   7712  CG  TYR E 140      34.441  51.170  40.991  1.00 26.43           C
ATOM   7713  CD1 TYR E 140      35.393  50.637  40.125  1.00 28.62           C
ATOM   7714  CD2 TYR E 140      33.089  51.002  40.673  1.00 27.87           C
ATOM   7715  CE1 TYR E 140      35.015  49.964  38.959  1.00 27.30           C
ATOM   7716  CE2 TYR E 140      32.707  50.341  39.507  1.00 28.24           C
ATOM   7717  CZ  TYR E 140      33.680  49.833  38.660  1.00 27.59           C
ATOM   7718  OH  TYR E 140      33.322  49.168  37.510  1.00 28.27           O
ATOM   7719  N   PRO E 141      36.003  48.683  42.505  1.00 25.67           N
ATOM   7720  CA  PRO E 141      34.755  48.019  42.130  1.00 25.83           C
ATOM   7721  C   PRO E 141      34.001  47.431  43.328  1.00 26.73           C
ATOM   7722  O   PRO E 141      34.496  47.448  44.471  1.00 23.30           O
ATOM   7723  CB  PRO E 141      35.228  46.880  41.224  1.00 25.44           C
```

```
ATOM   7724  CG   PRO E 141    36.555  46.524  41.762  1.00 26.28        C
ATOM   7725  CD   PRO E 141    37.171  47.833  42.218  1.00 25.16        C
ATOM   7726  N    LYS E 142    32.824  46.899  43.027  1.00 28.26        N
ATOM   7727  CA   LYS E 142    31.906  46.300  44.002  1.00 29.66        C
ATOM   7728  C    LYS E 142    32.454  45.219  44.949  1.00 29.12        C
ATOM   7729  O    LYS E 142    31.969  45.106  46.066  1.00 27.41        O
ATOM   7730  CB   LYS E 142    30.718  45.707  43.241  1.00 30.66        C
ATOM   7731  CG   LYS E 142    31.105  44.504  42.333  1.00 32.72        C
ATOM   7732  CD   LYS E 142    29.931  43.936  41.525  1.00 33.13        C
ATOM   7733  CE   LYS E 142    29.186  45.006  40.726  1.00 35.29        C
ATOM   7734  NZ   LYS E 142    28.463  44.405  39.552  1.00 35.91        N
ATOM   7735  N    ASP E 143    33.404  44.394  44.502  1.00 29.71        N
ATOM   7736  CA   ASP E 143    33.863  43.242  45.305  1.00 30.39        C
ATOM   7737  C    ASP E 143    34.691  43.717  46.505  1.00 29.03        C
ATOM   7738  O    ASP E 143    35.644  44.491  46.362  1.00 27.63        O
ATOM   7739  CB   ASP E 143    34.713  42.250  44.488  1.00 32.44        C
ATOM   7740  CG   ASP E 143    33.893  41.389  43.515  1.00 35.99        C
ATOM   7741  OD1  ASP E 143    34.533  40.617  42.748  1.00 38.74        O
ATOM   7742  OD2  ASP E 143    32.638  41.470  43.489  1.00 39.93        O
ATOM   7743  N    ILE E 144    34.320  43.257  47.690  1.00 27.74        N
ATOM   7744  CA   ILE E 144    35.017  43.647  48.904  1.00 27.65        C
ATOM   7745  C    ILE E 144    34.770  42.584  49.959  1.00 27.95        C
ATOM   7746  O    ILE E 144    33.808  41.815  49.868  1.00 26.70        O
ATOM   7747  CB   ILE E 144    34.578  45.049  49.407  1.00 28.42        C
ATOM   7748  CG1  ILE E 144    35.502  45.551  50.522  1.00 27.15        C
ATOM   7749  CG2  ILE E 144    33.119  45.056  49.855  1.00 29.23        C
ATOM   7750  CD1  ILE E 144    35.500  47.061  50.682  1.00 28.00        C
ATOM   7751  N    ASN E 145    35.658  42.521  50.933  1.00 27.19        N
ATOM   7752  CA   ASN E 145    35.473  41.597  52.022  1.00 28.10        C
ATOM   7753  C    ASN E 145    35.748  42.287  53.339  1.00 26.54        C
ATOM   7754  O    ASN E 145    36.601  43.156  53.433  1.00 26.69        O
ATOM   7755  CB  AASN E 145    36.326  40.340  51.817  0.50 27.94        C
ATOM   7756  CB  BASN E 145    36.395  40.387  51.855  0.50 28.25        C
ATOM   7757  CG  AASN E 145    35.690  39.353  50.816  0.50 28.47        C
ATOM   7758  CG  BASN E 145    35.927  39.163  52.644  0.50 29.40        C
ATOM   7759  OD1 AASN E 145    34.470  39.295  50.669  0.50 28.30        O
ATOM   7760  OD1 BASN E 145    36.745  38.441  53.202  0.50 30.80        O
ATOM   7761  ND2 AASN E 145    36.522  38.578  50.135  0.50 29.04        N
ATOM   7762  ND2 BASN E 145    34.618  38.919  52.675  0.50 29.95        N
ATOM   7763  N    VAL E 146    34.969  41.914  54.344  1.00 26.31        N
ATOM   7764  CA   VAL E 146    35.121  42.441  55.691  1.00 25.81        C
ATOM   7765  C    VAL E 146    35.384  41.256  56.619  1.00 24.95        C
ATOM   7766  O    VAL E 146    34.767  40.199  56.486  1.00 24.82        O
ATOM   7767  CB   VAL E 146    33.865  43.279  56.121  1.00 26.76        C
ATOM   7768  CG1  VAL E 146    32.630  42.397  56.247  1.00 28.17        C
ATOM   7769  CG2  VAL E 146    34.111  44.051  57.398  1.00 27.49        C
ATOM   7770  N    LYS E 147    36.333  41.420  57.526  1.00 23.28        N
ATOM   7771  CA   LYS E 147    36.571  40.423  58.554  1.00 24.14        C
ATOM   7772  C    LYS E 147    36.474  41.097  59.914  1.00 22.24        C
ATOM   7773  O    LYS E 147    36.997  42.198  60.116  1.00 20.01        O
ATOM   7774  CB   LYS E 147    37.932  39.765  58.381  1.00 24.68        C
ATOM   7775  CG   LYS E 147    38.135  38.560  59.294  1.00 28.48        C
ATOM   7776  CD   LYS E 147    39.589  38.448  59.810  1.00 31.36        C
ATOM   7777  CE   LYS E 147    40.537  37.968  58.734  1.00 33.03        C
ATOM   7778  NZ   LYS E 147    41.744  37.312  59.354  1.00 36.32        N
ATOM   7779  N    TRP E 148    35.762  40.450  60.837  1.00 20.67        N
ATOM   7780  CA   TRP E 148    35.649  40.956  62.210  1.00 19.98        C
ATOM   7781  C    TRP E 148    36.594  40.189  63.105  1.00 20.23        C
ATOM   7782  O    TRP E 148    36.809  39.000  62.900  1.00 20.15        O
ATOM   7783  CB   TRP E 148    34.232  40.794  62.751  1.00 19.56        C
ATOM   7784  CG   TRP E 148    33.297  41.792  62.254  1.00 18.44        C
ATOM   7785  CD1  TRP E 148    32.487  41.673  61.172  1.00 19.47        C
ATOM   7786  CD2  TRP E 148    33.051  43.078  62.803  1.00 18.43        C
ATOM   7787  NE1  TRP E 148    31.759  42.820  61.002  1.00 19.40        N
ATOM   7788  CE2  TRP E 148    32.082  43.702  61.988  1.00 18.45        C
```

378

| ATOM | 7789 | CE3 | TRP | E | 148 | 33.578 | 43.783 | 63.888 | 1.00 | 17.78 | C |
|------|------|-----|-----|---|-----|--------|--------|--------|------|-------|---|
| ATOM | 7790 | CZ2 | TRP | E | 148 | 31.605 | 44.983 | 62.234 | 1.00 | 18.72 | C |
| ATOM | 7791 | CZ3 | TRP | E | 148 | 33.107 | 45.067 | 64.137 | 1.00 | 18.80 | C |
| ATOM | 7792 | CH2 | TRP | E | 148 | 32.128 | 45.658 | 63.310 | 1.00 | 18.85 | C |
| ATOM | 7793 | N   | LYS | E | 149 | 37.168 | 40.878 | 64.081 | 1.00 | 20.22 | N |
| ATOM | 7794 | CA  | LYS | E | 149 | 37.917 | 40.213 | 65.142 | 1.00 | 20.33 | C |
| ATOM | 7795 | C   | LYS | E | 149 | 37.430 | 40.677 | 66.490 | 1.00 | 19.68 | C |
| ATOM | 7796 | O   | LYS | E | 149 | 37.172 | 41.873 | 66.677 | 1.00 | 19.46 | O |
| ATOM | 7797 | CB  | LYS | E | 149 | 39.407 | 40.475 | 65.027 | 1.00 | 21.31 | C |
| ATOM | 7798 | CG  | LYS | E | 149 | 39.991 | 39.923 | 63.769 | 1.00 | 24.61 | C |
| ATOM | 7799 | CD  | LYS | E | 149 | 41.467 | 40.163 | 63.719 | 1.00 | 26.54 | C |
| ATOM | 7800 | CE  | LYS | E | 149 | 42.062 | 39.453 | 62.551 | 1.00 | 28.73 | C |
| ATOM | 7801 | NZ  | LYS | E | 149 | 43.541 | 39.599 | 62.570 | 1.00 | 30.25 | N |
| ATOM | 7802 | N   | ILE | E | 150 | 37.301 | 39.732 | 67.421 | 1.00 | 19.59 | N |
| ATOM | 7803 | CA  | ILE | E | 150 | 37.058 | 40.066 | 68.822 | 1.00 | 20.15 | C |
| ATOM | 7804 | C   | ILE | E | 150 | 38.245 | 39.596 | 69.636 | 1.00 | 19.66 | C |
| ATOM | 7805 | O   | ILE | E | 150 | 38.590 | 38.442 | 69.591 | 1.00 | 18.71 | O |
| ATOM | 7806 | CB  | ILE | E | 150 | 35.768 | 39.423 | 69.360 | 1.00 | 19.48 | C |
| ATOM | 7807 | CG1 | ILE | E | 150 | 34.565 | 39.944 | 68.581 | 1.00 | 20.27 | C |
| ATOM | 7808 | CG2 | ILE | E | 150 | 35.634 | 39.671 | 70.882 | 1.00 | 19.89 | C |
| ATOM | 7809 | CD1 | ILE | E | 150 | 33.264 | 39.426 | 69.045 | 1.00 | 21.33 | C |
| ATOM | 7810 | N   | ASP | E | 151 | 38.885 | 40.509 | 70.345 | 1.00 | 19.27 | N |
| ATOM | 7811 | CA  | ASP | E | 151 | 40.118 | 40.206 | 71.074 | 1.00 | 20.98 | C |
| ATOM | 7812 | C   | ASP | E | 151 | 41.145 | 39.495 | 70.175 | 1.00 | 21.40 | C |
| ATOM | 7813 | O   | ASP | E | 151 | 41.808 | 38.535 | 70.592 | 1.00 | 21.02 | O |
| ATOM | 7814 | CB  | ASP | E | 151 | 39.806 | 39.403 | 72.342 | 1.00 | 22.99 | C |
| ATOM | 7815 | CG  | ASP | E | 151 | 39.078 | 40.225 | 73.381 | 1.00 | 24.05 | C |
| ATOM | 7816 | OD1 | ASP | E | 151 | 39.061 | 41.455 | 73.245 | 1.00 | 25.71 | O |
| ATOM | 7817 | OD2 | ASP | E | 151 | 38.522 | 39.649 | 74.343 | 1.00 | 26.70 | O |
| ATOM | 7818 | N   | GLY | E | 152 | 41.238 | 39.973 | 68.932 | 1.00 | 21.08 | N |
| ATOM | 7819 | CA  | GLY | E | 152 | 42.182 | 39.454 | 67.966 | 1.00 | 21.24 | C |
| ATOM | 7820 | C   | GLY | E | 152 | 41.777 | 38.169 | 67.273 | 1.00 | 21.49 | C |
| ATOM | 7821 | O   | GLY | E | 152 | 42.452 | 37.743 | 66.330 | 1.00 | 20.44 | O |
| ATOM | 7822 | N   | SER | E | 153 | 40.700 | 37.528 | 67.728 | 1.00 | 22.01 | N |
| ATOM | 7823 | CA  | SER | E | 153 | 40.254 | 36.271 | 67.130 | 1.00 | 23.44 | C |
| ATOM | 7824 | C   | SER | E | 153 | 39.130 | 36.543 | 66.152 | 1.00 | 24.02 | C |
| ATOM | 7825 | O   | SER | E | 153 | 38.184 | 37.251 | 66.485 | 1.00 | 23.61 | O |
| ATOM | 7826 | CB  | SER | E | 153 | 39.738 | 35.300 | 68.189 | 1.00 | 24.62 | C |
| ATOM | 7827 | OG  | SER | E | 153 | 40.740 | 34.922 | 69.121 | 1.00 | 26.40 | O |
| ATOM | 7828 | N   | GLU | E | 154 | 39.219 | 35.921 | 64.978 | 1.00 | 24.81 | N |
| ATOM | 7829 | CA  | GLU | E | 154 | 38.261 | 36.116 | 63.897 | 1.00 | 26.77 | C |
| ATOM | 7830 | C   | GLU | E | 154 | 36.882 | 35.666 | 64.339 | 1.00 | 26.22 | C |
| ATOM | 7831 | O   | GLU | E | 154 | 36.718 | 34.563 | 64.885 | 1.00 | 24.61 | O |
| ATOM | 7832 | CB  | GLU | E | 154 | 38.662 | 35.323 | 62.647 | 1.00 | 27.70 | C |
| ATOM | 7833 | CG  | GLU | E | 154 | 37.732 | 35.579 | 61.458 | 1.00 | 30.57 | C |
| ATOM | 7834 | CD  | GLU | E | 154 | 38.260 | 35.022 | 60.148 | 1.00 | 31.82 | C |
| ATOM | 7835 | OE1 | GLU | E | 154 | 39.483 | 35.127 | 59.896 | 1.00 | 37.43 | O |
| ATOM | 7836 | OE2 | GLU | E | 154 | 37.444 | 34.481 | 59.370 | 1.00 | 37.94 | O |
| ATOM | 7837 | N   | ARG | E | 155 | 35.907 | 36.525 | 64.072 | 1.00 | 25.62 | N |
| ATOM | 7838 | CA  | ARG | E | 155 | 34.541 | 36.319 | 64.469 | 1.00 | 26.54 | C |
| ATOM | 7839 | C   | ARG | E | 155 | 33.630 | 36.291 | 63.233 | 1.00 | 26.83 | C |
| ATOM | 7840 | O   | ARG | E | 155 | 33.570 | 37.257 | 62.449 | 1.00 | 25.48 | O |
| ATOM | 7841 | CB  | ARG | E | 155 | 34.140 | 37.441 | 65.418 | 1.00 | 26.75 | C |
| ATOM | 7842 | CG  | ARG | E | 155 | 32.697 | 37.426 | 65.851 | 1.00 | 28.07 | C |
| ATOM | 7843 | CD  | ARG | E | 155 | 32.401 | 36.219 | 66.697 | 1.00 | 29.38 | C |
| ATOM | 7844 | NE  | ARG | E | 155 | 31.041 | 36.272 | 67.216 | 1.00 | 31.29 | N |
| ATOM | 7845 | CZ  | ARG | E | 155 | 30.707 | 36.218 | 68.505 | 1.00 | 29.88 | C |
| ATOM | 7846 | NH1 | ARG | E | 155 | 31.623 | 36.096 | 69.457 | 1.00 | 30.33 | N |
| ATOM | 7847 | NH2 | ARG | E | 155 | 29.427 | 36.271 | 68.829 | 1.00 | 32.07 | N |
| ATOM | 7848 | N   | GLN | E | 156 | 32.913 | 35.186 | 63.061 | 1.00 | 27.50 | N |
| ATOM | 7849 | CA  | GLN | E | 156 | 31.997 | 35.067 | 61.929 | 1.00 | 30.12 | C |
| ATOM | 7850 | C   | GLN | E | 156 | 30.497 | 35.068 | 62.292 | 1.00 | 31.40 | C |
| ATOM | 7851 | O   | GLN | E | 156 | 29.681 | 35.659 | 61.570 | 1.00 | 33.62 | O |
| ATOM | 7852 | CB  | GLN | E | 156 | 32.369 | 33.845 | 61.106 | 1.00 | 31.49 | C |
| ATOM | 7853 | CG  | GLN | E | 156 | 33.746 | 33.968 | 60.495 | 1.00 | 33.11 | C |

| ATOM | 7854 | CD  | GLN | E | 156 | 34.263 | 32.669 | 59.967 | 1.00 | 35.74 | C |
| ATOM | 7855 | OE1 | GLN | E | 156 | 33.526 | 31.939 | 59.308 | 1.00 | 38.86 | O |
| ATOM | 7856 | NE2 | GLN | E | 156 | 35.536 | 32.353 | 60.262 | 1.00 | 35.75 | N |
| ATOM | 7857 | N   | ASN | E | 157 | 30.099 | 34.435 | 63.386 | 1.00 | 32.28 | N |
| ATOM | 7858 | CA  | ASN | E | 157 | 28.655 | 34.443 | 63.693 | 1.00 | 32.98 | C |
| ATOM | 7859 | C   | ASN | E | 157 | 28.175 | 35.714 | 64.400 | 1.00 | 30.48 | C |
| ATOM | 7860 | O   | ASN | E | 157 | 28.927 | 36.343 | 65.148 | 1.00 | 30.38 | O |
| ATOM | 7861 | CB  | ASN | E | 157 | 28.237 | 33.160 | 64.399 | 1.00 | 35.29 | C |
| ATOM | 7862 | CG  | ASN | E | 157 | 28.054 | 32.005 | 63.419 | 1.00 | 37.20 | C |
| ATOM | 7863 | OD1 | ASN | E | 157 | 28.603 | 32.026 | 62.304 | 1.00 | 41.40 | O |
| ATOM | 7864 | ND2 | ASN | E | 157 | 27.266 | 31.010 | 63.814 | 1.00 | 39.59 | N |
| ATOM | 7865 | N   | GLY | E | 158 | 26.931 | 36.100 | 64.102 | 1.00 | 28.11 | N |
| ATOM | 7866 | CA  | GLY | E | 158 | 26.359 | 37.374 | 64.532 | 1.00 | 26.91 | C |
| ATOM | 7867 | C   | GLY | E | 158 | 26.641 | 38.536 | 63.591 | 1.00 | 25.59 | C |
| ATOM | 7868 | O   | GLY | E | 158 | 26.337 | 39.684 | 63.914 | 1.00 | 24.31 | O |
| ATOM | 7869 | N   | VAL | E | 159 | 27.230 | 38.245 | 62.431 | 1.00 | 25.37 | N |
| ATOM | 7870 | CA  | VAL | E | 159 | 27.615 | 39.281 | 61.457 | 1.00 | 25.22 | C |
| ATOM | 7871 | C   | VAL | E | 159 | 26.610 | 39.324 | 60.297 | 1.00 | 26.08 | C |
| ATOM | 7872 | O   | VAL | E | 159 | 26.352 | 38.306 | 59.678 | 1.00 | 27.49 | O |
| ATOM | 7873 | CB  | VAL | E | 159 | 29.059 | 39.065 | 60.901 | 1.00 | 23.88 | C |
| ATOM | 7874 | CG1 | VAL | E | 159 | 29.390 | 40.078 | 59.804 | 1.00 | 23.66 | C |
| ATOM | 7875 | CG2 | VAL | E | 159 | 30.100 | 39.140 | 62.009 | 1.00 | 24.52 | C |
| ATOM | 7876 | N   | LEU | E | 160 | 26.046 | 40.507 | 60.047 | 1.00 | 25.81 | N |
| ATOM | 7877 | CA  | LEU | E | 160 | 25.126 | 40.764 | 58.949 | 1.00 | 27.61 | C |
| ATOM | 7878 | C   | LEU | E | 160 | 25.639 | 41.899 | 58.060 | 1.00 | 26.13 | C |
| ATOM | 7879 | O   | LEU | E | 160 | 25.897 | 43.015 | 58.528 | 1.00 | 25.33 | O |
| ATOM | 7880 | CB  | LEU | E | 160 | 23.742 | 41.163 | 59.480 | 1.00 | 29.13 | C |
| ATOM | 7881 | CG  | LEU | E | 160 | 22.913 | 40.138 | 60.274 | 1.00 | 31.65 | C |
| ATOM | 7882 | CD1 | LEU | E | 160 | 21.482 | 40.626 | 60.333 | 1.00 | 33.09 | C |
| ATOM | 7883 | CD2 | LEU | E | 160 | 22.945 | 38.711 | 59.701 | 1.00 | 32.17 | C |
| ATOM | 7884 | N   | ASN | E | 161 | 25.743 | 41.596 | 56.769 | 1.00 | 26.35 | N |
| ATOM | 7885 | CA  | ASN | E | 161 | 26.305 | 42.486 | 55.765 | 1.00 | 25.21 | C |
| ATOM | 7886 | C   | ASN | E | 161 | 25.243 | 42.866 | 54.753 | 1.00 | 26.11 | C |
| ATOM | 7887 | O   | ASN | E | 161 | 24.438 | 42.014 | 54.327 | 1.00 | 24.98 | O |
| ATOM | 7888 | CB  | ASN | E | 161 | 27.424 | 41.793 | 54.978 | 1.00 | 24.74 | C |
| ATOM | 7889 | CG  | ASN | E | 161 | 28.575 | 41.358 | 55.838 | 1.00 | 25.20 | C |
| ATOM | 7890 | OD1 | ASN | E | 161 | 29.142 | 40.280 | 55.641 | 1.00 | 29.54 | O |
| ATOM | 7891 | ND2 | ASN | E | 161 | 28.933 | 42.170 | 56.779 | 1.00 | 23.18 | N |
| ATOM | 7892 | N   | SER | E | 162 | 25.292 | 44.124 | 54.344 | 1.00 | 25.16 | N |
| ATOM | 7893 | CA  | SER | E | 162 | 24.412 | 44.663 | 53.331 | 1.00 | 26.76 | C |
| ATOM | 7894 | C   | SER | E | 162 | 25.168 | 45.584 | 52.373 | 1.00 | 27.76 | C |
| ATOM | 7895 | O   | SER | E | 162 | 25.944 | 46.429 | 52.802 | 1.00 | 28.17 | O |
| ATOM | 7896 | CB  | SER | E | 162 | 23.299 | 45.457 | 53.989 | 1.00 | 25.63 | C |
| ATOM | 7897 | OG  | SER | E | 162 | 22.257 | 45.606 | 53.062 | 1.00 | 27.65 | O |
| ATOM | 7898 | N   | TRP | E | 163 | 24.898 | 45.440 | 51.081 | 1.00 | 28.70 | N |
| ATOM | 7899 | CA  | TRP | E | 163 | 25.577 | 46.215 | 50.053 | 1.00 | 29.30 | C |
| ATOM | 7900 | C   | TRP | E | 163 | 24.590 | 47.170 | 49.384 | 1.00 | 28.15 | C |
| ATOM | 7901 | O   | TRP | E | 163 | 23.458 | 46.796 | 49.088 | 1.00 | 24.95 | O |
| ATOM | 7902 | CB  | TRP | E | 163 | 26.192 | 45.243 | 49.033 | 1.00 | 32.03 | C |
| ATOM | 7903 | CG  | TRP | E | 163 | 27.170 | 44.259 | 49.654 | 1.00 | 33.12 | C |
| ATOM | 7904 | CD1 | TRP | E | 163 | 28.529 | 44.356 | 49.663 | 1.00 | 34.00 | C |
| ATOM | 7905 | CD2 | TRP | E | 163 | 26.851 | 43.054 | 50.371 | 1.00 | 33.73 | C |
| ATOM | 7906 | NE1 | TRP | E | 163 | 29.079 | 43.287 | 50.331 | 1.00 | 34.50 | N |
| ATOM | 7907 | CE2 | TRP | E | 163 | 28.072 | 42.478 | 50.783 | 1.00 | 33.60 | C |
| ATOM | 7908 | CE3 | TRP | E | 163 | 25.647 | 42.405 | 50.704 | 1.00 | 34.77 | C |
| ATOM | 7909 | CZ2 | TRP | E | 163 | 28.136 | 41.276 | 51.498 | 1.00 | 33.89 | C |
| ATOM | 7910 | CZ3 | TRP | E | 163 | 25.708 | 41.205 | 51.428 | 1.00 | 34.24 | C |
| ATOM | 7911 | CH2 | TRP | E | 163 | 26.950 | 40.658 | 51.814 | 1.00 | 34.76 | C |
| ATOM | 7912 | N   | THR | E | 164 | 24.995 | 48.418 | 49.161 | 1.00 | 26.45 | N |
| ATOM | 7913 | CA  | THR | E | 164 | 24.173 | 49.328 | 48.368 | 1.00 | 26.79 | C |
| ATOM | 7914 | C   | THR | E | 164 | 24.260 | 48.984 | 46.865 | 1.00 | 26.52 | C |
| ATOM | 7915 | O   | THR | E | 164 | 25.136 | 48.226 | 46.432 | 1.00 | 24.35 | O |
| ATOM | 7916 | CB  | THR | E | 164 | 24.570 | 50.790 | 48.570 | 1.00 | 26.45 | C |
| ATOM | 7917 | OG1 | THR | E | 164 | 25.957 | 50.955 | 48.261 | 1.00 | 29.30 | O |
| ATOM | 7918 | CG2 | THR | E | 164 | 24.323 | 51.221 | 49.996 | 1.00 | 26.27 | C |

```
ATOM   7919   N    ASP E 165    23.284   49.490   46.111   1.00  27.27        N
ATOM   7920   CA   ASP E 165    23.360   49.571   44.650   1.00  27.52        C
ATOM   7921   C    ASP E 165    24.371   50.656   44.286   1.00  26.40        C
ATOM   7922   O    ASP E 165    24.666   51.557   45.081   1.00  26.45        O
ATOM   7923   CB   ASP E 165    22.021   50.028   44.047   1.00  28.44        C
ATOM   7924   CG   ASP E 165    20.890   49.056   44.279   1.00  30.57        C
ATOM   7925   OD1  ASP E 165    21.136   47.823   44.315   1.00  32.03        O
ATOM   7926   OD2  ASP E 165    19.730   49.539   44.378   1.00  34.87        O
ATOM   7927   N    GLN E 166    24.864   50.601   43.057   1.00  25.66        N
ATOM   7928   CA   GLN E 166    25.797   51.609   42.595   1.00  24.88        C
ATOM   7929   C    GLN E 166    25.216   53.012   42.755   1.00  25.38        C
ATOM   7930   O    GLN E 166    24.050   53.257   42.413   1.00  26.06        O
ATOM   7931   CB   GLN E 166    26.154   51.359   41.134   1.00  24.75        C
ATOM   7932   CG   GLN E 166    27.310   52.214   40.667   1.00  24.35        C
ATOM   7933   CD   GLN E 166    27.872   51.724   39.369   1.00  24.52        C
ATOM   7934   OE1  GLN E 166    27.126   51.246   38.504   1.00  23.23        O
ATOM   7935   NE2  GLN E 166    29.204   51.797   39.227   1.00  21.90        N
ATOM   7936   N    ASP E 167    26.023   53.938   43.274   1.00  26.84        N
ATOM   7937   CA   ASP E 167    25.567   55.302   43.545   1.00  28.67        C
ATOM   7938   C    ASP E 167    25.342   56.116   42.253   1.00  29.39        C
ATOM   7939   O    ASP E 167    26.200   56.126   41.358   1.00  27.46        O
ATOM   7940   CB   ASP E 167    26.574   56.028   44.446   1.00  29.72        C
ATOM   7941   CG   ASP E 167    26.088   57.396   44.885   1.00  33.30        C
ATOM   7942   OD1  ASP E 167    25.253   57.448   45.817   1.00  38.21        O
ATOM   7943   OD2  ASP E 167    26.531   58.425   44.316   1.00  32.91        O
ATOM   7944   N    SER E 168    24.189   56.788   42.175   1.00  28.98        N
ATOM   7945   CA   SER E 168    23.804   57.603   41.005   1.00  29.92        C
ATOM   7946   C    SER E 168    24.610   58.888   40.806   1.00  30.18        C
ATOM   7947   O    SER E 168    24.506   59.533   39.757   1.00  28.31        O
ATOM   7948   CB   SER E 168    22.329   57.988   41.093   1.00  30.37        C
ATOM   7949   OG   SER E 168    21.500   56.833   41.078   1.00  32.25        O
ATOM   7950   N    LYS E 169    25.396   59.276   41.809   1.00  30.89        N
ATOM   7951   CA   LYS E 169    26.199   60.490   41.696   1.00  31.89        C
ATOM   7952   C    LYS E 169    27.676   60.186   41.427   1.00  30.78        C
ATOM   7953   O    LYS E 169    28.264   60.781   40.536   1.00  30.87        O
ATOM   7954   CB   LYS E 169    26.017   61.383   42.934   1.00  34.03        C
ATOM   7955   CG   LYS E 169    25.159   62.645   42.714   1.00  36.60        C
ATOM   7956   CD   LYS E 169    23.640   62.411   42.836   1.00  38.27        C
ATOM   7957   CE   LYS E 169    22.909   63.625   43.478   1.00  38.14        C
ATOM   7958   NZ   LYS E 169    23.018   64.921   42.714   1.00  40.13        N
ATOM   7959   N    ASP E 170    28.271   59.242   42.148   1.00  29.92        N
ATOM   7960   CA   ASP E 170    29.699   58.979   41.977   1.00  28.31        C
ATOM   7961   C    ASP E 170    30.072   57.571   41.502   1.00  27.34        C
ATOM   7962   O    ASP E 170    31.253   57.259   41.382   1.00  27.34        O
ATOM   7963   CB   ASP E 170    30.462   59.350   43.256   1.00  29.75        C
ATOM   7964   CG   ASP E 170    30.299   58.340   44.373   1.00  31.83        C
ATOM   7965   OD1  ASP E 170    29.573   57.335   44.212   1.00  31.65        O
ATOM   7966   OD2  ASP E 170    30.914   58.561   45.440   1.00  35.02        O
ATOM   7967   N    SER E 171    29.075   56.729   41.234   1.00  24.76        N
ATOM   7968   CA   SER E 171    29.290   55.390   40.652   1.00  24.68        C
ATOM   7969   C    SER E 171    30.067   54.433   41.557   1.00  23.71        C
ATOM   7970   O    SER E 171    30.606   53.424   41.099   1.00  23.91        O
ATOM   7971   CB   SER E 171    29.931   55.505   39.252   1.00  23.56        C
ATOM   7972   OG   SER E 171    29.209   56.440   38.461   1.00  21.78        O
ATOM   7973   N    THR E 172    30.073   54.726   42.857   1.00  24.92        N
ATOM   7974   CA   THR E 172    30.699   53.843   43.841   1.00  24.85        C
ATOM   7975   C    THR E 172    29.689   52.886   44.507   1.00  24.26        C
ATOM   7976   O    THR E 172    28.467   52.962   44.287   1.00  23.42        O
ATOM   7977   CB   THR E 172    31.407   54.657   44.942   1.00  25.06        C
ATOM   7978   OG1  THR E 172    30.470   55.503   45.625   1.00  25.98        O
ATOM   7979   CG2  THR E 172    32.531   55.490   44.323   1.00  24.37        C
ATOM   7980   N    TYR E 173    30.242   51.980   45.302   1.00  23.34        N
ATOM   7981   CA   TYR E 173    29.485   51.068   46.126   1.00  24.15        C
ATOM   7982   C    TYR E 173    29.865   51.258   47.579   1.00  24.80        C
ATOM   7983   O    TYR E 173    30.969   51.711   47.889   1.00  24.19        O
```

```
ATOM   7984  CB   TYR E 173      29.803  49.641  45.724  1.00 25.10           C
ATOM   7985  CG   TYR E 173      29.403  49.324  44.324  1.00 26.74           C
ATOM   7986  CD1  TYR E 173      30.292  49.515  43.277  1.00 27.01           C
ATOM   7987  CD2  TYR E 173      28.122  48.849  44.035  1.00 27.54           C
ATOM   7988  CE1  TYR E 173      29.932  49.232  41.989  1.00 27.48           C
ATOM   7989  CE2  TYR E 173      27.754  48.556  42.738  1.00 27.61           C
ATOM   7990  CZ   TYR E 173      28.667  48.752  41.720  1.00 27.15           C
ATOM   7991  OH   TYR E 173      28.342  48.474  40.415  1.00 27.69           O
ATOM   7992  N    SER E 174      28.929  50.900  48.456  1.00 24.98           N
ATOM   7993  CA   SER E 174      29.136  50.903  49.897  1.00 23.70           C
ATOM   7994  C    SER E 174      28.612  49.611  50.488  1.00 24.16           C
ATOM   7995  O    SER E 174      27.769  48.916  49.880  1.00 21.11           O
ATOM   7996  CB   SER E 174      28.415  52.072  50.531  1.00 23.60           C
ATOM   7997  OG   SER E 174      28.991  53.285  50.097  1.00 22.60           O
ATOM   7998  N    MET E 175      29.110  49.308  51.682  1.00 24.00           N
ATOM   7999  CA   MET E 175      28.785  48.075  52.389  1.00 25.21           C
ATOM   8000  C    MET E 175      28.698  48.435  53.871  1.00 23.76           C
ATOM   8001  O    MET E 175      29.493  49.237  54.373  1.00 20.56           O
ATOM   8002  CB   MET E 175      29.874  47.029  52.156  1.00 26.20           C
ATOM   8003  CG   MET E 175      29.728  45.732  52.972  1.00 28.12           C
ATOM   8004  SD   MET E 175      31.107  44.587  52.772  1.00 32.77           S
ATOM   8005  CE   MET E 175      30.517  43.201  53.719  1.00 31.45           C
ATOM   8006  N    SER E 176      27.708  47.884  54.557  1.00 22.62           N
ATOM   8007  CA   SER E 176      27.666  47.978  56.022  1.00 23.35           C
ATOM   8008  C    SER E 176      27.829  46.581  56.564  1.00 21.75           C
ATOM   8009  O    SER E 176      27.318  45.626  55.988  1.00 21.32           O
ATOM   8010  CB   SER E 176      26.339  48.515  56.497  1.00 24.36           C
ATOM   8011  OG   SER E 176      25.346  47.564  56.193  1.00 28.30           O
ATOM   8012  N    SER E 177      28.549  46.457  57.674  1.00 20.80           N
ATOM   8013  CA   SER E 177      28.676  45.171  58.347  1.00 20.34           C
ATOM   8014  C    SER E 177      28.337  45.409  59.819  1.00 20.45           C
ATOM   8015  O    SER E 177      28.884  46.322  60.413  1.00 18.81           O
ATOM   8016  CB   SER E 177      30.082  44.628  58.211  1.00 20.20           C
ATOM   8017  OG   SER E 177      30.138  43.287  58.663  1.00 19.76           O
ATOM   8018  N    THR E 178      27.429  44.607  60.370  1.00 20.98           N
ATOM   8019  CA   THR E 178      27.005  44.769  61.770  1.00 20.76           C
ATOM   8020  C    THR E 178      27.258  43.471  62.533  1.00 20.30           C
ATOM   8021  O    THR E 178      26.828  42.385  62.123  1.00 20.00           O
ATOM   8022  CB   THR E 178      25.527  45.170  61.855  1.00 22.60           C
ATOM   8023  OG1  THR E 178      25.323  46.379  61.118  1.00 23.96           O
ATOM   8024  CG2  THR E 178      25.070  45.388  63.300  1.00 22.41           C
ATOM   8025  N    LEU E 179      27.975  43.591  63.639  1.00 19.40           N
ATOM   8026  CA   LEU E 179      28.232  42.475  64.520  1.00 19.30           C
ATOM   8027  C    LEU E 179      27.339  42.675  65.734  1.00 19.60           C
ATOM   8028  O    LEU E 179      27.503  43.661  66.460  1.00 20.17           O
ATOM   8029  CB   LEU E 179      29.702  42.464  64.928  1.00 19.77           C
ATOM   8030  CG   LEU E 179      30.123  41.567  66.091  1.00 20.26           C
ATOM   8031  CD1  LEU E 179      29.906  40.118  65.789  1.00 20.24           C
ATOM   8032  CD2  LEU E 179      31.580  41.821  66.476  1.00 20.66           C
ATOM   8033  N    THR E 180      26.367  41.785  65.922  1.00 19.54           N
ATOM   8034  CA   THR E 180      25.437  41.924  67.044  1.00 19.35           C
ATOM   8035  C    THR E 180      25.652  40.829  68.079  1.00 18.46           C
ATOM   8036  O    THR E 180      25.670  39.623  67.771  1.00 18.37           O
ATOM   8037  CB   THR E 180      23.979  41.999  66.617  1.00 20.84           C
ATOM   8038  OG1  THR E 180      23.780  43.154  65.778  1.00 20.55           O
ATOM   8039  CG2  THR E 180      23.092  42.162  67.856  1.00 19.80           C
ATOM   8040  N    LEU E 181      25.827  41.290  69.306  1.00 17.74           N
ATOM   8041  CA   LEU E 181      26.037  40.455  70.466  1.00 17.93           C
ATOM   8042  C    LEU E 181      24.996  40.793  71.523  1.00 16.65           C
ATOM   8043  O    LEU E 181      24.351  41.837  71.475  1.00 16.08           O
ATOM   8044  CB   LEU E 181      27.410  40.749  71.048  1.00 19.45           C
ATOM   8045  CG   LEU E 181      28.578  40.745  70.057  1.00 22.26           C
ATOM   8046  CD1  LEU E 181      29.821  41.304  70.693  1.00 24.75           C
ATOM   8047  CD2  LEU E 181      28.817  39.321  69.565  1.00 22.62           C
ATOM   8048  N    THR E 182      24.841  39.914  72.499  1.00 15.98           N
```

```
ATOM   8049  CA   THR E 182     24.127  40.281  73.714  1.00 15.26           C
ATOM   8050  C    THR E 182     25.064  41.168  74.507  1.00 14.43           C
ATOM   8051  O    THR E 182     26.260  41.062  74.325  1.00 15.41           O
ATOM   8052  CB   THR E 182     23.828  39.053  74.552  1.00 17.50           C
ATOM   8053  OG1  THR E 182     25.020  38.298  74.755  1.00 16.85           O
ATOM   8054  CG2  THR E 182     22.804  38.193  73.877  1.00 20.50           C
ATOM   8055  N    LYS E 183     24.541  41.989  75.418  1.00 12.31           N
ATOM   8056  CA   LYS E 183     25.384  42.776  76.310  1.00 13.04           C
ATOM   8057  C    LYS E 183     26.311  41.885  77.114  1.00 12.53           C
ATOM   8058  O    LYS E 183     27.484  42.191  77.279  1.00 12.91           O
ATOM   8059  CB   LYS E 183     24.544  43.652  77.252  1.00 12.43           C
ATOM   8060  CG   LYS E 183     25.340  44.505  78.234  1.00 13.46           C
ATOM   8061  CD   LYS E 183     24.462  45.137  79.279  1.00 15.63           C
ATOM   8062  CE   LYS E 183     25.298  45.655  80.430  1.00 17.28           C
ATOM   8063  NZ   LYS E 183     24.562  46.009  81.627  1.00 17.06           N
ATOM   8064  N    ASP E 184     25.826  40.746  77.593  1.00 11.42           N
ATOM   8065  CA   ASP E 184     26.692  39.906  78.404  1.00 12.96           C
ATOM   8066  C    ASP E 184     27.949  39.504  77.634  1.00 12.14           C
ATOM   8067  O    ASP E 184     29.018  39.609  78.156  1.00 14.82           O
ATOM   8068  CB   ASP E 184     25.967  38.707  79.049  1.00 12.44           C
ATOM   8069  CG   ASP E 184     25.616  37.584  78.082  1.00 18.28           C
ATOM   8070  OD1  ASP E 184     26.034  36.434  78.373  1.00 19.04           O
ATOM   8071  OD2  ASP E 184     24.880  37.805  77.084  1.00 19.35           O
ATOM   8072  N    GLU E 185     27.809  39.055  76.397  1.00 14.73           N
ATOM   8073  CA   GLU E 185     28.972  38.605  75.631  1.00 15.54           C
ATOM   8074  C    GLU E 185     29.864  39.795  75.256  1.00 15.62           C
ATOM   8075  O    GLU E 185     31.074  39.698  75.369  1.00 16.15           O
ATOM   8076  CB   GLU E 185     28.572  37.807  74.385  1.00 16.51           C
ATOM   8077  CG   GLU E 185     29.792  37.337  73.582  1.00 18.25           C
ATOM   8078  CD   GLU E 185     29.486  36.389  72.414  1.00 21.21           C
ATOM   8079  OE1  GLU E 185     28.363  36.381  71.895  1.00 26.05           O
ATOM   8080  OE2  GLU E 185     30.422  35.669  72.008  1.00 27.50           O
ATOM   8081  N    TYR E 186     29.268  40.913  74.833  1.00 14.76           N
ATOM   8082  CA   TYR E 186     30.038  42.129  74.538  1.00 14.16           C
ATOM   8083  C    TYR E 186     30.952  42.493  75.704  1.00 13.91           C
ATOM   8084  O    TYR E 186     32.099  42.869  75.499  1.00 15.06           O
ATOM   8085  CB   TYR E 186     29.084  43.269  74.214  1.00 13.99           C
ATOM   8086  CG   TYR E 186     29.733  44.587  73.957  1.00 11.86           C
ATOM   8087  CD1  TYR E 186     30.528  44.776  72.829  1.00 12.45           C
ATOM   8088  CD2  TYR E 186     29.563  45.645  74.819  1.00 13.16           C
ATOM   8089  CE1  TYR E 186     31.146  45.994  72.581  1.00 14.03           C
ATOM   8090  CE2  TYR E 186     30.148  46.865  74.564  1.00 13.12           C
ATOM   8091  CZ   TYR E 186     30.945  47.021  73.443  1.00 12.58           C
ATOM   8092  OH   TYR E 186     31.544  48.233  73.199  1.00 15.81           O
ATOM   8093  N    GLU E 187     30.444  42.366  76.931  1.00 15.09           N
ATOM   8094  CA   GLU E 187     31.166  42.833  78.115  1.00 16.37           C
ATOM   8095  C    GLU E 187     32.198  41.813  78.610  1.00 16.76           C
ATOM   8096  O    GLU E 187     33.001  42.111  79.500  1.00 16.82           O
ATOM   8097  CB   GLU E 187     30.167  43.266  79.198  1.00 16.05           C
ATOM   8098  CG   GLU E 187     29.415  44.512  78.768  1.00 17.46           C
ATOM   8099  CD   GLU E 187     28.589  45.197  79.831  1.00 20.44           C
ATOM   8100  OE1  GLU E 187     28.142  44.568  80.841  1.00 21.01           O
ATOM   8101  OE2  GLU E 187     28.358  46.421  79.608  1.00 21.49           O
ATOM   8102  N    ARG E 188     32.198  40.618  78.026  1.00 17.17           N
ATOM   8103  CA   ARG E 188     33.264  39.638  78.294  1.00 18.32           C
ATOM   8104  C    ARG E 188     34.526  39.839  77.412  1.00 18.57           C
ATOM   8105  O    ARG E 188     35.542  39.149  77.581  1.00 18.28           O
ATOM   8106  CB   ARG E 188     32.731  38.224  78.133  1.00 18.34           C
ATOM   8107  CG   ARG E 188     31.680  37.822  79.149  1.00 19.35           C
ATOM   8108  CD   ARG E 188     31.599  36.312  79.292  1.00 19.05           C
ATOM   8109  NE   ARG E 188     31.104  35.686  78.072  1.00 20.63           N
ATOM   8110  CZ   ARG E 188     29.824  35.574  77.756  1.00 19.14           C
ATOM   8111  NH1  ARG E 188     28.900  36.052  78.560  1.00 19.35           N
ATOM   8112  NH2  ARG E 188     29.473  35.005  76.611  1.00 21.45           N
ATOM   8113  N    HIS E 189     34.481  40.784  76.486  1.00 17.89           N
```

383

| ATOM | 8114 | CA | HIS | E | 189 | 35.613 | 41.036 | 75.605 | 1.00 | 20.01 | C |
|------|------|------|-----|---|-----|--------|--------|--------|------|-------|---|
| ATOM | 8115 | C | HIS | E | 189 | 35.985 | 42.507 | 75.597 | 1.00 | 19.92 | C |
| ATOM | 8116 | O | HIS | E | 189 | 35.296 | 43.322 | 76.185 | 1.00 | 20.88 | O |
| ATOM | 8117 | CB | HIS | E | 189 | 35.277 | 40.529 | 74.214 | 1.00 | 21.14 | C |
| ATOM | 8118 | CG | HIS | E | 189 | 35.019 | 39.060 | 74.182 | 1.00 | 22.53 | C |
| ATOM | 8119 | ND1 | HIS | E | 189 | 36.035 | 38.131 | 74.251 | 1.00 | 24.46 | N |
| ATOM | 8120 | CD2 | HIS | E | 189 | 33.865 | 38.359 | 74.157 | 1.00 | 23.74 | C |
| ATOM | 8121 | CE1 | HIS | E | 189 | 35.515 | 36.917 | 74.228 | 1.00 | 24.36 | C |
| ATOM | 8122 | NE2 | HIS | E | 189 | 34.200 | 37.025 | 74.171 | 1.00 | 25.03 | N |
| ATOM | 8123 | N | ASN | E | 190 | 37.088 | 42.850 | 74.957 | 1.00 | 19.57 | N |
| ATOM | 8124 | CA | ASN | E | 190 | 37.542 | 44.235 | 74.982 | 1.00 | 19.25 | C |
| ATOM | 8125 | C | ASN | E | 190 | 37.752 | 44.869 | 73.626 | 1.00 | 18.94 | C |
| ATOM | 8126 | O | ASN | E | 190 | 37.414 | 46.026 | 73.448 | 1.00 | 20.20 | O |
| ATOM | 8127 | CB | ASN | E | 190 | 38.815 | 44.346 | 75.811 | 1.00 | 20.86 | C |
| ATOM | 8128 | CG | ASN | E | 190 | 38.568 | 44.137 | 77.316 | 1.00 | 23.45 | C |
| ATOM | 8129 | OD1 | ASN | E | 190 | 38.512 | 43.003 | 77.805 | 1.00 | 27.86 | O |
| ATOM | 8130 | ND2 | ASN | E | 190 | 38.442 | 45.237 | 78.049 | 1.00 | 26.66 | N |
| ATOM | 8131 | N | SER | E | 191 | 38.314 | 44.123 | 72.688 | 1.00 | 19.37 | N |
| ATOM | 8132 | CA | SER | E | 191 | 38.775 | 44.704 | 71.432 | 1.00 | 20.65 | C |
| ATOM | 8133 | C | SER | E | 191 | 37.924 | 44.245 | 70.290 | 1.00 | 18.31 | C |
| ATOM | 8134 | O | SER | E | 191 | 37.822 | 43.053 | 70.058 | 1.00 | 18.35 | O |
| ATOM | 8135 | CB | SER | E | 191 | 40.225 | 44.294 | 71.150 | 1.00 | 22.06 | C |
| ATOM | 8136 | OG | SER | E | 191 | 40.863 | 45.316 | 70.410 | 1.00 | 30.05 | O |
| ATOM | 8137 | N | TYR | E | 192 | 37.388 | 45.206 | 69.543 | 1.00 | 16.91 | N |
| ATOM | 8138 | CA | TYR | E | 192 | 36.517 | 44.939 | 68.403 | 1.00 | 16.78 | C |
| ATOM | 8139 | C | TYR | E | 192 | 37.100 | 45.599 | 67.147 | 1.00 | 17.22 | C |
| ATOM | 8140 | O | TYR | E | 192 | 37.352 | 46.797 | 67.140 | 1.00 | 16.86 | O |
| ATOM | 8141 | CB | TYR | E | 192 | 35.128 | 45.477 | 68.700 | 1.00 | 16.58 | C |
| ATOM | 8142 | CG | TYR | E | 192 | 34.499 | 44.714 | 69.846 | 1.00 | 15.14 | C |
| ATOM | 8143 | CD1 | TYR | E | 192 | 33.750 | 43.595 | 69.603 | 1.00 | 15.92 | C |
| ATOM | 8144 | CD2 | TYR | E | 192 | 34.692 | 45.103 | 71.172 | 1.00 | 16.24 | C |
| ATOM | 8145 | CE1 | TYR | E | 192 | 33.196 | 42.856 | 70.645 | 1.00 | 16.17 | C |
| ATOM | 8146 | CE2 | TYR | E | 192 | 34.133 | 44.372 | 72.224 | 1.00 | 16.08 | C |
| ATOM | 8147 | CZ | TYR | E | 192 | 33.403 | 43.241 | 71.948 | 1.00 | 15.65 | C |
| ATOM | 8148 | OH | TYR | E | 192 | 32.814 | 42.482 | 72.969 | 1.00 | 15.50 | O |
| ATOM | 8149 | N | THR | E | 193 | 37.313 | 44.780 | 66.121 | 1.00 | 19.25 | N |
| ATOM | 8150 | CA | THR | E | 193 | 38.042 | 45.179 | 64.931 | 1.00 | 19.43 | C |
| ATOM | 8151 | C | THR | E | 193 | 37.273 | 44.808 | 63.672 | 1.00 | 19.81 | C |
| ATOM | 8152 | O | THR | E | 193 | 36.828 | 43.672 | 63.507 | 1.00 | 19.64 | O |
| ATOM | 8153 | CB | THR | E | 193 | 39.423 | 44.484 | 64.882 | 1.00 | 20.28 | C |
| ATOM | 8154 | OG1 | THR | E | 193 | 40.199 | 44.832 | 66.047 | 1.00 | 21.97 | O |
| ATOM | 8155 | CG2 | THR | E | 193 | 40.175 | 44.871 | 63.629 | 1.00 | 19.52 | C |
| ATOM | 8156 | N | CYS | E | 194 | 37.189 | 45.770 | 62.766 | 1.00 | 21.83 | N |
| ATOM | 8157 | CA | CYS | E | 194 | 36.676 | 45.592 | 61.409 | 1.00 | 21.18 | C |
| ATOM | 8158 | C | CYS | E | 194 | 37.841 | 45.729 | 60.413 | 1.00 | 20.38 | C |
| ATOM | 8159 | O | CYS | E | 194 | 38.527 | 46.737 | 60.447 | 1.00 | 16.97 | O |
| ATOM | 8160 | CB | CYS | E | 194 | 35.667 | 46.731 | 61.139 | 1.00 | 22.38 | C |
| ATOM | 8161 | SG | CYS | E | 194 | 34.906 | 46.599 | 59.585 | 1.00 | 30.65 | S |
| ATOM | 8162 | N | GLU | E | 195 | 38.063 | 44.716 | 59.563 | 1.00 | 20.34 | N |
| ATOM | 8163 | CA | GLU | E | 195 | 39.129 | 44.750 | 58.526 | 1.00 | 21.98 | C |
| ATOM | 8164 | C | GLU | E | 195 | 38.542 | 44.599 | 57.128 | 1.00 | 21.08 | C |
| ATOM | 8165 | O | GLU | E | 195 | 37.933 | 43.574 | 56.827 | 1.00 | 20.65 | O |
| ATOM | 8166 | CB | GLU | E | 195 | 40.168 | 43.641 | 58.733 | 1.00 | 21.30 | C |
| ATOM | 8167 | CG | GLU | E | 195 | 40.717 | 43.547 | 60.143 | 1.00 | 23.62 | C |
| ATOM | 8168 | CD | GLU | E | 195 | 41.863 | 42.594 | 60.257 | 1.00 | 25.59 | C |
| ATOM | 8169 | OE1 | GLU | E | 195 | 42.050 | 41.736 | 59.354 | 1.00 | 29.13 | O |
| ATOM | 8170 | OE2 | GLU | E | 195 | 42.580 | 42.692 | 61.265 | 1.00 | 29.64 | O |
| ATOM | 8171 | N | ALA | E | 196 | 38.788 | 45.593 | 56.273 | 1.00 | 22.09 | N |
| ATOM | 8172 | CA | ALA | E | 196 | 38.269 | 45.637 | 54.898 | 1.00 | 22.12 | C |
| ATOM | 8173 | C | ALA | E | 196 | 39.375 | 45.317 | 53.885 | 1.00 | 23.71 | C |
| ATOM | 8174 | O | ALA | E | 196 | 40.386 | 46.018 | 53.861 | 1.00 | 24.81 | O |
| ATOM | 8175 | CB | ALA | E | 196 | 37.726 | 47.023 | 54.605 | 1.00 | 22.05 | C |
| ATOM | 8176 | N | THR | E | 197 | 39.179 | 44.293 | 53.067 | 1.00 | 23.98 | N |
| ATOM | 8177 | CA | THR | E | 197 | 40.143 | 43.914 | 52.036 | 1.00 | 25.46 | C |
| ATOM | 8178 | C | THR | E | 197 | 39.554 | 44.202 | 50.650 | 1.00 | 24.83 | C |

| ATOM | 8179 | O   | THR E 197 | 38.549 | 43.626 | 50.250 | 1.00 24.81 | O |
| ATOM | 8180 | CB  | THR E 197 | 40.559 | 42.426 | 52.148 | 1.00 26.34 | C |
| ATOM | 8181 | OG1 | THR E 197 | 41.292 | 42.220 | 53.362 | 1.00 28.38 | O |
| ATOM | 8182 | CG2 | THR E 197 | 41.455 | 42.011 | 50.968 | 1.00 26.92 | C |
| ATOM | 8183 | N   | HIS E 198 | 40.205 | 45.112 | 49.930 | 1.00 24.94 | N |
| ATOM | 8184 | CA  | HIS E 198 | 39.722 | 45.599 | 48.650 | 1.00 24.98 | C |
| ATOM | 8185 | C   | HIS E 198 | 40.899 | 45.616 | 47.668 | 1.00 26.11 | C |
| ATOM | 8186 | O   | HIS E 198 | 42.054 | 45.788 | 48.073 | 1.00 25.14 | O |
| ATOM | 8187 | CB  | HIS E 198 | 39.153 | 46.998 | 48.814 | 1.00 24.46 | C |
| ATOM | 8188 | CG  | HIS E 198 | 38.407 | 47.494 | 47.613 | 1.00 25.36 | C |
| ATOM | 8189 | ND1 | HIS E 198 | 38.852 | 48.546 | 46.842 | 1.00 25.39 | N |
| ATOM | 8190 | CD2 | HIS E 198 | 37.245 | 47.078 | 47.049 | 1.00 24.98 | C |
| ATOM | 8191 | CE1 | HIS E 198 | 37.990 | 48.763 | 45.865 | 1.00 24.41 | C |
| ATOM | 8192 | NE2 | HIS E 198 | 37.008 | 47.886 | 45.968 | 1.00 24.65 | N |
| ATOM | 8193 | N   | LYS E 199 | 40.602 | 45.437 | 46.386 | 1.00 27.20 | N |
| ATOM | 8194 | CA  | LYS E 199 | 41.657 | 45.302 | 45.389 | 1.00 28.53 | C |
| ATOM | 8195 | C   | LYS E 199 | 42.606 | 46.505 | 45.357 | 1.00 29.06 | C |
| ATOM | 8196 | O   | LYS E 199 | 43.749 | 46.371 | 44.914 | 1.00 30.39 | O |
| ATOM | 8197 | CB  | LYS E 199 | 41.070 | 45.051 | 44.002 | 1.00 28.66 | C |
| ATOM | 8198 | CG  | LYS E 199 | 40.457 | 46.279 | 43.349 | 1.00 29.93 | C |
| ATOM | 8199 | CD  | LYS E 199 | 40.188 | 46.031 | 41.880 | 1.00 30.27 | C |
| ATOM | 8200 | CE  | LYS E 199 | 41.462 | 46.044 | 41.060 | 1.00 31.98 | C |
| ATOM | 8201 | NZ  | LYS E 199 | 41.153 | 45.866 | 39.617 | 1.00 32.19 | N |
| ATOM | 8202 | N   | THR E 200 | 42.136 | 47.662 | 45.815 | 1.00 29.72 | N |
| ATOM | 8203 | CA  | THR E 200 | 42.946 | 48.883 | 45.844 | 1.00 30.60 | C |
| ATOM | 8204 | C   | THR E 200 | 44.179 | 48.812 | 46.768 | 1.00 31.34 | C |
| ATOM | 8205 | O   | THR E 200 | 45.015 | 49.728 | 46.733 | 1.00 32.49 | O |
| ATOM | 8206 | CB  | THR E 200 | 42.126 | 50.132 | 46.240 | 1.00 30.33 | C |
| ATOM | 8207 | OG1 | THR E 200 | 41.333 | 49.849 | 47.402 | 1.00 30.28 | O |
| ATOM | 8208 | CG2 | THR E 200 | 41.217 | 50.599 | 45.101 | 1.00 30.53 | C |
| ATOM | 8209 | N   | SER E 201 | 44.312 | 47.763 | 47.581 | 1.00 31.62 | N |
| ATOM | 8210 | CA  | SER E 201 | 45.488 | 47.630 | 48.438 | 1.00 32.23 | C |
| ATOM | 8211 | C   | SER E 201 | 45.760 | 46.203 | 48.879 | 1.00 32.91 | C |
| ATOM | 8212 | O   | SER E 201 | 44.855 | 45.390 | 49.042 | 1.00 33.32 | O |
| ATOM | 8213 | CB  | SER E 201 | 45.381 | 48.555 | 49.653 | 1.00 32.92 | C |
| ATOM | 8214 | OG  | SER E 201 | 46.520 | 48.451 | 50.497 | 1.00 33.77 | O |
| ATOM | 8215 | N   | THR E 202 | 47.038 | 45.910 | 49.074 | 1.00 33.68 | N |
| ATOM | 8216 | CA  | THR E 202 | 47.457 | 44.604 | 49.570 | 1.00 34.22 | C |
| ATOM | 8217 | C   | THR E 202 | 47.183 | 44.498 | 51.079 | 1.00 33.52 | C |
| ATOM | 8218 | O   | THR E 202 | 46.973 | 43.412 | 51.606 | 1.00 34.95 | O |
| ATOM | 8219 | CB  | THR E 202 | 48.971 | 44.362 | 49.243 | 1.00 34.19 | C |
| ATOM | 8220 | OG1 | THR E 202 | 49.762 | 45.461 | 49.729 | 1.00 36.37 | O |
| ATOM | 8221 | CG2 | THR E 202 | 49.163 | 44.269 | 47.745 | 1.00 34.78 | C |
| ATOM | 8222 | N   | SER E 203 | 47.189 | 45.640 | 51.757 | 1.00 33.27 | N |
| ATOM | 8223 | CA  | SER E 203 | 46.950 | 45.716 | 53.193 | 1.00 32.64 | C |
| ATOM | 8224 | C   | SER E 203 | 45.477 | 45.939 | 53.456 | 1.00 30.69 | C |
| ATOM | 8225 | O   | SER E 203 | 44.864 | 46.784 | 52.795 | 1.00 29.81 | O |
| ATOM | 8226 | CB  | SER E 203 | 47.710 | 46.903 | 53.788 | 1.00 33.02 | C |
| ATOM | 8227 | OG  | SER E 203 | 49.107 | 46.779 | 53.584 | 1.00 36.55 | O |
| ATOM | 8228 | N   | PRO E 204 | 44.897 | 45.219 | 54.440 | 1.00 29.24 | N |
| ATOM | 8229 | CA  | PRO E 204 | 43.526 | 45.580 | 54.774 | 1.00 27.43 | C |
| ATOM | 8230 | C   | PRO E 204 | 43.464 | 46.954 | 55.401 | 1.00 26.36 | C |
| ATOM | 8231 | O   | PRO E 204 | 44.443 | 47.418 | 55.996 | 1.00 25.92 | O |
| ATOM | 8232 | CB  | PRO E 204 | 43.109 | 44.526 | 55.793 | 1.00 27.42 | C |
| ATOM | 8233 | CG  | PRO E 204 | 44.382 | 44.046 | 56.401 | 1.00 29.16 | C |
| ATOM | 8234 | CD  | PRO E 204 | 45.388 | 44.104 | 55.273 | 1.00 28.83 | C |
| ATOM | 8235 | N   | ILE E 205 | 42.322 | 47.605 | 55.258 | 1.00 23.97 | N |
| ATOM | 8236 | CA  | ILE E 205 | 42.063 | 48.856 | 55.941 | 1.00 23.44 | C |
| ATOM | 8237 | C   | ILE E 205 | 41.328 | 48.462 | 57.197 | 1.00 22.51 | C |
| ATOM | 8238 | O   | ILE E 205 | 40.324 | 47.771 | 57.117 | 1.00 22.28 | O |
| ATOM | 8239 | CB  | ILE E 205 | 41.170 | 49.777 | 55.143 | 1.00 24.28 | C |
| ATOM | 8240 | CG1 | ILE E 205 | 41.831 | 50.119 | 53.805 | 1.00 24.71 | C |
| ATOM | 8241 | CG2 | ILE E 205 | 40.868 | 51.045 | 55.958 | 1.00 24.22 | C |
| ATOM | 8242 | CD1 | ILE E 205 | 41.070 | 51.155 | 53.018 | 1.00 25.94 | C |
| ATOM | 8243 | N   | VAL E 206 | 41.842 | 48.900 | 58.339 | 1.00 21.53 | N |

```
ATOM    8244  CA  VAL E 206      41.390  48.416  59.635  1.00 21.33           C
ATOM    8245  C   VAL E 206      40.936  49.554  60.542  1.00 20.21           C
ATOM    8246  O   VAL E 206      41.570  50.619  60.606  1.00 21.12           O
ATOM    8247  CB  VAL E 206      42.530  47.615  60.325  1.00 20.84           C
ATOM    8248  CG1 VAL E 206      42.101  47.175  61.708  1.00 21.30           C
ATOM    8249  CG2 VAL E 206      42.931  46.432  59.471  1.00 21.20           C
ATOM    8250  N   LYS E 207      39.830  49.332  61.255  1.00 19.65           N
ATOM    8251  CA  LYS E 207      39.351  50.243  62.279  1.00 19.81           C
ATOM    8252  C   LYS E 207      38.946  49.406  63.475  1.00 19.85           C
ATOM    8253  O   LYS E 207      38.439  48.299  63.311  1.00 19.14           O
ATOM    8254  CB  LYS E 207      38.173  51.068  61.783  1.00 21.89           C
ATOM    8255  CG  LYS E 207      38.538  51.995  60.623  1.00 23.57           C
ATOM    8256  CD  LYS E 207      39.443  53.134  61.070  1.00 24.53           C
ATOM    8257  CE  LYS E 207      39.827  54.013  59.887  1.00 25.92           C
ATOM    8258  NZ  LYS E 207      40.335  55.360  60.308  1.00 27.86           N
ATOM    8259  N   SER E 208      39.258  49.898  64.664  1.00 20.46           N
ATOM    8260  CA  SER E 208      38.946  49.156  65.882  1.00 22.39           C
ATOM    8261  C   SER E 208      38.623  50.069  67.039  1.00 23.83           C
ATOM    8262  O   SER E 208      38.794  51.296  66.962  1.00 23.18           O
ATOM    8263  CB  SER E 208      40.101  48.197  66.222  1.00 23.47           C
ATOM    8264  OG  SER E 208      41.234  48.869  66.741  1.00 23.82           O
ATOM    8265  N   PHE E 209      38.099  49.494  68.116  1.00 24.87           N
ATOM    8266  CA  PHE E 209      38.029  50.235  69.389  1.00 26.13           C
ATOM    8267  C   PHE E 209      38.115  49.241  70.552  1.00 25.81           C
ATOM    8268  O   PHE E 209      37.863  48.051  70.363  1.00 24.34           O
ATOM    8269  CB  PHE E 209      36.755  51.083  69.457  1.00 26.87           C
ATOM    8270  CG  PHE E 209      35.521  50.271  69.608  1.00 25.68           C
ATOM    8271  CD1 PHE E 209      35.027  49.960  70.866  1.00 27.21           C
ATOM    8272  CD2 PHE E 209      34.904  49.746  68.495  1.00 27.24           C
ATOM    8273  CE1 PHE E 209      33.923  49.158  71.020  1.00 27.33           C
ATOM    8274  CE2 PHE E 209      33.787  48.947  68.629  1.00 29.05           C
ATOM    8275  CZ  PHE E 209      33.298  48.637  69.904  1.00 27.54           C
ATOM    8276  N   ASN E 210      38.529  49.728  71.716  1.00 27.38           N
ATOM    8277  CA  ASN E 210      38.562  48.937  72.947  1.00 27.77           C
ATOM    8278  C   ASN E 210      37.398  49.410  73.784  1.00 25.79           C
ATOM    8279  O   ASN E 210      37.171  50.612  73.947  1.00 25.14           O
ATOM    8280  CB  ASN E 210      39.881  49.093  73.745  1.00 29.28           C
ATOM    8281  CG  ASN E 210      40.075  47.986  74.823  1.00 30.85           C
ATOM    8282  OD1 ASN E 210      39.314  47.899  75.809  1.00 35.29           O
ATOM    8283  ND2 ASN E 210      41.107  47.135  74.632  1.00 35.58           N
ATOM    8284  N   ARG E 211      36.632  48.450  74.280  1.00 25.17           N
ATOM    8285  CA  ARG E 211      35.503  48.746  75.134  1.00 25.65           C
ATOM    8286  C   ARG E 211      36.050  49.325  76.438  1.00 26.29           C
ATOM    8287  O   ARG E 211      35.554  50.326  76.954  1.00 29.45           O
ATOM    8288  CB  ARG E 211      34.706  47.460  75.386  1.00 25.04           C
ATOM    8289  CG  ARG E 211      33.420  47.658  76.146  1.00 26.35           C
ATOM    8290  CD  ARG E 211      32.760  46.328  76.535  1.00 25.84           C
ATOM    8291  NE  ARG E 211      33.677  45.510  77.293  1.00 26.71           N
ATOM    8292  CZ  ARG E 211      33.807  45.526  78.609  1.00 26.02           C
ATOM    8293  NH1 ARG E 211      33.041  46.299  79.366  1.00 27.02           N
ATOM    8294  NH2 ARG E 211      34.716  44.755  79.168  1.00 28.74           N
TER     8295      ARG E 211
ATOM    8296  N   GLU F   1       0.930  68.983  37.175  1.00 37.63           N
ATOM    8297  CA  GLU F   1       1.837  67.791  37.194  1.00 37.20           C
ATOM    8298  C   GLU F   1       1.066  66.497  37.529  1.00 34.74           C
ATOM    8299  O   GLU F   1       0.235  66.475  38.430  1.00 33.90           O
ATOM    8300  CB  GLU F   1       2.966  68.008  38.208  1.00 39.02           C
ATOM    8301  CG  GLU F   1       4.336  68.327  37.603  1.00 41.92           C
ATOM    8302  CD  GLU F   1       4.484  69.752  37.078  1.00 44.72           C
ATOM    8303  OE1 GLU F   1       3.617  70.617  37.369  1.00 47.88           O
ATOM    8304  OE2 GLU F   1       5.490  70.011  36.376  1.00 46.90           O
ATOM    8305  N   VAL F   2       1.348  65.412  36.811  1.00 32.88           N
ATOM    8306  CA  VAL F   2       0.691  64.142  37.109  1.00 31.59           C
ATOM    8307  C   VAL F   2       1.212  63.563  38.430  1.00 30.02           C
ATOM    8308  O   VAL F   2       2.415  63.444  38.624  1.00 29.59           O
```

```
ATOM   8309   CB   VAL  F   2      0.882  63.127  35.985  1.00 31.51        C
ATOM   8310   CG1  VAL  F   2      0.315  61.776  36.387  1.00 32.09        C
ATOM   8311   CG2  VAL  F   2      0.210  63.624  34.714  1.00 32.29        C
ATOM   8312   N    LYS  F   3      0.298  63.235  39.334  1.00 29.65        N
ATOM   8313   CA   LYS  F   3      0.634  62.577  40.600  1.00 29.51        C
ATOM   8314   C    LYS  F   3     -0.255  61.357  40.764  1.00 27.85        C
ATOM   8315   O    LYS  F   3     -1.469  61.430  40.601  1.00 27.08        O
ATOM   8316   CB   LYS  F   3      0.417  63.486  41.802  1.00 30.25        C
ATOM   8317   CG   LYS  F   3      1.306  64.698  41.876  1.00 31.93        C
ATOM   8318   CD   LYS  F   3      1.469  65.141  43.339  1.00 32.75        C
ATOM   8319   CE   LYS  F   3      1.716  66.647  43.483  1.00 34.50        C
ATOM   8320   NZ   LYS  F   3      1.856  67.022  44.930  1.00 34.50        N
ATOM   8321   N    VAL  F   4      0.366  60.232  41.077  1.00 26.58        N
ATOM   8322   CA   VAL  F   4     -0.351  58.998  41.300  1.00 25.06        C
ATOM   8323   C    VAL  F   4      0.128  58.551  42.657  1.00 24.33        C
ATOM   8324   O    VAL  F   4      1.335  58.364  42.847  1.00 23.47        O
ATOM   8325   CB   VAL  F   4     -0.025  57.960  40.236  1.00 25.40        C
ATOM   8326   CG1  VAL  F   4     -0.824  56.694  40.475  1.00 25.15        C
ATOM   8327   CG2  VAL  F   4     -0.291  58.532  38.845  1.00 25.67        C
ATOM   8328   N    GLU  F   5     -0.807  58.422  43.595  1.00 23.30        N
ATOM   8329   CA   GLU  F   5     -0.452  58.227  44.992  1.00 25.92        C
ATOM   8330   C    GLU  F   5     -1.243  57.113  45.664  1.00 24.01        C
ATOM   8331   O    GLU  F   5     -2.441  57.255  45.936  1.00 23.20        O
ATOM   8332   CB   GLU  F   5     -0.645  59.542  45.746  1.00 27.11        C
ATOM   8333   CG   GLU  F   5      0.157  59.616  47.031  1.00 31.23        C
ATOM   8334   CD   GLU  F   5      0.087  61.003  47.677  1.00 32.37        C
ATOM   8335   OE1  GLU  F   5     -1.036  61.427  48.039  1.00 37.34        O
ATOM   8336   OE2  GLU  F   5      1.152  61.649  47.828  1.00 37.06        O
ATOM   8337   N    GLU  F   6     -0.552  56.017  45.970  1.00 22.78        N
ATOM   8338   CA   GLU  F   6     -1.203  54.827  46.527  1.00 22.30        C
ATOM   8339   C    GLU  F   6     -1.250  54.913  48.040  1.00 22.51        C
ATOM   8340   O    GLU  F   6     -0.447  55.619  48.652  1.00 24.13        O
ATOM   8341   CB   GLU  F   6     -0.446  53.555  46.129  1.00 22.06        C
ATOM   8342   CG   GLU  F   6     -0.383  53.313  44.619  1.00 21.89        C
ATOM   8343   CD   GLU  F   6      0.815  53.922  43.946  1.00 20.75        C
ATOM   8344   OE1  GLU  F   6      1.399  54.914  44.462  1.00 19.97        O
ATOM   8345   OE2  GLU  F   6      1.203  53.385  42.887  1.00 22.12        O
ATOM   8346   N    SER  F   7     -2.174  54.166  48.628  1.00 22.11        N
ATOM   8347   CA   SER  F   7     -2.267  54.025  50.080  1.00 21.13        C
ATOM   8348   C    SER  F   7     -2.964  52.725  50.409  1.00 20.51        C
ATOM   8349   O    SER  F   7     -3.549  52.089  49.530  1.00 18.05        O
ATOM   8350   CB   SER  F   7     -3.048  55.198  50.681  1.00 22.18        C
ATOM   8351   OG   SER  F   7     -4.360  55.236  50.139  1.00 21.26        O
ATOM   8352   N    GLY  F   8     -2.905  52.337  51.684  1.00 19.57        N
ATOM   8353   CA   GLY  F   8     -3.687  51.225  52.180  1.00 19.47        C
ATOM   8354   C    GLY  F   8     -2.880  49.971  52.472  1.00 18.70        C
ATOM   8355   O    GLY  F   8     -3.435  48.970  52.906  1.00 19.60        O
ATOM   8356   N    GLY  F   9     -1.575  50.025  52.244  1.00 20.27        N
ATOM   8357   CA   GLY  F   9     -0.714  48.871  52.492  1.00 20.59        C
ATOM   8358   C    GLY  F   9     -0.520  48.560  53.965  1.00 21.49        C
ATOM   8359   O    GLY  F   9     -1.002  49.290  54.862  1.00 22.51        O
ATOM   8360   N    GLY  F  10      0.168  47.449  54.218  1.00 20.02        N
ATOM   8361   CA   GLY  F  10      0.436  46.990  55.577  1.00 18.98        C
ATOM   8362   C    GLY  F  10      0.543  45.485  55.580  1.00 17.97        C
ATOM   8363   O    GLY  F  10      0.839  44.896  54.567  1.00 17.23        O
ATOM   8364   N    LEU  F  11      0.274  44.882  56.727  1.00 17.33        N
ATOM   8365   CA   LEU  F  11      0.375  43.454  56.914  1.00 18.13        C
ATOM   8366   C    LEU  F  11     -0.989  42.782  57.124  1.00 18.89        C
ATOM   8367   O    LEU  F  11     -1.850  43.300  57.857  1.00 18.44        O
ATOM   8368   CB   LEU  F  11      1.263  43.181  58.139  1.00 18.28        C
ATOM   8369   CG   LEU  F  11      1.571  41.684  58.342  1.00 19.93        C
ATOM   8370   CD1  LEU  F  11      3.032  41.444  58.673  1.00 21.69        C
ATOM   8371   CD2  LEU  F  11      0.713  41.086  59.399  1.00 20.92        C
ATOM   8372   N    VAL  F  12     -1.177  41.614  56.516  1.00 17.40        N
ATOM   8373   CA   VAL  F  12     -2.274  40.734  56.915  1.00 19.43        C
```

| ATOM | 8374 | C   | VAL | F | 12 | -1.818  | 39.279 | 56.852 | 1.00 | 20.20 | C |
| ATOM | 8375 | O   | VAL | F | 12 | -0.788  | 38.969 | 56.251 | 1.00 | 19.20 | O |
| ATOM | 8376 | CB  | VAL | F | 12 | -3.576  | 40.965 | 56.066 | 1.00 | 20.32 | C |
| ATOM | 8377 | CG1 | VAL | F | 12 | -3.408  | 40.478 | 54.621 | 1.00 | 20.19 | C |
| ATOM | 8378 | CG2 | VAL | F | 12 | -4.786  | 40.336 | 56.734 | 1.00 | 21.24 | C |
| ATOM | 8379 | N   | GLN | F | 13 | -2.578  | 38.411 | 57.503 | 1.00 | 20.46 | N |
| ATOM | 8380 | CA  | GLN | F | 13 | -2.249  | 37.000 | 57.605 | 1.00 | 21.71 | C |
| ATOM | 8381 | C   | GLN | F | 13 | -2.733  | 36.284 | 56.346 | 1.00 | 21.86 | C |
| ATOM | 8382 | O   | GLN | F | 13 | -3.689  | 36.738 | 55.702 | 1.00 | 21.81 | O |
| ATOM | 8383 | CB  | GLN | F | 13 | -2.930  | 36.391 | 58.836 | 1.00 | 22.74 | C |
| ATOM | 8384 | CG  | GLN | F | 13 | -2.518  | 37.001 | 60.175 | 1.00 | 24.92 | C |
| ATOM | 8385 | CD  | GLN | F | 13 | -3.134  | 38.379 | 60.477 | 1.00 | 25.48 | C |
| ATOM | 8386 | OE1 | GLN | F | 13 | -4.288  | 38.676 | 60.127 | 1.00 | 25.72 | O |
| ATOM | 8387 | NE2 | GLN | F | 13 | -2.359  | 39.220 | 61.160 | 1.00 | 26.90 | N |
| ATOM | 8388 | N   | PRO | F | 14 | -2.106  | 35.145 | 56.004 | 1.00 | 23.75 | N |
| ATOM | 8389 | CA  | PRO | F | 14 | -2.617  | 34.289 | 54.941 | 1.00 | 23.48 | C |
| ATOM | 8390 | C   | PRO | F | 14 | -4.092  | 33.947 | 55.138 | 1.00 | 23.38 | C |
| ATOM | 8391 | O   | PRO | F | 14 | -4.466  | 33.496 | 56.225 | 1.00 | 23.12 | O |
| ATOM | 8392 | CB  | PRO | F | 14 | -1.752  | 33.022 | 55.063 | 1.00 | 24.28 | C |
| ATOM | 8393 | CG  | PRO | F | 14 | -0.493  | 33.486 | 55.642 | 1.00 | 23.89 | C |
| ATOM | 8394 | CD  | PRO | F | 14 | -0.865  | 34.608 | 56.588 | 1.00 | 23.79 | C |
| ATOM | 8395 | N   | GLY | F | 15 | -4.909  | 34.155 | 54.096 | 1.00 | 23.06 | N |
| ATOM | 8396 | CA  | GLY | F | 15 | -6.359  | 33.958 | 54.160 | 1.00 | 22.30 | C |
| ATOM | 8397 | C   | GLY | F | 15 | -7.129  | 35.262 | 54.324 | 1.00 | 22.27 | C |
| ATOM | 8398 | O   | GLY | F | 15 | -8.349  | 35.316 | 54.125 | 1.00 | 23.66 | O |
| ATOM | 8399 | N   | GLY | F | 16 | -6.414  | 36.322 | 54.679 | 1.00 | 21.79 | N |
| ATOM | 8400 | CA  | GLY | F | 16 | -7.021  | 37.584 | 54.997 | 1.00 | 21.65 | C |
| ATOM | 8401 | C   | GLY | F | 16 | -7.186  | 38.463 | 53.793 | 1.00 | 21.78 | C |
| ATOM | 8402 | O   | GLY | F | 16 | -6.859  | 38.075 | 52.681 | 1.00 | 21.54 | O |
| ATOM | 8403 | N   | SER | F | 17 | -7.685  | 39.664 | 54.029 | 1.00 | 21.52 | N |
| ATOM | 8404 | CA  | SER | F | 17 | -7.996  | 40.596 | 52.965 | 1.00 | 22.35 | C |
| ATOM | 8405 | C   | SER | F | 17 | -7.325  | 41.905 | 53.196 | 1.00 | 22.10 | C |
| ATOM | 8406 | O   | SER | F | 17 | -6.952  | 42.223 | 54.321 | 1.00 | 21.96 | O |
| ATOM | 8407 | CB  | SER | F | 17 | -9.500  | 40.848 | 52.892 | 1.00 | 23.94 | C |
| ATOM | 8408 | OG  | SER | F | 17 | -10.150 | 39.677 | 52.481 | 1.00 | 26.93 | O |
| ATOM | 8409 | N   | MET | F | 18 | -7.197  | 42.673 | 52.117 | 1.00 | 22.12 | N |
| ATOM | 8410 | CA  | MET | F | 18 | -6.695  | 44.031 | 52.170 | 1.00 | 22.90 | C |
| ATOM | 8411 | C   | MET | F | 18 | -7.408  | 44.877 | 51.111 | 1.00 | 22.17 | C |
| ATOM | 8412 | O   | MET | F | 18 | -7.818  | 44.362 | 50.108 | 1.00 | 20.78 | O |
| ATOM | 8413 | CB  | MET | F | 18 | -5.207  | 44.020 | 51.859 | 1.00 | 24.87 | C |
| ATOM | 8414 | CG  | MET | F | 18 | -4.418  | 45.038 | 52.575 | 1.00 | 29.20 | C |
| ATOM | 8415 | SD  | MET | F | 18 | -3.743  | 44.407 | 54.144 | 1.00 | 32.97 | S |
| ATOM | 8416 | CE  | MET | F | 18 | -2.411  | 45.600 | 54.215 | 1.00 | 31.12 | C |
| ATOM | 8417 | N   | LYS | F | 19 | -7.515  | 46.174 | 51.336 | 1.00 | 21.86 | N |
| ATOM | 8418 | CA  | LYS | F | 19 | -8.017  | 47.071 | 50.307 | 1.00 | 22.56 | C |
| ATOM | 8419 | C   | LYS | F | 19 | -7.065  | 48.249 | 50.170 | 1.00 | 19.93 | C |
| ATOM | 8420 | O   | LYS | F | 19 | -6.794  | 48.973 | 51.128 | 1.00 | 19.91 | O |
| ATOM | 8421 | CB  | LYS | F | 19 | -9.461  | 47.538 | 50.571 | 1.00 | 23.85 | C |
| ATOM | 8422 | CG  | LYS | F | 19 | -10.086 | 48.255 | 49.340 | 1.00 | 24.37 | C |
| ATOM | 8423 | CD  | LYS | F | 19 | -11.571 | 48.528 | 49.476 | 1.00 | 25.78 | C |
| ATOM | 8424 | CE  | LYS | F | 19 | -12.195 | 48.763 | 48.092 | 1.00 | 28.23 | C |
| ATOM | 8425 | NZ  | LYS | F | 19 | -13.584 | 49.347 | 48.176 | 1.00 | 29.66 | N |
| ATOM | 8426 | N   | ILE | F | 20 | -6.543  | 48.408 | 48.962 | 1.00 | 17.52 | N |
| ATOM | 8427 | CA  | ILE | F | 20 | -5.615  | 49.469 | 48.648 | 1.00 | 17.42 | C |
| ATOM | 8428 | C   | ILE | F | 20 | -6.247  | 50.346 | 47.590 | 1.00 | 16.82 | C |
| ATOM | 8429 | O   | ILE | F | 20 | -7.166  | 49.934 | 46.899 | 1.00 | 16.45 | O |
| ATOM | 8430 | CB  | ILE | F | 20 | -4.233  | 48.916 | 48.159 | 1.00 | 16.82 | C |
| ATOM | 8431 | CG1 | ILE | F | 20 | -4.399  | 47.997 | 46.953 | 1.00 | 17.51 | C |
| ATOM | 8432 | CG2 | ILE | F | 20 | -3.510  | 48.154 | 49.307 | 1.00 | 17.56 | C |
| ATOM | 8433 | CD1 | ILE | F | 20 | -3.079  | 47.593 | 46.303 | 1.00 | 17.24 | C |
| ATOM | 8434 | N   | SER | F | 21 | -5.758  | 51.564 | 47.488 | 1.00 | 18.37 | N |
| ATOM | 8435 | CA  | SER | F | 21 | -6.304  | 52.488 | 46.535 | 1.00 | 19.50 | C |
| ATOM | 8436 | C   | SER | F | 21 | -5.226  | 53.444 | 46.096 | 1.00 | 20.03 | C |
| ATOM | 8437 | O   | SER | F | 21 | -4.155  | 53.529 | 46.709 | 1.00 | 18.97 | O |
| ATOM | 8438 | CB  | SER | F | 21 | -7.486  | 53.247 | 47.149 | 1.00 | 21.02 | C |

```
ATOM   8439  OG   SER F  21    -7.097  53.960  48.302  1.00  22.87        O
ATOM   8440  N    CYS F  22    -5.490  54.157  45.015  1.00  21.51        N
ATOM   8441  CA   CYS F  22    -4.663  55.311  44.683  1.00  22.49        C
ATOM   8442  C    CYS F  22    -5.483  56.453  44.145  1.00  22.57        C
ATOM   8443  O    CYS F  22    -6.518  56.246  43.536  1.00  22.29        O
ATOM   8444  CB   CYS F  22    -3.582  54.959  43.665  1.00  24.67        C
ATOM   8445  SG   CYS F  22    -4.184  54.414  42.121  1.00  28.98        S
ATOM   8446  N    VAL F  23    -4.989  57.661  44.369  1.00  23.07        N
ATOM   8447  CA   VAL F  23    -5.625  58.847  43.833  1.00  24.02        C
ATOM   8448  C    VAL F  23    -4.686  59.472  42.795  1.00  23.32        C
ATOM   8449  O    VAL F  23    -3.465  59.582  43.011  1.00  22.61        O
ATOM   8450  CB   VAL F  23    -6.011  59.847  44.952  1.00  24.79        C
ATOM   8451  CG1  VAL F  23    -4.788  60.287  45.748  1.00  26.30        C
ATOM   8452  CG2  VAL F  23    -6.763  61.054  44.351  1.00  26.38        C
ATOM   8453  N    VAL F  24    -5.264  59.848  41.661  1.00  22.79        N
ATOM   8454  CA   VAL F  24    -4.513  60.431  40.564  1.00  23.23        C
ATOM   8455  C    VAL F  24    -4.894  61.902  40.441  1.00  24.74        C
ATOM   8456  O    VAL F  24    -6.070  62.221  40.401  1.00  25.53        O
ATOM   8457  CB   VAL F  24    -4.852  59.737  39.239  1.00  23.17        C
ATOM   8458  CG1  VAL F  24    -4.032  60.301  38.117  1.00  23.44        C
ATOM   8459  CG2  VAL F  24    -4.621  58.262  39.343  1.00  23.25        C
ATOM   8460  N    SER F  25    -3.913  62.788  40.379  1.00  26.61        N
ATOM   8461  CA   SER F  25    -4.192  64.192  40.091  1.00  27.52        C
ATOM   8462  C    SER F  25    -3.368  64.680  38.902  1.00  27.70        C
ATOM   8463  O    SER F  25    -2.367  64.070  38.534  1.00  26.19        O
ATOM   8464  CB   SER F  25    -3.943  65.048  41.329  1.00  28.00        C
ATOM   8465  OG   SER F  25    -2.575  65.054  41.705  1.00  28.44        O
ATOM   8466  N    GLY F  26    -3.803  65.788  38.298  1.00  27.75        N
ATOM   8467  CA   GLY F  26    -3.074  66.402  37.194  1.00  27.66        C
ATOM   8468  C    GLY F  26    -3.538  65.978  35.817  1.00  28.74        C
ATOM   8469  O    GLY F  26    -3.041  66.493  34.811  1.00  29.85        O
ATOM   8470  N    LEU F  27    -4.495  65.057  35.764  1.00  29.77        N
ATOM   8471  CA   LEU F  27    -5.100  64.654  34.494  1.00  30.62        C
ATOM   8472  C    LEU F  27    -6.571  64.276  34.684  1.00  30.63        C
ATOM   8473  O    LEU F  27    -7.046  64.092  35.812  1.00  31.32        O
ATOM   8474  CB   LEU F  27    -4.280  63.525  33.840  1.00  31.53        C
ATOM   8475  CG   LEU F  27    -3.886  62.327  34.699  1.00  31.42        C
ATOM   8476  CD1  LEU F  27    -5.139  61.596  35.166  1.00  33.27        C
ATOM   8477  CD2  LEU F  27    -2.974  61.365  33.954  1.00  31.81        C
ATOM   8478  N    THR F  28    -7.288  64.169  33.571  1.00  29.97        N
ATOM   8479  CA   THR F  28    -8.694  63.783  33.566  1.00  29.01        C
ATOM   8480  C    THR F  28    -8.843  62.274  33.782  1.00  28.32        C
ATOM   8481  O    THR F  28    -8.885  61.496  32.829  1.00  27.25        O
ATOM   8482  CB   THR F  28    -9.378  64.245  32.247  1.00  29.39        C
ATOM   8483  OG1  THR F  28    -9.144  65.652  32.047  1.00  30.67        O
ATOM   8484  CG2  THR F  28   -10.877  63.984  32.280  1.00  29.92        C
ATOM   8485  N    PHE F  29    -8.920  61.886  35.057  1.00  28.28        N
ATOM   8486  CA   PHE F  29    -8.942  60.474  35.498  1.00  27.58        C
ATOM   8487  C    PHE F  29    -9.906  59.571  34.719  1.00  27.52        C
ATOM   8488  O    PHE F  29    -9.552  58.443  34.339  1.00  24.75        O
ATOM   8489  CB   PHE F  29    -9.292  60.440  36.996  1.00  27.95        C
ATOM   8490  CG   PHE F  29    -9.341  59.072  37.599  1.00  27.30        C
ATOM   8491  CD1  PHE F  29    -8.225  58.536  38.213  1.00  27.38        C
ATOM   8492  CD2  PHE F  29   -10.515  58.343  37.606  1.00  27.04        C
ATOM   8493  CE1  PHE F  29    -8.271  57.279  38.799  1.00  27.23        C
ATOM   8494  CE2  PHE F  29   -10.565  57.093  38.181  1.00  27.79        C
ATOM   8495  CZ   PHE F  29    -9.431  56.561  38.780  1.00  27.46        C
ATOM   8496  N    SER F  30   -11.126  60.054  34.483  1.00  26.22        N
ATOM   8497  CA   SER F  30   -12.142  59.242  33.816  1.00  26.16        C
ATOM   8498  C    SER F  30   -11.800  58.855  32.365  1.00  24.91        C
ATOM   8499  O    SER F  30   -12.444  57.950  31.801  1.00  24.83        O
ATOM   8500  CB   SER F  30   -13.505  59.951  33.850  1.00  27.17        C
ATOM   8501  OG   SER F  30   -13.357  61.275  33.387  1.00  30.87        O
ATOM   8502  N    ASN F  31   -10.809  59.521  31.768  1.00  25.14        N
ATOM   8503  CA   ASN F  31   -10.364  59.202  30.394  1.00  24.80        C
```

| ATOM | 8504 | C | ASN | F | 31 | -9.309 | 58.075 | 30.289 | 1.00 | 25.52 | C |
|------|------|------|-----|---|----|--------|--------|--------|------|-------|---|
| ATOM | 8505 | O | ASN | F | 31 | -9.009 | 57.618 | 29.172 | 1.00 | 24.90 | O |
| ATOM | 8506 | CB | ASN | F | 31 | -9.775 | 60.440 | 29.711 | 1.00 | 26.16 | C |
| ATOM | 8507 | CG | ASN | F | 31 | -10.820 | 61.512 | 29.379 | 1.00 | 28.93 | C |
| ATOM | 8508 | OD1 | ASN | F | 31 | -10.456 | 62.651 | 29.065 | 1.00 | 33.16 | O |
| ATOM | 8509 | ND2 | ASN | F | 31 | -12.097 | 61.160 | 29.439 | 1.00 | 27.56 | N |
| ATOM | 8510 | N | TYR | F | 32 | -8.755 | 57.634 | 31.424 | 1.00 | 24.02 | N |
| ATOM | 8511 | CA | TYR | F | 32 | -7.579 | 56.753 | 31.406 | 1.00 | 22.93 | C |
| ATOM | 8512 | C | TYR | F | 32 | -7.894 | 55.366 | 31.885 | 1.00 | 21.34 | C |
| ATOM | 8513 | O | TYR | F | 32 | -8.604 | 55.193 | 32.874 | 1.00 | 19.82 | O |
| ATOM | 8514 | CB | TYR | F | 32 | -6.459 | 57.332 | 32.274 | 1.00 | 23.56 | C |
| ATOM | 8515 | CG | TYR | F | 32 | -5.837 | 58.528 | 31.669 | 1.00 | 24.61 | C |
| ATOM | 8516 | CD1 | TYR | F | 32 | -4.741 | 58.414 | 30.822 | 1.00 | 24.29 | C |
| ATOM | 8517 | CD2 | TYR | F | 32 | -6.373 | 59.795 | 31.893 | 1.00 | 24.11 | C |
| ATOM | 8518 | CE1 | TYR | F | 32 | -4.172 | 59.525 | 30.238 | 1.00 | 25.44 | C |
| ATOM | 8519 | CE2 | TYR | F | 32 | -5.812 | 60.914 | 31.306 | 1.00 | 25.81 | C |
| ATOM | 8520 | CZ | TYR | F | 32 | -4.714 | 60.771 | 30.472 | 1.00 | 25.69 | C |
| ATOM | 8521 | OH | TYR | F | 32 | -4.138 | 61.882 | 29.887 | 1.00 | 26.83 | O |
| ATOM | 8522 | N | TRP | F | 33 | -7.347 | 54.368 | 31.193 | 1.00 | 19.82 | N |
| ATOM | 8523 | CA | TRP | F | 33 | -7.360 | 53.009 | 31.696 | 1.00 | 19.62 | C |
| ATOM | 8524 | C | TRP | F | 33 | -6.470 | 52.937 | 32.939 | 1.00 | 17.24 | C |
| ATOM | 8525 | O | TRP | F | 33 | -5.494 | 53.659 | 33.055 | 1.00 | 18.18 | O |
| ATOM | 8526 | CB | TRP | F | 33 | -6.849 | 52.029 | 30.659 | 1.00 | 19.56 | C |
| ATOM | 8527 | CG | TRP | F | 33 | -7.606 | 52.005 | 29.392 | 1.00 | 19.79 | C |
| ATOM | 8528 | CD1 | TRP | F | 33 | -8.805 | 52.612 | 29.123 | 1.00 | 21.36 | C |
| ATOM | 8529 | CD2 | TRP | F | 33 | -7.223 | 51.321 | 28.207 | 1.00 | 20.27 | C |
| ATOM | 8530 | NE1 | TRP | F | 33 | -9.188 | 52.346 | 27.831 | 1.00 | 21.54 | N |
| ATOM | 8531 | CE2 | TRP | F | 33 | -8.226 | 51.561 | 27.240 | 1.00 | 21.53 | C |
| ATOM | 8532 | CE3 | TRP | F | 33 | -6.126 | 50.519 | 27.862 | 1.00 | 21.25 | C |
| ATOM | 8533 | CZ2 | TRP | F | 33 | -8.165 | 51.020 | 25.944 | 1.00 | 21.73 | C |
| ATOM | 8534 | CZ3 | TRP | F | 33 | -6.068 | 49.977 | 26.579 | 1.00 | 20.81 | C |
| ATOM | 8535 | CH2 | TRP | F | 33 | -7.083 | 50.229 | 25.639 | 1.00 | 20.81 | C |
| ATOM | 8536 | N | MET | F | 34 | -6.836 | 52.062 | 33.862 | 1.00 | 17.91 | N |
| ATOM | 8537 | CA | MET | F | 34 | -6.183 | 51.971 | 35.163 | 1.00 | 17.71 | C |
| ATOM | 8538 | C | MET | F | 34 | -5.822 | 50.523 | 35.416 | 1.00 | 16.93 | C |
| ATOM | 8539 | O | MET | F | 34 | -6.615 | 49.646 | 35.165 | 1.00 | 16.95 | O |
| ATOM | 8540 | CB | MET | F | 34 | -7.139 | 52.372 | 36.276 | 1.00 | 20.95 | C |
| ATOM | 8541 | CG | MET | F | 34 | -7.560 | 53.816 | 36.250 | 1.00 | 22.12 | C |
| ATOM | 8542 | SD | MET | F | 34 | -6.317 | 54.940 | 36.824 | 1.00 | 27.21 | S |
| ATOM | 8543 | CE | MET | F | 34 | -6.889 | 56.375 | 35.912 | 1.00 | 26.14 | C |
| ATOM | 8544 | N | SER | F | 35 | -4.648 | 50.299 | 35.991 | 1.00 | 16.64 | N |
| ATOM | 8545 | CA | SER | F | 35 | -4.207 | 48.952 | 36.290 | 1.00 | 15.79 | C |
| ATOM | 8546 | C | SER | F | 35 | -3.377 | 48.938 | 37.562 | 1.00 | 15.83 | C |
| ATOM | 8547 | O | SER | F | 35 | -2.908 | 49.983 | 38.032 | 1.00 | 14.99 | O |
| ATOM | 8548 | CB | SER | F | 35 | -3.402 | 48.389 | 35.111 | 1.00 | 16.00 | C |
| ATOM | 8549 | OG | SER | F | 35 | -2.047 | 48.810 | 35.128 | 1.00 | 15.59 | O |
| ATOM | 8550 | N | TRP | F | 36 | -3.225 | 47.738 | 38.112 | 1.00 | 14.79 | N |
| ATOM | 8551 | CA | TRP | F | 36 | -2.326 | 47.496 | 39.226 | 1.00 | 14.90 | C |
| ATOM | 8552 | C | TRP | F | 36 | -1.257 | 46.544 | 38.742 | 1.00 | 13.93 | C |
| ATOM | 8553 | O | TRP | F | 36 | -1.564 | 45.560 | 38.121 | 1.00 | 12.72 | O |
| ATOM | 8554 | CB | TRP | F | 36 | -3.058 | 46.840 | 40.382 | 1.00 | 16.39 | C |
| ATOM | 8555 | CG | TRP | F | 36 | -4.016 | 47.774 | 41.090 | 1.00 | 16.68 | C |
| ATOM | 8556 | CD1 | TRP | F | 36 | -5.341 | 47.903 | 40.869 | 1.00 | 17.69 | C |
| ATOM | 8557 | CD2 | TRP | F | 36 | -3.685 | 48.679 | 42.141 | 1.00 | 16.06 | C |
| ATOM | 8558 | NE1 | TRP | F | 36 | -5.873 | 48.840 | 41.719 | 1.00 | 16.90 | N |
| ATOM | 8559 | CE2 | TRP | F | 36 | -4.872 | 49.340 | 42.509 | 1.00 | 17.46 | C |
| ATOM | 8560 | CE3 | TRP | F | 36 | -2.493 | 49.003 | 42.796 | 1.00 | 16.64 | C |
| ATOM | 8561 | CZ2 | TRP | F | 36 | -4.913 | 50.317 | 43.501 | 1.00 | 17.09 | C |
| ATOM | 8562 | CZ3 | TRP | F | 36 | -2.531 | 49.980 | 43.804 | 1.00 | 17.48 | C |
| ATOM | 8563 | CH2 | TRP | F | 36 | -3.743 | 50.621 | 44.144 | 1.00 | 18.03 | C |
| ATOM | 8564 | N | VAL | F | 37 | -0.017 | 46.860 | 39.083 | 1.00 | 13.53 | N |
| ATOM | 8565 | CA | VAL | F | 37 | 1.149 | 46.071 | 38.756 | 1.00 | 13.82 | C |
| ATOM | 8566 | C | VAL | F | 37 | 1.908 | 45.967 | 40.071 | 1.00 | 13.62 | C |
| ATOM | 8567 | O | VAL | F | 37 | 2.136 | 46.980 | 40.747 | 1.00 | 15.65 | O |
| ATOM | 8568 | CB | VAL | F | 37 | 2.029 | 46.747 | 37.686 | 1.00 | 12.80 | C |

| ATOM | 8569 | CG1 | VAL | F | 37 | 3.366 | 45.965 | 37.511 | 1.00 | 14.94 | C |
|------|------|-----|-----|---|-----|--------|--------|--------|------|-------|---|
| ATOM | 8570 | CG2 | VAL | F | 37 | 1.305 | 46.897 | 36.387 | 1.00 | 12.95 | C |
| ATOM | 8571 | N | ARG | F | 38 | 2.243 | 44.744 | 40.451 | 1.00 | 13.30 | N |
| ATOM | 8572 | CA | ARG | F | 38 | 2.999 | 44.522 | 41.688 | 1.00 | 14.33 | C |
| ATOM | 8573 | C | ARG | F | 38 | 4.431 | 44.155 | 41.362 | 1.00 | 14.93 | C |
| ATOM | 8574 | O | ARG | F | 38 | 4.709 | 43.702 | 40.271 | 1.00 | 15.19 | O |
| ATOM | 8575 | CB | ARG | F | 38 | 2.319 | 43.500 | 42.599 | 1.00 | 14.50 | C |
| ATOM | 8576 | CG | ARG | F | 38 | 2.247 | 42.077 | 42.054 | 1.00 | 14.03 | C |
| ATOM | 8577 | CD | ARG | F | 38 | 1.292 | 41.212 | 42.859 | 1.00 | 16.24 | C |
| ATOM | 8578 | NE | ARG | F | 38 | 1.371 | 39.816 | 42.421 | 1.00 | 16.46 | N |
| ATOM | 8579 | CZ | ARG | F | 38 | 0.573 | 38.839 | 42.839 | 1.00 | 18.43 | C |
| ATOM | 8580 | NH1 | ARG | F | 38 | -0.342 | 39.046 | 43.782 | 1.00 | 17.73 | N |
| ATOM | 8581 | NH2 | ARG | F | 38 | 0.739 | 37.614 | 42.354 | 1.00 | 19.78 | N |
| ATOM | 8582 | N | GLN | F | 39 | 5.348 | 44.449 | 42.273 | 1.00 | 15.52 | N |
| ATOM | 8583 | CA | GLN | F | 39 | 6.748 | 44.083 | 42.096 | 1.00 | 15.51 | C |
| ATOM | 8584 | C | GLN | F | 39 | 7.252 | 43.303 | 43.308 | 1.00 | 17.61 | C |
| ATOM | 8585 | O | GLN | F | 39 | 7.025 | 43.706 | 44.467 | 1.00 | 16.18 | O |
| ATOM | 8586 | CB | GLN | F | 39 | 7.592 | 45.335 | 41.896 | 1.00 | 16.32 | C |
| ATOM | 8587 | CG | GLN | F | 39 | 9.024 | 45.061 | 41.494 | 1.00 | 16.58 | C |
| ATOM | 8588 | CD | GLN | F | 39 | 9.723 | 46.309 | 41.075 | 1.00 | 18.60 | C |
| ATOM | 8589 | OE1 | GLN | F | 39 | 9.524 | 47.370 | 41.680 | 1.00 | 19.73 | O |
| ATOM | 8590 | NE2 | GLN | F | 39 | 10.561 | 46.209 | 40.042 | 1.00 | 18.94 | N |
| ATOM | 8591 | N | SER | F | 40 | 7.912 | 42.177 | 43.031 | 1.00 | 19.53 | N |
| ATOM | 8592 | CA | SER | F | 40 | 8.576 | 41.351 | 44.059 | 1.00 | 21.47 | C |
| ATOM | 8593 | C | SER | F | 40 | 9.942 | 40.913 | 43.550 | 1.00 | 24.04 | C |
| ATOM | 8594 | O | SER | F | 40 | 10.168 | 40.847 | 42.348 | 1.00 | 24.14 | O |
| ATOM | 8595 | CB | SER | F | 40 | 7.743 | 40.108 | 44.375 | 1.00 | 21.67 | C |
| ATOM | 8596 | OG | SER | F | 40 | 7.661 | 39.254 | 43.238 | 1.00 | 23.20 | O |
| ATOM | 8597 | N | PRO | F | 41 | 10.892 | 40.669 | 44.466 | 1.00 | 27.13 | N |
| ATOM | 8598 | CA | PRO | F | 41 | 12.165 | 40.084 | 44.037 | 1.00 | 29.53 | C |
| ATOM | 8599 | C | PRO | F | 41 | 12.018 | 38.772 | 43.241 | 1.00 | 29.43 | C |
| ATOM | 8600 | O | PRO | F | 41 | 12.721 | 38.579 | 42.263 | 1.00 | 31.38 | O |
| ATOM | 8601 | CB | PRO | F | 41 | 12.899 | 39.854 | 45.370 | 1.00 | 29.51 | C |
| ATOM | 8602 | CG | PRO | F | 41 | 12.335 | 40.911 | 46.277 | 1.00 | 28.39 | C |
| ATOM | 8603 | CD | PRO | F | 41 | 10.882 | 40.985 | 45.905 | 1.00 | 27.56 | C |
| ATOM | 8604 | N | GLU | F | 42 | 11.088 | 37.910 | 43.629 | 1.00 | 31.91 | N |
| ATOM | 8605 | CA | GLU | F | 42 | 10.919 | 36.599 | 42.994 | 1.00 | 31.30 | C |
| ATOM | 8606 | C | GLU | F | 42 | 10.348 | 36.660 | 41.581 | 1.00 | 30.93 | C |
| ATOM | 8607 | O | GLU | F | 42 | 10.787 | 35.915 | 40.708 | 1.00 | 28.89 | O |
| ATOM | 8608 | CB | GLU | F | 42 | 9.993 | 35.719 | 43.840 | 1.00 | 33.62 | C |
| ATOM | 8609 | CG | GLU | F | 42 | 9.891 | 34.245 | 43.409 | 1.00 | 36.22 | C |
| ATOM | 8610 | CD | GLU | F | 42 | 11.141 | 33.415 | 43.746 | 1.00 | 39.66 | C |
| ATOM | 8611 | OE1 | GLU | F | 42 | 11.785 | 33.674 | 44.790 | 1.00 | 41.67 | O |
| ATOM | 8612 | OE2 | GLU | F | 42 | 11.473 | 32.498 | 42.959 | 1.00 | 43.01 | O |
| ATOM | 8613 | N | LYS | F | 43 | 9.352 | 37.523 | 41.365 | 1.00 | 28.87 | N |
| ATOM | 8614 | CA | LYS | F | 43 | 8.626 | 37.536 | 40.098 | 1.00 | 27.24 | C |
| ATOM | 8615 | C | LYS | F | 43 | 8.794 | 38.824 | 39.296 | 1.00 | 24.86 | C |
| ATOM | 8616 | O | LYS | F | 43 | 8.245 | 38.935 | 38.205 | 1.00 | 25.94 | O |
| ATOM | 8617 | CB | LYS | F | 43 | 7.136 | 37.267 | 40.340 | 1.00 | 29.57 | C |
| ATOM | 8618 | CG | LYS | F | 43 | 6.843 | 35.975 | 41.098 | 1.00 | 32.06 | C |
| ATOM | 8619 | CD | LYS | F | 43 | 7.417 | 34.750 | 40.380 | 1.00 | 34.36 | C |
| ATOM | 8620 | CE | LYS | F | 43 | 6.765 | 33.433 | 40.860 | 1.00 | 35.65 | C |
| ATOM | 8621 | NZ | LYS | F | 43 | 7.668 | 32.243 | 40.712 | 1.00 | 36.56 | N |
| ATOM | 8622 | N | GLY | F | 44 | 9.526 | 39.802 | 39.815 | 1.00 | 22.34 | N |
| ATOM | 8623 | CA | GLY | F | 44 | 9.717 | 41.076 | 39.078 | 1.00 | 21.47 | C |
| ATOM | 8624 | C | GLY | F | 44 | 8.403 | 41.816 | 38.954 | 1.00 | 18.88 | C |
| ATOM | 8625 | O | GLY | F | 44 | 7.556 | 41.704 | 39.850 | 1.00 | 18.30 | O |
| ATOM | 8626 | N | LEU | F | 45 | 8.226 | 42.546 | 37.847 | 1.00 | 16.54 | N |
| ATOM | 8627 | CA | LEU | F | 45 | 6.983 | 43.301 | 37.593 | 1.00 | 16.51 | C |
| ATOM | 8628 | C | LEU | F | 45 | 5.866 | 42.398 | 37.071 | 1.00 | 16.89 | C |
| ATOM | 8629 | O | LEU | F | 45 | 6.049 | 41.656 | 36.085 | 1.00 | 16.53 | O |
| ATOM | 8630 | CB | LEU | F | 45 | 7.209 | 44.460 | 36.624 | 1.00 | 16.45 | C |
| ATOM | 8631 | CG | LEU | F | 45 | 8.137 | 45.559 | 37.128 | 1.00 | 16.98 | C |
| ATOM | 8632 | CD1 | LEU | F | 45 | 8.578 | 46.463 | 35.993 | 1.00 | 18.17 | C |
| ATOM | 8633 | CD2 | LEU | F | 45 | 7.450 | 46.397 | 38.208 | 1.00 | 17.49 | C |

```
ATOM   8634  N    GLU F  46      4.719  42.458  37.747  1.00 15.81          N
ATOM   8635  CA   GLU F  46      3.609  41.530  37.495  1.00 18.10          C
ATOM   8636  C    GLU F  46      2.292  42.306  37.431  1.00 16.46          C
ATOM   8637  O    GLU F  46      1.758  42.748  38.454  1.00 13.67          O
ATOM   8638  CB   GLU F  46      3.575  40.485  38.605  1.00 19.33          C
ATOM   8639  CG   GLU F  46      2.721  39.292  38.365  1.00 22.43          C
ATOM   8640  CD   GLU F  46      2.688  38.345  39.577  1.00 23.66          C
ATOM   8641  OE1  GLU F  46      3.410  38.574  40.595  1.00 24.93          O
ATOM   8642  OE2  GLU F  46      1.938  37.349  39.490  1.00 30.93          O
ATOM   8643  N    TRP F  47      1.802  42.507  36.217  1.00 15.74          N
ATOM   8644  CA   TRP F  47      0.471  43.060  35.991  1.00 15.43          C
ATOM   8645  C    TRP F  47     -0.584  42.161  36.622  1.00 15.56          C
ATOM   8646  O    TRP F  47     -0.594  40.954  36.375  1.00 16.15          O
ATOM   8647  CB   TRP F  47      0.237  43.190  34.496  1.00 15.27          C
ATOM   8648  CG   TRP F  47     -1.127  43.651  34.083  1.00 16.63          C
ATOM   8649  CD1  TRP F  47     -1.534  44.948  33.964  1.00 14.09          C
ATOM   8650  CD2  TRP F  47     -2.245  42.839  33.696  1.00 14.31          C
ATOM   8651  NE1  TRP F  47     -2.836  44.995  33.535  1.00 14.68          N
ATOM   8652  CE2  TRP F  47     -3.297  43.721  33.353  1.00 15.15          C
ATOM   8653  CE3  TRP F  47     -2.468  41.453  33.602  1.00 15.22          C
ATOM   8654  CZ2  TRP F  47     -4.539  43.267  32.910  1.00 15.04          C
ATOM   8655  CZ3  TRP F  47     -3.708  41.011  33.177  1.00 15.88          C
ATOM   8656  CH2  TRP F  47     -4.730  41.915  32.841  1.00 16.05          C
ATOM   8657  N    VAL F  48     -1.491  42.721  37.412  1.00 15.20          N
ATOM   8658  CA   VAL F  48     -2.501  41.877  38.040  1.00 15.84          C
ATOM   8659  C    VAL F  48     -3.963  42.174  37.698  1.00 15.97          C
ATOM   8660  O    VAL F  48     -4.751  41.247  37.663  1.00 17.56          O
ATOM   8661  CB   VAL F  48     -2.338  41.757  39.582  1.00 17.11          C
ATOM   8662  CG1  VAL F  48     -0.983  41.122  39.917  1.00 18.63          C
ATOM   8663  CG2  VAL F  48     -2.549  43.092  40.274  1.00 17.02          C
ATOM   8664  N    ALA F  49     -4.314  43.426  37.413  1.00 15.13          N
ATOM   8665  CA   ALA F  49     -5.698  43.755  37.133  1.00 15.88          C
ATOM   8666  C    ALA F  49     -5.823  45.095  36.460  1.00 15.51          C
ATOM   8667  O    ALA F  49     -4.981  45.966  36.631  1.00 15.04          O
ATOM   8668  CB   ALA F  49     -6.537  43.741  38.451  1.00 16.25          C
ATOM   8669  N    GLU F  50     -6.898  45.243  35.695  1.00 15.02          N
ATOM   8670  CA   GLU F  50     -7.067  46.408  34.862  1.00 14.93          C
ATOM   8671  C    GLU F  50     -8.552  46.756  34.727  1.00 15.64          C
ATOM   8672  O    GLU F  50     -9.377  45.875  34.679  1.00 14.80          O
ATOM   8673  CB   GLU F  50     -6.483  46.148  33.487  1.00 14.79          C
ATOM   8674  CG   GLU F  50     -6.530  47.368  32.598  1.00 14.36          C
ATOM   8675  CD   GLU F  50     -5.474  47.357  31.522  1.00 14.87          C
ATOM   8676  OE1  GLU F  50     -4.358  46.848  31.772  1.00 17.22          O
ATOM   8677  OE2  GLU F  50     -5.754  47.863  30.421  1.00 16.68          O
ATOM   8678  N    ILE F  51     -8.866  48.051  34.702  1.00 16.79          N
ATOM   8679  CA   ILE F  51    -10.247  48.478  34.521  1.00 18.70          C
ATOM   8680  C    ILE F  51    -10.296  49.528  33.432  1.00 19.60          C
ATOM   8681  O    ILE F  51     -9.407  50.405  33.330  1.00 20.69          O
ATOM   8682  CB   ILE F  51    -10.907  49.002  35.830  1.00 18.40          C
ATOM   8683  CG1  ILE F  51    -12.413  49.238  35.596  1.00 17.74          C
ATOM   8684  CG2  ILE F  51    -10.222  50.268  36.325  1.00 19.94          C
ATOM   8685  CD1  ILE F  51    -13.227  49.290  36.855  1.00 18.58          C
ATOM   8686  N    ARG F  52    -11.335  49.431  32.616  1.00 21.07          N
ATOM   8687  CA   ARG F  52    -11.477  50.310  31.451  1.00 22.45          C
ATOM   8688  C    ARG F  52    -12.409  51.493  31.792  1.00 22.87          C
ATOM   8689  O    ARG F  52    -12.625  51.777  32.977  1.00 23.00          O
ATOM   8690  CB   ARG F  52    -11.942  49.469  30.245  1.00 21.88          C
ATOM   8691  CG   ARG F  52    -10.901  48.423  29.781  1.00 23.09          C
ATOM   8692  CD   ARG F  52     -9.616  49.118  29.331  1.00 23.31          C
ATOM   8693  NE   ARG F  52     -8.487  48.202  29.196  1.00 23.68          N
ATOM   8694  CZ   ARG F  52     -8.217  47.489  28.108  1.00 23.53          C
ATOM   8695  NH1  ARG F  52     -9.000  47.553  27.034  1.00 23.56          N
ATOM   8696  NH2  ARG F  52     -7.159  46.697  28.096  1.00 23.46          N
ATOM   8697  N    LEU F  52A   -12.948  52.173  30.780  1.00 23.95          N
ATOM   8698  CA   LEU F  52A   -13.699  53.426  30.985  1.00 24.71          C
```

| ATOM | 8699 | C    | LEU  | F | 52A | -15.214 | 53.223 | 31.113 | 1.00 | 26.47 | C |
| ATOM | 8700 | O    | LEU  | F | 52A | -15.753 | 52.136 | 30.835 | 1.00 | 24.61 | O |
| ATOM | 8701 | CB   | LEU  | F | 52A | -13.429 | 54.406 | 29.844 | 1.00 | 25.50 | C |
| ATOM | 8702 | CG   | LEU  | F | 52A | -11.980 | 54.633 | 29.415 | 1.00 | 24.84 | C |
| ATOM | 8703 | CD1  | LEU  | F | 52A | -11.927 | 55.692 | 28.341 | 1.00 | 23.99 | C |
| ATOM | 8704 | CD2  | LEU  | F | 52A | -11.098 | 55.020 | 30.606 | 1.00 | 25.27 | C |
| ATOM | 8705 | N    | LYS  | F | 52B | -15.913 | 54.280 | 31.520 | 1.00 | 28.61 | N |
| ATOM | 8706 | CA   | LYS  | F | 52B | -17.383 | 54.192 | 31.594 | 1.00 | 31.63 | C |
| ATOM | 8707 | C    | LYS  | F | 52B | -17.954 | 53.757 | 30.245 | 1.00 | 31.01 | C |
| ATOM | 8708 | O    | LYS  | F | 52B | -18.809 | 52.875 | 30.183 | 1.00 | 30.17 | O |
| ATOM | 8709 | CB   | LYS  | F | 52B | -18.013 | 55.510 | 32.040 | 1.00 | 32.60 | C |
| ATOM | 8710 | CG   | LYS  | F | 52B | -19.484 | 55.348 | 32.424 | 1.00 | 33.84 | C |
| ATOM | 8711 | CD   | LYS  | F | 52B | -20.149 | 56.681 | 32.725 | 1.00 | 34.90 | C |
| ATOM | 8712 | CE   | LYS  | F | 52B | -21.666 | 56.543 | 32.616 | 1.00 | 36.62 | C |
| ATOM | 8713 | NZ   | LYS  | F | 52B | -22.373 | 57.831 | 32.829 | 1.00 | 38.85 | N |
| ATOM | 8714 | N    | SER  | F | 52C | -17.423 | 54.335 | 29.170 | 1.00 | 31.81 | N |
| ATOM | 8715 | CA   | SER  | F | 52C | -17.846 | 53.998 | 27.817 | 1.00 | 31.82 | C |
| ATOM | 8716 | C    | SER  | F | 52C | -17.665 | 52.520 | 27.481 | 1.00 | 32.38 | C |
| ATOM | 8717 | O    | SER  | F | 52C | -18.233 | 52.044 | 26.506 | 1.00 | 31.95 | O |
| ATOM | 8718 | CB   | SER  | F | 52C | -17.133 | 54.874 | 26.772 | 1.00 | 31.69 | C |
| ATOM | 8719 | OG   | SER  | F | 52C | -15.765 | 55.127 | 27.081 | 1.00 | 32.99 | O |
| ATOM | 8720 | N    | ASP  | F | 53  | -16.888 | 51.805 | 28.296 | 1.00 | 31.57 | N |
| ATOM | 8721 | CA   | ASP  | F | 53  | -16.660 | 50.378 | 28.128 | 1.00 | 30.87 | C |
| ATOM | 8722 | C    | ASP  | F | 53  | -17.434 | 49.562 | 29.150 | 1.00 | 30.38 | C |
| ATOM | 8723 | O    | ASP  | F | 53  | -17.137 | 48.395 | 29.338 | 1.00 | 32.36 | O |
| ATOM | 8724 | CB   | ASP  | F | 53  | -15.170 | 50.077 | 28.295 | 1.00 | 31.02 | C |
| ATOM | 8725 | CG   | ASP  | F | 53  | -14.298 | 50.962 | 27.436 | 1.00 | 31.59 | C |
| ATOM | 8726 | OD1  | ASP  | F | 53  | -14.527 | 50.980 | 26.210 | 1.00 | 33.14 | O |
| ATOM | 8727 | OD2  | ASP  | F | 53  | -13.378 | 51.628 | 27.974 | 1.00 | 29.67 | O |
| ATOM | 8728 | N    | ASN  | F | 54  | -18.425 | 50.163 | 29.806 | 1.00 | 29.98 | N |
| ATOM | 8729 | CA   | ASN  | F | 54  | -19.118 | 49.511 | 30.923 | 1.00 | 29.52 | C |
| ATOM | 8730 | C    | ASN  | F | 54  | -18.142 | 49.070 | 32.037 | 1.00 | 28.38 | C |
| ATOM | 8731 | O    | ASN  | F | 54  | -18.365 | 48.058 | 32.718 | 1.00 | 28.12 | O |
| ATOM | 8732 | CB  A| ASN  | F | 54  | -19.969 | 48.336 | 30.422 | 0.50 | 30.31 | C |
| ATOM | 8733 | CB  B| ASN  | F | 54  | -19.904 | 48.276 | 30.449 | 0.50 | 29.93 | C |
| ATOM | 8734 | CG  A| ASN  | F | 54  | -21.116 | 47.987 | 31.371 | 0.50 | 30.70 | C |
| ATOM | 8735 | CG  B| ASN  | F | 54  | -21.195 | 48.618 | 29.744 | 0.50 | 30.30 | C |
| ATOM | 8736 | OD1 A| ASN  | F | 54  | -21.514 | 48.789 | 32.221 | 0.50 | 31.79 | O |
| ATOM | 8737 | OD1 B| ASN  | F | 54  | -21.636 | 49.767 | 29.716 | 0.50 | 28.31 | O |
| ATOM | 8738 | ND2 A| ASN  | F | 54  | -21.646 | 46.784 | 31.226 | 0.50 | 31.43 | N |
| ATOM | 8739 | ND2 B| ASN  | F | 54  | -21.825 | 47.592 | 29.179 | 0.50 | 29.99 | N |
| ATOM | 8740 | N    | TYR  | F | 55  | -17.067 | 49.842 | 32.207 | 1.00 | 27.62 | N |
| ATOM | 8741 | CA   | TYR  | F | 55  | -16.054 | 49.593 | 33.236 | 1.00 | 26.82 | C |
| ATOM | 8742 | C    | TYR  | F | 55  | -15.536 | 48.147 | 33.212 | 1.00 | 25.34 | C |
| ATOM | 8743 | O    | TYR  | F | 55  | -15.358 | 47.510 | 34.260 | 1.00 | 24.43 | O |
| ATOM | 8744 | CB   | TYR  | F | 55  | -16.631 | 49.933 | 34.609 | 1.00 | 27.83 | C |
| ATOM | 8745 | CG   | TYR  | F | 55  | -16.938 | 51.396 | 34.824 | 1.00 | 27.91 | C |
| ATOM | 8746 | CD1  | TYR  | F | 55  | -15.925 | 52.343 | 34.800 | 1.00 | 28.67 | C |
| ATOM | 8747 | CD2  | TYR  | F | 55  | -18.234 | 51.833 | 35.095 | 1.00 | 29.85 | C |
| ATOM | 8748 | CE1  | TYR  | F | 55  | -16.183 | 53.692 | 35.018 | 1.00 | 28.76 | C |
| ATOM | 8749 | CE2  | TYR  | F | 55  | -18.509 | 53.202 | 35.325 | 1.00 | 28.91 | C |
| ATOM | 8750 | CZ   | TYR  | F | 55  | -17.481 | 54.114 | 35.287 | 1.00 | 29.55 | C |
| ATOM | 8751 | OH   | TYR  | F | 55  | -17.729 | 55.457 | 35.510 | 1.00 | 30.92 | O |
| ATOM | 8752 | N    | ALA  | F | 56  | -15.265 | 47.632 | 32.016 | 1.00 | 23.84 | N |
| ATOM | 8753 | CA   | ALA  | F | 56  | -14.812 | 46.249 | 31.886 | 1.00 | 23.74 | C |
| ATOM | 8754 | C    | ALA  | F | 56  | -13.532 | 46.046 | 32.685 | 1.00 | 23.09 | C |
| ATOM | 8755 | O    | ALA  | F | 56  | -12.716 | 46.953 | 32.799 | 1.00 | 21.13 | O |
| ATOM | 8756 | CB   | ALA  | F | 56  | -14.574 | 45.897 | 30.452 | 1.00 | 24.45 | C |
| ATOM | 8757 | N    | THR  | F | 57  | -13.389 | 44.860 | 33.256 | 1.00 | 23.67 | N |
| ATOM | 8758 | CA   | THR  | F | 57  | -12.263 | 44.559 | 34.146 | 1.00 | 23.30 | C |
| ATOM | 8759 | C    | THR  | F | 57  | -11.598 | 43.302 | 33.651 | 1.00 | 22.50 | C |
| ATOM | 8760 | O    | THR  | F | 57  | -12.251 | 42.484 | 33.027 | 1.00 | 23.61 | O |
| ATOM | 8761 | CB   | THR  | F | 57  | -12.715 | 44.342 | 35.590 | 1.00 | 23.60 | C |
| ATOM | 8762 | OG1  | THR  | F | 57  | -13.635 | 43.236 | 35.656 | 1.00 | 24.71 | O |
| ATOM | 8763 | CG2  | THR  | F | 57  | -13.355 | 45.592 | 36.134 | 1.00 | 23.42 | C |

| ATOM | 8764 | N   | TYR  | F | 58  | -10.290 | 43.180 | 33.902 | 1.00 | 21.79 | N |
|------|------|-----|------|---|-----|---------|--------|--------|------|-------|---|
| ATOM | 8765 | CA  | TYR  | F | 58  | -9.479  | 42.059 | 33.412 | 1.00 | 21.62 | C |
| ATOM | 8766 | C   | TYR  | F | 58  | -8.501  | 41.716 | 34.521 | 1.00 | 20.73 | C |
| ATOM | 8767 | O   | TYR  | F | 58  | -8.087  | 42.597 | 35.258 | 1.00 | 21.26 | O |
| ATOM | 8768 | CB  | TYR  | F | 58  | -8.728  | 42.436 | 32.116 | 1.00 | 20.75 | C |
| ATOM | 8769 | CG  | TYR  | F | 58  | -9.657  | 42.905 | 31.014 | 1.00 | 20.00 | C |
| ATOM | 8770 | CD1 | TYR  | F | 58  | -10.394 | 41.993 | 30.261 | 1.00 | 21.02 | C |
| ATOM | 8771 | CD2 | TYR  | F | 58  | -9.826  | 44.266 | 30.751 | 1.00 | 21.89 | C |
| ATOM | 8772 | CE1 | TYR  | F | 58  | -11.269 | 42.424 | 29.269 | 1.00 | 21.23 | C |
| ATOM | 8773 | CE2 | TYR  | F | 58  | -10.702 | 44.697 | 29.779 | 1.00 | 21.25 | C |
| ATOM | 8774 | CZ  | TYR  | F | 58  | -11.414 | 43.789 | 29.044 | 1.00 | 20.85 | C |
| ATOM | 8775 | OH  | TYR  | F | 58  | -12.269 | 44.243 | 28.066 | 1.00 | 22.26 | O |
| ATOM | 8776 | N   | TYR  | F | 59  | -8.144  | 40.443 | 34.636 | 1.00 | 22.28 | N |
| ATOM | 8777 | CA  | TYR  | F | 59  | -7.257  | 39.987 | 35.705 | 1.00 | 21.48 | C |
| ATOM | 8778 | C   | TYR  | F | 59  | -6.217  | 38.994 | 35.214 | 1.00 | 22.86 | C |
| ATOM | 8779 | O   | TYR  | F | 59  | -6.430  | 38.278 | 34.234 | 1.00 | 22.49 | O |
| ATOM | 8780 | CB  | TYR  | F | 59  | -8.086  | 39.340 | 36.819 | 1.00 | 21.11 | C |
| ATOM | 8781 | CG  | TYR  | F | 59  | -9.113  | 40.270 | 37.422 | 1.00 | 21.93 | C |
| ATOM | 8782 | CD1 | TYR  | F | 59  | -8.772  | 41.157 | 38.446 | 1.00 | 20.92 | C |
| ATOM | 8783 | CD2 | TYR  | F | 59  | -10.419 | 40.276 | 36.966 | 1.00 | 21.74 | C |
| ATOM | 8784 | CE1 | TYR  | F | 59  | -9.713  | 42.006 | 39.009 | 1.00 | 20.29 | C |
| ATOM | 8785 | CE2 | TYR  | F | 59  | -11.371 | 41.128 | 37.515 | 1.00 | 21.06 | C |
| ATOM | 8786 | CZ  | TYR  | F | 59  | -11.020 | 41.988 | 38.540 | 1.00 | 20.38 | C |
| ATOM | 8787 | OH  | TYR  | F | 59  | -11.973 | 42.842 | 39.069 | 1.00 | 18.82 | O |
| ATOM | 8788 | N   | ALA  | F | 60  | -5.094  | 38.950 | 35.915 | 1.00 | 22.14 | N |
| ATOM | 8789 | CA  | ALA  | F | 60  | -4.123  | 37.895 | 35.740 | 1.00 | 23.75 | C |
| ATOM | 8790 | C   | ALA  | F | 60  | -4.727  | 36.596 | 36.229 | 1.00 | 24.80 | C |
| ATOM | 8791 | O   | ALA  | F | 60  | -5.413  | 36.574 | 37.254 | 1.00 | 21.97 | O |
| ATOM | 8792 | CB  | ALA  | F | 60  | -2.840  | 38.213 | 36.513 | 1.00 | 23.47 | C |
| ATOM | 8793 | N   | GLU  | F | 61  | -4.487  | 35.526 | 35.469 | 1.00 | 27.64 | N |
| ATOM | 8794 | CA  | GLU  | F | 61  | -4.921  | 34.170 | 35.835 | 1.00 | 29.59 | C |
| ATOM | 8795 | C   | GLU  | F | 61  | -4.635  | 33.844 | 37.310 | 1.00 | 30.56 | C |
| ATOM | 8796 | O   | GLU  | F | 61  | -5.454  | 33.200 | 37.978 | 1.00 | 32.30 | O |
| ATOM | 8797 | CB  | AGLU | F | 61  | -4.266  | 33.129 | 34.912 | 0.50 | 29.69 | C |
| ATOM | 8798 | CB  | BGLU | F | 61  | -4.235  | 33.138 | 34.919 | 0.50 | 29.65 | C |
| ATOM | 8799 | CG  | AGLU | F | 61  | -4.945  | 31.737 | 34.913 | 0.50 | 30.09 | C |
| ATOM | 8800 | CG  | BGLU | F | 61  | -4.229  | 31.685 | 35.450 | 0.50 | 30.19 | C |
| ATOM | 8801 | CD  | AGLU | F | 61  | -6.191  | 31.651 | 34.040 | 0.50 | 31.23 | C |
| ATOM | 8802 | CD  | BGLU | F | 61  | -4.074  | 30.635 | 34.350 | 0.50 | 30.75 | C |
| ATOM | 8803 | OE1 | AGLU | F | 61  | -6.700  | 32.702 | 33.593 | 0.50 | 30.88 | O |
| ATOM | 8804 | OE1 | BGLU | F | 61  | -3.581  | 30.971 | 33.252 | 0.50 | 31.38 | O |
| ATOM | 8805 | OE2 | AGLU | F | 61  | -6.668  | 30.513 | 33.803 | 0.50 | 32.00 | O |
| ATOM | 8806 | OE2 | BGLU | F | 61  | -4.452  | 29.467 | 34.589 | 0.50 | 32.73 | O |
| ATOM | 8807 | N   | SER  | F | 62  | -3.484  | 34.284 | 37.811 | 1.00 | 30.95 | N |
| ATOM | 8808 | CA  | SER  | F | 62  | -3.056  | 33.992 | 39.190 | 1.00 | 31.36 | C |
| ATOM | 8809 | C   | SER  | F | 62  | -3.876  | 34.665 | 40.315 | 1.00 | 31.93 | C |
| ATOM | 8810 | O   | SER  | F | 62  | -3.747  | 34.271 | 41.482 | 1.00 | 33.14 | O |
| ATOM | 8811 | CB  | SER  | F | 62  | -1.584  | 34.369 | 39.362 | 1.00 | 31.43 | C |
| ATOM | 8812 | OG  | SER  | F | 62  | -1.408  | 35.772 | 39.307 | 1.00 | 32.16 | O |
| ATOM | 8813 | N   | VAL  | F | 63  | -4.686  | 35.674 | 39.990 | 1.00 | 31.69 | N |
| ATOM | 8814 | CA  | VAL  | F | 63  | -5.496  | 36.375 | 41.012 | 1.00 | 31.84 | C |
| ATOM | 8815 | C   | VAL  | F | 63  | -6.994  | 36.395 | 40.717 | 1.00 | 32.46 | C |
| ATOM | 8816 | O   | VAL  | F | 63  | -7.771  | 37.009 | 41.455 | 1.00 | 32.04 | O |
| ATOM | 8817 | CB  | VAL  | F | 63  | -5.001  | 37.835 | 41.247 | 1.00 | 31.57 | C |
| ATOM | 8818 | CG1 | VAL  | F | 63  | -3.510  | 37.855 | 41.576 | 1.00 | 31.17 | C |
| ATOM | 8819 | CG2 | VAL  | F | 63  | -5.298  | 38.742 | 40.047 | 1.00 | 31.16 | C |
| ATOM | 8820 | N   | LYS  | F | 64  | -7.416  | 35.752 | 39.633 | 1.00 | 33.98 | N |
| ATOM | 8821 | CA  | LYS  | F | 64  | -8.838  | 35.713 | 39.300 | 1.00 | 35.83 | C |
| ATOM | 8822 | C   | LYS  | F | 64  | -9.587  | 35.041 | 40.444 | 1.00 | 34.90 | C |
| ATOM | 8823 | O   | LYS  | F | 64  | -9.133  | 34.039 | 40.974 | 1.00 | 35.30 | O |
| ATOM | 8824 | CB  | LYS  | F | 64  | -9.087  | 34.977 | 37.975 | 1.00 | 36.57 | C |
| ATOM | 8825 | CG  | LYS  | F | 64  | -8.968  | 35.867 | 36.765 | 1.00 | 38.03 | C |
| ATOM | 8826 | CD  | LYS  | F | 64  | -8.962  | 35.061 | 35.466 | 1.00 | 38.82 | C |
| ATOM | 8827 | CE  | LYS  | F | 64  | -8.571  | 35.919 | 34.263 | 1.00 | 39.20 | C |
| ATOM | 8828 | NZ  | LYS  | F | 64  | -7.813  | 35.117 | 33.254 | 1.00 | 41.05 | N |

.

| ATOM | 8829 | N   | GLY | F | 65 | -10.699 | 35.640 | 40.860 | 1.00 | 34.38 | N |
| ATOM | 8830 | CA  | GLY | F | 65 | -11.494 | 35.108 | 41.959 | 1.00 | 33.60 | C |
| ATOM | 8831 | C   | GLY | F | 65 | -11.076 | 35.612 | 43.324 | 1.00 | 32.78 | C |
| ATOM | 8832 | O   | GLY | F | 65 | -11.817 | 35.435 | 44.288 | 1.00 | 34.97 | O |
| ATOM | 8833 | N   | LYS | F | 66 | -9.898  | 36.227 | 43.413 | 1.00 | 30.08 | N |
| ATOM | 8834 | CA  | LYS | F | 66 | -9.380  | 36.800 | 44.664 | 1.00 | 29.81 | C |
| ATOM | 8835 | C   | LYS | F | 66 | -9.414  | 38.337 | 44.686 | 1.00 | 27.10 | C |
| ATOM | 8836 | O   | LYS | F | 66 | -9.690  | 38.953 | 45.723 | 1.00 | 27.81 | O |
| ATOM | 8837 | CB  | LYS | F | 66 | -7.936  | 36.368 | 44.868 | 1.00 | 30.27 | C |
| ATOM | 8838 | CG  | LYS | F | 66 | -7.761  | 34.958 | 45.333 | 1.00 | 31.93 | C |
| ATOM | 8839 | CD  | LYS | F | 66 | -6.320  | 34.511 | 45.152 | 1.00 | 32.40 | C |
| ATOM | 8840 | CE  | LYS | F | 66 | -5.332  | 35.315 | 45.994 | 1.00 | 33.28 | C |
| ATOM | 8841 | NZ  | LYS | F | 66 | -3.949  | 34.719 | 45.985 | 1.00 | 34.42 | N |
| ATOM | 8842 | N   | PHE | F | 67 | -9.076  | 38.944 | 43.554 | 1.00 | 24.69 | N |
| ATOM | 8843 | CA  | PHE | F | 67 | -8.907  | 40.396 | 43.452 | 1.00 | 23.63 | C |
| ATOM | 8844 | C   | PHE | F | 67 | -10.109 | 41.006 | 42.748 | 1.00 | 22.82 | C |
| ATOM | 8845 | O   | PHE | F | 67 | -10.651 | 40.418 | 41.812 | 1.00 | 21.80 | O |
| ATOM | 8846 | CB  | PHE | F | 67 | -7.629  | 40.738 | 42.680 | 1.00 | 23.45 | C |
| ATOM | 8847 | CG  | PHE | F | 67 | -6.348  | 40.522 | 43.467 | 1.00 | 23.18 | C |
| ATOM | 8848 | CD1 | PHE | F | 67 | -6.320  | 39.742 | 44.621 | 1.00 | 22.46 | C |
| ATOM | 8849 | CD2 | PHE | F | 67 | -5.151  | 41.070 | 43.014 | 1.00 | 22.75 | C |
| ATOM | 8850 | CE1 | PHE | F | 67 | -5.127  | 39.540 | 45.307 | 1.00 | 22.46 | C |
| ATOM | 8851 | CE2 | PHE | F | 67 | -3.977  | 40.876 | 43.704 | 1.00 | 20.63 | C |
| ATOM | 8852 | CZ  | PHE | F | 67 | -3.963  | 40.115 | 44.849 | 1.00 | 22.04 | C |
| ATOM | 8853 | N   | THR | F | 68 | -10.519 | 42.178 | 43.213 | 1.00 | 22.13 | N |
| ATOM | 8854 | CA  | THR | F | 68 | -11.505 | 42.986 | 42.503 | 1.00 | 22.21 | C |
| ATOM | 8855 | C   | THR | F | 68 | -10.992 | 44.402 | 42.355 | 1.00 | 19.89 | C |
| ATOM | 8856 | O   | THR | F | 68 | -10.750 | 45.092 | 43.336 | 1.00 | 20.44 | O |
| ATOM | 8857 | CB  | THR | F | 68 | -12.860 | 43.038 | 43.207 | 1.00 | 22.44 | C |
| ATOM | 8858 | OG1 | THR | F | 68 | -13.342 | 41.710 | 43.368 | 1.00 | 23.14 | O |
| ATOM | 8859 | CG2 | THR | F | 68 | -13.847 | 43.810 | 42.370 | 1.00 | 24.39 | C |
| ATOM | 8860 | N   | ILE | F | 69 | -10.845 | 44.826 | 41.104 | 1.00 | 19.58 | N |
| ATOM | 8861 | CA  | ILE | F | 69 | -10.510 | 46.204 | 40.789 | 1.00 | 18.69 | C |
| ATOM | 8862 | C   | ILE | F | 69 | -11.782 | 47.024 | 40.539 | 1.00 | 19.20 | C |
| ATOM | 8863 | O   | ILE | F | 69 | -12.755 | 46.523 | 39.976 | 1.00 | 20.38 | O |
| ATOM | 8864 | CB  | ILE | F | 69 | -9.512  | 46.269 | 39.587 | 1.00 | 18.28 | C |
| ATOM | 8865 | CG1 | ILE | F | 69 | -8.853  | 47.644 | 39.490 | 1.00 | 18.83 | C |
| ATOM | 8866 | CG2 | ILE | F | 69 | -10.168 | 45.914 | 38.296 | 1.00 | 19.12 | C |
| ATOM | 8867 | CD1 | ILE | F | 69 | -7.869  | 47.754 | 38.327 | 1.00 | 19.25 | C |
| ATOM | 8868 | N   | SER | F | 70 | -11.753 | 48.286 | 40.963 | 1.00 | 20.33 | N |
| ATOM | 8869 | CA  | SER | F | 70 | -12.863 | 49.201 | 40.750 | 1.00 | 20.64 | C |
| ATOM | 8870 | C   | SER | F | 70 | -12.366 | 50.631 | 40.753 | 1.00 | 20.87 | C |
| ATOM | 8871 | O   | SER | F | 70 | -11.280 | 50.916 | 41.226 | 1.00 | 20.74 | O |
| ATOM | 8872 | CB  | SER | F | 70 | -13.920 | 49.030 | 41.845 | 1.00 | 21.37 | C |
| ATOM | 8873 | OG  | SER | F | 70 | -13.369 | 49.283 | 43.118 | 1.00 | 21.31 | O |
| ATOM | 8874 | N   | ARG | F | 71 | -13.188 | 51.541 | 40.249 | 1.00 | 21.38 | N |
| ATOM | 8875 | CA  | ARG | F | 71 | -12.808 | 52.936 | 40.179 | 1.00 | 21.82 | C |
| ATOM | 8876 | C   | ARG | F | 71 | -13.976 | 53.839 | 40.551 | 1.00 | 23.70 | C |
| ATOM | 8877 | O   | ARG | F | 71 | -15.125 | 53.443 | 40.416 | 1.00 | 23.87 | O |
| ATOM | 8878 | CB  | ARG | F | 71 | -12.302 | 53.273 | 38.785 | 1.00 | 21.55 | C |
| ATOM | 8879 | CG  | ARG | F | 71 | -13.345 | 53.136 | 37.670 | 1.00 | 21.23 | C |
| ATOM | 8880 | CD  | ARG | F | 71 | -12.693 | 53.189 | 36.306 | 1.00 | 21.34 | C |
| ATOM | 8881 | NE  | ARG | F | 71 | -12.090 | 54.493 | 36.030 | 1.00 | 20.75 | N |
| ATOM | 8882 | CZ  | ARG | F | 71 | -11.170 | 54.720 | 35.097 | 1.00 | 19.90 | C |
| ATOM | 8883 | NH1 | ARG | F | 71 | -10.681 | 53.724 | 34.371 | 1.00 | 20.20 | N |
| ATOM | 8884 | NH2 | ARG | F | 71 | -10.700 | 55.949 | 34.929 | 1.00 | 20.99 | N |
| ATOM | 8885 | N   | ASP | F | 72 | -13.643 | 55.031 | 41.025 | 1.00 | 25.69 | N |
| ATOM | 8886 | CA  | ASP | F | 72 | -14.613 | 56.045 | 41.371 | 1.00 | 26.51 | C |
| ATOM | 8887 | C   | ASP | F | 72 | -14.184 | 57.309 | 40.673 | 1.00 | 25.98 | C |
| ATOM | 8888 | O   | ASP | F | 72 | -13.401 | 58.100 | 41.215 | 1.00 | 26.02 | O |
| ATOM | 8889 | CB  | ASP | F | 72 | -14.682 | 56.256 | 42.886 | 1.00 | 27.56 | C |
| ATOM | 8890 | CG  | ASP | F | 72 | -15.766 | 57.251 | 43.276 | 1.00 | 29.01 | C |
| ATOM | 8891 | OD1 | ASP | F | 72 | -16.151 | 58.064 | 42.405 | 1.00 | 30.02 | O |
| ATOM | 8892 | OD2 | ASP | F | 72 | -16.249 | 57.212 | 44.428 | 1.00 | 31.34 | O |
| ATOM | 8893 | N   | ASP | F | 73 | -14.711 | 57.508 | 39.466 | 1.00 | 27.17 | N |

| ATOM | 8894 | CA | ASP F | 73 | -14.282 | 58.634 | 38.634 | 1.00 | 28.16 | C |
|------|------|-----|-------|----|---------|--------|--------|------|-------|---|
| ATOM | 8895 | C | ASP F | 73 | -14.505 | 59.954 | 39.359 | 1.00 | 28.50 | C |
| ATOM | 8896 | O | ASP F | 73 | -13.709 | 60.876 | 39.207 | 1.00 | 29.20 | O |
| ATOM | 8897 | CB | ASP F | 73 | -14.975 | 58.627 | 37.261 | 1.00 | 27.95 | C |
| ATOM | 8898 | CG | ASP F | 73 | -14.502 | 57.468 | 36.343 | 1.00 | 29.74 | C |
| ATOM | 8899 | OD1 | ASP F | 73 | -13.420 | 56.896 | 36.581 | 1.00 | 29.21 | O |
| ATOM | 8900 | OD2 | ASP F | 73 | -15.217 | 57.131 | 35.375 | 1.00 | 28.67 | O |
| ATOM | 8901 | N | SER F | 74 | -15.557 | 60.045 | 40.175 | 1.00 | 30.19 | N |
| ATOM | 8902 | CA | SER F | 74 | -15.856 | 61.320 | 40.862 | 1.00 | 31.04 | C |
| ATOM | 8903 | C | SER F | 74 | -14.791 | 61.690 | 41.877 | 1.00 | 31.42 | C |
| ATOM | 8904 | O | SER F | 74 | -14.556 | 62.879 | 42.125 | 1.00 | 31.68 | O |
| ATOM | 8905 | CB | SER F | 74 | -17.251 | 61.313 | 41.520 | 1.00 | 31.60 | C |
| ATOM | 8906 | OG | SER F | 74 | -17.392 | 60.304 | 42.510 | 1.00 | 33.53 | O |
| ATOM | 8907 | N | LYS F | 75 | -14.138 | 60.685 | 42.462 | 1.00 | 30.36 | N |
| ATOM | 8908 | CA | LYS F | 75 | -13.060 | 60.932 | 43.427 | 1.00 | 30.67 | C |
| ATOM | 8909 | C | LYS F | 75 | -11.664 | 60.714 | 42.857 | 1.00 | 29.31 | C |
| ATOM | 8910 | O | LYS F | 75 | -10.668 | 60.872 | 43.562 | 1.00 | 27.91 | O |
| ATOM | 8911 | CB | LYS F | 75 | -13.265 | 60.073 | 44.657 | 1.00 | 31.67 | C |
| ATOM | 8912 | CG | LYS F | 75 | -14.547 | 60.433 | 45.386 | 1.00 | 32.68 | C |
| ATOM | 8913 | CD | LYS F | 75 | -14.846 | 59.458 | 46.491 | 1.00 | 33.34 | C |
| ATOM | 8914 | CE | LYS F | 75 | -16.203 | 59.752 | 47.128 | 1.00 | 34.12 | C |
| ATOM | 8915 | NZ | LYS F | 75 | -16.666 | 58.607 | 47.960 | 1.00 | 35.93 | N |
| ATOM | 8916 | N | SER F | 76 | -11.598 | 60.398 | 41.568 | 1.00 | 28.67 | N |
| ATOM | 8917 | CA | SER F | 76 | -10.331 | 60.145 | 40.888 | 1.00 | 27.84 | C |
| ATOM | 8918 | C | SER F | 76 | -9.545 | 59.079 | 41.632 | 1.00 | 25.96 | C |
| ATOM | 8919 | O | SER F | 76 | -8.340 | 59.240 | 41.845 | 1.00 | 24.81 | O |
| ATOM | 8920 | CB | SER F | 76 | -9.502 | 61.431 | 40.764 | 1.00 | 28.15 | C |
| ATOM | 8921 | OG | SER F | 76 | -10.078 | 62.286 | 39.800 | 1.00 | 29.63 | O |
| ATOM | 8922 | N | ARG F | 77 | -10.241 | 58.010 | 42.019 | 1.00 | 25.07 | N |
| ATOM | 8923 | CA | ARG F | 77 | -9.673 | 56.978 | 42.862 | 1.00 | 25.60 | C |
| ATOM | 8924 | C | ARG F | 77 | -9.864 | 55.583 | 42.281 | 1.00 | 24.19 | C |
| ATOM | 8925 | O | ARG F | 77 | -10.958 | 55.202 | 41.860 | 1.00 | 23.19 | O |
| ATOM | 8926 | CB | ARG F | 77 | -10.274 | 57.026 | 44.259 | 1.00 | 26.04 | C |
| ATOM | 8927 | CG | ARG F | 77 | -9.450 | 56.260 | 45.297 | 1.00 | 29.38 | C |
| ATOM | 8928 | CD | ARG F | 77 | -9.881 | 56.581 | 46.717 | 1.00 | 30.51 | C |
| ATOM | 8929 | NE | ARG F | 77 | -9.273 | 57.816 | 47.213 | 1.00 | 33.10 | N |
| ATOM | 8930 | CZ | ARG F | 77 | -8.052 | 57.897 | 47.746 | 1.00 | 35.02 | C |
| ATOM | 8931 | NH1 | ARG F | 77 | -7.261 | 56.818 | 47.827 | 1.00 | 35.31 | N |
| ATOM | 8932 | NH2 | ARG F | 77 | -7.599 | 59.075 | 48.184 | 1.00 | 35.13 | N |
| ATOM | 8933 | N | LEU F | 78 | -8.768 | 54.829 | 42.296 | 1.00 | 22.10 | N |
| ATOM | 8934 | CA | LEU F | 78 | -8.755 | 53.424 | 41.891 | 1.00 | 19.37 | C |
| ATOM | 8935 | C | LEU F | 78 | -8.620 | 52.597 | 43.144 | 1.00 | 19.58 | C |
| ATOM | 8936 | O | LEU F | 78 | -7.915 | 53.011 | 44.065 | 1.00 | 19.05 | O |
| ATOM | 8937 | CB | LEU F | 78 | -7.537 | 53.175 | 41.011 | 1.00 | 20.32 | C |
| ATOM | 8938 | CG | LEU F | 78 | -7.355 | 51.769 | 40.423 | 1.00 | 19.07 | C |
| ATOM | 8939 | CD1 | LEU F | 78 | -8.429 | 51.501 | 39.409 | 1.00 | 19.62 | C |
| ATOM | 8940 | CD2 | LEU F | 78 | -5.987 | 51.700 | 39.780 | 1.00 | 17.96 | C |
| ATOM | 8941 | N | TYR F | 79 | -9.262 | 51.434 | 43.169 | 1.00 | 18.91 | N |
| ATOM | 8942 | CA | TYR F | 79 | -9.210 | 50.527 | 44.319 | 1.00 | 21.06 | C |
| ATOM | 8943 | C | TYR F | 79 | -8.858 | 49.112 | 43.888 | 1.00 | 20.33 | C |
| ATOM | 8944 | O | TYR F | 79 | -9.147 | 48.720 | 42.763 | 1.00 | 20.92 | O |
| ATOM | 8945 | CB | TYR F | 79 | -10.570 | 50.417 | 45.012 | 1.00 | 23.72 | C |
| ATOM | 8946 | CG | TYR F | 79 | -11.182 | 51.716 | 45.459 | 1.00 | 24.94 | C |
| ATOM | 8947 | CD1 | TYR F | 79 | -10.936 | 52.229 | 46.731 | 1.00 | 26.94 | C |
| ATOM | 8948 | CD2 | TYR F | 79 | -12.043 | 52.408 | 44.621 | 1.00 | 26.87 | C |
| ATOM | 8949 | CE1 | TYR F | 79 | -11.515 | 53.433 | 47.139 | 1.00 | 27.55 | C |
| ATOM | 8950 | CE2 | TYR F | 79 | -12.623 | 53.598 | 45.013 | 1.00 | 27.05 | C |
| ATOM | 8951 | CZ | TYR F | 79 | -12.362 | 54.108 | 46.266 | 1.00 | 27.61 | C |
| ATOM | 8952 | OH | TYR F | 79 | -12.966 | 55.299 | 46.627 | 1.00 | 27.36 | O |
| ATOM | 8953 | N | LEU F | 80 | -8.268 | 48.356 | 44.811 | 1.00 | 19.91 | N |
| ATOM | 8954 | CA | LEU F | 80 | -8.075 | 46.914 | 44.651 | 1.00 | 19.67 | C |
| ATOM | 8955 | C | LEU F | 80 | -8.428 | 46.217 | 45.959 | 1.00 | 19.56 | C |
| ATOM | 8956 | O | LEU F | 80 | -7.804 | 46.455 | 46.980 | 1.00 | 19.25 | O |
| ATOM | 8957 | CB | LEU F | 80 | -6.630 | 46.556 | 44.248 | 1.00 | 19.89 | C |
| ATOM | 8958 | CG | LEU F | 80 | -6.327 | 45.095 | 43.866 | 1.00 | 19.45 | C |

```
ATOM   8959  CD1 LEU F  80     -7.090  44.654  42.631  1.00 20.80         C
ATOM   8960  CD2 LEU F  80     -4.807  44.834  43.646  1.00 19.84         C
ATOM   8961  N   GLN F  81     -9.437  45.361  45.894  1.00 20.64         N
ATOM   8962  CA  GLN F  81     -9.897  44.580  47.011  1.00 21.02         C
ATOM   8963  C   GLN F  81     -9.255  43.246  46.816  1.00 21.02         C
ATOM   8964  O   GLN F  81     -9.415  42.634  45.773  1.00 19.90         O
ATOM   8965  CB  GLN F  81    -11.423  44.448  47.000  1.00 21.98         C
ATOM   8966  CG  GLN F  81    -11.944  43.461  48.018  1.00 23.29         C
ATOM   8967  CD  GLN F  81    -11.709  43.931  49.418  1.00 23.32         C
ATOM   8968  OE1 GLN F  81    -11.978  45.091  49.726  1.00 26.98         O
ATOM   8969  NE2 GLN F  81    -11.196  43.050  50.280  1.00 23.74         N
ATOM   8970  N   MET F  82     -8.513  42.801  47.820  1.00 21.55         N
ATOM   8971  CA  MET F  82     -7.677  41.632  47.682  1.00 23.81         C
ATOM   8972  C   MET F  82     -8.087  40.646  48.760  1.00 23.60         C
ATOM   8973  O   MET F  82     -7.907  40.913  49.944  1.00 24.11         O
ATOM   8974  CB  MET F  82     -6.198  42.027  47.836  1.00 23.81         C
ATOM   8975  CG  MET F  82     -5.730  43.190  46.979  1.00 25.12         C
ATOM   8976  SD  MET F  82     -4.225  43.972  47.657  1.00 29.16         S
ATOM   8977  CE  MET F  82     -3.135  42.649  47.286  1.00 26.36         C
ATOM   8978  N   ASN F  82A    -8.659  39.518  48.357  1.00 24.74         N
ATOM   8979  CA  ASN F  82A    -9.157  38.519  49.310  1.00 25.27         C
ATOM   8980  C   ASN F  82A    -8.376  37.215  49.259  1.00 26.09         C
ATOM   8981  O   ASN F  82A    -7.647  36.952  48.315  1.00 25.46         O
ATOM   8982  CB  ASN F  82A   -10.648  38.230  49.065  1.00 26.51         C
ATOM   8983  CG  ASN F  82A   -11.523  39.459  49.232  1.00 27.15         C
ATOM   8984  OD1 ASN F  82A   -11.342  40.254  50.155  1.00 28.37         O
ATOM   8985  ND2 ASN F  82A   -12.499  39.609  48.343  1.00 28.76         N
ATOM   8986  N   ASN F  82B    -8.560  36.397  50.293  1.00 26.47         N
ATOM   8987  CA  ASN F  82B    -7.854  35.119  50.444  1.00 27.85         C
ATOM   8988  C   ASN F  82B    -6.376  35.152  50.050  1.00 27.04         C
ATOM   8989  O   ASN F  82B    -5.894  34.381  49.205  1.00 26.73         O
ATOM   8990  CB  ASN F  82B    -8.569  33.994  49.697  1.00 29.64         C
ATOM   8991  CG  ASN F  82B    -7.940  32.654  49.983  1.00 34.06         C
ATOM   8992  OD1 ASN F  82B    -7.613  32.346  51.144  1.00 37.76         O
ATOM   8993  ND2 ASN F  82B    -7.713  31.864  48.932  1.00 36.66         N
ATOM   8994  N   LEU F  82C    -5.651  36.040  50.704  1.00 25.34         N
ATOM   8995  CA  LEU F  82C    -4.292  36.314  50.327  1.00 24.45         C
ATOM   8996  C   LEU F  82C    -3.331  35.206  50.771  1.00 24.52         C
ATOM   8997  O   LEU F  82C    -3.530  34.522  51.773  1.00 24.18         O
ATOM   8998  CB  LEU F  82C    -3.864  37.679  50.875  1.00 23.48         C
ATOM   8999  CG  LEU F  82C    -4.467  38.866  50.138  1.00 23.95         C
ATOM   9000  CD1 LEU F  82C    -4.429  40.092  51.000  1.00 22.46         C
ATOM   9001  CD2 LEU F  82C    -3.693  39.128  48.842  1.00 26.53         C
ATOM   9002  N   ARG F  83     -2.296  35.030  49.964  1.00 25.00         N
ATOM   9003  CA  ARG F  83     -1.239  34.086  50.222  1.00 26.33         C
ATOM   9004  C   ARG F  83      0.070  34.827  50.335  1.00 24.47         C
ATOM   9005  O   ARG F  83      0.178  35.992  49.955  1.00 21.90         O
ATOM   9006  CB  ARG F  83     -1.163  33.063  49.085  1.00 28.72         C
ATOM   9007  CG  ARG F  83     -1.507  31.658  49.507  1.00 34.32         C
ATOM   9008  CD  ARG F  83     -2.884  31.564  50.157  1.00 38.76         C
ATOM   9009  NE  ARG F  83     -2.847  30.929  51.481  1.00 40.37         N
ATOM   9010  CZ  ARG F  83     -3.865  30.936  52.344  1.00 42.31         C
ATOM   9011  NH1 ARG F  83     -5.012  31.534  52.024  1.00 42.43         N
ATOM   9012  NH2 ARG F  83     -3.743  30.333  53.531  1.00 42.26         N
ATOM   9013  N   THR F  84      1.069  34.146  50.876  1.00 23.71         N
ATOM   9014  CA  THR F  84      2.384  34.731  51.049  1.00 23.93         C
ATOM   9015  C   THR F  84      2.922  35.281  49.735  1.00 22.98         C
ATOM   9016  O   THR F  84      3.525  36.343  49.711  1.00 23.62         O
ATOM   9017  CB  THR F  84      3.354  33.688  51.652  1.00 25.42         C
ATOM   9018  OG1 THR F  84      3.105  33.593  53.064  1.00 29.79         O
ATOM   9019  CG2 THR F  84      4.809  34.087  51.415  1.00 26.77         C
ATOM   9020  N   GLU F  85      2.667  34.558  48.647  1.00 22.70         N
ATOM   9021  CA AGLU F  85      3.095  34.906  47.291  0.50 22.66         C
ATOM   9022  CA BGLU F  85      3.174  34.960  47.323  0.50 22.77         C
ATOM   9023  C   GLU F  85      2.552  36.252  46.789  1.00 22.63         C
```

```
ATOM   9024  O    GLU  F  85      3.070  36.810  45.819  1.00 23.15          O
ATOM   9025  CB  AGLU  F  85      2.639  33.796  46.334  0.50 23.75          C
ATOM   9026  CB  BGLU  F  85      3.042  33.841  46.274  0.50 24.05          C
ATOM   9027  CG  AGLU  F  85      3.427  32.472  46.441  0.50 24.07          C
ATOM   9028  CG  BGLU  F  85      1.618  33.323  46.006  0.50 24.69          C
ATOM   9029  CD  AGLU  F  85      3.261  31.699  47.763  0.50 25.37          C
ATOM   9030  CD  BGLU  F  85      1.225  32.151  46.897  0.50 26.15          C
ATOM   9031  OE1 AGLU  F  85      2.311  31.961  48.542  0.50 24.61          O
ATOM   9032  OE1 BGLU  F  85      1.944  31.861  47.878  0.50 26.57          O
ATOM   9033  OE2 AGLU  F  85      4.101  30.798  48.012  0.50 25.51          O
ATOM   9034  OE2 BGLU  F  85      0.172  31.531  46.631  0.50 27.56          O
ATOM   9035  N    ASP  F  86      1.480  36.743  47.433  1.00 21.13          N
ATOM   9036  CA   ASP  F  86      0.859  38.054  47.089  1.00 20.26          C
ATOM   9037  C    ASP  F  86      1.576  39.256  47.715  1.00 19.74          C
ATOM   9038  O    ASP  F  86      1.248  40.443  47.444  1.00 17.88          O
ATOM   9039  CB   ASP  F  86     -0.615  38.058  47.494  1.00 19.47          C
ATOM   9040  CG   ASP  F  86     -1.413  37.032  46.724  1.00 21.32          C
ATOM   9041  OD1  ASP  F  86     -1.280  37.000  45.474  1.00 19.51          O
ATOM   9042  OD2  ASP  F  86     -2.142  36.242  47.344  1.00 21.24          O
ATOM   9043  N    THR  F  87      2.571  38.963  48.544  1.00 19.10          N
ATOM   9044  CA   THR  F  87      3.414  39.986  49.104  1.00 18.20          C
ATOM   9045  C    THR  F  87      4.127  40.742  47.984  1.00 17.98          C
ATOM   9046  O    THR  F  87      4.638  40.148  47.040  1.00 19.03          O
ATOM   9047  CB   THR  F  87      4.454  39.356  50.093  1.00 16.88          C
ATOM   9048  OG1  THR  F  87      3.763  38.757  51.198  1.00 15.94          O
ATOM   9049  CG2  THR  F  87      5.414  40.402  50.603  1.00 17.76          C
ATOM   9050  N    GLY  F  88      4.142  42.072  48.059  1.00 17.51          N
ATOM   9051  CA   GLY  F  88      4.917  42.852  47.127  1.00 17.20          C
ATOM   9052  C    GLY  F  88      4.591  44.322  47.244  1.00 16.62          C
ATOM   9053  O    GLY  F  88      3.839  44.711  48.131  1.00 15.17          O
ATOM   9054  N    ILE  F  89      5.209  45.107  46.373  1.00 16.25          N
ATOM   9055  CA   ILE  F  89      4.938  46.541  46.217  1.00 15.96          C
ATOM   9056  C    ILE  F  89      3.916  46.674  45.101  1.00 15.81          C
ATOM   9057  O    ILE  F  89      4.166  46.243  43.975  1.00 15.70          O
ATOM   9058  CB   ILE  F  89      6.197  47.327  45.840  1.00 16.29          C
ATOM   9059  CG1  ILE  F  89      7.301  47.169  46.895  1.00 18.53          C
ATOM   9060  CG2  ILE  F  89      5.868  48.830  45.663  1.00 16.03          C
ATOM   9061  CD1  ILE  F  89      6.804  47.113  48.303  1.00 22.42          C
ATOM   9062  N    TYR  F  90      2.741  47.196  45.439  1.00 14.55          N
ATOM   9063  CA   TYR  F  90      1.644  47.379  44.489  1.00 15.54          C
ATOM   9064  C    TYR  F  90      1.620  48.801  43.960  1.00 14.88          C
ATOM   9065  O    TYR  F  90      1.435  49.727  44.721  1.00 15.12          O
ATOM   9066  CB   TYR  F  90      0.314  47.040  45.142  1.00 15.72          C
ATOM   9067  CG   TYR  F  90      0.121  45.538  45.300  1.00 15.50          C
ATOM   9068  CD1  TYR  F  90      0.832  44.828  46.261  1.00 14.34          C
ATOM   9069  CD2  TYR  F  90     -0.778  44.844  44.485  1.00 16.23          C
ATOM   9070  CE1  TYR  F  90      0.666  43.440  46.411  1.00 15.62          C
ATOM   9071  CE2  TYR  F  90     -0.948  43.482  44.627  1.00 15.74          C
ATOM   9072  CZ   TYR  F  90     -0.213  42.784  45.575  1.00 14.45          C
ATOM   9073  OH   TYR  F  90     -0.399  41.436  45.689  1.00 16.54          O
ATOM   9074  N    TYR  F  91      1.821  48.930  42.650  1.00 15.67          N
ATOM   9075  CA   TYR  F  91      1.800  50.199  41.892  1.00 15.83          C
ATOM   9076  C    TYR  F  91      0.495  50.352  41.119  1.00 16.87          C
ATOM   9077  O    TYR  F  91      0.024  49.413  40.496  1.00 15.77          O
ATOM   9078  CB   TYR  F  91      2.903  50.208  40.824  1.00 17.30          C
ATOM   9079  CG   TYR  F  91      4.293  50.257  41.364  1.00 16.82          C
ATOM   9080  CD1  TYR  F  91      4.839  51.449  41.788  1.00 16.03          C
ATOM   9081  CD2  TYR  F  91      5.051  49.106  41.484  1.00 17.89          C
ATOM   9082  CE1  TYR  F  91      6.116  51.495  42.290  1.00 17.01          C
ATOM   9083  CE2  TYR  F  91      6.319  49.145  41.989  1.00 18.79          C
ATOM   9084  CZ   TYR  F  91      6.843  50.337  42.392  1.00 16.71          C
ATOM   9085  OH   TYR  F  91      8.114  50.399  42.895  1.00 19.66          O
ATOM   9086  N    CYS  F  92     -0.091  51.539  41.180  1.00 18.12          N
ATOM   9087  CA   CYS  F  92     -1.040  51.964  40.169  1.00 18.88          C
ATOM   9088  C    CYS  F  92     -0.232  52.292  38.899  1.00 18.14          C
```

| ATOM | 9089 | O   | CYS F | 92  | 0.786  | 53.001 | 38.960 | 1.00 17.41 | O |
|------|------|-----|-------|-----|--------|--------|--------|------------|---|
| ATOM | 9090 | CB  | CYS F | 92  | -1.854 | 53.184 | 40.641 | 1.00 21.30 | C |
| ATOM | 9091 | SG  | CYS F | 92  | -3.099 | 52.700 | 41.817 | 1.00 27.22 | S |
| ATOM | 9092 | N   | PHE F | 93  | -0.653 | 51.696 | 37.790 | 1.00 17.20 | N |
| ATOM | 9093 | CA  | PHE F | 93  | -0.038 | 51.878 | 36.487 | 1.00 17.30 | C |
| ATOM | 9094 | C   | PHE F | 93  | -1.140 | 52.248 | 35.519 | 1.00 17.30 | C |
| ATOM | 9095 | O   | PHE F | 93  | -2.079 | 51.442 | 35.278 | 1.00 17.34 | O |
| ATOM | 9096 | CB  | PHE F | 93  | 0.606  | 50.559 | 36.063 | 1.00 16.03 | C |
| ATOM | 9097 | CG  | PHE F | 93  | 1.182  | 50.550 | 34.661 | 1.00 16.55 | C |
| ATOM | 9098 | CD1 | PHE F | 93  | 2.057  | 51.541 | 34.242 | 1.00 15.45 | C |
| ATOM | 9099 | CD2 | PHE F | 93  | 0.883  | 49.515 | 33.788 | 1.00 16.80 | C |
| ATOM | 9100 | CE1 | PHE F | 93  | 2.630  | 51.511 | 32.963 | 1.00 15.51 | C |
| ATOM | 9101 | CE2 | PHE F | 93  | 1.443  | 49.487 | 32.504 | 1.00 16.23 | C |
| ATOM | 9102 | CZ  | PHE F | 93  | 2.315  | 50.493 | 32.105 | 1.00 15.62 | C |
| ATOM | 9103 | N   | LEU F | 94  | -1.044 | 53.468 | 34.998 | 1.00 18.08 | N |
| ATOM | 9104 | CA  | LEU F | 94  | -1.926 | 53.938 | 33.923 | 1.00 18.24 | C |
| ATOM | 9105 | C   | LEU F | 94  | -1.282 | 53.573 | 32.585 | 1.00 16.35 | C |
| ATOM | 9106 | O   | LEU F | 94  | -0.384 | 54.270 | 32.113 | 1.00 17.63 | O |
| ATOM | 9107 | CB  | LEU F | 94  | -2.143 | 55.451 | 34.006 | 1.00 18.52 | C |
| ATOM | 9108 | CG  | LEU F | 94  | -3.075 | 55.972 | 35.110 | 1.00 19.23 | C |
| ATOM | 9109 | CD1 | LEU F | 94  | -2.569 | 55.596 | 36.488 | 1.00 19.53 | C |
| ATOM | 9110 | CD2 | LEU F | 94  | -3.204 | 57.481 | 34.943 | 1.00 19.86 | C |
| ATOM | 9111 | N   | PRO F | 95  | -1.712 | 52.451 | 31.986 | 1.00 15.79 | N |
| ATOM | 9112 | CA  | PRO F | 95  | -1.006 | 51.924 | 30.819 | 1.00 16.67 | C |
| ATOM | 9113 | C   | PRO F | 95  | -1.040 | 52.949 | 29.671 | 1.00 18.28 | C |
| ATOM | 9114 | O   | PRO F | 95  | -2.119 | 53.466 | 29.399 | 1.00 19.00 | O |
| ATOM | 9115 | CB  | PRO F | 95  | -1.822 | 50.674 | 30.442 | 1.00 16.61 | C |
| ATOM | 9116 | CG  | PRO F | 95  | -2.689 | 50.382 | 31.633 | 1.00 15.58 | C |
| ATOM | 9117 | CD  | PRO F | 95  | -2.896 | 51.646 | 32.340 | 1.00 15.19 | C |
| ATOM | 9118 | N   | MET F | 96  | 0.066  | 53.275 | 29.004 | 1.00 17.57 | N |
| ATOM | 9119 | CA  | MET F | 96  | 1.419  | 52.782 | 29.280 | 1.00 18.80 | C |
| ATOM | 9120 | C   | MET F | 96  | 2.348  | 53.827 | 29.922 | 1.00 18.67 | C |
| ATOM | 9121 | O   | MET F | 96  | 3.526  | 53.565 | 30.113 | 1.00 18.74 | O |
| ATOM | 9122 | CB  | MET F | 96  | 2.032  | 52.349 | 27.954 | 1.00 17.44 | C |
| ATOM | 9123 | CG  | MET F | 96  | 1.311  | 51.210 | 27.281 | 1.00 18.48 | C |
| ATOM | 9124 | SD  | MET F | 96  | 1.343  | 49.669 | 28.202 | 1.00 19.44 | S |
| ATOM | 9125 | CE  | MET F | 96  | 3.053  | 49.178 | 28.053 | 1.00 18.70 | C |
| ATOM | 9126 | N   | ASP F | 101 | 1.838  | 55.003 | 30.275 | 1.00 19.76 | N |
| ATOM | 9127 | CA  | ASP F | 101 | 2.695  | 56.161 | 30.562 | 1.00 20.93 | C |
| ATOM | 9128 | C   | ASP F | 101 | 3.009  | 56.561 | 32.001 | 1.00 19.65 | C |
| ATOM | 9129 | O   | ASP F | 101 | 3.985  | 57.274 | 32.210 | 1.00 19.61 | O |
| ATOM | 9130 | CB  | ASP F | 101 | 2.114  | 57.410 | 29.880 | 1.00 23.21 | C |
| ATOM | 9131 | CG  | ASP F | 101 | 1.772  | 57.167 | 28.434 | 1.00 26.48 | C |
| ATOM | 9132 | OD1 | ASP F | 101 | 2.663  | 56.756 | 27.673 | 1.00 29.66 | O |
| ATOM | 9133 | OD2 | ASP F | 101 | 0.595  | 57.346 | 28.055 | 1.00 32.69 | O |
| ATOM | 9134 | N   | TYR F | 102 | 2.196  | 56.181 | 32.983 | 1.00 18.46 | N |
| ATOM | 9135 | CA  | TYR F | 102 | 2.385  | 56.703 | 34.345 | 1.00 18.43 | C |
| ATOM | 9136 | C   | TYR F | 102 | 2.392  | 55.625 | 35.406 | 1.00 17.21 | C |
| ATOM | 9137 | O   | TYR F | 102 | 1.535  | 54.752 | 35.392 | 1.00 15.68 | O |
| ATOM | 9138 | CB  | TYR F | 102 | 1.270  | 57.686 | 34.692 | 1.00 20.02 | C |
| ATOM | 9139 | CG  | TYR F | 102 | 1.046  | 58.746 | 33.640 | 1.00 20.75 | C |
| ATOM | 9140 | CD1 | TYR F | 102 | 1.962  | 59.770 | 33.467 | 1.00 22.22 | C |
| ATOM | 9141 | CD2 | TYR F | 102 | -0.073 | 58.717 | 32.828 | 1.00 21.36 | C |
| ATOM | 9142 | CE1 | TYR F | 102 | 1.786  | 60.738 | 32.502 | 1.00 22.54 | C |
| ATOM | 9143 | CE2 | TYR F | 102 | -0.276 | 59.709 | 31.853 | 1.00 20.36 | C |
| ATOM | 9144 | CZ  | TYR F | 102 | 0.666  | 60.707 | 31.702 | 1.00 21.82 | C |
| ATOM | 9145 | OH  | TYR F | 102 | 0.507  | 61.682 | 30.749 | 1.00 22.94 | O |
| ATOM | 9146 | N   | TRP F | 103 | 3.314  | 55.758 | 36.359 | 1.00 16.51 | N |
| ATOM | 9147 | CA  | TRP F | 103 | 3.396  | 54.893 | 37.518 | 1.00 17.27 | C |
| ATOM | 9148 | C   | TRP F | 103 | 3.238  | 55.686 | 38.819 | 1.00 17.78 | C |
| ATOM | 9149 | O   | TRP F | 103 | 3.719  | 56.812 | 38.933 | 1.00 18.41 | O |
| ATOM | 9150 | CB  | TRP F | 103 | 4.760  | 54.197 | 37.571 | 1.00 15.69 | C |
| ATOM | 9151 | CG  | TRP F | 103 | 5.046  | 53.205 | 36.472 | 1.00 15.32 | C |
| ATOM | 9152 | CD1 | TRP F | 103 | 5.442  | 53.489 | 35.201 | 1.00 14.57 | C |
| ATOM | 9153 | CD2 | TRP F | 103 | 5.017  | 51.778 | 36.570 | 1.00 14.12 | C |

```
ATOM   9154  NE1 TRP F 103      5.651  52.340  34.505  1.00 14.35           N
ATOM   9155  CE2 TRP F 103      5.387  51.270  35.311  1.00 14.00           C
ATOM   9156  CE3 TRP F 103      4.711  50.886  37.595  1.00 14.29           C
ATOM   9157  CZ2 TRP F 103      5.452  49.902  35.038  1.00 14.67           C
ATOM   9158  CZ3 TRP F 103      4.768  49.533  37.325  1.00 15.27           C
ATOM   9159  CH2 TRP F 103      5.147  49.054  36.055  1.00 14.77           C
ATOM   9160  N   GLY F 104      2.626  55.067  39.816  1.00 18.33           N
ATOM   9161  CA  GLY F 104      2.641  55.633  41.154  1.00 19.60           C
ATOM   9162  C   GLY F 104      3.923  55.398  41.931  1.00 20.36           C
ATOM   9163  O   GLY F 104      4.987  55.134  41.352  1.00 19.89           O
ATOM   9164  N   GLN F 105      3.799  55.504  43.254  1.00 21.10           N
ATOM   9165  CA  GLN F 105      4.912  55.366  44.182  1.00 22.74           C
ATOM   9166  C   GLN F 105      5.019  53.965  44.758  1.00 20.98           C
ATOM   9167  O   GLN F 105      6.085  53.583  45.246  1.00 19.80           O
ATOM   9168  CB  GLN F 105      4.742  56.334  45.368  1.00 26.12           C
ATOM   9169  CG  GLN F 105      4.290  57.739  45.009  1.00 30.48           C
ATOM   9170  CD  GLN F 105      5.132  58.390  43.932  1.00 34.45           C
ATOM   9171  OE1 GLN F 105      6.345  58.139  43.821  1.00 39.93           O
ATOM   9172  NE2 GLN F 105      4.495  59.251  43.128  1.00 37.24           N
ATOM   9173  N   GLY F 106      3.909  53.233  44.718  1.00 18.28           N
ATOM   9174  CA  GLY F 106      3.793  51.917  45.309  1.00 18.56           C
ATOM   9175  C   GLY F 106      3.368  51.975  46.764  1.00 18.90           C
ATOM   9176  O   GLY F 106      3.685  52.916  47.500  1.00 18.69           O
ATOM   9177  N   THR F 107      2.638  50.961  47.179  1.00 18.31           N
ATOM   9178  CA  THR F 107      2.352  50.755  48.584  1.00 18.84           C
ATOM   9179  C   THR F 107      2.648  49.284  48.836  1.00 18.46           C
ATOM   9180  O   THR F 107      2.317  48.436  48.020  1.00 16.66           O
ATOM   9181  CB  THR F 107      0.884  51.154  48.915  1.00 19.16           C
ATOM   9182  OG1 THR F 107      0.615  51.071  50.322  1.00 20.52           O
ATOM   9183  CG2 THR F 107     -0.093  50.261  48.186  1.00 19.97           C
ATOM   9184  N   SER F 108      3.266  49.001  49.978  1.00 18.40           N
ATOM   9185  CA  SER F 108      3.743  47.656  50.288  1.00 18.09           C
ATOM   9186  C   SER F 108      2.665  46.842  50.997  1.00 18.15           C
ATOM   9187  O   SER F 108      1.995  47.325  51.916  1.00 18.13           O
ATOM   9188  CB  SER F 108      5.028  47.772  51.112  1.00 18.31           C
ATOM   9189  OG  SER F 108      5.565  46.510  51.398  1.00 20.83           O
ATOM   9190  N   VAL F 109      2.455  45.619  50.514  1.00 16.49           N
ATOM   9191  CA  VAL F 109      1.547  44.657  51.125  1.00 16.83           C
ATOM   9192  C   VAL F 109      2.380  43.468  51.543  1.00 16.78           C
ATOM   9193  O   VAL F 109      3.064  42.888  50.716  1.00 16.16           O
ATOM   9194  CB  VAL F 109      0.434  44.210  50.136  1.00 18.27           C
ATOM   9195  CG1 VAL F 109     -0.402  43.053  50.705  1.00 18.25           C
ATOM   9196  CG2 VAL F 109     -0.475  45.420  49.786  1.00 18.74           C
ATOM   9197  N   THR F 110      2.350  43.147  52.833  1.00 16.49           N
ATOM   9198  CA  THR F 110      2.958  41.930  53.371  1.00 17.08           C
ATOM   9199  C   THR F 110      1.906  40.946  53.834  1.00 17.75           C
ATOM   9200  O   THR F 110      1.023  41.275  54.651  1.00 16.51           O
ATOM   9201  CB  THR F 110      3.888  42.256  54.542  1.00 18.84           C
ATOM   9202  OG1 THR F 110      4.900  43.142  54.080  1.00 18.78           O
ATOM   9203  CG2 THR F 110      4.512  40.986  55.097  1.00 19.48           C
ATOM   9204  N   VAL F 111      2.001  39.718  53.324  1.00 18.26           N
ATOM   9205  CA  VAL F 111      1.140  38.631  53.747  1.00 19.73           C
ATOM   9206  C   VAL F 111      2.020  37.680  54.530  1.00 20.45           C
ATOM   9207  O   VAL F 111      2.970  37.092  53.990  1.00 20.12           O
ATOM   9208  CB  VAL F 111      0.487  37.913  52.538  1.00 19.99           C
ATOM   9209  CG1 VAL F 111     -0.407  36.779  53.004  1.00 21.33           C
ATOM   9210  CG2 VAL F 111     -0.284  38.914  51.721  1.00 21.35           C
ATOM   9211  N   SER F 112      1.732  37.570  55.816  1.00 20.40           N
ATOM   9212  CA  SER F 112      2.619  36.871  56.737  1.00 21.91           C
ATOM   9213  C   SER F 112      1.917  36.579  58.052  1.00 23.06           C
ATOM   9214  O   SER F 112      1.046  37.333  58.478  1.00 22.12           O
ATOM   9215  CB  SER F 112      3.848  37.731  57.030  1.00 21.89           C
ATOM   9216  OG  SER F 112      4.702  37.130  57.998  1.00 22.42           O
ATOM   9217  N   SER F 113      2.332  35.496  58.701  1.00 23.89           N
ATOM   9218  CA  SER F 113      1.837  35.173  60.025  1.00 26.15           C
```

| ATOM | 9219 | C | SER | F | 113 | 2.764 | 35.711 | 61.106 | 1.00 | 26.47 | C |
|------|------|------|-----|---|-----|--------|--------|--------|------|-------|---|
| ATOM | 9220 | O | SER | F | 113 | 2.470 | 35.573 | 62.303 | 1.00 | 26.32 | O |
| ATOM | 9221 | CB | SER | F | 113 | 1.662 | 33.674 | 60.163 | 1.00 | 27.60 | C |
| ATOM | 9222 | OG | SER | F | 113 | 2.911 | 33.044 | 60.020 | 1.00 | 31.72 | O |
| ATOM | 9223 | N | ALA | F | 114 | 3.868 | 36.334 | 60.686 | 1.00 | 24.52 | N |
| ATOM | 9224 | CA | ALA | F | 114 | 4.751 | 37.066 | 61.600 | 1.00 | 24.87 | C |
| ATOM | 9225 | C | ALA | F | 114 | 4.067 | 38.322 | 62.093 | 1.00 | 24.16 | C |
| ATOM | 9226 | O | ALA | F | 114 | 3.188 | 38.867 | 61.432 | 1.00 | 25.38 | O |
| ATOM | 9227 | CB | ALA | F | 114 | 6.037 | 37.447 | 60.922 | 1.00 | 24.99 | C |
| ATOM | 9228 | N | LYS | F | 115 | 4.499 | 38.792 | 63.247 | 1.00 | 23.15 | N |
| ATOM | 9229 | CA | LYS | F | 115 | 3.859 | 39.934 | 63.852 | 1.00 | 25.15 | C |
| ATOM | 9230 | C | LYS | F | 115 | 4.597 | 41.218 | 63.527 | 1.00 | 22.88 | C |
| ATOM | 9231 | O | LYS | F | 115 | 5.822 | 41.234 | 63.410 | 1.00 | 24.98 | O |
| ATOM | 9232 | CB | LYS | F | 115 | 3.743 | 39.706 | 65.354 | 1.00 | 26.85 | C |
| ATOM | 9233 | CG | LYS | F | 115 | 2.397 | 39.053 | 65.776 | 1.00 | 31.24 | C |
| ATOM | 9234 | CD | LYS | F | 115 | 1.926 | 37.827 | 64.947 | 1.00 | 33.30 | C |
| ATOM | 9235 | CE | LYS | F | 115 | 0.747 | 38.161 | 63.992 | 1.00 | 34.89 | C |
| ATOM | 9236 | NZ | LYS | F | 115 | -0.067 | 36.944 | 63.490 | 1.00 | 33.86 | N |
| ATOM | 9237 | N | THR | F | 116 | 3.845 | 42.299 | 63.371 | 1.00 | 20.68 | N |
| ATOM | 9238 | CA | THR | F | 116 | 4.423 | 43.618 | 63.179 | 1.00 | 19.18 | C |
| ATOM | 9239 | C | THR | F | 116 | 5.153 | 44.064 | 64.439 | 1.00 | 19.54 | C |
| ATOM | 9240 | O | THR | F | 116 | 4.634 | 43.916 | 65.543 | 1.00 | 19.35 | O |
| ATOM | 9241 | CB | THR | F | 116 | 3.323 | 44.635 | 62.786 | 1.00 | 19.51 | C |
| ATOM | 9242 | OG1 | THR | F | 116 | 2.882 | 44.349 | 61.459 | 1.00 | 18.29 | O |
| ATOM | 9243 | CG2 | THR | F | 116 | 3.814 | 46.041 | 62.836 | 1.00 | 19.09 | C |
| ATOM | 9244 | N | THR | F | 117 | 6.357 | 44.616 | 64.272 | 1.00 | 18.17 | N |
| ATOM | 9245 | CA | THR | F | 117 | 7.153 | 45.110 | 65.398 | 1.00 | 18.20 | C |
| ATOM | 9246 | C | THR | F | 117 | 7.792 | 46.418 | 64.943 | 1.00 | 17.71 | C |
| ATOM | 9247 | O | THR | F | 117 | 8.389 | 46.441 | 63.862 | 1.00 | 18.36 | O |
| ATOM | 9248 | CB | THR | F | 117 | 8.269 | 44.140 | 65.751 | 1.00 | 18.83 | C |
| ATOM | 9249 | OG1 | THR | F | 117 | 7.727 | 42.828 | 66.045 | 1.00 | 20.00 | O |
| ATOM | 9250 | CG2 | THR | F | 117 | 9.087 | 44.686 | 66.935 | 1.00 | 18.60 | C |
| ATOM | 9251 | N | PRO | F | 118 | 7.667 | 47.495 | 65.731 | 1.00 | 17.48 | N |
| ATOM | 9252 | CA | PRO | F | 118 | 8.307 | 48.749 | 65.299 | 1.00 | 17.84 | C |
| ATOM | 9253 | C | PRO | F | 118 | 9.829 | 48.749 | 65.518 | 1.00 | 18.72 | C |
| ATOM | 9254 | O | PRO | F | 118 | 10.302 | 48.081 | 66.406 | 1.00 | 19.21 | O |
| ATOM | 9255 | CB | PRO | F | 118 | 7.663 | 49.772 | 66.226 | 1.00 | 17.56 | C |
| ATOM | 9256 | CG | PRO | F | 118 | 7.452 | 48.988 | 67.489 | 1.00 | 16.71 | C |
| ATOM | 9257 | CD | PRO | F | 118 | 6.950 | 47.690 | 67.008 | 1.00 | 17.41 | C |
| ATOM | 9258 | N | PRO | F | 119 | 10.581 | 49.538 | 64.737 | 1.00 | 18.87 | N |
| ATOM | 9259 | CA | PRO | F | 119 | 12.049 | 49.601 | 64.925 | 1.00 | 18.84 | C |
| ATOM | 9260 | C | PRO | F | 119 | 12.531 | 50.245 | 66.224 | 1.00 | 18.08 | C |
| ATOM | 9261 | O | PRO | F | 119 | 11.872 | 51.099 | 66.813 | 1.00 | 18.17 | O |
| ATOM | 9262 | CB | PRO | F | 119 | 12.533 | 50.422 | 63.733 | 1.00 | 18.91 | C |
| ATOM | 9263 | CG | PRO | F | 119 | 11.370 | 51.171 | 63.246 | 1.00 | 20.44 | C |
| ATOM | 9264 | CD | PRO | F | 119 | 10.115 | 50.426 | 63.664 | 1.00 | 19.51 | C |
| ATOM | 9265 | N | SER | F | 120 | 13.703 | 49.798 | 66.649 | 1.00 | 17.22 | N |
| ATOM | 9266 | CA | SER | F | 120 | 14.480 | 50.421 | 67.688 | 1.00 | 17.09 | C |
| ATOM | 9267 | C | SER | F | 120 | 15.574 | 51.168 | 66.934 | 1.00 | 16.98 | C |
| ATOM | 9268 | O | SER | F | 120 | 16.278 | 50.580 | 66.154 | 1.00 | 19.47 | O |
| ATOM | 9269 | CB | SER | F | 120 | 15.085 | 49.369 | 68.623 | 1.00 | 16.07 | C |
| ATOM | 9270 | OG | SER | F | 120 | 14.058 | 48.669 | 69.331 | 1.00 | 17.75 | O |
| ATOM | 9271 | N | VAL | F | 121 | 15.691 | 52.458 | 67.179 | 1.00 | 16.21 | N |
| ATOM | 9272 | CA | VAL | F | 121 | 16.668 | 53.318 | 66.479 | 1.00 | 16.42 | C |
| ATOM | 9273 | C | VAL | F | 121 | 17.795 | 53.770 | 67.411 | 1.00 | 14.73 | C |
| ATOM | 9274 | O | VAL | F | 121 | 17.561 | 54.409 | 68.430 | 1.00 | 15.55 | O |
| ATOM | 9275 | CB | VAL | F | 121 | 15.978 | 54.538 | 65.891 | 1.00 | 17.22 | C |
| ATOM | 9276 | CG1 | VAL | F | 121 | 16.947 | 55.312 | 65.022 | 1.00 | 17.74 | C |
| ATOM | 9277 | CG2 | VAL | F | 121 | 14.764 | 54.062 | 65.063 | 1.00 | 18.65 | C |
| ATOM | 9278 | N | TYR | F | 122 | 19.012 | 53.357 | 67.070 | 1.00 | 15.03 | N |
| ATOM | 9279 | CA | TYR | F | 122 | 20.216 | 53.621 | 67.876 | 1.00 | 14.72 | C |
| ATOM | 9280 | C | TYR | F | 122 | 21.173 | 54.586 | 67.151 | 1.00 | 15.01 | C |
| ATOM | 9281 | O | TYR | F | 122 | 21.409 | 54.438 | 65.943 | 1.00 | 13.95 | O |
| ATOM | 9282 | CB | TYR | F | 122 | 20.946 | 52.328 | 68.129 | 1.00 | 14.79 | C |
| ATOM | 9283 | CG | TYR | F | 122 | 20.096 | 51.278 | 68.814 | 1.00 | 13.81 | C |

| ATOM | 9284 | CD1 | TYR | F | 122 | 19.488 | 51.536 | 70.025 | 1.00 | 14.19 | C |
|------|------|-----|-----|---|-----|--------|--------|--------|------|-------|---|
| ATOM | 9285 | CD2 | TYR | F | 122 | 19.938 | 50.020 | 68.249 | 1.00 | 16.31 | C |
| ATOM | 9286 | CE1 | TYR | F | 122 | 18.724 | 50.575 | 70.657 | 1.00 | 15.40 | C |
| ATOM | 9287 | CE2 | TYR | F | 122 | 19.193 | 49.048 | 68.881 | 1.00 | 16.27 | C |
| ATOM | 9288 | CZ | TYR | F | 122 | 18.593 | 49.330 | 70.068 | 1.00 | 15.20 | C |
| ATOM | 9289 | OH | TYR | F | 122 | 17.844 | 48.363 | 70.700 | 1.00 | 17.12 | O |
| ATOM | 9290 | N | PRO | F | 123 | 21.734 | 55.558 | 67.886 | 1.00 | 16.93 | N |
| ATOM | 9291 | CA | PRO | F | 123 | 22.678 | 56.455 | 67.231 | 1.00 | 18.77 | C |
| ATOM | 9292 | C | PRO | F | 123 | 24.032 | 55.791 | 66.974 | 1.00 | 20.53 | C |
| ATOM | 9293 | O | PRO | F | 123 | 24.486 | 54.954 | 67.784 | 1.00 | 21.95 | O |
| ATOM | 9294 | CB | PRO | F | 123 | 22.809 | 57.618 | 68.227 | 1.00 | 18.51 | C |
| ATOM | 9295 | CG | PRO | F | 123 | 22.518 | 57.003 | 69.580 | 1.00 | 19.09 | C |
| ATOM | 9296 | CD | PRO | F | 123 | 21.526 | 55.899 | 69.304 | 1.00 | 18.18 | C |
| ATOM | 9297 | N | LEU | F | 124 | 24.650 | 56.134 | 65.842 | 1.00 | 20.59 | N |
| ATOM | 9298 | CA | LEU | F | 124 | 26.015 | 55.698 | 65.530 | 1.00 | 21.84 | C |
| ATOM | 9299 | C | LEU | F | 124 | 26.886 | 56.937 | 65.556 | 1.00 | 23.26 | C |
| ATOM | 9300 | O | LEU | F | 124 | 26.816 | 57.775 | 64.660 | 1.00 | 21.57 | O |
| ATOM | 9301 | CB | LEU | F | 124 | 26.081 | 54.999 | 64.180 | 1.00 | 21.88 | C |
| ATOM | 9302 | CG | LEU | F | 124 | 25.191 | 53.749 | 64.072 | 1.00 | 21.38 | C |
| ATOM | 9303 | CD1 | LEU | F | 124 | 25.239 | 53.202 | 62.692 | 1.00 | 19.42 | C |
| ATOM | 9304 | CD2 | LEU | F | 124 | 25.592 | 52.670 | 65.107 | 1.00 | 21.96 | C |
| ATOM | 9305 | N | ALA | F | 125 | 27.665 | 57.063 | 66.627 | 1.00 | 24.21 | N |
| ATOM | 9306 | CA | ALA | F | 125 | 28.571 | 58.199 | 66.816 | 1.00 | 24.64 | C |
| ATOM | 9307 | C | ALA | F | 125 | 30.005 | 57.692 | 67.013 | 1.00 | 25.89 | C |
| ATOM | 9308 | O | ALA | F | 125 | 30.221 | 56.626 | 67.599 | 1.00 | 25.33 | O |
| ATOM | 9309 | CB | ALA | F | 125 | 28.119 | 59.016 | 68.009 | 1.00 | 24.20 | C |
| ATOM | 9310 | N | PRO | F | 126 | 31.006 | 58.457 | 66.546 | 1.00 | 27.63 | N |
| ATOM | 9311 | CA | PRO | F | 126 | 32.378 | 57.974 | 66.648 | 1.00 | 28.71 | C |
| ATOM | 9312 | C | PRO | F | 126 | 32.795 | 57.742 | 68.100 | 1.00 | 29.78 | C |
| ATOM | 9313 | O | PRO | F | 126 | 32.393 | 58.510 | 68.971 | 1.00 | 30.70 | O |
| ATOM | 9314 | CB | PRO | F | 126 | 33.196 | 59.134 | 66.061 | 1.00 | 29.09 | C |
| ATOM | 9315 | CG | PRO | F | 126 | 32.244 | 59.917 | 65.261 | 1.00 | 28.72 | C |
| ATOM | 9316 | CD | PRO | F | 126 | 30.938 | 59.803 | 65.941 | 1.00 | 28.39 | C |
| ATOM | 9317 | N | GLY | F | 127 | 33.581 | 56.698 | 68.351 | 1.00 | 31.13 | N |
| ATOM | 9318 | CA | GLY | F | 127 | 34.177 | 56.482 | 69.668 | 1.00 | 32.30 | C |
| ATOM | 9319 | C | GLY | F | 127 | 35.126 | 57.601 | 70.090 | 1.00 | 33.74 | C |
| ATOM | 9320 | O | GLY | F | 127 | 35.810 | 58.200 | 69.241 | 1.00 | 35.62 | O |
| ATOM | 9321 | N | SER | F | 136 | 38.508 | 66.548 | 58.483 | 1.00 | 42.56 | N |
| ATOM | 9322 | CA | SER | F | 136 | 37.758 | 67.232 | 57.423 | 1.00 | 42.56 | C |
| ATOM | 9323 | C | SER | F | 136 | 36.266 | 66.857 | 57.453 | 1.00 | 42.05 | C |
| ATOM | 9324 | O | SER | F | 136 | 35.388 | 67.730 | 57.551 | 1.00 | 42.06 | O |
| ATOM | 9325 | CB | SER | F | 136 | 38.370 | 66.889 | 56.058 | 1.00 | 42.70 | C |
| ATOM | 9326 | OG | SER | F | 136 | 37.552 | 67.318 | 54.977 | 1.00 | 44.50 | O |
| ATOM | 9327 | N | MET | F | 137 | 36.000 | 65.553 | 57.375 | 1.00 | 40.80 | N |
| ATOM | 9328 | CA | MET | F | 137 | 34.637 | 65.021 | 57.363 | 1.00 | 39.35 | C |
| ATOM | 9329 | C | MET | F | 137 | 34.427 | 64.125 | 58.573 | 1.00 | 36.61 | C |
| ATOM | 9330 | O | MET | F | 137 | 35.389 | 63.713 | 59.227 | 1.00 | 36.15 | O |
| ATOM | 9331 | CB | MET | F | 137 | 34.385 | 64.217 | 56.082 | 1.00 | 40.56 | C |
| ATOM | 9332 | CG | MET | F | 137 | 34.475 | 65.040 | 54.804 | 1.00 | 42.56 | C |
| ATOM | 9333 | SD | MET | F | 137 | 33.367 | 66.465 | 54.859 | 1.00 | 46.86 | S |
| ATOM | 9334 | CE | MET | F | 137 | 31.999 | 65.898 | 53.841 | 1.00 | 46.23 | C |
| ATOM | 9335 | N | VAL | F | 138 | 33.164 | 63.847 | 58.871 | 1.00 | 33.12 | N |
| ATOM | 9336 | CA | VAL | F | 138 | 32.804 | 62.885 | 59.908 | 1.00 | 31.37 | C |
| ATOM | 9337 | C | VAL | F | 138 | 31.603 | 62.085 | 59.424 | 1.00 | 29.28 | C |
| ATOM | 9338 | O | VAL | F | 138 | 30.675 | 62.634 | 58.813 | 1.00 | 28.01 | O |
| ATOM | 9339 | CB | VAL | F | 138 | 32.516 | 63.557 | 61.263 | 1.00 | 30.99 | C |
| ATOM | 9340 | CG1 | VAL | F | 138 | 31.340 | 64.512 | 61.170 | 1.00 | 31.71 | C |
| ATOM | 9341 | CG2 | VAL | F | 138 | 32.278 | 62.502 | 62.363 | 1.00 | 32.17 | C |
| ATOM | 9342 | N | THR | F | 139 | 31.667 | 60.782 | 59.661 | 1.00 | 26.96 | N |
| ATOM | 9343 | CA | THR | F | 139 | 30.603 | 59.865 | 59.294 | 1.00 | 25.26 | C |
| ATOM | 9344 | C | THR | F | 139 | 29.851 | 59.469 | 60.576 | 1.00 | 24.45 | C |
| ATOM | 9345 | O | THR | F | 139 | 30.437 | 59.016 | 61.557 | 1.00 | 22.88 | O |
| ATOM | 9346 | CB | THR | F | 139 | 31.159 | 58.648 | 58.551 | 1.00 | 26.06 | C |
| ATOM | 9347 | OG1 | THR | F | 139 | 31.715 | 59.071 | 57.293 | 1.00 | 24.89 | O |
| ATOM | 9348 | CG2 | THR | F | 139 | 30.060 | 57.607 | 58.283 | 1.00 | 24.38 | C |

| ATOM | 9349 | N   | LEU | F | 140 | 28.549 | 59.711 | 60.554 | 1.00 | 23.75 | N |
| ATOM | 9350 | CA  | LEU | F | 140 | 27.648 | 59.329 | 61.624 | 1.00 | 22.46 | C |
| ATOM | 9351 | C   | LEU | F | 140 | 26.645 | 58.371 | 61.028 | 1.00 | 20.88 | C |
| ATOM | 9352 | O   | LEU | F | 140 | 26.679 | 58.079 | 59.830 | 1.00 | 20.09 | O |
| ATOM | 9353 | CB  | LEU | F | 140 | 26.921 | 60.542 | 62.171 | 1.00 | 23.77 | C |
| ATOM | 9354 | CG  | LEU | F | 140 | 27.746 | 61.748 | 62.621 | 1.00 | 25.00 | C |
| ATOM | 9355 | CD1 | LEU | F | 140 | 26.817 | 62.906 | 62.929 | 1.00 | 24.99 | C |
| ATOM | 9356 | CD2 | LEU | F | 140 | 28.576 | 61.405 | 63.832 | 1.00 | 24.59 | C |
| ATOM | 9357 | N   | GLY | F | 141 | 25.745 | 57.868 | 61.856 | 1.00 | 19.57 | N |
| ATOM | 9358 | CA  | GLY | F | 141 | 24.731 | 56.995 | 61.320 | 1.00 | 18.93 | C |
| ATOM | 9359 | C   | GLY | F | 141 | 23.644 | 56.676 | 62.303 | 1.00 | 18.07 | C |
| ATOM | 9360 | O   | GLY | F | 141 | 23.694 | 57.096 | 63.442 | 1.00 | 15.63 | O |
| ATOM | 9361 | N   | CYS | F | 142 | 22.640 | 55.967 | 61.799 | 1.00 | 19.81 | N |
| ATOM | 9362 | CA  | CYS | F | 142 | 21.584 | 55.421 | 62.619 | 1.00 | 21.28 | C |
| ATOM | 9363 | C   | CYS | F | 142 | 21.490 | 53.950 | 62.347 | 1.00 | 18.81 | C |
| ATOM | 9364 | O   | CYS | F | 142 | 21.592 | 53.529 | 61.208 | 1.00 | 19.20 | O |
| ATOM | 9365 | CB  | CYS | F | 142 | 20.264 | 56.103 | 62.316 | 1.00 | 25.34 | C |
| ATOM | 9366 | SG  | CYS | F | 142 | 20.045 | 57.449 | 63.431 | 1.00 | 34.16 | S |
| ATOM | 9367 | N   | LEU | F | 143 | 21.325 | 53.161 | 63.400 | 1.00 | 16.99 | N |
| ATOM | 9368 | CA  | LEU | F | 143 | 21.089 | 51.750 | 63.264 | 1.00 | 16.76 | C |
| ATOM | 9369 | C   | LEU | F | 143 | 19.611 | 51.469 | 63.584 | 1.00 | 16.75 | C |
| ATOM | 9370 | O   | LEU | F | 143 | 19.122 | 51.849 | 64.645 | 1.00 | 17.50 | O |
| ATOM | 9371 | CB  | LEU | F | 143 | 21.987 | 50.964 | 64.225 | 1.00 | 17.36 | C |
| ATOM | 9372 | CG  | LEU | F | 143 | 21.770 | 49.446 | 64.281 | 1.00 | 17.21 | C |
| ATOM | 9373 | CD1 | LEU | F | 143 | 22.197 | 48.794 | 62.997 | 1.00 | 19.53 | C |
| ATOM | 9374 | CD2 | LEU | F | 143 | 22.544 | 48.845 | 65.449 | 1.00 | 19.75 | C |
| ATOM | 9375 | N   | VAL | F | 144 | 18.938 | 50.767 | 62.678 | 1.00 | 17.17 | N |
| ATOM | 9376 | CA  | VAL | F | 144 | 17.477 | 50.617 | 62.709 | 1.00 | 18.61 | C |
| ATOM | 9377 | C   | VAL | F | 144 | 17.196 | 49.141 | 62.832 | 1.00 | 18.34 | C |
| ATOM | 9378 | O   | VAL | F | 144 | 17.375 | 48.370 | 61.889 | 1.00 | 19.13 | O |
| ATOM | 9379 | CB  | VAL | F | 144 | 16.795 | 51.224 | 61.452 | 1.00 | 19.32 | C |
| ATOM | 9380 | CG1 | VAL | F | 144 | 15.278 | 51.101 | 61.552 | 1.00 | 18.02 | C |
| ATOM | 9381 | CG2 | VAL | F | 144 | 17.187 | 52.673 | 61.291 | 1.00 | 19.29 | C |
| ATOM | 9382 | N   | LYS | F | 145 | 16.815 | 48.725 | 64.028 | 1.00 | 18.52 | N |
| ATOM | 9383 | CA  | LYS | F | 145 | 16.861 | 47.318 | 64.373 | 1.00 | 18.52 | C |
| ATOM | 9384 | C   | LYS | F | 145 | 15.543 | 46.753 | 64.888 | 1.00 | 17.88 | C |
| ATOM | 9385 | O   | LYS | F | 145 | 14.798 | 47.427 | 65.604 | 1.00 | 18.92 | O |
| ATOM | 9386 | CB  | LYS | F | 145 | 17.923 | 47.140 | 65.462 | 1.00 | 19.25 | C |
| ATOM | 9387 | CG  | LYS | F | 145 | 18.284 | 45.707 | 65.739 | 1.00 | 21.23 | C |
| ATOM | 9388 | CD  | LYS | F | 145 | 19.657 | 45.594 | 66.365 | 1.00 | 23.01 | C |
| ATOM | 9389 | CE  | LYS | F | 145 | 20.124 | 44.166 | 66.363 | 1.00 | 25.87 | C |
| ATOM | 9390 | NZ  | LYS | F | 145 | 20.320 | 43.587 | 64.988 | 1.00 | 28.74 | N |
| ATOM | 9391 | N   | GLY | F | 146 | 15.297 | 45.489 | 64.568 | 1.00 | 18.54 | N |
| ATOM | 9392 | CA  | GLY | F | 146 | 14.262 | 44.731 | 65.229 | 1.00 | 19.99 | C |
| ATOM | 9393 | C   | GLY | F | 146 | 12.854 | 45.023 | 64.769 | 1.00 | 20.65 | C |
| ATOM | 9394 | O   | GLY | F | 146 | 11.922 | 44.952 | 65.566 | 1.00 | 22.06 | O |
| ATOM | 9395 | N   | TYR | F | 147 | 12.691 | 45.328 | 63.486 | 1.00 | 21.07 | N |
| ATOM | 9396 | CA  | TYR | F | 147 | 11.362 | 45.642 | 62.951 | 1.00 | 20.55 | C |
| ATOM | 9397 | C   | TYR | F | 147 | 10.855 | 44.610 | 61.932 | 1.00 | 20.46 | C |
| ATOM | 9398 | O   | TYR | F | 147 | 11.618 | 43.814 | 61.342 | 1.00 | 21.78 | O |
| ATOM | 9399 | CB  | TYR | F | 147 | 11.323 | 47.039 | 62.356 | 1.00 | 20.84 | C |
| ATOM | 9400 | CG  | TYR | F | 147 | 12.107 | 47.185 | 61.078 | 1.00 | 20.69 | C |
| ATOM | 9401 | CD1 | TYR | F | 147 | 13.478 | 47.469 | 61.111 | 1.00 | 20.68 | C |
| ATOM | 9402 | CD2 | TYR | F | 147 | 11.488 | 47.072 | 59.837 | 1.00 | 19.78 | C |
| ATOM | 9403 | CE1 | TYR | F | 147 | 14.216 | 47.615 | 59.948 | 1.00 | 19.11 | C |
| ATOM | 9404 | CE2 | TYR | F | 147 | 12.224 | 47.224 | 58.652 | 1.00 | 20.86 | C |
| ATOM | 9405 | CZ  | TYR | F | 147 | 13.590 | 47.501 | 58.715 | 1.00 | 22.34 | C |
| ATOM | 9406 | OH  | TYR | F | 147 | 14.343 | 47.639 | 57.551 | 1.00 | 23.39 | O |
| ATOM | 9407 | N   | PHE | F | 148 | 9.534 | 44.633 | 61.754 | 1.00 | 21.11 | N |
| ATOM | 9408 | CA  | PHE | F | 148 | 8.860 | 43.799 | 60.767 | 1.00 | 20.55 | C |
| ATOM | 9409 | C   | PHE | F | 148 | 7.464 | 44.352 | 60.501 | 1.00 | 19.88 | C |
| ATOM | 9410 | O   | PHE | F | 148 | 6.815 | 44.869 | 61.416 | 1.00 | 19.91 | O |
| ATOM | 9411 | CB  | PHE | F | 148 | 8.776 | 42.348 | 61.235 | 1.00 | 20.75 | C |
| ATOM | 9412 | CG  | PHE | F | 148 | 8.267 | 41.424 | 60.186 | 1.00 | 20.81 | C |
| ATOM | 9413 | CD1 | PHE | F | 148 | 9.133 | 40.863 | 59.260 | 1.00 | 22.09 | C |

| ATOM | 9414 | CD2 | PHE | F | 148 | 6.897 | 41.153 | 60.086 | 1.00 | 21.54 | C |
|------|------|-----|-----|---|-----|-------|--------|--------|------|-------|---|
| ATOM | 9415 | CE1 | PHE | F | 148 | 8.665 | 40.020 | 58.267 | 1.00 | 22.24 | C |
| ATOM | 9416 | CE2 | PHE | F | 148 | 6.416 | 40.309 | 59.107 | 1.00 | 20.28 | C |
| ATOM | 9417 | CZ | PHE | F | 148 | 7.289 | 39.743 | 58.195 | 1.00 | 21.73 | C |
| ATOM | 9418 | N | PRO | F | 149 | 7.011 | 44.318 | 59.235 | 1.00 | 19.38 | N |
| ATOM | 9419 | CA | PRO | F | 149 | 7.704 | 44.031 | 57.987 | 1.00 | 19.75 | C |
| ATOM | 9420 | C | PRO | F | 149 | 8.478 | 45.227 | 57.421 | 1.00 | 21.03 | C |
| ATOM | 9421 | O | PRO | F | 149 | 8.383 | 46.346 | 57.906 | 1.00 | 20.76 | O |
| ATOM | 9422 | CB | PRO | F | 149 | 6.544 | 43.696 | 57.038 | 1.00 | 19.27 | C |
| ATOM | 9423 | CG | PRO | F | 149 | 5.468 | 44.523 | 57.477 | 1.00 | 17.79 | C |
| ATOM | 9424 | CD | PRO | F | 149 | 5.584 | 44.565 | 58.997 | 1.00 | 19.47 | C |
| ATOM | 9425 | N | GLU | F | 150 | 9.202 | 44.972 | 56.346 | 1.00 | 23.30 | N |
| ATOM | 9426 | CA | GLU | F | 150 | 9.666 | 46.023 | 55.469 | 1.00 | 24.86 | C |
| ATOM | 9427 | C | GLU | F | 150 | 8.451 | 46.706 | 54.870 | 1.00 | 25.98 | C |
| ATOM | 9428 | O | GLU | F | 150 | 7.397 | 46.075 | 54.746 | 1.00 | 26.21 | O |
| ATOM | 9429 | CB | GLU | F | 150 | 10.518 | 45.395 | 54.362 | 1.00 | 25.47 | C |
| ATOM | 9430 | CG | GLU | F | 150 | 11.953 | 45.245 | 54.761 | 1.00 | 28.05 | C |
| ATOM | 9431 | CD | GLU | F | 150 | 12.753 | 46.453 | 54.382 | 1.00 | 28.59 | C |
| ATOM | 9432 | OE1 | GLU | F | 150 | 13.218 | 46.459 | 53.227 | 1.00 | 32.51 | O |
| ATOM | 9433 | OE2 | GLU | F | 150 | 12.886 | 47.402 | 55.200 | 1.00 | 30.21 | O |
| ATOM | 9434 | N | PRO | F | 151 | 8.573 | 47.991 | 54.498 | 1.00 | 26.55 | N |
| ATOM | 9435 | CA | PRO | F | 151 | 9.719 | 48.878 | 54.559 | 1.00 | 25.92 | C |
| ATOM | 9436 | C | PRO | F | 151 | 9.784 | 49.873 | 55.726 | 1.00 | 25.66 | C |
| ATOM | 9437 | O | PRO | F | 151 | 8.818 | 50.095 | 56.474 | 1.00 | 23.93 | O |
| ATOM | 9438 | CB | PRO | F | 151 | 9.567 | 49.674 | 53.260 | 1.00 | 26.96 | C |
| ATOM | 9439 | CG | PRO | F | 151 | 8.061 | 49.846 | 53.146 | 1.00 | 27.94 | C |
| ATOM | 9440 | CD | PRO | F | 151 | 7.428 | 48.663 | 53.852 | 1.00 | 28.52 | C |
| ATOM | 9441 | N | VAL | F | 152 | 10.959 | 50.476 | 55.862 | 1.00 | 25.07 | N |
| ATOM | 9442 | CA | VAL | F | 152 | 11.108 | 51.732 | 56.593 | 1.00 | 25.29 | C |
| ATOM | 9443 | C | VAL | F | 152 | 11.690 | 52.772 | 55.649 | 1.00 | 25.88 | C |
| ATOM | 9444 | O | VAL | F | 152 | 12.350 | 52.428 | 54.657 | 1.00 | 26.52 | O |
| ATOM | 9445 | CB | VAL | F | 152 | 12.053 | 51.640 | 57.826 | 1.00 | 25.50 | C |
| ATOM | 9446 | CG1 | VAL | F | 152 | 11.456 | 50.734 | 58.922 | 1.00 | 24.60 | C |
| ATOM | 9447 | CG2 | VAL | F | 152 | 13.459 | 51.175 | 57.405 | 1.00 | 25.64 | C |
| ATOM | 9448 | N | THR | F | 153 | 11.462 | 54.026 | 55.998 | 1.00 | 25.44 | N |
| ATOM | 9449 | CA | THR | F | 153 | 11.998 | 55.179 | 55.284 | 1.00 | 27.05 | C |
| ATOM | 9450 | C | THR | F | 153 | 12.870 | 55.967 | 56.247 | 1.00 | 27.00 | C |
| ATOM | 9451 | O | THR | F | 153 | 12.382 | 56.483 | 57.254 | 1.00 | 26.86 | O |
| ATOM | 9452 | CB | THR | F | 153 | 10.842 | 56.078 | 54.758 | 1.00 | 27.12 | C |
| ATOM | 9453 | OG1 | THR | F | 153 | 10.160 | 55.383 | 53.701 | 1.00 | 29.33 | O |
| ATOM | 9454 | CG2 | THR | F | 153 | 11.365 | 57.423 | 54.249 | 1.00 | 28.63 | C |
| ATOM | 9455 | N | VAL | F | 154 | 14.161 | 56.055 | 55.938 | 1.00 | 26.91 | N |
| ATOM | 9456 | CA | VAL | F | 154 | 15.076 | 56.841 | 56.761 | 1.00 | 27.05 | C |
| ATOM | 9457 | C | VAL | F | 154 | 15.401 | 58.157 | 56.076 | 1.00 | 27.88 | C |
| ATOM | 9458 | O | VAL | F | 154 | 15.747 | 58.174 | 54.882 | 1.00 | 28.47 | O |
| ATOM | 9459 | CB | VAL | F | 154 | 16.405 | 56.126 | 57.012 | 1.00 | 27.40 | C |
| ATOM | 9460 | CG1 | VAL | F | 154 | 17.229 | 56.915 | 58.023 | 1.00 | 27.23 | C |
| ATOM | 9461 | CG2 | VAL | F | 154 | 16.163 | 54.698 | 57.478 | 1.00 | 27.20 | C |
| ATOM | 9462 | N | THR | F | 155 | 15.281 | 59.239 | 56.831 | 1.00 | 27.08 | N |
| ATOM | 9463 | CA | THR | F | 155 | 15.767 | 60.535 | 56.391 | 1.00 | 28.19 | C |
| ATOM | 9464 | C | THR | F | 155 | 16.661 | 61.116 | 57.469 | 1.00 | 28.40 | C |
| ATOM | 9465 | O | THR | F | 155 | 16.746 | 60.578 | 58.573 | 1.00 | 27.24 | O |
| ATOM | 9466 | CB | THR | F | 155 | 14.607 | 61.526 | 56.075 | 1.00 | 28.64 | C |
| ATOM | 9467 | OG1 | THR | F | 155 | 13.744 | 61.669 | 57.210 | 1.00 | 30.88 | O |
| ATOM | 9468 | CG2 | THR | F | 155 | 13.792 | 61.042 | 54.896 | 1.00 | 29.33 | C |
| ATOM | 9469 | N | TRP | F | 156 | 17.328 | 62.219 | 57.138 | 1.00 | 27.73 | N |
| ATOM | 9470 | CA | TRP | F | 156 | 18.186 | 62.928 | 58.073 | 1.00 | 28.30 | C |
| ATOM | 9471 | C | TRP | F | 156 | 17.800 | 64.389 | 58.119 | 1.00 | 30.23 | C |
| ATOM | 9472 | O | TRP | F | 156 | 17.584 | 65.022 | 57.072 | 1.00 | 30.73 | O |
| ATOM | 9473 | CB | TRP | F | 156 | 19.631 | 62.793 | 57.652 | 1.00 | 26.59 | C |
| ATOM | 9474 | CG | TRP | F | 156 | 20.158 | 61.410 | 57.798 | 1.00 | 25.59 | C |
| ATOM | 9475 | CD1 | TRP | F | 156 | 20.141 | 60.424 | 56.868 | 1.00 | 25.56 | C |
| ATOM | 9476 | CD2 | TRP | F | 156 | 20.804 | 60.868 | 58.953 | 1.00 | 24.44 | C |
| ATOM | 9477 | NE1 | TRP | F | 156 | 20.741 | 59.281 | 57.372 | 1.00 | 25.23 | N |
| ATOM | 9478 | CE2 | TRP | F | 156 | 21.151 | 59.537 | 58.654 | 1.00 | 24.86 | C |

404

```
ATOM   9479  CE3 TRP F 156     21.129  61.387  60.212  1.00 24.56           C
ATOM   9480  CZ2 TRP F 156     21.809  58.715  59.572  1.00 24.89           C
ATOM   9481  CZ3 TRP F 156     21.775  60.571  61.115  1.00 24.10           C
ATOM   9482  CH2 TRP F 156     22.116  59.255  60.785  1.00 24.87           C
ATOM   9483  N   ASN F 157     17.698  64.923  59.327  1.00 32.32           N
ATOM   9484  CA  ASN F 157     17.187  66.277  59.549  1.00 34.85           C
ATOM   9485  C   ASN F 157     15.908  66.577  58.758  1.00 36.34           C
ATOM   9486  O   ASN F 157     15.742  67.666  58.192  1.00 36.99           O
ATOM   9487  CB  ASN F 157     18.284  67.311  59.263  1.00 35.22           C
ATOM   9488  CG  ASN F 157     19.443  67.201  60.232  1.00 35.39           C
ATOM   9489  OD1 ASN F 157     19.305  66.612  61.303  1.00 34.81           O
ATOM   9490  ND2 ASN F 157     20.588  67.773  59.870  1.00 34.55           N
ATOM   9491  N   SER F 158     15.001  65.600  58.756  1.00 37.21           N
ATOM   9492  CA  SER F 158     13.705  65.703  58.081  1.00 37.84           C
ATOM   9493  C   SER F 158     13.810  66.001  56.574  1.00 38.55           C
ATOM   9494  O   SER F 158     12.999  66.747  56.022  1.00 39.85           O
ATOM   9495  CB  SER F 158     12.827  66.744  58.785  1.00 37.68           C
ATOM   9496  OG  SER F 158     12.680  66.449  60.168  1.00 38.10           O
ATOM   9497  N   GLY F 159     14.787  65.387  55.913  1.00 39.08           N
ATOM   9498  CA  GLY F 159     14.972  65.523  54.466  1.00 39.90           C
ATOM   9499  C   GLY F 159     15.987  66.586  54.084  1.00 40.52           C
ATOM   9500  O   GLY F 159     16.508  66.569  52.969  1.00 40.51           O
ATOM   9501  N   SER F 160     16.259  67.500  55.018  1.00 41.48           N
ATOM   9502  CA  SER F 160     17.245  68.578  54.856  1.00 42.59           C
ATOM   9503  C   SER F 160     18.673  68.119  54.584  1.00 43.42           C
ATOM   9504  O   SER F 160     19.415  68.813  53.884  1.00 43.33           O
ATOM   9505  CB  SER F 160     17.273  69.437  56.115  1.00 42.86           C
ATOM   9506  OG  SER F 160     15.987  69.941  56.408  1.00 43.96           O
ATOM   9507  N   LEU F 161     19.070  66.993  55.182  1.00 43.85           N
ATOM   9508  CA  LEU F 161     20.375  66.379  54.925  1.00 44.22           C
ATOM   9509  C   LEU F 161     20.192  65.249  53.938  1.00 45.05           C
ATOM   9510  O   LEU F 161     19.719  64.162  54.301  1.00 44.89           O
ATOM   9511  CB  LEU F 161     21.006  65.819  56.209  1.00 44.46           C
ATOM   9512  CG  LEU F 161     21.928  66.709  57.039  1.00 44.48           C
ATOM   9513  CD1 LEU F 161     22.420  65.934  58.252  1.00 44.21           C
ATOM   9514  CD2 LEU F 161     23.112  67.214  56.213  1.00 44.45           C
ATOM   9515  N   SER F 162     20.586  65.503  52.694  1.00 45.55           N
ATOM   9516  CA  SER F 162     20.313  64.589  51.582  1.00 46.11           C
ATOM   9517  C   SER F 162     21.568  63.957  50.995  1.00 45.98           C
ATOM   9518  O   SER F 162     21.549  62.801  50.558  1.00 46.35           O
ATOM   9519  CB  SER F 162     19.555  65.349  50.485  1.00 46.62           C
ATOM   9520  OG  SER F 162     19.932  66.723  50.450  1.00 47.38           O
ATOM   9521  N   SER F 163     22.653  64.719  50.975  1.00 45.46           N
ATOM   9522  CA  SER F 163     23.887  64.283  50.347  1.00 45.87           C
ATOM   9523  C   SER F 163     24.846  63.744  51.400  1.00 44.69           C
ATOM   9524  O   SER F 163     24.783  64.134  52.566  1.00 45.39           O
ATOM   9525  CB  SER F 163     24.516  65.454  49.580  1.00 46.65           C
ATOM   9526  OG  SER F 163     23.640  65.901  48.546  1.00 48.01           O
ATOM   9527  N   GLY F 164     25.739  62.859  50.970  1.00 43.77           N
ATOM   9528  CA  GLY F 164     26.619  62.125  51.884  1.00 42.79           C
ATOM   9529  C   GLY F 164     25.908  60.972  52.586  1.00 41.64           C
ATOM   9530  O   GLY F 164     26.404  60.458  53.585  1.00 41.26           O
ATOM   9531  N   VAL F 165     24.766  60.550  52.040  1.00 40.14           N
ATOM   9532  CA  VAL F 165     23.872  59.596  52.699  1.00 38.91           C
ATOM   9533  C   VAL F 165     23.789  58.276  51.934  1.00 38.25           C
ATOM   9534  O   VAL F 165     23.453  58.270  50.760  1.00 38.17           O
ATOM   9535  CB  VAL F 165     22.444  60.188  52.846  1.00 38.66           C
ATOM   9536  CG1 VAL F 165     21.477  59.151  53.413  1.00 39.46           C
ATOM   9537  CG2 VAL F 165     22.455  61.412  53.735  1.00 37.64           C
ATOM   9538  N   HIS F 166     24.118  57.169  52.607  1.00 36.77           N
ATOM   9539  CA  HIS F 166     23.816  55.824  52.128  1.00 36.10           C
ATOM   9540  C   HIS F 166     22.822  55.240  53.114  1.00 33.74           C
ATOM   9541  O   HIS F 166     23.012  55.385  54.321  1.00 33.38           O
ATOM   9542  CB  HIS F 166     25.049  54.905  52.132  1.00 36.43           C
ATOM   9543  CG  HIS F 166     26.251  55.494  51.481  1.00 38.03           C
```

| ATOM | 9544 | ND1 | HIS | F | 166 | 26.444 | 55.468 | 50.118 | 1.00 | 39.79 | N |
|------|------|-----|-----|---|-----|--------|--------|--------|------|-------|---|
| ATOM | 9545 | CD2 | HIS | F | 166 | 27.336 | 56.105 | 52.006 | 1.00 | 38.08 | C |
| ATOM | 9546 | CE1 | HIS | F | 166 | 27.588 | 56.063 | 49.828 | 1.00 | 40.24 | C |
| ATOM | 9547 | NE2 | HIS | F | 166 | 28.150 | 56.455 | 50.957 | 1.00 | 40.54 | N |
| ATOM | 9548 | N | THR | F | 167 | 21.771 | 54.606 | 52.610 | 1.00 | 31.37 | N |
| ATOM | 9549 | CA | THR | F | 167 | 20.939 | 53.746 | 53.445 | 1.00 | 29.01 | C |
| ATOM | 9550 | C | THR | F | 167 | 21.045 | 52.370 | 52.869 | 1.00 | 28.60 | C |
| ATOM | 9551 | O | THR | F | 167 | 20.826 | 52.176 | 51.674 | 1.00 | 29.49 | O |
| ATOM | 9552 | CB | THR | F | 167 | 19.482 | 54.231 | 53.502 | 1.00 | 29.50 | C |
| ATOM | 9553 | OG1 | THR | F | 167 | 19.449 | 55.520 | 54.134 | 1.00 | 28.57 | O |
| ATOM | 9554 | CG2 | THR | F | 167 | 18.611 | 53.251 | 54.286 | 1.00 | 29.40 | C |
| ATOM | 9555 | N | PHE | F | 168 | 21.394 | 51.408 | 53.710 | 1.00 | 25.96 | N |
| ATOM | 9556 | CA | PHE | F | 168 | 21.704 | 50.081 | 53.240 | 1.00 | 26.41 | C |
| ATOM | 9557 | C | PHE | F | 168 | 20.403 | 49.293 | 53.165 | 1.00 | 26.09 | C |
| ATOM | 9558 | O | PHE | F | 168 | 19.468 | 49.612 | 53.894 | 1.00 | 25.10 | O |
| ATOM | 9559 | CB | PHE | F | 168 | 22.753 | 49.467 | 54.164 | 1.00 | 26.57 | C |
| ATOM | 9560 | CG | PHE | F | 168 | 24.050 | 50.244 | 54.165 | 1.00 | 27.05 | C |
| ATOM | 9561 | CD1 | PHE | F | 168 | 24.246 | 51.311 | 55.035 | 1.00 | 26.57 | C |
| ATOM | 9562 | CD2 | PHE | F | 168 | 25.035 | 49.961 | 53.237 | 1.00 | 26.09 | C |
| ATOM | 9563 | CE1 | PHE | F | 168 | 25.404 | 52.036 | 55.010 | 1.00 | 27.24 | C |
| ATOM | 9564 | CE2 | PHE | F | 168 | 26.201 | 50.703 | 53.210 | 1.00 | 27.21 | C |
| ATOM | 9565 | CZ | PHE | F | 168 | 26.377 | 51.739 | 54.092 | 1.00 | 26.88 | C |
| ATOM | 9566 | N | PRO | F | 169 | 20.316 | 48.314 | 52.245 | 1.00 | 26.78 | N |
| ATOM | 9567 | CA | PRO | F | 169 | 19.175 | 47.390 | 52.248 | 1.00 | 27.12 | C |
| ATOM | 9568 | C | PRO | F | 169 | 19.047 | 46.592 | 53.556 | 1.00 | 27.22 | C |
| ATOM | 9569 | O | PRO | F | 169 | 20.060 | 46.157 | 54.149 | 1.00 | 24.30 | O |
| ATOM | 9570 | CB | PRO | F | 169 | 19.476 | 46.430 | 51.087 | 1.00 | 26.99 | C |
| ATOM | 9571 | CG | PRO | F | 169 | 20.423 | 47.173 | 50.208 | 1.00 | 28.00 | C |
| ATOM | 9572 | CD | PRO | F | 169 | 21.235 | 48.044 | 51.119 | 1.00 | 26.06 | C |
| ATOM | 9573 | N | ALA | F | 170 | 17.805 | 46.396 | 53.991 | 1.00 | 27.00 | N |
| ATOM | 9574 | CA | ALA | F | 170 | 17.544 | 45.656 | 55.213 | 1.00 | 27.41 | C |
| ATOM | 9575 | C | ALA | F | 170 | 17.934 | 44.191 | 55.048 | 1.00 | 28.63 | C |
| ATOM | 9576 | O | ALA | F | 170 | 17.911 | 43.645 | 53.945 | 1.00 | 29.25 | O |
| ATOM | 9577 | CB | ALA | F | 170 | 16.080 | 45.789 | 55.611 | 1.00 | 27.01 | C |
| ATOM | 9578 | N | VAL | F | 171 | 18.353 | 43.574 | 56.141 | 1.00 | 29.04 | N |
| ATOM | 9579 | CA | VAL | F | 171 | 18.623 | 42.156 | 56.179 | 1.00 | 30.33 | C |
| ATOM | 9580 | C | VAL | F | 171 | 17.632 | 41.540 | 57.172 | 1.00 | 31.01 | C |
| ATOM | 9581 | O | VAL | F | 171 | 17.399 | 42.094 | 58.243 | 1.00 | 28.91 | O |
| ATOM | 9582 | CB | VAL | F | 171 | 20.080 | 41.887 | 56.606 | 1.00 | 31.43 | C |
| ATOM | 9583 | CG1 | VAL | F | 171 | 20.352 | 40.401 | 56.692 | 1.00 | 31.58 | C |
| ATOM | 9584 | CG2 | VAL | F | 171 | 21.039 | 42.541 | 55.621 | 1.00 | 31.79 | C |
| ATOM | 9585 | N | LEU | F | 172 | 17.041 | 40.406 | 56.803 | 1.00 | 32.60 | N |
| ATOM | 9586 | CA | LEU | F | 172 | 16.077 | 39.742 | 57.667 | 1.00 | 33.70 | C |
| ATOM | 9587 | C | LEU | F | 172 | 16.774 | 38.663 | 58.457 | 1.00 | 35.47 | C |
| ATOM | 9588 | O | LEU | F | 172 | 17.514 | 37.864 | 57.901 | 1.00 | 35.49 | O |
| ATOM | 9589 | CB | LEU | F | 172 | 14.939 | 39.125 | 56.842 | 1.00 | 33.78 | C |
| ATOM | 9590 | CG | LEU | F | 172 | 13.889 | 38.294 | 57.592 | 1.00 | 33.23 | C |
| ATOM | 9591 | CD1 | LEU | F | 172 | 12.980 | 39.163 | 58.418 | 1.00 | 32.27 | C |
| ATOM | 9592 | CD2 | LEU | F | 172 | 13.072 | 37.485 | 56.609 | 1.00 | 34.32 | C |
| ATOM | 9593 | N | GLN | F | 173 | 16.500 | 38.624 | 59.755 | 1.00 | 38.03 | N |
| ATOM | 9594 | CA | GLN | F | 173 | 17.028 | 37.595 | 60.629 | 1.00 | 40.62 | C |
| ATOM | 9595 | C | GLN | F | 173 | 16.031 | 37.278 | 61.731 | 1.00 | 41.06 | C |
| ATOM | 9596 | O | GLN | F | 173 | 15.662 | 38.153 | 62.508 | 1.00 | 41.69 | O |
| ATOM | 9597 | CB | GLN | F | 173 | 18.332 | 38.074 | 61.244 | 1.00 | 41.31 | C |
| ATOM | 9598 | CG | GLN | F | 173 | 18.809 | 37.237 | 62.415 | 1.00 | 42.98 | C |
| ATOM | 9599 | CD | GLN | F | 173 | 20.235 | 37.545 | 62.799 | 1.00 | 43.42 | C |
| ATOM | 9600 | OE1 | GLN | F | 173 | 20.562 | 38.678 | 63.183 | 1.00 | 46.16 | O |
| ATOM | 9601 | NE2 | GLN | F | 173 | 21.096 | 36.537 | 62.713 | 1.00 | 45.59 | N |
| ATOM | 9602 | N | SER | F | 174 | 15.595 | 36.027 | 61.800 | 1.00 | 41.56 | N |
| ATOM | 9603 | CA | SER | F | 174 | 14.769 | 35.582 | 62.917 | 1.00 | 41.52 | C |
| ATOM | 9604 | C | SER | F | 174 | 13.501 | 36.418 | 62.967 | 1.00 | 39.87 | C |
| ATOM | 9605 | O | SER | F | 174 | 13.078 | 36.894 | 64.030 | 1.00 | 39.35 | O |
| ATOM | 9606 | CB | SER | F | 174 | 15.559 | 35.691 | 64.231 | 1.00 | 42.81 | C |
| ATOM | 9607 | OG | SER | F | 174 | 16.941 | 35.410 | 64.017 | 1.00 | 44.69 | O |
| ATOM | 9608 | N | ASP | F | 175 | 12.921 | 36.621 | 61.788 | 1.00 | 37.69 | N |

```
ATOM   9609  CA   ASP F 175    11.630  37.265  61.662  1.00 36.58        C
ATOM   9610  C    ASP F 175    11.666  38.771  61.904  1.00 33.00        C
ATOM   9611  O    ASP F 175    10.618  39.387  62.103  1.00 32.61        O
ATOM   9612  CB   ASP F 175    10.645  36.599  62.633  1.00 38.06        C
ATOM   9613  CG   ASP F 175     9.235  36.620  62.131  1.00 41.08        C
ATOM   9614  OD1  ASP F 175     8.883  37.608  61.445  1.00 43.62        O
ATOM   9615  OD2  ASP F 175     8.487  35.648  62.421  1.00 42.74        O
ATOM   9616  N    LEU F 176    12.862  39.363  61.911  1.00 29.72        N
ATOM   9617  CA   LEU F 176    13.014  40.806  62.111  1.00 26.71        C
ATOM   9618  C    LEU F 176    14.099  41.367  61.213  1.00 24.60        C
ATOM   9619  O    LEU F 176    15.079  40.694  60.916  1.00 23.96        O
ATOM   9620  CB   LEU F 176    13.385  41.122  63.557  1.00 27.08        C
ATOM   9621  CG   LEU F 176    12.399  40.605  64.604  1.00 26.51        C
ATOM   9622  CD1  LEU F 176    13.069  40.597  65.947  1.00 26.53        C
ATOM   9623  CD2  LEU F 176    11.144  41.464  64.606  1.00 27.12        C
ATOM   9624  N    TYR F 177    13.904  42.605  60.799  1.00 23.79        N
ATOM   9625  CA   TYR F 177    14.836  43.275  59.907  1.00 23.77        C
ATOM   9626  C    TYR F 177    15.776  44.194  60.657  1.00 22.43        C
ATOM   9627  O    TYR F 177    15.437  44.732  61.706  1.00 21.86        O
ATOM   9628  CB   TYR F 177    14.081  44.122  58.906  1.00 23.52        C
ATOM   9629  CG   TYR F 177    13.417  43.315  57.835  1.00 23.49        C
ATOM   9630  CD1  TYR F 177    14.106  42.937  56.698  1.00 23.57        C
ATOM   9631  CD2  TYR F 177    12.096  42.925  57.965  1.00 25.98        C
ATOM   9632  CE1  TYR F 177    13.485  42.195  55.697  1.00 25.04        C
ATOM   9633  CE2  TYR F 177    11.458  42.188  56.971  1.00 24.29        C
ATOM   9634  CZ   TYR F 177    12.158  41.826  55.848  1.00 24.87        C
ATOM   9635  OH   TYR F 177    11.506  41.097  54.888  1.00 25.93        O
ATOM   9636  N    THR F 178    16.951  44.394  60.080  1.00 21.60        N
ATOM   9637  CA   THR F 178    17.848  45.454  60.530  1.00 22.02        C
ATOM   9638  C    THR F 178    18.397  46.184  59.334  1.00 20.26        C
ATOM   9639  O    THR F 178    18.766  45.559  58.350  1.00 20.44        O
ATOM   9640  CB   THR F 178    19.039  44.886  61.326  1.00 22.80        C
ATOM   9641  OG1  THR F 178    18.539  44.151  62.447  1.00 26.77        O
ATOM   9642  CG2  THR F 178    19.918  46.037  61.833  1.00 23.52        C
ATOM   9643  N    LEU F 179    18.481  47.504  59.431  1.00 20.23        N
ATOM   9644  CA   LEU F 179    19.278  48.262  58.473  1.00 20.80        C
ATOM   9645  C    LEU F 179    20.026  49.412  59.148  1.00 21.37        C
ATOM   9646  O    LEU F 179    19.793  49.738  60.306  1.00 20.71        O
ATOM   9647  CB   LEU F 179    18.411  48.800  57.340  1.00 21.11        C
ATOM   9648  CG   LEU F 179    17.352  49.875  57.532  1.00 20.73        C
ATOM   9649  CD1  LEU F 179    17.865  51.291  57.826  1.00 20.51        C
ATOM   9650  CD2  LEU F 179    16.486  49.893  56.261  1.00 21.79        C
ATOM   9651  N    SER F 180    20.936  50.017  58.395  1.00 20.86        N
ATOM   9652  CA   SER F 180    21.659  51.186  58.863  1.00 21.71        C
ATOM   9653  C    SER F 180    21.667  52.262  57.796  1.00 20.83        C
ATOM   9654  O    SER F 180    21.527  51.980  56.608  1.00 20.67        O
ATOM   9655  CB   SER F 180    23.089  50.800  59.220  1.00 21.95        C
ATOM   9656  OG   SER F 180    23.635  49.989  58.188  1.00 23.18        O
ATOM   9657  N    SER F 181    21.837  53.495  58.237  1.00 21.89        N
ATOM   9658  CA   SER F 181    22.003  54.606  57.333  1.00 23.51        C
ATOM   9659  C    SER F 181    23.236  55.390  57.785  1.00 24.56        C
ATOM   9660  O    SER F 181    23.444  55.603  58.977  1.00 22.74        O
ATOM   9661  CB   SER F 181    20.767  55.494  57.351  1.00 23.36        C
ATOM   9662  OG   SER F 181    20.850  56.493  56.345  1.00 24.60        O
ATOM   9663  N    SER F 182    24.085  55.765  56.843  1.00 27.09        N
ATOM   9664  CA   SER F 182    25.225  56.598  57.186  1.00 28.32        C
ATOM   9665  C    SER F 182    25.046  57.970  56.590  1.00 28.30        C
ATOM   9666  O    SER F 182    24.393  58.131  55.566  1.00 28.94        O
ATOM   9667  CB   SER F 182    26.552  55.965  56.741  1.00 29.84        C
ATOM   9668  OG   SER F 182    26.834  56.156  55.373  1.00 30.53        O
ATOM   9669  N    VAL F 183    25.605  58.962  57.263  1.00 28.06        N
ATOM   9670  CA   VAL F 183    25.601  60.330  56.761  1.00 29.19        C
ATOM   9671  C    VAL F 183    26.985  60.901  57.043  1.00 30.87        C
ATOM   9672  O    VAL F 183    27.532  60.713  58.128  1.00 29.94        O
ATOM   9673  CB   VAL F 183    24.478  61.198  57.394  1.00 28.61        C
```

| ATOM | 9674 | CG1 | VAL | F | 183 | 24.622 | 61.308 | 58.894 | 1.00 | 28.33 | C |
|------|------|-----|-----|---|-----|--------|--------|--------|------|-------|---|
| ATOM | 9675 | CG2 | VAL | F | 183 | 24.447 | 62.602 | 56.768 | 1.00 | 29.14 | C |
| ATOM | 9676 | N | THR | F | 184 | 27.569 | 61.559 | 56.048 | 1.00 | 32.53 | N |
| ATOM | 9677 | CA | THR | F | 184 | 28.881 | 62.164 | 56.225 | 1.00 | 33.49 | C |
| ATOM | 9678 | C | THR | F | 184 | 28.747 | 63.680 | 56.113 | 1.00 | 34.46 | C |
| ATOM | 9679 | O | THR | F | 184 | 28.176 | 64.184 | 55.143 | 1.00 | 34.87 | O |
| ATOM | 9680 | CB | THR | F | 184 | 29.854 | 61.594 | 55.189 | 1.00 | 33.58 | C |
| ATOM | 9681 | OG1 | THR | F | 184 | 29.877 | 60.164 | 55.320 | 1.00 | 33.66 | O |
| ATOM | 9682 | CG2 | THR | F | 184 | 31.242 | 62.144 | 55.385 | 1.00 | 34.30 | C |
| ATOM | 9683 | N | VAL | F | 185 | 29.247 | 64.388 | 57.120 | 1.00 | 35.35 | N |
| ATOM | 9684 | CA | VAL | F | 185 | 29.192 | 65.843 | 57.168 | 1.00 | 36.37 | C |
| ATOM | 9685 | C | VAL | F | 185 | 30.599 | 66.401 | 57.422 | 1.00 | 37.23 | C |
| ATOM | 9686 | O | VAL | F | 185 | 31.497 | 65.655 | 57.811 | 1.00 | 37.46 | O |
| ATOM | 9687 | CB | VAL | F | 185 | 28.231 | 66.366 | 58.276 | 1.00 | 36.41 | C |
| ATOM | 9688 | CG1 | VAL | F | 185 | 26.788 | 65.917 | 58.020 | 1.00 | 36.73 | C |
| ATOM | 9689 | CG2 | VAL | F | 185 | 28.685 | 65.947 | 59.688 | 1.00 | 37.25 | C |
| ATOM | 9690 | N | PRO | F | 186 | 30.797 | 67.715 | 57.198 | 1.00 | 38.16 | N |
| ATOM | 9691 | CA | PRO | F | 186 | 32.047 | 68.354 | 57.616 | 1.00 | 37.90 | C |
| ATOM | 9692 | C | PRO | F | 186 | 32.223 | 68.360 | 59.141 | 1.00 | 38.21 | C |
| ATOM | 9693 | O | PRO | F | 186 | 31.283 | 68.676 | 59.877 | 1.00 | 37.81 | O |
| ATOM | 9694 | CB | PRO | F | 186 | 31.910 | 69.791 | 57.069 | 1.00 | 38.45 | C |
| ATOM | 9695 | CG | PRO | F | 186 | 30.841 | 69.726 | 56.029 | 1.00 | 37.88 | C |
| ATOM | 9696 | CD | PRO | F | 186 | 29.899 | 68.670 | 56.525 | 1.00 | 38.32 | C |
| ATOM | 9697 | N | SER | F | 187 | 33.425 | 68.007 | 59.600 | 1.00 | 38.40 | N |
| ATOM | 9698 | CA | SER | F | 187 | 33.740 | 67.967 | 61.033 | 1.00 | 39.02 | C |
| ATOM | 9699 | C | SER | F | 187 | 33.438 | 69.281 | 61.727 | 1.00 | 38.29 | C |
| ATOM | 9700 | O | SER | F | 187 | 33.046 | 69.300 | 62.885 | 1.00 | 37.83 | O |
| ATOM | 9701 | CB | SER | F | 187 | 35.214 | 67.625 | 61.252 | 1.00 | 39.54 | C |
| ATOM | 9702 | OG | SER | F | 187 | 35.664 | 66.685 | 60.296 | 1.00 | 42.54 | O |
| ATOM | 9703 | N | SER | F | 188 | 33.605 | 70.393 | 61.022 | 1.00 | 38.70 | N |
| ATOM | 9704 | CA | SER | F | 188 | 33.322 | 71.695 | 61.623 | 1.00 | 38.90 | C |
| ATOM | 9705 | C | SER | F | 188 | 31.847 | 71.891 | 62.014 | 1.00 | 39.21 | C |
| ATOM | 9706 | O | SER | F | 188 | 31.554 | 72.690 | 62.904 | 1.00 | 39.47 | O |
| ATOM | 9707 | CB | SER | F | 188 | 33.786 | 72.834 | 60.697 | 1.00 | 38.68 | C |
| ATOM | 9708 | OG | SER | F | 188 | 33.618 | 72.488 | 59.335 | 1.00 | 38.08 | O |
| ATOM | 9709 | N | THR | F | 189 | 30.934 | 71.156 | 61.379 | 1.00 | 39.44 | N |
| ATOM | 9710 | CA | THR | F | 189 | 29.491 | 71.357 | 61.579 | 1.00 | 39.70 | C |
| ATOM | 9711 | C | THR | F | 189 | 28.894 | 70.565 | 62.759 | 1.00 | 39.61 | C |
| ATOM | 9712 | O | THR | F | 189 | 27.860 | 70.953 | 63.311 | 1.00 | 39.98 | O |
| ATOM | 9713 | CB | THR | F | 189 | 28.694 | 70.999 | 60.289 | 1.00 | 40.27 | C |
| ATOM | 9714 | OG1 | THR | F | 189 | 28.800 | 69.594 | 60.017 | 1.00 | 40.93 | O |
| ATOM | 9715 | CG2 | THR | F | 189 | 29.215 | 71.791 | 59.086 | 1.00 | 40.33 | C |
| ATOM | 9716 | N | TRP | F | 190 | 29.536 | 69.466 | 63.142 | 1.00 | 38.54 | N |
| ATOM | 9717 | CA | TRP | F | 190 | 29.009 | 68.602 | 64.193 | 1.00 | 37.75 | C |
| ATOM | 9718 | C | TRP | F | 190 | 29.998 | 68.577 | 65.348 | 1.00 | 37.98 | C |
| ATOM | 9719 | O | TRP | F | 190 | 31.196 | 68.445 | 65.124 | 1.00 | 38.27 | O |
| ATOM | 9720 | CB | TRP | F | 190 | 28.795 | 67.195 | 63.637 | 1.00 | 37.42 | C |
| ATOM | 9721 | CG | TRP | F | 190 | 28.120 | 66.248 | 64.592 | 1.00 | 36.99 | C |
| ATOM | 9722 | CD1 | TRP | F | 190 | 26.794 | 66.200 | 64.901 | 1.00 | 36.62 | C |
| ATOM | 9723 | CD2 | TRP | F | 190 | 28.749 | 65.213 | 65.357 | 1.00 | 36.86 | C |
| ATOM | 9724 | NE1 | TRP | F | 190 | 26.556 | 65.195 | 65.814 | 1.00 | 36.61 | N |
| ATOM | 9725 | CE2 | TRP | F | 190 | 27.740 | 64.575 | 66.108 | 1.00 | 36.69 | C |
| ATOM | 9726 | CE3 | TRP | F | 190 | 30.065 | 64.766 | 65.480 | 1.00 | 36.58 | C |
| ATOM | 9727 | CZ2 | TRP | F | 190 | 28.010 | 63.508 | 66.973 | 1.00 | 37.01 | C |
| ATOM | 9728 | CZ3 | TRP | F | 190 | 30.335 | 63.708 | 66.339 | 1.00 | 36.97 | C |
| ATOM | 9729 | CH2 | TRP | F | 190 | 29.310 | 63.092 | 67.074 | 1.00 | 36.66 | C |
| ATOM | 9730 | N | PRO | F | 191 | 29.509 | 68.675 | 66.594 | 1.00 | 38.18 | N |
| ATOM | 9731 | CA | PRO | F | 191 | 28.125 | 68.749 | 67.082 | 1.00 | 38.81 | C |
| ATOM | 9732 | C | PRO | F | 191 | 27.441 | 70.116 | 67.086 | 1.00 | 39.53 | C |
| ATOM | 9733 | O | PRO | F | 191 | 26.284 | 70.211 | 67.521 | 1.00 | 39.47 | O |
| ATOM | 9734 | CB | PRO | F | 191 | 28.253 | 68.236 | 68.520 | 1.00 | 39.33 | C |
| ATOM | 9735 | CG | PRO | F | 191 | 29.634 | 68.632 | 68.933 | 1.00 | 38.47 | C |
| ATOM | 9736 | CD | PRO | F | 191 | 30.480 | 68.648 | 67.702 | 1.00 | 38.54 | C |
| ATOM | 9737 | N | SER | F | 192 | 28.114 | 71.165 | 66.608 | 1.00 | 39.90 | N |
| ATOM | 9738 | CA | SER | F | 192 | 27.525 | 72.515 | 66.624 | 1.00 | 39.16 | C |

| | | | | | | | | | | | |
|---|---|---|---|---|---|---|---|---|---|---|---|
| ATOM | 9739 | C | SER | F | 192 | 26.158 | 72.576 | 65.931 | 1.00 | 39.39 | C |
| ATOM | 9740 | O | SER | F | 192 | 25.231 | 73.222 | 66.430 | 1.00 | 40.48 | O |
| ATOM | 9741 | CB | SER | F | 192 | 28.486 | 73.547 | 66.007 | 1.00 | 38.87 | C |
| ATOM | 9742 | OG | SER | F | 192 | 28.926 | 73.148 | 64.722 | 1.00 | 38.00 | O |
| ATOM | 9743 | N | GLU | F | 193 | 26.039 | 71.919 | 64.783 | 1.00 | 38.73 | N |
| ATOM | 9744 | CA | GLU | F | 193 | 24.756 | 71.788 | 64.104 | 1.00 | 38.65 | C |
| ATOM | 9745 | C | GLU | F | 193 | 24.235 | 70.369 | 64.334 | 1.00 | 36.40 | C |
| ATOM | 9746 | O | GLU | F | 193 | 24.962 | 69.395 | 64.124 | 1.00 | 35.32 | O |
| ATOM | 9747 | CB | GLU | F | 193 | 24.902 | 72.074 | 62.605 | 1.00 | 39.43 | C |
| ATOM | 9748 | CG | GLU | F | 193 | 25.314 | 73.522 | 62.289 | 1.00 | 40.94 | C |
| ATOM | 9749 | CD | GLU | F | 193 | 25.853 | 73.708 | 60.869 | 1.00 | 41.86 | C |
| ATOM | 9750 | OE1 | GLU | F | 193 | 25.737 | 72.781 | 60.037 | 1.00 | 44.71 | O |
| ATOM | 9751 | OE2 | GLU | F | 193 | 26.389 | 74.800 | 60.580 | 1.00 | 44.38 | O |
| ATOM | 9752 | N | THR | F | 194 | 22.976 | 70.262 | 64.758 | 1.00 | 34.81 | N |
| ATOM | 9753 | CA | THR | F | 194 | 22.406 | 68.961 | 65.116 | 1.00 | 32.19 | C |
| ATOM | 9754 | C | THR | F | 194 | 22.210 | 68.062 | 63.899 | 1.00 | 30.58 | C |
| ATOM | 9755 | O | THR | F | 194 | 21.941 | 68.527 | 62.784 | 1.00 | 28.33 | O |
| ATOM | 9756 | CB | THR | F | 194 | 21.070 | 69.082 | 65.875 | 1.00 | 32.32 | C |
| ATOM | 9757 | OG1 | THR | F | 194 | 20.072 | 69.617 | 65.006 | 1.00 | 33.14 | O |
| ATOM | 9758 | CG2 | THR | F | 194 | 21.217 | 69.953 | 67.096 | 1.00 | 32.36 | C |
| ATOM | 9759 | N | VAL | F | 195 | 22.368 | 66.762 | 64.132 | 1.00 | 27.55 | N |
| ATOM | 9760 | CA | VAL | F | 195 | 22.154 | 65.754 | 63.112 | 1.00 | 27.42 | C |
| ATOM | 9761 | C | VAL | F | 195 | 21.252 | 64.675 | 63.713 | 1.00 | 27.07 | C |
| ATOM | 9762 | O | VAL | F | 195 | 21.573 | 64.121 | 64.755 | 1.00 | 24.19 | O |
| ATOM | 9763 | CB | VAL | F | 195 | 23.473 | 65.142 | 62.656 | 1.00 | 27.59 | C |
| ATOM | 9764 | CG1 | VAL | F | 195 | 23.219 | 64.025 | 61.650 | 1.00 | 27.89 | C |
| ATOM | 9765 | CG2 | VAL | F | 195 | 24.386 | 66.230 | 62.064 | 1.00 | 28.06 | C |
| ATOM | 9766 | N | THR | F | 196 | 20.119 | 64.419 | 63.059 | 1.00 | 26.83 | N |
| ATOM | 9767 | CA | THR | F | 196 | 19.043 | 63.599 | 63.630 | 1.00 | 26.42 | C |
| ATOM | 9768 | C | THR | F | 196 | 18.504 | 62.716 | 62.540 | 1.00 | 26.44 | C |
| ATOM | 9769 | O | THR | F | 196 | 18.187 | 63.216 | 61.457 | 1.00 | 26.95 | O |
| ATOM | 9770 | CB | THR | F | 196 | 17.897 | 64.504 | 64.122 | 1.00 | 26.57 | C |
| ATOM | 9771 | OG1 | THR | F | 196 | 18.374 | 65.385 | 65.139 | 1.00 | 26.49 | O |
| ATOM | 9772 | CG2 | THR | F | 196 | 16.745 | 63.700 | 64.687 | 1.00 | 27.10 | C |
| ATOM | 9773 | N | CYS | F | 197 | 18.414 | 61.412 | 62.775 | 1.00 | 25.38 | N |
| ATOM | 9774 | CA | CYS | F | 197 | 17.798 | 60.555 | 61.785 | 1.00 | 26.08 | C |
| ATOM | 9775 | C | CYS | F | 197 | 16.326 | 60.396 | 62.133 | 1.00 | 25.48 | C |
| ATOM | 9776 | O | CYS | F | 197 | 15.958 | 60.381 | 63.309 | 1.00 | 25.78 | O |
| ATOM | 9777 | CB | CYS | F | 197 | 18.506 | 59.212 | 61.641 | 1.00 | 27.66 | C |
| ATOM | 9778 | SG | CYS | F | 197 | 18.181 | 58.034 | 62.946 | 1.00 | 31.91 | S |
| ATOM | 9779 | N | ASN | F | 198 | 15.492 | 60.342 | 61.102 | 1.00 | 24.70 | N |
| ATOM | 9780 | CA | ASN | F | 198 | 14.052 | 60.130 | 61.256 | 1.00 | 24.07 | C |
| ATOM | 9781 | C | ASN | F | 198 | 13.724 | 58.813 | 60.578 | 1.00 | 22.72 | C |
| ATOM | 9782 | O | ASN | F | 198 | 14.102 | 58.596 | 59.424 | 1.00 | 23.21 | O |
| ATOM | 9783 | CB | ASN | F | 198 | 13.268 | 61.269 | 60.599 | 1.00 | 25.25 | C |
| ATOM | 9784 | CG | ASN | F | 198 | 14.026 | 62.584 | 60.615 | 1.00 | 25.23 | C |
| ATOM | 9785 | OD1 | ASN | F | 198 | 14.580 | 63.010 | 59.587 | 1.00 | 26.66 | O |
| ATOM | 9786 | ND2 | ASN | F | 198 | 14.074 | 63.224 | 61.774 | 1.00 | 25.46 | N |
| ATOM | 9787 | N | VAL | F | 199 | 13.041 | 57.928 | 61.302 | 1.00 | 20.97 | N |
| ATOM | 9788 | CA | VAL | F | 199 | 12.713 | 56.597 | 60.806 | 1.00 | 20.17 | C |
| ATOM | 9789 | C | VAL | F | 199 | 11.207 | 56.390 | 60.847 | 1.00 | 20.31 | C |
| ATOM | 9790 | O | VAL | F | 199 | 10.605 | 56.410 | 61.932 | 1.00 | 20.90 | O |
| ATOM | 9791 | CB | VAL | F | 199 | 13.396 | 55.509 | 61.647 | 1.00 | 19.14 | C |
| ATOM | 9792 | CG1 | VAL | F | 199 | 13.177 | 54.144 | 61.032 | 1.00 | 18.92 | C |
| ATOM | 9793 | CG2 | VAL | F | 199 | 14.910 | 55.830 | 61.820 | 1.00 | 18.89 | C |
| ATOM | 9794 | N | ALA | F | 200 | 10.623 | 56.169 | 59.673 | 1.00 | 20.43 | N |
| ATOM | 9795 | CA | ALA | F | 200 | 9.189 | 55.925 | 59.544 | 1.00 | 20.04 | C |
| ATOM | 9796 | C | ALA | F | 200 | 8.944 | 54.473 | 59.166 | 1.00 | 19.32 | C |
| ATOM | 9797 | O | ALA | F | 200 | 9.579 | 53.936 | 58.263 | 1.00 | 20.50 | O |
| ATOM | 9798 | CB | ALA | F | 200 | 8.594 | 56.844 | 58.510 | 1.00 | 21.50 | C |
| ATOM | 9799 | N | HIS | F | 201 | 8.002 | 53.842 | 59.850 | 1.00 | 19.08 | N |
| ATOM | 9800 | CA | HIS | F | 201 | 7.656 | 52.445 | 59.607 | 1.00 | 19.09 | C |
| ATOM | 9801 | C | HIS | F | 201 | 6.149 | 52.383 | 59.298 | 1.00 | 20.38 | C |
| ATOM | 9802 | O | HIS | F | 201 | 5.344 | 52.389 | 60.217 | 1.00 | 20.30 | O |
| ATOM | 9803 | CB | HIS | F | 201 | 7.945 | 51.630 | 60.872 | 1.00 | 18.80 | C |

| ATOM | 9804 | CG | HIS | F | 201 | 7.814 | 50.155 | 60.684 | 1.00 | 15.27 | C |
| ATOM | 9805 | ND1 | HIS | F | 201 | 7.271 | 49.332 | 61.645 | 1.00 | 16.84 | N |
| ATOM | 9806 | CD2 | HIS | F | 201 | 8.156 | 49.358 | 59.651 | 1.00 | 16.78 | C |
| ATOM | 9807 | CE1 | HIS | F | 201 | 7.284 | 48.086 | 61.206 | 1.00 | 16.16 | C |
| ATOM | 9808 | NE2 | HIS | F | 201 | 7.820 | 48.071 | 59.999 | 1.00 | 16.33 | N |
| ATOM | 9809 | N | PRO | F | 202 | 5.767 | 52.390 | 58.010 | 1.00 | 20.58 | N |
| ATOM | 9810 | CA | PRO | F | 202 | 4.325 | 52.369 | 57.685 | 1.00 | 21.50 | C |
| ATOM | 9811 | C | PRO | F | 202 | 3.498 | 51.313 | 58.432 | 1.00 | 21.54 | C |
| ATOM | 9812 | O | PRO | F | 202 | 2.424 | 51.630 | 58.946 | 1.00 | 22.28 | O |
| ATOM | 9813 | CB | PRO | F | 202 | 4.321 | 52.075 | 56.185 | 1.00 | 21.72 | C |
| ATOM | 9814 | CG | PRO | F | 202 | 5.589 | 52.707 | 55.702 | 1.00 | 21.80 | C |
| ATOM | 9815 | CD | PRO | F | 202 | 6.597 | 52.405 | 56.789 | 1.00 | 21.65 | C |
| ATOM | 9816 | N | ALA | F | 203 | 3.988 | 50.082 | 58.518 | 1.00 | 20.41 | N |
| ATOM | 9817 | CA | ALA | F | 203 | 3.172 | 49.013 | 59.077 | 1.00 | 20.52 | C |
| ATOM | 9818 | C | ALA | F | 203 | 2.769 | 49.243 | 60.529 | 1.00 | 21.26 | C |
| ATOM | 9819 | O | ALA | F | 203 | 1.718 | 48.757 | 60.949 | 1.00 | 22.48 | O |
| ATOM | 9820 | CB | ALA | F | 203 | 3.855 | 47.665 | 58.933 | 1.00 | 19.42 | C |
| ATOM | 9821 | N | SER | F | 204 | 3.590 | 49.974 | 61.292 | 1.00 | 21.46 | N |
| ATOM | 9822 | CA | SER | F | 204 | 3.289 | 50.285 | 62.687 | 1.00 | 20.93 | C |
| ATOM | 9823 | C | SER | F | 204 | 2.820 | 51.722 | 62.858 | 1.00 | 20.76 | C |
| ATOM | 9824 | O | SER | F | 204 | 2.546 | 52.144 | 63.960 | 1.00 | 21.69 | O |
| ATOM | 9825 | CB | SER | F | 204 | 4.501 | 50.013 | 63.604 | 1.00 | 20.07 | C |
| ATOM | 9826 | OG | SER | F | 204 | 5.630 | 50.811 | 63.245 | 1.00 | 23.35 | O |
| ATOM | 9827 | N | SER | F | 205 | 2.698 | 52.474 | 61.766 | 1.00 | 22.08 | N |
| ATOM | 9828 | CA | SER | F | 205 | 2.209 | 53.852 | 61.832 | 1.00 | 21.41 | C |
| ATOM | 9829 | C | SER | F | 205 | 3.045 | 54.701 | 62.787 | 1.00 | 21.58 | C |
| ATOM | 9830 | O | SER | F | 205 | 2.524 | 55.567 | 63.497 | 1.00 | 21.00 | O |
| ATOM | 9831 | CB | SER | F | 205 | 0.719 | 53.861 | 62.232 | 1.00 | 21.64 | C |
| ATOM | 9832 | OG | SER | F | 205 | -0.027 | 53.161 | 61.240 | 1.00 | 20.26 | O |
| ATOM | 9833 | N | THR | F | 206 | 4.358 | 54.457 | 62.785 | 1.00 | 21.37 | N |
| ATOM | 9834 | CA | THR | F | 206 | 5.291 | 55.126 | 63.685 | 1.00 | 21.73 | C |
| ATOM | 9835 | C | THR | F | 206 | 6.326 | 55.950 | 62.917 | 1.00 | 22.35 | C |
| ATOM | 9836 | O | THR | F | 206 | 6.703 | 55.622 | 61.787 | 1.00 | 20.37 | O |
| ATOM | 9837 | CB | THR | F | 206 | 6.053 | 54.099 | 64.573 | 1.00 | 21.46 | C |
| ATOM | 9838 | OG1 | THR | F | 206 | 6.707 | 53.125 | 63.745 | 1.00 | 22.15 | O |
| ATOM | 9839 | CG2 | THR | F | 206 | 5.080 | 53.384 | 65.504 | 1.00 | 22.72 | C |
| ATOM | 9840 | N | LYS | F | 207 | 6.775 | 57.016 | 63.565 | 1.00 | 24.35 | N |
| ATOM | 9841 | CA | LYS | F | 207 | 7.933 | 57.762 | 63.130 | 1.00 | 26.76 | C |
| ATOM | 9842 | C | LYS | F | 207 | 8.715 | 58.246 | 64.358 | 1.00 | 26.51 | C |
| ATOM | 9843 | O | LYS | F | 207 | 8.156 | 58.863 | 65.250 | 1.00 | 27.25 | O |
| ATOM | 9844 | CB | LYS | F | 207 | 7.527 | 58.933 | 62.235 | 1.00 | 27.78 | C |
| ATOM | 9845 | CG | LYS | F | 207 | 8.696 | 59.475 | 61.412 | 1.00 | 29.38 | C |
| ATOM | 9846 | CD | LYS | F | 207 | 8.208 | 60.378 | 60.320 | 1.00 | 30.91 | C |
| ATOM | 9847 | CE | LYS | F | 207 | 9.320 | 61.179 | 59.692 | 1.00 | 32.90 | C |
| ATOM | 9848 | NZ | LYS | F | 207 | 8.718 | 62.167 | 58.757 | 1.00 | 35.78 | N |
| ATOM | 9849 | N | VAL | F | 208 | 10.007 | 57.931 | 64.391 | 1.00 | 25.16 | N |
| ATOM | 9850 | CA | VAL | F | 208 | 10.862 | 58.221 | 65.535 | 1.00 | 25.77 | C |
| ATOM | 9851 | C | VAL | F | 208 | 12.082 | 58.983 | 65.083 | 1.00 | 24.94 | C |
| ATOM | 9852 | O | VAL | F | 208 | 12.568 | 58.753 | 63.988 | 1.00 | 23.51 | O |
| ATOM | 9853 | CB | VAL | F | 208 | 11.276 | 56.909 | 66.235 | 1.00 | 26.46 | C |
| ATOM | 9854 | CG1 | VAL | F | 208 | 12.463 | 57.123 | 67.155 | 1.00 | 29.02 | C |
| ATOM | 9855 | CG2 | VAL | F | 208 | 10.094 | 56.396 | 67.025 | 1.00 | 27.59 | C |
| ATOM | 9856 | N | ASP | F | 209 | 12.545 | 59.893 | 65.932 | 1.00 | 25.90 | N |
| ATOM | 9857 | CA | ASP | F | 209 | 13.791 | 60.624 | 65.701 | 1.00 | 26.95 | C |
| ATOM | 9858 | C | ASP | F | 209 | 14.837 | 60.219 | 66.701 | 1.00 | 26.53 | C |
| ATOM | 9859 | O | ASP | F | 209 | 14.535 | 60.010 | 67.886 | 1.00 | 27.33 | O |
| ATOM | 9860 | CB | ASP | F | 209 | 13.586 | 62.121 | 65.881 | 1.00 | 28.08 | C |
| ATOM | 9861 | CG | ASP | F | 209 | 12.589 | 62.695 | 64.926 | 1.00 | 30.10 | C |
| ATOM | 9862 | OD1 | ASP | F | 209 | 12.381 | 62.156 | 63.813 | 1.00 | 33.49 | O |
| ATOM | 9863 | OD2 | ASP | F | 209 | 12.011 | 63.724 | 65.304 | 1.00 | 34.83 | O |
| ATOM | 9864 | N | LYS | F | 210 | 16.078 | 60.147 | 66.233 | 1.00 | 24.16 | N |
| ATOM | 9865 | CA | LYS | F | 210 | 17.217 | 59.932 | 67.107 | 1.00 | 23.54 | C |
| ATOM | 9866 | C | LYS | F | 210 | 18.299 | 60.948 | 66.756 | 1.00 | 23.40 | C |
| ATOM | 9867 | O | LYS | F | 210 | 18.881 | 60.891 | 65.672 | 1.00 | 22.80 | O |
| ATOM | 9868 | CB | LYS | F | 210 | 17.735 | 58.500 | 66.937 | 1.00 | 22.39 | C |

| ATOM | 9869 | CG | LYS F 210 | 18.941 | 58.120 | 67.813 | 1.00 | 22.96 | C |
|---|---|---|---|---|---|---|---|---|---|
| ATOM | 9870 | CD | LYS F 210 | 18.759 | 58.454 | 69.296 | 1.00 | 21.68 | C |
| ATOM | 9871 | CE | LYS F 210 | 17.655 | 57.637 | 69.952 | 1.00 | 20.41 | C |
| ATOM | 9872 | NZ | LYS F 210 | 17.544 | 57.921 | 71.431 | 1.00 | 21.34 | N |
| ATOM | 9873 | N | LYS F 211 | 18.528 | 61.885 | 67.663 | 1.00 | 24.19 | N |
| ATOM | 9874 | CA | LYS F 211 | 19.633 | 62.814 | 67.536 | 1.00 | 25.54 | C |
| ATOM | 9875 | C | LYS F 211 | 20.936 | 62.111 | 67.858 | 1.00 | 24.89 | C |
| ATOM | 9876 | O | LYS F 211 | 21.022 | 61.352 | 68.843 | 1.00 | 22.56 | O |
| ATOM | 9877 | CB | LYS F 211 | 19.444 | 64.020 | 68.470 | 1.00 | 26.20 | C |
| ATOM | 9878 | CG | LYS F 211 | 20.612 | 65.022 | 68.428 | 1.00 | 27.28 | C |
| ATOM | 9879 | CD | LYS F 211 | 20.238 | 66.448 | 68.855 | 1.00 | 30.05 | C |
| ATOM | 9880 | CE | LYS F 211 | 20.296 | 66.676 | 70.356 | 1.00 | 32.40 | C |
| ATOM | 9881 | NZ | LYS F 211 | 20.783 | 68.068 | 70.675 | 1.00 | 32.94 | N |
| ATOM | 9882 | N | ILE F 212 | 21.952 | 62.394 | 67.042 | 1.00 | 23.36 | N |
| ATOM | 9883 | CA | ILE F 212 | 23.271 | 61.804 | 67.198 | 1.00 | 23.96 | C |
| ATOM | 9884 | C | ILE F 212 | 24.105 | 62.790 | 67.993 | 1.00 | 25.90 | C |
| ATOM | 9885 | O | ILE F 212 | 24.284 | 63.936 | 67.564 | 1.00 | 26.03 | O |
| ATOM | 9886 | CB | ILE F 212 | 23.925 | 61.530 | 65.833 | 1.00 | 23.43 | C |
| ATOM | 9887 | CG1 | ILE F 212 | 22.956 | 60.805 | 64.882 | 1.00 | 24.37 | C |
| ATOM | 9888 | CG2 | ILE F 212 | 25.237 | 60.744 | 65.996 | 1.00 | 24.40 | C |
| ATOM | 9889 | CD1 | ILE F 212 | 22.468 | 59.451 | 65.382 | 1.00 | 23.62 | C |
| ATOM | 9890 | N | VAL F 213 | 24.571 | 62.377 | 69.171 | 1.00 | 27.14 | N |
| ATOM | 9891 | CA | VAL F 213 | 25.394 | 63.255 | 69.994 | 1.00 | 29.29 | C |
| ATOM | 9892 | C | VAL F 213 | 26.757 | 62.657 | 70.314 | 1.00 | 29.74 | C |
| ATOM | 9893 | O | VAL F 213 | 26.927 | 61.440 | 70.324 | 1.00 | 26.92 | O |
| ATOM | 9894 | CB | VAL F 213 | 24.675 | 63.716 | 71.286 | 1.00 | 30.17 | C |
| ATOM | 9895 | CG1 | VAL F 213 | 23.365 | 64.391 | 70.945 | 1.00 | 31.70 | C |
| ATOM | 9896 | CG2 | VAL F 213 | 24.451 | 62.584 | 72.245 | 1.00 | 30.65 | C |
| ATOM | 9897 | N | PRO F 214 | 27.753 | 63.524 | 70.546 | 1.00 | 30.93 | N |
| ATOM | 9898 | CA | PRO F 214 | 29.076 | 63.034 | 70.924 | 1.00 | 31.85 | C |
| ATOM | 9899 | C | PRO F 214 | 29.053 | 62.108 | 72.141 | 1.00 | 31.86 | C |
| ATOM | 9900 | O | PRO F 214 | 28.258 | 62.290 | 73.057 | 1.00 | 31.87 | O |
| ATOM | 9901 | CB | PRO F 214 | 29.845 | 64.325 | 71.226 | 1.00 | 32.28 | C |
| ATOM | 9902 | CG | PRO F 214 | 29.147 | 65.364 | 70.397 | 1.00 | 32.01 | C |
| ATOM | 9903 | CD | PRO F 214 | 27.718 | 64.996 | 70.427 | 1.00 | 31.87 | C |
| ATOM | 9904 | N | ARG F 215 | 29.903 | 61.095 | 72.140 | 1.00 | 33.56 | N |
| ATOM | 9905 | CA | ARG F 215 | 29.899 | 60.129 | 73.238 | 1.00 | 34.34 | C |
| ATOM | 9906 | C | ARG F 215 | 30.617 | 60.700 | 74.451 | 1.00 | 35.49 | C |
| ATOM | 9907 | O | ARG F 215 | 31.463 | 61.585 | 74.312 | 1.00 | 37.10 | O |
| ATOM | 9908 | CB | ARG F 215 | 30.535 | 58.821 | 72.793 | 1.00 | 34.31 | C |
| ATOM | 9909 | CG | ARG F 215 | 29.748 | 58.134 | 71.702 | 1.00 | 34.56 | C |
| ATOM | 9910 | CD | ARG F 215 | 30.507 | 56.969 | 71.122 | 1.00 | 34.55 | C |
| ATOM | 9911 | NE | ARG F 215 | 30.976 | 56.076 | 72.171 | 1.00 | 35.01 | N |
| ATOM | 9912 | CZ | ARG F 215 | 30.292 | 55.047 | 72.671 | 1.00 | 36.19 | C |
| ATOM | 9913 | NH1 | ARG F 215 | 29.064 | 54.742 | 72.234 | 1.00 | 35.66 | N |
| ATOM | 9914 | NH2 | ARG F 215 | 30.851 | 54.311 | 73.630 | 1.00 | 35.37 | N |
| TER | 9915 | | ARG F 215 | | | | | | |
| ATOM | 9916 | N | ASP G 1 | 37.312 | 30.351 | -53.551 | 1.00 | 30.08 | N |
| ATOM | 9917 | CA | ASP G 1 | 36.645 | 29.028 | -53.541 | 1.00 | 28.55 | C |
| ATOM | 9918 | C | ASP G 1 | 35.579 | 28.987 | -54.625 | 1.00 | 27.18 | C |
| ATOM | 9919 | O | ASP G 1 | 35.061 | 30.034 | -55.049 | 1.00 | 27.84 | O |
| ATOM | 9920 | CB | ASP G 1 | 35.954 | 28.786 | -52.200 | 1.00 | 29.21 | C |
| ATOM | 9921 | CG | ASP G 1 | 36.853 | 29.056 | -51.009 | 1.00 | 29.82 | C |
| ATOM | 9922 | OD1 | ASP G 1 | 38.060 | 29.329 | -51.224 | 1.00 | 29.96 | O |
| ATOM | 9923 | OD2 | ASP G 1 | 36.327 | 28.966 | -49.861 | 1.00 | 32.20 | O |
| ATOM | 9924 | N | ILE G 2 | 35.221 | 27.781 | -55.042 | 1.00 | 22.87 | N |
| ATOM | 9925 | CA | ILE G 2 | 34.094 | 27.609 | -55.937 | 1.00 | 20.58 | C |
| ATOM | 9926 | C | ILE G 2 | 32.815 | 27.805 | -55.146 | 1.00 | 21.44 | C |
| ATOM | 9927 | O | ILE G 2 | 32.607 | 27.199 | -54.068 | 1.00 | 20.10 | O |
| ATOM | 9928 | CB | ILE G 2 | 34.110 | 26.241 | -56.651 | 1.00 | 20.52 | C |
| ATOM | 9929 | CG1 | ILE G 2 | 35.308 | 26.166 | -57.607 | 1.00 | 18.46 | C |
| ATOM | 9930 | CG2 | ILE G 2 | 32.775 | 25.981 | -57.413 | 1.00 | 20.22 | C |
| ATOM | 9931 | CD1 | ILE G 2 | 35.580 | 24.784 | -58.146 | 1.00 | 19.29 | C |
| ATOM | 9932 | N | VAL G 3 | 31.954 | 28.661 | -55.694 | 1.00 | 19.81 | N |
| ATOM | 9933 | CA | VAL G 3 | 30.639 | 28.865 | -55.175 | 1.00 | 20.51 | C |

| ATOM | 9934 | C | VAL | G | 3 | 29.635 | 27.998 | -55.933 | 1.00 | 19.43 | C |
|------|------|-----|-----|---|----|--------|--------|---------|------|-------|---|
| ATOM | 9935 | O | VAL | G | 3 | 29.509 | 28.082 | -57.171 | 1.00 | 18.40 | O |
| ATOM | 9936 | CB | VAL | G | 3 | 30.194 | 30.320 | -55.286 | 1.00 | 20.66 | C |
| ATOM | 9937 | CG1 | VAL | G | 3 | 28.793 | 30.511 | -54.650 | 1.00 | 21.82 | C |
| ATOM | 9938 | CG2 | VAL | G | 3 | 31.225 | 31.225 | -54.633 | 1.00 | 22.32 | C |
| ATOM | 9939 | N | MET | G | 4 | 28.908 | 27.202 | -55.158 | 1.00 | 17.71 | N |
| ATOM | 9940 | CA | MET | G | 4 | 27.852 | 26.351 | -55.678 | 1.00 | 17.53 | C |
| ATOM | 9941 | C | MET | G | 4 | 26.552 | 27.017 | -55.267 | 1.00 | 18.13 | C |
| ATOM | 9942 | O | MET | G | 4 | 26.290 | 27.213 | -54.059 | 1.00 | 18.43 | O |
| ATOM | 9943 | CB | MET | G | 4 | 27.937 | 24.927 | -55.077 | 1.00 | 16.55 | C |
| ATOM | 9944 | CG | MET | G | 4 | 29.271 | 24.186 | -55.258 | 1.00 | 17.33 | C |
| ATOM | 9945 | SD | MET | G | 4 | 29.629 | 23.852 | -56.992 | 1.00 | 19.69 | S |
| ATOM | 9946 | CE | MET | G | 4 | 28.388 | 22.664 | -57.414 | 1.00 | 19.84 | C |
| ATOM | 9947 | N | THR | G | 5 | 25.715 | 27.352 | -56.246 | 1.00 | 18.33 | N |
| ATOM | 9948 | CA | THR | G | 5 | 24.438 | 28.058 | -55.983 | 1.00 | 18.64 | C |
| ATOM | 9949 | C | THR | G | 5 | 23.216 | 27.196 | -56.270 | 1.00 | 19.20 | C |
| ATOM | 9950 | O | THR | G | 5 | 23.069 | 26.647 | -57.359 | 1.00 | 18.66 | O |
| ATOM | 9951 | CB | THR | G | 5 | 24.355 | 29.398 | -56.785 | 1.00 | 19.12 | C |
| ATOM | 9952 | OG1 | THR | G | 5 | 25.524 | 30.162 | -56.523 | 1.00 | 21.13 | O |
| ATOM | 9953 | CG2 | THR | G | 5 | 23.159 | 30.229 | -56.367 | 1.00 | 18.85 | C |
| ATOM | 9954 | N | GLN | G | 6 | 22.364 | 27.032 | -55.261 | 1.00 | 18.21 | N |
| ATOM | 9955 | CA | GLN | G | 6 | 21.061 | 26.448 | -55.449 | 1.00 | 19.51 | C |
| ATOM | 9956 | C | GLN | G | 6 | 20.006 | 27.184 | -54.607 | 1.00 | 21.82 | C |
| ATOM | 9957 | O | GLN | G | 6 | 20.317 | 27.841 | -53.607 | 1.00 | 21.34 | O |
| ATOM | 9958 | CB | GLN | G | 6 | 21.062 | 24.927 | -55.168 | 1.00 | 20.95 | C |
| ATOM | 9959 | CG | GLN | G | 6 | 21.638 | 24.561 | -53.878 | 1.00 | 20.86 | C |
| ATOM | 9960 | CD | GLN | G | 6 | 21.872 | 23.071 | -53.716 | 1.00 | 19.09 | C |
| ATOM | 9961 | OE1 | GLN | G | 6 | 22.799 | 22.685 | -53.041 | 1.00 | 18.04 | O |
| ATOM | 9962 | NE2 | GLN | G | 6 | 21.037 | 22.251 | -54.320 | 1.00 | 20.66 | N |
| ATOM | 9963 | N | ALA | G | 7 | 18.765 | 27.093 | -55.066 | 1.00 | 22.45 | N |
| ATOM | 9964 | CA | ALA | G | 7 | 17.626 | 27.649 | -54.355 | 1.00 | 23.49 | C |
| ATOM | 9965 | C | ALA | G | 7 | 17.356 | 26.836 | -53.084 | 1.00 | 23.82 | C |
| ATOM | 9966 | O | ALA | G | 7 | 17.559 | 25.624 | -53.059 | 1.00 | 22.53 | O |
| ATOM | 9967 | CB | ALA | G | 7 | 16.413 | 27.642 | -55.259 | 1.00 | 24.18 | C |
| ATOM | 9968 | N | ALA | G | 8 | 16.909 | 27.505 | -52.021 | 1.00 | 25.55 | N |
| ATOM | 9969 | CA | ALA | G | 8 | 16.627 | 26.812 | -50.760 | 1.00 | 27.50 | C |
| ATOM | 9970 | C | ALA | G | 8 | 15.410 | 25.906 | -50.850 | 1.00 | 29.01 | C |
| ATOM | 9971 | O | ALA | G | 8 | 15.321 | 24.929 | -50.137 | 1.00 | 28.05 | O |
| ATOM | 9972 | CB | ALA | G | 8 | 16.469 | 27.811 | -49.613 | 1.00 | 27.89 | C |
| ATOM | 9973 | N | PHE | G | 9 | 14.478 | 26.236 | -51.740 | 1.00 | 32.00 | N |
| ATOM | 9974 | CA | PHE | G | 9 | 13.200 | 25.528 | -51.826 | 1.00 | 34.62 | C |
| ATOM | 9975 | C | PHE | G | 9 | 12.832 | 25.165 | -53.267 | 1.00 | 36.62 | C |
| ATOM | 9976 | O | PHE | G | 9 | 13.035 | 25.955 | -54.201 | 1.00 | 36.32 | O |
| ATOM | 9977 | CB | PHE | G | 9 | 12.059 | 26.360 | -51.198 | 1.00 | 35.99 | C |
| ATOM | 9978 | CG | PHE | G | 9 | 12.367 | 26.859 | -49.823 | 1.00 | 36.28 | C |
| ATOM | 9979 | CD1 | PHE | G | 9 | 12.421 | 25.981 | -48.754 | 1.00 | 37.03 | C |
| ATOM | 9980 | CD2 | PHE | G | 9 | 12.637 | 28.204 | -49.604 | 1.00 | 37.93 | C |
| ATOM | 9981 | CE1 | PHE | G | 9 | 12.739 | 26.427 | -47.482 | 1.00 | 36.89 | C |
| ATOM | 9982 | CE2 | PHE | G | 9 | 12.943 | 28.666 | -48.334 | 1.00 | 38.28 | C |
| ATOM | 9983 | CZ | PHE | G | 9 | 13.001 | 27.767 | -47.268 | 1.00 | 37.86 | C |
| ATOM | 9984 | N | SER | G | 10 | 12.320 | 23.939 | -53.413 | 1.00 | 39.27 | N |
| ATOM | 9985 | CA | SER | G | 10 | 11.662 | 23.465 | -54.628 | 1.00 | 40.64 | C |
| ATOM | 9986 | C | SER | G | 10 | 10.281 | 24.111 | -54.723 | 1.00 | 42.05 | C |
| ATOM | 9987 | O | SER | G | 10 | 9.803 | 24.728 | -53.769 | 1.00 | 42.87 | O |
| ATOM | 9988 | CB | SER | G | 10 | 11.511 | 21.923 | -54.583 | 1.00 | 40.43 | C |
| ATOM | 9989 | OG | SER | G | 10 | 10.420 | 21.487 | -53.737 | 1.00 | 40.98 | O |
| ATOM | 9990 | N | ASN | G | 11 | 9.645 | 23.991 | -55.884 | 1.00 | 43.83 | N |
| ATOM | 9991 | CA | ASN | G | 11 | 8.200 | 24.197 | -55.972 | 1.00 | 44.27 | C |
| ATOM | 9992 | C | ASN | G | 11 | 7.597 | 22.928 | -55.373 | 1.00 | 43.73 | C |
| ATOM | 9993 | O | ASN | G | 11 | 8.091 | 21.836 | -55.662 | 1.00 | 43.69 | O |
| ATOM | 9994 | CB | ASN | G | 11 | 7.742 | 24.362 | -57.426 | 1.00 | 45.23 | C |
| ATOM | 9995 | CG | ASN | G | 11 | 8.471 | 25.496 | -58.161 | 1.00 | 46.26 | C |
| ATOM | 9996 | OD1 | ASN | G | 11 | 8.414 | 26.668 | -57.754 | 1.00 | 47.58 | O |
| ATOM | 9997 | ND2 | ASN | G | 11 | 9.149 | 25.147 | -59.256 | 1.00 | 46.68 | N |
| ATOM | 9998 | N | PRO | G | 12 | 6.548 | 23.050 | -54.530 | 1.00 | 42.87 | N |

```
ATOM   9999  CA  PRO G  12      6.032  21.807 -53.923  1.00 41.80           C
ATOM  10000  C   PRO G  12      5.584  20.832 -54.998  1.00 39.97           C
ATOM  10001  O   PRO G  12      4.971  21.247 -55.978  1.00 40.31           O
ATOM  10002  CB  PRO G  12      4.855  22.282 -53.061  1.00 42.38           C
ATOM  10003  CG  PRO G  12      5.078  23.769 -52.871  1.00 42.81           C
ATOM  10004  CD  PRO G  12      5.789  24.240 -54.101  1.00 43.04           C
ATOM  10005  N   VAL G  13      5.918  19.557 -54.829  1.00 37.35           N
ATOM  10006  CA  VAL G  13      5.748  18.572 -55.876  1.00 35.65           C
ATOM  10007  C   VAL G  13      4.763  17.504 -55.437  1.00 34.41           C
ATOM  10008  O   VAL G  13      4.866  16.970 -54.350  1.00 32.58           O
ATOM  10009  CB  VAL G  13      7.100  17.917 -56.246  1.00 36.12           C
ATOM  10010  CG1 VAL G  13      6.913  16.783 -57.250  1.00 36.28           C
ATOM  10011  CG2 VAL G  13      8.079  18.976 -56.774  1.00 36.55           C
ATOM  10012  N   THR G  14      3.808  17.195 -56.304  1.00 33.15           N
ATOM  10013  CA  THR G  14      2.755  16.257 -55.984  1.00 33.44           C
ATOM  10014  C   THR G  14      3.318  14.863 -56.066  1.00 33.04           C
ATOM  10015  O   THR G  14      4.109  14.571 -56.967  1.00 33.60           O
ATOM  10016  CB  THR G  14      1.578  16.437 -56.967  1.00 34.18           C
ATOM  10017  OG1 THR G  14      1.126  17.799 -56.884  1.00 33.98           O
ATOM  10018  CG2 THR G  14      0.438  15.493 -56.665  1.00 34.60           C
ATOM  10019  N   LEU G  15      2.929  14.013 -55.115  1.00 32.28           N
ATOM  10020  CA  LEU G  15      3.372  12.620 -55.083  1.00 32.65           C
ATOM  10021  C   LEU G  15      3.135  11.930 -56.426  1.00 32.49           C
ATOM  10022  O   LEU G  15      2.047  12.015 -57.004  1.00 32.76           O
ATOM  10023  CB  LEU G  15      2.657  11.829 -53.965  1.00 32.97           C
ATOM  10024  CG  LEU G  15      3.316  11.801 -52.580  1.00 33.95           C
ATOM  10025  CD1 LEU G  15      2.335  11.336 -51.505  1.00 35.13           C
ATOM  10026  CD2 LEU G  15      4.554  10.913 -52.564  1.00 34.68           C
ATOM  10027  N   GLY G  16      4.162  11.242 -56.909  1.00 31.73           N
ATOM  10028  CA  GLY G  16      4.089  10.518 -58.162  1.00 31.63           C
ATOM  10029  C   GLY G  16      4.395  11.350 -59.391  1.00 30.85           C
ATOM  10030  O   GLY G  16      4.421  10.810 -60.499  1.00 32.66           O
ATOM  10031  N   THR G  17      4.606  12.651 -59.214  1.00 29.79           N
ATOM  10032  CA  THR G  17      5.013  13.528 -60.315  1.00 29.30           C
ATOM  10033  C   THR G  17      6.500  13.884 -60.188  1.00 28.70           C
ATOM  10034  O   THR G  17      7.166  13.493 -59.216  1.00 26.90           O
ATOM  10035  CB  THR G  17      4.145  14.814 -60.404  1.00 29.28           C
ATOM  10036  OG1 THR G  17      4.462  15.727 -59.350  1.00 29.84           O
ATOM  10037  CG2 THR G  17      2.652  14.474 -60.345  1.00 30.40           C
ATOM  10038  N   SER G  18      7.006  14.604 -61.189  1.00 26.70           N
ATOM  10039  CA  SER G  18      8.431  14.855 -61.321  1.00 26.09           C
ATOM  10040  C   SER G  18      8.867  16.097 -60.570  1.00 24.66           C
ATOM  10041  O   SER G  18      8.136  17.082 -60.482  1.00 24.45           O
ATOM  10042  CB  SER G  18      8.817  14.985 -62.805  1.00 26.26           C
ATOM  10043  OG  SER G  18      8.142  16.088 -63.402  1.00 27.35           O
ATOM  10044  N   ALA G  19     10.070  16.020 -60.008  1.00 23.94           N
ATOM  10045  CA  ALA G  19     10.745  17.170 -59.439  1.00 23.45           C
ATOM  10046  C   ALA G  19     12.033  17.423 -60.217  1.00 22.91           C
ATOM  10047  O   ALA G  19     12.606  16.520 -60.844  1.00 22.71           O
ATOM  10048  CB  ALA G  19     11.065  16.912 -57.976  1.00 24.15           C
ATOM  10049  N   SER G  20     12.468  18.663 -60.185  1.00 22.56           N
ATOM  10050  CA  SER G  20     13.677  19.089 -60.854  1.00 23.27           C
ATOM  10051  C   SER G  20     14.367  20.134 -59.984  1.00 23.70           C
ATOM  10052  O   SER G  20     13.765  21.159 -59.630  1.00 25.30           O
ATOM  10053  CB  SER G  20     13.337  19.622 -62.253  1.00 24.59           C
ATOM  10054  OG  SER G  20     14.428  20.274 -62.856  1.00 24.10           O
ATOM  10055  N   ILE G  21     15.608  19.839 -59.604  1.00 22.42           N
ATOM  10056  CA  ILE G  21     16.417  20.692 -58.733  1.00 22.11           C
ATOM  10057  C   ILE G  21     17.646  21.151 -59.495  1.00 21.50           C
ATOM  10058  O   ILE G  21     18.336  20.344 -60.071  1.00 19.92           O
ATOM  10059  CB  ILE G  21     16.848  19.898 -57.483  1.00 22.06           C
ATOM  10060  CG1 ILE G  21     15.614  19.549 -56.639  1.00 24.57           C
ATOM  10061  CG2 ILE G  21     17.839  20.685 -56.610  1.00 23.11           C
ATOM  10062  CD1 ILE G  21     15.871  18.438 -55.674  1.00 26.18           C
ATOM  10063  N   SER G  22     17.913  22.451 -59.471  1.00 22.12           N
```

```
ATOM  10064  CA   SER G  22    19.011  23.058 -60.222  1.00 21.76          C
ATOM  10065  C    SER G  22    20.175  23.440 -59.330  1.00 22.18          C
ATOM  10066  O    SER G  22    20.013  23.680 -58.133  1.00 21.41          O
ATOM  10067  CB   SER G  22    18.523  24.341 -60.891  1.00 22.31          C
ATOM  10068  OG   SER G  22    17.369  24.096 -61.656  1.00 27.21          O
ATOM  10069  N    CYS G  23    21.349  23.529 -59.929  1.00 20.18          N
ATOM  10070  CA   CYS G  23    22.511  24.047 -59.241  1.00 20.88          C
ATOM  10071  C    CYS G  23    23.383  24.749 -60.295  1.00 19.95          C
ATOM  10072  O    CYS G  23    23.303  24.428 -61.480  1.00 18.75          O
ATOM  10073  CB   CYS G  23    23.247  22.892 -58.524  1.00 21.05          C
ATOM  10074  SG   CYS G  23    24.883  23.285 -57.806  1.00 22.66          S
ATOM  10075  N    ARG G  24    24.177  25.727 -59.881  1.00 20.09          N
ATOM  10076  CA   ARG G  24    25.205  26.264 -60.760  1.00 21.95          C
ATOM  10077  C    ARG G  24    26.512  26.414 -59.989  1.00 20.53          C
ATOM  10078  O    ARG G  24    26.498  26.578 -58.767  1.00 18.88          O
ATOM  10079  CB   ARG G  24    24.780  27.593 -61.396  1.00 23.90          C
ATOM  10080  CG   ARG G  24    24.334  28.670 -60.425  1.00 25.62          C
ATOM  10081  CD   ARG G  24    23.836  29.954 -61.161  1.00 28.22          C
ATOM  10082  NE   ARG G  24    23.538  31.056 -60.231  1.00 30.36          N
ATOM  10083  CZ   ARG G  24    24.375  32.054 -59.939  1.00 32.30          C
ATOM  10084  NH1  ARG G  24    25.565  32.169 -60.529  1.00 34.11          N
ATOM  10085  NH2  ARG G  24    24.008  32.973 -59.057  1.00 33.70          N
ATOM  10086  N    SER G  25    27.634  26.319 -60.711  1.00 18.84          N
ATOM  10087  CA   SER G  25    28.957  26.509 -60.139  1.00 19.19          C
ATOM  10088  C    SER G  25    29.590  27.742 -60.722  1.00 19.70          C
ATOM  10089  O    SER G  25    29.313  28.106 -61.886  1.00 20.12          O
ATOM  10090  CB   SER G  25    29.858  25.299 -60.389  1.00 19.88          C
ATOM  10091  OG   SER G  25    29.958  25.008 -61.765  1.00 20.72          O
ATOM  10092  N    SER G  26    30.443  28.385 -59.924  1.00 20.25          N
ATOM  10093  CA   SER G  26    31.147  29.604 -60.337  1.00 20.01          C
ATOM  10094  C    SER G  26    32.349  29.339 -61.238  1.00 22.05          C
ATOM  10095  O    SER G  26    32.892  30.263 -61.834  1.00 22.53          O
ATOM  10096  CB   SER G  26    31.593  30.425 -59.126  1.00 20.12          C
ATOM  10097  OG   SER G  26    32.507  29.718 -58.318  1.00 17.90          O
ATOM  10098  N    LYS G  27    32.767  28.081 -61.327  1.00 21.64          N
ATOM  10099  CA   LYS G  27    33.855  27.671 -62.193  1.00 23.15          C
ATOM  10100  C    LYS G  27    33.397  26.390 -62.856  1.00 21.13          C
ATOM  10101  O    LYS G  27    32.607  25.641 -62.276  1.00 20.61          O
ATOM  10102  CB   LYS G  27    35.112  27.352 -61.392  1.00 24.06          C
ATOM  10103  CG   LYS G  27    35.741  28.517 -60.653  1.00 26.83          C
ATOM  10104  CD   LYS G  27    37.186  28.152 -60.302  1.00 28.33          C
ATOM  10105  CE   LYS G  27    37.762  29.023 -59.202  1.00 30.72          C
ATOM  10106  NZ   LYS G  27    38.871  28.293 -58.477  1.00 32.89          N
ATOM  10107  N    SER G  27A   33.883  26.136 -64.061  1.00 18.96          N
ATOM  10108  CA   SER G  27A   33.496  24.920 -64.762  1.00 17.56          C
ATOM  10109  C    SER G  27A   34.047  23.711 -64.016  1.00 17.29          C
ATOM  10110  O    SER G  27A   35.224  23.705 -63.605  1.00 18.96          O
ATOM  10111  CB   SER G  27A   34.025  24.914 -66.203  1.00 17.71          C
ATOM  10112  OG   SER G  27A   33.688  23.702 -66.861  1.00 17.73          O
ATOM  10113  N    LEU G  27B   33.189  22.702 -63.862  1.00 15.41          N
ATOM  10114  CA   LEU G  27B   33.539  21.419 -63.292  1.00 15.55          C
ATOM  10115  C    LEU G  27B   33.857  20.344 -64.325  1.00 16.24          C
ATOM  10116  O    LEU G  27B   34.102  19.182 -63.944  1.00 16.48          O
ATOM  10117  CB   LEU G  27B   32.406  20.933 -62.385  1.00 14.95          C
ATOM  10118  CG   LEU G  27B   31.970  21.970 -61.334  1.00 15.94          C
ATOM  10119  CD1  LEU G  27B   30.900  21.364 -60.454  1.00 15.49          C
ATOM  10120  CD2  LEU G  27B   33.097  22.486 -60.476  1.00 15.62          C
ATOM  10121  N    LEU G  27C   33.878  20.730 -65.607  1.00 17.92          N
ATOM  10122  CA   LEU G  27C   34.245  19.822 -66.699  1.00 18.84          C
ATOM  10123  C    LEU G  27C   35.771  19.819 -66.783  1.00 18.94          C
ATOM  10124  O    LEU G  27C   36.404  20.870 -66.972  1.00 19.23          O
ATOM  10125  CB   LEU G  27C   33.613  20.255 -68.041  1.00 18.57          C
ATOM  10126  CG   LEU G  27C   34.076  19.487 -69.287  1.00 18.51          C
ATOM  10127  CD1  LEU G  27C   33.773  18.000 -69.127  1.00 19.45          C
ATOM  10128  CD2  LEU G  27C   33.405  20.046 -70.548  1.00 20.17          C
```

414

```
ATOM  10129  N    HIS G  27D    36.330  18.640 -66.554  1.00 20.19        N
ATOM  10130  CA   HIS G  27D    37.751  18.400 -66.449  1.00 21.72        C
ATOM  10131  C    HIS G  27D    38.317  17.991 -67.826  1.00 22.21        C
ATOM  10132  O    HIS G  27D    37.590  17.525 -68.701  1.00 22.07        O
ATOM  10133  CB   HIS G  27D    37.967  17.271 -65.436  1.00 22.29        C
ATOM  10134  CG   HIS G  27D    39.393  17.083 -65.030  1.00 23.10        C
ATOM  10135  ND1  HIS G  27D    40.297  16.382 -65.793  1.00 23.99        N
ATOM  10136  CD2  HIS G  27D    40.075  17.524 -63.947  1.00 23.89        C
ATOM  10137  CE1  HIS G  27D    41.470  16.376 -65.187  1.00 24.74        C
ATOM  10138  NE2  HIS G  27D    41.367  17.076 -64.070  1.00 23.11        N
ATOM  10139  N    SER G  27E    39.619  18.148 -68.027  1.00 24.83        N
ATOM  10140  CA   SER G  27E    40.215  17.765 -69.307  1.00 24.91        C
ATOM  10141  C    SER G  27E    40.092  16.263 -69.634  1.00 25.02        C
ATOM  10142  O    SER G  27E    40.223  15.866 -70.814  1.00 25.14        O
ATOM  10143  CB   SER G  27E    41.682  18.209 -69.359  1.00 25.69        C
ATOM  10144  OG   SER G  27E    42.359  17.744 -68.217  1.00 28.87        O
ATOM  10145  N    ASP G  28     39.817  15.434 -68.625  1.00 22.11        N
ATOM  10146  CA   ASP G  28     39.608  14.005 -68.844  1.00 23.20        C
ATOM  10147  C    ASP G  28     38.195  13.693 -69.322  1.00 22.14        C
ATOM  10148  O    ASP G  28     37.849  12.529 -69.482  1.00 22.51        O
ATOM  10149  CB   ASP G  28     39.949  13.190 -67.587  1.00 23.06        C
ATOM  10150  CG   ASP G  28     38.955  13.392 -66.424  1.00 23.20        C
ATOM  10151  OD1  ASP G  28     37.913  14.038 -66.595  1.00 20.89        O
ATOM  10152  OD2  ASP G  28     39.237  12.879 -65.311  1.00 23.12        O
ATOM  10153  N    GLY G  29     37.373  14.729 -69.500  1.00 22.20        N
ATOM  10154  CA   GLY G  29     36.023  14.572 -70.055  1.00 20.49        C
ATOM  10155  C    GLY G  29     34.916  14.391 -69.024  1.00 19.56        C
ATOM  10156  O    GLY G  29     33.750  14.424 -69.374  1.00 18.27        O
ATOM  10157  N    ILE G  30     35.280  14.197 -67.759  1.00 18.29        N
ATOM  10158  CA   ILE G  30     34.303  13.991 -66.680  1.00 18.09        C
ATOM  10159  C    ILE G  30     33.874  15.348 -66.089  1.00 17.46        C
ATOM  10160  O    ILE G  30     34.704  16.237 -65.913  1.00 17.51        O
ATOM  10161  CB   ILE G  30     34.910  13.102 -65.591  1.00 18.98        C
ATOM  10162  CG1  ILE G  30     35.219  11.733 -66.179  1.00 ·19.15       C
ATOM  10163  CG2  ILE G  30     33.993  13.003 -64.370  1.00 18.16        C
ATOM  10164  CD1  ILE G  30     35.779  10.735 -65.196  1.00 19.61        C
ATOM  10165  N    THR G  31     32.580  15.504 -65.788  1.00 16.70        N
ATOM  10166  CA   THR G  31     32.115  16.690 -65.070  1.00 15.54        C
ATOM  10167  C    THR G  31     31.974  16.294 -63.597  1.00 14.53        C
ATOM  10168  O    THR G  31     31.202  15.404 -63.258  1.00 13.94        O
ATOM  10169  CB   THR G  31     30.782  17.273 -65.629  1.00 16.08        C
ATOM  10170  OG1  THR G  31     30.914  17.573 -67.034  1.00 16.11        O
ATOM  10171  CG2  THR G  31     30.425  18.543 -64.889  1.00 16.34        C
ATOM  10172  N    TYR G  32     32.764  16.944 -62.738  1.00 15.20        N
ATOM  10173  CA   TYR G  32     32.926  16.487 -61.365  1.00 16.37        C
ATOM  10174  C    TYR G  32     31.904  17.170 -60.459  1.00 15.23        C
ATOM  10175  O    TYR G  32     32.248  17.932 -59.549  1.00 15.24        O
ATOM  10176  CB   TYR G  32     34.373  16.678 -60.900  1.00 17.47        C
ATOM  10177  CG   TYR G  32     35.324  15.643 -61.469  1.00 18.04        C
ATOM  10178  CD1  TYR G  32     36.041  15.890 -62.642  1.00 17.10        C
ATOM  10179  CD2  TYR G  32     35.491  14.411 -60.848  1.00 17.99        C
ATOM  10180  CE1  TYR G  32     36.918  14.911 -63.172  1.00 17.34        C
ATOM  10181  CE2  TYR G  32     36.346  13.458 -61.350  1.00 17.37        C
ATOM  10182  CZ   TYR G  32     37.059  13.701 -62.525  1.00 18.18        C
ATOM  10183  OH   TYR G  32     37.922  12.721 -63.007  1.00 18.31        O
ATOM  10184  N    LEU G  33     30.644  16.847 -60.741  1.00 15.92        N
ATOM  10185  CA   LEU G  33     29.469  17.349 -60.043  1.00 15.14        C
ATOM  10186  C    LEU G  33     28.846  16.169 -59.280  1.00 14.19        C
ATOM  10187  O    LEU G  33     28.690  15.055 -59.816  1.00 14.09        O
ATOM  10188  CB   LEU G  33     28.498  17.955 -61.071  1.00 15.61        C
ATOM  10189  CG   LEU G  33     27.211  18.626 -60.611  1.00 14.29        C
ATOM  10190  CD1  LEU G  33     26.139  17.633 -60.202  1.00 14.69        C
ATOM  10191  CD2  LEU G  33     27.477  19.607 -59.471  1.00 15.13        C
ATOM  10192  N    TYR G  34     28.480  16.409 -58.026  1.00 14.17        N
ATOM  10193  CA   TYR G  34     27.942  15.361 -57.157  1.00 13.80        C
```

| ATOM | 10194 | C | TYR | G | 34 | 26.681 | 15.883 | -56.518 | 1.00 | 15.03 | C |
|------|-------|-----|-----|---|----|--------|--------|---------|------|-------|---|
| ATOM | 10195 | O | TYR | G | 34 | 26.563 | 17.081 | -56.254 | 1.00 | 13.31 | O |
| ATOM | 10196 | CB | TYR | G | 34 | 28.958 | 14.965 | -56.051 | 1.00 | 13.92 | C |
| ATOM | 10197 | CG | TYR | G | 34 | 30.239 | 14.327 | -56.548 | 1.00 | 14.07 | C |
| ATOM | 10198 | CD1 | TYR | G | 34 | 31.163 | 15.039 | -57.324 | 1.00 | 13.35 | C |
| ATOM | 10199 | CD2 | TYR | G | 34 | 30.545 | 13.007 | -56.219 | 1.00 | 13.26 | C |
| ATOM | 10200 | CE1 | TYR | G | 34 | 32.350 | 14.422 | -57.806 | 1.00 | 13.01 | C |
| ATOM | 10201 | CE2 | TYR | G | 34 | 31.726 | 12.401 | -56.680 | 1.00 | 13.71 | C |
| ATOM | 10202 | CZ | TYR | G | 34 | 32.597 | 13.096 | -57.480 | 1.00 | 14.13 | C |
| ATOM | 10203 | OH | TYR | G | 34 | 33.745 | 12.483 | -57.901 | 1.00 | 14.60 | O |
| ATOM | 10204 | N | TRP | G | 35 | 25.753 | 14.970 | -56.247 | 1.00 | 14.56 | N |
| ATOM | 10205 | CA | TRP | G | 35 | 24.554 | 15.278 | -55.495 | 1.00 | 15.01 | C |
| ATOM | 10206 | C | TRP | G | 35 | 24.502 | 14.401 | -54.252 | 1.00 | 16.39 | C |
| ATOM | 10207 | O | TRP | G | 35 | 24.722 | 13.186 | -54.335 | 1.00 | 14.94 | O |
| ATOM | 10208 | CB | TRP | G | 35 | 23.298 | 14.988 | -56.304 | 1.00 | 15.82 | C |
| ATOM | 10209 | CG | TRP | G | 35 | 23.044 | 15.919 | -57.481 | 1.00 | 16.38 | C |
| ATOM | 10210 | CD1 | TRP | G | 35 | 23.271 | 15.643 | -58.804 | 1.00 | 17.07 | C |
| ATOM | 10211 | CD2 | TRP | G | 35 | 22.468 | 17.244 | -57.441 | 1.00 | 15.58 | C |
| ATOM | 10212 | NE1 | TRP | G | 35 | 22.893 | 16.737 | -59.581 | 1.00 | 16.43 | N |
| ATOM | 10213 | CE2 | TRP | G | 35 | 22.417 | 17.723 | -58.767 | 1.00 | 17.45 | C |
| ATOM | 10214 | CE3 | TRP | G | 35 | 22.013 | 18.079 | -56.408 | 1.00 | 17.35 | C |
| ATOM | 10215 | CZ2 | TRP | G | 35 | 21.897 | 18.986 | -59.089 | 1.00 | 15.24 | C |
| ATOM | 10216 | CZ3 | TRP | G | 35 | 21.517 | 19.324 | -56.722 | 1.00 | 17.80 | C |
| ATOM | 10217 | CH2 | TRP | G | 35 | 21.446 | 19.762 | -58.060 | 1.00 | 17.20 | C |
| ATOM | 10218 | N | TYR | G | 36 | 24.131 | 15.023 | -53.133 | 1.00 | 15.47 | N |
| ATOM | 10219 | CA | TYR | G | 36 | 23.862 | 14.325 | -51.864 | 1.00 | 16.65 | C |
| ATOM | 10220 | C | TYR | G | 36 | 22.423 | 14.590 | -51.408 | 1.00 | 16.35 | C |
| ATOM | 10221 | O | TYR | G | 36 | 21.881 | 15.655 | -51.679 | 1.00 | 15.70 | O |
| ATOM | 10222 | CB | TYR | G | 36 | 24.876 | 14.787 | -50.788 | 1.00 | 16.17 | C |
| ATOM | 10223 | CG | TYR | G | 36 | 26.310 | 14.416 | -51.125 | 1.00 | 16.46 | C |
| ATOM | 10224 | CD1 | TYR | G | 36 | 26.850 | 13.193 | -50.758 | 1.00 | 13.80 | C |
| ATOM | 10225 | CD2 | TYR | G | 36 | 27.126 | 15.306 | -51.816 | 1.00 | 14.55 | C |
| ATOM | 10226 | CE1 | TYR | G | 36 | 28.146 | 12.861 | -51.101 | 1.00 | 14.14 | C |
| ATOM | 10227 | CE2 | TYR | G | 36 | 28.414 | 14.989 | -52.151 | 1.00 | 16.48 | C |
| ATOM | 10228 | CZ | TYR | G | 36 | 28.933 | 13.784 | -51.796 | 1.00 | 15.43 | C |
| ATOM | 10229 | OH | TYR | G | 36 | 30.224 | 13.529 | -52.168 | 1.00 | 16.85 | O |
| ATOM | 10230 | N | LEU | G | 37 | 21.790 | 13.611 | -50.745 | 1.00 | 16.26 | N |
| ATOM | 10231 | CA | LEU | G | 37 | 20.495 | 13.825 | -50.090 | 1.00 | 15.65 | C |
| ATOM | 10232 | C | LEU | G | 37 | 20.699 | 13.736 | -48.587 | 1.00 | 15.63 | C |
| ATOM | 10233 | O | LEU | G | 37 | 21.300 | 12.798 | -48.124 | 1.00 | 15.29 | O |
| ATOM | 10234 | CB | LEU | G | 37 | 19.458 | 12.765 | -50.510 | 1.00 | 16.21 | C |
| ATOM | 10235 | CG | LEU | G | 37 | 18.091 | 12.756 | -49.812 | 1.00 | 15.43 | C |
| ATOM | 10236 | CD1 | LEU | G | 37 | 17.409 | 14.104 | -49.838 | 1.00 | 15.76 | C |
| ATOM | 10237 | CD2 | LEU | G | 37 | 17.138 | 11.731 | -50.427 | 1.00 | 17.57 | C |
| ATOM | 10238 | N | GLN | G | 38 | 20.200 | 14.721 | -47.859 | 1.00 | 16.59 | N |
| ATOM | 10239 | CA | GLN | G | 38 | 20.103 | 14.634 | -46.401 | 1.00 | 17.80 | C |
| ATOM | 10240 | C | GLN | G | 38 | 18.637 | 14.571 | -46.010 | 1.00 | 18.58 | C |
| ATOM | 10241 | O | GLN | G | 38 | 17.934 | 15.597 | -46.025 | 1.00 | 17.13 | O |
| ATOM | 10242 | CB | GLN | G | 38 | 20.742 | 15.836 | -45.735 | 1.00 | 18.37 | C |
| ATOM | 10243 | CG | GLN | G | 38 | 20.730 | 15.751 | -44.206 | 1.00 | 19.60 | C |
| ATOM | 10244 | CD | GLN | G | 38 | 21.696 | 16.731 | -43.590 | 1.00 | 19.36 | C |
| ATOM | 10245 | OE1 | GLN | G | 38 | 21.857 | 17.836 | -44.088 | 1.00 | 22.76 | O |
| ATOM | 10246 | NE2 | GLN | G | 38 | 22.367 | 16.316 | -42.516 | 1.00 | 21.49 | N |
| ATOM | 10247 | N | LYS | G | 39 | 18.185 | 13.370 | -45.678 | 1.00 | 21.18 | N |
| ATOM | 10248 | CA | LYS | G | 39 | 16.813 | 13.143 | -45.234 | 1.00 | 24.95 | C |
| ATOM | 10249 | C | LYS | G | 39 | 16.665 | 13.672 | -43.825 | 1.00 | 26.49 | C |
| ATOM | 10250 | O | LYS | G | 39 | 17.646 | 13.690 | -43.074 | 1.00 | 25.17 | O |
| ATOM | 10251 | CB | LYS | G | 39 | 16.473 | 11.655 | -45.265 | 1.00 | 24.76 | C |
| ATOM | 10252 | CG | LYS | G | 39 | 16.482 | 11.055 | -46.661 | 1.00 | 26.28 | C |
| ATOM | 10253 | CD | LYS | G | 39 | 16.211 | 9.564 | -46.612 | 1.00 | 26.60 | C |
| ATOM | 10254 | CE | LYS | G | 39 | 16.159 | 8.983 | -48.012 | 1.00 | 27.73 | C |
| ATOM | 10255 | NZ | LYS | G | 39 | 15.396 | 7.700 | -48.082 | 1.00 | 31.63 | N |
| ATOM | 10256 | N | PRO | G | 40 | 15.450 | 14.148 | -43.465 | 1.00 | 29.26 | N |
| ATOM | 10257 | CA | PRO | G | 40 | 15.328 | 14.746 | -42.131 | 1.00 | 30.30 | C |
| ATOM | 10258 | C | PRO | G | 40 | 15.845 | 13.797 | -41.040 | 1.00 | 30.47 | C |

```
ATOM  10259  O    PRO G  40    15.539  12.600 -41.059  1.00 30.87        O
ATOM  10260  CB   PRO G  40    13.820  15.048 -41.988  1.00 30.42        C
ATOM  10261  CG   PRO G  40    13.143  14.371 -43.183  1.00 30.94        C
ATOM  10262  CD   PRO G  40    14.191  14.220 -44.237  1.00 29.49        C
ATOM  10263  N    GLY G  41    16.692  14.326 -40.156  1.00 30.76        N
ATOM  10264  CA   GLY G  41    17.262  13.541 -39.068  1.00 31.34        C
ATOM  10265  C    GLY G  41    18.472  12.672 -39.376  1.00 31.22        C
ATOM  10266  O    GLY G  41    19.041  12.082 -38.444  1.00 31.41        O
ATOM  10267  N    GLN G  42    18.864  12.579 -40.658  1.00 29.14        N
ATOM  10268  CA   GLN G  42    19.968  11.729 -41.092  1.00 28.58        C
ATOM  10269  C    GLN G  42    21.198  12.553 -41.500  1.00 25.51        C
ATOM  10270  O    GLN G  42    21.144  13.772 -41.611  1.00 22.93        O
ATOM  10271  CB   GLN G  42    19.558  10.847 -42.295  1.00 30.23        C
ATOM  10272  CG   GLN G  42    18.377   9.908 -42.085  1.00 32.07        C
ATOM  10273  CD   GLN G  42    18.215   8.848 -43.220  1.00 33.55        C
ATOM  10274  OE1  GLN G  42    18.968   8.829 -44.224  1.00 37.34        O
ATOM  10275  NE2  GLN G  42    17.217   7.974 -43.063  1.00 37.05        N
ATOM  10276  N    SER G  43    22.297  11.849 -41.729  1.00 23.18        N
ATOM  10277  CA   SER G  43    23.477  12.397 -42.363  1.00 22.37        C
ATOM  10278  C    SER G  43    23.222  12.523 -43.854  1.00 20.55        C
ATOM  10279  O    SER G  43    22.300  11.900 -44.357  1.00 20.38        O
ATOM  10280  CB   SER G  43    24.652  11.452 -42.169  1.00 22.60        C
ATOM  10281  OG   SER G  43    24.845  11.140 -40.801  1.00 25.17        O
ATOM  10282  N    PRO G  44    24.057  13.304 -44.569  1.00 19.66        N
ATOM  10283  CA   PRO G  44    24.004  13.266 -46.035  1.00 19.26        C
ATOM  10284  C    PRO G  44    24.394  11.895 -46.581  1.00 18.21        C
ATOM  10285  O    PRO G  44    25.181  11.165 -45.959  1.00 17.52        O
ATOM  10286  CB   PRO G  44    25.052  14.302 -46.439  1.00 19.63        C
ATOM  10287  CG   PRO G  44    25.194  15.201 -45.195  1.00 18.96        C
ATOM  10288  CD   PRO G  44    25.117  14.224 -44.103  1.00 19.25        C
ATOM  10289  N    HIS G  45    23.835  11.542 -47.732  1.00 18.69        N
ATOM  10290  CA   HIS G  45    24.329  10.405 -48.463  1.00 17.92        C
ATOM  10291  C    HIS G  45    24.378  10.673 -49.963  1.00 17.25        C
ATOM  10292  O    HIS G  45    23.589  11.441 -50.500  1.00 13.76        O
ATOM  10293  CB   HIS G  45    23.531   9.136 -48.158  1.00 18.88        C
ATOM  10294  CG   HIS G  45    22.164   9.105 -48.755  1.00 20.24        C
ATOM  10295  ND1  HIS G  45    21.055   9.614 -48.109  1.00 22.86        N
ATOM  10296  CD2  HIS G  45    21.717   8.596 -49.928  1.00 22.19        C
ATOM  10297  CE1  HIS G  45    19.986   9.432 -48.863  1.00 21.01        C
ATOM  10298  NE2  HIS G  45    20.362   8.822 -49.976  1.00 21.41        N
ATOM  10299  N    LEU G  46    25.343  10.023 -50.596  1.00 16.26        N
ATOM  10300  CA   LEU G  46    25.631  10.197 -52.020  1.00 15.81        C
ATOM  10301  C    LEU G  46    24.524   9.671 -52.890  1.00 17.25        C
ATOM  10302  O    LEU G  46    24.103   8.518 -52.751  1.00 16.24        O
ATOM  10303  CB   LEU G  46    26.928   9.476 -52.385  1.00 14.94        C
ATOM  10304  CG   LEU G  46    27.378   9.598 -53.844  1.00 15.15        C
ATOM  10305  CD1  LEU G  46    27.897  10.961 -54.151  1.00 14.05        C
ATOM  10306  CD2  LEU G  46    28.429   8.603 -54.064  1.00 14.77        C
ATOM  10307  N    LEU G  47    24.068  10.525 -53.800  1.00 17.18        N
ATOM  10308  CA   LEU G  47    23.064  10.141 -54.788  1.00 18.88        C
ATOM  10309  C    LEU G  47    23.659   9.897 -56.175  1.00 18.50        C
ATOM  10310  O    LEU G  47    23.342   8.910 -56.829  1.00 19.36        O
ATOM  10311  CB   LEU G  47    22.066  11.263 -54.960  1.00 19.80        C
ATOM  10312  CG   LEU G  47    20.738  11.369 -54.243  1.00 23.84        C
ATOM  10313  CD1  LEU G  47    19.946  12.361 -55.076  1.00 23.85        C
ATOM  10314  CD2  LEU G  47    20.008  10.044 -54.110  1.00 24.20        C
ATOM  10315  N    ILE G  48    24.460  10.859 -56.624  1.00 18.23        N
ATOM  10316  CA   ILE G  48    24.943  10.944 -57.992  1.00 18.38        C
ATOM  10317  C    ILE G  48    26.379  11.416 -57.969  1.00 16.29        C
ATOM  10318  O    ILE G  48    26.704  12.311 -57.217  1.00 17.50        O
ATOM  10319  CB   ILE G  48    24.160  11.994 -58.804  1.00 19.06        C
ATOM  10320  CG1  ILE G  48    22.673  11.668 -58.918  1.00 23.16        C
ATOM  10321  CG2  ILE G  48    24.805  12.207 -60.175  1.00 20.81        C
ATOM  10322  CD1  ILE G  48    22.374  10.425 -59.686  1.00 24.57        C
ATOM  10323  N    TYR G  49    27.254  10.789 -58.754  1.00 15.88        N
```

```
ATOM   10324  CA  TYR G  49      28.632  11.234 -58.868  1.00 15.53           C
ATOM   10325  C   TYR G  49      28.974  11.425 -60.339  1.00 16.08           C
ATOM   10326  O   TYR G  49      28.324  10.852 -61.201  1.00 16.27           O
ATOM   10327  CB  TYR G  49      29.609  10.226 -58.248  1.00 17.58           C
ATOM   10328  CG  TYR G  49      29.643   8.850 -58.914  1.00 18.26           C
ATOM   10329  CD1 TYR G  49      28.731   7.870 -58.560  1.00 20.06           C
ATOM   10330  CD2 TYR G  49      30.597   8.538 -59.904  1.00 17.87           C
ATOM   10331  CE1 TYR G  49      28.737   6.612 -59.173  1.00 19.33           C
ATOM   10332  CE2 TYR G  49      30.611   7.273 -60.521  1.00 19.24           C
ATOM   10333  CZ  TYR G  49      29.679   6.322 -60.145  1.00 21.03           C
ATOM   10334  OH  TYR G  49      29.686   5.053 -60.729  1.00 21.41           O
ATOM   10335  N   HIS G  50      29.998  12.225 -60.603  1.00 15.98           N
ATOM   10336  CA  HIS G  50      30.463  12.488 -61.958  1.00 15.87           C
ATOM   10337  C   HIS G  50      29.314  12.945 -62.857  1.00 16.39           C
ATOM   10338  O   HIS G  50      29.182  12.470 -64.000  1.00 15.27           O
ATOM   10339  CB  HIS G  50      31.158  11.254 -62.533  1.00 17.26           C
ATOM   10340  CG  HIS G  50      32.456  10.919 -61.862  1.00 18.43           C
ATOM   10341  ND1 HIS G  50      33.209   9.822 -62.218  1.00 19.82           N
ATOM   10342  CD2 HIS G  50      33.154  11.552 -60.887  1.00 18.63           C
ATOM   10343  CE1 HIS G  50      34.305   9.781 -61.479  1.00 20.93           C
ATOM   10344  NE2 HIS G  50      34.296  10.823 -60.664  1.00 19.92           N
ATOM   10345  N   LEU G  51      28.496  13.856 -62.312  1.00 15.31           N
ATOM   10346  CA  LEU G  51      27.341  14.491 -62.995  1.00 16.04           C
ATOM   10347  C   LEU G  51      26.128  13.583 -63.260  1.00 16.15           C
ATOM   10348  O   LEU G  51      25.009  14.013 -63.081  1.00 16.90           O
ATOM   10349  CB  LEU G  51      27.794  15.192 -64.302  1.00 16.46           C
ATOM   10350  CG  LEU G  51      26.731  15.999 -65.034  1.00 15.57           C
ATOM   10351  CD1 LEU G  51      26.294  17.208 -64.208  1.00 16.89           C
ATOM   10352  CD2 LEU G  51      27.168  16.468 -66.412  1.00 16.42           C
ATOM   10353  N   SER G  52      26.327  12.342 -63.699  1.00 16.72           N
ATOM   10354  CA  SER G  52      25.233  11.560 -64.222  1.00 18.48           C
ATOM   10355  C   SER G  52      25.169  10.115 -63.740  1.00 18.56           C
ATOM   10356  O   SER G  52      24.213   9.408 -64.069  1.00 20.43           O
ATOM   10357  CB  SER G  52      25.278  11.631 -65.761  1.00 18.83           C
ATOM   10358  OG  SER G  52      25.076  12.982 -66.178  1.00 18.70           O
ATOM   10359  N   ASN G  53      26.150   9.673 -62.956  1.00 18.61           N
ATOM   10360  CA  ASN G  53      26.171   8.278 -62.506  1.00 20.51           C
ATOM   10361  C   ASN G  53      25.484   8.089 -61.175  1.00 21.37           C
ATOM   10362  O   ASN G  53      25.742   8.824 -60.234  1.00 19.96           O
ATOM   10363  CB  ASN G  53      27.589   7.768 -62.426  1.00 20.46           C
ATOM   10364  CG  ASN G  53      28.301   7.882 -63.739  1.00 23.10           C
ATOM   10365  OD1 ASN G  53      27.708   7.636 -64.775  1.00 28.60           O
ATOM   10366  ND2 ASN G  53      29.555   8.270 -63.714  1.00 25.97           N
ATOM   10367  N   LEU G  54      24.614   7.083 -61.106  1.00 21.79           N
ATOM   10368  CA  LEU G  54      23.850   6.823 -59.899  1.00 23.05           C
ATOM   10369  C   LEU G  54      24.730   6.061 -58.921  1.00 22.18           C
ATOM   10370  O   LEU G  54      25.411   5.116 -59.291  1.00 21.06           O
ATOM   10371  CB  LEU G  54      22.560   6.039 -60.203  1.00 23.33           C
ATOM   10372  CG  LEU G  54      21.371   6.909 -60.604  1.00 25.35           C
ATOM   10373  CD1 LEU G  54      21.642   7.614 -61.939  1.00 26.79           C
ATOM   10374  CD2 LEU G  54      20.081   6.102 -60.628  1.00 26.13           C
ATOM   10375  N   ALA G  55      24.714   6.480 -57.662  1.00 22.63           N
ATOM   10376  CA  ALA G  55      25.394   5.729 -56.622  1.00 23.43           C
ATOM   10377  C   ALA G  55      24.683   4.394 -56.417  1.00 24.49           C
ATOM   10378  O   ALA G  55      23.490   4.262 -56.651  1.00 23.81           O
ATOM   10379  CB  ALA G  55      25.432   6.513 -55.321  1.00 23.32           C
ATOM   10380  N   SER G  56      25.444   3.418 -55.958  1.00 27.12           N
ATOM   10381  CA  SER G  56      24.936   2.074 -55.736  1.00 29.37           C
ATOM   10382  C   SER G  56      23.655   2.107 -54.875  1.00 29.16           C
ATOM   10383  O   SER G  56      23.580   2.796 -53.861  1.00 29.33           O
ATOM   10384  CB  SER G  56      26.048   1.213 -55.114  1.00 30.26           C
ATOM   10385  OG  SER G  56      25.639  -0.135 -54.976  1.00 35.00           O
ATOM   10386  N   GLY G  57      22.621   1.408 -55.327  1.00 28.72           N
ATOM   10387  CA  GLY G  57      21.347   1.379 -54.619  1.00 27.62           C
ATOM   10388  C   GLY G  57      20.408   2.557 -54.801  1.00 27.31           C
```

```
ATOM  10389  O    GLY G  57     19.297   2.537 -54.267  1.00 26.87      O
ATOM  10390  N    VAL G  58     20.825   3.591 -55.538  1.00 25.56      N
ATOM  10391  CA   VAL G  58     19.923   4.709 -55.845  1.00 24.79      C
ATOM  10392  C    VAL G  58     18.934   4.287 -56.938  1.00 26.03      C
ATOM  10393  O    VAL G  58     19.347   3.727 -57.938  1.00 26.05      O
ATOM  10394  CB   VAL G  58     20.672   5.973 -56.322  1.00 23.64      C
ATOM  10395  CG1  VAL G  58     19.661   7.084 -56.659  1.00 21.41      C
ATOM  10396  CG2  VAL G  58     21.636   6.438 -55.247  1.00 23.69      C
ATOM  10397  N    PRO G  59     17.629   4.514 -56.723  1.00 27.80      N
ATOM  10398  CA   PRO G  59     16.644   4.132 -57.734  1.00 28.54      C
ATOM  10399  C    PRO G  59     16.784   4.885 -59.059  1.00 29.46      C
ATOM  10400  O    PRO G  59     17.219   6.034 -59.080  1.00 29.37      O
ATOM  10401  CB   PRO G  59     15.314   4.469 -57.069  1.00 28.26      C
ATOM  10402  CG   PRO G  59     15.585   4.516 -55.646  1.00 28.83      C
ATOM  10403  CD   PRO G  59     16.971   5.044 -55.517  1.00 28.26      C
ATOM  10404  N    ASP G  60     16.383   4.245 -60.159  1.00 29.94      N
ATOM  10405  CA   ASP G  60     16.526   4.862 -61.482  1.00 29.83      C
ATOM  10406  C    ASP G  60     15.590   6.041 -61.712  1.00 27.33      C
ATOM  10407  O    ASP G  60     15.687   6.697 -62.742  1.00 27.56      O
ATOM  10408  CB   ASP G  60     16.370   3.829 -62.608  1.00 31.98      C
ATOM  10409  CG   ASP G  60     15.138   2.982 -62.450  1.00 35.36      C
ATOM  10410  OD1  ASP G  60     14.071   3.536 -62.094  1.00 37.99      O
ATOM  10411  OD2  ASP G  60     15.246   1.748 -62.665  1.00 39.43      O
ATOM  10412  N    ARG G  61     14.700   6.312 -60.757  1.00 25.12      N
ATOM  10413  CA   ARG G  61     13.893   7.535 -60.784  1.00 24.59      C
ATOM  10414  C    ARG G  61     14.774   8.795 -60.712  1.00 22.11      C
ATOM  10415  O    ARG G  61     14.341   9.872 -61.108  1.00 22.00      O
ATOM  10416  CB   ARG G  61     12.901   7.550 -59.626  1.00 25.87      C
ATOM  10417  CG   ARG G  61     11.917   6.384 -59.632  1.00 28.59      C
ATOM  10418  CD   ARG G  61     10.933   6.451 -58.461  1.00 28.73      C
ATOM  10419  NE   ARG G  61     11.578   6.243 -57.158  1.00 30.54      N
ATOM  10420  CZ   ARG G  61     11.911   7.211 -56.299  1.00 30.11      C
ATOM  10421  NH1  ARG G  61     11.689   8.486 -56.584  1.00 30.07      N
ATOM  10422  NH2  ARG G  61     12.476   6.884 -55.150  1.00 30.38      N
ATOM  10423  N    PHE G  62     15.980   8.657 -60.154  1.00 21.15      N
ATOM  10424  CA   PHE G  62     16.943   9.770 -60.099  1.00 21.03      C
ATOM  10425  C    PHE G  62     17.812   9.802 -61.356  1.00 19.23      C
ATOM  10426  O    PHE G  62     18.335   8.780 -61.790  1.00 21.64      O
ATOM  10427  CB   PHE G  62     17.837   9.668 -58.842  1.00 21.26      C
ATOM  10428  CG   PHE G  62     17.070   9.811 -57.546  1.00 21.82      C
ATOM  10429  CD1  PHE G  62     16.504   8.700 -56.932  1.00 23.52      C
ATOM  10430  CD2  PHE G  62     16.893  11.059 -56.968  1.00 20.99      C
ATOM  10431  CE1  PHE G  62     15.775   8.844 -55.742  1.00 23.11      C
ATOM  10432  CE2  PHE G  62     16.165  11.206 -55.799  1.00 22.25      C
ATOM  10433  CZ   PHE G  62     15.609  10.103 -55.192  1.00 22.33      C
ATOM  10434  N    SER G  63     17.971  10.986 -61.921  1.00 17.13      N
ATOM  10435  CA   SER G  63     18.856  11.204 -63.050  1.00 17.03      C
ATOM  10436  C    SER G  63     19.436  12.607 -62.945  1.00 16.92      C
ATOM  10437  O    SER G  63     18.874  13.452 -62.260  1.00 16.68      O
ATOM  10438  CB   SER G  63     18.103  11.036 -64.379  1.00 16.58      C
ATOM  10439  OG   SER G  63     17.103  12.032 -64.586  1.00 17.02      O
ATOM  10440  N    SER G  64     20.530  12.870 -63.661  1.00 15.94      N
ATOM  10441  CA   SER G  64     21.165  14.174 -63.602  1.00 14.67      C
ATOM  10442  C    SER G  64     21.912  14.474 -64.881  1.00 15.80      C
ATOM  10443  O    SER G  64     22.486  13.581 -65.501  1.00 15.76      O
ATOM  10444  CB   SER G  64     22.130  14.232 -62.408  1.00 15.83      C
ATOM  10445  OG   SER G  64     22.845  15.467 -62.340  1.00 15.21      O
ATOM  10446  N    SER G  65     21.940  15.755 -65.240  1.00 15.80      N
ATOM  10447  CA   SER G  65     22.545  16.218 -66.463  1.00 15.63      C
ATOM  10448  C    SER G  65     23.125  17.575 -66.200  1.00 15.49      C
ATOM  10449  O    SER G  65     22.790  18.220 -65.203  1.00 16.40      O
ATOM  10450  CB   SER G  65     21.514  16.322 -67.587  1.00 16.19      C
ATOM  10451  OG   SER G  65     20.548  17.339 -67.317  1.00 16.48      O
ATOM  10452  N    GLY G  66     24.021  18.005 -67.078  1.00 14.16      N
ATOM  10453  CA   GLY G  66     24.518  19.372 -67.016  1.00 14.53      C
```

| ATOM | 10454 | C   | GLY  | G | 66 | 25.729 | 19.690 | -67.872 | 1.00 | 14.71 | C |
| ATOM | 10455 | O   | GLY  | G | 66 | 26.320 | 18.815 | -68.473 | 1.00 | 16.65 | O |
| ATOM | 10456 | N   | SER  | G | 67 | 26.057 | 20.978 | -67.911 | 1.00 | 15.73 | N |
| ATOM | 10457 | CA  | SER  | G | 67 | 27.258 | 21.511 | -68.531 | 1.00 | 16.88 | C |
| ATOM | 10458 | C   | SER  | G | 67 | 28.394 | 21.465 | -67.496 | 1.00 | 16.84 | C |
| ATOM | 10459 | O   | SER  | G | 67 | 28.329 | 20.703 | -66.543 | 1.00 | 18.74 | O |
| ATOM | 10460 | CB  | SER  | G | 67 | 27.002 | 22.941 | -69.026 | 1.00 | 16.84 | C |
| ATOM | 10461 | OG  | SER  | G | 67 | 26.738 | 23.822 | -67.975 | 1.00 | 15.65 | O |
| ATOM | 10462 | N   | GLY  | G | 68 | 29.454 | 22.219 | -67.714 | 1.00 | 17.83 | N |
| ATOM | 10463 | CA  | GLY  | G | 68 | 30.468 | 22.428 | -66.673 | 1.00 | 16.45 | C |
| ATOM | 10464 | C   | GLY  | G | 68 | 29.986 | 23.309 | -65.528 | 1.00 | 17.82 | C |
| ATOM | 10465 | O   | GLY  | G | 68 | 30.594 | 23.308 | -64.453 | 1.00 | 17.25 | O |
| ATOM | 10466 | N   | THR  | G | 69 | 28.906 | 24.068 | -65.760 | 1.00 | 16.75 | N |
| ATOM | 10467 | CA  | THR  | G | 69 | 28.475 | 25.135 | -64.837 | 1.00 | 16.98 | C |
| ATOM | 10468 | C   | THR  | G | 69 | 27.015 | 25.150 | -64.401 | 1.00 | 16.03 | C |
| ATOM | 10469 | O   | THR  | G | 69 | 26.687 | 25.848 | -63.448 | 1.00 | 18.45 | O |
| ATOM | 10470 | CB  | THR  | G | 69 | 28.741 | 26.515 | -65.414 | 1.00 | 17.94 | C |
| ATOM | 10471 | OG1 | THR  | G | 69 | 27.967 | 26.678 | -66.611 | 1.00 | 18.87 | O |
| ATOM | 10472 | CG2 | THR  | G | 69 | 30.235 | 26.707 | -65.714 | 1.00 | 19.62 | C |
| ATOM | 10473 | N   | ASP  | G | 70 | 26.147 | 24.407 | -65.091 | 1.00 | 17.57 | N |
| ATOM | 10474 | CA  | ASP  | G | 70 | 24.712 | 24.386 | -64.807 | 1.00 | 16.56 | C |
| ATOM | 10475 | C   | ASP  | G | 70 | 24.230 | 22.963 | -64.849 | 1.00 | 16.63 | C |
| ATOM | 10476 | O   | ASP  | G | 70 | 24.498 | 22.243 | -65.800 | 1.00 | 15.54 | O |
| ATOM | 10477 | CB  | ASP  | G | 70 | 23.933 | 25.234 | -65.820 | 1.00 | 19.19 | C |
| ATOM | 10478 | CG  | ASP  | G | 70 | 24.314 | 26.684 | -65.766 | 1.00 | 22.04 | C |
| ATOM | 10479 | OD1 | ASP  | G | 70 | 23.771 | 27.420 | -64.892 | 1.00 | 22.54 | O |
| ATOM | 10480 | OD2 | ASP  | G | 70 | 25.147 | 27.091 | -66.616 | 1.00 | 24.38 | O |
| ATOM | 10481 | N   | PHE  | G | 71 | 23.536 | 22.553 | -63.787 | 1.00 | 14.61 | N |
| ATOM | 10482 | CA  | PHE  | G | 71 | 23.219 | 21.141 | -63.555 | 1.00 | 15.59 | C |
| ATOM | 10483 | C   | PHE  | G | 71 | 21.764 | 20.997 | -63.094 | 1.00 | 15.21 | C |
| ATOM | 10484 | O   | PHE  | G | 71 | 21.204 | 21.898 | -62.457 | 1.00 | 17.21 | O |
| ATOM | 10485 | CB  | PHE  | G | 71 | 24.154 | 20.555 | -62.482 | 1.00 | 14.67 | C |
| ATOM | 10486 | CG  | PHE  | G | 71 | 25.534 | 21.161 | -62.456 | 1.00 | 14.79 | C |
| ATOM | 10487 | CD1 | PHE  | G | 71 | 26.501 | 20.810 | -63.418 | 1.00 | 15.51 | C |
| ATOM | 10488 | CD2 | PHE  | G | 71 | 25.882 | 22.084 | -61.474 | 1.00 | 14.43 | C |
| ATOM | 10489 | CE1 | PHE  | G | 71 | 27.787 | 21.349 | -63.377 | 1.00 | 14.19 | C |
| ATOM | 10490 | CE2 | PHE  | G | 71 | 27.153 | 22.633 | -61.445 | 1.00 | 15.41 | C |
| ATOM | 10491 | CZ  | PHE  | G | 71 | 28.103 | 22.278 | -62.407 | 1.00 | 15.65 | C |
| ATOM | 10492 | N   | THR  | G | 72 | 21.165 | 19.850 | -63.403 | 1.00 | 15.10 | N |
| ATOM | 10493 | CA  | THR  | G | 72 | 19.780 | 19.593 | -63.086 | 1.00 | 15.47 | C |
| ATOM | 10494 | C   | THR  | G | 72 | 19.645 | 18.154 | -62.604 | 1.00 | 14.61 | C |
| ATOM | 10495 | O   | THR  | G | 72 | 19.978 | 17.207 | -63.322 | 1.00 | 15.57 | O |
| ATOM | 10496 | CB  | THR  | G | 72 | 18.847 | 19.797 | -64.324 | 1.00 | 16.07 | C |
| ATOM | 10497 | OG1 | THR  | G | 72 | 19.056 | 21.092 | -64.899 | 1.00 | 16.94 | O |
| ATOM | 10498 | CG2 | THR  | G | 72 | 17.414 | 19.654 | -63.935 | 1.00 | 16.98 | C |
| ATOM | 10499 | N   | LEU  | G | 73 | 19.185 | 17.993 | -61.366 | 1.00 | 16.83 | N |
| ATOM | 10500 | CA  | LEU  | G | 73 | 18.740 | 16.696 | -60.857 | 1.00 | 16.50 | C |
| ATOM | 10501 | C   | LEU  | G | 73 | 17.240 | 16.556 | -61.100 | 1.00 | 17.11 | C |
| ATOM | 10502 | O   | LEU  | G | 73 | 16.472 | 17.442 | -60.712 | 1.00 | 19.08 | O |
| ATOM | 10503 | CB  | LEU  | G | 73 | 18.993 | 16.636 | -59.350 | 1.00 | 17.15 | C |
| ATOM | 10504 | CG  | LEU  | G | 73 | 18.547 | 15.399 | -58.591 | 1.00 | 17.11 | C |
| ATOM | 10505 | CD1 | LEU  | G | 73 | 19.324 | 14.192 | -58.975 | 1.00 | 17.12 | C |
| ATOM | 10506 | CD2 | LEU  | G | 73 | 18.717 | 15.664 | -57.087 | 1.00 | 17.99 | C |
| ATOM | 10507 | N   | ARG  | G | 74 | 16.832 | 15.434 | -61.678 | 1.00 | 17.52 | N |
| ATOM | 10508 | CA  | ARG  | G | 74 | 15.420 | 15.132 | -61.900 | 1.00 | 18.86 | C |
| ATOM | 10509 | C   | ARG  | G | 74 | 15.011 | 13.904 | -61.106 | 1.00 | 19.07 | C |
| ATOM | 10510 | O   | ARG  | G | 74 | 15.706 | 12.911 | -61.093 | 1.00 | 18.80 | O |
| ATOM | 10511 | CB  | AARG | G | 74 | 15.139 | 14.915 | -63.386 | 0.50 | 19.15 | C |
| ATOM | 10512 | CB  | BARG | G | 74 | 15.118 | 14.883 | -63.394 | 0.50 | 18.51 | C |
| ATOM | 10513 | CG  | AARG | G | 74 | 13.689 | 14.550 | -63.663 | 0.50 | 20.62 | C |
| ATOM | 10514 | CG  | BARG | G | 74 | 13.674 | 14.362 | -63.682 | 0.50 | 18.62 | C |
| ATOM | 10515 | CD  | AARG | G | 74 | 13.184 | 15.169 | -64.951 | 0.50 | 21.72 | C |
| ATOM | 10516 | CD  | BARG | G | 74 | 13.294 | 14.442 | -65.172 | 0.50 | 19.39 | C |
| ATOM | 10517 | NE  | AARG | G | 74 | 11.783 | 14.814 | -65.209 | 0.50 | 21.88 | N |
| ATOM | 10518 | NE  | BARG | G | 74 | 11.837 | 14.453 | -65.441 | 0.50 | 18.79 | N |

```
ATOM   10519  CZ AARG G  74      11.351  13.610 -65.592  0.50 21.44           C
ATOM   10520  CZ BARG G  74      11.047  15.539 -65.475  0.50 18.16           C
ATOM   10521  NH1AARG G  74      12.190  12.591 -65.774  0.50 20.84           N
ATOM   10522  NH1BARG G  74      11.512  16.754 -65.243  0.50 16.50           N
ATOM   10523  NH2AARG G  74      10.058  13.421 -65.796  0.50 21.76           N
ATOM   10524  NH2BARG G  74       9.754  15.407 -65.750  0.50 19.94           N
ATOM   10525  N   ILE G  75      13.868  13.984 -60.446  1.00 21.20           N
ATOM   10526  CA  ILE G  75      13.264  12.823 -59.822  1.00 22.86           C
ATOM   10527  C   ILE G  75      11.946  12.602 -60.560  1.00 24.86           C
ATOM   10528  O   ILE G  75      11.060  13.433 -60.473  1.00 25.59           O
ATOM   10529  CB  ILE G  75      12.978  13.063 -58.332  1.00 23.52           C
ATOM   10530  CG1 ILE G  75      14.204  13.671 -57.622  1.00 24.17           C
ATOM   10531  CG2 ILE G  75      12.587  11.765 -57.686  1.00 24.52           C
ATOM   10532  CD1 ILE G  75      13.918  14.129 -56.175  1.00 24.64           C
ATOM   10533  N   SER G  76      11.826  11.498 -61.283  1.00 27.44           N
ATOM   10534  CA  SER G  76      10.732  11.327 -62.252  1.00 29.88           C
ATOM   10535  C   SER G  76       9.329  11.166 -61.650  1.00 31.92           C
ATOM   10536  O   SER G  76       8.350  11.716 -62.193  1.00 33.87           O
ATOM   10537  CB  SER G  76      11.027  10.149 -63.185  1.00 29.68           C
ATOM   10538  OG  SER G  76      11.056   8.928 -62.484  1.00 29.69           O
ATOM   10539  N   ARG G  77       9.249  10.378 -60.576  1.00 32.60           N
ATOM   10540  CA  ARG G  77       7.999   9.998 -59.916  1.00 33.97           C
ATOM   10541  C   ARG G  77       8.245   9.989 -58.404  1.00 32.07           C
ATOM   10542  O   ARG G  77       8.543   8.951 -57.796  1.00 31.38           O
ATOM   10543  CB  ARG G  77       7.556   8.612 -60.364  1.00 34.37           C
ATOM   10544  CG  ARG G  77       7.171   8.549 -61.839  1.00 37.19           C
ATOM   10545  CD  ARG G  77       6.957   7.112 -62.320  1.00 38.87           C
ATOM   10546  NE  ARG G  77       8.105   6.231 -62.071  1.00 42.19           N
ATOM   10547  CZ  ARG G  77       9.247   6.221 -62.772  1.00 43.86           C
ATOM   10548  NH1 ARG G  77       9.451   7.066 -63.789  1.00 45.57           N
ATOM   10549  NH2 ARG G  77      10.209   5.360 -62.444  1.00 44.29           N
ATOM   10550  N   VAL G  78       8.126  11.164 -57.823  1.00 30.26           N
ATOM   10551  CA  VAL G  78       8.445  11.394 -56.418  1.00 29.76           C
ATOM   10552  C   VAL G  78       7.693  10.460 -55.444  1.00 29.21           C
ATOM   10553  O   VAL G  78       6.475  10.222 -55.568  1.00 27.71           O
ATOM   10554  CB  VAL G  78       8.196  12.879 -56.076  1.00 30.18           C
ATOM   10555  CG1 VAL G  78       8.031  13.092 -54.593  1.00 30.39           C
ATOM   10556  CG2 VAL G  78       9.351  13.728 -56.615  1.00 30.57           C
ATOM   10557  N   GLU G  79       8.454   9.930 -54.489  1.00 29.17           N
ATOM   10558  CA  GLU G  79       7.943   9.090 -53.399  1.00 29.04           C
ATOM   10559  C   GLU G  79       8.158   9.798 -52.058  1.00 27.86           C
ATOM   10560  O   GLU G  79       9.011  10.677 -51.942  1.00 25.62           O
ATOM   10561  CB  GLU G  79       8.643   7.735 -53.410  1.00 29.86           C
ATOM   10562  CG  GLU G  79       8.435   6.969 -54.713  1.00 31.88           C
ATOM   10563  CD  GLU G  79       8.962   5.538 -54.679  1.00 33.48           C
ATOM   10564  OE1 GLU G  79       9.396   5.086 -53.597  1.00 39.65           O
ATOM   10565  OE2 GLU G  79       8.919   4.849 -55.740  1.00 37.70           O
ATOM   10566  N   ALA G  80       7.361   9.430 -51.055  1.00 25.71           N
ATOM   10567  CA  ALA G  80       7.437  10.055 -49.744  1.00 24.85           C
ATOM   10568  C   ALA G  80       8.881  10.098 -49.210  1.00 23.01           C
ATOM   10569  O   ALA G  80       9.308  11.130 -48.683  1.00 21.32           O
ATOM   10570  CB  ALA G  80       6.504   9.327 -48.750  1.00 24.87           C
ATOM   10571  N   GLU G  81       9.606   8.988 -49.368  1.00 23.83           N
ATOM   10572  CA  GLU G  81      11.001   8.840 -48.884  1.00 25.51           C
ATOM   10573  C   GLU G  81      12.007   9.821 -49.510  1.00 24.40           C
ATOM   10574  O   GLU G  81      13.112  10.001 -48.993  1.00 23.37           O
ATOM   10575  CB  GLU G  81      11.528   7.412 -49.116  1.00 28.01           C
ATOM   10576  CG  GLU G  81      11.327   6.899 -50.566  1.00 33.31           C
ATOM   10577  CD  GLU G  81      12.540   6.197 -51.179  1.00 37.65           C
ATOM   10578  OE1 GLU G  81      12.486   4.956 -51.371  1.00 41.10           O
ATOM   10579  OE2 GLU G  81      13.534   6.892 -51.508  1.00 42.71           O
ATOM   10580  N   ASP G  82      11.637  10.455 -50.616  1.00 24.16           N
ATOM   10581  CA  ASP G  82      12.539  11.401 -51.284  1.00 23.49           C
ATOM   10582  C   ASP G  82      12.641  12.770 -50.597  1.00 22.96           C
ATOM   10583  O   ASP G  82      13.478  13.590 -50.990  1.00 20.65           O
```

| ATOM | 10584 | CB | ASP G | 82 | 12.088 | 11.617 | -52.722 | 1.00 | 23.56 | C |
|------|-------|-----|-------|----|--------|--------|---------|------|-------|---|
| ATOM | 10585 | CG | ASP G | 82 | 12.067 | 10.342 | -53.527 | 1.00 | 24.75 | C |
| ATOM | 10586 | OD1 | ASP G | 82 | 12.784 | 9.369 | -53.176 | 1.00 | 27.02 | O |
| ATOM | 10587 | OD2 | ASP G | 82 | 11.321 | 10.323 | -54.534 | 1.00 | 26.08 | O |
| ATOM | 10588 | N | VAL G | 83 | 11.810 | 13.036 | -49.580 | 1.00 | 21.28 | N |
| ATOM | 10589 | CA | VAL G | 83 | 11.864 | 14.350 | -48.923 | 1.00 | 21.69 | C |
| ATOM | 10590 | C | VAL G | 83 | 13.194 | 14.536 | -48.211 | 1.00 | 19.74 | C |
| ATOM | 10591 | O | VAL G | 83 | 13.784 | 13.600 | -47.711 | 1.00 | 19.12 | O |
| ATOM | 10592 | CB | VAL G | 83 | 10.721 | 14.626 | -47.899 | 1.00 | 22.59 | C |
| ATOM | 10593 | CG1 | VAL G | 83 | 9.404 | 14.859 | -48.614 | 1.00 | 26.00 | C |
| ATOM | 10594 | CG2 | VAL G | 83 | 10.619 | 13.532 | -46.843 | 1.00 | 22.59 | C |
| ATOM | 10595 | N | GLY G | 84 | 13.629 | 15.778 | -48.120 | 1.00 | 19.75 | N |
| ATOM | 10596 | CA | GLY G | 84 | 14.933 | 16.036 | -47.547 | 1.00 | 19.26 | C |
| ATOM | 10597 | C | GLY G | 84 | 15.537 | 17.198 | -48.266 | 1.00 | 19.12 | C |
| ATOM | 10598 | O | GLY G | 84 | 14.874 | 17.877 | -49.051 | 1.00 | 19.74 | O |
| ATOM | 10599 | N | ILE G | 85 | 16.800 | 17.442 | -47.961 | 1.00 | 18.66 | N |
| ATOM | 10600 | CA | ILE G | 85 | 17.552 | 18.502 | -48.595 | 1.00 | 18.26 | C |
| ATOM | 10601 | C | ILE G | 85 | 18.583 | 17.904 | -49.561 | 1.00 | 16.65 | C |
| ATOM | 10602 | O | ILE G | 85 | 19.375 | 17.059 | -49.199 | 1.00 | 16.80 | O |
| ATOM | 10603 | CB | ILE G | 85 | 18.197 | 19.405 | -47.532 | 1.00 | 19.60 | C |
| ATOM | 10604 | CG1 | ILE G | 85 | 17.096 | 20.024 | -46.667 | 1.00 | 21.28 | C |
| ATOM | 10605 | CG2 | ILE G | 85 | 18.989 | 20.531 | -48.180 | 1.00 | 19.12 | C |
| ATOM | 10606 | CD1 | ILE G | 85 | 17.575 | 20.363 | -45.325 | 1.00 | 25.00 | C |
| ATOM | 10607 | N | TYR G | 86 | 18.531 | 18.352 | -50.809 | 1.00 | 15.14 | N |
| ATOM | 10608 | CA | TYR G | 86 | 19.467 | 17.961 | -51.846 | 1.00 | 15.00 | C |
| ATOM | 10609 | C | TYR G | 86 | 20.604 | 18.979 | -51.979 | 1.00 | 15.07 | C |
| ATOM | 10610 | O | TYR G | 86 | 20.367 | 20.161 | -52.254 | 1.00 | 16.19 | O |
| ATOM | 10611 | CB | TYR G | 86 | 18.708 | 17.798 | -53.171 | 1.00 | 14.82 | C |
| ATOM | 10612 | CG | TYR G | 86 | 17.756 | 16.635 | -53.139 | 1.00 | 15.78 | C |
| ATOM | 10613 | CD1 | TYR G | 86 | 16.471 | 16.763 | -52.589 | 1.00 | 15.02 | C |
| ATOM | 10614 | CD2 | TYR G | 86 | 18.144 | 15.405 | -53.599 | 1.00 | 16.46 | C |
| ATOM | 10615 | CE1 | TYR G | 86 | 15.617 | 15.689 | -52.542 | 1.00 | 16.64 | C |
| ATOM | 10616 | CE2 | TYR G | 86 | 17.301 | 14.343 | -53.559 | 1.00 | 16.74 | C |
| ATOM | 10617 | CZ | TYR G | 86 | 16.045 | 14.477 | -53.016 | 1.00 | 16.78 | C |
| ATOM | 10618 | OH | TYR G | 86 | 15.251 | 13.368 | -52.980 | 1.00 | 16.43 | O |
| ATOM | 10619 | N | TYR G | 87 | 21.833 | 18.510 | -51.793 | 1.00 | 15.41 | N |
| ATOM | 10620 | CA | TYR G | 87 | 23.025 | 19.328 | -51.923 | 1.00 | 14.71 | C |
| ATOM | 10621 | C | TYR G | 87 | 23.848 | 18.955 | -53.136 | 1.00 | 14.40 | C |
| ATOM | 10622 | O | TYR G | 87 | 24.122 | 17.773 | -53.359 | 1.00 | 13.63 | O |
| ATOM | 10623 | CB | TYR G | 87 | 23.951 | 19.172 | -50.712 | 1.00 | 15.62 | C |
| ATOM | 10624 | CG | TYR G | 87 | 23.360 | 19.578 | -49.385 | 1.00 | 16.77 | C |
| ATOM | 10625 | CD1 | TYR G | 87 | 23.475 | 20.899 | -48.925 | 1.00 | 15.66 | C |
| ATOM | 10626 | CD2 | TYR G | 87 | 22.694 | 18.649 | -48.581 | 1.00 | 17.93 | C |
| ATOM | 10627 | CE1 | TYR G | 87 | 22.953 | 21.272 | -47.692 | 1.00 | 17.33 | C |
| ATOM | 10628 | CE2 | TYR G | 87 | 22.158 | 19.026 | -47.348 | 1.00 | 16.72 | C |
| ATOM | 10629 | CZ | TYR G | 87 | 22.299 | 20.343 | -46.923 | 1.00 | 18.52 | C |
| ATOM | 10630 | OH | TYR G | 87 | 21.768 | 20.751 | -45.720 | 1.00 | 19.63 | O |
| ATOM | 10631 | N | CYS G | 88 | 24.263 | 19.972 | -53.889 | 1.00 | 15.90 | N |
| ATOM | 10632 | CA | CYS G | 88 | 25.315 | 19.832 | -54.913 | 1.00 | 16.44 | C |
| ATOM | 10633 | C | CYS G | 88 | 26.718 | 20.109 | -54.347 | 1.00 | 15.60 | C |
| ATOM | 10634 | O | CYS G | 88 | 26.883 | 20.839 | -53.345 | 1.00 | 14.16 | O |
| ATOM | 10635 | CB | CYS G | 88 | 25.018 | 20.717 | -56.128 | 1.00 | 17.72 | C |
| ATOM | 10636 | SG | CYS G | 88 | 24.851 | 22.515 | -55.837 | 1.00 | 20.59 | S |
| ATOM | 10637 | N | ALA G | 89 | 27.729 | 19.505 | -54.984 | 1.00 | 15.31 | N |
| ATOM | 10638 | CA | ALA G | 89 | 29.117 | 19.633 | -54.573 | 1.00 | 13.91 | C |
| ATOM | 10639 | C | ALA G | 89 | 30.024 | 19.368 | -55.769 | 1.00 | 14.90 | C |
| ATOM | 10640 | O | ALA G | 89 | 29.626 | 18.663 | -56.686 | 1.00 | 16.23 | O |
| ATOM | 10641 | CB | ALA G | 89 | 29.448 | 18.649 | -53.438 | 1.00 | 14.62 | C |
| ATOM | 10642 | N | HIS G | 90 | 31.209 | 19.964 | -55.762 | 1.00 | 13.63 | N |
| ATOM | 10643 | CA | HIS G | 90 | 32.201 | 19.739 | -56.805 | 1.00 | 14.20 | C |
| ATOM | 10644 | C | HIS G | 90 | 33.347 | 18.874 | -56.269 | 1.00 | 14.84 | C |
| ATOM | 10645 | O | HIS G | 90 | 33.541 | 18.737 | -55.041 | 1.00 | 12.37 | O |
| ATOM | 10646 | CB | HIS G | 90 | 32.744 | 21.073 | -57.307 | 1.00 | 14.66 | C |
| ATOM | 10647 | CG | HIS G | 90 | 33.691 | 21.724 | -56.355 | 1.00 | 13.48 | C |
| ATOM | 10648 | ND1 | HIS G | 90 | 35.025 | 21.387 | -56.267 | 1.00 | 15.62 | N |

| ATOM | 10649 | CD2 | HIS | G | 90 | 33.480 | 22.671 | -55.422 | 1.00 | 13.84 | C |
|------|-------|-----|-----|---|----|--------|--------|---------|------|-------|---|
| ATOM | 10650 | CE1 | HIS | G | 90 | 35.593 | 22.095 | -55.308 | 1.00 | 14.18 | C |
| ATOM | 10651 | NE2 | HIS | G | 90 | 34.676 | 22.893 | -54.789 | 1.00 | 13.89 | N |
| ATOM | 10652 | N | ASN | G | 91 | 34.105 | 18.282 | -57.187 | 1.00 | 15.15 | N |
| ATOM | 10653 | CA | ASN | G | 91 | 35.324 | 17.574 | -56.838 | 1.00 | 15.93 | C |
| ATOM | 10654 | C | ASN | G | 91 | 36.400 | 17.875 | -57.885 | 1.00 | 17.24 | C |
| ATOM | 10655 | O | ASN | G | 91 | 36.920 | 16.975 | -58.527 | 1.00 | 16.31 | O |
| ATOM | 10656 | CB | ASN | G | 91 | 35.051 | 16.082 | -56.731 | 1.00 | 15.89 | C |
| ATOM | 10657 | CG | ASN | G | 91 | 36.288 | 15.282 | -56.421 | 1.00 | 16.83 | C |
| ATOM | 10658 | OD1 | ASN | G | 91 | 37.238 | 15.775 | -55.805 | 1.00 | 15.44 | O |
| ATOM | 10659 | ND2 | ASN | G | 91 | 36.312 | 14.054 | -56.907 | 1.00 | 15.79 | N |
| ATOM | 10660 | N | VAL | G | 92 | 36.714 | 19.153 | -58.048 | 1.00 | 17.61 | N |
| ATOM | 10661 | CA | VAL | G | 92 | 37.765 | 19.541 | -58.995 | 1.00 | 18.84 | C |
| ATOM | 10662 | C | VAL | G | 92 | 38.984 | 20.189 | -58.326 | 1.00 | 19.53 | C |
| ATOM | 10663 | O | VAL | G | 92 | 39.990 | 20.433 | -58.990 | 1.00 | 19.88 | O |
| ATOM | 10664 | CB | VAL | G | 92 | 37.224 | 20.486 | -60.097 | 1.00 | 18.74 | C |
| ATOM | 10665 | CG1 | VAL | G | 92 | 36.144 | 19.805 | -60.858 | 1.00 | 19.67 | C |
| ATOM | 10666 | CG2 | VAL | G | 92 | 36.764 | 21.828 | -59.527 | 1.00 | 19.34 | C |
| ATOM | 10667 | N | GLU | G | 93 | 38.873 | 20.481 | -57.032 | 1.00 | 19.75 | N |
| ATOM | 10668 | CA | GLU | G | 93 | 39.872 | 21.227 | -56.295 | 1.00 | 19.36 | C |
| ATOM | 10669 | C | GLU | G | 93 | 39.549 | 21.243 | -54.807 | 1.00 | 18.41 | C |
| ATOM | 10670 | O | GLU | G | 93 | 38.431 | 20.879 | -54.392 | 1.00 | 16.23 | O |
| ATOM | 10671 | CB | GLU | G | 93 | 39.973 | 22.678 | -56.790 | 1.00 | 20.25 | C |
| ATOM | 10672 | CG | GLU | G | 93 | 38.893 | 23.631 | -56.277 | 1.00 | 21.29 | C |
| ATOM | 10673 | CD | GLU | G | 93 | 39.043 | 25.060 | -56.783 | 1.00 | 23.65 | C |
| ATOM | 10674 | OE1 | GLU | G | 93 | 39.681 | 25.269 | -57.850 | 1.00 | 27.33 | O |
| ATOM | 10675 | OE2 | GLU | G | 93 | 38.517 | 25.990 | -56.107 | 1.00 | 25.37 | O |
| ATOM | 10676 | N | LEU | G | 94 | 40.514 | 21.708 | -54.020 | 1.00 | 17.53 | N |
| ATOM | 10677 | CA | LEU | G | 94 | 40.264 | 22.107 | -52.631 | 1.00 | 16.99 | C |
| ATOM | 10678 | C | LEU | G | 94 | 40.228 | 23.625 | -52.618 | 1.00 | 17.35 | C |
| ATOM | 10679 | O | LEU | G | 94 | 40.922 | 24.223 | -53.425 | 1.00 | 17.05 | O |
| ATOM | 10680 | CB | LEU | G | 94 | 41.396 | 21.614 | -51.729 | 1.00 | 17.17 | C |
| ATOM | 10681 | CG | LEU | G | 94 | 41.708 | 20.119 | -51.738 | 1.00 | 17.33 | C |
| ATOM | 10682 | CD1 | LEU | G | 94 | 42.842 | 19.866 | -50.786 | 1.00 | 17.62 | C |
| ATOM | 10683 | CD2 | LEU | G | 94 | 40.502 | 19.302 | -51.387 | 1.00 | 18.18 | C |
| ATOM | 10684 | N | PRO | G | 95 | 39.416 | 24.244 | -51.735 | 1.00 | 18.40 | N |
| ATOM | 10685 | CA | PRO | G | 95 | 38.544 | 23.631 | -50.728 | 1.00 | 17.86 | C |
| ATOM | 10686 | C | PRO | G | 95 | 37.331 | 22.970 | -51.346 | 1.00 | 17.22 | C |
| ATOM | 10687 | O | PRO | G | 95 | 36.800 | 23.425 | -52.371 | 1.00 | 17.58 | O |
| ATOM | 10688 | CB | PRO | G | 95 | 38.080 | 24.823 | -49.887 | 1.00 | 18.23 | C |
| ATOM | 10689 | CG | PRO | G | 95 | 38.095 | 25.946 | -50.832 | 1.00 | 18.48 | C |
| ATOM | 10690 | CD | PRO | G | 95 | 39.279 | 25.715 | -51.720 | 1.00 | 17.49 | C |
| ATOM | 10691 | N | ARG | G | 96 | 36.873 | 21.909 | -50.703 | 1.00 | 15.74 | N |
| ATOM | 10692 | CA | ARG | G | 96 | 35.676 | 21.254 | -51.141 | 1.00 | 15.75 | C |
| ATOM | 10693 | C | ARG | G | 96 | 34.529 | 22.143 | -50.719 | 1.00 | 15.49 | C |
| ATOM | 10694 | O | ARG | G | 96 | 34.484 | 22.618 | -49.576 | 1.00 | 15.67 | O |
| ATOM | 10695 | CB | ARG | G | 96 | 35.565 | 19.880 | -50.496 | 1.00 | 15.81 | C |
| ATOM | 10696 | CG | ARG | G | 96 | 36.800 | 19.020 | -50.743 | 1.00 | 16.26 | C |
| ATOM | 10697 | CD | ARG | G | 96 | 36.516 | 17.563 | -50.476 | 1.00 | 15.89 | C |
| ATOM | 10698 | NE | ARG | G | 96 | 37.680 | 16.735 | -50.754 | 1.00 | 16.17 | N |
| ATOM | 10699 | CZ | ARG | G | 96 | 38.038 | 16.323 | -51.966 | 1.00 | 16.63 | C |
| ATOM | 10700 | NH1 | ARG | G | 96 | 37.295 | 16.596 | -53.040 | 1.00 | 16.30 | N |
| ATOM | 10701 | NH2 | ARG | G | 96 | 39.124 | 15.576 | -52.101 | 1.00 | 16.24 | N |
| ATOM | 10702 | N | THR | G | 97 | 33.626 | 22.424 | -51.652 | 1.00 | 15.57 | N |
| ATOM | 10703 | CA | THR | G | 97 | 32.492 | 23.283 | -51.338 | 1.00 | 14.78 | C |
| ATOM | 10704 | C | THR | G | 97 | 31.179 | 22.661 | -51.792 | 1.00 | 13.79 | C |
| ATOM | 10705 | O | THR | G | 97 | 31.141 | 21.877 | -52.739 | 1.00 | 13.50 | O |
| ATOM | 10706 | CB | THR | G | 97 | 32.672 | 24.717 | -51.912 | 1.00 | 14.75 | C |
| ATOM | 10707 | OG1 | THR | G | 97 | 32.818 | 24.653 | -53.327 | 1.00 | 14.86 | O |
| ATOM | 10708 | CG2 | THR | G | 97 | 33.889 | 25.384 | -51.289 | 1.00 | 15.55 | C |
| ATOM | 10709 | N | PHE | G | 98 | 30.117 | 23.023 | -51.061 | 1.00 | 13.91 | N |
| ATOM | 10710 | CA | PHE | G | 98 | 28.774 | 22.515 | -51.218 | 1.00 | 13.78 | C |
| ATOM | 10711 | C | PHE | G | 98 | 27.810 | 23.656 | -51.513 | 1.00 | 15.08 | C |
| ATOM | 10712 | O | PHE | G | 98 | 28.052 | 24.814 | -51.095 | 1.00 | 14.55 | O |
| ATOM | 10713 | CB | PHE | G | 98 | 28.337 | 21.824 | -49.907 | 1.00 | 13.40 | C |

```
ATOM   10714  CG   PHE G  98      29.195  20.646 -49.530  1.00 12.94           C
ATOM   10715  CD1  PHE G  98      30.357  20.826 -48.793  1.00 13.64           C
ATOM   10716  CD2  PHE G  98      28.843  19.371 -49.914  1.00 14.12           C
ATOM   10717  CE1  PHE G  98      31.139  19.758 -48.443  1.00 13.80           C
ATOM   10718  CE2  PHE G  98      29.658  18.283 -49.590  1.00 13.91           C
ATOM   10719  CZ   PHE G  98      30.792  18.477 -48.849  1.00 14.18           C
ATOM   10720  N    GLY G  99      26.734  23.348 -52.240  1.00 13.86           N
ATOM   10721  CA   GLY G  99      25.610  24.264 -52.358  1.00 14.58           C
ATOM   10722  C    GLY G  99      24.891  24.352 -51.022  1.00 14.61           C
ATOM   10723  O    GLY G  99      25.104  23.503 -50.143  1.00 15.77           O
ATOM   10724  N    GLY G 100      24.058  25.368 -50.846  1.00 16.76           N
ATOM   10725  CA   GLY G 100      23.321  25.541 -49.591  1.00 17.51           C
ATOM   10726  C    GLY G 100      22.160  24.584 -49.369  1.00 17.90           C
ATOM   10727  O    GLY G 100      21.512  24.608 -48.314  1.00 16.58           O
ATOM   10728  N    GLY G 101      21.862  23.750 -50.353  1.00 17.80           N
ATOM   10729  CA   GLY G 101      20.788  22.760 -50.232  1.00 19.47           C
ATOM   10730  C    GLY G 101      19.450  23.242 -50.776  1.00 19.62           C
ATOM   10731  O    GLY G 101      19.088  24.410 -50.614  1.00 21.40           O
ATOM   10732  N    THR G 102      18.721  22.351 -51.430  1.00 19.27           N
ATOM   10733  CA   THR G 102      17.355  22.626 -51.860  1.00 18.77           C
ATOM   10734  C    THR G 102      16.438  21.635 -51.147  1.00 19.68           C
ATOM   10735  O    THR G 102      16.619  20.433 -51.277  1.00 18.82           O
ATOM   10736  CB   THR G 102      17.187  22.473 -53.389  1.00 19.59           C
ATOM   10737  OG1  THR G 102      18.052  23.397 -54.079  1.00 18.22           O
ATOM   10738  CG2  THR G 102      15.757  22.741 -53.799  1.00 19.90           C
ATOM   10739  N    LYS G 103      15.479  22.149 -50.376  1.00 22.62           N
ATOM   10740  CA   LYS G 103      14.484  21.312 -49.704  1.00 24.01           C
ATOM   10741  C    LYS G 103      13.411  20.878 -50.698  1.00 23.67           C
ATOM   10742  O    LYS G 103      12.835  21.719 -51.385  1.00 23.36           O
ATOM   10743  CB   LYS G 103      13.808  22.051 -48.539  1.00 24.22           C
ATOM   10744  CG   LYS G 103      13.083  21.091 -47.568  1.00 26.74           C
ATOM   10745  CD   LYS G 103      12.080  21.772 -46.640  1.00 28.38           C
ATOM   10746  CE   LYS G 103      12.676  22.919 -45.854  1.00 31.24           C
ATOM   10747  NZ   LYS G 103      11.670  23.482 -44.869  1.00 32.86           N
ATOM   10748  N    LEU G 104      13.179  19.569 -50.794  1.00 23.09           N
ATOM   10749  CA   LEU G 104      12.081  19.039 -51.575  1.00 23.98           C
ATOM   10750  C    LEU G 104      10.868  18.894 -50.660  1.00 25.80           C
ATOM   10751  O    LEU G 104      10.949  18.289 -49.591  1.00 23.99           O
ATOM   10752  CB   LEU G 104      12.445  17.693 -52.196  1.00 23.55           C
ATOM   10753  CG   LEU G 104      11.348  16.918 -52.938  1.00 23.65           C
ATOM   10754  CD1  LEU G 104      10.949  17.622 -54.234  1.00 25.43           C
ATOM   10755  CD2  LEU G 104      11.805  15.499 -53.228  1.00 24.58           C
ATOM   10756  N    GLU G 105       9.760  19.474 -51.108  1.00 29.03           N
ATOM   10757  CA   GLU G 105       8.496  19.501 -50.382  1.00 31.30           C
ATOM   10758  C    GLU G 105       7.494  18.763 -51.256  1.00 29.91           C
ATOM   10759  O    GLU G 105       7.439  18.987 -52.467  1.00 29.17           O
ATOM   10760  CB   GLU G 105       8.058  20.947 -50.172  1.00 32.75           C
ATOM   10761  CG   GLU G 105       6.782  21.128 -49.366  1.00 35.33           C
ATOM   10762  CD   GLU G 105       6.378  22.598 -49.229  1.00 35.89           C
ATOM   10763  OE1  GLU G 105       7.265  23.451 -48.949  1.00 40.86           O
ATOM   10764  OE2  GLU G 105       5.170  22.891 -49.395  1.00 38.57           O
ATOM   10765  N    ILE G 106       6.722  17.888 -50.628  1.00 28.93           N
ATOM   10766  CA   ILE G 106       5.781  17.041 -51.315  1.00 29.29           C
ATOM   10767  C    ILE G 106       4.360  17.469 -50.999  1.00 30.02           C
ATOM   10768  O    ILE G 106       4.051  17.841 -49.866  1.00 27.87           O
ATOM   10769  CB   ILE G 106       5.998  15.570 -50.923  1.00 28.86           C
ATOM   10770  CG1  ILE G 106       7.337  15.097 -51.485  1.00 29.61           C
ATOM   10771  CG2  ILE G 106       4.882  14.720 -51.425  1.00 28.86           C
ATOM   10772  CD1  ILE G 106       7.628  13.626 -51.262  1.00 30.07           C
ATOM   10773  N    LYS G 107       3.505  17.407 -52.020  1.00 31.23           N
ATOM   10774  CA   LYS G 107       2.092  17.701 -51.877  1.00 33.27           C
ATOM   10775  C    LYS G 107       1.355  16.385 -51.897  1.00 32.44           C
ATOM   10776  O    LYS G 107       1.609  15.525 -52.754  1.00 31.88           O
ATOM   10777  CB   LYS G 107       1.604  18.614 -53.002  1.00 34.34           C
ATOM   10778  CG   LYS G 107       0.206  19.184 -52.769  1.00 36.98           C
```

| ATOM | 10779 | CD | LYS | G | 107 | -0.445 | 19.653 | -54.078 | 1.00 | 37.41 | C |
|------|-------|-----|-----|---|-----|--------|--------|---------|------|-------|---|
| ATOM | 10780 | CE | LYS | G | 107 | -1.225 | 18.520 | -54.777 | 1.00 | 39.18 | C |
| ATOM | 10781 | NZ | LYS | G | 107 | -1.494 | 18.765 | -56.250 | 1.00 | 39.91 | N |
| ATOM | 10782 | N | ARG | G | 108 | 0.470 | 16.213 | -50.919 | 1.00 | 31.84 | N |
| ATOM | 10783 | CA | ARG | G | 108 | -0.304 | 15.001 | -50.788 | 1.00 | 31.28 | C |
| ATOM | 10784 | C | ARG | G | 108 | -1.731 | 15.369 | -50.436 | 1.00 | 31.03 | C |
| ATOM | 10785 | O | ARG | G | 108 | -2.074 | 16.534 | -50.326 | 1.00 | 31.24 | O |
| ATOM | 10786 | CB | ARG | G | 108 | 0.300 | 14.081 | -49.724 | 1.00 | 31.32 | C |
| ATOM | 10787 | CG | ARG | G | 108 | 0.486 | 14.735 | -48.361 | 1.00 | 30.88 | C |
| ATOM | 10788 | CD | ARG | G | 108 | 0.392 | 13.705 | -47.267 | 1.00 | 30.36 | C |
| ATOM | 10789 | NE | ARG | G | 108 | -1.006 | 13.393 | -46.977 | 1.00 | 29.12 | N |
| ATOM | 10790 | CZ | ARG | G | 108 | -1.439 | 12.239 | -46.478 | 1.00 | 29.44 | C |
| ATOM | 10791 | NH1 | ARG | G | 108 | -0.605 | 11.242 | -46.202 | 1.00 | 29.28 | N |
| ATOM | 10792 | NH2 | ARG | G | 108 | -2.739 | 12.076 | -46.265 | 1.00 | 29.19 | N |
| ATOM | 10793 | N | ALA | G | 109 | -2.572 | 14.361 | -50.284 | 1.00 | 31.80 | N |
| ATOM | 10794 | CA | ALA | G | 109 | -3.968 | 14.594 | -49.920 | 1.00 | 31.66 | C |
| ATOM | 10795 | C | ALA | G | 109 | -4.073 | 15.206 | -48.523 | 1.00 | 30.99 | C |
| ATOM | 10796 | O | ALA | G | 109 | -3.329 | 14.817 | -47.626 | 1.00 | 30.54 | O |
| ATOM | 10797 | CB | ALA | G | 109 | -4.725 | 13.269 | -49.986 | 1.00 | 32.01 | C |
| ATOM | 10798 | N | ASP | G | 110 | -4.983 | 16.165 | -48.338 | 1.00 | 30.76 | N |
| ATOM | 10799 | CA | ASP | G | 110 | -5.251 | 16.700 | -47.003 | 1.00 | 31.66 | C |
| ATOM | 10800 | C | ASP | G | 110 | -5.603 | 15.606 | -45.982 | 1.00 | 30.43 | C |
| ATOM | 10801 | O | ASP | G | 110 | -6.239 | 14.602 | -46.318 | 1.00 | 29.89 | O |
| ATOM | 10802 | CB | ASP | G | 110 | -6.372 | 17.735 | -47.025 | 1.00 | 32.71 | C |
| ATOM | 10803 | CG | ASP | G | 110 | -6.014 | 18.969 | -47.817 | 1.00 | 34.70 | C |
| ATOM | 10804 | OD1 | ASP | G | 110 | -4.845 | 19.125 | -48.228 | 1.00 | 36.73 | O |
| ATOM | 10805 | OD2 | ASP | G | 110 | -6.922 | 19.791 | -48.027 | 1.00 | 37.12 | O |
| ATOM | 10806 | N | ALA | G | 111 | -5.159 | 15.804 | -44.736 | 1.00 | 28.47 | N |
| ATOM | 10807 | CA | ALA | G | 111 | -5.341 | 14.816 | -43.673 | 1.00 | 26.92 | C |
| ATOM | 10808 | C | ALA | G | 111 | -5.539 | 15.539 | -42.347 | 1.00 | 26.11 | C |
| ATOM | 10809 | O | ALA | G | 111 | -4.832 | 16.497 | -42.033 | 1.00 | 25.50 | O |
| ATOM | 10810 | CB | ALA | G | 111 | -4.153 | 13.860 | -43.595 | 1.00 | 27.04 | C |
| ATOM | 10811 | N | ALA | G | 112 | -6.536 | 15.103 | -41.590 | 1.00 | 25.89 | N |
| ATOM | 10812 | CA | ALA | G | 112 | -6.829 | 15.739 | -40.316 | 1.00 | 25.41 | C |
| ATOM | 10813 | C | ALA | G | 112 | -5.860 | 15.213 | -39.281 | 1.00 | 23.82 | C |
| ATOM | 10814 | O | ALA | G | 112 | -5.404 | 14.070 | -39.363 | 1.00 | 24.22 | O |
| ATOM | 10815 | CB | ALA | G | 112 | -8.272 | 15.461 | -39.874 | 1.00 | 25.95 | C |
| ATOM | 10816 | N | PRO | G | 113 | -5.538 | 16.049 | -38.297 | 1.00 | 23.20 | N |
| ATOM | 10817 | CA | PRO | G | 113 | -4.673 | 15.651 | -37.219 | 1.00 | 22.39 | C |
| ATOM | 10818 | C | PRO | G | 113 | -5.320 | 14.690 | -36.251 | 1.00 | 22.71 | C |
| ATOM | 10819 | O | PRO | G | 113 | -6.549 | 14.745 | -36.023 | 1.00 | 23.34 | O |
| ATOM | 10820 | CB | PRO | G | 113 | -4.400 | 16.966 | -36.490 | 1.00 | 21.60 | C |
| ATOM | 10821 | CG | PRO | G | 113 | -5.569 | 17.788 | -36.766 | 1.00 | 23.49 | C |
| ATOM | 10822 | CD | PRO | G | 113 | -5.984 | 17.440 | -38.157 | 1.00 | 22.42 | C |
| ATOM | 10823 | N | THR | G | 114 | -4.481 | 13.834 | -35.679 | 1.00 | 23.62 | N |
| ATOM | 10824 | CA | THR | G | 114 | -4.841 | 12.967 | -34.552 | 1.00 | 24.13 | C |
| ATOM | 10825 | C | THR | G | 114 | -4.348 | 13.696 | -33.322 | 1.00 | 24.43 | C |
| ATOM | 10826 | O | THR | G | 114 | -3.152 | 13.941 | -33.177 | 1.00 | 23.84 | O |
| ATOM | 10827 | CB | THR | G | 114 | -4.187 | 11.593 | -34.678 | 1.00 | 25.40 | C |
| ATOM | 10828 | OG1 | THR | G | 114 | -4.565 | 11.006 | -35.928 | 1.00 | 26.24 | O |
| ATOM | 10829 | CG2 | THR | G | 114 | -4.617 | 10.676 | -33.546 | 1.00 | 25.68 | C |
| ATOM | 10830 | N | VAL | G | 115 | -5.283 | 14.082 | -32.458 | 1.00 | 24.22 | N |
| ATOM | 10831 | CA | VAL | G | 115 | -4.976 | 14.896 | -31.291 | 1.00 | 23.76 | C |
| ATOM | 10832 | C | VAL | G | 115 | -4.959 | 14.093 | -30.000 | 1.00 | 22.97 | C |
| ATOM | 10833 | O | VAL | G | 115 | -5.878 | 13.338 | -29.741 | 1.00 | 21.14 | O |
| ATOM | 10834 | CB | VAL | G | 115 | -5.998 | 16.039 | -31.141 | 1.00 | 24.39 | C |
| ATOM | 10835 | CG1 | VAL | G | 115 | -5.686 | 16.877 | -29.891 | 1.00 | 24.54 | C |
| ATOM | 10836 | CG2 | VAL | G | 115 | -6.030 | 16.900 | -32.405 | 1.00 | 25.51 | C |
| ATOM | 10837 | N | SER | G | 116 | -3.914 | 14.280 | -29.186 | 1.00 | 22.43 | N |
| ATOM | 10838 | CA | SER | G | 116 | -3.734 | 13.574 | -27.922 | 1.00 | 21.81 | C |
| ATOM | 10839 | C | SER | G | 116 | -3.340 | 14.596 | -26.866 | 1.00 | 22.32 | C |
| ATOM | 10840 | O | SER | G | 116 | -2.467 | 15.416 | -27.121 | 1.00 | 21.73 | O |
| ATOM | 10841 | CB | SER | G | 116 | -2.628 | 12.522 | -28.041 | 1.00 | 22.53 | C |
| ATOM | 10842 | OG | SER | G | 116 | -2.954 | 11.498 | -28.954 | 1.00 | 24.66 | O |
| ATOM | 10843 | N | ILE | G | 117 | -3.986 | 14.549 | -25.689 | 1.00 | 22.68 | N |

```
ATOM  10844  CA   ILE G 117    -3.636  15.415 -24.558  1.00 23.12           C
ATOM  10845  C    ILE G 117    -3.052  14.601 -23.401  1.00 21.84           C
ATOM  10846  O    ILE G 117    -3.455  13.465 -23.137  1.00 19.83           O
ATOM  10847  CB   ILE G 117    -4.854  16.284 -24.075  1.00 23.50           C
ATOM  10848  CG1  ILE G 117    -4.394  17.399 -23.127  1.00 25.01           C
ATOM  10849  CG2  ILE G 117    -5.905  15.406 -23.422  1.00 25.68           C
ATOM  10850  CD1  ILE G 117    -5.408  18.511 -22.954  1.00 24.86           C
ATOM  10851  N    PHE G 118    -2.065  15.190 -22.733  1.00 22.51           N
ATOM  10852  CA   PHE G 118    -1.359  14.526 -21.647  1.00 23.02           C
ATOM  10853  C    PHE G 118    -1.242  15.464 -20.450  1.00 23.65           C
ATOM  10854  O    PHE G 118    -0.781  16.612 -20.595  1.00 22.31           O
ATOM  10855  CB   PHE G 118     0.024  14.095 -22.093  1.00 23.91           C
ATOM  10856  CG   PHE G 118     0.013  13.107 -23.211  1.00 23.52           C
ATOM  10857  CD1  PHE G 118    -0.065  11.752 -22.943  1.00 23.47           C
ATOM  10858  CD2  PHE G 118     0.091  13.527 -24.524  1.00 24.49           C
ATOM  10859  CE1  PHE G 118    -0.063  10.830 -23.968  1.00 24.94           C
ATOM  10860  CE2  PHE G 118     0.093  12.600 -25.556  1.00 24.67           C
ATOM  10861  CZ   PHE G 118     0.013  11.254 -25.275  1.00 25.58           C
ATOM  10862  N    PRO G 119    -1.720  15.000 -19.275  1.00 24.09           N
ATOM  10863  CA   PRO G 119    -1.602  15.768 -18.061  1.00 25.39           C
ATOM  10864  C    PRO G 119    -0.150  15.773 -17.611  1.00 27.00           C
ATOM  10865  O    PRO G 119     0.596  14.874 -18.008  1.00 25.88           O
ATOM  10866  CB   PRO G 119    -2.425  14.958 -17.039  1.00 25.60           C
ATOM  10867  CG   PRO G 119    -3.177  13.965 -17.798  1.00 26.04           C
ATOM  10868  CD   PRO G 119    -2.411  13.719 -19.049  1.00 24.49           C
ATOM  10869  N    PRO G 120     0.230  16.738 -16.762  1.00 28.94           N
ATOM  10870  CA   PRO G 120     1.532  16.689 -16.095  1.00 30.90           C
ATOM  10871  C    PRO G 120     1.783  15.355 -15.415  1.00 32.55           C
ATOM  10872  O    PRO G 120     0.863  14.762 -14.844  1.00 32.52           O
ATOM  10873  CB   PRO G 120     1.424  17.805 -15.061  1.00 30.57           C
ATOM  10874  CG   PRO G 120     0.506  18.788 -15.677  1.00 30.24           C
ATOM  10875  CD   PRO G 120    -0.521  17.943 -16.385  1.00 28.97           C
ATOM  10876  N    SER G 121     3.015  14.867 -15.484  1.00 34.51           N
ATOM  10877  CA   SER G 121     3.372  13.635 -14.779  1.00 35.94           C
ATOM  10878  C    SER G 121     3.525  13.964 -13.294  1.00 36.96           C
ATOM  10879  O    SER G 121     3.745  15.122 -12.924  1.00 37.75           O
ATOM  10880  CB   SER G 121     4.669  13.051 -15.337  1.00 35.40           C
ATOM  10881  OG   SER G 121     5.719  13.971 -15.124  1.00 35.51           O
ATOM  10882  N    SER G 122     3.375  12.949 -12.453  1.00 39.50           N
ATOM  10883  CA   SER G 122     3.516  13.122 -11.005  1.00 41.38           C
ATOM  10884  C    SER G 122     4.907  13.640 -10.678  1.00 42.42           C
ATOM  10885  O    SER G 122     5.062  14.603  -9.903  1.00 43.51           O
ATOM  10886  CB   SER G 122     3.240  11.808 -10.270  1.00 41.55           C
ATOM  10887  OG   SER G 122     3.573  10.685 -11.076  1.00 43.72           O
ATOM  10888  N    GLU G 123     5.906  13.021 -11.304  1.00 42.71           N
ATOM  10889  CA   GLU G 123     7.301  13.452 -11.177  1.00 41.96           C
ATOM  10890  C    GLU G 123     7.451  14.944 -11.275  1.00 40.72           C
ATOM  10891  O    GLU G 123     8.052  15.562 -10.411  1.00 41.30           O
ATOM  10892  CB   GLU G 123     8.168  12.844 -12.270  1.00 42.72           C
ATOM  10893  CG   GLU G 123     8.337  11.365 -12.182  1.00 44.00           C
ATOM  10894  CD   GLU G 123     7.436  10.600 -13.114  1.00 45.38           C
ATOM  10895  OE1  GLU G 123     6.345  11.111 -13.461  1.00 47.67           O
ATOM  10896  OE2  GLU G 123     7.822   9.475 -13.481  1.00 46.32           O
ATOM  10897  N    GLN G 124     6.907  15.524 -12.338  1.00 38.23           N
ATOM  10898  CA   GLN G 124     7.115  16.925 -12.603  1.00 35.70           C
ATOM  10899  C    GLN G 124     6.547  17.852 -11.523  1.00 35.56           C
ATOM  10900  O    GLN G 124     7.138  18.886 -11.209  1.00 35.27           O
ATOM  10901  CB   GLN G 124     6.535  17.298 -13.961  1.00 35.14           C
ATOM  10902  CG   GLN G 124     7.064  18.617 -14.465  1.00 33.34           C
ATOM  10903  CD   GLN G 124     6.367  19.137 -15.699  1.00 32.93           C
ATOM  10904  OE1  GLN G 124     5.448  18.515 -16.225  1.00 29.69           O
ATOM  10905  NE2  GLN G 124     6.787  20.310 -16.153  1.00 31.75           N
ATOM  10906  N    LEU G 125     5.392  17.495 -10.972  1.00 35.11           N
ATOM  10907  CA   LEU G 125     4.689  18.384 -10.045  1.00 33.85           C
ATOM  10908  C    LEU G 125     5.507  18.634  -8.781  1.00 32.02           C
```

```
ATOM   10909  O    LEU G 125      5.561  19.761  -8.261  1.00 31.26          O
ATOM   10910  CB   LEU G 125      3.300  17.815  -9.720  1.00 34.98          C
ATOM   10911  CG   LEU G 125      2.335  17.819 -10.918  1.00 34.83          C
ATOM   10912  CD1  LEU G 125      1.162  16.871 -10.683  1.00 36.00          C
ATOM   10913  CD2  LEU G 125      1.848  19.227 -11.199  1.00 34.83          C
ATOM   10914  N    THR G 126      6.159  17.590  -8.299  1.00 30.61          N
ATOM   10915  CA   THR G 126      7.120  17.725  -7.205  1.00 30.34          C
ATOM   10916  C    THR G 126      8.182  18.796  -7.448  1.00 27.92          C
ATOM   10917  O    THR G 126      8.631  19.461  -6.511  1.00 27.71          O
ATOM   10918  CB   THR G 126      7.832  16.404  -6.983  1.00 31.58          C
ATOM   10919  OG1  THR G 126      6.851  15.365  -6.899  1.00 34.74          O
ATOM   10920  CG2  THR G 126      8.641  16.443  -5.723  1.00 31.95          C
ATOM   10921  N    SER G 127      8.589  18.950  -8.709  1.00 24.77          N
ATOM   10922  CA   SER G 127      9.573  19.967  -9.077  1.00 22.47          C
ATOM   10923  C    SER G 127      9.065  21.398  -8.941  1.00 20.64          C
ATOM   10924  O    SER G 127      9.848  22.322  -9.045  1.00 20.43          O
ATOM   10925  CB   SER G 127     10.030  19.767 -10.524  1.00 21.80          C
ATOM   10926  OG   SER G 127      8.966  20.053 -11.381  1.00 21.84          O
ATOM   10927  N    GLY G 128      7.760  21.596  -8.752  1.00 20.27          N
ATOM   10928  CA   GLY G 128      7.199  22.939  -8.730  1.00 21.76          C
ATOM   10929  C    GLY G 128      6.600  23.385 -10.054  1.00 21.53          C
ATOM   10930  O    GLY G 128      5.974  24.441 -10.119  1.00 22.90          O
ATOM   10931  N    GLY G 129      6.787  22.587 -11.103  1.00 21.89          N
ATOM   10932  CA   GLY G 129      6.285  22.916 -12.438  1.00 22.52          C
ATOM   10933  C    GLY G 129      5.234  21.920 -12.920  1.00 22.91          C
ATOM   10934  O    GLY G 129      5.143  20.816 -12.425  1.00 22.03          O
ATOM   10935  N    ALA G 130      4.470  22.320 -13.919  1.00 23.87          N
ATOM   10936  CA   ALA G 130      3.433  21.485 -14.491  1.00 24.95          C
ATOM   10937  C    ALA G 130      3.326  21.781 -15.993  1.00 24.90          C
ATOM   10938  O    ALA G 130      3.054  22.911 -16.407  1.00 24.74          O
ATOM   10939  CB   ALA G 130      2.114  21.751 -13.806  1.00 26.52          C
ATOM   10940  N    SER G 131      3.586  20.754 -16.793  1.00 23.97          N
ATOM   10941  CA   SER G 131      3.591  20.880 -18.238  1.00 24.26          C
ATOM   10942  C    SER G 131      2.447  20.062 -18.791  1.00 22.57          C
ATOM   10943  O    SER G 131      2.367  18.882 -18.521  1.00 22.96          O
ATOM   10944  CB   SER G 131      4.931  20.388 -18.819  1.00 25.55          C
ATOM   10945  OG   SER G 131      5.956  21.365 -18.627  1.00 26.40          O
ATOM   10946  N    VAL G 132      1.551  20.711 -19.530  1.00 22.45          N
ATOM   10947  CA   VAL G 132      0.470  20.022 -20.236  1.00 23.11          C
ATOM   10948  C    VAL G 132      0.795  20.012 -21.733  1.00 21.59          C
ATOM   10949  O    VAL G 132      1.065  21.045 -22.330  1.00 23.13          O
ATOM   10950  CB   VAL G 132     -0.920  20.659 -20.041  1.00 23.39          C
ATOM   10951  CG1  VAL G 132     -1.998  19.624 -20.366  1.00 24.60          C
ATOM   10952  CG2  VAL G 132     -1.089  21.180 -18.614  1.00 23.80          C
ATOM   10953  N    VAL G 133      0.738  18.824 -22.311  1.00 22.49          N
ATOM   10954  CA   VAL G 133      1.218  18.594 -23.674  1.00 22.55          C
ATOM   10955  C    VAL G 133      0.103  18.043 -24.570  1.00 23.16          C
ATOM   10956  O    VAL G 133     -0.596  17.093 -24.215  1.00 23.33          O
ATOM   10957  CB   VAL G 133      2.377  17.595 -23.691  1.00 22.64          C
ATOM   10958  CG1  VAL G 133      2.885  17.350 -25.126  1.00 24.51          C
ATOM   10959  CG2  VAL G 133      3.515  18.095 -22.795  1.00 22.43          C
ATOM   10960  N    CYS G 134     -0.015  18.654 -25.736  1.00 24.04          N
ATOM   10961  CA   CYS G 134     -0.951  18.248 -26.762  1.00 24.13          C
ATOM   10962  C    CYS G 134     -0.155  17.923 -28.028  1.00 22.52          C
ATOM   10963  O    CYS G 134      0.618  18.757 -28.489  1.00 22.02          O
ATOM   10964  CB   CYS G 134     -1.889  19.401 -27.042  1.00 26.35          C
ATOM   10965  SG   CYS G 134     -3.290  18.943 -28.059  1.00 30.15          S
ATOM   10966  N    PHE G 135     -0.329  16.712 -28.559  1.00 20.86          N
ATOM   10967  CA   PHE G 135      0.210  16.349 -29.872  1.00 20.10          C
ATOM   10968  C    PHE G 135     -0.890  16.454 -30.938  1.00 20.03          C
ATOM   10969  O    PHE G 135     -2.023  16.019 -30.721  1.00 18.41          O
ATOM   10970  CB   PHE G 135      0.744  14.922 -29.909  1.00 20.57          C
ATOM   10971  CG   PHE G 135      1.901  14.650 -28.988  1.00 21.56          C
ATOM   10972  CD1  PHE G 135      2.891  15.588 -28.774  1.00 21.22          C
ATOM   10973  CD2  PHE G 135      2.019  13.418 -28.368  1.00 22.61          C
```

```
ATOM  10974  CE1 PHE G 135      3.978  15.311 -27.926  1.00 22.52           C
ATOM  10975  CE2 PHE G 135      3.093  13.137 -27.517  1.00 24.75           C
ATOM  10976  CZ  PHE G 135      4.083  14.092 -27.308  1.00 22.30           C
ATOM  10977  N   LEU G 136     -0.539  17.054 -32.069  1.00 20.57           N
ATOM  10978  CA  LEU G 136     -1.418  17.172 -33.228  1.00 21.10           C
ATOM  10979  C   LEU G 136     -0.660  16.501 -34.332  1.00 21.01           C
ATOM  10980  O   LEU G 136      0.212  17.112 -34.984  1.00 20.02           O
ATOM  10981  CB  LEU G 136     -1.711  18.626 -33.573  1.00 21.56           C
ATOM  10982  CG  LEU G 136     -2.521  19.457 -32.576  1.00 23.27           C
ATOM  10983  CD1 LEU G 136     -1.758  19.682 -31.299  1.00 25.84           C
ATOM  10984  CD2 LEU G 136     -2.811  20.788 -33.213  1.00 23.92           C
ATOM  10985  N   ASN G 137     -0.934  15.210 -34.492  1.00 20.65           N
ATOM  10986  CA  ASN G 137     -0.098  14.365 -35.323  1.00 20.74           C
ATOM  10987  C   ASN G 137     -0.656  14.025 -36.706  1.00 21.47           C
ATOM  10988  O   ASN G 137     -1.845  13.755 -36.865  1.00 20.21           O
ATOM  10989  CB  ASN G 137      0.227  13.070 -34.593  1.00 19.91           C
ATOM  10990  CG  ASN G 137      1.275  13.260 -33.495  1.00 21.63           C
ATOM  10991  OD1 ASN G 137      1.907  14.309 -33.410  1.00 22.14           O
ATOM  10992  ND2 ASN G 137      1.460  12.240 -32.663  1.00 21.67           N
ATOM  10993  N   ASN G 138      0.257  14.011 -37.679  1.00 22.32           N
ATOM  10994  CA  ASN G 138      0.020  13.464 -39.015  1.00 22.74           C
ATOM  10995  C   ASN G 138     -1.100  14.165 -39.761  1.00 22.54           C
ATOM  10996  O   ASN G 138     -2.061  13.547 -40.206  1.00 22.66           O
ATOM  10997  CB  ASN G 138     -0.208  11.937 -38.955  1.00 23.58           C
ATOM  10998  CG  ASN G 138      0.940  11.185 -38.284  1.00 25.36           C
ATOM  10999  OD1 ASN G 138      0.999  11.094 -37.062  1.00 27.42           O
ATOM  11000  ND2 ASN G 138      1.843  10.618 -39.089  1.00 27.36           N
ATOM  11001  N   PHE G 139     -0.970  15.475 -39.902  1.00 22.55           N
ATOM  11002  CA  PHE G 139     -1.896  16.247 -40.697  1.00 22.26           C
ATOM  11003  C   PHE G 139     -1.214  16.834 -41.954  1.00 23.28           C
ATOM  11004  O   PHE G 139      0.006  16.859 -42.073  1.00 22.04           O
ATOM  11005  CB  PHE G 139     -2.515  17.353 -39.857  1.00 21.73           C
ATOM  11006  CG  PHE G 139     -1.515  18.299 -39.270  1.00 19.34           C
ATOM  11007  CD1 PHE G 139     -1.101  19.398 -39.994  1.00 21.17           C
ATOM  11008  CD2 PHE G 139     -0.996  18.093 -38.002  1.00 21.02           C
ATOM  11009  CE1 PHE G 139     -0.177  20.297 -39.462  1.00 21.69           C
ATOM  11010  CE2 PHE G 139     -0.063  18.993 -37.454  1.00 20.09           C
ATOM  11011  CZ  PHE G 139      0.337  20.090 -38.194  1.00 21.93           C
ATOM  11012  N   TYR G 140     -2.040  17.257 -42.896  1.00 24.04           N
ATOM  11013  CA  TYR G 140     -1.601  17.929 -44.106  1.00 25.78           C
ATOM  11014  C   TYR G 140     -2.791  18.755 -44.594  1.00 26.80           C
ATOM  11015  O   TYR G 140     -3.893  18.217 -44.705  1.00 25.89           O
ATOM  11016  CB  TYR G 140     -1.180  16.925 -45.171  1.00 26.97           C
ATOM  11017  CG  TYR G 140     -0.574  17.631 -46.343  1.00 28.05           C
ATOM  11018  CD1 TYR G 140     -1.372  18.118 -47.371  1.00 28.45           C
ATOM  11019  CD2 TYR G 140      0.795  17.899 -46.385  1.00 29.01           C
ATOM  11020  CE1 TYR G 140     -0.819  18.806 -48.436  1.00 29.44           C
ATOM  11021  CE2 TYR G 140      1.350  18.584 -47.439  1.00 29.16           C
ATOM  11022  CZ  TYR G 140      0.541  19.034 -48.459  1.00 29.04           C
ATOM  11023  OH  TYR G 140      1.086  19.746 -49.494  1.00 29.84           O
ATOM  11024  N   PRO G 141     -2.591  20.048 -44.915  1.00 27.88           N
ATOM  11025  CA  PRO G 141     -1.350  20.838 -45.016  1.00 29.02           C
ATOM  11026  C   PRO G 141     -0.742  21.255 -43.669  1.00 28.81           C
ATOM  11027  O   PRO G 141     -1.352  21.030 -42.612  1.00 26.32           O
ATOM  11028  CB  PRO G 141     -1.788  22.080 -45.801  1.00 29.34           C
ATOM  11029  CG  PRO G 141     -3.250  22.261 -45.433  1.00 28.97           C
ATOM  11030  CD  PRO G 141     -3.787  20.858 -45.223  1.00 29.13           C
ATOM  11031  N   LYS G 142      0.440  21.881 -43.729  1.00 29.15           N
ATOM  11032  CA  LYS G 142      1.221  22.244 -42.531  1.00 30.19           C
ATOM  11033  C   LYS G 142      0.570  23.280 -41.599  1.00 29.30           C
ATOM  11034  O   LYS G 142      0.810  23.304 -40.402  1.00 28.15           O
ATOM  11035  CB  LYS G 142      2.602  22.758 -42.940  1.00 31.14           C
ATOM  11036  CG  LYS G 142      3.564  22.900 -41.757  1.00 32.20           C
ATOM  11037  CD  LYS G 142      4.947  23.308 -42.216  1.00 33.52           C
ATOM  11038  CE  LYS G 142      5.907  23.413 -41.037  1.00 34.71           C
```

428

```
ATOM   11039  NZ   LYS G 142      7.279  23.756 -41.496  1.00 37.10          N
ATOM   11040  N    ASP G 143     -0.264  24.138 -42.150  1.00 30.15          N
ATOM   11041  CA   ASP G 143     -0.873  25.190 -41.367  1.00 30.65          C
ATOM   11042  C    ASP G 143     -1.893  24.608 -40.398  1.00 29.41          C
ATOM   11043  O    ASP G 143     -2.684  23.753 -40.766  1.00 28.33          O
ATOM   11044  CB   ASP G 143     -1.524  26.194 -42.312  1.00 33.25          C
ATOM   11045  CG   ASP G 143     -0.712  26.377 -43.584  1.00 36.79          C
ATOM   11046  OD1  ASP G 143      0.091  27.330 -43.604  1.00 40.59          O
ATOM   11047  OD2  ASP G 143     -0.824  25.526 -44.527  1.00 39.66          O
ATOM   11048  N    ILE G 144     -1.852  25.063 -39.154  1.00 28.71          N
ATOM   11049  CA   ILE G 144     -2.759  24.559 -38.126  1.00 29.25          C
ATOM   11050  C    ILE G 144     -2.763  25.506 -36.944  1.00 29.65          C
ATOM   11051  O    ILE G 144     -1.748  26.143 -36.677  1.00 28.94          O
ATOM   11052  CB   ILE G 144     -2.340  23.136 -37.671  1.00 29.06          C
ATOM   11053  CG1  ILE G 144     -3.472  22.461 -36.887  1.00 29.02          C
ATOM   11054  CG2  ILE G 144     -1.037  23.197 -36.854  1.00 29.17          C
ATOM   11055  CD1  ILE G 144     -3.380  20.973 -36.877  1.00 29.91          C
ATOM   11056  N    ASN G 145     -3.892  25.593 -36.241  1.00 30.00          N
ATOM   11057  CA   ASN G 145     -4.013  26.431 -35.048  1.00 32.15          C
ATOM   11058  C    ASN G 145     -4.399  25.586 -33.841  1.00 31.96          C
ATOM   11059  O    ASN G 145     -5.158  24.614 -33.950  1.00 29.77          O
ATOM   11060  CB   ASN G 145     -5.025  27.588 -35.249  1.00 34.31          C
ATOM   11061  CG   ASN G 145     -4.353  28.911 -35.668  1.00 38.14          C
ATOM   11062  OD1  ASN G 145     -4.085  29.775 -34.830  1.00 43.18          O
ATOM   11063  ND2  ASN G 145     -4.079  29.067 -36.963  1.00 41.25          N
ATOM   11064  N    VAL G 146     -3.830  25.937 -32.688  1.00 31.90          N
ATOM   11065  CA   VAL G 146     -4.152  25.258 -31.444  1.00 32.24          C
ATOM   11066  C    VAL G 146     -4.569  26.285 -30.401  1.00 33.20          C
ATOM   11067  O    VAL G 146     -3.996  27.375 -30.317  1.00 34.18          O
ATOM   11068  CB   VAL G 146     -2.985  24.361 -30.923  1.00 32.19          C
ATOM   11069  CG1  VAL G 146     -1.815  25.207 -30.449  1.00 32.70          C
ATOM   11070  CG2  VAL G 146     -3.468  23.432 -29.795  1.00 31.28          C
ATOM   11071  N    LYS G 147     -5.572  25.919 -29.616  1.00 33.59          N
ATOM   11072  CA   LYS G 147     -6.131  26.785 -28.599  1.00 35.33          C
ATOM   11073  C    LYS G 147     -6.202  26.001 -27.292  1.00 34.33          C
ATOM   11074  O    LYS G 147     -6.636  24.851 -27.266  1.00 34.12          O
ATOM   11075  CB   LYS G 147     -7.511  27.239 -29.065  1.00 35.81          C
ATOM   11076  CG   LYS G 147     -8.151  28.375 -28.308  1.00 37.58          C
ATOM   11077  CD   LYS G 147     -9.277  28.975 -29.175  1.00 37.95          C
ATOM   11078  CE   LYS G 147    -10.232  29.861 -28.403  1.00 39.93          C
ATOM   11079  NZ   LYS G 147    -11.581  29.931 -29.059  1.00 40.55          N
ATOM   11080  N    TRP G 148     -5.731  26.625 -26.213  1.00 34.44          N
ATOM   11081  CA   TRP G 148     -5.766  26.027 -24.878  1.00 33.74          C
ATOM   11082  C    TRP G 148     -6.866  26.679 -24.076  1.00 34.62          C
ATOM   11083  O    TRP G 148     -7.026  27.887 -24.122  1.00 34.43          O
ATOM   11084  CB   TRP G 148     -4.429  26.228 -24.132  1.00 31.92          C
ATOM   11085  CG   TRP G 148     -3.351  25.315 -24.617  1.00 30.49          C
ATOM   11086  CD1  TRP G 148     -2.423  25.593 -25.567  1.00 30.60          C
ATOM   11087  CD2  TRP G 148     -3.100  23.973 -24.189  1.00 29.31          C
ATOM   11088  NE1  TRP G 148     -1.604  24.508 -25.758  1.00 29.64          N
ATOM   11089  CE2  TRP G 148     -2.004  23.497 -24.932  1.00 29.12          C
ATOM   11090  CE3  TRP G 148     -3.706  23.118 -23.265  1.00 29.54          C
ATOM   11091  CZ2  TRP G 148     -1.482  22.216 -24.758  1.00 29.21          C
ATOM   11092  CZ3  TRP G 148     -3.190  21.843 -23.096  1.00 30.17          C
ATOM   11093  CH2  TRP G 148     -2.092  21.401 -23.838  1.00 29.64          C
ATOM   11094  N    LYS G 149     -7.622  25.868 -23.353  1.00 36.05          N
ATOM   11095  CA   LYS G 149     -8.618  26.379 -22.431  1.00 37.97          C
ATOM   11096  C    LYS G 149     -8.459  25.720 -21.082  1.00 38.63          C
ATOM   11097  O    LYS G 149     -8.285  24.502 -20.984  1.00 39.15          O
ATOM   11098  CB   LYS G 149    -10.028  26.150 -22.973  1.00 37.98          C
ATOM   11099  CG   LYS G 149    -10.401  27.085 -24.097  1.00 38.59          C
ATOM   11100  CD   LYS G 149    -11.672  26.588 -24.796  1.00 39.21          C
ATOM   11101  CE   LYS G 149    -12.238  27.631 -25.750  1.00 39.20          C
ATOM   11102  NZ   LYS G 149    -13.517  27.169 -26.361  1.00 40.57          N
ATOM   11103  N    ILE G 150     -8.505  26.549 -20.041  1.00 40.09          N
```

```
ATOM  11104  CA  ILE G 150      -8.515  26.093 -18.661  1.00 40.64           C
ATOM  11105  C   ILE G 150      -9.870  26.498 -18.072  1.00 41.65           C
ATOM  11106  O   ILE G 150     -10.215  27.678 -18.061  1.00 41.51           O
ATOM  11107  CB  ILE G 150      -7.363  26.727 -17.837  1.00 41.10           C
ATOM  11108  CG1 ILE G 150      -6.027  26.605 -18.594  1.00 41.10           C
ATOM  11109  CG2 ILE G 150      -7.273  26.063 -16.470  1.00 40.61           C
ATOM  11110  CD1 ILE G 150      -4.836  27.173 -17.841  1.00 40.88           C
ATOM  11111  N   ASP G 151     -10.626  25.514 -17.588  1.00 42.93           N
ATOM  11112  CA  ASP G 151     -12.004  25.731 -17.156  1.00 44.38           C
ATOM  11113  C   ASP G 151     -12.734  26.607 -18.167  1.00 45.79           C
ATOM  11114  O   ASP G 151     -13.273  27.673 -17.822  1.00 47.25           O
ATOM  11115  CB  ASP G 151     -12.042  26.370 -15.767  1.00 44.63           C
ATOM  11116  CG  ASP G 151     -11.471  25.475 -14.684  1.00 44.57           C
ATOM  11117  OD1 ASP G 151     -11.457  24.237 -14.841  1.00 45.48           O
ATOM  11118  OD2 ASP G 151     -11.044  26.020 -13.645  1.00 45.87           O
ATOM  11119  N   GLY G 152     -12.704  26.184 -19.426  1.00 46.64           N
ATOM  11120  CA  GLY G 152     -13.413  26.876 -20.497  1.00 47.64           C
ATOM  11121  C   GLY G 152     -12.903  28.247 -20.911  1.00 48.62           C
ATOM  11122  O   GLY G 152     -13.444  28.846 -21.839  1.00 49.20           O
ATOM  11123  N   SER G 153     -11.863  28.748 -20.251  1.00 49.95           N
ATOM  11124  CA  SER G 153     -11.332  30.076 -20.555  1.00 51.04           C
ATOM  11125  C   SER G 153     -10.003  29.971 -21.309  1.00 51.38           C
ATOM  11126  O   SER G 153      -9.081  29.296 -20.851  1.00 51.35           O
ATOM  11127  CB  SER G 153     -11.155  30.870 -19.260  1.00 51.39           C
ATOM  11128  OG  SER G 153     -12.329  30.793 -18.466  1.00 52.35           O
ATOM  11129  N   GLU G 154      -9.913  30.640 -22.459  1.00 51.94           N
ATOM  11130  CA  GLU G 154      -8.724  30.564 -23.303  1.00 52.65           C
ATOM  11131  C   GLU G 154      -7.497  31.087 -22.555  1.00 52.28           C
ATOM  11132  O   GLU G 154      -7.552  32.110 -21.861  1.00 52.16           O
ATOM  11133  CB  GLU G 154      -8.893  31.329 -24.623  1.00 52.99           C
ATOM  11134  CG  GLU G 154      -7.840  30.950 -25.674  1.00 53.94           C
ATOM  11135  CD  GLU G 154      -7.667  31.982 -26.786  1.00 54.57           C
ATOM  11136  OE1 GLU G 154      -8.629  32.728 -27.079  1.00 55.55           O
ATOM  11137  OE2 GLU G 154      -6.557  32.034 -27.377  1.00 56.14           O
ATOM  11138  N   ARG G 155      -6.397  30.361 -22.723  1.00 51.45           N
ATOM  11139  CA  ARG G 155      -5.148  30.612 -22.032  1.00 50.65           C
ATOM  11140  C   ARG G 155      -4.098  30.921 -23.088  1.00 49.31           C
ATOM  11141  O   ARG G 155      -3.863  30.100 -23.961  1.00 48.76           O
ATOM  11142  CB  ARG G 155      -4.768  29.353 -21.249  1.00 51.33           C
ATOM  11143  CG  ARG G 155      -3.411  29.391 -20.602  1.00 52.37           C
ATOM  11144  CD  ARG G 155      -3.428  30.245 -19.358  1.00 53.94           C
ATOM  11145  NE  ARG G 155      -2.072  30.628 -18.983  1.00 54.46           N
ATOM  11146  CZ  ARG G 155      -1.630  30.729 -17.733  1.00 54.90           C
ATOM  11147  NH1 ARG G 155      -2.430  30.487 -16.695  1.00 55.06           N
ATOM  11148  NH2 ARG G 155      -0.365  31.080 -17.521  1.00 55.90           N
ATOM  11149  N   GLN G 156      -3.472  32.092 -23.011  1.00 48.07           N
ATOM  11150  CA  GLN G 156      -2.547  32.529 -24.059  1.00 47.44           C
ATOM  11151  C   GLN G 156      -1.085  32.671 -23.608  1.00 46.68           C
ATOM  11152  O   GLN G 156      -0.185  32.719 -24.441  1.00 47.95           O
ATOM  11153  CB  GLN G 156      -3.047  33.834 -24.661  1.00 47.44           C
ATOM  11154  CG  GLN G 156      -4.483  33.732 -25.145  1.00 47.64           C
ATOM  11155  CD  GLN G 156      -4.976  34.982 -25.849  1.00 47.96           C
ATOM  11156  OE1 GLN G 156      -5.771  34.890 -26.783  1.00 48.94           O
ATOM  11157  NE2 GLN G 156      -4.514  36.155 -25.405  1.00 48.01           N
ATOM  11158  N   ASN G 157      -0.859  32.737 -22.300  1.00 45.04           N
ATOM  11159  CA  ASN G 157       0.488  32.761 -21.723  1.00 43.35           C
ATOM  11160  C   ASN G 157       0.999  31.336 -21.481  1.00 40.03           C
ATOM  11161  O   ASN G 157       0.246  30.472 -21.035  1.00 38.88           O
ATOM  11162  CB  ASN G 157       0.471  33.479 -20.360  1.00 44.68           C
ATOM  11163  CG  ASN G 157      -0.148  34.890 -20.403  1.00 47.00           C
ATOM  11164  OD1 ASN G 157      -0.147  35.592 -19.386  1.00 48.16           O
ATOM  11165  ND2 ASN G 157      -0.674  35.304 -21.564  1.00 49.09           N
ATOM  11166  N   GLY G 158       2.279  31.090 -21.750  1.00 37.16           N
ATOM  11167  CA  GLY G 158       2.926  29.829 -21.336  1.00 35.13           C
ATOM  11168  C   GLY G 158       2.837  28.674 -22.321  1.00 33.01           C
```

```
ATOM  11169  O    GLY G 158      3.129  27.525 -21.961  1.00 32.88       O
ATOM  11170  N    VAL G 159      2.449  28.995 -23.553  1.00 30.77       N
ATOM  11171  CA   VAL G 159      2.302  28.035 -24.640  1.00 30.23       C
ATOM  11172  C    VAL G 159      3.450  28.173 -25.634  1.00 29.60       C
ATOM  11173  O    VAL G 159      3.741  29.268 -26.140  1.00 29.80       O
ATOM  11174  CB   VAL G 159      0.975  28.238 -25.424  1.00 30.30       C
ATOM  11175  CG1  VAL G 159      0.777  27.104 -26.450  1.00 31.15       C
ATOM  11176  CG2  VAL G 159     -0.207  28.302 -24.479  1.00 30.63       C
ATOM  11177  N    LEU G 160      4.103  27.044 -25.909  1.00 28.28       N
ATOM  11178  CA   LEU G 160      5.127  26.970 -26.920  1.00 26.93       C
ATOM  11179  C    LEU G 160      4.789  25.800 -27.853  1.00 25.55       C
ATOM  11180  O    LEU G 160      4.463  24.710 -27.391  1.00 24.12       O
ATOM  11181  CB   LEU G 160      6.495  26.778 -26.258  1.00 27.93       C
ATOM  11182  CG   LEU G 160      7.052  28.017 -25.547  1.00 28.79       C
ATOM  11183  CD1  LEU G 160      8.322  27.692 -24.805  1.00 29.94       C
ATOM  11184  CD2  LEU G 160      7.283  29.150 -26.554  1.00 30.33       C
ATOM  11185  N    ASN G 161      4.870  26.051 -29.157  1.00 25.68       N
ATOM  11186  CA   ASN G 161      4.582  25.043 -30.193  1.00 24.35       C
ATOM  11187  C    ASN G 161      5.794  24.742 -31.054  1.00 24.14       C
ATOM  11188  O    ASN G 161      6.611  25.610 -31.325  1.00 25.76       O
ATOM  11189  CB   ASN G 161      3.480  25.522 -31.132  1.00 25.46       C
ATOM  11190  CG   ASN G 161      2.242  26.033 -30.412  1.00 26.48       C
ATOM  11191  OD1  ASN G 161      1.612  26.992 -30.880  1.00 30.39       O
ATOM  11192  ND2  ASN G 161      1.893  25.431 -29.290  1.00 25.05       N
ATOM  11193  N    SER G 162      5.879  23.505 -31.527  1.00 23.08       N
ATOM  11194  CA   SER G 162      6.972  23.071 -32.374  1.00 22.02       C
ATOM  11195  C    SER G 162      6.451  22.149 -33.473  1.00 22.04       C
ATOM  11196  O    SER G 162      5.730  21.207 -33.173  1.00 20.38       O
ATOM  11197  CB   SER G 162      7.985  22.321 -31.514  1.00 21.21       C
ATOM  11198  OG   SER G 162      9.123  21.999 -32.277  1.00 21.36       O
ATOM  11199  N    TRP G 163      6.851  22.399 -34.724  1.00 21.98       N
ATOM  11200  CA   TRP G 163      6.444  21.582 -35.888  1.00 22.96       C
ATOM  11201  C    TRP G 163      7.557  20.657 -36.334  1.00 22.15       C
ATOM  11202  O    TRP G 163      8.728  21.053 -36.372  1.00 21.24       O
ATOM  11203  CB   TRP G 163      6.090  22.460 -37.100  1.00 25.06       C
ATOM  11204  CG   TRP G 163      4.673  23.000 -37.171  1.00 26.60       C
ATOM  11205  CD1  TRP G 163      3.669  22.571 -38.003  1.00 28.10       C
ATOM  11206  CD2  TRP G 163      4.135  24.099 -36.437  1.00 27.60       C
ATOM  11207  NE1  TRP G 163      2.528  23.312 -37.790  1.00 27.55       N
ATOM  11208  CE2  TRP G 163      2.790  24.261 -36.841  1.00 28.09       C
ATOM  11209  CE3  TRP G 163      4.647  24.944 -35.449  1.00 28.83       C
ATOM  11210  CZ2  TRP G 163      1.957  25.245 -36.300  1.00 28.04       C
ATOM  11211  CZ3  TRP G 163      3.822  25.923 -34.917  1.00 28.26       C
ATOM  11212  CH2  TRP G 163      2.491  26.066 -35.343  1.00 28.33       C
ATOM  11213  N    THR G 164      7.202  19.441 -36.733  1.00 21.37       N
ATOM  11214  CA   THR G 164      8.178  18.538 -37.321  1.00 20.15       C
ATOM  11215  C    THR G 164      8.416  18.910 -38.783  1.00 20.99       C
ATOM  11216  O    THR G 164      7.650  19.666 -39.375  1.00 20.76       O
ATOM  11217  CB   THR G 164      7.723  17.074 -37.286  1.00 21.04       C
ATOM  11218  OG1  THR G 164      6.445  16.954 -37.925  1.00 21.84       O
ATOM  11219  CG2  THR G 164      7.667  16.578 -35.864  1.00 20.65       C
ATOM  11220  N    ASP G 165      9.501  18.380 -39.336  1.00 22.68       N
ATOM  11221  CA   ASP G 165      9.712  18.366 -40.781  1.00 24.82       C
ATOM  11222  C    ASP G 165      8.707  17.383 -41.407  1.00 24.59       C
ATOM  11223  O    ASP G 165      8.152  16.523 -40.723  1.00 24.25       O
ATOM  11224  CB   ASP G 165     11.145  17.923 -41.113  1.00 26.74       C
ATOM  11225  CG   ASP G 165     12.204  18.958 -40.686  1.00 30.35       C
ATOM  11226  OD1  ASP G 165     11.963  20.172 -40.822  1.00 33.29       O
ATOM  11227  OD2  ASP G 165     13.297  18.542 -40.234  1.00 35.46       O
ATOM  11228  N    GLN G 166      8.498  17.507 -42.712  1.00 22.97       N
ATOM  11229  CA   GLN G 166      7.630  16.600 -43.440  1.00 23.35       C
ATOM  11230  C    GLN G 166      8.095  15.142 -43.264  1.00 23.73       C
ATOM  11231  O    GLN G 166      9.286  14.833 -43.366  1.00 22.71       O
ATOM  11232  CB   GLN G 166      7.604  17.008 -44.914  1.00 23.23       C
ATOM  11233  CG   GLN G 166      6.613  16.254 -45.733  1.00 22.08       C
```

```
ATOM   11234   CD    GLN G 166      6.289   16.962  -47.035  1.00  23.22       C
ATOM   11235   OE1   GLN G 166      7.171   17.541  -47.701  1.00  19.56       O
ATOM   11236   NE2   GLN G 166      5.013   16.932  -47.402  1.00  19.91       N
ATOM   11237   N     ASP G 167      7.146   14.255  -42.962  1.00  23.91       N
ATOM   11238   CA    ASP G 167      7.428   12.859  -42.644  1.00  25.77       C
ATOM   11239   C     ASP G 167      7.848   12.063  -43.888  1.00  25.36       C
ATOM   11240   O     ASP G 167      7.206   12.170  -44.932  1.00  23.79       O
ATOM   11241   CB    ASP G 167      6.171   12.215  -42.038  1.00  26.74       C
ATOM   11242   CG    ASP G 167      6.423   10.828  -41.502  1.00  29.60       C
ATOM   11243   OD1   ASP G 167      6.811   10.731  -40.322  1.00  33.38       O
ATOM   11244   OD2   ASP G 167      6.235    9.840  -42.254  1.00  30.75       O
ATOM   11245   N     SER G 168      8.908   11.272  -43.752  1.00  25.84       N
ATOM   11246   CA    SER G 168      9.456   10.460  -44.852  1.00  26.87       C
ATOM   11247   C     SER G 168      8.606    9.260  -45.254  1.00  28.70       C
ATOM   11248   O     SER G 168      8.886    8.610  -46.276  1.00  28.45       O
ATOM   11249   CB    SER G 168     10.870    9.959  -44.506  1.00  27.34       C
ATOM   11250   OG    SER G 168     11.797   11.033  -44.395  1.00  27.62       O
ATOM   11251   N     LYS G 169      7.592    8.936  -44.456  1.00  29.15       N
ATOM   11252   CA    LYS G 169      6.776    7.762  -44.730  1.00  29.74       C
ATOM   11253   C     LYS G 169      5.405    8.133  -45.268  1.00  28.20       C
ATOM   11254   O     LYS G 169      4.861    7.430  -46.124  1.00  28.50       O
ATOM   11255   CB    LYS G 169      6.639    6.897  -43.474  1.00  31.17       C
ATOM   11256   CG    LYS G 169      7.019    5.441  -43.705  1.00  34.90       C
ATOM   11257   CD    LYS G 169      8.540    5.285  -43.810  1.00  36.97       C
ATOM   11258   CE    LYS G 169      8.948    4.087  -44.691  1.00  38.95       C
ATOM   11259   NZ    LYS G 169      8.838    4.365  -46.164  1.00  39.62       N
ATOM   11260   N     ASP G 170      4.819    9.221  -44.776  1.00  27.44       N
ATOM   11261   CA    ASP G 170      3.473    9.585  -45.221  1.00  26.18       C
ATOM   11262   C     ASP G 170      3.307   11.040  -45.654  1.00  25.50       C
ATOM   11263   O     ASP G 170      2.196   11.483  -45.920  1.00  26.12       O
ATOM   11264   CB    ASP G 170      2.444    9.198  -44.147  1.00  27.21       C
ATOM   11265   CG    ASP G 170      2.495   10.088  -42.902  1.00  28.13       C
ATOM   11266   OD1   ASP G 170      3.243   11.084  -42.852  1.00  29.22       O
ATOM   11267   OD2   ASP G 170      1.755    9.788  -41.948  1.00  31.38       O
ATOM   11268   N     SER G 171      4.402   11.789  -45.707  1.00  23.14       N
ATOM   11269   CA    SER G 171      4.371   13.162  -46.193  1.00  22.89       C
ATOM   11270   C     SER G 171      3.509   14.098  -45.370  1.00  22.42       C
ATOM   11271   O     SER G 171      3.129   15.157  -45.844  1.00  23.78       O
ATOM   11272   CB    SER G 171      3.952   13.200  -47.680  1.00  22.90       C
ATOM   11273   OG    SER G 171      4.755   12.291  -48.412  1.00  23.87       O
ATOM   11274   N     THR G 172      3.250   13.754  -44.112  1.00  22.29       N
ATOM   11275   CA    THR G 172      2.454   14.630  -43.257  1.00  22.52       C
ATOM   11276   C     THR G 172      3.349   15.487  -42.364  1.00  22.15       C
ATOM   11277   O     THR G 172      4.577   15.323  -42.352  1.00  23.40       O
ATOM   11278   CB    THR G 172      1.481   13.815  -42.352  1.00  23.08       C
ATOM   11279   OG1   THR G 172      2.224   12.999  -41.458  1.00  23.52       O
ATOM   11280   CG2   THR G 172      0.589   12.907  -43.184  1.00  25.53       C
ATOM   11281   N     TYR G 173      2.709   16.395  -41.625  1.00  20.65       N
ATOM   11282   CA    TYR G 173      3.352   17.173  -40.572  1.00  21.55       C
ATOM   11283   C     TYR G 173      2.731   16.825  -39.226  1.00  20.52       C
ATOM   11284   O     TYR G 173      1.621   16.303  -39.156  1.00  18.58       O
ATOM   11285   CB    TYR G 173      3.175   18.667  -40.839  1.00  22.61       C
ATOM   11286   CG    TYR G 173      3.781   19.088  -42.155  1.00  23.87       C
ATOM   11287   CD1   TYR G 173      3.058   18.964  -43.337  1.00  23.73       C
ATOM   11288   CD2   TYR G 173      5.092   19.580  -42.226  1.00  24.66       C
ATOM   11289   CE1   TYR G 173      3.615   19.335  -44.569  1.00  25.94       C
ATOM   11290   CE2   TYR G 173      5.654   19.952  -43.453  1.00  25.16       C
ATOM   11291   CZ    TYR G 173      4.912   19.822  -44.619  1.00  24.91       C
ATOM   11292   OH    TYR G 173      5.436   20.186  -45.847  1.00  25.19       O
ATOM   11293   N     SER G 174      3.471   17.097  -38.156  1.00  19.57       N
ATOM   11294   CA    SER G 174      2.939   17.037  -36.806  1.00  19.15       C
ATOM   11295   C     SER G 174      3.392   18.285  -36.051  1.00  19.66       C
ATOM   11296   O     SER G 174      4.332   18.970  -36.469  1.00  16.38       O
ATOM   11297   CB    SER G 174      3.449   15.796  -36.085  1.00  20.01       C
ATOM   11298   OG    SER G 174      3.011   14.616  -36.734  1.00  19.41       O
```

| ATOM | 11299 | N | MET | G | 175 | 2.743 | 18.541 | -34.926 | 1.00 | 19.61 | N |
| ATOM | 11300 | CA | MET | G | 175 | 3.024 | 19.699 | -34.105 | 1.00 | 23.05 | C |
| ATOM | 11301 | C | MET | G | 175 | 2.736 | 19.366 | -32.637 | 1.00 | 21.26 | C |
| ATOM | 11302 | O | MET | G | 175 | 1.713 | 18.746 | -32.335 | 1.00 | 22.72 | O |
| ATOM | 11303 | CB | MET | G | 175 | 2.142 | 20.866 | -34.546 | 1.00 | 23.93 | C |
| ATOM | 11304 | CG | MET | G | 175 | 2.673 | 22.205 | -34.134 | 1.00 | 27.64 | C |
| ATOM | 11305 | SD | MET | G | 175 | 1.388 | 23.393 | -33.697 | 1.00 | 34.03 | S |
| ATOM | 11306 | CE | MET | G | 175 | 1.051 | 22.752 | -32.077 | 1.00 | 31.12 | C |
| ATOM | 11307 | N | SER | G | 176 | 3.633 | 19.771 | -31.735 | 1.00 | 21.40 | N |
| ATOM | 11308 | CA | SER | G | 176 | 3.411 | 19.660 | -30.282 | 1.00 | 20.39 | C |
| ATOM | 11309 | C | SER | G | 176 | 3.126 | 21.043 | -29.749 | 1.00 | 20.50 | C |
| ATOM | 11310 | O | SER | G | 176 | 3.742 | 22.019 | -30.193 | 1.00 | 19.56 | O |
| ATOM | 11311 | CB | SER | G | 176 | 4.644 | 19.129 | -29.545 | 1.00 | 22.12 | C |
| ATOM | 11312 | OG | SER | G | 176 | 5.667 | 20.114 | -29.568 | 1.00 | 23.99 | O |
| ATOM | 11313 | N | SER | G | 177 | 2.189 | 21.116 | -28.816 | 1.00 | 19.41 | N |
| ATOM | 11314 | CA | SER | G | 177 | 1.909 | 22.339 | -28.087 | 1.00 | 21.28 | C |
| ATOM | 11315 | C | SER | G | 177 | 2.032 | 22.013 | -26.620 | 1.00 | 21.00 | C |
| ATOM | 11316 | O | SER | G | 177 | 1.470 | 21.036 | -26.127 | 1.00 | 20.64 | O |
| ATOM | 11317 | CB | SER | G | 177 | 0.525 | 22.874 | -28.415 | 1.00 | 21.60 | C |
| ATOM | 11318 | OG | SER | G | 177 | 0.311 | 24.094 | -27.737 | 1.00 | 24.08 | O |
| ATOM | 11319 | N | THR | G | 178 | 2.804 | 22.822 | -25.918 | 1.00 | 22.67 | N |
| ATOM | 11320 | CA | THR | G | 178 | 3.074 | 22.576 | -24.515 | 1.00 | 23.03 | C |
| ATOM | 11321 | C | THR | G | 178 | 2.696 | 23.809 | -23.719 | 1.00 | 23.41 | C |
| ATOM | 11322 | O | THR | G | 178 | 3.178 | 24.901 | -24.011 | 1.00 | 23.10 | O |
| ATOM | 11323 | CB | THR | G | 178 | 4.556 | 22.230 | -24.285 | 1.00 | 24.18 | C |
| ATOM | 11324 | OG1 | THR | G | 178 | 4.909 | 21.109 | -25.112 | 1.00 | 25.46 | O |
| ATOM | 11325 | CG2 | THR | G | 178 | 4.816 | 21.876 | -22.810 | 1.00 | 25.24 | C |
| ATOM | 11326 | N | LEU | G | 179 | 1.833 | 23.600 | -22.728 | 1.00 | 25.48 | N |
| ATOM | 11327 | CA | LEU | G | 179 | 1.388 | 24.641 | -21.816 | 1.00 | 26.68 | C |
| ATOM | 11328 | C | LEU | G | 179 | 2.091 | 24.401 | -20.497 | 1.00 | 28.27 | C |
| ATOM | 11329 | O | LEU | G | 179 | 1.886 | 23.355 | -19.860 | 1.00 | 28.08 | O |
| ATOM | 11330 | CB | LEU | G | 179 | -0.130 | 24.557 | -21.619 | 1.00 | 26.56 | C |
| ATOM | 11331 | CG | LEU | G | 179 | -0.738 | 25.416 | -20.505 | 1.00 | 26.48 | C |
| ATOM | 11332 | CD1 | LEU | G | 179 | -0.459 | 26.868 | -20.806 | 1.00 | 26.30 | C |
| ATOM | 11333 | CD2 | LEU | G | 179 | -2.244 | 25.169 | -20.336 | 1.00 | 25.83 | C |
| ATOM | 11334 | N | THR | G | 180 | 2.923 | 25.359 | -20.098 | 1.00 | 30.90 | N |
| ATOM | 11335 | CA | THR | G | 180 | 3.744 | 25.216 | -18.893 | 1.00 | 33.17 | C |
| ATOM | 11336 | C | THR | G | 180 | 3.229 | 26.113 | -17.754 | 1.00 | 33.55 | C |
| ATOM | 11337 | O | THR | G | 180 | 3.107 | 27.321 | -17.924 | 1.00 | 34.19 | O |
| ATOM | 11338 | CB | THR | G | 180 | 5.238 | 25.488 | -19.228 | 1.00 | 34.24 | C |
| ATOM | 11339 | OG1 | THR | G | 180 | 5.360 | 26.565 | -20.172 | 1.00 | 36.13 | O |
| ATOM | 11340 | CG2 | THR | G | 180 | 5.852 | 24.241 | -19.861 | 1.00 | 33.37 | C |
| ATOM | 11341 | N | LEU | G | 181 | 2.911 | 25.501 | -16.616 | 1.00 | 34.51 | N |
| ATOM | 11342 | CA | LEU | G | 181 | 2.310 | 26.201 | -15.467 | 1.00 | 35.39 | C |
| ATOM | 11343 | C | LEU | G | 181 | 3.070 | 25.845 | -14.199 | 1.00 | 37.19 | C |
| ATOM | 11344 | O | LEU | G | 181 | 3.925 | 24.952 | -14.217 | 1.00 | 37.07 | O |
| ATOM | 11345 | CB | LEU | G | 181 | 0.861 | 25.733 | -15.268 | 1.00 | 35.48 | C |
| ATOM | 11346 | CG | LEU | G | 181 | -0.103 | 25.689 | -16.459 | 1.00 | 34.29 | C |
| ATOM | 11347 | CD1 | LEU | G | 181 | -1.178 | 24.662 | -16.202 | 1.00 | 34.54 | C |
| ATOM | 11348 | CD2 | LEU | G | 181 | -0.722 | 27.037 | -16.743 | 1.00 | 34.38 | C |
| ATOM | 11349 | N | THR | G | 182 | 2.725 | 26.514 | -13.092 | 1.00 | 38.47 | N |
| ATOM | 11350 | CA | THR | G | 182 | 3.208 | 26.120 | -11.771 | 1.00 | 39.52 | C |
| ATOM | 11351 | C | THR | G | 182 | 2.402 | 24.914 | -11.304 | 1.00 | 41.53 | C |
| ATOM | 11352 | O | THR | G | 182 | 1.338 | 24.631 | -11.852 | 1.00 | 41.09 | O |
| ATOM | 11353 | CB | THR | G | 182 | 3.036 | 27.237 | -10.725 | 1.00 | 39.67 | C |
| ATOM | 11354 | OG1 | THR | G | 182 | 1.642 | 27.408 | -10.426 | 1.00 | 39.15 | O |
| ATOM | 11355 | CG2 | THR | G | 182 | 3.626 | 28.548 | -11.226 | 1.00 | 39.13 | C |
| ATOM | 11356 | N | LYS | G | 183 | 2.897 | 24.204 | -10.294 | 1.00 | 43.73 | N |
| ATOM | 11357 | CA | LYS | G | 183 | 2.097 | 23.138 | -9.660 | 1.00 | 46.43 | C |
| ATOM | 11358 | C | LYS | G | 183 | 0.761 | 23.706 | -9.146 | 1.00 | 48.02 | C |
| ATOM | 11359 | O | LYS | G | 183 | -0.259 | 23.011 | -9.127 | 1.00 | 47.93 | O |
| ATOM | 11360 | CB | LYS | G | 183 | 2.859 | 22.499 | -8.505 | 1.00 | 47.35 | C |
| ATOM | 11361 | CG | LYS | G | 183 | 2.473 | 21.062 | -8.224 | 1.00 | 48.31 | C |
| ATOM | 11362 | CD | LYS | G | 183 | 2.685 | 20.738 | -6.759 | 1.00 | 49.28 | C |
| ATOM | 11363 | CE | LYS | G | 183 | 2.748 | 19.253 | -6.454 | 1.00 | 49.46 | C |

```
ATOM  11364  NZ   LYS G 183      2.950  19.061   -4.988  1.00 51.19      N
ATOM  11365  N    ASP G 184      0.785  24.986   -8.773  1.00 49.76      N
ATOM  11366  CA   ASP G 184     -0.377  25.694   -8.241  1.00 50.76      C
ATOM  11367  C    ASP G 184     -1.447  26.007   -9.282  1.00 51.17      C
ATOM  11368  O    ASP G 184     -2.559  25.484   -9.201  1.00 51.95      O
ATOM  11369  CB   ASP G 184      0.076  26.999   -7.578  1.00 51.20      C
ATOM  11370  CG   ASP G 184      0.367  26.831   -6.113  1.00 50.90      C
ATOM  11371  OD1  ASP G 184     -0.318  27.497   -5.306  1.00 50.75      O
ATOM  11372  OD2  ASP G 184      1.258  26.024   -5.775  1.00 50.79      O
ATOM  11373  N    GLU G 185     -1.125  26.883  -10.234  1.00 51.03      N
ATOM  11374  CA   GLU G 185     -2.026  27.162  -11.349  1.00 51.13      C
ATOM  11375  C    GLU G 185     -2.697  25.865  -11.775  1.00 50.14      C
ATOM  11376  O    GLU G 185     -3.924  25.749  -11.754  1.00 50.51      O
ATOM  11377  CB   GLU G 185     -1.257  27.764  -12.519  1.00 51.43      C
ATOM  11378  CG   GLU G 185     -0.899  29.226  -12.314  1.00 52.51      C
ATOM  11379  CD   GLU G 185      0.203  29.727  -13.241  1.00 52.87      C
ATOM  11380  OE1  GLU G 185      1.061  28.920  -13.685  1.00 53.30      O
ATOM  11381  OE2  GLU G 185      0.214  30.952  -13.511  1.00 55.29      O
ATOM  11382  N    TYR G 186     -1.879  24.878  -12.117  1.00 48.80      N
ATOM  11383  CA   TYR G 186     -2.386  23.566  -12.500  1.00 47.61      C
ATOM  11384  C    TYR G 186     -3.405  23.022  -11.498  1.00 49.22      C
ATOM  11385  O    TYR G 186     -4.491  22.598  -11.886  1.00 49.56      O
ATOM  11386  CB   TYR G 186     -1.253  22.552  -12.667  1.00 44.31      C
ATOM  11387  CG   TYR G 186     -1.764  21.195  -13.066  1.00 42.12      C
ATOM  11388  CD1  TYR G 186     -2.416  21.009  -14.284  1.00 40.94      C
ATOM  11389  CD2  TYR G 186     -1.621  20.104  -12.236  1.00 40.03      C
ATOM  11390  CE1  TYR G 186     -2.894  19.774  -14.658  1.00 39.36      C
ATOM  11391  CE2  TYR G 186     -2.095  18.858  -12.610  1.00 40.48      C
ATOM  11392  CZ   TYR G 186     -2.738  18.708  -13.823  1.00 40.35      C
ATOM  11393  OH   TYR G 186     -3.214  17.471  -14.202  1.00 41.46      O
ATOM  11394  N    GLU G 187     -3.049  23.027  -10.217  1.00 50.53      N
ATOM  11395  CA   GLU G 187     -3.911  22.445   -9.181  1.00 51.58      C
ATOM  11396  C    GLU G 187     -5.102  23.341   -8.803  1.00 51.79      C
ATOM  11397  O    GLU G 187     -6.107  22.849   -8.293  1.00 51.89      O
ATOM  11398  CB   GLU G 187     -3.082  22.050   -7.956  1.00 51.56      C
ATOM  11399  CG   GLU G 187     -2.324  20.734   -8.165  1.00 51.93      C
ATOM  11400  CD   GLU G 187     -1.255  20.466   -7.118  1.00 52.10      C
ATOM  11401  OE1  GLU G 187     -0.916  21.383   -6.340  1.00 52.90      O
ATOM  11402  OE2  GLU G 187     -0.738  19.329   -7.078  1.00 52.85      O
ATOM  11403  N    ARG G 188     -4.993  24.636   -9.094  1.00 52.08      N
ATOM  11404  CA   ARG G 188     -6.071  25.597   -8.848  1.00 52.80      C
ATOM  11405  C    ARG G 188     -7.165  25.631   -9.940  1.00 52.06      C
ATOM  11406  O    ARG G 188     -8.148  26.370   -9.816  1.00 52.74      O
ATOM  11407  CB   ARG G 188     -5.484  26.996   -8.648  1.00 53.11      C
ATOM  11408  CG   ARG G 188     -4.645  27.132   -7.373  1.00 54.06      C
ATOM  11409  CD   ARG G 188     -4.405  28.586   -7.000  1.00 54.80      C
ATOM  11410  NE   ARG G 188     -4.118  29.422   -8.173  1.00 56.17      N
ATOM  11411  CZ   ARG G 188     -2.930  29.949   -8.488  1.00 57.20      C
ATOM  11412  NH1  ARG G 188     -1.852  29.766   -7.718  1.00 57.75      N
ATOM  11413  NH2  ARG G 188     -2.821  30.688   -9.593  1.00 56.97      N
ATOM  11414  N    HIS G 189     -6.998  24.849  -11.005  1.00 50.42      N
ATOM  11415  CA   HIS G 189     -8.069  24.627  -11.969  1.00 48.94      C
ATOM  11416  C    HIS G 189     -8.229  23.123  -12.176  1.00 47.61      C
ATOM  11417  O    HIS G 189     -7.407  22.341  -11.707  1.00 47.65      O
ATOM  11418  CB   HIS G 189     -7.773  25.340  -13.281  1.00 49.46      C
ATOM  11419  CG   HIS G 189     -7.527  26.814  -13.130  1.00 50.13      C
ATOM  11420  ND1  HIS G 189     -8.547  27.740  -13.066  1.00 51.11      N
ATOM  11421  CD2  HIS G 189     -6.374  27.522  -13.054  1.00 51.04      C
ATOM  11422  CE1  HIS G 189     -8.034  28.953  -12.952  1.00 50.59      C
ATOM  11423  NE2  HIS G 189     -6.717  28.849  -12.946  1.00 50.55      N
ATOM  11424  N    ASN G 190     -9.293  22.703  -12.849  1.00 45.43      N
ATOM  11425  CA   ASN G 190     -9.570  21.271  -12.966  1.00 44.35      C
ATOM  11426  C    ASN G 190     -9.646  20.731  -14.393  1.00 43.07      C
ATOM  11427  O    ASN G 190     -9.195  19.613  -14.660  1.00 43.89      O
ATOM  11428  CB   ASN G 190    -10.861  20.925  -12.228  1.00 44.51      C
```

434

```
ATOM   11429  CG  ASN G 190     -11.080  19.442 -12.132  1.00 45.18           C
ATOM   11430  OD1 ASN G 190     -10.168  18.686 -11.785  1.00 46.38           O
ATOM   11431  ND2 ASN G 190     -12.280  19.005 -12.465  1.00 46.45           N
ATOM   11432  N   SER G 191     -10.227  21.518 -15.291  1.00 41.56           N
ATOM   11433  CA  SER G 191     -10.479  21.097 -16.661  1.00 40.19           C
ATOM   11434  C   SER G 191      -9.441  21.712 -17.605  1.00 39.18           C
ATOM   11435  O   SER G 191      -9.263  22.944 -17.657  1.00 39.18           O
ATOM   11436  CB  SER G 191     -11.892  21.513 -17.073  1.00 40.02           C
ATOM   11437  OG  SER G 191     -12.235  21.006 -18.356  1.00 41.89           O
ATOM   11438  N   TYR G 192      -8.748  20.843 -18.339  1.00 37.96           N
ATOM   11439  CA  TYR G 192      -7.711  21.278 -19.283  1.00 36.71           C
ATOM   11440  C   TYR G 192      -8.068  20.788 -20.667  1.00 34.98           C
ATOM   11441  O   TYR G 192      -8.344  19.609 -20.855  1.00 32.99           O
ATOM   11442  CB  TYR G 192      -6.346  20.738 -18.865  1.00 37.18           C
ATOM   11443  CG  TYR G 192      -5.908  21.365 -17.584  1.00 37.28           C
ATOM   11444  CD1 TYR G 192      -5.279  22.601 -17.579  1.00 37.31           C
ATOM   11445  CD2 TYR G 192      -6.206  20.774 -16.369  1.00 37.73           C
ATOM   11446  CE1 TYR G 192      -4.917  23.207 -16.396  1.00 37.74           C
ATOM   11447  CE2 TYR G 192      -5.845  21.367 -15.187  1.00 37.95           C
ATOM   11448  CZ  TYR G 192      -5.212  22.591 -15.211  1.00 37.95           C
ATOM   11449  OH  TYR G 192      -4.853  23.181 -14.037  1.00 38.74           O
ATOM   11450  N   THR G 193      -8.053  21.708 -21.620  1.00 34.17           N
ATOM   11451  CA  THR G 193      -8.530  21.444 -22.970  1.00 34.24           C
ATOM   11452  C   THR G 193      -7.575  21.938 -24.068  1.00 33.89           C
ATOM   11453  O   THR G 193      -7.132  23.098 -24.068  1.00 33.21           O
ATOM   11454  CB  THR G 193      -9.924  22.105 -23.182  1.00 34.28           C
ATOM   11455  OG1 THR G 193     -10.818  21.679 -22.148  1.00 35.29           O
ATOM   11456  CG2 THR G 193     -10.509  21.741 -24.534  1.00 35.31           C
ATOM   11457  N   CYS G 194      -7.287  21.040 -25.010  1.00 34.21           N
ATOM   11458  CA  CYS G 194      -6.548  21.357 -26.222  1.00 34.10           C
ATOM   11459  C   CYS G 194      -7.509  21.324 -27.414  1.00 33.00           C
ATOM   11460  O   CYS G 194      -8.185  20.324 -27.616  1.00 32.88           O
ATOM   11461  CB  CYS G 194      -5.443  20.302 -26.428  1.00 34.31           C
ATOM   11462  SG  CYS G 194      -4.503  20.564 -27.895  1.00 38.74           S
ATOM   11463  N   GLU G 195      -7.572  22.407 -28.190  1.00 32.84           N
ATOM   11464  CA  GLU G 195      -8.411  22.458 -29.409  1.00 33.82           C
ATOM   11465  C   GLU G 195      -7.604  22.677 -30.682  1.00 32.11           C
ATOM   11466  O   GLU G 195      -6.917  23.683 -30.825  1.00 31.48           O
ATOM   11467  CB  GLU G 195      -9.433  23.592 -29.333  1.00 34.59           C
ATOM   11468  CG  GLU G 195     -10.302  23.571 -28.102  1.00 36.64           C
ATOM   11469  CD  GLU G 195     -11.637  24.259 -28.323  1.00 37.11           C
ATOM   11470  OE1 GLU G 195     -11.677  25.324 -28.986  1.00 40.45           O
ATOM   11471  OE2 GLU G 195     -12.644  23.715 -27.832  1.00 40.84           O
ATOM   11472  N   ALA G 196      -7.752  21.765 -31.632  1.00 30.60           N
ATOM   11473  CA  ALA G 196      -7.031  21.831 -32.892  1.00 29.94           C
ATOM   11474  C   ALA G 196      -7.978  22.222 -34.026  1.00 31.02           C
ATOM   11475  O   ALA G 196      -8.936  21.495 -34.326  1.00 30.61           O
ATOM   11476  CB  ALA G 196      -6.408  20.493 -33.183  1.00 29.34           C
ATOM   11477  N   THR G 197      -7.711  23.371 -34.641  1.00 32.08           N
ATOM   11478  CA  THR G 197      -8.405  23.801 -35.866  1.00 33.67           C
ATOM   11479  C   THR G 197      -7.501  23.703 -37.100  1.00 33.25           C
ATOM   11480  O   THR G 197      -6.435  24.304 -37.151  1.00 32.52           O
ATOM   11481  CB  THR G 197      -8.890  25.251 -35.759  1.00 34.27           C
ATOM   11482  OG1 THR G 197      -9.647  25.405 -34.555  1.00 36.77           O
ATOM   11483  CG2 THR G 197      -9.765  25.612 -36.957  1.00 35.69           C
ATOM   11484  N   HIS G 198      -7.962  22.948 -38.089  1.00 33.20           N
ATOM   11485  CA  HIS G 198      -7.207  22.646 -39.290  1.00 33.59           C
ATOM   11486  C   HIS G 198      -8.164  22.747 -40.478  1.00 35.57           C
ATOM   11487  O   HIS G 198      -9.376  22.568 -40.323  1.00 35.79           O
ATOM   11488  CB  HIS G 198      -6.662  21.232 -39.190  1.00 32.78           C
ATOM   11489  CG  HIS G 198      -5.745  20.853 -40.310  1.00 31.74           C
ATOM   11490  ND1 HIS G 198      -6.045  19.854 -41.209  1.00 30.56           N
ATOM   11491  CD2 HIS G 198      -4.529  21.333 -40.668  1.00 30.55           C
ATOM   11492  CE1 HIS G 198      -5.057  19.739 -42.079  1.00 30.85           C
ATOM   11493  NE2 HIS G 198      -4.126  20.627 -41.776  1.00 29.64           N
```

435

```
ATOM  11494  N   LYS G 199     -7.639  23.009 -41.666  1.00 36.72        N
ATOM  11495  CA  LYS G 199     -8.530  23.275 -42.797  1.00 37.68        C
ATOM  11496  C   LYS G 199     -9.399  22.064 -43.184  1.00 37.65        C
ATOM  11497  O   LYS G 199    -10.408  22.223 -43.870  1.00 38.89        O
ATOM  11498  CB  LYS G 199     -7.763  23.866 -43.990  1.00 37.88        C
ATOM  11499  CG  LYS G 199     -7.314  22.898 -45.071  1.00 38.83        C
ATOM  11500  CD  LYS G 199     -7.119  23.669 -46.380  1.00 39.02        C
ATOM  11501  CE  LYS G 199     -7.294  22.774 -47.574  1.00 39.86        C
ATOM  11502  NZ  LYS G 199     -7.014  23.524 -48.815  1.00 41.38        N
ATOM  11503  N   THR G 200     -9.033  20.873 -42.715  1.00 37.37        N
ATOM  11504  CA  THR G 200     -9.804  19.660 -42.988  1.00 37.64        C
ATOM  11505  C   THR G 200    -11.215  19.669 -42.378  1.00 38.85        C
ATOM  11506  O   THR G 200    -12.059  18.886 -42.795  1.00 39.06        O
ATOM  11507  CB  THR G 200     -9.052  18.372 -42.520  1.00 37.07        C
ATOM  11508  OG1 THR G 200     -8.513  18.571 -41.200  1.00 36.46        O
ATOM  11509  CG2 THR G 200     -7.941  18.026 -43.482  1.00 35.96        C
ATOM  11510  N   SER G 201    -11.469  20.526 -41.388  1.00 39.87        N
ATOM  11511  CA  SER G 201    -12.845  20.746 -40.912  1.00 40.75        C
ATOM  11512  C   SER G 201    -13.067  22.132 -40.327  1.00 41.14        C
ATOM  11513  O   SER G 201    -12.153  22.754 -39.802  1.00 41.25        O
ATOM  11514  CB  SER G 201    -13.249  19.715 -39.861  1.00 40.93        C
ATOM  11515  OG  SER G 201    -14.482  20.099 -39.256  1.00 41.31        O
ATOM  11516  N   THR G 202    -14.310  22.592 -40.399  1.00 41.70        N
ATOM  11517  CA  THR G 202    -14.688  23.846 -39.768  1.00 42.41        C
ATOM  11518  C   THR G 202    -14.766  23.662 -38.246  1.00 42.48        C
ATOM  11519  O   THR G 202    -14.571  24.619 -37.491  1.00 43.18        O
ATOM  11520  CB  THR G 202    -16.020  24.384 -40.351  1.00 43.02        C
ATOM  11521  OG1 THR G 202    -17.050  23.399 -40.216  1.00 44.49        O
ATOM  11522  CG2 THR G 202    -15.854  24.701 -41.826  1.00 43.22        C
ATOM  11523  N   SER G 203    -15.012  22.424 -37.809  1.00 42.01        N
ATOM  11524  CA  SER G 203    -15.096  22.072 -36.382  1.00 41.52        C
ATOM  11525  C   SER G 203    -13.710  21.736 -35.799  1.00 40.05        C
ATOM  11526  O   SER G 203    -13.011  20.886 -36.349  1.00 39.35        O
ATOM  11527  CB  SER G 203    -16.005  20.847 -36.195  1.00 41.97        C
ATOM  11528  OG  SER G 203    -17.253  21.014 -36.853  1.00 43.21        O
ATOM  11529  N   PRO G 204    -13.317  22.380 -34.682  1.00 38.57        N
ATOM  11530  CA  PRO G 204    -12.076  21.954 -34.022  1.00 37.67        C
ATOM  11531  C   PRO G 204    -12.147  20.557 -33.415  1.00 36.61        C
ATOM  11532  O   PRO G 204    -13.217  20.119 -32.974  1.00 35.84        O
ATOM  11533  CB  PRO G 204    -11.882  22.994 -32.907  1.00 37.63        C
ATOM  11534  CG  PRO G 204    -13.197  23.562 -32.668  1.00 38.28        C
ATOM  11535  CD  PRO G 204    -13.932  23.522 -33.984  1.00 38.85        C
ATOM  11536  N   ILE G 205    -11.010  19.867 -33.412  1.00 36.00        N
ATOM  11537  CA  ILE G 205    -10.867  18.607 -32.693  1.00 35.26        C
ATOM  11538  C   ILE G 205    -10.452  18.958 -31.268  1.00 35.02        C
ATOM  11539  O   ILE G 205     -9.447  19.634 -31.057  1.00 33.74        O
ATOM  11540  CB  ILE G 205     -9.820  17.684 -33.346  1.00 35.44        C
ATOM  11541  CG1 ILE G 205    -10.239  17.335 -34.778  1.00 36.08        C
ATOM  11542  CG2 ILE G 205     -9.638  16.415 -32.522  1.00 34.77        C
ATOM  11543  CD1 ILE G 205     -9.099  16.908 -35.650  1.00 35.86        C
ATOM  11544  N   VAL G 206    -11.253  18.516 -30.303  1.00 34.78        N
ATOM  11545  CA  VAL G 206    -11.105  18.916 -28.912  1.00 34.68        C
ATOM  11546  C   VAL G 206    -10.712  17.720 -28.072  1.00 34.01        C
ATOM  11547  O   VAL G 206    -11.368  16.676 -28.135  1.00 34.31        O
ATOM  11548  CB  VAL G 206    -12.432  19.487 -28.356  1.00 35.42        C
ATOM  11549  CG1 VAL G 206    -12.316  19.752 -26.853  1.00 36.13        C
ATOM  11550  CG2 VAL G 206    -12.832  20.765 -29.098  1.00 35.83        C
ATOM  11551  N   LYS G 207     -9.652  17.868 -27.280  1.00 32.22        N
ATOM  11552  CA  LYS G 207     -9.292  16.853 -26.283  1.00 32.15        C
ATOM  11553  C   LYS G 207     -9.134  17.512 -24.924  1.00 30.81        C
ATOM  11554  O   LYS G 207     -8.653  18.643 -24.812  1.00 29.69        O
ATOM  11555  CB  LYS G 207     -8.015  16.088 -26.662  1.00 32.73        C
ATOM  11556  CG  LYS G 207     -8.147  15.186 -27.887  1.00 33.50        C
ATOM  11557  CD  LYS G 207     -9.114  14.032 -27.653  1.00 34.49        C
ATOM  11558  CE  LYS G 207     -9.152  13.057 -28.816  1.00 35.34        C
```

| | | | | | | | | | |
|---|---|---|---|---|---|---|---|---|---|
| ATOM | 11559 | NZ | LYS | G | 207 | -9.231 | 11.628 | -28.329 | 1.00 36.60 | N |
| ATOM | 11560 | N | SER | G | 208 | -9.560 | 16.794 | -23.895 | 1.00 30.65 | N |
| ATOM | 11561 | CA | SER | G | 208 | -9.618 | 17.343 | -22.554 | 1.00 30.72 | C |
| ATOM | 11562 | C | SER | G | 208 | -9.302 | 16.299 | -21.514 | 1.00 28.60 | C |
| ATOM | 11563 | O | SER | G | 208 | -9.437 | 15.091 | -21.751 | 1.00 27.23 | O |
| ATOM | 11564 | CB | SER | G | 208 | -11.014 | 17.925 | -22.276 | 1.00 31.50 | C |
| ATOM | 11565 | OG | SER | G | 208 | -11.194 | 19.154 | -22.957 | 1.00 35.34 | O |
| ATOM | 11566 | N | PHE | G | 209 | -8.865 | 16.770 | -20.355 | 1.00 27.96 | N |
| ATOM | 11567 | CA | PHE | G | 209 | -8.874 | 15.930 | -19.164 | 1.00 28.30 | C |
| ATOM | 11568 | C | PHE | G | 209 | -9.269 | 16.737 | -17.937 | 1.00 29.14 | C |
| ATOM | 11569 | O | PHE | G | 209 | -9.192 | 17.980 | -17.932 | 1.00 27.30 | O |
| ATOM | 11570 | CB | PHE | G | 209 | -7.510 | 15.254 | -18.944 | 1.00 29.13 | C |
| ATOM | 11571 | CG | PHE | G | 209 | -6.402 | 16.211 | -18.669 | 1.00 28.71 | C |
| ATOM | 11572 | CD1 | PHE | G | 209 | -6.088 | 16.579 | -17.367 | 1.00 29.67 | C |
| ATOM | 11573 | CD2 | PHE | G | 209 | -5.652 | 16.734 | -19.704 | 1.00 29.47 | C |
| ATOM | 11574 | CE1 | PHE | G | 209 | -5.058 | 17.475 | -17.107 | 1.00 28.81 | C |
| ATOM | 11575 | CE2 | PHE | G | 209 | -4.607 | 17.625 | -19.444 | 1.00 28.42 | C |
| ATOM | 11576 | CZ | PHE | G | 209 | -4.321 | 17.996 | -18.152 | 1.00 28.71 | C |
| ATOM | 11577 | N | ASN | G | 210 | -9.712 | 16.008 | -16.911 | 1.00 30.63 | N |
| ATOM | 11578 | CA | ASN | G | 210 | -9.942 | 16.558 | -15.576 | 1.00 31.72 | C |
| ATOM | 11579 | C | ASN | G | 210 | -8.859 | 16.098 | -14.617 | 1.00 33.18 | C |
| ATOM | 11580 | O | ASN | G | 210 | -8.617 | 14.893 | -14.468 | 1.00 33.64 | O |
| ATOM | 11581 | CB | ASN | G | 210 | -11.289 | 16.099 | -15.021 | 1.00 31.96 | C |
| ATOM | 11582 | CG | ASN | G | 210 | -12.465 | 16.607 | -15.834 | 1.00 30.59 | C |
| ATOM | 11583 | OD1 | ASN | G | 210 | -12.601 | 17.811 | -16.075 | 1.00 30.22 | O |
| ATOM | 11584 | ND2 | ASN | G | 210 | -13.332 | 15.686 | -16.244 | 1.00 31.67 | N |
| ATOM | 11585 | N | ARG | G | 211 | -8.234 | 17.066 | -13.963 | 1.00 35.34 | N |
| ATOM | 11586 | CA | ARG | G | 211 | -7.114 | 16.834 | -13.051 | 1.00 38.03 | C |
| ATOM | 11587 | C | ARG | G | 211 | -7.400 | 15.809 | -11.947 | 1.00 38.50 | C |
| ATOM | 11588 | O | ARG | G | 211 | -8.519 | 15.728 | -11.408 | 1.00 39.39 | O |
| ATOM | 11589 | CB | ARG | G | 211 | -6.704 | 18.167 | -12.427 | 1.00 38.69 | C |
| ATOM | 11590 | CG | ARG | G | 211 | -5.457 | 18.088 | -11.547 | 1.00 40.69 | C |
| ATOM | 11591 | CD | ARG | G | 211 | -5.059 | 19.466 | -10.999 | 1.00 41.36 | C |
| ATOM | 11592 | NE | ARG | G | 211 | -6.209 | 20.158 | -10.425 | 1.00 44.03 | N |
| ATOM | 11593 | CZ | ARG | G | 211 | -6.837 | 19.785 | -9.312 | 1.00 44.05 | C |
| ATOM | 11594 | NH1 | ARG | G | 211 | -6.425 | 18.733 | -8.605 | 1.00 44.43 | N |
| ATOM | 11595 | NH2 | ARG | G | 211 | -7.889 | 20.487 | -8.897 | 1.00 44.98 | N |
| TER | 11596 | | ARG | G | 211 | | | | | |
| ATOM | 11597 | N | GLU | H | 1 | 27.522 | -1.810 | -44.688 | 1.00 38.13 | N |
| ATOM | 11598 | CA | GLU | H | 1 | 28.784 | -2.485 | -44.272 | 1.00 37.24 | C |
| ATOM | 11599 | C | GLU | H | 1 | 29.866 | -1.484 | -43.895 | 1.00 35.01 | C |
| ATOM | 11600 | O | GLU | H | 1 | 30.599 | -1.707 | -42.927 | 1.00 34.87 | O |
| ATOM | 11601 | CB | GLU | H | 1 | 29.284 | -3.407 | -45.379 | 1.00 37.53 | C |
| ATOM | 11602 | CG | GLU | H | 1 | 30.503 | -4.238 | -45.003 | 1.00 39.27 | C |
| ATOM | 11603 | CD | GLU | H | 1 | 31.769 | -3.871 | -45.769 | 1.00 40.55 | C |
| ATOM | 11604 | OE1 | GLU | H | 1 | 31.736 | -2.963 | -46.632 | 1.00 42.22 | O |
| ATOM | 11605 | OE2 | GLU | H | 1 | 32.807 | -4.523 | -45.518 | 1.00 43.55 | O |
| ATOM | 11606 | N | VAL | H | 2 | 29.986 | -0.397 | -44.658 | 1.00 33.08 | N |
| ATOM | 11607 | CA | VAL | H | 2 | 30.845 | 0.706 | -44.217 | 1.00 31.48 | C |
| ATOM | 11608 | C | VAL | H | 2 | 30.155 | 1.451 | -43.062 | 1.00 29.34 | C |
| ATOM | 11609 | O | VAL | H | 2 | 28.976 | 1.803 | -43.130 | 1.00 27.82 | O |
| ATOM | 11610 | CB | VAL | H | 2 | 31.220 | 1.673 | -45.350 | 1.00 31.55 | C |
| ATOM | 11611 | CG1 | VAL | H | 2 | 32.046 | 2.847 | -44.799 | 1.00 31.23 | C |
| ATOM | 11612 | CG2 | VAL | H | 2 | 32.012 | 0.931 | -46.439 | 1.00 31.49 | C |
| ATOM | 11613 | N | LYS | H | 3 | 30.914 | 1.668 | -41.997 | 1.00 27.82 | N |
| ATOM | 11614 | CA | LYS | H | 3 | 30.411 | 2.307 | -40.807 | 1.00 28.36 | C |
| ATOM | 11615 | C | LYS | H | 3 | 31.438 | 3.346 | -40.403 | 1.00 25.18 | C |
| ATOM | 11616 | O | LYS | H | 3 | 32.600 | 3.019 | -40.208 | 1.00 23.84 | O |
| ATOM | 11617 | CB | LYS | H | 3 | 30.240 | 1.265 | -39.712 | 1.00 29.96 | C |
| ATOM | 11618 | CG | LYS | H | 3 | 29.542 | 1.749 | -38.453 | 1.00 33.03 | C |
| ATOM | 11619 | CD | LYS | H | 3 | 29.505 | 0.618 | -37.425 | 1.00 32.93 | C |
| ATOM | 11620 | CE | LYS | H | 3 | 28.607 | 0.938 | -36.246 | 1.00 34.66 | C |
| ATOM | 11621 | NZ | LYS | H | 3 | 28.718 | -0.121 | -35.172 | 1.00 36.49 | N |
| ATOM | 11622 | N | VAL | H | 4 | 30.998 | 4.597 | -40.314 | 1.00 23.47 | N |
| ATOM | 11623 | CA | VAL | H | 4 | 31.838 | 5.725 | -39.893 | 1.00 22.17 | C |

| ATOM | 11624 | C | VAL H | 4 | 31.191 | 6.284 | -38.625 | 1.00 | 20.72 | C |
|------|-------|-----|-------|----|--------|--------|---------|------|-------|---|
| ATOM | 11625 | O | VAL H | 4 | 30.026 | 6.694 | -38.663 | 1.00 | 19.46 | O |
| ATOM | 11626 | CB | VAL H | 4 | 31.910 | 6.805 | -41.003 | 1.00 | 22.22 | C |
| ATOM | 11627 | CG1 | VAL H | 4 | 32.818 | 7.975 | -40.586 | 1.00 | 23.84 | C |
| ATOM | 11628 | CG2 | VAL H | 4 | 32.381 | 6.183 | -42.323 | 1.00 | 21.99 | C |
| ATOM | 11629 | N | GLU H | 5 | 31.929 | 6.279 | -37.512 | 1.00 | 19.79 | N |
| ATOM | 11630 | CA | GLU H | 5 | 31.351 | 6.659 | -36.211 | 1.00 | 22.48 | C |
| ATOM | 11631 | C | GLU H | 5 | 32.194 | 7.666 | -35.423 | 1.00 | 19.54 | C |
| ATOM | 11632 | O | GLU H | 5 | 33.282 | 7.345 | -34.968 | 1.00 | 18.62 | O |
| ATOM | 11633 | CB | GLU H | 5 | 31.109 | 5.418 | -35.337 | 1.00 | 23.70 | C |
| ATOM | 11634 | CG | GLU H | 5 | 30.283 | 5.763 | -34.114 | 1.00 | 26.55 | C |
| ATOM | 11635 | CD | GLU H | 5 | 30.138 | 4.650 | -33.121 | 1.00 | 28.67 | C |
| ATOM | 11636 | OE1 | GLU H | 5 | 30.138 | 3.471 | -33.540 | 1.00 | 33.41 | O |
| ATOM | 11637 | OE2 | GLU H | 5 | 30.009 | 4.966 | -31.906 | 1.00 | 35.24 | O |
| ATOM | 11638 | N | GLU H | 6 | 31.657 | 8.869 | -35.238 | 1.00 | 18.05 | N |
| ATOM | 11639 | CA | GLU H | 6 | 32.384 | 9.954 | -34.576 | 1.00 | 17.33 | C |
| ATOM | 11640 | C | GLU H | 6 | 32.142 | 9.924 | -33.079 | 1.00 | 17.64 | C |
| ATOM | 11641 | O | GLU H | 6 | 31.120 | 9.428 | -32.627 | 1.00 | 17.07 | O |
| ATOM | 11642 | CB | GLU H | 6 | 31.929 | 11.329 | -35.100 | 1.00 | 17.12 | C |
| ATOM | 11643 | CG | GLU H | 6 | 32.188 | 11.596 | -36.582 | 1.00 | 18.25 | C |
| ATOM | 11644 | CD | GLU H | 6 | 31.117 | 11.010 | -37.489 | 1.00 | 20.02 | C |
| ATOM | 11645 | OE1 | GLU H | 6 | 30.331 | 10.140 | -37.037 | 1.00 | 20.96 | O |
| ATOM | 11646 | OE2 | GLU H | 6 | 31.033 | 11.448 | -38.646 | 1.00 | 20.65 | O |
| ATOM | 11647 | N | SER H | 7 | 33.077 | 10.498 | -32.321 | 1.00 | 17.35 | N |
| ATOM | 11648 | CA | SER H | 7 | 32.902 | 10.691 | -30.883 | 1.00 | 17.72 | C |
| ATOM | 11649 | C | SER H | 7 | 33.721 | 11.891 | -30.450 | 1.00 | 16.08 | C |
| ATOM | 11650 | O | SER H | 7 | 34.480 | 12.409 | -31.249 | 1.00 | 14.97 | O |
| ATOM | 11651 | CB | SER H | 7 | 33.362 | 9.433 | -30.147 | 1.00 | 18.96 | C |
| ATOM | 11652 | OG | SER H | 7 | 34.706 | 9.136 | -30.457 | 1.00 | 22.13 | O |
| ATOM | 11653 | N | GLY H | 8 | 33.559 | 12.326 | -29.196 | 1.00 | 16.33 | N |
| ATOM | 11654 | CA | GLY H | 8 | 34.375 | 13.393 | -28.619 | 1.00 | 15.79 | C |
| ATOM | 11655 | C | GLY H | 8 | 33.777 | 14.791 | -28.510 | 1.00 | 15.08 | C |
| ATOM | 11656 | O | GLY H | 8 | 34.445 | 15.733 | -28.022 | 1.00 | 17.14 | O |
| ATOM | 11657 | N | GLY H | 9 | 32.539 | 14.944 | -28.944 | 1.00 | 14.42 | N |
| ATOM | 11658 | CA | GLY H | 9 | 31.871 | 16.238 | -28.893 | 1.00 | 13.69 | C |
| ATOM | 11659 | C | GLY H | 9 | 31.543 | 16.621 | -27.462 | 1.00 | 13.82 | C |
| ATOM | 11660 | O | GLY H | 9 | 31.861 | 15.894 | -26.514 | 1.00 | 13.58 | O |
| ATOM | 11661 | N | GLY H | 10 | 30.923 | 17.784 | -27.286 | 1.00 | 11.95 | N |
| ATOM | 11662 | CA | GLY H | 10 | 30.621 | 18.293 | -25.972 | 1.00 | 11.06 | C |
| ATOM | 11663 | C | GLY H | 10 | 30.684 | 19.815 | -26.056 | 1.00 | 11.12 | C |
| ATOM | 11664 | O | GLY H | 10 | 30.496 | 20.356 | -27.140 | 1.00 | 11.06 | O |
| ATOM | 11665 | N | LEU H | 11 | 31.014 | 20.471 | -24.942 | 1.00 | 10.35 | N |
| ATOM | 11666 | CA | LEU H | 11 | 30.963 | 21.961 | -24.827 | 1.00 | 10.03 | C |
| ATOM | 11667 | C | LEU H | 11 | 32.308 | 22.467 | -24.336 | 1.00 | 9.35 | C |
| ATOM | 11668 | O | LEU H | 11 | 32.956 | 21.825 | -23.520 | 1.00 | 8.97 | O |
| ATOM | 11669 | CB | LEU H | 11 | 29.830 | 22.413 | -23.882 | 1.00 | 9.77 | C |
| ATOM | 11670 | CG | LEU H | 11 | 29.647 | 23.941 | -23.664 | 1.00 | 9.84 | C |
| ATOM | 11671 | CD1 | LEU H | 11 | 28.174 | 24.215 | -23.374 | 1.00 | 11.30 | C |
| ATOM | 11672 | CD2 | LEU H | 11 | 30.553 | 24.421 | -22.527 | 1.00 | 9.12 | C |
| ATOM | 11673 | N | VAL H | 12 | 32.755 | 23.606 | -24.868 | 1.00 | 10.11 | N |
| ATOM | 11674 | CA | VAL H | 12 | 33.905 | 24.289 | -24.289 | 1.00 | 8.59 | C |
| ATOM | 11675 | C | VAL H | 12 | 33.669 | 25.787 | -24.515 | 1.00 | 9.32 | C |
| ATOM | 11676 | O | VAL H | 12 | 32.870 | 26.199 | -25.334 | 1.00 | 8.92 | O |
| ATOM | 11677 | CB | VAL H | 12 | 35.270 | 23.839 | -24.881 | 1.00 | 8.77 | C |
| ATOM | 11678 | CG1 | VAL H | 12 | 35.383 | 24.216 | -26.392 | 1.00 | 11.73 | C |
| ATOM | 11679 | CG2 | VAL H | 12 | 36.445 | 24.345 | -24.061 | 1.00 | 11.94 | C |
| ATOM | 11680 | N | GLN H | 13 | 34.391 | 26.559 | -23.741 | 1.00 | 9.23 | N |
| ATOM | 11681 | CA | GLN H | 13 | 34.334 | 28.028 | -23.790 | 1.00 | 8.93 | C |
| ATOM | 11682 | C | GLN H | 13 | 35.175 | 28.563 | -24.934 | 1.00 | 9.89 | C |
| ATOM | 11683 | O | GLN H | 13 | 36.160 | 27.973 | -25.341 | 1.00 | 8.88 | O |
| ATOM | 11684 | CB | GLN H | 13 | 34.872 | 28.576 | -22.478 | 1.00 | 8.32 | C |
| ATOM | 11685 | CG | GLN H | 13 | 34.045 | 28.204 | -21.266 | 1.00 | 10.27 | C |
| ATOM | 11686 | CD | GLN H | 13 | 34.347 | 26.763 | -20.809 | 1.00 | 10.80 | C |
| ATOM | 11687 | OE1 | GLN H | 13 | 35.477 | 26.276 | -20.975 | 1.00 | 10.90 | O |
| ATOM | 11688 | NE2 | GLN H | 13 | 33.330 | 26.089 | -20.253 | 1.00 | 11.19 | N |

| ATOM | 11689 | N   | PRO H | 14 | 34.794 | 29.727 | -25.470 | 1.00 | 10.84 | N |
| ATOM | 11690 | CA  | PRO H | 14 | 35.674 | 30.415 | -26.412 | 1.00 | 11.42 | C |
| ATOM | 11691 | C   | PRO H | 14 | 37.124 | 30.528 | -25.914 | 1.00 |  9.07 | C |
| ATOM | 11692 | O   | PRO H | 14 | 37.367 | 30.887 | -24.755 | 1.00 | 11.43 | O |
| ATOM | 11693 | CB  | PRO H | 14 | 35.031 | 31.804 | -26.523 | 1.00 | 11.80 | C |
| ATOM | 11694 | CG  | PRO H | 14 | 33.609 | 31.607 | -26.203 | 1.00 | 12.51 | C |
| ATOM | 11695 | CD  | PRO H | 14 | 33.516 | 30.419 | -25.261 | 1.00 | 12.11 | C |
| ATOM | 11696 | N   | GLY H | 15 | 38.074 | 30.222 | -26.784 | 1.00 | 10.21 | N |
| ATOM | 11697 | CA  | GLY H | 15 | 39.473 | 30.131 | -26.441 | 1.00 | 12.37 | C |
| ATOM | 11698 | C   | GLY H | 15 | 39.975 | 28.721 | -26.180 | 1.00 | 12.71 | C |
| ATOM | 11699 | O   | GLY H | 15 | 41.166 | 28.476 | -26.205 | 1.00 | 15.12 | O |
| ATOM | 11700 | N   | GLY H | 16 | 39.044 | 27.818 | -25.919 | 1.00 | 11.96 | N |
| ATOM | 11701 | CA  | GLY H | 16 | 39.318 | 26.469 | -25.448 | 1.00 | 12.33 | C |
| ATOM | 11702 | C   | GLY H | 16 | 39.537 | 25.510 | -26.583 | 1.00 | 11.29 | C |
| ATOM | 11703 | O   | GLY H | 16 | 39.530 | 25.889 | -27.752 | 1.00 | 10.90 | O |
| ATOM | 11704 | N   | SER H | 17 | 39.701 | 24.243 | -26.220 | 1.00 | 11.66 | N |
| ATOM | 11705 | CA  | SER H | 17 | 40.127 | 23.198 | -27.164 | 1.00 | 12.13 | C |
| ATOM | 11706 | C   | SER H | 17 | 39.203 | 22.011 | -27.041 | 1.00 | 11.84 | C |
| ATOM | 11707 | O   | SER H | 17 | 38.546 | 21.836 | -26.018 | 1.00 | 10.94 | O |
| ATOM | 11708 | CB  | SER H | 17 | 41.570 | 22.733 | -26.873 | 1.00 | 13.30 | C |
| ATOM | 11709 | OG  | SER H | 17 | 42.541 | 23.746 | -27.151 | 1.00 | 15.58 | O |
| ATOM | 11710 | N   | MET H | 18 | 39.125 | 21.231 | -28.122 | 1.00 | 11.35 | N |
| ATOM | 11711 | CA  | MET H | 18 | 38.430 | 19.928 | -28.123 | 1.00 | 13.58 | C |
| ATOM | 11712 | C   | MET H | 18 | 39.209 | 19.012 | -29.021 | 1.00 | 13.91 | C |
| ATOM | 11713 | O   | MET H | 18 | 39.949 | 19.482 | -29.882 | 1.00 | 14.81 | O |
| ATOM | 11714 | CB  | MET H | 18 | 37.044 | 20.034 | -28.715 | 1.00 | 14.30 | C |
| ATOM | 11715 | CG  | MET H | 18 | 36.159 | 21.082 | -28.097 | 1.00 | 18.56 | C |
| ATOM | 11716 | SD  | MET H | 18 | 35.254 | 20.430 | -26.669 | 1.00 | 22.31 | S |
| ATOM | 11717 | CE  | MET H | 18 | 34.148 | 19.319 | -27.578 | 1.00 | 17.65 | C |
| ATOM | 11718 | N   | LYS H | 19 | 39.018 | 17.705 | -28.852 | 1.00 | 14.79 | N |
| ATOM | 11719 | CA  | LYS H | 19 | 39.621 | 16.719 | -29.757 | 1.00 | 15.39 | C |
| ATOM | 11720 | C   | LYS H | 19 | 38.536 | 15.709 | -30.065 | 1.00 | 14.30 | C |
| ATOM | 11721 | O   | LYS H | 19 | 38.005 | 15.067 | -29.159 | 1.00 | 15.32 | O |
| ATOM | 11722 | CB  | LYS H | 19 | 40.842 | 16.009 | -29.158 | 1.00 | 16.91 | C |
| ATOM | 11723 | CG  | LYS H | 19 | 41.584 | 15.189 | -30.231 | 1.00 | 18.12 | C |
| ATOM | 11724 | CD  | LYS H | 19 | 42.789 | 14.407 | -29.701 | 1.00 | 20.75 | C |
| ATOM | 11725 | CE  | LYS H | 19 | 43.703 | 13.971 | -30.850 | 1.00 | 21.86 | C |
| ATOM | 11726 | NZ  | LYS H | 19 | 45.045 | 13.389 | -30.375 | 1.00 | 23.81 | N |
| ATOM | 11727 | N   | ILE H | 20 | 38.163 | 15.623 | -31.331 | 1.00 | 13.21 | N |
| ATOM | 11728 | CA  | ILE H | 20 | 37.139 | 14.680 | -31.765 | 1.00 | 13.41 | C |
| ATOM | 11729 | C   | ILE H | 20 | 37.784 | 13.617 | -32.651 | 1.00 | 14.10 | C |
| ATOM | 11730 | O   | ILE H | 20 | 38.892 | 13.779 | -33.125 | 1.00 | 14.15 | O |
| ATOM | 11731 | CB  | ILE H | 20 | 35.946 | 15.392 | -32.477 | 1.00 | 13.86 | C |
| ATOM | 11732 | CG1 | ILE H | 20 | 36.414 | 16.113 | -33.752 | 1.00 | 14.56 | C |
| ATOM | 11733 | CG2 | ILE H | 20 | 35.264 | 16.406 | -31.521 | 1.00 | 14.10 | C |
| ATOM | 11734 | CD1 | ILE H | 20 | 35.289 | 16.742 | -34.545 | 1.00 | 15.59 | C |
| ATOM | 11735 | N   | SER H | 21 | 37.092 | 12.500 | -32.832 | 1.00 | 16.52 | N |
| ATOM | 11736 | CA  | SER H | 21 | 37.644 | 11.409 | -33.601 | 1.00 | 17.20 | C |
| ATOM | 11737 | C   | SER H | 21 | 36.554 | 10.599 | -34.298 | 1.00 | 17.01 | C |
| ATOM | 11738 | O   | SER H | 21 | 35.375 | 10.665 | -33.952 | 1.00 | 16.35 | O |
| ATOM | 11739 | CB  | SER H | 21 | 38.466 | 10.533 | -32.669 | 1.00 | 19.03 | C |
| ATOM | 11740 | OG  | SER H | 21 | 37.629 |  9.849 | -31.763 | 1.00 | 22.42 | O |
| ATOM | 11741 | N   | CYS H | 22 | 36.941 |  9.816 | -35.294 | 1.00 | 18.27 | N |
| ATOM | 11742 | CA  | CYS H | 22 | 36.025 |  8.812 | -35.800 | 1.00 | 18.94 | C |
| ATOM | 11743 | C   | CYS H | 22 | 36.767 |  7.536 | -36.139 | 1.00 | 17.42 | C |
| ATOM | 11744 | O   | CYS H | 22 | 37.941 |  7.555 | -36.455 | 1.00 | 17.23 | O |
| ATOM | 11745 | CB  | CYS H | 22 | 35.238 |  9.328 | -37.000 | 1.00 | 21.74 | C |
| ATOM | 11746 | SG  | CYS H | 22 | 36.269 |  9.737 | -38.374 | 1.00 | 26.92 | S |
| ATOM | 11747 | N   | VAL H | 23 | 36.047 |  6.435 | -36.012 | 1.00 | 18.95 | N |
| ATOM | 11748 | CA  | VAL H | 23 | 36.541 |  5.130 | -36.378 | 1.00 | 18.99 | C |
| ATOM | 11749 | C   | VAL H | 23 | 35.698 |  4.582 | -37.525 | 1.00 | 19.21 | C |
| ATOM | 11750 | O   | VAL H | 23 | 34.461 |  4.711 | -37.542 | 1.00 | 18.11 | O |
| ATOM | 11751 | CB  | VAL H | 23 | 36.604 |  4.172 | -35.138 | 1.00 | 20.21 | C |
| ATOM | 11752 | CG1 | VAL H | 23 | 35.248 |  3.961 | -34.498 | 1.00 | 21.49 | C |
| ATOM | 11753 | CG2 | VAL H | 23 | 37.265 |  2.853 | -35.520 | 1.00 | 21.97 | C |

| | | | | | | | | | | | |
|---|---|---|---|---|---|---|---|---|---|---|---|
| ATOM | 11754 | N | VAL | H | 24 | 36.397 | 3.983 | -38.490 | 1.00 | 19.44 | N |
| ATOM | 11755 | CA | VAL | H | 24 | 35.772 | 3.490 | -39.695 | 1.00 | 21.46 | C |
| ATOM | 11756 | C | VAL | H | 24 | 35.946 | 1.974 | -39.736 | 1.00 | 21.02 | C |
| ATOM | 11757 | O | VAL | H | 24 | 37.054 | 1.505 | -39.560 | 1.00 | 22.59 | O |
| ATOM | 11758 | CB | VAL | H | 24 | 36.419 | 4.101 | -40.937 | 1.00 | 22.31 | C |
| ATOM | 11759 | CG1 | VAL | H | 24 | 35.615 | 3.739 | -42.177 | 1.00 | 23.82 | C |
| ATOM | 11760 | CG2 | VAL | H | 24 | 36.501 | 5.623 | -40.784 | 1.00 | 22.58 | C |
| ATOM | 11761 | N | SER | H | 25 | 34.855 | 1.252 | -39.953 | 1.00 | 22.26 | N |
| ATOM | 11762 | CA | SER | H | 25 | 34.921 | -0.182 | -40.260 | 1.00 | 23.14 | C |
| ATOM | 11763 | C | SER | H | 25 | 34.244 | -0.507 | -41.593 | 1.00 | 24.38 | C |
| ATOM | 11764 | O | SER | H | 25 | 33.564 | 0.330 | -42.190 | 1.00 | 24.18 | O |
| ATOM | 11765 | CB | SER | H | 25 | 34.270 | -0.982 | -39.137 | 1.00 | 21.84 | C |
| ATOM | 11766 | OG | SER | H | 25 | 32.902 | -0.670 | -39.027 | 1.00 | 23.23 | O |
| ATOM | 11767 | N | GLY | H | 26 | 34.459 | -1.735 | -42.071 | 1.00 | 25.56 | N |
| ATOM | 11768 | CA | GLY | H | 26 | 33.861 | -2.175 | -43.337 | 1.00 | 25.89 | C |
| ATOM | 11769 | C | GLY | H | 26 | 34.649 | -1.747 | -44.565 | 1.00 | 25.95 | C |
| ATOM | 11770 | O | GLY | H | 26 | 34.178 | -1.927 | -45.693 | 1.00 | 26.93 | O |
| ATOM | 11771 | N | LEU | H | 27 | 35.840 | -1.191 | -44.351 | 1.00 | 25.93 | N |
| ATOM | 11772 | CA | LEU | H | 27 | 36.766 | -0.861 | -45.434 | 1.00 | 26.36 | C |
| ATOM | 11773 | C | LEU | H | 27 | 38.186 | -0.792 | -44.886 | 1.00 | 26.50 | C |
| ATOM | 11774 | O | LEU | H | 27 | 38.402 | -0.876 | -43.661 | 1.00 | 27.32 | O |
| ATOM | 11775 | CB | LEU | H | 27 | 36.349 | 0.441 | -46.167 | 1.00 | 27.08 | C |
| ATOM | 11776 | CG | LEU | H | 27 | 36.161 | 1.745 | -45.377 | 1.00 | 26.84 | C |
| ATOM | 11777 | CD1 | LEU | H | 27 | 37.480 | 2.286 | -44.842 | 1.00 | 26.90 | C |
| ATOM | 11778 | CD2 | LEU | H | 27 | 35.463 | 2.803 | -46.232 | 1.00 | 26.79 | C |
| ATOM | 11779 | N | THR | H | 28 | 39.161 | -0.658 | -45.777 | 1.00 | 26.06 | N |
| ATOM | 11780 | CA | THR | H | 28 | 40.561 | -0.540 | -45.361 | 1.00 | 26.07 | C |
| ATOM | 11781 | C | THR | H | 28 | 40.923 | 0.927 | -45.197 | 1.00 | 25.81 | C |
| ATOM | 11782 | O | THR | H | 28 | 41.265 | 1.619 | -46.169 | 1.00 | 24.84 | O |
| ATOM | 11783 | CB | THR | H | 28 | 41.510 | -1.216 | -46.355 | 1.00 | 26.57 | C |
| ATOM | 11784 | OG1 | THR | H | 28 | 41.162 | -2.599 | -46.442 | 1.00 | 27.89 | O |
| ATOM | 11785 | CG2 | THR | H | 28 | 42.946 | -1.091 | -45.905 | 1.00 | 26.39 | C |
| ATOM | 11786 | N | PHE | H | 29 | 40.855 | 1.374 | -43.946 | 1.00 | 25.59 | N |
| ATOM | 11787 | CA | PHE | H | 29 | 40.981 | 2.799 | -43.583 | 1.00 | 24.85 | C |
| ATOM | 11788 | C | PHE | H | 29 | 42.202 | 3.439 | -44.213 | 1.00 | 25.49 | C |
| ATOM | 11789 | O | PHE | H | 29 | 42.116 | 4.529 | -44.794 | 1.00 | 25.06 | O |
| ATOM | 11790 | CB | PHE | H | 29 | 40.998 | 2.915 | -42.055 | 1.00 | 25.40 | C |
| ATOM | 11791 | CG | PHE | H | 29 | 41.213 | 4.305 | -41.525 | 1.00 | 23.78 | C |
| ATOM | 11792 | CD1 | PHE | H | 29 | 40.143 | 5.090 | -41.160 | 1.00 | 24.80 | C |
| ATOM | 11793 | CD2 | PHE | H | 29 | 42.487 | 4.795 | -41.340 | 1.00 | 24.71 | C |
| ATOM | 11794 | CE1 | PHE | H | 29 | 40.345 | 6.348 | -40.634 | 1.00 | 23.87 | C |
| ATOM | 11795 | CE2 | PHE | H | 29 | 42.699 | 6.050 | -40.829 | 1.00 | 25.03 | C |
| ATOM | 11796 | CZ | PHE | H | 29 | 41.614 | 6.836 | -40.479 | 1.00 | 23.11 | C |
| ATOM | 11797 | N | SER | H | 30 | 43.337 | 2.755 | -44.136 | 1.00 | 24.69 | N |
| ATOM | 11798 | CA | SER | H | 30 | 44.584 | 3.285 | -44.663 | 1.00 | 25.68 | C |
| ATOM | 11799 | C | SER | H | 30 | 44.564 | 3.615 | -46.164 | 1.00 | 23.54 | C |
| ATOM | 11800 | O | SER | H | 30 | 45.445 | 4.331 | -46.659 | 1.00 | 24.00 | O |
| ATOM | 11801 | CB | SER | H | 30 | 45.734 | 2.305 | -44.364 | 1.00 | 25.90 | C |
| ATOM | 11802 | OG | SER | H | 30 | 45.483 | 1.040 | -44.966 | 1.00 | 28.48 | O |
| ATOM | 11803 | N | ASN | H | 31 | 43.563 | 3.121 | -46.885 | 1.00 | 23.67 | N |
| ATOM | 11804 | CA | ASN | H | 31 | 43.497 | 3.340 | -48.321 | 1.00 | 23.62 | C |
| ATOM | 11805 | C | ASN | H | 31 | 42.653 | 4.557 | -48.725 | 1.00 | 23.23 | C |
| ATOM | 11806 | O | ASN | H | 31 | 42.613 | 4.916 | -49.905 | 1.00 | 22.63 | O |
| ATOM | 11807 | CB | ASN | H | 31 | 42.987 | 2.070 | -49.017 | 1.00 | 25.43 | C |
| ATOM | 11808 | CG | ASN | H | 31 | 44.001 | 0.903 | -48.943 | 1.00 | 28.24 | C |
| ATOM | 11809 | OD1 | ASN | H | 31 | 43.617 | -0.270 | -48.925 | 1.00 | 30.38 | O |
| ATOM | 11810 | ND2 | ASN | H | 31 | 45.289 | 1.231 | -48.913 | 1.00 | 28.05 | N |
| ATOM | 11811 | N | TYR | H | 32 | 42.015 | 5.219 | -47.754 | 1.00 | 21.82 | N |
| ATOM | 11812 | CA | TYR | H | 32 | 41.032 | 6.246 | -48.061 | 1.00 | 19.97 | C |
| ATOM | 11813 | C | TYR | H | 32 | 41.438 | 7.615 | -47.568 | 1.00 | 19.44 | C |
| ATOM | 11814 | O | TYR | H | 32 | 41.962 | 7.753 | -46.467 | 1.00 | 18.93 | O |
| ATOM | 11815 | CB | TYR | H | 32 | 39.675 | 5.896 | -47.456 | 1.00 | 21.02 | C |
| ATOM | 11816 | CG | TYR | H | 32 | 38.951 | 4.823 | -48.202 | 1.00 | 21.52 | C |
| ATOM | 11817 | CD1 | TYR | H | 32 | 37.991 | 5.140 | -49.154 | 1.00 | 21.85 | C |
| ATOM | 11818 | CD2 | TYR | H | 32 | 39.220 | 3.496 | -47.953 | 1.00 | 20.80 | C |

440

| ATOM | 11819 | CE1 | TYR | H | 32 | 37.320 | 4.154 | -49.852 | 1.00 | 23.39 | C |
|------|-------|-----|-----|---|----|--------|-------|---------|------|-------|---|
| ATOM | 11820 | CE2 | TYR | H | 32 | 38.541 | 2.480 | -48.643 | 1.00 | 23.24 | C |
| ATOM | 11821 | CZ  | TYR | H | 32 | 37.603 | 2.821 | -49.597 | 1.00 | 23.50 | C |
| ATOM | 11822 | OH  | TYR | H | 32 | 36.903 | 1.844 | -50.297 | 1.00 | 24.58 | O |
| ATOM | 11823 | N   | TRP | H | 33 | 41.190 | 8.615 | -48.404 | 1.00 | 18.37 | N |
| ATOM | 11824 | CA  | TRP | H | 33 | 41.332 | 9.998 | -47.995 | 1.00 | 18.31 | C |
| ATOM | 11825 | C   | TRP | H | 33 | 40.301 | 10.253 | -46.893 | 1.00 | 16.40 | C |
| ATOM | 11826 | O   | TRP | H | 33 | 39.185 | 9.738 | -46.937 | 1.00 | 15.99 | O |
| ATOM | 11827 | CB  | TRP | H | 33 | 41.094 | 10.935 | -49.173 | 1.00 | 17.59 | C |
| ATOM | 11828 | CG  | TRP | H | 33 | 42.086 | 10.834 | -50.285 | 1.00 | 19.15 | C |
| ATOM | 11829 | CD1 | TRP | H | 33 | 43.244 | 10.086 | -50.321 | 1.00 | 19.11 | C |
| ATOM | 11830 | CD2 | TRP | H | 33 | 42.029 | 11.540 | -51.526 | 1.00 | 20.27 | C |
| ATOM | 11831 | NE1 | TRP | H | 33 | 43.901 | 10.287 | -51.521 | 1.00 | 19.41 | N |
| ATOM | 11832 | CE2 | TRP | H | 33 | 43.173 | 11.168 | -52.280 | 1.00 | 20.17 | C |
| ATOM | 11833 | CE3 | TRP | H | 33 | 41.114 | 12.442 | -52.087 | 1.00 | 19.42 | C |
| ATOM | 11834 | CZ2 | TRP | H | 33 | 43.422 | 11.677 | -53.546 | 1.00 | 20.21 | C |
| ATOM | 11835 | CZ3 | TRP | H | 33 | 41.369 | 12.946 | -53.343 | 1.00 | 19.17 | C |
| ATOM | 11836 | CH2 | TRP | H | 33 | 42.513 | 12.565 | -54.063 | 1.00 | 19.06 | C |
| ATOM | 11837 | N   | MET | H | 34 | 40.686 | 11.055 | -45.914 | 1.00 | 16.85 | N |
| ATOM | 11838 | CA  | MET | H | 34 | 39.829 | 11.363 | -44.787 | 1.00 | 16.67 | C |
| ATOM | 11839 | C   | MET | H | 34 | 39.651 | 12.880 | -44.644 | 1.00 | 14.97 | C |
| ATOM | 11840 | O   | MET | H | 34 | 40.618 | 13.613 | -44.758 | 1.00 | 15.30 | O |
| ATOM | 11841 | CB  | MET | H | 34 | 40.480 | 10.860 | -43.519 | 1.00 | 17.14 | C |
| ATOM | 11842 | CG  | MET | H | 34 | 40.570 | 9.352 | -43.418 | 1.00 | 16.61 | C |
| ATOM | 11843 | SD  | MET | H | 34 | 38.987 | 8.642 | -43.115 | 1.00 | 20.67 | S |
| ATOM | 11844 | CE  | MET | H | 34 | 39.297 | 7.067 | -43.971 | 1.00 | 20.62 | C |
| ATOM | 11845 | N   | SER | H | 35 | 38.428 | 13.317 | -44.362 | 1.00 | 15.89 | N |
| ATOM | 11846 | CA  | SER | H | 35 | 38.134 | 14.736 | -44.101 | 1.00 | 13.88 | C |
| ATOM | 11847 | C   | SER | H | 35 | 37.101 | 14.973 | -43.007 | 1.00 | 14.54 | C |
| ATOM | 11848 | O   | SER | H | 35 | 36.319 | 14.092 | -42.646 | 1.00 | 13.87 | O |
| ATOM | 11849 | CB  | SER | H | 35 | 37.684 | 15.461 | -45.382 | 1.00 | 13.57 | C |
| ATOM | 11850 | OG  | SER | H | 35 | 36.337 | 15.192 | -45.669 | 1.00 | 16.13 | O |
| ATOM | 11851 | N   | TRP | H | 36 | 37.078 | 16.226 | -42.512 | 1.00 | 13.41 | N |
| ATOM | 11852 | CA  | TRP | H | 36 | 36.063 | 16.664 | -41.572 | 1.00 | 12.08 | C |
| ATOM | 11853 | C   | TRP | H | 36 | 35.246 | 17.734 | -42.273 | 1.00 | 13.06 | C |
| ATOM | 11854 | O   | TRP | H | 36 | 35.806 | 18.638 | -42.895 | 1.00 | 11.49 | O |
| ATOM | 11855 | CB  | TRP | H | 36 | 36.691 | 17.227 | -40.293 | 1.00 | 13.29 | C |
| ATOM | 11856 | CG  | TRP | H | 36 | 37.298 | 16.184 | -39.443 | 1.00 | 13.04 | C |
| ATOM | 11857 | CD1 | TRP | H | 36 | 38.593 | 15.796 | -39.412 | 1.00 | 12.98 | C |
| ATOM | 11858 | CD2 | TRP | H | 36 | 36.607 | 15.381 | -38.479 | 1.00 | 10.31 | C |
| ATOM | 11859 | NE1 | TRP | H | 36 | 38.763 | 14.784 | -38.482 | 1.00 | 13.14 | N |
| ATOM | 11860 | CE2 | TRP | H | 36 | 37.549 | 14.514 | -37.906 | 1.00 | 13.25 | C |
| ATOM | 11861 | CE3 | TRP | H | 36 | 35.288 | 15.333 | -38.036 | 1.00 | 13.26 | C |
| ATOM | 11862 | CZ2 | TRP | H | 36 | 37.200 | 13.604 | -36.902 | 1.00 | 12.18 | C |
| ATOM | 11863 | CZ3 | TRP | H | 36 | 34.945 | 14.406 | -37.067 | 1.00 | 14.34 | C |
| ATOM | 11864 | CH2 | TRP | H | 36 | 35.897 | 13.578 | -36.508 | 1.00 | 11.78 | C |
| ATOM | 11865 | N   | VAL | H | 37 | 33.931 | 17.564 | -42.232 | 1.00 | 12.07 | N |
| ATOM | 11866 | CA  | VAL | H | 37 | 32.997 | 18.535 | -42.738 | 1.00 | 12.12 | C |
| ATOM | 11867 | C   | VAL | H | 37 | 32.041 | 18.917 | -41.610 | 1.00 | 12.17 | C |
| ATOM | 11868 | O   | VAL | H | 37 | 31.478 | 18.045 | -40.963 | 1.00 | 12.65 | O |
| ATOM | 11869 | CB  | VAL | H | 37 | 32.190 | 17.975 | -43.941 | 1.00 | 11.78 | C |
| ATOM | 11870 | CG1 | VAL | H | 37 | 31.141 | 18.999 | -44.392 | 1.00 | 12.32 | C |
| ATOM | 11871 | CG2 | VAL | H | 37 | 33.144 | 17.620 | -45.067 | 1.00 | 12.35 | C |
| ATOM | 11872 | N   | ARG | H | 38 | 31.855 | 20.208 | -41.352 | 1.00 | 12.28 | N |
| ATOM | 11873 | CA  | ARG | H | 38 | 30.917 | 20.609 | -40.274 | 1.00 | 10.37 | C |
| ATOM | 11874 | C   | ARG | H | 38 | 29.650 | 21.221 | -40.853 | 1.00 | 11.40 | C |
| ATOM | 11875 | O   | ARG | H | 38 | 29.654 | 21.701 | -41.993 | 1.00 | 9.61 | O |
| ATOM | 11876 | CB  | ARG | H | 38 | 31.588 | 21.502 | -39.242 | 1.00 | 12.09 | C |
| ATOM | 11877 | CG  | ARG | H | 38 | 31.991 | 22.865 | -39.803 | 1.00 | 10.81 | C |
| ATOM | 11878 | CD  | ARG | H | 38 | 32.858 | 23.567 | -38.808 | 1.00 | 13.59 | C |
| ATOM | 11879 | NE  | ARG | H | 38 | 33.224 | 24.896 | -39.303 | 1.00 | 12.55 | N |
| ATOM | 11880 | CZ  | ARG | H | 38 | 34.061 | 25.721 | -38.688 | 1.00 | 13.81 | C |
| ATOM | 11881 | NH1 | ARG | H | 38 | 34.653 | 25.386 | -37.552 | 1.00 | 13.13 | N |
| ATOM | 11882 | NH2 | ARG | H | 38 | 34.296 | 26.909 | -39.224 | 1.00 | 13.12 | N |
| ATOM | 11883 | N   | GLN | H | 39 | 28.551 | 21.107 | -40.111 | 1.00 | 11.37 | N |

| ATOM | 11884 | CA | GLN | H | 39 | 27.275 | 21.658 | -40.540 | 1.00 | 14.24 | C |
|------|-------|-----|-----|---|----|--------|--------|---------|------|-------|---|
| ATOM | 11885 | C | GLN | H | 39 | 26.721 | 22.589 | -39.451 | 1.00 | 15.46 | C |
| ATOM | 11886 | O | GLN | H | 39 | 26.637 | 22.196 | -38.283 | 1.00 | 11.65 | O |
| ATOM | 11887 | CB | GLN | H | 39 | 26.270 | 20.555 | -40.813 | 1.00 | 14.55 | C |
| ATOM | 11888 | CG | GLN | H | 39 | 24.990 | 21.023 | -41.463 | 1.00 | 16.22 | C |
| ATOM | 11889 | CD | GLN | H | 39 | 24.148 | 19.878 | -41.998 | 1.00 | 16.94 | C |
| ATOM | 11890 | OE1 | GLN | H | 39 | 24.144 | 18.768 | -41.443 | 1.00 | 19.08 | O |
| ATOM | 11891 | NE2 | GLN | H | 39 | 23.440 | 20.135 | -43.094 | 1.00 | 18.49 | N |
| ATOM | 11892 | N | SER | H | 40 | 26.396 | 23.806 | -39.863 | 1.00 | 17.73 | N |
| ATOM | 11893 | CA | SER | H | 40 | 25.672 | 24.754 | -39.038 | 1.00 | 22.73 | C |
| ATOM | 11894 | C | SER | H | 40 | 24.522 | 25.372 | -39.848 | 1.00 | 23.76 | C |
| ATOM | 11895 | O | SER | H | 40 | 24.539 | 25.357 | -41.087 | 1.00 | 22.57 | O |
| ATOM | 11896 | CB | SER | H | 40 | 26.608 | 25.850 | -38.551 | 1.00 | 22.64 | C |
| ATOM | 11897 | OG | SER | H | 40 | 27.261 | 26.496 | -39.633 | 1.00 | 24.95 | O |
| ATOM | 11898 | N | PRO | H | 41 | 23.488 | 25.881 | -39.154 | 1.00 | 27.46 | N |
| ATOM | 11899 | CA | PRO | H | 41 | 22.442 | 26.693 | -39.835 | 1.00 | 27.82 | C |
| ATOM | 11900 | C | PRO | H | 41 | 22.978 | 27.923 | -40.598 | 1.00 | 30.36 | C |
| ATOM | 11901 | O | PRO | H | 41 | 22.481 | 28.261 | -41.678 | 1.00 | 32.42 | O |
| ATOM | 11902 | CB | PRO | H | 41 | 21.520 | 27.104 | -38.678 | 1.00 | 29.12 | C |
| ATOM | 11903 | CG | PRO | H | 41 | 21.692 | 26.009 | -37.662 | 1.00 | 28.70 | C |
| ATOM | 11904 | CD | PRO | H | 41 | 23.167 | 25.665 | -37.731 | 1.00 | 27.92 | C |
| ATOM | 11905 | N | GLU | H | 42 | 24.014 | 28.555 | -40.080 | 1.00 | 30.90 | N |
| ATOM | 11906 | CA | GLU | H | 42 | 24.510 | 29.795 | -40.668 | 1.00 | 31.62 | C |
| ATOM | 11907 | C | GLU | H | 42 | 25.382 | 29.557 | -41.914 | 1.00 | 32.03 | C |
| ATOM | 11908 | O | GLU | H | 42 | 25.325 | 30.346 | -42.863 | 1.00 | 32.19 | O |
| ATOM | 11909 | CB | GLU | H | 42 | 25.298 | 30.620 | -39.638 | 1.00 | 33.30 | C |
| ATOM | 11910 | CG | GLU | H | 42 | 24.814 | 30.500 | -38.189 | 1.00 | 35.92 | C |
| ATOM | 11911 | CD | GLU | H | 42 | 25.309 | 29.222 | -37.518 | 1.00 | 38.02 | C |
| ATOM | 11912 | OE1 | GLU | H | 42 | 26.532 | 28.929 | -37.603 | 1.00 | 41.85 | O |
| ATOM | 11913 | OE2 | GLU | H | 42 | 24.472 | 28.505 | -36.927 | 1.00 | 38.97 | O |
| ATOM | 11914 | N | LYS | H | 43 | 26.188 | 28.491 | -41.916 | 1.00 | 29.59 | N |
| ATOM | 11915 | CA | LYS | H | 43 | 27.127 | 28.244 | -43.013 | 1.00 | 29.50 | C |
| ATOM | 11916 | C | LYS | H | 43 | 26.867 | 26.947 | -43.788 | 1.00 | 26.44 | C |
| ATOM | 11917 | O | LYS | H | 43 | 27.637 | 26.613 | -44.695 | 1.00 | 28.25 | O |
| ATOM | 11918 | CB | LYS | H | 43 | 28.578 | 28.257 | -42.502 | 1.00 | 31.06 | C |
| ATOM | 11919 | CG | LYS | H | 43 | 29.098 | 29.648 | -42.054 | 1.00 | 33.33 | C |
| ATOM | 11920 | CD | LYS | H | 43 | 29.069 | 30.677 | -43.200 | 1.00 | 34.98 | C |
| ATOM | 11921 | CE | LYS | H | 43 | 29.944 | 31.917 | -42.915 | 1.00 | 35.44 | C |
| ATOM | 11922 | NZ | LYS | H | 43 | 30.308 | 32.649 | -44.200 | 1.00 | 36.92 | N |
| ATOM | 11923 | N | GLY | H | 44 | 25.805 | 26.229 | -43.452 | 1.00 | 22.10 | N |
| ATOM | 11924 | CA | GLY | H | 44 | 25.521 | 24.950 | -44.104 | 1.00 | 20.85 | C |
| ATOM | 11925 | C | GLY | H | 44 | 26.682 | 23.980 | -43.951 | 1.00 | 18.54 | C |
| ATOM | 11926 | O | GLY | H | 44 | 27.271 | 23.897 | -42.889 | 1.00 | 19.21 | O |
| ATOM | 11927 | N | LEU | H | 45 | 26.992 | 23.234 | -45.000 | 1.00 | 15.22 | N |
| ATOM | 11928 | CA | LEU | H | 45 | 28.056 | 22.242 | -44.952 | 1.00 | 14.10 | C |
| ATOM | 11929 | C | LEU | H | 45 | 29.376 | 22.919 | -45.308 | 1.00 | 14.32 | C |
| ATOM | 11930 | O | LEU | H | 45 | 29.485 | 23.569 | -46.372 | 1.00 | 15.40 | O |
| ATOM | 11931 | CB | LEU | H | 45 | 27.778 | 21.102 | -45.924 | 1.00 | 13.85 | C |
| ATOM | 11932 | CG | LEU | H | 45 | 26.625 | 20.143 | -45.644 | 1.00 | 14.92 | C |
| ATOM | 11933 | CD1 | LEU | H | 45 | 26.323 | 19.267 | -46.865 | 1.00 | 16.98 | C |
| ATOM | 11934 | CD2 | LEU | H | 45 | 26.953 | 19.277 | -44.431 | 1.00 | 14.92 | C |
| ATOM | 11935 | N | GLU | H | 46 | 30.356 | 22.766 | -44.420 | 1.00 | 12.68 | N |
| ATOM | 11936 | CA | GLU | H | 46 | 31.660 | 23.419 | -44.524 | 1.00 | 14.62 | C |
| ATOM | 11937 | C | GLU | H | 46 | 32.784 | 22.390 | -44.325 | 1.00 | 12.45 | C |
| ATOM | 11938 | O | GLU | H | 46 | 32.988 | 21.916 | -43.225 | 1.00 | 10.59 | O |
| ATOM | 11939 | CB | GLU | H | 46 | 31.785 | 24.473 | -43.448 | 1.00 | 16.04 | C |
| ATOM | 11940 | CG | GLU | H | 46 | 32.869 | 25.464 | -43.670 | 1.00 | 19.50 | C |
| ATOM | 11941 | CD | GLU | H | 46 | 33.000 | 26.457 | -42.499 | 1.00 | 21.58 | C |
| ATOM | 11942 | OE1 | GLU | H | 46 | 32.094 | 26.488 | -41.625 | 1.00 | 22.06 | O |
| ATOM | 11943 | OE2 | GLU | H | 46 | 34.012 | 27.198 | -42.488 | 1.00 | 25.69 | O |
| ATOM | 11944 | N | TRP | H | 47 | 33.486 | 22.067 | -45.405 | 1.00 | 12.94 | N |
| ATOM | 11945 | CA | TRP | H | 47 | 34.729 | 21.259 | -45.346 | 1.00 | 12.09 | C |
| ATOM | 11946 | C | TRP | H | 47 | 35.765 | 22.050 | -44.555 | 1.00 | 12.91 | C |
| ATOM | 11947 | O | TRP | H | 47 | 35.936 | 23.248 | -44.766 | 1.00 | 11.41 | O |
| ATOM | 11948 | CB | TRP | H | 47 | 35.210 | 20.986 | -46.773 | 1.00 | 12.36 | C |

| ATOM | 11949 | CG | TRP | H | 47 | 36.532 | 20.332 | -46.951 | 1.00 | 13.18 | C |
|------|-------|------|-----|---|-----|--------|--------|---------|------|-------|---|
| ATOM | 11950 | CD1 | TRP | H | 47 | 36.775 | 18.998 | -47.035 | 1.00 | 13.42 | C |
| ATOM | 11951 | CD2 | TRP | H | 47 | 37.797 | 20.983 | -47.145 | 1.00 | 13.41 | C |
| ATOM | 11952 | NE1 | TRP | H | 47 | 38.107 | 18.774 | -47.272 | 1.00 | 13.01 | N |
| ATOM | 11953 | CE2 | TRP | H | 47 | 38.755 | 19.976 | -47.348 | 1.00 | 13.32 | C |
| ATOM | 11954 | CE3 | TRP | H | 47 | 38.207 | 22.320 | -47.175 | 1.00 | 13.81 | C |
| ATOM | 11955 | CZ2 | TRP | H | 47 | 40.091 | 20.261 | -47.565 | 1.00 | 13.08 | C |
| ATOM | 11956 | CZ3 | TRP | H | 47 | 39.542 | 22.597 | -47.396 | 1.00 | 13.78 | C |
| ATOM | 11957 | CH2 | TRP | H | 47 | 40.459 | 21.577 | -47.589 | 1.00 | 14.23 | C |
| ATOM | 11958 | N | VAL | H | 48 | 36.421 | 21.412 | -43.609 | 1.00 | 13.55 | N |
| ATOM | 11959 | CA | VAL | H | 48 | 37.441 | 22.120 | -42.817 | 1.00 | 15.16 | C |
| ATOM | 11960 | C | VAL | H | 48 | 38.848 | 21.605 | -42.872 | 1.00 | 13.23 | C |
| ATOM | 11961 | O | VAL | H | 48 | 39.787 | 22.383 | -42.701 | 1.00 | 13.62 | O |
| ATOM | 11962 | CB | VAL | H | 48 | 37.027 | 22.298 | -41.312 | 1.00 | 15.71 | C |
| ATOM | 11963 | CG1 | VAL | H | 48 | 35.857 | 23.269 | -41.210 | 1.00 | 16.58 | C |
| ATOM | 11964 | CG2 | VAL | H | 48 | 36.729 | 20.983 | -40.609 | 1.00 | 17.32 | C |
| ATOM | 11965 | N | ALA | H | 49 | 39.024 | 20.296 | -43.069 | 1.00 | 12.96 | N |
| ATOM | 11966 | CA | ALA | H | 49 | 40.356 | 19.692 | -43.027 | 1.00 | 13.88 | C |
| ATOM | 11967 | C | ALA | H | 49 | 40.343 | 18.348 | -43.733 | 1.00 | 13.36 | C |
| ATOM | 11968 | O | ALA | H | 49 | 39.336 | 17.642 | -43.706 | 1.00 | 13.97 | O |
| ATOM | 11969 | CB | ALA | H | 49 | 40.820 | 19.498 | -41.587 | 1.00 | 14.05 | C |
| ATOM | 11970 | N | GLU | H | 50 | 41.460 | 18.017 | -44.376 | 1.00 | 14.45 | N |
| ATOM | 11971 | CA | GLU | H | 50 | 41.616 | 16.735 | -45.069 | 1.00 | 14.39 | C |
| ATOM | 11972 | C | GLU | H | 50 | 43.022 | 16.175 | -44.904 | 1.00 | 14.41 | C |
| ATOM | 11973 | O | GLU | H | 50 | 43.972 | 16.899 | -44.798 | 1.00 | 12.91 | O |
| ATOM | 11974 | CB | GLU | H | 50 | 41.303 | 16.913 | -46.540 | 1.00 | 15.12 | C |
| ATOM | 11975 | CG | GLU | H | 50 | 41.319 | 15.659 | -47.371 | 1.00 | 15.35 | C |
| ATOM | 11976 | CD | GLU | H | 50 | 40.477 | 15.777 | -48.620 | 1.00 | 15.08 | C |
| ATOM | 11977 | OE1 | GLU | H | 50 | 39.395 | 16.442 | -48.556 | 1.00 | 15.44 | O |
| ATOM | 11978 | OE2 | GLU | H | 50 | 40.898 | 15.211 | -49.683 | 1.00 | 15.53 | O |
| ATOM | 11979 | N | ILE | H | 51 | 43.127 | 14.857 | -44.854 | 1.00 | 14.32 | N |
| ATOM | 11980 | CA | ILE | H | 51 | 44.433 | 14.235 | -44.776 | 1.00 | 16.46 | C |
| ATOM | 11981 | C | ILE | H | 51 | 44.463 | 13.086 | -45.766 | 1.00 | 17.35 | C |
| ATOM | 11982 | O | ILE | H | 51 | 43.485 | 12.357 | -45.907 | 1.00 | 18.21 | O |
| ATOM | 11983 | CB | ILE | H | 51 | 44.761 | 13.778 | -43.343 | 1.00 | 15.50 | C |
| ATOM | 11984 | CG1 | ILE | H | 51 | 46.202 | 13.287 | -43.250 | 1.00 | 17.36 | C |
| ATOM | 11985 | CG2 | ILE | H | 51 | 43.747 | 12.756 | -42.873 | 1.00 | 17.18 | C |
| ATOM | 11986 | CD1 | ILE | H | 51 | 46.641 | 12.944 | -41.847 | 1.00 | 16.27 | C |
| ATOM | 11987 | N | ARG | H | 52 | 45.591 | 12.960 | -46.458 | 1.00 | 19.67 | N |
| ATOM | 11988 | CA | ARG | H | 52 | 45.771 | 11.949 | -47.502 | 1.00 | 20.23 | C |
| ATOM | 11989 | C | ARG | H | 52 | 46.514 | 10.700 | -46.981 | 1.00 | 21.29 | C |
| ATOM | 11990 | O | ARG | H | 52 | 46.485 | 10.414 | -45.784 | 1.00 | 20.22 | O |
| ATOM | 11991 | CB | ARG | H | 52 | 46.466 | 12.610 | -48.695 | 1.00 | 20.73 | C |
| ATOM | 11992 | CG | ARG | H | 52 | 45.701 | 13.809 | -49.288 | 1.00 | 20.16 | C |
| ATOM | 11993 | CD | ARG | H | 52 | 44.424 | 13.376 | -49.939 | 1.00 | 20.17 | C |
| ATOM | 11994 | NE | ARG | H | 52 | 43.553 | 14.477 | -50.363 | 1.00 | 19.03 | N |
| ATOM | 11995 | CZ | ARG | H | 52 | 43.695 | 15.183 | -51.475 | 1.00 | 18.72 | C |
| ATOM | 11996 | NH1 | ARG | H | 52 | 44.700 | 14.949 | -52.291 | 1.00 | 19.52 | N |
| ATOM | 11997 | NH2 | ARG | H | 52 | 42.838 | 16.155 | -51.767 | 1.00 | 20.04 | N |
| ATOM | 11998 | N | LEU | H | 52A | 47.160 | 9.946 | -47.869 | 1.00 | 21.55 | N |
| ATOM | 11999 | CA | LEU | H | 52A | 47.669 | 8.618 | -47.511 | 1.00 | 23.23 | C |
| ATOM | 12000 | C | LEU | H | 52A | 49.124 | 8.571 | -47.054 | 1.00 | 24.16 | C |
| ATOM | 12001 | O | LEU | H | 52A | 49.894 | 9.525 | -47.223 | 1.00 | 24.67 | O |
| ATOM | 12002 | CB | LEU | H | 52A | 47.473 | 7.649 | -48.679 | 1.00 | 23.12 | C |
| ATOM | 12003 | CG | LEU | H | 52A | 46.072 | 7.595 | -49.284 | 1.00 | 23.30 | C |
| ATOM | 12004 | CD1 | LEU | H | 52A | 46.041 | 6.546 | -50.387 | 1.00 | 24.29 | C |
| ATOM | 12005 | CD2 | LEU | H | 52A | 45.001 | 7.326 | -48.219 | 1.00 | 22.21 | C |
| ATOM | 12006 | N | LYS | H | 52B | 49.500 | 7.442 | -46.466 | 1.00 | 25.67 | N |
| ATOM | 12007 | CA | LYS | H | 52B | 50.898 | 7.193 | -46.137 | 1.00 | 27.54 | C |
| ATOM | 12008 | C | LYS | H | 52B | 51.781 | 7.451 | -47.371 | 1.00 | 27.13 | C |
| ATOM | 12009 | O | LYS | H | 52B | 52.818 | 8.089 | -47.274 | 1.00 | 26.79 | O |
| ATOM | 12010 | CB | LYS | H | 52B | 51.077 | 5.758 | -45.615 | 1.00 | 28.47 | C |
| ATOM | 12011 | CG | LYS | H | 52B | 52.466 | 5.498 | -45.089 | 1.00 | 30.33 | C |
| ATOM | 12012 | CD | LYS | H | 52B | 52.576 | 4.130 | -44.444 | 1.00 | 31.24 | C |
| ATOM | 12013 | CE | LYS | H | 52B | 54.003 | 3.912 | -43.943 | 1.00 | 33.30 | C |

| ATOM | 12014 | NZ | LYS | H | 52B | 54.088 | 2.786 | -42.954 | 1.00 | 36.82 | N |
|------|-------|-----|-----|---|-----|--------|--------|---------|------|-------|---|
| ATOM | 12015 | N | SER | H | 52C | 51.328 | 7.016 | -48.543 | 1.00 | 27.78 | N |
| ATOM | 12016 | CA | SER | H | 52C | 52.073 | 7.231 | -49.804 | 1.00 | 28.29 | C |
| ATOM | 12017 | C | SER | H | 52C | 52.201 | 8.704 | -50.220 | 1.00 | 29.51 | C |
| ATOM | 12018 | O | SER | H | 52C | 53.053 | 9.043 | -51.037 | 1.00 | 29.36 | O |
| ATOM | 12019 | CB | SER | H | 52C | 51.455 | 6.392 | -50.941 | 1.00 | 28.58 | C |
| ATOM | 12020 | OG | SER | H | 52C | 50.039 | 6.302 | -50.821 | 1.00 | 28.72 | O |
| ATOM | 12021 | N | ASP | H | 53 | 51.378 | 9.582 | -49.641 | 1.00 | 29.00 | N |
| ATOM | 12022 | CA | ASP | H | 53 | 51.471 | 11.032 | -49.903 | 1.00 | 29.46 | C |
| ATOM | 12023 | C | ASP | H | 53 | 52.127 | 11.769 | -48.735 | 1.00 | 29.51 | C |
| ATOM | 12024 | O | ASP | H | 53 | 52.005 | 13.003 | -48.608 | 1.00 | 29.38 | O |
| ATOM | 12025 | CB | ASP | H | 53 | 50.070 | 11.598 | -50.155 | 1.00 | 29.74 | C |
| ATOM | 12026 | CG | ASP | H | 53 | 49.227 | 10.679 | -50.998 | 1.00 | 30.47 | C |
| ATOM | 12027 | OD1 | ASP | H | 53 | 49.624 | 10.438 | -52.163 | 1.00 | 31.28 | O |
| ATOM | 12028 | OD2 | ASP | H | 53 | 48.177 | 10.191 | -50.517 | 1.00 | 26.92 | O |
| ATOM | 12029 | N | ASN | H | 54 | 52.855 | 11.006 | -47.918 | 1.00 | 29.36 | N |
| ATOM | 12030 | CA | ASN | H | 54 | 53.431 | 11.472 | -46.659 | 1.00 | 29.90 | C |
| ATOM | 12031 | C | ASN | H | 54 | 52.381 | 12.096 | -45.748 | 1.00 | 27.41 | C |
| ATOM | 12032 | O | ASN | H | 54 | 52.635 | 13.090 | -45.074 | 1.00 | 25.63 | O |
| ATOM | 12033 | CB | ASN | H | 54 | 54.604 | 12.424 | -46.901 | 1.00 | 31.74 | C |
| ATOM | 12034 | CG | ASN | H | 54 | 55.793 | 11.730 | -47.559 | 1.00 | 35.72 | C |
| ATOM | 12035 | OD1 | ASN | H | 54 | 56.163 | 10.599 | -47.192 | 1.00 | 39.33 | O |
| ATOM | 12036 | ND2 | ASN | H | 54 | 56.392 | 12.397 | -48.543 | 1.00 | 37.21 | N |
| ATOM | 12037 | N | TYR | H | 55 | 51.196 | 11.493 | -45.741 | 1.00 | 25.14 | N |
| ATOM | 12038 | CA | TYR | H | 55 | 50.097 | 11.960 | -44.898 | 1.00 | 25.03 | C |
| ATOM | 12039 | C | TYR | H | 55 | 49.851 | 13.462 | -45.071 | 1.00 | 22.91 | C |
| ATOM | 12040 | O | TYR | H | 55 | 49.627 | 14.186 | -44.090 | 1.00 | 22.90 | O |
| ATOM | 12041 | CB | TYR | H | 55 | 50.360 | 11.620 | -43.420 | 1.00 | 25.62 | C |
| ATOM | 12042 | CG | TYR | H | 55 | 50.376 | 10.145 | -43.108 | 1.00 | 26.43 | C |
| ATOM | 12043 | CD1 | TYR | H | 55 | 49.236 | 9.381 | -43.247 | 1.00 | 27.18 | C |
| ATOM | 12044 | CD2 | TYR | H | 55 | 51.525 | 9.519 | -42.643 | 1.00 | 27.29 | C |
| ATOM | 12045 | CE1 | TYR | H | 55 | 49.235 | 8.021 | -42.966 | 1.00 | 27.92 | C |
| ATOM | 12046 | CE2 | TYR | H | 55 | 51.529 | 8.153 | -42.344 | 1.00 | 28.99 | C |
| ATOM | 12047 | CZ | TYR | H | 55 | 50.385 | 7.417 | -42.507 | 1.00 | 26.87 | C |
| ATOM | 12048 | OH | TYR | H | 55 | 50.383 | 6.078 | -42.208 | 1.00 | 29.67 | O |
| ATOM | 12049 | N | ALA | H | 56 | 49.888 | 13.925 | -46.317 | 1.00 | 21.26 | N |
| ATOM | 12050 | CA | ALA | H | 56 | 49.636 | 15.345 | -46.630 | 1.00 | 19.75 | C |
| ATOM | 12051 | C | ALA | H | 56 | 48.294 | 15.864 | -46.044 | 1.00 | 19.56 | C |
| ATOM | 12052 | O | ALA | H | 56 | 47.274 | 15.190 | -46.147 | 1.00 | 19.45 | O |
| ATOM | 12053 | CB | ALA | H | 56 | 49.653 | 15.549 | -48.110 | 1.00 | 19.54 | C |
| ATOM | 12054 | N | THR | H | 57 | 48.324 | 17.067 | -45.465 | 1.00 | 19.20 | N |
| ATOM | 12055 | CA | THR | H | 57 | 47.152 | 17.685 | -44.836 | 1.00 | 18.51 | C |
| ATOM | 12056 | C | THR | H | 57 | 46.817 | 19.008 | -45.483 | 1.00 | 18.40 | C |
| ATOM | 12057 | O | THR | H | 57 | 47.712 | 19.746 | -45.938 | 1.00 | 20.11 | O |
| ATOM | 12058 | CB | THR | H | 57 | 47.323 | 17.915 | -43.297 | 1.00 | 18.35 | C |
| ATOM | 12059 | OG1 | THR | H | 57 | 48.363 | 18.862 | -43.044 | 1.00 | 18.62 | O |
| ATOM | 12060 | CG2 | THR | H | 57 | 47.640 | 16.634 | -42.574 | 1.00 | 18.38 | C |
| ATOM | 12061 | N | TYR | H | 58 | 45.511 | 19.284 | -45.541 | 1.00 | 17.89 | N |
| ATOM | 12062 | CA | TYR | H | 58 | 44.948 | 20.511 | -46.141 | 1.00 | 17.06 | C |
| ATOM | 12063 | C | TYR | H | 58 | 43.845 | 21.051 | -45.222 | 1.00 | 15.65 | C |
| ATOM | 12064 | O | TYR | H | 58 | 43.186 | 20.273 | -44.538 | 1.00 | 16.20 | O |
| ATOM | 12065 | CB | TYR | H | 58 | 44.385 | 20.192 | -47.520 | 1.00 | 18.73 | C |
| ATOM | 12066 | CG | TYR | H | 58 | 45.405 | 19.476 | -48.401 | 1.00 | 18.75 | C |
| ATOM | 12067 | CD1 | TYR | H | 58 | 46.331 | 20.187 | -49.145 | 1.00 | 21.66 | C |
| ATOM | 12068 | CD2 | TYR | H | 58 | 45.452 | 18.100 | -48.448 | 1.00 | 20.15 | C |
| ATOM | 12069 | CE1 | TYR | H | 58 | 47.273 | 19.539 | -49.930 | 1.00 | 19.71 | C |
| ATOM | 12070 | CE2 | TYR | H | 58 | 46.398 | 17.430 | -49.227 | 1.00 | 21.38 | C |
| ATOM | 12071 | CZ | TYR | H | 58 | 47.301 | 18.160 | -49.967 | 1.00 | 21.75 | C |
| ATOM | 12072 | OH | TYR | H | 58 | 48.229 | 17.492 | -50.744 | 1.00 | 20.79 | O |
| ATOM | 12073 | N | TYR | H | 59 | 43.652 | 22.367 | -45.205 | 1.00 | 14.59 | N |
| ATOM | 12074 | CA | TYR | H | 59 | 42.738 | 23.020 | -44.249 | 1.00 | 15.42 | C |
| ATOM | 12075 | C | TYR | H | 59 | 41.959 | 24.142 | -44.922 | 1.00 | 14.33 | C |
| ATOM | 12076 | O | TYR | H | 59 | 42.466 | 24.832 | -45.818 | 1.00 | 15.86 | O |
| ATOM | 12077 | CB | TYR | H | 59 | 43.502 | 23.639 | -43.074 | 1.00 | 15.41 | C |
| ATOM | 12078 | CG | TYR | H | 59 | 44.193 | 22.628 | -42.230 | 1.00 | 15.14 | C |

```
ATOM  12079  CD1 TYR H  59   43.495  21.923 -41.242  1.00 14.87      C
ATOM  12080  CD2 TYR H  59   45.534  22.327 -42.432  1.00 16.25      C
ATOM  12081  CE1 TYR H  59   44.115  20.953 -40.490  1.00 15.70      C
ATOM  12082  CE2 TYR H  59   46.170  21.354 -41.664  1.00 16.06      C
ATOM  12083  CZ  TYR H  59   45.449  20.662 -40.715  1.00 16.35      C
ATOM  12084  OH  TYR H  59   46.064  19.724 -39.939  1.00 15.73      O
ATOM  12085  N   ALA H  60   40.731  24.345 -44.484  1.00 14.36      N
ATOM  12086  CA  ALA H  60   39.993  25.517 -44.905  1.00 15.91      C
ATOM  12087  C   ALA H  60   40.696  26.742 -44.312  1.00 15.98      C
ATOM  12088  O   ALA H  60   41.226  26.684 -43.215  1.00 15.47      O
ATOM  12089  CB  ALA H  60   38.594  25.452 -44.430  1.00 16.71      C
ATOM  12090  N   GLU H  61   40.668  27.856 -45.033  1.00 18.73      N
ATOM  12091  CA  GLU H  61   41.266  29.092 -44.515  1.00 21.30      C
ATOM  12092  C   GLU H  61   40.659  29.499 -43.169  1.00 22.18      C
ATOM  12093  O   GLU H  61   41.357  30.021 -42.299  1.00 22.75      O
ATOM  12094  CB  GLU H  61   41.115  30.241 -45.524  1.00 22.78      C
ATOM  12095  CG  GLU H  61   41.986  30.071 -46.749  1.00 24.03      C
ATOM  12096  CD  GLU H  61   43.473  30.168 -46.422  1.00 27.66      C
ATOM  12097  OE1 GLU H  61   43.872  31.129 -45.722  1.00 29.48      O
ATOM  12098  OE2 GLU H  61   44.243  29.281 -46.860  1.00 28.25      O
ATOM  12099  N   SER H  62   39.373  29.221 -42.980  1.00 23.23      N
ATOM  12100  CA  SER H  62   38.688  29.568 -41.726  1.00 23.41      C
ATOM  12101  C   SER H  62   39.238  28.896 -40.466  1.00 23.42      C
ATOM  12102  O   SER H  62   38.999  29.376 -39.361  1.00 25.16      O
ATOM  12103  CB  SER H  62   37.193  29.256 -41.839  1.00 23.58      C
ATOM  12104  OG  SER H  62   36.927  27.883 -42.104  1.00 23.39      O
ATOM  12105  N   VAL H  63   39.964  27.794 -40.605  1.00 21.35      N
ATOM  12106  CA  VAL H  63   40.474  27.091 -39.432  1.00 20.75      C
ATOM  12107  C   VAL H  63   41.979  26.861 -39.465  1.00 21.10      C
ATOM  12108  O   VAL H  63   42.532  26.217 -38.572  1.00 20.09      O
ATOM  12109  CB  VAL H  63   39.766  25.736 -39.269  1.00 20.09      C
ATOM  12110  CG1 VAL H  63   38.254  25.928 -39.235  1.00 20.08      C
ATOM  12111  CG2 VAL H  63   40.181  24.789 -40.378  1.00 18.48      C
ATOM  12112  N   LYS H  64   42.665  27.387 -40.482  1.00 23.87      N
ATOM  12113  CA  LYS H  64   44.084  27.120 -40.590  1.00 25.36      C
ATOM  12114  C   LYS H  64   44.805  27.802 -39.420  1.00 24.87      C
ATOM  12115  O   LYS H  64   44.497  28.946 -39.063  1.00 24.92      O
ATOM  12116  CB  LYS H  64   44.650  27.492 -41.983  1.00 26.19      C
ATOM  12117  CG  LYS H  64   45.442  28.777 -42.091  1.00 28.09      C
ATOM  12118  CD  LYS H  64   46.109  28.827 -43.470  1.00 28.29      C
ATOM  12119  CE  LYS H  64   47.211  29.852 -43.549  1.00 31.05      C
ATOM  12120  NZ  LYS H  64   47.990  29.748 -44.836  1.00 31.05      N
ATOM  12121  N   GLY H  65   45.732  27.069 -38.811  1.00 25.12      N
ATOM  12122  CA  GLY H  65   46.421  27.523 -37.614  1.00 24.82      C
ATOM  12123  C   GLY H  65   45.728  27.106 -36.319  1.00 25.69      C
ATOM  12124  O   GLY H  65   46.367  27.077 -35.264  1.00 28.39      O
ATOM  12125  N   LYS H  66   44.439  26.764 -36.395  1.00 23.01      N
ATOM  12126  CA  LYS H  66   43.629  26.400 -35.218  1.00 22.69      C
ATOM  12127  C   LYS H  66   43.364  24.919 -35.079  1.00 20.01      C
ATOM  12128  O   LYS H  66   43.254  24.428 -33.958  1.00 19.68      O
ATOM  12129  CB  LYS H  66   42.240  27.020 -35.301  1.00 23.63      C
ATOM  12130  CG  LYS H  66   42.187  28.479 -35.140  1.00 26.59      C
ATOM  12131  CD  LYS H  66   40.828  28.988 -35.556  1.00 26.11      C
ATOM  12132  CE  LYS H  66   39.695  28.380 -34.796  1.00 24.20      C
ATOM  12133  NZ  LYS H  66   38.541  29.327 -34.714  1.00 21.04      N
ATOM  12134  N   PHE H  67   43.152  24.242 -36.206  1.00 17.16      N
ATOM  12135  CA  PHE H  67   42.791  22.828 -36.222  1.00 16.31      C
ATOM  12136  C   PHE H  67   43.951  22.012 -36.775  1.00 16.72      C
ATOM  12137  O   PHE H  67   44.705  22.490 -37.650  1.00 14.78      O
ATOM  12138  CB  PHE H  67   41.585  22.568 -37.121  1.00 16.44      C
ATOM  12139  CG  PHE H  67   40.262  23.060 -36.584  1.00 14.76      C
ATOM  12140  CD1 PHE H  67   40.165  23.864 -35.454  1.00 16.50      C
ATOM  12141  CD2 PHE H  67   39.104  22.751 -37.263  1.00 16.85      C
ATOM  12142  CE1 PHE H  67   38.918  24.328 -35.008  1.00 14.63      C
ATOM  12143  CE2 PHE H  67   37.878  23.208 -36.828  1.00 15.78      C
```

EP 2 123 668 A1

| ATOM | 12144 | CZ | PHE | H | 67 | 37.788 | 24.001 | -35.715 | 1.00 | 16.23 | C |
|---|---|---|---|---|---|---|---|---|---|---|---|
| ATOM | 12145 | N | THR | H | 68 | 44.050 | 20.770 | -36.311 | 1.00 | 17.89 | N |
| ATOM | 12146 | CA | THR | H | 68 | 45.084 | 19.846 | -36.772 | 1.00 | 17.36 | C |
| ATOM | 12147 | C | THR | H | 68 | 44.432 | 18.485 | -36.956 | 1.00 | 17.21 | C |
| ATOM | 12148 | O | THR | H | 68 | 43.888 | 17.899 | -36.008 | 1.00 | 16.39 | O |
| ATOM | 12149 | CB | THR | H | 68 | 46.241 | 19.745 | -35.746 | 1.00 | 18.79 | C |
| ATOM | 12150 | OG1 | THR | H | 68 | 46.916 | 21.009 | -35.643 | 1.00 | 19.78 | O |
| ATOM | 12151 | CG2 | THR | H | 68 | 47.248 | 18.704 | -36.162 | 1.00 | 20.43 | C |
| ATOM | 12152 | N | ILE | H | 69 | 44.443 | 18.003 | -38.188 | 1.00 | 16.34 | N |
| ATOM | 12153 | CA | ILE | H | 69 | 43.867 | 16.699 | -38.526 | 1.00 | 15.18 | C |
| ATOM | 12154 | C | ILE | H | 69 | 44.997 | 15.665 | -38.527 | 1.00 | 16.35 | C |
| ATOM | 12155 | O | ILE | H | 69 | 46.099 | 15.981 | -38.953 | 1.00 | 16.62 | O |
| ATOM | 12156 | CB | ILE | H | 69 | 43.140 | 16.776 | -39.900 | 1.00 | 15.19 | C |
| ATOM | 12157 | CG1 | ILE | H | 69 | 42.377 | 15.474 | -40.188 | 1.00 | 14.82 | C |
| ATOM | 12158 | CG2 | ILE | H | 69 | 44.117 | 17.108 | -41.047 | 1.00 | 13.39 | C |
| ATOM | 12159 | CD1 | ILE | H | 69 | 41.483 | 15.498 | -41.438 | 1.00 | 15.07 | C |
| ATOM | 12160 | N | SER | H | 70 | 44.707 | 14.456 | -38.042 | 1.00 | 16.05 | N |
| ATOM | 12161 | CA | SER | H | 70 | 45.651 | 13.322 | -38.055 | 1.00 | 16.96 | C |
| ATOM | 12162 | C | SER | H | 70 | 44.920 | 11.998 | -38.148 | 1.00 | 17.30 | C |
| ATOM | 12163 | O | SER | H | 70 | 43.715 | 11.924 | -37.952 | 1.00 | 18.60 | O |
| ATOM | 12164 | CB | SER | H | 70 | 46.570 | 13.334 | -36.817 | 1.00 | 16.89 | C |
| ATOM | 12165 | OG | SER | H | 70 | 45.830 | 13.302 | -35.606 | 1.00 | 17.35 | O |
| ATOM | 12166 | N | ARG | H | 71 | 45.662 | 10.934 | -38.460 | 1.00 | 18.70 | N |
| ATOM | 12167 | CA | ARG | H | 71 | 45.069 | 9.601 | -38.601 | 1.00 | 19.64 | C |
| ATOM | 12168 | C | ARG | H | 71 | 45.990 | 8.572 | -37.968 | 1.00 | 20.42 | C |
| ATOM | 12169 | O | ARG | H | 71 | 47.194 | 8.758 | -37.915 | 1.00 | 19.36 | O |
| ATOM | 12170 | CB | ARG | H | 71 | 44.830 | 9.253 | -40.087 | 1.00 | 19.71 | C |
| ATOM | 12171 | CG | ARG | H | 71 | 46.070 | 9.271 | -40.975 | 1.00 | 18.47 | C |
| ATOM | 12172 | CD | ARG | H | 71 | 45.710 | 9.278 | -42.466 | 1.00 | 19.57 | C |
| ATOM | 12173 | NE | ARG | H | 71 | 44.930 | 8.098 | -42.844 | 1.00 | 21.06 | N |
| ATOM | 12174 | CZ | ARG | H | 71 | 44.138 | 8.009 | -43.908 | 1.00 | 19.34 | C |
| ATOM | 12175 | NH1 | ARG | H | 71 | 43.981 | 9.039 | -44.716 | 1.00 | 20.80 | N |
| ATOM | 12176 | NH2 | ARG | H | 71 | 43.475 | 6.885 | -44.130 | 1.00 | 20.38 | N |
| ATOM | 12177 | N | ASP | H | 72 | 45.400 | 7.496 | -37.482 | 1.00 | 22.01 | N |
| ATOM | 12178 | CA | ASP | H | 72 | 46.144 | 6.360 | -36.975 | 1.00 | 23.80 | C |
| ATOM | 12179 | C | ASP | H | 72 | 45.622 | 5.169 | -37.737 | 1.00 | 23.63 | C |
| ATOM | 12180 | O | ASP | H | 72 | 44.590 | 4.605 | -37.377 | 1.00 | 24.63 | O |
| ATOM | 12181 | CB | ASP | H | 72 | 45.901 | 6.189 | -35.479 | 1.00 | 25.36 | C |
| ATOM | 12182 | CG | ASP | H | 72 | 46.717 | 5.049 | -34.881 | 1.00 | 28.50 | C |
| ATOM | 12183 | OD1 | ASP | H | 72 | 47.162 | 4.130 | -35.625 | 1.00 | 30.20 | O |
| ATOM | 12184 | OD2 | ASP | H | 72 | 46.924 | 5.089 | -33.658 | 1.00 | 30.41 | O |
| ATOM | 12185 | N | ASP | H | 73 | 46.314 | 4.811 | -38.817 | 1.00 | 25.04 | N |
| ATOM | 12186 | CA | ASP | H | 73 | 45.860 | 3.728 | -39.706 | 1.00 | 26.19 | C |
| ATOM | 12187 | C | ASP | H | 73 | 45.719 | 2.397 | -38.960 | 1.00 | 26.62 | C |
| ATOM | 12188 | O | ASP | H | 73 | 44.778 | 1.650 | -39.188 | 1.00 | 26.54 | O |
| ATOM | 12189 | CB | ASP | H | 73 | 46.804 | 3.580 | -40.913 | 1.00 | 27.01 | C |
| ATOM | 12190 | CG | ASP | H | 73 | 46.668 | 4.732 | -41.922 | 1.00 | 27.86 | C |
| ATOM | 12191 | OD1 | ASP | H | 73 | 45.615 | 5.402 | -41.915 | 1.00 | 26.03 | O |
| ATOM | 12192 | OD2 | ASP | H | 73 | 47.597 | 4.963 | -42.739 | 1.00 | 29.33 | O |
| ATOM | 12193 | N | SER | H | 74 | 46.628 | 2.127 | -38.032 | 1.00 | 28.47 | N |
| ATOM | 12194 | CA | SER | H | 74 | 46.593 | 0.870 | -37.293 | 1.00 | 28.70 | C |
| ATOM | 12195 | C | SER | H | 74 | 45.308 | 0.708 | -36.466 | 1.00 | 29.07 | C |
| ATOM | 12196 | O | SER | H | 74 | 44.868 | -0.423 | -36.203 | 1.00 | 29.35 | O |
| ATOM | 12197 | CB | SER | H | 74 | 47.821 | 0.736 | -36.388 | 1.00 | 28.89 | C |
| ATOM | 12198 | OG | SER | H | 74 | 47.725 | 1.585 | -35.259 | 1.00 | 29.52 | O |
| ATOM | 12199 | N | LYS | H | 75 | 44.706 | 1.828 | -36.048 | 1.00 | 28.12 | N |
| ATOM | 12200 | CA | LYS | H | 75 | 43.469 | 1.782 | -35.272 | 1.00 | 27.76 | C |
| ATOM | 12201 | C | LYS | H | 75 | 42.236 | 2.160 | -36.091 | 1.00 | 25.55 | C |
| ATOM | 12202 | O | LYS | H | 75 | 41.131 | 2.167 | -35.555 | 1.00 | 24.68 | O |
| ATOM | 12203 | CB | LYS | H | 75 | 43.544 | 2.737 | -34.087 | 1.00 | 27.56 | C |
| ATOM | 12204 | CG | LYS | H | 75 | 44.756 | 2.618 | -33.193 | 1.00 | 30.32 | C |
| ATOM | 12205 | CD | LYS | H | 75 | 44.606 | 3.646 | -32.068 | 1.00 | 31.69 | C |
| ATOM | 12206 | CE | LYS | H | 75 | 45.471 | 3.345 | -30.846 | 1.00 | 34.26 | C |
| ATOM | 12207 | NZ | LYS | H | 75 | 44.822 | 3.953 | -29.638 | 1.00 | 34.80 | N |
| ATOM | 12208 | N | SER | H | 76 | 42.439 | 2.485 | -37.369 | 1.00 | 23.86 | N |

446

| ATOM | 12209 | CA | SER | H | 76 | 41.376 | 2.933 | -38.268 | 1.00 | 22.52 | C |
|------|-------|-----|-----|---|-----|--------|--------|---------|------|-------|---|
| ATOM | 12210 | C | SER | H | 76 | 40.669 | 4.171 | -37.695 | 1.00 | 20.93 | C |
| ATOM | 12211 | O | SER | H | 76 | 39.456 | 4.292 | -37.795 | 1.00 | 20.33 | O |
| ATOM | 12212 | CB | SER | H | 76 | 40.362 | 1.810 | -38.556 | 1.00 | 23.80 | C |
| ATOM | 12213 | OG | SER | H | 76 | 41.009 | 0.673 | -39.166 | 1.00 | 21.67 | O |
| ATOM | 12214 | N | ARG | H | 77 | 41.452 | 5.089 | -37.137 | 1.00 | 20.48 | N |
| ATOM | 12215 | CA | ARG | H | 77 | 40.894 | 6.253 | -36.434 | 1.00 | 20.60 | C |
| ATOM | 12216 | C | ARG | H | 77 | 41.417 | 7.555 | -37.032 | 1.00 | 18.10 | C |
| ATOM | 12217 | O | ARG | H | 77 | 42.612 | 7.691 | -37.281 | 1.00 | 17.24 | O |
| ATOM | 12218 | CB | ARG | H | 77 | 41.222 | 6.194 | -34.940 | 1.00 | 20.74 | C |
| ATOM | 12219 | CG | ARG | H | 77 | 40.487 | 7.240 | -34.103 | 1.00 | 22.29 | C |
| ATOM | 12220 | CD | ARG | H | 77 | 40.527 | 6.897 | -32.616 | 1.00 | 24.46 | C |
| ATOM | 12221 | NE | ARG | H | 77 | 39.632 | 5.793 | -32.246 | 1.00 | 27.17 | N |
| ATOM | 12222 | CZ | ARG | H | 77 | 38.304 | 5.889 | -32.099 | 1.00 | 29.90 | C |
| ATOM | 12223 | NH1 | ARG | H | 77 | 37.648 | 7.045 | -32.314 | 1.00 | 27.96 | N |
| ATOM | 12224 | NH2 | ARG | H | 77 | 37.607 | 4.807 | -31.749 | 1.00 | 29.09 | N |
| ATOM | 12225 | N | LEU | H | 78 | 40.497 | 8.503 | -37.247 | 1.00 | 17.03 | N |
| ATOM | 12226 | CA | LEU | H | 78 | 40.828 | 9.882 | -37.670 | 1.00 | 15.21 | C |
| ATOM | 12227 | C | LEU | H | 78 | 40.603 | 10.812 | -36.490 | 1.00 | 16.12 | C |
| ATOM | 12228 | O | LEU | H | 78 | 39.681 | 10.599 | -35.755 | 1.00 | 16.83 | O |
| ATOM | 12229 | CB | LEU | H | 78 | 39.875 | 10.308 | -38.785 | 1.00 | 15.46 | C |
| ATOM | 12230 | CG | LEU | H | 78 | 40.037 | 11.694 | -39.436 | 1.00 | 14.89 | C |
| ATOM | 12231 | CD1 | LEU | H | 78 | 41.308 | 11.748 | -40.251 | 1.00 | 16.24 | C |
| ATOM | 12232 | CD2 | LEU | H | 78 | 38.818 | 11.985 | -40.278 | 1.00 | 16.49 | C |
| ATOM | 12233 | N | TYR | H | 79 | 41.425 | 11.853 | -36.356 | 1.00 | 17.02 | N |
| ATOM | 12234 | CA | TYR | H | 79 | 41.324 | 12.817 | -35.255 | 1.00 | 17.49 | C |
| ATOM | 12235 | C | TYR | H | 79 | 41.208 | 14.229 | -35.783 | 1.00 | 16.42 | C |
| ATOM | 12236 | O | TYR | H | 79 | 41.746 | 14.534 | -36.846 | 1.00 | 15.44 | O |
| ATOM | 12237 | CB | TYR | H | 79 | 42.586 | 12.768 | -34.403 | 1.00 | 20.88 | C |
| ATOM | 12238 | CG | TYR | H | 79 | 42.840 | 11.410 | -33.782 | 1.00 | 21.46 | C |
| ATOM | 12239 | CD1 | TYR | H | 79 | 42.204 | 11.045 | -32.608 | 1.00 | 23.18 | C |
| ATOM | 12240 | CD2 | TYR | H | 79 | 43.715 | 10.509 | -34.370 | 1.00 | 23.78 | C |
| ATOM | 12241 | CE1 | TYR | H | 79 | 42.426 | 9.803 | -32.019 | 1.00 | 24.90 | C |
| ATOM | 12242 | CE2 | TYR | H | 79 | 43.952 | 9.241 | -33.786 | 1.00 | 25.86 | C |
| ATOM | 12243 | CZ | TYR | H | 79 | 43.307 | 8.910 | -32.612 | 1.00 | 25.33 | C |
| ATOM | 12244 | OH | TYR | H | 79 | 43.521 | 7.678 | -32.035 | 1.00 | 27.08 | O |
| ATOM | 12245 | N | LEU | H | 80 | 40.558 | 15.097 | -35.003 | 1.00 | 14.29 | N |
| ATOM | 12246 | CA | LEU | H | 80 | 40.639 | 16.543 | -35.222 | 1.00 | 13.43 | C |
| ATOM | 12247 | C | LEU | H | 80 | 40.895 | 17.224 | -33.886 | 1.00 | 13.37 | C |
| ATOM | 12248 | O | LEU | H | 80 | 40.029 | 17.208 | -33.002 | 1.00 | 12.06 | O |
| ATOM | 12249 | CB | LEU | H | 80 | 39.356 | 17.073 | -35.855 | 1.00 | 13.50 | C |
| ATOM | 12250 | CG | LEU | H | 80 | 39.453 | 18.510 | -36.387 | 1.00 | 12.75 | C |
| ATOM | 12251 | CD1 | LEU | H | 80 | 40.475 | 18.636 | -37.508 | 1.00 | 13.96 | C |
| ATOM | 12252 | CD2 | LEU | H | 80 | 38.106 | 19.012 | -36.837 | 1.00 | 13.70 | C |
| ATOM | 12253 | N | GLN | H | 81 | 42.082 | 17.814 | -33.760 | 1.00 | 14.06 | N |
| ATOM | 12254 | CA | GLN | H | 81 | 42.459 | 18.629 | -32.621 | 1.00 | 15.13 | C |
| ATOM | 12255 | C | GLN | H | 81 | 42.068 | 20.074 | -32.941 | 1.00 | 16.25 | C |
| ATOM | 12256 | O | GLN | H | 81 | 42.512 | 20.631 | -33.939 | 1.00 | 15.89 | O |
| ATOM | 12257 | CB | GLN | H | 81 | 43.960 | 18.528 | -32.352 | 1.00 | 15.97 | C |
| ATOM | 12258 | CG | GLN | H | 81 | 44.447 | 19.414 | -31.217 | 1.00 | 17.80 | C |
| ATOM | 12259 | CD | GLN | H | 81 | 43.878 | 19.010 | -29.867 | 1.00 | 19.68 | C |
| ATOM | 12260 | OE1 | GLN | H | 81 | 43.977 | 17.846 | -29.469 | 1.00 | 19.14 | O |
| ATOM | 12261 | NE2 | GLN | H | 81 | 43.338 | 19.986 | -29.125 | 1.00 | 18.93 | N |
| ATOM | 12262 | N | MET | H | 82 | 41.217 | 20.643 | -32.100 | 1.00 | 15.92 | N |
| ATOM | 12263 | CA | MET | H | 82 | 40.625 | 21.953 | -32.325 | 1.00 | 17.04 | C |
| ATOM | 12264 | C | MET | H | 82 | 41.038 | 22.862 | -31.182 | 1.00 | 16.70 | C |
| ATOM | 12265 | O | MET | H | 82 | 40.652 | 22.637 | -30.024 | 1.00 | 17.24 | O |
| ATOM | 12266 | CB | MET | H | 82 | 39.110 | 21.825 | -32.395 | 1.00 | 16.54 | C |
| ATOM | 12267 | CG | MET | H | 82 | 38.641 | 20.760 | -33.422 | 1.00 | 16.88 | C |
| ATOM | 12268 | SD | MET | H | 82 | 36.955 | 20.127 | -33.138 | 1.00 | 20.57 | S |
| ATOM | 12269 | CE | MET | H | 82 | 36.067 | 21.579 | -33.497 | 1.00 | 16.54 | C |
| ATOM | 12270 | N | ASN | H | 82A | 41.824 | 23.879 | -31.512 | 1.00 | 15.77 | N |
| ATOM | 12271 | CA | ASN | H | 82A | 42.269 | 24.850 | -30.533 | 1.00 | 15.52 | C |
| ATOM | 12272 | C | ASN | H | 82A | 41.687 | 26.220 | -30.862 | 1.00 | 14.35 | C |
| ATOM | 12273 | O | ASN | H | 82A | 41.205 | 26.466 | -31.975 | 1.00 | 13.92 | O |

```
ATOM  12274  CB  ASN H  82A    43.804  24.897 -30.490  1.00 16.44           C
ATOM  12275  CG  ASN H  82A    44.420  23.537 -30.162  1.00 18.03           C
ATOM  12276  OD1 ASN H  82A    43.888  22.780 -29.350  1.00 19.30           O
ATOM  12277  ND2 ASN H  82A    45.546  23.225 -30.806  1.00 21.74           N
ATOM  12278  N   ASN H  82B    41.733  27.106 -29.879  1.00 14.77           N
ATOM  12279  CA  ASN H  82B    41.338  28.483 -30.064  1.00 16.89           C
ATOM  12280  C   ASN H  82B    39.934  28.547 -30.630  1.00 15.57           C
ATOM  12281  O   ASN H  82B    39.692  29.232 -31.638  1.00 14.42           O
ATOM  12282  CB  ASN H  82B    42.342  29.208 -30.972  1.00 19.44           C
ATOM  12283  CG  ASN H  82B    41.995  30.677 -31.182  1.00 25.29           C
ATOM  12284  OD1 ASN H  82B    41.389  31.324 -30.317  1.00 29.36           O
ATOM  12285  ND2 ASN H  82B    42.374  31.213 -32.349  1.00 29.88           N
ATOM  12286  N   LEU H  82C    39.012  27.812 -29.993  1.00 13.93           N
ATOM  12287  CA  LEU H  82C    37.657  27.697 -30.506  1.00 13.32           C
ATOM  12288  C   LEU H  82C    36.884  28.965 -30.282  1.00 13.47           C
ATOM  12289  O   LEU H  82C    37.084  29.618 -29.280  1.00 12.89           O
ATOM  12290  CB  LEU H  82C    36.920  26.510 -29.879  1.00 12.43           C
ATOM  12291  CG  LEU H  82C    37.384  25.205 -30.537  1.00 12.59           C
ATOM  12292  CD1 LEU H  82C    36.968  23.976 -29.739  1.00 14.28           C
ATOM  12293  CD2 LEU H  82C    36.826  25.110 -31.957  1.00 16.93           C
ATOM  12294  N   ARG H  83     35.992  29.279 -31.217  1.00 14.17           N
ATOM  12295  CA  ARG H  83     35.141  30.444 -31.117  1.00 14.21           C
ATOM  12296  C   ARG H  83     33.702  30.032 -31.413  1.00 13.58           C
ATOM  12297  O   ARG H  83     33.468  28.922 -31.880  1.00 11.93           O
ATOM  12298  CB  ARG H  83     35.649  31.537 -32.058  1.00 13.99           C
ATOM  12299  CG  ARG H  83     37.105  31.943 -31.814  1.00 14.72           C
ATOM  12300  CD  ARG H  83     37.277  32.838 -30.581  1.00 15.89           C
ATOM  12301  NE  ARG H  83     38.670  32.806 -30.101  1.00 16.19           N
ATOM  12302  CZ  ARG H  83     39.061  33.198 -28.896  1.00 15.53           C
ATOM  12303  NH1 ARG H  83     38.191  33.698 -28.015  1.00 15.09           N
ATOM  12304  NH2 ARG H  83     40.340  33.099 -28.557  1.00 16.17           N
ATOM  12305  N   THR H  84     32.725  30.891 -31.119  1.00 14.01           N
ATOM  12306  CA  THR H  84     31.321  30.456 -31.271  1.00 15.22           C
ATOM  12307  C   THR H  84     31.028  29.968 -32.698  1.00 15.26           C
ATOM  12308  O   THR H  84     30.274  29.017 -32.872  1.00 17.16           O
ATOM  12309  CB  THR H  84     30.285  31.498 -30.812  1.00 16.65           C
ATOM  12310  OG1 THR H  84     30.276  32.610 -31.699  1.00 19.75           O
ATOM  12311  CG2 THR H  84     30.616  31.977 -29.424  1.00 17.90           C
ATOM  12312  N   GLU H  85     31.663  30.588 -33.700  1.00 15.33           N
ATOM  12313  CA  GLU H  85     31.467  30.242 -35.106  1.00 15.91           C
ATOM  12314  C   GLU H  85     31.965  28.832 -35.450  1.00 14.39           C
ATOM  12315  O   GLU H  85     31.668  28.290 -36.512  1.00 13.43           O
ATOM  12316  CB  GLU H  85     32.162  31.240 -36.054  1.00 18.32           C
ATOM  12317  CG  GLU H  85     33.656  31.433 -35.853  1.00 19.67           C
ATOM  12318  CD  GLU H  85     34.017  32.583 -34.885  1.00 21.39           C
ATOM  12319  OE1 GLU H  85     33.218  32.908 -33.969  1.00 17.84           O
ATOM  12320  OE2 GLU H  85     35.153  33.115 -35.015  1.00 25.67           O
ATOM  12321  N   ASP H  86     32.725  28.246 -34.550  1.00 12.01           N
ATOM  12322  CA  ASP H  86     33.107  26.845 -34.710  1.00 11.18           C
ATOM  12323  C   ASP H  86     32.065  25.829 -34.194  1.00 12.24           C
ATOM  12324  O   ASP H  86     32.250  24.615 -34.350  1.00 11.53           O
ATOM  12325  CB  ASP H  86     34.435  26.606 -34.022  1.00 11.79           C
ATOM  12326  CG  ASP H  86     35.581  27.424 -34.637  1.00 11.72           C
ATOM  12327  OD1 ASP H  86     35.788  27.353 -35.877  1.00 11.72           O
ATOM  12328  OD2 ASP H  86     36.275  28.109 -33.864  1.00 12.14           O
ATOM  12329  N   THR H  87     30.985  26.304 -33.589  1.00 11.92           N
ATOM  12330  CA  THR H  87     29.865  25.447 -33.140  1.00 11.12           C
ATOM  12331  C   THR H  87     29.224  24.703 -34.319  1.00 11.86           C
ATOM  12332  O   THR H  87     28.922  25.303 -35.356  1.00 12.18           O
ATOM  12333  CB  THR H  87     28.799  26.295 -32.400  1.00 10.49           C
ATOM  12334  OG1 THR H  87     29.340  26.777 -31.162  1.00 10.72           O
ATOM  12335  CG2 THR H  87     27.543  25.495 -32.108  1.00 12.22           C
ATOM  12336  N   GLY H  88     29.027  23.392 -34.197  1.00 10.10           N
ATOM  12337  CA  GLY H  88     28.301  22.653 -35.246  1.00  9.76           C
ATOM  12338  C   GLY H  88     28.389  21.161 -35.067  1.00  9.45           C
```

| ATOM | 12339 | O   | GLY | H | 88 | 28.966 | 20.700 | -34.111 | 1.00 | 9.67  | O |
|------|-------|-----|-----|---|----|--------|--------|---------|------|-------|---|
| ATOM | 12340 | N   | ILE | H | 89 | 27.814 | 20.446 | -36.019 | 1.00 | 9.41  | N |
| ATOM | 12341 | CA  | ILE | H | 89 | 27.904 | 18.996 | -36.077 | 1.00 | 12.01 | C |
| ATOM | 12342 | C   | ILE | H | 89 | 29.096 | 18.699 | -36.955 | 1.00 | 12.00 | C |
| ATOM | 12343 | O   | ILE | H | 89 | 29.143 | 19.153 | -38.088 | 1.00 | 12.01 | O |
| ATOM | 12344 | CB  | ILE | H | 89 | 26.660 | 18.366 | -36.721 | 1.00 | 12.21 | C |
| ATOM | 12345 | CG1 | ILE | H | 89 | 25.367 | 18.754 | -36.009 | 1.00 | 15.10 | C |
| ATOM | 12346 | CG2 | ILE | H | 89 | 26.812 | 16.839 | -36.757 | 1.00 | 11.76 | C |
| ATOM | 12347 | CD1 | ILE | H | 89 | 25.344 | 18.474 | -34.577 | 1.00 | 19.09 | C |
| ATOM | 12348 | N   | TYR | H | 90 | 30.066 | 17.944 | -36.435 | 1.00 | 13.01 | N |
| ATOM | 12349 | CA  | TYR | H | 90 | 31.266 | 17.613 | -37.180 | 1.00 | 10.74 | C |
| ATOM | 12350 | C   | TYR | H | 90 | 31.111 | 16.193 | -37.723 | 1.00 | 14.10 | C |
| ATOM | 12351 | O   | TYR | H | 90 | 30.884 | 15.235 | -36.948 | 1.00 | 13.49 | O |
| ATOM | 12352 | CB  | TYR | H | 90 | 32.516 | 17.748 | -36.312 | 1.00 | 10.60 | C |
| ATOM | 12353 | CG  | TYR | H | 90 | 32.913 | 19.198 | -36.123 | 1.00 | 8.52  | C |
| ATOM | 12354 | CD1 | TYR | H | 90 | 32.099 | 20.062 | -35.404 | 1.00 | 10.90 | C |
| ATOM | 12355 | CD2 | TYR | H | 90 | 34.064 | 19.707 | -36.716 | 1.00 | 9.28  | C |
| ATOM | 12356 | CE1 | TYR | H | 90 | 32.440 | 21.394 | -35.245 | 1.00 | 11.14 | C |
| ATOM | 12357 | CE2 | TYR | H | 90 | 34.390 | 21.063 | -36.577 | 1.00 | 11.35 | C |
| ATOM | 12358 | CZ  | TYR | H | 90 | 33.582 | 21.886 | -35.859 | 1.00 | 9.69  | C |
| ATOM | 12359 | OH  | TYR | H | 90 | 33.966 | 23.241 | -35.718 | 1.00 | 9.38  | O |
| ATOM | 12360 | N   | TYR | H | 91 | 31.170 | 16.098 | -39.052 | 1.00 | 13.68 | N |
| ATOM | 12361 | CA  | TYR | H | 91 | 31.138 | 14.804 | -39.754 | 1.00 | 15.66 | C |
| ATOM | 12362 | C   | TYR | H | 91 | 32.497 | 14.346 | -40.269 | 1.00 | 14.55 | C |
| ATOM | 12363 | O   | TYR | H | 91 | 33.275 | 15.127 | -40.863 | 1.00 | 13.76 | O |
| ATOM | 12364 | CB  | TYR | H | 91 | 30.203 | 14.868 | -40.960 | 1.00 | 15.99 | C |
| ATOM | 12365 | CG  | TYR | H | 91 | 28.765 | 15.079 | -40.692 | 1.00 | 15.64 | C |
| ATOM | 12366 | CD1 | TYR | H | 91 | 27.927 | 14.016 | -40.343 | 1.00 | 14.60 | C |
| ATOM | 12367 | CD2 | TYR | H | 91 | 28.196 | 16.342 | -40.831 | 1.00 | 15.39 | C |
| ATOM | 12368 | CE1 | TYR | H | 91 | 26.564 | 14.226 | -40.119 | 1.00 | 15.45 | C |
| ATOM | 12369 | CE2 | TYR | H | 91 | 26.863 | 16.553 | -40.616 | 1.00 | 16.62 | C |
| ATOM | 12370 | CZ  | TYR | H | 91 | 26.048 | 15.506 | -40.265 | 1.00 | 16.96 | C |
| ATOM | 12371 | OH  | TYR | H | 91 | 24.723 | 15.766 | -40.073 | 1.00 | 18.50 | O |
| ATOM | 12372 | N   | CYS | H | 92 | 32.789 | 13.050 | -40.098 | 1.00 | 15.43 | N |
| ATOM | 12373 | CA  | CYS | H | 92 | 33.831 | 12.451 | -40.885 | 1.00 | 16.54 | C |
| ATOM | 12374 | C   | CYS | H | 92 | 33.246 | 12.240 | -42.275 | 1.00 | 15.72 | C |
| ATOM | 12375 | O   | CYS | H | 92 | 32.139 | 11.720 | -42.414 | 1.00 | 14.72 | O |
| ATOM | 12376 | CB  | CYS | H | 92 | 34.353 | 11.141 | -40.274 | 1.00 | 18.81 | C |
| ATOM | 12377 | SG  | CYS | H | 92 | 35.508 | 11.553 | -38.974 | 1.00 | 26.20 | S |
| ATOM | 12378 | N   | PHE | H | 93 | 33.977 | 12.727 | -43.272 | 1.00 | 15.32 | N |
| ATOM | 12379 | CA  | PHE | H | 93 | 33.620 | 12.587 | -44.680 | 1.00 | 16.12 | C |
| ATOM | 12380 | C   | PHE | H | 93 | 34.817 | 12.016 | -45.441 | 1.00 | 16.29 | C |
| ATOM | 12381 | O   | PHE | H | 93 | 35.874 | 12.633 | -45.502 | 1.00 | 17.49 | O |
| ATOM | 12382 | CB  | PHE | H | 93 | 33.229 | 13.942 | -45.240 | 1.00 | 16.45 | C |
| ATOM | 12383 | CG  | PHE | H | 93 | 32.919 | 13.958 | -46.716 | 1.00 | 16.76 | C |
| ATOM | 12384 | CD1 | PHE | H | 93 | 31.982 | 13.098 | -47.265 | 1.00 | 16.36 | C |
| ATOM | 12385 | CD2 | PHE | H | 93 | 33.547 | 14.873 | -47.544 | 1.00 | 16.53 | C |
| ATOM | 12386 | CE1 | PHE | H | 93 | 31.656 | 13.172 | -48.634 | 1.00 | 15.68 | C |
| ATOM | 12387 | CE2 | PHE | H | 93 | 33.244 | 14.943 | -48.901 | 1.00 | 17.01 | C |
| ATOM | 12388 | CZ  | PHE | H | 93 | 32.301 | 14.091 | -49.439 | 1.00 | 16.11 | C |
| ATOM | 12389 | N   | LEU | H | 94 | 34.630 | 10.814 | -45.996 | 1.00 | 16.47 | N |
| ATOM | 12390 | CA  | LEU | H | 94 | 35.610 | 10.151 | -46.845 | 1.00 | 16.81 | C |
| ATOM | 12391 | C   | LEU | H | 94 | 35.277 | 10.568 | -48.265 | 1.00 | 15.21 | C |
| ATOM | 12392 | O   | LEU | H | 94 | 34.320 | 10.055 | -48.843 | 1.00 | 15.31 | O |
| ATOM | 12393 | CB  | LEU | H | 94 | 35.521 | 8.612  | -46.716 | 1.00 | 16.84 | C |
| ATOM | 12394 | CG  | LEU | H | 94 | 36.074 | 7.964  | -45.438 | 1.00 | 19.78 | C |
| ATOM | 12395 | CD1 | LEU | H | 94 | 35.467 | 8.584  | -44.202 | 1.00 | 19.99 | C |
| ATOM | 12396 | CD2 | LEU | H | 94 | 35.814 | 6.449  | -45.466 | 1.00 | 19.80 | C |
| ATOM | 12397 | N   | PRO | H | 95 | 36.009 | 11.557 | -48.802 | 1.00 | 14.37 | N |
| ATOM | 12398 | CA  | PRO | H | 95 | 35.597 | 12.101 | -50.083 | 1.00 | 15.24 | C |
| ATOM | 12399 | C   | PRO | H | 95 | 35.612 | 10.985 | -51.135 | 1.00 | 15.99 | C |
| ATOM | 12400 | O   | PRO | H | 95 | 36.631 | 10.277 | -51.197 | 1.00 | 16.05 | O |
| ATOM | 12401 | CB  | PRO | H | 95 | 36.671 | 13.149 | -50.368 | 1.00 | 15.66 | C |
| ATOM | 12402 | CG  | PRO | H | 95 | 37.200 | 13.527 | -48.999 | 1.00 | 15.46 | C |
| ATOM | 12403 | CD  | PRO | H | 95 | 37.218 | 12.226 | -48.285 | 1.00 | 14.81 | C |

| ATOM | 12404 | N | MET | H | 96 | 34.561 | 10.797 | -51.945 | 1.00 | 16.24 | N |
| ATOM | 12405 | CA | MET | H | 96 | 33.312 | 11.582 | -51.992 | 1.00 | 15.83 | C |
| ATOM | 12406 | C | MET | H | 96 | 32.110 | 10.755 | -51.531 | 1.00 | 17.14 | C |
| ATOM | 12407 | O | MET | H | 96 | 30.967 | 11.173 | -51.676 | 1.00 | 16.71 | O |
| ATOM | 12408 | CB | MET | H | 96 | 33.061 | 12.050 | -53.449 | 1.00 | 17.30 | C |
| ATOM | 12409 | CG | MET | H | 96 | 34.156 | 12.980 | -53.999 | 1.00 | 16.58 | C |
| ATOM | 12410 | SD | MET | H | 96 | 34.199 | 14.583 | -53.164 | 1.00 | 17.13 | S |
| ATOM | 12411 | CE | MET | H | 96 | 32.616 | 15.270 | -53.582 | 1.00 | 14.61 | C |
| ATOM | 12412 | N | ASP | H | 101 | 32.369 | 9.567 | -50.998 | 1.00 | 18.13 | N |
| ATOM | 12413 | CA | ASP | H | 101 | 31.338 | 8.536 | -50.936 | 1.00 | 19.64 | C |
| ATOM | 12414 | C | ASP | H | 101 | 30.644 | 8.304 | -49.593 | 1.00 | 19.54 | C |
| ATOM | 12415 | O | ASP | H | 101 | 29.486 | 7.908 | -49.598 | 1.00 | 19.95 | O |
| ATOM | 12416 | CB | ASP | H | 101 | 31.898 | 7.214 | -51.442 | 1.00 | 22.10 | C |
| ATOM | 12417 | CG | ASP | H | 101 | 32.323 | 7.284 | -52.900 | 1.00 | 24.98 | C |
| ATOM | 12418 | OD1 | ASP | H | 101 | 31.679 | 7.986 | -53.697 | 1.00 | 30.53 | O |
| ATOM | 12419 | OD2 | ASP | H | 101 | 33.315 | 6.644 | -53.255 | 1.00 | 32.29 | O |
| ATOM | 12420 | N | TYR | H | 102 | 31.328 | 8.522 | -48.466 | 1.00 | 18.47 | N |
| ATOM | 12421 | CA | TYR | H | 102 | 30.804 | 8.111 | -47.166 | 1.00 | 18.73 | C |
| ATOM | 12422 | C | TYR | H | 102 | 30.811 | 9.219 | -46.138 | 1.00 | 17.47 | C |
| ATOM | 12423 | O | TYR | H | 102 | 31.762 | 9.977 | -46.072 | 1.00 | 16.51 | O |
| ATOM | 12424 | CB | TYR | H | 102 | 31.610 | 6.967 | -46.584 | 1.00 | 20.97 | C |
| ATOM | 12425 | CG | TYR | H | 102 | 31.813 | 5.817 | -47.535 | 1.00 | 21.91 | C |
| ATOM | 12426 | CD1 | TYR | H | 102 | 30.769 | 4.951 | -47.830 | 1.00 | 24.34 | C |
| ATOM | 12427 | CD2 | TYR | H | 102 | 33.047 | 5.599 | -48.125 | 1.00 | 23.21 | C |
| ATOM | 12428 | CE1 | TYR | H | 102 | 30.951 | 3.906 | -48.704 | 1.00 | 23.39 | C |
| ATOM | 12429 | CE2 | TYR | H | 102 | 33.244 | 4.553 | -48.994 | 1.00 | 23.65 | C |
| ATOM | 12430 | CZ | TYR | H | 102 | 32.194 | 3.708 | -49.271 | 1.00 | 24.40 | C |
| ATOM | 12431 | OH | TYR | H | 102 | 32.376 | 2.660 | -50.138 | 1.00 | 24.90 | O |
| ATOM | 12432 | N | TRP | H | 103 | 29.733 | 9.271 | -45.354 | 1.00 | 17.69 | N |
| ATOM | 12433 | CA | TRP | H | 103 | 29.567 | 10.217 | -44.258 | 1.00 | 16.87 | C |
| ATOM | 12434 | C | TRP | H | 103 | 29.282 | 9.480 | -42.972 | 1.00 | 17.76 | C |
| ATOM | 12435 | O | TRP | H | 103 | 28.601 | 8.446 | -42.972 | 1.00 | 17.85 | O |
| ATOM | 12436 | CB | TRP | H | 103 | 28.372 | 11.130 | -44.503 | 1.00 | 16.38 | C |
| ATOM | 12437 | CG | TRP | H | 103 | 28.479 | 12.064 | -45.636 | 1.00 | 16.13 | C |
| ATOM | 12438 | CD1 | TRP | H | 103 | 28.268 | 11.779 | -46.950 | 1.00 | 15.19 | C |
| ATOM | 12439 | CD2 | TRP | H | 103 | 28.730 | 13.493 | -45.570 | 1.00 | 15.73 | C |
| ATOM | 12440 | NE1 | TRP | H | 103 | 28.393 | 12.912 | -47.705 | 1.00 | 16.33 | N |
| ATOM | 12441 | CE2 | TRP | H | 103 | 28.687 | 13.978 | -46.894 | 1.00 | 15.40 | C |
| ATOM | 12442 | CE3 | TRP | H | 103 | 29.012 | 14.391 | -44.530 | 1.00 | 15.88 | C |
| ATOM | 12443 | CZ2 | TRP | H | 103 | 28.894 | 15.321 | -47.207 | 1.00 | 15.93 | C |
| ATOM | 12444 | CZ3 | TRP | H | 103 | 29.234 | 15.726 | -44.839 | 1.00 | 16.06 | C |
| ATOM | 12445 | CH2 | TRP | H | 103 | 29.177 | 16.181 | -46.164 | 1.00 | 15.95 | C |
| ATOM | 12446 | N | GLY | H | 104 | 29.805 | 10.030 | -41.877 | 1.00 | 15.92 | N |
| ATOM | 12447 | CA | GLY | H | 104 | 29.498 | 9.590 | -40.539 | 1.00 | 15.00 | C |
| ATOM | 12448 | C | GLY | H | 104 | 28.154 | 10.038 | -40.032 | 1.00 | 15.53 | C |
| ATOM | 12449 | O | GLY | H | 104 | 27.304 | 10.510 | -40.787 | 1.00 | 17.56 | O |
| ATOM | 12450 | N | GLN | H | 105 | 27.945 | 9.845 | -38.739 | 1.00 | 16.03 | N |
| ATOM | 12451 | CA | GLN | H | 105 | 26.695 | 10.216 | -38.078 | 1.00 | 18.31 | C |
| ATOM | 12452 | C | GLN | H | 105 | 26.788 | 11.605 | -37.454 | 1.00 | 17.21 | C |
| ATOM | 12453 | O | GLN | H | 105 | 25.779 | 12.222 | -37.157 | 1.00 | 16.67 | O |
| ATOM | 12454 | CB | GLN | H | 105 | 26.321 | 9.163 | -37.023 | 1.00 | 21.58 | C |
| ATOM | 12455 | CG | GLN | H | 105 | 25.336 | 8.077 | -37.577 | 1.00 | 27.68 | C |
| ATOM | 12456 | CD | GLN | H | 105 | 25.689 | 7.537 | -38.964 | 1.00 | 32.49 | C |
| ATOM | 12457 | OE1 | GLN | H | 105 | 24.970 | 7.796 | -39.950 | 1.00 | 35.00 | O |
| ATOM | 12458 | NE2 | GLN | H | 105 | 26.771 | 6.741 | -39.041 | 1.00 | 36.14 | N |
| ATOM | 12459 | N | GLY | H | 106 | 28.008 | 12.090 | -37.308 | 1.00 | 16.41 | N |
| ATOM | 12460 | CA | GLY | H | 106 | 28.271 | 13.416 | -36.774 | 1.00 | 16.22 | C |
| ATOM | 12461 | C | GLY | H | 106 | 28.428 | 13.454 | -35.268 | 1.00 | 15.30 | C |
| ATOM | 12462 | O | GLY | H | 106 | 27.878 | 12.627 | -34.528 | 1.00 | 14.42 | O |
| ATOM | 12463 | N | THR | H | 107 | 29.236 | 14.393 | -34.797 | 1.00 | 13.80 | N |
| ATOM | 12464 | CA | THR | H | 107 | 29.342 | 14.629 | -33.348 | 1.00 | 13.50 | C |
| ATOM | 12465 | C | THR | H | 107 | 29.215 | 16.135 | -33.145 | 1.00 | 12.90 | C |
| ATOM | 12466 | O | THR | H | 107 | 29.785 | 16.916 | -33.915 | 1.00 | 12.72 | O |
| ATOM | 12467 | CB | THR | H | 107 | 30.636 | 14.055 | -32.735 | 1.00 | 13.05 | C |
| ATOM | 12468 | OG1 | THR | H | 107 | 30.572 | 14.154 | -31.306 | 1.00 | 13.24 | O |

EP 2 123 668 A1

```
ATOM  12469  CG2 THR H 107    31.903  14.765 -33.233  1.00 12.81         C
ATOM  12470  N   SER H 108    28.458  16.525 -32.136  1.00 11.28         N
ATOM  12471  CA  SER H 108    28.174  17.947 -31.920  1.00 11.67         C
ATOM  12472  C   SER H 108    29.227  18.617 -31.018  1.00 11.12         C
ATOM  12473  O   SER H 108    29.615  18.103 -29.959  1.00  9.64         O
ATOM  12474  CB  SER H 108    26.769  18.119 -31.337  1.00 12.66         C
ATOM  12475  OG  SER H 108    26.440  19.488 -31.183  1.00 14.49         O
ATOM  12476  N   VAL H 109    29.634  19.819 -31.432  1.00 10.42         N
ATOM  12477  CA  VAL H 109    30.588  20.633 -30.692  1.00 11.09         C
ATOM  12478  C   VAL H 109    29.914  21.969 -30.442  1.00 11.00         C
ATOM  12479  O   VAL H 109    29.435  22.601 -31.372  1.00 11.42         O
ATOM  12480  CB  VAL H 109    31.894  20.825 -31.484  1.00 12.07         C
ATOM  12481  CG1 VAL H 109    32.806  21.824 -30.758  1.00 12.83         C
ATOM  12482  CG2 VAL H 109    32.628  19.477 -31.604  1.00 14.13         C
ATOM  12483  N   THR H 110    29.803  22.341 -29.172  1.00 10.73         N
ATOM  12484  CA  THR H 110    29.256  23.615 -28.810  1.00  9.14         C
ATOM  12485  C   THR H 110    30.380  24.447 -28.173  1.00  8.59         C
ATOM  12486  O   THR H 110    31.018  24.020 -27.226  1.00  8.63         O
ATOM  12487  CB  THR H 110    28.059  23.461 -27.833  1.00 11.13         C
ATOM  12488  OG1 THR H 110    26.974  22.797 -28.496  1.00 13.39         O
ATOM  12489  CG2 THR H 110    27.563  24.810 -27.335  1.00 11.18         C
ATOM  12490  N   VAL H 111    30.574  25.655 -28.702  1.00  8.65         N
ATOM  12491  CA  VAL H 111    31.471  26.657 -28.119  1.00  8.35         C
ATOM  12492  C   VAL H 111    30.622  27.753 -27.505  1.00  9.95         C
ATOM  12493  O   VAL H 111    29.944  28.481 -28.211  1.00 11.52         O
ATOM  12494  CB  VAL H 111    32.447  27.259 -29.139  1.00  8.97         C
ATOM  12495  CG1 VAL H 111    33.517  28.063 -28.412  1.00 11.40         C
ATOM  12496  CG2 VAL H 111    33.093  26.169 -29.982  1.00 10.47         C
ATOM  12497  N   SER H 112    30.638  27.821 -26.185  1.00 10.28         N
ATOM  12498  CA  SER H 112    29.651  28.578 -25.420  1.00 11.72         C
ATOM  12499  C   SER H 112    30.199  28.947 -24.066  1.00 12.26         C
ATOM  12500  O   SER H 112    30.943  28.183 -23.435  1.00 11.12         O
ATOM  12501  CB  SER H 112    28.368  27.742 -25.245  1.00 13.25         C
ATOM  12502  OG  SER H 112    27.429  28.417 -24.436  1.00 14.33         O
ATOM  12503  N   SER H 113    29.760  30.090 -23.559  1.00 12.74         N
ATOM  12504  CA  SER H 113    30.039  30.455 -22.186  1.00 14.71         C
ATOM  12505  C   SER H 113    28.934  30.047 -21.199  1.00 16.80         C
ATOM  12506  O   SER H 113    29.094  30.234 -19.981  1.00 19.30         O
ATOM  12507  CB  SER H 113    30.334  31.974 -22.111  1.00 15.44         C
ATOM  12508  OG  SER H 113    29.175  32.734 -22.403  1.00 17.27         O
ATOM  12509  N   ALA H 114    27.842  29.432 -21.685  1.00 16.01         N
ATOM  12510  CA  ALA H 114    26.784  28.951 -20.799  1.00 15.87         C
ATOM  12511  C   ALA H 114    27.170  27.584 -20.208  1.00 16.68         C
ATOM  12512  O   ALA H 114    28.080  26.942 -20.697  1.00 17.56         O
ATOM  12513  CB  ALA H 114    25.444  28.880 -21.553  1.00 16.39         C
ATOM  12514  N   LYS H 115    26.523  27.182 -19.124  1.00 18.53         N
ATOM  12515  CA  LYS H 115    26.855  25.911 -18.480  1.00 19.98         C
ATOM  12516  C   LYS H 115    25.979  24.810 -19.055  1.00 16.99         C
ATOM  12517  O   LYS H 115    24.820  25.030 -19.415  1.00 19.27         O
ATOM  12518  CB  LYS H 115    26.725  26.000 -16.961  1.00 22.05         C
ATOM  12519  CG  LYS H 115    27.902  26.762 -16.337  1.00 26.04         C
ATOM  12520  CD  LYS H 115    27.753  26.974 -14.835  1.00 26.27         C
ATOM  12521  CE  LYS H 115    29.080  27.390 -14.212  1.00 28.18         C
ATOM  12522  NZ  LYS H 115    29.701  28.554 -14.921  1.00 31.02         N
ATOM  12523  N   THR H 116    26.543  23.610 -19.129  1.00 14.73         N
ATOM  12524  CA  THR H 116    25.775  22.415 -19.467  1.00 13.08         C
ATOM  12525  C   THR H 116    24.774  22.065 -18.336  1.00 11.35         C
ATOM  12526  O   THR H 116    25.124  22.075 -17.154  1.00  9.74         O
ATOM  12527  CB  THR H 116    26.742  21.244 -19.724  1.00 11.57         C
ATOM  12528  OG1 THR H 116    27.523  21.535 -20.880  1.00 12.97         O
ATOM  12529  CG2 THR H 116    26.007  19.940 -19.932  1.00 12.53         C
ATOM  12530  N   THR H 117    23.541  21.763 -18.709  1.00 11.48         N
ATOM  12531  CA  THR H 117    22.462  21.411 -17.780  1.00 12.83         C
ATOM  12532  C   THR H 117    21.841  20.150 -18.363  1.00 12.14         C
ATOM  12533  O   THR H 117    21.430  20.147 -19.507  1.00  9.89         O
```

451

```
ATOM   12534   CB   THR H 117     21.416  22.545 -17.708  1.00 15.07          C
ATOM   12535   OG1  THR H 117     22.089  23.759 -17.329  1.00 13.56          O
ATOM   12536   CG2  THR H 117     20.321  22.256 -16.696  1.00 15.69          C
ATOM   12537   N    PRO H 118     21.766  19.087 -17.575  1.00 13.19          N
ATOM   12538   CA   PRO H 118     21.113  17.900 -18.103  1.00 13.16          C
ATOM   12539   C    PRO H 118     19.582  18.024 -18.084  1.00 14.45          C
ATOM   12540   O    PRO H 118     19.027  18.789 -17.284  1.00 14.34          O
ATOM   12541   CB   PRO H 118     21.576  16.795 -17.145  1.00 13.18          C
ATOM   12542   CG   PRO H 118     21.735  17.486 -15.844  1.00 13.62          C
ATOM   12543   CD   PRO H 118     22.226  18.899 -16.188  1.00 13.46          C
ATOM   12544   N    PRO H 119     18.902  17.241 -18.935  1.00 15.50          N
ATOM   12545   CA   PRO H 119     17.448  17.258 -19.010  1.00 15.63          C
ATOM   12546   C    PRO H 119     16.764  16.543 -17.847  1.00 16.57          C
ATOM   12547   O    PRO H 119     17.335  15.614 -17.244  1.00 13.94          O
ATOM   12548   CB   PRO H 119     17.149  16.507 -20.315  1.00 16.16          C
ATOM   12549   CG   PRO H 119     18.304  15.723 -20.610  1.00 15.82          C
ATOM   12550   CD   PRO H 119     19.490  16.326 -19.926  1.00 14.85          C
ATOM   12551   N    SER H 120     15.547  17.002 -17.571  1.00 17.11          N
ATOM   12552   CA   SER H 120     14.577  16.280 -16.762  1.00 17.61          C
ATOM   12553   C    SER H 120     13.679  15.568 -17.734  1.00 16.96          C
ATOM   12554   O    SER H 120     13.276  16.133 -18.722  1.00 17.90          O
ATOM   12555   CB   SER H 120     13.791  17.257 -15.890  1.00 18.34          C
ATOM   12556   OG   SER H 120     14.705  17.834 -14.970  1.00 18.45          O
ATOM   12557   N    VAL H 121     13.404  14.298 -17.473  1.00 18.07          N
ATOM   12558   CA   VAL H 121     12.667  13.456 -18.411  1.00 18.87          C
ATOM   12559   C    VAL H 121     11.350  12.998 -17.774  1.00 20.29          C
ATOM   12560   O    VAL H 121     11.353  12.428 -16.682  1.00 19.91          O
ATOM   12561   CB   VAL H 121     13.499  12.242 -18.818  1.00 19.43          C
ATOM   12562   CG1  VAL H 121     12.783  11.403 -19.881  1.00 18.98          C
ATOM   12563   CG2  VAL H 121     14.864  12.682 -19.364  1.00 19.73          C
ATOM   12564   N    TYR H 122     10.238  13.275 -18.446  1.00 21.89          N
ATOM   12565   CA   TYR H 122      8.918  12.988 -17.898  1.00 24.11          C
ATOM   12566   C    TYR H 122      8.077  12.152 -18.823  1.00 25.23          C
ATOM   12567   O    TYR H 122      8.006  12.438 -20.008  1.00 22.53          O
ATOM   12568   CB   TYR H 122      8.193  14.277 -17.606  1.00 25.44          C
ATOM   12569   CG   TYR H 122      8.886  15.065 -16.537  1.00 25.47          C
ATOM   12570   CD1  TYR H 122      9.171  14.477 -15.303  1.00 27.59          C
ATOM   12571   CD2  TYR H 122      9.271  16.372 -16.753  1.00 25.76          C
ATOM   12572   CE1  TYR H 122      9.815  15.189 -14.303  1.00 27.99          C
ATOM   12573   CE2  TYR H 122      9.931  17.097 -15.751  1.00 26.70          C
ATOM   12574   CZ   TYR H 122     10.190  16.500 -14.538  1.00 26.45          C
ATOM   12575   OH   TYR H 122     10.824  17.180 -13.511  1.00 28.33          O
ATOM   12576   N    PRO H 123      7.370  11.152 -18.263  1.00 28.11          N
ATOM   12577   CA   PRO H 123      6.524  10.289 -19.097  1.00 28.77          C
ATOM   12578   C    PRO H 123      5.254  11.015 -19.485  1.00 29.14          C
ATOM   12579   O    PRO H 123      4.752  11.860 -18.725  1.00 30.13          O
ATOM   12580   CB   PRO H 123      6.179   9.130 -18.168  1.00 30.15          C
ATOM   12581   CG   PRO H 123      6.109   9.752 -16.822  1.00 29.64          C
ATOM   12582   CD   PRO H 123      7.221  10.832 -16.830  1.00 29.11          C
ATOM   12583   N    LEU H 124      4.748  10.702 -20.669  1.00 28.48          N
ATOM   12584   CA   LEU H 124      3.455  11.184 -21.097  1.00 28.27          C
ATOM   12585   C    LEU H 124      2.599   9.945 -21.384  1.00 28.62          C
ATOM   12586   O    LEU H 124      2.711   9.317 -22.440  1.00 25.61          O
ATOM   12587   CB   LEU H 124      3.584  12.078 -22.317  1.00 28.56          C
ATOM   12588   CG   LEU H 124      4.561  13.270 -22.208  1.00 28.48          C
ATOM   12589   CD1  LEU H 124      4.692  13.980 -23.544  1.00 27.92          C
ATOM   12590   CD2  LEU H 124      4.156  14.250 -21.125  1.00 31.01          C
ATOM   12591   N    ALA H 125      1.811   9.561 -20.385  1.00 30.60          N
ATOM   12592   CA   ALA H 125      0.848   8.468 -20.521  1.00 31.95          C
ATOM   12593   C    ALA H 125     -0.528   9.107 -20.649  1.00 33.27          C
ATOM   12594   O    ALA H 125     -0.767  10.194 -20.112  1.00 32.67          O
ATOM   12595   CB   ALA H 125      0.907   7.541 -19.337  1.00 31.46          C
ATOM   12596   N    PRO H 126     -1.439   8.457 -21.388  1.00 35.66          N
ATOM   12597   CA   PRO H 126     -2.778   9.029 -21.530  1.00 37.62          C
ATOM   12598   C    PRO H 126     -3.498   9.121 -20.186  1.00 39.19          C
```

```
ATOM   12599  O   PRO H 126    -3.227    8.320 -19.280  1.00 39.28           O
ATOM   12600  CB  PRO H 126    -3.501    8.038 -22.448  1.00 37.16           C
ATOM   12601  CG  PRO H 126    -2.695    6.793 -22.408  1.00 37.14           C
ATOM   12602  CD  PRO H 126    -1.291    7.187 -22.114  1.00 36.60           C
ATOM   12603  N   GLY H 127    -4.382   10.107 -20.067  1.00 41.07           N
ATOM   12604  CA  GLY H 127    -5.235   10.255 -18.887  1.00 42.10           C
ATOM   12605  C   GLY H 127    -6.178    9.073 -18.731  1.00 42.69           C
ATOM   12606  O   GLY H 127    -6.487    8.373 -19.708  1.00 43.57           O
ATOM   12607  N   SER H 136    -7.979    1.571 -31.575  1.00 35.50           N
ATOM   12608  CA  SER H 136    -7.049    0.614 -32.169  1.00 35.09           C
ATOM   12609  C   SER H 136    -5.582    0.988 -31.889  1.00 33.51           C
ATOM   12610  O   SER H 136    -4.726    0.109 -31.729  1.00 32.61           O
ATOM   12611  CB  SER H 136    -7.280    0.561 -33.672  1.00 35.83           C
ATOM   12612  OG  SER H 136    -6.256   -0.171 -34.326  1.00 40.25           O
ATOM   12613  N   MET H 137    -5.297    2.289 -31.857  1.00 31.60           N
ATOM   12614  CA  MET H 137    -3.932    2.770 -31.603  1.00 29.83           C
ATOM   12615  C   MET H 137    -3.913    3.578 -30.328  1.00 27.68           C
ATOM   12616  O   MET H 137    -4.935    4.085 -29.913  1.00 27.05           O
ATOM   12617  CB  MET H 137    -3.450    3.661 -32.744  1.00 30.14           C
ATOM   12618  CG  MET H 137    -3.425    3.023 -34.088  1.00 32.39           C
ATOM   12619  SD  MET H 137    -2.361    1.586 -34.144  1.00 33.95           S
ATOM   12620  CE  MET H 137    -0.722    2.312 -34.210  1.00 35.16           C
ATOM   12621  N   VAL H 138    -2.736    3.702 -29.723  1.00 25.42           N
ATOM   12622  CA  VAL H 138    -2.545    4.546 -28.558  1.00 24.18           C
ATOM   12623  C   VAL H 138    -1.282    5.376 -28.773  1.00 23.37           C
ATOM   12624  O   VAL H 138    -0.304    4.897 -29.334  1.00 21.63           O
ATOM   12625  CB  VAL H 138    -2.480    3.732 -27.252  1.00 24.84           C
ATOM   12626  CG1 VAL H 138    -1.398    2.661 -27.295  1.00 24.16           C
ATOM   12627  CG2 VAL H 138    -2.274    4.607 -26.081  1.00 25.65           C
ATOM   12628  N   THR H 139    -1.333    6.633 -28.347  1.00 23.88           N
ATOM   12629  CA  THR H 139    -0.180    7.521 -28.444  1.00 22.45           C
ATOM   12630  C   THR H 139     0.398    7.851 -27.053  1.00 21.81           C
ATOM   12631  O   THR H 139    -0.324    8.250 -26.134  1.00 21.55           O
ATOM   12632  CB  THR H 139    -0.566    8.794 -29.213  1.00 23.12           C
ATOM   12633  OG1 THR H 139    -0.881    8.436 -30.574  1.00 23.65           O
ATOM   12634  CG2 THR H 139     0.566    9.771 -29.209  1.00 23.13           C
ATOM   12635  N   LEU H 140     1.700    7.616 -26.910  1.00 20.92           N
ATOM   12636  CA  LEU H 140     2.460    7.887 -25.692  1.00 20.93           C
ATOM   12637  C   LEU H 140     3.540    8.932 -25.995  1.00 19.46           C
ATOM   12638  O   LEU H 140     3.736    9.300 -27.140  1.00 18.76           O
ATOM   12639  CB  LEU H 140     3.152    6.627 -25.231  1.00 20.60           C
ATOM   12640  CG  LEU H 140     2.265    5.380 -25.104  1.00 23.06           C
ATOM   12641  CD1 LEU H 140     3.147    4.245 -24.668  1.00 24.46           C
ATOM   12642  CD2 LEU H 140     1.140    5.627 -24.121  1.00 22.01           C
ATOM   12643  N   GLY H 141     4.245    9.389 -24.969  1.00 20.09           N
ATOM   12644  CA  GLY H 141     5.207   10.474 -25.179  1.00 19.88           C
ATOM   12645  C   GLY H 141     6.231   10.594 -24.076  1.00 19.98           C
ATOM   12646  O   GLY H 141     6.108    9.958 -23.019  1.00 17.89           O
ATOM   12647  N   CYS H 142     7.244   11.431 -24.322  1.00 20.13           N
ATOM   12648  CA  CYS H 142     8.237   11.774 -23.309  1.00 22.33           C
ATOM   12649  C   CYS H 142     8.489   13.264 -23.438  1.00 20.65           C
ATOM   12650  O   CYS H 142     8.537   13.765 -24.537  1.00 17.31           O
ATOM   12651  CB  CYS H 142     9.532   10.978 -23.505  1.00 26.41           C
ATOM   12652  SG  CYS H 142     9.442    9.458 -22.534  1.00 37.07           S
ATOM   12653  N   LEU H 143     8.586   13.953 -22.314  1.00 19.59           N
ATOM   12654  CA  LEU H 143     8.903   15.378 -22.301  1.00 18.81           C
ATOM   12655  C   LEU H 143    10.326   15.487 -21.761  1.00 17.44           C
ATOM   12656  O   LEU H 143    10.674   14.871 -20.742  1.00 17.07           O
ATOM   12657  CB  LEU H 143     7.915   16.132 -21.419  1.00 19.82           C
ATOM   12658  CG  LEU H 143     8.125   17.639 -21.238  1.00 19.51           C
ATOM   12659  CD1 LEU H 143     7.957   18.333 -22.535  1.00 19.62           C
ATOM   12660  CD2 LEU H 143     7.146   18.166 -20.191  1.00 20.86           C
ATOM   12661  N   VAL H 144    11.166   16.225 -22.483  1.00 16.61           N
ATOM   12662  CA  VAL H 144    12.586   16.273 -22.177  1.00 16.18           C
ATOM   12663  C   VAL H 144    12.837   17.756 -21.937  1.00 15.53           C
```

| ATOM | 12664 | O | VAL H 144 | 12.832 | 18.540 | -22.877 | 1.00 | 15.87 | O |
|------|-------|------|-----------|--------|--------|---------|------|-------|---|
| ATOM | 12665 | CB | VAL H 144 | 13.464 | 15.758 | -23.324 | 1.00 | 16.48 | C |
| ATOM | 12666 | CG1 | VAL H 144 | 14.905 | 15.840 | -22.935 | 1.00 | 16.35 | C |
| ATOM | 12667 | CG2 | VAL H 144 | 13.121 | 14.344 | -23.667 | 1.00 | 16.89 | C |
| ATOM | 12668 | N | LYS H 145 | 13.003 | 18.130 | -20.683 | 1.00 | 16.36 | N |
| ATOM | 12669 | CA | LYS H 145 | 12.851 | 19.541 | -20.313 | 1.00 | 18.20 | C |
| ATOM | 12670 | C | LYS H 145 | 14.091 | 20.126 | -19.649 | 1.00 | 16.09 | C |
| ATOM | 12671 | O | LYS H 145 | 14.682 | 19.507 | -18.755 | 1.00 | 15.53 | O |
| ATOM | 12672 | CB | LYS H 145 | 11.643 | 19.672 | -19.372 | 1.00 | 20.27 | C |
| ATOM | 12673 | CG | LYS H 145 | 11.323 | 21.090 | -18.950 | 1.00 | 22.98 | C |
| ATOM | 12674 | CD | LYS H 145 | 10.109 | 21.158 | -18.026 | 1.00 | 23.36 | C |
| ATOM | 12675 | CE | LYS H 145 | 9.840 | 22.629 | -17.630 | 1.00 | 26.09 | C |
| ATOM | 12676 | NZ | LYS H 145 | 9.643 | 23.467 | -18.847 | 1.00 | 27.21 | N |
| ATOM | 12677 | N | GLY H 146 | 14.427 | 21.367 | -20.026 | 1.00 | 14.92 | N |
| ATOM | 12678 | CA | GLY H 146 | 15.396 | 22.146 | -19.284 | 1.00 | 14.67 | C |
| ATOM | 12679 | C | GLY H 146 | 16.868 | 21.836 | -19.486 | 1.00 | 14.30 | C |
| ATOM | 12680 | O | GLY H 146 | 17.623 | 21.882 | -18.536 | 1.00 | 15.14 | O |
| ATOM | 12681 | N | TYR H 147 | 17.295 | 21.539 | -20.712 | 1.00 | 13.52 | N |
| ATOM | 12682 | CA | TYR H 147 | 18.678 | 21.121 | -20.926 | 1.00 | 11.76 | C |
| ATOM | 12683 | C | TYR H 147 | 19.428 | 22.128 | -21.742 | 1.00 | 10.87 | C |
| ATOM | 12684 | O | TYR H 147 | 18.833 | 22.933 | -22.457 | 1.00 | 10.74 | O |
| ATOM | 12685 | CB | TYR H 147 | 18.784 | 19.737 | -21.619 | 1.00 | 11.70 | C |
| ATOM | 12686 | CG | TYR H 147 | 18.178 | 19.652 | -23.003 | 1.00 | 10.64 | C |
| ATOM | 12687 | CD1 | TYR H 147 | 16.811 | 19.384 | -23.184 | 1.00 | 11.80 | C |
| ATOM | 12688 | CD2 | TYR H 147 | 18.965 | 19.823 | -24.144 | 1.00 | 10.84 | C |
| ATOM | 12689 | CE1 | TYR H 147 | 16.267 | 19.276 | -24.450 | 1.00 | 12.07 | C |
| ATOM | 12690 | CE2 | TYR H 147 | 18.422 | 19.742 | -25.416 | 1.00 | 11.03 | C |
| ATOM | 12691 | CZ | TYR H 147 | 17.060 | 19.499 | -25.562 | 1.00 | 11.57 | C |
| ATOM | 12692 | OH | TYR H 147 | 16.513 | 19.378 | -26.824 | 1.00 | 13.24 | O |
| ATOM | 12693 | N | PHE H 148 | 20.746 | 22.045 | -21.649 | 1.00 | 10.77 | N |
| ATOM | 12694 | CA | PHE H 148 | 21.623 | 22.838 | -22.501 | 1.00 | 11.83 | C |
| ATOM | 12695 | C | PHE H 148 | 22.989 | 22.140 | -22.603 | 1.00 | 10.76 | C |
| ATOM | 12696 | O | PHE H 148 | 23.462 | 21.562 | -21.604 | 1.00 | 11.44 | O |
| ATOM | 12697 | CB | PHE H 148 | 21.811 | 24.267 | -21.941 | 1.00 | 12.31 | C |
| ATOM | 12698 | CG | PHE H 148 | 22.614 | 25.125 | -22.850 | 1.00 | 12.36 | C |
| ATOM | 12699 | CD1 | PHE H 148 | 21.999 | 25.787 | -23.892 | 1.00 | 15.30 | C |
| ATOM | 12700 | CD2 | PHE H 148 | 23.995 | 25.204 | -22.729 | 1.00 | 14.69 | C |
| ATOM | 12701 | CE1 | PHE H 148 | 22.739 | 26.511 | -24.787 | 1.00 | 13.87 | C |
| ATOM | 12702 | CE2 | PHE H 148 | 24.731 | 25.957 | -23.614 | 1.00 | 12.09 | C |
| ATOM | 12703 | CZ | PHE H 148 | 24.107 | 26.600 | -24.634 | 1.00 | 14.57 | C |
| ATOM | 12704 | N | PRO H 149 | 23.627 | 22.147 | -23.799 | 1.00 | 10.37 | N |
| ATOM | 12705 | CA | PRO H 149 | 23.199 | 22.585 | -25.112 | 1.00 | 10.80 | C |
| ATOM | 12706 | C | PRO H 149 | 22.439 | 21.489 | -25.836 | 1.00 | 13.20 | C |
| ATOM | 12707 | O | PRO H 149 | 22.332 | 20.375 | -25.346 | 1.00 | 12.89 | O |
| ATOM | 12708 | CB | PRO H 149 | 24.519 | 22.846 | -25.819 | 1.00 | 11.23 | C |
| ATOM | 12709 | CG | PRO H 149 | 25.428 | 21.855 | -25.258 | 1.00 | 10.01 | C |
| ATOM | 12710 | CD | PRO H 149 | 25.020 | 21.689 | -23.820 | 1.00 | 10.78 | C |
| ATOM | 12711 | N | GLU H 150 | 21.937 | 21.807 | -27.015 | 1.00 | 14.12 | N |
| ATOM | 12712 | CA | GLU H 150 | 21.520 | 20.776 | -27.941 | 1.00 | 15.33 | C |
| ATOM | 12713 | C | GLU H 150 | 22.757 | 20.022 | -28.366 | 1.00 | 14.43 | C |
| ATOM | 12714 | O | GLU H 150 | 23.862 | 20.573 | -28.305 | 1.00 | 13.23 | O |
| ATOM | 12715 | CB | GLU H 150 | 20.875 | 21.411 | -29.162 | 1.00 | 17.32 | C |
| ATOM | 12716 | CG | GLU H 150 | 19.500 | 21.912 | -28.872 | 1.00 | 18.58 | C |
| ATOM | 12717 | CD | GLU H 150 | 18.441 | 20.975 | -29.408 | 1.00 | 24.03 | C |
| ATOM | 12718 | OE1 | GLU H 150 | 17.861 | 21.417 | -30.444 | 1.00 | 21.28 | O |
| ATOM | 12719 | OE2 | GLU H 150 | 18.188 | 19.832 | -28.825 | 1.00 | 19.51 | O |
| ATOM | 12720 | N | PRO H 151 | 22.592 | 18.776 | -28.847 | 1.00 | 14.79 | N |
| ATOM | 12721 | CA | PRO H 151 | 21.412 | 17.978 | -29.045 | 1.00 | 14.38 | C |
| ATOM | 12722 | C | PRO H 151 | 21.068 | 17.007 | -27.938 | 1.00 | 13.62 | C |
| ATOM | 12723 | O | PRO H 151 | 21.871 | 16.759 | -27.058 | 1.00 | 13.46 | O |
| ATOM | 12724 | CB | PRO H 151 | 21.790 | 17.171 | -30.292 | 1.00 | 14.68 | C |
| ATOM | 12725 | CG | PRO H 151 | 23.196 | 16.833 | -30.075 | 1.00 | 15.60 | C |
| ATOM | 12726 | CD | PRO H 151 | 23.778 | 18.094 | -29.389 | 1.00 | 15.21 | C |
| ATOM | 12727 | N | VAL H 152 | 19.848 | 16.475 | -27.986 | 1.00 | 15.43 | N |
| ATOM | 12728 | CA | VAL H 152 | 19.528 | 15.230 | -27.326 | 1.00 | 15.13 | C |

```
ATOM  12729  C    VAL H 152    19.126  14.252 -28.403  1.00 18.28       C
ATOM  12730  O    VAL H 152    18.685  14.664 -29.495  1.00 18.72       O
ATOM  12731  CB   VAL H 152    18.383  15.314 -26.300  1.00 15.90       C
ATOM  12732  CG1  VAL H 152    18.818  16.089 -25.098  1.00 15.02       C
ATOM  12733  CG2  VAL H 152    17.103  15.901 -26.918  1.00 15.91       C
ATOM  12734  N    THR H 153    19.281  12.969 -28.072  1.00 19.01       N
ATOM  12735  CA   THR H 153    18.799  11.855 -28.902  1.00 20.71       C
ATOM  12736  C    THR H 153    17.741  11.079 -28.133  1.00 19.57       C
ATOM  12737  O    THR H 153    18.002  10.552 -27.045  1.00 19.51       O
ATOM  12738  CB   THR H 153    19.947  10.919 -29.261  1.00 21.86       C
ATOM  12739  OG1  THR H 153    20.845  11.616 -30.132  1.00 25.91       O
ATOM  12740  CG2  THR H 153    19.441   9.667 -29.992  1.00 24.56       C
ATOM  12741  N    VAL H 154    16.530  11.032 -28.678  1.00 19.56       N
ATOM  12742  CA   VAL H 154    15.467  10.284 -28.039  1.00 18.41       C
ATOM  12743  C    VAL H 154    15.179   9.032 -28.884  1.00 19.60       C
ATOM  12744  O    VAL H 154    15.033   9.120 -30.103  1.00 19.24       O
ATOM  12745  CB   VAL H 154    14.179  11.082 -27.924  1.00 19.90       C
ATOM  12746  CG1  VAL H 154    13.118  10.234 -27.230  1.00 18.58       C
ATOM  12747  CG2  VAL H 154    14.439  12.393 -27.181  1.00 20.22       C
ATOM  12748  N    THR H 155    15.102   7.895 -28.221  1.00 19.33       N
ATOM  12749  CA   THR H 155    14.749   6.644 -28.909  1.00 21.28       C
ATOM  12750  C    THR H 155    13.707   5.977 -28.061  1.00 20.83       C
ATOM  12751  O    THR H 155    13.459   6.390 -26.932  1.00 20.61       O
ATOM  12752  CB   THR H 155    15.950   5.704 -29.083  1.00 20.74       C
ATOM  12753  OG1  THR H 155    16.520   5.425 -27.813  1.00 20.22       O
ATOM  12754  CG2  THR H 155    17.018   6.302 -30.010  1.00 21.34       C
ATOM  12755  N    TRP H 156    13.072   4.942 -28.608  1.00 22.10       N
ATOM  12756  CA   TRP H 156    12.001   4.262 -27.898  1.00 21.94       C
ATOM  12757  C    TRP H 156    12.378   2.782 -27.932  1.00 23.13       C
ATOM  12758  O    TRP H 156    12.789   2.314 -28.972  1.00 21.82       O
ATOM  12759  CB   TRP H 156    10.671   4.501 -28.601  1.00 21.97       C
ATOM  12760  CG   TRP H 156    10.126   5.895 -28.412  1.00 21.48       C
ATOM  12761  CD1  TRP H 156    10.276   6.958 -29.252  1.00 21.48       C
ATOM  12762  CD2  TRP H 156     9.343   6.361 -27.310  1.00 20.45       C
ATOM  12763  NE1  TRP H 156     9.654   8.063 -28.735  1.00 21.75       N
ATOM  12764  CE2  TRP H 156     9.071   7.722 -27.539  1.00 20.81       C
ATOM  12765  CE3  TRP H 156     8.864   5.765 -26.140  1.00 21.12       C
ATOM  12766  CZ2  TRP H 156     8.304   8.481 -26.668  1.00 19.89       C
ATOM  12767  CZ3  TRP H 156     8.096   6.523 -25.266  1.00 21.33       C
ATOM  12768  CH2  TRP H 156     7.838   7.876 -25.533  1.00 21.99       C
ATOM  12769  N    ASN H 157    12.288   2.104 -26.789  1.00 24.89       N
ATOM  12770  CA   ASN H 157    12.762   0.719 -26.626  1.00 26.60       C
ATOM  12771  C    ASN H 157    14.134   0.474 -27.219  1.00 28.20       C
ATOM  12772  O    ASN H 157    14.358  -0.492 -27.971  1.00 28.18       O
ATOM  12773  CB   ASN H 157    11.742  -0.260 -27.211  1.00 26.84       C
ATOM  12774  CG   ASN H 157    10.452  -0.253 -26.435  1.00 25.71       C
ATOM  12775  OD1  ASN H 157    10.378   0.341 -25.359  1.00 27.27       O
ATOM  12776  ND2  ASN H 157     9.431  -0.924 -26.958  1.00 28.07       N
ATOM  12777  N    SER H 158    15.055   1.363 -26.849  1.00 29.25       N
ATOM  12778  CA   SER H 158    16.430   1.326 -27.303  1.00 30.06       C
ATOM  12779  C    SER H 158    16.577   1.367 -28.822  1.00 30.79       C
ATOM  12780  O    SER H 158    17.593   0.917 -29.366  1.00 31.74       O
ATOM  12781  CB   SER H 158    17.137   0.111 -26.696  1.00 29.97       C
ATOM  12782  OG   SER H 158    16.898   0.087 -25.299  1.00 31.67       O
ATOM  12783  N    GLY H 159    15.593   1.955 -29.502  1.00 31.20       N
ATOM  12784  CA   GLY H 159    15.615   2.077 -30.951  1.00 32.56       C
ATOM  12785  C    GLY H 159    14.838   0.989 -31.681  1.00 33.65       C
ATOM  12786  O    GLY H 159    14.585   1.107 -32.885  1.00 33.55       O
ATOM  12787  N    SER H 160    14.468  -0.065 -30.954  1.00 34.42       N
ATOM  12788  CA   SER H 160    13.634  -1.148 -31.494  1.00 35.54       C
ATOM  12789  C    SER H 160    12.293  -0.650 -31.967  1.00 35.17       C
ATOM  12790  O    SER H 160    11.754  -1.141 -32.955  1.00 34.61       O
ATOM  12791  CB   SER H 160    13.376  -2.192 -30.417  1.00 35.78       C
ATOM  12792  OG   SER H 160    14.598  -2.704 -29.943  1.00 38.61       O
ATOM  12793  N    LEU H 161    11.746   0.311 -31.228  1.00 34.94       N
```

```
ATOM  12794  CA  LEU H 161     10.472   0.915 -31.571  1.00 35.14           C
ATOM  12795  C   LEU H 161     10.791   2.176 -32.359  1.00 35.23           C
ATOM  12796  O   LEU H 161     11.128   3.214 -31.767  1.00 35.41           O
ATOM  12797  CB  LEU H 161      9.689   1.253 -30.295  1.00 35.04           C
ATOM  12798  CG  LEU H 161      8.180   1.050 -30.268  1.00 35.52           C
ATOM  12799  CD1 LEU H 161      7.574   1.889 -29.152  1.00 35.26           C
ATOM  12800  CD2 LEU H 161      7.536   1.357 -31.611  1.00 36.13           C
ATOM  12801  N   SER H 162     10.714   2.095 -33.685  1.00 34.97           N
ATOM  12802  CA  SER H 162     11.069   3.236 -34.525  1.00 34.93           C
ATOM  12803  C   SER H 162      9.968   3.729 -35.466  1.00 34.56           C
ATOM  12804  O   SER H 162     10.015   4.871 -35.903  1.00 34.51           O
ATOM  12805  CB  SER H 162     12.360   2.955 -35.308  1.00 36.03           C
ATOM  12806  OG  SER H 162     12.246   1.830 -36.162  1.00 36.94           O
ATOM  12807  N   SER H 163      8.990   2.887 -35.784  1.00 33.57           N
ATOM  12808  CA  SER H 163      7.841   3.337 -36.572  1.00 32.73           C
ATOM  12809  C   SER H 163      6.826   4.038 -35.684  1.00 30.47           C
ATOM  12810  O   SER H 163      6.633   3.665 -34.535  1.00 29.99           O
ATOM  12811  CB  SER H 163      7.180   2.159 -37.301  1.00 33.50           C
ATOM  12812  OG  SER H 163      7.952   1.802 -38.438  1.00 35.78           O
ATOM  12813  N   GLY H 164      6.171   5.052 -36.231  1.00 28.68           N
ATOM  12814  CA  GLY H 164      5.166   5.787 -35.487  1.00 27.48           C
ATOM  12815  C   GLY H 164      5.761   6.758 -34.483  1.00 26.38           C
ATOM  12816  O   GLY H 164      5.079   7.181 -33.559  1.00 25.39           O
ATOM  12817  N   VAL H 165      7.026   7.113 -34.674  1.00 26.09           N
ATOM  12818  CA  VAL H 165      7.711   8.067 -33.789  1.00 25.95           C
ATOM  12819  C   VAL H 165      7.810   9.469 -34.441  1.00 26.69           C
ATOM  12820  O   VAL H 165      8.151   9.582 -35.632  1.00 27.26           O
ATOM  12821  CB  VAL H 165      9.119   7.553 -33.428  1.00 26.89           C
ATOM  12822  CG1 VAL H 165      9.887   8.609 -32.614  1.00 27.54           C
ATOM  12823  CG2 VAL H 165      9.045   6.209 -32.663  1.00 26.94           C
ATOM  12824  N   HIS H 166      7.485  10.510 -33.670  1.00 25.84           N
ATOM  12825  CA  HIS H 166      7.759  11.923 -34.027  1.00 25.73           C
ATOM  12826  C   HIS H 166      8.536  12.524 -32.864  1.00 23.11           C
ATOM  12827  O   HIS H 166      8.047  12.515 -31.738  1.00 23.13           O
ATOM  12828  CB  HIS H 166      6.485  12.773 -34.166  1.00 26.40           C
ATOM  12829  CG  HIS H 166      5.510  12.276 -35.182  1.00 29.49           C
ATOM  12830  ND1 HIS H 166      5.795  12.225 -36.530  1.00 31.86           N
ATOM  12831  CD2 HIS H 166      4.233  11.856 -35.053  1.00 30.74           C
ATOM  12832  CE1 HIS H 166      4.746  11.759 -37.186  1.00 32.29           C
ATOM  12833  NE2 HIS H 166      3.781  11.531 -36.312  1.00 32.80           N
ATOM  12834  N   THR H 167      9.733  13.039 -33.118  1.00 22.84           N
ATOM  12835  CA  THR H 167     10.423  13.841 -32.117  1.00 20.22           C
ATOM  12836  C   THR H 167     10.414  15.283 -32.615  1.00 19.29           C
ATOM  12837  O   THR H 167     10.876  15.582 -33.715  1.00 20.15           O
ATOM  12838  CB  THR H 167     11.821  13.285 -31.805  1.00 21.37           C
ATOM  12839  OG1 THR H 167     11.692  11.959 -31.264  1.00 21.77           O
ATOM  12840  CG2 THR H 167     12.521  14.136 -30.780  1.00 20.33           C
ATOM  12841  N   PHE H 168      9.864  16.176 -31.807  1.00 17.03           N
ATOM  12842  CA  PHE H 168      9.662  17.547 -32.221  1.00 16.57           C
ATOM  12843  C   PHE H 168     10.941  18.332 -31.996  1.00 18.34           C
ATOM  12844  O   PHE H 168     11.731  18.001 -31.099  1.00 18.25           O
ATOM  12845  CB  PHE H 168      8.484  18.152 -31.460  1.00 16.58           C
ATOM  12846  CG  PHE H 168      7.190  17.433 -31.724  1.00 18.67           C
ATOM  12847  CD1 PHE H 168      6.815  16.334 -30.946  1.00 17.45           C
ATOM  12848  CD2 PHE H 168      6.392  17.816 -32.786  1.00 17.09           C
ATOM  12849  CE1 PHE H 168      5.635  15.646 -31.221  1.00 18.85           C
ATOM  12850  CE2 PHE H 168      5.218  17.143 -33.072  1.00 19.83           C
ATOM  12851  CZ  PHE H 168      4.844  16.047 -32.291  1.00 17.57           C
ATOM  12852  N   PRO H 169     11.177  19.359 -32.825  1.00 18.51           N
ATOM  12853  CA  PRO H 169     12.308  20.233 -32.575  1.00 19.00           C
ATOM  12854  C   PRO H 169     12.242  20.927 -31.223  1.00 18.48           C
ATOM  12855  O   PRO H 169     11.158  21.258 -30.736  1.00 19.72           O
ATOM  12856  CB  PRO H 169     12.195  21.280 -33.686  1.00 18.94           C
ATOM  12857  CG  PRO H 169     11.462  20.591 -34.773  1.00 20.23           C
ATOM  12858  CD  PRO H 169     10.461  19.731 -34.059  1.00 18.19           C
```

```
ATOM  12859  N    ALA H 170    13.410  21.180 -30.637  1.00 16.73        N
ATOM  12860  CA   ALA H 170    13.476  21.779 -29.320  1.00 15.78        C
ATOM  12861  C    ALA H 170    13.159  23.257 -29.422  1.00 16.11        C
ATOM  12862  O    ALA H 170    13.388  23.862 -30.472  1.00 16.89        O
ATOM  12863  CB   ALA H 170    14.853  21.599 -28.735  1.00 15.62        C
ATOM  12864  N    VAL H 171    12.606  23.795 -28.356  1.00 17.30        N
ATOM  12865  CA   VAL H 171    12.333  25.218 -28.247  1.00 20.93        C
ATOM  12866  C    VAL H 171    13.201  25.729 -27.111  1.00 22.16        C
ATOM  12867  O    VAL H 171    13.358  25.056 -26.108  1.00 20.50        O
ATOM  12868  CB   VAL H 171    10.844  25.462 -27.950  1.00 21.20        C
ATOM  12869  CG1  VAL H 171    10.544  26.936 -27.700  1.00 24.94        C
ATOM  12870  CG2  VAL H 171    10.002  24.966 -29.123  1.00 24.14        C
ATOM  12871  N    LEU H 172    13.755  26.925 -27.275  1.00 24.82        N
ATOM  12872  CA   LEU H 172    14.588  27.543 -26.266  1.00 26.40        C
ATOM  12873  C    LEU H 172    13.717  28.491 -25.480  1.00 29.01        C
ATOM  12874  O    LEU H 172    13.026  29.316 -26.075  1.00 30.97        O
ATOM  12875  CB   LEU H 172    15.722  28.304 -26.958  1.00 26.80        C
ATOM  12876  CG   LEU H 172    16.848  28.914 -26.131  1.00 26.07        C
ATOM  12877  CD1  LEU H 172    17.482  27.896 -25.212  1.00 25.72        C
ATOM  12878  CD2  LEU H 172    17.891  29.462 -27.062  1.00 26.74        C
ATOM  12879  N    GLN H 173    13.692  28.347 -24.158  1.00 30.47        N
ATOM  12880  CA   GLN H 173    13.035  29.326 -23.289  1.00 32.49        C
ATOM  12881  C    GLN H 173    13.858  29.516 -22.036  1.00 30.99        C
ATOM  12882  O    GLN H 173    14.247  28.541 -21.390  1.00 31.33        O
ATOM  12883  CB   GLN H 173    11.635  28.864 -22.898  1.00 32.82        C
ATOM  12884  CG   GLN H 173    10.694  30.000 -22.440  1.00 35.49        C
ATOM  12885  CD   GLN H 173     9.551  29.507 -21.553  1.00 36.40        C
ATOM  12886  OE1  GLN H 173     8.971  28.435 -21.788  1.00 38.73        O
ATOM  12887  NE2  GLN H 173     9.235  30.283 -20.506  1.00 39.67        N
ATOM  12888  N    SER H 174    14.117  30.773 -21.691  1.00 30.58        N
ATOM  12889  CA   SER H 174    14.913  31.111 -20.511  1.00 29.23        C
ATOM  12890  C    SER H 174    16.238  30.389 -20.547  1.00 26.03        C
ATOM  12891  O    SER H 174    16.678  29.832 -19.548  1.00 26.14        O
ATOM  12892  CB   SER H 174    14.155  30.779 -19.230  1.00 30.10        C
ATOM  12893  OG   SER H 174    12.816  31.255 -19.298  1.00 32.24        O
ATOM  12894  N    ASP H 175    16.850  30.393 -21.729  1.00 25.09        N
ATOM  12895  CA   ASP H 175    18.195  29.865 -21.961  1.00 23.37        C
ATOM  12896  C    ASP H 175    18.279  28.327 -21.906  1.00 21.59        C
ATOM  12897  O    ASP H 175    19.365  27.786 -21.959  1.00 20.91        O
ATOM  12898  CB   ASP H 175    19.211  30.481 -20.986  1.00 26.24        C
ATOM  12899  CG   ASP H 175    19.251  32.020 -21.051  1.00 30.19        C
ATOM  12900  OD1  ASP H 175    19.257  32.580 -22.172  1.00 33.26        O
ATOM  12901  OD2  ASP H 175    19.308  32.663 -19.968  1.00 35.29        O
ATOM  12902  N    LEU H 176    17.146  27.630 -21.821  1.00 19.24        N
ATOM  12903  CA   LEU H 176    17.177  26.156 -21.780  1.00 17.67        C
ATOM  12904  C    LEU H 176    16.277  25.546 -22.830  1.00 16.48        C
ATOM  12905  O    LEU H 176    15.256  26.122 -23.178  1.00 16.65        O
ATOM  12906  CB   LEU H 176    16.734  25.665 -20.419  1.00 17.36        C
ATOM  12907  CG   LEU H 176    17.655  26.059 -19.267  1.00 16.49        C
ATOM  12908  CD1  LEU H 176    16.916  25.762 -17.965  1.00 18.95        C
ATOM  12909  CD2  LEU H 176    19.001  25.335 -19.359  1.00 15.94        C
ATOM  12910  N    TYR H 177    16.664  24.378 -23.331  1.00 13.36        N
ATOM  12911  CA   TYR H 177    15.863  23.695 -24.350  1.00 13.79        C
ATOM  12912  C    TYR H 177    14.843  22.734 -23.746  1.00 14.23        C
ATOM  12913  O    TYR H 177    15.082  22.107 -22.710  1.00 13.47        O
ATOM  12914  CB   TYR H 177    16.759  22.934 -25.324  1.00 15.05        C
ATOM  12915  CG   TYR H 177    17.588  23.844 -26.188  1.00 16.82        C
ATOM  12916  CD1  TYR H 177    17.070  24.393 -27.343  1.00 15.86        C
ATOM  12917  CD2  TYR H 177    18.890  24.161 -25.824  1.00 16.56        C
ATOM  12918  CE1  TYR H 177    17.842  25.228 -28.142  1.00 18.70        C
ATOM  12919  CE2  TYR H 177    19.683  24.972 -26.615  1.00 17.61        C
ATOM  12920  CZ   TYR H 177    19.152  25.513 -27.771  1.00 19.37        C
ATOM  12921  OH   TYR H 177    19.952  26.364 -28.538  1.00 22.43        O
ATOM  12922  N    THR H 178    13.710  22.629 -24.433  1.00 14.67        N
ATOM  12923  CA   THR H 178    12.715  21.593 -24.153  1.00 14.72        C
```

```
ATOM  12924  C    THR H 178   12.215  20.976 -25.459  1.00 14.32        C
ATOM  12925  O    THR H 178   12.024  21.656 -26.451  1.00 13.59        O
ATOM  12926  CB   THR H 178   11.519  22.179 -23.354  1.00 15.22        C
ATOM  12927  OG1  THR H 178   11.973  22.672 -22.087  1.00 18.11        O
ATOM  12928  CG2  THR H 178   10.458  21.112 -23.083  1.00 14.58        C
ATOM  12929  N    LEU H 179   12.017  19.659 -25.478  1.00 13.60        N
ATOM  12930  CA   LEU H 179   11.347  19.053 -26.593  1.00 14.05        C
ATOM  12931  C    LEU H 179   10.537  17.851 -26.111  1.00 14.00        C
ATOM  12932  O    LEU H 179   10.672  17.426 -24.978  1.00 14.71        O
ATOM  12933  CB   LEU H 179   12.341  18.620 -27.664  1.00 16.34        C
ATOM  12934  CG   LEU H 179   13.315  17.480 -27.414  1.00 16.10        C
ATOM  12935  CD1  LEU H 179   12.711  16.063 -27.180  1.00 16.80        C
ATOM  12936  CD2  LEU H 179   14.209  17.422 -28.654  1.00 16.24        C
ATOM  12937  N    SER H 180    9.637  17.379 -26.958  1.00 16.47        N
ATOM  12938  CA   SER H 180    8.892  16.162 -26.700  1.00 18.24        C
ATOM  12939  C    SER H 180    9.029  15.192 -27.850  1.00 17.80        C
ATOM  12940  O    SER H 180    9.314  15.575 -28.987  1.00 14.87        O
ATOM  12941  CB   SER H 180    7.398  16.452 -26.522  1.00 20.06        C
ATOM  12942  OG   SER H 180    7.158  17.077 -25.281  1.00 24.60        O
ATOM  12943  N    SER H 181    8.824  13.923 -27.512  1.00 16.86        N
ATOM  12944  CA   SER H 181    8.726  12.857 -28.502  1.00 17.37        C
ATOM  12945  C    SER H 181    7.385  12.157 -28.352  1.00 17.53        C
ATOM  12946  O    SER H 181    6.891  11.978 -27.237  1.00 14.16        O
ATOM  12947  CB   SER H 181    9.848  11.840 -28.300  1.00 17.12        C
ATOM  12948  OG   SER H 181    9.931  10.936 -29.382  1.00 18.48        O
ATOM  12949  N    SER H 182    6.809  11.739 -29.472  1.00 19.64        N
ATOM  12950  CA   SER H 182    5.606  10.915 -29.415  1.00 20.10        C
ATOM  12951  C    SER H 182    5.818   9.608 -30.131  1.00 19.35        C
ATOM  12952  O    SER H 182    6.569   9.532 -31.103  1.00 19.16        O
ATOM  12953  CB   SER H 182    4.386  11.679 -29.933  1.00 21.74        C
ATOM  12954  OG   SER H 182    4.117  11.557 -31.296  1.00 22.32        O
ATOM  12955  N    VAL H 183    5.187   8.569 -29.593  1.00 19.81        N
ATOM  12956  CA   VAL H 183    5.121   7.275 -30.264  1.00 20.24        C
ATOM  12957  C    VAL H 183    3.683   6.774 -30.259  1.00 20.56        C
ATOM  12958  O    VAL H 183    2.960   6.916 -29.272  1.00 20.30        O
ATOM  12959  CB   VAL H 183    6.105   6.207 -29.665  1.00 20.16        C
ATOM  12960  CG1  VAL H 183    5.746   5.790 -28.266  1.00 20.28        C
ATOM  12961  CG2  VAL H 183    6.181   4.989 -30.572  1.00 19.78        C
ATOM  12962  N    THR H 184    3.281   6.201 -31.383  1.00 21.38        N
ATOM  12963  CA   THR H 184    1.948   5.645 -31.524  1.00 22.97        C
ATOM  12964  C    THR H 184    2.094   4.142 -31.822  1.00 23.41        C
ATOM  12965  O    THR H 184    2.784   3.757 -32.759  1.00 22.81        O
ATOM  12966  CB   THR H 184    1.196   6.387 -32.601  1.00 23.97        C
ATOM  12967  OG1  THR H 184    1.118   7.775 -32.226  1.00 24.23        O
ATOM  12968  CG2  THR H 184   -0.200   5.849 -32.795  1.00 23.28        C
ATOM  12969  N    VAL H 185    1.478   3.324 -30.971  1.00 23.75        N
ATOM  12970  CA   VAL H 185    1.555   1.869 -31.059  1.00 24.50        C
ATOM  12971  C    VAL H 185    0.142   1.259 -31.016  1.00 25.02        C
ATOM  12972  O    VAL H 185   -0.790   1.903 -30.552  1.00 23.37        O
ATOM  12973  CB   VAL H 185    2.397   1.255 -29.907  1.00 25.28        C
ATOM  12974  CG1  VAL H 185    3.826   1.754 -29.963  1.00 25.44        C
ATOM  12975  CG2  VAL H 185    1.787   1.523 -28.542  1.00 23.99        C
ATOM  12976  N    PRO H 186   -0.017   0.011 -31.496  1.00 26.31        N
ATOM  12977  CA   PRO H 186   -1.346  -0.615 -31.370  1.00 26.50        C
ATOM  12978  C    PRO H 186   -1.752  -0.799 -29.912  1.00 26.52        C
ATOM  12979  O    PRO H 186   -0.912  -1.144 -29.086  1.00 24.90        O
ATOM  12980  CB   PRO H 186   -1.171  -1.975 -32.072  1.00 27.12        C
ATOM  12981  CG   PRO H 186    0.051  -1.864 -32.877  1.00 25.88        C
ATOM  12982  CD   PRO H 186    0.948  -0.884 -32.155  1.00 25.98        C
ATOM  12983  N    SER H 187   -3.026  -0.566 -29.597  1.00 26.97        N
ATOM  12984  CA   SER H 187   -3.514  -0.720 -28.221  1.00 28.26        C
ATOM  12985  C    SER H 187   -3.307  -2.144 -27.697  1.00 28.04        C
ATOM  12986  O    SER H 187   -3.129  -2.353 -26.500  1.00 27.53        O
ATOM  12987  CB   SER H 187   -4.996  -0.336 -28.127  1.00 28.59        C
ATOM  12988  OG   SER H 187   -5.213   0.973 -28.622  1.00 30.51        O
```

```
ATOM   12989  N    SER H 188      -3.301  -3.114 -28.609  1.00 29.86           N
ATOM   12990  CA   SER H 188      -3.000  -4.510 -28.265  1.00 30.15           C
ATOM   12991  C    SER H 188      -1.573  -4.756 -27.753  1.00 30.76           C
ATOM   12992  O    SER H 188      -1.312  -5.795 -27.148  1.00 30.78           O
ATOM   12993  CB   SER H 188      -3.299  -5.434 -29.456  1.00 29.83           C
ATOM   12994  OG   SER H 188      -2.723  -4.967 -30.660  1.00 28.12           O
ATOM   12995  N    THR H 189      -0.657  -3.809 -27.981  1.00 31.24           N
ATOM   12996  CA   THR H 189       0.730  -3.933 -27.510  1.00 31.23           C
ATOM   12997  C    THR H 189       1.058  -3.106 -26.255  1.00 31.79           C
ATOM   12998  O    THR H 189       2.080  -3.343 -25.619  1.00 33.03           O
ATOM   12999  CB   THR H 189       1.713  -3.522 -28.611  1.00 30.85           C
ATOM   13000  OG1  THR H 189       1.522  -2.131 -28.902  1.00 29.38           O
ATOM   13001  CG2  THR H 189       1.467  -4.332 -29.875  1.00 29.68           C
ATOM   13002  N    TRP H 190       0.219  -2.139 -25.896  1.00 31.80           N
ATOM   13003  CA   TRP H 190       0.464  -1.322 -24.701  1.00 31.83           C
ATOM   13004  C    TRP H 190      -0.819  -1.160 -23.902  1.00 33.46           C
ATOM   13005  O    TRP H 190      -1.854  -0.842 -24.488  1.00 34.05           O
ATOM   13006  CB   TRP H 190       0.992   0.078 -25.083  1.00 30.29           C
ATOM   13007  CG   TRP H 190       1.451   0.830 -23.896  1.00 29.46           C
ATOM   13008  CD1  TRP H 190       2.677   0.759 -23.320  1.00 29.39           C
ATOM   13009  CD2  TRP H 190       0.682   1.731 -23.093  1.00 28.79           C
ATOM   13010  NE1  TRP H 190       2.727   1.556 -22.207  1.00 28.61           N
ATOM   13011  CE2  TRP H 190       1.517   2.166 -22.040  1.00 28.91           C
ATOM   13012  CE3  TRP H 190      -0.635   2.196 -23.146  1.00 29.71           C
ATOM   13013  CZ2  TRP H 190       1.092   3.065 -21.064  1.00 28.26           C
ATOM   13014  CZ3  TRP H 190      -1.062   3.101 -22.170  1.00 29.93           C
ATOM   13015  CH2  TRP H 190      -0.195   3.520 -21.143  1.00 29.56           C
ATOM   13016  N    PRO H 191      -0.751  -1.282 -22.563  1.00 34.95           N
ATOM   13017  CA   PRO H 191       0.366  -1.547 -21.659  1.00 36.27           C
ATOM   13018  C    PRO H 191       0.790  -2.999 -21.519  1.00 38.34           C
ATOM   13019  O    PRO H 191       1.589  -3.318 -20.632  1.00 38.54           O
ATOM   13020  CB   PRO H 191      -0.165  -1.054 -20.320  1.00 36.11           C
ATOM   13021  CG   PRO H 191      -1.590  -1.356 -20.386  1.00 35.81           C
ATOM   13022  CD   PRO H 191      -1.983  -1.022 -21.801  1.00 35.40           C
ATOM   13023  N    SER H 192       0.252  -3.858 -22.380  1.00 39.68           N
ATOM   13024  CA   SER H 192       0.601  -5.271 -22.435  1.00 40.61           C
ATOM   13025  C    SER H 192       2.121  -5.468 -22.463  1.00 41.22           C
ATOM   13026  O    SER H 192       2.680  -6.169 -21.618  1.00 42.45           O
ATOM   13027  CB   SER H 192      -0.046  -5.886 -23.688  1.00 40.47           C
ATOM   13028  OG   SER H 192      -1.117  -5.058 -24.150  1.00 42.12           O
ATOM   13029  N    GLU H 193       2.771  -4.835 -23.437  1.00 41.12           N
ATOM   13030  CA   GLU H 193       4.224  -4.875 -23.595  1.00 40.96           C
ATOM   13031  C    GLU H 193       4.814  -3.477 -23.368  1.00 39.70           C
ATOM   13032  O    GLU H 193       4.184  -2.473 -23.694  1.00 39.33           O
ATOM   13033  CB   GLU H 193       4.573  -5.389 -24.981  1.00 42.18           C
ATOM   13034  CG   GLU H 193       4.214  -6.868 -25.176  1.00 44.94           C
ATOM   13035  CD   GLU H 193       3.516  -7.160 -26.503  1.00 47.00           C
ATOM   13036  OE1  GLU H 193       3.455  -6.261 -27.370  1.00 47.94           O
ATOM   13037  OE2  GLU H 193       3.010  -8.299 -26.674  1.00 49.65           O
ATOM   13038  N    THR H 194       6.024  -3.426 -22.816  1.00 37.41           N
ATOM   13039  CA   THR H 194       6.565  -2.183 -22.254  1.00 36.34           C
ATOM   13040  C    THR H 194       7.027  -1.212 -23.333  1.00 33.82           C
ATOM   13041  O    THR H 194       7.549  -1.611 -24.372  1.00 33.91           O
ATOM   13042  CB   THR H 194       7.750  -2.436 -21.291  1.00 36.64           C
ATOM   13043  OG1  THR H 194       8.897  -2.803 -22.045  1.00 37.21           O
ATOM   13044  CG2  THR H 194       7.432  -3.553 -20.284  1.00 37.45           C
ATOM   13045  N    VAL H 195       6.811   0.071 -23.065  1.00 32.56           N
ATOM   13046  CA   VAL H 195       7.293   1.163 -23.914  1.00 30.06           C
ATOM   13047  C    VAL H 195       8.112   2.082 -23.012  1.00 28.59           C
ATOM   13048  O    VAL H 195       7.615   2.575 -21.987  1.00 25.95           O
ATOM   13049  CB   VAL H 195       6.128   1.940 -24.553  1.00 30.15           C
ATOM   13050  CG1  VAL H 195       6.632   3.123 -25.370  1.00 30.18           C
ATOM   13051  CG2  VAL H 195       5.288   1.005 -25.430  1.00 30.16           C
ATOM   13052  N    THR H 196       9.377   2.254 -23.387  1.00 27.15           N
ATOM   13053  CA   THR H 196      10.339   3.045 -22.623  1.00 26.52           C
```

| ATOM | 13054 | C | THR | H | 196 | 10.950 | 4.097 | -23.545 | 1.00 | 24.82 | C |
| ATOM | 13055 | O | THR | H | 196 | 11.402 | 3.769 | -24.634 | 1.00 | 24.45 | O |
| ATOM | 13056 | CB | THR | H | 196 | 11.452 | 2.128 | -22.108 | 1.00 | 26.94 | C |
| ATOM | 13057 | OG1 | THR | H | 196 | 10.895 | 1.148 | -21.213 | 1.00 | 28.23 | O |
| ATOM | 13058 | CG2 | THR | H | 196 | 12.520 | 2.903 | -21.376 | 1.00 | 26.19 | C |
| ATOM | 13059 | N | CYS | H | 197 | 11.003 | 5.354 | -23.126 | 1.00 | 25.56 | N |
| ATOM | 13060 | CA | CYS | H | 197 | 11.809 | 6.294 | -23.902 | 1.00 | 25.34 | C |
| ATOM | 13061 | C | CYS | H | 197 | 13.200 | 6.411 | -23.289 | 1.00 | 23.55 | C |
| ATOM | 13062 | O | CYS | H | 197 | 13.363 | 6.350 | -22.065 | 1.00 | 24.46 | O |
| ATOM | 13063 | CB | CYS | H | 197 | 11.137 | 7.628 | -24.073 | 1.00 | 27.77 | C |
| ATOM | 13064 | SG | CYS | H | 197 | 11.305 | 8.676 | -22.733 | 1.00 | 32.68 | S |
| ATOM | 13065 | N | ASN | H | 198 | 14.184 | 6.539 | -24.173 | 1.00 | 21.90 | N |
| ATOM | 13066 | CA | ASN | H | 198 | 15.603 | 6.649 | -23.818 | 1.00 | 21.25 | C |
| ATOM | 13067 | C | ASN | H | 198 | 16.114 | 8.023 | -24.266 | 1.00 | 19.59 | C |
| ATOM | 13068 | O | ASN | H | 198 | 15.908 | 8.417 | -25.417 | 1.00 | 17.33 | O |
| ATOM | 13069 | CB | ASN | H | 198 | 16.401 | 5.545 | -24.504 | 1.00 | 23.42 | C |
| ATOM | 13070 | CG | ASN | H | 198 | 15.630 | 4.232 | -24.593 | 1.00 | 24.79 | C |
| ATOM | 13071 | OD1 | ASN | H | 198 | 15.179 | 3.848 | -25.674 | 1.00 | 26.49 | O |
| ATOM | 13072 | ND2 | ASN | H | 198 | 15.480 | 3.550 | -23.461 | 1.00 | 27.29 | N |
| ATOM | 13073 | N | VAL | H | 199 | 16.698 | 8.774 | -23.342 | 1.00 | 18.99 | N |
| ATOM | 13074 | CA | VAL | H | 199 | 17.117 | 10.134 | -23.653 | 1.00 | 18.75 | C |
| ATOM | 13075 | C | VAL | H | 199 | 18.606 | 10.258 | -23.379 | 1.00 | 18.12 | C |
| ATOM | 13076 | O | VAL | H | 199 | 19.046 | 10.108 | -22.250 | 1.00 | 19.01 | O |
| ATOM | 13077 | CB | VAL | H | 199 | 16.308 | 11.182 | -22.853 | 1.00 | 18.44 | C |
| ATOM | 13078 | CG1 | VAL | H | 199 | 16.775 | 12.600 | -23.204 | 1.00 | 19.83 | C |
| ATOM | 13079 | CG2 | VAL | H | 199 | 14.805 | 11.018 | -23.156 | 1.00 | 18.49 | C |
| ATOM | 13080 | N | ALA | H | 200 | 19.360 | 10.533 | -24.435 | 1.00 | 17.53 | N |
| ATOM | 13081 | CA | ALA | H | 200 | 20.780 | 10.748 | -24.323 | 1.00 | 16.84 | C |
| ATOM | 13082 | C | ALA | H | 200 | 21.072 | 12.231 | -24.556 | 1.00 | 16.23 | C |
| ATOM | 13083 | O | ALA | H | 200 | 20.549 | 12.839 | -25.483 | 1.00 | 16.68 | O |
| ATOM | 13084 | CB | ALA | H | 200 | 21.515 | 9.900 | -25.338 | 1.00 | 18.14 | C |
| ATOM | 13085 | N | HIS | H | 201 | 21.902 | 12.786 | -23.688 | 1.00 | 15.43 | N |
| ATOM | 13086 | CA | HIS | H | 201 | 22.355 | 14.166 | -23.786 | 1.00 | 13.25 | C |
| ATOM | 13087 | C | HIS | H | 201 | 23.879 | 14.098 | -23.740 | 1.00 | 12.91 | C |
| ATOM | 13088 | O | HIS | H | 201 | 24.457 | 13.958 | -22.667 | 1.00 | 14.40 | O |
| ATOM | 13089 | CB | HIS | H | 201 | 21.817 | 14.965 | -22.615 | 1.00 | 12.15 | C |
| ATOM | 13090 | CG | HIS | H | 201 | 22.139 | 16.426 | -22.688 | 1.00 | 10.37 | C |
| ATOM | 13091 | ND1 | HIS | H | 201 | 22.706 | 17.125 | -21.635 | 1.00 | 11.84 | N |
| ATOM | 13092 | CD2 | HIS | H | 201 | 22.004 | 17.311 | -23.704 | 1.00 | 12.61 | C |
| ATOM | 13093 | CE1 | HIS | H | 201 | 22.876 | 18.391 | -22.001 | 1.00 | 11.52 | C |
| ATOM | 13094 | NE2 | HIS | H | 201 | 22.470 | 18.525 | -23.254 | 1.00 | 10.21 | N |
| ATOM | 13095 | N | PRO | H | 202 | 24.528 | 14.106 | -24.910 | 1.00 | 13.17 | N |
| ATOM | 13096 | CA | PRO | H | 202 | 25.964 | 13.887 | -24.926 | 1.00 | 13.67 | C |
| ATOM | 13097 | C | PRO | H | 202 | 26.775 | 14.886 | -24.127 | 1.00 | 12.69 | C |
| ATOM | 13098 | O | PRO | H | 202 | 27.802 | 14.516 | -23.545 | 1.00 | 11.77 | O |
| ATOM | 13099 | CB | PRO | H | 202 | 26.351 | 13.977 | -26.395 | 1.00 | 14.23 | C |
| ATOM | 13100 | CG | PRO | H | 202 | 25.096 | 14.009 | -27.159 | 1.00 | 16.95 | C |
| ATOM | 13101 | CD | PRO | H | 202 | 23.945 | 14.227 | -26.262 | 1.00 | 14.94 | C |
| ATOM | 13102 | N | ALA | H | 203 | 26.354 | 16.137 | -24.101 | 1.00 | 12.24 | N |
| ATOM | 13103 | CA | ALA | H | 203 | 27.143 | 17.115 | -23.356 | 1.00 | 13.16 | C |
| ATOM | 13104 | C | ALA | H | 203 | 27.244 | 16.805 | -21.859 | 1.00 | 14.06 | C |
| ATOM | 13105 | O | ALA | H | 203 | 28.237 | 17.161 | -21.257 | 1.00 | 13.17 | O |
| ATOM | 13106 | CB | ALA | H | 203 | 26.628 | 18.482 | -23.556 | 1.00 | 13.31 | C |
| ATOM | 13107 | N | SER | H | 204 | 26.238 | 16.147 | -21.276 | 1.00 | 14.62 | N |
| ATOM | 13108 | CA | SER | H | 204 | 26.249 | 15.789 | -19.843 | 1.00 | 14.49 | C |
| ATOM | 13109 | C | SER | H | 204 | 26.577 | 14.309 | -19.644 | 1.00 | 14.43 | C |
| ATOM | 13110 | O | SER | H | 204 | 26.578 | 13.844 | -18.518 | 1.00 | 15.28 | O |
| ATOM | 13111 | CB | SER | H | 204 | 24.897 | 16.115 | -19.141 | 1.00 | 14.11 | C |
| ATOM | 13112 | OG | SER | H | 204 | 23.768 | 15.555 | -19.817 | 1.00 | 14.49 | O |
| ATOM | 13113 | N | SER | H | 205 | 26.847 | 13.596 | -20.728 | 1.00 | 15.09 | N |
| ATOM | 13114 | CA | SER | H | 205 | 27.150 | 12.169 | -20.654 | 1.00 | 16.80 | C |
| ATOM | 13115 | C | SER | H | 205 | 26.066 | 11.431 | -19.888 | 1.00 | 18.11 | C |
| ATOM | 13116 | O | SER | H | 205 | 26.357 | 10.518 | -19.120 | 1.00 | 18.99 | O |
| ATOM | 13117 | CB | SER | H | 205 | 28.525 | 11.957 | -20.015 | 1.00 | 17.82 | C |
| ATOM | 13118 | OG | SER | H | 205 | 29.501 | 12.670 | -20.745 | 1.00 | 16.89 | O |

```
ATOM  13119  N   THR H 206     24.811  11.802 -20.130  1.00 17.83          N
ATOM  13120  CA  THR H 206     23.686  11.168 -19.453  1.00 19.72          C
ATOM  13121  C   THR H 206     22.838  10.364 -20.433  1.00 19.57          C
ATOM  13122  O   THR H 206     22.712  10.698 -21.609  1.00 16.35          O
ATOM  13123  CB  THR H 206     22.809  12.168 -18.730  1.00 20.78          C
ATOM  13124  OG1 THR H 206     22.433  13.224 -19.621  1.00 19.32          O
ATOM  13125  CG2 THR H 206     23.528  12.716 -17.523  1.00 22.21          C
ATOM  13126  N   LYS H 207     22.316   9.254 -19.935  1.00 21.18          N
ATOM  13127  CA  LYS H 207     21.373   8.455 -20.676  1.00 24.38          C
ATOM  13128  C   LYS H 207     20.328   8.032 -19.653  1.00 24.28          C
ATOM  13129  O   LYS H 207     20.666   7.394 -18.650  1.00 24.29          O
ATOM  13130  CB  LYS H 207     22.069   7.257 -21.326  1.00 26.97          C
ATOM  13131  CG  LYS H 207     21.739   7.077 -22.809  1.00 30.27          C
ATOM  13132  CD  LYS H 207     20.279   6.706 -23.081  1.00 31.60          C
ATOM  13133  CE  LYS H 207     20.038   6.366 -24.565  1.00 31.87          C
ATOM  13134  NZ  LYS H 207     20.302   4.932 -24.927  1.00 36.02          N
ATOM  13135  N   VAL H 208     19.089   8.480 -19.857  1.00 24.78          N
ATOM  13136  CA  VAL H 208     18.021   8.277 -18.901  1.00 25.14          C
ATOM  13137  C   VAL H 208     16.920   7.476 -19.619  1.00 27.22          C
ATOM  13138  O   VAL H 208     16.593   7.744 -20.779  1.00 25.42          O
ATOM  13139  CB  VAL H 208     17.476   9.623 -18.331  1.00 25.59          C
ATOM  13140  CG1 VAL H 208     16.234   9.397 -17.482  1.00 27.81          C
ATOM  13141  CG2 VAL H 208     18.532  10.339 -17.498  1.00 25.73          C
ATOM  13142  N   ASP H 209     16.370   6.491 -18.913  1.00 28.44          N
ATOM  13143  CA  ASP H 209     15.277   5.661 -19.423  1.00 29.69          C
ATOM  13144  C   ASP H 209     14.052   5.933 -18.576  1.00 29.72          C
ATOM  13145  O   ASP H 209     14.140   5.927 -17.361  1.00 29.05          O
ATOM  13146  CB  ASP H 209     15.611   4.176 -19.314  1.00 30.61          C
ATOM  13147  CG  ASP H 209     16.744   3.758 -20.214  1.00 32.45          C
ATOM  13148  OD1 ASP H 209     16.858   4.242 -21.359  1.00 34.17          O
ATOM  13149  OD2 ASP H 209     17.552   2.925 -19.767  1.00 36.72          O
ATOM  13150  N   LYS H 210     12.915   6.192 -19.218  1.00 31.24          N
ATOM  13151  CA  LYS H 210     11.656   6.327 -18.504  1.00 32.39          C
ATOM  13152  C   LYS H 210     10.611   5.431 -19.167  1.00 32.53          C
ATOM  13153  O   LYS H 210     10.223   5.643 -20.317  1.00 30.83          O
ATOM  13154  CB  LYS H 210     11.176   7.780 -18.468  1.00 33.95          C
ATOM  13155  CG  LYS H 210     12.094   8.736 -17.698  1.00 36.04          C
ATOM  13156  CD  LYS H 210     12.125   8.499 -16.186  1.00 36.28          C
ATOM  13157  CE  LYS H 210     13.032   9.557 -15.494  1.00 36.89          C
ATOM  13158  NZ  LYS H 210     12.794   9.674 -14.015  1.00 37.60          N
ATOM  13159  N   LYS H 211     10.180   4.410 -18.430  1.00 33.71          N
ATOM  13160  CA  LYS H 211      9.101   3.523 -18.878  1.00 33.77          C
ATOM  13161  C   LYS H 211      7.769   4.265 -18.771  1.00 32.16          C
ATOM  13162  O   LYS H 211      7.505   4.971 -17.799  1.00 31.65          O
ATOM  13163  CB  LYS H 211      9.091   2.229 -18.043  1.00 34.37          C
ATOM  13164  CG  LYS H 211      8.060   1.173 -18.466  1.00 34.85          C
ATOM  13165  CD  LYS H 211      8.232  -0.094 -17.627  1.00 35.06          C
ATOM  13166  CE  LYS H 211      7.020  -1.030 -17.657  1.00 35.42          C
ATOM  13167  NZ  LYS H 211      7.303  -2.265 -16.839  1.00 35.63          N
ATOM  13168  N   ILE H 212      6.943   4.126 -19.800  1.00 31.46          N
ATOM  13169  CA  ILE H 212      5.630   4.730 -19.805  1.00 31.09          C
ATOM  13170  C   ILE H 212      4.646   3.760 -19.119  1.00 33.29          C
ATOM  13171  O   ILE H 212      4.422   2.661 -19.606  1.00 32.08          O
ATOM  13172  CB  ILE H 212      5.154   5.030 -21.249  1.00 30.77          C
ATOM  13173  CG1 ILE H 212      6.204   5.840 -22.032  1.00 30.00          C
ATOM  13174  CG2 ILE H 212      3.836   5.777 -21.220  1.00 29.73          C
ATOM  13175  CD1 ILE H 212      6.769   7.042 -21.291  1.00 30.23          C
ATOM  13176  N   VAL H 213      4.067   4.174 -17.997  1.00 35.32          N
ATOM  13177  CA  VAL H 213      3.137   3.303 -17.270  1.00 38.22          C
ATOM  13178  C   VAL H 213      1.753   3.923 -17.226  1.00 39.16          C
ATOM  13179  O   VAL H 213      1.624   5.142 -17.107  1.00 39.45          O
ATOM  13180  CB  VAL H 213      3.620   2.984 -15.829  1.00 38.54          C
ATOM  13181  CG1 VAL H 213      5.039   2.460 -15.856  1.00 38.70          C
ATOM  13182  CG2 VAL H 213      3.509   4.197 -14.917  1.00 39.15          C
ATOM  13183  N   PRO H 214      0.700   3.090 -17.341  1.00 41.54          N
```

```
ATOM   13184  CA   PRO H 214    -0.636    3.657 -17.204  1.00 42.84          C
ATOM   13185  C    PRO H 214    -0.775    4.366 -15.867  1.00 44.20          C
ATOM   13186  O    PRO H 214    -0.119    3.977 -14.901  1.00 44.98          O
ATOM   13187  CB   PRO H 214    -1.547    2.430 -17.284  1.00 42.91          C
ATOM   13188  CG   PRO H 214    -0.756    1.426 -18.038  1.00 42.32          C
ATOM   13189  CD   PRO H 214     0.655    1.643 -17.621  1.00 41.63          C
ATOM   13190  N    ARG H 215    -1.607    5.399 -15.820  1.00 45.78          N
ATOM   13191  CA   ARG H 215    -1.693    6.275 -14.647  1.00 47.82          C
ATOM   13192  C    ARG H 215    -2.293    5.565 -13.433  1.00 47.94          C
ATOM   13193  O    ARG H 215    -3.502    5.376 -13.357  1.00 48.37          O
ATOM   13194  CB   ARG H 215    -2.514    7.524 -14.965  1.00 47.75          C
ATOM   13195  CG   ARG H 215    -2.073    8.231 -16.217  1.00 49.37          C
ATOM   13196  CD   ARG H 215    -2.731    9.592 -16.368  1.00 50.27          C
ATOM   13197  NE   ARG H 215    -1.982   10.642 -15.683  1.00 51.96          N
ATOM   13198  CZ   ARG H 215    -0.833   11.158 -16.122  1.00 52.71          C
ATOM   13199  NH1  ARG H 215    -0.282   10.720 -17.252  1.00 53.18          N
ATOM   13200  NH2  ARG H 215    -0.227   12.117 -15.427  1.00 52.56          N
TER    13201       ARG H 215
HETATM13202  C1   EAG B 310   -14.064   25.986  31.772  1.00 38.92          C
HETATM13203  O1   EAG B 310   -14.022   26.398  33.124  1.00 42.01          O
HETATM13204  C2   EAG B 310   -12.651   26.084  31.192  1.00 36.63          C
HETATM13205  N2   EAG B 310   -12.138   27.438  31.294  1.00 35.60          N
HETATM13206  C7   EAG B 310   -11.065   27.718  32.032  1.00 34.40          C
HETATM13207  O7   EAG B 310   -10.492   26.881  32.732  1.00 35.21          O
HETATM13208  C8   EAG B 310   -10.550   29.124  31.997  1.00 33.95          C
HETATM13209  C3   EAG B 310   -12.591   25.586  29.754  1.00 36.09          C
HETATM13210  O3   EAG B 310   -11.225   25.430  29.369  1.00 32.19          O
HETATM13211  C4   EAG B 310   -13.326   24.255  29.633  1.00 37.93          C
HETATM13212  O4   EAG B 310   -13.508   23.959  28.276  1.00 39.73          O
HETATM13213  C5   EAG B 310   -14.691   24.267  30.327  1.00 39.99          C
HETATM13214  C6   EAG B 310   -15.371   22.898  30.316  1.00 40.99          C
HETATM13215  O6   EAG B 310   -14.651   21.993  31.134  1.00 43.71          O
HETATM13216  O5   EAG B 310   -14.509   24.643  31.668  1.00 38.89          O
HETATM13217  C9   EAG B 310   -14.659   25.655  34.173  1.00 43.25          C
HETATM13218  C1   RAM B 308   -10.814   26.371  28.359  1.00 28.78          C
HETATM13219  C2   RAM B 308    -9.304   26.532  28.426  1.00 25.64          C
HETATM13220  C3   RAM B 308    -8.609   25.227  28.039  1.00 23.24          C
HETATM13221  C4   RAM B 308    -9.079   24.743  26.670  1.00 23.35          C
HETATM13222  C5   RAM B 308   -10.615   24.666  26.714  1.00 24.58          C
HETATM13223  C6   RAM B 308   -11.215   24.237  25.393  1.00 25.69          C
HETATM13224  O2   RAM B 308    -8.895   27.507  27.506  1.00 26.13          O
HETATM13225  O3   RAM B 308    -7.216   25.451  28.014  1.00 20.78          O
HETATM13226  O4   RAM B 308    -8.467   23.482  26.437  1.00 20.90          O
HETATM13227  O5   RAM B 308   -11.153   25.943  27.053  1.00 27.76          O
HETATM13228  C1   RAM B 307    -6.595   24.779  29.115  1.00 21.13          C
HETATM13229  C2   RAM B 307    -5.097   24.736  28.853  1.00 19.58          C
HETATM13230  C3   RAM B 307    -4.510   26.130  28.891  1.00 19.67          C
HETATM13231  C4   RAM B 307    -4.851   26.785  30.220  1.00 21.87          C
HETATM13232  C5   RAM B 307    -6.373   26.759  30.416  1.00 21.97          C
HETATM13233  C6   RAM B 307    -6.749   27.363  31.756  1.00 23.21          C
HETATM13234  O2   RAM B 307    -4.449   23.959  29.839  1.00 18.53          O
HETATM13235  O3   RAM B 307    -3.109   26.171  28.651  1.00 20.51          O
HETATM13236  O4   RAM B 307    -4.348   28.092  30.157  1.00 20.40          O
HETATM13237  O5   RAM B 307    -6.878   25.429  30.350  1.00 21.64          O
HETATM13238  C1   RAM B 306    -4.325   22.574  29.537  1.00 17.82          C
HETATM13239  C2   RAM B 306    -3.193   22.062  30.411  1.00 16.76          C
HETATM13240  C3   RAM B 306    -3.616   22.227  31.878  1.00 19.32          C
HETATM13241  C4   RAM B 306    -4.926   21.489  32.128  1.00 21.94          C
HETATM13242  C5   RAM B 306    -5.975   21.981  31.118  1.00 22.02          C
HETATM13243  C6   RAM B 306    -7.246   21.171  31.217  1.00 23.12          C
HETATM13244  O2   RAM B 306    -3.072   20.671  30.189  1.00 14.54          O
HETATM13245  O3   RAM B 306    -2.613   21.732  32.723  1.00 19.89          O
HETATM13246  O4   RAM B 306    -5.372   21.689  33.473  1.00 22.27          O
HETATM13247  O5   RAM B 306    -5.474   21.800  29.802  1.00 21.27          O
HETATM13248  C1   NAG B 305    -1.951   20.282  29.428  1.00 13.48          C
```

```
HETATM13249  C2  NAG B 305   -2.005  18.770  29.516  1.00 13.59        C
HETATM13250  C3  NAG B 305   -1.170  18.117  28.438  1.00 15.46        C
HETATM13251  C4  NAG B 305   -1.326  18.804  27.067  1.00 13.39        C
HETATM13252  C5  NAG B 305   -1.087  20.291  27.197  1.00 13.10        C
HETATM13253  C6  NAG B 305   -1.171  21.004  25.860  1.00 13.12        C
HETATM13254  C7  NAG B 305   -2.515  17.768  31.715  1.00 17.75        C
HETATM13255  C8  NAG B 305   -2.056  17.588  33.122  1.00 18.24        C
HETATM13256  N2  NAG B 305   -1.611  18.278  30.842  1.00 15.28        N
HETATM13257  O3  NAG B 305   -1.614  16.798  28.294  1.00 15.51        O
HETATM13258  O4  NAG B 305   -0.398  18.245  26.174  1.00 14.68        O
HETATM13259  O5  NAG B 305   -2.074  20.776  28.099  1.00 14.55        O
HETATM13260  O6  NAG B 305   -2.448  20.795  25.305  1.00 14.33        O
HETATM13261  O7  NAG B 305   -3.666  17.444  31.430  1.00 20.22        O
HETATM13262  C1  RAM B 303   -0.740  15.783  28.814  1.00 15.89        C
HETATM13263  C2  RAM B 303   -1.587  14.612  29.310  1.00 16.93        C
HETATM13264  C3  RAM B 303   -2.195  13.886  28.125  1.00 18.78        C
HETATM13265  C4  RAM B 303   -1.123  13.463  27.124  1.00 15.52        C
HETATM13266  C5  RAM B 303   -0.361  14.718  26.684  1.00 14.42        C
HETATM13267  C6  RAM B 303    0.707  14.465  25.612  1.00 13.58        C
HETATM13268  O2  RAM B 303   -0.712  13.738  29.995  1.00 16.83        O
HETATM13269  O3  RAM B 303   -2.954  12.761  28.552  1.00 24.00        O
HETATM13270  O4  RAM B 303   -1.750  12.901  25.998  1.00 15.76        O
HETATM13271  O5  RAM B 303    0.188  15.315  27.859  1.00 15.47        O
HETATM13272  C1  RAM B 302   -4.368  13.016  28.282  1.00 29.83        C
HETATM13273  C2  RAM B 302   -5.175  11.721  28.153  1.00 32.85        C
HETATM13274  C3  RAM B 302   -4.979  10.953  29.459  1.00 34.07        C
HETATM13275  C4  RAM B 302   -5.392  11.836  30.648  1.00 34.83        C
HETATM13276  C5  RAM B 302   -4.789  13.243  30.574  1.00 33.00        C
HETATM13277  C6  RAM B 302   -5.431  14.182  31.594  1.00 33.31        C
HETATM13278  O2  RAM B 302   -6.582  11.964  27.965  1.00 33.63        O
HETATM13279  O3  RAM B 302   -5.784   9.806  29.429  1.00 34.04        O
HETATM13280  O4  RAM B 302   -5.015  11.227  31.868  1.00 35.17        O
HETATM13281  O5  RAM B 302   -4.963  13.810  29.287  1.00 30.71        O
HETATM13282  C1  RAM B 301   -7.022  12.392  26.658  1.00 35.77        C
HETATM13283  C2  RAM B 301   -8.463  11.872  26.459  1.00 37.82        C
HETATM13284  C3  RAM B 301   -9.460  12.658  27.302  1.00 37.88        C
HETATM13285  C4  RAM B 301   -9.293  14.137  26.997  1.00 38.72        C
HETATM13286  C5  RAM B 301   -7.852  14.543  27.294  1.00 38.09        C
HETATM13287  C6  RAM B 301   -7.667  16.036  27.074  1.00 38.45        C
HETATM13288  O2  RAM B 301   -8.890  11.923  25.101  1.00 37.89        O
HETATM13289  O3  RAM B 301  -10.773  12.308  26.941  1.00 39.28        O
HETATM13290  O4  RAM B 301  -10.254  14.898  27.715  1.00 39.43        O
HETATM13291  O5  RAM B 301   -6.968  13.801  26.468  1.00 36.68        O
HETATM13292  C1  GLC B 304   -1.791  11.454  26.034  1.00 17.07        C
HETATM13293  C2  GLC B 304   -0.594  10.743  25.395  1.00 17.39        C
HETATM13294  C3  GLC B 304   -0.500  11.137  23.922  1.00 19.13        C
HETATM13295  C4  GLC B 304   -1.839  10.858  23.227  1.00 18.00        C
HETATM13296  C5  GLC B 304   -3.007  11.479  24.012  1.00 19.77        C
HETATM13297  C6  GLC B 304   -4.368  11.061  23.452  1.00 19.41        C
HETATM13298  O2  GLC B 304    0.657  10.913  26.073  1.00 18.54        O
HETATM13299  O3  GLC B 304    0.568  10.441  23.311  1.00 18.74        O
HETATM13300  O4  GLC B 304   -1.795  11.294  21.867  1.00 18.88        O
HETATM13301  O5  GLC B 304   -2.961  11.060  25.368  1.00 19.52        O
HETATM13302  O6  GLC B 304   -4.487   9.658  23.524  1.00 18.54        O
HETATM13303  C1  GLC B 309   -8.116  23.185  25.058  1.00 20.75        C
HETATM13304  C2  GLC B 309   -7.448  21.822  25.073  1.00 20.61        C
HETATM13305  C3  GLC B 309   -6.073  21.913  25.730  1.00 19.24        C
HETATM13306  C4  GLC B 309   -5.230  22.979  25.081  1.00 19.69        C
HETATM13307  C5  GLC B 309   -5.995  24.315  24.939  1.00 19.98        C
HETATM13308  C6  GLC B 309   -5.316  25.267  23.974  1.00 21.34        C
HETATM13309  O2  GLC B 309   -8.248  20.984  25.878  1.00 20.86        O
HETATM13310  O3  GLC B 309   -5.415  20.665  25.745  1.00 19.58        O
HETATM13311  O4  GLC B 309   -4.075  23.141  25.878  1.00 18.05        O
HETATM13312  O5  GLC B 309   -7.305  24.158  24.414  1.00 21.66        O
HETATM13313  O6  GLC B 309   -6.095  26.448  23.897  1.00 22.29        O
```

```
HETATM13314  C1  EAG D 310    44.603  43.869  11.547  1.00 39.34           C
HETATM13315  O1  EAG D 310    44.324  43.550  12.899  1.00 40.67           O
HETATM13316  C2  EAG D 310    43.369  43.636  10.669  1.00 36.58           C
HETATM13317  N2  EAG D 310    42.952  42.250  10.729  1.00 36.80           N
HETATM13318  C7  EAG D 310    41.817  41.896  11.323  1.00 36.86           C
HETATM13319  O7  EAG D 310    41.033  42.710  11.805  1.00 37.86           O
HETATM13320  C8  EAG D 310    41.518  40.429  11.381  1.00 36.77           C
HETATM13321  C3  EAG D 310    43.601  44.075   9.221  1.00 35.91           C
HETATM13322  O3  EAG D 310    42.353  44.077   8.556  1.00 31.78           O
HETATM13323  C4  EAG D 310    44.151  45.499   9.228  1.00 36.92           C
HETATM13324  O4  EAG D 310    44.501  45.937   7.932  1.00 37.48           O
HETATM13325  C5  EAG D 310    45.363  45.601  10.154  1.00 39.59           C
HETATM13326  C6  EAG D 310    45.909  47.017  10.212  1.00 41.07           C
HETATM13327  O6  EAG D 310    45.113  47.779  11.095  1.00 44.80           O
HETATM13328  O5  EAG D 310    44.988  45.232  11.459  1.00 39.39           O
HETATM13329  C9  EAG D 310    45.363  43.529  13.884  1.00 40.92           C
HETATM13330  C1  RAM D 308    42.236  43.155   7.456  1.00 28.72           C
HETATM13331  C2  RAM D 308    40.760  42.853   7.276  1.00 26.92           C
HETATM13332  C3  RAM D 308    40.019  44.079   6.752  1.00 25.36           C
HETATM13333  C4  RAM D 308    40.696  44.643   5.516  1.00 26.30           C
HETATM13334  C5  RAM D 308    42.171  44.922   5.858  1.00 27.71           C
HETATM13335  C6  RAM D 308    42.978  45.466   4.699  1.00 27.32           C
HETATM13336  O2  RAM D 308    40.574  41.796   6.365  1.00 25.59           O
HETATM13337  O3  RAM D 308    38.678  43.732   6.441  1.00 22.76           O
HETATM13338  O4  RAM D 308    39.976  45.812   5.191  1.00 24.52           O
HETATM13339  O5  RAM D 308    42.752  43.680   6.245  1.00 29.07           O
HETATM13340  C1  RAM D 307    37.755  44.255   7.414  1.00 21.80           C
HETATM13341  C2  RAM D 307    36.335  44.123   6.871  1.00 19.24           C
HETATM13342  C3  RAM D 307    35.922  42.660   6.811  1.00 20.14           C
HETATM13343  C4  RAM D 307    36.123  42.006   8.177  1.00 20.56           C
HETATM13344  C5  RAM D 307    37.550  42.232   8.671  1.00 22.29           C
HETATM13345  C6  RAM D 307    37.783  41.681  10.076  1.00 22.47           C
HETATM13346  O2  RAM D 307    35.432  44.745   7.748  1.00 18.49           O
HETATM13347  O3  RAM D 307    34.561  42.589   6.444  1.00 20.80           O
HETATM13348  O4  RAM D 307    35.820  40.635   8.045  1.00 25.52           O
HETATM13349  O5  RAM D 307    37.841  43.628   8.686  1.00 21.65           O
HETATM13350  C1  RAM D 306    35.138  46.131   7.449  1.00 18.05           C
HETATM13351  C2  RAM D 306    33.828  46.600   8.090  1.00 17.02           C
HETATM13352  C3  RAM D 306    33.936  46.485   9.612  1.00 19.04           C
HETATM13353  C4  RAM D 306    35.105  47.308  10.110  1.00 21.11           C
HETATM13354  C5  RAM D 306    36.360  46.970   9.300  1.00 21.09           C
HETATM13355  C6  RAM D 306    37.526  47.895   9.619  1.00 21.73           C
HETATM13356  O2  RAM D 306    33.663  47.996   7.766  1.00 15.67           O
HETATM13357  O3  RAM D 306    32.729  46.846  10.228  1.00 19.88           O
HETATM13358  O4  RAM D 306    35.361  47.045  11.489  1.00 22.40           O
HETATM13359  O5  RAM D 306    36.128  47.007   7.900  1.00 20.05           O
HETATM13360  C1  NAG D 305    32.691  48.242   6.769  1.00 13.95           C
HETATM13361  C2  NAG D 305    32.503  49.765   6.816  1.00 13.96           C
HETATM13362  C3  NAG D 305    31.821  50.317   5.589  1.00 14.86           C
HETATM13363  C4  NAG D 305    32.315  49.653   4.326  1.00 13.44           C
HETATM13364  C5  NAG D 305    32.287  48.121   4.479  1.00 13.95           C
HETATM13365  C6  NAG D 305    32.727  47.398   3.209  1.00 13.88           C
HETATM13366  C7  NAG D 305    32.556  50.778   9.069  1.00 16.34           C
HETATM13367  C8  NAG D 305    31.795  51.100  10.317  1.00 17.20           C
HETATM13368  N2  NAG D 305    31.869  50.179   8.081  1.00 14.20           N
HETATM13369  O3  NAG D 305    32.161  51.702   5.446  1.00 14.47           O
HETATM13370  O4  NAG D 305    31.487  50.065   3.267  1.00 11.22           O
HETATM13371  O5  NAG D 305    33.168  47.777   5.522  1.00 14.56           O
HETATM13372  O6  NAG D 305    34.064  47.732   2.913  1.00 13.44           O
HETATM13373  O7  NAG D 305    33.748  51.100   9.014  1.00 16.79           O
HETATM13374  C1  RAM D 303    31.146  52.582   5.928  1.00 14.87           C
HETATM13375  C2  RAM D 303    31.837  53.791   6.551  1.00 15.42           C
HETATM13376  C3  RAM D 303    32.561  54.567   5.460  1.00 14.40           C
HETATM13377  C4  RAM D 303    31.622  54.950   4.319  1.00 13.26           C
HETATM13378  C5  RAM D 303    30.998  53.643   3.795  1.00 13.03           C
```

```
HETATM13379  C6   RAM D 303     30.015  53.800   2.648  1.00 13.41           C
HETATM13380  O2   RAM D 303     30.864  54.582   7.172  1.00 15.76           O
HETATM13381  O3   RAM D 303     33.171  55.740   6.004  1.00 18.19           O
HETATM13382  O4   RAM D 303     32.327  55.619   3.296  1.00 12.64           O
HETATM13383  O5   RAM D 303     30.310  53.025   4.879  1.00 13.80           O
HETATM13384  C1   RAM D 302     34.614  55.680   6.017  1.00 20.30           C
HETATM13385  C2   RAM D 302     35.167  57.104   6.167  1.00 22.94           C
HETATM13386  C3   RAM D 302     34.664  57.658   7.492  1.00 24.38           C
HETATM13387  C4   RAM D 302     35.038  56.719   8.633  1.00 26.40           C
HETATM13388  C5   RAM D 302     34.575  55.291   8.361  1.00 25.29           C
HETATM13389  C6   RAM D 302     35.067  54.357   9.466  1.00 25.65           C
HETATM13390  O2   RAM D 302     36.593  57.120   6.212  1.00 24.82           O
HETATM13391  O3   RAM D 302     35.213  58.945   7.709  1.00 22.81           O
HETATM13392  O4   RAM D 302     34.477  57.169   9.855  1.00 29.21           O
HETATM13393  O5   RAM D 302     35.083  54.878   7.095  1.00 21.49           O
HETATM13394  C1   RAM D 301     37.309  56.783   4.990  1.00 28.99           C
HETATM13395  C2   RAM D 301     38.668  57.480   4.995  1.00 30.33           C
HETATM13396  C3   RAM D 301     39.613  56.834   5.995  1.00 30.66           C
HETATM13397  C4   RAM D 301     39.717  55.336   5.741  1.00 30.66           C
HETATM13398  C5   RAM D 301     38.335  54.683   5.670  1.00 28.75           C
HETATM13399  C6   RAM D 301     38.416  53.217   5.216  1.00 28.99           C
HETATM13400  O2   RAM D 301     39.260  57.416   3.704  1.00 30.65           O
HETATM13401  O3   RAM D 301     40.899  57.382   5.825  1.00 33.41           O
HETATM13402  O4   RAM D 301     40.520  54.809   6.773  1.00 30.61           O
HETATM13403  O5   RAM D 301     37.538  55.399   4.750  1.00 27.89           O
HETATM13404  C1   GLC D 304     32.344  57.052   3.361  1.00 16.76           C
HETATM13405  C2   GLC D 304     31.211  57.619   2.532  1.00 15.42           C
HETATM13406  C3   GLC D 304     31.395  57.235   1.074  1.00 12.96           C
HETATM13407  C4   GLC D 304     32.775  57.651   0.609  1.00 12.70           C
HETATM13408  C5   GLC D 304     33.883  57.158   1.542  1.00 14.46           C
HETATM13409  C6   GLC D 304     35.251  57.739   1.219  1.00 16.17           C
HETATM13410  O2   GLC D 304     29.944  57.247   3.033  1.00 16.50           O
HETATM13411  O3   GLC D 304     30.404  57.777   0.218  1.00 14.52           O
HETATM13412  O4   GLC D 304     32.947  57.257  -0.752  1.00 13.24           O
HETATM13413  O5   GLC D 304     33.597  57.472   2.891  1.00 15.25           O
HETATM13414  O6   GLC D 304     35.162  59.157   1.299  1.00 15.56           O
HETATM13415  C1   GLC D 309     39.902  46.144   3.797  1.00 23.23           C
HETATM13416  C2   GLC D 309     39.062  47.412   3.659  1.00 23.14           C
HETATM13417  C3   GLC D 309     37.604  47.140   4.034  1.00 21.69           C
HETATM13418  C4   GLC D 309     37.051  45.976   3.231  1.00 21.27           C
HETATM13419  C5   GLC D 309     37.998  44.780   3.272  1.00 22.42           C
HETATM13420  C6   GLC D 309     37.699  43.775   2.179  1.00 21.55           C
HETATM13421  O2   GLC D 309     39.605  48.434   4.483  1.00 21.49           O
HETATM13422  O3   GLC D 309     36.796  48.289   3.823  1.00 21.03           O
HETATM13423  O4   GLC D 309     35.783  45.627   3.739  1.00 19.95           O
HETATM13424  O5   GLC D 309     39.343  45.105   3.023  1.00 20.38           O
HETATM13425  O6   GLC D 309     38.505  42.660   2.460  1.00 24.79           O
HETATM13426  PD   PD  E 215      2.964  32.488  27.722  1.00 25.65          PD
HETATM13427  C1   EAG F 310    -15.841  40.281  17.103  1.00 41.18           C
HETATM13428  O1   EAG F 310    -15.464  40.077  15.753  1.00 42.93           O
HETATM13429  C2   EAG F 310    -14.603  40.167  17.998  1.00 39.89           C
HETATM13430  N2   EAG F 310    -14.124  38.799  17.998  1.00 40.17           N
HETATM13431  C7   EAG F 310    -12.890  38.496  17.606  1.00 41.36           C
HETATM13432  O7   EAG F 310    -12.140  39.307  17.071  1.00 43.27           O
HETATM13433  C8   EAG F 310    -12.442  37.087  17.847  1.00 41.28           C
HETATM13434  C3   EAG F 310    -14.850  40.618  19.436  1.00 38.99           C
HETATM13435  O3   EAG F 310    -13.604  40.818  20.098  1.00 35.74           O
HETATM13436  C4   EAG F 310    -15.665  41.900  19.493  1.00 40.08           C
HETATM13437  O4   EAG F 310    -16.146  42.055  20.807  1.00 41.28           O
HETATM13438  C5   EAG F 310    -16.844  41.859  18.519  1.00 41.34           C
HETATM13439  C6   EAG F 310    -17.567  43.200  18.484  1.00 41.86           C
HETATM13440  O6   EAG F 310    -16.765  44.158  17.824  1.00 42.91           O
HETATM13441  O5   EAG F 310    -16.384  41.576  17.218  1.00 41.47           O
HETATM13442  C9   EAG F 310    -16.313  39.419  14.813  1.00 44.01           C
HETATM13443  C1   RAM F 308    -13.421  39.921  21.204  1.00 33.15           C
```

```
HETATM13444  C2   RAM F 308   -11.922  39.768  21.431  1.00 31.48           C
HETATM13445  C3   RAM F 308   -11.308  41.045  21.998  1.00 30.10           C
HETATM13446  C4   RAM F 308   -12.085  41.573  23.197  1.00 29.34           C
HETATM13447  C5   RAM F 308   -13.557  41.674  22.762  1.00 30.65           C
HETATM13448  C6   RAM F 308   -14.476  42.254  23.831  1.00 30.53           C
HETATM13449  O2   RAM F 308   -11.725  38.711  22.334  1.00 33.49           O
HETATM13450  O3   RAM F 308    -9.939  40.860  22.324  1.00 28.62           O
HETATM13451  O4   RAM F 308   -11.530  42.844  23.455  1.00 26.20           O
HETATM13452  O5   RAM F 308   -14.024  40.386  22.399  1.00 32.54           O
HETATM13453  C1   RAM F 307    -9.096  41.492  21.329  1.00 26.93           C
HETATM13454  C2   RAM F 307    -7.654  41.484  21.827  1.00 24.36           C
HETATM13455  C3   RAM F 307    -7.118  40.066  21.909  1.00 25.45           C
HETATM13456  C4   RAM F 307    -7.278  39.405  20.541  1.00 26.72           C
HETATM13457  C5   RAM F 307    -8.736  39.511  20.089  1.00 25.88           C
HETATM13458  C6   RAM F 307    -8.956  38.893  18.711  1.00 26.83           C
HETATM13459  O2   RAM F 307    -6.801  42.162  20.930  1.00 26.22           O
HETATM13460  O3   RAM F 307    -5.759  40.125  22.321  1.00 27.16           O
HETATM13461  O4   RAM F 307    -6.927  38.044  20.627  1.00 26.59           O
HETATM13462  O5   RAM F 307    -9.148  40.864  20.061  1.00 24.11           O
HETATM13463  C1   RAM F 306    -6.687  43.558  21.186  1.00 24.14           C
HETATM13464  C2   RAM F 306    -5.457  44.119  20.494  1.00 22.95           C
HETATM13465  C3   RAM F 306    -5.592  43.931  18.978  1.00 24.18           C
HETATM13466  C4   RAM F 306    -6.862  44.635  18.502  1.00 27.61           C
HETATM13467  C5   RAM F 306    -8.057  44.196  19.343  1.00 27.80           C
HETATM13468  C6   RAM F 306    -9.296  45.025  19.032  1.00 28.68           C
HETATM13469  O2   RAM F 306    -5.486  45.501  20.750  1.00 19.06           O
HETATM13470  O3   RAM F 306    -4.483  44.475  18.295  1.00 24.28           O
HETATM13471  O4   RAM F 306    -7.104  44.323  17.146  1.00 28.91           O
HETATM13472  O5   RAM F 306    -7.793  44.317  20.734  1.00 27.23           O
HETATM13473  C1   NAG F 305    -4.546  45.975  21.725  1.00 19.60           C
HETATM13474  C2   NAG F 305    -4.600  47.484  21.681  1.00 18.36           C
HETATM13475  C3   NAG F 305    -3.947  48.162  22.880  1.00 19.58           C
HETATM13476  C4   NAG F 305    -4.367  47.504  24.185  1.00 18.02           C
HETATM13477  C5   NAG F 305    -4.136  46.011  24.078  1.00 19.60           C
HETATM13478  C6   NAG F 305    -4.526  45.246  25.341  1.00 20.64           C
HETATM13479  C7   NAG F 305    -4.735  48.465  19.434  1.00 21.06           C
HETATM13480  C8   NAG F 305    -4.005  48.806  18.170  1.00 21.00           C
HETATM13481  N2   NAG F 305    -3.998  48.016  20.465  1.00 20.66           N
HETATM13482  O3   NAG F 305    -4.358  49.523  22.899  1.00 18.50           O
HETATM13483  O4   NAG F 305    -3.594  48.062  25.236  1.00 18.52           O
HETATM13484  O5   NAG F 305    -4.912  45.533  23.004  1.00 18.84           O
HETATM13485  O6   NAG F 305    -5.878  45.473  25.709  1.00 19.62           O
HETATM13486  O7   NAG F 305    -5.951  48.600  19.484  1.00 22.76           O
HETATM13487  C1   RAM F 303    -3.399  50.508  22.526  1.00 18.92           C
HETATM13488  C2   RAM F 303    -4.159  51.690  21.914  1.00 19.91           C
HETATM13489  C3   RAM F 303    -5.004  52.403  22.975  1.00 20.42           C
HETATM13490  C4   RAM F 303    -4.108  52.820  24.131  1.00 19.41           C
HETATM13491  C5   RAM F 303    -3.376  51.580  24.664  1.00 18.49           C
HETATM13492  C6   RAM F 303    -2.447  51.916  25.824  1.00 16.66           C
HETATM13493  O2   RAM F 303    -3.240  52.624  21.394  1.00 18.92           O
HETATM13494  O3   RAM F 303    -5.629  53.572  22.435  1.00 23.63           O
HETATM13495  O4   RAM F 303    -4.865  53.408  25.182  1.00 20.90           O
HETATM13496  O5   RAM F 303    -2.615  50.975  23.615  1.00 17.78           O
HETATM13497  C1   RAM F 302    -7.062  53.388  22.298  1.00 27.54           C
HETATM13498  C2   RAM F 302    -7.746  54.751  22.137  1.00 29.41           C
HETATM13499  C3   RAM F 302    -7.224  55.397  20.861  1.00 30.65           C
HETATM13500  C4   RAM F 302    -7.428  54.461  19.677  1.00 30.78           C
HETATM13501  C5   RAM F 302    -6.842  53.084  19.979  1.00 28.72           C
HETATM13502  C6   RAM F 302    -7.110  52.122  18.827  1.00 28.46           C
HETATM13503  O2   RAM F 302    -9.166  54.630  21.990  1.00 32.42           O
HETATM13504  O3   RAM F 302    -7.931  56.595  20.650  1.00 32.75           O
HETATM13505  O4   RAM F 302    -6.826  54.959  18.492  1.00 30.75           O
HETATM13506  O5   RAM F 302    -7.381  52.579  21.193  1.00 27.28           O
HETATM13507  C1   RAM F 301    -9.908  54.298  23.185  1.00 34.80           C
HETATM13508  C2   RAM F 301   -11.355  54.804  23.118  1.00 36.69           C
```

```
HETATM13509  C3  RAM F 301    -12.252  53.953  22.226  1.00 36.87        C
HETATM13510  C4  RAM F 301    -12.082  52.470  22.497  1.00 37.06        C
HETATM13511  C5  RAM F 301    -10.605  52.114  22.491  1.00 35.72        C
HETATM13512  C6  RAM F 301    -10.407  50.643  22.853  1.00 35.80        C
HETATM13513  O2  RAM F 301    -11.918  54.806  24.427  1.00 38.36        O
HETATM13514  O3  RAM F 301    -13.607  54.269  22.455  1.00 38.91        O
HETATM13515  O4  RAM F 301    -12.787  51.760  21.499  1.00 37.37        O
HETATM13516  O5  RAM F 301     -9.940  52.909  23.445  1.00 34.36        O
HETATM13517  C1  GLC F 304     -4.990  54.835  25.049  1.00 21.49        C
HETATM13518  C2  GLC F 304     -3.937  55.576  25.869  1.00 22.19        C
HETATM13519  C3  GLC F 304     -4.098  55.279  27.361  1.00 20.99        C
HETATM13520  C4  GLC F 304     -5.533  55.646  27.761  1.00 20.34        C
HETATM13521  C5  GLC F 304     -6.558  54.967  26.843  1.00 22.19        C
HETATM13522  C6  GLC F 304     -7.979  55.433  27.138  1.00 22.05        C
HETATM13523  O2  GLC F 304     -2.667  55.212  25.409  1.00 22.35        O
HETATM13524  O3  GLC F 304     -3.111  55.926  28.142  1.00 18.13        O
HETATM13525  O4  GLC F 304     -5.752  55.228  29.094  1.00 22.27        O
HETATM13526  O5  GLC F 304     -6.274  55.219  25.471  1.00 22.42        O
HETATM13527  O6  GLC F 304     -8.048  56.826  26.874  1.00 22.57        O
HETATM13528  C1  GLC F 309    -11.502  43.248  24.844  1.00 26.57        C
HETATM13529  C2  GLC F 309    -10.819  44.603  24.896  1.00 25.47        C
HETATM13530  C3  GLC F 309     -9.326  44.478  24.585  1.00 24.97        C
HETATM13531  C4  GLC F 309     -8.644  43.431  25.446  1.00 24.25        C
HETATM13532  C5  GLC F 309     -9.471  42.143  25.462  1.00 26.52        C
HETATM13533  C6  GLC F 309     -9.009  41.201  26.546  1.00 27.34        C
HETATM13534  O2  GLC F 309    -11.431  45.436  23.933  1.00 25.76        O
HETATM13535  O3  GLC F 309     -8.623  45.669  24.807  1.00 21.86        O
HETATM13536  O4  GLC F 309     -7.353  43.254  24.883  1.00 24.31        O
HETATM13537  O5  GLC F 309    -10.849  42.370  25.729  1.00 26.59        O
HETATM13538  O6  GLC F 309     -9.713  39.998  26.307  1.00 30.54        O
HETATM13539  C1  EAG H 310     54.657  20.297 -60.745  1.00 45.53        C
HETATM13540  O1  EAG H 310     54.586  20.657 -62.105  1.00 47.58        O
HETATM13541  C2  EAG H 310     53.309  20.619 -60.105  1.00 44.12        C
HETATM13542  N2  EAG H 310     53.060  22.046 -60.126  1.00 44.30        N
HETATM13543  C7  EAG H 310     52.144  22.538 -60.949  1.00 45.18        C
HETATM13544  O7  EAG H 310     51.528  21.832 -61.754  1.00 46.04        O
HETATM13545  C8  EAG H 310     51.875  24.011 -60.867  1.00 45.23        C
HETATM13546  C3  EAG H 310     53.150  20.069 -58.698  1.00 42.40        C
HETATM13547  O3  EAG H 310     51.788  20.183 -58.338  1.00 38.78        O
HETATM13548  C4  EAG H 310     53.511  18.598 -58.710  1.00 44.06        C
HETATM13549  O4  EAG H 310     53.490  18.153 -57.378  1.00 45.11        O
HETATM13550  C5  EAG H 310     54.896  18.402 -59.343  1.00 45.44        C
HETATM13551  C6  EAG H 310     55.308  16.932 -59.364  1.00 45.73        C
HETATM13552  O6  EAG H 310     54.249  16.136 -59.854  1.00 46.92        O
HETATM13553  O5  EAG H 310     54.911  18.910 -60.668  1.00 45.83        O
HETATM13554  C9  EAG H 310     55.685  21.289 -62.762  1.00 48.49        C
HETATM13555  C1  RAM H 308     51.543  21.132 -57.285  1.00 34.46        C
HETATM13556  C2  RAM H 308     50.097  21.615 -57.388  1.00 31.93        C
HETATM13557  C3  RAM H 308     49.119  20.524 -56.958  1.00 29.79        C
HETATM13558  C4  RAM H 308     49.480  19.880 -55.620  1.00 29.31        C
HETATM13559  C5  RAM H 308     50.947  19.435 -55.707  1.00 31.14        C
HETATM13560  C6  RAM H 308     51.459  18.785 -54.428  1.00 30.60        C
HETATM13561  O2  RAM H 308     49.903  22.762 -56.604  1.00 32.26        O
HETATM13562  O3  RAM H 308     47.789  21.024 -56.970  1.00 27.50        O
HETATM13563  O4  RAM H 308     48.626  18.766 -55.473  1.00 27.14        O
HETATM13564  O5  RAM H 308     51.763  20.563 -56.019  1.00 33.65        O
HETATM13565  C1  RAM H 307     47.049  20.453 -58.061  1.00 25.96        C
HETATM13566  C2  RAM H 307     45.550  20.697 -57.849  1.00 24.13        C
HETATM13567  C3  RAM H 307     45.232  22.173 -57.914  1.00 24.57        C
HETATM13568  C4  RAM H 307     45.754  22.763 -59.221  1.00 25.99        C
HETATM13569  C5  RAM H 307     47.240  22.410 -59.402  1.00 26.93        C
HETATM13570  C6  RAM H 307     47.792  22.933 -60.735  1.00 26.46        C
HETATM13571  O2  RAM H 307     44.810  20.068 -58.873  1.00 23.64        O
HETATM13572  O3  RAM H 307     43.846  22.396 -57.798  1.00 22.36        O
HETATM13573  O4  RAM H 307     45.577  24.168 -59.166  1.00 28.95        O
```

| | | | | | | | | | |
|---|---|---|---|---|---|---|---|---|---|
| HETATM13574 | O5 | RAM H | 307 | 47.459 | 21.002 | -59.305 | 1.00 | 27.29 | O |
| HETATM13575 | C1 | RAM H | 306 | 44.352 | 18.748 | -58.561 | 1.00 | 23.53 | C |
| HETATM13576 | C2 | RAM H | 306 | 43.129 | 18.422 | -59.410 | 1.00 | 21.64 | C |
| HETATM13577 | C3 | RAM H | 306 | 43.541 | 18.489 | -60.885 | 1.00 | 24.92 | C |
| HETATM13578 | C4 | RAM H | 306 | 44.703 | 17.515 | -61.108 | 1.00 | 25.37 | C |
| HETATM13579 | C5 | RAM H | 306 | 45.841 | 17.782 | -60.116 | 1.00 | 26.81 | C |
| HETATM13580 | C6 | RAM H | 306 | 46.949 | 16.737 | -60.229 | 1.00 | 26.39 | C |
| HETATM13581 | O2 | RAM H | 306 | 42.741 | 17.085 | -59.163 | 1.00 | 21.01 | O |
| HETATM13582 | O3 | RAM H | 306 | 42.434 | 18.120 | -61.681 | 1.00 | 24.81 | O |
| HETATM13583 | O4 | RAM H | 306 | 45.169 | 17.600 | -62.435 | 1.00 | 26.93 | O |
| HETATM13584 | O5 | RAM H | 306 | 45.354 | 17.783 | -58.774 | 1.00 | 26.55 | O |
| HETATM13585 | C1 | NAG H | 305 | 41.608 | 16.892 | -58.328 | 1.00 | 17.78 | C |
| HETATM13586 | C2 | NAG H | 305 | 41.431 | 15.387 | -58.383 | 1.00 | 18.32 | C |
| HETATM13587 | C3 | NAG H | 305 | 40.461 | 14.890 | -57.323 | 1.00 | 16.88 | C |
| HETATM13588 | C4 | NAG H | 305 | 40.721 | 15.556 | -55.971 | 1.00 | 17.29 | C |
| HETATM13589 | C5 | NAG H | 305 | 40.817 | 17.064 | -56.120 | 1.00 | 17.25 | C |
| HETATM13590 | C6 | NAG H | 305 | 41.132 | 17.786 | -54.807 | 1.00 | 16.64 | C |
| HETATM13591 | C7 | NAG H | 305 | 41.716 | 14.295 | -60.591 | 1.00 | 21.24 | C |
| HETATM13592 | C8 | NAG H | 305 | 41.081 | 14.036 | -61.930 | 1.00 | 20.62 | C |
| HETATM13593 | N2 | NAG H | 305 | 40.976 | 15.003 | -59.726 | 1.00 | 19.89 | N |
| HETATM13594 | O3 | NAG H | 305 | 40.633 | 13.494 | -57.181 | 1.00 | 18.41 | O |
| HETATM13595 | O4 | NAG H | 305 | 39.690 | 15.220 | -55.060 | 1.00 | 17.68 | O |
| HETATM13596 | O5 | NAG H | 305 | 41.857 | 17.395 | -57.028 | 1.00 | 17.83 | O |
| HETATM13597 | O6 | NAG H | 305 | 42.330 | 17.277 | -54.262 | 1.00 | 15.99 | O |
| HETATM13598 | O7 | NAG H | 305 | 42.854 | 13.878 | -60.355 | 1.00 | 20.02 | O |
| HETATM13599 | C1 | RAM H | 303 | 39.640 | 12.646 | -57.768 | 1.00 | 15.92 | C |
| HETATM13600 | C2 | RAM H | 303 | 40.331 | 11.339 | -58.202 | 1.00 | 17.67 | C |
| HETATM13601 | C3 | RAM H | 303 | 40.803 | 10.600 | -56.973 | 1.00 | 18.16 | C |
| HETATM13602 | C4 | RAM H | 303 | 39.618 | 10.364 | -56.050 | 1.00 | 15.64 | C |
| HETATM13603 | C5 | RAM H | 303 | 39.019 | 11.737 | -55.675 | 1.00 | 17.77 | C |
| HETATM13604 | C6 | RAM H | 303 | 37.818 | 11.627 | -54.728 | 1.00 | 18.48 | C |
| HETATM13605 | O2 | RAM H | 303 | 39.460 | 10.511 | -58.962 | 1.00 | 16.69 | O |
| HETATM13606 | O3 | RAM H | 303 | 41.427 | 9.366 | -57.330 | 1.00 | 20.75 | O |
| HETATM13607 | O4 | RAM H | 303 | 40.092 | 9.697 | -54.912 | 1.00 | 16.74 | O |
| HETATM13608 | O5 | RAM H | 303 | 38.608 | 12.377 | -56.865 | 1.00 | 17.58 | O |
| HETATM13609 | C1 | RAM H | 302 | 42.866 | 9.432 | -57.222 | 1.00 | 25.12 | C |
| HETATM13610 | C2 | RAM H | 302 | 43.368 | 7.992 | -57.135 | 1.00 | 27.48 | C |
| HETATM13611 | C3 | RAM H | 302 | 42.984 | 7.268 | -58.422 | 1.00 | 28.63 | C |
| HETATM13612 | C4 | RAM H | 302 | 43.649 | 7.998 | -59.586 | 1.00 | 28.96 | C |
| HETATM13613 | C5 | RAM H | 302 | 43.233 | 9.479 | -59.581 | 1.00 | 27.97 | C |
| HETATM13614 | C6 | RAM H | 302 | 43.931 | 10.271 | -60.682 | 1.00 | 27.82 | C |
| HETATM13615 | O2 | RAM H | 302 | 44.785 | 7.952 | -56.990 | 1.00 | 29.83 | O |
| HETATM13616 | O3 | RAM H | 302 | 43.417 | 5.922 | -58.363 | 1.00 | 29.25 | O |
| HETATM13617 | O4 | RAM H | 302 | 43.293 | 7.338 | -60.790 | 1.00 | 29.73 | O |
| HETATM13618 | O5 | RAM H | 302 | 43.478 | 10.107 | -58.302 | 1.00 | 25.38 | O |
| HETATM13619 | C1 | RAM H | 301 | 45.281 | 8.206 | -55.659 | 1.00 | 32.17 | C |
| HETATM13620 | C2 | RAM H | 301 | 46.578 | 7.412 | -55.465 | 1.00 | 34.14 | C |
| HETATM13621 | C3 | RAM H | 301 | 47.713 | 8.006 | -56.282 | 1.00 | 35.43 | C |
| HETATM13622 | C4 | RAM H | 301 | 47.808 | 9.512 | -56.065 | 1.00 | 35.66 | C |
| HETATM13623 | C5 | RAM H | 301 | 46.456 | 10.191 | -56.278 | 1.00 | 33.86 | C |
| HETATM13624 | C6 | RAM H | 301 | 46.526 | 11.673 | -55.913 | 1.00 | 33.99 | C |
| HETATM13625 | O2 | RAM H | 301 | 46.969 | 7.341 | -54.107 | 1.00 | 34.53 | O |
| HETATM13626 | O3 | RAM H | 301 | 48.923 | 7.427 | -55.844 | 1.00 | 38.04 | O |
| HETATM13627 | O4 | RAM H | 301 | 48.815 | 10.051 | -56.906 | 1.00 | 36.84 | O |
| HETATM13628 | O5 | RAM H | 301 | 45.492 | 9.590 | -55.441 | 1.00 | 32.41 | O |
| HETATM13629 | C1 | GLC H | 304 | 39.944 | 8.258 | -54.900 | 1.00 | 16.94 | C |
| HETATM13630 | C2 | GLC H | 304 | 38.624 | 7.781 | -54.295 | 1.00 | 17.67 | C |
| HETATM13631 | C3 | GLC H | 304 | 38.584 | 8.139 | -52.810 | 1.00 | 17.04 | C |
| HETATM13632 | C4 | GLC H | 304 | 39.833 | 7.557 | -52.134 | 1.00 | 18.26 | C |
| HETATM13633 | C5 | GLC H | 304 | 41.139 | 7.979 | -52.833 | 1.00 | 18.55 | C |
| HETATM13634 | C6 | GLC H | 304 | 42.374 | 7.244 | -52.313 | 1.00 | 19.55 | C |
| HETATM13635 | O2 | GLC H | 304 | 37.489 | 8.245 | -55.027 | 1.00 | 17.34 | O |
| HETATM13636 | O3 | GLC H | 304 | 37.388 | 7.730 | -52.161 | 1.00 | 16.16 | O |
| HETATM13637 | O4 | GLC H | 304 | 39.874 | 7.944 | -50.786 | 1.00 | 20.24 | O |
| HETATM13638 | O5 | GLC H | 304 | 41.068 | 7.725 | -54.227 | 1.00 | 18.62 | O |

```
HETATM13639  O6  GLC H 304    42.157    5.859 -52.545  1.00 21.43         O
HETATM13640  C1  GLC H 309    48.234   18.465 -54.120  1.00 24.73         C
HETATM13641  C2  GLC H 309    47.275   17.284 -54.132  1.00 24.69         C
HETATM13642  C3  GLC H 309    45.970   17.682 -54.815  1.00 22.47         C
HETATM13643  C4  GLC H 309    45.388   18.910 -54.117  1.00 22.51         C
HETATM13644  C5  GLC H 309    46.420   20.037 -54.011  1.00 24.57         C
HETATM13645  C6  GLC H 309    45.970   21.106 -53.040  1.00 23.80         C
HETATM13646  O2  GLC H 309    47.886   16.226 -54.837  1.00 25.89         O
HETATM13647  O3  GLC H 309    45.078   16.596 -54.730  1.00 21.44         O
HETATM13648  O4  GLC H 309    44.224   19.352 -54.796  1.00 22.37         O
HETATM13649  O5  GLC H 309    47.639   19.559 -53.473  1.00 25.31         O
HETATM13650  O6  GLC H 309    46.886   22.189 -53.086  1.00 24.17         O
HETATM13651  O   HOH A 215    20.714   15.559  36.681  1.00 16.38         O
HETATM13652  O   HOH A 216    34.651   16.032 -12.209  1.00 11.51         O
HETATM13653  O   HOH A 217     0.940   19.032  31.591  1.00 16.69         O
HETATM13654  O   HOH A 218     6.382   31.766  30.174  1.00 12.40         O
HETATM13655  O   HOH A 219     7.576   25.716  18.779  1.00 12.64         O
HETATM13656  O   HOH A 220    48.812   17.455   9.971  1.00 17.10         O
HETATM13657  O   HOH A 221    41.272   11.528   2.015  1.00 16.60         O
HETATM13658  O   HOH A 222    18.387   10.918  16.594  1.00 18.64         O
HETATM13659  O   HOH A 223    42.161   25.884 -22.745  1.00 17.59         O
HETATM13660  O   HOH A 224     4.645   27.590  24.999  1.00 20.88         O
HETATM13661  O   HOH A 225    20.653   26.451  22.063  1.00 18.69         O
HETATM13662  O   HOH A 226     8.027   14.455  37.176  1.00 12.89         O
HETATM13663  O   HOH A 227    19.229   18.518  40.421  1.00 18.69         O
HETATM13664  O   HOH A 228    13.901   30.414  29.525  1.00 17.20         O
HETATM13665  O   HOH A 229    17.404   27.781  23.902  1.00 16.38         O
HETATM13666  O   HOH A 230    12.691   14.071  39.369  1.00 16.81         O
HETATM13667  O   HOH A 231    31.624   10.777  18.363  1.00 17.61         O
HETATM13668  O   HOH A 232    43.428   10.593  -4.693  1.00 20.69         O
HETATM13669  O   HOH A 233    57.392   18.928  10.219  1.00 17.47         O
HETATM13670  O   HOH A 234    36.743   16.143  -9.783  1.00 21.50         O
HETATM13671  O   HOH A 235     3.966   12.368  27.805  1.00 21.35         O
HETATM13672  O   HOH A 236    29.473   23.579 -18.471  1.00 17.12         O
HETATM13673  O   HOH A 237     2.701   20.297  42.437  1.00 21.39         O
HETATM13674  O   HOH A 238     8.267   16.673  39.727  1.00 19.09         O
HETATM13675  O   HOH A 239    47.314   24.872   4.217  1.00 20.31         O
HETATM13676  O   HOH A 240    37.392   29.389 -14.622  1.00 19.50         O
HETATM13677  O   HOH A 241    23.662    9.733  34.381  1.00 20.70         O
HETATM13678  O   HOH A 242    35.031   12.216   9.997  1.00 21.22         O
HETATM13679  O   HOH A 243    33.164   27.408  -6.878  1.00 16.84         O
HETATM13680  O   HOH A 244    44.221   22.247  13.967  1.00 21.47         O
HETATM13681  O   HOH A 245     3.762   21.401  24.884  1.00 18.61         O
HETATM13682  O   HOH A 246    43.861   28.660  -1.542  1.00 19.81         O
HETATM13683  O   HOH A 247    53.030   16.075   2.524  1.00 22.95         O
HETATM13684  O   HOH A 248    16.740   10.304  35.491  1.00 21.00         O
HETATM13685  O   HOH A 249    14.683   29.684  23.049  1.00 24.13         O
HETATM13686  O   HOH A 250    43.051   10.690   9.138  1.00 21.34         O
HETATM13687  O   HOH A 251    33.962   25.002  -6.339  1.00 15.23         O
HETATM13688  O   HOH A 252    34.029   28.373  -9.345  1.00 21.69         O
HETATM13689  O   HOH A 253    37.472   30.123 -18.980  1.00 20.20         O
HETATM13690  O   HOH A 254     0.756   27.337  31.002  1.00 26.64         O
HETATM13691  O   HOH A 255    34.048   14.845 -24.938  1.00 20.11         O
HETATM13692  O   HOH A 256    47.328   12.132   3.784  1.00 17.41         O
HETATM13693  O   HOH A 257    11.875   28.101  23.494  1.00 15.09         O
HETATM13694  O   HOH A 258    12.880    6.974  22.157  1.00 29.12         O
HETATM13695  O   HOH A 259    14.911   24.729  18.479  1.00 21.90         O
HETATM13696  O   HOH A 260    47.645   11.683  13.519  1.00 18.16         O
HETATM13697  O   HOH A 261    -0.890   23.144  37.181  1.00 28.26         O
HETATM13698  O   HOH A 262    25.571   10.025  17.340  1.00 20.62         O
HETATM13699  O   HOH A 263    39.518   31.638 -22.996  1.00 23.68         O
HETATM13700  O   HOH A 264    50.533   23.364  12.230  1.00 28.37         O
HETATM13701  O   HOH A 265    42.931   16.117  21.628  1.00 29.97         O
HETATM13702  O   HOH A 266    40.444   23.809 -23.364  1.00 14.40         O
HETATM13703  O   HOH A 267    36.462   14.245 -21.427  1.00 19.43         O
```

| | | | | | | | | | | |
|---|---|---|---|---|---|---|---|---|---|---|
| HETATM13704 | O | HOH | A | 268 | 20.931 | 12.891 | 37.275 | 1.00 | 22.10 | O |
| HETATM13705 | O | HOH | A | 269 | 50.499 | 13.914 | -4.269 | 1.00 | 25.24 | O |
| HETATM13706 | O | HOH | A | 270 | 25.309 | 17.169 | 36.345 | 1.00 | 17.54 | O |
| HETATM13707 | O | HOH | A | 271 | 32.680 | 13.550 | 10.341 | 1.00 | 21.73 | O |
| HETATM13708 | O | HOH | A | 272 | 6.078 | 16.923 | 42.971 | 1.00 | 19.52 | O |
| HETATM13709 | O | HOH | A | 273 | 45.654 | 11.823 | -3.447 | 1.00 | 18.43 | O |
| HETATM13710 | O | HOH | A | 274 | 50.809 | 15.704 | 9.219 | 1.00 | 22.32 | O |
| HETATM13711 | O | HOH | A | 275 | 54.983 | 19.704 | 3.646 | 1.00 | 22.01 | O |
| HETATM13712 | O | HOH | A | 276 | 35.743 | 15.716 | 30.064 | 1.00 | 28.70 | O |
| HETATM13713 | O | HOH | A | 277 | 19.250 | 11.618 | 38.998 | 1.00 | 26.95 | O |
| HETATM13714 | O | HOH | A | 278 | 16.440 | 12.096 | 38.679 | 1.00 | 24.68 | O |
| HETATM13715 | O | HOH | A | 279 | 19.272 | 20.482 | 38.193 | 1.00 | 23.05 | O |
| HETATM13716 | O | HOH | A | 280 | 34.031 | 27.939 | 1.367 | 1.00 | 28.88 | O |
| HETATM13717 | O | HOH | A | 281 | 21.208 | 23.885 | 18.201 | 1.00 | 30.00 | O |
| HETATM13718 | O | HOH | A | 282 | 48.108 | 11.991 | -4.732 | 1.00 | 27.63 | O |
| HETATM13719 | O | HOH | A | 283 | 19.365 | 13.635 | 41.013 | 1.00 | 21.80 | O |
| HETATM13720 | O | HOH | A | 284 | 35.383 | 17.226 | -24.800 | 1.00 | 23.10 | O |
| HETATM13721 | O | HOH | A | 285 | 33.441 | 11.710 | 34.925 | 1.00 | 32.50 | O |
| HETATM13722 | O | HOH | A | 286 | 32.647 | 12.162 | -21.606 | 1.00 | 23.39 | O |
| HETATM13723 | O | HOH | A | 287 | 13.248 | 32.748 | 30.648 | 1.00 | 26.36 | O |
| HETATM13724 | O | HOH | A | 288 | 6.081 | 13.499 | 40.960 | 1.00 | 27.83 | O |
| HETATM13725 | O | HOH | A | 289 | 12.879 | 9.443 | 15.935 | 1.00 | 25.28 | O |
| HETATM13726 | O | HOH | A | 290 | 29.668 | 4.747 | 20.622 | 1.00 | 23.74 | O |
| HETATM13727 | O | HOH | A | 291 | 41.775 | 9.439 | 12.091 | 1.00 | 26.48 | O |
| HETATM13728 | O | HOH | A | 292 | 13.335 | 5.712 | 34.184 | 1.00 | 24.05 | O |
| HETATM13729 | O | HOH | A | 293 | 46.248 | 27.042 | 4.711 | 1.00 | 30.60 | O |
| HETATM13730 | O | HOH | A | 294 | 23.088 | 16.460 | 15.215 | 1.00 | 30.59 | O |
| HETATM13731 | O | HOH | A | 295 | 35.418 | 7.731 | 19.285 | 1.00 | 31.33 | O |
| HETATM13732 | O | HOH | A | 296 | 28.122 | 15.906 | 8.628 | 1.00 | 27.17 | O |
| HETATM13733 | O | HOH | A | 297 | 41.022 | 27.108 | 4.841 | 1.00 | 36.39 | O |
| HETATM13734 | O | HOH | A | 298 | 49.873 | 20.000 | 9.309 | 1.00 | 25.47 | O |
| HETATM13735 | O | HOH | A | 299 | 52.716 | 17.156 | 7.518 | 1.00 | 23.87 | O |
| HETATM13736 | O | HOH | A | 300 | 33.948 | 21.087 | -1.308 | 1.00 | 23.62 | O |
| HETATM13737 | O | HOH | A | 301 | 5.768 | 29.700 | 36.946 | 1.00 | 23.55 | O |
| HETATM13738 | O | HOH | A | 302 | 3.981 | 33.319 | 20.029 | 1.00 | 35.79 | O |
| HETATM13739 | O | HOH | A | 303 | 22.918 | 7.239 | 35.539 | 1.00 | 34.07 | O |
| HETATM13740 | O | HOH | A | 304 | 29.679 | 21.533 | -16.400 | 1.00 | 28.95 | O |
| HETATM13741 | O | HOH | A | 305 | 39.320 | 18.064 | -25.270 | 1.00 | 30.35 | O |
| HETATM13742 | O | HOH | A | 306 | 50.379 | 22.241 | 17.350 | 1.00 | 42.74 | O |
| HETATM13743 | O | HOH | A | 307 | 11.192 | 30.864 | 35.213 | 1.00 | 24.31 | O |
| HETATM13744 | O | HOH | A | 308 | 29.605 | 22.847 | 5.422 | 1.00 | 26.04 | O |
| HETATM13745 | O | HOH | A | 309 | -2.820 | 27.733 | 24.760 | 1.00 | 27.76 | O |
| HETATM13746 | O | HOH | A | 310 | 33.202 | 7.063 | -15.421 | 1.00 | 29.26 | O |
| HETATM13747 | O | HOH | A | 311 | 36.662 | 7.992 | 6.121 | 1.00 | 25.07 | O |
| HETATM13748 | O | HOH | A | 312 | 10.034 | 24.321 | 41.682 | 1.00 | 32.78 | O |
| HETATM13749 | O | HOH | A | 313 | 40.649 | 14.445 | -16.316 | 1.00 | 25.03 | O |
| HETATM13750 | O | HOH | A | 314 | 17.803 | 7.905 | 36.595 | 1.00 | 27.88 | O |
| HETATM13751 | O | HOH | A | 315 | 41.253 | 31.173 | -19.633 | 1.00 | 34.93 | O |
| HETATM13752 | O | HOH | A | 316 | 53.177 | 15.851 | 5.281 | 1.00 | 25.82 | O |
| HETATM13753 | O | HOH | A | 317 | 24.539 | 29.526 | 23.474 | 1.00 | 34.03 | O |
| HETATM13754 | O | HOH | A | 318 | 40.993 | 9.299 | 21.324 | 1.00 | 35.19 | O |
| HETATM13755 | O | HOH | A | 319 | 38.003 | 10.703 | 24.413 | 1.00 | 31.53 | O |
| HETATM13756 | O | HOH | A | 320 | 47.758 | 24.677 | 12.593 | 1.00 | 42.87 | O |
| HETATM13757 | O | HOH | A | 321 | 30.922 | 21.860 | -13.974 | 1.00 | 24.53 | O |
| HETATM13758 | O | HOH | A | 322 | 37.097 | 16.765 | -22.623 | 1.00 | 26.29 | O |
| HETATM13759 | O | HOH | A | 323 | 14.409 | 30.444 | 33.891 | 1.00 | 29.95 | O |
| HETATM13760 | O | HOH | A | 324 | 15.973 | 9.283 | 12.414 | 1.00 | 26.88 | O |
| HETATM13761 | O | HOH | A | 325 | 50.655 | 23.483 | -8.122 | 1.00 | 28.21 | O |
| HETATM13762 | O | HOH | A | 326 | 15.480 | 9.684 | 39.489 | 1.00 | 32.68 | O |
| HETATM13763 | O | HOH | A | 327 | 27.322 | 28.009 | 24.018 | 1.00 | 35.45 | O |
| HETATM13764 | O | HOH | A | 328 | 37.987 | 7.508 | 19.460 | 1.00 | 31.60 | O |
| HETATM13765 | O | HOH | A | 329 | 32.430 | 5.108 | 22.337 | 1.00 | 31.22 | O |
| HETATM13766 | O | HOH | A | 330 | -0.302 | 29.863 | 25.297 | 1.00 | 31.87 | O |
| HETATM13767 | O | HOH | A | 331 | 24.021 | 23.611 | 18.951 | 1.00 | 27.68 | O |
| HETATM13768 | O | HOH | A | 332 | 51.611 | 24.043 | 14.483 | 1.00 | 47.38 | O |

```
HETATM13769  O   HOH A 333     36.739  29.452 -11.746  1.00 32.03        O
HETATM13770  O   HOH A 334     42.837  33.813  -6.587  1.00 42.31        O
HETATM13771  O   HOH A 335     19.964  23.760  34.954  1.00 27.88        O
HETATM13772  O   HOH A 336     37.010  10.410 -15.456  1.00 29.48        O
HETATM13773  O   HOH A 337      8.861   4.995  26.596  1.00 36.22        O
HETATM13774  O   HOH A 338     19.993  26.853  30.551  1.00 31.11        O
HETATM13775  O   HOH A 339     50.566  13.280   7.940  1.00 28.95        O
HETATM13776  O   HOH A 340     33.458  10.160 -19.992  1.00 29.42        O
HETATM13777  O   HOH A 341     39.789  23.342  16.711  1.00 34.21        O
HETATM13778  O   HOH A 342     15.112  11.930   9.603  1.00 30.05        O
HETATM13779  O   HOH A 343     43.021  18.243 -19.561  1.00 28.94        O
HETATM13780  O   HOH A 344     50.083  25.419   3.215  1.00 30.23        O
HETATM13781  O   HOH A 345     14.063  -0.112  28.167  1.00 46.07        O
HETATM13782  O   HOH A 346     -1.023  12.804  40.547  1.00 41.26        O
HETATM13783  O   HOH A 347     20.407  27.140  32.822  1.00 33.56        O
HETATM13784  O   HOH A 348      2.088  13.215  29.771  1.00 22.56        O
HETATM13785  O   HOH A 349     23.257   7.369  22.534  1.00 30.42        O
HETATM13786  O   HOH A 350     13.917  27.277  20.260  1.00 31.81        O
HETATM13787  O   HOH A 351     16.091  31.088  24.792  1.00 36.45        O
HETATM13788  O   HOH A 352     41.586  27.649  -0.355  1.00 36.49        O
HETATM13789  O   HOH A 353     25.684  17.178  15.702  1.00 40.25        O
HETATM13790  O   HOH A 354     43.755  19.356  20.532  1.00 40.67        O
HETATM13791  O   HOH A 355     29.721  16.681  18.115  1.00 23.99        O
HETATM13792  O   HOH A 356     42.552  15.964 -16.148  1.00 31.58        O
HETATM13793  O   HOH A 357     15.730  29.628  36.094  1.00 36.63        O
HETATM13794  O   HOH A 358     46.106  14.340  21.370  1.00 29.91        O
HETATM13795  O   HOH A 359     20.475   7.275  22.904  1.00 48.89        O
HETATM13796  O   HOH A 360     22.716  22.138  35.547  1.00 36.24        O
HETATM13797  O   HOH A 361     38.928  13.727 -20.581  1.00 35.11        O
HETATM13798  O   HOH A 362     21.482  17.600  13.631  1.00 37.89        O
HETATM13799  O   HOH A 363     34.501  21.786  16.836  1.00 36.23        O
HETATM13800  O   HOH A 364     51.844  32.031  -4.087  1.00 40.00        O
HETATM13801  O   HOH A 365     40.919  33.346 -23.803  1.00 36.14        O
HETATM13802  O   HOH A 366     28.191  13.246  11.581  1.00 35.25        O
HETATM13803  O   HOH A 367     15.454  28.873  20.774  1.00 27.73        O
HETATM13804  O   HOH A 368     34.469  19.225   9.925  1.00 26.13        O
HETATM13805  O   HOH A 369     -2.641  14.669  35.537  1.00 39.44        O
HETATM13806  O   HOH A 370     49.813  13.129 -11.728  1.00 39.13        O
HETATM13807  O   HOH A 371     28.079  15.878  15.807  1.00 35.26        O
HETATM13808  O   HOH A 372     39.794  17.447 -22.305  1.00 39.55        O
HETATM13809  O   HOH A 373     37.472  20.321  17.761  1.00 27.88        O
HETATM13810  O   HOH A 374     24.586   9.775  13.291  1.00 37.26        O
HETATM13811  O   HOH A 375     44.885   9.265   0.202  1.00 36.25        O
HETATM13812  O   HOH A 376      7.004  12.400  38.349  1.00 29.26        O
HETATM13813  O   HOH A 377     19.801  19.828  14.085  1.00 46.21        O
HETATM13814  O   HOH A 378     19.223  15.667  10.966  1.00 38.38        O
HETATM13815  O   HOH A 379     52.146  13.658  -6.679  1.00 40.24        O
HETATM13816  O   HOH A 380     36.515  31.078  -3.209  1.00 36.59        O
HETATM13817  O   HOH A 381     31.092  19.062  17.097  1.00 36.03        O
HETATM13818  O   HOH A 382     47.744  12.659  -7.520  1.00 35.19        O
HETATM13819  O   HOH A 383     45.445  25.070  10.857  1.00 34.77        O
HETATM13820  O   HOH A 384     38.227  25.201   7.842  1.00 46.06        O
HETATM13821  O   HOH A 385     19.073  25.976  34.592  1.00 39.29        O
HETATM13822  O   HOH A 386     44.762  25.806 -23.726  1.00 42.09        O
HETATM13823  O   HOH A 387     32.473  19.200  -3.425  1.00 29.08        O
HETATM13824  O   HOH A 388      5.288   9.902  35.162  1.00 31.94        O
HETATM13825  O   HOH A 389     52.349  14.986  -8.911  1.00 40.46        O
HETATM13826  O   HOH A 390     45.340  30.659 -11.009  1.00 36.84        O
HETATM13827  O   HOH A 391     48.475  11.426  -9.908  1.00 44.16        O
HETATM13828  O   HOH A 392     11.192  29.942  21.900  1.00 28.51        O
HETATM13829  O   HOH A 393     31.477  21.997  -2.226  1.00 47.03        O
HETATM13830  O   HOH A 394     46.966  18.854  21.622  1.00 40.07        O
HETATM13831  O   HOH A 395     52.496  18.153  -9.595  1.00 37.80        O
HETATM13832  O   HOH A 396      7.802  30.861  23.178  1.00 26.78        O
HETATM13833  O   HOH A 397     32.891  25.438 -14.652  1.00 33.76        O
```

```
HETATM13834  O    HOH A 398      17.212  22.971  15.656  1.00 33.70           O
HETATM13835  O    HOH A 399      29.836  16.757  34.031  1.00 35.90           O
HETATM13836  O    HOH A 400      39.020  31.963 -12.588  1.00 29.92           O
HETATM13837  O    HOH A 401      25.241   6.424  16.893  1.00 51.32           O
HETATM13838  O    HOH A 402      33.151  28.153 -13.514  1.00 35.10           O
HETATM13839  O    HOH A 403      10.274   4.851  32.104  1.00 36.64           O
HETATM13840  O    HOH A 404      25.285  21.213  35.015  1.00 40.78           O
HETATM13841  O    HOH A 405      38.272   6.144   6.585  1.00 36.13           O
HETATM13842  O    HOH A 406      13.635   7.851  37.016  1.00 46.15           O
HETATM13843  O    HOH A 407      49.575  14.992 -18.220  1.00 51.52           O
HETATM13844  O    HOH A 408      33.093   5.474  26.418  1.00 33.71           O
HETATM13845  O    HOH A 409      13.263   8.736  11.186  1.00 39.29           O
HETATM13846  O    HOH A 410      43.065  15.901 -19.086  1.00 43.06           O
HETATM13847  O    HOH A 411      31.536  25.779 -16.977  1.00 39.09           O
HETATM13848  O    HOH A 412      -4.897  15.801  35.561  1.00 34.53           O
HETATM13849  O    HOH A 413      31.990  23.266  -5.717  1.00 37.13           O
HETATM13850  O    HOH A 414      22.472  25.515  28.924  1.00 36.27           O
HETATM13851  O    HOH A 415      38.853  31.660 -17.151  1.00 33.64           O
HETATM13852  O    HOH A 416      44.401  30.339   0.133  1.00 36.06           O
HETATM13853  O    HOH A 417      20.680  28.553  20.217  1.00 34.97           O
HETATM13854  O    HOH A 418      24.985  23.494  29.492  1.00 39.36           O
HETATM13855  O    HOH A 419      30.644  12.369   9.670  1.00 41.12           O
HETATM13856  O    HOH A 420      51.835  11.414  -2.461  1.00 54.61           O
HETATM13857  O    HOH A 421      39.611   7.678  12.486  1.00 46.24           O
HETATM13858  O    HOH A 422      44.709  28.871   2.972  1.00 42.03           O
HETATM13859  O    HOH A 423      26.728  21.644  28.739  1.00 44.59           O
HETATM13860  O    HOH A 424      47.018  30.681  -9.066  1.00 40.37           O
HETATM13861  O    HOH A 425      35.372  31.386  -9.436  1.00 53.81           O
HETATM13862  O    HOH A 426      39.253  26.014  10.861  1.00 36.09           O
HETATM13863  O    HOH A 427      39.843  19.210  21.909  1.00 40.42           O
HETATM13864  O    HOH A 428       0.525  32.139  30.837  1.00 48.26           O
HETATM13865  O    HOH A 429      56.229  19.673  12.481  1.00 15.00           O
HETATM13866  O    HOH A 430      55.619  16.022 -10.992  1.00 35.38           O
HETATM13867  O    HOH A 431      36.170  21.442  10.458  1.00 48.17           O
HETATM13868  O    HOH A 432      34.584  23.863  10.851  1.00 47.28           O
HETATM13869  O    HOH A 433      50.951  14.830  11.643  1.00 30.89           O
HETATM13870  O    HOH A 434      56.306  24.866   7.352  1.00 34.81           O
HETATM13871  O    HOH A 435      30.004   4.433  18.059  1.00 41.16           O
HETATM13872  O    HOH A 436      36.649  25.166  11.350  1.00 50.37           O
HETATM13873  O    HOH A 437       1.709  31.897  20.895  1.00 34.25           O
HETATM13874  O    HOH A 438      45.499  10.228  10.927  1.00 38.42           O
HETATM13875  O    HOH A 439      46.487  20.697 -23.011  1.00 49.24           O
HETATM13876  O    HOH A 440      28.059  10.848  12.135  1.00 39.47           O
HETATM13877  O    HOH A 441      51.551  25.550  10.977  1.00 42.21           O
HETATM13878  O    HOH A 442      12.004  27.126  40.422  1.00 40.87           O
HETATM13879  O    HOH A 443      46.536  10.244  -1.504  1.00 33.58           O
HETATM13880  O    HOH A 444      29.687  21.551  22.225  1.00 40.33           O
HETATM13881  O    HOH A 445      29.407  18.661  29.917  1.00 44.17           O
HETATM13882  O    HOH A 446      30.033  25.017   3.887  1.00 55.82           O
HETATM13883  O    HOH A 447      19.382   6.376  34.891  1.00 37.49           O
HETATM13884  O    HOH A 448      49.505   9.895  -3.898  1.00 43.45           O
HETATM13885  O    HOH A 449       6.710  25.558  40.739  1.00 36.20           O
HETATM13886  O    HOH A 450      39.944  12.973 -18.258  1.00 33.54           O
HETATM13887  O    HOH A 451      34.946   9.147 -15.806  1.00 45.84           O
HETATM13888  O    HOH A 452      41.300  34.390  -8.768  1.00 47.01           O
HETATM13889  O    HOH A 453      20.927  30.348  24.927  1.00 40.99           O
HETATM13890  O    HOH A 454      43.908  24.032  16.128  1.00 39.42           O
HETATM13891  O    HOH A 455      17.802  25.604  17.322  1.00 40.33           O
HETATM13892  O    HOH A 456      21.976  29.856  27.353  1.00 39.95           O
HETATM13893  O    HOH A 457      29.514   4.197  28.591  1.00 50.68           O
HETATM13894  O    HOH A 458      45.035  10.367  16.985  1.00 34.94           O
HETATM13895  O    HOH A 459      51.193  25.462   6.548  1.00 38.19           O
HETATM13896  O    HOH A 460       8.784  34.872  32.319  1.00 42.63           O
HETATM13897  O    HOH A 461      33.537   4.674  34.973  1.00 53.99           O
HETATM13898  O    HOH A 462      -5.545  17.664  37.813  1.00 51.83           O
```

```
HETATM13899  O   HOH A 463    13.283  33.047  33.379  1.00 46.73        O
HETATM13900  O   HOH A 464    31.719  21.537  20.284  1.00 47.73        O
HETATM13901  O   HOH A 465    41.271  21.229  20.690  1.00 47.20        O
HETATM13902  O   HOH A 466     4.276  24.960  40.031  1.00 41.81        O
HETATM13903  O   HOH A 467    48.039  12.250   7.492  1.00 31.63        O
HETATM13904  O   HOH A 468    15.467   8.868  15.596  1.00 49.22        O
HETATM13905  O   HOH A 469    51.659  13.634   1.301  1.00 39.35        O
HETATM13906  O   HOH A 470    22.885  23.372  27.965  1.00 35.20        O
HETATM13907  O   HOH A 471    29.602  19.038  14.930  1.00 40.74        O
HETATM13908  O   HOH A 472    33.732  15.324  31.757  1.00 48.93        O
HETATM13909  O   HOH A 473    55.361  21.957   2.387  1.00 42.84        O
HETATM13910  O   HOH A 474    56.516  24.059   4.897  1.00 50.36        O
HETATM13911  O   HOH A 475    46.714  33.714  -3.935  1.00 52.78        O
HETATM13912  O   HOH A 476    18.965   6.860  17.288  1.00 46.56        O
HETATM13913  O   HOH A 477    54.213  26.077   3.387  1.00 39.57        O
HETATM13914  O   HOH A 478     7.230  21.353  23.461  1.00 36.57        O
HETATM13915  O   HOH A 479    11.742   4.838  23.238  1.00 35.86        O
HETATM13916  O   HOH A 480    30.163  19.008  24.433  1.00 41.05        O
HETATM13917  O   HOH A 481    39.755  10.261 -17.122  1.00 46.65        O
HETATM13918  O   HOH A 482    40.077  30.726  -1.255  1.00 47.48        O
HETATM13919  O   HOH A 483    24.614  30.739  25.848  1.00 51.61        O
HETATM13920  O   HOH A 484     1.046  23.626  34.830  1.00 39.78        O
HETATM13921  O   HOH A 485    45.531  14.493 -19.463  1.00 47.90        O
HETATM13922  O   HOH A 486    43.945  33.153  -9.643  1.00 53.86        O
HETATM13923  O   HOH A 487    23.177  26.627  33.226  1.00 43.07        O
HETATM13924  O   HOH A 488    43.910  14.435  24.073  1.00 47.62        O
HETATM13925  O   HOH A 489    50.378  27.754   7.353  1.00 50.24        O
HETATM13926  O   HOH A 490    44.480  19.089  23.472  1.00 42.12        O
HETATM13927  O   HOH A 491    20.408  29.471  30.731  1.00 57.62        O
HETATM13928  O   HOH A 492     5.677  32.653  33.821  1.00 39.09        O
HETATM13929  O   HOH A 493    15.032   2.567  29.102  1.00 45.52        O
HETATM13930  O   HOH A 494    51.339  17.079 -17.856  1.00 50.12        O
HETATM13931  O   HOH A 495    55.069  16.327   9.032  1.00 24.93        O
HETATM13932  O   HOH A 496    27.617  16.392  36.336  1.00 43.66        O
HETATM13933  O   HOH A 497    40.360  27.572   1.667  1.00 44.33        O
HETATM13934  O   HOH A 498    38.563   5.775  16.980  1.00 43.74        O
HETATM13935  O   HOH A 499    48.316  12.101  16.402  1.00 44.41        O
HETATM13936  O   HOH A 500    -3.879  18.044  39.795  1.00 46.35        O
HETATM13937  O   HOH A 501    29.544   7.703   9.750  1.00 57.36        O
HETATM13938  O   HOH A 502    57.729  20.318   5.709  1.00 47.85        O
HETATM13939  O   HOH A 503    10.817   5.421  34.773  1.00 49.20        O
HETATM13940  O   HOH A 504    16.377  33.038  27.423  1.00 46.33        O
HETATM13941  O   HOH A 505    42.822  31.409 -17.808  1.00 59.77        O
HETATM13942  O   HOH A 506    51.878  26.750   4.303  1.00 47.43        O
HETATM13943  O   HOH A 507    41.437  13.488  24.651  1.00 50.17        O
HETATM13944  O   HOH A 508    19.645  22.843  37.698  1.00 48.51        O
HETATM13945  O   HOH A 509    40.719   6.622  15.354  1.00 50.19        O
HETATM13946  O   HOH A 510    24.792   2.462  25.198  1.00 50.91        O
HETATM13947  O   HOH A 511    31.965  27.972 -11.121  1.00 42.76        O
HETATM13948  O   HOH A 512    47.201  12.629 -17.341  1.00 50.04        O
HETATM13949  O   HOH A 513    45.546   8.049  19.231  1.00 48.67        O
HETATM13950  O   HOH A 514    11.332   9.557  38.526  1.00 44.51        O
HETATM13951  O   HOH A 515    41.604  33.261 -13.543  1.00 41.02        O
HETATM13952  O   HOH A 516    45.382   8.968  14.680  1.00 51.95        O
HETATM13953  O   HOH A 517    42.042  18.242  22.647  1.00 40.07        O
HETATM13954  O   HOH A 518    43.489  20.845 -24.049  1.00 47.44        O
HETATM13955  O   HOH A 519    13.554  30.264  37.510  1.00 62.35        O
HETATM13956  O   HOH A 520    19.240   8.986  38.929  1.00 46.44        O
HETATM13957  O   HOH A 521    34.789  20.042  30.381  1.00 56.52        O
HETATM13958  O   HOH A 522    51.958  13.278   5.651  1.00 40.30        O
HETATM13959  O   HOH A 523    24.553  26.543  17.329  1.00 52.85        O
HETATM13960  O   HOH A 524    18.795  31.346  23.996  1.00 47.89        O
HETATM13961  O   HOH A 525    48.869  33.882  -5.990  1.00 47.12        O
HETATM13962  O   HOH A 526    -6.113  20.186  37.439  1.00 43.35        O
HETATM13963  O   HOH A 527    35.361  24.412  26.803  1.00 55.21        O
```

| | | | | | | | | | |
|---|---|---|---|---|---|---|---|---|---|
| HETATM13964 | O | HOH A 528 | 35.196 | 6.822 | -16.968 | 1.00 | 59.66 | | O |
| HETATM13965 | O | HOH A 529 | 37.780 | 7.434 | 30.505 | 1.00 | 49.54 | | O |
| HETATM13966 | O | HOH A 530 | 38.294 | 20.850 | 24.134 | 1.00 | 46.22 | | O |
| HETATM13967 | O | HOH A 531 | 33.440 | 6.897 | 10.598 | 1.00 | 55.60 | | O |
| HETATM13968 | O | HOH A 532 | 49.650 | 13.178 | -16.448 | 1.00 | 54.38 | | O |
| HETATM13969 | O | HOH A 533 | 38.723 | 3.392 | 6.512 | 1.00 | 49.04 | | O |
| HETATM13970 | O | HOH A 534 | 22.125 | 26.933 | 17.938 | 1.00 | 39.64 | | O |
| HETATM13971 | O | HOH A 535 | 57.242 | 23.663 | 9.564 | 1.00 | 42.40 | | O |
| HETATM13972 | O | HOH A 536 | 39.121 | 7.855 | 27.105 | 1.00 | 47.54 | | O |
| HETATM13973 | O | HOH A 537 | 14.452 | 5.501 | 11.504 | 1.00 | 48.82 | | O |
| HETATM13974 | O | HOH A 538 | 38.762 | 8.955 | 22.203 | 1.00 | 50.65 | | O |
| HETATM13975 | O | HOH A 539 | 36.647 | 28.236 | 4.347 | 1.00 | 45.80 | | O |
| HETATM13976 | O | HOH A 540 | 34.794 | 5.276 | 20.396 | 1.00 | 51.27 | | O |
| HETATM13977 | O | HOH A 541 | 16.584 | 29.520 | 39.396 | 1.00 | 43.20 | | O |
| HETATM13978 | O | HOH A 542 | 11.335 | 6.491 | 37.504 | 1.00 | 54.03 | | O |
| HETATM13979 | O | HOH A 543 | 36.261 | 12.172 | -22.820 | 1.00 | 35.11 | | O |
| HETATM13980 | O | HOH A 544 | 38.851 | 21.521 | 19.575 | 1.00 | 47.21 | | O |
| HETATM13981 | O | HOH A 545 | 45.893 | 7.871 | 6.610 | 1.00 | 41.10 | | O |
| HETATM13982 | O | HOH A 546 | 29.187 | 21.139 | 9.323 | 1.00 | 53.83 | | O |
| HETATM13983 | O | HOH A 547 | 53.460 | 22.708 | -2.864 | 1.00 | 44.83 | | O |
| HETATM13984 | O | HOH A 548 | 23.682 | 1.774 | 30.554 | 1.00 | 58.21 | | O |
| HETATM13985 | O | HOH A 549 | 47.855 | 9.764 | 6.721 | 1.00 | 46.37 | | O |
| HETATM13986 | O | HOH A 550 | -4.169 | 22.659 | 37.999 | 1.00 | 50.03 | | O |
| HETATM13987 | O | HOH A 551 | 47.106 | 9.744 | 2.094 | 1.00 | 42.85 | | O |
| HETATM13988 | O | HOH A 552 | 41.530 | 27.290 | 10.885 | 1.00 | 54.28 | | O |
| HETATM13989 | O | HOH A 553 | 54.840 | 17.737 | 1.862 | 1.00 | 46.62 | | O |
| HETATM13990 | O | HOH A 554 | 51.496 | 12.498 | -13.735 | 1.00 | 65.45 | | O |
| HETATM13991 | O | HOH A 555 | 51.848 | 24.210 | -17.717 | 1.00 | 54.91 | | O |
| HETATM13992 | O | HOH A 556 | 29.160 | 2.404 | 16.468 | 1.00 | 68.64 | | O |
| HETATM13993 | O | HOH A 557 | 47.463 | 30.174 | -21.526 | 1.00 | 50.83 | | O |
| HETATM13994 | O | HOH A 558 | 49.626 | 23.536 | -19.264 | 1.00 | 48.78 | | O |
| HETATM13995 | O | HOH A 559 | 31.724 | 10.275 | 37.212 | 1.00 | 66.14 | | O |
| HETATM13996 | O | HOH A 560 | 45.156 | 31.623 | -17.137 | 1.00 | 44.90 | | O |
| HETATM13997 | O | HOH A 561 | 38.871 | 27.302 | 6.377 | 1.00 | 66.86 | | O |
| HETATM13998 | O | HOH A 562 | 10.758 | 33.702 | 33.706 | 1.00 | 51.75 | | O |
| HETATM13999 | O | HOH A 563 | 44.392 | 31.081 | -14.809 | 1.00 | 47.30 | | O |
| HETATM14000 | O | HOH A 564 | 47.996 | 25.274 | 15.244 | 1.00 | 51.21 | | O |
| HETATM14001 | O | HOH A 565 | 51.557 | 28.436 | -15.881 | 1.00 | 52.24 | | O |
| HETATM14002 | O | HOH A 566 | 30.893 | 24.418 | -13.486 | 1.00 | 37.90 | | O |
| HETATM14003 | O | HOH A 567 | 48.253 | 24.246 | -21.233 | 1.00 | 44.49 | | O |
| HETATM14004 | O | HOH A 568 | 27.619 | 26.254 | 27.408 | 1.00 | 51.52 | | O |
| HETATM14005 | O | HOH A 569 | 15.575 | 4.506 | 22.440 | 1.00 | 40.57 | | O |
| HETATM14006 | O | HOH A 570 | 52.929 | 29.841 | -3.112 | 1.00 | 58.06 | | O |
| HETATM14007 | O | HOH A 571 | 17.650 | 31.556 | 36.090 | 1.00 | 50.97 | | O |
| HETATM14008 | O | HOH A 572 | 27.415 | 7.266 | 37.298 | 1.00 | 61.41 | | O |
| HETATM14009 | O | HOH A 573 | 9.631 | 10.782 | 37.424 | 1.00 | 49.82 | | O |
| HETATM14010 | O | HOH A 574 | 54.231 | 14.207 | 9.496 | 1.00 | 33.13 | | O |
| HETATM14011 | O | HOH A 575 | 48.568 | 9.878 | 11.418 | 1.00 | 58.02 | | O |
| HETATM14012 | O | HOH A 576 | 10.127 | 32.244 | 21.889 | 1.00 | 22.09 | | O |
| HETATM14013 | O | HOH B 311 | -11.467 | 25.786 | 15.683 | 1.00 | 24.82 | | O |
| HETATM14014 | O | HOH B 312 | 10.039 | 26.059 | 19.755 | 1.00 | 14.20 | | O |
| HETATM14015 | O | HOH B 313 | 3.293 | 11.432 | 25.228 | 1.00 | 16.01 | | O |
| HETATM14016 | O | HOH B 314 | 13.173 | 20.785 | -0.570 | 1.00 | 15.70 | | O |
| HETATM14017 | O | HOH B 315 | 41.309 | -4.342 | -6.020 | 1.00 | 24.47 | | O |
| HETATM14018 | O | HOH B 316 | 35.766 | 8.189 | 3.549 | 1.00 | 14.05 | | O |
| HETATM14019 | O | HOH B 317 | 1.980 | 30.521 | 18.813 | 1.00 | 18.79 | | O |
| HETATM14020 | O | HOH B 318 | -8.364 | 24.051 | 11.262 | 1.00 | 21.13 | | O |
| HETATM14021 | O | HOH B 319 | 0.326 | 11.632 | 20.246 | 1.00 | 16.07 | | O |
| HETATM14022 | O | HOH B 320 | -4.903 | 27.907 | 17.499 | 1.00 | 21.55 | | O |
| HETATM14023 | O | HOH B 321 | -9.613 | 16.266 | 10.168 | 1.00 | 20.69 | | O |
| HETATM14024 | O | HOH B 322 | 40.355 | 9.325 | 3.411 | 1.00 | 19.26 | | O |
| HETATM14025 | O | HOH B 323 | 10.877 | 9.633 | 19.788 | 1.00 | 17.09 | | O |
| HETATM14026 | O | HOH B 324 | -10.547 | 11.044 | 10.401 | 1.00 | 23.24 | | O |
| HETATM14027 | O | HOH B 325 | 31.510 | 18.848 | -0.782 | 1.00 | 18.02 | | O |
| HETATM14028 | O | HOH B 326 | 22.301 | 5.914 | -13.944 | 1.00 | 22.97 | | O |

```
HETATM14029  O   HOH B 327     5.570  27.402  17.459  1.00 19.81      O
HETATM14030  O   HOH B 328   -12.129   7.909  14.285  1.00 22.07      O
HETATM14031  O   HOH B 329    -2.588  25.501  26.024  1.00 17.99      O
HETATM14032  O   HOH B 330    23.398  19.740 -13.159  1.00 18.66      O
HETATM14033  O   HOH B 331    34.022   0.168  -9.554  1.00 18.02      O
HETATM14034  O   HOH B 332    10.620   8.998  17.334  1.00 21.89      O
HETATM14035  O   HOH B 333    15.023  17.110  -3.947  1.00 23.16      O
HETATM14036  O   HOH B 334    25.047  12.924 -13.965  1.00 19.25      O
HETATM14037  O   HOH B 335    38.474  -8.526  -4.031  1.00 27.64      O
HETATM14038  O   HOH B 336    27.852  17.349 -16.738  1.00 19.01      O
HETATM14039  O   HOH B 337    24.856   0.291  -6.881  1.00 28.47      O
HETATM14040  O   HOH B 338    23.133  11.424   1.740  1.00 37.43      O
HETATM14041  O   HOH B 339     9.301   7.067  21.969  1.00 29.23      O
HETATM14042  O   HOH B 340    42.633   6.278   1.823  1.00 24.07      O
HETATM14043  O   HOH B 341    12.139  15.173   1.612  1.00 21.01      O
HETATM14044  O   HOH B 342     4.567  31.540  16.255  1.00 26.10      O
HETATM14045  O   HOH B 343     3.443   9.132   3.586  1.00 22.91      O
HETATM14046  O   HOH B 344    25.815   3.614   1.278  1.00 19.35      O
HETATM14047  O   HOH B 345    -8.316  13.987  23.419  1.00 21.06      O
HETATM14048  O   HOH B 346    38.043  11.845 -10.842  1.00 30.33      O
HETATM14049  O   HOH B 347    17.247   9.969 -13.814  1.00 24.10      O
HETATM14050  O   HOH B 348    12.054   8.889  13.352  1.00 26.27      O
HETATM14051  O   HOH B 349    -5.594  19.527  28.154  1.00 24.42      O
HETATM14052  O   HOH B 350    20.681   7.340  -3.168  1.00 25.09      O
HETATM14053  O   HOH B 351    28.224  20.481 -13.208  1.00 24.99      O
HETATM14054  O   HOH B 352    -5.315  17.398  33.573  1.00 24.21      O
HETATM14055  O   HOH B 353    23.197  22.287 -13.107  1.00 23.57      O
HETATM14056  O   HOH B 354    37.781  10.131 -12.728  1.00 31.79      O
HETATM14057  O   HOH B 355    -9.524  10.538  16.343  1.00 23.61      O
HETATM14058  O   HOH B 356    -5.487  19.399   5.734  1.00 20.66      O
HETATM14059  O   HOH B 357    11.986  26.284  11.717  1.00 26.60      O
HETATM14060  O   HOH B 358    29.727  20.716  -0.450  1.00 23.31      O
HETATM14061  O   HOH B 359    10.528  28.047  -8.138  1.00 26.06      O
HETATM14062  O   HOH B 360     3.010  10.035  21.613  1.00 26.39      O
HETATM14063  O   HOH B 361     4.103   4.177   8.526  1.00 31.50      O
HETATM14064  O   HOH B 362    -6.398  28.030  26.291  1.00 28.27      O
HETATM14065  O   HOH B 363     4.609  29.534  18.743  1.00 24.01      O
HETATM14066  O   HOH B 364    28.296  12.677 -15.206  1.00 21.93      O
HETATM14067  O   HOH B 365    -2.342   1.801  19.836  1.00 31.98      O
HETATM14068  O   HOH B 366    -7.890  17.251  23.530  1.00 27.40      O
HETATM14069  O   HOH B 367    45.068  12.089  -7.678  1.00 22.49      O
HETATM14070  O   HOH B 368    -7.098  20.923   4.297  1.00 29.86      O
HETATM14071  O   HOH B 369    28.293   4.247 -14.187  1.00 26.78      O
HETATM14072  O   HOH B 370    -0.514  28.453  19.889  1.00 36.05      O
HETATM14073  O   HOH B 371    22.650  14.043 -14.272  1.00 25.21      O
HETATM14074  O   HOH B 372    26.958  23.908  -8.501  1.00 26.81      O
HETATM14075  O   HOH B 373   -12.242   9.797  19.685  1.00 32.84      O
HETATM14076  O   HOH B 374    13.177  28.756   6.407  1.00 24.09      O
HETATM14077  O   HOH B 375   -10.035  24.201  21.938  1.00 26.80      O
HETATM14078  O   HOH B 376    42.165   5.970 -10.487  1.00 21.79      O
HETATM14079  O   HOH B 377    11.297  17.072  -0.636  1.00 21.90      O
HETATM14080  O   HOH B 378    42.310   8.837  -3.230  1.00 27.61      O
HETATM14081  O   HOH B 379    23.562  19.052   2.669  1.00 31.20      O
HETATM14082  O   HOH B 380    24.587  15.669 -15.663  1.00 27.69      O
HETATM14083  O   HOH B 381    42.511  -7.808  -4.763  1.00 25.81      O
HETATM14084  O   HOH B 382    -5.033  19.789  35.039  1.00 24.81      O
HETATM14085  O   HOH B 383    16.978  23.607  -0.967  1.00 28.03      O
HETATM14086  O   HOH B 384    -1.475  25.437  30.624  1.00 20.21      O
HETATM14087  O   HOH B 385    18.098  27.009  -9.745  1.00 24.77      O
HETATM14088  O   HOH B 386    20.625  12.803 -15.589  1.00 21.63      O
HETATM14089  O   HOH B 387    32.253   4.067   9.292  1.00 27.57      O
HETATM14090  O   HOH B 388    -7.084   4.742  12.960  1.00 30.63      O
HETATM14091  O   HOH B 389    36.327  -7.436  -2.361  1.00 30.76      O
HETATM14092  O   HOH B 390    12.844  32.722  -4.033  1.00 30.74      O
HETATM14093  O   HOH B 391    15.852  29.537 -11.514  1.00 29.15      O
```

| | | | | | | | | | |
|---|---|---|---|---|---|---|---|---|---|
| HETATM14094 | O | HOH B 392 | -5.318 | 16.817 | 29.223 | 1.00 | 29.84 | | O |
| HETATM14095 | O | HOH B 393 | 39.571 | -8.170 | 0.178 | 1.00 | 31.97 | | O |
| HETATM14096 | O | HOH B 394 | 27.352 | -0.159 | -10.995 | 1.00 | 26.12 | | O |
| HETATM14097 | O | HOH B 395 | 34.084 | 9.646 | 8.448 | 1.00 | 33.35 | | O |
| HETATM14098 | O | HOH B 396 | 31.685 | 5.019 | -14.794 | 1.00 | 26.51 | | O |
| HETATM14099 | O | HOH B 397 | 43.523 | 6.827 | -1.888 | 1.00 | 31.26 | | O |
| HETATM14100 | O | HOH B 398 | 34.358 | -3.144 | -3.999 | 1.00 | 25.07 | | O |
| HETATM14101 | O | HOH B 399 | 9.472 | 13.283 | 1.544 | 1.00 | 35.52 | | O |
| HETATM14102 | O | HOH B 400 | 6.128 | 20.502 | -5.322 | 1.00 | 33.32 | | O |
| HETATM14103 | O | HOH B 401 | 27.831 | 11.604 | 6.300 | 1.00 | 26.05 | | O |
| HETATM14104 | O | HOH B 402 | -3.812 | 2.982 | 12.685 | 1.00 | 31.90 | | O |
| HETATM14105 | O | HOH B 403 | -8.970 | 26.124 | 14.869 | 1.00 | 34.02 | | O |
| HETATM14106 | O | HOH B 404 | -9.646 | 21.439 | 28.387 | 1.00 | 35.31 | | O |
| HETATM14107 | O | HOH B 405 | 43.934 | -3.381 | 3.908 | 1.00 | 32.46 | | O |
| HETATM14108 | O | HOH B 406 | 41.204 | -9.588 | -3.469 | 1.00 | 32.75 | | O |
| HETATM14109 | O | HOH B 407 | 4.837 | -0.520 | 11.914 | 1.00 | 38.22 | | O |
| HETATM14110 | O | HOH B 408 | 22.954 | 14.444 | 1.557 | 1.00 | 34.17 | | O |
| HETATM14111 | O | HOH B 409 | 5.913 | 30.857 | 20.793 | 1.00 | 38.73 | | O |
| HETATM14112 | O | HOH B 410 | 8.867 | 6.912 | 13.184 | 1.00 | 34.68 | | O |
| HETATM14113 | O | HOH B 411 | -14.006 | 20.606 | 19.977 | 1.00 | 35.09 | | O |
| HETATM14114 | O | HOH B 412 | -11.623 | 13.314 | 23.894 | 1.00 | 28.58 | | O |
| HETATM14115 | O | HOH B 413 | 40.720 | 8.194 | 5.829 | 1.00 | 31.87 | | O |
| HETATM14116 | O | HOH B 414 | 43.388 | -0.437 | -5.583 | 1.00 | 30.56 | | O |
| HETATM14117 | O | HOH B 415 | 44.334 | -3.661 | 0.707 | 1.00 | 29.14 | | O |
| HETATM14118 | O | HOH B 416 | -8.957 | 24.558 | 31.910 | 1.00 | 32.20 | | O |
| HETATM14119 | O | HOH B 417 | 27.097 | 5.208 | 5.744 | 1.00 | 29.46 | | O |
| HETATM14120 | O | HOH B 418 | -10.829 | 23.770 | 16.915 | 1.00 | 28.39 | | O |
| HETATM14121 | O | HOH B 419 | 29.286 | -1.326 | -7.864 | 1.00 | 32.29 | | O |
| HETATM14122 | O | HOH B 420 | 27.151 | 24.212 | -11.453 | 1.00 | 31.83 | | O |
| HETATM14123 | O | HOH B 421 | -1.620 | 29.387 | -1.885 | 1.00 | 28.35 | | O |
| HETATM14124 | O | HOH B 422 | 32.276 | -6.440 | -8.413 | 1.00 | 26.99 | | O |
| HETATM14125 | O | HOH B 423 | 25.870 | 19.202 | -16.563 | 1.00 | 31.63 | | O |
| HETATM14126 | O | HOH B 424 | 0.726 | 9.369 | 28.038 | 1.00 | 31.74 | | O |
| HETATM14127 | O | HOH B 425 | 34.337 | 6.660 | -13.033 | 1.00 | 28.51 | | O |
| HETATM14128 | O | HOH B 426 | 10.354 | 30.932 | 9.830 | 1.00 | 38.10 | | O |
| HETATM14129 | O | HOH B 427 | 35.757 | -1.799 | -10.684 | 1.00 | 23.52 | | O |
| HETATM14130 | O | HOH B 428 | 21.111 | 16.572 | 0.739 | 1.00 | 30.28 | | O |
| HETATM14131 | O | HOH B 429 | 14.311 | 10.408 | -5.451 | 1.00 | 36.54 | | O |
| HETATM14132 | O | HOH B 430 | -5.465 | 27.942 | 10.145 | 1.00 | 43.21 | | O |
| HETATM14133 | O | HOH B 431 | -0.065 | 21.877 | 32.364 | 1.00 | 33.52 | | O |
| HETATM14134 | O | HOH B 432 | 36.643 | -0.913 | -12.797 | 1.00 | 35.58 | | O |
| HETATM14135 | O | HOH B 433 | -0.095 | 2.399 | 21.100 | 1.00 | 29.09 | | O |
| HETATM14136 | O | HOH B 434 | -6.715 | 20.047 | 1.570 | 1.00 | 36.19 | | O |
| HETATM14137 | O | HOH B 435 | 1.163 | 13.260 | 0.390 | 1.00 | 36.80 | | O |
| HETATM14138 | O | HOH B 436 | 14.036 | 22.256 | 3.139 | 1.00 | 29.53 | | O |
| HETATM14139 | O | HOH B 437 | 26.575 | 9.484 | 3.727 | 1.00 | 32.91 | | O |
| HETATM14140 | O | HOH B 438 | -8.497 | 9.777 | 29.326 | 1.00 | 41.86 | | O |
| HETATM14141 | O | HOH B 439 | 30.389 | 5.468 | 7.485 | 1.00 | 34.10 | | O |
| HETATM14142 | O | HOH B 440 | 48.450 | 2.061 | 0.285 | 1.00 | 45.53 | | O |
| HETATM14143 | O | HOH B 441 | 30.629 | -4.508 | -5.251 | 1.00 | 21.35 | | O |
| HETATM14144 | O | HOH B 442 | 1.949 | 5.406 | 23.261 | 1.00 | 39.44 | | O |
| HETATM14145 | O | HOH B 443 | 1.810 | 0.857 | 19.766 | 1.00 | 41.15 | | O |
| HETATM14146 | O | HOH B 444 | 13.783 | 29.812 | -0.120 | 1.00 | 32.86 | | O |
| HETATM14147 | O | HOH B 445 | 12.805 | 18.241 | -2.635 | 1.00 | 31.04 | | O |
| HETATM14148 | O | HOH B 446 | -4.232 | 2.528 | 21.270 | 1.00 | 31.31 | | O |
| HETATM14149 | O | HOH B 447 | 24.975 | 16.293 | 2.579 | 1.00 | 31.08 | | O |
| HETATM14150 | O | HOH B 448 | 35.567 | 1.960 | 8.048 | 1.00 | 36.24 | | O |
| HETATM14151 | O | HOH B 449 | 14.767 | 10.606 | -7.841 | 1.00 | 38.34 | | O |
| HETATM14152 | O | HOH B 450 | -4.655 | 26.276 | 4.891 | 1.00 | 36.53 | | O |
| HETATM14153 | O | HOH B 451 | -16.809 | 13.170 | 13.491 | 1.00 | 26.97 | | O |
| HETATM14154 | O | HOH B 452 | 14.175 | 14.467 | 6.684 | 1.00 | 36.74 | | O |
| HETATM14155 | O | HOH B 453 | 10.103 | 28.667 | 19.802 | 1.00 | 30.16 | | O |
| HETATM14156 | O | HOH B 454 | 29.695 | -2.868 | -13.025 | 1.00 | 45.88 | | O |
| HETATM14157 | O | HOH B 455 | 0.780 | 19.487 | -2.913 | 1.00 | 31.42 | | O |
| HETATM14158 | O | HOH B 456 | 26.485 | -1.742 | -7.239 | 1.00 | 47.54 | | O |

```
HETATM14159  O   HOH B 457    49.280   8.147 -11.178  1.00 59.53       O
HETATM14160  O   HOH B 458    49.061   8.185  -8.608  1.00 47.33       O
HETATM14161  O   HOH B 459    35.825   4.856   9.116  1.00 30.17       O
HETATM14162  O   HOH B 460    -9.499  19.748  22.175  1.00 27.55       O
HETATM14163  O   HOH B 461    45.779   5.702  -9.510  1.00 52.17       O
HETATM14164  O   HOH B 462   -13.122  17.864  23.815  1.00 37.43       O
HETATM14165  O   HOH B 463   -10.367  28.039  25.009  1.00 40.47       O
HETATM14166  O   HOH B 464    10.351  27.424   8.300  1.00 21.56       O
HETATM14167  O   HOH B 465    11.055  35.674  -5.412  1.00 37.15       O
HETATM14168  O   HOH B 466    -8.974  26.277  23.180  1.00 32.60       O
HETATM14169  O   HOH B 467     5.635  16.033  -4.058  1.00 36.38       O
HETATM14170  O   HOH B 468    20.159  13.681 -11.695  1.00 33.76       O
HETATM14171  O   HOH B 469     4.253  25.263  -7.632  1.00 35.27       O
HETATM14172  O   HOH B 470    34.250   0.162  10.933  1.00 51.11       O
HETATM14173  O   HOH B 471     6.175  10.034  27.212  1.00 29.38       O
HETATM14174  O   HOH B 472    19.197  30.731  -4.369  1.00 50.47       O
HETATM14175  O   HOH B 473     1.714   8.143  24.154  1.00 35.52       O
HETATM14176  O   HOH B 474    49.557   6.503  -6.723  1.00 48.18       O
HETATM14177  O   HOH B 475    46.423   3.337   2.699  1.00 43.40       O
HETATM14178  O   HOH B 476     6.422  37.164  -4.098  1.00 42.45       O
HETATM14179  O   HOH B 477    -0.824   2.747   8.307  1.00 41.34       O
HETATM14180  O   HOH B 478    19.871  25.623 -14.597  1.00 36.92       O
HETATM14181  O   HOH B 479    -7.358  26.649  17.639  1.00 35.06       O
HETATM14182  O   HOH B 480     4.929  23.382  -5.961  1.00 35.88       O
HETATM14183  O   HOH B 481    13.061  29.863   9.138  1.00 41.55       O
HETATM14184  O   HOH B 482    17.396  24.530 -14.524  1.00 36.29       O
HETATM14185  O   HOH B 483    -5.680   2.730   8.709  1.00 41.69       O
HETATM14186  O   HOH B 484     2.422  22.277  -4.447  1.00 33.54       O
HETATM14187  O   HOH B 485    19.595  12.393  -9.066  1.00 30.83       O
HETATM14188  O   HOH B 486    -3.088  28.530  32.366  1.00 38.79       O
HETATM14189  O   HOH B 487    16.355  19.392   2.554  1.00 40.56       O
HETATM14190  O   HOH B 488     8.805   6.904  15.737  1.00 45.96       O
HETATM14191  O   HOH B 489     3.985  -1.312  17.668  1.00 41.37       O
HETATM14192  O   HOH B 490     4.833   8.187  27.825  1.00 50.14       O
HETATM14193  O   HOH B 491   -12.501   7.286  16.989  1.00 34.78       O
HETATM14194  O   HOH B 492    -1.054  13.891  32.496  1.00 28.23       O
HETATM14195  O   HOH B 493    -4.238   8.526  30.998  1.00 58.30       O
HETATM14196  O   HOH B 494   -13.483  21.267  16.997  1.00 38.68       O
HETATM14197  O   HOH B 495     2.492  17.625  -2.216  1.00 33.30       O
HETATM14198  O   HOH B 496    19.039  13.471   2.038  1.00 41.66       O
HETATM14199  O   HOH B 497   -11.192   9.155  22.197  1.00 38.93       O
HETATM14200  O   HOH B 498    20.271   2.193  -5.923  1.00 31.59       O
HETATM14201  O   HOH B 499    -0.317  -0.527  12.122  1.00 44.13       O
HETATM14202  O   HOH B 500    16.299  18.747  10.132  1.00 41.00       O
HETATM14203  O   HOH B 501    15.981  12.821   1.777  1.00 40.95       O
HETATM14204  O   HOH B 502    -1.213  11.052  30.049  1.00 33.52       O
HETATM14205  O   HOH B 503    41.590   7.753   8.215  1.00 41.82       O
HETATM14206  O   HOH B 504    13.173  12.177   4.928  1.00 41.86       O
HETATM14207  O   HOH B 505    -9.264  14.616  30.344  1.00 45.63       O
HETATM14208  O   HOH B 506    25.460  27.056   1.679  1.00 44.28       O
HETATM14209  O   HOH B 507    -2.833  27.220  20.443  1.00 42.42       O
HETATM14210  O   HOH B 508    47.433   6.850 -12.445  1.00 52.45       O
HETATM14211  O   HOH B 509   -12.106   8.627   8.930  1.00 39.45       O
HETATM14212  O   HOH B 510    33.781   7.398   7.685  1.00 42.82       O
HETATM14213  O   HOH B 511    16.658  21.322   9.359  1.00 44.44       O
HETATM14214  O   HOH B 512    12.366  27.510  17.651  1.00 43.46       O
HETATM14215  O   HOH B 513    -3.512   5.298  23.325  1.00 37.62       O
HETATM14216  O   HOH B 514    31.733  -5.559  -2.934  1.00 38.18       O
HETATM14217  O   HOH B 515     8.497  31.254  12.439  1.00 43.82       O
HETATM14218  O   HOH B 516    -2.065   7.104  24.146  1.00 45.67       O
HETATM14219  O   HOH B 517    27.154  24.930  -6.033  1.00 34.35       O
HETATM14220  O   HOH B 518    36.509   7.557 -12.494  1.00 37.58       O
HETATM14221  O   HOH B 519     8.404   1.736  18.334  1.00 31.49       O
HETATM14222  O   HOH B 520    -1.830  29.212  28.356  1.00 43.50       O
HETATM14223  O   HOH B 521    18.743   8.155 -14.588  1.00 31.86       O
```

| | | | | | | | | | | |
|---|---|---|---|---|---|---|---|---|---|---|
| HETATM14224 | O | HOH | B | 522 | 17.892 | 29.856 | -2.089 | 1.00 | 45.28 | O |
| HETATM14225 | O | HOH | B | 523 | 25.078 | 5.189 | 3.721 | 1.00 | 26.40 | O |
| HETATM14226 | O | HOH | B | 524 | -0.150 | 33.518 | 9.895 | 1.00 | 55.57 | O |
| HETATM14227 | O | HOH | B | 525 | 15.902 | 14.503 | 9.317 | 1.00 | 35.83 | O |
| HETATM14228 | O | HOH | B | 526 | -2.989 | 23.175 | 35.600 | 1.00 | 47.53 | O |
| HETATM14229 | O | HOH | B | 527 | -7.805 | 16.571 | 4.254 | 1.00 | 36.41 | O |
| HETATM14230 | O | HOH | B | 528 | 6.279 | 31.449 | 8.856 | 1.00 | 48.89 | O |
| HETATM14231 | O | HOH | B | 529 | 30.682 | 8.657 | -19.926 | 1.00 | 39.54 | O |
| HETATM14232 | O | HOH | B | 530 | -4.139 | 31.241 | -1.313 | 1.00 | 46.73 | O |
| HETATM14233 | O | HOH | B | 531 | 15.431 | 27.615 | 5.857 | 1.00 | 36.72 | O |
| HETATM14234 | O | HOH | B | 532 | 10.228 | 2.362 | 13.198 | 1.00 | 51.21 | O |
| HETATM14235 | O | HOH | B | 533 | 23.935 | -3.846 | -3.000 | 1.00 | 40.31 | O |
| HETATM14236 | O | HOH | B | 534 | 18.843 | 6.635 | -1.305 | 1.00 | 38.99 | O |
| HETATM14237 | O | HOH | B | 535 | -4.045 | 20.550 | -2.163 | 1.00 | 38.21 | O |
| HETATM14238 | O | HOH | B | 536 | 49.164 | 1.497 | 3.638 | 1.00 | 45.68 | O |
| HETATM14239 | O | HOH | B | 537 | 16.674 | 16.574 | 4.104 | 1.00 | 34.80 | O |
| HETATM14240 | O | HOH | B | 538 | -3.615 | 7.083 | 2.498 | 1.00 | 48.60 | O |
| HETATM14241 | O | HOH | B | 539 | 31.200 | -3.070 | -1.124 | 1.00 | 38.13 | O |
| HETATM14242 | O | HOH | B | 540 | -15.449 | 19.473 | 30.895 | 1.00 | 48.98 | O |
| HETATM14243 | O | HOH | B | 541 | 49.913 | 5.386 | -4.303 | 1.00 | 55.81 | O |
| HETATM14244 | O | HOH | B | 542 | 42.883 | 5.972 | 4.535 | 1.00 | 33.30 | O |
| HETATM14245 | O | HOH | B | 543 | -16.850 | 20.969 | 19.666 | 1.00 | 48.83 | O |
| HETATM14246 | O | HOH | B | 544 | 45.831 | 2.902 | -2.329 | 1.00 | 42.93 | O |
| HETATM14247 | O | HOH | B | 545 | 25.309 | 7.531 | -14.656 | 1.00 | 53.30 | O |
| HETATM14248 | O | HOH | B | 546 | 19.444 | 33.127 | 6.782 | 1.00 | 42.58 | O |
| HETATM14249 | O | HOH | B | 547 | -1.768 | 8.508 | 27.692 | 1.00 | 45.53 | O |
| HETATM14250 | O | HOH | B | 548 | -3.604 | 8.106 | 25.827 | 1.00 | 36.68 | O |
| HETATM14251 | O | HOH | B | 549 | 34.868 | -6.554 | -11.054 | 1.00 | 47.00 | O |
| HETATM14252 | O | HOH | B | 550 | 28.793 | 9.370 | 7.501 | 1.00 | 42.26 | O |
| HETATM14253 | O | HOH | B | 551 | 31.710 | 2.914 | -16.105 | 1.00 | 43.49 | O |
| HETATM14254 | O | HOH | B | 552 | -8.434 | 18.520 | 33.103 | 1.00 | 46.49 | O |
| HETATM14255 | O | HOH | B | 553 | 12.575 | 9.526 | -7.483 | 1.00 | 47.41 | O |
| HETATM14256 | O | HOH | B | 554 | -10.378 | 13.095 | 6.352 | 1.00 | 42.95 | O |
| HETATM14257 | O | HOH | B | 555 | 45.447 | 4.735 | -6.633 | 1.00 | 47.51 | O |
| HETATM14258 | O | HOH | B | 556 | -10.349 | 8.177 | 20.107 | 1.00 | 41.84 | O |
| HETATM14259 | O | HOH | B | 557 | 20.479 | 3.437 | -2.924 | 1.00 | 39.31 | O |
| HETATM14260 | O | HOH | B | 558 | 2.788 | 29.589 | -7.068 | 1.00 | 49.10 | O |
| HETATM14261 | O | HOH | B | 559 | 26.207 | 22.669 | -13.256 | 1.00 | 43.15 | O |
| HETATM14262 | O | HOH | B | 560 | 19.112 | 6.097 | -5.654 | 1.00 | 45.11 | O |
| HETATM14263 | O | HOH | B | 561 | -8.951 | 26.618 | 11.164 | 1.00 | 47.24 | O |
| HETATM14264 | O | HOH | B | 562 | 45.025 | 5.990 | -4.337 | 1.00 | 51.27 | O |
| HETATM14265 | O | HOH | B | 563 | -18.194 | 15.375 | 19.814 | 1.00 | 48.09 | O |
| HETATM14266 | O | HOH | B | 564 | -10.882 | 16.249 | 5.941 | 1.00 | 37.97 | O |
| HETATM14267 | O | HOH | B | 565 | 46.368 | 12.801 | -12.874 | 1.00 | 46.70 | O |
| HETATM14268 | O | HOH | B | 566 | 22.668 | 31.267 | -4.508 | 1.00 | 41.20 | O |
| HETATM14269 | O | HOH | B | 567 | -1.915 | 34.800 | 7.132 | 1.00 | 51.65 | O |
| HETATM14270 | O | HOH | B | 568 | -10.159 | 4.843 | 18.061 | 1.00 | 49.44 | O |
| HETATM14271 | O | HOH | B | 569 | -2.674 | 25.791 | 33.197 | 1.00 | 40.33 | O |
| HETATM14272 | O | HOH | B | 570 | 6.797 | 12.547 | -1.238 | 1.00 | 42.23 | O |
| HETATM14273 | O | HOH | B | 571 | 5.372 | 38.299 | 1.735 | 1.00 | 47.95 | O |
| HETATM14274 | O | HOH | B | 572 | 9.459 | 13.747 | -3.309 | 1.00 | 41.96 | O |
| HETATM14275 | O | HOH | B | 573 | -5.508 | 29.325 | 4.072 | 1.00 | 55.47 | O |
| HETATM14276 | O | HOH | B | 574 | 10.970 | 11.741 | 3.753 | 1.00 | 41.89 | O |
| HETATM14277 | O | HOH | B | 575 | -9.828 | 24.331 | 18.840 | 1.00 | 38.36 | O |
| HETATM14278 | O | HOH | B | 576 | 16.064 | 23.209 | 2.281 | 1.00 | 40.81 | O |
| HETATM14279 | O | HOH | B | 577 | -7.344 | 23.959 | 0.527 | 1.00 | 48.29 | O |
| HETATM14280 | O | HOH | B | 578 | 14.719 | 7.385 | -4.690 | 1.00 | 46.29 | O |
| HETATM14281 | O | HOH | B | 579 | 15.372 | 23.426 | 5.118 | 1.00 | 43.08 | O |
| HETATM14282 | O | HOH | B | 580 | 28.965 | 30.633 | -2.065 | 1.00 | 54.44 | O |
| HETATM14283 | O | HOH | B | 581 | 9.957 | 11.460 | -4.721 | 1.00 | 43.64 | O |
| HETATM14284 | O | HOH | B | 582 | 4.268 | 32.202 | 7.708 | 1.00 | 55.00 | O |
| HETATM14285 | O | HOH | B | 583 | 43.676 | 3.853 | -10.600 | 1.00 | 47.07 | O |
| HETATM14286 | O | HOH | B | 584 | 25.245 | -2.072 | 5.115 | 1.00 | 52.26 | O |
| HETATM14287 | O | HOH | B | 585 | 13.585 | 33.021 | -10.306 | 1.00 | 51.01 | O |
| HETATM14288 | O | HOH | B | 586 | 14.216 | 15.857 | 4.465 | 1.00 | 42.06 | O |

| | | | | | | | | | | |
|---|---|---|---|---|---|---|---|---|---|---|
| HETATM14289 | O | HOH | B | 587 | 14.972 | 30.738 | 2.499 | 1.00 | 51.84 | O |
| HETATM14290 | O | HOH | B | 588 | 42.943 | -4.634 | -8.259 | 1.00 | 37.49 | O |
| HETATM14291 | O | HOH | B | 589 | 16.803 | 6.394 | -3.184 | 1.00 | 48.72 | O |
| HETATM14292 | O | HOH | B | 590 | 1.695 | -2.228 | 12.263 | 1.00 | 49.17 | O |
| HETATM14293 | O | HOH | B | 591 | -1.983 | -0.937 | 14.266 | 1.00 | 47.97 | O |
| HETATM14294 | O | HOH | B | 592 | 18.375 | 23.625 | 13.219 | 1.00 | 51.67 | O |
| HETATM14295 | O | HOH | B | 593 | 14.170 | 14.010 | 2.813 | 1.00 | 40.36 | O |
| HETATM14296 | O | HOH | B | 594 | 21.273 | -2.202 | 0.957 | 1.00 | 65.58 | O |
| HETATM14297 | O | HOH | B | 595 | 28.290 | 0.234 | 8.552 | 1.00 | 45.31 | O |
| HETATM14298 | O | HOH | B | 596 | 27.657 | 1.451 | -13.937 | 1.00 | 53.79 | O |
| HETATM14299 | O | HOH | B | 597 | 45.160 | 14.159 | -14.858 | 1.00 | 45.78 | O |
| HETATM14300 | O | HOH | B | 598 | -7.089 | 27.831 | 8.450 | 1.00 | 56.12 | O |
| HETATM14301 | O | HOH | B | 599 | 15.791 | 22.741 | 7.452 | 1.00 | 44.40 | O |
| HETATM14302 | O | HOH | B | 600 | -4.255 | 5.081 | 0.223 | 1.00 | 59.85 | O |
| HETATM14303 | O | HOH | B | 601 | -0.298 | 0.792 | 23.243 | 1.00 | 47.31 | O |
| HETATM14304 | O | HOH | B | 602 | -7.212 | 9.518 | 24.708 | 1.00 | 48.14 | O |
| HETATM14305 | O | HOH | B | 603 | -19.376 | 7.164 | 14.338 | 1.00 | 51.99 | O |
| HETATM14306 | O | HOH | B | 604 | 14.997 | 10.110 | -2.891 | 1.00 | 53.14 | O |
| HETATM14307 | O | HOH | B | 605 | 15.008 | 31.568 | -1.963 | 1.00 | 51.31 | O |
| HETATM14308 | O | HOH | B | 606 | 15.803 | 26.644 | -14.788 | 1.00 | 41.91 | O |
| HETATM14309 | O | HOH | B | 607 | 1.773 | 2.324 | 6.875 | 1.00 | 50.69 | O |
| HETATM14310 | O | HOH | B | 608 | 45.467 | 9.313 | -14.118 | 1.00 | 63.45 | O |
| HETATM14311 | O | HOH | B | 609 | -13.864 | 7.732 | 23.271 | 1.00 | 48.82 | O |
| HETATM14312 | O | HOH | B | 610 | 28.699 | 8.374 | -18.499 | 1.00 | 41.13 | O |
| HETATM14313 | O | HOH | B | 611 | -7.552 | 24.220 | 3.507 | 1.00 | 55.11 | O |
| HETATM14314 | O | HOH | B | 612 | -10.408 | 22.562 | 30.554 | 1.00 | 54.23 | O |
| HETATM14315 | O | HOH | B | 613 | -11.272 | 5.113 | 13.438 | 1.00 | 52.67 | O |
| HETATM14316 | O | HOH | B | 614 | 44.731 | 4.832 | -12.989 | 1.00 | 47.04 | O |
| HETATM14317 | O | HOH | B | 615 | 6.586 | 29.847 | 16.069 | 1.00 | 46.24 | O |
| HETATM14318 | O | HOH | B | 616 | 14.324 | 30.427 | 4.751 | 1.00 | 48.06 | O |
| HETATM14319 | O | HOH | B | 617 | -15.093 | 10.706 | 11.901 | 1.00 | 52.47 | O |
| HETATM14320 | O | HOH | B | 618 | 30.972 | 1.494 | 10.126 | 1.00 | 43.80 | O |
| HETATM14321 | O | HOH | B | 619 | -1.040 | -1.404 | 16.603 | 1.00 | 54.77 | O |
| HETATM14322 | O | HOH | B | 620 | 49.713 | 2.205 | -4.477 | 1.00 | 56.18 | O |
| HETATM14323 | O | HOH | B | 621 | 35.766 | -11.777 | -8.383 | 1.00 | 45.63 | O |
| HETATM14324 | O | HOH | B | 622 | -8.157 | 11.264 | 3.044 | 1.00 | 49.42 | O |
| HETATM14325 | O | HOH | B | 623 | 46.854 | 1.594 | -4.499 | 1.00 | 63.18 | O |
| HETATM14326 | O | HOH | B | 624 | 2.328 | 12.895 | -1.857 | 1.00 | 47.35 | O |
| HETATM14327 | O | HOH | B | 625 | -8.173 | 17.274 | 30.581 | 1.00 | 44.50 | O |
| HETATM14328 | O | HOH | B | 626 | 28.638 | 13.395 | 7.871 | 1.00 | 47.50 | O |
| HETATM14329 | O | HOH | B | 627 | 27.246 | 34.090 | -10.353 | 1.00 | 53.11 | O |
| HETATM14330 | O | HOH | B | 628 | -16.218 | 14.295 | 15.716 | 1.00 | 29.46 | O |
| HETATM14331 | O | HOH | B | 629 | -11.988 | 24.341 | 34.839 | 1.00 | 53.52 | O |
| HETATM14332 | O | HOH | B | 630 | -7.770 | 28.463 | 13.281 | 1.00 | 54.73 | O |
| HETATM14333 | O | HOH | B | 631 | 27.016 | -4.658 | -2.156 | 1.00 | 51.12 | O |
| HETATM14334 | O | HOH | B | 632 | 7.961 | 9.050 | 2.066 | 1.00 | 49.27 | O |
| HETATM14335 | O | HOH | B | 633 | 11.161 | 35.144 | 8.442 | 1.00 | 52.26 | O |
| HETATM14336 | O | HOH | B | 634 | 35.718 | -0.944 | -15.351 | 1.00 | 51.55 | O |
| HETATM14337 | O | HOH | B | 635 | -0.690 | 15.151 | 0.577 | 1.00 | 57.55 | O |
| HETATM14338 | O | HOH | B | 636 | -2.156 | 34.059 | 4.201 | 1.00 | 51.84 | O |
| HETATM14339 | O | HOH | B | 637 | 19.776 | 15.959 | 2.833 | 1.00 | 58.63 | O |
| HETATM14340 | O | HOH | B | 638 | -3.102 | 32.285 | 0.470 | 1.00 | 53.93 | O |
| HETATM14341 | O | HOH | B | 639 | 9.654 | 6.356 | 8.923 | 1.00 | 52.43 | O |
| HETATM14342 | O | HOH | B | 640 | 9.191 | 26.301 | -9.090 | 1.00 | 48.20 | O |
| HETATM14343 | O | HOH | B | 641 | 23.392 | 32.143 | -2.037 | 1.00 | 50.24 | O |
| HETATM14344 | O | HOH | B | 642 | -14.528 | 26.416 | 26.306 | 1.00 | 43.35 | O |
| HETATM14345 | O | HOH | B | 643 | -3.364 | 4.479 | 4.626 | 1.00 | 50.80 | O |
| HETATM14346 | O | HOH | B | 644 | 11.312 | 11.088 | -2.456 | 1.00 | 46.47 | O |
| HETATM14347 | O | HOH | B | 645 | 21.547 | 21.063 | -14.051 | 1.00 | 50.77 | O |
| HETATM14348 | O | HOH | B | 646 | -15.763 | 9.387 | 19.100 | 1.00 | 42.02 | O |
| HETATM14349 | O | HOH | B | 647 | 8.638 | 35.409 | 0.075 | 1.00 | 30.26 | O |
| HETATM14350 | O | HOH | B | 648 | 10.402 | 36.040 | 0.518 | 1.00 | 28.82 | O |
| HETATM14351 | O | HOH | C | 215 | 25.940 | 41.961 | -5.406 | 1.00 | 10.74 | O |
| HETATM14352 | O | HOH | C | 216 | 8.618 | 50.216 | 9.752 | 1.00 | 11.69 | O |
| HETATM14353 | O | HOH | C | 217 | 13.848 | 50.321 | 14.256 | 1.00 | 12.75 | O |

```
HETATM14354  O   HOH C 218   28.055 40.080   1.514 1.00 17.32       O
HETATM14355  O   HOH C 219   18.670 42.150  -6.995 1.00 18.48       O
HETATM14356  O   HOH C 220   12.570 39.777  -4.458 1.00 15.51       O
HETATM14357  O   HOH C 221   14.125 55.677  -9.632 1.00 18.52       O
HETATM14358  O   HOH C 222   29.324 49.111   8.395 1.00 18.42       O
HETATM14359  O   HOH C 223    4.213 40.402 -34.741 1.00 23.63       O
HETATM14360  O   HOH C 224   20.647 52.666  12.527 1.00 13.00       O
HETATM14361  O   HOH C 225   20.414 50.473  15.066 1.00 16.49       O
HETATM14362  O   HOH C 226   27.910 46.498   1.291 1.00 20.56       O
HETATM14363  O   HOH C 227  -11.610 39.413 -39.605 1.00 20.07       O
HETATM14364  O   HOH C 228   10.449 45.303  11.459 1.00 17.44       O
HETATM14365  O   HOH C 229    4.234 47.879   8.487 1.00 15.89       O
HETATM14366  O   HOH C 230   18.227 36.374   3.909 1.00 14.93       O
HETATM14367  O   HOH C 231   12.226 55.997   9.368 1.00 13.38       O
HETATM14368  O · HOH C 232    7.854 52.723  10.257 1.00 18.37       O
HETATM14369  O   HOH C 233   10.687 42.077   8.251 1.00 20.65       O
HETATM14370  O   HOH C 234   21.931 50.323  18.579 1.00 16.47       O
HETATM14371  O   HOH C 235   15.653 52.486  13.947 1.00 17.68       O
HETATM14372  O   HOH C 236   11.920 54.192  12.549 1.00 20.24       O
HETATM14373  O   HOH C 237    2.803 35.575 -43.230 1.00 28.97       O
HETATM14374  O   HOH C 238    5.630 55.756   7.053 1.00 18.10       O
HETATM14375  O   HOH C 239    0.940 48.608 -38.541 1.00 27.54       O
HETATM14376  O   HOH C 240   15.560 38.880  -2.187 1.00 13.97       O
HETATM14377  O   HOH C 241  -14.354 42.494 -22.040 1.00 30.11       O
HETATM14378  O   HOH C 242    8.774 36.400  -3.614 1.00 20.01       O
HETATM14379  O   HOH C 243   10.869 41.997 -45.856 1.00 21.41       O
HETATM14380  O   HOH C 244   20.843 62.238   1.583 1.00 21.30       O
HETATM14381  O   HOH C 245    3.809 49.106 -40.649 1.00 24.97       O
HETATM14382  O   HOH C 246    9.137 54.038  12.424 1.00 16.75       O
HETATM14383  O   HOH C 247   25.598 47.532  18.794 1.00 26.74       O
HETATM14384  O   HOH C 248    6.608 55.667 -10.108 1.00 26.59       O
HETATM14385  O   HOH C 249   21.875 54.010  16.800 1.00 25.46       O
HETATM14386  O   HOH C 250    8.824 51.976  14.139 1.00 24.01       O
HETATM14387  O   HOH C 251  -13.031 50.790 -51.385 1.00 28.88       O
HETATM14388  O   HOH C 252   11.725 39.239   3.961 1.00 27.07       O
HETATM14389  O   HOH C 253   21.024 38.981  -1.586 1.00 18.90       O
HETATM14390  O   HOH C 254   19.490 57.745  -9.232 1.00 25.34       O
HETATM14391  O   HOH C 255    1.056 40.367 -52.746 1.00 26.70       O
HETATM14392  O   HOH C 256    9.019 42.448   0.581 1.00 26.76       O
HETATM14393  O   HOH C 257    1.438 46.464   2.074 1.00 33.38       O
HETATM14394  O   HOH C 258   26.326 55.311   4.152 1.00 25.70       O
HETATM14395  O   HOH C 259    3.431 50.541 -50.055 1.00 20.82       O
HETATM14396  O   HOH C 260   30.384 45.359  14.297 1.00 27.50       O
HETATM14397  O   HOH C 261   18.644 52.356  14.326 1.00 20.97       O
HETATM14398  O   HOH C 262   19.966 69.217   0.563 1.00 24.79       O
HETATM14399  O   HOH C 263   11.493 39.705   8.386 1.00 28.14       O
HETATM14400  O   HOH C 264    9.774 44.182 -43.747 1.00 23.70       O
HETATM14401  O   HOH C 265   15.157 60.937   8.479 1.00 23.12       O
HETATM14402  O   HOH C 266   10.486 42.843  10.946 1.00 25.36       O
HETATM14403  O   HOH C 267   -5.958 52.591 -27.861 1.00 28.64       O
HETATM14404  O   HOH C 268    1.454 60.369  -7.518 1.00 31.21       O
HETATM14405  O   HOH C 269   -3.606 52.155   5.776 1.00 25.95       O
HETATM14406  O   HOH C 270  -10.701 38.471 -26.752 1.00 29.79       O
HETATM14407  O   HOH C 271    0.510 53.831 -10.116 1.00 21.75       O
HETATM14408  O   HOH C 272   12.141 42.296  -8.484 1.00 25.95       O
HETATM14409  O   HOH C 273    3.101 44.289 -29.933 1.00 24.32       O
HETATM14410  O   HOH C 274   12.879 37.820  -6.259 1.00 28.41       O
HETATM14411  O   HOH C 275    1.042 51.166 -18.164 1.00 29.95       O
HETATM14412  O   HOH C 276   24.820 37.581  12.397 1.00 24.76       O
HETATM14413  O   HOH C 277    6.752 42.530   1.454 1.00 36.38       O
HETATM14414  O   HOH C 278   26.927 43.848  11.601 1.00 34.97       O
HETATM14415  O   HOH C 279    3.273 48.296  -9.978 1.00 24.54       O
HETATM14416  O   HOH C 280   16.889 43.639 -10.189 1.00 37.44       O
HETATM14417  O   HOH C 281   19.795 36.400   9.989 1.00 31.81       O
HETATM14418  O   HOH C 282   -1.217 52.363 -18.937 1.00 27.15       O
```

```
HETATM14419  O   HOH C 283      8.101  58.690  -4.689  1.00 37.87          O
HETATM14420  O   HOH C 284     -0.312  52.593 -39.834  1.00 34.61          O
HETATM14421  O   HOH C 285      3.371  48.512 -51.303  1.00 34.04          O
HETATM14422  O   HOH C 286     12.042  36.242  -1.980  1.00 24.24          O
HETATM14423  O   HOH C 287    -11.865  49.592 -42.886  1.00 28.78          O
HETATM14424  O   HOH C 288     -6.649  53.593 -35.004  1.00 27.45          O
HETATM14425  O   HOH C 289     -0.698  37.889 -52.278  1.00 34.93          O
HETATM14426  O   HOH C 290     17.986  59.997  -3.093  1.00 26.15          O
HETATM14427  O   HOH C 291     17.127  57.465 -13.540  1.00 34.32          O
HETATM14428  O   HOH C 292     10.607  58.824  -3.779  1.00 46.25          O
HETATM14429  O   HOH C 293     18.331  61.930   7.028  1.00 28.44          O
HETATM14430  O   HOH C 294     16.614  58.747  -9.672  1.00 40.31          O
HETATM14431  O   HOH C 295     14.952  36.557   9.861  1.00 29.36          O
HETATM14432  O   HOH C 296     18.826  37.086  -2.571  1.00 23.92          O
HETATM14433  O   HOH C 297     18.801  54.689 -15.852  1.00 30.08          O
HETATM14434  O   HOH C 298      8.381  43.540 -41.636  1.00 24.12          O
HETATM14435  O   HOH C 299    -10.310  50.947 -38.390  1.00 32.13          O
HETATM14436  O   HOH C 300      3.725  37.491 -27.318  1.00 33.84          O
HETATM14437  O   HOH C 301     -9.122  51.761 -34.012  1.00 27.62          O
HETATM14438  O   HOH C 302     -2.599  59.150  -2.746  1.00 38.48          O
HETATM14439  O   HOH C 303      6.085  50.421 -19.089  1.00 41.40          O
HETATM14440  O   HOH C 304      9.880  42.389  -8.383  1.00 33.61          O
HETATM14441  O   HOH C 305     -1.701  59.673  -6.477  1.00 35.43          O
HETATM14442  O   HOH C 306    -20.611  41.994 -18.469  1.00 43.48          O
HETATM14443  O   HOH C 307     15.931  40.888  -8.865  1.00 31.95          O
HETATM14444  O   HOH C 308     35.285  40.949   2.489  1.00 33.05          O
HETATM14445  O   HOH C 309     25.364  34.930   5.493  1.00 34.39          O
HETATM14446  O   HOH C 310     -3.274  38.369 -19.181  1.00 38.50          O
HETATM14447  O   HOH C 311    -14.092  44.883 -21.392  1.00 27.49          O
HETATM14448  O   HOH C 312     21.347  55.083  13.667  1.00 26.33          O
HETATM14449  O   HOH C 313     10.425  49.961 -11.657  1.00 28.56          O
HETATM14450  O   HOH C 314     17.289  61.256  10.132  1.00 40.49          O
HETATM14451  O   HOH C 315     -3.486  48.115 -46.084  1.00 27.47          O
HETATM14452  O   HOH C 316     16.685  67.266  -1.840  1.00 44.55          O
HETATM14453  O   HOH C 317     14.618  59.085  11.309  1.00 34.79          O
HETATM14454  O   HOH C 318    -10.884  38.487 -48.545  1.00 37.41          O
HETATM14455  O   HOH C 319      5.073  41.858 -21.654  1.00 32.14          O
HETATM14456  O   HOH C 320     23.387  57.744  10.977  1.00 37.37          O
HETATM14457  O   HOH C 321    -12.313  51.329 -27.200  1.00 26.10          O
HETATM14458  O   HOH C 322     -4.809  48.711   0.656  1.00 33.28          O
HETATM14459  O   HOH C 323      0.417  60.992  -0.212  1.00 38.28          O
HETATM14460  O   HOH C 324      4.918  58.071 -43.588  1.00 29.51          O
HETATM14461  O   HOH C 325     -0.578  36.633 -24.716  1.00 36.27          O
HETATM14462  O   HOH C 326     19.432  40.042  -5.417  1.00 37.20          O
HETATM14463  O   HOH C 327     23.823  37.328  -1.903  1.00 41.43          O
HETATM14464  O   HOH C 328     14.738  50.745 -14.962  1.00 34.63          O
HETATM14465  O   HOH C 329     17.924  70.889   2.032  1.00 47.27          O
HETATM14466  O   HOH C 330      1.334  45.685 -54.108  1.00 50.76          O
HETATM14467  O   HOH C 331     30.828  41.004   8.078  1.00 30.80          O
HETATM14468  O   HOH C 332     10.871  38.675   6.105  1.00 34.38          O
HETATM14469  O   HOH C 333     -3.824  56.345  -9.845  1.00 32.72          O
HETATM14470  O   HOH C 334      1.875  35.682 -28.180  1.00 43.61          O
HETATM14471  O   HOH C 335     -3.741  44.066 -11.796  1.00 36.77          O
HETATM14472  O   HOH C 336     22.510  41.789  16.197  1.00 29.12          O
HETATM14473  O   HOH C 337     14.064  63.901   3.616  1.00 37.15          O
HETATM14474  O   HOH C 338      4.916  55.262 -15.835  1.00 45.38          O
HETATM14475  O   HOH C 339     18.196  39.464  15.013  1.00 37.89          O
HETATM14476  O   HOH C 340      7.910  43.054   8.297  1.00 28.67          O
HETATM14477  O   HOH C 341    -11.734  51.784 -53.337  1.00 35.75          O
HETATM14478  O   HOH C 342     12.455  56.498  13.481  1.00 32.07          O
HETATM14479  O   HOH C 343     19.076  41.687  15.654  1.00 35.92          O
HETATM14480  O   HOH C 344     -1.400  28.288 -33.027  1.00 34.74          O
HETATM14481  O   HOH C 345     32.575  50.596  17.167  1.00 35.54          O
HETATM14482  O   HOH C 346     15.497  36.812  -3.539  1.00 34.59          O
HETATM14483  O   HOH C 347      3.206  33.573 -45.294  1.00 47.79          O
```

```
HETATM14484   O    HOH C 348      -7.332  43.664 -53.740  1.00 40.47         O
HETATM14485   O    HOH C 349     -11.116  51.491 -35.969  1.00 31.54         O
HETATM14486   O    HOH C 350       4.749  43.821   0.775  1.00 39.78         O
HETATM14487   O    HOH C 351      25.106  37.868  15.029  1.00 38.13         O
HETATM14488   O    HOH C 352      12.484  48.902 -12.936  1.00 47.93         O
HETATM14489   O    HOH C 353       5.492  52.763 -16.329  1.00 50.29         O
HETATM14490   O    HOH C 354       2.169  44.147   3.485  1.00 45.26         O
HETATM14491   O    HOH C 355      -5.658  35.232 -31.901  1.00 37.75         O
HETATM14492   O    HOH C 356     -13.067  49.694 -35.642  1.00 34.41         O
HETATM14493   O    HOH C 357       1.852  43.172  -7.831  1.00 39.14         O
HETATM14494   O    HOH C 358      -1.645  49.948 -45.740  1.00 28.12         O
HETATM14495   O    HOH C 359       2.219  45.771 -10.938  1.00 41.64         O
HETATM14496   O    HOH C 360      14.035  47.288 -11.236  1.00 39.15         O
HETATM14497   O    HOH C 361       6.131  54.816 -47.985  1.00 41.58         O
HETATM14498   O    HOH C 362      12.280  39.270  -8.906  1.00 44.19         O
HETATM14499   O    HOH C 363       9.063  59.476   8.126  1.00 34.64         O
HETATM14500   O    HOH C 364      -8.568  33.723 -42.679  1.00 45.54         O
HETATM14501   O    HOH C 365      18.503  62.358  -2.031  1.00 30.67         O
HETATM14502   O    HOH C 366      16.859  36.087   8.399  1.00 38.86         O
HETATM14503   O    HOH C 367      -8.425  54.055 -17.908  1.00 42.19         O
HETATM14504   O    HOH C 368       7.051  52.932 -49.549  1.00 35.48         O
HETATM14505   O    HOH C 369      25.075  33.287   3.445  1.00 47.71         O
HETATM14506   O    HOH C 370      27.013  40.475  11.363  1.00 36.31         O
HETATM14507   O    HOH C 371     -16.841  46.538 -29.111  1.00 31.41         O
HETATM14508   O    HOH C 372      10.626  61.910   8.299  1.00 45.77         O
HETATM14509   O    HOH C 373      -2.953  56.391 -22.897  1.00 50.18         O
HETATM14510   O    HOH C 374       4.445  63.433  -1.204  1.00 56.03         O
HETATM14511   O    HOH C 375      -6.545  50.365 -44.904  1.00 34.63         O
HETATM14512   O    HOH C 376      -9.696  38.101 -19.496  1.00 47.44         O
HETATM14513   O    HOH C 377       8.875  42.286 -10.777  1.00 45.96         O
HETATM14514   O    HOH C 378     -17.695  46.179 -26.624  1.00 39.29         O
HETATM14515   O    HOH C 379       0.826  51.357 -49.554  1.00 35.73         O
HETATM14516   O    HOH C 380      29.215  44.540  11.540  1.00 31.78         O
HETATM14517   O    HOH C 381       8.457  56.650  11.984  1.00 33.32         O
HETATM14518   O    HOH C 382       6.048  57.404  10.917  1.00 37.71         O
HETATM14519   O    HOH C 383       2.154  54.516 -44.186  1.00 44.02         O
HETATM14520   O    HOH C 384      -2.986  45.827 -50.091  1.00 40.58         O
HETATM14521   O    HOH C 385      -1.362  55.739   4.976  1.00 37.17         O
HETATM14522   O    HOH C 386      -6.327  56.078  -9.767  1.00 39.56         O
HETATM14523   O    HOH C 387      22.256  36.412  11.868  1.00 45.32         O
HETATM14524   O    HOH C 388      10.315  58.057  10.132  1.00 39.99         O
HETATM14525   O    HOH C 389      17.129  35.635  -1.023  1.00 34.38         O
HETATM14526   O    HOH C 390      27.072  37.183  -3.542  1.00 41.96         O
HETATM14527   O    HOH C 391      -2.336  57.124 -19.842  1.00 42.27         O
HETATM14528   O    HOH C 392      20.729  35.164  -3.371  1.00 32.73         O
HETATM14529   O    HOH C 393       2.668  32.672 -51.728  1.00 35.93         O
HETATM14530   O    HOH C 394       6.886  39.741 -42.784  1.00 37.64         O
HETATM14531   O    HOH C 395      -5.699  40.569 -13.119  1.00 55.79         O
HETATM14532   O    HOH C 396      20.438  32.541  -2.049  1.00 40.78         O
HETATM14533   O    HOH C 397       5.914  58.216   8.250  1.00 37.45         O
HETATM14534   O    HOH C 398      21.319  65.698   4.904  1.00 48.89         O
HETATM14535   O    HOH C 399     -13.490  44.885 -40.957  1.00 37.10         O
HETATM14536   O    HOH C 400       0.902  47.254 -51.707  1.00 39.83         O
HETATM14537   O    HOH C 401       3.254  53.034 -51.763  1.00 46.94         O
HETATM14538   O    HOH C 402       2.729  33.430 -31.946  1.00 54.26         O
HETATM14539   O    HOH C 403      20.925  33.551   8.823  1.00 39.67         O
HETATM14540   O    HOH C 404      27.062  40.674  14.592  1.00 47.32         O
HETATM14541   O    HOH C 405     -16.090  46.643 -21.712  1.00 43.58         O
HETATM14542   O    HOH C 406      -1.933  60.627  -0.759  1.00 43.92         O
HETATM14543   O    HOH C 407      -9.344  36.539 -26.112  1.00 37.08         O
HETATM14544   O    HOH C 408       8.313  41.222 -41.024  1.00 35.44         O
HETATM14545   O    HOH C 409       4.203  48.053 -12.401  1.00 40.46         O
HETATM14546   O    HOH C 410      29.484  36.715   3.988  1.00 57.08         O
HETATM14547   O    HOH C 411     -12.774  37.475 -28.234  1.00 40.74         O
HETATM14548   O    HOH C 412       9.636  65.756   0.193  1.00 46.11         O
```

```
HETATM14549  O   HOH C 413     32.506  38.348    2.686  1.00 47.58           O
HETATM14550  O   HOH C 414     13.705  43.563  -10.165  1.00 57.81           O
HETATM14551  O   HOH C 415      8.458  48.572  -11.849  1.00 40.03           O
HETATM14552  O   HOH C 416     25.463  34.096   -0.862  1.00 49.91           O
HETATM14553  O   HOH C 417    -14.604  38.819  -19.287  1.00 51.58           O
HETATM14554  O   HOH C 418     -9.781  52.098  -42.995  1.00 56.06           O
HETATM14555  O   HOH C 419      6.001  50.032  -11.980  1.00 49.23           O
HETATM14556  O   HOH C 420    -14.548  51.050  -17.592  1.00 45.73           O
HETATM14557  O   HOH C 421     -9.333  52.834  -21.041  1.00 40.16           O
HETATM14558  O   HOH C 422     21.654  57.487   12.818  1.00 53.45           O
HETATM14559  O   HOH C 423    -10.013  47.316   -8.915  1.00 41.31           O
HETATM14560  O   HOH C 424     -5.681  49.445  -46.989  1.00 39.84           O
HETATM14561  O   HOH C 425     31.721  53.474   13.406  1.00 42.03           O
HETATM14562  O   HOH C 426     -6.984  32.979  -30.422  1.00 49.16           O
HETATM14563  O   HOH C 427     -8.718  49.181  -52.607  1.00 41.92           O
HETATM14564  O   HOH C 428     -4.527  58.520  -22.640  1.00 47.28           O
HETATM14565  O   HOH C 429      9.618  46.243  -13.602  1.00 50.01           O
HETATM14566  O   HOH C 430     27.742  48.219   20.871  1.00 40.01           O
HETATM14567  O   HOH C 431      4.113  34.961  -48.044  1.00 52.74           O
HETATM14568  O   HOH C 432      9.770  39.555    2.257  1.00 48.25           O
HETATM14569  O   HOH C 433     -6.484  55.948  -23.699  1.00 53.62           O
HETATM14570  O   HOH C 434     31.995  35.988   -2.350  1.00 43.37           O
HETATM14571  O   HOH C 435     16.150  38.357   -7.979  1.00 51.90           O
HETATM14572  O   HOH C 436      3.031  44.154   -5.607  1.00 48.85           O
HETATM14573  O   HOH C 437     22.807  61.755    8.356  1.00 41.27           O
HETATM14574  O   HOH C 438      2.382  62.671   -6.613  1.00 49.63           O
HETATM14575  O   HOH C 439      7.778  65.478    3.728  1.00 43.85           O
HETATM14576  O   HOH C 440      6.276  44.936  -18.512  1.00 50.44           O
HETATM14577  O   HOH C 441     -0.655  39.955  -17.658  1.00 45.83           O
HETATM14578  O   HOH C 442    -16.072  49.056  -22.231  1.00 41.99           O
HETATM14579  O   HOH C 443      1.710  60.704  -10.008  1.00 47.50           O
HETATM14580  O   HOH C 444    -13.730  41.620  -39.244  1.00 53.64           O
HETATM14581  O   HOH C 445    -17.561  44.973  -23.927  1.00 50.56           O
HETATM14582  O   HOH C 446      8.717  45.778  -17.996  1.00 46.46           O
HETATM14583  O   HOH C 447      2.923  31.485  -37.989  1.00 52.08           O.
HETATM14584  O   HOH C 448     14.911  66.640    2.797  1.00 48.02           O
HETATM14585  O   HOH C 449     -0.600  51.709  -47.503  1.00 35.89           O
HETATM14586  O   HOH C 450     -8.210  56.944  -15.008  1.00 52.49           O
HETATM14587  O   HOH C 451      0.614  53.210  -51.421  1.00 47.42           O
HETATM14588  O   HOH C 452     26.021  43.122   16.408  1.00 53.52           O
HETATM14589  O   HOH C 453     -7.711  49.510    1.618  1.00 48.86           O
HETATM14590  O   HOH C 454     -3.345  49.676    5.539  1.00 52.94           O
HETATM14591  O   HOH C 455     16.254  59.535  -12.204  1.00 56.60           O
HETATM14592  O   HOH C 456     -6.998  39.386  -52.227  1.00 39.16           O
HETATM14593  O   HOH C 457      4.772  34.576  -33.278  1.00 54.65           O
HETATM14594  O   HOH C 458     -5.021  59.004    2.404  1.00 55.76           O
HETATM14595  O   HOH C 459     24.605  59.997    9.379  1.00 37.91           O
HETATM14596  O   HOH C 460      2.254  53.245  -18.782  1.00 51.67           O
HETATM14597  O   HOH C 461     -3.874  58.837   -8.834  1.00 46.21           O
HETATM14598  O   HOH C 462     21.885  37.655   14.509  1.00 44.48           O
HETATM14599  O   HOH C 463     -7.028  34.239  -49.983  1.00 47.50           O
HETATM14600  O   HOH C 464     25.244  44.803   18.891  1.00 50.15           O
HETATM14601  O   HOH C 465      5.113  65.645    3.909  1.00 64.10           O
HETATM14602  O   HOH C 466     13.642  55.606  -19.198  1.00 50.76           O
HETATM14603  O   HOH C 467     18.210  56.286   12.536  1.00 45.99           O
HETATM14604  O   HOH C 468      5.947  42.759  -19.584  1.00 51.66           O
HETATM14605  O   HOH C 469     -6.971  53.321   -5.329  1.00 42.00           O
HETATM14606  O   HOH C 470     16.060  57.371   13.212  1.00 45.37           O
HETATM14607  O   HOH C 471     13.087  59.631   -9.191  1.00 62.79           O
HETATM14608  O   HOH C 472     28.017  55.423   16.593  1.00 45.58           O
HETATM14609  O   HOH C 473     -2.525  47.917  -48.894  1.00 45.35           O
HETATM14610  O   HOH C 474     -5.915  33.487  -43.000  1.00 48.77           O
HETATM14611  O   HOH C 475      8.569  56.639  -13.582  1.00 45.32           O
HETATM14612  O   HOH C 476     -3.211  49.372    2.540  1.00 48.54           O
HETATM14613  O   HOH C 477    -17.167  48.669  -26.489  1.00 60.28           O
```

```
HETATM14614  O   HOH C 478     -9.457  40.066 -17.213  1.00 43.85          O
HETATM14615  O   HOH C 479     29.888  33.132  -0.155  1.00 46.22          O
HETATM14616  O   HOH C 480     18.140  60.490  -5.709  1.00 56.69          O
HETATM14617  O   HOH C 481     -0.516  42.405 -11.227  1.00 54.07          O
HETATM14618  O   HOH C 482     -0.723  49.289 -50.403  1.00 40.35          O
HETATM14619  O   HOH C 483     -5.828  58.902  -1.762  1.00 56.44          O
HETATM14620  O   HOH C 484     19.205  31.757  -0.051  1.00 47.92          O
HETATM14621  O   HOH C 485     18.666  37.169  17.557  1.00 63.15          O
HETATM14622  O   HOH C 486     32.159  55.295  11.714  1.00 57.46          O
HETATM14623  O   HOH C 487    -13.985  40.496  -9.027  1.00 64.33          O
HETATM14624  O   HOH C 488      9.715  39.546 -10.125  1.00 49.94          O
HETATM14625  O   HOH C 489     -5.096  56.480   5.169  1.00 50.95          O
HETATM14626  O   HOH C 490      6.255  61.935  -3.845  1.00 61.14          O
HETATM14627  O   HOH C 491     24.886  46.210  -0.667  1.00 49.11          O
HETATM14628  O   HOH C 492     21.263  63.379   4.248  1.00 52.53          O
HETATM14629  O   HOH C 493      7.165  59.272 -10.612  1.00 43.38          O
HETATM14630  O   HOH C 494      5.485  36.967 -31.265  1.00 52.27          O
HETATM14631  O   HOH C 495     28.782  44.095  17.737  1.00 43.01          O
HETATM14632  O   HOH C 496     14.491  56.712  15.129  1.00 33.52          O
HETATM14633  O   HOH D 311     42.753  38.924 -19.751  1.00 55.41          O
HETATM14634  O   HOH D 312     23.247  41.317  -4.863  1.00  9.85          O
HETATM14635  O   HOH D 313     40.232  49.586 -15.986  1.00 15.73          O
HETATM14636  O   HOH D 314     43.318  53.124 -10.856  1.00 17.26          O
HETATM14637  O   HOH D 315     35.942  32.428 -23.015  1.00 12.91          O
HETATM14638  O   HOH D 316     31.150  56.785  -2.697  1.00 12.86          O
HETATM14639  O   HOH D 317     27.498  56.574   1.726  1.00 14.32          O
HETATM14640  O   HOH D 318     21.288  49.070 -28.163  1.00 13.32          O
HETATM14641  O   HOH D 319     15.616  46.851 -39.482  1.00 16.77          O
HETATM14642  O   HOH D 320     34.521  43.191   3.661  1.00 13.99          O
HETATM14643  O   HOH D 321     33.303  33.398 -22.879  1.00 20.36          O
HETATM14644  O   HOH D 322     21.369  58.428  -7.500  1.00 18.95          O
HETATM14645  O   HOH D 323     38.773  39.228 -24.290  1.00 14.85          O
HETATM14646  O   HOH D 324     27.363  51.686 -33.170  1.00 14.95          O
HETATM14647  O   HOH D 325     23.101  46.266 -25.237  1.00 15.12          O
HETATM14648  O   HOH D 326     23.610  48.565 -26.709  1.00 15.88          O
HETATM14649  O   HOH D 327     19.224  42.372 -26.284  1.00 21.02          O
HETATM14650  O   HOH D 328     31.563  46.729 -28.837  1.00 15.12          O
HETATM14651  O   HOH D 329     20.719  58.495 -11.672  1.00 23.73          O
HETATM14652  O   HOH D 330     28.149  40.330  -6.497  1.00 17.01          O
HETATM14653  O   HOH D 331     31.862  37.864  -4.281  1.00 23.88          O
HETATM14654  O   HOH D 332     26.210  54.125 -28.740  1.00 18.36          O
HETATM14655  O   HOH D 333     39.153  51.965   1.895  1.00 18.97          O
HETATM14656  O   HOH D 334     20.674  57.820  -4.911  1.00 19.94          O
HETATM14657  O   HOH D 335     23.389  51.979 -23.206  1.00 19.23          O
HETATM14658  O   HOH D 336     38.950  64.260  -8.721  1.00 18.41          O
HETATM14659  O   HOH D 337     12.028  47.083 -23.784  1.00 24.91          O
HETATM14660  O   HOH D 338     41.458  58.827  -4.887  1.00 16.73          O
HETATM14661  O   HOH D 339     15.512  44.230 -39.549  1.00 22.80          O
HETATM14662  O   HOH D 340     29.583  58.472   5.334  1.00 24.28          O
HETATM14663  O   HOH D 341     38.728  34.909 -25.376  1.00 19.71          O
HETATM14664  O   HOH D 342     17.685  53.892 -34.582  1.00 19.80          O
HETATM14665  O   HOH D 343     25.392  51.597 -37.478  1.00 17.92          O
HETATM14666  O   HOH D 344     15.646  51.990 -40.482  1.00 20.39          O
HETATM14667  O   HOH D 345     17.597  52.702 -37.198  1.00 19.03          O
HETATM14668  O   HOH D 346     27.221  32.797 -19.534  1.00 23.61          O
HETATM14669  O   HOH D 347     23.019  38.422 -18.227  1.00 20.49          O
HETATM14670  O   HOH D 348     42.688  45.497  -9.758  1.00 21.60          O
HETATM14671  O   HOH D 349     22.157  36.654 -27.632  1.00 23.31          O
HETATM14672  O   HOH D 350     27.972  39.162 -32.428  1.00 26.62          O
HETATM14673  O   HOH D 351     30.908  58.790 -19.670  1.00 22.80          O
HETATM14674  O   HOH D 352     29.201  63.699 -14.914  1.00 19.25          O
HETATM14675  O   HOH D 353     22.961  41.079 -12.983  1.00 22.69          O
HETATM14676  O   HOH D 354     33.710  67.259  -2.853  1.00 18.27          O
HETATM14677  O   HOH D 355     14.514  49.443 -24.804  1.00 26.84          O
HETATM14678  O   HOH D 356     39.119  55.237   2.068  1.00 25.88          O
```

| | | | | | | | | | | |
|---|---|---|---|---|---|---|---|---|---|---|
| HETATM14679 | O | HOH | D | 357 | 43.789 | 58.567 | -10.591 | 1.00 | 25.07 | O |
| HETATM14680 | O | HOH | D | 358 | 19.265 | 53.790 | -39.178 | 1.00 | 19.38 | O |
| HETATM14681 | O | HOH | D | 359 | 43.361 | 60.193 | -1.245 | 1.00 | 26.73 | O |
| HETATM14682 | O | HOH | D | 360 | 24.760 | 50.298 | -24.994 | 1.00 | 18.27 | O |
| HETATM14683 | O | HOH | D | 361 | 23.059 | 37.941 | -24.930 | 1.00 | 23.64 | O |
| HETATM14684 | O | HOH | D | 362 | 32.588 | 43.120 | 8.167 | 1.00 | 25.78 | O |
| HETATM14685 | O | HOH | D | 363 | 27.879 | 58.030 | -1.864 | 1.00 | 24.96 | O |
| HETATM14686 | O | HOH | D | 364 | 32.329 | 48.960 | -27.368 | 1.00 | 21.53 | O |
| HETATM14687 | O | HOH | D | 365 | 11.393 | 48.443 | -43.780 | 1.00 | 19.19 | O |
| HETATM14688 | O | HOH | D | 366 | 40.277 | 41.334 | -11.414 | 1.00 | 25.43 | O |
| HETATM14689 | O | HOH | D | 367 | 6.954 | 44.502 | -27.801 | 1.00 | 30.79 | O |
| HETATM14690 | O | HOH | D | 368 | 21.369 | 58.369 | -36.692 | 1.00 | 30.23 | O |
| HETATM14691 | O | HOH | D | 369 | 35.529 | 65.705 | -9.670 | 1.00 | 27.34 | O |
| HETATM14692 | O | HOH | D | 370 | 5.045 | 45.946 | -28.931 | 1.00 | 26.51 | O |
| HETATM14693 | O | HOH | D | 371 | 32.741 | 69.567 | -3.738 | 1.00 | 29.06 | O |
| HETATM14694 | O | HOH | D | 372 | 34.943 | 51.226 | 11.431 | 1.00 | 28.79 | O |
| HETATM14695 | O | HOH | D | 373 | 28.184 | 54.899 | 6.371 | 1.00 | 21.46 | O |
| HETATM14696 | O | HOH | D | 374 | -5.482 | 54.878 | -26.297 | 1.00 | 33.04 | O |
| HETATM14697 | O | HOH | D | 375 | 29.923 | 70.593 | -5.453 | 1.00 | 27.02 | O |
| HETATM14698 | O | HOH | D | 376 | -4.813 | 58.645 | -40.168 | 1.00 | 29.76 | O |
| HETATM14699 | O | HOH | D | 377 | 6.640 | 54.210 | -21.083 | 1.00 | 37.48 | O |
| HETATM14700 | O | HOH | D | 378 | 8.774 | 61.882 | -25.411 | 1.00 | 34.81 | O |
| HETATM14701 | O | HOH | D | 379 | 24.080 | 57.384 | 3.186 | 1.00 | 28.12 | O |
| HETATM14702 | O | HOH | D | 380 | 46.166 | 48.490 | -4.247 | 1.00 | 36.90 | O |
| HETATM14703 | O | HOH | D | 381 | 33.467 | 49.146 | -24.949 | 1.00 | 29.39 | O |
| HETATM14704 | O | HOH | D | 382 | 25.410 | 34.287 | -27.938 | 1.00 | 25.36 | O |
| HETATM14705 | O | HOH | D | 383 | 3.226 | 58.318 | -41.389 | 1.00 | 34.73 | O |
| HETATM14706 | O | HOH | D | 384 | 41.160 | 49.707 | 1.194 | 1.00 | 26.91 | O |
| HETATM14707 | O | HOH | D | 385 | 20.552 | 39.302 | -19.354 | 1.00 | 24.62 | O |
| HETATM14708 | O | HOH | D | 386 | 38.522 | 41.080 | -4.431 | 1.00 | 27.80 | O |
| HETATM14709 | O | HOH | D | 387 | 10.558 | 45.461 | -40.158 | 1.00 | 29.28 | O |
| HETATM14710 | O | HOH | D | 388 | 29.414 | 60.384 | 1.136 | 1.00 | 29.88 | O |
| HETATM14711 | O | HOH | D | 389 | 20.906 | 39.580 | -24.509 | 1.00 | 29.52 | O |
| HETATM14712 | O | HOH | D | 390 | 44.750 | 49.802 | -7.820 | 1.00 | 25.26 | O |
| HETATM14713 | O | HOH | D | 391 | -7.636 | 57.233 | -32.012 | 1.00 | 42.27 | O |
| HETATM14714 | O | HOH | D | 392 | 33.543 | 60.741 | 3.111 | 1.00 | 30.30 | O |
| HETATM14715 | O | HOH | D | 393 | 24.221 | 47.255 | -37.482 | 1.00 | 24.68 | O |
| HETATM14716 | O | HOH | D | 394 | 15.613 | 60.118 | -39.318 | 1.00 | 24.62 | O |
| HETATM14717 | O | HOH | D | 395 | 44.390 | 46.567 | -4.855 | 1.00 | 37.38 | O |
| HETATM14718 | O | HOH | D | 396 | 21.103 | 42.313 | -24.437 | 1.00 | 24.28 | O |
| HETATM14719 | O | HOH | D | 397 | 36.341 | 49.187 | 6.288 | 1.00 | 24.06 | O |
| HETATM14720 | O | HOH | D | 398 | 30.147 | 36.476 | -7.371 | 1.00 | 24.08 | O |
| HETATM14721 | O | HOH | D | 399 | 6.307 | 60.051 | -42.376 | 1.00 | 29.06 | O |
| HETATM14722 | O | HOH | D | 400 | 14.160 | 50.534 | -42.043 | 1.00 | 26.14 | O |
| HETATM14723 | O | HOH | D | 401 | 19.761 | 44.114 | -17.964 | 1.00 | 34.59 | O |
| HETATM14724 | O | HOH | D | 402 | 27.026 | 32.114 | -27.909 | 1.00 | 32.13 | O |
| HETATM14725 | O | HOH | D | 403 | 20.491 | 40.265 | -35.196 | 1.00 | 23.31 | O |
| HETATM14726 | O | HOH | D | 404 | -6.102 | 55.494 | -33.315 | 1.00 | 34.18 | O |
| HETATM14727 | O | HOH | D | 405 | 33.319 | 31.333 | -20.861 | 1.00 | 27.54 | O |
| HETATM14728 | O | HOH | D | 406 | 44.867 | 61.241 | -11.942 | 1.00 | 29.35 | O |
| HETATM14729 | O | HOH | D | 407 | 25.854 | 54.173 | -22.925 | 1.00 | 31.25 | O |
| HETATM14730 | O | HOH | D | 408 | 22.372 | 44.433 | -41.062 | 1.00 | 28.04 | O |
| HETATM14731 | O | HOH | D | 409 | 27.223 | 68.288 | -11.655 | 1.00 | 33.66 | O |
| HETATM14732 | O | HOH | D | 410 | 23.524 | 60.402 | -9.503 | 1.00 | 28.08 | O |
| HETATM14733 | O | HOH | D | 411 | 39.768 | 34.623 | -17.989 | 1.00 | 42.67 | O |
| HETATM14734 | O | HOH | D | 412 | 13.329 | 46.720 | -43.013 | 1.00 | 24.96 | O |
| HETATM14735 | O | HOH | D | 413 | 22.927 | 65.952 | -6.225 | 1.00 | 25.85 | O |
| HETATM14736 | O | HOH | D | 414 | 35.769 | 31.541 | -20.379 | 1.00 | 27.09 | O |
| HETATM14737 | O | HOH | D | 415 | 29.024 | 55.294 | -25.320 | 1.00 | 24.29 | O |
| HETATM14738 | O | HOH | D | 416 | 22.606 | 37.458 | -15.645 | 1.00 | 27.55 | O |
| HETATM14739 | O | HOH | D | 417 | 10.360 | 40.666 | -32.950 | 1.00 | 28.93 | O |
| HETATM14740 | O | HOH | D | 418 | 42.459 | 48.330 | -17.259 | 1.00 | 25.66 | O |
| HETATM14741 | O | HOH | D | 419 | 22.565 | 37.390 | -36.010 | 1.00 | 26.20 | O |
| HETATM14742 | O | HOH | D | 420 | 33.694 | 65.932 | -13.720 | 1.00 | 28.63 | O |
| HETATM14743 | O | HOH | D | 421 | 34.476 | 48.981 | 12.899 | 1.00 | 26.48 | O |

```
HETATM14744  O   HOH D 422    11.898  54.134 -24.487  1.00 34.65        O
HETATM14745  O   HOH D 423    40.696  48.108   7.083  1.00 33.08        O
HETATM14746  O   HOH D 424    40.549  37.619 -18.636  1.00 32.40        O
HETATM14747  O   HOH D 425     9.576  61.287 -41.094  1.00 29.04        O
HETATM14748  O   HOH D 426    25.001  37.731 -14.327  1.00 29.17        O
HETATM14749  O   HOH D 427    38.370  66.535 -13.417  1.00 33.60        O
HETATM14750  O   HOH D 428    12.687  43.483 -40.127  1.00 30.42        O
HETATM14751  O   HOH D 429    44.564  46.848 -10.798  1.00 26.25        O
HETATM14752  O   HOH D 430    37.226  34.100  -9.037  1.00 45.12        O
HETATM14753  O   HOH D 431    20.245  56.243 -38.726  1.00 34.96        O
HETATM14754  O   HOH D 432    33.560  66.137  -0.393  1.00 24.45        O
HETATM14755  O   HOH D 433    36.119  31.610 -15.428  1.00 33.08        O
HETATM14756  O   HOH D 434    38.656  41.113   4.577  1.00 29.62        O
HETATM14757  O   HOH D 435    42.320  45.296   1.022  1.00 34.40        O
HETATM14758  O   HOH D 436    24.685  56.030 -27.592  1.00 27.66        O
HETATM14759  O   HOH D 437    41.940  41.056  -9.518  1.00 26.48        O
HETATM14760  O   HOH D 438    13.052  53.228 -40.111  1.00 19.96        O
HETATM14761  O   HOH D 439    33.564  31.743 -16.091  1.00 25.18        O
HETATM14762  O   HOH D 440    35.619  51.866   7.266  1.00 31.20        O
HETATM14763  O   HOH D 441    20.662  35.816 -34.867  1.00 37.73        O
HETATM14764  O   HOH D 442    27.881  57.936   7.463  1.00 36.70        O
HETATM14765  O   HOH D 443    11.809  41.542 -38.429  1.00 30.76        O
HETATM14766  O   HOH D 444    11.240  42.087 -35.788  1.00 33.73        O
HETATM14767  O   HOH D 445    37.892  59.293   8.399  1.00 38.22        O
HETATM14768  O   HOH D 446    -0.843  56.786 -25.241  1.00 30.23        O
HETATM14769  O   HOH D 447    14.808  58.901 -28.759  1.00 27.93        O
HETATM14770  O   HOH D 448    20.378  52.438 -18.497  1.00 26.73        O
HETATM14771  O   HOH D 449    36.652  64.404 -17.517  1.00 26.64        O
HETATM14772  O   HOH D 450    40.448  43.125 -17.109  1.00 25.41        O
HETATM14773  O   HOH D 451    14.014  34.634 -30.869  1.00 39.92        O
HETATM14774  O   HOH D 452    11.050  65.246 -33.192  1.00 33.37        O
HETATM14775  O   HOH D 453    43.985  61.729  -6.357  1.00 29.15        O
HETATM14776  O   HOH D 454    38.087  52.010   8.925  1.00 30.13        O
HETATM14777  O   HOH D 455    27.677  53.241 -26.603  1.00 30.95        O
HETATM14778  O   HOH D 456    21.138  51.090 -20.590  1.00 31.85        O
HETATM14779  O   HOH D 457    25.031  57.349 -19.379  1.00 37.09        O
HETATM14780  O   HOH D 458    18.483  52.031 -16.327  1.00 37.61        O
HETATM14781  O   HOH D 459    14.955  62.774 -28.406  1.00 36.01        O
HETATM14782  O   HOH D 460    39.543  45.031  10.606  1.00 31.10        O
HETATM14783  O   HOH D 461    29.592  38.525  -5.075  1.00 28.25        O
HETATM14784  O   HOH D 462    16.130  43.037 -12.450  1.00 33.59        O
HETATM14785  O   HOH D 463    42.590  43.458  -6.250  1.00 36.27        O
HETATM14786  O   HOH D 464    35.008  53.686 -22.450  1.00 39.89        O
HETATM14787  O   HOH D 465    10.753  52.834 -42.142  1.00 31.85        O
HETATM14788  O   HOH D 466   -11.579  60.203 -27.527  1.00 46.26        O
HETATM14789  O   HOH D 467    24.037  55.482 -20.654  1.00 29.78        O
HETATM14790  O   HOH D 468    18.336  50.132 -20.750  1.00 36.04        O
HETATM14791  O   HOH D 469    42.691  53.309 -16.405  1.00 34.27        O
HETATM14792  O   HOH D 470    22.643  36.244 -19.810  1.00 33.90        O
HETATM14793  O   HOH D 471    16.553  43.742 -14.946  1.00 39.74        O
HETATM14794  O   HOH D 472    24.643  40.536 -15.719  1.00 32.89        O
HETATM14795  O   HOH D 473    23.160  60.929 -14.568  1.00 48.29        O
HETATM14796  O   HOH D 474    32.920  29.491  -5.547  1.00 40.22        O
HETATM14797  O   HOH D 475    26.546  58.139 -34.813  1.00 30.71        O
HETATM14798  O   HOH D 476    22.088  56.418 -30.742  1.00 32.56        O
HETATM14799  O   HOH D 477    17.645  47.580 -21.253  1.00 32.71        O
HETATM14800  O   HOH D 478    21.348  52.786 -22.607  1.00 36.67        O
HETATM14801  O   HOH D 479    21.213  60.016  -2.511  1.00 34.97        O
HETATM14802  O   HOH D 480    42.023  60.401   1.307  1.00 41.07        O
HETATM14803  O   HOH D 481     2.523  64.813 -37.871  1.00 30.56        O
HETATM14804  O   HOH D 482     0.441  54.971 -42.265  1.00 35.42        O
HETATM14805  O   HOH D 483    18.025  45.219 -16.550  1.00 43.37        O
HETATM14806  O   HOH D 484    43.199  57.784 -17.770  1.00 38.31        O
HETATM14807  O   HOH D 485    23.432  61.133 -11.852  1.00 32.46        O
HETATM14808  O   HOH D 486     3.081  50.050 -38.486  1.00 33.50        O
```

```
HETATM14809  O   HOH D 487   40.559  42.494  -3.524  1.00 38.77        O
HETATM14810  O   HOH D 488   17.466  52.260 -22.060  1.00 34.91        O
HETATM14811  O   HOH D 489  -10.041  56.962 -43.097  1.00 47.89        O
HETATM14812  O   HOH D 490   22.064  56.320 -28.342  1.00 42.19        O
HETATM14813  O   HOH D 491   20.248  56.069 -26.393  1.00 32.64        O
HETATM14814  O   HOH D 492    5.836  38.059 -35.247  1.00 33.33        O
HETATM14815  O   HOH D 493   43.409  48.345   6.941  1.00 39.62        O
HETATM14816  O   HOH D 494   43.290  49.221 -11.465  1.00 36.00        O
HETATM14817  O   HOH D 495   40.900  48.351 -23.630  1.00 36.60        O
HETATM14818  O   HOH D 496   31.992  67.186   1.727  1.00 28.70        O
HETATM14819  O   HOH D 497    1.307  66.756 -39.449  1.00 37.32        O
HETATM14820  O   HOH D 498   36.244  60.216   4.896  1.00 35.99        O
HETATM14821  O   HOH D 499   12.049  46.317 -21.245  1.00 38.93        O
HETATM14822  O   HOH D 500   43.312  44.403 -20.341  1.00 34.81        O
HETATM14823  O   HOH D 501   45.896  61.262  -0.972  1.00 34.98        O
HETATM14824  O   HOH D 502   22.863  71.944  -7.386  1.00 45.58        O
HETATM14825  O   HOH D 503   10.533  49.257 -24.042  1.00 29.55        O
HETATM14826  O   HOH D 504   38.662  46.491 -25.016  1.00 42.22        O
HETATM14827  O   HOH D 505   24.514  57.611 -31.567  1.00 52.96        O
HETATM14828  O   HOH D 506   39.289  41.240  13.459  1.00 42.22        O
HETATM14829  O   HOH D 507   16.576  34.681 -30.609  1.00 41.80        O
HETATM14830  O   HOH D 508   15.139  37.172 -37.913  1.00 39.78        O
HETATM14831  O   HOH D 509   -7.680  65.023 -35.231  1.00 47.99        O
HETATM14832  O   HOH D 510   42.314  56.521   3.128  1.00 34.37        O
HETATM14833  O   HOH D 511   25.768  43.983 -37.842  1.00 31.09        O
HETATM14834  O   HOH D 512   11.410  38.590 -25.273  1.00 31.03        O
HETATM14835  O   HOH D 513   34.141  33.013 -12.426  1.00 47.79        O
HETATM14836  O   HOH D 514   43.051  45.299  -3.102  1.00 40.69        O
HETATM14837  O   HOH D 515   20.033  43.931 -21.943  1.00 54.39        O
HETATM14838  O   HOH D 516   43.675  62.024  -3.718  1.00 47.54        O
HETATM14839  O   HOH D 517   19.736  46.414 -22.031  1.00 43.97        O
HETATM14840  O   HOH D 518   37.656  61.427   1.139  1.00 30.87        O
HETATM14841  O   HOH D 519   40.832  46.971   9.395  1.00 36.99        O
HETATM14842  O   HOH D 520   39.300  54.670   9.082  1.00 35.24        O
HETATM14843  O   HOH D 521    7.098  66.595 -34.569  1.00 40.24        O
HETATM14844  O   HOH D 522   30.801  45.830   9.477  1.00 31.90        O
HETATM14845  O   HOH D 523   18.801  38.724 -26.354  1.00 35.37        O
HETATM14846  O   HOH D 524   14.869  46.674 -13.607  1.00 46.95        O
HETATM14847  O   HOH D 525   -6.954  59.201 -47.408  1.00 64.58        O
HETATM14848  O   HOH D 526   -3.566  57.624 -47.796  1.00 58.63        O
HETATM14849  O   HOH D 527   19.547  36.625 -27.868  1.00 31.20        O
HETATM14850  O   HOH D 528   25.531  59.088   5.974  1.00 40.21        O
HETATM14851  O   HOH D 529   33.999  39.898  -3.206  1.00 47.28        O
HETATM14852  O   HOH D 530   25.526  72.783  -6.310  1.00 38.02        O
HETATM14853  O   HOH D 531   42.265  48.918 -19.882  1.00 33.88        O
HETATM14854  O   HOH D 532   21.680  60.801  -8.290  1.00 36.81        O
HETATM14855  O   HOH D 533   38.167  61.542 -19.947  1.00 47.41        O
HETATM14856  O   HOH D 534   -7.377  72.716 -35.038  1.00 48.15        O
HETATM14857  O   HOH D 535   16.516  59.507 -26.823  1.00 38.62        O
HETATM14858  O   HOH D 536    8.414  56.673 -24.117  1.00 41.30        O
HETATM14859  O   HOH D 537   43.398  38.849 -10.420  1.00 47.18        O
HETATM14860  O   HOH D 538   12.531  51.692 -25.155  1.00 39.64        O
HETATM14861  O   HOH D 539   20.289  41.757 -19.523  1.00 37.23        O
HETATM14862  O   HOH D 540   29.056  37.839 -34.415  1.00 39.69        O
HETATM14863  O   HOH D 541   46.709  63.295  -2.544  1.00 48.11        O
HETATM14864  O   HOH D 542   41.995  51.996   5.592  1.00 43.03        O
HETATM14865  O   HOH D 543   30.255  54.436   9.809  1.00 51.09        O
HETATM14866  O   HOH D 544   33.254  45.520  13.006  1.00 44.44        O
HETATM14867  O   HOH D 545   33.014  39.009   8.872  1.00 44.21        O
HETATM14868  O   HOH D 546   42.784  41.740   4.033  1.00 45.14        O
HETATM14869  O   HOH D 547   40.040  39.680   8.137  1.00 50.27        O
HETATM14870  O   HOH D 548   41.428  43.102   2.092  1.00 34.61        O
HETATM14871  O   HOH D 549   39.388  41.002  -0.154  1.00 48.74        O
HETATM14872  O   HOH D 550   30.639  37.525   9.723  1.00 42.22        O
HETATM14873  O   HOH D 551   18.529  36.097 -25.364  1.00 43.45        O
```

```
HETATM14874  O   HOH D 552    43.239  46.011 -17.657  1.00 41.69        O
HETATM14875  O   HOH D 553    44.066  41.370 -16.602  1.00 45.08        O
HETATM14876  O   HOH D 554    19.363  58.601 -39.575  1.00 38.89        O
HETATM14877  O   HOH D 555    11.470  43.925 -20.744  1.00 45.88        O
HETATM14878  O   HOH D 556    47.336  50.602  -6.719  1.00 38.70        O
HETATM14879  O   HOH D 557    13.126  55.765 -40.957  1.00 40.03        O
HETATM14880  O   HOH D 558    22.418  32.869  -8.715  1.00 50.30        O
HETATM14881  O   HOH D 559    15.302  40.642 -11.327  1.00 37.82        O
HETATM14882  O   HOH D 560    -8.647  57.965 -34.721  1.00 35.57        O
HETATM14883  O   HOH D 561    17.789  37.334 -37.721  1.00 41.54        O
HETATM14884  O   HOH D 562    21.792  34.108 -30.243  1.00 45.88        O
HETATM14885  O   HOH D 563    -7.536  58.115 -28.449  1.00 43.82        O
HETATM14886  O   HOH D 564    41.449  40.282 -17.763  1.00 39.28        O
HETATM14887  O   HOH D 565    11.614  38.772 -35.716  1.00 46.34        O
HETATM14888  O   HOH D 566    29.083  36.117 -13.645  1.00 39.97        O
HETATM14889  O   HOH D 567     1.039  57.560 -41.437  1.00 38.89        O
HETATM14890  O   HOH D 568    37.577  59.449   3.026  1.00 52.66        O
HETATM14891  O   HOH D 569    15.466  63.300 -25.405  1.00 47.61        O
HETATM14892  O   HOH D 570    -0.509  63.474 -25.280  1.00 41.53        O
HETATM14893  O   HOH D 571     9.518  67.473 -33.968  1.00 48.86        O
HETATM14894  O   HOH D 572    -6.874  60.011 -40.010  1.00 48.94        O
HETATM14895  O   HOH D 573     8.725  38.924 -34.969  1.00 46.57        O
HETATM14896  O   HOH D 574    18.740  40.805 -39.942  1.00 40.09        O
HETATM14897  O   HOH D 575     0.079  57.521 -20.890  1.00 54.50        O
HETATM14898  O   HOH D 576    42.755  64.517  -7.388  1.00 48.72        O
HETATM14899  O   HOH D 577    -6.878  68.821 -37.100  1.00 60.66        O
HETATM14900  O   HOH D 578    21.602  61.331  -0.528  1.00 46.48        O
HETATM14901  O   HOH D 579    37.548  53.902 -21.808  1.00 44.14        O
HETATM14902  O   HOH D 580    35.848  41.502  -2.031  1.00 45.82        O
HETATM14903  O   HOH D 581    43.515  42.952 -10.255  1.00 42.35        O
HETATM14904  O   HOH D 582    -7.377  51.941 -38.263  1.00 35.38        O
HETATM14905  O   HOH D 583    40.381  50.793 -25.028  1.00 50.77        O
HETATM14906  O   HOH D 584   -11.885  63.089 -31.838  1.00 52.28        O
HETATM14907  O   HOH D 585    34.968  39.899  10.417  1.00 46.54        O
HETATM14908  O   HOH D 586     9.181  40.063 -38.578  1.00 43.30        O
HETATM14909  O   HOH D 587    40.992  37.889 -23.603  1.00 46.29        O
HETATM14910  O   HOH D 588   -13.350  64.898 -33.396  1.00 47.86        O
HETATM14911  O   HOH D 589    32.703  63.264   2.566  1.00 43.92        O
HETATM14912  O   HOH D 590     8.964  59.935 -42.801  1.00 44.75        O
HETATM14913  O   HOH D 591    35.012  32.190 -18.135  1.00 46.86        O
HETATM14914  O   HOH D 592    42.064  53.623 -18.820  1.00 50.04        O
HETATM14915  O   HOH D 593    33.549  42.898  10.778  1.00 42.44        O
HETATM14916  O   HOH D 594    44.330  50.880  -9.887  1.00 35.10        O
HETATM14917  O   HOH D 595    28.110  55.239 -30.163  1.00 43.90        O
HETATM14918  O   HOH D 596    25.478  35.130 -25.619  1.00 44.27        O
HETATM14919  O   HOH D 597    19.378  39.190 -37.842  1.00 55.45        O
HETATM14920  O   HOH D 598    -8.968  58.200 -39.353  1.00 49.01        O
HETATM14921  O   HOH D 599    29.568  34.059  -7.273  1.00 46.88        O
HETATM14922  O   HOH D 600    39.508  34.035 -10.533  1.00 56.54        O
HETATM14923  O   HOH D 601    48.835  56.072  -3.499  1.00 42.56        O
HETATM14924  O   HOH D 602    24.629  31.795 -29.901  1.00 54.05        O
HETATM14925  O   HOH D 603    28.508  67.397 -19.990  1.00 45.64        O
HETATM14926  O   HOH D 604     6.143  42.045 -34.355  1.00 46.61        O
HETATM14927  O   HOH D 605    44.257  64.510  -9.525  1.00 47.91        O
HETATM14928  O   HOH D 606    23.196  59.544   3.048  1.00 46.75        O
HETATM14929  O   HOH D 607    30.598  61.155 -20.830  1.00 51.28        O
HETATM14930  O   HOH D 608    45.270  47.029  -7.565  1.00 55.10        O
HETATM14931  O   HOH D 609    36.683  62.496   6.985  1.00 50.84        O
HETATM14932  O   HOH D 610    37.946  66.660  -9.047  1.00 58.05        O
HETATM14933  O   HOH D 611    44.925  40.560   8.482  1.00 56.67        O
HETATM14934  O   HOH D 612    38.321  39.217  12.778  1.00 48.06        O
HETATM14935  O   HOH D 613    42.713  42.371 -13.145  1.00 47.93        O
HETATM14936  O   HOH D 614    15.121  63.900 -30.904  1.00 50.39        O
HETATM14937  O   HOH D 615    11.828  35.164 -26.786  1.00 60.08        O
HETATM14938  O   HOH D 616    23.232  38.970  -4.523  1.00 39.20        O
```

```
HETATM14939  O    HOH D 617    42.405  36.711 -10.467  1.00 43.97         O
HETATM14940  O    HOH D 618    13.803  40.472 -13.329  1.00 51.47         O
HETATM14941  O    HOH D 619    45.587  49.425   0.245  1.00 60.83         O
HETATM14942  O    HOH D 620    48.087  59.965  -0.340  1.00 52.09         O
HETATM14943  O    HOH D 621    37.439  64.170 -20.999  1.00 63.97         O
HETATM14944  O    HOH D 622    43.531  55.810 -16.148  1.00 44.69         O
HETATM14945  O    HOH D 623    22.072  64.134 -11.726  1.00 43.98         O
HETATM14946  O    HOH D 624    49.736  54.106  -1.359  1.00 61.67         O
HETATM14947  O    HOH D 625    10.748  70.103 -30.014  1.00 58.14         O
HETATM14948  O    HOH D 626    45.184  61.259 -17.055  1.00 52.41         O
HETATM14949  O    HOH D 627    36.273  35.079 -12.041  1.00 50.47         O
HETATM14950  O    HOH D 628    17.075  51.201 -18.256  1.00 45.57         O
HETATM14951  O    HOH D 629    23.606  33.207 -36.837  1.00 52.63         O
HETATM14952  O    HOH D 630    23.526  59.500 -18.557  1.00 59.82         O
HETATM14953  O    HOH D 631     9.284  65.173 -38.115  1.00 50.73         O
HETATM14954  O    HOH D 632    31.806  66.063 -16.279  1.00 45.88         O
HETATM14955  O    HOH D 633    -8.580  56.327 -29.767  1.00 56.53         O
HETATM14956  O    HOH D 634    34.675  39.684   4.790  1.00 64.88         O
HETATM14957  O    HOH D 635    27.777  38.159  -8.171  1.00 44.06         O
HETATM14958  O    HOH D 636    44.839  46.863   1.867  1.00 54.91         O
HETATM14959  O    HOH D 637    39.904  60.218   2.980  1.00 49.86         O
HETATM14960  O    HOH D 638    10.542  63.456 -39.547  1.00 46.95         O
HETATM14961  O    HOH D 639    44.633  49.416 -14.572  1.00 50.33         O
HETATM14962  O    HOH D 640    44.574  55.813   5.148  1.00 67.01         O
HETATM14963  O    HOH D 641    -8.157  58.306 -25.736  1.00 48.14         O
HETATM14964  O    HOH D 642     8.919  33.125 -36.677  1.00 52.90         O
HETATM14965  O    HOH D 643    18.127  48.267 -15.382  1.00 43.94         O
HETATM14966  O    HOH D 644    21.482  37.532  -5.446  1.00 42.93         O
HETATM14967  O    HOH D 645     4.067  66.259 -22.583  1.00 58.81         O
HETATM14968  O    HOH D 646    38.917  56.811   9.936  1.00 56.38         O
HETATM14969  O    HOH D 647     0.384  65.519 -41.919  1.00 52.68         O
HETATM14970  O    HOH D 648    30.213  55.469 -11.028  1.00 24.30         O
HETATM14971  O    HOH E 216     6.821  51.947  16.071  1.00 15.57         O
HETATM14972  O    HOH E 217    29.228  39.936  80.888  1.00 12.70         O
HETATM14973  O    HOH E 218     0.934  38.442  27.175  1.00 15.61         O
HETATM14974  O    HOH E 219    19.129  50.721  18.837  1.00 12.60         O
HETATM14975  O    HOH E 220    11.310  36.091  24.568  1.00 14.78         O
HETATM14976  O    HOH E 221    18.202  47.733  14.870  1.00 14.66         O
HETATM14977  O    HOH E 222    14.465  40.363  14.313  1.00 14.24         O
HETATM14978  O    HOH E 223    23.795  50.672  69.289  1.00 12.37         O
HETATM14979  O    HOH E 224     9.936  47.152  13.715  1.00 16.07         O
HETATM14980  O    HOH E 225    -1.227  47.204  20.166  1.00 16.85         O
HETATM14981  O    HOH E 226    19.525  53.435  18.242  1.00 17.38         O
HETATM14982  O    HOH E 227    32.904  55.004  56.526  1.00 19.54         O
HETATM14983  O    HOH E 228    20.220  56.690  38.541  1.00 23.49         O
HETATM14984  O    HOH E 229     2.709  40.729  34.093  1.00 16.69         O
HETATM14985  O    HOH E 230    13.109  55.709  38.075  1.00 16.85         O
HETATM14986  O    HOH E 231    21.355  56.666  21.340  1.00 19.35         O
HETATM14987  O    HOH E 232    14.791  56.252  19.148  1.00 17.97         O
HETATM14988  O    HOH E 233    16.353  40.065  33.303  1.00 18.07         O
HETATM14989  O    HOH E 234    26.184  35.373  80.741  1.00 16.51         O
HETATM14990  O    HOH E 235    26.357  50.396  67.387  1.00 21.48         O
HETATM14991  O    HOH E 236     8.451  38.224  30.338  1.00 20.90         O
HETATM14992  O    HOH E 237    40.010  42.511  68.074  1.00 18.88         O
HETATM14993  O    HOH E 238    36.163  55.040  62.741  1.00 21.99         O
HETATM14994  O    HOH E 239    26.045  37.315  71.746  1.00 20.87         O
HETATM14995  O    HOH E 240    10.131  42.047  35.749  1.00 19.01         O
HETATM14996  O    HOH E 241    23.873  52.258  78.836  1.00 17.41         O
HETATM14997  O    HOH E 242    29.066  37.268  81.287  1.00 16.96         O
HETATM14998  O    HOH E 243    11.733  52.143  14.712  1.00 16.53         O
HETATM14999  O    HOH E 244    17.833  45.940  17.280  1.00 23.04         O
HETATM15000  O    HOH E 245     5.906  49.485  10.057  1.00 16.41         O
HETATM15001  O    HOH E 246    19.681  44.775  70.397  1.00 20.95         O
HETATM15002  O    HOH E 247    13.470  38.818  30.826  1.00 16.76         O
HETATM15003  O    HOH E 248    17.557  42.912  74.585  1.00 18.28         O
```

```
HETATM15004  O   HOH E 249   39.399  41.789  55.360  1.00  26.64        O
HETATM15005  O   HOH E 250   23.723  48.906  20.031  1.00  22.21        O
HETATM15006  O   HOH E 251    4.638  36.232  16.408  1.00  21.88        O
HETATM15007  O   HOH E 252   11.583  34.477  22.428  1.00  18.34        O
HETATM15008  O   HOH E 253    7.321  49.782  13.502  1.00  16.32        O
HETATM15009  O   HOH E 254    5.503  53.061  11.915  1.00  23.87        O
HETATM15010  O   HOH E 255   43.682  43.310  47.878  1.00  35.18        O
HETATM15011  O   HOH E 256   18.013  45.198  72.624  1.00  24.15        O
HETATM15012  O   HOH E 257   28.414  54.352  47.405  1.00  35.39        O
HETATM15013  O   HOH E 258   24.059  41.939  63.287  1.00  21.06        O
HETATM15014  O   HOH E 259   15.340  54.402  15.979  1.00  20.53        O
HETATM15015  O   HOH E 260   21.244  51.461  81.918  1.00  18.41        O
HETATM15016  O   HOH E 261    2.592  45.863   9.798  1.00  23.46        O
HETATM15017  O   HOH E 262   16.069  42.956  37.312  1.00  28.93        O
HETATM15018  O   HOH E 263   39.309  54.686  55.924  1.00  26.50        O
HETATM15019  O   HOH E 264   12.522  41.342  37.336  1.00  25.99        O
HETATM15020  O   HOH E 265   34.480  37.986  60.082  1.00  26.30        O
HETATM15021  O   HOH E 266   17.275  40.091  20.382  1.00  23.95        O
HETATM15022  O   HOH E 267   18.231  54.612  16.247  1.00  20.82        O
HETATM15023  O   HOH E 268    8.174  32.599  19.723  1.00  25.46        O
HETATM15024  O   HOH E 269    7.501  57.058  37.742  1.00  26.99        O
HETATM15025  O   HOH E 270    6.240  36.903  30.633  1.00  23.57        O
HETATM15026  O   HOH E 271   26.616  55.969  38.744  1.00  26.13        O
HETATM15027  O   HOH E 272   42.109  49.337  49.918  1.00  27.65        O
HETATM15028  O   HOH E 273   38.135  50.790  37.690  1.00  39.56        O
HETATM15029  O   HOH E 274    0.357  44.968  27.242  1.00  26.51        O
HETATM15030  O   HOH E 275   18.499  59.521  32.999  1.00  40.32        O
HETATM15031  O   HOH E 276    4.961  33.926  17.605  1.00  26.68        O
HETATM15032  O   HOH E 277   22.977  38.358  32.940  1.00  32.96        O
HETATM15033  O   HOH E 278    0.938  53.970  24.321  1.00  21.48        O
HETATM15034  O   HOH E 279   17.442  39.633  24.962  1.00  32.11        O
HETATM15035  O   HOH E 280   24.937  62.028  36.177  1.00  27.99        O
HETATM15036  O   HOH E 281   24.477  49.711  80.060  1.00  25.74        O
HETATM15037  O   HOH E 282   29.319  51.910  74.192  1.00  27.08        O
HETATM15038  O   HOH E 283   25.019  45.878  58.372  1.00  31.09        O
HETATM15039  O   HOH E 284   41.617  40.685  56.914  1.00  30.65        O
HETATM15040  O   HOH E 285   11.902  43.830  38.922  1.00  29.61        O
HETATM15041  O   HOH E 286   14.081  36.824  32.297  1.00  28.72        O
HETATM15042  O   HOH E 287   38.020  44.516  45.209  1.00  28.83        O
HETATM15043  O   HOH E 288   33.780  56.302  63.711  1.00  28.89        O
HETATM15044  O   HOH E 289   -0.101  34.919  25.066  1.00  38.02        O
HETATM15045  O   HOH E 290   -3.834  41.025  20.580  1.00  26.07        O
HETATM15046  O   HOH E 291   42.777  43.849  66.088  1.00  31.49        O
HETATM15047  O   HOH E 292    8.003  61.393  21.213  1.00  34.93        O
HETATM15048  O   HOH E 293   26.598  47.828  26.815  1.00  35.21        O
HETATM15049  O   HOH E 294   27.824  47.604  47.393  1.00  32.40        O
HETATM15050  O   HOH E 295   26.537  53.021  78.381  1.00  25.85        O
HETATM15051  O   HOH E 296   12.724  50.887  43.826  1.00  32.31        O
HETATM15052  O   HOH E 297   13.906  36.666  18.403  1.00  31.59        O
HETATM15053  O   HOH E 298   17.932  42.417  20.256  1.00  24.74        O
HETATM15054  O   HOH E 299    6.005  33.786  31.725  1.00  32.99        O
HETATM15055  O   HOH E 300   41.045  36.248  71.686  1.00  33.28        O
HETATM15056  O   HOH E 301   31.190  50.253  74.816  1.00  24.01        O
HETATM15057  O   HOH E 302   12.603  35.162  29.752  1.00  30.01        O
HETATM15058  O   HOH E 303    2.636  36.082  33.071  1.00  28.07        O
HETATM15059  O   HOH E 304   42.764  45.947  50.829  1.00  30.18        O
HETATM15060  O   HOH E 305   10.621  36.581  31.313  1.00  26.84        O
HETATM15061  O   HOH E 306   31.073  54.440  22.955  1.00  38.38        O
HETATM15062  O   HOH E 307   34.515  54.339  66.929  1.00  33.08        O
HETATM15063  O   HOH E 308   20.566  43.974  20.512  1.00  27.54        O
HETATM15064  O   HOH E 309   32.236  57.168  55.077  1.00  28.10        O
HETATM15065  O   HOH E 310   32.237  46.757  40.166  1.00  37.19        O
HETATM15066  O   HOH E 311   21.532  41.417  71.415  1.00  35.19        O
HETATM15067  O   HOH E 312   17.473  59.892  20.393  1.00  33.81        O
HETATM15068  O   HOH E 313   21.152  56.100  76.810  1.00  40.24        O
```

```
HETATM15069  O   HOH E 314    -4.383  48.146  11.459  1.00 33.26         O
HETATM15070  O   HOH E 315    38.155  54.588  64.651  1.00 31.87         O
HETATM15071  O   HOH E 316    24.535  50.298  38.589  1.00 32.02         O
HETATM15072  O   HOH E 317    16.194  59.238  32.012  1.00 38.26         O
HETATM15073  O   HOH E 318    32.733  51.367  28.033  1.00 30.28         O
HETATM15074  O   HOH E 319    16.198  38.295  35.174  1.00 24.11         O
HETATM15075  O   HOH E 320    37.457  36.370  70.823  1.00 40.60         O
HETATM15076  O   HOH E 321    33.896  56.277  33.955  1.00 40.42         O
HETATM15077  O   HOH E 322    32.738  40.106  53.662  1.00 33.91         O
HETATM15078  O   HOH E 323    38.272  39.632  54.494  1.00 41.36         O
HETATM15079  O   HOH E 324    19.004  42.908  36.987  1.00 31.42         O
HETATM15080  O   HOH E 325     7.337  35.075  16.838  1.00 35.92         O
HETATM15081  O   HOH E 326    -4.330  51.190  15.247  1.00 33.32         O
HETATM15082  O   HOH E 327    17.029  36.348  30.918  1.00 32.96         O
HETATM15083  O   HOH E 328    25.011  45.082  35.015  1.00 38.66         O
HETATM15084  O   HOH E 329    17.923  38.698  22.661  1.00 30.26         O
HETATM15085  O   HOH E 330    34.843  47.562  36.470  1.00 49.20         O
HETATM15086  O   HOH E 331    14.101  47.181  40.114  1.00 28.77         O
HETATM15087  O   HOH E 332     9.134  59.585  31.672  1.00 34.33         O
HETATM15088  O   HOH E 333     9.476  39.432  33.937  1.00 33.52         O
HETATM15089  O   HOH E 334    33.234  34.502  76.182  1.00 32.85         O
HETATM15090  O   HOH E 335    17.280  50.378  40.275  1.00 39.67         O
HETATM15091  O   HOH E 336    28.036  61.403  34.991  1.00 30.08         O
HETATM15092  O   HOH E 337    20.551  37.059  32.755  1.00 41.37         O
HETATM15093  O   HOH E 338    38.253  51.776  77.003  1.00 47.25         O
HETATM15094  O   HOH E 339    18.856  36.442  28.537  1.00 29.99         O
HETATM15095  O   HOH E 340     3.753  56.506  17.411  1.00 33.86         O
HETATM15096  O   HOH E 341    37.360  53.621  67.017  1.00 35.01         O
HETATM15097  O   HOH E 342    29.385  45.207  46.546  1.00 41.19         O
HETATM15098  O   HOH E 343    -4.330  40.492  17.913  1.00 35.79         O
HETATM15099  O   HOH E 344    19.101  37.904  26.454  1.00 30.43         O
HETATM15100  O   HOH E 345    28.038  53.675  76.413  1.00 32.71         O
HETATM15101  O   HOH E 346    35.426  37.775  55.974  1.00 37.58         O
HETATM15102  O   HOH E 347    20.472  54.437  78.559  1.00 30.55         O
HETATM15103  O   HOH E 348    35.793  35.333  67.779  1.00 45.12         O
HETATM15104  O   HOH E 349    26.098  62.264  28.359  1.00 46.47         O
HETATM15105  O   HOH E 350    26.253  53.035  46.733  1.00 31.84         O
HETATM15106  O   HOH E 351    49.180  48.326  48.746  1.00 46.88         O
HETATM15107  O   HOH E 352    18.411  34.240  27.317  1.00 39.64         O
HETATM15108  O   HOH E 353    18.718  57.346  16.459  1.00 25.92         O
HETATM15109  O   HOH E 354    22.384  43.798  50.687  1.00 40.26         O
HETATM15110  O   HOH E 355    31.475  36.722  82.884  1.00 40.17         O
HETATM15111  O   HOH E 356    30.546  58.638  38.368  1.00 38.72         O
HETATM15112  O   HOH E 357    41.481  55.291  46.111  1.00 39.97         O
HETATM15113  O   HOH E 358    30.301  49.000  77.812  1.00 43.95         O
HETATM15114  O   HOH E 359    29.143  61.225  31.012  1.00 36.69         O
HETATM15115  O   HOH E 360    20.029  49.331  40.331  1.00 35.55         O
HETATM15116  O   HOH E 361    41.053  47.711  71.417  1.00 38.06         O
HETATM15117  O   HOH E 362     8.547  66.826  30.173  1.00 47.73         O
HETATM15118  O   HOH E 363    21.600  59.001  20.597  1.00 40.44         O
HETATM15119  O   HOH E 364    41.000  33.250  64.779  1.00 41.65         O
HETATM15120  O   HOH E 365    21.447  51.172  47.913  1.00 41.50         O
HETATM15121  O   HOH E 366    11.747  63.639  24.763  1.00 40.48         O
HETATM15122  O   HOH E 367    38.210  43.094  43.050  1.00 39.75         O
HETATM15123  O   HOH E 368    30.230  36.187  59.013  1.00 36.98         O
HETATM15124  O   HOH E 369    21.206  36.174  30.105  1.00 44.91         O
HETATM15125  O   HOH E 370    16.635  58.221  18.316  1.00 36.97         O
HETATM15126  O   HOH E 371    27.273  48.768  79.825  1.00 41.16         O
HETATM15127  O   HOH E 372    24.899  62.358  38.479  1.00 39.00         O
HETATM15128  O   HOH E 373    31.654  40.732  51.261  1.00 47.54         O
HETATM15129  O   HOH E 374    32.043  41.069  47.195  1.00 42.21         O
HETATM15130  O   HOH E 375     9.476  41.488  12.641  1.00 34.44         O
HETATM15131  O   HOH E 376    28.142  33.163  67.397  1.00 42.38         O
HETATM15132  O   HOH E 377    10.588  66.276  25.431  1.00 38.43         O
HETATM15133  O   HOH E 378    14.587  44.035  39.264  1.00 55.29         O
```

```
HETATM15134  O   HOH E 379    11.396  60.564  19.590  1.00 31.30        O
HETATM15135  O   HOH E 380    10.989  39.258  13.653  1.00 36.65        O
HETATM15136  O   HOH E 381    36.343  57.385  37.272  1.00 36.13        O
HETATM15137  O   HOH E 382    -1.964  43.540  14.272  1.00 46.49        O
HETATM15138  O   HOH E 383    42.984  52.325  59.174  1.00 30.70        O
HETATM15139  O   HOH E 384    29.690  42.065  45.469  1.00 59.94        O
HETATM15140  O   HOH E 385    26.860  45.268  36.680  1.00 45.99        O
HETATM15141  O   HOH E 386    21.661  59.366  72.514  1.00 37.62        O
HETATM15142  O   HOH E 387    19.438  40.774  26.900  1.00 38.81        O
HETATM15143  O   HOH E 388    18.453  43.438  17.403  1.00 37.93        O
HETATM15144  O   HOH E 389    -0.068  47.034   7.071  1.00 47.35        O
HETATM15145  O   HOH E 390    41.834  47.377  69.034  1.00 44.21        O
HETATM15146  O   HOH E 391    42.682  36.042  64.121  1.00 43.21        O
HETATM15147  O   HOH E 392    17.873  56.979  42.635  1.00 37.39        O
HETATM15148  O   HOH E 393    33.167  58.367  38.621  1.00 35.94        O
HETATM15149  O   HOH E 394    27.666  56.419  75.314  1.00 39.47        O
HETATM15150  O   HOH E 395    32.708  34.789  73.607  1.00 37.89        O
HETATM15151  O   HOH E 396    27.308  42.120  81.217  1.00 15.68        O
HETATM15152  O   HOH E 397    33.807  52.047  73.247  1.00 41.10        O
HETATM15153  O   HOH E 398    35.433  57.444  46.757  1.00 36.60        O
HETATM15154  O   HOH E 399    26.482  36.987  68.330  1.00 30.51        O
HETATM15155  O   HOH E 400     9.817  57.350  41.841  1.00 35.30        O
HETATM15156  O   HOH E 401    11.159  37.604  33.868  1.00 36.27        O
HETATM15157  O   HOH E 402    31.667  40.814  81.729  1.00 38.37        O
HETATM15158  O   HOH E 403     6.160  66.273  25.666  1.00 45.34        O
HETATM15159  O   HOH E 404    -6.388  38.672  26.213  1.00 36.80        O
HETATM15160  O   HOH E 405    37.670  49.244  77.382  1.00 37.11        O
HETATM15161  O   HOH E 406    23.505  38.363  66.288  1.00 38.79        O
HETATM15162  O   HOH E 407    41.278  53.150  38.000  1.00 39.75        O
HETATM15163  O   HOH E 408    24.395  56.643  73.057  1.00 42.84        O
HETATM15164  O   HOH E 409    43.743  39.736  59.378  1.00 45.08        O
HETATM15165  O   HOH E 410    15.769  36.721  15.703  1.00 39.59        O
HETATM15166  O   HOH E 411    25.095  38.721  56.030  1.00 48.07        O
HETATM15167  O   HOH E 412     6.027  54.185  14.799  1.00 37.09        O
HETATM15168  O   HOH E 413    26.229  47.989  32.440  1.00 33.02        O
HETATM15169  O   HOH E 414    38.626  32.743  65.825  1.00 36.73        O
HETATM15170  O   HOH E 415    36.236  56.150  49.831  1.00 39.78        O
HETATM15171  O   HOH E 416    51.246  43.294  51.445  1.00 54.98        O
HETATM15172  O   HOH E 417    21.825  43.218  27.290  1.00 46.44        O
HETATM15173  O   HOH E 418    15.707  62.063  19.691  1.00 38.74        O
HETATM15174  O   HOH E 419     8.917  60.289  34.402  1.00 47.68        O
HETATM15175  O   HOH E 420    -0.247  32.396  25.137  1.00 37.46        O
HETATM15176  O   HOH E 421    22.890  44.915  22.462  1.00 41.57        O
HETATM15177  O   HOH E 422    39.290  47.702  38.720  1.00 40.79        O
HETATM15178  O   HOH E 423    45.959  49.497  55.331  1.00 39.08        O
HETATM15179  O   HOH E 424    21.221  57.519  17.541  1.00 42.89        O
HETATM15180  O   HOH E 425    19.461  42.929  28.016  1.00 37.82        O
HETATM15181  O   HOH E 426     3.518  43.493   9.161  1.00 40.43        O
HETATM15182  O   HOH E 427    22.084  51.045  40.621  1.00 36.75        O
HETATM15183  O   HOH E 428    38.230  41.566  47.999  1.00 52.16        O
HETATM15184  O   HOH E 429    23.394  54.163  80.318  1.00 35.00        O
HETATM15185  O   HOH E 430    21.609  39.870  25.898  1.00 38.76        O
HETATM15186  O   HOH E 431    45.176  49.253  52.752  1.00 39.76        O
HETATM15187  O   HOH E 432     8.942  55.787  15.728  1.00 40.64        O
HETATM15188  O   HOH E 433    43.774  50.874  50.869  1.00 44.84        O
HETATM15189  O   HOH E 434    16.876  39.953  37.744  1.00 38.86        O
HETATM15190  O   HOH E 435    13.833  37.157  35.050  1.00 47.02        O
HETATM15191  O   HOH E 436    38.948  57.231  42.810  1.00 37.58        O
HETATM15192  O   HOH E 437     2.457  53.621  11.569  1.00 45.42        O
HETATM15193  O   HOH E 438     7.900  69.922  29.195  1.00 46.58        O
HETATM15194  O   HOH E 439    19.564  65.679  25.293  1.00 59.74        O
HETATM15195  O   HOH E 440    15.442  48.955  41.619  1.00 53.55        O
HETATM15196  O   HOH E 441    39.388  40.594  76.652  1.00 41.83        O
HETATM15197  O   HOH E 442    30.937  51.723  25.755  1.00 45.04        O
HETATM15198  O   HOH E 443     8.432  67.592  25.341  1.00 45.38        O
```

| | | | | | | | | | | |
|---|---|---|---|---|---|---|---|---|---|---|
| HETATM15199 | O | HOH | E | 444 | 12.976 | 39.045 | 37.942 | 1.00 | 42.84 | O |
| HETATM15200 | O | HOH | E | 445 | 18.915 | 58.572 | 40.870 | 1.00 | 39.48 | O |
| HETATM15201 | O | HOH | E | 446 | 18.637 | 58.107 | 76.384 | 1.00 | 37.69 | O |
| HETATM15202 | O | HOH | E | 447 | 23.665 | 59.960 | 74.447 | 1.00 | 52.51 | O |
| HETATM15203 | O | HOH | E | 448 | -5.903 | 48.970 | 13.033 | 1.00 | 47.56 | O |
| HETATM15204 | O | HOH | E | 449 | 42.301 | 54.527 | 62.346 | 1.00 | 50.45 | O |
| HETATM15205 | O | HOH | E | 450 | 12.043 | 35.964 | 20.138 | 1.00 | 42.40 | O |
| HETATM15206 | O | HOH | E | 451 | 9.822 | 35.461 | 18.616 | 1.00 | 37.78 | O |
| HETATM15207 | O | HOH | E | 452 | 40.407 | 54.788 | 52.973 | 1.00 | 42.09 | O |
| HETATM15208 | O | HOH | E | 453 | 24.126 | 54.678 | 46.971 | 1.00 | 54.86 | O |
| HETATM15209 | O | HOH | E | 454 | -4.424 | 36.808 | 23.209 | 1.00 | 40.11 | O |
| HETATM15210 | O | HOH | E | 455 | -6.572 | 50.382 | 15.239 | 1.00 | 41.06 | O |
| HETATM15211 | O | HOH | E | 456 | -4.126 | 36.597 | 26.592 | 1.00 | 40.46 | O |
| HETATM15212 | O | HOH | E | 457 | 35.758 | 36.214 | 58.452 | 1.00 | 40.19 | O |
| HETATM15213 | O | HOH | E | 458 | 34.378 | 35.149 | 69.968 | 1.00 | 44.01 | O |
| HETATM15214 | O | HOH | E | 459 | -8.538 | 52.751 | 14.910 | 1.00 | 45.41 | O |
| HETATM15215 | O | HOH | E | 460 | 24.898 | 35.411 | 67.399 | 1.00 | 46.99 | O |
| HETATM15216 | O | HOH | E | 461 | 37.187 | 54.868 | 69.359 | 1.00 | 46.72 | O |
| HETATM15217 | O | HOH | E | 462 | -9.829 | 50.725 | 15.346 | 1.00 | 49.26 | O |
| HETATM15218 | O | HOH | E | 463 | 43.563 | 45.960 | 38.184 | 1.00 | 49.76 | O |
| HETATM15219 | O | HOH | E | 464 | 34.504 | 33.484 | 72.193 | 1.00 | 49.47 | O |
| HETATM15220 | O | HOH | E | 465 | 23.770 | 47.669 | 41.800 | 1.00 | 46.57 | O |
| HETATM15221 | O | HOH | E | 466 | 37.256 | 56.644 | 61.235 | 1.00 | 41.07 | O |
| HETATM15222 | O | HOH | E | 467 | 41.415 | 37.146 | 55.877 | 1.00 | 61.49 | O |
| HETATM15223 | O | HOH | E | 468 | 24.581 | 40.653 | 29.086 | 1.00 | 51.52 | O |
| HETATM15224 | O | HOH | E | 469 | 22.531 | 41.444 | 52.323 | 1.00 | 50.01 | O |
| HETATM15225 | O | HOH | E | 470 | 35.068 | 53.309 | 71.492 | 1.00 | 45.04 | O |
| HETATM15226 | O | HOH | E | 471 | 1.948 | 55.722 | 12.898 | 1.00 | 45.47 | O |
| HETATM15227 | O | HOH | E | 472 | 23.744 | 44.537 | 27.577 | 1.00 | 52.05 | O |
| HETATM15228 | O | HOH | E | 473 | -0.003 | 53.855 | 12.266 | 1.00 | 43.62 | O |
| HETATM15229 | O | HOH | E | 474 | 22.022 | 53.978 | 44.630 | 1.00 | 53.96 | O |
| HETATM15230 | O | HOH | E | 475 | 8.561 | 70.671 | 25.639 | 1.00 | 63.94 | O |
| HETATM15231 | O | HOH | E | 476 | 34.549 | 34.161 | 78.388 | 1.00 | 61.18 | O |
| HETATM15232 | O | HOH | E | 477 | 34.456 | 58.802 | 28.006 | 1.00 | 50.62 | O |
| HETATM15233 | O | HOH | E | 478 | 15.520 | 50.217 | 43.711 | 1.00 | 50.85 | O |
| HETATM15234 | O | HOH | E | 479 | 37.107 | 59.725 | 39.423 | 1.00 | 55.71 | O |
| HETATM15235 | O | HOH | E | 480 | 25.612 | 65.786 | 42.503 | 1.00 | 53.24 | O |
| HETATM15236 | O | HOH | E | 481 | -1.488 | 33.814 | 29.430 | 1.00 | 53.15 | O |
| HETATM15237 | O | HOH | E | 482 | 35.796 | 58.056 | 58.769 | 1.00 | 53.64 | O |
| HETATM15238 | O | HOH | E | 483 | 35.558 | 41.737 | 80.985 | 1.00 | 61.87 | O |
| HETATM15239 | O | HOH | E | 484 | 20.249 | 65.990 | 33.559 | 1.00 | 48.49 | O |
| HETATM15240 | O | HOH | E | 485 | 33.321 | 59.091 | 46.130 | 1.00 | 46.42 | O |
| HETATM15241 | O | HOH | E | 486 | 18.769 | 61.123 | 38.356 | 1.00 | 57.28 | O |
| HETATM15242 | O | HOH | E | 487 | 19.787 | 41.049 | 73.480 | 1.00 | 41.93 | O |
| HETATM15243 | O | HOH | E | 488 | 25.990 | 47.526 | 39.743 | 1.00 | 43.99 | O |
| HETATM15244 | O | HOH | E | 489 | 1.034 | 44.955 | 7.644 | 1.00 | 44.13 | O |
| HETATM15245 | O | HOH | E | 490 | 5.190 | 60.931 | 26.454 | 1.00 | 49.02 | O |
| HETATM15246 | O | HOH | E | 491 | 43.299 | 50.129 | 42.028 | 1.00 | 44.77 | O |
| HETATM15247 | O | HOH | E | 492 | 18.008 | 36.562 | 34.042 | 1.00 | 57.35 | O |
| HETATM15248 | O | HOH | E | 493 | 3.531 | 45.314 | 30.156 | 1.00 | 37.24 | O |
| HETATM15249 | O | HOH | E | 494 | 37.103 | 47.084 | 37.749 | 1.00 | 50.81 | O |
| HETATM15250 | O | HOH | E | 495 | 38.039 | 58.137 | 47.163 | 1.00 | 49.28 | O |
| HETATM15251 | O | HOH | E | 496 | 22.697 | 43.216 | 24.826 | 1.00 | 47.47 | O |
| HETATM15252 | O | HOH | E | 497 | 44.640 | 38.238 | 57.293 | 1.00 | 60.40 | O |
| HETATM15253 | O | HOH | E | 498 | 8.051 | 30.132 | 18.641 | 1.00 | 50.80 | O |
| HETATM15254 | O | HOH | E | 499 | 23.329 | 39.476 | 54.287 | 1.00 | 50.62 | O |
| HETATM15255 | O | HOH | E | 500 | 25.912 | 37.347 | 53.422 | 1.00 | 49.35 | O |
| HETATM15256 | O | HOH | E | 501 | 39.948 | 57.274 | 52.198 | 1.00 | 50.30 | O |
| HETATM15257 | O | HOH | E | 502 | 16.175 | 66.541 | 28.363 | 1.00 | 52.97 | O |
| HETATM15258 | O | HOH | E | 503 | 18.900 | 47.573 | 42.143 | 1.00 | 67.22 | O |
| HETATM15259 | O | HOH | E | 504 | 29.319 | 33.530 | 70.743 | 1.00 | 57.35 | O |
| HETATM15260 | O | HOH | E | 505 | 21.304 | 41.711 | 19.872 | 1.00 | 46.82 | O |
| HETATM15261 | O | HOH | E | 506 | 27.407 | 41.838 | 38.658 | 1.00 | 68.55 | O |
| HETATM15262 | O | HOH | E | 507 | 12.738 | 58.803 | 17.056 | 1.00 | 49.63 | O |
| HETATM15263 | O | HOH | E | 508 | 35.901 | 60.199 | 43.063 | 1.00 | 70.52 | O |

| | | | | | | | | | | |
|---|---|---|---|---|---|---|---|---|---|---|
| HETATM15264 | O | HOH | E | 509 | 25.794 | 34.866 | 61.103 | 1.00 | 65.79 | O |
| HETATM15265 | O | HOH | E | 510 | 7.350 | 36.459 | 14.077 | 1.00 | 62.56 | O |
| HETATM15266 | O | HOH | E | 511 | 13.247 | 46.821 | 42.651 | 1.00 | 50.32 | O |
| HETATM15267 | O | HOH | E | 512 | 32.607 | 38.651 | 58.542 | 1.00 | 45.29 | O |
| HETATM15268 | O | HOH | E | 513 | 19.176 | 65.882 | 36.607 | 1.00 | 53.91 | O |
| HETATM15269 | O | HOH | E | 514 | 3.812 | 36.141 | 35.286 | 1.00 | 48.48 | O |
| HETATM15270 | O | HOH | E | 515 | 6.997 | 40.391 | 12.278 | 1.00 | 55.43 | O |
| HETATM15271 | O | HOH | E | 516 | -2.224 | 53.825 | 14.104 | 1.00 | 55.59 | O |
| HETATM15272 | O | HOH | E | 517 | 37.511 | 59.040 | 50.392 | 1.00 | 58.57 | O |
| HETATM15273 | O | HOH | E | 518 | 26.614 | 55.684 | 79.720 | 1.00 | 46.24 | O |
| HETATM15274 | O | HOH | E | 519 | 18.639 | 44.106 | 41.111 | 1.00 | 51.36 | O |
| HETATM15275 | O | HOH | E | 520 | 39.697 | 57.578 | 45.172 | 1.00 | 56.66 | O |
| HETATM15276 | O | HOH | E | 521 | 13.533 | 33.812 | 28.169 | 1.00 | 43.40 | O |
| HETATM15277 | O | HOH | E | 522 | 21.016 | 39.706 | 22.791 | 1.00 | 60.58 | O |
| HETATM15278 | O | HOH | E | 523 | 48.636 | 42.212 | 53.779 | 1.00 | 75.71 | O |
| HETATM15279 | O | HOH | E | 524 | 36.405 | 57.230 | 26.736 | 1.00 | 48.34 | O |
| HETATM15280 | O | HOH | E | 525 | 27.140 | 44.711 | 44.325 | 1.00 | 53.43 | O |
| HETATM15281 | O | HOH | E | 526 | 23.026 | 53.251 | 82.584 | 1.00 | 33.74 | O |
| HETATM15282 | O | HOH | E | 527 | 2.091 | 30.831 | 28.912 | 1.00 | 16.24 | O |
| HETATM15283 | O | HOH | F | 311 | 5.183 | 59.413 | 31.113 | 1.00 | 36.00 | O |
| HETATM15284 | O | HOH | F | 312 | 4.983 | 60.146 | 28.848 | 1.00 | 47.37 | O |
| HETATM15285 | O | HOH | F | 313 | -3.979 | 54.747 | 31.044 | 1.00 | 17.30 | O |
| HETATM15286 | O | HOH | F | 314 | 5.506 | 40.388 | 33.630 | 1.00 | 12.72 | O |
| HETATM15287 | O | HOH | F | 315 | 0.808 | 39.012 | 35.159 | 1.00 | 20.27 | O |
| HETATM15288 | O | HOH | F | 316 | -5.804 | 40.905 | 24.949 | 1.00 | 21.33 | O |
| HETATM15289 | O | HOH | F | 317 | 6.303 | 48.798 | 56.984 | 1.00 | 23.13 | O |
| HETATM15290 | O | HOH | F | 318 | 12.104 | 47.211 | 68.320 | 1.00 | 16.88 | O |
| HETATM15291 | O | HOH | F | 319 | 9.651 | 52.798 | 66.024 | 1.00 | 19.56 | O |
| HETATM15292 | O | HOH | F | 320 | 27.909 | 58.554 | 54.343 | 1.00 | 21.00 | O |
| HETATM15293 | O | HOH | F | 321 | -10.005 | 37.096 | 52.689 | 1.00 | 23.23 | O |
| HETATM15294 | O | HOH | F | 322 | 6.326 | 56.914 | 33.483 | 1.00 | 17.09 | O |
| HETATM15295 | O | HOH | F | 323 | 11.601 | 52.313 | 69.221 | 1.00 | 23.59 | O |
| HETATM15296 | O | HOH | F | 324 | 5.693 | 40.505 | 41.656 | 1.00 | 24.21 | O |
| HETATM15297 | O | HOH | F | 325 | 1.889 | 50.809 | 66.139 | 1.00 | 17.38 | O |
| HETATM15298 | O | HOH | F | 326 | -0.232 | 54.878 | 26.738 | 1.00 | 18.02 | O |
| HETATM15299 | O | HOH | F | 327 | 4.879 | 45.805 | 53.940 | 1.00 | 21.82 | O |
| HETATM15300 | O | HOH | F | 328 | -12.388 | 47.027 | 44.438 | 1.00 | 22.96 | O |
| HETATM15301 | O | HOH | F | 329 | 0.080 | 50.737 | 61.831 | 1.00 | 20.82 | O |
| HETATM15302 | O | HOH | F | 330 | 15.570 | 55.955 | 72.121 | 1.00 | 22.58 | O |
| HETATM15303 | O | HOH | F | 331 | -11.672 | 52.486 | 26.300 | 1.00 | 25.54 | O |
| HETATM15304 | O | HOH | F | 332 | 2.894 | 49.404 | 53.711 | 1.00 | 23.75 | O |
| HETATM15305 | O | HOH | F | 333 | 16.129 | 49.147 | 72.573 | 1.00 | 20.46 | O |
| HETATM15306 | O | HOH | F | 334 | 23.730 | 59.886 | 70.295 | 1.00 | 25.80 | O |
| HETATM15307 | O | HOH | F | 335 | -3.458 | 50.274 | 56.088 | 1.00 | 25.17 | O |
| HETATM15308 | O | HOH | F | 336 | 9.336 | 54.013 | 63.464 | 1.00 | 17.75 | O |
| HETATM15309 | O | HOH | F | 337 | 6.088 | 37.856 | 46.996 | 1.00 | 23.56 | O |
| HETATM15310 | O | HOH | F | 338 | -4.104 | 57.236 | 48.322 | 1.00 | 23.99 | O |
| HETATM15311 | O | HOH | F | 339 | -3.498 | 45.508 | 57.433 | 1.00 | 22.55 | O |
| HETATM15312 | O | HOH | F | 340 | -14.436 | 42.612 | 38.404 | 1.00 | 25.73 | O |
| HETATM15313 | O | HOH | F | 341 | 4.098 | 47.892 | 55.469 | 1.00 | 23.72 | O |
| HETATM15314 | O | HOH | F | 342 | -0.123 | 37.106 | 33.553 | 1.00 | 34.02 | O |
| HETATM15315 | O | HOH | F | 343 | -0.948 | 56.360 | 30.252 | 1.00 | 27.76 | O |
| HETATM15316 | O | HOH | F | 344 | 5.494 | 57.786 | 35.910 | 1.00 | 25.77 | O |
| HETATM15317 | O | HOH | F | 345 | -1.041 | 35.034 | 35.957 | 1.00 | 27.23 | O |
| HETATM15318 | O | HOH | F | 346 | -0.568 | 53.035 | 22.126 | 1.00 | 23.67 | O |
| HETATM15319 | O | HOH | F | 347 | -1.893 | 58.116 | 27.999 | 1.00 | 30.28 | O |
| HETATM15320 | O | HOH | F | 348 | -14.210 | 56.261 | 32.850 | 1.00 | 26.61 | O |
| HETATM15321 | O | HOH | F | 349 | -14.007 | 42.388 | 27.486 | 1.00 | 34.74 | O |
| HETATM15322 | O | HOH | F | 350 | -9.936 | 38.613 | 32.971 | 1.00 | 31.02 | O |
| HETATM15323 | O | HOH | F | 351 | 4.883 | 55.784 | 59.623 | 1.00 | 32.98 | O |
| HETATM15324 | O | HOH | F | 352 | 4.179 | 51.292 | 51.764 | 1.00 | 27.88 | O |
| HETATM15325 | O | HOH | F | 353 | 16.852 | 53.874 | 70.933 | 1.00 | 26.92 | O |
| HETATM15326 | O | HOH | F | 354 | 31.153 | 60.724 | 69.348 | 1.00 | 31.29 | O |
| HETATM15327 | O | HOH | F | 355 | 14.654 | 65.933 | 62.225 | 1.00 | 32.45 | O |
| HETATM15328 | O | HOH | F | 356 | 7.945 | 39.988 | 63.889 | 1.00 | 26.15 | O |

```
HETATM15329  O   HOH F 357     27.236  54.634  68.694  1.00 30.05           O
HETATM15330  O   HOH F 358     -8.235  46.807  22.293  1.00 27.18           O
HETATM15331  O   HOH F 359     -3.141  52.088  18.769  1.00 28.88           O
HETATM15332  O   HOH F 360     19.987  61.313  71.385  1.00 31.21           O
HETATM15333  O   HOH F 361     12.252  43.083  41.383  1.00 29.35           O
HETATM15334  O   HOH F 362     24.528  68.082  68.057  1.00 35.50           O
HETATM15335  O   HOH F 363      3.599  39.439  44.607  1.00 24.19           O
HETATM15336  O   HOH F 364     13.122  49.790  53.710  1.00 39.23           O
HETATM15337  O   HOH F 365      7.679  53.789  68.107  1.00 24.65           O
HETATM15338  O   HOH F 366      1.514  55.053  66.046  1.00 18.94           O
HETATM15339  O   HOH F 367     32.903  58.312  62.163  1.00 37.38           O
HETATM15340  O   HOH F 368      8.985  42.317  54.911  1.00 27.45           O
HETATM15341  O   HOH F 369     13.176  50.763  70.870  1.00 23.63           O
HETATM15342  O   HOH F 370     19.045  67.501  63.690  1.00 27.00           O
HETATM15343  O   HOH F 371     -2.314  44.283  19.187  1.00 29.88           O
HETATM15344  O   HOH F 372     23.351  66.213  66.750  1.00 30.29           O
HETATM15345  O   HOH F 373     17.288  46.167  68.996  1.00 30.18           O
HETATM15346  O   HOH F 374    -16.392  56.900  29.287  1.00 31.47           O
HETATM15347  O   HOH F 375     -2.723  62.294  43.517  1.00 28.09           O
HETATM15348  O   HOH F 376     10.972  60.287  68.517  1.00 26.94           O
HETATM15349  O   HOH F 377      9.604  37.618  46.184  1.00 42.24           O
HETATM15350  O   HOH F 378     16.714  62.147  69.753  1.00 29.17           O
HETATM15351  O   HOH F 379     -1.775  68.963  35.342  1.00 37.06           O
HETATM15352  O   HOH F 380    -14.470  42.276  31.411  1.00 48.89           O
HETATM15353  O   HOH F 381      7.192  58.111  68.736  1.00 32.39           O
HETATM15354  O   HOH F 382     13.903  53.990  68.923  1.00 24.05           O
HETATM15355  O   HOH F 383    -15.803  50.368  39.147  1.00 23.98           O
HETATM15356  O   HOH F 384     11.870  58.986  57.491  1.00 30.48           O
HETATM15357  O   HOH F 385      1.413  53.026  51.445  1.00 31.08           O
HETATM15358  O   HOH F 386     22.676  47.815  57.465  1.00 26.25           O
HETATM15359  O   HOH F 387     -5.086  38.083  31.342  1.00 41.15           O
HETATM15360  O   HOH F 388     13.830  56.644  69.924  1.00 34.44           O
HETATM15361  O   HOH F 389     -6.492  46.459  15.645  1.00 33.26           O
HETATM15362  O   HOH F 390     -7.844  49.326  21.321  1.00 31.52           O
HETATM15363  O   HOH F 391      0.757  38.151  60.882  1.00 43.83           O
HETATM15364  O   HOH F 392      0.277  31.481  52.148  1.00 35.71           O
HETATM15365  O   HOH F 393     -3.328  32.447  58.327  1.00 36.75           O
HETATM15366  O   HOH F 394     -2.688  36.101  32.966  1.00 41.39           O
HETATM15367  O   HOH F 395     15.020  54.691  53.470  1.00 39.46           O
HETATM15368  O   HOH F 396     33.927  60.740  57.100  1.00 40.03           O
HETATM15369  O   HOH F 397     21.055  45.931  56.636  1.00 34.00           O
HETATM15370  O   HOH F 398     20.633  55.377  49.851  1.00 36.19           O
HETATM15371  O   HOH F 399     23.203  39.268  63.720  1.00 36.68           O
HETATM15372  O   HOH F 400     -7.248  63.898  42.350  1.00 35.32           O
HETATM15373  O   HOH F 401     17.100  50.253  52.761  1.00 33.42           O
HETATM15374  O   HOH F 402     -9.604  38.673  24.084  1.00 30.71           O
HETATM15375  O   HOH F 403     17.910  38.793  54.764  1.00 44.93           O
HETATM15376  O   HOH F 404      6.270  56.184  67.612  1.00 27.25           O
HETATM15377  O   HOH F 405      3.190  60.152  40.606  1.00 40.56           O
HETATM15378  O   HOH F 406      4.088  33.573  56.885  1.00 35.99           O
HETATM15379  O   HOH F 407     34.663  70.542  58.219  1.00 37.24           O
HETATM15380  O   HOH F 408      6.294  57.726  40.189  1.00 30.13           O
HETATM15381  O   HOH F 409    -13.155  45.864  52.119  1.00 33.23           O
HETATM15382  O   HOH F 410     -1.709  53.899  53.757  1.00 35.76           O
HETATM15383  O   HOH F 411      6.794  42.029  68.655  1.00 29.67           O
HETATM15384  O   HOH F 412     11.838  44.058  43.863  1.00 35.87           O
HETATM15385  O   HOH F 413      5.686  37.219  53.619  1.00 26.10           O
HETATM15386  O   HOH F 414    -18.152  58.090  39.800  1.00 36.96           O
HETATM15387  O   HOH F 415    -11.563  38.596  39.835  1.00 31.51           O
HETATM15388  O   HOH F 416     -4.863  67.921  32.858  1.00 33.83           O
HETATM15389  O   HOH F 417     26.321  68.683  60.990  1.00 32.37           O
HETATM15390  O   HOH F 418     17.722  41.701  61.965  1.00 38.62           O
HETATM15391  O   HOH F 419      5.179  58.002  65.725  1.00 40.51           O
HETATM15392  O   HOH F 420      5.051  61.586  33.346  1.00 35.17           O
HETATM15393  O   HOH F 421      2.203  31.312  56.995  1.00 33.64           O
```

```
HETATM15394  O   HOH F 422      6.102  37.053  64.836  1.00 29.91           O
HETATM15395  O   HOH F 423    -14.666  53.377  25.127  1.00 44.65           O
HETATM15396  O   HOH F 424    -16.899  55.750  38.581  1.00 39.56           O
HETATM15397  O   HOH F 425     17.432  60.609  71.983  1.00 46.29           O
HETATM15398  O   HOH F 426      6.574  36.176  56.484  1.00 43.98           O
HETATM15399  O   HOH F 427    -10.972  56.122  20.237  1.00 43.27           O
HETATM15400  O   HOH F 428    -13.256  46.790  27.346  1.00 30.44           O
HETATM15401  O   HOH F 429     -5.685  51.633  55.121  1.00 38.08           O
HETATM15402  O   HOH F 430    -10.870  41.936  18.013  1.00 37.14           O
HETATM15403  O   HOH F 431    -15.753  37.054  19.897  1.00 50.89           O
HETATM15404  O   HOH F 432    -19.189  49.551  26.026  1.00 50.81           O
HETATM15405  O   HOH F 433    -11.991  40.460  45.448  1.00 37.33           O
HETATM15406  O   HOH F 434     -0.699  42.718  17.070  1.00 36.10           O
HETATM15407  O   HOH F 435     15.188  44.708  68.799  1.00 38.19           O
HETATM15408  O   HOH F 436     -4.507  47.506  56.071  1.00 29.61           O
HETATM15409  O   HOH F 437    -14.328  46.484  48.222  1.00 34.68           O
HETATM15410  O   HOH F 438    -16.092  43.196  33.383  1.00 55.73           O
HETATM15411  O   HOH F 439     -5.844  48.087  53.510  1.00 41.78           O
HETATM15412  O   HOH F 440      0.935  53.421  57.577  1.00 32.66           O
HETATM15413  O   HOH F 441     -4.492  42.959  15.802  1.00 43.93           O
HETATM15414  O   HOH F 442     16.759  66.253  67.062  1.00 42.74           O
HETATM15415  O   HOH F 443     26.668  56.442  70.729  1.00 30.95           O
HETATM15416  O   HOH F 444     -9.358  63.665  37.561  1.00 37.04           O
HETATM15417  O   HOH F 445     16.520  42.568  67.047  1.00 33.61           O
HETATM15418  O   HOH F 446      2.285  43.305  66.693  1.00 34.96           O
HETATM15419  O   HOH F 447      8.342  45.649  50.924  1.00 45.97           O
HETATM15420  O   HOH F 448     19.974  63.697  72.959  1.00 40.85           O
HETATM15421  O   HOH F 449      3.573  64.023  32.957  1.00 39.40           O
HETATM15422  O   HOH F 450    -16.865  58.988  34.413  1.00 39.45           O
HETATM15423  O   HOH F 451     17.106  42.818  64.548  1.00 35.36           O
HETATM15424  O   HOH F 452    -15.046  50.368  45.076  1.00 41.72           O
HETATM15425  O   HOH F 453    -12.295  43.819  18.977  1.00 36.25           O
HETATM15426  O   HOH F 454    -11.552  49.171  26.507  1.00 35.72           O
HETATM15427  O   HOH F 455     15.562  64.416  68.927  1.00 33.18           O
HETATM15428  O   HOH F 456     26.028  65.595  54.095  1.00 41.58           O
HETATM15429  O   HOH F 457      9.853  44.852  45.006  1.00 39.33           O
HETATM15430  O   HOH F 458     -5.806  64.169  30.959  1.00 31.10           O
HETATM15431  O   HOH F 459    -14.808  41.487  48.794  1.00 51.08           O
HETATM15432  O   HOH F 460     -7.233  48.641  17.183  1.00 30.98           O
HETATM15433  O   HOH F 461     14.856  51.982  53.718  1.00 37.21           O
HETATM15434  O   HOH F 462     31.040  71.465  65.547  1.00 47.07           O
HETATM15435  O   HOH F 463      9.062  52.011  44.702  1.00 42.26           O
HETATM15436  O   HOH F 464     -7.345  39.260  30.962  1.00 35.48           O
HETATM15437  O   HOH F 465      4.943  55.926  57.296  1.00 41.41           O
HETATM15438  O   HOH F 466     17.510  62.853  54.367  1.00 44.39           O
HETATM15439  O   HOH F 467    -12.128  44.833  21.272  1.00 38.34           O
HETATM15440  O   HOH F 468     10.730  64.861  60.871  1.00 50.03           O
HETATM15441  O   HOH F 469     -3.769  59.763  49.145  1.00 48.02           O
HETATM15442  O   HOH F 470      8.920  39.071  66.421  1.00 36.14           O
HETATM15443  O   HOH F 471      3.421  55.075  49.125  1.00 47.46           O
HETATM15444  O   HOH F 472     32.381  59.497  76.742  1.00 62.07           O
HETATM15445  O   HOH F 473      6.653  56.179  55.363  1.00 38.77           O
HETATM15446  O   HOH F 474      8.417  54.081  54.423  1.00 44.96           O
HETATM15447  O   HOH F 475      2.684  58.218  25.094  1.00 46.84           O
HETATM15448  O   HOH F 476      3.831  65.671  35.195  1.00 46.72           O
HETATM15449  O   HOH F 477    -15.024  40.276  38.492  1.00 47.37           O
HETATM15450  O   HOH F 478     -3.694  55.375  21.162  1.00 33.93           O
HETATM15451  O   HOH F 479     -4.455  53.711  17.129  1.00 40.66           O
HETATM15452  O   HOH F 480      7.052  36.633  51.257  1.00 35.52           O
HETATM15453  O   HOH F 481    -15.327  46.717  39.397  1.00 42.70           O
HETATM15454  O   HOH F 482    -10.590  44.207  53.270  1.00 41.55           O
HETATM15455  O   HOH F 483      2.387  56.779  60.264  1.00 42.22           O
HETATM15456  O   HOH F 484     11.271  63.066  57.434  1.00 38.90           O
HETATM15457  O   HOH F 485      8.131  42.832  48.491  1.00 39.76           O
HETATM15458  O   HOH F 486     -6.248  58.136  24.303  1.00 48.61           O
```

```
HETATM15459  O   HOH F 487    -11.505  51.962  18.878  1.00 48.40           O
HETATM15460  O   HOH F 488    -15.645  52.971  20.695  1.00 49.71           O
HETATM15461  O   HOH F 489    -14.015  49.276  22.252  1.00 52.47           O
HETATM15462  O   HOH F 490     -1.995  56.956  23.427  1.00 38.71           O
HETATM15463  O   HOH F 491     -9.335  42.762  16.111  1.00 50.88           O
HETATM15464  O   HOH F 492     -5.631  37.539  18.122  1.00 50.35           O
HETATM15465  O   HOH F 493    -13.881  38.592  24.500  1.00 51.02           O
HETATM15466  O   HOH F 494    -12.891  40.106  26.706  1.00 38.65           O
HETATM15467  O   HOH F 495    -11.682  38.722  29.479  1.00 58.66           O
HETATM15468  O   HOH F 496    -10.096  38.296  15.404  1.00 45.57           O
HETATM15469  O   HOH F 497      1.906  56.475  47.840  1.00 44.93           O
HETATM15470  O   HOH F 498      3.769  59.308  37.266  1.00 36.63           O
HETATM15471  O   HOH F 499     38.386  57.830  69.355  1.00 50.46           O
HETATM15472  O   HOH F 500     -4.867  32.465  47.251  1.00 49.76           O
HETATM15473  O   HOH F 501     -3.583  36.411  19.474  1.00 47.87           O
HETATM15474  O   HOH F 502      7.619  36.642  34.447  1.00 39.06           O
HETATM15475  O   HOH F 503      7.178  42.235  53.008  1.00 34.15           O
HETATM15476  O   HOH F 504      5.822  37.991  33.277  1.00 33.12           O
HETATM15477  O   HOH F 505     24.884  67.588  70.926  1.00 44.78           O
HETATM15478  O   HOH F 506     27.239  74.460  57.645  1.00 39.74           O
HETATM15479  O   HOH F 507    -14.636  45.860  45.516  1.00 37.21           O
HETATM15480  O   HOH F 508     -6.186  67.710  39.706  1.00 52.12           O
HETATM15481  O   HOH F 509     34.245  59.821  60.672  1.00 29.68           O
HETATM15482  O   HOH F 510     -0.749  35.279  43.655  1.00 39.05           O
HETATM15483  O   HOH F 511    -16.623  46.973  36.456  1.00 39.80           O
HETATM15484  O   HOH F 512     -7.404  52.241  50.890  1.00 49.43           O
HETATM15485  O   HOH F 513    -13.632  40.295  34.742  1.00 45.28           O
HETATM15486  O   HOH F 514      6.681  35.853  48.760  1.00 35.99           O
HETATM15487  O   HOH F 515      6.446  44.162  50.233  1.00 36.79           O
HETATM15488  O   HOH F 516    -16.918  59.263  31.644  1.00 46.52           O
HETATM15489  O   HOH F 517    -12.059  39.881  32.474  1.00 50.99           O
HETATM15490  O   HOH F 518    -10.683  56.859  25.087  1.00 41.22           O
HETATM15491  O   HOH F 519     13.122  35.046  59.310  1.00 47.68           O
HETATM15492  O   HOH F 520     -0.875  52.016  55.262  1.00 41.54           O
HETATM15493  O   HOH F 521      0.793  40.414  67.797  1.00 48.30           O
HETATM15494  O   HOH F 522     34.471  61.853  68.338  1.00 55.17           O
HETATM15495  O   HOH F 523      6.856  39.144  35.768  1.00 43.76           O
HETATM15496  O   HOH F 524     -0.890  67.018  40.475  1.00 46.43           O
HETATM15497  O   HOH F 525      3.955  37.453  42.987  1.00 37.32           O
HETATM15498  O   HOH F 526      2.698  55.805  25.106  1.00 43.43           O
HETATM15499  O   HOH F 527     14.222  59.221  70.237  1.00 40.42           O
HETATM15500  O   HOH F 528     -9.776  49.517  19.070  1.00 45.09           O
HETATM15501  O   HOH F 529     15.697  47.319  52.475  1.00 35.90           O
HETATM15502  O   HOH F 530     12.930  40.365  52.841  1.00 38.54           O
HETATM15503  O   HOH F 531     17.897  57.580  53.417  1.00 37.15           O
HETATM15504  O   HOH F 532     16.843  39.767  64.366  1.00 53.35           O
HETATM15505  O   HOH F 533    -12.668  36.505  52.140  1.00 44.47           O
HETATM15506  O   HOH F 534    -12.995  37.811  46.320  1.00 46.77           O
HETATM15507  O   HOH F 535     10.072  47.485  50.233  1.00 43.52           O
HETATM15508  O   HOH F 536      9.425  49.922  49.587  1.00 31.30           O
HETATM15509  O   HOH F 537     -7.334  46.242  13.110  1.00 46.13           O
HETATM15510  O   HOH F 538      9.026  31.031  36.756  1.00 41.25           O
HETATM15511  O   HOH F 539      9.769  52.309  50.587  1.00 41.33           O
HETATM15512  O   HOH F 540     -9.956  62.242  45.685  1.00 54.81           O
HETATM15513  O   HOH F 541      5.096  40.190  68.453  1.00 43.31           O
HETATM15514  O   HOH F 542      1.222  56.674  23.197  1.00 40.61           O
HETATM15515  O   HOH F 543    -15.912  61.777  35.355  1.00 53.83           O
HETATM15516  O   HOH F 544     22.523  67.262  51.752  1.00 43.25           O
HETATM15517  O   HOH F 545    -16.383  48.756  24.713  1.00 59.11           O
HETATM15518  O   HOH F 546     14.439  58.562  52.295  1.00 44.66           O
HETATM15519  O   HOH F 547     37.544  60.407  71.311  1.00 64.50           O
HETATM15520  O   HOH F 548     19.828  40.888  66.593  1.00 50.62           O
HETATM15521  O   HOH F 549    -16.445  43.672  39.477  1.00 34.21           O
HETATM15522  O   HOH F 550     -6.685  59.384  27.712  1.00 52.23           O
HETATM15523  O   HOH F 551     10.050  62.876  62.637  1.00 44.49           O
```

| | | | | | | | | | | |
|---|---|---|---|---|---|---|---|---|---|---|
| HETATM15524 | O | HOH | F | 552 | -7.545 | 62.420 | 48.025 | 1.00 | 48.27 | O |
| HETATM15525 | O | HOH | F | 553 | 8.386 | 34.585 | 52.734 | 1.00 | 45.72 | O |
| HETATM15526 | O | HOH | F | 554 | 6.501 | 50.621 | 49.381 | 1.00 | 45.67 | O |
| HETATM15527 | O | HOH | F | 555 | -1.192 | 62.680 | 45.624 | 1.00 | 48.83 | O |
| HETATM15528 | O | HOH | F | 556 | 23.715 | 36.328 | 61.972 | 1.00 | 48.88 | O |
| HETATM15529 | O | HOH | F | 557 | 23.149 | 73.304 | 68.709 | 1.00 | 55.22 | O |
| HETATM15530 | O | HOH | F | 558 | -18.314 | 45.343 | 32.518 | 1.00 | 50.61 | O |
| HETATM15531 | O | HOH | F | 559 | 6.420 | 34.197 | 55.052 | 1.00 | 44.76 | O |
| HETATM15532 | O | HOH | F | 560 | -17.318 | 52.140 | 38.399 | 1.00 | 49.51 | O |
| HETATM15533 | O | HOH | F | 561 | 2.970 | 35.203 | 42.966 | 1.00 | 44.77 | O |
| HETATM15534 | O | HOH | F | 562 | -11.050 | 59.569 | 48.609 | 1.00 | 55.84 | O |
| HETATM15535 | O | HOH | F | 563 | 6.066 | 54.307 | 48.417 | 1.00 | 52.26 | O |
| HETATM15536 | O | HOH | F | 564 | -11.307 | 64.591 | 42.184 | 1.00 | 66.79 | O |
| HETATM15537 | O | HOH | F | 565 | 21.111 | 71.001 | 62.300 | 1.00 | 46.70 | O |
| HETATM15538 | O | HOH | F | 566 | -17.033 | 53.210 | 23.259 | 1.00 | 57.55 | O |
| HETATM15539 | O | HOH | F | 567 | 34.933 | 61.139 | 54.249 | 1.00 | 56.33 | O |
| HETATM15540 | O | HOH | F | 568 | -5.950 | 53.724 | 53.370 | 1.00 | 45.37 | O |
| HETATM15541 | O | HOH | F | 569 | 6.639 | 36.770 | 44.768 | 1.00 | 52.41 | O |
| HETATM15542 | O | HOH | F | 570 | 9.822 | 38.670 | 55.072 | 1.00 | 47.81 | O |
| HETATM15543 | O | HOH | F | 571 | -11.257 | 36.690 | 20.771 | 1.00 | 61.12 | O |
| HETATM15544 | O | HOH | F | 572 | 16.293 | 47.192 | 49.594 | 1.00 | 52.25 | O |
| HETATM15545 | O | HOH | F | 573 | 26.102 | 68.619 | 55.556 | 1.00 | 43.05 | O |
| HETATM15546 | O | HOH | F | 574 | 35.999 | 56.984 | 65.914 | 1.00 | 41.11 | O |
| HETATM15547 | O | HOH | F | 575 | 7.410 | 39.158 | 53.688 | 1.00 | 43.27 | O |
| HETATM15548 | O | HOH | F | 576 | 9.393 | 30.445 | 39.222 | 1.00 | 54.95 | O |
| HETATM15549 | O | HOH | F | 577 | 41.590 | 67.044 | 57.238 | 1.00 | 51.61 | O |
| HETATM15550 | O | HOH | F | 578 | 21.318 | 72.660 | 64.322 | 1.00 | 46.92 | O |
| HETATM15551 | O | HOH | F | 579 | -15.489 | 36.574 | 15.361 | 1.00 | 67.59 | O |
| HETATM15552 | O | HOH | F | 580 | 17.638 | 49.498 | 49.223 | 1.00 | 52.04 | O |
| HETATM15553 | O | HOH | F | 581 | 36.117 | 60.139 | 67.399 | 1.00 | 56.65 | O |
| HETATM15554 | O | HOH | F | 582 | 11.099 | 44.702 | 47.094 | 1.00 | 50.41 | O |
| HETATM15555 | O | HOH | F | 583 | 15.031 | 33.279 | 59.586 | 1.00 | 66.81 | O |
| HETATM15556 | O | HOH | F | 584 | -4.218 | 63.844 | 45.537 | 1.00 | 60.39 | O |
| HETATM15557 | O | HOH | F | 585 | -12.181 | 56.457 | 48.921 | 1.00 | 58.05 | O |
| HETATM15558 | O | HOH | F | 586 | 23.034 | 69.676 | 69.937 | 1.00 | 56.10 | O |
| HETATM15559 | O | HOH | F | 587 | 20.058 | 36.515 | 58.492 | 1.00 | 48.80 | O |
| HETATM15560 | O | HOH | F | 588 | 19.651 | 70.465 | 58.151 | 1.00 | 53.25 | O |
| HETATM15561 | O | HOH | F | 589 | 10.626 | 34.697 | 65.165 | 1.00 | 58.92 | O |
| HETATM15562 | O | HOH | F | 590 | 0.330 | 57.201 | 63.867 | 1.00 | 45.11 | O |
| HETATM15563 | O | HOH | F | 591 | 16.252 | 44.564 | 51.715 | 1.00 | 54.19 | O |
| HETATM15564 | O | HOH | F | 592 | 14.984 | 69.606 | 60.684 | 1.00 | 57.50 | O |
| HETATM15565 | O | HOH | F | 593 | 6.670 | 33.657 | 46.118 | 1.00 | 55.51 | O |
| HETATM15566 | O | HOH | F | 594 | -17.732 | 62.579 | 45.069 | 1.00 | 50.81 | O |
| HETATM15567 | O | HOH | F | 595 | 5.926 | 73.094 | 36.325 | 1.00 | 51.84 | O |
| HETATM15568 | O | HOH | F | 596 | -18.252 | 44.621 | 29.450 | 1.00 | 49.51 | O |
| HETATM15569 | O | HOH | F | 597 | 23.416 | 69.126 | 53.019 | 1.00 | 39.81 | O |
| HETATM15570 | O | HOH | G | 215 | 25.358 | 25.742 | -69.025 | 1.00 | 18.28 | O |
| HETATM15571 | O | HOH | G | 216 | 24.530 | 15.759 | -69.238 | 1.00 | 11.68 | O |
| HETATM15572 | O | HOH | G | 217 | 29.161 | 27.109 | -52.269 | 1.00 | 18.72 | O |
| HETATM15573 | O | HOH | G | 218 | 20.583 | 19.142 | -69.329 | 1.00 | 14.82 | O |
| HETATM15574 | O | HOH | G | 219 | 33.280 | 23.923 | -47.716 | 1.00 | 13.76 | O |
| HETATM15575 | O | HOH | G | 220 | 30.763 | 13.324 | -66.294 | 1.00 | 14.32 | O |
| HETATM15576 | O | HOH | G | 221 | 27.540 | 29.697 | -58.288 | 1.00 | 21.29 | O |
| HETATM15577 | O | HOH | G | 222 | 25.793 | 24.050 | -47.450 | 1.00 | 19.99 | O |
| HETATM15578 | O | HOH | G | 223 | 18.444 | 16.294 | -65.670 | 1.00 | 16.80 | O |
| HETATM15579 | O | HOH | G | 224 | 7.307 | 12.704 | -47.522 | 1.00 | 20.86 | O |
| HETATM15580 | O | HOH | G | 225 | 17.871 | 13.848 | -66.356 | 1.00 | 21.22 | O |
| HETATM15581 | O | HOH | G | 226 | 20.426 | 26.779 | -50.827 | 1.00 | 20.33 | O |
| HETATM15582 | O | HOH | G | 227 | 36.672 | 25.525 | -54.160 | 1.00 | 23.32 | O |
| HETATM15583 | O | HOH | G | 228 | 10.824 | 24.304 | -10.735 | 1.00 | 23.59 | O |
| HETATM15584 | O | HOH | G | 229 | -1.879 | 12.321 | -31.234 | 1.00 | 25.21 | O |
| HETATM15585 | O | HOH | G | 230 | 19.288 | 12.555 | -68.155 | 1.00 | 21.79 | O |
| HETATM15586 | O | HOH | G | 231 | 23.696 | 27.685 | -52.560 | 1.00 | 19.42 | O |
| HETATM15587 | O | HOH | G | 232 | 14.737 | 11.033 | -63.639 | 1.00 | 24.55 | O |
| HETATM15588 | O | HOH | G | 233 | 13.095 | 11.169 | -46.684 | 1.00 | 22.83 | O |

```
HETATM15589  O   HOH G 234   19.593  14.601 -70.098  1.00 20.96        O
HETATM15590  O   HOH G 235    5.513  25.557 -22.598  1.00 30.07        O
HETATM15591  O   HOH G 236    6.472  14.819 -39.485  1.00 22.70        O
HETATM15592  O   HOH G 237    5.780  22.038 -27.740  1.00 21.63        O
HETATM15593  O   HOH G 238   38.550  16.303 -60.469  1.00 17.50        O
HETATM15594  O   HOH G 239   26.162  13.825 -68.427  1.00 23.10        O
HETATM15595  O   HOH G 240    3.907  13.763 -39.255  1.00 24.35        O
HETATM15596  O   HOH G 241   31.169  15.461 -68.818  1.00 20.56        O
HETATM15597  O   HOH G 242   14.204  18.599 -65.281  1.00 24.37        O
HETATM15598  O   HOH G 243   40.876  20.097 -66.285  1.00 31.56        O
HETATM15599  O   HOH G 244   -9.671  18.806 -38.905  1.00 28.60        O
HETATM15600  O   HOH G 245   34.440  10.346 -56.958  1.00 23.28        O
HETATM15601  O   HOH G 246   19.924  10.961 -45.670  1.00 18.94        O
HETATM15602  O   HOH G 247   20.543  24.161 -63.721  1.00 34.91        O
HETATM15603  O   HOH G 248   22.148  12.208 -67.809  1.00 23.14        O
HETATM15604  O   HOH G 249   -6.135  12.527 -25.486  1.00 27.90        O
HETATM15605  O   HOH G 250   33.182  15.563 -71.988  1.00 21.72        O
HETATM15606  O   HOH G 251    2.803  16.574 -19.367  1.00 27.30        O
HETATM15607  O   HOH G 252   36.251  19.200 -53.848  1.00 26.83        O
HETATM15608  O   HOH G 253   36.511  10.490 -58.758  1.00 26.50        O
HETATM15609  O   HOH G 254   37.134  18.180 -71.309  1.00 27.46        O
HETATM15610  O   HOH G 255   24.277   5.622 -63.508  1.00 27.73        O
HETATM15611  O   HOH G 256   36.901  27.022 -47.643  1.00 22.59        O
HETATM15612  O   HOH G 257   21.661  10.312 -64.688  1.00 21.04        O
HETATM15613  O   HOH G 258   32.769  12.014 -70.240  1.00 32.81        O
HETATM15614  O   HOH G 259   20.702  20.718 -66.996  1.00 24.74        O
HETATM15615  O   HOH G 260   40.483  20.005 -61.440  1.00 30.39        O
HETATM15616  O   HOH G 261   21.530  26.553 -63.510  1.00 28.54        O
HETATM15617  O   HOH G 262    8.068  19.518 -28.196  1.00 28.92        O
HETATM15618  O   HOH G 263   26.986  26.159 -48.600  1.00 35.21        O
HETATM15619  O   HOH G 264   25.242   8.524 -44.900  1.00 29.73        O
HETATM15620  O   HOH G 265   22.988  25.239 -46.251  1.00 28.52        O
HETATM15621  O   HOH G 266   39.503  20.797 -63.686  1.00 30.30        O
HETATM15622  O   HOH G 267   18.971  24.340 -47.072  1.00 28.98        O
HETATM15623  O   HOH G 268   -8.146  25.826 -32.302  1.00 33.48        O
HETATM15624  O   HOH G 269   31.585  28.448 -51.502  1.00 34.10        O
HETATM15625  O   HOH G 270    1.593  22.438 -46.087  1.00 32.94        O
HETATM15626  O   HOH G 271   18.448   9.708 -67.815  1.00 30.05        O
HETATM15627  O   HOH G 272   -3.978  12.160 -38.221  1.00 29.65        O
HETATM15628  O   HOH G 273   31.556  10.851 -67.466  1.00 32.13        O
HETATM15629  O   HOH G 274   39.463  29.750 -49.142  1.00 28.26        O
HETATM15630  O   HOH G 275   -3.843  11.068 -24.114  1.00 28.15        O
HETATM15631  O   HOH G 276   17.234  17.279 -44.107  1.00 42.20        O
HETATM15632  O   HOH G 277    3.076   9.904 -48.758  1.00 32.40        O
HETATM15633  O   HOH G 278   43.239  19.589 -64.614  1.00 34.24        O
HETATM15634  O   HOH G 279   35.446  28.104 -65.371  1.00 27.83        O
HETATM15635  O   HOH G 280    5.059  16.440 -17.208  1.00 26.67        O
HETATM15636  O   HOH G 281   20.470  28.519 -48.832  1.00 40.21        O
HETATM15637  O   HOH G 282    3.578  18.713 -58.858  1.00 28.93        O
HETATM15638  O   HOH G 283   -3.650  18.496 -50.439  1.00 38.25        O
HETATM15639  O   HOH G 284   28.384  31.900 -59.484  1.00 39.78        O
HETATM15640  O   HOH G 285   26.642  28.972 -51.808  1.00 27.17        O
HETATM15641  O   HOH G 286   30.362  24.049 -69.912  1.00 36.07        O
HETATM15642  O   HOH G 287   24.736   6.352 -51.534  1.00 30.23        O
HETATM15643  O   HOH G 288   32.682   8.250 -64.439  1.00 30.84        O
HETATM15644  O   HOH G 289   34.715  30.971 -58.190  1.00 36.34        O
HETATM15645  O   HOH G 290   17.647  24.510 -56.716  1.00 30.82        O
HETATM15646  O   HOH G 291   28.549   3.847 -55.688  1.00 39.88        O
HETATM15647  O   HOH G 292   10.492  15.969 -11.119  1.00 32.89        O
HETATM15648  O   HOH G 293   11.349  16.703 -37.597  1.00 33.93        O
HETATM15649  O   HOH G 294   37.110  21.797 -64.500  1.00 34.55        O
HETATM15650  O   HOH G 295   23.183  22.765 -44.364  1.00 40.01        O
HETATM15651  O   HOH G 296   19.817  15.832 -40.066  1.00 39.89        O
HETATM15652  O   HOH G 297    8.455   6.113 -49.935  1.00 28.25        O
HETATM15653  O   HOH G 298    5.500  28.794 -30.200  1.00 32.69        O
```

| HETATM15654 | O | HOH G 299 | 9.902 | 15.597 | -9.019 | 1.00 30.97 | O |
|---|---|---|---|---|---|---|---|
| HETATM15655 | O | HOH G 300 | 17.348 | 23.193 | -64.453 | 1.00 29.33 | O |
| HETATM15656 | O | HOH G 301 | 18.740 | 26.346 | -57.857 | 1.00 31.11 | O |
| HETATM15657 | O | HOH G 302 | -7.881 | 12.940 | -32.765 | 1.00 25.79 | O |
| HETATM15658 | O | HOH G 303 | 10.479 | 14.291 | -40.639 | 1.00 40.70 | O |
| HETATM15659 | O | HOH G 304 | -18.974 | 22.531 | -37.840 | 1.00 32.64 | O |
| HETATM15660 | O | HOH G 305 | 5.477 | 15.021 | -63.763 | 1.00 39.65 | O |
| HETATM15661 | O | HOH G 306 | 5.317 | 7.402 | -51.619 | 1.00 33.92 | O |
| HETATM15662 | O | HOH G 307 | -1.934 | 11.357 | -50.497 | 1.00 39.22 | O |
| HETATM15663 | O | HOH G 308 | 10.299 | 20.753 | -59.002 | 1.00 40.27 | O |
| HETATM15664 | O | HOH G 309 | 24.835 | 29.686 | -64.161 | 1.00 56.26 | O |
| HETATM15665 | O | HOH G 310 | 16.483 | 30.160 | -52.360 | 1.00 41.03 | O |
| HETATM15666 | O | HOH G 311 | -11.258 | 25.163 | -40.419 | 1.00 35.44 | O |
| HETATM15667 | O | HOH G 312 | 9.049 | 20.942 | -14.165 | 1.00 38.99 | O |
| HETATM15668 | O | HOH G 313 | 14.014 | 10.819 | -42.424 | 1.00 34.54 | O |
| HETATM15669 | O | HOH G 314 | 8.329 | 24.727 | -35.318 | 1.00 33.84 | O |
| HETATM15670 | O | HOH G 315 | 10.171 | 22.461 | -37.906 | 1.00 29.96 | O |
| HETATM15671 | O | HOH G 316 | 6.954 | 5.658 | -47.654 | 1.00 39.31 | O |
| HETATM15672 | O | HOH G 317 | 43.927 | 24.267 | -53.680 | 1.00 30.08 | O |
| HETATM15673 | O | HOH G 318 | 20.841 | 27.185 | -59.196 | 1.00 29.90 | O |
| HETATM15674 | O | HOH G 319 | 16.582 | 31.712 | -54.928 | 1.00 34.57 | O |
| HETATM15675 | O | HOH G 320 | 21.597 | 4.148 | -51.836 | 1.00 39.53 | O |
| HETATM15676 | O | HOH G 321 | 13.395 | 22.905 | -57.493 | 1.00 39.47 | O |
| HETATM15677 | O | HOH G.322 | 8.011 | 6.291 | -57.663 | 1.00 42.41 | O |
| HETATM15678 | O | HOH G 323 | 29.306 | 26.157 | -69.135 | 1.00 46.64 | O |
| HETATM15679 | O | HOH G 324 | -10.106 | 13.457 | -17.279 | 1.00 37.77 | O |
| HETATM15680 | O | HOH G 325 | -8.732 | 13.230 | -36.561 | 1.00 41.74 | O |
| HETATM15681 | O | HOH G 326 | 13.223 | 7.231 | -46.041 | 1.00 52.35 | O |
| HETATM15682 | O | HOH G 327 | 8.246 | 12.817 | -38.804 | 1.00 40.22 | O |
| HETATM15683 | O | HOH G 328 | 8.419 | 21.713 | -40.880 | 1.00 49.49 | O |
| HETATM15684 | O | HOH G 329 | 9.881 | 18.460 | -62.927 | 1.00 42.75 | O |
| HETATM15685 | O | HOH G 330 | 14.404 | 30.374 | -56.235 | 1.00 36.15 | O |
| HETATM15686 | O | HOH G 331 | 39.641 | 21.380 | -69.920 | 1.00 58.00 | O |
| HETATM15687 | O | HOH G 332 | -10.646 | 14.097 | -24.227 | 1.00 40.80 | O |
| HETATM15688 | O | HOH G 333 | -4.842 | 9.688 | -29.960 | 1.00 59.71 | O |
| HETATM15689 | O | HOH G 334 | 9.349 | 10.304 | -66.511 | 1.00 62.63 | O |
| HETATM15690 | O | HOH G 335 | 9.939 | 19.169 | -44.441 | 1.00 32.05 | O |
| HETATM15691 | O | HOH G 336 | 4.030 | 20.462 | -48.103 | 1.00 45.81 | O |
| HETATM15692 | O | HOH G 337 | 9.989 | 23.841 | -34.096 | 1.00 33.23 | O |
| HETATM15693 | O | HOH G 338 | -0.949 | 9.592 | -35.258 | 1.00 39.99 | O |
| HETATM15694 | O | HOH G 339 | -11.962 | 13.605 | -19.149 | 1.00 46.06 | O |
| HETATM15695 | O | HOH G 340 | -8.577 | 13.447 | -42.882 | 1.00 49.30 | O |
| HETATM15696 | O | HOH G 341 | 18.664 | 7.415 | -63.999 | 1.00 30.50 | O |
| HETATM15697 | O | HOH G 342 | 4.586 | 8.352 | -55.397 | 1.00 37.51 | O |
| HETATM15698 | O | HOH G 343 | -4.934 | 23.939 | -42.134 | 1.00 38.88 | O |
| HETATM15699 | O | HOH G 344 | 15.706 | 1.460 | -59.755 | 1.00 43.12 | O |
| HETATM15700 | O | HOH G 345 | 9.473 | 18.640 | -47.124 | 1.00 36.03 | O |
| HETATM15701 | O | HOH G 346 | 11.266 | 8.477 | -66.356 | 1.00 45.63 | O |
| HETATM15702 | O | HOH G 347 | 10.645 | 11.365 | -41.357 | 1.00 37.19 | O |
| HETATM15703 | O | HOH G 348 | 20.758 | 27.625 | -61.477 | 1.00 30.06 | O |
| HETATM15704 | O | HOH G 349 | -6.910 | 10.893 | -27.654 | 1.00 41.08 | O |
| HETATM15705 | O | HOH G 350 | 26.083 | 28.297 | -49.294 | 1.00 34.85 | O |
| HETATM15706 | O | HOH G 351 | 25.438 | 3.983 | -52.401 | 1.00 33.30 | O |
| HETATM15707 | O | HOH G 352 | -19.283 | 24.933 | -41.465 | 1.00 44.20 | O |
| HETATM15708 | O | HOH G 353 | 29.681 | 29.963 | -63.968 | 1.00 32.40 | O |
| HETATM15709 | O | HOH G 354 | -16.691 | 21.489 | -41.366 | 1.00 40.40 | O |
| HETATM15710 | O | HOH G 355 | 18.401 | 17.603 | -42.225 | 1.00 37.95 | O |
| HETATM15711 | O | HOH G 356 | 14.804 | 8.581 | -64.488 | 1.00 46.20 | O |
| HETATM15712 | O | HOH G 357 | 11.112 | 17.256 | -44.717 | 1.00 32.94 | O |
| HETATM15713 | O | HOH G 358 | -5.274 | 12.716 | -21.173 | 1.00 37.31 | O |
| HETATM15714 | O | HOH G 359 | 21.772 | 2.880 | -58.783 | 1.00 30.94 | O |
| HETATM15715 | O | HOH G 360 | -5.815 | 12.380 | -46.579 | 1.00 32.05 | O |
| HETATM15716 | O | HOH G 361 | -7.865 | 12.629 | -12.363 | 1.00 56.97 | O |
| HETATM15717 | O | HOH G 362 | -15.437 | 17.431 | -41.428 | 1.00 39.35 | O |
| HETATM15718 | O | HOH G 363 | 20.756 | 30.545 | -53.343 | 1.00 42.85 | O |

```
HETATM15719  O   HOH G 364     -9.314  13.875 -10.396  1.00 50.11           O
HETATM15720  O   HOH G 365     -8.464  12.642 -24.061  1.00 38.04           O
HETATM15721  O   HOH G 366      8.312  21.002 -46.050  1.00 39.69           O
HETATM15722  O   HOH G 367     22.307   7.683 -44.543  1.00 43.87           O
HETATM15723  O   HOH G 368    -11.112  24.057 -20.434  1.00 49.53           O
HETATM15724  O   HOH G 369     41.583  11.725 -64.872  1.00 46.28           O
HETATM15725  O   HOH G 370    -15.691  20.335 -32.193  1.00 44.86           O
HETATM15726  O   HOH G 371     22.625  -0.206 -57.467  1.00 46.11           O
HETATM15727  O   HOH G 372     37.455  25.359 -64.131  1.00 42.42           O
HETATM15728  O   HOH G 373     22.816   9.878 -68.668  1.00 36.85           O
HETATM15729  O   HOH G 374      7.792  24.430 -23.908  1.00 34.78           O
HETATM15730  O   HOH G 375     19.991  19.804 -43.371  1.00 48.61           O
HETATM15731  O   HOH G 376     -6.874  16.603 -50.834  1.00 41.55           O
HETATM15732  O   HOH G 377      8.168  22.364 -25.206  1.00 38.54           O
HETATM15733  O   HOH G 378      0.232  10.007 -48.981  1.00 45.19           O
HETATM15734  O   HOH G 379     32.145  30.013 -65.392  1.00 45.83           O
HETATM15735  O   HOH G 380    -10.733  21.192 -38.049  1.00 43.67           O
HETATM15736  O   HOH G 381     12.936  13.233 -68.235  1.00 47.79           O
HETATM15737  O   HOH G 382     16.483  24.436 -47.616  1.00 44.09           O
HETATM15738  O   HOH G 383     17.971  28.174 -61.844  1.00 41.36           O
HETATM15739  O   HOH G 384      3.864   8.522 -38.539  1.00 40.18           O
HETATM15740  O   HOH G 385      7.103  27.126 -33.851  1.00 45.49           O
HETATM15741  O   HOH G 386     -1.325   8.103 -44.957  1.00 47.65           O
HETATM15742  O   HOH G 387    -10.642  23.443 -46.326  1.00 42.80           O
HETATM15743  O   HOH G 388      2.189   9.100 -35.284  1.00 33.04           O
HETATM15744  O   HOH G 389     37.544   9.656 -62.214  1.00 47.32           O
HETATM15745  O   HOH G 390     -4.550  25.763 -44.208  1.00 44.81           O
HETATM15746  O   HOH G 391     14.922  17.373 -43.859  1.00 45.11           O
HETATM15747  O   HOH G 392      2.663  31.938 -24.968  1.00 44.06           O
HETATM15748  O   HOH G 393      3.303   7.468 -49.877  1.00 44.63           O
HETATM15749  O   HOH G 394      5.234  32.523 -21.163  1.00 39.50           O
HETATM15750  O   HOH G 395    -12.375  13.378 -22.150  1.00 50.77           O
HETATM15751  O   HOH G 396     18.249   7.355 -52.334  1.00 41.56           O
HETATM15752  O   HOH G 397     39.029  28.595 -55.878  1.00 53.70           O
HETATM15753  O   HOH G 398    -14.109  17.453 -34.161  1.00 41.84           O
HETATM15754  O   HOH G 399     -1.299  14.018 -54.012  1.00 49.26           O
HETATM15755  O   HOH G 400     -6.092  11.746 -40.421  1.00 49.88           O
HETATM15756  O   HOH G 401     -7.091  12.604 -16.053  1.00 52.99           O
HETATM15757  O   HOH G 402    -12.214  28.216 -12.770  1.00 41.48           O
HETATM15758  O   HOH G 403     -4.778  29.148 -26.220  1.00 46.40           O
HETATM15759  O   HOH G 404     31.062   4.928 -63.150  1.00 42.83           O
HETATM15760  O   HOH G 405     26.851   3.984 -61.368  1.00 35.58           O
HETATM15761  O   HOH G 406     40.359  24.047 -60.175  1.00 53.59           O
HETATM15762  O   HOH G 407     39.495  25.583 -62.300  1.00 47.07           O
HETATM15763  O   HOH G 408      9.302  21.179 -43.320  1.00 45.77           O
HETATM15764  O   HOH G 409    -12.470  18.536 -18.556  1.00 42.49           O
HETATM15765  O   HOH G 410     10.814   6.803 -45.654  1.00 50.46           O
HETATM15766  O   HOH G 411     -9.991  19.854 -46.645  1.00 49.48           O
HETATM15767  O   HOH G 412    -10.356  28.033  -8.334  1.00 48.93           O
HETATM15768  O   HOH G 413    -12.539  16.066 -19.437  1.00 45.36           O
HETATM15769  O   HOH G 414     -2.763  16.124 -53.927  1.00 56.87           O
HETATM15770  O   HOH G 415     15.739  24.267 -58.073  1.00 40.59           O
HETATM15771  O   HOH G 416     -1.349  23.514  -5.060  1.00 48.16           O
HETATM15772  O   HOH G 417     -8.332  12.718 -21.609  1.00 41.59           O
HETATM15773  O   HOH G 418      3.786  14.569  -7.496  1.00 49.48           O
HETATM15774  O   HOH G 419     16.519   1.010 -55.183  1.00 51.18           O
HETATM15775  O   HOH G 420     -4.267  34.034 -21.027  1.00 48.41           O
HETATM15776  O   HOH G 421     23.862   2.265 -59.378  1.00 40.57           O
HETATM15777  O   HOH G 422    -11.572  16.948 -38.020  1.00 49.89           O
HETATM15778  O   HOH G 423     20.024   8.689 -65.834  1.00 39.27           O
HETATM15779  O   HOH G 424     12.977   5.200 -64.597  1.00 58.10           O
HETATM15780  O   HOH G 425    -18.821  24.503 -43.752  1.00 42.88           O
HETATM15781  O   HOH G 426      1.686  12.905 -18.055  1.00 48.84           O
HETATM15782  O   HOH G 427      7.093   9.437 -64.946  1.00 68.71           O
HETATM15783  O   HOH G 428     -0.596  22.212 -49.757  1.00 57.71           O
```

```
HETATM15784  O   HOH G 429     34.704  23.535 -69.198  1.00 54.39          O
HETATM15785  O   HOH G 430    -11.897  17.497  -9.014  1.00 47.32          O
HETATM15786  O   HOH G 431     26.924   8.991 -67.502  1.00 47.78          O
HETATM15787  O   HOH G 432     38.248  10.242 -69.049  1.00 47.22          O
HETATM15788  O   HOH G 433     19.975  31.503 -50.249  1.00 59.68          O
HETATM15789  O   HOH G 434      5.793   7.631 -40.555  1.00 50.15          O
HETATM15790  O   HOH G 435    -16.621  17.897 -38.831  1.00 43.54          O
HETATM15791  O   HOH G 436     -0.954  28.413 -30.325  1.00 47.21          O
HETATM15792  O   HOH G 437      6.629  10.968  -8.910  1.00 48.21          O
HETATM15793  O   HOH G 438     34.376  30.432 -64.725  1.00 43.40          O
HETATM15794  O   HOH G 439     42.068  26.489 -54.306  1.00 61.49          O
HETATM15795  O   HOH G 440     37.173  20.565 -71.616  1.00 54.73          O
HETATM15796  O   HOH G 441     -3.091  37.647 -23.593  1.00 53.74          O
HETATM15797  O   HOH G 442     -2.933  11.245 -41.389  1.00 40.15          O
HETATM15798  O   HOH G 443     32.004  32.754 -62.005  1.00 45.37          O
HETATM15799  O   HOH G 444     21.111  29.608 -59.709  1.00 53.22          O
HETATM15800  O   HOH G 445     -4.399  21.907 -48.973  1.00 58.81          O
HETATM15801  O   HOH G 446     23.256  30.504 -52.221  1.00 45.83          O
HETATM15802  O   HOH G 447     19.162  27.221 -46.245  1.00 54.55          O
HETATM15803  O   HOH G 448     19.248  30.593 -55.747  1.00 53.01          O
HETATM15804  O   HOH G 449      6.290  34.425 -23.133  1.00 54.54          O
HETATM15805  O   HOH G 450      4.180  24.026 -45.256  1.00 60.81          O
HETATM15806  O   HOH G 451      5.227  31.104 -24.862  1.00 47.45          O
HETATM15807  O   HOH G 452     -8.894   9.622 -26.091  1.00 54.42          O
HETATM15808  O   HOH G 453     20.490  19.915 -40.813  1.00 53.66          O
HETATM15809  O   HOH G 454     24.784   5.896 -49.024  1.00 51.50          O
HETATM15810  O   HOH G 455     -6.045  20.061  -5.561  1.00 64.59          O
HETATM15811  O   HOH G 456     25.673   0.070 -58.960  1.00 59.74          O
HETATM15812  O   HOH G 457    -13.347  15.102 -26.633  1.00 49.92          O
HETATM15813  O   HOH G 458     24.465   7.283 -66.069  1.00 52.57          O
HETATM15814  O   HOH G 459     11.391  23.165 -60.507  1.00 48.12          O
HETATM15815  O   HOH G 460      1.065  38.628 -19.074  1.00 55.42          O
HETATM15816  O   HOH G 461     28.692   2.067 -59.180  1.00 57.54          O
HETATM15817  O   HOH G 462     39.531   9.789 -65.690  1.00 59.30          O
HETATM15818  O   HOH G 463     -9.118  33.515 -20.020  1.00 45.30          O
HETATM15819  O   HOH G 464      5.229  18.540  -3.906  1.00 44.00          O
HETATM15820  O   HOH G 465     19.064   5.091 -52.203  1.00 58.30          O
HETATM15821  O   HOH G 466     15.830   9.412 -67.159  1.00 46.91          O
HETATM15822  O   HOH G 467     15.091   8.292 -51.865  1.00 40.52          O
HETATM15823  O   HOH G 468    -18.140  27.454 -40.280  1.00 41.40          O
HETATM15824  O   HOH G 469     22.372   8.963 -41.288  1.00 34.57          O
HETATM15825  O   HOH H 311     49.141  23.776 -33.513  1.00 47.35          O
HETATM15826  O   HOH H 312     24.432  17.572 -25.857  1.00 13.23          O
HETATM15827  O   HOH H 313     29.512  15.031 -19.804  1.00 13.90          O
HETATM15828  O   HOH H 314     34.523  19.630 -23.608  1.00 10.97          O
HETATM15829  O   HOH H 315     26.589  20.329 -28.696  1.00 14.09          O
HETATM15830  O   HOH H 316     28.055  31.876 -25.241  1.00 16.09          O
HETATM15831  O   HOH H 317     34.967   9.230 -54.213  1.00 15.04          O
HETATM15832  O   HOH H 318     28.163  16.201 -28.481  1.00 16.51          O
HETATM15833  O   HOH H 319     30.788  24.675 -48.665  1.00 14.13          O
HETATM15834  O   HOH H 320     22.606  24.361 -28.260  1.00 16.26          O
HETATM15835  O   HOH H 321     42.775  26.392 -27.240  1.00 11.57          O
HETATM15836  O   HOH H 322     37.762   8.827 -49.202  1.00 16.97          O
HETATM15837  O   HOH H 323     34.507   7.051 -32.482  1.00 22.54          O
HETATM15838  O   HOH H 324     33.470  33.457 -29.993  1.00 15.15          O
HETATM15839  O   HOH H 325     33.227  34.897 -32.173  1.00 16.66          O
HETATM15840  O   HOH H 326     19.927  12.612 -20.428  1.00 17.85          O
HETATM15841  O   HOH H 327     27.144  10.654 -16.462  1.00 18.50          O
HETATM15842  O   HOH H 328     26.833  18.166 -26.995  1.00 16.55          O
HETATM15843  O   HOH H 329     39.646  27.631 -47.726  1.00 22.70          O
HETATM15844  O   HOH H 330     30.545  35.098 -31.878  1.00 23.88          O
HETATM15845  O   HOH H 331     27.311   7.730 -46.321  1.00 24.45          O
HETATM15846  O   HOH H 332     29.048  25.156 -40.863  1.00 28.44          O
HETATM15847  O   HOH H 333     46.764  12.825 -52.356  1.00 22.43          O
HETATM15848  O   HOH H 334     44.755  15.653 -34.666  1.00 21.14          O
```

```
HETATM15849  O   HOH H 335   43.228  21.996 -55.105  1.00 20.20          O
HETATM15850  O   HOH H 336   16.865  22.097 -15.938  1.00 18.90          O
HETATM15851  O   HOH H 337   24.931  10.421 -23.285  1.00 23.66          O
HETATM15852  O   HOH H 338   36.075  28.904 -38.003  1.00 20.00          O
HETATM15853  O   HOH H 339   48.633  11.796 -39.039  1.00 24.66          O
HETATM15854  O   HOH H 340   27.034  14.660 -30.269  1.00 15.82          O
HETATM15855  O   HOH H 341   47.251   5.783 -45.230  1.00 26.23          O
HETATM15856  O   HOH H 342   26.898  14.867 -16.218  1.00 18.72          O
HETATM15857  O   HOH H 343   29.147  12.318 -24.314  1.00 18.70          O
HETATM15858  O   HOH H 344   27.221   8.449 -48.875  1.00 22.85          O
HETATM15859  O   HOH H 345   35.404  25.325 -46.430  1.00 17.90          O
HETATM15860  O   HOH H 346   16.898  19.534 -16.190  1.00 21.20          O
HETATM15861  O   HOH H 347   14.501  17.126 -32.062  1.00 25.08          O
HETATM15862  O   HOH H 348   13.303   4.781 -31.481  1.00 27.77          O
HETATM15863  O   HOH H 349   49.812   8.273 -53.207  1.00 28.60          O
HETATM15864  O   HOH H 350    9.804  21.591 -28.570  1.00 29.03          O
HETATM15865  O   HOH H 351   46.564   9.785 -52.509  1.00 32.05          O
HETATM15866  O   HOH H 352   44.343  12.830 -58.459  1.00 25.51          O
HETATM15867  O   HOH H 353   -4.796  -2.660 -31.255  1.00 32.33          O
HETATM15868  O   HOH H 354   27.888  10.532 -26.025  1.00 22.74          O
HETATM15869  O   HOH H 355   30.756  27.117 -20.309  1.00 25.82          O
HETATM15870  O   HOH H 356   19.519  13.812 -17.878  1.00 21.11          O
HETATM15871  O   HOH H 357   30.749  25.881 -37.503  1.00 28.01          O
HETATM15872  O   HOH H 358    1.327  -7.652 -28.603  1.00 34.14          O
HETATM15873  O   HOH H 359   18.511   7.759 -27.107  1.00 26.91          O
HETATM15874  O   HOH H 360   22.700  26.076 -18.819  1.00 28.66          O
HETATM15875  O   HOH H 361   16.289  12.466 -31.203  1.00 30.14          O
HETATM15876  O   HOH H 362    3.488   9.502 -32.812  1.00 29.52          O
HETATM15877  O   HOH H 363   42.129  22.057 -59.820  1.00 24.53          O
HETATM15878  O   HOH H 364   25.015  24.391 -29.623  1.00 24.11          O
HETATM15879  O   HOH H 365   15.910  19.980 -31.649  1.00 30.70          O
HETATM15880  O   HOH H 366   28.458   5.367 -41.734  1.00 35.66          O
HETATM15881  O   HOH H 367   27.455  29.768 -33.797  1.00 27.93          O
HETATM15882  O   HOH H 368   45.741  23.651 -46.625  1.00 24.25          O
HETATM15883  O   HOH H 369   48.579   6.359 -39.388  1.00 29.81          O
HETATM15884  O   HOH H 370   12.650  25.399 -22.908  1.00 29.78          O
HETATM15885  O   HOH H 371   21.791  28.156 -20.880  1.00 33.05          O
HETATM15886  O   HOH H 372   28.028  27.808 -35.753  1.00 21.47          O
HETATM15887  O   HOH H 373   18.157  17.481 -30.239  1.00 24.57          O
HETATM15888  O   HOH H 374   48.739  19.343 -40.275  1.00 28.23          O
HETATM15889  O   HOH H 375   25.101  10.333 -25.808  1.00 27.81          O
HETATM15890  O   HOH H 376   46.296  23.906 -39.265  1.00 31.58          O
HETATM15891  O   HOH H 377   45.869  16.047 -30.114  1.00 29.19          O
HETATM15892  O   HOH H 378   32.751   2.445 -37.247  1.00 35.69          O
HETATM15893  O   HOH H 379   49.748   4.626 -48.681  1.00 37.06          O
HETATM15894  O   HOH H 380   42.065  19.143 -26.302  1.00 29.20          O
HETATM15895  O   HOH H 381   13.489  28.165 -29.847  1.00 31.67          O
HETATM15896  O   HOH H 382   48.725  14.976 -51.217  1.00 32.48          O
HETATM15897  O   HOH H 383   47.494  23.622 -55.286  1.00 27.10          O
HETATM15898  O   HOH H 384   22.023  10.334 -15.871  1.00 30.42          O
HETATM15899  O   HOH H 385   17.035  14.134 -15.079  1.00 27.66          O
HETATM15900  O   HOH H 386    5.252   0.512 -20.521  1.00 38.57          O
HETATM15901  O   HOH H 387   34.770   7.739 -50.308  1.00 31.60          O
HETATM15902  O   HOH H 388   31.869  30.408 -18.854  1.00 31.92          O
HETATM15903  O   HOH H 389   44.839  15.734 -63.958  1.00 29.11          O
HETATM15904  O   HOH H 390   18.646  12.507 -13.760  1.00 31.43          O
HETATM15905  O   HOH H 391   16.652  32.128 -23.728  1.00 38.65          O
HETATM15906  O   HOH H 392   38.834  -1.255 -48.576  1.00 31.10          O
HETATM15907  O   HOH H 393   14.590   0.728 -23.519  1.00 32.91          O
HETATM15908  O   HOH H 394   41.857  25.289 -48.407  1.00 30.97          O
HETATM15909  O   HOH H 395   36.601  16.416 -27.048  1.00 25.81          O
HETATM15910  O   HOH H 396   24.231  23.842 -32.136  1.00 31.20          O
HETATM15911  O   HOH H 397   21.239  28.386 -27.384  1.00 27.87          O
HETATM15912  O   HOH H 398   14.168  13.366 -14.911  1.00 27.36          O
HETATM15913  O   HOH H 399    6.449  28.308 -21.804  1.00 33.08          O
```

```
HETATM15914  O   HOH H 400    26.760    9.005 -21.999  1.00 34.36        O
HETATM15915  O   HOH H 401    40.929   -0.508 -50.243  1.00 44.98        O
HETATM15916  O   HOH H 402    48.659   15.966 -38.874  1.00 33.05        O
HETATM15917  O   HOH H 403    24.655   29.415 -18.168  1.00 22.79        O
HETATM15918  O   HOH H 404    49.421   19.666 -61.072  1.00 33.90        O
HETATM15919  O   HOH H 405    46.526   24.965 -32.975  1.00 39.92        O
HETATM15920  O   HOH H 406    25.934    8.102 -42.263  1.00 30.23        O
HETATM15921  O   HOH H 407    49.981   19.861 -47.463  1.00 26.60        O
HETATM15922  O   HOH H 408    24.626   13.859 -31.139  1.00 35.56        O
HETATM15923  O   HOH H 409    12.996   26.916 -19.804  1.00 36.76        O
HETATM15924  O   HOH H 410    30.888   13.123 -26.195  1.00 27.19        O
HETATM15925  O   HOH H 411    -4.072    7.801 -27.806  1.00 33.10        O
HETATM15926  O   HOH H 412    23.462    8.197 -17.301  1.00 34.00        O
HETATM15927  O   HOH H 413    18.811   17.502 -32.894  1.00 44.46        O
HETATM15928  O   HOH H 414    12.881   24.405 -18.492  1.00 38.07        O
HETATM15929  O   HOH H 415    31.015   -2.060 -40.365  1.00 48.11        O
HETATM15930  O   HOH H 416    25.142   15.312 -33.392  1.00 29.33        O
HETATM15931  O   HOH H 417    22.675   13.259 -29.156  1.00 39.11        O
HETATM15932  O   HOH H 418    50.467   19.141 -50.844  1.00 29.51        O
HETATM15933  O   HOH H 419    49.120    3.376 -37.602  1.00 42.95        O
HETATM15934  O   HOH H 420    33.358    6.239 -56.039  1.00 44.34        O
HETATM15935  O   HOH H 421    24.074   10.891 -28.200  1.00 38.31        O
HETATM15936  O   HOH H 422    31.865   11.001 -27.253  1.00 31.91        O
HETATM15937  O   HOH H 423    28.593   32.203 -34.154  1.00 36.41        O
HETATM15938  O   HOH H 424    44.004    0.924 -41.976  1.00 27.82        O
HETATM15939  O   HOH H 425    17.355    6.044 -16.193  1.00 35.23        O
HETATM15940  O   HOH H 426    28.487   12.352 -30.356  1.00 34.85        O
HETATM15941  O   HOH H 427    28.968    5.773 -44.195  1.00 41.80        O
HETATM15942  O   HOH H 428    43.460   22.160 -62.244  1.00 41.22        O
HETATM15943  O   HOH H 429    53.456   15.336 -48.742  1.00 53.10        O
HETATM15944  O   HOH H 430    30.615   28.850 -38.857  1.00 36.98        O
HETATM15945  O   HOH H 431    41.776   33.456 -26.034  1.00 39.40        O
HETATM15946  O   HOH H 432    22.489   24.429 -14.701  1.00 25.15        O
HETATM15947  O   HOH H 433     9.914   -2.637 -29.371  1.00 39.92        O
HETATM15948  O   HOH H 434    24.891   22.542 -36.170  1.00 31.96        O
HETATM15949  O   HOH H 435    27.371   29.053 -29.010  1.00 29.53        O
HETATM15950  O   HOH H 436    -9.277    3.506 -30.256  1.00 51.67        O
HETATM15951  O   HOH H 437    49.953    6.116 -35.234  1.00 46.19        O
HETATM15952  O   HOH H 438    44.812   13.658 -62.428  1.00 42.21        O
HETATM15953  O   HOH H 439    24.548   26.070 -14.627  1.00 34.59        O
HETATM15954  O   HOH H 440    45.079   15.548 -57.220  1.00 26.65        O
HETATM15955  O   HOH H 441    25.524   29.729 -25.933  1.00 33.12        O
HETATM15956  O   HOH H 442    39.659   10.908 -61.546  1.00 35.05        O
HETATM15957  O   HOH H 443    -2.820    9.196 -25.806  1.00 38.32        O
HETATM15958  O   HOH H 444    23.220   28.029 -16.782  1.00 39.68        O
HETATM15959  O   HOH H 445    -3.340    7.177 -31.175  1.00 34.36        O
HETATM15960  O   HOH H 446    10.316   -0.903 -23.125  1.00 43.84        O
HETATM15961  O   HOH H 447    39.138    0.160 -41.253  1.00 50.65        O
HETATM15962  O   HOH H 448    49.613   21.325 -52.043  1.00 32.25        O
HETATM15963  O   HOH H 449    52.970   15.878 -45.495  1.00 40.51        O
HETATM15964  O   HOH H 450    28.112    5.478 -37.157  1.00 51.79        O
HETATM15965  O   HOH H 451    43.024   30.475 -27.127  1.00 33.00        O
HETATM15966  O   HOH H 452    16.664    8.989 -32.420  1.00 41.43        O
HETATM15967  O   HOH H 453    21.029    7.925 -15.528  1.00 33.60        O
HETATM15968  O   HOH H 454     3.882   -1.883 -19.893  1.00 29.98        O
HETATM15969  O   HOH H 455    40.506    1.453 -33.085  1.00 39.05        O
HETATM15970  O   HOH H 456    49.247   16.473 -57.397  1.00 35.76        O
HETATM15971  O   HOH H 457    10.837   12.763 -35.950  1.00 35.69        O
HETATM15972  O   HOH H 458    16.303   15.446 -31.018  1.00 30.67        O
HETATM15973  O   HOH H 459    12.316    7.047 -35.354  1.00 42.17        O
HETATM15974  O   HOH H 460    -4.304   -0.307 -24.441  1.00 36.36        O
HETATM15975  O   HOH H 461    49.978   12.392 -56.125  1.00 41.42        O
HETATM15976  O   HOH H 462     7.917    5.111 -15.274  1.00 38.23        O
HETATM15977  O   HOH H 463    -2.359    5.935 -18.889  1.00 39.32        O
HETATM15978  O   HOH H 464     9.391   -0.267 -34.901  1.00 43.97        O
```

| | | | | | | | | |
|---|---|---|---|---|---|---|---|---|
| HETATM15979 | O | HOH H 465 | 11.041 | 4.435 | -15.379 | 1.00 | 42.17 | O |
| HETATM15980 | O | HOH H 466 | 37.115 | 6.204 | -56.611 | 1.00 | 39.38 | O |
| HETATM15981 | O | HOH H 467 | 18.576 | 3.865 | -27.598 | 1.00 | 41.06 | O |
| HETATM15982 | O | HOH H 468 | 4.825 | 2.250 | -33.536 | 1.00 | 39.37 | O |
| HETATM15983 | O | HOH H 469 | 36.834 | -3.078 | -40.501 | 1.00 | 48.30 | O |
| HETATM15984 | O | HOH H 470 | 48.073 | 10.070 | -59.330 | 1.00 | 36.35 | O |
| HETATM15985 | O | HOH H 471 | 47.028 | 16.526 | -32.920 | 1.00 | 36.77 | O |
| HETATM15986 | O | HOH H 472 | 20.597 | 6.976 | -28.430 | 1.00 | 37.27 | O |
| HETATM15987 | O | HOH H 473 | 48.064 | 22.226 | -46.868 | 1.00 | 37.23 | O |
| HETATM15988 | O | HOH H 474 | 46.933 | 12.517 | -59.489 | 1.00 | 40.86 | O |
| HETATM15989 | O | HOH H 475 | 36.305 | 35.018 | -33.596 | 1.00 | 32.98 | O |
| HETATM15990 | O | HOH H 476 | 21.544 | 4.572 | -18.624 | 1.00 | 44.59 | O |
| HETATM15991 | O | HOH H 477 | 44.248 | 26.999 | -45.553 | 1.00 | 33.36 | O |
| HETATM15992 | O | HOH H 478 | 42.884 | 32.942 | -44.208 | 1.00 | 31.02 | O |
| HETATM15993 | O | HOH H 479 | 50.797 | 14.746 | -41.819 | 1.00 | 41.58 | O |
| HETATM15994 | O | HOH H 480 | 31.114 | 27.243 | -48.394 | 1.00 | 48.49 | O |
| HETATM15995 | O | HOH H 481 | 27.967 | 5.910 | -50.831 | 1.00 | 43.08 | O |
| HETATM15996 | O | HOH H 482 | 23.319 | 14.534 | -38.200 | 1.00 | 40.08 | O |
| HETATM15997 | O | HOH H 483 | 23.514 | 12.106 | -38.487 | 1.00 | 32.05 | O |
| HETATM15998 | O | HOH H 484 | 18.865 | 27.620 | -30.660 | 1.00 | 47.79 | O |
| HETATM15999 | O | HOH H 485 | 42.352 | -0.892 | -37.614 | 1.00 | 42.64 | O |
| HETATM16000 | O | HOH H 486 | 32.888 | 5.322 | -31.444 | 1.00 | 46.19 | O |
| HETATM16001 | O | HOH H 487 | 43.965 | 23.832 | -49.240 | 1.00 | 41.72 | O |
| HETATM16002 | O | HOH H 488 | 51.348 | 8.551 | -57.639 | 1.00 | 44.14 | O |
| HETATM16003 | O | HOH H 489 | 35.776 | 5.650 | -52.577 | 1.00 | 40.42 | O |
| HETATM16004 | O | HOH H 490 | 51.190 | 23.200 | -53.968 | 1.00 | 46.41 | O |
| HETATM16005 | O | HOH H 491 | 50.861 | 24.887 | -58.001 | 1.00 | 46.86 | O |
| HETATM16006 | O | HOH H 492 | 50.497 | 23.424 | -63.709 | 1.00 | 55.72 | O |
| HETATM16007 | O | HOH H 493 | 45.465 | 22.457 | -33.836 | 1.00 | 39.42 | O |
| HETATM16008 | O | HOH H 494 | 46.200 | 32.341 | -46.125 | 1.00 | 43.75 | O |
| HETATM16009 | O | HOH H 495 | 25.036 | 24.354 | -34.637 | 1.00 | 39.04 | O |
| HETATM16010 | O | HOH H 496 | 47.336 | 21.246 | -30.000 | 1.00 | 39.34 | O |
| HETATM16011 | O | HOH H 497 | 24.657 | 14.098 | -35.510 | 1.00 | 33.51 | O |
| HETATM16012 | O | HOH H 498 | 27.951 | 10.841 | -32.682 | 1.00 | 33.72 | O |
| HETATM16013 | O | HOH H 499 | 39.051 | 7.744 | -58.918 | 1.00 | 40.06 | O |
| HETATM16014 | O | HOH H 500 | 51.074 | 18.555 | -45.544 | 1.00 | 39.18 | O |
| HETATM16015 | O | HOH H 501 | 50.323 | 17.475 | -59.707 | 1.00 | 36.47 | O |
| HETATM16016 | O | HOH H 502 | 38.323 | 31.912 | -35.543 | 1.00 | 45.28 | O |
| HETATM16017 | O | HOH H 503 | 34.776 | 30.771 | -39.228 | 1.00 | 40.20 | O |
| HETATM16018 | O | HOH H 504 | 20.611 | 29.509 | -24.459 | 1.00 | 37.18 | O |
| HETATM16019 | O | HOH H 505 | 22.365 | 16.429 | -33.765 | 1.00 | 34.69 | O |
| HETATM16020 | O | HOH H 506 | 24.964 | 10.497 | -15.018 | 1.00 | 35.74 | O |
| HETATM16021 | O | HOH H 507 | -0.830 | 10.148 | -32.696 | 1.00 | 33.94 | O |
| HETATM16022 | O | HOH H 508 | 22.122 | 23.793 | -42.389 | 1.00 | 38.02 | O |
| HETATM16023 | O | HOH H 509 | 22.002 | 19.265 | -33.306 | 1.00 | 42.01 | O |
| HETATM16024 | O | HOH H 510 | 39.505 | 0.094 | -34.829 | 1.00 | 44.06 | O |
| HETATM16025 | O | HOH H 511 | 45.064 | 30.009 | -33.720 | 1.00 | 44.81 | O |
| HETATM16026 | O | HOH H 512 | 23.006 | 33.863 | -18.168 | 1.00 | 43.95 | O |
| HETATM16027 | O | HOH H 513 | 20.355 | 35.058 | -19.780 | 1.00 | 43.35 | O |
| HETATM16028 | O | HOH H 514 | 45.677 | 27.723 | -32.085 | 1.00 | 52.74 | O |
| HETATM16029 | O | HOH H 515 | 7.350 | 19.824 | -25.733 | 1.00 | 37.48 | O |
| HETATM16030 | O | HOH H 516 | -4.537 | 5.678 | -10.617 | 1.00 | 61.24 | O |
| HETATM16031 | O | HOH H 517 | 22.796 | 34.783 | -15.709 | 1.00 | 46.46 | O |
| HETATM16032 | O | HOH H 518 | 25.258 | 31.891 | -17.877 | 1.00 | 40.28 | O |
| HETATM16033 | O | HOH H 519 | 25.589 | 31.424 | -15.107 | 1.00 | 43.36 | O |
| HETATM16034 | O | HOH H 520 | 51.196 | 12.090 | -53.523 | 1.00 | 43.56 | O |
| HETATM16035 | O | HOH H 521 | 47.497 | 7.546 | -60.174 | 1.00 | 40.26 | O |
| HETATM16036 | O | HOH H 522 | 22.500 | 29.344 | -35.613 | 1.00 | 46.81 | O |
| HETATM16037 | O | HOH H 523 | 18.968 | 3.857 | -22.539 | 1.00 | 54.66 | O |
| HETATM16038 | O | HOH H 524 | 3.910 | -1.298 | -27.601 | 1.00 | 40.96 | O |
| HETATM16039 | O | HOH H 525 | 25.212 | 27.166 | -29.461 | 1.00 | 37.60 | O |
| HETATM16040 | O | HOH H 526 | 46.457 | 12.325 | -33.125 | 1.00 | 48.86 | O |
| HETATM16041 | O | HOH H 527 | 19.598 | 31.835 | -24.507 | 1.00 | 36.82 | O |
| HETATM16042 | O | HOH H 528 | 20.118 | 32.394 | -27.720 | 1.00 | 48.02 | O |
| HETATM16043 | O | HOH H 529 | 19.014 | 2.489 | -24.771 | 1.00 | 54.19 | O |

| | | | | | | | | | |
|---|---|---|---|---|---|---|---|---|---|
| HETATM16044 | O | HOH | H | 530 | 20.372 | 6.157 | -30.962 | 1.00 40.00 | O |
| HETATM16045 | O | HOH | H | 531 | 29.389 | 4.463 | -52.351 | 1.00 54.82 | O |
| HETATM16046 | O | HOH | H | 532 | 29.407 | 31.622 | -38.676 | 1.00 48.43 | O |
| HETATM16047 | O | HOH | H | 533 | 28.934 | 26.574 | -46.730 | 1.00 45.25 | O |
| HETATM16048 | O | HOH | H | 534 | 29.589 | 13.815 | -28.108 | 1.00 48.98 | O |
| HETATM16049 | O | HOH | H | 535 | 27.232 | 21.844 | -15.741 | 1.00 47.98 | O |
| HETATM16050 | O | HOH | H | 536 | 34.146 | 29.626 | -41.903 | 1.00 43.45 | O |
| HETATM16051 | O | HOH | H | 537 | 13.241 | 24.412 | -20.867 | 1.00 35.89 | O |
| HETATM16052 | O | HOH | H | 538 | 52.135 | 15.979 | -56.587 | 1.00 38.16 | O |
| HETATM16053 | O | HOH | H | 539 | 43.094 | 25.281 | -57.452 | 1.00 58.85 | O |
| HETATM16054 | O | HOH | H | 540 | 45.781 | 16.288 | -66.461 | 1.00 51.80 | O |
| HETATM16055 | O | HOH | H | 541 | 32.474 | 8.775 | -56.079 | 1.00 38.41 | O |
| HETATM16056 | O | HOH | H | 542 | 33.929 | 7.396 | -58.292 | 1.00 52.58 | O |
| HETATM16057 | O | HOH | H | 543 | 28.673 | 9.040 | -34.120 | 1.00 44.58 | O |
| HETATM16058 | O | HOH | H | 544 | 25.623 | 27.106 | -35.135 | 1.00 47.88 | O |
| HETATM16059 | O | HOH | H | 545 | 49.656 | 13.994 | -39.853 | 1.00 44.57 | O |
| HETATM16060 | O | HOH | H | 546 | 10.404 | 31.051 | -25.677 | 1.00 58.58 | O |
| HETATM16061 | O | HOH | H | 547 | 13.635 | 10.242 | -31.938 | 1.00 43.63 | O |
| HETATM16062 | O | HOH | H | 548 | 4.219 | 6.767 | -17.234 | 1.00 45.98 | O |
| HETATM16063 | O | HOH | H | 549 | 15.119 | 15.772 | -13.912 | 1.00 40.11 | O |
| HETATM16064 | O | HOH | H | 550 | 9.098 | 6.645 | -37.132 | 1.00 40.06 | O |
| HETATM16065 | O | HOH | H | 551 | -6.997 | 4.374 | -32.406 | 1.00 50.13 | O |
| HETATM16066 | O | HOH | H | 552 | 31.193 | 2.394 | -35.365 | 1.00 49.74 | O |
| HETATM16067 | O | HOH | H | 553 | 4.883 | -2.247 | -30.641 | 1.00 50.57 | O |
| HETATM16068 | O | HOH | H | 554 | 49.262 | 2.737 | -43.427 | 1.00 51.87 | O |
| HETATM16069 | O | HOH | H | 555 | 37.406 | -0.550 | -37.519 | 1.00 41.45 | O |
| HETATM16070 | O | HOH | H | 556 | 2.655 | 9.668 | -16.806 | 1.00 45.54 | O |
| HETATM16071 | O | HOH | H | 557 | 30.976 | 27.949 | -45.219 | 1.00 48.78 | O |
| HETATM16072 | O | HOH | H | 558 | 41.811 | -2.030 | -34.326 | 1.00 56.13 | O |
| HETATM16073 | O | HOH | H | 559 | 24.028 | 21.328 | -32.021 | 1.00 55.52 | O |
| HETATM16074 | O | HOH | H | 560 | 46.435 | 6.939 | -32.022 | 1.00 46.70 | O |
| HETATM16075 | O | HOH | H | 561 | 16.370 | 29.088 | -16.865 | 1.00 45.93 | O |
| HETATM16076 | O | HOH | H | 562 | 20.391 | 34.975 | -23.669 | 1.00 48.47 | O |
| HETATM16077 | O | HOH | H | 563 | 36.897 | -0.724 | -49.873 | 1.00 43.43 | O |
| HETATM16078 | O | HOH | H | 564 | 49.290 | 22.233 | -49.170 | 1.00 48.36 | O |
| HETATM16079 | O | HOH | H | 565 | 38.945 | -3.760 | -42.665 | 1.00 49.37 | O |
| HETATM16080 | O | HOH | H | 566 | 44.150 | 17.313 | -26.901 | 1.00 37.70 | O |
| HETATM16081 | O | HOH | H | 567 | 36.018 | -5.012 | -42.139 | 1.00 62.07 | O |
| HETATM16082 | O | HOH | H | 568 | 7.129 | -1.520 | -34.347 | 1.00 49.10 | O |
| HETATM16083 | O | HOH | H | 569 | 51.834 | 14.770 | -50.736 | 1.00 47.86 | O |
| HETATM16084 | O | HOH | H | 570 | -10.212 | 3.641 | -27.424 | 1.00 55.24 | O |
| HETATM16085 | O | HOH | H | 571 | -7.610 | 7.151 | -17.150 | 1.00 59.14 | O |
| HETATM16086 | O | HOH | H | 572 | 38.050 | 14.188 | -26.787 | 1.00 39.60 | O |
| HETATM16087 | O | HOH | H | 573 | 51.426 | 10.635 | -38.051 | 1.00 52.34 | O |
| HETATM16088 | O | HOH | H | 574 | 30.265 | 0.591 | -49.605 | 1.00 45.70 | O |
| HETATM16089 | O | HOH | H | 575 | 43.692 | 32.474 | -29.101 | 1.00 55.60 | O |
| HETATM16090 | O | HOH | H | 576 | 50.511 | 17.368 | -43.539 | 1.00 53.47 | O |
| HETATM16091 | O | HOH | H | 577 | 42.374 | 30.234 | -37.841 | 1.00 53.93 | O |
| HETATM16092 | O | HOH | H | 578 | -9.415 | 2.923 | -34.079 | 1.00 63.61 | O |
| HETATM16093 | O | HOH | H | 579 | 46.474 | 11.003 | -31.122 | 1.00 47.81 | O |
| HETATM16094 | O | HOH | H | 580 | 9.814 | -2.124 | -15.907 | 1.00 63.49 | O |
| HETATM16095 | O | HOH | H | 581 | -9.911 | 7.957 | -18.837 | 1.00 66.80 | O |
| HETATM16096 | O | HOH | H | 582 | 48.320 | 3.340 | -32.551 | 1.00 45.40 | O |
| HETATM16097 | O | HOH | H | 583 | 12.758 | -1.248 | -23.264 | 1.00 47.96 | O |
| HETATM16098 | O | HOH | H | 584 | 29.454 | 10.425 | -28.744 | 1.00 56.83 | O |
| HETATM16099 | O | HOH | H | 585 | 44.101 | 13.904 | -66.491 | 1.00 57.05 | O |
| HETATM16100 | O | HOH | H | 586 | 33.698 | -6.966 | -44.620 | 1.00 52.42 | O |
| HETATM16101 | O | HOH | H | 587 | 40.072 | 8.195 | -29.530 | 1.00 51.91 | O |
| HETATM16102 | O | HOH | H | 588 | 36.074 | 13.123 | -25.445 | 1.00 35.16 | O |
| HETATM16103 | O | HOH | H | 589 | 48.747 | 22.018 | -43.962 | 1.00 51.00 | O |
| HETATM16104 | O | HOH | H | 590 | 23.772 | 32.462 | -43.361 | 1.00 53.40 | O |
| HETATM16105 | O | HOH | H | 591 | 18.334 | 34.028 | -27.520 | 1.00 40.26 | O |
| HETATM16106 | O | HOH | H | 592 | 37.948 | 11.807 | -29.319 | 1.00 47.35 | O |
| HETATM16107 | O | HOH | H | 593 | 15.410 | 27.077 | -30.840 | 1.00 60.12 | O |
| HETATM16108 | O | HOH | H | 594 | 22.644 | 30.612 | -20.106 | 1.00 40.72 | O |

```
HETATM16109  O    HOH H 595      54.651    8.376 -45.351  1.00 50.97          O
HETATM16110  O    HOH H 596       6.293    9.041 -37.571  1.00 46.10          O
HETATM16111  O    HOH H 597       9.755   -5.179 -22.359  1.00 49.69          O
HETATM16112  O    HOH H 598      28.859    0.430 -47.533  1.00 46.12          O
HETATM16113  O    HOH H 599      21.060   31.838 -18.319  1.00 46.45          O
HETATM16114  O    HOH H 600      30.414    7.608 -30.712  1.00 55.65          O
HETATM16115  O    HOH H 601       8.946   -3.995 -25.522  1.00 42.44          O
HETATM16116  O    HOH H 602      12.452    2.474 -16.965  1.00 58.29          O
HETATM16117  O    HOH H 603      44.753    3.516 -51.875  1.00 49.62          O
HETATM16118  O    HOH H 604      10.122   25.727 -21.964  1.00 55.47          O
HETATM16119  O    HOH H 605       9.449   25.585 -16.566  1.00 65.72          O
HETATM16120  O    HOH H 606      15.120    4.964 -14.941  1.00 59.79          O
HETATM16121  O    HOH H 607      28.572    8.016 -25.590  1.00 41.24          O
HETATM16122  O    HOH H 608      23.358    9.466 -30.356  1.00 46.55          O
HETATM16123  O    HOH H 609      14.196    0.184 -35.241  1.00 52.08          O
HETATM16124  O    HOH H 610      45.204    6.971 -62.715  1.00 56.00          O
HETATM16125  O    HOH H 611      40.785   11.471 -28.885  1.00 40.16          O
END
```

## TABLE 16

### Crystal 4- 3C6S

Crystal structure of Fab F22-4 in complex with a *Shigella flexneri* 2a O-Ag pentadecasaccharide

```
HEADER    IMMUNE SYSTEM                          05-FEB-08   3C6S
TITLE     CRYSTAL STRUCTURE OF FAB F22-4 IN COMPLEX WITH A SHIGELLA
TITLE    2 FLEXNERI 2A O-AG PENTADECASACCHARIDE
CRYST1   66.860   137.150   109.380   90.00   94.97   90.00 P 1 21 1      8
ATOM      1  N   ASP A   1      1.409  32.550  23.612  1.00 18.67           N
ATOM      2  CA  ASP A   1      2.086  31.216  23.526  1.00 17.96           C
ATOM      3  C   ASP A   1      3.100  30.929  24.615  1.00 15.30           C
ATOM      4  O   ASP A   1      3.770  31.800  25.107  1.00 10.77           O
ATOM      5  CB  ASP A   1      2.708  30.975  22.135  1.00 19.16           C
ATOM      6  CG  ASP A   1      3.997  31.789  21.838  1.00 21.13           C
ATOM      7  OD1 ASP A   1      4.426  32.743  22.556  1.00 18.90           O
ATOM      8  OD2 ASP A   1      4.610  31.446  20.790  1.00 24.51           O
ATOM      9  N   ILE A   2      3.154  29.682  25.042  1.00 12.96           N
ATOM     10  CA  ILE A   2      4.193  29.265  25.962  1.00 15.43           C
ATOM     11  C   ILE A   2      5.544  29.246  25.241  1.00 15.13           C
ATOM     12  O   ILE A   2      5.692  28.617  24.163  1.00 14.76           O
ATOM     13  CB  ILE A   2      3.892  27.875  26.564  1.00 15.06           C
ATOM     14  CG1 ILE A   2      2.693  27.949  27.521  1.00 17.73           C
ATOM     15  CG2 ILE A   2      5.065  27.342  27.377  1.00 16.30           C
ATOM     16  CD1 ILE A   2      2.172  26.615  27.952  1.00 18.04           C
ATOM     17  N   VAL A   3      6.538  29.927  25.832  1.00 12.22           N
ATOM     18  CA  VAL A   3      7.903  29.887  25.333  1.00 12.91           C
ATOM     19  C   VAL A   3      8.744  28.868  26.082  1.00 11.96           C
ATOM     20  O   VAL A   3      8.847  28.918  27.320  1.00 11.30           O
ATOM     21  CB  VAL A   3      8.619  31.212  25.566  1.00 12.13           C
ATOM     22  CG1 VAL A   3      9.985  31.141  25.011  1.00 11.54           C
ATOM     23  CG2 VAL A   3      7.766  32.339  24.993  1.00 12.60           C
ATOM     24  N   MET A   4      9.342  27.961  25.311  1.00 13.77           N
ATOM     25  CA  MET A   4     10.208  26.920  25.815  1.00 13.48           C
ATOM     26  C   MET A   4     11.607  27.349  25.407  1.00 15.40           C
ATOM     27  O   MET A   4     11.923  27.442  24.204  1.00 16.53           O
ATOM     28  CB  MET A   4      9.908  25.564  25.148  1.00 14.72           C
ATOM     29  CG  MET A   4      8.515  25.064  25.288  1.00 14.52           C
ATOM     30  SD  MET A   4      7.987  24.732  26.972  1.00 15.86           S
ATOM     31  CE  MET A   4      8.790  23.142  27.274  1.00 15.76           C
ATOM     32  N   THR A   5     12.463  27.578  26.396  1.00 14.19           N
ATOM     33  CA  THR A   5     13.786  28.133  26.146  1.00 15.87           C
ATOM     34  C   THR A   5     14.874  27.120  26.383  1.00 14.86           C
ATOM     35  O   THR A   5     15.003  26.588  27.476  1.00 15.98           O
ATOM     36  CB  THR A   5     14.048  29.306  27.061  1.00 16.95           C
ATOM     37  OG1 THR A   5     13.147  30.372  26.759  1.00 17.43           O
ATOM     38  CG2 THR A   5     15.471  29.810  26.899  1.00 17.41           C
ATOM     39  N   GLN A   6     15.653  26.850  25.342  1.00 16.05           N
ATOM     40  CA  GLN A   6     16.803  25.996  25.485  1.00 18.61           C
ATOM     41  C   GLN A   6     17.943  26.503  24.600  1.00 18.92           C
ATOM     42  O   GLN A   6     17.743  27.057  23.511  1.00 19.34           O
ATOM     43  CB  GLN A   6     16.407  24.514  25.242  1.00 19.95           C
ATOM     44  CG  GLN A   6     16.059  24.179  23.844  1.00 19.78           C
ATOM     45  CD  GLN A   6     15.526  22.770  23.627  1.00 18.22           C
ATOM     46  OE1 GLN A   6     14.500  22.620  23.009  1.00 14.98           O
ATOM     47  NE2 GLN A   6     16.249  21.733  24.095  1.00 16.03           N
ATOM     48  N   ALA A   7     19.163  26.356  25.086  1.00 19.03           N
ATOM     49  CA  ALA A   7     20.318  26.780  24.316  1.00 19.91           C
ATOM     50  C   ALA A   7     20.489  25.966  23.043  1.00 21.24           C
ATOM     51  O   ALA A   7     20.171  24.789  23.007  1.00 19.30           O
ATOM     52  CB  ALA A   7     21.566  26.726  25.162  1.00 20.04           C
ATOM     53  N   ALA A   8     20.991  26.612  21.998  1.00 21.92           N
```

| ATOM | 54 | CA | ALA | A | 8 | 21.255 | 25.951 | 20.717 | 1.00 | 24.17 | C |
| ATOM | 55 | C | ALA | A | 8 | 22.255 | 24.828 | 20.809 | 1.00 | 24.23 | C |
| ATOM | 56 | O | ALA | A | 8 | 22.171 | 23.896 | 20.041 | 1.00 | 21.82 | O |
| ATOM | 57 | CB | ALA | A | 8 | 21.751 | 26.933 | 19.712 | 1.00 | 23.31 | C |
| ATOM | 58 | N | PHE | A | 9 | 23.237 | 24.963 | 21.694 | 1.00 | 27.58 | N |
| ATOM | 59 | CA | PHE | A | 9 | 24.373 | 24.037 | 21.727 | 1.00 | 31.25 | C |
| ATOM | 60 | C | PHE | A | 9 | 24.773 | 23.597 | 23.127 | 1.00 | 33.70 | C |
| ATOM | 61 | O | PHE | A | 9 | 24.471 | 24.278 | 24.119 | 1.00 | 34.37 | O |
| ATOM | 62 | CB | PHE | A | 9 | 25.577 | 24.680 | 21.042 | 1.00 | 32.47 | C |
| ATOM | 63 | CG | PHE | A | 9 | 25.358 | 24.924 | 19.578 | 1.00 | 33.38 | C |
| ATOM | 64 | CD1 | PHE | A | 9 | 25.097 | 26.194 | 19.102 | 1.00 | 33.14 | C |
| ATOM | 65 | CD2 | PHE | A | 9 | 25.347 | 23.856 | 18.684 | 1.00 | 33.31 | C |
| ATOM | 66 | CE1 | PHE | A | 9 | 24.874 | 26.412 | 17.761 | 1.00 | 33.12 | C |
| ATOM | 67 | CE2 | PHE | A | 9 | 25.119 | 24.064 | 17.348 | 1.00 | 33.38 | C |
| ATOM | 68 | CZ | PHE | A | 9 | 24.885 | 25.345 | 16.879 | 1.00 | 33.31 | C |
| ATOM | 69 | N | SER | A | 10 | 25.445 | 22.443 | 23.175 | 1.00 | 36.01 | N |
| ATOM | 70 | CA | SER | A | 10 | 26.134 | 21.959 | 24.367 | 1.00 | 37.02 | C |
| ATOM | 71 | C | SER | A | 10 | 27.640 | 22.023 | 24.102 | 1.00 | 38.58 | C |
| ATOM | 72 | O | SER | A | 10 | 28.099 | 21.773 | 22.980 | 1.00 | 40.57 | O |
| ATOM | 73 | CB | SER | A | 10 | 25.715 | 20.509 | 24.659 | 1.00 | 37.63 | C |
| ATOM | 74 | OG | SER | A | 10 | 26.464 | 19.575 | 23.875 | 1.00 | 38.48 | O |
| ATOM | 75 | N | ASN | A | 11 | 28.410 | 22.377 | 25.123 | 1.00 | 39.96 | N |
| ATOM | 76 | CA | ASN | A | 11 | 29.858 | 22.110 | 25.138 | 1.00 | 39.44 | C |
| ATOM | 77 | C | ASN | A | 11 | 30.109 | 20.659 | 24.744 | 1.00 | 39.16 | C |
| ATOM | 78 | O | ASN | A | 11 | 29.410 | 19.775 | 25.243 | 1.00 | 39.47 | O |
| ATOM | 79 | CB | ASN | A | 11 | 30.406 | 22.339 | 26.548 | 1.00 | 41.43 | C |
| ATOM | 80 | CG | ASN | A | 11 | 29.485 | 21.774 | 27.633 | 1.00 | 43.59 | C |
| ATOM | 81 | OD1 | ASN | A | 11 | 28.371 | 21.293 | 27.346 | 1.00 | 45.06 | O |
| ATOM | 82 | ND2 | ASN | A | 11 | 29.944 | 21.830 | 28.885 | 1.00 | 46.08 | N |
| ATOM | 83 | N | PRO | A | 12 | 31.085 | 20.396 | 23.847 | 1.00 | 37.78 | N |
| ATOM | 84 | CA | PRO | A | 12 | 31.223 | 18.999 | 23.423 | 1.00 | 36.71 | C |
| ATOM | 85 | C | PRO | A | 12 | 31.386 | 18.075 | 24.622 | 1.00 | 35.99 | C |
| ATOM | 86 | O | PRO | A | 12 | 32.020 | 18.451 | 25.612 | 1.00 | 36.02 | O |
| ATOM | 87 | CB | PRO | A | 12 | 32.476 | 19.000 | 22.527 | 1.00 | 37.09 | C |
| ATOM | 88 | CG | PRO | A | 12 | 32.612 | 20.426 | 22.066 | 1.00 | 37.59 | C |
| ATOM | 89 | CD | PRO | A | 12 | 32.057 | 21.282 | 23.181 | 1.00 | 37.63 | C |
| ATOM | 90 | N | VAL | A | 13 | 30.794 | 16.891 | 24.538 | 1.00 | 34.70 | N |
| ATOM | 91 | CA | VAL | A | 13 | 30.866 | 15.920 | 25.612 | 1.00 | 33.40 | C |
| ATOM | 92 | C | VAL | A | 13 | 31.713 | 14.722 | 25.175 | 1.00 | 31.97 | C |
| ATOM | 93 | O | VAL | A | 13 | 31.459 | 14.075 | 24.153 | 1.00 | 30.57 | O |
| ATOM | 94 | CB | VAL | A | 13 | 29.457 | 15.470 | 26.115 | 1.00 | 33.61 | C |
| ATOM | 95 | CG1 | VAL | A | 13 | 28.603 | 14.889 | 24.996 | 1.00 | 35.17 | C |
| ATOM | 96 | CG2 | VAL | A | 13 | 29.588 | 14.458 | 27.207 | 1.00 | 33.98 | C |
| ATOM | 97 | N | THR | A | 14 | 32.728 | 14.428 | 25.976 | 1.00 | 30.35 | N |
| ATOM | 98 | CA | THR | A | 14 | 33.562 | 13.287 | 25.733 | 1.00 | 30.49 | C |
| ATOM | 99 | C | THR | A | 14 | 32.716 | 12.029 | 25.764 | 1.00 | 29.31 | C |
| ATOM | 100 | O | THR | A | 14 | 31.877 | 11.850 | 26.638 | 1.00 | 28.24 | O |
| ATOM | 101 | CB | THR | A | 14 | 34.713 | 13.233 | 26.775 | 1.00 | 30.36 | C |
| ATOM | 102 | OG1 | THR | A | 14 | 35.494 | 14.423 | 26.654 | 1.00 | 31.29 | O |
| ATOM | 103 | CG2 | THR | A | 14 | 35.596 | 12.039 | 26.571 | 1.00 | 30.19 | C |
| ATOM | 104 | N | LEU | A | 15 | 32.924 | 11.165 | 24.779 | 1.00 | 29.51 | N |
| ATOM | 105 | CA | LEU | A | 15 | 32.341 | 9.846 | 24.792 | 1.00 | 30.43 | C |
| ATOM | 106 | C | LEU | A | 15 | 32.509 | 9.235 | 26.160 | 1.00 | 30.46 | C |
| ATOM | 107 | O | LEU | A | 15 | 33.525 | 9.430 | 26.813 | 1.00 | 31.52 | O |
| ATOM | 108 | CB | LEU | A | 15 | 33.013 | 8.953 | 23.738 | 1.00 | 31.42 | C |
| ATOM | 109 | CG | LEU | A | 15 | 32.389 | 8.976 | 22.347 | 1.00 | 31.52 | C |
| ATOM | 110 | CD1 | LEU | A | 15 | 33.443 | 8.642 | 21.297 | 1.00 | 32.33 | C |
| ATOM | 111 | CD2 | LEU | A | 15 | 31.239 | 7.991 | 22.283 | 1.00 | 31.33 | C |
| ATOM | 112 | N | GLY | A | 16 | 31.499 | 8.496 | 26.590 | 1.00 | 31.03 | N |
| ATOM | 113 | CA | GLY | A | 16 | 31.506 | 7.844 | 27.880 | 1.00 | 30.54 | C |
| ATOM | 114 | C | GLY | A | 16 | 31.333 | 8.761 | 29.073 | 1.00 | 30.39 | C |
| ATOM | 115 | O | GLY | A | 16 | 31.409 | 8.299 | 30.195 | 1.00 | 31.61 | O |
| ATOM | 116 | N | THR | A | 17 | 31.124 | 10.057 | 28.861 | 1.00 | 30.49 | N |
| ATOM | 117 | CA | THR | A | 17 | 30.840 | 10.954 | 29.991 | 1.00 | 29.54 | C |
| ATOM | 118 | C | THR | A | 17 | 29.443 | 11.568 | 29.931 | 1.00 | 29.13 | C |

| ATOM | 119 | O   | THR | A | 17 | 28.679 | 11.333 | 29.000 | 1.00 | 28.48 | O |
| ATOM | 120 | CB  | THR | A | 17 | 31.907 | 12.044 | 30.145 | 1.00 | 30.41 | C |
| ATOM | 121 | OG1 | THR | A | 17 | 31.748 | 13.054 | 29.146 | 1.00 | 29.10 | O |
| ATOM | 122 | CG2 | THR | A | 17 | 33.301 | 11.427 | 30.061 | 1.00 | 30.65 | C |
| ATOM | 123 | N   | SER | A | 18 | 29.145 | 12.364 | 30.949 | 1.00 | 27.87 | N |
| ATOM | 124 | CA  | SER | A | 18 | 27.828 | 12.900 | 31.213 | 1.00 | 26.55 | C |
| ATOM | 125 | C   | SER | A | 18 | 27.501 | 14.144 | 30.411 | 1.00 | 24.37 | C |
| ATOM | 126 | O   | SER | A | 18 | 28.279 | 15.103 | 30.383 | 1.00 | 23.02 | O |
| ATOM | 127 | CB  | SER | A | 18 | 27.763 | 13.280 | 32.707 | 1.00 | 27.25 | C |
| ATOM | 128 | OG  | SER | A | 18 | 26.437 | 13.482 | 33.099 | 1.00 | 32.45 | O |
| ATOM | 129 | N   | ALA | A | 19 | 26.313 | 14.152 | 29.810 | 1.00 | 23.32 | N |
| ATOM | 130 | CA  | ALA | A | 19 | 25.763 | 15.352 | 29.184 | 1.00 | 21.95 | C |
| ATOM | 131 | C   | ALA | A | 19 | 24.538 | 15.809 | 29.978 | 1.00 | 20.60 | C |
| ATOM | 132 | O   | ALA | A | 19 | 23.831 | 15.014 | 30.569 | 1.00 | 17.09 | O |
| ATOM | 133 | CB  | ALA | A | 19 | 25.360 | 15.059 | 27.772 | 1.00 | 22.25 | C |
| ATOM | 134 | N   | SER | A | 20 | 24.278 | 17.107 | 29.954 | 1.00 | 19.78 | N |
| ATOM | 135 | CA  | SER | A | 20 | 23.147 | 17.698 | 30.645 | 1.00 | 20.51 | C |
| ATOM | 136 | C   | SER | A | 20 | 22.543 | 18.774 | 29.733 | 1.00 | 20.82 | C |
| ATOM | 137 | O   | SER | A | 20 | 23.290 | 19.593 | 29.195 | 1.00 | 21.28 | O |
| ATOM | 138 | CB  | SER | A | 20 | 23.639 | 18.351 | 31.933 | 1.00 | 21.45 | C |
| ATOM | 139 | OG  | SER | A | 20 | 22.568 | 18.906 | 32.681 | 1.00 | 23.13 | O |
| ATOM | 140 | N   | ILE | A | 21 | 21.227 | 18.738 | 29.533 | 1.00 | 19.44 | N |
| ATOM | 141 | CA  | ILE | A | 21 | 20.545 | 19.675 | 28.647 | 1.00 | 19.73 | C |
| ATOM | 142 | C   | ILE | A | 21 | 19.395 | 20.366 | 29.402 | 1.00 | 18.42 | C |
| ATOM | 143 | O   | ILE | A | 21 | 18.502 | 19.723 | 29.931 | 1.00 | 17.10 | O |
| ATOM | 144 | CB  | ILE | A | 21 | 20.043 | 18.985 | 27.342 | 1.00 | 19.35 | C |
| ATOM | 145 | CG1 | ILE | A | 21 | 21.248 | 18.471 | 26.519 | 1.00 | 21.84 | C |
| ATOM | 146 | CG2 | ILE | A | 21 | 19.228 | 19.994 | 26.493 | 1.00 | 20.04 | C |
| ATOM | 147 | CD1 | ILE | A | 21 | 20.923 | 17.452 | 25.428 | 1.00 | 22.11 | C |
| ATOM | 148 | N   | SER | A | 22 | 19.444 | 21.693 | 29.466 | 1.00 | 19.41 | N |
| ATOM | 149 | CA  | SER | A | 22 | 18.452 | 22.454 | 30.205 | 1.00 | 19.05 | C |
| ATOM | 150 | C   | SER | A | 22 | 17.289 | 22.903 | 29.328 | 1.00 | 18.76 | C |
| ATOM | 151 | O   | SER | A | 22 | 17.435 | 23.139 | 28.135 | 1.00 | 18.96 | O |
| ATOM | 152 | CB  | SER | A | 22 | 19.100 | 23.691 | 30.816 | 1.00 | 21.08 | C |
| ATOM | 153 | OG  | SER | A | 22 | 20.041 | 23.297 | 31.802 | 1.00 | 25.94 | O |
| ATOM | 154 | N   | CYS | A | 23 | 16.120 | 23.019 | 29.945 | 1.00 | 17.63 | N |
| ATOM | 155 | CA  | CYS | A | 23 | 14.975 | 23.679 | 29.316 | 1.00 | 17.59 | C |
| ATOM | 156 | C   | CYS | A | 23 | 14.265 | 24.480 | 30.397 | 1.00 | 16.95 | C |
| ATOM | 157 | O   | CYS | A | 23 | 14.345 | 24.159 | 31.585 | 1.00 | 14.46 | O |
| ATOM | 158 | CB  | CYS | A | 23 | 14.043 | 22.650 | 28.638 | 1.00 | 18.49 | C |
| ATOM | 159 | SG  | CYS | A | 23 | 12.546 | 23.380 | 27.802 | 1.00 | 21.60 | S |
| ATOM | 160 | N   | ARG | A | 24 | 13.635 | 25.584 | 29.995 | 1.00 | 17.00 | N |
| ATOM | 161 | CA  | ARG | A | 24 | 12.646 | 26.230 | 30.842 | 1.00 | 19.85 | C |
| ATOM | 162 | C   | ARG | A | 24 | 11.412 | 26.630 | 30.061 | 1.00 | 17.84 | C |
| ATOM | 163 | O   | ARG | A | 24 | 11.439 | 26.834 | 28.828 | 1.00 | 18.31 | O |
| ATOM | 164 | CB  | ARG | A | 24 | 13.210 | 27.456 | 31.577 | 1.00 | 21.63 | C |
| ATOM | 165 | CG  | ARG | A | 24 | 13.791 | 28.492 | 30.719 | 1.00 | 25.24 | C |
| ATOM | 166 | CD  | ARG | A | 24 | 14.309 | 29.668 | 31.548 | 1.00 | 26.95 | C |
| ATOM | 167 | NE  | ARG | A | 24 | 15.288 | 30.415 | 30.766 | 1.00 | 30.38 | N |
| ATOM | 168 | CZ  | ARG | A | 24 | 15.084 | 31.620 | 30.237 | 1.00 | 32.08 | C |
| ATOM | 169 | NH1 | ARG | A | 24 | 13.940 | 32.286 | 30.420 | 1.00 | 33.22 | N |
| ATOM | 170 | NH2 | ARG | A | 24 | 16.065 | 32.176 | 29.543 | 1.00 | 35.34 | N |
| ATOM | 171 | N   | SER | A | 25 | 10.329 | 26.737 | 30.814 | 1.00 | 17.09 | N |
| ATOM | 172 | CA  | SER | A | 25 | 9.023  | 27.069 | 30.301 | 1.00 | 17.25 | C |
| ATOM | 173 | C   | SER | A | 25 | 8.500  | 28.338 | 30.932 | 1.00 | 16.73 | C |
| ATOM | 174 | O   | SER | A | 25 | 8.646  | 28.543 | 32.139 | 1.00 | 17.05 | O |
| ATOM | 175 | CB  | SER | A | 25 | 8.074  | 25.962 | 30.688 | 1.00 | 15.21 | C |
| ATOM | 176 | OG  | SER | A | 25 | 6.806  | 26.229 | 30.159 | 1.00 | 17.33 | O |
| ATOM | 177 | N   | SER | A | 26 | 7.832  | 29.171 | 30.137 | 1.00 | 17.75 | N |
| ATOM | 178 | CA  | SER | A | 26 | 7.317  | 30.448 | 30.631 | 1.00 | 16.95 | C |
| ATOM | 179 | C   | SER | A | 26 | 6.056  | 30.294 | 31.499 | 1.00 | 16.44 | C |
| ATOM | 180 | O   | SER | A | 26 | 5.637  | 31.212 | 32.196 | 1.00 | 17.37 | O |
| ATOM | 181 | CB  | SER | A | 26 | 7.101  | 31.407 | 29.462 | 1.00 | 16.74 | C |
| ATOM | 182 | OG  | SER | A | 26 | 6.039  | 30.992 | 28.634 | 1.00 | 15.23 | O |
| ATOM | 183 | N   | LYS | A | 27 | 5.479  | 29.097 | 31.499 | 1.00 | 15.92 | N |

| ATOM | 184 | CA | LYS | A | 27 | 4.349 | 28.788 | 32.297 | 1.00 | 17.35 | C |
|------|-----|------|-----|---|-----|--------|--------|--------|------|-------|---|
| ATOM | 185 | C | LYS | A | 27 | 4.661 | 27.487 | 32.988 | 1.00 | 15.11 | C |
| ATOM | 186 | O | LYS | A | 27 | 5.252 | 26.618 | 32.393 | 1.00 | 15.63 | O |
| ATOM | 187 | CB | LYS | A | 27 | 3.129 | 28.593 | 31.397 | 1.00 | 19.05 | C |
| ATOM | 188 | CG | LYS | A | 27 | 1.801 | 28.823 | 32.074 | 1.00 | 22.34 | C |
| ATOM | 189 | CD | LYS | A | 27 | 0.715 | 29.052 | 31.017 | 1.00 | 22.48 | C |
| ATOM | 190 | CE | LYS | A | 27 | 0.816 | 30.453 | 30.398 | 1.00 | 25.89 | C |
| ATOM | 191 | NZ | LYS | A | 27 | 0.347 | 31.488 | 31.392 | 1.00 | 25.88 | N |
| ATOM | 192 | N | SER | A | 27A | 4.228 | 27.336 | 34.227 | 1.00 | 14.57 | N |
| ATOM | 193 | CA | SER | A | 27A | 4.347 | 26.048 | 34.890 | 1.00 | 15.48 | C |
| ATOM | 194 | C | SER | A | 27A | 3.640 | 24.954 | 34.118 | 1.00 | 15.74 | C |
| ATOM | 195 | O | SER | A | 27A | 2.499 | 25.135 | 33.691 | 1.00 | 16.96 | O |
| ATOM | 196 | CB | SER | A | 27A | 3.777 | 26.088 | 36.304 | 1.00 | 15.18 | C |
| ATOM | 197 | OG | SER | A | 27A | 3.937 | 24.806 | 36.876 | 1.00 | 15.19 | O |
| ATOM | 198 | N | LEU | A | 27B | 4.311 | 23.810 | 33.973 | 1.00 | 17.11 | N |
| ATOM | 199 | CA | LEU | A | 27B | 3.706 | 22.630 | 33.348 | 1.00 | 16.46 | C |
| ATOM | 200 | C | LEU | A | 27B | 3.240 | 21.605 | 34.344 | 1.00 | 18.86 | C |
| ATOM | 201 | O | LEU | A | 27B | 2.892 | 20.483 | 33.936 | 1.00 | 18.24 | O |
| ATOM | 202 | CB | LEU | A | 27B | 4.695 | 21.964 | 32.374 | 1.00 | 15.09 | C |
| ATOM | 203 | CG | LEU | A | 27B | 5.358 | 22.909 | 31.411 | 1.00 | 13.53 | C |
| ATOM | 204 | CD1 | LEU | A | 27B | 6.260 | 22.169 | 30.483 | 1.00 | 11.99 | C |
| ATOM | 205 | CD2 | LEU | A | 27B | 4.306 | 23.701 | 30.615 | 1.00 | 15.39 | C |
| ATOM | 206 | N | LEU | A | 27C | 3.289 | 21.943 | 35.641 | 1.00 | 19.59 | N |
| ATOM | 207 | CA | LEU | A | 27C | 2.821 | 21.057 | 36.691 | 1.00 | 20.46 | C |
| ATOM | 208 | C | LEU | A | 27C | 1.344 | 21.227 | 36.836 | 1.00 | 21.68 | C |
| ATOM | 209 | O | LEU | A | 27C | 0.879 | 22.312 | 37.189 | 1.00 | 22.20 | O |
| ATOM | 210 | CB | LEU | A | 27C | 3.484 | 21.357 | 38.035 | 1.00 | 19.12 | C |
| ATOM | 211 | CG | LEU | A | 27C | 3.060 | 20.515 | 39.269 | 1.00 | 20.88 | C |
| ATOM | 212 | CD1 | LEU | A | 27C | 3.031 | 18.975 | 39.056 | 1.00 | 18.37 | C |
| ATOM | 213 | CD2 | LEU | A | 27C | 3.959 | 20.817 | 40.431 | 1.00 | 21.29 | C |
| ATOM | 214 | N | HIS | A | 27D | 0.615 | 20.142 | 36.582 | 1.00 | 23.24 | N |
| ATOM | 215 | CA | HIS | A | 27D | -0.837 | 20.148 | 36.567 | 1.00 | 23.19 | C |
| ATOM | 216 | C | HIS | A | 27D | -1.371 | 19.714 | 37.957 | 1.00 | 23.31 | C |
| ATOM | 217 | O | HIS | A | 27D | -0.668 | 19.043 | 38.762 | 1.00 | 20.75 | O |
| ATOM | 218 | CB | HIS | A | 27D | -1.318 | 19.234 | 35.422 | 1.00 | 24.86 | C |
| ATOM | 219 | CG | HIS | A | 27D | -2.796 | 19.254 | 35.182 | 1.00 | 25.81 | C |
| ATOM | 220 | ND1 | HIS | A | 27D | -3.377 | 19.971 | 34.159 | 1.00 | 28.14 | N |
| ATOM | 221 | CD2 | HIS | A | 27D | -3.809 | 18.597 | 35.799 | 1.00 | 28.94 | C |
| ATOM | 222 | CE1 | HIS | A | 27D | -4.687 | 19.781 | 34.179 | 1.00 | 28.42 | C |
| ATOM | 223 | NE2 | HIS | A | 27D | -4.976 | 18.953 | 35.166 | 1.00 | 28.66 | N |
| ATOM | 224 | N | SER | A | 27E | -2.608 | 20.105 | 38.250 | 1.00 | 24.19 | N |
| ATOM | 225 | CA | SER | A | 27E | -3.245 | 19.723 | 39.516 | 1.00 | 25.09 | C |
| ATOM | 226 | C | SER | A | 27E | -3.216 | 18.208 | 39.747 | 1.00 | 25.50 | C |
| ATOM | 227 | O | SER | A | 27E | -3.377 | 17.751 | 40.875 | 1.00 | 27.49 | O |
| ATOM | 228 | CB | SER | A | 27E | -4.701 | 20.178 | 39.525 | 1.00 | 26.62 | C |
| ATOM | 229 | OG | SER | A | 27E | -5.396 | 19.523 | 38.490 | 1.00 | 28.73 | O |
| ATOM | 230 | N | ASP | A | 28 | -2.968 | 17.439 | 38.695 | 1.00 | 24.55 | N |
| ATOM | 231 | CA | ASP | A | 28 | -3.108 | 15.977 | 38.735 | 1.00 | 23.59 | C |
| ATOM | 232 | C | ASP | A | 28 | -1.794 | 15.282 | 39.118 | 1.00 | 23.94 | C |
| ATOM | 233 | O | ASP | A | 28 | -1.711 | 14.033 | 39.204 | 1.00 | 23.64 | O |
| ATOM | 234 | CB | ASP | A | 28 | -3.677 | 15.474 | 37.403 | 1.00 | 25.69 | C |
| ATOM | 235 | CG | ASP | A | 28 | -2.711 | 15.656 | 36.204 | 1.00 | 25.47 | C |
| ATOM | 236 | OD1 | ASP | A | 28 | -1.494 | 15.797 | 36.388 | 1.00 | 25.26 | O |
| ATOM | 237 | OD2 | ASP | A | 28 | -3.201 | 15.615 | 35.060 | 1.00 | 29.84 | O |
| ATOM | 238 | N | GLY | A | 29 | -0.773 | 16.096 | 39.370 | 1.00 | 21.87 | N |
| ATOM | 239 | CA | GLY | A | 29 | 0.516 | 15.595 | 39.856 | 1.00 | 22.61 | C |
| ATOM | 240 | C | GLY | A | 29 | 1.571 | 15.565 | 38.761 | 1.00 | 21.56 | C |
| ATOM | 241 | O | GLY | A | 29 | 2.789 | 15.592 | 39.047 | 1.00 | 24.23 | O |
| ATOM | 242 | N | ILE | A | 30 | 1.102 | 15.514 | 37.508 | 1.00 | 21.22 | N |
| ATOM | 243 | CA | ILE | A | 30 | 1.959 | 15.323 | 36.351 | 1.00 | 20.35 | C |
| ATOM | 244 | C | ILE | A | 30 | 2.483 | 16.654 | 35.868 | 1.00 | 17.85 | C |
| ATOM | 245 | O | ILE | A | 30 | 1.760 | 17.617 | 35.811 | 1.00 | 17.27 | O |
| ATOM | 246 | CB | ILE | A | 30 | 1.209 | 14.617 | 35.194 | 1.00 | 21.45 | C |
| ATOM | 247 | CG1 | ILE | A | 30 | 0.769 | 13.223 | 35.683 | 1.00 | 20.64 | C |
| ATOM | 248 | CG2 | ILE | A | 30 | 2.092 | 14.504 | 33.916 | 1.00 | 20.20 | C |

| ATOM | 249 | CD1 | ILE | A | 30 | -0.134 | 12.427 | 34.719 | 1.00 | 22.08 | C |
|------|-----|-----|-----|---|----|--------|--------|--------|------|-------|---|
| ATOM | 250 | N | THR | A | 31 | 3.758 | 16.645 | 35.495 | 1.00 | 17.73 | N |
| ATOM | 251 | CA | THR | A | 31 | 4.368 | 17.758 | 34.827 | 1.00 | 16.21 | C |
| ATOM | 252 | C | THR | A | 31 | 4.492 | 17.338 | 33.361 | 1.00 | 15.88 | C |
| ATOM | 253 | O | THR | A | 31 | 5.234 | 16.406 | 33.035 | 1.00 | 14.29 | O |
| ATOM | 254 | CB | THR | A | 31 | 5.745 | 18.074 | 35.389 | 1.00 | 16.37 | C |
| ATOM | 255 | OG1 | THR | A | 31 | 5.673 | 18.186 | 36.821 | 1.00 | 15.91 | O |
| ATOM | 256 | CG2 | THR | A | 31 | 6.259 | 19.408 | 34.767 | 1.00 | 17.35 | C |
| ATOM | 257 | N | TYR | A | 32 | 3.827 | 18.085 | 32.506 | 1.00 | 15.00 | N |
| ATOM | 258 | CA | TYR | A | 32 | 3.603 | 17.705 | 31.130 | 1.00 | 16.03 | C |
| ATOM | 259 | C | TYR | A | 32 | 4.720 | 18.299 | 30.287 | 1.00 | 14.92 | C |
| ATOM | 260 | O | TYR | A | 32 | 4.490 | 19.221 | 29.480 | 1.00 | 15.22 | O |
| ATOM | 261 | CB | TYR | A | 32 | 2.231 | 18.167 | 30.653 | 1.00 | 17.09 | C |
| ATOM | 262 | CG | TYR | A | 32 | 1.106 | 17.346 | 31.240 | 1.00 | 17.22 | C |
| ATOM | 263 | CD1 | TYR | A | 32 | 0.515 | 17.683 | 32.458 | 1.00 | 18.70 | C |
| ATOM | 264 | CD2 | TYR | A | 32 | 0.673 | 16.184 | 30.593 | 1.00 | 18.10 | C |
| ATOM | 265 | CE1 | TYR | A | 32 | -0.512 | 16.880 | 33.004 | 1.00 | 19.16 | C |
| ATOM | 266 | CE2 | TYR | A | 32 | -0.369 | 15.411 | 31.109 | 1.00 | 18.41 | C |
| ATOM | 267 | CZ | TYR | A | 32 | -0.945 | 15.760 | 32.315 | 1.00 | 20.08 | C |
| ATOM | 268 | OH | TYR | A | 32 | -1.964 | 14.971 | 32.823 | 1.00 | 19.02 | O |
| ATOM | 269 | N | LEU | A | 33 | 5.914 | 17.786 | 30.543 | 1.00 | 15.68 | N |
| ATOM | 270 | CA | LEU | A | 33 | 7.151 | 18.159 | 29.844 | 1.00 | 15.10 | C |
| ATOM | 271 | C | LEU | A | 33 | 7.588 | 16.933 | 29.075 | 1.00 | 16.30 | C |
| ATOM | 272 | O | LEU | A | 33 | 7.562 | 15.820 | 29.622 | 1.00 | 13.88 | O |
| ATOM | 273 | CB | LEU | A | 33 | 8.216 | 18.588 | 30.847 | 1.00 | 16.49 | C |
| ATOM | 274 | CG | LEU | A | 33 | 9.522 | 19.218 | 30.370 | 1.00 | 16.04 | C |
| ATOM | 275 | CD1 | LEU | A | 33 | 10.554 | 18.166 | 29.997 | 1.00 | 15.44 | C |
| ATOM | 276 | CD2 | LEU | A | 33 | 9.350 | 20.182 | 29.205 | 1.00 | 14.73 | C |
| ATOM | 277 | N | TYR | A | 34 | 7.997 | 17.139 | 27.815 | 1.00 | 15.00 | N |
| ATOM | 278 | CA | TYR | A | 34 | 8.396 | 16.046 | 26.926 | 1.00 | 14.88 | C |
| ATOM | 279 | C | TYR | A | 34 | 9.696 | 16.414 | 26.308 | 1.00 | 15.72 | C |
| ATOM | 280 | O | TYR | A | 34 | 9.966 | 17.596 | 26.095 | 1.00 | 11.03 | O |
| ATOM | 281 | CB | TYR | A | 34 | 7.360 | 15.812 | 25.818 | 1.00 | 13.00 | C |
| ATOM | 282 | CG | TYR | A | 34 | 5.990 | 15.410 | 26.319 | 1.00 | 14.18 | C |
| ATOM | 283 | CD1 | TYR | A | 34 | 5.240 | 16.268 | 27.121 | 1.00 | 10.99 | C |
| ATOM | 284 | CD2 | TYR | A | 34 | 5.444 | 14.155 | 26.017 | 1.00 | 13.26 | C |
| ATOM | 285 | CE1 | TYR | A | 34 | 4.010 | 15.900 | 27.619 | 1.00 | 12.91 | C |
| ATOM | 286 | CE2 | TYR | A | 34 | 4.189 | 13.811 | 26.484 | 1.00 | 12.98 | C |
| ATOM | 287 | CZ | TYR | A | 34 | 3.494 | 14.664 | 27.300 | 1.00 | 12.39 | C |
| ATOM | 288 | OH | TYR | A | 34 | 2.250 | 14.337 | 27.743 | 1.00 | 13.22 | O |
| ATOM | 289 | N | TRP | A | 35 | 10.510 | 15.410 | 26.049 | 1.00 | 14.15 | N |
| ATOM | 290 | CA | TRP | A | 35 | 11.776 | 15.560 | 25.297 | 1.00 | 15.28 | C |
| ATOM | 291 | C | TRP | A | 35 | 11.701 | 14.700 | 24.048 | 1.00 | 15.48 | C |
| ATOM | 292 | O | TRP | A | 35 | 11.287 | 13.532 | 24.136 | 1.00 | 14.13 | O |
| ATOM | 293 | CB | TRP | A | 35 | 12.973 | 15.088 | 26.134 | 1.00 | 15.71 | C |
| ATOM | 294 | CG | TRP | A | 35 | 13.368 | 15.968 | 27.301 | 1.00 | 16.80 | C |
| ATOM | 295 | CD1 | TRP | A | 35 | 13.101 | 15.753 | 28.634 | 1.00 | 15.69 | C |
| ATOM | 296 | CD2 | TRP | A | 35 | 14.108 | 17.193 | 27.234 | 1.00 | 16.13 | C |
| ATOM | 297 | NE1 | TRP | A | 35 | 13.637 | 16.780 | 29.395 | 1.00 | 17.60 | N |
| ATOM | 298 | CE2 | TRP | A | 35 | 14.253 | 17.672 | 28.557 | 1.00 | 16.71 | C |
| ATOM | 299 | CE3 | TRP | A | 35 | 14.663 | 17.927 | 26.187 | 1.00 | 16.93 | C |
| ATOM | 300 | CZ2 | TRP | A | 35 | 14.918 | 18.856 | 28.852 | 1.00 | 16.93 | C |
| ATOM | 301 | CZ3 | TRP | A | 35 | 15.342 | 19.111 | 26.489 | 1.00 | 16.97 | C |
| ATOM | 302 | CH2 | TRP | A | 35 | 15.472 | 19.543 | 27.811 | 1.00 | 16.91 | C |
| ATOM | 303 | N | TYR | A | 36 | 12.116 | 15.273 | 22.907 | 1.00 | 14.64 | N |
| ATOM | 304 | CA | TYR | A | 36 | 12.259 | 14.582 | 21.630 | 1.00 | 14.47 | C |
| ATOM | 305 | C | TYR | A | 36 | 13.718 | 14.646 | 21.158 | 1.00 | 14.43 | C |
| ATOM | 306 | O | TYR | A | 36 | 14.428 | 15.624 | 21.409 | 1.00 | 12.41 | O |
| ATOM | 307 | CB | TYR | A | 36 | 11.317 | 15.164 | 20.547 | 1.00 | 14.52 | C |
| ATOM | 308 | CG | TYR | A | 36 | 9.869 | 15.071 | 20.920 | 1.00 | 13.55 | C |
| ATOM | 309 | CD1 | TYR | A | 36 | 9.226 | 16.068 | 21.610 | 1.00 | 10.56 | C |
| ATOM | 310 | CD2 | TYR | A | 36 | 9.135 | 13.972 | 20.570 | 1.00 | 12.85 | C |
| ATOM | 311 | CE1 | TYR | A | 36 | 7.881 | 15.936 | 21.971 | 1.00 | 11.77 | C |
| ATOM | 312 | CE2 | TYR | A | 36 | 7.825 | 13.832 | 20.928 | 1.00 | 13.02 | C |
| ATOM | 313 | CZ | TYR | A | 36 | 7.197 | 14.781 | 21.633 | 1.00 | 12.72 | C |

```
ATOM   314   OH   TYR A   36      5.873   14.587   21.935   1.00 13.87        O
ATOM   315   N    LEU A   37     14.166   13.566   20.514   1.00 13.21        N
ATOM   316   CA   LEU A   37     15.475   13.510   19.878   1.00 14.70        C
ATOM   317   C    LEU A   37     15.277   13.478   18.374   1.00 15.01        C
ATOM   318   O    LEU A   37     14.479   12.695   17.886   1.00 13.78        O
ATOM   319   CB   LEU A   37     16.236   12.255   20.315   1.00 16.15        C
ATOM   320   CG   LEU A   37     17.614   12.072   19.675   1.00 14.83        C
ATOM   321   CD1  LEU A   37     18.478   13.379   19.704   1.00 15.74        C
ATOM   322   CD2  LEU A   37     18.323   10.961   20.382   1.00 17.12        C
ATOM   323   N    GLN A   38     15.929   14.383   17.654   1.00 15.36        N
ATOM   324   CA   GLN A   38     15.991   14.306   16.209   1.00 16.39        C
ATOM   325   C    GLN A   38     17.402   13.999   15.773   1.00 17.80        C
ATOM   326   O    GLN A   38     18.270   14.872   15.758   1.00 17.21        O
ATOM   327   CB   GLN A   38     15.487   15.609   15.557   1.00 17.06        C
ATOM   328   CG   GLN A   38     15.287   15.470   14.059   1.00 15.44        C
ATOM   329   CD   GLN A   38     14.660   16.693   13.463   1.00 18.11        C
ATOM   330   OE1  GLN A   38     14.789   17.794   14.013   1.00 18.02        O
ATOM   331   NE2  GLN A   38     13.985   16.521   12.348   1.00 20.05        N
ATOM   332   N    LYS A   39     17.646   12.740   15.415   1.00 18.80        N
ATOM   333   CA   LYS A   39     18.956   12.357   14.912   1.00 18.99        C
ATOM   334   C    LYS A   39     19.176   12.845   13.503   1.00 18.80        C
ATOM   335   O    LYS A   39     18.220   13.161   12.795   1.00 19.99        O
ATOM   336   CB   LYS A   39     19.123   10.832   15.007   1.00 18.27        C
ATOM   337   CG   LYS A   39     19.186   10.388   16.434   1.00 19.76        C
ATOM   338   CD   LYS A   39     19.474    8.900   16.532   1.00 18.94        C
ATOM   339   CE   LYS A   39     19.453    8.388   17.947   1.00 20.64        C
ATOM   340   NZ   LYS A   39     19.644    6.886   17.970   1.00 21.60        N
ATOM   341   N    PRO A   40     20.447   12.969   13.088   1.00 21.21        N
ATOM   342   CA   PRO A   40     20.663   13.611   11.807   1.00 22.24        C
ATOM   343   C    PRO A   40     20.026   12.823   10.681   1.00 23.46        C
ATOM   344   O    PRO A   40     20.203   11.589   10.604   1.00 19.94        O
ATOM   345   CB   PRO A   40     22.199   13.649   11.693   1.00 22.74        C
ATOM   346   CG   PRO A   40     22.655   13.678   13.101   1.00 22.87        C
ATOM   347   CD   PRO A   40     21.736   12.646   13.718   1.00 21.62        C
ATOM   348   N    GLY A   41     19.282   13.545    9.832   1.00 24.32        N
ATOM   349   CA   GLY A   41     18.565   12.968    8.708   1.00 24.91        C
ATOM   350   C    GLY A   41     17.317   12.197    9.088   1.00 25.93        C
ATOM   351   O    GLY A   41     16.762   11.443    8.269   1.00 25.78        O
ATOM   352   N    GLN A   42     16.866   12.361   10.328   1.00 25.68        N
ATOM   353   CA   GLN A   42     15.745   11.598   10.845   1.00 25.31        C
ATOM   354   C    GLN A   42     14.643   12.551   11.257   1.00 23.66        C
ATOM   355   O    GLN A   42     14.831   13.768   11.322   1.00 21.25        O
ATOM   356   CB   GLN A   42     16.158   10.772   12.065   1.00 25.71        C
ATOM   357   CG   GLN A   42     17.002    9.533   11.761   1.00 28.03        C
ATOM   358   CD   GLN A   42     17.027    8.509   12.918   1.00 29.68        C
ATOM   359   OE1  GLN A   42     17.856    7.597   12.919   1.00 36.23        O
ATOM   360   NE2  GLN A   42     16.115    8.644   13.892   1.00 32.04        N
ATOM   361   N    SER A   43     13.491   11.984   11.549   1.00 22.70        N
ATOM   362   CA   SER A   43     12.394   12.746   12.105   1.00 23.03        C
ATOM   363   C    SER A   43     12.572   12.776   13.634   1.00 21.62        C
ATOM   364   O    SER A   43     13.353   12.009   14.175   1.00 20.41        O
ATOM   365   CB   SER A   43     11.086   12.096   11.687   1.00 25.11        C
ATOM   366   OG   SER A   43     10.739   11.090   12.611   1.00 30.95        O
ATOM   367   N    PRO A   44     11.880   13.689   14.333   1.00 20.80        N
ATOM   368   CA   PRO A   44     11.963   13.636   15.798   1.00 20.18        C
ATOM   369   C    PRO A   44     11.368   12.328   16.295   1.00 19.56        C
ATOM   370   O    PRO A   44     10.475   11.788   15.636   1.00 18.52        O
ATOM   371   CB   PRO A   44     11.083   14.787   16.253   1.00 20.99        C
ATOM   372   CG   PRO A   44     10.988   15.694   15.061   1.00 20.14        C
ATOM   373   CD   PRO A   44     11.002   14.776   13.877   1.00 21.62        C
ATOM   374   N    HIS A   45     11.840   11.838   17.434   1.00 18.91        N
ATOM   375   CA   HIS A   45     11.154   10.722   18.108   1.00 17.88        C
ATOM   376   C    HIS A   45     11.050   10.994   19.597   1.00 16.71        C
ATOM   377   O    HIS A   45     11.890   11.689   20.163   1.00 14.53        O
ATOM   378   CB   HIS A   45     11.807    9.355   17.808   1.00 19.49        C
```

| ATOM | 379 | CG | HIS | A | 45 | 13.175 | 9.176 | 18.412 | 1.00 | 20.50 | C |
|------|-----|-----|-----|---|----|--------|-------|--------|------|-------|---|
| ATOM | 380 | ND1 | HIS | A | 45 | 14.334 | 9.360 | 17.692 | 1.00 | 22.49 | N |
| ATOM | 381 | CD2 | HIS | A | 45 | 13.564 | 8.840 | 19.666 | 1.00 | 21.92 | C |
| ATOM | 382 | CE1 | HIS | A | 45 | 15.380 | 9.145 | 18.471 | 1.00 | 21.08 | C |
| ATOM | 383 | NE2 | HIS | A | 45 | 14.939 | 8.841 | 19.679 | 1.00 | 20.53 | N |
| ATOM | 384 | N | LEU | A | 46 | 10.010 | 10.461 | 20.219 | 1.00 | 15.87 | N |
| ATOM | 385 | CA | LEU | A | 46 | 9.769 | 10.695 | 21.652 | 1.00 | 16.68 | C |
| ATOM | 386 | C | LEU | A | 46 | 10.855 | 10.050 | 22.512 | 1.00 | 18.16 | C |
| ATOM | 387 | O | LEU | A | 46 | 11.130 | 8.846 | 22.362 | 1.00 | 15.32 | O |
| ATOM | 388 | CB | LEU | A | 46 | 8.420 | 10.116 | 22.058 | 1.00 | 15.61 | C |
| ATOM | 389 | CG | LEU | A | 46 | 8.050 | 10.308 | 23.530 | 1.00 | 15.59 | C |
| ATOM | 390 | CD1 | LEU | A | 46 | 8.000 | 11.794 | 23.906 | 1.00 | 15.95 | C |
| ATOM | 391 | CD2 | LEU | A | 46 | 6.717 | 9.709 | 23.812 | 1.00 | 15.34 | C |
| ATOM | 392 | N | LEU | A | 47 | 11.413 | 10.815 | 23.443 | 1.00 | 17.49 | N |
| ATOM | 393 | CA | LEU | A | 47 | 12.458 | 10.318 | 24.337 | 1.00 | 19.00 | C |
| ATOM | 394 | C | LEU | A | 47 | 11.907 | 10.121 | 25.765 | 1.00 | 19.74 | C |
| ATOM | 395 | O | LEU | A | 47 | 12.023 | 9.046 | 26.360 | 1.00 | 20.17 | O |
| ATOM | 396 | CB | LEU | A | 47 | 13.624 | 11.304 | 24.334 | 1.00 | 18.61 | C |
| ATOM | 397 | CG | LEU | A | 47 | 15.127 | 10.999 | 24.305 | 1.00 | 20.57 | C |
| ATOM | 398 | CD1 | LEU | A | 47 | 15.809 | 11.729 | 25.403 | 1.00 | 19.85 | C |
| ATOM | 399 | CD2 | LEU | A | 47 | 15.446 | 9.540 | 24.411 | 1.00 | 21.42 | C |
| ATOM | 400 | N | ILE | A | 48 | 11.290 | 11.175 | 26.288 | 1.00 | 18.69 | N |
| ATOM | 401 | CA | ILE | A | 48 | 10.778 | 11.226 | 27.652 | 1.00 | 18.86 | C |
| ATOM | 402 | C | ILE | A | 48 | 9.414 | 11.890 | 27.647 | 1.00 | 18.02 | C |
| ATOM | 403 | O | ILE | A | 48 | 9.210 | 12.882 | 26.934 | 1.00 | 15.74 | O |
| ATOM | 404 | CB | ILE | A | 48 | 11.711 | 12.111 | 28.581 | 1.00 | 17.13 | C |
| ATOM | 405 | CG1 | ILE | A | 48 | 13.123 | 11.549 | 28.690 | 1.00 | 20.29 | C |
| ATOM | 406 | CG2 | ILE | A | 48 | 11.077 | 12.363 | 29.967 | 1.00 | 18.33 | C |
| ATOM | 407 | CD1 | ILE | A | 48 | 13.237 | 10.285 | 29.411 | 1.00 | 21.35 | C |
| ATOM | 408 | N | TYR | A | 49 | 8.470 | 11.351 | 28.412 | 1.00 | 18.02 | N |
| ATOM | 409 | CA | TYR | A | 49 | 7.170 | 11.973 | 28.552 | 1.00 | 18.83 | C |
| ATOM | 410 | C | TYR | A | 49 | 6.831 | 12.195 | 30.008 | 1.00 | 18.97 | C |
| ATOM | 411 | O | TYR | A | 49 | 7.377 | 11.535 | 30.878 | 1.00 | 18.73 | O |
| ATOM | 412 | CB | TYR | A | 49 | 6.080 | 11.115 | 27.907 | 1.00 | 19.76 | C |
| ATOM | 413 | CG | TYR | A | 49 | 5.912 | 9.731 | 28.509 | 1.00 | 20.79 | C |
| ATOM | 414 | CD1 | TYR | A | 49 | 6.799 | 8.696 | 28.213 | 1.00 | 21.19 | C |
| ATOM | 415 | CD2 | TYR | A | 49 | 4.842 | 9.460 | 29.350 | 1.00 | 22.03 | C |
| ATOM | 416 | CE1 | TYR | A | 49 | 6.624 | 7.427 | 28.789 | 1.00 | 20.22 | C |
| ATOM | 417 | CE2 | TYR | A | 49 | 4.669 | 8.215 | 29.911 | 1.00 | 21.29 | C |
| ATOM | 418 | CZ | TYR | A | 49 | 5.545 | 7.211 | 29.616 | 1.00 | 21.73 | C |
| ATOM | 419 | OH | TYR | A | 49 | 5.337 | 5.985 | 30.201 | 1.00 | 22.48 | O |
| ATOM | 420 | N | HIS | A | 50 | 5.920 | 13.127 | 30.270 | 1.00 | 19.02 | N |
| ATOM | 421 | CA | HIS | A | 50 | 5.489 | 13.425 | 31.630 | 1.00 | 19.25 | C |
| ATOM | 422 | C | HIS | A | 50 | 6.717 | 13.676 | 32.519 | 1.00 | 18.89 | C |
| ATOM | 423 | O | HIS | A | 50 | 6.794 | 13.157 | 33.630 | 1.00 | 18.72 | O |
| ATOM | 424 | CB | HIS | A | 50 | 4.611 | 12.292 | 32.186 | 1.00 | 18.99 | C |
| ATOM | 425 | CG | HIS | A | 50 | 3.245 | 12.217 | 31.563 | 1.00 | 18.55 | C |
| ATOM | 426 | ND1 | HIS | A | 50 | 2.412 | 11.131 | 31.727 | 1.00 | 20.93 | N |
| ATOM | 427 | CD2 | HIS | A | 50 | 2.591 | 13.074 | 30.737 | 1.00 | 22.07 | C |
| ATOM | 428 | CE1 | HIS | A | 50 | 1.287 | 11.342 | 31.061 | 1.00 | 21.15 | C |
| ATOM | 429 | NE2 | HIS | A | 50 | 1.369 | 12.517 | 30.458 | 1.00 | 21.98 | N |
| ATOM | 430 | N | LEU | A | 51 | 7.676 | 14.452 | 31.992 | 1.00 | 19.70 | N |
| ATOM | 431 | CA | LEU | A | 51 | 8.883 | 14.909 | 32.698 | 1.00 | 18.94 | C |
| ATOM | 432 | C | LEU | A | 51 | 10.027 | 13.897 | 32.893 | 1.00 | 20.43 | C |
| ATOM | 433 | O | LEU | A | 51 | 11.199 | 14.220 | 32.657 | 1.00 | 22.16 | O |
| ATOM | 434 | CB | LEU | A | 51 | 8.490 | 15.536 | 34.057 | 1.00 | 19.98 | C |
| ATOM | 435 | CG | LEU | A | 51 | 9.639 | 16.103 | 34.911 | 1.00 | 19.17 | C |
| ATOM | 436 | CD1 | LEU | A | 51 | 10.379 | 17.245 | 34.203 | 1.00 | 18.81 | C |
| ATOM | 437 | CD2 | LEU | A | 51 | 9.128 | 16.588 | 36.231 | 1.00 | 19.20 | C |
| ATOM | 438 | N | SER | A | 52 | 9.705 | 12.694 | 33.365 | 1.00 | 17.94 | N |
| ATOM | 439 | CA | SER | A | 52 | 10.725 | 11.771 | 33.848 | 1.00 | 19.02 | C |
| ATOM | 440 | C | SER | A | 52 | 10.509 | 10.344 | 33.389 | 1.00 | 19.35 | C |
| ATOM | 441 | O | SER | A | 52 | 11.268 | 9.453 | 33.777 | 1.00 | 18.65 | O |
| ATOM | 442 | CB | SER | A | 52 | 10.769 | 11.824 | 35.373 | 1.00 | 18.00 | C |
| ATOM | 443 | OG | SER | A | 52 | 9.471 | 11.589 | 35.886 | 1.00 | 23.69 | O |

| ATOM | 444 | N   | ASN | A | 53 | 9.507  | 10.124 | 32.544 | 1.00 | 19.90 | N |
|------|-----|-----|-----|---|----|--------|--------|--------|------|-------|---|
| ATOM | 445 | CA  | ASN | A | 53 | 9.222  | 8.781  | 32.063 | 1.00 | 20.84 | C |
| ATOM | 446 | C   | ASN | A | 53 | 9.869  | 8.474  | 30.705 | 1.00 | 21.85 | C |
| ATOM | 447 | O   | ASN | A | 53 | 9.666  | 9.189  | 29.746 | 1.00 | 21.70 | O |
| ATOM | 448 | CB  | ASN | A | 53 | 7.717  | 8.544  | 32.013 | 1.00 | 21.52 | C |
| ATOM | 449 | CG  | ASN | A | 53 | 7.074  | 8.668  | 33.374 | 1.00 | 24.43 | C |
| ATOM | 450 | OD1 | ASN | A | 53 | 7.341  | 7.868  | 34.279 | 1.00 | 26.79 | O |
| ATOM | 451 | ND2 | ASN | A | 53 | 6.288  | 9.721  | 33.557 | 1.00 | 26.92 | N |
| ATOM | 452 | N   | LEU | A | 54 | 10.628 | 7.386  | 30.636 | 1.00 | 22.65 | N |
| ATOM | 453 | CA  | LEU | A | 54 | 11.326 | 7.016  | 29.410 | 1.00 | 22.90 | C |
| ATOM | 454 | C   | LEU | A | 54 | 10.338 | 6.337  | 28.464 | 1.00 | 22.86 | C |
| ATOM | 455 | O   | LEU | A | 54 | 9.575  | 5.457  | 28.890 | 1.00 | 23.17 | O |
| ATOM | 456 | CB  | LEU | A | 54 | 12.520 | 6.075  | 29.705 | 1.00 | 23.28 | C |
| ATOM | 457 | CG  | LEU | A | 54 | 13.885 | 6.664  | 30.062 | 1.00 | 24.69 | C |
| ATOM | 458 | CD1 | LEU | A | 54 | 13.827 | 7.556  | 31.276 | 1.00 | 26.56 | C |
| ATOM | 459 | CD2 | LEU | A | 54 | 14.923 | 5.567  | 30.285 | 1.00 | 24.59 | C |
| ATOM | 460 | N   | ALA | A | 55 | 10.368 | 6.724  | 27.189 | 1.00 | 22.21 | N |
| ATOM | 461 | CA  | ALA | A | 55 | 9.545  | 6.075  | 26.156 | 1.00 | 24.08 | C |
| ATOM | 462 | C   | ALA | A | 55 | 10.035 | 4.645  | 25.938 | 1.00 | 24.61 | C |
| ATOM | 463 | O   | ALA | A | 55 | 11.143 | 4.295  | 26.338 | 1.00 | 23.72 | O |
| ATOM | 464 | CB  | ALA | A | 55 | 9.605  | 6.859  | 24.858 | 1.00 | 23.80 | C |
| ATOM | 465 | N   | SER | A | 56 | 9.232  | 3.818  | 25.278 | 1.00 | 27.47 | N |
| ATOM | 466 | CA  | SER | A | 56 | 9.656  | 2.434  | 25.031 | 1.00 | 28.69 | C |
| ATOM | 467 | C   | SER | A | 56 | 10.963 | 2.379  | 24.221 | 1.00 | 29.49 | C |
| ATOM | 468 | O   | SER | A | 56 | 11.176 | 3.169  | 23.296 | 1.00 | 29.23 | O |
| ATOM | 469 | CB  | SER | A | 56 | 8.553  | 1.648  | 24.331 | 1.00 | 30.27 | C |
| ATOM | 470 | OG  | SER | A | 56 | 8.812  | 0.254  | 24.424 | 1.00 | 34.70 | O |
| ATOM | 471 | N   | GLY | A | 57 | 11.850 | 1.462  | 24.593 | 1.00 | 29.73 | N |
| ATOM | 472 | CA  | GLY | A | 57 | 13.141 | 1.315  | 23.928 | 1.00 | 29.49 | C |
| ATOM | 473 | C   | GLY | A | 57 | 14.226 | 2.312  | 24.307 | 1.00 | 30.27 | C |
| ATOM | 474 | O   | GLY | A | 57 | 15.406 | 2.100  | 23.954 | 1.00 | 31.85 | O |
| ATOM | 475 | N   | VAL | A | 58 | 13.885 | 3.396  | 25.017 | 1.00 | 28.82 | N |
| ATOM | 476 | CA  | VAL | A | 58 | 14.910 | 4.399  | 25.342 | 1.00 | 27.62 | C |
| ATOM | 477 | C   | VAL | A | 58 | 15.827 | 3.824  | 26.419 | 1.00 | 27.47 | C |
| ATOM | 478 | O   | VAL | A | 58 | 15.331 | 3.333  | 27.429 | 1.00 | 26.71 | O |
| ATOM | 479 | CB  | VAL | A | 58 | 14.312 | 5.755  | 25.823 | 1.00 | 26.28 | C |
| ATOM | 480 | CG1 | VAL | A | 58 | 15.403 | 6.718  | 26.259 | 1.00 | 25.15 | C |
| ATOM | 481 | CG2 | VAL | A | 58 | 13.460 | 6.370  | 24.736 | 1.00 | 25.71 | C |
| ATOM | 482 | N   | PRO | A | 59 | 17.157 | 3.927  | 26.225 | 1.00 | 28.29 | N |
| ATOM | 483 | CA  | PRO | A | 59 | 18.140 | 3.425  | 27.180 | 1.00 | 28.26 | C |
| ATOM | 484 | C   | PRO | A | 59 | 18.087 | 4.156  | 28.516 | 1.00 | 28.72 | C |
| ATOM | 485 | O   | PRO | A | 59 | 17.950 | 5.381  | 28.555 | 1.00 | 29.32 | O |
| ATOM | 486 | CB  | PRO | A | 59 | 19.489 | 3.727  | 26.512 | 1.00 | 28.07 | C |
| ATOM | 487 | CG  | PRO | A | 59 | 19.214 | 4.092  | 25.163 | 1.00 | 29.42 | C |
| ATOM | 488 | CD  | PRO | A | 59 | 17.814 | 4.576  | 25.076 | 1.00 | 29.74 | C |
| ATOM | 489 | N   | ASP | A | 60 | 18.256 | 3.419  | 29.601 | 1.00 | 27.77 | N |
| ATOM | 490 | CA  | ASP | A | 60 | 18.232 | 4.035  | 30.909 | 1.00 | 27.93 | C |
| ATOM | 491 | C   | ASP | A | 60 | 19.438 | 4.930  | 31.191 | 1.00 | 26.08 | C |
| ATOM | 492 | O   | ASP | A | 60 | 19.527 | 5.493  | 32.269 | 1.00 | 26.59 | O |
| ATOM | 493 | CB  | ASP | A | 60 | 18.005 | 2.986  | 32.021 | 1.00 | 30.14 | C |
| ATOM | 494 | CG  | ASP | A | 60 | 19.131 | 1.989  | 32.159 | 1.00 | 32.40 | C |
| ATOM | 495 | OD1 | ASP | A | 60 | 20.238 | 2.212  | 31.609 | 1.00 | 35.28 | O |
| ATOM | 496 | OD2 | ASP | A | 60 | 18.893 | 0.964  | 32.856 | 1.00 | 34.82 | O |
| ATOM | 497 | N   | ARG | A | 61 | 20.354 | 5.088  | 30.234 | 1.00 | 24.80 | N |
| ATOM | 498 | CA  | ARG | A | 61 | 21.368 | 6.124  | 30.336 | 1.00 | 24.71 | C |
| ATOM | 499 | C   | ARG | A | 61 | 20.692 | 7.497  | 30.301 | 1.00 | 22.50 | C |
| ATOM | 500 | O   | ARG | A | 61 | 21.277 | 8.476  | 30.736 | 1.00 | 21.00 | O |
| ATOM | 501 | CB  | ARG | A | 61 | 22.397 | 6.027  | 29.200 | 1.00 | 26.14 | C |
| ATOM | 502 | CG  | ARG | A | 61 | 23.145 | 4.697  | 29.167 | 1.00 | 29.30 | C |
| ATOM | 503 | CD  | ARG | A | 61 | 24.103 | 4.600  | 27.985 | 1.00 | 29.48 | C |
| ATOM | 504 | NE  | ARG | A | 61 | 23.446 | 4.626  | 26.668 | 1.00 | 32.46 | N |
| ATOM | 505 | CZ  | ARG | A | 61 | 23.288 | 5.706  | 25.901 | 1.00 | 30.38 | C |
| ATOM | 506 | NH1 | ARG | A | 61 | 23.718 | 6.893  | 26.314 | 1.00 | 30.04 | N |
| ATOM | 507 | NH2 | ARG | A | 61 | 22.694 | 5.581  | 24.716 | 1.00 | 31.26 | N |
| ATOM | 508 | N   | PHE | A | 62 | 19.474 | 7.545  | 29.769 | 1.00 | 22.09 | N |

| ATOM | 509 | CA  | PHE | A | 62 | 18.664 | 8.784  | 29.741 | 1.00 | 20.84 | C |
| ATOM | 510 | C   | PHE | A | 62 | 17.768 | 8.969  | 30.985 | 1.00 | 19.77 | C |
| ATOM | 511 | O   | PHE | A | 62 | 17.154 | 8.038  | 31.477 | 1.00 | 19.35 | O |
| ATOM | 512 | CB  | PHE | A | 62 | 17.800 | 8.792  | 28.486 | 1.00 | 20.51 | C |
| ATOM | 513 | CG  | PHE | A | 62 | 18.596 | 8.910  | 27.217 | 1.00 | 20.61 | C |
| ATOM | 514 | CD1 | PHE | A | 62 | 19.052 | 7.773  | 26.556 | 1.00 | 21.07 | C |
| ATOM | 515 | CD2 | PHE | A | 62 | 18.887 | 10.155 | 26.687 | 1.00 | 19.59 | C |
| ATOM | 516 | CE1 | PHE | A | 62 | 19.791 | 7.891  | 25.374 | 1.00 | 21.03 | C |
| ATOM | 517 | CE2 | PHE | A | 62 | 19.617 | 10.289 | 25.499 | 1.00 | 20.08 | C |
| ATOM | 518 | CZ  | PHE | A | 62 | 20.070 | 9.172  | 24.845 | 1.00 | 19.89 | C |
| ATOM | 519 | N   | SER | A | 63 | 17.694 | 10.207 | 31.480 | 1.00 | 17.23 | N |
| ATOM | 520 | CA  | SER | A | 63 | 16.893 | 10.494 | 32.639 | 1.00 | 16.65 | C |
| ATOM | 521 | C   | SER | A | 63 | 16.540 | 11.968 | 32.611 | 1.00 | 15.95 | C |
| ATOM | 522 | O   | SER | A | 63 | 17.264 | 12.754 | 32.004 | 1.00 | 15.66 | O |
| ATOM | 523 | CB  | SER | A | 63 | 17.658 | 10.141 | 33.926 | 1.00 | 16.64 | C |
| ATOM | 524 | OG  | SER | A | 63 | 18.685 | 11.065 | 34.241 | 1.00 | 16.18 | O |
| ATOM | 525 | N   | SER | A | 64 | 15.459 | 12.326 | 33.282 | 1.00 | 14.81 | N |
| ATOM | 526 | CA  | SER | A | 64 | 14.975 | 13.702 | 33.236 | 1.00 | 15.50 | C |
| ATOM | 527 | C   | SER | A | 64 | 14.302 | 14.071 | 34.551 | 1.00 | 14.76 | C |
| ATOM | 528 | O   | SER | A | 64 | 13.629 | 13.257 | 35.156 | 1.00 | 17.17 | O |
| ATOM | 529 | CB  | SER | A | 64 | 14.005 | 13.852 | 32.063 | 1.00 | 15.11 | C |
| ATOM | 530 | OG  | SER | A | 64 | 13.467 | 15.169 | 31.991 | 1.00 | 18.02 | O |
| ATOM | 531 | N   | SER | A | 65 | 14.522 | 15.304 | 34.987 | 1.00 | 13.04 | N |
| ATOM | 532 | CA  | SER | A | 65 | 14.014 | 15.827 | 36.261 | 1.00 | 13.38 | C |
| ATOM | 533 | C   | SER | A | 65 | 13.579 | 17.255 | 36.047 | 1.00 | 11.83 | C |
| ATOM | 534 | O   | SER | A | 65 | 13.976 | 17.873 | 35.066 | 1.00 | 13.68 | O |
| ATOM | 535 | CB  | SER | A | 65 | 15.090 | 15.804 | 37.366 | 1.00 | 13.70 | C |
| ATOM | 536 | OG  | SER | A | 65 | 16.279 | 16.570 | 37.082 | 1.00 | 15.18 | O |
| ATOM | 537 | N   | GLY | A | 66 | 12.735 | 17.766 | 36.932 | 1.00 | 11.12 | N |
| ATOM | 538 | CA  | GLY | A | 66 | 12.440 | 19.217 | 36.901 | 1.00 | 11.68 | C |
| ATOM | 539 | C   | GLY | A | 66 | 11.263 | 19.676 | 37.726 | 1.00 | 10.17 | C |
| ATOM | 540 | O   | GLY | A | 66 | 10.532 | 18.884 | 38.273 | 1.00 | 11.42 | O |
| ATOM | 541 | N   | SER | A | 67 | 11.072 | 21.016 | 37.797 | 1.00 | 12.24 | N |
| ATOM | 542 | CA  | SER | A | 67 | 9.925  | 21.584 | 38.464 | 1.00 | 12.23 | C |
| ATOM | 543 | C   | SER | A | 67 | 8.795  | 21.760 | 37.466 | 1.00 | 14.01 | C |
| ATOM | 544 | O   | SER | A | 67 | 8.769  | 21.086 | 36.471 | 1.00 | 16.52 | O |
| ATOM | 545 | CB  | SER | A | 67 | 10.310 | 22.931 | 39.070 | 1.00 | 12.52 | C |
| ATOM | 546 | OG  | SER | A | 67 | 10.811 | 23.720 | 38.039 | 1.00 | 13.56 | O |
| ATOM | 547 | N   | GLY | A | 68 | 7.856  | 22.678 | 37.718 | 1.00 | 12.94 | N |
| ATOM | 548 | CA  | GLY | A | 68 | 6.898  | 23.058 | 36.718 | 1.00 | 11.77 | C |
| ATOM | 549 | C   | GLY | A | 68 | 7.499  | 23.907 | 35.608 | 1.00 | 13.34 | C |
| ATOM | 550 | O   | GLY | A | 68 | 6.933  | 23.957 | 34.531 | 1.00 | 13.45 | O |
| ATOM | 551 | N   | THR | A | 69 | 8.645  | 24.558 | 35.857 | 1.00 | 13.02 | N |
| ATOM | 552 | CA  | THR | A | 69 | 9.188  | 25.530 | 34.950 | 1.00 | 14.86 | C |
| ATOM | 553 | C   | THR | A | 69 | 10.643 | 25.348 | 34.492 | 1.00 | 15.26 | C |
| ATOM | 554 | O   | THR | A | 69 | 11.064 | 25.978 | 33.520 | 1.00 | 14.45 | O |
| ATOM | 555 | CB  | THR | A | 69 | 9.051  | 26.922 | 35.575 | 1.00 | 15.57 | C |
| ATOM | 556 | OG1 | THR | A | 69 | 9.768  | 26.967 | 36.809 | 1.00 | 14.71 | O |
| ATOM | 557 | CG2 | THR | A | 69 | 7.633  | 27.216 | 35.849 | 1.00 | 15.58 | C |
| ATOM | 558 | N   | ASP | A | 70 | 11.425 | 24.515 | 35.173 | 1.00 | 15.21 | N |
| ATOM | 559 | CA  | ASP | A | 70 | 12.844 | 24.356 | 34.856 | 1.00 | 14.26 | C |
| ATOM | 560 | C   | ASP | A | 70 | 13.166 | 22.877 | 34.820 | 1.00 | 14.44 | C |
| ATOM | 561 | O   | ASP | A | 70 | 12.895 | 22.186 | 35.775 | 1.00 | 13.62 | O |
| ATOM | 562 | CB  | ASP | A | 70 | 13.707 | 24.956 | 35.959 | 1.00 | 15.50 | C |
| ATOM | 563 | CG  | ASP | A | 70 | 13.595 | 26.440 | 36.014 | 1.00 | 16.86 | C |
| ATOM | 564 | OD1 | ASP | A | 70 | 14.008 | 27.076 | 35.037 | 1.00 | 21.01 | O |
| ATOM | 565 | OD2 | ASP | A | 70 | 13.063 | 26.945 | 37.007 | 1.00 | 21.12 | O |
| ATOM | 566 | N   | PHE | A | 71 | 13.793 | 22.429 | 33.735 | 1.00 | 14.69 | N |
| ATOM | 567 | CA  | PHE | A | 71 | 13.968 | 20.999 | 33.469 | 1.00 | 14.77 | C |
| ATOM | 568 | C   | PHE | A | 71 | 15.369 | 20.628 | 33.025 | 1.00 | 16.21 | C |
| ATOM | 569 | O   | PHE | A | 71 | 16.084 | 21.439 | 32.429 | 1.00 | 17.57 | O |
| ATOM | 570 | CB  | PHE | A | 71 | 12.969 | 20.552 | 32.392 | 1.00 | 13.95 | C |
| ATOM | 571 | CG  | PHE | A | 71 | 11.654 | 21.245 | 32.465 | 1.00 | 14.09 | C |
| ATOM | 572 | CD1 | PHE | A | 71 | 10.754 | 20.922 | 33.443 | 1.00 | 13.73 | C |
| ATOM | 573 | CD2 | PHE | A | 71 | 11.327 | 22.240 | 31.560 | 1.00 | 15.19 | C |

```
ATOM   574   CE1  PHE A  71      9.516   21.567   33.527   1.00 14.50           C
ATOM   575   CE2  PHE A  71     10.121   22.882   31.629   1.00 15.89           C
ATOM   576   CZ   PHE A  71      9.210   22.565   32.617   1.00 14.34           C
ATOM   577   N    THR A  72     15.752   19.372   33.284   1.00 16.28           N
ATOM   578   CA   THR A  72     17.110   18.925   32.973   1.00 16.11           C
ATOM   579   C    THR A  72     17.108   17.499   32.405   1.00 16.67           C
ATOM   580   O    THR A  72     16.623   16.568   33.059   1.00 17.02           O
ATOM   581   CB   THR A  72     18.071   19.020   34.218   1.00 16.72           C
ATOM   582   OG1  THR A  72     17.988   20.318   34.823   1.00 16.66           O
ATOM   583   CG2  THR A  72     19.497   18.788   33.802   1.00 17.25           C
ATOM   584   N    LEU A  73     17.651   17.348   31.197   1.00 15.19           N
ATOM   585   CA   LEU A  73     17.878   16.020   30.579   1.00 17.18           C
ATOM   586   C    LEU A  73     19.321   15.646   30.821   1.00 17.41           C
ATOM   587   O    LEU A  73     20.231   16.424   30.518   1.00 16.80           O
ATOM   588   CB   LEU A  73     17.601   16.055   29.073   1.00 15.95           C
ATOM   589   CG   LEU A  73     17.765   14.734   28.294   1.00 16.28           C
ATOM   590   CD1  LEU A  73     16.705   13.791   28.717   1.00 15.18           C
ATOM   591   CD2  LEU A  73     17.700   15.006   26.792   1.00 18.03           C
ATOM   592   N    ARG A  74     19.538   14.435   31.318   1.00 19.04           N
ATOM   593   CA   ARG A  74     20.872   13.963   31.594   1.00 20.21           C
ATOM   594   C    ARG A  74     21.065   12.675   30.821   1.00 20.39           C
ATOM   595   O    ARG A  74     20.157   11.862   30.771   1.00 19.75           O
ATOM   596   CB   ARG A  74     21.075   13.763   33.102   1.00 21.53           C
ATOM   597   CG   ARG A  74     22.529   13.527   33.461   1.00 22.32           C
ATOM   598   CD   ARG A  74     22.843   13.638   34.904   1.00 24.33           C
ATOM   599   NE   ARG A  74     24.140   13.014   35.194   1.00 28.12           N
ATOM   600   CZ   ARG A  74     24.323   11.691   35.337   1.00 30.68           C
ATOM   601   NH1  ARG A  74     23.311   10.828   35.202   1.00 33.36           N
ATOM   602   NH2  ARG A  74     25.536   11.215   35.601   1.00 31.76           N
ATOM   603   N    ILE A  75     22.204   12.548   30.135   1.00 22.25           N
ATOM   604   CA   ILE A  75     22.602   11.259   29.523   1.00 23.02           C
ATOM   605   C    ILE A  75     23.850   10.868   30.296   1.00 23.46           C
ATOM   606   O    ILE A  75     24.784   11.654   30.372   1.00 24.34           O
ATOM   607   CB   ILE A  75     22.946   11.390   28.022   1.00 23.13           C
ATOM   608   CG1  ILE A  75     21.810   12.083   27.269   1.00 23.59           C
ATOM   609   CG2  ILE A  75     23.251   10.018   27.430   1.00 23.59           C
ATOM   610   CD1  ILE A  75     22.261   12.891   25.991   1.00 23.52           C
ATOM   611   N    SER A  76     23.874    9.677   30.889   1.00 26.14           N
ATOM   612   CA   SER A  76     24.904    9.395   31.914   1.00 27.82           C
ATOM   613   C    SER A  76     26.300    9.121   31.359   1.00 29.73           C
ATOM   614   O    SER A  76     27.317    9.530   31.982   1.00 31.15           O
ATOM   615   CB   SER A  76     24.466    8.230   32.798   1.00 28.09           C
ATOM   616   OG   SER A  76     24.056    7.172   31.979   1.00 28.69           O
ATOM   617   N    ARG A  77     26.337    8.419   30.220   1.00 30.38           N
ATOM   618   CA   ARG A  77     27.568    7.986   29.558   1.00 31.07           C
ATOM   619   C    ARG A  77     27.306    8.028   28.060   1.00 30.29           C
ATOM   620   O    ARG A  77     26.834    7.061   27.467   1.00 31.21           O
ATOM   621   CB   ARG A  77     27.946    6.550   29.966   1.00 32.45           C
ATOM   622   CG   ARG A  77     28.508    6.409   31.394   1.00 34.64           C
ATOM   623   CD   ARG A  77     28.430    4.969   31.902   1.00 35.70           C
ATOM   624   NE   ARG A  77     27.038    4.488   31.963   1.00 38.62           N
ATOM   625   CZ   ARG A  77     26.171    4.717   32.961   1.00 39.74           C
ATOM   626   NH1  ARG A  77     26.524    5.411   34.044   1.00 40.63           N
ATOM   627   NH2  ARG A  77     24.929    4.233   32.885   1.00 39.96           N
ATOM   628   N    VAL A  78     27.619    9.159   27.452   1.00 29.50           N
ATOM   629   CA   VAL A  78     27.194    9.412   26.087   1.00 28.68           C
ATOM   630   C    VAL A  78     27.843    8.423   25.103   1.00 27.53           C
ATOM   631   O    VAL A  78     29.020    8.049   25.243   1.00 25.88           O
ATOM   632   CB   VAL A  78     27.475   10.882   25.694   1.00 28.74           C
ATOM   633   CG1  VAL A  78     27.198   11.132   24.215   1.00 29.50           C
ATOM   634   CG2  VAL A  78     26.619   11.814   26.523   1.00 28.90           C
ATOM   635   N    GLU A  79     27.046    8.002   24.124   1.00 26.56           N
ATOM   636   CA   GLU A  79     27.492    7.137   23.046   1.00 27.09           C
ATOM   637   C    GLU A  79     27.314    7.846   21.724   1.00 25.13           C
ATOM   638   O    GLU A  79     26.490    8.739   21.595   1.00 24.16           O
```

| ATOM | 639 | CB  | GLU | A | 79 | 26.695 | 5.840  | 23.059 | 1.00 | 27.55 | C |
| ---- | --- | --- | --- | - | -- | ------ | ------ | ------ | ---- | ----- | - |
| ATOM | 640 | CG  | GLU | A | 79 | 26.976 | 5.038  | 24.300 | 1.00 | 30.06 | C |
| ATOM | 641 | CD  | GLU | A | 79 | 26.024 | 3.881  | 24.505 | 1.00 | 30.22 | C |
| ATOM | 642 | OE1 | GLU | A | 79 | 25.252 | 3.541  | 23.568 | 1.00 | 34.24 | O |
| ATOM | 643 | OE2 | GLU | A | 79 | 26.039 | 3.331  | 25.629 | 1.00 | 33.46 | O |
| ATOM | 644 | N   | ALA | A | 80 | 28.120 | 7.452  | 20.742 | 1.00 | 24.98 | N |
| ATOM | 645 | CA  | ALA | A | 80 | 28.102 | 8.074  | 19.422 | 1.00 | 24.81 | C |
| ATOM | 646 | C   | ALA | A | 80 | 26.676 | 8.271  | 18.877 | 1.00 | 23.14 | C |
| ATOM | 647 | O   | ALA | A | 80 | 26.364 | 9.338  | 18.333 | 1.00 | 23.23 | O |
| ATOM | 648 | CB  | ALA | A | 80 | 28.932 | 7.254  | 18.451 | 1.00 | 23.94 | C |
| ATOM | 649 | N   | GLU | A | 81 | 25.831 | 7.249  | 19.053 | 1.00 | 22.46 | N |
| ATOM | 650 | CA  | GLU | A | 81 | 24.451 | 7.214  | 18.526 | 1.00 | 24.16 | C |
| ATOM | 651 | C   | GLU | A | 81 | 23.521 | 8.301  | 19.097 | 1.00 | 22.19 | C |
| ATOM | 652 | O   | GLU | A | 81 | 22.460 | 8.566  | 18.548 | 1.00 | 21.99 | O |
| ATOM | 653 | CB  | GLU | A | 81 | 23.812 | 5.862  | 18.849 | 1.00 | 25.85 | C |
| ATOM | 654 | CG  | GLU | A | 81 | 23.469 | 5.740  | 20.344 | 1.00 | 30.90 | C |
| ATOM | 655 | CD  | GLU | A | 81 | 23.319 | 4.328  | 20.858 | 1.00 | 31.21 | C |
| ATOM | 656 | OE1 | GLU | A | 81 | 24.327 | 3.562  | 20.908 | 1.00 | 38.97 | O |
| ATOM | 657 | OE2 | GLU | A | 81 | 22.183 | 3.985  | 21.261 | 1.00 | 38.71 | O |
| ATOM | 658 | N   | ASP | A | 82 | 23.916 | 8.905  | 20.207 | 1.00 | 20.07 | N |
| ATOM | 659 | CA  | ASP | A | 82 | 23.077 | 9.847  | 20.917 | 1.00 | 19.72 | C |
| ATOM | 660 | C   | ASP | A | 82 | 23.135 | 11.224 | 20.296 | 1.00 | 19.09 | C |
| ATOM | 661 | O   | ASP | A | 82 | 22.380 | 12.073 | 20.719 | 1.00 | 19.94 | O |
| ATOM | 662 | CB  | ASP | A | 82 | 23.514 | 9.977  | 22.375 | 1.00 | 19.25 | C |
| ATOM | 663 | CG  | ASP | A | 82 | 23.425 | 8.667  | 23.123 | 1.00 | 19.19 | C |
| ATOM | 664 | OD1 | ASP | A | 82 | 22.608 | 7.836  | 22.727 | 1.00 | 21.23 | O |
| ATOM | 665 | OD2 | ASP | A | 82 | 24.149 | 8.502  | 24.098 | 1.00 | 18.05 | O |
| ATOM | 666 | N   | VAL | A | 83 | 24.037 | 11.454 | 19.337 | 1.00 | 17.57 | N |
| ATOM | 667 | CA  | VAL | A | 83 | 24.139 | 12.790 | 18.696 | 1.00 | 17.25 | C |
| ATOM | 668 | C   | VAL | A | 83 | 22.860 | 13.158 | 17.958 | 1.00 | 15.41 | C |
| ATOM | 669 | O   | VAL | A | 83 | 22.199 | 12.314 | 17.365 | 1.00 | 14.64 | O |
| ATOM | 670 | CB  | VAL | A | 83 | 25.299 | 12.926 | 17.671 | 1.00 | 17.66 | C |
| ATOM | 671 | CG1 | VAL | A | 83 | 26.599 | 12.956 | 18.366 | 1.00 | 20.16 | C |
| ATOM | 672 | CG2 | VAL | A | 83 | 25.235 | 11.817 | 16.601 | 1.00 | 17.23 | C |
| ATOM | 673 | N   | GLY | A | 84 | 22.523 | 14.446 | 17.980 | 1.00 | 15.47 | N |
| ATOM | 674 | CA  | GLY | A | 84 | 21.300 | 14.908 | 17.365 | 1.00 | 16.14 | C |
| ATOM | 675 | C   | GLY | A | 84 | 20.863 | 16.191 | 18.053 | 1.00 | 17.14 | C |
| ATOM | 676 | O   | GLY | A | 84 | 21.627 | 16.783 | 18.784 | 1.00 | 16.96 | O |
| ATOM | 677 | N   | ILE | A | 85 | 19.621 | 16.591 | 17.806 | 1.00 | 17.35 | N |
| ATOM | 678 | CA  | ILE | A | 85 | 19.050 | 17.796 | 18.377 | 1.00 | 17.34 | C |
| ATOM | 679 | C   | ILE | A | 85 | 17.939 | 17.378 | 19.354 | 1.00 | 17.26 | C |
| ATOM | 680 | O   | ILE | A | 85 | 17.018 | 16.598 | 18.985 | 1.00 | 16.15 | O |
| ATOM | 681 | CB  | ILE | A | 85 | 18.514 | 18.720 | 17.270 | 1.00 | 18.40 | C |
| ATOM | 682 | CG1 | ILE | A | 85 | 19.646 | 19.074 | 16.293 | 1.00 | 20.14 | C |
| ATOM | 683 | CG2 | ILE | A | 85 | 17.957 | 20.022 | 17.853 | 1.00 | 16.84 | C |
| ATOM | 684 | CD1 | ILE | A | 85 | 19.261 | 20.117 | 15.256 | 1.00 | 19.80 | C |
| ATOM | 685 | N   | TYR | A | 86 | 18.045 | 17.880 | 20.588 | 1.00 | 15.48 | N |
| ATOM | 686 | CA  | TYR | A | 86 | 17.109 | 17.511 | 21.671 | 1.00 | 15.39 | C |
| ATOM | 687 | C   | TYR | A | 86 | 16.161 | 18.675 | 21.848 | 1.00 | 16.17 | C |
| ATOM | 688 | O   | TYR | A | 86 | 16.626 | 19.808 | 22.038 | 1.00 | 17.61 | O |
| ATOM | 689 | CB  | TYR | A | 86 | 17.845 | 17.126 | 22.985 | 1.00 | 15.18 | C |
| ATOM | 690 | CG  | TYR | A | 86 | 18.611 | 15.798 | 22.918 | 1.00 | 14.57 | C |
| ATOM | 691 | CD1 | TYR | A | 86 | 19.876 | 15.728 | 22.352 | 1.00 | 14.95 | C |
| ATOM | 692 | CD2 | TYR | A | 86 | 18.067 | 14.625 | 23.410 | 1.00 | 15.64 | C |
| ATOM | 693 | CE1 | TYR | A | 86 | 20.561 | 14.557 | 22.246 | 1.00 | 14.41 | C |
| ATOM | 694 | CE2 | TYR | A | 86 | 18.780 | 13.415 | 23.339 | 1.00 | 15.16 | C |
| ATOM | 695 | CZ  | TYR | A | 86 | 20.026 | 13.386 | 22.744 | 1.00 | 16.71 | C |
| ATOM | 696 | OH  | TYR | A | 86 | 20.720 | 12.203 | 22.623 | 1.00 | 15.78 | O |
| ATOM | 697 | N   | TYR | A | 87 | 14.853 | 18.389 | 21.759 | 1.00 | 14.54 | N |
| ATOM | 698 | CA  | TYR | A | 87 | 13.795 | 19.400 | 21.857 | 1.00 | 14.98 | C |
| ATOM | 699 | C   | TYR | A | 87 | 12.940 | 19.129 | 23.074 | 1.00 | 13.89 | C |
| ATOM | 700 | O   | TYR | A | 87 | 12.529 | 17.988 | 23.340 | 1.00 | 12.74 | O |
| ATOM | 701 | CB  | TYR | A | 87 | 12.840 | 19.345 | 20.665 | 1.00 | 14.37 | C |
| ATOM | 702 | CG  | TYR | A | 87 | 13.464 | 19.625 | 19.327 | 1.00 | 14.27 | C |
| ATOM | 703 | CD1 | TYR | A | 87 | 13.562 | 20.928 | 18.838 | 1.00 | 13.34 | C |

| ATOM | 704 | CD2 | TYR A | 87 | 13.931 | 18.603 | 18.531 | 1.00 | 13.94 | C |
|------|-----|-----|-------|----|--------|--------|--------|------|-------|---|
| ATOM | 705 | CE1 | TYR A | 87 | 14.163 | 21.178 | 17.595 | 1.00 | 13.09 | C |
| ATOM | 706 | CE2 | TYR A | 87 | 14.480 | 18.840 | 17.300 | 1.00 | 14.47 | C |
| ATOM | 707 | CZ | TYR A | 87 | 14.601 | 20.141 | 16.829 | 1.00 | 13.76 | C |
| ATOM | 708 | OH | TYR A | 87 | 15.148 | 20.347 | 15.565 | 1.00 | 15.94 | O |
| ATOM | 709 | N | CYS A | 88 | 12.613 | 20.194 | 23.781 | 1.00 | 12.53 | N |
| ATOM | 710 | CA | CYS A | 88 | 11.642 | 20.115 | 24.855 | 1.00 | 14.48 | C |
| ATOM | 711 | C | CYS A | 88 | 10.326 | 20.662 | 24.294 | 1.00 | 15.89 | C |
| ATOM | 712 | O | CYS A | 88 | 10.308 | 21.465 | 23.340 | 1.00 | 13.75 | O |
| ATOM | 713 | CB | CYS A | 88 | 12.128 | 20.861 | 26.100 | 1.00 | 16.38 | C |
| ATOM | 714 | SG | CYS A | 88 | 12.489 | 22.599 | 25.847 | 1.00 | 21.63 | S |
| ATOM | 715 | N | ALA A | 89 | 9.236 | 20.189 | 24.896 | 1.00 | 14.58 | N |
| ATOM | 716 | CA | ALA A | 89 | 7.879 | 20.524 | 24.509 | 1.00 | 13.83 | C |
| ATOM | 717 | C | ALA A | 89 | 6.981 | 20.360 | 25.718 | 1.00 | 12.50 | C |
| ATOM | 718 | O | ALA A | 89 | 7.259 | 19.513 | 26.567 | 1.00 | 15.46 | O |
| ATOM | 719 | CB | ALA A | 89 | 7.385 | 19.589 | 23.402 | 1.00 | 13.27 | C |
| ATOM | 720 | N | HIS A | 90 | 5.901 | 21.130 | 25.746 | 1.00 | 11.38 | N |
| ATOM | 721 | CA | HIS A | 90 | 4.885 | 21.069 | 26.769 | 1.00 | 11.70 | C |
| ATOM | 722 | C | HIS A | 90 | 3.666 | 20.410 | 26.153 | 1.00 | 12.15 | C |
| ATOM | 723 | O | HIS A | 90 | 3.490 | 20.423 | 24.919 | 1.00 | 11.96 | O |
| ATOM | 724 | CB | HIS A | 90 | 4.511 | 22.499 | 27.243 | 1.00 | 11.90 | C |
| ATOM | 725 | CG | HIS A | 90 | 3.595 | 23.236 | 26.311 | 1.00 | 13.02 | C |
| ATOM | 726 | ND1 | HIS A | 90 | 4.048 | 24.085 | 25.327 | 1.00 | 16.36 | N |
| ATOM | 727 | CD2 | HIS A | 90 | 2.252 | 23.202 | 26.180 | 1.00 | 13.35 | C |
| ATOM | 728 | CE1 | HIS A | 90 | 3.016 | 24.553 | 24.647 | 1.00 | 13.48 | C |
| ATOM | 729 | NE2 | HIS A | 90 | 1.915 | 24.025 | 25.144 | 1.00 | 12.63 | N |
| ATOM | 730 | N | ASN A | 91 | 2.773 | 19.939 | 27.018 | 1.00 | 13.30 | N |
| ATOM | 731 | CA | ASN A | 91 | 1.443 | 19.501 | 26.656 | 1.00 | 14.11 | C |
| ATOM | 732 | C | ASN A | 91 | 0.410 | 19.973 | 27.710 | 1.00 | 14.59 | C |
| ATOM | 733 | O | ASN A | 91 | -0.218 | 19.172 | 28.367 | 1.00 | 13.61 | O |
| ATOM | 734 | CB | ASN A | 91 | 1.490 | 17.970 | 26.575 | 1.00 | 13.54 | C |
| ATOM | 735 | CG | ASN A | 91 | 0.153 | 17.322 | 26.297 | 1.00 | 13.17 | C |
| ATOM | 736 | OD1 | ASN A | 91 | -0.062 | 16.147 | 26.666 | 1.00 | 15.84 | O |
| ATOM | 737 | ND2 | ASN A | 91 | -0.700 | 18.006 | 25.618 | 1.00 | 10.14 | N |
| ATOM | 738 | N | VAL A | 92 | 0.224 | 21.284 | 27.861 | 1.00 | 14.95 | N |
| ATOM | 739 | CA | VAL A | 92 | -0.772 | 21.848 | 28.784 | 1.00 | 16.70 | C |
| ATOM | 740 | C | VAL A | 92 | -1.806 | 22.778 | 28.099 | 1.00 | 17.49 | C |
| ATOM | 741 | O | VAL A | 92 | -2.805 | 23.139 | 28.687 | 1.00 | 18.03 | O |
| ATOM | 742 | CB | VAL A | 92 | -0.122 | 22.618 | 29.959 | 1.00 | 18.12 | C |
| ATOM | 743 | CG1 | VAL A | 92 | 0.806 | 21.714 | 30.749 | 1.00 | 16.38 | C |
| ATOM | 744 | CG2 | VAL A | 92 | 0.611 | 23.870 | 29.500 | 1.00 | 17.48 | C |
| ATOM | 745 | N | GLU A | 93 | -1.574 | 23.113 | 26.846 | 1.00 | 18.68 | N |
| ATOM | 746 | CA | GLU A | 93 | -2.489 | 23.937 | 26.101 | 1.00 | 19.42 | C |
| ATOM | 747 | C | GLU A | 93 | -2.159 | 23.877 | 24.628 | 1.00 | 19.86 | C |
| ATOM | 748 | O | GLU A | 93 | -1.169 | 23.258 | 24.234 | 1.00 | 16.56 | O |
| ATOM | 749 | CB | GLU A | 93 | -2.382 | 25.382 | 26.577 | 1.00 | 20.81 | C |
| ATOM | 750 | CG | GLU A | 93 | -1.090 | 26.068 | 26.180 | 1.00 | 22.07 | C |
| ATOM | 751 | CD | GLU A | 93 | -1.047 | 27.527 | 26.585 | 1.00 | 22.02 | C |
| ATOM | 752 | OE1 | GLU A | 93 | -1.682 | 27.886 | 27.610 | 1.00 | 27.19 | O |
| ATOM | 753 | OE2 | GLU A | 93 | -0.387 | 28.315 | 25.886 | 1.00 | 25.14 | O |
| ATOM | 754 | N | LEU A | 94 | -3.004 | 24.535 | 23.835 | 1.00 | 19.24 | N |
| ATOM | 755 | CA | LEU A | 94 | -2.649 | 24.936 | 22.486 | 1.00 | 19.06 | C |
| ATOM | 756 | C | LEU A | 94 | -2.406 | 26.422 | 22.524 | 1.00 | 19.50 | C |
| ATOM | 757 | O | LEU A | 94 | -3.005 | 27.101 | 23.350 | 1.00 | 20.76 | O |
| ATOM | 758 | CB | LEU A | 94 | -3.770 | 24.638 | 21.490 | 1.00 | 19.01 | C |
| ATOM | 759 | CG | LEU A | 94 | -4.451 | 23.285 | 21.564 | 1.00 | 18.60 | C |
| ATOM | 760 | CD1 | LEU A | 94 | -5.583 | 23.295 | 20.548 | 1.00 | 20.14 | C |
| ATOM | 761 | CD2 | LEU A | 94 | -3.495 | 22.113 | 21.349 | 1.00 | 20.16 | C |
| ATOM | 762 | N | PRO A | 95 | -1.533 | 26.927 | 21.639 | 1.00 | 18.59 | N |
| ATOM | 763 | CA | PRO A | 95 | -0.812 | 26.086 | 20.682 | 1.00 | 18.08 | C |
| ATOM | 764 | C | PRO A | 95 | 0.279 | 25.245 | 21.301 | 1.00 | 18.14 | C |
| ATOM | 765 | O | PRO A | 95 | 0.852 | 25.603 | 22.326 | 1.00 | 16.95 | O |
| ATOM | 766 | CB | PRO A | 95 | -0.188 | 27.101 | 19.735 | 1.00 | 18.63 | C |
| ATOM | 767 | CG | PRO A | 95 | -0.004 | 28.307 | 20.588 | 1.00 | 18.10 | C |
| ATOM | 768 | CD | PRO A | 95 | -1.201 | 28.350 | 21.463 | 1.00 | 17.96 | C |

```
ATOM    769  N    ARG A   96     0.553  24.112  20.665  1.00 16.05           N
ATOM    770  CA   ARG A   96     1.672  23.315  21.052  1.00 14.88           C
ATOM    771  C    ARG A   96     2.957  24.035  20.622  1.00 15.63           C
ATOM    772  O    ARG A   96     3.082  24.491  19.477  1.00 17.17           O
ATOM    773  CB   ARG A   96     1.568  21.982  20.393  1.00 14.79           C
ATOM    774  CG   ARG A   96     0.219  21.357  20.584  1.00 12.07           C
ATOM    775  CD   ARG A   96     0.326  19.859  20.355  1.00 12.86           C
ATOM    776  NE   ARG A   96    -0.975  19.280  20.582  1.00 11.95           N
ATOM    777  CZ   ARG A   96    -1.454  18.963  21.773  1.00 11.97           C
ATOM    778  NH1  ARG A   96    -0.740  19.128  22.866  1.00 13.21           N
ATOM    779  NH2  ARG A   96    -2.629  18.415  21.870  1.00 16.19           N
ATOM    780  N    THR A   97     3.907  24.147  21.537  1.00 13.99           N
ATOM    781  CA   THR A   97     5.187  24.754  21.216  1.00 13.73           C
ATOM    782  C    THR A   97     6.347  23.871  21.686  1.00 13.23           C
ATOM    783  O    THR A   97     6.232  23.055  22.636  1.00 14.04           O
ATOM    784  CB   THR A   97     5.337  26.256  21.757  1.00 14.00           C
ATOM    785  OG1  THR A   97     5.133  26.312  23.164  1.00 14.12           O
ATOM    786  CG2  THR A   97     4.323  27.117  21.131  1.00 14.93           C
ATOM    787  N    PHE A   98     7.431  24.039  20.956  1.00 13.83           N
ATOM    788  CA   PHE A   98     8.696  23.324  21.128  1.00 12.91           C
ATOM    789  C    PHE A   98     9.753  24.336  21.492  1.00 13.73           C
ATOM    790  O    PHE A   98     9.647  25.544  21.119  1.00 15.01           O
ATOM    791  CB   PHE A   98     9.057  22.593  19.831  1.00 13.12           C
ATOM    792  CG   PHE A   98     8.007  21.602  19.403  1.00 10.73           C
ATOM    793  CD1  PHE A   98     6.921  21.995  18.633  1.00 11.45           C
ATOM    794  CD2  PHE A   98     8.099  20.269  19.793  1.00 12.77           C
ATOM    795  CE1  PHE A   98     5.929  21.110  18.297  1.00 12.63           C
ATOM    796  CE2  PHE A   98     7.138  19.402  19.456  1.00 11.18           C
ATOM    797  CZ   PHE A   98     6.066  19.787  18.692  1.00 13.37           C
ATOM    798  N    GLY A   99    10.768  23.889  22.225  1.00 12.42           N
ATOM    799  CA   GLY A   99    12.019  24.627  22.340  1.00 12.73           C
ATOM    800  C    GLY A   99    12.749  24.648  21.000  1.00 12.10           C
ATOM    801  O    GLY A   99    12.403  23.895  20.076  1.00 12.86           O
ATOM    802  N    GLY A  100    13.757  25.515  20.877  1.00 11.20           N
ATOM    803  CA   GLY A  100    14.574  25.583  19.667  1.00 11.82           C
ATOM    804  C    GLY A  100    15.483  24.408  19.327  1.00 13.67           C
ATOM    805  O    GLY A  100    16.061  24.353  18.242  1.00 14.76           O
ATOM    806  N    GLY A  101    15.636  23.474  20.253  1.00 12.51           N
ATOM    807  CA   GLY A  101    16.565  22.387  20.060  1.00 14.99           C
ATOM    808  C    GLY A  101    17.935  22.675  20.624  1.00 14.79           C
ATOM    809  O    GLY A  101    18.479  23.795  20.526  1.00 15.37           O
ATOM    810  N    THR A  102    18.519  21.663  21.246  1.00 16.38           N
ATOM    811  CA   THR A  102    19.886  21.755  21.760  1.00 16.23           C
ATOM    812  C    THR A  102    20.616  20.627  21.066  1.00 17.21           C
ATOM    813  O    THR A  102    20.209  19.463  21.183  1.00 15.10           O
ATOM    814  CB   THR A  102    19.981  21.548  23.316  1.00 16.81           C
ATOM    815  OG1  THR A  102    19.309  22.626  24.016  1.00 12.15           O
ATOM    816  CG2  THR A  102    21.419  21.507  23.770  1.00 16.70           C
ATOM    817  N    LYS A  103    21.673  20.985  20.353  1.00 18.57           N
ATOM    818  CA   LYS A  103    22.509  20.039  19.638  1.00 19.84           C
ATOM    819  C    LYS A  103    23.545  19.486  20.583  1.00 20.19           C
ATOM    820  O    LYS A  103    24.275  20.243  21.239  1.00 21.45           O
ATOM    821  CB   LYS A  103    23.190  20.723  18.448  1.00 20.02           C
ATOM    822  CG   LYS A  103    24.001  19.810  17.579  1.00 21.29           C
ATOM    823  CD   LYS A  103    24.547  20.529  16.338  1.00 23.42           C
ATOM    824  CE   LYS A  103    23.547  20.548  15.169  1.00 26.93           C
ATOM    825  NZ   LYS A  103    24.047  21.227  13.919  1.00 26.50           N
ATOM    826  N    LEU A  104    23.564  18.167  20.693  1.00 18.96           N
ATOM    827  CA   LEU A  104    24.590  17.467  21.428  1.00 21.75           C
ATOM    828  C    LEU A  104    25.800  17.237  20.535  1.00 22.59           C
ATOM    829  O    LEU A  104    25.687  16.589  19.486  1.00 23.79           O
ATOM    830  CB   LEU A  104    24.076  16.104  21.863  1.00 20.76           C
ATOM    831  CG   LEU A  104    25.053  15.297  22.711  1.00 21.05           C
ATOM    832  CD1  LEU A  104    25.164  15.915  24.097  1.00 24.01           C
ATOM    833  CD2  LEU A  104    24.597  13.873  22.801  1.00 21.68           C
```

```
ATOM    834  N   GLU A 105      26.947  17.757  20.945  1.00 25.02           N
ATOM    835  CA  GLU A 105      28.177  17.426  20.257  1.00 26.70           C
ATOM    836  C   GLU A 105      28.978  16.476  21.119  1.00 27.17           C
ATOM    837  O   GLU A 105      29.047  16.633  22.324  1.00 27.48           O
ATOM    838  CB  GLU A 105      29.006  18.659  19.899  1.00 27.51           C
ATOM    839  CG  GLU A 105      29.841  18.409  18.632  1.00 28.61           C
ATOM    840  CD  GLU A 105      31.197  19.085  18.618  1.00 31.51           C
ATOM    841  OE1 GLU A 105      31.242  20.296  18.956  1.00 36.17           O
ATOM    842  OE2 GLU A 105      32.206  18.410  18.232  1.00 31.35           O
ATOM    843  N   ILE A 106      29.537  15.470  20.467  1.00 28.08           N
ATOM    844  CA  ILE A 106      30.447  14.546  21.076  1.00 28.20           C
ATOM    845  C   ILE A 106      31.881  14.992  20.833  1.00 28.31           C
ATOM    846  O   ILE A 106      32.223  15.451  19.744  1.00 27.89           O
ATOM    847  CB  ILE A 106      30.239  13.156  20.493  1.00 28.67           C
ATOM    848  CG1 ILE A 106      28.833  12.662  20.879  1.00 29.24           C
ATOM    849  CG2 ILE A 106      31.358  12.221  20.895  1.00 29.53           C
ATOM    850  CD1 ILE A 106      28.751  11.235  21.325  1.00 30.00           C
ATOM    851  N   LYS A 107      32.707  14.855  21.869  1.00 28.70           N
ATOM    852  CA  LYS A 107      34.141  15.053  21.779  1.00 28.76           C
ATOM    853  C   LYS A 107      34.781  13.685  21.656  1.00 28.14           C
ATOM    854  O   LYS A 107      34.440  12.778  22.396  1.00 26.84           O
ATOM    855  CB  LYS A 107      34.661  15.762  23.019  1.00 30.14           C
ATOM    856  CG  LYS A 107      36.129  16.174  22.940  1.00 31.32           C
ATOM    857  CD  LYS A 107      36.587  16.799  24.242  1.00 32.03           C
ATOM    858  CE  LYS A 107      37.861  17.608  24.045  1.00 33.31           C
ATOM    859  NZ  LYS A 107      38.386  18.124  25.362  1.00 35.03           N
ATOM    860  N   ARG A 108      35.677  13.546  20.681  1.00 27.89           N
ATOM    861  CA  ARG A 108      36.449  12.327  20.470  1.00 25.93           C
ATOM    862  C   ARG A 108      37.891  12.704  20.145  1.00 25.27           C
ATOM    863  O   ARG A 108      38.245  13.885  20.113  1.00 26.50           O
ATOM    864  CB  ARG A 108      35.826  11.499  19.347  1.00 25.29           C
ATOM    865  CG  ARG A 108      35.628  12.276  18.039  1.00 23.80           C
ATOM    866  CD  ARG A 108      35.769  11.372  16.823  1.00 25.05           C
ATOM    867  NE  ARG A 108      37.114  10.831  16.691  1.00 22.91           N
ATOM    868  CZ  ARG A 108      37.432   9.679  16.087  1.00 25.57           C
ATOM    869  NH1 ARG A 108      36.522   8.913  15.540  1.00 25.67           N
ATOM    870  NH2 ARG A 108      38.700   9.285  16.036  1.00 26.26           N
ATOM    871  N   ALA A 109      38.727  11.698  19.923  1.00 25.69           N
ATOM    872  CA  ALA A 109      40.123  11.913  19.555  1.00 25.32           C
ATOM    873  C   ALA A 109      40.199  12.587  18.204  1.00 25.06           C
ATOM    874  O   ALA A 109      39.412  12.275  17.302  1.00 26.55           O
ATOM    875  CB  ALA A 109      40.861  10.580  19.485  1.00 26.17           C
ATOM    876  N   ASP A 110      41.151  13.503  18.072  1.00 24.09           N
ATOM    877  CA  ASP A 110      41.405  14.171  16.817  1.00 23.80           C
ATOM    878  C   ASP A 110      41.665  13.097  15.767  1.00 23.27           C
ATOM    879  O   ASP A 110      42.233  12.048  16.083  1.00 21.66           O
ATOM    880  CB  ASP A 110      42.608  15.099  16.925  1.00 24.94           C
ATOM    881  CG  ASP A 110      42.425  16.220  17.932  1.00 26.10           C
ATOM    882  OD1 ASP A 110      41.312  16.399  18.491  1.00 25.51           O
ATOM    883  OD2 ASP A 110      43.430  16.941  18.158  1.00 28.87           O
ATOM    884  N   ALA A 111      41.200  13.336  14.540  1.00 21.34           N
ATOM    885  CA  ALA A 111      41.367  12.397  13.435  1.00 20.38           C
ATOM    886  C   ALA A 111      41.624  13.252  12.222  1.00 20.48           C
ATOM    887  O   ALA A 111      40.905  14.240  12.008  1.00 19.63           O
ATOM    888  CB  ALA A 111      40.111  11.563  13.252  1.00 18.51           C
ATOM    889  N   ALA A 112      42.654  12.897  11.450  1.00 18.27           N
ATOM    890  CA  ALA A 112      42.968  13.600  10.234  1.00 17.90           C
ATOM    891  C   ALA A 112      41.962  13.210   9.148  1.00 16.50           C
ATOM    892  O   ALA A 112      41.462  12.086   9.123  1.00 15.98           O
ATOM    893  CB  ALA A 112      44.393  13.315   9.773  1.00 18.43           C
ATOM    894  N   PRO A 113      41.647  14.145   8.260  1.00 16.40           N
ATOM    895  CA  PRO A 113      40.795  13.799   7.151  1.00 17.13           C
ATOM    896  C   PRO A 113      41.458  12.893   6.143  1.00 17.69           C
ATOM    897  O   PRO A 113      42.684  12.958   5.948  1.00 18.43           O
ATOM    898  CB  PRO A 113      40.552  15.150   6.493  1.00 17.65           C
```

```
ATOM   899  CG   PRO A 113      41.753  15.861   6.773  1.00 17.67           C
ATOM   900  CD   PRO A 113      42.009  15.563   8.191  1.00 16.60           C
ATOM   901  N    THR A 114      40.628  12.054   5.525  1.00 17.94           N
ATOM   902  CA   THR A 114      40.984  11.306   4.367  1.00 18.77           C
ATOM   903  C    THR A 114      40.534  12.095   3.178  1.00 17.94           C
ATOM   904  O    THR A 114      39.353  12.413   3.061  1.00 17.76           O
ATOM   905  CB   THR A 114      40.326   9.943   4.396  1.00 18.83           C
ATOM   906  OG1  THR A 114      40.787   9.264   5.564  1.00 21.76           O
ATOM   907  CG2  THR A 114      40.685   9.148   3.163  1.00 19.75           C
ATOM   908  N    VAL A 115      41.493  12.504   2.353  1.00 19.14           N
ATOM   909  CA   VAL A 115      41.232  13.404   1.221  1.00 17.98           C
ATOM   910  C    VAL A 115      41.241  12.643  -0.122  1.00 17.83           C
ATOM   911  O    VAL A 115      42.141  11.859  -0.411  1.00 16.67           O
ATOM   912  CB   VAL A 115      42.223  14.586   1.201  1.00 17.70           C
ATOM   913  CG1  VAL A 115      41.809  15.635   0.184  1.00 18.62           C
ATOM   914  CG2  VAL A 115      42.310  15.227   2.585  1.00 15.38           C
ATOM   915  N    SER A 116      40.222  12.894  -0.942  1.00 17.26           N
ATOM   916  CA   SER A 116      40.109  12.304  -2.286  1.00 17.82           C
ATOM   917  C    SER A 116      39.735  13.391  -3.279  1.00 17.10           C
ATOM   918  O    SER A 116      38.848  14.123  -3.007  1.00 15.56           O
ATOM   919  CB   SER A 116      38.999  11.254  -2.292  1.00 18.55           C
ATOM   920  OG   SER A 116      39.222  10.263  -1.310  1.00 21.05           O
ATOM   921  N    ILE A 117      40.388  13.458  -4.435  1.00 15.96           N
ATOM   922  CA   ILE A 117      40.027  14.388  -5.486  1.00 17.02           C
ATOM   923  C    ILE A 117      39.549  13.614  -6.701  1.00 17.41           C
ATOM   924  O    ILE A 117      40.078  12.530  -7.009  1.00 15.85           O
ATOM   925  CB   ILE A 117      41.229  15.293  -5.859  1.00 16.78           C
ATOM   926  CG1  ILE A 117      40.772  16.551  -6.626  1.00 16.54           C
ATOM   927  CG2  ILE A 117      42.254  14.507  -6.599  1.00 18.92           C
ATOM   928  CD1  ILE A 117      41.862  17.585  -6.756  1.00 17.61           C
ATOM   929  N    PHE A 118      38.573  14.187  -7.394  1.00 15.79           N
ATOM   930  CA   PHE A 118      37.927  13.554  -8.556  1.00 17.33           C
ATOM   931  C    PHE A 118      37.888  14.509  -9.734  1.00 17.55           C
ATOM   932  O    PHE A 118      37.342  15.592  -9.602  1.00 16.16           O
ATOM   933  CB   PHE A 118      36.517  13.178  -8.194  1.00 18.31           C
ATOM   934  CG   PHE A 118      36.427  12.229  -7.054  1.00 16.02           C
ATOM   935  CD1  PHE A 118      36.239  12.683  -5.760  1.00 18.68           C
ATOM   936  CD2  PHE A 118      36.544  10.870  -7.278  1.00 18.81           C
ATOM   937  CE1  PHE A 118      36.137  11.801  -4.716  1.00 18.71           C
ATOM   938  CE2  PHE A 118      36.465   9.991  -6.244  1.00 18.44           C
ATOM   939  CZ   PHE A 118      36.276  10.446  -4.960  1.00 19.65           C
ATOM   940  N    PRO A 119      38.476  14.123 -10.876  1.00 18.23           N
ATOM   941  CA   PRO A 119      38.343  14.912 -12.089  1.00 19.38           C
ATOM   942  C    PRO A 119      36.914  14.878 -12.648  1.00 19.56           C
ATOM   943  O    PRO A 119      36.130  13.982 -12.296  1.00 19.11           O
ATOM   944  CB   PRO A 119      39.306  14.246 -13.073  1.00 18.50           C
ATOM   945  CG   PRO A 119      39.557  12.920 -12.533  1.00 19.76           C
ATOM   946  CD   PRO A 119      39.323  12.928 -11.080  1.00 18.73           C
ATOM   947  N    PRO A 120      36.552  15.886 -13.456  1.00 20.05           N
ATOM   948  CA   PRO A 120      35.249  15.820 -14.101  1.00 20.24           C
ATOM   949  C    PRO A 120      35.052  14.543 -14.935  1.00 20.92           C
ATOM   950  O    PRO A 120      35.946  14.165 -15.700  1.00 21.65           O
ATOM   951  CB   PRO A 120      35.268  17.023 -15.039  1.00 20.22           C
ATOM   952  CG   PRO A 120      36.256  17.957 -14.469  1.00 20.37           C
ATOM   953  CD   PRO A 120      37.291  17.110 -13.828  1.00 19.90           C
ATOM   954  N    SER A 121      33.873  13.944 -14.828  1.00 21.06           N
ATOM   955  CA   SER A 121      33.477  12.825 -15.686  1.00 22.00           C
ATOM   956  C    SER A 121      33.493  13.196 -17.172  1.00 22.77           C
ATOM   957  O    SER A 121      33.317  14.360 -17.555  1.00 20.83           O
ATOM   958  CB   SER A 121      32.077  12.309 -15.304  1.00 22.40           C
ATOM   959  OG   SER A 121      31.099  13.342 -15.274  1.00 22.37           O
ATOM   960  N    SER A 122      33.742  12.204 -18.009  1.00 23.34           N
ATOM   961  CA   SER A 122      33.510  12.335 -19.429  1.00 24.19           C
ATOM   962  C    SER A 122      32.080  12.800 -19.699  1.00 24.18           C
ATOM   963  O    SER A 122      31.862  13.631 -20.582  1.00 23.61           O
```

```
ATOM    964  CB  SER A 122      33.756  10.999 -20.125  1.00 26.08           C
ATOM    965  OG  SER A 122      32.972   9.996 -19.529  1.00 29.00           O
ATOM    966  N   GLU A 123      31.110  12.302 -18.930  1.00 22.59           N
ATOM    967  CA  GLU A 123      29.714  12.726 -19.111  1.00 23.27           C
ATOM    968  C   GLU A 123      29.502  14.194 -18.877  1.00 20.99           C
ATOM    969  O   GLU A 123      28.797  14.863 -19.610  1.00 19.54           O
ATOM    970  CB  GLU A 123      28.788  11.996 -18.164  1.00 25.97           C
ATOM    971  CG  GLU A 123      28.652  10.547 -18.505  1.00 28.52           C
ATOM    972  CD  GLU A 123      29.221   9.668 -17.443  1.00 32.67           C
ATOM    973  OE1 GLU A 123      30.307  10.026 -16.903  1.00 34.06           O
ATOM    974  OE2 GLU A 123      28.592   8.615 -17.172  1.00 32.93           O
ATOM    975  N   GLN A 124      30.070  14.695 -17.799  1.00 19.26           N
ATOM    976  CA  GLN A 124      29.887  16.103 -17.499  1.00 18.06           C
ATOM    977  C   GLN A 124      30.507  16.936 -18.593  1.00 16.30           C
ATOM    978  O   GLN A 124      29.939  17.961 -18.980  1.00 17.58           O
ATOM    979  CB  GLN A 124      30.481  16.492 -16.152  1.00 18.69           C
ATOM    980  CG  GLN A 124      29.930  17.823 -15.705  1.00 17.60           C
ATOM    981  CD  GLN A 124      30.505  18.326 -14.417  1.00 18.24           C
ATOM    982  OE1 GLN A 124      31.431  17.763 -13.872  1.00 15.83           O
ATOM    983  NE2 GLN A 124      29.937  19.413 -13.918  1.00 16.93           N
ATOM    984  N   LEU A 125      31.677  16.526 -19.062  1.00 15.55           N
ATOM    985  CA  LEU A 125      32.361  17.303 -20.109  1.00 16.53           C
ATOM    986  C   LEU A 125      31.534  17.322 -21.380  1.00 14.48           C
ATOM    987  O   LEU A 125      31.463  18.307 -22.022  1.00 13.33           O
ATOM    988  CB  LEU A 125      33.770  16.781 -20.361  1.00 17.56           C
ATOM    989  CG  LEU A 125      34.757  17.032 -19.212  1.00 18.04           C
ATOM    990  CD1 LEU A 125      35.998  16.194 -19.379  1.00 19.04           C
ATOM    991  CD2 LEU A 125      35.123  18.464 -19.063  1.00 20.02           C
ATOM    992  N   THR A 126      30.877  16.217 -21.734  1.00 15.07           N
ATOM    993  CA  THR A 126      29.912  16.251 -22.847  1.00 13.50           C
ATOM    994  C   THR A 126      28.807  17.343 -22.684  1.00 13.27           C
ATOM    995  O   THR A 126      28.334  17.917 -23.656  1.00 12.68           O
ATOM    996  CB  THR A 126      29.325  14.833 -23.033  1.00 15.18           C
ATOM    997  OG1 THR A 126      30.374  13.992 -23.500  1.00 12.09           O
ATOM    998  CG2 THR A 126      28.206  14.805 -24.011  1.00 14.59           C
ATOM    999  N   SER A 127      28.382  17.585 -21.451  1.00 14.09           N
ATOM   1000  CA  SER A 127      27.320  18.536 -21.157  1.00 14.71           C
ATOM   1001  C   SER A 127      27.860  19.950 -21.154  1.00 14.56           C
ATOM   1002  O   SER A 127      27.075  20.888 -21.162  1.00 16.12           O
ATOM   1003  CB  SER A 127      26.642  18.214 -19.801  1.00 14.30           C
ATOM   1004  OG  SER A 127      27.472  18.595 -18.714  1.00 17.14           O
ATOM   1005  N   GLY A 128      29.187  20.122 -21.166  1.00 15.55           N
ATOM   1006  CA  GLY A 128      29.783  21.472 -21.197  1.00 15.61           C
ATOM   1007  C   GLY A 128      30.281  21.944 -19.851  1.00 17.07           C
ATOM   1008  O   GLY A 128      30.743  23.073 -19.721  1.00 19.68           O
ATOM   1009  N   GLY A 129      30.177  21.080 -18.848  1.00 17.68           N
ATOM   1010  CA  GLY A 129      30.583  21.389 -17.487  1.00 17.28           C
ATOM   1011  C   GLY A 129      31.846  20.675 -17.062  1.00 17.62           C
ATOM   1012  O   GLY A 129      32.216  19.666 -17.647  1.00 17.62           O
ATOM   1013  N   ALA A 130      32.503  21.210 -16.029  1.00 17.67           N
ATOM   1014  CA  ALA A 130      33.663  20.572 -15.424  1.00 17.34           C
ATOM   1015  C   ALA A 130      33.734  20.926 -13.931  1.00 18.26           C
ATOM   1016  O   ALA A 130      34.248  21.978 -13.558  1.00 19.40           O
ATOM   1017  CB  ALA A 130      34.947  20.966 -16.126  1.00 16.68           C
ATOM   1018  N   SER A 131      33.148  20.081 -13.092  1.00 19.59           N
ATOM   1019  CA  SER A 131      33.202  20.277 -11.645  1.00 19.10           C
ATOM   1020  C   SER A 131      34.237  19.336 -11.085  1.00 20.15           C
ATOM   1021  O   SER A 131      34.240  18.135 -11.396  1.00 20.61           O
ATOM   1022  CB  SER A 131      31.866  20.016 -10.987  1.00 20.21           C
ATOM   1023  OG  SER A 131      30.869  20.936 -11.402  1.00 20.43           O
ATOM   1024  N   VAL A 132      35.160  19.881 -10.308  1.00 18.70           N
ATOM   1025  CA  VAL A 132      36.178  19.078  -9.660  1.00 18.17           C
ATOM   1026  C   VAL A 132      35.774  18.930  -8.208  1.00 17.06           C
ATOM   1027  O   VAL A 132      35.429  19.891  -7.560  1.00 15.60           O
ATOM   1028  CB  VAL A 132      37.580  19.723  -9.799  1.00 18.72           C
```

| | | | | | | | | | | |
|---|---|---|---|---|---|---|---|---|---|---|
| ATOM | 1029 | CG1 | VAL | A | 132 | 38.622 | 18.754 | -9.379 | 1.00 19.41 | C |
| ATOM | 1030 | CG2 | VAL | A | 132 | 37.823 | 20.154 | -11.247 | 1.00 18.64 | C |
| ATOM | 1031 | N | VAL | A | 133 | 35.762 | 17.708 | -7.693 | 1.00 16.97 | N |
| ATOM | 1032 | CA | VAL | A | 133 | 35.233 | 17.473 | -6.377 | 1.00 16.94 | C |
| ATOM | 1033 | C | VAL | A | 133 | 36.374 | 17.011 | -5.499 | 1.00 17.06 | C |
| ATOM | 1034 | O | VAL | A | 133 | 37.209 | 16.232 | -5.915 | 1.00 17.26 | O |
| ATOM | 1035 | CB | VAL | A | 133 | 34.125 | 16.389 | -6.390 | 1.00 18.09 | C |
| ATOM | 1036 | CG1 | VAL | A | 133 | 33.591 | 16.086 | -4.960 | 1.00 20.16 | C |
| ATOM | 1037 | CG2 | VAL | A | 133 | 32.984 | 16.816 | -7.318 | 1.00 18.93 | C |
| ATOM | 1038 | N | CYS | A | 134 | 36.410 | 17.517 | -4.283 | 1.00 17.39 | N |
| ATOM | 1039 | CA | CYS | A | 134 | 37.339 | 17.028 | -3.277 | 1.00 16.12 | C |
| ATOM | 1040 | C | CYS | A | 134 | 36.561 | 16.706 | -1.994 | 1.00 15.33 | C |
| ATOM | 1041 | O | CYS | A | 134 | 35.827 | 17.537 | -1.470 | 1.00 14.80 | O |
| ATOM | 1042 | CB | CYS | A | 134 | 38.418 | 18.076 | -3.070 | 1.00 17.56 | C |
| ATOM | 1043 | SG | CYS | A | 134 | 39.672 | 17.710 | -1.887 | 1.00 20.91 | S |
| ATOM | 1044 | N | PHE | A | 135 | 36.716 | 15.473 | -1.505 | 1.00 13.47 | N |
| ATOM | 1045 | CA | PHE | A | 135 | 36.111 | 15.024 | -0.250 | 1.00 14.31 | C |
| ATOM | 1046 | C | PHE | A | 135 | 37.142 | 15.012 | 0.860 | 1.00 14.84 | C |
| ATOM | 1047 | O | PHE | A | 135 | 38.209 | 14.441 | 0.719 | 1.00 16.42 | O |
| ATOM | 1048 | CB | PHE | A | 135 | 35.503 | 13.605 | -0.369 | 1.00 15.39 | C |
| ATOM | 1049 | CG | PHE | A | 135 | 34.313 | 13.500 | -1.273 | 1.00 15.56 | C |
| ATOM | 1050 | CD1 | PHE | A | 135 | 33.338 | 14.483 | -1.324 | 1.00 18.25 | C |
| ATOM | 1051 | CD2 | PHE | A | 135 | 34.145 | 12.398 | -2.076 | 1.00 19.78 | C |
| ATOM | 1052 | CE1 | PHE | A | 135 | 32.225 | 14.353 | -2.155 | 1.00 19.66 | C |
| ATOM | 1053 | CE2 | PHE | A | 135 | 33.042 | 12.289 | -2.915 | 1.00 19.95 | C |
| ATOM | 1054 | CZ | PHE | A | 135 | 32.096 | 13.242 | -2.941 | 1.00 17.93 | C |
| ATOM | 1055 | N | LEU | A | 136 | 36.809 | 15.630 | 1.980 | 1.00 16.58 | N |
| ATOM | 1056 | CA | LEU | A | 136 | 37.637 | 15.586 | 3.172 | 1.00 17.10 | C |
| ATOM | 1057 | C | LEU | A | 136 | 36.827 | 14.856 | 4.243 | 1.00 16.85 | C |
| ATOM | 1058 | O | LEU | A | 136 | 35.940 | 15.421 | 4.904 | 1.00 15.91 | O |
| ATOM | 1059 | CB | LEU | A | 136 | 38.046 | 17.001 | 3.615 | 1.00 17.92 | C |
| ATOM | 1060 | CG | LEU | A | 136 | 38.797 | 17.993 | 2.713 | 1.00 19.99 | C |
| ATOM | 1061 | CD1 | LEU | A | 136 | 37.967 | 18.590 | 1.583 | 1.00 20.98 | C |
| ATOM | 1062 | CD2 | LEU | A | 136 | 39.273 | 19.125 | 3.641 | 1.00 19.14 | C |
| ATOM | 1063 | N | ASN | A | 137 | 37.099 | 13.565 | 4.380 | 1.00 16.28 | N |
| ATOM | 1064 | CA | ASN | A | 137 | 36.218 | 12.659 | 5.107 | 1.00 16.92 | C |
| ATOM | 1065 | C | ASN | A | 137 | 36.712 | 12.151 | 6.465 | 1.00 17.08 | C |
| ATOM | 1066 | O | ASN | A | 137 | 37.898 | 11.813 | 6.630 | 1.00 15.64 | O |
| ATOM | 1067 | CB | ASN | A | 137 | 35.918 | 11.420 | 4.239 | 1.00 16.85 | C |
| ATOM | 1068 | CG | ASN | A | 137 | 34.904 | 11.703 | 3.144 | 1.00 19.19 | C |
| ATOM | 1069 | OD1 | ASN | A | 137 | 34.191 | 12.699 | 3.197 | 1.00 20.32 | O |
| ATOM | 1070 | ND2 | ASN | A | 137 | 34.835 | 10.829 | 2.150 | 1.00 17.74 | N |
| ATOM | 1071 | N | ASN | A | 138 | 35.763 | 12.104 | 7.408 | 1.00 18.36 | N |
| ATOM | 1072 | CA | ASN | A | 138 | 35.919 | 11.495 | 8.725 | 1.00 18.28 | C |
| ATOM | 1073 | C | ASN | A | 138 | 37.048 | 12.082 | 9.532 | 1.00 17.93 | C |
| ATOM | 1074 | O | ASN | A | 138 | 37.933 | 11.382 | 10.017 | 1.00 16.36 | O |
| ATOM | 1075 | CB | ASN | A | 138 | 36.096 | 9.993 | 8.598 | 1.00 19.25 | C |
| ATOM | 1076 | CG | ASN | A | 138 | 34.966 | 9.344 | 7.883 | 1.00 20.90 | C |
| ATOM | 1077 | OD1 | ASN | A | 138 | 34.924 | 9.304 | 6.657 | 1.00 22.87 | O |
| ATOM | 1078 | ND2 | ASN | A | 138 | 34.024 | 8.828 | 8.647 | 1.00 18.96 | N |
| ATOM | 1079 | N | PHE | A | 139 | 37.001 | 13.397 | 9.683 | 1.00 17.12 | N |
| ATOM | 1080 | CA | PHE | A | 139 | 37.942 | 14.106 | 10.530 | 1.00 18.72 | C |
| ATOM | 1081 | C | PHE | A | 139 | 37.309 | 14.599 | 11.839 | 1.00 18.14 | C |
| ATOM | 1082 | O | PHE | A | 139 | 36.091 | 14.671 | 11.978 | 1.00 16.24 | O |
| ATOM | 1083 | CB | PHE | A | 139 | 38.582 | 15.258 | 9.768 | 1.00 18.47 | C |
| ATOM | 1084 | CG | PHE | A | 139 | 37.598 | 16.270 | 9.228 | 1.00 19.77 | C |
| ATOM | 1085 | CD1 | PHE | A | 139 | 37.212 | 17.356 | 10.008 | 1.00 19.93 | C |
| ATOM | 1086 | CD2 | PHE | A | 139 | 37.088 | 16.155 | 7.942 | 1.00 17.10 | C |
| ATOM | 1087 | CE1 | PHE | A | 139 | 36.312 | 18.310 | 9.529 | 1.00 19.48 | C |
| ATOM | 1088 | CE2 | PHE | A | 139 | 36.196 | 17.109 | 7.446 | 1.00 18.64 | C |
| ATOM | 1089 | CZ | PHE | A | 139 | 35.812 | 18.198 | 8.235 | 1.00 20.60 | C |
| ATOM | 1090 | N | TYR | A | 140 | 38.164 | 14.888 | 12.803 | 1.00 20.22 | N |
| ATOM | 1091 | CA | TYR | A | 140 | 37.750 | 15.528 | 14.043 | 1.00 20.84 | C |
| ATOM | 1092 | C | TYR | A | 140 | 38.911 | 16.357 | 14.583 | 1.00 21.74 | C |
| ATOM | 1093 | O | TYR | A | 140 | 40.039 | 15.872 | 14.633 | 1.00 20.70 | O |

| ATOM | 1094 | CB | TYR A 140 | 37.288 | 14.493 | 15.062 | 1.00 | 22.10 | C |
|------|------|-----|-----------|--------|--------|--------|------|-------|---|
| ATOM | 1095 | CG | TYR A 140 | 36.694 | 15.167 | 16.255 | 1.00 | 21.77 | C |
| ATOM | 1096 | CD1 | TYR A 140 | 37.505 | 15.635 | 17.281 | 1.00 | 21.75 | C |
| ATOM | 1097 | CD2 | TYR A 140 | 35.335 | 15.411 | 16.325 | 1.00 | 23.27 | C |
| ATOM | 1098 | CE1 | TYR A 140 | 36.963 | 16.323 | 18.359 | 1.00 | 22.99 | C |
| ATOM | 1099 | CE2 | TYR A 140 | 34.793 | 16.077 | 17.401 | 1.00 | 23.54 | C |
| ATOM | 1100 | CZ | TYR A 140 | 35.614 | 16.530 | 18.401 | 1.00 | 22.39 | C |
| ATOM | 1101 | OH | TYR A 140 | 35.066 | 17.210 | 19.437 | 1.00 | 24.44 | O |
| ATOM | 1102 | N | PRO A 141 | 38.643 | 17.600 | 15.020 | 1.00 | 21.39 | N |
| ATOM | 1103 | CA | PRO A 141 | 37.379 | 18.324 | 15.176 | 1.00 | 21.97 | C |
| ATOM | 1104 | C | PRO A 141 | 36.829 | 18.945 | 13.890 | 1.00 | 20.92 | C |
| ATOM | 1105 | O | PRO A 141 | 37.448 | 18.852 | 12.835 | 1.00 | 17.81 | O |
| ATOM | 1106 | CB | PRO A 141 | 37.762 | 19.416 | 16.181 | 1.00 | 21.97 | C |
| ATOM | 1107 | CG | PRO A 141 | 39.163 | 19.733 | 15.791 | 1.00 | 22.90 | C |
| ATOM | 1108 | CD | PRO A 141 | 39.773 | 18.408 | 15.519 | 1.00 | 22.80 | C |
| ATOM | 1109 | N | LYS A 142 | 35.671 | 19.593 | 14.005 | 1.00 | 23.08 | N |
| ATOM | 1110 | CA | LYS A 142 | 34.912 | 20.088 | 12.861 | 1.00 | 24.23 | C |
| ATOM | 1111 | C | LYS A 142 | 35.654 | 21.072 | 11.981 | 1.00 | 24.82 | C |
| ATOM | 1112 | O | LYS A 142 | 35.425 | 21.099 | 10.774 | 1.00 | 25.12 | O |
| ATOM | 1113 | CB | LYS A 142 | 33.594 | 20.752 | 13.308 | 1.00 | 24.83 | C |
| ATOM | 1114 | CG | LYS A 142 | 32.635 | 20.884 | 12.143 | 1.00 | 25.35 | C |
| ATOM | 1115 | CD | LYS A 142 | 31.398 | 21.690 | 12.419 | 1.00 | 27.30 | C |
| ATOM | 1116 | CE | LYS A 142 | 30.749 | 22.108 | 11.091 | 1.00 | 28.09 | C |
| ATOM | 1117 | NZ | LYS A 142 | 29.263 | 22.194 | 11.205 | 1.00 | 32.25 | N |
| ATOM | 1118 | N | ASP A 143 | 36.507 | 21.904 | 12.580 | 1.00 | 24.80 | N |
| ATOM | 1119 | CA | ASP A 143 | 37.134 | 23.001 | 11.853 | 1.00 | 25.19 | C |
| ATOM | 1120 | C | ASP A 143 | 38.179 | 22.520 | 10.883 | 1.00 | 23.84 | C |
| ATOM | 1121 | O | ASP A 143 | 39.025 | 21.695 | 11.217 | 1.00 | 22.31 | O |
| ATOM | 1122 | CB | ASP A 143 | 37.778 | 24.003 | 12.802 | 1.00 | 27.67 | C |
| ATOM | 1123 | CG | ASP A 143 | 36.757 | 24.895 | 13.510 | 1.00 | 31.53 | C |
| ATOM | 1124 | OD1 | ASP A 143 | 35.590 | 24.478 | 13.678 | 1.00 | 34.98 | O |
| ATOM | 1125 | OD2 | ASP A 143 | 37.134 | 26.031 | 13.909 | 1.00 | 37.08 | O |
| ATOM | 1126 | N | ILE A 144 | 38.134 | 23.070 | 9.677 | 1.00 | 23.57 | N |
| ATOM | 1127 | CA | ILE A 144 | 39.008 | 22.636 | 8.601 | 1.00 | 23.43 | C |
| ATOM | 1128 | C | ILE A 144 | 39.022 | 23.658 | 7.474 | 1.00 | 23.33 | C |
| ATOM | 1129 | O | ILE A 144 | 38.061 | 24.408 | 7.301 | 1.00 | 22.16 | O |
| ATOM | 1130 | CB | ILE A 144 | 38.581 | 21.243 | 8.069 | 1.00 | 23.55 | C |
| ATOM | 1131 | CG1 | ILE A 144 | 39.752 | 20.579 | 7.335 | 1.00 | 23.13 | C |
| ATOM | 1132 | CG2 | ILE A 144 | 37.310 | 21.350 | 7.183 | 1.00 | 23.43 | C |
| ATOM | 1133 | CD1 | ILE A 144 | 39.590 | 19.097 | 7.143 | 1.00 | 22.75 | C |
| ATOM | 1134 | N | ASN A 145 | 40.118 | 23.680 | 6.726 | 1.00 | 23.07 | N |
| ATOM | 1135 | CA | ASN A 145 | 40.287 | 24.589 | 5.606 | 1.00 | 24.77 | C |
| ATOM | 1136 | C | ASN A 145 | 40.667 | 23.819 | 4.364 | 1.00 | 23.95 | C |
| ATOM | 1137 | O | ASN A 145 | 41.453 | 22.856 | 4.443 | 1.00 | 22.53 | O |
| ATOM | 1138 | CB | ASN A 145 | 41.386 | 25.599 | 5.921 | 1.00 | 26.80 | C |
| ATOM | 1139 | CG | ASN A 145 | 40.967 | 26.622 | 6.958 | 1.00 | 30.89 | C |
| ATOM | 1140 | OD1 | ASN A 145 | 40.045 | 27.427 | 6.722 | 1.00 | 36.47 | O |
| ATOM | 1141 | ND2 | ASN A 145 | 41.654 | 26.626 | 8.107 | 1.00 | 32.13 | N |
| ATOM | 1142 | N | VAL A 146 | 40.115 | 24.226 | 3.222 | 1.00 | 23.05 | N |
| ATOM | 1143 | CA | VAL A 146 | 40.495 | 23.651 | 1.934 | 1.00 | 23.13 | C |
| ATOM | 1144 | C | VAL A 146 | 40.971 | 24.783 | 1.031 | 1.00 | 22.66 | C |
| ATOM | 1145 | O | VAL A 146 | 40.413 | 25.870 | 1.044 | 1.00 | 22.25 | O |
| ATOM | 1146 | CB | VAL A 146 | 39.337 | 22.826 | 1.257 | 1.00 | 23.17 | C |
| ATOM | 1147 | CG1 | VAL A 146 | 38.162 | 23.715 | 0.908 | 1.00 | 24.29 | C |
| ATOM | 1148 | CG2 | VAL A 146 | 39.828 | 22.109 | 0.000 | 1.00 | 22.92 | C |
| ATOM | 1149 | N | LYS A 147 | 42.036 | 24.519 | 0.302 | 1.00 | 23.67 | N |
| ATOM | 1150 | CA | LYS A 147 | 42.595 | 25.440 | -0.685 | 1.00 | 24.03 | C |
| ATOM | 1151 | C | LYS A 147 | 42.710 | 24.712 | -2.010 | 1.00 | 23.77 | C |
| ATOM | 1152 | O | LYS A 147 | 43.268 | 23.617 | -2.066 | 1.00 | 25.74 | O |
| ATOM | 1153 | CB | LYS A 147 | 43.976 | 25.882 | -0.244 | 1.00 | 24.80 | C |
| ATOM | 1154 | CG | LYS A 147 | 44.698 | 26.795 | -1.210 | 1.00 | 26.70 | C |
| ATOM | 1155 | CD | LYS A 147 | 45.919 | 27.480 | -0.559 | 1.00 | 27.06 | C |
| ATOM | 1156 | CE | LYS A 147 | 45.548 | 28.157 | 0.781 | 1.00 | 30.93 | C |
| ATOM | 1157 | NZ | LYS A 147 | 46.506 | 29.236 | 1.266 | 1.00 | 31.31 | N |
| ATOM | 1158 | N | TRP A 148 | 42.149 | 25.304 | -3.062 | 1.00 | 22.98 | N |

| ATOM | 1159 | CA  | TRP A 148 | 42.298 | 24.795 | -4.428  | 1.00 | 22.60 | C |
| ATOM | 1160 | C   | TRP A 148 | 43.410 | 25.518 | -5.167  | 1.00 | 23.86 | C |
| ATOM | 1161 | O   | TRP A 148 | 43.513 | 26.756 | -5.096  | 1.00 | 24.02 | O |
| ATOM | 1162 | CB  | TRP A 148 | 41.033 | 25.035 | -5.232  | 1.00 | 20.95 | C |
| ATOM | 1163 | CG  | TRP A 148 | 39.982 | 24.158 | -4.856  | 1.00 | 19.35 | C |
| ATOM | 1164 | CD1 | TRP A 148 | 39.053 | 24.377 | -3.930  | 1.00 | 19.56 | C |
| ATOM | 1165 | CD2 | TRP A 148 | 39.717 | 22.879 | -5.421  | 1.00 | 18.48 | C |
| ATOM | 1166 | NE1 | TRP A 148 | 38.212 | 23.297 | -3.843  | 1.00 | 18.95 | N |
| ATOM | 1167 | CE2 | TRP A 148 | 38.606 | 22.361 | -4.754  | 1.00 | 19.00 | C |
| ATOM | 1168 | CE3 | TRP A 148 | 40.338 | 22.107 | -6.406  | 1.00 | 18.48 | C |
| ATOM | 1169 | CZ2 | TRP A 148 | 38.078 | 21.099 | -5.043  | 1.00 | 19.32 | C |
| ATOM | 1170 | CZ3 | TRP A 148 | 39.823 | 20.857 | -6.694  | 1.00 | 19.14 | C |
| ATOM | 1171 | CH2 | TRP A 148 | 38.695 | 20.370 | -6.029  | 1.00 | 20.26 | C |
| ATOM | 1172 | N   | LYS A 149 | 44.211 | 24.753 | -5.886  | 1.00 | 24.21 | N |
| ATOM | 1173 | CA  | LYS A 149 | 45.164 | 25.326 | -6.818  | 1.00 | 26.07 | C |
| ATOM | 1174 | C   | LYS A 149 | 44.978 | 24.801 | -8.231  | 1.00 | 26.44 | C |
| ATOM | 1175 | O   | LYS A 149 | 44.795 | 23.599 | -8.436  | 1.00 | 26.59 | O |
| ATOM | 1176 | CB  | LYS A 149 | 46.586 | 25.042 | -6.363  | 1.00 | 26.69 | C |
| ATOM | 1177 | CG  | LYS A 149 | 46.961 | 25.825 | -5.150  | 1.00 | 27.65 | C |
| ATOM | 1178 | CD  | LYS A 149 | 48.305 | 25.434 | -4.644  | 1.00 | 27.53 | C |
| ATOM | 1179 | CE  | LYS A 149 | 48.642 | 26.238 | -3.419  | 1.00 | 28.55 | C |
| ATOM | 1180 | NZ  | LYS A 149 | 49.888 | 25.705 | -2.784  | 1.00 | 30.49 | N |
| ATOM | 1181 | N   | ILE A 150 | 45.041 | 25.718 | -9.193  | 1.00 | 26.55 | N |
| ATOM | 1182 | CA  | ILE A 150 | 45.082 | 25.379 | -10.607 | 1.00 | 27.83 | C |
| ATOM | 1183 | C   | ILE A 150 | 46.461 | 25.780 | -11.153 | 1.00 | 28.60 | C |
| ATOM | 1184 | O   | ILE A 150 | 46.845 | 26.939 | -11.080 | 1.00 | 28.82 | O |
| ATOM | 1185 | CB  | ILE A 150 | 43.979 | 26.099 | -11.392 | 1.00 | 27.32 | C |
| ATOM | 1186 | CG1 | ILE A 150 | 42.594 | 25.715 | -10.866 | 1.00 | 27.36 | C |
| ATOM | 1187 | CG2 | ILE A 150 | 44.082 | 25.798 | -12.905 | 1.00 | 26.76 | C |
| ATOM | 1188 | CD1 | ILE A 150 | 41.461 | 26.475 | -11.538 | 1.00 | 27.93 | C |
| ATOM | 1189 | N   | ASP A 151 | 47.197 | 24.818 | -11.686 | 1.00 | 29.21 | N |
| ATOM | 1190 | CA  | ASP A 151 | 48.540 | 25.071 | -12.173 | 1.00 | 30.83 | C |
| ATOM | 1191 | C   | ASP A 151 | 49.291 | 25.933 | -11.157 | 1.00 | 32.30 | C |
| ATOM | 1192 | O   | ASP A 151 | 49.825 | 27.002 | -11.479 | 1.00 | 33.04 | O |
| ATOM | 1193 | CB  | ASP A 151 | 48.483 | 25.732 | -13.559 | 1.00 | 29.92 | C |
| ATOM | 1194 | CG  | ASP A 151 | 48.035 | 24.772 | -14.638 | 1.00 | 29.64 | C |
| ATOM | 1195 | OD1 | ASP A 151 | 48.214 | 23.543 | -14.485 | 1.00 | 29.49 | O |
| ATOM | 1196 | OD2 | ASP A 151 | 47.515 | 25.247 | -15.661 | 1.00 | 32.33 | O |
| ATOM | 1197 | N   | GLY A 152 | 49.278 | 25.465 | -9.912  | 1.00 | 33.30 | N |
| ATOM | 1198 | CA  | GLY A 152 | 50.021 | 26.104 | -8.830  | 1.00 | 32.88 | C |
| ATOM | 1199 | C   | GLY A 152 | 49.428 | 27.363 | -8.223  | 1.00 | 32.94 | C |
| ATOM | 1200 | O   | GLY A 152 | 49.848 | 27.763 | -7.139  | 1.00 | 34.06 | O |
| ATOM | 1201 | N   | SER A 153 | 48.465 | 27.989 | -8.896  | 1.00 | 32.78 | N |
| ATOM | 1202 | CA  | SER A 153 | 47.899 | 29.256 | -8.429  | 1.00 | 32.28 | C |
| ATOM | 1203 | C   | SER A 153 | 46.588 | 29.046 | -7.686  | 1.00 | 31.80 | C |
| ATOM | 1204 | O   | SER A 153 | 45.758 | 28.247 | -8.107  | 1.00 | 29.65 | O |
| ATOM | 1205 | CB  | SER A 153 | 47.678 | 30.198 | -9.602  | 1.00 | 33.13 | C |
| ATOM | 1206 | OG  | SER A 153 | 48.903 | 30.472 | -10.248 | 1.00 | 35.03 | O |
| ATOM | 1207 | N   | GLU A 154 | 46.393 | 29.799 | -6.604  | 1.00 | 31.06 | N |
| ATOM | 1208 | CA  | GLU A 154 | 45.245 | 29.589 | -5.743  | 1.00 | 31.23 | C |
| ATOM | 1209 | C   | GLU A 154 | 43.975 | 30.115 | -6.395  | 1.00 | 30.84 | C |
| ATOM | 1210 | O   | GLU A 154 | 43.914 | 31.235 | -6.903  | 1.00 | 31.00 | O |
| ATOM | 1211 | CB  | GLU A 154 | 45.436 | 30.228 | -4.368  | 1.00 | 30.92 | C |
| ATOM | 1212 | CG  | GLU A 154 | 44.192 | 30.144 | -3.486  | 1.00 | 32.03 | C |
| ATOM | 1213 | CD  | GLU A 154 | 44.392 | 30.752 | -2.113  | 1.00 | 33.28 | C |
| ATOM | 1214 | OE1 | GLU A 154 | 45.536 | 31.178 | -1.807  | 1.00 | 37.55 | O |
| ATOM | 1215 | OE2 | GLU A 154 | 43.410 | 30.806 | -1.335  | 1.00 | 34.36 | O |
| ATOM | 1216 | N   | ARG A 155 | 42.950 | 29.288 | -6.312  | 1.00 | 29.75 | N |
| ATOM | 1217 | CA  | ARG A 155 | 41.678 | 29.523 | -6.938  | 1.00 | 29.17 | C |
| ATOM | 1218 | C   | ARG A 155 | 40.623 | 29.531 | -5.855  | 1.00 | 28.26 | C |
| ATOM | 1219 | O   | ARG A 155 | 40.518 | 28.587 | -5.078  | 1.00 | 26.79 | O |
| ATOM | 1220 | CB  | ARG A 155 | 41.413 | 28.375 | -7.898  | 1.00 | 30.27 | C |
| ATOM | 1221 | CG  | ARG A 155 | 40.056 | 28.400 | -8.509  | 1.00 | 31.15 | C |
| ATOM | 1222 | CD  | ARG A 155 | 40.004 | 29.420 | -9.593  | 1.00 | 31.80 | C |
| ATOM | 1223 | NE  | ARG A 155 | 38.696 | 29.369 | -10.216 | 1.00 | 32.07 | N |

```
ATOM   1224  CZ   ARG A 155      38.476  29.448 -11.524  1.00 33.05           C
ATOM   1225  NH1  ARG A 155      39.489  29.543 -12.381  1.00 32.94           N
ATOM   1226  NH2  ARG A 155      37.219  29.410 -11.967  1.00 34.62           N
ATOM   1227  N    GLN A 156      39.849  30.599  -5.787  1.00 27.84           N
ATOM   1228  CA   GLN A 156      38.723  30.669  -4.856  1.00 29.02           C
ATOM   1229  C    GLN A 156      37.388  30.755  -5.580  1.00 28.28           C
ATOM   1230  O    GLN A 156      36.356  30.310  -5.069  1.00 28.51           O
ATOM   1231  CB   GLN A 156      38.908  31.846  -3.919  1.00 29.90           C
ATOM   1232  CG   GLN A 156      39.984  31.604  -2.893  1.00 30.72           C
ATOM   1233  CD   GLN A 156      40.494  32.881  -2.259  1.00 31.93           C
ATOM   1234  OE1  GLN A 156      41.702  33.143  -2.245  1.00 34.93           O
ATOM   1235  NE2  GLN A 156      39.578  33.687  -1.741  1.00 31.73           N
ATOM   1236  N    ASN A 157      37.435  31.300  -6.789  1.00 28.03           N
ATOM   1237  CA   ASN A 157      36.267  31.510  -7.632  1.00 26.88           C
ATOM   1238  C    ASN A 157      35.615  30.188  -8.023  1.00 24.25           C
ATOM   1239  O    ASN A 157      36.274  29.333  -8.582  1.00 24.79           O
ATOM   1240  CB   ASN A 157      36.735  32.250  -8.885  1.00 27.87           C
ATOM   1241  CG   ASN A 157      35.665  32.398  -9.928  1.00 29.41           C
ATOM   1242  OD1  ASN A 157      35.957  32.297 -11.121  1.00 33.62           O
ATOM   1243  ND2  ASN A 157      34.432  32.665  -9.505  1.00 30.48           N
ATOM   1244  N    GLY A 158      34.328  30.034  -7.750  1.00 23.73           N
ATOM   1245  CA   GLY A 158      33.607  28.814  -8.156  1.00 24.56           C
ATOM   1246  C    GLY A 158      33.739  27.666  -7.165  1.00 24.71           C
ATOM   1247  O    GLY A 158      33.436  26.517  -7.499  1.00 23.78           O
ATOM   1248  N    VAL A 159      34.163  27.975  -5.941  1.00 24.47           N
ATOM   1249  CA   VAL A 159      34.322  26.954  -4.891  1.00 24.20           C
ATOM   1250  C    VAL A 159      33.148  26.980  -3.938  1.00 24.12           C
ATOM   1251  O    VAL A 159      32.786  28.012  -3.380  1.00 22.96           O
ATOM   1252  CB   VAL A 159      35.649  27.115  -4.109  1.00 24.36           C
ATOM   1253  CG1  VAL A 159      35.772  26.042  -3.012  1.00 25.09           C
ATOM   1254  CG2  VAL A 159      36.855  27.065  -5.057  1.00 23.91           C
ATOM   1255  N    LEU A 160      32.533  25.828  -3.739  1.00 22.89           N
ATOM   1256  CA   LEU A 160      31.457  25.758  -2.784  1.00 24.47           C
ATOM   1257  C    LEU A 160      31.682  24.554  -1.896  1.00 23.74           C
ATOM   1258  O    LEU A 160      32.033  23.484  -2.386  1.00 21.97           O
ATOM   1259  CB   LEU A 160      30.104  25.695  -3.484  1.00 25.77           C
ATOM   1260  CG   LEU A 160      29.668  27.066  -4.068  1.00 27.42           C
ATOM   1261  CD1  LEU A 160      28.489  26.905  -5.006  1.00 28.66           C
ATOM   1262  CD2  LEU A 160      29.338  28.061  -2.967  1.00 29.06           C
ATOM   1263  N    ASN A 161      31.465  24.778  -0.598  1.00 23.37           N
ATOM   1264  CA   ASN A 161      31.812  23.851   0.448  1.00 22.89           C
ATOM   1265  C    ASN A 161      30.567  23.459   1.206  1.00 23.10           C
ATOM   1266  O    ASN A 161      29.647  24.248   1.337  1.00 22.68           O
ATOM   1267  CB   ASN A 161      32.830  24.509   1.385  1.00 21.81           C
ATOM   1268  CG   ASN A 161      34.151  24.780   0.711  1.00 22.20           C
ATOM   1269  OD1  ASN A 161      34.518  24.115  -0.248  1.00 20.88           O
ATOM   1270  ND2  ASN A 161      34.892  25.752   1.231  1.00 23.57           N
ATOM   1271  N    SER A 162      30.524  22.216   1.660  1.00 21.47           N
ATOM   1272  CA   SER A 162      29.407  21.705   2.405  1.00 22.18           C
ATOM   1273  C    SER A 162      29.924  20.738   3.468  1.00 22.21           C
ATOM   1274  O    SER A 162      30.717  19.852   3.160  1.00 20.72           O
ATOM   1275  CB   SER A 162      28.455  20.967   1.462  1.00 21.85           C
ATOM   1276  OG   SER A 162      27.302  20.584   2.150  1.00 23.96           O
ATOM   1277  N    TRP A 163      29.427  20.885   4.696  1.00 22.34           N
ATOM   1278  CA   TRP A 163      29.896  20.107   5.842  1.00 23.09           C
ATOM   1279  C    TRP A 163      28.754  19.174   6.297  1.00 20.69           C
ATOM   1280  O    TRP A 163      27.604  19.582   6.360  1.00 18.40           O
ATOM   1281  CB   TRP A 163      30.368  21.070   6.970  1.00 27.37           C
ATOM   1282  CG   TRP A 163      31.284  22.213   6.465  1.00 29.04           C
ATOM   1283  CD1  TRP A 163      32.639  22.302   6.587  1.00 30.17           C
ATOM   1284  CD2  TRP A 163      30.873  23.391   5.747  1.00 30.38           C
ATOM   1285  NE1  TRP A 163      33.101  23.464   5.991  1.00 30.80           N
ATOM   1286  CE2  TRP A 163      32.038  24.135   5.454  1.00 29.95           C
ATOM   1287  CE3  TRP A 163      29.630  23.882   5.310  1.00 31.18           C
ATOM   1288  CZ2  TRP A 163      31.999  25.360   4.766  1.00 30.50           C
```

527

```
ATOM   1289  CZ3 TRP A 163      29.594  25.110   4.618  1.00 30.38           C
ATOM   1290  CH2 TRP A 163      30.766  25.819   4.350  1.00 30.56           C
ATOM   1291  N   THR A 164      29.046  17.911   6.581  1.00 20.03           N
ATOM   1292  CA  THR A 164      28.046  17.061   7.203  1.00 19.47           C
ATOM   1293  C   THR A 164      27.850  17.434   8.701  1.00 17.69           C
ATOM   1294  O   THR A 164      28.669  18.121   9.315  1.00 18.13           O
ATOM   1295  CB  THR A 164      28.411  15.556   7.137  1.00 18.79           C
ATOM   1296  OG1 THR A 164      29.694  15.361   7.736  1.00 17.74           O
ATOM   1297  CG2 THR A 164      28.394  15.043   5.714  1.00 19.34           C
ATOM   1298  N   ASP A 165      26.720  17.008   9.245  1.00 19.05           N
ATOM   1299  CA  ASP A 165      26.534  16.913  10.685  1.00 19.55           C
ATOM   1300  C   ASP A 165      27.432  15.810  11.283  1.00 19.65           C
ATOM   1301  O   ASP A 165      28.021  14.969  10.571  1.00 20.31           O
ATOM   1302  CB  ASP A 165      25.078  16.610  11.016  1.00 22.57           C
ATOM   1303  CG  ASP A 165      24.130  17.739  10.621  1.00 24.90           C
ATOM   1304  OD1 ASP A 165      24.455  18.934  10.834  1.00 28.97           O
ATOM   1305  OD2 ASP A 165      23.046  17.425  10.087  1.00 30.95           O
ATOM   1306  N   GLN A 166      27.532  15.791  12.597  1.00 18.23           N
ATOM   1307  CA  GLN A 166      28.438  14.849  13.234  1.00 17.91           C
ATOM   1308  C   GLN A 166      27.945  13.433  12.987  1.00 19.04           C
ATOM   1309  O   GLN A 166      26.753  13.159  13.042  1.00 19.57           O
ATOM   1310  CB  GLN A 166      28.544  15.107  14.715  1.00 17.12           C
ATOM   1311  CG  GLN A 166      29.618  14.252  15.378  1.00 17.01           C
ATOM   1312  CD  GLN A 166      29.984  14.740  16.738  1.00 16.77           C
ATOM   1313  OE1 GLN A 166      29.120  15.184  17.513  1.00 19.10           O
ATOM   1314  NE2 GLN A 166      31.286  14.672  17.063  1.00 12.83           N
ATOM   1315  N   ASP A 167      28.869  12.530  12.731  1.00 18.97           N
ATOM   1316  CA  ASP A 167      28.504  11.159  12.378  1.00 20.66           C
ATOM   1317  C   ASP A 167      27.996  10.379  13.608  1.00 20.80           C
ATOM   1318  O   ASP A 167      28.646  10.377  14.624  1.00 19.47           O
ATOM   1319  CB  ASP A 167      29.699  10.460  11.775  1.00 20.51           C
ATOM   1320  CG  ASP A 167      29.336   9.163  11.140  1.00 22.80           C
ATOM   1321  OD1 ASP A 167      28.842   9.207  10.003  1.00 27.30           O
ATOM   1322  OD2 ASP A 167      29.522   8.091  11.766  1.00 23.63           O
ATOM   1323  N   SER A 168      26.867   9.678  13.468  1.00 22.99           N
ATOM   1324  CA  SER A 168      26.259   8.885  14.553  1.00 23.69           C
ATOM   1325  C   SER A 168      27.066   7.656  14.940  1.00 25.04           C
ATOM   1326  O   SER A 168      26.813   7.026  15.981  1.00 25.40           O
ATOM   1327  CB  SER A 168      24.869   8.419  14.138  1.00 23.46           C
ATOM   1328  OG  SER A 168      23.981   9.525  14.023  1.00 28.08           O
ATOM   1329  N   LYS A 169      28.015   7.286  14.089  1.00 26.21           N
ATOM   1330  CA  LYS A 169      28.800   6.079  14.306  1.00 24.96           C
ATOM   1331  C   LYS A 169      30.191   6.348  14.841  1.00 24.58           C
ATOM   1332  O   LYS A 169      30.573   5.781  15.857  1.00 24.71           O
ATOM   1333  CB  LYS A 169      28.860   5.255  13.010  1.00 27.71           C
ATOM   1334  CG  LYS A 169      27.889   4.103  12.998  1.00 29.20           C
ATOM   1335  CD  LYS A 169      26.454   4.480  13.403  1.00 31.38           C
ATOM   1336  CE  LYS A 169      25.618   3.212  13.778  1.00 32.41           C
ATOM   1337  NZ  LYS A 169      25.588   2.839  15.266  1.00 34.33           N
ATOM   1338  N   ASP A 170      30.955   7.227  14.198  1.00 23.63           N
ATOM   1339  CA  ASP A 170      32.322   7.486  14.645  1.00 22.23           C
ATOM   1340  C   ASP A 170      32.553   8.892  15.164  1.00 19.69           C
ATOM   1341  O   ASP A 170      33.661   9.221  15.488  1.00 17.81           O
ATOM   1342  CB  ASP A 170      33.347   7.144  13.551  1.00 23.65           C
ATOM   1343  CG  ASP A 170      33.195   7.978  12.294  1.00 26.27           C
ATOM   1344  OD1 ASP A 170      32.597   9.079  12.327  1.00 23.63           O
ATOM   1345  OD2 ASP A 170      33.695   7.522  11.246  1.00 28.60           O
ATOM   1346  N   SER A 171      31.506   9.703  15.251  1.00 18.64           N
ATOM   1347  CA  SER A 171      31.611  11.036  15.834  1.00 19.75           C
ATOM   1348  C   SER A 171      32.519  11.994  15.054  1.00 18.05           C
ATOM   1349  O   SER A 171      32.902  13.075  15.569  1.00 18.88           O
ATOM   1350  CB  SER A 171      32.009  10.950  17.317  1.00 19.55           C
ATOM   1351  OG  SER A 171      31.155  10.014  17.981  1.00 21.94           O
ATOM   1352  N   THR A 172      32.842  11.634  13.814  1.00 18.18           N
ATOM   1353  CA  THR A 172      33.605  12.534  12.930  1.00 18.39           C
```

528

| ATOM | 1354 | C | THR | A | 172 | 32.715 | 13.420 | 12.056 | 1.00 | 17.66 | C |
|------|------|-----|-----|---|-----|--------|--------|--------|------|-------|---|
| ATOM | 1355 | O | THR | A | 172 | 31.496 | 13.268 | 12.022 | 1.00 | 16.05 | O |
| ATOM | 1356 | CB | THR | A | 172 | 34.494 | 11.755 | 11.951 | 1.00 | 17.97 | C |
| ATOM | 1357 | OG1 | THR | A | 172 | 33.667 | 11.047 | 11.012 | 1.00 | 19.49 | O |
| ATOM | 1358 | CG2 | THR | A | 172 | 35.408 | 10.824 | 12.693 | 1.00 | 19.64 | C |
| ATOM | 1359 | N | TYR | A | 173 | 33.375 | 14.328 | 11.343 | 1.00 | 17.71 | N |
| ATOM | 1360 | CA | TYR | A | 173 | 32.768 | 15.203 | 10.367 | 1.00 | 18.04 | C |
| ATOM | 1361 | C | TYR | A | 173 | 33.440 | 14.989 | 9.035 | 1.00 | 17.90 | C |
| ATOM | 1362 | O | TYR | A | 173 | 34.567 | 14.531 | 8.988 | 1.00 | 19.17 | O |
| ATOM | 1363 | CB | TYR | A | 173 | 33.004 | 16.660 | 10.768 | 1.00 | 18.44 | C |
| ATOM | 1364 | CG | TYR | A | 173 | 32.403 | 17.034 | 12.080 | 1.00 | 18.30 | C |
| ATOM | 1365 | CD1 | TYR | A | 173 | 33.112 | 16.878 | 13.273 | 1.00 | 18.68 | C |
| ATOM | 1366 | CD2 | TYR | A | 173 | 31.108 | 17.538 | 12.142 | 1.00 | 20.80 | C |
| ATOM | 1367 | CE1 | TYR | A | 173 | 32.538 | 17.214 | 14.491 | 1.00 | 18.52 | C |
| ATOM | 1368 | CE2 | TYR | A | 173 | 30.536 | 17.904 | 13.357 | 1.00 | 19.29 | C |
| ATOM | 1369 | CZ | TYR | A | 173 | 31.247 | 17.743 | 14.512 | 1.00 | 19.71 | C |
| ATOM | 1370 | OH | TYR | A | 173 | 30.632 | 18.100 | 15.689 | 1.00 | 20.82 | O |
| ATOM | 1371 | N | SER | A | 174 | 32.741 | 15.343 | 7.968 | 1.00 | 18.71 | N |
| ATOM | 1372 | CA | SER | A | 174 | 33.238 | 15.278 | 6.596 | 1.00 | 17.61 | C |
| ATOM | 1373 | C | SER | A | 174 | 32.838 | 16.579 | 5.862 | 1.00 | 18.49 | C |
| ATOM | 1374 | O | SER | A | 174 | 31.887 | 17.272 | 6.242 | 1.00 | 17.94 | O |
| ATOM | 1375 | CB | SER | A | 174 | 32.649 | 14.064 | 5.884 | 1.00 | 17.43 | C |
| ATOM | 1376 | OG | SER | A | 174 | 32.970 | 12.837 | 6.583 | 1.00 | 15.06 | O |
| ATOM | 1377 | N | MET | A | 175 | 33.571 | 16.936 | 4.815 | 1.00 | 19.78 | N |
| ATOM | 1378 | CA | MET | A | 175 | 33.258 | 18.162 | 4.070 | 1.00 | 19.38 | C |
| ATOM | 1379 | C | MET | A | 175 | 33.538 | 17.863 | 2.614 | 1.00 | 18.14 | C |
| ATOM | 1380 | O | MET | A | 175 | 34.471 | 17.128 | 2.306 | 1.00 | 16.21 | O |
| ATOM | 1381 | CB | MET | A | 175 | 34.098 | 19.350 | 4.578 | 1.00 | 21.01 | C |
| ATOM | 1382 | CG | MET | A | 175 | 33.937 | 20.703 | 3.824 | 1.00 | 23.02 | C |
| ATOM | 1383 | SD | MET | A | 175 | 35.510 | 21.599 | 3.959 | 1.00 | 29.29 | S |
| ATOM | 1384 | CE | MET | A | 175 | 35.441 | 22.947 | 2.825 | 1.00 | 28.47 | C |
| ATOM | 1385 | N | SER | A | 176 | 32.698 | 18.387 | 1.720 | 1.00 | 16.50 | N |
| ATOM | 1386 | CA | SER | A | 176 | 33.005 | 18.354 | 0.276 | 1.00 | 17.00 | C |
| ATOM | 1387 | C | SER | A | 176 | 33.373 | 19.766 | -0.181 | 1.00 | 15.91 | C |
| ATOM | 1388 | O | SER | A | 176 | 32.808 | 20.721 | 0.295 | 1.00 | 15.34 | O |
| ATOM | 1389 | CB | SER | A | 176 | 31.790 | 17.881 | -0.525 | 1.00 | 18.26 | C |
| ATOM | 1390 | OG | SER | A | 176 | 30.797 | 18.912 | -0.492 | 1.00 | 21.23 | O |
| ATOM | 1391 | N | SER | A | 177 | 34.331 | 19.884 | -1.091 | 1.00 | 15.49 | N |
| ATOM | 1392 | CA | SER | A | 177 | 34.613 | 21.138 | -1.748 | 1.00 | 16.37 | C |
| ATOM | 1393 | C | SER | A | 177 | 34.503 | 20.864 | -3.254 | 1.00 | 16.93 | C |
| ATOM | 1394 | O | SER | A | 177 | 35.108 | 19.925 | -3.758 | 1.00 | 15.21 | O |
| ATOM | 1395 | CB | SER | A | 177 | 35.999 | 21.636 | -1.391 | 1.00 | 16.59 | C |
| ATOM | 1396 | OG | SER | A | 177 | 36.154 | 22.961 | -1.877 | 1.00 | 16.93 | O |
| ATOM | 1397 | N | THR | A | 178 | 33.686 | 21.640 | -3.954 | 1.00 | 18.37 | N |
| ATOM | 1398 | CA | THR | A | 178 | 33.438 | 21.413 | -5.362 | 1.00 | 18.32 | C |
| ATOM | 1399 | C | THR | A | 178 | 33.888 | 22.662 | -6.106 | 1.00 | 19.14 | C |
| ATOM | 1400 | O | THR | A | 178 | 33.442 | 23.753 | -5.792 | 1.00 | 16.72 | O |
| ATOM | 1401 | CB | THR | A | 178 | 31.949 | 21.090 | -5.638 | 1.00 | 20.85 | C |
| ATOM | 1402 | OG1 | THR | A | 178 | 31.606 | 19.843 | -5.009 | 1.00 | 17.60 | O |
| ATOM | 1403 | CG2 | THR | A | 178 | 31.642 | 20.967 | -7.158 | 1.00 | 19.66 | C |
| ATOM | 1404 | N | LEU | A | 179 | 34.813 | 22.495 | -7.040 | 1.00 | 17.75 | N |
| ATOM | 1405 | CA | LEU | A | 179 | 35.263 | 23.597 | -7.887 | 1.00 | 18.82 | C |
| ATOM | 1406 | C | LEU | A | 179 | 34.508 | 23.486 | -9.213 | 1.00 | 19.18 | C |
| ATOM | 1407 | O | LEU | A | 179 | 34.702 | 22.526 | -9.962 | 1.00 | 18.85 | O |
| ATOM | 1408 | CB | LEU | A | 179 | 36.764 | 23.524 | -8.115 | 1.00 | 17.88 | C |
| ATOM | 1409 | CG | LEU | A | 179 | 37.371 | 24.565 | -9.073 | 1.00 | 18.46 | C |
| ATOM | 1410 | CD1 | LEU | A | 179 | 36.850 | 25.950 | -8.773 | 1.00 | 18.17 | C |
| ATOM | 1411 | CD2 | LEU | A | 179 | 38.846 | 24.512 | -8.997 | 1.00 | 19.67 | C |
| ATOM | 1412 | N | THR | A | 180 | 33.614 | 24.440 | -9.451 | 1.00 | 21.01 | N |
| ATOM | 1413 | CA | THR | A | 180 | 32.736 | 24.443 | -10.627 | 1.00 | 21.56 | C |
| ATOM | 1414 | C | THR | A | 180 | 33.361 | 25.283 | -11.725 | 1.00 | 22.62 | C |
| ATOM | 1415 | O | THR | A | 180 | 33.483 | 26.490 | -11.581 | 1.00 | 22.51 | O |
| ATOM | 1416 | CB | THR | A | 180 | 31.354 | 25.060 | -10.305 | 1.00 | 22.38 | C |
| ATOM | 1417 | OG1 | THR | A | 180 | 30.824 | 24.487 | -9.118 | 1.00 | 27.59 | O |
| ATOM | 1418 | CG2 | THR | A | 180 | 30.351 | 24.795 | -11.429 | 1.00 | 23.57 | C |

```
ATOM   1419  N    LEU A 181      33.774  24.623 -12.799  1.00 23.15           N
ATOM   1420  CA   LEU A 181      34.286  25.270 -13.991  1.00 23.21           C
ATOM   1421  C    LEU A 181      33.374  24.955 -15.176  1.00 24.40           C
ATOM   1422  O    LEU A 181      32.586  23.988 -15.128  1.00 24.54           O
ATOM   1423  CB   LEU A 181      35.702  24.786 -14.261  1.00 24.13           C
ATOM   1424  CG   LEU A 181      36.748  25.065 -13.163  1.00 23.60           C
ATOM   1425  CD1  LEU A 181      38.076  24.489 -13.519  1.00 25.41           C
ATOM   1426  CD2  LEU A 181      36.905  26.556 -12.916  1.00 24.78           C
ATOM   1427  N    THR A 182      33.444  25.792 -16.211  1.00 23.21           N
ATOM   1428  CA   THR A 182      32.950  25.418 -17.531  1.00 21.84           C
ATOM   1429  C    THR A 182      34.003  24.548 -18.191  1.00 21.09           C
ATOM   1430  O    THR A 182      35.193  24.628 -17.851  1.00 19.19           O
ATOM   1431  CB   THR A 182      32.735  26.642 -18.454  1.00 22.47           C
ATOM   1432  OG1  THR A 182      33.967  27.369 -18.615  1.00 22.04           O
ATOM   1433  CG2  THR A 182      31.666  27.537 -17.900  1.00 22.73           C
ATOM   1434  N    LYS A 183      33.572  23.706 -19.134  1.00 18.71           N
ATOM   1435  CA   LYS A 183      34.504  22.915 -19.936  1.00 19.56           C
ATOM   1436  C    LYS A 183      35.532  23.804 -20.569  1.00 19.90           C
ATOM   1437  O    LYS A 183      36.710  23.494 -20.572  1.00 16.90           O
ATOM   1438  CB   LYS A 183      33.770  22.134 -21.050  1.00 19.23           C
ATOM   1439  CG   LYS A 183      34.715  21.373 -21.976  1.00 20.75           C
ATOM   1440  CD   LYS A 183      33.981  20.607 -23.086  1.00 20.91           C
ATOM   1441  CE   LYS A 183      34.938  20.223 -24.181  1.00 22.24           C
ATOM   1442  NZ   LYS A 183      34.239  19.532 -25.255  1.00 25.70           N
ATOM   1443  N    ASP A 184      35.063  24.936 -21.096  1.00 20.48           N
ATOM   1444  CA   ASP A 184      35.937  25.924 -21.709  1.00 21.10           C
ATOM   1445  C    ASP A 184      37.104  26.326 -20.769  1.00 22.28           C
ATOM   1446  O    ASP A 184      38.260  26.235 -21.164  1.00 24.07           O
ATOM   1447  CB   ASP A 184      35.086  27.114 -22.128  1.00 21.22           C
ATOM   1448  CG   ASP A 184      35.891  28.311 -22.451  1.00 22.62           C
ATOM   1449  OD1  ASP A 184      36.835  28.183 -23.267  1.00 22.07           O
ATOM   1450  OD2  ASP A 184      35.562  29.389 -21.893  1.00 22.10           O
ATOM   1451  N    GLU A 185      36.823  26.701 -19.528  1.00 22.42           N
ATOM   1452  CA   GLU A 185      37.901  27.097 -18.594  1.00 22.66           C
ATOM   1453  C    GLU A 185      38.732  25.938 -18.064  1.00 21.95           C
ATOM   1454  O    GLU A 185      39.936  26.067 -17.869  1.00 20.30           O
ATOM   1455  CB   GLU A 185      37.367  27.860 -17.393  1.00 23.62           C
ATOM   1456  CG   GLU A 185      38.522  28.414 -16.511  1.00 25.58           C
ATOM   1457  CD   GLU A 185      38.048  29.363 -15.458  1.00 27.39           C
ATOM   1458  OE1  GLU A 185      38.845  30.256 -15.030  1.00 31.93           O
ATOM   1459  OE2  GLU A 185      36.866  29.242 -15.063  1.00 32.25           O
ATOM   1460  N    TYR A 186      38.086  24.813 -17.777  1.00 20.06           N
ATOM   1461  CA   TYR A 186      38.808  23.619 -17.338  1.00 20.78           C
ATOM   1462  C    TYR A 186      39.904  23.198 -18.325  1.00 21.99           C
ATOM   1463  O    TYR A 186      41.001  22.800 -17.938  1.00 24.22           O
ATOM   1464  CB   TYR A 186      37.800  22.488 -17.178  1.00 20.18           C
ATOM   1465  CG   TYR A 186      38.386  21.154 -16.786  1.00 18.89           C
ATOM   1466  CD1  TYR A 186      38.963  20.960 -15.532  1.00 18.88           C
ATOM   1467  CD2  TYR A 186      38.309  20.078 -17.640  1.00 18.49           C
ATOM   1468  CE1  TYR A 186      39.501  19.686 -15.180  1.00 17.01           C
ATOM   1469  CE2  TYR A 186      38.810  18.842 -17.283  1.00 18.64           C
ATOM   1470  CZ   TYR A 186      39.404  18.656 -16.055  1.00 19.04           C
ATOM   1471  OH   TYR A 186      39.930  17.399 -15.737  1.00 18.59           O
ATOM   1472  N    GLU A 187      39.581  23.227 -19.610  1.00 23.03           N
ATOM   1473  CA   GLU A 187      40.531  22.846 -20.647  1.00 24.65           C
ATOM   1474  C    GLU A 187      41.630  23.881 -20.925  1.00 26.14           C
ATOM   1475  O    GLU A 187      42.522  23.600 -21.735  1.00 25.63           O
ATOM   1476  CB   GLU A 187      39.795  22.415 -21.911  1.00 24.07           C
ATOM   1477  CG   GLU A 187      39.097  21.095 -21.667  1.00 24.29           C
ATOM   1478  CD   GLU A 187      38.218  20.609 -22.792  1.00 25.72           C
ATOM   1479  OE1  GLU A 187      37.847  21.390 -23.718  1.00 22.84           O
ATOM   1480  OE2  GLU A 187      37.885  19.407 -22.704  1.00 26.54           O
ATOM   1481  N    ARG A 188      41.608  25.020 -20.222  1.00 26.66           N
ATOM   1482  CA   ARG A 188      42.678  26.022 -20.335  1.00 27.97           C
ATOM   1483  C    ARG A 188      43.804  25.807 -19.312  1.00 27.58           C
```

```
ATOM   1484  O    ARG A 188      44.757  26.605 -19.256  1.00 27.99           O
ATOM   1485  CB AARG A 188      42.088  27.432 -20.220  0.50 27.66           C
ATOM   1486  CB BARG A 188      42.123  27.439 -20.140  0.50 27.80           C
ATOM   1487  CG AARG A 188      41.190  27.776 -21.392  0.50 28.19           C
ATOM   1488  CG BARG A 188      41.241  27.929 -21.242  0.50 28.53           C
ATOM   1489  CD AARG A 188      40.644  29.187 -21.358  0.50 27.97           C
ATOM   1490  CD BARG A 188      40.954  29.417 -21.072  0.50 28.69           C
ATOM   1491  NE AARG A 188      39.578  29.374 -22.344  0.50 27.88           N
ATOM   1492  NE BARG A 188      39.522  29.693 -21.133  0.50 29.30           N
ATOM   1493  CZ AARG A 188      39.753  29.454 -23.664  0.50 27.88           C
ATOM   1494  CZ BARG A 188      38.817  30.330 -20.204  0.50 29.01           C
ATOM   1495  NH1AARG A 188      40.964  29.354 -24.229  0.50 28.36           N
ATOM   1496  NH1BARG A 188      39.374  30.828 -19.105  0.50 30.63           N
ATOM   1497  NH2AARG A 188      38.705  29.622 -24.441  0.50 27.04           N
ATOM   1498  NH2BARG A 188      37.526  30.494 -20.390  0.50 29.69           N
ATOM   1499  N    HIS A 189      43.709  24.737 -18.513  1.00 27.27           N
ATOM   1500  CA   HIS A 189      44.701  24.421 -17.471  1.00 27.52           C
ATOM   1501  C    HIS A 189      44.930  22.931 -17.363  1.00 27.34           C
ATOM   1502  O    HIS A 189      44.140  22.165 -17.880  1.00 28.22           O
ATOM   1503  CB   HIS A 189      44.224  24.932 -16.128  1.00 27.81           C
ATOM   1504  CG   HIS A 189      43.746  26.339 -16.167  1.00 28.14           C
ATOM   1505  ND1  HIS A 189      44.590  27.391 -16.441  1.00 30.78           N
ATOM   1506  CD2  HIS A 189      42.517  26.871 -15.993  1.00 28.93           C
ATOM   1507  CE1  HIS A 189      43.902  28.518 -16.403  1.00 31.30           C
ATOM   1508  NE2  HIS A 189      42.641  28.230 -16.138  1.00 31.31           N
ATOM   1509  N    ASN A 190      45.996  22.522 -16.684  1.00 27.96           N
ATOM   1510  CA   ASN A 190      46.372  21.088 -16.612  1.00 27.79           C
ATOM   1511  C    ASN A 190      46.261  20.484 -15.233  1.00 27.26           C
ATOM   1512  O    ASN A 190      45.691  19.408 -15.074  1.00 28.03           O
ATOM   1513  CB   ASN A 190      47.804  20.845 -17.129  1.00 29.37           C
ATOM   1514  CG   ASN A 190      48.180  19.343 -17.154  1.00 30.99           C
ATOM   1515  OD1  ASN A 190      47.443  18.518 -17.726  1.00 35.26           O
ATOM   1516  ND2  ASN A 190      49.307  18.984 -16.500  1.00 32.23           N
ATOM   1517  N    SER A 191      46.828  21.141 -14.228  1.00 26.91           N
ATOM   1518  CA   SER A 191      46.948  20.508 -12.923  1.00 26.71           C
ATOM   1519  C    SER A 191      45.999  21.121 -11.911  1.00 24.85           C
ATOM   1520  O    SER A 191      45.956  22.344 -11.745  1.00 25.88           O
ATOM   1521  CB   SER A 191      48.369  20.608 -12.396  1.00 28.16           C
ATOM   1522  OG   SER A 191      48.518  21.869 -11.767  1.00 31.84           O
ATOM   1523  N    TYR A 192      45.281  20.242 -11.223  1.00 23.83           N
ATOM   1524  CA   TYR A 192      44.236  20.608 -10.269  1.00 22.07           C
ATOM   1525  C    TYR A 192      44.592  19.993  -8.916  1.00 21.37           C
ATOM   1526  O    TYR A 192      44.902  18.799  -8.814  1.00 20.27           O
ATOM   1527  CB   TYR A 192      42.862  20.122 -10.772  1.00 19.70           C
ATOM   1528  CG   TYR A 192      42.405  20.929 -11.962  1.00 18.47           C
ATOM   1529  CD1  TYR A 192      41.612  22.040 -11.787  1.00 17.30           C
ATOM   1530  CD2  TYR A 192      42.859  20.654 -13.247  1.00 18.95           C
ATOM   1531  CE1  TYR A 192      41.229  22.810 -12.867  1.00 19.39           C
ATOM   1532  CE2  TYR A 192      42.490  21.440 -14.333  1.00 18.53           C
ATOM   1533  CZ   TYR A 192      41.693  22.524 -14.127  1.00 18.38           C
ATOM   1534  OH   TYR A 192      41.304  23.282 -15.198  1.00 19.98           O
ATOM   1535  N    THR A 193      44.601  20.834  -7.882  1.00 21.57           N
ATOM   1536  CA   THR A 193      45.027  20.413  -6.544  1.00 21.36           C
ATOM   1537  C    THR A 193      44.050  20.912  -5.495  1.00 20.87           C
ATOM   1538  O    THR A 193      43.619  22.043  -5.573  1.00 20.90           O
ATOM   1539  CB   THR A 193      46.412  20.980  -6.204  1.00 21.28           C
ATOM   1540  OG1  THR A 193      47.381  20.515  -7.150  1.00 23.98           O
ATOM   1541  CG2  THR A 193      46.848  20.567  -4.829  1.00 22.06           C
ATOM   1542  N    CYS A 194      43.702  20.062  -4.533  1.00 21.41           N
ATOM   1543  CA   CYS A 194      42.993  20.495  -3.304  1.00 23.19           C
ATOM   1544  C    CYS A 194      43.890  20.158  -2.100  1.00 22.74           C
ATOM   1545  O    CYS A 194      44.539  19.118  -2.051  1.00 20.49           O
ATOM   1546  CB   CYS A 194      41.559  19.903  -3.167  1.00 24.19           C
ATOM   1547  SG   CYS A 194      41.429  18.142  -2.907  1.00 30.22           S
ATOM   1548  N    GLU A 195      43.967  21.089  -1.164  1.00 24.25           N
```

531

```
ATOM   1549  CA  GLU A 195      44.833  20.941  -0.002  1.00 24.15           C
ATOM   1550  C   GLU A 195      43.998  21.121   1.242  1.00 23.21           C
ATOM   1551  O   GLU A 195      43.320  22.134   1.384  1.00 23.68           O
ATOM   1552  CB  GLU A 195      45.943  22.000  -0.004  1.00 25.34           C
ATOM   1553  CG  GLU A 195      46.722  22.127  -1.317  1.00 26.76           C
ATOM   1554  CD  GLU A 195      47.933  23.043  -1.186  1.00 27.29           C
ATOM   1555  OE1 GLU A 195      47.956  23.916  -0.287  1.00 28.80           O
ATOM   1556  OE2 GLU A 195      48.860  22.873  -1.991  1.00 31.12           O
ATOM   1557  N   ALA A 196      44.035  20.138   2.133  1.00 21.31           N
ATOM   1558  CA  ALA A 196      43.300  20.196   3.393  1.00 21.70           C
ATOM   1559  C   ALA A 196      44.239  20.563   4.549  1.00 22.22           C
ATOM   1560  O   ALA A 196      45.210  19.868   4.783  1.00 21.13           O
ATOM   1561  CB  ALA A 196      42.665  18.861   3.673  1.00 21.33           C
ATOM   1562  N   THR A 197      43.944  21.650   5.256  1.00 22.53           N
ATOM   1563  CA  THR A 197      44.621  21.976   6.511  1.00 22.22           C
ATOM   1564  C   THR A 197      43.669  21.809   7.716  1.00 23.01           C
ATOM   1565  O   THR A 197      42.581  22.421   7.785  1.00 23.27           O
ATOM   1566  CB  THR A 197      45.210  23.411   6.523  1.00 22.97           C
ATOM   1567  OG1 THR A 197      45.907  23.660   5.299  1.00 24.75           O
ATOM   1568  CG2 THR A 197      46.193  23.567   7.698  1.00 23.10           C
ATOM   1569  N   HIS A 198      44.127  21.004   8.670  1.00 20.08           N
ATOM   1570  CA  HIS A 198      43.385  20.612   9.856  1.00 20.40           C
ATOM   1571  C   HIS A 198      44.350  20.589  11.062  1.00 19.53           C
ATOM   1572  O   HIS A 198      45.554  20.417  10.908  1.00 20.53           O
ATOM   1573  CB  HIS A 198      42.812  19.211   9.603  1.00 17.51           C
ATOM   1574  CG  HIS A 198      41.905  18.710  10.683  1.00 18.47           C
ATOM   1575  ND1 HIS A 198      42.253  17.660  11.505  1.00 15.60           N
ATOM   1576  CD2 HIS A 198      40.650  19.075  11.049  1.00 17.06           C
ATOM   1577  CE1 HIS A 198      41.273  17.431  12.361  1.00 16.77           C
ATOM   1578  NE2 HIS A 198      40.265  18.236  12.069  1.00 16.62           N
ATOM   1579  N   LYS A 199      43.840  20.747  12.268  1.00 21.30           N
ATOM   1580  CA  LYS A 199      44.746  20.793  13.436  1.00 22.69           C
ATOM   1581  C   LYS A 199      45.649  19.565  13.650  1.00 23.56           C
ATOM   1582  O   LYS A 199      46.662  19.634  14.348  1.00 23.36           O
ATOM   1583  CB  LYS A 199      43.964  21.091  14.702  1.00 24.15           C
ATOM   1584  CG  LYS A 199      43.124  19.966  15.249  1.00 24.15           C
ATOM   1585  CD  LYS A 199      42.439  20.420  16.523  1.00 24.09           C
ATOM   1586  CE  LYS A 199      43.411  20.746  17.626  1.00 25.35           C
ATOM   1587  NZ  LYS A 199      42.666  20.951  18.881  1.00 25.82           N
ATOM   1588  N   THR A 200      45.307  18.447  13.033  1.00 22.36           N
ATOM   1589  CA  THR A 200      46.069  17.216  13.213  1.00 22.59           C
ATOM   1590  C   THR A 200      47.463  17.228  12.567  1.00 22.87           C
ATOM   1591  O   THR A 200      48.249  16.294  12.781  1.00 25.10           O
ATOM   1592  CB  THR A 200      45.270  15.995  12.694  1.00 21.33           C
ATOM   1593  OG1 THR A 200      44.628  16.333  11.466  1.00 20.41           O
ATOM   1594  CG2 THR A 200      44.230  15.579  13.735  1.00 21.52           C
ATOM   1595  N   SER A 201      47.767  18.262  11.774  1.00 22.18           N
ATOM   1596  CA  SER A 201      49.120  18.487  11.283  1.00 22.09           C
ATOM   1597  C   SER A 201      49.316  19.924  10.863  1.00 22.43           C
ATOM   1598  O   SER A 201      48.381  20.625  10.505  1.00 20.87           O
ATOM   1599  CB  SER A 201      49.486  17.576  10.112  1.00 22.18           C
ATOM   1600  OG  SER A 201      50.840  17.766   9.674  1.00 20.72           O
ATOM   1601  N   THR A 202      50.591  20.385  10.906  1.00 22.22           N
ATOM   1602  CA  THR A 202      50.923  21.739  10.456  1.00 23.65           C
ATOM   1603  C   THR A 202      51.093  21.764   8.932  1.00 24.10           C
ATOM   1604  O   THR A 202      51.185  22.826   8.346  1.00 26.52           O
ATOM   1605  CB  THR A 202      52.205  22.283  11.169  1.00 23.81           C
ATOM   1606  OG1 THR A 202      53.357  21.572  10.714  1.00 26.08           O
ATOM   1607  CG2 THR A 202      52.106  22.102  12.666  1.00 23.51           C
ATOM   1608  N   SER A 203      51.139  20.579   8.323  1.00 25.22           N
ATOM   1609  CA  SER A 203      51.264  20.443   6.873  1.00 26.62           C
ATOM   1610  C   SER A 203      49.918  20.020   6.265  1.00 27.16           C
ATOM   1611  O   SER A 203      49.280  19.091   6.770  1.00 27.67           O
ATOM   1612  CB  SER A 203      52.257  19.327   6.530  1.00 26.55           C
ATOM   1613  OG  SER A 203      53.604  19.724   6.602  1.00 29.35           O
```

```
ATOM   1614  N    PRO A 204      49.509  20.656   5.151  1.00 26.90           N
ATOM   1615  CA   PRO A 204      48.262  20.239   4.521  1.00 26.67           C
ATOM   1616  C    PRO A 204      48.405  18.882   3.853  1.00 26.44           C
ATOM   1617  O    PRO A 204      49.515  18.486   3.480  1.00 27.37           O
ATOM   1618  CB   PRO A 204      48.033  21.319   3.467  1.00 26.85           C
ATOM   1619  CG   PRO A 204      49.379  21.721   3.078  1.00 27.72           C
ATOM   1620  CD   PRO A 204      50.179  21.706   4.370  1.00 27.19           C
ATOM   1621  N    ILE A 205      47.280  18.178   3.739  1.00 25.79           N
ATOM   1622  CA   ILE A 205      47.150  16.969   2.951  1.00 25.52           C
ATOM   1623  C    ILE A 205      46.788  17.413   1.562  1.00 25.82           C
ATOM   1624  O    ILE A 205      45.768  18.066   1.381  1.00 25.64           O
ATOM   1625  CB   ILE A 205      46.046  16.062   3.489  1.00 25.39           C
ATOM   1626  CG1  ILE A 205      46.327  15.743   4.965  1.00 25.64           C
ATOM   1627  CG2  ILE A 205      45.951  14.758   2.627  1.00 25.20           C
ATOM   1628  CD1  ILE A 205      45.180  15.198   5.716  1.00 25.57           C
ATOM   1629  N    VAL A 206      47.637  17.071   0.592  1.00 25.81           N
ATOM   1630  CA   VAL A 206      47.526  17.598  -0.763  1.00 25.67           C
ATOM   1631  C    VAL A 206      47.163  16.478  -1.716  1.00 26.51           C
ATOM   1632  O    VAL A 206      47.866  15.444  -1.748  1.00 26.76           O
ATOM   1633  CB   VAL A 206      48.842  18.227  -1.215  1.00 26.14           C
ATOM   1634  CG1  VAL A 206      48.755  18.661  -2.672  1.00 27.25           C
ATOM   1635  CG2  VAL A 206      49.214  19.424  -0.328  1.00 26.84           C
ATOM   1636  N    LYS A 207      46.084  16.679  -2.489  1.00 24.91           N
ATOM   1637  CA   LYS A 207      45.689  15.753  -3.546  1.00 24.56           C
ATOM   1638  C    LYS A 207      45.598  16.483  -4.875  1.00 23.81           C
ATOM   1639  O    LYS A 207      45.186  17.635  -4.950  1.00 24.66           O
ATOM   1640  CB   LYS A 207      44.366  15.081  -3.210  1.00 25.27           C
ATOM   1641  CG   LYS A 207      44.439  14.223  -1.973  1.00 25.59           C
ATOM   1642  CD   LYS A 207      45.112  12.889  -2.260  1.00 27.99           C
ATOM   1643  CE   LYS A 207      45.476  12.190  -0.970  1.00 27.63           C
ATOM   1644  NZ   LYS A 207      45.762  10.767  -1.216  1.00 29.03           N
ATOM   1645  N    SER A 208      46.035  15.823  -5.934  1.00 24.04           N
ATOM   1646  CA   SER A 208      46.121  16.466  -7.239  1.00 24.41           C
ATOM   1647  C    SER A 208      45.815  15.495  -8.370  1.00 23.36           C
ATOM   1648  O    SER A 208      45.855  14.269  -8.195  1.00 22.43           O
ATOM   1649  CB   SER A 208      47.530  17.056  -7.445  1.00 25.79           C
ATOM   1650  OG   SER A 208      47.853  17.992  -6.415  1.00 30.50           O
ATOM   1651  N    PHE A 209      45.491  16.055  -9.525  1.00 23.47           N
ATOM   1652  CA   PHE A 209      45.533  15.285 -10.770  1.00 23.00           C
ATOM   1653  C    PHE A 209      45.929  16.186 -11.929  1.00 22.98           C
ATOM   1654  O    PHE A 209      45.815  17.396 -11.838  1.00 19.81           O
ATOM   1655  CB   PHE A 209      44.191  14.610 -11.039  1.00 22.83           C
ATOM   1656  CG   PHE A 209      43.088  15.565 -11.335  1.00 21.91           C
ATOM   1657  CD1  PHE A 209      42.850  16.003 -12.630  1.00 22.24           C
ATOM   1658  CD2  PHE A 209      42.290  16.035 -10.320  1.00 21.95           C
ATOM   1659  CE1  PHE A 209      41.835  16.899 -12.895  1.00 21.25           C
ATOM   1660  CE2  PHE A 209      41.272  16.922 -10.588  1.00 20.22           C
ATOM   1661  CZ   PHE A 209      41.051  17.349 -11.871  1.00 21.05           C
ATOM   1662  N    ASN A 210      46.391  15.594 -13.024  1.00 24.17           N
ATOM   1663  CA   ASN A 210      46.606  16.375 -14.263  1.00 24.44           C
ATOM   1664  C    ASN A 210      45.488  16.052 -15.245  1.00 23.93           C
ATOM   1665  O    ASN A 210      45.127  14.899 -15.405  1.00 21.59           O
ATOM   1666  CB   ASN A 210      47.969  16.063 -14.904  1.00 24.74           C
ATOM   1667  CG   ASN A 210      49.117  16.304 -13.971  1.00 24.27           C
ATOM   1668  OD1  ASN A 210      49.338  17.433 -13.526  1.00 29.32           O
ATOM   1669  ND2  ASN A 210      49.856  15.252 -13.660  1.00 26.01           N
ATOM   1670  N    ARG A 211      44.908  17.084 -15.845  1.00 24.90           N
ATOM   1671  CA   ARG A 211      43.951  16.896 -16.923  1.00 25.89           C
ATOM   1672  C    ARG A 211      44.680  16.335 -18.134  1.00 28.38           C
ATOM   1673  O    ARG A 211      44.376  15.227 -18.575  1.00 32.61           O
ATOM   1674  CB   ARG A 211      43.307  18.220 -17.322  1.00 26.22           C
ATOM   1675  CG   ARG A 211      42.361  18.047 -18.481  1.00 25.45           C
ATOM   1676  CD   ARG A 211      41.756  19.351 -18.884  1.00 28.00           C
ATOM   1677  NE   ARG A 211      42.709  20.284 -19.441  1.00 28.37           N
ATOM   1678  CZ   ARG A 211      43.292  20.153 -20.627  1.00 31.19           C
```

| ATOM | 1679 | NH1 | ARG | A | 211 | 43.066 | 19.090 | -21.396 | 1.00 | 32.81 | N |
|------|------|-----|-----|---|-----|--------|--------|---------|------|-------|---|
| ATOM | 1680 | NH2 | ARG | A | 211 | 44.132 | 21.090 | -21.044 | 1.00 | 32.38 | N |
| TER | 1681 | | ARG | A | 211 | | | | | | |
| ATOM | 1682 | N | GLU | B | 1 | 5.203 | -1.844 | 14.259 | 1.00 | 36.90 | N |
| ATOM | 1683 | CA | GLU | B | 1 | 4.355 | -1.096 | 15.234 | 1.00 | 35.54 | C |
| ATOM | 1684 | C | GLU | B | 1 | 3.559 | -0.031 | 14.467 | 1.00 | 33.78 | C |
| ATOM | 1685 | O | GLU | B | 1 | 2.977 | -0.309 | 13.425 | 1.00 | 33.23 | O |
| ATOM | 1686 | CB | GLU | B | 1 | 5.244 | -0.465 | 16.342 | 1.00 | 37.19 | C |
| ATOM | 1687 | CG | GLU | B | 1 | 4.512 | -0.108 | 17.670 | 1.00 | 38.07 | C |
| ATOM | 1688 | CD | GLU | B | 1 | 4.778 | 1.332 | 18.172 | 1.00 | 38.27 | C |
| ATOM | 1689 | OE1 | GLU | B | 1 | 5.532 | 2.070 | 17.487 | 1.00 | 41.49 | O |
| ATOM | 1690 | OE2 | GLU | B | 1 | 4.220 | 1.722 | 19.243 | 1.00 | 38.53 | O |
| ATOM | 1691 | N | VAL | B | 2 | 3.555 | 1.190 | 14.984 | 1.00 | 31.56 | N |
| ATOM | 1692 | CA | VAL | B | 2 | 2.854 | 2.300 | 14.378 | 1.00 | 29.45 | C |
| ATOM | 1693 | C | VAL | B | 2 | 3.727 | 2.849 | 13.268 | 1.00 | 28.64 | C |
| ATOM | 1694 | O | VAL | B | 2 | 4.932 | 3.059 | 13.471 | 1.00 | 28.88 | O |
| ATOM | 1695 | CB | VAL | B | 2 | 2.610 | 3.394 | 15.432 | 1.00 | 28.27 | C |
| ATOM | 1696 | CG1 | VAL | B | 2 | 2.020 | 4.659 | 14.816 | 1.00 | 27.73 | C |
| ATOM | 1697 | CG2 | VAL | B | 2 | 1.703 | 2.853 | 16.551 | 1.00 | 27.88 | C |
| ATOM | 1698 | N | LYS | B | 3 | 3.104 | 3.118 | 12.147 | 1.00 | 27.33 | N |
| ATOM | 1699 | CA | LYS | B | 3 | 3.790 | 3.735 | 11.023 | 1.00 | 26.78 | C |
| ATOM | 1700 | C | LYS | B | 3 | 2.992 | 4.916 | 10.564 | 1.00 | 24.93 | C |
| ATOM | 1701 | O | LYS | B | 3 | 1.787 | 4.804 | 10.363 | 1.00 | 23.59 | O |
| ATOM | 1702 | CB | LYS | B | 3 | 3.893 | 2.770 | 9.866 | 1.00 | 28.72 | C |
| ATOM | 1703 | CG | LYS | B | 3 | 4.450 | 3.388 | 8.625 | 1.00 | 29.64 | C |
| ATOM | 1704 | CD | LYS | B | 3 | 4.772 | 2.326 | 7.560 | 1.00 | 30.30 | C |
| ATOM | 1705 | CE | LYS | B | 3 | 5.378 | 3.047 | 6.348 | 1.00 | 32.31 | C |
| ATOM | 1706 | NZ | LYS | B | 3 | 6.696 | 3.688 | 6.710 | 1.00 | 34.69 | N |
| ATOM | 1707 | N | VAL | B | 4 | 3.669 | 6.052 | 10.382 | 1.00 | 23.91 | N |
| ATOM | 1708 | CA | VAL | B | 4 | 3.010 | 7.298 | 9.955 | 1.00 | 23.59 | C |
| ATOM | 1709 | C | VAL | B | 4 | 3.746 | 7.715 | 8.689 | 1.00 | 22.37 | C |
| ATOM | 1710 | O | VAL | B | 4 | 4.949 | 7.837 | 8.721 | 1.00 | 21.89 | O |
| ATOM | 1711 | CB | VAL | B | 4 | 3.145 | 8.409 | 11.017 | 1.00 | 23.03 | C |
| ATOM | 1712 | CG1 | VAL | B | 4 | 2.473 | 9.705 | 10.520 | 1.00 | 22.80 | C |
| ATOM | 1713 | CG2 | VAL | B | 4 | 2.569 | 7.955 | 12.368 | 1.00 | 25.35 | C |
| ATOM | 1714 | N | GLU | B | 5 | 3.038 | 7.918 | 7.579 | 1.00 | 22.93 | N |
| ATOM | 1715 | CA | GLU | B | 5 | 3.693 | 8.119 | 6.280 | 1.00 | 22.87 | C |
| ATOM | 1716 | C | GLU | B | 5 | 3.013 | 9.206 | 5.443 | 1.00 | 20.60 | C |
| ATOM | 1717 | O | GLU | B | 5 | 1.878 | 9.053 | 5.007 | 1.00 | 18.91 | O |
| ATOM | 1718 | CB | GLU | B | 5 | 3.711 | 6.803 | 5.502 | 1.00 | 24.45 | C |
| ATOM | 1719 | CG | GLU | B | 5 | 4.476 | 6.882 | 4.212 | 1.00 | 25.92 | C |
| ATOM | 1720 | CD | GLU | B | 5 | 4.247 | 5.675 | 3.313 | 1.00 | 29.48 | C |
| ATOM | 1721 | OE1 | GLU | B | 5 | 4.091 | 4.522 | 3.825 | 1.00 | 35.15 | O |
| ATOM | 1722 | OE2 | GLU | B | 5 | 4.216 | 5.885 | 2.075 | 1.00 | 35.53 | O |
| ATOM | 1723 | N | GLU | B | 6 | 3.732 | 10.303 | 5.236 | 1.00 | 19.76 | N |
| ATOM | 1724 | CA | GLU | B | 6 | 3.228 | 11.455 | 4.531 | 1.00 | 20.23 | C |
| ATOM | 1725 | C | GLU | B | 6 | 3.496 | 11.381 | 3.040 | 1.00 | 20.85 | C |
| ATOM | 1726 | O | GLU | B | 6 | 4.414 | 10.700 | 2.576 | 1.00 | 20.76 | O |
| ATOM | 1727 | CB | GLU | B | 6 | 3.841 | 12.758 | 5.096 | 1.00 | 19.91 | C |
| ATOM | 1728 | CG | GLU | B | 6 | 3.561 | 12.974 | 6.573 | 1.00 | 20.34 | C |
| ATOM | 1729 | CD | GLU | B | 6 | 4.673 | 12.501 | 7.451 | 1.00 | 20.98 | C |
| ATOM | 1730 | OE1 | GLU | B | 6 | 5.356 | 11.518 | 7.075 | 1.00 | 18.12 | O |
| ATOM | 1731 | OE2 | GLU | B | 6 | 4.886 | 13.120 | 8.513 | 1.00 | 21.12 | O |
| ATOM | 1732 | N | SER | B | 7 | 2.669 | 12.103 | 2.290 | 1.00 | 20.81 | N |
| ATOM | 1733 | CA | SER | B | 7 | 2.805 | 12.212 | 0.853 | 1.00 | 20.65 | C |
| ATOM | 1734 | C | SER | B | 7 | 2.126 | 13.485 | 0.401 | 1.00 | 18.69 | C |
| ATOM | 1735 | O | SER | B | 7 | 1.416 | 14.143 | 1.183 | 1.00 | 17.14 | O |
| ATOM | 1736 | CB | SER | B | 7 | 2.162 | 11.017 | 0.134 | 1.00 | 21.65 | C |
| ATOM | 1737 | OG | SER | B | 7 | 0.849 | 10.780 | 0.577 | 1.00 | 22.69 | O |
| ATOM | 1738 | N | GLY | B | 8 | 2.326 | 13.806 | -0.866 | 1.00 | 19.37 | N |
| ATOM | 1739 | CA | GLY | B | 8 | 1.622 | 14.917 | -1.487 | 1.00 | 19.52 | C |
| ATOM | 1740 | C | GLY | B | 8 | 2.409 | 16.216 | -1.589 | 1.00 | 19.79 | C |
| ATOM | 1741 | O | GLY | B | 8 | 1.915 | 17.196 | -2.164 | 1.00 | 20.15 | O |
| ATOM | 1742 | N | GLY | B | 9 | 3.622 | 16.244 | -1.054 | 1.00 | 20.76 | N |
| ATOM | 1743 | CA | GLY | B | 9 | 4.469 | 17.444 | -1.158 | 1.00 | 18.94 | C |

```
ATOM   1744  C    GLY B   9       5.020  17.702  -2.548  1.00 20.18           C
ATOM   1745  O    GLY B   9       4.834  16.908  -3.485  1.00 21.54           O
ATOM   1746  N    GLY B  10       5.674  18.841  -2.706  1.00 19.32           N
ATOM   1747  CA   GLY B  10       6.248  19.221  -3.996  1.00 19.01           C
ATOM   1748  C    GLY B  10       6.411  20.721  -4.084  1.00 19.26           C
ATOM   1749  O    GLY B  10       6.591  21.359  -3.062  1.00 18.54           O
ATOM   1750  N    LEU B  11       6.362  21.260  -5.302  1.00 16.84           N
ATOM   1751  CA   LEU B  11       6.624  22.687  -5.538  1.00 16.98           C
ATOM   1752  C    LEU B  11       5.328  23.371  -5.968  1.00 16.58           C
ATOM   1753  O    LEU B  11       4.564  22.815  -6.718  1.00 16.50           O
ATOM   1754  CB   LEU B  11       7.696  22.851  -6.624  1.00 16.03           C
ATOM   1755  CG   LEU B  11       8.032  24.285  -7.099  1.00 17.58           C
ATOM   1756  CD1  LEU B  11       8.652  25.104  -6.031  1.00 16.66           C
ATOM   1757  CD2  LEU B  11       8.977  24.221  -8.287  1.00 20.04           C
ATOM   1758  N    VAL B  12       5.084  24.588  -5.489  1.00 16.94           N
ATOM   1759  CA   VAL B  12       3.986  25.383  -6.006  1.00 18.44           C
ATOM   1760  C    VAL B  12       4.400  26.843  -5.936  1.00 18.59           C
ATOM   1761  O    VAL B  12       5.250  27.201  -5.151  1.00 17.20           O
ATOM   1762  CB   VAL B  12       2.650  25.144  -5.235  1.00 18.19           C
ATOM   1763  CG1  VAL B  12       2.685  25.718  -3.841  1.00 18.61           C
ATOM   1764  CG2  VAL B  12       1.449  25.690  -6.023  1.00 19.81           C
ATOM   1765  N    GLN B  13       3.766  27.646  -6.786  1.00 20.10           N
ATOM   1766  CA   GLN B  13       4.000  29.082  -6.923  1.00 20.65           C
ATOM   1767  C    GLN B  13       3.430  29.854  -5.727  1.00 18.96           C
ATOM   1768  O    GLN B  13       2.384  29.498  -5.205  1.00 16.17           O
ATOM   1769  CB   GLN B  13       3.302  29.557  -8.213  1.00 23.49           C
ATOM   1770  CG   GLN B  13       3.760  28.828  -9.520  1.00 25.93           C
ATOM   1771  CD   GLN B  13       3.151  27.405  -9.762  1.00 29.27           C
ATOM   1772  OE1  GLN B  13       2.295  26.917  -9.012  1.00 29.22           O
ATOM   1773  NE2  GLN B  13       3.629  26.747 -10.826  1.00 29.23           N
ATOM   1774  N    PRO B  14       4.106. 30.923  -5.286  1.00 20.25           N
ATOM   1775  CA   PRO B  14       3.428  31.760  -4.299  1.00 21.55           C
ATOM   1776  C    PRO B  14       1.980  32.057  -4.714  1.00 20.99           C
ATOM   1777  O    PRO B  14       1.687  32.222  -5.910  1.00 22.86           O
ATOM   1778  CB   PRO B  14       4.295  33.040  -4.233  1.00 22.24           C
ATOM   1779  CG   PRO B  14       5.472  32.820  -5.063  1.00 21.41           C
ATOM   1780  CD   PRO B  14       5.460  31.404  -5.596  1.00 20.95           C
ATOM   1781  N    GLY B  15       1.068  32.066  -3.745  1.00 22.50           N
ATOM   1782  CA   GLY B  15      -0.356  32.249  -4.010  1.00 22.21           C
ATOM   1783  C    GLY B  15      -1.085  30.977  -4.360  1.00 23.10           C
ATOM   1784  O    GLY B  15      -2.301  30.940  -4.344  1.00 23.11           O
ATOM   1785  N    GLY B  16      -0.345  29.914  -4.667  1.00 22.88           N
ATOM   1786  CA   GLY B  16      -0.956  28.648  -5.005  1.00 23.34           C
ATOM   1787  C    GLY B  16      -1.322  27.825  -3.795  1.00 23.36           C
ATOM   1788  O    GLY B  16      -1.228  28.280  -2.657  1.00 23.36           O
ATOM   1789  N    SER B  17      -1.721  26.588  -4.064  1.00 24.07           N
ATOM   1790  CA   SER B  17      -2.243  25.689  -3.058  1.00 24.72           C
ATOM   1791  C    SER B  17      -1.571  24.339  -3.113  1.00 24.68           C
ATOM   1792  O    SER B  17      -0.944  23.960  -4.110  1.00 22.80           O
ATOM   1793  CB   SER B  17      -3.739  25.454  -3.267  1.00 25.61           C
ATOM   1794  OG   SER B  17      -4.439  26.691  -3.204  1.00 30.25           O
ATOM   1795  N    MET B  18      -1.772  23.585  -2.049  1.00 23.75           N
ATOM   1796  CA   MET B  18      -1.257  22.247  -1.981  1.00 25.70           C
ATOM   1797  C    MET B  18      -2.080  21.456  -1.007  1.00 23.78           C
ATOM   1798  O    MET B  18      -2.589  22.008  -0.044  1.00 21.69           O
ATOM   1799  CB   MET B  18       0.186  22.317  -1.525  1.00 27.11           C
ATOM   1800  CG   MET B  18       0.851  20.989  -1.435  1.00 29.60           C
ATOM   1801  SD   MET B  18       2.561  21.142  -1.840  1.00 34.64           S
ATOM   1802  CE   MET B  18       2.513  21.630  -3.552  1.00 31.16           C
ATOM   1803  N    LYS B  19      -2.202  20.154  -1.238  1.00 22.93           N
ATOM   1804  CA   LYS B  19      -2.921  19.294  -0.313  1.00 22.52           C
ATOM   1805  C    LYS B  19      -2.020  18.112  -0.015  1.00 21.75           C
ATOM   1806  O    LYS B  19      -1.599  17.415  -0.927  1.00 22.24           O
ATOM   1807  CB   LYS B  19      -4.257  18.830  -0.890  1.00 23.70           C
ATOM   1808  CG   LYS B  19      -5.040  17.933   0.078  1.00 24.44           C
```

| ATOM | 1809 | CD | LYS | B | 19 | -6.506 | 17.782 | -0.285 | 1.00 | 24.96 | C |
|------|------|-----|-----|---|----|--------|--------|--------|------|-------|---|
| ATOM | 1810 | CE | LYS | B | 19 | -7.295 | 17.300 | 0.899 | 1.00 | 26.29 | C |
| ATOM | 1811 | NZ | LYS | B | 19 | -8.755 | 17.294 | 0.601 | 1.00 | 27.41 | N |
| ATOM | 1812 | N | ILE | B | 20 | -1.669 | 17.957 | 1.258 | 1.00 | 19.64 | N |
| ATOM | 1813 | CA | ILE | B | 20 | -0.812 | 16.852 | 1.692 | 1.00 | 18.59 | C |
| ATOM | 1814 | C | ILE | B | 20 | -1.616 | 15.909 | 2.546 | 1.00 | 16.33 | C |
| ATOM | 1815 | O | ILE | B | 20 | -2.680 | 16.239 | 3.016 | 1.00 | 14.22 | O |
| ATOM | 1816 | CB | ILE | B | 20 | 0.460 | 17.343 | 2.422 | 1.00 | 17.72 | C |
| ATOM | 1817 | CG1 | ILE | B | 20 | 0.153 | 18.204 | 3.656 | 1.00 | 18.88 | C |
| ATOM | 1818 | CG2 | ILE | B | 20 | 1.334 | 18.146 | 1.479 | 1.00 | 18.28 | C |
| ATOM | 1819 | CD1 | ILE | B | 20 | 1.429 | 18.712 | 4.323 | 1.00 | 17.86 | C |
| ATOM | 1820 | N | SER | B | 21 | -1.096 | 14.703 | 2.760 | 1.00 | 17.43 | N |
| ATOM | 1821 | CA | SER | B | 21 | -1.824 | 13.725 | 3.540 | 1.00 | 18.70 | C |
| ATOM | 1822 | C | SER | B | 21 | -0.836 | 12.786 | 4.187 | 1.00 | 16.80 | C |
| ATOM | 1823 | O | SER | B | 21 | 0.330 | 12.830 | 3.863 | 1.00 | 16.49 | O |
| ATOM | 1824 | CB | SER | B | 21 | -2.840 | 12.947 | 2.689 | 1.00 | 20.17 | C |
| ATOM | 1825 | OG | SER | B | 21 | -2.158 | 12.135 | 1.750 | 1.00 | 22.49 | O |
| ATOM | 1826 | N | CYS | B | 22 | -1.297 | 12.026 | 5.172 | 1.00 | 17.62 | N |
| ATOM | 1827 | CA | CYS | B | 22 | -0.546 | 10.892 | 5.693 | 1.00 | 20.02 | C |
| ATOM | 1828 | C | CYS | B | 22 | -1.489 | 9.771 | 6.040 | 1.00 | 21.14 | C |
| ATOM | 1829 | O | CYS | B | 22 | -2.636 | 9.992 | 6.403 | 1.00 | 18.09 | O |
| ATOM | 1830 | CB | CYS | B | 22 | 0.307 | 11.245 | 6.919 | 1.00 | 22.48 | C |
| ATOM | 1831 | SG | CYS | B | 22 | -0.578 | 11.789 | 8.362 | 1.00 | 27.12 | S |
| ATOM | 1832 | N | VAL | B | 23 | -0.979 | 8.559 | 5.923 | 1.00 | 21.79 | N |
| ATOM | 1833 | CA | VAL | B | 23 | -1.719 | 7.382 | 6.311 | 1.00 | 23.02 | C |
| ATOM | 1834 | C | VAL | B | 23 | -0.994 | 6.750 | 7.493 | 1.00 | 22.70 | C |
| ATOM | 1835 | O | VAL | B | 23 | 0.243 | 6.671 | 7.511 | 1.00 | 21.84 | O |
| ATOM | 1836 | CB | VAL | B | 23 | -1.884 | 6.402 | 5.120 | 1.00 | 23.64 | C |
| ATOM | 1837 | CG1 | VAL | B | 23 | -0.537 | 5.928 | 4.598 | 1.00 | 24.94 | C |
| ATOM | 1838 | CG2 | VAL | B | 23 | -2.746 | 5.218 | 5.521 | 1.00 | 23.69 | C |
| ATOM | 1839 | N | VAL | B | 24 | -1.767 | 6.344 | 8.498 | 1.00 | 22.20 | N |
| ATOM | 1840 | CA | VAL | B | 24 | -1.205 | 5.780 | 9.684 | 1.00 | 23.06 | C |
| ATOM | 1841 | C | VAL | B | 24 | -1.631 | 4.329 | 9.773 | 1.00 | 22.98 | C |
| ATOM | 1842 | O | VAL | B | 24 | -2.780 | 3.989 | 9.496 | 1.00 | 24.37 | O |
| ATOM | 1843 | CB | VAL | B | 24 | -1.676 | 6.524 | 10.936 | 1.00 | 22.08 | C |
| ATOM | 1844 | CG1 | VAL | B | 24 | -1.071 | 5.914 | 12.176 | 1.00 | 22.92 | C |
| ATOM | 1845 | CG2 | VAL | B | 24 | -1.319 | 8.005 | 10.832 | 1.00 | 20.93 | C |
| ATOM | 1846 | N | SER | B | 25 | -0.704 | 3.478 | 10.165 | 1.00 | 24.43 | N |
| ATOM | 1847 | CA | SER | B | 25 | -1.037 | 2.076 | 10.370 | 1.00 | 25.68 | C |
| ATOM | 1848 | C | SER | B | 25 | -0.452 | 1.617 | 11.693 | 1.00 | 26.27 | C |
| ATOM | 1849 | O | SER | B | 25 | 0.460 | 2.244 | 12.232 | 1.00 | 25.67 | O |
| ATOM | 1850 | CB | SER | B | 25 | -0.526 | 1.238 | 9.195 | 1.00 | 26.48 | C |
| ATOM | 1851 | OG | SER | B | 25 | 0.881 | 1.286 | 9.096 | 1.00 | 29.04 | O |
| ATOM | 1852 | N | GLY | B | 26 | -0.986 | 0.525 | 12.222 | 1.00 | 26.43 | N |
| ATOM | 1853 | CA | GLY | B | 26 | -0.463 | -0.066 | 13.443 | 1.00 | 26.79 | C |
| ATOM | 1854 | C | GLY | B | 26 | -1.128 | 0.475 | 14.689 | 1.00 | 26.80 | C |
| ATOM | 1855 | O | GLY | B | 26 | -0.739 | 0.131 | 15.806 | 1.00 | 27.84 | O |
| ATOM | 1856 | N | LEU | B | 27 | -2.117 | 1.332 | 14.504 | 1.00 | 26.12 | N |
| ATOM | 1857 | CA | LEU | B | 27 | -3.028 | 1.706 | 15.565 | 1.00 | 25.77 | C |
| ATOM | 1858 | C | LEU | B | 27 | -4.410 | 1.940 | 14.963 | 1.00 | 25.38 | C |
| ATOM | 1859 | O | LEU | B | 27 | -4.578 | 1.933 | 13.752 | 1.00 | 26.68 | O |
| ATOM | 1860 | CB | LEU | B | 27 | -2.529 | 2.941 | 16.340 | 1.00 | 27.34 | C |
| ATOM | 1861 | CG | LEU | B | 27 | -2.147 | 4.186 | 15.545 | 1.00 | 26.15 | C |
| ATOM | 1862 | CD1 | LEU | B | 27 | -3.366 | 4.850 | 14.899 | 1.00 | 25.55 | C |
| ATOM | 1863 | CD2 | LEU | B | 27 | -1.412 | 5.162 | 16.455 | 1.00 | 26.38 | C |
| ATOM | 1864 | N | THR | B | 28 | -5.407 | 2.114 | 15.815 | 1.00 | 24.83 | N |
| ATOM | 1865 | CA | THR | B | 28 | -6.762 | 2.414 | 15.376 | 1.00 | 24.38 | C |
| ATOM | 1866 | C | THR | B | 28 | -6.855 | 3.929 | 15.248 | 1.00 | 24.09 | C |
| ATOM | 1867 | O | THR | B | 28 | -7.046 | 4.632 | 16.242 | 1.00 | 23.68 | O |
| ATOM | 1868 | CB | THR | B | 28 | -7.787 | 1.884 | 16.374 | 1.00 | 24.60 | C |
| ATOM | 1869 | OG1 | THR | B | 28 | -7.616 | 0.458 | 16.525 | 1.00 | 25.20 | O |
| ATOM | 1870 | CG2 | THR | B | 28 | -9.178 | 2.183 | 15.915 | 1.00 | 23.82 | C |
| ATOM | 1871 | N | PHE | B | 29 | -6.710 | 4.396 | 14.007 | 1.00 | 24.04 | N |
| ATOM | 1872 | CA | PHE | B | 29 | -6.559 | 5.808 | 13.629 | 1.00 | 22.80 | C |
| ATOM | 1873 | C | PHE | B | 29 | -7.643 | 6.711 | 14.205 | 1.00 | 23.25 | C |

| ATOM | 1874 | O | PHE | B | 29 | -7.354 | 7.809 | 14.716 | 1.00 | 21.29 | O |
| ATOM | 1875 | CB | PHE | B | 29 | -6.488 | 5.886 | 12.078 | 1.00 | 23.10 | C |
| ATOM | 1876 | CG | PHE | B | 29 | -6.429 | 7.280 | 11.507 | 1.00 | 22.86 | C |
| ATOM | 1877 | CD1 | PHE | B | 29 | -5.213 | 7.876 | 11.221 | 1.00 | 22.70 | C |
| ATOM | 1878 | CD2 | PHE | B | 29 | -7.592 | 7.960 | 11.163 | 1.00 | 22.29 | C |
| ATOM | 1879 | CE1 | PHE | B | 29 | -5.150 | 9.148 | 10.686 | 1.00 | 23.35 | C |
| ATOM | 1880 | CE2 | PHE | B | 29 | -7.539 | 9.256 | 10.626 | 1.00 | 22.93 | C |
| ATOM | 1881 | CZ | PHE | B | 29 | -6.302 | 9.840 | 10.382 | 1.00 | 21.52 | C |
| ATOM | 1882 | N | SER | B | 30 | -8.889 | 6.256 | 14.118 | 1.00 | 22.94 | N |
| ATOM | 1883 | CA | SER | B | 30 | -10.043 | 7.000 | 14.603 | 1.00 | 22.89 | C |
| ATOM | 1884 | C | SER | B | 30 | -10.016 | 7.368 | 16.105 | 1.00 | 21.90 | C |
| ATOM | 1885 | O | SER | B | 30 | -10.812 | 8.199 | 16.559 | 1.00 | 21.84 | O |
| ATOM | 1886 | CB | SER | B | 30 | -11.308 | 6.186 | 14.289 | 1.00 | 23.02 | C |
| ATOM | 1887 | OG | SER | B | 30 | -11.097 | 4.813 | 14.629 | 1.00 | 26.55 | O |
| ATOM | 1888 | N | ASN | B | 31 | -9.129 | 6.732 | 16.868 | 1.00 | 21.39 | N |
| ATOM | 1889 | CA | ASN | B | 31 | -9.036 | 6.955 | 18.312 | 1.00 | 22.43 | C |
| ATOM | 1890 | C | ASN | B | 31 | -7.954 | 7.944 | 18.724 | 1.00 | 21.11 | C |
| ATOM | 1891 | O | ASN | B | 31 | -7.817 | 8.226 | 19.913 | 1.00 | 22.36 | O |
| ATOM | 1892 | CB | ASN | B | 31 | -8.782 | 5.624 | 19.057 | 1.00 | 23.13 | C |
| ATOM | 1893 | CG | ASN | B | 31 | -9.980 | 4.695 | 19.030 | 1.00 | 24.38 | C |
| ATOM | 1894 | OD1 | ASN | B | 31 | -9.831 | 3.473 | 19.097 | 1.00 | 28.23 | O |
| ATOM | 1895 | ND2 | ASN | B | 31 | -11.161 | 5.261 | 18.918 | 1.00 | 23.95 | N |
| ATOM | 1896 | N | TYR | B | 32 | -7.184 | 8.443 | 17.758 | 1.00 | 20.41 | N |
| ATOM | 1897 | CA | TYR | B | 32 | -6.082 | 9.361 | 18.038 | 1.00 | 20.79 | C |
| ATOM | 1898 | C | TYR | B | 32 | -6.299 | 10.787 | 17.544 | 1.00 | 19.75 | C |
| ATOM | 1899 | O | TYR | B | 32 | -6.867 | 11.024 | 16.468 | 1.00 | 20.39 | O |
| ATOM | 1900 | CB | TYR | B | 32 | -4.817 | 8.804 | 17.430 | 1.00 | 20.79 | C |
| ATOM | 1901 | CG | TYR | B | 32 | -4.331 | 7.632 | 18.195 | 1.00 | 21.80 | C |
| ATOM | 1902 | CD1 | TYR | B | 32 | -3.318 | 7.783 | 19.126 | 1.00 | 22.23 | C |
| ATOM | 1903 | CD2 | TYR | B | 32 | -4.911 | 6.370 | 18.034 | 1.00 | 19.86 | C |
| ATOM | 1904 | CE1 | TYR | B | 32 | -2.862 | 6.720 | 19.850 | 1.00 | 21.49 | C |
| ATOM | 1905 | CE2 | TYR | B | 32 | -4.460 | 5.291 | 18.775 | 1.00 | 21.65 | C |
| ATOM | 1906 | CZ | TYR | B | 32 | -3.424 | 5.477 | 19.675 | 1.00 | 20.90 | C |
| ATOM | 1907 | OH | TYR | B | 32 | -2.900 | 4.452 | 20.449 | 1.00 | 23.40 | O |
| ATOM | 1908 | N | TRP | B | 33 | -5.836 | 11.759 | 18.327 | 1.00 | 19.81 | N |
| ATOM | 1909 | CA | TRP | B | 33 | -5.788 | 13.110 | 17.840 | 1.00 | 18.10 | C |
| ATOM | 1910 | C | TRP | B | 33 | -4.695 | 13.208 | 16.768 | 1.00 | 17.15 | C |
| ATOM | 1911 | O | TRP | B | 33 | -3.715 | 12.463 | 16.776 | 1.00 | 16.36 | O |
| ATOM | 1912 | CB | TRP | B | 33 | -5.464 | 14.100 | 18.966 | 1.00 | 17.22 | C |
| ATOM | 1913 | CG | TRP | B | 33 | -6.460 | 14.144 | 20.057 | 1.00 | 16.36 | C |
| ATOM | 1914 | CD1 | TRP | B | 33 | -7.731 | 13.662 | 20.033 | 1.00 | 16.38 | C |
| ATOM | 1915 | CD2 | TRP | B | 33 | -6.298 | 14.789 | 21.316 | 1.00 | 15.69 | C |
| ATOM | 1916 | NE1 | TRP | B | 33 | -8.358 | 13.933 | 21.208 | 1.00 | 17.52 | N |
| ATOM | 1917 | CE2 | TRP | B | 33 | -7.503 | 14.631 | 22.019 | 1.00 | 16.24 | C |
| ATOM | 1918 | CE3 | TRP | B | 33 | -5.234 | 15.464 | 21.929 | 1.00 | 14.46 | C |
| ATOM | 1919 | CZ2 | TRP | B | 33 | -7.679 | 15.112 | 23.311 | 1.00 | 17.90 | C |
| ATOM | 1920 | CZ3 | TRP | B | 33 | -5.408 | 15.965 | 23.194 | 1.00 | 15.46 | C |
| ATOM | 1921 | CH2 | TRP | B | 33 | -6.608 | 15.774 | 23.886 | 1.00 | 18.04 | C |
| ATOM | 1922 | N | MET | B | 34 | -4.846 | 14.189 | 15.886 | 1.00 | 17.43 | N |
| ATOM | 1923 | CA | MET | B | 34 | -3.987 | 14.328 | 14.733 | 1.00 | 17.99 | C |
| ATOM | 1924 | C | MET | B | 34 | -3.569 | 15.771 | 14.555 | 1.00 | 17.21 | C |
| ATOM | 1925 | O | MET | B | 34 | -4.377 | 16.687 | 14.669 | 1.00 | 17.99 | O |
| ATOM | 1926 | CB | MET | B | 34 | -4.715 | 13.838 | 13.471 | 1.00 | 19.92 | C |
| ATOM | 1927 | CG | MET | B | 34 | -4.999 | 12.334 | 13.438 | 1.00 | 19.84 | C |
| ATOM | 1928 | SD | MET | B | 34 | -3.541 | 11.363 | 13.008 | 1.00 | 21.16 | S |
| ATOM | 1929 | CE | MET | B | 34 | -3.931 | 9.814 | 13.801 | 1.00 | 21.31 | C |
| ATOM | 1930 | N | SER | B | 35 | -2.289 | 15.973 | 14.258 | 1.00 | 17.74 | N |
| ATOM | 1931 | CA | SER | B | 35 | -1.751 | 17.288 | 14.061 | 1.00 | 16.33 | C |
| ATOM | 1932 | C | SER | B | 35 | -0.761 | 17.314 | 12.916 | 1.00 | 16.10 | C |
| ATOM | 1933 | O | SER | B | 35 | -0.261 | 16.308 | 12.511 | 1.00 | 13.69 | O |
| ATOM | 1934 | CB | SER | B | 35 | -1.049 | 17.824 | 15.324 | 1.00 | 17.52 | C |
| ATOM | 1935 | OG | SER | B | 35 | 0.199 | 17.148 | 15.569 | 1.00 | 14.23 | O |
| ATOM | 1936 | N | TRP | B | 36 | -0.489 | 18.520 | 12.426 | 1.00 | 13.89 | N |
| ATOM | 1937 | CA | TRP | B | 36 | 0.623 | 18.764 | 11.511 | 1.00 | 15.06 | C |
| ATOM | 1938 | C | TRP | B | 36 | 1.585 | 19.741 | 12.168 | 1.00 | 14.98 | C |

| | | | | | | | | | | | |
|---|---|---|---|---|---|---|---|---|---|---|---|
| ATOM | 1939 | O | TRP | B | 36 | 1.173 | 20.734 | 12.727 | 1.00 | 16.36 | O |
| ATOM | 1940 | CB | TRP | B | 36 | 0.153 | 19.367 | 10.199 | 1.00 | 16.63 | C |
| ATOM | 1941 | CG | TRP | B | 36 | -0.637 | 18.464 | 9.337 | 1.00 | 14.79 | C |
| ATOM | 1942 | CD1 | TRP | B | 36 | -1.971 | 18.338 | 9.304 | 1.00 | 16.75 | C |
| ATOM | 1943 | CD2 | TRP | B | 36 | -0.119 | 17.564 | 8.355 | 1.00 | 16.37 | C |
| ATOM | 1944 | NE1 | TRP | B | 36 | -2.341 | 17.413 | 8.352 | 1.00 | 16.27 | N |
| ATOM | 1945 | CE2 | TRP | B | 36 | -1.215 | 16.932 | 7.750 | 1.00 | 16.39 | C |
| ATOM | 1946 | CE3 | TRP | B | 36 | 1.174 | 17.256 | 7.904 | 1.00 | 16.27 | C |
| ATOM | 1947 | CZ2 | TRP | B | 36 | -1.059 | 15.979 | 6.739 | 1.00 | 17.52 | C |
| ATOM | 1948 | CZ3 | TRP | B | 36 | 1.325 | 16.328 | 6.887 | 1.00 | 16.96 | C |
| ATOM | 1949 | CH2 | TRP | B | 36 | 0.215 | 15.690 | 6.329 | 1.00 | 16.35 | C |
| ATOM | 1950 | N | VAL | B | 37 | 2.866 | 19.422 | 12.091 | 1.00 | 16.34 | N |
| ATOM | 1951 | CA | VAL | B | 37 | 3.951 | 20.235 | 12.622 | 1.00 | 15.12 | C |
| ATOM | 1952 | C | VAL | B | 37 | 4.933 | 20.391 | 11.484 | 1.00 | 14.98 | C |
| ATOM | 1953 | O | VAL | B | 37 | 5.358 | 19.384 | 10.895 | 1.00 | 14.97 | O |
| ATOM | 1954 | CB | VAL | B | 37 | 4.643 | 19.533 | 13.806 | 1.00 | 13.74 | C |
| ATOM | 1955 | CG1 | VAL | B | 37 | 5.984 | 20.223 | 14.233 | 1.00 | 14.49 | C |
| ATOM | 1956 | CG2 | VAL | B | 37 | 3.700 | 19.458 | 14.971 | 1.00 | 13.01 | C |
| ATOM | 1957 | N | ARG | B | 38 | 5.304 | 21.636 | 11.181 | 1.00 | 13.81 | N |
| ATOM | 1958 | CA | ARG | B | 38 | 6.275 | 21.893 | 10.121 | 1.00 | 15.00 | C |
| ATOM | 1959 | C | ARG | B | 38 | 7.624 | 22.295 | 10.680 | 1.00 | 15.13 | C |
| ATOM | 1960 | O | ARG | B | 38 | 7.724 | 22.745 | 11.823 | 1.00 | 16.80 | O |
| ATOM | 1961 | CB | ARG | B | 38 | 5.767 | 22.914 | 9.109 | 1.00 | 15.62 | C |
| ATOM | 1962 | CG | ARG | B | 38 | 5.612 | 24.282 | 9.619 | 1.00 | 16.23 | C |
| ATOM | 1963 | CD | ARG | B | 38 | 4.781 | 25.115 | 8.625 | 1.00 | 17.60 | C |
| ATOM | 1964 | NE | ARG | B | 38 | 4.659 | 26.472 | 9.124 | 1.00 | 17.16 | N |
| ATOM | 1965 | CZ | ARG | B | 38 | 4.052 | 27.469 | 8.494 | 1.00 | 17.37 | C |
| ATOM | 1966 | NH1 | ARG | B | 38 | 3.441 | 27.298 | 7.333 | 1.00 | 17.41 | N |
| ATOM | 1967 | NH2 | ARG | B | 38 | 4.037 | 28.667 | 9.068 | 1.00 | 20.17 | N |
| ATOM | 1968 | N | GLN | B | 39 | 8.675 | 22.056 | 9.906 | 1.00 | 16.08 | N |
| ATOM | 1969 | CA | GLN | B | 39 | 10.007 | 22.364 | 10.340 | 1.00 | 18.00 | C |
| ATOM | 1970 | C | GLN | B | 39 | 10.716 | 23.183 | 9.261 | 1.00 | 18.63 | C |
| ATOM | 1971 | O | GLN | B | 39 | 10.743 | 22.823 | 8.081 | 1.00 | 15.13 | O |
| ATOM | 1972 | CB | GLN | B | 39 | 10.777 | 21.092 | 10.711 | 1.00 | 18.23 | C |
| ATOM | 1973 | CG | GLN | B | 39 | 12.091 | 21.354 | 11.454 | 1.00 | 18.34 | C |
| ATOM | 1974 | CD | GLN | B | 39 | 12.798 | 20.081 | 11.881 | 1.00 | 20.85 | C |
| ATOM | 1975 | OE1 | GLN | B | 39 | 12.707 | 19.055 | 11.203 | 1.00 | 20.63 | O |
| ATOM | 1976 | NE2 | GLN | B | 39 | 13.485 | 20.130 | 13.033 | 1.00 | 21.38 | N |
| ATOM | 1977 | N | SER | B | 40 | 11.222 | 24.347 | 9.658 | 1.00 | 20.58 | N |
| ATOM | 1978 | CA | SER | B | 40 | 12.057 | 25.130 | 8.769 | 1.00 | 23.05 | C |
| ATOM | 1979 | C | SER | B | 40 | 13.260 | 25.564 | 9.574 | 1.00 | 24.89 | C |
| ATOM | 1980 | O | SER | B | 40 | 13.164 | 25.696 | 10.780 | 1.00 | 25.52 | O |
| ATOM | 1981 | CB | SER | B | 40 | 11.303 | 26.369 | 8.240 | 1.00 | 22.55 | C |
| ATOM | 1982 | OG | SER | B | 40 | 10.721 | 27.109 | 9.311 | 1.00 | 23.97 | O |
| ATOM | 1983 | N | PRO | B | 41 | 14.398 | 25.785 | 8.915 | 1.00 | 27.13 | N |
| ATOM | 1984 | CA | PRO | B | 41 | 15.531 | 26.315 | 9.670 | 1.00 | 27.97 | C |
| ATOM | 1985 | C | PRO | B | 41 | 15.185 | 27.644 | 10.380 | 1.00 | 28.77 | C |
| ATOM | 1986 | O | PRO | B | 41 | 15.693 | 27.920 | 11.461 | 1.00 | 29.03 | O |
| ATOM | 1987 | CB | PRO | B | 41 | 16.622 | 26.497 | 8.599 | 1.00 | 29.21 | C |
| ATOM | 1988 | CG | PRO | B | 41 | 16.191 | 25.660 | 7.434 | 1.00 | 29.11 | C |
| ATOM | 1989 | CD | PRO | B | 41 | 14.704 | 25.568 | 7.489 | 1.00 | 28.37 | C |
| ATOM | 1990 | N | GLU | B | 42 | 14.281 | 28.428 | 9.810 | 1.00 | 30.89 | N |
| ATOM | 1991 | CA | GLU | B | 42 | 14.011 | 29.772 | 10.318 | 1.00 | 30.31 | C |
| ATOM | 1992 | C | GLU | B | 42 | 13.157 | 29.777 | 11.577 | 1.00 | 30.45 | C |
| ATOM | 1993 | O | GLU | B | 42 | 13.373 | 30.593 | 12.483 | 1.00 | 30.99 | O |
| ATOM | 1994 | CB | GLU | B | 42 | 13.334 | 30.603 | 9.227 | 1.00 | 32.45 | C |
| ATOM | 1995 | CG | GLU | B | 42 | 12.942 | 32.025 | 9.633 | 1.00 | 34.63 | C |
| ATOM | 1996 | CD | GLU | B | 42 | 14.131 | 32.936 | 9.825 | 1.00 | 39.59 | C |
| ATOM | 1997 | OE1 | GLU | B | 42 | 15.284 | 32.494 | 9.569 | 1.00 | 43.78 | O |
| ATOM | 1998 | OE2 | GLU | B | 42 | 13.915 | 34.102 | 10.234 | 1.00 | 42.16 | O |
| ATOM | 1999 | N | LYS | B | 43 | 12.190 | 28.870 | 11.627 | 1.00 | 28.83 | N |
| ATOM | 2000 | CA | LYS | B | 43 | 11.254 | 28.816 | 12.732 | 1.00 | 27.58 | C |
| ATOM | 2001 | C | LYS | B | 43 | 11.351 | 27.549 | 13.588 | 1.00 | 26.13 | C |
| ATOM | 2002 | O | LYS | B | 43 | 10.670 | 27.452 | 14.611 | 1.00 | 26.12 | O |
| ATOM | 2003 | CB | LYS | B | 43 | 9.834 | 28.977 | 12.195 | 1.00 | 28.64 | C |

| ATOM | 2004 | CG | LYS | B | 43 | 9.565 | 30.363 | 11.577 | 1.00 | 31.09 | C |
|---|---|---|---|---|---|---|---|---|---|---|---|
| ATOM | 2005 | CD | LYS | B | 43 | 9.435 | 31.448 | 12.638 | 1.00 | 33.00 | C |
| ATOM | 2006 | CE | LYS | B | 43 | 9.470 | 32.883 | 12.040 | 1.00 | 33.48 | C |
| ATOM | 2007 | NZ | LYS | B | 43 | 10.350 | 33.797 | 12.850 | 1.00 | 34.57 | N |
| ATOM | 2008 | N | GLY | B | 44 | 12.192 | 26.594 | 13.190 | 1.00 | 22.91 | N |
| ATOM | 2009 | CA | GLY | B | 44 | 12.261 | 25.302 | 13.886 | 1.00 | 21.55 | C |
| ATOM | 2010 | C | GLY | B | 44 | 10.958 | 24.543 | 13.734 | 1.00 | 20.58 | C |
| ATOM | 2011 | O | GLY | B | 44 | 10.291 | 24.669 | 12.689 | 1.00 | 17.18 | O |
| ATOM | 2012 | N | LEU | B | 45 | 10.601 | 23.791 | 14.785 | 1.00 | 19.68 | N |
| ATOM | 2013 | CA | LEU | B | 45 | 9.368 | 23.033 | 14.847 | 1.00 | 18.71 | C |
| ATOM | 2014 | C | LEU | B | 45 | 8.188 | 23.949 | 15.145 | 1.00 | 18.42 | C |
| ATOM | 2015 | O | LEU | B | 45 | 8.169 | 24.653 | 16.162 | 1.00 | 15.97 | O |
| ATOM | 2016 | CB | LEU | B | 45 | 9.445 | 21.967 | 15.934 | 1.00 | 18.97 | C |
| ATOM | 2017 | CG | LEU | B | 45 | 10.365 | 20.790 | 15.638 | 1.00 | 18.05 | C |
| ATOM | 2018 | CD1 | LEU | B | 45 | 10.493 | 19.934 | 16.874 | 1.00 | 18.45 | C |
| ATOM | 2019 | CD2 | LEU | B | 45 | 9.849 | 19.974 | 14.435 | 1.00 | 18.12 | C |
| ATOM | 2020 | N | GLU | B | 46 | 7.195 | 23.895 | 14.272 | 1.00 | 16.49 | N |
| ATOM | 2021 | CA | GLU | B | 46 | 6.048 | 24.745 | 14.355 | 1.00 | 18.16 | C |
| ATOM | 2022 | C | GLU | B | 46 | 4.733 | 23.952 | 14.153 | 1.00 | 15.57 | C |
| ATOM | 2023 | O | GLU | B | 46 | 4.429 | 23.525 | 13.053 | 1.00 | 13.82 | O |
| ATOM | 2024 | CB | GLU | B | 46 | 6.215 | 25.799 | 13.276 | 1.00 | 17.34 | C |
| ATOM | 2025 | CG | GLU | B | 46 | 5.384 | 27.030 | 13.460 | 1.00 | 22.34 | C |
| ATOM | 2026 | CD | GLU | B | 46 | 5.416 | 27.949 | 12.240 | 1.00 | 23.11 | C |
| ATOM | 2027 | OE1 | GLU | B | 46 | 6.243 | 27.731 | 11.318 | 1.00 | 25.75 | O |
| ATOM | 2028 | OE2 | GLU | B | 46 | 4.583 | 28.892 | 12.205 | 1.00 | 29.39 | O |
| ATOM | 2029 | N | TRP | B | 47 | 3.971 | 23.765 | 15.229 | 1.00 | 15.88 | N |
| ATOM | 2030 | CA | TRP | B | 47 | 2.637 | 23.171 | 15.170 | 1.00 | 15.32 | C |
| ATOM | 2031 | C | TRP | B | 47 | 1.742 | 24.115 | 14.398 | 1.00 | 15.78 | C |
| ATOM | 2032 | O | TRP | B | 47 | 1.767 | 25.333 | 14.643 | 1.00 | 16.15 | O |
| ATOM | 2033 | CB | TRP | B | 47 | 2.140 | 22.964 | 16.595 | 1.00 | 15.67 | C |
| ATOM | 2034 | CG | TRP | B | 47 | 0.736 | 22.527 | 16.790 | 1.00 | 14.75 | C |
| ATOM | 2035 | CD1 | TRP | B | 47 | 0.274 | 21.243 | 16.892 | 1.00 | 15.00 | C |
| ATOM | 2036 | CD2 | TRP | B | 47 | -0.402 | 23.377 | 16.983 | 1.00 | 13.01 | C |
| ATOM | 2037 | NE1 | TRP | B | 47 | -1.067 | 21.248 | 17.113 | 1.00 | 14.19 | N |
| ATOM | 2038 | CE2 | TRP | B | 47 | -1.512 | 22.544 | 17.170 | 1.00 | 15.31 | C |
| ATOM | 2039 | CE3 | TRP | B | 47 | -0.585 | 24.761 | 17.002 | 1.00 | 15.14 | C |
| ATOM | 2040 | CZ2 | TRP | B | 47 | -2.795 | 23.049 | 17.394 | 1.00 | 15.20 | C |
| ATOM | 2041 | CZ3 | TRP | B | 47 | -1.839 | 25.261 | 17.228 | 1.00 | 14.14 | C |
| ATOM | 2042 | CH2 | TRP | B | 47 | -2.938 | 24.405 | 17.412 | 1.00 | 16.15 | C |
| ATOM | 2043 | N | VAL | B | 48 | 0.989 | 23.591 | 13.438 | 1.00 | 14.89 | N |
| ATOM | 2044 | CA | VAL | B | 48 | 0.137 | 24.446 | 12.604 | 1.00 | 15.02 | C |
| ATOM | 2045 | C | VAL | B | 48 | -1.338 | 24.098 | 12.614 | 1.00 | 14.70 | C |
| ATOM | 2046 | O | VAL | B | 48 | -2.151 | 24.972 | 12.452 | 1.00 | 15.00 | O |
| ATOM | 2047 | CB | VAL | B | 48 | 0.632 | 24.562 | 11.152 | 1.00 | 14.87 | C |
| ATOM | 2048 | CG1 | VAL | B | 48 | 1.936 | 25.338 | 11.090 | 1.00 | 15.84 | C |
| ATOM | 2049 | CG2 | VAL | B | 48 | 0.727 | 23.154 | 10.510 | 1.00 | 14.94 | C |
| ATOM | 2050 | N | ALA | B | 49 | -1.705 | 22.845 | 12.852 | 1.00 | 13.79 | N |
| ATOM | 2051 | CA | ALA | B | 49 | -3.116 | 22.482 | 12.808 | 1.00 | 14.82 | C |
| ATOM | 2052 | C | ALA | B | 49 | -3.331 | 21.174 | 13.544 | 1.00 | 15.37 | C |
| ATOM | 2053 | O | ALA | B | 49 | -2.442 | 20.344 | 13.543 | 1.00 | 13.91 | O |
| ATOM | 2054 | CB | ALA | B | 49 | -3.575 | 22.381 | 11.353 | 1.00 | 15.77 | C |
| ATOM | 2055 | N | GLU | B | 50 | -4.489 | 21.018 | 14.187 | 1.00 | 14.97 | N |
| ATOM | 2056 | CA | GLU | B | 50 | -4.847 | 19.767 | 14.894 | 1.00 | 15.44 | C |
| ATOM | 2057 | C | GLU | B | 50 | -6.324 | 19.442 | 14.743 | 1.00 | 15.86 | C |
| ATOM | 2058 | O | GLU | B | 50 | -7.163 | 20.334 | 14.660 | 1.00 | 16.32 | O |
| ATOM | 2059 | CB | GLU | B | 50 | -4.506 | 19.895 | 16.376 | 1.00 | 14.65 | C |
| ATOM | 2060 | CG | GLU | B | 50 | -4.716 | 18.662 | 17.191 | 1.00 | 14.65 | C |
| ATOM | 2061 | CD | GLU | B | 50 | -3.882 | 18.652 | 18.455 | 1.00 | 16.91 | C |
| ATOM | 2062 | OE1 | GLU | B | 50 | -2.717 | 19.073 | 18.382 | 1.00 | 16.32 | O |
| ATOM | 2063 | OE2 | GLU | B | 50 | -4.397 | 18.241 | 19.532 | 1.00 | 17.24 | O |
| ATOM | 2064 | N | ILE | B | 51 | -6.643 | 18.146 | 14.703 | 1.00 | 16.40 | N |
| ATOM | 2065 | CA | ILE | B | 51 | -8.026 | 17.697 | 14.601 | 1.00 | 16.59 | C |
| ATOM | 2066 | C | ILE | B | 51 | -8.266 | 16.596 | 15.635 | 1.00 | 17.29 | C |
| ATOM | 2067 | O | ILE | B | 51 | -7.425 | 15.727 | 15.843 | 1.00 | 18.07 | O |
| ATOM | 2068 | CB | ILE | B | 51 | -8.390 | 17.225 | 13.159 | 1.00 | 15.92 | C |

```
ATOM   2069  CG1 ILE B   51       -9.868  16.866  13.058  1.00 16.90           C
ATOM   2070  CG2 ILE B   51       -7.472  16.076  12.687  1.00 16.10           C
ATOM   2071  CD1 ILE B   51      -10.397  16.910  11.625  1.00 15.95           C
ATOM   2072  N   ARG B   52       -9.412  16.679  16.302  1.00 17.40           N
ATOM   2073  CA  ARG B   52       -9.778  15.767  17.367  1.00 17.80           C
ATOM   2074  C   ARG B   52      -10.664  14.685  16.774  1.00 18.21           C
ATOM   2075  O   ARG B   52      -10.565  14.409  15.579  1.00 17.35           O
ATOM   2076  CB  ARG B   52      -10.429  16.546  18.514  1.00 17.82           C
ATOM   2077  CG  ARG B   52       -9.513  17.644  19.042  1.00 18.16           C
ATOM   2078  CD  ARG B   52       -8.240  17.034  19.628  1.00 19.43           C
ATOM   2079  NE  ARG B   52       -7.234  18.000  20.075  1.00 18.03           N
ATOM   2080  CZ  ARG B   52       -7.262  18.663  21.232  1.00 18.92           C
ATOM   2081  NH1 ARG B   52       -8.268  18.511  22.088  1.00 19.58           N
ATOM   2082  NH2 ARG B   52       -6.273  19.500  21.533  1.00 16.35           N
ATOM   2083  N   LEU B   52A     -11.513  14.060  17.587  1.00 19.36           N
ATOM   2084  CA  LEU B   52A     -12.166  12.830  17.164  1.00 18.70           C
ATOM   2085  C   LEU B   52A     -13.578  13.036  16.679  1.00 18.59           C
ATOM   2086  O   LEU B   52A     -14.163  14.084  16.856  1.00 17.30           O
ATOM   2087  CB  LEU B   52A     -12.154  11.815  18.308  1.00 19.03           C
ATOM   2088  CG  LEU B   52A     -10.795  11.572  18.971  1.00 17.59           C
ATOM   2089  CD1 LEU B   52A     -10.960  10.551  20.023  1.00 17.93           C
ATOM   2090  CD2 LEU B   52A      -9.758  11.128  17.983  1.00 15.63           C
ATOM   2091  N   LYS B   52B     -14.137  11.990  16.092  1.00 19.22           N
ATOM   2092  CA  LYS B   52B     -15.553  11.972  15.721  1.00 20.18           C
ATOM   2093  C   LYS B   52B     -16.472  12.387  16.894  1.00 21.39           C
ATOM   2094  O   LYS B   52B     -17.425  13.138  16.692  1.00 23.50           O
ATOM   2095  CB  LYS B   52B     -15.943  10.573  15.242  1.00 20.27           C
ATOM   2096  CG  LYS B   52B     -17.348  10.515  14.657  1.00 21.65           C
ATOM   2097  CD  LYS B   52B     -17.639   9.138  14.056  1.00 21.65           C
ATOM   2098  CE  LYS B   52B     -19.129   8.867  14.039  1.00 25.09           C
ATOM   2099  NZ  LYS B   52B     -19.487   7.618  13.268  1.00 26.36           N
ATOM   2100  N   SER B   52C     -16.177  11.936  18.116  1.00 21.94           N
ATOM   2101  CA  SER B   52C     -16.945  12.354  19.314  1.00 22.53           C
ATOM   2102  C   SER B   52C     -16.836  13.842  19.671  1.00 23.09           C
ATOM   2103  O   SER B   52C     -17.632  14.360  20.467  1.00 23.11           O
ATOM   2104  CB  SER B   52C     -16.536  11.507  20.531  1.00 24.39           C
ATOM   2105  OG  SER B   52C     -15.151  11.164  20.522  1.00 26.74           O
ATOM   2106  N   ASP B   53      -15.846  14.526  19.106  1.00 21.41           N
ATOM   2107  CA  ASP B   53      -15.622  15.946  19.376  1.00 20.81           C
ATOM   2108  C   ASP B   53      -16.140  16.786  18.220  1.00 19.90           C
ATOM   2109  O   ASP B   53      -15.763  17.960  18.047  1.00 21.25           O
ATOM   2110  CB  ASP B   53      -14.124  16.195  19.588  1.00 20.17           C
ATOM   2111  CG  ASP B   53      -13.528  15.260  20.607  1.00 20.67           C
ATOM   2112  OD1 ASP B   53      -13.984  15.316  21.767  1.00 18.83           O
ATOM   2113  OD2 ASP B   53      -12.640  14.451  20.238  1.00 18.03           O
ATOM   2114  N   ASN B   54      -17.027  16.194  17.430  1.00 20.09           N
ATOM   2115  CA  ASN B   54      -17.441  16.746  16.129  1.00 18.79           C
ATOM   2116  C   ASN B   54      -16.235  17.140  15.285  1.00 17.04           C
ATOM   2117  O   ASN B   54      -16.225  18.234  14.685  1.00 16.95           O
ATOM   2118  CB  ASN B   54      -18.368  17.969  16.281  1.00 19.50           C
ATOM   2119  CG  ASN B   54      -19.662  17.648  16.974  1.00 21.80           C
ATOM   2120  OD1 ASN B   54      -20.199  18.480  17.720  1.00 26.49           O
ATOM   2121  ND2 ASN B   54      -20.176  16.463  16.736  1.00 18.32           N
ATOM   2122  N   TYR B   55      -15.204  16.293  15.303  1.00 15.74           N
ATOM   2123  CA  TYR B   55      -13.998  16.529  14.558  1.00 17.56           C
ATOM   2124  C   TYR B   55      -13.487  17.973  14.744  1.00 16.79           C
ATOM   2125  O   TYR B   55      -13.002  18.624  13.792  1.00 17.62           O
ATOM   2126  CB  TYR B   55      -14.249  16.237  13.086  1.00 18.64           C
ATOM   2127  CG  TYR B   55      -14.445  14.784  12.768  1.00 19.96           C
ATOM   2128  CD1 TYR B   55      -13.454  13.865  13.036  1.00 20.49           C
ATOM   2129  CD2 TYR B   55      -15.625  14.332  12.161  1.00 20.20           C
ATOM   2130  CE1 TYR B   55      -13.615  12.533  12.731  1.00 20.74           C
ATOM   2131  CE2 TYR B   55      -15.799  13.007  11.858  1.00 20.22           C
ATOM   2132  CZ  TYR B   55      -14.797  12.109  12.135  1.00 20.45           C
ATOM   2133  OH  TYR B   55      -14.975  10.782  11.807  1.00 21.10           O
```

| ATOM | 2134 | N | ALA | B | 56 | -13.557 | 18.446 | 15.990 | 1.00 | 16.49 | N |
|------|------|------|-----|---|----|---------|--------|--------|------|-------|---|
| ATOM | 2135 | CA | ALA | B | 56 | -13.029 | 19.798 | 16.341 | 1.00 | 16.77 | C |
| ATOM | 2136 | C | ALA | B | 56 | -11.609 | 20.070 | 15.806 | 1.00 | 16.65 | C |
| ATOM | 2137 | O | ALA | B | 56 | -10.741 | 19.211 | 15.843 | 1.00 | 15.07 | O |
| ATOM | 2138 | CB | ALA | B | 56 | -13.050 | 19.998 | 17.874 | 1.00 | 17.55 | C |
| ATOM | 2139 | N | THR | B | 57 | -11.382 | 21.305 | 15.332 | 1.00 | 16.05 | N |
| ATOM | 2140 | CA | THR | B | 57 | -10.096 | 21.688 | 14.713 | 1.00 | 17.36 | C |
| ATOM | 2141 | C | THR | B | 57 | -9.529 | 22.936 | 15.363 | 1.00 | 18.09 | C |
| ATOM | 2142 | O | THR | B | 57 | -10.284 | 23.760 | 15.903 | 1.00 | 17.95 | O |
| ATOM | 2143 | CB | THR | B | 57 | -10.212 | 21.960 | 13.199 | 1.00 | 17.76 | C |
| ATOM | 2144 | OG1 | THR | B | 57 | -11.210 | 22.960 | 12.957 | 1.00 | 14.16 | O |
| ATOM | 2145 | CG2 | THR | B | 57 | -10.622 | 20.731 | 12.498 | 1.00 | 15.56 | C |
| ATOM | 2146 | N | TYR | B | 58 | -8.202 | 23.029 | 15.308 | 1.00 | 17.75 | N |
| ATOM | 2147 | CA | TYR | B | 58 | -7.414 | 24.097 | 15.923 | 1.00 | 17.68 | C |
| ATOM | 2148 | C | TYR | B | 58 | -6.232 | 24.426 | 15.025 | 1.00 | 18.22 | C |
| ATOM | 2149 | O | TYR | B | 58 | -5.621 | 23.539 | 14.431 | 1.00 | 16.47 | O |
| ATOM | 2150 | CB | TYR | B | 58 | -6.926 | 23.661 | 17.316 | 1.00 | 18.98 | C |
| ATOM | 2151 | CG | TYR | B | 58 | -8.034 | 23.146 | 18.194 | 1.00 | 19.43 | C |
| ATOM | 2152 | CD1 | TYR | B | 58 | -8.295 | 21.783 | 18.305 | 1.00 | 17.97 | C |
| ATOM | 2153 | CD2 | TYR | B | 58 | -8.875 | 24.019 | 18.850 | 1.00 | 20.12 | C |
| ATOM | 2154 | CE1 | TYR | B | 58 | -9.326 | 21.334 | 19.088 | 1.00 | 19.50 | C |
| ATOM | 2155 | CE2 | TYR | B | 58 | -9.931 | 23.553 | 19.637 | 1.00 | 19.13 | C |
| ATOM | 2156 | CZ | TYR | B | 58 | -10.145 | 22.215 | 19.742 | 1.00 | 18.87 | C |
| ATOM | 2157 | OH | TYR | B | 58 | -11.191 | 21.735 | 20.519 | 1.00 | 21.70 | O |
| ATOM | 2158 | N | TYR | B | 59 | -5.897 | 25.712 | 14.924 | 1.00 | 16.40 | N |
| ATOM | 2159 | CA | TYR | B | 59 | -4.863 | 26.176 | 14.014 | 1.00 | 18.42 | C |
| ATOM | 2160 | C | TYR | B | 59 | -3.941 | 27.166 | 14.684 | 1.00 | 19.87 | C |
| ATOM | 2161 | O | TYR | B | 59 | -4.377 | 27.877 | 15.587 | 1.00 | 19.69 | O |
| ATOM | 2162 | CB | TYR | B | 59 | -5.488 | 26.900 | 12.818 | 1.00 | 17.47 | C |
| ATOM | 2163 | CG | TYR | B | 59 | -6.338 | 25.996 | 12.003 | 1.00 | 17.46 | C |
| ATOM | 2164 | CD1 | TYR | B | 59 | -5.782 | 25.195 | 11.019 | 1.00 | 15.69 | C |
| ATOM | 2165 | CD2 | TYR | B | 59 | -7.702 | 25.902 | 12.254 | 1.00 | 17.45 | C |
| ATOM | 2166 | CE1 | TYR | B | 59 | -6.575 | 24.343 | 10.292 | 1.00 | 15.98 | C |
| ATOM | 2167 | CE2 | TYR | B | 59 | -8.495 | 25.050 | 11.545 | 1.00 | 16.59 | C |
| ATOM | 2168 | CZ | TYR | B | 59 | -7.931 | 24.272 | 10.562 | 1.00 | 16.43 | C |
| ATOM | 2169 | OH | TYR | B | 59 | -8.771 | 23.427 | 9.886 | 1.00 | 16.79 | O |
| ATOM | 2170 | N | ALA | B | 60 | -2.688 | 27.232 | 14.228 | 1.00 | 19.53 | N |
| ATOM | 2171 | CA | ALA | B | 60 | -1.788 | 28.285 | 14.674 | 1.00 | 20.35 | C |
| ATOM | 2172 | C | ALA | B | 60 | -2.277 | 29.589 | 14.082 | 1.00 | 21.41 | C |
| ATOM | 2173 | O | ALA | B | 60 | -2.821 | 29.615 | 12.982 | 1.00 | 20.96 | O |
| ATOM | 2174 | CB | ALA | B | 60 | -0.374 | 28.017 | 14.242 | 1.00 | 19.74 | C |
| ATOM | 2175 | N | GLU | B | 61 | -2.069 | 30.677 | 14.810 | 1.00 | 24.86 | N |
| ATOM | 2176 | CA | GLU | B | 61 | -2.574 | 31.960 | 14.351 | 1.00 | 26.12 | C |
| ATOM | 2177 | C | GLU | B | 61 | -2.014 | 32.295 | 12.971 | 1.00 | 26.30 | C |
| ATOM | 2178 | O | GLU | B | 61 | -2.736 | 32.827 | 12.137 | 1.00 | 26.85 | O |
| ATOM | 2179 | CB | GLU | B | 61 | -2.283 | 33.073 | 15.364 | 1.00 | 27.69 | C |
| ATOM | 2180 | CG | GLU | B | 61 | -3.317 | 34.209 | 15.324 | 1.00 | 31.19 | C |
| ATOM | 2181 | CD | GLU | B | 61 | -4.769 | 33.711 | 15.175 | 1.00 | 36.21 | C |
| ATOM | 2182 | OE1 | GLU | B | 61 | -5.395 | 33.342 | 16.209 | 1.00 | 39.14 | O |
| ATOM | 2183 | OE2 | GLU | B | 61 | -5.280 | 33.695 | 14.015 | 1.00 | 40.91 | O |
| ATOM | 2184 | N | SER | B | 62 | -0.754 | 31.930 | 12.722 | 1.00 | 27.05 | N |
| ATOM | 2185 | CA | SER | B | 62 | -0.089 | 32.239 | 11.458 | 1.00 | 28.19 | C |
| ATOM | 2186 | C | SER | B | 62 | -0.682 | 31.572 | 10.209 | 1.00 | 29.60 | C |
| ATOM | 2187 | O | SER | B | 62 | -0.349 | 31.977 | 9.086 | 1.00 | 31.53 | O |
| ATOM | 2188 | CB | SER | B | 62 | 1.395 | 31.896 | 11.536 | 1.00 | 28.91 | C |
| ATOM | 2189 | OG | SER | B | 62 | 1.608 | 30.483 | 11.538 | 1.00 | 31.21 | O |
| ATOM | 2190 | N | VAL | B | 63 | -1.556 | 30.579 | 10.383 | 1.00 | 28.66 | N |
| ATOM | 2191 | CA | VAL | B | 63 | -2.158 | 29.868 | 9.251 | 1.00 | 28.52 | C |
| ATOM | 2192 | C | VAL | B | 63 | -3.694 | 29.801 | 9.267 | 1.00 | 28.98 | C |
| ATOM | 2193 | O | VAL | B | 63 | -4.303 | 29.250 | 8.325 | 1.00 | 28.67 | O |
| ATOM | 2194 | CB | VAL | B | 63 | -1.613 | 28.434 | 9.183 | 1.00 | 28.48 | C |
| ATOM | 2195 | CG1 | VAL | B | 63 | -0.101 | 28.446 | 9.256 | 1.00 | 27.38 | C |
| ATOM | 2196 | CG2 | VAL | B | 63 | -2.181 | 27.584 | 10.314 | 1.00 | 29.24 | C |
| ATOM | 2197 | N | LYS | B | 64 | -4.339 | 30.320 | 10.321 | 1.00 | 28.76 | N |
| ATOM | 2198 | CA | LYS | B | 64 | -5.802 | 30.320 | 10.354 | 1.00 | 29.59 | C |

```
ATOM   2199  C    LYS B  64    -6.293  31.045   9.110  1.00 28.62          C
ATOM   2200  O    LYS B  64    -5.699  32.042   8.677  1.00 26.82          O
ATOM   2201  CB   LYS B  64    -6.380  31.001  11.594  1.00 31.13          C
ATOM   2202  CG   LYS B  64    -5.895  30.464  12.909  1.00 33.75          C
ATOM   2203  CD   LYS B  64    -7.009  30.535  13.996  1.00 33.47          C
ATOM   2204  CE   LYS B  64    -6.463  30.150  15.382  1.00 34.24          C
ATOM   2205  NZ   LYS B  64    -7.508  30.223  16.460  1.00 36.47          N
ATOM   2206  N    GLY B  65    -7.350  30.502   8.510  1.00 28.46          N
ATOM   2207  CA   GLY B  65    -7.876  31.023   7.257  1.00 27.91          C
ATOM   2208  C    GLY B  65    -7.199  30.449   6.022  1.00 28.00          C
ATOM   2209  O    GLY B  65    -7.826  30.364   4.966  1.00 28.55          O
ATOM   2210  N    LYS B  66    -5.929  30.053   6.141  1.00 26.12          N
ATOM   2211  CA   LYS B  66    -5.170  29.537   5.001  1.00 25.47          C
ATOM   2212  C    LYS B  66    -5.204  28.011   4.920  1.00 24.31          C
ATOM   2213  O    LYS B  66    -5.209  27.436   3.820  1.00 23.57          O
ATOM   2214  CB   LYS B  66    -3.706  30.015   5.079  1.00 27.06          C
ATOM   2215  CG   LYS B  66    -3.369  31.160   4.157  1.00 27.45          C
ATOM   2216  CD   LYS B  66    -2.060  31.807   4.529  1.00 28.36          C
ATOM   2217  CE   LYS B  66    -0.874  30.882   4.315  1.00 28.55          C
ATOM   2218  NZ   LYS B  66     0.444  31.543   4.529  1.00 31.66          N
ATOM   2219  N    PHE B  67    -5.200  27.355   6.075  1.00 22.16          N
ATOM   2220  CA   PHE B  67    -5.045  25.908   6.138  1.00 21.29          C
ATOM   2221  C    PHE B  67    -6.317  25.259   6.675  1.00 21.22          C
ATOM   2222  O    PHE B  67    -6.979  25.817   7.561  1.00 20.62          O
ATOM   2223  CB   PHE B  67    -3.907  25.547   7.092  1.00 19.83          C
ATOM   2224  CG   PHE B  67    -2.520  25.777   6.575  1.00 19.30          C
ATOM   2225  CD1  PHE B  67    -2.244  26.505   5.414  1.00 19.81          C
ATOM   2226  CD2  PHE B  67    -1.456  25.317   7.330  1.00 20.10          C
ATOM   2227  CE1  PHE B  67    -0.955  26.707   4.995  1.00 19.07          C
ATOM   2228  CE2  PHE B  67    -0.168  25.518   6.920  1.00 20.63          C
ATOM   2229  CZ   PHE B  67     0.085  26.227   5.741  1.00 19.94          C
ATOM   2230  N    THR B  68    -6.618  24.062   6.174  1.00 21.06          N
ATOM   2231  CA   THR B  68    -7.751  23.274   6.605  1.00 20.57          C
ATOM   2232  C    THR B  68    -7.333  21.829   6.857  1.00 19.83          C
ATOM   2233  O    THR B  68    -6.880  21.111   5.959  1.00 17.54          O
ATOM   2234  CB   THR B  68    -8.925  23.337   5.567  1.00 20.96          C
ATOM   2235  OG1  THR B  68    -9.283  24.697   5.347  1.00 23.86          O
ATOM   2236  CG2  THR B  68   -10.161  22.589   6.051  1.00 22.79          C
ATOM   2237  N    ILE B  69    -7.514  21.399   8.090  1.00 18.28          N
ATOM   2238  CA   ILE B  69    -7.138  20.058   8.473  1.00 16.80          C
ATOM   2239  C    ILE B  69    -8.392  19.168   8.442  1.00 17.58          C
ATOM   2240  O    ILE B  69    -9.486  19.612   8.824  1.00 15.32          O
ATOM   2241  CB   ILE B  69    -6.394  20.056   9.850  1.00 15.91          C
ATOM   2242  CG1  ILE B  69    -5.808  18.650  10.139  1.00 14.18          C
ATOM   2243  CG2  ILE B  69    -7.300  20.461  10.987  1.00 13.14          C
ATOM   2244  CD1  ILE B  69    -4.949  18.614  11.381  1.00 14.94          C
ATOM   2245  N    SER B  70    -8.232  17.920   7.997  1.00 17.36          N
ATOM   2246  CA   SER B  70    -9.342  17.011   7.904  1.00 17.20          C
ATOM   2247  C    SER B  70    -8.861  15.566   7.926  1.00 19.29          C
ATOM   2248  O    SER B  70    -7.671  15.289   7.716  1.00 19.38          O
ATOM   2249  CB   SER B  70   -10.149  17.298   6.639  1.00 17.44          C
ATOM   2250  OG   SER B  70    -9.393  17.038   5.469  1.00 15.89          O
ATOM   2251  N    ARG B  71    -9.786  14.653   8.199  1.00 17.84          N
ATOM   2252  CA   ARG B  71    -9.458  13.242   8.378  1.00 19.49          C
ATOM   2253  C    ARG B  71   -10.559  12.344   7.824  1.00 19.86          C
ATOM   2254  O    ARG B  71   -11.754  12.695   7.859  1.00 21.39          O
ATOM   2255  CB   ARG B  71    -9.211  12.935   9.862  1.00 18.09          C
ATOM   2256  CG   ARG B  71   -10.384  13.223  10.773  1.00 18.72          C
ATOM   2257  CD   ARG B  71   -10.021  13.120  12.247  1.00 18.62          C
ATOM   2258  NE   ARG B  71    -9.443  11.833  12.590  1.00 19.12          N
ATOM   2259  CZ   ARG B  71    -8.696  11.579  13.662  1.00 20.81          C
ATOM   2260  NH1  ARG B  71    -8.398  12.525  14.546  1.00 20.83          N
ATOM   2261  NH2  ARG B  71    -8.218  10.357  13.840  1.00 20.18          N
ATOM   2262  N    ASP B  72   -10.137  11.180   7.353  1.00 21.40          N
ATOM   2263  CA   ASP B  72   -11.024  10.167   6.822  1.00 22.13          C
```

```
ATOM   2264  C   ASP B  72   -10.706   8.916   7.590  1.00 21.35           C
ATOM   2265  O   ASP B  72    -9.799   8.212   7.248  1.00 22.07           O
ATOM   2266  CB  ASP B  72   -10.774   9.974   5.329  1.00 23.13           C
ATOM   2267  CG  ASP B  72   -11.800   9.056   4.679  1.00 24.29           C
ATOM   2268  OD1 ASP B  72   -12.400   8.226   5.405  1.00 27.13           O
ATOM   2269  OD2 ASP B  72   -11.991   9.175   3.448  1.00 25.04           O
ATOM   2270  N   ASP B  73   -11.459   8.687   8.656  1.00 21.20           N
ATOM   2271  CA  ASP B  73   -11.185   7.631   9.598  1.00 23.06           C
ATOM   2272  C   ASP B  73   -11.255   6.266   8.935  1.00 24.27           C
ATOM   2273  O   ASP B  73   -10.593   5.339   9.367  1.00 25.67           O
ATOM   2274  CB  ASP B  73   -12.173   7.674  10.772  1.00 22.64           C
ATOM   2275  CG  ASP B  73   -11.885   8.799  11.766  1.00 21.48           C
ATOM   2276  OD1 ASP B  73   -10.806   9.425  11.687  1.00 20.20           O
ATOM   2277  OD2 ASP B  73   -12.750   9.046  12.640  1.00 22.02           O
ATOM   2278  N   SER B  74   -12.061   6.150   7.884  1.00 26.26           N
ATOM   2279  CA  SER B  74   -12.251   4.875   7.200  1.00 26.06           C
ATOM   2280  C   SER B  74   -11.013   4.491   6.401  1.00 26.41           C
ATOM   2281  O   SER B  74   -10.769   3.303   6.176  1.00 26.35           O
ATOM   2282  CB  SER B  74   -13.471   4.961   6.278  1.00 27.01           C
ATOM   2283  OG  SER B  74   -13.249   5.875   5.226  1.00 27.78           O
ATOM   2284  N   LYS B  75   -10.249   5.499   5.966  1.00 25.38           N
ATOM   2285  CA  LYS B  75    -9.036   5.310   5.178  1.00 25.52           C
ATOM   2286  C   LYS B  75    -7.761   5.461   6.020  1.00 24.67           C
ATOM   2287  O   LYS B  75    -6.652   5.291   5.514  1.00 25.30           O
ATOM   2288  CB  LYS B  75    -8.990   6.336   4.047  1.00 26.64           C
ATOM   2289  CG  LYS B  75   -10.101   6.234   3.035  1.00 28.15           C
ATOM   2290  CD  LYS B  75    -9.844   7.248   1.929  1.00 28.75           C
ATOM   2291  CE  LYS B  75   -10.839   7.152   0.769  1.00 29.76           C
ATOM   2292  NZ  LYS B  75   -10.184   7.550  -0.531  1.00 31.63           N
ATOM   2293  N   SER B  76    -7.922   5.764   7.302  1.00 23.94           N
ATOM   2294  CA  SER B  76    -6.792   6.013   8.201  1.00 23.65           C
ATOM   2295  C   SER B  76    -5.872   7.106   7.654  1.00 21.50           C
ATOM   2296  O   SER B  76    -4.646   6.972   7.708  1.00 20.88           O
ATOM   2297  CB  SER B  76    -6.000   4.725   8.450  1.00 22.33           C
ATOM   2298  OG  SER B  76    -6.757   3.821   9.212  1.00 25.92           O
ATOM   2299  N   ARG B  77    -6.484   8.173   7.136  1.00 21.72           N
ATOM   2300  CA  ARG B  77    -5.776   9.238   6.420  1.00 21.27           C
ATOM   2301  C   ARG B  77    -6.139  10.619   6.991  1.00 19.07           C
ATOM   2302  O   ARG B  77    -7.309  10.901   7.235  1.00 19.03           O
ATOM   2303  CB  ARG B  77    -6.113   9.208   4.912  1.00 21.98           C
ATOM   2304  CG  ARG B  77    -5.251  10.141   4.069  1.00 24.14           C
ATOM   2305  CD  ARG B  77    -5.274   9.798   2.590  1.00 25.23           C
ATOM   2306  NE  ARG B  77    -4.694   8.482   2.296  1.00 26.53           N
ATOM   2307  CZ  ARG B  77    -3.390   8.229   2.120  1.00 29.54           C
ATOM   2308  NH1 ARG B  77    -2.465   9.196   2.230  1.00 30.00           N
ATOM   2309  NH2 ARG B  77    -2.996   6.978   1.827  1.00 30.68           N
ATOM   2310  N   LEU B  78    -5.110  11.445   7.159  1.00 17.22           N
ATOM   2311  CA  LEU B  78    -5.195  12.854   7.579  1.00 16.37           C
ATOM   2312  C   LEU B  78    -4.817  13.747   6.410  1.00 15.36           C
ATOM   2313  O   LEU B  78    -3.916  13.430   5.645  1.00 15.74           O
ATOM   2314  CB  LEU B  78    -4.190  13.080   8.720  1.00 15.29           C
ATOM   2315  CG  LEU B  78    -4.155  14.455   9.424  1.00 13.94           C
ATOM   2316  CD1 LEU B  78    -5.394  14.678  10.272  1.00 14.08           C
ATOM   2317  CD2 LEU B  78    -2.887  14.580  10.264  1.00 15.65           C
ATOM   2318  N   TYR B  79    -5.460  14.905   6.282  1.00 16.64           N
ATOM   2319  CA  TYR B  79    -5.146  15.808   5.192  1.00 16.37           C
ATOM   2320  C   TYR B  79    -4.889  17.185   5.730  1.00 16.48           C
ATOM   2321  O   TYR B  79    -5.428  17.557   6.770  1.00 15.20           O
ATOM   2322  CB  TYR B  79    -6.320  15.951   4.244  1.00 19.81           C
ATOM   2323  CG  TYR B  79    -6.811  14.656   3.673  1.00 21.59           C
ATOM   2324  CD1 TYR B  79    -6.232  14.122   2.527  1.00 22.77           C
ATOM   2325  CD2 TYR B  79    -7.849  13.971   4.282  1.00 22.04           C
ATOM   2326  CE1 TYR B  79    -6.672  12.908   2.008  1.00 23.56           C
ATOM   2327  CE2 TYR B  79    -8.303  12.781   3.789  1.00 22.21           C
ATOM   2328  CZ  TYR B  79    -7.712  12.249   2.643  1.00 23.93           C
```

```
ATOM   2329  OH   TYR B  79      -8.173  11.056   2.130  1.00 25.59           O
ATOM   2330  N    LEU B  80      -4.085  17.916   4.980  1.00 15.98           N
ATOM   2331  CA   LEU B  80      -3.938  19.354   5.151  1.00 16.82           C
ATOM   2332  C    LEU B  80      -4.080  20.040   3.806  1.00 19.51           C
ATOM   2333  O    LEU B  80      -3.274  19.808   2.877  1.00 18.98           O
ATOM   2334  CB   LEU B  80      -2.589  19.685   5.772  1.00 15.67           C
ATOM   2335  CG   LEU B  80      -2.380  21.182   6.189  1.00 13.83           C
ATOM   2336  CD1  LEU B  80      -3.295  21.547   7.284  1.00 14.79           C
ATOM   2337  CD2  LEU B  80      -0.983  21.390   6.622  1.00 16.41           C
ATOM   2338  N    GLN B  81      -5.090  20.913   3.713  1.00 19.62           N
ATOM   2339  CA   GLN B  81      -5.318  21.719   2.533  1.00 19.06           C
ATOM   2340  C    GLN B  81      -4.737  23.092   2.833  1.00 18.75           C
ATOM   2341  O    GLN B  81      -5.121  23.717   3.802  1.00 18.28           O
ATOM   2342  CB   GLN B  81      -6.813  21.798   2.208  1.00 18.37           C
ATOM   2343  CG   GLN B  81      -7.176  22.747   1.038  1.00 20.43           C
ATOM   2344  CD   GLN B  81      -6.688  22.236  -0.300  1.00 21.41           C
ATOM   2345  OE1  GLN B  81      -7.012  21.117  -0.696  1.00 24.07           O
ATOM   2346  NE2  GLN B  81      -5.896  23.031  -0.994  1.00 23.80           N
ATOM   2347  N    MET B  82      -3.779  23.516   2.015  1.00 19.49           N
ATOM   2348  CA   MET B  82      -3.017  24.732   2.254  1.00 21.78           C
ATOM   2349  C    MET B  82      -3.254  25.656   1.081  1.00 21.40           C
ATOM   2350  O    MET B  82      -2.851  25.354  -0.057  1.00 21.17           O
ATOM   2351  CB   MET B  82      -1.524  24.398   2.387  1.00 23.35           C
ATOM   2352  CG   MET B  82      -1.176  23.275   3.384  1.00 25.15           C
ATOM   2353  SD   MET B  82       0.444  22.481   3.111  1.00 27.69           S
ATOM   2354  CE   MET B  82       1.502  23.867   3.293  1.00 26.10           C
ATOM   2355  N    ASN B  82A     -3.895  26.788   1.339  1.00 21.28           N
ATOM   2356  CA   ASN B  82A     -4.184  27.773   0.310  1.00 22.39           C
ATOM   2357  C    ASN B  82A     -3.421  29.078   0.472  1.00 21.96           C
ATOM   2358  O    ASN B  82A     -2.934  29.374   1.551  1.00 21.39           O
ATOM   2359  CB   ASN B  82A     -5.674  28.102   0.314  1.00 22.74           C
ATOM   2360  CG   ASN B  82A     -6.561  26.881   0.035  1.00 23.61           C
ATOM   2361  OD1  ASN B  82A     -6.189  25.964  -0.700  1.00 25.58           O
ATOM   2362  ND2  ASN B  82A     -7.770  26.892   0.615  1.00 25.40           N
ATOM   2363  N    ASN B  82B     -3.319  29.844  -0.613  1.00 22.88           N
ATOM   2364  CA   ASN B  82B     -2.620  31.150  -0.614  1.00 22.98           C
ATOM   2365  C    ASN B  82B     -1.226  31.098   0.017  1.00 22.51           C
ATOM   2366  O    ASN B  82B     -0.867  31.910   0.886  1.00 21.39           O
ATOM   2367  CB   ASN B  82B     -3.497  32.177   0.094  1.00 24.41           C
ATOM   2368  CG   ASN B  82B     -3.157  33.612  -0.277  1.00 26.85           C
ATOM   2369  OD1  ASN B  82B     -3.745  34.541   0.279  1.00 33.04           O
ATOM   2370  ND2  ASN B  82B     -2.221  33.807  -1.204  1.00 31.70           N
ATOM   2371  N    LEU B  82C     -0.434  30.117  -0.426  1.00 22.27           N
ATOM   2372  CA   LEU B  82C      0.852  29.850   0.162  1.00 20.67           C
ATOM   2373  C    LEU B  82C      1.838  30.966  -0.120  1.00 21.04           C
ATOM   2374  O    LEU B  82C      1.805  31.617  -1.171  1.00 18.54           O
ATOM   2375  CB   LEU B  82C      1.400  28.499  -0.327  1.00 20.77           C
ATOM   2376  CG   LEU B  82C      0.708  27.267   0.253  1.00 20.01           C
ATOM   2377  CD1  LEU B  82C      0.959  25.978  -0.585  1.00 20.74           C
ATOM   2378  CD2  LEU B  82C      1.190  27.014   1.671  1.00 20.50           C
ATOM   2379  N    ARG B  83       2.677  31.212   0.867  1.00 21.52           N
ATOM   2380  CA   ARG B  83       3.766  32.148   0.763  1.00 24.82           C
ATOM   2381  C    ARG B  83       5.091  31.428   0.901  1.00 24.02           C
ATOM   2382  O    ARG B  83       5.142  30.326   1.409  1.00 22.19           O
ATOM   2383  CB   ARG B  83       3.658  33.172   1.877  1.00 27.32           C
ATOM   2384  CG   ARG B  83       2.331  33.838   1.960  1.00 32.61           C
ATOM   2385  CD   ARG B  83       2.479  35.342   1.915  1.00 36.84           C
ATOM   2386  NE   ARG B  83       3.335  35.889   2.978  1.00 38.80           N
ATOM   2387  CZ   ARG B  83       3.794  37.146   2.995  1.00 38.81           C
ATOM   2388  NH1  ARG B  83       3.491  37.982   2.007  1.00 39.17           N
ATOM   2389  NH2  ARG B  83       4.567  37.571   3.996  1.00 39.86           N
ATOM   2390  N    THR B  84       6.165  32.072   0.459  1.00 24.58           N
ATOM   2391  CA   THR B  84       7.509  31.503   0.548  1.00 25.19           C
ATOM   2392  C    THR B  84       7.844  30.994   1.949  1.00 24.56           C
ATOM   2393  O    THR B  84       8.519  29.982   2.078  1.00 25.38           O
```

| ATOM | 2394 | CB | THR | B | 84 | 8.579 | 32.512 | 0.121 | 1.00 | 26.60 | C |
| ATOM | 2395 | OG1 | THR | B | 84 | 8.428 | 33.706 | 0.897 | 1.00 | 29.56 | O |
| ATOM | 2396 | CG2 | THR | B | 84 | 8.414 | 32.866 | -1.335 | 1.00 | 28.35 | C |
| ATOM | 2397 | N | GLU | B | 85 | 7.327 | 31.658 | 2.986 | 1.00 | 24.20 | N |
| ATOM | 2398 | CA | GLU | B | 85 | 7.603 | 31.256 | 4.377 | 1.00 | 24.29 | C |
| ATOM | 2399 | C | GLU | B | 85 | 6.855 | 29.999 | 4.821 | 1.00 | 22.21 | C |
| ATOM | 2400 | O | GLU | B | 85 | 7.117 | 29.489 | 5.894 | 1.00 | 21.84 | O |
| ATOM | 2401 | CB | GLU | B | 85 | 7.353 | 32.392 | 5.393 | 1.00 | 26.74 | C |
| ATOM | 2402 | CG | GLU | B | 85 | 6.301 | 33.419 | 5.010 | 1.00 | 30.71 | C |
| ATOM | 2403 | CD | GLU | B | 85 | 6.854 | 34.454 | 4.019 | 1.00 | 33.48 | C |
| ATOM | 2404 | OE1 | GLU | B | 85 | 7.871 | 35.134 | 4.326 | 1.00 | 38.26 | O |
| ATOM | 2405 | OE2 | GLU | B | 85 | 6.297 | 34.563 | 2.920 | 1.00 | 34.50 | O |
| ATOM | 2406 | N | ASP | B | 86 | 5.930 | 29.514 | 3.998 | 1.00 | 21.58 | N |
| ATOM | 2407 | CA | ASP | B | 86 | 5.238 | 28.246 | 4.254 | 1.00 | 20.24 | C |
| ATOM | 2408 | C | ASP | B | 86 | 6.048 | 27.055 | 3.764 | 1.00 | 18.96 | C |
| ATOM | 2409 | O | ASP | B | 86 | 5.609 | 25.903 | 3.926 | 1.00 | 18.74 | O |
| ATOM | 2410 | CB | ASP | B | 86 | 3.846 | 28.248 | 3.623 | 1.00 | 19.96 | C |
| ATOM | 2411 | CG | ASP | B | 86 | 2.933 | 29.319 | 4.242 | 1.00 | 21.41 | C |
| ATOM | 2412 | OD1 | ASP | B | 86 | 2.827 | 29.385 | 5.476 | 1.00 | 18.99 | O |
| ATOM | 2413 | OD2 | ASP | B | 86 | 2.350 | 30.114 | 3.481 | 1.00 | 19.02 | O |
| ATOM | 2414 | N | THR | B | 87 | 7.194 | 27.336 | 3.151 | 1.00 | 17.90 | N |
| ATOM | 2415 | CA | THR | B | 87 | 8.177 | 26.332 | 2.730 | 1.00 | 18.58 | C |
| ATOM | 2416 | C | THR | B | 87 | 8.690 | 25.602 | 3.971 | 1.00 | 18.73 | C |
| ATOM | 2417 | O | THR | B | 87 | 9.091 | 26.231 | 4.935 | 1.00 | 19.57 | O |
| ATOM | 2418 | CB | THR | B | 87 | 9.347 | 26.999 | 1.994 | 1.00 | 17.90 | C |
| ATOM | 2419 | OG1 | THR | B | 87 | 8.876 | 27.596 | 0.765 | 1.00 | 16.42 | O |
| ATOM | 2420 | CG2 | THR | B | 87 | 10.476 | 26.039 | 1.706 | 1.00 | 17.93 | C |
| ATOM | 2421 | N | GLY | B | 88 | 8.659 | 24.273 | 3.946 | 1.00 | 17.27 | N |
| ATOM | 2422 | CA | GLY | B | 88 | 9.138 | 23.500 | 5.050 | 1.00 | 14.65 | C |
| ATOM | 2423 | C | GLY | B | 88 | 8.897 | 22.017 | 4.843 | 1.00 | 14.22 | C |
| ATOM | 2424 | O | GLY | B | 88 | 8.301 | 21.593 | 3.847 | 1.00 | 12.43 | O |
| ATOM | 2425 | N | ILE | B | 89 | 9.366 | 21.265 | 5.815 | 1.00 | 13.86 | N |
| ATOM | 2426 | CA | ILE | B | 89 | 9.027 | 19.844 | 5.950 | 1.00 | 14.45 | C |
| ATOM | 2427 | C | ILE | B | 89 | 7.790 | 19.790 | 6.827 | 1.00 | 15.43 | C |
| ATOM | 2428 | O | ILE | B | 89 | 7.775 | 20.345 | 7.933 | 1.00 | 16.84 | O |
| ATOM | 2429 | CB | ILE | B | 89 | 10.161 | 19.037 | 6.592 | 1.00 | 13.48 | C |
| ATOM | 2430 | CG1 | ILE | B | 89 | 11.495 | 19.317 | 5.909 | 1.00 | 15.54 | C |
| ATOM | 2431 | CG2 | ILE | B | 89 | 9.815 | 17.551 | 6.590 | 1.00 | 14.92 | C |
| ATOM | 2432 | CD1 | ILE | B | 89 | 11.474 | 19.209 | 4.494 | 1.00 | 16.84 | C |
| ATOM | 2433 | N | TYR | B | 90 | 6.736 | 19.160 | 6.317 | 1.00 | 14.35 | N |
| ATOM | 2434 | CA | TYR | B | 90 | 5.503 | 18.996 | 7.063 | 1.00 | 13.20 | C |
| ATOM | 2435 | C | TYR | B | 90 | 5.414 | 17.553 | 7.581 | 1.00 | 13.85 | C |
| ATOM | 2436 | O | TYR | B | 90 | 5.428 | 16.633 | 6.781 | 1.00 | 14.50 | O |
| ATOM | 2437 | CB | TYR | B | 90 | 4.316 | 19.312 | 6.160 | 1.00 | 12.99 | C |
| ATOM | 2438 | CG | TYR | B | 90 | 4.157 | 20.806 | 5.953 | 1.00 | 12.81 | C |
| ATOM | 2439 | CD1 | TYR | B | 90 | 5.088 | 21.515 | 5.211 | 1.00 | 14.77 | C |
| ATOM | 2440 | CD2 | TYR | B | 90 | 3.101 | 21.489 | 6.519 | 1.00 | 13.80 | C |
| ATOM | 2441 | CE1 | TYR | B | 90 | 4.971 | 22.904 | 5.010 | 1.00 | 14.01 | C |
| ATOM | 2442 | CE2 | TYR | B | 90 | 2.981 | 22.914 | 6.353 | 1.00 | 14.02 | C |
| ATOM | 2443 | CZ | TYR | B | 90 | 3.909 | 23.578 | 5.592 | 1.00 | 13.59 | C |
| ATOM | 2444 | OH | TYR | B | 90 | 3.809 | 24.952 | 5.414 | 1.00 | 15.39 | O |
| ATOM | 2445 | N | TYR | B | 91 | 5.380 | 17.399 | 8.906 | 1.00 | 13.29 | N |
| ATOM | 2446 | CA | TYR | B | 91 | 5.129 | 16.116 | 9.573 | 1.00 | 15.54 | C |
| ATOM | 2447 | C | TYR | B | 91 | 3.693 | 15.964 | 10.065 | 1.00 | 16.53 | C |
| ATOM | 2448 | O | TYR | B | 91 | 3.066 | 16.930 | 10.553 | 1.00 | 13.88 | O |
| ATOM | 2449 | CB | TYR | B | 91 | 6.012 | 15.987 | 10.796 | 1.00 | 16.49 | C |
| ATOM | 2450 | CG | TYR | B | 91 | 7.490 | 16.057 | 10.541 | 1.00 | 17.38 | C |
| ATOM | 2451 | CD1 | TYR | B | 91 | 8.236 | 14.900 | 10.293 | 1.00 | 17.60 | C |
| ATOM | 2452 | CD2 | TYR | B | 91 | 8.172 | 17.282 | 10.605 | 1.00 | 16.63 | C |
| ATOM | 2453 | CE1 | TYR | B | 91 | 9.592 | 14.966 | 10.064 | 1.00 | 16.88 | C |
| ATOM | 2454 | CE2 | TYR | B | 91 | 9.540 | 17.342 | 10.384 | 1.00 | 16.98 | C |
| ATOM | 2455 | CZ | TYR | B | 91 | 10.236 | 16.171 | 10.119 | 1.00 | 17.59 | C |
| ATOM | 2456 | OH | TYR | B | 91 | 11.580 | 16.216 | 9.913 | 1.00 | 19.21 | O |
| ATOM | 2457 | N | CYS | B | 92 | 3.184 | 14.730 | 9.958 | 1.00 | 16.85 | N |
| ATOM | 2458 | CA | CYS | B | 92 | 2.092 | 14.286 | 10.798 | 1.00 | 17.68 | C |

```
ATOM   2459  C    CYS B  92      2.658  13.918  12.162  1.00 17.19           C
ATOM   2460  O    CYS B  92      3.603  13.104  12.289  1.00 14.47           O
ATOM   2461  CB   CYS B  92      1.359  13.083  10.212  1.00 20.01           C
ATOM   2462  SG   CYS B  92      0.363  13.548   8.794  1.00 26.07           S
ATOM   2463  N    PHE B  93      2.076  14.559  13.168  1.00 15.57           N
ATOM   2464  CA   PHE B  93      2.424  14.368  14.563  1.00 16.04           C
ATOM   2465  C    PHE B  93      1.169  13.989  15.291  1.00 15.44           C
ATOM   2466  O    PHE B  93      0.175  14.723  15.289  1.00 13.23           O
ATOM   2467  CB   PHE B  93      3.022  15.663  15.133  1.00 15.77           C
ATOM   2468  CG   PHE B  93      3.268  15.639  16.609  1.00 15.98           C
ATOM   2469  CD1  PHE B  93      4.009  14.618  17.190  1.00 15.68           C
ATOM   2470  CD2  PHE B  93      2.799  16.663  17.418  1.00 17.08           C
ATOM   2471  CE1  PHE B  93      4.263  14.613  18.534  1.00 16.87           C
ATOM   2472  CE2  PHE B  93      3.067  16.659  18.757  1.00 17.27           C
ATOM   2473  CZ   PHE B  93      3.813  15.625  19.311  1.00 17.35           C
ATOM   2474  N    LEU B  94      1.170  12.778  15.836  1.00 14.69           N
ATOM   2475  CA   LEU B  94      0.086  12.325  16.673  1.00 15.01           C
ATOM   2476  C    LEU B  94      0.457  12.654  18.104  1.00 15.40           C
ATOM   2477  O    LEU B  94      1.214  11.919  18.736  1.00 13.42           O
ATOM   2478  CB   LEU B  94     -0.126  10.807  16.542  1.00 14.50           C
ATOM   2479  CG   LEU B  94     -0.891  10.330  15.316  1.00 14.77           C
ATOM   2480  CD1  LEU B  94     -0.189  10.733  14.037  1.00 14.53           C
ATOM   2481  CD2  LEU B  94     -0.958   8.809  15.419  1.00 15.39           C
ATOM   2482  N    PRO B  95     -0.105  13.751  18.649  1.00 14.79           N
ATOM   2483  CA   PRO B  95      0.328  14.167  19.972  1.00 15.92           C
ATOM   2484  C    PRO B  95      0.093  13.058  20.987  1.00 17.10           C
ATOM   2485  O    PRO B  95     -0.989  12.486  20.981  1.00 18.87           O
ATOM   2486  CB   PRO B  95     -0.568  15.381  20.280  1.00 14.97           C
ATOM   2487  CG   PRO B  95     -1.126  15.803  18.973  1.00 16.15           C
ATOM   2488  CD   PRO B  95     -1.185  14.589  18.111  1.00 15.96           C
ATOM   2489  N    MET B  96      1.075  12.732  21.824  1.00 17.83           N
ATOM   2490  CA   MET B  96      2.420  13.320  21.807  1.00 17.05           C
ATOM   2491  C    MET B  96      3.460  12.331  21.326  1.00 15.37           C
ATOM   2492  O    MET B  96      4.661  12.578  21.380  1.00 12.70           O
ATOM   2493  CB   MET B  96      2.787  13.790  23.223  1.00 18.75           C
ATOM   2494  CG   MET B  96      1.914  14.897  23.756  1.00 15.56           C
ATOM   2495  SD   MET B  96      2.064  16.515  22.990  1.00 16.63           S
ATOM   2496  CE   MET B  96      3.739  16.966  23.479  1.00 16.73           C
ATOM   2497  N    ASP B 101      3.012  11.152  20.887  1.00 16.88           N
ATOM   2498  CA   ASP B 101      3.915  10.037  20.804  1.00 16.71           C
ATOM   2499  C    ASP B 101      4.499   9.689  19.463  1.00 16.88           C
ATOM   2500  O    ASP B 101      5.530   9.024  19.454  1.00 18.31           O
ATOM   2501  CB   ASP B 101      3.246   8.759  21.308  1.00 18.55           C
ATOM   2502  CG   ASP B 101      2.762   8.880  22.719  1.00 22.30           C
ATOM   2503  OD1  ASP B 101      3.400   9.600  23.506  1.00 23.95           O
ATOM   2504  OD2  ASP B 101      1.716   8.300  23.019  1.00 27.94           O
ATOM   2505  N    TYR B 102      3.832  10.023  18.355  1.00 15.54           N
ATOM   2506  CA   TYR B 102      4.303   9.585  17.032  1.00 16.87           C
ATOM   2507  C    TYR B 102      4.523  10.699  16.031  1.00 18.01           C
ATOM   2508  O    TYR B 102      3.738  11.627  15.953  1.00 17.83           O
ATOM   2509  CB   TYR B 102      3.337   8.566  16.435  1.00 19.56           C
ATOM   2510  CG   TYR B 102      3.028   7.462  17.396  1.00 20.30           C
ATOM   2511  CD1  TYR B 102      3.962   6.459  17.671  1.00 20.76           C
ATOM   2512  CD2  TYR B 102      1.808   7.434  18.062  1.00 20.54           C
ATOM   2513  CE1  TYR B 102      3.687   5.458  18.595  1.00 21.43           C
ATOM   2514  CE2  TYR B 102      1.508   6.433  18.950  1.00 21.21           C
ATOM   2515  CZ   TYR B 102      2.439   5.448  19.218  1.00 22.20           C
ATOM   2516  OH   TYR B 102      2.088   4.474  20.132  1.00 23.43           O
ATOM   2517  N    TRP B 103      5.574  10.537  15.236  1.00 19.05           N
ATOM   2518  CA   TRP B 103      5.932  11.447  14.154  1.00 17.74           C
ATOM   2519  C    TRP B 103      6.011  10.662  12.856  1.00 18.93           C
ATOM   2520  O    TRP B 103      6.410   9.502  12.877  1.00 17.13           O
ATOM   2521  CB   TRP B 103      7.323  11.991  14.418  1.00 16.36           C
ATOM   2522  CG   TRP B 103      7.366  12.939  15.509  1.00 15.78           C
ATOM   2523  CD1  TRP B 103      7.431  12.671  16.835  1.00 14.39           C
```

| | | | | | | | | | | |
|---|---|---|---|---|---|---|---|---|---|---|
| ATOM | 2524 | CD2 | TRP | B | 103 | 7.332 | 14.369 | 15.381 | 1.00 12.82 | C |
| ATOM | 2525 | NE1 | TRP | B | 103 | 7.458 | 13.846 | 17.546 | 1.00 15.34 | N |
| ATOM | 2526 | CE2 | TRP | B | 103 | 7.378 | 14.897 | 16.673 | 1.00 14.49 | C |
| ATOM | 2527 | CE3 | TRP | B | 103 | 7.246 | 15.238 | 14.293 | 1.00 14.90 | C |
| ATOM | 2528 | CZ2 | TRP | B | 103 | 7.375 | 16.273 | 16.919 | 1.00 13.92 | C |
| ATOM | 2529 | CZ3 | TRP | B | 103 | 7.221 | 16.602 | 14.530 | 1.00 15.04 | C |
| ATOM | 2530 | CH2 | TRP | B | 103 | 7.302 | 17.105 | 15.833 | 1.00 16.16 | C |
| ATOM | 2531 | N | GLY | B | 104 | 5.664 | 11.290 | 11.745 | 1.00 17.64 | N |
| ATOM | 2532 | CA | GLY | B | 104 | 5.832 | 10.675 | 10.431 | 1.00 17.69 | C |
| ATOM | 2533 | C | GLY | B | 104 | 7.217 | 10.997 | 9.936 | 1.00 17.49 | C |
| ATOM | 2534 | O | GLY | B | 104 | 8.078 | 11.372 | 10.728 | 1.00 16.72 | O |
| ATOM | 2535 | N | GLN | B | 105 | 7.478 | 10.824 | 8.642 | 1.00 18.07 | N |
| ATOM | 2536 | CA | GLN | B | 105 | 8.841 | 11.056 | 8.161 | 1.00 18.95 | C |
| ATOM | 2537 | C | GLN | B | 105 | 8.972 | 12.365 | 7.420 | 1.00 17.51 | C |
| ATOM | 2538 | O | GLN | B | 105 | 10.061 | 12.707 | 6.969 | 1.00 17.92 | O |
| ATOM | 2539 | CB | GLN | B | 105 | 9.371 | 9.929 | 7.298 | 1.00 22.69 | C |
| ATOM | 2540 | CG | GLN | B | 105 | 8.939 | 9.976 | 5.879 | 1.00 28.21 | C |
| ATOM | 2541 | CD | GLN | B | 105 | 7.515 | 9.565 | 5.727 | 1.00 32.18 | C |
| ATOM | 2542 | OE1 | GLN | B | 105 | 6.781 | 10.078 | 4.866 | 1.00 35.16 | O |
| ATOM | 2543 | NE2 | GLN | B | 105 | 7.107 | 8.595 | 6.548 | 1.00 35.66 | N |
| ATOM | 2544 | N | GLY | B | 106 | 7.867 | 13.082 | 7.318 | 1.00 16.97 | N |
| ATOM | 2545 | CA | GLY | B | 106 | 7.852 | 14.403 | 6.712 | 1.00 17.25 | C |
| ATOM | 2546 | C | GLY | B | 106 | 7.612 | 14.371 | 5.207 | 1.00 18.48 | C |
| ATOM | 2547 | O | GLY | B | 106 | 8.020 | 13.431 | 4.491 | 1.00 18.88 | O |
| ATOM | 2548 | N | THR | B | 107 | 6.961 | 15.420 | 4.721 | 1.00 18.78 | N |
| ATOM | 2549 | CA | THR | B | 107 | 6.887 | 15.683 | 3.283 | 1.00 17.22 | C |
| ATOM | 2550 | C | THR | B | 107 | 7.236 | 17.149 | 3.013 | 1.00 17.77 | C |
| ATOM | 2551 | O | THR | B | 107 | 6.861 | 18.051 | 3.763 | 1.00 15.62 | O |
| ATOM | 2552 | CB | THR | B | 107 | 5.528 | 15.237 | 2.657 | 1.00 17.79 | C |
| ATOM | 2553 | OG1 | THR | B | 107 | 5.613 | 15.354 | 1.235 | 1.00 17.80 | O |
| ATOM | 2554 | CG2 | THR | B | 107 | 4.340 | 16.037 | 3.197 | 1.00 17.26 | C |
| ATOM | 2555 | N | SER | B | 108 | 8.017 | 17.361 | 1.964 | 1.00 17.17 | N |
| ATOM | 2556 | CA | SER | B | 108 | 8.615 | 18.652 | 1.707 | 1.00 16.59 | C |
| ATOM | 2557 | C | SER | B | 108 | 7.707 | 19.466 | 0.799 | 1.00 16.31 | C |
| ATOM | 2558 | O | SER | B | 108 | 7.236 | 18.999 | -0.200 | 1.00 15.64 | O |
| ATOM | 2559 | CB | SER | B | 108 | 9.998 | 18.509 | 1.069 | 1.00 17.51 | C |
| ATOM | 2560 | OG | SER | B | 108 | 10.617 | 19.800 | 0.958 | 1.00 21.55 | O |
| ATOM | 2561 | N | VAL | B | 109 | 7.503 | 20.718 | 1.173 | 1.00 16.95 | N |
| ATOM | 2562 | CA | VAL | B | 109 | 6.665 | 21.633 | 0.442 | 1.00 15.44 | C |
| ATOM | 2563 | C | VAL | B | 109 | 7.518 | 22.850 | 0.214 | 1.00 15.39 | C |
| ATOM | 2564 | O | VAL | B | 109 | 8.049 | 23.380 | 1.174 | 1.00 15.17 | O |
| ATOM | 2565 | CB | VAL | B | 109 | 5.452 | 22.002 | 1.289 | 1.00 15.69 | C |
| ATOM | 2566 | CG1 | VAL | B | 109 | 4.697 | 23.158 | 0.668 | 1.00 17.73 | C |
| ATOM | 2567 | CG2 | VAL | B | 109 | 4.547 | 20.751 | 1.478 | 1.00 15.95 | C |
| ATOM | 2568 | N | THR | B | 110 | 7.685 | 23.231 | -1.052 | 1.00 14.72 | N |
| ATOM | 2569 | CA | THR | B | 110 | 8.477 | 24.393 | -1.443 | 1.00 16.09 | C |
| ATOM | 2570 | C | THR | B | 110 | 7.537 | 25.359 | -2.116 | 1.00 16.32 | C |
| ATOM | 2571 | O | THR | B | 110 | 6.772 | 24.974 | -2.992 | 1.00 16.55 | O |
| ATOM | 2572 | CB | THR | B | 110 | 9.635 | 24.010 | -2.405 | 1.00 17.37 | C |
| ATOM | 2573 | OG1 | THR | B | 110 | 10.545 | 23.114 | -1.741 | 1.00 18.84 | O |
| ATOM | 2574 | CG2 | THR | B | 110 | 10.409 | 25.239 | -2.908 | 1.00 17.25 | C |
| ATOM | 2575 | N | VAL | B | 111 | 7.609 | 26.633 | -1.721 | 1.00 16.84 | N |
| ATOM | 2576 | CA | VAL | B | 111 | 6.764 | 27.670 | -2.308 | 1.00 17.43 | C |
| ATOM | 2577 | C | VAL | B | 111 | 7.758 | 28.642 | -2.887 | 1.00 17.05 | C |
| ATOM | 2578 | O | VAL | B | 111 | 8.544 | 29.226 | -2.162 | 1.00 16.51 | O |
| ATOM | 2579 | CB | VAL | B | 111 | 5.808 | 28.354 | -1.268 | 1.00 17.17 | C |
| ATOM | 2580 | CG1 | VAL | B | 111 | 4.883 | 29.360 | -1.957 | 1.00 17.31 | C |
| ATOM | 2581 | CG2 | VAL | B | 111 | 4.991 | 27.314 | -0.546 | 1.00 19.42 | C |
| ATOM | 2582 | N | SER | B | 112 | 7.777 | 28.728 | -4.210 | 1.00 17.92 | N |
| ATOM | 2583 | CA | SER | B | 112 | 8.826 | 29.435 | -4.935 | 1.00 18.86 | C |
| ATOM | 2584 | C | SER | B | 112 | 8.320 | 29.727 | -6.310 | 1.00 20.95 | C |
| ATOM | 2585 | O | SER | B | 112 | 7.485 | 28.999 | -6.835 | 1.00 21.63 | O |
| ATOM | 2586 | CB | SER | B | 112 | 10.085 | 28.548 | -5.047 | 1.00 18.79 | C |
| ATOM | 2587 | OG | SER | B | 112 | 11.069 | 29.149 | -5.873 | 1.00 20.29 | O |
| ATOM | 2588 | N | SER | B | 113 | 8.831 | 30.786 | -6.923 | 1.00 22.06 | N |

```
ATOM   2589  CA  SER B 113       8.490  31.049   -8.303  1.00 24.21          C
ATOM   2590  C   SER B 113       9.501  30.444   -9.293  1.00 23.92          C
ATOM   2591  O   SER B 113       9.312  30.542  -10.499  1.00 26.16          O
ATOM   2592  CB  SER B 113       8.353  32.551   -8.502  1.00 25.16          C
ATOM   2593  OG  SER B 113       9.647  33.112   -8.572  1.00 31.09          O
ATOM   2594  N   ALA B 114      10.585  29.859   -8.795  1.00 23.84          N
ATOM   2595  CA  ALA B 114      11.546  29.170   -9.646  1.00 22.64          C
ATOM   2596  C   ALA B 114      10.907  27.860  -10.101  1.00 23.34          C
ATOM   2597  O   ALA B 114       9.978  27.386   -9.461  1.00 23.80          O
ATOM   2598  CB  ALA B 114      12.791  28.912   -8.903  1.00 23.17          C
ATOM   2599  N   LYS B 115      11.391  27.307  -11.213  1.00 23.01          N
ATOM   2600  CA  LYS B 115      10.788  26.110  -11.802  1.00 22.99          C
ATOM   2601  C   LYS B 115      11.474  24.867  -11.329  1.00 19.23          C
ATOM   2602  O   LYS B 115      12.623  24.899  -10.978  1.00 18.91          O
ATOM   2603  CB  LYS B 115      10.848  26.139  -13.337  1.00 25.77          C
ATOM   2604  CG  LYS B 115       9.663  26.843  -13.993  1.00 29.56          C
ATOM   2605  CD  LYS B 115       9.857  28.337  -13.953  1.00 31.28          C
ATOM   2606  CE  LYS B 115       8.531  29.117  -14.099  1.00 31.58          C
ATOM   2607  NZ  LYS B 115       8.649  30.412  -13.330  1.00 33.64          N
ATOM   2608  N   THR B 116      10.757  23.757  -11.367  1.00 20.01          N
ATOM   2609  CA  THR B 116      11.362  22.469  -11.088  1.00 18.12          C
ATOM   2610  C   THR B 116      12.360  22.123  -12.209  1.00 18.56          C
ATOM   2611  O   THR B 116      12.056  22.274  -13.391  1.00 18.57          O
ATOM   2612  CB  THR B 116      10.296  21.386  -10.970  1.00 18.83          C
ATOM   2613  OG1 THR B 116       9.447  21.640   -9.824  1.00 18.13          O
ATOM   2614  CG2 THR B 116      10.949  20.054  -10.798  1.00 18.70          C
ATOM   2615  N   THR B 117      13.548  21.675  -11.822  1.00 18.16          N
ATOM   2616  CA  THR B 117      14.602  21.277  -12.730  1.00 18.39          C
ATOM   2617  C   THR B 117      15.155  19.927  -12.245  1.00 17.08          C
ATOM   2618  O   THR B 117      15.499  19.806  -11.094  1.00 17.04          O
ATOM   2619  CB  THR B 117      15.735  22.272  -12.707  1.00 19.15          C
ATOM   2620  OG1 THR B 117      15.228  23.586  -13.016  1.00 20.94          O
ATOM   2621  CG2 THR B 117      16.792  21.916  -13.731  1.00 19.80          C
ATOM   2622  N   PRO B 118      15.238  18.929  -13.121  1.00 18.05          N
ATOM   2623  CA  PRO B 118      15.841  17.658  -12.649  1.00 17.91          C
ATOM   2624  C   PRO B 118      17.354  17.743  -12.528  1.00 18.53          C
ATOM   2625  O   PRO B 118      17.996  18.498  -13.263  1.00 18.49          O
ATOM   2626  CB  PRO B 118      15.491  16.658  -13.757  1.00 17.90          C
ATOM   2627  CG  PRO B 118      15.055  17.434  -14.910  1.00 17.58          C
ATOM   2628  CD  PRO B 118      14.773  18.846  -14.517  1.00 18.35          C
ATOM   2629  N   PRO B 119      17.941  16.922  -11.653  1.00 18.68          N
ATOM   2630  CA  PRO B 119      19.399  16.910  -11.493  1.00 18.59          C
ATOM   2631  C   PRO B 119      20.073  16.205  -12.657  1.00 18.87          C
ATOM   2632  O   PRO B 119      19.455  15.344  -13.293  1.00 18.78          O
ATOM   2633  CB  PRO B 119      19.593  16.076  -10.227  1.00 17.65          C
ATOM   2634  CG  PRO B 119      18.483  15.169  -10.229  1.00 19.06          C
ATOM   2635  CD  PRO B 119      17.299  15.927  -10.791  1.00 18.62          C
ATOM   2636  N   SER B 120      21.307  16.602  -12.947  1.00 20.63          N
ATOM   2637  CA  SER B 120      22.200  15.806  -13.759  1.00 19.82          C
ATOM   2638  C   SER B 120      23.065  15.029  -12.786  1.00 19.47          C
ATOM   2639  O   SER B 120      23.513  15.561  -11.779  1.00 18.06          O
ATOM   2640  CB  SER B 120      23.058  16.707  -14.638  1.00 21.38          C
ATOM   2641  OG  SER B 120      22.275  17.535  -15.476  1.00 24.14          O
ATOM   2642  N   VAL B 121      23.301  13.746  -13.060  1.00 18.87          N
ATOM   2643  CA  VAL B 121      24.051  12.939  -12.119  1.00 19.47          C
ATOM   2644  C   VAL B 121      25.301  12.438  -12.781  1.00 18.80          C
ATOM   2645  O   VAL B 121      25.218  11.846  -13.847  1.00 18.31          O
ATOM   2646  CB  VAL B 121      23.258  11.713  -11.687  1.00 19.82          C
ATOM   2647  CG1 VAL B 121      24.064  10.906  -10.718  1.00 20.45          C
ATOM   2648  CG2 VAL B 121      21.905  12.139  -11.068  1.00 20.23          C
ATOM   2649  N   TYR B 122      26.443  12.681  -12.159  1.00 18.52          N
ATOM   2650  CA  TYR B 122      27.727  12.301  -12.750  1.00 20.03          C
ATOM   2651  C   TYR B 122      28.563  11.513  -11.779  1.00 19.21          C
ATOM   2652  O   TYR B 122      28.647  11.863  -10.595  1.00 16.99          O
ATOM   2653  CB  TYR B 122      28.570  13.512  -13.084  1.00 21.20          C
```

| ATOM | 2654 | CG | TYR | B | 122 | 27.868 | 14.592 | -13.778 | 1.00 | 22.55 | C |
|---|---|---|---|---|---|---|---|---|---|---|---|
| ATOM | 2655 | CD1 | TYR | B | 122 | 27.400 | 14.415 | -15.035 | 1.00 | 22.66 | C |
| ATOM | 2656 | CD2 | TYR | B | 122 | 27.712 | 15.836 | -13.169 | 1.00 | 23.84 | C |
| ATOM | 2657 | CE1 | TYR | B | 122 | 26.754 | 15.435 | -15.685 | 1.00 | 24.70 | C |
| ATOM | 2658 | CE2 | TYR | B | 122 | 27.084 | 16.849 | -13.810 | 1.00 | 24.87 | C |
| ATOM | 2659 | CZ | TYR | B | 122 | 26.618 | 16.649 | -15.069 | 1.00 | 23.10 | C |
| ATOM | 2660 | OH | TYR | B | 122 | 25.973 | 17.700 | -15.716 | 1.00 | 25.54 | O |
| ATOM | 2661 | N | PRO | B | 123 | 29.264 | 10.492 | -12.307 | 1.00 | 21.05 | N |
| ATOM | 2662 | CA | PRO | B | 123 | 30.045 | 9.603 | -11.479 | 1.00 | 22.08 | C |
| ATOM | 2663 | C | PRO | B | 123 | 31.334 | 10.270 | -11.075 | 1.00 | 22.75 | C |
| ATOM | 2664 | O | PRO | B | 123 | 31.940 | 10.990 | -11.887 | 1.00 | 23.75 | O |
| ATOM | 2665 | CB | PRO | B | 123 | 30.334 | 8.421 | -12.420 | 1.00 | 21.99 | C |
| ATOM | 2666 | CG | PRO | B | 123 | 30.424 | 9.063 | -13.789 | 1.00 | 22.88 | C |
| ATOM | 2667 | CD | PRO | B | 123 | 29.381 | 10.165 | -13.748 | 1.00 | 22.25 | C |
| ATOM | 2668 | N | LEU | B | 124 | 31.724 | 10.058 | -9.819 | 1.00 | 23.75 | N |
| ATOM | 2669 | CA | LEU | B | 124 | 33.049 | 10.437 | -9.322 | 1.00 | 22.59 | C |
| ATOM | 2670 | C | LEU | B | 124 | 33.913 | 9.188 | -9.139 | 1.00 | 22.08 | C |
| ATOM | 2671 | O | LEU | B | 124 | 33.733 | 8.406 | -8.224 | 1.00 | 18.72 | O |
| ATOM | 2672 | CB | LEU | B | 124 | 32.951 | 11.230 | -8.030 | 1.00 | 22.95 | C |
| ATOM | 2673 | CG | LEU | B | 124 | 32.010 | 12.442 | -8.082 | 1.00 | 21.36 | C |
| ATOM | 2674 | CD1 | LEU | B | 124 | 31.765 | 12.989 | -6.688 | 1.00 | 22.53 | C |
| ATOM | 2675 | CD2 | LEU | B | 124 | 32.538 | 13.511 | -8.999 | 1.00 | 21.81 | C |
| ATOM | 2676 | N | ALA | B | 125 | 34.850 | 9.025 | -10.059 | 1.00 | 24.60 | N |
| ATOM | 2677 | CA | ALA | B | 125 | 35.775 | 7.922 | -10.027 | 1.00 | 27.07 | C |
| ATOM | 2678 | C | ALA | B | 125 | 37.165 | 8.492 | -9.860 | 1.00 | 28.29 | C |
| ATOM | 2679 | O | ALA | B | 125 | 37.437 | 9.596 | -10.343 | 1.00 | 28.51 | O |
| ATOM | 2680 | CB | ALA | B | 125 | 35.688 | 7.148 | -11.302 | 1.00 | 27.29 | C |
| ATOM | 2681 | N | PRO | B | 126 | 38.064 | 7.734 | -9.187 | 1.00 | 30.43 | N |
| ATOM | 2682 | CA | PRO | B | 126 | 39.478 | 8.108 | -9.113 | 1.00 | 31.80 | C |
| ATOM | 2683 | C | PRO | B | 126 | 40.136 | 8.296 | -10.491 | 1.00 | 32.63 | C |
| ATOM | 2684 | O | PRO | B | 126 | 39.812 | 7.564 | -11.421 | 1.00 | 32.99 | O |
| ATOM | 2685 | CB | PRO | B | 126 | 40.100 | 6.915 | -8.390 | 1.00 | 31.62 | C |
| ATOM | 2686 | CG | PRO | B | 126 | 39.012 | 6.342 | -7.589 | 1.00 | 31.14 | C |
| ATOM | 2687 | CD | PRO | B | 126 | 37.789 | 6.503 | -8.418 | 1.00 | 30.95 | C |
| ATOM | 2688 | N | GLY | B | 127 | 41.054 | 9.264 | -10.596 | 1.00 | 33.52 | N |
| ATOM | 2689 | CA | GLY | B | 127 | 41.766 | 9.581 | -11.847 | 1.00 | 33.74 | C |
| ATOM | 2690 | C | GLY | B | 127 | 42.821 | 8.563 | -12.257 | 1.00 | 34.13 | C |
| ATOM | 2691 | O | GLY | B | 127 | 43.053 | 7.585 | -11.535 | 1.00 | 35.95 | O |
| ATOM | 2692 | N | SER | B | 136 | 43.914 | 0.567 | 0.365 | 1.00 | 31.21 | N |
| ATOM | 2693 | CA | SER | B | 136 | 43.110 | -0.542 | 0.888 | 1.00 | 31.81 | C |
| ATOM | 2694 | C | SER | B | 136 | 41.593 | -0.279 | 0.861 | 1.00 | 31.18 | C |
| ATOM | 2695 | O | SER | B | 136 | 40.796 | -1.197 | 0.675 | 1.00 | 31.18 | O |
| ATOM | 2696 | CB | SER | B | 136 | 43.519 | -0.843 | 2.316 | 1.00 | 32.58 | C |
| ATOM | 2697 | OG | SER | B | 136 | 42.542 | -1.653 | 2.939 | 1.00 | 32.87 | O |
| ATOM | 2698 | N | MET | B | 137 | 41.216 | 0.967 | 1.107 | 1.00 | 30.69 | N |
| ATOM | 2699 | CA | MET | B | 137 | 39.851 | 1.429 | 0.902 | 1.00 | 30.24 | C |
| ATOM | 2700 | C | MET | B | 137 | 39.881 | 2.256 | -0.371 | 1.00 | 29.03 | C |
| ATOM | 2701 | O | MET | B | 137 | 40.920 | 2.801 | -0.728 | 1.00 | 28.50 | O |
| ATOM | 2702 | CB | MET | B | 137 | 39.365 | 2.295 | 2.078 | 1.00 | 30.97 | C |
| ATOM | 2703 | CG | MET | B | 137 | 39.319 | 1.630 | 3.445 | 1.00 | 33.70 | C |
| ATOM | 2704 | SD | MET | B | 137 | 38.326 | 0.113 | 3.551 | 1.00 | 38.41 | S |
| ATOM | 2705 | CE | MET | B | 137 | 36.660 | 0.754 | 3.560 | 1.00 | 37.42 | C |
| ATOM | 2706 | N | VAL | B | 138 | 38.751 | 2.330 | -1.070 | 1.00 | 27.95 | N |
| ATOM | 2707 | CA | VAL | B | 138 | 38.616 | 3.220 | -2.228 | 1.00 | 26.70 | C |
| ATOM | 2708 | C | VAL | B | 138 | 37.371 | 4.066 | -2.004 | 1.00 | 26.07 | C |
| ATOM | 2709 | O | VAL | B | 138 | 36.388 | 3.584 | -1.475 | 1.00 | 24.84 | O |
| ATOM | 2710 | CB | VAL | B | 138 | 38.613 | 2.452 | -3.586 | 1.00 | 26.87 | C |
| ATOM | 2711 | CG1 | VAL | B | 138 | 37.429 | 1.479 | -3.705 | 1.00 | 26.90 | C |
| ATOM | 2712 | CG2 | VAL | B | 138 | 38.643 | 3.416 | -4.737 | 1.00 | 27.48 | C |
| ATOM | 2713 | N | THR | B | 139 | 37.472 | 5.362 | -2.300 | 1.00 | 24.68 | N |
| ATOM | 2714 | CA | THR | B | 139 | 36.350 | 6.282 | -2.152 | 1.00 | 23.76 | C |
| ATOM | 2715 | C | THR | B | 139 | 35.835 | 6.704 | -3.546 | 1.00 | 22.24 | C |
| ATOM | 2716 | O | THR | B | 139 | 36.606 | 7.046 | -4.444 | 1.00 | 22.04 | O |
| ATOM | 2717 | CB | THR | B | 139 | 36.738 | 7.512 | -1.307 | 1.00 | 23.95 | C |
| ATOM | 2718 | OG1 | THR | B | 139 | 37.047 | 7.092 | 0.026 | 1.00 | 23.13 | O |

```
ATOM   2719  CG2 THR B 139      35.601   8.507  -1.250  1.00 24.14           C
ATOM   2720  N   LEU B 140      34.513   6.655  -3.698  1.00 21.41           N
ATOM   2721  CA  LEU B 140      33.821   6.940  -4.940  1.00 21.41           C
ATOM   2722  C   LEU B 140      32.771   7.993  -4.603  1.00 20.17           C
ATOM   2723  O   LEU B 140      32.535   8.281  -3.434  1.00 21.09           O
ATOM   2724  CB  LEU B 140      33.111   5.688  -5.467  1.00 22.13           C
ATOM   2725  CG  LEU B 140      33.976   4.442  -5.723  1.00 23.88           C
ATOM   2726  CD1 LEU B 140      33.121   3.326  -6.288  1.00 25.11           C
ATOM   2727  CD2 LEU B 140      35.133   4.715  -6.661  1.00 23.32           C
ATOM   2728  N   GLY B 141      32.110   8.536  -5.606  1.00 19.99           N
ATOM   2729  CA  GLY B 141      31.053   9.504  -5.324  1.00 20.09           C
ATOM   2730  C   GLY B 141      30.104   9.721  -6.464  1.00 19.34           C
ATOM   2731  O   GLY B 141      30.271   9.165  -7.543  1.00 19.29           O
ATOM   2732  N   CYS B 142      29.059  10.495  -6.196  1.00 21.40           N
ATOM   2733  CA  CYS B 142      28.140  10.958  -7.231  1.00 21.15           C
ATOM   2734  C   CYS B 142      27.919  12.438  -7.030  1.00 20.52           C
ATOM   2735  O   CYS B 142      27.683  12.869  -5.926  1.00 20.32           O
ATOM   2736  CB  CYS B 142      26.813  10.215  -7.177  1.00 24.89           C
ATOM   2737  SG  CYS B 142      26.820   8.840  -8.352  1.00 35.89           S
ATOM   2738  N   LEU B 143      28.038  13.187  -8.108  1.00 19.27           N
ATOM   2739  CA  LEU B 143      27.749  14.593  -8.119  1.00 18.69           C
ATOM   2740  C   LEU B 143      26.341  14.761  -8.675  1.00 17.14           C
ATOM   2741  O   LEU B 143      26.069  14.402  -9.818  1.00 17.48           O
ATOM   2742  CB  LEU B 143      28.751  15.299  -9.019  1.00 19.15           C
ATOM   2743  CG  LEU B 143      28.639  16.810  -9.222  1.00 19.52           C
ATOM   2744  CD1 LEU B 143      28.764  17.565  -7.913  1.00 19.73           C
ATOM   2745  CD2 LEU B 143      29.725  17.201 -10.230  1.00 20.53           C
ATOM   2746  N   VAL B 144      25.468  15.368  -7.879  1.00 16.06           N
ATOM   2747  CA  VAL B 144      24.104  15.631  -8.277  1.00 15.64           C
ATOM   2748  C   VAL B 144      23.916  17.148  -8.433  1.00 15.63           C
ATOM   2749  O   VAL B 144      23.813  17.830  -7.445  1.00 13.13           O
ATOM   2750  CB  VAL B 144      23.180  15.125  -7.206  1.00 15.77           C
ATOM   2751  CG1 VAL B 144      21.708  15.166  -7.683  1.00 16.54           C
ATOM   2752  CG2 VAL B 144      23.600  13.690  -6.834  1.00 13.50           C
ATOM   2753  N   LYS B 145      23.819  17.608  -9.672  1.00 17.11           N
ATOM   2754  CA  LYS B 145      24.003  19.010 -10.007  1.00 17.36           C
ATOM   2755  C   LYS B 145      22.804  19.646 -10.662  1.00 16.21           C
ATOM   2756  O   LYS B 145      22.228  19.107 -11.584  1.00 14.89           O
ATOM   2757  CB  LYS B 145      25.182  19.117 -10.969  1.00 19.37           C
ATOM   2758  CG  LYS B 145      25.590  20.531 -11.259  1.00 20.99           C
ATOM   2759  CD  LYS B 145      27.093  20.671 -11.374  1.00 23.36           C
ATOM   2760  CE  LYS B 145      27.432  22.172 -11.346  1.00 24.98           C
ATOM   2761  NZ  LYS B 145      27.027  22.862 -10.054  1.00 24.71           N
ATOM   2762  N   GLY B 146      22.477  20.859 -10.229  1.00 17.36           N
ATOM   2763  CA  GLY B 146      21.606  21.712 -11.021  1.00 17.27           C
ATOM   2764  C   GLY B 146      20.144  21.427 -10.879  1.00 17.24           C
ATOM   2765  O   GLY B 146      19.416  21.510 -11.855  1.00 16.11           O
ATOM   2766  N   TYR B 147      19.696  21.034  -9.683  1.00 17.97           N
ATOM   2767  CA  TYR B 147      18.265  20.701  -9.488  1.00 17.27           C
ATOM   2768  C   TYR B 147      17.509  21.724  -8.644  1.00 16.42           C
ATOM   2769  O   TYR B 147      18.099  22.514  -7.903  1.00 19.03           O
ATOM   2770  CB  TYR B 147      18.080  19.313  -8.864  1.00 15.81           C
ATOM   2771  CG  TYR B 147      18.632  19.145  -7.467  1.00 14.13           C
ATOM   2772  CD1 TYR B 147      19.981  18.860  -7.240  1.00 14.68           C
ATOM   2773  CD2 TYR B 147      17.795  19.204  -6.378  1.00 12.04           C
ATOM   2774  CE1 TYR B 147      20.468  18.699  -5.957  1.00 13.70           C
ATOM   2775  CE2 TYR B 147      18.267  19.058  -5.106  1.00 15.55           C
ATOM   2776  CZ  TYR B 147      19.594  18.801  -4.891  1.00 15.74           C
ATOM   2777  OH  TYR B 147      20.014  18.641  -3.604  1.00 16.71           O
ATOM   2778  N   PHE B 148      16.191  21.702  -8.777  1.00 17.24           N
ATOM   2779  CA  PHE B 148      15.302  22.498  -7.934  1.00 16.55           C
ATOM   2780  C   PHE B 148      13.911  21.892  -7.901  1.00 16.06           C
ATOM   2781  O   PHE B 148      13.438  21.367  -8.918  1.00 15.96           O
ATOM   2782  CB  PHE B 148      15.236  23.941  -8.452  1.00 17.40           C
ATOM   2783  CG  PHE B 148      14.535  24.854  -7.516  1.00 16.23           C
```

| ATOM | 2784 | CD1 | PHE | B | 148 | 15.214 | 25.429 | -6.477 | 1.00 | 17.72 | C |
|------|------|-----|-----|---|-----|--------|--------|--------|------|-------|---|
| ATOM | 2785 | CD2 | PHE | B | 148 | 13.171 | 25.094 | -7.645 | 1.00 | 19.31 | C |
| ATOM | 2786 | CE1 | PHE | B | 148 | 14.566 | 26.264 | -5.583 | 1.00 | 19.11 | C |
| ATOM | 2787 | CE2 | PHE | B | 148 | 12.510 | 25.907 | -6.745 | 1.00 | 16.68 | C |
| ATOM | 2788 | CZ  | PHE | B | 148 | 13.214 | 26.489 | -5.708 | 1.00 | 18.15 | C |
| ATOM | 2789 | N   | PRO | B | 149 | 13.236 | 21.896 | -6.732 | 1.00 | 17.56 | N |
| ATOM | 2790 | CA  | PRO | B | 149 | 13.607 | 22.259 | -5.398 | 1.00 | 18.26 | C |
| ATOM | 2791 | C   | PRO | B | 149 | 14.304 | 21.053 | -4.769 | 1.00 | 19.50 | C |
| ATOM | 2792 | O   | PRO | B | 149 | 14.370 | 20.004 | -5.386 | 1.00 | 19.62 | O |
| ATOM | 2793 | CB  | PRO | B | 149 | 12.245 | 22.458 | -4.728 | 1.00 | 18.31 | C |
| ATOM | 2794 | CG  | PRO | B | 149 | 11.410 | 21.394 | -5.341 | 1.00 | 16.85 | C |
| ATOM | 2795 | CD  | PRO | B | 149 | 11.849 | 21.401 | -6.782 | 1.00 | 17.33 | C |
| ATOM | 2796 | N   | GLU | B | 150 | 14.799 | 21.239 | -3.557 | 1.00 | 20.66 | N |
| ATOM | 2797 | CA  | GLU | B | 150 | 15.057 | 20.140 | -2.643 | 1.00 | 21.65 | C |
| ATOM | 2798 | C   | GLU | B | 150 | 13.761 | 19.374 | -2.344 | 1.00 | 22.73 | C |
| ATOM | 2799 | O   | GLU | B | 150 | 12.664 | 19.951 | -2.405 | 1.00 | 23.12 | O |
| ATOM | 2800 | CB  | GLU | B | 150 | 15.626 | 20.683 | -1.342 | 1.00 | 22.16 | C |
| ATOM | 2801 | CG  | GLU | B | 150 | 17.044 | 21.122 | -1.449 | 1.00 | 24.86 | C |
| ATOM | 2802 | CD  | GLU | B | 150 | 17.997 | 20.054 | -0.986 | 1.00 | 26.20 | C |
| ATOM | 2803 | OE1 | GLU | B | 150 | 18.500 | 20.220 |  0.136 | 1.00 | 26.76 | O |
| ATOM | 2804 | OE2 | GLU | B | 150 | 18.209 | 19.042 | -1.699 | 1.00 | 28.46 | O |
| ATOM | 2805 | N   | PRO | B | 151 | 13.876 | 18.101 | -1.949 | 1.00 | 23.13 | N |
| ATOM | 2806 | CA  | PRO | B | 151 | 15.101 | 17.351 | -1.742 | 1.00 | 22.96 | C |
| ATOM | 2807 | C   | PRO | B | 151 | 15.433 | 16.426 | -2.887 | 1.00 | 21.21 | C |
| ATOM | 2808 | O   | PRO | B | 151 | 14.612 | 16.213 | -3.793 | 1.00 | 20.23 | O |
| ATOM | 2809 | CB  | PRO | B | 151 | 14.763 | 16.534 | -0.494 | 1.00 | 23.31 | C |
| ATOM | 2810 | CG  | PRO | B | 151 | 13.315 | 16.151 | -0.717 | 1.00 | 23.57 | C |
| ATOM | 2811 | CD  | PRO | B | 151 | 12.705 | 17.293 | -1.551 | 1.00 | 23.70 | C |
| ATOM | 2812 | N   | VAL | B | 152 | 16.639 | 15.871 | -2.839 | 1.00 | 21.17 | N |
| ATOM | 2813 | CA  | VAL | B | 152 | 16.941 | 14.622 | -3.547 | 1.00 | 21.23 | C |
| ATOM | 2814 | C   | VAL | B | 152 | 17.280 | 13.570 | -2.510 | 1.00 | 21.40 | C |
| ATOM | 2815 | O   | VAL | B | 152 | 17.653 | 13.908 | -1.404 | 1.00 | 21.88 | O |
| ATOM | 2816 | CB  | VAL | B | 152 | 18.142 | 14.717 | -4.558 | 1.00 | 20.94 | C |
| ATOM | 2817 | CG1 | VAL | B | 152 | 17.833 | 15.648 | -5.714 | 1.00 | 20.39 | C |
| ATOM | 2818 | CG2 | VAL | B | 152 | 19.459 | 15.063 | -3.859 | 1.00 | 21.96 | C |
| ATOM | 2819 | N   | THR | B | 153 | 17.135 | 12.294 | -2.860 | 1.00 | 22.91 | N |
| ATOM | 2820 | CA  | THR | B | 153 | 17.688 | 11.210 | -2.000 | 1.00 | 22.50 | C |
| ATOM | 2821 | C   | THR | B | 153 | 18.733 | 10.468 | -2.816 | 1.00 | 21.90 | C |
| ATOM | 2822 | O   | THR | B | 153 | 18.527 | 10.204 | -4.000 | 1.00 | 23.52 | O |
| ATOM | 2823 | CB  | THR | B | 153 | 16.614 | 10.204 | -1.513 | 1.00 | 24.28 | C |
| ATOM | 2824 | OG1 | THR | B | 153 | 16.071 |  9.514 | -2.642 | 1.00 | 27.67 | O |
| ATOM | 2825 | CG2 | THR | B | 153 | 15.480 | 10.907 | -0.748 | 1.00 | 25.37 | C |
| ATOM | 2826 | N   | VAL | B | 154 | 19.868 | 10.160 | -2.200 | 1.00 | 20.59 | N |
| ATOM | 2827 | CA  | VAL | B | 154 | 20.855 |  9.334 | -2.841 | 1.00 | 20.83 | C |
| ATOM | 2828 | C   | VAL | B | 154 | 21.028 |  8.082 | -2.031 | 1.00 | 19.78 | C |
| ATOM | 2829 | O   | VAL | B | 154 | 21.177 |  8.139 | -0.820 | 1.00 | 20.63 | O |
| ATOM | 2830 | CB  | VAL | B | 154 | 22.236 | 10.038 | -2.958 | 1.00 | 21.73 | C |
| ATOM | 2831 | CG1 | VAL | B | 154 | 23.142 |  9.297 | -3.946 | 1.00 | 20.94 | C |
| ATOM | 2832 | CG2 | VAL | B | 154 | 22.057 | 11.499 | -3.392 | 1.00 | 22.25 | C |
| ATOM | 2833 | N   | THR | B | 155 | 21.023 |  6.953 | -2.727 | 1.00 | 21.76 | N |
| ATOM | 2834 | CA  | THR | B | 155 | 21.433 |  5.685 | -2.145 | 1.00 | 20.42 | C |
| ATOM | 2835 | C   | THR | B | 155 | 22.525 |  5.060 | -3.007 | 1.00 | 19.19 | C |
| ATOM | 2836 | O   | THR | B | 155 | 22.790 |  5.490 | -4.134 | 1.00 | 18.21 | O |
| ATOM | 2837 | CB  | THR | B | 155 | 20.223 |  4.740 | -1.985 | 1.00 | 20.31 | C |
| ATOM | 2838 | OG1 | THR | B | 155 | 19.684 |  4.446 | -3.259 | 1.00 | 22.49 | O |
| ATOM | 2839 | CG2 | THR | B | 155 | 19.138 |  5.355 | -1.110 | 1.00 | 20.35 | C |
| ATOM | 2840 | N   | TRP | B | 156 | 23.155 |  4.016 | -2.469 | 1.00 | 18.84 | N |
| ATOM | 2841 | CA  | TRP | B | 156 | 24.233 |  3.357 | -3.131 | 1.00 | 19.47 | C |
| ATOM | 2842 | C   | TRP | B | 156 | 23.836 |  1.882 | -3.237 | 1.00 | 20.54 | C |
| ATOM | 2843 | O   | TRP | B | 156 | 23.329 |  1.347 | -2.271 | 1.00 | 21.52 | O |
| ATOM | 2844 | CB  | TRP | B | 156 | 25.503 |  3.557 | -2.320 | 1.00 | 17.62 | C |
| ATOM | 2845 | CG  | TRP | B | 156 | 26.082 |  4.960 | -2.519 | 1.00 | 16.01 | C |
| ATOM | 2846 | CD1 | TRP | B | 156 | 25.883 |  6.035 | -1.734 | 1.00 | 17.00 | C |
| ATOM | 2847 | CD2 | TRP | B | 156 | 26.860 |  5.387 | -3.606 | 1.00 | 15.21 | C |
| ATOM | 2848 | NE1 | TRP | B | 156 | 26.565 |  7.131 | -2.229 | 1.00 | 15.08 | N |

```
ATOM   2849  CE2 TRP B 156      27.165    6.755   -3.395  1.00 16.22           C
ATOM   2850  CE3 TRP B 156      27.363    4.755   -4.740  1.00 15.50           C
ATOM   2851  CZ2 TRP B 156      27.943    7.457   -4.257  1.00 17.85           C
ATOM   2852  CZ3 TRP B 156      28.149    5.432   -5.567  1.00 17.42           C
ATOM   2853  CH2 TRP B 156      28.399    6.814   -5.357  1.00 16.82           C
ATOM   2854  N   ASN B 157      24.009    1.285   -4.416  1.00 23.26           N
ATOM   2855  CA  ASN B 157      23.534   -0.091   -4.697  1.00 24.60           C
ATOM   2856  C   ASN B 157      22.135   -0.350   -4.128  1.00 25.55           C
ATOM   2857  O   ASN B 157      21.914   -1.306   -3.386  1.00 23.98           O
ATOM   2858  CB  ASN B 157      24.554   -1.109   -4.174  1.00 24.21           C
ATOM   2859  CG  ASN B 157      25.717   -1.275   -5.097  1.00 25.98           C
ATOM   2860  OD1 ASN B 157      25.803   -0.607   -6.123  1.00 28.51           O
ATOM   2861  ND2 ASN B 157      26.619   -2.198   -4.767  1.00 26.66           N
ATOM   2862  N   SER B 158      21.209    0.558   -4.455  1.00 27.42           N
ATOM   2863  CA  SER B 158      19.805    0.466   -4.059  1.00 28.29           C
ATOM   2864  C   SER B 158      19.631    0.367   -2.553  1.00 30.22           C
ATOM   2865  O   SER B 158      18.643   -0.202   -2.074  1.00 31.83           O
ATOM   2866  CB  SER B 158      19.131   -0.737   -4.738  1.00 29.05           C
ATOM   2867  OG  SER B 158      19.503   -0.867   -6.096  1.00 30.31           O
ATOM   2868  N   GLY B 159      20.580    0.924   -1.801  1.00 30.29           N
ATOM   2869  CA  GLY B 159      20.538    0.875   -0.341  1.00 30.28           C
ATOM   2870  C   GLY B 159      21.407   -0.197    0.312  1.00 30.29           C
ATOM   2871  O   GLY B 159      21.532   -0.213    1.533  1.00 29.77           O
ATOM   2872  N   SER B 160      22.005   -1.080   -0.492  1.00 30.47           N
ATOM   2873  CA  SER B 160      22.873   -2.155    0.018  1.00 30.75           C
ATOM   2874  C   SER B 160      24.138   -1.681    0.703  1.00 30.40           C
ATOM   2875  O   SER B 160      24.577   -2.289    1.682  1.00 30.30           O
ATOM   2876  CB  SER B 160      23.311   -3.078   -1.114  1.00 31.28           C
ATOM   2877  OG  SER B 160      22.217   -3.787   -1.610  1.00 32.03           O
ATOM   2878  N   LEU B 161      24.766   -0.660    0.128  1.00 29.15           N
ATOM   2879  CA  LEU B 161      25.887    0.010    0.755  1.00 29.32           C
ATOM   2880  C   LEU B 161      25.352    1.162    1.587  1.00 29.67           C
ATOM   2881  O   LEU B 161      24.865    2.149    1.039  1.00 28.42           O
ATOM   2882  CB  LEU B 161      26.865    0.532   -0.303  1.00 29.21           C
ATOM   2883  CG  LEU B 161      27.422   -0.518   -1.261  1.00 29.18           C
ATOM   2884  CD1 LEU B 161      28.331    0.107   -2.304  1.00 28.81           C
ATOM   2885  CD2 LEU B 161      28.162   -1.583   -0.457  1.00 30.12           C
ATOM   2886  N   SER B 162      25.447    1.028    2.910  1.00 30.17           N
ATOM   2887  CA  SER B 162      24.894    2.009    3.850  1.00 29.87           C
ATOM   2888  C   SER B 162      25.964    2.560    4.770  1.00 30.42           C
ATOM   2889  O   SER B 162      25.995    3.759    5.084  1.00 32.11           O
ATOM   2890  CB  SER B 162      23.733    1.397    4.656  1.00 30.43           C
ATOM   2891  OG  SER B 162      24.043    0.126    5.213  1.00 31.34           O
ATOM   2892  N   SER B 163      26.864    1.694    5.191  1.00 29.58           N
ATOM   2893  CA  SER B 163      27.972    2.104    6.017  1.00 28.43           C
ATOM   2894  C   SER B 163      29.069    2.659    5.123  1.00 27.48           C
ATOM   2895  O   SER B 163      29.196    2.270    3.972  1.00 28.46           O
ATOM   2896  CB  SER B 163      28.453    0.927    6.882  1.00 28.94           C
ATOM   2897  OG  SER B 163      29.860    0.839    6.896  1.00 30.87           O
ATOM   2898  N   GLY B 164      29.842    3.606    5.631  1.00 26.29           N
ATOM   2899  CA  GLY B 164      30.851    4.264    4.813  1.00 25.55           C
ATOM   2900  C   GLY B 164      30.284    5.258    3.794  1.00 24.72           C
ATOM   2901  O   GLY B 164      30.969    5.612    2.844  1.00 24.36           O
ATOM   2902  N   VAL B 165      29.035    5.678    3.985  1.00 24.14           N
ATOM   2903  CA  VAL B 165      28.354    6.600    3.062  1.00 24.32           C
ATOM   2904  C   VAL B 165      28.070    7.926    3.760  1.00 24.45           C
ATOM   2905  O   VAL B 165      27.700    7.966    4.924  1.00 26.29           O
ATOM   2906  CB  VAL B 165      26.993    6.021    2.534  1.00 23.49           C
ATOM   2907  CG1 VAL B 165      26.196    7.103    1.761  1.00 24.27           C
ATOM   2908  CG2 VAL B 165      27.203    4.856    1.649  1.00 22.10           C
ATOM   2909  N   HIS B 166      28.251    9.033    3.065  1.00 25.02           N
ATOM   2910  CA  HIS B 166      27.572   10.234    3.519  1.00 24.77           C
ATOM   2911  C   HIS B 166      27.270   11.122    2.348  1.00 22.56           C
ATOM   2912  O   HIS B 166      27.999   11.155    1.379  1.00 21.70           O
ATOM   2913  CB  HIS B 166      28.344   10.976    4.623  1.00 26.29           C
```

```
ATOM   2914  CG  HIS B 166      29.798  11.121   4.341  1.00 26.86           C
ATOM   2915  ND1 HIS B 166      30.758  10.390   5.004  1.00 27.58           N
ATOM   2916  CD2 HIS B 166      30.454  11.906   3.460  1.00 27.00           C
ATOM   2917  CE1 HIS B 166      31.947  10.698   4.523  1.00 29.02           C
ATOM   2918  NE2 HIS B 166      31.795  11.635   3.599  1.00 29.70           N
ATOM   2919  N   THR B 167      26.148  11.813   2.471  1.00 21.44           N
ATOM   2920  CA  THR B 167      25.669  12.696   1.451  1.00 19.99           C
ATOM   2921  C   THR B 167      25.738  14.059   2.119  1.00 19.19           C
ATOM   2922  O   THR B 167      25.360  14.229   3.258  1.00 19.91           O
ATOM   2923  CB  THR B 167      24.264  12.244   0.995  1.00 21.06           C
ATOM   2924  OG1 THR B 167      24.353  10.897   0.485  1.00 20.19           O
ATOM   2925  CG2 THR B 167      23.701  13.131  -0.084  1.00 20.52           C
ATOM   2926  N   PHE B 168      26.312  15.002   1.404  1.00 19.27           N
ATOM   2927  CA  PHE B 168      26.597  16.322   1.914  1.00 18.72           C
ATOM   2928  C   PHE B 168      25.376  17.197   1.658  1.00 19.13           C
ATOM   2929  O   PHE B 168      24.649  17.002   0.662  1.00 17.17           O
ATOM   2930  CB  PHE B 168      27.813  16.874   1.198  1.00 17.24           C
ATOM   2931  CG  PHE B 168      29.074  16.102   1.473  1.00 16.41           C
ATOM   2932  CD1 PHE B 168      29.429  15.022   0.665  1.00 18.42           C
ATOM   2933  CD2 PHE B 168      29.876  16.431   2.545  1.00 17.04           C
ATOM   2934  CE1 PHE B 168      30.602  14.291   0.932  1.00 18.25           C
ATOM   2935  CE2 PHE B 168      31.030  15.714   2.817  1.00 17.19           C
ATOM   2936  CZ  PHE B 168      31.390  14.636   2.007  1.00 16.88           C
ATOM   2937  N   PRO B 169      25.127  18.154   2.560  1.00 19.75           N
ATOM   2938  CA  PRO B 169      24.026  19.095   2.348  1.00 20.64           C
ATOM   2939  C   PRO B 169      24.126  19.802   1.004  1.00 21.29           C
ATOM   2940  O   PRO B 169      25.222  20.059   0.504  1.00 20.33           O
ATOM   2941  CB  PRO B 169      24.194  20.102   3.490  1.00 20.12           C
ATOM   2942  CG  PRO B 169      24.852  19.337   4.566  1.00 21.22           C
ATOM   2943  CD  PRO B 169      25.802  18.374   3.849  1.00 19.53           C
ATOM   2944  N   ALA B 170      22.978  20.109   0.418  1.00 22.20           N
ATOM   2945  CA  ALA B 170      22.956  20.759  -0.883  1.00 22.27           C
ATOM   2946  C   ALA B 170      23.312  22.225  -0.700  1.00 23.44           C
ATOM   2947  O   ALA B 170      23.012  22.814   0.328  1.00 23.75           O
ATOM   2948  CB  ALA B 170      21.596  20.629  -1.505  1.00 21.76           C
ATOM   2949  N   VAL B 171      23.964  22.790  -1.702  1.00 24.53           N
ATOM   2950  CA  VAL B 171      24.310  24.196  -1.731  1.00 25.27           C
ATOM   2951  C   VAL B 171      23.519  24.847  -2.868  1.00 25.43           C
ATOM   2952  O   VAL B 171      23.427  24.294  -3.962  1.00 21.32           O
ATOM   2953  CB  VAL B 171      25.829  24.397  -1.902  1.00 25.92           C
ATOM   2954  CG1 VAL B 171      26.337  23.860  -3.239  1.00 27.00           C
ATOM   2955  CG2 VAL B 171      26.184  25.854  -1.791  1.00 26.32           C
ATOM   2956  N   LEU B 172      22.941  26.012  -2.594  1.00 27.80           N
ATOM   2957  CA  LEU B 172      22.090  26.711  -3.565  1.00 29.70           C
ATOM   2958  C   LEU B 172      22.887  27.760  -4.292  1.00 31.21           C
ATOM   2959  O   LEU B 172      23.544  28.590  -3.665  1.00 31.10           O
ATOM   2960  CB  LEU B 172      20.919  27.392  -2.866  1.00 29.67           C
ATOM   2961  CG  LEU B 172      19.863  28.043  -3.781  1.00 30.08           C
ATOM   2962  CD1 LEU B 172      19.282  27.040  -4.779  1.00 30.22           C
ATOM   2963  CD2 LEU B 172      18.767  28.693  -2.948  1.00 30.80           C
ATOM   2964  N   GLN B 173      22.851  27.715  -5.618  1.00 33.59           N
ATOM   2965  CA  GLN B 173      23.370  28.828  -6.421  1.00 34.38           C
ATOM   2966  C   GLN B 173      22.439  29.104  -7.608  1.00 34.59           C
ATOM   2967  O   GLN B 173      22.063  28.188  -8.345  1.00 34.07           O
ATOM   2968  CB  GLN B 173      24.797  28.546  -6.896  1.00 35.25           C
ATOM   2969  CG  GLN B 173      25.629  29.838  -7.039  1.00 36.79           C
ATOM   2970  CD  GLN B 173      26.978  29.630  -7.693  1.00 37.80           C
ATOM   2971  OE1 GLN B 173      27.110  28.886  -8.677  1.00 40.43           O
ATOM   2972  NE2 GLN B 173      27.997  30.307  -7.160  1.00 41.45           N
ATOM   2973  N   SER B 174      22.050  30.366  -7.771  1.00 35.21           N
ATOM   2974  CA  SER B 174      21.202  30.762  -8.894  1.00 34.72           C
ATOM   2975  C   SER B 174      19.993  29.830  -9.019  1.00 33.90           C
ATOM   2976  O   SER B 174      19.725  29.257 -10.094  1.00 35.06           O
ATOM   2977  CB  SER B 174      22.019  30.783 -10.202  1.00 36.26           C
ATOM   2978  OG  SER B 174      23.408  31.018  -9.966  1.00 38.71           O
```

```
ATOM   2979  N   ASP B 175      19.318  29.656  -7.887  1.00 32.16           N
ATOM   2980  CA  ASP B 175      18.131  28.824  -7.735  1.00 30.88           C
ATOM   2981  C   ASP B 175      18.268  27.419  -8.240  1.00 27.26           C
ATOM   2982  O   ASP B 175      17.290  26.833  -8.664  1.00 27.65           O
ATOM   2983  CB  ASP B 175      16.893  29.495  -8.356  1.00 33.83           C
ATOM   2984  CG  ASP B 175      16.133  30.387  -7.358  1.00 37.34           C
ATOM   2985  OD1 ASP B 175      16.305  30.212  -6.120  1.00 41.36           O
ATOM   2986  OD2 ASP B 175      15.360  31.256  -7.824  1.00 40.88           O
ATOM   2987  N   LEU B 176      19.478  26.872  -8.175  1.00 24.65           N
ATOM   2988  CA  LEU B 176      19.662  25.445  -8.383  1.00 23.54           C
ATOM   2989  C   LEU B 176      20.549  24.870  -7.299  1.00 21.76           C
ATOM   2990  O   LEU B 176      21.472  25.520  -6.803  1.00 19.97           O
ATOM   2991  CB  LEU B 176      20.258  25.150  -9.762  1.00 23.00           C
ATOM   2992  CG  LEU B 176      19.467  25.708 -10.953  1.00 19.10           C
ATOM   2993  CD1 LEU B 176      20.266  25.622 -12.224  1.00 20.43           C
ATOM   2994  CD2 LEU B 176      18.155  25.041 -11.102  1.00 20.27           C
ATOM   2995  N   TYR B 177      20.264  23.626  -6.946  1.00 20.62           N
ATOM   2996  CA  TYR B 177      21.003  22.983  -5.893  1.00 19.34           C
ATOM   2997  C   TYR B 177      21.974  22.010  -6.504  1.00 19.19           C
ATOM   2998  O   TYR B 177      21.706  21.460  -7.565  1.00 18.15           O
ATOM   2999  CB  TYR B 177      20.061  22.207  -4.984  1.00 21.02           C
ATOM   3000  CG  TYR B 177      19.224  23.061  -4.083  1.00 21.40           C
ATOM   3001  CD1 TYR B 177      19.731  23.510  -2.874  1.00 20.36           C
ATOM   3002  CD2 TYR B 177      17.909  23.408  -4.440  1.00 21.73           C
ATOM   3003  CE1 TYR B 177      18.969  24.296  -2.038  1.00 21.70           C
ATOM   3004  CE2 TYR B 177      17.141  24.202  -3.623  1.00 21.42           C
ATOM   3005  CZ  TYR B 177      17.668  24.633  -2.416  1.00 22.65           C
ATOM   3006  OH  TYR B 177      16.913  25.404  -1.572  1.00 24.02           O
ATOM   3007  N   THR B 178      23.083  21.812  -5.806  1.00 18.62           N
ATOM   3008  CA  THR B 178      24.058  20.769  -6.124  1.00 18.30           C
ATOM   3009  C   THR B 178      24.472  20.069  -4.835  1.00 17.10           C
ATOM   3010  O   THR B 178      24.651  20.680  -3.810  1.00 14.96           O
ATOM   3011  CB  THR B 178      25.288  21.315  -6.861  1.00 18.23           C
ATOM   3012  OG1 THR B 178      24.866  21.882  -8.106  1.00 19.26           O
ATOM   3013  CG2 THR B 178      26.312  20.169  -7.180  1.00 19.16           C
ATOM   3014  N   LEU B 179      24.596  18.753  -4.885  1.00 16.47           N
ATOM   3015  CA  LEU B 179      25.168  18.051  -3.767  1.00 16.84           C
ATOM   3016  C   LEU B 179      26.000  16.910  -4.287  1.00 14.80           C
ATOM   3017  O   LEU B 179      25.968  16.635  -5.457  1.00 13.32           O
ATOM   3018  CB  LEU B 179      24.133  17.555  -2.779  1.00 17.56           C
ATOM   3019  CG  LEU B 179      23.094  16.470  -3.063  1.00 16.37           C
ATOM   3020  CD1 LEU B 179      23.716  15.106  -3.342  1.00 17.22           C
ATOM   3021  CD2 LEU B 179      22.190  16.362  -1.850  1.00 17.89           C
ATOM   3022  N   SER B 180      26.794  16.336  -3.405  1.00 15.35           N
ATOM   3023  CA  SER B 180      27.505  15.108  -3.707  1.00 15.54           C
ATOM   3024  C   SER B 180      27.264  14.054  -2.645  1.00 13.41           C
ATOM   3025  O   SER B 180      26.930  14.308  -1.524  1.00 14.22           O
ATOM   3026  CB  SER B 180      29.022  15.367  -3.844  1.00 16.83           C
ATOM   3027  OG  SER B 180      29.386  15.833  -5.154  1.00 19.45           O
ATOM   3028  N   SER B 181      27.550  12.820  -3.011  1.00 15.94           N
ATOM   3029  CA  SER B 181      27.518  11.729  -2.048  1.00 15.96           C
ATOM   3030  C   SER B 181      28.824  10.981  -2.204  1.00 13.73           C
ATOM   3031  O   SER B 181      29.279  10.818  -3.314  1.00 11.92           O
ATOM   3032  CB  SER B 181      26.355  10.785  -2.334  1.00 18.41           C
ATOM   3033  OG  SER B 181      26.232   9.861  -1.273  1.00 17.61           O
ATOM   3034  N   SER B 182      29.434  10.589  -1.097  1.00 16.22           N
ATOM   3035  CA  SER B 182      30.620   9.715  -1.144  1.00 17.58           C
ATOM   3036  C   SER B 182      30.350   8.363  -0.495  1.00 17.56           C
ATOM   3037  O   SER B 182      29.571   8.250   0.453  1.00 18.48           O
ATOM   3038  CB  SER B 182      31.852  10.413  -0.558  1.00 18.11           C
ATOM   3039  OG  SER B 182      32.178  10.021   0.735  1.00 18.24           O
ATOM   3040  N   VAL B 183      30.975   7.339  -1.075  1.00 18.96           N
ATOM   3041  CA  VAL B 183      30.934   5.982  -0.538  1.00 19.20           C
ATOM   3042  C   VAL B 183      32.374   5.488  -0.483  1.00 17.70           C
ATOM   3043  O   VAL B 183      33.128   5.691  -1.400  1.00 16.43           O
```

| ATOM | 3044 | CB | VAL B 183 | 30.052 | 5.033 | -1.395 | 1.00 18.68 | C |
|------|------|-----|-----------|--------|-------|--------|------------|---|
| ATOM | 3045 | CG1 | VAL B 183 | 30.484 | 5.013 | -2.846 | 1.00 18.37 | C |
| ATOM | 3046 | CG2 | VAL B 183 | 30.013 | 3.609 | -0.773 | 1.00 20.28 | C |
| ATOM | 3047 | N | THR B 184 | 32.728 | 4.858 | 0.616 | 1.00 20.77 | N |
| ATOM | 3048 | CA | THR B 184 | 34.041 | 4.242 | 0.752 | 1.00 22.14 | C |
| ATOM | 3049 | C | THR B 184 | 33.895 | 2.713 | 0.887 | 1.00 21.94 | C |
| ATOM | 3050 | O | THR B 184 | 33.257 | 2.249 | 1.820 | 1.00 22.32 | O |
| ATOM | 3051 | CB | THR B 184 | 34.753 | 4.806 | 1.973 | 1.00 22.56 | C |
| ATOM | 3052 | OG1 | THR B 184 | 34.829 | 6.244 | 1.878 | 1.00 23.72 | O |
| ATOM | 3053 | CG2 | THR B 184 | 36.146 | 4.236 | 2.099 | 1.00 22.19 | C |
| ATOM | 3054 | N | VAL B 185 | 34.514 | 1.983 | -0.033 | 1.00 23.44 | N |
| ATOM | 3055 | CA | VAL B 185 | 34.498 | 0.506 | -0.050 | 1.00 26.40 | C |
| ATOM | 3056 | C | VAL B 185 | 35.946 | -0.039 | -0.089 | 1.00 27.88 | C |
| ATOM | 3057 | O | VAL B 185 | 36.877 | 0.730 | -0.365 | 1.00 28.63 | O |
| ATOM | 3058 | CB | VAL B 185 | 33.663 | -0.037 | -1.234 | 1.00 25.40 | C |
| ATOM | 3059 | CG1 | VAL B 185 | 32.255 | 0.544 | -1.194 | 1.00 26.82 | C |
| ATOM | 3060 | CG2 | VAL B 185 | 34.297 | 0.218 | -2.582 | 1.00 25.32 | C |
| ATOM | 3061 | N | PRO B 186 | 36.151 | -1.335 | 0.261 | 1.00 29.38 | N |
| ATOM | 3062 | CA | PRO B 186 | 37.444 | -1.985 | 0.024 | 1.00 29.55 | C |
| ATOM | 3063 | C | PRO B 186 | 37.906 | -2.009 | -1.435 | 1.00 30.25 | C |
| ATOM | 3064 | O | PRO B 186 | 37.105 | -2.211 | -2.340 | 1.00 30.43 | O |
| ATOM | 3065 | CB | PRO B 186 | 37.225 | -3.406 | 0.568 | 1.00 29.63 | C |
| ATOM | 3066 | CG | PRO B 186 | 36.204 | -3.245 | 1.627 | 1.00 29.89 | C |
| ATOM | 3067 | CD | PRO B 186 | 35.248 | -2.205 | 1.033 | 1.00 29.29 | C |
| ATOM | 3068 | N | SER B 187 | 39.210 | -1.842 | -1.634 | 1.00 32.17 | N |
| ATOM | 3069 | CA | SER B 187 | 39.842 | -1.680 | -2.947 | 1.00 34.18 | C |
| ATOM | 3070 | C | SER B 187 | 39.592 | -2.792 | -3.960 | 1.00 35.29 | C |
| ATOM | 3071 | O | SER B 187 | 39.353 | -2.535 | -5.142 | 1.00 36.84 | O |
| ATOM | 3072 | CB | SER B 187 | 41.357 | -1.561 | -2.776 | 1.00 34.95 | C |
| ATOM | 3073 | OG | SER B 187 | 41.700 | -0.297 | -2.253 | 1.00 38.40 | O |
| ATOM | 3074 | N | SER B 188 | 39.733 | -4.024 | -3.501 | 1.00 35.69 | N |
| ATOM | 3075 | CA | SER B 188 | 39.493 | -5.202 | -4.326 | 1.00 36.71 | C |
| ATOM | 3076 | C | SER B 188 | 38.061 | -5.234 | -4.879 | 1.00 37.68 | C |
| ATOM | 3077 | O | SER B 188 | 37.851 | -5.648 | -6.013 | 1.00 38.64 | O |
| ATOM | 3078 | CB | SER B 188 | 39.749 | -6.466 | -3.490 | 1.00 36.09 | C |
| ATOM | 3079 | OG | SER B 188 | 38.828 | -6.533 | -2.405 | 1.00 34.35 | O |
| ATOM | 3080 | N | THR B 189 | 37.096 | -4.775 | -4.078 | 1.00 38.65 | N |
| ATOM | 3081 | CA | THR B 189 | 35.671 | -4.933 | -4.394 | 1.00 38.74 | C |
| ATOM | 3082 | C | THR B 189 | 35.241 | -4.155 | -5.629 | 1.00 38.77 | C |
| ATOM | 3083 | O | THR B 189 | 34.430 | -4.639 | -6.412 | 1.00 39.21 | O |
| ATOM | 3084 | CB | THR B 189 | 34.774 | -4.509 | -3.240 | 1.00 39.20 | C |
| ATOM | 3085 | OG1 | THR B 189 | 35.015 | -3.138 | -2.943 | 1.00 42.27 | O |
| ATOM | 3086 | CG2 | THR B 189 | 35.042 | -5.351 | -2.003 | 1.00 40.45 | C |
| ATOM | 3087 | N | TRP B 190 | 35.766 | -2.949 | -5.792 | 1.00 37.42 | N |
| ATOM | 3088 | CA | TRP B 190 | 35.454 | -2.145 | -6.952 | 1.00 36.92 | C |
| ATOM | 3089 | C | TRP B 190 | 36.725 | -1.966 | -7.740 | 1.00 36.34 | C |
| ATOM | 3090 | O | TRP B 190 | 37.759 | -1.686 | -7.158 | 1.00 36.80 | O |
| ATOM | 3091 | CB | TRP B 190 | 34.912 | -0.790 | -6.494 | 1.00 35.35 | C |
| ATOM | 3092 | CG | TRP B 190 | 34.601 | 0.081 | -7.627 | 1.00 34.06 | C |
| ATOM | 3093 | CD1 | TRP B 190 | 33.436 | 0.121 | -8.333 | 1.00 33.87 | C |
| ATOM | 3094 | CD2 | TRP B 190 | 35.482 | 1.018 | -8.240 | 1.00 34.26 | C |
| ATOM | 3095 | NE1 | TRP B 190 | 33.538 | 1.030 | -9.355 | 1.00 33.35 | N |
| ATOM | 3096 | CE2 | TRP B 190 | 34.785 | 1.602 | -9.315 | 1.00 33.19 | C |
| ATOM | 3097 | CE3 | TRP B 190 | 36.794 | 1.427 | -7.981 | 1.00 33.42 | C |
| ATOM | 3098 | CZ2 | TRP B 190 | 35.353 | 2.575 | -10.126 | 1.00 33.36 | C |
| ATOM | 3099 | CZ3 | TRP B 190 | 37.358 | 2.381 | -8.793 | 1.00 33.47 | C |
| ATOM | 3100 | CH2 | TRP B 190 | 36.638 | 2.951 | -9.854 | 1.00 34.01 | C |
| ATOM | 3101 | N | PRO B 191 | 36.670 | -2.087 | -9.072 | 1.00 36.40 | N |
| ATOM | 3102 | CA | PRO B 191 | 35.574 | -2.356 | -10.004 | 1.00 37.00 | C |
| ATOM | 3103 | C | PRO B 191 | 35.067 | -3.802 | -10.148 | 1.00 36.97 | C |
| ATOM | 3104 | O | PRO B 191 | 34.153 | -4.044 | -10.939 | 1.00 38.42 | O |
| ATOM | 3105 | CB | PRO B 191 | 36.141 | -1.876 | -11.342 | 1.00 36.95 | C |
| ATOM | 3106 | CG | PRO B 191 | 37.598 | -2.049 | -11.217 | 1.00 36.79 | C |
| ATOM | 3107 | CD | PRO B 191 | 37.932 | -1.813 | -9.778 | 1.00 36.29 | C |
| ATOM | 3108 | N | SER B 192 | 35.612 | -4.748 | -9.401 | 1.00 37.04 | N |

| | | | | | | | | | | |
|---|---|---|---|---|---|---|---|---|---|---|
| ATOM | 3109 | CA | SER | B | 192 | 35.215 | -6.158 | -9.581 | 1.00 36.56 | C |
| ATOM | 3110 | C | SER | B | 192 | 33.719 | -6.379 | -9.311 | 1.00 36.40 | C |
| ATOM | 3111 | O | SER | B | 192 | 33.028 | -7.074 | -10.069 | 1.00 35.80 | O |
| ATOM | 3112 | CB | SER | B | 192 | 36.053 | -7.066 | -8.674 | 1.00 36.80 | C |
| ATOM | 3113 | OG | SER | B | 192 | 35.806 | -6.809 | -7.298 | 1.00 37.33 | O |
| ATOM | 3114 | N | GLU | B | 193 | 33.237 | -5.796 | -8.217 | 1.00 35.80 | N |
| ATOM | 3115 | CA | GLU | B | 193 | 31.813 | -5.732 | -7.912 | 1.00 35.07 | C |
| ATOM | 3116 | C | GLU | B | 193 | 31.301 | -4.331 | -8.259 | 1.00 34.35 | C |
| ATOM | 3117 | O | GLU | B | 193 | 32.031 | -3.341 | -8.132 | 1.00 33.52 | O |
| ATOM | 3118 | CB | GLU | B | 193 | 31.566 | -6.019 | -6.435 | 1.00 36.00 | C |
| ATOM | 3119 | CG | GLU | B | 193 | 32.123 | -7.367 | -5.932 | 1.00 37.52 | C |
| ATOM | 3120 | CD | GLU | B | 193 | 31.431 | -8.583 | -6.541 | 1.00 39.10 | C |
| ATOM | 3121 | OE1 | GLU | B | 193 | 32.062 | -9.670 | -6.625 | 1.00 40.18 | O |
| ATOM | 3122 | OE2 | GLU | B | 193 | 30.252 | -8.465 | -6.934 | 1.00 42.29 | O |
| ATOM | 3123 | N | THR | B | 194 | 30.044 | -4.251 | -8.692 | 1.00 33.13 | N |
| ATOM | 3124 | CA | THR | B | 194 | 29.491 | -2.977 | -9.177 | 1.00 32.39 | C |
| ATOM | 3125 | C | THR | B | 194 | 29.084 | -2.042 | -8.036 | 1.00 30.61 | C |
| ATOM | 3126 | O | THR | B | 194 | 28.514 | -2.475 | -7.025 | 1.00 29.74 | O |
| ATOM | 3127 | CB | THR | B | 194 | 28.278 | -3.200 | -10.084 | 1.00 33.07 | C |
| ATOM | 3128 | OG1 | THR | B | 194 | 27.298 | -3.941 | -9.364 | 1.00 35.36 | O |
| ATOM | 3129 | CG2 | THR | B | 194 | 28.654 | -3.956 | -11.344 | 1.00 33.75 | C |
| ATOM | 3130 | N | VAL | B | 195 | 29.394 | -0.751 | -8.202 | 1.00 29.60 | N |
| ATOM | 3131 | CA | VAL | B | 195 | 28.910 | 0.294 | -7.303 | 1.00 28.14 | C |
| ATOM | 3132 | C | VAL | B | 195 | 28.135 | 1.330 | -8.124 | 1.00 25.76 | C |
| ATOM | 3133 | O | VAL | B | 195 | 28.606 | 1.844 | -9.132 | 1.00 24.08 | O |
| ATOM | 3134 | CB | VAL | B | 195 | 30.020 | 0.983 | -6.517 | 1.00 28.60 | C |
| ATOM | 3135 | CG1 | VAL | B | 195 | 29.423 | 1.974 | -5.523 | 1.00 28.81 | C |
| ATOM | 3136 | CG2 | VAL | B | 195 | 30.891 | -0.047 | -5.748 | 1.00 29.48 | C |
| ATOM | 3137 | N | THR | B | 196 | 26.933 | 1.614 | -7.657 | 1.00 26.01 | N |
| ATOM | 3138 | CA | THR | B | 196 | 25.973 | 2.430 | -8.392 | 1.00 25.26 | C |
| ATOM | 3139 | C | THR | B | 196 | 25.279 | 3.389 | -7.444 | 1.00 23.36 | C |
| ATOM | 3140 | O | THR | B | 196 | 24.803 | 2.979 | -6.406 | 1.00 22.12 | O |
| ATOM | 3141 | CB | THR | B | 196 | 24.936 | 1.485 | -9.010 | 1.00 24.84 | C |
| ATOM | 3142 | OG1 | THR | B | 196 | 25.567 | 0.780 | -10.072 | 1.00 25.12 | O |
| ATOM | 3143 | CG2 | THR | B | 196 | 23.702 | 2.236 | -9.539 | 1.00 25.42 | C |
| ATOM | 3144 | N | CYS | B | 197 | 25.214 | 4.674 | -7.792 | 1.00 23.12 | N |
| ATOM | 3145 | CA | CYS | B | 197 | 24.443 | 5.592 | -6.971 | 1.00 22.93 | C |
| ATOM | 3146 | C | CYS | B | 197 | 23.086 | 5.808 | -7.614 | 1.00 21.57 | C |
| ATOM | 3147 | O | CYS | B | 197 | 22.965 | 5.920 | -8.823 | 1.00 22.84 | O |
| ATOM | 3148 | CB | CYS | B | 197 | 25.161 | 6.909 | -6.734 | 1.00 25.42 | C |
| ATOM | 3149 | SG | CYS | B | 197 | 24.965 | 8.029 | -8.073 | 1.00 30.07 | S |
| ATOM | 3150 | N | ASN | B | 198 | 22.073 | 5.817 | -6.771 | 1.00 20.13 | N |
| ATOM | 3151 | CA | ASN | B | 198 | 20.679 | 5.892 | -7.184 | 1.00 19.95 | C |
| ATOM | 3152 | C | ASN | B | 198 | 20.173 | 7.206 | -6.649 | 1.00 17.26 | C |
| ATOM | 3153 | O | ASN | B | 198 | 20.207 | 7.422 | -5.458 | 1.00 17.28 | O |
| ATOM | 3154 | CB | ASN | B | 198 | 19.881 | 4.764 | -6.540 | 1.00 18.90 | C |
| ATOM | 3155 | CG | ASN | B | 198 | 20.630 | 3.466 | -6.497 | 1.00 19.43 | C |
| ATOM | 3156 | OD1 | ASN | B | 198 | 21.021 | 3.025 | -5.429 | 1.00 23.88 | O |
| ATOM | 3157 | ND2 | ASN | B | 198 | 20.840 | 2.853 | -7.643 | 1.00 21.89 | N |
| ATOM | 3158 | N | VAL | B | 199 | 19.781 | 8.104 | -7.544 | 1.00 17.63 | N |
| ATOM | 3159 | CA | VAL | B | 199 | 19.327 | 9.420 | -7.161 | 1.00 17.67 | C |
| ATOM | 3160 | C | VAL | B | 199 | 17.851 | 9.564 | -7.516 | 1.00 17.78 | C |
| ATOM | 3161 | O | VAL | B | 199 | 17.446 | 9.218 | -8.612 | 1.00 20.51 | O |
| ATOM | 3162 | CB | VAL | B | 199 | 20.151 | 10.504 | -7.888 | 1.00 15.77 | C |
| ATOM | 3163 | CG1 | VAL | B | 199 | 19.661 | 11.914 | -7.495 | 1.00 16.73 | C |
| ATOM | 3164 | CG2 | VAL | B | 199 | 21.600 | 10.337 | -7.527 | 1.00 18.09 | C |
| ATOM | 3165 | N | ALA | B | 200 | 17.053 | 10.017 | -6.561 | 1.00 18.67 | N |
| ATOM | 3166 | CA | ALA | B | 200 | 15.651 | 10.345 | -6.809 | 1.00 19.10 | C |
| ATOM | 3167 | C | ALA | B | 200 | 15.387 | 11.838 | -6.551 | 1.00 18.83 | C |
| ATOM | 3168 | O | ALA | B | 200 | 15.868 | 12.380 | -5.567 | 1.00 18.72 | O |
| ATOM | 3169 | CB | ALA | B | 200 | 14.784 | 9.544 | -5.942 | 1.00 18.42 | C |
| ATOM | 3170 | N | HIS | B | 201 | 14.615 | 12.458 | -7.443 | 1.00 19.60 | N |
| ATOM | 3171 | CA | HIS | B | 201 | 14.156 | 13.843 | -7.284 | 1.00 20.66 | C |
| ATOM | 3172 | C | HIS | B | 201 | 12.655 | 13.793 | -7.479 | 1.00 21.75 | C |
| ATOM | 3173 | O | HIS | B | 201 | 12.177 | 13.878 | -8.603 | 1.00 21.88 | O |

```
ATOM   3174  CB  HIS B 201      14.778  14.762   -8.326  1.00 20.44           C
ATOM   3175  CG  HIS B 201      14.502  16.218   -8.112  1.00 18.38           C
ATOM   3176  ND1 HIS B 201      14.039  17.039   -9.116  1.00 16.16           N
ATOM   3177  CD2 HIS B 201      14.624  16.999   -7.013  1.00 17.34           C
ATOM   3178  CE1 HIS B 201      13.919  18.269   -8.651  1.00 18.88           C
ATOM   3179  NE2 HIS B 201      14.246  18.270   -7.372  1.00 17.02           N
ATOM   3180  N   PRO B 202      11.915  13.625   -6.385  1.00 23.11           N
ATOM   3181  CA  PRO B 202      10.460  13.484   -6.447  1.00 24.20           C
ATOM   3182  C   PRO B 202       9.761  14.587   -7.245  1.00 24.53           C
ATOM   3183  O   PRO B 202       8.868  14.288   -8.019  1.00 25.78           O
ATOM   3184  CB  PRO B 202      10.038  13.538   -4.971  1.00 24.79           C
ATOM   3185  CG  PRO B 202      11.186  13.078   -4.228  1.00 25.53           C
ATOM   3186  CD  PRO B 202      12.403  13.559   -4.996  1.00 24.40           C
ATOM   3187  N   ALA B 203      10.189  15.835   -7.107  1.00 24.16           N
ATOM   3188  CA  ALA B 203       9.487  16.941   -7.761  1.00 24.17           C
ATOM   3189  C   ALA B 203       9.471  16.867   -9.299  1.00 24.56           C
ATOM   3190  O   ALA B 203       8.587  17.432   -9.931  1.00 22.44           O
ATOM   3191  CB  ALA B 203      10.071  18.284   -7.298  1.00 24.54           C
ATOM   3192  N   SER B 204      10.453  16.197   -9.907  1.00 23.25           N
ATOM   3193  CA  SER B 204      10.484  16.069  -11.349  1.00 23.76           C
ATOM   3194  C   SER B 204      10.191  14.629  -11.766  1.00 24.24           C
ATOM   3195  O   SER B 204      10.165  14.317  -12.970  1.00 26.82           O
ATOM   3196  CB  SER B 204      11.827  16.528  -11.909  1.00 23.89           C
ATOM   3197  OG  SER B 204      12.884  15.861  -11.259  1.00 23.93           O
ATOM   3198  N   SER B 205       9.941  13.786  -10.775  1.00 23.68           N
ATOM   3199  CA  SER B 205       9.731  12.352  -10.969  1.00 24.93           C
ATOM   3200  C   SER B 205      10.913  11.649  -11.641  1.00 25.16           C
ATOM   3201  O   SER B 205      10.753  10.707  -12.420  1.00 25.19           O
ATOM   3202  CB  SER B 205       8.407  12.098  -11.699  1.00 25.34           C
ATOM   3203  OG  SER B 205       7.357  12.122  -10.755  1.00 28.54           O
ATOM   3204  N   THR B 206      12.110  12.108  -11.306  1.00 24.11           N
ATOM   3205  CA  THR B 206      13.338  11.537  -11.801  1.00 25.07           C
ATOM   3206  C   THR B 206      13.853  10.435  -10.858  1.00 25.15           C
ATOM   3207  O   THR B 206      13.899  10.599   -9.644  1.00 22.99           O
ATOM   3208  CB  THR B 206      14.418  12.609  -11.892  1.00 26.14           C
ATOM   3209  OG1 THR B 206      14.464  13.278  -10.636  1.00 30.85           O
ATOM   3210  CG2 THR B 206      14.084  13.615  -12.949  1.00 22.98           C
ATOM   3211  N   LYS B 207      14.206   9.305  -11.458  1.00 25.63           N
ATOM   3212  CA  LYS B 207      14.904   8.227  -10.788  1.00 25.95           C
ATOM   3213  C   LYS B 207      16.028   7.843  -11.729  1.00 24.53           C
ATOM   3214  O   LYS B 207      15.776   7.408  -12.862  1.00 24.12           O
ATOM   3215  CB  LYS B 207      13.983   7.028  -10.568  1.00 26.37           C
ATOM   3216  CG  LYS B 207      14.697   5.813   -9.997  1.00 29.63           C
ATOM   3217  CD  LYS B 207      14.386   4.456  -10.726  1.00 30.66           C
ATOM   3218  CE  LYS B 207      15.196   4.294  -12.032  1.00 32.91           C
ATOM   3219  NZ  LYS B 207      15.676   2.896  -12.256  1.00 33.30           N
ATOM   3220  N   VAL B 208      17.263   8.009  -11.267  1.00 23.21           N
ATOM   3221  CA  VAL B 208      18.429   7.776  -12.088  1.00 23.76           C
ATOM   3222  C   VAL B 208      19.378   6.852  -11.349  1.00 21.97           C
ATOM   3223  O   VAL B 208      19.496   6.905  -10.144  1.00 19.65           O
ATOM   3224  CB  VAL B 208      19.166   9.135  -12.414  1.00 24.77           C
ATOM   3225  CG1 VAL B 208      20.599   8.900  -12.919  1.00 26.58           C
ATOM   3226  CG2 VAL B 208      18.369   9.952  -13.429  1.00 26.36           C
ATOM   3227  N   ASP B 209      20.051   5.997  -12.094  1.00 23.09           N
ATOM   3228  CA  ASP B 209      21.105   5.181  -11.550  1.00 24.29           C
ATOM   3229  C   ASP B 209      22.355   5.471  -12.334  1.00 24.60           C
ATOM   3230  O   ASP B 209      22.307   5.562  -13.565  1.00 24.36           O
ATOM   3231  CB  ASP B 209      20.766   3.710  -11.708  1.00 26.02           C
ATOM   3232  CG  ASP B 209      19.600   3.295  -10.856  1.00 27.57           C
ATOM   3233  OD1 ASP B 209      19.415   3.849   -9.751  1.00 28.25           O
ATOM   3234  OD2 ASP B 209      18.849   2.413  -11.317  1.00 32.96           O
ATOM   3235  N   LYS B 210      23.470   5.619  -11.625  1.00 23.97           N
ATOM   3236  CA  LYS B 210      24.739   5.880  -12.264  1.00 24.40           C
ATOM   3237  C   LYS B 210      25.788   4.888  -11.748  1.00 23.98           C
ATOM   3238  O   LYS B 210      26.211   4.938  -10.594  1.00 22.70           O
```

| ATOM | 3239 | CB | LYS | B | 210 | 25.156 | 7.342 | -12.019 | 1.00 | 23.77 | C |
|------|------|-----|-----|---|-----|--------|--------|---------|------|-------|---|
| ATOM | 3240 | CG | LYS | B | 210 | 26.508 | 7.719 | -12.634 | 1.00 | 25.03 | C |
| ATOM | 3241 | CD | LYS | B | 210 | 26.575 | 7.589 | -14.158 | 1.00 | 25.32 | C |
| ATOM | 3242 | CE | LYS | B | 210 | 25.591 | 8.517 | -14.854 | 1.00 | 25.13 | C |
| ATOM | 3243 | NZ | LYS | B | 210 | 25.852 | 8.609 | -16.316 | 1.00 | 27.60 | N |
| ATOM | 3244 | N | LYS | B | 211 | 26.181 | 3.963 | -12.619 | 1.00 | 25.27 | N |
| ATOM | 3245 | CA | LYS | B | 211 | 27.277 | 3.056 | -12.297 | 1.00 | 26.10 | C |
| ATOM | 3246 | C | LYS | B | 211 | 28.568 | 3.865 | -12.302 | 1.00 | 25.34 | C |
| ATOM | 3247 | O | LYS | B | 211 | 28.797 | 4.681 | -13.192 | 1.00 | 25.03 | O |
| ATOM | 3248 | CB | LYS | B | 211 | 27.367 | 1.923 | -13.309 | 1.00 | 26.02 | C |
| ATOM | 3249 | CG | LYS | B | 211 | 28.223 | 0.751 | -12.865 | 1.00 | 26.44 | C |
| ATOM | 3250 | CD | LYS | B | 211 | 28.274 | -0.241 | -14.007 | 1.00 | 28.10 | C |
| ATOM | 3251 | CE | LYS | B | 211 | 29.188 | -1.425 | -13.715 | 1.00 | 29.25 | C |
| ATOM | 3252 | NZ | LYS | B | 211 | 29.806 | -1.912 | -14.997 | 1.00 | 31.58 | N |
| ATOM | 3253 | N | ILE | B | 212 | 29.394 | 3.639 | -11.288 | 1.00 | 26.63 | N |
| ATOM | 3254 | CA | ILE | B | 212 | 30.707 | 4.240 | -11.211 | 1.00 | 27.88 | C |
| ATOM | 3255 | C | ILE | B | 212 | 31.693 | 3.289 | -11.855 | 1.00 | 28.42 | C |
| ATOM | 3256 | O | ILE | B | 212 | 31.966 | 2.237 | -11.306 | 1.00 | 28.61 | O |
| ATOM | 3257 | CB | ILE | B | 212 | 31.185 | 4.503 | -9.750 | 1.00 | 26.75 | C |
| ATOM | 3258 | CG1 | ILE | B | 212 | 30.094 | 5.121 | -8.881 | 1.00 | 27.53 | C |
| ATOM | 3259 | CG2 | ILE | B | 212 | 32.378 | 5.440 | -9.775 | 1.00 | 26.77 | C |
| ATOM | 3260 | CD1 | ILE | B | 212 | 29.500 | 6.400 | -9.465 | 1.00 | 27.51 | C |
| ATOM | 3261 | N | VAL | B | 213 | 32.223 | 3.652 | -13.013 | 1.00 | 30.21 | N |
| ATOM | 3262 | CA | VAL | B | 213 | 33.254 | 2.835 | -13.644 | 1.00 | 31.83 | C |
| ATOM | 3263 | C | VAL | B | 213 | 34.609 | 3.536 | -13.596 | 1.00 | 32.64 | C |
| ATOM | 3264 | O | VAL | B | 213 | 34.680 | 4.775 | -13.647 | 1.00 | 30.70 | O |
| ATOM | 3265 | CB | VAL | B | 213 | 32.897 | 2.427 | -15.087 | 1.00 | 32.45 | C |
| ATOM | 3266 | CG1 | VAL | B | 213 | 31.480 | 1.857 | -15.137 | 1.00 | 33.14 | C |
| ATOM | 3267 | CG2 | VAL | B | 213 | 33.045 | 3.590 | -16.040 | 1.00 | 33.71 | C |
| ATOM | 3268 | N | PRO | B | 214 | 35.694 | 2.730 | -13.472 | 1.00 | 34.72 | N |
| ATOM | 3269 | CA | PRO | B | 214 | 37.070 | 3.232 | -13.566 | 1.00 | 35.24 | C |
| ATOM | 3270 | C | PRO | B | 214 | 37.258 | 4.065 | -14.812 | 1.00 | 35.72 | C |
| ATOM | 3271 | O | PRO | B | 214 | 36.627 | 3.797 | -15.832 | 1.00 | 36.46 | O |
| ATOM | 3272 | CB | PRO | B | 214 | 37.911 | 1.952 | -13.655 | 1.00 | 35.17 | C |
| ATOM | 3273 | CG | PRO | B | 214 | 37.090 | 0.910 | -12.975 | 1.00 | 35.08 | C |
| ATOM | 3274 | CD | PRO | B | 214 | 35.657 | 1.272 | -13.214 | 1.00 | 34.45 | C |
| ATOM | 3275 | N | ARG | B | 215 | 38.106 | 5.078 | -14.723 | 1.00 | 37.26 | N |
| ATOM | 3276 | CA | ARG | B | 215 | 38.303 | 5.996 | -15.822 | 1.00 | 37.61 | C |
| ATOM | 3277 | C | ARG | B | 215 | 39.315 | 5.415 | -16.794 | 1.00 | 39.16 | C |
| ATOM | 3278 | O | ARG | B | 215 | 38.944 | 4.952 | -17.872 | 1.00 | 40.42 | O |
| ATOM | 3279 | CB | ARG | B | 215 | 38.779 | 7.350 | -15.295 | 1.00 | 38.36 | C |
| ATOM | 3280 | CG | ARG | B | 215 | 37.783 | 8.019 | -14.368 | 1.00 | 38.61 | C |
| ATOM | 3281 | CD | ARG | B | 215 | 38.330 | 9.286 | -13.707 | 1.00 | 39.44 | C |
| ATOM | 3282 | NE | ARG | B | 215 | 39.184 | 10.071 | -14.592 | 1.00 | 40.62 | N |
| ATOM | 3283 | CZ | ARG | B | 215 | 38.767 | 11.011 | -15.443 | 1.00 | 41.65 | C |
| ATOM | 3284 | NH1 | ARG | B | 215 | 37.471 | 11.307 | -15.570 | 1.00 | 41.74 | N |
| ATOM | 3285 | NH2 | ARG | B | 215 | 39.665 | 11.657 | -16.183 | 1.00 | 41.73 | N |
| TER | 3286 | | ARG | B | 215 | | | | | | |
| ATOM | 3287 | N | ASP | C | 1 | 32.649 | 35.782 | 1.851 | 1.00 | 30.36 | N |
| ATOM | 3288 | CA | ASP | C | 1 | 31.717 | 36.607 | 1.017 | 1.00 | 28.59 | C |
| ATOM | 3289 | C | ASP | C | 1 | 30.375 | 36.676 | 1.705 | 1.00 | 27.84 | C |
| ATOM | 3290 | O | ASP | C | 1 | 29.702 | 35.652 | 1.883 | 1.00 | 28.76 | O |
| ATOM | 3291 | CB | ASP | C | 1 | 31.530 | 36.004 | -0.363 | 1.00 | 30.92 | C |
| ATOM | 3292 | CG | ASP | C | 1 | 32.733 | 36.222 | -1.272 | 1.00 | 32.46 | C |
| ATOM | 3293 | OD1 | ASP | C | 1 | 33.875 | 36.367 | -0.767 | 1.00 | 34.57 | O |
| ATOM | 3294 | OD2 | ASP | C | 1 | 32.510 | 36.282 | -2.497 | 1.00 | 37.34 | O |
| ATOM | 3295 | N | ILE | C | 2 | 29.981 | 37.879 | 2.099 | 1.00 | 24.44 | N |
| ATOM | 3296 | CA | ILE | C | 2 | 28.712 | 38.052 | 2.784 | 1.00 | 22.67 | C |
| ATOM | 3297 | C | ILE | C | 2 | 27.597 | 37.919 | 1.753 | 1.00 | 21.07 | C |
| ATOM | 3298 | O | ILE | C | 2 | 27.671 | 38.531 | 0.670 | 1.00 | 20.04 | O |
| ATOM | 3299 | CB | ILE | C | 2 | 28.646 | 39.418 | 3.489 | 1.00 | 21.52 | C |
| ATOM | 3300 | CG1 | ILE | C | 2 | 29.673 | 39.476 | 4.623 | 1.00 | 21.30 | C |
| ATOM | 3301 | CG2 | ILE | C | 2 | 27.250 | 39.670 | 4.034 | 1.00 | 23.46 | C |
| ATOM | 3302 | CD1 | ILE | C | 2 | 29.826 | 40.851 | 5.220 | 1.00 | 22.61 | C |
| ATOM | 3303 | N | VAL | C | 3 | 26.570 | 37.148 | 2.093 | 1.00 | 19.73 | N |

```
ATOM   3304   CA  VAL C  3    25.368  36.999   1.273  1.00 19.46           C
ATOM   3305   C   VAL C  3    24.258  37.874   1.868  1.00 19.11           C
ATOM   3306   O   VAL C  3    23.930  37.767   3.073  1.00 16.35           O
ATOM   3307   CB  VAL C  3    24.863  35.528   1.206  1.00 20.26           C
ATOM   3308   CG1 VAL C  3    23.634  35.401   0.300  1.00 21.23           C
ATOM   3309   CG2 VAL C  3    25.980  34.608   0.727  1.00 20.48           C
ATOM   3310   N   MET C  4    23.707  38.737   1.009  1.00 17.78           N
ATOM   3311   CA  MET C  4    22.639  39.647   1.350  1.00 17.31           C
ATOM   3312   C   MET C  4    21.386  39.120   0.679  1.00 16.76           C
ATOM   3313   O   MET C  4    21.332  38.997  -0.545  1.00 18.27           O
ATOM   3314   CB  MET C  4    22.942  41.062   0.804  1.00 17.51           C
ATOM   3315   CG  MET C  4    24.270  41.718   1.229  1.00 18.05           C
ATOM   3316   SD  MET C  4    24.409  42.051   2.987  1.00 20.04           S
ATOM   3317   CE  MET C  4    23.241  43.393   3.198  1.00 20.37           C
ATOM   3318   N   THR C  5    20.391  38.763   1.475  1.00 17.41           N
ATOM   3319   CA  THR C  5    19.219  38.068   1.005  1.00 18.07           C
ATOM   3320   C   THR C  5    18.000  38.951   1.118  1.00 19.31           C
ATOM   3321   O   THR C  5    17.691  39.465   2.204  1.00 18.52           O
ATOM   3322   CB  THR C  5    18.941  36.757   1.803  1.00 18.75           C
ATOM   3323   OG1 THR C  5    20.110  35.943   1.807  1.00 18.99           O
ATOM   3324   CG2 THR C  5    17.864  35.996   1.171  1.00 18.25           C
ATOM   3325   N   GLN C  6    17.331  39.146  -0.015  1.00 18.88           N
ATOM   3326   CA  GLN C  6    16.025  39.792  -0.065  1.00 17.98           C
ATOM   3327   C   GLN C  6    15.134  39.133  -1.089  1.00 19.33           C
ATOM   3328   O   GLN C  6    15.602  38.464  -2.030  1.00 20.33           O
ATOM   3329   CB  GLN C  6    16.139  41.312  -0.361  1.00 18.74           C
ATOM   3330   CG  GLN C  6    17.020  41.662  -1.475  1.00 15.81           C
ATOM   3331   CD  GLN C  6    17.321  43.175  -1.595  1.00 15.97           C
ATOM   3332   OE1 GLN C  6    18.400  43.528  -2.078  1.00 12.71           O
ATOM   3333   NE2 GLN C  6    16.342  44.043  -1.260  1.00 15.39           N
ATOM   3334   N   ALA C  7    13.833  39.325  -0.914  1.00 18.95           N
ATOM   3335   CA  ALA C  7    12.840  38.813  -1.838  1.00 18.95           C
ATOM   3336   C   ALA C  7    12.864  39.596  -3.122  1.00 20.73           C
ATOM   3337   O   ALA C  7    13.053  40.803  -3.099  1.00 17.64           O
ATOM   3338   CB  ALA C  7    11.453  38.892  -1.214  1.00 19.26           C
ATOM   3339   N   ALA C  8    12.645  38.917  -4.243  1.00 22.03           N
ATOM   3340   CA  ALA C  8    12.553  39.597  -5.534  1.00 23.26           C
ATOM   3341   C   ALA C  8    11.421  40.608  -5.603  1.00 24.42           C
ATOM   3342   O   ALA C  8    11.525  41.608  -6.297  1.00 21.84           O
ATOM   3343   CB  ALA C  8    12.394  38.589  -6.648  1.00 23.89           C
ATOM   3344   N   PHE C  9    10.319  40.315  -4.922  1.00 27.24           N
ATOM   3345   CA  PHE C  9     9.097  41.089  -5.099  1.00 28.80           C
ATOM   3346   C   PHE C  9     8.504  41.475  -3.780  1.00 29.61           C
ATOM   3347   O   PHE C  9     8.467  40.656  -2.863  1.00 29.53           O
ATOM   3348   CB  PHE C  9     8.091  40.277  -5.906  1.00 31.09           C
ATOM   3349   CG  PHE C  9     8.574  39.965  -7.280  1.00 31.78           C
ATOM   3350   CD1 PHE C  9     8.990  38.693  -7.609  1.00 31.52           C
ATOM   3351   CD2 PHE C  9     8.665  40.967  -8.228  1.00 31.97           C
ATOM   3352   CE1 PHE C  9     9.471  38.418  -8.881  1.00 32.36           C
ATOM   3353   CE2 PHE C  9     9.142  40.692  -9.490  1.00 33.23           C
ATOM   3354   CZ  PHE C  9     9.542  39.411  -9.816  1.00 32.40           C
ATOM   3355   N   SER C 10     8.079  42.741  -3.693  1.00 31.20           N
ATOM   3356   CA  SER C 10     7.465  43.277  -2.498  1.00 31.56           C
ATOM   3357   C   SER C 10     6.019  42.850  -2.534  1.00 34.08           C
ATOM   3358   O   SER C 10     5.480  42.510  -3.599  1.00 34.34           O
ATOM   3359   CB  SER C 10     7.537  44.807  -2.479  1.00 30.58           C
ATOM   3360   OG  SER C 10     6.462  45.384  -3.213  1.00 32.64           O
ATOM   3361   N   ASN C 11     5.377  42.888  -1.372  1.00 36.06           N
ATOM   3362   CA  ASN C 11     3.926  42.853  -1.330  1.00 37.13           C
ATOM   3363   C   ASN C 11     3.379  44.138  -2.028  1.00 37.49           C
ATOM   3364   O   ASN C 11     3.813  45.241  -1.693  1.00 37.51           O
ATOM   3365   CB  ASN C 11     3.482  42.710   0.131  1.00 38.90           C
ATOM   3366   CG  ASN C 11     3.944  41.362   0.756  1.00 40.65           C
ATOM   3367   OD1 ASN C 11     3.653  40.281   0.222  1.00 43.42           O
ATOM   3368   ND2 ASN C 11     4.672  41.434   1.868  1.00 41.53           N
```

| ATOM | 3369 | N   | PRO | C | 12 | 2.498   | 43.995 | -3.052 | 1.00 | 36.74 | N |
|------|------|-----|-----|---|----|---------|--------|--------|------|-------|---|
| ATOM | 3370 | CA  | PRO | C | 12 | 1.944   | 45.157 | -3.790 | 1.00 | 35.41 | C |
| ATOM | 3371 | C   | PRO | C | 12 | 1.258   | 46.188 | -2.887 | 1.00 | 34.54 | C |
| ATOM | 3372 | O   | PRO | C | 12 | 0.472   | 45.828 | -2.008 | 1.00 | 35.15 | O |
| ATOM | 3373 | CB  | PRO | C | 12 | 0.933   | 44.533 | -4.755 | 1.00 | 36.07 | C |
| ATOM | 3374 | CG  | PRO | C | 12 | 1.368   | 43.132 | -4.904 | 1.00 | 36.39 | C |
| ATOM | 3375 | CD  | PRO | C | 12 | 2.004   | 42.724 | -3.608 | 1.00 | 37.30 | C |
| ATOM | 3376 | N   | VAL | C | 13 | 1.558   | 47.460 | -3.102 | 1.00 | 31.81 | N |
| ATOM | 3377 | CA  | VAL | C | 13 | 1.214   | 48.474 | -2.133 | 1.00 | 30.49 | C |
| ATOM | 3378 | C   | VAL | C | 13 | 0.329   | 49.541 | -2.752 | 1.00 | 28.75 | C |
| ATOM | 3379 | O   | VAL | C | 13 | 0.626   | 50.097 | -3.804 | 1.00 | 27.72 | O |
| ATOM | 3380 | CB  | VAL | C | 13 | 2.488   | 49.121 | -1.497 | 1.00 | 29.76 | C |
| ATOM | 3381 | CG1 | VAL | C | 13 | 3.299   | 49.864 | -2.512 | 1.00 | 29.70 | C |
| ATOM | 3382 | CG2 | VAL | C | 13 | 2.100   | 50.053 | -0.368 | 1.00 | 31.18 | C |
| ATOM | 3383 | N   | THR | C | 14 | -0.766  | 49.820 | -2.071 | 1.00 | 27.59 | N |
| ATOM | 3384 | CA  | THR | C | 14 | -1.707  | 50.813 | -2.525 | 1.00 | 27.91 | C |
| ATOM | 3385 | C   | THR | C | 14 | -1.065  | 52.171 | -2.359 | 1.00 | 27.67 | C |
| ATOM | 3386 | O   | THR | C | 14 | -0.431  | 52.422 | -1.348 | 1.00 | 26.01 | O |
| ATOM | 3387 | CB  | THR | C | 14 | -3.015  | 50.682 | -1.729 | 1.00 | 27.90 | C |
| ATOM | 3388 | OG1 | THR | C | 14 | -3.589  | 49.394 | -2.004 | 1.00 | 25.79 | O |
| ATOM | 3389 | CG2 | THR | C | 14 | -3.985  | 51.741 | -2.126 | 1.00 | 27.51 | C |
| ATOM | 3390 | N   | LEU | C | 15 | -1.190  | 53.030 | -3.370 | 1.00 | 27.11 | N |
| ATOM | 3391 | CA  | LEU | C | 15 | -0.659  | 54.378 | -3.283 | 1.00 | 27.73 | C |
| ATOM | 3392 | C   | LEU | C | 15 | -1.093  | 55.012 | -1.979 | 1.00 | 28.27 | C |
| ATOM | 3393 | O   | LEU | C | 15 | -2.219  | 54.802 | -1.524 | 1.00 | 29.28 | O |
| ATOM | 3394 | CB  | LEU | C | 15 | -1.143  | 55.237 | -4.463 | 1.00 | 29.63 | C |
| ATOM | 3395 | CG  | LEU | C | 15 | -0.346  | 55.087 | -5.765 | 1.00 | 31.93 | C |
| ATOM | 3396 | CD1 | LEU | C | 15 | -1.214  | 55.387 | -6.993 | 1.00 | 33.47 | C |
| ATOM | 3397 | CD2 | LEU | C | 15 | 0.873   | 55.998 | -5.742 | 1.00 | 32.27 | C |
| ATOM | 3398 | N   | GLY | C | 16 | -0.199  | 55.772 | -1.357 | 1.00 | 27.26 | N |
| ATOM | 3399 | CA  | GLY | C | 16 | -0.513  | 56.452 | -0.103 | 1.00 | 27.20 | C |
| ATOM | 3400 | C   | GLY | C | 16 | -0.433  | 55.598 | 1.154  | 1.00 | 26.30 | C |
| ATOM | 3401 | O   | GLY | C | 16 | -0.611  | 56.117 | 2.246  | 1.00 | 28.16 | O |
| ATOM | 3402 | N   | THR | C | 17 | -0.196  | 54.297 | 1.020  | 1.00 | 25.71 | N |
| ATOM | 3403 | CA  | THR | C | 17 | 0.007   | 53.433 | 2.179  | 1.00 | 24.34 | C |
| ATOM | 3404 | C   | THR | C | 17 | 1.460   | 53.011 | 2.295  | 1.00 | 23.17 | C |
| ATOM | 3405 | O   | THR | C | 17 | 2.267   | 53.355 | 1.462  | 1.00 | 22.11 | O |
| ATOM | 3406 | CB  | THR | C | 17 | -0.878  | 52.194 | 2.097  | 1.00 | 24.82 | C |
| ATOM | 3407 | OG1 | THR | C | 17 | -0.452  | 51.357 | 1.011  | 1.00 | 26.09 | O |
| ATOM | 3408 | CG2 | THR | C | 17 | -2.333  | 52.641 | 1.912  | 1.00 | 25.70 | C |
| ATOM | 3409 | N   | SER | C | 18 | 1.797   | 52.262 | 3.337  | 1.00 | 23.02 | N |
| ATOM | 3410 | CA  | SER | C | 18 | 3.200   | 51.983 | 3.610  | 1.00 | 22.40 | C |
| ATOM | 3411 | C   | SER | C | 18 | 3.671   | 50.672 | 2.984  | 1.00 | 20.69 | C |
| ATOM | 3412 | O   | SER | C | 18 | 2.915   | 49.711 | 2.863  | 1.00 | 18.93 | O |
| ATOM | 3413 | CB  | SER | C | 18 | 3.450   | 51.955 | 5.115  | 1.00 | 24.33 | C |
| ATOM | 3414 | OG  | SER | C | 18 | 4.831   | 51.747 | 5.350  | 1.00 | 28.92 | O |
| ATOM | 3415 | N   | ALA | C | 19 | 4.924   | 50.655 | 2.554  | 1.00 | 18.38 | N |
| ATOM | 3416 | CA  | ALA | C | 19 | 5.570   | 49.408 | 2.141  | 1.00 | 19.37 | C |
| ATOM | 3417 | C   | ALA | C | 19 | 6.715   | 49.057 | 3.067  | 1.00 | 17.21 | C |
| ATOM | 3418 | O   | ALA | C | 19 | 7.270   | 49.909 | 3.739  | 1.00 | 16.38 | O |
| ATOM | 3419 | CB  | ALA | C | 19 | 6.071   | 49.518 | 0.727  | 1.00 | 19.43 | C |
| ATOM | 3420 | N   | SER | C | 20 | 7.083   | 47.777 | 3.063  | 1.00 | 17.59 | N |
| ATOM | 3421 | CA  | SER | C | 20 | 8.076   | 47.250 | 3.987  | 1.00 | 18.47 | C |
| ATOM | 3422 | C   | SER | C | 20 | 8.889   | 46.239 | 3.215  | 1.00 | 18.28 | C |
| ATOM | 3423 | O   | SER | C | 20 | 8.305   | 45.284 | 2.662  | 1.00 | 18.53 | O |
| ATOM | 3424 | CB  | SER | C | 20 | 7.387   | 46.563 | 5.178  | 1.00 | 19.22 | C |
| ATOM | 3425 | OG  | SER | C | 20 | 8.304   | 46.310 | 6.229  | 1.00 | 19.39 | O |
| ATOM | 3426 | N   | ILE | C | 21 | 10.199  | 46.469 | 3.143  | 1.00 | 17.20 | N |
| ATOM | 3427 | CA  | ILE | C | 21 | 11.138  | 45.652 | 2.383  | 1.00 | 17.72 | C |
| ATOM | 3428 | C   | ILE | C | 21 | 12.194  | 45.111 | 3.333  | 1.00 | 17.49 | C |
| ATOM | 3429 | O   | ILE | C | 21 | 12.846  | 45.874 | 4.063  | 1.00 | 17.44 | O |
| ATOM | 3430 | CB  | ILE | C | 21 | 11.746  | 46.484 | 1.211  | 1.00 | 16.27 | C |
| ATOM | 3431 | CG1 | ILE | C | 21 | 10.643  | 46.810 | 0.197  | 1.00 | 17.07 | C |
| ATOM | 3432 | CG2 | ILE | C | 21 | 12.913  | 45.745 | 0.516  | 1.00 | 17.45 | C |
| ATOM | 3433 | CD1 | ILE | C | 21 | 11.060  | 47.700 | -0.909 | 1.00 | 20.02 | C |

```
ATOM   3434  N   SER C  22    12.364  43.781   3.328  1.00  18.39        N
ATOM   3435  CA  SER C  22    13.232  43.085   4.248  1.00  19.05        C
ATOM   3436  C   SER C  22    14.557  42.682   3.607  1.00  19.67        C
ATOM   3437  O   SER C  22    14.617  42.448   2.405  1.00  18.49        O
ATOM   3438  CB  SER C  22    12.536  41.792   4.717  1.00  20.76        C
ATOM   3439  OG  SER C  22    11.447  42.125   5.559  1.00  24.60        O
ATOM   3440  N   CYS C  23    15.610  42.635   4.412  1.00  19.66        N
ATOM   3441  CA  CYS C  23    16.904  42.114   3.968  1.00  20.50        C
ATOM   3442  C   CYS C  23    17.585  41.428   5.150  1.00  21.55        C
ATOM   3443  O   CYS C  23    17.407  41.840   6.310  1.00  20.70        O
ATOM   3444  CB  CYS C  23    17.780  43.234   3.406  1.00  22.22        C
ATOM   3445  SG  CYS C  23    19.507  42.751   2.954  1.00  22.03        S
ATOM   3446  N   ARG C  24    18.306  40.342   4.872  1.00  20.27        N
ATOM   3447  CA  ARG C  24    19.126  39.721   5.887  1.00  21.76        C
ATOM   3448  C   ARG C  24    20.553  39.588   5.404  1.00  20.57        C
ATOM   3449  O   ARG C  24    20.799  39.518   4.204  1.00  18.99        O
ATOM   3450  CB  ARG C  24    18.515  38.402   6.374  1.00  24.06        C
ATOM   3451  CG  ARG C  24    18.092  37.378   5.315  1.00  26.14        C
ATOM   3452  CD  ARG C  24    17.205  36.274   5.935  1.00  28.49        C
ATOM   3453  NE  ARG C  24    17.182  35.018   5.162  1.00  30.03        N
ATOM   3454  CZ  ARG C  24    16.213  34.641   4.324  1.00  33.52        C
ATOM   3455  NH1 ARG C  24    15.142  35.406   4.117  1.00  35.81        N
ATOM   3456  NH2 ARG C  24    16.305  33.472   3.678  1.00  35.36        N
ATOM   3457  N   SER C  25    21.491  39.626   6.356  1.00  20.46        N
ATOM   3458  CA  SER C  25    22.908  39.400   6.110  1.00  21.11        C
ATOM   3459  C   SER C  25    23.409  38.099   6.724  1.00  21.59        C
ATOM   3460  O   SER C  25    22.973  37.686   7.809  1.00  20.36        O
ATOM   3461  CB  SER C  25    23.706  40.519   6.738  1.00  21.87        C
ATOM   3462  OG  SER C  25    25.006  40.552   6.181  1.00  26.39        O
ATOM   3463  N   SER C  26    24.401  37.507   6.071  1.00  22.58        N
ATOM   3464  CA  SER C  26    24.939  36.196   6.488  1.00  22.34        C
ATOM   3465  C   SER C  26    25.967  36.340   7.621  1.00  22.61        C
ATOM   3466  O   SER C  26    26.323  35.367   8.288  1.00  22.08        O
ATOM   3467  CB  SER C  26    25.539  35.471   5.284  1.00  21.81        C
ATOM   3468  OG  SER C  26    26.663  36.185   4.815  1.00  22.66        O
ATOM   3469  N   LYS C  27    26.463  37.552   7.809  1.00  22.28        N
ATOM   3470  CA  LYS C  27    27.302  37.916   8.927  1.00  23.34        C
ATOM   3471  C   LYS C  27    26.714  39.207   9.442  1.00  21.85        C
ATOM   3472  O   LYS C  27    26.052  39.913   8.684  1.00  22.17        O
ATOM   3473  CB  LYS C  27    28.726  38.208   8.470  1.00  24.41        C
ATOM   3474  CG  LYS C  27    29.423  37.045   7.824  1.00  26.14        C
ATOM   3475  CD  LYS C  27    30.840  37.446   7.324  1.00  26.25        C
ATOM   3476  CE  LYS C  27    31.733  36.192   7.150  1.00  27.80        C
ATOM   3477  NZ  LYS C  27    32.507  36.185   5.870  1.00  29.79        N
ATOM   3478  N   SER C  27A   26.972  39.515  10.712  1.00  21.04        N
ATOM   3479  CA  SER C  27A   26.536  40.780  11.315  1.00  20.58        C
ATOM   3480  C   SER C  27A   27.278  41.954  10.708  1.00  20.47        C
ATOM   3481  O   SER C  27A   28.506  41.864  10.497  1.00  21.61        O
ATOM   3482  CB  SER C  27A   26.773  40.755  12.842  1.00  20.30        C
ATOM   3483  OG  SER C  27A   26.568  42.021  13.419  1.00  20.21        O
ATOM   3484  N   LEU C  27B   26.547  43.044  10.434  1.00  18.35        N
ATOM   3485  CA  LEU C  27B   27.134  44.307   9.959  1.00  19.52        C
ATOM   3486  C   LEU C  27B   27.310  45.364  11.063  1.00  18.23        C
ATOM   3487  O   LEU C  27B   27.720  46.511  10.804  1.00  18.99        O
ATOM   3488  CB  LEU C  27B   26.299  44.870   8.805  1.00  20.06        C
ATOM   3489  CG  LEU C  27B   25.894  43.856   7.725  1.00  20.15        C
ATOM   3490  CD1 LEU C  27B   25.093  44.582   6.628  1.00  18.88        C
ATOM   3491  CD2 LEU C  27B   27.107  43.141   7.136  1.00  20.42        C
ATOM   3492  N   LEU C  27C   26.983  44.993  12.297  1.00  19.58        N
ATOM   3493  CA  LEU C  27C   27.192  45.867  13.427  1.00  20.58        C
ATOM   3494  C   LEU C  27C   28.684  45.917  13.755  1.00  20.77        C
ATOM   3495  O   LEU C  27C   29.298  44.920  14.042  1.00  20.76        O
ATOM   3496  CB  LEU C  27C   26.365  45.395  14.637  1.00  21.27        C
ATOM   3497  CG  LEU C  27C   26.637  46.143  15.932  1.00  21.35        C
ATOM   3498  CD1 LEU C  27C   26.480  47.648  15.762  1.00  23.36        C
```

| ATOM | 3499 | CD2 | LEU | C | 27C | 25.696 | 45.600 | 16.998 | 1.00 | 22.36 | C |
|------|------|-----|-----|---|-----|--------|--------|--------|------|-------|---|
| ATOM | 3500 | N | HIS | C | 27D | 29.265 | 47.096 | 13.715 | 1.00 | 22.54 | N |
| ATOM | 3501 | CA | HIS | C | 27D | 30.708 | 47.234 | 13.919 | 1.00 | 24.23 | C |
| ATOM | 3502 | C | HIS | C | 27D | 31.003 | 47.676 | 15.359 | 1.00 | 25.10 | C |
| ATOM | 3503 | O | HIS | C | 27D | 30.119 | 48.164 | 16.071 | 1.00 | 24.90 | O |
| ATOM | 3504 | CB | HIS | C | 27D | 31.221 | 48.237 | 12.904 | 1.00 | 24.53 | C |
| ATOM | 3505 | CG | HIS | C | 27D | 32.709 | 48.362 | 12.832 | 1.00 | 25.36 | C |
| ATOM | 3506 | ND1 | HIS | C | 27D | 33.437 | 47.928 | 11.741 | 1.00 | 26.91 | N |
| ATOM | 3507 | CD2 | HIS | C | 27D | 33.598 | 48.965 | 13.659 | 1.00 | 26.04 | C |
| ATOM | 3508 | CE1 | HIS | C | 27D | 34.715 | 48.228 | 11.927 | 1.00 | 26.60 | C |
| ATOM | 3509 | NE2 | HIS | C | 27D | 34.840 | 48.844 | 13.086 | 1.00 | 27.58 | N |
| ATOM | 3510 | N | SER | C | 27E | 32.249 | 47.473 | 15.793 | 1.00 | 26.73 | N |
| ATOM | 3511 | CA | SER | C | 27E | 32.685 | 47.789 | 17.158 | 1.00 | 26.09 | C |
| ATOM | 3512 | C | SER | C | 27E | 32.497 | 49.274 | 17.467 | 1.00 | 26.88 | C |
| ATOM | 3513 | O | SER | C | 27E | 32.298 | 49.665 | 18.620 | 1.00 | 27.12 | O |
| ATOM | 3514 | CB | SER | C | 27E | 34.150 | 47.378 | 17.340 | 1.00 | 27.72 | C |
| ATOM | 3515 | OG | SER | C | 27E | 34.993 | 48.172 | 16.518 | 1.00 | 30.38 | O |
| ATOM | 3516 | N | ASP | C | 28 | 32.518 | 50.084 | 16.420 | 1.00 | 24.73 | N |
| ATOM | 3517 | CA | ASP | C | 28 | 32.280 | 51.510 | 16.523 | 1.00 | 24.05 | C |
| ATOM | 3518 | C | ASP | C | 28 | 30.801 | 51.822 | 16.780 | 1.00 | 22.75 | C |
| ATOM | 3519 | O | ASP | C | 28 | 30.430 | 52.973 | 16.917 | 1.00 | 22.97 | O |
| ATOM | 3520 | CB | ASP | C | 28 | 32.785 | 52.250 | 15.268 | 1.00 | 24.59 | C |
| ATOM | 3521 | CG | ASP | C | 28 | 32.114 | 51.788 | 13.947 | 1.00 | 24.05 | C |
| ATOM | 3522 | OD1 | ASP | C | 28 | 31.052 | 51.128 | 13.956 | 1.00 | 24.16 | O |
| ATOM | 3523 | OD2 | ASP | C | 28 | 32.681 | 52.098 | 12.878 | 1.00 | 25.21 | O |
| ATOM | 3524 | N | GLY | C | 29 | 29.967 | 50.794 | 16.803 | 1.00 | 21.59 | N |
| ATOM | 3525 | CA | GLY | C | 29 | 28.568 | 50.942 | 17.145 | 1.00 | 21.74 | C |
| ATOM | 3526 | C | GLY | C | 29 | 27.693 | 51.012 | 15.924 | 1.00 | 21.70 | C |
| ATOM | 3527 | O | GLY | C | 29 | 26.487 | 50.781 | 16.037 | 1.00 | 23.85 | O |
| ATOM | 3528 | N | ILE | C | 30 | 28.302 | 51.305 | 14.767 | 1.00 | 20.45 | N |
| ATOM | 3529 | CA | ILE | C | 30 | 27.556 | 51.584 | 13.525 | 1.00 | 19.69 | C |
| ATOM | 3530 | C | ILE | C | 30 | 27.219 | 50.293 | 12.804 | 1.00 | 18.21 | C |
| ATOM | 3531 | O | ILE | C | 30 | 28.053 | 49.413 | 12.730 | 1.00 | 20.34 | O |
| ATOM | 3532 | CB | ILE | C | 30 | 28.350 | 52.475 | 12.587 | 1.00 | 18.67 | C |
| ATOM | 3533 | CG1 | ILE | C | 30 | 28.475 | 53.879 | 13.196 | 1.00 | 20.95 | C |
| ATOM | 3534 | CG2 | ILE | C | 30 | 27.695 | 52.517 | 11.221 | 1.00 | 19.25 | C |
| ATOM | 3535 | CD1 | ILE | C | 30 | 29.595 | 54.677 | 12.617 | 1.00 | 21.12 | C |
| ATOM | 3536 | N | THR | C | 31 | 25.991 | 50.186 | 12.289 | 1.00 | 16.17 | N |
| ATOM | 3537 | CA | THR | C | 31 | 25.563 | 49.030 | 11.474 | 1.00 | 16.05 | C |
| ATOM | 3538 | C | THR | C | 31 | 25.711 | 49.491 | 10.025 | 1.00 | 15.87 | C |
| ATOM | 3539 | O | THR | C | 31 | 25.010 | 50.403 | 9.561 | 1.00 | 15.86 | O |
| ATOM | 3540 | CB | THR | C | 31 | 24.117 | 48.525 | 11.799 | 1.00 | 16.94 | C |
| ATOM | 3541 | OG1 | THR | C | 31 | 24.011 | 48.163 | 13.173 | 1.00 | 15.65 | O |
| ATOM | 3542 | CG2 | THR | C | 31 | 23.722 | 47.275 | 10.990 | 1.00 | 15.63 | C |
| ATOM | 3543 | N | TYR | C | 32 | 26.655 | 48.876 | 9.316 | 1.00 | 14.30 | N |
| ATOM | 3544 | CA | TYR | C | 32 | 27.025 | 49.304 | 7.975 | 1.00 | 14.70 | C |
| ATOM | 3545 | C | TYR | C | 32 | 26.155 | 48.646 | 6.914 | 1.00 | 16.02 | C |
| ATOM | 3546 | O | TYR | C | 32 | 26.624 | 47.873 | 6.090 | 1.00 | 15.52 | O |
| ATOM | 3547 | CB | TYR | C | 32 | 28.505 | 49.056 | 7.739 | 1.00 | 16.91 | C |
| ATOM | 3548 | CG | TYR | C | 32 | 29.410 | 49.970 | 8.515 | 1.00 | 17.86 | C |
| ATOM | 3549 | CD1 | TYR | C | 32 | 29.891 | 49.611 | 9.767 | 1.00 | 19.55 | C |
| ATOM | 3550 | CD2 | TYR | C | 32 | 29.784 | 51.214 | 7.990 | 1.00 | 17.53 | C |
| ATOM | 3551 | CE1 | TYR | C | 32 | 30.713 | 50.476 | 10.485 | 1.00 | 19.58 | C |
| ATOM | 3552 | CE2 | TYR | C | 32 | 30.608 | 52.058 | 8.683 | 1.00 | 18.69 | C |
| ATOM | 3553 | CZ | TYR | C | 32 | 31.070 | 51.677 | 9.942 | 1.00 | 18.29 | C |
| ATOM | 3554 | OH | TYR | C | 32 | 31.886 | 52.499 | 10.649 | 1.00 | 18.83 | O |
| ATOM | 3555 | N | LEU | C | 33 | 24.871 | 48.985 | 6.979 | 1.00 | 14.71 | N |
| ATOM | 3556 | CA | LEU | C | 33 | 23.842 | 48.548 | 6.063 | 1.00 | 13.03 | C |
| ATOM | 3557 | C | LEU | C | 33 | 23.455 | 49.762 | 5.191 | 1.00 | 13.95 | C |
| ATOM | 3558 | O | LEU | C | 33 | 23.285 | 50.872 | 5.700 | 1.00 | 12.53 | O |
| ATOM | 3559 | CB | LEU | C | 33 | 22.662 | 48.001 | 6.863 | 1.00 | 14.26 | C |
| ATOM | 3560 | CG | LEU | C | 33 | 21.461 | 47.365 | 6.143 | 1.00 | 14.16 | C |
| ATOM | 3561 | CD1 | LEU | C | 33 | 20.555 | 48.376 | 5.546 | 1.00 | 16.15 | C |
| ATOM | 3562 | CD2 | LEU | C | 33 | 21.915 | 46.334 | 5.066 | 1.00 | 14.96 | C |
| ATOM | 3563 | N | TYR | C | 34 | 23.351 | 49.561 | 3.878 | 1.00 | 13.28 | N |

| ATOM | 3564 | CA | TYR C | 34 | 22.967 | 50.654 | 2.981 | 1.00 13.66 | C |
|------|------|-----|-------|-----|--------|--------|--------|-------------|---|
| ATOM | 3565 | C | TYR C | 34 | 21.801 | 50.214 | 2.152 | 1.00 14.78 | C |
| ATOM | 3566 | O | TYR C | 34 | 21.678 | 49.035 | 1.846 | 1.00 11.24 | O |
| ATOM | 3567 | CB | TYR C | 34 | 24.131 | 50.956 | 2.066 | 1.00 13.62 | C |
| ATOM | 3568 | CG | TYR C | 34 | 25.314 | 51.494 | 2.792 | 1.00 14.08 | C |
| ATOM | 3569 | CD1 | TYR C | 34 | 26.012 | 50.723 | 3.721 | 1.00 13.50 | C |
| ATOM | 3570 | CD2 | TYR C | 34 | 25.752 | 52.787 | 2.549 | 1.00 13.25 | C |
| ATOM | 3571 | CE1 | TYR C | 34 | 27.114 | 51.228 | 4.402 | 1.00 12.78 | C |
| ATOM | 3572 | CE2 | TYR C | 34 | 26.847 | 53.302 | 3.234 | 1.00 14.51 | C |
| ATOM | 3573 | CZ | TYR C | 34 | 27.515 | 52.517 | 4.167 | 1.00 14.08 | C |
| ATOM | 3574 | OH | TYR C | 34 | 28.618 | 53.017 | 4.804 | 1.00 14.67 | O |
| ATOM | 3575 | N | TRP C | 35 | 20.945 | 51.133 | 1.763 | 1.00 12.07 | N |
| ATOM | 3576 | CA | TRP C | 35 | 19.898 | 50.813 | 0.751 | 1.00 13.45 | C |
| ATOM | 3577 | C | TRP C | 35 | 20.073 | 51.688 | -0.487 | 1.00 14.31 | C |
| ATOM | 3578 | O | TRP C | 35 | 20.289 | 52.900 | -0.374 | 1.00 14.61 | O |
| ATOM | 3579 | CB | TRP C | 35 | 18.499 | 51.032 | 1.271 | 1.00 15.22 | C |
| ATOM | 3580 | CG | TRP C | 35 | 18.034 | 50.156 | 2.354 | 1.00 14.21 | C |
| ATOM | 3581 | CD1 | TRP C | 35 | 18.063 | 50.438 | 3.688 | 1.00 14.74 | C |
| ATOM | 3582 | CD2 | TRP C | 35 | 17.467 | 48.838 | 2.232 | 1.00 16.21 | C |
| ATOM | 3583 | NE1 | TRP C | 35 | 17.530 | 49.423 | 4.394 | 1.00 13.15 | N |
| ATOM | 3584 | CE2 | TRP C | 35 | 17.160 | 48.414 | 3.530 | 1.00 14.78 | C |
| ATOM | 3585 | CE3 | TRP C | 35 | 17.125 | 48.014 | 1.152 | 1.00 13.26 | C |
| ATOM | 3586 | CZ2 | TRP C | 35 | 16.559 | 47.189 | 3.785 | 1.00 14.86 | C |
| ATOM | 3587 | CZ3 | TRP C | 35 | 16.554 | 46.788 | 1.412 | 1.00 15.33 | C |
| ATOM | 3588 | CH2 | TRP C | 35 | 16.253 | 46.397 | 2.707 | 1.00 13.50 | C |
| ATOM | 3589 | N | TYR C | 36 | 20.002 | 51.068 | -1.659 | 1.00 12.64 | N |
| ATOM | 3590 | CA | TYR C | 36 | 20.011 | 51.777 | -2.950 | 1.00 13.63 | C |
| ATOM | 3591 | C | TYR C | 36 | 18.683 | 51.577 | -3.681 | 1.00 14.36 | C |
| ATOM | 3592 | O | TYR C | 36 | 17.990 | 50.544 | -3.529 | 1.00 13.61 | O |
| ATOM | 3593 | CB | TYR C | 36 | 21.174 | 51.369 | -3.872 | 1.00 12.95 | C |
| ATOM | 3594 | CG | TYR C | 36 | 22.518 | 51.572 | -3.252 | 1.00 13.44 | C |
| ATOM | 3595 | CD1 | TYR C | 36 | 23.116 | 50.592 | -2.487 | 1.00 13.17 | C |
| ATOM | 3596 | CD2 | TYR C | 36 | 23.184 | 52.785 | -3.401 | 1.00 14.43 | C |
| ATOM | 3597 | CE1 | TYR C | 36 | 24.340 | 50.809 | -1.878 | 1.00 11.35 | C |
| ATOM | 3598 | CE2 | TYR C | 36 | 24.403 | 53.011 | -2.812 | 1.00 13.09 | C |
| ATOM | 3599 | CZ | TYR C | 36 | 24.981 | 52.029 | -2.038 | 1.00 14.26 | C |
| ATOM | 3600 | OH | TYR C | 36 | 26.185 | 52.299 | -1.453 | 1.00 14.05 | O |
| ATOM | 3601 | N | LEU C | 37 | 18.316 | 52.608 | -4.440 | 1.00 12.41 | N |
| ATOM | 3602 | CA | LEU C | 37 | 17.161 | 52.514 | -5.320 | 1.00 12.77 | C |
| ATOM | 3603 | C | LEU C | 37 | 17.593 | 52.683 | -6.742 | 1.00 12.61 | C |
| ATOM | 3604 | O | LEU C | 37 | 18.229 | 53.671 | -7.095 | 1.00 13.52 | O |
| ATOM | 3605 | CB | LEU C | 37 | 16.087 | 53.531 | -4.944 | 1.00 12.10 | C |
| ATOM | 3606 | CG | LEU C | 37 | 14.899 | 53.673 | -5.873 | 1.00 13.44 | C |
| ATOM | 3607 | CD1 | LEU C | 37 | 14.156 | 52.378 | -6.075 | 1.00 10.97 | C |
| ATOM | 3608 | CD2 | LEU C | 37 | 13.965 | 54.728 | -5.334 | 1.00 14.53 | C |
| ATOM | 3609 | N | GLN C | 38 | 17.220 | 51.713 | -7.562 | 1.00 12.96 | N |
| ATOM | 3610 | CA | GLN C | 38 | 17.360 | 51.810 | -9.007 | 1.00 14.71 | C |
| ATOM | 3611 | C | GLN C | 38 | 16.009 | 51.940 | -9.673 | 1.00 16.02 | C |
| ATOM | 3612 | O | GLN C | 38 | 15.293 | 50.961 | -9.825 | 1.00 14.60 | O |
| ATOM | 3613 | CB | GLN C | 38 | 18.083 | 50.582 | -9.548 | 1.00 13.95 | C |
| ATOM | 3614 | CG | GLN C | 38 | 18.390 | 50.642 | -11.035 | 1.00 13.13 | C |
| ATOM | 3615 | CD | GLN C | 38 | 19.363 | 49.546 | -11.438 | 1.00 14.98 | C |
| ATOM | 3616 | OE1 | GLN C | 38 | 19.321 | 48.446 | -10.889 | 1.00 15.85 | O |
| ATOM | 3617 | NE2 | GLN C | 38 | 20.230 | 49.833 | -12.378 | 1.00 15.05 | N |
| ATOM | 3618 | N | LYS C | 39 | 15.639 | 53.173 | -10.044 | 1.00 16.65 | N |
| ATOM | 3619 | CA | LYS C | 39 | 14.390 | 53.411 | -10.785 | 1.00 20.44 | C |
| ATOM | 3620 | C | LYS C | 39 | 14.507 | 52.957 | -12.224 | 1.00 22.40 | C |
| ATOM | 3621 | O | LYS C | 39 | 15.633 | 52.834 | -12.757 | 1.00 22.86 | O |
| ATOM | 3622 | CB | LYS C | 39 | 14.010 | 54.882 | -10.728 | 1.00 19.94 | C |
| ATOM | 3623 | CG | LYS C | 39 | 13.699 | 55.357 | -9.357 | 1.00 21.45 | C |
| ATOM | 3624 | CD | LYS C | 39 | 13.202 | 56.770 | -9.393 | 1.00 21.76 | C |
| ATOM | 3625 | CE | LYS C | 39 | 12.899 | 57.249 | -8.010 | 1.00 24.03 | C |
| ATOM | 3626 | NZ | LYS C | 39 | 12.298 | 58.636 | -8.062 | 1.00 24.30 | N |
| ATOM | 3627 | N | PRO C | 40 | 13.345 | 52.714 | -12.885 | 1.00 26.22 | N |
| ATOM | 3628 | CA | PRO C | 40 | 13.404 | 52.094 | -14.212 | 1.00 26.19 | C |

```
ATOM   3629  C   PRO C  40      14.227  52.938 -15.181  1.00 27.53           C
ATOM   3630  O   PRO C  40      14.061  54.167 -15.230  1.00 27.52           O
ATOM   3631  CB  PRO C  40      11.934  52.052 -14.645  1.00 27.08           C
ATOM   3632  CG  PRO C  40      11.168  52.066 -13.396  1.00 27.88           C
ATOM   3633  CD  PRO C  40      11.945  52.996 -12.500  1.00 26.40           C
ATOM   3634  N   GLY C  41      15.142  52.290 -15.897  1.00 27.86           N
ATOM   3635  CA  GLY C  41      16.001  52.996 -16.846  1.00 27.82           C
ATOM   3636  C   GLY C  41      17.129  53.835 -16.271  1.00 28.61           C
ATOM   3637  O   GLY C  41      17.938  54.345 -17.053  1.00 29.97           O
ATOM   3638  N   GLN C  42      17.210  53.957 -14.929  1.00 26.72           N
ATOM   3639  CA  GLN C  42      18.279  54.702 -14.246  1.00 24.55           C
ATOM   3640  C   GLN C  42      19.351  53.809 -13.591  1.00 22.33           C
ATOM   3641  O   GLN C  42      19.172  52.584 -13.420  1.00 21.92           O
ATOM   3642  CB  GLN C  42      17.691  55.586 -13.139  1.00 25.69           C
ATOM   3643  CG  GLN C  42      16.501  56.500 -13.513  1.00 27.24           C
ATOM   3644  CD  GLN C  42      16.173  57.510 -12.402  1.00 27.42           C
ATOM   3645  OE1 GLN C  42      16.622  57.376 -11.234  1.00 29.47           O
ATOM   3646  NE2 GLN C  42      15.397  58.551 -12.759  1.00 29.22           N
ATOM   3647  N   SER C  43      20.457  54.453 -13.212  1.00 19.04           N
ATOM   3648  CA  SER C  43      21.458  53.876 -12.324  1.00 18.52           C
ATOM   3649  C   SER C  43      20.954  53.756 -10.891  1.00 15.70           C
ATOM   3650  O   SER C  43      20.009  54.464 -10.494  1.00 16.79           O
ATOM   3651  CB  SER C  43      22.703  54.748 -12.293  1.00 18.89           C
ATOM   3652  OG  SER C  43      23.179  54.948 -13.612  1.00 21.97           O
ATOM   3653  N   PRO C  44      21.594  52.889 -10.081  1.00 14.12           N
ATOM   3654  CA  PRO C  44      21.223  52.920  -8.657  1.00 13.12           C
ATOM   3655  C   PRO C  44      21.584  54.265  -8.011  1.00 12.71           C
ATOM   3656  O   PRO C  44      22.523  54.904  -8.458  1.00 10.60           O
ATOM   3657  CB  PRO C  44      22.057  51.779  -8.037  1.00 14.80           C
ATOM   3658  CG  PRO C  44      22.391  50.874  -9.240  1.00 12.61           C
ATOM   3659  CD  PRO C  44      22.553  51.808 -10.397  1.00 15.37           C
ATOM   3660  N   HIS C  45      20.862  54.655  -6.974  1.00 12.11           N
ATOM   3661  CA  HIS C  45      21.322  55.722  -6.129  1.00 13.22           C
ATOM   3662  C   HIS C  45      21.108  55.446  -4.658  1.00 13.55           C
ATOM   3663  O   HIS C  45      20.137  54.800  -4.272  1.00 14.39           O
ATOM   3664  CB  HIS C  45      20.583  57.006  -6.507  1.00 14.02           C
ATOM   3665  CG  HIS C  45      19.124  57.012  -6.163  1.00 15.31           C
ATOM   3666  ND1 HIS C  45      18.142  56.551  -7.025  1.00 17.58           N
ATOM   3667  CD2 HIS C  45      18.472  57.465  -5.062  1.00 18.21           C
ATOM   3668  CE1 HIS C  45      16.959  56.719  -6.466  1.00 18.52           C
ATOM   3669  NE2 HIS C  45      17.131  57.272  -5.276  1.00 18.41           N
ATOM   3670  N   LEU C  46      21.991  55.991  -3.829  1.00 14.13           N
ATOM   3671  CA  LEU C  46      21.883  55.827  -2.379  1.00 14.26           C
ATOM   3672  C   LEU C  46      20.624  56.438  -1.801  1.00 16.08           C
ATOM   3673  O   LEU C  46      20.307  57.637  -2.073  1.00 15.74           O
ATOM   3674  CB  LEU C  46      23.086  56.475  -1.691  1.00 14.03           C
ATOM   3675  CG  LEU C  46      23.263  56.237  -0.209  1.00 10.28           C
ATOM   3676  CD1 LEU C  46      23.453  54.781   0.120  1.00 10.40           C
ATOM   3677  CD2 LEU C  46      24.481  56.973   0.270  1.00 13.84           C
ATOM   3678  N   LEU C  47      19.926  55.628  -0.998  1.00 16.84           N
ATOM   3679  CA  LEU C  47      18.775  56.047  -0.203  1.00 17.49           C
ATOM   3680  C   LEU C  47      19.174  56.195   1.264  1.00 17.34           C
ATOM   3681  O   LEU C  47      19.012  57.247   1.852  1.00 19.32           O
ATOM   3682  CB  LEU C  47      17.608  55.036  -0.282  1.00 18.61           C
ATOM   3683  CG  LEU C  47      16.574  55.137  -1.394  1.00 21.70           C
ATOM   3684  CD1 LEU C  47      15.516  54.084  -1.134  1.00 22.40           C
ATOM   3685  CD2 LEU C  47      15.900  56.512  -1.520  1.00 22.58           C
ATOM   3686  N   ILE C  48      19.680  55.112   1.854  1.00 16.73           N
ATOM   3687  CA  ILE C  48      19.948  55.043   3.275  1.00 16.74           C
ATOM   3688  C   ILE C  48      21.365  54.520   3.458  1.00 15.62           C
ATOM   3689  O   ILE C  48      21.774  53.515   2.798  1.00 14.01           O
ATOM   3690  CB  ILE C  48      18.954  54.058   4.004  1.00 17.88           C
ATOM   3691  CG1 ILE C  48      17.480  54.460   3.822  1.00 20.21           C
ATOM   3692  CG2 ILE C  48      19.316  53.875   5.487  1.00 19.42           C
ATOM   3693  CD1 ILE C  48      17.024  55.538   4.733  1.00 20.03           C
```

| ATOM | 3694 | N | TYR | C | 49 | 22.086 | 55.146 | 4.379 | 1.00 | 15.13 | N |
|------|------|------|-----|---|----|--------|--------|-------|------|-------|---|
| ATOM | 3695 | CA | TYR | C | 49 | 23.418 | 54.678 | 4.774 | 1.00 | 16.29 | C |
| ATOM | 3696 | C | TYR | C | 49 | 23.517 | 54.488 | 6.277 | 1.00 | 16.98 | C |
| ATOM | 3697 | O | TYR | C | 49 | 22.762 | 55.103 | 7.025 | 1.00 | 16.90 | O |
| ATOM | 3698 | CB | TYR | C | 49 | 24.476 | 55.648 | 4.276 | 1.00 | 18.67 | C |
| ATOM | 3699 | CG | TYR | C | 49 | 24.471 | 57.028 | 4.898 | 1.00 | 18.84 | C |
| ATOM | 3700 | CD1 | TYR | C | 49 | 23.734 | 58.049 | 4.351 | 1.00 | 19.63 | C |
| ATOM | 3701 | CD2 | TYR | C | 49 | 25.275 | 57.320 | 6.001 | 1.00 | 20.80 | C |
| ATOM | 3702 | CE1 | TYR | C | 49 | 23.747 | 59.315 | 4.913 | 1.00 | 19.83 | C |
| ATOM | 3703 | CE2 | TYR | C | 49 | 25.276 | 58.590 | 6.567 | 1.00 | 21.11 | C |
| ATOM | 3704 | CZ | TYR | C | 49 | 24.521 | 59.572 | 6.001 | 1.00 | 20.63 | C |
| ATOM | 3705 | OH | TYR | C | 49 | 24.538 | 60.847 | 6.518 | 1.00 | 22.21 | O |
| ATOM | 3706 | N | HIS | C | 50 | 24.419 | 53.599 | 6.706 | 1.00 | 16.85 | N |
| ATOM | 3707 | CA | HIS | C | 50 | 24.635 | 53.320 | 8.110 | 1.00 | 18.18 | C |
| ATOM | 3708 | C | HIS | C | 50 | 23.350 | 52.900 | 8.818 | 1.00 | 18.12 | C |
| ATOM | 3709 | O | HIS | C | 50 | 23.102 | 53.256 | 9.974 | 1.00 | 19.95 | O |
| ATOM | 3710 | CB | HIS | C | 50 | 25.284 | 54.514 | 8.800 | 1.00 | 18.23 | C |
| ATOM | 3711 | CG | HIS | C | 50 | 26.713 | 54.762 | 8.392 | 1.00 | 21.17 | C |
| ATOM | 3712 | ND1 | HIS | C | 50 | 27.402 | 55.884 | 8.791 | 1.00 | 21.23 | N |
| ATOM | 3713 | CD2 | HIS | C | 50 | 27.586 | 54.026 | 7.656 | 1.00 | 22.33 | C |
| ATOM | 3714 | CE1 | HIS | C | 50 | 28.633 | 55.837 | 8.307 | 1.00 | 22.39 | C |
| ATOM | 3715 | NE2 | HIS | C | 50 | 28.772 | 54.724 | 7.613 | 1.00 | 21.45 | N |
| ATOM | 3716 | N | LEU | C | 51 | 22.547 | 52.124 | 8.100 | 1.00 | 17.02 | N |
| ATOM | 3717 | CA | LEU | C | 51 | 21.314 | 51.504 | 8.586 | 1.00 | 17.04 | C |
| ATOM | 3718 | C | LEU | C | 51 | 20.147 | 52.483 | 8.646 | 1.00 | 16.05 | C |
| ATOM | 3719 | O | LEU | C | 51 | 19.058 | 52.122 | 8.243 | 1.00 | 15.64 | O |
| ATOM | 3720 | CB | LEU | C | 51 | 21.480 | 50.779 | 9.934 | 1.00 | 15.76 | C |
| ATOM | 3721 | CG | LEU | C | 51 | 20.259 | 50.027 | 10.452 | 1.00 | 15.97 | C |
| ATOM | 3722 | CD1 | LEU | C | 51 | 19.871 | 48.822 | 9.577 | 1.00 | 14.48 | C |
| ATOM | 3723 | CD2 | LEU | C | 51 | 20.507 | 49.551 | 11.873 | 1.00 | 15.30 | C |
| ATOM | 3724 | N | SER | C | 52 | 20.355 | 53.712 | 9.114 | 1.00 | 16.87 | N |
| ATOM | 3725 | CA | SER | C | 52 | 19.185 | 54.555 | 9.369 | 1.00 | 17.07 | C |
| ATOM | 3726 | C | SER | C | 52 | 19.319 | 56.020 | 8.983 | 1.00 | 17.47 | C |
| ATOM | 3727 | O | SER | C | 52 | 18.371 | 56.784 | 9.209 | 1.00 | 16.38 | O |
| ATOM | 3728 | CB | SER | C | 52 | 18.782 | 54.430 | 10.841 | 1.00 | 16.82 | C |
| ATOM | 3729 | OG | SER | C | 52 | 18.529 | 53.065 | 11.215 | 1.00 | 17.93 | O |
| ATOM | 3730 | N | ASN | C | 53 | 20.423 | 56.401 | 8.356 | 1.00 | 17.69 | N |
| ATOM | 3731 | CA | ASN | C | 53 | 20.606 | 57.797 | 7.947 | 1.00 | 20.13 | C |
| ATOM | 3732 | C | ASN | C | 53 | 20.157 | 57.964 | 6.514 | 1.00 | 21.14 | C |
| ATOM | 3733 | O | ASN | C | 53 | 20.583 | 57.220 | 5.638 | 1.00 | 22.09 | O |
| ATOM | 3734 | CB | ASN | C | 53 | 22.064 | 58.213 | 8.080 | 1.00 | 20.50 | C |
| ATOM | 3735 | CG | ASN | C | 53 | 22.578 | 58.078 | 9.494 | 1.00 | 24.19 | C |
| ATOM | 3736 | OD1 | ASN | C | 53 | 21.872 | 58.377 | 10.449 | 1.00 | 28.94 | O |
| ATOM | 3737 | ND2 | ASN | C | 53 | 23.812 | 57.641 | 9.641 | 1.00 | 28.81 | N |
| ATOM | 3738 | N | LEU | C | 54 | 19.303 | 58.946 | 6.260 | 1.00 | 21.79 | N |
| ATOM | 3739 | CA | LEU | C | 54 | 18.900 | 59.279 | 4.894 | 1.00 | 22.98 | C |
| ATOM | 3740 | C | LEU | C | 54 | 19.969 | 60.075 | 4.127 | 1.00 | 23.06 | C |
| ATOM | 3741 | O | LEU | C | 54 | 20.541 | 61.054 | 4.640 | 1.00 | 20.28 | O |
| ATOM | 3742 | CB | LEU | C | 54 | 17.593 | 60.098 | 4.893 | 1.00 | 23.91 | C |
| ATOM | 3743 | CG | LEU | C | 54 | 16.249 | 59.361 | 5.035 | 1.00 | 25.15 | C |
| ATOM | 3744 | CD1 | LEU | C | 54 | 16.199 | 58.582 | 6.336 | 1.00 | 26.18 | C |
| ATOM | 3745 | CD2 | LEU | C | 54 | 15.062 | 60.323 | 4.968 | 1.00 | 25.13 | C |
| ATOM | 3746 | N | ALA | C | 55 | 20.209 | 59.672 | 2.877 | 1.00 | 22.77 | N |
| ATOM | 3747 | CA | ALA | C | 55 | 21.068 | 60.440 | 1.988 | 1.00 | 22.74 | C |
| ATOM | 3748 | C | ALA | C | 55 | 20.443 | 61.813 | 1.728 | 1.00 | 23.30 | C |
| ATOM | 3749 | O | ALA | C | 55 | 19.238 | 61.978 | 1.839 | 1.00 | 21.57 | O |
| ATOM | 3750 | CB | ALA | C | 55 | 21.243 | 59.722 | 0.669 | 1.00 | 22.41 | C |
| ATOM | 3751 | N | SER | C | 56 | 21.247 | 62.779 | 1.302 | 1.00 | 24.41 | N |
| ATOM | 3752 | CA | SER | C | 56 | 20.688 | 64.097 | 1.005 | 1.00 | 25.16 | C |
| ATOM | 3753 | C | SER | C | 56 | 19.602 | 63.982 | -0.063 | 1.00 | 24.42 | C |
| ATOM | 3754 | O | SER | C | 56 | 19.759 | 63.279 | -1.045 | 1.00 | 24.64 | O |
| ATOM | 3755 | CB | SER | C | 56 | 21.770 | 65.072 | 0.543 | 1.00 | 26.35 | C |
| ATOM | 3756 | OG | SER | C | 56 | 21.187 | 66.358 | 0.377 | 1.00 | 30.92 | O |
| ATOM | 3757 | N | GLY | C | 57 | 18.483 | 64.662 | 0.160 | 1.00 | 25.73 | N |
| ATOM | 3758 | CA | GLY | C | 57 | 17.420 | 64.752 | -0.828 | 1.00 | 24.68 | C |

| ATOM | 3759 | C | GLY | C | 57 | 16.402 | 63.641 | -0.738 | 1.00 | 23.92 | C |
|------|------|-----|-----|---|----|--------|--------|--------|------|-------|---|
| ATOM | 3760 | O | GLY | C | 57 | 15.420 | 63.650 | -1.451 | 1.00 | 24.12 | O |
| ATOM | 3761 | N | VAL | C | 58 | 16.633 | 62.675 | 0.136 | 1.00 | 22.97 | N |
| ATOM | 3762 | CA | VAL | C | 58 | 15.705 | 61.585 | 0.282 | 1.00 | 22.83 | C |
| ATOM | 3763 | C | VAL | C | 58 | 14.569 | 62.028 | 1.216 | 1.00 | 22.55 | C |
| ATOM | 3764 | O | VAL | C | 58 | 14.825 | 62.559 | 2.269 | 1.00 | 22.47 | O |
| ATOM | 3765 | CB | VAL | C | 58 | 16.428 | 60.318 | 0.807 | 1.00 | 20.84 | C |
| ATOM | 3766 | CG1 | VAL | C | 58 | 15.445 | 59.157 | 1.066 | 1.00 | 20.99 | C |
| ATOM | 3767 | CG2 | VAL | C | 58 | 17.497 | 59.897 | -0.197 | 1.00 | 20.40 | C |
| ATOM | 3768 | N | PRO | C | 59 | 13.310 | 61.816 | 0.806 | 1.00 | 24.22 | N |
| ATOM | 3769 | CA | PRO | C | 59 | 12.149 | 62.200 | 1.602 | 1.00 | 25.17 | C |
| ATOM | 3770 | C | PRO | C | 59 | 12.082 | 61.426 | 2.923 | 1.00 | 25.42 | C |
| ATOM | 3771 | O | PRO | C | 59 | 12.531 | 60.291 | 3.003 | 1.00 | 23.75 | O |
| ATOM | 3772 | CB | PRO | C | 59 | 10.959 | 61.849 | 0.699 | 1.00 | 25.85 | C |
| ATOM | 3773 | CG | PRO | C | 59 | 11.503 | 61.654 | -0.629 | 1.00 | 26.10 | C |
| ATOM | 3774 | CD | PRO | C | 59 | 12.922 | 61.233 | -0.483 | 1.00 | 25.11 | C |
| ATOM | 3775 | N | ASP | C | 60 | 11.536 | 62.056 | 3.956 | 1.00 | 25.86 | N |
| ATOM | 3776 | CA | ASP | C | 60 | 11.470 | 61.434 | 5.268 | 1.00 | 25.67 | C |
| ATOM | 3777 | C | ASP | C | 60 | 10.380 | 60.365 | 5.363 | 1.00 | 25.27 | C |
| ATOM | 3778 | O | ASP | C | 60 | 10.212 | 59.745 | 6.405 | 1.00 | 27.26 | O |
| ATOM | 3779 | CB | ASP | C | 60 | 11.342 | 62.496 | 6.368 | 1.00 | 28.58 | C |
| ATOM | 3780 | CG | ASP | C | 60 | 9.987 | 63.182 | 6.393 | 1.00 | 30.47 | C |
| ATOM | 3781 | OD1 | ASP | C | 60 | 9.179 | 62.974 | 5.455 | 1.00 | 33.54 | O |
| ATOM | 3782 | OD2 | ASP | C | 60 | 9.756 | 63.961 | 7.358 | 1.00 | 35.85 | O |
| ATOM | 3783 | N | ARG | C | 61 | 9.651 | 60.131 | 4.268 | 1.00 | 24.05 | N |
| ATOM | 3784 | CA | ARG | C | 61 | 8.827 | 58.925 | 4.135 | 1.00 | 23.04 | C |
| ATOM | 3785 | C | ARG | C | 61 | 9.634 | 57.631 | 4.209 | 1.00 | 20.85 | C |
| ATOM | 3786 | O | ARG | C | 61 | 9.068 | 56.572 | 4.526 | 1.00 | 21.00 | O |
| ATOM | 3787 | CB | ARG | C | 61 | 8.078 | 58.936 | 2.804 | 1.00 | 24.05 | C |
| ATOM | 3788 | CG | ARG | C | 61 | 6.969 | 59.916 | 2.815 | 1.00 | 26.05 | C |
| ATOM | 3789 | CD | ARG | C | 61 | 6.310 | 60.122 | 1.461 | 1.00 | 26.64 | C |
| ATOM | 3790 | NE | ARG | C | 61 | 7.215 | 60.271 | 0.305 | 1.00 | 29.46 | N |
| ATOM | 3791 | CZ | ARG | C | 61 | 7.577 | 59.296 | -0.536 | 1.00 | 26.85 | C |
| ATOM | 3792 | NH1 | ARG | C | 61 | 7.149 | 58.054 | -0.362 | 1.00 | 28.33 | N |
| ATOM | 3793 | NH2 | ARG | C | 61 | 8.359 | 59.579 | -1.577 | 1.00 | 27.00 | N |
| ATOM | 3794 | N | PHE | C | 62 | 10.921 | 57.716 | 3.871 | 1.00 | 19.34 | N |
| ATOM | 3795 | CA | PHE | C | 62 | 11.837 | 56.548 | 3.901 | 1.00 | 17.72 | C |
| ATOM | 3796 | C | PHE | C | 62 | 12.469 | 56.394 | 5.275 | 1.00 | 16.87 | C |
| ATOM | 3797 | O | PHE | C | 62 | 12.871 | 57.365 | 5.889 | 1.00 | 16.14 | O |
| ATOM | 3798 | CB | PHE | C | 62 | 12.904 | 56.666 | 2.803 | 1.00 | 15.95 | C |
| ATOM | 3799 | CG | PHE | C | 62 | 12.350 | 56.510 | 1.420 | 1.00 | 15.70 | C |
| ATOM | 3800 | CD1 | PHE | C | 62 | 11.880 | 57.596 | 0.715 | 1.00 | 15.81 | C |
| ATOM | 3801 | CD2 | PHE | C | 62 | 12.200 | 55.247 | 0.869 | 1.00 | 14.38 | C |
| ATOM | 3802 | CE1 | PHE | C | 62 | 11.343 | 57.447 | -0.549 | 1.00 | 15.30 | C |
| ATOM | 3803 | CE2 | PHE | C | 62 | 11.665 | 55.094 | -0.395 | 1.00 | 14.99 | C |
| ATOM | 3804 | CZ | PHE | C | 62 | 11.207 | 56.182 | -1.093 | 1.00 | 15.03 | C |
| ATOM | 3805 | N | SER | C | 63 | 12.535 | 55.171 | 5.792 | 1.00 | 12.98 | N |
| ATOM | 3806 | CA | SER | C | 63 | 13.201 | 54.961 | 7.039 | 1.00 | 13.01 | C |
| ATOM | 3807 | C | SER | C | 63 | 13.649 | 53.538 | 7.068 | 1.00 | 12.93 | C |
| ATOM | 3808 | O | SER | C | 63 | 13.155 | 52.746 | 6.297 | 1.00 | 10.13 | O |
| ATOM | 3809 | CB | SER | C | 63 | 12.324 | 55.262 | 8.261 | 1.00 | 13.97 | C |
| ATOM | 3810 | OG | SER | C | 63 | 11.146 | 54.511 | 8.296 | 1.00 | 13.72 | O |
| ATOM | 3811 | N | SER | C | 64 | 14.581 | 53.259 | 7.964 | 1.00 | 13.73 | N |
| ATOM | 3812 | CA | SER | C | 64 | 15.181 | 51.933 | 8.039 | 1.00 | 13.80 | C |
| ATOM | 3813 | C | SER | C | 64 | 15.685 | 51.616 | 9.432 | 1.00 | 13.34 | C |
| ATOM | 3814 | O | SER | C | 64 | 16.187 | 52.466 | 10.143 | 1.00 | 13.14 | O |
| ATOM | 3815 | CB | SER | C | 64 | 16.291 | 51.841 | 7.012 | 1.00 | 14.55 | C |
| ATOM | 3816 | OG | SER | C | 64 | 17.007 | 50.621 | 7.155 | 1.00 | 16.70 | O |
| ATOM | 3817 | N | SER | C | 65 | 15.544 | 50.344 | 9.815 | 1.00 | 13.33 | N |
| ATOM | 3818 | CA | SER | C | 65 | 15.947 | 49.898 | 11.104 | 1.00 | 11.97 | C |
| ATOM | 3819 | C | SER | C | 65 | 16.450 | 48.468 | 10.931 | 1.00 | 11.77 | C |
| ATOM | 3820 | O | SER | C | 65 | 16.160 | 47.810 | 9.917 | 1.00 | 12.63 | O |
| ATOM | 3821 | CB | SER | C | 65 | 14.770 | 49.923 | 12.069 | 1.00 | 13.68 | C |
| ATOM | 3822 | OG | SER | C | 65 | 13.717 | 49.082 | 11.650 | 1.00 | 15.93 | O |
| ATOM | 3823 | N | GLY | C | 66 | 17.173 | 48.002 | 11.918 | 1.00 | 11.69 | N |

```
ATOM   3824  CA  GLY C  66      17.618  46.590  11.913  1.00 12.03           C
ATOM   3825  C   GLY C  66      18.661  46.212  12.973  1.00 11.13           C
ATOM   3826  O   GLY C  66      19.229  47.042  13.677  1.00 13.55           O
ATOM   3827  N   SER C  67      18.961  44.919  13.035  1.00 14.72           N
ATOM   3828  CA  SER C  67      19.997  44.456  13.894  1.00 14.76           C
ATOM   3829  C   SER C  67      21.295  44.519  13.095  1.00 16.88           C
ATOM   3830  O   SER C  67      21.425  45.287  12.139  1.00 17.14           O
ATOM   3831  CB  SER C  67      19.694  43.038  14.380  1.00 15.60           C
ATOM   3832  OG  SER C  67      19.505  42.166  13.299  1.00 14.29           O
ATOM   3833  N   GLY C  68      22.292  43.738  13.496  1.00 15.77           N
ATOM   3834  CA  GLY C  68      23.412  43.523  12.616  1.00 15.79           C
ATOM   3835  C   GLY C  68      23.118  42.587  11.445  1.00 16.99           C
ATOM   3836  O   GLY C  68      23.911  42.536  10.512  1.00 16.80           O
ATOM   3837  N   THR C  69      22.044  41.787  11.516  1.00 16.84           N
ATOM   3838  CA  THR C  69      21.746  40.805  10.487  1.00 17.13           C
ATOM   3839  C   THR C  69      20.375  40.871   9.816  1.00 15.92           C
ATOM   3840  O   THR C  69      20.184  40.242   8.796  1.00 15.51           O
ATOM   3841  CB  THR C  69      21.899  39.364  11.033  1.00 18.30           C
ATOM   3842  OG1 THR C  69      20.934  39.129  12.062  1.00 18.90           O
ATOM   3843  CG2 THR C  69      23.296  39.174  11.589  1.00 20.07           C
ATOM   3844  N   ASP C  70      19.411  41.558  10.401  1.00 15.48           N
ATOM   3845  CA  ASP C  70      18.064  41.594   9.849  1.00 14.89           C
ATOM   3846  C   ASP C  70      17.638  43.064   9.765  1.00 14.31           C
ATOM   3847  O   ASP C  70      17.728  43.814  10.745  1.00 13.29           O
ATOM   3848  CB  ASP C  70      17.098  40.791  10.722  1.00 16.46           C
ATOM   3849  CG  ASP C  70      17.378  39.302  10.679  1.00 19.84           C
ATOM   3850  OD1 ASP C  70      17.055  38.692   9.637  1.00 19.59           O
ATOM   3851  OD2 ASP C  70      17.885  38.760  11.695  1.00 21.58           O
ATOM   3852  N   PHE C  71      17.177  43.475   8.588  1.00 13.70           N
ATOM   3853  CA  PHE C  71      16.942  44.920   8.321  1.00 13.72           C
ATOM   3854  C   PHE C  71      15.607  45.113   7.651  1.00 13.51           C
ATOM   3855  O   PHE C  71      15.125  44.209   6.956  1.00 14.57           O
ATOM   3856  CB  PHE C  71      18.035  45.480   7.414  1.00 14.58           C
ATOM   3857  CG  PHE C  71      19.364  44.834   7.607  1.00 15.47           C
ATOM   3858  CD1 PHE C  71      20.116  45.125   8.723  1.00 14.81           C
ATOM   3859  CD2 PHE C  71      19.838  43.889   6.699  1.00 15.04           C
ATOM   3860  CE1 PHE C  71      21.341  44.509   8.932  1.00 15.76           C
ATOM   3861  CE2 PHE C  71      21.081  43.272   6.896  1.00 15.08           C
ATOM   3862  CZ  PHE C  71      21.832  43.583   8.008  1.00 13.53           C
ATOM   3863  N   THR C  72      15.010  46.295   7.844  1.00 12.70           N
ATOM   3864  CA  THR C  72      13.682  46.597   7.321  1.00 12.31           C
ATOM   3865  C   THR C  72      13.642  48.037   6.812  1.00 13.21           C
ATOM   3866  O   THR C  72      13.817  48.963   7.586  1.00 11.19           O
ATOM   3867  CB  THR C  72      12.575  46.424   8.392  1.00 14.70           C
ATOM   3868  OG1 THR C  72      12.569  45.062   8.885  1.00 12.93           O
ATOM   3869  CG2 THR C  72      11.235  46.696   7.764  1.00 13.72           C
ATOM   3870  N   LEU C  73      13.458  48.189   5.517  1.00 13.06           N
ATOM   3871  CA  LEU C  73      13.168  49.479   4.894  1.00 13.02           C
ATOM   3872  C   LEU C  73      11.665  49.765   4.900  1.00 13.87           C
ATOM   3873  O   LEU C  73      10.858  48.958   4.427  1.00 13.69           O
ATOM   3874  CB  LEU C  73      13.691  49.443   3.452  1.00 13.15           C
ATOM   3875  CG  LEU C  73      13.496  50.689   2.613  1.00 14.37           C
ATOM   3876  CD1 LEU C  73      14.233  51.847   3.252  1.00 14.68           C
ATOM   3877  CD2 LEU C  73      14.005  50.420   1.209  1.00 14.37           C
ATOM   3878  N   ARG C  74      11.250  50.931   5.420  1.00 14.35           N
ATOM   3879  CA  ARG C  74       9.810  51.282   5.384  1.00 15.78           C
ATOM   3880  C   ARG C  74       9.648  52.543   4.567  1.00 16.96           C
ATOM   3881  O   ARG C  74      10.459  53.462   4.698  1.00 15.58           O
ATOM   3882  CB  ARG C  74       9.215  51.521   6.778  1.00 16.60           C
ATOM   3883  CG  ARG C  74       7.670  51.692   6.782  1.00 17.23           C
ATOM   3884  CD  ARG C  74       7.033  51.469   8.164  1.00 20.98           C
ATOM   3885  NE  ARG C  74       5.550  51.616   8.208  1.00 22.60           N
ATOM   3886  CZ  ARG C  74       4.658  50.628   8.122  1.00 23.16           C
ATOM   3887  NH1 ARG C  74       5.035  49.358   7.984  1.00 24.36           N
ATOM   3888  NH2 ARG C  74       3.345  50.913   8.202  1.00 25.25           N
```

```
ATOM   3889  N    ILE C  75      8.638  52.538   3.700  1.00 17.44           N
ATOM   3890  CA   ILE C  75      8.211  53.738   2.971  1.00 19.37           C
ATOM   3891  C    ILE C  75      6.794  54.010   3.482  1.00 20.54           C
ATOM   3892  O    ILE C  75      5.921  53.158   3.337  1.00 19.85           O
ATOM   3893  CB   ILE C  75      8.248  53.482   1.453  1.00 19.49           C
ATOM   3894  CG1  ILE C  75      9.520  52.701   1.078  1.00 19.93           C
ATOM   3895  CG2  ILE C  75      8.160  54.825   0.684  1.00 19.61           C
ATOM   3896  CD1  ILE C  75      9.484  51.976  -0.291  1.00 19.25           C
ATOM   3897  N    SER C  76      6.553  55.159   4.113  1.00 22.75           N
ATOM   3898  CA   SER C  76      5.289  55.341   4.856  1.00 24.71           C
ATOM   3899  C    SER C  76      4.030  55.596   4.004  1.00 27.64           C
ATOM   3900  O    SER C  76      2.949  55.044   4.295  1.00 30.05           O
ATOM   3901  CB   SER C  76      5.431  56.448   5.887  1.00 24.57           C
ATOM   3902  OG   SER C  76      5.828  57.649   5.263  1.00 24.80           O
ATOM   3903  N    ARG C  77      4.151  56.482   3.017  1.00 27.42           N
ATOM   3904  CA   ARG C  77      3.037  56.873   2.157  1.00 27.56           C
ATOM   3905  C    ARG C  77      3.573  56.799   0.734  1.00 25.94           C
ATOM   3906  O    ARG C  77      3.977  57.803   0.139  1.00 26.31           O
ATOM   3907  CB   ARG C  77      2.583  58.318   2.450  1.00 28.73           C
ATOM   3908  CG   ARG C  77      1.847  58.526   3.779  1.00 31.68           C
ATOM   3909  CD   ARG C  77      1.813  60.011   4.161  1.00 33.96           C
ATOM   3910  NE   ARG C  77      3.166  60.547   4.374  1.00 36.65           N
ATOM   3911  CZ   ARG C  77      3.898  60.370   5.481  1.00 38.69           C
ATOM   3912  NH1  ARG C  77      3.424  59.689   6.529  1.00 40.80           N
ATOM   3913  NH2  ARG C  77      5.115  60.899   5.558  1.00 39.48           N
ATOM   3914  N    VAL C  78      3.572  55.592   0.203  1.00 25.51           N
ATOM   3915  CA   VAL C  78      4.114  55.332  -1.116  1.00 24.31           C
ATOM   3916  C    VAL C  78      3.522  56.229  -2.242  1.00 23.38           C
ATOM   3917  O    VAL C  78      2.294  56.393  -2.379  1.00 20.72           O
ATOM   3918  CB   VAL C  78      3.960  53.849  -1.443  1.00 23.09           C
ATOM   3919  CG1  VAL C  78      4.414  53.556  -2.824  1.00 24.47           C
ATOM   3920  CG2  VAL C  78      4.787  53.039  -0.451  1.00 22.39           C
ATOM   3921  N    GLU C  79      4.439  56.781  -3.038  1.00 23.88           N
ATOM   3922  CA   GLU C  79      4.135  57.594  -4.206  1.00 24.28           C
ATOM   3923  C    GLU C  79      4.506  56.816  -5.474  1.00 25.00           C
ATOM   3924  O    GLU C  79      5.317  55.865  -5.444  1.00 23.98           O
ATOM   3925  CB   GLU C  79      4.884  58.926  -4.134  1.00 24.76           C
ATOM   3926  CG   GLU C  79      4.653  59.684  -2.836  1.00 27.05           C
ATOM   3927  CD   GLU C  79      5.193  61.111  -2.862  1.00 27.12           C
ATOM   3928  OE1  GLU C  79      5.842  61.495  -3.857  1.00 34.32           O
ATOM   3929  OE2  GLU C  79      5.026  61.842  -1.865  1.00 30.84           O
ATOM   3930  N    ALA C  80      3.868  57.188  -6.580  1.00 24.28           N
ATOM   3931  CA   ALA C  80      4.046  56.501  -7.854  1.00 23.21           C
ATOM   3932  C    ALA C  80      5.514  56.425  -8.226  1.00 21.59           C
ATOM   3933  O    ALA C  80      5.966  55.377  -8.673  1.00 20.31           O
ATOM   3934  CB   ALA C  80      3.256  57.220  -8.962  1.00 23.23           C
ATOM   3935  N    GLU C  81      6.235  57.532  -8.025  1.00 20.70           N
ATOM   3936  CA   GLU C  81      7.671  57.640  -8.327  1.00 22.98           C
ATOM   3937  C    GLU C  81      8.596  56.727  -7.516  1.00 21.29           C
ATOM   3938  O    GLU C  81      9.763  56.633  -7.818  1.00 21.12           O
ATOM   3939  CB   GLU C  81      8.172  59.067  -8.121  1.00 24.34           C
ATOM   3940  CG   GLU C  81      8.264  59.501  -6.648  1.00 28.37           C
ATOM   3941  CD   GLU C  81      9.466  60.392  -6.338  1.00 30.09           C
ATOM   3942  OE1  GLU C  81      9.347  61.239  -5.415  1.00 35.80           O
ATOM   3943  OE2  GLU C  81     10.537  60.237  -6.988  1.00 36.63           O
ATOM   3944  N    ASP C  82      8.091  56.110  -6.458  1.00 19.84           N
ATOM   3945  CA   ASP C  82      8.908  55.215  -5.637  1.00 18.24           C
ATOM   3946  C    ASP C  82      9.107  53.844  -6.260  1.00 18.62           C
ATOM   3947  O    ASP C  82      9.909  53.062  -5.725  1.00 17.15           O
ATOM   3948  CB   ASP C  82      8.276  55.053  -4.255  1.00 15.88           C
ATOM   3949  CG   ASP C  82      8.148  56.358  -3.524  1.00 18.34           C
ATOM   3950  OD1  ASP C  82      8.981  57.238  -3.796  1.00 16.81           O
ATOM   3951  OD2  ASP C  82      7.235  56.493  -2.681  1.00 17.30           O
ATOM   3952  N    VAL C  83      8.394  53.535  -7.356  1.00 17.76           N
ATOM   3953  CA   VAL C  83      8.635  52.257  -8.084  1.00 17.15           C
```

```
ATOM   3954  C    VAL C  83    10.061  52.091  -8.529  1.00  14.99       C
ATOM   3955  O    VAL C  83    10.715  53.023  -9.029  1.00  15.60       O
ATOM   3956  CB   VAL C  83     7.757  52.026  -9.345  1.00  18.92       C
ATOM   3957  CG1  VAL C  83     6.421  51.628  -8.949  1.00  22.95       C
ATOM   3958  CG2  VAL C  83     7.727  53.257 -10.268  1.00  20.04       C
ATOM   3959  N    GLY C  84    10.546  50.869  -8.379  1.00  15.61       N
ATOM   3960  CA   GLY C  84    11.921  50.537  -8.689  1.00  15.26       C
ATOM   3961  C    GLY C  84    12.325  49.285  -7.912  1.00  14.20       C
ATOM   3962  O    GLY C  84    11.501  48.636  -7.266  1.00  16.14       O
ATOM   3963  N    ILE C  85    13.605  48.983  -7.996  1.00  13.94       N
ATOM   3964  CA   ILE C  85    14.235  47.915  -7.275  1.00  15.41       C
ATOM   3965  C    ILE C  85    15.163  48.510  -6.194  1.00  14.23       C
ATOM   3966  O    ILE C  85    16.048  49.339  -6.467  1.00  15.12       O
ATOM   3967  CB   ILE C  85    14.998  47.012  -8.237  1.00  15.71       C
ATOM   3968  CG1  ILE C  85    14.072  46.559  -9.370  1.00  19.92       C
ATOM   3969  CG2  ILE C  85    15.560  45.804  -7.513  1.00  15.65       C
ATOM   3970  CD1  ILE C  85    14.771  45.682 -10.420  1.00  19.67       C
ATOM   3971  N    TYR C  86    14.900  48.073  -4.977  1.00  13.65       N
ATOM   3972  CA   TYR C  86    15.583  48.458  -3.762  1.00  12.87       C
ATOM   3973  C    TYR C  86    16.580  47.365  -3.447  1.00  13.73       C
ATOM   3974  O    TYR C  86    16.192  46.202  -3.303  1.00  13.84       O
ATOM   3975  CB   TYR C  86    14.560  48.604  -2.627  1.00  12.29       C
ATOM   3976  CG   TYR C  86    13.640  49.799  -2.839  1.00  12.03       C
ATOM   3977  CD1  TYR C  86    12.541  49.707  -3.694  1.00  10.98       C
ATOM   3978  CD2  TYR C  86    13.887  51.028  -2.224  1.00  11.85       C
ATOM   3979  CE1  TYR C  86    11.702  50.744  -3.926  1.00  10.72       C
ATOM   3980  CE2  TYR C  86    13.049  52.089  -2.469  1.00  12.47       C
ATOM   3981  CZ   TYR C  86    11.963  51.948  -3.317  1.00  11.29       C
ATOM   3982  OH   TYR C  86    11.108  52.995  -3.585  1.00  14.41       O
ATOM   3983  N    TYR C  87    17.841  47.768  -3.375  1.00  11.39       N
ATOM   3984  CA   TYR C  87    18.953  46.889  -3.058  1.00  14.10       C
ATOM   3985  C    TYR C  87    19.542  47.218  -1.685  1.00  13.27       C
ATOM   3986  O    TYR C  87    19.756  48.395  -1.334  1.00  12.07       O
ATOM   3987  CB   TYR C  87    20.052  47.045  -4.092  1.00  14.36       C
ATOM   3988  CG   TYR C  87    19.703  46.694  -5.493  1.00  15.26       C
ATOM   3989  CD1  TYR C  87    19.892  45.399  -5.959  1.00  13.82       C
ATOM   3990  CD2  TYR C  87    19.249  47.650  -6.391  1.00  16.48       C
ATOM   3991  CE1  TYR C  87    19.612  45.058  -7.258  1.00  14.86       C
ATOM   3992  CE2  TYR C  87    18.967  47.315  -7.701  1.00  14.80       C
ATOM   3993  CZ   TYR C  87    19.143  46.004  -8.124  1.00  16.58       C
ATOM   3994  OH   TYR C  87    18.865  45.670  -9.429  1.00  17.56       O
ATOM   3995  N    CYS C  88    19.826  46.157  -0.933  1.00  12.12       N
ATOM   3996  CA   CYS C  88    20.606  46.247   0.292  1.00  13.55       C
ATOM   3997  C    CYS C  88    22.061  45.886  -0.039  1.00  13.62       C
ATOM   3998  O    CYS C  88    22.338  45.123  -0.972  1.00  13.17       O
ATOM   3999  CB   CYS C  88    20.052  45.342   1.399  1.00  11.64       C
ATOM   4000  SG   CYS C  88    19.903  43.551   1.061  1.00  18.09       S
ATOM   4001  N    ALA C  89    22.977  46.500   0.704  1.00  14.05       N
ATOM   4002  CA   ALA C  89    24.399  46.296   0.567  1.00  13.94       C
ATOM   4003  C    ALA C  89    25.033  46.470   1.946  1.00  15.57       C
ATOM   4004  O    ALA C  89    24.432  47.076   2.826  1.00  16.61       O
ATOM   4005  CB   ALA C  89    25.009  47.272  -0.457  1.00  14.19       C
ATOM   4006  N    HIS C  90    26.204  45.863   2.142  1.00  15.70       N
ATOM   4007  CA   HIS C  90    27.001  46.063   3.353  1.00  15.85       C
ATOM   4008  C    HIS C  90    28.238  46.877   3.041  1.00  16.13       C
ATOM   4009  O    HIS C  90    28.659  47.037   1.865  1.00  15.61       O
ATOM   4010  CB   HIS C  90    27.444  44.724   3.962  1.00  16.22       C
ATOM   4011  CG   HIS C  90    28.561  44.071   3.209  1.00  17.14       C
ATOM   4012  ND1  HIS C  90    28.343  43.155   2.205  1.00  20.39       N
ATOM   4013  CD2  HIS C  90    29.903  44.216   3.300  1.00  17.85       C
ATOM   4014  CE1  HIS C  90    29.504  42.760   1.717  1.00  16.81       C
ATOM   4015  NE2  HIS C  90    30.464  43.387   2.365  1.00  19.03       N
ATOM   4016  N    ASN C  91    28.846  47.346   4.110  1.00  15.44       N
ATOM   4017  CA   ASN C  91    30.130  48.029   4.058  1.00  17.19       C
ATOM   4018  C    ASN C  91    30.978  47.642   5.272  1.00  17.10       C
```

| ATOM | 4019 | O | ASN C | 91 | 31.452 | 48.482 | 6.003 | 1.00 | 18.80 | O |
|------|------|-----|-------|----|--------|--------|--------|------|-------|---|
| ATOM | 4020 | CB | ASN C | 91 | 29.919 | 49.527 | 3.956 | 1.00 | 15.57 | C |
| ATOM | 4021 | CG | ASN C | 91 | 31.219 | 50.323 | 3.899 | 1.00 | 17.81 | C |
| ATOM | 4022 | OD1 | ASN C | 91 | 31.234 | 51.514 | 4.238 | 1.00 | 19.14 | O |
| ATOM | 4023 | ND2 | ASN C | 91 | 32.295 | 49.697 | 3.448 | 1.00 | 13.38 | N |
| ATOM | 4024 | N | VAL C | 92 | 31.206 | 46.346 | 5.453 | 1.00 | 16.68 | N |
| ATOM | 4025 | CA | VAL C | 92 | 32.119 | 45.889 | 6.510 | 1.00 | 19.50 | C |
| ATOM | 4026 | C | VAL C | 92 | 33.368 | 45.128 | 6.041 | 1.00 | 20.28 | C |
| ATOM | 4027 | O | VAL C | 92 | 34.241 | 44.821 | 6.850 | 1.00 | 22.78 | O |
| ATOM | 4028 | CB | VAL C | 92 | 31.398 | 45.023 | 7.548 | 1.00 | 19.85 | C |
| ATOM | 4029 | CG1 | VAL C | 92 | 30.296 | 45.796 | 8.188 | 1.00 | 20.40 | C |
| ATOM | 4030 | CG2 | VAL C | 92 | 30.854 | 43.737 | 6.936 | 1.00 | 19.33 | C |
| ATOM | 4031 | N | GLU C | 93 | 33.457 | 44.823 | 4.750 | 1.00 | 20.60 | N |
| ATOM | 4032 | CA | GLU C | 93 | 34.568 | 44.082 | 4.224 | 1.00 | 19.37 | C |
| ATOM | 4033 | C | GLU C | 93 | 34.546 | 44.129 | 2.708 | 1.00 | 19.29 | C |
| ATOM | 4034 | O | GLU C | 93 | 33.523 | 44.440 | 2.093 | 1.00 | 18.18 | O |
| ATOM | 4035 | CB | GLU C | 93 | 34.471 | 42.600 | 4.676 | 1.00 | 19.12 | C |
| ATOM | 4036 | CG | GLU C | 93 | 33.388 | 41.791 | 4.045 | 1.00 | 21.69 | C |
| ATOM | 4037 | CD | GLU C | 93 | 33.507 | 40.313 | 4.392 | 1.00 | 21.89 | C |
| ATOM | 4038 | OE1 | GLU C | 93 | 33.940 | 40.030 | 5.524 | 1.00 | 25.75 | O |
| ATOM | 4039 | OE2 | GLU C | 93 | 33.160 | 39.431 | 3.572 | 1.00 | 27.71 | O |
| ATOM | 4040 | N | LEU C | 94 | 35.652 | 43.731 | 2.106 | 1.00 | 19.95 | N |
| ATOM | 4041 | CA | LEU C | 94 | 35.630 | 43.312 | 0.699 | 1.00 | 20.55 | C |
| ATOM | 4042 | C | LEU C | 94 | 35.523 | 41.767 | 0.647 | 1.00 | 21.03 | C |
| ATOM | 4043 | O | LEU C | 94 | 36.070 | 41.088 | 1.500 | 1.00 | 24.29 | O |
| ATOM | 4044 | CB | LEU C | 94 | 36.897 | 43.767 | -0.007 | 1.00 | 20.53 | C |
| ATOM | 4045 | CG | LEU C | 94 | 37.212 | 45.253 | 0.081 | 1.00 | 19.70 | C |
| ATOM | 4046 | CD1 | LEU C | 94 | 38.555 | 45.495 | -0.580 | 1.00 | 21.09 | C |
| ATOM | 4047 | CD2 | LEU C | 94 | 36.125 | 46.112 | -0.576 | 1.00 | 18.00 | C |
| ATOM | 4048 | N | PRO C | 95 | 34.844 | 41.202 | -0.352 | 1.00 | 20.98 | N |
| ATOM | 4049 | CA | PRO C | 95 | 34.210 | 41.841 | -1.492 | 1.00 | 19.38 | C |
| ATOM | 4050 | C | PRO C | 95 | 32.967 | 42.603 | -1.072 | 1.00 | 17.31 | C |
| ATOM | 4051 | O | PRO C | 95 | 32.269 | 42.205 | -0.129 | 1.00 | 15.37 | O |
| ATOM | 4052 | CB | PRO C | 95 | 33.817 | 40.671 | -2.387 | 1.00 | 21.15 | C |
| ATOM | 4053 | CG | PRO C | 95 | 33.759 | 39.486 | -1.488 | 1.00 | 21.36 | C |
| ATOM | 4054 | CD | PRO C | 95 | 34.647 | 39.740 | -0.349 | 1.00 | 20.93 | C |
| ATOM | 4055 | N | ARG C | 96 | 32.698 | 43.712 | -1.766 | 1.00 | 15.59 | N |
| ATOM | 4056 | CA | ARG C | 96 | 31.458 | 44.409 | -1.549 | 1.00 | 15.66 | C |
| ATOM | 4057 | C | ARG C | 96 | 30.377 | 43.582 | -2.206 | 1.00 | 16.19 | C |
| ATOM | 4058 | O | ARG C | 96 | 30.527 | 43.145 | -3.348 | 1.00 | 18.47 | O |
| ATOM | 4059 | CB | ARG C | 96 | 31.511 | 45.827 | -2.131 | 1.00 | 15.24 | C |
| ATOM | 4060 | CG | ARG C | 96 | 32.777 | 46.556 | -1.783 | 1.00 | 14.98 | C |
| ATOM | 4061 | CD | ARG C | 96 | 32.585 | 48.083 | -1.936 | 1.00 | 14.81 | C |
| ATOM | 4062 | NE | ARG C | 96 | 33.714 | 48.845 | -1.460 | 1.00 | 15.67 | N |
| ATOM | 4063 | CZ | ARG C | 96 | 33.886 | 49.235 | -0.196 | 1.00 | 16.94 | C |
| ATOM | 4064 | NH1 | ARG C | 96 | 33.017 | 48.901 | 0.747 | 1.00 | 17.18 | N |
| ATOM | 4065 | NH2 | ARG C | 96 | 34.956 | 49.946 | 0.133 | 1.00 | 15.10 | N |
| ATOM | 4066 | N | THR C | 97 | 29.298 | 43.321 | -1.477 | 1.00 | 15.82 | N |
| ATOM | 4067 | CA | THR C | 97 | 28.220 | 42.548 | -2.014 | 1.00 | 15.71 | C |
| ATOM | 4068 | C | THR C | 97 | 26.871 | 43.218 | -1.767 | 1.00 | 15.67 | C |
| ATOM | 4069 | O | THR C | 97 | 26.702 | 43.995 | -0.827 | 1.00 | 18.06 | O |
| ATOM | 4070 | CB | THR C | 97 | 28.272 | 41.061 | -1.521 | 1.00 | 16.54 | C |
| ATOM | 4071 | OG1 | THR C | 97 | 27.966 | 40.978 | -0.127 | 1.00 | 16.58 | O |
| ATOM | 4072 | CG2 | THR C | 97 | 29.630 | 40.506 | -1.757 | 1.00 | 16.59 | C |
| ATOM | 4073 | N | PHE C | 98 | 25.938 | 42.894 | -2.654 | 1.00 | 14.92 | N |
| ATOM | 4074 | CA | PHE C | 98 | 24.609 | 43.469 | -2.753 | 1.00 | 14.63 | C |
| ATOM | 4075 | C | PHE C | 98 | 23.549 | 42.370 | -2.681 | 1.00 | 15.33 | C |
| ATOM | 4076 | O | PHE C | 98 | 23.798 | 41.247 | -3.114 | 1.00 | 15.09 | O |
| ATOM | 4077 | CB | PHE C | 98 | 24.456 | 44.163 | -4.112 | 1.00 | 14.75 | C |
| ATOM | 4078 | CG | PHE C | 98 | 25.408 | 45.271 | -4.322 | 1.00 | 11.35 | C |
| ATOM | 4079 | CD1 | PHE C | 98 | 26.650 | 45.047 | -4.912 | 1.00 | 13.90 | C |
| ATOM | 4080 | CD2 | PHE C | 98 | 25.072 | 46.570 | -3.940 | 1.00 | 13.06 | C |
| ATOM | 4081 | CE1 | PHE C | 98 | 27.543 | 46.058 | -5.101 | 1.00 | 13.31 | C |
| ATOM | 4082 | CE2 | PHE C | 98 | 25.990 | 47.586 | -4.126 | 1.00 | 13.27 | C |
| ATOM | 4083 | CZ | PHE C | 98 | 27.205 | 47.339 | -4.711 | 1.00 | 14.76 | C |

```
ATOM   4084   N    GLY  C   99     22.376   42.669    -2.130   1.00  14.30        N
ATOM   4085   CA   GLY  C   99     21.223   41.810    -2.342   1.00  15.77        C
ATOM   4086   C    GLY  C   99     20.733   41.751    -3.781   1.00  15.78        C
ATOM   4087   O    GLY  C   99     21.168   42.510    -4.641   1.00  17.06        O
ATOM   4088   N    GLY  C  100     19.822   40.831    -4.057   1.00  16.57        N
ATOM   4089   CA   GLY  C  100     19.304   40.640    -5.419   1.00  16.46        C
ATOM   4090   C    GLY  C  100     18.302   41.682    -5.886   1.00  15.25        C
ATOM   4091   O    GLY  C  100     17.931   41.711    -7.065   1.00  14.47        O
ATOM   4092   N    GLY  C  101     17.843   42.507    -4.943   1.00  14.65        N
ATOM   4093   CA   GLY  C  101     16.954   43.644    -5.206   1.00  16.10        C
ATOM   4094   C    GLY  C  101     15.518   43.249    -4.972   1.00  16.45        C
ATOM   4095   O    GLY  C  101     15.143   42.096    -5.270   1.00  19.44        O
ATOM   4096   N    THR  C  102     14.730   44.142    -4.397   1.00  16.10        N
ATOM   4097   CA   THR  C  102     13.297   43.906    -4.225   1.00  17.05        C
ATOM   4098   C    THR  C  102     12.557   44.934    -5.037   1.00  17.31        C
ATOM   4099   O    THR  C  102     12.782   46.148    -4.883   1.00  16.00        O
ATOM   4100   CB   THR  C  102     12.844   44.001    -2.734   1.00  17.34        C
ATOM   4101   OG1  THR  C  102     13.513   42.991    -1.974   1.00  16.57        O
ATOM   4102   CG2  THR  C  102     11.344   43.832    -2.577   1.00  16.92        C
ATOM   4103   N    LYS  C  103     11.668   44.453    -5.901   1.00  19.50        N
ATOM   4104   CA   LYS  C  103     10.865   45.346    -6.720   1.00  20.10        C
ATOM   4105   C    LYS  C  103      9.620   45.780    -5.990   1.00  20.30        C
ATOM   4106   O    LYS  C  103      8.821   44.957    -5.552   1.00  20.92        O
ATOM   4107   CB   LYS  C  103     10.489   44.705    -8.060   1.00  21.25        C
ATOM   4108   CG   LYS  C  103      9.792   45.678    -9.017   1.00  22.73        C
ATOM   4109   CD   LYS  C  103      9.283   44.977   -10.261   1.00  24.08        C
ATOM   4110   CE   LYS  C  103     10.357   44.830   -11.307   1.00  27.02        C
ATOM   4111   NZ   LYS  C  103     10.079   43.619   -12.183   1.00  29.36        N
ATOM   4112   N    LEU  C  104      9.466   47.098    -5.844   1.00  18.10        N
ATOM   4113   CA   LEU  C  104      8.282   47.648    -5.265   1.00  19.88        C
ATOM   4114   C    LEU  C  104      7.216   47.675    -6.352   1.00  20.95        C
ATOM   4115   O    LEU  C  104      7.450   48.237    -7.428   1.00  19.42        O
ATOM   4116   CB   LEU  C  104      8.544   49.063    -4.754   1.00  18.51        C
ATOM   4117   CG   LEU  C  104      7.340   49.727    -4.101   1.00  18.81        C
ATOM   4118   CD1  LEU  C  104      6.909   48.973    -2.866   1.00  19.80        C
ATOM   4119   CD2  LEU  C  104      7.685   51.142    -3.738   1.00  18.87        C
ATOM   4120   N    GLU  C  105      6.077   47.029    -6.090   1.00  23.59        N
ATOM   4121   CA   GLU  C  105      4.918   47.124    -6.983   1.00  25.56        C
ATOM   4122   C    GLU  C  105      3.781   47.907    -6.363   1.00  25.63        C
ATOM   4123   O    GLU  C  105      3.390   47.618    -5.246   1.00  25.44        O
ATOM   4124   CB   GLU  C  105      4.391   45.741    -7.399   1.00  27.07        C
ATOM   4125   CG   GLU  C  105      3.592   45.846    -8.720   1.00  28.43        C
ATOM   4126   CD   GLU  C  105      2.622   44.686    -9.030   1.00  30.61        C
ATOM   4127   OE1  GLU  C  105      2.913   43.512    -8.654   1.00  34.35        O
ATOM   4128   OE2  GLU  C  105      1.564   44.977    -9.672   1.00  33.13        O
ATOM   4129   N    ILE  C  106      3.278   48.893    -7.110   1.00  26.77        N
ATOM   4130   CA   ILE  C  106      2.171   49.741    -6.706   1.00  27.54        C
ATOM   4131   C    ILE  C  106      0.854   49.107    -7.148   1.00  27.30        C
ATOM   4132   O    ILE  C  106      0.712   48.692    -8.304   1.00  26.29        O
ATOM   4133   CB   ILE  C  106      2.233   51.146    -7.385   1.00  28.14        C
ATOM   4134   CG1  ILE  C  106      3.583   51.845    -7.159   1.00  29.58        C
ATOM   4135   CG2  ILE  C  106      1.107   52.029    -6.921   1.00  27.98        C
ATOM   4136   CD1  ILE  C  106      3.905   52.195    -5.757   1.00  29.92        C
ATOM   4137   N    LYS  C  107     -0.115   49.061    -6.233   1.00  27.94        N
ATOM   4138   CA   LYS  C  107     -1.486   48.682    -6.562   1.00  28.11        C
ATOM   4139   C    LYS  C  107     -2.280   49.948    -6.824   1.00  28.17        C
ATOM   4140   O    LYS  C  107     -2.291   50.863    -6.007   1.00  27.40        O
ATOM   4141   CB   LYS  C  107     -2.121   47.876    -5.434   1.00  28.48        C
ATOM   4142   CG   LYS  C  107     -3.588   47.577    -5.644   1.00  29.68        C
ATOM   4143   CD   LYS  C  107     -4.045   46.356    -4.838   1.00  30.31        C
ATOM   4144   CE   LYS  C  107     -5.559   46.079    -5.017   1.00  31.69        C
ATOM   4145   NZ   LYS  C  107     -6.155   45.195    -3.922   1.00  33.30        N
ATOM   4146   N    ARG  C  108     -2.928   49.994    -7.986   1.00  28.40        N
ATOM   4147   CA   ARG  C  108     -3.805   51.078    -8.347   1.00  27.66        C
ATOM   4148   C    ARG  C  108     -5.116   50.510    -8.884   1.00  27.55        C
```

| ATOM | 4149 | O | ARG | C | 108 | -5.319 | 49.287 | -8.957 | 1.00 | 26.17 | O |
|------|------|-----|-----|---|-----|--------|--------|---------|------|-------|---|
| ATOM | 4150 | CB | ARG | C | 108 | -3.117 | 51.997 | -9.371 | 1.00 | 28.48 | C |
| ATOM | 4151 | CG | ARG | C | 108 | -2.624 | 51.300 | -10.653 | 1.00 | 26.68 | C |
| ATOM | 4152 | CD | ARG | C | 108 | -2.595 | 52.297 | -11.780 | 1.00 | 27.25 | C |
| ATOM | 4153 | NE | ARG | C | 108 | -3.939 | 52.458 | -12.327 | 1.00 | 28.69 | N |
| ATOM | 4154 | CZ | ARG | C | 108 | -4.428 | 53.568 | -12.863 | 1.00 | 27.58 | C |
| ATOM | 4155 | NH1 | ARG | C | 108 | -3.700 | 54.671 | -12.959 | 1.00 | 28.96 | N |
| ATOM | 4156 | NH2 | ARG | C | 108 | -5.677 | 53.567 | -13.307 | 1.00 | 27.71 | N |
| ATOM | 4157 | N | ALA | C | 109 | -6.017 | 51.421 | -9.211 | 1.00 | 27.95 | N |
| ATOM | 4158 | CA | ALA | C | 109 | -7.308 | 51.084 | -9.770 | 1.00 | 28.62 | C |
| ATOM | 4159 | C | ALA | C | 109 | -7.108 | 50.423 | -11.119 | 1.00 | 28.97 | C |
| ATOM | 4160 | O | ALA | C | 109 | -6.236 | 50.819 | -11.882 | 1.00 | 29.23 | O |
| ATOM | 4161 | CB | ALA | C | 109 | -8.157 | 52.366 | -9.919 | 1.00 | 28.37 | C |
| ATOM | 4162 | N | ASP | C | 110 | -7.913 | 49.413 | -11.404 | 1.00 | 29.79 | N |
| ATOM | 4163 | CA | ASP | C | 110 | -7.865 | 48.763 | -12.702 | 1.00 | 29.58 | C |
| ATOM | 4164 | C | ASP | C | 110 | -8.047 | 49.776 | -13.842 | 1.00 | 29.40 | C |
| ATOM | 4165 | O | ASP | C | 110 | -8.726 | 50.806 | -13.685 | 1.00 | 28.08 | O |
| ATOM | 4166 | CB | ASP | C | 110 | -8.908 | 47.659 | -12.767 | 1.00 | 30.60 | C |
| ATOM | 4167 | CG | ASP | C | 110 | -8.592 | 46.504 | -11.837 | 1.00 | 31.84 | C |
| ATOM | 4168 | OD1 | ASP | C | 110 | -7.594 | 46.583 | -11.084 | 1.00 | 31.19 | O |
| ATOM | 4169 | OD2 | ASP | C | 110 | -9.346 | 45.501 | -11.869 | 1.00 | 34.47 | O |
| ATOM | 4170 | N | ALA | C | 111 | -7.378 | 49.506 | -14.970 | 1.00 | 28.65 | N |
| ATOM | 4171 | CA | ALA | C | 111 | -7.500 | 50.321 | -16.184 | 1.00 | 28.01 | C |
| ATOM | 4172 | C | ALA | C | 111 | -7.356 | 49.407 | -17.390 | 1.00 | 27.41 | C |
| ATOM | 4173 | O | ALA | C | 111 | -6.370 | 48.668 | -17.486 | 1.00 | 26.76 | O |
| ATOM | 4174 | CB | ALA | C | 111 | -6.457 | 51.356 | -16.226 | 1.00 | 28.09 | C |
| ATOM | 4175 | N | ALA | C | 112 | -8.329 | 49.466 | -18.302 | 1.00 | 26.74 | N |
| ATOM | 4176 | CA | ALA | C | 112 | -8.242 | 48.768 | -19.595 | 1.00 | 26.35 | C |
| ATOM | 4177 | C | ALA | C | 112 | -7.170 | 49.368 | -20.493 | 1.00 | 25.32 | C |
| ATOM | 4178 | O | ALA | C | 112 | -6.886 | 50.567 | -20.412 | 1.00 | 25.13 | O |
| ATOM | 4179 | CB | ALA | C | 112 | -9.583 | 48.808 | -20.311 | 1.00 | 27.09 | C |
| ATOM | 4180 | N | PRO | C | 113 | -6.589 | 48.542 | -21.375 | 1.00 | 24.59 | N |
| ATOM | 4181 | CA | PRO | C | 113 | -5.603 | 49.043 | -22.320 | 1.00 | 24.36 | C |
| ATOM | 4182 | C | PRO | C | 113 | -6.195 | 49.927 | -23.415 | 1.00 | 24.02 | C |
| ATOM | 4183 | O | PRO | C | 113 | -7.343 | 49.728 | -23.839 | 1.00 | 23.60 | O |
| ATOM | 4184 | CB | PRO | C | 113 | -5.021 | 47.764 | -22.929 | 1.00 | 24.61 | C |
| ATOM | 4185 | CG | PRO | C | 113 | -6.089 | 46.821 | -22.877 | 1.00 | 25.61 | C |
| ATOM | 4186 | CD | PRO | C | 113 | -6.847 | 47.113 | -21.591 | 1.00 | 25.71 | C |
| ATOM | 4187 | N | THR | C | 114 | -5.403 | 50.907 | -23.844 | 1.00 | 23.45 | N |
| ATOM | 4188 | CA | THR | C | 114 | -5.659 | 51.653 | -25.065 | 1.00 | 23.22 | C |
| ATOM | 4189 | C | THR | C | 114 | -4.852 | 50.951 | -26.166 | 1.00 | 22.55 | C |
| ATOM | 4190 | O | THR | C | 114 | -3.623 | 50.859 | -26.096 | 1.00 | 20.09 | O |
| ATOM | 4191 | CB | THR | C | 114 | -5.208 | 53.102 | -24.928 | 1.00 | 23.91 | C |
| ATOM | 4192 | OG1 | THR | C | 114 | -5.991 | 53.758 | -23.918 | 1.00 | 25.02 | O |
| ATOM | 4193 | CG2 | THR | C | 114 | -5.366 | 53.845 | -26.242 | 1.00 | 23.71 | C |
| ATOM | 4194 | N | VAL | C | 115 | -5.557 | 50.443 | -27.172 | 1.00 | 22.12 | N |
| ATOM | 4195 | CA | VAL | C | 115 | -4.951 | 49.558 | -28.159 | 1.00 | 20.63 | C |
| ATOM | 4196 | C | VAL | C | 115 | -4.851 | 50.273 | -29.484 | 1.00 | 20.73 | C |
| ATOM | 4197 | O | VAL | C | 115 | -5.840 | 50.807 | -29.961 | 1.00 | 21.04 | O |
| ATOM | 4198 | CB | VAL | C | 115 | -5.795 | 48.294 | -28.275 | 1.00 | 21.04 | C |
| ATOM | 4199 | CG1 | VAL | C | 115 | -5.161 | 47.266 | -29.214 | 1.00 | 21.53 | C |
| ATOM | 4200 | CG2 | VAL | C | 115 | -5.989 | 47.736 | -26.908 | 1.00 | 19.97 | C |
| ATOM | 4201 | N | SER | C | 116 | -3.641 | 50.266 | -30.053 | 1.00 | 19.84 | N |
| ATOM | 4202 | CA | SER | C | 116 | -3.315 | 50.821 | -31.343 | 1.00 | 19.88 | C |
| ATOM | 4203 | C | SER | C | 116 | -2.698 | 49.735 | -32.222 | 1.00 | 20.03 | C |
| ATOM | 4204 | O | SER | C | 116 | -1.901 | 48.925 | -31.725 | 1.00 | 18.85 | O |
| ATOM | 4205 | CB | SER | C | 116 | -2.306 | 51.944 | -31.157 | 1.00 | 21.09 | C |
| ATOM | 4206 | OG | SER | C | 116 | -2.911 | 52.993 | -30.413 | 1.00 | 22.72 | O |
| ATOM | 4207 | N | ILE | C | 117 | -3.111 | 49.707 | -33.496 | 1.00 | 19.28 | N |
| ATOM | 4208 | CA | ILE | C | 117 | -2.565 | 48.765 | -34.483 | 1.00 | 18.76 | C |
| ATOM | 4209 | C | ILE | C | 117 | -1.869 | 49.571 | -35.586 | 1.00 | 17.94 | C |
| ATOM | 4210 | O | ILE | C | 117 | -2.320 | 50.678 | -35.963 | 1.00 | 14.43 | O |
| ATOM | 4211 | CB | ILE | C | 117 | -3.622 | 47.805 | -35.070 | 1.00 | 19.53 | C |
| ATOM | 4212 | CG1 | ILE | C | 117 | -2.898 | 46.636 | -35.761 | 1.00 | 20.53 | C |
| ATOM | 4213 | CG2 | ILE | C | 117 | -4.586 | 48.563 | -36.010 | 1.00 | 20.27 | C |

```
ATOM   4214  CD1 ILE C 117     -3.765  45.478 -36.130  1.00 19.14          C
ATOM   4215  N   PHE C 118     -0.750  49.039 -36.070  1.00 15.52          N
ATOM   4216  CA  PHE C 118      0.094  49.747 -37.029  1.00 17.20          C
ATOM   4217  C   PHE C 118      0.464  48.816 -38.162  1.00 17.28          C
ATOM   4218  O   PHE C 118      1.094  47.774 -37.932  1.00 19.71          O
ATOM   4219  CB  PHE C 118      1.399  50.239 -36.350  1.00 16.55          C
ATOM   4220  CG  PHE C 118      1.188  51.283 -35.279  1.00 16.45          C
ATOM   4221  CD1 PHE C 118      1.205  50.954 -33.934  1.00 17.45          C
ATOM   4222  CD2 PHE C 118      1.005  52.603 -35.626  1.00 17.32          C
ATOM   4223  CE1 PHE C 118      0.990  51.928 -32.963  1.00 18.16          C
ATOM   4224  CE2 PHE C 118      0.822  53.584 -34.668  1.00 16.64          C
ATOM   4225  CZ  PHE C 118      0.809  53.274 -33.359  1.00 16.53          C
ATOM   4226  N   PRO C 119      0.106  49.165 -39.410  1.00 18.21          N
ATOM   4227  CA  PRO C 119      0.575  48.283 -40.505  1.00 17.47          C
ATOM   4228  C   PRO C 119      2.069  48.476 -40.843  1.00 16.85          C
ATOM   4229  O   PRO C 119      2.687  49.422 -40.362  1.00 16.45          O
ATOM   4230  CB  PRO C 119     -0.313  48.670 -41.691  1.00 18.43          C
ATOM   4231  CG  PRO C 119     -1.089  49.869 -41.286  1.00 19.61          C
ATOM   4232  CD  PRO C 119     -0.668  50.311 -39.911  1.00 19.58          C
ATOM   4233  N   PRO C 120      2.661  47.552 -41.617  1.00 16.45          N
ATOM   4234  CA  PRO C 120      4.053  47.744 -42.044  1.00 15.57          C
ATOM   4235  C   PRO C 120      4.322  49.097 -42.747  1.00 15.66          C
ATOM   4236  O   PRO C 120      3.454  49.651 -43.411  1.00 16.79          O
ATOM   4237  CB  PRO C 120      4.353  46.525 -42.943  1.00 14.89          C
ATOM   4238  CG  PRO C 120      3.125  45.687 -42.945  1.00 15.83          C
ATOM   4239  CD  PRO C 120      2.110  46.243 -42.022  1.00 17.04          C
ATOM   4240  N   SER C 121      5.507  49.644 -42.531  1.00 15.44          N
ATOM   4241  CA  SER C 121      5.931  50.866 -43.168  1.00 14.24          C
ATOM   4242  C   SER C 121      6.321  50.508 -44.599  1.00 14.82          C
ATOM   4243  O   SER C 121      6.618  49.340 -44.889  1.00 13.59          O
ATOM   4244  CB  SER C 121      7.124  51.433 -42.406  1.00 15.86          C
ATOM   4245  OG  SER C 121      8.210  50.516 -42.333  1.00 13.97          O
ATOM   4246  N   SER C 122      6.256  51.458 -45.508  1.00 14.13          N
ATOM   4247  CA  SER C 122      6.788  51.254 -46.858  1.00 14.19          C
ATOM   4248  C   SER C 122      8.280  50.901 -46.841  1.00 15.01          C
ATOM   4249  O   SER C 122      8.698  50.036 -47.610  1.00 16.49          O
ATOM   4250  CB  SER C 122      6.525  52.473 -47.731  1.00 17.18          C
ATOM   4251  OG  SER C 122      7.043  53.618 -47.075  1.00 19.70          O
ATOM   4252  N   GLU C 123      9.073  51.532 -45.967  1.00 14.05          N
ATOM   4253  CA  GLU C 123     10.491  51.224 -45.844  1.00 13.94          C
ATOM   4254  C   GLU C 123     10.651  49.728 -45.617  1.00 13.25          C
ATOM   4255  O   GLU C 123     11.425  49.026 -46.294  1.00 13.81          O
ATOM   4256  CB  GLU C 123     11.101  51.920 -44.630  1.00 15.25          C
ATOM   4257  CG  GLU C 123     11.302  53.428 -44.741  1.00 18.01          C
ATOM   4258  CD  GLU C 123     10.118  54.230 -44.220  1.00 18.57          C
ATOM   4259  OE1 GLU C 123      8.995  53.709 -44.213  1.00 16.40          O
ATOM   4260  OE2 GLU C 123     10.306  55.426 -43.856  1.00 20.97          O
ATOM   4261  N   GLN C 124      9.950  49.237 -44.614  1.00 11.70          N
ATOM   4262  CA  GLN C 124     10.114  47.802 -44.303  1.00 12.38          C
ATOM   4263  C   GLN C 124      9.686  46.884 -45.481  1.00 12.19          C
ATOM   4264  O   GLN C 124     10.398  45.917 -45.826  1.00 10.04          O
ATOM   4265  CB  GLN C 124      9.453  47.422 -42.973  1.00 12.45          C
ATOM   4266  CG  GLN C 124      9.810  45.980 -42.571  1.00 11.25          C
ATOM   4267  CD  GLN C 124      9.053  45.456 -41.377  1.00 13.92          C
ATOM   4268  OE1 GLN C 124      7.951  45.905 -41.066  1.00 14.66          O
ATOM   4269  NE2 GLN C 124      9.642  44.466 -40.709  1.00 16.95          N
ATOM   4270  N   LEU C 125      8.575  47.213 -46.140  1.00 12.11          N
ATOM   4271  CA  LEU C 125      8.075  46.432 -47.281  1.00 12.35          C
ATOM   4272  C   LEU C 125      9.053  46.493 -48.437  1.00 13.31          C
ATOM   4273  O   LEU C 125      9.256  45.516 -49.120  1.00  9.74          O
ATOM   4274  CB  LEU C 125      6.696  46.968 -47.736  1.00 11.98          C
ATOM   4275  CG  LEU C 125      5.555  46.745 -46.741  1.00 13.01          C
ATOM   4276  CD1 LEU C 125      4.370  47.479 -47.120  1.00 13.32          C
ATOM   4277  CD2 LEU C 125      5.231  45.216 -46.695  1.00 10.49          C
ATOM   4278  N   THR C 126      9.692  47.644 -48.639  1.00 11.95          N
```

573

EP 2 123 668 A1

```
ATOM   4279  CA   THR C 126      10.751  47.741 -49.680  1.00 11.77        C
ATOM   4280  C    THR C 126      11.934  46.752 -49.432  1.00 11.36        C
ATOM   4281  O    THR C 126      12.551  46.281 -50.369  1.00 11.83        O
ATOM   4282  CB   THR C 126      11.256  49.205 -49.783  1.00 12.99        C
ATOM   4283  OG1  THR C 126      10.240  50.042 -50.391  1.00 12.98        O
ATOM   4284  CG2  THR C 126      12.555  49.274 -50.538  1.00 12.14        C
ATOM   4285  N    SER C 127      12.229  46.492 -48.156  1.00 11.19        N
ATOM   4286  CA   SER C 127      13.297  45.581 -47.704  1.00  9.82        C
ATOM   4287  C    SER C 127      12.892  44.124 -47.751  1.00  8.92        C
ATOM   4288  O    SER C 127      13.726  43.302 -47.459  1.00 12.01        O
ATOM   4289  CB   SER C 127      13.758  45.909 -46.300  1.00  8.15        C
ATOM   4290  OG   SER C 127      12.868  45.490 -45.262  1.00 10.40        O
ATOM   4291  N    GLY C 128      11.635  43.836 -48.056  1.00 10.57        N
ATOM   4292  CA   GLY C 128      11.117  42.447 -48.082  1.00 10.87        C
ATOM   4293  C    GLY C 128      10.492  41.901 -46.799  1.00 12.18        C
ATOM   4294  O    GLY C 128      10.216  40.689 -46.704  1.00 12.61        O
ATOM   4295  N    GLY C 129      10.251  42.759 -45.821  1.00 14.61        N
ATOM   4296  CA   GLY C 129       9.650  42.337 -44.526  1.00 13.04        C
ATOM   4297  C    GLY C 129       8.317  43.003 -44.258  1.00 13.13        C
ATOM   4298  O    GLY C 129       7.984  43.980 -44.920  1.00 12.64        O
ATOM   4299  N    ALA C 130       7.537  42.454 -43.319  1.00 12.61        N
ATOM   4300  CA   ALA C 130       6.250  43.028 -42.943  1.00 13.61        C
ATOM   4301  C    ALA C 130       5.947  42.742 -41.482  1.00 13.27        C
ATOM   4302  O    ALA C 130       5.639  41.595 -41.114  1.00 15.57        O
ATOM   4303  CB   ALA C 130       5.153  42.489 -43.815  1.00 10.98        C
ATOM   4304  N    SER C 131       6.134  43.754 -40.651  1.00 14.37        N
ATOM   4305  CA   SER C 131       5.798  43.679 -39.243  1.00 14.67        C
ATOM   4306  C    SER C 131       4.536  44.476 -38.970  1.00 15.34        C
ATOM   4307  O    SER C 131       4.421  45.629 -39.382  1.00 13.59        O
ATOM   4308  CB   SER C 131       6.913  44.279 -38.377  1.00 15.61        C
ATOM   4309  OG   SER C 131       8.112  43.536 -38.400  1.00 13.79        O
ATOM   4310  N    VAL C 132       3.611  43.867 -38.236  1.00 15.79        N
ATOM   4311  CA   VAL C 132       2.401  44.546 -37.776  1.00 15.95        C
ATOM   4312  C    VAL C 132       2.565  44.720 -36.270  1.00 15.63        C
ATOM   4313  O    VAL C 132       2.965  43.785 -35.582  1.00 13.06        O
ATOM   4314  CB   VAL C 132       1.096  43.726 -38.117  1.00 16.78        C
ATOM   4315  CG1  VAL C 132      -0.156  44.568 -37.853  1.00 15.26        C
ATOM   4316  CG2  VAL C 132       1.085  43.245 -39.569  1.00 17.86        C
ATOM   4317  N    VAL C 133       2.348  45.931 -35.765  1.00 15.57        N
ATOM   4318  CA   VAL C 133       2.593  46.195 -34.360  1.00 16.84        C
ATOM   4319  C    VAL C 133       1.276  46.547 -33.681  1.00 18.29        C
ATOM   4320  O    VAL C 133       0.488  47.273 -34.229  1.00 16.81        O
ATOM   4321  CB   VAL C 133       3.603  47.302 -34.153  1.00 16.31        C
ATOM   4322  CG1  VAL C 133       3.723  47.642 -32.662  1.00 17.19        C
ATOM   4323  CG2  VAL C 133       4.979  46.893 -34.737  1.00 16.69        C
ATOM   4324  N    CYS C 134       1.057  45.961 -32.507  1.00 19.45        N
ATOM   4325  CA   CYS C 134      -0.046  46.301 -31.628  1.00 19.17        C
ATOM   4326  C    CYS C 134       0.514  46.853 -30.289  1.00 18.02        C
ATOM   4327  O    CYS C 134       1.300  46.183 -29.633  1.00 15.79        O
ATOM   4328  CB   CYS C 134      -0.860  45.006 -31.435  1.00 21.16        C
ATOM   4329  SG   CYS C 134      -2.475  45.136 -30.701  1.00 27.75        S
ATOM   4330  N    PHE C 135       0.220  48.106 -29.936  1.00 16.51        N
ATOM   4331  CA   PHE C 135       0.472  48.576 -28.567  1.00 17.16        C
ATOM   4332  C    PHE C 135      -0.763  48.385 -27.673  1.00 18.02        C
ATOM   4333  O    PHE C 135      -1.897  48.637 -28.088  1.00 17.76        O
ATOM   4334  CB   PHE C 135       0.854  50.043 -28.529  1.00 15.96        C
ATOM   4335  CG   PHE C 135       2.140  50.378 -29.226  1.00 15.65        C
ATOM   4336  CD1  PHE C 135       3.188  49.505 -29.296  1.00 15.75        C
ATOM   4337  CD2  PHE C 135       2.323  51.640 -29.732  1.00 14.55        C
ATOM   4338  CE1  PHE C 135       4.369  49.856 -29.926  1.00 16.56        C
ATOM   4339  CE2  PHE C 135       3.500  51.996 -30.377  1.00 17.36        C
ATOM   4340  CZ   PHE C 135       4.523  51.104 -30.462  1.00 17.18        C
ATOM   4341  N    LEU C 136      -0.532  47.900 -26.458  1.00 18.52        N
ATOM   4342  CA   LEU C 136      -1.561  47.763 -25.428  1.00 20.03        C
ATOM   4343  C    LEU C 136      -1.074  48.645 -24.295  1.00 20.77        C
```

574

```
ATOM   4344  O    LEU C 136      -0.274  48.215 -23.480  1.00 18.79           O
ATOM   4345  CB   LEU C 136      -1.660  46.316 -24.972  1.00 21.86           C
ATOM   4346  CG   LEU C 136      -2.349  45.320 -25.904  1.00 23.18           C
ATOM   4347  CD1  LEU C 136      -1.596  45.137 -27.220  1.00 24.71           C
ATOM   4348  CD2  LEU C 136      -2.499  43.995 -25.181  1.00 22.51           C
ATOM   4349  N    ASN C 137      -1.486  49.905 -24.315  1.00 20.62           N
ATOM   4350  CA   ASN C 137      -0.908  50.948 -23.490  1.00 21.17           C
ATOM   4351  C    ASN C 137      -1.726  51.289 -22.231  1.00 21.54           C
ATOM   4352  O    ASN C 137      -2.967  51.285 -22.242  1.00 19.88           O
ATOM   4353  CB   ASN C 137      -0.710  52.216 -24.314  1.00 21.53           C
ATOM   4354  CG   ASN C 137       0.571  52.188 -25.171  1.00 22.08           C
ATOM   4355  OD1  ASN C 137       1.419  51.335 -25.004  1.00 23.35           O
ATOM   4356  ND2  ASN C 137       0.709  53.158 -26.068  1.00 21.54           N
ATOM   4357  N    ASN C 138      -0.991  51.558 -21.157  1.00 22.01           N
ATOM   4358  CA   ASN C 138      -1.524  52.099 -19.897  1.00 23.49           C
ATOM   4359  C    ASN C 138      -2.739  51.364 -19.327  1.00 23.66           C
ATOM   4360  O    ASN C 138      -3.826  51.929 -19.173  1.00 24.71           O
ATOM   4361  CB   ASN C 138      -1.778  53.598 -20.031  1.00 24.53           C
ATOM   4362  CG   ASN C 138      -0.557  54.352 -20.531  1.00 26.56           C
ATOM   4363  OD1  ASN C 138      -0.230  54.319 -21.712  1.00 29.80           O
ATOM   4364  ND2  ASN C 138       0.130  55.032 -19.625  1.00 29.74           N
ATOM   4365  N    PHE C 139      -2.522  50.088 -19.008  1.00 22.67           N
ATOM   4366  CA   PHE C 139      -3.492  49.239 -18.330  1.00 24.10           C
ATOM   4367  C    PHE C 139      -2.977  48.771 -16.961  1.00 24.58           C
ATOM   4368  O    PHE C 139      -1.794  48.879 -16.650  1.00 23.87           O
ATOM   4369  CB   PHE C 139      -3.811  48.001 -19.164  1.00 23.23           C
ATOM   4370  CG   PHE C 139      -2.601  47.150 -19.491  1.00 22.90           C
ATOM   4371  CD1  PHE C 139      -2.214  46.109 -18.650  1.00 22.63           C
ATOM   4372  CD2  PHE C 139      -1.856  47.381 -20.636  1.00 23.25           C
ATOM   4373  CE1  PHE C 139      -1.098  45.332 -18.932  1.00 23.70           C
ATOM   4374  CE2  PHE C 139      -0.734  46.605 -20.932  1.00 21.10           C
ATOM   4375  CZ   PHE C 139      -0.364  45.566 -20.080  1.00 23.24           C
ATOM   4376  N    TYR C 140      -3.897  48.282 -16.143  1.00 25.44           N
ATOM   4377  CA   TYR C 140      -3.556  47.706 -14.851  1.00 26.04           C
ATOM   4378  C    TYR C 140      -4.674  46.785 -14.454  1.00 26.50           C
ATOM   4379  O    TYR C 140      -5.827  47.164 -14.591  1.00 25.93           O
ATOM   4380  CB   TYR C 140      -3.381  48.805 -13.799  1.00 26.82           C
ATOM   4381  CG   TYR C 140      -2.889  48.235 -12.483  1.00 26.72           C
ATOM   4382  CD1  TYR C 140      -3.770  47.759 -11.534  1.00 26.87           C
ATOM   4383  CD2  TYR C 140      -1.538  48.138 -12.224  1.00 25.53           C
ATOM   4384  CE1  TYR C 140      -3.302  47.206 -10.358  1.00 27.80           C
ATOM   4385  CE2  TYR C 140      -1.073  47.608 -11.063  1.00 27.12           C
ATOM   4386  CZ   TYR C 140      -1.954  47.128 -10.141  1.00 27.44           C
ATOM   4387  OH   TYR C 140      -1.446  46.593  -8.991  1.00 29.09           O
ATOM   4388  N    PRO C 141      -4.358  45.587 -13.928  1.00 27.77           N
ATOM   4389  CA   PRO C 141      -3.061  44.992 -13.582  1.00 27.94           C
ATOM   4390  C    PRO C 141      -2.207  44.467 -14.755  1.00 27.96           C
ATOM   4391  O    PRO C 141      -2.649  44.507 -15.904  1.00 28.03           O
ATOM   4392  CB   PRO C 141      -3.454  43.837 -12.649  1.00 28.15           C
ATOM   4393  CG   PRO C 141      -4.794  43.444 -13.108  1.00 28.88           C
ATOM   4394  CD   PRO C 141      -5.474  44.696 -13.571  1.00 28.77           C
ATOM   4395  N    LYS C 142      -1.017  43.956 -14.424  1.00 27.95           N
ATOM   4396  CA   LYS C 142       0.023  43.588 -15.399  1.00 28.98           C
ATOM   4397  C    LYS C 142      -0.383  42.517 -16.402  1.00 28.70           C
ATOM   4398  O    LYS C 142       0.089  42.513 -17.545  1.00 28.95           O
ATOM   4399  CB   LYS C 142       1.342  43.164 -14.704  1.00 28.69           C
ATOM   4400  CG   LYS C 142       2.562  43.211 -15.660  1.00 29.54           C
ATOM   4401  CD   LYS C 142       3.970  43.193 -14.996  1.00 29.99           C
ATOM   4402  CE   LYS C 142       5.083  43.346 -16.088  1.00 29.48           C
ATOM   4403  NZ   LYS C 142       6.447  43.859 -15.668  1.00 29.40           N
ATOM   4404  N    ASP C 143      -1.249  41.607 -15.982  1.00 29.24           N
ATOM   4405  CA   ASP C 143      -1.533  40.432 -16.791  1.00 30.88           C
ATOM   4406  C    ASP C 143      -2.429  40.770 -17.961  1.00 30.36           C
ATOM   4407  O    ASP C 143      -3.476  41.371 -17.784  1.00 30.32           O
ATOM   4408  CB   ASP C 143      -2.152  39.354 -15.924  1.00 32.56           C
```

```
ATOM   4409  CG  ASP C 143      -1.283  39.048 -14.718  1.00 36.18           C
ATOM   4410  OD1 ASP C 143      -0.357  38.212 -14.870  1.00 39.75           O
ATOM   4411  OD2 ASP C 143      -1.504  39.691 -13.651  1.00 39.39           O
ATOM   4412  N   ILE C 144      -2.000  40.386 -19.157  1.00 30.07           N
ATOM   4413  CA  ILE C 144      -2.807  40.612 -20.345  1.00 30.05           C
ATOM   4414  C   ILE C 144      -2.499  39.603 -21.443  1.00 30.79           C
ATOM   4415  O   ILE C 144      -1.394  39.057 -21.502  1.00 31.97           O
ATOM   4416  CB  ILE C 144      -2.607  42.048 -20.840  1.00 29.23           C
ATOM   4417  CG1 ILE C 144      -3.747  42.455 -21.779  1.00 28.93           C
ATOM   4418  CG2 ILE C 144      -1.238  42.207 -21.482  1.00 29.09           C
ATOM   4419  CD1 ILE C 144      -3.910  43.933 -21.841  1.00 29.21           C
ATOM   4420  N   ASN C 145      -3.490  39.328 -22.288  1.00 31.03           N
ATOM   4421  CA  ASN C 145      -3.304  38.432 -23.419  1.00 31.22           C
ATOM   4422  C   ASN C 145      -3.573  39.130 -24.728  1.00 29.93           C
ATOM   4423  O   ASN C 145      -4.444  39.975 -24.818  1.00 28.41           O
ATOM   4424  CB  ASN C 145      -4.178  37.198 -23.293  1.00 32.72           C
ATOM   4425  CG  ASN C 145      -3.595  36.179 -22.334  1.00 36.88           C
ATOM   4426  OD1 ASN C 145      -2.703  35.392 -22.692  1.00 40.09           O
ATOM   4427  ND2 ASN C 145      -4.093  36.185 -21.104  1.00 38.47           N
ATOM   4428  N   VAL C 146      -2.800  38.765 -25.744  1.00 30.22           N
ATOM   4429  CA  VAL C 146      -3.007  39.289 -27.089  1.00 29.56           C
ATOM   4430  C   VAL C 146      -3.053  38.115 -28.041  1.00 28.66           C
ATOM   4431  O   VAL C 146      -2.262  37.183 -27.918  1.00 27.17           O
ATOM   4432  CB  VAL C 146      -1.887  40.268 -27.532  1.00 30.81           C
ATOM   4433  CG1 VAL C 146      -0.526  39.540 -27.640  1.00 31.32           C
ATOM   4434  CG2 VAL C 146      -2.249  40.933 -28.858  1.00 30.73           C
ATOM   4435  N   LYS C 147      -3.987  38.186 -28.983  1.00 27.95           N
ATOM   4436  CA  LYS C 147      -4.111  37.195 -30.042  1.00 26.50           C
ATOM   4437  C   LYS C 147      -4.141  37.875 -31.387  1.00 24.26           C
ATOM   4438  O   LYS C 147      -4.936  38.781 -31.593  1.00 26.06           O
ATOM   4439  CB  LYS C 147      -5.383  36.383 -29.899  1.00 28.12           C
ATOM   4440  CG  LYS C 147      -5.283  35.040 -30.623  1.00 30.60           C
ATOM   4441  CD  LYS C 147      -6.506  34.736 -31.457  1.00 33.45           C
ATOM   4442  CE  LYS C 147      -7.716  34.360 -30.610  1.00 34.84           C
ATOM   4443  NZ  LYS C 147      -8.696  33.519 -31.413  1.00 35.64           N
ATOM   4444  N   TRP C 148      -3.286  37.412 -32.295  1.00 22.79           N
ATOM   4445  CA  TRP C 148      -3.253  37.881 -33.691  1.00 21.63           C
ATOM   4446  C   TRP C 148      -4.115  37.003 -34.603  1.00 21.93           C
ATOM   4447  O   TRP C 148      -4.084  35.767 -34.508  1.00 20.62           O
ATOM   4448  CB  TRP C 148      -1.826  37.899 -34.230  1.00 19.17           C
ATOM   4449  CG  TRP C 148      -1.008  39.017 -33.695  1.00 17.21           C
ATOM   4450  CD1 TRP C 148      -0.170  38.979 -32.628  1.00 17.86           C
ATOM   4451  CD2 TRP C 148      -0.976  40.360 -34.184  1.00 16.93           C
ATOM   4452  NE1 TRP C 148       0.398  40.223 -32.427  1.00 18.24           N
ATOM   4453  CE2 TRP C 148      -0.088  41.083 -33.367  1.00 16.64           C
ATOM   4454  CE3 TRP C 148      -1.582  41.008 -35.256  1.00 16.09           C
ATOM   4455  CZ2 TRP C 148       0.201  42.426 -33.589  1.00 19.29           C
ATOM   4456  CZ3 TRP C 148      -1.329  42.339 -35.447  1.00 17.67           C
ATOM   4457  CH2 TRP C 148      -0.445  43.034 -34.632  1.00 17.04           C
ATOM   4458  N   LYS C 149      -4.856  37.641 -35.504  1.00 20.57           N
ATOM   4459  CA  LYS C 149      -5.606  36.893 -36.521  1.00 21.66           C
ATOM   4460  C   LYS C 149      -5.218  37.381 -37.895  1.00 20.44           C
ATOM   4461  O   LYS C 149      -5.117  38.583 -38.119  1.00 19.99           O
ATOM   4462  CB  LYS C 149      -7.103  37.067 -36.357  1.00 22.90           C
ATOM   4463  CG  LYS C 149      -7.741  36.310 -35.213  1.00 24.37           C
ATOM   4464  CD  LYS C 149      -9.234  36.306 -35.459  1.00 25.99           C
ATOM   4465  CE  LYS C 149     -10.011  35.515 -34.433  1.00 28.27           C
ATOM   4466  NZ  LYS C 149     -11.486  35.828 -34.582  1.00 28.53           N
ATOM   4467  N   ILE C 150      -4.992  36.448 -38.801  1.00 19.46           N
ATOM   4468  CA  ILE C 150      -4.732  36.779 -40.194  1.00 19.73           C
ATOM   4469  C   ILE C 150      -5.866  36.196 -40.999  1.00 19.47           C
ATOM   4470  O   ILE C 150      -6.071  34.990 -40.944  1.00 18.21           O
ATOM   4471  CB  ILE C 150      -3.397  36.191 -40.690  1.00 18.81           C
ATOM   4472  CG1 ILE C 150      -2.255  36.703 -39.797  1.00 19.76           C
ATOM   4473  CG2 ILE C 150      -3.209  36.498 -42.181  1.00 18.78           C
```

576

```
ATOM   4474  CD1 ILE C 150    -0.864  36.270 -40.187  1.00 20.04          C
ATOM   4475  N   ASP C 151    -6.585  37.037 -41.743  1.00 18.68          N
ATOM   4476  CA  ASP C 151    -7.816  36.608 -42.451  1.00 20.36          C
ATOM   4477  C   ASP C 151    -8.675  35.731 -41.550  1.00 20.81          C
ATOM   4478  O   ASP C 151    -9.099  34.648 -41.950  1.00 20.31          O
ATOM   4479  CB  ASP C 151    -7.495  35.859 -43.738  1.00 20.94          C
ATOM   4480  CG  ASP C 151    -6.984  36.772 -44.842  1.00 22.70          C
ATOM   4481  OD1 ASP C 151    -7.084  38.024 -44.697  1.00 23.21          O
ATOM   4482  OD2 ASP C 151    -6.475  36.235 -45.862  1.00 20.53          O
ATOM   4483  N   GLY C 152    -8.894  36.191 -40.321  1.00 21.74          N
ATOM   4484  CA  GLY C 152    -9.788  35.511 -39.386  1.00 23.24          C
ATOM   4485  C   GLY C 152    -9.240  34.288 -38.679  1.00 23.58          C
ATOM   4486  O   GLY C 152    -9.868  33.805 -37.751  1.00 24.94          O
ATOM   4487  N   SER C 153    -8.077  33.796 -39.083  1.00 24.87          N
ATOM   4488  CA  SER C 153    -7.460  32.622 -38.436  1.00 26.98          C
ATOM   4489  C   SER C 153    -6.305  32.987 -37.489  1.00 27.26          C
ATOM   4490  O   SER C 153    -5.472  33.852 -37.776  1.00 24.53          O
ATOM   4491  CB  SER C 153    -6.959  31.618 -39.484  1.00 27.46          C
ATOM   4492  OG  SER C 153    -8.034  31.050 -40.221  1.00 31.24          O
ATOM   4493  N   GLU C 154    -6.277  32.308 -36.350  1.00 29.44          N
ATOM   4494  CA  GLU C 154    -5.328  32.622 -35.290  1.00 31.17          C
ATOM   4495  C   GLU C 154    -3.914  32.384 -35.789  1.00 31.81          C
ATOM   4496  O   GLU C 154    -3.663  31.441 -36.547  1.00 33.33          O
ATOM   4497  CB  GLU C 154    -5.598  31.776 -34.038  1.00 31.74          C
ATOM   4498  CG  GLU C 154    -4.500  31.841 -32.986  1.00 32.26          C
ATOM   4499  CD  GLU C 154    -4.779  30.988 -31.744  1.00 34.15          C
ATOM   4500  OE1 GLU C 154    -5.584  30.019 -31.817  1.00 35.52          O
ATOM   4501  OE2 GLU C 154    -4.161  31.302 -30.693  1.00 38.13          O
ATOM   4502  N   ARG C 155    -3.004  33.240 -35.337  1.00 32.95          N
ATOM   4503  CA  ARG C 155    -1.608  33.185 -35.723  1.00 33.11          C
ATOM   4504  C   ARG C 155    -0.718  33.284 -34.478  1.00 34.30          C
ATOM   4505  O   ARG C 155    -0.718  34.312 -33.773  1.00 33.71          O
ATOM   4506  CB  ARG C 155    -1.298  34.332 -36.689  1.00 33.35          C
ATOM   4507  CG  ARG C 155     0.161  34.439 -37.112  1.00 33.02          C
ATOM   4508  CD  ARG C 155     0.647  33.154 -37.750  1.00 34.42          C
ATOM   4509  NE  ARG C 155     1.969  33.323 -38.345  1.00 34.62          N
ATOM   4510  CZ  ARG C 155     2.225  33.303 -39.649  1.00 35.69          C
ATOM   4511  NH1 ARG C 155     1.260  33.095 -40.544  1.00 35.82          N
ATOM   4512  NH2 ARG C 155     3.471  33.486 -40.058  1.00 34.95          N
ATOM   4513  N   GLN C 156     0.045  32.217 -34.228  1.00 34.81          N
ATOM   4514  CA  GLN C 156     0.935  32.138 -33.065  1.00 35.05          C
ATOM   4515  C   GLN C 156     2.379  32.491 -33.428  1.00 34.63          C
ATOM   4516  O   GLN C 156     3.026  33.309 -32.763  1.00 34.84          O
ATOM   4517  CB  GLN C 156     0.889  30.727 -32.452  1.00 35.89          C
ATOM   4518  CG  GLN C 156    -0.509  30.238 -32.045  1.00 36.80          C
ATOM   4519  CD  GLN C 156    -0.476  29.198 -30.922  1.00 37.87          C
ATOM   4520  OE1 GLN C 156    -0.348  29.543 -29.741  1.00 41.22          O
ATOM   4521  NE2 GLN C 156    -0.596  27.922 -31.284  1.00 38.56          N
ATOM   4522  N   ASN C 157     2.872  31.893 -34.504  1.00 33.72          N
ATOM   4523  CA  ASN C 157     4.287  31.969 -34.849  1.00 32.61          C
ATOM   4524  C   ASN C 157     4.630  33.305 -35.475  1.00 30.38          C
ATOM   4525  O   ASN C 157     3.833  33.840 -36.225  1.00 29.39          O
ATOM   4526  CB  ASN C 157     4.628  30.814 -35.780  1.00 34.48          C
ATOM   4527  CG  ASN C 157     4.312  29.454 -35.140  1.00 35.79          C
ATOM   4528  OD1 ASN C 157     4.958  29.055 -34.174  1.00 37.28          O
ATOM   4529  ND2 ASN C 157     3.280  28.785 -35.630  1.00 37.17          N
ATOM   4530  N   GLY C 158     5.807  33.834 -35.155  1.00 28.82          N
ATOM   4531  CA  GLY C 158     6.242  35.154 -35.635  1.00 28.45          C
ATOM   4532  C   GLY C 158     5.843  36.312 -34.741  1.00 27.39          C
ATOM   4533  O   GLY C 158     6.011  37.482 -35.122  1.00 27.24          O
ATOM   4534  N   VAL C 159     5.331  35.995 -33.549  1.00 26.71          N
ATOM   4535  CA  VAL C 159     4.833  36.995 -32.609  1.00 26.17          C
ATOM   4536  C   VAL C 159     5.841  37.206 -31.511  1.00 26.62          C
ATOM   4537  O   VAL C 159     6.317  36.245 -30.916  1.00 28.27          O
ATOM   4538  CB  VAL C 159     3.462  36.600 -31.981  1.00 26.07          C
```

```
ATOM   4539  CG1 VAL C 159      2.972  37.681 -30.991  1.00 25.33           C
ATOM   4540  CG2 VAL C 159      2.407  36.367 -33.054  1.00 24.84           C
ATOM   4541  N   LEU C 160      6.168  38.469 -31.249  1.00 25.04           N
ATOM   4542  CA  LEU C 160      7.047  38.835 -30.152  1.00 25.72           C
ATOM   4543  C   LEU C 160      6.430  39.911 -29.253  1.00 24.03           C
ATOM   4544  O   LEU C 160      6.079  41.020 -29.701  1.00 22.55           O
ATOM   4545  CB  LEU C 160      8.381  39.327 -30.692  1.00 27.11           C
ATOM   4546  CG  LEU C 160      9.274  38.346 -31.461  1.00 29.10           C
ATOM   4547  CD1 LEU C 160     10.464  39.158 -31.944  1.00 29.93           C
ATOM   4548  CD2 LEU C 160      9.749  37.090 -30.654  1.00 30.59           C
ATOM   4549  N   ASN C 161      6.353  39.575 -27.971  1.00 22.73           N
ATOM   4550  CA  ASN C 161      5.715  40.391 -26.957  1.00 23.24           C
ATOM   4551  C   ASN C 161      6.716  40.933 -25.945  1.00 23.39           C
ATOM   4552  O   ASN C 161      7.644  40.231 -25.529  1.00 24.82           O
ATOM   4553  CB  ASN C 161      4.650  39.565 -26.230  1.00 22.51           C
ATOM   4554  CG  ASN C 161      3.593  39.037 -27.172  1.00 24.08           C
ATOM   4555  OD1 ASN C 161      3.126  39.752 -28.050  1.00 20.84           O
ATOM   4556  ND2 ASN C 161      3.234  37.753 -27.017  1.00 23.44           N
ATOM   4557  N   SER C 162      6.490  42.172 -25.528  1.00 22.24           N
ATOM   4558  CA  SER C 162      7.315  42.843 -24.542  1.00 21.94           C
ATOM   4559  C   SER C 162      6.472  43.745 -23.626  1.00 21.33           C
ATOM   4560  O   SER C 162      5.644  44.518 -24.109  1.00 19.14           O
ATOM   4561  CB  SER C 162      8.317  43.728 -25.266  1.00 21.31           C
ATOM   4562  OG  SER C 162      9.214  44.298 -24.357  1.00 20.85           O
ATOM   4563  N   TRP C 163      6.734  43.679 -22.320  1.00 21.52           N
ATOM   4564  CA  TRP C 163      6.042  44.474 -21.315  1.00 22.32           C
ATOM   4565  C   TRP C 163      6.975  45.478 -20.735  1.00 22.26           C
ATOM   4566  O   TRP C 163      8.099  45.138 -20.363  1.00 20.40           O
ATOM   4567  CB  TRP C 163      5.625  43.614 -20.117  1.00 26.09           C
ATOM   4568  CG  TRP C 163      4.338  42.965 -20.235  1.00 27.44           C
ATOM   4569  CD1 TRP C 163      3.165  43.364 -19.666  1.00 28.96           C
ATOM   4570  CD2 TRP C 163      4.060  41.758 -20.939  1.00 28.18           C
ATOM   4571  NE1 TRP C 163      2.164  42.487 -19.998  1.00 29.11           N
ATOM   4572  CE2 TRP C 163      2.691  41.491 -20.778  1.00 29.01           C
ATOM   4573  CE3 TRP C 163      4.831  40.890 -21.704  1.00 27.66           C
ATOM   4574  CZ2 TRP C 163      2.077  40.380 -21.347  1.00 28.91           C
ATOM   4575  CZ3 TRP C 163      4.227  39.796 -22.268  1.00 28.67           C
ATOM   4576  CH2 TRP C 163      2.859  39.551 -22.095  1.00 29.27           C
ATOM   4577  N   THR C 164      6.491  46.689 -20.543  1.00 19.79           N
ATOM   4578  CA  THR C 164      7.250  47.668 -19.794  1.00 21.49           C
ATOM   4579  C   THR C 164      7.218  47.364 -18.297  1.00 20.67           C
ATOM   4580  O   THR C 164      6.417  46.555 -17.813  1.00 18.93           O
ATOM   4581  CB  THR C 164      6.689  49.089 -19.940  1.00 21.20           C
ATOM   4582  OG1 THR C 164      5.310  49.088 -19.548  1.00 23.59           O
ATOM   4583  CG2 THR C 164      6.827  49.587 -21.344  1.00 22.64           C
ATOM   4584  N   ASP C 165      8.097  48.055 -17.581  1.00 22.70           N
ATOM   4585  CA  ASP C 165      8.035  48.124 -16.126  1.00 23.69           C
ATOM   4586  C   ASP C 165      6.932  49.086 -15.761  1.00 23.59           C
ATOM   4587  O   ASP C 165      6.565  49.961 -16.561  1.00 22.93           O
ATOM   4588  CB  ASP C 165      9.326  48.689 -15.542  1.00 25.01           C
ATOM   4589  CG  ASP C 165     10.477  47.713 -15.576  1.00 28.27           C
ATOM   4590  OD1 ASP C 165     10.279  46.469 -15.563  1.00 31.34           O
ATOM   4591  OD2 ASP C 165     11.610  48.220 -15.568  1.00 32.06           O
ATOM   4592  N   GLN C 166      6.452  48.957 -14.524  1.00 23.18           N
ATOM   4593  CA  GLN C 166      5.369  49.788 -14.028  1.00 22.97           C
ATOM   4594  C   GLN C 166      5.734  51.250 -14.185  1.00 22.66           C
ATOM   4595  O   GLN C 166      6.853  51.673 -13.884  1.00 22.07           O
ATOM   4596  CB  GLN C 166      5.126  49.510 -12.550  1.00 23.11           C
ATOM   4597  CG  GLN C 166      3.910  50.193 -11.963  1.00 21.44           C
ATOM   4598  CD  GLN C 166      3.409  49.456 -10.741  1.00 22.21           C
ATOM   4599  OE1 GLN C 166      4.202  48.879  -9.949  1.00 19.07           O
ATOM   4600  NE2 GLN C 166      2.095  49.410 -10.596  1.00 19.62           N
ATOM   4601  N   ASP C 167      4.780  52.030 -14.658  1.00 23.73           N
ATOM   4602  CA  ASP C 167      5.072  53.413 -14.970  1.00 26.10           C
ATOM   4603  C   ASP C 167      5.253  54.265 -13.721  1.00 26.89           C
```

```
ATOM   4604  O    ASP C 167    4.439  54.185 -12.791  1.00 26.36          O
ATOM   4605  CB   ASP C 167    3.965  54.014 -15.810  1.00 27.18          C
ATOM   4606  CG   ASP C 167    4.313  55.380 -16.271  1.00 27.94          C
ATOM   4607  OD1  ASP C 167    5.187  55.460 -17.156  1.00 34.05          O
ATOM   4608  OD2  ASP C 167    3.786  56.373 -15.732  1.00 29.46          O
ATOM   4609  N    SER C 168    6.296  55.103 -13.725  1.00 27.88          N
ATOM   4610  CA   SER C 168    6.676  55.931 -12.558  1.00 29.05          C
ATOM   4611  C    SER C 168    5.748  57.134 -12.291  1.00 30.01          C
ATOM   4612  O    SER C 168    5.935  57.866 -11.310  1.00 29.92          O
ATOM   4613  CB   SER C 168    8.110  56.438 -12.709  1.00 29.86          C
ATOM   4614  OG   SER C 168    9.044  55.411 -12.423  1.00 33.43          O
ATOM   4615  N    LYS C 169    4.757  57.307 -13.165  1.00 29.88          N
ATOM   4616  CA   LYS C 169    3.822  58.419 -13.141  1.00 29.97          C
ATOM   4617  C    LYS C 169    2.400  57.941 -12.820  1.00 29.78          C
ATOM   4618  O    LYS C 169    1.758  58.430 -11.883  1.00 30.97          O
ATOM   4619  CB   LYS C 169    3.844  59.113 -14.523  1.00 31.37          C
ATOM   4620  CG   LYS C 169    4.494  60.465 -14.580  1.00 33.48          C
ATOM   4621  CD   LYS C 169    5.917  60.478 -14.085  1.00 36.14          C
ATOM   4622  CE   LYS C 169    6.571  61.838 -14.396  1.00 36.29          C
ATOM   4623  NZ   LYS C 169    5.590  62.965 -14.290  1.00 39.20          N
ATOM   4624  N    ASP C 170    1.903  56.980 -13.590  1.00 28.38          N
ATOM   4625  CA   ASP C 170    0.522  56.542 -13.436  1.00 26.98          C
ATOM   4626  C    ASP C 170    0.387  55.102 -12.963  1.00 24.94          C
ATOM   4627  O    ASP C 170   -0.708  54.579 -12.878  1.00 25.36          O
ATOM   4628  CB   ASP C 170   -0.273  56.784 -14.730  1.00 27.26          C
ATOM   4629  CG   ASP C 170    0.201  55.940 -15.907  1.00 29.52          C
ATOM   4630  OD1  ASP C 170    0.880  54.911 -15.718  1.00 28.34          O
ATOM   4631  OD2  ASP C 170   -0.140  56.305 -17.053  1.00 30.60          O
ATOM   4632  N    SER C 171    1.498  54.461 -12.656  1.00 24.29          N
ATOM   4633  CA   SER C 171    1.476  53.121 -12.079  1.00 23.05          C
ATOM   4634  C    SER C 171    0.875  52.049 -12.983  1.00 22.17          C
ATOM   4635  O    SER C 171    0.571  50.930 -12.536  1.00 22.92          O
ATOM   4636  CB   SER C 171    0.795  53.173 -10.710  1.00 23.61          C
ATOM   4637  OG   SER C 171    1.478  54.128  -9.921  1.00 20.06          O
ATOM   4638  N    THR C 172    0.784  52.358 -14.280  1.00 22.18          N
ATOM   4639  CA   THR C 172    0.270  51.417 -15.256  1.00 21.89          C
ATOM   4640  C    THR C 172    1.405  50.643 -15.917  1.00 21.12          C
ATOM   4641  O    THR C 172    2.589  50.988 -15.788  1.00 21.83          O
ATOM   4642  CB   THR C 172   -0.529  52.148 -16.358  1.00 21.45          C
ATOM   4643  OG1  THR C 172    0.358  53.026 -17.067  1.00 20.55          O
ATOM   4644  CG2  THR C 172   -1.694  52.927 -15.750  1.00 20.26          C
ATOM   4645  N    TYR C 173    1.013  49.581 -16.605  1.00 20.45          N
ATOM   4646  CA   TYR C 173    1.878  48.842 -17.501  1.00 20.39          C
ATOM   4647  C    TYR C 173    1.446  49.018 -18.959  1.00 20.00          C
ATOM   4648  O    TYR C 173    0.271  49.283 -19.243  1.00 20.73          O
ATOM   4649  CB   TYR C 173    1.796  47.361 -17.152  1.00 20.88          C
ATOM   4650  CG   TYR C 173    2.179  47.056 -15.728  1.00 21.01          C
ATOM   4651  CD1  TYR C 173    1.226  47.016 -14.724  1.00 21.49          C
ATOM   4652  CD2  TYR C 173    3.507  46.832 -15.382  1.00 23.41          C
ATOM   4653  CE1  TYR C 173    1.586  46.719 -13.379  1.00 22.19          C
ATOM   4654  CE2  TYR C 173    3.876  46.531 -14.058  1.00 21.98          C
ATOM   4655  CZ   TYR C 173    2.903  46.468 -13.066  1.00 22.68          C
ATOM   4656  OH   TYR C 173    3.247  46.195 -11.740  1.00 24.06          O
ATOM   4657  N    SER C 174    2.396  48.831 -19.873  1.00 19.17          N
ATOM   4658  CA   SER C 174    2.093  48.751 -21.291  1.00 19.33          C
ATOM   4659  C    SER C 174    2.738  47.502 -21.890  1.00 20.03          C
ATOM   4660  O    SER C 174    3.656  46.928 -21.288  1.00 19.23          O
ATOM   4661  CB   SER C 174    2.558  50.009 -21.997  1.00 18.91          C
ATOM   4662  OG   SER C 174    1.952  51.140 -21.393  1.00 18.15          O
ATOM   4663  N    MET C 175    2.248  47.068 -23.051  1.00 20.12          N
ATOM   4664  CA   MET C 175    2.804  45.903 -23.722  1.00 20.26          C
ATOM   4665  C    MET C 175    2.797  46.126 -25.240  1.00 20.44          C
ATOM   4666  O    MET C 175    1.906  46.807 -25.751  1.00 18.87          O
ATOM   4667  CB   MET C 175    2.009  44.655 -23.353  1.00 21.83          C
ATOM   4668  CG   MET C 175    2.660  43.371 -23.760  1.00 24.67          C
```

,

```
ATOM   4669  SD  MET C 175      1.936  42.628 -25.235  1.00 33.67           S
ATOM   4670  CE  MET C 175      0.827  41.465 -24.459  1.00 30.18           C
ATOM   4671  N   SER C 176      3.827  45.605 -25.919  1.00 18.31           N
ATOM   4672  CA  SER C 176      3.975  45.711 -27.355  1.00 18.24           C
ATOM   4673  C   SER C 176      3.939  44.286 -27.886  1.00 18.12           C
ATOM   4674  O   SER C 176      4.558  43.423 -27.306  1.00 17.36           O
ATOM   4675  CB  SER C 176      5.300  46.382 -27.701  1.00 19.50           C
ATOM   4676  OG  SER C 176      5.512  46.474 -29.103  1.00 22.42           O
ATOM   4677  N   SER C 177      3.165  44.062 -28.939  1.00 17.34           N
ATOM   4678  CA  SER C 177      3.134  42.807 -29.639  1.00 17.27           C
ATOM   4679  C   SER C 177      3.393  43.103 -31.108  1.00 17.05           C
ATOM   4680  O   SER C 177      2.715  43.951 -31.693  1.00 15.08           O
ATOM   4681  CB  SER C 177      1.773  42.173 -29.486  1.00 17.22           C
ATOM   4682  OG  SER C 177      1.774  40.863 -30.024  1.00 22.31           O
ATOM   4683  N   THR C 178      4.387  42.432 -31.677  1.00 15.73           N
ATOM   4684  CA  THR C 178      4.788  42.637 -33.058  1.00 16.79           C
ATOM   4685  C   THR C 178      4.714  41.293 -33.749  1.00 16.35           C
ATOM   4686  O   THR C 178      5.341  40.337 -33.289  1.00 17.92           O
ATOM   4687  CB  THR C 178      6.201  43.147 -33.168  1.00 17.73           C
ATOM   4688  OG1 THR C 178      6.333  44.381 -32.432  1.00 20.21           O
ATOM   4689  CG2 THR C 178      6.574  43.405 -34.639  1.00 17.04           C
ATOM   4690  N   LEU C 179      3.939  41.229 -34.840  1.00 17.28           N
ATOM   4691  CA  LEU C 179      3.859  40.028 -35.696  1.00 16.21           C
ATOM   4692  C   LEU C 179      4.705  40.284 -36.940  1.00 16.23           C
ATOM   4693  O   LEU C 179      4.405  41.208 -37.722  1.00 14.21           O
ATOM   4694  CB  LEU C 179      2.417  39.837 -36.132  1.00 17.16           C
ATOM   4695  CG  LEU C 179      2.104  38.837 -37.225  1.00 17.83           C
ATOM   4696  CD1 LEU C 179      2.462  37.464 -36.752  1.00 18.21           C
ATOM   4697  CD2 LEU C 179      0.626  38.935 -37.575  1.00 17.39           C
ATOM   4698  N   THR C 180      5.741  39.488 -37.137  1.00 16.35           N
ATOM   4699  CA  THR C 180      6.621  39.670 -38.273  1.00 15.71           C
ATOM   4700  C   THR C 180      6.464  38.601 -39.328  1.00 16.42           C
ATOM   4701  O   THR C 180      6.588  37.392 -39.055  1.00 16.33           O
ATOM   4702  CB  THR C 180      8.065  39.836 -37.826  1.00 16.79           C
ATOM   4703  OG1 THR C 180      8.172  41.112 -37.149  1.00 18.32           O
ATOM   4704  CG2 THR C 180      9.022  39.815 -39.052  1.00 18.01           C
ATOM   4705  N   LEU C 181      6.197  39.073 -40.538  1.00 14.61           N
ATOM   4706  CA  LEU C 181      6.069  38.233 -41.704  1.00 16.75           C
ATOM   4707  C   LEU C 181      7.037  38.648 -42.807  1.00 17.56           C
ATOM   4708  O   LEU C 181      7.608  39.755 -42.798  1.00 17.90           O
ATOM   4709  CB  LEU C 181      4.657  38.308 -42.239  1.00 17.35           C
ATOM   4710  CG  LEU C 181      3.549  37.944 -41.246  1.00 19.36           C
ATOM   4711  CD1 LEU C 181      2.221  38.374 -41.785  1.00 22.08           C
ATOM   4712  CD2 LEU C 181      3.575  36.426 -40.983  1.00 20.41           C
ATOM   4713  N   THR C 182      7.219  37.754 -43.765  1.00 16.14           N
ATOM   4714  CA  THR C 182      7.913  38.131 -44.987  1.00 15.40           C
ATOM   4715  C   THR C 182      6.906  38.917 -45.790  1.00 14.33           C
ATOM   4716  O   THR C 182      5.705  38.731 -45.641  1.00 14.63           O
ATOM   4717  CB  THR C 182      8.302  36.955 -45.817  1.00 15.82           C
ATOM   4718  OG1 THR C 182      7.128  36.253 -46.226  1.00 16.54           O
ATOM   4719  CG2 THR C 182      9.234  36.033 -45.068  1.00 17.29           C
ATOM   4720  N   LYS C 183      7.395  39.791 -46.665  1.00 14.91           N
ATOM   4721  CA  LYS C 183      6.501  40.525 -47.552  1.00 15.29           C
ATOM   4722  C   LYS C 183      5.668  39.559 -48.385  1.00 15.10           C
ATOM   4723  O   LYS C 183      4.479  39.794 -48.629  1.00 13.17           O
ATOM   4724  CB  LYS C 183      7.301  41.438 -48.478  1.00 14.61           C
ATOM   4725  CG  LYS C 183      6.440  42.194 -49.494  1.00 15.47           C
ATOM   4726  CD  LYS C 183      7.223  42.920 -50.568  1.00 15.31           C
ATOM   4727  CE  LYS C 183      6.282  43.229 -51.724  1.00 18.54           C
ATOM   4728  NZ  LYS C 183      6.916  43.889 -52.879  1.00 20.79           N
ATOM   4729  N   ASP C 184      6.266  38.466 -48.842  1.00 17.80           N
ATOM   4730  CA  ASP C 184      5.510  37.476 -49.619  1.00 19.41           C
ATOM   4731  C   ASP C 184      4.258  36.979 -48.857  1.00 21.31           C
ATOM   4732  O   ASP C 184      3.135  37.029 -49.378  1.00 23.67           O
ATOM   4733  CB  ASP C 184      6.420  36.306 -49.978  1.00 20.42           C
```

```
ATOM   4734  CG  ASP C 184    5.698  35.167 -50.607  1.00 22.70        C
ATOM   4735  OD1 ASP C 184    4.774  35.393 -51.423  1.00 27.83        O
ATOM   4736  OD2 ASP C 184    6.061  34.011 -50.266  1.00 24.36        O
ATOM   4737  N   GLU C 185    4.455  36.459 -47.653  1.00 20.83        N
ATOM   4738  CA  GLU C 185    3.332  36.025 -46.822  1.00 20.70        C
ATOM   4739  C   GLU C 185    2.332  37.118 -46.470  1.00 20.50        C
ATOM   4740  O   GLU C 185    1.119  36.869 -46.527  1.00 20.82        O
ATOM   4741  CB  GLU C 185    3.805  35.338 -45.540  1.00 20.44        C
ATOM   4742  CG  GLU C 185    2.634  34.759 -44.772  1.00 23.53        C
ATOM   4743  CD  GLU C 185    2.999  33.682 -43.757  1.00 25.57        C
ATOM   4744  OE1 GLU C 185    2.061  32.963 -43.348  1.00 30.12        O
ATOM   4745  OE2 GLU C 185    4.181  33.566 -43.353  1.00 30.96        O
ATOM   4746  N   TYR C 186    2.826  38.313 -46.120  1.00 18.39        N
ATOM   4747  CA  TYR C 186    1.976  39.438 -45.768  1.00 18.05        C
ATOM   4748  C   TYR C 186    1.020  39.776 -46.904  1.00 18.15        C
ATOM   4749  O   TYR C 186   -0.146  40.053 -46.670  1.00 16.86        O
ATOM   4750  CB  TYR C 186    2.809  40.671 -45.428  1.00 16.53        C
ATOM   4751  CG  TYR C 186    2.013  41.957 -45.210  1.00 16.23        C
ATOM   4752  CD1 TYR C 186    1.216  42.100 -44.077  1.00 16.62        C
ATOM   4753  CD2 TYR C 186    2.082  43.026 -46.099  1.00 13.72        C
ATOM   4754  CE1 TYR C 186    0.494  43.225 -43.845  1.00 15.97        C
ATOM   4755  CE2 TYR C 186    1.365  44.203 -45.864  1.00 15.34        C
ATOM   4756  CZ  TYR C 186    0.579  44.278 -44.717  1.00 16.41        C
ATOM   4757  OH  TYR C 186   -0.159  45.362 -44.393  1.00 16.72        O
ATOM   4758  N   GLU C 187    1.522  39.729 -48.137  1.00 18.02        N
ATOM   4759  CA  GLU C 187    0.711  40.073 -49.299  1.00 19.59        C
ATOM   4760  C   GLU C 187   -0.177  38.948 -49.809  1.00 20.14        C
ATOM   4761  O   GLU C 187   -0.877  39.137 -50.787  1.00 21.75        O
ATOM   4762  CB  GLU C 187    1.609  40.644 -50.394  1.00 19.33        C
ATOM   4763  CG  GLU C 187    2.353  41.849 -49.859  1.00 20.32        C
ATOM   4764  CD  GLU C 187    2.954  42.739 -50.917  1.00 22.46        C
ATOM   4765  OE1 GLU C 187    3.472  42.205 -51.934  1.00 22.32        O
ATOM   4766  OE2 GLU C 187    2.927  43.997 -50.694  1.00 26.72        O
ATOM   4767  N   ARG C 188   -0.170  37.793 -49.150  1.00 20.35        N
ATOM   4768  CA  ARG C 188   -1.111  36.731 -49.467  1.00 22.76        C
ATOM   4769  C   ARG C 188   -2.416  36.846 -48.677  1.00 22.90        C
ATOM   4770  O   ARG C 188   -3.340  36.072 -48.908  1.00 24.16        O
ATOM   4771  CB  ARG C 188   -0.489  35.358 -49.204  1.00 23.23        C
ATOM   4772  CG  ARG C 188    0.774  35.112 -49.963  1.00 27.00        C
ATOM   4773  CD  ARG C 188    1.128  33.616 -50.007  1.00 29.17        C
ATOM   4774  NE  ARG C 188    1.306  33.009 -48.681  1.00 32.40        N
ATOM   4775  CZ  ARG C 188    2.473  32.635 -48.130  1.00 34.37        C
ATOM   4776  NH1 ARG C 188    3.639  32.786 -48.760  1.00 35.16        N
ATOM   4777  NH2 ARG C 188    2.472  32.107 -46.912  1.00 35.13        N
ATOM   4778  N   HIS C 189   -2.488  37.804 -47.755  1.00 21.90        N
ATOM   4779  CA  HIS C 189   -3.672  38.005 -46.906  1.00 22.85        C
ATOM   4780  C   HIS C 189   -3.987  39.493 -46.793  1.00 22.67        C
ATOM   4781  O   HIS C 189   -3.103  40.315 -47.006  1.00 23.09        O
ATOM   4782  CB  HIS C 189   -3.396  37.436 -45.524  1.00 23.41        C
ATOM   4783  CG  HIS C 189   -3.039  35.993 -45.558  1.00 23.91        C
ATOM   4784  ND1 HIS C 189   -3.998  35.007 -45.580  1.00 27.21        N
ATOM   4785  CD2 HIS C 189   -1.844  35.364 -45.637  1.00 25.32        C
ATOM   4786  CE1 HIS C 189   -3.408  33.826 -45.647  1.00 26.12        C
ATOM   4787  NE2 HIS C 189   -2.101  34.014 -45.682  1.00 26.34        N
ATOM   4788  N   ASN C 190   -5.234  39.815 -46.436  1.00 22.23        N
ATOM   4789  CA  ASN C 190   -5.743  41.188 -46.389  1.00 22.87        C
ATOM   4790  C   ASN C 190   -6.063  41.659 -44.977  1.00 21.58        C
ATOM   4791  O   ASN C 190   -5.975  42.852 -44.671  1.00 20.72        O
ATOM   4792  CB  ASN C 190   -7.046  41.286 -47.193  1.00 25.56        C
ATOM   4793  CG  ASN C 190   -6.815  41.424 -48.676  1.00 30.68        C
ATOM   4794  OD1 ASN C 190   -7.307  42.387 -49.310  1.00 35.66        O
ATOM   4795  ND2 ASN C 190   -6.067  40.478 -49.256  1.00 32.08        N
ATOM   4796  N   SER C 191   -6.477  40.730 -44.118  1.00 20.43        N
ATOM   4797  CA  SER C 191   -7.061  41.116 -42.823  1.00 20.48        C
ATOM   4798  C   SER C 191   -6.146  40.791 -41.651  1.00 19.20        C
```

```
ATOM   4799  O    SER C 191     -5.834  39.637 -41.428  1.00 20.07           O
ATOM   4800  CB   SER C 191     -8.424  40.432 -42.619  1.00 20.88           C
ATOM   4801  OG   SER C 191     -9.357  40.842 -43.629  1.00 23.27           O
ATOM   4802  N    TYR C 192     -5.768  41.834 -40.910  1.00 17.36           N
ATOM   4803  CA   TYR C 192     -4.811  41.773 -39.809  1.00 18.72           C
ATOM   4804  C    TYR C 192     -5.450  42.373 -38.562  1.00 18.43           C
ATOM   4805  O    TYR C 192     -5.929  43.518 -38.581  1.00 19.74           O
ATOM   4806  CB   TYR C 192     -3.504  42.507 -40.183  1.00 16.83           C
ATOM   4807  CG   TYR C 192     -2.777  41.746 -41.241  1.00 17.54           C
ATOM   4808  CD1  TYR C 192     -1.999  40.617 -40.918  1.00 16.29           C
ATOM   4809  CD2  TYR C 192     -2.933  42.066 -42.577  1.00 17.96           C
ATOM   4810  CE1  TYR C 192     -1.345  39.901 -41.902  1.00 14.89           C
ATOM   4811  CE2  TYR C 192     -2.288  41.331 -43.568  1.00 15.32           C
ATOM   4812  CZ   TYR C 192     -1.503  40.255 -43.217  1.00 16.51           C
ATOM   4813  OH   TYR C 192     -0.920  39.524 -44.235  1.00 17.38           O
ATOM   4814  N    THR C 193     -5.473  41.575 -37.497  1.00 19.80           N
ATOM   4815  CA   THR C 193     -6.186  41.892 -36.277  1.00 21.03           C
ATOM   4816  C    THR C 193     -5.372  41.466 -35.070  1.00 21.05           C
ATOM   4817  O    THR C 193     -4.844  40.347 -35.033  1.00 19.64           O
ATOM   4818  CB   THR C 193     -7.577  41.172 -36.237  1.00 21.89           C
ATOM   4819  OG1  THR C 193     -8.322  41.515 -37.414  1.00 26.83           O
ATOM   4820  CG2  THR C 193     -8.387  41.582 -35.028  1.00 22.49           C
ATOM   4821  N    CYS C 194     -5.275  42.372 -34.094  1.00 21.18           N
ATOM   4822  CA   CYS C 194     -4.834  41.992 -32.741  1.00 23.08           C
ATOM   4823  C    CYS C 194     -5.990  42.190 -31.734  1.00 23.15           C
ATOM   4824  O    CYS C 194     -6.683  43.201 -31.765  1.00 22.25           O
ATOM   4825  CB   CYS C 194     -3.592  42.748 -32.316  1.00 22.69           C
ATOM   4826  SG   CYS C 194     -3.847  44.502 -32.093  1.00 25.92           S
ATOM   4827  N    GLU C 195     -6.193  41.182 -30.886  1.00 25.17           N
ATOM   4828  CA   GLU C 195     -7.269  41.163 -29.880  1.00 26.19           C
ATOM   4829  C    GLU C 195     -6.668  41.123 -28.480  1.00 26.71           C
ATOM   4830  O    GLU C 195     -5.989  40.157 -28.145  1.00 26.03           O
ATOM   4831  CB   GLU C 195     -8.121  39.914 -30.054  1.00 27.54           C
ATOM   4832  CG   GLU C 195     -8.835  39.751 -31.403  1.00 28.49           C
ATOM   4833  CD   GLU C 195     -9.511  38.379 -31.502  1.00 30.38           C
ATOM   4834  OE1  GLU C 195     -9.926  37.828 -30.461  1.00 33.44           O
ATOM   4835  OE2  GLU C 195     -9.611  37.837 -32.619  1.00 36.19           O
ATOM   4836  N    ALA C 196     -6.903  42.164 -27.678  1.00 25.56           N
ATOM   4837  CA   ALA C 196     -6.356  42.265 -26.318  1.00 26.72           C
ATOM   4838  C    ALA C 196     -7.399  41.827 -25.299  1.00 27.33           C
ATOM   4839  O    ALA C 196     -8.488  42.386 -25.277  1.00 27.19           O
ATOM   4840  CB   ALA C 196     -5.943  43.707 -26.027  1.00 26.24           C
ATOM   4841  N    THR C 197     -7.072  40.828 -24.482  1.00 28.26           N
ATOM   4842  CA   THR C 197     -7.948  40.408 -23.392  1.00 29.17           C
ATOM   4843  C    THR C 197     -7.343  40.762 -22.036  1.00 30.25           C
ATOM   4844  O    THR C 197     -6.245  40.292 -21.691  1.00 30.55           O
ATOM   4845  CB   THR C 197     -8.226  38.897 -23.413  1.00 29.33           C
ATOM   4846  OG1  THR C 197     -8.713  38.497 -24.699  1.00 30.99           O
ATOM   4847  CG2  THR C 197     -9.289  38.571 -22.409  1.00 30.10           C
ATOM   4848  N    HIS C 198     -8.087  41.574 -21.279  1.00 31.11           N
ATOM   4849  CA   HIS C 198     -7.703  42.059 -19.967  1.00 31.51           C
ATOM   4850  C    HIS C 198     -8.868  41.820 -18.996  1.00 32.80           C
ATOM   4851  O    HIS C 198     -9.991  41.527 -19.424  1.00 31.87           O
ATOM   4852  CB   HIS C 198     -7.377  43.552 -20.059  1.00 30.84           C
ATOM   4853  CG   HIS C 198     -6.626  44.075 -18.883  1.00 29.77           C
ATOM   4854  ND1  HIS C 198     -7.087  45.118 -18.117  1.00 30.47           N
ATOM   4855  CD2  HIS C 198     -5.453  43.690 -18.332  1.00 30.34           C
ATOM   4856  CE1  HIS C 198     -6.234  45.352 -17.139  1.00 29.73           C
ATOM   4857  NE2  HIS C 198     -5.235  44.496 -17.246  1.00 29.79           N
ATOM   4858  N    LYS C 199     -8.595  41.920 -17.697  1.00 34.79           N
ATOM   4859  CA   LYS C 199     -9.611  41.643 -16.660  1.00 36.16           C
ATOM   4860  C    LYS C 199    -10.700  42.692 -16.669  1.00 37.09           C
ATOM   4861  O    LYS C 199    -11.838  42.412 -16.303  1.00 39.31           O
ATOM   4862  CB   LYS C 199     -8.988  41.559 -15.253  1.00 36.57           C
ATOM   4863  CG   LYS C 199     -8.865  42.904 -14.515  1.00 38.75           C
```

```
ATOM   4864  CD  LYS C 199      -8.293  42.733 -13.118  1.00 38.68           C
ATOM   4865  CE  LYS C 199      -9.351  42.245 -12.124  1.00 40.58           C
ATOM   4866  NZ  LYS C 199      -8.734  41.838 -10.820  1.00 40.91           N
ATOM   4867  N   THR C 200     -10.348  43.899 -17.088  1.00 37.21           N
ATOM   4868  CA  THR C 200     -11.280  45.009 -17.120  1.00 37.64           C
ATOM   4869  C   THR C 200     -12.526  44.750 -17.965  1.00 38.79           C
ATOM   4870  O   THR C 200     -13.533  45.428 -17.794  1.00 39.22           O
ATOM   4871  CB  THR C 200     -10.598  46.268 -17.649  1.00 37.61           C
ATOM   4872  OG1 THR C 200      -9.732  45.915 -18.744  1.00 37.42           O
ATOM   4873  CG2 THR C 200      -9.801  46.932 -16.537  1.00 36.21           C
ATOM   4874  N   SER C 201     -12.448  43.800 -18.891  1.00 39.91           N
ATOM   4875  CA  SER C 201     -13.632  43.322 -19.582  1.00 40.28           C
ATOM   4876  C   SER C 201     -13.516  41.884 -19.995  1.00 40.67           C
ATOM   4877  O   SER C 201     -12.424  41.354 -20.194  1.00 41.57           O
ATOM   4878  CB  SER C 201     -13.942  44.144 -20.823  1.00 40.96           C
ATOM   4879  OG  SER C 201     -15.117  43.622 -21.442  1.00 42.25           O
ATOM   4880  N   THR C 202     -14.707  41.114 -20.127  1.00 41.01           N
ATOM   4881  CA  THR C 202     -14.781  39.748 -20.669  1.00 40.35           C
ATOM   4882  C   THR C 202     -14.547  39.763 -22.174  1.00 39.73           C
ATOM   4883  O   THR C 202     -13.991  38.817 -22.735  1.00 39.26           O
ATOM   4884  CB  THR C 202     -16.174  39.094 -20.423  1.00 40.93           C
ATOM   4885  OG1 THR C 202     -17.213  40.076 -20.608  1.00 42.87           O
ATOM   4886  CG2 THR C 202     -16.255  38.518 -19.028  1.00 41.15           C
ATOM   4887  N   SER C 203     -15.010  40.887 -22.785  1.00 38.67           N
ATOM   4888  CA  SER C 203     -14.878  40.941 -24.242  1.00 38.06           C
ATOM   4889  C   SER C 203     -13.637  41.729 -24.672  1.00 36.71           C
ATOM   4890  O   SER C 203     -13.415  42.843 -24.185  1.00 36.84           O
ATOM   4891  CB  SER C 203     -16.136  41.535 -24.875  1.00 39.13           C
ATOM   4892  OG  SER C 203     -17.121  40.521 -25.028  1.00 40.19           O
ATOM   4893  N   PRO C 204     -12.853  41.168 -25.612  1.00 35.30           N
ATOM   4894  CA  PRO C 204     -11.554  41.733 -25.978  1.00 34.30           C
ATOM   4895  C   PRO C 204     -11.613  43.120 -26.624  1.00 33.32           C
ATOM   4896  O   PRO C 204     -12.627  43.505 -27.207  1.00 32.96           O
ATOM   4897  CB  PRO C 204     -10.977  40.700 -26.960  1.00 34.71           C
ATOM   4898  CG  PRO C 204     -12.124  39.963 -27.481  1.00 35.13           C
ATOM   4899  CD  PRO C 204     -13.142  39.941 -26.382  1.00 35.37           C
ATOM   4900  N   ILE C 205     -10.522  43.870 -26.484  1.00 31.85           N
ATOM   4901  CA  ILE C 205     -10.373  45.130 -27.184  1.00 31.11           C
ATOM   4902  C   ILE C 205      -9.658  44.775 -28.471  1.00 29.95           C
ATOM   4903  O   ILE C 205      -8.573  44.204 -28.439  1.00 28.95           O
ATOM   4904  CB  ILE C 205      -9.555  46.151 -26.397  1.00 31.18           C
ATOM   4905  CG1 ILE C 205     -10.221  46.444 -25.051  1.00 30.91           C
ATOM   4906  CG2 ILE C 205      -9.442  47.433 -27.179  1.00 31.32           C
ATOM   4907  CD1 ILE C 205      -9.455  47.404 -24.199  1.00 31.98           C
ATOM   4908  N   VAL C 206     -10.277  45.123 -29.592  1.00 28.96           N
ATOM   4909  CA  VAL C 206      -9.825  44.681 -30.891  1.00 28.18           C
ATOM   4910  C   VAL C 206      -9.495  45.855 -31.811  1.00 27.14           C
ATOM   4911  O   VAL C 206     -10.210  46.876 -31.845  1.00 27.73           O
ATOM   4912  CB  VAL C 206     -10.880  43.786 -31.511  1.00 28.32           C
ATOM   4913  CG1 VAL C 206     -10.399  43.201 -32.825  1.00 30.09           C
ATOM   4914  CG2 VAL C 206     -11.255  42.667 -30.519  1.00 28.88           C
ATOM   4915  N   LYS C 207      -8.387  45.708 -32.540  1.00 25.85           N
ATOM   4916  CA  LYS C 207      -7.972  46.654 -33.562  1.00 25.37           C
ATOM   4917  C   LYS C 207      -7.589  45.868 -34.796  1.00 24.09           C
ATOM   4918  O   LYS C 207      -6.952  44.825 -34.697  1.00 21.78           O
ATOM   4919  CB  LYS C 207      -6.762  47.448 -33.100  1.00 25.84           C
ATOM   4920  CG  LYS C 207      -7.034  48.365 -31.972  1.00 27.96           C
ATOM   4921  CD  LYS C 207      -7.903  49.543 -32.388  1.00 29.41           C
ATOM   4922  CE  LYS C 207      -8.754  50.015 -31.218  1.00 31.67           C
ATOM   4923  NZ  LYS C 207      -8.788  51.481 -31.180  1.00 32.07           N
ATOM   4924  N   SER C 208      -8.009  46.345 -35.956  1.00 23.56           N
ATOM   4925  CA  SER C 208      -7.760  45.623 -37.206  1.00 24.28           C
ATOM   4926  C   SER C 208      -7.481  46.599 -38.305  1.00 22.71           C
ATOM   4927  O   SER C 208      -7.764  47.787 -38.191  1.00 22.23           O
ATOM   4928  CB  SER C 208      -8.974  44.792 -37.622  1.00 24.50           C
```

| ATOM | 4929 | OG | SER | C | 208 | -9.670 | 44.336 | -36.492 | 1.00 | 28.10 | O |
|------|------|------|------|---|-----|--------|--------|---------|------|-------|---|
| ATOM | 4930 | N | PHE | C | 209 | -6.922 | 46.099 | -39.389 | 1.00 | 22.42 | N |
| ATOM | 4931 | CA | PHE | C | 209 | -6.916 | 46.877 | -40.631 | 1.00 | 22.07 | C |
| ATOM | 4932 | C | PHE | C | 209 | -6.910 | 45.934 | -41.795 | 1.00 | 21.26 | C |
| ATOM | 4933 | O | PHE | C | 209 | -6.555 | 44.759 | -41.664 | 1.00 | 20.40 | O |
| ATOM | 4934 | CB | PHE | C | 209 | -5.699 | 47.808 | -40.719 | 1.00 | 21.73 | C |
| ATOM | 4935 | CG | PHE | C | 209 | -4.395 | 47.070 | -40.828 | 1.00 | 21.23 | C |
| ATOM | 4936 | CD1 | PHE | C | 209 | -3.833 | 46.796 | -42.067 | 1.00 | 20.87 | C |
| ATOM | 4937 | CD2 | PHE | C | 209 | -3.748 | 46.652 | -39.691 | 1.00 | 22.04 | C |
| ATOM | 4938 | CE1 | PHE | C | 209 | -2.691 | 46.098 | -42.168 | 1.00 | 21.99 | C |
| ATOM | 4939 | CE2 | PHE | C | 209 | -2.601 | 45.946 | -39.772 | 1.00 | 22.08 | C |
| ATOM | 4940 | CZ | PHE | C | 209 | -2.055 | 45.663 | -41.009 | 1.00 | 23.04 | C |
| ATOM | 4941 | N | ASN | C | 210 | -7.272 | 46.478 | -42.948 | 1.00 | 20.51 | N |
| ATOM | 4942 | CA | ASN | C | 210 | -7.205 | 45.734 | -44.182 | 1.00 | 20.62 | C |
| ATOM | 4943 | C | ASN | C | 210 | -6.096 | 46.280 | -45.083 | 1.00 | 19.88 | C |
| ATOM | 4944 | O | ASN | C | 210 | -5.978 | 47.502 | -45.304 | 1.00 | 16.91 | O |
| ATOM | 4945 | CB | ASN | C | 210 | -8.579 | 45.707 | -44.868 | 1.00 | 20.67 | C |
| ATOM | 4946 | CG | ASN | C | 210 | -8.608 | 44.810 | -46.087 | 1.00 | 22.10 | C |
| ATOM | 4947 | OD1 | ASN | C | 210 | -7.964 | 45.115 | -47.104 | 1.00 | 22.13 | O |
| ATOM | 4948 | ND2 | ASN | C | 210 | -9.370 | 43.691 | -46.014 | 1.00 | 22.61 | N |
| ATOM | 4949 | N | ARG | C | 211 | -5.266 | 45.359 | -45.556 | 1.00 | 20.93 | N |
| ATOM | 4950 | CA | ARG | C | 211 | -4.099 | 45.660 | -46.372 | 1.00 | 22.05 | C |
| ATOM | 4951 | C | ARG | C | 211 | -4.435 | 46.487 | -47.603 | 1.00 | 23.19 | C |
| ATOM | 4952 | O | ARG | C | 211 | -3.661 | 47.345 | -47.994 | 1.00 | 23.34 | O |
| ATOM | 4953 | CB | ARG | C | 211 | -3.418 | 44.344 | -46.785 | 1.00 | 21.73 | C |
| ATOM | 4954 | CG | ARG | C | 211 | -2.027 | 44.533 | -47.363 | 1.00 | 22.26 | C |
| ATOM | 4955 | CD | ARG | C | 211 | -1.354 | 43.205 | -47.633 | 1.00 | 21.37 | C |
| ATOM | 4956 | NE | ARG | C | 211 | -2.166 | 42.328 | -48.443 | 1.00 | 18.84 | N |
| ATOM | 4957 | CZ | ARG | C | 211 | -2.292 | 42.374 | -49.757 | 1.00 | 21.14 | C |
| ATOM | 4958 | NH1 | ARG | C | 211 | -1.677 | 43.291 | -50.489 | 1.00 | 22.43 | N |
| ATOM | 4959 | NH2 | ARG | C | 211 | -3.075 | 41.476 | -50.353 | 1.00 | 23.89 | N |
| ATOM | 4960 | N | ASN | C | 212 | -5.601 | 46.241 | -48.192 | 1.00 | 23.85 | N |
| ATOM | 4961 | CA | ASN | C | 212 | -6.032 | 46.923 | -49.417 | 1.00 | 25.25 | C |
| ATOM | 4962 | C | ASN | C | 212 | -6.886 | 48.160 | -49.216 | 1.00 | 25.42 | C |
| ATOM | 4963 | O | ASN | C | 212 | -7.402 | 48.696 | -50.190 | 1.00 | 26.85 | O |
| ATOM | 4964 | CB | ASN | C | 212 | -6.823 | 45.943 | -50.292 | 1.00 | 25.73 | C |
| ATOM | 4965 | CG | ASN | C | 212 | -5.934 | 44.927 | -50.968 | 1.00 | 26.71 | C |
| ATOM | 4966 | OD1 | ASN | C | 212 | -5.026 | 45.289 | -51.710 | 1.00 | 26.40 | O |
| ATOM | 4967 | ND2 | ASN | C | 212 | -6.214 | 43.656 | -50.747 | 1.00 | 28.60 | N |
| ATOM | 4968 | N | GLU | C | 213 | -7.079 | 48.595 | -47.977 | 1.00 | 25.05 | N |
| ATOM | 4969 | CA | GLU | C | 213 | -7.962 | 49.725 | -47.727 | 1.00 | 25.98 | C |
| ATOM | 4970 | C | GLU | C | 213 | -7.142 | 51.002 | -47.633 | 1.00 | 28.31 | C |
| ATOM | 4971 | O | GLU | C | 213 | -6.025 | 50.901 | -47.134 | 1.00 | 30.04 | O |
| ATOM | 4972 | CB | GLU | C | 213 | -8.760 | 49.488 | -46.459 | 1.00 | 24.70 | C |
| ATOM | 4973 | CG | GLU | C | 213 | -9.817 | 50.516 | -46.211 | 1.00 | 26.37 | C |
| ATOM | 4974 | CD | GLU | C | 213 | -10.788 | 50.087 | -45.157 | 1.00 | 26.99 | C |
| ATOM | 4975 | OE1 | GLU | C | 213 | -10.694 | 48.927 | -44.687 | 1.00 | 26.49 | O |
| ATOM | 4976 | OE2 | GLU | C | 213 | -11.646 | 50.917 | -44.808 | 1.00 | 28.12 | O |
| TER | 4977 | | GLU | C | 213 | | | | | | |
| ATOM | 4978 | N | GLU | D | 1 | 27.858 | 69.640 | -8.202 | 1.00 | 32.07 | N |
| ATOM | 4979 | CA | GLU | D | 1 | 27.268 | 68.261 | -8.207 | 1.00 | 31.52 | C |
| ATOM | 4980 | C | GLU | D | 1 | 28.320 | 67.247 | -8.625 | 1.00 | 29.49 | C |
| ATOM | 4981 | O | GLU | D | 1 | 29.157 | 67.519 | -9.492 | 1.00 | 28.62 | O |
| ATOM | 4982 | CB | GLU | D | 1 | 26.106 | 68.187 | -9.192 | 1.00 | 32.99 | C |
| ATOM | 4983 | CG | GLU | D | 1 | 25.427 | 66.805 | -9.277 | 1.00 | 34.18 | C |
| ATOM | 4984 | CD | GLU | D | 1 | 24.998 | 66.445 | -10.700 | 1.00 | 36.36 | C |
| ATOM | 4985 | OE1 | GLU | D | 1 | 24.745 | 67.379 | -11.509 | 1.00 | 39.54 | O |
| ATOM | 4986 | OE2 | GLU | D | 1 | 24.916 | 65.224 | -11.003 | 1.00 | 40.05 | O |
| ATOM | 4987 | N | VAL | D | 2 | 28.266 | 66.072 | -8.025 | 1.00 | 27.61 | N |
| ATOM | 4988 | CA | VAL | D | 2 | 29.140 | 64.992 | -8.465 | 1.00 | 27.12 | C |
| ATOM | 4989 | C | VAL | D | 2 | 28.597 | 64.478 | -9.815 | 1.00 | 26.31 | C |
| ATOM | 4990 | O | VAL | D | 2 | 27.395 | 64.234 | -9.942 | 1.00 | 26.31 | O |
| ATOM | 4991 | CB | VAL | D | 2 | 29.230 | 63.926 | -7.370 | 1.00 | 26.19 | C |
| ATOM | 4992 | CG1 | VAL | D | 2 | 30.104 | 62.757 | -7.793 | 1.00 | 25.74 | C |
| ATOM | 4993 | CG2 | VAL | D | 2 | 29.739 | 64.585 | -6.070 | 1.00 | 27.00 | C |

| ATOM | 4994 | N | LYS | D | 3 | 29.467 | 64.366 | -10.799 | 1.00 | 25.19 | N |
|------|------|------|-----|---|----|--------|--------|---------|------|-------|---|
| ATOM | 4995 | CA | LYS | D | 3 | 29.117 | 63.736 | -12.060 | 1.00 | 25.42 | C |
| ATOM | 4996 | C | LYS | D | 3 | 30.114 | 62.612 | -12.342 | 1.00 | 23.44 | C |
| ATOM | 4997 | O | LYS | D | 3 | 31.296 | 62.811 | -12.242 | 1.00 | 22.49 | O |
| ATOM | 4998 | CB | LYS | D | 3 | 29.081 | 64.739 | -13.212 | 1.00 | 26.38 | C |
| ATOM | 4999 | CG | LYS | D | 3 | 28.574 | 64.101 | -14.506 | 1.00 | 27.61 | C |
| ATOM | 5000 | CD | LYS | D | 3 | 28.133 | 65.105 | -15.565 | 1.00 | 29.92 | C |
| ATOM | 5001 | CE | LYS | D | 3 | 27.670 | 64.350 | -16.835 | 1.00 | 30.13 | C |
| ATOM | 5002 | NZ | LYS | D | 3 | 28.808 | 63.698 | -17.536 | 1.00 | 33.20 | N |
| ATOM | 5003 | N | VAL | D | 4 | 29.606 | 61.413 | -12.634 | 1.00 | 22.63 | N |
| ATOM | 5004 | CA | VAL | D | 4 | 30.448 | 60.251 | -12.910 | 1.00 | 22.21 | C |
| ATOM | 5005 | C | VAL | D | 4 | 30.020 | 59.742 | -14.269 | 1.00 | 21.38 | C |
| ATOM | 5006 | O | VAL | D | 4 | 28.836 | 59.477 | -14.472 | 1.00 | 21.05 | O |
| ATOM | 5007 | CB | VAL | D | 4 | 30.255 | 59.140 | -11.860 | 1.00 | 21.59 | C |
| ATOM | 5008 | CG1 | VAL | D | 4 | 31.024 | 57.886 | -12.256 | 1.00 | 21.17 | C |
| ATOM | 5009 | CG2 | VAL | D | 4 | 30.683 | 59.592 | -10.467 | 1.00 | 22.08 | C |
| ATOM | 5010 | N | GLU | D | 5 | 30.973 | 59.636 | -15.202 | 1.00 | 22.42 | N |
| ATOM | 5011 | CA | GLU | D | 5 | 30.672 | 59.332 | -16.585 | 1.00 | 22.44 | C |
| ATOM | 5012 | C | GLU | D | 5 | 31.590 | 58.314 | -17.239 | 1.00 | 20.85 | C |
| ATOM | 5013 | O | GLU | D | 5 | 32.739 | 58.605 | -17.534 | 1.00 | 19.75 | O |
| ATOM | 5014 | CB | GLU | D | 5 | 30.690 | 60.585 | -17.436 | 1.00 | 23.47 | C |
| ATOM | 5015 | CG | GLU | D | 5 | 30.277 | 60.296 | -18.861 | 1.00 | 25.10 | C |
| ATOM | 5016 | CD | GLU | D | 5 | 29.899 | 61.551 | -19.608 | 1.00 | 27.21 | C |
| ATOM | 5017 | OE1 | GLU | D | 5 | 28.681 | 61.818 | -19.714 | 1.00 | 32.85 | O |
| ATOM | 5018 | OE2 | GLU | D | 5 | 30.821 | 62.265 | -20.079 | 1.00 | 33.65 | O |
| ATOM | 5019 | N | GLU | D | 6 | 31.030 | 57.147 | -17.527 | 1.00 | 20.54 | N |
| ATOM | 5020 | CA | GLU | D | 6 | 31.767 | 56.043 | -18.156 | 1.00 | 19.01 | C |
| ATOM | 5021 | C | GLU | D | 6 | 31.737 | 56.092 | -19.669 | 1.00 | 17.47 | C |
| ATOM | 5022 | O | GLU | D | 6 | 30.756 | 56.520 | -20.276 | 1.00 | 17.39 | O |
| ATOM | 5023 | CB | GLU | D | 6 | 31.186 | 54.678 | -17.740 | 1.00 | 18.72 | C |
| ATOM | 5024 | CG | GLU | D | 6 | 31.136 | 54.425 | -16.242 | 1.00 | 17.47 | C |
| ATOM | 5025 | CD | GLU | D | 6 | 29.914 | 55.035 | -15.583 | 1.00 | 16.50 | C |
| ATOM | 5026 | OE1 | GLU | D | 6 | 29.239 | 55.907 | -16.167 | 1.00 | 15.15 | O |
| ATOM | 5027 | OE2 | GLU | D | 6 | 29.603 | 54.624 | -14.474 | 1.00 | 19.91 | O |
| ATOM | 5028 | N | SER | D | 7 | 32.790 | 55.539 | -20.271 | 1.00 | 17.15 | N |
| ATOM | 5029 | CA | SER | D | 7 | 32.917 | 55.374 | -21.702 | 1.00 | 17.14 | C |
| ATOM | 5030 | C | SER | D | 7 | 33.731 | 54.094 | -22.004 | 1.00 | 17.02 | C |
| ATOM | 5031 | O | SER | D | 7 | 34.340 | 53.520 | -21.106 | 1.00 | 16.35 | O |
| ATOM | 5032 | CB | SER | D | 7 | 33.597 | 56.610 | -22.298 | 1.00 | 18.27 | C |
| ATOM | 5033 | OG | SER | D | 7 | 34.866 | 56.807 | -21.727 | 1.00 | 19.91 | O |
| ATOM | 5034 | N | GLY | D | 8 | 33.738 | 53.672 | -23.263 | 1.00 | 17.01 | N |
| ATOM | 5035 | CA | GLY | D | 8 | 34.587 | 52.574 | -23.692 | 1.00 | 14.09 | C |
| ATOM | 5036 | C | GLY | D | 8 | 33.938 | 51.253 | -23.974 | 1.00 | 14.91 | C |
| ATOM | 5037 | O | GLY | D | 8 | 34.633 | 50.268 | -24.295 | 1.00 | 15.24 | O |
| ATOM | 5038 | N | GLY | D | 9 | 32.630 | 51.152 | -23.842 | 1.00 | 15.24 | N |
| ATOM | 5039 | CA | GLY | D | 9 | 31.996 | 49.839 | -24.130 | 1.00 | 14.28 | C |
| ATOM | 5040 | C | GLY | D | 9 | 31.875 | 49.468 | -25.585 | 1.00 | 14.29 | C |
| ATOM | 5041 | O | GLY | D | 9 | 32.295 | 50.191 | -26.494 | 1.00 | 13.76 | O |
| ATOM | 5042 | N | GLY | D | 10 | 31.303 | 48.294 | -25.843 | 1.00 | 10.56 | N |
| ATOM | 5043 | CA | GLY | D | 10 | 31.170 | 47.822 | -27.190 | 1.00 | 9.96 | C |
| ATOM | 5044 | C | GLY | D | 10 | 31.115 | 46.316 | -27.078 | 1.00 | 9.66 | C |
| ATOM | 5045 | O | GLY | D | 10 | 30.741 | 45.835 | -26.037 | 1.00 | 8.15 | O |
| ATOM | 5046 | N | LEU | D | 11 | 31.531 | 45.643 | -28.129 | 1.00 | 12.50 | N |
| ATOM | 5047 | CA | LEU | D | 11 | 31.499 | 44.188 | -28.217 | 1.00 | 10.90 | C |
| ATOM | 5048 | C | LEU | D | 11 | 32.892 | 43.654 | -28.497 | 1.00 | 11.12 | C |
| ATOM | 5049 | O | LEU | D | 11 | 33.716 | 44.253 | -29.215 | 1.00 | 12.18 | O |
| ATOM | 5050 | CB | LEU | D | 11 | 30.526 | 43.775 | -29.319 | 1.00 | 11.12 | C |
| ATOM | 5051 | CG | LEU | D | 11 | 30.382 | 42.270 | -29.586 | 1.00 | 12.07 | C |
| ATOM | 5052 | CD1 | LEU | D | 11 | 28.994 | 41.995 | -30.076 | 1.00 | 10.69 | C |
| ATOM | 5053 | CD2 | LEU | D | 11 | 31.369 | 41.799 | -30.600 | 1.00 | 12.39 | C |
| ATOM | 5054 | N | VAL | D | 12 | 33.199 | 42.495 | -27.915 | 1.00 | 10.21 | N |
| ATOM | 5055 | CA | VAL | D | 12 | 34.391 | 41.824 | -28.278 | 1.00 | 9.24 | C |
| ATOM | 5056 | C | VAL | D | 12 | 34.087 | 40.342 | -28.118 | 1.00 | 9.01 | C |
| ATOM | 5057 | O | VAL | D | 12 | 33.129 | 39.955 | -27.484 | 1.00 | 8.79 | O |
| ATOM | 5058 | CB | VAL | D | 12 | 35.629 | 42.261 | -27.485 | 1.00 | 9.29 | C |

| ATOM | 5059 | CG1 | VAL | D | 12 | 35.524 | 42.010 | -26.021 | 1.00 | 10.99 | C |
|------|------|-----|-----|---|----|--------|--------|---------|------|-------|---|
| ATOM | 5060 | CG2 | VAL | D | 12 | 36.958 | 41.673 | -28.106 | 1.00 | 10.24 | C |
| ATOM | 5061 | N | GLN | D | 13 | 34.910 | 39.571 | -28.746 | 1.00 | 9.63 | N |
| ATOM | 5062 | CA | GLN | D | 13 | 34.760 | 38.097 | -28.728 | 1.00 | 9.08 | C |
| ATOM | 5063 | C | GLN | D | 13 | 35.363 | 37.460 | -27.492 | 1.00 | 11.32 | C |
| ATOM | 5064 | O | GLN | D | 13 | 36.252 | 37.983 | -26.871 | 1.00 | 9.93 | O |
| ATOM | 5065 | CB | GLN | D | 13 | 35.476 | 37.562 | -29.931 | 1.00 | 10.27 | C |
| ATOM | 5066 | CG | GLN | D | 13 | 34.908 | 38.002 | -31.253 | 1.00 | 9.95 | C |
| ATOM | 5067 | CD | GLN | D | 13 | 35.272 | 39.422 | -31.613 | 1.00 | 11.52 | C |
| ATOM | 5068 | OE1 | GLN | D | 13 | 36.356 | 39.914 | -31.270 | 1.00 | 11.60 | O |
| ATOM | 5069 | NE2 | GLN | D | 13 | 34.374 | 40.071 | -32.337 | 1.00 | 11.82 | N |
| ATOM | 5070 | N | PRO | D | 14 | 34.906 | 36.244 | -27.135 | 1.00 | 11.44 | N |
| ATOM | 5071 | CA | PRO | D | 14 | 35.576 | 35.538 | -26.033 | 1.00 | 10.84 | C |
| ATOM | 5072 | C | PRO | D | 14 | 37.068 | 35.353 | -26.230 | 1.00 | 9.30 | C |
| ATOM | 5073 | O | PRO | D | 14 | 37.497 | 34.980 | -27.327 | 1.00 | 13.69 | O |
| ATOM | 5074 | CB | PRO | D | 14 | 34.853 | 34.179 | -26.017 | 1.00 | 11.56 | C |
| ATOM | 5075 | CG | PRO | D | 14 | 33.481 | 34.494 | -26.544 | 1.00 | 11.28 | C |
| ATOM | 5076 | CD | PRO | D | 14 | 33.730 | 35.519 | -27.639 | 1.00 | 12.57 | C |
| ATOM | 5077 | N | GLY | D | 15 | 37.871 | 35.651 | -25.202 | 1.00 | 10.46 | N |
| ATOM | 5078 | CA | GLY | D | 15 | 39.336 | 35.605 | -25.270 | 1.00 | 10.56 | C |
| ATOM | 5079 | C | GLY | D | 15 | 39.925 | 37.008 | -25.431 | 1.00 | 12.12 | C |
| ATOM | 5080 | O | GLY | D | 15 | 41.085 | 37.299 | -25.094 | 1.00 | 14.78 | O |
| ATOM | 5081 | N | GLY | D | 16 | 39.076 | 37.902 | -25.884 | 1.00 | 13.21 | N |
| ATOM | 5082 | CA | GLY | D | 16 | 39.491 | 39.250 | -26.198 | 1.00 | 12.02 | C |
| ATOM | 5083 | C | GLY | D | 16 | 39.462 | 40.184 | -25.031 | 1.00 | 14.06 | C |
| ATOM | 5084 | O | GLY | D | 16 | 39.260 | 39.800 | -23.871 | 1.00 | 11.36 | O |
| ATOM | 5085 | N | SER | D | 17 | 39.695 | 41.461 | -25.343 | 1.00 | 12.78 | N |
| ATOM | 5086 | CA | SER | D | 17 | 40.016 | 42.446 | -24.330 | 1.00 | 14.00 | C |
| ATOM | 5087 | C | SER | D | 17 | 39.181 | 43.684 | -24.604 | 1.00 | 12.25 | C |
| ATOM | 5088 | O | SER | D | 17 | 38.663 | 43.829 | -25.692 | 1.00 | 11.65 | O |
| ATOM | 5089 | CB | SER | D | 17 | 41.487 | 42.868 | -24.391 | 1.00 | 14.31 | C |
| ATOM | 5090 | OG | SER | D | 17 | 42.312 | 41.839 | -23.861 | 1.00 | 15.52 | O |
| ATOM | 5091 | N | MET | D | 18 | 39.008 | 44.491 | -23.561 | 1.00 | 14.68 | N |
| ATOM | 5092 | CA | MET | D | 18 | 38.343 | 45.787 | -23.629 | 1.00 | 16.72 | C |
| ATOM | 5093 | C | MET | D | 18 | 38.948 | 46.756 | -22.612 | 1.00 | 15.41 | C |
| ATOM | 5094 | O | MET | D | 18 | 39.481 | 46.334 | -21.620 | 1.00 | 15.06 | O |
| ATOM | 5095 | CB | MET | D | 18 | 36.886 | 45.604 | -23.317 | 1.00 | 20.03 | C |
| ATOM | 5096 | CG | MET | D | 18 | 36.025 | 46.446 | -24.129 | 1.00 | 24.74 | C |
| ATOM | 5097 | SD | MET | D | 18 | 35.481 | 45.665 | -25.662 | 1.00 | 25.44 | S |
| ATOM | 5098 | CE | MET | D | 18 | 33.996 | 46.622 | -25.649 | 1.00 | 24.11 | C |
| ATOM | 5099 | N | LYS | D | 19 | 38.877 | 48.068 | -22.854 | 1.00 | 15.97 | N |
| ATOM | 5100 | CA | LYS | D | 19 | 39.359 | 49.024 | -21.855 | 1.00 | 16.32 | C |
| ATOM | 5101 | C | LYS | D | 19 | 38.281 | 50.058 | -21.715 | 1.00 | 16.28 | C |
| ATOM | 5102 | O | LYS | D | 19 | 37.894 | 50.676 | -22.693 | 1.00 | 15.50 | O |
| ATOM | 5103 | CB | LYS | D | 19 | 40.727 | 49.674 | -22.192 | 1.00 | 18.30 | C |
| ATOM | 5104 | CG | LYS | D | 19 | 41.212 | 50.601 | -21.038 | 1.00 | 18.60 | C |
| ATOM | 5105 | CD | LYS | D | 19 | 42.656 | 51.065 | -21.150 | 1.00 | 21.56 | C |
| ATOM | 5106 | CE | LYS | D | 19 | 43.245 | 51.544 | -19.790 | 1.00 | 22.52 | C |
| ATOM | 5107 | NZ | LYS | D | 19 | 44.666 | 52.018 | -19.951 | 1.00 | 25.34 | N |
| ATOM | 5108 | N | ILE | D | 20 | 37.738 | 50.165 | -20.507 | 1.00 | 14.21 | N |
| ATOM | 5109 | CA | ILE | D | 20 | 36.712 | 51.149 | -20.226 | 1.00 | 14.65 | C |
| ATOM | 5110 | C | ILE | D | 20 | 37.229 | 52.174 | -19.218 | 1.00 | 13.86 | C |
| ATOM | 5111 | O | ILE | D | 20 | 38.250 | 51.990 | -18.580 | 1.00 | 14.15 | O |
| ATOM | 5112 | CB | ILE | D | 20 | 35.388 | 50.479 | -19.739 | 1.00 | 13.06 | C |
| ATOM | 5113 | CG1 | ILE | D | 20 | 35.557 | 49.663 | -18.449 | 1.00 | 12.43 | C |
| ATOM | 5114 | CG2 | ILE | D | 20 | 34.784 | 49.561 | -20.871 | 1.00 | 12.79 | C |
| ATOM | 5115 | CD1 | ILE | D | 20 | 34.236 | 49.271 | -17.827 | 1.00 | 12.82 | C |
| ATOM | 5116 | N | SER | D | 21 | 36.535 | 53.302 | -19.081 | 1.00 | 16.20 | N |
| ATOM | 5117 | CA | SER | D | 21 | 37.052 | 54.327 | -18.189 | 1.00 | 15.38 | C |
| ATOM | 5118 | C | SER | D | 21 | 35.905 | 55.207 | -17.723 | 1.00 | 14.34 | C |
| ATOM | 5119 | O | SER | D | 21 | 34.846 | 55.190 | -18.334 | 1.00 | 13.66 | O |
| ATOM | 5120 | CB | SER | D | 21 | 38.136 | 55.173 | -18.895 | 1.00 | 16.48 | C |
| ATOM | 5121 | OG | SER | D | 21 | 37.563 | 56.023 | -19.862 | 1.00 | 18.73 | O |
| ATOM | 5122 | N | CYS | D | 22 | 36.131 | 55.954 | -16.644 | 1.00 | 14.94 | N |
| ATOM | 5123 | CA | CYS | D | 22 | 35.198 | 57.020 | -16.272 | 1.00 | 16.02 | C |

| ATOM | 5124 | C | CYS | D | 22 | 35.888 | 58.255 | -15.769 | 1.00 | 13.59 | C |
|------|------|-----|-----|---|----|--------|--------|---------|------|-------|---|
| ATOM | 5125 | O | CYS | D | 22 | 36.921 | 58.199 | -15.155 | 1.00 | 13.22 | O |
| ATOM | 5126 | CB | CYS | D | 22 | 34.163 | 56.583 | -15.224 | 1.00 | 17.91 | C |
| ATOM | 5127 | SG | CYS | D | 22 | 34.759 | 56.083 | -13.647 | 1.00 | 22.82 | S |
| ATOM | 5128 | N | VAL | D | 23 | 35.237 | 59.374 | -16.014 | 1.00 | 16.25 | N |
| ATOM | 5129 | CA | VAL | D | 23 | 35.722 | 60.648 | -15.514 | 1.00 | 16.36 | C |
| ATOM | 5130 | C | VAL | D | 23 | 34.720 | 61.148 | -14.469 | 1.00 | 17.19 | C |
| ATOM | 5131 | O | VAL | D | 23 | 33.508 | 60.992 | -14.616 | 1.00 | 18.21 | O |
| ATOM | 5132 | CB | VAL | D | 23 | 35.939 | 61.592 | -16.665 | 1.00 | 16.53 | C |
| ATOM | 5133 | CG1 | VAL | D | 23 | 34.708 | 61.711 | -17.524 | 1.00 | 18.66 | C |
| ATOM | 5134 | CG2 | VAL | D | 23 | 36.429 | 63.005 | -16.153 | 1.00 | 17.32 | C |
| ATOM | 5135 | N | VAL | D | 24 | 35.251 | 61.676 | -13.381 | 1.00 | 18.46 | N |
| ATOM | 5136 | CA | VAL | D | 24 | 34.465 | 62.140 | -12.272 | 1.00 | 19.61 | C |
| ATOM | 5137 | C | VAL | D | 24 | 34.727 | 63.642 | -12.140 | 1.00 | 21.37 | C |
| ATOM | 5138 | O | VAL | D | 24 | 35.872 | 64.065 | -12.165 | 1.00 | 22.21 | O |
| ATOM | 5139 | CB | VAL | D | 24 | 34.882 | 61.455 | -10.975 | 1.00 | 18.94 | C |
| ATOM | 5140 | CG1 | VAL | D | 24 | 34.075 | 61.940 | -9.794 | 1.00 | 19.62 | C |
| ATOM | 5141 | CG2 | VAL | D | 24 | 34.728 | 59.948 | -11.087 | 1.00 | 20.65 | C |
| ATOM | 5142 | N | SER | D | 25 | 33.662 | 64.423 | -12.016 | 1.00 | 23.28 | N |
| ATOM | 5143 | CA | SER | D | 25 | 33.804 | 65.850 | -11.699 | 1.00 | 22.92 | C |
| ATOM | 5144 | C | SER | D | 25 | 33.027 | 66.205 | -10.430 | 1.00 | 22.84 | C |
| ATOM | 5145 | O | SER | D | 25 | 32.235 | 65.407 | -9.928 | 1.00 | 22.34 | O |
| ATOM | 5146 | CB | SER | D | 25 | 33.416 | 66.718 | -12.898 | 1.00 | 23.22 | C |
| ATOM | 5147 | OG | SER | D | 25 | 32.064 | 66.561 | -13.292 | 1.00 | 27.47 | O |
| ATOM | 5148 | N | GLY | D | 26 | 33.293 | 67.380 | -9.871 | 1.00 | 21.89 | N |
| ATOM | 5149 | CA | GLY | D | 26 | 32.550 | 67.821 | -8.689 | 1.00 | 21.26 | C |
| ATOM | 5150 | C | GLY | D | 26 | 33.030 | 67.376 | -7.328 | 1.00 | 20.57 | C |
| ATOM | 5151 | O | GLY | D | 26 | 32.442 | 67.751 | -6.319 | 1.00 | 19.53 | O |
| ATOM | 5152 | N | LEU | D | 27 | 34.091 | 66.564 | -7.281 | 1.00 | 21.38 | N |
| ATOM | 5153 | CA | LEU | D | 27 | 34.798 | 66.256 | -6.046 | 1.00 | 21.39 | C |
| ATOM | 5154 | C | LEU | D | 27 | 36.278 | 66.174 | -6.384 | 1.00 | 21.21 | C |
| ATOM | 5155 | O | LEU | D | 27 | 36.628 | 66.287 | -7.551 | 1.00 | 22.42 | O |
| ATOM | 5156 | CB | LEU | D | 27 | 34.292 | 64.944 | -5.429 | 1.00 | 23.59 | C |
| ATOM | 5157 | CG | LEU | D | 27 | 34.063 | 63.736 | -6.344 | 1.00 | 23.57 | C |
| ATOM | 5158 | CD1 | LEU | D | 27 | 35.266 | 62.833 | -6.410 | 1.00 | 25.32 | C |
| ATOM | 5159 | CD2 | LEU | D | 27 | 32.934 | 62.930 | -5.799 | 1.00 | 23.66 | C |
| ATOM | 5160 | N | THR | D | 28 | 37.130 | 66.018 | -5.378 | 1.00 | 21.44 | N |
| ATOM | 5161 | CA | THR | D | 28 | 38.567 | 65.826 | -5.580 | 1.00 | 21.17 | C |
| ATOM | 5162 | C | THR | D | 28 | 38.822 | 64.339 | -5.792 | 1.00 | 19.26 | C |
| ATOM | 5163 | O | THR | D | 28 | 38.908 | 63.564 | -4.842 | 1.00 | 17.23 | O |
| ATOM | 5164 | CB | THR | D | 28 | 39.407 | 66.328 | -4.386 | 1.00 | 22.05 | C |
| ATOM | 5165 | OG1 | THR | D | 28 | 39.028 | 67.665 | -4.054 | 1.00 | 23.27 | O |
| ATOM | 5166 | CG2 | THR | D | 28 | 40.877 | 66.305 | -4.724 | 1.00 | 22.71 | C |
| ATOM | 5167 | N | PHE | D | 29 | 38.970 | 63.970 | -7.068 | 1.00 | 20.29 | N |
| ATOM | 5168 | CA | PHE | D | 29 | 39.003 | 62.569 | -7.505 | 1.00 | 19.70 | C |
| ATOM | 5169 | C | PHE | D | 29 | 40.016 | 61.795 | -6.718 | 1.00 | 19.95 | C |
| ATOM | 5170 | O | PHE | D | 29 | 39.728 | 60.696 | -6.244 | 1.00 | 17.40 | O |
| ATOM | 5171 | CB | PHE | D | 29 | 39.291 | 62.503 | -9.018 | 1.00 | 20.33 | C |
| ATOM | 5172 | CG | PHE | D | 29 | 39.537 | 61.101 | -9.555 | 1.00 | 19.74 | C |
| ATOM | 5173 | CD1 | PHE | D | 29 | 38.506 | 60.354 | -10.132 | 1.00 | 19.52 | C |
| ATOM | 5174 | CD2 | PHE | D | 29 | 40.793 | 60.548 | -9.509 | 1.00 | 20.12 | C |
| ATOM | 5175 | CE1 | PHE | D | 29 | 38.739 | 59.075 | -10.648 | 1.00 | 19.95 | C |
| ATOM | 5176 | CE2 | PHE | D | 29 | 41.034 | 59.252 | -10.031 | 1.00 | 19.32 | C |
| ATOM | 5177 | CZ | PHE | D | 29 | 40.002 | 58.530 | -10.578 | 1.00 | 20.27 | C |
| ATOM | 5178 | N | SER | D | 30 | 41.200 | 62.376 | -6.531 | 1.00 | 20.71 | N |
| ATOM | 5179 | CA | SER | D | 30 | 42.313 | 61.681 | -5.864 | 1.00 | 20.74 | C |
| ATOM | 5180 | C | SER | D | 30 | 42.067 | 61.306 | -4.411 | 1.00 | 20.28 | C |
| ATOM | 5181 | O | SER | D | 30 | 42.764 | 60.442 | -3.853 | 1.00 | 19.19 | O |
| ATOM | 5182 | CB | SER | D | 30 | 43.564 | 62.534 | -5.919 | 1.00 | 21.93 | C |
| ATOM | 5183 | OG | SER | D | 30 | 43.328 | 63.783 | -5.300 | 1.00 | 20.39 | O |
| ATOM | 5184 | N | ASN | D | 31 | 41.086 | 61.962 | -3.795 | 1.00 | 21.68 | N |
| ATOM | 5185 | CA | ASN | D | 31 | 40.753 | 61.705 | -2.413 | 1.00 | 21.39 | C |
| ATOM | 5186 | C | ASN | D | 31 | 39.696 | 60.634 | -2.159 | 1.00 | 22.13 | C |
| ATOM | 5187 | O | ASN | D | 31 | 39.392 | 60.336 | -0.995 | 1.00 | 22.98 | O |
| ATOM | 5188 | CB | ASN | D | 31 | 40.334 | 62.999 | -1.718 | 1.00 | 22.20 | C |

```
ATOM   5189  CG  ASN D  31    40.535  62.921   -0.223  1.00 24.56          C
ATOM   5190  OD1 ASN D  31    41.537  62.354    0.251  1.00 26.54          O
ATOM   5191  ND2 ASN D  31    39.580  63.447    0.533  1.00 26.95          N
ATOM   5192  N   TYR D  32    39.187  60.020   -3.234  1.00 22.01          N
ATOM   5193  CA  TYR D  32    38.124  59.032   -3.160  1.00 20.16          C
ATOM   5194  C   TYR D  32    38.497  57.628   -3.675  1.00 19.07          C
ATOM   5195  O   TYR D  32    39.142  57.472   -4.729  1.00 18.71          O
ATOM   5196  CB  TYR D  32    36.934  59.535   -3.962  1.00 22.77          C
ATOM   5197  CG  TYR D  32    36.161  60.618   -3.261  1.00 24.13          C
ATOM   5198  CD1 TYR D  32    34.984  60.310   -2.568  1.00 26.50          C
ATOM   5199  CD2 TYR D  32    36.595  61.943   -3.289  1.00 23.87          C
ATOM   5200  CE1 TYR D  32    34.251  61.289   -1.907  1.00 25.20          C
ATOM   5201  CE2 TYR D  32    35.880  62.942   -2.642  1.00 24.69          C
ATOM   5202  CZ  TYR D  32    34.702  62.609   -1.940  1.00 25.91          C
ATOM   5203  OH  TYR D  32    33.954  63.559   -1.286  1.00 27.28          O
ATOM   5204  N   TRP D  33    38.109  56.625   -2.903  1.00 17.84          N
ATOM   5205  CA  TRP D  33    38.151  55.254   -3.360  1.00 16.61          C
ATOM   5206  C   TRP D  33    37.277  55.101   -4.610  1.00 15.56          C
ATOM   5207  O   TRP D  33    36.202  55.711   -4.733  1.00 15.49          O
ATOM   5208  CB  TRP D  33    37.678  54.297   -2.265  1.00 15.24          C
ATOM   5209  CG  TRP D  33    38.458  54.332   -0.971  1.00 16.28          C
ATOM   5210  CD1 TRP D  33    39.640  54.993   -0.719  1.00 16.08          C
ATOM   5211  CD2 TRP D  33    38.135  53.623    0.230  1.00 16.01          C
ATOM   5212  NE1 TRP D  33    40.044  54.756    0.574  1.00 16.85          N
ATOM   5213  CE2 TRP D  33    39.143  53.912    1.177  1.00 16.60          C
ATOM   5214  CE3 TRP D  33    37.082  52.782    0.602  1.00 16.63          C
ATOM   5215  CZ2 TRP D  33    39.124  53.387    2.470  1.00 17.68          C
ATOM   5216  CZ3 TRP D  33    37.074  52.251    1.871  1.00 15.85          C
ATOM   5217  CH2 TRP D  33    38.091  52.542    2.791  1.00 15.72          C
ATOM   5218  N   MET D  34    37.778  54.334   -5.581  1.00 15.79          N
ATOM   5219  CA  MET D  34    37.085  54.126   -6.844  1.00 14.08          C
ATOM   5220  C   MET D  34    36.909  52.640   -7.066  1.00 13.23          C
ATOM   5221  O   MET D  34    37.829  51.856   -6.811  1.00 13.88          O
ATOM   5222  CB  MET D  34    37.931  54.608   -8.007  1.00 14.87          C
ATOM   5223  CG  MET D  34    38.228  56.069   -7.969  1.00 15.07          C
ATOM   5224  SD  MET D  34    36.749  56.868   -8.441  1.00 19.09          S
ATOM   5225  CE  MET D  34    37.013  58.431   -7.580  1.00 15.05          C
ATOM   5226  N   SER D  35    35.761  52.282   -7.599  1.00 12.95          N
ATOM   5227  CA  SER D  35    35.490  50.859   -7.913  1.00 12.83          C
ATOM   5228  C   SER D  35    34.643  50.686   -9.172  1.00 13.87          C
ATOM   5229  O   SER D  35    34.032  51.648   -9.704  1.00 11.35          O
ATOM   5230  CB  SER D  35    34.809  50.211   -6.729  1.00 14.08          C
ATOM   5231  OG  SER D  35    33.448  50.628   -6.578  1.00 13.64          O
ATOM   5232  N   TRP D  36    34.606  49.430   -9.671  1.00 11.26          N
ATOM   5233  CA  TRP D  36    33.758  49.089  -10.785  1.00 12.01          C
ATOM   5234  C   TRP D  36    32.808  48.024  -10.283  1.00 12.84          C
ATOM   5235  O   TRP D  36    33.258  47.093   -9.646  1.00 11.09          O
ATOM   5236  CB  TRP D  36    34.551  48.537  -11.959  1.00 11.49          C
ATOM   5237  CG  TRP D  36    35.370  49.529  -12.667  1.00 12.16          C
ATOM   5238  CD1 TRP D  36    36.633  49.811  -12.420  1.00 11.07          C
ATOM   5239  CD2 TRP D  36    34.943  50.404  -13.712  1.00 10.61          C
ATOM   5240  NE1 TRP D  36    37.082  50.803  -13.257  1.00 12.14          N
ATOM   5241  CE2 TRP D  36    36.054  51.194  -14.064  1.00 11.11          C
ATOM   5242  CE3 TRP D  36    33.745  50.579  -14.399  1.00  9.31          C
ATOM   5243  CZ2 TRP D  36    36.003  52.161  -15.056  1.00 10.68          C
ATOM   5244  CZ3 TRP D  36    33.688  51.586  -15.400  1.00 13.35          C
ATOM   5245  CH2 TRP D  36    34.815  52.349  -15.708  1.00 10.99          C
ATOM   5246  N   VAL D  37    31.512  48.239  -10.509  1.00 12.37          N
ATOM   5247  CA  VAL D  37    30.456  47.264  -10.251  1.00 13.08          C
ATOM   5248  C   VAL D  37    29.705  46.983  -11.554  1.00 13.35          C
ATOM   5249  O   VAL D  37    29.312  47.907  -12.269  1.00 13.69          O
ATOM   5250  CB  VAL D  37    29.508  47.817   -9.158  1.00 12.13          C
ATOM   5251  CG1 VAL D  37    28.254  47.003   -9.028  1.00 14.20          C
ATOM   5252  CG2 VAL D  37    30.233  47.939   -7.854  1.00 13.09          C
ATOM   5253  N   ARG D  38    29.554  45.702  -11.911  1.00 11.30          N
```

```
ATOM   5254  CA   ARG D   38     28.791  45.329 -13.091  1.00 12.49         C
ATOM   5255  C    ARG D   38     27.402  44.799 -12.725  1.00 12.00         C
ATOM   5256  O    ARG D   38     27.200  44.329 -11.629  1.00 11.79         O
ATOM   5257  CB   ARG D   38     29.551  44.346 -14.007  1.00 12.28         C
ATOM   5258  CG   ARG D   38     29.849  43.036 -13.370  1.00 14.09         C
ATOM   5259  CD   ARG D   38     30.801  42.263 -14.246  1.00 14.31         C
ATOM   5260  NE   ARG D   38     31.112  40.973 -13.648  1.00 15.42         N
ATOM   5261  CZ   ARG D   38     32.043  40.149 -14.102  1.00 16.16         C
ATOM   5262  NH1  ARG D   38     32.792  40.478 -15.129  1.00 16.47         N
ATOM   5263  NH2  ARG D   38     32.226  38.988 -13.495  1.00 16.84         N
ATOM   5264  N    GLN D   39     26.450  45.001 -13.616  1.00 13.16         N
ATOM   5265  CA   GLN D   39     25.090  44.540 -13.462  1.00 15.87         C
ATOM   5266  C    GLN D   39     24.702  43.613 -14.634  1.00 16.86         C
ATOM   5267  O    GLN D   39     24.869  43.953 -15.788  1.00 15.60         O
ATOM   5268  CB   GLN D   39     24.158  45.751 -13.382  1.00 15.61         C
ATOM   5269  CG   GLN D   39     22.756  45.396 -12.934  1.00 17.21         C
ATOM   5270  CD   GLN D   39     21.886  46.608 -12.619  1.00 18.69         C
ATOM   5271  OE1  GLN D   39     22.095  47.730 -13.131  1.00 21.94         O
ATOM   5272  NE2  GLN D   39     20.909  46.389 -11.769  1.00 21.76         N
ATOM   5273  N    SER D   40     24.219  42.406 -14.337  1.00 18.87         N
ATOM   5274  CA   SER D   40     23.632  41.571 -15.367  1.00 21.37         C
ATOM   5275  C    SER D   40     22.353  40.910 -14.850  1.00 24.65         C
ATOM   5276  O    SER D   40     22.135  40.819 -13.646  1.00 25.01         O
ATOM   5277  CB   SER D   40     24.622  40.509 -15.751  1.00 22.50         C
ATOM   5278  OG   SER D   40     24.814  39.656 -14.648  1.00 22.02         O
ATOM   5279  N    PRO D   41     21.496  40.458 -15.762  1.00 26.75         N
ATOM   5280  CA   PRO D   41     20.247  39.821 -15.349  1.00 28.21         C
ATOM   5281  C    PRO D   41     20.452  38.637 -14.432  1.00 29.49         C
ATOM   5282  O    PRO D   41     19.709  38.468 -13.450  1.00 30.73         O
ATOM   5283  CB   PRO D   41     19.640  39.360 -16.681  1.00 28.36         C
ATOM   5284  CG   PRO D   41     20.214  40.274 -17.701  1.00 28.46         C
ATOM   5285  CD   PRO D   41     21.608  40.564 -17.229  1.00 26.86         C
ATOM   5286  N    GLU D   42     21.478  37.847 -14.722  1.00 30.21         N
ATOM   5287  CA   GLU D   42     21.661  36.572 -14.043  1.00 30.65         C
ATOM   5288  C    GLU D   42     22.465  36.655 -12.756  1.00 30.71         C
ATOM   5289  O    GLU D   42     22.302  35.803 -11.894  1.00 31.58         O
ATOM   5290  CB   GLU D   42     22.239  35.489 -14.980  1.00 32.51         C
ATOM   5291  CG   GLU D   42     23.310  35.920 -15.954  1.00 34.65         C
ATOM   5292  CD   GLU D   42     22.750  36.511 -17.234  1.00 36.51         C
ATOM   5293  OE1  GLU D   42     22.844  37.749 -17.393  1.00 34.69         O
ATOM   5294  OE2  GLU D   42     22.213  35.749 -18.075  1.00 38.09         O
ATOM   5295  N    LYS D   43     23.294  37.686 -12.597  1.00 29.34         N
ATOM   5296  CA   LYS D   43     24.111  37.819 -11.401  1.00 28.28         C
ATOM   5297  C    LYS D   43     23.813  39.068 -10.557  1.00 26.00         C
ATOM   5298  O    LYS D   43     24.373  39.215  -9.472  1.00 26.96         O
ATOM   5299  CB   LYS D   43     25.595  37.803 -11.783  1.00 30.56         C
ATOM   5300  CG   LYS D   43     26.130  36.419 -12.081  1.00 32.26         C
ATOM   5301  CD   LYS D   43     26.795  35.802 -10.856  1.00 33.38         C
ATOM   5302  CE   LYS D   43     26.850  34.275 -11.003  1.00 33.90         C
ATOM   5303  NZ   LYS D   43     28.033  33.702 -10.311  1.00 34.85         N
ATOM   5304  N    GLY D   44     22.972  39.971 -11.058  1.00 23.03         N
ATOM   5305  CA   GLY D   44     22.659  41.192 -10.347  1.00 21.90         C
ATOM   5306  C    GLY D   44     23.815  42.161 -10.312  1.00 18.28         C
ATOM   5307  O    GLY D   44     24.601  42.253 -11.236  1.00 16.29         O
ATOM   5308  N    LEU D   45     23.922  42.881  -9.218  1.00 16.86         N
ATOM   5309  CA   LEU D   45     25.017  43.778  -9.001  1.00 15.53         C
ATOM   5310  C    LEU D   45     26.258  43.054  -8.471  1.00 14.79         C
ATOM   5311  O    LEU D   45     26.197  42.435  -7.412  1.00 15.96         O
ATOM   5312  CB   LEU D   45     24.581  44.868  -8.019  1.00 16.29         C
ATOM   5313  CG   LEU D   45     23.574  45.855  -8.598  1.00 15.99         C
ATOM   5314  CD1  LEU D   45     22.996  46.676  -7.436  1.00 16.37         C
ATOM   5315  CD2  LEU D   45     24.176  46.759  -9.694  1.00 15.47         C
ATOM   5316  N    GLU D   46     27.369  43.141  -9.210  1.00 13.32         N
ATOM   5317  CA   GLU D   46     28.646  42.483  -8.853  1.00 14.50         C
ATOM   5318  C    GLU D   46     29.825  43.453  -8.828  1.00 12.48         C
```

```
ATOM   5319  O    GLU D  46     30.256  43.943  -9.873  1.00 12.45           O
ATOM   5320  CB   GLU D  46     28.949  41.410  -9.895  1.00 14.60           C
ATOM   5321  CG   GLU D  46     29.992  40.355  -9.522  1.00 16.41           C
ATOM   5322  CD   GLU D  46     30.222  39.417 -10.699  1.00 19.64           C
ATOM   5323  OE1  GLU D  46     29.513  39.601 -11.732  1.00 18.77           O
ATOM   5324  OE2  GLU D  46     31.050  38.475 -10.554  1.00 27.01           O
ATOM   5325  N    TRP D  47     30.347  43.696  -7.643  1.00 11.34           N
ATOM   5326  CA   TRP D  47     31.625  44.365  -7.438  1.00 12.59           C
ATOM   5327  C    TRP D  47     32.764  43.544  -8.032  1.00 12.63           C
ATOM   5328  O    TRP D  47     32.852  42.329  -7.771  1.00 14.58           O
ATOM   5329  CB   TRP D  47     31.828  44.606  -5.950  1.00 12.86           C
ATOM   5330  CG   TRP D  47     33.051  45.272  -5.538  1.00 12.32           C
ATOM   5331  CD1  TRP D  47     33.268  46.626  -5.437  1.00 13.18           C
ATOM   5332  CD2  TRP D  47     34.248  44.640  -5.114  1.00 12.34           C
ATOM   5333  NE1  TRP D  47     34.527  46.844  -4.969  1.00 14.76           N
ATOM   5334  CE2  TRP D  47     35.144  45.644  -4.743  1.00 11.36           C
ATOM   5335  CE3  TRP D  47     34.638  43.298  -4.982  1.00 14.27           C
ATOM   5336  CZ2  TRP D  47     36.426  45.367  -4.271  1.00 13.24           C
ATOM   5337  CZ3  TRP D  47     35.917  43.024  -4.498  1.00 12.45           C
ATOM   5338  CH2  TRP D  47     36.791  44.046  -4.160  1.00 15.47           C
ATOM   5339  N    VAL D  48     33.591  44.180  -8.860  1.00 15.04           N
ATOM   5340  CA   VAL D  48     34.710  43.497  -9.523  1.00 14.39           C
ATOM   5341  C    VAL D  48     36.106  43.990  -9.168  1.00 14.31           C
ATOM   5342  O    VAL D  48     37.038  43.203  -9.119  1.00 13.74           O
ATOM   5343  CB   VAL D  48     34.536  43.411 -11.078  1.00 14.98           C
ATOM   5344  CG1  VAL D  48     33.275  42.658 -11.465  1.00 16.33           C
ATOM   5345  CG2  VAL D  48     34.593  44.823 -11.744  1.00 14.66           C
ATOM   5346  N    ALA D  49     36.292  45.292  -8.905  1.00 11.39           N
ATOM   5347  CA   ALA D  49     37.624  45.830  -8.678  1.00 13.14           C
ATOM   5348  C    ALA D  49     37.527  47.152  -7.929  1.00 11.68           C
ATOM   5349  O    ALA D  49     36.552  47.866  -8.097  1.00 15.39           O
ATOM   5350  CB   ALA D  49     38.324  46.078  -9.982  1.00 11.12           C
ATOM   5351  N    GLU D  50     38.552  47.443  -7.146  1.00 14.37           N
ATOM   5352  CA   GLU D  50     38.648  48.696  -6.375  1.00 13.72           C
ATOM   5353  C    GLU D  50     40.067  49.174  -6.256  1.00 13.14           C
ATOM   5354  O    GLU D  50     41.008  48.411  -6.185  1.00 14.85           O
ATOM   5355  CB   GLU D  50     38.058  48.492  -4.989  1.00 14.54           C
ATOM   5356  CG   GLU D  50     37.909  49.771  -4.137  1.00 15.83           C
ATOM   5357  CD   GLU D  50     36.849  49.627  -3.050  1.00 14.60           C
ATOM   5358  OE1  GLU D  50     35.790  49.046  -3.357  1.00 17.86           O
ATOM   5359  OE2  GLU D  50     37.050  50.095  -1.889  1.00 14.61           O
ATOM   5360  N    ILE D  51     40.238  50.489  -6.211  1.00 13.38           N
ATOM   5361  CA   ILE D  51     41.550  51.060  -6.074  1.00 14.27           C
ATOM   5362  C    ILE D  51     41.491  52.211  -5.064  1.00 16.16           C
ATOM   5363  O    ILE D  51     40.588  53.027  -5.121  1.00 15.22           O
ATOM   5364  CB   ILE D  51     42.112  51.517  -7.441  1.00 12.05           C
ATOM   5365  CG1  ILE D  51     43.592  51.891  -7.314  1.00 13.80           C
ATOM   5366  CG2  ILE D  51     41.244  52.604  -8.095  1.00 12.20           C
ATOM   5367  CD1  ILE D  51     44.286  51.975  -8.624  1.00 13.61           C
ATOM   5368  N    ARG D  52     42.437  52.205  -4.142  1.00 17.32           N
ATOM   5369  CA   ARG D  52     42.500  53.181  -3.053  1.00 18.63           C
ATOM   5370  C    ARG D  52     43.388  54.340  -3.470  1.00 19.20           C
ATOM   5371  O    ARG D  52     43.564  54.575  -4.664  1.00 18.78           O
ATOM   5372  CB   ARG D  52     43.009  52.498  -1.792  1.00 19.53           C
ATOM   5373  CG   ARG D  52     42.170  51.301  -1.384  1.00 19.62           C
ATOM   5374  CD   ARG D  52     40.736  51.754  -1.052  1.00 20.18           C
ATOM   5375  NE   ARG D  52     39.755  50.690  -0.810  1.00 19.77           N
ATOM   5376  CZ   ARG D  52     39.574  50.040   0.335  1.00 20.58           C
ATOM   5377  NH1  ARG D  52     40.379  50.212   1.367  1.00 21.72           N
ATOM   5378  NH2  ARG D  52     38.608  49.144   0.432  1.00 17.36           N
ATOM   5379  N    LEU D  52A    43.898  55.096  -2.493  1.00 21.14           N
ATOM   5380  CA   LEU D  52A    44.522  56.386  -2.762  1.00 22.00           C
ATOM   5381  C    LEU D  52A    46.059  56.300  -2.911  1.00 23.07           C
ATOM   5382  O    LEU D  52A    46.691  55.298  -2.568  1.00 21.65           O
ATOM   5383  CB   LEU D  52A    44.174  57.386  -1.635  1.00 21.76           C
```

```
ATOM   5384   CG   LEU D   52A    42.728   57.442   -1.154   1.00 21.64          C
ATOM   5385   CD1  LEU D   52A    42.505   58.589   -0.129   1.00 23.24          C
ATOM   5386   CD2  LEU D   52A    41.768   57.591   -2.319   1.00 21.62          C
ATOM   5387   N    LYS D   52B    46.640   57.383   -3.414   1.00 25.78          N
ATOM   5388   CA   LYS D   52B    48.089   57.549   -3.476   1.00 27.01          C
ATOM   5389   C    LYS D   52B    48.711   57.213   -2.124   1.00 28.64          C
ATOM   5390   O    LYS D   52B    49.677   56.436   -2.037   1.00 29.85          O
ATOM   5391   CB   LYS D   52B    48.416   58.992   -3.860   1.00 27.85          C
ATOM   5392   CG   LYS D   52B    49.874   59.255   -4.182   1.00 28.67          C
ATOM   5393   CD   LYS D   52B    49.997   60.554   -4.977   1.00 29.81          C
ATOM   5394   CE   LYS D   52B    51.450   60.974   -5.221   1.00 31.58          C
ATOM   5395   NZ   LYS D   52B    51.614   61.613   -6.562   1.00 32.14          N
ATOM   5396   N    SER D   52C    48.110   57.755   -1.062   1.00 27.93          N
ATOM   5397   CA   SER D   52C    48.581   57.543    0.290   1.00 28.06          C
ATOM   5398   C    SER D   52C    48.455   56.100    0.750   1.00 28.20          C
ATOM   5399   O    SER D   52C    48.989   55.746    1.783   1.00 28.58          O
ATOM   5400   CB   SER D   52C    47.821   58.456    1.251   1.00 28.95          C
ATOM   5401   OG   SER D   52C    46.459   58.549    0.883   1.00 29.92          O
ATOM   5402   N    ASP D   53     47.745   55.271   -0.011   1.00 27.68          N
ATOM   5403   CA   ASP D   53     47.664   53.829    0.259   1.00 27.10          C
ATOM   5404   C    ASP D   53     48.507   53.023   -0.740   1.00 27.79          C
ATOM   5405   O    ASP D   53     48.318   51.812   -0.902   1.00 25.03          O
ATOM   5406   CB   ASP D   53     46.209   53.361    0.129   1.00 28.44          C
ATOM   5407   CG   ASP D   53     45.228   54.205    0.940   1.00 28.21          C
ATOM   5408   OD1  ASP D   53     45.325   54.183    2.185   1.00 26.61          O
ATOM   5409   OD2  ASP D   53     44.355   54.879    0.333   1.00 29.39          O
ATOM   5410   N    ASN D   54     49.414   53.697   -1.434   1.00 28.22          N
ATOM   5411   CA   ASN D   54     50.200   53.079   -2.477   1.00 27.64          C
ATOM   5412   C    ASN D   54     49.298   52.570   -3.605   1.00 26.75          C
ATOM   5413   O    ASN D   54     49.559   51.521   -4.204   1.00 27.19          O
ATOM   5414   CB   ASN D   54     51.036   51.928   -1.915   1.00 29.71          C
ATOM   5415   CG   ASN D   54     52.105   51.458   -2.882   1.00 31.18          C
ATOM   5416   OD1  ASN D   54     52.767   52.280   -3.536   1.00 35.70          O
ATOM   5417   ND2  ASN D   54     52.288   50.123   -2.984   1.00 33.69          N
ATOM   5418   N    TYR D   55     48.232   53.319   -3.884   1.00 24.93          N
ATOM   5419   CA   TYR D   55     47.301   52.957   -4.931   1.00 22.87          C
ATOM   5420   C    TYR D   55     46.837   51.503   -4.888   1.00 21.70          C
ATOM   5421   O    TYR D   55     46.711   50.892   -5.942   1.00 21.58          O
ATOM   5422   CB   TYR D   55     47.949   53.220   -6.275   1.00 22.89          C
ATOM   5423   CG   TYR D   55     48.085   54.677   -6.578   1.00 24.12          C
ATOM   5424   CD1  TYR D   55     46.956   55.465   -6.652   1.00 22.09          C
ATOM   5425   CD2  TYR D   55     49.325   55.272   -6.788   1.00 23.61          C
ATOM   5426   CE1  TYR D   55     47.035   56.807   -6.941   1.00 24.24          C
ATOM   5427   CE2  TYR D   55     49.407   56.659   -7.078   1.00 24.45          C
ATOM   5428   CZ   TYR D   55     48.248   57.395   -7.142   1.00 22.88          C
ATOM   5429   OH   TYR D   55     48.251   58.748   -7.437   1.00 27.25          O
ATOM   5430   N    ALA D   56     46.540   51.014   -3.687   1.00 20.23          N
ATOM   5431   CA   ALA D   56     46.179   49.639   -3.411   1.00 19.38          C
ATOM   5432   C    ALA D   56     44.947   49.171   -4.174   1.00 18.90          C
ATOM   5433   O    ALA D   56     43.928   49.866   -4.220   1.00 15.30          O
ATOM   5434   CB   ALA D   56     45.955   49.470   -1.938   1.00 20.50          C
ATOM   5435   N    THR D   57     45.032   47.968   -4.744   1.00 16.23          N
ATOM   5436   CA   THR D   57     43.998   47.478   -5.637   1.00 15.68          C
ATOM   5437   C    THR D   57     43.477   46.156   -5.074   1.00 15.89          C
ATOM   5438   O    THR D   57     44.225   45.444   -4.389   1.00 16.10          O
ATOM   5439   CB   THR D   57     44.520   47.298   -7.100   1.00 16.18          C
ATOM   5440   OG1  THR D   57     45.562   46.311   -7.108   1.00 19.27          O
ATOM   5441   CG2  THR D   57     45.059   48.598   -7.677   1.00 17.47          C
ATOM   5442   N    TYR D   58     42.181   45.916   -5.265   1.00 16.00          N
ATOM   5443   CA   TYR D   58     41.466   44.688   -4.805   1.00 15.52          C
ATOM   5444   C    TYR D   58     40.517   44.248   -5.904   1.00 16.21          C
ATOM   5445   O    TYR D   58     39.965   45.075   -6.652   1.00 15.02          O
ATOM   5446   CB   TYR D   58     40.676   44.934   -3.494   1.00 16.94          C
ATOM   5447   CG   TYR D   58     41.531   45.568   -2.430   1.00 16.66          C
ATOM   5448   CD1  TYR D   58     41.584   46.934   -2.305   1.00 16.36          C
```

```
ATOM   5449  CD2 TYR D  58     42.333  44.793  -1.572  1.00 15.96          C
ATOM   5450  CE1 TYR D  58     42.404  47.544  -1.347  1.00 17.91          C
ATOM   5451  CE2 TYR D  58     43.138  45.383  -0.615  1.00 17.15          C
ATOM   5452  CZ  TYR D  58     43.161  46.779  -0.506  1.00 17.11          C
ATOM   5453  OH  TYR D  58     43.968  47.415   0.414  1.00 16.57          O
ATOM   5454  N   TYR D  59     40.313  42.928  -6.014  1.00 14.84          N
ATOM   5455  CA  TYR D  59     39.553  42.380  -7.104  1.00 16.43          C
ATOM   5456  C   TYR D  59     38.631  41.307  -6.607  1.00 17.20          C
ATOM   5457  O   TYR D  59     38.963  40.604  -5.646  1.00 18.99          O
ATOM   5458  CB  TYR D  59     40.479  41.747  -8.170  1.00 16.94          C
ATOM   5459  CG  TYR D  59     41.379  42.742  -8.843  1.00 17.07          C
ATOM   5460  CD1 TYR D  59     40.941  43.447  -9.959  1.00 18.16          C
ATOM   5461  CD2 TYR D  59     42.642  43.023  -8.338  1.00 16.51          C
ATOM   5462  CE1 TYR D  59     41.720  44.385 -10.563  1.00 15.97          C
ATOM   5463  CE2 TYR D  59     43.438  43.960  -8.938  1.00 17.34          C
ATOM   5464  CZ  TYR D  59     42.986  44.631 -10.069  1.00 15.86          C
ATOM   5465  OH  TYR D  59     43.775  45.572 -10.657  1.00 16.28          O
ATOM   5466  N   ALA D  60     37.502  41.145  -7.283  1.00 15.18          N
ATOM   5467  CA  ALA D  60     36.657  39.993  -7.049  1.00 15.72          C
ATOM   5468  C   ALA D  60     37.439  38.786  -7.545  1.00 14.41          C
ATOM   5469  O   ALA D  60     38.132  38.862  -8.545  1.00 13.90          O
ATOM   5470  CB  ALA D  60     35.376  40.114  -7.778  1.00 14.62          C
ATOM   5471  N   GLU D  61     37.326  37.686  -6.835  1.00 16.85          N
ATOM   5472  CA  GLU D  61     37.977  36.451  -7.249  1.00 18.72          C
ATOM   5473  C   GLU D  61     37.590  35.959  -8.646  1.00 19.95          C
ATOM   5474  O   GLU D  61     38.374  35.266  -9.302  1.00 21.36          O
ATOM   5475  CB  GLU D  61     37.729  35.355  -6.202  1.00 19.67          C
ATOM   5476  CG  GLU D  61     38.412  35.606  -4.846  1.00 21.39          C
ATOM   5477  CD  GLU D  61     39.943  35.596  -4.918  1.00 24.94          C
ATOM   5478  OE1 GLU D  61     40.611  36.516  -4.363  1.00 26.15          O
ATOM   5479  OE2 GLU D  61     40.493  34.675  -5.559  1.00 26.70          O
ATOM   5480  N   SER D  62     36.386  36.289  -9.099  1.00 19.16          N
ATOM   5481  CA  SER D  62     35.930  35.974 -10.453  1.00 19.80          C
ATOM   5482  C   SER D  62     36.668  36.715 -11.584  1.00 20.11          C
ATOM   5483  O   SER D  62     36.481  36.393 -12.759  1.00 20.00          O
ATOM   5484  CB  SER D  62     34.448  36.305 -10.585  1.00 20.91          C
ATOM   5485  OG  SER D  62     34.205  37.701 -10.331  1.00 23.83          O
ATOM   5486  N   VAL D  63     37.442  37.746 -11.252  1.00 18.13          N
ATOM   5487  CA  VAL D  63     38.171  38.474 -12.276  1.00 18.94          C
ATOM   5488  C   VAL D  63     39.649  38.645 -11.945  1.00 19.28          C
ATOM   5489  O   VAL D  63     40.397  39.294 -12.686  1.00 19.19          O
ATOM   5490  CB  VAL D  63     37.495  39.832 -12.554  1.00 17.64          C
ATOM   5491  CG1 VAL D  63     36.041  39.606 -12.920  1.00 18.24          C
ATOM   5492  CG2 VAL D  63     37.672  40.783 -11.385  1.00 19.66          C
ATOM   5493  N   LYS D  64     40.092  38.029 -10.856  1.00 20.02          N
ATOM   5494  CA  LYS D  64     41.496  38.091 -10.489  1.00 22.36          C
ATOM   5495  C   LYS D  64     42.324  37.516 -11.635  1.00 22.46          C
ATOM   5496  O   LYS D  64     41.982  36.478 -12.181  1.00 22.32          O
ATOM   5497  CB  LYS D  64     41.770  37.324  -9.182  1.00 25.06          C
ATOM   5498  CG  LYS D  64     41.078  37.923  -7.999  1.00 29.44          C
ATOM   5499  CD  LYS D  64     42.032  38.330  -6.881  1.00 30.89          C
ATOM   5500  CE  LYS D  64     41.280  38.899  -5.699  1.00 29.27          C
ATOM   5501  NZ  LYS D  64     41.999  38.622  -4.397  1.00 33.74          N
ATOM   5502  N   GLY D  65     43.374  38.231 -12.018  1.00 22.41          N
ATOM   5503  CA  GLY D  65     44.260  37.816 -13.100  1.00 22.44          C
ATOM   5504  C   GLY D  65     43.830  38.253 -14.486  1.00 23.46          C
ATOM   5505  O   GLY D  65     44.619  38.124 -15.426  1.00 26.42          O
ATOM   5506  N   LYS D  66     42.606  38.789 -14.612  1.00 21.88          N
ATOM   5507  CA  LYS D  66     41.984  39.176 -15.876  1.00 20.62          C
ATOM   5508  C   LYS D  66     41.852  40.685 -16.031  1.00 18.45          C
ATOM   5509  O   LYS D  66     42.030  41.188 -17.119  1.00 18.74          O
ATOM   5510  CB  LYS D  66     40.548  38.692 -15.939  1.00 22.48          C
ATOM   5511  CG  LYS D  66     40.381  37.233 -16.101  1.00 23.60          C
ATOM   5512  CD  LYS D  66     38.924  36.834 -15.970  1.00 24.06          C
ATOM   5513  CE  LYS D  66     38.084  37.416 -17.051  1.00 24.58          C
```

```
ATOM   5514   NZ   LYS D   66   36.947  36.537 -17.405  1.00 25.63      N
ATOM   5515   N    PHE D   67   41.401  41.334 -14.966  1.00 14.92      N
ATOM   5516   CA   PHE D   67   41.031  42.763 -14.976  1.00 15.29      C
ATOM   5517   C    PHE D   67   42.143  43.503 -14.236  1.00 14.43      C
ATOM   5518   O    PHE D   67   42.726  42.979 -13.279  1.00 15.50      O
ATOM   5519   CB   PHE D   67   39.682  42.997 -14.249  1.00 13.45      C
ATOM   5520   CG   PHE D   67   38.445  42.605 -15.024  1.00 13.73      C
ATOM   5521   CD1  PHE D   67   38.503  41.766 -16.130  1.00 14.36      C
ATOM   5522   CD2  PHE D   67   37.193  42.984 -14.564  1.00 13.24      C
ATOM   5523   CE1  PHE D   67   37.372  41.378 -16.788  1.00 14.63      C
ATOM   5524   CE2  PHE D   67   36.062  42.586 -15.228  1.00 15.06      C
ATOM   5525   CZ   PHE D   67   36.153  41.781 -16.328  1.00 16.07      C
ATOM   5526   N    THR D   68   42.412  44.735 -14.645  1.00 14.91      N
ATOM   5527   CA   THR D   68   43.361  45.551 -13.973  1.00 14.31      C
ATOM   5528   C    THR D   68   42.719  46.922 -13.853  1.00 14.30      C
ATOM   5529   O    THR D   68   42.334  47.514 -14.825  1.00 13.35      O
ATOM   5530   CB   THR D   68   44.716  45.637 -14.732  1.00 15.67      C
ATOM   5531   OG1  THR D   68   45.302  44.321 -14.897  1.00 15.18      O
ATOM   5532   CG2  THR D   68   45.690  46.479 -14.009  1.00 15.48      C
ATOM   5533   N    ILE D   69   42.570  47.379 -12.627  1.00 15.50      N
ATOM   5534   CA   ILE D   69   42.042  48.710 -12.354  1.00 12.89      C
ATOM   5535   C    ILE D   69   43.181  49.662 -12.163  1.00 14.08      C
ATOM   5536   O    ILE D   69   44.219  49.317 -11.588  1.00 14.28      O
ATOM   5537   CB   ILE D   69   41.070  48.704 -11.078  1.00 12.82      C
ATOM   5538   CG1  ILE D   69   40.204  49.987 -11.080  1.00 11.96      C
ATOM   5539   CG2  ILE D   69   41.837  48.345  -9.720  1.00 11.16      C
ATOM   5540   CD1  ILE D   69   39.195  50.075  -9.963  1.00 11.81      C
ATOM   5541   N    SER D   70   43.019  50.895 -12.644  1.00 13.68      N
ATOM   5542   CA   SER D   70   44.051  51.885 -12.427  1.00 15.33      C
ATOM   5543   C    SER D   70   43.407  53.247 -12.408  1.00 15.52      C
ATOM   5544   O    SER D   70   42.255  53.374 -12.766  1.00 15.74      O
ATOM   5545   CB   SER D   70   45.088  51.826 -13.534  1.00 15.74      C
ATOM   5546   OG   SER D   70   44.485  51.999 -14.793  1.00 17.39      O
ATOM   5547   N    ARG D   71   44.148  54.255 -11.953  1.00 17.08      N
ATOM   5548   CA   ARG D   71   43.623  55.633 -11.936  1.00 17.79      C
ATOM   5549   C    ARG D   71   44.672  56.667 -12.350  1.00 18.95      C
ATOM   5550   O    ARG D   71   45.845  56.511 -12.064  1.00 21.45      O
ATOM   5551   CB   ARG D   71   43.062  55.953 -10.538  1.00 17.29      C
ATOM   5552   CG   ARG D   71   44.111  55.892  -9.405  1.00 14.87      C
ATOM   5553   CD   ARG D   71   43.493  55.943  -7.996  1.00 15.41      C
ATOM   5554   NE   ARG D   71   42.682  57.149  -7.778  1.00 18.95      N
ATOM   5555   CZ   ARG D   71   41.670  57.239  -6.916  1.00 16.63      C
ATOM   5556   NH1  ARG D   71   41.330  56.209  -6.172  1.00 18.70      N
ATOM   5557   NH2  ARG D   71   40.984  58.359  -6.829  1.00 18.61      N
ATOM   5558   N    ASP D   72   44.228  57.735 -12.996  1.00 21.55      N
ATOM   5559   CA   ASP D   72   45.081  58.855 -13.349  1.00 21.46      C
ATOM   5560   C    ASP D   72   44.485  60.054 -12.606  1.00 21.24      C
ATOM   5561   O    ASP D   72   43.541  60.670 -13.063  1.00 19.51      O
ATOM   5562   CB   ASP D   72   45.077  59.044 -14.864  1.00 22.70      C
ATOM   5563   CG   ASP D   72   45.942  60.214 -15.332  1.00 23.95      C
ATOM   5564   OD1  ASP D   72   46.356  61.076 -14.521  1.00 26.62      O
ATOM   5565   OD2  ASP D   72   46.209  60.265 -16.537  1.00 27.54      O
ATOM   5566   N    ASP D   73   45.011  60.311 -11.425  1.00 21.86      N
ATOM   5567   CA   ASP D   73   44.506  61.374 -10.578  1.00 23.23      C
ATOM   5568   C    ASP D   73   44.580  62.731 -11.251  1.00 24.04      C
ATOM   5569   O    ASP D   73   43.687  63.544 -11.083  1.00 26.42      O
ATOM   5570   CB   ASP D   73   45.243  61.381  -9.244  1.00 22.21      C
ATOM   5571   CG   ASP D   73   44.804  60.268  -8.331  1.00 21.86      C
ATOM   5572   OD1  ASP D   73   43.729  59.683  -8.548  1.00 20.01      O
ATOM   5573   OD2  ASP D   73   45.510  59.996  -7.364  1.00 21.95      O
ATOM   5574   N    SER D   74   45.610  62.962 -12.053  1.00 24.63      N
ATOM   5575   CA   SER D   74   45.735  64.209 -12.811  1.00 25.51      C
ATOM   5576   C    SER D   74   44.604  64.408 -13.794  1.00 25.67      C
ATOM   5577   O    SER D   74   44.251  65.541 -14.100  1.00 26.48      O
ATOM   5578   CB   SER D   74   47.052  64.233 -13.582  1.00 26.10      C
```

| ATOM | 5579 | OG | SER | D | 74 | 46.886 | 63.607 | -14.840 | 1.00 | 28.46 | O |
|------|------|-----|-----|---|----|--------|--------|---------|------|-------|---|
| ATOM | 5580 | N | LYS | D | 75 | 44.019 | 63.318 | -14.292 | 1.00 | 25.13 | N |
| ATOM | 5581 | CA | LYS | D | 75 | 42.931 | 63.432 | -15.262 | 1.00 | 25.27 | C |
| ATOM | 5582 | C | LYS | D | 75 | 41.537 | 63.167 | -14.682 | 1.00 | 23.21 | C |
| ATOM | 5583 | O | LYS | D | 75 | 40.537 | 63.247 | -15.409 | 1.00 | 23.68 | O |
| ATOM | 5584 | CB | LYS | D | 75 | 43.167 | 62.475 | -16.439 | 1.00 | 25.39 | C |
| ATOM | 5585 | CG | LYS | D | 75 | 44.349 | 62.826 | -17.311 | 1.00 | 27.79 | C |
| ATOM | 5586 | CD | LYS | D | 75 | 44.257 | 62.101 | -18.642 | 1.00 | 28.52 | C |
| ATOM | 5587 | CE | LYS | D | 75 | 45.579 | 62.124 | -19.418 | 1.00 | 28.90 | C |
| ATOM | 5588 | NZ | LYS | D | 75 | 45.360 | 61.590 | -20.804 | 1.00 | 30.60 | N |
| ATOM | 5589 | N | SER | D | 76 | 41.485 | 62.827 | -13.400 | 1.00 | 23.15 | N |
| ATOM | 5590 | CA | SER | D | 76 | 40.250 | 62.447 | -12.702 | 1.00 | 22.09 | C |
| ATOM | 5591 | C | SER | D | 76 | 39.586 | 61.308 | -13.459 | 1.00 | 21.20 | C |
| ATOM | 5592 | O | SER | D | 76 | 38.378 | 61.343 | -13.719 | 1.00 | 20.10 | O |
| ATOM | 5593 | CB | SER | D | 76 | 39.301 | 63.643 | -12.575 | 1.00 | 22.91 | C |
| ATOM | 5594 | OG | SER | D | 76 | 39.837 | 64.575 | -11.670 | 1.00 | 21.13 | O |
| ATOM | 5595 | N | ARG | D | 77 | 40.406 | 60.330 | -13.847 | 1.00 | 20.59 | N |
| ATOM | 5596 | CA | ARG | D | 77 | 39.930 | 59.222 | -14.671 | 1.00 | 20.28 | C |
| ATOM | 5597 | C | ARG | D | 77 | 40.273 | 57.894 | -14.017 | 1.00 | 17.18 | C |
| ATOM | 5598 | O | ARG | D | 77 | 41.372 | 57.706 | -13.505 | 1.00 | 19.38 | O |
| ATOM | 5599 | CB | ARG | D | 77 | 40.530 | 59.283 | -16.087 | 1.00 | 21.57 | C |
| ATOM | 5600 | CG | ARG | D | 77 | 39.766 | 58.452 | -17.120 | 1.00 | 22.45 | C |
| ATOM | 5601 | CD | ARG | D | 77 | 40.038 | 58.902 | -18.557 | 1.00 | 24.69 | C |
| ATOM | 5602 | NE | ARG | D | 77 | 39.215 | 60.027 | -19.037 | 1.00 | 25.89 | N |
| ATOM | 5603 | CZ | ARG | D | 77 | 37.966 | 59.920 | -19.510 | 1.00 | 29.95 | C |
| ATOM | 5604 | NH1 | ARG | D | 77 | 37.320 | 58.742 | -19.521 | 1.00 | 29.55 | N |
| ATOM | 5605 | NH2 | ARG | D | 77 | 37.332 | 61.012 | -19.945 | 1.00 | 30.74 | N |
| ATOM | 5606 | N | LEU | D | 78 | 39.315 | 56.982 | -14.049 | 1.00 | 16.45 | N |
| ATOM | 5607 | CA | LEU | D | 78 | 39.476 | 55.601 | -13.573 | 1.00 | 15.91 | C |
| ATOM | 5608 | C | LEU | D | 78 | 39.439 | 54.683 | -14.800 | 1.00 | 14.92 | C |
| ATOM | 5609 | O | LEU | D | 78 | 38.634 | 54.873 | -15.709 | 1.00 | 14.65 | O |
| ATOM | 5610 | CB | LEU | D | 78 | 38.288 | 55.230 | -12.688 | 1.00 | 15.81 | C |
| ATOM | 5611 | CG | LEU | D | 78 | 38.250 | 53.877 | -11.977 | 1.00 | 14.40 | C |
| ATOM | 5612 | CD1 | LEU | D | 78 | 39.335 | 53.789 | -10.959 | 1.00 | 15.88 | C |
| ATOM | 5613 | CD2 | LEU | D | 78 | 36.844 | 53.640 | -11.361 | 1.00 | 15.20 | C |
| ATOM | 5614 | N | TYR | D | 79 | 40.212 | 53.608 | -14.776 | 1.00 | 15.87 | N |
| ATOM | 5615 | CA | TYR | D | 79 | 40.261 | 52.715 | -15.913 | 1.00 | 15.12 | C |
| ATOM | 5616 | C | TYR | D | 79 | 40.008 | 51.279 | -15.459 | 1.00 | 12.56 | C |
| ATOM | 5617 | O | TYR | D | 79 | 40.244 | 50.953 | -14.340 | 1.00 | 12.72 | O |
| ATOM | 5618 | CB | TYR | D | 79 | 41.619 | 52.779 | -16.585 | 1.00 | 18.09 | C |
| ATOM | 5619 | CG | TYR | D | 79 | 42.034 | 54.132 | -17.140 | 1.00 | 20.08 | C |
| ATOM | 5620 | CD1 | TYR | D | 79 | 41.701 | 54.493 | -18.412 | 1.00 | 20.38 | C |
| ATOM | 5621 | CD2 | TYR | D | 79 | 42.775 | 55.015 | -16.393 | 1.00 | 22.47 | C |
| ATOM | 5622 | CE1 | TYR | D | 79 | 42.095 | 55.717 | -18.958 | 1.00 | 22.50 | C |
| ATOM | 5623 | CE2 | TYR | D | 79 | 43.162 | 56.231 | -16.915 | 1.00 | 22.96 | C |
| ATOM | 5624 | CZ | TYR | D | 79 | 42.822 | 56.578 | -18.204 | 1.00 | 22.73 | C |
| ATOM | 5625 | OH | TYR | D | 79 | 43.199 | 57.813 | -18.752 | 1.00 | 25.89 | O |
| ATOM | 5626 | N | LEU | D | 80 | 39.454 | 50.483 | -16.350 | 1.00 | 12.08 | N |
| ATOM | 5627 | CA | LEU | D | 80 | 39.425 | 49.026 | -16.156 | 1.00 | 11.76 | C |
| ATOM | 5628 | C | LEU | D | 80 | 39.896 | 48.364 | -17.455 | 1.00 | 11.31 | C |
| ATOM | 5629 | O | LEU | D | 80 | 39.244 | 48.494 | -18.491 | 1.00 | 11.14 | O |
| ATOM | 5630 | CB | LEU | D | 80 | 38.032 | 48.574 | -15.781 | 1.00 | 12.03 | C |
| ATOM | 5631 | CG | LEU | D | 80 | 38.036 | 47.100 | -15.266 | 1.00 | 9.64 | C |
| ATOM | 5632 | CD1 | LEU | D | 80 | 38.815 | 46.920 | -13.976 | 1.00 | 9.29 | C |
| ATOM | 5633 | CD2 | LEU | D | 80 | 36.629 | 46.701 | -15.121 | 1.00 | 11.10 | C |
| ATOM | 5634 | N | GLN | D | 81 | 41.059 | 47.708 | -17.384 | 1.00 | 13.56 | N |
| ATOM | 5635 | CA | GLN | D | 81 | 41.600 | 46.888 | -18.454 | 1.00 | 14.47 | C |
| ATOM | 5636 | C | GLN | D | 81 | 41.070 | 45.487 | -18.234 | 1.00 | 15.37 | C |
| ATOM | 5637 | O | GLN | D | 81 | 41.278 | 44.920 | -17.176 | 1.00 | 14.17 | O |
| ATOM | 5638 | CB | GLN | D | 81 | 43.126 | 46.856 | -18.433 | 1.00 | 16.80 | C |
| ATOM | 5639 | CG | GLN | D | 81 | 43.781 | 45.979 | -19.512 | 1.00 | 15.89 | C |
| ATOM | 5640 | CD | GLN | D | 81 | 43.511 | 46.460 | -20.907 | 1.00 | 20.37 | C |
| ATOM | 5641 | OE1 | GLN | D | 81 | 43.599 | 47.666 | -21.179 | 1.00 | 19.87 | O |
| ATOM | 5642 | NE2 | GLN | D | 81 | 43.194 | 45.528 | -21.821 | 1.00 | 21.84 | N |
| ATOM | 5643 | N | MET | D | 82 | 40.377 | 44.966 | -19.237 | 1.00 | 15.88 | N |

```
ATOM   5644  CA   MET  D  82    39.653  43.738 -19.114  1.00 16.31       C
ATOM   5645  C    MET  D  82    40.251  42.775 -20.165  1.00 15.97       C
ATOM   5646  O    MET  D  82    40.112  43.004 -21.364  1.00 15.67       O
ATOM   5647  CB   MET  D  82    38.164  43.988 -19.378  1.00 17.58       C
ATOM   5648  CG   MET  D  82    37.460  44.997 -18.404  1.00 17.62       C
ATOM   5649  SD   MET  D  82    35.856  45.616 -19.021  1.00 20.81       S
ATOM   5650  CE   MET  D  82    34.981  44.064 -19.009  1.00 16.97       C
ATOM   5651  N    ASN  D  82A   40.868  41.693 -19.714  1.00 16.81       N
ATOM   5652  CA   ASN  D  82A   41.465  40.723 -20.630  1.00 16.29       C
ATOM   5653  C    ASN  D  82A   40.808  39.397 -20.424  1.00 16.92       C
ATOM   5654  O    ASN  D  82A   40.133  39.214 -19.409  1.00 14.60       O
ATOM   5655  CB   ASN  D  82A   42.959  40.563 -20.357  1.00 17.51       C
ATOM   5656  CG   ASN  D  82A   43.735  41.832 -20.611  1.00 18.21       C
ATOM   5657  OD1  ASN  D  82A   43.498  42.471 -21.582  1.00 14.50       O
ATOM   5658  ND2  ASN  D  82A   44.731  42.138 -19.760  1.00 20.30       N
ATOM   5659  N    ASN  D  82B   40.992  38.517 -21.419  1.00 16.06       N
ATOM   5660  CA   ASN  D  82B   40.568  37.111 -21.375  1.00 17.18       C
ATOM   5661  C    ASN  D  82B   39.076  37.055 -21.061  1.00 16.94       C
ATOM   5662  O    ASN  D  82B   38.621  36.323 -20.172  1.00 17.67       O
ATOM   5663  CB   ASN  D  82B   41.399  36.349 -20.346  1.00 19.18       C
ATOM   5664  CG   ASN  D  82B   40.993  34.886 -20.232  1.00 22.30       C
ATOM   5665  OD1  ASN  D  82B   40.988  34.292 -19.132  1.00 29.81       O
ATOM   5666  ND2  ASN  D  82B   40.635  34.299 -21.369  1.00 26.31       N
ATOM   5667  N    LEU  D  82C   38.319  37.864 -21.792  1.00 16.24       N
ATOM   5668  CA   LEU  D  82C   36.917  38.031 -21.511  1.00 15.07       C
ATOM   5669  C    LEU  D  82C   36.128  36.811 -21.926  1.00 15.34       C
ATOM   5670  O    LEU  D  82C   36.476  36.105 -22.869  1.00 15.41       O
ATOM   5671  CB   LEU  D  82C   36.357  39.307 -22.183  1.00 14.05       C
ATOM   5672  CG   LEU  D  82C   36.828  40.600 -21.483  1.00 14.00       C
ATOM   5673  CD1  LEU  D  82C   36.663  41.844 -22.354  1.00 15.07       C
ATOM   5674  CD2  LEU  D  82C   36.066  40.814 -20.171  1.00 16.28       C
ATOM   5675  N    ARG  D  83    35.087  36.538 -21.161  1.00 15.40       N
ATOM   5676  CA   ARG  D  83    34.237  35.404 -21.419  1.00 17.74       C
ATOM   5677  C    ARG  D  83    32.790  35.874 -21.392  1.00 17.30       C
ATOM   5678  O    ARG  D  83    32.510  37.012 -21.002  1.00 14.02       O
ATOM   5679  CB   ARG  D  83    34.568  34.304 -20.390  1.00 19.43       C
ATOM   5680  CG   ARG  D  83    36.069  33.901 -20.370  1.00 20.87       C
ATOM   5681  CD   ARG  D  83    36.494  33.133 -21.668  1.00 23.53       C
ATOM   5682  NE   ARG  D  83    37.940  33.044 -21.891  1.00 24.28       N
ATOM   5683  CZ   ARG  D  83    38.519  32.647 -23.027  1.00 25.10       C
ATOM   5684  NH1  ARG  D  83    37.810  32.251 -24.080  1.00 25.81       N
ATOM   5685  NH2  ARG  D  83    39.837  32.622 -23.123  1.00 26.91       N
ATOM   5686  N    THR  D  84    31.850  35.045 -21.830  1.00 17.37       N
ATOM   5687  CA   THR  D  84    30.463  35.510 -21.919  1.00 17.96       C
ATOM   5688  C    THR  D  84    29.905  36.020 -20.579  1.00 18.09       C
ATOM   5689  O    THR  D  84    29.189  37.022 -20.593  1.00 18.72       O
ATOM   5690  CB   THR  D  84    29.520  34.487 -22.555  1.00 18.81       C
ATOM   5691  OG1  THR  D  84    29.622  33.269 -21.829  1.00 19.53       O
ATOM   5692  CG2  THR  D  84    29.900  34.260 -24.005  1.00 18.94       C
ATOM   5693  N    GLU  D  85    30.284  35.358 -19.470  1.00 17.34       N
ATOM   5694  CA  AGLU  D  85    29.953  35.744 -18.087  0.50 18.10       C
ATOM   5695  CA  BGLU  D  85    29.916  35.771 -18.107  0.50 17.29       C
ATOM   5696  C    GLU  D  85    30.356  37.194 -17.745  1.00 16.76       C
ATOM   5697  O    GLU  D  85    29.919  37.758 -16.725  1.00 18.09       O
ATOM   5698  CB  AGLU  D  85    30.617  34.778 -17.071  0.50 18.78       C
ATOM   5699  CB  BGLU  D  85    30.528  34.841 -17.067  0.50 17.25       C
ATOM   5700  CG  AGLU  D  85    31.932  35.276 -16.415  0.50 20.07       C
ATOM   5701  CG  BGLU  D  85    32.005  34.561 -17.289  0.50 17.00       C
ATOM   5702  CD  AGLU  D  85    32.938  34.176 -16.087  0.50 20.87       C
ATOM   5703  CD  BGLU  D  85    32.221  33.239 -17.993  0.50 16.24       C
ATOM   5704  OE1 AGLU  D  85    33.681  33.754 -17.005  0.50 24.17       O
ATOM   5705  OE1 BGLU  D  85    31.617  33.017 -19.064  0.50 11.79       O
ATOM   5706  OE2 AGLU  D  85    33.015  33.748 -14.907  0.50 23.02       O
ATOM   5707  OE2 BGLU  D  85    32.997  32.432 -17.461  0.50 19.25       O
ATOM   5708  N    ASP  D  86    31.229  37.774 -18.548  1.00 17.03       N
```

| ATOM | 5709 | CA | ASP | D | 86 | 31.709 | 39.122 | -18.272 | 1.00 | 15.45 | C |
| ATOM | 5710 | C | ASP | D | 86 | 30.823 | 40.213 | -18.899 | 1.00 | 14.64 | C |
| ATOM | 5711 | O | ASP | D | 86 | 31.035 | 41.405 | -18.700 | 1.00 | 15.45 | O |
| ATOM | 5712 | CB | ASP | D | 86 | 33.144 | 39.249 | -18.733 | 1.00 | 15.92 | C |
| ATOM | 5713 | CG | ASP | D | 86 | 34.092 | 38.389 | -17.908 | 1.00 | 17.39 | C |
| ATOM | 5714 | OD1 | ASP | D | 86 | 34.063 | 38.477 | -16.649 | 1.00 | 15.93 | O |
| ATOM | 5715 | OD2 | ASP | D | 86 | 34.824 | 37.585 | -18.527 | 1.00 | 16.91 | O |
| ATOM | 5716 | N | THR | D | 87 | 29.849 | 39.801 | -19.684 | 1.00 | 14.59 | N |
| ATOM | 5717 | CA | THR | D | 87 | 28.864 | 40.720 | -20.284 | 1.00 | 12.63 | C |
| ATOM | 5718 | C | THR | D | 87 | 28.018 | 41.427 | -19.225 | 1.00 | 12.74 | C |
| ATOM | 5719 | O | THR | D | 87 | 27.550 | 40.811 | -18.291 | 1.00 | 11.82 | O |
| ATOM | 5720 | CB | THR | D | 87 | 27.923 | 39.928 | -21.208 | 1.00 | 12.15 | C |
| ATOM | 5721 | OG1 | THR | D | 87 | 28.682 | 39.392 | -22.287 | 1.00 | 11.17 | O |
| ATOM | 5722 | CG2 | THR | D | 87 | 26.821 | 40.795 | -21.749 | 1.00 | 11.08 | C |
| ATOM | 5723 | N | GLY | D | 88 | 27.820 | 42.744 | -19.356 | 1.00 | 10.12 | N |
| ATOM | 5724 | CA | GLY | D | 88 | 27.067 | 43.449 | -18.336 | 1.00 | 11.20 | C |
| ATOM | 5725 | C | GLY | D | 88 | 27.242 | 44.936 | -18.546 | 1.00 | 10.95 | C |
| ATOM | 5726 | O | GLY | D | 88 | 27.955 | 45.357 | -19.436 | 1.00 | 11.54 | O |
| ATOM | 5727 | N | ILE | D | 89 | 26.512 | 45.687 | -17.754 | 1.00 | 11.05 | N |
| ATOM | 5728 | CA | ILE | D | 89 | 26.655 | 47.145 | -17.655 | 1.00 | 12.07 | C |
| ATOM | 5729 | C | ILE | D | 89 | 27.651 | 47.387 | -16.549 | 1.00 | 11.55 | C |
| ATOM | 5730 | O | ILE | D | 89 | 27.424 | 46.945 | -15.419 | 1.00 | 13.53 | O |
| ATOM | 5731 | CB | ILE | D | 89 | 25.332 | 47.830 | -17.254 | 1.00 | 11.28 | C |
| ATOM | 5732 | CG1 | ILE | D | 89 | 24.211 | 47.530 | -18.254 | 1.00 | 14.87 | C |
| ATOM | 5733 | CG2 | ILE | D | 89 | 25.532 | 49.374 | -17.104 | 1.00 | 12.36 | C |
| ATOM | 5734 | CD1 | ILE | D | 89 | 24.540 | 47.875 | -19.659 | 1.00 | 18.95 | C |
| ATOM | 5735 | N | TYR | D | 90 | 28.728 | 48.100 | -16.886 | 1.00 | 10.58 | N |
| ATOM | 5736 | CA | TYR | D | 90 | 29.826 | 48.398 | -15.986 | 1.00 | 10.36 | C |
| ATOM | 5737 | C | TYR | D | 90 | 29.666 | 49.837 | -15.491 | 1.00 | 11.66 | C |
| ATOM | 5738 | O | TYR | D | 90 | 29.631 | 50.746 | -16.312 | 1.00 | 12.47 | O |
| ATOM | 5739 | CB | TYR | D | 90 | 31.142 | 48.251 | -16.711 | 1.00 | 10.05 | C |
| ATOM | 5740 | CG | TYR | D | 90 | 31.523 | 46.790 | -16.826 | 1.00 | 8.22 | C |
| ATOM | 5741 | CD1 | TYR | D | 90 | 30.782 | 45.932 | -17.618 | 1.00 | 11.37 | C |
| ATOM | 5742 | CD2 | TYR | D | 90 | 32.548 | 46.271 | -16.080 | 1.00 | 11.29 | C |
| ATOM | 5743 | CE1 | TYR | D | 90 | 31.065 | 44.552 | -17.681 | 1.00 | 9.89 | C |
| ATOM | 5744 | CE2 | TYR | D | 90 | 32.888 | 44.900 | -16.192 | 1.00 | 9.73 | C |
| ATOM | 5745 | CZ | TYR | D | 90 | 32.125 | 44.069 | -16.992 | 1.00 | 11.89 | C |
| ATOM | 5746 | OH | TYR | D | 90 | 32.509 | 42.727 | -17.044 | 1.00 | 10.15 | O |
| ATOM | 5747 | N | TYR | D | 91 | 29.516 | 49.969 | -14.176 | 1.00 | 11.60 | N |
| ATOM | 5748 | CA | TYR | D | 91 | 29.395 | 51.265 | -13.478 | 1.00 | 12.19 | C |
| ATOM | 5749 | C | TYR | D | 91 | 30.642 | 51.538 | -12.673 | 1.00 | 13.29 | C |
| ATOM | 5750 | O | TYR | D | 91 | 31.182 | 50.672 | -11.920 | 1.00 | 13.38 | O |
| ATOM | 5751 | CB | TYR | D | 91 | 28.260 | 51.271 | -12.470 | 1.00 | 13.85 | C |
| ATOM | 5752 | CG | TYR | D | 91 | 26.876 | 51.077 | -12.974 | 1.00 | 14.85 | C |
| ATOM | 5753 | CD1 | TYR | D | 91 | 26.100 | 52.154 | -13.396 | 1.00 | 12.40 | C |
| ATOM | 5754 | CD2 | TYR | D | 91 | 26.306 | 49.811 | -12.995 | 1.00 | 15.46 | C |
| ATOM | 5755 | CE1 | TYR | D | 91 | 24.791 | 51.954 | -13.815 | 1.00 | 14.04 | C |
| ATOM | 5756 | CE2 | TYR | D | 91 | 25.027 | 49.603 | -13.434 | 1.00 | 15.82 | C |
| ATOM | 5757 | CZ | TYR | D | 91 | 24.265 | 50.673 | -13.841 | 1.00 | 15.52 | C |
| ATOM | 5758 | OH | TYR | D | 91 | 22.984 | 50.436 | -14.261 | 1.00 | 16.21 | O |
| ATOM | 5759 | N | CYS | D | 92 | 31.082 | 52.777 | -12.763 | 1.00 | 14.71 | N |
| ATOM | 5760 | CA | CYS | D | 92 | 31.906 | 53.308 | -11.735 | 1.00 | 15.39 | C |
| ATOM | 5761 | C | CYS | D | 92 | 31.056 | 53.565 | -10.492 | 1.00 | 14.67 | C |
| ATOM | 5762 | O | CYS | D | 92 | 29.982 | 54.211 | -10.547 | 1.00 | 15.55 | O |
| ATOM | 5763 | CB | CYS | D | 92 | 32.584 | 54.590 | -12.192 | 1.00 | 18.86 | C |
| ATOM | 5764 | SG | CYS | D | 92 | 33.899 | 54.232 | -13.350 | 1.00 | 23.47 | S |
| ATOM | 5765 | N | PHE | D | 93 | 31.587 | 53.090 | -9.381 | 1.00 | 12.05 | N |
| ATOM | 5766 | CA | PHE | D | 93 | 30.963 | 53.174 | -8.079 | 1.00 | 12.44 | C |
| ATOM | 5767 | C | PHE | D | 93 | 31.997 | 53.678 | -7.125 | 1.00 | 13.79 | C |
| ATOM | 5768 | O | PHE | D | 93 | 32.974 | 53.010 | -6.856 | 1.00 | 12.95 | O |
| ATOM | 5769 | CB | PHE | D | 93 | 30.453 | 51.790 | -7.676 | 1.00 | 12.20 | C |
| ATOM | 5770 | CG | PHE | D | 93 | 29.873 | 51.722 | -6.300 | 1.00 | 11.50 | C |
| ATOM | 5771 | CD1 | PHE | D | 93 | 28.847 | 52.576 | -5.915 | 1.00 | 12.91 | C |
| ATOM | 5772 | CD2 | PHE | D | 93 | 30.316 | 50.765 | -5.388 | 1.00 | 10.89 | C |
| ATOM | 5773 | CE1 | PHE | D | 93 | 28.308 | 52.517 | -4.639 | 1.00 | 11.44 | C |

```
ATOM   5774  CE2 PHE D  93     29.763  50.714   -4.130  1.00  13.19        C
ATOM   5775  CZ  PHE D  93     28.759  51.591   -3.766  1.00   8.84        C
ATOM   5776  N   LEU D  94     31.815  54.919   -6.651  1.00  13.51        N
ATOM   5777  CA  LEU D  94     32.621  55.492   -5.596  1.00  14.23        C
ATOM   5778  C   LEU D  94     31.987  55.066   -4.276  1.00  14.56        C
ATOM   5779  O   LEU D  94     30.965  55.602   -3.850  1.00  15.64        O
ATOM   5780  CB  LEU D  94     32.611  57.037   -5.713  1.00  13.99        C
ATOM   5781  CG  LEU D  94     33.523  57.593   -6.778  1.00  16.10        C
ATOM   5782  CD1 LEU D  94     33.233  57.026   -8.133  1.00  17.81        C
ATOM   5783  CD2 LEU D  94     33.390  59.131   -6.845  1.00  16.55        C
ATOM   5784  N   PRO D  95     32.552  54.052   -3.640  1.00  15.28        N
ATOM   5785  CA  PRO D  95     31.890  53.538   -2.462  1.00  15.74        C
ATOM   5786  C   PRO D  95     31.762  54.599   -1.366  1.00  16.59        C
ATOM   5787  O   PRO D  95     32.777  55.264   -1.067  1.00  15.69        O
ATOM   5788  CB  PRO D  95     32.807  52.422   -2.001  1.00  16.16        C
ATOM   5789  CG  PRO D  95     33.825  52.260   -3.028  1.00  15.54        C
ATOM   5790  CD  PRO D  95     33.822  53.364   -3.932  1.00  15.68        C
ATOM   5791  N   MET D  96     30.591  54.779   -0.742  1.00  17.18        N
ATOM   5792  CA  MET D  96     29.323  54.052   -0.956  1.00  16.97        C
ATOM   5793  C   MET D  96     28.276  54.966   -1.632  1.00  16.75        C
ATOM   5794  O   MET D  96     27.128  54.579   -1.824  1.00  16.23        O
ATOM   5795  CB  MET D  96     28.750  53.625    0.398  1.00  15.70        C
ATOM   5796  CG  MET D  96     29.594  52.681    1.207  1.00  16.34        C
ATOM   5797  SD  MET D  96     29.861  51.161    0.283  1.00  17.68        S
ATOM   5798  CE  MET D  96     28.286  50.286    0.374  1.00  16.89        C
ATOM   5799  N   ASP D 101     28.674  56.185   -1.996  1.00  18.06        N
ATOM   5800  CA  ASP D 101     27.710  57.258   -2.270  1.00  19.07        C
ATOM   5801  C   ASP D 101     27.339  57.552   -3.711  1.00  18.37        C
ATOM   5802  O   ASP D 101     26.279  58.144   -3.953  1.00  17.30        O
ATOM   5803  CB  ASP D 101     28.217  58.625   -1.729  1.00  21.03        C
ATOM   5804  CG  ASP D 101     28.394  58.665   -0.233  1.00  24.62        C
ATOM   5805  OD1 ASP D 101     28.059  57.694    0.461  1.00  29.25        O
ATOM   5806  OD2 ASP D 101     28.909  59.699    0.260  1.00  26.87        O
ATOM   5807  N   TYR D 102     28.204  57.220   -4.672  1.00  17.69        N
ATOM   5808  CA  TYR D 102     27.985  57.642   -6.042  1.00  17.25        C
ATOM   5809  C   TYR D 102     28.136  56.519   -7.034  1.00  16.24        C
ATOM   5810  O   TYR D 102     29.093  55.782   -6.953  1.00  14.84        O
ATOM   5811  CB  TYR D 102     28.991  58.712   -6.425  1.00  21.10        C
ATOM   5812  CG  TYR D 102     28.979  59.882   -5.469  1.00  21.50        C
ATOM   5813  CD1 TYR D 102     27.972  60.815   -5.526  1.00  23.93        C
ATOM   5814  CD2 TYR D 102     29.965  60.026   -4.505  1.00  22.92        C
ATOM   5815  CE1 TYR D 102     27.948  61.886   -4.657  1.00  23.70        C
ATOM   5816  CE2 TYR D 102     29.950  61.097   -3.609  1.00  22.95        C
ATOM   5817  CZ  TYR D 102     28.939  62.009   -3.694  1.00  23.55        C
ATOM   5818  OH  TYR D 102     28.895  63.074   -2.834  1.00  25.65        O
ATOM   5819  N   TRP D 103     27.213  56.487   -7.987  1.00  16.46        N
ATOM   5820  CA  TRP D 103     27.216  55.654   -9.172  1.00  16.04        C
ATOM   5821  C   TRP D 103     27.213  56.464  -10.461  1.00  17.30        C
ATOM   5822  O   TRP D 103     26.550  57.523  -10.567  1.00  16.94        O
ATOM   5823  CB  TRP D 103     25.957  54.813   -9.213  1.00  14.91        C
ATOM   5824  CG  TRP D 103     25.776  53.875   -8.119  1.00  13.89        C
ATOM   5825  CD1 TRP D 103     25.339  54.156   -6.884  1.00  12.06        C
ATOM   5826  CD2 TRP D 103     25.961  52.447   -8.171  1.00  13.75        C
ATOM   5827  NE1 TRP D 103     25.246  53.007   -6.134  1.00  13.57        N
ATOM   5828  CE2 TRP D 103     25.613  51.942   -6.905  1.00  13.26        C
ATOM   5829  CE3 TRP D 103     26.371  51.558   -9.166  1.00  15.39        C
ATOM   5830  CZ2 TRP D 103     25.683  50.584   -6.585  1.00  14.07        C
ATOM   5831  CZ3 TRP D 103     26.421  50.183   -8.854  1.00  14.46        C
ATOM   5832  CH2 TRP D 103     26.099  49.722   -7.568  1.00  14.32        C
ATOM   5833  N   GLY D 104     27.898  55.938  -11.474  1.00  17.01        N
ATOM   5834  CA  GLY D 104     27.883  56.546  -12.803  1.00  17.01        C
ATOM   5835  C   GLY D 104     26.666  56.145  -13.610  1.00  16.59        C
ATOM   5836  O   GLY D 104     25.664  55.745  -13.039  1.00  17.12        O
ATOM   5837  N   GLN D 105     26.730  56.261  -14.936  1.00  17.26        N
ATOM   5838  CA AGLN D 105     25.565  55.948  -15.746  0.50  17.58        C
```

```
ATOM   5839  CA BGLN D 105      25.587  55.967 -15.801  0.50 17.79           C
ATOM   5840  C   GLN D 105      25.664  54.568 -16.402  1.00 17.56           C
ATOM   5841  O   GLN D 105      24.690  54.092 -16.953  1.00 17.67           O
ATOM   5842  CB AGLN D 105      25.284  57.069 -16.756  0.50 19.42           C
ATOM   5843  CB BGLN D 105      25.461  56.994 -16.937  0.50 19.89           C
ATOM   5844  CG AGLN D 105      24.675  58.332 -16.087  0.50 20.66           C
ATOM   5845  CG BGLN D 105      25.155  58.426 -16.470  0.50 22.12           C
ATOM   5846  CD AGLN D 105      23.259  58.099 -15.584  0.50 21.39           C
ATOM   5847  CD BGLN D 105      26.382  59.199 -16.083  0.50 23.14           C
ATOM   5848  OE1AGLN D 105      22.980  58.216 -14.386  0.50 19.02           O
ATOM   5849  OE1BGLN D 105      26.317  60.150 -15.294  0.50 24.39           O
ATOM   5850  NE2AGLN D 105      22.358  57.743 -16.501  0.50 22.86           N
ATOM   5851  NE2BGLN D 105      27.521  58.799 -16.628  0.50 26.13           N
ATOM   5852  N   GLY D 106      26.834  53.942 -16.283  1.00 15.86           N
ATOM   5853  CA  GLY D 106      27.110  52.590 -16.814  1.00 15.20           C
ATOM   5854  C   GLY D 106      27.509  52.637 -18.270  1.00 14.52           C
ATOM   5855  O   GLY D 106      27.015  53.476 -19.010  1.00 13.60           O
ATOM   5856  N   THR D 107      28.426  51.759 -18.677  1.00 12.79           N
ATOM   5857  CA  THR D 107      28.718  51.560 -20.086  1.00 12.76           C
ATOM   5858  C   THR D 107      28.585  50.061 -20.354  1.00 12.95           C
ATOM   5859  O   THR D 107      28.953  49.272 -19.513  1.00  9.53           O
ATOM   5860  CB  THR D 107      30.095  52.123 -20.487  1.00 13.97           C
ATOM   5861  OG1 THR D 107      30.285  51.998 -21.900  1.00 14.97           O
ATOM   5862  CG2 THR D 107      31.225  51.474 -19.763  1.00 16.27           C
ATOM   5863  N   SER D 108      28.007  49.694 -21.490  1.00 11.18           N
ATOM   5864  CA  SER D 108      27.698  48.248 -21.731  1.00 11.25           C
ATOM   5865  C   SER D 108      28.820  47.532 -22.431  1.00 11.24           C
ATOM   5866  O   SER D 108      29.367  48.029 -23.418  1.00  9.14           O
ATOM   5867  CB  SER D 108      26.456  48.212 -22.601  1.00 14.58           C
ATOM   5868  OG  SER D 108      25.943  46.894 -22.748  1.00 16.48           O
ATOM   5869  N   VAL D 109      29.148  46.304 -21.975  1.00 10.99           N
ATOM   5870  CA  VAL D 109      30.194  45.535 -22.568  1.00 11.43           C
ATOM   5871  C   VAL D 109      29.546  44.191 -22.865  1.00 11.04           C
ATOM   5872  O   VAL D 109      28.955  43.600 -21.977  1.00 10.01           O
ATOM   5873  CB  VAL D 109      31.386  45.277 -21.585  1.00 12.27           C
ATOM   5874  CG1 VAL D 109      32.343  44.230 -22.158  1.00 14.31           C
ATOM   5875  CG2 VAL D 109      32.136  46.579 -21.268  1.00 12.73           C
ATOM   5876  N   THR D 110      29.562  43.813 -24.131  1.00 12.43           N
ATOM   5877  CA  THR D 110      29.050  42.497 -24.566  1.00 10.89           C
ATOM   5878  C   THR D 110      30.231  41.676 -25.025  1.00 10.15           C
ATOM   5879  O   THR D 110      31.037  42.116 -25.839  1.00  7.65           O
ATOM   5880  CB  THR D 110      27.982  42.632 -25.698  1.00 12.33           C
ATOM   5881  OG1 THR D 110      26.852  43.401 -25.237  1.00 17.43           O
ATOM   5882  CG2 THR D 110      27.535  41.267 -26.230  1.00 14.05           C
ATOM   5883  N   VAL D 111      30.293  40.409 -24.549  1.00 11.78           N
ATOM   5884  CA  VAL D 111      31.269  39.470 -24.994  1.00 13.17           C
ATOM   5885  C   VAL D 111      30.484  38.355 -25.691  1.00 14.47           C
ATOM   5886  O   VAL D 111      29.663  37.689 -25.052  1.00 14.22           O
ATOM   5887  CB  VAL D 111      32.056  38.840 -23.821  1.00 12.56           C
ATOM   5888  CG1 VAL D 111      33.127  37.957 -24.347  1.00 15.32           C
ATOM   5889  CG2 VAL D 111      32.622  39.907 -22.889  1.00 14.43           C
ATOM   5890  N   SER D 112      30.723  38.214 -26.993  1.00 13.68           N
ATOM   5891  CA  SER D 112      29.837  37.464 -27.885  1.00 13.24           C
ATOM   5892  C   SER D 112      30.569  37.055 -29.144  1.00 13.80           C
ATOM   5893  O   SER D 112      31.465  37.758 -29.623  1.00 13.28           O
ATOM   5894  CB  SER D 112      28.643  38.340 -28.249  1.00 12.00           C
ATOM   5895  OG  SER D 112      27.778  37.753 -29.189  1.00 13.00           O
ATOM   5896  N   SER D 113      30.204  35.894 -29.671  1.00 14.26           N
ATOM   5897  CA  SER D 113      30.716  35.432 -30.961  1.00 15.46           C
ATOM   5898  C   SER D 113      29.821  35.924 -32.114  1.00 17.03           C
ATOM   5899  O   SER D 113      30.106  35.672 -33.270  1.00 20.15           O
ATOM   5900  CB  SER D 113      30.817  33.886 -30.961  1.00 14.73           C
ATOM   5901  OG  SER D 113      29.564  33.278 -30.761  1.00 16.08           O
ATOM   5902  N   ALA D 114      28.754  36.660 -31.805  1.00 16.98           N
ATOM   5903  CA  ALA D 114      27.883  37.213 -32.811  1.00 17.53           C
```

| ATOM | 5904 | C   | ALA D 114 | 28.415 | 38.564 | -33.258 | 1.00 | 18.39 | C |
|------|------|-----|-----------|--------|--------|---------|------|-------|---|
| ATOM | 5905 | O   | ALA D 114 | 29.232 | 39.183 | -32.560 | 1.00 | 18.76 | O |
| ATOM | 5906 | CB  | ALA D 114 | 26.455 | 37.302 | -32.300 | 1.00 | 17.58 | C |
| ATOM | 5907 | N   | LYS D 115 | 27.976 | 39.005 | -34.426 | 1.00 | 17.66 | N |
| ATOM | 5908 | CA  | LYS D 115 | 28.468 | 40.252 | -35.003 | 1.00 | 20.05 | C |
| ATOM | 5909 | C   | LYS D 115 | 27.554 | 41.406 | -34.646 | 1.00 | 16.80 | C |
| ATOM | 5910 | O   | LYS D 115 | 26.364 | 41.251 | -34.533 | 1.00 | 19.03 | O |
| ATOM | 5911 | CB  | LYS D 115 | 28.596 | 40.134 | -36.527 | 1.00 | 23.11 | C |
| ATOM | 5912 | CG  | LYS D 115 | 29.355 | 38.856 | -36.987 | 1.00 | 28.03 | C |
| ATOM | 5913 | CD  | LYS D 115 | 28.359 | 37.731 | -37.383 | 1.00 | 29.98 | C |
| ATOM | 5914 | CE  | LYS D 115 | 28.999 | 36.310 | -37.353 | 1.00 | 30.17 | C |
| ATOM | 5915 | NZ  | LYS D 115 | 28.270 | 35.334 | -38.234 | 1.00 | 32.13 | N |
| ATOM | 5916 | N   | THR D 116 | 28.126 | 42.579 | -34.477 | 1.00 | 15.25 | N |
| ATOM | 5917 | CA  | THR D 116 | 27.314 | 43.766 | -34.337 | 1.00 | 13.85 | C |
| ATOM | 5918 | C   | THR D 116 | 26.552 | 44.041 | -35.619 | 1.00 | 13.45 | C |
| ATOM | 5919 | O   | THR D 116 | 27.128 | 44.037 | -36.719 | 1.00 | 12.33 | O |
| ATOM | 5920 | CB  | THR D 116 | 28.198 | 44.920 | -33.972 | 1.00 | 13.10 | C |
| ATOM | 5921 | OG1 | THR D 116 | 28.759 | 44.618 | -32.705 | 1.00 | 12.75 | O |
| ATOM | 5922 | CG2 | THR D 116 | 27.468 | 46.271 | -33.942 | 1.00 | 13.15 | C |
| ATOM | 5923 | N   | THR D 117 | 25.254 | 44.308 | -35.469 | 1.00 | 14.85 | N |
| ATOM | 5924 | CA  | THR D 117 | 24.391 | 44.739 | -36.590 | 1.00 | 13.35 | C |
| ATOM | 5925 | C   | THR D 117 | 23.654 | 45.956 | -36.132 | 1.00 | 12.45 | C |
| ATOM | 5926 | O   | THR D 117 | 23.043 | 45.924 | -35.113 | 1.00 | 11.21 | O |
| ATOM | 5927 | CB  | THR D 117 | 23.381 | 43.642 | -36.929 | 1.00 | 16.05 | C |
| ATOM | 5928 | OG1 | THR D 117 | 24.100 | 42.434 | -37.190 | 1.00 | 16.27 | O |
| ATOM | 5929 | CG2 | THR D 117 | 22.527 | 43.993 | -38.157 | 1.00 | 16.61 | C |
| ATOM | 5930 | N   | PRO D 118 | 23.660 | 47.025 | -36.919 | 1.00 | 10.67 | N |
| ATOM | 5931 | CA  | PRO D 118 | 22.979 | 48.243 | -36.451 | 1.00 | 11.94 | C |
| ATOM | 5932 | C   | PRO D 118 | 21.460 | 48.100 | -36.659 | 1.00 | 13.73 | C |
| ATOM | 5933 | O   | PRO D 118 | 21.054 | 47.240 | -37.438 | 1.00 | 14.52 | O |
| ATOM | 5934 | CB  | PRO D 118 | 23.535 | 49.297 | -37.389 | 1.00 | 10.22 | C |
| ATOM | 5935 | CG  | PRO D 118 | 23.669 | 48.577 | -38.702 | 1.00 | 10.35 | C |
| ATOM | 5936 | CD  | PRO D 118 | 24.179 | 47.183 | -38.278 | 1.00 | 11.61 | C |
| ATOM | 5937 | N   | PRO D 119 | 20.629 | 48.915 | -35.986 | 1.00 | 12.83 | N |
| ATOM | 5938 | CA  | PRO D 119 | 19.199 | 48.696 | -36.195 | 1.00 | 13.65 | C |
| ATOM | 5939 | C   | PRO D 119 | 18.612 | 49.273 | -37.499 | 1.00 | 14.21 | C |
| ATOM | 5940 | O   | PRO D 119 | 19.155 | 50.241 | -38.086 | 1.00 | 15.05 | O |
| ATOM | 5941 | CB  | PRO D 119 | 18.566 | 49.374 | -34.984 | 1.00 | 14.37 | C |
| ATOM | 5942 | CG  | PRO D 119 | 19.521 | 50.531 | -34.687 | 1.00 | 14.80 | C |
| ATOM | 5943 | CD  | PRO D 119 | 20.912 | 50.009 | -35.032 | 1.00 | 15.41 | C |
| ATOM | 5944 | N   | SER D 120 | 17.497 | 48.665 | -37.917 | 1.00 | 13.92 | N |
| ATOM | 5945 | CA  | SER D 120 | 16.592 | 49.224 | -38.907 | 1.00 | 13.27 | C |
| ATOM | 5946 | C   | SER D 120 | 15.446 | 49.911 | -38.167 | 1.00 | 14.72 | C |
| ATOM | 5947 | O   | SER D 120 | 14.836 | 49.340 | -37.302 | 1.00 | 16.29 | O |
| ATOM | 5948 | CB  | SER D 120 | 16.087 | 48.089 | -39.812 | 1.00 | 14.38 | C |
| ATOM | 5949 | OG  | SER D 120 | 17.181 | 47.631 | -40.592 | 1.00 | 12.54 | O |
| ATOM | 5950 | N   | VAL D 121 | 15.196 | 51.180 | -38.477 | 1.00 | 14.85 | N |
| ATOM | 5951 | CA  | VAL D 121 | 14.170 | 51.955 | -37.773 | 1.00 | 15.34 | C |
| ATOM | 5952 | C   | VAL D 121 | 12.986 | 52.223 | -38.730 | 1.00 | 15.42 | C |
| ATOM | 5953 | O   | VAL D 121 | 13.138 | 52.806 | -39.811 | 1.00 | 13.30 | O |
| ATOM | 5954 | CB  | VAL D 121 | 14.754 | 53.256 | -37.218 | 1.00 | 15.19 | C |
| ATOM | 5955 | CG1 | VAL D 121 | 13.765 | 53.941 | -36.304 | 1.00 | 17.55 | C |
| ATOM | 5956 | CG2 | VAL D 121 | 16.101 | 52.960 | -36.488 | 1.00 | 17.19 | C |
| ATOM | 5957 | N   | TYR D 122 | 11.820 | 51.744 | -38.333 | 1.00 | 15.16 | N |
| ATOM | 5958 | CA  | TYR D 122 | 10.600 | 51.913 | -39.121 | 1.00 | 15.85 | C |
| ATOM | 5959 | C   | TYR D 122 | 9.601  | 52.780 | -38.386 | 1.00 | 16.54 | C |
| ATOM | 5960 | O   | TYR D 122 | 9.382  | 52.620 | -37.175 | 1.00 | 16.23 | O |
| ATOM | 5961 | CB  | TYR D 122 | 9.968  | 50.553 | -39.387 | 1.00 | 15.76 | C |
| ATOM | 5962 | CG  | TYR D 122 | 10.895 | 49.581 | -40.039 | 1.00 | 14.85 | C |
| ATOM | 5963 | CD1 | TYR D 122 | 11.481 | 49.880 | -41.240 | 1.00 | 16.01 | C |
| ATOM | 5964 | CD2 | TYR D 122 | 11.170 | 48.336 | -39.445 | 1.00 | 16.41 | C |
| ATOM | 5965 | CE1 | TYR D 122 | 12.340 | 48.982 | -41.874 | 1.00 | 15.07 | C |
| ATOM | 5966 | CE2 | TYR D 122 | 12.014 | 47.433 | -40.066 | 1.00 | 16.74 | C |
| ATOM | 5967 | CZ  | TYR D 122 | 12.591 | 47.762 | -41.284 | 1.00 | 15.39 | C |
| ATOM | 5968 | OH  | TYR D 122 | 13.445 | 46.900 | -41.916 | 1.00 | 17.10 | O |

```
ATOM    5969  N    PRO D 123     8.944  53.689 -39.118  1.00 17.40      N
ATOM    5970  CA   PRO D 123     7.898  54.478 -38.477  1.00 17.29      C
ATOM    5971  C    PRO D 123     6.650  53.658 -38.198  1.00 17.39      C
ATOM    5972  O    PRO D 123     6.302  52.774 -38.975  1.00 19.76      O
ATOM    5973  CB   PRO D 123     7.601  55.564 -39.520  1.00 16.83      C
ATOM    5974  CG   PRO D 123     7.976  54.934 -40.826  1.00 18.03      C
ATOM    5975  CD   PRO D 123     9.110  54.034 -40.548  1.00 17.67      C
ATOM    5976  N    LEU D 124     5.972  53.958 -37.105  1.00 15.61      N
ATOM    5977  CA   LEU D 124     4.670  53.377 -36.811  1.00 17.87      C
ATOM    5978  C    LEU D 124     3.632  54.500 -36.789  1.00 17.30      C
ATOM    5979  O    LEU D 124     3.626  55.310 -35.890  1.00 18.25      O
ATOM    5980  CB   LEU D 124     4.684  52.657 -35.475  1.00 17.51      C
ATOM    5981  CG   LEU D 124     5.514  51.373 -35.331  1.00 19.46      C
ATOM    5982  CD1  LEU D 124     5.629  50.935 -33.902  1.00 18.53      C
ATOM    5983  CD2  LEU D 124     4.946  50.283 -36.140  1.00 20.50      C
ATOM    5984  N    ALA D 125     2.808  54.558 -37.825  1.00 20.23      N
ATOM    5985  CA   ALA D 125     1.844  55.673 -37.999  1.00 21.89      C
ATOM    5986  C    ALA D 125     0.511  55.000 -38.200  1.00 23.67      C
ATOM    5987  O    ALA D 125     0.473  53.917 -38.739  1.00 19.64      O
ATOM    5988  CB   ALA D 125     2.181  56.494 -39.182  1.00 21.89      C
ATOM    5989  N    PRO D 126    -0.584  55.624 -37.736  1.00 25.84      N
ATOM    5990  CA   PRO D 126    -1.945  55.108 -37.969  1.00 27.43      C
ATOM    5991  C    PRO D 126    -2.302  54.867 -39.439  1.00 28.31      C
ATOM    5992  O    PRO D 126    -2.043  55.724 -40.271  1.00 27.97      O
ATOM    5993  CB   PRO D 126    -2.832  56.235 -37.422  1.00 27.23      C
ATOM    5994  CG   PRO D 126    -2.024  56.879 -36.409  1.00 26.56      C
ATOM    5995  CD   PRO D 126    -0.603  56.830 -36.900  1.00 26.42      C
ATOM    5996  N    GLY D 127    -2.895  53.712 -39.738  1.00 29.90      N
ATOM    5997  CA   GLY D 127    -3.457  53.421 -41.076  1.00 30.43      C
ATOM    5998  C    GLY D 127    -4.190  54.575 -41.761  1.00 31.87      C
ATOM    5999  O    GLY D 127    -5.059  55.219 -41.162  1.00 33.13      O
ATOM    6000  N    SER D 136    -8.770  62.804 -30.228  1.00 35.42      N
ATOM    6001  CA   SER D 136    -8.133  63.859 -29.424  1.00 35.12      C
ATOM    6002  C    SER D 136    -6.640  63.584 -29.113  1.00 34.88      C
ATOM    6003  O    SER D 136    -5.809  64.498 -29.108  1.00 33.93      O
ATOM    6004  CB   SER D 136    -8.906  64.049 -28.112  1.00 35.75      C
ATOM    6005  OG   SER D 136    -8.086  64.605 -27.093  1.00 36.86      O
ATOM    6006  N    MET D 137    -6.325  62.329 -28.806  1.00 33.92      N
ATOM    6007  CA   MET D 137    -4.930  61.896 -28.717  1.00 32.93      C
ATOM    6008  C    MET D 137    -4.570  61.115 -29.975  1.00 31.57      C
ATOM    6009  O    MET D 137    -5.424  60.483 -30.586  1.00 31.17      O
ATOM    6010  CB   MET D 137    -4.714  61.027 -27.472  1.00 34.98      C
ATOM    6011  CG   MET D 137    -4.948  61.755 -26.132  1.00 35.38      C
ATOM    6012  SD   MET D 137    -3.935  63.236 -25.923  1.00 37.15      S
ATOM    6013  CE   MET D 137    -2.767  62.681 -24.690  1.00 36.92      C
ATOM    6014  N    VAL D 138    -3.308  61.169 -30.383  1.00 29.43      N
ATOM    6015  CA   VAL D 138    -2.834  60.224 -31.389  1.00 27.83      C
ATOM    6016  C    VAL D 138    -1.608  59.503 -30.821  1.00 26.16      C
ATOM    6017  O    VAL D 138    -0.786  60.128 -30.157  1.00 26.47      O
ATOM    6018  CB   VAL D 138    -2.531  60.889 -32.765  1.00 27.72      C
ATOM    6019  CG1  VAL D 138    -1.501  61.998 -32.655  1.00 27.86      C
ATOM    6020  CG2  VAL D 138    -2.064  59.836 -33.758  1.00 28.63      C
ATOM    6021  N    THR D 139    -1.530  58.192 -31.062  1.00 22.93      N
ATOM    6022  CA   THR D 139    -0.356  57.384 -30.671  1.00 21.82      C
ATOM    6023  C    THR D 139     0.463  56.955 -31.890  1.00 20.39      C
ATOM    6024  O    THR D 139    -0.044  56.318 -32.835  1.00 19.58      O
ATOM    6025  CB   THR D 139    -0.756  56.171 -29.843  1.00 21.60      C
ATOM    6026  OG1  THR D 139    -1.375  56.618 -28.637  1.00 19.69      O
ATOM    6027  CG2  THR D 139     0.466  55.348 -29.464  1.00 21.20      C
ATOM    6028  N    LEU D 140     1.731  57.334 -31.865  1.00 20.32      N
ATOM    6029  CA   LEU D 140     2.693  56.966 -32.905  1.00 19.90      C
ATOM    6030  C    LEU D 140     3.765  56.116 -32.265  1.00 19.74      C
ATOM    6031  O    LEU D 140     3.746  55.894 -31.050  1.00 19.12      O
ATOM    6032  CB   LEU D 140     3.337  58.207 -33.483  1.00 20.44      C
ATOM    6033  CG   LEU D 140     2.355  59.305 -33.873  1.00 21.16      C
```

```
ATOM   6034  CD1 LEU D 140      3.106  60.548 -34.291  1.00 22.30          C
ATOM   6035  CD2 LEU D 140      1.445  58.835 -34.973  1.00 21.47          C
ATOM   6036  N   GLY D 141      4.719  55.660 -33.078  1.00 18.04          N
ATOM   6037  CA  GLY D 141      5.854  54.951 -32.537  1.00 17.75          C
ATOM   6038  C   GLY D 141      6.970  54.696 -33.527  1.00 16.55          C
ATOM   6039  O   GLY D 141      6.905  55.124 -34.674  1.00 14.28          O
ATOM   6040  N   CYS D 142      8.009  54.015 -33.044  1.00 17.21          N
ATOM   6041  CA  CYS D 142      9.095  53.576 -33.888  1.00 18.15          C
ATOM   6042  C   CYS D 142      9.363  52.133 -33.558  1.00 16.59          C
ATOM   6043  O   CYS D 142      9.310  51.738 -32.374  1.00 15.34          O
ATOM   6044  CB  CYS D 142     10.350  54.412 -33.692  1.00 20.95          C
ATOM   6045  SG  CYS D 142     10.343  55.845 -34.786  1.00 31.05          S
ATOM   6046  N   LEU D 143      9.565  51.356 -34.610  1.00 14.65          N
ATOM   6047  CA  LEU D 143      9.958  49.951 -34.506  1.00 14.89          C
ATOM   6048  C   LEU D 143     11.439  49.876 -34.860  1.00 14.36          C
ATOM   6049  O   LEU D 143     11.888  50.378 -35.905  1.00 15.41          O
ATOM   6050  CB  LEU D 143      9.071  49.072 -35.404  1.00 14.10          C
ATOM   6051  CG  LEU D 143      9.385  47.566 -35.548  1.00 14.49          C
ATOM   6052  CD1 LEU D 143      9.167  46.863 -34.259  1.00 14.02          C
ATOM   6053  CD2 LEU D 143      8.484  47.019 -36.639  1.00 17.31          C
ATOM   6054  N   VAL D 144     12.197  49.285 -33.943  1.00 15.33          N
ATOM   6055  CA  VAL D 144     13.647  49.204 -34.004  1.00 13.56          C
ATOM   6056  C   VAL D 144     13.993  47.721 -34.047  1.00 14.93          C
ATOM   6057  O   VAL D 144     13.841  47.001 -33.068  1.00 13.04          O
ATOM   6058  CB  VAL D 144     14.291  49.871 -32.786  1.00 15.30          C
ATOM   6059  CG1 VAL D 144     15.810  49.888 -32.889  1.00 16.16          C
ATOM   6060  CG2 VAL D 144     13.807  51.333 -32.651  1.00 12.67          C
ATOM   6061  N   LYS D 145     14.489  47.304 -35.196  1.00 14.81          N
ATOM   6062  CA  LYS D 145     14.558  45.893 -35.535  1.00 15.44          C
ATOM   6063  C   LYS D 145     15.917  45.483 -36.092  1.00 14.15          C
ATOM   6064  O   LYS D 145     16.592  46.255 -36.779  1.00 14.29          O
ATOM   6065  CB  LYS D 145     13.457  45.644 -36.575  1.00 17.51          C .
ATOM   6066  CG  LYS D 145     13.130  44.229 -36.772  1.00 20.38          C
ATOM   6067  CD  LYS D 145     11.756  44.051 -37.361  1.00 20.05          C
ATOM   6068  CE  LYS D 145     11.614  42.582 -37.690  1.00 20.96          C
ATOM   6069  NZ  LYS D 145     11.722  41.832 -36.445  1.00 21.26          N
ATOM   6070  N   GLY D 146     16.316  44.235 -35.841  1.00 15.23          N
ATOM   6071  CA  GLY D 146     17.420  43.676 -36.578  1.00 14.58          C
ATOM   6072  C   GLY D 146     18.787  44.048 -36.060  1.00 13.60          C
ATOM   6073  O   GLY D 146     19.762  43.974 -36.793  1.00 16.81          O
ATOM   6074  N   TYR D 147     18.874  44.435 -34.796  1.00 13.82          N
ATOM   6075  CA  TYR D 147     20.149  44.859 -34.226  1.00 12.74          C
ATOM   6076  C   TYR D 147     20.722  43.901 -33.202  1.00 12.81          C
ATOM   6077  O   TYR D 147     20.007  43.018 -32.644  1.00 15.12          O
ATOM   6078  CB  TYR D 147     20.065  46.291 -33.620  1.00 12.79          C
ATOM   6079  CG  TYR D 147     19.279  46.374 -32.330  1.00 13.67          C
ATOM   6080  CD1 TYR D 147     17.898  46.517 -32.348  1.00 12.53          C
ATOM   6081  CD2 TYR D 147     19.914  46.315 -31.082  1.00 12.98          C
ATOM   6082  CE1 TYR D 147     17.164  46.571 -31.174  1.00 11.55          C
ATOM   6083  CE2 TYR D 147     19.186  46.386 -29.911  1.00 12.56          C
ATOM   6084  CZ  TYR D 147     17.806  46.507 -29.970  1.00 12.95          C
ATOM   6085  OH  TYR D 147     17.063  46.626 -28.817  1.00 16.48          O
ATOM   6086  N   PHE D 148     22.024  44.070 -32.985  1.00 12.63          N
ATOM   6087  CA  PHE D 148     22.773  43.361 -31.965  1.00 13.03          C
ATOM   6088  C   PHE D 148     24.116  44.056 -31.706  1.00 13.86          C
ATOM   6089  O   PHE D 148     24.704  44.645 -32.652  1.00 14.52          O
ATOM   6090  CB  PHE D 148     23.001  41.901 -32.392  1.00 14.43          C
ATOM   6091  CG  PHE D 148     23.664  41.095 -31.356  1.00 13.07          C
ATOM   6092  CD1 PHE D 148     22.884  40.462 -30.384  1.00 16.27          C
ATOM   6093  CD2 PHE D 148     25.048  40.992 -31.284  1.00 13.89          C
ATOM   6094  CE1 PHE D 148     23.454  39.714 -29.383  1.00 16.80          C
ATOM   6095  CE2 PHE D 148     25.633  40.229 -30.257  1.00 13.49          C
ATOM   6096  CZ  PHE D 148     24.829  39.607 -29.310  1.00 17.00          C
ATOM   6097  N   PRO D 149     24.597  44.080 -30.432  1.00 12.95          N
ATOM   6098  CA  PRO D 149     23.957  43.730 -29.194  1.00 13.70          C
```

```
ATOM   6099   C    PRO  D  149      23.092   44.865  -28.690   1.00  14.14        C
ATOM   6100   O    PRO  D  149      23.099   45.964  -29.263   1.00  16.27        O
ATOM   6101   CB   PRO  D  149      25.145   43.564  -28.250   1.00  14.47        C
ATOM   6102   CG   PRO  D  149      26.074   44.577  -28.664   1.00  11.41        C
ATOM   6103   CD   PRO  D  149      25.973   44.557  -30.177   1.00  13.55        C
ATOM   6104   N    GLU  D  150      22.410   44.629  -27.577   1.00  14.65        N
ATOM   6105   CA   GLU  D  150      21.903   45.732  -26.756   1.00  14.37        C
ATOM   6106   C    GLU  D  150      23.025   46.554  -26.157   1.00  14.63        C
ATOM   6107   O    GLU  D  150      24.153   46.126  -26.147   1.00  16.07        O
ATOM   6108   CB   GLU  D  150      21.047   45.137  -25.649   1.00  14.04        C
ATOM   6109   CG   GLU  D  150      19.690   44.789  -26.129   1.00  17.76        C
ATOM   6110   CD   GLU  D  150      18.732   45.829  -25.771   1.00  19.66        C
ATOM   6111   OE1  GLU  D  150      18.174   45.650  -24.664   1.00  22.34        O
ATOM   6112   OE2  GLU  D  150      18.595   46.828  -26.527   1.00  18.97        O
ATOM   6113   N    PRO  D  151      22.746   47.793  -25.721   1.00  17.02        N
ATOM   6114   CA   PRO  D  151      21.520   48.544  -25.795   1.00  18.41        C
ATOM   6115   C    PRO  D  151      21.360   49.442  -27.030   1.00  18.33        C
ATOM   6116   O    PRO  D  151      22.286   49.647  -27.824   1.00  16.15        O
ATOM   6117   CB   PRO  D  151      21.630   49.447  -24.569   1.00  18.61        C
ATOM   6118   CG   PRO  D  151      23.089   49.796  -24.549   1.00  18.41        C
ATOM   6119   CD   PRO  D  151      23.793   48.548  -24.999   1.00  17.19        C
ATOM   6120   N    VAL  D  152      20.150   49.932  -27.185   1.00  19.32        N
ATOM   6121   CA   VAL  D  152      19.876   51.099  -28.001   1.00  19.95        C
ATOM   6122   C    VAL  D  152      19.219   52.098  -27.064   1.00  21.59        C
ATOM   6123   O    VAL  D  152      18.649   51.711  -26.055   1.00  22.20        O
ATOM   6124   CB   VAL  D  152      18.955   50.801  -29.236   1.00  20.38        C
ATOM   6125   CG1  VAL  D  152      19.650   49.892  -30.217   1.00  19.21        C
ATOM   6126   CG2  VAL  D  152      17.640   50.222  -28.817   1.00  20.34        C
ATOM   6127   N    THR  D  153      19.306   53.377  -27.386   1.00  22.36        N
ATOM   6128   CA   THR  D  153      18.549   54.389  -26.653   1.00  22.97        C
ATOM   6129   C    THR  D  153      17.696   55.141  -27.668   1.00  22.87        C
ATOM   6130   O    THR  D  153      18.203   55.585  -28.698   1.00  22.77        O
ATOM   6131   CB   THR  D  153      19.441   55.352  -25.899   1.00  24.64        C
ATOM   6132   OG1  THR  D  153      20.209   56.114  -26.818   1.00  30.82        O
ATOM   6133   CG2  THR  D  153      20.390   54.626  -24.991   1.00  24.82        C
ATOM   6134   N    VAL  D  154      16.405   55.251  -27.365   1.00  20.91        N
ATOM   6135   CA   VAL  D  154      15.419   55.863  -28.232   1.00  21.75        C
ATOM   6136   C    VAL  D  154      14.928   57.124  -27.558   1.00  21.98        C
ATOM   6137   O    VAL  D  154      14.590   57.106  -26.360   1.00  20.83        O
ATOM   6138   CB   VAL  D  154      14.230   54.947  -28.465   1.00  22.04        C
ATOM   6139   CG1  VAL  D  154      13.159   55.647  -29.285   1.00  21.24        C
ATOM   6140   CG2  VAL  D  154      14.690   53.666  -29.160   1.00  22.97        C
ATOM   6141   N    THR  D  155      14.931   58.215  -28.312   1.00  22.81        N
ATOM   6142   CA   THR  D  155      14.356   59.465  -27.852   1.00  22.51        C
ATOM   6143   C    THR  D  155      13.391   59.978  -28.907   1.00  23.73        C
ATOM   6144   O    THR  D  155      13.341   59.477  -30.039   1.00  23.05        O
ATOM   6145   CB   THR  D  155      15.439   60.539  -27.559   1.00  22.88        C
ATOM   6146   OG1  THR  D  155      16.268   60.708  -28.692   1.00  23.25        O
ATOM   6147   CG2  THR  D  155      16.313   60.125  -26.421   1.00  23.06        C
ATOM   6148   N    TRP  D  156      12.584   60.955  -28.515   1.00  23.15        N
ATOM   6149   CA   TRP  D  156      11.664   61.564  -29.426   1.00  24.19        C
ATOM   6150   C    TRP  D  156      11.945   63.056  -29.485   1.00  25.21        C
ATOM   6151   O    TRP  D  156      12.088   63.711  -28.449   1.00  24.28        O
ATOM   6152   CB   TRP  D  156      10.241   61.291  -28.978   1.00  22.41        C
ATOM   6153   CG   TRP  D  156       9.819   59.872  -29.158   1.00  21.90        C
ATOM   6154   CD1  TRP  D  156       9.895   58.860  -28.253   1.00  21.24        C
ATOM   6155   CD2  TRP  D  156       9.249   59.325  -30.332   1.00  21.46        C
ATOM   6156   NE1  TRP  D  156       9.410   57.694  -28.807   1.00  21.99        N
ATOM   6157   CE2  TRP  D  156       8.981   57.966  -30.079   1.00  22.05        C
ATOM   6158   CE3  TRP  D  156       8.923   59.859  -31.584   1.00  21.75        C
ATOM   6159   CZ2  TRP  D  156       8.437   57.131  -31.042   1.00  22.18        C
ATOM   6160   CZ3  TRP  D  156       8.357   59.028  -32.532   1.00  21.38        C
ATOM   6161   CH2  TRP  D  156       8.121   57.686  -32.254   1.00  21.19        C
ATOM   6162   N    ASN  D  157      12.020   63.576  -30.709   1.00  27.28        N
ATOM   6163   CA   ASN  D  157      12.390   64.973  -30.966   1.00  27.78        C
```

602

```
ATOM   6164  C    ASN D 157    13.659  65.421 -30.234  1.00 28.99       C
ATOM   6165  O    ASN D 157    13.701  66.511 -29.653  1.00 27.79       O
ATOM   6166  CB   ASN D 157    11.216  65.916 -30.650  1.00 28.39       C
ATOM   6167  CG   ASN D 157    10.078  65.767 -31.624  1.00 29.86       C
ATOM   6168  OD1  ASN D 157    10.210  65.102 -32.664  1.00 29.94       O
ATOM   6169  ND2  ASN D 157     8.945  66.384 -31.302  1.00 29.78       N
ATOM   6170  N    SER D 158    14.686  64.564 -30.289  1.00 28.19       N
ATOM   6171  CA   SER D 158    15.985  64.824 -29.676  1.00 29.41       C
ATOM   6172  C    SER D 158    15.878  65.048 -28.165  1.00 30.49       C
ATOM   6173  O    SER D 158    16.684  65.785 -27.581  1.00 32.61       O
ATOM   6174  CB   SER D 158    16.683  66.003 -30.360  1.00 29.65       C
ATOM   6175  OG   SER D 158    16.759  65.801 -31.762  1.00 29.24       O
ATOM   6176  N    GLY D 159    14.885  64.405 -27.549  1.00 29.85       N
ATOM   6177  CA   GLY D 159    14.694  64.437 -26.107  1.00 30.30       C
ATOM   6178  C    GLY D 159    13.682  65.464 -25.610  1.00 30.24       C
ATOM   6179  O    GLY D 159    13.281  65.405 -24.458  1.00 29.42       O
ATOM   6180  N    SER D 160    13.273  66.402 -26.468  1.00 31.64       N
ATOM   6181  CA   SER D 160    12.348  67.461 -26.069  1.00 32.37       C
ATOM   6182  C    SER D 160    10.900  66.971 -25.963  1.00 33.62       C
ATOM   6183  O    SER D 160    10.038  67.688 -25.470  1.00 34.28       O
ATOM   6184  CB   SER D 160    12.439  68.639 -27.031  1.00 32.62       C
ATOM   6185  OG   SER D 160    11.994  68.272 -28.320  1.00 35.56       O
ATOM   6186  N    LEU D 161    10.638  65.756 -26.430  1.00 33.50       N
ATOM   6187  CA   LEU D 161     9.342  65.131 -26.288  1.00 33.08       C
ATOM   6188  C    LEU D 161     9.532  63.927 -25.364  1.00 33.59       C
ATOM   6189  O    LEU D 161    10.024  62.888 -25.782  1.00 33.54       O
ATOM   6190  CB   LEU D 161     8.821  64.701 -27.668  1.00 33.56       C
ATOM   6191  CG   LEU D 161     7.313  64.705 -27.939  1.00 34.27       C
ATOM   6192  CD1  LEU D 161     7.002  63.884 -29.215  1.00 33.74       C
ATOM   6193  CD2  LEU D 161     6.517  64.191 -26.770  1.00 35.27       C
ATOM   6194  N    SER D 162     9.164  64.088 -24.096  1.00 33.36       N
ATOM   6195  CA   SER D 162     9.365  63.050 -23.079  1.00 33.57       C
ATOM   6196  C    SER D 162     8.069  62.590 -22.398  1.00 33.17       C
ATOM   6197  O    SER D 162     7.953  61.416 -22.001  1.00 32.63       O
ATOM   6198  CB   SER D 162    10.362  63.545 -22.033  1.00 34.29       C
ATOM   6199  OG   SER D 162    10.103  64.890 -21.658  1.00 37.25       O
ATOM   6200  N    SER D 163     7.097  63.493 -22.263  1.00 33.08       N
ATOM   6201  CA   SER D 163     5.788  63.115 -21.724  1.00 32.66       C
ATOM   6202  C    SER D 163     5.089  62.182 -22.714  1.00 32.28       C
ATOM   6203  O    SER D 163     5.118  62.424 -23.926  1.00 33.54       O
ATOM   6204  CB   SER D 163     4.908  64.349 -21.464  1.00 33.83       C
ATOM   6205  OG   SER D 163     3.537  64.099 -21.786  1.00 35.14       O
ATOM   6206  N    GLY D 164     4.481  61.121 -22.193  1.00 29.94       N
ATOM   6207  CA   GLY D 164     3.628  60.247 -22.990  1.00 29.69       C
ATOM   6208  C    GLY D 164     4.376  59.225 -23.816  1.00 28.16       C
ATOM   6209  O    GLY D 164     3.853  58.724 -24.813  1.00 25.88       O
ATOM   6210  N    VAL D 165     5.598  58.914 -23.386  1.00 28.21       N
ATOM   6211  CA   VAL D 165     6.477  57.967 -24.103  1.00 27.23       C
ATOM   6212  C    VAL D 165     6.697  56.670 -23.313  1.00 26.50       C
ATOM   6213  O    VAL D 165     7.061  56.731 -22.148  1.00 27.65       O
ATOM   6214  CB   VAL D 165     7.848  58.580 -24.357  1.00 27.41       C
ATOM   6215  CG1  VAL D 165     8.804  57.538 -24.973  1.00 27.61       C
ATOM   6216  CG2  VAL D 165     7.756  59.824 -25.275  1.00 26.86       C
ATOM   6217  N    HIS D 166     6.479  55.528 -23.959  1.00 25.62       N
ATOM   6218  CA   HIS D 166     6.835  54.193 -23.442  1.00 25.78       C
ATOM   6219  C    HIS D 166     7.870  53.598 -24.375  1.00 25.42       C
ATOM   6220  O    HIS D 166     7.641  53.557 -25.584  1.00 24.40       O
ATOM   6221  CB   HIS D 166     5.682  53.186 -23.543  1.00 26.71       C
ATOM   6222  CG   HIS D 166     4.432  53.597 -22.862  1.00 29.07       C
ATOM   6223  ND1  HIS D 166     4.358  53.801 -21.505  1.00 32.07       N
ATOM   6224  CD2  HIS D 166     3.182  53.780 -23.345  1.00 31.13       C
ATOM   6225  CE1  HIS D 166     3.119  54.125 -21.180  1.00 33.53       C
ATOM   6226  NE2  HIS D 166     2.387  54.130 -22.283  1.00 33.77       N
ATOM   6227  N    THR D 167     8.971  53.078 -23.837  1.00 24.10       N
ATOM   6228  CA   THR D 167     9.864  52.289 -24.669  1.00 22.57       C
```

| ATOM | 6229 | C | THR | D | 167 | 9.894 | 50.896 | -24.112 | 1.00 | 21.76 | C |
| ATOM | 6230 | O | THR | D | 167 | 10.128 | 50.719 | -22.923 | 1.00 | 21.94 | O |
| ATOM | 6231 | CB | THR | D | 167 | 11.259 | 52.883 | -24.749 | 1.00 | 22.54 | C |
| ATOM | 6232 | OG1 | THR | D | 167 | 11.174 | 54.170 | -25.358 | 1.00 | 19.92 | O |
| ATOM | 6233 | CG2 | THR | D | 167 | 12.149 | 51.999 | -25.628 | 1.00 | 22.36 | C |
| ATOM | 6234 | N | PHE | D | 168 | 9.650 | 49.911 | -24.969 | 1.00 | 20.32 | N |
| ATOM | 6235 | CA | PHE | D | 168 | 9.461 | 48.544 | -24.508 | 1.00 | 20.14 | C |
| ATOM | 6236 | C | PHE | D | 168 | 10.803 | 47.836 | -24.482 | 1.00 | 19.42 | C |
| ATOM | 6237 | O | PHE | D | 168 | 11.645 | 48.077 | -25.334 | 1.00 | 20.96 | O |
| ATOM | 6238 | CB | PHE | D | 168 | 8.464 | 47.820 | -25.407 | 1.00 | 21.16 | C |
| ATOM | 6239 | CG | PHE | D | 168 | 7.113 | 48.482 | -25.455 | 1.00 | 21.88 | C |
| ATOM | 6240 | CD1 | PHE | D | 168 | 6.782 | 49.369 | -26.483 | 1.00 | 24.29 | C |
| ATOM | 6241 | CD2 | PHE | D | 168 | 6.180 | 48.259 | -24.446 | 1.00 | 23.80 | C · |
| ATOM | 6242 | CE1 | PHE | D | 168 | 5.551 | 50.018 | -26.518 | 1.00 | 21.47 | C |
| ATOM | 6243 | CE2 | PHE | D | 168 | 4.940 | 48.890 | -24.480 | 1.00 | 22.03 | C |
| ATOM | 6244 | CZ | PHE | D | 168 | 4.625 | 49.765 | -25.521 | 1.00 | 22.73 | C |
| ATOM | 6245 | N | PRO | D | 169 | 11.034 | 46.981 | -23.486 | 1.00 | 19.57 | N |
| ATOM | 6246 | CA | PRO | D | 169 | 12.270 | 46.194 | -23.490 | 1.00 | 19.26 | C |
| ATOM | 6247 | C | PRO | D | 169 | 12.465 | 45.393 | -24.777 | 1.00 | 18.96 | C |
| ATOM | 6248 | O | PRO | D | 169 | 11.504 | 44.905 | -25.351 | 1.00 | 19.80 | O |
| ATOM | 6249 | CB | PRO | D | 169 | 12.094 | 45.259 | -22.290 | 1.00 | 20.03 | C |
| ATOM | 6250 | CG | PRO | D | 169 | 11.186 | 46.009 | -21.372 | 1.00 | 19.16 | C |
| ATOM | 6251 | CD | PRO | D | 169 | 10.220 | 46.716 | -22.279 | 1.00 | 19.42 | C |
| ATOM | 6252 | N | ALA | D | 170 | 13.714 | 45.274 | -25.219 | 1.00 | 18.56 | N |
| ATOM | 6253 | CA | ALA | D | 170 | 14.051 | 44.492 | -26.407 | 1.00 | 19.22 | C |
| ATOM | 6254 | C | ALA | D | 170 | 13.829 | 43.023 | -26.148 | 1.00 | 19.48 | C |
| ATOM | 6255 | O | ALA | D | 170 | 13.974 | 42.598 | -25.014 | 1.00 | 21.53 | O |
| ATOM | 6256 | CB | ALA | D | 170 | 15.511 | 44.729 | -26.782 | 1.00 | 17.89 | C |
| ATOM | 6257 | N | VAL | D | 171 | 13.496 | 42.277 | -27.202 | 1.00 | 21.95 | N |
| ATOM | 6258 | CA | VAL | D | 171 | 13.482 | 40.804 | -27.192 | 1.00 | 22.68 | C |
| ATOM | 6259 | C | VAL | D | 171 | 14.428 | 40.277 | -28.259 | 1.00 | 23.41 | C |
| ATOM | 6260 | O | VAL | D | 171 | 14.493 | 40.803 | -29.359 | 1.00 | 21.41 | O |
| ATOM | 6261 | CB | VAL | D | 171 | 12.071 | 40.187 | -27.414 | 1.00 | 22.65 | C |
| ATOM | 6262 | CG1 | VAL | D | 171 | 11.360 | 40.811 | -28.613 | 1.00 | 24.19 | C |
| ATOM | 6263 | CG2 | VAL | D | 171 | 12.149 | 38.662 | -27.560 | 1.00 | 23.64 | C |
| ATOM | 6264 | N | LEU | D | 172 | 15.153 | 39.223 | -27.907 | 1.00 | 25.68 | N |
| ATOM | 6265 | CA | LEU | D | 172 | 16.068 | 38.566 | -28.806 | 1.00 | 27.31 | C |
| ATOM | 6266 | C | LEU | D | 172 | 15.321 | 37.485 | -29.524 | 1.00 | 29.07 | C |
| ATOM | 6267 | O | LEU | D | 172 | 14.706 | 36.642 | -28.884 | 1.00 | 29.08 | O |
| ATOM | 6268 | CB | LEU | D | 172 | 17.213 | 37.926 | -28.019 | 1.00 | 27.26 | C |
| ATOM | 6269 | CG | LEU | D | 172 | 18.433 | 37.468 | -28.817 | 1.00 | 27.78 | C |
| ATOM | 6270 | CD1 | LEU | D | 172 | 19.228 | 38.652 | -29.320 | 1.00 | 27.98 | C |
| ATOM | 6271 | CD2 | LEU | D | 172 | 19.321 | 36.624 | -27.953 | 1.00 | 28.21 | C |
| ATOM | 6272 | N | GLN | D | 173 | 15.374 | 37.501 | -30.845 | 1.00 | 32.08 | N |
| ATOM | 6273 | CA | GLN | D | 173 | 14.843 | 36.400 | -31.635 | 1.00 | 33.74 | C |
| ATOM | 6274 | C | GLN | D | 173 | 15.730 | 36.160 | -32.850 | 1.00 | 34.83 | C |
| ATOM | 6275 | O | GLN | D | 173 | 15.998 | 37.073 | -33.628 | 1.00 | 35.39 | O |
| ATOM | 6276 | CB | GLN | D | 173 | 13.414 | 36.676 | -32.073 | 1.00 | 34.73 | C |
| ATOM | 6277 | CG | GLN | D | 173 | 12.662 | 35.397 | -32.441 | 1.00 | 36.00 | C |
| ATOM | 6278 | CD | GLN | D | 173 | 11.703 | 35.574 | -33.607 | 1.00 | 37.98 | C |
| ATOM | 6279 | OE1 | GLN | D | 173 | 11.085 | 36.633 | -33.771 | 1.00 | 43.15 | O |
| ATOM | 6280 | NE2 | GLN | D | 173 | 11.556 | 34.522 | -34.418 | 1.00 | 41.28 | N |
| ATOM | 6281 | N | SER | D | 174 | 16.201 | 34.929 | -33.002 | 1.00 | 35.73 | N |
| ATOM | 6282 | CA | SER | D | 174 | 17.062 | 34.589 | -34.128 | 1.00 | 35.35 | C |
| ATOM | 6283 | C | SER | D | 174 | 18.330 | 35.466 | -34.158 | 1.00 | 34.53 | C |
| ATOM | 6284 | O | SER | D | 174 | 18.670 | 35.979 | -35.221 | 1.00 | 36.29 | O |
| ATOM | 6285 | CB | SER | D | 174 | 16.288 | 34.746 | -35.461 | 1.00 | 36.94 | C |
| ATOM | 6286 | OG | SER | D | 174 | 14.925 | 34.306 | -35.382 | 1.00 | 38.70 | O |
| ATOM | 6287 | N | ASP | D | 175 | 18.994 | 35.653 | -32.999 | 1.00 | 33.09 | N |
| ATOM | 6288 | CA | ASP | D | 175 | 20.302 | 36.362 | -32.883 | 1.00 | 30.70 | C |
| ATOM | 6289 | C | ASP | D | 175 | 20.222 | 37.904 | -32.976 | 1.00 | 28.05 | C |
| ATOM | 6290 | O | ASP | D | 175 | 21.250 | 38.566 | -32.973 | 1.00 | 27.68 | O |
| ATOM | 6291 | CB | ASP | D | 175 | 21.289 | 35.847 | -33.965 | 1.00 | 33.81 | C |
| ATOM | 6292 | CG | ASP | D | 175 | 22.720 | 35.538 | -33.420 | 1.00 | 36.79 | C |
| ATOM | 6293 | OD1 | ASP | D | 175 | 22.874 | 35.219 | -32.215 | 1.00 | 41.24 | O |

```
ATOM   6294  OD2 ASP D 175    23.703  35.579 -34.224  1.00 40.00          O
ATOM   6295  N   LEU D 176    19.007  38.455 -33.084  1.00 23.91          N
ATOM   6296  CA  LEU D 176    18.777  39.901 -33.327  1.00 21.58          C
ATOM   6297  C   LEU D 176    17.686  40.405 -32.394  1.00 19.59          C
ATOM   6298  O   LEU D 176    16.779  39.647 -32.027  1.00 18.82          O
ATOM   6299  CB  LEU D 176    18.374  40.114 -34.776  1.00 19.94          C
ATOM   6300  CG  LEU D 176    19.442  39.814 -35.834  1.00 16.78          C
ATOM   6301  CD1 LEU D 176    18.880  40.019 -37.238  1.00 18.45          C
ATOM   6302  CD2 LEU D 176    20.685  40.660 -35.632  1.00 16.71          C
ATOM   6303  N   TYR D 177    17.773  41.670 -31.985  1.00 16.18          N
ATOM   6304  CA  TYR D 177    16.802  42.260 -31.083  1.00 15.74          C
ATOM   6305  C   TYR D 177    15.796  43.080 -31.845  1.00 16.20          C
ATOM   6306  O   TYR D 177    16.085  43.666 -32.900  1.00 12.44          O
ATOM   6307  CB  TYR D 177    17.453  43.209 -30.079  1.00 16.38          C
ATOM   6308  CG  TYR D 177    18.242  42.503 -29.018  1.00 15.56          C
ATOM   6309  CD1 TYR D 177    17.629  42.007 -27.867  1.00 14.43          C
ATOM   6310  CD2 TYR D 177    19.601  42.328 -29.165  1.00 14.54          C
ATOM   6311  CE1 TYR D 177    18.344  41.374 -26.908  1.00 16.34          C
ATOM   6312  CE2 TYR D 177    20.328  41.724 -28.228  1.00 14.25          C
ATOM   6313  CZ  TYR D 177    19.707  41.218 -27.089  1.00 16.98          C
ATOM   6314  OH  TYR D 177    20.484  40.577 -26.148  1.00 17.59          O
ATOM   6315  N   THR D 178    14.629  43.147 -31.245  1.00 17.38          N
ATOM   6316  CA  THR D 178    13.558  44.021 -31.662  1.00 18.82          C
ATOM   6317  C   THR D 178    13.070  44.755 -30.443  1.00 18.70          C
ATOM   6318  O   THR D 178    12.946  44.164 -29.382  1.00 17.41          O
ATOM   6319  CB  THR D 178    12.405  43.194 -32.226  1.00 19.86          C
ATOM   6320  OG1 THR D 178    12.882  42.476 -33.365  1.00 20.54          O
ATOM   6321  CG2 THR D 178    11.270  44.088 -32.647  1.00 19.12          C
ATOM   6322  N   LEU D 179    12.834  46.054 -30.600  1.00 18.81          N
ATOM   6323  CA  LEU D 179    12.035  46.811 -29.666  1.00 19.89          C
ATOM   6324  C   LEU D 179    11.219  47.915 -30.347  1.00 19.07          C
ATOM   6325  O   LEU D 179    11.403  48.210 -31.525  1.00 15.56          O
ATOM   6326  CB  LEU D 179    12.874  47.401 -28.552  1.00 20.84          C
ATOM   6327  CG  LEU D 179    13.861  48.558 -28.576  1.00 20.95          C
ATOM   6328  CD1 LEU D 179    13.257  49.900 -28.977  1.00 19.99          C
ATOM   6329  CD2 LEU D 179    14.405  48.713 -27.208  1.00 21.64          C
ATOM   6330  N   SER D 180    10.329  48.513 -29.575  1.00 19.72          N
ATOM   6331  CA  SER D 180     9.532  49.620 -30.062  1.00 19.68          C
ATOM   6332  C   SER D 180     9.339  50.690 -28.989  1.00 18.55          C
ATOM   6333  O   SER D 180     9.513  50.469 -27.776  1.00 17.62          O
ATOM   6334  CB  SER D 180     8.182  49.109 -30.532  1.00 20.39          C
ATOM   6335  OG  SER D 180     7.601  48.283 -29.504  1.00 24.24          O
ATOM   6336  N   SER D 181     9.041  51.883 -29.465  1.00 19.21          N
ATOM   6337  CA  SER D 181     8.798  53.004 -28.611  1.00 18.37          C
ATOM   6338  C   SER D 181     7.455  53.602 -29.029  1.00 19.21          C
ATOM   6339  O   SER D 181     7.187  53.732 -30.208  1.00 17.46          O
ATOM   6340  CB  SER D 181     9.888  54.044 -28.761  1.00 19.95          C
ATOM   6341  OG  SER D 181     9.731  55.074 -27.806  1.00 19.13          O
ATOM   6342  N   SER D 182     6.652  53.964 -28.036  1.00 19.66          N
ATOM   6343  CA  SER D 182     5.366  54.592 -28.244  1.00 20.42          C
ATOM   6344  C   SER D 182     5.409  56.037 -27.755  1.00 20.55          C
ATOM   6345  O   SER D 182     5.965  56.325 -26.710  1.00 21.45          O
ATOM   6346  CB  SER D 182     4.312  53.817 -27.468  1.00 20.56          C
ATOM   6347  OG  SER D 182     3.112  54.552 -27.365  1.00 24.35          O
ATOM   6348  N   VAL D 183     4.799  56.934 -28.513  1.00 21.06          N
ATOM   6349  CA  VAL D 183     4.554  58.289 -28.024  1.00 22.02          C
ATOM   6350  C   VAL D 183     3.094  58.650 -28.347  1.00 21.53          C
ATOM   6351  O   VAL D 183     2.564  58.314 -29.415  1.00 20.67          O
ATOM   6352  CB  VAL D 183     5.579  59.286 -28.568  1.00 21.61          C
ATOM   6353  CG1 VAL D 183     5.548  59.346 -30.106  1.00 22.94          C
ATOM   6354  CG2 VAL D 183     5.402  60.696 -27.915  1.00 22.90          C
ATOM   6355  N   THR D 184     2.438  59.254 -27.364  1.00 20.99          N
ATOM   6356  CA  THR D 184     1.076  59.672 -27.494  1.00 22.93          C
ATOM   6357  C   THR D 184     1.115  61.199 -27.405  1.00 23.46          C
ATOM   6358  O   THR D 184     1.654  61.768 -26.433  1.00 25.48          O
```

```
ATOM   6359   CB    THR  D 184      0.226   59.013  -26.421   1.00  21.88           C
ATOM   6360   OG1   THR  D 184      0.353   57.582  -26.547   1.00  22.58           O
ATOM   6361   CG2   THR  D 184     -1.229   59.354  -26.566   1.00  20.92           C
ATOM   6362   N     VAL  D 185      0.617   61.849  -28.459   1.00  25.46           N
ATOM   6363   CA    VAL  D 185      0.582   63.318  -28.532   1.00  26.74           C
ATOM   6364   C     VAL  D 185     -0.846   63.820  -28.804   1.00  28.60           C
ATOM   6365   O     VAL  D 185     -1.696   63.044  -29.229   1.00  28.60           O
ATOM   6366   CB    VAL  D 185      1.551   63.865  -29.615   1.00  27.01           C
ATOM   6367   CG1   VAL  D 185      2.996   63.518  -29.243   1.00  27.65           C
ATOM   6368   CG2   VAL  D 185      1.203   63.374  -31.009   1.00  28.01           C
ATOM   6369   N     PRO  D 186     -1.113   65.120  -28.567   1.00  30.06           N
ATOM   6370   CA    PRO  D 186     -2.441   65.618  -28.938   1.00  31.18           C
ATOM   6371   C     PRO  D 186     -2.623   65.629  -30.454   1.00  32.56           C
ATOM   6372   O     PRO  D 186     -1.751   66.120  -31.165   1.00  33.78           O
ATOM   6373   CB    PRO  D 186     -2.470   67.038  -28.360   1.00  31.56           C
ATOM   6374   CG    PRO  D 186     -1.266   67.151  -27.457   1.00  31.02           C
ATOM   6375   CD    PRO  D 186     -0.275   66.167  -27.955   1.00  30.40           C
ATOM   6376   N     SER  D 187     -3.745   65.094  -30.935   1.00  33.58           N
ATOM   6377   CA    SER  D 187     -4.034   64.991  -32.377   1.00  34.91           C
ATOM   6378   C     SER  D 187     -3.835   66.289  -33.153   1.00  35.95           C
ATOM   6379   O     SER  D 187     -3.514   66.256  -34.351   1.00  36.34           O
ATOM   6380   CB    SER  D 187     -5.470   64.515  -32.607   1.00  35.70           C
ATOM   6381   OG    SER  D 187     -5.673   63.246  -32.005   1.00  39.83           O
ATOM   6382   N     SER  D 188     -4.029   67.414  -32.472   1.00  35.45           N
ATOM   6383   CA    SER  D 188     -3.881   68.724  -33.082   1.00  36.34           C
ATOM   6384   C     SER  D 188     -2.429   69.108  -33.349   1.00  36.96           C
ATOM   6385   O     SER  D 188     -2.185   69.990  -34.166   1.00  37.81           O
ATOM   6386   CB    SER  D 188     -4.559   69.797  -32.213   1.00  37.10           C
ATOM   6387   OG    SER  D 188     -3.842   70.051  -31.018   1.00  38.23           O
ATOM   6388   N     THR  D 189     -1.475   68.446  -32.677   1.00  36.26           N
ATOM   6389   CA    THR  D 189     -0.037   68.736  -32.849   1.00  35.54           C
ATOM   6390   C     THR  D 189      0.626   67.925  -33.968   1.00  34.25           C
ATOM   6391   O     THR  D 189      1.687   68.282  -34.438   1.00  33.10           O
ATOM   6392   CB    THR  D 189      0.762   68.478  -31.539   1.00  35.90           C
ATOM   6393   OG1   THR  D 189      0.707   67.087  -31.197   1.00  35.78           O
ATOM   6394   CG2   THR  D 189      0.196   69.299  -30.380   1.00  36.59           C
ATOM   6395   N     TRP  D 190      0.008   66.815  -34.361   1.00  33.28           N
ATOM   6396   CA    TRP  D 190      0.529   65.970  -35.420   1.00  33.69           C
ATOM   6397   C     TRP  D 190     -0.506   65.795  -36.518   1.00  34.30           C
ATOM   6398   O     TRP  D 190     -1.669   65.588  -36.223   1.00  35.30           O
ATOM   6399   CB    TRP  D 190      0.918   64.588  -34.853   1.00  31.77           C
ATOM   6400   CG    TRP  D 190      1.600   63.737  -35.869   1.00  30.47           C
ATOM   6401   CD1   TRP  D 190      2.899   63.829  -36.279   1.00  29.51           C
ATOM   6402   CD2   TRP  D 190      1.009   62.697  -36.629   1.00  29.11           C
ATOM   6403   NE1   TRP  D 190      3.154   62.892  -37.243   1.00  28.12           N
ATOM   6404   CE2   TRP  D 190      2.006   62.186  -37.483   1.00  30.20           C
ATOM   6405   CE3   TRP  D 190     -0.269   62.144  -36.677   1.00  30.14           C
ATOM   6406   CZ2   TRP  D 190      1.763   61.126  -38.366   1.00  30.24           C
ATOM   6407   CZ3   TRP  D 190     -0.507   61.084  -37.541   1.00  29.64           C
ATOM   6408   CH2   TRP  D 190      0.504   60.590  -38.376   1.00  30.29           C
ATOM   6409   N     PRO  D 191     -0.088   65.852  -37.793   1.00  36.16           N
ATOM   6410   CA    PRO  D 191      1.255   66.047  -38.353   1.00  37.28           C
ATOM   6411   C     PRO  D 191      1.684   67.502  -38.533   1.00  37.83           C
ATOM   6412   O     PRO  D 191      2.649   67.762  -39.249   1.00  38.54           O
ATOM   6413   CB    PRO  D 191      1.146   65.366  -39.718   1.00  37.60           C
ATOM   6414   CG    PRO  D 191     -0.276   65.599  -40.122   1.00  37.27           C
ATOM   6415   CD    PRO  D 191     -1.090   65.673  -38.861   1.00  36.97           C
ATOM   6416   N     SER  D 192      0.986   68.441  -37.894   1.00  38.58           N
ATOM   6417   CA    SER  D 192      1.300   69.864  -38.046   1.00  38.00           C
ATOM   6418   C     SER  D 192      2.675   70.192  -37.444   1.00  38.24           C
ATOM   6419   O     SER  D 192      3.428   70.972  -38.014   1.00  38.96           O
ATOM   6420   CB    SER  D 192      0.199   70.723  -37.415   1.00  38.40           C
ATOM   6421   OG    SER  D 192     -0.206   70.172  -36.175   1.00  38.55           O
ATOM   6422   N     GLU  D 193      2.994   69.600  -36.295   1.00  37.68           N
ATOM   6423   CA    GLU  D 193      4.355   69.630  -35.767   1.00  37.10           C
```

```
ATOM   6424  C    GLU D 193       5.006  68.263 -36.000  1.00 35.88          C
ATOM   6425  O    GLU D 193       4.327  67.225 -35.926  1.00 36.09          O
ATOM   6426  CB   GLU D 193       4.380  69.951 -34.270  1.00 38.58          C
ATOM   6427  CG   GLU D 193       4.123  71.399 -33.912  1.00 41.38          C
ATOM   6428  CD   GLU D 193       2.742  71.619 -33.341  1.00 43.88          C
ATOM   6429  OE1  GLU D 193       2.648  72.029 -32.155  1.00 45.40          O
ATOM   6430  OE2  GLU D 193       1.751  71.364 -34.075  1.00 48.40          O
ATOM   6431  N    THR D 194       6.311  68.268 -36.269  1.00 33.95          N
ATOM   6432  CA   THR D 194       7.010  67.039 -36.607  1.00 32.51          C
ATOM   6433  C    THR D 194       7.219  66.228 -35.338  1.00 30.45          C
ATOM   6434  O    THR D 194       7.348  66.778 -34.228  1.00 29.27          O
ATOM   6435  CB   THR D 194       8.362  67.275 -37.324  1.00 32.73          C
ATOM   6436  OG1  THR D 194       9.208  68.073 -36.506  1.00 33.70          O
ATOM   6437  CG2  THR D 194       8.169  67.981 -38.663  1.00 34.33          C
ATOM   6438  N    VAL D 195       7.206  64.915 -35.523  1.00 28.12          N
ATOM   6439  CA   VAL D 195       7.521  63.961 -34.493  1.00 26.87          C
ATOM   6440  C    VAL D 195       8.533  63.002 -35.115  1.00 25.06          C
ATOM   6441  O    VAL D 195       8.218  62.324 -36.087  1.00 23.92          O
ATOM   6442  CB   VAL D 195       6.266  63.215 -34.047  1.00 26.47          C
ATOM   6443  CG1  VAL D 195       6.609  61.928 -33.265  1.00 26.87          C
ATOM   6444  CG2  VAL D 195       5.389  64.132 -33.227  1.00 25.91          C
ATOM   6445  N    THR D 196       9.731  62.972 -34.545  1.00 24.03          N
ATOM   6446  CA   THR D 196      10.840  62.149 -35.041  1.00 23.89          C
ATOM   6447  C    THR D 196      11.405  61.314 -33.901  1.00 23.13          C
ATOM   6448  O    THR D 196      11.666  61.847 -32.826  1.00 24.42          O
ATOM   6449  CB   THR D 196      11.938  63.073 -35.598  1.00 23.59          C
ATOM   6450  OG1  THR D 196      11.385  63.830 -36.683  1.00 23.27          O
ATOM   6451  CG2  THR D 196      13.187  62.307 -36.067  1.00 23.95          C
ATOM   6452  N    CYS D 197      11.603  60.012 -34.133  1.00 21.88          N
ATOM   6453  CA   CYS D 197      12.294  59.182 -33.165  1.00 21.36          C
ATOM   6454  C    CYS D 197      13.765  59.072 -33.523  1.00 19.24          C
ATOM   6455  O    CYS D 197      14.128  59.004 -34.689  1.00 18.63          O
ATOM   6456  CB   CYS D 197      11.625  57.832 -33.032  1.00 22.74          C
ATOM   6457  SG   CYS D 197      12.084  56.700 -34.285  1.00 27.70          S
ATOM   6458  N    ASN D 198      14.616  59.147 -32.500  1.00 19.90          N
ATOM   6459  CA   ASN D 198      16.065  59.145 -32.674  1.00 18.31          C
ATOM   6460  C    ASN D 198      16.563  57.883 -32.002  1.00 16.57          C
ATOM   6461  O    ASN D 198      16.193  57.634 -30.881  1.00 16.75          O
ATOM   6462  CB   ASN D 198      16.682  60.332 -31.969  1.00 19.83          C
ATOM   6463  CG   ASN D 198      15.848  61.572 -32.093  1.00 19.74          C
ATOM   6464  OD1  ASN D 198      15.249  62.009 -31.125  1.00 22.81          O
ATOM   6465  ND2  ASN D 198      15.783  62.131 -33.288  1.00 21.79          N
ATOM   6466  N    VAL D 199      17.356  57.104 -32.713  1.00 15.62          N
ATOM   6467  CA   VAL D 199      17.811  55.789 -32.237  1.00 15.38          C
ATOM   6468  C    VAL D 199      19.348  55.682 -32.300  1.00 15.28          C
ATOM   6469  O    VAL D 199      19.950  55.706 -33.374  1.00 16.74          O
ATOM   6470  CB   VAL D 199      17.171  54.682 -33.055  1.00 15.25          C
ATOM   6471  CG1  VAL D 199      17.612  53.337 -32.519  1.00 15.38          C
ATOM   6472  CG2  VAL D 199      15.664  54.822 -33.028  1.00 15.12          C
ATOM   6473  N    ALA D 200      19.966  55.595 -31.131  1.00 15.79          N
ATOM   6474  CA   ALA D 200      21.397  55.494 -31.008  1.00 14.40          C
ATOM   6475  C    ALA D 200      21.742  54.052 -30.617  1.00 15.18          C
ATOM   6476  O    ALA D 200      21.077  53.479 -29.795  1.00 16.01          O
ATOM   6477  CB   ALA D 200      21.881  56.439 -29.964  1.00 16.65          C
ATOM   6478  N    HIS D 201      22.768  53.503 -31.239  1.00 14.99          N
ATOM   6479  CA   HIS D 201      23.225  52.132 -30.973  1.00 14.39          C
ATOM   6480  C    HIS D 201      24.716  52.278 -30.689  1.00 13.59          C
ATOM   6481  O    HIS D 201      25.515  52.364 -31.611  1.00 14.49          O
ATOM   6482  CB   HIS D 201      22.968  51.273 -32.204  1.00 13.85          C
ATOM   6483  CG   HIS D 201      23.248  49.808 -32.004  1.00 14.21          C
ATOM   6484  ND1  HIS D 201      23.925  49.054 -32.934  1.00 12.62          N
ATOM   6485  CD2  HIS D 201      22.928  48.972 -31.000  1.00 15.46          C
ATOM   6486  CE1  HIS D 201      24.007  47.801 -32.504  1.00 14.07          C
ATOM   6487  NE2  HIS D 201      23.397  47.724 -31.337  1.00 13.66          N
ATOM   6488  N    PRO D 202      25.094  52.338 -29.424  1.00 15.63          N
```

```
ATOM   6489  CA   PRO D 202    26.502  52.500 -29.058  1.00 15.20          C
ATOM   6490  C    PRO D 202    27.462  51.508 -29.720  1.00 14.42          C
ATOM   6491  O    PRO D 202    28.519  51.893 -30.204  1.00 13.65          O
ATOM   6492  CB   PRO D 202    26.500  52.314 -27.539  1.00 16.89          C
ATOM   6493  CG   PRO D 202    25.142  52.245 -27.116  1.00 17.47          C
ATOM   6494  CD   PRO D 202    24.209  52.295 -28.245  1.00 17.59          C
ATOM   6495  N    ALA D 203    27.101  50.228 -29.786  1.00 14.70          N
ATOM   6496  CA   ALA D 203    27.993  49.267 -30.396  1.00 13.49          C
ATOM   6497  C    ALA D 203    28.380  49.491 -31.859  1.00 13.78          C
ATOM   6498  O    ALA D 203    29.476  49.051 -32.246  1.00 13.36          O
ATOM   6499  CB   ALA D 203    27.445  47.859 -30.217  1.00 12.05          C
ATOM   6500  N    SER D 204    27.528  50.185 -32.645  1.00 12.30          N
ATOM   6501  CA   SER D 204    27.797  50.553 -34.040  1.00 12.82          C
ATOM   6502  C    SER D 204    28.086  52.053 -34.195  1.00 12.41          C
ATOM   6503  O    SER D 204    28.290  52.529 -35.300  1.00 12.45          O
ATOM   6504  CB   SER D 204    26.616  50.165 -34.955  1.00 13.91          C
ATOM   6505  OG   SER D 204    25.397  50.715 -34.515  1.00 14.12          O
ATOM   6506  N    SER D 205    28.088  52.761 -33.090  1.00 12.94          N
ATOM   6507  CA   SER D 205    28.362  54.195 -33.096  1.00 15.50          C
ATOM   6508  C    SER D 205    27.457  54.845 -34.127  1.00 16.07          C
ATOM   6509  O    SER D 205    27.893  55.715 -34.875  1.00 17.55          O
ATOM   6510  CB   SER D 205    29.834  54.464 -33.426  1.00 16.27          C
ATOM   6511  OG   SER D 205    30.687  53.698 -32.574  1.00 17.19          O
ATOM   6512  N    THR D 206    26.198  54.407 -34.181  1.00 17.21          N
ATOM   6513  CA   THR D 206    25.209  54.968 -35.110  1.00 17.66          C
ATOM   6514  C    THR D 206    24.137  55.773 -34.362  1.00 19.78          C
ATOM   6515  O    THR D 206    23.841  55.522 -33.191  1.00 19.01          O
ATOM   6516  CB   THR D 206    24.534  53.870 -35.971  1.00 18.86          C
ATOM   6517  OG1  THR D 206    23.865  52.912 -35.141  1.00 18.83          O
ATOM   6518  CG2  THR D 206    25.559  53.134 -36.828  1.00 17.00          C
ATOM   6519  N    LYS D 207    23.566  56.752 -35.055  1.00 20.99          N
ATOM   6520  CA   LYS D 207    22.421  57.490 -34.557  1.00 22.58          C
ATOM   6521  C    LYS D 207    21.518  57.775 -35.772  1.00 22.32          C
ATOM   6522  O    LYS D 207    21.952  58.353 -36.769  1.00 22.65          O
ATOM   6523  CB   LYS D 207    22.862  58.752 -33.770  1.00 25.73          C
ATOM   6524  CG   LYS D 207    22.949  60.092 -34.568  1.00 29.50          C
ATOM   6525  CD   LYS D 207    24.131  60.135 -35.561  1.00 32.10          C
ATOM   6526  CE   LYS D 207    23.924  61.150 -36.710  1.00 31.39          C
ATOM   6527  NZ   LYS D 207    24.682  60.757 -37.958  1.00 32.75          N
ATOM   6528  N    VAL D 208    20.300  57.253 -35.734  1.00 21.52          N
ATOM   6529  CA   VAL D 208    19.404  57.291 -36.874  1.00 21.94          C
ATOM   6530  C    VAL D 208    18.113  57.999 -36.455  1.00 21.71          C
ATOM   6531  O    VAL D 208    17.619  57.801 -35.342  1.00 19.12          O
ATOM   6532  CB   VAL D 208    19.151  55.833 -37.401  1.00 22.82          C
ATOM   6533  CG1  VAL D 208    17.880  55.736 -38.195  1.00 25.58          C
ATOM   6534  CG2  VAL D 208    20.328  55.393 -38.233  1.00 23.08          C
ATOM   6535  N    ASP D 209    17.615  58.862 -37.331  1.00 20.56          N
ATOM   6536  CA   ASP D 209    16.361  59.562 -37.113  1.00 21.52          C
ATOM   6537  C    ASP D 209    15.311  59.020 -38.058  1.00 21.58          C
ATOM   6538  O    ASP D 209    15.602  58.752 -39.225  1.00 23.09          O
ATOM   6539  CB   ASP D 209    16.538  61.058 -37.419  1.00 23.40          C
ATOM   6540  CG   ASP D 209    17.420  61.786 -36.410  1.00 25.77          C
ATOM   6541  OD1  ASP D 209    17.508  61.382 -35.228  1.00 26.75          O
ATOM   6542  OD2  ASP D 209    18.019  62.816 -36.810  1.00 29.19          O
ATOM   6543  N    LYS D 210    14.078  58.892 -37.586  1.00 20.45          N
ATOM   6544  CA   LYS D 210    12.952  58.554 -38.436  1.00 20.32          C
ATOM   6545  C    LYS D 210    11.773  59.479 -38.104  1.00 20.53          C
ATOM   6546  O    LYS D 210    11.224  59.475 -37.000  1.00 18.45          O
ATOM   6547  CB   LYS D 210    12.568  57.059 -38.307  1.00 20.47          C
ATOM   6548  CG   LYS D 210    11.362  56.577 -39.157  1.00 20.28          C
ATOM   6549  CD   LYS D 210    11.528  56.848 -40.649  1.00 20.25          C
ATOM   6550  CE   LYS D 210    12.529  55.913 -41.276  1.00 18.59          C
ATOM   6551  NZ   LYS D 210    12.742  56.221 -42.726  1.00 18.48          N
ATOM   6552  N    LYS D 211    11.414  60.297 -39.079  1.00 22.37          N
ATOM   6553  CA   LYS D 211    10.252  61.145 -38.959  1.00 22.43          C
```

| ATOM | 6554 | C | LYS | D | 211 | 9.000 | 60.320 | -39.213 | 1.00 | 22.34 | C |
|------|------|------|-----|---|-----|-------|--------|---------|------|-------|---|
| ATOM | 6555 | O | LYS | D | 211 | 8.929 | 59.578 | -40.168 | 1.00 | 21.23 | O |
| ATOM | 6556 | CB | LYS | D | 211 | 10.356 | 62.288 | -39.955 | 1.00 | 24.02 | C |
| ATOM | 6557 | CG | LYS | D | 211 | 9.323 | 63.385 | -39.726 | 1.00 | 24.71 | C |
| ATOM | 6558 | CD | LYS | D | 211 | 9.579 | 64.549 | -40.642 | 1.00 | 25.38 | C |
| ATOM | 6559 | CE | LYS | D | 211 | 8.318 | 65.304 | -40.940 | 1.00 | 27.31 | C |
| ATOM | 6560 | NZ | LYS | D | 211 | 8.616 | 66.640 | -41.575 | 1.00 | 28.37 | N |
| ATOM | 6561 | N | ILE | D | 212 | 8.013 | 60.450 | -38.342 | 1.00 | 22.27 | N |
| ATOM | 6562 | CA | ILE | D | 212 | 6.739 | 59.777 | -38.524 | 1.00 | 23.04 | C |
| ATOM | 6563 | C | ILE | D | 212 | 5.858 | 60.694 | -39.336 | 1.00 | 24.70 | C |
| ATOM | 6564 | O | ILE | D | 212 | 5.534 | 61.786 | -38.887 | 1.00 | 24.40 | O |
| ATOM | 6565 | CB | ILE | D | 212 | 6.036 | 59.476 | -37.187 | 1.00 | 21.69 | C |
| ATOM | 6566 | CG1 | ILE | D | 212 | 7.043 | 58.959 | -36.147 | 1.00 | 22.88 | C |
| ATOM | 6567 | CG2 | ILE | D | 212 | 4.871 | 58.493 | -37.420 | 1.00 | 21.02 | C |
| ATOM | 6568 | CD1 | ILE | D | 212 | 7.928 | 57.817 | -36.629 | 1.00 | 22.41 | C |
| ATOM | 6569 | N | VAL | D | 213 | 5.493 | 60.253 | -40.530 | 1.00 | 25.22 | N |
| ATOM | 6570 | CA | VAL | D | 213 | 4.656 | 61.041 | -41.424 | 1.00 | 26.72 | C |
| ATOM | 6571 | C | VAL | D | 213 | 3.361 | 60.286 | -41.685 | 1.00 | 27.52 | C |
| ATOM | 6572 | O | VAL | D | 213 | 3.366 | 59.052 | -41.729 | 1.00 | 25.99 | O |
| ATOM | 6573 | CB | VAL | D | 213 | 5.383 | 61.385 | -42.746 | 1.00 | 27.22 | C |
| ATOM | 6574 | CG1 | VAL | D | 213 | 6.764 | 61.983 | -42.466 | 1.00 | 28.75 | C |
| ATOM | 6575 | CG2 | VAL | D | 213 | 5.542 | 60.194 | -43.608 | 1.00 | 27.05 | C |
| ATOM | 6576 | N | PRO | D | 214 | 2.237 | 61.016 | -41.857 | 1.00 | 28.15 | N |
| ATOM | 6577 | CA | PRO | D | 214 | 0.974 | 60.322 | -42.111 | 1.00 | 28.54 | C |
| ATOM | 6578 | C | PRO | D | 214 | 0.965 | 59.463 | -43.382 | 1.00 | 28.53 | C |
| ATOM | 6579 | O | PRO | D | 214 | 1.662 | 59.751 | -44.362 | 1.00 | 27.98 | O |
| ATOM | 6580 | CB | PRO | D | 214 | -0.049 | 61.451 | -42.255 | 1.00 | 29.43 | C |
| ATOM | 6581 | CG | PRO | D | 214 | 0.605 | 62.672 | -41.685 | 1.00 | 28.55 | C |
| ATOM | 6582 | CD | PRO | D | 214 | 2.078 | 62.483 | -41.841 | 1.00 | 28.65 | C |
| ATOM | 6583 | N | ARG | D | 215 | 0.159 | 58.421 | -43.356 | 1.00 | 29.13 | N |
| ATOM | 6584 | CA | ARG | D | 215 | 0.133 | 57.477 | -44.453 | 1.00 | 30.35 | C |
| ATOM | 6585 | C | ARG | D | 215 | -0.829 | 57.934 | -45.547 | 1.00 | 30.73 | C |
| ATOM | 6586 | O | ARG | D | 215 | -1.711 | 58.746 | -45.283 | 1.00 | 33.30 | O |
| ATOM | 6587 | CB | ARG | D | 215 | -0.235 | 56.084 | -43.938 | 1.00 | 30.49 | C |
| ATOM | 6588 | CG | ARG | D | 215 | 0.820 | 55.487 | -43.007 | 1.00 | 31.19 | C |
| ATOM | 6589 | CD | ARG | D | 215 | 0.325 | 54.247 | -42.273 | 1.00 | 31.15 | C |
| ATOM | 6590 | NE | ARG | D | 215 | -0.132 | 53.219 | -43.202 | 1.00 | 32.04 | N |
| ATOM | 6591 | CZ | ARG | D | 215 | 0.605 | 52.203 | -43.671 | 1.00 | 32.83 | C |
| ATOM | 6592 | NH1 | ARG | D | 215 | 1.870 | 52.027 | -43.290 | 1.00 | 31.19 | N |
| ATOM | 6593 | NH2 | ARG | D | 215 | 0.054 | 51.338 | -44.535 | 1.00 | 31.50 | N |
| TER | 6594 | | ARG | D | 215 | | | | | | |
| ATOM | 6595 | N | ASP | E | 1 | 2.876 | 34.887 | 26.032 | 1.00 | 16.14 | N |
| ATOM | 6596 | CA | ASP | E | 1 | 4.177 | 35.047 | 26.723 | 1.00 | 16.19 | C |
| ATOM | 6597 | C | ASP | E | 1 | 5.232 | 35.404 | 25.715 | 1.00 | 15.95 | C |
| ATOM | 6598 | O | ASP | E | 1 | 5.248 | 34.879 | 24.611 | 1.00 | 17.62 | O |
| ATOM | 6599 | CB | ASP | E | 1 | 4.648 | 33.740 | 27.443 | 1.00 | 17.79 | C |
| ATOM | 6600 | CG | ASP | E | 1 | 3.733 | 33.308 | 28.556 | 1.00 | 19.38 | C |
| ATOM | 6601 | OD1 | ASP | E | 1 | 2.772 | 34.047 | 28.827 | 1.00 | 22.36 | O |
| ATOM | 6602 | OD2 | ASP | E | 1 | 3.959 | 32.213 | 29.171 | 1.00 | 19.16 | O |
| ATOM | 6603 | N | ILE | E | 2 | 6.142 | 36.275 | 26.123 | 1.00 | 14.13 | N |
| ATOM | 6604 | CA | ILE | E | 2 | 7.350 | 36.577 | 25.388 | 1.00 | 13.61 | C |
| ATOM | 6605 | C | ILE | E | 2 | 8.461 | 36.582 | 26.445 | 1.00 | 13.56 | C |
| ATOM | 6606 | O | ILE | E | 2 | 8.277 | 37.138 | 27.550 | 1.00 | 15.77 | O |
| ATOM | 6607 | CB | ILE | E | 2 | 7.239 | 37.975 | 24.751 | 1.00 | 13.05 | C |
| ATOM | 6608 | CG1 | ILE | E | 2 | 6.167 | 37.941 | 23.642 | 1.00 | 14.44 | C |
| ATOM | 6609 | CG2 | ILE | E | 2 | 8.583 | 38.451 | 24.226 | 1.00 | 12.77 | C |
| ATOM | 6610 | CD1 | ILE | E | 2 | 5.967 | 39.256 | 22.932 | 1.00 | 15.63 | C |
| ATOM | 6611 | N | VAL | E | 3 | 9.586 | 35.975 | 26.097 | 1.00 | 11.70 | N |
| ATOM | 6612 | CA | VAL | E | 3 | 10.778 | 36.008 | 26.873 | 1.00 | 13.11 | C |
| ATOM | 6613 | C | VAL | E | 3 | 11.755 | 36.958 | 26.239 | 1.00 | 11.01 | C |
| ATOM | 6614 | O | VAL | E | 3 | 12.072 | 36.873 | 25.035 | 1.00 | 10.90 | O |
| ATOM | 6615 | CB | VAL | E | 3 | 11.452 | 34.618 | 26.952 | 1.00 | 13.82 | C |
| ATOM | 6616 | CG1 | VAL | E | 3 | 12.839 | 34.747 | 27.573 | 1.00 | 12.81 | C |
| ATOM | 6617 | CG2 | VAL | E | 3 | 10.580 | 33.640 | 27.717 | 1.00 | 14.97 | C |
| ATOM | 6618 | N | MET | E | 4 | 12.240 | 37.864 | 27.077 | 1.00 | 13.04 | N |

```
ATOM   6619  CA   MET E   4     13.243  38.885  26.754  1.00 13.15           C
ATOM   6620  C    MET E   4     14.551  38.495  27.382  1.00 13.96           C
ATOM   6621  O    MET E   4     14.629  38.322  28.601  1.00 13.94           O
ATOM   6622  CB   MET E   4     12.814  40.207  27.384  1.00 14.02           C
ATOM   6623  CG   MET E   4     11.448  40.670  26.871  1.00 12.81           C
ATOM   6624  SD   MET E   4     11.358  40.954  25.112  1.00 13.35           S
ATOM   6625  CE   MET E   4     12.330  42.449  24.924  1.00 12.22           C
ATOM   6626  N    THR E   5     15.576  38.291  26.562  1.00 14.02           N
ATOM   6627  CA   THR E   5     16.849  37.724  27.041  1.00 15.11           C
ATOM   6628  C    THR E   5     18.023  38.700  26.976  1.00 15.43           C
ATOM   6629  O    THR E   5     18.417  39.176  25.897  1.00 15.46           O
ATOM   6630  CB   THR E   5     17.233  36.454  26.237  1.00 14.95           C
ATOM   6631  OG1  THR E   5     16.155  35.535  26.306  1.00 16.99           O
ATOM   6632  CG2  THR E   5     18.485  35.789  26.812  1.00 15.88           C
ATOM   6633  N    GLN E   6     18.609  38.973  28.135  1.00 15.24           N
ATOM   6634  CA   GLN E   6     19.843  39.699  28.159  1.00 16.09           C
ATOM   6635  C    GLN E   6     20.819  39.122  29.182  1.00 17.18           C
ATOM   6636  O    GLN E   6     20.432  38.432  30.136  1.00 15.43           O
ATOM   6637  CB   GLN E   6     19.562  41.179  28.413  1.00 16.44           C
ATOM   6638  CG   GLN E   6     18.653  41.443  29.557  1.00 16.70           C
ATOM   6639  CD   GLN E   6     18.301  42.905  29.709  1.00 16.93           C
ATOM   6640  OE1  GLN E   6     17.270  43.209  30.269  1.00 15.64           O
ATOM   6641  NE2  GLN E   6     19.154  43.793  29.245  1.00 20.17           N
ATOM   6642  N    ALA E   7     22.088  39.412  28.974  1.00 17.19           N
ATOM   6643  CA   ALA E   7     23.098  38.928  29.888  1.00 20.27           C
ATOM   6644  C    ALA E   7     23.081  39.764  31.162  1.00 21.84           C
ATOM   6645  O    ALA E   7     22.804  40.973  31.130  1.00 22.07           O
ATOM   6646  CB   ALA E   7     24.464  38.964  29.230  1.00 20.84           C
ATOM   6647  N    ALA E   8     23.392  39.127  32.286  1.00 23.65           N
ATOM   6648  CA   ALA E   8     23.358  39.801  33.587  1.00 24.52           C
ATOM   6649  C    ALA E   8     24.439  40.884  33.693  1.00 25.33           C
ATOM   6650  O    ALA E   8     24.270  41.907  34.383  1.00 23.31           O
ATOM   6651  CB   ALA E   8     23.494  38.770  34.703  1.00 25.44           C
ATOM   6652  N    PHE E   9     25.537  40.661  32.989  1.00 27.52           N
ATOM   6653  CA   PHE E   9     26.693  41.540  33.059  1.00 30.66           C
ATOM   6654  C    PHE E   9     27.249  41.779  31.688  1.00 32.33           C
ATOM   6655  O    PHE E   9     27.136  40.936  30.808  1.00 32.31           O
ATOM   6656  CB   PHE E   9     27.791  40.902  33.910  1.00 30.76           C
ATOM   6657  CG   PHE E   9     27.305  40.384  35.216  1.00 30.00           C
ATOM   6658  CD1  PHE E   9     27.262  39.021  35.466  1.00 31.74           C
ATOM   6659  CD2  PHE E   9     26.856  41.256  36.192  1.00 32.36           C
ATOM   6660  CE1  PHE E   9     26.781  38.543  36.661  1.00 31.16           C
ATOM   6661  CE2  PHE E   9     26.378  40.784  37.403  1.00 31.19           C
ATOM   6662  CZ   PHE E   9     26.349  39.419  37.638  1.00 31.33           C
ATOM   6663  N    SER E  10     27.870  42.935  31.527  1.00 35.25           N
ATOM   6664  CA   SER E  10     28.665  43.245  30.349  1.00 36.02           C
ATOM   6665  C    SER E  10     30.113  43.397  30.803  1.00 37.77           C
ATOM   6666  O    SER E  10     30.398  43.423  32.012  1.00 38.11           O
ATOM   6667  CB   SER E  10     28.159  44.539  29.705  1.00 36.22           C
ATOM   6668  OG   SER E  10     28.065  45.602  30.647  1.00 35.95           O
ATOM   6669  N    ASN E  11     31.040  43.477  29.850  1.00 39.09           N
ATOM   6670  CA   ASN E  11     32.446  43.667  30.216  1.00 39.09           C
ATOM   6671  C    ASN E  11     32.662  45.110  30.652  1.00 38.55           C
ATOM   6672  O    ASN E  11     32.028  46.012  30.109  1.00 39.01           O
ATOM   6673  CB   ASN E  11     33.385  43.276  29.069  1.00 41.03           C
ATOM   6674  CG   ASN E  11     33.644  41.772  29.018  1.00 43.63           C
ATOM   6675  OD1  ASN E  11     34.161  41.179  29.981  1.00 45.93           O
ATOM   6676  ND2  ASN E  11     33.281  41.147  27.900  1.00 44.62           N
ATOM   6677  N    PRO E  12     33.528  45.330  31.653  1.00 37.87           N
ATOM   6678  CA   PRO E  12     33.697  46.673  32.194  1.00 37.30           C
ATOM   6679  C    PRO E  12     34.142  47.663  31.146  1.00 35.97           C
ATOM   6680  O    PRO E  12     34.833  47.295  30.190  1.00 36.09           O
ATOM   6681  CB   PRO E  12     34.782  46.496  33.258  1.00 37.49           C
ATOM   6682  CG   PRO E  12     34.739  45.071  33.616  1.00 37.82           C
ATOM   6683  CD   PRO E  12     34.387  44.358  32.349  1.00 37.69           C
```

| ATOM | 6684 | N | VAL | E | 13 | 33.746 | 48.916 | 31.323 | 1.00 | 34.06 | N |
|---|---|---|---|---|---|---|---|---|---|---|---|
| ATOM | 6685 | CA | VAL | E | 13 | 33.975 | 49.912 | 30.314 | 1.00 | 32.59 | C |
| ATOM | 6686 | C | VAL | E | 13 | 34.693 | 51.078 | 30.938 | 1.00 | 30.90 | C |
| ATOM | 6687 | O | VAL | E | 13 | 34.312 | 51.583 | 31.986 | 1.00 | 26.89 | O |
| ATOM | 6688 | CB | VAL | E | 13 | 32.649 | 50.331 | 29.596 | 1.00 | 33.09 | C |
| ATOM | 6689 | CG1 | VAL | E | 13 | 31.656 | 50.940 | 30.554 | 1.00 | 34.21 | C |
| ATOM | 6690 | CG2 | VAL | E | 13 | 32.936 | 51.274 | 28.469 | 1.00 | 33.83 | C |
| ATOM | 6691 | N | THR | E | 14 | 35.779 | 51.461 | 30.289 | 1.00 | 30.02 | N |
| ATOM | 6692 | CA | THR | E | 14 | 36.602 | 52.550 | 30.733 | 1.00 | 30.15 | C |
| ATOM | 6693 | C | THR | E | 14 | 35.779 | 53.792 | 30.564 | 1.00 | 30.20 | C |
| ATOM | 6694 | O | THR | E | 14 | 35.065 | 53.925 | 29.570 | 1.00 | 29.73 | O |
| ATOM | 6695 | CB | THR | E | 14 | 37.897 | 52.593 | 29.883 | 1.00 | 30.40 | C |
| ATOM | 6696 | OG1 | THR | E | 14 | 38.648 | 51.398 | 30.130 | 1.00 | 31.06 | O |
| ATOM | 6697 | CG2 | THR | E | 14 | 38.735 | 53.806 | 30.198 | 1.00 | 30.27 | C |
| ATOM | 6698 | N | LEU | E | 15 | 35.825 | 54.682 | 31.549 | 1.00 | 30.91 | N |
| ATOM | 6699 | CA | LEU | E | 15 | 35.096 | 55.939 | 31.450 | 1.00 | 31.33 | C |
| ATOM | 6700 | C | LEU | E | 15 | 35.440 | 56.617 | 30.164 | 1.00 | 32.04 | C |
| ATOM | 6701 | O | LEU | E | 15 | 36.595 | 56.560 | 29.715 | 1.00 | 32.65 | O |
| ATOM | 6702 | CB | LEU | E | 15 | 35.473 | 56.921 | 32.558 | 1.00 | 32.67 | C |
| ATOM | 6703 | CG | LEU | E | 15 | 34.890 | 56.756 | 33.949 | 1.00 | 33.64 | C |
| ATOM | 6704 | CD1 | LEU | E | 15 | 35.794 | 57.486 | 34.919 | 1.00 | 35.23 | C |
| ATOM | 6705 | CD2 | LEU | E | 15 | 33.461 | 57.282 | 34.024 | 1.00 | 34.21 | C |
| ATOM | 6706 | N | GLY | E | 16 | 34.462 | 57.318 | 29.607 | 1.00 | 29.85 | N |
| ATOM | 6707 | CA | GLY | E | 16 | 34.652 | 58.024 | 28.352 | 1.00 | 30.17 | C |
| ATOM | 6708 | C | GLY | E | 16 | 34.715 | 57.122 | 27.130 | 1.00 | 29.44 | C |
| ATOM | 6709 | O | GLY | E | 16 | 34.854 | 57.614 | 26.030 | 1.00 | 29.85 | O |
| ATOM | 6710 | N | THR | E | 17 | 34.616 | 55.807 | 27.314 | 1.00 | 29.20 | N |
| ATOM | 6711 | CA | THR | E | 17 | 34.538 | 54.905 | 26.181 | 1.00 | 29.13 | C |
| ATOM | 6712 | C | THR | E | 17 | 33.111 | 54.355 | 26.059 | 1.00 | 29.15 | C |
| ATOM | 6713 | O | THR | E | 17 | 32.234 | 54.628 | 26.895 | 1.00 | 29.06 | O |
| ATOM | 6714 | CB | THR | E | 17 | 35.558 | 53.752 | 26.260 | 1.00 | 30.32 | C |
| ATOM | 6715 | OG1 | THR | E | 17 | 35.212 | 52.857 | 27.315 | 1.00 | 29.92 | O |
| ATOM | 6716 | CG2 | THR | E | 17 | 37.000 | 54.286 | 26.446 | 1.00 | 30.90 | C |
| ATOM | 6717 | N | SER | E | 18 | 32.883 | 53.566 | 25.020 | 1.00 | 28.31 | N |
| ATOM | 6718 | CA | SER | E | 18 | 31.532 | 53.200 | 24.660 | 1.00 | 27.61 | C |
| ATOM | 6719 | C | SER | E | 18 | 31.092 | 51.856 | 25.266 | 1.00 | 26.41 | C |
| ATOM | 6720 | O | SER | E | 18 | 31.867 | 50.886 | 25.379 | 1.00 | 25.35 | O |
| ATOM | 6721 | CB | SER | E | 18 | 31.416 | 53.164 | 23.143 | 1.00 | 28.46 | C |
| ATOM | 6722 | OG | SER | E | 18 | 31.917 | 51.925 | 22.686 | 1.00 | 29.34 | O |
| ATOM | 6723 | N | ALA | E | 19 | 29.822 | 51.797 | 25.649 | 1.00 | 25.05 | N |
| ATOM | 6724 | CA | ALA | E | 19 | 29.250 | 50.562 | 26.158 | 1.00 | 24.80 | C |
| ATOM | 6725 | C | ALA | E | 19 | 28.178 | 50.042 | 25.207 | 1.00 | 23.48 | C |
| ATOM | 6726 | O | ALA | E | 19 | 27.481 | 50.821 | 24.589 | 1.00 | 21.31 | O |
| ATOM | 6727 | CB | ALA | E | 19 | 28.671 | 50.798 | 27.541 | 1.00 | 25.20 | C |
| ATOM | 6728 | N | SER | E | 20 | 28.063 | 48.723 | 25.110 | 1.00 | 21.87 | N |
| ATOM | 6729 | CA | SER | E | 20 | 27.074 | 48.069 | 24.243 | 1.00 | 22.55 | C |
| ATOM | 6730 | C | SER | E | 20 | 26.308 | 47.001 | 24.982 | 1.00 | 22.13 | C |
| ATOM | 6731 | O | SER | E | 20 | 26.894 | 45.987 | 25.414 | 1.00 | 22.15 | O |
| ATOM | 6732 | CB | SER | E | 20 | 27.751 | 47.423 | 23.028 | 1.00 | 21.01 | C |
| ATOM | 6733 | OG | SER | E | 20 | 26.814 | 46.746 | 22.194 | 1.00 | 22.92 | O |
| ATOM | 6734 | N | ILE | E | 21 | 24.993 | 47.192 | 25.104 | 1.00 | 21.00 | N |
| ATOM | 6735 | CA | ILE | E | 21 | 24.142 | 46.192 | 25.769 | 1.00 | 19.86 | C |
| ATOM | 6736 | C | ILE | E | 21 | 23.137 | 45.558 | 24.792 | 1.00 | 18.15 | C |
| ATOM | 6737 | O | ILE | E | 21 | 22.443 | 46.227 | 24.044 | 1.00 | 15.64 | O |
| ATOM | 6738 | CB | ILE | E | 21 | 23.485 | 46.816 | 27.027 | 1.00 | 19.94 | C |
| ATOM | 6739 | CG1 | ILE | E | 21 | 24.595 | 47.084 | 28.081 | 1.00 | 20.23 | C |
| ATOM | 6740 | CG2 | ILE | E | 21 | 22.391 | 45.948 | 27.571 | 1.00 | 18.93 | C |
| ATOM | 6741 | CD1 | ILE | E | 21 | 24.277 | 48.040 | 29.179 | 1.00 | 21.73 | C |
| ATOM | 6742 | N | SER | E | 22 | 23.059 | 44.235 | 24.819 | 1.00 | 19.51 | N |
| ATOM | 6743 | CA | SER | E | 22 | 22.247 | 43.488 | 23.871 | 1.00 | 19.51 | C |
| ATOM | 6744 | C | SER | E | 22 | 20.996 | 42.992 | 24.516 | 1.00 | 18.55 | C |
| ATOM | 6745 | O | SER | E | 22 | 20.959 | 42.803 | 25.734 | 1.00 | 16.58 | O |
| ATOM | 6746 | CB | SER | E | 22 | 23.018 | 42.260 | 23.374 | 1.00 | 21.54 | C |
| ATOM | 6747 | OG | SER | E | 22 | 24.237 | 42.656 | 22.784 | 1.00 | 27.01 | O |
| ATOM | 6748 | N | CYS | E | 23 | 19.977 | 42.753 | 23.700 | 1.00 | 17.99 | N |

```
ATOM  6749  CA   CYS E  23     18.784  42.052  24.148  1.00 18.14           C
ATOM  6750  C    CYS E  23     18.193  41.288  22.991  1.00 18.42           C
ATOM  6751  O    CYS E  23     18.453  41.573  21.827  1.00 17.75           O
ATOM  6752  CB   CYS E  23     17.746  43.034  24.751  1.00 18.95           C
ATOM  6753  SG   CYS E  23     16.162  42.299  25.249  1.00 20.62           S
ATOM  6754  N    ARG E  24     17.416  40.275  23.297  1.00 17.82           N
ATOM  6755  CA   ARG E  24     16.703  39.622  22.250  1.00 18.51           C
ATOM  6756  C    ARG E  24     15.390  39.099  22.736  1.00 16.71           C
ATOM  6757  O    ARG E  24     15.227  38.806  23.924  1.00 16.67           O
ATOM  6758  CB   ARG E  24     17.548  38.529  21.608  1.00 19.50           C
ATOM  6759  CG   ARG E  24     17.720  37.314  22.424  1.00 22.24           C
ATOM  6760  CD   ARG E  24     18.710  36.386  21.751  1.00 23.52           C
ATOM  6761  NE   ARG E  24     19.304  35.431  22.681  1.00 25.86           N
ATOM  6762  CZ   ARG E  24     18.698  34.343  23.147  1.00 27.35           C
ATOM  6763  NH1  ARG E  24     17.456  34.040  22.772  1.00 26.38           N
ATOM  6764  NH2  ARG E  24     19.352  33.556  23.987  1.00 29.16           N
ATOM  6765  N    SER E  25     14.445  39.008  21.803  1.00 14.56           N
ATOM  6766  CA   SER E  25     13.080  38.576  22.089  1.00 15.25           C
ATOM  6767  C    SER E  25     12.739  37.233  21.442  1.00 14.40           C
ATOM  6768  O    SER E  25     13.243  36.902  20.352  1.00 13.22           O
ATOM  6769  CB   SER E  25     12.112  39.614  21.549  1.00 17.29           C
ATOM  6770  OG   SER E  25     10.772  39.203  21.810  1.00 20.98           O
ATOM  6771  N    SER E  26     11.792  36.534  22.052  1.00 14.06           N
ATOM  6772  CA   SER E  26     11.306  35.242  21.558  1.00 15.41           C
ATOM  6773  C    SER E  26     10.284  35.389  20.431  1.00 16.36           C
ATOM  6774  O    SER E  26      9.922  34.418  19.801  1.00 17.84           O
ATOM  6775  CB   SER E  26     10.695  34.411  22.681  1.00 14.78           C
ATOM  6776  OG   SER E  26      9.636  35.063  23.345  1.00 14.66           O
ATOM  6777  N    LYS E  27      9.809  36.615  20.188  1.00 16.97           N
ATOM  6778  CA   LYS E  27      8.850  36.873  19.150  1.00 16.70           C
ATOM  6779  C    LYS E  27      9.217  38.246  18.596  1.00 15.42           C
ATOM  6780  O    LYS E  27      9.687  39.073  19.351  1.00 12.62           O
ATOM  6781  CB   LYS E  27      7.462  36.913  19.783  1.00 19.13           C
ATOM  6782  CG   LYS E  27      6.312  37.031  18.827  1.00 19.58           C
ATOM  6783  CD   LYS E  27      4.992  37.280  19.558  1.00 21.74           C
ATOM  6784  CE   LYS E  27      4.588  36.107  20.482  1.00 25.27           C
ATOM  6785  NZ   LYS E  27      4.557  34.737  19.821  1.00 27.90           N
ATOM  6786  N    SER E  27A     9.004  38.485  17.302  1.00 14.80           N
ATOM  6787  CA   SER E  27A     9.251  39.815  16.725  1.00 14.00           C
ATOM  6788  C    SER E  27A     8.403  40.893  17.404  1.00 14.65           C
ATOM  6789  O    SER E  27A     7.239  40.688  17.666  1.00 14.27           O
ATOM  6790  CB   SER E  27A     9.008  39.842  15.214  1.00 14.13           C
ATOM  6791  OG   SER E  27A     9.208  41.154  14.699  1.00 14.29           O
ATOM  6792  N    LEU E  27B     9.011  42.046  17.656  1.00 15.01           N
ATOM  6793  CA   LEU E  27B     8.305  43.190  18.264  1.00 15.02           C
ATOM  6794  C    LEU E  27B     8.046  44.284  17.233  1.00 15.27           C
ATOM  6795  O    LEU E  27B     7.614  45.410  17.585  1.00 14.76           O
ATOM  6796  CB   LEU E  27B     9.138  43.780  19.405  1.00 14.23           C
ATOM  6797  CG   LEU E  27B     9.577  42.780  20.484  1.00 12.02           C
ATOM  6798  CD1  LEU E  27B    10.376  43.430  21.556  1.00 11.41           C
ATOM  6799  CD2  LEU E  27B     8.378  42.043  21.060  1.00 11.45           C
ATOM  6800  N    LEU E  27C     8.388  43.966  15.980  1.00 15.58           N
ATOM  6801  CA   LEU E  27C     8.075  44.773  14.807  1.00 16.75           C
ATOM  6802  C    LEU E  27C     6.631  44.593  14.399  1.00 16.91           C
ATOM  6803  O    LEU E  27C     6.213  43.506  13.980  1.00 18.81           O
ATOM  6804  CB   LEU E  27C     8.948  44.406  13.619  1.00 15.49           C
ATOM  6805  CG   LEU E  27C     8.590  45.221  12.357  1.00 15.80           C
ATOM  6806  CD1  LEU E  27C     8.843  46.705  12.599  1.00 14.41           C
ATOM  6807  CD2  LEU E  27C     9.419  44.769  11.226  1.00 17.57           C
ATOM  6808  N    HIS E  27D     5.888  45.681  14.527  1.00 18.49           N
ATOM  6809  CA   HIS E  27D     4.459  45.724  14.302  1.00 18.24           C
ATOM  6810  C    HIS E  27D     4.178  46.101  12.854  1.00 18.26           C
ATOM  6811  O    HIS E  27D     5.010  46.733  12.192  1.00 18.09           O
ATOM  6812  CB   HIS E  27D     3.848  46.768  15.255  1.00 19.61           C
ATOM  6813  CG   HIS E  27D     2.356  46.786  15.272  1.00 20.58           C
```

```
ATOM   6814   ND1 HIS E   27D      1.619   46.117   16.222   1.00 24.35        N
ATOM   6815   CD2 HIS E   27D      1.455   47.375   14.451   1.00 21.01        C
ATOM   6816   CE1 HIS E   27D      0.330   46.319   16.002   1.00 22.26        C
ATOM   6817   NE2 HIS E   27D      0.206   47.077   14.929   1.00 22.58        N
ATOM   6818   N   SER E   27E      3.002   45.704   12.366   1.00 18.86        N
ATOM   6819   CA  SER E   27E      2.517   46.115   11.047   1.00 18.69        C
ATOM   6820   C   SER E   27E      2.530   47.637   10.831   1.00 18.24        C
ATOM   6821   O   SER E   27E      2.658   48.095    9.705   1.00 19.86        O
ATOM   6822   CB  SER E   27E      1.115   45.556   10.802   1.00 20.15        C
ATOM   6823   OG  SER E   27E      0.171   46.145   11.676   1.00 24.48        O
ATOM   6824   N   ASP E   28       2.483   48.411   11.906   1.00 17.43        N
ATOM   6825   CA  ASP E   28       2.494   49.894   11.817   1.00 17.36        C
ATOM   6826   C   ASP E   28       3.908   50.449   11.624   1.00 17.08        C
ATOM   6827   O   ASP E   28       4.105   51.671   11.540   1.00 17.00        O
ATOM   6828   CB  ASP E   28       1.789   50.533   13.029   1.00 18.09        C
ATOM   6829   CG  ASP E   28       2.508   50.275   14.364   1.00 19.86        C
ATOM   6830   OD1 ASP E   28       3.638   49.718   14.370   1.00 20.42        O
ATOM   6831   OD2 ASP E   28       1.933   50.636   15.423   1.00 19.13        O
ATOM   6832   N   GLY E   29       4.886   49.546   11.581   1.00 15.11        N
ATOM   6833   CA  GLY E   29       6.270   49.859   11.285   1.00 15.56        C
ATOM   6834   C   GLY E   29       7.108   50.219   12.494   1.00 14.38        C
ATOM   6835   O   GLY E   29       8.321   50.571   12.347   1.00 15.35        O
ATOM   6836   N   ILE E   30       6.466   50.211   13.673   1.00 14.17        N
ATOM   6837   CA  ILE E   30       7.145   50.530   14.932   1.00 13.90        C
ATOM   6838   C   ILE E   30       7.656   49.228   15.559   1.00 13.01        C
ATOM   6839   O   ILE E   30       6.955   48.215   15.545   1.00 11.66        O
ATOM   6840   CB  ILE E   30       6.202   51.303   15.926   1.00 14.78        C
ATOM   6841   CG1 ILE E   30       5.834   52.676   15.313   1.00 15.87        C
ATOM   6842   CG2 ILE E   30       6.848   51.503   17.291   1.00 13.46        C
ATOM   6843   CD1 ILE E   30       4.766   53.424   16.091   1.00 15.30        C
ATOM   6844   N   THR E   31       8.895   49.266   16.048   1.00 13.50        N
ATOM   6845   CA  THR E   31       9.423   48.137   16.841   1.00 12.48        C
ATOM   6846   C   THR E   31       9.233   48.489   18.289   1.00 11.44        C
ATOM   6847   O   THR E   31       9.874   49.369   18.802   1.00  9.55        O
ATOM   6848   CB  THR E   31      10.892   47.764   16.514   1.00 12.83        C
ATOM   6849   OG1 THR E   31      11.027   47.580   15.088   1.00 10.65        O
ATOM   6850   CG2 THR E   31      11.303   46.467   17.241   1.00 14.42        C
ATOM   6851   N   TYR E   32       8.312   47.791   18.948   1.00 11.96        N
ATOM   6852   CA  TYR E   32       7.907   48.150   20.305   1.00 12.94        C
ATOM   6853   C   TYR E   32       8.842   47.570   21.383   1.00 13.02        C
ATOM   6854   O   TYR E   32       8.414   46.786   22.212   1.00 11.97        O
ATOM   6855   CB  TYR E   32       6.478   47.732   20.562   1.00 14.00        C
ATOM   6856   CG  TYR E   32       5.467   48.582   19.820   1.00 14.12        C
ATOM   6857   CD1 TYR E   32       5.053   48.254   18.528   1.00 15.07        C
ATOM   6858   CD2 TYR E   32       4.941   49.711   20.411   1.00 16.14        C
ATOM   6859   CE1 TYR E   32       4.116   49.054   17.837   1.00 14.39        C
ATOM   6860   CE2 TYR E   32       4.014   50.519   19.731   1.00 15.76        C
ATOM   6861   CZ  TYR E   32       3.612   50.182   18.447   1.00 15.62        C
ATOM   6862   OH  TYR E   32       2.686   50.962   17.766   1.00 15.61        O
ATOM   6863   N   LEU E   33      10.091   48.021   21.340   1.00 14.22        N
ATOM   6864   CA  LEU E   33      11.163   47.634   22.238   1.00 13.24        C
ATOM   6865   C   LEU E   33      11.502   48.865   23.068   1.00 14.07        C
ATOM   6866   O   LEU E   33      11.683   49.955   22.521   1.00 12.53        O
ATOM   6867   CB  LEU E   33      12.378   47.206   21.437   1.00 12.52        C
ATOM   6868   CG  LEU E   33      13.636   46.664   22.148   1.00 14.05        C
ATOM   6869   CD1 LEU E   33      14.514   47.742   22.748   1.00 12.19        C
ATOM   6870   CD2 LEU E   33      13.288   45.665   23.222   1.00 13.71        C
ATOM   6871   N   TYR E   34      11.605   48.675   24.374   1.00 13.33        N
ATOM   6872   CA  TYR E   34      11.846   49.749   25.315   1.00 12.78        C
ATOM   6873   C   TYR E   34      13.049   49.422   26.175   1.00 13.17        C
ATOM   6874   O   TYR E   34      13.293   48.255   26.490   1.00 11.90        O
ATOM   6875   CB  TYR E   34      10.613   49.904   26.235   1.00 12.49        C
ATOM   6876   CG  TYR E   34       9.390   50.401   25.517   1.00 13.44        C
ATOM   6877   CD1 TYR E   34       8.701   49.596   24.621   1.00 12.02        C
ATOM   6878   CD2 TYR E   34       8.910   51.677   25.747   1.00 13.58        C
```

| ATOM | 6879 | CE1 | TYR | E | 34 | 7.574 | 50.073 | 23.942 | 1.00 | 13.46 | C |
|------|------|-----|-----|---|----|-------|--------|--------|------|-------|---|
| ATOM | 6880 | CE2 | TYR | E | 34 | 7.793 | 52.138 | 25.100 | 1.00 | 12.69 | C |
| ATOM | 6881 | CZ | TYR | E | 34 | 7.135 | 51.346 | 24.190 | 1.00 | 14.23 | C |
| ATOM | 6882 | OH | TYR | E | 34 | 6.008 | 51.832 | 23.568 | 1.00 | 15.30 | O |
| ATOM | 6883 | N | TRP | E | 35 | 13.763 | 50.451 | 26.618 | 1.00 | 11.53 | N |
| ATOM | 6884 | CA | TRP | E | 35 | 14.867 | 50.272 | 27.542 | 1.00 | 12.98 | C |
| ATOM | 6885 | C | TRP | E | 35 | 14.635 | 51.151 | 28.767 | 1.00 | 14.44 | C |
| ATOM | 6886 | O | TRP | E | 35 | 14.267 | 52.342 | 28.624 | 1.00 | 13.63 | O |
| ATOM | 6887 | CB | TRP | E | 35 | 16.210 | 50.687 | 26.933 | 1.00 | 13.35 | C |
| ATOM | 6888 | CG | TRP | E | 35 | 16.765 | 49.891 | 25.844 | 1.00 | 13.29 | C |
| ATOM | 6889 | CD1 | TRP | E | 35 | 16.781 | 50.203 | 24.535 | 1.00 | 14.18 | C |
| ATOM | 6890 | CD2 | TRP | E | 35 | 17.470 | 48.647 | 25.972 | 1.00 | 14.60 | C |
| ATOM | 6891 | NE1 | TRP | E | 35 | 17.441 | 49.223 | 23.813 | 1.00 | 13.52 | N |
| ATOM | 6892 | CE2 | TRP | E | 35 | 17.881 | 48.266 | 24.680 | 1.00 | 14.31 | C |
| ATOM | 6893 | CE3 | TRP | E | 35 | 17.814 | 47.844 | 27.048 | 1.00 | 15.58 | C |
| ATOM | 6894 | CZ2 | TRP | E | 35 | 18.586 | 47.088 | 24.440 | 1.00 | 14.37 | C |
| ATOM | 6895 | CZ3 | TRP | E | 35 | 18.525 | 46.694 | 26.803 | 1.00 | 13.21 | C |
| ATOM | 6896 | CH2 | TRP | E | 35 | 18.922 | 46.349 | 25.519 | 1.00 | 13.04 | C |
| ATOM | 6897 | N | TYR | E | 36 | 14.853 | 50.557 | 29.944 | 1.00 | 14.46 | N |
| ATOM | 6898 | CA | TYR | E | 36 | 14.763 | 51.205 | 31.251 | 1.00 | 13.31 | C |
| ATOM | 6899 | C | TYR | E | 36 | 16.136 | 51.165 | 31.966 | 1.00 | 15.34 | C |
| ATOM | 6900 | O | TYR | E | 36 | 16.865 | 50.206 | 31.833 | 1.00 | 13.32 | O |
| ATOM | 6901 | CB | TYR | E | 36 | 13.685 | 50.471 | 32.106 | 1.00 | 13.93 | C |
| ATOM | 6902 | CG | TYR | E | 36 | 12.298 | 50.556 | 31.503 | 1.00 | 13.14 | C |
| ATOM | 6903 | CD1 | TYR | E | 36 | 11.792 | 49.565 | 30.644 | 1.00 | 14.53 | C |
| ATOM | 6904 | CD2 | TYR | E | 36 | 11.481 | 51.649 | 31.789 | 1.00 | 12.65 | C |
| ATOM | 6905 | CE1 | TYR | E | 36 | 10.505 | 49.691 | 30.076 | 1.00 | 14.16 | C |
| ATOM | 6906 | CE2 | TYR | E | 36 | 10.237 | 51.776 | 31.248 | 1.00 | 13.96 | C |
| ATOM | 6907 | CZ | TYR | E | 36 | 9.742 | 50.829 | 30.384 | 1.00 | 11.70 | C |
| ATOM | 6908 | OH | TYR | E | 36 | 8.484 | 51.046 | 29.888 | 1.00 | 13.79 | O |
| ATOM | 6909 | N | LEU | E | 37 | 16.496 | 52.216 | 32.717 | 1.00 | 13.49 | N |
| ATOM | 6910 | CA | LEU | E | 37 | 17.653 | 52.169 | 33.612 | 1.00 | 13.00 | C |
| ATOM | 6911 | C | LEU | E | 37 | 17.173 | 52.136 | 35.065 | 1.00 | 13.88 | C |
| ATOM | 6912 | O | LEU | E | 37 | 16.264 | 52.840 | 35.425 | 1.00 | 14.60 | O |
| ATOM | 6913 | CB | LEU | E | 37 | 18.590 | 53.350 | 33.360 | 1.00 | 13.31 | C |
| ATOM | 6914 | CG | LEU | E | 37 | 19.783 | 53.573 | 34.264 | 1.00 | 13.32 | C |
| ATOM | 6915 | CD1 | LEU | E | 37 | 20.682 | 52.360 | 34.411 | 1.00 | 12.50 | C |
| ATOM | 6916 | CD2 | LEU | E | 37 | 20.594 | 54.790 | 33.798 | 1.00 | 13.83 | C |
| ATOM | 6917 | N | GLN | E | 38 | 17.727 | 51.252 | 35.882 | 1.00 | 14.38 | N |
| ATOM | 6918 | CA | GLN | E | 38 | 17.493 | 51.286 | 37.322 | 1.00 | 16.54 | C |
| ATOM | 6919 | C | GLN | E | 38 | 18.839 | 51.509 | 38.026 | 1.00 | 18.40 | C |
| ATOM | 6920 | O | GLN | E | 38 | 19.670 | 50.599 | 38.161 | 1.00 | 15.21 | O |
| ATOM | 6921 | CB | GLN | E | 38 | 16.783 | 50.028 | 37.818 | 1.00 | 16.44 | C |
| ATOM | 6922 | CG | GLN | E | 38 | 16.434 | 50.123 | 39.288 | 1.00 | 15.77 | C |
| ATOM | 6923 | CD | GLN | E | 38 | 15.571 | 49.055 | 39.761 | 1.00 | 16.60 | C |
| ATOM | 6924 | OE1 | GLN | E | 38 | 15.675 | 47.899 | 39.303 | 1.00 | 18.07 | O |
| ATOM | 6925 | NE2 | GLN | E | 38 | 14.714 | 49.386 | 40.736 | 1.00 | 13.44 | N |
| ATOM | 6926 | N | LYS | E | 39 | 19.044 | 52.757 | 38.438 | 1.00 | 20.19 | N |
| ATOM | 6927 | CA | LYS | E | 39 | 20.248 | 53.173 | 39.153 | 1.00 | 22.76 | C |
| ATOM | 6928 | C | LYS | E | 39 | 20.205 | 52.668 | 40.588 | 1.00 | 23.63 | C |
| ATOM | 6929 | O | LYS | E | 39 | 19.142 | 52.307 | 41.107 | 1.00 | 24.11 | O |
| ATOM | 6930 | CB | LYS | E | 39 | 20.404 | 54.704 | 39.094 | 1.00 | 22.82 | C |
| ATOM | 6931 | CG | LYS | E | 39 | 20.745 | 55.239 | 37.709 | 1.00 | 24.71 | C |
| ATOM | 6932 | CD | LYS | E | 39 | 21.041 | 56.703 | 37.697 | 1.00 | 25.06 | C |
| ATOM | 6933 | CE | LYS | E | 39 | 21.350 | 57.194 | 36.306 | 1.00 | 26.13 | C |
| ATOM | 6934 | NZ | LYS | E | 39 | 21.451 | 58.694 | 36.267 | 1.00 | 28.11 | N |
| ATOM | 6935 | N | PRO | E | 40 | 21.378 | 52.605 | 41.245 | 1.00 | 27.71 | N |
| ATOM | 6936 | CA | PRO | E | 40 | 21.391 | 51.986 | 42.572 | 1.00 | 27.89 | C |
| ATOM | 6937 | C | PRO | E | 40 | 20.414 | 52.643 | 43.545 | 1.00 | 28.61 | C |
| ATOM | 6938 | O | PRO | E | 40 | 20.384 | 53.877 | 43.668 | 1.00 | 29.13 | O |
| ATOM | 6939 | CB | PRO | E | 40 | 22.850 | 52.175 | 43.038 | 1.00 | 28.97 | C |
| ATOM | 6940 | CG | PRO | E | 40 | 23.648 | 52.345 | 41.778 | 1.00 | 29.83 | C |
| ATOM | 6941 | CD | PRO | E | 40 | 22.725 | 53.073 | 40.836 | 1.00 | 27.88 | C |
| ATOM | 6942 | N | GLY | E | 41 | 19.599 | 51.819 | 44.190 | 1.00 | 29.20 | N |
| ATOM | 6943 | CA | GLY | E | 41 | 18.616 | 52.298 | 45.152 | 1.00 | 28.71 | C |

| ATOM | 6944 | C   | GLY | E | 41 | 17.445 | 53.110 | 44.602 | 1.00 | 30.02 | C |
|------|------|-----|-----|---|----|--------|--------|--------|------|-------|---|
| ATOM | 6945 | O   | GLY | E | 41 | 16.669 | 53.659 | 45.389 | 1.00 | 31.17 | O |
| ATOM | 6946 | N   | GLN | E | 42 | 17.295 | 53.194 | 43.275 | 1.00 | 28.21 | N |
| ATOM | 6947 | CA  | GLN | E | 42 | 16.204 | 53.960 | 42.670 | 1.00 | 26.18 | C |
| ATOM | 6948 | C   | GLN | E | 42 | 15.200 | 53.053 | 41.971 | 1.00 | 23.60 | C |
| ATOM | 6949 | O   | GLN | E | 42 | 15.372 | 51.823 | 41.910 | 1.00 | 21.90 | O |
| ATOM | 6950 | CB  | GLN | E | 42 | 16.740 | 54.965 | 41.661 | 1.00 | 26.23 | C |
| ATOM | 6951 | CG  | GLN | E | 42 | 17.577 | 56.114 | 42.216 | 1.00 | 29.92 | C |
| ATOM | 6952 | CD  | GLN | E | 42 | 18.135 | 56.978 | 41.092 | 1.00 | 30.50 | C |
| ATOM | 6953 | OE1 | GLN | E | 42 | 17.777 | 56.796 | 39.924 | 1.00 | 34.89 | O |
| ATOM | 6954 | NE2 | GLN | E | 42 | 19.002 | 57.936 | 41.433 | 1.00 | 33.53 | N |
| ATOM | 6955 | N   | SER | E | 43 | 14.112 | 53.672 | 41.488 | 1.00 | 20.06 | N |
| ATOM | 6956 | CA  | SER | E | 43 | 13.136 | 53.000 | 40.671 | 1.00 | 19.20 | C |
| ATOM | 6957 | C   | SER | E | 43 | 13.690 | 52.938 | 39.273 | 1.00 | 17.11 | C |
| ATOM | 6958 | O   | SER | E | 43 | 14.626 | 53.685 | 38.951 | 1.00 | 17.27 | O |
| ATOM | 6959 | CB  | SER | E | 43 | 11.811 | 53.747 | 40.678 | 1.00 | 19.20 | C |
| ATOM | 6960 | OG  | SER | E | 43 | 11.337 | 53.804 | 42.011 | 1.00 | 22.38 | O |
| ATOM | 6961 | N   | PRO | E | 44 | 13.134 | 52.050 | 38.433 | 1.00 | 15.80 | N |
| ATOM | 6962 | CA  | PRO | E | 44 | 13.511 | 52.155 | 37.025 | 1.00 | 15.09 | C |
| ATOM | 6963 | C   | PRO | E | 44 | 13.046 | 53.485 | 36.419 | 1.00 | 14.18 | C |
| ATOM | 6964 | O   | PRO | E | 44 | 12.115 | 54.116 | 36.926 | 1.00 | 14.66 | O |
| ATOM | 6965 | CB  | PRO | E | 44 | 12.781 | 50.994 | 36.374 | 1.00 | 15.13 | C |
| ATOM | 6966 | CG  | PRO | E | 44 | 12.397 | 50.079 | 37.500 | 1.00 | 15.80 | C |
| ATOM | 6967 | CD  | PRO | E | 44 | 12.161 | 50.956 | 38.662 | 1.00 | 15.23 | C |
| ATOM | 6968 | N   | HIS | E | 45 | 13.713 | 53.910 | 35.359 | 1.00 | 13.64 | N |
| ATOM | 6969 | CA  | HIS | E | 45 | 13.199 | 54.991 | 34.554 | 1.00 | 13.08 | C |
| ATOM | 6970 | C   | HIS | E | 45 | 13.392 | 54.717 | 33.065 | 1.00 | 13.98 | C |
| ATOM | 6971 | O   | HIS | E | 45 | 14.365 | 54.080 | 32.669 | 1.00 | 13.47 | O |
| ATOM | 6972 | CB  | HIS | E | 45 | 13.845 | 56.336 | 34.965 | 1.00 | 14.99 | C |
| ATOM | 6973 | CG  | HIS | E | 45 | 15.305 | 56.470 | 34.631 | 1.00 | 18.20 | C |
| ATOM | 6974 | ND1 | HIS | E | 45 | 16.308 | 56.176 | 35.534 | 1.00 | 19.15 | N |
| ATOM | 6975 | CD2 | HIS | E | 45 | 15.928 | 56.950 | 33.529 | 1.00 | 20.38 | C |
| ATOM | 6976 | CE1 | HIS | E | 45 | 17.482 | 56.451 | 34.993 | 1.00 | 17.51 | C |
| ATOM | 6977 | NE2 | HIS | E | 45 | 17.277 | 56.911 | 33.772 | 1.00 | 18.45 | N |
| ATOM | 6978 | N   | LEU | E | 46 | 12.468 | 55.223 | 32.244 | 1.00 | 13.85 | N |
| ATOM | 6979 | CA  | LEU | E | 46 | 12.497 | 54.984 | 30.820 | 1.00 | 13.07 | C |
| ATOM | 6980 | C   | LEU | E | 46 | 13.637 | 55.732 | 30.189 | 1.00 | 15.06 | C |
| ATOM | 6981 | O   | LEU | E | 46 | 13.770 | 56.961 | 30.406 | 1.00 | 14.07 | O |
| ATOM | 6982 | CB  | LEU | E | 46 | 11.170 | 55.446 | 30.178 | 1.00 | 13.07 | C |
| ATOM | 6983 | CG  | LEU | E | 46 | 11.027 | 55.222 | 28.693 | 1.00 | 12.00 | C |
| ATOM | 6984 | CD1 | LEU | E | 46 | 10.930 | 53.739 | 28.386 | 1.00 | 14.04 | C |
| ATOM | 6985 | CD2 | LEU | E | 46 |  9.864 | 55.935 | 28.122 | 1.00 | 13.79 | C |
| ATOM | 6986 | N   | LEU | E | 47 | 14.398 | 55.044 | 29.364 | 1.00 | 14.76 | N |
| ATOM | 6987 | CA  | LEU | E | 47 | 15.490 | 55.640 | 28.590 | 1.00 | 16.97 | C |
| ATOM | 6988 | C   | LEU | E | 47 | 15.111 | 55.759 | 27.118 | 1.00 | 16.55 | C |
| ATOM | 6989 | O   | LEU | E | 47 | 15.266 | 56.780 | 26.510 | 1.00 | 17.47 | O |
| ATOM | 6990 | CB  | LEU | E | 47 | 16.732 | 54.757 | 28.645 | 1.00 | 18.80 | C |
| ATOM | 6991 | CG  | LEU | E | 47 | 17.591 | 54.694 | 29.872 | 1.00 | 22.90 | C |
| ATOM | 6992 | CD1 | LEU | E | 47 | 18.661 | 53.659 | 29.629 | 1.00 | 26.22 | C |
| ATOM | 6993 | CD2 | LEU | E | 47 | 18.198 | 56.059 | 30.148 | 1.00 | 23.72 | C |
| ATOM | 6994 | N   | ILE | E | 48 | 14.628 | 54.668 | 26.549 | 1.00 | 16.45 | N |
| ATOM | 6995 | CA  | ILE | E | 48 | 14.408 | 54.576 | 25.125 | 1.00 | 16.69 | C |
| ATOM | 6996 | C   | ILE | E | 48 | 13.065 | 53.898 | 24.914 | 1.00 | 15.66 | C |
| ATOM | 6997 | O   | ILE | E | 48 | 12.772 | 52.929 | 25.613 | 1.00 | 15.11 | O |
| ATOM | 6998 | CB  | ILE | E | 48 | 15.458 | 53.618 | 24.454 | 1.00 | 16.12 | C |
| ATOM | 6999 | CG1 | ILE | E | 48 | 16.924 | 54.110 | 24.613 | 1.00 | 17.87 | C |
| ATOM | 7000 | CG2 | ILE | E | 48 | 15.045 | 53.238 | 23.020 | 1.00 | 15.80 | C |
| ATOM | 7001 | CD1 | ILE | E | 48 | 17.435 | 55.026 | 23.608 | 1.00 | 18.90 | C |
| ATOM | 7002 | N   | TYR | E | 49 | 12.276 | 54.403 | 23.960 | 1.00 | 16.40 | N |
| ATOM | 7003 | CA  | TYR | E | 49 | 10.972 | 53.813 | 23.597 | 1.00 | 16.71 | C |
| ATOM | 7004 | C   | TYR | E | 49 | 10.873 | 53.583 | 22.089 | 1.00 | 15.70 | C |
| ATOM | 7005 | O   | TYR | E | 49 | 11.544 | 54.257 | 21.330 | 1.00 | 17.64 | O |
| ATOM | 7006 | CB  | TYR | E | 49 |  9.798 | 54.683 | 24.081 | 1.00 | 17.23 | C |
| ATOM | 7007 | CG  | TYR | E | 49 |  9.688 | 56.052 | 23.431 | 1.00 | 16.85 | C |
| ATOM | 7008 | CD1 | TYR | E | 49 | 10.418 | 57.130 | 23.911 | 1.00 | 17.04 | C |

```
ATOM   7009  CD2 TYR E  49      8.864  56.255  22.324  1.00 17.98          C
ATOM   7010  CE1 TYR E  49     10.312  58.387  23.312  1.00 17.63          C
ATOM   7011  CE2 TYR E  49      8.754  57.502  21.710  1.00 16.97          C
ATOM   7012  CZ  TYR E  49      9.478  58.562  22.214  1.00 19.06          C
ATOM   7013  OH  TYR E  49      9.370  59.818  21.608  1.00 19.42          O
ATOM   7014  N   HIS E  50     10.064  52.606  21.654  1.00 15.95          N
ATOM   7015  CA  HIS E  50      9.896  52.324  20.221  1.00 15.68          C
ATOM   7016  C   HIS E  50     11.267  52.155  19.554  1.00 16.01          C
ATOM   7017  O   HIS E  50     11.591  52.788  18.533  1.00 14.87          O
ATOM   7018  CB  HIS E  50      9.135  53.444  19.499  1.00 15.25          C
ATOM   7019  CG  HIS E  50      7.720  53.614  19.933  1.00 15.37          C
ATOM   7020  ND1 HIS E  50      6.935  54.646  19.471  1.00 16.22          N
ATOM   7021  CD2 HIS E  50      6.936  52.885  20.757  1.00 16.00          C
ATOM   7022  CE1 HIS E  50      5.730  54.547  20.002  1.00 17.29          C
ATOM   7023  NE2 HIS E  50      5.708  53.485  20.787  1.00 16.24          N
ATOM   7024  N   LEU E  51     12.092  51.328  20.178  1.00 14.40          N
ATOM   7025  CA  LEU E  51     13.400  50.914  19.674  1.00 14.70          C
ATOM   7026  C   LEU E  51     14.478  51.976  19.691  1.00 15.17          C
ATOM   7027  O   LEU E  51     15.595  51.721  20.146  1.00 15.67          O
ATOM   7028  CB  LEU E  51     13.298  50.259  18.277  1.00 15.58          C
ATOM   7029  CG  LEU E  51     14.564  49.589  17.756  1.00 14.48          C
ATOM   7030  CD1 LEU E  51     14.972  48.449  18.657  1.00 18.07          C
ATOM   7031  CD2 LEU E  51     14.366  49.090  16.335  1.00 15.39          C
ATOM   7032  N   SER E  52     14.155  53.162  19.201  1.00 14.37          N
ATOM   7033  CA  SER E  52     15.187  54.129  18.912  1.00 16.79          C
ATOM   7034  C   SER E  52     14.947  55.539  19.404  1.00 17.38          C
ATOM   7035  O   SER E  52     15.829  56.374  19.293  1.00 16.87          O
ATOM   7036  CB  SER E  52     15.471  54.083  17.409  1.00 17.47          C
ATOM   7037  OG  SER E  52     16.006  52.803  17.113  1.00 19.43          O
ATOM   7038  N   ASN E  53     13.802  55.802  20.004  1.00 17.86          N
ATOM   7039  CA  ASN E  53     13.507  57.169  20.462  1.00 18.57          C
ATOM   7040  C   ASN E  53     13.921  57.420  21.912  1.00 18.48          C
ATOM   7041  O   ASN E  53     13.539  56.693  22.801  1.00 17.20          O
ATOM   7042  CB  ASN E  53     12.028  57.453  20.266  1.00 20.07          C
ATOM   7043  CG  ASN E  53     11.569  57.190  18.841  1.00 21.36          C
ATOM   7044  OD1 ASN E  53     12.339  57.332  17.882  1.00 26.06          O
ATOM   7045  ND2 ASN E  53     10.312  56.821  18.691  1.00 27.46          N
ATOM   7046  N   LEU E  54     14.684  58.474  22.154  1.00 20.75          N
ATOM   7047  CA  LEU E  54     15.086  58.827  23.524  1.00 21.64          C
ATOM   7048  C   LEU E  54     13.995  59.510  24.363  1.00 21.99          C
ATOM   7049  O   LEU E  54     13.376  60.488  23.927  1.00 20.71          O
ATOM   7050  CB  LEU E  54     16.322  59.720  23.496  1.00 23.79          C
ATOM   7051  CG  LEU E  54     17.657  58.963  23.437  1.00 22.83          C
ATOM   7052  CD1 LEU E  54     17.825  58.255  22.092  1.00 24.82          C
ATOM   7053  CD2 LEU E  54     18.786  59.910  23.669  1.00 23.14          C
ATOM   7054  N   ALA E  55     13.791  59.042  25.591  1.00 20.94          N
ATOM   7055  CA  ALA E  55     12.867  59.731  26.497  1.00 21.90          C
ATOM   7056  C   ALA E  55     13.390  61.141  26.814  1.00 22.50          C
ATOM   7057  O   ALA E  55     14.590  61.415  26.690  1.00 22.51          O
ATOM   7058  CB  ALA E  55     12.682  58.941  27.769  1.00 21.15          C
ATOM   7059  N   SER E  56     12.502  62.024  27.248  1.00 25.28          N
ATOM   7060  CA  SER E  56     12.886  63.422  27.541  1.00 26.37          C
ATOM   7061  C   SER E  56     13.954  63.479  28.612  1.00 27.37          C
ATOM   7062  O   SER E  56     13.866  62.788  29.623  1.00 28.00          O
ATOM   7063  CB  SER E  56     11.685  64.244  27.999  1.00 27.80          C
ATOM   7064  OG  SER E  56     12.094  65.585  28.279  1.00 32.62          O
ATOM   7065  N   GLY E  57     14.989  64.280  28.390  1.00 28.23          N
ATOM   7066  CA  GLY E  57     16.056  64.407  29.381  1.00 28.35          C
ATOM   7067  C   GLY E  57     17.141  63.357  29.339  1.00 28.76          C
ATOM   7068  O   GLY E  57     18.069  63.403  30.141  1.00 30.26          O
ATOM   7069  N   VAL E  58     17.051  62.413  28.407  1.00 28.47          N
ATOM   7070  CA  VAL E  58     18.061  61.370  28.255  1.00 27.90          C
ATOM   7071  C   VAL E  58     19.113  61.935  27.313  1.00 28.18          C
ATOM   7072  O   VAL E  58     18.772  62.433  26.261  1.00 28.82          O
ATOM   7073  CB  VAL E  58     17.428  60.065  27.663  1.00 26.92          C
```

| ATOM | 7074 | CG1 | VAL | E | 58 | 18.482 | 59.012 | 27.375 | 1.00 | 26.05 | C |
|------|------|-----|-----|---|----|--------|--------|--------|------|-------|---|
| ATOM | 7075 | CG2 | VAL | E | 58 | 16.374 | 59.523 | 28.611 | 1.00 | 26.17 | C |
| ATOM | 7076 | N | PRO | E | 59 | 20.397 | 61.900 | 27.697 | 1.00 | 29.43 | N |
| ATOM | 7077 | CA | PRO | E | 59 | 21.357 | 62.590 | 26.838 | 1.00 | 29.60 | C |
| ATOM | 7078 | C | PRO | E | 59 | 21.642 | 61.797 | 25.563 | 1.00 | 29.69 | C |
| ATOM | 7079 | O | PRO | E | 59 | 21.482 | 60.582 | 25.538 | 1.00 | 27.82 | O |
| ATOM | 7080 | CB | PRO | E | 59 | 22.595 | 62.704 | 27.731 | 1.00 | 29.64 | C |
| ATOM | 7081 | CG | PRO | E | 59 | 22.524 | 61.539 | 28.608 | 1.00 | 29.95 | C |
| ATOM | 7082 | CD | PRO | E | 59 | 21.052 | 61.280 | 28.860 | 1.00 | 29.83 | C |
| ATOM | 7083 | N | ASP | E | 60 | 22.066 | 62.463 | 24.497 | 1.00 | 29.73 | N |
| ATOM | 7084 | CA | ASP | E | 60 | 22.179 | 61.763 | 23.213 | 1.00 | 29.82 | C |
| ATOM | 7085 | C | ASP | E | 60 | 23.403 | 60.824 | 23.088 | 1.00 | 28.84 | C |
| ATOM | 7086 | O | ASP | E | 60 | 23.657 | 60.273 | 22.024 | 1.00 | 28.56 | O |
| ATOM | 7087 | CB | ASP | E | 60 | 22.055 | 62.750 | 22.046 | 1.00 | 32.62 | C |
| ATOM | 7088 | CG | ASP | E | 60 | 23.313 | 63.513 | 21.795 | 1.00 | 34.69 | C |
| ATOM | 7089 | OD1 | ASP | E | 60 | 24.117 | 63.677 | 22.737 | 1.00 | 37.67 | O |
| ATOM | 7090 | OD2 | ASP | E | 60 | 23.493 | 63.944 | 20.633 | 1.00 | 39.72 | O |
| ATOM | 7091 | N | ARG | E | 61 | 24.112 | 60.616 | 24.196 | 1.00 | 27.09 | N |
| ATOM | 7092 | CA | ARG | E | 61 | 25.123 | 59.561 | 24.309 | 1.00 | 26.91 | C |
| ATOM | 7093 | C | ARG | E | 61 | 24.464 | 58.183 | 24.248 | 1.00 | 24.83 | C |
| ATOM | 7094 | O | ARG | E | 61 | 25.125 | 57.182 | 23.964 | 1.00 | 25.27 | O |
| ATOM | 7095 | CB | ARG | E | 61 | 25.894 | 59.681 | 25.627 | 1.00 | 27.90 | C |
| ATOM | 7096 | CG | ARG | E | 61 | 26.143 | 61.111 | 26.108 | 1.00 | 29.97 | C |
| ATOM | 7097 | CD | ARG | E | 61 | 27.089 | 61.179 | 27.327 | 1.00 | 30.40 | C |
| ATOM | 7098 | NE | ARG | E | 61 | 26.401 | 61.081 | 28.626 | 1.00 | 31.56 | N |
| ATOM | 7099 | CZ | ARG | E | 61 | 26.230 | 59.961 | 29.325 | 1.00 | 28.94 | C |
| ATOM | 7100 | NH1 | ARG | E | 61 | 26.678 | 58.800 | 28.861 | 1.00 | 29.70 | N |
| ATOM | 7101 | NH2 | ARG | E | 61 | 25.614 | 60.021 | 30.497 | 1.00 | 28.29 | N |
| ATOM | 7102 | N | PHE | E | 62 | 23.168 | 58.127 | 24.557 | 1.00 | 23.36 | N |
| ATOM | 7103 | CA | PHE | E | 62 | 22.376 | 56.898 | 24.462 | 1.00 | 22.03 | C |
| ATOM | 7104 | C | PHE | E | 62 | 21.703 | 56.761 | 23.087 | 1.00 | 20.43 | C |
| ATOM | 7105 | O | PHE | E | 62 | 21.104 | 57.687 | 22.583 | 1.00 | 17.35 | O |
| ATOM | 7106 | CB | PHE | E | 62 | 21.302 | 56.880 | 25.563 | 1.00 | 22.27 | C |
| ATOM | 7107 | CG | PHE | E | 62 | 21.872 | 56.843 | 26.962 | 1.00 | 22.55 | C |
| ATOM | 7108 | CD1 | PHE | E | 62 | 22.192 | 58.013 | 27.628 | 1.00 | 22.02 | C |
| ATOM | 7109 | CD2 | PHE | E | 62 | 22.092 | 55.624 | 27.612 | 1.00 | 21.81 | C |
| ATOM | 7110 | CE1 | PHE | E | 62 | 22.732 | 57.981 | 28.916 | 1.00 | 21.81 | C |
| ATOM | 7111 | CE2 | PHE | E | 62 | 22.659 | 55.593 | 28.898 | 1.00 | 21.42 | C |
| ATOM | 7112 | CZ | PHE | E | 62 | 22.967 | 56.777 | 29.544 | 1.00 | 20.98 | C |
| ATOM | 7113 | N | SER | E | 63 | 21.860 | 55.595 | 22.466 | 1.00 | 18.19 | N |
| ATOM | 7114 | CA | SER | E | 63 | 21.148 | 55.253 | 21.245 | 1.00 | 17.31 | C |
| ATOM | 7115 | C | SER | E | 63 | 20.811 | 53.806 | 21.259 | 1.00 | 16.85 | C |
| ATOM | 7116 | O | SER | E | 63 | 21.410 | 53.031 | 22.005 | 1.00 | 18.34 | O |
| ATOM | 7117 | CB | SER | E | 63 | 21.974 | 55.553 | 19.975 | 1.00 | 16.80 | C |
| ATOM | 7118 | OG | SER | E | 63 | 23.250 | 54.953 | 20.019 | 1.00 | 15.81 | O |
| ATOM | 7119 | N | SER | E | 64 | 19.898 | 53.425 | 20.377 | 1.00 | 15.63 | N |
| ATOM | 7120 | CA | SER | E | 64 | 19.471 | 52.027 | 20.282 | 1.00 | 15.73 | C |
| ATOM | 7121 | C | SER | E | 64 | 19.014 | 51.730 | 18.870 | 1.00 | 15.25 | C |
| ATOM | 7122 | O | SER | E | 64 | 18.503 | 52.603 | 18.171 | 1.00 | 14.26 | O |
| ATOM | 7123 | CB | SER | E | 64 | 18.338 | 51.785 | 21.285 | 1.00 | 14.52 | C |
| ATOM | 7124 | OG | SER | E | 64 | 17.753 | 50.500 | 21.171 | 1.00 | 14.56 | O |
| ATOM | 7125 | N | SER | E | 65 | 19.179 | 50.470 | 18.481 | 1.00 | 15.14 | N |
| ATOM | 7126 | CA | SER | E | 65 | 18.825 | 49.999 | 17.175 | 1.00 | 15.42 | C |
| ATOM | 7127 | C | SER | E | 65 | 18.466 | 48.534 | 17.336 | 1.00 | 14.62 | C |
| ATOM | 7128 | O | SER | E | 65 | 18.714 | 47.960 | 18.396 | 1.00 | 13.71 | O |
| ATOM | 7129 | CB | SER | E | 65 | 20.023 | 50.144 | 16.214 | 1.00 | 17.02 | C |
| ATOM | 7130 | OG | SER | E | 65 | 21.117 | 49.335 | 16.622 | 1.00 | 18.51 | O |
| ATOM | 7131 | N | GLY | E | 66 | 17.857 | 47.953 | 16.309 | 1.00 | 13.87 | N |
| ATOM | 7132 | CA | GLY | E | 66 | 17.593 | 46.499 | 16.272 | 1.00 | 14.39 | C |
| ATOM | 7133 | C | GLY | E | 66 | 16.551 | 46.086 | 15.248 | 1.00 | 12.22 | C |
| ATOM | 7134 | O | GLY | E | 66 | 15.968 | 46.901 | 14.574 | 1.00 | 12.08 | O |
| ATOM | 7135 | N | SER | E | 67 | 16.343 | 44.773 | 15.095 | 1.00 | 12.61 | N |
| ATOM | 7136 | CA | SER | E | 67 | 15.319 | 44.266 | 14.244 | 1.00 | 13.57 | C |
| ATOM | 7137 | C | SER | E | 67 | 14.078 | 44.104 | 15.116 | 1.00 | 15.82 | C |
| ATOM | 7138 | O | SER | E | 67 | 13.961 | 44.740 | 16.151 | 1.00 | 16.10 | O |

| | | | | | | | | | | |
|---|---|---|---|---|---|---|---|---|---|---|
| ATOM | 7139 | CB | SER | E | 67 | 15.787 | 42.894 | 13.701 | 1.00 12.78 | C |
| ATOM | 7140 | OG | SER | E | 67 | 16.204 | 42.103 | 14.794 | 1.00 12.92 | O |
| ATOM | 7141 | N | GLY | E | 68 | 13.181 | 43.204 | 14.738 | 1.00 14.63 | N |
| ATOM | 7142 | CA | GLY | E | 68 | 12.077 | 42.827 | 15.593 | 1.00 14.34 | C |
| ATOM | 7143 | C | GLY | E | 68 | 12.502 | 41.911 | 16.725 | 1.00 13.06 | C |
| ATOM | 7144 | O | GLY | E | 68 | 11.793 | 41.781 | 17.706 | 1.00 11.72 | O |
| ATOM | 7145 | N | THR | E | 69 | 13.691 | 41.326 | 16.637 | 1.00 13.39 | N |
| ATOM | 7146 | CA | THR | E | 69 | 14.068 | 40.268 | 17.602 | 1.00 14.43 | C |
| ATOM | 7147 | C | THR | E | 69 | 15.421 | 40.433 | 18.299 | 1.00 13.46 | C |
| ATOM | 7148 | O | THR | E | 69 | 15.685 | 39.754 | 19.296 | 1.00 13.85 | O |
| ATOM | 7149 | CB | THR | E | 69 | 14.054 | 38.887 | 16.933 | 1.00 14.69 | C |
| ATOM | 7150 | OG1 | THR | E | 69 | 14.925 | 38.875 | 15.811 | 1.00 17.73 | O |
| ATOM | 7151 | CG2 | THR | E | 69 | 12.699 | 38.551 | 16.438 | 1.00 17.53 | C |
| ATOM | 7152 | N | ASP | E | 70 | 16.293 | 41.276 | 17.773 | 1.00 12.91 | N |
| ATOM | 7153 | CA | ASP | E | 70 | 17.652 | 41.391 | 18.295 | 1.00 13.51 | C |
| ATOM | 7154 | C | ASP | E | 70 | 17.958 | 42.885 | 18.402 | 1.00 13.68 | C |
| ATOM | 7155 | O | ASP | E | 70 | 17.844 | 43.616 | 17.409 | 1.00 15.60 | O |
| ATOM | 7156 | CB | ASP | E | 70 | 18.642 | 40.730 | 17.351 | 1.00 13.67 | C |
| ATOM | 7157 | CG | ASP | E | 70 | 18.555 | 39.242 | 17.400 | 1.00 15.11 | C |
| ATOM | 7158 | OD1 | ASP | E | 70 | 18.837 | 38.694 | 18.457 | 1.00 20.88 | O |
| ATOM | 7159 | OD2 | ASP | E | 70 | 18.205 | 38.627 | 16.398 | 1.00 20.55 | O |
| ATOM | 7160 | N | PHE | E | 71 | 18.348 | 43.333 | 19.586 | 1.00 13.45 | N |
| ATOM | 7161 | CA | PHE | E | 71 | 18.454 | 44.774 | 19.853 | 1.00 14.24 | C |
| ATOM | 7162 | C | PHE | E | 71 | 19.764 | 45.123 | 20.551 | 1.00 15.43 | C |
| ATOM | 7163 | O | PHE | E | 71 | 20.408 | 44.265 | 21.170 | 1.00 14.88 | O |
| ATOM | 7164 | CB | PHE | E | 71 | 17.281 | 45.225 | 20.724 | 1.00 15.38 | C |
| ATOM | 7165 | CG | PHE | E | 71 | 16.003 | 44.452 | 20.495 | 1.00 15.80 | C |
| ATOM | 7166 | CD1 | PHE | E | 71 | 15.191 | 44.710 | 19.387 | 1.00 16.39 | C |
| ATOM | 7167 | CD2 | PHE | E | 71 | 15.586 | 43.520 | 21.406 | 1.00 14.84 | C |
| ATOM | 7168 | CE1 | PHE | E | 71 | 14.006 | 44.003 | 19.197 | 1.00 14.52 | C |
| ATOM | 7169 | CE2 | PHE | E | 71 | 14.434 | 42.838 | 21.231 | 1.00 15.38 | C |
| ATOM | 7170 | CZ | PHE | E | 71 | 13.628 | 43.072 | 20.130 | 1.00 15.30 | C |
| ATOM | 7171 | N | THR | E | 72 | 20.154 | 46.395 | 20.455 | 1.00 14.95 | N |
| ATOM | 7172 | CA | THR | E | 72 | 21.428 | 46.848 | 20.971 | 1.00 14.96 | C |
| ATOM | 7173 | C | THR | E | 72 | 21.349 | 48.254 | 21.529 | 1.00 15.84 | C |
| ATOM | 7174 | O | THR | E | 72 | 21.104 | 49.223 | 20.777 | 1.00 15.18 | O |
| ATOM | 7175 | CB | THR | E | 72 | 22.511 | 46.905 | 19.858 | 1.00 15.33 | C |
| ATOM | 7176 | OG1 | THR | E | 72 | 22.691 | 45.609 | 19.302 | 1.00 14.48 | O |
| ATOM | 7177 | CG2 | THR | E | 72 | 23.821 | 47.420 | 20.445 | 1.00 16.02 | C |
| ATOM | 7178 | N | LEU | E | 73 | 21.559 | 48.394 | 22.830 | 1.00 15.40 | N |
| ATOM | 7179 | CA | LEU | E | 73 | 21.687 | 49.736 | 23.410 | 1.00 16.54 | C |
| ATOM | 7180 | C | LEU | E | 73 | 23.147 | 50.191 | 23.329 | 1.00 17.16 | C |
| ATOM | 7181 | O | LEU | E | 73 | 24.045 | 49.461 | 23.717 | 1.00 17.11 | O |
| ATOM | 7182 | CB | LEU | E | 73 | 21.177 | 49.706 | 24.865 | 1.00 16.68 | C |
| ATOM | 7183 | CG | LEU | E | 73 | 21.247 | 50.984 | 25.698 | 1.00 16.73 | C |
| ATOM | 7184 | CD1 | LEU | E | 73 | 20.397 | 52.052 | 25.090 | 1.00 17.00 | C |
| ATOM | 7185 | CD2 | LEU | E | 73 | 20.816 | 50.687 | 27.110 | 1.00 17.63 | C |
| ATOM | 7186 | N | ARG | E | 74 | 23.400 | 51.407 | 22.847 | 1.00 17.86 | N |
| ATOM | 7187 | CA | ARG | E | 74 | 24.751 | 51.931 | 22.868 | 1.00 20.28 | C |
| ATOM | 7188 | C | ARG | E | 74 | 24.829 | 53.166 | 23.783 | 1.00 20.84 | C |
| ATOM | 7189 | O | ARG | E | 74 | 23.929 | 54.013 | 23.780 | 1.00 21.15 | O |
| ATOM | 7190 | CB | ARG | E | 74 | 25.276 | 52.240 | 21.455 | 1.00 19.96 | C |
| ATOM | 7191 | CG | ARG | E | 74 | 26.739 | 52.630 | 21.446 | 1.00 21.52 | C |
| ATOM | 7192 | CD | ARG | E | 74 | 27.249 | 52.840 | 20.055 | 1.00 24.71 | C |
| ATOM | 7193 | NE | ARG | E | 74 | 28.671 | 53.228 | 19.997 | 1.00 27.97 | N |
| ATOM | 7194 | CZ | ARG | E | 74 | 29.090 | 54.494 | 19.952 | 1.00 31.89 | C |
| ATOM | 7195 | NH1 | ARG | E | 74 | 28.218 | 55.506 | 19.928 | 1.00 32.00 | N |
| ATOM | 7196 | NH2 | ARG | E | 74 | 30.390 | 54.753 | 19.886 | 1.00 33.03 | N |
| ATOM | 7197 | N | ILE | E | 75 | 25.879 | 53.217 | 24.601 | 1.00 21.78 | N |
| ATOM | 7198 | CA | ILE | E | 75 | 26.205 | 54.418 | 25.388 | 1.00 23.60 | C |
| ATOM | 7199 | C | ILE | E | 75 | 27.576 | 54.860 | 24.884 | 1.00 25.20 | C |
| ATOM | 7200 | O | ILE | E | 75 | 28.537 | 54.118 | 25.012 | 1.00 26.12 | O |
| ATOM | 7201 | CB | ILE | E | 75 | 26.216 | 54.078 | 26.891 | 1.00 24.06 | C |
| ATOM | 7202 | CG1 | ILE | E | 75 | 25.021 | 53.175 | 27.224 | 1.00 22.29 | C |
| ATOM | 7203 | CG2 | ILE | E | 75 | 26.156 | 55.327 | 27.726 | 1.00 23.49 | C |

```
ATOM   7204  CD1 ILE E  75      25.007  52.649  28.664  1.00 24.35           C
ATOM   7205  N   SER E  76      27.671  56.037  24.275  1.00 27.55           N
ATOM   7206  CA  SER E  76      28.825  56.338  23.403  1.00 28.95           C
ATOM   7207  C   SER E  76      30.111  56.666  24.143  1.00 31.81           C
ATOM   7208  O   SER E  76      31.216  56.227  23.728  1.00 35.06           O
ATOM   7209  CB  SER E  76      28.499  57.473  22.448  1.00 28.89           C
ATOM   7210  OG  SER E  76      28.324  58.666  23.159  1.00 28.96           O
ATOM   7211  N   ARG E  77      29.983  57.474  25.195  1.00 31.69           N
ATOM   7212  CA  ARG E  77      31.111  57.846  26.036  1.00 32.11           C
ATOM   7213  C   ARG E  77      30.629  57.840  27.474  1.00 30.43           C
ATOM   7214  O   ARG E  77      30.111  58.829  28.005  1.00 30.17           O
ATOM   7215  CB  ARG E  77      31.658  59.217  25.670  1.00 33.48           C
ATOM   7216  CG  ARG E  77      32.287  59.268  24.295  1.00 34.92           C
ATOM   7217  CD  ARG E  77      32.288  60.697  23.772  1.00 36.44           C
ATOM   7218  NE  ARG E  77      32.792  60.771  22.398  1.00 38.62           N
ATOM   7219  CZ  ARG E  77      34.081  60.715  22.061  1.00 40.61           C
ATOM   7220  NH1 ARG E  77      35.031  60.521  22.981  1.00 42.04           N
ATOM   7221  NH2 ARG E  77      34.421  60.809  20.779  1.00 41.48           N
ATOM   7222  N   VAL E  78      30.828  56.690  28.089  1.00 28.48           N
ATOM   7223  CA  VAL E  78      30.276  56.414  29.389  1.00 27.17           C
ATOM   7224  C   VAL E  78      30.735  57.432  30.454  1.00 26.24           C
ATOM   7225  O   VAL E  78      31.889  57.879  30.464  1.00 26.03           O
ATOM   7226  CB  VAL E  78      30.609  54.964  29.767  1.00 26.96           C
ATOM   7227  CG1 VAL E  78      30.298  54.716  31.171  1.00 27.62           C
ATOM   7228  CG2 VAL E  78      29.822  53.992  28.854  1.00 27.75           C
ATOM   7229  N   GLU E  79      29.789  57.830  31.303  1.00 25.50           N
ATOM   7230  CA  GLU E  79      30.045  58.657  32.479  1.00 25.35           C
ATOM   7231  C   GLU E  79      29.716  57.833  33.718  1.00 23.57           C
ATOM   7232  O   GLU E  79      28.996  56.846  33.638  1.00 22.62           O
ATOM   7233  CB  GLU E  79      29.207  59.927  32.431  1.00 25.25           C
ATOM   7234  CG  GLU E  79      29.411  60.697  31.158  1.00 27.44           C
ATOM   7235  CD  GLU E  79      28.703  62.029  31.119  1.00 30.07           C
ATOM   7236  OE1 GLU E  79      27.733  62.240  31.888  1.00 34.51           O
ATOM   7237  OE2 GLU E  79      29.099  62.853  30.250  1.00 35.70           O
ATOM   7238  N   ALA E  80      30.261  58.223  34.863  1.00 22.78           N
ATOM   7239  CA  ALA E  80      30.148  57.406  36.082  1.00 21.80           C
ATOM   7240  C   ALA E  80      28.697  57.261  36.545  1.00 20.06           C
ATOM   7241  O   ALA E  80      28.336  56.270  37.143  1.00 18.74           O
ATOM   7242  CB  ALA E  80      30.991  57.987  37.187  1.00 22.26           C
ATOM   7243  N   GLU E  81      27.877  58.273  36.283  1.00 19.37           N
ATOM   7244  CA  GLU E  81      26.434  58.235  36.624  1.00 20.92           C
ATOM   7245  C   GLU E  81      25.624  57.202  35.838  1.00 18.64           C
ATOM   7246  O   GLU E  81      24.460  56.999  36.123  1.00 17.17           O
ATOM   7247  CB  GLU E  81      25.798  59.574  36.312  1.00 23.12           C
ATOM   7248  CG  GLU E  81      25.622  59.746  34.786  1.00 26.99           C
ATOM   7249  CD  GLU E  81      25.736  61.168  34.342  1.00 27.64           C
ATOM   7250  OE1 GLU E  81      26.825  61.768  34.550  1.00 36.96           O
ATOM   7251  OE2 GLU E  81      24.745  61.676  33.773  1.00 35.33           O
ATOM   7252  N   ASP E  82      26.208  56.620  34.804  1.00 18.11           N
ATOM   7253  CA  ASP E  82      25.509  55.634  33.983  1.00 17.55           C
ATOM   7254  C   ASP E  82      25.415  54.241  34.593  1.00 17.47           C
ATOM   7255  O   ASP E  82      24.683  53.401  34.086  1.00 15.48           O
ATOM   7256  CB  ASP E  82      26.204  55.518  32.631  1.00 19.45           C
ATOM   7257  CG  ASP E  82      26.182  56.821  31.848  1.00 19.97           C
ATOM   7258  OD1 ASP E  82      25.276  57.618  32.067  1.00 22.77           O
ATOM   7259  OD2 ASP E  82      27.041  57.017  30.976  1.00 25.46           O
ATOM   7260  N   VAL E  83      26.151  53.981  35.654  1.00 16.08           N
ATOM   7261  CA  VAL E  83      26.080  52.660  36.290  1.00 16.43           C
ATOM   7262  C   VAL E  83      24.693  52.366  36.818  1.00 14.48           C
ATOM   7263  O   VAL E  83      23.990  53.226  37.355  1.00 15.00           O
ATOM   7264  CB  VAL E  83      27.163  52.460  37.383  1.00 17.17           C
ATOM   7265  CG1 VAL E  83      28.547  52.689  36.772  1.00 18.57           C
ATOM   7266  CG2 VAL E  83      26.924  53.398  38.579  1.00 19.27           C
ATOM   7267  N   GLY E  84      24.298  51.110  36.703  1.00 14.04           N
ATOM   7268  CA  GLY E  84      22.971  50.703  37.079  1.00 15.57           C
```

```
ATOM   7269  C    GLY E   84     22.669  49.508  36.198  1.00 16.03          C
ATOM   7270  O    GLY E   84     23.538  49.066  35.440  1.00 17.68          O
ATOM   7271  N    ILE E   85     21.450  49.009  36.315  1.00 15.70          N
ATOM   7272  CA   ILE E   85     20.978  47.862  35.575  1.00 14.90          C
ATOM   7273  C    ILE E   85     20.049  48.331  34.477  1.00 16.02          C
ATOM   7274  O    ILE E   85     19.107  49.059  34.736  1.00 14.45          O
ATOM   7275  CB   ILE E   85     20.297  46.867  36.505  1.00 16.86          C
ATOM   7276  CG1  ILE E   85     21.338  46.316  37.495  1.00 17.52          C
ATOM   7277  CG2  ILE E   85     19.679  45.698  35.715  1.00 17.07          C
ATOM   7278  CD1  ILE E   85     20.725  45.809  38.700  1.00 19.86          C
ATOM   7279  N    TYR E   86     20.371  47.918  33.250  1.00 13.97          N
ATOM   7280  CA   TYR E   86     19.596  48.253  32.063  1.00 13.47          C
ATOM   7281  C    TYR E   86     18.671  47.104  31.737  1.00 15.37          C
ATOM   7282  O    TYR E   86     19.129  45.972  31.639  1.00 13.45          O
ATOM   7283  CB   TYR E   86     20.555  48.542  30.907  1.00 13.47          C
ATOM   7284  CG   TYR E   86     21.324  49.818  31.161  1.00 12.19          C
ATOM   7285  CD1  TYR E   86     22.481  49.825  31.946  1.00 13.69          C
ATOM   7286  CD2  TYR E   86     20.905  51.020  30.617  1.00 14.75          C
ATOM   7287  CE1  TYR E   86     23.174  51.003  32.194  1.00 13.24          C
ATOM   7288  CE2  TYR E   86     21.596  52.185  30.855  1.00 12.71          C
ATOM   7289  CZ   TYR E   86     22.723  52.174  31.643  1.00 12.99          C
ATOM   7290  OH   TYR E   86     23.364  53.386  31.866  1.00 14.50          O
ATOM   7291  N    TYR E   87     17.371  47.398  31.637  1.00 16.05          N
ATOM   7292  CA   TYR E   87     16.368  46.405  31.357  1.00 13.93          C
ATOM   7293  C    TYR E   87     15.727  46.632  29.985  1.00 14.32          C
ATOM   7294  O    TYR E   87     15.370  47.771  29.633  1.00 11.17          O
ATOM   7295  CB   TYR E   87     15.227  46.462  32.373  1.00 14.57          C
ATOM   7296  CG   TYR E   87     15.570  46.101  33.788  1.00 15.18          C
ATOM   7297  CD1  TYR E   87     15.544  44.773  34.214  1.00 12.96          C
ATOM   7298  CD2  TYR E   87     15.978  47.069  34.701  1.00 14.25          C
ATOM   7299  CE1  TYR E   87     15.862  44.443  35.521  1.00 13.96          C
ATOM   7300  CE2  TYR E   87     16.273  46.723  35.997  1.00 14.36          C
ATOM   7301  CZ   TYR E   87     16.202  45.394  36.397  1.00 15.94          C
ATOM   7302  OH   TYR E   87     16.538  45.071  37.701  1.00 17.95          O
ATOM   7303  N    CYS E   88     15.513  45.538  29.249  1.00 12.36          N
ATOM   7304  CA   CYS E   88     14.735  45.601  28.011  1.00 14.56          C
ATOM   7305  C    CYS E   88     13.327  45.195  28.304  1.00 14.99          C
ATOM   7306  O    CYS E   88     13.050  44.402  29.237  1.00 15.09          O
ATOM   7307  CB   CYS E   88     15.344  44.764  26.861  1.00 15.49          C
ATOM   7308  SG   CYS E   88     15.700  43.054  27.158  1.00 19.73          S
ATOM   7309  N    ALA E   89     12.391  45.767  27.546  1.00 13.62          N
ATOM   7310  CA   ALA E   89     11.016  45.289  27.638  1.00 14.25          C
ATOM   7311  C    ALA E   89     10.302  45.431  26.307  1.00 14.06          C
ATOM   7312  O    ALA E   89     10.731  46.217  25.455  1.00 13.21          O
ATOM   7313  CB   ALA E   89     10.305  46.052  28.692  1.00 14.40          C
ATOM   7314  N    HIS E   90      9.194  44.702  26.158  1.00 13.21          N
ATOM   7315  CA   HIS E   90      8.347  44.796  24.977  1.00 12.80          C
ATOM   7316  C    HIS E   90      7.008  45.435  25.349  1.00 13.03          C
ATOM   7317  O    HIS E   90      6.615  45.460  26.508  1.00 12.99          O
ATOM   7318  CB   HIS E   90      8.090  43.392  24.417  1.00 13.25          C
ATOM   7319  CG   HIS E   90      6.997  42.663  25.125  1.00 12.65          C
ATOM   7320  ND1  HIS E   90      7.233  41.638  26.023  1.00 15.94          N
ATOM   7321  CD2  HIS E   90      5.657  42.806  25.059  1.00 12.68          C
ATOM   7322  CE1  HIS E   90      6.078  41.202  26.492  1.00 13.96          C
ATOM   7323  NE2  HIS E   90      5.106  41.881  25.909  1.00 16.65          N
ATOM   7324  N    ASN E   91      6.305  45.913  24.325  1.00 13.69          N
ATOM   7325  CA   ASN E   91      4.979  46.476  24.436  1.00 13.76          C
ATOM   7326  C    ASN E   91      4.187  45.993  23.238  1.00 14.48          C
ATOM   7327  O    ASN E   91      3.715  46.755  22.424  1.00 13.90          O
ATOM   7328  CB   ASN E   91      5.089  48.025  24.506  1.00 13.05          C
ATOM   7329  CG   ASN E   91      3.746  48.737  24.590  1.00 14.64          C
ATOM   7330  OD1  ASN E   91      3.633  49.928  24.200  1.00 16.16          O
ATOM   7331  ND2  ASN E   91      2.748  48.075  25.103  1.00  6.95          N
ATOM   7332  N    VAL E   92      4.062  44.677  23.113  1.00 13.88          N
ATOM   7333  CA   VAL E   92      3.270  44.095  22.043  1.00 15.12          C
```

| ATOM | 7334 | C | VAL | E | 92 | 2.101 | 43.236 | 22.498 | 1.00 | 14.22 | C |
|------|------|-----|-----|---|-----|--------|--------|--------|------|-------|---|
| ATOM | 7335 | O | VAL | E | 92 | 1.195 | 42.977 | 21.695 | 1.00 | 14.00 | O |
| ATOM | 7336 | CB | VAL | E | 92 | 4.130 | 43.341 | 20.960 | 1.00 | 15.45 | C |
| ATOM | 7337 | CG1 | VAL | E | 92 | 5.233 | 44.227 | 20.435 | 1.00 | 16.41 | C |
| ATOM | 7338 | CG2 | VAL | E | 92 | 4.703 | 42.003 | 21.457 | 1.00 | 16.30 | C |
| ATOM | 7339 | N | GLU | E | 93 | 2.116 | 42.816 | 23.764 | 1.00 | 14.64 | N |
| ATOM | 7340 | CA | GLU | E | 93 | 1.074 | 42.007 | 24.346 | 1.00 | 14.27 | C |
| ATOM | 7341 | C | GLU | E | 93 | 1.103 | 42.071 | 25.863 | 1.00 | 13.24 | C |
| ATOM | 7342 | O | GLU | E | 93 | 2.066 | 42.581 | 26.462 | 1.00 | 13.76 | O |
| ATOM | 7343 | CB | GLU | E | 93 | 1.246 | 40.536 | 23.921 | 1.00 | 14.87 | C |
| ATOM | 7344 | CG | GLU | E | 93 | 2.570 | 39.904 | 24.343 | 1.00 | 15.44 | C |
| ATOM | 7345 | CD | GLU | E | 93 | 2.591 | 38.381 | 24.234 | 1.00 | 16.76 | C |
| ATOM | 7346 | OE1 | GLU | E | 93 | 2.111 | 37.864 | 23.209 | 1.00 | 16.41 | O |
| ATOM | 7347 | OE2 | GLU | E | 93 | 3.114 | 37.704 | 25.158 | 1.00 | 17.65 | O |
| ATOM | 7348 | N | LEU | E | 94 | 0.055 | 41.541 | 26.480 | 1.00 | 13.87 | N |
| ATOM | 7349 | CA | LEU | E | 94 | 0.065 | 41.175 | 27.877 | 1.00 | 13.42 | C |
| ATOM | 7350 | C | LEU | E | 94 | 0.321 | 39.646 | 27.910 | 1.00 | 15.39 | C |
| ATOM | 7351 | O | LEU | E | 94 | -0.158 | 38.941 | 27.028 | 1.00 | 16.36 | O |
| ATOM | 7352 | CB | LEU | E | 94 | -1.266 | 41.548 | 28.568 | 1.00 | 12.39 | C |
| ATOM | 7353 | CG | LEU | E | 94 | -1.819 | 42.967 | 28.361 | 1.00 | 15.08 | C |
| ATOM | 7354 | CD1 | LEU | E | 94 | -3.081 | 43.228 | 29.188 | 1.00 | 15.55 | C |
| ATOM | 7355 | CD2 | LEU | E | 94 | -0.752 | 43.925 | 28.773 | 1.00 | 13.57 | C |
| ATOM | 7356 | N | PRO | E | 95 | 1.125 | 39.152 | 28.881 | 1.00 | 16.28 | N |
| ATOM | 7357 | CA | PRO | E | 95 | 1.721 | 39.924 | 29.970 | 1.00 | 16.02 | C |
| ATOM | 7358 | C | PRO | E | 95 | 2.892 | 40.771 | 29.536 | 1.00 | 14.76 | C |
| ATOM | 7359 | O | PRO | E | 95 | 3.594 | 40.473 | 28.581 | 1.00 | 12.40 | O |
| ATOM | 7360 | CB | PRO | E | 95 | 2.200 | 38.847 | 30.977 | 1.00 | 16.58 | C |
| ATOM | 7361 | CG | PRO | E | 95 | 2.097 | 37.529 | 30.250 | 1.00 | 18.79 | C |
| ATOM | 7362 | CD | PRO | E | 95 | 1.580 | 37.748 | 28.896 | 1.00 | 17.79 | C |
| ATOM | 7363 | N | ARG | E | 96 | 3.072 | 41.876 | 30.231 | 1.00 | 13.77 | N |
| ATOM | 7364 | CA | ARG | E | 96 | 4.203 | 42.702 | 29.987 | 1.00 | 13.16 | C |
| ATOM | 7365 | C | ARG | E | 96 | 5.424 | 42.056 | 30.620 | 1.00 | 13.41 | C |
| ATOM | 7366 | O | ARG | E | 96 | 5.400 | 41.659 | 31.793 | 1.00 | 14.74 | O |
| ATOM | 7367 | CB | ARG | E | 96 | 3.952 | 44.097 | 30.548 | 1.00 | 11.19 | C |
| ATOM | 7368 | CG | ARG | E | 96 | 2.616 | 44.698 | 30.201 | 1.00 | 11.08 | C |
| ATOM | 7369 | CD | ARG | E | 96 | 2.676 | 46.226 | 30.468 | 1.00 | 11.28 | C |
| ATOM | 7370 | NE | ARG | E | 96 | 1.481 | 46.883 | 29.958 | 1.00 | 9.64 | N |
| ATOM | 7371 | CZ | ARG | E | 96 | 1.311 | 47.183 | 28.672 | 1.00 | 12.48 | C |
| ATOM | 7372 | NH1 | ARG | E | 96 | 2.201 | 46.839 | 27.753 | 1.00 | 14.53 | N |
| ATOM | 7373 | NH2 | ARG | E | 96 | 0.234 | 47.807 | 28.289 | 1.00 | 14.81 | N |
| ATOM | 7374 | N | THR | E | 97 | 6.471 | 41.896 | 29.831 | 1.00 | 12.70 | N |
| ATOM | 7375 | CA | THR | E | 97 | 7.674 | 41.209 | 30.302 | 1.00 | 13.09 | C |
| ATOM | 7376 | C | THR | E | 97 | 8.938 | 42.003 | 30.075 | 1.00 | 12.66 | C |
| ATOM | 7377 | O | THR | E | 97 | 9.061 | 42.813 | 29.125 | 1.00 | 13.63 | O |
| ATOM | 7378 | CB | THR | E | 97 | 7.805 | 39.749 | 29.754 | 1.00 | 13.01 | C |
| ATOM | 7379 | OG1 | THR | E | 97 | 7.861 | 39.726 | 28.323 | 1.00 | 12.54 | O |
| ATOM | 7380 | CG2 | THR | E | 97 | 6.659 | 38.944 | 30.188 | 1.00 | 14.90 | C |
| ATOM | 7381 | N | PHE | E | 98 | 9.873 | 41.792 | 30.993 | 1.00 | 11.75 | N |
| ATOM | 7382 | CA | PHE | E | 98 | 11.130 | 42.492 | 31.038 | 1.00 | 12.88 | C |
| ATOM | 7383 | C | PHE | E | 98 | 12.275 | 41.485 | 30.957 | 1.00 | 14.78 | C |
| ATOM | 7384 | O | PHE | E | 98 | 12.132 | 40.317 | 31.402 | 1.00 | 11.86 | O |
| ATOM | 7385 | CB | PHE | E | 98 | 11.224 | 43.230 | 32.370 | 1.00 | 14.24 | C |
| ATOM | 7386 | CG | PHE | E | 98 | 10.114 | 44.217 | 32.588 | 1.00 | 14.64 | C |
| ATOM | 7387 | CD1 | PHE | E | 98 | 8.904 | 43.820 | 33.089 | 1.00 | 15.08 | C |
| ATOM | 7388 | CD2 | PHE | E | 98 | 10.313 | 45.564 | 32.319 | 1.00 | 16.29 | C |
| ATOM | 7389 | CE1 | PHE | E | 98 | 7.890 | 44.739 | 33.311 | 1.00 | 15.22 | C |
| ATOM | 7390 | CE2 | PHE | E | 98 | 9.312 | 46.462 | 32.480 | 1.00 | 14.05 | C |
| ATOM | 7391 | CZ | PHE | E | 98 | 8.110 | 46.077 | 32.978 | 1.00 | 14.85 | C |
| ATOM | 7392 | N | GLY | E | 99 | 13.390 | 41.933 | 30.392 | 1.00 | 12.33 | N |
| ATOM | 7393 | CA | GLY | E | 99 | 14.650 | 41.238 | 30.547 | 1.00 | 13.65 | C |
| ATOM | 7394 | C | GLY | E | 99 | 15.107 | 41.200 | 31.984 | 1.00 | 14.69 | C |
| ATOM | 7395 | O | GLY | E | 99 | 14.572 | 41.895 | 32.874 | 1.00 | 14.83 | O |
| ATOM | 7396 | N | GLY | E | 100 | 16.120 | 40.370 | 32.242 | 1.00 | 15.10 | N |
| ATOM | 7397 | CA | GLY | E | 100 | 16.643 | 40.211 | 33.565 | 1.00 | 16.36 | C |
| ATOM | 7398 | C | GLY | E | 100 | 17.551 | 41.328 | 34.023 | 1.00 | 16.28 | C |

| ATOM | 7399 | O | GLY | E | 100 | 17.912 | 41.408 | 35.206 | 1.00 | 15.83 | O |
|------|------|------|-----|---|-----|--------|--------|--------|------|-------|---|
| ATOM | 7400 | N | GLY | E | 101 | 17.935 | 42.168 | 33.069 | 1.00 | 14.96 | N |
| ATOM | 7401 | CA | GLY | E | 101 | 18.709 | 43.362 | 33.328 | 1.00 | 17.82 | C |
| ATOM | 7402 | C | GLY | E | 101 | 20.154 | 43.015 | 33.097 | 1.00 | 18.15 | C |
| ATOM | 7403 | O | GLY | E | 101 | 20.583 | 41.890 | 33.379 | 1.00 | 17.01 | O |
| ATOM | 7404 | N | THR | E | 102 | 20.871 | 43.973 | 32.551 | 1.00 | 18.51 | N |
| ATOM | 7405 | CA | THR | E | 102 | 22.305 | 43.914 | 32.374 | 1.00 | 19.48 | C |
| ATOM | 7406 | C | THR | E | 102 | 22.936 | 44.999 | 33.217 | 1.00 | 19.06 | C |
| ATOM | 7407 | O | THR | E | 102 | 22.617 | 46.174 | 33.057 | 1.00 | 17.95 | O |
| ATOM | 7408 | CB | THR | E | 102 | 22.680 | 44.114 | 30.900 | 1.00 | 19.00 | C |
| ATOM | 7409 | OG1 | THR | E | 102 | 22.114 | 43.037 | 30.126 | 1.00 | 16.23 | O |
| ATOM | 7410 | CG2 | THR | E | 102 | 24.173 | 44.113 | 30.714 | 1.00 | 19.81 | C |
| ATOM | 7411 | N | LYS | E | 103 | 23.835 | 44.617 | 34.117 | 1.00 | 21.34 | N |
| ATOM | 7412 | CA | LYS | E | 103 | 24.601 | 45.625 | 34.875 | 1.00 | 22.24 | C |
| ATOM | 7413 | C | LYS | E | 103 | 25.739 | 46.238 | 34.068 | 1.00 | 22.54 | C |
| ATOM | 7414 | O | LYS | E | 103 | 26.563 | 45.518 | 33.521 | 1.00 | 22.61 | O |
| ATOM | 7415 | CB | LYS | E | 103 | 25.188 | 45.003 | 36.126 | 1.00 | 23.94 | C |
| ATOM | 7416 | CG | LYS | E | 103 | 25.782 | 46.015 | 37.065 | 1.00 | 24.10 | C |
| ATOM | 7417 | CD | LYS | E | 103 | 26.533 | 45.359 | 38.193 | 1.00 | 25.88 | C |
| ATOM | 7418 | CE | LYS | E | 103 | 25.617 | 44.700 | 39.215 | 1.00 | 26.87 | C |
| ATOM | 7419 | NZ | LYS | E | 103 | 26.493 | 44.017 | 40.230 | 1.00 | 29.05 | N |
| ATOM | 7420 | N | LEU | E | 104 | 25.793 | 47.571 | 34.005 | 1.00 | 21.69 | N |
| ATOM | 7421 | CA | LEU | E | 104 | 26.936 | 48.256 | 33.447 | 1.00 | 23.20 | C |
| ATOM | 7422 | C | LEU | E | 104 | 27.989 | 48.395 | 34.566 | 1.00 | 24.77 | C |
| ATOM | 7423 | O | LEU | E | 104 | 27.658 | 48.906 | 35.652 | 1.00 | 23.25 | O |
| ATOM | 7424 | CB | LEU | E | 104 | 26.518 | 49.644 | 32.931 | 1.00 | 22.03 | C |
| ATOM | 7425 | CG | LEU | E | 104 | 27.613 | 50.539 | 32.337 | 1.00 | 22.01 | C |
| ATOM | 7426 | CD1 | LEU | E | 104 | 28.060 | 50.068 | 30.981 | 1.00 | 23.94 | C |
| ATOM | 7427 | CD2 | LEU | E | 104 | 27.158 | 51.990 | 32.263 | 1.00 | 23.12 | C |
| ATOM | 7428 | N | GLU | E | 105 | 29.209 | 47.883 | 34.318 | 1.00 | 26.41 | N |
| ATOM | 7429 | CA | GLU | E | 105 | 30.391 | 48.159 | 35.173 | 1.00 | 27.66 | C |
| ATOM | 7430 | C | GLU | E | 105 | 31.369 | 49.140 | 34.508 | 1.00 | 25.76 | C |
| ATOM | 7431 | O | GLU | E | 105 | 31.601 | 49.091 | 33.314 | 1.00 | 25.38 | O |
| ATOM | 7432 | CB | GLU | E | 105 | 31.188 | 46.905 | 35.581 | 1.00 | 28.88 | C |
| ATOM | 7433 | CG | GLU | E | 105 | 32.081 | 47.241 | 36.858 | 1.00 | 30.78 | C |
| ATOM | 7434 | CD | GLU | E | 105 | 33.464 | 46.528 | 37.044 | 1.00 | 32.46 | C |
| ATOM | 7435 | OE1 | GLU | E | 105 | 33.659 | 45.354 | 36.622 | 1.00 | 37.20 | O |
| ATOM | 7436 | OE2 | GLU | E | 105 | 34.348 | 47.163 | 37.697 | 1.00 | 32.17 | O |
| ATOM | 7437 | N | ILE | E | 106 | 31.940 | 50.027 | 35.309 | 1.00 | 25.86 | N |
| ATOM | 7438 | CA | ILE | E | 106 | 32.958 | 50.957 | 34.846 | 1.00 | 26.56 | C |
| ATOM | 7439 | C | ILE | E | 106 | 34.318 | 50.429 | 35.305 | 1.00 | 26.60 | C |
| ATOM | 7440 | O | ILE | E | 106 | 34.450 | 49.955 | 36.433 | 1.00 | 25.83 | O |
| ATOM | 7441 | CB | ILE | E | 106 | 32.723 | 52.348 | 35.426 | 1.00 | 27.38 | C |
| ATOM | 7442 | CG1 | ILE | E | 106 | 31.369 | 52.889 | 34.933 | 1.00 | 29.64 | C |
| ATOM | 7443 | CG2 | ILE | E | 106 | 33.922 | 53.274 | 35.167 | 1.00 | 27.10 | C |
| ATOM | 7444 | CD1 | ILE | E | 106 | 31.408 | 54.091 | 34.083 | 1.00 | 30.70 | C |
| ATOM | 7445 | N | LYS | E | 107 | 35.304 | 50.505 | 34.417 | 1.00 | 26.50 | N |
| ATOM | 7446 | CA | LYS | E | 107 | 36.697 | 50.257 | 34.747 | 1.00 | 26.66 | C |
| ATOM | 7447 | C | LYS | E | 107 | 37.370 | 51.618 | 34.937 | 1.00 | 25.79 | C |
| ATOM | 7448 | O | LYS | E | 107 | 37.202 | 52.526 | 34.140 | 1.00 | 25.67 | O |
| ATOM | 7449 | CB | LYS | E | 107 | 37.365 | 49.452 | 33.623 | 1.00 | 28.31 | C |
| ATOM | 7450 | CG | LYS | E | 107 | 38.872 | 49.234 | 33.748 | 1.00 | 30.16 | C |
| ATOM | 7451 | CD | LYS | E | 107 | 39.468 | 48.750 | 32.396 | 1.00 | 30.41 | C |
| ATOM | 7452 | CE | LYS | E | 107 | 41.012 | 48.773 | 32.392 | 1.00 | 31.51 | C |
| ATOM | 7453 | NZ | LYS | E | 107 | 41.601 | 48.293 | 31.082 | 1.00 | 32.24 | N |
| ATOM | 7454 | N | ARG | E | 108 | 38.112 | 51.752 | 36.023 | 1.00 | 24.71 | N |
| ATOM | 7455 | CA | ARG | E | 108 | 38.784 | 52.985 | 36.369 | 1.00 | 24.24 | C |
| ATOM | 7456 | C | ARG | E | 108 | 40.124 | 52.644 | 37.002 | 1.00 | 22.83 | C |
| ATOM | 7457 | O | ARG | E | 108 | 40.491 | 51.476 | 37.137 | 1.00 | 24.28 | O |
| ATOM | 7458 | CB | ARG | E | 108 | 37.907 | 53.824 | 37.314 | 1.00 | 24.31 | C |
| ATOM | 7459 | CG | ARG | E | 108 | 37.541 | 53.135 | 38.628 | 1.00 | 23.35 | C |
| ATOM | 7460 | CD | ARG | E | 108 | 37.453 | 54.125 | 39.804 | 1.00 | 23.39 | C |
| ATOM | 7461 | NE | ARG | E | 108 | 38.783 | 54.566 | 40.261 | 1.00 | 22.20 | N |
| ATOM | 7462 | CZ | ARG | E | 108 | 39.020 | 55.671 | 40.977 | 1.00 | 21.65 | C |
| ATOM | 7463 | NH1 | ARG | E | 108 | 38.043 | 56.490 | 41.341 | 1.00 | 20.37 | N |

| ATOM | 7464 | NH2 | ARG | E | 108 | 40.269 | 55.958 | 41.338 | 1.00 | 21.44 | N |
|------|------|-----|-----|---|-----|--------|--------|--------|------|-------|---|
| ATOM | 7465 | N | ALA | E | 109 | 40.863 | 53.669 | 37.389 | 1.00 | 23.49 | N |
| ATOM | 7466 | CA | ALA | E | 109 | 42.181 | 53.477 | 37.972 | 1.00 | 22.62 | C |
| ATOM | 7467 | C | ALA | E | 109 | 42.029 | 52.781 | 39.317 | 1.00 | 22.86 | C |
| ATOM | 7468 | O | ALA | E | 109 | 41.025 | 52.979 | 40.000 | 1.00 | 21.60 | O |
| ATOM | 7469 | CB | ALA | E | 109 | 42.853 | 54.815 | 38.139 | 1.00 | 22.65 | C |
| ATOM | 7470 | N | ASP | E | 110 | 43.021 | 51.965 | 39.683 | 1.00 | 23.27 | N |
| ATOM | 7471 | CA | ASP | E | 110 | 43.084 | 51.364 | 41.017 | 1.00 | 22.94 | C |
| ATOM | 7472 | C | ASP | E | 110 | 43.120 | 52.400 | 42.108 | 1.00 | 22.24 | C |
| ATOM | 7473 | O | ASP | E | 110 | 43.616 | 53.525 | 41.913 | 1.00 | 22.55 | O |
| ATOM | 7474 | CB | ASP | E | 110 | 44.307 | 50.461 | 41.179 | 1.00 | 24.52 | C |
| ATOM | 7475 | CG | ASP | E | 110 | 44.270 | 49.295 | 40.271 | 1.00 | 25.80 | C |
| ATOM | 7476 | OD1 | ASP | E | 110 | 43.263 | 49.156 | 39.565 | 1.00 | 26.68 | O |
| ATOM | 7477 | OD2 | ASP | E | 110 | 45.249 | 48.522 | 40.260 | 1.00 | 29.20 | O |
| ATOM | 7478 | N | ALA | E | 111 | 42.540 | 52.027 | 43.249 | 1.00 | 20.18 | N |
| ATOM | 7479 | CA | ALA | E | 111 | 42.438 | 52.906 | 44.402 | 1.00 | 20.84 | C |
| ATOM | 7480 | C | ALA | E | 111 | 42.443 | 52.054 | 45.654 | 1.00 | 20.01 | C |
| ATOM | 7481 | O | ALA | E | 111 | 41.685 | 51.089 | 45.767 | 1.00 | 18.81 | O |
| ATOM | 7482 | CB | ALA | E | 111 | 41.190 | 53.722 | 44.339 | 1.00 | 21.98 | C |
| ATOM | 7483 | N | ALA | E | 112 | 43.340 | 52.383 | 46.575 | 1.00 | 19.93 | N |
| ATOM | 7484 | CA | ALA | E | 112 | 43.446 | 51.644 | 47.823 | 1.00 | 19.43 | C |
| ATOM | 7485 | C | ALA | E | 112 | 42.283 | 52.039 | 48.702 | 1.00 | 18.72 | C |
| ATOM | 7486 | O | ALA | E | 112 | 41.824 | 53.194 | 48.653 | 1.00 | 16.42 | O |
| ATOM | 7487 | CB | ALA | E | 112 | 44.779 | 51.963 | 48.522 | 1.00 | 20.29 | C |
| ATOM | 7488 | N | PRO | E | 113 | 41.794 | 51.102 | 49.524 | 1.00 | 17.59 | N |
| ATOM | 7489 | CA | PRO | E | 113 | 40.725 | 51.494 | 50.446 | 1.00 | 18.66 | C |
| ATOM | 7490 | C | PRO | E | 113 | 41.204 | 52.419 | 51.557 | 1.00 | 18.48 | C |
| ATOM | 7491 | O | PRO | E | 113 | 42.366 | 52.315 | 52.027 | 1.00 | 18.03 | O |
| ATOM | 7492 | CB | PRO | E | 113 | 40.300 | 50.149 | 51.068 | 1.00 | 19.00 | C |
| ATOM | 7493 | CG | PRO | E | 113 | 41.532 | 49.344 | 51.020 | 1.00 | 18.35 | C |
| ATOM | 7494 | CD | PRO | E | 113 | 42.116 | 49.669 | 49.666 | 1.00 | 18.85 | C |
| ATOM | 7495 | N | THR | E | 114 | 40.309 | 53.313 | 51.967 | 1.00 | 17.85 | N |
| ATOM | 7496 | CA | THR | E | 114 | 40.432 | 54.033 | 53.201 | 1.00 | 17.63 | C |
| ATOM | 7497 | C | THR | E | 114 | 39.681 | 53.331 | 54.302 | 1.00 | 17.75 | C |
| ATOM | 7498 | O | THR | E | 114 | 38.452 | 53.276 | 54.267 | 1.00 | 17.43 | O |
| ATOM | 7499 | CB | THR | E | 114 | 39.805 | 55.393 | 53.073 | 1.00 | 18.92 | C |
| ATOM | 7500 | OG1 | THR | E | 114 | 40.436 | 56.071 | 51.989 | 1.00 | 19.20 | O |
| ATOM | 7501 | CG2 | THR | E | 114 | 39.951 | 56.159 | 54.344 | 1.00 | 21.22 | C |
| ATOM | 7502 | N | VAL | E | 115 | 40.426 | 52.870 | 55.299 | 1.00 | 17.18 | N |
| ATOM | 7503 | CA | VAL | E | 115 | 39.914 | 52.026 | 56.354 | 1.00 | 17.40 | C |
| ATOM | 7504 | C | VAL | E | 115 | 39.753 | 52.776 | 57.656 | 1.00 | 17.56 | C |
| ATOM | 7505 | O | VAL | E | 115 | 40.646 | 53.510 | 58.082 | 1.00 | 17.61 | O |
| ATOM | 7506 | CB | VAL | E | 115 | 40.872 | 50.840 | 56.531 | 1.00 | 18.73 | C |
| ATOM | 7507 | CG1 | VAL | E | 115 | 40.363 | 49.824 | 57.563 | 1.00 | 19.78 | C |
| ATOM | 7508 | CG2 | VAL | E | 115 | 41.103 | 50.197 | 55.187 | 1.00 | 19.32 | C |
| ATOM | 7509 | N | SER | E | 116 | 38.586 | 52.577 | 58.268 | 1.00 | 16.75 | N |
| ATOM | 7510 | CA | SER | E | 116 | 38.185 | 53.196 | 59.500 | 1.00 | 16.33 | C |
| ATOM | 7511 | C | SER | E | 116 | 37.620 | 52.106 | 60.378 | 1.00 | 15.87 | C |
| ATOM | 7512 | O | SER | E | 116 | 36.766 | 51.358 | 59.925 | 1.00 | 14.13 | O |
| ATOM | 7513 | CB | SER | E | 116 | 37.072 | 54.183 | 59.230 | 1.00 | 16.71 | C |
| ATOM | 7514 | OG | SER | E | 116 | 37.557 | 55.242 | 58.427 | 1.00 | 20.96 | O |
| ATOM | 7515 | N | ILE | E | 117 | 38.036 | 52.087 | 61.636 | 1.00 | 15.45 | N |
| ATOM | 7516 | CA | ILE | E | 117 | 37.486 | 51.172 | 62.632 | 1.00 | 15.69 | C |
| ATOM | 7517 | C | ILE | E | 117 | 36.795 | 51.934 | 63.760 | 1.00 | 16.11 | C |
| ATOM | 7518 | O | ILE | E | 117 | 37.234 | 53.020 | 64.150 | 1.00 | 15.10 | O |
| ATOM | 7519 | CB | ILE | E | 117 | 38.571 | 50.258 | 63.202 | 1.00 | 16.45 | C |
| ATOM | 7520 | CG1 | ILE | E | 117 | 37.961 | 49.066 | 63.942 | 1.00 | 15.52 | C |
| ATOM | 7521 | CG2 | ILE | E | 117 | 39.520 | 51.003 | 64.126 | 1.00 | 16.03 | C |
| ATOM | 7522 | CD1 | ILE | E | 117 | 39.033 | 48.075 | 64.341 | 1.00 | 15.00 | C |
| ATOM | 7523 | N | PHE | E | 118 | 35.737 | 51.331 | 64.292 | 1.00 | 14.52 | N |
| ATOM | 7524 | CA | PHE | E | 118 | 34.856 | 51.957 | 65.266 | 1.00 | 14.84 | C |
| ATOM | 7525 | C | PHE | E | 118 | 34.539 | 50.977 | 66.365 | 1.00 | 14.98 | C |
| ATOM | 7526 | O | PHE | E | 118 | 34.013 | 49.934 | 66.106 | 1.00 | 18.00 | O |
| ATOM | 7527 | CB | PHE | E | 118 | 33.550 | 52.417 | 64.620 | 1.00 | 15.13 | C |
| ATOM | 7528 | CG | PHE | E | 118 | 33.744 | 53.450 | 63.552 | 1.00 | 14.77 | C |

```
ATOM   7529  CD1 PHE E 118      33.874  53.084  62.225  1.00 15.79           C
ATOM   7530  CD2 PHE E 118      33.786  54.786  63.890  1.00 14.44           C
ATOM   7531  CE1 PHE E 118      34.085  54.019  61.248  1.00 16.92           C
ATOM   7532  CE2 PHE E 118      33.984  55.733  62.912  1.00 13.96           C
ATOM   7533  CZ  PHE E 118      34.122  55.376  61.613  1.00 14.82           C
ATOM   7534  N   PRO E 119      34.835  51.339  67.612  1.00 16.48           N
ATOM   7535  CA  PRO E 119      34.498  50.523  68.765  1.00 15.20           C
ATOM   7536  C   PRO E 119      33.004  50.646  69.096  1.00 13.72           C
ATOM   7537  O   PRO E 119      32.276  51.459  68.479  1.00 11.80           O
ATOM   7538  CB  PRO E 119      35.409  51.100  69.850  1.00 16.43           C
ATOM   7539  CG  PRO E 119      35.416  52.537  69.522  1.00 17.92           C
ATOM   7540  CD  PRO E 119      35.487  52.598  68.034  1.00 18.01           C
ATOM   7541  N   PRO E 120      32.502  49.757  69.970  1.00 14.00           N
ATOM   7542  CA  PRO E 120      31.087  49.778  70.341  1.00 13.27           C
ATOM   7543  C   PRO E 120      30.678  51.049  71.002  1.00 13.99           C
ATOM   7544  O   PRO E 120      31.458  51.625  71.739  1.00 15.44           O
ATOM   7545  CB  PRO E 120      30.915  48.579  71.301  1.00 12.59           C
ATOM   7546  CG  PRO E 120      32.113  47.803  71.149  1.00 14.14           C
ATOM   7547  CD  PRO E 120      33.207  48.615  70.569  1.00 13.45           C
ATOM   7548  N   SER E 121      29.452  51.459  70.713  1.00 13.49           N
ATOM   7549  CA  SER E 121      28.861  52.623  71.345  1.00 13.58           C
ATOM   7550  C   SER E 121      28.504  52.327  72.803  1.00 13.80           C
ATOM   7551  O   SER E 121      28.255  51.191  73.188  1.00 12.36           O
ATOM   7552  CB  SER E 121      27.620  53.055  70.589  1.00 13.97           C
ATOM   7553  OG  SER E 121      26.613  52.042  70.570  1.00 13.89           O
ATOM   7554  N   SER E 122      28.468  53.371  73.614  1.00 13.40           N
ATOM   7555  CA  SER E 122      27.993  53.258  74.991  1.00 13.86           C
ATOM   7556  C   SER E 122      26.546  52.763  75.058  1.00 14.46           C
ATOM   7557  O   SER E 122      26.190  51.999  75.983  1.00 15.68           O
ATOM   7558  CB  SER E 122      28.166  54.614  75.711  1.00 15.36           C
ATOM   7559  OG  SER E 122      27.518  55.605  74.968  1.00 18.27           O
ATOM   7560  N   GLU E 123      25.730  53.131  74.061  1.00 13.35           N
ATOM   7561  CA  GLU E 123      24.335  52.679  73.968  1.00 13.47           C
ATOM   7562  C   GLU E 123      24.316  51.158  73.774  1.00 12.32           C
ATOM   7563  O   GLU E 123      23.577  50.465  74.439  1.00 12.63           O
ATOM   7564  CB  GLU E 123      23.607  53.290  72.763  1.00 15.49           C
ATOM   7565  CG  GLU E 123      23.360  54.777  72.858  1.00 16.06           C
ATOM   7566  CD  GLU E 123      24.480  55.637  72.259  1.00 19.25           C
ATOM   7567  OE1 GLU E 123      25.669  55.245  72.283  1.00 17.03           O
ATOM   7568  OE2 GLU E 123      24.145  56.732  71.772  1.00 16.10           O
ATOM   7569  N   GLN E 124      25.131  50.666  72.859  1.00 11.79           N
ATOM   7570  CA  GLN E 124      25.156  49.202  72.622  1.00 11.03           C
ATOM   7571  C   GLN E 124      25.688  48.466  73.822  1.00 11.10           C
ATOM   7572  O   GLN E 124      25.181  47.371  74.160  1.00  9.04           O
ATOM   7573  CB  GLN E 124      25.973  48.853  71.379  1.00 12.63           C
ATOM   7574  CG  GLN E 124      25.747  47.406  70.984  1.00  9.66           C
ATOM   7575  CD  GLN E 124      26.502  47.016  69.777  1.00 11.94           C
ATOM   7576  OE1 GLN E 124      27.447  47.698  69.351  1.00 12.02           O
ATOM   7577  NE2 GLN E 124      26.078  45.894  69.181  1.00 11.30           N
ATOM   7578  N   LEU E 125      26.722  49.008  74.455  1.00  9.71           N
ATOM   7579  CA  LEU E 125      27.294  48.347  75.641  1.00 11.41           C
ATOM   7580  C   LEU E 125      26.271  48.290  76.774  1.00 11.79           C
ATOM   7581  O   LEU E 125      26.159  47.301  77.454  1.00 10.62           O
ATOM   7582  CB  LEU E 125      28.598  48.997  76.098  1.00 11.73           C
ATOM   7583  CG  LEU E 125      29.813  48.772  75.177  1.00 14.89           C
ATOM   7584  CD1 LEU E 125      30.930  49.717  75.555  1.00 14.16           C
ATOM   7585  CD2 LEU E 125      30.297  47.335  75.247  1.00 15.23           C
ATOM   7586  N   THR E 126      25.525  49.372  77.005  1.00  9.22           N
ATOM   7587  CA  THR E 126      24.436  49.322  77.975  1.00  9.70           C
ATOM   7588  C   THR E 126      23.389  48.215  77.750  1.00 10.68           C
ATOM   7589  O   THR E 126      22.855  47.660  78.716  1.00 11.65           O
ATOM   7590  CB  THR E 126      23.753  50.689  78.064  1.00 11.15           C
ATOM   7591  OG1 THR E 126      24.697  51.564  78.688  1.00 11.60           O
ATOM   7592  CG2 THR E 126      22.508  50.641  78.904  1.00  9.48           C
ATOM   7593  N   SER E 127      23.143  47.890  76.480  1.00 10.38           N
```

```
ATOM   7594  CA   SER E 127      22.138  46.928  76.099  1.00   8.96           C
ATOM   7595  C    SER E 127      22.690  45.508  76.206  1.00   9.67           C
ATOM   7596  O    SER E 127      21.932  44.574  75.967  1.00  12.65           O
ATOM   7597  CB   SER E 127      21.656  47.121  74.666  1.00   8.28           C
ATOM   7598  OG   SER E 127      22.619  46.777  73.666  1.00  11.16           O
ATOM   7599  N    GLY E 128      23.994  45.377  76.462  1.00  10.97           N
ATOM   7600  CA   GLY E 128      24.677  44.066  76.542  1.00   9.80           C
ATOM   7601  C    GLY E 128      25.294  43.514  75.267  1.00  10.88           C
ATOM   7602  O    GLY E 128      25.636  42.307  75.179  1.00   9.32           O
ATOM   7603  N    GLY E 129      25.457  44.362  74.263  1.00  11.61           N
ATOM   7604  CA   GLY E 129      26.093  43.922  73.015  1.00  11.39           C
ATOM   7605  C    GLY E 129      27.363  44.680  72.728  1.00  12.40           C
ATOM   7606  O    GLY E 129      27.677  45.676  73.394  1.00   9.81           O
ATOM   7607  N    ALA E 130      28.138  44.198  71.757  1.00  11.79           N
ATOM   7608  CA   ALA E 130      29.320  44.902  71.366  1.00  12.51           C
ATOM   7609  C    ALA E 130      29.686  44.576  69.936  1.00  13.12           C
ATOM   7610  O    ALA E 130      30.123  43.472  69.624  1.00  11.98           O
ATOM   7611  CB   ALA E 130      30.467  44.612  72.271  1.00  12.03           C
ATOM   7612  N    SER E 131      29.558  45.588  69.074  1.00  13.08           N
ATOM   7613  CA   SER E 131      29.841  45.445  67.671  1.00  12.45           C
ATOM   7614  C    SER E 131      30.999  46.382  67.379  1.00  13.13           C
ATOM   7615  O    SER E 131      30.951  47.564  67.761  1.00  14.78           O
ATOM   7616  CB   SER E 131      28.612  45.819  66.813  1.00  12.45           C
ATOM   7617  OG   SER E 131      27.544  44.900  66.875  1.00  13.95           O
ATOM   7618  N    VAL E 132      32.024  45.851  66.711  1.00  13.80           N
ATOM   7619  CA   VAL E 132      33.130  46.604  66.175  1.00  12.96           C
ATOM   7620  C    VAL E 132      32.958  46.621  64.657  1.00  12.68           C
ATOM   7621  O    VAL E 132      32.767  45.598  64.035  1.00  10.11           O
ATOM   7622  CB   VAL E 132      34.480  45.932  66.537  1.00  14.34           C
ATOM   7623  CG1  VAL E 132      35.634  46.875  66.238  1.00  14.22           C
ATOM   7624  CG2  VAL E 132      34.486  45.483  68.008  1.00  13.34           C
ATOM   7625  N    VAL E 133      33.010  47.806  64.064  1.00  11.78           N
ATOM   7626  CA   VAL E 133      32.748  47.993  62.661  1.00  12.97           C
ATOM   7627  C    VAL E 133      34.022  48.535  61.993  1.00  13.75           C
ATOM   7628  O    VAL E 133      34.723  49.401  62.533  1.00  11.26           O
ATOM   7629  CB   VAL E 133      31.576  49.027  62.434  1.00  13.08           C
ATOM   7630  CG1  VAL E 133      31.213  49.184  60.931  1.00  14.05           C
ATOM   7631  CG2  VAL E 133      30.353  48.638  63.185  1.00  14.25           C
ATOM   7632  N    CYS E 134      34.304  47.982  60.828  1.00  14.34           N
ATOM   7633  CA   CYS E 134      35.356  48.418  59.945  1.00  15.44           C
ATOM   7634  C    CYS E 134      34.751  48.757  58.578  1.00  14.09           C
ATOM   7635  O    CYS E 134      34.134  47.929  57.958  1.00  12.14           O
ATOM   7636  CB   CYS E 134      36.345  47.265  59.822  1.00  18.78           C
ATOM   7637  SG   CYS E 134      37.825  47.673  59.023  1.00  22.27           S
ATOM   7638  N    PHE E 135      34.890  50.013  58.147  1.00  12.37           N
ATOM   7639  CA   PHE E 135      34.633  50.409  56.787  1.00  14.37           C
ATOM   7640  C    PHE E 135      35.904  50.352  55.923  1.00  13.97           C
ATOM   7641  O    PHE E 135      36.968  50.834  56.320  1.00  15.50           O
ATOM   7642  CB   PHE E 135      34.096  51.859  56.743  1.00  14.52           C
ATOM   7643  CG   PHE E 135      32.763  52.053  57.421  1.00  15.86           C
ATOM   7644  CD1  PHE E 135      31.735  51.125  57.282  1.00  16.97           C
ATOM   7645  CD2  PHE E 135      32.500  53.206  58.148  1.00  15.30           C
ATOM   7646  CE1  PHE E 135      30.490  51.327  57.887  1.00  17.10           C
ATOM   7647  CE2  PHE E 135      31.270  53.388  58.778  1.00  17.35           C
ATOM   7648  CZ   PHE E 135      30.256  52.461  58.633  1.00  15.67           C
ATOM   7649  N    LEU E 136      35.779  49.769  54.744  1.00  13.36           N
ATOM   7650  CA   LEU E 136      36.830  49.747  53.744  1.00  14.49           C
ATOM   7651  C    LEU E 136      36.257  50.521  52.580  1.00  14.88           C
ATOM   7652  O    LEU E 136      35.478  49.993  51.788  1.00  13.26           O
ATOM   7653  CB   LEU E 136      37.168  48.293  53.353  1.00  15.07           C
ATOM   7654  CG   LEU E 136      38.084  47.404  54.226  1.00  17.57           C
ATOM   7655  CD1  LEU E 136      37.676  47.316  55.633  1.00  19.26           C
ATOM   7656  CD2  LEU E 136      38.176  45.989  53.677  1.00  19.05           C
ATOM   7657  N    ASN E 137      36.571  51.812  52.498  1.00  14.04           N
ATOM   7658  CA   ASN E 137      35.859  52.689  51.565  1.00  15.34           C
```

| ATOM | 7659 | C | ASN | E | 137 | 36.630 | 53.119 | 50.332 | 1.00 | 15.64 | C |
|------|------|------|------|---|-----|--------|--------|--------|------|-------|---|
| ATOM | 7660 | O | ASN | E | 137 | 37.831 | 53.382 | 50.389 | 1.00 | 15.12 | O |
| ATOM | 7661 | CB | ASN | E | 137 | 35.415 | 53.971 | 52.266 | 1.00 | 15.46 | C |
| ATOM | 7662 | CG | ASN | E | 137 | 34.204 | 53.769 | 53.155 | 1.00 | 15.98 | C |
| ATOM | 7663 | OD1 | ASN | E | 137 | 33.562 | 52.696 | 53.154 | 1.00 | 16.68 | O |
| ATOM | 7664 | ND2 | ASN | E | 137 | 33.868 | 54.795 | 53.902 | 1.00 | 15.17 | N |
| ATOM | 7665 | N | ASN | E | 138 | 35.865 | 53.252 | 49.248 | 1.00 | 16.43 | N |
| ATOM | 7666 | CA | ASN | E | 138 | 36.274 | 53.854 | 48.002 | 1.00 | 16.84 | C |
| ATOM | 7667 | C | ASN | E | 138 | 37.512 | 53.234 | 47.354 | 1.00 | 17.47 | C |
| ATOM | 7668 | O | ASN | E | 138 | 38.461 | 53.919 | 46.996 | 1.00 | 16.81 | O |
| ATOM | 7669 | CB | ASN | E | 138 | 36.415 | 55.385 | 48.166 | 1.00 | 17.48 | C |
| ATOM | 7670 | CG | ASN | E | 138 | 35.155 | 56.057 | 48.659 | 1.00 | 18.71 | C |
| ATOM | 7671 | OD1 | ASN | E | 138 | 34.946 | 56.229 | 49.858 | 1.00 | 17.57 | O |
| ATOM | 7672 | ND2 | ASN | E | 138 | 34.339 | 56.509 | 47.732 | 1.00 | 19.76 | N |
| ATOM | 7673 | N | PHE | E | 139 | 37.485 | 51.916 | 47.167 | 1.00 | 16.41 | N |
| ATOM | 7674 | CA | PHE | E | 139 | 38.571 | 51.248 | 46.509 | 1.00 | 16.91 | C |
| ATOM | 7675 | C | PHE | E | 139 | 38.203 | 50.724 | 45.125 | 1.00 | 17.39 | C |
| ATOM | 7676 | O | PHE | E | 139 | 37.050 | 50.738 | 44.741 | 1.00 | 15.60 | O |
| ATOM | 7677 | CB | PHE | E | 139 | 39.029 | 50.070 | 47.364 | 1.00 | 16.16 | C |
| ATOM | 7678 | CG | PHE | E | 139 | 37.930 | 49.121 | 47.756 | 1.00 | 14.87 | C |
| ATOM | 7679 | CD1 | PHE | E | 139 | 37.661 | 48.019 | 46.965 | 1.00 | 16.65 | C |
| ATOM | 7680 | CD2 | PHE | E | 139 | 37.248 | 49.253 | 48.968 | 1.00 | 16.74 | C |
| ATOM | 7681 | CE1 | PHE | E | 139 | 36.679 | 47.104 | 47.324 | 1.00 | 16.39 | C |
| ATOM | 7682 | CE2 | PHE | E | 139 | 36.273 | 48.308 | 49.351 | 1.00 | 16.20 | C |
| ATOM | 7683 | CZ | PHE | E | 139 | 35.992 | 47.246 | 48.529 | 1.00 | 15.14 | C |
| ATOM | 7684 | N | TYR | E | 140 | 39.213 | 50.268 | 44.394 | 1.00 | 18.96 | N |
| ATOM | 7685 | CA | TYR | E | 140 | 39.027 | 49.722 | 43.059 | 1.00 | 19.29 | C |
| ATOM | 7686 | C | TYR | E | 140 | 40.255 | 48.899 | 42.720 | 1.00 | 19.40 | C |
| ATOM | 7687 | O | TYR | E | 140 | 41.377 | 49.355 | 42.929 | 1.00 | 19.30 | O |
| ATOM | 7688 | CB | TYR | E | 140 | 38.782 | 50.812 | 41.977 | 1.00 | 20.20 | C |
| ATOM | 7689 | CG | TYR | E | 140 | 38.413 | 50.157 | 40.671 | 1.00 | 20.41 | C |
| ATOM | 7690 | CD1 | TYR | E | 140 | 39.406 | 49.639 | 39.814 | 1.00 | 21.22 | C |
| ATOM | 7691 | CD2 | TYR | E | 140 | 37.082 | 49.969 | 40.316 | 1.00 | 19.71 | C |
| ATOM | 7692 | CE1 | TYR | E | 140 | 39.064 | 48.955 | 38.655 | 1.00 | 20.45 | C |
| ATOM | 7693 | CE2 | TYR | E | 140 | 36.741 | 49.300 | 39.159 | 1.00 | 21.14 | C |
| ATOM | 7694 | CZ | TYR | E | 140 | 37.733 | 48.797 | 38.328 | 1.00 | 21.10 | C |
| ATOM | 7695 | OH | TYR | E | 140 | 37.365 | 48.120 | 37.176 | 1.00 | 21.23 | O |
| ATOM | 7696 | N | PRO | E | 141 | 40.069 | 47.686 | 42.171 | 1.00 | 20.50 | N |
| ATOM | 7697 | CA | PRO | E | 141 | 38.882 | 46.947 | 41.728 | 1.00 | 21.24 | C |
| ATOM | 7698 | C | PRO | E | 141 | 38.045 | 46.428 | 42.894 | 1.00 | 21.54 | C |
| ATOM | 7699 | O | PRO | E | 141 | 38.416 | 46.644 | 44.041 | 1.00 | 20.96 | O |
| ATOM | 7700 | CB | PRO | E | 141 | 39.492 | 45.812 | 40.916 | 1.00 | 21.18 | C |
| ATOM | 7701 | CG | PRO | E | 141 | 40.782 | 45.585 | 41.530 | 1.00 | 22.00 | C |
| ATOM | 7702 | CD | PRO | E | 141 | 41.296 | 46.917 | 41.925 | 1.00 | 21.37 | C |
| ATOM | 7703 | N | LYS | E | 142 | 36.941 | 45.737 | 42.612 | 1.00 | 22.66 | N |
| ATOM | 7704 | CA | LYS | E | 142 | 35.967 | 45.362 | 43.655 | 1.00 | 23.78 | C |
| ATOM | 7705 | C | LYS | E | 142 | 36.476 | 44.301 | 44.642 | 1.00 | 24.05 | C |
| ATOM | 7706 | O | LYS | E | 142 | 36.004 | 44.233 | 45.770 | 1.00 | 23.82 | O |
| ATOM | 7707 | CB | LYS | E | 142 | 34.638 | 44.885 | 43.028 | 1.00 | 24.84 | C |
| ATOM | 7708 | CG | LYS | E | 142 | 33.600 | 44.294 | 44.041 | 1.00 | 26.29 | C |
| ATOM | 7709 | CD | LYS | E | 142 | 32.108 | 44.213 | 43.497 | 1.00 | 27.56 | C |
| ATOM | 7710 | CE | LYS | E | 142 | 31.029 | 44.375 | 44.613 | 1.00 | 28.62 | C |
| ATOM | 7711 | NZ | LYS | E | 142 | 29.701 | 44.944 | 44.140 | 1.00 | 27.88 | N |
| ATOM | 7712 | N | ASP | E | 143 | 37.424 | 43.475 | 44.224 | 1.00 | 24.70 | N |
| ATOM | 7713 | CA | ASP | E | 143 | 37.853 | 42.335 | 45.061 | 1.00 | 24.61 | C |
| ATOM | 7714 | C | ASP | E | 143 | 38.626 | 42.853 | 46.255 | 1.00 | 23.64 | C |
| ATOM | 7715 | O | ASP | E | 143 | 39.544 | 43.668 | 46.109 | 1.00 | 22.54 | O |
| ATOM | 7716 | CB | ASP | E | 143 | 38.724 | 41.370 | 44.286 | 1.00 | 27.07 | C |
| ATOM | 7717 | CG | ASP | E | 143 | 37.965 | 40.675 | 43.179 | 1.00 | 31.81 | C |
| ATOM | 7718 | OD1 | ASP | E | 143 | 38.056 | 41.138 | 42.012 | 1.00 | 36.09 | O |
| ATOM | 7719 | OD2 | ASP | E | 143 | 37.256 | 39.689 | 43.486 | 1.00 | 37.21 | O |
| ATOM | 7720 | N | ILE | E | 144 | 38.228 | 42.398 | 47.432 | 1.00 | 21.92 | N |
| ATOM | 7721 | CA | ILE | E | 144 | 38.886 | 42.788 | 48.667 | 1.00 | 22.76 | C |
| ATOM | 7722 | C | ILE | E | 144 | 38.669 | 41.715 | 49.699 | 1.00 | 22.89 | C |
| ATOM | 7723 | O | ILE | E | 144 | 37.694 | 40.971 | 49.622 | 1.00 | 23.35 | O |

```
ATOM   7724  CB   ILE E 144       38.379  44.172  49.185  1.00 21.68           C
ATOM   7725  CG1  ILE E 144       39.403  44.798  50.137  1.00 20.52           C
ATOM   7726  CG2  ILE E 144       37.054  44.018  49.893  1.00 24.32           C
ATOM   7727  CD1  ILE E 144       39.293  46.292  50.248  1.00 20.45           C
ATOM   7728  N    ASN E 145       39.613  41.598  50.628  1.00 24.27           N
ATOM   7729  CA   ASN E 145       39.469  40.689  51.759  1.00 25.21           C
ATOM   7730  C    ASN E 145       39.685  41.417  53.057  1.00 24.27           C
ATOM   7731  O    ASN E 145       40.515  42.329  53.138  1.00 23.70           O
ATOM   7732  CB   ASN E 145       40.443  39.518  51.664  1.00 27.92           C
ATOM   7733  CG   ASN E 145       39.903  38.380  50.806  1.00 32.58           C
ATOM   7734  OD1  ASN E 145       38.926  37.696  51.175  1.00 38.37           O
ATOM   7735  ND2  ASN E 145       40.532  38.171  49.646  1.00 35.44           N
ATOM   7736  N    VAL E 146       38.924  41.016  54.071  1.00 23.80           N
ATOM   7737  CA   VAL E 146       39.059  41.574  55.398  1.00 23.00           C
ATOM   7738  C    VAL E 146       39.335  40.456  56.388  1.00 23.38           C
ATOM   7739  O    VAL E 146       38.760  39.360  56.270  1.00 21.96           O
ATOM   7740  CB   VAL E 146       37.814  42.399  55.791  1.00 23.98           C
ATOM   7741  CG1  VAL E 146       36.588  41.511  55.976  1.00 23.22           C
ATOM   7742  CG2  VAL E 146       38.076  43.192  57.062  1.00 23.97           C
ATOM   7743  N    LYS E 147       40.261  40.708  57.313  1.00 23.14           N
ATOM   7744  CA   LYS E 147       40.562  39.770  58.385  1.00 22.86           C
ATOM   7745  C    LYS E 147       40.417  40.468  59.725  1.00 21.29           C
ATOM   7746  O    LYS E 147       40.930  41.584  59.897  1.00 22.10           O
ATOM   7747  CB   LYS E 147       41.993  39.223  58.245  1.00 23.87           C
ATOM   7748  CG   LYS E 147       42.294  38.056  59.175  1.00 26.42           C
ATOM   7749  CD   LYS E 147       43.732  37.528  59.014  1.00 26.84           C
ATOM   7750  CE   LYS E 147       43.976  36.993  57.602  1.00 29.21           C
ATOM   7751  NZ   LYS E 147       45.051  35.941  57.562  1.00 30.96           N
ATOM   7752  N    TRP E 148       39.730  39.807  60.667  1.00 20.52           N
ATOM   7753  CA   TRP E 148       39.596  40.284  62.053  1.00 19.88           C
ATOM   7754  C    TRP E 148       40.506  39.517  62.977  1.00 18.55           C
ATOM   7755  O    TRP E 148       40.642  38.323  62.850  1.00 19.31           O
ATOM   7756  CB   TRP E 148       38.149  40.161  62.590  1.00 18.17           C
ATOM   7757  CG   TRP E 148       37.213  41.216  62.086  1.00 16.85           C
ATOM   7758  CD1  TRP E 148       36.359  41.119  61.026  1.00 17.51           C
ATOM   7759  CD2  TRP E 148       37.051  42.543  62.612  1.00 16.70           C
ATOM   7760  NE1  TRP E 148       35.664  42.305  60.870  1.00 18.16           N
ATOM   7761  CE2  TRP E 148       36.064  43.186  61.835  1.00 17.22           C
ATOM   7762  CE3  TRP E 148       37.603  43.226  63.681  1.00 15.73           C
ATOM   7763  CZ2  TRP E 148       35.645  44.492  62.086  1.00 16.94           C
ATOM   7764  CZ3  TRP E 148       37.199  44.554  63.920  1.00 16.93           C
ATOM   7765  CH2  TRP E 148       36.227  45.155  63.127  1.00 17.00           C
ATOM   7766  N    LYS E 149       41.109  40.217  63.917  1.00 18.82           N
ATOM   7767  CA   LYS E 149       41.852  39.569  64.981  1.00 20.54           C
ATOM   7768  C    LYS E 149       41.372  40.097  66.308  1.00 20.42           C
ATOM   7769  O    LYS E 149       41.095  41.289  66.450  1.00 19.43           O
ATOM   7770  CB   LYS E 149       43.354  39.789  64.823  1.00 20.54           C
ATOM   7771  CG   LYS E 149       43.860  39.232  63.537  1.00 21.22           C
ATOM   7772  CD   LYS E 149       45.306  39.487  63.294  1.00 22.93           C
ATOM   7773  CE   LYS E 149       45.696  38.740  62.029  1.00 25.94           C
ATOM   7774  NZ   LYS E 149       47.073  39.021  61.597  1.00 29.13           N
ATOM   7775  N    ILE E 150       41.252  39.192  67.271  1.00 18.93           N
ATOM   7776  CA   ILE E 150       40.997  39.585  68.639  1.00 19.79           C
ATOM   7777  C    ILE E 150       42.230  39.176  69.459  1.00 19.29           C
ATOM   7778  O    ILE E 150       42.565  38.001  69.511  1.00 20.40           O
ATOM   7779  CB   ILE E 150       39.731  38.914  69.152  1.00 18.88           C
ATOM   7780  CG1  ILE E 150       38.510  39.392  68.335  1.00 20.05           C
ATOM   7781  CG2  ILE E 150       39.590  39.143  70.622  1.00 19.62           C
ATOM   7782  CD1  ILE E 150       37.159  38.825  68.812  1.00 21.00           C
ATOM   7783  N    ASP E 151       42.904  40.144  70.074  1.00 19.66           N
ATOM   7784  CA   ASP E 151       44.133  39.852  70.809  1.00 20.96           C
ATOM   7785  C    ASP E 151       45.124  39.100  69.918  1.00 21.44           C
ATOM   7786  O    ASP E 151       45.777  38.145  70.363  1.00 19.39           O
ATOM   7787  CB   ASP E 151       43.838  39.069  72.112  1.00 21.99           C
ATOM   7788  CG   ASP E 151       43.101  39.908  73.147  1.00 23.95           C
```

| ATOM | 7789 | OD1 | ASP | E | 151 | 43.179 | 41.150 | 73.070 | 1.00 | 22.54 | O |
|------|------|-----|-----|---|-----|--------|--------|--------|------|-------|---|
| ATOM | 7790 | OD2 | ASP | E | 151 | 42.489 | 39.329 | 74.072 | 1.00 | 27.20 | O |
| ATOM | 7791 | N   | GLY | E | 152 | 45.210 | 39.535 | 68.656 | 1.00 | 20.13 | N |
| ATOM | 7792 | CA  | GLY | E | 152 | 46.128 | 38.941 | 67.671 | 1.00 | 21.68 | C |
| ATOM | 7793 | C   | GLY | E | 152 | 45.703 | 37.597 | 67.083 | 1.00 | 21.92 | C |
| ATOM | 7794 | O   | GLY | E | 152 | 46.341 | 37.100 | 66.155 | 1.00 | 21.68 | O |
| ATOM | 7795 | N   | SER | E | 153 | 44.625 | 37.008 | 67.592 | 1.00 | 22.70 | N |
| ATOM | 7796 | CA  | SER | E | 153 | 44.118 | 35.742 | 67.048 | 1.00 | 23.52 | C |
| ATOM | 7797 | C   | SER | E | 153 | 43.083 | 35.960 | 65.963 | 1.00 | 24.42 | C |
| ATOM | 7798 | O   | SER | E | 153 | 42.097 | 36.663 | 66.200 | 1.00 | 21.91 | O |
| ATOM | 7799 | CB  | SER | E | 153 | 43.469 | 34.919 | 68.163 | 1.00 | 24.49 | C |
| ATOM | 7800 | OG  | SER | E | 153 | 44.475 | 34.316 | 68.951 | 1.00 | 28.74 | O |
| ATOM | 7801 | N   | GLU | E | 154 | 43.258 | 35.327 | 64.799 | 1.00 | 25.24 | N |
| ATOM | 7802 | CA  | GLU | E | 154 | 42.281 | 35.459 | 63.727 | 1.00 | 26.48 | C |
| ATOM | 7803 | C   | GLU | E | 154 | 40.890 | 35.085 | 64.246 | 1.00 | 27.37 | C |
| ATOM | 7804 | O   | GLU | E | 154 | 40.731 | 34.100 | 64.965 | 1.00 | 28.56 | O |
| ATOM | 7805 | CB  | GLU | E | 154 | 42.643 | 34.627 | 62.488 | 1.00 | 27.05 | C |
| ATOM | 7806 | CG  | GLU | E | 154 | 41.603 | 34.791 | 61.356 | 1.00 | 27.56 | C |
| ATOM | 7807 | CD  | GLU | E | 154 | 41.969 | 34.094 | 60.064 | 1.00 | 29.94 | C |
| ATOM | 7808 | OE1 | GLU | E | 154 | 42.880 | 33.228 | 60.076 | 1.00 | 33.79 | O |
| ATOM | 7809 | OE2 | GLU | E | 154 | 41.325 | 34.405 | 59.035 | 1.00 | 34.29 | O |
| ATOM | 7810 | N   | ARG | E | 155 | 39.894 | 35.899 | 63.900 | 1.00 | 28.44 | N |
| ATOM | 7811 | CA  | ARG | E | 155 | 38.506 | 35.675 | 64.306 | 1.00 | 28.49 | C |
| ATOM | 7812 | C   | ARG | E | 155 | 37.633 | 35.595 | 63.060 | 1.00 | 29.37 | C |
| ATOM | 7813 | O   | ARG | E | 155 | 37.672 | 36.472 | 62.207 | 1.00 | 30.68 | O |
| ATOM | 7814 | CB  | ARG | E | 155 | 38.027 | 36.804 | 65.239 | 1.00 | 27.80 | C |
| ATOM | 7815 | CG  | ARG | E | 155 | 36.544 | 36.765 | 65.622 | 1.00 | 28.35 | C |
| ATOM | 7816 | CD  | ARG | E | 155 | 36.212 | 35.543 | 66.470 | 1.00 | 27.96 | C |
| ATOM | 7817 | NE  | ARG | E | 155 | 34.922 | 35.667 | 67.127 | 1.00 | 27.53 | N |
| ATOM | 7818 | CZ  | ARG | E | 155 | 34.695 | 35.525 | 68.427 | 1.00 | 28.83 | C |
| ATOM | 7819 | NH1 | ARG | E | 155 | 35.671 | 35.272 | 69.294 | 1.00 | 29.92 | N |
| ATOM | 7820 | NH2 | ARG | E | 155 | 33.458 | 35.658 | 68.867 | 1.00 | 30.37 | N |
| ATOM | 7821 | N   | GLN | E | 156 | 36.827 | 34.553 | 62.955 | 1.00 | 30.06 | N |
| ATOM | 7822 | CA  | GLN | E | 156 | 35.934 | 34.428 | 61.802 | 1.00 | 30.22 | C |
| ATOM | 7823 | C   | GLN | E | 156 | 34.445 | 34.387 | 62.173 | 1.00 | 29.75 | C |
| ATOM | 7824 | O   | GLN | E | 156 | 33.591 | 34.861 | 61.418 | 1.00 | 31.37 | O |
| ATOM | 7825 | CB  | GLN | E | 156 | 36.348 | 33.205 | 60.989 | 1.00 | 31.50 | C |
| ATOM | 7826 | CG  | GLN | E | 156 | 37.667 | 33.425 | 60.235 | 1.00 | 32.81 | C |
| ATOM | 7827 | CD  | GLN | E | 156 | 38.194 | 32.150 | 59.566 | 1.00 | 33.50 | C |
| ATOM | 7828 | OE1 | GLN | E | 156 | 38.065 | 31.048 | 60.104 | 1.00 | 37.55 | O |
| ATOM | 7829 | NE2 | GLN | E | 156 | 38.798 | 32.306 | 58.394 | 1.00 | 35.67 | N |
| ATOM | 7830 | N   | ASN | E | 157 | 34.135 | 33.830 | 63.337 | 1.00 | 28.58 | N |
| ATOM | 7831 | CA  | ASN | E | 157 | 32.762 | 33.785 | 63.837 | 1.00 | 27.07 | C |
| ATOM | 7832 | C   | ASN | E | 157 | 32.270 | 35.166 | 64.301 | 1.00 | 24.07 | C |
| ATOM | 7833 | O   | ASN | E | 157 | 33.005 | 35.900 | 64.943 | 1.00 | 22.07 | O |
| ATOM | 7834 | CB  | ASN | E | 157 | 32.705 | 32.813 | 64.992 | 1.00 | 28.88 | C |
| ATOM | 7835 | CG  | ASN | E | 157 | 31.318 | 32.374 | 65.300 | 1.00 | 31.65 | C |
| ATOM | 7836 | OD1 | ASN | E | 157 | 30.659 | 31.728 | 64.480 | 1.00 | 34.85 | O |
| ATOM | 7837 | ND2 | ASN | E | 157 | 30.856 | 32.706 | 66.490 | 1.00 | 34.74 | N |
| ATOM | 7838 | N   | GLY | E | 158 | 31.031 | 35.500 | 63.972 | 1.00 | 22.45 | N |
| ATOM | 7839 | CA  | GLY | E | 158 | 30.422 | 36.761 | 64.381 | 1.00 | 21.24 | C |
| ATOM | 7840 | C   | GLY | E | 158 | 30.685 | 37.914 | 63.441 | 1.00 | 19.99 | C |
| ATOM | 7841 | O   | GLY | E | 158 | 30.385 | 39.057 | 63.793 | 1.00 | 18.37 | O |
| ATOM | 7842 | N   | VAL | E | 159 | 31.219 | 37.628 | 62.244 | 1.00 | 19.02 | N |
| ATOM | 7843 | CA  | VAL | E | 159 | 31.582 | 38.686 | 61.280 | 1.00 | 18.75 | C |
| ATOM | 7844 | C   | VAL | E | 159 | 30.530 | 38.784 | 60.212 | 1.00 | 19.97 | C |
| ATOM | 7845 | O   | VAL | E | 159 | 30.165 | 37.786 | 59.620 | 1.00 | 21.27 | O |
| ATOM | 7846 | CB  | VAL | E | 159 | 32.959 | 38.439 | 60.656 | 1.00 | 16.89 | C |
| ATOM | 7847 | CG1 | VAL | E | 159 | 33.292 | 39.498 | 59.517 | 1.00 | 16.25 | C |
| ATOM | 7848 | CG2 | VAL | E | 159 | 34.005 | 38.469 | 61.723 | 1.00 | 14.61 | C |
| ATOM | 7849 | N   | LEU | E | 160 | 30.023 | 39.984 | 59.957 | 1.00 | 19.08 | N |
| ATOM | 7850 | CA  | LEU | E | 160 | 29.071 | 40.157 | 58.865 | 1.00 | 18.82 | C |
| ATOM | 7851 | C   | LEU | E | 160 | 29.609 | 41.200 | 57.898 | 1.00 | 18.24 | C |
| ATOM | 7852 | O   | LEU | E | 160 | 29.992 | 42.264 | 58.324 | 1.00 | 17.64 | O |
| ATOM | 7853 | CB  | LEU | E | 160 | 27.695 | 40.561 | 59.388 | 1.00 | 19.66 | C |

| ATOM | 7854 | CG | LEU | E | 160 | 26.800 | 39.494 | 60.045 | 1.00 | 21.27 | C |
|------|------|-----|-----|---|-----|--------|--------|--------|------|-------|---|
| ATOM | 7855 | CD1 | LEU | E | 160 | 25.321 | 39.879 | 59.938 | 1.00 | 22.66 | C |
| ATOM | 7856 | CD2 | LEU | E | 160 | 27.015 | 38.046 | 59.443 | 1.00 | 23.26 | C |
| ATOM | 7857 | N | ASN | E | 161 | 29.640 | 40.854 | 56.613 | 1.00 | 16.72 | N |
| ATOM | 7858 | CA | ASN | E | 161 | 30.201 | 41.684 | 55.561 | 1.00 | 17.17 | C |
| ATOM | 7859 | C | ASN | E | 161 | 29.116 | 42.183 | 54.602 | 1.00 | 17.57 | C |
| ATOM | 7860 | O | ASN | E | 161 | 28.147 | 41.491 | 54.316 | 1.00 | 17.29 | O |
| ATOM | 7861 | CB | ASN | E | 161 | 31.309 | 40.949 | 54.803 | 1.00 | 17.23 | C |
| ATOM | 7862 | CG | ASN | E | 161 | 32.516 | 41.611 | 55.690 | 1.00 | 16.95 | C |
| ATOM | 7863 | OD1 | ASN | E | 161 | 32.850 | 41.342 | 56.598 | 1.00 | 16.61 | O |
| ATOM | 7864 | ND2 | ASN | E | 161 | 33.123 | 39.442 | 55.452 | 1.00 | 19.19 | N |
| ATOM | 7865 | N | SER | E | 162 | 29.293 | 43.392 | 54.084 | 1.00 | 17.49 | N |
| ATOM | 7866 | CA | SER | E | 162 | 28.429 | 43.897 | 53.043 | 1.00 | 17.96 | C |
| ATOM | 7867 | C | SER | E | 162 | 29.167 | 44.842 | 52.103 | 1.00 | 18.11 | C |
| ATOM | 7868 | O | SER | E | 162 | 30.016 | 45.613 | 52.540 | 1.00 | 20.31 | O |
| ATOM | 7869 | CB | SER | E | 162 | 27.259 | 44.640 | 53.665 | 1.00 | 17.44 | C |
| ATOM | 7870 | OG | SER | E | 162 | 26.277 | 44.902 | 52.679 | 1.00 | 18.99 | O |
| ATOM | 7871 | N | TRP | E | 163 | 28.818 | 44.779 | 50.823 | 1.00 | 18.49 | N |
| ATOM | 7872 | CA | TRP | E | 163 | 29.509 | 45.489 | 49.734 | 1.00 | 19.13 | C |
| ATOM | 7873 | C | TRP | E | 163 | 28.560 | 46.423 | 48.994 | 1.00 | 18.26 | C |
| ATOM | 7874 | O | TRP | E | 163 | 27.435 | 46.039 | 48.634 | 1.00 | 14.64 | O |
| ATOM | 7875 | CB | TRP | E | 163 | 30.102 | 44.467 | 48.757 | 1.00 | 23.02 | C |
| ATOM | 7876 | CG | TRP | E | 163 | 31.056 | 43.462 | 49.402 | 1.00 | 24.64 | C |
| ATOM | 7877 | CD1 | TRP | E | 163 | 32.422 | 43.532 | 49.445 | 1.00 | 24.95 | C |
| ATOM | 7878 | CD2 | TRP | E | 163 | 30.696 | 42.242 | 50.078 | 1.00 | 25.13 | C |
| ATOM | 7879 | NE1 | TRP | E | 163 | 32.932 | 42.442 | 50.101 | 1.00 | 26.70 | N |
| ATOM | 7880 | CE2 | TRP | E | 163 | 31.899 | 41.631 | 50.502 | 1.00 | 25.77 | C |
| ATOM | 7881 | CE3 | TRP | E | 163 | 29.481 | 41.604 | 50.349 | 1.00 | 26.73 | C |
| ATOM | 7882 | CZ2 | TRP | E | 163 | 31.919 | 40.413 | 51.201 | 1.00 | 25.63 | C |
| ATOM | 7883 | CZ3 | TRP | E | 163 | 29.495 | 40.398 | 51.052 | 1.00 | 25.91 | C |
| ATOM | 7884 | CH2 | TRP | E | 163 | 30.708 | 39.816 | 51.474 | 1.00 | 25.35 | C |
| ATOM | 7885 | N | THR | E | 164 | 28.974 | 47.673 | 48.766 | 1.00 | 17.96 | N |
| ATOM | 7886 | CA | THR | E | 164 | 28.130 | 48.577 | 47.969 | 1.00 | 18.71 | C |
| ATOM | 7887 | C | THR | E | 164 | 28.187 | 48.198 | 46.493 | 1.00 | 19.08 | C |
| ATOM | 7888 | O | THR | E | 164 | 29.105 | 47.489 | 46.043 | 1.00 | 20.91 | O |
| ATOM | 7889 | CB | THR | E | 164 | 28.551 | 50.050 | 48.081 | 1.00 | 18.04 | C |
| ATOM | 7890 | OG1 | THR | E | 164 | 29.973 | 50.158 | 47.967 | 1.00 | 16.00 | O |
| ATOM | 7891 | CG2 | THR | E | 164 | 28.096 | 50.665 | 49.391 | 1.00 | 19.54 | C |
| ATOM | 7892 | N | ASP | E | 165 | 27.199 | 48.677 | 45.744 | 1.00 | 19.92 | N |
| ATOM | 7893 | CA | ASP | E | 165 | 27.282 | 48.657 | 44.297 | 1.00 | 19.99 | C |
| ATOM | 7894 | C | ASP | E | 165 | 28.279 | 49.717 | 43.917 | 1.00 | 19.82 | C |
| ATOM | 7895 | O | ASP | E | 165 | 28.611 | 50.561 | 44.730 | 1.00 | 19.89 | O |
| ATOM | 7896 | CB | ASP | E | 165 | 25.940 | 48.996 | 43.639 | 1.00 | 21.55 | C |
| ATOM | 7897 | CG | ASP | E | 165 | 24.815 | 48.094 | 44.072 | 1.00 | 24.97 | C |
| ATOM | 7898 | OD1 | ASP | E | 165 | 24.894 | 46.853 | 43.822 | 1.00 | 23.25 | O |
| ATOM | 7899 | OD2 | ASP | E | 165 | 23.846 | 48.658 | 44.653 | 1.00 | 29.42 | O |
| ATOM | 7900 | N | GLN | E | 166 | 28.725 | 49.708 | 42.669 | 1.00 | 18.56 | N |
| ATOM | 7901 | CA | GLN | E | 166 | 29.696 | 50.688 | 42.211 | 1.00 | 17.79 | C |
| ATOM | 7902 | C | GLN | E | 166 | 29.117 | 52.092 | 42.399 | 1.00 | 18.20 | C |
| ATOM | 7903 | O | GLN | E | 166 | 27.963 | 52.308 | 42.105 | 1.00 | 19.70 | O |
| ATOM | 7904 | CB | GLN | E | 166 | 30.025 | 50.432 | 40.740 | 1.00 | 17.74 | C |
| ATOM | 7905 | CG | GLN | E | 166 | 31.149 | 51.284 | 40.193 | 1.00 | 18.73 | C |
| ATOM | 7906 | CD | GLN | E | 166 | 31.733 | 50.714 | 38.896 | 1.00 | 16.53 | C |
| ATOM | 7907 | OE1 | GLN | E | 166 | 30.995 | 50.161 | 38.066 | 1.00 | 18.14 | O |
| ATOM | 7908 | NE2 | GLN | E | 166 | 33.069 | 50.816 | 38.732 | 1.00 | 11.77 | N |
| ATOM | 7909 | N | ASP | E | 167 | 29.936 | 53.010 | 42.910 | 1.00 | 18.63 | N |
| ATOM | 7910 | CA | ASP | E | 167 | 29.557 | 54.394 | 43.213 | 1.00 | 19.54 | C |
| ATOM | 7911 | C | ASP | E | 167 | 29.292 | 55.192 | 41.931 | 1.00 | 19.54 | C |
| ATOM | 7912 | O | ASP | E | 167 | 30.090 | 55.131 | 41.002 | 1.00 | 17.61 | O |
| ATOM | 7913 | CB | ASP | E | 167 | 30.695 | 55.058 | 43.979 | 1.00 | 19.31 | C |
| ATOM | 7914 | CG | ASP | E | 167 | 30.308 | 56.412 | 44.574 | 1.00 | 20.97 | C |
| ATOM | 7915 | OD1 | ASP | E | 167 | 29.667 | 56.402 | 45.643 | 1.00 | 21.30 | O |
| ATOM | 7916 | OD2 | ASP | E | 167 | 30.657 | 57.477 | 43.993 | 1.00 | 20.40 | O |
| ATOM | 7917 | N | SER | E | 168 | 28.185 | 55.951 | 41.912 | 1.00 | 20.49 | N |
| ATOM | 7918 | CA | SER | E | 168 | 27.746 | 56.739 | 40.753 | 1.00 | 22.62 | C |

| ATOM | 7919 | C | SER E 168 | 28.595 | 57.980 | 40.464 | 1.00 24.20 | C |
| ATOM | 7920 | O | SER E 168 | 28.426 | 58.620 | 39.404 | 1.00 23.63 | O |
| ATOM | 7921 | CB | SER E 168 | 26.295 | 57.212 | 40.936 | 1.00 23.54 | C |
| ATOM | 7922 | OG | SER E 168 | 25.374 | 56.130 | 40.816 | 1.00 28.58 | O |
| ATOM | 7923 | N | LYS E 169 | 29.477 | 58.333 | 41.402 | 1.00 24.75 | N |
| ATOM | 7924 | CA | LYS E 169 | 30.269 | 59.564 | 41.313 | 1.00 25.00 | C |
| ATOM | 7925 | C | LYS E 169 | 31.731 | 59.276 | 41.008 | 1.00 24.48 | C |
| ATOM | 7926 | O | LYS E 169 | 32.295 | 59.874 | 40.107 | 1.00 24.19 | O |
| ATOM | 7927 | CB | LYS E 169 | 30.162 | 60.364 | 42.622 | 1.00 28.08 | C |
| ATOM | 7928 | CG | LYS E 169 | 29.166 | 61.521 | 42.593 | 1.00 30.82 | C |
| ATOM | 7929 | CD | LYS E 169 | 27.728 | 61.077 | 42.745 | 1.00 33.34 | C |
| ATOM | 7930 | CE | LYS E 169 | 26.814 | 62.291 | 43.008 | 1.00 33.62 | C |
| ATOM | 7931 | NZ | LYS E 169 | 27.108 | 63.405 | 42.058 | 1.00 35.61 | N |
| ATOM | 7932 | N | ASP E 170 | 32.341 | 58.354 | 41.755 | 1.00 22.93 | N |
| ATOM | 7933 | CA | ASP E 170 | 33.749 | 58.072 | 41.573 | 1.00 21.99 | C |
| ATOM | 7934 | C | ASP E 170 | 34.028 | 56.651 | 41.106 | 1.00 20.13 | C |
| ATOM | 7935 | O | ASP E 170 | 35.160 | 56.297 | 40.911 | 1.00 18.62 | O |
| ATOM | 7936 | CB | ASP E 170 | 34.560 | 58.436 | 42.826 | 1.00 22.81 | C |
| ATOM | 7937 | CG | ASP E 170 | 34.383 | 57.443 | 43.959 | 1.00 24.93 | C |
| ATOM | 7938 | OD1 | ASP E 170 | 33.696 | 56.421 | 43.763 | 1.00 22.60 | O |
| ATOM | 7939 | OD2 | ASP E 170 | 34.951 | 57.678 | 45.047 | 1.00 24.81 | O |
| ATOM | 7940 | N | SER E 171 | 32.991 | 55.869 | 40.875 | 1.00 20.29 | N |
| ATOM | 7941 | CA | SER E 171 | 33.147 | 54.552 | 40.245 | 1.00 19.21 | C |
| ATOM | 7942 | C | SER E 171 | 33.908 | 53.552 | 41.114 | 1.00 18.22 | C |
| ATOM | 7943 | O | SER E 171 | 34.405 | 52.535 | 40.613 | 1.00 18.37 | O |
| ATOM | 7944 | CB | SER E 171 | 33.821 | 54.670 | 38.866 | 1.00 20.38 | C |
| ATOM | 7945 | OG | SER E 171 | 33.073 | 55.477 | 37.980 | 1.00 20.91 | O |
| ATOM | 7946 | N | THR E 172 | 33.956 | 53.821 | 42.412 | 1.00 17.84 | N |
| ATOM | 7947 | CA | THR E 172 | 34.637 | 52.931 | 43.369 | 1.00 17.04 | C |
| ATOM | 7948 | C | THR E 172 | 33.662 | 52.021 | 44.105 | 1.00 15.63 | C |
| ATOM | 7949 | O | THR E 172 | 32.450 | 52.066 | 43.896 | 1.00 12.73 | O |
| ATOM | 7950 | CB | THR E 172 | 35.406 | 53.715 | 44.438 | 1.00 16.38 | C |
| ATOM | 7951 | OG1 | THR E 172 | 34.487 | 54.470 | 45.239 | 1.00 17.86 | O |
| ATOM | 7952 | CG2 | THR E 172 | 36.485 | 54.595 | 43.819 | 1.00 15.83 | C |
| ATOM | 7953 | N | TYR E 173 | 34.236 | 51.143 | 44.919 | 1.00 15.82 | N |
| ATOM | 7954 | CA | TYR E 173 | 33.480 | 50.156 | 45.699 | 1.00 15.29 | C |
| ATOM | 7955 | C | TYR E 173 | 33.805 | 50.394 | 47.164 | 1.00 14.71 | C |
| ATOM | 7956 | O | TYR E 173 | 34.806 | 51.035 | 47.463 | 1.00 14.18 | O |
| ATOM | 7957 | CB | TYR E 173 | 33.892 | 48.731 | 45.291 | 1.00 15.63 | C |
| ATOM | 7958 | CG | TYR E 173 | 33.491 | 48.370 | 43.871 | 1.00 15.49 | C |
| ATOM | 7959 | CD1 | TYR E 173 | 34.361 | 48.582 | 42.802 | 1.00 17.39 | C |
| ATOM | 7960 | CD2 | TYR E 173 | 32.251 | 47.887 | 43.592 | 1.00 15.69 | C |
| ATOM | 7961 | CE1 | TYR E 173 | 33.982 | 48.291 | 41.491 | 1.00 17.43 | C |
| ATOM | 7962 | CE2 | TYR E 173 | 31.874 | 47.570 | 42.300 | 1.00 15.12 | C |
| ATOM | 7963 | CZ | TYR E 173 | 32.744 | 47.781 | 41.256 | 1.00 15.36 | C |
| ATOM | 7964 | OH | TYR E 173 | 32.360 | 47.451 | 39.974 | 1.00 17.39 | O |
| ATOM | 7965 | N | SER E 174 | 32.945 | 49.915 | 48.077 | 1.00 13.63 | N |
| ATOM | 7966 | CA | SER E 174 | 33.218 | 49.973 | 49.494 | 1.00 13.86 | C |
| ATOM | 7967 | C | SER E 174 | 32.688 | 48.701 | 50.124 | 1.00 14.00 | C |
| ATOM | 7968 | O | SER E 174 | 31.823 | 48.047 | 49.552 | 1.00 14.90 | O |
| ATOM | 7969 | CB | SER E 174 | 32.555 | 51.186 | 50.156 | 1.00 13.89 | C |
| ATOM | 7970 | OG | SER E 174 | 33.032 | 52.399 | 49.585 | 1.00 14.26 | O |
| ATOM | 7971 | N | MET E 175 | 33.199 | 48.396 | 51.303 | 1.00 15.81 | N |
| ATOM | 7972 | CA | MET E 175 | 32.777 | 47.230 | 52.057 | 1.00 16.77 | C |
| ATOM | 7973 | C | MET E 175 | 32.685 | 47.597 | 53.518 | 1.00 15.38 | C |
| ATOM | 7974 | O | MET E 175 | 33.502 | 48.392 | 54.017 | 1.00 13.15 | O |
| ATOM | 7975 | CB | MET E 175 | 33.797 | 46.114 | 51.883 | 1.00 17.32 | C |
| ATOM | 7976 | CG | MET E 175 | 33.463 | 44.798 | 52.575 | 1.00 20.66 | C |
| ATOM | 7977 | SD | MET E 175 | 34.996 | 43.921 | 52.720 | 1.00 24.66 | S |
| ATOM | 7978 | CE | MET E 175 | 34.456 | 42.269 | 53.130 | 1.00 23.75 | C |
| ATOM | 7979 | N | SER E 176 | 31.705 | 46.999 | 54.205 | 1.00 13.67 | N |
| ATOM | 7980 | CA | SER E 176 | 31.553 | 47.160 | 55.632 | 1.00 14.22 | C |
| ATOM | 7981 | C | SER E 176 | 31.681 | 45.770 | 56.212 | 1.00 14.57 | C |
| ATOM | 7982 | O | SER E 176 | 31.092 | 44.850 | 55.657 | 1.00 14.65 | O |
| ATOM | 7983 | CB | SER E 176 | 30.198 | 47.684 | 55.981 | 1.00 14.31 | C |

```
ATOM   7984  OG   SER E 176      30.099  47.924  57.374  1.00  16.71           O
ATOM   7985  N    SER E 177      32.437  45.652  57.316  1.00  16.34           N
ATOM   7986  CA   SER E 177      32.591  44.393  58.082  1.00  14.80           C
ATOM   7987  C    SER E 177      32.298  44.695  59.549  1.00  14.88           C
ATOM   7988  O    SER E 177      32.878  45.623  60.119  1.00  13.69           O
ATOM   7989  CB   SER E 177      34.002  43.820  57.974  1.00  15.73           C
ATOM   7990  OG   SER E 177      34.026  42.489  58.448  1.00  19.72           O
ATOM   7991  N    THR E 178      31.373  43.929  60.129  1.00  15.74           N
ATOM   7992  CA   THR E 178      30.957  44.095  61.522  1.00  14.67           C
ATOM   7993  C    THR E 178      31.210  42.812  62.290  1.00  16.40           C
ATOM   7994  O    THR E 178      30.736  41.771  61.886  1.00  15.42           O
ATOM   7995  CB   THR E 178      29.467  44.461  61.625  1.00  16.83           C
ATOM   7996  OG1  THR E 178      29.205  45.598  60.808  1.00  17.30           O
ATOM   7997  CG2  THR E 178      29.057  44.775  63.051  1.00  14.73           C
ATOM   7998  N    LEU E 179      31.990  42.909  63.374  1.00  16.73           N
ATOM   7999  CA   LEU E 179      32.224  41.783  64.275  1.00  14.85           C
ATOM   8000  C    LEU E 179      31.344  42.004  65.502  1.00  14.74           C
ATOM   8001  O    LEU E 179      31.549  42.967  66.209  1.00  15.47           O
ATOM   8002  CB   LEU E 179      33.679  41.762  64.677  1.00  15.21           C
ATOM   8003  CG   LEU E 179      34.089  40.830  65.824  1.00  15.87           C
ATOM   8004  CD1  LEU E 179      33.747  39.401  65.486  1.00  15.24           C
ATOM   8005  CD2  LEU E 179      35.579  40.960  66.167  1.00  17.65           C
ATOM   8006  N    THR E 180      30.343  41.158  65.731  1.00  12.24           N
ATOM   8007  CA   THR E 180      29.422  41.331  66.873  1.00  12.97           C
ATOM   8008  C    THR E 180      29.657  40.285  67.955  1.00  14.54           C
ATOM   8009  O    THR E 180      29.670  39.074  67.661  1.00  11.46           O
ATOM   8010  CB   THR E 180      27.948  41.300  66.401  1.00  14.51           C
ATOM   8011  OG1  THR E 180      27.732  42.427  65.539  1.00  16.62           O
ATOM   8012  CG2  THR E 180      26.988  41.423  67.554  1.00  13.75           C
ATOM   8013  N    LEU E 181      29.878  40.778  69.162  1.00  13.92           N
ATOM   8014  CA   LEU E 181      30.122  39.952  70.319  1.00  14.69           C
ATOM   8015  C    LEU E 181      29.091  40.354  71.374  1.00  15.07           C
ATOM   8016  O    LEU E 181      28.465  41.435  71.308  1.00  14.78           O
ATOM   8017  CB   LEU E 181      31.548  40.205  70.854  1.00  14.71           C
ATOM   8018  CG   LEU E 181      32.786  39.966  69.974  1.00  15.98           C
ATOM   8019  CD1  LEU E 181      34.021  40.336  70.717  1.00  18.82           C
ATOM   8020  CD2  LEU E 181      32.890  38.490  69.595  1.00  17.63           C
ATOM   8021  N    THR E 182      28.952  39.507  72.377  1.00  13.63           N
ATOM   8022  CA   THR E 182      28.292  39.878  73.611  1.00  14.18           C
ATOM   8023  C    THR E 182      29.216  40.849  74.343  1.00  14.70           C
ATOM   8024  O    THR E 182      30.448  40.832  74.162  1.00  12.18           O
ATOM   8025  CB   THR E 182      27.934  38.643  74.507  1.00  14.89           C
ATOM   8026  OG1  THR E 182      29.144  38.046  74.965  1.00  13.08           O
ATOM   8027  CG2  THR E 182      27.116  37.676  73.747  1.00  15.31           C
ATOM   8028  N    LYS E 183      28.620  41.749  75.126  1.00  13.82           N
ATOM   8029  CA   LYS E 183      29.405  42.512  76.091  1.00  14.05           C
ATOM   8030  C    LYS E 183      30.345  41.642  76.916  1.00  15.26           C
ATOM   8031  O    LYS E 183      31.510  41.980  77.098  1.00  12.73           O
ATOM   8032  CB   LYS E 183      28.516  43.339  77.018  1.00  13.65           C
ATOM   8033  CG   LYS E 183      29.269  44.251  77.951  1.00  14.07           C
ATOM   8034  CD   LYS E 183      28.350  44.821  78.979  1.00  15.05           C
ATOM   8035  CE   LYS E 183      28.969  46.036  79.691  1.00  17.61           C
ATOM   8036  NZ   LYS E 183      28.689  45.999  81.152  1.00  19.68           N
ATOM   8037  N    ASP E 184      29.844  40.520  77.412  1.00  14.74           N
ATOM   8038  CA   ASP E 184      30.683  39.662  78.206  1.00  15.80           C
ATOM   8039  C    ASP E 184      31.968  39.320  77.449  1.00  14.47           C
ATOM   8040  O    ASP E 184      33.076  39.524  77.963  1.00  16.22           O
ATOM   8041  CB   ASP E 184      29.906  38.427  78.642  1.00  14.42           C
ATOM   8042  CG   ASP E 184      30.772  37.402  79.340  1.00  18.21           C
ATOM   8043  OD1  ASP E 184      31.443  37.761  80.323  1.00  19.63           O
ATOM   8044  OD2  ASP E 184      30.770  36.222  78.909  1.00  20.66           O
ATOM   8045  N    GLU E 185      31.846  38.771  76.258  1.00  14.70           N
ATOM   8046  CA   GLU E 185      33.043  38.341  75.548  1.00  16.01           C
ATOM   8047  C    GLU E 185      33.912  39.531  75.110  1.00  15.54           C
ATOM   8048  O    GLU E 185      35.125  39.474  75.218  1.00  16.22           O
```

| ATOM | 8049 | CB | GLU | E | 185 | 32.712 | 37.401 | 74.389 | 1.00 | 17.19 | C |
|------|------|------|-----|---|-----|--------|--------|--------|------|-------|---|
| ATOM | 8050 | CG | GLU | E | 185 | 33.962 | 36.912 | 73.700 | 1.00 | 18.13 | C |
| ATOM | 8051 | CD | GLU | E | 185 | 33.719 | 35.991 | 72.568 | 1.00 | 20.40 | C |
| ATOM | 8052 | OE1 | GLU | E | 185 | 32.587 | 35.958 | 72.031 | 1.00 | 25.63 | O |
| ATOM | 8053 | OE2 | GLU | E | 185 | 34.690 | 35.302 | 72.186 | 1.00 | 24.42 | O |
| ATOM | 8054 | N | TYR | E | 186 | 33.291 | 40.612 | 74.662 | 1.00 | 14.80 | N |
| ATOM | 8055 | CA | TYR | E | 186 | 34.034 | 41.795 | 74.292 | 1.00 | 15.57 | C |
| ATOM | 8056 | C | TYR | E | 186 | 34.977 | 42.207 | 75.410 | 1.00 | 15.99 | C |
| ATOM | 8057 | O | TYR | E | 186 | 36.125 | 42.598 | 75.167 | 1.00 | 18.52 | O |
| ATOM | 8058 | CB | TYR | E | 186 | 33.051 | 42.906 | 73.978 | 1.00 | 14.51 | C |
| ATOM | 8059 | CG | TYR | E | 186 | 33.691 | 44.214 | 73.677 | 1.00 | 13.97 | C |
| ATOM | 8060 | CD1 | TYR | E | 186 | 34.460 | 44.379 | 72.519 | 1.00 | 15.66 | C |
| ATOM | 8061 | CD2 | TYR | E | 186 | 33.499 | 45.297 | 74.499 | 1.00 | 13.82 | C |
| ATOM | 8062 | CE1 | TYR | E | 186 | 35.066 | 45.611 | 72.210 | 1.00 | 13.99 | C |
| ATOM | 8063 | CE2 | TYR | E | 186 | 34.109 | 46.553 | 74.201 | 1.00 | 13.63 | C |
| ATOM | 8064 | CZ | TYR | E | 186 | 34.888 | 46.667 | 73.046 | 1.00 | 15.90 | C |
| ATOM | 8065 | OH | TYR | E | 186 | 35.460 | 47.859 | 72.732 | 1.00 | 14.21 | O |
| ATOM | 8066 | N | GLU | E | 187 | 34.506 | 42.120 | 76.649 | 1.00 | 17.24 | N |
| ATOM | 8067 | CA | GLU | E | 187 | 35.226 | 42.701 | 77.776 | 1.00 | 18.93 | C |
| ATOM | 8068 | C | GLU | E | 187 | 36.289 | 41.758 | 78.321 | 1.00 | 18.69 | C |
| ATOM | 8069 | O | GLU | E | 187 | 37.087 | 42.162 | 79.145 | 1.00 | 18.84 | O |
| ATOM | 8070 | CB | GLU | E | 187 | 34.254 | 43.204 | 78.861 | 1.00 | 18.37 | C |
| ATOM | 8071 | CG | GLU | E | 187 | 33.488 | 44.451 | 78.420 | 1.00 | 19.45 | C |
| ATOM | 8072 | CD | GLU | E | 187 | 32.617 | 45.102 | 79.487 | 1.00 | 20.62 | C |
| ATOM | 8073 | OE1 | GLU | E | 187 | 32.177 | 44.425 | 80.452 | 1.00 | 24.16 | O |
| ATOM | 8074 | OE2 | GLU | E | 187 | 32.324 | 46.318 | 79.320 | 1.00 | 26.36 | O |
| ATOM | 8075 | N | ARG | E | 188 | 36.301 | 40.521 | 77.815 | 1.00 | 19.93 | N |
| ATOM | 8076 | CA | ARG | E | 188 | 37.361 | 39.538 | 78.088 | 1.00 | 22.02 | C |
| ATOM | 8077 | C | ARG | E | 188 | 38.604 | 39.681 | 77.194 | 1.00 | 22.99 | C |
| ATOM | 8078 | O | ARG | E | 188 | 39.561 | 38.937 | 77.370 | 1.00 | 23.13 | O |
| ATOM | 8079 | CB | ARG | E | 188 | 36.804 | 38.119 | 77.942 | 1.00 | 22.68 | C |
| ATOM | 8080 | CG | ARG | E | 188 | 35.586 | 37.845 | 78.777 | 1.00 | 23.60 | C |
| ATOM | 8081 | CD | ARG | E | 188 | 35.178 | 36.362 | 78.841 | 1.00 | 27.42 | C |
| ATOM | 8082 | NE | ARG | E | 188 | 35.102 | 35.683 | 77.544 | 1.00 | 31.68 | N |
| ATOM | 8083 | CZ | ARG | E | 188 | 34.006 | 35.120 | 77.008 | 1.00 | 33.08 | C |
| ATOM | 8084 | NH1 | ARG | E | 188 | 32.826 | 35.158 | 77.621 | 1.00 | 34.47 | N |
| ATOM | 8085 | NH2 | ARG | E | 188 | 34.082 | 34.520 | 75.825 | 1.00 | 33.98 | N |
| ATOM | 8086 | N | HIS | E | 189 | 38.600 | 40.625 | 76.250 | 1.00 | 21.53 | N |
| ATOM | 8087 | CA | HIS | E | 189 | 39.746 | 40.868 | 75.363 | 1.00 | 22.58 | C |
| ATOM | 8088 | C | HIS | E | 189 | 40.009 | 42.335 | 75.291 | 1.00 | 22.03 | C |
| ATOM | 8089 | O | HIS | E | 189 | 39.233 | 43.102 | 75.807 | 1.00 | 22.39 | O |
| ATOM | 8090 | CB | HIS | E | 189 | 39.456 | 40.318 | 73.979 | 1.00 | 23.27 | C |
| ATOM | 8091 | CG | HIS | E | 189 | 39.088 | 38.874 | 74.010 | 1.00 | 23.56 | C |
| ATOM | 8092 | ND1 | HIS | E | 189 | 40.029 | 37.876 | 74.126 | 1.00 | 24.34 | N |
| ATOM | 8093 | CD2 | HIS | E | 189 | 37.886 | 38.267 | 74.024 | 1.00 | 24.37 | C |
| ATOM | 8094 | CE1 | HIS | E | 189 | 39.418 | 36.707 | 74.175 | 1.00 | 25.57 | C |
| ATOM | 8095 | NE2 | HIS | E | 189 | 38.116 | 36.917 | 74.117 | 1.00 | 24.96 | N |
| ATOM | 8096 | N | ASN | E | 190 | 41.099 | 42.720 | 74.651 | 1.00 | 22.49 | N |
| ATOM | 8097 | CA | ASN | E | 190 | 41.538 | 44.112 | 74.680 | 1.00 | 23.15 | C |
| ATOM | 8098 | C | ASN | E | 190 | 41.682 | 44.744 | 73.312 | 1.00 | 22.46 | C |
| ATOM | 8099 | O | ASN | E | 190 | 41.252 | 45.864 | 73.089 | 1.00 | 24.10 | O |
| ATOM | 8100 | CB | ASN | E | 190 | 42.852 | 44.205 | 75.469 | 1.00 | 24.70 | C |
| ATOM | 8101 | CG | ASN | E | 190 | 42.638 | 44.059 | 76.976 | 1.00 | 28.20 | C |
| ATOM | 8102 | OD1 | ASN | E | 190 | 42.606 | 42.938 | 77.502 | 1.00 | 30.35 | O |
| ATOM | 8103 | ND2 | ASN | E | 190 | 42.503 | 45.206 | 77.682 | 1.00 | 27.42 | N |
| ATOM | 8104 | N | SER | E | 191 | 42.272 | 44.015 | 72.387 | 1.00 | 22.39 | N |
| ATOM | 8105 | CA | SER | E | 191 | 42.707 | 44.594 | 71.131 | 1.00 | 21.36 | C |
| ATOM | 8106 | C | SER | E | 191 | 41.854 | 44.061 | 69.978 | 1.00 | 20.20 | C |
| ATOM | 8107 | O | SER | E | 191 | 41.696 | 42.854 | 69.801 | 1.00 | 18.82 | O |
| ATOM | 8108 | CB | SER | E | 191 | 44.191 | 44.281 | 70.951 | 1.00 | 21.14 | C |
| ATOM | 8109 | OG | SER | E | 191 | 44.536 | 44.238 | 69.619 | 1.00 | 25.15 | O |
| ATOM | 8110 | N | TYR | E | 192 | 41.299 | 44.974 | 69.193 | 1.00 | 18.58 | N |
| ATOM | 8111 | CA | TYR | E | 192 | 40.419 | 44.602 | 68.086 | 1.00 | 18.65 | C |
| ATOM | 8112 | C | TYR | E | 192 | 40.976 | 45.128 | 66.779 | 1.00 | 18.42 | C |
| ATOM | 8113 | O | TYR | E | 192 | 41.220 | 46.326 | 66.653 | 1.00 | 19.66 | O |

| ATOM | 8114 | CB | TYR | E | 192 | 39.012 | 45.101 | 68.346 | 1.00 | 17.88 | C |
|------|------|-----|-----|---|-----|--------|--------|--------|------|-------|---|
| ATOM | 8115 | CG | TYR | E | 192 | 38.408 | 44.324 | 69.490 | 1.00 | 18.26 | C |
| ATOM | 8116 | CD1 | TYR | E | 192 | 37.700 | 43.141 | 69.237 | 1.00 | 15.63 | C |
| ATOM | 8117 | CD2 | TYR | E | 192 | 38.609 | 44.706 | 70.819 | 1.00 | 16.75 | C |
| ATOM | 8118 | CE1 | TYR | E | 192 | 37.186 | 42.409 | 70.247 | 1.00 | 18.05 | C |
| ATOM | 8119 | CE2 | TYR | E | 192 | 38.067 | 43.941 | 71.865 | 1.00 | 16.51 | C |
| ATOM | 8120 | CZ | TYR | E | 192 | 37.364 | 42.786 | 71.550 | 1.00 | 16.98 | C |
| ATOM | 8121 | OH | TYR | E | 192 | 36.811 | 41.983 | 72.548 | 1.00 | 16.52 | O |
| ATOM | 8122 | N | THR | E | 193 | 41.184 | 44.214 | 65.836 | 1.00 | 17.66 | N |
| ATOM | 8123 | CA | THR | E | 193 | 41.933 | 44.509 | 64.621 | 1.00 | 18.31 | C |
| ATOM | 8124 | C | THR | E | 193 | 41.213 | 44.106 | 63.340 | 1.00 | 17.53 | C |
| ATOM | 8125 | O | THR | E | 193 | 40.799 | 42.964 | 63.172 | 1.00 | 17.11 | O |
| ATOM | 8126 | CB | THR | E | 193 | 43.353 | 43.848 | 64.671 | 1.00 | 17.75 | C |
| ATOM | 8127 | OG1 | THR | E | 193 | 44.059 | 44.325 | 65.821 | 1.00 | 17.30 | O |
| ATOM | 8128 | CG2 | THR | E | 193 | 44.155 | 44.148 | 63.439 | 1.00 | 19.00 | C |
| ATOM | 8129 | N | CYS | E | 194 | 41.109 | 45.091 | 62.446 | 1.00 | 19.56 | N |
| ATOM | 8130 | CA | CYS | E | 194 | 40.630 | 44.951 | 61.087 | 1.00 | 20.36 | C |
| ATOM | 8131 | C | CYS | E | 194 | 41.804 | 45.070 | 60.098 | 1.00 | 19.70 | C |
| ATOM | 8132 | O | CYS | E | 194 | 42.455 | 46.123 | 60.053 | 1.00 | 17.72 | O |
| ATOM | 8133 | CB | CYS | E | 194 | 39.676 | 46.131 | 60.782 | 1.00 | 21.07 | C |
| ATOM | 8134 | SG | CYS | E | 194 | 38.912 | 45.979 | 59.272 | 1.00 | 28.52 | S |
| ATOM | 8135 | N | GLU | E | 195 | 42.027 | 44.028 | 59.296 | 1.00 | 18.34 | N |
| ATOM | 8136 | CA | GLU | E | 195 | 43.057 | 44.048 | 58.235 | 1.00 | 20.37 | C |
| ATOM | 8137 | C | GLU | E | 195 | 42.445 | 43.883 | 56.827 | 1.00 | 20.53 | C |
| ATOM | 8138 | O | GLU | E | 195 | 41.762 | 42.906 | 56.546 | 1.00 | 20.23 | O |
| ATOM | 8139 | CB | GLU | E | 195 | 44.109 | 42.963 | 58.482 | 1.00 | 21.85 | C |
| ATOM | 8140 | CG | GLU | E | 195 | 44.602 | 42.848 | 59.913 | 1.00 | 21.27 | C |
| ATOM | 8141 | CD | GLU | E | 195 | 45.828 | 41.930 | 60.044 | 1.00 | 23.65 | C |
| ATOM | 8142 | OE1 | GLU | E | 195 | 45.966 | 40.991 | 59.225 | 1.00 | 25.06 | O |
| ATOM | 8143 | OE2 | GLU | E | 195 | 46.624 | 42.147 | 60.983 | 1.00 | 25.11 | O |
| ATOM | 8144 | N | ALA | E | 196 | 42.729 | 44.839 | 55.942 | 1.00 | 19.23 | N |
| ATOM | 8145 | CA | ALA | E | 196 | 42.182 | 44.864 | 54.590 | 1.00 | 19.24 | C |
| ATOM | 8146 | C | ALA | E | 196 | 43.256 | 44.488 | 53.564 | 1.00 | 20.38 | C |
| ATOM | 8147 | O | ALA | E | 196 | 44.282 | 45.146 | 53.498 | 1.00 | 21.39 | O |
| ATOM | 8148 | CB | ALA | E | 196 | 41.682 | 46.282 | 54.295 | 1.00 | 18.67 | C |
| ATOM | 8149 | N | THR | E | 197 | 43.044 | 43.417 | 52.801 | 1.00 | 20.17 | N |
| ATOM | 8150 | CA | THR | E | 197 | 43.976 | 43.067 | 51.724 | 1.00 | 20.36 | C |
| ATOM | 8151 | C | THR | E | 197 | 43.352 | 43.390 | 50.361 | 1.00 | 20.41 | C |
| ATOM | 8152 | O | THR | E | 197 | 42.223 | 43.032 | 50.080 | 1.00 | 19.94 | O |
| ATOM | 8153 | CB | THR | E | 197 | 44.423 | 41.611 | 51.865 | 1.00 | 21.70 | C |
| ATOM | 8154 | OG1 | THR | E | 197 | 45.062 | 41.480 | 53.141 | 1.00 | 22.85 | O |
| ATOM | 8155 | CG2 | THR | E | 197 | 45.450 | 41.229 | 50.758 | 1.00 | 21.88 | C |
| ATOM | 8156 | N | HIS | E | 198 | 44.097 | 44.120 | 49.538 | 1.00 | 20.49 | N |
| ATOM | 8157 | CA | HIS | E | 198 | 43.604 | 44.647 | 48.290 | 1.00 | 20.63 | C |
| ATOM | 8158 | C | HIS | E | 198 | 44.807 | 44.663 | 47.367 | 1.00 | 20.87 | C |
| ATOM | 8159 | O | HIS | E | 198 | 45.951 | 44.713 | 47.831 | 1.00 | 20.37 | O |
| ATOM | 8160 | CB | HIS | E | 198 | 43.029 | 46.066 | 48.477 | 1.00 | 20.32 | C |
| ATOM | 8161 | CG | HIS | E | 198 | 42.333 | 46.593 | 47.259 | 1.00 | 20.11 | C |
| ATOM | 8162 | ND1 | HIS | E | 198 | 42.898 | 47.537 | 46.439 | 1.00 | 21.15 | N |
| ATOM | 8163 | CD2 | HIS | E | 198 | 41.148 | 46.259 | 46.689 | 1.00 | 20.00 | C |
| ATOM | 8164 | CE1 | HIS | E | 198 | 42.074 | 47.792 | 45.435 | 1.00 | 21.09 | C |
| ATOM | 8165 | NE2 | HIS | E | 198 | 41.012 | 47.019 | 45.558 | 1.00 | 20.56 | N |
| ATOM | 8166 | N | LYS | E | 199 | 44.554 | 44.578 | 46.075 | 1.00 | 23.09 | N |
| ATOM | 8167 | CA | LYS | E | 199 | 45.634 | 44.409 | 45.115 | 1.00 | 23.75 | C |
| ATOM | 8168 | C | LYS | E | 199 | 46.570 | 45.625 | 45.057 | 1.00 | 24.50 | C |
| ATOM | 8169 | O | LYS | E | 199 | 47.698 | 45.547 | 44.529 | 1.00 | 24.67 | O |
| ATOM | 8170 | CB | LYS | E | 199 | 45.076 | 44.052 | 43.735 | 1.00 | 24.78 | C |
| ATOM | 8171 | CG | LYS | E | 199 | 44.320 | 45.165 | 43.002 | 1.00 | 25.79 | C |
| ATOM | 8172 | CD | LYS | E | 199 | 44.230 | 44.903 | 41.515 | 1.00 | 25.92 | C |
| ATOM | 8173 | CE | LYS | E | 199 | 45.537 | 45.173 | 40.798 | 1.00 | 27.27 | C |
| ATOM | 8174 | NZ | LYS | E | 199 | 45.451 | 44.853 | 39.347 | 1.00 | 29.22 | N |
| ATOM | 8175 | N | THR | E | 200 | 46.118 | 46.746 | 45.605 | 1.00 | 24.11 | N |
| ATOM | 8176 | CA | THR | E | 200 | 46.922 | 47.966 | 45.628 | 1.00 | 24.06 | C |
| ATOM | 8177 | C | THR | E | 200 | 48.127 | 47.969 | 46.599 | 1.00 | 25.01 | C |
| ATOM | 8178 | O | THR | E | 200 | 48.918 | 48.950 | 46.638 | 1.00 | 23.97 | O |

633

| ATOM | 8179 | CB | THR | E | 200 | 46.034 | 49.139 | 45.967 | 1.00 | 23.81 | C |
| ATOM | 8180 | OG1 | THR | E | 200 | 45.220 | 48.786 | 47.091 | 1.00 | 21.27 | O |
| ATOM | 8181 | CG2 | THR | E | 200 | 45.149 | 49.476 | 44.784 | 1.00 | 21.62 | C |
| ATOM | 8182 | N | SER | E | 201 | 48.250 | 46.924 | 47.407 | 1.00 | 23.89 | N |
| ATOM | 8183 | CA | SER | E | 201 | 49.387 | 46.810 | 48.283 | 1.00 | 25.72 | C |
| ATOM | 8184 | C | SER | E | 201 | 49.618 | 45.379 | 48.656 | 1.00 | 25.93 | C |
| ATOM | 8185 | O | SER | E | 201 | 48.691 | 44.574 | 48.796 | 1.00 | 25.79 | O |
| ATOM | 8186 | CB | SER | E | 201 | 49.252 | 47.648 | 49.549 | 1.00 | 26.61 | C |
| ATOM | 8187 | OG | SER | E | 201 | 50.318 | 47.333 | 50.452 | 1.00 | 31.67 | O |
| ATOM | 8188 | N | THR | E | 202 | 50.922 | 45.044 | 48.833 | 1.00 | 27.14 | N |
| ATOM | 8189 | CA | THR | E | 202 | 51.353 | 43.711 | 49.222 | 1.00 | 28.07 | C |
| ATOM | 8190 | C | THR | E | 202 | 51.160 | 43.528 | 50.702 | 1.00 | 28.56 | C |
| ATOM | 8191 | O | THR | E | 202 | 51.105 | 42.396 | 51.192 | 1.00 | 30.55 | O |
| ATOM | 8192 | CB | THR | E | 202 | 52.843 | 43.498 | 48.876 | 1.00 | 28.49 | C |
| ATOM | 8193 | OG1 | THR | E | 202 | 53.638 | 44.614 | 49.363 | 1.00 | 28.86 | O |
| ATOM | 8194 | CG2 | THR | E | 202 | 52.983 | 43.393 | 47.371 | 1.00 | 29.80 | C |
| ATOM | 8195 | N | SER | E | 203 | 51.083 | 44.659 | 51.398 | 1.00 | 27.07 | N |
| ATOM | 8196 | CA | SER | E | 203 | 50.834 | 44.669 | 52.818 | 1.00 | 27.44 | C |
| ATOM | 8197 | C | SER | E | 203 | 49.370 | 45.019 | 53.094 | 1.00 | 26.57 | C |
| ATOM | 8198 | O | SER | E | 203 | 48.783 | 45.827 | 52.369 | 1.00 | 25.97 | O |
| ATOM | 8199 | CB | SER | E | 203 | 51.717 | 45.745 | 53.458 | 1.00 | 29.09 | C |
| ATOM | 8200 | OG | SER | E | 203 | 53.069 | 45.534 | 53.089 | 1.00 | 32.26 | O |
| ATOM | 8201 | N | PRO | E | 204 | 48.781 | 44.406 | 54.141 | 1.00 | 26.18 | N |
| ATOM | 8202 | CA | PRO | E | 204 | 47.498 | 44.819 | 54.696 | 1.00 | 25.11 | C |
| ATOM | 8203 | C | PRO | E | 204 | 47.441 | 46.266 | 55.132 | 1.00 | 24.65 | C |
| ATOM | 8204 | O | PRO | E | 204 | 48.420 | 46.807 | 55.651 | 1.00 | 23.19 | O |
| ATOM | 8205 | CB | PRO | E | 204 | 47.355 | 43.942 | 55.946 | 1.00 | 26.17 | C |
| ATOM | 8206 | CG | PRO | E | 204 | 48.147 | 42.753 | 55.673 | 1.00 | 25.76 | C |
| ATOM | 8207 | CD | PRO | E | 204 | 49.298 | 43.200 | 54.823 | 1.00 | 25.80 | C |
| ATOM | 8208 | N | ILE | E | 205 | 46.285 | 46.882 | 54.917 | 1.00 | 22.56 | N |
| ATOM | 8209 | CA | ILE | E | 205 | 45.982 | 48.152 | 55.557 | 1.00 | 22.20 | C |
| ATOM | 8210 | C | ILE | E | 205 | 45.209 | 47.832 | 56.838 | 1.00 | 20.05 | C |
| ATOM | 8211 | O | ILE | E | 205 | 44.141 | 47.229 | 56.788 | 1.00 | 18.98 | O |
| ATOM | 8212 | CB | ILE | E | 205 | 45.149 | 49.075 | 54.676 | 1.00 | 22.76 | C |
| ATOM | 8213 | CG1 | ILE | E | 205 | 45.844 | 49.272 | 53.338 | 1.00 | 23.94 | C |
| ATOM | 8214 | CG2 | ILE | E | 205 | 44.964 | 50.431 | 55.381 | 1.00 | 22.57 | C |
| ATOM | 8215 | CD1 | ILE | E | 205 | 45.249 | 50.435 | 52.523 | 1.00 | 24.25 | C |
| ATOM | 8216 | N | VAL | E | 206 | 45.777 | 48.227 | 57.969 | 1.00 | 19.75 | N |
| ATOM | 8217 | CA | VAL | E | 206 | 45.277 | 47.804 | 59.274 | 1.00 | 19.61 | C |
| ATOM | 8218 | C | VAL | E | 206 | 44.799 | 48.951 | 60.135 | 1.00 | 19.36 | C |
| ATOM | 8219 | O | VAL | E | 206 | 45.436 | 50.013 | 60.215 | 1.00 | 18.85 | O |
| ATOM | 8220 | CB | VAL | E | 206 | 46.387 | 47.014 | 59.993 | 1.00 | 20.23 | C |
| ATOM | 8221 | CG1 | VAL | E | 206 | 45.921 | 46.497 | 61.368 | 1.00 | 20.66 | C |
| ATOM | 8222 | CG2 | VAL | E | 206 | 46.846 | 45.905 | 59.105 | 1.00 | 20.39 | C |
| ATOM | 8223 | N | LYS | E | 207 | 43.684 | 48.726 | 60.829 | 1.00 | 18.81 | N |
| ATOM | 8224 | CA | LYS | E | 207 | 43.237 | 49.623 | 61.871 | 1.00 | 19.77 | C |
| ATOM | 8225 | C | LYS | E | 207 | 42.880 | 48.794 | 63.110 | 1.00 | 18.89 | C |
| ATOM | 8226 | O | LYS | E | 207 | 42.377 | 47.679 | 63.006 | 1.00 | 18.86 | O |
| ATOM | 8227 | CB | LYS | E | 207 | 42.088 | 50.508 | 61.392 | 1.00 | 20.82 | C |
| ATOM | 8228 | CG | LYS | E | 207 | 42.445 | 51.456 | 60.230 | 1.00 | 21.52 | C |
| ATOM | 8229 | CD | LYS | E | 207 | 43.356 | 52.573 | 60.663 | 1.00 | 23.10 | C |
| ATOM | 8230 | CE | LYS | E | 207 | 43.776 | 53.443 | 59.498 | 1.00 | 23.15 | C |
| ATOM | 8231 | NZ | LYS | E | 207 | 44.480 | 54.659 | 59.999 | 1.00 | 25.21 | N |
| ATOM | 8232 | N | SER | E | 208 | 43.258 | 49.308 | 64.263 | 1.00 | 19.35 | N |
| ATOM | 8233 | CA | SER | E | 208 | 43.029 | 48.641 | 65.555 | 1.00 | 20.62 | C |
| ATOM | 8234 | C | SER | E | 208 | 42.469 | 49.637 | 66.547 | 1.00 | 23.17 | C |
| ATOM | 8235 | O | SER | E | 208 | 42.564 | 50.842 | 66.362 | 1.00 | 21.77 | O |
| ATOM | 8236 | CB | SER | E | 208 | 44.354 | 48.095 | 66.148 | 1.00 | 21.63 | C |
| ATOM | 8237 | OG | SER | E | 208 | 44.320 | 46.665 | 66.276 | 1.00 | 25.26 | O |
| ATOM | 8238 | N | PHE | E | 209 | 41.883 | 49.111 | 67.612 | 1.00 | 26.46 | N |
| ATOM | 8239 | CA | PHE | E | 209 | 41.812 | 49.833 | 68.881 | 1.00 | 29.13 | C |
| ATOM | 8240 | C | PHE | E | 209 | 41.925 | 48.852 | 70.041 | 1.00 | 29.45 | C |
| ATOM | 8241 | O | PHE | E | 209 | 41.604 | 47.664 | 69.898 | 1.00 | 29.91 | O |
| ATOM | 8242 | CB | PHE | E | 209 | 40.516 | 50.635 | 69.009 | 1.00 | 29.86 | C |
| ATOM | 8243 | CG | PHE | E | 209 | 39.323 | 49.785 | 69.100 | 1.00 | 30.09 | C |

```
ATOM   8244  CD1 PHE E 209      38.802  49.413  70.332  1.00 30.38           C
ATOM   8245  CD2 PHE E 209      38.750  49.305  67.956  1.00 30.12           C
ATOM   8246  CE1 PHE E 209      37.717  48.604  70.414  1.00 30.42           C
ATOM   8247  CE2 PHE E 209      37.667  48.497  68.014  1.00 30.75           C
ATOM   8248  CZ  PHE E 209      37.129  48.134  69.258  1.00 30.73           C
ATOM   8249  N   ASN E 210      42.405  49.379  71.169  1.00 30.75           N
ATOM   8250  CA  ASN E 210      42.457  48.700  72.442  1.00 30.47           C
ATOM   8251  C   ASN E 210      41.286  49.170  73.275  1.00 29.85           C
ATOM   8252  O   ASN E 210      41.027  50.372  73.400  1.00 28.99           O
ATOM   8253  CB  ASN E 210      43.763  49.007  73.193  1.00 31.16           C
ATOM   8254  CG  ASN E 210      44.167  47.890  74.195  1.00 33.41           C
ATOM   8255  OD1 ASN E 210      43.377  47.452  75.062  1.00 35.93           O
ATOM   8256  ND2 ASN E 210      45.426  47.466  74.101  1.00 33.90           N
ATOM   8257  N   ARG E 211      40.561  48.206  73.819  1.00 29.99           N
ATOM   8258  CA  ARG E 211      39.496  48.469  74.761  1.00 30.04           C
ATOM   8259  C   ARG E 211      40.136  48.965  76.051  1.00 30.50           C
ATOM   8260  O   ARG E 211      39.963  50.105  76.442  1.00 31.81           O
ATOM   8261  CB  ARG E 211      38.717  47.173  75.033  1.00 28.62           C
ATOM   8262  CG  ARG E 211      37.475  47.392  75.880  1.00 30.63           C
ATOM   8263  CD  ARG E 211      36.757  46.094  76.216  1.00 29.56           C
ATOM   8264  NE  ARG E 211      37.669  45.149  76.819  1.00 30.99           N
ATOM   8265  CZ  ARG E 211      38.048  45.178  78.085  1.00 29.55           C
ATOM   8266  NH1 ARG E 211      37.552  46.069  78.929  1.00 29.49           N
ATOM   8267  NH2 ARG E 211      38.898  44.269  78.523  1.00 32.91           N
TER    8268      ARG E 211
ATOM   8269  N   GLU F   1       5.772  68.313  37.117  1.00 28.40           N
ATOM   8270  CA  GLU F   1       6.297  66.924  36.909  1.00 27.19           C
ATOM   8271  C   GLU F   1       5.324  65.874  37.387  1.00 24.61           C
ATOM   8272  O   GLU F   1       4.519  66.120  38.277  1.00 23.65           O
ATOM   8273  CB  GLU F   1       7.669  66.720  37.582  1.00 29.70           C
ATOM   8274  CG  GLU F   1       8.825  66.601  36.558  1.00 33.94           C
ATOM   8275  CD  GLU F   1       8.787  65.303  35.723  1.00 36.78           C
ATOM   8276  OE1 GLU F   1       7.683  64.878  35.250  1.00 36.18           O
ATOM   8277  OE2 GLU F   1       9.884  64.709  35.538  1.00 40.70           O
ATOM   8278  N   VAL F   2       5.401  64.695  36.773  1.00 23.12           N
ATOM   8279  CA  VAL F   2       4.551  63.584  37.153  1.00 23.17           C
ATOM   8280  C   VAL F   2       5.120  63.021  38.462  1.00 22.15           C
ATOM   8281  O   VAL F   2       6.333  62.816  38.578  1.00 21.14           O
ATOM   8282  CB  VAL F   2       4.491  62.492  36.015  1.00 20.75           C
ATOM   8283  CG1 VAL F   2       3.623  61.336  36.415  1.00 21.87           C
ATOM   8284  CG2 VAL F   2       3.970  63.087  34.710  1.00 20.46           C
ATOM   8285  N   LYS F   3       4.246  62.790  39.442  1.00 22.64           N
ATOM   8286  CA  LYS F   3       4.595  62.158  40.699  1.00 22.96           C
ATOM   8287  C   LYS F   3       3.708  60.951  40.920  1.00 21.48           C
ATOM   8288  O   LYS F   3       2.501  61.072  40.938  1.00 20.19           O
ATOM   8289  CB  LYS F   3       4.394  63.108  41.863  1.00 25.10           C
ATOM   8290  CG  LYS F   3       5.219  64.392  41.787  1.00 27.65           C
ATOM   8291  CD  LYS F   3       4.714  65.432  42.797  1.00 28.18           C
ATOM   8292  CE  LYS F   3       3.913  66.566  42.153  1.00 30.14           C
ATOM   8293  NZ  LYS F   3       2.965  66.160  41.034  1.00 30.77           N
ATOM   8294  N   VAL F   4       4.332  59.815  41.086  1.00 20.53           N
ATOM   8295  CA  VAL F   4       3.622  58.579  41.363  1.00 19.36           C
ATOM   8296  C   VAL F   4       4.090  58.060  42.745  1.00 17.03           C
ATOM   8297  O   VAL F   4       5.270  57.845  42.932  1.00 15.04           O
ATOM   8298  CB  VAL F   4       3.931  57.541  40.265  1.00 19.48           C
ATOM   8299  CG1 VAL F   4       3.205  56.244  40.552  1.00 19.17           C
ATOM   8300  CG2 VAL F   4       3.552  58.083  38.898  1.00 20.60           C
ATOM   8301  N   GLU F   5       3.169  57.874  43.696  1.00 17.49           N
ATOM   8302  CA  GLU F   5       3.526  57.727  45.111  1.00 18.79           C
ATOM   8303  C   GLU F   5       2.764  56.568  45.747  1.00 18.12           C
ATOM   8304  O   GLU F   5       1.563  56.654  45.973  1.00 17.27           O
ATOM   8305  CB  GLU F   5       3.280  59.044  45.868  1.00 20.50           C
ATOM   8306  CG  GLU F   5       3.810  59.110  47.293  1.00 22.53           C
ATOM   8307  CD  GLU F   5       3.740  60.545  47.863  1.00 25.66           C
ATOM   8308  OE1 GLU F   5       2.879  60.827  48.733  1.00 31.76           O
```

| ATOM | 8309 | OE2 | GLU | F | 5 | 4.533 | 61.402 | 47.401 | 1.00 | 31.15 | O |
|------|------|-----|-----|---|----|--------|--------|--------|------|-------|---|
| ATOM | 8310 | N   | GLU | F | 6 | 3.461 | 55.442 | 45.980 | 1.00 | 17.29 | N |
| ATOM | 8311 | CA  | GLU | F | 6 | 2.821 | 54.251 | 46.557 | 1.00 | 17.26 | C |
| ATOM | 8312 | C   | GLU | F | 6 | 2.789 | 54.302 | 48.065 | 1.00 | 17.90 | C |
| ATOM | 8313 | O   | GLU | F | 6 | 3.625 | 54.941 | 48.696 | 1.00 | 18.90 | O |
| ATOM | 8314 | CB  | GLU | F | 6 | 3.538 | 52.954 | 46.159 | 1.00 | 15.46 | C |
| ATOM | 8315 | CG  | GLU | F | 6 | 3.507 | 52.675 | 44.673 | 1.00 | 16.61 | C |
| ATOM | 8316 | CD  | GLU | F | 6 | 4.686 | 53.288 | 43.982 | 1.00 | 15.24 | C |
| ATOM | 8317 | OE1 | GLU | F | 6 | 5.272 | 54.204 | 44.559 | 1.00 | 15.29 | O |
| ATOM | 8318 | OE2 | GLU | F | 6 | 5.075 | 52.820 | 42.903 | 1.00 | 16.85 | O |
| ATOM | 8319 | N   | SER | F | 7 | 1.834 | 53.585 | 48.636 | 1.00 | 19.68 | N |
| ATOM | 8320 | CA  | SER | F | 7 | 1.764 | 53.419 | 50.094 | 1.00 | 19.14 | C |
| ATOM | 8321 | C   | SER | F | 7 | 1.017 | 52.165 | 50.435 | 1.00 | 17.77 | C |
| ATOM | 8322 | O   | SER | F | 7 | 0.431 | 51.531 | 49.570 | 1.00 | 16.90 | O |
| ATOM | 8323 | CB  | SER | F | 7 | 1.098 | 54.635 | 50.766 | 1.00 | 20.13 | C |
| ATOM | 8324 | OG  | SER | F | 7 | -0.233 | 54.800 | 50.312 | 1.00 | 22.41 | O |
| ATOM | 8325 | N   | GLY | F | 8 | 1.060 | 51.804 | 51.712 | 1.00 | 17.47 | N |
| ATOM | 8326 | CA  | GLY | F | 8 | 0.264 | 50.714 | 52.243 | 1.00 | 16.33 | C |
| ATOM | 8327 | C   | GLY | F | 8 | 0.996 | 49.396 | 52.422 | 1.00 | 15.79 | C |
| ATOM | 8328 | O   | GLY | F | 8 | 0.379 | 48.415 | 52.809 | 1.00 | 18.70 | O |
| ATOM | 8329 | N   | GLY | F | 9 | 2.301 | 49.370 | 52.151 | 1.00 | 16.03 | N |
| ATOM | 8330 | CA  | GLY | F | 9 | 3.089 | 48.144 | 52.384 | 1.00 | 14.27 | C |
| ATOM | 8331 | C   | GLY | F | 9 | 3.283 | 47.841 | 53.850 | 1.00 | 14.71 | C |
| ATOM | 8332 | O   | GLY | F | 9 | 2.879 | 48.609 | 54.727 | 1.00 | 13.07 | O |
| ATOM | 8333 | N   | GLY | F | 10 | 3.900 | 46.700 | 54.151 | 1.00 | 10.96 | N |
| ATOM | 8334 | CA  | GLY | F | 10 | 4.210 | 46.350 | 55.505 | 1.00 | 9.05 | C |
| ATOM | 8335 | C   | GLY | F | 10 | 4.393 | 44.858 | 55.498 | 1.00 | 9.95 | C |
| ATOM | 8336 | O   | GLY | F | 10 | 4.816 | 44.342 | 54.493 | 1.00 | 8.99 | O |
| ATOM | 8337 | N   | LEU | F | 11 | 4.014 | 44.211 | 56.582 | 1.00 | 9.32 | N |
| ATOM | 8338 | CA  | LEU | F | 11 | 4.195 | 42.747 | 56.712 | 1.00 | 9.92 | C |
| ATOM | 8339 | C   | LEU | F | 11 | 2.845 | 42.081 | 56.957 | 1.00 | 10.10 | C |
| ATOM | 8340 | O   | LEU | F | 11 | 1.973 | 42.593 | 57.646 | 1.00 | 9.79 | O |
| ATOM | 8341 | CB  | LEU | F | 11 | 5.164 | 42.446 | 57.850 | 1.00 | 8.93 | C |
| ATOM | 8342 | CG  | LEU | F | 11 | 5.511 | 40.937 | 58.122 | 1.00 | 10.62 | C |
| ATOM | 8343 | CD1 | LEU | F | 11 | 6.947 | 40.842 | 58.551 | 1.00 | 10.50 | C |
| ATOM | 8344 | CD2 | LEU | F | 11 | 4.591 | 40.245 | 59.154 | 1.00 | 14.05 | C |
| ATOM | 8345 | N   | VAL | F | 12 | 2.667 | 40.871 | 56.418 | 1.00 | 9.06 | N |
| ATOM | 8346 | CA  | VAL | F | 12 | 1.555 | 40.096 | 56.847 | 1.00 | 9.22 | C |
| ATOM | 8347 | C   | VAL | F | 12 | 2.066 | 38.655 | 56.748 | 1.00 | 9.62 | C |
| ATOM | 8348 | O   | VAL | F | 12 | 3.074 | 38.388 | 56.086 | 1.00 | 9.92 | O |
| ATOM | 8349 | CB  | VAL | F | 12 | 0.255 | 40.317 | 55.973 | 1.00 | 10.70 | C |
| ATOM | 8350 | CG1 | VAL | F | 12 | 0.430 | 39.810 | 54.507 | 1.00 | 11.88 | C |
| ATOM | 8351 | CG2 | VAL | F | 12 | -0.983 | 39.721 | 56.649 | 1.00 | 9.77 | C |
| ATOM | 8352 | N   | GLN | F | 13 | 1.346 | 37.788 | 57.418 | 1.00 | 11.62 | N |
| ATOM | 8353 | CA  | GLN | F | 13 | 1.616 | 36.352 | 57.458 | 1.00 | 12.25 | C |
| ATOM | 8354 | C   | GLN | F | 13 | 1.107 | 35.622 | 56.215 | 1.00 | 12.75 | C |
| ATOM | 8355 | O   | GLN | F | 13 | 0.110 | 35.993 | 55.593 | 1.00 | 12.86 | O |
| ATOM | 8356 | CB  | GLN | F | 13 | 0.936 | 35.767 | 58.677 | 1.00 | 12.78 | C |
| ATOM | 8357 | CG  | GLN | F | 13 | 1.334 | 36.308 | 60.062 | 1.00 | 14.60 | C |
| ATOM | 8358 | CD  | GLN | F | 13 | 0.876 | 37.726 | 60.321 | 1.00 | 14.35 | C |
| ATOM | 8359 | OE1 | GLN | F | 13 | -0.253 | 38.108 | 60.013 | 1.00 | 15.40 | O |
| ATOM | 8360 | NE2 | GLN | F | 13 | 1.757 | 38.514 | 60.875 | 1.00 | 14.55 | N |
| ATOM | 8361 | N   | PRO | F | 14 | 1.776 | 34.519 | 55.838 | 1.00 | 13.41 | N |
| ATOM | 8362 | CA  | PRO | F | 14 | 1.238 | 33.742 | 54.745 | 1.00 | 13.98 | C |
| ATOM | 8363 | C   | PRO | F | 14 | -0.232 | 33.389 | 54.942 | 1.00 | 14.33 | C |
| ATOM | 8364 | O   | PRO | F | 14 | -0.646 | 32.988 | 56.040 | 1.00 | 15.00 | O |
| ATOM | 8365 | CB  | PRO | F | 14 | 2.074 | 32.460 | 54.791 | 1.00 | 14.00 | C |
| ATOM | 8366 | CG  | PRO | F | 14 | 3.348 | 32.830 | 55.414 | 1.00 | 14.40 | C |
| ATOM | 8367 | CD  | PRO | F | 14 | 3.022 | 33.945 | 56.381 | 1.00 | 14.06 | C |
| ATOM | 8368 | N   | GLY | F | 15 | -0.990 | 33.486 | 53.856 | 1.00 | 15.47 | N |
| ATOM | 8369 | CA  | GLY | F | 15 | -2.413 | 33.252 | 53.858 | 1.00 | 15.22 | C |
| ATOM | 8370 | C   | GLY | F | 15 | -3.217 | 34.502 | 54.146 | 1.00 | 15.81 | C |
| ATOM | 8371 | O   | GLY | F | 15 | -4.428 | 34.480 | 54.068 | 1.00 | 17.15 | O |
| ATOM | 8372 | N   | GLY | F | 16 | -2.526 | 35.608 | 54.442 | 1.00 | 16.33 | N |
| ATOM | 8373 | CA  | GLY | F | 16 | -3.141 | 36.840 | 54.878 | 1.00 | 14.91 | C |

```
ATOM   8374  C    GLY F   16     -3.246  37.799  53.718  1.00  14.80           C
ATOM   8375  O    GLY F   16     -2.996  37.432  52.588  1.00  12.95           O
ATOM   8376  N    SER F   17     -3.607  39.046  54.005  1.00  15.30           N
ATOM   8377  CA   SER F   17     -3.987  39.980  52.972  1.00  15.21           C
ATOM   8378  C    SER F   17     -3.346  41.324  53.177  1.00  15.58           C
ATOM   8379  O    SER F   17     -2.944  41.665  54.280  1.00  14.91           O
ATOM   8380  CB   SER F   17     -5.505  40.227  52.996  1.00  16.70           C
ATOM   8381  OG   SER F   17     -6.235  39.213  52.371  1.00  20.60           O
ATOM   8382  N    MET F   18     -3.316  42.097  52.095  1.00  15.99           N
ATOM   8383  CA   MET F   18     -2.760  43.444  52.093  1.00  17.38           C
ATOM   8384  C    MET F   18     -3.550  44.297  51.110  1.00  17.66           C
ATOM   8385  O    MET F   18     -4.141  43.776  50.194  1.00  19.36           O
ATOM   8386  CB   MET F   18     -1.311  43.332  51.636  1.00  20.60           C
ATOM   8387  CG   MET F   18     -0.371  44.205  52.313  1.00  25.29           C
ATOM   8388  SD   MET F   18      0.116  43.711  53.988  1.00  26.24           S
ATOM   8389  CE   MET F   18      1.423  44.907  53.948  1.00  24.55           C
ATOM   8390  N    LYS F   19     -3.555  45.615  51.272  1.00  18.61           N
ATOM   8391  CA   LYS F   19     -4.119  46.495  50.243  1.00  18.18           C
ATOM   8392  C    LYS F   19     -3.189  47.681  50.109  1.00  17.73           C
ATOM   8393  O    LYS F   19     -2.988  48.422  51.078  1.00  18.43           O
ATOM   8394  CB   LYS F   19     -5.546  46.955  50.591  1.00  19.73           C
ATOM   8395  CG   LYS F   19     -6.219  47.709  49.418  1.00  21.03           C
ATOM   8396  CD   LYS F   19     -7.755  47.838  49.512  1.00  21.82           C
ATOM   8397  CE   LYS F   19     -8.357  48.135  48.128  1.00  24.32           C
ATOM   8398  NZ   LYS F   19     -9.793  48.654  48.185  1.00  24.37           N
ATOM   8399  N    ILE F   20     -2.561  47.811  48.935  1.00  15.97           N
ATOM   8400  CA   ILE F   20     -1.644  48.911  48.649  1.00  15.73           C
ATOM   8401  C    ILE F   20     -2.280  49.858  47.656  1.00  15.19           C
ATOM   8402  O    ILE F   20     -3.197  49.491  46.947  1.00  13.37           O
ATOM   8403  CB   ILE F   20     -0.250  48.380  48.154  1.00  15.88           C
ATOM   8404  CG1  ILE F   20     -0.376  47.563  46.865  1.00  15.18           C
ATOM   8405  CG2  ILE F   20      0.400  47.543  49.266  1.00  15.93           C
ATOM   8406  CD1  ILE F   20      0.936  47.145  46.253  1.00  15.85           C
ATOM   8407  N    SER F   21     -1.836  51.117  47.654  1.00  14.92           N
ATOM   8408  CA   SER F   21     -2.397  52.069  46.748  1.00  16.09           C
ATOM   8409  C    SER F   21     -1.284  52.960  46.217  1.00  16.36           C
ATOM   8410  O    SER F   21     -0.163  52.947  46.708  1.00  15.19           O
ATOM   8411  CB   SER F   21     -3.437  52.939  47.463  1.00  15.20           C
ATOM   8412  OG   SER F   21     -2.846  53.579  48.575  1.00  19.49           O
ATOM   8413  N    CYS F   22     -1.601  53.730  45.183  1.00  17.60           N
ATOM   8414  CA   CYS F   22     -0.741  54.841  44.813  1.00  18.20           C
ATOM   8415  C    CYS F   22     -1.564  55.961  44.274  1.00  18.44           C
ATOM   8416  O    CYS F   22     -2.634  55.741  43.755  1.00  18.14           O
ATOM   8417  CB   CYS F   22      0.333  54.449  43.819  1.00  21.21           C
ATOM   8418  SG   CYS F   22     -0.172  54.083  42.164  1.00  26.10           S
ATOM   8419  N    VAL F   23     -1.040  57.155  44.452  1.00  17.92           N
ATOM   8420  CA   VAL F   23     -1.704  58.355  43.988  1.00  20.13           C
ATOM   8421  C    VAL F   23     -0.787  58.987  42.965  1.00  18.86           C
ATOM   8422  O    VAL F   23      0.432  58.956  43.115  1.00  19.29           O
ATOM   8423  CB   VAL F   23     -2.064  59.314  45.147  1.00  19.91           C
ATOM   8424  CG1  VAL F   23     -0.852  59.703  45.969  1.00  22.81           C
ATOM   8425  CG2  VAL F   23     -2.751  60.564  44.598  1.00  20.41           C
ATOM   8426  N    VAL F   24     -1.390  59.512  41.903  1.00  19.76           N
ATOM   8427  CA   VAL F   24     -0.665  60.199  40.836  1.00  20.40           C
ATOM   8428  C    VAL F   24     -1.055  61.680  40.717  1.00  21.61           C
ATOM   8429  O    VAL F   24     -2.243  62.028  40.730  1.00  23.60           O
ATOM   8430  CB   VAL F   24     -0.919  59.530  39.492  1.00  21.37           C
ATOM   8431  CG1  VAL F   24      0.058  60.025  38.448  1.00  21.17           C
ATOM   8432  CG2  VAL F   24     -0.805  58.011  39.645  1.00  20.47           C
ATOM   8433  N    SER F   25     -0.048  62.531  40.616  1.00  21.51           N
ATOM   8434  CA   SER F   25     -0.240  63.952  40.269  1.00  20.75           C
ATOM   8435  C    SER F   25      0.647  64.339  39.100  1.00  20.83           C
ATOM   8436  O    SER F   25      1.576  63.622  38.723  1.00  19.26           O
ATOM   8437  CB   SER F   25      0.075  64.837  41.459  1.00  22.14           C
ATOM   8438  OG   SER F   25      1.370  64.546  41.989  1.00  26.04           O
```

| ATOM | 8439 | N   | GLY | F | 26 | 0.375  | 65.507 | 38.521 | 1.00 | 21.73 | N |
|------|------|-----|-----|---|----|--------|--------|--------|------|-------|---|
| ATOM | 8440 | CA  | GLY | F | 26 | 1.131  | 65.981 | 37.369 | 1.00 | 21.32 | C |
| ATOM | 8441 | C   | GLY | F | 26 | 0.473  | 65.600 | 36.067 | 1.00 | 20.78 | C |
| ATOM | 8442 | O   | GLY | F | 26 | 0.891  | 66.051 | 35.001 | 1.00 | 22.44 | O |
| ATOM | 8443 | N   | LEU | F | 27 | -0.546 | 64.759 | 36.134 | 1.00 | 20.81 | N |
| ATOM | 8444 | CA  | LEU | F | 27 | -1.290 | 64.386 | 34.944 | 1.00 | 20.56 | C |
| ATOM | 8445 | C   | LEU | F | 27 | -2.711 | 63.980 | 35.301 | 1.00 | 20.78 | C |
| ATOM | 8446 | O   | LEU | F | 27 | -3.029 | 63.814 | 36.472 | 1.00 | 20.34 | O |
| ATOM | 8447 | CB  | LEU | F | 27 | -0.586 | 63.284 | 34.150 | 1.00 | 21.54 | C |
| ATOM | 8448 | CG  | LEU | F | 27 | -0.200 | 61.980 | 34.866 | 1.00 | 21.39 | C |
| ATOM | 8449 | CD1 | LEU | F | 27 | -1.410 | 61.214 | 35.343 | 1.00 | 21.47 | C |
| ATOM | 8450 | CD2 | LEU | F | 27 | 0.605  | 61.167 | 33.899 | 1.00 | 20.78 | C |
| ATOM | 8451 | N   | THR | F | 28 | -3.565 | 63.877 | 34.286 | 1.00 | 19.24 | N |
| ATOM | 8452 | CA  | THR | F | 28 | -4.949 | 63.538 | 34.491 | 1.00 | 19.12 | C |
| ATOM | 8453 | C   | THR | F | 28 | -5.040 | 62.010 | 34.561 | 1.00 | 19.31 | C |
| ATOM | 8454 | O   | THR | F | 28 | -5.109 | 61.314 | 33.554 | 1.00 | 20.71 | O |
| ATOM | 8455 | CB  | THR | F | 28 | -5.861 | 64.113 | 33.392 | 1.00 | 20.34 | C |
| ATOM | 8456 | OG1 | THR | F | 28 | -5.587 | 65.522 | 33.237 | 1.00 | 20.85 | O |
| ATOM | 8457 | CG2 | THR | F | 28 | -7.310 | 63.947 | 33.794 | 1.00 | 19.84 | C |
| ATOM | 8458 | N   | PHE | F | 29 | -5.069 | 61.537 | 35.788 | 1.00 | 17.60 | N |
| ATOM | 8459 | CA  | PHE | F | 29 | -5.017 | 60.096 | 36.119 | 1.00 | 17.11 | C |
| ATOM | 8460 | C   | PHE | F | 29 | -5.981 | 59.233 | 35.319 | 1.00 | 18.48 | C |
| ATOM | 8461 | O   | PHE | F | 29 | -5.644 | 58.105 | 34.886 | 1.00 | 15.72 | O |
| ATOM | 8462 | CB  | PHE | F | 29 | -5.251 | 59.983 | 37.620 | 1.00 | 16.30 | C |
| ATOM | 8463 | CG  | PHE | F | 29 | -5.349 | 58.560 | 38.131 | 1.00 | 17.66 | C |
| ATOM | 8464 | CD1 | PHE | F | 29 | -4.236 | 57.922 | 38.620 | 1.00 | 16.92 | C |
| ATOM | 8465 | CD2 | PHE | F | 29 | -6.548 | 57.900 | 38.116 | 1.00 | 15.71 | C |
| ATOM | 8466 | CE1 | PHE | F | 29 | -4.321 | 56.607 | 39.102 | 1.00 | 16.90 | C |
| ATOM | 8467 | CE2 | PHE | F | 29 | -6.641 | 56.609 | 38.611 | 1.00 | 16.42 | C |
| ATOM | 8468 | CZ  | PHE | F | 29 | -5.511 | 55.967 | 39.082 | 1.00 | 15.97 | C |
| ATOM | 8469 | N   | SER | F | 30 | -7.189 | 59.736 | 35.081 | 1.00 | 17.81 | N |
| ATOM | 8470 | CA  | SER | F | 30 | -8.206 | 58.963 | 34.349 | 1.00 | 17.15 | C |
| ATOM | 8471 | C   | SER | F | 30 | -7.867 | 58.714 | 32.877 | 1.00 | 16.85 | C |
| ATOM | 8472 | O   | SER | F | 30 | -8.492 | 57.859 | 32.226 | 1.00 | 16.27 | O |
| ATOM | 8473 | CB  | SER | F | 30 | -9.572 | 59.664 | 34.438 | 1.00 | 18.92 | C |
| ATOM | 8474 | OG  | SER | F | 30 | -9.466 | 60.947 | 33.833 | 1.00 | 18.58 | O |
| ATOM | 8475 | N   | ASN | F | 31 | -6.892 | 59.448 | 32.340 | 1.00 | 15.98 | N |
| ATOM | 8476 | CA  | ASN | F | 31 | -6.549 | 59.306 | 30.954 | 1.00 | 17.79 | C |
| ATOM | 8477 | C   | ASN | F | 31 | -5.554 | 58.157 | 30.685 | 1.00 | 16.75 | C |
| ATOM | 8478 | O   | ASN | F | 31 | -5.340 | 57.800 | 29.523 | 1.00 | 16.32 | O |
| ATOM | 8479 | CB  | ASN | F | 31 | -5.947 | 60.594 | 30.411 | 1.00 | 20.06 | C |
| ATOM | 8480 | CG  | ASN | F | 31 | -6.917 | 61.780 | 30.414 | 1.00 | 23.88 | C |
| ATOM | 8481 | OD1 | ASN | F | 31 | -6.571 | 62.842 | 29.887 | 1.00 | 26.20 | O |
| ATOM | 8482 | ND2 | ASN | F | 31 | -8.105 | 61.618 | 31.005 | 1.00 | 24.01 | N |
| ATOM | 8483 | N   | TYR | F | 32 | -4.977 | 57.579 | 31.737 | 1.00 | 17.10 | N |
| ATOM | 8484 | CA  | TYR | F | 32 | -3.836 | 56.658 | 31.595 | 1.00 | 16.80 | C |
| ATOM | 8485 | C   | TYR | F | 32 | -4.106 | 55.259 | 32.114 | 1.00 | 15.01 | C |
| ATOM | 8486 | O   | TYR | F | 32 | -4.712 | 55.082 | 33.155 | 1.00 | 16.36 | O |
| ATOM | 8487 | CB  | TYR | F | 32 | -2.609 | 57.220 | 32.328 | 1.00 | 17.87 | C |
| ATOM | 8488 | CG  | TYR | F | 32 | -2.056 | 58.496 | 31.736 | 1.00 | 19.38 | C |
| ATOM | 8489 | CD1 | TYR | F | 32 | -0.944 | 58.487 | 30.895 | 1.00 | 18.77 | C |
| ATOM | 8490 | CD2 | TYR | F | 32 | -2.675 | 59.709 | 31.982 | 1.00 | 19.46 | C |
| ATOM | 8491 | CE1 | TYR | F | 32 | -0.440 | 59.678 | 30.335 | 1.00 | 20.57 | C |
| ATOM | 8492 | CE2 | TYR | F | 32 | -2.201 | 60.882 | 31.428 | 1.00 | 20.33 | C |
| ATOM | 8493 | CZ  | TYR | F | 32 | -1.079 | 60.873 | 30.614 | 1.00 | 21.81 | C |
| ATOM | 8494 | OH  | TYR | F | 32 | -0.586 | 62.064 | 30.091 | 1.00 | 22.30 | O |
| ATOM | 8495 | N   | TRP | F | 33 | -3.637 | 54.266 | 31.374 | 1.00 | 16.74 | N |
| ATOM | 8496 | CA  | TRP | F | 33 | -3.635 | 52.910 | 31.850 | 1.00 | 15.28 | C |
| ATOM | 8497 | C   | TRP | F | 33 | -2.731 | 52.815 | 33.079 | 1.00 | 12.81 | C |
| ATOM | 8498 | O   | TRP | F | 33 | -1.691 | 53.486 | 33.187 | 1.00 | 13.58 | O |
| ATOM | 8499 | CB  | TRP | F | 33 | -3.141 | 51.951 | 30.767 | 1.00 | 14.97 | C |
| ATOM | 8500 | CG  | TRP | F | 33 | -3.880 | 51.990 | 29.478 | 1.00 | 15.89 | C |
| ATOM | 8501 | CD1 | TRP | F | 33 | -5.067 | 52.635 | 29.195 | 1.00 | 15.39 | C |
| ATOM | 8502 | CD2 | TRP | F | 33 | -3.500 | 51.301 | 28.287 | 1.00 | 15.20 | C |
| ATOM | 8503 | NE1 | TRP | F | 33 | -5.440 | 52.371 | 27.909 | 1.00 | 16.93 | N |

| ATOM | 8504 | CE2 | TRP | F | 33 | -4.493 | 51.564 | 27.316 | 1.00 | 13.44 | C |
|------|------|-----|-----|---|----|--------|--------|--------|------|-------|---|
| ATOM | 8505 | CE3 | TRP | F | 33 | -2.399 | 50.511 | 27.937 | 1.00 | 16.57 | C |
| ATOM | 8506 | CZ2 | TRP | F | 33 | -4.425 | 51.066 | 26.041 | 1.00 | 16.10 | C |
| ATOM | 8507 | CZ3 | TRP | F | 33 | -2.358 | 49.953 | 26.638 | 1.00 | 16.36 | C |
| ATOM | 8508 | CH2 | TRP | F | 33 | -3.358 | 50.245 | 25.717 | 1.00 | 15.90 | C |
| ATOM | 8509 | N | MET | F | 34 | -3.132 | 51.964 | 34.009 | 1.00 | 15.93 | N |
| ATOM | 8510 | CA | MET | F | 34 | -2.433 | 51.796 | 35.280 | 1.00 | 13.69 | C |
| ATOM | 8511 | C | MET | F | 34 | -2.084 | 50.304 | 35.498 | 1.00 | 13.47 | C |
| ATOM | 8512 | O | MET | F | 34 | -2.882 | 49.423 | 35.224 | 1.00 | 12.89 | O |
| ATOM | 8513 | CB | MET | F | 34 | -3.341 | 52.272 | 36.398 | 1.00 | 15.61 | C |
| ATOM | 8514 | CG | MET | F | 34 | -3.646 | 53.771 | 36.371 | 1.00 | 14.22 | C |
| ATOM | 8515 | SD | MET | F | 34 | -2.291 | 54.665 | 37.032 | 1.00 | 19.10 | S |
| ATOM | 8516 | CE | MET | F | 34 | -2.619 | 56.177 | 36.167 | 1.00 | 17.94 | C |
| ATOM | 8517 | N | SER | F | 35 | -0.880 | 50.045 | 35.996 | 1.00 | 13.59 | N |
| ATOM | 8518 | CA | SER | F | 35 | -0.453 | 48.632 | 36.288 | 1.00 | 12.47 | C |
| ATOM | 8519 | C | SER | F | 35 | 0.341 | 48.559 | 37.590 | 1.00 | 13.45 | C |
| ATOM | 8520 | O | SER | F | 35 | 0.780 | 49.550 | 38.135 | 1.00 | 13.03 | O |
| ATOM | 8521 | CB | SER | F | 35 | 0.380 | 48.064 | 35.123 | 1.00 | 12.01 | C |
| ATOM | 8522 | OG | SER | F | 35 | 1.716 | 48.585 | 35.071 | 1.00 | 11.53 | O |
| ATOM | 8523 | N | TRP | F | 36 | 0.496 | 47.345 | 38.101 | 1.00 | 11.46 | N |
| ATOM | 8524 | CA | TRP | F | 36 | 1.402 | 47.077 | 39.213 | 1.00 | 11.74 | C |
| ATOM | 8525 | C | TRP | F | 36 | 2.478 | 46.116 | 38.726 | 1.00 | 12.51 | C |
| ATOM | 8526 | O | TRP | F | 36 | 2.151 | 45.160 | 38.070 | 1.00 | 9.57 | O |
| ATOM | 8527 | CB | TRP | F | 36 | 0.653 | 46.434 | 40.363 | 1.00 | 11.31 | C |
| ATOM | 8528 | CG | TRP | F | 36 | -0.268 | 47.342 | 41.081 | 1.00 | 11.95 | C |
| ATOM | 8529 | CD1 | TRP | F | 36 | -1.587 | 47.457 | 40.901 | 1.00 | 11.53 | C |
| ATOM | 8530 | CD2 | TRP | F | 36 | 0.086 | 48.240 | 42.122 | 1.00 | 10.69 | C |
| ATOM | 8531 | NE1 | TRP | F | 36 | -2.108 | 48.422 | 41.744 | 1.00 | 11.81 | N |
| ATOM | 8532 | CE2 | TRP | F | 36 | -1.100 | 48.904 | 42.520 | 1.00 | 12.94 | C |
| ATOM | 8533 | CE3 | TRP | F | 36 | 1.289 | 48.536 | 42.774 | 1.00 | 9.72 | C |
| ATOM | 8534 | CZ2 | TRP | F | 36 | -1.122 | 49.864 | 43.516 | 1.00 | 11.61 | C |
| ATOM | 8535 | CZ3 | TRP | F | 36 | 1.270 | 49.506 | 43.781 | 1.00 | 12.37 | C |
| ATOM | 8536 | CH2 | TRP | F | 36 | 0.065 | 50.136 | 44.151 | 1.00 | 12.46 | C |
| ATOM | 8537 | N | VAL | F | 37 | 3.740 | 46.424 | 39.003 | 1.00 | 12.78 | N |
| ATOM | 8538 | CA | VAL | F | 37 | 4.870 | 45.557 | 38.655 | 1.00 | 11.86 | C |
| ATOM | 8539 | C | VAL | F | 37 | 5.651 | 45.368 | 39.938 | 1.00 | 12.33 | C |
| ATOM | 8540 | O | VAL | F | 37 | 5.973 | 46.333 | 40.614 | 1.00 | 11.87 | O |
| ATOM | 8541 | CB | VAL | F | 37 | 5.760 | 46.202 | 37.563 | 1.00 | 11.17 | C |
| ATOM | 8542 | CG1 | VAL | F | 37 | 7.061 | 45.407 | 37.285 | 1.00 | 13.16 | C |
| ATOM | 8543 | CG2 | VAL | F | 37 | 4.979 | 46.420 | 36.299 | 1.00 | 11.14 | C |
| ATOM | 8544 | N | ARG | F | 38 | 5.905 | 44.123 | 40.317 | 1.00 | 11.66 | N |
| ATOM | 8545 | CA | ARG | F | 38 | 6.689 | 43.875 | 41.521 | 1.00 | 13.91 | C |
| ATOM | 8546 | C | ARG | F | 38 | 8.129 | 43.432 | 41.197 | 1.00 | 12.76 | C |
| ATOM | 8547 | O | ARG | F | 38 | 8.444 | 43.029 | 40.087 | 1.00 | 11.84 | O |
| ATOM | 8548 | CB | ARG | F | 38 | 5.988 | 42.881 | 42.429 | 1.00 | 13.22 | C |
| ATOM | 8549 | CG | ARG | F | 38 | 5.989 | 41.495 | 41.928 | 1.00 | 14.93 | C |
| ATOM | 8550 | CD | ARG | F | 38 | 4.997 | 40.666 | 42.654 | 1.00 | 15.14 | C |
| ATOM | 8551 | NE | ARG | F | 38 | 4.965 | 39.305 | 42.118 | 1.00 | 16.35 | N |
| ATOM | 8552 | CZ | ARG | F | 38 | 4.216 | 38.318 | 42.589 | 1.00 | 16.75 | C |
| ATOM | 8553 | NH1 | ARG | F | 38 | 3.344 | 38.521 | 43.564 | 1.00 | 17.46 | N |
| ATOM | 8554 | NH2 | ARG | F | 38 | 4.307 | 37.107 | 42.034 | 1.00 | 17.43 | N |
| ATOM | 8555 | N | GLN | F | 39 | 9.016 | 43.589 | 42.153 | 1.00 | 14.29 | N |
| ATOM | 8556 | CA | GLN | F | 39 | 10.422 | 43.316 | 41.935 | 1.00 | 14.64 | C |
| ATOM | 8557 | C | GLN | F | 39 | 10.948 | 42.575 | 43.146 | 1.00 | 14.82 | C |
| ATOM | 8558 | O | GLN | F | 39 | 10.696 | 42.970 | 44.271 | 1.00 | 15.04 | O |
| ATOM | 8559 | CB | GLN | F | 39 | 11.202 | 44.622 | 41.749 | 1.00 | 13.65 | C |
| ATOM | 8560 | CG | GLN | F | 39 | 12.651 | 44.396 | 41.399 | 1.00 | 14.86 | C |
| ATOM | 8561 | CD | GLN | F | 39 | 13.348 | 45.634 | 40.936 | 1.00 | 15.31 | C |
| ATOM | 8562 | OE1 | GLN | F | 39 | 13.105 | 46.733 | 41.455 | 1.00 | 19.22 | O |
| ATOM | 8563 | NE2 | GLN | F | 39 | 14.247 | 45.474 | 39.978 | 1.00 | 16.68 | N |
| ATOM | 8564 | N | SER | F | 40 | 11.589 | 41.438 | 42.909 | 1.00 | 17.56 | N |
| ATOM | 8565 | CA | SER | F | 40 | 12.332 | 40.761 | 43.949 | 1.00 | 19.59 | C |
| ATOM | 8566 | C | SER | F | 40 | 13.669 | 40.308 | 43.385 | 1.00 | 22.24 | C |
| ATOM | 8567 | O | SER | F | 40 | 13.798 | 40.102 | 42.192 | 1.00 | 21.81 | O |
| ATOM | 8568 | CB | SER | F | 40 | 11.561 | 39.535 | 44.379 | 1.00 | 19.59 | C |

```
ATOM   8569  OG   SER F  40    11.318  38.778  43.230  1.00 19.66        O
ATOM   8570  N    PRO F  41    14.657  40.081  44.259  1.00 25.83        N
ATOM   8571  CA   PRO F  41    15.974  39.623  43.791  1.00 26.60        C
ATOM   8572  C    PRO F  41    15.886  38.311  43.044  1.00 28.97        C
ATOM   8573  O    PRO F  41    16.645  38.060  42.099  1.00 31.35        O
ATOM   8574  CB   PRO F  41    16.757  39.407  45.083  1.00 27.08        C
ATOM   8575  CG   PRO F  41    16.042  40.215  46.116  1.00 25.72        C
ATOM   8576  CD   PRO F  41    14.611  40.269  45.717  1.00 25.79        C
ATOM   8577  N    GLU F  42    14.932  37.505  43.462  1.00 29.95        N
ATOM   8578  CA   GLU F  42    14.755  36.164  42.958  1.00 31.09        C
ATOM   8579  C    GLU F  42    14.213  36.206  41.538  1.00 31.46        C
ATOM   8580  O    GLU F  42    14.837  35.642  40.643  1.00 32.03        O
ATOM   8581  CB   GLU F  42    13.810  35.382  43.868  1.00 32.91        C
ATOM   8582  CG   GLU F  42    14.209  35.374  45.361  1.00 35.61        C
ATOM   8583  CD   GLU F  42    14.022  36.725  46.050  1.00 37.71        C
ATOM   8584  OE1  GLU F  42    12.907  37.045  46.477  1.00 40.13        O
ATOM   8585  OE2  GLU F  42    15.006  37.472  46.170  1.00 41.04        O
ATOM   8586  N    LYS F  43    13.089  36.907  41.320  1.00 29.20        N
ATOM   8587  CA   LYS F  43    12.367  36.828  40.056  1.00 28.03        C
ATOM   8588  C    LYS F  43    12.456  38.064  39.134  1.00 25.90        C
ATOM   8589  O    LYS F  43    11.898  38.041  38.039  1.00 26.70        O
ATOM   8590  CB   LYS F  43    10.889  36.539  40.326  1.00 30.36        C
ATOM   8591  CG   LYS F  43    10.598  35.325  41.198  1.00 32.17        C
ATOM   8592  CD   LYS F  43    11.095  34.034  40.563  1.00 34.62        C
ATOM   8593  CE   LYS F  43    10.556  32.779  41.306  1.00 35.81        C
ATOM   8594  NZ   LYS F  43    11.655  31.838  41.706  1.00 37.08        N
ATOM   8595  N    GLY F  44    13.122  39.128  39.570  1.00 21.98        N
ATOM   8596  CA   GLY F  44    13.286  40.323  38.756  1.00 21.19        C
ATOM   8597  C    GLY F  44    12.049  41.205  38.748  1.00 18.13        C
ATOM   8598  O    GLY F  44    11.269  41.203  39.704  1.00 16.70        O
ATOM   8599  N    LEU F  45    11.861  41.944  37.654  1.00 18.34        N
ATOM   8600  CA   LEU F  45    10.620  42.699  37.417  1.00 18.06        C
ATOM   8601  C    LEU F  45     9.492  41.815  36.895  1.00 17.58        C
ATOM   8602  O    LEU F  45     9.640  41.163  35.860  1.00 16.02        O
ATOM   8603  CB   LEU F  45    10.877  43.815  36.414  1.00 19.20        C
ATOM   8604  CG   LEU F  45    11.859  44.888  36.832  1.00 17.89        C
ATOM   8605  CD1  LEU F  45    12.210  45.727  35.641  1.00 19.41        C
ATOM   8606  CD2  LEU F  45    11.275  45.756  37.966  1.00 18.41        C
ATOM   8607  N    GLU F  46     8.361  41.817  37.597  1.00 16.12        N
ATOM   8608  CA   GLU F  46     7.204  41.025  37.252  1.00 16.29        C
ATOM   8609  C    GLU F  46     5.896  41.833  37.231  1.00 14.56        C
ATOM   8610  O    GLU F  46     5.403  42.262  38.278  1.00 14.02        O
ATOM   8611  CB   GLU F  46     7.091  39.889  38.272  1.00 17.19        C
ATOM   8612  CG   GLU F  46     5.938  38.937  38.091  1.00 20.53        C
ATOM   8613  CD   GLU F  46     6.036  37.752  39.066  1.00 22.96        C
ATOM   8614  OE1  GLU F  46     6.811  37.867  40.069  1.00 23.88        O
ATOM   8615  OE2  GLU F  46     5.372  36.705  38.802  1.00 30.24        O
ATOM   8616  N    TRP F  47     5.361  42.048  36.031  1.00 12.56        N
ATOM   8617  CA   TRP F  47     4.018  42.598  35.832  1.00 12.77        C
ATOM   8618  C    TRP F  47     2.979  41.678  36.413  1.00 12.88        C
ATOM   8619  O    TRP F  47     2.998  40.452  36.128  1.00 11.24        O
ATOM   8620  CB   TRP F  47     3.796  42.798  34.337  1.00 13.17        C
ATOM   8621  CG   TRP F  47     2.474  43.309  33.921  1.00 12.67        C
ATOM   8622  CD1  TRP F  47     2.127  44.622  33.797  1.00 11.61        C
ATOM   8623  CD2  TRP F  47     1.334  42.546  33.513  1.00 11.00        C
ATOM   8624  NE1  TRP F  47     0.850  44.718  33.348  1.00  9.58        N
ATOM   8625  CE2  TRP F  47     0.329  43.451  33.196  1.00 11.13        C
ATOM   8626  CE3  TRP F  47     1.056  41.164  33.419  1.00 11.92        C
ATOM   8627  CZ2  TRP F  47    -0.909  43.048  32.746  1.00 11.26        C
ATOM   8628  CZ3  TRP F  47    -0.151  40.771  32.994  1.00 12.36        C
ATOM   8629  CH2  TRP F  47    -1.146  41.693  32.666  1.00 12.36        C
ATOM   8630  N    VAL F  48     2.107  42.229  37.251  1.00 12.87        N
ATOM   8631  CA   VAL F  48     1.107  41.446  37.953  1.00 13.40        C
ATOM   8632  C    VAL F  48    -0.350  41.785  37.593  1.00 12.60        C
ATOM   8633  O    VAL F  48    -1.162  40.892  37.492  1.00 14.52        O
```

| ATOM | 8634 | CB | VAL | F | 48 | 1.316 | 41.441 | 39.502 | 1.00 | 12.80 | C |
|------|------|-----|-----|---|-----|--------|--------|--------|------|-------|---|
| ATOM | 8635 | CG1 | VAL | F | 48 | 2.689 | 41.009 | 39.863 | 1.00 | 16.86 | C |
| ATOM | 8636 | CG2 | VAL | F | 48 | 1.025 | 42.784 | 40.127 | 1.00 | 14.02 | C |
| ATOM | 8637 | N | ALA | F | 49 | -0.676 | 43.057 | 37.304 | 1.00 | 10.95 | N |
| ATOM | 8638 | CA | ALA | F | 49 | -2.061 | 43.416 | 37.093 | 1.00 | 10.67 | C |
| ATOM | 8639 | C | ALA | F | 49 | -2.102 | 44.742 | 36.373 | 1.00 | 10.48 | C |
| ATOM | 8640 | O | ALA | F | 49 | -1.179 | 45.549 | 36.534 | 1.00 | 10.88 | O |
| ATOM | 8641 | CB | ALA | F | 49 | -2.828 | 43.543 | 38.425 | 1.00 | 11.99 | C |
| ATOM | 8642 | N | GLU | F | 50 | -3.175 | 44.943 | 35.617 | 1.00 | 12.63 | N |
| ATOM | 8643 | CA | GLU | F | 50 | -3.365 | 46.204 | 34.881 | 1.00 | 13.25 | C |
| ATOM | 8644 | C | GLU | F | 50 | -4.854 | 46.524 | 34.743 | 1.00 | 14.25 | C |
| ATOM | 8645 | O | GLU | F | 50 | -5.692 | 45.638 | 34.610 | 1.00 | 16.14 | O |
| ATOM | 8646 | CB | GLU | F | 50 | -2.728 | 46.069 | 33.503 | 1.00 | 13.81 | C |
| ATOM | 8647 | CG | GLU | F | 50 | -2.741 | 47.342 | 32.665 | 1.00 | 12.90 | C |
| ATOM | 8648 | CD | GLU | F | 50 | -1.736 | 47.331 | 31.555 | 1.00 | 13.89 | C |
| ATOM | 8649 | OE1 | GLU | F | 50 | -0.620 | 46.856 | 31.764 | 1.00 | 13.59 | O |
| ATOM | 8650 | OE2 | GLU | F | 50 | -2.067 | 47.808 | 30.461 | 1.00 | 13.64 | O |
| ATOM | 8651 | N | ILE | F | 51 | -5.164 | 47.816 | 34.720 | 1.00 | 14.44 | N |
| ATOM | 8652 | CA | ILE | F | 51 | -6.541 | 48.260 | 34.592 | 1.00 | 15.25 | C |
| ATOM | 8653 | C | ILE | F | 51 | -6.550 | 49.379 | 33.546 | 1.00 | 15.88 | C |
| ATOM | 8654 | O | ILE | F | 51 | -5.663 | 50.233 | 33.536 | 1.00 | 18.60 | O |
| ATOM | 8655 | CB | ILE | F | 51 | -7.157 | 48.703 | 35.942 | 1.00 | 13.27 | C |
| ATOM | 8656 | CG1 | ILE | F | 51 | -8.648 | 49.013 | 35.762 | 1.00 | 14.32 | C |
| ATOM | 8657 | CG2 | ILE | F | 51 | -6.393 | 49.905 | 36.534 | 1.00 | 15.83 | C |
| ATOM | 8658 | CD1 | ILE | F | 51 | -9.448 | 48.948 | 37.038 | 1.00 | 15.81 | C |
| ATOM | 8659 | N | ARG | F | 52 | -7.536 | 49.340 | 32.657 | 1.00 | 16.99 | N |
| ATOM | 8660 | CA | ARG | F | 52 | -7.646 | 50.346 | 31.580 | 1.00 | 17.32 | C |
| ATOM | 8661 | C | ARG | F | 52 | -8.584 | 51.487 | 31.991 | 1.00 | 17.80 | C |
| ATOM | 8662 | O | ARG | F | 52 | -8.819 | 51.699 | 33.171 | 1.00 | 17.50 | O |
| ATOM | 8663 | CB | ARG | F | 52 | -8.066 | 49.690 | 30.263 | 1.00 | 16.99 | C |
| ATOM | 8664 | CG | ARG | F | 52 | -7.150 | 48.534 | 29.788 | 1.00 | 17.85 | C |
| ATOM | 8665 | CD | ARG | F | 52 | -5.800 | 49.110 | 29.463 | 1.00 | 18.07 | C |
| ATOM | 8666 | NE | ARG | F | 52 | -4.686 | 48.183 | 29.265 | 1.00 | 18.03 | N |
| ATOM | 8667 | CZ | ARG | F | 52 | -4.469 | 47.466 | 28.163 | 1.00 | 18.02 | C |
| ATOM | 8668 | NH1 | ARG | F | 52 | -5.307 | 47.489 | 27.134 | 1.00 | 16.91 | N |
| ATOM | 8669 | NH2 | ARG | F | 52 | -3.399 | 46.731 | 28.076 | 1.00 | 16.41 | N |
| ATOM | 8670 | N | LEU | F | 52A | -9.129 | 52.207 | 31.008 | 1.00 | 18.43 | N |
| ATOM | 8671 | CA | LEU | F | 52A | -9.899 | 53.434 | 31.270 | 1.00 | 20.73 | C |
| ATOM | 8672 | C | LEU | F | 52A | -11.399 | 53.197 | 31.441 | 1.00 | 22.72 | C |
| ATOM | 8673 | O | LEU | F | 52A | -11.927 | 52.101 | 31.197 | 1.00 | 21.55 | O |
| ATOM | 8674 | CB | LEU | F | 52A | -9.696 | 54.387 | 30.104 | 1.00 | 19.19 | C |
| ATOM | 8675 | CG | LEU | F | 52A | -8.248 | 54.660 | 29.703 | 1.00 | 19.02 | C |
| ATOM | 8676 | CD1 | LEU | F | 52A | -8.172 | 55.832 | 28.683 | 1.00 | 17.72 | C |
| ATOM | 8677 | CD2 | LEU | F | 52A | -7.415 | 55.001 | 30.937 | 1.00 | 19.58 | C |
| ATOM | 8678 | N | LYS | F | 52B | -12.096 | 54.262 | 31.827 | 1.00 | 24.93 | N |
| ATOM | 8679 | CA | LYS | F | 52B | -13.559 | 54.226 | 31.899 | 1.00 | 25.82 | C |
| ATOM | 8680 | C | LYS | F | 52B | -14.132 | 53.825 | 30.543 | 1.00 | 25.93 | C |
| ATOM | 8681 | O | LYS | F | 52B | -15.062 | 53.038 | 30.477 | 1.00 | 25.05 | O |
| ATOM | 8682 | CB | LYS | F | 52B | -14.120 | 55.592 | 32.321 | 1.00 | 26.87 | C |
| ATOM | 8683 | CG | LYS | F | 52B | -15.609 | 55.552 | 32.670 | 1.00 | 28.43 | C |
| ATOM | 8684 | CD | LYS | F | 52B | -16.203 | 56.958 | 32.730 | 1.00 | 29.62 | C |
| ATOM | 8685 | CE | LYS | F | 52B | -17.658 | 56.902 | 33.214 | 1.00 | 31.78 | C |
| ATOM | 8686 | NZ | LYS | F | 52B | -18.301 | 58.269 | 33.251 | 1.00 | 32.64 | N |
| ATOM | 8687 | N | SER | F | 52C | -13.544 | 54.333 | 29.461 | 1.00 | 26.03 | N |
| ATOM | 8688 | CA | SER | F | 52C | -14.053 | 54.046 | 28.116 | 1.00 | 26.63 | C |
| ATOM | 8689 | C | SER | F | 52C | -13.863 | 52.583 | 27.680 | 1.00 | 26.56 | C |
| ATOM | 8690 | O | SER | F | 52C | -14.506 | 52.122 | 26.730 | 1.00 | 27.47 | O |
| ATOM | 8691 | CB | SER | F | 52C | -13.448 | 55.024 | 27.117 | 1.00 | 26.45 | C |
| ATOM | 8692 | OG | SER | F | 52C | -12.062 | 55.137 | 27.304 | 1.00 | 27.27 | O |
| ATOM | 8693 | N | ASP | F | 53 | -13.017 | 51.844 | 28.404 | 1.00 | 26.22 | N |
| ATOM | 8694 | CA | ASP | F | 53 | -12.841 | 50.407 | 28.210 | 1.00 | 26.60 | C |
| ATOM | 8695 | C | ASP | F | 53 | -13.615 | 49.613 | 29.258 | 1.00 | 26.09 | C |
| ATOM | 8696 | O | ASP | F | 53 | -13.393 | 48.425 | 29.422 | 1.00 | 25.40 | O |
| ATOM | 8697 | CB | ASP | F | 53 | -11.356 | 50.056 | 28.300 | 1.00 | 26.12 | C |
| ATOM | 8698 | CG | ASP | F | 53 | -10.477 | 51.034 | 27.547 | 1.00 | 27.19 | C |

| | | | | | | | | | | | | |
|---|---|---|---|---|---|---|---|---|---|---|---|---|
| ATOM | 8699 | OD1 | ASP | F | 53 | -10.559 | 51.109 | 26.301 | 1.00 | 28.76 | | O |
| ATOM | 8700 | OD2 | ASP | F | 53 | -9.676 | 51.730 | 28.189 | 1.00 | 25.24 | | O |
| ATOM | 8701 | N | ASN | F | 54 | -14.525 | 50.277 | 29.971 | 1.00 | 26.22 | | N |
| ATOM | 8702 | CA | ASN | F | 54 | -15.262 | 49.649 | 31.063 | 1.00 | 25.44 | | C |
| ATOM | 8703 | C | ASN | F | 54 | -14.321 | 49.193 | 32.193 | 1.00 | 23.81 | | C |
| ATOM | 8704 | O | ASN | F | 54 | -14.614 | 48.219 | 32.890 | 1.00 | 24.82 | | O |
| ATOM | 8705 | CB | ASN | F | 54 | -16.066 | 48.473 | 30.538 | 1.00 | 27.16 | | C |
| ATOM | 8706 | CG | ASN | F | 54 | -17.265 | 48.135 | 31.404 | 1.00 | 29.86 | | C |
| ATOM | 8707 | OD1 | ASN | F | 54 | -17.540 | 48.785 | 32.417 | 1.00 | 35.28 | | O |
| ATOM | 8708 | ND2 | ASN | F | 54 | -17.990 | 47.091 | 31.006 | 1.00 | 34.35 | | N |
| ATOM | 8709 | N | TYR | F | 55 | -13.208 | 49.912 | 32.376 | 1.00 | 21.84 | | N |
| ATOM | 8710 | CA | TYR | F | 55 | -12.238 | 49.587 | 33.418 | 1.00 | 21.55 | | C |
| ATOM | 8711 | C | TYR | F | 55 | -11.773 | 48.139 | 33.308 | 1.00 | 19.24 | | C |
| ATOM | 8712 | O | TYR | F | 55 | -11.651 | 47.458 | 34.324 | 1.00 | 19.24 | | O |
| ATOM | 8713 | CB | TYR | F | 55 | -12.846 | 49.812 | 34.800 | 1.00 | 22.21 | | C |
| ATOM | 8714 | CG | TYR | F | 55 | -13.122 | 51.252 | 35.078 | 1.00 | 22.64 | | C |
| ATOM | 8715 | CD1 | TYR | F | 55 | -12.120 | 52.181 | 34.966 | 1.00 | 22.58 | | C |
| ATOM | 8716 | CD2 | TYR | F | 55 | -14.385 | 51.696 | 35.446 | 1.00 | 23.87 | | C |
| ATOM | 8717 | CE1 | TYR | F | 55 | -12.346 | 53.498 | 35.196 | 1.00 | 23.83 | | C |
| ATOM | 8718 | CE2 | TYR | F | 55 | -14.618 | 53.041 | 35.699 | 1.00 | 23.97 | | C |
| ATOM | 8719 | CZ | TYR | F | 55 | -13.585 | 53.929 | 35.567 | 1.00 | 23.66 | | C |
| ATOM | 8720 | OH | TYR | F | 55 | -13.735 | 55.267 | 35.798 | 1.00 | 26.30 | | O |
| ATOM | 8721 | N | ALA | F | 56 | -11.490 | 47.697 | 32.094 | 1.00 | 18.35 | | N |
| ATOM | 8722 | CA | ALA | F | 56 | -11.044 | 46.305 | 31.864 | 1.00 | 18.11 | | C |
| ATOM | 8723 | C | ALA | F | 56 | -9.807 | 46.036 | 32.696 | 1.00 | 17.44 | | C |
| ATOM | 8724 | O | ALA | F | 56 | -8.916 | 46.886 | 32.782 | 1.00 | 16.46 | | O |
| ATOM | 8725 | CB | ALA | F | 56 | -10.716 | 46.101 | 30.418 | 1.00 | 19.43 | | C |
| ATOM | 8726 | N | THR | F | 57 | -9.719 | 44.836 | 33.254 | 1.00 | 16.84 | | N |
| ATOM | 8727 | CA | THR | F | 57 | -8.589 | 44.481 | 34.123 | 1.00 | 16.40 | | C |
| ATOM | 8728 | C | THR | F | 57 | -7.975 | 43.203 | 33.604 | 1.00 | 15.71 | | C |
| ATOM | 8729 | O | THR | F | 57 | -8.661 | 42.427 | 32.937 | 1.00 | 12.10 | | O |
| ATOM | 8730 | CB | THR | F | 57 | -9.018 | 44.290 | 35.585 | 1.00 | 17.12 | | C |
| ATOM | 8731 | OG1 | THR | F | 57 | -10.059 | 43.314 | 35.650 | 1.00 | 15.95 | | O |
| ATOM | 8732 | CG2 | THR | F | 57 | -9.553 | 45.567 | 36.187 | 1.00 | 17.18 | | C |
| ATOM | 8733 | N | TYR | F | 58 | -6.662 | 43.072 | 33.838 | 1.00 | 15.16 | | N |
| ATOM | 8734 | CA | TYR | F | 58 | -5.841 | 41.982 | 33.327 | 1.00 | 15.86 | | C |
| ATOM | 8735 | C | TYR | F | 58 | -4.835 | 41.616 | 34.415 | 1.00 | 14.63 | | C |
| ATOM | 8736 | O | TYR | F | 58 | -4.380 | 42.477 | 35.160 | 1.00 | 14.22 | | O |
| ATOM | 8737 | CB | TYR | F | 58 | -5.101 | 42.388 | 32.047 | 1.00 | 15.81 | | C |
| ATOM | 8738 | CG | TYR | F | 58 | -6.001 | 42.914 | 30.964 | 1.00 | 15.32 | | C |
| ATOM | 8739 | CD1 | TYR | F | 58 | -6.202 | 44.302 | 30.794 | 1.00 | 16.65 | | C |
| ATOM | 8740 | CD2 | TYR | F | 58 | -6.689 | 42.049 | 30.136 | 1.00 | 16.11 | | C |
| ATOM | 8741 | CE1 | TYR | F | 58 | -7.064 | 44.762 | 29.808 | 1.00 | 16.06 | | C |
| ATOM | 8742 | CE2 | TYR | F | 58 | -7.567 | 42.520 | 29.167 | 1.00 | 14.46 | | C |
| ATOM | 8743 | CZ | TYR | F | 58 | -7.727 | 43.872 | 29.010 | 1.00 | 14.76 | | C |
| ATOM | 8744 | OH | TYR | F | 58 | -8.569 | 44.310 | 28.033 | 1.00 | 15.68 | | O |
| ATOM | 8745 | N | TYR | F | 59 | -4.515 | 40.327 | 34.514 | 1.00 | 15.73 | | N |
| ATOM | 8746 | CA | TYR | F | 59 | -3.671 | 39.791 | 35.578 | 1.00 | 16.66 | | C |
| ATOM | 8747 | C | TYR | F | 59 | -2.649 | 38.817 | 35.020 | 1.00 | 17.45 | | C |
| ATOM | 8748 | O | TYR | F | 59 | -2.925 | 38.106 | 34.041 | 1.00 | 17.21 | | O |
| ATOM | 8749 | CB | TYR | F | 59 | -4.512 | 39.038 | 36.616 | 1.00 | 16.42 | | C |
| ATOM | 8750 | CG | TYR | F | 59 | -5.483 | 39.918 | 37.289 | 1.00 | 16.22 | | C |
| ATOM | 8751 | CD1 | TYR | F | 59 | -5.095 | 40.714 | 38.369 | 1.00 | 17.25 | | C |
| ATOM | 8752 | CD2 | TYR | F | 59 | -6.767 | 40.045 | 36.808 | 1.00 | 17.40 | | C |
| ATOM | 8753 | CE1 | TYR | F | 59 | -5.975 | 41.589 | 38.974 | 1.00 | 15.10 | | C |
| ATOM | 8754 | CE2 | TYR | F | 59 | -7.651 | 40.921 | 37.396 | 1.00 | 16.66 | | C |
| ATOM | 8755 | CZ | TYR | F | 59 | -7.264 | 41.673 | 38.479 | 1.00 | 16.20 | | C |
| ATOM | 8756 | OH | TYR | F | 59 | -8.171 | 42.511 | 39.041 | 1.00 | 16.57 | | O |
| ATOM | 8757 | N | ALA | F | 60 | -1.489 | 38.786 | 35.664 | 1.00 | 19.07 | | N |
| ATOM | 8758 | CA | ALA | F | 60 | -0.500 | 37.779 | 35.394 | 1.00 | 22.27 | | C |
| ATOM | 8759 | C | ALA | F | 60 | -1.096 | 36.461 | 35.860 | 1.00 | 22.49 | | C |
| ATOM | 8760 | O | ALA | F | 60 | -1.831 | 36.416 | 36.838 | 1.00 | 20.79 | | O |
| ATOM | 8761 | CB | ALA | F | 60 | 0.768 | 38.079 | 36.118 | 1.00 | 21.81 | | C |
| ATOM | 8762 | N | GLU | F | 61 | -0.797 | 35.395 | 35.132 | 1.00 | 26.61 | | N |
| ATOM | 8763 | CA | GLU | F | 61 | -1.306 | 34.065 | 35.438 | 1.00 | 27.64 | | C |

```
ATOM   8764  C    GLU F  61    -1.092  33.655  36.904  1.00 29.64         C
ATOM   8765  O    GLU F  61    -1.941  32.981  37.519  1.00 30.68         O
ATOM   8766  CB   GLU F  61    -0.575  33.067  34.540  1.00 29.66         C
ATOM   8767  CG   GLU F  61    -1.098  31.658  34.582  1.00 31.66         C
ATOM   8768  CD   GLU F  61    -2.388  31.514  33.818  1.00 36.27         C
ATOM   8769  OE1  GLU F  61    -2.342  31.689  32.576  1.00 40.65         O
ATOM   8770  OE2  GLU F  61    -3.437  31.226  34.449  1.00 38.12         O
ATOM   8771  N    SER F  62     0.062  34.030  37.442  1.00 29.58         N
ATOM   8772  CA   SER F  62     0.485  33.592  38.770  1.00 30.29         C
ATOM   8773  C    SER F  62    -0.260  34.235  39.942  1.00 30.82         C
ATOM   8774  O    SER F  62    -0.143  33.765  41.079  1.00 32.39         O
ATOM   8775  CB   SER F  62     1.967  33.864  38.933  1.00 30.69         C
ATOM   8776  OG   SER F  62     2.230  35.255  39.015  1.00 31.47         O
ATOM   8777  N    VAL F  63    -1.008  35.307  39.682  1.00 29.53         N
ATOM   8778  CA   VAL F  63    -1.717  36.006  40.745  1.00 30.04         C
ATOM   8779  C    VAL F  63    -3.212  36.038  40.522  1.00 30.05         C
ATOM   8780  O    VAL F  63    -3.945  36.561  41.351  1.00 28.47         O
ATOM   8781  CB   VAL F  63    -1.195  37.458  40.880  1.00 29.56         C
ATOM   8782  CG1  VAL F  63     0.311  37.462  41.097  1.00 28.76         C
ATOM   8783  CG2  VAL F  63    -1.569  38.293  39.663  1.00 29.83         C
ATOM   8784  N    LYS F  64    -3.677  35.494  39.394  1.00 31.21         N
ATOM   8785  CA   LYS F  64    -5.103  35.555  39.059  1.00 32.06         C
ATOM   8786  C    LYS F  64    -5.881  34.893  40.191  1.00 31.36         C
ATOM   8787  O    LYS F  64    -5.489  33.844  40.682  1.00 30.90         O
ATOM   8788  CB   LYS F  64    -5.411  34.863  37.719  1.00 33.33         C
ATOM   8789  CG   LYS F  64    -4.597  35.372  36.535  1.00 34.05         C
ATOM   8790  CD   LYS F  64    -5.291  35.089  35.200  1.00 34.93         C
ATOM   8791  CE   LYS F  64    -4.448  35.531  33.990  1.00 35.22         C
ATOM   8792  NZ   LYS F  64    -4.588  34.584  32.843  1.00 36.25         N
ATOM   8793  N    GLY F  65    -6.942  35.549  40.646  1.00 31.68         N
ATOM   8794  CA   GLY F  65    -7.739  35.024  41.747  1.00 30.65         C
ATOM   8795  C    GLY F  65    -7.261  35.428  43.130  1.00 30.55         C
ATOM   8796  O    GLY F  65    -8.040  35.387  44.082  1.00 32.68         O
ATOM   8797  N    LYS F  66    -5.993  35.812  43.264  1.00 28.41         N
ATOM   8798  CA   LYS F  66    -5.499  36.356  44.531  1.00 26.20         C
ATOM   8799  C    LYS F  66    -5.495  37.886  44.551  1.00 23.33         C
ATOM   8800  O    LYS F  66    -5.705  38.485  45.601  1.00 22.56         O
ATOM   8801  CB   LYS F  66    -4.096  35.865  44.805  1.00 26.52         C
ATOM   8802  CG   LYS F  66    -4.045  34.445  45.292  1.00 28.43         C
ATOM   8803  CD   LYS F  66    -2.648  33.878  45.210  1.00 28.34         C
ATOM   8804  CE   LYS F  66    -1.575  34.817  45.705  1.00 28.81         C
ATOM   8805  NZ   LYS F  66    -0.329  34.040  46.069  1.00 31.03         N
ATOM   8806  N    PHE F  67    -5.227  38.509  43.404  1.00 19.83         N
ATOM   8807  CA   PHE F  67    -5.090  39.990  43.333  1.00 19.72         C
ATOM   8808  C    PHE F  67    -6.297  40.623  42.625  1.00 17.88         C
ATOM   8809  O    PHE F  67    -6.819  40.058  41.673  1.00 17.41         O
ATOM   8810  CB   PHE F  67    -3.801  40.385  42.605  1.00 18.70         C
ATOM   8811  CG   PHE F  67    -2.515  40.065  43.357  1.00 19.36         C
ATOM   8812  CD1  PHE F  67    -2.500  39.317  44.531  1.00 19.88         C
ATOM   8813  CD2  PHE F  67    -1.310  40.476  42.834  1.00 20.25         C
ATOM   8814  CE1  PHE F  67    -1.299  39.033  45.192  1.00 20.31         C
ATOM   8815  CE2  PHE F  67    -0.093  40.188  43.488  1.00 19.97         C
ATOM   8816  CZ   PHE F  67    -0.093  39.489  44.651  1.00 19.57         C
ATOM   8817  N    THR F  68    -6.722  41.795  43.102  1.00 18.05         N
ATOM   8818  CA   THR F  68    -7.714  42.614  42.411  1.00 16.08         C
ATOM   8819  C    THR F  68    -7.209  44.041  42.296  1.00 15.47         C
ATOM   8820  O    THR F  68    -6.903  44.665  43.302  1.00 13.10         O
ATOM   8821  CB   THR F  68    -9.068  42.611  43.108  1.00 16.77         C
ATOM   8822  OG1  THR F  68    -9.466  41.267  43.365  1.00 15.75         O
ATOM   8823  CG2  THR F  68   -10.118  43.227  42.225  1.00 17.48         C
ATOM   8824  N    ILE F  69    -7.110  44.529  41.058  1.00 14.31         N
ATOM   8825  CA   ILE F  69    -6.676  45.902  40.790  1.00 13.02         C
ATOM   8826  C    ILE F  69    -7.921  46.742  40.570  1.00 13.42         C
ATOM   8827  O    ILE F  69    -8.910  46.285  40.053  1.00 14.25         O
ATOM   8828  CB   ILE F  69    -5.700  45.970  39.571  1.00 12.38         C
```

```
ATOM   8829  CG1 ILE F  69     -5.044  47.354  39.452  1.00 12.71        C
ATOM   8830  CG2 ILE F  69     -6.399  45.575  38.260  1.00 13.95        C
ATOM   8831  CD1 ILE F  69     -4.019  47.441  38.322  1.00 13.41        C
ATOM   8832  N   SER F  70     -7.901  47.984  41.023  1.00 13.64        N
ATOM   8833  CA  SER F  70     -9.059  48.817  40.869  1.00 13.30        C
ATOM   8834  C   SER F  70     -8.530  50.220  40.917  1.00 12.32        C
ATOM   8835  O   SER F  70     -7.385  50.438  41.315  1.00 12.16        O
ATOM   8836  CB  SER F  70    -10.052  48.622  42.011  1.00 14.61        C
ATOM   8837  OG  SER F  70     -9.457  48.862  43.265  1.00 15.13        O
ATOM   8838  N   ARG F  71     -9.368  51.155  40.500  1.00 15.13        N
ATOM   8839  CA  ARG F  71     -8.978  52.582  40.444  1.00 16.34        C
ATOM   8840  C   ARG F  71    -10.116  53.508  40.850  1.00 16.54        C
ATOM   8841  O   ARG F  71    -11.265  53.179  40.638  1.00 17.38        O
ATOM   8842  CB  ARG F  71     -8.506  52.977  39.037  1.00 16.48        C
ATOM   8843  CG  ARG F  71     -9.552  52.855  37.903  1.00 17.19        C
ATOM   8844  CD  ARG F  71     -8.953  52.956  36.481  1.00 16.64        C
ATOM   8845  NE  ARG F  71     -8.262  54.239  36.229  1.00 15.65        N
ATOM   8846  CZ  ARG F  71     -7.292  54.433  35.327  1.00 15.43        C
ATOM   8847  NH1 ARG F  71     -6.835  53.437  34.584  1.00 13.50        N
ATOM   8848  NH2 ARG F  71     -6.719  55.637  35.216  1.00 14.19        N
ATOM   8849  N   ASP F  72     -9.744  54.676  41.381  1.00 18.65        N
ATOM   8850  CA  ASP F  72    -10.691  55.739  41.765  1.00 18.31        C
ATOM   8851  C   ASP F  72    -10.251  57.015  41.064  1.00 17.56        C
ATOM   8852  O   ASP F  72     -9.385  57.771  41.540  1.00 17.05        O
ATOM   8853  CB  ASP F  72    -10.754  55.942  43.284  1.00 19.67        C
ATOM   8854  CG  ASP F  72    -11.824  56.982  43.701  1.00 22.09        C
ATOM   8855  OD1 ASP F  72    -12.277  57.739  42.828  1.00 25.84        O
ATOM   8856  OD2 ASP F  72    -12.240  57.018  44.887  1.00 25.07        O
ATOM   8857  N   ASP F  73    -10.852  57.251  39.920  1.00 18.61        N
ATOM   8858  CA  ASP F  73    -10.400  58.339  39.075  1.00 19.69        C
ATOM   8859  C   ASP F  73    -10.584  59.675  39.817  1.00 20.00        C
ATOM   8860  O   ASP F  73     -9.741  60.564  39.682  1.00 20.32        O
ATOM   8861  CB  ASP F  73    -11.107  58.320  37.723  1.00 20.70        C
ATOM   8862  CG  ASP F  73    -10.590  57.202  36.789  1.00 21.20        C
ATOM   8863  OD1 ASP F  73     -9.477  56.663  37.023  1.00 17.36        O
ATOM   8864  OD2 ASP F  73    -11.284  56.914  35.796  1.00 23.83        O
ATOM   8865  N   SER F  74    -11.622  59.783  40.651  1.00 21.05        N
ATOM   8866  CA  SER F  74    -11.866  61.049  41.394  1.00 21.34        C
ATOM   8867  C   SER F  74    -10.803  61.377  42.451  1.00 21.89        C
ATOM   8868  O   SER F  74    -10.630  62.571  42.868  1.00 21.17        O
ATOM   8869  CB  SER F  74    -13.265  61.030  42.025  1.00 22.80        C
ATOM   8870  OG  SER F  74    -13.361  60.022  43.020  1.00 23.64        O
ATOM   8871  N   LYS F  75    -10.083  60.343  42.878  1.00 19.96        N
ATOM   8872  CA  LYS F  75     -9.053  60.452  43.910  1.00 21.03        C
ATOM   8873  C   LYS F  75     -7.647  60.342  43.318  1.00 19.72        C
ATOM   8874  O   LYS F  75     -6.658  60.510  44.041  1.00 19.46        O
ATOM   8875  CB  LYS F  75     -9.259  59.342  44.970  1.00 23.14        C
ATOM   8876  CG  LYS F  75    -10.448  59.571  45.879  1.00 24.63        C
ATOM   8877  CD  LYS F  75    -10.386  58.642  47.092  1.00 25.51        C
ATOM   8878  CE  LYS F  75    -11.670  58.709  47.911  1.00 26.69        C
ATOM   8879  NZ  LYS F  75    -11.956  60.115  48.389  1.00 28.19        N
ATOM   8880  N   SER F  76     -7.569  60.097  42.005  1.00 19.39        N
ATOM   8881  CA  SER F  76     -6.313  59.842  41.297  1.00 18.60        C
ATOM   8882  C   SER F  76     -5.571  58.696  41.966  1.00 18.03        C
ATOM   8883  O   SER F  76     -4.381  58.784  42.195  1.00 16.68        O
ATOM   8884  CB  SER F  76     -5.425  61.085  41.256  1.00 19.30        C
ATOM   8885  OG  SER F  76     -6.088  62.123  40.546  1.00 20.03        O
ATOM   8886  N   ARG F  77     -6.289  57.629  42.279  1.00 18.31        N
ATOM   8887  CA  ARG F  77     -5.719  56.579  43.110  1.00 18.00        C
ATOM   8888  C   ARG F  77     -5.951  55.183  42.531  1.00 16.99        C
ATOM   8889  O   ARG F  77     -7.014  54.859  42.039  1.00 15.90        O
ATOM   8890  CB  ARG F  77     -6.269  56.697  44.537  1.00 18.33        C
ATOM   8891  CG  ARG F  77     -5.526  55.847  45.556  1.00 19.85        C
ATOM   8892  CD  ARG F  77     -5.972  56.156  46.973  1.00 22.02        C
ATOM   8893  NE  ARG F  77     -5.336  57.361  47.518  1.00 25.95        N
```

| ATOM | 8894 | CZ | ARG | F | 77 | -4.068 | 57.454 | 47.929 | 1.00 | 28.17 | C |
|------|------|-----|-----|---|-----|--------|--------|--------|------|-------|---|
| ATOM | 8895 | NH1 | ARG | F | 77 | -3.226 | 56.407 | 47.873 | 1.00 | 29.59 | N |
| ATOM | 8896 | NH2 | ARG | F | 77 | -3.630 | 58.618 | 48.413 | 1.00 | 28.35 | N |
| ATOM | 8897 | N | LEU | F | 78 | -4.902 | 54.376 | 42.594 | 1.00 | 15.74 | N |
| ATOM | 8898 | CA | LEU | F | 78 | -4.909 | 53.007 | 42.076 | 1.00 | 15.45 | C |
| ATOM | 8899 | C | LEU | F | 78 | -4.734 | 52.103 | 43.281 | 1.00 | 13.66 | C |
| ATOM | 8900 | O | LEU | F | 78 | -3.939 | 52.401 | 44.149 | 1.00 | 13.76 | O |
| ATOM | 8901 | CB | LEU | F | 78 | -3.748 | 52.839 | 41.082 | 1.00 | 14.09 | C |
| ATOM | 8902 | CG | LEU | F | 78 | -3.648 | 51.460 | 40.402 | 1.00 | 15.18 | C |
| ATOM | 8903 | CD1 | LEU | F | 78 | -4.703 | 51.410 | 39.346 | 1.00 | 15.48 | C |
| ATOM | 8904 | CD2 | LEU | F | 78 | -2.250 | 51.213 | 39.815 | 1.00 | 15.35 | C |
| ATOM | 8905 | N | TYR | F | 79 | -5.444 | 50.978 | 43.316 | 1.00 | 13.90 | N |
| ATOM | 8906 | CA | TYR | F | 79 | -5.379 | 50.056 | 44.421 | 1.00 | 15.18 | C |
| ATOM | 8907 | C | TYR | F | 79 | -5.003 | 48.673 | 43.930 | 1.00 | 12.72 | C |
| ATOM | 8908 | O | TYR | F | 79 | -5.294 | 48.334 | 42.814 | 1.00 | 11.95 | O |
| ATOM | 8909 | CB | TYR | F | 79 | -6.732 | 49.932 | 45.112 | 1.00 | 17.33 | C |
| ATOM | 8910 | CG | TYR | F | 79 | -7.259 | 51.241 | 45.633 | 1.00 | 19.37 | C |
| ATOM | 8911 | CD1 | TYR | F | 79 | -6.933 | 51.676 | 46.905 | 1.00 | 19.62 | C |
| ATOM | 8912 | CD2 | TYR | F | 79 | -8.107 | 52.024 | 44.862 | 1.00 | 19.21 | C |
| ATOM | 8913 | CE1 | TYR | F | 79 | -7.421 | 52.911 | 47.388 | 1.00 | 21.25 | C |
| ATOM | 8914 | CE2 | TYR | F | 79 | -8.589 | 53.240 | 45.325 | 1.00 | 19.60 | C |
| ATOM | 8915 | CZ | TYR | F | 79 | -8.252 | 53.679 | 46.570 | 1.00 | 20.29 | C |
| ATOM | 8916 | OH | TYR | F | 79 | -8.753 | 54.892 | 47.006 | 1.00 | 23.46 | O |
| ATOM | 8917 | N | LEU | F | 80 | -4.326 | 47.902 | 44.780 | 1.00 | 13.46 | N |
| ATOM | 8918 | CA | LEU | F | 80 | -4.234 | 46.432 | 44.595 | 1.00 | 13.87 | C |
| ATOM | 8919 | C | LEU | F | 80 | -4.625 | 45.731 | 45.887 | 1.00 | 14.14 | C |
| ATOM | 8920 | O | LEU | F | 80 | -3.985 | 45.932 | 46.921 | 1.00 | 14.12 | O |
| ATOM | 8921 | CB | LEU | F | 80 | -2.799 | 46.018 | 44.220 | 1.00 | 13.29 | C |
| ATOM | 8922 | CG | LEU | F | 80 | -2.630 | 44.574 | 43.722 | 1.00 | 13.52 | C |
| ATOM | 8923 | CD1 | LEU | F | 80 | -3.309 | 44.378 | 42.385 | 1.00 | 15.01 | C |
| ATOM | 8924 | CD2 | LEU | F | 80 | -1.146 | 44.251 | 43.682 | 1.00 | 15.32 | C |
| ATOM | 8925 | N | GLN | F | 81 | -5.650 | 44.881 | 45.822 | 1.00 | 15.30 | N |
| ATOM | 8926 | CA | GLN | F | 81 | -6.037 | 44.075 | 46.945 | 1.00 | 16.00 | C |
| ATOM | 8927 | C | GLN | F | 81 | -5.392 | 42.712 | 46.759 | 1.00 | 16.63 | C |
| ATOM | 8928 | O | GLN | F | 81 | -5.507 | 42.108 | 45.701 | 1.00 | 15.72 | O |
| ATOM | 8929 | CB | GLN | F | 81 | -7.544 | 43.940 | 47.006 | 1.00 | 16.21 | C |
| ATOM | 8930 | CG | GLN | F | 81 | -8.034 | 43.044 | 48.118 | 1.00 | 18.78 | C |
| ATOM | 8931 | CD | GLN | F | 81 | -7.911 | 43.694 | 49.451 | 1.00 | 20.45 | C |
| ATOM | 8932 | OE1 | GLN | F | 81 | -8.345 | 44.821 | 49.629 | 1.00 | 26.18 | O |
| ATOM | 8933 | NE2 | GLN | F | 81 | -7.294 | 43.000 | 50.406 | 1.00 | 21.96 | N |
| ATOM | 8934 | N | MET | F | 82 | -4.730 | 42.243 | 47.801 | 1.00 | 17.37 | N |
| ATOM | 8935 | CA | MET | F | 82 | -3.890 | 41.063 | 47.701 | 1.00 | 18.62 | C |
| ATOM | 8936 | C | MET | F | 82 | -4.373 | 40.106 | 48.747 | 1.00 | 17.92 | C |
| ATOM | 8937 | O | MET | F | 82 | -4.288 | 40.411 | 49.915 | 1.00 | 19.38 | O |
| ATOM | 8938 | CB | MET | F | 82 | -2.441 | 41.466 | 47.955 | 1.00 | 18.16 | C |
| ATOM | 8939 | CG | MET | F | 82 | -1.972 | 42.615 | 47.053 | 1.00 | 20.67 | C |
| ATOM | 8940 | SD | MET | F | 82 | -0.423 | 43.427 | 47.534 | 1.00 | 22.51 | S |
| ATOM | 8941 | CE | MET | F | 82 | 0.584 | 42.127 | 46.951 | 1.00 | 20.97 | C |
| ATOM | 8942 | N | ASN | F | 82A | -4.879 | 38.952 | 48.332 | 1.00 | 18.69 | N |
| ATOM | 8943 | CA | ASN | F | 82A | -5.401 | 37.974 | 49.249 | 1.00 | 19.47 | C |
| ATOM | 8944 | C | ASN | F | 82A | -4.625 | 36.643 | 49.149 | 1.00 | 19.49 | C |
| ATOM | 8945 | O | ASN | F | 82A | -3.986 | 36.363 | 48.145 | 1.00 | 16.89 | O |
| ATOM | 8946 | CB | ASN | F | 82A | -6.854 | 37.668 | 48.923 | 1.00 | 20.71 | C |
| ATOM | 8947 | CG | ASN | F | 82A | -7.773 | 38.852 | 49.096 | 1.00 | 21.54 | C |
| ATOM | 8948 | OD1 | ASN | F | 82A | -7.582 | 39.727 | 49.956 | 1.00 | 21.60 | O |
| ATOM | 8949 | ND2 | ASN | F | 82A | -8.796 | 38.894 | 48.249 | 1.00 | 23.78 | N |
| ATOM | 8950 | N | ASN | F | 82B | -4.721 | 35.836 | 50.191 | 1.00 | 20.41 | N |
| ATOM | 8951 | CA | ASN | F | 82B | -3.989 | 34.558 | 50.257 | 1.00 | 21.20 | C |
| ATOM | 8952 | C | ASN | F | 82B | -2.579 | 34.718 | 49.804 | 1.00 | 19.64 | C |
| ATOM | 8953 | O | ASN | F | 82B | -2.114 | 34.030 | 48.889 | 1.00 | 20.14 | O |
| ATOM | 8954 | CB | ASN | F | 82B | -4.647 | 33.500 | 49.399 | 1.00 | 22.42 | C |
| ATOM | 8955 | CG | ASN | F | 82B | -3.943 | 32.142 | 49.520 | 1.00 | 25.47 | C |
| ATOM | 8956 | OD1 | ASN | F | 82B | -3.632 | 31.503 | 48.509 | 1.00 | 32.77 | O |
| ATOM | 8957 | ND2 | ASN | F | 82B | -3.631 | 31.735 | 50.760 | 1.00 | 28.40 | N |
| ATOM | 8958 | N | LEU | F | 82C | -1.895 | 35.669 | 50.416 | 1.00 | 19.55 | N |

| ATOM | 8959 | CA | LEU | F | 82C | -0.509 | 35.944 | 50.074 | 1.00 | 17.59 | C |
|------|------|------|------|---|-----|--------|--------|--------|------|-------|---|
| ATOM | 8960 | C | LEU | F | 82C | 0.397 | 34.810 | 50.540 | 1.00 | 17.18 | C |
| ATOM | 8961 | O | LEU | F | 82C | 0.151 | 34.239 | 51.587 | 1.00 | 17.36 | O |
| ATOM | 8962 | CB | LEU | F | 82C | -0.070 | 37.233 | 50.728 | 1.00 | 17.43 | C |
| ATOM | 8963 | CG | LEU | F | 82C | -0.568 | 38.483 | 49.982 | 1.00 | 16.20 | C |
| ATOM | 8964 | CD1 | LEU | F | 82C | -0.428 | 39.794 | 50.815 | 1.00 | 17.78 | C |
| ATOM | 8965 | CD2 | LEU | F | 82C | 0.182 | 38.581 | 48.658 | 1.00 | 17.29 | C |
| ATOM | 8966 | N | ARG | F | 83 | 1.421 | 34.502 | 49.741 | 1.00 | 16.64 | N |
| ATOM | 8967 | CA | ARG | F | 83 | 2.442 | 33.523 | 50.099 | 1.00 | 17.98 | C |
| ATOM | 8968 | C | ARG | F | 83 | 3.791 | 34.228 | 50.194 | 1.00 | 16.30 | C |
| ATOM | 8969 | O | ARG | F | 83 | 3.984 | 35.364 | 49.729 | 1.00 | 16.73 | O |
| ATOM | 8970 | CB | ARG | F | 83 | 2.527 | 32.402 | 49.066 | 1.00 | 19.75 | C |
| ATOM | 8971 | CG | ARG | F | 83 | 1.198 | 31.775 | 48.625 | 1.00 | 22.07 | C |
| ATOM | 8972 | CD | ARG | F | 83 | 0.257 | 31.411 | 49.767 | 1.00 | 25.86 | C |
| ATOM | 8973 | NE | ARG | F | 83 | 0.845 | 30.600 | 50.845 | 1.00 | 27.53 | N |
| ATOM | 8974 | CZ | ARG | F | 83 | 0.167 | 30.185 | 51.918 | 1.00 | 27.60 | C |
| ATOM | 8975 | NH1 | ARG | F | 83 | -1.120 | 30.514 | 52.061 | 1.00 | 28.12 | N |
| ATOM | 8976 | NH2 | ARG | F | 83 | 0.771 | 29.432 | 52.851 | 1.00 | 28.25 | N |
| ATOM | 8977 | N | THR | F | 84 | 4.761 | 33.566 | 50.781 | 1.00 | 15.90 | N |
| ATOM | 8978 | CA | THR | F | 84 | 6.074 | 34.188 | 50.959 | 1.00 | 16.20 | C |
| ATOM | 8979 | C | THR | F | 84 | 6.684 | 34.619 | 49.620 | 1.00 | 15.72 | C |
| ATOM | 8980 | O | THR | F | 84 | 7.433 | 35.600 | 49.580 | 1.00 | 17.40 | O |
| ATOM | 8981 | CB | THR | F | 84 | 7.053 | 33.277 | 51.728 | 1.00 | 17.12 | C |
| ATOM | 8982 | OG1 | THR | F | 84 | 7.152 | 32.022 | 51.054 | 1.00 | 18.93 | O |
| ATOM | 8983 | CG2 | THR | F | 84 | 6.557 | 33.031 | 53.151 | 1.00 | 16.04 | C |
| ATOM | 8984 | N | GLU | F | 85 | 6.313 | 33.924 | 48.537 | 1.00 | 15.11 | N |
| ATOM | 8985 | CA | AGLU | F | 85 | 6.836 | 34.210 | 47.196 | 0.50 | 15.51 | C |
| ATOM | 8986 | CA | BGLU | F | 85 | 6.810 | 34.190 | 47.183 | 0.50 | 15.96 | C |
| ATOM | 8987 | C | GLU | F | 85 | 6.310 | 35.541 | 46.664 | 1.00 | 15.87 | C |
| ATOM | 8988 | O | GLU | F | 85 | 6.846 | 36.071 | 45.686 | 1.00 | 16.25 | O |
| ATOM | 8989 | CB | AGLU | F | 85 | 6.476 | 33.119 | 46.185 | 0.50 | 15.87 | C |
| ATOM | 8990 | CB | BGLU | F | 85 | 6.392 | 33.080 | 46.197 | 0.50 | 16.34 | C |
| ATOM | 8991 | CG | AGLU | F | 85 | 6.721 | 31.703 | 46.668 | 0.50 | 17.07 | C |
| ATOM | 8992 | CG | BGLU | F | 85 | 4.872 | 32.900 | 46.025 | 0.50 | 17.31 | C |
| ATOM | 8993 | CD | AGLU | F | 85 | 5.524 | 31.123 | 47.397 | 0.50 | 17.40 | C |
| ATOM | 8994 | CD | BGLU | F | 85 | 4.479 | 31.635 | 45.278 | 0.50 | 19.01 | C |
| ATOM | 8995 | OE1 | AGLU | F | 85 | 5.569 | 31.025 | 48.633 | 0.50 | 14.03 | O |
| ATOM | 8996 | OE1 | BGLU | F | 85 | 4.980 | 31.412 | 44.153 | 0.50 | 19.87 | O |
| ATOM | 8997 | OE2 | AGLU | F | 85 | 4.529 | 30.756 | 46.724 | 0.50 | 21.57 | O |
| ATOM | 8998 | OE2 | BGLU | F | 85 | 3.634 | 30.883 | 45.809 | 0.50 | 23.06 | O |
| ATOM | 8999 | N | ASP | F | 86 | 5.266 | 36.073 | 47.302 | 1.00 | 13.97 | N |
| ATOM | 9000 | CA | ASP | F | 86 | 4.705 | 37.357 | 46.871 | 1.00 | 14.09 | C |
| ATOM | 9001 | C | ASP | F | 86 | 5.386 | 38.564 | 47.505 | 1.00 | 13.61 | C |
| ATOM | 9002 | O | ASP | F | 86 | 5.048 | 39.702 | 47.162 | 1.00 | 15.36 | O |
| ATOM | 9003 | CB | ASP | F | 86 | 3.209 | 37.423 | 47.154 | 1.00 | 13.88 | C |
| ATOM | 9004 | CG | ASP | F | 86 | 2.438 | 36.375 | 46.425 | 1.00 | 15.74 | C |
| ATOM | 9005 | OD1 | ASP | F | 86 | 2.593 | 36.262 | 45.192 | 1.00 | 15.32 | O |
| ATOM | 9006 | OD2 | ASP | F | 86 | 1.641 | 35.707 | 47.076 | 1.00 | 16.04 | O |
| ATOM | 9007 | N | THR | F | 87 | 6.337 | 38.331 | 48.417 | 1.00 | 13.69 | N |
| ATOM | 9008 | CA | THR | F | 87 | 7.195 | 39.387 | 48.977 | 1.00 | 12.95 | C |
| ATOM | 9009 | C | THR | F | 87 | 7.876 | 40.173 | 47.865 | 1.00 | 11.77 | C |
| ATOM | 9010 | O | THR | F | 87 | 8.399 | 39.589 | 46.912 | 1.00 | 13.49 | O |
| ATOM | 9011 | CB | THR | F | 87 | 8.237 | 38.716 | 49.923 | 1.00 | 12.50 | C |
| ATOM | 9012 | OG1 | THR | F | 87 | 7.567 | 38.101 | 51.038 | 1.00 | 13.25 | O |
| ATOM | 9013 | CG2 | THR | F | 87 | 9.274 | 39.685 | 50.452 | 1.00 | 12.72 | C |
| ATOM | 9014 | N | GLY | F | 88 | 7.900 | 41.506 | 47.924 | 1.00 | 10.28 | N |
| ATOM | 9015 | CA | GLY | F | 88 | 8.563 | 42.189 | 46.838 | 1.00 | 8.97 | C |
| ATOM | 9016 | C | GLY | F | 88 | 8.360 | 43.674 | 47.060 | 1.00 | 7.13 | C |
| ATOM | 9017 | O | GLY | F | 88 | 7.610 | 44.061 | 47.957 | 1.00 | 6.22 | O |
| ATOM | 9018 | N | ILE | F | 89 | 9.016 | 44.416 | 46.208 | 1.00 | 7.75 | N |
| ATOM | 9019 | CA | ILE | F | 89 | 8.731 | 45.886 | 46.065 | 1.00 | 9.45 | C |
| ATOM | 9020 | C | ILE | F | 89 | 7.680 | 45.952 | 44.981 | 1.00 | 11.25 | C |
| ATOM | 9021 | O | ILE | F | 89 | 7.848 | 45.318 | 43.920 | 1.00 | 11.74 | O |
| ATOM | 9022 | CB | ILE | F | 89 | 9.952 | 46.606 | 45.679 | 1.00 | 9.47 | C |
| ATOM | 9023 | CG1 | ILE | F | 89 | 11.065 | 46.347 | 46.683 | 1.00 | 11.66 | C |

```
ATOM   9024  CG2 ILE F   89      9.686  48.177  45.538  1.00   8.07          C
ATOM   9025  CD1 ILE F   89     10.657  46.378  48.038  1.00  15.24          C
ATOM   9026  N   TYR F   90      6.560  46.609  45.284  1.00  10.51          N
ATOM   9027  CA  TYR F   90      5.420  46.716  44.382  1.00  10.45          C
ATOM   9028  C   TYR F   90      5.413  48.137  43.874  1.00  11.22          C
ATOM   9029  O   TYR F   90      5.329  49.063  44.687  1.00  12.65          O
ATOM   9030  CB  TYR F   90      4.113  46.409  45.115  1.00  11.12          C
ATOM   9031  CG  TYR F   90      3.900  44.924  45.246  1.00  11.53          C
ATOM   9032  CD1 TYR F   90      4.694  44.207  46.103  1.00  12.95          C
ATOM   9033  CD2 TYR F   90      2.962  44.262  44.455  1.00  11.39          C
ATOM   9034  CE1 TYR F   90      4.564  42.819  46.228  1.00  10.99          C
ATOM   9035  CE2 TYR F   90      2.810  42.849  44.555  1.00  11.93          C
ATOM   9036  CZ  TYR F   90      3.618  42.159  45.429  1.00  13.21          C
ATOM   9037  OH  TYR F   90      3.443  40.789  45.529  1.00  13.29          O
ATOM   9038  N   TYR F   91      5.525  48.285  42.556  1.00  13.63          N
ATOM   9039  CA  TYR F   91      5.514  49.591  41.866  1.00  13.44          C
ATOM   9040  C   TYR F   91      4.215  49.768  41.133  1.00  15.46          C
ATOM   9041  O   TYR F   91      3.716  48.832  40.460  1.00  13.83          O
ATOM   9042  CB  TYR F   91      6.626  49.652  40.802  1.00  14.26          C
ATOM   9043  CG  TYR F   91      8.054  49.638  41.309  1.00  14.04          C
ATOM   9044  CD1 TYR F   91      8.676  50.794  41.796  1.00  12.67          C
ATOM   9045  CD2 TYR F   91      8.782  48.476  41.300  1.00  15.28          C
ATOM   9046  CE1 TYR F   91      9.980  50.752  42.229  1.00  13.29          C
ATOM   9047  CE2 TYR F   91     10.062  48.417  41.765  1.00  13.95          C
ATOM   9048  CZ  TYR F   91     10.676  49.533  42.206  1.00  15.77          C
ATOM   9049  OH  TYR F   91     11.983  49.423  42.652  1.00  16.69          O
ATOM   9050  N   CYS F   92      3.680  50.982  41.205  1.00  15.63          N
ATOM   9051  CA  CYS F   92      2.795  51.465  40.161  1.00  15.82          C
ATOM   9052  C   CYS F   92      3.621  51.830  38.948  1.00  13.68          C
ATOM   9053  O   CYS F   92      4.583  52.564  39.023  1.00  12.88          O
ATOM   9054  CB  CYS F   92      2.000  52.686  40.585  1.00  17.54          C
ATOM   9055  SG  CYS F   92      0.817  52.315  41.786  1.00  23.84          S
ATOM   9056  N   PHE F   93      3.167  51.325  37.828  1.00  13.52          N
ATOM   9057  CA  PHE F   93      3.791  51.512  36.539  1.00  11.79          C
ATOM   9058  C   PHE F   93      2.666  51.908  35.595  1.00  12.27          C
ATOM   9059  O   PHE F   93      1.728  51.138  35.329  1.00  11.53          O
ATOM   9060  CB  PHE F   93      4.439  50.187  36.085  1.00  12.05          C
ATOM   9061  CG  PHE F   93      4.998  50.203  34.681  1.00  11.14          C
ATOM   9062  CD1 PHE F   93      5.900  51.157  34.288  1.00  13.82          C
ATOM   9063  CD2 PHE F   93      4.631  49.234  33.765  1.00  12.14          C
ATOM   9064  CE1 PHE F   93      6.440  51.181  32.997  1.00  12.55          C
ATOM   9065  CE2 PHE F   93      5.147  49.231  32.475  1.00  12.31          C
ATOM   9066  CZ  PHE F   93      6.054  50.198  32.072  1.00  10.75          C
ATOM   9067  N   LEU F   94      2.740  53.158  35.153  1.00  11.70          N
ATOM   9068  CA  LEU F   94      1.929  53.685  34.084  1.00  12.90          C
ATOM   9069  C   LEU F   94      2.616  53.370  32.763  1.00  13.71          C
ATOM   9070  O   LEU F   94      3.544  54.068  32.329  1.00  14.21          O
ATOM   9071  CB  LEU F   94      1.797  55.211  34.211  1.00  11.60          C
ATOM   9072  CG  LEU F   94      0.911  55.720  35.333  1.00  14.00          C
ATOM   9073  CD1 LEU F   94      1.408  55.294  36.674  1.00  13.10          C
ATOM   9074  CD2 LEU F   94      0.937  57.257  35.226  1.00  13.52          C
ATOM   9075  N   PRO F   95      2.121  52.351  32.084  1.00  12.19          N
ATOM   9076  CA  PRO F   95      2.834  51.859  30.921  1.00  13.80          C
ATOM   9077  C   PRO F   95      2.753  52.886  29.800  1.00  14.64          C
ATOM   9078  O   PRO F   95      1.648  53.420  29.536  1.00  15.62          O
ATOM   9079  CB  PRO F   95      2.068  50.591  30.562  1.00  11.93          C
ATOM   9080  CG  PRO F   95      1.128  50.362  31.691  1.00  10.80          C
ATOM   9081  CD  PRO F   95      0.861  51.625  32.308  1.00  13.84          C
ATOM   9082  N   MET F   96      3.880  53.197  29.152  1.00  14.17          N
ATOM   9083  CA  MET F   96      5.197  52.618  29.398  1.00  14.85          C
ATOM   9084  C   MET F   96      6.161  53.583  30.067  1.00  15.78          C
ATOM   9085  O   MET F   96      7.337  53.247  30.260  1.00  15.96          O
ATOM   9086  CB  MET F   96      5.788  52.221  28.055  1.00  13.98          C
ATOM   9087  CG  MET F   96      5.013  51.252  27.299  1.00  13.49          C
ATOM   9088  SD  MET F   96      5.036  49.611  28.067  1.00  15.35          S
```

| ATOM | 9089 | CE  | MET | F | 96  | 6.724 | 49.022 | 27.859 | 1.00 | 16.21 | C |
|------|------|-----|-----|---|-----|-------|--------|--------|------|-------|---|
| ATOM | 9090 | N   | ASP | F | 101 | 5.677 | 54.774 | 30.443 | 1.00 | 16.79 | N |
| ATOM | 9091 | CA  | ASP | F | 101 | 6.543 | 55.906 | 30.710 | 1.00 | 17.22 | C |
| ATOM | 9092 | C   | ASP | F | 101 | 6.928 | 56.186 | 32.168 | 1.00 | 15.31 | C |
| ATOM | 9093 | O   | ASP | F | 101 | 7.991 | 56.729 | 32.394 | 1.00 | 17.21 | O |
| ATOM | 9094 | CB  | ASP | F | 101 | 5.964 | 57.183 | 30.047 | 1.00 | 18.73 | C |
| ATOM | 9095 | CG  | ASP | F | 101 | 5.800 | 57.038 | 28.551 | 1.00 | 22.91 | C |
| ATOM | 9096 | OD1 | ASP | F | 101 | 6.763 | 56.639 | 27.880 | 1.00 | 27.90 | O |
| ATOM | 9097 | OD2 | ASP | F | 101 | 4.703 | 57.304 | 28.022 | 1.00 | 26.55 | O |
| ATOM | 9098 | N   | TYR | F | 102 | 6.087 | 55.819 | 33.135 | 1.00 | 16.28 | N |
| ATOM | 9099 | CA  | TYR | F | 102 | 6.263 | 56.206 | 34.533 | 1.00 | 16.73 | C |
| ATOM | 9100 | C   | TYR | F | 102 | 6.247 | 55.076 | 35.566 | 1.00 | 16.52 | C |
| ATOM | 9101 | O   | TYR | F | 102 | 5.415 | 54.185 | 35.517 | 1.00 | 13.78 | O |
| ATOM | 9102 | CB  | TYR | F | 102 | 5.181 | 57.211 | 34.926 | 1.00 | 20.61 | C |
| ATOM | 9103 | CG  | TYR | F | 102 | 5.137 | 58.371 | 33.991 | 1.00 | 20.01 | C |
| ATOM | 9104 | CD1 | TYR | F | 102 | 6.131 | 59.339 | 34.026 | 1.00 | 24.07 | C |
| ATOM | 9105 | CD2 | TYR | F | 102 | 4.151 | 58.478 | 33.033 | 1.00 | 21.96 | C |
| ATOM | 9106 | CE1 | TYR | F | 102 | 6.127 | 60.407 | 33.135 | 1.00 | 22.93 | C |
| ATOM | 9107 | CE2 | TYR | F | 102 | 4.126 | 59.555 | 32.140 | 1.00 | 22.52 | C |
| ATOM | 9108 | CZ  | TYR | F | 102 | 5.105 | 60.506 | 32.207 | 1.00 | 23.58 | C |
| ATOM | 9109 | OH  | TYR | F | 102 | 5.090 | 61.586 | 31.329 | 1.00 | 26.57 | O |
| ATOM | 9110 | N   | TRP | F | 103 | 7.171 | 55.169 | 36.513 | 1.00 | 16.70 | N |
| ATOM | 9111 | CA  | TRP | F | 103 | 7.263 | 54.259 | 37.640 | 1.00 | 16.20 | C |
| ATOM | 9112 | C   | TRP | F | 103 | 7.149 | 55.051 | 38.926 | 1.00 | 16.13 | C |
| ATOM | 9113 | O   | TRP | F | 103 | 7.693 | 56.178 | 39.036 | 1.00 | 17.24 | O |
| ATOM | 9114 | CB  | TRP | F | 103 | 8.631 | 53.574 | 37.649 | 1.00 | 14.65 | C |
| ATOM | 9115 | CG  | TRP | F | 103 | 8.891 | 52.704 | 36.556 | 1.00 | 13.47 | C |
| ATOM | 9116 | CD1 | TRP | F | 103 | 9.274 | 53.043 | 35.298 | 1.00 | 13.93 | C |
| ATOM | 9117 | CD2 | TRP | F | 103 | 8.842 | 51.277 | 36.593 | 1.00 | 12.87 | C |
| ATOM | 9118 | NE1 | TRP | F | 103 | 9.460 | 51.908 | 34.540 | 1.00 | 12.48 | N |
| ATOM | 9119 | CE2 | TRP | F | 103 | 9.187 | 50.814 | 35.304 | 1.00 | 13.83 | C |
| ATOM | 9120 | CE3 | TRP | F | 103 | 8.561 | 50.357 | 37.594 | 1.00 | 14.92 | C |
| ATOM | 9121 | CZ2 | TRP | F | 103 | 9.241 | 49.467 | 34.984 | 1.00 | 12.45 | C |
| ATOM | 9122 | CZ3 | TRP | F | 103 | 8.600 | 48.996 | 37.281 | 1.00 | 12.39 | C |
| ATOM | 9123 | CH2 | TRP | F | 103 | 8.953 | 48.571 | 35.989 | 1.00 | 14.05 | C |
| ATOM | 9124 | N   | GLY | F | 104 | 6.526 | 54.436 | 39.922 | 1.00 | 17.04 | N |
| ATOM | 9125 | CA  | GLY | F | 104 | 6.496 | 55.008 | 41.254 | 1.00 | 17.32 | C |
| ATOM | 9126 | C   | GLY | F | 104 | 7.767 | 54.739 | 42.034 | 1.00 | 17.23 | C |
| ATOM | 9127 | O   | GLY | F | 104 | 8.785 | 54.341 | 41.469 | 1.00 | 19.91 | O |
| ATOM | 9128 | N   | GLN | F | 105 | 7.722 | 54.993 | 43.334 | 1.00 | 16.54 | N |
| ATOM | 9129 | CA  | GLN | F | 105 | 8.880 | 54.788 | 44.160 | 1.00 | 17.70 | C |
| ATOM | 9130 | C   | GLN | F | 105 | 8.904 | 53.383 | 44.756 | 1.00 | 15.44 | C |
| ATOM | 9131 | O   | GLN | F | 105 | 9.917 | 52.984 | 45.281 | 1.00 | 15.83 | O |
| ATOM | 9132 | CB  | GLN | F | 105 | 8.986 | 55.832 | 45.270 | 1.00 | 21.25 | C |
| ATOM | 9133 | CG  | GLN | F | 105 | 8.323 | 55.463 | 46.589 | 1.00 | 25.45 | C |
| ATOM | 9134 | CD  | GLN | F | 105 | 6.942 | 56.032 | 46.758 | 1.00 | 29.89 | C |
| ATOM | 9135 | OE1 | GLN | F | 105 | 6.322 | 55.917 | 47.839 | 1.00 | 30.66 | O |
| ATOM | 9136 | NE2 | GLN | F | 105 | 6.442 | 56.677 | 45.700 | 1.00 | 32.28 | N |
| ATOM | 9137 | N   | GLY | F | 106 | 7.799 | 52.663 | 44.665 | 1.00 | 14.08 | N |
| ATOM | 9138 | CA  | GLY | F | 106 | 7.688 | 51.308 | 45.227 | 1.00 | 14.49 | C |
| ATOM | 9139 | C   | GLY | F | 106 | 7.191 | 51.306 | 46.663 | 1.00 | 14.66 | C |
| ATOM | 9140 | O   | GLY | F | 106 | 7.470 | 52.213 | 47.421 | 1.00 | 15.14 | O |
| ATOM | 9141 | N   | THR | F | 107 | 6.431 | 50.281 | 47.048 | 1.00 | 13.61 | N |
| ATOM | 9142 | CA  | THR | F | 107 | 6.126 | 50.054 | 48.458 | 1.00 | 11.93 | C |
| ATOM | 9143 | C   | THR | F | 107 | 6.406 | 48.575 | 48.693 | 1.00 | 10.79 | C |
| ATOM | 9144 | O   | THR | F | 107 | 6.100 | 47.747 | 47.841 | 1.00 | 11.15 | O |
| ATOM | 9145 | CB  | THR | F | 107 | 4.662 | 50.407 | 48.830 | 1.00 | 12.64 | C |
| ATOM | 9146 | OG1 | THR | F | 107 | 4.503 | 50.350 | 50.248 | 1.00 | 12.63 | O |
| ATOM | 9147 | CG2 | THR | F | 107 | 3.654 | 49.461 | 48.221 | 1.00 | 11.01 | C |
| ATOM | 9148 | N   | SER | F | 108 | 7.045 | 48.296 | 49.805 | 1.00 | 11.00 | N |
| ATOM | 9149 | CA  | SER | F | 108 | 7.546 | 46.942 | 50.084 | 1.00 | 11.27 | C |
| ATOM | 9150 | C   | SER | F | 108 | 6.484 | 46.186 | 50.815 | 1.00 | 11.48 | C |
| ATOM | 9151 | O   | SER | F | 108 | 5.902 | 46.675 | 51.786 | 1.00 | 11.17 | O |
| ATOM | 9152 | CB  | SER | F | 108 | 8.834 | 47.055 | 50.901 | 1.00 | 13.39 | C |
| ATOM | 9153 | OG  | SER | F | 108 | 9.393 | 45.802 | 51.209 | 1.00 | 18.11 | O |

| ATOM | 9154 | N   | VAL | F | 109 | 6.306  | 44.924 | 50.423 | 1.00 | 11.45 | N |
|------|------|-----|-----|---|-----|--------|--------|--------|------|-------|---|
| ATOM | 9155 | CA  | VAL | F | 109 | 5.322  | 44.093 | 51.015 | 1.00 | 11.33 | C |
| ATOM | 9156 | C   | VAL | F | 109 | 6.139  | 42.857 | 51.381 | 1.00 | 11.98 | C |
| ATOM | 9157 | O   | VAL | F | 109 | 6.761  | 42.249 | 50.503 | 1.00 | 12.59 | O |
| ATOM | 9158 | CB  | VAL | F | 109 | 4.239  | 43.690 | 50.000 | 1.00 | 12.88 | C |
| ATOM | 9159 | CG1 | VAL | F | 109 | 3.430  | 42.466 | 50.509 | 1.00 | 12.09 | C |
| ATOM | 9160 | CG2 | VAL | F | 109 | 3.321  | 44.863 | 49.630 | 1.00 | 13.30 | C |
| ATOM | 9161 | N   | THR | F | 110 | 6.163  | 42.556 | 52.665 | 1.00 | 12.90 | N |
| ATOM | 9162 | CA  | THR | F | 110 | 6.746  | 41.306 | 53.169 | 1.00 | 11.88 | C |
| ATOM | 9163 | C   | THR | F | 110 | 5.686  | 40.328 | 53.637 | 1.00 | 13.02 | C |
| ATOM | 9164 | O   | THR | F | 110 | 4.814  | 40.664 | 54.447 | 1.00 | 11.55 | O |
| ATOM | 9165 | CB  | THR | F | 110 | 7.733  | 41.612 | 54.339 | 1.00 | 13.42 | C |
| ATOM | 9166 | OG1 | THR | F | 110 | 8.831  | 42.408 | 53.876 | 1.00 | 13.53 | O |
| ATOM | 9167 | CG2 | THR | F | 110 | 8.277  | 40.335 | 54.906 | 1.00 | 14.13 | C |
| ATOM | 9168 | N   | VAL | F | 111 | 5.766  | 39.078 | 53.144 | 1.00 | 11.87 | N |
| ATOM | 9169 | CA  | VAL | F | 111 | 4.910  | 37.993 | 53.590 | 1.00 | 13.11 | C |
| ATOM | 9170 | C   | VAL | F | 111 | 5.801  | 36.977 | 54.336 | 1.00 | 13.39 | C |
| ATOM | 9171 | O   | VAL | F | 111 | 6.658  | 36.342 | 53.747 | 1.00 | 14.49 | O |
| ATOM | 9172 | CB  | VAL | F | 111 | 4.209  | 37.249 | 52.414 | 1.00 | 12.90 | C |
| ATOM | 9173 | CG1 | VAL | F | 111 | 3.187  | 36.238 | 52.945 | 1.00 | 11.24 | C |
| ATOM | 9174 | CG2 | VAL | F | 111 | 3.500  | 38.210 | 51.481 | 1.00 | 12.83 | C |
| ATOM | 9175 | N   | SER | F | 112 | 5.604  | 36.877 | 55.631 | 1.00 | 14.38 | N |
| ATOM | 9176 | CA  | SER | F | 112 | 6.492  | 36.134 | 56.518 | 1.00 | 15.17 | C |
| ATOM | 9177 | C   | SER | F | 112 | 5.775  | 35.768 | 57.807 | 1.00 | 16.33 | C |
| ATOM | 9178 | O   | SER | F | 112 | 4.878  | 36.491 | 58.275 | 1.00 | 17.57 | O |
| ATOM | 9179 | CB  | SER | F | 112 | 7.718  | 36.978 | 56.825 | 1.00 | 15.43 | C |
| ATOM | 9180 | OG  | SER | F | 112 | 8.534  | 36.396 | 57.819 | 1.00 | 14.25 | O |
| ATOM | 9181 | N   | SER | F | 113 | 6.231  | 34.675 | 58.411 | 1.00 | 18.18 | N |
| ATOM | 9182 | CA  | SER | F | 113 | 5.783  | 34.281 | 59.722 | 1.00 | 18.52 | C |
| ATOM | 9183 | C   | SER | F | 113 | 6.727  | 34.826 | 60.798 | 1.00 | 19.43 | C |
| ATOM | 9184 | O   | SER | F | 113 | 6.527  | 34.558 | 61.991 | 1.00 | 20.80 | O |
| ATOM | 9185 | CB  | SER | F | 113 | 5.732  | 32.769 | 59.790 | 1.00 | 20.05 | C |
| ATOM | 9186 | OG  | SER | F | 113 | 7.001  | 32.268 | 59.476 | 1.00 | 22.70 | O |
| ATOM | 9187 | N   | ALA | F | 114 | 7.753  | 35.584 | 60.388 | 1.00 | 19.89 | N |
| ATOM | 9188 | CA  | ALA | F | 114 | 8.591  | 36.312 | 61.340 | 1.00 | 18.90 | C |
| ATOM | 9189 | C   | ALA | F | 114 | 7.901  | 37.567 | 61.815 | 1.00 | 19.63 | C |
| ATOM | 9190 | O   | ALA | F | 114 | 6.988  | 38.078 | 61.180 | 1.00 | 18.92 | O |
| ATOM | 9191 | CB  | ALA | F | 114 | 9.926  | 36.637 | 60.754 | 1.00 | 18.63 | C |
| ATOM | 9192 | N   | LYS | F | 115 | 8.379  | 38.104 | 62.921 | 1.00 | 20.77 | N |
| ATOM | 9193 | CA  | LYS | F | 115 | 7.728  | 39.279 | 63.475 | 1.00 | 20.97 | C |
| ATOM | 9194 | C   | LYS | F | 115 | 8.537  | 40.518 | 63.170 | 1.00 | 17.94 | C |
| ATOM | 9195 | O   | LYS | F | 115 | 9.775  | 40.485 | 63.133 | 1.00 | 15.96 | O |
| ATOM | 9196 | CB  | LYS | F | 115 | 7.467  | 39.101 | 64.979 | 1.00 | 24.46 | C |
| ATOM | 9197 | CG  | LYS | F | 115 | 6.040  | 38.566 | 65.268 | 1.00 | 28.01 | C |
| ATOM | 9198 | CD  | LYS | F | 115 | 5.695  | 37.252 | 64.465 | 1.00 | 28.82 | C |
| ATOM | 9199 | CE  | LYS | F | 115 | 4.215  | 37.180 | 63.987 | 1.00 | 29.54 | C |
| ATOM | 9200 | NZ  | LYS | F | 115 | 3.878  | 35.944 | 63.132 | 1.00 | 28.63 | N |
| ATOM | 9201 | N   | THR | F | 116 | 7.813  | 41.618 | 62.962 | 1.00 | 14.20 | N |
| ATOM | 9202 | CA  | THR | F | 116 | 8.435  | 42.911 | 62.811 | 1.00 | 13.51 | C |
| ATOM | 9203 | C   | THR | F | 116 | 9.168  | 43.328 | 64.106 | 1.00 | 12.80 | C |
| ATOM | 9204 | O   | THR | F | 116 | 8.626  | 43.222 | 65.233 | 1.00 | 11.67 | O |
| ATOM | 9205 | CB  | THR | F | 116 | 7.377  | 43.917 | 62.388 | 1.00 | 11.25 | C |
| ATOM | 9206 | OG1 | THR | F | 116 | 6.868  | 43.563 | 61.084 | 1.00 | 12.51 | O |
| ATOM | 9207 | CG2 | THR | F | 116 | 7.918  | 45.365 | 62.341 | 1.00 | 14.65 | C |
| ATOM | 9208 | N   | THR | F | 117 | 10.390 | 43.825 | 63.934 | 1.00 | 10.39 | N |
| ATOM | 9209 | CA  | THR | F | 117 | 11.197 | 44.352 | 65.044 | 1.00 | 11.32 | C |
| ATOM | 9210 | C   | THR | F | 117 | 11.810 | 45.651 | 64.559 | 1.00 | 9.61  | C |
| ATOM | 9211 | O   | THR | F | 117 | 12.399 | 45.664 | 63.518 | 1.00 | 7.31  | O |
| ATOM | 9212 | CB  | THR | F | 117 | 12.333 | 43.421 | 65.396 | 1.00 | 11.42 | C |
| ATOM | 9213 | OG1 | THR | F | 117 | 11.774 | 42.137 | 65.722 | 1.00 | 15.00 | O |
| ATOM | 9214 | CG2 | THR | F | 117 | 13.101 | 43.940 | 66.628 | 1.00 | 14.72 | C |
| ATOM | 9215 | N   | PRO | F | 118 | 11.724 | 46.703 | 65.363 | 1.00 | 8.55  | N |
| ATOM | 9216 | CA  | PRO | F | 118 | 12.299 | 47.938 | 64.840 | 1.00 | 9.51  | C |
| ATOM | 9217 | C   | PRO | F | 118 | 13.845 | 47.978 | 65.049 | 1.00 | 10.66 | C |
| ATOM | 9218 | O   | PRO | F | 118 | 14.356 | 47.218 | 65.877 | 1.00 | 13.88 | O |

| ATOM | 9219 | CB  | PRO | F | 118 | 11.565 | 48.991 | 65.678 | 1.00 | 7.62  | C |
|------|------|-----|-----|---|-----|--------|--------|--------|------|-------|---|
| ATOM | 9220 | CG  | PRO | F | 118 | 11.443 | 48.428 | 66.977 | 1.00 | 7.72  | C |
| ATOM | 9221 | CD  | PRO | F | 118 | 11.164 | 46.889 | 66.703 | 1.00 | 8.75  | C |
| ATOM | 9222 | N   | PRO | F | 119 | 14.600 | 48.805 | 64.264 | 1.00 | 12.22 | N |
| ATOM | 9223 | CA  | PRO | F | 119 | 16.044 | 48.898 | 64.455 | 1.00 | 12.88 | C |
| ATOM | 9224 | C   | PRO | F | 119 | 16.497 | 49.560 | 65.729 | 1.00 | 14.20 | C |
| ATOM | 9225 | O   | PRO | F | 119 | 15.795 | 50.411 | 66.274 | 1.00 | 12.31 | O |
| ATOM | 9226 | CB  | PRO | F | 119 | 16.521 | 49.779 | 63.274 | 1.00 | 13.28 | C |
| ATOM | 9227 | CG  | PRO | F | 119 | 15.329 | 50.479 | 62.770 | 1.00 | 12.55 | C |
| ATOM | 9228 | CD  | PRO | F | 119 | 14.149 | 49.567 | 63.100 | 1.00 | 11.85 | C |
| ATOM | 9229 | N   | SER | F | 120 | 17.677 | 49.131 | 66.180 | 1.00 | 12.81 | N |
| ATOM | 9230 | CA  | SER | F | 120 | 18.458 | 49.811 | 67.175 | 1.00 | 10.27 | C |
| ATOM | 9231 | C   | SER | F | 120 | 19.514 | 50.526 | 66.406 | 1.00 | 11.33 | C |
| ATOM | 9232 | O   | SER | F | 120 | 20.195 | 49.967 | 65.577 | 1.00 | 10.75 | O |
| ATOM | 9233 | CB  | SER | F | 120 | 19.096 | 48.818 | 68.150 | 1.00 | 12.77 | C |
| ATOM | 9234 | OG  | SER | F | 120 | 18.090 | 48.144 | 68.888 | 1.00 | 10.99 | O |
| ATOM | 9235 | N   | VAL | F | 121 | 19.665 | 51.813 | 66.647 | 1.00 | 11.07 | N |
| ATOM | 9236 | CA  | VAL | F | 121 | 20.631 | 52.571 | 65.897 | 1.00 | 10.07 | C |
| ATOM | 9237 | C   | VAL | F | 121 | 21.767 | 52.972 | 66.838 | 1.00 | 11.40 | C |
| ATOM | 9238 | O   | VAL | F | 121 | 21.511 | 53.495 | 67.897 | 1.00 | 13.02 | O |
| ATOM | 9239 | CB  | VAL | F | 121 | 19.987 | 53.866 | 65.360 | 1.00 | 10.67 | C |
| ATOM | 9240 | CG1 | VAL | F | 121 | 20.947 | 54.588 | 64.490 | 1.00 | 9.54  | C |
| ATOM | 9241 | CG2 | VAL | F | 121 | 18.693 | 53.523 | 64.573 | 1.00 | 12.36 | C |
| ATOM | 9242 | N   | TYR | F | 122 | 23.002 | 52.723 | 66.443 | 1.00 | 11.91 | N |
| ATOM | 9243 | CA  | TYR | F | 122 | 24.167 | 52.996 | 67.271 | 1.00 | 12.39 | C |
| ATOM | 9244 | C   | TYR | F | 122 | 25.109 | 53.948 | 66.562 | 1.00 | 11.64 | C |
| ATOM | 9245 | O   | TYR | F | 122 | 25.384 | 53.799 | 65.372 | 1.00 | 11.71 | O |
| ATOM | 9246 | CB  | TYR | F | 122 | 24.949 | 51.721 | 67.599 | 1.00 | 12.45 | C |
| ATOM | 9247 | CG  | TYR | F | 122 | 24.141 | 50.656 | 68.286 | 1.00 | 11.12 | C |
| ATOM | 9248 | CD1 | TYR | F | 122 | 23.482 | 50.921 | 69.463 | 1.00 | 14.18 | C |
| ATOM | 9249 | CD2 | TYR | F | 122 | 24.027 | 49.389 | 67.734 | 1.00 | 14.37 | C |
| ATOM | 9250 | CE1 | TYR | F | 122 | 22.716 | 49.950 | 70.091 | 1.00 | 11.54 | C |
| ATOM | 9251 | CE2 | TYR | F | 122 | 23.299 | 48.417 | 68.353 | 1.00 | 13.43 | C |
| ATOM | 9252 | CZ  | TYR | F | 122 | 22.643 | 48.702 | 69.537 | 1.00 | 14.35 | C |
| ATOM | 9253 | OH  | TYR | F | 122 | 21.894 | 47.730 | 70.159 | 1.00 | 10.31 | O |
| ATOM | 9254 | N   | PRO | F | 123 | 25.653 | 54.921 | 67.298 | 1.00 | 14.22 | N |
| ATOM | 9255 | CA  | PRO | F | 123 | 26.568 | 55.840 | 66.625 | 1.00 | 13.86 | C |
| ATOM | 9256 | C   | PRO | F | 123 | 27.945 | 55.219 | 66.411 | 1.00 | 15.86 | C |
| ATOM | 9257 | O   | PRO | F | 123 | 28.439 | 54.469 | 67.276 | 1.00 | 18.55 | O |
| ATOM | 9258 | CB  | PRO | F | 123 | 26.628 | 57.026 | 67.607 | 1.00 | 14.65 | C |
| ATOM | 9259 | CG  | PRO | F | 123 | 26.482 | 56.334 | 68.959 | 1.00 | 14.81 | C |
| ATOM | 9260 | CD  | PRO | F | 123 | 25.487 | 55.242 | 68.726 | 1.00 | 14.06 | C |
| ATOM | 9261 | N   | LEU | F | 124 | 28.554 | 55.507 | 65.263 | 1.00 | 15.47 | N |
| ATOM | 9262 | CA  | LEU | F | 124 | 29.923 | 55.089 | 64.964 | 1.00 | 16.42 | C |
| ATOM | 9263 | C   | LEU | F | 124 | 30.803 | 56.329 | 64.920 | 1.00 | 17.88 | C |
| ATOM | 9264 | O   | LEU | F | 124 | 30.853 | 57.005 | 63.911 | 1.00 | 17.60 | O |
| ATOM | 9265 | CB  | LEU | F | 124 | 30.010 | 54.367 | 63.639 | 1.00 | 16.39 | C |
| ATOM | 9266 | CG  | LEU | F | 124 | 29.182 | 53.079 | 63.542 | 1.00 | 14.44 | C |
| ATOM | 9267 | CD1 | LEU | F | 124 | 29.119 | 52.584 | 62.155 | 1.00 | 16.02 | C |
| ATOM | 9268 | CD2 | LEU | F | 124 | 29.735 | 52.014 | 64.466 | 1.00 | 17.46 | C |
| ATOM | 9269 | N   | ALA | F | 125 | 31.488 | 56.557 | 66.030 | 1.00 | 19.90 | N |
| ATOM | 9270 | CA  | ALA | F | 125 | 32.370 | 57.715 | 66.218 | 1.00 | 22.29 | C |
| ATOM | 9271 | C   | ALA | F | 125 | 33.799 | 57.235 | 66.384 | 1.00 | 23.65 | C |
| ATOM | 9272 | O   | ALA | F | 125 | 34.053 | 56.151 | 66.957 | 1.00 | 24.75 | O |
| ATOM | 9273 | CB  | ALA | F | 125 | 31.933 | 58.515 | 67.397 | 1.00 | 21.67 | C |
| ATOM | 9274 | N   | PRO | F | 126 | 34.761 | 58.031 | 65.890 | 1.00 | 26.53 | N |
| ATOM | 9275 | CA  | PRO | F | 126 | 36.160 | 57.609 | 65.942 | 1.00 | 27.93 | C |
| ATOM | 9276 | C   | PRO | F | 126 | 36.656 | 57.415 | 67.373 | 1.00 | 29.30 | C |
| ATOM | 9277 | O   | PRO | F | 126 | 36.360 | 58.239 | 68.226 | 1.00 | 31.04 | O |
| ATOM | 9278 | CB  | PRO | F | 126 | 36.887 | 58.773 | 65.282 | 1.00 | 27.33 | C |
| ATOM | 9279 | CG  | PRO | F | 126 | 35.846 | 59.430 | 64.458 | 1.00 | 27.83 | C |
| ATOM | 9280 | CD  | PRO | F | 126 | 34.624 | 59.345 | 65.259 | 1.00 | 26.11 | C |
| ATOM | 9281 | N   | GLY | F | 127 | 37.353 | 56.309 | 67.628 | 1.00 | 30.45 | N |
| ATOM | 9282 | CA  | GLY | F | 127 | 38.053 | 56.111 | 68.896 | 1.00 | 30.92 | C |
| ATOM | 9283 | C   | GLY | F | 127 | 39.248 | 57.048 | 69.074 | 1.00 | 32.79 | C |

| ATOM | 9284 | O | GLY | F | 127 | 39.704 | 57.706 | 68.121 | 1.00 | 33.21 | O |
|------|------|-----|-----|---|-----|--------|--------|--------|------|-------|---|
| ATOM | 9285 | N | SER | F | 136 | 42.635 | 64.892 | 56.892 | 1.00 | 33.10 | N |
| ATOM | 9286 | CA | SER | F | 136 | 41.883 | 65.828 | 56.064 | 1.00 | 32.95 | C |
| ATOM | 9287 | C | SER | F | 136 | 40.366 | 65.587 | 56.087 | 1.00 | 33.11 | C |
| ATOM | 9288 | O | SER | F | 136 | 39.565 | 66.545 | 56.049 | 1.00 | 34.08 | O |
| ATOM | 9289 | CB | SER | F | 136 | 42.397 | 65.755 | 54.630 | 1.00 | 33.98 | C |
| ATOM | 9290 | OG | SER | F | 136 | 41.379 | 66.081 | 53.701 | 1.00 | 36.34 | O |
| ATOM | 9291 | N | MET | F | 137 | 39.970 | 64.308 | 56.131 | 1.00 | 32.25 | N |
| ATOM | 9292 | CA | MET | F | 137 | 38.548 | 63.922 | 56.204 | 1.00 | 29.80 | C |
| ATOM | 9293 | C | MET | F | 137 | 38.346 | 63.180 | 57.501 | 1.00 | 27.62 | C |
| ATOM | 9294 | O | MET | F | 137 | 39.301 | 62.673 | 58.064 | 1.00 | 27.86 | O |
| ATOM | 9295 | CB | MET | F | 137 | 38.170 | 62.988 | 55.044 | 1.00 | 30.68 | C |
| ATOM | 9296 | CG | MET | F | 137 | 38.463 | 63.544 | 53.680 | 1.00 | 32.04 | C |
| ATOM | 9297 | SD | MET | F | 137 | 37.587 | 65.092 | 53.459 | 1.00 | 33.64 | S |
| ATOM | 9298 | CE | MET | F | 137 | 36.035 | 64.615 | 52.731 | 1.00 | 32.36 | C |
| ATOM | 9299 | N | VAL | F | 138 | 37.119 | 63.146 | 57.987 | 1.00 | 24.61 | N |
| ATOM | 9300 | CA | VAL | F | 138 | 36.772 | 62.238 | 59.087 | 1.00 | 24.04 | C |
| ATOM | 9301 | C | VAL | F | 138 | 35.595 | 61.379 | 58.614 | 1.00 | 21.14 | C |
| ATOM | 9302 | O | VAL | F | 138 | 34.674 | 61.887 | 57.979 | 1.00 | 21.05 | O |
| ATOM | 9303 | CB | VAL | F | 138 | 36.434 | 62.999 | 60.390 | 1.00 | 23.67 | C |
| ATOM | 9304 | CG1 | VAL | F | 138 | 35.318 | 64.002 | 60.182 | 1.00 | 24.44 | C |
| ATOM | 9305 | CG2 | VAL | F | 138 | 36.096 | 62.047 | 61.539 | 1.00 | 25.02 | C |
| ATOM | 9306 | N | THR | F | 139 | 35.649 | 60.081 | 58.900 | 1.00 | 20.77 | N |
| ATOM | 9307 | CA | THR | F | 139 | 34.540 | 59.205 | 58.568 | 1.00 | 18.97 | C |
| ATOM | 9308 | C | THR | F | 139 | 33.778 | 58.877 | 59.852 | 1.00 | 17.30 | C |
| ATOM | 9309 | O | THR | F | 139 | 34.381 | 58.514 | 60.867 | 1.00 | 18.16 | O |
| ATOM | 9310 | CB | THR | F | 139 | 35.010 | 57.925 | 57.836 | 1.00 | 18.47 | C |
| ATOM | 9311 | OG1 | THR | F | 139 | 35.586 | 58.282 | 56.576 | 1.00 | 17.80 | O |
| ATOM | 9312 | CG2 | THR | F | 139 | 33.855 | 56.998 | 57.566 | 1.00 | 19.63 | C |
| ATOM | 9313 | N | LEU | F | 140 | 32.464 | 59.086 | 59.802 | 1.00 | 17.01 | N |
| ATOM | 9314 | CA | LEU | F | 140 | 31.532 | 58.716 | 60.868 | 1.00 | 16.13 | C |
| ATOM | 9315 | C | LEU | F | 140 | 30.563 | 57.668 | 60.295 | 1.00 | 15.23 | C |
| ATOM | 9316 | O | LEU | F | 140 | 30.609 | 57.368 | 59.102 | 1.00 | 13.47 | O |
| ATOM | 9317 | CB | LEU | F | 140 | 30.775 | 59.934 | 61.378 | 1.00 | 17.05 | C |
| ATOM | 9318 | CG | LEU | F | 140 | 31.648 | 61.164 | 61.699 | 1.00 | 17.42 | C |
| ATOM | 9319 | CD1 | LEU | F | 140 | 30.815 | 62.406 | 61.821 | 1.00 | 17.74 | C |
| ATOM | 9320 | CD2 | LEU | F | 140 | 32.428 | 60.963 | 62.948 | 1.00 | 19.54 | C |
| ATOM | 9321 | N | GLY | F | 141 | 29.689 | 57.127 | 61.139 | 1.00 | 14.17 | N |
| ATOM | 9322 | CA | GLY | F | 141 | 28.625 | 56.246 | 60.652 | 1.00 | 15.29 | C |
| ATOM | 9323 | C | GLY | F | 141 | 27.533 | 55.923 | 61.645 | 1.00 | 13.44 | C |
| ATOM | 9324 | O | GLY | F | 141 | 27.558 | 56.365 | 62.793 | 1.00 | 13.48 | O |
| ATOM | 9325 | N | CYS | F | 142 | 26.534 | 55.171 | 61.177 | 1.00 | 13.89 | N |
| ATOM | 9326 | CA | CYS | F | 142 | 25.515 | 54.611 | 62.043 | 1.00 | 14.31 | C |
| ATOM | 9327 | C | CYS | F | 142 | 25.450 | 53.135 | 61.788 | 1.00 | 12.50 | C |
| ATOM | 9328 | O | CYS | F | 142 | 25.460 | 52.743 | 60.639 | 1.00 | 11.99 | O |
| ATOM | 9329 | CB | CYS | F | 142 | 24.150 | 55.226 | 61.762 | 1.00 | 19.05 | C |
| ATOM | 9330 | SG | CYS | F | 142 | 23.989 | 56.728 | 62.736 | 1.00 | 26.79 | S |
| ATOM | 9331 | N | LEU | F | 143 | 25.439 | 52.351 | 62.852 | 1.00 | 11.73 | N |
| ATOM | 9332 | CA | LEU | F | 143 | 25.133 | 50.911 | 62.747 | 1.00 | 11.98 | C |
| ATOM | 9333 | C | LEU | F | 143 | 23.652 | 50.681 | 63.073 | 1.00 | 11.24 | C |
| ATOM | 9334 | O | LEU | F | 143 | 23.185 | 51.021 | 64.161 | 1.00 | 12.83 | O |
| ATOM | 9335 | CB | LEU | F | 143 | 25.975 | 50.119 | 63.740 | 1.00 | 11.70 | C |
| ATOM | 9336 | CG | LEU | F | 143 | 25.852 | 48.579 | 63.751 | 1.00 | 11.09 | C |
| ATOM | 9337 | CD1 | LEU | F | 143 | 26.226 | 48.046 | 62.398 | 1.00 | 9.63 | C |
| ATOM | 9338 | CD2 | LEU | F | 143 | 26.780 | 48.070 | 64.783 | 1.00 | 13.31 | C |
| ATOM | 9339 | N | VAL | F | 144 | 22.957 | 49.979 | 62.181 | 1.00 | 13.02 | N |
| ATOM | 9340 | CA | VAL | F | 144 | 21.515 | 49.822 | 62.240 | 1.00 | 13.21 | C |
| ATOM | 9341 | C | VAL | F | 144 | 21.265 | 48.326 | 62.368 | 1.00 | 14.25 | C |
| ATOM | 9342 | O | VAL | F | 144 | 21.442 | 47.591 | 61.418 | 1.00 | 13.92 | O |
| ATOM | 9343 | CB | VAL | F | 144 | 20.836 | 50.367 | 60.986 | 1.00 | 14.03 | C |
| ATOM | 9344 | CG1 | VAL | F | 144 | 19.298 | 50.140 | 61.035 | 1.00 | 14.15 | C |
| ATOM | 9345 | CG2 | VAL | F | 144 | 21.177 | 51.843 | 60.849 | 1.00 | 14.29 | C |
| ATOM | 9346 | N | LYS | F | 145 | 20.825 | 47.939 | 63.550 | 1.00 | 14.01 | N |
| ATOM | 9347 | CA | LYS | F | 145 | 20.887 | 46.534 | 63.954 | 1.00 | 14.60 | C |
| ATOM | 9348 | C | LYS | F | 145 | 19.557 | 45.985 | 64.484 | 1.00 | 13.87 | C |

```
ATOM   9349  O    LYS F 145    18.739  46.713  65.069  1.00 12.17       O
ATOM   9350  CB   LYS F 145    21.971  46.471  65.022  1.00 16.27       C
ATOM   9351  CG   LYS F 145    22.569  45.174  65.159  1.00 19.34       C
ATOM   9352  CD   LYS F 145    23.904  45.258  65.867  1.00 18.86       C
ATOM   9353  CE   LYS F 145    24.221  43.886  66.340  1.00 20.48       C
ATOM   9354  NZ   LYS F 145    24.018  42.947  65.224  1.00 21.24       N
ATOM   9355  N    GLY F 146    19.337  44.672  64.321  1.00 11.78       N
ATOM   9356  CA   GLY F 146    18.246  44.024  65.049  1.00 12.67       C
ATOM   9357  C    GLY F 146    16.829  44.187  64.498  1.00 12.01       C
ATOM   9358  O    GLY F 146    15.842  44.019  65.238  1.00 13.85       O
ATOM   9359  N    TYR F 147    16.714  44.539  63.219  1.00 11.50       N
ATOM   9360  CA   TYR F 147    15.407  44.788  62.620  1.00 10.89       C
ATOM   9361  C    TYR F 147    14.923  43.740  61.616  1.00 11.16       C
ATOM   9362  O    TYR F 147    15.710  43.033  60.941  1.00 11.44       O
ATOM   9363  CB   TYR F 147    15.328  46.210  61.984  1.00 11.00       C
ATOM   9364  CG   TYR F 147    16.113  46.381  60.719  1.00 10.10       C
ATOM   9365  CD1  TYR F 147    17.473  46.709  60.752  1.00  9.74       C
ATOM   9366  CD2  TYR F 147    15.507  46.244  59.475  1.00 11.06       C
ATOM   9367  CE1  TYR F 147    18.196  46.822  59.588  1.00 10.06       C
ATOM   9368  CE2  TYR F 147    16.228  46.344  58.320  1.00 10.90       C
ATOM   9369  CZ   TYR F 147    17.558  46.615  58.374  1.00  8.94       C
ATOM   9370  OH   TYR F 147    18.243  46.784  57.213  1.00 12.33       O
ATOM   9371  N    PHE F 148    13.598  43.715  61.487  1.00 11.36       N
ATOM   9372  CA   PHE F 148    12.898  42.918  60.512  1.00 13.09       C
ATOM   9373  C    PHE F 148    11.540  43.542  60.192  1.00 14.00       C
ATOM   9374  O    PHE F 148    10.873  44.110  61.091  1.00 15.18       O
ATOM   9375  CB   PHE F 148    12.767  41.474  61.023  1.00 14.01       C
ATOM   9376  CG   PHE F 148    12.253  40.537  59.997  1.00 13.51       C
ATOM   9377  CD1  PHE F 148    13.113  39.950  59.089  1.00 16.24       C
ATOM   9378  CD2  PHE F 148    10.897  40.291  59.881  1.00 15.69       C
ATOM   9379  CE1  PHE F 148    12.620  39.116  58.095  1.00 15.46       C
ATOM   9380  CE2  PHE F 148    10.406  39.451  58.916  1.00 14.17       C
ATOM   9381  CZ   PHE F 148    11.262  38.865  58.013  1.00 15.31       C
ATOM   9382  N    PRO F 149    11.122  43.516  58.920  1.00 12.93       N
ATOM   9383  CA   PRO F 149    11.796  43.205  57.692  1.00 12.05       C
ATOM   9384  C    PRO F 149    12.533  44.374  57.098  1.00 13.13       C
ATOM   9385  O    PRO F 149    12.433  45.491  57.578  1.00 12.38       O
ATOM   9386  CB   PRO F 149    10.638  42.823  56.784  1.00 14.13       C
ATOM   9387  CG   PRO F 149     9.582  43.756  57.177  1.00 11.23       C
ATOM   9388  CD   PRO F 149     9.705  43.801  58.666  1.00 12.72       C
ATOM   9389  N    GLU F 150    13.223  44.108  56.004  1.00 12.68       N
ATOM   9390  CA   GLU F 150    13.685  45.173  55.124  1.00 12.88       C
ATOM   9391  C    GLU F 150    12.451  45.797  54.512  1.00 14.00       C
ATOM   9392  O    GLU F 150    11.427  45.130  54.415  1.00 13.81       O
ATOM   9393  CB   GLU F 150    14.547  44.577  54.017  1.00 12.18       C
ATOM   9394  CG   GLU F 150    15.979  44.393  54.419  1.00 13.91       C
ATOM   9395  CD   GLU F 150    16.788  45.591  54.087  1.00 18.13       C
ATOM   9396  OE1  GLU F 150    17.379  45.563  52.978  1.00 22.47       O
ATOM   9397  OE2  GLU F 150    16.808  46.588  54.855  1.00 18.45       O
ATOM   9398  N    PRO F 151    12.547  47.052  54.044  1.00 15.67       N
ATOM   9399  CA   PRO F 151    13.670  47.990  54.060  1.00 15.63       C
ATOM   9400  C    PRO F 151    13.765  48.984  55.246  1.00 14.09       C
ATOM   9401  O    PRO F 151    12.795  49.196  56.004  1.00 12.49       O
ATOM   9402  CB   PRO F 151    13.445  48.773  52.755  1.00 15.77       C
ATOM   9403  CG   PRO F 151    11.963  48.919  52.696  1.00 15.14       C
ATOM   9404  CD   PRO F 151    11.429  47.580  53.252  1.00 16.38       C
ATOM   9405  N    VAL F 152    14.966  49.526  55.434  1.00 15.48       N
ATOM   9406  CA   VAL F 152    15.125  50.827  56.149  1.00 17.63       C
ATOM   9407  C    VAL F 152    15.642  51.861  55.172  1.00 18.38       C
ATOM   9408  O    VAL F 152    16.286  51.515  54.174  1.00 19.13       O
ATOM   9409  CB   VAL F 152    16.048  50.786  57.422  1.00 18.09       C
ATOM   9410  CG1  VAL F 152    15.402  50.018  58.550  1.00 18.46       C
ATOM   9411  CG2  VAL F 152    17.430  50.282  57.097  1.00 17.31       C
ATOM   9412  N    THR F 153    15.354  53.134  55.427  1.00 20.49       N
ATOM   9413  CA   THR F 153    16.014  54.212  54.675  1.00 20.88       C
```

652

```
ATOM   9414  C   THR F 153     16.842 55.032 55.638  1.00 20.90           C
ATOM   9415  O   THR F 153     16.342 55.473 56.680  1.00 22.53           O
ATOM   9416  CB  THR F 153     15.037 55.184 53.977  1.00 22.36           C
ATOM   9417  OG1 THR F 153     14.154 55.736 54.937  1.00 28.94           O
ATOM   9418  CG2 THR F 153     14.218 54.494 52.923  1.00 24.11           C
ATOM   9419  N   VAL F 154     18.097 55.255 55.283  1.00 19.65           N
ATOM   9420  CA  VAL F 154     18.974 56.043 56.111  1.00 18.98           C
ATOM   9421  C   VAL F 154     19.326 57.312 55.390  1.00 19.05           C
ATOM   9422  O   VAL F 154     19.650 57.282 54.221  1.00 19.84           O
ATOM   9423  CB  VAL F 154     20.256 55.284 56.410  1.00 20.27           C
ATOM   9424  CG1 VAL F 154     21.159 56.091 57.250  1.00 19.14           C
ATOM   9425  CG2 VAL F 154     19.915 53.939 57.088  1.00 20.73           C
ATOM   9426  N   THR F 155     19.304 58.417 56.120  1.00 18.60           N
ATOM   9427  CA  THR F 155     19.762 59.702 55.608  1.00 18.79           C
ATOM   9428  C   THR F 155     20.668 60.307 56.657  1.00 17.76           C
ATOM   9429  O   THR F 155     20.775 59.798 57.797  1.00 15.69           O
ATOM   9430  CB  THR F 155     18.587 60.660 55.281  1.00 19.48           C
ATOM   9431  OG1 THR F 155     17.731 60.798 56.419  1.00 20.11           O
ATOM   9432  CG2 THR F 155     17.772 60.141 54.107  1.00 20.71           C
ATOM   9433  N   TRP F 156     21.319 61.413 56.274  1.00 17.77           N
ATOM   9434  CA  TRP F 156     22.249 62.112 57.139  1.00 18.52           C
ATOM   9435  C   TRP F 156     21.796 63.550 57.157  1.00 19.18           C
ATOM   9436  O   TRP F 156     21.542 64.132 56.095  1.00 17.45           O
ATOM   9437  CB  TRP F 156     23.679 61.997 56.623  1.00 17.59           C
ATOM   9438  CG  TRP F 156     24.226 60.623 56.859  1.00 17.19           C
ATOM   9439  CD1 TRP F 156     24.236 59.590 55.980  1.00 17.26           C
ATOM   9440  CD2 TRP F 156     24.793 60.132 58.080  1.00 16.53           C
ATOM   9441  NE1 TRP F 156     24.783 58.469 56.573  1.00 16.76           N
ATOM   9442  CE2 TRP F 156     25.133 58.780 57.865  1.00 15.05           C
ATOM   9443  CE3 TRP F 156     25.044 60.706 59.339  1.00 15.71           C
ATOM   9444  CZ2 TRP F 156     25.700 58.002 58.850  1.00 15.95           C
ATOM   9445  CZ3 TRP F 156     25.629 59.931 60.319  1.00 17.20           C
ATOM   9446  CH2 TRP F 156     25.959 58.586 60.058  1.00 17.58           C
ATOM   9447  N   ASN F 157     21.641 64.078 58.361  1.00 21.49           N
ATOM   9448  CA  ASN F 157     21.112 65.438 58.564  1.00 23.42           C
ATOM   9449  C   ASN F 157     19.864 65.674 57.722  1.00 25.07           C
ATOM   9450  O   ASN F 157     19.777 66.631 56.939  1.00 25.29           O
ATOM   9451  CB  ASN F 157     22.178 66.492 58.273  1.00 23.25           C
ATOM   9452  CG  ASN F 157     23.341 66.437 59.250  1.00 24.02           C
ATOM   9453  OD1 ASN F 157     23.303 65.714 60.245  1.00 19.87           O
ATOM   9454  ND2 ASN F 157     24.382 67.250 58.989  1.00 24.83           N
ATOM   9455  N   SER F 158     18.907 64.763 57.878  1.00 25.33           N
ATOM   9456  CA  SER F 158     17.627 64.858 57.211  1.00 25.69           C
ATOM   9457  C   SER F 158     17.734 65.043 55.690  1.00 27.10           C
ATOM   9458  O   SER F 158     16.828 65.594 55.075  1.00 28.64           O
ATOM   9459  CB  SER F 158     16.790 65.978 57.869  1.00 26.32           C
ATOM   9460  OG  SER F 158     16.651 65.780 59.286  1.00 24.96           O
ATOM   9461  N   GLY F 159     18.809 64.542 55.075  1.00 27.45           N
ATOM   9462  CA  GLY F 159     18.996 64.621 53.629  1.00 27.61           C
ATOM   9463  C   GLY F 159     19.933 65.745 53.171  1.00 28.37           C
ATOM   9464  O   GLY F 159     20.294 65.815 52.004  1.00 29.30           O
ATOM   9465  N   SER F 160     20.344 66.610 54.091  1.00 29.07           N
ATOM   9466  CA  SER F 160     21.307 67.670 53.785  1.00 28.89           C
ATOM   9467  C   SER F 160     22.716 67.165 53.557  1.00 28.89           C
ATOM   9468  O   SER F 160     23.517 67.831 52.894  1.00 30.51           O
ATOM   9469  CB  SER F 160     21.346 68.687 54.917  1.00 28.70           C
ATOM   9470  OG  SER F 160     20.128 69.370 54.932  1.00 31.45           O
ATOM   9471  N   LEU F 161     23.047 66.041 54.180  1.00 28.04           N
ATOM   9472  CA  LEU F 161     24.333 65.414 53.989  1.00 28.08           C
ATOM   9473  C   LEU F 161     24.088 64.290 53.012  1.00 28.24           C
ATOM   9474  O   LEU F 161     23.594 63.238 53.379  1.00 26.70           O
ATOM   9475  CB  LEU F 161     24.910 64.862 55.312  1.00 28.40           C
ATOM   9476  CG  LEU F 161     26.061 65.617 55.956  1.00 29.91           C
ATOM   9477  CD1 LEU F 161     26.443 64.971 57.300  1.00 28.30           C
ATOM   9478  CD2 LEU F 161     27.251 65.651 55.009  1.00 31.11           C
```

| ATOM | 9479 | N | SER | F | 162 | 24.394 | 64.549 | 51.750 | 1.00 | 27.19 | N |
|------|------|-----|-----|---|-----|--------|--------|--------|------|-------|---|
| ATOM | 9480 | CA | SER | F | 162 | 24.191 | 63.573 | 50.706 | 1.00 | 27.03 | C |
| ATOM | 9481 | C | SER | F | 162 | 25.504 | 63.129 | 50.103 | 1.00 | 27.20 | C |
| ATOM | 9482 | O | SER | F | 162 | 25.610 | 61.993 | 49.648 | 1.00 | 28.79 | O |
| ATOM | 9483 | CB | SER | F | 162 | 23.245 | 64.118 | 49.632 | 1.00 | 27.01 | C |
| ATOM | 9484 | OG | SER | F | 162 | 23.742 | 65.308 | 49.043 | 1.00 | 28.17 | O |
| ATOM | 9485 | N | SER | F | 163 | 26.503 | 64.003 | 50.096 | 1.00 | 26.34 | N |
| ATOM | 9486 | CA | SER | F | 163 | 27.793 | 63.675 | 49.486 | 1.00 | 25.63 | C |
| ATOM | 9487 | C | SER | F | 163 | 28.649 | 62.843 | 50.438 | 1.00 | 24.10 | C |
| ATOM | 9488 | O | SER | F | 163 | 28.560 | 63.014 | 51.644 | 1.00 | 23.77 | O |
| ATOM | 9489 | CB | SER | F | 163 | 28.564 | 64.952 | 49.133 | 1.00 | 27.68 | C |
| ATOM | 9490 | OG | SER | F | 163 | 29.368 | 64.715 | 47.992 | 1.00 | 30.22 | O |
| ATOM | 9491 | N | GLY | F | 164 | 29.482 | 61.963 | 49.889 | 1.00 | 22.31 | N |
| ATOM | 9492 | CA | GLY | F | 164 | 30.416 | 61.187 | 50.700 | 1.00 | 22.78 | C |
| ATOM | 9493 | C | GLY | F | 164 | 29.752 | 60.178 | 51.631 | 1.00 | 21.93 | C |
| ATOM | 9494 | O | GLY | F | 164 | 30.328 | 59.825 | 52.664 | 1.00 | 22.39 | O |
| ATOM | 9495 | N | VAL | F | 165 | 28.554 | 59.730 | 51.254 | 1.00 | 20.56 | N |
| ATOM | 9496 | CA | VAL | F | 165 | 27.723 | 58.801 | 52.040 | 1.00 | 19.88 | C |
| ATOM | 9497 | C | VAL | F | 165 | 27.692 | 57.442 | 51.323 | 1.00 | 19.20 | C |
| ATOM | 9498 | O | VAL | F | 165 | 27.515 | 57.380 | 50.101 | 1.00 | 18.83 | O |
| ATOM | 9499 | CB | VAL | F | 165 | 26.266 | 59.344 | 52.219 | 1.00 | 19.18 | C |
| ATOM | 9500 | CG1 | VAL | F | 165 | 25.347 | 58.309 | 52.855 | 1.00 | 19.95 | C |
| ATOM | 9501 | CG2 | VAL | F | 165 | 26.230 | 60.625 | 53.057 | 1.00 | 17.22 | C |
| ATOM | 9502 | N | HIS | F | 166 | 27.897 | 56.369 | 52.076 | 1.00 | 18.96 | N |
| ATOM | 9503 | CA | HIS | F | 166 | 27.743 | 54.998 | 51.572 | 1.00 | 19.35 | C |
| ATOM | 9504 | C | HIS | F | 166 | 26.785 | 54.300 | 52.499 | 1.00 | 16.91 | C |
| ATOM | 9505 | O | HIS | F | 166 | 27.050 | 54.255 | 53.670 | 1.00 | 15.72 | O |
| ATOM | 9506 | CB | HIS | F | 166 | 29.066 | 54.201 | 51.671 | 1.00 | 20.46 | C |
| ATOM | 9507 | CG | HIS | F | 166 | 30.196 | 54.784 | 50.886 | 1.00 | 22.02 | C |
| ATOM | 9508 | ND1 | HIS | F | 166 | 30.194 | 54.830 | 49.515 | 1.00 | 25.45 | N |
| ATOM | 9509 | CD2 | HIS | F | 166 | 31.376 | 55.315 | 51.278 | 1.00 | 24.30 | C |
| ATOM | 9510 | CE1 | HIS | F | 166 | 31.320 | 55.378 | 49.087 | 1.00 | 25.66 | C |
| ATOM | 9511 | NE2 | HIS | F | 166 | 32.061 | 55.673 | 50.137 | 1.00 | 25.99 | N |
| ATOM | 9512 | N | THR | F | 167 | 25.706 | 53.714 | 51.987 | 1.00 | 17.80 | N |
| ATOM | 9513 | CA | THR | F | 167 | 24.847 | 52.847 | 52.822 | 1.00 | 16.71 | C |
| ATOM | 9514 | C | THR | F | 167 | 24.942 | 51.444 | 52.279 | 1.00 | 16.48 | C |
| ATOM | 9515 | O | THR | F | 167 | 24.645 | 51.226 | 51.122 | 1.00 | 14.74 | O |
| ATOM | 9516 | CB | THR | F | 167 | 23.389 | 53.399 | 52.887 | 1.00 | 18.62 | C |
| ATOM | 9517 | OG1 | THR | F | 167 | 23.423 | 54.710 | 53.493 | 1.00 | 18.02 | O |
| ATOM | 9518 | CG2 | THR | F | 167 | 22.509 | 52.533 | 53.743 | 1.00 | 18.61 | C |
| ATOM | 9519 | N | PHE | F | 168 | 25.391 | 50.520 | 53.121 | 1.00 | 14.21 | N |
| ATOM | 9520 | CA | PHE | F | 168 | 25.675 | 49.142 | 52.724 | 1.00 | 16.19 | C |
| ATOM | 9521 | C | PHE | F | 168 | 24.359 | 48.353 | 52.713 | 1.00 | 16.87 | C |
| ATOM | 9522 | O | PHE | F | 168 | 23.450 | 48.658 | 53.478 | 1.00 | 16.47 | O |
| ATOM | 9523 | CB | PHE | F | 168 | 26.741 | 48.567 | 53.658 | 1.00 | 15.28 | C |
| ATOM | 9524 | CG | PHE | F | 168 | 28.041 | 49.349 | 53.622 | 1.00 | 16.48 | C |
| ATOM | 9525 | CD1 | PHE | F | 168 | 28.242 | 50.456 | 54.489 | 1.00 | 14.33 | C |
| ATOM | 9526 | CD2 | PHE | F | 168 | 29.019 | 49.057 | 52.670 | 1.00 | 15.49 | C |
| ATOM | 9527 | CE1 | PHE | F | 168 | 29.415 | 51.188 | 54.469 | 1.00 | 14.96 | C |
| ATOM | 9528 | CE2 | PHE | F | 168 | 30.213 | 49.785 | 52.634 | 1.00 | 16.65 | C |
| ATOM | 9529 | CZ | PHE | F | 168 | 30.414 | 50.867 | 53.528 | 1.00 | 16.05 | C |
| ATOM | 9530 | N | PRO | F | 169 | 24.219 | 47.392 | 51.781 | 1.00 | 15.43 | N |
| ATOM | 9531 | CA | PRO | F | 169 | 23.066 | 46.504 | 51.771 | 1.00 | 15.77 | C |
| ATOM | 9532 | C | PRO | F | 169 | 22.951 | 45.774 | 53.099 | 1.00 | 17.09 | C |
| ATOM | 9533 | O | PRO | F | 169 | 23.971 | 45.510 | 53.754 | 1.00 | 15.82 | O |
| ATOM | 9534 | CB | PRO | F | 169 | 23.391 | 45.512 | 50.640 | 1.00 | 15.79 | C |
| ATOM | 9535 | CG | PRO | F | 169 | 24.339 | 46.210 | 49.780 | 1.00 | 16.15 | C |
| ATOM | 9536 | CD | PRO | F | 169 | 25.117 | 47.148 | 50.646 | 1.00 | 14.99 | C |
| ATOM | 9537 | N | ALA | F | 170 | 21.715 | 45.464 | 53.503 | 1.00 | 15.31 | N |
| ATOM | 9538 | CA | ALA | F | 170 | 21.486 | 44.805 | 54.742 | 1.00 | 13.95 | C |
| ATOM | 9539 | C | ALA | F | 170 | 21.821 | 43.345 | 54.557 | 1.00 | 15.21 | C |
| ATOM | 9540 | O | ALA | F | 170 | 21.735 | 42.806 | 53.452 | 1.00 | 14.54 | O |
| ATOM | 9541 | CB | ALA | F | 170 | 20.058 | 44.934 | 55.176 | 1.00 | 13.16 | C |
| ATOM | 9542 | N | VAL | F | 171 | 22.220 | 42.761 | 55.658 | 1.00 | 16.35 | N |
| ATOM | 9543 | CA | VAL | F | 171 | 22.544 | 41.335 | 55.745 | 1.00 | 17.42 | C |

```
ATOM   9544  C   VAL F 171      21.650  40.749  56.816  1.00 19.06           C
ATOM   9545  O   VAL F 171      21.528  41.296  57.914  1.00 18.49           O
ATOM   9546  CB  VAL F 171      24.007  41.162  56.121  1.00 17.39           C
ATOM   9547  CG1 VAL F 171      24.375  39.686  56.295  1.00 18.27           C
ATOM   9548  CG2 VAL F 171      24.857  41.769  55.035  1.00 20.47           C
ATOM   9549  N   LEU F 172      21.030  39.615  56.490  1.00 19.90           N
ATOM   9550  CA  LEU F 172      20.112  38.968  57.384  1.00 21.87           C
ATOM   9551  C   LEU F 172      20.933  37.955  58.122  1.00 24.27           C
ATOM   9552  O   LEU F 172      21.631  37.180  57.477  1.00 22.91           O
ATOM   9553  CB  LEU F 172      19.020  38.220  56.588  1.00 21.50           C
ATOM   9554  CG  LEU F 172      17.928  37.456  57.353  1.00 20.72           C
ATOM   9555  CD1 LEU F 172      17.144  38.386  58.228  1.00 18.97           C
ATOM   9556  CD2 LEU F 172      17.012  36.762  56.378  1.00 20.32           C
ATOM   9557  N   GLN F 173      20.850  37.965  59.447  1.00 27.31           N
ATOM   9558  CA  GLN F 173      21.388  36.877  60.256  1.00 29.28           C
ATOM   9559  C   GLN F 173      20.505  36.624  61.454  1.00 31.06           C
ATOM   9560  O   GLN F 173      20.217  37.548  62.226  1.00 32.66           O
ATOM   9561  CB  GLN F 173      22.787  37.204  60.717  1.00 30.39           C
ATOM   9562  CG  GLN F 173      23.328  36.228  61.772  1.00 30.76           C
ATOM   9563  CD  GLN F 173      24.784  36.441  62.014  1.00 31.52           C
ATOM   9564  OE1 GLN F 173      25.237  37.579  62.269  1.00 35.35           O
ATOM   9565  NE2 GLN F 173      25.556  35.370  61.903  1.00 33.88           N
ATOM   9566  N   SER F 174      20.061  35.373  61.584  1.00 31.86           N
ATOM   9567  CA  SER F 174      19.235  34.909  62.703  1.00 31.78           C
ATOM   9568  C   SER F 174      17.903  35.634  62.809  1.00 31.91           C
ATOM   9569  O   SER F 174      17.501  36.032  63.918  1.00 34.18           O
ATOM   9570  CB  SER F 174      19.988  35.054  64.027  1.00 32.81           C
ATOM   9571  OG  SER F 174      21.341  34.678  63.890  1.00 36.13           O
ATOM   9572  N   ASP F 175      17.231  35.805  61.670  1.00 29.62           N
ATOM   9573  CA  ASP F 175      15.920  36.450  61.603  1.00 27.94           C
ATOM   9574  C   ASP F 175      15.975  37.969  61.677  1.00 24.28           C
ATOM   9575  O   ASP F 175      14.953  38.613  61.647  1.00 23.20           O
ATOM   9576  CB  ASP F 175      14.992  35.953  62.726  1.00 30.39           C
ATOM   9577  CG  ASP F 175      13.518  36.293  62.476  1.00 33.37           C
ATOM   9578  OD1 ASP F 175      13.100  37.477  62.694  1.00 38.33           O
ATOM   9579  OD2 ASP F 175      12.764  35.354  62.095  1.00 40.28           O
ATOM   9580  N   LEU F 176      17.154  38.549  61.809  1.00 21.39           N
ATOM   9581  CA  LEU F 176      17.222  40.004  61.942  1.00 18.76           C
ATOM   9582  C   LEU F 176      18.271  40.511  61.008  1.00 17.39           C
ATOM   9583  O   LEU F 176      19.234  39.798  60.717  1.00 14.01           O
ATOM   9584  CB  LEU F 176      17.563  40.386  63.369  1.00 18.43           C
ATOM   9585  CG  LEU F 176      16.551  39.949  64.434  1.00 17.13           C
ATOM   9586  CD1 LEU F 176      17.183  40.156  65.832  1.00 17.72           C
ATOM   9587  CD2 LEU F 176      15.238  40.695  64.300  1.00 17.70           C
ATOM   9588  N   TYR F 177      18.078  41.753  60.574  1.00 14.30           N
ATOM   9589  CA  TYR F 177      18.953  42.434  59.645  1.00 14.53           C
ATOM   9590  C   TYR F 177      19.881  43.401  60.358  1.00 12.78           C
ATOM   9591  O   TYR F 177      19.544  43.918  61.434  1.00 10.24           O
ATOM   9592  CB  TYR F 177      18.146  43.254  58.630  1.00 14.15           C
ATOM   9593  CG  TYR F 177      17.511  42.460  57.567  1.00 13.56           C
ATOM   9594  CD1 TYR F 177      18.241  42.024  56.461  1.00 15.59           C
ATOM   9595  CD2 TYR F 177      16.176  42.130  57.651  1.00 12.92           C
ATOM   9596  CE1 TYR F 177      17.632  41.260  55.444  1.00 16.15           C
ATOM   9597  CE2 TYR F 177      15.546  41.346  56.669  1.00 14.81           C
ATOM   9598  CZ  TYR F 177      16.269  40.918  55.560  1.00 16.71           C
ATOM   9599  OH  TYR F 177      15.627  40.153  54.601  1.00 16.87           O
ATOM   9600  N   THR F 178      21.043  43.594  59.745  1.00 14.20           N
ATOM   9601  CA  THR F 178      22.040  44.579  60.140  1.00 15.13           C
ATOM   9602  C   THR F 178      22.446  45.335  58.898  1.00 14.50           C
ATOM   9603  O   THR F 178      22.655  44.742  57.865  1.00 12.30           O
ATOM   9604  CB  THR F 178      23.317  43.935  60.741  1.00 16.67           C
ATOM   9605  OG1 THR F 178      22.997  43.214  61.934  1.00 20.73           O
ATOM   9606  CG2 THR F 178      24.331  45.029  61.094  1.00 16.64           C
ATOM   9607  N   LEU F 179      22.492  46.672  58.975  1.00 14.66           N
ATOM   9608  CA  LEU F 179      23.166  47.463  57.981  1.00 14.62           C
```

```
ATOM   9609  C   LEU F 179      23.911  48.614  58.636  1.00 14.06         C
ATOM   9610  O   LEU F 179      23.734  48.881  59.779  1.00  9.88         O
ATOM   9611  CB  LEU F 179      22.219  48.013  56.931  1.00 17.00         C
ATOM   9612  CG  LEU F 179      21.227  49.113  57.029  1.00 16.92         C
ATOM   9613  CD1 LEU F 179      21.832  50.531  57.146  1.00 14.48         C
ATOM   9614  CD2 LEU F 179      20.399  49.069  55.753  1.00 16.93         C
ATOM   9615  N   SER F 180      24.797  49.211  57.872  1.00 14.62         N
ATOM   9616  CA  SER F 180      25.541  50.359  58.324  1.00 14.30         C
ATOM   9617  C   SER F 180      25.525  51.405  57.229  1.00 13.39         C
ATOM   9618  O   SER F 180      25.311  51.122  56.058  1.00 14.23         O
ATOM   9619  CB  SER F 180      26.953  49.914  58.683  1.00 15.34         C
ATOM   9620  OG  SER F 180      27.553  49.363  57.545  1.00 17.51         O
ATOM   9621  N   SER F 181      25.724  52.653  57.630  1.00 14.74         N
ATOM   9622  CA  SER F 181      25.914  53.755  56.696  1.00 14.53         C
ATOM   9623  C   SER F 181      27.128  54.553  57.165  1.00 14.47         C
ATOM   9624  O   SER F 181      27.337  54.690  58.377  1.00 13.80         O
ATOM   9625  CB  SER F 181      24.689  54.651  56.657  1.00 15.73         C
ATOM   9626  OG  SER F 181      24.872  55.716  55.732  1.00 15.77         O
ATOM   9627  N   SER F 182      27.952  55.004  56.227  1.00 14.94         N
ATOM   9628  CA  SER F 182      29.126  55.840  56.569  1.00 16.83         C
ATOM   9629  C   SER F 182      29.012  57.178  55.850  1.00 16.49         C
ATOM   9630  O   SER F 182      28.550  57.224  54.715  1.00 15.93         O
ATOM   9631  CB  SER F 182      30.448  55.166  56.171  1.00 18.87         C
ATOM   9632  OG  SER F 182      30.758  55.330  54.831  1.00 19.50         O
ATOM   9633  N   VAL F 183      29.434  58.239  56.524  1.00 18.26         N
ATOM   9634  CA  VAL F 183      29.487  59.553  55.924  1.00 19.27         C
ATOM   9635  C   VAL F 183      30.872  60.097  56.216  1.00 18.88         C
ATOM   9636  O   VAL F 183      31.415  59.888  57.294  1.00 20.34         O
ATOM   9637  CB  VAL F 183      28.357  60.469  56.433  1.00 17.72         C
ATOM   9638  CG1 VAL F 183      28.431  60.653  57.916  1.00 18.70         C
ATOM   9639  CG2 VAL F 183      28.391  61.817  55.710  1.00 22.14         C
ATOM   9640  N   THR F 184      31.467  60.731  55.218  1.00 20.87         N
ATOM   9641  CA  THR F 184      32.787  61.313  55.358  1.00 21.12         C
ATOM   9642  C   THR F 184      32.630  62.825  55.153  1.00 22.89         C
ATOM   9643  O   THR F 184      32.030  63.268  54.176  1.00 23.35         O
ATOM   9644  CB  THR F 184      33.762  60.732  54.325  1.00 21.49         C
ATOM   9645  OG1 THR F 184      33.943  59.321  54.573  1.00 21.50         O
ATOM   9646  CG2 THR F 184      35.103  61.399  54.397  1.00 21.29         C
ATOM   9647  N   VAL F 185      33.195  63.594  56.067  1.00 23.82         N
ATOM   9648  CA  VAL F 185      33.074  65.050  56.017  1.00 25.09         C
ATOM   9649  C   VAL F 185      34.456  65.641  56.228  1.00 25.46         C
ATOM   9650  O   VAL F 185      35.360  64.954  56.726  1.00 24.70         O
ATOM   9651  CB  VAL F 185      32.076  65.592  57.097  1.00 25.52         C
ATOM   9652  CG1 VAL F 185      30.656  65.021  56.893  1.00 25.60         C
ATOM   9653  CG2 VAL F 185      32.569  65.304  58.525  1.00 26.01         C
ATOM   9654  N   PRO F 186      34.644  66.924  55.847  1.00 27.18         N
ATOM   9655  CA  PRO F 186      35.904  67.591  56.214  1.00 28.48         C
ATOM   9656  C   PRO F 186      36.086  67.655  57.727  1.00 29.23         C
ATOM   9657  O   PRO F 186      35.139  67.969  58.461  1.00 29.19         O
ATOM   9658  CB  PRO F 186      35.742  69.001  55.641  1.00 28.25         C
ATOM   9659  CG  PRO F 186      34.692  68.889  54.608  1.00 28.08         C
ATOM   9660  CD  PRO F 186      33.767  67.794  55.050  1.00 27.43         C
ATOM   9661  N   SER F 187      37.285  67.333  58.193  1.00 30.67         N
ATOM   9662  CA  SER F 187      37.559  67.254  59.625  1.00 32.59         C
ATOM   9663  C   SER F 187      37.266  68.549  60.365  1.00 33.70         C
ATOM   9664  O   SER F 187      37.018  68.541  61.577  1.00 35.05         O.
ATOM   9665  CB  SER F 187      39.013  66.872  59.851  1.00 33.39         C
ATOM   9666  OG  SER F 187      39.331  65.747  59.056  1.00 36.17         O
ATOM   9667  N   SER F 188      37.312  69.660  59.633  1.00 34.00         N
ATOM   9668  CA  SER F 188      37.022  70.965  60.206  1.00 33.93         C
ATOM   9669  C   SER F 188      35.558  71.135  60.542  1.00 33.76         C
ATOM   9670  O   SER F 188      35.213  72.077  61.247  1.00 35.78         O
ATOM   9671  CB  SER F 188      37.453  72.069  59.237  1.00 34.60         C
ATOM   9672  OG  SER F 188      36.635  72.102  58.066  1.00 37.18         O
ATOM   9673  N   THR F 189      34.691  70.259  60.023  1.00 32.08         N
```

```
ATOM   9674  CA   THR F 189     33.249  70.417  60.215  1.00 31.42        C
ATOM   9675  C    THR F 189     32.645  69.531  61.312  1.00 31.13        C
ATOM   9676  O    THR F 189     31.466  69.648  61.595  1.00 31.68        O
ATOM   9677  CB   THR F 189     32.460  70.226  58.883  1.00 31.89        C
ATOM   9678  OG1  THR F 189     32.515  68.864  58.449  1.00 30.14        O
ATOM   9679  CG2  THR F 189     33.043  71.105  57.793  1.00 31.62        C
ATOM   9680  N    TRP F 190     33.434  68.645  61.918  1.00 29.59        N
ATOM   9681  CA   TRP F 190     32.940  67.787  62.996  1.00 29.31        C
ATOM   9682  C    TRP F 190     33.984  67.729  64.083  1.00 29.58        C
ATOM   9683  O    TRP F 190     35.144  67.503  63.783  1.00 31.53        O
ATOM   9684  CB   TRP F 190     32.657  66.358  62.489  1.00 28.06        C
ATOM   9685  CG   TRP F 190     32.046  65.487  63.541  1.00 26.91        C
ATOM   9686  CD1  TRP F 190     30.744  65.448  63.911  1.00 26.12        C
ATOM   9687  CD2  TRP F 190     32.732  64.561  64.392  1.00 27.10        C
ATOM   9688  NE1  TRP F 190     30.566  64.542  64.926  1.00 25.79        N
ATOM   9689  CE2  TRP F 190     31.772  63.986  65.244  1.00 24.49        C
ATOM   9690  CE3  TRP F 190     34.066  64.160  64.513  1.00 27.11        C
ATOM   9691  CZ2  TRP F 190     32.097  63.020  66.196  1.00 26.16        C
ATOM   9692  CZ3  TRP F 190     34.386  63.206  65.465  1.00 26.24        C
ATOM   9693  CH2  TRP F 190     33.404  62.644  66.291  1.00 26.24        C
ATOM   9694  N    PRO F 191     33.580  67.865  65.359  1.00 31.23        N
ATOM   9695  CA   PRO F 191     32.227  68.056  65.908  1.00 32.40        C
ATOM   9696  C    PRO F 191     31.572  69.434  65.813  1.00 32.82        C
ATOM   9697  O    PRO F 191     30.409  69.556  66.187  1.00 33.79        O
ATOM   9698  CB   PRO F 191     32.398  67.693  67.378  1.00 32.12        C
ATOM   9699  CG   PRO F 191     33.802  67.994  67.682  1.00 32.13        C
ATOM   9700  CD   PRO F 191     34.581  67.727  66.432  1.00 32.14        C
ATOM   9701  N    SER F 192     32.265  70.454  65.315  1.00 34.02        N
ATOM   9702  CA   SER F 192     31.690  71.813  65.260  1.00 33.27        C
ATOM   9703  C    SER F 192     30.306  71.841  64.626  1.00 32.96        C
ATOM   9704  O    SER F 192     29.381  72.446  65.174  1.00 32.70        O
ATOM   9705  CB   SER F 192     32.618  72.790  64.517  1.00 33.78        C
ATOM   9706  OG   SER F 192     33.019  72.291  63.255  1.00 34.07        O
ATOM   9707  N    GLU F 193     30.170  71.197  63.466  1.00 32.93        N
ATOM   9708  CA   GLU F 193     28.858  71.008  62.824  1.00 33.20        C
ATOM   9709  C    GLU F 193     28.323  69.599  63.152  1.00 31.96        C
ATOM   9710  O    GLU F 193     29.048  68.610  63.018  1.00 31.58        O
ATOM   9711  CB   GLU F 193     28.947  71.235  61.313  1.00 34.18        C
ATOM   9712  CG   GLU F 193     28.834  72.731  60.935  1.00 37.89        C
ATOM   9713  CD   GLU F 193     29.755  73.145  59.803  1.00 38.64        C
ATOM   9714  OE1  GLU F 193     29.248  73.680  58.772  1.00 43.69        O
ATOM   9715  OE2  GLU F 193     30.994  72.959  59.960  1.00 43.93        O
ATOM   9716  N    THR F 194     27.067  69.530  63.584  1.00 29.52        N
ATOM   9717  CA   THR F 194     26.480  68.266  64.024  1.00 28.95        C
ATOM   9718  C    THR F 194     26.244  67.366  62.829  1.00 26.69        C
ATOM   9719  O    THR F 194     25.950  67.857  61.718  1.00 25.19        O
ATOM   9720  CB   THR F 194     25.147  68.456  64.769  1.00 29.00        C
ATOM   9721  OG1  THR F 194     24.185  69.061  63.905  1.00 30.76        O
ATOM   9722  CG2  THR F 194     25.329  69.330  65.978  1.00 30.20        C
ATOM   9723  N    VAL F 195     26.384  66.056  63.081  1.00 24.83        N
ATOM   9724  CA   VAL F 195     26.117  65.011  62.106  1.00 24.10        C
ATOM   9725  C    VAL F 195     25.203  64.002  62.781  1.00 22.16        C
ATOM   9726  O    VAL F 195     25.560  63.445  63.824  1.00 20.78        O
ATOM   9727  CB   VAL F 195     27.408  64.322  61.641  1.00 23.64        C
ATOM   9728  CG1  VAL F 195     27.097  63.178  60.663  1.00 24.25        C
ATOM   9729  CG2  VAL F 195     28.350  65.339  61.015  1.00 24.30        C
ATOM   9730  N    THR F 196     24.044  63.787  62.165  1.00 21.50        N
ATOM   9731  CA   THR F 196     22.942  63.006  62.746  1.00 20.87        C
ATOM   9732  C    THR F 196     22.395  62.086  61.676  1.00 20.41        C
ATOM   9733  O    THR F 196     21.962  62.539  60.611  1.00 19.52        O
ATOM   9734  CB   THR F 196     21.823  63.932  63.235  1.00 20.79        C
ATOM   9735  OG1  THR F 196     22.386  64.945  64.075  1.00 19.13        O
ATOM   9736  CG2  THR F 196     20.760  63.179  64.022  1.00 22.17        C
ATOM   9737  N    CYS F 197     22.419  60.789  61.946  1.00 18.99        N
ATOM   9738  CA   CYS F 197     21.778  59.864  61.033  1.00 18.28        C
```

| ATOM | 9739 | C | CYS | F | 197 | 20.311 | 59.657 | 61.395 | 1.00 | 16.81 | C |
|---|---|---|---|---|---|---|---|---|---|---|---|
| ATOM | 9740 | O | CYS | F | 197 | 19.931 | 59.616 | 62.560 | 1.00 | 16.58 | O |
| ATOM | 9741 | CB | CYS | F | 197 | 22.550 | 58.572 | 60.923 | 1.00 | 20.18 | C |
| ATOM | 9742 | SG | CYS | F | 197 | 22.191 | 57.419 | 62.160 | 1.00 | 24.38 | S |
| ATOM | 9743 | N | ASN | F | 198 | 19.492 | 59.606 | 60.358 | 1.00 | 17.48 | N |
| ATOM | 9744 | CA | ASN | F | 198 | 18.036 | 59.444 | 60.467 | 1.00 | 16.80 | C |
| ATOM | 9745 | C | ASN | F | 198 | 17.692 | 58.123 | 59.827 | 1.00 | 15.79 | C |
| ATOM | 9746 | O | ASN | F | 198 | 18.051 | 57.854 | 58.689 | 1.00 | 16.41 | O |
| ATOM | 9747 | CB | ASN | F | 198 | 17.284 | 60.521 | 59.705 | 1.00 | 18.19 | C |
| ATOM | 9748 | CG | ASN | F | 198 | 17.989 | 61.831 | 59.721 | 1.00 | 19.19 | C |
| ATOM | 9749 | OD1 | ASN | F | 198 | 18.573 | 62.254 | 58.703 | 1.00 | 21.91 | O |
| ATOM | 9750 | ND2 | ASN | F | 198 | 18.002 | 62.476 | 60.882 | 1.00 | 22.29 | N |
| ATOM | 9751 | N | VAL | F | 199 | 17.007 | 57.307 | 60.585 | 1.00 | 14.71 | N |
| ATOM | 9752 | CA | VAL | F | 199 | 16.740 | 55.938 | 60.165 | 1.00 | 12.48 | C |
| ATOM | 9753 | C | VAL | F | 199 | 15.237 | 55.686 | 60.206 | 1.00 | 13.11 | C |
| ATOM | 9754 | O | VAL | F | 199 | 14.627 | 55.740 | 61.279 | 1.00 | 16.67 | O |
| ATOM | 9755 | CB | VAL | F | 199 | 17.462 | 54.914 | 61.078 | 1.00 | 13.67 | C |
| ATOM | 9756 | CG1 | VAL | F | 199 | 17.151 | 53.564 | 60.601 | 1.00 | 13.01 | C |
| ATOM | 9757 | CG2 | VAL | F | 199 | 18.973 | 55.142 | 61.078 | 1.00 | 14.00 | C |
| ATOM | 9758 | N | ALA | F | 200 | 14.650 | 55.365 | 59.058 | 1.00 | 14.85 | N |
| ATOM | 9759 | CA | ALA | F | 200 | 13.228 | 55.079 | 58.951 | 1.00 | 12.14 | C |
| ATOM | 9760 | C | ALA | F | 200 | 12.947 | 53.604 | 58.613 | 1.00 | 11.96 | C |
| ATOM | 9761 | O | ALA | F | 200 | 13.538 | 53.046 | 57.699 | 1.00 | 14.39 | O |
| ATOM | 9762 | CB | ALA | F | 200 | 12.581 | 55.974 | 57.934 | 1.00 | 15.34 | C |
| ATOM | 9763 | N | HIS | F | 201 | 11.973 | 53.050 | 59.310 | 1.00 | 9.58 | N |
| ATOM | 9764 | CA | HIS | F | 201 | 11.601 | 51.626 | 59.106 | 1.00 | 11.17 | C |
| ATOM | 9765 | C | HIS | F | 201 | 10.122 | 51.517 | 58.838 | 1.00 | 10.95 | C |
| ATOM | 9766 | O | HIS | F | 201 | 9.308 | 51.456 | 59.763 | 1.00 | 11.52 | O |
| ATOM | 9767 | CB | HIS | F | 201 | 12.009 | 50.854 | 60.336 | 1.00 | 9.70 | C |
| ATOM | 9768 | CG | HIS | F | 201 | 11.867 | 49.365 | 60.190 | 1.00 | 8.61 | C |
| ATOM | 9769 | ND1 | HIS | F | 201 | 11.245 | 48.596 | 61.140 | 1.00 | 11.24 | N |
| ATOM | 9770 | CD2 | HIS | F | 201 | 12.184 | 48.540 | 59.177 | 1.00 | 12.72 | C |
| ATOM | 9771 | CE1 | HIS | F | 201 | 11.252 | 47.328 | 60.749 | 1.00 | 8.87 | C |
| ATOM | 9772 | NE2 | HIS | F | 201 | 11.820 | 47.260 | 59.562 | 1.00 | 9.69 | N |
| ATOM | 9773 | N | PRO | F | 202 | 9.744 | 51.510 | 57.562 | 1.00 | 13.17 | N |
| ATOM | 9774 | CA | PRO | F | 202 | 8.317 | 51.409 | 57.215 | 1.00 | 14.22 | C |
| ATOM | 9775 | C | PRO | F | 202 | 7.481 | 50.386 | 57.970 | 1.00 | 14.04 | C |
| ATOM | 9776 | O | PRO | F | 202 | 6.388 | 50.714 | 58.443 | 1.00 | 11.19 | O |
| ATOM | 9777 | CB | PRO | F | 202 | 8.326 | 51.084 | 55.731 | 1.00 | 14.05 | C |
| ATOM | 9778 | CG | PRO | F | 202 | 9.599 | 51.540 | 55.232 | 1.00 | 14.25 | C |
| ATOM | 9779 | CD | PRO | F | 202 | 10.591 | 51.605 | 56.362 | 1.00 | 13.73 | C |
| ATOM | 9780 | N | ALA | F | 203 | 7.969 | 49.160 | 58.120 | 1.00 | 13.59 | N |
| ATOM | 9781 | CA | ALA | F | 203 | 7.148 | 48.132 | 58.748 | 1.00 | 14.11 | C |
| ATOM | 9782 | C | ALA | F | 203 | 6.802 | 48.402 | 60.204 | 1.00 | 13.72 | C |
| ATOM | 9783 | O | ALA | F | 203 | 5.773 | 47.947 | 60.662 | 1.00 | 13.77 | O |
| ATOM | 9784 | CB | ALA | F | 203 | 7.794 | 46.757 | 58.601 | 1.00 | 14.02 | C |
| ATOM | 9785 | N | SER | F | 204 | 7.615 | 49.196 | 60.897 | 1.00 | 13.70 | N |
| ATOM | 9786 | CA | SER | F | 204 | 7.327 | 49.599 | 62.264 | 1.00 | 13.85 | C |
| ATOM | 9787 | C | SER | F | 204 | 6.861 | 51.036 | 62.377 | 1.00 | 13.85 | C |
| ATOM | 9788 | O | SER | F | 204 | 6.602 | 51.491 | 63.482 | 1.00 | 15.12 | O |
| ATOM | 9789 | CB | SER | F | 204 | 8.540 | 49.403 | 63.185 | 1.00 | 11.49 | C |
| ATOM | 9790 | OG | SER | F | 204 | 9.669 | 50.169 | 62.806 | 1.00 | 11.95 | O |
| ATOM | 9791 | N | SER | F | 205 | 6.751 | 51.748 | 61.255 | 1.00 | 15.78 | N |
| ATOM | 9792 | CA | SER | F | 205 | 6.353 | 53.162 | 61.282 | 1.00 | 15.63 | C |
| ATOM | 9793 | C | SER | F | 205 | 7.180 | 53.980 | 62.254 | 1.00 | 16.38 | C |
| ATOM | 9794 | O | SER | F | 205 | 6.674 | 54.850 | 62.974 | 1.00 | 16.64 | O |
| ATOM | 9795 | CB | SER | F | 205 | 4.865 | 53.261 | 61.614 | 1.00 | 15.92 | C |
| ATOM | 9796 | OG | SER | F | 205 | 4.117 | 52.413 | 60.774 | 1.00 | 14.95 | O |
| ATOM | 9797 | N | THR | F | 206 | 8.478 | 53.716 | 62.282 | 1.00 | 16.26 | N |
| ATOM | 9798 | CA | THR | F | 206 | 9.377 | 54.424 | 63.155 | 1.00 | 15.19 | C |
| ATOM | 9799 | C | THR | F | 206 | 10.351 | 55.195 | 62.345 | 1.00 | 15.96 | C |
| ATOM | 9800 | O | THR | F | 206 | 10.589 | 54.902 | 61.171 | 1.00 | 13.16 | O |
| ATOM | 9801 | CB | THR | F | 206 | 10.172 | 53.512 | 64.066 | 1.00 | 14.34 | C |
| ATOM | 9802 | OG1 | THR | F | 206 | 10.808 | 52.494 | 63.284 | 1.00 | 14.42 | O |
| ATOM | 9803 | CG2 | THR | F | 206 | 9.264 | 52.886 | 65.116 | 1.00 | 16.79 | C |

```
ATOM  9804  N    LYS F 207    10.831  56.266  62.970  1.00 19.30        N
ATOM  9805  CA   LYS F 207    11.921  57.066  62.452  1.00 20.80        C
ATOM  9806  C    LYS F 207    12.734  57.547  63.638  1.00 20.50        C
ATOM  9807  O    LYS F 207    12.193  58.149  64.583  1.00 21.91        O
ATOM  9808  CB   LYS F 207    11.423  58.273  61.659  1.00 23.83        C
ATOM  9809  CG   LYS F 207    12.406  58.710  60.595  1.00 27.63        C
ATOM  9810  CD   LYS F 207    12.710  60.205  60.622  1.00 30.93        C
ATOM  9811  CE   LYS F 207    13.997  60.481  61.385  1.00 30.74        C
ATOM  9812  NZ   LYS F 207    14.436  61.902  61.210  1.00 32.68        N
ATOM  9813  N    VAL F 208    14.021  57.228  63.620  1.00 19.12        N
ATOM  9814  CA   VAL F 208    14.893  57.440  64.756  1.00 20.33        C
ATOM  9815  C    VAL F 208    16.067  58.319  64.324  1.00 19.67        C
ATOM  9816  O    VAL F 208    16.603  58.146  63.219  1.00 18.00        O
ATOM  9817  CB   VAL F 208    15.409  56.078  65.288  1.00 21.75        C
ATOM  9818  CG1  VAL F 208    16.670  56.246  66.087  1.00 24.21        C
ATOM  9819  CG2  VAL F 208    14.325  55.388  66.100  1.00 23.42        C
ATOM  9820  N    ASP F 209    16.460  59.253  65.194  1.00 20.36        N
ATOM  9821  CA   ASP F 209    17.651  60.075  64.955  1.00 21.64        C
ATOM  9822  C    ASP F 209    18.763  59.745  65.927  1.00 21.82        C
ATOM  9823  O    ASP F 209    18.527  59.570  67.131  1.00 24.75        O
ATOM  9824  CB   ASP F 209    17.320  61.570  65.070  1.00 23.69        C
ATOM  9825  CG   ASP F 209    16.356  62.014  64.027  1.00 26.25        C
ATOM  9826  OD1  ASP F 209    16.374  61.463  62.903  1.00 25.78        O
ATOM  9827  OD2  ASP F 209    15.549  62.901  64.344  1.00 30.07        O
ATOM  9828  N    LYS F 210    19.979  59.636  65.413  1.00 19.41        N
ATOM  9829  CA   LYS F 210    21.117  59.430  66.258  1.00 18.84        C
ATOM  9830  C    LYS F 210    22.173  60.437  65.867  1.00 19.46        C
ATOM  9831  O    LYS F 210    22.708  60.394  64.772  1.00 18.87        O
ATOM  9832  CB   LYS F 210    21.661  57.998  66.105  1.00 18.07        C
ATOM  9833  CG   LYS F 210    22.882  57.676  66.969  1.00 15.27        C
ATOM  9834  CD   LYS F 210    22.754  57.955  68.443  1.00 15.68        C
ATOM  9835  CE   LYS F 210    21.672  57.098  69.123  1.00 11.26        C
ATOM  9836  NZ   LYS F 210    21.450  57.357  70.571  1.00  9.36        N
ATOM  9837  N    LYS F 211    22.420  61.388  66.760  1.00 21.11        N
ATOM  9838  CA   LYS F 211    23.517  62.328  66.614  1.00 21.60        C
ATOM  9839  C    LYS F 211    24.834  61.634  66.943  1.00 20.71        C
ATOM  9840  O    LYS F 211    24.922  60.862  67.905  1.00 24.10        O
ATOM  9841  CB   LYS F 211    23.274  63.497  67.557  1.00 21.89        C
ATOM  9842  CG   LYS F 211    24.355  64.541  67.548  1.00 23.73        C
ATOM  9843  CD   LYS F 211    24.021  65.630  68.544  1.00 24.27        C
ATOM  9844  CE   LYS F 211    25.152  66.665  68.634  1.00 25.41        C
ATOM  9845  NZ   LYS F 211    24.997  67.644  69.774  1.00 27.63        N
ATOM  9846  N    ILE F 212    25.839  61.855  66.110  1.00 19.40        N
ATOM  9847  CA   ILE F 212    27.160  61.308  66.318  1.00 21.61        C
ATOM  9848  C    ILE F 212    27.992  62.306  67.119  1.00 24.04        C
ATOM  9849  O    ILE F 212    28.308  63.390  66.625  1.00 23.11        O
ATOM  9850  CB   ILE F 212    27.869  60.974  64.976  1.00 19.56        C
ATOM  9851  CG1  ILE F 212    26.929  60.245  64.002  1.00 20.91        C
ATOM  9852  CG2  ILE F 212    29.127  60.141  65.210  1.00 20.09        C
ATOM  9853  CD1  ILE F 212    26.274  58.956  64.558  1.00 21.25        C
ATOM  9854  N    VAL F 213    28.328  61.945  68.352  1.00 25.48        N
ATOM  9855  CA   VAL F 213    29.088  62.842  69.226  1.00 27.06        C
ATOM  9856  C    VAL F 213    30.451  62.243  69.529  1.00 27.62        C
ATOM  9857  O    VAL F 213    30.602  61.015  69.566  1.00 25.26        O
ATOM  9858  CB   VAL F 213    28.326  63.163  70.532  1.00 27.67        C
ATOM  9859  CG1  VAL F 213    26.984  63.782  70.220  1.00 28.68        C
ATOM  9860  CG2  VAL F 213    28.145  61.942  71.385  1.00 28.57        C
ATOM  9861  N    PRO F 214    31.468  63.098  69.720  1.00 30.02        N
ATOM  9862  CA   PRO F 214    32.801  62.551  69.995  1.00 30.50        C
ATOM  9863  C    PRO F 214    32.874  61.786  71.298  1.00 31.28        C
ATOM  9864  O    PRO F 214    32.223  62.139  72.258  1.00 31.26        O
ATOM  9865  CB   PRO F 214    33.692  63.798  70.071  1.00 30.95        C
ATOM  9866  CG   PRO F 214    32.948  64.848  69.411  1.00 30.36        C
ATOM  9867  CD   PRO F 214    31.497  64.568  69.664  1.00 30.16        C
ATOM  9868  N    ARG F 215    33.683  60.746  71.320  1.00 32.31        N
```

| ATOM | 9869 | CA | ARG | F | 215 | 33.818 | 59.903 | 72.493 | 1.00 | 33.08 | C |
| ATOM | 9870 | C | ARG | F | 215 | 34.652 | 60.603 | 73.583 | 1.00 | 34.20 | C |
| ATOM | 9871 | O | ARG | F | 215 | 35.692 | 61.211 | 73.294 | 1.00 | 35.20 | O |
| ATOM | 9872 | CB | ARG | F | 215 | 34.449 | 58.586 | 72.072 | 1.00 | 33.77 | C |
| ATOM | 9873 | CG | ARG | F | 215 | 33.569 | 57.818 | 71.091 | 1.00 | 34.47 | C |
| ATOM | 9874 | CD | ARG | F | 215 | 34.318 | 56.737 | 70.414 | 1.00 | 34.00 | C |
| ATOM | 9875 | NE | ARG | F | 215 | 34.900 | 55.825 | 71.383 | 1.00 | 35.97 | N |
| ATOM | 9876 | CZ | ARG | F | 215 | 34.300 | 54.742 | 71.875 | 1.00 | 36.11 | C |
| ATOM | 9877 | NH1 | ARG | F | 215 | 33.046 | 54.419 | 71.504 | 1.00 | 35.07 | N |
| ATOM | 9878 | NH2 | ARG | F | 215 | 34.962 | 53.980 | 72.758 | 1.00 | 34.62 | N |
| TER | 9879 | | ARG | F | 215 | | | | | | |
| ATOM | 9880 | N | ASP | G | 1 | 33.751 | 30.752 | -55.116 | 1.00 | 24.85 | N |
| ATOM | 9881 | CA | ASP | G | 1 | 32.887 | 29.626 | -54.689 | 1.00 | 22.48 | C |
| ATOM | 9882 | C | ASP | G | 1 | 31.651 | 29.478 | -55.582 | 1.00 | 21.74 | C |
| ATOM | 9883 | O | ASP | G | 1 | 30.991 | 30.445 | -55.952 | 1.00 | 21.71 | O |
| ATOM | 9884 | CB | ASP | G | 1 | 32.461 | 29.808 | -53.244 | 1.00 | 26.01 | C |
| ATOM | 9885 | CG | ASP | G | 1 | 33.638 | 30.128 | -52.335 | 1.00 | 29.11 | C |
| ATOM | 9886 | OD1 | ASP | G | 1 | 34.795 | 30.216 | -52.848 | 1.00 | 33.76 | O |
| ATOM | 9887 | OD2 | ASP | G | 1 | 33.385 | 30.317 | -51.130 | 1.00 | 33.91 | O |
| ATOM | 9888 | N | ILE | G | 2 | 31.329 | 28.242 | -55.912 | 1.00 | 17.94 | N |
| ATOM | 9889 | CA | ILE | G | 2 | 30.212 | 27.970 | -56.774 | 1.00 | 15.46 | C |
| ATOM | 9890 | C | ILE | G | 2 | 28.927 | 28.085 | -55.962 | 1.00 | 15.90 | C |
| ATOM | 9891 | O | ILE | G | 2 | 28.797 | 27.473 | -54.890 | 1.00 | 14.05 | O |
| ATOM | 9892 | CB | ILE | G | 2 | 30.391 | 26.564 | -57.455 | 1.00 | 15.07 | C |
| ATOM | 9893 | CG1 | ILE | G | 2 | 31.583 | 26.566 | -58.419 | 1.00 | 14.14 | C |
| ATOM | 9894 | CG2 | ILE | G | 2 | 29.106 | 26.145 | -58.189 | 1.00 | 16.03 | C |
| ATOM | 9895 | CD1 | ILE | G | 2 | 31.914 | 25.170 | -59.036 | 1.00 | 14.44 | C |
| ATOM | 9896 | N | VAL | G | 3 | 27.995 | 28.921 | -56.443 | 1.00 | 15.93 | N |
| ATOM | 9897 | CA | VAL | G | 3 | 26.627 | 28.976 | -55.913 | 1.00 | 14.64 | C |
| ATOM | 9898 | C | VAL | G | 3 | 25.685 | 28.037 | -56.663 | 1.00 | 14.95 | C |
| ATOM | 9899 | O | VAL | G | 3 | 25.505 | 28.158 | -57.884 | 1.00 | 13.15 | O |
| ATOM | 9900 | CB | VAL | G | 3 | 26.016 | 30.385 | -55.976 | 1.00 | 15.63 | C |
| ATOM | 9901 | CG1 | VAL | G | 3 | 24.585 | 30.333 | -55.459 | 1.00 | 14.83 | C |
| ATOM | 9902 | CG2 | VAL | G | 3 | 26.851 | 31.303 | -55.165 | 1.00 | 15.95 | C |
| ATOM | 9903 | N | MET | G | 4 | 25.076 | 27.127 | -55.898 | 1.00 | 12.88 | N |
| ATOM | 9904 | CA | MET | G | 4 | 24.087 | 26.160 | -56.410 | 1.00 | 12.61 | C |
| ATOM | 9905 | C | MET | G | 4 | 22.695 | 26.605 | -55.959 | 1.00 | 12.28 | C |
| ATOM | 9906 | O | MET | G | 4 | 22.467 | 26.735 | -54.769 | 1.00 | 12.53 | O |
| ATOM | 9907 | CB | MET | G | 4 | 24.356 | 24.745 | -55.868 | 1.00 | 12.24 | C |
| ATOM | 9908 | CG | MET | G | 4 | 25.712 | 24.167 | -56.114 | 1.00 | 14.17 | C |
| ATOM | 9909 | SD | MET | G | 4 | 26.146 | 23.803 | -57.800 | 1.00 | 13.71 | S |
| ATOM | 9910 | CE | MET | G | 4 | 24.994 | 22.472 | -58.186 | 1.00 | 14.29 | C |
| ATOM | 9911 | N | THR | G | 5 | 21.799 | 26.896 | -56.911 | 1.00 | 12.82 | N |
| ATOM | 9912 | CA | THR | G | 5 | 20.510 | 27.445 | -56.640 | 1.00 | 13.42 | C |
| ATOM | 9913 | C | THR | G | 5 | 19.389 | 26.486 | -57.011 | 1.00 | 13.00 | C |
| ATOM | 9914 | O | THR | G | 5 | 19.253 | 26.105 | -58.150 | 1.00 | 15.43 | O |
| ATOM | 9915 | CB | THR | G | 5 | 20.317 | 28.771 | -57.438 | 1.00 | 13.98 | C |
| ATOM | 9916 | OG1 | THR | G | 5 | 21.412 | 29.650 | -57.155 | 1.00 | 16.46 | O |
| ATOM | 9917 | CG2 | THR | G | 5 | 18.965 | 29.460 | -57.112 | 1.00 | 15.37 | C |
| ATOM | 9918 | N | GLN | G | 6 | 18.526 | 26.216 | -56.044 | 1.00 | 13.21 | N |
| ATOM | 9919 | CA | GLN | G | 6 | 17.367 | 25.456 | -56.266 | 1.00 | 14.77 | C |
| ATOM | 9920 | C | GLN | G | 6 | 16.241 | 25.979 | -55.369 | 1.00 | 15.99 | C |
| ATOM | 9921 | O | GLN | G | 6 | 16.485 | 26.501 | -54.297 | 1.00 | 15.37 | O |
| ATOM | 9922 | CB | GLN | G | 6 | 17.701 | 23.978 | -55.988 | 1.00 | 16.42 | C |
| ATOM | 9923 | CG | GLN | G | 6 | 18.204 | 23.724 | -54.609 | 1.00 | 14.57 | C |
| ATOM | 9924 | CD | GLN | G | 6 | 18.548 | 22.235 | -54.346 | 1.00 | 12.48 | C |
| ATOM | 9925 | OE1 | GLN | G | 6 | 19.514 | 21.948 | -53.654 | 1.00 | 11.74 | O |
| ATOM | 9926 | NE2 | GLN | G | 6 | 17.728 | 21.336 | -54.826 | 1.00 | 14.19 | N |
| ATOM | 9927 | N | ALA | G | 7 | 15.007 | 25.812 | -55.801 | 1.00 | 17.57 | N |
| ATOM | 9928 | CA | ALA | G | 7 | 13.866 | 26.275 | -55.006 | 1.00 | 18.88 | C |
| ATOM | 9929 | C | ALA | G | 7 | 13.686 | 25.437 | -53.735 | 1.00 | 20.52 | C |
| ATOM | 9930 | O | ALA | G | 7 | 13.970 | 24.230 | -53.711 | 1.00 | 18.31 | O |
| ATOM | 9931 | CB | ALA | G | 7 | 12.580 | 26.253 | -55.852 | 1.00 | 19.16 | C |
| ATOM | 9932 | N | ALA | G | 8 | 13.163 | 26.077 | -52.700 | 1.00 | 21.38 | N |
| ATOM | 9933 | CA | ALA | G | 8 | 12.979 | 25.417 | -51.400 | 1.00 | 23.85 | C |

```
ATOM   9934  C   ALA G   8      11.903  24.362 -51.445  1.00 25.46          C
ATOM   9935  O   ALA G   8      11.981  23.326 -50.755  1.00 25.18          O
ATOM   9936  CB  ALA G   8      12.665  26.471 -50.318  1.00 23.82          C
ATOM   9937  N   PHE G   9      10.883  24.621 -52.252  1.00 27.91          N
ATOM   9938  CA  PHE G   9       9.737  23.729 -52.333  1.00 31.58          C
ATOM   9939  C   PHE G   9       9.365  23.517 -53.781  1.00 33.90          C
ATOM   9940  O   PHE G   9       9.538  24.415 -54.615  1.00 33.79          O
ATOM   9941  CB  PHE G   9       8.532  24.312 -51.572  1.00 32.74          C
ATOM   9942  CG  PHE G   9       8.872  24.836 -50.208  1.00 32.95          C
ATOM   9943  CD1 PHE G   9       8.890  26.212 -49.956  1.00 33.37          C
ATOM   9944  CD2 PHE G   9       9.191  23.959 -49.176  1.00 33.57          C
ATOM   9945  CE1 PHE G   9       9.225  26.694 -48.696  1.00 33.11          C
ATOM   9946  CE2 PHE G   9       9.530  24.431 -47.904  1.00 33.64          C
ATOM   9947  CZ  PHE G   9       9.542  25.798 -47.661  1.00 33.06          C
ATOM   9948  N   SER G  10       8.886  22.308 -54.071  1.00 37.68          N
ATOM   9949  CA  SER G  10       8.202  22.005 -55.328  1.00 38.17          C
ATOM   9950  C   SER G  10       6.703  21.941 -55.045  1.00 39.95          C
ATOM   9951  O   SER G  10       6.283  21.733 -53.905  1.00 40.99          O
ATOM   9952  CB  SER G  10       8.703  20.680 -55.917  1.00 39.32          C
ATOM   9953  OG  SER G  10       8.616  19.602 -54.995  1.00 39.92          O
ATOM   9954  N   ASN G  11       5.893  22.137 -56.080  1.00 40.66          N
ATOM   9955  CA  ASN G  11       4.442  22.026 -55.944  1.00 40.11          C
ATOM   9956  C   ASN G  11       4.124  20.562 -55.652  1.00 39.80          C
ATOM   9957  O   ASN G  11       4.710  19.688 -56.286  1.00 40.49          O
ATOM   9958  CB  ASN G  11       3.745  22.494 -57.224  1.00 41.64          C
ATOM   9959  CG  ASN G  11       4.055  23.961 -57.559  1.00 43.85          C
ATOM   9960  OD1 ASN G  11       3.991  24.830 -56.689  1.00 46.71          O
ATOM   9961  ND2 ASN G  11       4.404  24.230 -58.820  1.00 45.24          N
ATOM   9962  N   PRO G  12       3.231  20.284 -54.677  1.00 38.53          N
ATOM   9963  CA  PRO G  12       2.956  18.892 -54.287  1.00 37.58          C
ATOM   9964  C   PRO G  12       2.583  18.028 -55.475  1.00 36.45          C
ATOM   9965  O   PRO G  12       2.015  18.521 -56.437  1.00 37.20          O
ATOM   9966  CB  PRO G  12       1.774  19.018 -53.326  1.00 37.38          C
ATOM   9967  CG  PRO G  12       1.872  20.386 -52.789  1.00 38.50          C
ATOM   9968  CD  PRO G  12       2.434  21.235 -53.881  1.00 38.79          C
ATOM   9969  N   VAL G  13       2.882  16.746 -55.383  1.00 34.85          N
ATOM   9970  CA  VAL G  13       2.799  15.856 -56.513  1.00 34.18          C
ATOM   9971  C   VAL G  13       1.982  14.665 -56.092  1.00 32.87          C
ATOM   9972  O   VAL G  13       2.263  14.038 -55.079  1.00 31.89          O
ATOM   9973  CB  VAL G  13       4.221  15.421 -57.021  1.00 34.97          C
ATOM   9974  CG1 VAL G  13       5.244  15.458 -55.905  1.00 36.28          C
ATOM   9975  CG2 VAL G  13       4.213  14.020 -57.673  1.00 34.20          C
ATOM   9976  N   THR G  14       0.956  14.361 -56.879  1.00 32.13          N
ATOM   9977  CA  THR G  14       0.078  13.243 -56.582  1.00 31.26          C
ATOM   9978  C   THR G  14       0.825  11.942 -56.681  1.00 29.76          C
ATOM   9979  O   THR G  14       1.610  11.742 -57.589  1.00 28.84          O
ATOM   9980  CB  THR G  14      -1.110  13.222 -57.547  1.00 30.91          C
ATOM   9981  OG1 THR G  14      -1.714  14.517 -57.555  1.00 32.51          O
ATOM   9982  CG2 THR G  14      -2.130  12.182 -57.134  1.00 32.13          C
ATOM   9983  N   LEU G  15       0.580  11.047 -55.739  1.00 29.86          N
ATOM   9984  CA  LEU G  15       1.138   9.706 -55.834  1.00 30.84          C
ATOM   9985  C   LEU G  15       0.970   9.159 -57.240  1.00 30.60          C
ATOM   9986  O   LEU G  15      -0.103   9.293 -57.832  1.00 29.39          O
ATOM   9987  CB  LEU G  15       0.441   8.744 -54.868  1.00 31.10          C
ATOM   9988  CG  LEU G  15       0.922   8.716 -53.431  1.00 32.06          C
ATOM   9989  CD1 LEU G  15      -0.099   7.935 -52.586  1.00 31.38          C
ATOM   9990  CD2 LEU G  15       2.326   8.118 -53.351  1.00 31.51          C
ATOM   9991  N   GLY G  16       2.032   8.543 -57.756  1.00 31.00          N
ATOM   9992  CA  GLY G  16       2.013   7.904 -59.067  1.00 30.46          C
ATOM   9993  C   GLY G  16       2.143   8.822 -60.277  1.00 30.56          C
ATOM   9994  O   GLY G  16       2.080   8.337 -61.413  1.00 28.55          O
ATOM   9995  N   THR G  17       2.280  10.133 -60.048  1.00 29.33          N
ATOM   9996  CA  THR G  17       2.552  11.079 -61.111  1.00 29.35          C
ATOM   9997  C   THR G  17       3.997  11.582 -60.984  1.00 29.00          C
ATOM   9998  O   THR G  17       4.704  11.233 -60.043  1.00 28.41          O
```

```
ATOM    9999  CB  THR G  17      1.571  12.262 -61.096  1.00 30.21           C
ATOM   10000  OG1 THR G  17      1.853  13.129 -59.992  1.00 30.62           O
ATOM   10001  CG2 THR G  17      0.147  11.762 -60.993  1.00 30.45           C
ATOM   10002  N   SER G  18      4.415  12.418 -61.933  1.00 28.41           N
ATOM   10003  CA  SER G  18      5.819  12.744 -62.093  1.00 27.60           C
ATOM   10004  C   SER G  18      6.196  14.060 -61.388  1.00 26.68           C
ATOM   10005  O   SER G  18      5.426  15.024 -61.374  1.00 27.23           O
ATOM   10006  CB  SER G  18      6.131  12.839 -63.581  1.00 27.65           C
ATOM   10007  OG  SER G  18      5.775  14.136 -64.022  1.00 29.91           O
ATOM   10008  N   ALA G  19      7.394  14.080 -60.813  1.00 26.10           N
ATOM   10009  CA  ALA G  19      7.924  15.256 -60.144  1.00 25.43           C
ATOM   10010  C   ALA G  19      9.150  15.775 -60.919  1.00 24.96           C
ATOM   10011  O   ALA G  19      9.883  14.980 -61.516  1.00 25.18           O
ATOM   10012  CB  ALA G  19      8.322  14.916 -58.736  1.00 24.61           C
ATOM   10013  N   SER G  20      9.333  17.102 -60.891  1.00 24.23           N
ATOM   10014  CA  SER G  20     10.430  17.789 -61.565  1.00 23.99           C
ATOM   10015  C   SER G  20     11.085  18.760 -60.590  1.00 22.69           C
ATOM   10016  O   SER G  20     10.394  19.584 -59.991  1.00 23.14           O
ATOM   10017  CB  SER G  20      9.933  18.566 -62.785  1.00 24.97           C
ATOM   10018  OG  SER G  20     11.013  19.078 -63.564  1.00 26.91           O
ATOM   10019  N   ILE G  21     12.412  18.664 -60.462  1.00 21.09           N
ATOM   10020  CA  ILE G  21     13.198  19.484 -59.528  1.00 21.99           C
ATOM   10021  C   ILE G  21     14.405  20.076 -60.270  1.00 21.95           C
ATOM   10022  O   ILE G  21     15.229  19.348 -60.825  1.00 20.74           O
ATOM   10023  CB  ILE G  21     13.614  18.639 -58.276  1.00 21.72           C
ATOM   10024  CG1 ILE G  21     12.374  18.258 -57.445  1.00 23.33           C
ATOM   10025  CG2 ILE G  21     14.560  19.354 -57.358  1.00 21.08           C
ATOM   10026  CD1 ILE G  21     12.661  17.313 -56.255  1.00 22.93           C
ATOM   10027  N   SER G  22     14.480  21.402 -60.276  1.00 21.36           N
ATOM   10028  CA  SER G  22     15.494  22.134 -60.979  1.00 21.01           C
ATOM   10029  C   SER G  22     16.633  22.550 -60.075  1.00 19.49           C
ATOM   10030  O   SER G  22     16.465  22.697 -58.877  1.00 18.81           O
ATOM   10031  CB  SER G  22     14.905  23.430 -61.519  1.00 22.64           C
ATOM   10032  OG  SER G  22     13.835  23.170 -62.395  1.00 29.58           O
ATOM   10033  N   CYS G  23     17.788  22.765 -60.679  1.00 17.26           N
ATOM   10034  CA  CYS G  23     18.953  23.323 -59.993  1.00 17.04           C
ATOM   10035  C   CYS G  23     19.772  24.095 -61.033  1.00 15.31           C
ATOM   10036  O   CYS G  23     19.684  23.816 -62.227  1.00 14.86           O
ATOM   10037  CB  CYS G  23     19.807  22.222 -59.306  1.00 17.12           C
ATOM   10038  SG  CYS G  23     21.339  22.864 -58.519  1.00 18.71           S
ATOM   10039  N   ARG G  24     20.485  25.130 -60.608  1.00 15.17           N
ATOM   10040  CA  ARG G  24     21.506  25.718 -61.472  1.00 15.72           C
ATOM   10041  C   ARG G  24     22.755  26.075 -60.713  1.00 12.71           C
ATOM   10042  O   ARG G  24     22.744  26.198 -59.507  1.00 13.89           O
ATOM   10043  CB  ARG G  24     20.983  26.935 -62.237  1.00 17.59           C
ATOM   10044  CG  ARG G  24     20.675  28.124 -61.371  1.00 20.33           C
ATOM   10045  CD  ARG G  24     19.923  29.168 -62.166  1.00 21.05           C
ATOM   10046  NE  ARG G  24     19.098  29.984 -61.278  1.00 24.63           N
ATOM   10047  CZ  ARG G  24     19.548  31.026 -60.610  1.00 23.63           C
ATOM   10048  NH1 ARG G  24     20.811  31.402 -60.745  1.00 25.92           N
ATOM   10049  NH2 ARG G  24     18.732  31.701 -59.820  1.00 24.16           N
ATOM   10050  N   SER G  25     23.832  26.242 -61.473  1.00 13.15           N
ATOM   10051  CA  SER G  25     25.136  26.517 -60.983  1.00 15.11           C
ATOM   10052  C   SER G  25     25.627  27.864 -61.510  1.00 14.07           C
ATOM   10053  O   SER G  25     25.373  28.238 -62.683  1.00 12.98           O
ATOM   10054  CB  SER G  25     26.111  25.460 -61.459  1.00 16.39           C
ATOM   10055  OG  SER G  25     27.253  25.563 -60.668  1.00 22.12           O
ATOM   10056  N   SER G  26     26.435  28.519 -60.682  1.00 14.95           N
ATOM   10057  CA  SER G  26     27.060  29.802 -61.058  1.00 15.09           C
ATOM   10058  C   SER G  26     28.268  29.664 -61.968  1.00 16.01           C
ATOM   10059  O   SER G  26     28.725  30.648 -62.514  1.00 17.41           O
ATOM   10060  CB  SER G  26     27.444  30.611 -59.822  1.00 15.86           C
ATOM   10061  OG  SER G  26     28.352  29.885 -59.008  1.00 17.01           O
ATOM   10062  N   LYS G  27     28.822  28.463 -62.084  1.00 15.55           N
ATOM   10063  CA  LYS G  27     29.938  28.173 -62.994  1.00 16.98           C
```

```
ATOM   10064  C    LYS  G    27      29.565  26.858  -63.656  1.00  14.97        C
ATOM   10065  O    LYS  G    27      28.821  26.094  -63.083  1.00  13.69        O
ATOM   10066  CB   LYS  G    27      31.252  27.976  -62.249  1.00  18.99        C
ATOM   10067  CG   LYS  G    27      31.699  29.051  -61.296  1.00  22.67        C
ATOM   10068  CD   LYS  G    27      33.167  28.742  -60.915  1.00  23.68        C
ATOM   10069  CE   LYS  G    27      33.755  29.635  -59.838  1.00  26.31        C
ATOM   10070  NZ   LYS  G    27      34.949  28.958  -59.206  1.00  26.16        N
ATOM   10071  N    SER  G    27A     30.057  26.620  -64.866  1.00  14.44        N
ATOM   10072  CA   SER  G    27A     29.789  25.376  -65.549  1.00  14.84        C
ATOM   10073  C    SER  G    27A     30.517  24.251  -64.839  1.00  14.03        C
ATOM   10074  O    SER  G    27A     31.676  24.412  -64.461  1.00  15.11        O
ATOM   10075  CB   SER  G    27A     30.275  25.436  -66.993  1.00  15.72        C
ATOM   10076  OG   SER  G    27A     30.201  24.145  -67.582  1.00  12.04        O
ATOM   10077  N    LEU  G    27B     29.818  23.126  -64.710  1.00  14.53        N
ATOM   10078  CA   LEU  G    27B     30.299  21.885  -64.110  1.00  15.93        C
ATOM   10079  C    LEU  G    27B     30.661  20.815  -65.162  1.00  17.03        C
ATOM   10080  O    LEU  G    27B     30.982  19.666  -64.813  1.00  19.74        O
ATOM   10081  CB   LEU  G    27B     29.216  21.314  -63.194  1.00  14.39        C
ATOM   10082  CG   LEU  G    27B     28.540  22.216  -62.157  1.00  12.46        C
ATOM   10083  CD1  LEU  G    27B     27.535  21.437  -61.302  1.00  12.17        C
ATOM   10084  CD2  LEU  G    27B     29.592  22.827  -61.296  1.00  13.91        C
ATOM   10085  N    LEU  G    27C     30.590  21.170  -66.449  1.00  18.15        N
ATOM   10086  CA   LEU  G    27C     30.988  20.237  -67.520  1.00  17.71        C
ATOM   10087  C    LEU  G    27C     32.483  20.421  -67.657  1.00  18.82        C
ATOM   10088  O    LEU  G    27C     32.971  21.536  -67.890  1.00  18.79        O
ATOM   10089  CB   LEU  G    27C     30.264  20.549  -68.832  1.00  18.30        C
ATOM   10090  CG   LEU  G    27C     30.776  19.964  -70.144  1.00  18.06        C
ATOM   10091  CD1  LEU  G    27C     30.786  18.432  -70.080  1.00  16.30        C
ATOM   10092  CD2  LEU  G    27C     29.914  20.466  -71.316  1.00  19.04        C
ATOM   10093  N    HIS  G    27D     33.200  19.314  -67.493  1.00  17.65        N
ATOM   10094  CA   HIS  G    27D     34.638  19.273  -67.407  1.00  18.61        C
ATOM   10095  C    HIS  G    27D     35.177  18.968  -68.806  1.00  18.95        C
ATOM   10096  O    HIS  G    27D     34.465  18.451  -69.633  1.00  19.48        O
ATOM   10097  CB   HIS  G    27D     34.999  18.127  -66.437  1.00  19.10        C
ATOM   10098  CG   HIS  G    27D     36.461  18.004  -66.139  1.00  19.32        C.
ATOM   10099  ND1  HIS  G    27D     37.060  18.645  -65.078  1.00  24.03        N
ATOM   10100  CD2  HIS  G    27D     37.441  17.292  -66.748  1.00  20.14        C
ATOM   10101  CE1  HIS  G    27D     38.350  18.343  -65.055  1.00  24.01        C
ATOM   10102  NE2  HIS  G    27D     38.606  17.521  -66.059  1.00  23.05        N
ATOM   10103  N    SER  G    27E     36.448  19.228  -69.052  1.00  20.81        N
ATOM   10104  CA   SER  G    27E     37.065  18.865  -70.334  1.00  21.10        C
ATOM   10105  C    SER  G    27E     36.982  17.372  -70.669  1.00  20.82        C
ATOM   10106  O    SER  G    27E     37.037  17.005  -71.841  1.00  23.51        O
ATOM   10107  CB   SER  G    27E     38.514  19.373  -70.410  1.00  22.08        C
ATOM   10108  OG   SER  G    27E     39.293  18.909  -69.321  1.00  25.79        O
ATOM   10109  N    ASP  G    28      36.780  16.524  -69.662  1.00  20.10        N
ATOM   10110  CA   ASP  G    28      36.766  15.075  -69.844  1.00  19.55        C
ATOM   10111  C    ASP  G    28      35.399  14.550  -70.275  1.00  19.72        C
ATOM   10112  O    ASP  G    28      35.197  13.334  -70.437  1.00  19.82        O
ATOM   10113  CB   ASP  G    28      37.257  14.353  -68.578  1.00  21.20        C
ATOM   10114  CG   ASP  G    28      36.309  14.495  -67.373  1.00  23.29        C
ATOM   10115  OD1  ASP  G    28      35.198  15.060  -67.538  1.00  19.92        O
ATOM   10116  OD2  ASP  G    28      36.704  14.032  -66.250  1.00  24.52        O
ATOM   10117  N    GLY  G    29      34.438  15.449  -70.450  1.00  17.83        N
ATOM   10118  CA   GLY  G    29      33.143  15.052  -70.969  1.00  17.48        C
ATOM   10119  C    GLY  G    29      32.073  14.927  -69.914  1.00  17.72        C
ATOM   10120  O    GLY  G    29      30.887  14.869  -70.238  1.00  18.45        O
ATOM   10121  N    ILE  G    30      32.497  14.812  -68.651  1.00  16.85        N
ATOM   10122  CA   ILE  G    30      31.600  14.514  -67.559  1.00  16.40        C
ATOM   10123  C    ILE  G    30      31.108  15.849  -66.988  1.00  15.27        C
ATOM   10124  O    ILE  G    30      31.900  16.766  -66.843  1.00  17.77        O
ATOM   10125  CB   ILE  G    30      32.322  13.733  -66.450  1.00  17.01        C
ATOM   10126  CG1  ILE  G    30      32.595  12.301  -66.935  1.00  16.44        C
ATOM   10127  CG2  ILE  G    30      31.474  13.753  -65.152  1.00  16.15        C
ATOM   10128  CD1  ILE  G    30      33.650  11.546  -66.173  1.00  19.30        C
```

```
ATOM   10129   N    THR G  31      29.812   15.905  -66.684   1.00  16.30      N
ATOM   10130   CA   THR G  31      29.183   17.002  -65.953   1.00  15.72      C
ATOM   10131   C    THR G  31      29.080   16.528  -64.497   1.00  14.98      C
ATOM   10132   O    THR G  31      28.315   15.605  -64.161   1.00  13.00      O
ATOM   10133   CB   THR G  31      27.788   17.457  -66.564   1.00  16.28      C
ATOM   10134   OG1  THR G  31      27.929   17.740  -67.962   1.00  16.77      O
ATOM   10135   CG2  THR G  31      27.277   18.756  -65.895   1.00  18.36      C
ATOM   10136   N    TYR G  32      29.861   17.200  -63.654   1.00  13.44      N
ATOM   10137   CA   TYR G  32      30.026   16.844  -62.253   1.00  15.68      C
ATOM   10138   C    TYR G  32      28.962   17.423  -61.358   1.00  14.28      C
ATOM   10139   O    TYR G  32      29.250   18.174  -60.438   1.00  15.57      O
ATOM   10140   CB   TYR G  32      31.415   17.231  -61.796   1.00  17.18      C
ATOM   10141   CG   TYR G  32      32.464   16.328  -62.377   1.00  17.73      C
ATOM   10142   CD1  TYR G  32      33.074   16.638  -63.587   1.00  19.28      C
ATOM   10143   CD2  TYR G  32      32.845   15.170  -61.717   1.00  19.32      C
ATOM   10144   CE1  TYR G  32      34.054   15.816  -64.122   1.00  17.93      C
ATOM   10145   CE2  TYR G  32      33.810   14.327  -62.253   1.00  18.54      C
ATOM   10146   CZ   TYR G  32      34.400   14.660  -63.459   1.00  18.78      C
ATOM   10147   OH   TYR G  32      35.376   13.884  -64.021   1.00  19.38      O
ATOM   10148   N    LEU G  33      27.741   17.021  -61.659   1.00  13.82      N
ATOM   10149   CA   LEU G  33      26.537   17.373  -60.957   1.00  13.53      C
ATOM   10150   C    LEU G  33      26.063   16.147  -60.202   1.00  14.96      C
ATOM   10151   O    LEU G  33      26.017   15.030  -60.752   1.00  15.28      O
ATOM   10152   CB   LEU G  33      25.476   17.883  -61.951   1.00  13.71      C
ATOM   10153   CG   LEU G  33      24.150   18.373  -61.361   1.00  13.28      C
ATOM   10154   CD1  LEU G  33      23.234   17.287  -60.903   1.00  14.25      C
ATOM   10155   CD2  LEU G  33      24.374   19.390  -60.233   1.00  14.03      C
ATOM   10156   N    TYR G  34      25.744   16.344  -58.925   1.00  14.22      N
ATOM   10157   CA   TYR G  34      25.313   15.263  -58.056   1.00  13.78      C
ATOM   10158   C    TYR G  34      24.006   15.644  -57.426   1.00  14.60      C
ATOM   10159   O    TYR G  34      23.772   16.814  -57.147   1.00  11.96      O
ATOM   10160   CB   TYR G  34      26.351   15.011  -56.955   1.00  13.99      C
ATOM   10161   CG   TYR G  34      27.661   14.463  -57.469   1.00  13.68      C
ATOM   10162   CD1  TYR G  34      28.515   15.263  -58.244   1.00  13.50      C
ATOM   10163   CD2  TYR G  34      28.048   13.144  -57.193   1.00  14.90      C
ATOM   10164   CE1  TYR G  34      29.700   14.788  -58.747   1.00  12.58      C
ATOM   10165   CE2  TYR G  34      29.279   12.655  -57.664   1.00  13.02      C
ATOM   10166   CZ   TYR G  34      30.079   13.462  -58.456   1.00  13.06      C
ATOM   10167   OH   TYR G  34      31.302   13.056  -58.919   1.00  11.90      O
ATOM   10168   N    TRP G  35      23.154   14.656  -57.199   1.00  13.24      N
ATOM   10169   CA   TRP G  35      21.948   14.855  -56.410   1.00  13.69      C
ATOM   10170   C    TRP G  35      22.013   13.961  -55.157   1.00  14.06      C
ATOM   10171   O    TRP G  35      22.436   12.786  -55.235   1.00  12.85      O
ATOM   10172   CB   TRP G  35      20.694   14.496  -57.220   1.00  15.37      C
ATOM   10173   CG   TRP G  35      20.345   15.406  -58.356   1.00  14.73      C
ATOM   10174   CD1  TRP G  35      20.612   15.195  -59.674   1.00  16.67      C
ATOM   10175   CD2  TRP G  35      19.604   16.632  -58.288   1.00  16.54      C
ATOM   10176   NE1  TRP G  35      20.105   16.221  -60.433   1.00  15.62      N
ATOM   10177   CE2  TRP G  35      19.500   17.129  -59.601   1.00  14.63      C
ATOM   10178   CE3  TRP G  35      19.036   17.366  -57.241   1.00  16.53      C
ATOM   10179   CZ2  TRP G  35      18.843   18.332  -59.900   1.00  17.12      C
ATOM   10180   CZ3  TRP G  35      18.366   18.588  -57.551   1.00  16.06      C
ATOM   10181   CH2  TRP G  35      18.285   19.034  -58.861   1.00  15.39      C
ATOM   10182   N    TYR G  36      21.573   14.498  -54.025   1.00  11.28      N
ATOM   10183   CA   TYR G  36      21.459   13.749  -52.776   1.00  11.56      C
ATOM   10184   C    TYR G  36      20.022   13.836  -52.309   1.00  13.69      C
ATOM   10185   O    TYR G  36      19.368   14.865  -52.487   1.00  12.82      O
ATOM   10186   CB   TYR G  36      22.415   14.266  -51.686   1.00  11.78      C
ATOM   10187   CG   TYR G  36      23.883   14.137  -52.048   1.00  11.06      C
ATOM   10188   CD1  TYR G  36      24.537   15.117  -52.763   1.00  12.06      C
ATOM   10189   CD2  TYR G  36      24.596   12.961  -51.740   1.00  11.89      C
ATOM   10190   CE1  TYR G  36      25.893   14.971  -53.120   1.00  11.16      C
ATOM   10191   CE2  TYR G  36      25.914   12.802  -52.088   1.00  12.95      C
ATOM   10192   CZ   TYR G  36      26.570   13.775  -52.784   1.00  11.43      C
ATOM   10193   OH   TYR G  36      27.891   13.619  -53.124   1.00  10.56      O
```

| ATOM | 10194 | N | LEU | G | 37 | 19.531 | 12.748 | -51.713 | 1.00 | 13.64 | N |
|------|-------|-----|-----|---|----|--------|--------|---------|------|-------|---|
| ATOM | 10195 | CA | LEU | G | 37 | 18.250 | 12.760 | -51.045 | 1.00 | 13.58 | C |
| ATOM | 10196 | C | LEU | G | 37 | 18.437 | 12.595 | -49.547 | 1.00 | 14.27 | C |
| ATOM | 10197 | O | LEU | G | 37 | 19.016 | 11.598 | -49.079 | 1.00 | 11.95 | O |
| ATOM | 10198 | CB | LEU | G | 37 | 17.364 | 11.636 | -51.568 | 1.00 | 14.39 | C |
| ATOM | 10199 | CG | LEU | G | 37 | 16.041 | 11.411 | -50.846 | 1.00 | 13.01 | C |
| ATOM | 10200 | CD1 | LEU | G | 37 | 15.207 | 12.709 | -50.778 | 1.00 | 11.54 | C |
| ATOM | 10201 | CD2 | LEU | G | 37 | 15.280 | 10.338 | -51.595 | 1.00 | 13.99 | C |
| ATOM | 10202 | N | GLN | G | 38 | 17.889 | 13.533 | -48.786 | 1.00 | 13.52 | N |
| ATOM | 10203 | CA | GLN | G | 38 | 17.746 | 13.349 | -47.347 | 1.00 | 15.22 | C |
| ATOM | 10204 | C | GLN | G | 38 | 16.290 | 13.043 | -46.937 | 1.00 | 15.85 | C |
| ATOM | 10205 | O | GLN | G | 38 | 15.449 | 13.936 | -46.884 | 1.00 | 15.67 | O |
| ATOM | 10206 | CB | GLN | G | 38 | 18.257 | 14.614 | -46.624 | 1.00 | 14.75 | C |
| ATOM | 10207 | CG | GLN | G | 38 | 18.312 | 14.432 | -45.122 | 1.00 | 15.35 | C |
| ATOM | 10208 | CD | GLN | G | 38 | 19.062 | 15.554 | -44.459 | 1.00 | 16.00 | C |
| ATOM | 10209 | OE1 | GLN | G | 38 | 19.025 | 16.705 | -44.929 | 1.00 | 19.35 | O |
| ATOM | 10210 | NE2 | GLN | G | 38 | 19.719 | 15.243 | -43.349 | 1.00 | 16.22 | N |
| ATOM | 10211 | N | LYS | G | 39 | 16.001 | 11.778 | -46.625 | 1.00 | 17.12 | N |
| ATOM | 10212 | CA | LYS | G | 39 | 14.679 | 11.355 | -46.149 | 1.00 | 18.39 | C |
| ATOM | 10213 | C | LYS | G | 39 | 14.460 | 11.844 | -44.730 | 1.00 | 18.42 | C |
| ATOM | 10214 | O | LYS | G | 39 | 15.437 | 12.031 | -43.988 | 1.00 | 17.27 | O |
| ATOM | 10215 | CB | LYS | G | 39 | 14.549 | 9.825 | -46.208 | 1.00 | 19.88 | C |
| ATOM | 10216 | CG | LYS | G | 39 | 14.452 | 9.305 | -47.637 | 1.00 | 21.39 | C |
| ATOM | 10217 | CD | LYS | G | 39 | 14.130 | 7.837 | -47.697 | 1.00 | 21.49 | C |
| ATOM | 10218 | CE | LYS | G | 39 | 14.327 | 7.284 | -49.085 | 1.00 | 23.55 | C |
| ATOM | 10219 | NZ | LYS | G | 39 | 13.935 | 5.850 | -49.174 | 1.00 | 26.20 | N |
| ATOM | 10220 | N | PRO | G | 40 | 13.189 | 12.064 | -44.339 | 1.00 | 19.65 | N |
| ATOM | 10221 | CA | PRO | G | 40 | 12.908 | 12.608 | -43.005 | 1.00 | 20.54 | C |
| ATOM | 10222 | C | PRO | G | 40 | 13.577 | 11.775 | -41.918 | 1.00 | 22.10 | C |
| ATOM | 10223 | O | PRO | G | 40 | 13.500 | 10.530 | -41.921 | 1.00 | 22.02 | O |
| ATOM | 10224 | CB | PRO | G | 40 | 11.376 | 12.560 | -42.906 | 1.00 | 21.00 | C |
| ATOM | 10225 | CG | PRO | G | 40 | 10.909 | 12.582 | -44.314 | 1.00 | 19.55 | C |
| ATOM | 10226 | CD | PRO | G | 40 | 11.954 | 11.884 | -45.125 | 1.00 | 19.09 | C |
| ATOM | 10227 | N | GLY | G | 41 | 14.311 | 12.478 | -41.061 | 1.00 | 22.44 | N |
| ATOM | 10228 | CA | GLY | G | 41 | 15.087 | 11.863 | -39.996 | 1.00 | 22.31 | C |
| ATOM | 10229 | C | GLY | G | 41 | 16.258 | 11.006 | -40.396 | 1.00 | 22.31 | C |
| ATOM | 10230 | O | GLY | G | 41 | 16.731 | 10.242 | -39.577 | 1.00 | 23.28 | O |
| ATOM | 10231 | N | GLN | G | 42 | 16.739 | 11.125 | -41.634 | 1.00 | 20.65 | N |
| ATOM | 10232 | CA | GLN | G | 42 | 17.903 | 10.388 | -42.085 | 1.00 | 20.73 | C |
| ATOM | 10233 | C | GLN | G | 42 | 18.991 | 11.359 | -42.466 | 1.00 | 19.48 | C |
| ATOM | 10234 | O | GLN | G | 42 | 18.763 | 12.557 | -42.500 | 1.00 | 19.77 | O |
| ATOM | 10235 | CB | GLN | G | 42 | 17.573 | 9.534 | -43.314 | 1.00 | 21.50 | C |
| ATOM | 10236 | CG | GLN | G | 42 | 16.540 | 8.381 | -43.101 | 1.00 | 22.97 | C |
| ATOM | 10237 | CD | GLN | G | 42 | 16.533 | 7.381 | -44.283 | 1.00 | 25.71 | C |
| ATOM | 10238 | OE1 | GLN | G | 42 | 15.748 | 6.418 | -44.299 | 1.00 | 30.60 | O |
| ATOM | 10239 | NE2 | GLN | G | 42 | 17.446 | 7.580 | -45.254 | 1.00 | 27.87 | N |
| ATOM | 10240 | N | SER | G | 43 | 20.172 | 10.811 | -42.764 | 1.00 | 19.42 | N |
| ATOM | 10241 | CA | SER | G | 43 | 21.279 | 11.547 | -43.367 | 1.00 | 17.09 | C |
| ATOM | 10242 | C | SER | G | 43 | 21.018 | 11.666 | -44.872 | 1.00 | 16.20 | C |
| ATOM | 10243 | O | SER | G | 43 | 20.174 | 10.960 | -45.421 | 1.00 | 15.37 | O |
| ATOM | 10244 | CB | SER | G | 43 | 22.583 | 10.763 | -43.183 | 1.00 | 17.39 | C |
| ATOM | 10245 | OG | SER | G | 43 | 22.779 | 10.427 | -41.834 | 1.00 | 16.82 | O |
| ATOM | 10246 | N | PRO | G | 44 | 21.744 | 12.563 | -45.558 | 1.00 | 14.21 | N |
| ATOM | 10247 | CA | PRO | G | 44 | 21.688 | 12.514 | -47.012 | 1.00 | 13.54 | C |
| ATOM | 10248 | C | PRO | G | 44 | 22.250 | 11.171 | -47.501 | 1.00 | 13.36 | C |
| ATOM | 10249 | O | PRO | G | 44 | 23.093 | 10.576 | -46.832 | 1.00 | 12.46 | O |
| ATOM | 10250 | CB | PRO | G | 44 | 22.619 | 13.663 | -47.437 | 1.00 | 13.29 | C |
| ATOM | 10251 | CG | PRO | G | 44 | 22.670 | 14.547 | -46.241 | 1.00 | 10.51 | C |
| ATOM | 10252 | CD | PRO | G | 44 | 22.615 | 13.640 | -45.068 | 1.00 | 14.29 | C |
| ATOM | 10253 | N | HIS | G | 45 | 21.780 | 10.725 | -48.647 | 1.00 | 13.03 | N |
| ATOM | 10254 | CA | HIS | G | 45 | 22.481 | 9.712 | -49.410 | 1.00 | 13.57 | C |
| ATOM | 10255 | C | HIS | G | 45 | 22.544 | 10.072 | -50.893 | 1.00 | 13.23 | C |
| ATOM | 10256 | O | HIS | G | 45 | 21.646 | 10.775 | -51.430 | 1.00 | 14.76 | O |
| ATOM | 10257 | CB | HIS | G | 45 | 21.784 | 8.348 | -49.224 | 1.00 | 14.65 | C |
| ATOM | 10258 | CG | HIS | G | 45 | 20.402 | 8.298 | -49.789 | 1.00 | 15.77 | C |

```
ATOM   10259  ND1 HIS G  45     19.292   8.750 -49.099  1.00 18.50         N
ATOM   10260  CD2 HIS G  45     19.948   7.854 -50.982  1.00 17.86         C
ATOM   10261  CE1 HIS G  45     18.215   8.560 -49.833  1.00 18.49         C
ATOM   10262  NE2 HIS G  45     18.587   8.029 -50.987  1.00 16.88         N
ATOM   10263  N   LEU G  46     23.583   9.583 -51.564  1.00 14.17         N
ATOM   10264  CA  LEU G  46     23.722   9.758 -53.016  1.00 14.53         C
ATOM   10265  C   LEU G  46     22.588   9.124 -53.819  1.00 17.87         C
ATOM   10266  O   LEU G  46     22.265   7.926 -53.652  1.00 18.93         O
ATOM   10267  CB  LEU G  46     25.042   9.193 -53.514  1.00 14.07         C
ATOM   10268  CG  LEU G  46     25.371   9.500 -54.969  1.00 11.65         C
ATOM   10269  CD1 LEU G  46     25.635  11.049 -55.210  1.00 12.17         C
ATOM   10270  CD2 LEU G  46     26.549   8.736 -55.456  1.00 13.19         C
ATOM   10271  N   LEU G  47     22.030   9.926 -54.721  1.00 18.62         N
ATOM   10272  CA  LEU G  47     20.937   9.513 -55.622  1.00 18.56         C
ATOM   10273  C   LEU G  47     21.433   9.430 -57.085  1.00 19.23         C
ATOM   10274  O   LEU G  47     21.254   8.402 -57.769  1.00 18.43         O
ATOM   10275  CB  LEU G  47     19.819  10.544 -55.453  1.00 18.56         C
ATOM   10276  CG  LEU G  47     18.336  10.343 -55.735  1.00 21.12         C
ATOM   10277  CD1 LEU G  47     18.073  10.523 -57.197  1.00 24.52         C
ATOM   10278  CD2 LEU G  47     17.804   9.013 -55.241  1.00 23.17         C
ATOM   10279  N   ILE G  48     22.086  10.503 -57.540  1.00 18.18         N
ATOM   10280  CA  ILE G  48     22.652  10.627 -58.892  1.00 18.71         C
ATOM   10281  C   ILE G  48     24.043  11.201 -58.833  1.00 18.42         C
ATOM   10282  O   ILE G  48     24.275  12.188 -58.117  1.00 20.02         O
ATOM   10283  CB  ILE G  48     21.835  11.704 -59.756  1.00 18.57         C
ATOM   10284  CG1 ILE G  48     20.369  11.340 -59.867  1.00 21.46         C
ATOM   10285  CG2 ILE G  48     22.450  11.926 -61.146  1.00 18.87         C
ATOM   10286  CD1 ILE G  48     20.120  10.023 -60.564  1.00 18.85         C
ATOM   10287  N   TYR G  49     24.952  10.662 -59.646  1.00 17.64         N
ATOM   10288  CA  TYR G  49     26.269  11.224 -59.832  1.00 17.09         C
ATOM   10289  C   TYR G  49     26.563  11.484 -61.315  1.00 16.98         C
ATOM   10290  O   TYR G  49     25.933  10.915 -62.191  1.00 17.33         O
ATOM   10291  CB  TYR G  49     27.352  10.326 -59.239  1.00 19.53         C
ATOM   10292  CG  TYR G  49     27.427   8.938 -59.863  1.00 18.14         C
ATOM   10293  CD1 TYR G  49     26.560   7.952 -59.471  1.00 20.24         C
ATOM   10294  CD2 TYR G  49     28.358   8.642 -60.849  1.00 19.97         C
ATOM   10295  CE1 TYR G  49     26.587   6.698 -60.036  1.00 20.18         C
ATOM   10296  CE2 TYR G  49     28.407   7.341 -61.432  1.00 19.11         C
ATOM   10297  CZ  TYR G  49     27.516   6.390 -61.000  1.00 20.05         C
ATOM   10298  OH  TYR G  49     27.540   5.092 -61.526  1.00 22.35         O
ATOM   10299  N   HIS G  50     27.480  12.413 -61.585  1.00 16.45         N
ATOM   10300  CA  HIS G  50     27.927  12.665 -62.936  1.00 17.53         C
ATOM   10301  C   HIS G  50     26.729  13.055 -63.832  1.00 16.73         C
ATOM   10302  O   HIS G  50     26.629  12.633 -64.998  1.00 17.80         O
ATOM   10303  CB  HIS G  50     28.697  11.433 -63.445  1.00 18.23         C
ATOM   10304  CG  HIS G  50     30.052  11.284 -62.823  1.00 18.21         C
ATOM   10305  ND1 HIS G  50     30.992  10.385 -63.290  1.00 18.01         N
ATOM   10306  CD2 HIS G  50     30.653  11.961 -61.809  1.00 19.29         C
ATOM   10307  CE1 HIS G  50     32.094  10.488 -62.565  1.00 19.01         C
ATOM   10308  NE2 HIS G  50     31.921  11.446 -61.670  1.00 19.78         N
ATOM   10309  N   LEU G  51     25.802  13.804 -63.228  1.00 14.41         N
ATOM   10310  CA  LEU G  51     24.648  14.446 -63.880  1.00 15.20         C
ATOM   10311  C   LEU G  51     23.498  13.517 -64.120  1.00 15.81         C
ATOM   10312  O   LEU G  51     22.348  13.849 -63.885  1.00 13.76         O
ATOM   10313  CB  LEU G  51     25.012  15.092 -65.198  1.00 14.75         C
ATOM   10314  CG  LEU G  51     23.852  15.822 -65.904  1.00 13.52         C
ATOM   10315  CD1 LEU G  51     23.289  16.978 -65.083  1.00 15.71         C
ATOM   10316  CD2 LEU G  51     24.325  16.328 -67.217  1.00 15.78         C
ATOM   10317  N   SER G  52     23.803  12.319 -64.608  1.00 16.42         N
ATOM   10318  CA  SER G  52     22.750  11.476 -65.064  1.00 17.12         C
ATOM   10319  C   SER G  52     22.881   9.986 -64.688  1.00 17.19         C
ATOM   10320  O   SER G  52     22.024   9.224 -65.062  1.00 18.16         O
ATOM   10321  CB  SER G  52     22.603  11.686 -66.578  1.00 15.94         C
ATOM   10322  OG  SER G  52     23.799  11.338 -67.242  1.00 20.11         O
ATOM   10323  N   ASN G  53     23.880   9.605 -63.898  1.00 18.36         N
```

```
ATOM  10324  CA  ASN G  53    24.064   8.173 -63.470  1.00 19.98        C
ATOM  10325  C   ASN G  53    23.386   7.837 -62.141  1.00 20.70        C
ATOM  10326  O   ASN G  53    23.591   8.516 -61.152  1.00 21.32        O
ATOM  10327  CB  ASN G  53    25.542   7.876 -63.408  1.00 20.83        C
ATOM  10328  CG  ASN G  53    26.214   8.071 -64.759  1.00 25.20        C
ATOM  10329  OD1 ASN G  53    25.790   7.485 -65.748  1.00 29.32        O
ATOM  10330  ND2 ASN G  53    27.219   8.925 -64.823  1.00 25.81        N
ATOM  10331  N   LEU G  54    22.539   6.815 -62.122  1.00 21.48        N
ATOM  10332  CA  LEU G  54    21.804   6.468 -60.906  1.00 22.88        C
ATOM  10333  C   LEU G  54    22.737   5.784 -59.942  1.00 23.97        C
ATOM  10334  O   LEU G  54    23.595   4.998 -60.349  1.00 25.34        O
ATOM  10335  CB  LEU G  54    20.577   5.579 -61.187  1.00 22.24        C
ATOM  10336  CG  LEU G  54    19.275   6.297 -61.535  1.00 24.21        C
ATOM  10337  CD1 LEU G  54    19.412   7.059 -62.862  1.00 26.58        C
ATOM  10338  CD2 LEU G  54    18.123   5.315 -61.607  1.00 24.66        C
ATOM  10339  N   ALA G  55    22.584   6.084 -58.658  1.00 24.20        N
ATOM  10340  CA  ALA G  55    23.320   5.362 -57.637  1.00 25.57        C
ATOM  10341  C   ALA G  55    22.651   3.993 -57.462  1.00 26.12        C
ATOM  10342  O   ALA G  55    21.461   3.828 -57.743  1.00 25.38        O
ATOM  10343  CB  ALA G  55    23.327   6.143 -56.332  1.00 24.67        C
ATOM  10344  N   SER G  56    23.414   3.013 -56.998  1.00 29.83        N
ATOM  10345  CA  SER G  56    22.861   1.676 -56.704  1.00 29.79        C
ATOM  10346  C   SER G  56    21.630   1.783 -55.793  1.00 31.01        C
ATOM  10347  O   SER G  56    21.627   2.565 -54.831  1.00 32.33        O
ATOM  10348  CB  SER G  56    23.899   0.831 -55.996  1.00 30.91        C
ATOM  10349  OG  SER G  56    24.153   1.366 -54.713  1.00 31.59        O
ATOM  10350  N   GLY G  57    20.594   1.000 -56.097  1.00 30.41        N
ATOM  10351  CA  GLY G  57    19.431   0.910 -55.244  1.00 29.63        C
ATOM  10352  C   GLY G  57    18.411   1.968 -55.556  1.00 30.46        C
ATOM  10353  O   GLY G  57    17.276   1.894 -55.074  1.00 30.32        O
ATOM  10354  N   VAL G  58    18.803   2.956 -56.369  1.00 29.63        N
ATOM  10355  CA  VAL G  58    17.885   3.986 -56.813  1.00 29.15        C
ATOM  10356  C   VAL G  58    16.978   3.425 -57.916  1.00 29.89        C
ATOM  10357  O   VAL G  58    17.477   2.877 -58.901  1.00 30.55        O
ATOM  10358  CB  VAL G  58    18.646   5.231 -57.312  1.00 28.02        C
ATOM  10359  CG1 VAL G  58    17.673   6.313 -57.757  1.00 27.13        C
ATOM  10360  CG2 VAL G  58    19.553   5.773 -56.207  1.00 27.87        C
ATOM  10361  N   PRO G  59    15.646   3.561 -57.756  1.00 30.97        N
ATOM  10362  CA  PRO G  59    14.704   3.122 -58.778  1.00 31.32        C
ATOM  10363  C   PRO G  59    14.826   3.930 -60.083  1.00 31.32        C
ATOM  10364  O   PRO G  59    15.147   5.125 -60.059  1.00 32.33        O
ATOM  10365  CB  PRO G  59    13.327   3.362 -58.136  1.00 31.24        C
ATOM  10366  CG  PRO G  59    13.572   3.668 -56.747  1.00 31.76        C
ATOM  10367  CD  PRO G  59    14.953   4.154 -56.604  1.00 31.05        C
ATOM  10368  N   ASP G  60    14.569   3.263 -61.206  1.00 31.19        N
ATOM  10369  CA  ASP G  60    14.713   3.864 -62.533  1.00 31.05        C
ATOM  10370  C   ASP G  60    13.673   4.940 -62.808  1.00 29.91        C
ATOM  10371  O   ASP G  60    13.672   5.557 -63.868  1.00 29.62        O
ATOM  10372  CB  ASP G  60    14.691   2.781 -63.631  1.00 33.94        C
ATOM  10373  CG  ASP G  60    13.406   1.937 -63.625  1.00 37.03        C
ATOM  10374  OD1 ASP G  60    12.299   2.499 -63.434  1.00 38.63        O
ATOM  10375  OD2 ASP G  60    13.505   0.696 -63.825  1.00 43.20        O
ATOM  10376  N   ARG G  61    12.778   5.155 -61.848  1.00 27.96        N
ATOM  10377  CA  ARG G  61    11.825   6.251 -61.909  1.00 27.36        C
ATOM  10378  C   ARG G  61    12.557   7.575 -61.835  1.00 24.71        C
ATOM  10379  O   ARG G  61    12.020   8.616 -62.236  1.00 26.33        O
ATOM  10380  CB  ARG G  61    10.817   6.132 -60.763  1.00 28.18        C
ATOM  10381  CG  ARG G  61    10.027   4.823 -60.814  1.00 30.85        C
ATOM  10382  CD  ARG G  61     9.046   4.627 -59.640  1.00 31.40        C
ATOM  10383  NE  ARG G  61     9.700   4.578 -58.323  1.00 33.96        N
ATOM  10384  CZ  ARG G  61     9.880   5.621 -57.515  1.00 32.67        C
ATOM  10385  NH1 ARG G  61     9.466   6.825 -57.881  1.00 32.74        N
ATOM  10386  NH2 ARG G  61    10.486   5.444 -56.348  1.00 32.44        N
ATOM  10387  N   PHE G  62    13.777   7.539 -61.305  1.00 23.00        N
ATOM  10388  CA  PHE G  62    14.618   8.737 -61.190  1.00 22.33        C
```

```
ATOM  10389  C    PHE G  62     15.528    8.908  -62.401  1.00 21.01        C
ATOM  10390  O    PHE G  62     16.137    7.935  -62.867  1.00 21.70        O
ATOM  10391  CB   PHE G  62     15.510    8.626  -59.964  1.00 22.18        C
ATOM  10392  CG   PHE G  62     14.772    8.767  -58.653  1.00 23.69        C
ATOM  10393  CD1  PHE G  62     14.277    7.655  -57.988  1.00 22.45        C
ATOM  10394  CD2  PHE G  62     14.594   10.017  -58.081  1.00 21.92        C
ATOM  10395  CE1  PHE G  62     13.614    7.798  -56.776  1.00 22.24        C
ATOM  10396  CE2  PHE G  62     13.932   10.165  -56.872  1.00 22.03        C
ATOM  10397  CZ   PHE G  62     13.447    9.071  -56.221  1.00 22.27        C
ATOM  10398  N    SER G  63     15.643   10.148  -62.876  1.00 19.34        N
ATOM  10399  CA   SER G  63     16.600   10.496  -63.917  1.00 18.00        C
ATOM  10400  C    SER G  63     16.943   11.968  -63.805  1.00 17.31        C
ATOM  10401  O    SER G  63     16.207   12.740  -63.186  1.00 16.32        O
ATOM  10402  CB   SER G  63     16.009   10.241  -65.288  1.00 18.93        C
ATOM  10403  OG   SER G  63     14.844   10.990  -65.522  1.00 17.04        O
ATOM  10404  N    SER G  64     18.040   12.337  -64.432  1.00 13.81        N
ATOM  10405  CA   SER G  64     18.498   13.725  -64.396  1.00 14.40        C
ATOM  10406  C    SER G  64     19.203   14.071  -65.690  1.00 11.41        C
ATOM  10407  O    SER G  64     19.828   13.243  -66.318  1.00 11.29        O
ATOM  10408  CB   SER G  64     19.421   13.891  -63.201  1.00 14.61        C
ATOM  10409  OG   SER G  64     20.099   15.147  -63.185  1.00 15.37        O
ATOM  10410  N    SER G  65     19.159   15.341  -66.066  1.00 10.95        N
ATOM  10411  CA   SER G  65     19.675   15.785  -67.311  1.00 11.04        C
ATOM  10412  C    SER G  65     20.082   17.217  -67.039  1.00  9.44        C
ATOM  10413  O    SER G  65     19.658   17.816  -66.048  1.00 11.32        O
ATOM  10414  CB   SER G  65     18.625   15.764  -68.438  1.00 13.74        C
ATOM  10415  OG   SER G  65     17.481   16.564  -68.159  1.00 14.00        O
ATOM  10416  N    GLY G  66     20.970   17.690  -67.860  1.00  7.35        N
ATOM  10417  CA   GLY G  66     21.298   19.125  -67.847  1.00  7.55        C
ATOM  10418  C    GLY G  66     22.489   19.516  -68.675  1.00  8.24        C
ATOM  10419  O    GLY G  66     23.158   18.701  -69.265  1.00 11.03        O
ATOM  10420  N    SER G  67     22.791   20.844  -68.681  1.00  8.74        N
ATOM  10421  CA   SER G  67     23.931   21.338  -69.319  1.00  9.20        C
ATOM  10422  C    SER G  67     25.049   21.378  -68.287  1.00 11.42        C
ATOM  10423  O    SER G  67     25.000   20.695  -67.265  1.00 13.07        O
ATOM  10424  CB   SER G  67     23.632   22.763  -69.823  1.00  9.69        C
ATOM  10425  OG   SER G  67     23.257   23.463  -68.698  1.00  8.44        O
ATOM  10426  N    GLY G  68     26.030   22.237  -68.498  1.00 12.85        N
ATOM  10427  CA   GLY G  68     27.006   22.524  -67.455  1.00 13.93        C
ATOM  10428  C    GLY G  68     26.460   23.407  -66.323  1.00 15.35        C
ATOM  10429  O    GLY G  68     27.029   23.416  -65.229  1.00 15.55        O
ATOM  10430  N    THR G  69     25.390   24.184  -66.582  1.00 16.18        N
ATOM  10431  CA   THR G  69     24.890   25.177  -65.627  1.00 15.14        C
ATOM  10432  C    THR G  69     23.424   25.052  -65.184  1.00 14.93        C
ATOM  10433  O    THR G  69     23.019   25.691  -64.215  1.00 14.23        O
ATOM  10434  CB   THR G  69     25.097   26.619  -66.190  1.00 16.19        C
ATOM  10435  OG1  THR G  69     24.319   26.754  -67.363  1.00 17.50        O
ATOM  10436  CG2  THR G  69     26.561   26.860  -66.505  1.00 15.70        C
ATOM  10437  N    ASP G  70     22.619   24.260  -65.887  1.00 12.90        N
ATOM  10438  CA   ASP G  70     21.188   24.113  -65.578  1.00 13.89        C
ATOM  10439  C    ASP G  70     20.826   22.620  -65.602  1.00 13.25        C
ATOM  10440  O    ASP G  70     21.095   21.966  -66.576  1.00 13.52        O
ATOM  10441  CB   ASP G  70     20.337   24.855  -66.610  1.00 14.45        C
ATOM  10442  CG   ASP G  70     20.567   26.386  -66.584  1.00 16.32        C
ATOM  10443  OD1  ASP G  70     20.068   27.058  -65.654  1.00 19.42        O
ATOM  10444  OD2  ASP G  70     21.282   26.906  -67.474  1.00 16.75        O
ATOM  10445  N    PHE G  71     20.148   22.146  -64.565  1.00 12.91        N
ATOM  10446  CA   PHE G  71     19.937   20.696  -64.342  1.00 12.86        C
ATOM  10447  C    PHE G  71     18.504   20.394  -63.930  1.00 13.78        C
ATOM  10448  O    PHE G  71     17.836   21.231  -63.348  1.00 12.99        O
ATOM  10449  CB   PHE G  71     20.891   20.189  -63.246  1.00 11.55        C
ATOM  10450  CG   PHE G  71     22.221   20.877  -63.231  1.00 12.78        C
ATOM  10451  CD1  PHE G  71     23.143   20.680  -64.253  1.00 11.79        C
ATOM  10452  CD2  PHE G  71     22.532   21.786  -62.215  1.00 10.60        C
ATOM  10453  CE1  PHE G  71     24.358   21.352  -64.239  1.00 10.64        C
```

```
ATOM   10454  CE2 PHE G  71    23.767  22.416 -62.207  1.00 11.06           C
ATOM   10455  CZ  PHE G  71    24.662  22.205 -63.193  1.00  8.90           C
ATOM   10456  N   THR G  72    18.032  19.167 -64.214  1.00 13.60           N
ATOM   10457  CA  THR G  72    16.673  18.808 -63.860  1.00 14.74           C
ATOM   10458  C   THR G  72    16.628  17.361 -63.403  1.00 14.61           C
ATOM   10459  O   THR G  72    17.053  16.520 -64.150  1.00 13.62           O
ATOM   10460  CB  THR G  72    15.688  18.979 -65.052  1.00 13.21           C
ATOM   10461  OG1 THR G  72    15.746  20.319 -65.569  1.00 15.94           O
ATOM   10462  CG2 THR G  72    14.316  18.696 -64.612  1.00 15.34           C
ATOM   10463  N   LEU G  73    16.142  17.129 -62.177  1.00 14.35           N
ATOM   10464  CA  LEU G  73    15.886  15.799 -61.642  1.00 17.38           C
ATOM   10465  C   LEU G  73    14.422  15.488 -61.923  1.00 17.81           C
ATOM   10466  O   LEU G  73    13.529  16.302 -61.639  1.00 19.54           O
ATOM   10467  CB  LEU G  73    16.159  15.751 -60.126  1.00 16.59           C
ATOM   10468  CG  LEU G  73    15.767  14.428 -59.421  1.00 17.24           C
ATOM   10469  CD1 LEU G  73    16.705  13.343 -59.760  1.00 19.17           C
ATOM   10470  CD2 LEU G  73    15.646  14.589 -57.889  1.00 18.62           C
ATOM   10471  N   ARG G  74    14.182  14.334 -62.524  1.00 20.54           N
ATOM   10472  CA  ARG G  74    12.830  13.895 -62.762  1.00 21.70           C
ATOM   10473  C   ARG G  74    12.574  12.601 -62.014  1.00 20.95           C
ATOM   10474  O   ARG G  74    13.435  11.691 -61.998  1.00 19.46           O
ATOM   10475  CB  ARG G  74    12.520  13.785 -64.262  1.00 22.71           C
ATOM   10476  CG  ARG G  74    10.996  13.525 -64.527  1.00 23.96           C
ATOM   10477  CD  ARG G  74    10.532  13.775 -65.951  1.00 26.00           C
ATOM   10478  NE  ARG G  74     9.199  13.207 -66.204  1.00 28.14           N
ATOM   10479  CZ  ARG G  74     8.982  11.903 -66.434  1.00 29.79           C
ATOM   10480  NH1 ARG G  74     9.995  11.039 -66.435  1.00 31.56           N
ATOM   10481  NH2 ARG G  74     7.748  11.455 -66.662  1.00 29.84           N
ATOM   10482  N   ILE G  75    11.405  12.567 -61.356  1.00 23.60           N
ATOM   10483  CA  ILE G  75    10.846  11.354 -60.748  1.00 25.29           C
ATOM   10484  C   ILE G  75     9.578  11.010 -61.508  1.00 25.69           C
ATOM   10485  O   ILE G  75     8.584  11.721 -61.390  1.00 26.52           O
ATOM   10486  CB  ILE G  75    10.472  11.527 -59.256  1.00 24.95           C
ATOM   10487  CG1 ILE G  75    11.614  12.208 -58.506  1.00 27.05           C
ATOM   10488  CG2 ILE G  75    10.148  10.153 -58.658  1.00 25.14           C
ATOM   10489  CD1 ILE G  75    11.361  12.482 -57.008  1.00 27.07           C
ATOM   10490  N   SER G  76     9.616   9.926 -62.278  1.00 26.52           N
ATOM   10491  CA  SER G  76     8.543   9.595 -63.224  1.00 28.61           C
ATOM   10492  C   SER G  76     7.197   9.350 -62.554  1.00 30.92           C
ATOM   10493  O   SER G  76     6.178   9.963 -62.943  1.00 33.91           O
ATOM   10494  CB  SER G  76     8.914   8.373 -64.064  1.00 28.26           C
ATOM   10495  OG  SER G  76     9.091   7.240 -63.244  1.00 28.51           O
ATOM   10496  N   ARG G  77     7.192   8.460 -61.563  1.00 30.93           N
ATOM   10497  CA  ARG G  77     5.972   8.113 -60.840  1.00 32.29           C
ATOM   10498  C   ARG G  77     6.279   8.072 -59.358  1.00 31.80           C
ATOM   10499  O   ARG G  77     6.815   7.082 -58.870  1.00 32.44           O
ATOM   10500  CB  ARG G  77     5.457   6.743 -61.278  1.00 32.36           C
ATOM   10501  CG  ARG G  77     4.868   6.774 -62.635  1.00 35.63           C
ATOM   10502  CD  ARG G  77     4.334   5.444 -63.052  1.00 37.32           C
ATOM   10503  NE  ARG G  77     3.559   5.622 -64.276  1.00 41.31           N
ATOM   10504  CZ  ARG G  77     2.867   4.667 -64.893  1.00 42.34           C
ATOM   10505  NH1 ARG G  77     2.831   3.421 -64.406  1.00 43.29           N
ATOM   10506  NH2 ARG G  77     2.202   4.974 -66.011  1.00 43.09           N
ATOM   10507  N   VAL G  78     5.912   9.133 -58.640  1.00 31.41           N
ATOM   10508  CA  VAL G  78     6.330   9.258 -57.249  1.00 31.09           C
ATOM   10509  C   VAL G  78     5.663   8.209 -56.330  1.00 31.03           C
ATOM   10510  O   VAL G  78     4.443   7.947 -56.421  1.00 29.80           O
ATOM   10511  CB  VAL G  78     6.081  10.670 -56.700  1.00 31.55           C
ATOM   10512  CG1 VAL G  78     6.572  10.750 -55.274  1.00 33.34           C
ATOM   10513  CG2 VAL G  78     6.825  11.680 -57.515  1.00 31.02           C
ATOM   10514  N   GLU G  79     6.501   7.624 -55.470  1.00 30.45           N
ATOM   10515  CA  GLU G  79     6.112   6.697 -54.410  1.00 29.86           C
ATOM   10516  C   GLU G  79     6.315   7.377 -53.066  1.00 28.21           C
ATOM   10517  O   GLU G  79     7.166   8.261 -52.941  1.00 26.29           O
ATOM   10518  CB  GLU G  79     6.987   5.460 -54.466  1.00 30.63           C
```

| ATOM | 10519 | CG | GLU | G | 79 | 6.756 | 4.639 | -55.718 | 1.00 | 33.30 | C |
|------|-------|-----|-----|---|----|-------|-------|---------|------|-------|---|
| ATOM | 10520 | CD | GLU | G | 79 | 7.376 | 3.239 | -55.659 | 1.00 | 34.59 | C |
| ATOM | 10521 | OE1 | GLU | G | 79 | 8.118 | 2.898 | -54.692 | 1.00 | 38.26 | O |
| ATOM | 10522 | OE2 | GLU | G | 79 | 7.118 | 2.475 | -56.613 | 1.00 | 40.33 | O |
| ATOM | 10523 | N | ALA | G | 80 | 5.556 | 6.943 | -52.062 | 1.00 | 26.72 | N |
| ATOM | 10524 | CA | ALA | G | 80 | 5.652 | 7.491 | -50.699 | 1.00 | 25.84 | C |
| ATOM | 10525 | C | ALA | G | 80 | 7.074 | 7.625 | -50.150 | 1.00 | 23.57 | C |
| ATOM | 10526 | O | ALA | G | 80 | 7.408 | 8.613 | -49.541 | 1.00 | 22.46 | O |
| ATOM | 10527 | CB | ALA | G | 80 | 4.813 | 6.666 | -49.744 | 1.00 | 25.83 | C |
| ATOM | 10528 | N | GLU | G | 81 | 7.900 | 6.619 | -50.391 | 1.00 | 23.41 | N |
| ATOM | 10529 | CA | GLU | G | 81 | 9.265 | 6.585 | -49.879 | 1.00 | 24.26 | C |
| ATOM | 10530 | C | GLU | G | 81 | 10.194 | 7.670 | -50.490 | 1.00 | 23.11 | C |
| ATOM | 10531 | O | GLU | G | 81 | 11.307 | 7.844 | -50.021 | 1.00 | 22.64 | O |
| ATOM | 10532 | CB | GLU | G | 81 | 9.881 | 5.173 | -50.027 | 1.00 | 26.43 | C |
| ATOM | 10533 | CG | GLU | G | 81 | 9.058 | 4.164 | -50.869 | 1.00 | 31.46 | C |
| ATOM | 10534 | CD | GLU | G | 81 | 7.671 | 3.821 | -50.251 | 1.00 | 33.48 | C |
| ATOM | 10535 | OE1 | GLU | G | 81 | 7.571 | 3.689 | -48.992 | 1.00 | 37.41 | O |
| ATOM | 10536 | OE2 | GLU | G | 81 | 6.692 | 3.678 | -51.028 | 1.00 | 33.87 | O |
| ATOM | 10537 | N | ASP | G | 82 | 9.706 | 8.408 | -51.489 | 1.00 | 21.96 | N |
| ATOM | 10538 | CA | ASP | G | 82 | 10.484 | 9.469 | -52.156 | 1.00 | 21.29 | C |
| ATOM | 10539 | C | ASP | G | 82 | 10.423 | 10.841 | -51.469 | 1.00 | 20.39 | C |
| ATOM | 10540 | O | ASP | G | 82 | 11.123 | 11.762 | -51.878 | 1.00 | 20.46 | O |
| ATOM | 10541 | CB | ASP | G | 82 | 10.010 | 9.634 | -53.602 | 1.00 | 22.54 | C |
| ATOM | 10542 | CG | ASP | G | 82 | 10.202 | 8.369 | -54.426 | 1.00 | 23.80 | C |
| ATOM | 10543 | OD1 | ASP | G | 82 | 11.030 | 7.520 | -54.035 | 1.00 | 24.48 | O |
| ATOM | 10544 | OD2 | ASP | G | 82 | 9.513 | 8.240 | -55.456 | 1.00 | 26.89 | O |
| ATOM | 10545 | N | VAL | G | 83 | 9.614 | 10.976 | -50.434 | 1.00 | 18.55 | N |
| ATOM | 10546 | CA | VAL | G | 83 | 9.550 | 12.247 | -49.727 | 1.00 | 18.75 | C |
| ATOM | 10547 | C | VAL | G | 83 | 10.879 | 12.521 | -49.027 | 1.00 | 16.78 | C |
| ATOM | 10548 | O | VAL | G | 83 | 11.582 | 11.620 | -48.547 | 1.00 | 15.28 | O |
| ATOM | 10549 | CB | VAL | G | 83 | 8.347 | 12.342 | -48.741 | 1.00 | 20.05 | C |
| ATOM | 10550 | CG1 | VAL | G | 83 | 7.044 | 12.200 | -49.500 | 1.00 | 21.36 | C |
| ATOM | 10551 | CG2 | VAL | G | 83 | 8.461 | 11.312 | -47.628 | 1.00 | 21.06 | C |
| ATOM | 10552 | N | GLY | G | 84 | 11.244 | 13.793 | -48.994 | 1.00 | 16.60 | N |
| ATOM | 10553 | CA | GLY | G | 84 | 12.487 | 14.178 | -48.392 | 1.00 | 15.88 | C |
| ATOM | 10554 | C | GLY | G | 84 | 12.941 | 15.469 | -49.028 | 1.00 | 14.77 | C |
| ATOM | 10555 | O | GLY | G | 84 | 12.208 | 16.082 | -49.787 | 1.00 | 14.39 | O |
| ATOM | 10556 | N | ILE | G | 85 | 14.173 | 15.838 | -48.727 | 1.00 | 15.30 | N |
| ATOM | 10557 | CA | ILE | G | 85 | 14.808 | 17.005 | -49.301 | 1.00 | 14.70 | C |
| ATOM | 10558 | C | ILE | G | 85 | 15.910 | 16.547 | -50.266 | 1.00 | 13.86 | C |
| ATOM | 10559 | O | ILE | G | 85 | 16.812 | 15.770 | -49.900 | 1.00 | 15.53 | O |
| ATOM | 10560 | CB | ILE | G | 85 | 15.394 | 17.928 | -48.203 | 1.00 | 15.45 | C |
| ATOM | 10561 | CG1 | ILE | G | 85 | 14.284 | 18.318 | -47.228 | 1.00 | 18.14 | C |
| ATOM | 10562 | CG2 | ILE | G | 85 | 15.994 | 19.188 | -48.834 | 1.00 | 15.77 | C |
| ATOM | 10563 | CD1 | ILE | G | 85 | 14.711 | 19.272 | -46.151 | 1.00 | 19.59 | C |
| ATOM | 10564 | N | TYR | G | 86 | 15.787 | 17.020 | -51.501 | 1.00 | 12.57 | N |
| ATOM | 10565 | CA | TYR | G | 86 | 16.718 | 16.767 | -52.599 | 1.00 | 12.77 | C |
| ATOM | 10566 | C | TYR | G | 86 | 17.721 | 17.931 | -52.742 | 1.00 | 13.33 | C |
| ATOM | 10567 | O | TYR | G | 86 | 17.318 | 19.071 | -52.888 | 1.00 | 13.91 | O |
| ATOM | 10568 | CB | TYR | G | 86 | 15.913 | 16.537 | -53.898 | 1.00 | 13.26 | C |
| ATOM | 10569 | CG | TYR | G | 86 | 15.107 | 15.238 | -53.907 | 1.00 | 12.32 | C |
| ATOM | 10570 | CD1 | TYR | G | 86 | 13.830 | 15.175 | -53.324 | 1.00 | 13.33 | C |
| ATOM | 10571 | CD2 | TYR | G | 86 | 15.625 | 14.084 | -54.468 | 1.00 | 13.05 | C |
| ATOM | 10572 | CE1 | TYR | G | 86 | 13.077 | 13.992 | -53.325 | 1.00 | 12.53 | C |
| ATOM | 10573 | CE2 | TYR | G | 86 | 14.882 | 12.881 | -54.447 | 1.00 | 13.75 | C |
| ATOM | 10574 | CZ | TYR | G | 86 | 13.612 | 12.857 | -53.870 | 1.00 | 14.41 | C |
| ATOM | 10575 | OH | TYR | G | 86 | 12.866 | 11.690 | -53.843 | 1.00 | 14.21 | O |
| ATOM | 10576 | N | TYR | G | 87 | 19.003 | 17.625 | -52.618 | 1.00 | 12.42 | N |
| ATOM | 10577 | CA | TYR | G | 87 | 20.091 | 18.580 | -52.799 | 1.00 | 12.39 | C |
| ATOM | 10578 | C | TYR | G | 87 | 20.959 | 18.310 | -54.049 | 1.00 | 12.37 | C |
| ATOM | 10579 | O | TYR | G | 87 | 21.352 | 17.172 | -54.343 | 1.00 | 11.05 | O |
| ATOM | 10580 | CB | TYR | G | 87 | 21.047 | 18.583 | -51.607 | 1.00 | 12.03 | C |
| ATOM | 10581 | CG | TYR | G | 87 | 20.437 | 18.817 | -50.253 | 1.00 | 11.13 | C |
| ATOM | 10582 | CD1 | TYR | G | 87 | 20.349 | 20.084 | -49.718 | 1.00 | 10.72 | C |
| ATOM | 10583 | CD2 | TYR | G | 87 | 19.988 | 17.761 | -49.497 | 1.00 | 11.05 | C |

```
ATOM   10584  CE1 TYR G   87      19.834  20.298 -48.500  1.00 11.40           C
ATOM   10585  CE2 TYR G   87      19.485  17.950 -48.235  1.00 11.72           C
ATOM   10586  CZ  TYR G   87      19.387  19.256 -47.740  1.00 13.12           C
ATOM   10587  OH  TYR G   87      18.874  19.502 -46.486  1.00 15.89           O
ATOM   10588  N   CYS G   88      21.262  19.373 -54.771  1.00 11.58           N
ATOM   10589  CA  CYS G   88      22.211  19.335 -55.866  1.00 12.25           C
ATOM   10590  C   CYS G   88      23.560  19.747 -55.256  1.00 13.14           C
ATOM   10591  O   CYS G   88      23.614  20.440 -54.236  1.00 13.14           O
ATOM   10592  CB  CYS G   88      21.763  20.246 -57.031  1.00 13.92           C
ATOM   10593  SG  CYS G   88      21.432  21.994 -56.604  1.00 19.00           S
ATOM   10594  N   ALA G   89      24.645  19.249 -55.849  1.00 13.73           N
ATOM   10595  CA  ALA G   89      25.998  19.574 -55.455  1.00 12.80           C
ATOM   10596  C   ALA G   89      26.936  19.403 -56.635  1.00 11.71           C
ATOM   10597  O   ALA G   89      26.623  18.706 -57.564  1.00 11.50           O
ATOM   10598  CB  ALA G   89      26.432  18.667 -54.319  1.00 13.65           C
ATOM   10599  N   HIS G   90      28.080  20.061 -56.582  1.00 11.80           N
ATOM   10600  CA  HIS G   90      29.086  19.951 -57.627  1.00 11.79           C
ATOM   10601  C   HIS G   90      30.295  19.193 -57.109  1.00 11.96           C
ATOM   10602  O   HIS G   90      30.492  19.033 -55.903  1.00  9.78           O
ATOM   10603  CB  HIS G   90      29.482  21.352 -58.107  1.00 12.16           C
ATOM   10604  CG  HIS G   90      30.476  22.069 -57.227  1.00 10.94           C
ATOM   10605  ND1 HIS G   90      30.120  23.091 -56.363  1.00 13.25           N
ATOM   10606  CD2 HIS G   90      31.818  21.922 -57.103  1.00 10.93           C
ATOM   10607  CE1 HIS G   90      31.205  23.516 -55.741  1.00 10.96           C
ATOM   10608  NE2 HIS G   90      32.248  22.840 -56.192  1.00 10.82           N
ATOM   10609  N   ASN G   91      31.131  18.803 -58.047  1.00 12.38           N
ATOM   10610  CA  ASN G   91      32.412  18.214 -57.758  1.00 13.19           C
ATOM   10611  C   ASN G   91      33.430  18.624 -58.816  1.00 13.18           C
ATOM   10612  O   ASN G   91      34.040  17.791 -59.469  1.00 16.77           O
ATOM   10613  CB  ASN G   91      32.223  16.687 -57.731  1.00 11.91           C
ATOM   10614  CG  ASN G   91      33.514  15.926 -57.491  1.00 14.15           C
ATOM   10615  OD1 ASN G   91      33.631  14.760 -57.887  1.00 17.73           O
ATOM   10616  ND2 ASN G   91      34.465  16.557 -56.864  1.00 10.56           N
ATOM   10617  N   VAL G   92      33.618  19.934 -58.983  1.00 14.01           N
ATOM   10618  CA  VAL G   92      34.582  20.452 -59.926  1.00 15.09           C
ATOM   10619  C   VAL G   92      35.714  21.230 -59.230  1.00 15.33           C
ATOM   10620  O   VAL G   92      36.746  21.458 -59.822  1.00 16.21           O
ATOM   10621  CB  VAL G   92      33.915  21.319 -61.020  1.00 15.87           C
ATOM   10622  CG1 VAL G   92      32.878  20.519 -61.739  1.00 16.11           C
ATOM   10623  CG2 VAL G   92      33.329  22.535 -60.455  1.00 17.44           C
ATOM   10624  N   GLU G   93      35.513  21.615 -57.973  1.00 16.17           N
ATOM   10625  CA  GLU G   93      36.499  22.388 -57.212  1.00 16.33           C
ATOM   10626  C   GLU G   93      36.164  22.357 -55.730  1.00 15.45           C
ATOM   10627  O   GLU G   93      35.120  21.852 -55.326  1.00 16.83           O
ATOM   10628  CB  GLU G   93      36.506  23.855 -57.694  1.00 17.60           C
ATOM   10629  CG  GLU G   93      35.191  24.549 -57.461  1.00 13.89           C
ATOM   10630  CD  GLU G   93      35.233  26.069 -57.747  1.00 18.09           C
ATOM   10631  OE1 GLU G   93      35.734  26.473 -58.813  1.00 21.90           O
ATOM   10632  OE2 GLU G   93      34.739  26.857 -56.920  1.00 15.28           O
ATOM   10633  N   LEU G   94      37.067  22.868 -54.916  1.00 15.69           N
ATOM   10634  CA  LEU G   94      36.757  23.197 -53.535  1.00 15.67           C
ATOM   10635  C   LEU G   94      36.605  24.718 -53.520  1.00 16.13           C
ATOM   10636  O   LEU G   94      37.199  25.388 -54.349  1.00 20.72           O
ATOM   10637  CB  LEU G   94      37.900  22.824 -52.600  1.00 15.87           C
ATOM   10638  CG  LEU G   94      38.368  21.380 -52.731  1.00 14.34           C
ATOM   10639  CD1 LEU G   94      39.590  21.175 -51.818  1.00 16.99           C
ATOM   10640  CD2 LEU G   94      37.207  20.498 -52.338  1.00 16.02           C
ATOM   10641  N   PRO G   95      35.813  25.250 -52.591  1.00 16.05           N
ATOM   10642  CA  PRO G   95      35.013  24.515 -51.625  1.00 16.56           C
ATOM   10643  C   PRO G   95      33.901  23.696 -52.274  1.00 15.21           C
ATOM   10644  O   PRO G   95      33.363  24.076 -53.325  1.00 15.06           O
ATOM   10645  CB  PRO G   95      34.408  25.605 -50.773  1.00 17.04           C
ATOM   10646  CG  PRO G   95      34.401  26.809 -51.685  1.00 17.35           C
ATOM   10647  CD  PRO G   95      35.606  26.711 -52.476  1.00 16.80           C
ATOM   10648  N   ARG G   96      33.548  22.585 -51.639  1.00 15.22           N
```

```
ATOM   10649  CA   ARG G  96      32.407  21.831 -52.076  1.00 13.86          C
ATOM   10650  C    ARG G  96      31.143  22.575 -51.610  1.00 13.56          C
ATOM   10651  O    ARG G  96      30.993  22.899 -50.435  1.00 13.99          O
ATOM   10652  CB   ARG G  96      32.437  20.408 -51.507  1.00 14.85          C
ATOM   10653  CG   ARG G  96      33.799  19.697 -51.605  1.00 13.74          C
ATOM   10654  CD   ARG G  96      33.655  18.196 -51.447  1.00 14.70          C
ATOM   10655  NE   ARG G  96      34.905  17.515 -51.747  1.00 15.95          N
ATOM   10656  CZ   ARG G  96      35.314  17.171 -52.968  1.00 16.16          C
ATOM   10657  NH1  ARG G  96      34.561  17.405 -54.038  1.00 15.09          N
ATOM   10658  NH2  ARG G  96      36.465  16.555 -53.122  1.00 15.77          N
ATOM   10659  N    THR G  97      30.219  22.814 -52.524  1.00 12.19          N
ATOM   10660  CA   THR G  97      28.983  23.451 -52.191  1.00 13.92          C
ATOM   10661  C    THR G  97      27.767  22.676 -52.617  1.00 12.69          C
ATOM   10662  O    THR G  97      27.829  21.859 -53.510  1.00 14.96          O
ATOM   10663  CB   THR G  97      28.968  24.912 -52.711  1.00 13.17          C
ATOM   10664  OG1  THR G  97      29.173  24.956 -54.133  1.00 10.58          O
ATOM   10665  CG2  THR G  97      30.068  25.680 -52.029  1.00 17.39          C
ATOM   10666  N    PHE G  98      26.677  22.918 -51.895  1.00 12.59          N
ATOM   10667  CA   PHE G  98      25.387  22.294 -52.063  1.00 13.35          C
ATOM   10668  C    PHE G  98      24.325  23.354 -52.303  1.00 14.73          C
ATOM   10669  O    PHE G  98      24.467  24.535 -51.831  1.00 15.06          O
ATOM   10670  CB   PHE G  98      25.008  21.515 -50.804  1.00 13.72          C
ATOM   10671  CG   PHE G  98      25.989  20.468 -50.418  1.00 10.80          C
ATOM   10672  CD1  PHE G  98      27.089  20.795 -49.649  1.00 11.84          C
ATOM   10673  CD2  PHE G  98      25.798  19.152 -50.786  1.00  9.91          C
ATOM   10674  CE1  PHE G  98      27.973  19.879 -49.255  1.00  8.58          C
ATOM   10675  CE2  PHE G  98      26.692  18.207 -50.406  1.00  8.65          C
ATOM   10676  CZ   PHE G  98      27.771  18.525 -49.652  1.00 11.40          C
ATOM   10677  N    GLY G  99      23.281  22.970 -53.053  1.00 14.51          N
ATOM   10678  CA   GLY G  99      22.046  23.766 -53.108  1.00 14.30          C
ATOM   10679  C    GLY G  99      21.368  23.816 -51.741  1.00 12.83          C
ATOM   10680  O    GLY G  99      21.734  23.075 -50.807  1.00 12.48          O
ATOM   10681  N    GLY G 100      20.381  24.707 -51.591  1.00 13.00          N
ATOM   10682  CA   GLY G 100      19.641  24.812 -50.340  1.00 12.80          C
ATOM   10683  C    GLY G 100      18.649  23.701 -50.048  1.00 14.24          C
ATOM   10684  O    GLY G 100      18.029  23.662 -48.979  1.00 15.63          O
ATOM   10685  N    GLY G 101      18.458  22.801 -51.002  1.00 14.33          N
ATOM   10686  CA   GLY G 101      17.555  21.660 -50.830  1.00 14.55          C
ATOM   10687  C    GLY G 101      16.193  22.038 -51.368  1.00 14.20          C
ATOM   10688  O    GLY G 101      15.733  23.206 -51.222  1.00 12.52          O
ATOM   10689  N    THR G 102      15.546  21.091 -52.024  1.00 12.46          N
ATOM   10690  CA   THR G 102      14.152  21.255 -52.451  1.00 14.73          C
ATOM   10691  C    THR G 102      13.351  20.128 -51.799  1.00 15.33          C
ATOM   10692  O    THR G 102      13.667  18.979 -52.024  1.00 14.93          O
ATOM   10693  CB   THR G 102      13.995  21.144 -53.997  1.00 14.61          C
ATOM   10694  OG1  THR G 102      14.724  22.184 -54.696  1.00 13.44          O
ATOM   10695  CG2  THR G 102      12.520  21.185 -54.395  1.00 15.06          C
ATOM   10696  N    LYS G 103      12.351  20.460 -50.980  1.00 18.18          N
ATOM   10697  CA   LYS G 103      11.520  19.450 -50.335  1.00 20.25          C
ATOM   10698  C    LYS G 103      10.464  18.891 -51.294  1.00 20.52          C
ATOM   10699  O    LYS G 103       9.731  19.659 -51.915  1.00 22.71          O
ATOM   10700  CB   LYS G 103      10.834  20.054 -49.117  1.00 21.55          C
ATOM   10701  CG   LYS G 103       9.969  19.070 -48.344  1.00 22.15          C
ATOM   10702  CD   LYS G 103       8.806  19.749 -47.618  1.00 24.78          C
ATOM   10703  CE   LYS G 103       9.254  20.348 -46.297  1.00 27.78          C
ATOM   10704  NZ   LYS G 103       8.099  20.746 -45.406  1.00 28.58          N
ATOM   10705  N    LEU G 104      10.405  17.562 -51.428  1.00 21.71          N
ATOM   10706  CA   LEU G 104       9.326  16.908 -52.150  1.00 22.56          C
ATOM   10707  C    LEU G 104       8.214  16.532 -51.179  1.00 23.14          C
ATOM   10708  O    LEU G 104       8.456  15.939 -50.141  1.00 22.33          O
ATOM   10709  CB   LEU G 104       9.784  15.608 -52.807  1.00 22.63          C
ATOM   10710  CG   LEU G 104       8.715  14.971 -53.671  1.00 22.83          C
ATOM   10711  CD1  LEU G 104       8.634  15.707 -55.001  1.00 26.41          C
ATOM   10712  CD2  LEU G 104       9.042  13.513 -53.897  1.00 22.97          C
ATOM   10713  N    GLU G 105       6.995  16.855 -51.569  1.00 26.09          N
```

```
ATOM  10714  CA  GLU G 105      5.833  16.552 -50.784  1.00 27.86           C
ATOM  10715  C   GLU G 105      4.826  15.889 -51.720  1.00 27.89           C
ATOM  10716  O   GLU G 105      4.769  16.198 -52.911  1.00 27.45           O
ATOM  10717  CB  GLU G 105      5.288  17.841 -50.199  1.00 30.09           C
ATOM  10718  CG  GLU G 105      4.247  17.605 -49.157  1.00 34.01           C
ATOM  10719  CD  GLU G 105      3.664  18.908 -48.642  1.00 34.46           C
ATOM  10720  OE1 GLU G 105      4.276  19.491 -47.703  1.00 41.38           O
ATOM  10721  OE2 GLU G 105      2.600  19.331 -49.173  1.00 39.73           O
ATOM  10722  N   ILE G 106      4.073  14.946 -51.173  1.00 27.51           N
ATOM  10723  CA  ILE G 106      3.270  14.028 -51.961  1.00 27.57           C
ATOM  10724  C   ILE G 106      1.823  14.270 -51.601  1.00 27.00           C
ATOM  10725  O   ILE G 106      1.508  14.577 -50.445  1.00 26.63           O
ATOM  10726  CB  ILE G 106      3.713  12.562 -51.687  1.00 28.47           C
ATOM  10727  CG1 ILE G 106      5.050  12.284 -52.401  1.00 29.27           C
ATOM  10728  CG2 ILE G 106      2.649  11.530 -52.135  1.00 28.44           C
ATOM  10729  CD1 ILE G 106      5.526  10.842 -52.246  1.00 30.33           C
ATOM  10730  N   LYS G 107      0.951  14.184 -52.604  1.00 25.92           N
ATOM  10731  CA  LYS G 107     -0.490  14.264 -52.408  1.00 26.01           C
ATOM  10732  C   LYS G 107     -1.036  12.836 -52.448  1.00 24.73           C
ATOM  10733  O   LYS G 107     -0.760  12.071 -53.376  1.00 24.96           O
ATOM  10734  CB  LYS G 107     -1.147  15.141 -53.484  1.00 26.66           C
ATOM  10735  CG  LYS G 107     -2.676  15.275 -53.398  1.00 27.59           C
ATOM  10736  CD  LYS G 107     -3.263  15.825 -54.711  1.00 28.78           C
ATOM  10737  CE  LYS G 107     -4.682  15.252 -55.068  1.00 30.10           C
ATOM  10738  NZ  LYS G 107     -4.734  13.846 -55.666  1.00 31.74           N
ATOM  10739  N   ARG G 108     -1.784  12.491 -51.413  1.00 23.53           N
ATOM  10740  CA  ARG G 108     -2.485  11.216 -51.342  1.00 23.74           C
ATOM  10741  C   ARG G 108     -3.944  11.493 -51.090  1.00 22.19           C
ATOM  10742  O   ARG G 108     -4.345  12.663 -51.022  1.00 20.59           O
ATOM  10743  CB  ARG G 108     -1.920  10.373 -50.215  1.00 24.24           C
ATOM  10744  CG  ARG G 108     -1.837  11.123 -48.895  1.00 24.43           C
ATOM  10745  CD  ARG G 108     -1.894  10.154 -47.743  1.00 24.37           C
ATOM  10746  NE  ARG G 108     -3.236   9.620 -47.556  1.00 23.88           N
ATOM  10747  CZ  ARG G 108     -3.502   8.422 -47.036  1.00 24.58           C
ATOM  10748  NH1 ARG G 108     -2.530   7.615 -46.644  1.00 25.41           N
ATOM  10749  NH2 ARG G 108     -4.753   8.030 -46.910  1.00 25.64           N
ATOM  10750  N   ALA G 109     -4.738  10.433 -50.935  1.00 19.06           N
ATOM  10751  CA  ALA G 109     -6.168  10.602 -50.613  1.00 20.66           C
ATOM  10752  C   ALA G 109     -6.360  11.318 -49.254  1.00 20.22           C
ATOM  10753  O   ALA G 109     -5.603  11.121 -48.303  1.00 17.79           O
ATOM  10754  CB  ALA G 109     -6.905   9.241 -50.629  1.00 20.83           C
ATOM  10755  N   ASP G 110     -7.350  12.179 -49.164  1.00 20.67           N
ATOM  10756  CA  ASP G 110     -7.619  12.827 -47.876  1.00 21.04           C
ATOM  10757  C   ASP G 110     -7.886  11.784 -46.794  1.00 21.63           C
ATOM  10758  O   ASP G 110     -8.491  10.750 -47.066  1.00 22.06           O
ATOM  10759  CB  ASP G 110     -8.785  13.801 -47.960  1.00 21.37           C
ATOM  10760  CG  ASP G 110     -8.509  14.976 -48.881  1.00 22.02           C
ATOM  10761  OD1 ASP G 110     -7.340  15.298 -49.135  1.00 21.06           O
ATOM  10762  OD2 ASP G 110     -9.496  15.545 -49.376  1.00 21.21           O
ATOM  10763  N   ALA G 111     -7.404  12.047 -45.572  1.00 21.01           N
ATOM  10764  CA  ALA G 111     -7.512  11.108 -44.465  1.00 20.17           C
ATOM  10765  C   ALA G 111     -7.705  11.893 -43.182  1.00 20.14           C
ATOM  10766  O   ALA G 111     -6.949  12.819 -42.892  1.00 18.22           O
ATOM  10767  CB  ALA G 111     -6.245  10.226 -44.360  1.00 20.81           C
ATOM  10768  N   ALA G 112     -8.712  11.500 -42.413  1.00 19.15           N
ATOM  10769  CA  ALA G 112     -9.075  12.177 -41.174  1.00 18.85           C
ATOM  10770  C   ALA G 112     -8.093  11.819 -40.067  1.00 18.50           C
ATOM  10771  O   ALA G 112     -7.508  10.729 -40.068  1.00 18.18           O
ATOM  10772  CB  ALA G 112    -10.495  11.770 -40.775  1.00 18.49           C
ATOM  10773  N   PRO G 113     -7.864  12.736 -39.116  1.00 18.98           N
ATOM  10774  CA  PRO G 113     -7.009  12.353 -37.981  1.00 19.01           C
ATOM  10775  C   PRO G 113     -7.692  11.439 -36.971  1.00 18.56           C
ATOM  10776  O   PRO G 113     -8.920  11.440 -36.844  1.00 18.28           O
ATOM  10777  CB  PRO G 113     -6.683  13.692 -37.316  1.00 19.89           C
ATOM  10778  CG  PRO G 113     -7.881  14.508 -37.622  1.00 19.34           C
```

```
ATOM  10779  CD   PRO G 113   -8.339  14.118 -38.990  1.00 20.31      C
ATOM  10780  N    THR G 114   -6.867  10.644 -36.300  1.00 17.34      N
ATOM  10781  CA   THR G 114   -7.204   9.958 -35.079  1.00 17.24      C
ATOM  10782  C    THR G 114   -6.775  10.902 -33.933  1.00 17.22      C
ATOM  10783  O    THR G 114   -5.616  11.332 -33.853  1.00 17.02      O
ATOM  10784  CB   THR G 114   -6.451   8.616 -35.040  1.00 17.95      C
ATOM  10785  OG1  THR G 114   -6.740   7.910 -36.243  1.00 18.25      O
ATOM  10786  CG2  THR G 114   -6.853   7.757 -33.852  1.00 17.73      C
ATOM  10787  N    VAL G 115   -7.722  11.253 -33.079  1.00 17.40      N
ATOM  10788  CA   VAL G 115   -7.478  12.195 -32.011  1.00 18.11      C
ATOM  10789  C    VAL G 115   -7.492  11.522 -30.660  1.00 18.89      C
ATOM  10790  O    VAL G 115   -8.410  10.747 -30.350  1.00 19.29      O
ATOM  10791  CB   VAL G 115   -8.465  13.345 -32.065  1.00 19.16      C
ATOM  10792  CG1  VAL G 115   -8.047  14.465 -31.089  1.00 20.12      C
ATOM  10793  CG2  VAL G 115   -8.524  13.875 -33.497  1.00 19.06      C
ATOM  10794  N    SER G 116   -6.437  11.795 -29.885  1.00 17.49      N
ATOM  10795  CA   SER G 116   -6.308  11.299 -28.542  1.00 17.64      C
ATOM  10796  C    SER G 116   -5.978  12.431 -27.614  1.00 16.71      C
ATOM  10797  O    SER G 116   -5.040  13.101 -27.870  1.00 16.37      O
ATOM  10798  CB   SER G 116   -5.182  10.280 -28.480  1.00 16.51      C
ATOM  10799  OG   SER G 116   -5.523   9.143 -29.247  1.00 17.67      O
ATOM  10800  N    ILE G 117   -6.709  12.569 -26.507  1.00 16.15      N
ATOM  10801  CA   ILE G 117   -6.409  13.593 -25.479  1.00 17.80      C
ATOM  10802  C    ILE G 117   -5.851  12.931 -24.214  1.00 18.08      C
ATOM  10803  O    ILE G 117   -6.280  11.823 -23.823  1.00 15.92      O
ATOM  10804  CB   ILE G 117   -7.658  14.452 -25.153  1.00 17.21      C
ATOM  10805  CG1  ILE G 117   -7.278  15.672 -24.299  1.00 18.25      C
ATOM  10806  CG2  ILE G 117   -8.767  13.632 -24.482  1.00 17.09      C
ATOM  10807  CD1  ILE G 117   -8.400  16.590 -24.109  1.00 18.41      C
ATOM  10808  N    PHE G 118   -4.896  13.569 -23.569  1.00 16.99      N
ATOM  10809  CA   PHE G 118   -4.227  13.057 -22.389  1.00 18.16      C
ATOM  10810  C    PHE G 118   -4.201  14.081 -21.239  1.00 18.76      C
ATOM  10811  O    PHE G 118   -3.739  15.220 -21.431  1.00 17.17      O
ATOM  10812  CB   PHE G 118   -2.812  12.671 -22.753  1.00 18.52      C
ATOM  10813  CG   PHE G 118   -2.723  11.647 -23.854  1.00 16.61      C
ATOM  10814  CD1  PHE G 118   -2.440  12.025 -25.144  1.00 19.22      C
ATOM  10815  CD2  PHE G 118   -2.912  10.308 -23.588  1.00 19.69      C
ATOM  10816  CE1  PHE G 118   -2.348  11.086 -26.159  1.00 20.01      C
ATOM  10817  CE2  PHE G 118   -2.814   9.360 -24.599  1.00 16.42      C
ATOM  10818  CZ   PHE G 118   -2.544   9.744 -25.873  1.00 17.61      C
ATOM  10819  N    PRO G 119   -4.716  13.700 -20.056  1.00 18.80      N
ATOM  10820  CA   PRO G 119   -4.562  14.634 -18.962  1.00 20.35      C
ATOM  10821  C    PRO G 119   -3.135  14.638 -18.426  1.00 20.20      C
ATOM  10822  O    PRO G 119   -2.320  13.795 -18.812  1.00 20.97      O
ATOM  10823  CB   PRO G 119   -5.541  14.125 -17.893  1.00 20.12      C
ATOM  10824  CG   PRO G 119   -5.796  12.722 -18.212  1.00 20.87      C
ATOM  10825  CD   PRO G 119   -5.422  12.469 -19.661  1.00 19.70      C
ATOM  10826  N    PRO G 120   -2.807  15.618 -17.591  1.00 22.58      N
ATOM  10827  CA   PRO G 120   -1.501  15.581 -16.929  1.00 23.77      C
ATOM  10828  C    PRO G 120   -1.262  14.267 -16.148  1.00 24.65      C
ATOM  10829  O    PRO G 120   -2.206  13.677 -15.614  1.00 22.72      O
ATOM  10830  CB   PRO G 120   -1.547  16.801 -16.004  1.00 22.95      C
ATOM  10831  CG   PRO G 120   -2.554  17.691 -16.605  1.00 23.08      C
ATOM  10832  CD   PRO G 120   -3.557  16.848 -17.284  1.00 22.93      C
ATOM  10833  N    SER G 121   -0.018  13.821 -16.098  1.00 26.65      N
ATOM  10834  CA   SER G 121    0.349  12.684 -15.266  1.00 28.30      C
ATOM  10835  C    SER G 121    0.237  13.117 -13.805  1.00 28.81      C
ATOM  10836  O    SER G 121    0.484  14.271 -13.469  1.00 28.14      O
ATOM  10837  CB   SER G 121    1.782  12.221 -15.584  1.00 29.10      C
ATOM  10838  OG   SER G 121    2.689  13.326 -15.535  1.00 30.48      O
ATOM  10839  N    SER G 122   -0.148  12.199 -12.926  1.00 29.68      N
ATOM  10840  CA   SER G 122   -0.046  12.482 -11.500  1.00 30.78      C
ATOM  10841  C    SER G 122    1.415  12.814 -11.130  1.00 31.31      C
ATOM  10842  O    SER G 122    1.658  13.564 -10.201  1.00 33.67      O
ATOM  10843  CB   SER G 122   -0.576  11.321 -10.663  1.00 32.06      C
```

```
ATOM  10844  OG   SER G 122      -0.028  10.073 -11.058  1.00 34.33           O
ATOM  10845  N    GLU G 123       2.382  12.302 -11.899  1.00 30.41           N
ATOM  10846  CA   GLU G 123       3.791  12.634 -11.686  1.00 29.52           C
ATOM  10847  C    GLU G 123       4.085  14.108 -11.807  1.00 27.56           C
ATOM  10848  O    GLU G 123       4.877  14.651 -11.033  1.00 28.08           O
ATOM  10849  CB   GLU G 123       4.697  11.924 -12.685  1.00 30.93           C
ATOM  10850  CG   GLU G 123       4.874  10.465 -12.408  1.00 34.53           C
ATOM  10851  CD   GLU G 123       4.102   9.609 -13.354  1.00 36.57           C
ATOM  10852  OE1  GLU G 123       2.940   9.949 -13.658  1.00 38.99           O
ATOM  10853  OE2  GLU G 123       4.663   8.589 -13.797  1.00 40.66           O
ATOM  10854  N    GLN G 124       3.483  14.741 -12.799  1.00 25.02           N
ATOM  10855  CA   GLN G 124       3.848  16.117 -13.154  1.00 24.51           C
ATOM  10856  C    GLN G 124       3.344  17.141 -12.152  1.00 24.09           C
ATOM  10857  O    GLN G 124       3.952  18.219 -11.982  1.00 22.59           O
ATOM  10858  CB   GLN G 124       3.326  16.457 -14.545  1.00 23.57           C
ATOM  10859  CG   GLN G 124       3.682  17.843 -15.005  1.00 23.23           C
ATOM  10860  CD   GLN G 124       3.261  18.175 -16.404  1.00 22.68           C
ATOM  10861  OE1  GLN G 124       2.348  17.556 -16.986  1.00 21.93           O
ATOM  10862  NE2  GLN G 124       3.923  19.170 -16.970  1.00 22.47           N
ATOM  10863  N    LEU G 125       2.209  16.852 -11.516  1.00 23.88           N
ATOM  10864  CA   LEU G 125       1.566  17.883 -10.725  1.00 22.85           C
ATOM  10865  C    LEU G 125       2.417  18.255  -9.523  1.00 22.06           C
ATOM  10866  O    LEU G 125       2.404  19.403  -9.086  1.00 20.62           O
ATOM  10867  CB   LEU G 125       0.135  17.511 -10.355  1.00 22.04           C
ATOM  10868  CG   LEU G 125      -0.867  17.445 -11.536  1.00 23.47           C
ATOM  10869  CD1  LEU G 125      -2.222  16.997 -11.041  1.00 23.51           C
ATOM  10870  CD2  LEU G 125      -1.044  18.773 -12.249  1.00 23.61           C
ATOM  10871  N    THR G 126       3.222  17.309  -9.058  1.00 21.28           N
ATOM  10872  CA   THR G 126       4.195  17.541  -8.004  1.00 21.71           C
ATOM  10873  C    THR G 126       5.191  18.643  -8.373  1.00 21.75           C
ATOM  10874  O    THR G 126       5.660  19.383  -7.510  1.00 23.27           O
ATOM  10875  CB   THR G 126       4.939  16.219  -7.694  1:00 23.00           C
ATOM  10876  OG1  THR G 126       3.973  15.160  -7.604  1.00 26.66           O
ATOM  10877  CG2  THR G 126       5.682  16.319  -6.417  1.00 22.24           C
ATOM  10878  N    SER G 127       5.509  18.752  -9.661  1.00 19.77           N
ATOM  10879  CA   SER G 127       6.375  19.819 -10.149  1.00 18.78           C
ATOM  10880  C    SER G 127       5.809  21.244 -10.084  1.00 18.77           C
ATOM  10881  O    SER G 127       6.573  22.196 -10.234  1.00 19.70           O
ATOM  10882  CB   SER G 127       6.759  19.570 -11.603  1.00 18.55           C
ATOM  10883  OG   SER G 127       5.681  19.852 -12.460  1.00 16.40           O
ATOM  10884  N    GLY G 128       4.503  21.385  -9.918  1.00 19.01           N
ATOM  10885  CA   GLY G 128       3.822  22.696  -9.907  1.00 19.41           C
ATOM  10886  C    GLY G 128       3.234  23.152 -11.230  1.00 18.59           C
ATOM  10887  O    GLY G 128       2.629  24.228 -11.299  1.00 21.29           O
ATOM  10888  N    GLY G 129       3.419  22.352 -12.273  1.00 17.49           N
ATOM  10889  CA   GLY G 129       2.885  22.608 -13.610  1.00 17.73           C
ATOM  10890  C    GLY G 129       2.034  21.456 -14.105  1.00 18.56           C
ATOM  10891  O    GLY G 129       2.075  20.389 -13.540  1.00 17.76           O
ATOM  10892  N    ALA G 130       1.266  21.691 -15.163  1.00 17.54           N
ATOM  10893  CA   ALA G 130       0.328  20.705 -15.689  1.00 18.73           C
ATOM  10894  C    ALA G 130       0.270  20.854 -17.199  1.00 19.22           C
ATOM  10895  O    ALA G 130       0.024  21.946 -17.697  1.00 20.61           O
ATOM  10896  CB   ALA G 130      -1.035  20.916 -15.084  1.00 19.64           C
ATOM  10897  N    SER G 131       0.573  19.770 -17.916  1.00 17.80           N
ATOM  10898  CA   SER G 131       0.572  19.776 -19.372  1.00 17.62           C
ATOM  10899  C    SER G 131      -0.540  18.889 -19.872  1.00 17.82           C
ATOM  10900  O    SER G 131      -0.577  17.712 -19.526  1.00 16.71           O
ATOM  10901  CB   SER G 131       1.905  19.287 -19.928  1.00 17.85           C
ATOM  10902  OG   SER G 131       2.973  20.172 -19.593  1.00 20.28           O
ATOM  10903  N    VAL G 132      -1.448  19.456 -20.666  1.00 18.11           N
ATOM  10904  CA   VAL G 132      -2.535  18.663 -21.295  1.00 18.53           C
ATOM  10905  C    VAL G 132      -2.207  18.478 -22.779  1.00 17.73           C
ATOM  10906  O    VAL G 132      -1.958  19.448 -23.468  1.00 18.40           O
ATOM  10907  CB   VAL G 132      -3.908  19.337 -21.157  1.00 18.24           C
ATOM  10908  CG1  VAL G 132      -5.022  18.327 -21.433  1.00 18.44           C
```

```
ATOM   10909  CG2 VAL G 132      -4.088  19.894 -19.763  1.00 17.82           C
ATOM   10910  N   VAL G 133      -2.231  17.242 -23.275  1.00 17.67           N
ATOM   10911  CA  VAL G 133      -1.676  16.962 -24.596  1.00 18.12           C
ATOM   10912  C   VAL G 133      -2.704  16.335 -25.516  1.00 19.25           C
ATOM   10913  O   VAL G 133      -3.429  15.446 -25.130  1.00 19.04           O
ATOM   10914  CB  VAL G 133      -0.479  15.999 -24.548  1.00 18.21           C
ATOM   10915  CG1 VAL G 133       0.172  15.833 -25.964  1.00 19.78           C
ATOM   10916  CG2 VAL G 133       0.555  16.459 -23.535  1.00 19.17           C
ATOM   10917  N   CYS G 134      -2.727  16.781 -26.760  1.00 20.41           N
ATOM   10918  CA  CYS G 134      -3.629  16.201 -27.729  1.00 19.27           C
ATOM   10919  C   CYS G 134      -2.865  15.732 -28.981  1.00 17.26           C
ATOM   10920  O   CYS G 134      -2.076  16.480 -29.526  1.00 19.23           O
ATOM   10921  CB  CYS G 134      -4.693  17.229 -28.036  1.00 20.39           C
ATOM   10922  SG  CYS G 134      -6.064  16.582 -28.970  1.00 27.37           S
ATOM   10923  N   PHE G 135      -3.024  14.463 -29.390  1.00 15.79           N
ATOM   10924  CA  PHE G 135      -2.410  13.997 -30.642  1.00 14.93           C
ATOM   10925  C   PHE G 135      -3.466  13.929 -31.740  1.00 15.70           C
ATOM   10926  O   PHE G 135      -4.556  13.386 -31.536  1.00 16.71           O
ATOM   10927  CB  PHE G 135      -1.880  12.544 -30.552  1.00 14.45           C
ATOM   10928  CG  PHE G 135      -0.737  12.321 -29.602  1.00 13.34           C
ATOM   10929  CD1 PHE G 135       0.075  13.317 -29.159  1.00 12.77           C
ATOM   10930  CD2 PHE G 135      -0.446  10.995 -29.197  1.00 12.38           C
ATOM   10931  CE1 PHE G 135       1.129  13.078 -28.306  1.00 14.47           C
ATOM   10932  CE2 PHE G 135       0.630  10.733 -28.336  1.00 14.19           C
ATOM   10933  CZ  PHE G 135       1.421  11.768 -27.872  1.00 15.03           C
ATOM   10934  N   LEU G 136      -3.105  14.413 -32.911  1.00 17.55           N
ATOM   10935  CA  LEU G 136      -3.926  14.344 -34.126  1.00 18.64           C
ATOM   10936  C   LEU G 136      -3.097  13.514 -35.070  1.00 18.09           C
ATOM   10937  O   LEU G 136      -2.186  14.005 -35.708  1.00 17.92           O
ATOM   10938  CB  LEU G 136      -4.125  15.745 -34.707  1.00 20.76           C
ATOM   10939  CG  LEU G 136      -5.153  16.690 -34.061  1.00 23.96           C
ATOM   10940  CD1 LEU G 136      -4.968  16.846 -32.572  1.00 25.14           C
ATOM   10941  CD2 LEU G 136      -5.088  18.066 -34.719  1.00 24.10           C
ATOM   10942  N   ASN G 137      -3.355  12.217 -35.123  1.00 17.28           N
ATOM   10943  CA  ASN G 137      -2.471  11.321 -35.841  1.00 16.23           C
ATOM   10944  C   ASN G 137      -2.937  10.880 -37.235  1.00 16.59           C
ATOM   10945  O   ASN G 137      -4.130  10.640 -37.464  1.00 15.53           O
ATOM   10946  CB  ASN G 137      -2.196  10.090 -34.959  1.00 16.02           C
ATOM   10947  CG  ASN G 137      -1.202  10.370 -33.841  1.00 15.99           C
ATOM   10948  OD1 ASN G 137      -0.527  11.383 -33.846  1.00 19.36           O
ATOM   10949  ND2 ASN G 137      -1.088   9.444 -32.889  1.00 17.52           N
ATOM   10950  N   ASN G 138      -1.949  10.775 -38.130  1.00 17.13           N
ATOM   10951  CA  ASN G 138      -2.061  10.229 -39.483  1.00 18.13           C
ATOM   10952  C   ASN G 138      -3.194  10.754 -40.347  1.00 18.39           C
ATOM   10953  O   ASN G 138      -4.091  10.018 -40.789  1.00 19.72           O
ATOM   10954  CB  ASN G 138      -2.098   8.707 -39.409  1.00 18.77           C
ATOM   10955  CG  ASN G 138      -0.932   8.144 -38.594  1.00 20.92           C
ATOM   10956  OD1 ASN G 138      -1.098   7.775 -37.442  1.00 24.00           O
ATOM   10957  ND2 ASN G 138       0.235   8.088 -39.193  1.00 21.53           N
ATOM   10958  N   PHE G 139      -3.078  12.026 -40.670  1.00 17.90           N
ATOM   10959  CA  PHE G 139      -4.058  12.695 -41.490  1.00 17.91           C
ATOM   10960  C   PHE G 139      -3.426  13.268 -42.752  1.00 16.80           C
ATOM   10961  O   PHE G 139      -2.194  13.403 -42.852  1.00 13.48           O
ATOM   10962  CB  PHE G 139      -4.726  13.777 -40.678  1.00 17.59           C
ATOM   10963  CG  PHE G 139      -3.796  14.760 -40.112  1.00 17.33           C
ATOM   10964  CD1 PHE G 139      -3.417  15.853 -40.871  1.00 19.24           C
ATOM   10965  CD2 PHE G 139      -3.278  14.615 -38.814  1.00 17.28           C
ATOM   10966  CE1 PHE G 139      -2.527  16.799 -40.356  1.00 18.59           C
ATOM   10967  CE2 PHE G 139      -2.411  15.561 -38.288  1.00 19.38           C
ATOM   10968  CZ  PHE G 139      -2.025  16.650 -39.059  1.00 19.66           C
ATOM   10969  N   TYR G 140      -4.291  13.586 -43.714  1.00 17.56           N
ATOM   10970  CA  TYR G 140      -3.879  14.241 -44.950  1.00 19.44           C
ATOM   10971  C   TYR G 140      -5.104  14.987 -45.480  1.00 20.81           C
ATOM   10972  O   TYR G 140      -6.196  14.410 -45.554  1.00 19.66           O
ATOM   10973  CB  TYR G 140      -3.331  13.264 -46.010  1.00 20.88           C
```

```
ATOM   10974   CG   TYR G 140     -2.794   14.046  -47.182   1.00  20.57        C
ATOM   10975   CD1  TYR G 140     -3.656   14.501  -48.180   1.00  21.73        C
ATOM   10976   CD2  TYR G 140     -1.450   14.420  -47.244   1.00  20.36        C
ATOM   10977   CE1  TYR G 140     -3.196   15.283  -49.215   1.00  22.79        C
ATOM   10978   CE2  TYR G 140     -0.977   15.195  -48.295   1.00  21.06        C
ATOM   10979   CZ   TYR G 140     -1.871   15.628  -49.269   1.00  22.36        C
ATOM   10980   OH   TYR G 140     -1.455   16.397  -50.316   1.00  22.71        O
ATOM   10981   N    PRO G 141     -4.933   16.267  -45.863   1.00  20.50        N
ATOM   10982   CA   PRO G 141     -3.709   17.046  -45.979   1.00  21.01        C
ATOM   10983   C    PRO G 141     -3.173   17.657  -44.667   1.00  20.75        C
ATOM   10984   O    PRO G 141     -3.757   17.501  -43.588   1.00  18.86        O
ATOM   10985   CB   PRO G 141     -4.104   18.141  -46.965   1.00  21.29        C
ATOM   10986   CG   PRO G 141     -5.510   18.363  -46.692   1.00  20.89        C
ATOM   10987   CD   PRO G 141     -6.105   17.049  -46.299   1.00  20.23        C
ATOM   10988   N    LYS G 142     -2.033   18.325  -44.783   1.00  21.99        N
ATOM   10989   CA   LYS G 142     -1.327   18.857  -43.609   1.00  22.77        C
ATOM   10990   C    LYS G 142     -2.122   19.900  -42.825   1.00  24.48        C
ATOM   10991   O    LYS G 142     -2.015   19.967  -41.586   1.00  25.16        O
ATOM   10992   CB   LYS G 142      0.032   19.436  -44.040   1.00  22.96        C
ATOM   10993   CG   LYS G 142      0.903   19.781  -42.871   1.00  23.85        C
ATOM   10994   CD   LYS G 142      2.257   20.300  -43.266   1.00  25.00        C
ATOM   10995   CE   LYS G 142      3.161   20.354  -42.020   1.00  26.68        C
ATOM   10996   NZ   LYS G 142      4.604   20.618  -42.363   1.00  28.18        N
ATOM   10997   N    ASP G 143     -2.912   20.723  -43.521   1.00  25.63        N
ATOM   10998   CA   ASP G 143     -3.623   21.798  -42.838   1.00  25.16        C
ATOM   10999   C    ASP G 143     -4.640   21.225  -41.882   1.00  24.23        C
ATOM   11000   O    ASP G 143     -5.480   20.436  -42.278   1.00  23.87        O
ATOM   11001   CB   ASP G 143     -4.335   22.747  -43.801   1.00  27.04        C
ATOM   11002   CG   ASP G 143     -4.705   24.067  -43.142   1.00  29.71        C
ATOM   11003   OD1  ASP G 143     -5.880   24.516  -43.255   1.00  35.59        O
ATOM   11004   OD2  ASP G 143     -3.813   24.661  -42.499   1.00  34.89        O
ATOM   11005   N    ILE G 144     -4.563   21.645  -40.625   1.00  23.83        N
ATOM   11006   CA   ILE G 144     -5.472   21.170  -39.594   1.00  24.03        C
ATOM   11007   C    ILE G 144     -5.572   22.205  -38.488   1.00  25.35        C
ATOM   11008   O    ILE G 144     -4.622   22.969  -38.268   1.00  21.76        O
ATOM   11009   CB   ILE G 144     -5.005   19.820  -39.035   1.00  24.08        C
ATOM   11010   CG1  ILE G 144     -6.158   19.123  -38.319   1.00  24.53        C
ATOM   11011   CG2  ILE G 144     -3.767   20.001  -38.123   1.00  22.49        C
ATOM   11012   CD1  ILE G 144     -6.047   17.585  -38.384   1.00  23.93        C
ATOM   11013   N    ASN G 145     -6.734   22.250  -37.829   1.00  27.25        N
ATOM   11014   CA   ASN G 145     -6.980   23.196  -36.747   1.00  27.90        C
ATOM   11015   C    ASN G 145     -7.296   22.475  -35.467   1.00  27.83        C
ATOM   11016   O    ASN G 145     -8.021   21.485  -35.482   1.00  26.22        O
ATOM   11017   CB   ASN G 145     -8.141   24.138  -37.062   1.00  29.30        C
ATOM   11018   CG   ASN G 145     -8.392   25.157  -35.948   1.00  30.47        C
ATOM   11019   OD1  ASN G 145     -9.261   24.962  -35.082   1.00  32.57        O
ATOM   11020   ND2  ASN G 145     -7.625   26.247  -35.962   1.00  33.49        N
ATOM   11021   N    VAL G 146     -6.730   22.975  -34.367   1.00  28.27        N
ATOM   11022   CA   VAL G 146     -7.060   22.475  -33.048   1.00  28.37        C
ATOM   11023   C    VAL G 146     -7.548   23.618  -32.144   1.00  28.11        C
ATOM   11024   O    VAL G 146     -6.936   24.680  -32.100   1.00  26.55        O
ATOM   11025   CB   VAL G 146     -5.872   21.672  -32.436   1.00  29.79        C
ATOM   11026   CG1  VAL G 146     -4.716   22.577  -32.145   1.00  31.82        C
ATOM   11027   CG2  VAL G 146     -6.314   20.920  -31.178   1.00  29.93        C
ATOM   11028   N    LYS G 147     -8.664   23.385  -31.454   1.00  28.11        N
ATOM   11029   CA   LYS G 147     -9.231   24.350  -30.504   1.00  28.65        C
ATOM   11030   C    LYS G 147     -9.237   23.673  -29.142   1.00  27.50        C
ATOM   11031   O    LYS G 147     -9.730   22.543  -29.025   1.00  28.85        O
ATOM   11032   CB   LYS G 147    -10.661   24.707  -30.920   1.00  29.55        C
ATOM   11033   CG   LYS G 147    -11.342   25.810  -30.123   1.00  31.05        C
ATOM   11034   CD   LYS G 147    -12.562   26.365  -30.902   1.00  31.63        C
ATOM   11035   CE   LYS G 147    -13.622   26.967  -29.972   1.00  33.75        C
ATOM   11036   NZ   LYS G 147    -14.618   27.873  -30.682   1.00  34.97        N
ATOM   11037   N    TRP G 148     -8.670   24.351  -28.144   1.00  26.84        N
ATOM   11038   CA   TRP G 148     -8.719   23.897  -26.742   1.00  27.00        C
```

```
ATOM   11039  C    TRP G 148      -9.884   24.568 -26.049   1.00 27.01           C
ATOM   11040  O    TRP G 148     -10.078   25.781 -26.197   1.00 26.27           O
ATOM   11041  CB   TRP G 148      -7.423   24.234 -25.978   1.00 25.50           C
ATOM   11042  CG   TRP G 148      -6.317   23.300 -26.266   1.00 25.35           C
ATOM   11043  CD1  TRP G 148      -5.312   23.472 -27.163   1.00 25.00           C
ATOM   11044  CD2  TRP G 148      -6.123   22.002 -25.683   1.00 25.09           C
ATOM   11045  NE1  TRP G 148      -4.484   22.370 -27.164   1.00 25.62           N
ATOM   11046  CE2  TRP G 148      -4.965   21.453 -26.266   1.00 24.99           C
ATOM   11047  CE3  TRP G 148      -6.814   21.261 -24.718   1.00 23.61           C
ATOM   11048  CZ2  TRP G 148      -4.474   20.206 -25.908   1.00 24.73           C
ATOM   11049  CZ3  TRP G 148      -6.342   20.015 -24.377   1.00 24.23           C
ATOM   11050  CH2  TRP G 148      -5.166   19.507 -24.953   1.00 25.27           C
ATOM   11051  N    LYS G 149     -10.670   23.770 -25.326   1.00 26.82           N
ATOM   11052  CA   LYS G 149     -11.772   24.300 -24.533   1.00 28.51           C
ATOM   11053  C    LYS G 149     -11.636   23.792 -23.108   1.00 27.97           C
ATOM   11054  O    LYS G 149     -11.314   22.628 -22.885   1.00 28.70           O
ATOM   11055  CB   LYS G 149     -13.119   23.913 -25.156   1.00 29.58           C
ATOM   11056  CG   LYS G 149     -13.594   24.863 -26.274   1.00 30.61           C
ATOM   11057  CD   LYS G 149     -14.510   24.141 -27.276   1.00 30.72           C
ATOM   11058  CE   LYS G 149     -15.811   24.879 -27.546   1.00 31.92           C
ATOM   11059  NZ   LYS G 149     -16.437   24.482 -28.842   1.00 32.62           N
ATOM   11060  N    ILE G 150     -11.823   24.696 -22.156   1.00 27.96           N
ATOM   11061  CA   ILE G 150     -11.775   24.391 -20.727   1.00 28.97           C
ATOM   11062  C    ILE G 150     -13.068   24.893 -20.079   1.00 29.70           C
ATOM   11063  O    ILE G 150     -13.379   26.073 -20.165   1.00 27.54           O
ATOM   11064  CB   ILE G 150     -10.603   25.106 -20.052   1.00 28.70           C
ATOM   11065  CG1  ILE G 150      -9.288   24.610 -20.628   1.00 27.88           C
ATOM   11066  CG2  ILE G 150     -10.632   24.907 -18.548   1.00 28.96           C
ATOM   11067  CD1  ILE G 150      -8.136   25.541 -20.347   1.00 28.69           C
ATOM   11068  N    ASP G 151     -13.812   24.001 -19.439   1.00 30.62           N
ATOM   11069  CA   ASP G 151     -15.138   24.353 -18.934   1.00 32.12           C
ATOM   11070  C    ASP G 151     -15.967   25.069 -20.019   1.00 33.67           C
ATOM   11071  O    ASP G 151     -16.546   26.142 -19.788   1.00 34.55           O
ATOM   11072  CB   ASP G 151     -15.018   25.198 -17.662   1.00 31.47           C
ATOM   11073  CG   ASP G 151     -14.655   24.376 -16.447   1.00 31.82           C
ATOM   11074  OD1  ASP G 151     -14.766   23.141 -16.510   1.00 31.50           O
ATOM   11075  OD2  ASP G 151     -14.274   24.977 -15.417   1.00 31.48           O
ATOM   11076  N    GLY G 152     -15.983   24.482 -21.214   1.00 34.86           N
ATOM   11077  CA   GLY G 152     -16.787   24.991 -22.318   1.00 35.85           C
ATOM   11078  C    GLY G 152     -16.240   26.192 -23.071   1.00 37.03           C
ATOM   11079  O    GLY G 152     -16.643   26.434 -24.215   1.00 39.07           O
ATOM   11080  N    SER G 153     -15.350   26.955 -22.445   1.00 37.35           N
ATOM   11081  CA   SER G 153     -14.757   28.124 -23.073   1.00 37.71           C
ATOM   11082  C    SER G 153     -13.427   27.788 -23.738   1.00 38.13           C
ATOM   11083  O    SER G 153     -12.587   27.086 -23.168   1.00 37.52           O
ATOM   11084  CB   SER G 153     -14.514   29.221 -22.038   1.00 37.83           C
ATOM   11085  OG   SER G 153     -15.600   29.311 -21.145   1.00 38.58           O
ATOM   11086  N    GLU G 154     -13.253   28.329 -24.940   1.00 38.85           N
ATOM   11087  CA   GLU G 154     -12.027   28.217 -25.703   1.00 39.64           C
ATOM   11088  C    GLU G 154     -10.873   28.834 -24.940   1.00 39.88           C
ATOM   11089  O    GLU G 154     -11.055   29.802 -24.203   1.00 38.82           O
ATOM   11090  CB   GLU G 154     -12.175   28.930 -27.046   1.00 39.63           C
ATOM   11091  CG   GLU G 154     -10.993   28.759 -27.980   1.00 39.69           C
ATOM   11092  CD   GLU G 154     -11.143   29.551 -29.262   1.00 40.18           C
ATOM   11093  OE1  GLU G 154     -11.545   30.730 -29.207   1.00 38.92           O
ATOM   11094  OE2  GLU G 154     -10.833   29.002 -30.326   1.00 41.42           O
ATOM   11095  N    ARG G 155      -9.691   28.264 -25.144   1.00 41.41           N
ATOM   11096  CA   ARG G 155      -8.488   28.635 -24.408   1.00 41.91           C
ATOM   11097  C    ARG G 155      -7.331   28.770 -25.387   1.00 42.62           C
ATOM   11098  O    ARG G 155      -6.935   27.782 -26.013   1.00 42.58           O
ATOM   11099  CB   ARG G 155      -8.161   27.550 -23.373   1.00 43.12           C
ATOM   11100  CG   ARG G 155      -6.740   27.613 -22.793   1.00 43.39           C
ATOM   11101  CD   ARG G 155      -6.482   28.939 -22.078   1.00 45.71           C
ATOM   11102  NE   ARG G 155      -5.206   28.927 -21.353   1.00 46.33           N
ATOM   11103  CZ   ARG G 155      -5.068   28.971 -20.027   1.00 47.08           C
```

```
ATOM   11104  NH1 ARG G 155     -6.119  29.008 -19.207  1.00 47.57           N
ATOM   11105  NH2 ARG G 155     -3.846  28.975 -19.510  1.00 47.94           N
ATOM   11106  N   GLN G 156     -6.773  29.975 -25.499  1.00 42.86           N
ATOM   11107  CA  GLN G 156     -5.670  30.221 -26.436  1.00 42.60           C
ATOM   11108  C   GLN G 156     -4.286  30.346 -25.780  1.00 42.24           C
ATOM   11109  O   GLN G 156     -3.281  30.039 -26.433  1.00 42.93           O
ATOM   11110  CB  GLN G 156     -5.962  31.461 -27.286  1.00 44.00           C
ATOM   11111  CG  GLN G 156     -7.307  31.418 -28.013  1.00 45.49           C
ATOM   11112  CD  GLN G 156     -7.308  30.479 -29.198  1.00 47.54           C
ATOM   11113  OE1 GLN G 156     -6.717  29.398 -29.149  1.00 49.12           O
ATOM   11114  NE2 GLN G 156     -7.980  30.883 -30.276  1.00 48.50           N
ATOM   11115  N   ASN G 157     -4.219  30.774 -24.510  1.00 41.26           N
ATOM   11116  CA  ASN G 157     -2.909  30.959 -23.844  1.00 39.56           C
ATOM   11117  C   ASN G 157     -2.265  29.603 -23.555  1.00 37.03           C
ATOM   11118  O   ASN G 157     -2.942  28.645 -23.203  1.00 37.37           O
ATOM   11119  CB  ASN G 157     -2.956  31.754 -22.514  1.00 41.41           C
ATOM   11120  CG  ASN G 157     -4.251  32.526 -22.296  1.00 44.48           C
ATOM   11121  OD1 ASN G 157     -4.895  32.378 -21.251  1.00 47.61           O
ATOM   11122  ND2 ASN G 157     -4.623  33.372 -23.257  1.00 47.81           N
ATOM   11123  N   GLY G 158     -0.956  29.539 -23.729  1.00 34.10           N
ATOM   11124  CA  GLY G 158     -0.173  28.396 -23.322  1.00 32.10           C
ATOM   11125  C   GLY G 158     -0.313  27.158 -24.189  1.00 29.89           C
ATOM   11126  O   GLY G 158     -0.113  26.070 -23.695  1.00 28.93           O
ATOM   11127  N   VAL G 159     -0.630  27.333 -25.471  1.00 27.63           N
ATOM   11128  CA  VAL G 159     -0.797  26.207 -26.419  1.00 26.43           C
ATOM   11129  C   VAL G 159      0.360  26.241 -27.394  1.00 25.03           C
ATOM   11130  O   VAL G 159      0.625  27.278 -27.985  1.00 24.02           O
ATOM   11131  CB  VAL G 159     -2.121  26.295 -27.217  1.00 25.48           C
ATOM   11132  CG1 VAL G 159     -2.233  25.152 -28.266  1.00 26.58           C
ATOM   11133  CG2 VAL G 159     -3.286  26.259 -26.278  1.00 24.53           C
ATOM   11134  N   LEU G 160      1.024  25.098 -27.580  1.00 23.47           N
ATOM   11135  CA  LEU G 160      2.030  24.967 -28.613  1.00 23.32           C
ATOM   11136  C   LEU G 160      1.820  23.720 -29.468  1.00 22.88           C
ATOM   11137  O   LEU G 160      1.690  22.603 -28.932  1.00 20.93           O
ATOM   11138  CB  LEU G 160      3.418  24.937 -27.976  1.00 24.06           C
ATOM   11139  CG  LEU G 160      3.810  26.246 -27.244  1.00 24.54           C
ATOM   11140  CD1 LEU G 160      5.105  26.072 -26.477  1.00 25.68           C
ATOM   11141  CD2 LEU G 160      3.960  27.393 -28.209  1.00 26.46           C
ATOM   11142  N   ASN G 161      1.847  23.907 -30.794  1.00 21.75           N
ATOM   11143  CA  ASN G 161      1.624  22.790 -31.754  1.00 21.33           C
ATOM   11144  C   ASN G 161      2.879  22.422 -32.553  1.00 19.58           C
ATOM   11145  O   ASN G 161      3.721  23.269 -32.828  1.00 17.82           O
ATOM   11146  CB  ASN G 161      0.484  23.156 -32.716  1.00 21.80           C
ATOM   11147  CG  ASN G 161     -0.792  23.571 -31.998  1.00 21.06           C
ATOM   11148  OD1 ASN G 161     -1.176  22.952 -31.032  1.00 21.09           O
ATOM   11149  ND2 ASN G 161     -1.443  24.646 -32.467  1.00 20.32           N
ATOM   11150  N   SER G 162      2.996  21.146 -32.937  1.00 18.61           N
ATOM   11151  CA  SER G 162      4.152  20.644 -33.658  1.00 18.79           C
ATOM   11152  C   SER G 162      3.741  19.570 -34.641  1.00 19.42           C
ATOM   11153  O   SER G 162      3.110  18.588 -34.231  1.00 16.10           O
ATOM   11154  CB  SER G 162      5.128  19.988 -32.689  1.00 18.16           C
ATOM   11155  OG  SER G 162      6.294  19.560 -33.375  1.00 14.12           O
ATOM   11156  N   TRP G 163      4.106  19.755 -35.907  1.00 19.63           N
ATOM   11157  CA  TRP G 163      3.757  18.820 -36.990  1.00 20.40           C
ATOM   11158  C   TRP G 163      4.924  17.899 -37.316  1.00 20.01           C
ATOM   11159  O   TRP G 163      6.068  18.333 -37.408  1.00 18.41           O
ATOM   11160  CB  TRP G 163      3.417  19.603 -38.267  1.00 22.82           C
ATOM   11161  CG  TRP G 163      1.981  20.072 -38.433  1.00 21.98           C
ATOM   11162  CD1 TRP G 163      1.024  19.511 -39.246  1.00 23.70           C
ATOM   11163  CD2 TRP G 163      1.377  21.243 -37.856  1.00 23.90           C
ATOM   11164  NE1 TRP G 163     -0.147  20.245 -39.180  1.00 24.24           N
ATOM   11165  CE2 TRP G 163      0.048  21.318 -38.343  1.00 24.13           C
ATOM   11166  CE3 TRP G 163      1.828  22.241 -36.979  1.00 23.99           C
ATOM   11167  CZ2 TRP G 163     -0.843  22.341 -37.954  1.00 25.24           C
ATOM   11168  CZ3 TRP G 163      0.943  23.260 -36.598  1.00 24.15           C
```

```
ATOM  11169  CH2 TRP G 163    -0.374  23.306 -37.092  1.00 23.94      C
ATOM  11170  N   THR G 164     4.650  16.633 -37.554  1.00 19.83      N
ATOM  11171  CA  THR G 164     5.700  15.759 -38.038  1.00 19.30      C
ATOM  11172  C   THR G 164     5.985  16.088 -39.509  1.00 19.73      C
ATOM  11173  O   THR G 164     5.237  16.824 -40.155  1.00 19.83      O
ATOM  11174  CB  THR G 164     5.295  14.276 -37.932  1.00 18.97      C
ATOM  11175  OG1 THR G 164     4.165  14.041 -38.775  1.00 16.94      O
ATOM  11176  CG2 THR G 164     4.998  13.906 -36.503  1.00 17.21      C
ATOM  11177  N   ASP G 165     7.072  15.549 -40.045  1.00 20.22      N
ATOM  11178  CA  ASP G 165     7.269  15.547 -41.508  1.00 21.00      C
ATOM  11179  C   ASP G 165     6.384  14.482 -42.108  1.00 21.11      C
ATOM  11180  O   ASP G 165     5.939  13.608 -41.392  1.00 21.42      O
ATOM  11181  CB  ASP G 165     8.715  15.234 -41.865  1.00 21.67      C
ATOM  11182  CG  ASP G 165     9.646  16.363 -41.525  1.00 26.27      C
ATOM  11183  OD1 ASP G 165     9.265  17.534 -41.778  1.00 28.66      O
ATOM  11184  OD2 ASP G 165    10.751  16.065 -41.015  1.00 31.23      O
ATOM  11185  N   GLN G 166     6.183  14.528 -43.432  1.00 21.31      N
ATOM  11186  CA  GLN G 166     5.334  13.550 -44.117  1.00 20.29      C
ATOM  11187  C   GLN G 166     5.955  12.170 -43.956  1.00 21.12      C
ATOM  11188  O   GLN G 166     7.164  12.003 -44.126  1.00 20.28      O
ATOM  11189  CB  GLN G 166     5.224  13.882 -45.601  1.00 21.56      C
ATOM  11190  CG  GLN G 166     4.162  13.115 -46.346  1.00 21.00      C
ATOM  11191  CD  GLN G 166     3.753  13.835 -47.626  1.00 20.81      C
ATOM  11192  OE1 GLN G 166     4.570  14.515 -48.257  1.00 19.80      O
ATOM  11193  NE2 GLN G 166     2.504  13.674 -48.018  1.00 20.80      N
ATOM  11194  N   ASP G 167     5.123  11.201 -43.610  1.00 20.77      N
ATOM  11195  CA  ASP G 167     5.581   9.840 -43.345  1.00 20.22      C
ATOM  11196  C   ASP G 167     6.068   9.199 -44.634  1.00 18.83      C
ATOM  11197  O   ASP G 167     5.407   9.272 -45.650  1.00 16.38      O
ATOM  11198  CB  ASP G 167     4.423   9.040 -42.728  1.00 20.34      C
ATOM  11199  CG  ASP G 167     4.836   7.665 -42.287  1.00 22.52      C
ATOM  11200  OD1 ASP G 167     5.424   7.546 -41.176  1.00 25.42      O
ATOM  11201  OD2 ASP G 167     4.571   6.704 -43.047  1.00 22.88      O
ATOM  11202  N   SER G 168     7.205   8.502 -44.576  1.00 19.80      N
ATOM  11203  CA  SER G 168     7.767   7.834 -45.746  1.00 20.56      C
ATOM  11204  C   SER G 168     6.947   6.624 -46.215  1.00 22.15      C
ATOM  11205  O   SER G 168     7.166   6.086 -47.313  1.00 21.89      O
ATOM  11206  CB  SER G 168     9.190   7.372 -45.438  1.00 20.00      C
ATOM  11207  OG  SER G 168    10.063   8.474 -45.237  1.00 16.18      O
ATOM  11208  N   LYS G 169     6.020   6.189 -45.372  1.00 24.01      N
ATOM  11209  CA  LYS G 169     5.294   4.963 -45.622  1.00 23.97      C
ATOM  11210  C   LYS G 169     3.877   5.212 -46.102  1.00 23.42      C
ATOM  11211  O   LYS G 169     3.504   4.752 -47.178  1.00 24.62      O
ATOM  11212  CB  LYS G 169     5.299   4.115 -44.353  1.00 26.50      C
ATOM  11213  CG  LYS G 169     5.641   2.673 -44.607  1.00 30.53      C
ATOM  11214  CD  LYS G 169     7.125   2.528 -44.953  1.00 34.18      C
ATOM  11215  CE  LYS G 169     7.343   1.577 -46.146  1.00 35.52      C
ATOM  11216  NZ  LYS G 169     6.800   2.109 -47.437  1.00 38.06      N
ATOM  11217  N   ASP G 170     3.091   5.937 -45.310  1.00 22.97      N
ATOM  11218  CA  ASP G 170     1.671   6.169 -45.612  1.00 22.01      C
ATOM  11219  C   ASP G 170     1.363   7.615 -46.055  1.00 20.02      C
ATOM  11220  O   ASP G 170     0.232   7.950 -46.312  1.00 20.73      O
ATOM  11221  CB  ASP G 170     0.772   5.720 -44.439  1.00 22.72      C
ATOM  11222  CG  ASP G 170     0.816   6.662 -43.225  1.00 25.50      C
ATOM  11223  OD1 ASP G 170     1.441   7.752 -43.279  1.00 25.72      O
ATOM  11224  OD2 ASP G 170     0.181   6.312 -42.194  1.00 26.47      O
ATOM  11225  N   SER G 171     2.385   8.445 -46.124  1.00 18.46      N
ATOM  11226  CA  SER G 171     2.315   9.804 -46.679  1.00 18.75      C
ATOM  11227  C   SER G 171     1.407  10.761 -45.890  1.00 17.77      C
ATOM  11228  O   SER G 171     0.987  11.796 -46.402  1.00 18.29      O
ATOM  11229  CB  SER G 171     1.910   9.799 -48.166  1.00 19.53      C
ATOM  11230  OG  SER G 171     2.752   8.986 -48.953  1.00 21.16      O
ATOM  11231  N   THR G 172     1.171  10.446 -44.631  1.00 18.45      N
ATOM  11232  CA  THR G 172     0.369  11.285 -43.759  1.00 18.15      C
ATOM  11233  C   THR G 172     1.253  12.205 -42.879  1.00 16.74      C
```

```
ATOM  11234  O    THR G 172    2.496  12.083 -42.816  1.00 16.83    O
ATOM  11235  CB   THR G 172   -0.499  10.422 -42.820  1.00 19.03    C
ATOM  11236  OG1  THR G 172    0.344   9.679 -41.931  1.00 18.44    O
ATOM  11237  CG2  THR G 172   -1.382   9.445 -43.587  1.00 19.16    C
ATOM  11238  N    TYR G 173    0.571  13.109 -42.183  1.00 17.67    N
ATOM  11239  CA   TYR G 173    1.147  13.989 -41.167  1.00 16.47    C
ATOM  11240  C    TYR G 173    0.476  13.661 -39.871  1.00 16.23    C
ATOM  11241  O    TYR G 173   -0.575  13.044 -39.853  1.00 18.85    O
ATOM  11242  CB   TYR G 173    0.871  15.475 -41.495  1.00 17.97    C
ATOM  11243  CG   TYR G 173    1.512  15.888 -42.784  1.00 16.54    C
ATOM  11244  CD1  TYR G 173    0.833  15.765 -43.988  1.00 18.38    C
ATOM  11245  CD2  TYR G 173    2.808  16.353 -42.811  1.00 16.25    C
ATOM  11246  CE1  TYR G 173    1.444  16.127 -45.199  1.00 18.23    C
ATOM  11247  CE2  TYR G 173    3.415  16.704 -44.015  1.00 18.05    C
ATOM  11248  CZ   TYR G 173    2.726  16.590 -45.194  1.00 17.04    C
ATOM  11249  OH   TYR G 173    3.341  16.939 -46.412  1.00 20.85    O
ATOM  11250  N    SER G 174    1.131  14.021 -38.782  1.00 16.21    N
ATOM  11251  CA   SER G 174    0.528  14.024 -37.470  1.00 13.95    C
ATOM  11252  C    SER G 174    0.940  15.294 -36.799  1.00 15.78    C
ATOM  11253  O    SER G 174    1.961  15.845 -37.141  1.00 16.16    O
ATOM  11254  CB   SER G 174    1.052  12.884 -36.637  1.00 14.68    C
ATOM  11255  OG   SER G 174    0.745  11.654 -37.280  1.00 13.20    O
ATOM  11256  N    MET G 175    0.191  15.680 -35.784  1.00 16.46    N
ATOM  11257  CA   MET G 175    0.413  16.927 -35.092  1.00 18.04    C
ATOM  11258  C    MET G 175    0.129  16.730 -33.602  1.00 18.03    C
ATOM  11259  O    MET G 175   -0.773  15.989 -33.224  1.00 17.37    O
ATOM  11260  CB   MET G 175   -0.510  17.951 -35.706  1.00 20.76    C
ATOM  11261  CG   MET G 175   -0.344  19.352 -35.224  1.00 24.54    C
ATOM  11262  SD   MET G 175   -1.626  19.702 -34.016  1.00 32.58    S
ATOM  11263  CE   MET G 175   -1.880  21.406 -34.436  1.00 30.72    C
ATOM  11264  N    SER G 176    0.952  17.338 -32.742  1.00 18.10    N
ATOM  11265  CA   SER G 176    0.672  17.352 -31.321  1.00 18.20    C
ATOM  11266  C    SER G 176    0.309  18.788 -30.951  1.00 16.54    C
ATOM  11267  O    SER G 176    0.900  19.720 -31.502  1.00 18.54    O
ATOM  11268  CB   SER G 176    1.913  17.009 -30.514  1.00 19.75    C
ATOM  11269  OG   SER G 176    2.844  18.091 -30.655  1.00 21.00    O
ATOM  11270  N    SER G 177   -0.611  18.933 -29.994  1.00 17.70    N
ATOM  11271  CA   SER G 177   -0.986  20.230 -29.368  1.00 17.66    C
ATOM  11272  C    SER G 177   -0.861  20.024 -27.865  1.00 17.98    C
ATOM  11273  O    SER G 177   -1.439  19.077 -27.326  1.00 18.39    O
ATOM  11274  CB   SER G 177   -2.443  20.622 -29.653  1.00 16.88    C
ATOM  11275  OG   SER G 177   -2.719  22.017 -29.325  1.00 16.75    O
ATOM  11276  N    THR G 178   -0.098  20.890 -27.200  1.00 18.01    N
ATOM  11277  CA   THR G 178    0.182  20.773 -25.776  1.00 18.67    C
ATOM  11278  C    THR G 178   -0.193  22.115 -25.130  1.00 19.40    C
ATOM  11279  O    THR G 178    0.293  23.154 -25.538  1.00 19.86    O
ATOM  11280  CB   THR G 178    1.658  20.432 -25.475  1.00 18.05    C
ATOM  11281  OG1  THR G 178    2.063  19.278 -26.235  1.00 19.25    O
ATOM  11282  CG2  THR G 178    1.884  20.161 -23.949  1.00 16.99    C
ATOM  11283  N    LEU G 179   -1.099  22.042 -24.165  1.00 20.91    N
ATOM  11284  CA   LEU G 179   -1.551  23.175 -23.375  1.00 21.59    C
ATOM  11285  C    LEU G 179   -0.866  23.093 -22.005  1.00 22.33    C
ATOM  11286  O    LEU G 179   -1.048  22.112 -21.276  1.00 21.61    O
ATOM  11287  CB   LEU G 179   -3.066  23.092 -23.212  1.00 22.14    C
ATOM  11288  CG   LEU G 179   -3.753  23.871 -22.104  1.00 23.03    C
ATOM  11289  CD1  LEU G 179   -3.737  25.341 -22.420  1.00 23.34    C
ATOM  11290  CD2  LEU G 179   -5.184  23.360 -21.943  1.00 23.73    C
ATOM  11291  N    THR G 180   -0.089  24.119 -21.657  1.00 21.84    N
ATOM  11292  CA   THR G 180    0.705  24.103 -20.441  1.00 23.35    C
ATOM  11293  C    THR G 180    0.140  25.095 -19.433  1.00 24.49    C
ATOM  11294  O    THR G 180   -0.071  26.279 -19.748  1.00 24.43    O
ATOM  11295  CB   THR G 180    2.176  24.416 -20.693  1.00 25.19    C
ATOM  11296  OG1  THR G 180    2.292  25.686 -21.339  1.00 30.33    O
ATOM  11297  CG2  THR G 180    2.790  23.377 -21.556  1.00 25.42    C
ATOM  11298  N    LEU G 181   -0.105  24.586 -18.231  1.00 24.16    N
```

```
ATOM  11299  CA   LEU G 181     -0.780  25.316 -17.161  1.00 24.13          C
ATOM  11300  C    LEU G 181     -0.027  25.099 -15.871  1.00 24.26          C
ATOM  11301  O    LEU G 181      0.839  24.202 -15.764  1.00 22.93          O
ATOM  11302  CB   LEU G 181     -2.203  24.789 -16.943  1.00 24.35          C
ATOM  11303  CG   LEU G 181     -3.129  24.564 -18.134  1.00 24.98          C
ATOM  11304  CD1  LEU G 181     -4.259  23.595 -17.803  1.00 24.86          C
ATOM  11305  CD2  LEU G 181     -3.676  25.914 -18.564  1.00 25.78          C
ATOM  11306  N    THR G 182     -0.369  25.903 -14.877  1.00 25.06          N
ATOM  11307  CA   THR G 182      0.072  25.641 -13.514  1.00 25.35          C
ATOM  11308  C    THR G 182     -0.837  24.568 -12.927  1.00 25.58          C
ATOM  11309  O    THR G 182     -1.963  24.319 -13.418  1.00 23.70          O
ATOM  11310  CB   THR G 182      0.020  26.902 -12.621  1.00 25.97          C
ATOM  11311  OG1  THR G 182     -1.334  27.368 -12.497  1.00 27.31          O
ATOM  11312  CG2  THR G 182      0.868  27.988 -13.199  1.00 25.98          C
ATOM  11313  N    LYS G 183     -0.364  23.929 -11.868  1.00 24.74          N
ATOM  11314  CA   LYS G 183     -1.194  22.976 -11.154  1.00 25.30          C
ATOM  11315  C    LYS G 183     -2.496  23.624 -10.703  1.00 24.81          C
ATOM  11316  O    LYS G 183     -3.545  23.006 -10.785  1.00 23.61          O
ATOM  11317  CB   LYS G 183     -0.431  22.397  -9.965  1.00 25.31          C
ATOM  11318  CG   LYS G 183     -1.171  21.306  -9.218  1.00 25.18          C
ATOM  11319  CD   LYS G 183     -0.503  20.968  -7.889  1.00 25.62          C
ATOM  11320  CE   LYS G 183     -0.669  22.126  -6.847  1.00 26.75          C
ATOM  11321  NZ   LYS G 183      0.061  21.874  -5.565  1.00 25.73          N
ATOM  11322  N    ASP G 184     -2.433  24.896 -10.297  1.00 26.09          N
ATOM  11323  CA   ASP G 184     -3.574  25.596  -9.684  1.00 27.46          C
ATOM  11324  C    ASP G 184     -4.638  26.000 -10.714  1.00 28.26          C
ATOM  11325  O    ASP G 184     -5.836  25.853 -10.470  1.00 28.06          O
ATOM  11326  CB   ASP G 184     -3.061  26.837  -8.935  1.00 28.88          C
ATOM  11327  CG   ASP G 184     -1.838  26.522  -8.075  1.00 32.02          C
ATOM  11328  OD1  ASP G 184     -2.051  26.125  -6.908  1.00 32.47          O
ATOM  11329  OD2  ASP G 184     -0.671  26.603  -8.604  1.00 35.43          O
ATOM  11330  N    GLU G 185     -4.193  26.524 -11.851  1.00 29.63          N
ATOM  11331  CA   GLU G 185     -5.087  26.769 -12.984  1.00 29.19          C
ATOM  11332  C    GLU G 185     -5.724  25.451 -13.481  1.00 27.91          C
ATOM  11333  O    GLU G 185     -6.924  25.382 -13.744  1.00 29.03          O
ATOM  11334  CB   GLU G 185     -4.338  27.454 -14.124  1.00 30.98          C
ATOM  11335  CG   GLU G 185     -5.235  27.853 -15.331  1.00 34.90          C
ATOM  11336  CD   GLU G 185     -6.096  29.104 -15.105  1.00 39.06          C
ATOM  11337  OE1  GLU G 185     -6.498  29.378 -13.953  1.00 43.13          O
ATOM  11338  OE2  GLU G 185     -6.401  29.815 -16.096  1.00 42.35          O
ATOM  11339  N    TYR G 186     -4.934  24.402 -13.599  1.00 25.31          N
ATOM  11340  CA   TYR G 186     -5.487  23.125 -14.006  1.00 25.55          C
ATOM  11341  C    TYR G 186     -6.520  22.572 -13.011  1.00 26.32          C
ATOM  11342  O    TYR G 186     -7.589  22.070 -13.405  1.00 25.22          O
ATOM  11343  CB   TYR G 186     -4.365  22.102 -14.222  1.00 23.24          C
ATOM  11344  CG   TYR G 186     -4.890  20.701 -14.497  1.00 21.78          C
ATOM  11345  CD1  TYR G 186     -5.564  20.406 -15.679  1.00 19.84          C
ATOM  11346  CD2  TYR G 186     -4.702  19.673 -13.587  1.00 22.41          C
ATOM  11347  CE1  TYR G 186     -6.058  19.102 -15.923  1.00 20.63          C
ATOM  11348  CE2  TYR G 186     -5.178  18.397 -13.834  1.00 19.55          C
ATOM  11349  CZ   TYR G 186     -5.856  18.127 -15.002  1.00 20.60          C
ATOM  11350  OH   TYR G 186     -6.324  16.841 -15.256  1.00 22.93          O
ATOM  11351  N    GLU G 187     -6.194  22.616 -11.724  1.00 26.84          N
ATOM  11352  CA   GLU G 187     -7.087  22.062 -10.697  1.00 28.86          C
ATOM  11353  C    GLU G 187     -8.349  22.907 -10.417  1.00 29.48          C
ATOM  11354  O    GLU G 187     -9.255  22.466  -9.708  1.00 29.30          O
ATOM  11355  CB   GLU G 187     -6.306  21.783  -9.408  1.00 28.33          C
ATOM  11356  CG   GLU G 187     -5.401  20.560  -9.550  1.00 30.15          C
ATOM  11357  CD   GLU G 187     -4.510  20.293  -8.328  1.00 32.27          C
ATOM  11358  OE1  GLU G 187     -4.323  21.190  -7.458  1.00 33.06          O
ATOM  11359  OE2  GLU G 187     -3.976  19.161  -8.253  1.00 34.31          O
ATOM  11360  N    ARG G 188     -8.399  24.103 -10.983  1.00 30.67          N
ATOM  11361  CA   ARG G 188     -9.544  25.009 -10.841  1.00 31.94          C
ATOM  11362  C    ARG G 188    -10.720  24.626 -11.726  1.00 31.82          C
ATOM  11363  O    ARG G 188    -11.867  24.931 -11.400  1.00 32.63          O
```

```
ATOM  11364  CB   ARG G 188      -9.131  26.446 -11.181  1.00 33.03          C
ATOM  11365  CG   ARG G 188      -8.565  27.256 -10.003  1.00 36.16          C
ATOM  11366  CD   ARG G 188      -8.621  28.767 -10.253  1.00 37.03          C
ATOM  11367  NE   ARG G 188      -7.497  29.284 -11.041  1.00 39.92          N
ATOM  11368  CZ   ARG G 188      -6.282  29.545 -10.553  1.00 40.29          C
ATOM  11369  NH1  ARG G 188      -6.008  29.331  -9.263  1.00 41.19          N
ATOM  11370  NH2  ARG G 188      -5.326  30.014 -11.355  1.00 39.69          N
ATOM  11371  N    HIS G 189     -10.430  23.969 -12.848  1.00 30.92          N
ATOM  11372  CA   HIS G 189     -11.433  23.634 -13.850  1.00 29.95          C
ATOM  11373  C    HIS G 189     -11.623  22.136 -13.915  1.00 28.51          C
ATOM  11374  O    HIS G 189     -10.913  21.399 -13.252  1.00 27.52          O
ATOM  11375  CB   HIS G 189     -10.989  24.180 -15.195  1.00 30.96          C
ATOM  11376  CG   HIS G 189     -10.737  25.649 -15.173  1.00 32.32          C
ATOM  11377  ND1  HIS G 189     -11.753  26.573 -15.066  1.00 34.14          N
ATOM  11378  CD2  HIS G 189      -9.582  26.356 -15.207  1.00 33.47          C
ATOM  11379  CE1  HIS G 189     -11.236  27.790 -15.058  1.00 33.57          C
ATOM  11380  NE2  HIS G 189      -9.921  27.686 -15.138  1.00 33.96          N
ATOM  11381  N    ASN G 190     -12.586  21.683 -14.704  1.00 28.29          N
ATOM  11382  CA   ASN G 190     -12.907  20.248 -14.747  1.00 28.85          C
ATOM  11383  C    ASN G 190     -12.917  19.613 -16.110  1.00 28.16          C
ATOM  11384  O    ASN G 190     -12.384  18.515 -16.283  1.00 29.35          O
ATOM  11385  CB   ASN G 190     -14.265  20.001 -14.111  1.00 30.88          C
ATOM  11386  CG   ASN G 190     -14.158  19.252 -12.848  1.00 34.09          C
ATOM  11387  OD1  ASN G 190     -14.259  19.835 -11.756  1.00 38.32          O
ATOM  11388  ND2  ASN G 190     -13.902  17.944 -12.960  1.00 35.75          N
ATOM  11389  N    SER G 191     -13.564  20.274 -17.058  1.00 27.36          N
ATOM  11390  CA   SER G 191     -13.750  19.694 -18.383  1.00 26.65          C
ATOM  11391  C    SER G 191     -12.639  20.205 -19.277  1.00 25.14          C
ATOM  11392  O    SER G 191     -12.418  21.403 -19.333  1.00 22.89          O
ATOM  11393  CB   SER G 191     -15.114  20.083 -18.961  1.00 27.01          C
ATOM  11394  OG   SER G 191     -15.196  19.803 -20.355  1.00 28.16          O
ATOM  11395  N    TYR G 192     -11.945  19.281 -19.944  1.00 23.01          N
ATOM  11396  CA   TYR G 192     -10.907  19.633 -20.891  1.00 22.86          C
ATOM  11397  C    TYR G 192     -11.178  18.963 -22.213  1.00 21.00          C
ATOM  11398  O    TYR G 192     -11.342  17.738 -22.270  1.00 19.54          O
ATOM  11399  CB   TYR G 192      -9.519  19.240 -20.366  1.00 23.26          C
ATOM  11400  CG   TYR G 192      -9.077  20.127 -19.231  1.00 23.76          C
ATOM  11401  CD1  TYR G 192      -8.416  21.319 -19.488  1.00 24.90          C
ATOM  11402  CD2  TYR G 192      -9.396  19.819 -17.905  1.00 24.30          C
ATOM  11403  CE1  TYR G 192      -8.039  22.171 -18.454  1.00 23.83          C
ATOM  11404  CE2  TYR G 192      -9.003  20.653 -16.862  1.00 23.37          C
ATOM  11405  CZ   TYR G 192      -8.340  21.840 -17.156  1.00 23.04          C
ATOM  11406  OH   TYR G 192      -7.949  22.704 -16.156  1.00 24.39          O
ATOM  11407  N    THR G 193     -11.167  19.773 -23.268  1.00 21.90          N
ATOM  11408  CA   THR G 193     -11.548  19.339 -24.600  1.00 22.68          C
ATOM  11409  C    THR G 193     -10.573  19.830 -25.660  1.00 22.88          C
ATOM  11410  O    THR G 193     -10.183  20.998 -25.674  1.00 21.48          O
ATOM  11411  CB   THR G 193     -12.946  19.856 -24.921  1.00 23.00          C
ATOM  11412  OG1  THR G 193     -13.850  19.341 -23.938  1.00 24.85          O
ATOM  11413  CG2  THR G 193     -13.418  19.412 -26.306  1.00 23.54          C
ATOM  11414  N    CYS G 194     -10.204  18.906 -26.539  1.00 23.90          N
ATOM  11415  CA   CYS G 194      -9.432  19.159 -27.728  1.00 23.22          C
ATOM  11416  C    CYS G 194     -10.374  18.933 -28.925  1.00 21.62          C
ATOM  11417  O    CYS G 194     -10.945  17.865 -29.049  1.00 22.63          O
ATOM  11418  CB   CYS G 194      -8.283  18.142 -27.758  1.00 24.49          C
ATOM  11419  SG   CYS G 194      -7.200  18.255 -29.138  1.00 30.94          S
ATOM  11420  N    GLU G 195     -10.546  19.936 -29.777  1.00 21.14          N
ATOM  11421  CA   GLU G 195     -11.385  19.813 -30.977  1.00 22.73          C
ATOM  11422  C    GLU G 195     -10.579  19.981 -32.260  1.00 21.99          C
ATOM  11423  O    GLU G 195      -9.972  21.040 -32.491  1.00 22.15          O
ATOM  11424  CB   GLU G 195     -12.503  20.838 -30.989  1.00 23.40          C
ATOM  11425  CG   GLU G 195     -13.440  20.793 -29.804  1.00 24.82          C
ATOM  11426  CD   GLU G 195     -14.707  21.621 -30.052  1.00 25.24          C
ATOM  11427  OE1  GLU G 195     -14.794  22.294 -31.097  1.00 29.80          O
ATOM  11428  OE2  GLU G 195     -15.600  21.611 -29.196  1.00 28.29          O
```

```
ATOM  11429  N    ALA G 196     -10.585  18.943 -33.099  1.00 20.66        N
ATOM  11430  CA   ALA G 196      -9.817  18.952 -34.327  1.00 21.53        C
ATOM  11431  C    ALA G 196     -10.693  19.158 -35.582  1.00 21.44        C
ATOM  11432  O    ALA G 196     -11.619  18.388 -35.871  1.00 22.90        O
ATOM  11433  CB   ALA G 196      -9.016  17.650 -34.456  1.00 22.04        C
ATOM  11434  N    THR G 197     -10.356  20.168 -36.362  1.00 22.03        N
ATOM  11435  CA   THR G 197     -11.101  20.472 -37.573  1.00 21.21        C
ATOM  11436  C    THR G 197     -10.186  20.315 -38.793  1.00 20.51        C
ATOM  11437  O    THR G 197      -9.099  20.909 -38.872  1.00 19.80        O
ATOM  11438  CB   THR G 197     -11.665  21.919 -37.504  1.00 21.76        C
ATOM  11439  OG1  THR G 197     -12.366  22.077 -36.269  1.00 23.22        O
ATOM  11440  CG2  THR G 197     -12.638  22.196 -38.645  1.00 22.92        C
ATOM  11441  N    HIS G 198     -10.643  19.503 -39.744  1.00 19.71        N
ATOM  11442  CA   HIS G 198      -9.836  19.106 -40.871  1.00 19.72        C
ATOM  11443  C    HIS G 198     -10.790  18.946 -42.047  1.00 19.76        C
ATOM  11444  O    HIS G 198     -11.985  18.742 -41.860  1.00 19.79        O
ATOM  11445  CB   HIS G 198      -9.145  17.770 -40.553  1.00 19.90        C
ATOM  11446  CG   HIS G 198      -8.140  17.347 -41.576  1.00 18.92        C
ATOM  11447  ND1  HIS G 198      -8.370  16.327 -42.472  1.00 18.65        N
ATOM  11448  CD2  HIS G 198      -6.889  17.802 -41.835  1.00 20.55        C
ATOM  11449  CE1  HIS G 198      -7.318  16.196 -43.257  1.00 15.59        C
ATOM  11450  NE2  HIS G 198      -6.393  17.057 -42.873  1.00 20.14        N
ATOM  11451  N    LYS G 199     -10.265  19.010 -43.255  1.00 19.67        N
ATOM  11452  CA   LYS G 199     -11.143  19.142 -44.417  1.00 20.81        C
ATOM  11453  C    LYS G 199     -11.982  17.900 -44.670  1.00 19.33        C
ATOM  11454  O    LYS G 199     -12.967  17.963 -45.405  1.00 18.55        O
ATOM  11455  CB   LYS G 199     -10.348  19.523 -45.660  1.00 22.45        C
ATOM  11456  CG   LYS G 199      -9.600  18.415 -46.320  1.00 22.91        C
ATOM  11457  CD   LYS G 199      -8.954  18.851 -47.633  1.00 24.04        C
ATOM  11458  CE   LYS G 199      -9.977  19.164 -48.728  1.00 24.61        C
ATOM  11459  NZ   LYS G 199      -9.306  19.404 -50.039  1.00 24.40        N
ATOM  11460  N    THR G 200     -11.594  16.784 -44.055  1.00 19.42        N
ATOM  11461  CA   THR G 200     -12.325  15.530 -44.162  1.00 19.71        C
ATOM  11462  C    THR G 200     -13.719  15.526 -43.473  1.00 19.71        C
ATOM  11463  O    THR G 200     -14.503  14.585 -43.642  1.00 17.34        O
ATOM  11464  CB   THR G 200     -11.462  14.397 -43.562  1.00 19.30        C
ATOM  11465  OG1  THR G 200     -10.995  14.792 -42.266  1.00 20.15        O
ATOM  11466  CG2  THR G 200     -10.261  14.114 -44.435  1.00 21.21        C
ATOM  11467  N    SER G 201     -14.022  16.546 -42.679  1.00 19.19        N
ATOM  11468  CA   SER G 201     -15.409  16.738 -42.190  1.00 20.08        C
ATOM  11469  C    SER G 201     -15.651  18.213 -41.954  1.00 21.32        C
ATOM  11470  O    SER G 201     -14.729  18.951 -41.657  1.00 21.35        O
ATOM  11471  CB   SER G 201     -15.688  15.972 -40.893  1.00 20.02        C
ATOM  11472  OG   SER G 201     -17.058  16.040 -40.491  1.00 17.63        O
ATOM  11473  N    THR G 202     -16.898  18.635 -42.101  1.00 21.76        N
ATOM  11474  CA   THR G 202     -17.292  19.967 -41.674  1.00 22.01        C
ATOM  11475  C    THR G 202     -17.590  19.965 -40.152  1.00 23.42        C
ATOM  11476  O    THR G 202     -17.791  21.011 -39.573  1.00 25.04        O
ATOM  11477  CB   THR G 202     -18.533  20.497 -42.457  1.00 21.31        C
ATOM  11478  OG1  THR G 202     -19.686  19.749 -42.067  1.00 18.69        O
ATOM  11479  CG2  THR G 202     -18.354  20.358 -43.990  1.00 18.73        C
ATOM  11480  N    SER G 203     -17.642  18.802 -39.506  1.00 22.88        N
ATOM  11481  CA   SER G 203     -17.801  18.756 -38.047  1.00 23.54        C
ATOM  11482  C    SER G 203     -16.428  18.500 -37.402  1.00 23.89        C
ATOM  11483  O    SER G 203     -15.702  17.618 -37.865  1.00 25.44        O
ATOM  11484  CB   SER G 203     -18.717  17.601 -37.628  1.00 23.33        C
ATOM  11485  OG   SER G 203     -20.020  17.738 -38.097  1.00 25.49        O
ATOM  11486  N    PRO G 204     -16.107  19.174 -36.276  1.00 24.21        N
ATOM  11487  CA   PRO G 204     -14.842  18.823 -35.617  1.00 24.70        C
ATOM  11488  C    PRO G 204     -14.851  17.454 -34.940  1.00 24.67        C
ATOM  11489  O    PRO G 204     -15.921  16.914 -34.605  1.00 23.83        O
ATOM  11490  CB   PRO G 204     -14.662  19.952 -34.581  1.00 24.56        C
ATOM  11491  CG   PRO G 204     -16.030  20.365 -34.243  1.00 24.60        C
ATOM  11492  CD   PRO G 204     -16.798  20.266 -35.553  1.00 24.62        C
ATOM  11493  N    ILE G 205     -13.668  16.878 -34.787  1.00 24.82        N
```

```
ATOM  11494  CA   ILE G 205    -13.493  15.693 -33.951  1.00 25.31           C
ATOM  11495  C    ILE G 205    -13.241  16.247 -32.548  1.00 25.00           C
ATOM  11496  O    ILE G 205    -12.339  17.038 -32.364  1.00 24.62           O
ATOM  11497  CB   ILE G 205    -12.330  14.782 -34.459  1.00 25.12           C
ATOM  11498  CG1  ILE G 205    -12.671  14.231 -35.846  1.00 25.53           C
ATOM  11499  CG2  ILE G 205    -12.059  13.622 -33.478  1.00 24.99           C
ATOM  11500  CD1  ILE G 205    -11.529  13.452 -36.523  1.00 26.48           C
ATOM  11501  N    VAL G 206    -14.092  15.868 -31.599  1.00 25.49           N
ATOM  11502  CA   VAL G 206    -14.043  16.359 -30.219  1.00 25.39           C
ATOM  11503  C    VAL G 206    -13.610  15.246 -29.270  1.00 24.87           C
ATOM  11504  O    VAL G 206    -14.230  14.181 -29.236  1.00 24.70           O
ATOM  11505  CB   VAL G 206    -15.414  16.859 -29.766  1.00 26.51           C
ATOM  11506  CG1  VAL G 206    -15.371  17.323 -28.309  1.00 27.53           C
ATOM  11507  CG2  VAL G 206    -15.890  17.998 -30.662  1.00 27.72           C
ATOM  11508  N    LYS G 207    -12.532  15.478 -28.517  1.00 24.73           N
ATOM  11509  CA   LYS G 207    -12.104  14.541 -27.485  1.00 24.41           C
ATOM  11510  C    LYS G 207    -11.989  15.305 -26.178  1.00 23.58           C
ATOM  11511  O    LYS G 207    -11.466  16.423 -26.149  1.00 22.26           O
ATOM  11512  CB   LYS G 207    -10.775  13.872 -27.844  1.00 25.51           C
ATOM  11513  CG   LYS G 207    -10.826  12.938 -29.036  1.00 26.01           C
ATOM  11514  CD   LYS G 207    -11.847  11.817 -28.867  1.00 28.69           C
ATOM  11515  CE   LYS G 207    -11.705  10.762 -29.979  1.00 27.86           C
ATOM  11516  NZ   LYS G 207    -12.717   9.687 -29.917  1.00 28.90           N
ATOM  11517  N    SER G 208    -12.508  14.697 -25.112  1.00 23.19           N
ATOM  11518  CA   SER G 208    -12.594  15.336 -23.814  1.00 24.05           C
ATOM  11519  C    SER G 208    -12.294  14.413 -22.660  1.00 22.87           C
ATOM  11520  O    SER G 208    -12.426  13.179 -22.774  1.00 21.84           O
ATOM  11521  CB   SER G 208    -13.996  15.881 -23.632  1.00 25.32           C
ATOM  11522  OG   SER G 208    -14.145  17.076 -24.364  1.00 28.03           O
ATOM  11523  N    PHE G 209    -11.873  15.012 -21.546  1.00 22.69           N
ATOM  11524  CA   PHE G 209    -11.876  14.322 -20.251  1.00 23.69           C
ATOM  11525  C    PHE G 209    -12.310  15.290 -19.173  1.00 23.18           C
ATOM  11526  O    PHE G 209    -12.328  16.516 -19.392  1.00 22.16           O
ATOM  11527  CB   PHE G 209    -10.506  13.717 -19.902  1.00 22.93           C
ATOM  11528  CG   PHE G 209     -9.439  14.750 -19.646  1.00 23.30           C
ATOM  11529  CD1  PHE G 209     -9.278  15.316 -18.382  1.00 22.67           C
ATOM  11530  CD2  PHE G 209     -8.593  15.168 -20.676  1.00 23.77           C
ATOM  11531  CE1  PHE G 209     -8.291  16.290 -18.137  1.00 23.44           C
ATOM  11532  CE2  PHE G 209     -7.593  16.124 -20.436  1.00 23.95           C
ATOM  11533  CZ   PHE G 209     -7.447  16.690 -19.160  1.00 23.73           C
ATOM  11534  N    ASN G 210    -12.699  14.722 -18.033  1.00 23.77           N
ATOM  11535  CA   ASN G 210    -13.003  15.496 -16.829  1.00 25.00           C
ATOM  11536  C    ASN G 210    -11.910  15.230 -15.810  1.00 25.21           C
ATOM  11537  O    ASN G 210    -11.566  14.066 -15.552  1.00 23.91           O
ATOM  11538  CB   ASN G 210    -14.362  15.085 -16.260  1.00 25.35           C
ATOM  11539  CG   ASN G 210    -15.523  15.497 -17.153  1.00 25.58           C
ATOM  11540  OD1  ASN G 210    -15.612  16.651 -17.588  1.00 26.20           O
ATOM  11541  ND2  ASN G 210    -16.427  14.569 -17.411  1.00 24.90           N
ATOM  11542  N    ARG G 211    -11.361  16.284 -15.228  1.00 25.43           N
ATOM  11543  CA   ARG G 211    -10.210  16.164 -14.315  1.00 26.68           C
ATOM  11544  C    ARG G 211    -10.457  15.271 -13.076  1.00 27.44           C
ATOM  11545  O    ARG G 211    -11.594  15.156 -12.566  1.00 29.42           O
ATOM  11546  CB   ARG G 211     -9.756  17.559 -13.885  1.00 26.39           C
ATOM  11547  CG   ARG G 211     -8.655  17.572 -12.823  1.00 27.42           C
ATOM  11548  CD   ARG G 211     -8.385  18.979 -12.325  1.00 28.37           C
ATOM  11549  NE   ARG G 211     -9.577  19.508 -11.683  1.00 28.87           N
ATOM  11550  CZ   ARG G 211     -9.966  19.222 -10.443  1.00 30.09           C
ATOM  11551  NH1  ARG G 211     -9.256  18.409  -9.656  1.00 30.43           N
ATOM  11552  NH2  ARG G 211    -11.083  19.767  -9.980  1.00 31.57           N
TER   11553       ARG G 211
ATOM  11554  N    GLU H   1     29.606  -3.850 -46.143  1.00 32.50           N
ATOM  11555  CA   GLU H   1     28.848  -2.557 -46.223  1.00 32.04           C
ATOM  11556  C    GLU H   1     29.713  -1.470 -45.610  1.00 29.94           C
ATOM  11557  O    GLU H   1     30.355  -1.685 -44.590  1.00 28.16           O
ATOM  11558  CB   GLU H   1     27.501  -2.636 -45.479  1.00 34.37           C
```

| ATOM | 11559 | CG | GLU | H | 1 | 26.275 | -2.301 | -46.335 | 1.00 | 39.04 | C |
|------|-------|-----|-----|---|----|--------|--------|---------|------|-------|---|
| ATOM | 11560 | CD | GLU | H | 1 | 26.089 | -0.810 | -46.633 | 1.00 | 42.92 | C |
| ATOM | 11561 | OE1 | GLU | H | 1 | 27.096 | -0.078 | -46.686 | 1.00 | 47.16 | O |
| ATOM | 11562 | OE2 | GLU | H | 1 | 24.924 | -0.368 | -46.831 | 1.00 | 46.63 | O |
| ATOM | 11563 | N | VAL | H | 2 | 29.740 | -0.308 | -46.243 | 1.00 | 28.51 | N |
| ATOM | 11564 | CA | VAL | H | 2 | 30.422 | 0.847 | -45.673 | 1.00 | 27.86 | C |
| ATOM | 11565 | C | VAL | H | 2 | 29.552 | 1.364 | -44.520 | 1.00 | 26.53 | C |
| ATOM | 11566 | O | VAL | H | 2 | 28.363 | 1.644 | -44.717 | 1.00 | 26.51 | O |
| ATOM | 11567 | CB | VAL | H | 2 | 30.662 | 1.925 | -46.749 | 1.00 | 26.91 | C |
| ATOM | 11568 | CG1 | VAL | H | 2 | 31.339 | 3.153 | -46.170 | 1.00 | 26.71 | C |
| ATOM | 11569 | CG2 | VAL | H | 2 | 31.528 | 1.354 | -47.873 | 1.00 | 26.74 | C |
| ATOM | 11570 | N | LYS | H | 3 | 30.128 | 1.420 | -43.393 | 1.00 | 25.99 | N |
| ATOM | 11571 | CA | LYS | H | 3 | 29.505 | 2.127 | -42.289 | 1.00 | 26.22 | C |
| ATOM | 11572 | C | LYS | H | 3 | 30.399 | 3.269 | -41.875 | 1.00 | 23.06 | C |
| ATOM | 11573 | O | LYS | H | 3 | 31.599 | 3.089 | -41.742 | 1.00 | 21.27 | O |
| ATOM | 11574 | CB | LYS | H | 3 | 29.318 | 1.223 | -41.094 | 1.00 | 27.40 | C |
| ATOM | 11575 | CG | LYS | H | 3 | 28.730 | 1.961 | -39.887 | 1.00 | 29.17 | C |
| ATOM | 11576 | CD | LYS | H | 3 | 28.498 | 1.020 | -38.718 | 1.00 | 31.11 | C |
| ATOM | 11577 | CE | LYS | H | 3 | 28.718 | 1.710 | -37.406 | 1.00 | 31.21 | C |
| ATOM | 11578 | NZ | LYS | H | 3 | 30.171 | 1.915 | -37.106 | 1.00 | 33.23 | N |
| ATOM | 11579 | N | VAL | H | 4 | 29.780 | 4.412 | -41.601 | 1.00 | 22.04 | N |
| ATOM | 11580 | CA | VAL | H | 4 | 30.467 | 5.631 | -41.185 | 1.00 | 21.97 | C |
| ATOM | 11581 | C | VAL | H | 4 | 29.758 | 6.058 | -39.903 | 1.00 | 21.73 | C |
| ATOM | 11582 | O | VAL | H | 4 | 28.541 | 6.263 | -39.918 | 1.00 | 20.48 | O |
| ATOM | 11583 | CB | VAL | H | 4 | 30.314 | 6.726 | -42.245 | 1.00 | 20.65 | C |
| ATOM | 11584 | CG1 | VAL | H | 4 | 30.967 | 8.017 | -41.780 | 1.00 | 20.31 | C |
| ATOM | 11585 | CG2 | VAL | H | 4 | 30.894 | 6.264 | -43.582 | 1.00 | 21.49 | C |
| ATOM | 11586 | N | GLU | H | 5 | 30.485 | 6.151 | -38.793 | 1.00 | 21.98 | N |
| ATOM | 11587 | CA | GLU | H | 5 | 29.848 | 6.385 | -37.491 | 1.00 | 21.73 | C |
| ATOM | 11588 | C | GLU | H | 5 | 30.580 | 7.420 | -36.634 | 1.00 | 19.90 | C |
| ATOM | 11589 | O | GLU | H | 5 | 31.682 | 7.185 | -36.142 | 1.00 | 17.82 | O |
| ATOM | 11590 | CB | GLU | H | 5 | 29.706 | 5.101 | -36.693 | 1.00 | 22.92 | C |
| ATOM | 11591 | CG | GLU | H | 5 | 28.847 | 5.369 | -35.476 | 1.00 | 25.70 | C |
| ATOM | 11592 | CD | GLU | H | 5 | 28.455 | 4.136 | -34.689 | 1.00 | 27.68 | C |
| ATOM | 11593 | OE1 | GLU | H | 5 | 28.458 | 2.996 | -35.239 | 1.00 | 32.78 | O |
| ATOM | 11594 | OE2 | GLU | H | 5 | 28.132 | 4.337 | -33.501 | 1.00 | 33.25 | O |
| ATOM | 11595 | N | GLU | H | 6 | 29.924 | 8.562 | -36.446 | 1.00 | 18.14 | N |
| ATOM | 11596 | CA | GLU | H | 6 | 30.520 | 9.672 | -35.746 | 1.00 | 18.53 | C |
| ATOM | 11597 | C | GLU | H | 6 | 30.318 | 9.541 | -34.262 | 1.00 | 19.21 | C |
| ATOM | 11598 | O | GLU | H | 6 | 29.461 | 8.774 | -33.769 | 1.00 | 19.54 | O |
| ATOM | 11599 | CB | GLU | H | 6 | 29.930 | 11.006 | -36.212 | 1.00 | 17.77 | C |
| ATOM | 11600 | CG | GLU | H | 6 | 30.101 | 11.300 | -37.668 | 1.00 | 18.05 | C |
| ATOM | 11601 | CD | GLU | H | 6 | 29.031 | 10.733 | -38.553 | 1.00 | 17.29 | C |
| ATOM | 11602 | OE1 | GLU | H | 6 | 28.330 | 9.730 | -38.189 | 1.00 | 15.16 | O |
| ATOM | 11603 | OE2 | GLU | H | 6 | 28.876 | 11.284 | -39.658 | 1.00 | 14.69 | O |
| ATOM | 11604 | N | SER | H | 7 | 31.125 | 10.288 | -33.531 | 1.00 | 17.51 | N |
| ATOM | 11605 | CA | SER | H | 7 | 30.941 | 10.427 | -32.099 | 1.00 | 19.09 | C |
| ATOM | 11606 | C | SER | H | 7 | 31.590 | 11.708 | -31.600 | 1.00 | 17.97 | C |
| ATOM | 11607 | O | SER | H | 7 | 32.305 | 12.387 | -32.356 | 1.00 | 18.56 | O |
| ATOM | 11608 | CB | SER | H | 7 | 31.524 | 9.210 | -31.356 | 1.00 | 17.83 | C |
| ATOM | 11609 | OG | SER | H | 7 | 32.877 | 9.037 | -31.695 | 1.00 | 21.83 | O |
| ATOM | 11610 | N | GLY | H | 8 | 31.281 | 12.062 | -30.351 | 1.00 | 19.73 | N |
| ATOM | 11611 | CA | GLY | H | 8 | 31.926 | 13.179 | -29.662 | 1.00 | 18.93 | C |
| ATOM | 11612 | C | GLY | H | 8 | 31.206 | 14.517 | -29.583 | 1.00 | 20.14 | C |
| ATOM | 11613 | O | GLY | H | 8 | 31.765 | 15.502 | -29.069 | 1.00 | 23.51 | O |
| ATOM | 11614 | N | GLY | H | 9 | 29.989 | 14.588 | -30.081 | 1.00 | 19.70 | N |
| ATOM | 11615 | CA | GLY | H | 9 | 29.190 | 15.785 | -29.892 | 1.00 | 19.37 | C |
| ATOM | 11616 | C | GLY | H | 9 | 28.917 | 16.116 | -28.421 | 1.00 | 18.76 | C |
| ATOM | 11617 | O | GLY | H | 9 | 29.393 | 15.458 | -27.479 | 1.00 | 20.32 | O |
| ATOM | 11618 | N | GLY | H | 10 | 28.152 | 17.168 | -28.257 | 1.00 | 16.88 | N |
| ATOM | 11619 | CA | GLY | H | 10 | 27.727 | 17.642 | -26.979 | 1.00 | 15.34 | C |
| ATOM | 11620 | C | GLY | H | 10 | 27.713 | 19.162 | -27.038 | 1.00 | 14.38 | C |
| ATOM | 11621 | O | GLY | H | 10 | 27.615 | 19.754 | -28.097 | 1.00 | 14.85 | O |
| ATOM | 11622 | N | LEU | H | 11 | 27.869 | 19.780 | -25.888 | 1.00 | 15.38 | N |
| ATOM | 11623 | CA | LEU | H | 11 | 27.818 | 21.254 | -25.762 | 1.00 | 12.53 | C |

```
ATOM  11624  C    LEU H   11     29.138  21.854 -25.259  1.00 13.89          C
ATOM  11625  O    LEU H   11     29.805  21.328 -24.379  1.00 10.84          O
ATOM  11626  CB   LEU H   11     26.683  21.661 -24.823  1.00 13.68          C
ATOM  11627  CG   LEU H   11     26.454  23.187 -24.624  1.00 14.56          C
ATOM  11628  CD1  LEU H   11     25.001  23.478 -24.418  1.00 16.75          C
ATOM  11629  CD2  LEU H   11     27.287  23.721 -23.467  1.00 13.97          C
ATOM  11630  N    VAL H   12     29.462  23.054 -25.743  1.00 12.61          N
ATOM  11631  CA   VAL H   12     30.585  23.762 -25.168  1.00 12.91          C
ATOM  11632  C    VAL H   12     30.224  25.246 -25.280  1.00 11.14          C
ATOM  11633  O    VAL H   12     29.331  25.605 -26.029  1.00 11.02          O
ATOM  11634  CB   VAL H   12     31.942  23.423 -25.831  1.00 12.59          C
ATOM  11635  CG1  VAL H   12     32.006  23.871 -27.303  1.00 15.99          C
ATOM  11636  CG2  VAL H   12     33.115  23.959 -24.994  1.00 15.32          C
ATOM  11637  N    GLN H   13     30.940  26.060 -24.534  1.00 14.21          N
ATOM  11638  CA   GLN H   13     30.700  27.493 -24.519  1.00 12.86          C
ATOM  11639  C    GLN H   13     31.398  28.162 -25.673  1.00 14.86          C
ATOM  11640  O    GLN H   13     32.415  27.671 -26.153  1.00 14.76          O
ATOM  11641  CB   GLN H   13     31.225  28.083 -23.229  1.00 13.61          C
ATOM  11642  CG   GLN H   13     30.531  27.614 -22.006  1.00 14.22          C
ATOM  11643  CD   GLN H   13     30.954  26.195 -21.590  1.00 17.62          C
ATOM  11644  OE1  GLN H   13     32.103  25.784 -21.761  1.00 15.29          O
ATOM  11645  NE2  GLN H   13     30.007  25.458 -21.039  1.00 16.56          N
ATOM  11646  N    PRO H   14     30.875  29.322 -26.101  1.00 14.76          N
ATOM  11647  CA   PRO H   14     31.565  30.119 -27.106  1.00 15.98          C
ATOM  11648  C    PRO H   14     32.985  30.418 -26.640  1.00 14.79          C
ATOM  11649  O    PRO H   14     33.182  30.756 -25.472  1.00 15.25          O
ATOM  11650  CB   PRO H   14     30.745  31.405 -27.160  1.00 16.77          C
ATOM  11651  CG   PRO H   14     29.432  31.072 -26.572  1.00 16.55          C
ATOM  11652  CD   PRO H   14     29.677  29.995 -25.591  1.00 17.19          C
ATOM  11653  N    GLY H   15     33.965  30.253 -27.532  1.00 13.83          N
ATOM  11654  CA   GLY H   15     35.369  30.367 -27.172  1.00 13.77          C
ATOM  11655  C    GLY H   15     36.022  29.044 -26.783  1.00 15.17          C
ATOM  11656  O    GLY H   15     37.256  28.944 -26.696  1.00 15.12          O
ATOM  11657  N    GLY H   16     35.186  28.048 -26.549  1.00 13.68          N
ATOM  11658  CA   GLY H   16     35.609  26.707 -26.133  1.00 14.68          C
ATOM  11659  C    GLY H   16     35.893  25.825 -27.322  1.00 14.45          C
ATOM  11660  O    GLY H   16     35.822  26.260 -28.478  1.00 13.64          O
ATOM  11661  N    SER H   17     36.227  24.563 -27.034  1.00 15.24          N
ATOM  11662  CA   SER H   17     36.711  23.624 -28.058  1.00 14.84          C
ATOM  11663  C    SER H   17     35.945  22.346 -27.986  1.00 16.31          C
ATOM  11664  O    SER H   17     35.299  22.062 -26.994  1.00 13.88          O
ATOM  11665  CB   SER H   17     38.199  23.303 -27.864  1.00 15.73          C
ATOM  11666  OG   SER H   17     38.957  24.512 -27.976  1.00 18.79          O
ATOM  11667  N    MET H   18     36.007  21.594 -29.079  1.00 17.34          N
ATOM  11668  CA   MET H   18     35.319  20.333 -29.182  1.00 19.63          C
ATOM  11669  C    MET H   18     36.149  19.457 -30.098  1.00 18.79          C
ATOM  11670  O    MET H   18     36.743  19.960 -31.055  1.00 17.70          O
ATOM  11671  CB   MET H   18     33.963  20.595 -29.786  1.00 22.58          C
ATOM  11672  CG   MET H   18     32.923  19.683 -29.378  1.00 28.97          C
ATOM  11673  SD   MET H   18     32.318  19.969 -27.663  1.00 33.11          S
ATOM  11674  CE   MET H   18     30.530  20.032 -27.868  1.00 30.33          C
ATOM  11675  N    LYS H   19     36.220  18.164 -29.804  1.00 19.00          N
ATOM  11676  CA   LYS H   19     36.866  17.237 -30.719  1.00 18.04          C
ATOM  11677  C    LYS H   19     35.860  16.152 -31.039  1.00 16.58          C
ATOM  11678  O    LYS H   19     35.351  15.477 -30.145  1.00 16.91          O
ATOM  11679  CB   LYS H   19     38.169  16.628 -30.162  1.00 19.70          C
ATOM  11680  CG   LYS H   19     39.008  15.957 -31.222  1.00 20.60          C
ATOM  11681  CD   LYS H   19     40.327  15.439 -30.694  1.00 21.51          C
ATOM  11682  CE   LYS H   19     41.043  14.628 -31.744  1.00 22.76          C
ATOM  11683  NZ   LYS H   19     42.538  14.699 -31.657  1.00 24.12          N
ATOM  11684  N    ILE H   20     35.559  16.006 -32.316  1.00 15.11          N
ATOM  11685  CA   ILE H   20     34.625  14.995 -32.772  1.00 15.27          C
ATOM  11686  C    ILE H   20     35.410  14.044 -33.659  1.00 15.32          C
ATOM  11687  O    ILE H   20     36.509  14.378 -34.123  1.00 14.80          O
ATOM  11688  CB   ILE H   20     33.378  15.608 -33.502  1.00 14.74          C
```

```
ATOM   11689  CG1 ILE H  20      33.757  16.391 -34.755  1.00 15.18           C
ATOM   11690  CG2 ILE H  20      32.521  16.477 -32.532  1.00 16.72           C
ATOM   11691  CD1 ILE H  20      32.559  16.872 -35.582  1.00 14.24           C
ATOM   11692  N   SER H  21      34.858  12.850 -33.876  1.00 16.32           N
ATOM   11693  CA  SER H  21      35.519  11.843 -34.694  1.00 16.14           C
ATOM   11694  C   SER H  21      34.547  10.938 -35.394  1.00 14.37           C
ATOM   11695  O   SER H  21      33.349  10.944 -35.111  1.00 11.82           O
ATOM   11696  CB  SER H  21      36.482  10.989 -33.865  1.00 17.37           C
ATOM   11697  OG  SER H  21      35.802  10.287 -32.853  1.00 17.80           O
ATOM   11698  N   CYS H  22      35.068  10.167 -36.344  1.00 16.00           N
ATOM   11699  CA  CYS H  22      34.301   9.063 -36.888  1.00 16.98           C
ATOM   11700  C   CYS H  22      35.156   7.884 -37.292  1.00 15.86           C
ATOM   11701  O   CYS H  22      36.319   8.012 -37.574  1.00 14.74           O
ATOM   11702  CB  CYS H  22      33.400   9.504 -38.029  1.00 19.51           C
ATOM   11703  SG  CYS H  22      34.188   9.920 -39.524  1.00 25.30           S
ATOM   11704  N   VAL H  23      34.519   6.734 -37.313  1.00 17.20           N
ATOM   11705  CA  VAL H  23      35.176   5.501 -37.672  1.00 17.11           C
ATOM   11706  C   VAL H  23      34.437   4.884 -38.856  1.00 15.67           C
ATOM   11707  O   VAL H  23      33.201   4.784 -38.882  1.00 16.39           O
ATOM   11708  CB  VAL H  23      35.285   4.560 -36.471  1.00 17.82           C
ATOM   11709  CG1 VAL H  23      33.934   4.331 -35.838  1.00 18.71           C
ATOM   11710  CG2 VAL H  23      35.978   3.281 -36.868  1.00 19.67           C
ATOM   11711  N   VAL H  24      35.222   4.521 -39.857  1.00 15.60           N
ATOM   11712  CA  VAL H  24      34.703   3.957 -41.059  1.00 16.39           C
ATOM   11713  C   VAL H  24      35.112   2.489 -41.123  1.00 17.10           C
ATOM   11714  O   VAL H  24      36.248   2.151 -40.823  1.00 18.23           O
ATOM   11715  CB  VAL H  24      35.212   4.690 -42.305  1.00 15.10           C
ATOM   11716  CG1 VAL H  24      34.492   4.177 -43.516  1.00 14.27           C
ATOM   11717  CG2 VAL H  24      34.978   6.199 -42.185  1.00 15.47           C
ATOM   11718  N   SER H  25      34.145   1.643 -41.447  1.00 19.65           N
ATOM   11719  CA  SER H  25      34.383   0.237 -41.764  1.00 20.00           C
ATOM   11720  C   SER H  25      33.810  -0.108 -43.138  1.00 20.79           C
ATOM   11721  O   SER H  25      32.949   0.602 -43.680  1.00 19.07           O
ATOM   11722  CB  SER H  25      33.785  -0.684 -40.684  1.00 20.78           C
ATOM   11723  OG  SER H  25      32.417  -0.430 -40.435  1.00 23.66           O
ATOM   11724  N   GLY H  26      34.292  -1.220 -43.692  1.00 22.04           N
ATOM   11725  CA  GLY H  26      33.774  -1.765 -44.939  1.00 21.24           C
ATOM   11726  C   GLY H  26      34.423  -1.219 -46.185  1.00 20.42           C
ATOM   11727  O   GLY H  26      33.874  -1.344 -47.276  1.00 23.35           O
ATOM   11728  N   LEU H  27      35.544  -0.536 -46.012  1.00 21.18           N
ATOM   11729  CA  LEU H  27      36.385  -0.100 -47.122  1.00 21.08           C
ATOM   11730  C   LEU H  27      37.754   0.161 -46.542  1.00 20.41           C
ATOM   11731  O   LEU H  27      37.916   0.123 -45.331  1.00 22.22           O
ATOM   11732  CB  LEU H  27      35.833   1.163 -47.824  1.00 21.72           C
ATOM   11733  CG  LEU H  27      35.457   2.378 -46.968  1.00 20.42           C
ATOM   11734  CD1 LEU H  27      36.662   3.012 -46.325  1.00 20.46           C
ATOM   11735  CD2 LEU H  27      34.697   3.369 -47.840  1.00 20.44           C
ATOM   11736  N   THR H  28      38.733   0.418 -47.393  1.00 20.63           N
ATOM   11737  CA  THR H  28      40.089   0.732 -46.924  1.00 19.75           C
ATOM   11738  C   THR H  28      40.208   2.232 -46.662  1.00 19.44           C
ATOM   11739  O   THR H  28      40.478   3.008 -47.574  1.00 19.27           O
ATOM   11740  CB  THR H  28      41.147   0.243 -47.914  1.00 20.51           C
ATOM   11741  OG1 THR H  28      40.966  -1.171 -48.111  1.00 20.76           O
ATOM   11742  CG2 THR H  28      42.564   0.480 -47.371  1.00 19.96           C
ATOM   11743  N   PHE H  29      40.027   2.596 -45.389  1.00 19.92           N
ATOM   11744  CA  PHE H  29      39.996   3.984 -44.932  1.00 19.42           C
ATOM   11745  C   PHE H  29      41.126   4.777 -45.529  1.00 20.30           C
ATOM   11746  O   PHE H  29      40.907   5.883 -46.034  1.00 21.17           O
ATOM   11747  CB  PHE H  29      40.060   4.022 -43.402  1.00 18.68           C
ATOM   11748  CG  PHE H  29      40.087   5.411 -42.804  1.00 19.85           C
ATOM   11749  CD1 PHE H  29      38.921   6.024 -42.383  1.00 19.34           C
ATOM   11750  CD2 PHE H  29      41.280   6.091 -42.620  1.00 16.55           C
ATOM   11751  CE1 PHE H  29      38.950   7.299 -41.810  1.00 19.23           C
ATOM   11752  CE2 PHE H  29      41.295   7.381 -42.042  1.00 18.52           C
ATOM   11753  CZ  PHE H  29      40.138   7.959 -41.628  1.00 16.49           C
```

| ATOM | 11754 | N | SER | H | 30 | 42.331 | 4.208 | -45.512 | 1.00 | 19.59 | N |
|------|-------|-----|-----|---|----|--------|--------|---------|------|-------|---|
| ATOM | 11755 | CA | SER | H | 30 | 43.523 | 4.919 | -45.972 | 1.00 | 19.55 | C |
| ATOM | 11756 | C | SER | H | 30 | 43.516 | 5.315 | -47.448 | 1.00 | 19.18 | C |
| ATOM | 11757 | O | SER | H | 30 | 44.318 | 6.147 | -47.863 | 1.00 | 21.11 | O |
| ATOM | 11758 | CB | SER | H | 30 | 44.784 | 4.092 | -45.654 | 1.00 | 20.97 | C |
| ATOM | 11759 | OG | SER | H | 30 | 44.785 | 2.927 | -46.432 | 1.00 | 23.98 | O |
| ATOM | 11760 | N | ASN | H | 31 | 42.604 | 4.758 | -48.237 | 1.00 | 17.24 | N |
| ATOM | 11761 | CA | ASN | H | 31 | 42.579 | 5.034 | -49.663 | 1.00 | 16.82 | C |
| ATOM | 11762 | C | ASN | H | 31 | 41.584 | 6.115 | -50.040 | 1.00 | 16.03 | C |
| ATOM | 11763 | O | ASN | H | 31 | 41.537 | 6.473 | -51.199 | 1.00 | 14.91 | O |
| ATOM | 11764 | CB | ASN | H | 31 | 42.228 | 3.789 | -50.493 | 1.00 | 17.32 | C |
| ATOM | 11765 | CG | ASN | H | 31 | 43.320 | 2.732 | -50.475 | 1.00 | 19.69 | C |
| ATOM | 11766 | OD1 | ASN | H | 31 | 43.060 | 1.568 | -50.810 | 1.00 | 22.34 | O |
| ATOM | 11767 | ND2 | ASN | H | 31 | 44.527 | 3.120 | -50.119 | 1.00 | 17.13 | N |
| ATOM | 11768 | N | TYR | H | 32 | 40.808 | 6.624 | -49.076 | 1.00 | 14.75 | N |
| ATOM | 11769 | CA | TYR | H | 32 | 39.735 | 7.547 | -49.408 | 1.00 | 15.32 | C |
| ATOM | 11770 | C | TYR | H | 32 | 39.953 | 8.914 | -48.848 | 1.00 | 13.14 | C |
| ATOM | 11771 | O | TYR | H | 32 | 40.442 | 9.056 | -47.738 | 1.00 | 11.97 | O |
| ATOM | 11772 | CB | TYR | H | 32 | 38.422 | 7.038 | -48.874 | 1.00 | 18.01 | C |
| ATOM | 11773 | CG | TYR | H | 32 | 37.837 | 5.923 | -49.681 | 1.00 | 20.01 | C |
| ATOM | 11774 | CD1 | TYR | H | 32 | 36.779 | 6.152 | -50.549 | 1.00 | 19.87 | C |
| ATOM | 11775 | CD2 | TYR | H | 32 | 38.334 | 4.631 | -49.565 | 1.00 | 20.13 | C |
| ATOM | 11776 | CE1 | TYR | H | 32 | 36.232 | 5.143 | -51.283 | 1.00 | 20.25 | C |
| ATOM | 11777 | CE2 | TYR | H | 32 | 37.790 | 3.583 | -50.331 | 1.00 | 21.41 | C |
| ATOM | 11778 | CZ | TYR | H | 32 | 36.730 | 3.861 | -51.183 | 1.00 | 20.77 | C |
| ATOM | 11779 | OH | TYR | H | 32 | 36.112 | 2.861 | -51.927 | 1.00 | 22.81 | O |
| ATOM | 11780 | N | TRP | H | 33 | 39.502 | 9.922 | -49.608 | 1.00 | 13.49 | N |
| ATOM | 11781 | CA | TRP | H | 33 | 39.436 | 11.269 | -49.092 | 1.00 | 13.25 | C |
| ATOM | 11782 | C | TRP | H | 33 | 38.330 | 11.361 | -48.020 | 1.00 | 11.86 | C |
| ATOM | 11783 | O | TRP | H | 33 | 37.322 | 10.660 | -48.060 | 1.00 | 12.65 | O |
| ATOM | 11784 | CB | TRP | H | 33 | 39.138 | 12.267 | -50.235 | 1.00 | 14.24 | C |
| ATOM | 11785 | CG | TRP | H | 33 | 40.152 | 12.280 | -51.375 | 1.00 | 15.54 | C |
| ATOM | 11786 | CD1 | TRP | H | 33 | 41.416 | 11.783 | -51.369 | 1.00 | 15.06 | C |
| ATOM | 11787 | CD2 | TRP | H | 33 | 40.006 | 12.968 | -52.618 | 1.00 | 17.68 | C |
| ATOM | 11788 | NE1 | TRP | H | 33 | 42.028 | 12.016 | -52.579 | 1.00 | 15.53 | N |
| ATOM | 11789 | CE2 | TRP | H | 33 | 41.187 | 12.749 | -53.363 | 1.00 | 16.48 | C |
| ATOM | 11790 | CE3 | TRP | H | 33 | 38.965 | 13.698 | -53.197 | 1.00 | 17.41 | C |
| ATOM | 11791 | CZ2 | TRP | H | 33 | 41.366 | 13.245 | -54.647 | 1.00 | 16.61 | C |
| ATOM | 11792 | CZ3 | TRP | H | 33 | 39.141 | 14.196 | -54.460 | 1.00 | 17.04 | C |
| ATOM | 11793 | CH2 | TRP | H | 33 | 40.329 | 13.962 | -55.183 | 1.00 | 17.03 | C |
| ATOM | 11794 | N | MET | H | 34 | 38.530 | 12.251 | -47.054 | 1.00 | 13.12 | N |
| ATOM | 11795 | CA | MET | H | 34 | 37.643 | 12.385 | -45.946 | 1.00 | 12.73 | C |
| ATOM | 11796 | C | MET | H | 34 | 37.292 | 13.870 | -45.774 | 1.00 | 11.89 | C |
| ATOM | 11797 | O | MET | H | 34 | 38.138 | 14.742 | -45.905 | 1.00 | 13.96 | O |
| ATOM | 11798 | CB | MET | H | 34 | 38.308 | 11.927 | -44.651 | 1.00 | 14.62 | C |
| ATOM | 11799 | CG | MET | H | 34 | 38.696 | 10.438 | -44.610 | 1.00 | 14.04 | C |
| ATOM | 11800 | SD | MET | H | 34 | 37.230 | 9.504 | -44.327 | 1.00 | 18.36 | S |
| ATOM | 11801 | CE | MET | H | 34 | 37.617 | 7.923 | -45.139 | 1.00 | 16.07 | C |
| ATOM | 11802 | N | SER | H | 35 | 36.051 | 14.123 | -45.425 | 1.00 | 11.64 | N |
| ATOM | 11803 | CA | SER | H | 35 | 35.609 | 15.507 | -45.165 | 1.00 | 11.34 | C |
| ATOM | 11804 | C | SER | H | 35 | 34.608 | 15.553 | -44.035 | 1.00 | 11.81 | C |
| ATOM | 11805 | O | SER | H | 35 | 34.031 | 14.541 | -43.650 | 1.00 | 9.86 | O |
| ATOM | 11806 | CB | SER | H | 35 | 35.034 | 16.140 | -46.436 | 1.00 | 12.09 | C |
| ATOM | 11807 | OG | SER | H | 35 | 33.674 | 15.780 | -46.658 | 1.00 | 13.78 | O |
| ATOM | 11808 | N | TRP | H | 36 | 34.366 | 16.772 | -43.559 | 1.00 | 8.60 | N |
| ATOM | 11809 | CA | TRP | H | 36 | 33.306 | 17.110 | -42.620 | 1.00 | 11.25 | C |
| ATOM | 11810 | C | TRP | H | 36 | 32.363 | 18.098 | -43.284 | 1.00 | 9.70 | C |
| ATOM | 11811 | O | TRP | H | 36 | 32.813 | 19.056 | -43.917 | 1.00 | 11.87 | O |
| ATOM | 11812 | CB | TRP | H | 36 | 33.849 | 17.690 | -41.309 | 1.00 | 10.27 | C |
| ATOM | 11813 | CG | TRP | H | 36 | 34.582 | 16.694 | -40.463 | 1.00 | 10.93 | C |
| ATOM | 11814 | CD1 | TRP | H | 36 | 35.890 | 16.452 | -40.448 | 1.00 | 12.60 | C |
| ATOM | 11815 | CD2 | TRP | H | 36 | 33.983 | 15.786 | -39.535 | 1.00 | 12.70 | C |
| ATOM | 11816 | NE1 | TRP | H | 36 | 36.193 | 15.444 | -39.535 | 1.00 | 12.12 | N |
| ATOM | 11817 | CE2 | TRP | H | 36 | 35.025 | 15.014 | -38.970 | 1.00 | 12.95 | C |
| ATOM | 11818 | CE3 | TRP | H | 36 | 32.680 | 15.599 | -39.079 | 1.00 | 11.45 | C |

```
ATOM   11819  CZ2 TRP H   36      34.798  14.037 -37.987  1.00 12.23          C
ATOM   11820  CZ3 TRP H   36      32.440  14.548 -38.115  1.00 12.92          C
ATOM   11821  CH2 TRP H   36      33.527  13.827 -37.568  1.00 12.18          C
ATOM   11822  N   VAL H   37      31.083  17.773 -43.198  1.00 10.55          N
ATOM   11823  CA  VAL H   37      29.975  18.592 -43.668  1.00 10.25          C
ATOM   11824  C   VAL H   37      29.012  18.786 -42.528  1.00 11.33          C
ATOM   11825  O   VAL H   37      28.519  17.816 -41.935  1.00 11.90          O
ATOM   11826  CB  VAL H   37      29.235  17.953 -44.876  1.00  9.67          C
ATOM   11827  CG1 VAL H   37      28.033  18.811 -45.261  1.00 10.89          C
ATOM   11828  CG2 VAL H   37      30.169  17.750 -46.047  1.00  7.97          C
ATOM   11829  N   ARG H   38      28.707  20.049 -42.211  1.00  8.90          N
ATOM   11830  CA  ARG H   38      27.771  20.306 -41.158  1.00  9.92          C
ATOM   11831  C   ARG H   38      26.430  20.834 -41.709  1.00 10.71          C
ATOM   11832  O   ARG H   38      26.369  21.404 -42.802  1.00 10.73          O
ATOM   11833  CB  ARG H   38      28.375  21.198 -40.097  1.00 11.69          C
ATOM   11834  CG  ARG H   38      28.509  22.631 -40.560  1.00 12.12          C
ATOM   11835  CD  ARG H   38      29.312  23.439 -39.586  1.00 14.39          C
ATOM   11836  NE  ARG H   38      29.462  24.801 -40.068  1.00 13.83          N
ATOM   11837  CZ  ARG H   38      30.239  25.722 -39.517  1.00 14.41          C
ATOM   11838  NH1 ARG H   38      30.964  25.462 -38.459  1.00 15.39          N
ATOM   11839  NH2 ARG H   38      30.278  26.927 -40.020  1.00 16.33          N
ATOM   11840  N   GLN H   39      25.373  20.605 -40.944  1.00 11.04          N
ATOM   11841  CA  GLN H   39      24.036  21.007 -41.312  1.00 14.23          C
ATOM   11842  C   GLN H   39      23.405  21.811 -40.190  1.00 15.33          C
ATOM   11843  O   GLN H   39      23.503  21.479 -39.014  1.00 12.81          O
ATOM   11844  CB  GLN H   39      23.206  19.773 -41.611  1.00 13.41          C
ATOM   11845  CG  GLN H   39      21.885  20.054 -42.227  1.00 14.13          C
ATOM   11846  CD  GLN H   39      21.225  18.801 -42.726  1.00 16.90          C
ATOM   11847  OE1 GLN H   39      21.377  17.734 -42.120  1.00 20.00          O
ATOM   11848  NE2 GLN H   39      20.506  18.908 -43.831  1.00 18.55          N
ATOM   11849  N   SER H   40      22.836  22.952 -40.562  1.00 20.07          N
ATOM   11850  CA  SER H   40      22.048  23.737 -39.653  1.00 21.83          C
ATOM   11851  C   SER H   40      20.844  24.236 -40.436  1.00 24.69          C
ATOM   11852  O   SER H   40      20.850  24.260 -41.674  1.00 23.93          O
ATOM   11853  CB  SER H   40      22.855  24.909 -39.110  1.00 23.56          C
ATOM   11854  OG  SER H   40      23.183  25.798 -40.165  1.00 22.79          O
ATOM   11855  N   PRO H   41      19.791  24.628 -39.722  1.00 28.14          N
ATOM   11856  CA  PRO H   41      18.682  25.316 -40.381  1.00 29.68          C
ATOM   11857  C   PRO H   41      19.071  26.605 -41.102  1.00 30.99          C
ATOM   11858  O   PRO H   41      18.488  26.898 -42.138  1.00 33.47          O
ATOM   11859  CB  PRO H   41      17.732  25.621 -39.220  1.00 30.34          C
ATOM   11860  CG  PRO H   41      18.040  24.599 -38.206  1.00 29.36          C
ATOM   11861  CD  PRO H   41      19.528  24.419 -38.292  1.00 28.90          C
ATOM   11862  N   GLU H   42      20.043  27.364 -40.599  1.00 33.91          N
ATOM   11863  CA  GLU H   42      20.362  28.678 -41.207  1.00 33.17          C
ATOM   11864  C   GLU H   42      21.267  28.609 -42.452  1.00 33.30          C
ATOM   11865  O   GLU H   42      21.119  29.426 -43.370  1.00 33.89          O
ATOM   11866  CB  GLU H   42      20.939  29.646 -40.163  1.00 35.13          C
ATOM   11867  CG  GLU H   42      21.360  31.043 -40.689  1.00 38.14          C
ATOM   11868  CD  GLU H   42      20.178  31.969 -41.134  1.00 42.99          C
ATOM   11869  OE1 GLU H   42      19.545  32.626 -40.256  1.00 44.60          O
ATOM   11870  OE2 GLU H   42      19.924  32.073 -42.370  1.00 44.67          O
ATOM   11871  N   LYS H   43      22.202  27.662 -42.492  1.00 31.12          N
ATOM   11872  CA  LYS H   43      23.155  27.588 -43.608  1.00 29.65          C
ATOM   11873  C   LYS H   43      23.054  26.278 -44.409  1.00 27.77          C
ATOM   11874  O   LYS H   43      23.855  26.060 -45.307  1.00 28.57          O
ATOM   11875  CB  LYS H   43      24.609  27.817 -43.118  1.00 31.63          C
ATOM   11876  CG  LYS H   43      24.908  29.224 -42.566  1.00 33.49          C
ATOM   11877  CD  LYS H   43      24.969  30.299 -43.682  1.00 35.42          C
ATOM   11878  CE  LYS H   43      25.437  31.684 -43.159  1.00 35.19          C
ATOM   11879  NZ  LYS H   43      25.230  32.839 -44.137  1.00 37.19          N
ATOM   11880  N   GLY H   44      22.063  25.437 -44.113  1.00 24.04          N
ATOM   11881  CA  GLY H   44      21.891  24.168 -44.809  1.00 23.14          C
ATOM   11882  C   GLY H   44      23.146  23.337 -44.676  1.00 21.35          C
ATOM   11883  O   GLY H   44      23.787  23.376 -43.630  1.00 21.40          O
```

| ATOM | 11884 | N   | LEU | H | 45 | 23.517 | 22.630 | -45.755 | 1.00 | 19.83 | N |
| ATOM | 11885 | CA  | LEU | H | 45 | 24.701 | 21.783 | -45.790 | 1.00 | 18.35 | C |
| ATOM | 11886 | C   | LEU | H | 45 | 25.925 | 22.614 | -46.123 | 1.00 | 16.62 | C |
| ATOM | 11887 | O   | LEU | H | 45 | 25.981 | 23.267 | -47.182 | 1.00 | 16.39 | O |
| ATOM | 11888 | CB  | LEU | H | 45 | 24.537 | 20.672 | -46.839 | 1.00 | 17.79 | C |
| ATOM | 11889 | CG  | LEU | H | 45 | 23.587 | 19.509 | -46.497 | 1.00 | 18.00 | C |
| ATOM | 11890 | CD1 | LEU | H | 45 | 23.421 | 18.600 | -47.682 | 1.00 | 17.22 | C |
| ATOM | 11891 | CD2 | LEU | H | 45 | 24.063 | 18.711 | -45.299 | 1.00 | 17.13 | C |
| ATOM | 11892 | N   | GLU | H | 46 | 26.915 | 22.551 | -45.249 | 1.00 | 14.28 | N |
| ATOM | 11893 | CA  | GLU | H | 46 | 28.155 | 23.301 | -45.384 | 1.00 | 16.46 | C |
| ATOM | 11894 | C   | GLU | H | 46 | 29.389 | 22.419 | -45.164 | 1.00 | 14.75 | C |
| ATOM | 11895 | O   | GLU | H | 46 | 29.668 | 22.025 | -44.059 | 1.00 | 11.64 | O |
| ATOM | 11896 | CB  | GLU | H | 46 | 28.119 | 24.373 | -44.314 | 1.00 | 17.20 | C |
| ATOM | 11897 | CG  | GLU | H | 46 | 29.238 | 25.320 | -44.228 | 1.00 | 21.97 | C |
| ATOM | 11898 | CD  | GLU | H | 46 | 28.924 | 26.380 | -43.176 | 1.00 | 23.38 | C |
| ATOM | 11899 | OE1 | GLU | H | 46 | 27.955 | 26.201 | -42.350 | 1.00 | 26.41 | O |
| ATOM | 11900 | OE2 | GLU | H | 46 | 29.637 | 27.400 | -43.195 | 1.00 | 29.19 | O |
| ATOM | 11901 | N   | TRP | H | 47 | 30.121 | 22.172 | -46.241 | 1.00 | 13.00 | N |
| ATOM | 11902 | CA  | TRP | H | 47 | 31.429 | 21.548 | -46.213 | 1.00 | 13.25 | C |
| ATOM | 11903 | C   | TRP | H | 47 | 32.405 | 22.420 | -45.421 | 1.00 | 13.04 | C |
| ATOM | 11904 | O   | TRP | H | 47 | 32.488 | 23.627 | -45.676 | 1.00 | 11.95 | O |
| ATOM | 11905 | CB  | TRP | H | 47 | 31.906 | 21.434 | -47.648 | 1.00 | 11.04 | C |
| ATOM | 11906 | CG  | TRP | H | 47 | 33.342 | 20.966 | -47.858 | 1.00 | 11.66 | C |
| ATOM | 11907 | CD1 | TRP | H | 47 | 33.737 | 19.666 | -48.012 | 1.00 | 11.53 | C |
| ATOM | 11908 | CD2 | TRP | H | 47 | 34.514 | 21.763 | -48.073 | 1.00 | 9.41  | C |
| ATOM | 11909 | NE1 | TRP | H | 47 | 35.081 | 19.604 | -48.242 | 1.00 | 11.50 | N |
| ATOM | 11910 | CE2 | TRP | H | 47 | 35.588 | 20.879 | -48.290 | 1.00 | 11.58 | C |
| ATOM | 11911 | CE3 | TRP | H | 47 | 34.769 | 23.153 | -48.087 | 1.00 | 11.73 | C |
| ATOM | 11912 | CZ2 | TRP | H | 47 | 36.891 | 21.323 | -48.524 | 1.00 | 11.46 | C |
| ATOM | 11913 | CZ3 | TRP | H | 47 | 36.049 | 23.584 | -48.283 | 1.00 | 11.42 | C |
| ATOM | 11914 | CH2 | TRP | H | 47 | 37.110 | 22.686 | -48.489 | 1.00 | 13.56 | C |
| ATOM | 11915 | N   | VAL | H | 48 | 33.117 | 21.848 | -44.468 | 1.00 | 11.23 | N |
| ATOM | 11916 | CA  | VAL | H | 48 | 34.032 | 22.653 | -43.644 | 1.00 | 12.98 | C |
| ATOM | 11917 | C   | VAL | H | 48 | 35.487 | 22.243 | -43.651 | 1.00 | 12.85 | C |
| ATOM | 11918 | O   | VAL | H | 48 | 36.364 | 23.069 | -43.512 | 1.00 | 10.16 | O |
| ATOM | 11919 | CB  | VAL | H | 48 | 33.501 | 22.837 | -42.183 | 1.00 | 14.41 | C |
| ATOM | 11920 | CG1 | VAL | H | 48 | 32.148 | 23.557 | -42.189 | 1.00 | 15.47 | C |
| ATOM | 11921 | CG2 | VAL | H | 48 | 33.447 | 21.576 | -41.419 | 1.00 | 14.30 | C |
| ATOM | 11922 | N   | ALA | H | 49 | 35.773 | 20.962 | -43.847 | 1.00 | 12.74 | N |
| ATOM | 11923 | CA  | ALA | H | 49 | 37.146 | 20.543 | -43.798 | 1.00 | 11.93 | C |
| ATOM | 11924 | C   | ALA | H | 49 | 37.292 | 19.267 | -44.605 | 1.00 | 11.84 | C |
| ATOM | 11925 | O   | ALA | H | 49 | 36.377 | 18.426 | -44.631 | 1.00 | 13.94 | O |
| ATOM | 11926 | CB  | ALA | H | 49 | 37.596 | 20.321 | -42.358 | 1.00 | 12.89 | C |
| ATOM | 11927 | N   | GLU | H | 50 | 38.424 | 19.171 | -45.273 | 1.00 | 12.45 | N |
| ATOM | 11928 | CA  | GLU | H | 50 | 38.800 | 17.963 | -46.025 | 1.00 | 12.47 | C |
| ATOM | 11929 | C   | GLU | H | 50 | 40.282 | 17.578 | -45.907 | 1.00 | 12.90 | C |
| ATOM | 11930 | O   | GLU | H | 50 | 41.169 | 18.414 | -45.781 | 1.00 | 13.02 | O |
| ATOM | 11931 | CB  | GLU | H | 50 | 38.487 | 18.178 | -47.502 | 1.00 | 12.68 | C |
| ATOM | 11932 | CG  | GLU | H | 50 | 38.525 | 16.873 | -48.333 | 1.00 | 11.07 | C |
| ATOM | 11933 | CD  | GLU | H | 50 | 37.693 | 16.956 | -49.595 | 1.00 | 13.88 | C |
| ATOM | 11934 | OE1 | GLU | H | 50 | 36.559 | 17.486 | -49.508 | 1.00 | 12.15 | O |
| ATOM | 11935 | OE2 | GLU | H | 50 | 38.130 | 16.453 | -50.679 | 1.00 | 18.87 | O |
| ATOM | 11936 | N   | ILE | H | 51 | 40.557 | 16.276 | -45.997 | 1.00 | 11.63 | N |
| ATOM | 11937 | CA  | ILE | H | 51 | 41.908 | 15.777 | -45.855 | 1.00 | 12.91 | C |
| ATOM | 11938 | C   | ILE | H | 51 | 42.157 | 14.688 | -46.888 | 1.00 | 12.01 | C |
| ATOM | 11939 | O   | ILE | H | 51 | 41.276 | 13.861 | -47.153 | 1.00 | 15.75 | O |
| ATOM | 11940 | CB  | ILE | H | 51 | 42.237 | 15.282 | -44.415 | 1.00 | 12.49 | C |
| ATOM | 11941 | CG1 | ILE | H | 51 | 43.742 | 15.029 | -44.289 | 1.00 | 10.02 | C |
| ATOM | 11942 | CG2 | ILE | H | 51 | 41.360 | 14.111 | -43.979 | 1.00 | 11.75 | C |
| ATOM | 11943 | CD1 | ILE | H | 51 | 44.224 | 14.861 | -42.936 | 1.00 | 12.95 | C |
| ATOM | 11944 | N   | ARG | H | 52 | 43.335 | 14.738 | -47.495 | 1.00 | 13.00 | N |
| ATOM | 11945 | CA  | ARG | H | 52 | 43.656 | 13.831 | -48.566 | 1.00 | 14.35 | C |
| ATOM | 11946 | C   | ARG | H | 52 | 44.529 | 12.693 | -48.001 | 1.00 | 15.52 | C |
| ATOM | 11947 | O   | ARG | H | 52 | 44.414 | 12.389 | -46.831 | 1.00 | 14.44 | O |
| ATOM | 11948 | CB  | ARG | H | 52 | 44.267 | 14.605 | -49.721 | 1.00 | 15.35 | C |

```
ATOM  11949  CG  ARG H  52    43.356  15.736 -50.267  1.00 14.13        C
ATOM  11950  CD  ARG H  52    42.118  15.171 -50.915  1.00 16.10        C
ATOM  11951  NE  ARG H  52    41.073  16.123 -51.332  1.00 14.71        N
ATOM  11952  CZ  ARG H  52    41.060  16.801 -52.476  1.00 16.27        C
ATOM  11953  NH1 ARG H  52    42.075  16.755 -53.314  1.00 17.17        N
ATOM  11954  NH2 ARG H  52    40.028  17.584 -52.773  1.00 13.33        N
ATOM  11955  N   LEU H  52A   45.335  12.030 -48.827  1.00 17.24        N
ATOM  11956  CA  LEU H  52A   45.957  10.767 -48.422  1.00 18.31        C
ATOM  11957  C   LEU H  52A   47.388  10.992 -47.935  1.00 19.54        C
ATOM  11958  O   LEU H  52A   47.921  12.093 -47.997  1.00 18.05        O
ATOM  11959  CB  LEU H  52A   45.954   9.754 -49.563  1.00 18.30        C
ATOM  11960  CG  LEU H  52A   44.624   9.569 -50.326  1.00 15.67        C
ATOM  11961  CD1 LEU H  52A   44.755   8.495 -51.393  1.00 20.31        C
ATOM  11962  CD2 LEU H  52A   43.466   9.279 -49.373  1.00 15.81        C
ATOM  11963  N   LYS H  52B   47.993   9.937 -47.406  1.00 20.23        N
ATOM  11964  CA  LYS H  52B   49.372  10.008 -46.965  1.00 20.42        C
ATOM  11965  C   LYS H  52B   50.275  10.437 -48.117  1.00 20.54        C
ATOM  11966  O   LYS H  52B   51.137  11.313 -47.943  1.00 20.29        O
ATOM  11967  CB  LYS H  52B   49.803   8.658 -46.439  1.00 21.81        C
ATOM  11968  CG  LYS H  52B   51.140   8.651 -45.754  1.00 22.03        C
ATOM  11969  CD  LYS H  52B   51.429   7.273 -45.172  1.00 24.02        C
ATOM  11970  CE  LYS H  52B   52.789   7.246 -44.478  1.00 24.90        C
ATOM  11971  NZ  LYS H  52B   53.267   5.814 -44.330  1.00 28.55        N
ATOM  11972  N   SER H  52C   50.000   9.871 -49.291  1.00 19.39        N
ATOM  11973  CA  SER H  52C   50.652  10.180 -50.552  1.00 21.41        C
ATOM  11974  C   SER H  52C   50.634  11.667 -50.835  1.00 21.71        C
ATOM  11975  O   SER H  52C   51.466  12.156 -51.592  1.00 23.40        O
ATOM  11976  CB  SER H  52C   49.886   9.509 -51.694  1.00 21.85        C
ATOM  11977  OG  SER H  52C   49.850   8.116 -51.490  1.00 29.48        O
ATOM  11978  N   ASP H  53    49.651  12.366 -50.268  1.00 20.61        N
ATOM  11979  CA  ASP H  53    49.514  13.789 -50.487  1.00 19.64        C
ATOM  11980  C   ASP H  53    50.068  14.574 -49.326  1.00 19.17        C
ATOM  11981  O   ASP H  53    49.755  15.762 -49.183  1.00 19.82        O
ATOM  11982  CB  ASP H  53    48.043  14.135 -50.678  1.00 19.76        C
ATOM  11983  CG  ASP H  53    47.407  13.335 -51.785  1.00 21.89        C
ATOM  11984  OD1 ASP H  53    47.840  13.519 -52.958  1.00 21.38        O
ATOM  11985  OD2 ASP H  53    46.499  12.515 -51.485  1.00 18.72        O
ATOM  11986  N   ASN H  54    50.917  13.943 -48.504  1.00 17.69        N
ATOM  11987  CA  ASN H  54    51.371  14.555 -47.273  1.00 17.73        C
ATOM  11988  C   ASN H  54    50.170  15.038 -46.457  1.00 16.28        C
ATOM  11989  O   ASN H  54    50.214  16.099 -45.810  1.00 14.36        O
ATOM  11990  CB  ASN H  54    52.333  15.721 -47.547  1.00 18.46        C
ATOM  11991  CG  ASN H  54    53.762  15.276 -47.900  1.00 20.34        C
ATOM  11992  OD1 ASN H  54    54.228  14.206 -47.505  1.00 20.55        O
ATOM  11993  ND2 ASN H  54    54.466  16.131 -48.609  1.00 21.03        N
ATOM  11994  N   TYR H  55    49.091  14.258 -46.514  1.00 15.27        N
ATOM  11995  CA  TYR H  55    47.844  14.555 -45.799  1.00 16.82        C
ATOM  11996  C   TYR H  55    47.344  15.987 -46.008  1.00 15.83        C
ATOM  11997  O   TYR H  55    46.944  16.655 -45.049  1.00 16.42        O
ATOM  11998  CB  TYR H  55    48.044  14.314 -44.326  1.00 17.72        C
ATOM  11999  CG  TYR H  55    48.291  12.878 -43.986  1.00 17.38        C
ATOM  12000  CD1 TYR H  55    47.321  11.936 -44.220  1.00 19.27        C
ATOM  12001  CD2 TYR H  55    49.494  12.466 -43.425  1.00 18.53        C
ATOM  12002  CE1 TYR H  55    47.501  10.617 -43.903  1.00 20.25        C
ATOM  12003  CE2 TYR H  55    49.692  11.125 -43.088  1.00 19.88        C
ATOM  12004  CZ  TYR H  55    48.686  10.222 -43.334  1.00 19.51        C
ATOM  12005  OH  TYR H  55    48.827   8.905 -43.004  1.00 20.66        O
ATOM  12006  N   ALA H  56    47.333  16.425 -47.256  1.00 15.75        N
ATOM  12007  CA  ALA H  56    46.940  17.813 -47.560  1.00 15.78        C
ATOM  12008  C   ALA H  56    45.553  18.063 -46.992  1.00 14.74        C
ATOM  12009  O   ALA H  56    44.672  17.187 -47.039  1.00 14.18        O
ATOM  12010  CB  ALA H  56    46.934  18.079 -49.041  1.00 17.15        C
ATOM  12011  N   THR H  57    45.357  19.263 -46.461  1.00 15.21        N
ATOM  12012  CA  THR H  57    44.065  19.635 -45.892  1.00 15.07        C
ATOM  12013  C   THR H  57    43.503  20.875 -46.568  1.00 15.02        C
```

| ATOM | 12014 | O | THR | H | 57 | 44.266 | 21.711 | -47.063 | 1.00 | 13.47 | O |
|------|-------|------|-----|---|----|--------|--------|---------|------|-------|---|
| ATOM | 12015 | CB | THR | H | 57 | 44.107 | 19.904 | -44.372 | 1.00 | 15.71 | C |
| ATOM | 12016 | OG1 | THR | H | 57 | 45.084 | 20.899 | -44.059 | 1.00 | 13.50 | O |
| ATOM | 12017 | CG2 | THR | H | 57 | 44.443 | 18.651 | -43.627 | 1.00 | 13.97 | C |
| ATOM | 12018 | N | TYR | H | 58 | 42.169 | 20.958 | -46.586 | 1.00 | 14.04 | N |
| ATOM | 12019 | CA | TYR | H | 58 | 41.439 | 22.130 | -47.110 | 1.00 | 14.81 | C |
| ATOM | 12020 | C | TYR | H | 58 | 40.324 | 22.502 | -46.155 | 1.00 | 12.98 | C |
| ATOM | 12021 | O | TYR | H | 58 | 39.769 | 21.660 | -45.480 | 1.00 | 11.28 | O |
| ATOM | 12022 | CB | TYR | H | 58 | 40.881 | 21.812 | -48.521 | 1.00 | 15.33 | C |
| ATOM | 12023 | CG | TYR | H | 58 | 41.977 | 21.255 | -49.404 | 1.00 | 15.86 | C |
| ATOM | 12024 | CD1 | TYR | H | 58 | 42.193 | 19.888 | -49.487 | 1.00 | 15.39 | C |
| ATOM | 12025 | CD2 | TYR | H | 58 | 42.850 | 22.094 | -50.080 | 1.00 | 14.71 | C |
| ATOM | 12026 | CE1 | TYR | H | 58 | 43.225 | 19.383 | -50.249 | 1.00 | 15.75 | C |
| ATOM | 12027 | CE2 | TYR | H | 58 | 43.874 | 21.592 | -50.842 | 1.00 | 15.18 | C |
| ATOM | 12028 | CZ | TYR | H | 58 | 44.061 | 20.223 | -50.911 | 1.00 | 15.92 | C |
| ATOM | 12029 | OH | TYR | H | 58 | 45.088 | 19.702 | -51.668 | 1.00 | 15.33 | O |
| ATOM | 12030 | N | TYR | H | 59 | 39.997 | 23.806 | -46.074 | 1.00 | 13.56 | N |
| ATOM | 12031 | CA | TYR | H | 59 | 39.043 | 24.277 | -45.110 | 1.00 | 13.80 | C |
| ATOM | 12032 | C | TYR | H | 59 | 38.170 | 25.330 | -45.743 | 1.00 | 15.34 | C |
| ATOM | 12033 | O | TYR | H | 59 | 38.651 | 26.085 | -46.604 | 1.00 | 13.81 | O |
| ATOM | 12034 | CB | TYR | H | 59 | 39.747 | 24.897 | -43.881 | 1.00 | 14.58 | C |
| ATOM | 12035 | CG | TYR | H | 59 | 40.581 | 23.888 | -43.093 | 1.00 | 12.45 | C |
| ATOM | 12036 | CD1 | TYR | H | 59 | 40.018 | 23.117 | -42.096 | 1.00 | 14.40 | C |
| ATOM | 12037 | CD2 | TYR | H | 59 | 41.938 | 23.731 | -43.365 | 1.00 | 12.87 | C |
| ATOM | 12038 | CE1 | TYR | H | 59 | 40.763 | 22.174 | -41.399 | 1.00 | 11.75 | C |
| ATOM | 12039 | CE2 | TYR | H | 59 | 42.721 | 22.791 | -42.657 | 1.00 | 13.53 | C |
| ATOM | 12040 | CZ | TYR | H | 59 | 42.116 | 22.013 | -41.678 | 1.00 | 14.40 | C |
| ATOM | 12041 | OH | TYR | H | 59 | 42.884 | 21.113 | -40.966 | 1.00 | 11.56 | O |
| ATOM | 12042 | N | ALA | H | 60 | 36.921 | 25.390 | -45.276 | 1.00 | 14.32 | N |
| ATOM | 12043 | CA | ALA | H | 60 | 36.028 | 26.481 | -45.608 | 1.00 | 15.07 | C |
| ATOM | 12044 | C | ALA | H | 60 | 36.624 | 27.731 | -45.007 | 1.00 | 14.99 | C |
| ATOM | 12045 | O | ALA | H | 60 | 37.240 | 27.681 | -43.934 | 1.00 | 12.72 | O |
| ATOM | 12046 | CB | ALA | H | 60 | 34.678 | 26.241 | -45.064 | 1.00 | 15.04 | C |
| ATOM | 12047 | N | GLU | H | 61 | 36.404 | 28.842 | -45.711 | 1.00 | 16.09 | N |
| ATOM | 12048 | CA | GLU | H | 61 | 36.973 | 30.140 | -45.365 | 1.00 | 18.47 | C |
| ATOM | 12049 | C | GLU | H | 61 | 36.631 | 30.486 | -43.949 | 1.00 | 18.93 | C |
| ATOM | 12050 | O | GLU | H | 61 | 37.456 | 31.027 | -43.217 | 1.00 | 20.72 | O |
| ATOM | 12051 | CB | GLU | H | 61 | 36.368 | 31.206 | -46.275 | 1.00 | 19.12 | C |
| ATOM | 12052 | CG | GLU | H | 61 | 36.872 | 32.631 | -46.061 | 1.00 | 22.97 | C |
| ATOM | 12053 | CD | GLU | H | 61 | 38.262 | 32.841 | -46.615 | 1.00 | 29.36 | C |
| ATOM | 12054 | OE1 | GLU | H | 61 | 38.771 | 31.977 | -47.383 | 1.00 | 33.83 | O |
| ATOM | 12055 | OE2 | GLU | H | 61 | 38.849 | 33.893 | -46.284 | 1.00 | 34.59 | O |
| ATOM | 12056 | N | SER | H | 62 | 35.398 | 30.205 | -43.569 | 1.00 | 19.87 | N |
| ATOM | 12057 | CA | SER | H | 62 | 34.896 | 30.592 | -42.243 | 1.00 | 21.26 | C |
| ATOM | 12058 | C | SER | H | 62 | 35.423 | 29.807 | -41.063 | 1.00 | 21.60 | C |
| ATOM | 12059 | O | SER | H | 62 | 35.273 | 30.252 | -39.938 | 1.00 | 23.29 | O |
| ATOM | 12060 | CB | SER | H | 62 | 33.372 | 30.512 | -42.232 | 1.00 | 22.12 | C |
| ATOM | 12061 | OG | SER | H | 62 | 32.964 | 29.167 | -42.326 | 1.00 | 23.47 | O |
| ATOM | 12062 | N | VAL | H | 63 | 36.030 | 28.641 | -41.281 | 1.00 | 20.36 | N |
| ATOM | 12063 | CA | VAL | H | 63 | 36.621 | 27.889 | -40.183 | 1.00 | 20.92 | C |
| ATOM | 12064 | C | VAL | H | 63 | 38.162 | 27.813 | -40.203 | 1.00 | 20.19 | C |
| ATOM | 12065 | O | VAL | H | 63 | 38.785 | 27.359 | -39.241 | 1.00 | 16.15 | O |
| ATOM | 12066 | CB | VAL | H | 63 | 36.017 | 26.461 | -40.126 | 1.00 | 20.63 | C |
| ATOM | 12067 | CG1 | VAL | H | 63 | 34.510 | 26.538 | -40.059 | 1.00 | 21.86 | C |
| ATOM | 12068 | CG2 | VAL | H | 63 | 36.409 | 25.669 | -41.344 | 1.00 | 21.77 | C |
| ATOM | 12069 | N | LYS | H | 64 | 38.781 | 28.282 | -41.277 | 1.00 | 21.00 | N |
| ATOM | 12070 | CA | LYS | H | 64 | 40.233 | 28.311 | -41.368 | 1.00 | 22.95 | C |
| ATOM | 12071 | C | LYS | H | 64 | 40.857 | 28.984 | -40.144 | 1.00 | 23.58 | C |
| ATOM | 12072 | O | LYS | H | 64 | 40.448 | 30.063 | -39.742 | 1.00 | 23.58 | O |
| ATOM | 12073 | CB | LYS | H | 64 | 40.666 | 29.021 | -42.644 | 1.00 | 25.11 | C |
| ATOM | 12074 | CG | LYS | H | 64 | 40.480 | 28.194 | -43.884 | 1.00 | 27.03 | C |
| ATOM | 12075 | CD | LYS | H | 64 | 40.840 | 28.973 | -45.158 | 1.00 | 27.06 | C |
| ATOM | 12076 | CE | LYS | H | 64 | 40.751 | 28.094 | -46.381 | 1.00 | 27.28 | C |
| ATOM | 12077 | NZ | LYS | H | 64 | 41.794 | 28.367 | -47.414 | 1.00 | 29.47 | N |
| ATOM | 12078 | N | GLY | H | 65 | 41.816 | 28.306 | -39.527 | 1.00 | 23.59 | N |

```
ATOM  12079  CA   GLY H  65     42.427  28.794 -38.302  1.00 23.53       C
ATOM  12080  C    GLY H  65     41.706  28.328 -37.055  1.00 23.15       C
ATOM  12081  O    GLY H  65     42.274  28.384 -35.959  1.00 25.70       O
ATOM  12082  N    LYS H  66     40.465  27.869 -37.196  1.00 22.00       N
ATOM  12083  CA   LYS H  66     39.721  27.363 -36.045  1.00 21.55       C
ATOM  12084  C    LYS H  66     39.712  25.843 -36.002  1.00 18.35       C
ATOM  12085  O    LYS H  66     39.867  25.272 -34.945  1.00 17.72       O
ATOM  12086  CB   LYS H  66     38.282  27.898 -36.003  1.00 23.27       C
ATOM  12087  CG   LYS H  66     38.186  29.417 -35.969  1.00 23.99       C
ATOM  12088  CD   LYS H  66     37.041  29.877 -35.101  1.00 24.93       C
ATOM  12089  CE   LYS H  66     37.009  31.381 -35.000  1.00 25.61       C
ATOM  12090  NZ   LYS H  66     37.027  31.874 -33.616  1.00 25.84       N
ATOM  12091  N    PHE H  67     39.514  25.201 -37.147  1.00 17.29       N
ATOM  12092  CA   PHE H  67     39.354  23.750 -37.210  1.00 16.11       C
ATOM  12093  C    PHE H  67     40.624  23.075 -37.730  1.00 16.66       C
ATOM  12094  O    PHE H  67     41.323  23.605 -38.606  1.00 13.03       O
ATOM  12095  CB   PHE H  67     38.229  23.378 -38.165  1.00 15.62       C
ATOM  12096  CG   PHE H  67     36.845  23.711 -37.687  1.00 15.67       C
ATOM  12097  CD1  PHE H  67     36.587  24.514 -36.586  1.00 17.25       C
ATOM  12098  CD2  PHE H  67     35.763  23.234 -38.413  1.00 17.78       C
ATOM  12099  CE1  PHE H  67     35.267  24.794 -36.204  1.00 16.27       C
ATOM  12100  CE2  PHE H  67     34.465  23.518 -38.039  1.00 15.99       C
ATOM  12101  CZ   PHE H  67     34.219  24.299 -36.939  1.00 14.86       C
ATOM  12102  N    THR H  68     40.854  21.859 -37.245  1.00 15.58       N
ATOM  12103  CA   THR H  68     41.932  21.048 -37.720  1.00 14.37       C
ATOM  12104  C    THR H  68     41.391  19.638 -37.947  1.00 12.17       C
ATOM  12105  O    THR H  68     40.870  19.042 -37.048  1.00 11.03       O
ATOM  12106  CB   THR H  68     43.062  21.034 -36.688  1.00 16.61       C
ATOM  12107  OG1  THR H  68     43.584  22.367 -36.542  1.00 18.14       O
ATOM  12108  CG2  THR H  68     44.164  20.120 -37.127  1.00 19.38       C
ATOM  12109  N    ILE H  69     41.494  19.165 -39.174  1.00 11.13       N
ATOM  12110  CA   ILE H  69     41.096  17.788 -39.544  1.00 11.06       C
ATOM  12111  C    ILE H  69     42.328  16.909 -39.533  1.00 11.35       C
ATOM  12112  O    ILE H  69     43.390  17.335 -39.939  1.00 11.31       O
ATOM  12113  CB   ILE H  69     40.346  17.766 -40.940  1.00  9.72       C
ATOM  12114  CG1  ILE H  69     39.780  16.356 -41.246  1.00  8.27       C
ATOM  12115  CG2  ILE H  69     41.215  18.275 -42.069  1.00 10.69       C
ATOM  12116  CD1  ILE H  69     38.764  16.305 -42.410  1.00 10.67       C
ATOM  12117  N    SER H  70     42.183  15.631 -39.145  1.00  9.57       N
ATOM  12118  CA   SER H  70     43.339  14.781 -39.068  1.00 11.26       C
ATOM  12119  C    SER H  70     42.791  13.393 -39.215  1.00  9.69       C
ATOM  12120  O    SER H  70     41.596  13.176 -39.070  1.00  7.81       O
ATOM  12121  CB   SER H  70     44.092  14.900 -37.737  1.00 11.82       C
ATOM  12122  OG   SER H  70     43.295  14.609 -36.601  1.00 12.28       O
ATOM  12123  N    ARG H  71     43.655  12.455 -39.490  1.00 14.33       N
ATOM  12124  CA   ARG H  71     43.189  11.102 -39.649  1.00 14.49       C
ATOM  12125  C    ARG H  71     44.213  10.122 -39.067  1.00 16.67       C
ATOM  12126  O    ARG H  71     45.417  10.397 -39.091  1.00 16.45       O
ATOM  12127  CB   ARG H  71     42.929  10.836 -41.138  1.00 15.12       C
ATOM  12128  CG   ARG H  71     44.163  11.028 -42.072  1.00 14.25       C
ATOM  12129  CD   ARG H  71     43.811  11.015 -43.564  1.00 13.22       C
ATOM  12130  NE   ARG H  71     43.272   9.724 -43.963  1.00 15.17       N
ATOM  12131  CZ   ARG H  71     42.484   9.532 -45.005  1.00 13.80       C
ATOM  12132  NH1  ARG H  71     42.121  10.545 -45.780  1.00 11.83       N
ATOM  12133  NH2  ARG H  71     42.024   8.305 -45.254  1.00 14.11       N
ATOM  12134  N    ASP H  72     43.716   9.009 -38.528  1.00 17.60       N
ATOM  12135  CA   ASP H  72     44.570   7.910 -38.093  1.00 18.11       C
ATOM  12136  C    ASP H  72     44.257   6.706 -38.961  1.00 17.16       C
ATOM  12137  O    ASP H  72     43.331   5.985 -38.683  1.00 18.46       O
ATOM  12138  CB   ASP H  72     44.323   7.569 -36.624  1.00 19.78       C
ATOM  12139  CG   ASP H  72     45.314   6.536 -36.082  1.00 20.31       C
ATOM  12140  OD1  ASP H  72     45.911   5.737 -36.867  1.00 23.37       O
ATOM  12141  OD2  ASP H  72     45.486   6.527 -34.837  1.00 25.36       O
ATOM  12142  N    ASP H  73     45.026   6.504 -40.024  1.00 16.30       N
ATOM  12143  CA   ASP H  73     44.722   5.462 -41.001  1.00 18.97       C
```

694

| ATOM | 12144 | C   | ASP H | 73 | 44.708 | 4.081  | -40.347 | 1.00 | 20.37 | C |
|------|-------|-----|-------|----|--------|--------|---------|------|-------|---|
| ATOM | 12145 | O   | ASP H | 73 | 43.892 | 3.216  | -40.694 | 1.00 | 20.84 | O |
| ATOM | 12146 | CB  | ASP H | 73 | 45.720 | 5.518  | -42.194 | 1.00 | 18.34 | C |
| ATOM | 12147 | CG  | ASP H | 73 | 45.482 | 6.755  | -43.097 | 1.00 | 18.91 | C |
| ATOM | 12148 | OD1 | ASP H | 73 | 44.333 | 7.213  | -43.095 | 1.00 | 18.22 | O |
| ATOM | 12149 | OD2 | ASP H | 73 | 46.407 | 7.260  | -43.779 | 1.00 | 17.45 | O |
| ATOM | 12150 | N   | SER H | 74 | 45.594 | 3.907  | -39.378 | 1.00 | 21.41 | N |
| ATOM | 12151 | CA  | SER H | 74 | 45.709 | 2.652  | -38.629 | 1.00 | 22.34 | C |
| ATOM | 12152 | C   | SER H | 74 | 44.474 | 2.270  | -37.825 | 1.00 | 23.63 | C |
| ATOM | 12153 | O   | SER H | 74 | 44.277 | 1.105  | -37.532 | 1.00 | 25.00 | O |
| ATOM | 12154 | CB  | SER H | 74 | 46.910 | 2.725  | -37.686 | 1.00 | 23.64 | C |
| ATOM | 12155 | OG  | SER H | 74 | 46.640 | 3.526  | -36.542 | 1.00 | 23.39 | O |
| ATOM | 12156 | N   | LYS H | 75 | 43.661 | 3.249  | -37.438 | 1.00 | 22.55 | N |
| ATOM | 12157 | CA  | LYS H | 75 | 42.439 | 2.985  | -36.672 | 1.00 | 22.49 | C |
| ATOM | 12158 | C   | LYS H | 75 | 41.199 | 3.269  | -37.498 | 1.00 | 21.19 | C |
| ATOM | 12159 | O   | LYS H | 75 | 40.087 | 3.157  | -36.995 | 1.00 | 19.87 | O |
| ATOM | 12160 | CB  | LYS H | 75 | 42.425 | 3.843  | -35.409 | 1.00 | 22.78 | C |
| ATOM | 12161 | CG  | LYS H | 75 | 43.589 | 3.596  | -34.481 | 1.00 | 23.98 | C |
| ATOM | 12162 | CD  | LYS H | 75 | 43.406 | 4.355  | -33.178 | 1.00 | 24.70 | C |
| ATOM | 12163 | CE  | LYS H | 75 | 44.619 | 4.234  | -32.255 | 1.00 | 25.48 | C |
| ATOM | 12164 | NZ  | LYS H | 75 | 44.693 | 5.403  | -31.286 | 1.00 | 27.24 | N |
| ATOM | 12165 | N   | SER H | 76 | 41.400 | 3.644  | -38.763 | 1.00 | 21.00 | N |
| ATOM | 12166 | CA  | SER H | 76 | 40.316 | 3.981  | -39.652 | 1.00 | 20.48 | C |
| ATOM | 12167 | C   | SER H | 76 | 39.464 | 5.063  | -39.027 | 1.00 | 19.14 | C |
| ATOM | 12168 | O   | SER H | 76 | 38.245 | 4.978  | -39.087 | 1.00 | 18.44 | O |
| ATOM | 12169 | CB  | SER H | 76 | 39.460 | 2.754  | -39.986 | 1.00 | 21.37 | C |
| ATOM | 12170 | OG  | SER H | 76 | 40.204 | 1.833  | -40.755 | 1.00 | 20.69 | O |
| ATOM | 12171 | N   | ARG H | 77 | 40.132 | 6.054  | -38.425 | 1.00 | 19.34 | N |
| ATOM | 12172 | CA  | ARG H | 77 | 39.465 | 7.108  | -37.663 | 1.00 | 19.00 | C |
| ATOM | 12173 | C   | ARG H | 77 | 39.810 | 8.509  | -38.176 | 1.00 | 16.68 | C |
| ATOM | 12174 | O   | ARG H | 77 | 40.975 | 8.840  | -38.410 | 1.00 | 16.07 | O |
| ATOM | 12175 | CB  | ARG H | 77 | 39.763 | 6.981  | -36.162 | 1.00 | 19.87 | C |
| ATOM | 12176 | CG  | ARG H | 77 | 38.910 | 7.912  | -35.283 | 1.00 | 21.06 | C |
| ATOM | 12177 | CD  | ARG H | 77 | 38.864 | 7.473  | -33.828 | 1.00 | 24.20 | C |
| ATOM | 12178 | NE  | ARG H | 77 | 37.991 | 6.312  | -33.599 | 1.00 | 27.62 | N |
| ATOM | 12179 | CZ  | ARG H | 77 | 36.663 | 6.362  | -33.423 | 1.00 | 29.43 | C |
| ATOM | 12180 | NH1 | ARG H | 77 | 35.976 | 7.516  | -33.490 | 1.00 | 29.17 | N |
| ATOM | 12181 | NH2 | ARG H | 77 | 35.994 | 5.230  | -33.214 | 1.00 | 30.44 | N |
| ATOM | 12182 | N   | LEU H | 78 | 38.758 | 9.300  | -38.360 | 1.00 | 14.84 | N |
| ATOM | 12183 | CA  | LEU H | 78 | 38.864 | 10.713 | -38.757 | 1.00 | 14.77 | C |
| ATOM | 12184 | C   | LEU H | 78 | 38.481 | 11.609 | -37.586 | 1.00 | 13.10 | C |
| ATOM | 12185 | O   | LEU H | 78 | 37.509 | 11.330 | -36.882 | 1.00 | 15.10 | O |
| ATOM | 12186 | CB  | LEU H | 78 | 37.908 | 10.973 | -39.909 | 1.00 | 14.16 | C |
| ATOM | 12187 | CG  | LEU H | 78 | 37.897 | 12.382 | -40.550 | 1.00 | 11.96 | C |
| ATOM | 12188 | CD1 | LEU H | 78 | 39.120 | 12.514 | -41.375 | 1.00 | 13.96 | C |
| ATOM | 12189 | CD2 | LEU H | 78 | 36.630 | 12.535 | -41.372 | 1.00 | 13.62 | C |
| ATOM | 12190 | N   | TYR H | 79 | 39.157 | 12.749 | -37.458 | 1.00 | 13.51 | N |
| ATOM | 12191 | CA  | TYR H | 79 | 38.953 | 13.644 | -36.330 | 1.00 | 14.46 | C |
| ATOM | 12192 | C   | TYR H | 79 | 38.682 | 15.021 | -36.850 | 1.00 | 12.24 | C |
| ATOM | 12193 | O   | TYR H | 79 | 39.182 | 15.371 | -37.883 | 1.00 | 10.28 | O |
| ATOM | 12194 | CB  | TYR H | 79 | 40.202 | 13.740 | -35.473 | 1.00 | 16.66 | C |
| ATOM | 12195 | CG  | TYR H | 79 | 40.621 | 12.419 | -34.846 | 1.00 | 18.46 | C |
| ATOM | 12196 | CD1 | TYR H | 79 | 40.077 | 12.002 | -33.632 | 1.00 | 18.41 | C |
| ATOM | 12197 | CD2 | TYR H | 79 | 41.549 | 11.621 | -35.452 | 1.00 | 19.27 | C |
| ATOM | 12198 | CE1 | TYR H | 79 | 40.464 | 10.794 | -33.048 | 1.00 | 19.35 | C |
| ATOM | 12199 | CE2 | TYR H | 79 | 41.946 | 10.387 | -34.873 | 1.00 | 19.82 | C |
| ATOM | 12200 | CZ  | TYR H | 79 | 41.396 | 9.997  | -33.679 | 1.00 | 20.22 | C |
| ATOM | 12201 | OH  | TYR H | 79 | 41.773 | 8.772  | -33.112 | 1.00 | 23.09 | O |
| ATOM | 12202 | N   | LEU H | 80 | 37.940 | 15.777 | -36.080 | 1.00 | 11.64 | N |
| ATOM | 12203 | CA  | LEU H | 80 | 37.891 | 17.213 | -36.265 | 1.00 | 12.32 | C |
| ATOM | 12204 | C   | LEU H | 80 | 38.059 | 17.876 | -34.919 | 1.00 | 13.27 | C |
| ATOM | 12205 | O   | LEU H | 80 | 37.252 | 17.671 | -34.011 | 1.00 | 15.28 | O |
| ATOM | 12206 | CB  | LEU H | 80 | 36.597 | 17.608 | -36.954 | 1.00 | 12.44 | C |
| ATOM | 12207 | CG  | LEU H | 80 | 36.538 | 19.110 | -37.356 | 1.00 | 9.84  | C |
| ATOM | 12208 | CD1 | LEU H | 80 | 37.541 | 19.390 | -38.472 | 1.00 | 13.29 | C |

| ATOM | 12209 | CD2 | LEU | H | 80 | 35.163 | 19.470 | -37.765 | 1.00 | 13.10 | C |
|------|-------|-----|-----|---|----|--------|--------|---------|------|-------|---|
| ATOM | 12210 | N | GLN | H | 81 | 39.146 | 18.639 | -34.784 | 1.00 | 15.33 | N |
| ATOM | 12211 | CA | GLN | H | 81 | 39.424 | 19.421 | -33.612 | 1.00 | 15.03 | C |
| ATOM | 12212 | C | GLN | H | 81 | 38.912 | 20.824 | -33.894 | 1.00 | 15.11 | C |
| ATOM | 12213 | O | GLN | H | 81 | 39.315 | 21.440 | -34.888 | 1.00 | 15.07 | O |
| ATOM | 12214 | CB | GLN | H | 81 | 40.926 | 19.464 | -33.330 | 1.00 | 14.41 | C |
| ATOM | 12215 | CG | GLN | H | 81 | 41.325 | 20.378 | -32.175 | 1.00 | 17.45 | C |
| ATOM | 12216 | CD | GLN | H | 81 | 40.842 | 19.886 | -30.820 | 1.00 | 17.69 | C |
| ATOM | 12217 | OE1 | GLN | H | 81 | 40.973 | 18.695 | -30.482 | 1.00 | 19.57 | O |
| ATOM | 12218 | NE2 | GLN | H | 81 | 40.237 | 20.789 | -30.052 | 1.00 | 18.67 | N |
| ATOM | 12219 | N | MET | H | 82 | 38.041 | 21.312 | -33.017 | 1.00 | 14.71 | N |
| ATOM | 12220 | CA | MET | H | 82 | 37.301 | 22.527 | -33.270 | 1.00 | 15.66 | C |
| ATOM | 12221 | C | MET | H | 82 | 37.614 | 23.525 | -32.147 | 1.00 | 14.84 | C |
| ATOM | 12222 | O | MET | H | 82 | 37.198 | 23.318 | -30.999 | 1.00 | 14.20 | O |
| ATOM | 12223 | CB | MET | H | 82 | 35.806 | 22.190 | -33.330 | 1.00 | 16.58 | C |
| ATOM | 12224 | CG | MET | H | 82 | 35.394 | 21.130 | -34.383 | 1.00 | 16.78 | C |
| ATOM | 12225 | SD | MET | H | 82 | 33.725 | 20.503 | -34.057 | 1.00 | 20.22 | S |
| ATOM | 12226 | CE | MET | H | 82 | 32.854 | 21.947 | -34.501 | 1.00 | 19.62 | C |
| ATOM | 12227 | N | ASN | H | 82A | 38.320 | 24.609 | -32.450 | 1.00 | 15.63 | N |
| ATOM | 12228 | CA | ASN | H | 82A | 38.788 | 25.508 | -31.399 | 1.00 | 15.78 | C |
| ATOM | 12229 | C | ASN | H | 82A | 38.212 | 26.911 | -31.501 | 1.00 | 16.30 | C |
| ATOM | 12230 | O | ASN | H | 82A | 37.883 | 27.372 | -32.595 | 1.00 | 16.93 | O |
| ATOM | 12231 | CB | ASN | H | 82A | 40.302 | 25.680 | -31.458 | 1.00 | 17.53 | C |
| ATOM | 12232 | CG | ASN | H | 82A | 41.052 | 24.403 | -31.261 | 1.00 | 18.62 | C |
| ATOM | 12233 | OD1 | ASN | H | 82A | 40.646 | 23.534 | -30.498 | 1.00 | 21.30 | O |
| ATOM | 12234 | ND2 | ASN | H | 82A | 42.158 | 24.276 | -31.962 | 1.00 | 22.59 | N |
| ATOM | 12235 | N | ASN | H | 82B | 38.107 | 27.553 | -30.344 | 1.00 | 14.29 | N |
| ATOM | 12236 | CA | ASN | H | 82B | 37.638 | 28.942 | -30.228 | 1.00 | 14.18 | C |
| ATOM | 12237 | C | ASN | H | 82B | 36.293 | 29.112 | -30.929 | 1.00 | 14.00 | C |
| ATOM | 12238 | O | ASN | H | 82B | 36.134 | 29.944 | -31.816 | 1.00 | 14.18 | O |
| ATOM | 12239 | CB | ASN | H | 82B | 38.679 | 29.838 | -30.836 | 1.00 | 15.10 | C |
| ATOM | 12240 | CG | ASN | H | 82B | 38.507 | 31.285 | -30.417 | 1.00 | 17.80 | C |
| ATOM | 12241 | OD1 | ASN | H | 82B | 39.106 | 32.189 | -31.022 | 1.00 | 23.80 | O |
| ATOM | 12242 | ND2 | ASN | H | 82B | 37.720 | 31.511 | -29.385 | 1.00 | 13.24 | N |
| ATOM | 12243 | N | LEU | H | 82C | 35.347 | 28.232 | -30.574 | 1.00 | 13.68 | N |
| ATOM | 12244 | CA | LEU | H | 82C | 34.087 | 28.116 | -31.287 | 1.00 | 14.25 | C |
| ATOM | 12245 | C | LEU | H | 82C | 33.143 | 29.288 | -31.082 | 1.00 | 14.73 | C |
| ATOM | 12246 | O | LEU | H | 82C | 33.236 | 30.028 | -30.089 | 1.00 | 14.18 | O |
| ATOM | 12247 | CB | LEU | H | 82C | 33.386 | 26.809 | -30.879 | 1.00 | 12.60 | C |
| ATOM | 12248 | CG | LEU | H | 82C | 34.071 | 25.504 | -31.313 | 1.00 | 14.01 | C |
| ATOM | 12249 | CD1 | LEU | H | 82C | 33.524 | 24.333 | -30.587 | 1.00 | 13.66 | C |
| ATOM | 12250 | CD2 | LEU | H | 82C | 33.935 | 25.277 | -32.811 | 1.00 | 16.98 | C |
| ATOM | 12251 | N | ARG | H | 83 | 32.204 | 29.389 | -32.023 | 1.00 | 16.13 | N |
| ATOM | 12252 | CA | ARG | H | 83 | 31.264 | 30.475 | -32.137 | 1.00 | 16.00 | C |
| ATOM | 12253 | C | ARG | H | 83 | 29.864 | 29.884 | -32.126 | 1.00 | 16.98 | C |
| ATOM | 12254 | O | ARG | H | 83 | 29.657 | 28.712 | -32.469 | 1.00 | 15.29 | O |
| ATOM | 12255 | CB | ARG | H | 83 | 31.481 | 31.251 | -33.446 | 1.00 | 16.99 | C |
| ATOM | 12256 | CG | ARG | H | 83 | 32.854 | 31.895 | -33.608 | 1.00 | 16.15 | C |
| ATOM | 12257 | CD | ARG | H | 83 | 33.222 | 32.110 | -35.058 | 1.00 | 17.95 | C |
| ATOM | 12258 | NE | ARG | H | 83 | 33.359 | 30.816 | -35.786 | 1.00 | 21.17 | N |
| ATOM | 12259 | CZ | ARG | H | 83 | 33.787 | 30.725 | -37.049 | 1.00 | 19.69 | C |
| ATOM | 12260 | NH1 | ARG | H | 83 | 34.143 | 31.818 | -37.725 | 1.00 | 19.55 | N |
| ATOM | 12261 | NH2 | ARG | H | 83 | 33.839 | 29.542 | -37.650 | 1.00 | 19.26 | N |
| ATOM | 12262 | N | THR | H | 84 | 28.877 | 30.682 | -31.757 | 1.00 | 16.83 | N |
| ATOM | 12263 | CA | THR | H | 84 | 27.504 | 30.176 | -31.831 | 1.00 | 19.00 | C |
| ATOM | 12264 | C | THR | H | 84 | 27.100 | 29.664 | -33.242 | 1.00 | 17.68 | C |
| ATOM | 12265 | O | THR | H | 84 | 26.373 | 28.671 | -33.366 | 1.00 | 21.06 | O |
| ATOM | 12266 | CB | THR | H | 84 | 26.514 | 31.178 | -31.201 | 1.00 | 19.97 | C |
| ATOM | 12267 | OG1 | THR | H | 84 | 26.553 | 32.442 | -31.871 | 1.00 | 24.89 | O |
| ATOM | 12268 | CG2 | THR | H | 84 | 26.894 | 31.360 | -29.724 | 1.00 | 20.07 | C |
| ATOM | 12269 | N | GLU | H | 85 | 27.661 | 30.268 | -34.284 | 1.00 | 17.61 | N |
| ATOM | 12270 | CA | GLU | H | 85 | 27.393 | 29.918 | -35.666 | 1.00 | 18.63 | C |
| ATOM | 12271 | C | GLU | H | 85 | 27.940 | 28.553 | -36.042 | 1.00 | 17.25 | C |
| ATOM | 12272 | O | GLU | H | 85 | 27.638 | 28.037 | -37.126 | 1.00 | 18.57 | O |
| ATOM | 12273 | CB | GLU | H | 85 | 27.967 | 30.980 | -36.622 | 1.00 | 20.43 | C |

```
ATOM  12274  CG   GLU  H  85     29.472  30.998 -36.707  1.00 22.51          C
ATOM  12275  CD   GLU  H  85     30.032  32.323 -37.224  1.00 25.10          C
ATOM  12276  OE1  GLU  H  85     29.786  33.359 -36.561  1.00 32.48          O
ATOM  12277  OE2  GLU  H  85     30.722  32.322 -38.277  1.00 31.24          O
ATOM  12278  N    ASP  H  86     28.760  27.981 -35.175  1.00 14.42          N
ATOM  12279  CA   ASP  H  86     29.336  26.667 -35.452  1.00 14.45          C
ATOM  12280  C    ASP  H  86     28.452  25.521 -34.963  1.00 12.36          C
ATOM  12281  O    ASP  H  86     28.758  24.316 -35.212  1.00 14.36          O
ATOM  12282  CB   ASP  H  86     30.717  26.565 -34.852  1.00 11.87          C
ATOM  12283  CG   ASP  H  86     31.676  27.564 -35.454  1.00 14.74          C
ATOM  12284  OD1  ASP  H  86     31.739  27.647 -36.717  1.00 14.79          O
ATOM  12285  OD2  ASP  H  86     32.381  28.234 -34.664  1.00 13.79          O
ATOM  12286  N    THR  H  87     27.345  25.875 -34.315  1.00 14.01          N
ATOM  12287  CA   THR  H  87     26.375  24.908 -33.864  1.00 13.31          C
ATOM  12288  C    THR  H  87     25.813  24.145 -35.079  1.00  8.98          C
ATOM  12289  O    THR  H  87     25.448  24.724 -36.084  1.00  8.71          O
ATOM  12290  CB   THR  H  87     25.266  25.643 -33.083  1.00 12.87          C
ATOM  12291  OG1  THR  H  87     25.799  26.135 -31.875  1.00 11.78          O
ATOM  12292  CG2  THR  H  87     24.069  24.742 -32.768  1.00 12.70          C
ATOM  12293  N    GLY  H  88     25.715  22.808 -35.039  1.00  8.86          N
ATOM  12294  CA   GLY  H  88     25.173  22.190 -36.163  1.00  6.50          C
ATOM  12295  C    GLY  H  88     25.293  20.688 -35.950  1.00  7.68          C
ATOM  12296  O    GLY  H  88     25.858  20.277 -34.961  1.00  8.08          O
ATOM  12297  N    ILE  H  89     24.760  19.954 -36.896  1.00  8.99          N
ATOM  12298  CA   ILE  H  89     24.989  18.482 -36.984  1.00  9.15          C
ATOM  12299  C    ILE  H  89     26.197  18.316 -37.890  1.00  9.59          C
ATOM  12300  O    ILE  H  89     26.210  18.812 -39.021  1.00 10.17          O
ATOM  12301  CB   ILE  H  89     23.770  17.752 -37.567  1.00  9.22          C
ATOM  12302  CG1  ILE  H  89     22.514  17.941 -36.724  1.00 13.43          C
ATOM  12303  CG2  ILE  H  89     23.994  16.170 -37.663  1.00 10.43          C
ATOM  12304  CD1  ILE  H  89     22.666  17.519 -35.305  1.00 17.85          C
ATOM  12305  N    TYR  H  90     27.227  17.603 -37.404  1.00 11.40          N
ATOM  12306  CA   TYR  H  90     28.467  17.395 -38.144  1.00  9.24          C
ATOM  12307  C    TYR  H  90     28.510  15.982 -38.700  1.00 11.97          C
ATOM  12308  O    TYR  H  90     28.461  15.057 -37.922  1.00 10.37          O
ATOM  12309  CB   TYR  H  90     29.675  17.637 -37.232  1.00  8.92          C
ATOM  12310  CG   TYR  H  90     29.956  19.164 -37.079  1.00  7.15          C
ATOM  12311  CD1  TYR  H  90     29.083  19.940 -36.355  1.00  9.21          C
ATOM  12312  CD2  TYR  H  90     31.038  19.769 -37.686  1.00 11.93          C
ATOM  12313  CE1  TYR  H  90     29.265  21.321 -36.215  1.00 11.22          C
ATOM  12314  CE2  TYR  H  90     31.250  21.172 -37.535  1.00  9.19          C
ATOM  12315  CZ   TYR  H  90     30.349  21.921 -36.803  1.00 11.27          C
ATOM  12316  OH   TYR  H  90     30.582  23.328 -36.663  1.00  8.45          O
ATOM  12317  N    TYR  H  91     28.617  15.856 -40.018  1.00 13.19          N
ATOM  12318  CA   TYR  H  91     28.647  14.580 -40.718  1.00 13.17          C
ATOM  12319  C    TYR  H  91     30.034  14.276 -41.223  1.00 14.78          C
ATOM  12320  O    TYR  H  91     30.693  15.144 -41.768  1.00 13.55          O
ATOM  12321  CB   TYR  H  91     27.738  14.611 -41.949  1.00 14.58          C
ATOM  12322  CG   TYR  H  91     26.266  14.733 -41.689  1.00 14.22          C
ATOM  12323  CD1  TYR  H  91     25.477  13.615 -41.393  1.00 14.57          C
ATOM  12324  CD2  TYR  H  91     25.634  15.974 -41.761  1.00 15.57          C
ATOM  12325  CE1  TYR  H  91     24.128  13.743 -41.158  1.00 15.74          C
ATOM  12326  CE2  TYR  H  91     24.290  16.091 -41.520  1.00 15.63          C
ATOM  12327  CZ   TYR  H  91     23.537  14.988 -41.225  1.00 16.11          C
ATOM  12328  OH   TYR  H  91     22.181  15.149 -41.026  1.00 18.92          O
ATOM  12329  N    CYS  H  92     30.476  13.011 -41.105  1.00 14.31          N
ATOM  12330  CA   CYS  H  92     31.596  12.590 -41.909  1.00 15.25          C
ATOM  12331  C    CYS  H  92     31.044  12.348 -43.294  1.00 14.04          C
ATOM  12332  O    CYS  H  92     29.968  11.787 -43.436  1.00 15.37          O
ATOM  12333  CB   CYS  H  92     32.278  11.307 -41.360  1.00 18.90          C
ATOM  12334  SG   CYS  H  92     33.385  11.748 -40.038  1.00 25.85          S
ATOM  12335  N    PHE  H  93     31.759  12.840 -44.302  1.00 13.69          N
ATOM  12336  CA   PHE  H  93     31.367  12.687 -45.707  1.00 14.30          C
ATOM  12337  C    PHE  H  93     32.598  12.249 -46.501  1.00 14.02          C
ATOM  12338  O    PHE  H  93     33.613  12.953 -46.526  1.00 14.79          O
```

| ATOM | 12339 | CB | PHE | H | 93 | 30.760 | 14.018 | -46.207 | 1.00 | 14.24 | C |
|------|-------|-----|-----|---|-----|--------|--------|---------|------|-------|---|
| ATOM | 12340 | CG | PHE | H | 93 | 30.490 | 14.092 | -47.695 | 1.00 | 13.79 | C |
| ATOM | 12341 | CD1 | PHE | H | 93 | 29.667 | 13.182 | -48.335 | 1.00 | 13.99 | C |
| ATOM | 12342 | CD2 | PHE | H | 93 | 31.002 | 15.155 | -48.435 | 1.00 | 14.98 | C |
| ATOM | 12343 | CE1 | PHE | H | 93 | 29.410 | 13.304 | -49.698 | 1.00 | 14.23 | C |
| ATOM | 12344 | CE2 | PHE | H | 93 | 30.760 | 15.282 | -49.800 | 1.00 | 13.10 | C |
| ATOM | 12345 | CZ | PHE | H | 93 | 29.954 | 14.354 | -50.428 | 1.00 | 14.76 | C |
| ATOM | 12346 | N | LEU | H | 94 | 32.554 | 11.030 | -47.053 | 1.00 | 13.65 | N |
| ATOM | 12347 | CA | LEU | H | 94 | 33.599 | 10.540 | -47.952 | 1.00 | 12.96 | C |
| ATOM | 12348 | C | LEU | H | 94 | 33.217 | 10.991 | -49.342 | 1.00 | 13.39 | C |
| ATOM | 12349 | O | LEU | H | 94 | 32.345 | 10.432 | -49.959 | 1.00 | 13.25 | O |
| ATOM | 12350 | CB | LEU | H | 94 | 33.722 | 8.986 | -47.865 | 1.00 | 13.02 | C |
| ATOM | 12351 | CG | LEU | H | 94 | 34.364 | 8.499 | -46.571 | 1.00 | 11.20 | C |
| ATOM | 12352 | CD1 | LEU | H | 94 | 33.612 | 8.858 | -45.335 | 1.00 | 11.98 | C |
| ATOM | 12353 | CD2 | LEU | H | 94 | 34.489 | 6.926 | -46.583 | 1.00 | 13.88 | C |
| ATOM | 12354 | N | PRO | H | 95 | 33.813 | 12.101 | -49.829 | 1.00 | 12.40 | N |
| ATOM | 12355 | CA | PRO | H | 95 | 33.334 | 12.607 | -51.093 | 1.00 | 13.84 | C |
| ATOM | 12356 | C | PRO | H | 95 | 33.514 | 11.549 | -52.167 | 1.00 | 15.44 | C |
| ATOM | 12357 | O | PRO | H | 95 | 34.587 | 10.934 | -52.194 | 1.00 | 15.72 | O |
| ATOM | 12358 | CB | PRO | H | 95 | 34.277 | 13.776 | -51.341 | 1.00 | 12.72 | C |
| ATOM | 12359 | CG | PRO | H | 95 | 34.668 | 14.187 | -50.000 | 1.00 | 13.91 | C |
| ATOM | 12360 | CD | PRO | H | 95 | 34.899 | 12.921 | -49.312 | 1.00 | 13.40 | C |
| ATOM | 12361 | N | MET | H | 96 | 32.518 | 11.311 | -53.026 | 1.00 | 14.92 | N |
| ATOM | 12362 | CA | MET | H | 96 | 31.168 | 11.949 | -52.994 | 1.00 | 15.77 | C |
| ATOM | 12363 | C | MET | H | 96 | 30.010 | 11.020 | -52.560 | 1.00 | 16.12 | C |
| ATOM | 12364 | O | MET | H | 96 | 28.795 | 11.366 | -52.638 | 1.00 | 14.87 | O |
| ATOM | 12365 | CB | MET | H | 96 | 30.857 | 12.464 | -54.398 | 1.00 | 15.08 | C |
| ATOM | 12366 | CG | MET | H | 96 | 31.799 | 13.481 | -54.928 | 1.00 | 15.60 | C |
| ATOM | 12367 | SD | MET | H | 96 | 31.670 | 15.108 | -54.143 | 1.00 | 15.41 | S |
| ATOM | 12368 | CE | MET | H | 96 | 30.053 | 15.726 | -54.656 | 1.00 | 14.32 | C |
| ATOM | 12369 | N | ASP | H | 101 | 30.369 | 9.823 | -52.122 | 1.00 | 16.61 | N |
| ATOM | 12370 | CA | ASP | H | 101 | 29.451 | 8.695 | -52.120 | 1.00 | 17.82 | C |
| ATOM | 12371 | C | ASP | H | 101 | 28.842 | 8.278 | -50.775 | 1.00 | 16.80 | C |
| ATOM | 12372 | O | ASP | H | 101 | 27.820 | 7.602 | -50.774 | 1.00 | 19.01 | O |
| ATOM | 12373 | CB | ASP | H | 101 | 30.188 | 7.467 | -52.676 | 1.00 | 18.30 | C |
| ATOM | 12374 | CG | ASP | H | 101 | 30.662 | 7.638 | -54.125 | 1.00 | 23.61 | C |
| ATOM | 12375 | OD1 | ASP | H | 101 | 30.096 | 8.431 | -54.904 | 1.00 | 27.07 | O |
| ATOM | 12376 | OD2 | ASP | H | 101 | 31.614 | 6.930 | -54.515 | 1.00 | 27.47 | O |
| ATOM | 12377 | N | TYR | H | 102 | 29.482 | 8.623 | -49.655 | 1.00 | 16.70 | N |
| ATOM | 12378 | CA | TYR | H | 102 | 29.131 | 8.090 | -48.333 | 1.00 | 15.69 | C |
| ATOM | 12379 | C | TYR | H | 102 | 29.021 | 9.183 | -47.277 | 1.00 | 15.25 | C |
| ATOM | 12380 | O | TYR | H | 102 | 29.894 | 10.055 | -47.170 | 1.00 | 16.62 | O |
| ATOM | 12381 | CB | TYR | H | 102 | 30.169 | 7.066 | -47.861 | 1.00 | 17.59 | C |
| ATOM | 12382 | CG | TYR | H | 102 | 30.472 | 5.959 | -48.855 | 1.00 | 17.40 | C |
| ATOM | 12383 | CD1 | TYR | H | 102 | 29.561 | 4.927 | -49.079 | 1.00 | 19.97 | C |
| ATOM | 12384 | CD2 | TYR | H | 102 | 31.679 | 5.927 | -49.539 | 1.00 | 20.32 | C |
| ATOM | 12385 | CE1 | TYR | H | 102 | 29.836 | 3.890 | -50.001 | 1.00 | 18.88 | C |
| ATOM | 12386 | CE2 | TYR | H | 102 | 31.967 | 4.888 | -50.464 | 1.00 | 18.67 | C |
| ATOM | 12387 | CZ | TYR | H | 102 | 31.033 | 3.878 | -50.682 | 1.00 | 19.60 | C |
| ATOM | 12388 | OH | TYR | H | 102 | 31.300 | 2.869 | -51.595 | 1.00 | 19.78 | O |
| ATOM | 12389 | N | TRP | H | 103 | 27.940 | 9.113 | -46.516 | 1.00 | 14.79 | N |
| ATOM | 12390 | CA | TRP | H | 103 | 27.664 | 9.978 | -45.387 | 1.00 | 15.69 | C |
| ATOM | 12391 | C | TRP | H | 103 | 27.463 | 9.143 | -44.135 | 1.00 | 17.46 | C |
| ATOM | 12392 | O | TRP | H | 103 | 26.823 | 8.100 | -44.180 | 1.00 | 17.73 | O |
| ATOM | 12393 | CB | TRP | H | 103 | 26.344 | 10.715 | -45.585 | 1.00 | 15.05 | C |
| ATOM | 12394 | CG | TRP | H | 103 | 26.315 | 11.689 | -46.681 | 1.00 | 13.23 | C |
| ATOM | 12395 | CD1 | TRP | H | 103 | 26.146 | 11.438 | -48.011 | 1.00 | 12.21 | C |
| ATOM | 12396 | CD2 | TRP | H | 103 | 26.332 | 13.121 | -46.542 | 1.00 | 11.60 | C |
| ATOM | 12397 | NE1 | TRP | H | 103 | 26.107 | 12.619 | -48.718 | 1.00 | 13.75 | N |
| ATOM | 12398 | CE2 | TRP | H | 103 | 26.232 | 13.663 | -47.844 | 1.00 | 12.36 | C |
| ATOM | 12399 | CE3 | TRP | H | 103 | 26.490 | 13.984 | -45.461 | 1.00 | 12.46 | C |
| ATOM | 12400 | CZ2 | TRP | H | 103 | 26.269 | 15.049 | -48.090 | 1.00 | 12.63 | C |
| ATOM | 12401 | CZ3 | TRP | H | 103 | 26.514 | 15.360 | -45.715 | 1.00 | 13.22 | C |
| ATOM | 12402 | CH2 | TRP | H | 103 | 26.405 | 15.859 | -47.003 | 1.00 | 12.86 | C |
| ATOM | 12403 | N | GLY | H | 104 | 27.954 | 9.662 | -43.018 | 1.00 | 17.00 | N |

```
ATOM  12404  CA   GLY H 104    27.622    9.140 -41.707  1.00 16.14        C
ATOM  12405  C    GLY H 104    26.251    9.531 -41.176  1.00 15.97        C
ATOM  12406  O    GLY H 104    25.366    9.940 -41.930  1.00 15.33        O
ATOM  12407  N    GLN H 105    26.094    9.354 -39.866  1.00 14.94        N
ATOM  12408  CA   GLN H 105    24.840    9.506 -39.165  1.00 16.60        C
ATOM  12409  C    GLN H 105    24.698   10.922 -38.601  1.00 13.74        C
ATOM  12410  O    GLN H 105    23.623   11.309 -38.187  1.00 15.65        O
ATOM  12411  CB   GLN H 105    24.738    8.570 -37.955  1.00 19.34        C
ATOM  12412  CG   GLN H 105    25.506    7.244 -38.041  1.00 25.16        C
ATOM  12413  CD   GLN H 105    25.144    6.414 -39.259  1.00 29.32        C
ATOM  12414  OE1  GLN H 105    24.063    6.585 -39.854  1.00 34.94        O
ATOM  12415  NE2  GLN H 105    26.044    5.493 -39.638  1.00 32.39        N
ATOM  12416  N    GLY H 106    25.802   11.617 -38.473  1.00 14.77        N
ATOM  12417  CA   GLY H 106    25.820   12.951 -37.864  1.00 14.25        C
ATOM  12418  C    GLY H 106    26.004   12.966 -36.351  1.00 14.50        C
ATOM  12419  O    GLY H 106    25.534   12.087 -35.652  1.00 18.30        O
ATOM  12420  N    THR H 107    26.698   13.979 -35.821  1.00 13.04        N
ATOM  12421  CA   THR H 107    26.801   14.156 -34.372  1.00 13.22        C
ATOM  12422  C    THR H 107    26.526   15.620 -34.087  1.00 11.88        C
ATOM  12423  O    THR H 107    27.042   16.442 -34.811  1.00 13.60        O
ATOM  12424  CB   THR H 107    28.160   13.656 -33.782  1.00 14.10        C
ATOM  12425  OG1  THR H 107    28.077   13.653 -32.356  1.00 15.14        O
ATOM  12426  CG2  THR H 107    29.350   14.490 -34.246  1.00 13.44        C
ATOM  12427  N    SER H 108    25.692   15.911 -33.089  1.00  9.83        N
ATOM  12428  CA   SER H 108    25.292   17.295 -32.791  1.00 12.51        C
ATOM  12429  C    SER H 108    26.318   18.019 -31.935  1.00 11.64        C
ATOM  12430  O    SER H 108    26.804   17.507 -30.918  1.00  9.92        O
ATOM  12431  CB   SER H 108    23.940   17.298 -32.081  1.00 13.65        C
ATOM  12432  OG   SER H 108    23.399   18.629 -32.022  1.00 17.24        O
ATOM  12433  N    VAL H 109    26.617   19.265 -32.315  1.00 11.75        N
ATOM  12434  CA   VAL H 109    27.557   20.067 -31.622  1.00 11.90        C
ATOM  12435  C    VAL H 109    26.764   21.358 -31.331  1.00 10.97        C
ATOM  12436  O    VAL H 109    26.210   21.905 -32.239  1.00 10.88        O
ATOM  12437  CB   VAL H 109    28.792   20.430 -32.511  1.00 13.52        C
ATOM  12438  CG1  VAL H 109    29.618   21.573 -31.837  1.00 12.93        C
ATOM  12439  CG2  VAL H 109    29.640   19.228 -32.770  1.00 15.16        C
ATOM  12440  N    THR H 110    26.616   21.714 -30.066  1.00 11.68        N
ATOM  12441  CA   THR H 110    25.999   22.986 -29.647  1.00 12.79        C
ATOM  12442  C    THR H 110    27.070   23.887 -29.013  1.00 12.02        C
ATOM  12443  O    THR H 110    27.818   23.468 -28.125  1.00 13.42        O
ATOM  12444  CB   THR H 110    24.815   22.745 -28.669  1.00 12.05        C
ATOM  12445  OG1  THR H 110    23.791   21.997 -29.341  1.00 14.88        O
ATOM  12446  CG2  THR H 110    24.207   24.083 -28.194  1.00 13.08        C
ATOM  12447  N    VAL H 111    27.134   25.123 -29.497  1.00 13.80        N
ATOM  12448  CA   VAL H 111    27.980   26.154 -28.929  1.00 12.10        C
ATOM  12449  C    VAL H 111    27.029   27.191 -28.332  1.00 13.32        C
ATOM  12450  O    VAL H 111    26.271   27.840 -29.065  1.00 14.87        O
ATOM  12451  CB   VAL H 111    28.898   26.826 -30.001  1.00 12.18        C
ATOM  12452  CG1  VAL H 111    29.814   27.840 -29.371  1.00 12.63        C
ATOM  12453  CG2  VAL H 111    29.737   25.820 -30.722  1.00 11.90        C
ATOM  12454  N    SER H 112    27.025   27.262 -27.003  1.00 13.75        N
ATOM  12455  CA   SER H 112    26.013   27.981 -26.244  1.00 15.57        C
ATOM  12456  C    SER H 112    26.516   28.310 -24.848  1.00 15.61        C
ATOM  12457  O    SER H 112    27.292   27.558 -24.263  1.00 13.69        O
ATOM  12458  CB   SER H 112    24.717   27.179 -26.169  1.00 17.43        C
ATOM  12459  OG   SER H 112    23.781   27.829 -25.312  1.00 15.54        O
ATOM  12460  N    SER H 113    26.130   29.490 -24.346  1.00 16.24        N
ATOM  12461  CA   SER H 113    26.378   29.845 -22.944  1.00 17.27        C
ATOM  12462  C    SER H 113    25.307   29.320 -21.970  1.00 17.59        C
ATOM  12463  O    SER H 113    25.445   29.463 -20.754  1.00 20.05        O
ATOM  12464  CB   SER H 113    26.513   31.374 -22.838  1.00 17.60        C
ATOM  12465  OG   SER H 113    25.323   32.002 -23.282  1.00 21.26        O
ATOM  12466  N    ALA H 114    24.258   28.699 -22.493  1.00 16.46        N
ATOM  12467  CA   ALA H 114    23.179   28.178 -21.671  1.00 17.23        C
ATOM  12468  C    ALA H 114    23.581   26.809 -21.171  1.00 16.90        C
```

```
ATOM   12469  O    ALA H 114      24.489  26.181 -21.725  1.00 16.31           O
ATOM   12470  CB   ALA H 114      21.883  28.084 -22.477  1.00 15.89           C
ATOM   12471  N    LYS H 115      22.897  26.336 -20.154  1.00 18.19           N
ATOM   12472  CA   LYS H 115      23.320  25.123 -19.465  1.00 19.29           C
ATOM   12473  C    LYS H 115      22.550  23.953 -20.024  1.00 19.59           C
ATOM   12474  O    LYS H 115      21.388  24.092 -20.401  1.00 21.49           O
ATOM   12475  CB   LYS H 115      23.053  25.270 -17.967  1.00 21.83           C
ATOM   12476  CG   LYS H 115      23.636  26.575 -17.393  1.00 25.76           C
ATOM   12477  CD   LYS H 115      22.584  27.681 -17.060  1.00 27.86           C
ATOM   12478  CE   LYS H 115      22.228  28.637 -18.220  1.00 29.25           C
ATOM   12479  NZ   LYS H 115      21.002  28.195 -18.937  1.00 28.85           N
ATOM   12480  N    THR H 116      23.210  22.795 -20.050  1.00 17.80           N
ATOM   12481  CA   THR H 116      22.590  21.544 -20.413  1.00 16.63           C
ATOM   12482  C    THR H 116      21.594  21.226 -19.316  1.00 15.90           C
ATOM   12483  O    THR H 116      21.909  21.354 -18.166  1.00 13.46           O
ATOM   12484  CB   THR H 116      23.642  20.450 -20.528  1.00 16.64           C
ATOM   12485  OG1  THR H 116      24.434  20.730 -21.676  1.00 15.34           O
ATOM   12486  CG2  THR H 116      22.983  19.052 -20.677  1.00 17.88           C
ATOM   12487  N    THR H 117      20.366  20.895 -19.696  1.00 15.72           N
ATOM   12488  CA   THR H 117      19.285  20.581 -18.754  1.00 15.60           C
ATOM   12489  C    THR H 117      18.648  19.291 -19.252  1.00 15.75           C
ATOM   12490  O    THR H 117      18.238  19.239 -20.417  1.00 15.63           O
ATOM   12491  CB   THR H 117      18.216  21.668 -18.812  1.00 15.67           C
ATOM   12492  OG1  THR H 117      18.846  22.935 -18.644  1.00 15.52           O
ATOM   12493  CG2  THR H 117      17.197  21.500 -17.707  1.00 15.41           C
ATOM   12494  N    PRO H 118      18.580  18.257 -18.398  1.00 15.31           N
ATOM   12495  CA   PRO H 118      17.918  17.060 -18.867  1.00 16.85           C
ATOM   12496  C    PRO H 118      16.412  17.204 -18.827  1.00 15.89           C
ATOM   12497  O    PRO H 118      15.882  18.091 -18.128  1.00 16.45           O
ATOM   12498  CB   PRO H 118      18.417  15.985 -17.901  1.00 16.60           C
ATOM   12499  CG   PRO H 118      18.688  16.669 -16.677  1.00 18.02           C
ATOM   12500  CD   PRO H 118      19.099  18.097 -17.039  1.00 15.32           C
ATOM   12501  N    PRO H 119      15.711  16.340 -19.575  1.00 16.31           N
ATOM   12502  CA   PRO H 119      14.271  16.402 -19.638  1.00 16.25           C
ATOM   12503  C    PRO H 119      13.604  15.793 -18.426  1.00 16.83           C
ATOM   12504  O    PRO H 119      14.192  14.982 -17.722  1.00 17.19           O
ATOM   12505  CB   PRO H 119      13.939  15.599 -20.909  1.00 16.11           C
ATOM   12506  CG   PRO H 119      14.979  14.615 -21.015  1.00 17.87           C
ATOM   12507  CD   PRO H 119      16.245  15.281 -20.451  1.00 15.89           C
ATOM   12508  N    SER H 120      12.384  16.222 -18.174  1.00 17.54           N
ATOM   12509  CA   SER H 120      11.498  15.536 -17.258  1.00 17.91           C
ATOM   12510  C    SER H 120      10.624  14.673 -18.146  1.00 17.84           C
ATOM   12511  O    SER H 120      10.195  15.110 -19.176  1.00 17.72           O
ATOM   12512  CB   SER H 120      10.687  16.536 -16.494  1.00 19.07           C
ATOM   12513  OG   SER H 120      11.538  17.229 -15.611  1.00 20.71           O
ATOM   12514  N    VAL H 121      10.393  13.420 -17.778  1.00 19.64           N
ATOM   12515  CA   VAL H 121       9.674  12.543 -18.678  1.00 19.37           C
ATOM   12516  C    VAL H 121       8.398  12.026 -18.013  1.00 20.25           C
ATOM   12517  O    VAL H 121       8.438  11.486 -16.896  1.00 19.70           O
ATOM   12518  CB   VAL H 121      10.541  11.344 -19.083  1.00 20.36           C
ATOM   12519  CG1  VAL H 121       9.770  10.388 -19.990  1.00 21.31           C
ATOM   12520  CG2  VAL H 121      11.836  11.839 -19.751  1.00 18.32           C
ATOM   12521  N    TYR H 122       7.297  12.125 -18.734  1.00 20.23           N
ATOM   12522  CA   TYR H 122       5.994  11.822 -18.179  1.00 22.09           C
ATOM   12523  C    TYR H 122       5.175  10.939 -19.114  1.00 22.95           C
ATOM   12524  O    TYR H 122       5.176  11.124 -20.330  1.00 19.94           O
ATOM   12525  CB   TYR H 122       5.202  13.093 -17.915  1.00 22.65           C
ATOM   12526  CG   TYR H 122       5.800  14.007 -16.881  1.00 22.68           C
ATOM   12527  CD1  TYR H 122       5.955  13.594 -15.556  1.00 23.56           C
ATOM   12528  CD2  TYR H 122       6.171  15.299 -17.209  1.00 23.25           C
ATOM   12529  CE1  TYR H 122       6.506  14.434 -14.609  1.00 24.15           C
ATOM   12530  CE2  TYR H 122       6.735  16.124 -16.277  1.00 22.74           C
ATOM   12531  CZ   TYR H 122       6.884  15.698 -14.979  1.00 23.19           C
ATOM   12532  OH   TYR H 122       7.440  16.558 -14.052  1.00 24.50           O
ATOM   12533  N    PRO H 123       4.413  10.020 -18.524  1.00 24.60           N
```

```
ATOM  12534  CA  PRO H 123    3.568   9.140 -19.302  1.00 25.23       C
ATOM  12535  C   PRO H 123    2.306   9.859 -19.740  1.00 25.45       C
ATOM  12536  O   PRO H 123    1.841  10.805 -19.074  1.00 27.04       O
ATOM  12537  CB  PRO H 123    3.263   7.998 -18.330  1.00 26.21       C
ATOM  12538  CG  PRO H 123    3.329   8.637 -16.974  1.00 25.90       C
ATOM  12539  CD  PRO H 123    4.272   9.799 -17.071  1.00 26.47       C
ATOM  12540  N   LEU H 124    1.820   9.494 -20.916  1.00 25.20       N
ATOM  12541  CA  LEU H 124    0.532   9.966 -21.407  1.00 25.45       C
ATOM  12542  C   LEU H 124   -0.307   8.710 -21.600  1.00 25.82       C
ATOM  12543  O   LEU H 124   -0.083   7.932 -22.525  1.00 22.72       O
ATOM  12544  CB  LEU H 124    0.694  10.711 -22.717  1.00 26.87       C
ATOM  12545  CG  LEU H 124    1.653  11.905 -22.698  1.00 27.08       C
ATOM  12546  CD1 LEU H 124    1.809  12.498 -24.086  1.00 28.81       C
ATOM  12547  CD2 LEU H 124    1.128  12.907 -21.786  1.00 29.51       C
ATOM  12548  N   ALA H 125   -1.211   8.474 -20.657  1.00 26.72       N
ATOM  12549  CA  ALA H 125   -2.080   7.317 -20.734  1.00 27.22       C
ATOM  12550  C   ALA H 125   -3.486   7.877 -20.918  1.00 27.59       C
ATOM  12551  O   ALA H 125   -3.782   9.002 -20.472  1.00 25.03       O
ATOM  12552  CB  ALA H 125   -1.949   6.452 -19.486  1.00 27.44       C
ATOM  12553  N   PRO H 126   -4.358   7.117 -21.600  1.00 28.60       N
ATOM  12554  CA  PRO H 126   -5.698   7.664 -21.790  1.00 30.11       C
ATOM  12555  C   PRO H 126   -6.400   7.899 -20.451  1.00 30.60       C
ATOM  12556  O   PRO H 126   -6.065   7.257 -19.453  1.00 29.75       O
ATOM  12557  CB  PRO H 126   -6.423   6.597 -22.607  1.00 30.09       C
ATOM  12558  CG  PRO H 126   -5.564   5.378 -22.586  1.00 29.69       C
ATOM  12559  CD  PRO H 126   -4.188   5.765 -22.171  1.00 28.90       C
ATOM  12560  N   GLY H 127   -7.335   8.845 -20.433  1.00 31.48       N
ATOM  12561  CA  GLY H 127   -8.151   9.113 -19.245  1.00 32.52       C
ATOM  12562  C   GLY H 127   -9.382   8.212 -19.157  1.00 33.40       C
ATOM  12563  O   GLY H 127   -9.412   7.103 -19.712  1.00 35.21       O
ATOM  12564  N   SER H 136  -10.470  -0.937 -30.246  1.00 28.67       N
ATOM  12565  CA  SER H 136   -9.636  -1.906 -30.955  1.00 28.00       C
ATOM  12566  C   SER H 136   -8.136  -1.643 -30.766  1.00 26.74       C
ATOM  12567  O   SER H 136   -7.341  -2.573 -30.497  1.00 24.32       O
ATOM  12568  CB  SER H 136  -10.031  -1.869 -32.430  1.00 28.78       C
ATOM  12569  OG  SER H 136   -8.916  -1.705 -33.283  1.00 31.78       O
ATOM  12570  N   MET H 137   -7.764  -0.370 -30.920  1.00 26.59       N
ATOM  12571  CA  MET H 137   -6.393   0.095 -30.700  1.00 26.04       C
ATOM  12572  C   MET H 137   -6.383   1.136 -29.603  1.00 25.96       C
ATOM  12573  O   MET H 137   -7.393   1.806 -29.385  1.00 26.57       O
ATOM  12574  CB  MET H 137   -5.831   0.749 -31.968  1.00 27.14       C
ATOM  12575  CG  MET H 137   -5.972  -0.064 -33.259  1.00 28.09       C
ATOM  12576  SD  MET H 137   -4.965  -1.551 -33.292  1.00 31.30       S
ATOM  12577  CE  MET H 137   -3.323  -0.852 -33.319  1.00 31.25       C
ATOM  12578  N   VAL H 138   -5.238   1.277 -28.929  1.00 24.57       N
ATOM  12579  CA  VAL H 138   -5.032   2.319 -27.899  1.00 24.51       C
ATOM  12580  C   VAL H 138   -3.754   3.112 -28.259  1.00 23.93       C
ATOM  12581  O   VAL H 138   -2.770   2.522 -28.722  1.00 23.46       O
ATOM  12582  CB  VAL H 138   -4.952   1.695 -26.472  1.00 25.23       C
ATOM  12583  CG1 VAL H 138   -3.804   0.684 -26.354  1.00 26.28       C
ATOM  12584  CG2 VAL H 138   -4.815   2.756 -25.414  1.00 25.40       C
ATOM  12585  N   THR H 139   -3.807   4.441 -28.112  1.00 22.93       N
ATOM  12586  CA  THR H 139   -2.666   5.315 -28.381  1.00 21.19       C
ATOM  12587  C   THR H 139   -2.172   5.812 -27.027  1.00 20.15       C
ATOM  12588  O   THR H 139   -2.945   6.269 -26.198  1.00 18.34       O
ATOM  12589  CB  THR H 139   -3.042   6.533 -29.270  1.00 20.64       C
ATOM  12590  OG1 THR H 139   -3.380   6.096 -30.582  1.00 18.98       O
ATOM  12591  CG2 THR H 139   -1.891   7.503 -29.390  1.00 21.55       C
ATOM  12592  N   LEU H 140   -0.874   5.672 -26.806  1.00 19.48       N
ATOM  12593  CA  LEU H 140   -0.231   6.123 -25.605  1.00 20.17       C
ATOM  12594  C   LEU H 140    0.867   7.069 -26.012  1.00 20.00       C
ATOM  12595  O   LEU H 140    1.223   7.143 -27.188  1.00 21.31       O
ATOM  12596  CB  LEU H 140    0.420   4.964 -24.886  1.00 19.87       C
ATOM  12597  CG  LEU H 140   -0.438   3.699 -24.814  1.00 21.62       C
ATOM  12598  CD1 LEU H 140    0.414   2.616 -24.236  1.00 21.68       C
```

```
ATOM  12599  CD2 LEU H 140    -1.638    3.943 -23.961  1.00 21.60           C
ATOM  12600  N   GLY H 141     1.437    7.748 -25.026  1.00 21.10           N
ATOM  12601  CA  GLY H 141     2.541    8.641 -25.300  1.00 20.88           C
ATOM  12602  C   GLY H 141     3.484    8.917 -24.153  1.00 20.68           C
ATOM  12603  O   GLY H 141     3.269    8.483 -23.020  1.00 17.13           O
ATOM  12604  N   CYS H 142     4.565    9.626 -24.487  1.00 21.98           N
ATOM  12605  CA  CYS H 142     5.541   10.110 -23.513  1.00 21.92           C
ATOM  12606  C   CYS H 142     5.790   11.584 -23.792  1.00 21.02           C
ATOM  12607  O   CYS H 142     6.037   11.948 -24.922  1.00 21.27           O
ATOM  12608  CB  CYS H 142     6.840    9.307 -23.554  1.00 25.01           C
ATOM  12609  SG  CYS H 142     6.745    8.004 -22.321  1.00 35.09           S
ATOM  12610  N   LEU H 143     5.683   12.403 -22.746  1.00 19.41           N
ATOM  12611  CA  LEU H 143     5.993   13.854 -22.814  1.00 18.84           C
ATOM  12612  C   LEU H 143     7.411   14.082 -22.279  1.00 17.76           C
ATOM  12613  O   LEU H 143     7.729   13.648 -21.187  1.00 19.65           O
ATOM  12614  CB  LEU H 143     4.977   14.626 -22.001  1.00 19.01           C
ATOM  12615  CG  LEU H 143     5.135   16.142 -21.868  1.00 20.27           C
ATOM  12616  CD1 LEU H 143     5.012   16.869 -23.201  1.00 21.36           C
ATOM  12617  CD2 LEU H 143     4.107   16.631 -20.860  1.00 20.28           C
ATOM  12618  N   VAL H 144     8.252   14.732 -23.073  1.00 16.90           N
ATOM  12619  CA  VAL H 144     9.663   14.936 -22.762  1.00 16.75           C
ATOM  12620  C   VAL H 144     9.867   16.461 -22.681  1.00 17.47           C
ATOM  12621  O   VAL H 144     9.940   17.128 -23.706  1.00 18.83           O
ATOM  12622  CB  VAL H 144    10.567   14.337 -23.863  1.00 17.18           C
ATOM  12623  CG1 VAL H 144    12.029   14.375 -23.453  1.00 16.89           C
ATOM  12624  CG2 VAL H 144    10.178   12.896 -24.127  1.00 18.49           C
ATOM  12625  N   LYS H 145     9.958   16.976 -21.461  1.00 16.50           N
ATOM  12626  CA  LYS H 145     9.757   18.402 -21.208  1.00 16.22           C
ATOM  12627  C   LYS H 145    10.954   19.098 -20.586  1.00 15.46           C
ATOM  12628  O   LYS H 145    11.502   18.637 -19.589  1.00 12.88           O
ATOM  12629  CB  LYS H 145     8.567   18.524 -20.266  1.00 17.41           C
ATOM  12630  CG  LYS H 145     8.147   19.923 -19.937  1.00 17.40           C
ATOM  12631  CD  LYS H 145     6.986   19.872 -18.997  1.00 19.79           C
ATOM  12632  CE  LYS H 145     6.678   21.271 -18.448  1.00 23.99           C
ATOM  12633  NZ  LYS H 145     6.586   22.227 -19.571  1.00 25.71           N
ATOM  12634  N   GLY H 146    11.282   20.273 -21.116  1.00 14.87           N
ATOM  12635  CA  GLY H 146    12.201   21.178 -20.448  1.00 15.00           C
ATOM  12636  C   GLY H 146    13.679   20.794 -20.537  1.00 13.76           C
ATOM  12637  O   GLY H 146    14.430   20.933 -19.544  1.00 15.07           O
ATOM  12638  N   TYR H 147    14.110   20.331 -21.708  1.00 14.22           N
ATOM  12639  CA  TYR H 147    15.518   19.921 -21.899  1.00 14.27           C
ATOM  12640  C   TYR H 147    16.263   20.937 -22.800  1.00 13.60           C
ATOM  12641  O   TYR H 147    15.654   21.701 -23.550  1.00 14.12           O
ATOM  12642  CB  TYR H 147    15.652   18.487 -22.454  1.00 14.42           C
ATOM  12643  CG  TYR H 147    15.096   18.311 -23.837  1.00 14.65           C
ATOM  12644  CD1 TYR H 147    13.763   17.999 -24.029  1.00 14.48           C
ATOM  12645  CD2 TYR H 147    15.893   18.504 -24.958  1.00 13.00           C
ATOM  12646  CE1 TYR H 147    13.271   17.841 -25.278  1.00 12.78           C
ATOM  12647  CE2 TYR H 147    15.403   18.375 -26.218  1.00 12.14           C
ATOM  12648  CZ  TYR H 147    14.091   18.044 -26.385  1.00 12.03           C
ATOM  12649  OH  TYR H 147    13.596   17.948 -27.664  1.00 13.59           O
ATOM  12650  N   PHE H 148    17.588   20.959 -22.677  1.00 15.82           N
ATOM  12651  CA  PHE H 148    18.442   21.756 -23.558  1.00 15.74           C
ATOM  12652  C   PHE H 148    19.823   21.110 -23.574  1.00 15.89           C
ATOM  12653  O   PHE H 148    20.257   20.611 -22.570  1.00 15.40           O
ATOM  12654  CB  PHE H 148    18.528   23.188 -23.055  1.00 17.26           C
ATOM  12655  CG  PHE H 148    19.317   24.057 -23.955  1.00 17.34           C
ATOM  12656  CD1 PHE H 148    18.733   24.612 -25.079  1.00 17.50           C
ATOM  12657  CD2 PHE H 148    20.689   24.241 -23.747  1.00 18.38           C
ATOM  12658  CE1 PHE H 148    19.494   25.388 -25.952  1.00 17.73           C
ATOM  12659  CE2 PHE H 148    21.456   25.019 -24.629  1.00 17.50           C
ATOM  12660  CZ  PHE H 148    20.865   25.585 -25.724  1.00 16.31           C
ATOM  12661  N   PRO H 149    20.501   21.056 -24.732  1.00 15.09           N
ATOM  12662  CA  PRO H 149    20.073   21.351 -26.084  1.00 16.14           C
ATOM  12663  C   PRO H 149    19.405   20.111 -26.698  1.00 16.44           C
```

```
ATOM  12664  O    PRO H 149      19.367  19.100 -26.074  1.00 17.06           O
ATOM  12665  CB   PRO H 149      21.396  21.641 -26.793  1.00 14.37           C
ATOM  12666  CG   PRO H 149      22.402  20.766 -26.121  1.00 14.39           C
ATOM  12667  CD   PRO H 149      21.906  20.639 -24.677  1.00 15.86           C
ATOM  12668  N    GLU H 150      18.857  20.234 -27.889  1.00 18.92           N
ATOM  12669  CA   GLU H 150      18.581  19.056 -28.716  1.00 19.87           C
ATOM  12670  C    GLU H 150      19.850  18.267 -29.022  1.00 20.93           C
ATOM  12671  O    GLU H 150      20.945  18.780 -28.882  1.00 19.78           O
ATOM  12672  CB   GLU H 150      17.927  19.491 -30.003  1.00 20.31           C
ATOM  12673  CG   GLU H 150      16.545  20.030 -29.787  1.00 22.42           C
ATOM  12674  CD   GLU H 150      15.488  19.121 -30.304  1.00 24.80           C
ATOM  12675  OE1  GLU H 150      14.855  19.558 -31.264  1.00 27.43           O
ATOM  12676  OE2  GLU H 150      15.308  17.975 -29.787  1.00 27.85           O
ATOM  12677  N    PRO H 151      19.705  16.985 -29.415  1.00 21.46           N
ATOM  12678  CA   PRO H 151      18.478  16.227 -29.598  1.00 21.00           C
ATOM  12679  C    PRO H 151      18.128  15.358 -28.404  1.00 18.69           C
ATOM  12680  O    PRO H 151      18.906  15.256 -27.437  1.00 17.16           O
ATOM  12681  CB   PRO H 151      18.838  15.333 -30.780  1.00 21.32           C
ATOM  12682  CG   PRO H 151      20.297  14.954 -30.462  1.00 22.87           C
ATOM  12683  CD   PRO H 151      20.887  16.173 -29.768  1.00 21.83           C
ATOM  12684  N    VAL H 152      16.933  14.768 -28.474  1.00 19.24           N
ATOM  12685  CA   VAL H 152      16.587  13.565 -27.696  1.00 19.11           C
ATOM  12686  C    VAL H 152      16.146  12.420 -28.626  1.00 18.85           C
ATOM  12687  O    VAL H 152      15.766  12.636 -29.745  1.00 18.32           O
ATOM  12688  CB   VAL H 152      15.469  13.817 -26.665  1.00 19.57           C
ATOM  12689  CG1  VAL H 152      15.916  14.774 -25.584  1.00 19.47           C
ATOM  12690  CG2  VAL H 152      14.245  14.302 -27.315  1.00 20.36           C
ATOM  12691  N    THR H 153      16.229  11.181 -28.159  1.00 20.75           N
ATOM  12692  CA   THR H 153      15.663  10.059 -28.906  1.00 20.91           C
ATOM  12693  C    THR H 153      14.679   9.351 -28.002  1.00 20.04           C
ATOM  12694  O    THR H 153      14.968   9.159 -26.816  1.00 20.57           O
ATOM  12695  CB   THR H 153      16.730   9.040 -29.258  1.00 21.98           C
ATOM  12696  OG1  THR H 153      17.463   8.696 -28.078  1.00 25.60           O
ATOM  12697  CG2  THR H 153      17.682   9.568 -30.296  1.00 24.43           C
ATOM  12698  N    VAL H 154      13.524   8.963 -28.541  1.00 18.60           N
ATOM  12699  CA   VAL H 154      12.542   8.228 -27.777  1.00 19.61           C
ATOM  12700  C    VAL H 154      12.315   6.896 -28.481  1.00 19.79           C
ATOM  12701  O    VAL H 154      12.107   6.873 -29.693  1.00 19.51           O
ATOM  12702  CB   VAL H 154      11.218   8.966 -27.720  1.00 20.02           C
ATOM  12703  CG1  VAL H 154      10.226   8.198 -26.904  1.00 20.61           C
ATOM  12704  CG2  VAL H 154      11.423  10.377 -27.152  1.00 19.49           C
ATOM  12705  N    THR H 155      12.354   5.806 -27.722  1.00 20.74           N
ATOM  12706  CA   THR H 155      11.981   4.495 -28.266  1.00 20.49           C
ATOM  12707  C    THR H 155      10.911   3.881 -27.384  1.00 20.63           C
ATOM  12708  O    THR H 155      10.548   4.448 -26.341  1.00 19.69           O
ATOM  12709  CB   THR H 155      13.202   3.575 -28.408  1.00 20.02           C
ATOM  12710  OG1  THR H 155      13.813   3.351 -27.140  1.00 21.76           O
ATOM  12711  CG2  THR H 155      14.194   4.183 -29.361  1.00 19.47           C
ATOM  12712  N    TRP H 156      10.376   2.737 -27.817  1.00 21.09           N
ATOM  12713  CA   TRP H 156       9.326   2.041 -27.051  1.00 20.97           C
ATOM  12714  C    TRP H 156       9.723   0.592 -26.904  1.00 21.16           C
ATOM  12715  O    TRP H 156      10.133  -0.040 -27.885  1.00 19.93           O
ATOM  12716  CB   TRP H 156       7.960   2.158 -27.739  1.00 19.87           C
ATOM  12717  CG   TRP H 156       7.460   3.589 -27.770  1.00 19.27           C
ATOM  12718  CD1  TRP H 156       7.731   4.546 -28.712  1.00 18.78           C
ATOM  12719  CD2  TRP H 156       6.675   4.223 -26.767  1.00 18.97           C
ATOM  12720  NE1  TRP H 156       7.111   5.728 -28.374  1.00 16.90           N
ATOM  12721  CE2  TRP H 156       6.452   5.556 -27.187  1.00 17.88           C
ATOM  12722  CE3  TRP H 156       6.103   3.786 -25.562  1.00 20.07           C
ATOM  12723  CZ2  TRP H 156       5.718   6.463 -26.428  1.00 18.89           C
ATOM  12724  CZ3  TRP H 156       5.355   4.687 -24.816  1.00 18.78           C
ATOM  12725  CH2  TRP H 156       5.163   6.011 -25.266  1.00 19.35           C
ATOM  12726  N    ASN H 157       9.628   0.098 -25.667  1.00 22.63           N
ATOM  12727  CA   ASN H 157      10.157  -1.222 -25.300  1.00 24.61           C
ATOM  12728  C    ASN H 157      11.559  -1.432 -25.885  1.00 26.21           C
```

```
ATOM   12729  O    ASN H 157    11.826  -2.388 -26.610  1.00 27.96          O
ATOM   12730  CB   ASN H 157     9.167  -2.312 -25.726  1.00 23.58          C
ATOM   12731  CG   ASN H 157     7.891  -2.287 -24.907  1.00 24.22          C
ATOM   12732  OD1  ASN H 157     7.718  -1.440 -24.035  1.00 23.74          O
ATOM   12733  ND2  ASN H 157     6.985  -3.247 -25.171  1.00 22.66          N
ATOM   12734  N    SER H 158    12.441  -0.485 -25.567  1.00 27.40          N
ATOM   12735  CA   SER H 158    13.816  -0.486 -26.035  1.00 27.98          C
ATOM   12736  C    SER H 158    13.947  -0.721 -27.527  1.00 29.54          C
ATOM   12737  O    SER H 158    14.891  -1.388 -27.968  1.00 32.79          O
ATOM   12738  CB   SER H 158    14.637  -1.494 -25.243  1.00 29.31          C
ATOM   12739  OG   SER H 158    14.526  -1.199 -23.873  1.00 30.51          O
ATOM   12740  N    GLY H 159    13.020  -0.156 -28.304  1.00 29.24          N
ATOM   12741  CA   GLY H 159    13.044  -0.268 -29.769  1.00 28.75          C
ATOM   12742  C    GLY H 159    12.301  -1.480 -30.338  1.00 28.98          C
ATOM   12743  O    GLY H 159    12.017  -1.535 -31.531  1.00 28.19          O
ATOM   12744  N    SER H 160    11.980  -2.448 -29.488  1.00 29.23          N
ATOM   12745  CA   SER H 160    11.325  -3.675 -29.933  1.00 30.14          C
ATOM   12746  C    SER H 160     9.902  -3.392 -30.436  1.00 29.80          C
ATOM   12747  O    SER H 160     9.403  -4.064 -31.329  1.00 30.93          O
ATOM   12748  CB   SER H 160    11.313  -4.718 -28.800  1.00 30.86          C
ATOM   12749  OG   SER H 160    10.412  -4.317 -27.771  1.00 32.80          O
ATOM   12750  N    LEU H 161     9.267  -2.385 -29.862  1.00 29.34          N
ATOM   12751  CA   LEU H 161     7.963  -1.937 -30.283  1.00 28.88          C
ATOM   12752  C    LEU H 161     8.178  -0.781 -31.293  1.00 28.41          C
ATOM   12753  O    LEU H 161     8.393   0.371 -30.905  1.00 27.90          O
ATOM   12754  CB   LEU H 161     7.199  -1.498 -29.035  1.00 29.13          C
ATOM   12755  CG   LEU H 161     5.685  -1.457 -29.030  1.00 30.01          C
ATOM   12756  CD1  LEU H 161     5.230  -0.921 -27.668  1.00 30.16          C
ATOM   12757  CD2  LEU H 161     5.163  -0.595 -30.177  1.00 31.76          C
ATOM   12758  N    SER H 162     8.175  -1.109 -32.582  1.00 26.62          N
ATOM   12759  CA   SER H 162     8.529  -0.148 -33.628  1.00 27.08          C
ATOM   12760  C    SER H 162     7.391   0.311 -34.547  1.00 26.56          C
ATOM   12761  O    SER H 162     7.476   1.395 -35.139  1.00 27.55          O
ATOM   12762  CB   SER H 162     9.672  -0.708 -34.481  1.00 28.17          C
ATOM   12763  OG   SER H 162     9.410  -2.034 -34.889  1.00 29.63          O
ATOM   12764  N    SER H 163     6.348  -0.500 -34.687  1.00 25.35          N
ATOM   12765  CA   SER H 163     5.202  -0.132 -35.509  1.00 25.63          C
ATOM   12766  C    SER H 163     4.322   0.875 -34.801  1.00 22.76          C
ATOM   12767  O    SER H 163     4.135   0.844 -33.583  1.00 21.86          O
ATOM   12768  CB   SER H 163     4.339  -1.346 -35.886  1.00 26.68          C
ATOM   12769  OG   SER H 163     5.153  -2.442 -36.314  1.00 30.79          O
ATOM   12770  N    GLY H 164     3.771   1.782 -35.589  1.00 24.24          N
ATOM   12771  CA   GLY H 164     2.824   2.765 -35.084  1.00 23.94          C
ATOM   12772  C    GLY H 164     3.410   3.741 -34.101  1.00 23.74          C
ATOM   12773  O    GLY H 164     2.697   4.210 -33.200  1.00 24.64          O
ATOM   12774  N    VAL H 165     4.688   4.070 -34.310  1.00 23.93          N
ATOM   12775  CA   VAL H 165     5.435   5.007 -33.456  1.00 22.74          C
ATOM   12776  C    VAL H 165     5.802   6.234 -34.257  1.00 22.10          C
ATOM   12777  O    VAL H 165     6.179   6.134 -35.407  1.00 21.10          O
ATOM   12778  CB   VAL H 165     6.723   4.381 -32.923  1.00 22.18          C
ATOM   12779  CG1  VAL H 165     7.637   5.431 -32.282  1.00 21.79          C
ATOM   12780  CG2  VAL H 165     6.407   3.286 -31.911  1.00 21.69          C
ATOM   12781  N    HIS H 166     5.688   7.404 -33.649  1.00 22.35          N
ATOM   12782  CA   HIS H 166     6.305   8.569 -34.233  1.00 21.47          C
ATOM   12783  C    HIS H 166     6.565   9.584 -33.151  1.00 20.28          C
ATOM   12784  O    HIS H 166     5.851   9.644 -32.168  1.00 19.87          O
ATOM   12785  CB   HIS H 166     5.483   9.169 -35.384  1.00 23.15          C
ATOM   12786  CG   HIS H 166     4.095   9.579 -35.006  1.00 22.92          C
ATOM   12787  ND1  HIS H 166     2.973   8.951 -35.509  1.00 25.53          N
ATOM   12788  CD2  HIS H 166     3.645  10.554 -34.187  1.00 21.50          C
ATOM   12789  CE1  HIS H 166     1.890   9.507 -34.992  1.00 25.28          C
ATOM   12790  NE2  HIS H 166     2.269  10.481 -34.184  1.00 23.63          N
ATOM   12791  N    THR H 167     7.614  10.372 -33.341  1.00 19.93          N
ATOM   12792  CA   THR H 167     7.963  11.418 -32.390  1.00 19.39          C
ATOM   12793  C    THR H 167     7.809  12.777 -33.065  1.00 18.37          C
```

704

| ATOM | 12794 | O | THR | H | 167 | 8.193 | 12.942 | -34.203 | 1.00 | 16.52 | O |
| ATOM | 12795 | CB | THR | H | 167 | 9.372 | 11.197 | -31.850 | 1.00 | 18.73 | C |
| ATOM | 12796 | OG1 | THR | H | 167 | 9.403 | 9.910 | -31.204 | 1.00 | 21.56 | O |
| ATOM | 12797 | CG2 | THR | H | 167 | 9.753 | 12.256 | -30.812 | 1.00 | 18.05 | C |
| ATOM | 12798 | N | PHE | H | 168 | 7.194 | 13.713 | -32.350 | 1.00 | 17.56 | N |
| ATOM | 12799 | CA | PHE | H | 168 | 6.942 | 15.057 | -32.844 | 1.00 | 19.25 | C |
| ATOM | 12800 | C | PHE | H | 168 | 8.205 | 15.888 | -32.679 | 1.00 | 19.97 | C |
| ATOM | 12801 | O | PHE | H | 168 | 8.979 | 15.626 | -31.762 | 1.00 | 20.64 | O |
| ATOM | 12802 | CB | PHE | H | 168 | 5.792 | 15.675 | -32.054 | 1.00 | 18.95 | C |
| ATOM | 12803 | CG | PHE | H | 168 | 4.480 | 14.948 | -32.237 | 1.00 | 18.21 | C |
| ATOM | 12804 | CD1 | PHE | H | 168 | 4.057 | 14.008 | -31.318 | 1.00 | 18.68 | C |
| ATOM | 12805 | CD2 | PHE | H | 168 | 3.686 | 15.200 | -33.349 | 1.00 | 19.00 | C |
| ATOM | 12806 | CE1 | PHE | H | 168 | 2.891 | 13.328 | -31.515 | 1.00 | 19.86 | C |
| ATOM | 12807 | CE2 | PHE | H | 168 | 2.506 | 14.545 | -33.537 | 1.00 | 18.52 | C |
| ATOM | 12808 | CZ | PHE | H | 168 | 2.108 | 13.610 | -32.635 | 1.00 | 19.70 | C |
| ATOM | 12809 | N | PRO | H | 169 | 8.462 | 16.840 | -33.604 | 1.00 | 20.92 | N |
| ATOM | 12810 | CA | PRO | H | 169 | 9.563 | 17.778 | -33.428 | 1.00 | 21.13 | C |
| ATOM | 12811 | C | PRO | H | 169 | 9.420 | 18.600 | -32.171 | 1.00 | 21.42 | C |
| ATOM | 12812 | O | PRO | H | 169 | 8.327 | 18.926 | -31.791 | 1.00 | 21.75 | O |
| ATOM | 12813 | CB | PRO | H | 169 | 9.458 | 18.667 | -34.670 | 1.00 | 21.85 | C |
| ATOM | 12814 | CG | PRO | H | 169 | 8.887 | 17.700 | -35.727 | 1.00 | 22.03 | C |
| ATOM | 12815 | CD | PRO | H | 169 | 7.821 | 17.014 | -34.925 | 1.00 | 21.09 | C |
| ATOM | 12816 | N | ALA | H | 170 | 10.537 | 18.936 | -31.542 | 1.00 | 21.15 | N |
| ATOM | 12817 | CA | ALA | H | 170 | 10.506 | 19.748 | -30.343 | 1.00 | 20.72 | C |
| ATOM | 12818 | C | ALA | H | 170 | 10.129 | 21.184 | -30.633 | 1.00 | 22.88 | C |
| ATOM | 12819 | O | ALA | H | 170 | 10.361 | 21.700 | -31.715 | 1.00 | 21.78 | O |
| ATOM | 12820 | CB | ALA | H | 170 | 11.836 | 19.710 | -29.653 | 1.00 | 20.50 | C |
| ATOM | 12821 | N | VAL | H | 171 | 9.522 | 21.798 | -29.637 | 1.00 | 24.85 | N |
| ATOM | 12822 | CA | VAL | H | 171 | 9.255 | 23.216 | -29.617 | 1.00 | 25.36 | C |
| ATOM | 12823 | C | VAL | H | 171 | 10.071 | 23.818 | -28.502 | 1.00 | 25.17 | C |
| ATOM | 12824 | O | VAL | H | 171 | 10.243 | 23.230 | -27.444 | 1.00 | 21.69 | O |
| ATOM | 12825 | CB | VAL | H | 171 | 7.749 | 23.555 | -29.388 | 1.00 | 25.89 | C |
| ATOM | 12826 | CG1 | VAL | H | 171 | 7.278 | 23.240 | -27.981 | 1.00 | 26.57 | C |
| ATOM | 12827 | CG2 | VAL | H | 171 | 7.513 | 25.019 | -29.636 | 1.00 | 27.02 | C |
| ATOM | 12828 | N | LEU | H | 172 | 10.526 | 25.036 | -28.732 | 1.00 | 26.71 | N |
| ATOM | 12829 | CA | LEU | H | 172 | 11.416 | 25.725 | -27.828 | 1.00 | 29.03 | C |
| ATOM | 12830 | C | LEU | H | 172 | 10.589 | 26.760 | -27.126 | 1.00 | 31.62 | C |
| ATOM | 12831 | O | LEU | H | 172 | 10.036 | 27.636 | -27.787 | 1.00 | 32.98 | O |
| ATOM | 12832 | CB | LEU | H | 172 | 12.517 | 26.411 | -28.634 | 1.00 | 29.10 | C |
| ATOM | 12833 | CG | LEU | H | 172 | 13.515 | 27.294 | -27.911 | 1.00 | 28.06 | C |
| ATOM | 12834 | CD1 | LEU | H | 172 | 14.357 | 26.460 | -26.970 | 1.00 | 28.61 | C |
| ATOM | 12835 | CD2 | LEU | H | 172 | 14.371 | 27.995 | -28.930 | 1.00 | 29.50 | C |
| ATOM | 12836 | N | GLN | H | 173 | 10.466 | 26.652 | -25.805 | 1.00 | 33.22 | N |
| ATOM | 12837 | CA | GLN | H | 173 | 9.750 | 27.651 | -25.012 | 1.00 | 32.96 | C |
| ATOM | 12838 | C | GLN | H | 173 | 10.568 | 28.054 | -23.781 | 1.00 | 33.04 | C |
| ATOM | 12839 | O | GLN | H | 173 | 11.045 | 27.192 | -23.023 | 1.00 | 31.58 | O |
| ATOM | 12840 | CB | GLN | H | 173 | 8.401 | 27.106 | -24.561 | 1.00 | 34.09 | C |
| ATOM | 12841 | CG | GLN | H | 173 | 7.422 | 28.198 | -24.115 | 1.00 | 35.64 | C |
| ATOM | 12842 | CD | GLN | H | 173 | 6.404 | 27.715 | -23.094 | 1.00 | 36.56 | C |
| ATOM | 12843 | OE1 | GLN | H | 173 | 5.972 | 26.543 | -23.108 | 1.00 | 38.89 | O |
| ATOM | 12844 | NE2 | GLN | H | 173 | 6.022 | 28.615 | -22.187 | 1.00 | 39.61 | N |
| ATOM | 12845 | N | SER | H | 174 | 10.713 | 29.363 | -23.579 | 1.00 | 32.26 | N |
| ATOM | 12846 | CA | SER | H | 174 | 11.587 | 29.901 | -22.532 | 1.00 | 31.55 | C |
| ATOM | 12847 | C | SER | H | 174 | 12.925 | 29.172 | -22.505 | 1.00 | 30.57 | C |
| ATOM | 12848 | O | SER | H | 174 | 13.349 | 28.652 | -21.477 | 1.00 | 31.37 | O |
| ATOM | 12849 | CB | SER | H | 174 | 10.914 | 29.812 | -21.162 | 1.00 | 32.53 | C |
| ATOM | 12850 | OG | SER | H | 174 | 9.704 | 30.551 | -21.152 | 1.00 | 35.63 | O |
| ATOM | 12851 | N | ASP | H | 175 | 13.572 | 29.097 | -23.656 | 1.00 | 28.75 | N |
| ATOM | 12852 | CA | ASP | H | 175 | 14.925 | 28.551 | -23.742 | 1.00 | 27.06 | C |
| ATOM | 12853 | C | ASP | H | 175 | 15.047 | 27.042 | -23.406 | 1.00 | 25.29 | C |
| ATOM | 12854 | O | ASP | H | 175 | 16.134 | 26.523 | -23.264 | 1.00 | 25.24 | O |
| ATOM | 12855 | CB | ASP | H | 175 | 15.869 | 29.389 | -22.868 | 1.00 | 29.25 | C |
| ATOM | 12856 | CG | ASP | H | 175 | 15.817 | 30.890 | -23.212 | 1.00 | 31.07 | C |
| ATOM | 12857 | OD1 | ASP | H | 175 | 15.737 | 31.222 | -24.415 | 1.00 | 34.02 | O |
| ATOM | 12858 | OD2 | ASP | H | 175 | 15.862 | 31.730 | -22.281 | 1.00 | 33.89 | O |

```
ATOM  12859  N   LEU H 176    13.944  26.330 -23.290  1.00 22.45    N
ATOM  12860  CA  LEU H 176    14.023  24.866 -23.144  1.00 19.88    C
ATOM  12861  C   LEU H 176    13.140  24.230 -24.181  1.00 18.59    C
ATOM  12862  O   LEU H 176    12.188  24.832 -24.631  1.00 17.81    O
ATOM  12863  CB  LEU H 176    13.559  24.446 -21.755  1.00 18.23    C
ATOM  12864  CG  LEU H 176    14.354  25.032 -20.580  1.00 16.31    C
ATOM  12865  CD1 LEU H 176    13.648  24.848 -19.253  1.00 15.22    C
ATOM  12866  CD2 LEU H 176    15.722  24.421 -20.567  1.00 15.03    C
ATOM  12867  N   TYR H 177    13.470  23.010 -24.558  1.00 17.81    N
ATOM  12868  CA  TYR H 177    12.715  22.265 -25.554  1.00 16.41    C
ATOM  12869  C   TYR H 177    11.713  21.324 -24.887  1.00 15.81    C
ATOM  12870  O   TYR H 177    11.935  20.836 -23.770  1.00 13.77    O
ATOM  12871  CB  TYR H 177    13.658  21.469 -26.421  1.00 17.56    C
ATOM  12872  CG  TYR H 177    14.509  22.293 -27.334  1.00 16.80    C
ATOM  12873  CD1 TYR H 177    14.055  22.649 -28.593  1.00 17.36    C
ATOM  12874  CD2 TYR H 177    15.772  22.716 -26.935  1.00 17.97    C
ATOM  12875  CE1 TYR H 177    14.858  23.438 -29.461  1.00 18.81    C
ATOM  12876  CE2 TYR H 177    16.578  23.495 -27.779  1.00 18.87    C
ATOM  12877  CZ  TYR H 177    16.109  23.858 -29.028  1.00 17.83    C
ATOM  12878  OH  TYR H 177    16.922  24.614 -29.846  1.00 20.82    O
ATOM  12879  N   THR H 178    10.587  21.105 -25.570  1.00 16.47    N
ATOM  12880  CA  THR H 178     9.623  20.063 -25.206  1.00 16.36    C
ATOM  12881  C   THR H 178     9.160  19.291 -26.462  1.00 16.92    C
ATOM  12882  O   THR H 178     8.817  19.892 -27.452  1.00 17.80    O
ATOM  12883  CB  THR H 178     8.394  20.678 -24.422  1.00 17.48    C
ATOM  12884  OG1 THR H 178     8.848  21.268 -23.195  1.00 17.88    O
ATOM  12885  CG2 THR H 178     7.379  19.625 -24.066  1.00 17.10    C
ATOM  12886  N   LEU H 179     9.152  17.957 -26.399  1.00 17.41    N
ATOM  12887  CA  LEU H 179     8.475  17.127 -27.414  1.00 18.29    C
ATOM  12888  C   LEU H 179     7.632  16.021 -26.801  1.00 17.30    C
ATOM  12889  O   LEU H 179     7.664  15.809 -25.608  1.00 16.73    O
ATOM  12890  CB  LEU H 179     9.480  16.527 -28.403  1.00 18.07    C
ATOM  12891  CG  LEU H 179    10.557  15.556 -27.969  1.00 17.33    C
ATOM  12892  CD1 LEU H 179    10.003  14.190 -27.565  1.00 17.19    C
ATOM  12893  CD2 LEU H 179    11.531  15.400 -29.126  1.00 18.41    C
ATOM  12894  N   SER H 180     6.844  15.357 -27.642  1.00 17.93    N
ATOM  12895  CA  SER H 180     6.098  14.179 -27.244  1.00 18.30    C
ATOM  12896  C   SER H 180     6.311  13.066 -28.246  1.00 17.73    C
ATOM  12897  O   SER H 180     6.511  13.317 -29.414  1.00 16.82    O
ATOM  12898  CB  SER H 180     4.607  14.522 -27.141  1.00 20.62    C
ATOM  12899  OG  SER H 180     4.333  15.109 -25.869  1.00 24.02    O
ATOM  12900  N   SER H 181     6.243  11.810 -27.789  1.00 17.35    N
ATOM  12901  CA  SER H 181     6.230  10.679 -28.698  1.00 16.82    C
ATOM  12902  C   SER H 181     4.924   9.888 -28.538  1.00 15.02    C
ATOM  12903  O   SER H 181     4.377   9.837 -27.465  1.00 15.04    O
ATOM  12904  CB  SER H 181     7.391   9.764 -28.388  1.00 17.54    C
ATOM  12905  OG  SER H 181     7.403   8.676 -29.301  1.00 18.70    O
ATOM  12906  N   SER H 182     4.464   9.293 -29.628  1.00 16.44    N
ATOM  12907  CA  SER H 182     3.164   8.593 -29.740  1.00 16.03    C
ATOM  12908  C   SER H 182     3.420   7.145 -30.173  1.00 15.82    C
ATOM  12909  O   SER H 182     4.213   6.925 -31.065  1.00 18.45    O
ATOM  12910  CB  SER H 182     2.360   9.271 -30.843  1.00 15.46    C
ATOM  12911  OG  SER H 182     1.078   8.680 -31.033  1.00 15.65    O
ATOM  12912  N   VAL H 183     2.757   6.184 -29.539  1.00 16.99    N
ATOM  12913  CA  VAL H 183     2.753   4.785 -29.996  1.00 16.95    C
ATOM  12914  C   VAL H 183     1.306   4.315 -29.984  1.00 16.88    C
ATOM  12915  O   VAL H 183     0.545   4.672 -29.078  1.00 15.20    O
ATOM  12916  CB  VAL H 183     3.622   3.850 -29.093  1.00 15.67    C
ATOM  12917  CG1 VAL H 183     3.208   3.912 -27.633  1.00 15.22    C
ATOM  12918  CG2 VAL H 183     3.626   2.381 -29.614  1.00 17.83    C
ATOM  12919  N   THR H 184     0.935   3.521 -30.990  1.00 18.63    N
ATOM  12920  CA  THR H 184    -0.425   2.984 -31.096  1.00 19.14    C
ATOM  12921  C   THR H 184    -0.310   1.482 -31.160  1.00 19.35    C
ATOM  12922  O   THR H 184     0.433   0.968 -31.994  1.00 17.68    O
ATOM  12923  CB  THR H 184    -1.130   3.518 -32.322  1.00 20.01    C
```

```
ATOM  12924  OG1 THR H 184   -1.315   4.929 -32.161  1.00 18.36        O
ATOM  12925  CG2 THR H 184   -2.462   2.886 -32.502  1.00 19.97        C
ATOM  12926  N   VAL H 185   -0.978   0.819 -30.222  1.00 20.06        N
ATOM  12927  CA  VAL H 185   -0.889  -0.649 -30.096  1.00 22.29        C
ATOM  12928  C   VAL H 185   -2.312  -1.237 -30.000  1.00 22.94        C
ATOM  12929  O   VAL H 185   -3.280  -0.532 -29.690  1.00 21.50        O
ATOM  12930  CB  VAL H 185   -0.025  -1.076 -28.882  1.00 22.58        C
ATOM  12931  CG1 VAL H 185    1.331  -0.382 -28.922  1.00 24.63        C
ATOM  12932  CG2 VAL H 185   -0.715  -0.768 -27.539  1.00 25.09        C
ATOM  12933  N   PRO H 186   -2.451  -2.528 -30.323  1.00 23.12        N
ATOM  12934  CA  PRO H 186   -3.713  -3.204 -30.058  1.00 23.52        C
ATOM  12935  C   PRO H 186   -4.177  -3.080 -28.597  1.00 23.08        C
ATOM  12936  O   PRO H 186   -3.351  -3.125 -27.670  1.00 21.63        O
ATOM  12937  CB  PRO H 186   -3.399  -4.667 -30.383  1.00 23.96        C
ATOM  12938  CG  PRO H 186   -2.240  -4.632 -31.300  1.00 23.44        C
ATOM  12939  CD  PRO H 186   -1.457  -3.393 -30.976  1.00 23.57        C
ATOM  12940  N   SER H 187   -5.493  -2.940 -28.397  1.00 24.30        N
ATOM  12941  CA  SER H 187   -6.067  -2.889 -27.046  1.00 25.48        C
ATOM  12942  C   SER H 187   -5.854  -4.196 -26.338  1.00 25.67        C
ATOM  12943  O   SER H 187   -5.802  -4.240 -25.118  1.00 26.71        O
ATOM  12944  CB  SER H 187   -7.558  -2.572 -27.078  1.00 26.80        C
ATOM  12945  OG  SER H 187   -7.745  -1.177 -27.064  1.00 30.12        O
ATOM  12946  N   SER H 188   -5.706  -5.252 -27.136  1.00 26.09        N
ATOM  12947  CA  SER H 188   -5.418  -6.590 -26.643  1.00 27.24        C
ATOM  12948  C   SER H 188   -4.023  -6.702 -26.025  1.00 28.17        C
ATOM  12949  O   SER H 188   -3.756  -7.639 -25.284  1.00 29.93        O
ATOM  12950  CB  SER H 188   -5.567  -7.585 -27.798  1.00 27.28        C
ATOM  12951  OG  SER H 188   -4.740  -7.220 -28.901  1.00 28.67        O
ATOM  12952  N   THR H 189   -3.137  -5.751 -26.324  1.00 28.00        N
ATOM  12953  CA  THR H 189   -1.759  -5.787 -25.819  1.00 28.40        C
ATOM  12954  C   THR H 189   -1.486  -4.861 -24.638  1.00 28.27        C
ATOM  12955  O   THR H 189   -0.638  -5.168 -23.803  1.00 29.41        O
ATOM  12956  CB  THR H 189   -0.758  -5.375 -26.901  1.00 29.58        C
ATOM  12957  OG1 THR H 189   -1.206  -4.160 -27.520  1.00 31.42        O
ATOM  12958  CG2 THR H 189   -0.665  -6.426 -27.919  1.00 29.29        C
ATOM  12959  N   TRP H 190   -2.170  -3.716 -24.590  1.00 26.85        N
ATOM  12960  CA  TRP H 190   -2.038  -2.802 -23.466  1.00 26.52        C
ATOM  12961  C   TRP H 190   -3.401  -2.549 -22.861  1.00 26.78        C
ATOM  12962  O   TRP H 190   -4.332  -2.183 -23.608  1.00 28.51        O
ATOM  12963  CB  TRP H 190   -1.437  -1.443 -23.892  1.00 24.93        C
ATOM  12964  CG  TRP H 190   -1.198  -0.532 -22.723  1.00 24.44        C
ATOM  12965  CD1 TRP H 190   -0.057  -0.458 -21.964  1.00 24.47        C
ATOM  12966  CD2 TRP H 190   -2.104   0.445 -22.170  1.00 23.91        C
ATOM  12967  NE1 TRP H 190   -0.209   0.481 -20.971  1.00 23.73        N
ATOM  12968  CE2 TRP H 190   -1.450   1.053 -21.078  1.00 23.76        C
ATOM  12969  CE3 TRP H 190   -3.411   0.844 -22.478  1.00 24.39        C
ATOM  12970  CZ2 TRP H 190   -2.051   2.062 -20.305  1.00 24.21        C
ATOM  12971  CZ3 TRP H 190   -4.002   1.835 -21.712  1.00 24.43        C
ATOM  12972  CH2 TRP H 190   -3.321   2.439 -20.643  1.00 24.27        C
ATOM  12973  N   PRO H 191   -3.497  -2.594 -21.515  1.00 27.68        N
ATOM  12974  CA  PRO H 191   -2.399  -2.764 -20.539  1.00 29.80        C
ATOM  12975  C   PRO H 191   -2.005  -4.199 -20.169  1.00 30.61        C
ATOM  12976  O   PRO H 191   -1.195  -4.395 -19.255  1.00 30.52        O
ATOM  12977  CB  PRO H 191   -2.937  -2.037 -19.305  1.00 29.31        C
ATOM  12978  CG  PRO H 191   -4.419  -2.219 -19.382  1.00 27.91        C
ATOM  12979  CD  PRO H 191   -4.785  -2.360 -20.830  1.00 27.51        C
ATOM  12980  N   SER H 192   -2.540  -5.197 -20.862  1.00 32.05        N
ATOM  12981  CA  SER H 192   -2.223  -6.583 -20.500  1.00 32.84        C
ATOM  12982  C   SER H 192   -0.704  -6.811 -20.507  1.00 33.88        C
ATOM  12983  O   SER H 192   -0.161  -7.414 -19.572  1.00 32.72        O
ATOM  12984  CB  SER H 192   -2.924  -7.592 -21.422  1.00 33.16        C
ATOM  12985  OG  SER H 192   -2.835  -7.210 -22.787  1.00 34.28        O
ATOM  12986  N   GLU H 193   -0.037  -6.319 -21.556  1.00 34.49        N
ATOM  12987  CA  GLU H 193    1.419  -6.401 -21.690  1.00 35.11        C
ATOM  12988  C   GLU H 193    1.997  -4.993 -21.422  1.00 35.63        C
```

```
ATOM   12989  O    GLU H 193      1.273  -3.978 -21.520  1.00 35.25           O
ATOM   12990  CB   GLU H 193      1.818  -6.966 -23.077  1.00 36.27           C
ATOM   12991  CG   GLU H 193      1.190  -8.376 -23.404  1.00 38.72           C
ATOM   12992  CD   GLU H 193      1.238  -8.819 -24.912  1.00 40.07           C
ATOM   12993  OE1  GLU H 193      2.339  -9.142 -25.440  1.00 42.68           O
ATOM   12994  OE2  GLU H 193      0.154  -8.883 -25.574  1.00 44.02           O
ATOM   12995  N    THR H 194      3.281  -4.935 -21.056  1.00 33.74           N
ATOM   12996  CA   THR H 194      3.936  -3.674 -20.673  1.00 32.59           C
ATOM   12997  C    THR H 194      4.251  -2.808 -21.868  1.00 30.02           C
ATOM   12998  O    THR H 194      4.664  -3.293 -22.900  1.00 29.36           O
ATOM   12999  CB   THR H 194      5.304  -3.894 -20.003  1.00 33.40           C
ATOM   13000  OG1  THR H 194      6.209  -4.484 -20.947  1.00 37.03           O
ATOM   13001  CG2  THR H 194      5.193  -4.784 -18.782  1.00 34.62           C
ATOM   13002  N    VAL H 195      4.086  -1.509 -21.714  1.00 29.40           N
ATOM   13003  CA   VAL H 195      4.594  -0.580 -22.711  1.00 26.92           C
ATOM   13004  C    VAL H 195      5.376   0.483 -21.946  1.00 25.76           C
ATOM   13005  O    VAL H 195      4.874   1.067 -20.968  1.00 24.66           O
ATOM   13006  CB   VAL H 195      3.475   0.024 -23.575  1.00 27.64           C
ATOM   13007  CG1  VAL H 195      4.011   1.116 -24.518  1.00 27.21           C
ATOM   13008  CG2  VAL H 195      2.843  -1.066 -24.410  1.00 27.59           C
ATOM   13009  N    THR H 196      6.612   0.681 -22.403  1.00 23.03           N
ATOM   13010  CA   THR H 196      7.596   1.536 -21.748  1.00 23.68           C
ATOM   13011  C    THR H 196      8.218   2.457 -22.792  1.00 22.01           C
ATOM   13012  O    THR H 196      8.653   2.003 -23.848  1.00 19.76           O
ATOM   13013  CB   THR H 196      8.695   0.665 -21.110  1.00 23.01           C
ATOM   13014  OG1  THR H 196      8.159   0.020 -19.946  1.00 24.00           O
ATOM   13015  CG2  THR H 196      9.909   1.475 -20.687  1.00 22.48           C
ATOM   13016  N    CYS H 197      8.281   3.753 -22.515  1.00 24.08           N
ATOM   13017  CA   CYS H 197      9.065   4.609 -23.409  1.00 24.71           C
ATOM   13018  C    CYS H 197     10.442   4.813 -22.785  1.00 23.68           C
ATOM   13019  O    CYS H 197     10.574   4.887 -21.561  1.00 21.99           O
ATOM   13020  CB   CYS H 197      8.374   5.930 -23.707  1.00 27.49           C
ATOM   13021  SG   CYS H 197      8.530   7.109 -22.415  1.00 32.82           S
ATOM   13022  N    ASN H 198     11.458   4.832 -23.642  1.00 23.88           N
ATOM   13023  CA   ASN H 198     12.846   5.073 -23.252  1.00 24.05           C
ATOM   13024  C    ASN H 198     13.317   6.394 -23.896  1.00 23.36           C
ATOM   13025  O    ASN H 198     13.317   6.528 -25.116  1.00 23.70           O
ATOM   13026  CB   ASN H 198     13.736   3.905 -23.706  1.00 24.32           C
ATOM   13027  CG   ASN H 198     12.983   2.587 -23.760  1.00 24.99           C
ATOM   13028  OD1  ASN H 198     12.603   2.132 -24.835  1.00 25.38           O
ATOM   13029  ND2  ASN H 198     12.736   1.992 -22.596  1.00 23.60           N
ATOM   13030  N    VAL H 199     13.722   7.357 -23.070  1.00 22.75           N
ATOM   13031  CA   VAL H 199     14.164   8.665 -23.555  1.00 23.24           C
ATOM   13032  C    VAL H 199     15.645   8.828 -23.291  1.00 21.75           C
ATOM   13033  O    VAL H 199     16.102   8.570 -22.185  1.00 22.26           O
ATOM   13034  CB   VAL H 199     13.420   9.792 -22.826  1.00 23.60           C
ATOM   13035  CG1  VAL H 199     13.796  11.181 -23.405  1.00 25.58           C
ATOM   13036  CG2  VAL H 199     11.897   9.545 -22.915  1.00 25.69           C
ATOM   13037  N    ALA H 200     16.377   9.249 -24.310  1.00 21.61           N
ATOM   13038  CA   ALA H 200     17.804   9.498 -24.190  1.00 21.83           C
ATOM   13039  C    ALA H 200     18.102  10.931 -24.578  1.00 20.56           C
ATOM   13040  O    ALA H 200     17.582  11.455 -25.576  1.00 20.01           O
ATOM   13041  CB   ALA H 200     18.586   8.546 -25.069  1.00 22.25           C
ATOM   13042  N    HIS H 201     18.914  11.584 -23.753  1.00 20.69           N
ATOM   13043  CA   HIS H 201     19.354  12.945 -24.034  1.00 19.60           C
ATOM   13044  C    HIS H 201     20.878  12.917 -24.004  1.00 20.44           C
ATOM   13045  O    HIS H 201     21.454  12.962 -22.931  1.00 19.99           O
ATOM   13046  CB   HIS H 201     18.779  13.910 -22.995  1.00 17.39           C
ATOM   13047  CG   HIS H 201     19.134  15.346 -23.223  1.00 17.23           C
ATOM   13048  ND1  HIS H 201     19.626  16.159 -22.219  1.00 14.02           N
ATOM   13049  CD2  HIS H 201     19.020  16.128 -24.316  1.00 16.42           C
ATOM   13050  CE1  HIS H 201     19.806  17.381 -22.698  1.00 14.08           C
ATOM   13051  NE2  HIS H 201     19.453  17.384 -23.965  1.00 12.56           N
ATOM   13052  N    PRO H 202     21.513  12.789 -25.186  1.00 22.61           N
ATOM   13053  CA   PRO H 202     22.965  12.650 -25.305  1.00 22.46           C
```

```
ATOM   13054  C   PRO H 202      23.747  13.667 -24.472  1.00 22.55           C
ATOM   13055  O   PRO H 202      24.662  13.294 -23.741  1.00 22.09           O
ATOM   13056  CB  PRO H 202      23.205  12.893 -26.801  1.00 23.61           C
ATOM   13057  CG  PRO H 202      21.994  12.456 -27.458  1.00 24.64           C
ATOM   13058  CD  PRO H 202      20.870  12.734 -26.510  1.00 23.92           C
ATOM   13059  N   ALA H 203      23.384  14.940 -24.582  1.00 21.82           N
ATOM   13060  CA  ALA H 203      24.174  16.016 -23.942  1.00 20.67           C
ATOM   13061  C   ALA H 203      24.322  15.871 -22.435  1.00 20.67           C
ATOM   13062  O   ALA H 203      25.368  16.191 -21.879  1.00 17.51           O
ATOM   13063  CB  ALA H 203      23.624  17.343 -24.283  1.00 20.34           C
ATOM   13064  N   SER H 204      23.287  15.349 -21.785  1.00 20.83           N
ATOM   13065  CA  SER H 204      23.339  15.045 -20.359  1.00 21.33           C
ATOM   13066  C   SER H 204      23.595  13.562 -20.091  1.00 21.39           C
ATOM   13067  O   SER H 204      23.561  13.162 -18.967  1.00 22.52           O
ATOM   13068  CB  SER H 204      22.063  15.513 -19.635  1.00 21.07           C
ATOM   13069  OG  SER H 204      20.874  14.873 -20.055  1.00 16.18           O
ATOM   13070  N   SER H 205      23.841  12.759 -21.120  1.00 23.87           N
ATOM   13071  CA  SER H 205      24.089  11.308 -20.940  1.00 24.93           C
ATOM   13072  C   SER H 205      23.010  10.633 -20.096  1.00 26.38           C
ATOM   13073  O   SER H 205      23.296   9.748 -19.279  1.00 26.80           O
ATOM   13074  CB  SER H 205      25.488  11.080 -20.337  1.00 24.58           C
ATOM   13075  OG  SER H 205      26.464  11.585 -21.209  1.00 24.66           O
ATOM   13076  N   THR H 206      21.763  11.065 -20.312  1.00 27.77           N
ATOM   13077  CA  THR H 206      20.605  10.584 -19.559  1.00 28.08           C
ATOM   13078  C   THR H 206      19.891   9.512 -20.379  1.00 27.95           C
ATOM   13079  O   THR H 206      19.780   9.611 -21.586  1.00 24.80           O
ATOM   13080  CB  THR H 206      19.621  11.703 -19.252  1.00 30.17           C
ATOM   13081  OG1 THR H 206      19.374  12.450 -20.450  1.00 33.34           O
ATOM   13082  CG2 THR H 206      20.176  12.640 -18.200  1.00 29.08           C
ATOM   13083  N   LYS H 207      19.461   8.466 -19.685  1.00 28.95           N
ATOM   13084  CA  LYS H 207      18.631   7.415 -20.277  1.00 30.83           C
ATOM   13085  C   LYS H 207      17.530   7.095 -19.279  1.00 30.38           C
ATOM   13086  O   LYS H 207      17.812   6.621 -18.178  1.00 31.47           O
ATOM   13087  CB  LYS H 207      19.479   6.187 -20.590  1.00 32.27           C
ATOM   13088  CG  LYS H 207      20.492   6.426 -21.699  1.00 32.93           C
ATOM   13089  CD  LYS H 207      20.977   5.107 -22.289  1.00 33.61           C
ATOM   13090  CE  LYS H 207      21.984   5.306 -23.410  1.00 34.21           C
ATOM   13091  NZ  LYS H 207      22.839   4.091 -23.583  1.00 34.89           N
ATOM   13092  N   VAL H 208      16.295   7.426 -19.652  1.00 30.58           N
ATOM   13093  CA  VAL H 208      15.127   7.381 -18.773  1.00 30.92           C
ATOM   13094  C   VAL H 208      14.078   6.451 -19.368  1.00 31.05           C
ATOM   13095  O   VAL H 208      13.801   6.520 -20.555  1.00 30.44           O
ATOM   13096  CB  VAL H 208      14.471   8.781 -18.667  1.00 31.37           C
ATOM   13097  CG1 VAL H 208      13.089   8.698 -18.016  1.00 32.23           C
ATOM   13098  CG2 VAL H 208      15.359   9.710 -17.907  1.00 32.42           C
ATOM   13099  N   ASP H 209      13.500   5.602 -18.522  1.00 31.12           N
ATOM   13100  CA  ASP H 209      12.373   4.768 -18.901  1.00 31.17           C
ATOM   13101  C   ASP H 209      11.139   5.148 -18.081  1.00 30.58           C
ATOM   13102  O   ASP H 209      11.221   5.366 -16.867  1.00 29.47           O
ATOM   13103  CB  ASP H 209      12.697   3.304 -18.659  1.00 32.09           C
ATOM   13104  CG  ASP H 209      13.901   2.826 -19.438  1.00 33.19           C
ATOM   13105  OD1 ASP H 209      14.041   3.129 -20.629  1.00 32.38           O
ATOM   13106  OD2 ASP H 209      14.712   2.101 -18.849  1.00 38.70           O
ATOM   13107  N   LYS H 210      10.003   5.228 -18.759  1.00 30.35           N
ATOM   13108  CA  LYS H 210       8.703   5.410 -18.118  1.00 31.31           C
ATOM   13109  C   LYS H 210       7.742   4.336 -18.616  1.00 30.77           C
ATOM   13110  O   LYS H 210       7.394   4.300 -19.812  1.00 28.59           O
ATOM   13111  CB  LYS H 210       8.113   6.800 -18.430  1.00 31.79           C
ATOM   13112  CG  LYS H 210       8.374   7.852 -17.374  1.00 33.84           C
ATOM   13113  CD  LYS H 210       7.472   7.644 -16.151  1.00 34.74           C
ATOM   13114  CE  LYS H 210       7.649   8.772 -15.143  1.00 34.35           C
ATOM   13115  NZ  LYS H 210       7.060   8.443 -13.816  1.00 35.25           N
ATOM   13116  N   LYS H 211       7.320   3.468 -17.701  1.00 30.26           N
ATOM   13117  CA  LYS H 211       6.242   2.522 -18.015  1.00 30.67           C
ATOM   13118  C   LYS H 211       4.924   3.321 -18.067  1.00 29.85           C
```

```
ATOM   13119  O    LYS H 211      4.651   4.172 -17.210  1.00 28.30          O
ATOM   13120  CB   LYS H 211      6.211   1.375 -16.988  1.00 30.60          C
ATOM   13121  CG   LYS H 211      5.316   0.192 -17.371  1.00 31.96          C
ATOM   13122  CD   LYS H 211      5.013  -0.693 -16.150  1.00 32.80          C
ATOM   13123  CE   LYS H 211      4.309  -1.982 -16.545  1.00 32.90          C
ATOM   13124  NZ   LYS H 211      4.501  -3.084 -15.536  1.00 35.05          N
ATOM   13125  N    ILE H 212      4.130   3.085 -19.107  1.00 29.30          N
ATOM   13126  CA   ILE H 212      2.837   3.700 -19.208  1.00 30.16          C
ATOM   13127  C    ILE H 212      1.890   2.765 -18.481  1.00 31.51          C
ATOM   13128  O    ILE H 212      1.781   1.586 -18.823  1.00 31.94          O
ATOM   13129  CB   ILE H 212      2.378   3.902 -20.664  1.00 29.22          C
ATOM   13130  CG1  ILE H 212      3.477   4.547 -21.505  1.00 27.47          C
ATOM   13131  CG2  ILE H 212      1.111   4.747 -20.692  1.00 29.48          C
ATOM   13132  CD1  ILE H 212      4.008   5.872 -20.913  1.00 27.86          C
ATOM   13133  N    VAL H 213      1.241   3.274 -17.450  1.00 32.86          N
ATOM   13134  CA   VAL H 213      0.284   2.469 -16.702  1.00 33.92          C
ATOM   13135  C    VAL H 213     -1.066   3.146 -16.734  1.00 34.95          C
ATOM   13136  O    VAL H 213     -1.141   4.368 -16.858  1.00 34.10          O
ATOM   13137  CB   VAL H 213      0.753   2.175 -15.264  1.00 34.64          C
ATOM   13138  CG1  VAL H 213      1.964   1.238 -15.309  1.00 34.71          C
ATOM   13139  CG2  VAL H 213      1.092   3.440 -14.514  1.00 35.10          C
ATOM   13140  N    PRO H 214     -2.139   2.350 -16.690  1.00 35.95          N
ATOM   13141  CA   PRO H 214     -3.463   2.918 -16.732  1.00 37.06          C
ATOM   13142  C    PRO H 214     -3.753   3.797 -15.531  1.00 37.91          C
ATOM   13143  O    PRO H 214     -3.312   3.504 -14.421  1.00 38.32          O
ATOM   13144  CB   PRO H 214     -4.371   1.686 -16.701  1.00 37.17          C
ATOM   13145  CG   PRO H 214     -3.539   0.583 -17.172  1.00 36.86          C
ATOM   13146  CD   PRO H 214     -2.190   0.878 -16.636  1.00 36.51          C
ATOM   13147  N    ARG H 215     -4.518   4.848 -15.769  1.00 39.52          N
ATOM   13148  CA   ARG H 215     -4.805   5.859 -14.765  1.00 41.23          C
ATOM   13149  C    ARG H 215     -5.762   5.351 -13.688  1.00 41.47          C
ATOM   13150  O    ARG H 215     -6.881   4.934 -13.990  1.00 42.50          O
ATOM   13151  CB   ARG H 215     -5.400   7.091 -15.448  1.00 42.32          C
ATOM   13152  CG   ARG H 215     -4.456   7.763 -16.445  1.00 43.90          C
ATOM   13153  CD   ARG H 215     -5.092   9.005 -17.032  1.00 44.30          C
ATOM   13154  NE   ARG H 215     -5.373   9.990 -15.987  1.00 46.21          N
ATOM   13155  CZ   ARG H 215     -4.468  10.814 -15.455  1.00 46.88          C
ATOM   13156  NH1  ARG H 215     -3.201  10.795 -15.869  1.00 46.27          N
ATOM   13157  NH2  ARG H 215     -4.834  11.672 -14.510  1.00 47.50          N
TER    13158       ARG H 215
HETATM 13159  PD   PD  A 215      3.370  33.713  24.104  1.00 26.69          PD
HETATM 13160  C1   RAM B 311    -18.582  26.280  33.101  1.00 58.70          C
HETATM 13161  C2   RAM B 311    -18.653  27.002  31.756  1.00 58.41          C
HETATM 13162  C3   RAM B 311    -19.119  28.417  32.058  1.00 58.94          C
HETATM 13163  C4   RAM B 311    -18.048  29.115  32.894  1.00 59.66          C
HETATM 13164  C5   RAM B 311    -17.605  28.285  34.119  1.00 59.73          C
HETATM 13165  C6   RAM B 311    -16.203  28.691  34.582  1.00 59.66          C
HETATM 13166  O2   RAM B 311    -17.385  27.059  31.102  1.00 55.73          O
HETATM 13167  O3   RAM B 311    -19.357  29.101  30.849  1.00 59.38          O
HETATM 13168  O4   RAM B 311    -18.558  30.364  33.319  1.00 60.08          O
HETATM 13169  O5   RAM B 311    -17.609  26.861  33.955  1.00 59.69          O
HETATM 13170  C1   NAG B 310    -17.202  26.091  30.047  1.00 52.38          C
HETATM 13171  C2   NAG B 310    -15.710  25.915  29.772  1.00 50.58          C
HETATM 13172  C3   NAG B 310    -15.545  25.077  28.503  1.00 47.75          C
HETATM 13173  C4   NAG B 310    -16.180  23.718  28.762  1.00 49.20          C
HETATM 13174  C5   NAG B 310    -17.588  23.836  29.371  1.00 50.98          C
HETATM 13175  C6   NAG B 310    -17.999  22.513  30.007  1.00 50.88          C
HETATM 13176  C7   NAG B 310    -13.953  27.443  30.513  1.00 51.68          C
HETATM 13177  C8   NAG B 310    -13.438  28.858  30.526  1.00 52.13          C
HETATM 13178  N2   NAG B 310    -14.999  27.189  29.723  1.00 51.40          N
HETATM 13179  O3   NAG B 310    -14.186  24.910  28.128  1.00 42.16          O
HETATM 13180  O4   NAG B 310    -16.245  23.001  27.549  1.00 49.18          O
HETATM 13181  O5   NAG B 310    -17.693  24.819  30.382  1.00 51.45          O
HETATM 13182  O6   NAG B 310    -18.110  21.522  29.015  1.00 51.68          O
HETATM 13183  O7   NAG B 310    -13.408  26.586  31.218  1.00 52.59          O
```

```
HETATM13184  C1  RAM B 308   -13.784  25.860  27.117  1.00 36.49        C
HETATM13185  C2  RAM B 308   -12.275  26.071  27.184  1.00 34.01        C
HETATM13186  C3  RAM B 308   -11.521  24.814  26.746  1.00 30.40        C
HETATM13187  C4  RAM B 308   -12.011  24.263  25.408  1.00 29.71        C
HETATM13188  C5  RAM B 308   -13.541  24.176  25.451  1.00 32.08        C
HETATM13189  C6  RAM B 308   -14.148  23.808  24.111  1.00 32.40        C
HETATM13190  O2  RAM B 308   -11.901  27.151  26.357  1.00 33.57        O
HETATM13191  O3  RAM B 308   -10.113  25.058  26.756  1.00 26.02        O
HETATM13192  O4  RAM B 308   -11.477  22.945  25.233  1.00 28.44        O
HETATM13193  O5  RAM B 308   -14.093  25.430  25.808  1.00 34.29        O
HETATM13194  C1  RAM B 307    -9.494  24.411  27.889  1.00 25.90        C
HETATM13195  C2  RAM B 307    -7.987  24.410  27.669  1.00 22.82        C
HETATM13196  C3  RAM B 307    -7.502  25.867  27.681  1.00 24.31        C
HETATM13197  C4  RAM B 307    -7.851  26.489  29.011  1.00 25.07        C
HETATM13198  C5  RAM B 307    -9.365  26.394  29.213  1.00 25.71        C
HETATM13199  C6  RAM B 307    -9.721  26.966  30.580  1.00 25.25        C
HETATM13200  O2  RAM B 307    -7.386  23.646  28.681  1.00 24.01        O
HETATM13201  O3  RAM B 307    -6.116  25.984  27.415  1.00 25.66        O
HETATM13202  O4  RAM B 307    -7.427  27.843  29.020  1.00 26.47        O
HETATM13203  O5  RAM B 307    -9.800  25.035  29.133  1.00 24.60        O
HETATM13204  C1  RAM B 306    -7.223  22.246  28.384  1.00 22.61        C
HETATM13205  C2  RAM B 306    -6.126  21.647  29.250  1.00 21.47        C
HETATM13206  C3  RAM B 306    -6.505  21.801  30.718  1.00 21.96        C
HETATM13207  C4  RAM B 306    -7.801  21.076  30.993  1.00 25.39        C
HETATM13208  C5  RAM B 306    -8.845  21.494  29.951  1.00 25.59        C
HETATM13209  C6  RAM B 306   -10.091  20.637  30.056  1.00 26.16        C
HETATM13210  O2  RAM B 306    -6.070  20.249  28.984  1.00 19.97        O
HETATM13211  O3  RAM B 306    -5.530  21.356  31.613  1.00 23.65        O
HETATM13212  O4  RAM B 306    -8.163  21.421  32.317  1.00 26.17        O
HETATM13213  O5  RAM B 306    -8.371  21.461  28.595  1.00 23.27        O
HETATM13214  C1  NAG B 305    -4.895  19.914  28.236  1.00 19.31        C
HETATM13215  C2  NAG B 305    -4.927  18.404  28.274  1.00 19.40        C
HETATM13216  C3  NAG B 305    -4.023  17.775  27.237  1.00 18.67        C
HETATM13217  C4  NAG B 305    -4.189  18.448  25.892  1.00 17.15        C
HETATM13218  C5  NAG B 305    -3.959  19.928  26.035  1.00 17.59        C
HETATM13219  C6  NAG B 305    -4.087  20.559  24.667  1.00 17.63        C
HETATM13220  C7  NAG B 305    -5.557  17.481  30.452  1.00 19.57        C
HETATM13221  C8  NAG B 305    -5.061  17.124  31.817  1.00 22.82        C
HETATM13222  N2  NAG B 305    -4.624  17.897  29.605  1.00 19.35        N
HETATM13223  O3  NAG B 305    -4.496  16.460  27.090  1.00 18.45        O
HETATM13224  O4  NAG B 305    -3.239  18.001  24.955  1.00 17.22        O
HETATM13225  O5  NAG B 305    -4.942  20.426  26.920  1.00 17.24        O
HETATM13226  O6  NAG B 305    -5.384  20.359  24.108  1.00 17.57        O
HETATM13227  O7  NAG B 305    -6.752  17.381  30.162  1.00 21.46        O
HETATM13228  C1  RAM B 303    -3.621  15.456  27.633  1.00 19.08        C
HETATM13229  C2  RAM B 303    -4.455  14.267  28.060  1.00 21.57        C
HETATM13230  C3  RAM B 303    -5.001  13.532  26.843  1.00 23.26        C
HETATM13231  C4  RAM B 303    -3.857  13.124  25.926  1.00 21.24        C
HETATM13232  C5  RAM B 303    -3.151  14.434  25.538  1.00 20.41        C
HETATM13233  C6  RAM B 303    -2.038  14.240  24.512  1.00 19.24        C
HETATM13234  O2  RAM B 303    -3.613  13.371  28.764  1.00 19.77        O
HETATM13235  O3  RAM B 303    -5.777  12.407  27.248  1.00 25.99        O
HETATM13236  O4  RAM B 303    -4.402  12.568  24.758  1.00 20.06        O
HETATM13237  O5  RAM B 303    -2.637  15.044  26.713  1.00 21.48        O
HETATM13238  C1  RAM B 302    -7.191  12.739  27.097  1.00 30.49        C
HETATM13239  C2  RAM B 302    -8.001  11.451  27.036  1.00 31.38        C
HETATM13240  C3  RAM B 302    -7.841  10.699  28.342  1.00 32.29        C
HETATM13241  C4  RAM B 302    -8.112  11.604  29.551  1.00 32.17        C
HETATM13242  C5  RAM B 302    -7.406  12.943  29.412  1.00 30.86        C
HETATM13243  C6  RAM B 302    -7.835  13.837  30.564  1.00 30.93        C
HETATM13244  O2  RAM B 302    -9.385  11.717  26.870  1.00 34.58        O
HETATM13245  O3  RAM B 302    -8.757   9.626  28.329  1.00 32.11        O
HETATM13246  O4  RAM B 302    -7.683  11.019  30.770  1.00 31.79        O
HETATM13247  O5  RAM B 302    -7.725  13.524  28.150  1.00 29.20        O
HETATM13248  C1  RAM B 301    -9.773  12.054  25.527  1.00 37.92        C
```

```
HETATM13249  C2  RAM B 301   -11.145  11.451  25.255  1.00  39.98       C
HETATM13250  C3  RAM B 301   -12.236  12.195  26.027  1.00  40.50       C
HETATM13251  C4  RAM B 301   -12.175  13.688  25.769  1.00  40.30       C
HETATM13252  C5  RAM B 301   -10.765  14.204  26.048  1.00  39.46       C
HETATM13253  C6  RAM B 301   -10.672  15.689  25.702  1.00  37.75       C
HETATM13254  O2  RAM B 301   -11.405  11.547  23.862  1.00  42.08       O
HETATM13255  O3  RAM B 301   -13.503  11.783  25.587  1.00  41.93       O
HETATM13256  O4  RAM B 301   -13.139  14.343  26.575  1.00  41.49       O
HETATM13257  O5  RAM B 301    -9.804  13.455  25.304  1.00  38.21       O
HETATM13258  C1  GLC B 304    -4.654  11.140  24.821  1.00  17.76       C
HETATM13259  C2  GLC B 304    -3.498  10.446  24.152  1.00  16.35       C
HETATM13260  C3  GLC B 304    -3.402  10.889  22.705  1.00  16.58       C
HETATM13261  C4  GLC B 304    -4.726  10.654  21.989  1.00  17.25       C
HETATM13262  C5  GLC B 304    -5.938  11.190  22.771  1.00  15.57       C
HETATM13263  C6  GLC B 304    -7.232  10.610  22.174  1.00  15.70       C
HETATM13264  O2  GLC B 304    -2.311  10.755  24.812  1.00  16.35       O
HETATM13265  O3  GLC B 304    -2.254  10.312  22.089  1.00  17.58       O
HETATM13266  O4  GLC B 304    -4.735  11.123  20.653  1.00  18.59       O
HETATM13267  O5  GLC B 304    -5.879  10.869  24.166  1.00  18.29       O
HETATM13268  O6  GLC B 304    -7.246   9.183  22.222  1.00  18.55       O
HETATM13269  C1  GLC B 309   -11.136  22.599  23.868  1.00  25.26       C
HETATM13270  C2  GLC B 309   -10.392  21.277  23.850  1.00  22.57       C
HETATM13271  C3  GLC B 309    -9.044  21.454  24.538  1.00  23.18       C
HETATM13272  C4  GLC B 309    -8.253  22.558  23.875  1.00  21.35       C
HETATM13273  C5  GLC B 309    -9.096  23.827  23.753  1.00  23.51       C
HETATM13274  C6  GLC B 309    -8.429  24.836  22.853  1.00  22.38       C
HETATM13275  O2  GLC B 309   -11.123  20.298  24.570  1.00  25.22       O
HETATM13276  O3  GLC B 309    -8.314  20.246  24.501  1.00  23.91       O
HETATM13277  O4  GLC B 309    -7.037  22.782  24.610  1.00  23.56       O
HETATM13278  O5  GLC B 309   -10.366  23.579  23.192  1.00  23.74       O
HETATM13279  O6  GLC B 309    -9.230  25.993  22.724  1.00  23.84       O
HETATM13280  C1  RAM D 311    48.166  42.494  15.054  1.00  52.84       C
HETATM13281  C2  RAM D 311    48.055  41.839  13.689  1.00  52.37       C
HETATM13282  C3  RAM D 311    47.696  40.373  13.948  1.00  53.04       C
HETATM13283  C4  RAM D 311    46.349  40.300  14.662  1.00  53.13       C
HETATM13284  C5  RAM D 311    46.255  41.265  15.853  1.00  53.08       C
HETATM13285  C6  RAM D 311    44.797  41.497  16.244  1.00  53.01       C
HETATM13286  O2  RAM D 311    47.029  42.508  12.962  1.00  50.98       O
HETATM13287  O3  RAM D 311    47.651  39.584  12.774  1.00  53.69       O
HETATM13288  O4  RAM D 311    46.134  38.972  15.084  1.00  52.32       O
HETATM13289  O5  RAM D 311    46.862  42.533  15.609  1.00  54.15       O
HETATM13290  C1  NAG D 310    47.478  43.184  11.760  1.00  48.74       C
HETATM13291  C2  NAG D 310    46.360  43.097  10.734  1.00  47.22       C
HETATM13292  C3  NAG D 310    46.726  43.828   9.445  1.00  45.80       C
HETATM13293  C4  NAG D 310    47.178  45.238   9.766  1.00  46.64       C
HETATM13294  C5  NAG D 310    48.304  45.172  10.786  1.00  47.54       C
HETATM13295  C6  NAG D 310    48.772  46.555  11.197  1.00  47.71       C
HETATM13296  C7  NAG D 310    45.148  41.018  11.128  1.00  47.34       C
HETATM13297  C8  NAG D 310    45.184  39.523  10.982  1.00  47.19       C
HETATM13298  N2  NAG D 310    46.068  41.704  10.458  1.00  46.87       N
HETATM13299  O3  NAG D 310    45.579  43.937   8.634  1.00  43.46       O
HETATM13300  O4  NAG D 310    47.586  45.900   8.586  1.00  47.45       O
HETATM13301  O5  NAG D 310    47.800  44.550  11.947  1.00  48.65       O
HETATM13302  O6  NAG D 310    48.216  46.787  12.473  1.00  48.01       O
HETATM13303  O7  NAG D 310    44.288  41.545  11.835  1.00  48.41       O
HETATM13304  C1  RAM D 308    45.571  43.114   7.455  1.00  41.54       C
HETATM13305  C2  RAM D 308    44.116  42.769   7.171  1.00  40.24       C
HETATM13306  C3  RAM D 308    43.337  44.018   6.746  1.00  38.77       C
HETATM13307  C4  RAM D 308    44.053  44.761   5.615  1.00  39.31       C
HETATM13308  C5  RAM D 308    45.485  45.029   6.100  1.00  39.61       C
HETATM13309  C6  RAM D 308    46.331  45.783   5.098  1.00  40.08       C
HETATM13310  O2  RAM D 308    44.025  41.808   6.153  1.00  40.32       O
HETATM13311  O3  RAM D 308    42.013  43.625   6.421  1.00  36.92       O
HETATM13312  O4  RAM D 308    43.412  45.986   5.306  1.00  37.23       O
HETATM13313  O5  RAM D 308    46.108  43.784   6.337  1.00  40.72       O
```

```
HETATM13314  C1   RAM D 307      41.102  44.216   7.381  1.00 35.07          C
HETATM13315  C2   RAM D 307      39.688  44.124   6.824  1.00 32.56          C
HETATM13316  C3   RAM D 307      39.261  42.670   6.765  1.00 33.16          C
HETATM13317  C4   RAM D 307      39.484  42.005   8.119  1.00 33.13          C
HETATM13318  C5   RAM D 307      40.936  42.198   8.516  1.00 34.53          C
HETATM13319  C6   RAM D 307      41.290  41.517   9.824  1.00 35.93          C
HETATM13320  O2   RAM D 307      38.836  44.802   7.711  1.00 29.10          O
HETATM13321  O3   RAM D 307      37.897  42.571   6.414  1.00 33.85          O
HETATM13322  O4   RAM D 307      39.131  40.641   8.026  1.00 33.88          O
HETATM13323  O5   RAM D 307      41.164  43.592   8.648  1.00 34.22          O
HETATM13324  C1   RAM D 306      38.575  46.144   7.365  1.00 29.01          C
HETATM13325  C2   RAM D 306      37.236  46.522   7.983  1.00 28.49          C
HETATM13326  C3   RAM D 306      37.376  46.446   9.507  1.00 31.54          C
HETATM13327  C4   RAM D 306      38.425  47.487   9.938  1.00 31.59          C
HETATM13328  C5   RAM D 306      39.751  47.255   9.199  1.00 32.65          C
HETATM13329  C6   RAM D 306      40.631  48.506   9.388  1.00 32.39          C
HETATM13330  O2   RAM D 306      37.059  47.878   7.682  1.00 24.11          O
HETATM13331  O3   RAM D 306      36.104  46.633  10.111  1.00 31.66          O
HETATM13332  O4   RAM D 306      38.703  47.437  11.323  1.00 34.07          O
HETATM13333  O5   RAM D 306      39.597  47.001   7.802  1.00 28.71          O
HETATM13334  C1   NAG D 305      36.094  48.208   6.667  1.00 22.45          C
HETATM13335  C2   NAG D 305      35.886  49.719   6.760  1.00 21.96          C
HETATM13336  C3   NAG D 305      35.142  50.319   5.572  1.00 19.82          C
HETATM13337  C4   NAG D 305      35.632  49.735   4.262  1.00 19.45          C
HETATM13338  C5   NAG D 305      35.649  48.227   4.346  1.00 19.34          C
HETATM13339  C6   NAG D 305      36.083  47.625   3.017  1.00 20.72          C
HETATM13340  C7   NAG D 305      35.909  50.737   8.969  1.00 24.46          C
HETATM13341  C8   NAG D 305      35.138  51.119  10.190  1.00 24.80          C
HETATM13342  N2   NAG D 305      35.236  50.098   8.012  1.00 23.74          N
HETATM13343  O3   NAG D 305      35.471  51.681   5.464  1.00 20.79          O
HETATM13344  O4   NAG D 305      34.816  50.168   3.188  1.00 18.70          O
HETATM13345  O5   NAG D 305      36.552  47.861   5.376  1.00 20.90          O
HETATM13346  O6   NAG D 305      37.423  47.929   2.812  1.00 19.68          O
HETATM13347  O7   NAG D 305      37.107  51.000   8.881  1.00 26.44          O
HETATM13348  C1   RAM D 303      34.459  52.580   5.909  1.00 22.22          C
HETATM13349  C2   RAM D 303      35.143  53.810   6.474  1.00 22.52          C
HETATM13350  C3   RAM D 303      35.865  54.577   5.377  1.00 23.09          C
HETATM13351  C4   RAM D 303      34.884  54.910   4.256  1.00 21.38          C
HETATM13352  C5   RAM D 303      34.335  53.567   3.767  1.00 19.16          C
HETATM13353  C6   RAM D 303      33.406  53.622   2.553  1.00 19.40          C
HETATM13354  O2   RAM D 303      34.131  54.627   7.048  1.00 23.59          O
HETATM13355  O3   RAM D 303      36.415  55.793   5.854  1.00 25.96          O
HETATM13356  O4   RAM D 303      35.524  55.574   3.204  1.00 20.40          O
HETATM13357  O5   RAM D 303      33.638  53.012   4.858  1.00 21.58          O
HETATM13358  C1   RAM D 302      37.866  55.771   5.915  1.00 29.66          C
HETATM13359  C2   RAM D 302      38.346  57.177   6.230  1.00 31.94          C
HETATM13360  C3   RAM D 302      37.874  57.483   7.657  1.00 32.55          C
HETATM13361  C4   RAM D 302      38.342  56.449   8.649  1.00 32.78          C
HETATM13362  C5   RAM D 302      37.949  55.070   8.202  1.00 33.43          C
HETATM13363  C6   RAM D 302      38.549  54.056   9.176  1.00 33.93          C
HETATM13364  O2   RAM D 302      39.766  57.370   6.146  1.00 34.41          O
HETATM13365  O3   RAM D 302      38.409  58.700   8.077  1.00 34.79          O
HETATM13366  O4   RAM D 302      37.713  56.643   9.885  1.00 33.71          O
HETATM13367  O5   RAM D 302      38.397  54.860   6.868  1.00 31.96          O
HETATM13368  C1   RAM D 301      40.396  57.158   4.848  1.00 37.57          C
HETATM13369  C2   RAM D 301      41.693  57.986   4.742  1.00 38.35          C
HETATM13370  C3   RAM D 301      42.764  57.399   5.679  1.00 38.51          C
HETATM13371  C4   RAM D 301      42.902  55.894   5.544  1.00 39.05          C
HETATM13372  C5   RAM D 301      41.552  55.194   5.553  1.00 38.56          C
HETATM13373  C6   RAM D 301      41.740  53.702   5.273  1.00 38.16          C
HETATM13374  O2   RAM D 301      42.154  58.088   3.392  1.00 37.69          O
HETATM13375  O3   RAM D 301      44.044  57.939   5.449  1.00 39.71          O
HETATM13376  O4   RAM D 301      43.718  55.403   6.579  1.00 38.81          O
HETATM13377  O5   RAM D 301      40.721  55.800   4.588  1.00 37.13          O
HETATM13378  C1   GLC D 304      35.450  57.027   3.210  1.00 18.38          C
```

```
HETATM13379   C2    GLC D 304        34.354    57.599     2.324    1.00 18.09               C
HETATM13380   C3    GLC D 304        34.588    57.176     0.870    1.00 16.05               C
HETATM13381   C4    GLC D 304        35.977    57.727     0.470    1.00 16.45               C
HETATM13382   C5    GLC D 304        37.082    57.320     1.449    1.00 17.80               C
HETATM13383   C6    GLC D 304        38.477    57.944     1.203    1.00 16.32               C
HETATM13384   O2    GLC D 304        33.090    57.279     2.868    1.00 18.83               O
HETATM13385   O3    GLC D 304        33.600    57.630    -0.034    1.00 16.01               O
HETATM13386   O4    GLC D 304        36.281    57.321    -0.833    1.00 17.08               O
HETATM13387   O5    GLC D 304        36.696    57.525     2.809    1.00 19.88               O
HETATM13388   O6    GLC D 304        38.373    59.350     1.110    1.00 19.98               O
HETATM13389   C1    GLC D 309        43.361    46.329     3.905    1.00 35.21               C
HETATM13390   C2    GLC D 309        42.502    47.575     3.742    1.00 34.21               C
HETATM13391   C3    GLC D 309        41.065    47.250     4.104    1.00 32.91               C
HETATM13392   C4    GLC D 309        40.574    46.134     3.214    1.00 32.14               C
HETATM13393   C5    GLC D 309        41.536    44.939     3.237    1.00 32.18               C
HETATM13394   C6    GLC D 309        41.231    43.955     2.126    1.00 32.62               C
HETATM13395   O2    GLC D 309        42.998    48.677     4.492    1.00 34.57               O
HETATM13396   O3    GLC D 309        40.242    48.375     3.933    1.00 33.07               O
HETATM13397   O4    GLC D 309        39.301    45.764     3.674    1.00 30.97               O
HETATM13398   O5    GLC D 309        42.874    45.311     3.045    1.00 32.88               O
HETATM13399   O6    GLC D 309        42.060    42.824     2.299    1.00 32.29               O
HETATM13400   C1    RAM F 311       -12.612    40.346    13.744    1.00 45.86               C
HETATM13401   C2    RAM F 311       -12.622    39.472    14.993    1.00 46.23               C
HETATM13402   C3    RAM F 311       -12.338    38.031    14.577    1.00 46.20               C
HETATM13403   C4    RAM F 311       -10.987    37.979    13.865    1.00 46.82               C
HETATM13404   C5    RAM F 311       -10.993    38.958    12.686    1.00 46.77               C
HETATM13405   C6    RAM F 311        -9.640    39.006    11.987    1.00 46.98               C
HETATM13406   O2    RAM F 311       -11.583    39.900    15.853    1.00 44.99               O
HETATM13407   O3    RAM F 311       -12.320    37.185    15.705    1.00 46.93               O
HETATM13408   O4    RAM F 311       -10.641    36.650    13.497    1.00 47.49               O
HETATM13409   O5    RAM F 311       -11.334    40.262    13.127    1.00 46.54               O
HETATM13410   C1    NAG F 310       -11.983    40.636    17.011    1.00 43.04               C
HETATM13411   C2    NAG F 310       -10.787    40.608    17.943    1.00 41.43               C
HETATM13412   C3    NAG F 310       -11.132    41.257    19.273    1.00 41.01               C
HETATM13413   C4    NAG F 310       -11.644    42.681    19.009    1.00 42.65               C
HETATM13414   C5    NAG F 310       -12.629    42.797    17.817    1.00 44.76               C
HETATM13415   C6    NAG F 310       -12.695    44.234    17.274    1.00 45.56               C
HETATM13416   C7    NAG F 310        -9.344    38.707    17.400    1.00 40.13               C
HETATM13417   C8    NAG F 310        -9.167    37.217    17.484    1.00 40.72               C
HETATM13418   N2    NAG F 310       -10.335    39.234    18.117    1.00 41.81               N
HETATM13419   O3    NAG F 310        -9.955    41.336    20.060    1.00 37.31               O
HETATM13420   O4    NAG F 310       -12.252    43.173    20.191    1.00 41.68               O
HETATM13421   O5    NAG F 310       -12.290    41.974    16.704    1.00 43.78               O
HETATM13422   O6    NAG F 310       -13.053    45.194    18.256    1.00 46.09               O
HETATM13423   O7    NAG F 310        -8.591    39.380    16.689    1.00 39.31               O
HETATM13424   C1    RAM F 308        -9.746    40.238    20.969    1.00 34.03               C
HETATM13425   C2    RAM F 308        -8.251    40.027    21.181    1.00 31.74               C
HETATM13426   C3    RAM F 308        -7.635    41.268    21.799    1.00 29.20               C
HETATM13427   C4    RAM F 308        -8.388    41.764    23.040    1.00 29.56               C
HETATM13428   C5    RAM F 308        -9.906    41.749    22.762    1.00 31.38               C
HETATM13429   C6    RAM F 308       -10.764    41.935    24.011    1.00 30.92               C
HETATM13430   O2    RAM F 308        -7.999    38.932    22.042    1.00 32.08               O
HETATM13431   O3    RAM F 308        -6.280    40.986    22.100    1.00 27.44               O
HETATM13432   O4    RAM F 308        -7.935    43.084    23.365    1.00 26.39               O
HETATM13433   O5    RAM F 308       -10.295    40.502    22.225    1.00 32.46               O
HETATM13434   C1    RAM F 307        -5.440    41.703    21.168    1.00 23.72               C
HETATM13435   C2    RAM F 307        -4.018    41.705    21.710    1.00 19.52               C
HETATM13436   C3    RAM F 307        -3.473    40.286    21.745    1.00 18.63               C
HETATM13437   C4    RAM F 307        -3.603    39.680    20.368    1.00 20.72               C
HETATM13438   C5    RAM F 307        -5.072    39.722    19.948    1.00 21.36               C
HETATM13439   C6    RAM F 307        -5.288    39.059    18.592    1.00 22.95               C.
HETATM13440   O2    RAM F 307        -3.222    42.403    20.822    1.00 19.84               O
HETATM13441   O3    RAM F 307        -2.139    40.198    22.235    1.00 17.89               O
HETATM13442   O4    RAM F 307        -3.195    38.335    20.472    1.00 21.93               O
HETATM13443   O5    RAM F 307        -5.489    41.093    19.882    1.00 24.12               O
```

```
HETATM13444  C1  RAM F 306    -3.112  43.806  21.174  1.00  18.85           C
HETATM13445  C2  RAM F 306    -1.838  44.345  20.575  1.00  17.80           C
HETATM13446  C3  RAM F 306    -1.910  44.146  19.072  1.00  20.06           C
HETATM13447  C4  RAM F 306    -3.171  44.854  18.557  1.00  20.30           C
HETATM13448  C5  RAM F 306    -4.398  44.342  19.284  1.00  22.63           C
HETATM13449  C6  RAM F 306    -5.683  45.031  18.824  1.00  23.14           C
HETATM13450  O2  RAM F 306    -1.732  45.745  20.797  1.00  16.14           O
HETATM13451  O3  RAM F 306    -0.756  44.599  18.439  1.00  20.60           O
HETATM13452  O4  RAM F 306    -3.325  44.775  17.155  1.00  21.75           O
HETATM13453  O5  RAM F 306    -4.206  44.536  20.683  1.00  19.29           O
HETATM13454  C1  NAG F 305    -0.846  46.135  21.844  1.00  12.77           C
HETATM13455  C2  NAG F 305    -0.918  47.657  21.782  1.00  13.71           C
HETATM13456  C3  NAG F 305    -0.239  48.331  22.962  1.00  15.60           C
HETATM13457  C4  NAG F 305    -0.609  47.629  24.284  1.00  13.87           C
HETATM13458  C5  NAG F 305    -0.420  46.122  24.144  1.00  13.83           C
HETATM13459  C6  NAG F 305    -0.794  45.420  25.418  1.00  14.25           C
HETATM13460  C7  NAG F 305    -1.006  48.652  19.521  1.00  17.45           C
HETATM13461  C8  NAG F 305    -0.246  49.029  18.272  1.00  19.01           C
HETATM13462  N2  NAG F 305    -0.327  48.074  20.517  1.00  15.27           N
HETATM13463  O3  NAG F 305    -0.706  49.657  22.990  1.00  16.00           O
HETATM13464  O4  NAG F 305     0.190  48.056  25.371  1.00  15.21           O
HETATM13465  O5  NAG F 305    -1.243  45.673  23.099  1.00  13.55           O
HETATM13466  O6  NAG F 305    -2.196  45.497  25.630  1.00  12.08           O
HETATM13467  O7  NAG F 305    -2.197  48.887  19.578  1.00  17.09           O
HETATM13468  C1  RAM F 303     0.267  50.646  22.649  1.00  13.57           C
HETATM13469  C2  RAM F 303    -0.492  51.818  22.049  1.00  15.96           C
HETATM13470  C3  RAM F 303    -1.275  52.553  23.123  1.00  18.27           C
HETATM13471  C4  RAM F 303    -0.405  52.962  24.313  1.00  15.11           C
HETATM13472  C5  RAM F 303     0.290  51.695  24.816  1.00  12.54           C
HETATM13473  C6  RAM F 303     1.230  51.986  25.983  1.00  14.49           C
HETATM13474  O2  RAM F 303     0.435  52.738  21.528  1.00  13.50           O
HETATM13475  O3  RAM F 303    -1.851  53.728  22.555  1.00  22.81           O
HETATM13476  O4  RAM F 303    -1.210  53.511  25.345  1.00  12.87           O
HETATM13477  O5  RAM F 303     1.032  51.122  23.734  1.00  17.00           O
HETATM13478  C1  RAM F 302    -3.284  53.553  22.504  1.00  24.42           C
HETATM13479  C2  RAM F 302    -3.973  54.912  22.342  1.00  28.49           C
HETATM13480  C3  RAM F 302    -3.505  55.525  21.040  1.00  28.48           C
HETATM13481  C4  RAM F 302    -3.825  54.555  19.906  1.00  27.96           C
HETATM13482  C5  RAM F 302    -3.244  53.165  20.176  1.00  26.76           C
HETATM13483  C6  RAM F 302    -3.723  52.209  19.096  1.00  27.63           C
HETATM13484  O2  RAM F 302    -5.388  54.760  22.251  1.00  31.72           O
HETATM13485  O3  RAM F 302    -4.201  56.737  20.831  1.00  30.92           O
HETATM13486  O4  RAM F 302    -3.290  55.014  18.677  1.00  27.86           O
HETATM13487  O5  RAM F 302    -3.631  52.688  21.447  1.00  24.76           O
HETATM13488  C1  RAM F 301    -6.102  54.436  23.473  1.00  35.17           C
HETATM13489  C2  RAM F 301    -7.525  54.955  23.316  1.00  36.65           C
HETATM13490  C3  RAM F 301    -8.335  54.097  22.354  1.00  37.83           C
HETATM13491  C4  RAM F 301    -8.272  52.607  22.690  1.00  38.62           C
HETATM13492  C5  RAM F 301    -6.836  52.164  22.938  1.00  36.85           C
HETATM13493  C6  RAM F 301    -6.852  50.756  23.525  1.00  35.62           C
HETATM13494  O2  RAM F 301    -8.149  55.022  24.584  1.00  39.47           O
HETATM13495  O3  RAM F 301    -9.666  54.533  22.433  1.00  40.21           O
HETATM13496  O4  RAM F 301    -8.860  51.816  21.654  1.00  41.25           O
HETATM13497  O5  RAM F 301    -6.187  53.052  23.829  1.00  34.51           O
HETATM13498  C1  GLC F 304    -1.197  54.974  25.290  1.00  15.44           C
HETATM13499  C2  GLC F 304    -0.123  55.613  26.158  1.00  15.50           C
HETATM13500  C3  GLC F 304    -0.261  55.260  27.641  1.00  14.13           C
HETATM13501  C4  GLC F 304    -1.682  55.583  28.078  1.00  15.15           C
HETATM13502  C5  GLC F 304    -2.720  55.052  27.090  1.00  13.12           C
HETATM13503  C6  GLC F 304    -4.152  55.461  27.376  1.00  16.92           C
HETATM13504  O2  GLC F 304     1.167  55.302  25.695  1.00  18.54           O
HETATM13505  O3  GLC F 304     0.700  55.919  28.475  1.00  14.77           O
HETATM13506  O4  GLC F 304    -1.968  55.102  29.366  1.00  16.00           O
HETATM13507  O5  GLC F 304    -2.437  55.391  25.724  1.00  14.66           O
HETATM13508  O6  GLC F 304    -4.291  56.865  27.275  1.00  16.32           O
```

```
HETATM13509  C1  GLC F 309    -7.886  43.343  24.789  1.00 23.29           C
HETATM13510  C2  GLC F 309    -7.147  44.653  24.936  1.00 22.78           C
HETATM13511  C3  GLC F 309    -5.693  44.482  24.560  1.00 20.10           C
HETATM13512  C4  GLC F 309    -5.055  43.374  25.386  1.00 19.63           C
HETATM13513  C5  GLC F 309    -5.897  42.114  25.277  1.00 22.47           C
HETATM13514  C6  GLC F 309    -5.372  41.057  26.225  1.00 21.02           C
HETATM13515  O2  GLC F 309    -7.739  45.562  24.031  1.00 22.63           O
HETATM13516  O3  GLC F 309    -5.030  45.712  24.720  1.00 22.50           O
HETATM13517  O4  GLC F 309    -3.763  43.155  24.874  1.00 21.37           O
HETATM13518  O5  GLC F 309    -7.257  42.363  25.590  1.00 23.35           O
HETATM13519  O6  GLC F 309    -6.203  39.923  26.162  1.00 24.61           O
HETATM13520  C1  RAM H 311    52.435  23.715 -64.855  1.00 54.10           C
HETATM13521  C2  RAM H 311    52.318  24.013 -63.367  1.00 53.13           C
HETATM13522  C3  RAM H 311    52.330  25.522 -63.156  1.00 53.68           C
HETATM13523  C4  RAM H 311    51.253  26.210 -63.999  1.00 54.44           C
HETATM13524  C5  RAM H 311    51.208  25.686 -65.440  1.00 54.54           C
HETATM13525  C6  RAM H 311    49.920  26.133 -66.133  1.00 54.53           C
HETATM13526  O2  RAM H 311    51.101  23.456 -62.899  1.00 51.01           O
HETATM13527  O3  RAM H 311    52.108  25.805 -61.793  1.00 54.54           O
HETATM13528  O4  RAM H 311    51.427  27.624 -63.997  1.00 54.25           O
HETATM13529  O5  RAM H 311    51.317  24.269 -65.520  1.00 54.72           O
HETATM13530  C1  NAG H 310    51.177  22.949 -61.546  1.00 47.75           C
HETATM13531  C2  NAG H 310    49.790  23.114 -60.917  1.00 45.78           C
HETATM13532  C3  NAG H 310    49.721  22.510 -59.508  1.00 43.54           C
HETATM13533  C4  NAG H 310    50.253  21.076 -59.571  1.00 44.99           C
HETATM13534  C5  NAG H 310    51.665  21.082 -60.164  1.00 46.00           C
HETATM13535  C6  NAG H 310    52.344  19.704 -60.148  1.00 46.37           C
HETATM13536  C7  NAG H 310    48.398  24.901 -61.816  1.00 45.72           C
HETATM13537  C8  NAG H 310    48.089  26.371 -61.870  1.00 46.03           C
HETATM13538  N2  NAG H 310    49.346  24.507 -60.973  1.00 45.82           N
HETATM13539  O3  NAG H 310    48.375  22.477 -59.073  1.00 37.79           O
HETATM13540  O4  NAG H 310    50.168  20.438 -58.310  1.00 45.67           O
HETATM13541  O5  NAG H 310    51.562  21.580 -61.488  1.00 46.55           O
HETATM13542  O6  NAG H 310    51.597  18.733 -60.852  1.00 47.44           O
HETATM13543  O7  NAG H 310    47.790  24.116 -62.540  1.00 46.19           O
HETATM13544  C1  RAM H 308    48.040  23.554 -58.163  1.00 33.47           C
HETATM13545  C2  RAM H 308    46.556  23.869 -58.293  1.00 30.78           C
HETATM13546  C3  RAM H 308    45.730  22.630 -57.951  1.00 27.96           C
HETATM13547  C4  RAM H 308    46.122  22.086 -56.582  1.00 27.72           C
HETATM13548  C5  RAM H 308    47.662  21.939 -56.501  1.00 31.54           C
HETATM13549  C6  RAM H 308    48.174  21.593 -55.107  1.00 32.77           C
HETATM13550  O2  RAM H 308    46.151  24.957 -57.484  1.00 30.25           O
HETATM13551  O3  RAM H 308    44.357  22.957 -58.016  1.00 23.86           O
HETATM13552  O4  RAM H 308    45.457  20.845 -56.431  1.00 26.19           O
HETATM13553  O5  RAM H 308    48.295  23.163 -56.844  1.00 32.46           O
HETATM13554  C1  RAM H 307    43.670  22.393 -59.152  1.00 24.12           C
HETATM13555  C2  RAM H 307    42.186  22.490 -58.808  1.00 22.40           C
HETATM13556  C3  RAM H 307    41.738  23.946 -58.837  1.00 23.55           C
HETATM13557  C4  RAM H 307    42.129  24.580 -60.166  1.00 24.88           C
HETATM13558  C5  RAM H 307    43.643  24.447 -60.334  1.00 24.81           C
HETATM13559  C6  RAM H 307    44.173  25.174 -61.578  1.00 26.95           C
HETATM13560  O2  RAM H 307    41.364  21.845 -59.712  1.00 23.68           O
HETATM13561  O3  RAM H 307    40.345  24.038 -58.642  1.00 24.99           O
HETATM13562  O4  RAM H 307    41.704  25.933 -60.183  1.00 23.19           O
HETATM13563  O5  RAM H 307    43.984  23.053 -60.371  1.00 25.86           O
HETATM13564  C1  RAM H 306    41.156  20.448 -59.464  1.00 21.75           C
HETATM13565  C2  RAM H 306    40.016  20.012 -60.334  1.00 21.36           C
HETATM13566  C3  RAM H 306    40.471  20.210 -61.783  1.00 23.09           C
HETATM13567  C4  RAM H 306    41.654  19.317 -62.082  1.00 23.11           C
HETATM13568  C5  RAM H 306    42.744  19.673 -61.073  1.00 25.51           C
HETATM13569  C6  RAM H 306    43.942  18.756 -61.185  1.00 26.52           C
HETATM13570  O2  RAM H 306    39.852  18.627 -60.166  1.00 19.81           O
HETATM13571  O3  RAM H 306    39.434  20.014 -62.695  1.00 20.01           O
HETATM13572  O4  RAM H 306    42.090  19.603 -63.395  1.00 25.68           O
HETATM13573  O5  RAM H 306    42.289  19.655 -59.719  1.00 24.05           O
```

| | | | | | | | | | |
|---|---|---|---|---|---|---|---|---|---|
| HETATM13574 | C1 | NAG | H | 305 | 38.722 | 18.248 | -59.408 | 1.00 16.51 | C |
| HETATM13575 | C2 | NAG | H | 305 | 38.696 | 16.696 | -59.527 | 1.00 19.01 | C |
| HETATM13576 | C3 | NAG | H | 305 | 37.827 | 16.087 | -58.446 | 1.00 16.84 | C |
| HETATM13577 | C4 | NAG | H | 305 | 37.994 | 16.741 | -57.067 | 1.00 15.87 | C |
| HETATM13578 | C5 | NAG | H | 305 | 37.870 | 18.256 | -57.193 | 1.00 16.51 | C |
| HETATM13579 | C6 | NAG | H | 305 | 37.998 | 18.929 | -55.864 | 1.00 16.34 | C |
| HETATM13580 | C7 | NAG | H | 305 | 39.068 | 15.688 | -61.768 | 1.00 21.60 | C |
| HETATM13581 | C8 | NAG | H | 305 | 38.504 | 15.603 | -63.164 | 1.00 20.28 | C |
| HETATM13582 | N2 | NAG | H | 305 | 38.253 | 16.226 | -60.834 | 1.00 19.01 | N |
| HETATM13583 | O3 | NAG | H | 305 | 38.152 | 14.719 | -58.327 | 1.00 19.98 | O |
| HETATM13584 | O4 | NAG | H | 305 | 36.980 | 16.288 | -56.187 | 1.00 16.99 | O |
| HETATM13585 | O5 | NAG | H | 305 | 38.889 | 18.715 | -58.073 | 1.00 17.52 | O |
| HETATM13586 | O6 | NAG | H | 305 | 39.246 | 18.635 | -55.262 | 1.00 17.15 | O |
| HETATM13587 | O7 | NAG | H | 305 | 40.196 | 15.237 | -61.537 | 1.00 20.62 | O |
| HETATM13588 | C1 | RAM | H | 303 | 37.169 | 13.822 | -58.843 | 1.00 17.91 | C |
| HETATM13589 | C2 | RAM | H | 303 | 37.893 | 12.596 | -59.384 | 1.00 19.55 | C |
| HETATM13590 | C3 | RAM | H | 303 | 38.520 | 11.850 | -58.198 | 1.00 22.55 | C |
| HETATM13591 | C4 | RAM | H | 303 | 37.491 | 11.511 | -57.132 | 1.00 18.81 | C |
| HETATM13592 | C5 | RAM | H | 303 | 36.829 | 12.821 | -56.717 | 1.00 14.62 | C |
| HETATM13593 | C6 | RAM | H | 303 | 35.728 | 12.589 | -55.709 | 1.00 14.03 | C |
| HETATM13594 | O2 | RAM | H | 303 | 36.896 | 11.840 | -60.032 | 1.00 20.45 | O |
| HETATM13595 | O3 | RAM | H | 303 | 39.129 | 10.643 | -58.593 | 1.00 27.64 | O |
| HETATM13596 | O4 | RAM | H | 303 | 38.116 | 10.889 | -56.019 | 1.00 18.41 | O |
| HETATM13597 | O5 | RAM | H | 303 | 36.235 | 13.342 | -57.885 | 1.00 17.67 | O |
| HETATM13598 | C1 | RAM | H | 302 | 40.562 | 10.751 | -58.415 | 1.00 33.21 | C |
| HETATM13599 | C2 | RAM | H | 302 | 41.141 | 9.340 | -58.471 | 1.00 37.55 | C |
| HETATM13600 | C3 | RAM | H | 302 | 40.787 | 8.743 | -59.821 | 1.00 38.69 | C |
| HETATM13601 | C4 | RAM | H | 302 | 41.295 | 9.638 | -60.958 | 1.00 38.48 | C |
| HETATM13602 | C5 | RAM | H | 302 | 40.866 | 11.084 | -60.721 | 1.00 36.03 | C |
| HETATM13603 | C6 | RAM | H | 302 | 41.476 | 12.030 | -61.756 | 1.00 36.77 | C |
| HETATM13604 | O2 | RAM | H | 302 | 42.550 | 9.362 | -58.338 | 1.00 41.91 | O |
| HETATM13605 | O3 | RAM | H | 302 | 41.295 | 7.428 | -59.876 | 1.00 41.68 | O |
| HETATM13606 | O4 | RAM | H | 302 | 40.724 | 9.244 | -62.192 | 1.00 38.10 | O |
| HETATM13607 | O5 | RAM | H | 302 | 41.203 | 11.513 | -59.411 | 1.00 32.35 | O |
| HETATM13608 | C1 | RAM | H | 301 | 42.976 | 9.513 | -56.980 | 1.00 43.61 | C |
| HETATM13609 | C2 | RAM | H | 301 | 44.187 | 8.609 | -56.811 | 1.00 45.97 | C |
| HETATM13610 | C3 | RAM | H | 301 | 45.420 | 9.268 | -57.420 | 1.00 46.59 | C |
| HETATM13611 | C4 | RAM | H | 301 | 45.549 | 10.705 | -56.921 | 1.00 46.63 | C |
| HETATM13612 | C5 | RAM | H | 301 | 44.303 | 11.461 | -57.350 | 1.00 45.16 | C |
| HETATM13613 | C6 | RAM | H | 301 | 44.376 | 12.961 | -57.064 | 1.00 45.06 | C |
| HETATM13614 | O2 | RAM | H | 301 | 44.366 | 8.331 | -55.432 | 1.00 47.01 | O |
| HETATM13615 | O3 | RAM | H | 301 | 46.556 | 8.518 | -57.065 | 1.00 49.14 | O |
| HETATM13616 | O4 | RAM | H | 301 | 46.719 | 11.316 | -57.418 | 1.00 47.10 | O |
| HETATM13617 | O5 | RAM | H | 301 | 43.254 | 10.863 | -56.630 | 1.00 44.84 | O |
| HETATM13618 | C1 | GLC | H | 304 | 38.063 | 9.440 | -56.081 | 1.00 19.46 | C |
| HETATM13619 | C2 | GLC | H | 304 | 36.862 | 8.833 | -55.396 | 1.00 19.98 | C |
| HETATM13620 | C3 | GLC | H | 304 | 36.859 | 9.240 | -53.921 | 1.00 17.56 | C |
| HETATM13621 | C4 | GLC | H | 304 | 38.178 | 8.849 | -53.267 | 1.00 19.34 | C |
| HETATM13622 | C5 | GLC | H | 304 | 39.325 | 9.425 | -54.079 | 1.00 18.48 | C |
| HETATM13623 | C6 | GLC | H | 304 | 40.698 | 9.017 | -53.596 | 1.00 21.06 | C |
| HETATM13624 | O2 | GLC | H | 304 | 35.627 | 9.073 | -56.049 | 1.00 20.26 | O |
| HETATM13625 | O3 | GLC | H | 304 | 35.782 | 8.601 | -53.315 | 1.00 18.47 | O |
| HETATM13626 | O4 | GLC | H | 304 | 38.259 | 9.390 | -51.966 | 1.00 22.87 | O |
| HETATM13627 | O5 | GLC | H | 304 | 39.182 | 8.930 | -55.389 | 1.00 17.26 | O |
| HETATM13628 | O6 | GLC | H | 304 | 40.692 | 7.619 | -53.563 | 1.00 21.39 | O |
| HETATM13629 | C1 | GLC | H | 309 | 45.109 | 20.520 | -55.079 | 1.00 23.13 | C |
| HETATM13630 | C2 | GLC | H | 309 | 44.281 | 19.265 | -55.106 | 1.00 21.93 | C |
| HETATM13631 | C3 | GLC | H | 309 | 42.950 | 19.583 | -55.787 | 1.00 19.16 | C |
| HETATM13632 | C4 | GLC | H | 309 | 42.222 | 20.697 | -55.081 | 1.00 18.95 | C |
| HETATM13633 | C5 | GLC | H | 309 | 43.144 | 21.904 | -54.925 | 1.00 20.92 | C |
| HETATM13634 | C6 | GLC | H | 309 | 42.538 | 22.943 | -54.009 | 1.00 19.43 | C |
| HETATM13635 | O2 | GLC | H | 309 | 44.948 | 18.266 | -55.852 | 1.00 21.97 | O |
| HETATM13636 | O3 | GLC | H | 309 | 42.143 | 18.441 | -55.773 | 1.00 18.63 | O |
| HETATM13637 | O4 | GLC | H | 309 | 41.067 | 20.956 | -55.834 | 1.00 17.14 | O |
| HETATM13638 | O5 | GLC | H | 309 | 44.397 | 21.533 | -54.393 | 1.00 21.55 | O |

```
HETATM13639  O6  GLC H 309   43.421  24.034 -53.977  1.00 23.01          O
HETATM13640  O   HOH A 216   18.027  15.351  35.453  1.00 11.71          O
HETATM13641  O   HOH A 217   12.084  15.555  38.913  1.00  7.59          O
HETATM13642  O   HOH A 218    4.504  25.614  17.555  1.00 12.86          O
HETATM13643  O   HOH A 219   12.225  25.661  39.126  1.00 14.28          O
HETATM13644  O   HOH A 220   15.507  10.784  15.360  1.00 16.32          O
HETATM13645  O   HOH A 221    5.345  13.938  35.858  1.00 11.85          O
HETATM13646  O   HOH A 222   22.609   9.879  16.249  1.00 16.04          O
HETATM13647  O   HOH A 223    1.273  27.675  24.013  1.00 17.50          O
HETATM13648  O   HOH A 224   10.986  30.210  28.594  1.00 14.02          O
HETATM13649  O   HOH A 225   18.373  12.887  35.986  1.00 14.34          O
HETATM13650  O   HOH A 226   10.891  31.555  30.871  1.00 13.45          O
HETATM13651  O   HOH A 227   38.097  11.045   0.830  1.00 16.70          O
HETATM13652  O   HOH A 228   14.480  27.473  22.802  1.00 18.34          O
HETATM13653  O   HOH A 229   26.283  22.872 -19.484  1.00 19.56          O
HETATM13654  O   HOH A 230   37.298  23.838 -24.320  1.00 14.52          O
HETATM13655  O   HOH A 231   21.031   9.860  33.394  1.00 19.04          O
HETATM13656  O   HOH A 232   16.548  20.720  37.089  1.00 18.56          O
HETATM13657  O   HOH A 233   32.033  12.059   8.977  1.00 19.40          O
HETATM13658  O   HOH A 234   16.789  11.616  37.774  1.00 14.53          O
HETATM13659  O   HOH A 235   11.860  24.544  17.332  1.00 20.10          O
HETATM13660  O   HOH A 236   28.685  10.671  17.222  1.00 20.52          O
HETATM13661  O   HOH A 237   17.696  26.363  20.833  1.00 21.55          O
HETATM13662  O   HOH A 238   14.005  13.788  39.547  1.00 17.97          O
HETATM13663  O   HOH A 239   44.148  11.475   2.813  1.00 15.85          O
HETATM13664  O   HOH A 240   13.937  10.048  34.326  1.00 18.64          O
HETATM13665  O   HOH A 241   36.812   8.940   2.349  1.00 22.91          O
HETATM13666  O   HOH A 242   44.554  24.171   2.960  1.00 21.87          O
HETATM13667  O   HOH A 243   42.508  11.410  -4.577  1.00 24.82          O
HETATM13668  O   HOH A 244    9.211  33.677  31.101  1.00 19.29          O
HETATM13669  O   HOH A 245   29.479  13.280   9.135  1.00 22.19          O
HETATM13670  O   HOH A 246    3.427  16.340  41.747  1.00 19.56          O
HETATM13671  O   HOH A 247   -1.843  18.552  30.324  1.00 18.49          O
HETATM13672  O   HOH A 248   26.646  14.223 -20.830  1.00 21.39          O
HETATM13673  O   HOH A 249    5.471  15.781  38.538  1.00 18.71          O
HETATM13674  O   HOH A 250   30.767  20.801  -2.243  1.00 17.96          O
HETATM13675  O   HOH A 251   28.265  18.467  -1.925  1.00 24.39          O
HETATM13676  O   HOH A 252   10.816  26.533  18.415  1.00 29.58          O
HETATM13677  O   HOH A 253   32.312   7.546  18.082  1.00 27.25          O
HETATM13678  O   HOH A 254   39.009  25.862 -23.664  1.00 22.16          O
HETATM13679  O   HOH A 255   54.342  18.513   9.074  1.00 17.27          O
HETATM13680  O   HOH A 256   49.963  15.325   1.293  1.00 24.97          O
HETATM13681  O   HOH A 257   31.614  15.379 -13.062  1.00 21.45          O
HETATM13682  O   HOH A 258   26.624   4.495  19.705  1.00 20.50          O
HETATM13683  O   HOH A 259   16.809  13.741  39.852  1.00 23.49          O
HETATM13684  O   HOH A 260   46.807  19.482   8.233  1.00 23.03          O
HETATM13685  O   HOH A 261   16.397   7.413  21.762  1.00 28.39          O
HETATM13686  O   HOH A 262    9.234  28.091  22.357  1.00 18.81          O
HETATM13687  O   HOH A 263   34.008  15.676 -10.851  1.00 23.58          O
HETATM13688  O   HOH A 264   26.855  16.968  17.048  1.00 20.88          O
HETATM13689  O   HOH A 265   13.790  11.894  37.442  1.00 20.15          O
HETATM13690  O   HOH A 266   44.573  11.205  12.585  1.00 21.59          O
HETATM13691  O   HOH A 267   33.699  13.792 -22.677  1.00 25.38          O
HETATM13692  O   HOH A 268    3.002  29.533  35.426  1.00 30.93          O
HETATM13693  O   HOH A 269   34.350  19.797  16.582  1.00 26.15          O
HETATM13694  O   HOH A 270    9.367   8.440  12.094  1.00 25.92          O
HETATM13695  O   HOH A 271   52.033  15.954   7.729  1.00 28.67          O
HETATM13696  O   HOH A 272   39.799  10.015   7.971  1.00 19.92          O
HETATM13697  O   HOH A 273   41.320  21.777  12.887  1.00 25.30          O
HETATM13698  O   HOH A 274   -2.209  26.802  29.899  1.00 29.47          O
HETATM13699  O   HOH A 275   11.447  29.739  21.877  1.00 29.13          O
HETATM13700  O   HOH A 276   19.666  16.145  13.985  1.00 33.22          O
HETATM13701  O   HOH A 277   14.550  25.069  16.081  1.00 24.84          O
HETATM13702  O   HOH A 278    3.630  12.645  39.767  1.00 24.98          O
HETATM13703  O   HOH A 279   32.648  17.057 -25.781  1.00 27.54          O
```

```
HETATM13704  O   HOH A 280    35.244    7.410   18.596  1.00 35.35           O
HETATM13705  O   HOH A 281    47.350   22.919   11.231  1.00 27.56           O
HETATM13706  O   HOH A 282    45.777   16.664    8.999  1.00 30.28           O
HETATM13707  O   HOH A 283     1.208   12.239   26.704  1.00 21.64           O
HETATM13708  O   HOH A 284    15.521    7.764   35.328  1.00 25.40           O
HETATM13709  O   HOH A 285    18.275   17.485   12.276  1.00 32.48           O
HETATM13710  O   HOH A 286    -0.764   12.761   28.532  1.00 23.99           O
HETATM13711  O   HOH A 287    21.455   29.262   22.029  1.00 32.79           O
HETATM13712  O   HOH A 288    49.711   16.601    6.426  1.00 17.04           O
HETATM13713  O   HOH A 289    -4.590   25.080   29.646  1.00 29.40           O
HETATM13714  O   HOH A 290    31.149   27.426    0.393  1.00 31.03           O
HETATM13715  O   HOH A 291    26.631   18.731   28.975  1.00 37.70           O
HETATM13716  O   HOH A 292    20.252    7.156   21.482  1.00 35.61           O
HETATM13717  O   HOH A 293    47.777   22.761   -9.266  1.00 26.28           O
HETATM13718  O   HOH A 294    41.468   28.376    1.708  1.00 51.80           O
HETATM13719  O   HOH A 295     0.944   21.155   23.776  1.00 25.44           O
HETATM13720  O   HOH A 296    10.423    9.281   14.653  1.00 27.19           O
HETATM13721  O   HOH A 297    34.295   28.352  -15.445  1.00 29.71           O
HETATM13722  O   HOH A 298    45.670   29.200  -12.745  1.00 35.08           O
HETATM13723  O   HOH A 299    38.386   26.313    3.542  1.00 33.77           O
HETATM13724  O   HOH A 300    19.793   24.304   26.853  1.00 26.55           O
HETATM13725  O   HOH A 301    24.911   15.263    7.702  1.00 38.37           O
HETATM13726  O   HOH A 302    10.784   30.030   33.073  1.00 29.64           O
HETATM13727  O   HOH A 303    10.460    6.822   20.908  1.00 32.34           O
HETATM13728  O   HOH A 304    28.263   19.945   10.854  1.00 37.47           O
HETATM13729  O   HOH A 305    40.872   27.799   -2.662  1.00 31.10           O
HETATM13730  O   HOH A 306    43.329   26.280    3.675  1.00 32.75           O
HETATM13731  O   HOH A 307    46.953   13.121  -12.782  1.00 32.55           O
HETATM13732  O   HOH A 308    18.249   23.574   16.817  1.00 39.89           O
HETATM13733  O   HOH A 309    36.024   25.338    9.796  1.00 30.47           O
HETATM13734  O   HOH A 310    12.395   29.571   35.072  1.00 36.38           O
HETATM13735  O   HOH A 311    29.289   18.598   -4.690  1.00 24.72           O
HETATM13736  O   HOH A 312    36.896   17.006  -23.546  1.00 27.32           O
HETATM13737  O   HOH A 313    -5.705   25.284   32.046  1.00 32.95           O
HETATM13738  O   HOH A 314    32.505   31.904   -6.565  1.00 38.05           O
HETATM13739  O   HOH A 315    21.740   23.129   27.577  1.00 38.07           O
HETATM13740  O   HOH A 316    30.808   12.487   33.425  1.00 37.51           O
HETATM13741  O   HOH A 317    33.599   27.700    3.196  1.00 35.15           O
HETATM13742  O   HOH A 318    30.865   24.860   -6.843  1.00 35.78           O
HETATM13743  O   HOH A 319    17.215    9.104   37.785  1.00 29.03           O
HETATM13744  O   HOH A 320    10.311   13.829   38.117  1.00 18.70           O
HETATM13745  O   HOH A 321    47.076   24.804    1.989  1.00 34.35           O
HETATM13746  O   HOH A 322    16.619   15.587   10.226  1.00 37.10           O
HETATM13747  O   HOH A 323     7.336   24.873   39.662  1.00 26.23           O
HETATM13748  O   HOH A 324    17.001   23.559   33.779  1.00 24.09           O
HETATM13749  O   HOH A 325    17.278    6.071   33.477  1.00 41.42           O
HETATM13750  O   HOH A 326     0.184   19.402   41.433  1.00 32.76           O
HETATM13751  O   HOH A 327    44.904   11.795   -6.023  1.00 30.28           O
HETATM13752  O   HOH A 328     6.621    5.113   24.909  1.00 39.44           O
HETATM13753  O   HOH A 329    29.501    5.024   21.198  1.00 33.47           O
HETATM13754  O   HOH A 330    14.420   27.970   16.008  1.00 41.70           O
HETATM13755  O   HOH A 331    17.133   26.723   29.068  1.00 30.95           O
HETATM13756  O   HOH A 332    48.838   24.918    9.437  1.00 39.74           O
HETATM13757  O   HOH A 333    18.724   26.291   16.211  1.00 46.71           O
HETATM13758  O   HOH A 334     7.501   33.450   32.838  1.00 30.41           O
HETATM13759  O   HOH A 335    12.532    7.890   14.147  1.00 32.26           O
HETATM13760  O   HOH A 336    27.201   20.882   20.970  1.00 48.01           O
HETATM13761  O   HOH A 337    51.906   18.739    2.500  1.00 29.08           O
HETATM13762  O   HOH A 338    15.271   29.497   18.899  1.00 29.16           O
HETATM13763  O   HOH A 339    17.094   27.116   31.572  1.00 29.14           O
HETATM13764  O   HOH A 340     7.097   29.492   21.570  1.00 24.41           O
HETATM13765  O   HOH A 341     7.870   22.991   41.511  1.00 24.90           O
HETATM13766  O   HOH A 342     0.242   31.926   26.891  1.00 40.28           O
HETATM13767  O   HOH A 343    46.195   14.302  -19.659  1.00 49.14           O
HETATM13768  O   HOH A 344    43.874   30.536  -10.181  1.00 40.24           O
```

```
HETATM13769  O   HOH A 345   23.849  21.368  27.358  1.00 40.09        O
HETATM13770  O   HOH A 346   27.174   4.613  27.473  1.00 42.65        O
HETATM13771  O   HOH A 347   40.354   9.859  -5.747  1.00 32.14        O
HETATM13772  O   HOH A 348   25.306  15.595  34.817  1.00 41.97        O
HETATM13773  O   HOH A 349   26.650  19.168  13.600  1.00 35.01        O
HETATM13774  O   HOH A 350   50.270  24.997   7.318  1.00 37.33        O
HETATM13775  O   HOH A 351   16.311  18.531  39.058  1.00 18.39        O
HETATM13776  O   HOH A 352   22.778  23.112  30.303  1.00 31.56        O
HETATM13777  O   HOH A 353   22.746  17.320  14.567  1.00 43.03        O
HETATM13778  O   HOH A 354   44.696  12.070  -8.489  1.00 30.28        O
HETATM13779  O   HOH A 355   10.963   5.630  33.014  1.00 27.64        O
HETATM13780  O   HOH A 356   31.368  30.204  -4.462  1.00 34.60        O
HETATM13781  O   HOH A 357   22.676  17.607  34.930  1.00 15.55        O
HETATM13782  O   HOH A 358   33.113  20.412   8.510  1.00 31.13        O
HETATM13783  O   HOH A 359   20.872  23.494  17.448  1.00 28.42        O
HETATM13784  O   HOH A 360   53.250  19.244  11.321  1.00 18.02        O
HETATM13785  O   HOH A 361   13.102   9.696  37.993  1.00 29.74        O
HETATM13786  O   HOH A 362   17.940  29.653  23.234  1.00 36.99        O
HETATM13787  O   HOH A 363   21.501   9.126  11.857  1.00 35.15        O
HETATM13788  O   HOH A 364   24.140  27.889  22.625  1.00 32.05        O
HETATM13789  O   HOH A 365   48.721  31.863  -5.776  1.00 38.20        O
HETATM13790  O   HOH A 366   12.954   2.851  28.038  1.00 31.54        O
HETATM13791  O   HOH A 367   26.653  22.752   4.436  1.00 34.60        O
HETATM13792  O   HOH A 368   47.047  13.155  -5.703  1.00 33.24        O
HETATM13793  O   HOH A 369   18.926  29.910  25.633  1.00 40.86        O
HETATM13794  O   HOH A 370   28.204  19.297  15.639  1.00 33.17        O
HETATM13795  O   HOH A 371   -3.828  13.875  31.402  1.00 29.34        O
HETATM13796  O   HOH A 372   12.931   9.504  11.068  1.00 35.61        O
HETATM13797  O   HOH A 373   30.175  27.091  -8.024  1.00 38.87        O
HETATM13798  O   HOH A 374    2.715  32.716  31.819  1.00 37.87        O
HETATM13799  O   HOH A 375   33.846  16.480 -23.882  1.00 32.05        O
HETATM13800  O   HOH A 376   -1.517  27.565  32.415  1.00 53.06        O
HETATM13801  O   HOH A 377   53.188  15.879   4.965  1.00 33.40        O
HETATM13802  O   HOH A 378   18.645  31.808  27.506  1.00 34.33        O
HETATM13803  O   HOH A 379    3.919  24.967  39.621  1.00 38.88        O
HETATM13804  O   HOH A 380    3.449  32.103  34.332  1.00 36.30        O
HETATM13805  O   HOH A 381   44.617  11.216   6.510  1.00 35.15        O
HETATM13806  O   HOH A 382   26.354  20.759 -17.283  1.00 32.77        O
HETATM13807  O   HOH A 383   36.983  22.604  15.509  1.00 36.93        O
HETATM13808  O   HOH A 384   15.653  34.917  29.068  1.00 35.12        O
HETATM13809  O   HOH A 385    4.218  30.079  18.536  1.00 31.97        O
HETATM13810  O   HOH A 386   34.347  29.524  -1.047  1.00 36.52        O
HETATM13811  O   HOH A 387   46.474  12.702 -17.651  1.00 46.72        O
HETATM13812  O   HOH A 388   51.096  30.603  -8.409  1.00 37.40        O
HETATM13813  O   HOH A 389   -3.846  22.685  35.280  1.00 50.52        O
HETATM13814  O   HOH A 390    4.562  11.854  36.929  1.00 24.49        O
HETATM13815  O   HOH A 391   18.076  28.047  18.965  1.00 35.24        O
HETATM13816  O   HOH A 392   49.187  16.733 -10.499  1.00 30.24        O
HETATM13817  O   HOH A 393   16.125  25.977  33.777  1.00 35.33        O
HETATM13818  O   HOH A 394   38.655   8.898  11.001  1.00 29.85        O
HETATM13819  O   HOH A 395   33.870  29.452 -20.035  1.00 28.64        O
HETATM13820  O   HOH A 396   11.352   7.353  35.329  1.00 40.95        O
HETATM13821  O   HOH A 397   37.412   8.253  19.875  1.00 39.45        O
HETATM13822  O   HOH A 398   25.353  12.748  10.290  1.00 35.30        O
HETATM13823  O   HOH A 399   41.815   9.722  16.056  1.00 28.43        O
HETATM13824  O   HOH A 400   39.222   6.523  18.882  1.00 34.88        O
HETATM13825  O   HOH A 401   41.061   7.949  -0.760  1.00 40.07        O
HETATM13826  O   HOH A 402   38.427  32.863 -11.896  1.00 43.40        O
HETATM13827  O   HOH A 403   21.695  26.087  15.606  1.00 27.59        O
HETATM13828  O   HOH A 404   17.009  22.096  13.908  1.00 40.18        O
HETATM13829  O   HOH A 405   30.271   3.657  23.242  1.00 47.15        O
HETATM13830  O   HOH A 406   14.045  22.738  14.902  1.00 37.78        O
HETATM13831  O   HOH A 407   50.316  15.165   4.156  1.00 32.79        O
HETATM13832  O   HOH A 408   -6.024  27.627  23.404  1.00 37.08        O
HETATM13833  O   HOH A 409   10.161  35.694  31.429  1.00 31.70        O
```

| HETATM13834 | O | HOH A 410 | 15.032 | 29.478 | 21.323 | 1.00 | 29.89 | O |
|---|---|---|---|---|---|---|---|---|
| HETATM13835 | O | HOH A 411 | 47.680 | 14.891 | 8.373 | 1.00 | 44.12 | O |
| HETATM13836 | O | HOH A 412 | 27.101 | 25.471 | 2.719 | 1.00 | 48.53 | O |
| HETATM13837 | O | HOH A 413 | 40.517 | 18.615 | 19.369 | 1.00 | 39.29 | O |
| HETATM13838 | O | HOH A 414 | 6.038 | 19.931 | 38.559 | 1.00 | 29.68 | O |
| HETATM13839 | O | HOH A 415 | 37.109 | 12.114 | -19.605 | 1.00 | 34.36 | O |
| HETATM13840 | O | HOH A 416 | 19.063 | 28.341 | 27.845 | 1.00 | 40.85 | O |
| HETATM13841 | O | HOH A 417 | 28.035 | 21.112 | -14.780 | 1.00 | 40.38 | O |
| HETATM13842 | O | HOH A 418 | 18.128 | 6.560 | 20.538 | 1.00 | 43.73 | O |
| HETATM13843 | O | HOH A 419 | 24.975 | 10.322 | 10.930 | 1.00 | 36.36 | O |
| HETATM13844 | O | HOH A 420 | 41.502 | 24.541 | 9.869 | 1.00 | 47.85 | O |
| HETATM13845 | O | HOH A 421 | 4.441 | 9.481 | 36.323 | 1.00 | 37.68 | O |
| HETATM13846 | O | HOH A 422 | 28.406 | 7.439 | 35.474 | 1.00 | 47.01 | O |
| HETATM13847 | O | HOH A 423 | 28.171 | 23.738 | -14.266 | 1.00 | 41.63 | O |
| HETATM13848 | O | HOH A 424 | 40.348 | 15.905 | -2.875 | 1.00 | 69.90 | O |
| HETATM13849 | O | HOH A 425 | 29.354 | 21.637 | -9.322 | 1.00 | 41.75 | O |
| HETATM13850 | O | HOH A 426 | 8.530 | 7.405 | 9.619 | 1.00 | 49.06 | O |
| HETATM13851 | O | HOH A 427 | 42.151 | 12.543 | -9.066 | 1.00 | 42.02 | O |
| HETATM13852 | O | HOH A 428 | 12.507 | 34.108 | 33.153 | 1.00 | 39.16 | O |
| HETATM13853 | O | HOH A 429 | 48.014 | 14.085 | 10.445 | 1.00 | 38.87 | O |
| HETATM13854 | O | HOH A 430 | 37.985 | 20.407 | 19.860 | 1.00 | 46.15 | O |
| HETATM13855 | O | HOH A 431 | -0.923 | 32.205 | 21.727 | 1.00 | 40.20 | O |
| HETATM13856 | O | HOH A 432 | 15.667 | 0.020 | 33.608 | 1.00 | 44.80 | O |
| HETATM13857 | O | HOH A 433 | 19.769 | 22.554 | 34.435 | 1.00 | 32.05 | O |
| HETATM13858 | O | HOH A 434 | 42.697 | 21.861 | -24.505 | 1.00 | 38.86 | O |
| HETATM13859 | O | HOH A 435 | 28.354 | 11.629 | 35.888 | 1.00 | 33.57 | O |
| HETATM13860 | O | HOH A 436 | 43.333 | 17.930 | 20.880 | 1.00 | 40.16 | O |
| HETATM13861 | O | HOH A 437 | 22.115 | 23.653 | 15.620 | 1.00 | 30.95 | O |
| HETATM13862 | O | HOH A 438 | 49.007 | 14.396 | -10.167 | 1.00 | 34.46 | O |
| HETATM13863 | O | HOH A 439 | 22.219 | 21.444 | 33.127 | 1.00 | 42.82 | O |
| HETATM13864 | O | HOH A 440 | 49.731 | 20.871 | 14.721 | 1.00 | 48.37 | O |
| HETATM13865 | O | HOH A 441 | 34.995 | 11.555 | -11.978 | 1.00 | 39.25 | O |
| HETATM13866 | O | HOH A 442 | 42.803 | 13.327 | 20.511 | 1.00 | 34.64 | O |
| HETATM13867 | O | HOH A 443 | 36.102 | 13.307 | -21.802 | 1.00 | 36.87 | O |
| HETATM13868 | O | HOH A 444 | 48.411 | 24.387 | 5.548 | 1.00 | 45.02 | O |
| HETATM13869 | O | HOH A 445 | 27.934 | 21.327 | 16.941 | 1.00 | 50.21 | O |
| HETATM13870 | O | HOH A 446 | 48.877 | 13.421 | -0.731 | 1.00 | 39.57 | O |
| HETATM13871 | O | HOH A 447 | 8.512 | 30.412 | 34.254 | 1.00 | 38.07 | O |
| HETATM13872 | O | HOH A 448 | 54.275 | 20.976 | 4.546 | 1.00 | 41.63 | O |
| HETATM13873 | O | HOH A 449 | 11.047 | 9.717 | 39.144 | 1.00 | 38.97 | O |
| HETATM13874 | O | HOH A 450 | 28.600 | 11.236 | 8.064 | 1.00 | 24.45 | O |
| HETATM13875 | O | HOH A 451 | 48.498 | 29.386 | -1.862 | 1.00 | 58.00 | O |
| HETATM13876 | O | HOH A 452 | 24.781 | 3.601 | 36.360 | 1.00 | 43.97 | O |
| HETATM13877 | O | HOH A 453 | 46.685 | 23.582 | -20.092 | 1.00 | 48.31 | O |
| HETATM13878 | O | HOH A 454 | 31.580 | 21.668 | 15.950 | 1.00 | 45.44 | O |
| HETATM13879 | O | HOH A 455 | 20.181 | 22.553 | 13.274 | 1.00 | 50.08 | O |
| HETATM13880 | O | HOH A 456 | 21.880 | 19.841 | 12.675 | 1.00 | 48.32 | O |
| HETATM13881 | O | HOH A 457 | 13.377 | 7.120 | 11.914 | 1.00 | 42.73 | O |
| HETATM13882 | O | HOH A 458 | 22.413 | 14.009 | 8.248 | 1.00 | 40.80 | O |
| HETATM13883 | O | HOH A 459 | 28.947 | 21.825 | 19.595 | 1.00 | 47.25 | O |
| HETATM13884 | O | HOH A 460 | 44.258 | 24.203 | 11.631 | 1.00 | 49.21 | O |
| HETATM13885 | O | HOH A 461 | 16.114 | 6.418 | 16.664 | 1.00 | 49.49 | O |
| HETATM13886 | O | HOH A 462 | 39.853 | 15.691 | 20.628 | 1.00 | 39.24 | O |
| HETATM13887 | O | HOH A 463 | 45.693 | 33.121 | -7.466 | 1.00 | 38.26 | O |
| HETATM13888 | O | HOH A 464 | 7.103 | 32.365 | 20.984 | 1.00 | 40.45 | O |
| HETATM13889 | O | HOH A 465 | 26.602 | 16.788 | 32.754 | 1.00 | 37.76 | O |
| HETATM13890 | O | HOH A 466 | 29.330 | 11.106 | -22.582 | 1.00 | 37.35 | O |
| HETATM13891 | O | HOH A 467 | 35.078 | 7.062 | 28.619 | 1.00 | 56.35 | O |
| HETATM13892 | O | HOH A 468 | 42.594 | 19.227 | -24.591 | 1.00 | 46.56 | O |
| HETATM13893 | O | HOH A 469 | 48.127 | 25.018 | -18.304 | 1.00 | 41.13 | O |
| HETATM13894 | O | HOH A 470 | 31.248 | 18.763 | 8.887 | 1.00 | 35.90 | O |
| HETATM13895 | O | HOH A 471 | 50.917 | 27.490 | 6.897 | 1.00 | 43.78 | O |
| HETATM13896 | O | HOH A 472 | -3.441 | 29.630 | 24.060 | 1.00 | 41.49 | O |
| HETATM13897 | O | HOH A 473 | 26.480 | 23.504 | 14.528 | 1.00 | 35.36 | O |
| HETATM13898 | O | HOH A 474 | 34.246 | 34.066 | -6.419 | 1.00 | 42.27 | O |

| | | | | | | | | | |
|---|---|---|---|---|---|---|---|---|---|
| HETATM13899 | O | HOH | A | 475 | 2.890 | 9.049 | 33.827 | 1.00 | 38.86 | O |
| HETATM13900 | O | HOH | A | 476 | 17.850 | 4.473 | 36.125 | 1.00 | 49.86 | O |
| HETATM13901 | O | HOH | A | 477 | 32.920 | 5.590 | 25.964 | 1.00 | 41.96 | O |
| HETATM13902 | O | HOH | A | 478 | 8.750 | 5.828 | 34.227 | 1.00 | 33.98 | O |
| HETATM13903 | O | HOH | A | 479 | 21.228 | 25.677 | 32.319 | 1.00 | 43.79 | O |
| HETATM13904 | O | HOH | A | 480 | 29.417 | 23.914 | 22.157 | 1.00 | 41.36 | O |
| HETATM13905 | O | HOH | A | 481 | 7.996 | 9.569 | 36.530 | 1.00 | 43.33 | O |
| HETATM13906 | O | HOH | A | 482 | 20.328 | 26.182 | 28.081 | 1.00 | 46.58 | O |
| HETATM13907 | O | HOH | A | 483 | 35.382 | 33.326 | -4.309 | 1.00 | 54.12 | O |
| HETATM13908 | O | HOH | A | 484 | -2.364 | 23.499 | 33.255 | 1.00 | 33.95 | O |
| HETATM13909 | O | HOH | A | 485 | 45.062 | 21.670 | 21.137 | 1.00 | 59.67 | O |
| HETATM13910 | O | HOH | A | 486 | 51.365 | 13.649 | 8.144 | 1.00 | 37.66 | O |
| HETATM13911 | O | HOH | A | 487 | 53.559 | 23.481 | 4.460 | 1.00 | 34.11 | O |
| HETATM13912 | O | HOH | A | 488 | 1.837 | 11.129 | 38.862 | 1.00 | 44.37 | O |
| HETATM13913 | O | HOH | B | 316 | -12.374 | 26.093 | 9.909 | 1.00 | 43.17 | O |
| HETATM13914 | O | HOH | B | 317 | 7.022 | 25.890 | 18.549 | 1.00 | 14.14 | O |
| HETATM13915 | O | HOH | B | 318 | 7.952 | 9.290 | 18.640 | 1.00 | 15.81 | O |
| HETATM13916 | O | HOH | B | 319 | 11.961 | 16.912 | -4.891 | 1.00 | 19.55 | O |
| HETATM13917 | O | HOH | B | 320 | -11.043 | 13.455 | 22.138 | 1.00 | 19.67 | O |
| HETATM13918 | O | HOH | B | 321 | 10.142 | 20.502 | -1.838 | 1.00 | 17.51 | O |
| HETATM13919 | O | HOH | B | 322 | -12.255 | 9.991 | 15.127 | 1.00 | 22.15 | O |
| HETATM13920 | O | HOH | B | 323 | -12.408 | 16.167 | 8.910 | 1.00 | 14.57 | O |
| HETATM13921 | O | HOH | B | 324 | 0.582 | 8.894 | 2.544 | 1.00 | 23.32 | O |
| HETATM13922 | O | HOH | B | 325 | 7.839 | 8.460 | 15.925 | 1.00 | 22.42 | O |
| HETATM13923 | O | HOH | B | 326 | 14.935 | 26.183 | -10.946 | 1.00 | 31.23 | O |
| HETATM13924 | O | HOH | B | 327 | -8.131 | 19.288 | 4.574 | 1.00 | 22.33 | O |
| HETATM13925 | O | HOH | B | 328 | 28.644 | -6.704 | -8.936 | 1.00 | 24.53 | O |
| HETATM13926 | O | HOH | B | 329 | 2.858 | 27.250 | 16.037 | 1.00 | 18.90 | O |
| HETATM13927 | O | HOH | B | 330 | 22.694 | 3.409 | 0.285 | 1.00 | 19.61 | O |
| HETATM13928 | O | HOH | B | 331 | 0.521 | 11.327 | 24.028 | 1.00 | 17.12 | O |
| HETATM13929 | O | HOH | B | 332 | 7.426 | 27.569 | -9.333 | 1.00 | 21.74 | O |
| HETATM13930 | O | HOH | B | 333 | 32.532 | 7.752 | 2.154 | 1.00 | 21.29 | O |
| HETATM13931 | O | HOH | B | 334 | -2.525 | 11.238 | 19.060 | 1.00 | 15.97 | O |
| HETATM13932 | O | HOH | B | 335 | 40.754 | -4.266 | -0.692 | 1.00 | 25.28 | O |
| HETATM13933 | O | HOH | B | 336. | 20.417 | 19.514 | -14.221 | 1.00 | 22.06 | O |
| HETATM13934 | O | HOH | B | 337 | -8.644 | 19.193 | 26.729 | 1.00 | 27.47 | O |
| HETATM13935 | O | HOH | B | 338 | 21.591 | -0.097 | -7.756 | 1.00 | 23.71 | O |
| HETATM13936 | O | HOH | B | 339 | 2.678 | 20.795 | -6.645 | 1.00 | 24.90 | O |
| HETATM13937 | O | HOH | B | 340 | 24.981 | 3.995 | -15.202 | 1.00 | 28.51 | O |
| HETATM13938 | O | HOH | B | 341 | 0.219 | 9.819 | 20.436 | 1.00 | 24.00 | O |
| HETATM13939 | O | HOH | B | 342 | 32.557 | -2.205 | -11.929 | 1.00 | 31.32 | O |
| HETATM13940 | O | HOH | B | 343 | -5.609 | 25.108 | 24.880 | 1.00 | 22.55 | O |
| HETATM13941 | O | HOH | B | 344 | -15.049 | 9.533 | 18.573 | 1.00 | 29.70 | O |
| HETATM13942 | O | HOH | B | 345 | 5.551 | 34.727 | -1.045 | 1.00 | 27.79 | O |
| HETATM13943 | O | HOH | B | 346 | 30.619 | -0.286 | -10.463 | 1.00 | 26.95 | O |
| HETATM13944 | O | HOH | B | 347 | -12.380 | 19.346 | 20.990 | 1.00 | 28.87 | O |
| HETATM13945 | O | HOH | B | 348 | 13.978 | 23.598 | -2.081 | 1.00 | 27.78 | O |
| HETATM13946 | O | HOH | B | 349 | 9.188 | 15.032 | 0.321 | 1.00 | 24.83 | O |
| HETATM13947 | O | HOH | B | 350 | 17.630 | 7.512 | -4.157 | 1.00 | 33.55 | O |
| HETATM13948 | O | HOH | B | 351 | 9.887 | 18.171 | -3.561 | 1.00 | 22.80 | O |
| HETATM13949 | O | HOH | B | 352 | -11.896 | 25.856 | 21.876 | 1.00 | 30.58 | O |
| HETATM13950 | O | HOH | B | 353 | 11.000 | 29.349 | -1.387 | 1.00 | 25.82 | O |
| HETATM13951 | O | HOH | B | 354 | -5.091 | 1.633 | 18.940 | 1.00 | 22.48 | O |
| HETATM13952 | O | HOH | B | 355 | 1.305 | 31.284 | 14.957 | 1.00 | 26.55 | O |
| HETATM13953 | O | HOH | B | 356 | 6.070 | 36.058 | 0.927 | 1.00 | 36.40 | O |
| HETATM13954 | O | HOH | B | 357 | 40.067 | 6.360 | -2.635 | 1.00 | 26.19 | O |
| HETATM13955 | O | HOH | B | 358 | 18.248 | 16.601 | -0.299 | 1.00 | 28.64 | O |
| HETATM13956 | O | HOH | B | 359 | 11.134 | 22.068 | 2.032 | 1.00 | 23.91 | O |
| HETATM13957 | O | HOH | B | 360 | 33.531 | 2.016 | 5.239 | 1.00 | 35.90 | O |
| HETATM13958 | O | HOH | B | 361 | 23.991 | 24.350 | -7.630 | 1.00 | 31.34 | O |
| HETATM13959 | O | HOH | B | 362 | 7.154 | 27.180 | 7.093 | 1.00 | 24.08 | O |
| HETATM13960 | O | HOH | B | 363 | 10.280 | 28.516 | 5.324 | 1.00 | 33.48 | O |
| HETATM13961 | O | HOH | B | 364 | 39.203 | 5.719 | 1.012 | 1.00 | 30.37 | O |
| HETATM13962 | O | HOH | B | 365 | 9.728 | 22.556 | -14.622 | 1.00 | 26.17 | O |
| HETATM13963 | O | HOH | B | 366 | 6.587 | 6.632 | 20.800 | 1.00 | 23.84 | O |

```
HETATM13964  O   HOH B 367      -8.484  17.187  32.142  1.00 32.03           O
HETATM13965  O   HOH B 368       1.320   4.238   7.264  1.00 30.73           O
HETATM13966  O   HOH B 369      25.178  12.372   5.882  1.00 36.11           O
HETATM13967  O   HOH B 370     -19.031  13.906  14.438  1.00 31.77           O
HETATM13968  O   HOH B 371      30.849   5.856 -14.206  1.00 33.26           O
HETATM13969  O   HOH B 372     -19.455  12.564  12.290  1.00 32.40           O
HETATM13970  O   HOH B 373      26.574  20.374  -1.640  1.00 25.20           O
HETATM13971  O   HOH B 374      -3.078  21.630  31.290  1.00 32.83           O
HETATM13972  O   HOH B 375      -9.351  26.971  25.023  1.00 36.11           O
HETATM13973  O   HOH B 376      15.530  23.746  12.307  1.00 33.20           O
HETATM13974  O   HOH B 377      20.654  18.576   1.268  1.00 26.70           O
HETATM13975  O   HOH B 378       9.255  35.959  -0.788  1.00 25.48           O
HETATM13976  O   HOH B 379     -14.628   7.468  13.295  1.00 28.59           O
HETATM13977  O   HOH B 380     -10.485  26.374  16.859  1.00 40.94           O
HETATM13978  O   HOH B 381      25.132  16.488 -17.729  1.00 21.27           O
HETATM13979  O   HOH B 382     -13.781  22.987  15.726  1.00 33.22           O
HETATM13980  O   HOH B 383     -11.789  23.803  30.936  1.00 38.34           O
HETATM13981  O   HOH B 384      13.508  19.480   1.420  1.00 31.34           O
HETATM13982  O   HOH B 385      -4.875  21.902  -3.708  1.00 30.45           O
HETATM13983  O   HOH B 386      12.939  14.755   8.202  1.00 30.25           O
HETATM13984  O   HOH B 387      -1.170  30.224  17.508  1.00 23.05           O
HETATM13985  O   HOH B 388       8.373  16.796  -1.720  1.00 26.99           O
HETATM13986  O   HOH B 389      21.738  16.279   1.444  1.00 21.84           O
HETATM13987  O   HOH B 390      38.828   5.265 -11.178  1.00 27.06           O
HETATM13988  O   HOH B 391      -0.997   0.653  18.611  1.00 40.42           O
HETATM13989  O   HOH B 392      -1.068   8.095  22.536  1.00 30.12           O
HETATM13990  O   HOH B 393     -16.870  20.982  18.500  1.00 40.38           O
HETATM13991  O   HOH B 394     -11.310  23.590  10.061  1.00 25.65           O
HETATM13992  O   HOH B 395      39.226   8.187  -4.274  1.00 33.60           O
HETATM13993  O   HOH B 396      12.459  12.436   8.527  1.00 29.88           O
HETATM13994  O   HOH B 397      42.225  10.647  -8.399  1.00 41.24           O
HETATM13995  O   HOH B 398      13.988   9.384 -14.427  1.00 32.72           O
HETATM13996  O   HOH B 399      11.793  32.205  -5.636  1.00 52.46           O
HETATM13997  O   HOH B 400      -4.424  29.195  -3.206  1.00 21.94           O
HETATM13998  O   HOH B 401      -0.287  17.314  -3.155  1.00 38.44           O
HETATM13999  O   HOH B 402      19.430   5.845 -14.795  1.00 29.76           O
HETATM14000  O   HOH B 403      20.291  11.650   0.784  1.00 32.64           O
HETATM14001  O   HOH B 404      34.462   9.381 -13.625  1.00 32.88           O
HETATM14002  O   HOH B 405       5.944   6.302  11.928  1.00 38.35           O
HETATM14003  O   HOH B 406      26.186  -1.502  -8.797  1.00 25.53           O
HETATM14004  O   HOH B 407      -0.655  33.852   2.677  1.00 47.30           O
HETATM14005  O   HOH B 408      12.397  23.283   4.014  1.00 34.78           O
HETATM14006  O   HOH B 409      42.884  -6.443  -1.642  1.00 29.79           O
HETATM14007  O   HOH B 410      13.018  29.972  -4.529  1.00 36.99           O
HETATM14008  O   HOH B 411     -11.959  25.364  13.656  1.00 26.11           O
HETATM14009  O   HOH B 412      11.131  10.112  -8.298  1.00 31.35           O
HETATM14010  O   HOH B 413     -13.191  22.319  29.341  1.00 39.50           O
HETATM14011  O   HOH B 414      23.250  -4.215   2.899  1.00 33.00           O
HETATM14012  O   HOH B 415     -14.504  13.222  22.649  1.00 33.23           O
HETATM14013  O   HOH B 416     -14.957   6.525  15.837  1.00 31.19           O
HETATM14014  O   HOH B 417      26.371  25.797  -7.327  1.00 43.58           O
HETATM14015  O   HOH B 418      -7.958  28.008   9.124  1.00 35.80           O
HETATM14016  O   HOH B 419      30.688  -0.977 -17.346  1.00 46.59           O
HETATM14017  O   HOH B 420       2.226  -0.518  10.681  1.00 27.90           O
HETATM14018  O   HOH B 421      -7.850  27.530  16.436  1.00 39.21           O
HETATM14019  O   HOH B 422      13.866  28.788 -12.533  1.00 36.54           O
HETATM14020  O   HOH B 423      12.416  34.945  -1.142  1.00 32.43           O
HETATM14021  O   HOH B 424     -13.102  23.619  20.760  1.00 31.23           O
HETATM14022  O   HOH B 425     -13.533  10.632   9.377  1.00 27.96           O
HETATM14023  O   HOH B 426       1.255   8.083  26.114  1.00 42.98           O
HETATM14024  O   HOH B 427      -7.060   2.666  20.297  1.00 38.32           O
HETATM14025  O   HOH B 428      -6.686   4.963  22.361  1.00 35.20           O
HETATM14026  O   HOH B 429       3.556   5.355  22.754  1.00 41.91           O
HETATM14027  O   HOH B 430      -1.832   9.154  26.713  1.00 27.03           O
HETATM14028  O   HOH B 431      -3.567  27.581  18.881  1.00 39.12           O
```

```
HETATM14029  O    HOH B 432      6.769  30.596   8.787  1.00 42.32        O
HETATM14030  O    HOH B 433     16.674   6.370  -7.974  1.00 37.56        O
HETATM14031  O    HOH B 434     30.834   0.626   2.850  1.00 39.85        O
HETATM14032  O    HOH B 435      3.300  29.469  14.416  1.00 32.78        O
HETATM14033  O    HOH B 436     23.278  27.141  -0.067  1.00 36.51        O
HETATM14034  O    HOH B 437     -0.403   4.844  21.789  1.00 41.79        O
HETATM14035  O    HOH B 438     41.649  -8.028  -0.119  1.00 42.83        O
HETATM14036  O    HOH B 439    -12.293  13.622   4.863  1.00 39.81        O
HETATM14037  O    HOH B 440     39.590   5.370   3.545  1.00 43.58        O
HETATM14038  O    HOH B 441     45.581   2.556  -0.943  1.00 52.67        O
HETATM14039  O    HOH B 442     22.992   8.664   1.234  1.00 30.24        O
HETATM14040  O    HOH B 443     33.226   7.013 -13.693  1.00 28.49        O
HETATM14041  O    HOH B 444     21.116  31.590  -5.773  1.00 44.11        O
HETATM14042  O    HOH B 445     -9.664   4.515  11.618  1.00 29.38        O
HETATM14043  O    HOH B 446     -1.628  19.551  -4.027  1.00 32.27        O
HETATM14044  O    HOH B 447     -3.729   1.747   8.097  1.00 44.30        O
HETATM14045  O    HOH B 448      4.614  14.049  -3.885  1.00 35.19        O
HETATM14046  O    HOH B 449    -14.838   7.914   7.634  1.00 32.03        O
HETATM14047  O    HOH B 450     13.478  28.826   6.808  1.00 41.53        O
HETATM14048  O    HOH B 451     39.699  -6.088   0.783  1.00 41.02        O
HETATM14049  O    HOH B 452     -2.809   1.996  19.771  1.00 43.89        O
HETATM14050  O    HOH B 453     10.037  29.856   7.804  1.00 35.64        O
HETATM14051  O    HOH B 454      0.079   2.567   5.542  1.00 58.46        O
HETATM14052  O    HOH B 455     32.050   1.701   6.942  1.00 36.39        O
HETATM14053  O    HOH B 456     27.928  -4.876  -6.395  1.00 43.26        O
HETATM14054  O    HOH B 457     16.332  13.521   0.973  1.00 29.82        O
HETATM14055  O    HOH B 458     23.920  24.745  -9.990  1.00 40.00        O
HETATM14056  O    HOH B 459     31.835  -9.652  -9.323  1.00 44.55        O
HETATM14057  O    HOH B 460     24.126  23.505 -12.236  1.00 28.81        O
HETATM14058  O    HOH B 461     -8.211  19.288  33.467  1.00 39.86        O
HETATM14059  O    HOH B 462    -13.454   8.067  18.287  1.00 37.02        O
HETATM14060  O    HOH B 463     31.883  -5.888 -13.228  1.00 55.16        O
HETATM14061  O    HOH B 464      6.717  13.189   0.187  1.00 30.56        O
HETATM14062  O    HOH B 465      1.035   6.309   1.330  1.00 47.71        O
HETATM14063  O    HOH B 466     -8.142  16.183  27.859  1.00 36.18        O
HETATM14064  O    HOH B 467      6.157  25.766 -10.243  1.00 31.05        O
HETATM14065  O    HOH B 468     12.338  25.765   4.771  1.00 46.21        O
HETATM14066  O    HOH B 469     11.815  32.635  14.835  1.00 45.01        O
HETATM14067  O    HOH B 470      7.956  11.804   2.583  1.00 45.29        O
HETATM14068  O    HOH B 471     13.318  18.572   8.744  1.00 44.75        O
HETATM14069  O    HOH B 472     46.763   8.336 -11.156  1.00 34.98        O
HETATM14070  O    HOH B 473    -10.803  15.393   3.406  1.00 44.25        O
HETATM14071  O    HOH B 474     13.567   7.863  -2.434  1.00 38.06        O
HETATM14072  O    HOH B 475     22.570   7.473 -15.151  1.00 43.78        O
HETATM14073  O    HOH B 476    -12.486  23.797  17.432  1.00 37.13        O
HETATM14074  O    HOH B 477     15.212  22.331   9.491  1.00 38.41        O
HETATM14075  O    HOH B 478    -13.881   9.126  21.366  1.00 44.81        O
HETATM14076  O    HOH B 479    -11.347   9.897  28.056  1.00 50.07        O
HETATM14077  O    HOH B 480     19.085  13.352 -15.028  1.00 47.09        O
HETATM14078  O    HOH B 481     18.511  22.503   1.380  1.00 45.29        O
HETATM14079  O    HOH B 482     37.754  -9.186  -6.512  1.00 42.11        O
HETATM14080  O    HOH B 483    -12.866   4.891  16.751  1.00 39.41        O
HETATM14081  O    HOH B 484    -16.598   7.239  11.139  1.00 42.27        O
HETATM14082  O    HOH B 485     20.396  22.131 -14.479  1.00 43.03        O
HETATM14083  O    HOH B 486     -9.112  28.810  11.201  1.00 44.67        O
HETATM14084  O    HOH B 487     -1.406  13.648  -0.709  1.00 36.87        O
HETATM14085  O    HOH B 488     24.138  18.582 -17.358  1.00 32.41        O
HETATM14086  O    HOH B 489     20.292  14.264   0.448  1.00 34.48        O
HETATM14087  O    HOH B 490     -6.515   8.098  24.882  1.00 42.02        O
HETATM14088  O    HOH B 491    -17.451  10.191  10.799  1.00 39.01        O
HETATM14089  O    HOH B 492     11.764  14.621   5.897  1.00 42.64        O
HETATM14090  O    HOH B 493     18.322  32.602  -9.000  1.00 47.72        O
HETATM14091  O    HOH B 494    -11.935   3.080  12.809  1.00 38.39        O
HETATM14092  O    HOH B 495     13.833  26.722  -1.794  1.00 43.53        O
HETATM14093  O    HOH B 496     42.243  13.139 -15.062  1.00 41.45        O
```

```
HETATM14094   O   HOH B 497     9.097  20.316 -15.848  1.00 31.88           O
HETATM14095   O   HOH B 498    -9.816  32.987   8.982  1.00 40.99           O
HETATM14096   O   HOH B 499    16.717  25.959  13.020  1.00 43.94           O
HETATM14097   O   HOH B 500     8.820  25.995  10.618  1.00 40.92           O
HETATM14098   O   HOH B 501    16.932   3.906  -8.962  1.00 47.24           O
HETATM14099   O   HOH B 502   -10.220  26.290   7.130  1.00 40.01           O
HETATM14100   O   HOH B 503    21.984  -2.087   4.620  1.00 46.32           O
HETATM14101   O   HOH B 504    41.697  -0.050   5.430  1.00 45.75           O
HETATM14102   O   HOH B 505     2.700   5.821  27.135  1.00 49.34           O
HETATM14103   O   HOH B 506    18.092  26.572   0.570  1.00 50.79           O
HETATM14104   O   HOH B 507    23.062  21.393 -14.813  1.00 54.60           O
HETATM14105   O   HOH B 508   -17.275   8.244  17.659  1.00 37.14           O
HETATM14106   O   HOH B 509    -9.255  28.368   6.702  1.00 52.01           O
HETATM14107   O   HOH B 510    22.685  32.905  -3.736  1.00 43.72           O
HETATM14108   O   HOH B 511   -10.000   7.017  22.443  1.00 64.54           O
HETATM14109   O   HOH B 512     1.866  33.188   6.108  1.00 61.10           O
HETATM14110   O   HOH B 513    41.745   6.074  -5.312  1.00 50.79           O
HETATM14111   O   HOH B 514    23.704   5.370   4.473  1.00 41.54           O
HETATM14112   O   HOH B 515    10.777  36.756  13.230  1.00 40.44           O
HETATM14113   O   HOH B 516    11.059  32.693   2.960  1.00 62.97           O
HETATM14114   O   HOH B 517    45.696   6.419 -12.744  1.00 63.97           O
HETATM14115   O   HOH B 518     9.896  32.542   7.899  1.00 51.88           O
HETATM14116   O   HOH B 519     0.610  34.542  -1.011  1.00 40.87           O
HETATM14117   O   HOH B 520    23.176  -2.360  -7.617  1.00 53.75           O
HETATM14118   O   HOH B 521    -4.304  10.372  29.143  1.00 45.32           O
HETATM14119   O   HOH B 522    -0.403  31.699  -7.452  1.00 38.29           O
HETATM14120   O   HOH B 523    11.291  31.733 -12.569  1.00 49.60           O
HETATM14121   O   HOH B 524    16.431   1.976  -0.836  1.00 47.96           O
HETATM14122   O   HOH B 525    -3.037  -0.728   8.766  1.00 56.14           O
HETATM14123   O   HOH B 526    43.096   3.436   0.997  1.00 46.05           O
HETATM14124   O   HOH C 316    25.509  50.306  18.358  1.00 18.99           O
HETATM14125   O   HOH C 317    13.603  49.945 -47.385  1.00 10.29           O
HETATM14126   O   HOH C 318    17.573  55.217  -9.589  1.00 16.36           O
HETATM14127   O   HOH C 319    10.268  55.397 -10.019  1.00 18.61           O
HETATM14128   O   HOH C 320    24.096  52.361  12.381  1.00 14.02           O
HETATM14129   O   HOH C 321    12.126  49.861   9.720  1.00 14.94           O
HETATM14130   O   HOH C 322    29.661  41.901  -5.425  1.00 12.99           O
HETATM14131   O   HOH C 323    17.969  39.970  14.056  1.00 14.85           O
HETATM14132   O   HOH C 324    16.171  39.575  -4.785  1.00 20.23           O
HETATM14133   O   HOH C 325     4.624  48.186 -38.567  1.00 20.29           O
HETATM14134   O   HOH C 326    24.528  38.958  -1.778  1.00 20.02           O
HETATM14135   O   HOH C 327     9.062  55.467   7.175  1.00 16.01           O
HETATM14136   O   HOH C 328    23.941  50.227  14.955  1.00 14.19           O
HETATM14137   O   HOH C 329     7.086  49.885 -50.000  1.00 13.83           O
HETATM14138   O   HOH C 330    22.248  41.999  -7.108  1.00 16.81           O
HETATM14139   O   HOH C 331    -7.992  38.771 -39.391  1.00 17.82           O
HETATM14140   O   HOH C 332     7.819  47.835   8.598  1.00 18.10           O
HETATM14141   O   HOH C 333     2.985  37.385 -52.042  1.00 22.64           O
HETATM14142   O   HOH C 334    -9.088  50.068 -51.333  1.00 18.63           O
HETATM14143   O   HOH C 335    22.854  57.406  -9.355  1.00 21.55           O
HETATM14144   O   HOH C 336    21.814  36.216   3.883  1.00 19.22           O
HETATM14145   O   HOH C 337    13.538  43.371 -43.656  1.00 14.76           O
HETATM14146   O   HOH C 338     4.554  39.910 -52.386  1.00 18.82           O
HETATM14147   O   HOH C 339     7.481  48.421 -40.581  1.00 16.08           O
HETATM14148   O   HOH C 340     6.495  35.052 -43.012  1.00 17.90           O
HETATM14149   O   HOH C 341    31.713  40.111   1.422  1.00 18.32           O
HETATM14150   O   HOH C 342    19.100  38.522  -2.418  1.00 15.77           O
HETATM14151   O   HOH C 343    13.826  45.430  11.363  1.00 18.30           O
HETATM14152   O   HOH C 344    15.898  41.806  -8.561  1.00 26.22           O
HETATM14153   O   HOH C 345    15.719  55.804   9.259  1.00 14.96           O
HETATM14154   O   HOH C 346   -10.762  42.444 -22.020  1.00 28.39           O
HETATM14155   O   HOH C 347    12.015  43.181 -41.417  1.00 20.48           O
HETATM14156   O   HOH C 348    32.798  49.042   8.234  1.00 20.02           O
HETATM14157   O   HOH C 349    -5.371  51.630 -34.062  1.00 25.64           O
HETATM14158   O   HOH C 350    11.591  58.119  -4.766  1.00 30.52           O
```

```
HETATM14159  O   HOH C 351    -2.076  52.450 -28.047  1.00 16.12           O
HETATM14160  O   HOH C 352    18.722  60.805   8.502  1.00 27.23           O
HETATM14161  O   HOH C 353    12.788  36.038  -4.391  1.00 29.68           O
HETATM14162  O   HOH C 354    14.448  41.348 -45.795  1.00 14.65           O
HETATM14163  O   HOH C 355     5.075  60.304  -7.646  1.00 29.07           O
HETATM14164  O   HOH C 356    15.636  53.927  12.648  1.00 21.86           O
HETATM14165  O   HOH C 357     2.519  52.519 -18.950  1.00 19.06           O
HETATM14166  O   HOH C 358     1.978  49.367 -45.613  1.00 22.40           O
HETATM14167  O   HOH C 359    29.158  47.350  18.496  1.00 15.93           O
HETATM14168  O   HOH C 360     4.375  54.071 -10.179  1.00 23.91           O
HETATM14169  O   HOH C 361    21.445  59.656  -3.347  1.00 20.78           O
HETATM14170  O   HOH C 362    10.459  52.487 -49.554  1.00 25.49           O
HETATM14171  O   HOH C 363    -8.445  51.102 -27.238  1.00 21.38           O
HETATM14172  O   HOH C 364    14.035  58.513  -3.692  1.00 33.15           O
HETATM14173  O   HOH C 365     8.085  40.005 -34.528  1.00 15.73           O
HETATM14174  O   HOH C 366    22.393  54.444 -15.953  1.00 28.48           O
HETATM14175  O   HOH C 367    31.518  46.548   1.112  1.00 23.47           O
HETATM14176  O   HOH C 368    22.150  52.302  14.073  1.00 23.54           O
HETATM14177  O   HOH C 369     9.858  49.353 -53.056  1.00 23.64           O
HETATM14178  O   HOH C 370    14.284  42.004   8.198  1.00 26.07           O
HETATM14179  O   HOH C 371     4.063  47.895   5.287  1.00 25.16           O
HETATM14180  O   HOH C 372    11.383  52.413  10.205  1.00 23.01           O
HETATM14181  O   HOH C 373    22.026  40.308  14.889  1.00 27.17           O
HETATM14182  O   HOH C 374    -0.041  44.075 -11.639  1.00 29.15           O
HETATM14183  O   HOH C 375     4.477  50.257 -49.495  1.00 23.96           O
HETATM14184  O   HOH C 376     6.514  47.433  -9.915  1.00 21.66           O
HETATM14185  O   HOH C 377    19.243  52.248  13.845  1.00 24.17           O
HETATM14186  O   HOH C 378     1.985  26.651 -31.799  1.00 25.11           O
HETATM14187  O   HOH C 379    23.467  40.342  -6.007  1.00 27.41           O
HETATM14188  O   HOH C 380     8.133  62.631   2.195  1.00 50.31           O
HETATM14189  O   HOH C 381     0.351  47.316 -46.121  1.00 33.14           O
HETATM14190  O   HOH C 382    12.587  59.092   7.985  1.00 24.74           O
HETATM14191  O   HOH C 383     6.638  43.868 -29.700  1.00 20.63           O
HETATM14192  O   HOH C 384    -7.229  51.313 -35.919  1.00 28.68           O
HETATM14193  O   HOH C 385     0.039  56.356  -9.976  1.00 32.63           O
HETATM14194  O   HOH C 386    -8.279  48.922 -42.832  1.00 23.26           O
HETATM14195  O   HOH C 387    10.747  59.318 -10.398  1.00 33.62           O
HETATM14196  O   HOH C 388     5.195  45.875   1.832  1.00 34.97           O
HETATM14197  O   HOH C 389    -2.933  53.211 -35.046  1.00 31.54           O
HETATM14198  O   HOH C 390     7.013  53.872 -18.374  1.00 39.40           O
HETATM14199  O   HOH C 391   -11.915  52.301 -42.173  1.00 29.85           O
HETATM14200  O   HOH C 392     8.069  43.621   0.660  1.00 33.16           O
HETATM14201  O   HOH C 393    -0.534  37.095 -25.109  1.00 29.71           O
HETATM14202  O   HOH C 394    -1.501  35.111 -31.564  1.00 26.89           O
HETATM14203  O   HOH C 395     7.632  37.110 -27.103  1.00 30.78           O
HETATM14204  O   HOH C 396    19.566  41.104  -8.914  1.00 21.95           O
HETATM14205  O   HOH C 397     3.754  60.593  -0.076  1.00 42.99           O
HETATM14206  O   HOH C 398    28.380  37.702  12.361  1.00 31.43           O
HETATM14207  O   HOH C 399     4.714  51.253 -18.055  1.00 29.08           O
HETATM14208  O   HOH C 400    -9.709  50.677 -23.986  1.00 40.99           O
HETATM14209  O   HOH C 401    12.638  41.979   0.720  1.00 31.80           O
HETATM14210  O   HOH C 402    33.511  41.393   8.534  1.00 48.94           O
HETATM14211  O   HOH C 403     2.967  51.100 -47.835  1.00 32.64           O
HETATM14212  O   HOH C 404    -3.879  42.131 -53.606  1.00 41.14           O
HETATM14213  O   HOH C 405     1.810  59.160  -6.484  1.00 36.94           O
HETATM14214  O   HOH C 406    33.947  45.225  14.174  1.00 26.06           O
HETATM14215  O   HOH C 407    26.736  37.485  -2.134  1.00 30.27           O
HETATM14216  O   HOH C 408    28.864  34.879   5.011  1.00 26.47           O
HETATM14217  O   HOH C 409    29.758  55.627   3.881  1.00 23.66           O
HETATM14218  O   HOH C 410    35.294  53.732  12.872  1.00 32.81           O
HETATM14219  O   HOH C 411     6.883  47.564 -51.328  1.00 29.11           O
HETATM14220  O   HOH C 412    31.328  54.828   6.157  1.00 26.13           O
HETATM14221  O   HOH C 413    -8.703  54.340 -47.745  1.00 41.46           O
HETATM14222  O   HOH C 414    11.371  64.995   3.717  1.00 37.75           O
HETATM14223  O   HOH C 415     6.274  34.727 -39.595  1.00 33.12           O
```

```
HETATM14224  O    HOH C 416   -13.909  45.189 -24.192  1.00 27.52      O
HETATM14225  O    HOH C 417    12.055  40.684 -40.818  1.00 32.34      O
HETATM14226  O    HOH C 418   -11.297  53.368 -46.183  1.00 44.23      O
HETATM14227  O    HOH C 419     8.213  47.615 -12.495  1.00 25.76      O
HETATM14228  O    HOH C 420     8.527  57.280 -43.566  1.00 38.89      O
HETATM14229  O    HOH C 421     1.525  30.211 -36.720  1.00 33.31      O
HETATM14230  O    HOH C 422     4.937  60.408 -10.169  1.00 29.97      O
HETATM14231  O    HOH C 423    25.213  53.975  16.681  1.00 31.63      O
HETATM14232  O    HOH C 424     9.762  42.506 -22.300  1.00 26.90      O
HETATM14233  O    HOH C 425     5.871  32.288 -51.990  1.00 40.68      O
HETATM14234  O    HOH C 426    15.165  39.632   8.071  1.00 22.99      O
HETATM14235  O    HOH C 427    35.981  35.951  -2.533  1.00 26.50      O
HETATM14236  O    HOH C 428     3.172  33.439 -52.016  1.00 37.64      O
HETATM14237  O    HOH C 429    22.094  36.825  -2.667  1.00 24.77      O
HETATM14238  O    HOH C 430   -10.468  51.218 -17.852  1.00 36.12      O
HETATM14239  O    HOH C 431    15.610  35.732  -2.204  1.00 29.55      O
HETATM14240  O    HOH C 432    -2.810  57.675 -10.938  1.00 42.10      O
HETATM14241  O    HOH C 433    -8.524  30.446 -35.574  1.00 35.42      O
HETATM14242  O    HOH C 434     8.496  44.046   6.836  1.00 30.30      O
HETATM14243  O    HOH C 435    17.957  66.294   2.247  1.00 30.95      O
HETATM14244  O    HOH C 436    -6.932  38.070 -26.796  1.00 36.81      O
HETATM14245  O    HOH C 437     3.188  52.287 -39.942  1.00 36.02      O
HETATM14246  O    HOH C 438    -5.799  35.926 -25.948  1.00 35.78      O
HETATM14247  O    HOH C 439    -0.170  52.087   5.614  1.00 30.76      O
HETATM14248  O    HOH C 440    16.667  37.895  -6.731  1.00 29.44      O
HETATM14249  O    HOH C 441     5.835  45.729 -54.295  1.00 30.08      O
HETATM14250  O    HOH C 442     0.520  38.261 -18.751  1.00 44.94      O
HETATM14251  O    HOH C 443     9.333  37.583  -4.231  1.00 31.40      O
HETATM14252  O    HOH C 444    -1.343  48.283   0.260  1.00 28.83      O
HETATM14253  O    HOH C 445    15.079  38.711   3.435  1.00 42.48      O
HETATM14254  O    HOH C 446    11.418  43.121   8.133  1.00 33.15      O
HETATM14255  O    HOH C 447    -4.690  54.281 -17.913  1.00 20.83      O
HETATM14256  O    HOH C 448    -2.783  33.212 -29.800  1.00 33.10      O
HETATM14257  O    HOH C 449    30.565  37.231  -3.666  1.00 40.54      O
HETATM14258  O    HOH C 450     2.691  39.855 -17.338  1.00 44.89      O
HETATM14259  O    HOH C 451    17.651  50.574 -15.063  1.00 35.02      O
HETATM14260  O    HOH C 452     5.657  45.085 -11.242  1.00 32.64      O
HETATM14261  O    HOH C 453     9.602  54.341 -48.038  1.00 31.66      O
HETATM14262  O    HOH C 454     4.696  38.679 -18.621  1.00 46.01      O
HETATM14263  O    HOH C 455   -12.200  45.572 -13.730  1.00 35.13      O
HETATM14264  O    HOH C 456    11.632  56.456 -47.088  1.00 39.18      O
HETATM14265  O    HOH C 457    -6.654  50.338 -38.399  1.00 33.20      O
HETATM14266  O    HOH C 458    12.801  41.867  -8.570  1.00 35.47      O
HETATM14267  O    HOH C 459     8.517  62.365  -2.645  1.00 40.95      O
HETATM14268  O    HOH C 460    23.030  36.006   9.782  1.00 36.09      O
HETATM14269  O    HOH C 461    12.391  41.547 -10.881  1.00 41.15      O
HETATM14270  O    HOH C 462    14.063  49.425 -11.692  1.00 32.77      O
HETATM14271  O    HOH C 463    21.512  37.743  -5.428  1.00 38.76      O
HETATM14272  O    HOH C 464    15.784  48.452 -13.416  1.00 37.80      O
HETATM14273  O    HOH C 465    37.011  38.890   5.744  1.00 41.58      O
HETATM14274  O    HOH C 466     6.970  32.577 -38.289  1.00 36.44      O
HETATM14275  O    HOH C 467     9.196  36.363 -37.280  1.00 32.87      O
HETATM14276  O    HOH C 468    18.020  36.277   9.133  1.00 49.64      O
HETATM14277  O    HOH C 469     8.967  52.049 -16.270  1.00 34.46      O
HETATM14278  O    HOH C 470    30.458  43.835  12.239  1.00 37.15      O
HETATM14279  O    HOH C 471    10.521  39.375 -42.480  1.00 32.62      O
HETATM14280  O    HOH C 472   -11.188  38.922 -19.093  1.00 37.03      O
HETATM14281  O    HOH C 473    33.022  38.596  -5.070  1.00 33.81      O
HETATM14282  O    HOH C 474    10.598  36.627 -41.366  1.00 29.23      O
HETATM14283  O    HOH C 475    30.734  40.256  13.992  1.00 32.09      O
HETATM14284  O    HOH C 476    17.646  63.154   4.063  1.00 34.17      O
HETATM14285  O    HOH C 477    25.266  32.771   8.344  1.00 40.89      O
HETATM14286  O    HOH C 478    21.936  62.331  -2.041  1.00 35.94      O
HETATM14287  O    HOH C 479    -5.888  40.747 -16.915  1.00 39.76      O
HETATM14288  O    HOH C 480    11.279  37.608 -38.232  1.00 42.10      O
```

```
HETATM14289  O   HOH C 481    -6.356  47.316  -8.888  1.00 35.64           O
HETATM14290  O   HOH C 482    22.132  34.227   5.448  1.00 28.91           O
HETATM14291  O   HOH C 483    -6.788  53.389 -32.284  1.00 41.67           O
HETATM14292  O   HOH C 484    35.107  37.068   2.007  1.00 56.21           O
HETATM14293  O   HOH C 485     2.085  33.016 -30.152  1.00 45.12           O
HETATM14294  O   HOH C 486    20.834  35.197  -1.269  1.00 42.43           O
HETATM14295  O   HOH C 487     0.670  36.700 -27.594  1.00 38.27           O
HETATM14296  O   HOH C 488    24.551  59.495  12.662  1.00 55.02           O
HETATM14297  O   HOH C 489    -6.256  50.047  -5.564  1.00 35.58           O
HETATM14298  O   HOH C 490    -5.514  52.735 -21.463  1.00 37.75           O
HETATM14299  O   HOH C 491    -6.582  32.220 -44.659  1.00 34.65           O
HETATM14300  O   HOH C 492    -0.163  58.646  -8.648  1.00 35.74           O
HETATM14301  O   HOH C 493    12.174  55.858 -14.143  1.00 46.76           O
HETATM14302  O   HOH C 494     8.837  49.710 -12.175  1.00 34.01           O
HETATM14303  O   HOH C 495    -6.053  37.940 -20.301  1.00 46.88           O
HETATM14304  O   HOH C 496    -6.041  41.065 -52.238  1.00 46.84           O
HETATM14305  O   HOH C 497    25.801  37.243  -5.856  1.00 32.03           O
HETATM14306  O   HOH C 498    34.663  55.896  11.749  1.00 40.57           O
HETATM14307  O   HOH C 499     9.839  57.307  10.800  1.00 35.47           O
HETATM14308  O   HOH C 500    -4.052  44.329  -7.563  1.00 32.67           O
HETATM14309  O   HOH C 501   -16.360  45.969 -20.950  1.00 51.02           O
HETATM14310  O   HOH C 502     0.619  47.368 -48.799  1.00 38.45           O
HETATM14311  O   HOH C 503     5.920  54.381   8.660  1.00 49.30           O
HETATM14312  O   HOH C 504     2.465  57.327 -18.827  1.00 40.63           O
HETATM14313  O   HOH C 505    25.624  34.994  -5.175  1.00 40.25           O
HETATM14314  O   HOH C 506   -10.710  40.271  -9.416  1.00 41.87           O
HETATM14315  O   HOH C 507    27.080  39.135  -4.745  1.00 24.69           O
HETATM14316  O   HOH C 508    -3.516  52.605  -5.393  1.00 38.70           O
HETATM14317  O   HOH C 509     7.134  49.202  -9.999  1.00 35.60           O
HETATM14318  O   HOH C 510     8.894  57.841   7.827  1.00 36.26           O
HETATM14319  O   HOH C 511     9.878  33.734 -29.675  1.00 50.50           O
HETATM14320  O   HOH C 512    14.253  38.541   5.639  1.00 47.36           O
HETATM14321  O   HOH C 513    20.240  38.743  -8.783  1.00 41.84           O
HETATM14322  O   HOH C 514     7.338  33.888 -47.287  1.00 37.95           O
HETATM14323  O   HOH C 515   -10.139  45.752 -21.143  1.00 38.50           O
HETATM14324  O   HOH C 516    -7.421  52.925 -19.677  1.00 49.31           O
HETATM14325  O   HOH C 517   -11.548  39.033 -33.915  1.00 43.97           O
HETATM14326  O   HOH C 518    10.094  41.965   0.725  1.00 42.20           O
HETATM14327  O   HOH C 519     8.553  32.305 -43.736  1.00 39.91           O
HETATM14328  O   HOH C 520     2.144  29.738 -39.343  1.00 41.41           O
HETATM14329  O   HOH C 521    15.547  60.090   9.291  1.00 35.14           O
HETATM14330  O   HOH C 522    22.094  56.113  12.247  1.00 33.22           O
HETATM14331  O   HOH C 523     3.529  54.840   7.724  1.00 55.92           O
HETATM14332  O   HOH C 524    -0.770  29.881 -36.270  1.00 39.60           O
HETATM14333  O   HOH C 525   -18.950  41.256 -22.994  1.00 59.57           O
HETATM14334  O   HOH C 526    21.333  61.753   6.934  1.00 35.78           O
HETATM14335  O   HOH C 527     5.659  53.856 -44.105  1.00 37.88           O
HETATM14336  O   HOH C 528    11.343  48.613 -12.061  1.00 46.48           O
HETATM14337  O   HOH C 529    16.084  56.304  13.400  1.00 38.10           O
HETATM14338  O   HOH C 530    -4.215  56.357  -3.281  1.00 48.12           O
HETATM14339  O   HOH C 531    -2.366  55.562 -24.176  1.00 34.13           O
HETATM14340  O   HOH C 532    32.778  44.572  11.470  1.00 37.24           O
HETATM14341  O   HOH C 533   -11.976  45.006 -35.138  1.00 51.83           O
HETATM14342  O   HOH C 534    -4.270  55.073 -22.238  1.00 53.49           O
HETATM14343  O   HOH C 535    26.389  56.937  11.122  1.00 40.69           O
HETATM14344  O   HOH C 536    -7.387  43.899  -9.917  1.00 46.38           O
HETATM14345  O   HOH C 537    32.488  44.533  17.752  1.00 51.26           O
HETATM14346  O   HOH C 538     8.581  34.731  -5.434  1.00 48.03           O
HETATM14347  O   HOH C 539    -0.693  31.821 -29.091  1.00 46.06           O
HETATM14348  O   HOH C 540     6.900  39.484  -0.652  1.00 55.20           O
HETATM14349  O   HOH C 541    -8.020  38.730 -18.745  1.00 60.40           O
HETATM14350  O   HOH C 542     6.983  61.127   7.615  1.00 46.13           O
HETATM14351  O   HOH C 543    -6.998  33.872 -46.716  1.00 45.88           O
HETATM14352  O   HOH C 544     2.645  26.376 -34.780  1.00 38.13           O
HETATM14353  O   HOH C 545    19.908  67.024  -2.183  1.00 44.75           O
```

```
HETATM14354  O    HOH C 546      11.194  38.594   2.905  1.00 40.08         O
HETATM14355  O    HOH C 547      10.064  44.864 -18.324  1.00 33.14         O
HETATM14356  O    HOH C 548      -2.062  34.176 -25.098  1.00 52.17         O
HETATM14357  O    HOH C 549      -1.411  31.803 -46.913  1.00 47.46         O
HETATM14358  O    HOH C 550       0.629  45.461 -50.014  1.00 33.82         O
HETATM14359  O    HOH C 551      -3.723  38.614 -51.286  1.00 46.92         O
HETATM14360  O    HOH C 552      21.692  37.925  14.732  1.00 41.98         O
HETATM14361  O    HOH C 553       1.363  59.950  -0.892  1.00 46.01         O
HETATM14362  O    HOH C 554      -8.608  53.107 -13.697  1.00 31.90         O
HETATM14363  O    HOH C 555      24.814  56.164  13.028  1.00 48.88         O
HETATM14364  O    HOH C 556      -6.987  37.367 -48.265  1.00 35.25         O
HETATM14365  O    HOH C 557      -0.178  36.248 -29.986  1.00 43.62         O
HETATM14366  O    HOH C 558      -4.828  47.896 -52.436  1.00 53.01         O
HETATM14367  O    HOH C 559       5.857  32.471 -45.969  1.00 50.84         O
HETATM14368  O    HOH C 560      -2.718  55.545 -10.055  1.00 35.30         O
HETATM14369  O    HOH C 561     -16.436  44.723 -23.551  1.00 47.10         O
HETATM14370  O    HOH C 562      20.097  35.931   7.453  1.00 53.52         O
HETATM14371  O    HOH C 563     -11.082  35.613 -30.942  1.00 39.65         O
HETATM14372  O    HOH C 564      -2.490  32.684 -39.547  1.00 53.82         O
HETATM14373  O    HOH C 565     -13.485  39.386 -16.688  1.00 54.77         O
HETATM14374  O    HOH C 566      17.198  36.216  12.982  1.00 39.92         O
HETATM14375  O    HOH C 567      29.682  45.112  18.829  1.00 38.54         O
HETATM14376  O    HOH C 568       3.275  53.704 -46.055  1.00 41.77         O
HETATM14377  O    HOH C 569       2.735  30.826 -42.457  1.00 44.56         O
HETATM14378  O    HOH C 570     -17.173  42.417 -18.260  1.00 51.58         O
HETATM14379  O    HOH C 571      -5.749  54.063 -30.076  1.00 49.73         O
HETATM14380  O    HOH C 572       3.504  30.806 -29.135  1.00 34.22         O
HETATM14381  O    HOH C 573       1.870  56.767   8.396  1.00 44.14         O
HETATM14382  O    HOH C 574      15.940  39.097  -8.919  1.00 44.33         O
HETATM14383  O    HOH C 575      12.537  39.659 -44.970  1.00 41.74         O
HETATM14384  O    HOH D 316      30.842  56.434   1.665  1.00 18.29         O
HETATM14385  O    HOH D 317      37.984  42.902   3.639  1.00 23.71         O
HETATM14386  O    HOH D 318      39.163  59.857   4.996  1.00 52.52         O
HETATM14387  O    HOH D 319      42.670  44.686  10.703  1.00 35.02         O
HETATM14388  O    HOH D 320      32.012  59.626   4.307  1.00 29.20         O
HETATM14389  O    HOH D 321      42.413  55.681   2.166  1.00 28.04         O
HETATM14390  O    HOH D 322      36.177  43.284   8.182  1.00 29.04         O
HETATM14391  O    HOH D 323      41.683  40.329  13.100  1.00 42.00         O
HETATM14392  O    HOH D 324      41.757  40.981   4.532  1.00 36.34         O
HETATM14393  O    HOH D 325      39.575  58.292  11.820  1.00 44.16         O
HETATM14394  O    HOH D 326      39.516  49.944   5.965  1.00 34.09         O
HETATM14395  O    HOH D 327      33.827  54.733   9.435  1.00 36.76         O
HETATM14396  O    HOH D 328      42.275  55.786   8.795  1.00 41.68         O
HETATM14397  O    HOH D 329      38.494  51.328  11.538  1.00 41.36         O
HETATM14398  O    HOH D 330      45.103  43.541   1.835  1.00 36.04         O
HETATM14399  O    HOH D 331      38.770  51.905   7.117  1.00 44.04         O
HETATM14400  O    HOH D 332      37.922  49.150  12.641  1.00 39.62         O
HETATM14401  O    HOH D 333      19.244  46.077 -39.551  1.00 16.06         O
HETATM14402  O    HOH D 334      24.939  48.705 -28.276  1.00 16.20         O
HETATM14403  O    HOH D 335      26.678  46.072 -25.223  1.00 14.44         O
HETATM14404  O    HOH D 336      22.967  42.161 -26.109  1.00 17.33         O
HETATM14405  O    HOH D 337      43.626  49.743 -16.057  1.00 19.80         O
HETATM14406  O    HOH D 338      21.299  53.326 -34.735  1.00 19.56         O
HETATM14407  O    HOH D 339      35.020  46.484 -28.891  1.00 17.63         O
HETATM14408  O    HOH D 340      29.081  51.129 -37.621  1.00 15.34         O
HETATM14409  O    HOH D 341      28.448  50.028 -25.077  1.00 18.57         O
HETATM14410  O    HOH D 342      46.143  43.674  -6.361  1.00 25.55         O
HETATM14411  O    HOH D 343      42.494  39.292 -23.860  1.00 14.15         O
HETATM14412  O    HOH D 344      26.627  40.883 -13.094  1.00 24.94         O
HETATM14413  O    HOH D 345      35.880  38.114  -4.232  1.00 21.93         O
HETATM14414  O    HOH D 346      26.661  51.620 -23.200  1.00 18.93         O
HETATM14415  O    HOH D 347      26.922  41.238  -4.954  1.00 10.86         O
HETATM14416  O    HOH D 348       6.868  57.840 -41.508  1.00 26.37         O
HETATM14417  O    HOH D 349      31.833  40.346  -6.442  1.00 20.79         O
HETATM14418  O    HOH D 350      28.289  34.144 -28.478  1.00 22.16         O
```

```
HETATM14419  O   HOH D 351    34.229  58.720 -19.808  1.00 28.54       O
HETATM14420  O   HOH D 352    21.321  52.234 -37.450  1.00 14.62       O
HETATM14421  O   HOH D 353    26.752  38.449 -18.193  1.00 22.12       O
HETATM14422  O   HOH D 354    22.861  53.221 -39.360  1.00 11.80       O
HETATM14423  O   HOH D 355    14.308  44.804 -40.117  1.00 26.95       O
HETATM14424  O   HOH D 356    46.651  53.329 -10.807  1.00 25.84       O
HETATM14425  O   HOH D 357    32.902  32.522 -22.922  1.00 21.08       O
HETATM14426  O   HOH D 358    14.989  47.722 -43.836  1.00 14.88       O
HETATM14427  O   HOH D 359    43.826  41.213 -11.365  1.00 27.21       O
HETATM14428  O   HOH D 360    16.757  52.680 -40.266  1.00 19.35       O
HETATM14429  O   HOH D 361    29.647  30.775 -21.758  1.00 24.71       O
HETATM14430  O   HOH D 362    36.215  66.864  -2.614  1.00 17.03       O
HETATM14431  O   HOH D 363    17.929  49.958 -42.135  1.00 21.67       O
HETATM14432  O   HOH D 364    19.200  51.460 -40.514  1.00 19.48       O
HETATM14433  O   HOH D 365    24.690  58.296  -7.808  1.00 22.36       O
HETATM14434  O   HOH D 366    44.698  59.176  -5.105  1.00 30.86       O
HETATM14435  O   HOH D 367    34.432  56.707  -2.842  1.00 17.15       O
HETATM14436  O   HOH D 368    14.948  41.459 -35.887  1.00 25.58       O
HETATM14437  O   HOH D 369    24.016  57.404  -5.125  1.00 17.51       O
HETATM14438  O   HOH D 370    19.105  59.420 -39.812  1.00 16.42       O
HETATM14439  O   HOH D 371    16.348  43.339 -39.900  1.00 24.13       O
HETATM14440  O   HOH D 372    18.543  58.313 -28.924  1.00 27.36       O
HETATM14441  O   HOH D 373    24.272  55.805 -38.877  1.00 22.24       O
HETATM14442  O   HOH D 374    18.216  48.945 -25.286  1.00 30.14       O
HETATM14443  O   HOH D 375    42.373  64.176  -8.459  1.00 25.37       O
HETATM14444  O   HOH D 376    34.279  34.758 -32.204  1.00 21.92       O
HETATM14445  O   HOH D 377    34.426  51.119 -27.585  1.00 21.20       O
HETATM14446  O   HOH D 378    17.030  46.287 -42.957  1.00 20.29       O
HETATM14447  O   HOH D 379    26.232  65.592  -6.017  1.00 26.21       O
HETATM14448  O   HOH D 380    27.347  48.292 -26.769  1.00 22.89       O
HETATM14449  O   HOH D 381     6.286  64.167 -38.086  1.00 26.30       O
HETATM14450  O   HOH D 382    19.184  43.623 -39.465  1.00 19.10       O
HETATM14451  O   HOH D 383     9.082  37.736 -34.897  1.00 30.09       O
HETATM14452  O   HOH D 384    24.130  39.867 -35.222  1.00 23.76       O
HETATM14453  O   HOH D 385    29.248  53.964 -23.173  1.00 33.46       O
HETATM14454  O   HOH D 386     4.858  66.027 -39.662  1.00 25.48       O
HETATM14455  O   HOH D 387    14.784  54.500 -43.447  1.00 23.63       O
HETATM14456  O   HOH D 388    34.305  36.796 -14.411  1.00 28.40       O
HETATM14457  O   HOH D 389    29.720  53.899 -28.863  1.00 20.60       O
HETATM14458  O   HOH D 390    32.342  31.106 -20.717  1.00 20.94       O
HETATM14459  O   HOH D 391    46.194  45.444 -10.027  1.00 26.30       O
HETATM14460  O   HOH D 392    10.518  44.625 -27.647  1.00 26.65       O
HETATM14461  O   HOH D 393    24.599  59.812  -2.962  1.00 29.70       O
HETATM14462  O   HOH D 394    -2.299  55.261 -33.422  1.00 30.74       O
HETATM14463  O   HOH D 395    40.072  32.822  -7.471  1.00 21.69       O
HETATM14464  O   HOH D 396    12.371  61.520 -25.810  1.00 29.30       O
HETATM14465  O   HOH D 397    26.094  36.635 -20.099  1.00 30.67       O
HETATM14466  O   HOH D 398     2.631  56.467 -25.581  1.00 26.73       O
HETATM14467  O   HOH D 399    45.562  49.288 -20.201  1.00 28.12       O
HETATM14468  O   HOH D 400    13.388  60.739 -41.255  1.00 35.08       O
HETATM14469  O   HOH D 401    24.071  45.255 -21.474  1.00 45.35       O
HETATM14470  O   HOH D 402     9.981  54.084 -21.273  1.00 28.61       O
HETATM14471  O   HOH D 403    28.135  55.684 -27.819  1.00 27.85       O
HETATM14472  O   HOH D 404    15.823  46.604 -23.681  1.00 23.11       O
HETATM14473  O   HOH D 405    15.613  53.926 -24.752  1.00 33.49       O
HETATM14474  O   HOH D 406    24.105  57.855 -12.154  1.00 31.68       O
HETATM14475  O   HOH D 407    37.792  39.916  -2.986  1.00 38.72       O
HETATM14476  O   HOH D 408    23.843  43.459 -19.430  1.00 39.87       O
HETATM14477  O   HOH D 409    45.637  56.471   3.336  1.00 30.59       O
HETATM14478  O   HOH D 410    -3.790  56.785 -32.135  1.00 33.27       O
HETATM14479  O   HOH D 411    32.492  63.673 -15.165  1.00 35.06       O
HETATM14480  O   HOH D 412    31.656  38.518 -32.242  1.00 26.05       O
HETATM14481  O   HOH D 413    31.357  52.846 -26.725  1.00 34.67       O
HETATM14482  O   HOH D 414    12.702  59.173 -43.239  1.00 32.03       O
HETATM14483  O   HOH D 415    22.905  57.858 -39.884  1.00 22.42       O
```

```
HETATM14484  O   HOH D 416    -1.862  54.641 -26.464  1.00 29.36           O
HETATM14485  O   HOH D 417    37.179  49.177 -25.059  1.00 31.17           O
HETATM14486  O   HOH D 418    44.332  42.916 -16.688  1.00 29.88           O
HETATM14487  O   HOH D 419    -7.635  55.022 -40.303  1.00 45.36           O
HETATM14488  O   HOH D 420    31.164  58.032  -2.216  1.00 22.56           O
HETATM14489  O   HOH D 421    24.962  57.513 -37.092  1.00 34.36           O
HETATM14490  O   HOH D 422    46.725  44.045 -20.694  1.00 38.01           O
HETATM14491  O   HOH D 423    20.159  59.754 -31.122  1.00 27.39           O
HETATM14492  O   HOH D 424    38.211  53.770 -22.369  1.00 39.44           O
HETATM14493  O   HOH D 425    18.534  62.457 -28.839  1.00 32.23           O
HETATM14494  O   HOH D 426    32.275  55.060 -25.342  1.00 24.07           O
HETATM14495  O   HOH D 427    42.666  51.920   1.849  1.00 27.36           O
HETATM14496  O   HOH D 428    24.754  42.353 -24.485  1.00 24.87           O
HETATM14497  O   HOH D 429    45.989  45.700   1.145  1.00 29.13           O
HETATM14498  O   HOH D 430    18.852  63.829 -31.929  1.00 32.18           O
HETATM14499  O   HOH D 431     3.912  54.110 -42.005  1.00 22.14           O
HETATM14500  O   HOH D 432    46.447  60.257  -1.285  1.00 34.84           O
HETATM14501  O   HOH D 433    42.982  33.530  -5.195  1.00 31.68           O
HETATM14502  O   HOH D 434    20.627  57.426 -13.493  1.00 29.48           O
HETATM14503  O   HOH D 435     8.563  45.158 -28.999  1.00 27.33           O
HETATM14504  O   HOH D 436    46.157  48.544 -16.877  1.00 26.86           O
HETATM14505  O   HOH D 437    26.844  60.017  -9.341  1.00 34.37           O
HETATM14506  O   HOH D 438    43.283  35.359 -17.459  1.00 33.68           O
HETATM14507  O   HOH D 439    26.709  33.854 -26.387  1.00 28.44           O
HETATM14508  O   HOH D 440    19.791  43.056 -12.172  1.00 44.04           O
HETATM14509  O   HOH D 441    12.846  64.936 -38.440  1.00 44.82           O
HETATM14510  O   HOH D 442    28.621  37.035 -14.188  1.00 37.25           O
HETATM14511  O   HOH D 443    45.132  60.494   1.311  1.00 39.26           O
HETATM14512  O   HOH D 444    26.710  61.231 -11.719  1.00 33.20           O
HETATM14513  O   HOH D 445     7.367  66.264 -23.388  1.00 27.46           O
HETATM14514  O   HOH D 446    40.845  61.678 -19.805  1.00 40.77           O
HETATM14515  O   HOH D 447    47.112  58.864 -10.499  1.00 31.78           O
HETATM14516  O   HOH D 448    14.046  40.392 -32.987  1.00 27.34           O
HETATM14517  O   HOH D 449    36.107  48.958 -27.475  1.00 23.78           O
HETATM14518  O   HOH D 450    -1.928  68.474 -36.627  1.00 35.81           O
HETATM14519  O   HOH D 451    27.253  55.502 -21.099  1.00 40.90           O
HETATM14520  O   HOH D 452    33.168  35.635 -34.152  1.00 30.63           O
HETATM14521  O   HOH D 453    46.723  45.911 -17.363  1.00 43.14           O
HETATM14522  O   HOH D 454    44.041  48.710 -23.951  1.00 33.88           O
HETATM14523  O   HOH D 455    34.070  36.441  -7.086  1.00 24.22           O
HETATM14524  O   HOH D 456    14.736  38.201 -25.075  1.00 26.60           O
HETATM14525  O   HOH D 457    30.490  35.840 -10.461  1.00 35.93           O
HETATM14526  O   HOH D 458    41.776  32.407 -25.198  1.00 42.92           O
HETATM14527  O   HOH D 459    44.261  61.894  -0.904  1.00 37.56           O
HETATM14528  O   HOH D 460    42.276  41.593  -4.239  1.00 25.62           O
HETATM14529  O   HOH D 461    47.753  46.335  -4.385  1.00 36.11           O
HETATM14530  O   HOH D 462    14.155  52.364 -42.162  1.00 28.14           O
HETATM14531  O   HOH D 463    12.213  35.631 -27.433  1.00 47.32           O
HETATM14532  O   HOH D 464    17.943  34.175 -30.669  1.00 42.54           O
HETATM14533  O   HOH D 465     9.467  68.564 -29.371  1.00 32.54           O
HETATM14534  O   HOH D 466    33.305  54.066 -31.720  1.00 45.37           O
HETATM14535  O   HOH D 467    26.750  64.892  -3.470  1.00 35.97           O
HETATM14536  O   HOH D 468    46.354  58.119 -18.144  1.00 32.97           O
HETATM14537  O   HOH D 469    -5.379  67.777 -29.987  1.00 49.02           O
HETATM14538  O   HOH D 470    45.026  40.332  -9.023  1.00 28.99           O
HETATM14539  O   HOH D 471    26.056  36.272 -27.343  1.00 26.07           O
HETATM14540  O   HOH D 472    25.747  56.198 -31.180  1.00 34.12           O
HETATM14541  O   HOH D 473    24.622  51.307 -20.869  1.00 34.20           O
HETATM14542  O   HOH D 474    38.616  65.955  -9.928  1.00 29.41           O
HETATM14543  O   HOH D 475    28.475  58.431   4.188  1.00 32.34           O
HETATM14544  O   HOH D 476    46.837  49.236 -11.439  1.00 30.76           O
HETATM14545  O   HOH D 477     3.994  56.607 -20.974  1.00 43.48           O
HETATM14546  O   HOH D 478    21.739  37.316 -37.892  1.00 27.90           O
HETATM14547  O   HOH D 479    12.649  56.053 -24.541  1.00 37.16           O
HETATM14548  O   HOH D 480    21.924  49.916 -20.938  1.00 25.57           O
```

```
HETATM14549  O   HOH D 481    47.105  42.879 -10.107  1.00 35.65           O
HETATM14550  O   HOH D 482    -3.220  59.144 -43.201  1.00 48.11           O
HETATM14551  O   HOH D 483    43.429  36.308  -3.737  1.00 33.34           O
HETATM14552  O   HOH D 484    44.249  37.509 -18.663  1.00 42.59           O
HETATM14553  O   HOH D 485    44.486  49.761   0.965  1.00 32.41           O
HETATM14554  O   HOH D 486    28.256  57.276 -19.276  1.00 32.91           O
HETATM14555  O   HOH D 487    23.644  55.771 -26.971  1.00 31.22           O
HETATM14556  O   HOH D 488    21.602  47.431 -21.324  1.00 33.93           O
HETATM14557  O   HOH D 489    48.100  50.288  -8.079  1.00 33.86           O
HETATM14558  O   HOH D 490    -8.691  56.845 -38.510  1.00 46.54           O
HETATM14559  O   HOH D 491    10.424  65.913 -35.142  1.00 35.09           O
HETATM14560  O   HOH D 492    19.669  62.135 -33.819  1.00 41.76           O
HETATM14561  O   HOH D 493    42.265  35.169 -23.530  1.00 38.17           O
HETATM14562  O   HOH D 494    -2.460  49.176 -44.856  1.00 33.67           O
HETATM14563  O   HOH D 495    21.452  51.906 -16.317  1.00 38.44           O
HETATM14564  O   HOH D 496    36.580  52.860 -26.798  1.00 26.45           O
HETATM14565  O   HOH D 497    42.740  34.993 -14.845  1.00 33.08           O
HETATM14566  O   HOH D 498    29.324  62.059   0.042  1.00 34.55           O
HETATM14567  O   HOH D 499     3.335  63.527 -25.669  1.00 42.20           O
HETATM14568  O   HOH D 500    14.292  49.234 -24.017  1.00 36.73           O
HETATM14569  O   HOH D 501     3.477  66.204 -26.058  1.00 30.06           O
HETATM14570  O   HOH D 502    49.864  48.471  -3.909  1.00 36.53           O
HETATM14571  O   HOH D 503     1.744  61.756 -46.013  1.00 44.93           O
HETATM14572  O   HOH D 504    27.899  57.447 -31.968  1.00 32.54           O
HETATM14573  O   HOH D 505    16.076  57.860 -41.458  1.00 37.25           O
HETATM14574  O   HOH D 506    45.823  41.870 -12.775  1.00 43.18           O
HETATM14575  O   HOH D 507    21.214  33.308 -32.327  1.00 51.39           O
HETATM14576  O   HOH D 508    29.045  34.799  -6.770  1.00 41.74           O
HETATM14577  O   HOH D 509    25.702  64.906  -1.196  1.00 47.55           O
HETATM14578  O   HOH D 510    42.136  46.583 -24.559  1.00 41.33           O
HETATM14579  O   HOH D 511    45.923  53.141 -16.705  1.00 36.29           O
HETATM14580  O   HOH D 512    45.278  38.741 -23.815  1.00 35.13           O
HETATM14581  O   HOH D 513    28.062  64.191   1.785  1.00 36.99           O
HETATM14582  O   HOH D 514    18.195  63.536 -39.294  1.00 38.84           O
HETATM14583  O   HOH D 515    45.331  40.267 -17.585  1.00 34.16           O
HETATM14584  O   HOH D 516    27.051  37.451 -24.644  1.00 33.48           O
HETATM14585  O   HOH D 517    18.811  38.670 -23.968  1.00 39.20           O
HETATM14586  O   HOH D 518    46.440  45.506  -2.620  1.00 34.40           O
HETATM14587  O   HOH D 519    53.120  55.289  -0.794  1.00 51.02           O
HETATM14588  O   HOH D 520    26.120  34.135 -34.674  1.00 45.90           O
HETATM14589  O   HOH D 521    24.194  52.916 -23.216  1.00 55.98           O
HETATM14590  O   HOH D 522    23.444  39.026 -21.811  1.00 36.88           O
HETATM14591  O   HOH D 523    46.805  62.481  -4.121  1.00 40.92           O
HETATM14592  O   HOH D 524     9.016  34.700 -33.136  1.00 45.22           O
HETATM14593  O   HOH D 525    29.826  57.515 -35.241  1.00 36.78           O
HETATM14594  O   HOH D 526    16.803  55.300 -41.432  1.00 39.43           O
HETATM14595  O   HOH D 527    19.730  57.665 -17.108  1.00 42.84           O
HETATM14596  O   HOH D 528    22.591  52.778 -25.327  1.00 50.83           O
HETATM14597  O   HOH D 529    10.098  49.684 -19.938  1.00 47.43           O
HETATM14598  O   HOH D 530    23.359  38.151 -34.238  1.00 33.70           O
HETATM14599  O   HOH D 531     9.007  46.724 -28.423  1.00 38.77           O
HETATM14600  O   HOH D 532    10.975  41.584 -25.812  1.00 34.69           O
HETATM14601  O   HOH D 533    51.222  51.330  -7.241  1.00 39.85           O
HETATM14602  O   HOH D 534    41.083  32.740 -27.611  1.00 43.54           O
HETATM14603  O   HOH D 535    27.196  57.071   2.857  1.00 33.85           O
HETATM14604  O   HOH D 536    15.182  32.223 -31.318  1.00 43.78           O
HETATM14605  O   HOH D 537    39.675  64.121 -17.556  1.00 50.07           O
HETATM14606  O   HOH D 538    11.031  68.149 -22.612  1.00 37.01           O
HETATM14607  O   HOH D 539    45.907  45.222 -23.311  1.00 33.70           O
HETATM14608  O   HOH D 540    -6.292  57.042 -43.624  1.00 47.76           O
HETATM14609  O   HOH D 541    -9.869  66.249 -30.908  1.00 60.58           O
HETATM14610  O   HOH D 542     6.725  49.691 -38.492  1.00 38.42           O
HETATM14611  O   HOH D 543    46.612  38.275  -9.544  1.00 33.17           O
HETATM14612  O   HOH D 544    49.553  61.357  -1.411  1.00 33.89           O
HETATM14613  O   HOH D 545    44.354  42.922  -3.266  1.00 37.51           O
```

```
HETATM14614  O    HOH D 546    -4.160  50.294 -43.479  1.00 42.84         O
HETATM14615  O    HOH D 547    47.959  47.101 -10.885  1.00 37.08         O
HETATM14616  O    HOH D 548    -8.879  61.122 -33.203  1.00 47.68         O
HETATM14617  O    HOH D 549    41.121  66.681 -14.346  1.00 52.82         O
HETATM14618  O    HOH D 550    18.658  48.881 -22.597  1.00 36.33         O
HETATM14619  O    HOH D 551     8.760  63.580 -18.664  1.00 56.14         O
HETATM14620  O    HOH D 552    21.129  32.669 -34.917  1.00 60.65         O
HETATM14621  O    HOH D 553    49.412  49.071  -0.583  1.00 43.59         O
HETATM14622  O    HOH D 554    36.748  65.803 -14.101  1.00 47.56         O
HETATM14623  O    HOH D 555    -0.909  58.002 -40.644  1.00 39.61         O
HETATM14624  O    HOH D 556    25.096  63.713  -8.073  1.00 45.26         O
HETATM14625  O    HOH D 557    16.297  57.768 -23.595  1.00 45.41         O
HETATM14626  O    HOH D 558    25.636  55.956 -28.689  1.00 38.28         O
HETATM14627  O    HOH D 559    40.634  64.505   2.703  1.00 51.13         O
HETATM14628  O    HOH D 560    12.057  38.526 -34.925  1.00 46.01         O
HETATM14629  O    HOH D 561    -2.839  59.745 -40.646  1.00 40.81         O
HETATM14630  O    HOH D 562    25.093  60.858  -7.881  1.00 35.74         O
HETATM14631  O    HOH D 563    41.070  32.850  -9.951  1.00 52.61         O
HETATM14632  O    HOH D 564     7.782  69.114 -27.398  1.00 45.41         O
HETATM14633  O    HOH D 565    29.286  43.781 -38.004  1.00 38.67         O
HETATM14634  O    HOH D 566    -4.501  55.145 -45.458  1.00 53.88         O
HETATM14635  O    HOH D 567    46.627  52.755   3.770  1.00 41.48         O
HETATM14636  O    HOH D 568    24.780  62.378  -1.621  1.00 48.25         O
HETATM14637  O    HOH D 569    21.901  60.072 -38.647  1.00 35.95         O
HETATM14638  O    HOH D 570    16.148  51.095 -25.023  1.00 38.20         O
HETATM14639  O    HOH D 571    14.661  64.929 -33.761  1.00 36.77         O
HETATM14640  O    HOH D 572    31.616  57.497 -25.094  1.00 45.06         O
HETATM14641  O    HOH D 573    12.744  50.391 -21.639  1.00 41.81         O
HETATM14642  O    HOH D 574    35.434  69.008 -10.513  1.00 42.06         O
HETATM14643  O    HOH D 575     7.659  70.902 -36.629  1.00 44.86         O
HETATM14644  O    HOH D 576    47.979  54.182 -13.607  1.00 42.80         O
HETATM14645  O    HOH D 577    40.504  64.363 -21.154  1.00 54.82         O
HETATM14646  O    HOH D 578    41.209  66.286  -8.724  1.00 50.65         O
HETATM14647  O    HOH D 579    18.384  43.758 -23.065  1.00 42.59         O
HETATM14648  O    HOH D 580    24.640  39.129 -24.754  1.00 30.11         O
HETATM14649  O    HOH D 581    27.048  58.909 -37.137  1.00 43.61         O
HETATM14650  O    HOH D 582    22.387  38.604 -25.939  1.00 35.06         O
HETATM14651  O    HOH D 583    46.596  56.184 -16.566  1.00 48.15         O
HETATM14652  O    HOH D 584    -8.690  57.488 -35.722  1.00 57.16         O
HETATM14653  O    HOH D 585    12.569  66.889 -34.074  1.00 48.03         O
HETATM14654  O    HOH D 586    21.655  34.506 -37.129  1.00 52.87         O
HETATM14655  O    HOH D 587    36.333  64.976  -9.720  1.00 38.96         O
HETATM14656  O    HOH D 588    26.773  55.725 -24.578  1.00 58.96         O
HETATM14657  O    HOH D 589    31.379  35.655 -35.680  1.00 56.57         O
HETATM14658  O    HOH D 590     9.973  41.727 -34.430  1.00 57.66         O
HETATM14659  O    HOH D 591     4.767  56.423 -41.412  1.00 45.44         O
HETATM14660  O    HOH D 592    37.979  68.366 -10.317  1.00 39.87         O
HETATM14661  O    HOH D 593    24.181  36.438 -28.860  1.00 45.37         O
HETATM14662  O    HOH D 594    -5.287  57.886 -34.529  1.00 36.19         O
HETATM14663  O    HOH D 595    47.402  61.687  -6.569  1.00 37.05         O
HETATM14664  O    HOH E 215    47.265  44.338  67.943  1.00 44.18         O
HETATM14665  O    HOH E 216    22.496  37.863  70.557  1.00 40.31         O
HETATM14666  O    HOH E 217    21.468  51.193  75.669  1.00 10.22         O
HETATM14667  O    HOH E 218    10.525  51.770  16.007  1.00  8.27         O
HETATM14668  O    HOH E 219     2.345  47.063  20.173  1.00 15.33         O
HETATM14669  O    HOH E 220    27.750  50.135  68.854  1.00 10.80         O
HETATM14670  O    HOH E 221    22.595  50.740  18.637  1.00 12.81         O
HETATM14671  O    HOH E 222    17.025  38.310  30.535  1.00 15.68         O
HETATM14672  O    HOH E 223    13.650  41.197  35.468  1.00 17.44         O
HETATM14673  O    HOH E 224    21.618  42.602  74.243  1.00 14.95         O
HETATM14674  O    HOH E 225    19.825  39.499  32.834  1.00 19.56         O
HETATM14675  O    HOH E 226    21.719  47.536  14.778  1.00 12.17         O
HETATM14676  O    HOH E 227     6.416  40.437  33.834  1.00 13.76         O
HETATM14677  O    HOH E 228     7.185  34.523  22.294  1.00 12.36         O
HETATM14678  O    HOH E 229    13.343  46.907  13.474  1.00 10.70         O
```

```
HETATM14679  O   HOH E 230      4.647  38.649  27.241  1.00 21.05           O
HETATM14680  O   HOH E 231      9.394  49.420   9.873  1.00 12.87           O
HETATM14681  O   HOH E 232     14.495  35.831  24.318  1.00 21.85           O
HETATM14682  O   HOH E 233      3.373  52.982  22.305  1.00 19.58           O
HETATM14683  O   HOH E 234     22.161  44.632  72.298  1.00 17.32           O
HETATM14684  O   HOH E 235     31.757  46.902  46.957  1.00 18.74           O
HETATM14685  O   HOH E 236     29.964  52.317  46.363  1.00 19.08           O
HETATM14686  O   HOH E 237     -2.178  40.746  24.984  1.00 15.97           O
HETATM14687  O   HOH E 238     32.480  53.505  47.000  1.00 16.60           O
HETATM14688  O   HOH E 239     18.486  55.852  19.220  1.00 17.97           O
HETATM14689  O   HOH E 240     31.381  42.015  80.941  1.00 15.25           O
HETATM14690  O   HOH E 241     38.310  37.350  60.068  1.00 18.73           O
HETATM14691  O   HOH E 242     28.108  41.257  63.095  1.00 22.10           O
HETATM14692  O   HOH E 243     10.939  49.541  13.375  1.00 15.43           O
HETATM14693  O   HOH E 244     20.505  39.031  24.491  1.00 18.71           O
HETATM14694  O   HOH E 245     28.940  45.294  58.157  1.00 21.99           O
HETATM14695  O   HOH E 246     11.477  38.066  29.905  1.00 18.33           O
HETATM14696  O   HOH E 247     30.382  54.905  38.317  1.00 20.37           O
HETATM14697  O   HOH E 248     33.381  39.962  80.658  1.00 15.83           O
HETATM14698  O   HOH E 249     23.046  53.088  18.095  1.00 13.24           O
HETATM14699  O   HOH E 250      3.905  44.771  27.263  1.00 19.28           O
HETATM14700  O   HOH E 251     21.375  45.630  16.887  1.00 23.15           O
HETATM14701  O   HOH E 252     43.934  42.210  67.659  1.00 17.71           O
HETATM14702  O   HOH E 253     24.173  55.400  38.515  1.00 20.43           O
HETATM14703  O   HOH E 254     33.202  51.623  73.671  1.00 17.99           O
HETATM14704  O   HOH E 255     25.141  56.127  21.285  1.00 20.98           O
HETATM14705  O   HOH E 256     30.432  49.822  66.893  1.00 20.60           O
HETATM14706  O   HOH E 257     17.061  54.857  37.911  1.00 22.17           O
HETATM14707  O   HOH E 258     26.131  42.768  50.594  1.00 23.20           O
HETATM14708  O   HOH E 259     19.917  41.991  36.853  1.00 23.69           O
HETATM14709  O   HOH E 260      2.544  35.421  22.910  1.00 11.84           O
HETATM14710  O   HOH E 261      9.140  53.003  11.897  1.00 17.71           O
HETATM14711  O   HOH E 262     27.265  48.596  19.830  1.00 17.47           O
HETATM14712  O   HOH E 263     43.288  40.895  55.071  1.00 23.12           O
HETATM14713  O   HOH E 264     46.844  44.774  50.770  1.00 36.69           O
HETATM14714  O   HOH E 265     12.566  51.818  14.200  1.00 22.02           O
HETATM14715  O   HOH E 266     36.859  54.431  55.840  1.00 17.23           O
HETATM14716  O   HOH E 267     12.732  53.547  12.236  1.00 16.45           O
HETATM14717  O   HOH E 268     30.129  36.820  71.773  1.00 19.11           O
HETATM14718  O   HOH E 269      5.983  46.150   9.692  1.00 18.20           O
HETATM14719  O   HOH E 270     15.414  51.957  14.624  1.00 19.92           O
HETATM14720  O   HOH E 271     46.047  48.639  49.750  1.00 23.81           O
HETATM14721  O   HOH E 272      4.686  53.768  24.386  1.00 20.90           O
HETATM14722  O   HOH E 273     25.656  40.675  71.143  1.00 22.75           O
HETATM14723  O   HOH E 274     43.286  54.020  55.552  1.00 22.37           O
HETATM14724  O   HOH E 275     21.764  54.449  16.184  1.00 24.21           O
HETATM14725  O   HOH E 276     40.197  54.164  62.236  1.00 20.79           O
HETATM14726  O   HOH E 277     25.612  58.842  71.757  1.00 28.50           O
HETATM14727  O   HOH E 278     23.756  44.098  69.909  1.00 20.13           O
HETATM14728  O   HOH E 279     22.725  58.344  33.202  1.00 28.72           O
HETATM14729  O   HOH E 280     20.392  35.800  30.453  1.00 20.79           O
HETATM14730  O   HOH E 281     39.868  55.230  49.498  1.00 26.17           O
HETATM14731  O   HOH E 282     13.889  56.603  41.871  1.00 26.75           O
HETATM14732  O   HOH E 283     11.451  56.427  37.736  1.00 21.96           O
HETATM14733  O   HOH E 284     36.030  56.495  54.418  1.00 21.85           O
HETATM14734  O   HOH E 285      1.968  38.911  20.739  1.00 19.21           O
HETATM14735  O   HOH E 286     43.580  40.631  76.481  1.00 30.86           O
HETATM14736  O   HOH E 287     31.849  60.616  34.654  1.00 26.21           O
HETATM14737  O   HOH E 288     23.734  36.307  32.275  1.00 35.87           O
HETATM14738  O   HOH E 289     36.800  32.417  64.609  1.00 32.37           O
HETATM14739  O   HOH E 290     36.426  39.202  53.230  1.00 35.92           O
HETATM14740  O   HOH E 291     37.782  55.698  62.985  1.00 21.88           O
HETATM14741  O   HOH E 292     47.535  50.445  49.941  1.00 32.41           O
HETATM14742  O   HOH E 293     45.416  40.002  56.531  1.00 26.20           O
HETATM14743  O   HOH E 294     27.859  51.874  78.274  1.00 19.29           O
```

```
HETATM14744  O   HOH E 295   20.914  39.926  20.192  1.00 22.72        O
HETATM14745  O   HOH E 296   42.465  46.501  38.149  1.00 36.53        O
HETATM14746  O   HOH E 297   12.478  58.982  31.581  1.00 25.62        O
HETATM14747  O   HOH E 298    1.362  43.569  13.961  1.00 26.32        O
HETATM14748  O   HOH E 299   20.528  48.995  40.165  1.00 26.73        O
HETATM14749  O   HOH E 300   41.787  43.346  44.919  1.00 26.09        O
HETATM14750  O   HOH E 301   36.594  57.472  51.756  1.00 32.52        O
HETATM14751  O   HOH E 302   38.332  54.152  66.302  1.00 27.46        O
HETATM14752  O   HOH E 303   28.738  60.995  35.889  1.00 28.31        O
HETATM14753  O   HOH E 304    9.470  53.969  14.768  1.00 28.65        O
HETATM14754  O   HOH E 305   37.142  34.744  75.335  1.00 38.91        O
HETATM14755  O   HOH E 306    7.229  56.512  17.755  1.00 34.94        O
HETATM14756  O   HOH E 307   27.643  37.585  65.941  1.00 25.59        O
HETATM14757  O   HOH E 308   30.167  46.140  39.376  1.00 27.87        O
HETATM14758  O   HOH E 309    8.089  36.408  15.634  1.00 23.57        O
HETATM14759  O   HOH E 310   19.918  58.428  32.202  1.00 40.48        O
HETATM14760  O   HOH E 311   35.164  49.731  74.369  1.00 31.66        O
HETATM14761  O   HOH E 312   15.341  42.941  38.748  1.00 38.29        O
HETATM14762  O   HOH E 313   23.230  42.444  27.656  1.00 25.04        O
HETATM14763  O   HOH E 314   -2.669  38.463  26.034  1.00 30.48        O
HETATM14764  O   HOH E 315   21.269  42.105  20.091  1.00 20.45        O
HETATM14765  O   HOH E 316   25.791  38.216  69.761  1.00 32.44        O
HETATM14766  O   HOH E 317   27.737  35.197  65.371  1.00 21.07        O
HETATM14767  O   HOH E 318   15.857  35.630  20.248  1.00 22.36        O
HETATM14768  O   HOH E 319   22.583  42.307  36.450  1.00 26.62        O
HETATM14769  O   HOH E 320   29.792  36.813  68.096  1.00 33.98        O
HETATM14770  O   HOH E 321   41.964  38.693  54.213  1.00 41.13        O
HETATM14771  O   HOH E 322   14.053  36.168  30.761  1.00 26.20        O
HETATM14772  O   HOH E 323   25.858  45.024  46.513  1.00 27.87        O
HETATM14773  O   HOH E 324    7.353  33.185  18.595  1.00 38.86        O
HETATM14774  O   HOH E 325   28.455  49.001  38.194  1.00 22.82        O
HETATM14775  O   HOH E 326   21.999  56.987  16.543  1.00 35.68        O
HETATM14776  O   HOH E 327   42.225  49.598  37.138  1.00 31.89        O
HETATM14777  O   HOH E 328   41.916  54.128  64.135  1.00 34.45        O
HETATM14778  O   HOH E 329   13.173  40.715  12.698  1.00 33.28        O
HETATM14779  O   HOH E 330   29.665  51.197  20.167  1.00 25.40        O
HETATM14780  O   HOH E 331   -0.156  36.424  25.952  1.00 32.14        O
HETATM14781  O   HOH E 332   45.433  54.711  45.757  1.00 37.96        O
HETATM14782  O   HOH E 333   19.697  37.617  34.708  1.00 31.27        O
HETATM14783  O   HOH E 334   46.779  43.247  65.764  1.00 27.54        O
HETATM14784  O   HOH E 335   33.134  60.401  31.094  1.00 42.57        O
HETATM14785  O   HOH E 336   13.219  35.436  18.208  1.00 23.92        O
HETATM14786  O   HOH E 337   28.860  38.054  56.097  1.00 37.15        O
HETATM14787  O   HOH E 338   12.380  53.538  44.255  1.00 22.87        O
HETATM14788  O   HOH E 339   19.060  53.911  15.921  1.00 25.60        O
HETATM14789  O   HOH E 340   30.484  34.419  80.302  1.00 28.32        O
HETATM14790  O   HOH E 341   23.862  41.726  69.492  1.00 25.90        O
HETATM14791  O   HOH E 342   24.067  43.510  20.259  1.00 23.64        O
HETATM14792  O   HOH E 343   25.187  49.670  47.008  1.00 27.42        O
HETATM14793  O   HOH E 344   43.261  55.928  42.257  1.00 40.44        O
HETATM14794  O   HOH E 345    7.205  35.282  29.384  1.00 26.72        O
HETATM14795  O   HOH E 346   38.750  46.502  36.222  1.00 32.61        O
HETATM14796  O   HOH E 347   30.214  61.326  28.382  1.00 37.32        O
HETATM14797  O   HOH E 348   17.811  36.674  18.373  1.00 33.99        O
HETATM14798  O   HOH E 349   22.785  40.303  26.734  1.00 32.54        O
HETATM14799  O   HOH E 350   34.542  57.771  38.041  1.00 28.35        O
HETATM14800  O   HOH E 351   16.799  50.049  43.151  1.00 35.17        O
HETATM14801  O   HOH E 352   44.419  35.699  71.467  1.00 34.55        O
HETATM14802  O   HOH E 353   42.213  52.083  76.755  1.00 50.04        O
HETATM14803  O   HOH E 354   14.240  56.691  15.544  1.00 32.18        O
HETATM14804  O   HOH E 355   33.235  44.992  46.822  1.00 37.51        O
HETATM14805  O   HOH E 356   30.836  47.602  82.951  1.00 28.55        O
HETATM14806  O   HOH E 357   11.690  60.892  21.417  1.00 32.85        O
HETATM14807  O   HOH E 358   36.420  38.257  58.560  1.00 40.31        O
HETATM14808  O   HOH E 359    0.893  36.840  19.244  1.00 40.16        O
```

```
HETATM14809  O  HOH E 360   46.988  51.703  58.680  1.00 37.44        O
HETATM14810  O  HOH E 361   28.680  61.127  38.628  1.00 26.14        O
HETATM14811  O  HOH E 362   33.517  36.108  81.513  1.00 38.88        O
HETATM14812  O  HOH E 363   17.360  50.118  14.193  1.00  9.42        O
HETATM14813  O  HOH E 364   29.197  36.003  76.190  1.00 23.00        O
HETATM14814  O  HOH E 365   38.144  44.257  38.110  1.00 31.95        O
HETATM14815  O  HOH E 366   27.561  47.964  40.712  1.00 38.13        O
HETATM14816  O  HOH E 367   42.333  55.267  47.927  1.00 41.12        O
HETATM14817  O  HOH E 368   44.279  46.099  79.591  1.00 24.45        O
HETATM14818  O  HOH E 369   15.301  60.390  19.907  1.00 21.79        O
HETATM14819  O  HOH E 370   47.707  38.684  59.107  1.00 33.29        O
HETATM14820  O  HOH E 371   -0.876  51.344  15.475  1.00 34.98        O
HETATM14821  O  HOH E 372   11.006  68.305  28.660  1.00 32.95        O
HETATM14822  O  HOH E 373   24.895  55.989  75.977  1.00 35.44        O
HETATM14823  O  HOH E 374   13.178  66.014  30.558  1.00 46.72        O
HETATM14824  O  HOH E 375   34.019  60.225  37.111  1.00 40.44        O
HETATM14825  O  HOH E 376   36.219  57.418  23.718  1.00 34.45        O
HETATM14826  O  HOH E 377   26.484  56.106  17.603  1.00 44.67        O
HETATM14827  O  HOH E 378   48.076  42.066  48.323  1.00 41.46        O
HETATM14828  O  HOH E 379   36.310  45.774  39.945  1.00 31.92        O
HETATM14829  O  HOH E 380   14.198  33.693  22.798  1.00 29.18        O
HETATM14830  O  HOH E 381   12.270  61.177  30.662  1.00 30.39        O
HETATM14831  O  HOH E 382   23.316  59.145  38.547  1.00 47.15        O
HETATM14832  O  HOH E 383   14.090  57.470  38.562  1.00 44.32        O
HETATM14833  O  HOH E 384   21.294  59.563  20.842  1.00 40.71        O
HETATM14834  O  HOH E 385   39.130  56.111  45.922  1.00 33.69        O
HETATM14835  O  HOH E 386   39.776  35.026  67.675  1.00 31.61        O
HETATM14836  O  HOH E 387   27.225  39.006  53.531  1.00 30.97        O
HETATM14837  O  HOH E 388   32.306  58.808  48.576  1.00 33.66        O
HETATM14838  O  HOH E 389   39.194  31.823  62.199  1.00 42.43        O
HETATM14839  O  HOH E 390   30.465  52.733  77.793  1.00 29.05        O
HETATM14840  O  HOH E 391   38.729  38.538  47.787  1.00 42.35        O
HETATM14841  O  HOH E 392   45.588  41.806  47.263  1.00 56.23        O
HETATM14842  O  HOH E 393   37.467  58.004  45.547  1.00 38.41        O
HETATM14843  O  HOH E 394   39.746  36.783  56.054  1.00 30.90        O
HETATM14844  O  HOH E 395   42.117  42.064  42.659  1.00 36.03        O
HETATM14845  O  HOH E 396   34.947  53.863  22.557  1.00 39.30        O
HETATM14846  O  HOH E 397   15.795  66.317  32.478  1.00 41.44        O
HETATM14847  O  HOH E 398   25.127  58.506  20.562  1.00 37.19        O
HETATM14848  O  HOH E 399   45.230  48.139  70.518  1.00 55.24        O
HETATM14849  O  HOH E 400   21.290  38.880  22.431  1.00 38.47        O
HETATM14850  O  HOH E 401   41.271  55.757  60.502  1.00 31.18        O
HETATM14851  O  HOH E 402   28.456  57.233  15.216  1.00 37.80        O
HETATM14852  O  HOH E 403    9.682  61.087  26.962  1.00 37.72        O
HETATM14853  O  HOH E 404   34.986  40.726  47.152  1.00 43.41        O
HETATM14854  O  HOH E 405   33.619  41.028  45.316  1.00 34.75        O
HETATM14855  O  HOH E 406    9.503  62.582  24.217  1.00 46.92        O
HETATM14856  O  HOH E 407   28.072  56.457  72.462  1.00 36.51        O
HETATM14857  O  HOH E 408   21.925  35.407  28.358  1.00 37.00        O
HETATM14858  O  HOH E 409    2.388  54.301  18.656  1.00 47.92        O
HETATM14859  O  HOH E 410   47.740  49.095  73.324  1.00 33.83        O
HETATM14860  O  HOH E 411    0.881  34.472  30.476  1.00 36.63        O
HETATM14861  O  HOH E 412   15.086  34.952  16.453  1.00 38.62        O
HETATM14862  O  HOH E 413   38.290  59.631  43.305  1.00 40.77        O
HETATM14863  O  HOH E 414   25.280  50.006  40.116  1.00 34.78        O
HETATM14864  O  HOH E 415   -0.136  49.273   8.712  1.00 44.02        O
HETATM14865  O  HOH E 416   14.513  37.147  33.409  1.00 39.32        O
HETATM14866  O  HOH E 417   31.311  48.388  22.885  1.00 36.36        O
HETATM14867  O  HOH E 418   34.503  50.440  25.442  1.00 34.84        O
HETATM14868  O  HOH E 419   15.585  37.498  36.874  1.00 37.82        O
HETATM14869  O  HOH E 420   16.034  40.708  36.810  1.00 31.74        O
HETATM14870  O  HOH E 421   32.903  43.631  40.747  1.00 43.94        O
HETATM14871  O  HOH E 422    5.583  55.779  12.928  1.00 32.94        O
HETATM14872  O  HOH E 423   28.469  35.179  59.405  1.00 53.37        O
HETATM14873  O  HOH E 424   41.185  53.008  66.420  1.00 36.49        O
```

```
HETATM14874  O   HOH E 425    31.211  48.886  79.116  1.00 35.34       O
HETATM14875  O   HOH E 426    17.451  55.204  47.876  1.00 40.66       O
HETATM14876  O   HOH E 427    25.736  52.740  44.333  1.00 51.45       O
HETATM14877  O   HOH E 428    33.596  58.706  51.072  1.00 38.26       O
HETATM14878  O   HOH E 429    19.090  48.498  42.046  1.00 46.39       O
HETATM14879  O   HOH E 430    39.001  32.908  67.598  1.00 31.67       O
HETATM14880  O   HOH E 431    33.672  32.459  71.261  1.00 48.40       O
HETATM14881  O   HOH E 432    37.362  58.743  21.267  1.00 69.41       O
HETATM14882  O   HOH E 433    37.626  51.364  72.640  1.00 51.01       O
HETATM14883  O   HOH E 434    -3.498  45.909  12.785  1.00 39.08       O
HETATM14884  O   HOH E 435    19.845  57.890  18.365  1.00 32.15       O
HETATM14885  O   HOH E 436    30.781  61.271  20.986  1.00 33.69       O
HETATM14886  O   HOH E 437    14.532  38.819  13.249  1.00 36.56       O
HETATM14887  O   HOH E 438    11.604  55.640  15.641  1.00 55.34       O
HETATM14888  O   HOH E 439    28.549  49.411  79.584  1.00 34.34       O
HETATM14889  O   HOH E 440    37.822  54.908  33.593  1.00 32.74       O
HETATM14890  O   HOH E 441    26.359  44.378  22.200  1.00 34.22       O
HETATM14891  O   HOH E 442    28.970  58.239  18.842  1.00 54.18       O
HETATM14892  O   HOH E 443    29.136  34.092  67.017  1.00 52.63       O
HETATM14893  O   HOH E 444    17.776  46.289  39.715  1.00 46.11       O
HETATM14894  O   HOH E 445    22.559  57.641  75.297  1.00 47.67       O
HETATM14895  O   HOH E 446    12.763  67.958  26.654  1.00 42.65       O
HETATM14896  O   HOH E 447    36.895  39.326  46.470  1.00 46.70       O
HETATM14897  O   HOH E 448     6.901  44.929  29.491  1.00 38.81       O
HETATM14898  O   HOH E 449    44.520  54.182  52.172  1.00 50.33       O
HETATM14899  O   HOH E 450     4.350  45.832   7.611  1.00 42.52       O
HETATM14900  O   HOH E 451    46.636  35.717  64.015  1.00 41.04       O
HETATM14901  O   HOH E 452    45.532  33.470  64.844  1.00 42.23       O
HETATM14902  O   HOH E 453    41.429  58.455  52.587  1.00 57.43       O
HETATM14903  O   HOH E 454     6.423  43.875   9.271  1.00 46.66       O
HETATM14904  O   HOH E 455    23.556  40.616  73.439  1.00 35.53       O
HETATM14905  O   HOH E 456    -0.857  48.340  11.154  1.00 37.81       O
HETATM14906  O   HOH E 457     8.578  55.163  11.254  1.00 30.81       O
HETATM14907  O   HOH E 458    13.664  42.625  11.310  1.00 33.45       O
HETATM14908  O   HOH E 459    33.845  45.139  82.215  1.00 45.84       O
HETATM14909  O   HOH E 460    39.654  57.021  58.049  1.00 45.24       O
HETATM14910  O   HOH E 461    30.965  65.427  29.498  1.00 46.97       O
HETATM14911  O   HOH E 462    50.342  48.735  55.201  1.00 40.83       O
HETATM14912  O   HOH E 463    36.178  50.203  27.416  1.00 36.33       O
HETATM14913  O   HOH E 464    46.727  46.771  68.442  1.00 37.14       O
HETATM14914  O   HOH E 465    47.568  39.806  53.938  1.00 57.45       O
HETATM14915  O   HOH E 466    50.060  44.244  45.095  1.00 48.97       O
HETATM14916  O   HOH E 467    18.394  56.246  15.252  1.00 37.14       O
HETATM14917  O   HOH E 468     8.916  33.830  16.756  1.00 46.79       O
HETATM14918  O   HOH E 469    14.363  63.217  24.175  1.00 41.24       O
HETATM14919  O   HOH E 470    30.568  40.965  46.593  1.00 51.01       O
HETATM14920  O   HOH E 471    34.320  48.138  77.542  1.00 43.12       O
HETATM14921  O   HOH E 472    -1.559  44.040  13.022  1.00 53.32       O
HETATM14922  O   HOH E 473    27.320  58.739  45.934  1.00 45.83       O
HETATM14923  O   HOH E 474    49.637  48.851  52.460  1.00 40.29       O
HETATM14924  O   HOH E 475    42.549  56.053  56.985  1.00 45.44       O
HETATM14925  O   HOH E 476    36.907  43.102  41.706  1.00 52.05       O
HETATM14926  O   HOH E 477    24.793  35.133  29.459  1.00 43.23       O
HETATM14927  O   HOH E 478    21.956  65.241  25.138  1.00 42.33       O
HETATM14928  O   HOH E 479    14.963  66.420  26.598  1.00 67.83       O
HETATM14929  O   HOH E 480    39.877  42.677  41.472  1.00 53.66       O
HETATM14930  O   HOH E 481    47.423  48.594  41.898  1.00 37.59       O
HETATM14931  O   HOH E 482    40.671  35.561  70.832  1.00 59.14       O
HETATM14932  O   HOH E 483    31.020  34.856  60.467  1.00 48.74       O
HETATM14933  O   HOH E 484    25.362  40.001  22.608  1.00 46.26       O
HETATM14934  O   HOH E 485    11.189  66.248  25.561  1.00 45.59       O
HETATM14935  O   HOH F 316   -11.187  40.912  48.532  1.00 34.50       O
HETATM14936  O   HOH F 317   -15.813  43.967  14.174  1.00 48.11       O
HETATM14937  O   HOH F 318     9.055  39.852  33.340  1.00 11.90       O
HETATM14938  O   HOH F 319    13.468  52.117  65.587  1.00 11.44       O
```

```
HETATM14939  O   HOH F 320     10.235  56.369  33.529  1.00 14.99          O
HETATM14940  O   HOH F 321     -8.573  46.728  44.429  1.00 13.13          O
HETATM14941  O   HOH F 322     13.395  53.359  62.885  1.00 12.64          O
HETATM14942  O   HOH F 323     -0.110  54.542  31.311  1.00 13.98          O
HETATM14943  O   HOH F 324     10.370  47.773  56.603  1.00 13.11          O
HETATM14944  O   HOH F 325    -11.931  50.027  39.388  1.00 19.67          O
HETATM14945  O   HOH F 326      4.628  37.509  61.064  1.00 20.44          O
HETATM14946  O   HOH F 327      6.766  48.812  53.442  1.00 14.35          O
HETATM14947  O   HOH F 328      8.003  46.981  55.149  1.00 17.64          O
HETATM14948  O   HOH F 329     11.838  52.996  67.361  1.00 11.34          O
HETATM14949  O   HOH F 330     20.154  48.707  72.194  1.00 16.27          O
HETATM14950  O   HOH F 331     15.595  51.839  68.704  1.00 15.73          O
HETATM14951  O   HOH F 332      6.030  50.294  65.791  1.00 14.04          O
HETATM14952  O   HOH F 333      8.825  44.921  53.671  1.00 14.30          O
HETATM14953  O   HOH F 334      3.507  54.968  26.989  1.00 17.49          O
HETATM14954  O   HOH F 335     21.094  45.700  68.692  1.00 25.55          O
HETATM14955  O   HOH F 336     16.155  46.288  67.780  1.00 12.00          O
HETATM14956  O   HOH F 337     17.877  53.359  68.303  1.00 15.46          O
HETATM14957  O   HOH F 338     -0.090  56.712  48.101  1.00 18.99          O
HETATM14958  O   HOH F 339     18.435  46.922  71.405  1.00 23.22          O
HETATM14959  O   HOH F 340      0.485  44.808  57.246  1.00 17.33          O
HETATM14960  O   HOH F 341     26.720  46.831  57.053  1.00 22.43          O
HETATM14961  O   HOH F 342     -6.245  38.538  32.758  1.00 21.54          O
HETATM14962  O   HOH F 343     15.804  58.454  56.820  1.00 22.33          O
HETATM14963  O   HOH F 344     21.441  61.963  53.489  1.00 15.77          O
HETATM14964  O   HOH F 345     13.137  41.560  54.892  1.00 18.06          O
HETATM14965  O   HOH F 346     -0.147  40.854  20.547  1.00 25.92          O
HETATM14966  O   HOH F 347     21.862  40.797  61.746  1.00 25.33          O
HETATM14967  O   HOH F 348     -6.234  36.645  52.484  1.00 22.23          O
HETATM14968  O   HOH F 349      9.452  39.522  41.481  1.00 23.30          O
HETATM14969  O   HOH F 350      8.342  33.162  56.895  1.00 26.88          O
HETATM14970  O   HOH F 351    -12.783  55.740  38.812  1.00 20.39          O
HETATM14971  O   HOH F 352    -10.588  42.511  38.118  1.00 23.13          O
HETATM14972  O   HOH F 353     19.519  46.912  52.244  1.00 22.86          O
HETATM14973  O   HOH F 354     19.663  55.407  71.353  1.00 20.02          O
HETATM14974  O   HOH F 355     -7.720  49.015  26.553  1.00 23.36          O
HETATM14975  O   HOH F 356     21.645  56.629  52.587  1.00 20.71          O
HETATM14976  O   HOH F 357     31.032  54.146  68.213  1.00 26.41          O
HETATM14977  O   HOH F 358      4.127  38.643  34.809  1.00 19.33          O
HETATM14978  O   HOH F 359     30.514  55.927  69.940  1.00 14.59          O
HETATM14979  O   HOH F 360      9.286  57.119  36.184  1.00 29.94          O
HETATM14980  O   HOH F 361     16.344  43.988  67.934  1.00 14.12          O
HETATM14981  O   HOH F 362     28.056  59.160  69.397  1.00 25.56          O
HETATM14982  O   HOH F 363     20.743  53.245  70.482  1.00 16.86          O
HETATM14983  O   HOH F 364     18.939  53.719  52.816  1.00 25.32          O
HETATM14984  O   HOH F 365     -8.587  61.392  37.362  1.00 27.19          O
HETATM14985  O   HOH F 366      9.330  36.163  53.285  1.00 21.37          O
HETATM14986  O   HOH F 367      7.904  50.526  51.821  1.00 20.99          O
HETATM14987  O   HOH F 368     17.201  50.044  70.286  1.00 18.64          O
HETATM14988  O   HOH F 369     27.580  65.662  65.849  1.00 21.67          O
HETATM14989  O   HOH F 370     14.992  59.670  67.463  1.00 17.19          O
HETATM14990  O   HOH F 371     -2.950  65.349  31.793  1.00 24.26          O
HETATM14991  O   HOH F 372      4.840  52.468  57.198  1.00 14.96          O
HETATM14992  O   HOH F 373     21.225  60.129  71.028  1.00 30.00          O
HETATM14993  O   HOH F 374    -13.188  58.601  35.149  1.00 25.46          O
HETATM14994  O   HOH F 375      8.699  55.481  59.041  1.00 29.44          O
HETATM14995  O   HOH F 376      0.518  35.754  32.772  1.00 27.94          O
HETATM14996  O   HOH F 377     -0.645  46.975  55.851  1.00 23.51          O
HETATM14997  O   HOH F 378    -10.435  55.988  33.277  1.00 22.68          O
HETATM14998  O   HOH F 379    -10.501  45.688  48.459  1.00 29.82          O
HETATM14999  O   HOH F 380     36.862  57.677  61.329  1.00 22.63          O
HETATM15000  O   HOH F 381      2.982  56.377  30.621  1.00 30.47          O
HETATM15001  O   HOH F 382    -10.702  45.147  45.438  1.00 24.89          O
HETATM15002  O   HOH F 383    -11.001  49.627  45.414  1.00 31.25          O
HETATM15003  O   HOH F 384     31.539  57.660  53.520  1.00 16.96          O
```

```
HETATM15004  O   HOH F 385    6.359  54.495  55.666  1.00 28.57        O
HETATM15005  O   HOH F 386  -13.268  60.877  35.802  1.00 27.60        O
HETATM15006  O   HOH F 387    4.220  51.161  54.748  1.00 45.13        O
HETATM15007  O   HOH F 388   20.744  61.484  69.212  1.00 22.41        O
HETATM15008  O   HOH F 389    0.387  55.335  21.448  1.00 24.20        O
HETATM15009  O   HOH F 390   -6.610  43.810  53.055  1.00 26.46        O
HETATM15010  O   HOH F 391    7.090  59.421  40.548  1.00 35.84        O
HETATM15011  O   HOH F 392   23.976  61.026  70.505  1.00 26.75        O
HETATM15012  O   HOH F 393   -6.276  61.871  46.064  1.00 29.87        O
HETATM15013  O   HOH F 394   -4.718  46.667  22.043  1.00 27.23        O
HETATM15014  O   HOH F 395   18.079  58.423  69.237  1.00 32.92        O
HETATM15015  O   HOH F 396   26.420  66.843  50.789  1.00 27.73        O
HETATM15016  O   HOH F 397   27.151  38.633  63.436  1.00 24.14        O
HETATM15017  O   HOH F 398    9.048  58.824  31.276  1.00 36.04        O
HETATM15018  O   HOH F 399    1.412  61.848  43.754  1.00 23.60        O
HETATM15019  O   HOH F 400   35.168  60.283  68.767  1.00 27.49        O
HETATM15020  O   HOH F 401    6.379  56.270  59.626  1.00 28.68        O
HETATM15021  O   HOH F 402   35.129  70.821  64.265  1.00 39.42        O
HETATM15022  O   HOH F 403    0.614  31.920  58.314  1.00 19.73        O
HETATM15023  O   HOH F 404   10.023  36.168  64.575  1.00 24.94        O
HETATM15024  O   HOH F 405   20.952  42.353  64.138  1.00 24.19        O
HETATM15025  O   HOH F 406   -6.264  63.201  37.696  1.00 33.00        O
HETATM15026  O   HOH F 407   -6.774  41.961  17.692  1.00 28.89        O
HETATM15027  O   HOH F 408    8.901  54.941  56.685  1.00 29.27        O
HETATM15028  O   HOH F 409   11.090  41.093  53.006  1.00 20.21        O
HETATM15029  O   HOH F 410   10.808  51.171  47.084  1.00 36.00        O
HETATM15030  O   HOH F 411   22.449  67.151  62.806  1.00 24.25        O
HETATM15031  O   HOH F 412    7.211  38.597  44.612  1.00 20.92        O
HETATM15032  O   HOH F 413   -1.821  37.716  31.471  1.00 42.46        O
HETATM15033  O   HOH F 414   -2.113  47.239  53.395  1.00 27.26        O
HETATM15034  O   HOH F 415   12.030  39.592  63.736  1.00 28.46        O
HETATM15035  O   HOH F 416    9.732  37.106  47.091  1.00 23.94        O
HETATM15036  O   HOH F 417   20.637  41.929  66.783  1.00 26.21        O
HETATM15037  O   HOH F 418   18.682  65.265  60.882  1.00 27.93        O
HETATM15038  O   HOH F 419   20.467  65.691  65.985  1.00 29.32        O
HETATM15039  O   HOH F 420   26.880  68.952  51.293  1.00 26.07        O
HETATM15040  O   HOH F 421   10.421  55.517  66.902  1.00 29.68        O
HETATM15041  O   HOH F 422   -3.748  48.950  17.304  1.00 31.60        O
HETATM15042  O   HOH F 423   17.586  55.751  69.393  1.00 23.10        O
HETATM15043  O   HOH F 424   10.687  41.501  68.178  1.00 29.47        O
HETATM15044  O   HOH F 425   -2.564  64.319  29.555  1.00 26.98        O
HETATM15045  O   HOH F 426    9.142  37.368  33.276  1.00 28.73        O
HETATM15046  O   HOH F 427    3.229  36.684  33.573  1.00 28.51        O
HETATM15047  O   HOH F 428   11.686  44.435  50.419  1.00 40.61        O
HETATM15048  O   HOH F 429    6.730  55.785  25.415  1.00 21.89        O
HETATM15049  O   HOH F 430   17.159  48.599  53.463  1.00 35.13        O
HETATM15050  O   HOH F 431  -12.638  43.323  33.305  1.00 33.15        O
HETATM15051  O   HOH F 432   11.307  57.706  67.824  1.00 37.94        O
HETATM15052  O   HOH F 433    5.635  52.359  51.337  1.00 30.48        O
HETATM15053  O   HOH F 434   22.014  67.505  50.310  1.00 38.55        O
HETATM15054  O   HOH F 435   19.415  43.730  68.479  1.00 33.57        O
HETATM15055  O   HOH F 436   28.024  51.072  66.571  1.00 38.68        O
HETATM15056  O   HOH F 437   -2.995  46.734  15.756  1.00 25.93        O
HETATM15057  O   HOH F 438   29.514  61.546  46.818  1.00 30.53        O
HETATM15058  O   HOH F 439    2.409  53.256  53.646  1.00 35.70        O
HETATM15059  O   HOH F 440   13.783  37.967  54.840  1.00 30.24        O
HETATM15060  O   HOH F 441   38.366  59.217  59.876  1.00 28.63        O
HETATM15061  O   HOH F 442   -7.807  52.117  19.231  1.00 30.06        O
HETATM15062  O   HOH F 443    1.511  44.548  19.188  1.00 20.39        O
HETATM15063  O   HOH F 444    7.498  58.879  46.716  1.00 44.32        O
HETATM15064  O   HOH F 445   11.419  38.257  53.173  1.00 30.62        O
HETATM15065  O   HOH F 446  -12.377  56.927  29.586  1.00 25.95        O
HETATM15066  O   HOH F 447   21.132  38.491  53.988  1.00 35.97        O
HETATM15067  O   HOH F 448   24.541  69.270  56.866  1.00 33.68        O
HETATM15068  O   HOH F 449   24.874  45.102  56.139  1.00 26.88        O
```

```
HETATM15069  O    HOH F 450      16.130  42.245  41.114  1.00 32.77           O
HETATM15070  O    HOH F 451      23.531  60.089  49.797  1.00 25.43           O
HETATM15071  O    HOH F 452      29.957  64.712  53.011  1.00 36.02           O
HETATM15072  O    HOH F 453      12.415  37.424  50.555  1.00 39.89           O
HETATM15073  O    HOH F 454      14.699  64.840  61.276  1.00 35.80           O
HETATM15074  O    HOH F 455      18.702  51.131  52.982  1.00 25.38           O
HETATM15075  O    HOH F 456      15.322  62.095  56.542  1.00 28.67           O
HETATM15076  O    HOH F 457      -7.681  38.134  40.257  1.00 43.27           O
HETATM15077  O    HOH F 458      39.217  69.894  57.279  1.00 27.06           O
HETATM15078  O    HOH F 459     -13.180  46.688  36.376  1.00 32.69           O
HETATM15079  O    HOH F 460      12.062  52.387  53.351  1.00 48.40           O
HETATM15080  O    HOH F 461      24.565  54.337  49.384  1.00 28.72           O
HETATM15081  O    HOH F 462      28.401  67.739  67.012  1.00 31.13           O
HETATM15082  O    HOH F 463       0.544  52.418  18.819  1.00 24.99           O
HETATM15083  O    HOH F 464       4.600  56.660  23.129  1.00 41.95           O
HETATM15084  O    HOH F 465     -13.980  58.134  39.828  1.00 31.46           O
HETATM15085  O    HOH F 466      20.826  49.387  52.224  1.00 27.44           O
HETATM15086  O    HOH F 467      33.437  63.095  51.557  1.00 34.07           O
HETATM15087  O    HOH F 468      12.949  40.107  68.829  1.00 37.43           O
HETATM15088  O    HOH F 469      12.778  51.110  44.686  1.00 36.41           O
HETATM15089  O    HOH F 470      -7.972  63.665  41.984  1.00 38.54           O
HETATM15090  O    HOH F 471      16.773  48.927  50.400  1.00 30.45           O
HETATM15091  O    HOH F 472     -15.405  41.754  16.626  1.00 42.81           O
HETATM15092  O    HOH F 473     -11.858  36.710  19.767  1.00 40.02           O
HETATM15093  O    HOH F 474      15.550  43.247  43.508  1.00 36.54           O
HETATM15094  O    HOH F 475      21.889  59.429  51.622  1.00 35.19           O
HETATM15095  O    HOH F 476      18.206  34.411  59.177  1.00 41.00           O
HETATM15096  O    HOH F 477       5.245  36.576  36.064  1.00 45.94           O
HETATM15097  O    HOH F 478      13.534  44.185  44.872  1.00 42.45           O
HETATM15098  O    HOH F 479       2.211  57.824  27.981  1.00 30.89           O
HETATM15099  O    HOH F 480      -9.236  61.171  49.477  1.00 42.88           O
HETATM15100  O    HOH F 481      19.667  64.473  50.047  1.00 37.03           O
HETATM15101  O    HOH F 482      10.183  57.088  40.355  1.00 32.86           O
HETATM15102  O    HOH F 483      13.350  36.022  56.197  1.00 27.74           O
HETATM15103  O    HOH F 484     -11.030  41.955  31.236  1.00 34.28           O
HETATM15104  O    HOH F 485       2.695  60.305  28.968  1.00 42.39           O
HETATM15105  O    HOH F 486      17.514  65.272  63.852  1.00 37.67           O
HETATM15106  O    HOH F 487      23.291  40.615  67.165  1.00 35.11           O
HETATM15107  O    HOH F 488       9.352  57.663  48.363  1.00 35.46           O
HETATM15108  O    HOH F 489     -14.798  51.995  39.324  1.00 42.73           O
HETATM15109  O    HOH F 490      -7.949  52.870  26.513  1.00 32.19           O
HETATM15110  O    HOH F 491      10.476  34.331  51.407  1.00 39.89           O
HETATM15111  O    HOH F 492      -2.223  29.496  54.740  1.00 36.39           O
HETATM15112  O    HOH F 493      -0.883  37.660  22.857  1.00 34.53           O
HETATM15113  O    HOH F 494      -2.709  53.095  51.302  1.00 43.06           O
HETATM15114  O    HOH F 495      -6.091  32.602  52.930  1.00 41.31           O
HETATM15115  O    HOH F 496     -11.325  39.756  38.376  1.00 39.03           O
HETATM15116  O    HOH F 497     -10.261  42.613  15.186  1.00 41.72           O
HETATM15117  O    HOH F 498      -6.653  59.072  27.398  1.00 37.16           O
HETATM15118  O    HOH F 499     -12.594  45.688  43.990  1.00 35.53           O
HETATM15119  O    HOH F 500      -8.161  39.927  45.403  1.00 34.97           O
HETATM15120  O    HOH F 501       6.862  62.980  30.110  1.00 43.31           O
HETATM15121  O    HOH F 502      -9.220  59.971  28.073  1.00 45.05           O
HETATM15122  O    HOH F 503      25.685  72.461  63.859  1.00 37.37           O
HETATM15123  O    HOH F 504      -8.558  44.061  17.415  1.00 41.02           O
HETATM15124  O    HOH F 505      10.245  51.829  51.374  1.00 35.17           O
HETATM15125  O    HOH F 506      -9.072  40.121  26.578  1.00 39.54           O
HETATM15126  O    HOH F 507     -12.463  43.751  39.348  1.00 27.02           O
HETATM15127  O    HOH F 508      18.001  56.798  51.435  1.00 41.00           O
HETATM15128  O    HOH F 509      10.326  60.028  64.844  1.00 42.49           O
HETATM15129  O    HOH F 510      29.284  64.930  45.351  1.00 34.67           O
HETATM15130  O    HOH F 511     -10.841  53.359  25.188  1.00 34.79           O
HETATM15131  O    HOH F 512      -5.774  38.738  23.719  1.00 38.54           O
HETATM15132  O    HOH F 513      10.732  36.630  44.348  1.00 38.27           O
HETATM15133  O    HOH F 514      -8.187  45.853  21.259  1.00 38.02           O
```

| | | | | | | | | | | |
|---|---|---|---|---|---|---|---|---|---|---|
| HETATM15134 | O | HOH | F | 515 | 42.284 | 58.153 | 67.747 | 1.00 | 65.92 | O |
| HETATM15135 | O | HOH | F | 516 | 7.181 | 58.150 | 25.623 | 1.00 | 32.34 | O |
| HETATM15136 | O | HOH | F | 517 | 0.631 | 59.004 | 49.460 | 1.00 | 51.79 | O |
| HETATM15137 | O | HOH | F | 518 | -16.451 | 47.381 | 34.963 | 1.00 | 49.49 | O |
| HETATM15138 | O | HOH | F | 519 | 43.148 | 59.268 | 65.461 | 1.00 | 45.36 | O |
| HETATM15139 | O | HOH | F | 520 | 42.486 | 61.446 | 66.594 | 1.00 | 37.48 | O |
| HETATM15140 | O | HOH | F | 521 | 10.522 | 55.022 | 54.860 | 1.00 | 35.68 | O |
| HETATM15141 | O | HOH | F | 522 | 14.806 | 33.862 | 55.502 | 1.00 | 37.20 | O |
| HETATM15142 | O | HOH | F | 523 | 43.392 | 62.760 | 63.483 | 1.00 | 48.56 | O |
| HETATM15143 | O | HOH | F | 524 | -9.419 | 45.454 | 51.843 | 1.00 | 29.69 | O |
| HETATM15144 | O | HOH | F | 525 | -1.556 | 38.122 | 18.129 | 1.00 | 55.21 | O |
| HETATM15145 | O | HOH | F | 526 | 37.162 | 62.488 | 68.220 | 1.00 | 46.14 | O |
| HETATM15146 | O | HOH | F | 527 | 3.418 | 63.422 | 48.560 | 1.00 | 49.21 | O |
| HETATM15147 | O | HOH | F | 528 | 11.232 | 32.883 | 58.863 | 1.00 | 39.65 | O |
| HETATM15148 | O | HOH | F | 529 | -12.747 | 43.639 | 10.187 | 1.00 | 53.70 | O |
| HETATM15149 | O | HOH | F | 530 | 38.018 | 72.461 | 54.071 | 1.00 | 43.44 | O |
| HETATM15150 | O | HOH | F | 531 | -6.237 | 51.380 | 50.319 | 1.00 | 39.40 | O |
| HETATM15151 | O | HOH | F | 532 | 17.213 | 54.161 | 50.785 | 1.00 | 46.49 | O |
| HETATM15152 | O | HOH | F | 533 | 39.737 | 64.363 | 61.900 | 1.00 | 49.98 | O |
| HETATM15153 | O | HOH | F | 534 | 9.056 | 63.110 | 29.144 | 1.00 | 51.10 | O |
| HETATM15154 | O | HOH | F | 535 | -15.822 | 39.249 | 15.270 | 1.00 | 43.96 | O |
| HETATM15155 | O | HOH | F | 536 | -15.530 | 40.958 | 13.001 | 1.00 | 58.44 | O |
| HETATM15156 | O | HOH | F | 537 | 11.941 | 38.403 | 47.989 | 1.00 | 39.82 | O |
| HETATM15157 | O | HOH | F | 538 | 14.012 | 59.485 | 58.283 | 1.00 | 50.02 | O |
| HETATM15158 | O | HOH | F | 539 | -0.218 | 57.429 | 22.774 | 1.00 | 50.89 | O |
| HETATM15159 | O | HOH | F | 540 | 27.197 | 61.303 | 46.232 | 1.00 | 44.61 | O |
| HETATM15160 | O | HOH | F | 541 | 37.036 | 75.321 | 57.478 | 1.00 | 39.76 | O |
| HETATM15161 | O | HOH | F | 542 | -9.559 | 37.498 | 43.219 | 1.00 | 42.68 | O |
| HETATM15162 | O | HOH | F | 543 | -6.107 | 59.937 | 46.925 | 1.00 | 43.68 | O |
| HETATM15163 | O | HOH | F | 544 | 14.101 | 46.745 | 49.864 | 1.00 | 31.89 | O |
| HETATM15164 | O | HOH | F | 545 | 7.287 | 53.989 | 49.227 | 1.00 | 47.36 | O |
| HETATM15165 | O | HOH | F | 546 | -8.169 | 54.420 | 18.570 | 1.00 | 40.14 | O |
| HETATM15166 | O | HOH | F | 547 | -13.340 | 45.708 | 41.515 | 1.00 | 37.37 | O |
| HETATM15167 | O | HOH | F | 548 | 27.804 | 36.479 | 62.166 | 1.00 | 41.28 | O |
| HETATM15168 | O | HOH | F | 549 | 12.231 | 40.703 | 47.988 | 1.00 | 35.35 | O |
| HETATM15169 | O | HOH | F | 550 | -7.558 | 34.042 | 47.655 | 1.00 | 40.75 | O |
| HETATM15170 | O | HOH | F | 551 | 0.056 | 32.936 | 60.851 | 1.00 | 34.29 | O |
| HETATM15171 | O | HOH | F | 552 | 4.152 | 57.471 | 49.998 | 1.00 | 47.99 | O |
| HETATM15172 | O | HOH | F | 553 | 9.602 | 32.794 | 61.852 | 1.00 | 35.39 | O |
| HETATM15173 | O | HOH | F | 554 | 7.205 | 54.435 | 53.121 | 1.00 | 53.06 | O |
| HETATM15174 | O | HOH | F | 555 | 2.557 | 34.530 | 35.635 | 1.00 | 36.50 | O |
| HETATM15175 | O | HOH | F | 556 | -0.695 | 53.499 | 17.111 | 1.00 | 38.80 | O |
| HETATM15176 | O | HOH | F | 557 | 13.921 | 49.001 | 48.980 | 1.00 | 38.71 | O |
| HETATM15177 | O | HOH | F | 558 | 29.891 | 58.013 | 48.703 | 1.00 | 42.87 | O |
| HETATM15178 | O | HOH | F | 559 | 12.234 | 42.824 | 47.343 | 1.00 | 37.04 | O |
| HETATM15179 | O | HOH | F | 560 | -4.008 | 49.237 | 53.760 | 1.00 | 44.28 | O |
| HETATM15180 | O | HOH | F | 561 | -11.749 | 46.118 | 39.708 | 1.00 | 36.51 | O |
| HETATM15181 | O | HOH | F | 562 | 11.228 | 55.097 | 48.363 | 1.00 | 44.21 | O |
| HETATM15182 | O | HOH | F | 563 | -9.227 | 46.951 | 27.455 | 1.00 | 37.60 | O |
| HETATM15183 | O | HOH | F | 564 | 31.931 | 53.375 | 75.437 | 1.00 | 40.06 | O |
| HETATM15184 | O | HOH | F | 565 | -4.129 | 62.095 | 48.202 | 1.00 | 48.35 | O |
| HETATM15185 | O | HOH | F | 566 | 39.754 | 73.044 | 56.688 | 1.00 | 41.07 | O |
| HETATM15186 | O | HOH | F | 567 | 31.732 | 55.809 | 74.222 | 1.00 | 41.35 | O |
| HETATM15187 | O | HOH | F | 568 | 11.871 | 34.665 | 37.372 | 1.00 | 52.74 | O |
| HETATM15188 | O | HOH | F | 569 | 10.587 | 35.107 | 56.314 | 1.00 | 32.86 | O |
| HETATM15189 | O | HOH | F | 570 | 8.859 | 60.986 | 62.680 | 1.00 | 34.05 | O |
| HETATM15190 | O | HOH | F | 571 | 9.038 | 57.307 | 64.977 | 1.00 | 31.53 | O |
| HETATM15191 | O | HOH | F | 572 | 2.529 | 33.383 | 62.242 | 1.00 | 31.00 | O |
| HETATM15192 | O | HOH | F | 573 | -14.735 | 37.320 | 16.869 | 1.00 | 50.94 | O |
| HETATM15193 | O | HOH | F | 574 | -8.672 | 40.234 | 32.152 | 1.00 | 43.10 | O |
| HETATM15194 | O | HOH | F | 575 | -3.937 | 50.090 | 21.647 | 1.00 | 40.10 | O |
| HETATM15195 | O | HOH | F | 576 | 37.324 | 66.005 | 63.139 | 1.00 | 45.68 | O |
| HETATM15196 | O | HOH | F | 577 | -10.333 | 42.299 | 27.542 | 1.00 | 43.73 | O |
| HETATM15197 | O | HOH | F | 578 | 32.474 | 62.870 | 49.057 | 1.00 | 51.94 | O |
| HETATM15198 | O | HOH | F | 579 | 21.207 | 48.762 | 48.825 | 1.00 | 36.20 | O |

```
HETATM15199   O   HOH F 580     31.045  63.491  45.916  1.00 40.16        O
HETATM15200   O   HOH F 581     15.807  51.385  51.094  1.00 45.33        O
HETATM15201   O   HOH F 582     24.513  70.316  61.036  1.00 42.08        O
HETATM15202   O   HOH F 583     28.485  54.055  47.291  1.00 41.30        O
HETATM15203   O   HOH F 584     36.281  59.180  75.814  1.00 59.34        O
HETATM15204   O   HOH F 585     -3.185  63.364  43.039  1.00 40.91        O
HETATM15205   O   HOH F 586     -1.820  46.832  17.885  1.00 54.12        O
HETATM15206   O   HOH F 587     -0.786  35.448  16.333  1.00 56.98        O
HETATM15207   O   HOH F 588      2.914  34.666  43.377  1.00 42.47        O
HETATM15208   O   HOH F 589     -5.864  31.775  46.576  1.00 41.09        O
HETATM15209   O   HOH F 590     41.192  63.713  51.718  1.00 44.48        O
HETATM15210   O   HOH F 591     38.281  59.382  56.663  1.00 38.39        O
HETATM15211   O   HOH F 592     10.927  34.051  44.932  1.00 55.30        O
HETATM15212   O   HOH F 593      8.504  58.491  43.480  1.00 43.69        O
HETATM15213   O   HOH F 594      1.746  28.112  49.694  1.00 42.38        O
HETATM15214   O   HOH F 595      9.205  31.610  44.160  1.00 49.33        O
HETATM15215   O   HOH F 596     -7.714  59.014  49.053  1.00 58.10        O
HETATM15216   O   HOH F 597    -10.759  57.186  26.832  1.00 35.82        O
HETATM15217   O   HOH F 598     41.641  62.332  55.329  1.00 57.45        O
HETATM15218   O   HOH F 599     10.890  37.348  66.847  1.00 51.12        O
HETATM15219   O   HOH F 600     16.020  37.398  48.745  1.00 50.02        O
HETATM15220   O   HOH F 601     43.747  64.060  59.994  1.00 67.94        O
HETATM15221   O   HOH F 602     -3.570  41.921  16.572  1.00 48.60        O
HETATM15222   O   HOH F 603     14.352  30.376  42.175  1.00 57.49        O
HETATM15223   O   HOH F 604    -10.587  39.728  41.595  1.00 41.37        O
HETATM15224   O   HOH F 605     23.004  39.184  51.655  1.00 43.16        O
HETATM15225   O   HOH F 606    -14.555  44.979  32.225  1.00 57.01        O
HETATM15226   O   HOH F 607    -13.153  59.398  32.588  1.00 48.26        O
HETATM15227   O   HOH F 608     20.096  45.146  49.531  1.00 44.71        O
HETATM15228   O   HOH F 609     19.417  63.951  67.608  1.00 45.66        O
HETATM15229   O   HOH F 610      4.577  57.817  25.377  1.00 43.69        O
HETATM15230   O   HOH F 611    -10.356  63.252  35.989  1.00 45.69        O
HETATM15231   O   HOH F 612     -3.596  50.895  50.732  1.00 47.55        O
HETATM15232   O   HOH F 613    -11.429  43.290  12.755  1.00 70.49        O
HETATM15233   O   HOH F 614      7.667  58.795  37.202  1.00 41.07        O
HETATM15234   O   HOH F 615     -4.167  62.774  28.556  1.00 38.11        O
HETATM15235   O   HOH F 616     -3.478  37.642  29.415  1.00 58.77        O
HETATM15236   O   HOH F 617    -13.111  46.266  14.676  1.00 49.50        O
HETATM15237   O   HOH F 618     25.389  42.044  63.242  1.00 42.13        O
HETATM15238   O   HOH F 619     -5.901  57.581  23.630  1.00 58.36        O
HETATM15239   O   HOH F 620     41.937  65.802  58.907  1.00 51.30        O
HETATM15240   O   HOH F 621     26.021  69.691  70.843  1.00 57.25        O
HETATM15241   O   HOH F 622     23.694  39.269  63.705  1.00 47.22        O
HETATM15242   O   HOH F 623    -17.750  51.694  32.783  1.00 49.07        O
HETATM15243   O   HOH F 624     30.293  58.299  70.177  1.00 34.83        O
HETATM15244   O   HOH F 625     24.040  63.100  72.109  1.00 37.19        O
HETATM15245   O   HOH F 626    -14.703  58.339  29.593  1.00 44.62        O
HETATM15246   O   HOH F 627     -4.172  63.586  38.751  1.00 51.45        O
HETATM15247   O   HOH G 215     15.054  30.102 -52.183  1.00 45.07        O
HETATM15248   O   HOH G 216     27.605  35.613 -64.524  1.00 44.72        O
HETATM15249   O   HOH G 217     29.627  24.034 -48.564  1.00 11.44        O
HETATM15250   O   HOH G 218     15.595  15.417 -66.410  1.00 15.34        O
HETATM15251   O   HOH G 219     28.106  13.557 -67.142  1.00 14.45        O
HETATM15252   O   HOH G 220     16.611  25.612 -51.548  1.00 16.64        O
HETATM15253   O   HOH G 221    -19.446  17.411 -42.472  1.00 13.18        O
HETATM15254   O   HOH G 222     18.077   9.828 -46.734  1.00 17.69        O
HETATM15255   O   HOH G 223     25.337  26.889 -52.971  1.00 14.95        O
HETATM15256   O   HOH G 224     22.174  23.471 -48.230  1.00 19.63        O
HETATM15257   O   HOH G 225     32.912  26.273 -55.003  1.00 18.77        O
HETATM15258   O   HOH G 226     19.967  26.773 -53.261  1.00 16.55        O
HETATM15259   O   HOH G 227     17.459  18.495 -69.873  1.00 14.63        O
HETATM15260   O   HOH G 228     23.423  29.605 -58.867  1.00 18.78        O
HETATM15261   O   HOH G 229     11.181   9.339 -47.611  1.00 23.41        O
HETATM15262   O   HOH G 230      5.204   9.679 -48.404  1.00 19.28        O
HETATM15263   O   HOH G 231     32.085  10.796 -58.018  1.00 17.20        O
```

```
HETATM15264  O   HOH G 232      -4.216    9.823 -31.774  1.00 20.79           O
HETATM15265  O   HOH G 233      35.624   17.379 -61.472  1.00 19.06           O
HETATM15266  O   HOH G 234     -20.711   16.444 -40.140  1.00 17.06           O
HETATM15267  O   HOH G 235      12.825   28.859 -53.092  1.00 20.99           O
HETATM15268  O   HOH G 236      11.218   17.584 -66.120  1.00 19.63           O
HETATM15269  O   HOH G 237      15.181   12.954 -67.053  1.00 20.98           O
HETATM15270  O   HOH G 238      33.788   18.982 -72.295  1.00 21.72           O
HETATM15271  O   HOH G 239       5.062   17.476 -28.989  1.00 20.47           O
HETATM15272  O   HOH G 240      33.141   19.645 -54.801  1.00 18.08           O
HETATM15273  O   HOH G 241       5.452   22.243 -36.671  1.00 22.49           O
HETATM15274  O   HOH G 242      -7.944    8.090 -47.007  1.00 24.05           O
HETATM15275  O   HOH G 243      12.480    9.915 -64.625  1.00 20.23           O
HETATM15276  O   HOH G 244      27.345   28.302 -52.062  1.00 27.57           O
HETATM15277  O   HOH G 245      28.106   15.664 -69.508  1.00 19.72           O
HETATM15278  O   HOH G 246      -7.483   20.375 -43.691  1.00 27.82           O
HETATM15279  O   HOH G 247      17.572   20.545 -67.684  1.00 26.34           O
HETATM15280  O   HOH G 248      23.244    8.217 -45.934  1.00 19.63           O
HETATM15281  O   HOH G 249      19.545   11.805 -68.654  1.00 22.13           O
HETATM15282  O   HOH G 250     -12.047   15.232 -39.817  1.00 20.61           O
HETATM15283  O   HOH G 251      19.261    9.775 -65.659  1.00 16.09           O
HETATM15284  O   HOH G 252      17.052   23.473 -64.503  1.00 19.07           O
HETATM15285  O   HOH G 253       7.129   15.868 -11.545  1.00 30.87           O
HETATM15286  O   HOH G 254       1.874   10.535 -39.490  1.00 28.17           O
HETATM15287  O   HOH G 255      37.577   21.184 -67.120  1.00 26.49           O
HETATM15288  O   HOH G 256      -5.932   14.467 -51.506  1.00 24.76           O
HETATM15289  O   HOH G 257     -10.519   10.044 -33.424  1.00 20.86           O
HETATM15290  O   HOH G 258      -8.745   10.286 -26.189  1.00 19.03           O
HETATM15291  O   HOH G 259       2.476   24.242 -24.236  1.00 23.59           O
HETATM15292  O   HOH G 260       5.857   20.483 -15.291  1.00 27.38           O
HETATM15293  O   HOH G 261      17.126   26.288 -59.969  1.00 26.34           O
HETATM15294  O   HOH G 262     -16.256   14.268 -32.180  1.00 28.50           O
HETATM15295  O   HOH G 263     -10.393    9.400 -43.534  1.00 19.31           O
HETATM15296  O   HOH G 264      33.634   22.567 -65.354  1.00 29.52           O
HETATM15297  O   HOH G 265     -15.952   14.835 -37.504  1.00 20.86           O
HETATM15298  O   HOH G 266      20.399    7.781 -42.371  1.00 27.48           O
HETATM15299  O   HOH G 267      30.245   16.203 -72.750  1.00 21.97           O
HETATM15300  O   HOH G 268     -11.163   23.074 -34.354  1.00 28.13           O
HETATM15301  O   HOH G 269       4.262   12.002 -40.044  1.00 21.81           O
HETATM15302  O   HOH G 270     -13.796   12.066 -25.468  1.00 22.93           O
HETATM15303  O   HOH G 271       2.978   26.574 -23.580  1.00 29.02           O
HETATM15304  O   HOH G 272      13.100    6.544 -52.810  1.00 34.12           O
HETATM15305  O   HOH G 273      22.862   28.666 -52.382  1.00 18.57           O
HETATM15306  O   HOH G 274      20.835    6.818 -45.838  1.00 38.77           O
HETATM15307  O   HOH G 275     -18.310   14.349 -39.090  1.00 29.08           O
HETATM15308  O   HOH G 276       6.477   18.883 -53.742  1.00 26.74           O
HETATM15309  O   HOH G 277       7.734   24.043 -11.960  1.00 18.09           O
HETATM15310  O   HOH G 278     -12.902   17.710 -39.103  1.00 34.17           O
HETATM15311  O   HOH G 279      19.153   25.252 -46.648  1.00 27.65           O
HETATM15312  O   HOH G 280       8.708   11.044 -41.487  1.00 35.66           O
HETATM15313  O   HOH G 281       8.985   14.198 -38.296  1.00 24.29           O
HETATM15314  O   HOH G 282      -6.542    9.267 -24.782  1.00 26.92           O
HETATM15315  O   HOH G 283      16.724   13.911 -70.885  1.00 21.47           O
HETATM15316  O   HOH G 284      16.365   29.567 -54.397  1.00 26.58           O
HETATM15317  O   HOH G 285       8.431   16.203 -66.023  1.00 26.35           O
HETATM15318  O   HOH G 286      16.451    6.170 -64.998  1.00 28.31           O
HETATM15319  O   HOH G 287      12.186    8.536 -43.388  1.00 30.79           O
HETATM15320  O   HOH G 288      29.885   12.296 -70.933  1.00 22.37           O
HETATM15321  O   HOH G 289      -5.937    8.799 -38.727  1.00 25.25           O
HETATM15322  O   HOH G 290     -14.233   21.941 -21.965  1.00 24.40           O
HETATM15323  O   HOH G 291     -14.807   16.403 -10.069  1.00 43.61           O
HETATM15324  O   HOH G 292     -10.979    9.955 -37.201  1.00 28.70           O
HETATM15325  O   HOH G 293      16.770   11.983 -69.112  1.00 20.15           O
HETATM15326  O   HOH G 294     -13.759   13.289 -39.107  1.00 23.31           O
HETATM15327  O   HOH G 295      18.183   25.947 -64.319  1.00 33.66           O
HETATM15328  O   HOH G 296       2.746   20.040 -28.896  1.00 24.06           O
```

```
HETATM15329  O   HOH G 297    28.964  11.217 -68.261  1.00 29.32           O
HETATM15330  O   HOH G 298    -3.799   7.976 -51.311  1.00 34.19           O
HETATM15331  O   HOH G 299    17.041  27.280 -62.290  1.00 28.03           O
HETATM15332  O   HOH G 300    -4.609   7.758 -41.873  1.00 21.95           O
HETATM15333  O   HOH G 301    34.040  11.254 -59.887  1.00 25.11           O
HETATM15334  O   HOH G 302   -11.139  22.629 -42.435  1.00 31.07           O
HETATM15335  O   HOH G 303     4.458  21.013 -21.426  1.00 32.83           O
HETATM15336  O   HOH G 304    -0.336  15.349 -20.302  1.00 20.24           O
HETATM15337  O   HOH G 305    -3.764  23.544  -6.789  1.00 37.15           O
HETATM15338  O   HOH G 306     7.688  24.128 -14.493  1.00 38.73           O
HETATM15339  O   HOH G 307    -6.837  17.513 -50.286  1.00 30.30           O
HETATM15340  O   HOH G 308    16.417  27.560 -49.764  1.00 30.98           O
HETATM15341  O   HOH G 309    14.298  23.237 -57.212  1.00 28.82           O
HETATM15342  O   HOH G 310   -13.728  21.072 -42.231  1.00 31.86           O
HETATM15343  O   HOH G 311    30.676  31.278 -59.221  1.00 28.71           O
HETATM15344  O   HOH G 312     6.772  17.455 -63.494  1.00 40.34           O
HETATM15345  O   HOH G 313   -13.614  10.927 -38.119  1.00 29.10           O
HETATM15346  O   HOH G 314    25.620  25.670 -70.043  1.00 36.91           O
HETATM15347  O   HOH G 315    15.708  22.783 -47.528  1.00 25.43           O
HETATM15348  O   HOH G 316     1.947  15.257 -17.661  1.00 30.18           O
HETATM15349  O   HOH G 317    22.326   5.097 -64.478  1.00 24.39           O
HETATM15350  O   HOH G 318    -7.176  26.894 -28.697  1.00 27.13           O
HETATM15351  O   HOH G 319    -8.385   7.928 -30.665  1.00 37.92           O
HETATM15352  O   HOH G 320    -0.703  18.743 -47.211  1.00 28.76           O
HETATM15353  O   HOH G 321    19.832   2.139 -59.767  1.00 38.21           O
HETATM15354  O   HOH G 322     4.514  24.646 -35.288  1.00 38.05           O
HETATM15355  O   HOH G 323    20.150  21.605 -45.425  1.00 40.05           O
HETATM15356  O   HOH G 324    23.304  25.815 -49.201  1.00 34.43           O
HETATM15357  O   HOH G 325    34.890  29.510 -56.993  1.00 39.14           O
HETATM15358  O   HOH G 326    -9.093  12.484 -51.418  1.00 29.15           O
HETATM15359  O   HOH G 327     6.633  18.609 -59.640  1.00 35.23           O
HETATM15360  O   HOH G 328    -8.067  17.572 -53.050  1.00 45.73           O
HETATM15361  O   HOH G 329    11.539   5.349 -47.394  1.00 38.55           O
HETATM15362  O   HOH G 330     1.520  29.456 -26.964  1.00 39.35           O
HETATM15363  O   HOH G 331    12.907   7.625 -65.736  1.00 33.88           O
HETATM15364  O   HOH G 332     5.676  18.684 -41.847  1.00 28.84           O
HETATM15365  O   HOH G 333    14.009  15.385 -45.136  1.00 27.60           O
HETATM15366  O   HOH G 334     0.018  28.865 -17.016  1.00 45.83           O
HETATM15367  O   HOH G 335    11.269  16.443 -45.565  1.00 29.71           O
HETATM15368  O   HOH G 336     1.399  17.260 -48.713  1.00 36.28           O
HETATM15369  O   HOH G 337    23.036   5.675 -52.666  1.00 29.93           O
HETATM15370  O   HOH G 338    -2.308  24.123 -40.677  1.00 34.18           O
HETATM15371  O   HOH G 339    16.950  14.301 -41.701  1.00 38.74           O
HETATM15372  O   HOH G 340   -15.352  27.537 -15.567  1.00 43.86           O
HETATM15373  O   HOH G 341    17.905   8.069 -67.350  1.00 33.56           O
HETATM15374  O   HOH G 342    21.415  29.165 -65.270  1.00 38.52           O
HETATM15375  O   HOH G 343    -3.677   6.719 -35.733  1.00 39.74           O
HETATM15376  O   HOH G 344   -15.467  23.290 -13.465  1.00 31.92           O
HETATM15377  O   HOH G 345    37.641  28.766 -54.361  1.00 38.11           O
HETATM15378  O   HOH G 346    36.206  22.279 -64.550  1.00 29.12           O
HETATM15379  O   HOH G 347    21.383  10.580 -39.575  1.00 41.14           O
HETATM15380  O   HOH G 348   -15.438  17.314 -19.972  1.00 31.26           O
HETATM15381  O   HOH G 349    -4.634  25.093 -34.598  1.00 22.22           O
HETATM15382  O   HOH G 350    -8.649   7.434 -44.485  1.00 37.53           O
HETATM15383  O   HOH G 351    -9.357   8.602 -28.164  1.00 27.19           O
HETATM15384  O   HOH G 352    24.028   8.228 -68.561  1.00 38.88           O
HETATM15385  O   HOH G 353   -15.339  13.754 -21.027  1.00 34.48           O
HETATM15386  O   HOH G 354   -13.651  23.585 -33.192  1.00 33.36           O
HETATM15387  O   HOH G 355    -0.016  26.616 -34.842  1.00 43.44           O
HETATM15388  O   HOH G 356   -16.548  13.801 -35.371  1.00 31.63           O
HETATM15389  O   HOH G 357    15.208  16.303 -43.190  1.00 38.99           O
HETATM15390  O   HOH G 358     5.694  18.091 -46.288  1.00 38.92           O
HETATM15391  O   HOH G 359     2.731  15.338 -61.141  1.00 33.41           O
HETATM15392  O   HOH G 360     1.716   4.450 -61.191  1.00 36.59           O
HETATM15393  O   HOH G 361     1.152   6.943 -49.489  1.00 31.29           O
```

```
HETATM15394  O   HOH G 362    12.043  22.994 -58.619  1.00 35.75           O
HETATM15395  O   HOH G 363    21.763   7.751 -67.573  1.00 31.90           O
HETATM15396  O   HOH G 364    -3.734  29.504 -28.893  1.00 30.20           O
HETATM15397  O   HOH G 365    -1.534   6.348 -49.812  1.00 38.80           O
HETATM15398  O   HOH G 366     9.059   8.617 -41.799  1.00 38.05           O
HETATM15399  O   HOH G 367    14.895  26.473 -58.668  1.00 34.30           O
HETATM15400  O   HOH G 368     0.350   6.163 -35.481  1.00 32.93           O
HETATM15401  O   HOH G 369     8.040   4.776 -63.812  1.00 42.08           O
HETATM15402  O   HOH G 370    -4.480  19.541 -50.385  1.00 35.92           O
HETATM15403  O   HOH G 371    19.215  28.809 -48.699  1.00 39.06           O
HETATM15404  O   HOH G 372    -4.471  25.618 -31.762  1.00 34.84           O
HETATM15405  O   HOH G 373     7.700  21.727 -34.812  1.00 37.14           O
HETATM15406  O   HOH G 374     6.244   3.978 -58.817  1.00 35.57           O
HETATM15407  O   HOH G 375   -15.676  12.210 -16.510  1.00 50.16           O
HETATM15408  O   HOH G 376    37.765  27.437 -56.142  1.00 35.88           O
HETATM15409  O   HOH G 377    15.560   4.542 -46.432  1.00 44.32           O
HETATM15410  O   HOH G 378    17.714   5.016 -48.321  1.00 41.98           O
HETATM15411  O   HOH G 379    31.646  28.504 -66.123  1.00 26.62           O
HETATM15412  O   HOH G 380    13.900  21.806 -65.072  1.00 42.51           O
HETATM15413  O   HOH G 381     3.726  12.271 -67.362  1.00 32.47           O
HETATM15414  O   HOH G 382    26.281   4.879 -54.104  1.00 43.27           O
HETATM15415  O   HOH G 383    15.592   0.591 -57.089  1.00 37.46           O
HETATM15416  O   HOH G 384     4.020   7.093 -10.618  1.00 33.23           O
HETATM15417  O   HOH G 385    11.471  18.382 -43.678  1.00 47.03           O
HETATM15418  O   HOH G 386   -18.246  17.720 -33.701  1.00 28.91           O
HETATM15419  O   HOH G 387    13.685  29.925 -56.902  1.00 16.22           O
HETATM15420  O   HOH G 388    -3.852  19.861  -4.794  1.00 46.76           O
HETATM15421  O   HOH G 389    40.838  25.366 -51.955  1.00 34.78           O
HETATM15422  O   HOH G 390    -0.402  29.481  -8.912  1.00 38.85           O
HETATM15423  O   HOH G 391    15.936   9.462 -69.052  1.00 38.68           O
HETATM15424  O   HOH G 392    36.218  22.962 -70.196  1.00 47.53           O
HETATM15425  O   HOH G 393    -2.499   5.488 -42.816  1.00 41.78           O
HETATM15426  O   HOH G 394     5.184  20.576 -27.001  1.00 45.33           O
HETATM15427  O   HOH G 395    41.336  13.948 -65.476  1.00 41.18           O
HETATM15428  O   HOH G 396    21.177  30.820 -54.689  1.00 46.62           O
HETATM15429  O   HOH G 397    29.179  29.088 -52.689  1.00 32.41           O
HETATM15430  O   HOH G 398    30.269   9.025 -65.717  1.00 40.72           O
HETATM15431  O   HOH G 399     2.522   2.665 -68.364  1.00 47.38           O
HETATM15432  O   HOH G 400    26.525  23.059 -71.774  1.00 32.32           O
HETATM15433  O   HOH G 401    24.958   3.671 -62.304  1.00 26.28           O
HETATM15434  O   HOH G 402    11.387  21.444 -63.355  1.00 40.14           O
HETATM15435  O   HOH G 403    -5.257  17.105 -52.817  1.00 38.74           O
HETATM15436  O   HOH G 404    -1.716   4.342 -47.112  1.00 44.15           O
HETATM15437  O   HOH G 405    30.148  24.331 -70.351  1.00 24.96           O
HETATM15438  O   HOH G 406     6.624  20.274 -51.976  1.00 39.95           O
HETATM15439  O   HOH G 407   -10.245  16.612 -51.741  1.00 33.76           O
HETATM15440  O   HOH G 408    33.401  23.255 -69.558  1.00 45.26           O
HETATM15441  O   HOH G 409    25.826   9.755 -67.907  1.00 39.95           O
HETATM15442  O   HOH G 410   -15.640  18.889  -8.196  1.00 49.13           O
HETATM15443  O   HOH G 411    28.567  29.694 -66.040  1.00 39.98           O
HETATM15444  O   HOH G 412    -7.085  21.847 -45.863  1.00 28.85           O
HETATM15445  O   HOH G 413    36.548  25.711 -60.963  1.00 39.12           O
HETATM15446  O   HOH G 414    -2.648  21.664 -46.158  1.00 33.09           O
HETATM15447  O   HOH G 415    33.375  33.421 -52.357  1.00 50.93           O
HETATM15448  O   HOH G 416    -2.740  17.376  -7.332  1.00 38.83           O
HETATM15449  O   HOH G 417     4.284  12.764  -7.795  1.00 48.66           O
HETATM15450  O   HOH G 418    -2.190  20.552 -49.507  1.00 38.79           O
HETATM15451  O   HOH G 419     4.248   4.500 -52.523  1.00 37.41           O
HETATM15452  O   HOH G 420    29.930  32.468 -53.903  1.00 38.14           O
HETATM15453  O   HOH G 421    15.794   6.076 -53.108  1.00 41.81           O
HETATM15454  O   HOH G 422     0.563  14.472  -8.339  1.00 42.15           O
HETATM15455  O   HOH G 423    29.459  28.706 -68.663  1.00 54.28           O
HETATM15456  O   HOH G 424    23.408  29.689 -63.529  1.00 41.03           O
HETATM15457  O   HOH G 425     5.840  19.806 -44.562  1.00 30.91           O
HETATM15458  O   HOH G 426    20.051   3.885 -52.834  1.00 44.09           O
```

```
HETATM15459  O   HOH G 427    27.168  33.320 -62.723  1.00 45.91        O
HETATM15460  O   HOH G 428    -6.654  24.614  -8.003  1.00 46.33        O
HETATM15461  O   HOH G 429    19.511  29.018 -51.937  1.00 37.59        O
HETATM15462  O   HOH G 430    16.374  27.420 -66.176  1.00 55.71        O
HETATM15463  O   HOH G 431     8.169  21.693 -60.772  1.00 48.89        O
HETATM15464  O   HOH G 432    24.061   3.667 -54.408  1.00 54.16        O
HETATM15465  O   HOH G 433   -11.306  10.695 -24.853  1.00 32.13        O
HETATM15466  O   HOH G 434     2.807  27.916 -20.672  1.00 50.06        O
HETATM15467  O   HOH G 435   -19.792  15.695 -35.701  1.00 52.89        O
HETATM15468  O   HOH G 436     7.491  16.557 -45.082  1.00 30.85        O
HETATM15469  O   HOH G 437    35.496  31.056 -49.916  1.00 38.47        O
HETATM15470  O   HOH G 438    11.220   1.991 -60.129  1.00 51.45        O
HETATM15471  O   HOH G 439    40.419  25.726 -54.893  1.00 45.93        O
HETATM15472  O   HOH G 440    31.836  31.209 -64.116  1.00 69.51        O
HETATM15473  O   HOH G 441   -18.650  14.660 -18.580  1.00 47.21        O
HETATM15474  O   HOH G 442     6.256  14.794 -66.634  1.00 39.56        O
HETATM15475  O   HOH G 443   -14.912  23.040 -36.560  1.00 45.73        O
HETATM15476  O   HOH G 444   -13.394  12.168 -18.153  1.00 38.01        O
HETATM15477  O   HOH G 445    38.896  12.824 -65.924  1.00 38.75        O
HETATM15478  O   HOH G 446    -2.247  10.989 -18.760  1.00 47.89        O
HETATM15479  O   HOH G 447     0.518  16.179 -59.326  1.00 43.65        O
HETATM15480  O   HOH G 448     3.230  25.590 -61.739  1.00 58.93        O
HETATM15481  O   HOH G 449     6.560   5.195 -65.961  1.00 43.02        O
HETATM15482  O   HOH G 450    -3.215  25.665 -47.203  1.00 55.38        O
HETATM15483  O   HOH G 451    13.072  23.193 -48.399  1.00 39.40        O
HETATM15484  O   HOH G 452   -12.892  13.275 -11.537  1.00 51.85        O
HETATM15485  O   HOH G 453     6.181  22.560 -43.133  1.00 48.12        O
HETATM15486  O   HOH G 454   -14.849  20.615 -44.796  1.00 50.03        O
HETATM15487  O   HOH G 455    -1.839  28.544 -15.541  1.00 45.49        O
HETATM15488  O   HOH G 456   -16.319  21.612 -24.345  1.00 48.64        O
HETATM15489  O   HOH G 457     3.046  28.950 -24.964  1.00 41.64        O
HETATM15490  O   HOH G 458     4.858  22.268 -24.975  1.00 32.59        O
HETATM15491  O   HOH G 459   -14.249  22.016 -10.604  1.00 50.42        O
HETATM15492  O   HOH G 460     9.046  26.004 -56.723  1.00 49.16        O
HETATM15493  O   HOH G 461    -2.609  18.547 -50.800  1.00 45.73        O
HETATM15494  O   HOH G 462    13.900   0.294 -60.654  1.00 54.30        O
HETATM15495  O   HOH G 463   -14.259  15.779 -12.350  1.00 30.01        O
HETATM15496  O   HOH G 464     5.432  21.403 -50.067  1.00 57.94        O
HETATM15497  O   HOH G 465   -19.137  22.423 -37.585  1.00 42.79        O
HETATM15498  O   HOH G 466   -10.004   7.989 -39.472  1.00 47.51        O
HETATM15499  O   HOH G 467    -8.770  22.473 -41.093  1.00 59.03        O
HETATM15500  O   HOH G 468   -10.559  10.873 -22.393  1.00 42.79        O
HETATM15501  O   HOH G 469   -17.415  12.168 -39.279  1.00 39.35        O
HETATM15502  O   HOH G 470   -11.680   8.142 -41.614  1.00 31.45        O
HETATM15503  O   HOH H 316    42.385  16.952 -35.721  1.00 17.54        O
HETATM15504  O   HOH H 317    36.048   9.464 -50.261  1.00 13.25        O
HETATM15505  O   HOH H 318    27.085  24.537 -49.418  1.00  9.18        O
HETATM15506  O   HOH H 319    46.271  13.778 -40.272  1.00 15.59        O
HETATM15507  O   HOH H 320    36.448  33.679 -29.405  1.00 17.45        O
HETATM15508  O   HOH H 321    45.907   7.899 -46.515  1.00 19.85        O
HETATM15509  O   HOH H 322    35.235  28.580 -48.393  1.00 23.39        O
HETATM15510  O   HOH H 323    25.811   7.199 -47.406  1.00 18.20        O
HETATM15511  O   HOH H 324    21.437  15.949 -26.310  1.00 22.11        O
HETATM15512  O   HOH H 325    25.474   8.207 -49.875  1.00 15.33        O
HETATM15513  O   HOH H 326    13.884  21.384 -16.993  1.00 16.93        O
HETATM15514  O   HOH H 327    39.879  23.193 -56.047  1.00 17.81        O
HETATM15515  O   HOH H 328    31.709  25.520 -47.185  1.00 14.66        O
HETATM15516  O   HOH H 329    33.239   9.823 -55.306  1.00 16.11        O
HETATM15517  O   HOH H 330     1.114   6.580 -32.833  1.00 21.70        O
HETATM15518  O   HOH H 331    42.144  17.206 -58.305  1.00 27.66        O
HETATM15519  O   HOH H 332    22.177   9.395 -23.307  1.00 27.32        O
HETATM15520  O   HOH H 333     2.725  -0.773 -19.216  1.00 29.56        O
HETATM15521  O   HOH H 334    23.330  21.007 -32.907  1.00 27.57        O
HETATM15522  O   HOH H 335    33.883  32.610 -30.109  1.00 19.30        O
HETATM15523  O   HOH H 336    -3.427   7.705 -32.776  1.00 19.62        O
```

```
HETATM15524  O   HOH H 337     31.438  19.374 -24.639  1.00 18.22           O
HETATM15525  O   HOH H 338     45.433  21.383 -40.894  1.00 23.55           O
HETATM15526  O   HOH H 339     44.761  11.935 -53.359  1.00 20.06           O
HETATM15527  O   HOH H 340     33.003   7.025 -33.748  1.00 17.63           O
HETATM15528  O   HOH H 341      6.789  19.517 -29.390  1.00 26.92           O
HETATM15529  O   HOH H 342     12.080  -1.165 -22.410  1.00 31.87           O
HETATM15530  O   HOH H 343     47.141   8.447 -40.411  1.00 20.49           O
HETATM15531  O   HOH H 344     19.357  25.703 -20.360  1.00 24.97           O
HETATM15532  O   HOH H 345     23.666  19.334 -29.370  1.00 31.27           O
HETATM15533  O   HOH H 346     -8.133  10.768 -21.876  1.00 21.52           O
HETATM15534  O   HOH H 347      4.292  17.715 -26.145  1.00 23.67           O
HETATM15535  O   HOH H 348      2.262  -0.987 -32.677  1.00 28.15           O
HETATM15536  O   HOH H 349     11.231  12.770 -15.159  1.00 26.08           O
HETATM15537  O   HOH H 350     19.528  22.859 -29.420  1.00 19.51           O
HETATM15538  O   HOH H 351     -4.792  -5.439 -22.891  1.00 30.47           O
HETATM15539  O   HOH H 352     22.289  13.228 -36.712  1.00 29.63           O
HETATM15540  O   HOH H 353     33.288  29.619 -45.682  1.00 26.48           O
HETATM15541  O   HOH H 354     38.398   0.033 -49.944  1.00 22.90           O
HETATM15542  O   HOH H 355     13.089  18.247 -32.597  1.00 31.44           O
HETATM15543  O   HOH H 356     48.291  10.635 -53.948  1.00 31.50           O
HETATM15544  O   HOH H 357     23.962  10.665 -16.736  1.00 27.73           O
HETATM15545  O   HOH H 358     46.016  24.030 -53.016  1.00 31.98           O
HETATM15546  O   HOH H 359     21.753  23.002 -30.449  1.00 34.72           O
HETATM15547  O   HOH H 360     25.140  31.405 -26.530  1.00 28.97           O
HETATM15548  O   HOH H 361     32.300   2.318 -38.715  1.00 28.60           O
HETATM15549  O   HOH H 362     33.167   8.488 -51.748  1.00 25.40           O
HETATM15550  O   HOH H 363     23.846  28.014 -30.329  1.00 27.24           O
HETATM15551  O   HOH H 364     10.593   1.966 -30.651  1.00 26.30           O
HETATM15552  O   HOH H 365     46.931  21.134 -51.728  1.00 30.26           O
HETATM15553  O   HOH H 366     25.405  24.659 -41.700  1.00 30.82           O
HETATM15554  O   HOH H 367     25.166  15.557 -29.141  1.00 25.05           O
HETATM15555  O   HOH H 368     45.592  23.282 -44.471  1.00 19.51           O
HETATM15556  O   HOH H 369     35.185  34.436 -36.493  1.00 29.13           O
HETATM15557  O   HOH H 370     48.519   7.143 -49.338  1.00 28.36           O
HETATM15558  O   HOH H 371     26.981  25.545 -38.113  1.00 20.62           O
HETATM15559  O   HOH H 372     50.684  18.600 -49.766  1.00 31.18           O
HETATM15560  O   HOH H 373     43.315   2.330 -43.109  1.00 28.11           O
HETATM15561  O   HOH H 374      4.632   2.019 -38.401  1.00 35.30           O
HETATM15562  O   HOH H 375     29.794   0.753 -51.110  1.00 29.17           O
HETATM15563  O   HOH H 376     20.535  27.730 -37.992  1.00 30.47           O
HETATM15564  O   HOH H 377     38.880  19.856 -27.368  1.00 28.29           O
HETATM15565  O   HOH H 378      9.653  18.426 -13.854  1.00 22.35           O
HETATM15566  O   HOH H 379     36.745  34.602 -32.044  1.00 25.33           O
HETATM15567  O   HOH H 380     15.880   6.240 -26.472  1.00 29.74           O
HETATM15568  O   HOH H 381     44.890   8.999 -33.237  1.00 35.10           O
HETATM15569  O   HOH H 382     44.105  17.894 -34.025  1.00 22.27           O
HETATM15570  O   HOH H 383     29.587  10.598 -28.556  1.00 29.54           O
HETATM15571  O   HOH H 384     53.298  11.954 -45.852  1.00 36.62           O
HETATM15572  O   HOH H 385     24.382  27.158 -36.347  1.00 26.23           O
HETATM15573  O   HOH H 386     39.140  26.960 -27.811  1.00 17.30           O
HETATM15574  O   HOH H 387     35.327  31.161 -23.961  1.00 26.66           O
HETATM15575  O   HOH H 388     42.549  25.486 -40.005  1.00 30.98           O
HETATM15576  O   HOH H 389     23.650  17.023 -27.819  1.00 22.25           O
HETATM15577  O   HOH H 390     36.036   0.291 -51.394  1.00 27.94           O
HETATM15578  O   HOH H 391     41.578   0.075 -39.077  1.00 25.82           O
HETATM15579  O   HOH H 392     48.468   5.396 -44.571  1.00 29.58           O
HETATM15580  O   HOH H 393     44.940  13.141 -35.029  1.00 29.53           O
HETATM15581  O   HOH H 394     21.272  22.668 -33.074  1.00 29.96           O
HETATM15582  O   HOH H 395     27.226  28.926 -40.143  1.00 35.32           O
HETATM15583  O   HOH H 396     29.407  29.905 -42.727  1.00 31.72           O
HETATM15584  O   HOH H 397     36.513  -2.589 -42.042  1.00 43.17           O
HETATM15585  O   HOH H 398     46.128  22.287 -61.864  1.00 34.28           O
HETATM15586  O   HOH H 399      8.216   3.577 -15.029  1.00 27.80           O
HETATM15587  O   HOH H 400     13.335  12.643 -31.179  1.00 25.80           O
HETATM15588  O   HOH H 401     36.475  -1.200 -53.617  1.00 22.57           O
```

```
HETATM15589  O   HOH H 402     54.830  12.382 -49.733  1.00 36.49           O
HETATM15590  O   HOH H 403     41.926  25.365 -47.472  1.00 32.14           O
HETATM15591  O   HOH H 404     22.023  28.676 -26.763  1.00 31.12           O
HETATM15592  O   HOH H 405      4.649  -7.717 -20.858  1.00 41.18           O
HETATM15593  O   HOH H 406     10.273   7.789 -33.779  1.00 36.07           O
HETATM15594  O   HOH H 407     31.013  -2.392 -42.631  1.00 42.55           O
HETATM15595  O   HOH H 408     15.322  15.905 -30.684  1.00 33.90           O
HETATM15596  O   HOH H 409     22.500  14.433 -34.289  1.00 34.83           O
HETATM15597  O   HOH H 410      9.928  26.224 -31.442  1.00 31.20           O
HETATM15598  O   HOH H 411     -5.715   4.716 -30.974  1.00 34.53           O
HETATM15599  O   HOH H 412     27.081  11.497 -31.587  1.00 29.30           O
HETATM15600  O   HOH H 413      9.791  23.046 -18.781  1.00 25.30           O
HETATM15601  O   HOH H 414      6.620   3.470 -36.913  1.00 37.63           O
HETATM15602  O   HOH H 415     24.197  13.924 -31.611  1.00 27.56           O
HETATM15603  O   HOH H 416     43.775  25.628 -56.190  1.00 38.62           O
HETATM15604  O   HOH H 417     38.693  23.521 -60.842  1.00 34.76           O
HETATM15605  O   HOH H 418     24.148  28.067 -38.903  1.00 32.58           O
HETATM15606  O   HOH H 419     21.652  21.804 -37.144  1.00 29.48           O
HETATM15607  O   HOH H 420     41.458  28.769 -33.001  1.00 36.24           O
HETATM15608  O   HOH H 421     42.286  15.323 -63.153  1.00 26.65           O
HETATM15609  O   HOH H 422     49.344  15.302 -53.624  1.00 44.28           O
HETATM15610  O   HOH H 423     -5.769   3.840 -34.084  1.00 27.85           O
HETATM15611  O   HOH H 424     19.787  14.719 -34.387  1.00 33.63           O
HETATM15612  O   HOH H 425     17.406  32.574 -38.771  1.00 37.78           O
HETATM15613  O   HOH H 426     21.983  26.260 -35.824  1.00 37.95           O
HETATM15614  O   HOH H 427     44.414  14.556 -53.413  1.00 29.36           O
HETATM15615  O   HOH H 428      0.714   6.335 -17.283  1.00 31.60           O
HETATM15616  O   HOH H 429     40.267   0.690 -43.007  1.00 27.54           O
HETATM15617  O   HOH H 430     45.650  10.774 -35.460  1.00 34.00           O
HETATM15618  O   HOH H 431     36.980   0.709 -55.825  1.00 42.62           O
HETATM15619  O   HOH H 432     46.541  21.747 -48.985  1.00 37.31           O
HETATM15620  O   HOH H 433     17.136   5.346 -23.018  1.00 43.17           O
HETATM15621  O   HOH H 434     12.966  10.112 -31.228  1.00 21.37           O
HETATM15622  O   HOH H 435      9.185   9.888 -35.802  1.00 32.35           O
HETATM15623  O   HOH H 436     25.838  10.178 -33.666  1.00 35.94           O
HETATM15624  O   HOH H 437     27.116   8.286 -35.072  1.00 36.84           O
HETATM15625  O   HOH H 438     30.903  14.060 -26.030  1.00 24.19           O
HETATM15626  O   HOH H 439     46.570   5.587 -49.835  1.00 35.61           O
HETATM15627  O   HOH H 440     -5.509   7.295 -26.141  1.00 25.91           O
HETATM15628  O   HOH H 441     -0.248   0.438 -34.482  1.00 36.37           O
HETATM15629  O   HOH H 442    -11.647   6.048 -20.545  1.00 51.12           O
HETATM15630  O   HOH H 443     42.072  17.379 -64.743  1.00 37.62           O
HETATM15631  O   HOH H 444      8.076  -2.359 -21.366  1.00 31.85           O
HETATM15632  O   HOH H 445     27.193   4.698 -42.690  1.00 34.89           O
HETATM15633  O   HOH H 446     -6.428   5.409 -27.727  1.00 35.16           O
HETATM15634  O   HOH H 447      7.628  -4.716 -27.201  1.00 24.38           O
HETATM15635  O   HOH H 448     28.257   0.187 -49.163  1.00 32.03           O
HETATM15636  O   HOH H 449     24.203   7.294 -43.697  1.00 36.26           O
HETATM15637  O   HOH H 450     35.673  17.544 -26.952  1.00 33.85           O
HETATM15638  O   HOH H 451      9.586   3.137 -35.113  1.00 32.98           O
HETATM15639  O   HOH H 452     11.297   3.399 -32.724  1.00 31.70           O
HETATM15640  O   HOH H 453     14.594  13.333 -15.693  1.00 39.27           O
HETATM15641  O   HOH H 454     26.473  11.630 -24.928  1.00 36.85           O
HETATM15642  O   HOH H 455     40.872  -1.582 -43.580  1.00 37.02           O
HETATM15643  O   HOH H 456     47.586   4.912 -33.811  1.00 37.39           O
HETATM15644  O   HOH H 457     12.510  21.346 -33.731  1.00 33.85           O
HETATM15645  O   HOH H 458     27.118  26.476 -21.690  1.00 39.43           O
HETATM15646  O   HOH H 459     30.176   8.913 -57.330  1.00 30.86           O
HETATM15647  O   HOH H 460     39.476   4.653 -32.232  1.00 41.07           O
HETATM15648  O   HOH H 461    -10.891   1.774 -29.908  1.00 35.09           O
HETATM15649  O   HOH H 462     27.744  27.437 -19.308  1.00 35.40           O
HETATM15650  O   HOH H 463     20.095  20.381 -38.838  1.00 40.65           O
HETATM15651  O   HOH H 464     28.005   0.514 -53.941  1.00 41.44           O
HETATM15652  O   HOH H 465     -5.416   5.158 -18.410  1.00 27.32           O
HETATM15653  O   HOH H 466     16.488   2.211 -26.841  1.00 35.41           O
```

| | | | | | | | | |
|---|---|---|---|---|---|---|---|---|
| HETATM15654 | O | HOH H 467 | 35.543 | 10.894 | -30.485 | 1.00 | 33.62 | O |
| HETATM15655 | O | HOH H 468 | 32.606 | 31.994 | -46.418 | 1.00 | 39.37 | O |
| HETATM15656 | O | HOH H 469 | 33.542 | 12.810 | -26.326 | 1.00 | 32.13 | O |
| HETATM15657 | O | HOH H 470 | -6.811 | 3.207 | -18.770 | 1.00 | 39.38 | O |
| HETATM15658 | O | HOH H 471 | 40.164 | 2.290 | -31.622 | 1.00 | 41.66 | O |
| HETATM15659 | O | HOH H 472 | 25.713 | 34.395 | -29.902 | 1.00 | 39.93 | O |
| HETATM15660 | O | HOH H 473 | 27.572 | -2.531 | -49.533 | 1.00 | 42.96 | O |
| HETATM15661 | O | HOH H 474 | -7.709 | 6.027 | -11.082 | 1.00 | 50.38 | O |
| HETATM15662 | O | HOH H 475 | 6.872 | 24.339 | -23.840 | 1.00 | 38.07 | O |
| HETATM15663 | O | HOH H 476 | 37.116 | 0.158 | -38.698 | 1.00 | 41.76 | O |
| HETATM15664 | O | HOH H 477 | 47.576 | 21.408 | -47.009 | 1.00 | 38.30 | O |
| HETATM15665 | O | HOH H 478 | 47.214 | 2.278 | -34.206 | 1.00 | 44.11 | O |
| HETATM15666 | O | HOH H 479 | 41.787 | 14.546 | -59.181 | 1.00 | 33.34 | O |
| HETATM15667 | O | HOH H 480 | 20.776 | 8.162 | -17.030 | 1.00 | 37.03 | O |
| HETATM15668 | O | HOH H 481 | 27.435 | 5.182 | -46.041 | 1.00 | 42.37 | O |
| HETATM15669 | O | HOH H 482 | -9.089 | 2.194 | -32.919 | 1.00 | 46.39 | O |
| HETATM15670 | O | HOH H 483 | 44.160 | 0.359 | -40.868 | 1.00 | 37.90 | O |
| HETATM15671 | O | HOH H 484 | 42.092 | 23.309 | -34.506 | 1.00 | 26.83 | O |
| HETATM15672 | O | HOH H 485 | -4.537 | 14.198 | -13.969 | 1.00 | 41.66 | O |
| HETATM15673 | O | HOH H 486 | -12.819 | 3.567 | -29.844 | 1.00 | 46.34 | O |
| HETATM15674 | O | HOH H 487 | -7.542 | 8.489 | -8.788 | 1.00 | 37.16 | O |
| HETATM15675 | O | HOH H 488 | 45.828 | 17.764 | -58.158 | 1.00 | 35.02 | O |
| HETATM15676 | O | HOH H 489 | 0.743 | -2.461 | -18.632 | 1.00 | 34.59 | O |
| HETATM15677 | O | HOH H 490 | 34.436 | 6.353 | -53.705 | 1.00 | 41.90 | O |
| HETATM15678 | O | HOH H 491 | -12.156 | 1.440 | -27.760 | 1.00 | 42.66 | O |
| HETATM15679 | O | HOH H 492 | 18.715 | 21.377 | -40.721 | 1.00 | 32.09 | O |
| HETATM15680 | O | HOH H 493 | 26.325 | 27.596 | -16.463 | 1.00 | 42.49 | O |
| HETATM15681 | O | HOH H 494 | 33.116 | 27.465 | -48.458 | 1.00 | 33.32 | O |
| HETATM15682 | O | HOH H 495 | 23.616 | 29.203 | -34.813 | 1.00 | 32.16 | O |
| HETATM15683 | O | HOH H 496 | 26.787 | 5.333 | -52.034 | 1.00 | 41.90 | O |
| HETATM15684 | O | HOH H 497 | 26.250 | 3.608 | -50.286 | 1.00 | 39.47 | O |
| HETATM15685 | O | HOH H 498 | 31.029 | 29.833 | -39.229 | 1.00 | 34.42 | O |
| HETATM15686 | O | HOH H 499 | 8.555 | -2.166 | -37.434 | 1.00 | 45.92 | O |
| HETATM15687 | O | HOH H 500 | 23.709 | 14.441 | -17.121 | 1.00 | 33.61 | O |
| HETATM15688 | O | HOH H 501 | 2.943 | -1.736 | -39.816 | 1.00 | 47.82 | O |
| HETATM15689 | O | HOH H 502 | -4.499 | 1.874 | -35.714 | 1.00 | 42.62 | O |
| HETATM15690 | O | HOH H 503 | 37.180 | 20.778 | -62.440 | 1.00 | 34.65 | O |
| HETATM15691 | O | HOH H 504 | 37.428 | 28.053 | -49.276 | 1.00 | 48.69 | O |
| HETATM15692 | O | HOH H 505 | 51.517 | 25.413 | -59.107 | 1.00 | 54.65 | O |
| HETATM15693 | O | HOH H 506 | 21.621 | 10.335 | -15.686 | 1.00 | 41.39 | O |
| HETATM15694 | O | HOH H 507 | 31.031 | 4.271 | -54.981 | 1.00 | 38.64 | O |
| HETATM15695 | O | HOH H 508 | 48.036 | 9.511 | -36.976 | 1.00 | 34.13 | O |
| HETATM15696 | O | HOH H 509 | 38.992 | 1.795 | -34.984 | 1.00 | 46.57 | O |
| HETATM15697 | O | HOH H 510 | 39.609 | 25.418 | -50.229 | 1.00 | 39.40 | O |
| HETATM15698 | O | HOH H 511 | 40.520 | 32.471 | -35.478 | 1.00 | 41.08 | O |
| HETATM15699 | O | HOH H 512 | 10.192 | 26.647 | -20.937 | 1.00 | 43.66 | O |
| HETATM15700 | O | HOH H 513 | 28.536 | 33.118 | -33.989 | 1.00 | 21.26 | O |
| HETATM15701 | O | HOH H 514 | 31.477 | 18.739 | -29.059 | 1.00 | 22.65 | O |
| HETATM15702 | O | HOH H 515 | 35.655 | 13.117 | -29.977 | 1.00 | 43.55 | O |
| HETATM15703 | O | HOH H 516 | 43.888 | 13.485 | -32.997 | 1.00 | 39.55 | O |
| HETATM15704 | O | HOH H 517 | 36.609 | 34.211 | -34.679 | 1.00 | 36.58 | O |
| HETATM15705 | O | HOH H 518 | 17.437 | 6.139 | -28.590 | 1.00 | 54.32 | O |
| HETATM15706 | O | HOH H 519 | -7.658 | 3.273 | -21.002 | 1.00 | 39.96 | O |
| HETATM15707 | O | HOH H 520 | 39.381 | 2.141 | -54.001 | 1.00 | 37.37 | O |
| HETATM15708 | O | HOH H 521 | 38.254 | 0.220 | -58.389 | 1.00 | 44.95 | O |
| HETATM15709 | O | HOH H 522 | 1.046 | 0.330 | -37.286 | 1.00 | 36.32 | O |
| HETATM15710 | O | HOH H 523 | 39.492 | 4.793 | -53.674 | 1.00 | 41.69 | O |
| HETATM15711 | O | HOH H 524 | 37.675 | 1.302 | -42.679 | 1.00 | 41.41 | O |
| HETATM15712 | O | HOH H 525 | -9.465 | 7.522 | -16.819 | 1.00 | 51.63 | O |
| HETATM15713 | O | HOH H 526 | 48.417 | 5.889 | -39.399 | 1.00 | 33.88 | O |
| HETATM15714 | O | HOH H 527 | 40.416 | 31.158 | -33.383 | 1.00 | 39.87 | O |
| HETATM15715 | O | HOH H 528 | 4.965 | 0.032 | -39.179 | 1.00 | 36.96 | O |
| HETATM15716 | O | HOH H 529 | -0.279 | -1.702 | -15.588 | 1.00 | 42.42 | O |
| HETATM15717 | O | HOH H 530 | 12.498 | 14.847 | -32.332 | 1.00 | 44.79 | O |
| HETATM15718 | O | HOH H 531 | 10.739 | 1.146 | -16.303 | 1.00 | 53.58 | O |

```
HETATM15719  O    HOH H 532    34.134  16.060 -27.957  1.00 41.48         O
HETATM15720  O    HOH H 533    40.609  21.166 -65.288  1.00 35.66         O
HETATM15721  O    HOH H 534    45.925  17.189 -52.085  1.00 40.92         O
HETATM15722  O    HOH H 535     9.278   5.684 -35.966  1.00 35.80         O
HETATM15723  O    HOH H 536    48.609  17.651 -53.326  1.00 49.81         O
HETATM15724  O    HOH H 537    24.928  34.707 -23.839  1.00 34.70         O
HETATM15725  O    HOH H 538    47.674  24.704 -54.928  1.00 48.33         O
HETATM15726  O    HOH H 539     8.128   5.701 -13.405  1.00 46.88         O
HETATM15727  O    HOH H 540    16.196   0.678 -23.509  1.00 42.87         O
HETATM15728  O    HOH H 541    30.211  -0.850 -36.545  1.00 48.78         O
HETATM15729  O    HOH H 542    44.221  22.075 -30.916  1.00 43.68         O
HETATM15730  O    HOH H 543    12.078  30.854 -25.805  1.00 43.90         O
HETATM15731  O    HOH H 544    37.171  11.915 -62.682  1.00 40.29         O
HETATM15732  O    HOH H 545    50.788  18.483 -45.464  1.00 45.13         O
HETATM15733  O    HOH H 546    13.698  19.163 -17.143  1.00 30.40         O
HETATM15734  O    HOH H 547    42.024   1.255 -54.201  1.00 48.98         O
HETATM15735  O    HOH H 548    43.463   6.001 -53.043  1.00 38.35         O
HETATM15736  O    HOH H 549     2.451  -5.326 -17.671  1.00 41.29         O
HETATM15737  O    HOH H 550    34.962   0.196 -37.116  1.00 45.63         O
HETATM15738  O    HOH H 551    19.879  26.009 -15.802  1.00 56.16         O
HETATM15739  O    HOH H 552    47.439   5.427 -31.209  1.00 54.03         O
HETATM15740  O    HOH H 553    33.906  10.975 -28.296  1.00 39.91         O
HETATM15741  O    HOH H 554     3.124   5.872 -37.312  1.00 46.14         O
HETATM15742  O    HOH H 555    20.652  13.261 -39.309  1.00 44.84         O
HETATM15743  O    HOH H 556     8.099  11.956 -37.034  1.00 49.89         O
HETATM15744  O    HOH H 557     3.115   8.352 -38.266  1.00 36.69         O
HETATM15745  O    HOH H 558    31.713  -1.213 -50.921  1.00 48.99         O
HETATM15746  O    HOH H 559    42.929  30.664 -47.534  1.00 51.64         O
HETATM15747  O    HOH H 560    44.608  24.233 -47.871  1.00 40.89         O
HETATM15748  O    HOH H 561     2.262  13.736 -19.285  1.00 37.55         O
HETATM15749  O    HOH H 562    53.500  18.403 -49.769  1.00 35.98         O
HETATM15750  O    HOH H 563    22.982   7.074 -18.650  1.00 38.05         O
HETATM15751  O    HOH H 564    35.494   6.593 -30.167  1.00 60.61         O
HETATM15752  O    HOH H 565    47.406  19.735 -60.078  1.00 41.05         O
HETATM15753  O    HOH H 566    41.209  17.737 -28.141  1.00 45.49         O
HETATM15754  O    HOH H 567     5.249   4.765 -14.809  1.00 49.40         O
HETATM15755  O    HOH H 568    29.035  12.384 -26.991  1.00 44.44         O
HETATM15756  O    HOH H 569    -2.361  -9.583 -25.053  1.00 44.92         O
HETATM15757  O    HOH H 570    34.728   2.783 -32.159  1.00 46.13         O
HETATM15758  O    HOH H 571    -0.569   9.671 -17.161  1.00 41.27         O
HETATM15759  O    HOH H 572    45.697   5.295 -52.299  1.00 47.21         O
HETATM15760  O    HOH H 573    47.769   6.919 -52.323  1.00 44.31         O
HETATM15761  O    HOH H 574    38.642  12.548 -30.289  1.00 47.50         O
HETATM15762  O    HOH H 575    26.589  13.291 -28.094  1.00 49.29         O
HETATM15763  O    HOH H 576    51.764   6.124 -53.208  1.00 48.66         O
END
```

**References**

1. Niyogi SK (2005) Shigellosis. J Microbiol 43:133-143
2. Kotloff KL, Winickoff JP, Ivanoff B, Clemens JD, Swerdlow DL, Sansonetti PJ, Adak GK, Levine MM (1999) Global burden of Shigella infections: implications for vaccine development and implementation of control strategies. Bull World Health Organ 77:651-666
3. Jennison AV, Verma NK (2004) Shigella flexneri infection: pathogenesis and vaccine development. FEMS Microbiol Rev 28:43-58
4. Lindberg AA., Karnell A, Weintraub A (1991) The lipopolysaccharide of Shigella bacteria as a virulence factor. Rev Infect Dis 13:S279-284
5. Allison GE, Verma NK (2000) Serotype-converting bacteriophages and O-antigen modification in Shigella flexneri. Trends Microbiol 8:17-23
6. Levine MM, Kotloff, KL, Barry EM, Pasetti MF, Sztein MB (2007) Clinical trials of Shigella vaccines: two steps forward and one step back on a long, hard road. Nat Rev Microbiol 5:540-553

7. Passwell JH, Harlev E, Ashkenazi S, Chu C, Miron D, Ramon R, Farzan N, Shiloach J, Bryla DA, Majadly F, Roberson R, Robbins JB, Schneerson R (2001) Safety and immunogenicity of improved Shigella O-specific polysaccharide-protein conjugate vaccines in adults in Israel. Infect Immun 69:1351-1357

8. Passwell JH, Ashkenazi S, Harlev E, Miron D, Ramon R, Farzam N, Lemer-Geva L, Levi Y, Chu C, Shiloach J, Robbins JB, Schneerson R (2003) Safety and immunogenicity of Shigella sonnei-CRM9 and Shigella flexneri type 2a-rEPAsucc conjugate vaccines in one- to four-year-old children. Pediatr Infect Dis J 22:701-706

9. Belot F, Wright K, Costachel C, Phalipon A, Mulard LA (2004) Blockwise approach to fragments of the O-specific polysaccharide of Shigella flexneri serotype 2a: convergent Synthesis of a decasaccharide representative of a dimer of the branched repeating unit. J Org Chem 69:1060-1074

10. Wright K, Guerreiro C, Laurent I, Baleux F, Mulard LA (2004) Preparation of synthetic glycoconjugates as potential vaccines against Shigella flexneri serotype 2a disease. Org Biomol Chem 2:1518-1527

11. Belot F, Guerreiro C, Baleux F, Mulard LA (2005) Synthesis of two linear PADRE conjugates bearing a deca- or pentadecasaccharide B epitope as potential synthetic vaccines against Shigella flexneri serotype 2a infection. Chemistry 11:1625-1635

12. Phalipon A, Costachel, C, Grandjean C, Thuizat A, Guerreiro C, Tanguy M, Nato F, Vulliez-Le Normand B, Belot F, Wright K, Marcel-Peyre V, Sansonetti PJ, Mulard LA (2006) Characterization of functional oligosaccharide mimics of the Shigella flexneri serotype 2a O-antigen: implications for the development of a chemically defined glycoconjugate vaccine. J Immunol 176:1686-1694

13. Mulard LA, Phalipon A (2008) From epitope characterization to the design of semi-synthetic glycoconjugate vaccines against Shigella flexneri 2a infection, in "Carbohydrate-based vaccines". ACS Symp Ser no 899, in press

14. Frank M, Lutteke T, von der Lieth CW (2007) GlycoMapsDB: a database of the accessible conformational space of glycosidic linkages. Nucleic Acids Res 35:287-290

15. Collaborative Computing Project 4 (1994) The CCP4 Suite: programs for protein crystallography. Acta Crystallogr D Biol Crystallogr 50:760-763

16. Kabat EA (1966) The nature of an antigenic determinant. J Immunol 97:1-11

17. Cisar J, Kabat EA, Dorner MM, Liao J (1975) Binding properties of immunoglobulin combining sites specific for terminal or nonterminal antigenic determinants in dextran. J Exp Med 142:435-459

18. Padlan EA, Kabat EA (1988) Model-building study of the combining sites of two antibodies to alpha (1----6) dextran. Proc Natl Acad Sci USA 85:6885-6889

19. Cygler M, Rose DR, Bundle DR (1991) Recognition of a cell-surface oligosaccharide of pathogenic Salmonella by an antibody Fab fragment. Science 253 :442-445

20. Villeneuve S, Souchon H, Riottot MM, Mazie JC, Lei P, Glaudemans CP, Kovac P, Fournier JM, Alzari PM (2000) Crystal structure of an anti-carbohydrate antibody directed against Vibrio cholerae Ol in complex with antigen: molecular basis for serotype specificity. Proc Natl Acad Sci U S A 97:8433-8438

21. Vyas NK, Vyas MN, Chervenak MC, Johnson MA, Pinto BM, Bundle DR, Quiocho FA (2002) Molecular recognition of oligosaccharide epitopes by a monoclonal Fab specific for Shigella flexneri Y lipopolysaccharide: X-ray structures and thermodynamics. Biochemistry 41:13575-13586

22. Cygler M, Wu S, Zdanov A, Bundle DR, Rose DR. (1993) Recognition of a carbohydrate antigenic determinant of Salmonella by an antibody. Biochem Soc Trans 21:437-441

23. Evans SV, Rose DR, To R, Young NM, Bundle DR (1994) Exploring the mimicry of polysaccharide antigens by anti-idiotypic antibodies. The crystallization, molecular replacement, and refinement to 2.8 A resolution of an idiotope-anti-idiotope Fab complex and of the unliganded anti-idiotope Fab. J Mol Biol 241:691-705

24. Clement MJ, Imberty A, Phalipon A, Perez S, Simenel C, Mulard LA, Delepierre M (2003) Conformational studies of the O-specific polysaccharide of Shigella flexneri 5a and of four related synthetic pentasaccharide fragments using NMR and molecular modeling. J Biol Chem 278:47928-47936

25. Goebel WF (1939) Studies on antibacterial immunity induced by artificial antigens. J Exp Med 69:353-364

26. Alonso de Velasco E, Verheul AF, Veeneman GH, Gomes LJ, van Boom JH, Verhoef J, Snippe H (1993) Protein-conjugated synthetic di- and trisaccharides of pneumococcal type 17F exhibit a different immunogenicity and antigenicity than tetrasaccharide. Vaccine 11:1429-1436

27. Pozsgay V, Chu C, Pannell L, Wolfe J, Robbins JB, Schneerson R (1999) Protein conjugates of synthetic saccharides elicit higher levels of serum IgG lipopolysaccharide antibodies in mice than do those of the O-specific polysaccharide from Shigella dysenteriae type 1. Proc Natl Acad Sci U S A 96:5194-5197

28. Laferriere CA, Sood RK, de Muys JM, Michon F, Jennings HJ (1998) Streptococcus pneumoniae type 14 polysaccharide-conjugate vaccines: length stabilization of opsonophagocytic conformational polysaccharide epitopes. Infect Immun 66:2441-2446

29. Kabsch, W. (1989) Evaluation of single crystal X-ray diffraction data from a position-sensitive detector. J Appl Crystallogr 21:916-924

30. Navaza J (2001) Implementation of molecular replacement in AMoRe. Acta Crystallogr D Biol Crystallogr 57: 1367-1372

31. McCoy AJ (2007) Solving structures of protein complexes by molecular replacement with Phaser. Acta Crystallogr D Biol Crystallogr 63:32-41

32. Pannu NS, Murshudov GN, Dodson EJ, Read RJ (1998) Incorporation of prior phase information strengthens maximum-likelihood structure refinement. Acta Crystallogr D Biol Crystallogr 54:1285-1294

33. Perrakis A, Sixma TK, Wilson KS, Lamzin VS (1997) wARP: improvement and extension of crystallographic phases by weighted averaging of multiple-refined dummy atomic models. Acta Crystallogr D Biol Crystallogr 53: 448-455

34. Kabsch W (1989) Evaluation of single crystal X-ray diffraction data from a position-sensitive detector. J Appl Crystallogr 21:916-924.

35. Jones TA, Zou JY, Cowan SW, Kjeldgaard M (1991) Improved methods for building protein models in electron density maps and the location of errors in these models. Acta Crystallogr A:110-119.

36. Martinez-Jean C, Folch G, Lefranc MP (2001) Nomenclature and overview of the mouse (Mus musculus and Mus sp.) immunoglobulin kappa (IGK) genes. Exp Clin Immunogenet 18:255-279.

37. Giudicelli V, Chaume D, Lefranc MP (2004) IMGT/V-QUEST, an integrated software program for immunoglobulin and T cell receptor V-J and V-D-J rearrangement analysis. Nucleic Acids Res 32:W435-440.

38. Martin AC, Thornton JM (1996) Structural families in loops of homologous proteins: automatic classification, modelling and application to antibodies. J Mol Biol 263:800-815.

39. Wu TT, Johnson G, Kabat EA (1993) Length distribution of CDRH3 in antibodies. Proteins 16:1-7.

40. Kubler-Kielb J, Vinogradov E, Chu C, Schneerson R (2007) O-Acetylation in the O-specific polysaccharide isolated from Shigella flexneri serotype 2a. Carbohydr Res 342:643-647

41. Clément, M.J., Imberty A., Phalipon A., Pérez S., Simenel C., Mulard L.A., Delepierre M. (2003). Conformational studies of the O-specific polysaccharide of Shigella flexneri 5a and of four related synthetic pentasaccharide fragments using NMR and molecular modeling. J. of Biol. Chem. 278, 47928-36.

42. Yamazaki, T., Muhandiram, R., Kay, L.E. (1994). NMR experiments for the measurement of carbon relaxation properties in highly enriched, uniformly 13C, 15N-labeled proteins: applications to 13C alpha carbon. JACS 116, 8266-78.

43. Clore, G.M., Gronenborn, A.M. (1982). Theory and applications of the Transferred Nuclear Overhauser Effect to the study of the conformations of small ligands bound to proteins. J. of Mag. Res. 48, 402-417.

44. Mayer, M., and Meyer, B. (1999). Characterization of ligand binding by saturation transfer difference NMR spectroscopy. Angew. Chem. Int. Ed. 38, 1784-1788

45. Mayer, M., and Meyer, B. (2001). Group epitope mapping by saturation transfer difference to identify segments of a ligand in direct contact with a protein receptor. JACS 123, 6108-17.

46. Baleja J.D., Moult J., Sykes B.D. (1990). Distance measurement and structure refinement with NOE data. J. of Mag. Res. 87, 375-84.

47. Delaglio, F., Grzesiek, S., Vuister, G. W., Zhu, G., Pfeifer, J. & Bax, A. (1995). NMRPipe: a multidimensional spectral processing system based on UNIX pipes. J Biomol NMR 6, 277-93.

48. Johnson, B. a. & Blevins, R. a. (1994). Nmr View - a Computer-Program for the Visualization and Analysis of Nmr Data. Journal of Biomolecular Nmr 4, 603-614.

49. Linge, J. P., O'Donoghue, S. I. & Nilges, M. (2001). Automated assignment of ambiguous nuclear overhauser effects with ARIA. Nuclear Magnetic Resonance of Biological Macromolecules, Pt B 339, 71-90.

50. Brunger, A. T., Adams, P. D., Clore, G. M., DeLano, W. L., Gros, P., Grosse-Kunstleve, R. W., Jiang, J. S., Kuszewski, J., Nilges, M., Pannu, N. S., Read, R. J., Rice, L. M., Simonson, T. & Warren, G. L. (1998). Crystallography & NMR system: A new software suite for macromolecular structure determination. Acta Crystallogr D Biol Crystallogr 54, 905-21.

51. Nilges, M., Macias, M. J., O'Donoghue, S. I. & Oschkinat, H. (1997). Automated NOESY interpretation with ambiguous distance restraints: the refined NMR solution structure of the pleckstrin homology domain from beta-spectrin. J Mol Biol 269, 408-22.

52. Laskowski, R. A., Rullmann, J. A., MacArthur, M. W., Kaptein, R. & Thornton, J. M. (1996). AQUA and PROCHECK-NMR: programs for checking the quality of protein structures solved by NMR. J Biomol NMR 8, 477-86.

53. Vriend, G. (1990). WHAT IF: a molecular modeling and drug design program. J Mol Graph 8, 52-6, 29.

54. Koradi, R., Billeter, M. & Wuthrich, K. (1996). MOLMOL: A program for display and analysis of macromolecular structures. Journal of Molecular Graphics 14, 51-55.

55. Case, D. A., Cheatham, T.E., III, Darden, T., Gohlke H., Luo, R., Merz, K.M., Jr., Onufriev, A., Simmerling C., Wang, B., Woods, R.J. (2005). The AMBER biomolecular simulation programs. J. Comput. Chem. 26, 1668-88.

56. Kirschner, K.N, Woods, R.J. (2001). Solvent interactions determine carbohydrate conformation. Proc. Natl. Acad. Sci. USA 98, 10541-5.

57. Kirschner, K.N, Woods, R.J. (2001). Quantum mechanical study of the nonbonded forces in water-methanol complexes. J. Phys. Chem. A 105, 4150-5.

58. Ryckaert J.P., Cicotti G., Berendsen H.J.C. (1977). Numerical integration of the Cartesian equations of motion of a system with constraints: molecular dynamics of n-alkanes. J. Comput. Phys. 23, 327-41.

59. Miyamoto S., Kollman P.A. (1992). SETTLE: an analytical version of the SHAKE and RATTLE algorithm for rigid water models. J. Comput. Chem. 13, 952-62.

60. Duan, Y., Wu S., Chowdhury S., Lee M.C., Xiong G., Zhang W., Yang R., Cieplak P., Luo R., Lee T. (2003). A point-charge force field for molecular mechanics simulations of proteins based on condensed-phase quantum mechanical calculations. J. Comput. Chem. 24, 1999-2012.

**Claims**

1. A purified polypeptide comprising a mimotope of the O-antigen (O-Ag) from serotype 2a *Shigella flexneri* and recognized by a protective monoclonal antibody (mAb) having a specificity for the lipopolysaccharide (LPS) of the surface antigen of serotype 2a *S. flexneri,* wherein said mimotope is a 12 aminoacid-long peptide selected from the group consisting of:

    - DQAAQHPRDFHK (SEQ ID No. 1);
    - YLEDWIKYNNQK (SEQ ID No. 2);
    - MKSWASYQAHLM (SEQ ID No. 3);
    - INDWISYYSSWK (SEQ ID No. 4);
    - PHGEIFHMMRRP (SEQ ID No. 5);
    - NRRIPCETGCLL (SEQ ID No. 6);
    - TFTRFCGKGCVP (SEQ ID No. 7), and
    a homologous peptide thereof.

2. The polypeptide according to claim 1, wherein said polypeptide comprises a peptide selected from the group consisting of

- AEGEFDQAAQHPRDFHKDPAKC (SEQ ID No. 8);
- AEGEFYLEDWIKYNNQKDPAKC (SEQ ID No. 9);
- AEGEFMKSWASYQAHLMDPAKC (SEQ ID No. 10);
- AEGEFINDWISYYSSWKDPAKC (SEQ ID No. 11);
- AEGEFPHGEIFHMMRRPDPAKC (SEQ ID No. 12);
- AEGEFNRRIPCETGCLLDPAKC (SEQ ID No. 13); and
- AEGEFTFTRFCGKGCVPDPAKC (SEQ ID No. 14).

3. The polypeptide according to claim 1 or 2, wherein said polypeptide comprises a peptide selected from the group consisting of the peptides having the sequences SEQ ID Nos 1 to 5 and SEQ ID Nos 8 to 12.

4. A purified polynucleotide coding for a polypeptide according to anyone of claims 1 to 3.

5. A polypeptide according to anyone of claims 1 to 3 covalently bound to a carrier.

6. A polypeptide according to claim 5, wherein said carrier is an immunocarrier which increases the immunogenicity of the mimotope.

7. An immunogenic composition comprising a polypeptide according to anyone of claims 1 to 3 or a polynucleotide according to claim 4, and, optionally, a pharmaceutical acceptable vehicle.

8. A polypeptide according to anyone of claims 1 to 3, 5 and 6, a polynucleotide according to claim 4 or an immunogenic composition according to claim 7, as a medicament.

9. A polypeptide according to anyone of claims 1 to 3, 5 and 6, a polynucleotide according to claim 4 and an immunogenic composition according to claim 7, for the preparation of pharmaceutical or vaccinal composition intended to prevent or to treat serotype 2a Shigella flexneri infection.

10. A method for the production of isolated or purified antibodies, or a functional fragment thereof, susceptible to recognize the O-Ag from serotype 2a *S. flexneri*, **characterized in that** said method comprises the step of:

a) immunizing a suitable non-human mammal subject with an immunogenic composition comprising a polypeptide according to anyone of claims 1 to 3, 5 and 6, a polynucleotide according to claim 4 or an immunogenic composition according to claim 7, said immunogen composition optionally further including an adjuvant; and
b) selecting the antibodies which are capable of specifically recognizing said O-Ag.

11. A kit for the diagnostic of serotype 2a *S. flexneri* infection, **characterized in that** said kit comprises the following elements:

a) a polypeptide according to anyone of claims 1 to 3; or
b) an antibody, or one of its functional fragments, obtained by the method according to claim 9;
c) optionally, the reagents for the formation of the medium favorable to the immunological reaction; and
d) optionally, the reagents allowing the demonstration of antigen-antibody complexes produced by the immunological reaction.

12. An of in vitro diagnostic method for detecting the presence of serotype 2a *S. flexneri* infection in a patient from a biological sample of said patient, said method comprising the steps of:

a) bringing into contact the biological sample with :

- a polypeptide according to anyone of claims 1 to 3; or
- an antibody, or one of its functional fragments, obtained by the method according to claim 9; and

b) detecting the formation of antigen-antibody complexes

13. A crystal compound obtained from the free Fab F22-4 antibody and having the following structural parameters:

|  |  |
|---|---|
| space group | P1 |
| unit cell | |
| a, b, c (Å) | 47.2, 59.8, 74.4 |
| α, β, γ (°) | 77.5, 84.0, 80.9 |
| Z (molecules/unit cell) | 2 |

**14.** A crystal according to claim 13 having the three dimensional atomic coordinates shown in Table 14

**15.** A complex crystal compound obtained from the Fab F22-4 antibody complexed with the 2a serotype $[AB(E)CD]_2$ decasaccharide and having the following structural parameters:

|  |  |
|---|---|
| space group | P21 |
| unit cell | |
| a, b, c (Å) | 67.1, 137.6, 109.8 |
| α, β, γ (°) | 90, 95.2, 90 |
| Z (molecules/unit cell) | 4. |

**16.** A crystal according to claim 1 having the three dimensional atomic coordinates shown in Table 15.

**17.** A complex crystal compound obtained from the Fab F22-4 antibody complexed with the 2a serotype pentadecasaccharide $[AB(E)CD]_3$ and having the following structural parameters:

|  |  |
|---|---|
| space group | P21 |
| unit cell | |
| a, b, c (Å) | 66.9, 137.2, 109.4 |
| α, β, γ (°) | 90, 95.0, 90 |
| Z (molecules/unit cell) | 4 |

**18.** A crystal according to claim 17 having the three dimensional atomic coordinates shown in Table 16.

**19.** A complex crystal compound obtained from the Fab F22-4 antibody complexed with the mimotope peptide B1 having the sequence SEQ ID No.2 and the three dimensional atomic coordinates shown in Table 13

**20.** A method of identifying a mimotope candidate of the O-Ag from serotype 2a *S. flexneri* and recognized by a protective mAb having a specificity for the LPS of serotype 2a *S. flexneri,* comprising the steps of:

a) providing a model of a binding site of Fab F22-4 antibody, said model including at least the binding site presenting at least one of the 11 hydrogen bond interactions between Fab F22-4 antibody and the O-Ag-antigen fragment defined by Table 2, preferably at least 2, 3, 4, 5, 6, 7, 8, 9, 10 and the 11 hydrogen bond interactions;
b) providing the structure of a candidate agent compound;
c) fitting the candidate agent compound to said binding site, including determining the interactions between the candidate agent compound and the at least one of the 11 hydrogen bond interactions between Fab F22-4 antibody and the O-Ag-antigen fragment defined by Table 2, preferably and the at least 2, 3, 4, 5, 6, 7, 8, 9, 10 and the 11 hydrogen bond interactions; and
d) selecting the fitted candidate agent compound whether this candidate compound satisfies the hydrogen bond interactions required.

**21.** A method of identifying a mimotope candidate of the O-Ag from serotype 2a *S. flexneri* and recognized by a protective mAb having a specificity for the LPS of serotype 2a *S. flexneri,* comprising the steps of :

a) providing a model of a complex crystal compound according to one of claims 15 to 19; and

b) forming a complex between Fab F22-4 antibody and said candidate agent compound; and

c) analysing said formed complex by X-ray crystallography or NMR spectroscopy to determine the ability of said candidate agent compound to interact with the binding site comprising the 11 hydrogen bond interactions shown in table 2; and

d) determinining the ability of said candidate compound to interact with said binding site by comparing the three dimensional atomic coordinates obtained for this candidate agent with the atomic coordinates of the complex crystal model of a).

22. The method according to claim 20, comprising the further step of: e) immunizing a non human mammal with said selected candidate agent and determine whether said candidate compound is able to raise specific antibodies directed against the serotype 2a *S. flexneri* LPS.

23. A computer system, intended to generate structures and/or perform rational drug design for mimotope candidate of the O-Ag from serotype 2a *S. flexneri*, the system containing either (a) at least one atomic coordinates of the crystal compound according to claims 14, 16 and 18, said data defining the three-dimensional structure of said crystal compound, or (b) structural parameters for crystal compound according to claim 13, 15 and 17.

24. Computer readable media with either (a) at least one atomic coordinates of the crystal compound according to claims 14, 16 and 18, said data defining the three-dimensional structure of said crystal compound, or (b) structural parameters for crystal compound according to claim 13, 15 and 17.

25. A computer-based method of rational drug design comprising:

a) providing a three-dimensional structure of Fab F22-4 antibody as defined by the atomic coordinates of Table 14;

b) providing a three-dimensional structure of a candidate mimotope of the O-Ag from serotype 2a *S. flexneri* compound; and

c) fitting the structure of said candidate mimotope to the structure of said Fab F22-4 antibody, particularly the binding site region determined by the following amino acids positions of the Fab: Asn L91, Arg L96, Arg H52, Tyr H58, Pro H95, Trp H33, Asn H31 and His L27D of the Fab F22-4 antibody sequence as depicted and as numbered in the figure 9.

26. A method to identify a mimotope which fully mimic the carbohydrate O-Ag epitopes from serotype 2a *S. flexneri* and is recognized by a protective mAb having a specificity for the LPS of serotype 2a *S. flexneri*, wherein said method implements the use of a synthetic O-Ag fragment from serotype 2a *S. flexneri* comprising at least two RUs.

27. A method according to claim 26, wherein said at least two RUs of serotype 2a polysaccharide are the synthetic [AB (E)CD]$_2$ decasaccharide or the synthetic pentadecasaccharide [AB(E)CD]$_3$ and wherein:

A is an alphaLRhap-(1,2) residue, B is an alphaLRhap-(1,3) residue, C is an alphaLRhap-(1,3) residue, E is an alphaDGlcp-(1,4) residue and D is a betaDGlcNAcp-(1,2) residue.

E

α-D-Glcp(1→4)

↓

[→2)-α-L-Rhap-(1→2)-α-L-Rhap-(1→3)-α-L-Rhap-(1→3)-β-D-GlcNAcp(1→]ₙ

A              B             C             D

**FIGURE 1**

**FIGURE 2A**                **FIGURE 2B**

**FIGURE 3**

**FIGURE 4**

**FIGURE 5A – FIGURE 5B**

**FIGURE 6**

L-Rhap-(1→2)-α-L-Rhap : (AB)

L-Rhap-(1→3)-α-L-Rhap : (BC)

L-Rhap-(1→3)-β-D-GlcNAcp : (CD)

D-GlcNAcp-(1→2)-α-L-Rhap : (DA')

D-Glcp-(1→4)-α-L-Rhap : (EC)

**FIGURE 7**

**FIGURE 8**

## V$_H$ domains of F22-4 and SYA/J6

```
                                                                                             1              1
           1         2         3         4         5         6         7         8         9  0              1
       12345678901234567890123456789012345678901234567890123abc3456789012345678901234567890123abc345678901234567890a1234567890123
F224   EVKVEESGGGLVQPGGSMKISCVVSGLTFSNYWMSWVRQSPEKGLEWVAEIRLKSDNYATYYAESVKGKFTISRDDSKSRLYLQMNNLRTEDTGIYYCFLPM        DYWGQGTSVTVSS
SYA/J6 EVKVEESGGGLVQPGGSMKLSCVASGFTFSNYWMEWVRQSPEKGLEWVAEIRLKSNNYATHYAESVKGRFTISRDDSKSSVYLQMNNLRAEDTGIYYCTRGGAVGAMDYWGQGTSVTVSS
*      ++++++++++++++++++++.+++.++.++++++++.++++++++++++++++++++.++++.++++++++.++++++++++..++++++++.+++++++++++..        +++++++++++++
```

## V$_L$ domains of F22-4 and SYA/J6

```
                                                                                       1
           1         2         3         4         5         6         7         8      9  0
       123456789012345678901234567abcde89012345678901234567890123456789012345678901234567890123456789012345678901234567
F224   DIVMTQAAFSNPVTLGTSASISCRSSKSLLHSDGITYLYWYLQKPGQSPHLLIYHLSNLASGVPDRFSSSGSGTDFTLRISRVEAEDVGIYYCAHNVELPRTFGGGTKLEIK
SYA/J6 DVVLTQTPLSLPVRLGDQASISCRSSQSLLHSDGNTYLHWYLQKPGQSPKLLIYKVSNRFSGVPDRFSGSGSGTDFTLKISRVEAEDLGVYFCSQTTHVP TFGGGTKLEIK
*      +.+.++...+.++.++..++++++++.++++++.+++.++++++++++.++++..++..++++++++.++++++++++.+++++++++.+.+.+......+ +++++++++++
```

**FIGURE 9A**

## V$_H$DOMAIN

```
                                                                                             1              1
           1         2         3         4         5         6         7         8         9  0              1
       12345678901234567890123456789012345678901234567890123abc3456789012345678901234567890123abc345678901234567890a1234567890123
F224   EVKVEESGGGLVQPGGSMKISCVVSGLTFSNYWMSWVRQSPEKGLEWVAEIRLKSDNYATYYAESVKGKFTISRDDSKSRLYLQMNNLRTEDTGIYYCFLPM        DYWGQGTSVTVSS
HV6-6  EVKLEESGGGLVQPGGSMKLSCVASGFTFSNYWMNWVRQSPEKGLEWVAEIRLKSNNYATHYAESVKGRFTISRDDSKSSVYLQMNNLRAEDTGIYYCTR
JH4                                                                                                         YYAM        DYWGQGTSVTVSS
```

## V$_L$DOMAIN

```
                                                                                       1
           1         2         3         4         5         6         7         8      9  0
       123456789012345678901234567890123456abcde89012345678901234567890123456789012345678901234567890123456789012345678901234567
F224    DIVMTQAAFSNPVTLGTSASISCRSSKSLLHSDGITYLYWYLQKPGQSPHLLIYHLSNLASGVPDRFSSSGSGTDFTLRISRVEAEDVGIYYCAHNVELPRTFGGGTKLEIK
KV2-109 DIVMTQAAFSNPVTLGTSASISCRSSKSLLHSNGITYLYWYLQKPGQSPQLLIYQMSNLASGVPDRFSSSGSGTDFTLRISRVEAEDVGVYYCAQNLELP
JΩ1 OR JΩ2                                                                                                              WTFGGGTKLEIK
```

**FIGURE 9B**

**FIGURE 10**

**FIGURE 11A**

**FIGURE 11B**

Buried surface ($\text{Å}^2$)

FIGURE 12

**FIGURE 13**

**AB(E)CDAB(E)CDAB(E)CD**

FIGURE 14A

$S^2 \approx$

FIGURE 14B

**FIGURE 15**

**FIGURE 16**

**FIGURE 17A**

FIGURE 17B

FIGURE 18

**FIGURE 19A**

**FIGURE 19B**

**FIGURE 19C**

**FIGURE 20A**

**FIGURE 20B**

**FIGURE 20C**

$S^2 \approx 1$
$S^2 \approx 0,95$
$S^2 \approx 0.90$
$S^2 \approx 0,85$
$S^2 \approx 0,80$
$S^2 \approx 0,75$

**FIGURE 20D**

FIGURE 21A

FIGURE 21B

FIGURE 21C

**FIGURE 21D**

**FIGURE 21E**

Φ (degrees)

**FIGURE 21F**

Φ (degrees)

**FIGURE 22**

**FIGURE 23A**

**FIGURE 23B**

**FIGURE 23C**

**FIGURE 23D**

**FIGURE 23E**

**FIGURES 24A**

**FIGURE 24B**

**FIGURE 25**

NAG D (J5)

Tyr 1

FIGURE 26

rhamnose C (J3)

Trp 5

**FIGURE 27**

glucose E (J4)

Tyr 8

**FIGURE 28**

**FIGURE 29**

**FIGURE 30**

**EUROPEAN SEARCH REPORT**

Europäisches
Patentamt
European
Patent Office
Office européen
des brevets

Application Number

EP 08 29 0476

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | PHALIPON ARMELLE ET AL: "Characterization of functional oligosaccharide mimics of the Shigella flexneri serotype 2a O-antigen: Implications for the development of a chemically defined glycoconjugate vaccine" JOURNAL OF IMMUNOLOGY, vol. 176, no. 3, February 2006 (2006-02), pages 1686-1694, XP002515563 ISSN: 0022-1767 * page 1687, left-hand column * * pages 168-9, paragraph BRIDGING * * page 1691; table 1 * | 1-27 | INV. C07K11/00 C07K14/00 A61K39/112 A61P31/04 |
| Y,D | WO 2005/003775 A (PASTEUR INSTITUT [FR]; INSERM INST DE LA SANTE ET DE [FR]; CENTRE NAT) 13 January 2005 (2005-01-13) * page 123, lines 21,22 * * pages 128,130 * * pages 136-143; figure 34 * | 1-27 | |
| Y,D | PHALIPON ET AL: "Induction of anti-carbohydrate antibodies by phage library-selected peptide mimics" EUROPEAN JOURNAL OF IMMUNOLOGY, WILEY - V C H VERLAG GMBH & CO. KGAA, DE, vol. 27, no. 10, 1 October 1997 (1997-10-01), pages 2620-2625, XP002096597 ISSN: 0014-2980 * pages 2623-4 * | 1-27 | TECHNICAL FIELDS SEARCHED (IPC) C07K A61K A61P |

-/--

The present search report has been drawn up for all claims

| Place of search | Date of completion of the search | Examiner |
|---|---|---|
| Munich | 19 February 2009 | Domingues, Helena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 08 29 0476

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CLÉMENT MARIE-JEANNE ET AL: "Toward a better understanding of the basis of the molecular mimicry of polysaccharide antigens by peptides: the example of Shigella flexneri 5a." THE JOURNAL OF BIOLOGICAL CHEMISTRY 27 JAN 2006, vol. 281, no. 4, 27 January 2006 (2006-01-27), pages 2317-2332, XP002515564 ISSN: 0021-9258 * the whole document * | 1-27 | |
| Y | HOU YINGCHUN ET AL: "Development of peptide mimotopes of lipooligosaccharide from nontypeable Haemophilus influenzae as vaccine candidates." JOURNAL OF IMMUNOLOGY, vol. 170, no. 8, 15 April 2003 (2003-04-15), pages 4373-4379, XP002515565 ISSN: 0022-1767 * page 4374, last paragraph * * page 4378 * | 1-27 | TECHNICAL FIELDS SEARCHED (IPC) |
| Y | BUCHWALD ULRIKE K ET AL: "A peptide mimotope of type 8 pneumococcal capsular polysaccharide induces a protective immune response in mice" INFECTION AND IMMUNITY, vol. 73, no. 1, January 2005 (2005-01), pages 325-333, XP002515566 ISSN: 0019-9567 * the whole document * | 1-27 | |

-/--

The present search report has been drawn up for all claims

| Place of search | Date of completion of the search | Examiner |
|---|---|---|
| Munich | 19 February 2009 | Domingues, Helena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 08 29 0476

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | PHALIPON ARMELLE ET AL: "A synthetic carbohydrate-protein conjugate vaccine candidate against Shigella flexneri 2a infection." JOURNAL OF IMMUNOLOGY (BALTIMORE, MD. : 1950) 15 FEB 2009, vol. 182, no. 4, 15 February 2009 (2009-02-15), pages 2241-2247, XP002515567 ISSN: 1550-6606 * the whole document * ----- | 1-27 | |
| T | NORMAND B VULLIEZ-LE ET AL: "Structures of synthetic O-antigen fragments from serotype 2a Shigella flexneri in complex with a protective monoclonal antibody" PROCEEDINGS OF THE NATIONAL ACADEMY OF SCIENCES OF THE UNITED STATES OF AMERICA, vol. 105, no. 29, July 2008 (2008-07), pages 9976-9981, XP002515568 ISSN: 0027-8424 * the whole document * ----- | 1-27 | |

TECHNICAL FIELDS
SEARCHED (IPC)

The present search report has been drawn up for all claims

| Place of search | Date of completion of the search | Examiner |
|---|---|---|
| Munich | 19 February 2009 | Domingues, Helena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    ...........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 29 0476

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2009

| Patent document cited in search report | Publication date | Patent family member(s) | | Publication date |
|---|---|---|---|---|
| WO 2005003775 A | 13-01-2005 | CA | 2434668 A1 | 04-01-2005 |
| | | CA | 2470262 A1 | 04-01-2005 |
| | | EP | 1642132 A2 | 05-04-2006 |
| | | US | 2008112951 A1 | 15-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6528061 B1
- WO 2005003775 A

### Non-patent literature cited in the description

- **Bowie et al.** *Sciences,* 1990, vol. 47, 1305-1310
- **Tam, JP.** *PNAS, USA,* 1988, vol. 85, 5409-5413
- *Infect. Immun.,* 2005, vol. 73, 5835
- *Infect. Immun.,* 2001, vol. 31, 3816
- **Van Houten, N.E. et al.** *Vaccine,* 08 May 2006, vol. 24 (19), 4188-4200
- **Beenhouwer, D.O. ; May, R. J. ; Valadon, P. ; Scharff, M. D.** *J. Immunol,* 2002, vol. 169, 6992-6999
- **Dharmasena, M. N. ; Jewell, D. A. ; Taylor R. K.** *J. Biol. Chem,* 2007, vol. 282, 33805-16
- **Niyogi SK.** Shigellosis. *J Microbiol,* 2005, vol. 43, 133-143
- **Kotloff KL ; Winickoff JP ; Ivanoff B ; Clemens JD ; Swerdlow DL ; Sansonetti PJ ; Adak GK ; Levine MM.** Global burden of Shigella infections: implications for vaccine development and implementation of control strategies. *Bull World Health Organ,* 1999, vol. 77, 651-666
- **Jennison AV ; Verma NK.** Shigella flexneri infection: pathogenesis and vaccine development. *FEMS Microbiol Rev,* 2004, vol. 28, 43-58
- **Lindberg AA. ; Karnell A ; Weintraub A.** The lipopolysaccharide of Shigella bacteria as a virulence factor. *Rev Infect Dis,* 1991, vol. 13, 279-284
- **Allison GE ; Verma NK.** Serotype-converting bacteriophages and O-antigen modification in Shigella flexneri. *Trends Microbiol,* 2000, vol. 8, 17-23
- **Levine MM ; Kotloff, KL ; Barry EM ; Pasetti MF ; Sztein MB.** Clinical trials of Shigella vaccines: two steps forward and one step back on a long, hard road. *Nat Rev Microbiol,* 2007, vol. 5, 540-553
- **Passwell JH ; Harlev E ; Ashkenazi S ; Chu C ; Miron D ; Ramon R ; Farzan N ; Shiloach J ; Bryla DA ; Majadly F.** Safety and immunogenicity of improved Shigella O-specific polysaccharide-protein conjugate vaccines in adults in Israel. *Infect Immun,* 2001, vol. 69, 1351-1357

- **Passwell JH ; Ashkenazi S ; Harlev E ; Miron D ; Ramon R ; Farzam N ; Lemer-Geva L ; Levi Y ; Chu C ; Shiloach J.** Safety and immunogenicity of Shigella sonnei-CRM9 and Shigella flexneri type 2a-rEPAsucc conjugate vaccines in one- to four-year-old children. *Pediatr Infect Dis J,* 2003, vol. 22, 701-706
- **Belot F ; Wright K ; Costachel C ; Phalipon A ; Mulard LA.** Blockwise approach to fragments of the O-specific polysaccharide of Shigella flexneri serotype 2a: convergent Synthesis of a decasaccharide representative of a dimer of the branched repeating unit. *J Org Chem,* 2004, vol. 69, 1060-1074
- **Wright K ; Guerreiro C ; Laurent I ; Baleux F ; Mulard LA.** Preparation of synthetic glycoconjugates as potential vaccines against Shigella flexneri serotype 2a disease. *Org Biomol Chem,* 2004, vol. 2, 1518-1527
- **Belot F ; Guerreiro C ; Baleux F ; Mulard LA.** Synthesis of two linear PADRE conjugates bearing a deca- or pentadecasaccharide B epitope as potential synthetic vaccines against Shigella flexneri serotype 2a infection. *Chemistry,* 2005, vol. 11, 1625-1635
- **Phalipon A ; Costachel, C ; Grandjean C ; Thuizat A ; Guerreiro C ; Tanguy M ; Nato F ; Vulliez-Le Normand B ; Belot F ; Wright K.** Characterization of functional oligosaccharide mimics of the Shigella flexneri serotype 2a O-antigen: implications for the development of a chemically defined glycoconjugate vaccine. *J Immunol,* 2006, vol. 176, 1686-1694
- From epitope characterization to the design of semi-synthetic glycoconjugate vaccines against Shigella flexneri 2a infection. **Mulard LA ; Phalipon A.** Carbohydrate-based vaccines. ACS Symp Ser no 899, 2008
- **Frank M ; Lutteke T ; von der Lieth CW.** GlycoMapsDB: a database of the accessible conformational space of glycosidic linkages. *Nucleic Acids Res,* 2007, vol. 35, 287-290

- Collaborative Computing Project 4 (1994) The CCP4 Suite: programs for protein crystallography. *Acta Crystallogr D Biol Crystallogr,* 1994, vol. 50, 760-763
- **Kabat EA.** The nature of an antigenic determinant. *J Immunol,* 1996, vol. 97, 1-11
- **Cisar J ; Kabat EA ; Dorner MM ; Liao J.** Binding properties of immunoglobulin combining sites specific for terminal or nonterminal antigenic determinants in dextran. *J Exp Med,* 1975, vol. 142, 435-459
- **Padlan EA ; Kabat EA.** Model-building study of the combining sites of two antibodies to alpha (1----6)dextran. *Proc Natl Acad Sci USA,* 1988, vol. 85, 6885-6889
- **Cygler M ; Rose DR ; Bundle DR.** Recognition of a cell-surface oligosaccharide of pathogenic Salmonella by an antibody Fab fragment. *Science,* 1991, vol. 253, 442-445
- **Villeneuve S ; Souchon H ; Riottot MM ; Mazie JC ; Lei P ; Glaudemans CP ; Kovac P ; Fournier JM ; Alzari PM.** Crystal structure of an anti-carbohydrate antibody directed against Vibrio cholerae OI in complex with antigen: molecular basis for serotype specificity. *Proc Natl Acad Sci U S A,* 2000, vol. 97, 8433-8438
- **Vyas NK ; Vyas MN ; Chervenak MC ; Johnson MA ; Pinto BM ; Bundle DR ; Quiocho FA.** Molecular recognition of oligosaccharide epitopes by a monoclonal Fab specific for Shigella flexneri Y lipopolysaccharide: X-ray structures and thermodynamics. *Biochemistry,* 2002, vol. 41, 13575-13586
- **Cygler M ; Wu S ; Zdanov A ; Bundle DR ; Rose DR.** Recognition of a carbohydrate antigenic determinant of Salmonella by an antibody. *Biochem Soc Trans,* 1993, vol. 21, 437-441
- **Evans SV ; Rose DR ; To R ; Young NM ; Bundle DR.** Exploring the mimicry of polysaccharide antigens by anti-idiotypic antibodies. The crystallization, molecular replacement, and refinement to 2.8 A resolution of an idiotope-anti-idiotope Fab complex and of the unliganded anti-idiotope Fab. *J Mol Biol,* 1994, vol. 241, 691-705
- **Clement MJ ; Imberty A ; Phalipon A ; Perez S ; Simenel C ; Mulard LA ; Delepierre M.** Conformational studies of the O-specific polysaccharide of Shigella flexneri 5a and of four related synthetic pentasaccharide fragments using NMR and molecular modeling. *J Biol Chem,* 2003, vol. 278, 47928-47936
- **Goebel WF.** Studies on antibacterial immunity induced by artificial antigens. *J Exp Med,* 1939, vol. 69, 353-364
- **Alonso de Velasco E ; Verheul AF ; Veeneman GH ; Gomes LJ ; van Boom JH ; Verhoef J ; Snippe H.** Protein-conjugated synthetic di- and trisaccharides of pneumococcal type 17F exhibit a different immunogenicity and antigenicity than tetrasaccharide. *Vaccine,* 1933, vol. 11, 1429-1436
- **Pozsgay V ; Chu C ; Pannell L ; Wolfe J ; Robbins JB ; Schneerson R.** Protein conjugates of synthetic saccharides elicit higher levels of serum IgG lipopolysaccharide antibodies in mice than do those of the O-specific polysaccharide from Shigella dysenteriae type 1. *Proc Natl Acad Sci U S A,* 1999, vol. 96, 5194-5197
- **Laferriere CA ; Sood RK ; de Muys JM ; Michon F ; Jennings HJ.** Streptococcus pneumoniae type 14 polysaccharide-conjugate vaccines: length stabilization of opsonophagocytic conformational polysaccharide epitopes. *Infect Immun,* 1998, vol. 66, 2441-2446
- **Kabsch, W.** Evaluation of single crystal X-ray diffraction data from a position-sensitive detector. *J Appl Crystallogr,* 1989, vol. 21, 916-924
- **Navaza J.** Implementation of molecular replacement in AMoRe. *Acta Crystallogr D Biol Crystallogr,* 2001, vol. 57, 1367-1372
- **McCoy AJ.** Solving structures of protein complexes by molecular replacement with Phaser. *Acta Crystallogr D Biol Crystallogr,* 2007, vol. 63, 32-41
- **Pannu NS ; Murshudov GN ; Dodson EJ ; Read RJ.** Incorporation of prior phase information strengthens maximum-likelihood structure refinement. *Acta Crystallogr D Biol Crystallogr,* 1998, vol. 54, 1285-1294
- **Perrakis A ; Sixma TK ; Wilson KS ; Lamzin VS.** wARP: improvement and extension of crystallographic phases by weighted averaging of multiple-refined dummy atomic models. *Acta Crystallogr D Biol Crystallogr,* 1997, vol. 53, 448-455
- **Kabsch W.** Evaluation of single crystal X-ray diffraction data from a position-sensitive detector. *J Appl Crystallogr,* 1989, vol. 21, 916-924
- **Jones TA ; Zou JY ; Cowan SW ; Kjeldgaard M.** Improved methods for building protein models in electron density maps and the location of errors in these models. *Acta Crystallogr A,* 1991, 110-119
- **Martinez-Jean C ; Folch G, Lefranc MP.** Nomenclature and overview of the mouse (Mus musculus and Mus sp.) immunoglobulin kappa (IGK) genes. *Exp Clin Immunogenet,* 2001, vol. 18, 255-279
- **Giudicelli V ; Chaume D ; Lefranc MP.** IMGT/V-QUEST, an integrated software program for immunoglobulin and T cell receptor V-J and V-D-J rearrangement analysis. *Nucleic Acids Res,* 2004, vol. 32, W435-440

- **Martin AC ; Thornton JM.** Structural families in loops of homologous proteins: automatic classification, modelling and application to antibodies. *J Mol Biol,* 1996, vol. 263, 800-815
- **Wu TT ; Johnson G ; Kabat EA.** Length distribution of CDRH3 in antibodies. *Proteins,* 1993, vol. 16, 1-7
- **Kubler-Kielb J ; Vinogradov E ; Chu C ; Schneerson R.** O-Acetylation in the O-specific polysaccharide isolated from Shigella flexneri serotype 2a. *Carbohydr Res,* 2007, vol. 342, 643-647
- **Clément, M.J. ; Imberty A. ; Phalipon A. ; Pérez S. ; Simenel C. ; Mulard L.A. ; Delepierre M.** Conformational studies of the O-specific polysaccharide of Shigella flexneri 5a and of four related synthetic pentasaccharide fragments using NMR and molecular modeling. *J. of Biol. Chem.,* 2003, vol. 278, 47928-36
- **Yamazaki, T. ; Muhandiram, R. ; Kay, L.E.** NMR experiments for the measurement of carbon relaxation properties in highly enriched, uniformly 13C, 15N-labeled proteins: applications to 13C alpha carbon. *JACS,* 1994, vol. 116, 8266-78
- **Clore, G.M. ; Gronenborn, A.M.** Theory and applications of the Transferred Nuclear Overhauser Effect to the study of the conformations of small ligands bound to proteins. *J. of Mag. Res.,* 1982, vol. 48, 402-417
- **Mayer, M. ; Meyer, B.** Characterization of ligand binding by saturation transfer difference NMR spectroscopy. *Angew. Chem. Int. Ed.,* 1999, vol. 38, 1784-1788
- **Mayer, M. ; Meyer, B.** Group epitope mapping by saturation transfer difference to identify segments of a ligand in direct contact with a protein receptor. *JACS,* 2001, vol. 123, 6108-17
- **Baleja J.D. ; Moult J. ; Sykes B.D.** Distance measurement and structure refinement with NOE data. *J. of Mag. Res.,* 1990, vol. 87, 375-84
- **Delaglio, F. ; Grzesiek, S. ; Vuister, G. W. ; Zhu, G. ; Pfeifer, J. ; Bax, A.** NMRPipe: a multidimensional spectral processing system based on UNIX pipes. *J Biomol NMR,* 1995, vol. 6, 277-93
- **Johnson, B. a. ; Blevins, R. a.** Nmr View - a Computer-Program for the Visualization and Analysis of Nmr Data. *Journal of Biomolecular,* 1994, 603-614
- **Linge, J. P. ; O'Donoghue, S. I. ; Nilges, M.** Automated assignment of ambiguous nuclear overhauser effects with ARIA. *Nuclear Magnetic Resonance of Biological Macromolecules,* 2001, vol. 339, 71-90
- **Brunger, A. T. ; Adams, P. D. ; Clore, G. M. ; De-Lano, W. L. ; Gros, P. ; Grosse-Kunstleve, R. W. ; Jiang, J. S. ; Kuszewski, J. ; Nilges, M. ; Pannu, N. S.** Crystallography & NMR system: A new software suite for macromolecular structure determination. *Acta Crystallogr D Biol Crystallogr,* 1998, vol. 54, 905-21
- **Nilges, M. ; Macias, M. J. ; O'Donoghue, S. I. ; Oschkinat, H.** Automated NOESY interpretation with ambiguous distance restraints: the refined NMR solution structure of the pleckstrin homology domain from beta-spectrin. *J Mol Biol,* 1997, vol. 269, 408-22
- **Laskowski, R. A. ; Rullmannn, J. A. ; MacArthur, M. W. ; Kaptein, R. ; Thornton, J. M.** AQUA and PROCHECK-NMR: programs for checking the quality of protein structures solved by NMR. *J Biomol NMR,* 1996, vol. 8, 477-86
- **Vriend, G.** WHAT IF: a molecular modeling and drug design program. *J Mol Graph,* 1990, vol. 8 (29), 52-6
- **Koradi, R. ; Billeter, M. ; Wuthrich, K.** MOLMOL: A program for display and analysis of macromolecular structures. *Journal of Molecular Graphics,* 1996, vol. 14, 51-55
- **Case, D. A. ; Cheatham, T.E. ; III, Darden, T. ; Gohlke H. ; Luo, R. ; Merz, K.M. ; Jr. ; Onufriev, A. ; Simmerling C. ; Wang, B.** The AMBER biomolecular simulation programs. *J. Comput. Chem.,* 2005, vol. 26, 1668-88
- **Kirschner, K.N ; Woods, R.J.** Solvent interactions determine carbohydrate conformation. *Proc. Natl. Acad. Sci. USA,* 2001, vol. 98, 10541-5
- **Kirschner, K.N ; Woods, R.J.** Quantum mechanical study of the nonbonded forces in water-methanol complexes. *J. Phys. Chem. A,* 2001, vol. 105, 4150-5
- **Ryckaert J.P. ; Cicotti G. ; Berendsen H.J.C.** Numerical integration of the Cartesian equations of motion of a system with constraints: molecular dynamics of n-alkanes. *J. Comput. Phys.,* 1977, vol. 23, 327-41
- **Miyamoto S. ; Kollman P.A.** SETTLE: an analytical version of the SHAKE and RATTLE algorithm for rigid water models. *J. Comput. Chem.,* 1992, vol. 13, 952-62
- **Duan, Y. ; Wu S. ; Chowdhury S. ; Lee M.C. ; Xiong G. ; Zhang W. ; Yang R. ; Cieplak P. ; Luo R. ; Lee T.** A point-charge force field for molecular mechanics simulations of proteins based on condensed-phase quantum mechanical calculations. *J. Comput. Chem.,* 2003, vol. 24, 1999-2012